(19) **Europäisches Patentamt / European Patent Office / Office européen des brevets**

(11)   **EP 2 635 709 B1**

(12) # EUROPEAN PATENT SPECIFICATION

(45) Date of publication and mention of the grant of the patent:
**01.05.2019  Bulletin 2019/18**

(21) Application number: **11838747.1**

(22) Date of filing: **02.11.2011**

(51) Int Cl.:
*C12Q 1/68* (2018.01)      *A61K 31/505* (2006.01)
*G06F 15/16* (2006.01)      *A61K 31/40* (2006.01)
*A61K 31/22* (2006.01)      *A61K 31/366* (2006.01)
*A61K 31/4439* (2006.01)     *A61K 31/5377* (2006.01)
*A61K 31/4545* (2006.01)     *A61P 7/02* (2006.01)

(86) International application number:
**PCT/US2011/058963**

(87) International publication number:
**WO 2012/061502 (10.05.2012 Gazette 2012/19)**

(54) **GENETIC POLYMORPHISMS ASSOCIATED WITH VENOUS THROMBOSIS AND STATIN RESPONSE, METHODS OF DETECTION AND USES THEREOF**

MIT VENENTHROMBOSE UND DEM ANSPRECHEN AUF STATIN ASSOZIIERTE GENETISCHE POLYMORPHISMEN, VERFAHREN ZU IHREM NACHWEIS UND VERWENDUNGEN DAVON

POLYMORPHISMES GÉNÉTIQUES ASSOCIÉS À LA THROMBOSE VEINEUSE ET LA RÉPONSE À LA STATINE, PROCÉDÉS DE DÉTECTION ET UTILISATIONS DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **02.11.2010  US 409434 P**

(43) Date of publication of application:
**11.09.2013  Bulletin 2013/37**

(60) Divisional application:
**18207051.6**

(73) Proprietors:
• **Celera Corporation**
  **San Clemente, CA 92673 (US)**
• **Leiden University Medical Center (LUMC) Acting on**
  **Behalf Of Academic Hospital Leiden (AZL)**
  **2333 ZA Leiden (NL)**

(72) Inventors:
• **BARE, Lance**
  **San Clemente, CA 92673 (US)**
• **DEVLIN, James, J.**
  **San Clemente, CA 92673 (US)**
• **ROSENDAAL, Frits R.**
  **San Clemente, CA 92673 (US)**
• **REITSMA, Pieter H.**
  **San Clemente, CA 92673 (US)**
• **BEZEMER, Irene D.**
  **San Clemente, CA 92673 (US)**

(74) Representative: **Weber, Martin**
  **Jones Day**
  **Prinzregentenstraße 11**
  **80538 München (DE)**

(56) References cited:
  WO-A2-2009/105680      US-A1- 2003 100 493
  US-A1- 2009 054 256      US-A1- 2009 258 003
  US-A1- 2009 270 332      US-A1- 2012 039 864

• **DELLUC AURÉLIEN ET AL: "Association of common genetic variations and idiopathic venous thromboembolism. Results from EDITh, a hospital-based case-control study", THROMBOSIS AND HAEMOSTASIS, SCHATTAUER GMBH, DE, vol. 103, no. 6, 1 June 2010 (2010-06-01), pages 1161-1169, XP009155048, ISSN: 0340-6245**
• **Li Yonghong: "Fine Mapping and Missing Heritability Follow Up for Genetic Association Studies", , 21 May 2010 (2010-05-21), XP55131587, Retrieved from the Internet: URL:https://www.celera.com/pdf/Li_UCSF_May 21-5-13-10.pdf [retrieved on 2014-07-24]**

- Haifeng M Wu ET AL: "Personalized Healthcare in Clotting Disorders", Personalized Medicine. 2010;7(1):65-73., 21 December 2009 (2009-12-21), XP55131578, Retrieved from the Internet: URL:http://www.medscape.com/viewarticle/717984_print [retrieved on 2014-07-24]
- BEZEMER IRENE D ET AL: "Gene variants associated with deep vein thrombosis", JAMA THE JOURNAL OF THE AMERICAN MEDICAL ASSOCIATION, AMERICAN MEDICAL ASSOCIATION, US, vol. 299, no. 11, 19 March 2008 (2008-03-19), pages 1306-1314, XP002665780, ISSN: 0098-7484
- E. S Nakou ET AL: "High-Sensitivity C-Reactive Protein: To Measure or not to Measure?~!2009-10-29~!2009-12-22~!2010-04-08", The Open Clinical Chemistry Journal, 15 April 2010 (2010-04-15), pages 10-18, XP055132174, DOI: 10.2174/1874241601003020010 Retrieved from the Internet: URL:http://benthamopen.com/tocchemj/articles/V003/SI0008TOCCHEMJ/10TOCCHEMJ.pdf
- "Genetic determinants of warfarin dosing in the Han-Chinese population", PHARMACOGENOMICS 2009 FUTURE MEDICINE LTD. GBR, vol. 10, no. 12, 2009, pages 1905-1913, XP009179417, ISSN: 1462-2416
- VAUGHAN ET AL.: 'Update on Statins: 2003' CIRCULATION vol. 110, no. 7, 2004, pages 886 - 892, XP055111894
- LI ET AL.: 'Genetic variants associated with deep vein thrombosis: the F11 locus' J THROMB HAEMOST. vol. 7, no. 11, 2009, pages 1802 - 1808, XP002665781

Remarks:

The complete document including Reference Tables and the Sequence Listing can be downloaded from the EPO website

**Description**

FIELD OF THE INVENTION

The present invention is in the field of disease risk and drug response, particularly genetic polymorphisms that are associated with risk for developing venous thrombosis (VT) and response to statins, especially statin treatment for the prevention or treatment of VT and related pathologies. In particular, the present invention relates to a specific single nucleotide polymorphisms (SNPs) in the human genome (rs2036914), and its association with risk for developing VT and variability in responsiveness to an HMG-CoA reductase inhibitor, (including preventive treatment) in reducing VT risk between different individuals. The SNPs disclosed herein can be used, for example, as targets for diagnostic reagents and for the development of therapeutic agents. In particular, the SNPs disclosed herein are useful for such uses as predicting an individual's response to therapeutic agents such as evaluating the likelihood of an individual differentially responding positively to statins, particularly for the treatment or prevention of VT (including recurrent VT), identifying an individual who has an increased or decreased risk of developing VT (including recurrent VT), for early detection of VT, for providing clinically important information for the prevention and/or treatment of VT, for predicting recurrence of VT, and for screening and selecting therapeutic agents. Methods, assays, kits, and reagents for detecting the presence of these polymorphisms and their encoded products are described.

BACKGROUND OF THE INVENTION

The present invention relates to a SNPs that is associated with risk for developing venous thrombosis (VT) and variability between individuals in their response to an HMG-CoA reductase inhibitor, particularly for reducing the risk of VT.
VT, which may also be referred to as venous thromboembolism (VTE), includes deep vein thrombosis (DVT) and pulmonary embolism (PE). VT can further include a first occurrence of VT (i.e., primary VT) or recurrent VT.

Venous Thrombosis (VT)

The development of a blood clot is known as thrombosis. Venous thrombosis (VT) is the formation of a blood clot in the veins. VT may also be referred to as venous thromboembolism (VTE). Over 200,000 new cases of VT occur annually. Of these, 30 percent of patients die within three days; one in five suffer sudden death due to pulmonary embolism (PE) (Seminars in Thrombosis and Hemostasis, 2002, Vol. 28, Suppl. 2) (Stein et al., Chest 2002; 122(3):960-962, further describes PE). Caucasians and African-Americans have a significantly higher incidence than Hispanics, Asians or Pacific Islanders (White, Circulation 107(23 Suppl 1):I4-8 Review, 2003).
Several conditions can lead to an increased tendency to develop blood clots in the veins or arteries (National Hemophilia Foundation, HemAware newsletter, Vol. 6 (5), 2001), and such conditions may be inherited (genetic) or acquired. Examples of acquired conditions are surgery and trauma, prolonged immobilization, cancer, myeloproliferative disorders, age, hormone therapy, and even pregnancy, all of which may result in thrombosis (Seligsohn et al., New Eng J Med 344(16):1222-1231, 2001 and Heit et al., Thromb Haemost 2001; 86(1):452-463). Family and twin studies indicate that inherited (genetic) causes account for about 60% of the risk for deep vein thrombosis (DVT) (Souto et al., Am J Hum Genet 2000; 67(6):1452-1459; Larsen et al., Epidemiology 2003; 14(3):328-332). Inherited causes include polymorphisms in any of several different clotting, anticoagulant, or thrombolytic factors, such as the factor V gene (the factor V Leiden (FVL) variant), prothrombin gene (factor II), and methylenetetrahydrofolate reductase gene (MTHFR). Other likely inherited causes are an increase in the expression levels of the factors VIII, IX or XI, or fibrinogen genes (Seligsohn et al., New Eng J Med 344(16):1222-1231, 2001). Deficiencies of natural anticoagulants antithrombin, protein C and protein S are strong risk factors for DVT; however, the variants causing these deficiencies are rare, and explain only about 1% of all DVTs (Rosendaal et al., Lancet 1999; 353(9159):1167-1173). The factor V Leiden (FVL) and prothrombin G20210A genetic variants have been consistently found to be associated with DVT (Bertina et al., Nature 1994; 369(6475):64-67 and Poort et al., Blood 1996; 88(10):3698-3703) but still only explain a fraction of the DVT events (Rosendaal, Lancet 1999; 353(9159):1167-1173; Bertina et al., Nature 1994; 369(6475):64-67; Poort et al., Blood 1996; 88(10):3698-3703). Elevated plasma concentrations of coagulation factors (e.g., VIII, IX, X, and XI) have also been shown to be important risk factors for DVT (Kyrle et al., N Engl J Med. 2000;343:457-462; van Hylckama Vlieg et al., Blood. 2000;95:3678-3682; de Visser et al., Thromb Haemost. 2001;85:1011-1017; and Meijers et al., N Engl J Med. 2000;342:696-701, respectively).
About one-third of patients with symptomatic VT manifest pulmonary embolism (PE), whereas two-thirds manifest deep vein thrombosis (DVT) (White, Circulation 107(23 Suppl 1):I4-8 Review, 2003). DVT is an acute VT in a deep vein, usually in the thigh, legs, or pelvis, and it is a serious and potentially fatal disorder that can arise as a complication for hospital patients, but may also affect otherwise healthy people (Lensing et al., Lancet 353:479-485, 1999). Large blood clots in VT may interfere with blood circulation and impede normal blood flow. In some instances, blood clots may break off and travel to distant major organs such as the brain, heart or lungs as in PE and result in fatality. There is evidence to suggest that patients with a first episode of VT be treated with anticoagulant agents (Kearon et al., New Engl J Med 340:901-907, 1999).

VT is a chronic disease with episodic recurrence; about 30% of patients develop recurrence within 10 years after a first occurrence of VT (Heit et al., Arch Intern Med. 2000; 160: 761-768; Heit et al., Thromb Haemost 2001; 86(1):452-463; and Schulman et al., J Thromb Haemost. 2006; 4: 732-742). Recurrence of VT may be referred to herein as recurrent VT. The hazard of recurrence varies with the time since the incident event and is highest within the first 6 to 12 months. Although anticoagulation is effective in preventing recurrence, the duration of anticoagulation does not affect the risk of recurrence once primary therapy for the incident event is stopped (Schulman et al., J Thromb Haemost. 2006; 4: 732-742 and van Dongen et al., Arch Intern Med. 2003; 163: 1285-1293). Independent predictors of recurrence include male gender (McRae et al., Lancet. 2006; 368: 371-378), increasing patient age and body mass index, neurological disease with leg paresis, and active cancer (Cushman et al., Am J Med. 2004; 117: 19-25; Heit et al., Arch Intern Med. 2000; 160: 761-768; Schulman et al., J Thromb Haemost. 2006; 4: 732-742; and Baglin et al., Lancet. 2003; 362: 523-526). Additional predictors include "idiopathic" venous thrombosis (Baglin et al., Lancet. 2003; 362: 523-526), a lupus anticoagulant or antiphospholipid antibody (Kearon et al., N Engl J Med. 1999; 340: 901-907 and Schulman et al., Am J Med. 1998; 104: 332-338), antithrombin, protein C or protein S deficiency (van den Belt et al., Arch Intern Med. 1997; 157: 227-232), and possibly persistently increased plasma fibrin D-dimer (Palareti et al., N Engl J Med. 2006; 355: 1780-1789) and residual venous thrombosis (Prandoni et al., Ann Intern Med. 2002; 137: 955-960).

VT and cancer can be coincident. According to clinical data prospectively collected on the population of Olmsted County, Minnesota, since 1966, the annual incidence of a first episode of DVT or PE in the general population is 117 of 100,000. Cancer alone was associated with a 4.1-fold risk of thrombosis, whereas chemotherapy increased the risk 6.5-fold. Combining these estimates yields an approximate annual incidence of VT in cancer patients of 1 in 200 cancer patients (Lee et al., Circulation. 2003; 107:I-17-I-21). Extrinsic factors such as surgery, hormonal therapy, chemotherapy, and long-term use of central venous catheters increase the cancer-associated prethrombotic state. Post-operative thrombosis occurs more frequently in patients with cancer as compared to non-neoplastic patients (Rarh et al., Blood coagulation and fibrinolysis 1992; 3:451).

Thus, there is a need for novel genetic markers that are predictive of predisposition to VT (as well as response to statin treatment for preventing VT), particularly for individuals who are unrecognized as having a predisposition to developing the disease based on conventional risk factors, as well as genetic markers that are predictive of recurrent VT in individuals who have already experienced a VT event. Such genetic markers may enable screening of VT in much larger populations compared with the populations that can currently be evaluated by using existing risk factors and biomarkers. The availability of a genetic test may allow, for example, appropriate preventive treatments for acute venous thrombotic events to be provided for high risk individuals (such preventive treatments may include, for example, statins as well as anticoagulant agents). Moreover, the discovery of genetic markers associated with VT may provide novel targets for therapeutic intervention or preventive treatments.

HMG-CoA Reductase Inhibitors (Statins)

HMG-CoA reductase inhibitors (statins) can be used for the prevention and treatment of VT, in addition to their use for the prevention and treatment of other cardiovascular diseases (CVD), particularly coronary heart disease (CHD) (including coronary events, such as myocardial infarction (MI), and cerebrovascular events, such as stroke and transient ischemic attack (TIA)). Reduction of MI, stroke, and other coronary and cerebrovascular events and total mortality by treatment with HMG-CoA reductase inhibitors has been demonstrated in a number of randomized, double-blinded, placebo-controlled prospective trials (D.D. Waters, Clin Cardiol 24(8 Suppl):III3-7 (2001); B.K. Singh and J.L. Mehta, Curr Opin Cardiol 17(5):503-11 (2002)). These drugs are thought to typically have their primary effect through the inhibition of hepatic cholesterol synthesis, thereby upregulating LDL receptors in the liver. The resultant increase in LDL catabolism results in decreased circulating LDL, a major risk factor for cardiovascular disease.

Examples of statins include, but are not limited to, atorvastatin (Lipitor®), rosuvastatin (Crestor®), pravastatin (Pravachol®), simvastatin (Zocor®), fluvastatin (Lescol®), and lovastatin (Mevacor®), as well as combination therapies that include a statin such as simvastatin + ezetimibe (Vytorin®), lovastatin + niacin (Advicor®), atorvastatin + amlodipine besylate (Caduet®), and simvastatin + niacin (Simeor®).

Statins can be divided into two types according to their physicochemical and pharmacokinetic properties. Statins such as atorvastatin, simvastatin, lovastatin, and cerivastatin are lipophilic in nature and, as such, diffuse across membranes and thus are highly cell permeable. Hydrophilic statins such as pravastatin are more polar, such that they require specific cell surface transporters for cellular uptake. K. Ziegler and W. Stunkel, Biochim Biophys Acta 1139(3):203-9 (1992); M. Yamazaki et al., Am J Physiol 264(1 Pt 1):G36-44 (1993); T. Komai et al., Biochem Pharmacol 43(4):667-70 (1992). The latter statins utilizes a transporter, OATP2, whose tissue distribution is confined to the liver and, therefore, they are relatively hepato-specific inhibitors. B. Hsiang et al., J Biol Chem 274(52):37161-37168 (1999). The former statins, not requiring specific transport mechanisms, are available to all cells and they can directly impact a much broader spectrum of cells and tissues. These differences in properties may influence the spectrum of activities that each statin possesses. Pravastatin, for instance, has a low myopathic potential in animal models and myocyte cultures compared to lipophilic statins. B.A. Masters et al., Toxicol Appl Pharmacol 131(1):163-174 (1995); K. Nakahara et al., Toxicol Appl Pharmacol 152(1):99-106 (1998); J.C. Reijneveld et al., Pediatr Res 39(6):1028-1035 (1996). Statins are reviewed in Vaughan et al., "Update on Statins: 2003", Circulation 2004; 110;886-892.

Evidence from gene association studies is accumulating to indicate that responses to drugs are, indeed, at least partly under genetic control. As such, pharmacogenetics - the study of variability in drug responses attributed to hereditary factors in different populations - may significantly assist in providing answers toward meeting this challenge. A.D. Roses, Nature 405(6788):857-865 (2000); V. Mooser et al., J Thromb Haemost 1(7):1398-1402 (2003); L.M. Humma and S.G. Terra, Am J Health Syst Pharm 59(13):1241-1252 (2002). Associations have been reported between specific genotypes, as defined by SNPs and other genetic sequence variations, and specific responses to cardiovascular drugs. For example, a polymorphism in the *KIF6* gene is associated with response to statin treatment (Iakoubova et al., "Polymorphism in KIF6 gene and benefit from statins after acute coronary syndromes: results from the PROVE IT-TIMI 22 study", J Am Coll Cardiol. 2008 Jan 29;51(4):449-55; Iakoubova et al., "Association of the 719Arg variant of K1F6 with both increased risk of coronary events and with greater response to statin therapy", J Am Coll Cardiol. 2008 Jun 3;51(22):2195; Iakoubova et al., "KIF6 Trp719Arg polymorphism and the effect of statin therapy in elderly patients: results from the PROSPER study", Eur J Cardiovasc Prev Rehabil. 2010 Apr 20; and Shiffman et al., "Effect of pravastatin therapy on coronary events in carriers of the KIF6 719Arg allele from the cholesterol and recurrent events trial", Am J Cardiol. 2010 May 1;105(9):1300-5).

There is a need for genetic markers that can be used to predict an individual's responsiveness to statins. For example, there is a growing need to better identify people who have a high chance of benefiting from statins, and those who have a low risk of developing side-effects. For example, severe myopathies represent a significant risk for a low percentage of the patient population, and this may be a particular concern for patients who are treated more aggressively with statins. Furthermore, different patients may have the same risk for adverse events but are more likely to benefit from a drug (such as statins) and this may justify use of the drug in those individuals who are more likely to benefit. Similarly, in individuals who are less likely to benefit from a drug but are at risk for adverse events, use of the drug in these individuals can be de-prioritized or delayed.

An example of a large trial which analyzed the benefits of statin treatment for reducing the risk of CVD in a large population was the JUPITER Study (described in Ridker et al., "Rosuvastatin to prevent vascular events in men and women with elevated C-reactive protein", N Engl J Med. 2008 Nov 20;359(21):2195-207), which demonstrated that rosuvastatin (Crestor®) significantly reduced the incidence of major cardiovascular events (including MI, stroke, arterial revascularization, hospitalization for unstable angina, and death from cardiovascular causes) in a study of 17,802 individuals.

## Use of HMG-CoA Reductase Inhibitors (Statins) for Venous Thrombosis (VT)

HMG-CoA reductase inhibitors (statins) can be used to reduce the risk of VT. For example, the following three case-control studies reported the association of statin use with a reduction in the number of VT events:

Simvastatin use was associated with a reduced risk of VT [OR= 0.51 (0.29-0.91)] in a Group Health Cooperative study of postmenopausal women, which contained about 500 DVT cases and 2000 controls of whom about 5% were statin users (Doggen et al., "HMG CoA reductase inhibitors and the risk of venous thrombosis among postmenopausal women", J Thromb Haemost 2004; 2: 700-1).

Current use of statins was associated with a reduced risk of venous thromboembolism [relative risk = 0.74 (95% CI, 0.63-0.85)] in a VT study which contained 3366 adult patients (18-89 years) diagnosed with primary incident venous thromboembolism (2310 with venous thrombosis and 1056 with pulmonary embolism) (Sorenson et al., "Arterial cardiovascular events, statins, low-dose aspirin and subsequent risk of venous thromboembolism: a population based case-control study", J Thromb Haemost 2009; 7: 521-8).

In another study, 154 of 4538 patients used statins (3.3%), as did 354 of 5914 control subjects (5.7%). The use of statins [odds ratio (OR) 0.45; 95% confidence interval (CI) 0.36-0.56] but not other lipid-lowering medications (OR 1.22; 95% CI 0.62-2.43), was associated with reduced VT risk as compared with individuals who did not use any lipid-lowering medication, after adjustment for age, sex, body mass index, atherosclerotic disease, anti-platelet therapy and use of vitamin K antagonists. Different types and various durations of statin therapy were all associated with reduced VT risk (Ramcharan et al., "HMG-CoA reductase inhibitors, other lipid-lowering medication, antiplatelet therapy, and the risk of venous thrombosis", J Thromb Haemost 2009; 7: 514-20).

Identification of individuals who will respond to statin therapy for the prevention or treatment of VT has the further benefit of enabling these individuals to be targeted for statin treatment as an alternative to anticoagulant therapy, which has a high risk of bleeding events, thus providing a safer course of treatment.

Single Nucleotide Polymorphisms (SNPs)

The genomes of all organisms undergo spontaneous mutations in the course of their continuing evolution, generating variant forms of progenitor genetic sequences. Gusella, Ann Rev Biochem 55:831-854 (1986). A variant form may confer an evolutionary advantage or disadvantage relative to a progenitor form or may be neutral. In some instances, a variant form confers an evolutionary advantage to individual members of a species and is eventually incorporated into the DNA of many or most members of the species and effectively becomes the progenitor form. Additionally, the effects of a variant form may be both beneficial and detrimental, depending on the environment. For example, a heterozygous sickle cell mutation confers resistance to malaria, but a homozygous sickle cell mutation is usually lethal. In many cases, both progenitor and variant forms survive and co-exist in a species population. The coexistence of multiple forms of a genetic sequence segregating at appreciable frequencies is defined as a genetic polymorphism, which includes single nucleotide polymorphisms (SNPs).

Approximately 90% of all genetic polymorphisms in the human genome are SNPs. SNPs are single base positions in DNA at which different alleles, or alternative nucleotides, exist in a population. The SNP position (interchangeably referred to herein as SNP, SNP site, SNP locus, SNP marker, or marker) is usually preceded by and followed by highly conserved sequences (*e.g.,* sequences that vary in less than 1/100 or 1/1000 members of the populations). An individual may be homozygous or heterozygous for an allele at each SNP position. A SNP can, in some instances, be referred to as a "cSNP" to denote that the nucleotide sequence containing the SNP is an amino acid coding sequence.

A SNP may arise from a substitution of one nucleotide for another at the polymorphic site. Substitutions can be transitions or transversions. A transition is the replacement of one purine nucleotide by another purine nucleotide, or one pyrimidine by another pyrimidine. A transversion is the replacement of a purine by a pyrimidine, or vice versa. A SNP may also be a single base insertion or deletion variant referred to as an "indel." Weber et al., "Human diallelic insertion/deletion polymorphisms," Am J Hum Genet 71(4):854-62 (Oct. 2002).

A synonymous codon change, or silent mutation/SNP (terms such as "SNP", "polymorphism", "mutation", "mutant", "variation", and "variant" are used herein interchangeably), is one that does not result in a change of amino acid due to the degeneracy of the genetic code. A substitution that changes a codon coding for one amino acid to a codon coding for a different amino acid (*i.e.,* a non-synonymous codon change) is referred to as a missense mutation. A nonsense mutation results in a type of non-synonymous codon change in which a stop codon is formed, thereby leading to premature termination of a polypeptide chain and a truncated protein. A read-through mutation is another type of non-synonymous codon change that causes the destruction of a stop codon, thereby resulting in an extended polypeptide product. While SNPs can be bi-, tri-, or tetra- allelic, the vast majority of SNPs are bi-allelic, and are thus often referred to as "bi-allelic markers," or "di-allelic markers."

As used herein, references to SNPs and SNP genotypes include individual SNPs and/or haplotypes, which are groups of SNPs that are generally inherited together. Haplotypes can have stronger correlations with diseases or other phenotypic effects compared with individual SNPs, and therefore may provide increased diagnostic accuracy in some cases. Stephens et al., Science 293:489-493 (Jul. 2001).

Causative SNPs are those SNPs that produce alterations in gene expression or in the expression, structure, and/or function of a gene product, and therefore are most predictive of a possible clinical phenotype. One such class includes SNPs falling within regions of genes encoding a polypeptide product, *i.e.* cSNPs. These SNPs may result in an alteration of the amino acid sequence of the polypeptide product (*i.e.*, non-synonymous codon changes) and give rise to the expression of a defective or other variant protein. Furthermore, in the case of nonsense mutations, a SNP may lead to premature termination of a polypeptide product. Such variant products can result in a pathological condition, *e.g.*, genetic disease. Examples of genes in which a SNP within a coding sequence causes a genetic disease include sickle cell anemia and cystic fibrosis.

Causative SNPs do not necessarily have to occur in coding regions; causative SNPs can occur in, for example, any genetic region that can ultimately affect the expression, structure, and/or activity of the protein encoded by a nucleic acid. Such genetic regions include, for example, those involved in transcription, such as SNPs in transcription factor binding domains, SNPs in promoter regions, in areas involved in transcript processing, such as SNPs at intron-exon boundaries that may cause defective splicing, or SNPs in mRNA processing signal sequences such as polyadenylation signal regions. Some SNPs that are not causative SNPs nevertheless are in close association with, and therefore segregate with, a disease-causing sequence. In this situation, the presence of a SNP correlates with the presence of, or predisposition to, or an increased risk in developing the disease. These SNPs, although not causative, are nonetheless also useful for diagnostics, disease predisposition screening, and other uses.

An association study of a SNP and a specific disorder involves determining the presence or frequency of the SNP allele in biological samples from individuals with the disorder of interest, such as VT, and comparing the information to that of controls (*i.e.*, individuals who do not have the disorder; controls may be also referred to as "healthy" or "normal" individuals) who are preferably of similar age and race. The appropriate selection of patients and controls is important to the success of SNP association studies. Therefore, a pool of individuals with well-characterized phenotypes is extremely desirable.

A SNP may be screened in diseased tissue samples or any biological sample obtained from a diseased individual, and compared to control samples, and selected for its increased (or decreased) occurrence in a specific pathological condition, such as pathologies related to VT. Once a statistically significant association is established between one or more SNP(s) and a pathological condition (or other phenotype) of interest, then the region around the SNP can optionally be thoroughly screened to identify the causative genetic locus/sequence(s) (*e.g.*, causative SNP/mutation, gene, regulatory region, etc.) that influences the pathological condition or phenotype. Association studies may be conducted within the general population and are not limited to studies performed on related individuals in affected families (linkage studies).

Clinical trials have shown that patient response to treatment with pharmaceuticals is often heterogeneous. There is a continuing need to improve pharmaceutical agent design and therapy. In that regard, SNPs can be used to identify patients most suited to therapy with particular pharmaceutical agents (this is often termed "pharmacogenomics"). Similarly, SNPs can be used to exclude patients from certain treatment due to the patient's increased likelihood of developing toxic side effects or their likelihood of not responding to the treatment. Pharmacogenomics can also be used in pharmaceutical research to assist the drug development and selection process. Linder et al., Clinical Chemistry 43:254 (1997); Marshall, Nature Biotechnology 15:1249 (1997); International Patent Application WO 97/40462, Spectra Biomedical; and Schafer et al., Nature Biotechnology 16:3 (1998).

SUMMARY OF THE INVENTION

The present disclosure relates to the identification of SNPs that are associated with risk for developing venous thrombosis (VT) and/or variability between individuals in their response to statins, particularly for the prevention or treatment of VT. These SNPs are useful for determining risk and/or statin response for primary and recurrent VT. Accordingly, the polymorphisms disclosed herein are directly useful as targets for the design of diagnostic and prognostic reagents and the development of therapeutic and preventive agents for use in the diagnosis, prognosis, treatment, and/or prevention of VT, as well as for predicting a patient's response to therapeutic agents such as statins, particularly for the treatment or prevention of VT.

Based on the identification of SNPs associated with risk for developing VT and/or variability between individuals in their response to statins, particularly for reducing the risk of VT, examples disclosed herein describe methods of detecting these variants as well as the design and preparation of detection reagents needed to accomplish this task. The disclosure specifically describes, for example, SNPs associated with VT risk and/or responsiveness to statin treatment for reducing VT risk, isolated nucleic acid molecules (including DNA and RNA molecules) containing these SNPs, variant proteins encoded by nucleic acid molecules containing such SNPs, antibodies to the encoded variant proteins, computer-based and data storage systems containing the novel SNP information, methods of detecting these SNPs in a test sample, methods of identifying individuals who have an altered (*i.e.,* increased or decreased) risk of developing VT, methods for determining the risk of an individual for developing recurrent VT, methods of treating an individual who has an increased risk for VT, and methods for identifying individuals (*e.g.*, determining a particular individual's likelihood) who have an altered (*i.e.*, increased or decreased) likelihood of responding to drug treatment (especially statin treatment), particularly drug treatment of VT, based on the presence or absence of one or more particular nucleotides (alleles) at one or more SNP sites disclosed herein or the detection of one or more encoded variant products (*e.g.*, variant mRNA transcripts or variant proteins), methods of identifying individuals who are more or less likely to respond to a treatment such as statins, methods of screening for compounds useful in the treatment or prevention of VT, compounds identified by these methods, methods of treating or preventing VT, etc.

The invention provides a method for determining whether a human's risk for venous thrombosis (VT) is reduced by treatment with an HMG-CoA reductase inhibitor, the method comprising testing nucleic acid from said human for the presence or absence of an allele at a polymorphism (rs2036914) represented by position 101 of the nucleotide sequence of SEQ ID NO:713 or its complement, wherein the presence of C at position 101 of SEQ ID NO:713 or G at position 101 of its complement indicates said human's risk for VT is reduced by treatment with said HMG-CoA reductase inhibitor.

In one embodiment, the method further comprises correlating the presence of said allele with a reduction of said risk for VT by an HMG-CoA reductase inhibitor. In another embodiment, said correlating is performed by computer software.

In one embodiment, said HMG-CoA reductase inhibitor is a hydrophilic statin, or a hydrophobic statin. In a more specific embodiment, said HMG-CoA reductase inhibitor is selected from the group consisting of atorvastatin, rosuvastatin, pravastatin, simvastatin, fluvastatin, and lovastatin, or any combination thereof. In one embodiment, said treatment with an HMG-CoA reductase inhibitor comprises treatment with an HMG-CoA reductase inhibitor in combination with at least one additional therapeutic agent. In a specific embodiment said HMG-CoA reductase inhibitor in combination with at least one additional therapeutic agent is selected from the group consisting of: simvastatin in combination with ezetimibe; lovastatin in combination with niacin; atorvastatin in combination with amlodipine besylate; and simvastatin in combination with niacin.

In another embodiment, said nucleic acid is a nucleic acid extract from a biological sample from said human. In one embodiment, said biological sample is blood, saliva, or buccal cells. In yet another embodiment, the method further comprises preparing said nucleic acid extract from said biological sample prior to said testing, preferably further comprises obtaining said biological sample from said human prior to said preparing. In one embodiment, said testing comprises nucleic acid amplification. In a specific embodiment, said nucleic acid amplification is carried out by polymerase chain reaction. In another embodiment, said testing is performed using sequencing, 5' nuclease digestion, molecular beacon assay, oligonucleotide ligation assay, single-stranded conformation polymorphism analysis, or denaturing gradient gel electrophoresis (DGGE) or an allele-specific method. In a specific embodiment, said allele-specific method comprises allele-specific probe hybridization, allele-specific primer extension, or allele-specific amplification.

In one embodiment, the method is an automated method. In another embodiment, said human is homozygous, or heterozygous for said allele. In one embodiment, said VT is deep vein thrombosis (DVT), or pulmonary embolism (PE). In another embodiment, said human did not have VT prior to said testing. In yet another embodiment, said human had VT prior to said testing and said risk is for recurrent VT.

In another embodiment, the method further comprises detecting any one or more of the following polymorphisms: an *F5* polymorphism rs6025, an *FGG* polymorphism rs2066865, an *ABO* polymorphism rs8176719, and an F2 polymorphism rs1799963. In a specific embodiment, the method comprises detecting the following polymorphisms: an *F5* polymorphism rs6025, an *FGG* polymorphism rs2066865, an *ABO* polymorphism rs8176719, and an F2 polymorphism rs1799963.

Further, the invention provides an HMG-CoA reductase inhibitor for use in a method of reducing the risk for VT, wherein the method comprises performing a method according to the present invention and administering the HMG-CoA reductase inhibitor to a human who has said allele.

The invention also provides the use of a detection reagent in the method of the present invention, wherein said detection reagent is an allele-specific probe or an allele-specific primer. Finally, the invention provides the use of a test kit in the method of the present invention, wherein said test kit comprises one or more containers containing the detection reagent of the present invention and one or more components selected from the group consisting of an enzyme, polymerase enzyme, ligase enzyme, buffer, amplification primer pair, dNTPs, ddNTPs, positive control nucleic acid, nucleic acid extraction reagent, and instructions for using said test kit which instruct that the presence of said allele indicates that said risk for VT is reduced by treatment with said HMG-CoA reductase inhibitor.

Hereinafter, reference to SNPs in the context of the invention shall mean the SNP (rs2036914) as defined above.

Exemplary embodiments of the present invention encompass selecting or formulating a treatment regimen (*e.g.*, methods for determining whether or not to administer statin treatment to an individual having VT, or who is at risk for developing VT in the future, or who has previously had VT, methods for selecting a particular statin-based treatment regimen such as dosage and frequency of administration of statin, or a particular form/type of statin such as a particular pharmaceutical formulation or statin compound, methods for administering an alternative, non-statin-based treatment (such as warfarin or other anticoagulants, e.g., direct thrombin inhibitors such as dabigatran, or direct factor Xa inhibitors such as rivaroxaban or apixaban) to individuals who are predicted to be unlikely to respond positively to statin treatment, etc.), and methods for determining the likelihood of experiencing toxicity or other undesirable side effects from statin treatment, etc. Various embodiments of the present invention also encompass selecting individuals to whom a statin will be administered based on the individual's genotype, and methods for selecting individuals for a clinical trial of a statin based on the genotypes of the individuals (*e.g.*, selecting individuals to participate in the trial who are most likely to respond positively from the statin treatment and/or excluding individuals from the trial who are unlikely to respond positively from the statin treatment based on their SNP genotype(s), or selecting individuals who are unlikely to respond positively to statins based on their SNP genotype(s) to participate in a clinical trial of another type of drug that may benefit them). Further embodiments of the present invention provide methods for reducing an individual's risk of developing VT using statin treatment, including preventing recurrent VT using statin treatment, when said individual carries the SNP of the invention, associated with statin response.

Tables 1 and 2 provides gene information, references to the identification of transcript sequences (SEQ ID NOS:1-84), encoded amino acid sequences (SEQ ID NOS:85-168), genomic sequences (SEQ ID NOS:338-500), transcript-based context sequences (SEQ ID NOS:169-337) and genomic-based context sequences (SEQ ID NOS:501-3098) that contain the SNP of the present application, and extensive SNP information that includes observed alleles, allele frequencies, populations/ethnic groups in which alleles have been observed, information about the type of SNP and corresponding functional effect, and, for cSNPs, information about the encoded polypeptide product. The actual transcript sequences (SEQ ID NOS:1-84), amino acid sequences (SEQ ID NOS:85-168), genomic sequences (SEQ ID NOS:338-500), transcript-based SNP context sequences (SEQ ID NOS:169-337), and genomic-based SNP context sequences (SEQ ID NOS:501-3098) are provided in the Sequence Listing.

In certain examples, disclosed herein are methods for identifying an individual who has an altered risk for developing VT (including, for example, a first incidence and/or a recurrence of the disease, such as primary or recurrent VT), in which the method comprises detecting a single nucleotide polymorphism (SNP) in any one of the nucleotide sequences of SEQ ID NOS:1-84, SEQ ID NOS: 169-337, SEQ ID NOS:338-500, and SEQ ID NOS:501-3098 in said individual's nucleic acids, wherein the SNP is specified in Table 1 and/or Table 2, and the presence of the SNP is indicative of an altered risk for VT in said individual. The VT is deep vein thrombosis (DVT), pulmonary embolism (PE), or recurrent VT. In certain exemples, SNPs that occur naturally in the human genome are provided within isolated nucleic acid molecules. These SNPs are associated with response to statin treatment thereby reducing the risk of VT, such that they can have a variety of uses in the diagnosis, prognosis, treatment, and/or prevention of VT, and particularly in the treatment or prevention of VT using statins. In an alternative example, a nucleic acid disclosed herein is an amplified polynucleotide, which is produced by amplification of a SNP-containing nucleic acid template. In another example, disclosed herein is a variant protein that is encoded by a nucleic acid molecule containing

In further embodiments, the invention provides reagents for detecting the SNP of the invention in the context of its naturally-occurring flanking nucleotide sequences (which can be, *e.g.*, either DNA or mRNA). In particular, such a reagent may be in the form of, for example, a hybridization probe or an amplification primer that is useful in the specific detection of a SNP of interest. In an alternative embodiment, a protein detection reagent is used to detect a variant protein that is encoded by a nucleic acid molecule containing the SNP provided herein. A preferred embodiment of a protein detection reagent is an antibody or an antigen-reactive antibody fragment. Various embodiments of the invention also provide kits comprising SNP detection reagents, and methods for detecting the SNPs disclosed herein by employing the SNP detection reagents. An exemplary embodiment of the present invention provides a kit comprising the SNP detection reagent for use in determining whether a human's risk for VT is reduced by treatment with statins based upon the presence or absence of a particular allele of the SNP of the invention.

In various embodiments, the present invention provides methods for evaluating whether an individual is likely (or unlikely) to respond to statin treatment (i.e., benefit from statin treatment)), particularly statin treatment for reducing the risk of VT (including recurrent VT), by detecting the presence or absence of the SNP alleles of the invention. In certain embodiments, the VT is DVT or PE. In certain embodiments, the VT is recurrent VT. Disclosed herein are also methods of identifying an individual having an increased or decreased risk of developing VT (including recurrent VT) by detecting the presence or absence of one or more SNP alleles disclosed herein. In certain embodiments, the VT is DVT or PE. In other examples, a method for diagnosis or prognosis of VT by detecting the presence or absence of one or more SNP alleles is disclosed herein.

The nucleic acid molecules disclosed herein can be inserted in an expression vector, such as to produce a variant protein in a host cell. Thus, disclosed herein is a vector comprising a SNP-containing nucleic acid molecule, genetically-engineered host cells containing the vector, and methods for expressing a recombinant variant protein using such host cells. In another example, the host cells, SNP-containing nucleic acid molecules, and/or variant proteins can be used as targets in a method for screening and identifying therapeutic agents or pharmaceutical compounds useful in the treatment or prevention of VT.

An example of this disclosure is a method for treating or preventing VT (including, for example, a first occurrence and/or a recurrence of the disease, such as primary or recurrent VT), in a human subject wherein said human subject harbors a SNP, gene, transcript, and/or encoded protein identified in Tables 1 and 2, which method comprises administering to said human subject a therapeutically or prophylactically effective amount of one or more agents counteracting the effects of the disease, such as by inhibiting (or stimulating) the activity of a gene, transcript, and/or encoded protein identified in Tables 1 and 2.

Another example of this disclosure is a method for identifying an agent useful in therapeutically or prophylactically treating VT, in a human subject wherein said human subject harbors a SNP, gene, transcript, and/or encoded protein identified in Tables 1 and 2, which method comprises contacting the gene, transcript, or encoded protein with a candidate agent under conditions suitable to allow formation of a binding complex between the gene, transcript, or encoded protein and the candidate agent and detecting the formation of the binding complex, wherein the presence of the complex identifies said agent.

Another example disclosed herein is a method for treating or preventing VT, in a human subject, in which the method comprises:

(i) determining that said human subject harbors a SNP, gene, transcript, and/or encoded protein identified in Tables 1 and 2, and
(ii) administering to said subject a therapeutically or prophylactically effective amount of one or more agents counteracting the effects of the disease, such as statins.

Another example disclosed herein is a method for identifying a human who is likely to benefit from statin treatment, in which the method comprises detecting an allele of one or more SNPs disclosed herein in said human's nucleic acids, wherein the presence of the allele indicates that said human is likely to benefit from statin treatment.

Another example disclosed herein is a method for identifying a human who is likely to benefit from statin treatment, in which the method comprises detecting an allele of one or more SNPs that are in LD with one or more SNPs disclosed herein in said human's nucleic acids, wherein the presence of the allele of the LD SNP indicates that said human is likely to benefit from statin treatment.

Many other uses and advantages of the present invention will be apparent to those skilled in the art upon review of the detailed description of the exemplary embodiments herein. Solely for clarity of discussion, the invention is described in the sections below by way of non-limiting examples.

DESCRIPTION OF THE TEXT (ASCII) FILE SUBMITTED ELECTRONICALLY

The following text (ASCII) files is submitted electronically as part of the instant application: File 103221PCEPT1-103221PCEPT1_seqlist.TXT provides the Sequence Listing. The Sequence Listing provides the transcript sequences (SEQ ID NOS:1-84) and protein sequences (SEQ ID NOS:85-168) as referred to in Table 1, and genomic sequences (SEQ ID NOS:338-500) as referred to in Table 2, for each gene (or genomic region for intergenic SNPs) that contains one or more statin response-associated SNPs of the present dissclosure. Also provided in the Sequence Listing are context sequences flanking each SNP, including both transcript-based context sequences as referred to in Table 1 (SEQ ID NOS:169-337) and genomic-based context sequences as referred to in Table 2 (SEQ ID NOS:501-3098). The context sequences generally provide 100bp upstream (5') and 100bp downstream (3') of each SNP, with the SNP in the middle of the context sequence, for a total of 200bp of context sequence surrounding each SNP.

DESCRIPTION OF THE FIGURE

The Figure shows two SNP in the *F11* gene significantly associated with statin response for reducing VT risk: *F11* SNP rs2036914 and *F11* SNP rs2289252. The Figure shows risk of VT according to statin use for rs2289252, rs2036914, and Factor V Leiden genotypes. The odds ratios (shown with 95% confidence intervals) were adjusted for sex and age.

DESCRIPTION OF TABLE 1 AND TABLE 2

Table 1 and Table 2 disclose the SNP and associated gene/transcript/protein information of the present invention. For each gene, Table 1 provides a header containing gene, transcript and protein information, followed by a transcript and protein sequence identifier (SEQ ID NO), and then SNP information regarding each SNP found in that gene/transcript including the transcript context sequence. For each gene in Table 2, a header is provided that contains gene and genomic information, followed by a genomic sequence identifier (SEQ ID NO) and then SNP information regarding each SNP found in that gene, including the genomic context sequence.

Note that SNP markers may be included in both Table 1 and Table 2; Table 1 presents the SNPs relative to their transcript sequences and encoded protein sequences, whereas Table 2 presents the SNPs relative to their genomic sequences, In some instances Table 2 may also include, after the last gene sequence, genomic sequences of one or more intergenic regions, as well as SNP context sequences and other SNP information for any SNPs that lie within these intergenic regions. Additionally, in either Table 1 or 2 a "Related Interrogated SNP" may be listed following a SNP which is determined to be in LD with that interrogated SNP according to the given Power value. SNPs can be readily cross-referenced between all Tables based on their Celera hCV (or, in some instances, hDV) identification numbers and/or public rs identification numbers, and to the Sequence Listing based on their corresponding SEQ ID NOs.

**[0057]** The gene/transcript/protein information includes:

- a gene number (1 through n, where n = the total number of genes in the Table),
- a gene symbol, along with an Entrez gene identification number (Entrez Gene database, National Center for Biotechnology Information (NCBI), National Library of Medicine, National Institutes of Health) (if Entrez gene information is unavailable, then Ensembl gene information is used instead)
- a gene name,
- an accession number for the transcript (*e.g.*, RefSeq NM number and/or a Celera hCT identification number) (Table 1 only) (if RefSeq transcript information is unavailable, then Ensembl transcript information is used instead),
- an accession number for the protein (*e.g.*, RefSeq NP number and/or a Celera hCP identification number) (Table 1 only) (if RefSeq protein information is unavailable, then Ensembl protein information is used instead),
- the chromosome number of the chromosome on which the gene is located,
- an OMIM ("Online Mendelian Inheritance in Man" database, Johns Hopkins University/NCBI) public reference number for the gene, and OMIM information such as alternative gene/protein name(s) and/or symbol(s) in the OMIM entry.

Note that, due to the presence of alternative splice forms, multiple transcript/protein entries may be provided for a single gene entry in Table 1; *i.e.*, for a single Gene Number, multiple entries may be provided in series that differ in their transcript/protein information and sequences.

Following the gene/transcript/protein information is a transcript context sequence (Table 1), or a genomic context sequence (Table 2), for each SNP within that gene.

After the last gene sequence, Table 2 may include additional genomic sequences of intergenic regions (in such instances, these sequences are identified as "Intergenic region:" followed by a numerical identification number), as well as SNP context sequences and other SNP information for any SNPs that lie within each intergenic region (such SNPs are identified as "INTERGENIC'" for SNP type).

Note that the transcript, protein, and transcript-based SNP context sequences are all provided in the Sequence Listing. The transcript-based SNP context sequences are provided in both Table 1 and also in the Sequence Listing. The genomic and genomic-based SNP context sequences are provided in the Sequence Listing. The genomic-based SNP context sequences are provided in both Table 2 and in the Sequence Listing. SEQ ID NOs are indicated in Table 1 for the transcript-based context sequences (SEQ ID NOS:169-337); SEQ ID NOs are indicated in Table 2 for the genomic-based context sequences (SEQ ID NOS:501-3098).

The SNP information includes:

- Context sequence (taken from the transcript sequence in Table 1, the genomic sequence in Table 2) with the SNP represented by its IUB code, including 100bp upstream (5') of the SNP position plus 100bp downstream (3') of the SNP position (the transcript-based SNP context sequences in Table 1 are provided in the Sequence listing as SEQ ID NOS:169-337; the genomic-based SNP context sequences in Table 2 are provided in the Sequence Listing as SEQ ID NOS:501-3098).
- Celera hCV internal identification number for the SNP (in some instances, an "hDV" number is given instead of an "hCV" number).
- The corresponding public identification number for the SNP, the rs number.
- "SNP Chromosome Position" indicates the nucleotide position of the SNP along the entire sequence of the chromosome as provided in NCBI Genome Build 37.
- SNP position (nucleotide position of the SNP within the given transcript sequence (Table 1) or within the given genomic sequence (Table 2)).
- "Related Interrogated SNP" is the interrogated SNP with which the listed SNP is in LD at the given value of Power.
- SNP source (may include any combination of one or more of the following five codes, depending on which internal sequencing projects and/or public databases the SNP has been observed in: "Applera" = SNP observed during the re-sequencing of genes and regulatory regions of 39 individuals, "Celera" = SNP observed during shotgun sequencing and assembly of the Celera human genome sequence, "Celera Diagnostics" = SNP observed during re-sequencing of nucleic acid samples from individuals who have a disease, "dbSNP" = SNP observed in the dbSNP public database, "HGBASE" = SNP observed in the HGBASE public database, "HGMD" = SNP observed in the Human Gene Mutation Database (HGMD) public database, "HapMap" = SNP observed in the International HapMap Project public database, "CSNP" = SNP observed in an internal Applied Biosystems (Foster City, CA) database of coding SNPS (cSNPs). Note that multiple "Applera" source entries for a single SNP indicate that the same SNP was covered by multiple overlapping amplification products and the re-sequencing results (*e.g.*, observed allele counts) from each of these amplification products is being provided.

- Population/allele/allele count information in the format of [population1(first_allele,count|second_allele,count)population2(first_allele,count|second_allele,count) total (first_allele,total count|second_allele,total count)]. The information in this field includes populations/ethnic groups in which particular SNP alleles have been observed ("cau" = Caucasian, "his" = Hispanic, "chn" = Chinese, and "afr" = African-American, "jpn" = Japanese, "ind" = Indian, "mex" = Mexican, "ain" = "American Indian, "cra" = Celera donor, "no_pop" = no population information available), identified SNP alleles, and observed allele counts (within each population group and total allele counts), where available ["-" in the allele field represents a deletion allele of an insertion/deletion ("indel") polymorphism (in which case the corresponding insertion allele, which may be comprised of one or more nucteotides, is indicated in the allele field on the opposite side of the "|"); "-"in the count field indicates that allele count information is not available]. For certain SNPs from the public dbSNP database, population/ethnic information is indicated as follows (this population information is publicly available in dbSNP): "HISP1" = human individual DNA (anonymized samples) from 23 individuals of self-desclibed HISPANIC heritage; "PAC1" = human individual DNA (anonymized samples) from 24 individuals of self-described PACIFIC RIM heritage; "CAUC1" = human individual DNA (anonymized samples) from 31 individuals of self-described CAUCASIAN heritage; "AFR1" = human individual DNA (anonymized samples) from 24 individuals of self-described AFRICAN/AFRICAN AMERICAN heritage; "P1" = human individual DNA (anonymized samples) from 102 individuals of self-described heritage; "PA130299515"; "SC_12_A" = SANGER 12 DNAs of Asian origin from Corielle cell repositories, 6 of which are male and 6 female; "SC_12_C" = SANGER 12 DNAs of Caucasian origin from Corielle cell repositories from the CEPH/UTAH library, six male and six female; "SC_12_AA" = SANGER 12 DNAs of African-American origin from Corielle cell repositories 6 of which are male and 6 female; "SC_95_C" = SANGER 95 DNAs of Caucasian origin from Corielle cell repositories from the CEPH/UTAH library; and "SC_12_CA" = Caucasians - 12 DNAs from Corielle cell repositories that are from the CEPH/UTAH library, six male and six female.

Note that for SNPs of "Applera" SNP source, genes/regulatory regions of 39 individuals (20 Caucasians and 19 African Americans) were re-sequenced and, since each SNP position is represented by two chromosomes in each individual (with the exception of SNPs on X and Y chromosomes in males, for which each SNP position is represented by a single chromosome), up to 78 chromosomes were genotyped for each SNP position. Thus, the sum of the African-American ("afr") allele counts is up to 38, the sum of the Caucasian allele counts ("cau") is up to 40, and the total sum of all allele counts is up to 78.

Note that semicolons separate population/allele/count information corresponding to each indicated SNP source; *i.e.*, if four SNP sources are indicated, such as "Celera," "dbSNP," "HGBASE," and "HGMD," then population/allele/count information is provided in four groups which are separated by semicolons and listed in the same order as the listing of SNP sources, with each population/allele/count information group corresponding to the respective SNP source based on order; thus, in this example, the first population/allele/count information group would correspond to the first listed SNP source (Celera) and the third population/allele/count information group separated by semicolons would correspond to the third listed SNP source (HGBASE); if population/allele/count information is not available for any particular SNP source, then a pair of semicolons is still inserted as a place-holder in order to maintain correspondence between the list of SNP sources and the corresponding listing of population/allele/count information.

- SNP type (*e.g.*, location within gene/transcript and/or predicted functional effect) ["MIS-SENSE MUTATION" = SNP causes a change in the encoded amino acid (*i.e.*, a non-synonymous coding SNP); "SILENT MUTATION" = SNP does not cause a change in the encoded amino acid (*i.e.*, a synonymous coding SNP); "STOP CODON MUTATION" = SNP is located in a stop codon; "NONSENSE MUTATION" = SNP creates or destroys a stop codon; "UTR 5" = SNP is located in a 5' UTR of a transcript; "UTR 3" = SNP is located in a 3' UTR of a transcript; "PUTATIVE UTR 5" = SNP is located in a putative 5' UTR; "PUTATIVE UTR 3" = SNP is located in a putative 3' UTR; "DONOR SPLICE SITE" = SNP is located in a donor splice site (5' intron boundary); "ACCEPTOR SPLICE SITE" = SNP is located in an acceptor splice site (3' intron boundary); "CODING REGION" = SNP is located in a protein-coding region of the transcript; "EXON" = SNP is located in an exon; "INTRON" = SNP is located in an intron; "hmCS" = SNP is located in a human-mouse conserved segment; "TFBS" = SNP is located in a transcription factor binding site; "UNKNOWN" = SNP type is not defined; "INTERGENIC" = SNP is intergenic, *i.e.*, outside of any gene boundary].

- Protein coding information (Table 1 only), where relevant, in the format of [protein SEQ ID NO, amino acid position, (amino acid-1, codon1) (amino acid-2, codon2)]. The information in this field includes SEQ ID NO of the encoded protein sequence, position of the amino acid residue within the protein identified by the SEQ ID NO that is encoded by the codon containing the SNP, amino acids (represented by one-letter amino acid codes) that are encoded by the alternative SNP alleles (in the case of stop codons, "X" is used for the one-letter amino acid code), and alternative codons containing the alternative SNP nucleotides which encode the amino acid residues (thus, for example, for missense mutation-type SNPs, at least two different amino acids and at least two different codons are generally indicated; for silent mutation-type SNPs, one amino acid and at least two different codons are generally indicated, etc.). In instances where the SNP is located outside of a protein-coding region (*e.g.,* in a UTR region), "None" is indicated following the protein SEQ ID NO.

DESCRIPTION OF TABLE 3

Table 3 provides a list of LD SNPs that are related to and derived from certain interrogated SNPs. The interrogated SNPs, which are shown in column 1 (which indicates the hCV identification numbers of each interrogated SNP) and column 2 (which indicates the public rs identification numbers of each interrogated SNP) of Table 3, are statistically significantly associated with VT risk (particularly risk for recurrent VT) and/or statin response for reducing VT risk, as described and shown herein, particularly in Tables 4-9 and in the Examples sections below. The LD SNPs are provided as an example of SNPs which can also serve as markers for disease association based on their being in LD with an interrogated SNP. The criteria and process of selecting such LD SNPs, including the calculation of the $r^2$ value and the threshold $r^2$ value, are described in Example 7, below.

In Table 3, the column labeled "Interrogated SNP" presents each marker as identified by its unique hCV identification number. The column labeled "Interrogated rs" presents the publicly known rs identification number for the corresponding hCV number. The column labeled "LD SNP" presents the hCV numbers of the LD SNPs that are derived from their corresponding interrogated SNPs. The column labeled "LD SNP rs" presents the publicly known rs identification number for the corresponding hCV number. The column labeled "Power" presents the level of power where the $r^2$

threshold is set. For example, when power is set at .51, the threshold $r^2$ value calculated therefrom is the minimum $r^2$ that an LD SNP must have in reference to an interrogated SNP, in order for the LD SNP to be classified as a marker capable of being associated with a disease phenotype at greater than 51 % probability. The column labeled "Threshold $r^2$" presents the minimum value of $r^2$ that an LD SNP must meet in reference to an interrogated SNP in order to qualify as an LD SNP. The column labeled "$r^2$" presents the actual $r^2$ value of the LD SNP in reference to the interrogated SNP to which it is related.

DESCRIPTION OF TABLES 4-9

Tables 4-9 provide the results of analyses for SNPs disclosed in Tables 1 and 2 (SNPs can be cross-referenced between all the tables herein based on their hCV and/or rs identification numbers).

The analyses in Tables 4-6 are further described in Example 1 below.

The analysis in Table 7 is further described in Example 3 below.

The analysis in Table 8 is further described in Example 4 below.

The analysis in Table 9 is further described in Example 5 below.

The results shown in Tables 4-9 provide support for the association of these SNPs with VT risk, particularly risk for recurrent VT, and/or response to statin treatment for reducing the risk of VT.

In Tables 4-6, "statin_1" or "statin user" are equivalent designations that refer to individuals who were using statins, and "statin_0" or "statin nonuser" are equivalent designations that refer to individuals who were not using statins.

Throughout Tables 4-9, "P" or "P-value" indicates the p-value, "p(int)" indicates the p(interaction) value, "OR" refers to the odds ratio, "HR" refers to the hazard ratio, and "95% CI" refers to the 95% confidence interval for the odds ratio or hazard ratio.

In Tables 7-9, "P_DF2" indicates the two degrees of freedom Wald Test p-value.

In Tables 8-9, "HW(control)pExact" indicates the Hardy-Weinberg p-value for all controls in the study.

With respect to drug response (*e.g.*, response to a statin), if the OR or IIR of those treated with the drug (*e.g., a statin*) compared with those treated with a placebo within a particular genotype (or with a particular allele) is less than one, this indicates that an individual with this particular genotype or allele would benefit from the drug (an OR or HR equal to one would indicate that the drug has no effect). In contrast, with respect to drug response, if the OR or HR is greater than one for a particular allele, then this indicates that an individual with the other alternative allele would benefit from the drug. As used herein, the term "benefit" (with respect to a preventive or therapeutic drug treatment) is defined as achieving a reduced risk for a disease that the drug is intended to treat or prevent (*e.g.,* VT) by administering the drug treatment, compared with the risk for the disease in the absence of receiving the drug treatment (or receiving a placebo in lieu of the drug treatment) for the same genotype.

With respect to disease risk, an OR or HR that is greater than one indicates that a given allele is a risk allele (which may also be referred to as a susceptibility allele), whereas an OR or HR that is less than one indicates that a given allele is a non-risk allele (which may also be referred to as a protective allele). For a given risk allele, the other alternative allele at the SNP position (which can be derived from the information provided in Tables 1-2, for example) may be considered a non-risk allele. For a given non-risk allele, the other alternative allele at the SNP position may be considered a risk allele. Thus, with respect to disease risk, if the OR or HR for a particular allele at a SNP position is greater than one, this indicates that an individual with this particular allele has a higher risk for the disease than an individual who has the other allele at the SNP position. In contrast, if the OR for a particular allele is less than one, this indicates that an individual with this particular allele has a reduced risk for the disease compared with an individual who has the other allele at the SNP position.

DETAILED DESCRIPTION OF THE INVENTION

The present invention relates to SNP (rs2036914) associated with risk for developing venous thrombosis (VT) (interchangeably referred to as venous thromboembolism (VTE)) and response to statin treatment, particularly statin treatment for reducing the risk of VT, and methods for their use. The present disclosure further describes nucleic acid molecules containing these SNPs, methods and reagents for the detection of the SNPs disclosed herein, uses of these SNPs for the development of detection reagents, and assays or kits that utilize such reagents. The statin response-associated SNPs disclosed herein are particularly useful for predicting, screening for, and evaluating response to statin treatment, particularly for prevention or treatment of VT using statins, in humans. The SNPs disclosed herein are also useful for diagnosing, prognosing, screening for, and evaluating predisposition to VT in humans. Furthermore, such SNPs and their encoded products are useful targets for the development of therapeutic and preventive agents.

Thus, disclosed herein are individual SNPs associated with risk for developing VT and/or response to statin treatment, particularly statin treatment for reducing the risk of VT, as well as combinations of SNPs and haplotypes, polymorphic/variant transcript sequences (SEQ ID NOS:1-84) and genomic sequences (SEQ ID NOS:338-500) containing SNPs, encoded amino acid sequences (SEQ ID NOS:85-168), and both transcript-based SNP context sequences (SEQ ID NOS: 169-337) and genomic-based SNP context sequences (SEQ ID NOS:501-3098) (transcript sequences, protein sequences, and transcript-based SNP context sequences are provided in Table 1 and the Sequence Listing; genomic sequences and genomic-based SNP context sequences are provided in Table 2 and the Sequence Listing), methods of detecting these polymorphisms in a test sample, methods of determining an individual's risk for developing VT, methods of determining if an individual is likely to respond to a particular treatment such as statins (particularly for treating or preventing VT), methods of screening for compounds useful for treating VT, compounds identified by these screening methods, methods of using the disclosed SNPs to select a treatment/preventive strategy or therapeutic agent, and methods of treating or preventing VT.

Exemplary embodiments of the present invention encompass selecting or formulating a treatment regimen (*e.g.,* methods for determining whether or not to administer statin treatment to an individual having VT, or who is at risk for developing VT in the future, or who has previously had VT, methods for selecting a particular statin-based treatment regimen such as dosage and frequency of administration of statin, or a particular form/type of statin such as a particular pharmaceutical formulation or statin compound, methods for administering an alternative, non-statin-based treatment (such as warfarin or other anticoagulants, e.g., direct thrombin inhibitors such as dabigatran, or direct factor Xa inhibitors such as rivaroxaban or apixaban) to individuals who are predicted to be unlikely to respond positively to statin treatment, etc.), and methods for determining the likelihood of experiencing toxicity or other undesirable side effects from statin treatment, etc. The present invention also encompasses selecting individuals to whom a statin will be administered based on the individual's genotype, and methods for selecting individuals for a clinical trial of a statin based on the genotypes of the individuals (*e.g.,* selecting individuals to participate in the trial who are most likely to respond positively from the statin treatment and/or excluding individuals from the trial who are unlikely to respond positively from the statin treatment based on their SNP genotype(s), or selecting individuals who are unlikely to respond positively to statins based on their SNP genotype(s) to participate in a clinical trial of another type of drug that may benefit them).

Examples disclosed herein may include novel SNPs associated with VT risk and/or response to statin treatment, as well as SNPs that were previously known in the art, but were not previously known to be associated with VT risk and/or response to statin treatment. Accordingly, disclosed herein are novel compositions and methods based on novel SNPs disclosed herein, and methods of using known, but previously unassociated, SNPs in methods relating to, for example, methods relating to evaluating an individual's likelihood of responding to statin treatment (particularly statin treatment, including preventive treatment, of VT. including recurrent VT), evaluating an individual's likelihood of having or developing VT, and predicting the likelihood of an individual experiencing a reccurrence of VT. In Tables 1 and 2, known SNPs are identified based on the public database in which they have been observed, which is indicated as one or more of the following SNP types: "dbSNP" = SNP observed in dbSNP, "HGBASE" = SNP observed in HGBASE, and "HGMD" = SNP observed in the Human Gene Mutation Database (HGMD).

Particular alleles of the SNPs disclosed herein can be associated with either an increased likelihood of responding to statin treatment (particularly for reducing the risk of VT) or increased risk of developing VT, or a decreased likelihood of responding to statin treatment or a decreased risk of developing VT. Thus, whereas certain SNPs (or their encoded products) can be assayed to determine whether an individual possesses a SNP allele that is indicative of an increased likelihood of responding to statin treatment or an increased risk of developing VT, other SNPs (or their encoded products) can be assayed to determine whether an individual possesses a SNP allele that is indicative of a decreased likelihood of responding to statin treatment or a decreased risk of developing VT. Similarly, particular alleles of the SNPs disclosed herein can be associated with either an increased or decreased likelihood of having a reccurrence of VT, or of experiencing toxic effects from a particular treatment or therapeutic compound such as statins, etc. The term "altered" may be used herein to encompass either of these two possibilities (*e.g.,* either an increased or a decreased likelihood/risk). SNP alleles that are associated with a decreased risk of having or developing VT may be referred to as "protective" alleles, and SNP alleles that are associated with an increased risk of having or developing VT may be referred to as "susceptibility" alleles, "risk" alleles, or "risk factors".

Those skilled in the art will readily recognize that nucleic acid molecules may be double-stranded molecules and that reference to a particular site on one strand refers, as well, to the corresponding site on a complementary strand. In defining a SNP position, SNP allele, or nucleotide sequence, reference to an adenine, a thymine (uridine), a cytosine, or a guanine at a particular site on one strand of a nucleic acid molecule also defines the thymine (uridine), adenine, guanine, or cytosine (respectively) at the corresponding site on a complementary strand of the nucleic acid molecule. Thus, reference may be made to either strand in order to refer to a particular SNP position, SNP allele, or nucleotide sequence. Probes and primers, may be designed to hybridize to either strand and SNP genotyping methods disclosed herein may generally target either strand. Throughout the specification, in identifying a SNP position, reference is generally made to the protein-encoding strand, only for the purpose of convenience.

References to variant peptides, polypeptides, or proteins disclosed herein include peptides, polypeptides, proteins, or fragments thereof, that contain at least one amino acid residue that differs from the corresponding amino acid sequence of the art-known peptide/polypeptide/protein (the art-known protein may be interchangeably referred to as the "wild-type," "reference," or "normal" protein). Such variant peptides/polypeptides/proteins can result from a codon change caused by a nonsynonymous nucleotide substitution at a protein-coding SNP position (*i.e.*, a missense mutation) disclosed by the present invention. Variant peptides/ polypeptides/proteins disclosed herein can also result from a nonsense mutation (*i.e.,* a SNP that creates a premature stop codon, a SNP that generates a read-through mutation by abolishing a stop codon), or due to any SNP disclosed herein that otherwise alters the structure, function, activity, or expression of a protein, such as a SNP in a regulatory region (*e.g.* a promoter or enhancer) or a SNP that leads to alternative or defective splicing, such as a SNP in an intron or a SNP at an exon/intron boundary. As used herein, the terms "polypeptide," "peptide," and "protein" are used interchangeably.

As used herein, an "allele" may refer to a nucleotide at a SNP position (wherein at least two alternative nucleotides exist in the population at the SNP position, in accordance with the inherent definition of a SNP) or may refer to an amino acid residue that is encoded by the codon which contains the SNP position (where the alternative nucleotides that are present in the population at the SNP position form alternative codons that encode different amino acid residues). An "allele" may also be referred to herein as a "variant". Also, an amino acid residue that is encoded by a codon containing a particular SNP may simply be referred to as being encoded by the SNP.

A phrase such as "represented by", "as represented by", "as shown by", "as symbolized by", or "as designated by" may be used herein to refer to a SNP within a sequence (*e.g.,* a polynucleotide context sequence surrounding a SNP), such as in the context of "a polymorphism as represented by position 101 of SEQ ID NO:X or its complement". Typically, the sequence surrounding a SNP may be recited when referring to a SNP, however the sequence is not intended as a structural limitation beyond the specific SNP position itself. Rather, the sequence is recited merely as a way of referring to the SNP (in this example, "SEQ ID NO:X or its complement" is recited in order to refer to the SNP located at position 101 of SEQ ID NO:X, but SEQ ID NO:X or its complement is not intended as a structural limitation beyond the specific SNP position itself). In other words, it is recognized that the context sequence of SEQ ID NO:X in this example may contain one or more polymorphic nucleotide positions outside of position 101 and therefore an exact match over the full-length of SEQ ID NO:X is irrelevant since SEQ ID NO:X is only meant to provide context for referring to the SNP at position 101 of SEQ ID NO:X. Likewise, the length of the context sequence is also irrelevant (100 nucleotides on each side of a SNP position has been arbitrarily used in the present application as the length for context sequences merely for convenience and because 201 nucleotides of total length is expected to provide sufficient uniqueness to unambiguously identify a given nucleotide sequence). Thus, since a SNP is a variation at a single nucleotide position, it is customary to refer to context sequence (*e.g.,* SEQ ID NO:X in this example) surrounding a particular SNP position in order to uniquely identify and refer to the SNP. Alternatively, a SNP can be referred to by a unique identification number such as a public "rs" identification number or an internal "hCV" identification number, such as provided herein for each SNP (*e.g.,* in Tables 1-2). For example, in the instant application, "rs2036914", "hCV 12066124", and "position 101 of SEQ ID NO:713" all refer to the same SNP.

As used herein, the term "benefit" (with respect to a preventive or therapeutic drug treatment, such as statin treatment) is defined as achieving a reduced risk for a disease that the drug is intended to treat or prevent (*e.g.,* VT) by administrating the drug treatment, compared with the risk for the disease in the absence of receiving the drug treatment (or receiving a placebo in lieu of the drug treatment) for the same genotype. The term "benefit" may be used herein interchangeably with terms such as "respond positively" or "positively respond".

As used herein, the terms "drug" and "therapeutic agent" are used interchangeably, and may include, but are not limited to, small molecule compounds, biologies (*e.g.,* antibodies, proteins, protein fragments, fusion proteins, glycoproteins, etc.), nucleic acid agents (*e.g.,* antisense, RNAi/siRNA, and microRNA molecules, etc.), vaccines, etc., which may be used for therapeutic and/or preventive treatment of a disease (*e.g.,* VT).

Examples of statins (also known as HMG-CoA reductase inhibitors) include, but are not limited to, atorvastatin (Lipitor®), rosuvastatin (Crestor®), pravastatin (Pravachol®), simvastatin (Zocor®), fluvastatin (Lescol®), and lovastatin (Mevacor®), as well as combination therapies that include a statin such as simvastatin + ezetimibe (Vytorin®), lovastatin + niacin (Advicor®), atorvastatin + amlodipine besylate (Caduet®), and simvastatin + niacin (Simcor®).

Certain examples disclosed herein describe the following compositions and uses: (1) a reagent (such as an allele-specific probe or primer, or any other oligonucleotide or other reagent suitable for detecting a polymorphism disclosed herein, which can include detection of any allele of the polymorphism) for use as a diagnostic or predictive agent for determining VT risk and/or statin response, particularly for reducing the risk of VT; (2) a kit, device, array, or assay component that includes or is coupled with the reagent of (1) above for use in determining VT risk and/or statin response, particularly for reducing the risk of VT; (3) the use of the reagent of (1) above for the manufacture of a kit, device, array, or assay component for determining VT risk and/or statin response, particularly for reducing the risk of VT; and (4) the use of a polymorphism disclosed herein for the manufacture of a reagent for use as a diagnostic or predictive agent for determining VT risk and/or statin response, particularly for reducing the risk of VT.

The various methods described herein, such as correlating the presence or absence of a polymorphism with the predicted response of an individual to a drug such as a statin, particularly for reducing the risk for VT, and/or correlating the presence or absence of a polymorphism with an altered (*e.g.,* increased or decreased) risk (or no altered risk) for developing VT, can be carried out by automated methods such as by using a computer (or other apparatus/devices such as biomedical devices, laboratory instrumentation, or other apparatus/devices having a computer processor) programmed to carry out any of the methods described herein. For example, computer software (which may be interchangeably referred to herein as a computer program) can perform the step of correlating the presence or absence of a polymorphism in an individual with an altered (*e.g.,* increased or decreased) response (or no altered response) to statin treatment for reducing the risk for VT, and/or correlating the presence or absence of a polymorphism with an altered (*e.g.,* increased or decreased) risk (or no altered risk) for developing VT. Accordingly, certain embodiments of the invention provide a computer (or other apparatus/device) programmed to carry out any of the methods described herein.

Reagents, and kits containing the reagents, for detecting a SNP disclosed herein can be manufactured in compliance with regulatory requirements for clinical diagnostic use, such as those set forth by the United States Food and Drug Administration (FDA). Reagents and kits can be manufactured in compliance with "good manufacturing practice" (GMP) guidelines, such as "current good manufacturing practices" (cGMP) guidelines in the United States. Furthermore, reagents and kits can be registed with the FDA (such as by satisfying 510(k) Pre-Market Notification (PMN) requirements or obtaining Pre-Market Approval (PMA)). Reagents (particularly reagents for clinical diagnostic use) for detecting a SNP disclosed herein can be classified by the FDA (or other agency) as an analyte specific reagent (ASR) (or similar classification), and kits (particularly kits for clinical diagnostic use) containing reagents for detecting a SNP disclosed herein can be classified by the FDA (or other agency) as *in vitro* diagnostic (IVD) kits or laboratory developed tests (LDTs) (or similar classifications), including *in vitro* diagnostic multivariate index assays (IVDMIAs). Furthermore, reagents and kits can be classified by the FDA (or other agency) as Class I, Class II, or Class III medical devices. Reagents and kits can also be registered with (e.g., approved by) and/or manufactured in compliance with regulatory requirements set forth by the Clinical Laboratory Improvement Amendments Act (CLIA), which is administered by the Centers for Medicare and Medicaid Services (CMS), or other agencies in the United States or throughout the rest of the world.

*Reports, Programmed Computers, Business Methods, and Systems*

The results of a test (*e.g.,* an individual's predicted responsiveness to statin treatment, or an individual's risk for developing VT, based on assaying one or more SNPs disclosed herein, and/or an individual's allele(s)/genotype at one or more SNPs disclosed herein, etc.), and/or any other information pertaining to a test, may be referred to herein as a "report". A tangible report can optionally be generated as part of a testing process (which may be interchangeably referred to herein as "reporting", or as "providing" a report, "producing" a report, or "generating" a report).

Examples of tangible reports may include, but are not limited to, reports in paper (such as computer-generated printouts of test results) or equivalent formats and reports stored on computer readable medium (such as a CD, USB flash drive or other removable storage device, computer hard drive, or computer network server, etc.). Reports, particularly those stored on computer readable medium, can be part of a database, which may optionally be accessible via the internet (such as a database of patient records or genetic information stored on a computer network server, which may be a "secure database" that has security features that limit access to the report, such as to allow only the patient and the patient's medical practioners to view the report while preventing other unauthorized individuals from viewing the report, for example). In addition to, or as an alternative to, generating a tangible report, reports can also be displayed on a computer screen (or the display of another electronic device or instrument).

A report can include, for example, an individual's predicted risk for developing DVT and/or predicted responsiveness to statin treatment (e.g., whether the individual will benefit from statin treatment by having their risk for VT reduced), or may just include the allele(s)/genotype that an individual carries at one or more SNPs disclosed herein, which may optionally be linked to information regarding the significance of having the allele(s)/genotype at the SNP (for example, a report on computer readable medium such as a network server may include hyperlink(s) to one or more journal publications or websites that describe the medical/biological implications, such as statin response and/or VT risk, for individuals having a certain allele/genotype at the SNP). Thus, for example, the report can include drug responsiveness, disease risk, and/or other medical/biological significance, as well as optionally also including the allele/genotype information, or the report may just include allele/genotype information without including drug responsiveness, disease risk, or other medical/biological significance (such that an individual viewing the report can use the allele/genotype information to determine the associated drug response, disease risk, or other medical/biological significance from a source outside of the report itself, such as from a medical practioner, publication, website, etc., which may optionally be linked to the report such as by a hyperlink).

A report can further be "transmitted" or "communicated" (these terms may be used herein interchangeably), such as to the individual who was tested, a medical practitioner (*e.g.,* a doctor, nurse, clinical laboratory practitioner, genetic counselor, etc.), a healthcare organization, a clinical laboratory, and/or any other party or requester intended to view or possess the report. The act of "transmitting" or "communicating" a report can be by any means known in the art, based on the format of the report. Furthermore, "transmitting" or "communicating" a report can include delivering/sending a report ("pushing") and/or retrieving ("pulling") a report. For example, reports can be transmitted/communicated by various means, including being physically transferred between parties (such as for reports in paper format) such as by being physically delivered from one party to another, or by being transmitted electronically (*e.g.,* via e-mail or over the internet, by facsimile, and/or by any wired or wireless communication methods known in the art) such as by being retrieved from a database stored on a computer network server, etc.

In certain exemplary embodiments, the method of the invention is carried out by computers (or other apparatus/devices such as biomedical devices or laboratory instrumentation) programmed to carry out the methods of the invention. For example, in certain embodiments, the computer is programmed to receive (*i.e*., as input) the identity (*e.g.,* the allele(s) or genotype at a SNP) of the SNP of the invention and provide (*i.e*., as output) the correlation between the presence of the respective allele with a reduction of the risk for VT by an HMG-CoA reductase inhibitor. Such output (*e.g.,* communication of disease risk, disease diagnosis or prognosis, drug responsiveness, *etc.*) may be, for example, in the form of a report on computer readable medium, printed in paper form, and/or displayed on a computer screen or other display.

In various examples, disclosed herein are methods of doing business (with respect to methods of doing business, the terms "individual" and "customer" are used herein interchangeably). For example, exemplary methods of doing business can comprise assaying one or more SNPs disclosed herein and providing a report that includes, for example, a customer's predicted response to statin treatment (e.g., for reducing their risk for VT) or their risk for developing VT (based on which allele(s)/genotype is present at the assayed SNP(s)) and/or that includes the allele(s)/genotype at the assayed SNP(s) which may optionally be linked to information (*e.g.,* journal publications, websites, *etc*.) pertaining to disease risk or other biological/medical significance such as by means of a hyperlink (the report may be provided, for example, on a computer network server or other computer readable medium that is internet-accessible, and the report may be included in a secure database that allows the customer to access their report while preventing other unauthorized individuals from viewing the report), and optionally transmitting the report. Customers (or another party who is associated with the customer, such as the customer's doctor, for example) can request/order (*e.g.,* purchase) the test online via the internet (or by phone, mail order, at an outlet/store, *etc*.), for example, and a kit can be sent/delivered (or otherwise provided) to the customer (or another party on behalf of the customer, such as the customer's doctor, for example) for collection of a biological sample from the customer (*e.g.,* a buccal swab for collecting buccal cells), and the customer (or a party who collects the customer's biological sample) can submit their biological samples for assaying (*e.g.,* to a laboratory or party associated with the laboratory such as a party that accepts the customer samples on behalf of the laboratory, a party for whom the laboratory is under the control of (*e.g.,* the laboratory carries out the assays by request of the party or under a contract with the party, for example), and/or a party that receives at least a portion of the customer's payment for the test). The report (*e.g.,* results of the assay including, for example, the customer's disease risk and/or allele(s)/genotype at the assayed SNP(s)) may be provided to the customer by, for example, the laboratory that assays the SNP(s) or a party associated with the laboratory (*e.g.,* a party that receives at least a portion of the customer's payment for the assay, or a party that requests the laboratory to carry out the assays or that contracts with the laboratory for the assays to be carried out) or a doctor or other medical practitioner who is associated with (*e.g.,* employed by or having a consulting or contracting arrangement with) the laboratory or with a party associated with the laboratory, or the report may be provided to a third party (*e.g.,* a doctor, genetic counselor, hospital, *etc.*) which optionally provides the report to the customer. In further examples, the customer may be a doctor or other medical practitioner, or a hospital, laboratory, medical insurance organization, or other medical organization that requests/orders (*e.g.,* purchases) tests for the purposes of having other individuals (*e.g.,* their patients or customers) assayed for one or more SNPs disclosed herein and optionally obtaining a report of the assay results.

In certain exemplary methods of doing business, a kit for collecting a biological sample (*e.g.,* a buccal swab for collecting buccal cells, or other sample collection device) is provided to a medical practitioner (e.g., a physician) which the medical practitioner uses to obtain a sample (e.g., buccal cells, saliva, blood, etc.) from a patient, the sample is then sent to a laboratory (e.g., a CLIA-certified laboratory) or other facility that tests the sample for one or more SNPs disclosed herein (e.g., to determine the genotype of one or more SNPs disclosed herein, such as to determine the patient's predicted response to statin treatment for reducing their risk for VT, and/or their risk for developing VT), and the results of the test (e.g., the patient's genotype at one or more SNPs disclosed herein and/or the patient's predicted statin response or VT risk based on their SNP genotype) are provided back to the medical practitioner (and/or directly to the patient and/or to another party such as a hospital, medical insurance company, genetic counselor, *etc*.) who may then provide or otherwise convey the results to the patient. The results are typically provided in the form of a report, such as described above.

In certain further exemplary methods of doing business, kits for collecting a biological sample from a customer (*e.g.,* a buccal swab for collecting buccal cells, or other sample collection device) are provided (*e.g.,* for sale), such as at an outlet (*e.g.,* a drug store, pharmacy, general merchandise store, or any other desirable outlet), online via the internet, by mail order, *etc*., whereby customers can obtain (*e.g.,* purchase) the kits, collect their own biological samples, and submit (*e.g.,* send/deliver via mail) their samples to a laboratory (e.g., a CLIA-certified laboratory) or other facility which tests the samples for one or more SNPs disclosed herein (e.g., to determine the genotype of one or more SNPs disclosed herein, such as to determine the customer's predicted response to statin treatment for reducing their risk for VT, and/or their risk for developing VT) and provides the results of the test (e.g., of the customer's genotype at one or more SNPs disclosed herein and/or the customer's statin response or VT risk based on their SNP genotype) back to the customer and/or to a third party (*e.g.,* a physician or other medical practitioner, hospital, medical insurance company, genetic counselor, *etc.*). The results are typically provided in the form of a report, such as described above. If the results of the test are provided to a third party, then this third party may optionally provide another report to the customer based on the results of the test (e.g., the result of the test from the laboratory may provide the customer's genotype at one or more SNPs disclosed herein without statin response or VT risk information, and the third party may provide a report of the customer's statin response or VT risk based on this genotype result).

The invention provides a method for determining whether an individual will benefit from statin treatment in reducing VT risk, or for determining an individual's risk for developing VT based on the presence of SNP (rs2036914). Certain exemplary systems comprise an integrated "loop" in which an individual (or their medical practitioner) requests a determination of such individual's predicted statin response (or VT risk, etc.), this determination is carried out by testing a sample from the individual, and then the results of this determination are provided back to the requestor. For example, in certain systems, a sample (e.g., buccal cells, saliva, blood, etc.) is obtained from an individual for testing (the sample may be obtained by the individual or, for example, by a medical practitioner), the sample is submitted to a laboratory (or other facility) for testing (e.g., determining the genotype of one or more SNPs disclosed herein), and then the results of the testing are sent to the patient (which optionally can be done by first sending the results to an intermediary, such as a medical practioner, who then provides or otherwise conveys the results to the individual and/or acts on the results), thereby forming an integrated loop system for determining an individual's predicted statin response (or VT risk, etc.). The portions of the system in which the results are transmitted (e.g., between any of a testing facility, a medical practitioner, and/or the individual) can be carried out by way of electronic transmission (e.g., by computer such as via e-mail or the internet, by providing the results on a website or computer network server which may optionally be a secure database, by phone or fax, or by any other wired or wireless transmission methods known in the art). Optionally, the system can further include a risk reduction component (i.e., a disease management system) as part of the integrated loop (for an example of a disease management system, see U.S. patent no. 6,770,029, "Disease management system and method including correlation assessment"). For example, the results of the test can be used to reduce the risk of the disease in the individual who was tested, such as by implementing a preventive therapy regimen (e.g., administration of a statin or other drug for reducing VT risk), modifying the individual's diet, increasing exercise, reducing stress, and/or implementing any other physiological or behavioral modifications in the individual with the goal of reducing disease risk. For reducing VT risk, this may include any means used in the art for improving aspects of an individual's health relevant to reducing VT risk. Thus, in some examples, the system is controlled by the individual and/or their medical practioner in that the individual and/or their medical practioner requests the test, receives the test results back, and (optionally) acts on the test results to reduce the individual's disease risk, such as by implementing a disease management system.

## ISOLATED NUCLEIC ACID MOLECULES AND SNP DETECTION REAGENTS & KITS

Tables 1 and 2 provide a variety of information about each SNP disclosed herein, including the SNP of the present invention that is associated with risk for developing VT and/or response to statin treatment (particularly for reducing an individual's risk for VT), including the transcript sequences (SEQ ID NOS:1-84), genomic sequences (SEQ ID NOS:338-500), and protein sequences (SEQ ID NOS:85-168) of the encoded gene products (with the SNPs indicated by IUB codes in the nucleic acid sequences). In addition, Tables 1 and 2 include SNP context sequences, which generally include 100 nucleotide upstream (5') plus 100 nucleotides downstream (3') of each SNP position (SEQ ID NOS: 169-337 correspond to transcript-based SNP context sequences disclosed in Table 1, and SEQ ID NOS:501-3098 correspond to genomic-based context sequences disclosed in Table 2), the alternative nucleotides (alleles) at each SNP position, and additional information about the variant where relevant, such as SNP type (coding, missense, splice site, UTR, etc.), human populations in which the SNP was observed, observed allele frequencies, information about the encoded protein, etc.

Isolated Nucleic Acid Molecules Disclosed herein are isolated nucleic acid molecules that contain one or more SNPs disclosed herein, particularly SNPs disclosed in Table 1 and/or Table 2. Isolated nucleic acid molecules containing one or more SNPs disclosed herein (such as in at least one of Tables 1 and 2) may be interchangeably referred to throughout the present text as "SNP-containing nucleic acid molecules." Isolated nucleic acid molecules may optionally encode a full-length variant protein or fragment thereof. The isolated nucleic acid molecules disclosed herein also include probes and primers (which are described in greater detail below in the section entitled "SNP Detection Reagents"), which may be used for assaying the disclosed SNPs, and isolated full-length genes, transcripts, cDNA molecules, and fragments thereof, which may be used for such purposes as expressing an encoded protein.

As used herein, an "isolated nucleic acid molecule" generally is one that contains a SNP of the present invention or one that hybridizes to such molecule such as a nucleic acid with a complementary sequence, and is separated from most other nucleic acids present in the natural source of the nucleic acid molecule. Moreover, an "isolated" nucleic acid molecule, such as a cDNA molecule containing a SNP of the present invention, can be substantially free of other cellular material, or culture medium when produced by recombinant techniques, or chemical precursors or other chemicals when chemically synthesized. A nucleic acid molecule can be fused to other coding or regulatory sequences and still be considered "isolated." Nucleic acid molecules present in non-human transgenic animals, which do not naturally occur in the animal, are also considered "isolated." For example, recombinant DNA molecules contained in a vector are considered "isolated." Further examples of "isolated" DNA molecules include recombinant DNA molecules maintained in heterologous host cells, and purified (partially or substantially) DNA molecules in solution. Isolated RNA molecules include in vivo or in vitro RNA transcripts of the isolated SNP-containing DNA molecules of the present invention. Isolated nucleic acid molecules according to the present disclosure further include such molecules produced synthetically.

Generally, an isolated SNP-containing nucleic acid molecule comprises one or more SNP positions with flanking nucleotide sequences on either side of the SNP positions. A flanking sequence can include nucleotide residues that are naturally associated with the SNP site and/or heterologous nucleotide sequences. Preferably, the flanking sequence is up to about 500, 300, 100, 60, 50, 30, 25, 20, 15, 10, 8, or 4 nucleotides (or any other length in-between) on either side of a SNP position, or as long as the full-length gene or entire protein-coding sequence (or any portion thereof such as an exon), especially if the SNP-containing nucleic acid molecule is to be used to produce a protein or protein fragment.

For full-length genes and entire protein-coding sequences, a SNP flanking sequence can be, for example, up to about 5KB, 4KB, 3KB, 2KB, 1KB on either side of the SNP. Furthermore, in such instances the isolated nucleic acid molecule comprises exonic sequences (including protein-coding and/or non-coding exonic sequences), but may also include intronic sequences. Thus, any protein coding sequence may be either contiguous or separated by introns. The important point is that the nucleic acid is isolated from remote and unimportant flanking sequences and is of appropriate length such that it can be subjected to the specific manipulations or uses described herein such as recombinant protein expression, preparation of probes and primers for assaying the SNP position, and other uses specific to the SNP-containing nucleic acid sequences.

An isolated SNP-containing nucleic acid molecule can comprise, for example, a full-length gene or transcript, such as a gene isolated from genomic DNA (*e.g.,* by cloning or PCR amplification), a cDNA molecule, or an mRNA transcript molecule. Polymorphic transcript sequences are referred to in Table 1 and provided in the Sequence Listing (SEQ ID NOS:1-84), and polymorphic genomic sequences are referred to in Table 2 and provided in the Sequence Listing (SEQ ID NOS:338-500). Furthermore, fragments of such full-length genes and transcripts that contain one or more SNPs disclosed herein are also encompassed by the present invention, and such fragments may be used, for example, to express any part of a protein, such as a particular functional domain or an antigenic epitope.

Thus, the present disclosure also encompasses fragments of the nucleic acid sequences as disclosed in Tables 1 and 2 (transcript sequences are referred to in Table 1 as SEQ ID NOS: 1-84, genomic sequences are referred to in Table 2 as SEQ ID NOS:338-500, transcript-based SNP context sequences are referred to in Table 1 as SEQ ID NOS:169-337, and genomic-based SNP context sequences are referred to in Table 2 as SEQ ID NOS:501-3098) and their complements. The actual sequences referred to in the tables are provided in the Sequence Listing. A fragment typically comprises a contiguous nucleotide sequence at least about 8 or more nucleotides, more preferably at least about 12 or more nucleotides, and even more preferably at least about 16 or more nucleotides. Furthermore, a fragment could comprise at least about 18, 20, 22, 25, 30, 40, 50, 60, 80, 100, 150, 200, 250 or 500 nucleotides in length (or any other number in between). The length of the fragment will be based on its intended use. For example, the fragment can encode epitope-bearing regions of a variant peptide or regions of a variant peptide that differ from the normal/wild-type protein, or can be useful as a polynucleotide probe or primer. Such fragments can be isolated using the nucleotide sequences provided in Table 1 and/or Table 2 for the synthesis of a polynucleotide probe. A labeled probe can then be used, for example, to screen a cDNA library, genomic DNA library, or mRNA to isolate nucleic acid corresponding to the coding region. Further, primers can be used in amplification reactions, such as for purposes of assaying one or more SNPs sites or for cloning specific regions of a gene.

An isolated nucleic acid molecule of the present disclosure further encompasses a SNP-containing polynucleotide that is the product of any one of a variety of nucleic acid amplification methods, which are used to increase the copy numbers of a polynucleotide of interest in a nucleic acid sample. Such amplification methods are well known in the art, and they include but are not limited to, polymerase chain reaction (PCR) (U.S. Patent Nos. 4,683,195 and 4,683,202; PCR Technology: Principles and Applications for DNA Amplification, ed. H.A. Erlich, Freeman Press, NY, NY (1992)), ligase chain reaction (LCR) (Wu and Wallace, Genomics 4:560 (1989); Landegren et al., Science 241:1077 (1988)), strand displacement amplification (SDA) (U.S. Patent Nos. 5,270,184 and 5,422,252), transcription-mediated amplification (TMA) (U.S. Patent No. 5,399,491), linked linear amplification (LLA) (U.S. Patent No. 6,027,923) and the like, and isothermal amplification methods such as nucleic acid sequence based amplification (NASBA) and self-sustained sequence replication (Guatelli et al., Proc Natl Acad Sci USA 87:1874 (1990)). Based on such methodologies, a person skilled in the art can readily design primers in any suitable regions 5' and 3' to a SNP disclosed herein. Such primers may be used to amplify DNA of any length so long that it contains the SNP of interest in its sequence.

As used herein, an "amplified polynucleotide" of the invention is a SNP-containing nucleic acid molecule whose amount has been increased at least two fold by any nucleic acid amplification method performed *in vitro* as compared to its starting amount in a test sample. In other examples, an amplified polynucleotide is the result of at least ten fold, fifty fold, one hundred fold, one thousand fold, or even ten thousand fold increase as compared to its starting amount in a test sample. In a typical PCR amplification, a polynucleotide of interest is often amplified at least fifty thousand fold in amount over the unamplified genomic DNA, but the precise amount of amplification needed for an assay depends on the sensitivity of the subsequent detection method used.

Generally, an amplified polynucleotide is at least about 16 nucleotides in length. More typically, an amplified polynucleotide is at least about 20 nucleotides in length. In one example, an amplified polynucleotide is at least about 30 nucleotides in length. In another example, an amplified polynucleotide is at least about 32, 40, 45, 50, or 60 nucleotides in length. In yet another example, an amplified polynucleotide is at least about 100, 200, 300, 400, or 500 nucleotides in length. While the total length of an amplified polynucleotide can be as long as an exon, an intron or the entire gene where the SNP of interest resides, an amplified product is typically up to about 1,000 nucleotides in length (although certain amplification methods may generate amplified products greater than 1000 nucleotides in length). More preferably, an amplified polynucleotide is not greater than about 600-700 nucleotides in length. It is understood that irrespective of the length of an amplified polynucleotide, a SNP of interest may be located anywhere along its sequence,

In a specific example, the amplified product is at least about 201 nucleotides in length, comprises one of the transcript-based context sequences or the genomic-based context sequences shown in Tables 1 and 2. Such a product may have additional sequences on its 5' end or 3' end or both. In another example, the amplified product is about 101 nucleotides in length, and it contains a SNP disclosed herein. Preferably, the SNP is located at the middle of the amplified product (*e.g.*, at position 101 in an amplified product that is 201 nucleotides in length, or at position 51 in an amplified product that is 101 nucleotides in length), or within 1, 2, 3, 4, 5, 6, 7, 8, 9, 10, 12, 15, or 20 nucleotides from the middle of the amplified product. However, as indicated above, the SNP of interest may be located anywhere along the length of the amplified product.

Disclosed herein are isolated nucleic acid molecules that comprise, consist of, or consist essentially of one or more polynucleotide sequences that contain one or more SNPs disclosed herein, complements thereof, and SNP-containing fragments thereof.

Accordingly, disclosed herein are nucleic acid molecules that consist of any of the nucleotide sequences shown in Table 1 and/or Table 2 (transcript sequences are referred to in Table 1 as SEQ ID NOS:1-84, genomic sequences are referred to in Table 2 as SEQ ID NOS:338-500, transcript-based SNP context sequences are referred to in Table 1 as SEQ ID NOS: 169-337, and genomic-based SNP context sequences are referred to in Table 2 as SEQ ID NOS:501-3098), or any nucleic acid molecule that encodes any of the variant proteins referred to in Table 1 (SEQ ID NOS:85-168). The actual sequences referred to in the tables are provided in the Sequence Listing. A nucleic acid molecule consists of a nucleotide sequence when the nucleotide sequence is the complete nucleotide sequence of the nucleic acid molecule.

Disclosed herein are also nucleic acid molecules that consist essentially of any of the nucleotide sequences referred to in Table 1 and/or Table 2 (transcript sequences are referred to in Table 1 as SEQ ID NOS:1-84, genomic sequences are referred to in Table 2 as SEQ ID NOS:338-500, transcript-based SNP context sequences are referred to in Table 1 as SEQ ID

NOS:169-337, and genomic-based SNP context sequences are referred to in Table 2 as SEQ ID NOS:501-3098), or any nucleic acid molecule that encodes any of the variant proteins referred to in Table 1 (SEQ ID NOS:85-168). The actual sequences referred to in the tables are provided in the Sequence Listing. A nucleic acid molecule consists essentially of a nucleotide sequence when such a nucleotide sequence is present with only a few additional nucleotide residues in the final nucleic acid molecule.

Further, disclosed herein are nucleic acid molecules that comprise any of the nucleotide sequences shown in Table 1 and/or Table 2 or a SNP-containing fragment thereof (transcript sequences are referred to in Table 1 as SEQ ID NOS:1-84, genomic sequences are referred to in Table 2 as SEQ ID NOS:338-500, transcript-based SNP context sequences are referred to in Table 1 as SEQ ID NOS:169-337, and genomic-based SNP context sequences are referred to in Table 2 as SEQ ID NOS:501-3098), or any nucleic acid molecule that encodes any of the variant proteins provided in Table 1 (SEQ ID NOS:85-168). The actual sequences referred to in the tables are provided in the Sequence Listing. A nucleic acid molecule comprises a nucleotide sequence when the nucleotide sequence is at least part of the final nucleotide sequence of the nucleic acid molecule. In such a fashion, the nucleic acid molecule can be only the nucleotide sequence or have additional nucleotide residues, such as residues that are naturally associated with it or heterologous nucleotide sequences. Such a nucleic acid molecule can have one to a few additional nucleotides or can comprise many more additional nucleotides. A brief description of how various types of these nucleic acid molecules can be readily made and isolated is provided below, and such techniques are well known to those of ordinary skill in the art. Sambrook and Russell, Molecular Clotting: A Laboratory Manual, Cold Spring Harbor Press, N.Y. (2000).

The isolated nucleic acid molecules can encode mature proteins plus additional amino or carboxyl-terminal amino acids or both, or amino acids interior to the mature peptide (when the mature form has more than one peptide chain, for instance). Such sequences may play a role in processing of a protein from precursor to a mature form, facilitate protein trafficking, prolong or shorten protein half-life, or facilitate manipulation of a protein for assay or production. As generally is the case *in situ,* the additional amino acids may be processed away from the mature protein by cellular enzymes.

Thus, the isolated nucleic acid molecules include, but are not limited to, nucleic acid molecules having a sequence encoding a peptide alone, a sequence encoding a mature peptide and additional coding sequences such as a leader or secretory sequence (*e.g.,* a pre-pro or pro-protein sequence), a sequence encoding a mature peptide with or without additional coding sequences, plus additional non-coding sequences, for example introns and non-coding 5' and 3' sequences such as transcribed but untranslated sequences that play a role in, for example, transcription, mRNA processing (including splicing and polyadenylation signals), ribosome binding, and/or stability of mRNA. In addition, the nucleic acid molecules may be fused to heterologous marker sequences encoding, for example, a peptide that facilitates purification.

Isolated nucleic acid molecules can be in the form of RNA, such as mRNA, or in the form DNA, including cDNA and genomic DNA, which may be obtained, for example, by molecular cloning or produced by chemical synthetic techniques or by a combination thereof. Sambrook and Russell, Molecular Cloning: A Laboratory Manual, Cold Spring Harbor Press, N.Y. (2000). Furthermore, isolated nucleic acid molecules, particularly SNP detection reagents such as probes and primers, can also be partially or completely in the form of one or more types of nucleic acid analogs, such as peptide nucleic acid (PNA). U.S. Patent Nos. 5,539,082; 5,527,675; 5,623,049; and 5,714,331. The nucleic acid, especially DNA, can be double-stranded or single-stranded. Single-stranded nucleic acid can be the coding strand (sense strand) or the complementary non-coding strand (anti-sense strand). DNA, RNA, or PNA segments can be assembled, for example, from fragments of the human genome (in the case of DNA or RNA) or single nucleotides, short oligonucleotide linkers, or from a series of oligonucleotides, to provide a synthetic nucleic acid molecule. Nucleic acid molecules can be readily synthesized using the sequences provided herein as a reference; oligonucleotide and PNA oligomer synthesis techniques are well known in the art. See, *e.g.*, Corey, "Peptide nucleic acids: expanding the scope of nucleic acid recognition," Trends Biotechnol 15(6):224-9 (Jun. 1997), and Hyrup et al., "Peptide nucleic acids (PNA): synthesis, properties and potential applications," Bioorg Med Chem 4(1):5-23 (Jan. 1996). Furthermore, large-scale automated oligonucleotide/PNA synthesis (including synthesis on an array or bead surface or other solid support) can readily be accomplished using commercially available nucleic acid synthesizers, such as the Applied Biosystems (Foster City, CA) 3900 High-Throughput DNA Synthesizer or Expedite 8909 Nucleic Acid Synthesis System, and the sequence information provided herein.

Disclosed herein are nucleic acid analogs that contain modified, synthetic, or non-naturally occurring nucleotides or structural elements or other alternative/modified nucleic acid chemistries known in the art. Such nucleic acid analogs are useful, for example, as detection reagents (*e.g.*, primers/probes) for detecting one or more SNPs identified in Table 1 and/or Table 2. Furthermore, the use of kits/systems (such as beads, arrays, etc.) that include these analogs in the method of the invention are also encompassed by the present invention. For example, PNA oligomers that are based on the polymorphic sequences of the present invention are specifically contemplated. PNA oligomers are analogs of DNA in which the phosphate backbone is replaced with a peptide-like backbone. Lagriffoul et al., Bioorganic & Medicinal Chemistry Letters 4:1081-1082 (1994); Petersen et al., Bioorganic & Medicinal Chemistry Letters 6:793-796 (1996); Kumar et al., Organic Letters 3(9):1269-1272 (2001); WO 96/04000. PNA hybridizes to complementary RNA or DNA with higher affinity and specificity than conventional oligonucleotides and oligonucleotide analogs. The properties of PNA enable novel molecular biology and biochemistry applications unachievable with traditional oligonucleotides and peptides.

Additional examples of nucleic acid modifications that improve the binding properties and/or stability of a nucleic acid include the use of base analogs such as inosine, intercalalors (U.S. Patent No. 4,835,263) and the minor groove binders (U.S. Patent No. 5,801,115). Thus, references are made herein to nucleic acid molecules, SNP-containing nucleic acid molecules, SNP detection reagents (*e.g.,* probes and primers), oligonucleotides/polynucleotides include PNA oligomers and other nucleic acid analogs. Other examples of nucleic acid analogs and alternative/modified nucleic acid chemistries known in the art are described in Current Protocols in Nucleic Acid Chemistry, John Wiley & Sons, N.Y. (2002).

Disclosed herein are also nucleic acid molecules that encode fragments of the variant polypeptides disclosed herein as well as nucleic acid molecules that encode obvious variants of such variant polypeptides. Such nucleic acid molecules may be naturally occurring, such as paralogs (different locus) and orthologs (different organism), or may be constructed by recombinant DNA methods or by chemical synthesis. Non-naturally occurring variants may be made by mutagenesis techniques, including those applied to nucleic acid molecules, cells, or organisms. Accordingly, the variants can contain nucleotide substitutions, deletions, inversions and insertions (in addition to the SNPs disclosed in Tables 1 and 2). Variation can occur in either or both the coding and non-coding regions. The variations can produce conservative and/or non-conservative amino acid substitutions.

Further variants of the nucleic acid molecules disclosed in Tables 1 and 2, such as naturally occurring allelic variants (as well as orthologs and paralogs) and synthetic variants produced by mutagenesis techniques, can be identified and/or produced using methods well known in the art. Such further variants can comprise a nucleotide sequence that shares at least 70-80%, 80-85%, 85-90%, 91%, 92%, 93%, 94%, 95%, 96%, 97%, 98%, or 99% sequence identity with a nucleic acid sequence disclosed in Table 1 and/or Table 2 (or a fragment thereof) and that includes a novel SNP allele disclosed in Table 1 and/or Table 2. Further, variants can comprise a nucleotide sequence that encodes a polypeptide that shares at least 70-80%, 80-85%, 85-90%, 91%, 92%, 93%, 94%, 95%, 96%, 97%, 98%, or 99% sequence identity with a polypeptide sequence disclosed in Table 1 (or a fragment thereof) and that includes a novel SNP allele disclosed in Table 1 and/or Table 2. Thus, the present disclosure contemplates isolated nucleic acid molecules that have a certain degree of sequence variation compared with the sequences shown in Tables 1-2, but that contain a novel SNP allele disclosed herein. In other words, as long as an isolated nucleic acid molecule contains a novel SNP allele disclosed herein, other portions of the nucleic acid molecule that flank the novel SNP allele can vary to some degree from the specific transcript, genomic, and context sequences referred to and shown in Tables 1 and 2, and can encode a polypeptide that varies to some degree from the specific polypeptide sequences referred to in Table 1.

To determine the percent identity of two amino acid sequences or two nucleotide sequences of two molecules that share sequence homology, the sequences are aligned for optimal comparison purposes (*e.g.,* gaps can be introduced in one or both of a first and a second amino acid or nucleic acid sequence for optimal alignment and non-homologous sequences can be disregarded for comparison purposes). In a preferred example, at least 30%, 40%, 50%, 60%, 70%, 80%, or 90% or more of the length of a reference sequence is aligned for comparison purposes. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein, amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. Computational Molecular Biology, A.M. Lesk, ed., Oxford University Press, N.Y (1988); Biocomputing: Informatics and Genome Projects, D.W. Smith, ed., Academic Press, N.Y. (1993); Computer Antilysis of Sequence Data, Part 1, A.M. Griffin and H.G. Griffin, eds., Humana Press, N.J. (1994); Sequence Analysis in Molecular Biology, G. von Heinje, ed., Academic Press, N.Y. (1987); and Sequence Analysis Primer, M. Gribskov and J. Devereux, eds., M. Stockton Press, N.Y. (1991). In a preferred example, the percent identity between two amino acid sequences is determined using the Needleman and Wunsch algorithm (J Mol Biol (48):444-453 (1970)) which has been incorporated into the GAP program in the GCG software package, using either a Blossom 62 matrix or a PAM250 matrix, and a gap weight of 16, 14, 12, 10, 8, 6, or 4 and a length weight of 1, 2, 3, 4, 5, or 6.

In yet another preferred example, the percent identity between two nucleotide sequences is determined using the GAP program in the GCG software package using a NWSgapdna.CMP matrix and a gap weight of 40, 50, 60, 70, or 80 and a length weight of 1, 2, 3, 4, 5, or 6. J. Devereux et al., Nucleic Acids Res. 12(1):387 (1984). In another example, the percent identity between two amino acid or nucleotide sequences is determined using the algorithm of E. Myers and W. Miller (CABIOS 4:11-17 (1989)) which has been incorporated into the ALIGN program (version 2.0), using a PAM120 weight residue table, a gap length penalty of 12, and a gap penalty of 4.

The nucleotide and amino acid sequences can further be used as a "query sequence" to perform a search against sequence databases; for example, to identify other family members or related sequences. Such searches can be performed using the NBLAST and XBLAST programs (version 2.0). Altschul et al., J Mol Biol 215:403-10 (1990). BLAST nucleotide searches can be perforated with the NBLAST program, score = 100, wordlength = 12 to obtain nucleotide sequences homologous to the nucleic acid molecules of the invention. BLAST protein searches can be performed with the XBLAST program, score = 50, wordlength = 3 to obtain amino acid sequences homologous to the proteins of the invention. To obtain gapped alignments for comparison purposes, Gapped BLAST can be utilized. Altschul et al., Nucleic Acids Res 25(17):3389-3402 (1997). When utilizing BLAST and gapped BLAST programs, the default parameters of the respective programs (*e.g.*, XBLAST and NBLAST) can be used. In addition to BLAST, examples of other search and sequence comparison programs used in the art include, but are not limited to, FASTA (Pearson, Methods Mot Biol 25, 365-389 (1994)) and KERR (Dufresne et al., Nat Biotechnol 20(12): 1269-71 (Dec. 2002)). For further information regarding bioinformatics techniques, see Current Protocols in Bioinformatics, John Wiley & Sons, Inc., N.Y.

Disclosed herein are also non-coding fragments of the nucleic acid molecules disclosed in Table 1 and/or Table 2. Preferred non-coding fragments include, but are not limited to, promoter sequences, enhancer sequences, intronic sequences, 5' untranslated regions (UTRs), 3' untranslated regions, gene modulating sequences and gene termination sequences. Such fragments are useful, for example, in controlling heterologous gene expression and in developing screens to identify gene-modulating agents.

SNP Detection Reagents

In a specific example, the SNPs disclosed in Table 1 and/or Table 2, and their associated transcript sequences (referred to in Table 1 as SEQ ID NOS: 1-84), genomic sequences (referred to in Table 2 as SEQ ID NOS:338-500), and context sequences (transcript-based context sequences are referred to in Table 1 as SEQ ID NOS: 169-337; genomic-based context sequences are provided in Table 2 as SEQ ID NOS:501-3098), can be used for the design of SNP detection reagents. The actual sequences referred to in the tables are provided in the Sequence Listing. As used herein, a "SNP detection reagent" is a reagent that specifically detects a specific target SNP position disclosed herein, and that is preferably specific for a particular nucleotide (allele) of the target SNP position (*i.e.*, the detection reagent preferably can differentiate between different alternative nucleotides at a target SNP position, thereby allowing the identity of the nucleotide present at the target SNP position to be determined). Typically, such detection reagent hybridizes to a target SNP-containing nucleic acid molecule by complementary base-pairing in a sequence specific manner, and discriminates the target variant sequence from other nucleic acid sequences such as an art-known form in a test sample. An example of a detection reagent is a probe that hybridizes to a target nucleic acid containing one or more of the SNPs referred to in Table 1 and/or Table 2. In a preferred example, such a probe can differentiate between nucleic acids having a particular nucleotide (allele) at a target SNP position from other nucleic acids that have a different nucleotide at the same target SNP position. In addition, a detection reagent may hybridize to a specific region 5' and/or 3' to a SNP position, particularly a region corresponding to the context sequences referred to in Table 1 and/or Table 2 (transcript-based context sequences are referred to in Table 1 as SEQ ID NOS: 169-337; genomic-based context sequences are referred to in Table 2 as SEQ ID NOS:501-3098). Another example of a detection reagent is a primer that acts as an initiation point of nucleotide extension along a complementary strand of a target polynucleotide. The SNP sequence information provided herein is also useful for designing primers, *e.g.* allele-specific primers, to amplify (*e.g.*, using PCR) the SNP of the present invention.

In one preferred embodiment of the invention, a SNP detection reagent is an isolated or synthetic DNA or RNA polynucleotide probe or primer or PNA oligomer, or a combination of DNA, RNA and/or PNA, that hybridizes to a segment of a target nucleic acid molecule containing a SNP identified in Table 1 and/or Table 2. A detection reagent in the form of a polynucleotide may optionally contain modified base analogs, intercalators or minor groove binders. Multiple detection reagents such as probes may be, for example, affixed to a solid support (*e.g.,* arrays or beads) or supplied in solution (*e.g.* probe/primer sets for enzymatic reactions such as PCR, RT-PCR, TaqMan assays, or primer-extension reactions) to form a SNP detection kit.

A probe or primer typically is a substantially purified oligonucleotide or PNA oligomer. Such oligonucleotide typically comprises a region of complementary nucleotide sequence that hybridizes under stringent conditions to at least about 8, 10, 12, 16, 18, 20, 22, 25, 30, 40, 50, 55, 60, 65, 70, 80, 90, 100, 120 (or any other number in-between) or more consecutive nucleotides in a target nucleic acid molecule. Depending on the particular assay, the consecutive nucleotides can either include the target SNP position, or be a specific region in close enough proximity 5' and/or 3' to the SNP position to carry out the desired assay.

Other preferred primer and probe sequences can readily be determined using the transcript sequences (SEQ ID NOS:1-84), genomic sequences (SEQ ID NOS:338-500), and SNP context sequences (transcript-based context sequences are referred to in Table 1 as SEQ ID NOS:169-337: genomic-based context sequences are referred to in Table 2 as SEQ ID NOS:501-3098) disclosed in the Sequence Listing and in Tables 1 and 2. The actual sequences referred to in the tables are provided in the Sequence Listing. It will be apparent to one of skill in the art that such primers and probes are directly useful as reagents for genotyping the SNPs of the present invention, and can be incorporated into any kit/system format.

In order to produce a probe or primer specific for a target SNP-containing sequence, the gene/transcript and/or context sequence surrounding the SNP of interest is typically examined using a computer algorithm that starts at the 5' or at the 3' end of the nucleotide sequence. Typical algorithms will then identify oligomers of defined length that are unique to the gene/SNP context sequence, have a GC content within a range suitable for hybridization, lack predicted secondary structure that may interfere with hybridization, and/or possess other desired characteristics or that lack other undesired characteristics,

A primer or probe used in the present invention is typically at least about 8 nucleotides in length. In one embodiment of the invention, a primer or a probe is at least about 10 nucleotides in length. In a preferred embodiment, a primer or a probe is at least about 12 nucleotides in length. In a more preferred embodiment, a primer or probe is at least about 16, 17, 18, 19, 20, 21, 22, 23, 24 or 25 nucleotides in length. While the maximal length of a probe can be as long as the target sequence to be detected, depending on the type of assay in which it is employed, it is typically less than about 50, 60, 65, or 70 nucleotides in length. In the case of a primer, it is typically less than about 30 nucleotides in length. In a specific preferred embodiment of the invention, a primer or a probe is within the length of about 18 and about 28 nucleotides. However, in other embodiments, such as nucleic acid arrays and other embodiments in which probes are affixed to a substrate, the probes can be longer, such as on the order of 30-70, 75, 80, 90, 100, or more

nucleotides in length (see the section below entitled "SNP Detection Kits and Systems").

For analyzing SNPs, it may be appropriate to use oligonucleotides specific for alternative SNP alleles. Such oligonucleotides that detect single nucleotide variations in target sequences may be referred to by such terms as "allele-specific oligonucleotides," "allele-specific probes," or "allele-specific primers." The design and use of allele-specific probes for analyzing polymorphisms is described in, *e.g.,* Mutation Detection: A Practical Approach, Cotton et al., eds., Oxford University Press (1998); Saiki et al., Nature 324:163-166 (1986); Dattagupta, EP235,726; and Saiki, WO 89/11548.

While the design of each allele-specific primer or probe depends on variables such as the precise composition of the nucleotide sequences flanking a SNP position in a target nucleic acid molecule, and the length of the primer or probe, another factor in the use of primers and probes is the stringency of the condition under which the hybridization between the probe or primer and the target sequence is performed. Higher stringency conditions utilize buffers with lower ionic strength and/or a higher reaction temperature, and tend to require a more perfect match between probe/primer and a target sequence in order to form a stable duplex. If the stringency is too high, however, hybridization may not occur at all. In contrast, lower stringency conditions utilize buffers with higher ionic strength and/or a lower reaction temperature, and permit the formation of stable duplexes with more mismatched bases between a probe/primer and a target sequence. By way of example and not limitation, exemplary conditions for high stringency hybridization conditions using an allele-specific probe are as follows: prehybridization with a solution containing 5X standard saline phosphate EDTA (SSPE), 0.5% NaDodSO$_4$ (SDS) at 55°C, and incubating probe with target nucleic acid molecules in the same solution at the same temperature, followed by washing with a solution containing 2X SSPE, and 0.1%SDS at 55°C or room temperature.

Moderate stringency hybridization conditions may be used for allele-specific primer extension reactions with a solution containing, *e.g.,* about 30mM KCl at about 46°C. Alternatively, the reaction may be carried out at an elevated temperature such as 60°C. In another embodiment, a moderately stringent hybridization condition suitable for oligonucleotide ligation assay (OLA) reactions wherein two probes are ligated if they are completely complementary to the target sequence may utilize a solution of about 100mM KCl at a temperature of 46°C.

In a hybridization-based assay, allele-specific probes can be designed that hybridize to a segment of target DNA from one individual but do not hybridize to the corresponding segment from another individual due to the presence of different polymorphic forms (*e.g.*, alternative SNP alleles/nucleotides) in the respective DNA segments from the two individuals. Hybridization conditions should be sufficiently stringent that there is a significant detectable difference in hybridization intensity between alleles, and preferably an essentially binary response, whereby a probe hybridizes to only one of the alleles or significantly more strongly to one allele. While a probe may be designed to hybridize to a target sequence that contains a SNP site such that the SNP site aligns anywhere along the sequence of the probe, the probe is preferably designed to hybridize to a segment of the target sequence such that the SNP site aligns with a central position of the probe (*e.g.*, a position within the probe that is at least three nucleotides from either end of the probe). This design of probe generally achieves good discrimination in hybridization between different allelic forms.

In another embodiment, a probe or primer may be designed to hybridize to a segment of target DNA such that the SNP aligns with either the 5' most end or the 3' most end of the probe or primer. In a specific preferred embodiment that is particularly suitable for use in a oligonucleotide ligation assay (U.S. Patent No. 4,988,617), the 3'most nucleotide of the probe aligns with the SNP position in the target sequence.

Oligonucleotide probes and primers may be prepared by methods well known in the art. Chemical synthetic methods include, but are not limited to, the phosphotriester method described by Narang et al., Methods in Enzymology 68:90 (1979); the phosphodiester method described by Brown et al., Methods in Enzymology 68:109 (1979); the diethylphosphoamidate method described by Beaucage et al., Tetrahedron Letters 22:1859 (1981); and the solid support method described in U.S. Patent No. 4,458,066.

Allele-specific probes are often used in pairs (or, less commonly, in sets of 3 or 4, such as if a SNP position is known to have 3 or 4 alleles, respectively, or to assay both strands of a nucleic acid molecule for a target SNP allele), and such pairs may be identical except for a one nucleotide mismatch that represents the allelic variants at the SNP position. Commonly, one member of a pair perfectly matches a reference form of a target sequence that has a more common SNP allele (*i.e.*, the allele that is more frequent in the target population) and the other member of the pair perfectly matches a form of the target sequence that has a less common SNP allele (*i.e.*, the allele that is rarer in the target population). In the case of an array, multiple pairs of probes can be immobilized on the same support for simultaneous analysis of multiple different polymorphisms.

In one type of PCR-based assay, an allele-specific primer hybridizes to a region on a target nucleic acid molecule that overlaps a SNP position and only primes amplification of an allelic form to which the primer exhibits perfect complementarity. Gibbs, Nucleic Acid Res 17:2427-2448 (1989). Typically, the primer's 3'-most nucleotide is aligned with and complementary to the SNP position of the target nucleic acid molecule. This primer is used in conjunction with a second primer that hybridizes at a distal site. Amplification proceeds from the two primers, producing a detectable product that indicates which allelic form is present in the test sample. A control is usually performed with a second pair of primers, one of which shows a single base mismatch at the polymorphic site and the other of which exhibits perfect complementarity to a distal site. The single-base mismatch prevents amplification or substantially reduces amplification efficiency, so that either no detectable product is formed or it is formed in lower amounts or at a slower pace. The method generally works most effectively when the mismatch is at the 3'-most position of the oligonucleotide (*i.e.*, the 3'-most position of the oligonucleotide aligns with the target SNP position) because this position is most destabilizing to elongation from the primer (see, *e.g.,* WO 93/22456). This PCR-based assay can be utilized as part of the TaqMan assay, described below.

In a specific embodiment of the invention, a primer contains a sequence substantially complementary to a segment of a target SNP-containing nucleic acid molecule except that the primer has a mismatched nucleotide in one of the three nucleotide positions at the 3'-most end of the primer, such that the mismatched nucleotide does not base pair with a particular allele at the SNP site. In a preferred embodiment, the mismatched nucleotide in the primer is the second from the last nucleotide at the 3'-most position of the primer. In a more preferred embodiment, the mismatched nucleotide in the primer is the last nucleotide at the 3'-most position of the primer.

In another embodiment of the invention, a SNP detection reagent of the invention is labeled with a fluorogenic reporter dye that emits a detectable signal. While the preferred reporter dye is a fluorescent dye, any reporter dye that can be attached to a detection reagent such as an oligonucleotide probe or primer is suitable for use in the invention. Such dyes include, but are not limited to, Acridine, AMCA, BODIPY, Cascade Blue, Cy2, Cy3, Cy5, Cy7, Dabcyl, Edans, Eosin, Erythrosin, Fluorescein, 6-Fam, Tet, Joe, Hex, Oregon Green, Rhodamine, Rhodol Green. Tamra, Rox, and Texas Red.

In yet another embodiment of the invention, the detection reagent may be further labeled with a quencher dye such as Tamra, especially when the reagent is used as a self-quenching probe such as a TaqMan (U.S. Patent Nos. 5,210,015 and 5,538,848) or Molecular Beacon probe (U.S. Patent Nos. 5,118,801 and 5,312,728), or other stemless or linear beacon probe (Livak et al., PCR Method Appl 4:357-362 (1995); Tyagi et al., Nature Biotechnology 14:303-308 (1996); Nazarenko et al., Nucl Acids Res 25:2516-2521 (1997); U.S. Patent Nos. 5,866,336 and 6,117,635.

The detection reagents of the invention may also contain other labels, including but not limited to, biotin for streptavidin binding, hapten for antibody binding, and oligonucleotide for binding to another complementary oligonucleotide such as pairs of zipcodes.

The present disclosure also contemplates reagents that do not contain (or that are complementary to) a SNP nucleotide identified herein but that are used to assay one or more SNPs disclosed herein. For example, primers that flank, but do not hybridize directly to a target SNP position provided herein are useful in primer extension reactions in which the primers hybridize to a region adjacent to the target SNP position (*i.e.*, within one or more nucleotides from the target SNP site). During the primer extension reaction, a primer is typically not able to extend past a target SNP site if a particular nucleotide (allele) is present at that target SNP site, and the primer extension product can be detected in order to determine which SNP allele is present at the target SNP site. For example, particular ddNTPs are typically used in the primer extension reaction to terminate primer extension once a ddNTP is incorporated into the extension product (a primer extension product which includes a ddNTP at the 3'-most end of the primer extension product, and in which the ddNTP is a nucleotide of a SNP disclosed herein, is a composition that is specifically contemplated by the present invention). Thus, reagents that bind to a nucleic acid molecule in a region adjacent to a SNP site and that are used for assaying the SNP site, even though the bound sequences do not necessarily include the SNP site itself, are also contemplated herein.

SNP Detection Kits and Systems

A person skilled in the art will recognize that, based on the SNP and associated sequence information disclosed herein, detection reagents can be developed and used to assay the SNP of the present invention, and such detection reagents can be readily incorporated into one of the established kit or system formats which are well known in the art. The terms "kits" and "systems," as used herein in the context of SNP detection reagents, are intended to refer to such things as combinations of multiple SNP detection reagents, or one or more SNP detection reagents in combination with one or more other types of elements or components (*e.g.*, other types of biochemical reagents, containers, packages such as packaging intended for commercial sale, substrates to which SNP detection reagents are attached, electronic hardware components, etc.). Accordingly, the present invention further provides the use of SNP detection kits and systems, including but not limited to, packaged probe and primer sets (*e.g.* TaqMan probe/primer sets), arrays/microarrays of nucleic acid molecules, and beads that contain one or more probes, primers, or other detection reagents for detecting the SNP of the present invention. The kits/systems can optionally include various electronic hardware components; for example, arrays ("DNA chips") and microfluidic systems (lab-on-a-chip" systems) provided by various manufacturers typically comprise hardware components. Other kits/systems (*e.g.*, probe/primer sets) may not include electronic hardware components, but may be comprised of, for example, one or more SNP detection reagents (along with, optionally, other biochemical reagents) packaged in one or more containers.

In some embodiments, a SNP detection kit typically contains one or more detection reagents and other components (*e.g.* a buffer, enzymes such as DNA polymerases or ligases, chain extension nucleotides such as deoxynucleotide triphosphates, and in the case of Sanger-type DNA sequencing reactions, chain terminating nucleotides, positive control sequences, negative control sequences, and the like) necessary to carry out an assay or reaction, such as amplification and/or detection of a SNP-containing nucleic acid molecule. A kit may further contain means for determining the amount of a target nucleic acid, and means for comparing the amount with a standard, and can comprise instructions for using the kit to detect the SNP-containing nucleic acid molecule of interest. In one embodiment, the use of test kits in the method of the invention are provided which contain the necessary reagents to carry out one or more assays to detect the SNP of the invention. In a preferred embodiment of the present invention, the SNP detection kits/systems are in the form of nucleic acid arrays, or compartmentalized kits, including microfluidic/lab-on-a-chip systems.

Exemplary kits of the invention comprise a container containing a SNP detection reagent which detects the SNP of the invention, said container can optionally be enclosed in a package (e.g., a box for commercial sale), and said package can further include other containers containing any or all of the following: enzyme (e.g., polymerase or ligase, any of which can be thermostable), dNTPs and/or ddNTPs (which can optionally be detectably labeled, such as with a fluorescent label or mass tag, and such label can optionally differ between any of the dATPs, dCTPs, dGTPs, dTTPs, ddATPs, ddCTPs, ddGTPs, and/or ddTTPs, so that each of these dNTPs and/or ddNTPs can be distinguished from each other by detection of the label, and any of these dNTPs and/or ddNTPs can optionally be stored in the same container or each in separate containers), buffer, controls (e.g., positive control nucleic acid, or a negative control), reagent(s) for extracting nucleic acid from a test sample, and instructions for using the kit (such as instructions for correlating the presence or absence of a particular allele or genotype with an increased or decreased risk for disease such as VT, or an increased or decreased likelihood of responding to a drug such as a statin). The SNP detection reagent can comprise, for example, at least one primer and/or probe, any of which can optionally be allele-specific, and any of which can optionally be detectably labeled (*e.g.*, with a fluorescent label).

SNP detection kits/systems may contain, for example, one or more probes, or pairs of probes, that hybridize to a nucleic acid molecule at or near each target SNP position. Multiple pairs of allele-specific probes may be included in the kit/system to simultaneously assay large numbers of SNPs, one of which is the SNP of the present invention. In some kits/systems, the allele-specific probes are immobilized to a substrate such as an array or bead. For example, the same substrate can comprise allele-specific probes for detecting at least 1; 10; 100; 1000; 10,000; 100,000 (or any other number in-between) or substantially all of the SNPs shown in Table 1 and/or Table 2.

The terms "arrays," "microarrays," and "DNA chips" are used herein interchangeably to refer to an array of distinct polynucleotides affixed to a substrate, such as glass, plastic, paper, nylon or other type of membrane, filter, chip, or any other suitable solid support. The polynucleotides can be synthesized directly on the substrate, or synthesized separate from the substrate and then affixed to the substrate. In one embodiment, the microarray is prepared and used according to the methods described in Chee et al., U.S. Patent No. 5,837,832 and PCT application WO95/11995; D.J. Lockhart et al., Nat Biotech 14:1675-1680 (1996); and M. Schena et al., Proc Natl Acad Sci 93:10614-10619 (1996). In other embodiments, such arrays are produced by the methods described by Brown et al., U.S. Patent No. 5,807,522.

Nucleic acid arrays are reviewed in the following references: Zammatteo et al., "New chips for molecular biology and diagnostics," Biotechnol Annu Rev 8:85-101 (2002); Sosnowski et al., "Active microelectronic array system for DNA hybridization, genotyping and pharmacogenomic applications," Psychiatr Genet 12(4):181-92 (Dec. 2002); Heller, "DNA microarray technology: devices, systems, and applications," Annu Rev Biomed Eng 4:129-53 (2002); Epub Mar. 22, 2002; Kolchinsky et al., "Analysis of SNPs and other genomic variations using gel-based chips," Hum Mutat 19(4):343-60 (Apr. 2002); and McGall et al., "High-density genechip oligonucleotide probe arrays," Adv Biochem Eng Biotechnol 77:21-42 (2002).

Any number of probes, such as allele-specific probes, may be implemented in an array, and each probe or pair of probes can hybridize to a different SNP position. In the case of polynucleotide probes, they can be synthesized at designated areas (or synthesized separately and then affixed to designated areas) on a substrate using a light-directed chemical process. Each DNA chip can contain, for example, thousands to millions of individual synthetic polynucleotide probes arranged in a grid-like pattern and miniaturized (*e.g.*, to the size of a dime). Preferably, probes are attached to a solid support in an ordered, addressable array.

A microarray can be composed of a large number of unique, single-stranded polynucleotides, usually either synthetic antisense polynucleotides or fragments of cDNAs, fixed to a solid support. Typical polynucleotides are preferably about 6-60 nucleotides in length, more preferably about 15-30 nucleotides in length, and most preferably about 18-25 nucleotides in length. For certain types of microarrays or other detection kits/systems, it may be preferable to use oligonucleotides that are only about 7-20 nucleotides in length. In other types of arrays, such as arrays used in conjunction with chemiluminescent detection technology, preferred probe lengths can be, for example, about 15-80 nucleotides in length, preferably about 50-70 nucleotides in length, more preferably about 55-65 nucleotides in length, and most preferably about 60 nucleotides in length. The microarray or detection kit can contain polynucleotides that cover the known 5' or 3' sequence of a gene/transcript or target SNP site, sequential polynucleotides that cover the full-length sequence of a gene/transcript; or unique polynucleotides selected from particular areas along the length of a target gene/transcript sequence, particularly areas corresponding to one or more SNPs disclosed in Table 1 and/or Table 2.

Polynucleotides used in the microarray or detection kit can be specific to a SNP or SNPs of interest (*e.g.*, specific to a particular SNP allele at a target SNP site, or specific to particular SNP alleles at multiple different SNP sites), or specific to a polymorphic gene/transcript or genes/transcripts of interest.

Hybridization assays based on polynucleotide arrays rely on the differences in hybridization stability of the probes to perfectly matched and mismatched target sequence variants. For SNP genotyping, it is generally preferable that stringency conditions used in hybridization assays are high enough such that nucleic acid molecules that differ from one another at as little as a single SNP position can be differentiated (*e.g.*, typical SNP hybridization assays are designed so that hybridization will occur only if one particular nucleotide is present at a SNP position, but will not occur if an alternative nucleotide is present at that SNP position). Such high stringency conditions may be preferable when using, for example, nucleic acid arrays of allele-specific probes for SNP detection. Such high stringency conditions are described in the preceding section, and are well known to those skilled in the art and can be found in, for example, Current Protocols in Molecular Biology 6,3.1-6,3.6, John Wiley & Sons, N.Y. (1989).

In other embodiments, the arrays are used in conjunction with chemiluminescent detection technology. The following patents and patent applications provide additional information pertaining to chemiluminescent detection. U.S. patent applications that describe chemiluminescent approaches for microarray detection: 10/620332 and 10/620333. U.S. patents that describe methods and compositions of dioxetane for performing chemiluminescent detection: Nos. 6,124,478; 6,107,024; 5,994,073; 5,981,768; 5,871,938; 5,843,681; 5,800,999 and 5,773,628. And the U.S. published application that discloses methods and compositions for microarray controls: US2002/0110828.

In one embodiment of the invention, a nucleic acid array can comprise an array of probes of about 15-25 nucleotides in length. In further embodiments, a nucleic acid array can comprise any number of probes, in which at least one probe is capable of detecting the SNP of the invention disclosed in Table 1 and/or Table 2, and at least one probe comprises a fragment of one of the sequences selected from the group consisting of those disclosed in Table 1, Table 2, the Sequence Listing, and sequences complementary thereto, said fragment comprising at least about 8 consecutive nucleotides, preferably 10, 12, 15, 16, 18, 20, more preferably 22, 25, 30, 40, 47, 50, 55, 60, 65, 70, 80, 90, 100, or more consecutive nucleotides (or any other number in-between) and containing (or being complementary to) a novel SNP allele disclosed in Table 1 and/or Table 2. In some embodiments, the nucleotide complementary to the SNP site is within 5, 4, 3, 2, or 1 nucleotide from the center of the probe, more preferably at the center of said probe.

A polynucleotide probe can be synthesized on the surface of the substrate by using a chemical coupling procedure and an ink jet application apparatus, as described in PCT application WO9-5/251116 (Baldeschweiler et al.) In another aspect, a "gridded" array analogous to a dot (or slot) blot may be used to arrange and link cDNA fragments or oligonucleotides to the surface of a substrate using a vacuum system, thermal, UV, mechanical or chemical bonding procedures. An array, such as those described above, may be produced by hand or by using available devices (slot blot or dot blot apparatus), materials (any suitable solid support), and machines (including robotic instruments), and may contain 8, 24, 96, 384, 1536, 6144 or more polynucleotides, or any other number which lends itself to the efficient use of commercially available instrumentation.

Using such arrays or other kits/systems, the method of the present invention encompasses a means of identifying the SNPs disclosed herein in a test sample. Such methods typically involve incubating a test sample of nucleic acids with an array comprising one or more probes corresponding to at least one SNP position of the present invention, and assaying for binding of a nucleic acid from the test sample with one or more of the probes. Conditions for incubating a SNP detection reagent (or a kit/system that employs one or more such SNP detection reagents) with a test sample vary. Incubation conditions depend on such factors as the format employed in the assay, the detection methods employed, and the type and nature of the detection reagents used in the assay. One skilled in the art will recognize that any one of the commonly available hybridization, amplification and array assay formats can readily be adapted to detect the SNPs disclosed herein.

A SNP detection kit/system of the present invention may include components that are used to prepare nucleic acids from a test sample for the subsequent amplification and/or detection of a SNP-containing nucleic acid molecule. Such sample preparation components can be used to produce nucleic acid extracts (including DNA and/or RNA), proteins or membrane extracts from any bodily fluids (such as blood, serum, plasma, urine, saliva, phlegm, gastric juices, semen, tears, sweat, etc.), skin, hair, cells (especially nucleated cells) such as buccal cells (*e.g.,* as obtained by buccal swabs), biopsies, or tissue specimens. The test samples used in the above-described methods will vary based on such factors as the assay format, nature of the detection method, and the specific tissues, cells or extracts used as the test sample to be assayed. Methods of preparing nucleic acids, proteins, and cell extracts are well known in the art and can be readily adapted to obtain a sample that is compatible with the system utilized. Automated sample preparation systems for extracting nucleic acids from a test sample are commercially available, and examples are Qiagen's BioRobot 9600, Applied Biosystems' PRISM™ 6700 sample preparation system, and Roche Molecular Systems' COBAS AmpliPrep System.

Another form of kit contemplated by the present invention is a compartmentalized kit A compartmentalized kit includes any kit in which reagents are contained in separate containers. Such containers include, for example, small glass containers, plastic containers, strips of plastic, glass or paper, or arraying material such as silica. Such containers allow one to efficiently transfer reagents from one compartment to another compartment such that the test samples and reagents are not cross-contaminated, or from one container to another vessel not included in the kit, and the agents or solutions of each container can be added in a quantitative fashion from one compartment to another or to another vessel. Such containers may include, for example, one or more containers which will accept the test sample, one or more containers which contain at least one probe or other SNP detection reagent for detecting the SNP of the present invention, one or more containers which contain wash reagents (such as phosphate buffered saline, Tris-buffers, etc.), and one or more containers which contain the reagents used to reveal the presence of the bound probe or other SNP detection reagents. The kit can optionally further comprise compartments and/or reagents for, for example, nucleic acid amplification or other enzymatic reactions such as primer extension reactions, hybridization, ligation, electrophoresis (preferably capillary electrophoresis), mass spectrometry, and/or laser-induced fluorescent detection. The kit may also include instructions for using the kit. Exemplary compartmentalized kits include microfluidic devices known in the art. See, *e.g.,* Weigl et al., "Lab-on-a-chip for drug development," Adv Drug Deliv Rev 55(3):349-77 (Feb. 2003). In such microfluidic devices, the containers may be referred to as, for example, microfluidic "compartments," "chambers," or "channels."

Microfluidic devices, which may also be referred to as "lab-on-a-chip" systems, biomedical micro-electro-mechanical systems (bioMEMs), or multicomponent integrated systems, are exemplary kits/systems for analyzing SNPs. Such systems miniaturize and compartmentalize processes such as probe/target hybridization, nucleic acid amplification, and capillary electrophoresis reactions in a single functional device. Such microfluidic devices typically utilize detection reagents in at least one aspect of the system, and such detection reagents may be used to detect the SNP of the present invention. One example of a microfluidic system is disclosed in U.S. Patent No. 5,589,136, which describes the integration of PCR amplification and capillary electrophoresis in chips. Exemplary microfluidic systems comprise a pattern of microchannels designed onto a glass, silicon, quartz, or plastic wafer included on a microchip. The movements of the samples may be controlled by electric, electroosmotic or hydrostatic forces applied across different areas of the microchip to create functional microscopic valves and pumps with no moving parts. Varying the voltage can be used as a means to control the liquid flow at intersections between the micro-machined channels and to change the liquid flow rate for pumping across different sections of the microchip. See, for example, U.S. Patent Nos. 6,153,073, Dubrow et al., and 6,156,181, Parce et al.

For genotyping SNPs, an exemplary microfluidic system may integrate, for example, nucleic acid amplification, primer extension, capillary electrophoresis, and a detection method such as laser induced fluorescence detection. In a first step of an exemplary process for using such an exemplary system, nucleic acid samples are amplified, preferably by PCR. Then, the amplification products are subjected to automated primer extension reactions using ddNTPs (specific fluorescence for each ddNTP) and the appropriate oligonucleotide primers to carry out primer extension reactions which hybridize just upstream of the targeted SNP. Once the extension at the 3' end is completed, the primers are separated from the unincorporated fluorescent ddNTPs by capillary electrophoresis. The separation medium used in capillary electrophoresis can be, for example, polyacrylamide, polyethyleneglycol or dextran. The incorporated ddNTPs in the single nucleotide primer extension products are identified by laser-induced fluorescence detection. Such an exemplary microchip can be used to process, for example, at least 96 to 384 samples, or more, in parallel.

USES OF NUCLEIC ACID MOLECULES

The nucleic acid molecules used as detection reagents in the method of the present invention have a variety of uses, particularly for predicting whether an individual will benefit from statin treatment by reducing their risk for VT in response to the statin treatment, as well as for the diagnosis, prognosis, treatment, and prevention of VT. For example, the nucleic acid molecules of the invention are useful for determining the likelihood of an individual who currently or previously has or has had VT or who is at increased risk for developing VT (such as an individual who has not yet had VT but is at increased risk for having VT in the future) of responding to treatment (or prevention) of VT with statins (such as by reducing their risk of developing primary or recurrent VT in the future), predicting the likelihood that the individual will experience toxicity or other undesirable side effects from the statin treatment, predicting an individual's risk for developing VT, etc. For example, the nucleic acid molecules are useful as hybridization probes, such as for genotyping SNPs in messenger RNA, transcript, cDNA, genomic DNA, amplified DNA or other nucleic acid molecules, and for isolating full-length cDNA and genomic clones encoding the variant peptides disclosed in Table 1 as well as their orthologs.

A probe can hybridize to any nucleotide sequence along the entire length of a nucleic acid molecule referred to in Table 1 and/or Table 2. Preferably, a probe of the present invention hybridizes to a region of a target sequence that encompasses a SNP position indicated in Table 1 and/or Table 2. More preferably, a probe hybridizes to a SNP-containing target sequence in a sequence-specific manner such that it distinguishes the target sequence from other nucleotide sequences which vary from the target sequence only by which nucleotide is present at the SNP site. Such a probe is particularly useful for detecting the presence of a SNP-containing nucleic acid in a test sample, or for determining which nucleotide (allele) is present at a particular SNP site (*i.e.,* genotyping the SNP site).

A nucleic acid hybridization probe may be used for determining the presence, level, form. and/or distribution of nucleic acid expression. The nucleic acid whose level is determined can be DNA or RNA. Accordingly, probes specific for the SNPs described herein can be used to assess the presence, expression and/or gene copy number in a given cell, tissue, or organism. These uses are relevant for diagnosis of disorders involving an increase or decrease in gene expression relative to normal levels. *In vitro* techniques for detection of mRNA include, for example, Northern blot hybridizations and *in situ* hybridizations. *In vitro* techniques for detecting DNA include Southern blot hybridizations and *in situ* hybridizations. Sambrook and Russell, Molecular Cloning: A laboratory Manual, Cold Spring Harbor Press, N.Y. (2000).

Probes can be used as part of a diagnostic test kit for identifying cells or tissues in which a variant protein is expressed, such as by measuring the level of a variant protein-encoding nucleic acid (*e.g.*, mRNA) in a sample of cells from a subject or determining if a polynucleotide contains a SNP of interest.

Thus, the nucleic acid molecules of the invention can be used as hybridization probes to detect the SNPs of the present invention, thereby determining the likelihood that an individual will respond positively to statin treatment for reducing the risk of VT, or whether an individual with the polymorphism(s) is at risk for developing VT (or has already developed early stage VT). Detection of a SNP associated with a disease phenotype provides a diagnostic tool for an active disease and/or genetic predisposition to the disease.

Furthermore, the nucleic acid molecules of the invention are therefore useful for detecting a gene (gene information is disclosed in Table 2, for example) which contains the SNP of the invention and/or products of such genes, such as expressed mRNA transcript molecules (transcript information is disclosed in Table 1, for example), and are thus useful for detecting gene expression. The nucleic acid molecules can optionally be implemented in, for example, an array or kit format for use in detecting gene expression.

The nucleic acid molecules of the invention are also useful as primers to amplify any given region of a nucleic acid molecule, particularly a region containing the SNP of the invention in Table 1 and/or Table 2.

The nucleic acid molecules disclosed herein are also useful for constructing recombinant vectors (described in greater detail below). Such vectors include expression vectors that express a portion of, or all of, any of the variant peptide sequences referred to in Table 1. Vectors also include insertion vectors, used to integrate into another nucleic acid molecule sequence, such as into the cellular genome, to alter *in situ* expression of a gene and/or gene product. For example, an endogenous coding sequence can be replaced via homologous recombination with all or part of the coding region containing one or more specifically introduced SNPs.

The nucleic acid molecules disclosed herein are also useful for expressing antigenic portions of the variant proteins, particularly antigenic portions that contain a variant amino acid sequence (*e.g.*, an amino acid substitution) caused by a SNP disclosed in Table 1 and/or Table 2.

The nucleic acid molecules disclosed herein also useful for constructing vectors containing a gene regulatory region of the nucleic acid molecules of the present invention.

The nucleic acid molecules disclosed herein are also useful for designing ribozymes corresponding to all, or a part, of an mRNA molecule expressed from a SNP-containing nucleic acid molecule described herein.

The nucleic acid molecules disclosed herein are also useful for constructing host cells expressing a part, or all, of the nucleic acid molecules and variant peptides.

The nucleic acid molecules disclosed herein are also useful for constructing transgenic animals expressing all, or a part, of the nucleic acid molecules and variant peptides. The production of recombinant cells and transgenic animals having nucleic acid molecules which contain the SNPs disclosed in Table 1 and/or Table 2 allows, for example, effective clinical design of treatment compounds and dosage regimens.

The nucleic acid molecules disclosed herein are also useful in assays for drug screening to identify compounds that, for example, modulate nucleic acid expression.

The nucleic acid molecules disclosed herein are also useful in gene therapy in patients whose cells have aberrant gene expression. Thus, recombinant cells, which include a patient's cells that have been engineered *ex vivo* and returned to the patient, can be introduced into an individual where the recombinant cells produce the desired protein to treat the individual.

SNP Genotyping Methods

The process of determining which nucleotide(s) is/are present at each of one or more SNP positions (such as a SNP position disclosed in Table 1 and/or Table 2), for either or both alleles, may be referred to by such phrases as SNP genotyping, determining the "identity" of a SNP, determining the "content" of a SNP, or determining which nucleotide(s)/allele(s) is/are present at a SNP position. Thus, these terms can refer to detecting a single allele (nucleotide) at a SNP position or can encompass detecting both alleles (nucleotides) at a SNP position (such as to determine the homozygous or heterozygous state of a SNP position). Furthermore, these terms may also refer to detecting an amino acid residue encoded by a SNP (such as alternative amino acid residues that are encoded by different codons created by alternative nucleotides at a missense SNP position, for example).

The method of the present invention encompasses SNP genotyping, such as for use in implementing a preventive or treatment regimen for an individual based on that individual having an increased susceptibility for developing VT and/or an increased likelihood of benefiting from statin treatment for reducing the risk of VT, in evaluating an individual's likelihood of responding to statin treatment (particularly for treating or preventing VT), in selecting a treatment or preventive regimen (*e.g.*, in deciding whether or not to administer statin treatment to an individual having VT, or who is at increased risk for developing VT in the future), or in formulating or selecting a particular statin-based treatment or preventive regimen such as dosage and/or frequency of administration of statin treatment or choosing which form/type of statin to be administered, such as a particular pharmaceutical composition or compound, etc.), determining the likelihood of experiencing toxicity or other undesirable side effects from statin treatment, or selecting individuals for a clinical trial of a statin (*e.g.*, selecting individuals to participate in the trial who are most likely to respond positively from the statin treatment and/or excluding individuals from the trial who are unlikely to respond positively from the statin treatment based on their SNP genotype(s), or selecting individuals who are unlikely to respond positively to statins based on their SNP genotype(s) to participate in a clinical trial of another type of drug that may benefit them), etc. The SNP genotyping methods encompassed by the method of the invention can also be useful for evaluating an individual's risk for developing VT and for predicting the likelihood that an individual who has previously had VT will have a recurrence of VT again in the future (recurrent VT).

Nucleic acid samples can be genotyped to determine which allele(s) is/are present at any given genetic region (*e.g.*, SNP position) of interest by methods well known in the art. The neighboring sequence can be used to design SNP detection reagents such as oligonucleotide probes, which may optionally be implemented in a kit format. Exemplary SNP genotyping methods are described in Chen et al., "Single nucleotide polymorphism genotyping: biochemistry. protocol, cost and throughput," Pharmacogenomics J 3(2):77-96 (2003); Kwok et al., "Detection of single nucleotide polymorphisms," Curr Issues Mol Biol 5(2):43-60 (Apr. 2003); Shi, "Technologies for individual genotyping: detection of genetic polymorphisms in drug targets and disease genes," Am J Pharmacogenomics 2(3): 197-205 (2002); and Kwok, "Methods for genotyping single nucleotide polymorphisms," Annu Rev Genomics Hum Genet 2:235-58 (2001). Exemplary techniques for high-throughput SNP genotyping are described in Marnellos, "High-throughput SNP analysis for genetic association studies," Curr Opin Drug Discov Devel 6(3)317-21 (May 2003). Common SNP genotyping methods include, but are not limited to, TaqMan assays, molecular beacon assays, nucleic acid arrays, allele-specific primer extension, allele-specific PCR, arrayed primer extension, homogeneous primer extension assays, primer extension with detection by mass spectrometry, pyrosequencing, multiplex primer extension sorted on genetic arrays, ligation with rolling circle amplification, homogeneous ligation, OLA (U.S. Patent No. 4,988,167), multiplex ligation reaction sorted on genetic arrays, restriction-fragment length polymorphism, single base extension-tag assays, and the Invader assay. Such methods may be used in combination with detection mechanisms such as, for example, luminescence or chemiluminescence detection, fluorescence detection, time-resolved fluorescence detection, fluorescence resonance energy transfer, fluorescence polarization, mass spectrometry, and electrical detection.

Various methods for detecting polymorphisms include, but are not limited to, methods in which protection from cleavage agents is used to detect mismatched bases in RNA/RNA or RNA/DNA duplexes (Myers et al., Science 230:1242 (1985); Cotton et al., PNAS 85:4397 (1988); and Saleeba et al., Meth. Enzymol 217:286-295 (1992)), comparison of the electrophoretic mobility of variant and wild type nucleic acid molecules (Orita et al., PNAS 86:2766 (1989); Cotton et al., Mutat Res 285:125-144 (1993); and Hayashi et al., Genet Anal Tech Appl 9:73-79 (1992)), and assaying the movement of polymorphic or wild-type fragments in polyacrylamide gels containing a gradient of denaturant using denaturing gradient gel electrophoresis (DGGE) (Myers et al., Nature 313:495 (1985)). Sequence variations at specific locations can also be assessed by nuclease protection assays such as RNase and S1 protection or chemical cleavage methods.

In a preferred embodiment, SNP genotyping is performed using the TaqMan assay, which is also known as the 5' nuclease assay (U.S. Patent Nos. 5,210,015 and 5,538,848). The TaqMan assay detects the accumulation of a specific amplified product during PCR. The TaqMan assay utilizes an oligonucleotide probe labeled with a fluorescent reporter dye and a quencher dye. The reporter dye is excited by irradiation at an appropriate wavelength, it transfers energy to the quencher dye in the same probe via a process called fluorescence resonance energy transfer (FRET). When attached to the probe, the excited reporter dye does not emit a signal. The proximity of the quencher dye to the reporter dye in the intact probe maintains a reduced fluorescence for the reporter. The reporter dye and quencher dye may be at the 5' most and the 3' most ends, respectively, or vice versa. Alternatively, the reporter dye may be at the 5' or 3' most end while the quencher dye is attached to an internal nucleotide, or vice versa. In yet another embodiment, both the reporter and the quencher may be attached to internal nucleotides at a distance from each other such that fluorescence of the reporter is reduced.

During PCR, the 5' nuclease activity of DNA polymerase cleaves the probe, thereby separating the reporter dye and the quencher dye and resulting in increased fluorescence of the reporter. Accumulation of PCR product is detected directly by monitoring the increase in fluorescence of the reporter dye. The DNA polymerase cleaves the probe between the reporter dye and the quencher dye only if the probe hybridizes to the target SNP-containing template which is amplified during PCR, and the probe is designed to hybridize to the target SNP site only if a particular SNP allele is present.

Preferred TaqMan primer and probe sequences can readily be determined using the SNP and associated nucleic acid sequence information provided herein. A number of computer programs, such as Primer Express (Applied Biosystems, Foster City, CA), can be used to rapidly obtain optimal primer/probe sets. It will be apparent to one of skill in the art that such primers and probes for detecting the SNPs of the present invention are useful in, for example, screening individuals for their likelihood of responding to statin treatment (i.e., benefiting from statin treatment), particularly individuals who have or are susceptible to VT, or in screening for individuals who are susceptible to developing VT. These probes and primers can be readily incorporated into a kit format. The present invention also includes modifications of the Taqman assay well known in the art such as the use of Molecular Beacon probes (U.S. Patent Nos. 5,118,801 and 5,312,728) and other variant formats (U.S. Patent Nos. 5,866,336 and 6,117,635).

Another preferred method for genotyping the SNP of the present invention is the use of two oligonucleotide probes in an OLA (see, *e.g.*, U.S. Patent No. 4,988,617). In this method, one probe hybridizes to a segment of a target nucleic acid with its 3' most end aligned with the SNP site. A second probe hybridizes to an adjacent segment of the target nucleic acid molecule directly 3' to the first probe. The two juxtaposed probes hybridize to the target nucleic acid molecule, and are ligated in the presence of a linking agent such as a ligase if there is perfect complementarity between the 3' most nucleotide of the first probe with the SNP site. If there is a mismatch, ligation would not occur. After the reaction, the ligated probes are separated from the target nucleic acid molecule, and detected as indicators of the presence of a SNP.

The following patents, patent applications, and published international patent applications, provide additional information pertaining to techniques for carrying out various types of OLA. The following U.S. patents describe OLA strategies for performing SNP detection: Nos. 6,027,889; 6,268,148; 5,494,810; 5,830,711 and 6,054,564. WO 97/31256 and WO 00/56927 describe OLA strategies for performing SNP detection using universal arrays, wherein a zipcode sequence can be introduced into one of the hybridization probes, and the resulting product, or amplified product, hybridized to a universal zip code array. U.S. application US01/17329 (and 09/584,905) describes OLA (or LDR) followed by PCR, wherein zipcodes are incorporated into OLA probes, and amplified PCR products are determined by electrophoretic or universal zipcode array readout. U.S. applications 60/427818, 60/445636, and 60/445494 describe SNPlex methods and software for multiplexed SNP detection using OLA followed by PCR, wherein zipcodes are incorporated into OLA probes, and amplified PCR products are hybridized with a zipchute reagent, and the identity of the SNP determined from electrophoretic readout of the zipchute. In some embodiments, OLA is carried out prior to PCR (or another method of nucleic acid amplification). In other embodiments, PCR (or another method of nucleic acid amplification) is carried out prior to OLA.

Another method for SNP genotyping is based on mass spectrometry. Mass spectrometry takes advantage of the unique mass of each of the four nucleotides of DNA. SNPs can be unambiguously genotyped by mass spectrometry by measuring the differences in the mass of nucleic acids having alternative SNP alleles. MALDI-TOF (Matrix Assisted Laser Desorption Ionization - Time of Flight) mass spectrometry technology is preferred for extremely precise determinations of molecular mass, such as SNPs. Numerous approaches to SNP analysis have been developed based on mass spectrometry. Preferred mass spectrometry-based methods of SNP genotyping include primer extension assays, which can also be utilized in combination with other approaches, such as traditional gel-based formats and microarrays.

Typically, the primer extension assay involves designing and annealing a primer to a template PCR amplicon upstream (5') from a target SNP position. A mix of dideoxynucleotide triphosphates (ddNTPs) and/or deoxynucleotide triphosphates (dNTPs) are added to a reaction mixture containing template (*e.g.,* a SNP-containing nucleic acid molecule which has typically been amplified, such as by PCR), primer, and DNA polymerase. Extension of the primer terminates at the first position in the template where a nucleotide complementary to one of the ddNTPs in the mix occurs. The primer can be either immediately adjacent (*i.e.,* the nucleotide at the 3' end of the primer hybridizes to the nucleotide next to the target SNP site) or two or more nucleotides removed from the SNP position. If the primer is several nucleotides removed from the target SNP position, the only limitation is that the template sequence between the 3' end of the primer and the SNP position cannot contain a nucleotide of the same type as the one to be detected, or this will cause premature termination of the extension primer. Alternatively, if all four ddNTPs alone, with no dNTPs, are added to the reaction mixture, the primer will always be extended by only one nucleotide, corresponding to the target SNP position. In this instance, primers are designed to bind one nucleotide upstream from the SNP position (*i.e.,* the nucleotide at the 3' end of the primer hybridizes to the nucleotide that is immediately adjacent to the target SNP site on the 5' side of the target SNP site). Extension by only one nucleotide is preferable, as it minimizes the overall mass of the extended primer, thereby increasing the resolution of mass differences between alternative SNP nucleotides. Furthermore, mass-tagged ddNTPs can be employed in the primer extension reactions in place of unmodified ddNTPs. This increases the mass difference between primers extended with these ddNTPs, thereby providing increased sensitivity and accuracy, and is particularly useful for typing heterozygous base positions. Mass-tagging also alleviates the need for intensive sample-preparation procedures and decreases the necessary resolving power of the mass spectrometer.

The extended primers can then be purified and analyzed by MALDI-TOF mass spectrometry to determine the identity of the nucleotide present at the target SNP position. In one method of analysis, the products from the primer extension reaction are combined with light absorbing crystals that form a matrix. The matrix is then hit with an energy source such as a laser to ionize and desorb the nucleic acid molecules into the gas-phase. The ionized molecules are then ejected into a flight tube and accelerated down the tube towards a detector. The time between the ionization event, such as a laser pulse, and collision of the molecule with the detector is the time of flight of that molecule. The time of flight is precisely correlated with the mass-to-charge ratio (m/z) of the ionized molecule. Ions with smaller m/z travel down the tube faster than ions with larger m/z and therefore the lighter ions reach the detector before the heavier ions. The time-of-flight is then converted into a corresponding, and highly precise, m/z. In this manner, SNPs can be identified based on the slight differences in mass, and the corresponding time of flight differences, inherent in nucleic acid molecules having different nucleotides at a single base position. For further information regarding the use of primer extension assays in conjunction with MALDI-TOF mass spectrometry for SNP genotyping, see, *e.g.,* Wise et al., "A standard protocol for single nucleotide primer extension in the human genome using matrix-assisted laser desorption/ionization time-of-flight mass spectrometry," Rapid Commun Mass Spectrom 17(11):1195-202 (2003).

The following references provide further information describing mass spectrometry-based methods for SNP genotyping: Bocker, "SNP and mutation discovery using base-specific cleavage and MALDI-TOF mass spectrometry," Bioinformatics 19 Suppl 1:I44-I53 (Jul. 2003); Storm et al., "MALDI-TOF mass spectrometry-based SNP genotyping," Methods Mol Biol 212:241-62 (2003); Jurinke et al., "The use of Mass ARRAY technology for high throughput genotyping," Adv Biochem Eng Biotechnol 77:57-74 (2002); and Jurinke et al., "Automated genotyping using the DNA MassArray technology," Methods Mol Biol 187:179-92 (2002).

SNPs can also be scored by direct DNA sequencing. A variety of automated sequencing procedures can be utilized (*e.g.* Biotechniques 19:448 (1995)), including sequencing by mass spectrometry. See, *e.g.*, PCT International Publication No. WO 94/16101; Cohen et al., Adv Chromatogr 36:127-162 (1996); and Griffin et al., Appl Biochem Biotechnol 38:147-159 (1993). The nucleic acid sequences of the present invention enable one of ordinary skill in the art to readily design sequencing primers for such automated sequencing procedures. Commercial instrumentation, such as the Applied Biosystems 377, 3100, 3700, 3730, and 3730xl DNA Analyzers (Foster City, CA), is commonly used in the art for automated sequencing.

Other methods that can be used to genotype the SNPs of the present invention include single-strand conformational polymorphism (SSCP), and denaturing gradient gel electrophoresis (DGGE). Myers et al., Nature 313:495 (1985). SSCP identifies base differences by alteration in electrophoretic migration of single stranded PCR products, as described in Orita et al., Proc. Nat. Acad. Single-stranded PCR products can be generated by heating or otherwise denaturing double stranded PCR products. Single-stranded nucleic acids may refold or form secondary structures that are partially dependent on the base sequence. The different electrophoretic mobilities of single-stranded amplification products are related to base-sequence differences at SNP positions. DGGE differentiates SNP alleles based on the different sequence-dependent stabilities and melting properties inherent in polymorphic DNA and the corresponding differences in electrophoretic migration patterns in a denaturing gradient gel. PCR Technology: Principles and Applications for DNA Amplification Chapter 7, Erlich, ed., W.H. Freeman and Co, N.Y. (1992).

Sequence-specific ribozymes (U.S. Patent No. 5,498,531) can also be used to score SNPs based on the development or loss of a ribozyme cleavage site. Perfectly matched sequences can be distinguished from mismatched sequences by nuclease cleavage digestion assays or by differences in melting temperature. If the SNP affects a restriction enzyme cleavage site, the SNP can be identified by alterations in restriction enzyme digestion patterns, and the corresponding changes in nucleic acid fragment lengths determined by gel electrophoresis.

SNP genotyping can include the steps of, for example, collecting a biological sample from a human subject (*e.g.*, sample of tissues, cells, fluids, secretions, etc.), isolating nucleic acids (*e.g.*, genomic DNA, mRNA or both) from the cells of the sample, contacting the nucleic acids with one or more primers which specifically hybridize to a region of the isolated nucleic acid containing a target SNP under conditions such that hybridization and amplification of the target nucleic acid region occurs, and determining the nucleotide present at the SNP position of interest, or, in some assays, detecting the presence or absence of an amplification product (assays can be designed so that hybridization and/or amplification will only occur if a particular SNP allele is present or absent). In some assays, the size of the amplification product is detected and compared to the length of a control sample; for example, deletions and insertions can be detected by a change in size of the amplified product compared to a normal genotype.

SNP genotyping is useful for numerous practical applications, as described below. Examples of such applications include, but are not limited to, SNP-disease association analysis, disease predisposition screening, disease diagnosis, disease prognosis, disease progression monitoring, determining therapeutic strategies based on an individual's genotype ("pharmacogenomics"), developing therapeutic agents based on SNP genotypes associated with a disease or likelihood of responding to a drug, stratifying patient populations for clinical trials of a therapeutic, preventive, or diagnostic agent, and predicting the likelihood that an individual will experience toxic side effects from a therapeutic agent.

Analysis of Genetic Associations between SNPs and Phenotypic Traits

SNP genotyping for disease diagnosis, disease predisposition screening, disease prognosis, determining drug responsiveness (pharmacogenomics), drug toxicity screening, and other uses described herein, typically relies on initially establishing a genetic association between one or more specific SNPs and the particular phenotypic traits of interest.

Different study designs may be used for genetic association studies. Modern Epidemiology 609-622, Lippincott, Williams & Wilkins (1998). Observational studies are most frequently carried out in which the response of the patients is not interfered with. The first type of observational study identifies a sample of persons in whom the suspected cause of the disease is present and another sample of persons in whom the suspected cause is absent, and then the frequency of development of disease in the two samples is compared. These sampled populations are called cohorts, and the study is a prospective study. The other type of observational study is case-control or a retrospective study. In typical case-control studies, samples are collected from individuals with the phenotype of interest (cases) such as certain manifestations of a disease, and from individuals without the phenotype (controls) in a population (target population) that conclusions are to be drawn from. Then the possible causes of the disease are investigated retrospectively. As the time and costs of collecting samples in case-control studies are considerably less than those for prospective studies, case-control studies are the more commonly used study design in genetic association studies, at least during the exploration and discovery stage.

Case-only studies are an alternative to case-control studies when gene-environment interaction is the association of interest (Piegorsch et al., "Non-hierarchical logistic models and case-only designs for assessing susceptibility in population-based case-control studies", Statistics in Medicine 13 (1994) pp 153-162). In a typical case-only study of gene-environment interaction, genotypes are obtained only from cases who are often selected from an existing cohort study. The association between genotypes and the environmental factor is then assessed and a significant association implies that the effect of the environmental factor on the endpoint of interest (the case definition) differs by genotype. The primary assumption underlying the test of association in case-only studies is that the environmental effect of interest is independent of genotype (e.g., allocation to statin therapy is independent of genotype) and it has been shown that the case-only design has more power than the case-control design to detect gene-environment interaction when this assumption is true in the population (Yang et al., "Sample Size Requirements in Case-Only Designs to Detect Gene-Environment Interaction", American Journal of Epidemiology 146:9 (1997) pp713-720). Selecting cases from a randomized clinical trial may be an ideal setting in which to perform a case-only study since genotypes will be independent of treatment by design.

In observational studies, there may be potential confounding factors that should be taken into consideration. Confounding factors are those that are associated with both the real cause(s) of the disease and the disease itself, and they include demographic information such as age, gender, ethnicity as well as environmental factors. When confounding factors are not matched in cases and controls in a study, and are not controlled properly, spurious association results can arise. If potential confounding factors are identified, they should be controlled for by analysis methods explained below.

In a genetic association study, the cause of interest to be tested is a certain allele or a SNP or a combination of alleles or a haplotype from several SNPs. Thus, tissue specimens (*e.g.*, whole blood) from the sampled individuals may be collected and genomic DNA genotyped for the SNP(s) of interest. In addition to the phenotypic trait of interest, other information such as demographic (*e.g.*, age, gender, ethnicity, etc.), clinical, and environmental information that may influence the outcome of the trait can be collected to further characterize and define the sample set. In many cases, these factors are known to be associated with diseases and/or SNP allele frequencies. There are likely gene-environment and/or gene-gene interactions as well. Analysis methods to address gene-environment and gene-gene interactions (for example, the effects of the presence of both susceptibility alleles at two different genes can be greater than the effects of the individual alleles at two genes combined) are discussed below.

After all the relevant phenotypic and genotypic information has been obtained, statistical analyses are carried out to determine if there is any significant correlation between the presence of an allele or a genotype with the phenotypic characteristics of an individual. Preferably, data inspection and cleaning are first performed before carrying out statistical tests for genetic association. Epidemiological and clinical data of the samples can be summarized by descriptive statistics with tables and graphs. Data validation is preferably performed to check for data completion, inconsistent entries, and outliers. Chi-squared tests and t-tests (Wilcoxon rank-sum tests if distributions are not normal) may then be used to check for significant differences between cases and controls for discrete and continuous variables, respectively. To ensure genotyping quality, Hardy-Weinberg disequilibrium tests can be performed on cases and controls separately. Significant deviation from Hardy-Weinberg equilibrium (HWE) in both cases and controls for individual markers can be indicative of genotyping errors. If HWE is violated in a majority of markers, it is indicative of population substructure that should be further investigated. Moreover, Hardy-Weinberg disequilibrium in cases only can indicate genetic association of the markers with the disease. B. Weir, Genetic Data Analysis, Sinauer (1990).

To test whether an allele of a single SNP is associated with the case or control status of a phenotypic trait, one skilled in the art can compare allele frequencies in cases and controls. Standard chi-squared tests and Fisher exact tests can be carried out on a 2x2 table (2 SNP alleles x 2 outcomes in the categorical trait of interest). To test whether genotypes of a SNP are associated, chi-squared tests can be carried out on a 3x2 table (3 genotypes x 2 outcomes). Score tests are also carried out for genotypic association to contrast the three genotypic frequencies (major homozygotes, heterozygotes and minor homozygotes) in cases and controls, and to look for trends using 3 different modes of inheritance, namely dominant (with contrast coefficients 2, -1, -1), additive or allelic (with contrast coefficients 1, 0, -1) and recessive (with contrast coefficients 1, 1, -2). Odds ratios for minor versus major alleles, and odds ratios for heterozygote and homozygote variants versus the wild type genotypes are calculated with the desired confidence limits, usually 95%.

In order to control for confounders and to test for interaction and effect modifiers, stratified analyses may be performed using stratified factors that are likely to be confounding, including demographic information such as age, ethnicity, and gender, or an interacting element or effect modifier, such as a known major gene (*e.g.*, APOE for Alzheimer's disease or HLA genes for autoimmune diseases), or environmental factors such as smoking in lung cancer. Stratified association tests may be carried out using Cochran-Mantel-Haenszel tests that take into account the ordinal nature of genotypes with 0, 1, and 2 variant alleles. Exact tests by StatXact may also be performed when computationally possible. Another way to adjust for confounding effects and test for interactions is to perform stepwise multiple logistic regression analysis using statistical packages such as SAS or R. Logistic regression is a model-building technique in which the best fitting and most parsimonious model is built to describe the relation between the dichotomous outcome (for instance, getting a certain disease or not) and a set of independent variables (for instance, genotypes of different associated genes, and the associated demographic and environmental factors). The most common model is one in which the logit transformation of the odds ratios is expressed as a linear combination of the variables (main effects) and their cross-product terms (interactions). Hosmer and Lemeshow, Applied Logistic Regression, Wiley (2000). To test whether a certain variable or interaction is significantly associated with the outcome, coefficients in the model are first estimated and then tested for statistical significance of their departure from zero.

In addition to performing association tests one marker at a time, haplotype association analysis may also be performed to study a number of markers that are closely linked together. Haplotype association tests can have better power than genotypic or allelic association tests when the tested markers are not the disease-causing mutations themselves but are in linkage disequilibrium with such mutations. The test will even be more powerful if the disease is indeed caused by a combination of alleles on a haplotype (*e.g.*, APOE is a haplotype formed by 2 SNPs that are very close to each other). In order to perform haplotype association effectively, marker-marker linkage disequilibrium measures, both D' and $r^2$, are typically calculated for the markers within a gene to elucidate the haplotype structure. Recent studies in linkage disequilibrium indicate that SNPs within a gene are organized in block pattern, and a high degree of linkage disequilibrium exists within blocks and very little linkage disequilibrium exists between blocks. Daly et al, Nature Genetics 29:232-235 (2001). Haplotype association with the disease status can be performed using such blocks once they have been elucidated.

Haplotype association tests can be carried out in a similar fashion as the allelic and genotypic association tests. Each haplotype in a gene is analogous to an allele in a multi-allelic marker. One skilled in the art can either compare the haplotype frequencies in cases and controls or test genetic association with different pairs of haplotypes. It has been proposed that score tests can be done on haplotypes using the program "haplo-score." Schaid et al, Am J Hum Genet 70:425-434 (2002). In that method, haplotypes are first inferred by EM algorithm and score tests are carried out with a generalized linear model (GLM) framework that allows the adjustment of other factors.

An important decision in the performance of genetic association tests is the determination of the significance level at which significant association can be declared when the P value of the tests reaches that level. In an exploratory analysis where positive hits will be followed up in subsequent confirmatory testing, an unadjusted P value < 0.2 (a significance level on the lenient side), for example, may be used for generating hypotheses for significant association of a SNP with certain phenotypic characteristics of a disease. It is preferred that a p-value < 0.05 (a significance level traditionally used in the art) is achieved in order for a SNP to be considered to have an association with a disease. It is more preferred that a p-value <0.01 (a significance level on the stringent side) is achieved for an association to be declared. When hits are followed up in confirmatory analyses in more samples of the same source or in different samples from different sources, adjustment for multiple testing will be performed as to avoid excess number of hits while maintaining the experiment-wide error rates at 0.05. While there are different methods to adjust for multiple testing to control for different kinds of error rates, a commonly used but rather conservative method is Bonferroni correction to control the experiment-wise or family-wise error rate. Westfall et al., Multiple comparisons and multiple tests, SAS Institute (1999). Permutation tests to control for the false discovery rates, FDR, can be more powerful. Benjamini and Hochberg, Journal of the Royal Statistical Society, Series B 57:1289-1300 (1995); Westfall and Young, Resampling-based Multiple Testing, Wiley (1993). Such methods to control for multiplicity would be preferred when the tests are dependent and controlling for false discovery rates is sufficient as opposed to controlling for the experiment-wise error rates.

In replication studies using samples from different populations after statistically significant markers have been identified in the exploratory stage, meta-analyses can then be performed by combining evidence of different studies.

Modern Epidemiology 643-673, Lippincott, Williams & Wilkins (1998). If available, association results known in the art for the same SNPs can be included in the meta-analyses.

Since both genotyping and disease status classification can involve errors, sensitivity analyses may be performed to see how odds ratios and p-values would change upon various estimates on genotyping and disease classification error rates.

It has been well known that subpopulation-based sampling bias between cases and controls can lead to spurious results in case-control association studies when prevalence of the disease is associated with different subpopulation groups. Ewens and Spielman, Am J Hum Genet 62:450-458 (1995). Such bias can also lead to a loss of statistical power in genetic association studies. To detect population stratification, Pritchard and Rosenberg suggested typing markers that are unlinked to the disease and using results of association tests on those markers to determine whether there is any population stratification. Pritchard et al., Am J Hum Gen 65:220-228 (1999). When stratification is detected, the genomic control (GC) method as proposed by Devlin and Roeder can be used to adjust for the inflation of test statistics due to population stratification. Devlin et al., Biometrics 55:997-1004 (1999). The GC method is robust to changes in population structure levels as well as being applicable to DNA pooling designs. Devlin et al., Genet Epidem 21:273-284 (2001).

While Pritchard's method recommended using 15-20 unlinked microsatellite markers, it suggested using more than 30 biallelic markers to get enough power to detect population stratification. For the GC method, it has been shown that about 60-70 biallelic markers are sufficient to estimate the inflation factor for the test statistics due to population stratification. Bacanu et al., Am J Hum Genet 66:1933-1944 (2000). Hence, 70 intergenic SNPs can be chosen in unlinked regions as indicated in a genome scan. Kehoe et al., Hum Mol Genet 8:237-245 (1999).

Once individual risk factors, genetic or non-genetic, have been found for the predisposition to disease, the next step is to set up a classification/prediction scheme to predict the category (for instance, disease or no-disease) that an individual will be in depending on his genotypes of associated SNPs and other non-genetic risk factors. Logistic regression for discrete trait and linear regression for continuous trait are standard techniques for such tasks. Draper and Smith, Applied Regression Analysis, Wiley (1998). Moreover, other techniques can also be used for setting up classification. Such techniques include, but are not limited to, MART, CART, neural network, and discriminant analyses that are suitable for use in comparing the performance of different methods. The Elements of Statistical Learning, Hastie, Tibshirani & Friedman, Springer (2002).

For further information about genetic association studies, see Balding, "A tutorial on statistical methods for population association studies", Nature Reviews Genetics 7, 781 (2006).

Disease Diagnosis and Predisposition Screening

Information on association/correlation between genotypes and disease-related phenotypes can be exploited in several ways. For example, in the case of a highly statistically significant association between one or more SNPs with predisposition to a disease for which treatment is available, detection of such a genotype pattern in an individual may justify immediate administration of treatment, or at least the institution of regular monitoring of the individual. Detection of the susceptibility alleles associated with serious disease in a couple contemplating having children may also be valuable to the couple in their reproductive decisions. In the case of a weaker but still statistically significant association between a SNP and a human disease, immediate therapeutic intervention or monitoring may not be justified after detecting the susceptibility allele or SNP. Nevertheless, the subject can be motivated to begin simple life-style changes (*e.g.*, diet, exercise) that can be accomplished at little or no cost to the individual but would confer potential benefits in reducing the risk of developing conditions for which that individual may have an increased risk by virtue of having the risk allele(s).

The SNPs of the invention may contribute to responsiveness of an individual to statin treatment, or to the development of VT, in different ways. Some polymorphisms occur within a protein coding sequence and contribute to disease phenotype by affecting protein structure. Other polymorphisms occur in noncoding regions but may exert phenotypic effects indirectly via influence on, for example, replication, transcription, and/or translation. A single SNP may affect more than one phenotypic trait. Likewise, a single phenotypic trait may be affected by multiple SNPs in different genes.

As used herein, the terms "diagnose," "diagnosis," and "diagnostics" include, but are not limited to, any of the following: detection of VT that an individual may presently have, predisposition/susceptibility/predictive screening (*i.e.*, determining whether an individual has an increased or decreased risk of developing VT in the future), predicting recurrence of VT in an individual, determining a particular type or subclass of VT in an individual who currently or previously had VT, confirming or reinforcing a previously made diagnosis of VT, evaluating an individual's likelihood of responding positively to a particular treatment or therapeutic agent (*i.e.*, benefiting) such as statin treatment (particularly treatment or prevention of VT using statins), determining or selecting a therapeutic or preventive strategy that an individual is most likely to positively respond to (*e.g.*, selecting a particular therapeutic agent such as a statin, or combination of therapeutic agents, or selecting a particular statin from among other statins, or determining a dosing regimen or selecting a dosage formulation, etc.), classifying (or confirming/reinforcing) an individual as a responder/non-responder (or determining a particular subtype of responder/non-responder) with respect to the individual's response to a drug treatment such as statin treatment, and predicting whether a patient is likely to experience toxic effects from a particular treatment or therapeutic compound. Such diagnostic uses can be based on the SNPs individually or a unique combination or SNPs disclosed herein, as well as SNP haplotypes.

Haplotypes are particularly useful in that, for example, fewer SNPs can be genotyped to determine if a particular genomic region harbors a locus that influences a particular phenotype, such as in linkage disequilibrium-based SNP association analysis.

Linkage disequilibrium (LD) refers to the co-inheritance of alleles (*e.g.*, alternative nucleotides) at two or more different SNP sites at frequencies greater than would be expected from the separate frequencies of occurrence of each allele in a given population. The expected frequency of co-occurrence of two alleles that are inherited independently is the frequency of the first allele multiplied by the frequency of the second allele. Alleles that co-occur at expected frequencies are said to be in "linkage equilibrium." In contrast, LD refers to any non-random genetic association between allele(s) at two or more different SNP sites, which is generally due to the physical proximity of the two loci along a chromosome. LD can occur when two or more SNPs sites are in close physical proximity to each other on a given chromosome and therefore alleles at these SNP sites will tend to remain unseparated for multiple generations with the consequence that a particular nucleotide (allele) at one SNP site will show a non-random association with a particular nucleotide (allele) at a different SNP site located nearby. Hence, genotyping one of the SNP sites will give almost the same information as genotyping the other SNP site that is in LD.

Various degrees of LD can be encountered between two or more SNPs with the result being that some SNPs are more closely associated (*i.e.*, in stronger LD) than others. furthermore, the physical distance over which LD extends along a chromosome differs between different regions of the genome, and therefore the degree of physical separation between two or more SNP sites necessary for LD to occur can differ between different regions of the genome.

For diagnostic purposes and similar uses, if a particular SNP site is found to be useful for, for example, predicting an individual's response to statin treatment or an individual's susceptibility to VT, then the skilled artisan would recognize that other SNP sites which are in LD with this SNP site would also be useful for the same purposes. Thus, polymorphisms (*e.g.*, SNPs and/or haplotypes) that are not the actual disease-causing (causative) polymorphisms, but are in LD with such causative polymorphisms, are also useful. In such instances, the genotype of the polymorphism(s) that is/are in LD with the causative polymorphism is predictive of the genotype of the causative polymorphism and, consequently, predictive of the phenotype (*e.g.*, response to statin treatment or risk for developing VT) that is influenced by the causative SNP(s). Therefore, polymorphic markers that are in LD with causative polymorphisms are useful as diagnostic markers, and are particularly useful when the actual causative polymorphism(s) is/are unknown.

Examples of polymorphisms that can be in LD with one or more causative polymorphisms (and/or in LD with one or more polymorphisms that have a significant statistical association with a condition) and therefore useful for diagnosing the same condition that the causative/associated SNP(s) is used to diagnose, include other SNPs in the same gene, protein-coding, or mRNA transcript-coding region as the causative/associated SNP, other SNPs in the same exon or same intron as the causative/associated SNP, other SNPs in the same haplotype block as the causative/associated SNP, other SNPs in the same intergenic region as the causative/associated SNP, SNPs that are outside but near a gene (*e.g.*, within 6kb on either side, 5' or 3', of a gene boundary) that harbors a causative/associated SNP, etc. Such useful LD SNPs can be selected from among the SNPs disclosed in Table 3, for example.

Linkage disequilibrium in the human genome is reviewed in Wall et al., "Haplotype blocks and linkage disequilibrium in the human genome," Nat Rev Genet 4(8):587-97 (Aug. 2003); Garner et al., "On selecting markers for association studies: patterns of linkage disequilibrium between two and three diallelic loci," Genet Epidemiol 24(1):57-67 (Jan. 2003); Ardlie et al., "Patterns of linkage disequilibrium in the human genome," Nat Rev Genet 3(4):299-309 (Apr. 2002); erratum in Nat Rev Genet 3(7):566 (Jul. 2002); and Remm et al., "High-density genotyping and linkage disequilibrium in the human genome using chromosome 22 as a model," Curr Opin Chem Biol 6(1):24-30 (Feb. 2002); J.B.S. Haldane, "The combination of linkage values, and the calculation of distances between the loci of linked factors," J Genet 8:299-309 (1919); G. Mendel, Versuche über Pflanzen-Hybriden. Verhandlungen des naturforschenden Vereines in Brünn (Proceedings of the Natural History Society of Brünn) (1866); Genes IV, B. Lewin, ed., Oxford University Press, N.Y. (1990); D.L. Hartl and A.G. Clark Principles of Population Genetics 2nd ed., Sinauer Associates, Inc., Mass. (1989); J.H. Gillespie Population Genetics: A Concise Guide.2nd ed., Johns Hopkins University Press (2004) ; R.C. Lewontin, "The interaction of selection and linkage. I. General considerations; heterotic models," Genetics 49:49-67 (1964); P.G. Hoel, Introduction to Mathematical Statistics 2nd ed., John Wiley & Sons, Inc., N.Y. (1954); R.R. Hudson, "Two-locus sampling distributions and their application," Genetics 159:1805-1817 (2001); A.P. Dempster, N.M. Laird, D.B. Rubin, "Maximum likelihood from incomplete data via the EM algorithm," J R Stat Soc 39:1-38 (1977); L. Excoffier, M. Slatkin, "Maximum-likelihood estimation of molecular haplotype frequencies in a diploid population," Mol Biol Evol 12(5):921-927 (1995); D.A. Tregouet, S. Escolano, L. Tiret, A. Mallet, J.L. Golmard, "A new algorithm for haplotype-based association analysis: the Stochastic-EM algorithm," Ann Hum Genet 68(Pt 2):165-177 (2004); A.D. Long and C.H. Langley CH, "The power of association studies to detect the contribution of candidate genetic loci to variation in complex traits," Genome Research 9:720-731 (1999); A. Agresti, Categorical Data Analysis, John Wiley & Sons, Inc., N.Y. (1990); K. Lange, Mathematical and Statistical Methods for Genetic Analysis, Springer-Verlag New York, Inc., N.Y. (1997); The International HapMap Consortium, "The International HapMap Project," Nature 426:789-796 (2003); The International HapMap Consortium, "A haplotype map of the human genome," Nature 437:1299-1320 (2005); G.A. Thorisson, A.V. Smith, L. Krishnan, L.D. Stein, "The International HapMap Project Web Site," Genome Research 15:1591-1593 (2005); G. McVean, C.C.A. Spencer, R. Chaix, "Perspectives on human genetic variation from the HapMap project," PLoS Genetics 1(4):413-418 (2005); J.N. Hirschhorn, M.J. Daly, "Genome-wide association studies for common diseases and complex traits," Nat Genet 6:95-108 (2005); S.J. Schrodi, "A probabilistic approach to large-scale association scans: a semi-Bayesian method to detect disease-predisposing alleles," SAGMB 4(1):31 (2005); W.Y.S. Wang, B.J. Barratt, D.G. Clayton, J.A. Todd, "Genome-wide association studies: theoretical and practical concerns," Nat Rev Genet 6:109-118 (2005); J.K. Pritchard, M. Przeworski, "Linkage disequilibrium in humans: models and data," Am J Hum Genet 69:1-14 (2001).

As discussed above, disclosed herein are SNPs that are in LD with an interrogated SNP and which can also be used as valid markers for determining an individual's likelihood of benefiting from statin treatment, or whether an individual has an increased or decreased risk of having or developing VT. As used herein, the term "interrogated SNP" refers to SNPs that have been found to be associated with statin response, particularly for reducing VT risk, using genotyping results and analysis, or other appropriate experimental method as exemplified in the working examples described in this application. As used herein, the term "LD SNP" refers to a SNP that has been characterized as a SNP associated with statin response or an increased or decreased risk of VT due to their being in LD with the "interrogated SNP" under the methods of calculation described in the application. Below, applicants describe the methods of calculation with which one of ordinary skilled in the art may determine if a particular SNP is in LD with an interrogated SNP. The parameter $r^2$ is commonly used in the genetics art to characterize the extent of linkage disequilibrium between markers (Hudson, 2001). As used herein, the term "in LD with" refers to a particular SNP that is measured at above the threshold of a parameter such as $r^2$ with an interrogated SNP.

It is now common place to directly observe genetic variants in a sample of chromosomes obtained from a population. Suppose one has genotype data at two genetic markers located on the same chromosome, for the markers A and B. Further suppose that two alleles segregate at each of these two markers such that alleles $A_1$ and $A_2$ can be found at marker A and alleles $B_1$ and $B_2$ at marker B. Also assume that these two markers are on a human autosome. If one is to examine a specific individual and find that they are heterozygous at both markers, such that their two-marker genotype is $A_1A_2B_1B_2$, then there are two possible configurations: the individual in question could have the alleles $A_1B_1$ on one chromosome and $A_2B_2$ on the remaining chromosome; alternatively, the individual could have alleles $A_1B_2$ on one chromosome and $A_2B_1$ on the other. The arrangement of alleles on a chromosome is called a haplotype. In this illustration, the individual could have haplotypes $A_1B_1/A_2B_2$ or $A_1B_2/A_2B_1$ (see Hart1 and Clark (1989) for a more complete description). The concept of linkage equilibrium relates the frequency of haplotypes to the allele frequencies.

Assume that a sample of individuals is selected from a larger population. Considering the two markers described above, each having two alleles, there are four possible haplotypes: $A_1B_1$, $A_1B_2$, $A_2B_1$ and $A_2B_2$. Denote the frequencies of these four haplotypes with the following notation.

$$P_{11} = freq(A_1B_1) \tag{1}$$

$$P_{12} = freq(A_1B_2) \tag{2}$$

$$P_{21} = freq(A_2B_1) \tag{3}$$

$$P_{22} = freq(A_2B_2) \tag{4}$$

The allele frequencies at the two markers are then the sum of different haplotype frequencies, it is straightforward to write down a similar set of equations relating single-marker allele frequencies to two-marker haplotype frequencies:

$$p_1 = freq(A_1) = P_{11} + P_{12} \tag{5}$$

$$p_2 = freq(A_2) = P_{21} + P_{22} \tag{6}$$

$$q_1 = freq(B_1) = P_{11} + P_{21} \tag{7}$$

$$q_2 = freq(B_2) = P_{12} + P_{22} \tag{8}$$

Note that the four haplotype frequencies and the allele frequencies at each marker must sum to a frequency of 1.

$$P_{11} + P_{12} + P_{21} + P_{22} = 1 \tag{9}$$

$$p_1 + p_2 = 1 \tag{10}$$

$$q_1 + q_2 = 1 \tag{11}$$

If there is no correlation between the alleles at the two markers, one would expect that the frequency of the haplotypes would be approximately the product of the composite alleles. Therefore,

$$P_{11} \approx p_1 q_1 \tag{12}$$

$$P_{12} \approx p_1 q_2 \tag{13}$$

$$P_{21} \approx p_2 q_1 \tag{14}$$

$$P_{22} \approx p_2 q_2 \tag{15}$$

These approximating equations (12)-(15) represent the concept of linkage equilibrium where there is independent assortment between the two markers - the alleles at the two markers occur together at random. These are represented as approximations because linkage equilibrium and linkage disequilibrium are concepts typically thought of as properties of a sample of chromosomes; and as such they are susceptible to stochastic fluctuations due to the sampling process. Empirically, many pairs of genetic markers will be in linkage equilibrium, but certainly not all pairs.

Having established the concept of linkage equilibrium above, applicants can now describe the concept of linkage disequilibrium (LD), which is the deviation from linkage equilibrium. Since the frequency of the $A_1B_1$ haplotype is approximately the product of the allele frequencies for $A_1$ and $B_1$ under the assumption of linkage equilibrium as stated mathematically in (12), a simple measure for the amount of departure from linkage equilibrium is the difference in these two quantities, $D$ ,

$$D = P_{11} - p_1 q_1 \tag{16}$$

$D$=0 indicates perfect linkage equilibrium. Substantial departures from $D$=0 indicates LD in the sample of chromosomes examined. Many properties of $D$ are discussed in Lewontin (1964) including the maximum and minimum values that $D$ can take. Mathematically, using basic algebra, it can be shown that $D$ can also be written solely in terms of haplotypes:

$$D = P_{11}P_{22} - P_{12}P_{21} \tag{17}$$

If one transforms $D$ by squaring it and subsequently dividing by the product of the allele frequencies of $A_1$, $A_2$, $B_1$ and $B_2$, the resulting quantity, called $r^2$, is equivalent to the square of the Pearson's correlation coefficient commonly used in statistics (*e.g.*, Hoel, 1954).

$$r^2 = \frac{D^2}{p_1 p_2 q_1 q_2} \qquad (18)$$

As with $D$, values of $r^2$ close to 0 indicate linkage equilibrium between the two markers examined in the sample set. As values of $r^2$ increase, the two markers are said to be in linkage disequilibrium. The range of values that $r^2$ can take are from 0 to 1. $r^2 = 1$ when there is a perfect correlation between the alleles at the two markers.

In addition, the quantities discussed above are sample-specific. And as such, it is necessary to formulate notation specific to the samples studied. In the approach discussed here, three types of samples are of primary interest: (i) a sample of chromosomes from individuals affected by a disease-related phenotype (cases), (ii) a sample of chromosomes obtained from individuals not affected by the disease-related phenotype (controls), and (iii) a standard sample set used for the construction of haplotypes and calculation pairwise linkage disequilibrium. For the allele frequencies used in the development of the method described below, an additional subscript will be added to denote either the case or control sample sets.

$$p_{1,cs} = freq(A_1 \text{ in cases}) \qquad (19)$$

$$p_{2,cs} = freq(A_2 \text{ in cases}) \qquad (20)$$

$$q_{1,cs} = freq(B_1 \text{ in cases}) \qquad (21)$$

$$q_{2,cs} = freq(B_2 \text{ in cases}) \qquad (22)$$

Similarly,

$$p_{1,ct} = freq(A_1 \text{ in controls}) \qquad (23)$$

$$p_{2,ct} = freq(A_2 \text{ in controls}) \qquad (24)$$

$$q_{1,ct} = freq(B_1 \text{ in controls}) \qquad (25)$$

$$q_{2,ct} = freq(B_2 \text{ in controls}) \qquad (26)$$

As a well-accepted sample set is necessary for robust linkage disequilibrium calculations, data obtained from the International HapMap project (The International HapMap Consortium 2003, 2005; Thorisson et al, 2005; McVean et al, 2005) can be used for the calculation of pairwise $r^2$ values. Indeed, the samples genotyped for the International HapMap Project were selected to be representative examples from various human sub-populations with sufficient numbers of chromosomes examined to draw meaningful and robust conclusions from the patterns of genetic variation observed. The International HapMap project website (*hapmap.org*) contains a description of the project, methods utilized and samples examined. It is useful to examine empirical data to get a sense of the patterns present in such data.

Haplotype frequencies were explicit arguments in equation (18) above. However, knowing the 2-marker haplotype frequencies requires that phase to be determined for doubly heterozygous samples. When phase is unknown in the data examined, various algorithms can be used to infer phase from the genotype data. This issue was discussed earlier where the doubly heterozygous individual with a 2-SNP genotype of $A_1A_2B_1B_2$ could have one of two different sets of chromosomes: $A_1B_1/A_2B_2$ or $A_1B_2/A_2B_1$. One such algorithm to estimate haplotype frequencies is the expectation-maximization (EM) algorithm first formalized by Dempster *et al.* (1977). This algorithm is often used in genetics to infer haplotype frequencies from genotype data (*e.g.* Excoffier and Slatkin (1995); Tregouet *et al.* (2004)). It should he noted that for the two-SNP case explored here, EM algorithms have very little error provided that the allele frequencies and sample sizes are not too small. The impact on $r^2$ values is typically negligible.

As correlated genetic markers share information, interrogation of SNP markers in LD with a disease-associated SNP marker can also have sufficient power to detect disease association (Long and Langley (1999)). The relationship between the power to directly find disease-associated alleles and the power to indirectly detect disease-association was investigated by Pritchard and Przeworski (2001). In a straight-forward derivation, it can be shown that the power to detect disease association indirectly at a marker locus in linkage disequilibrium with a disease-association locus is approximately the same as the power to detect disease-association directly at the disease- association locus if the sample size is increased by a factor of $\dfrac{1}{r^2}$ (the reciprocal of equation 18) at the marker in comparison with the disease-association locus.

Therefore, if one calculated the power to detect disease-association indirectly with an experiment having $N$ samples, then equivalent power to directly detect disease-association (at the actual disease-susceptibility locus) would necessitate an experiment using approximately $r^2N$ samples. This elementary relationship between power, sample size and linkage disequilibrium can be used to derive an $r^2$ threshold value useful in determining whether or not genotyping markers in linkage disequilibrium with a SNP marker directly associated with disease status has enough power to indirectly detect disease-association.

To commence a derivation of the power to detect disease-associated markers through an indirect process, define the effective chromosomal sample size as

$$n = \frac{4N_{cs}N_{ct}}{N_{cs}+N_{ct}}; \tag{27}$$

where $N_{cs}$ and $N_{ct}$ are the numbers of diploid cases and controls, respectively. This is necessary to handle situations where the numbers of cases and controls are not equivalent. For equal case and control sample sizes, $N_{cs} = N_{ct} = N$, the value of the effective number of chromosomes is simply $n = 2N$ - as expected. Let power be calculated for a significance level $\alpha$ (such that traditional P-values below $\alpha$ will be deemed statistically significant). Define the standard Gaussian distribution function as $\Phi(\cdot)$. Mathematically,

$$\Phi(x) = \frac{1}{\sqrt{2\pi}} \int_{-\infty}^{x} e^{-\frac{\theta^2}{2}} d\theta \tag{28}$$

Alternatively, the following error function notation (Erf) may also be used,

$$\Phi(x) = \frac{1}{2}\left[1 + Erf\left(\frac{x}{\sqrt{2}}\right)\right] \tag{29}$$

For example, $\Phi(1.644854) = 0.95$. The value of $r^2$ may be derived to yield a prespecified minimum amount of power to detect disease association though indirect interrogation. Noting that the LD SNP marker could be the one that is carrying the disease-association allele, therefore that this approach constitutes a lower-bound model where all indirect power results are expected to be at least as large as those interrogated.

Denote by $\beta$ the error rate for not detecting truly disease-associated markers. Therefore, $1 - \beta$ is the classical definition of statistical power. Substituting the Pritchard-Pzreworski result into the sample size, the power to detect disease association at a significance level of $\alpha$ is given by the approximation

$$1 - \beta \cong \Phi\left[\frac{|q_{1,cs} - q_{1,ct}|}{\sqrt{\dfrac{q_{1,cs}(1 - q_{1,cs}) + q_{1,ct}(1 - q_{1,ct})}{r^2 n}}} - Z_{1-\alpha/2}\right]; \tag{30}$$

where $Z_u$ is the inverse of the standard normal cumulative distribution evaluated at $u$ ($u \in (0,1)$). $Z_u = \Phi^{-1}(u)$, where $\Phi(\Phi^{-1}(u)) = \Phi^{-1}(\Phi(u)) = u$. For example, setting $\alpha = 0.05$, and therefore $1 - \alpha/2 = 0.975$, one obtains $Z_{0.975} = 1.95996$.

Next, setting power equal to a threshold of a minimum power of *T*,

$$T = \Phi\left[\frac{|q_{1,cs} - q_{1,ct}|}{\sqrt{\dfrac{q_{1,cs}(1 - q_{1,cs}) + q_{1,ct}(1 - q_{1,ct})}{r^2 n}}} - Z_{1 - \alpha/2}\right] \qquad (31)$$

and solving for $r^2$, the following threshold $r^2$ is obtained:

$$r_T^2 = \frac{[q_{1,cs}(1 - q_{1,cs}) + q_{1,ct}(1 - q_{1,ct})]}{n(q_{1,cs} - q_{1,ct})^2}\left[\Phi^{-1}(T) + Z_{1 - \alpha/2}\right]^2 \qquad (32)$$

Or,

$$r_T^2 = \frac{\left(Z_T + Z_{1 - \alpha/2}\right)^2}{n}\left[\frac{q_{1,cs} - (q_{1,cs})^2 + q_{1,ct} - (q_{1,ct})^2}{(q_{1,cs} - q_{1,ct})^2}\right] \qquad (33)$$

Suppose that $r^2$ is calculated between an interrogated SNP and a number of other SNPs with varying levels of LD with the interrogated SNP. The threshold value $r_T^2$ is the minimum value of linkage disequilibrium between the interrogated SNP and the potential LD SNPs such that the LD SNP still retains a power greater or equal to *T* for detecting disease-association. For example, suppose that SNP rs200 is genotyped in a case-control disease-association study and it is found to be associated with a disease phenotype. Further suppose that the minor allele frequency in 1,000 case chromosomes was found to be 16% in contrast with a minor allele frequency of 10% in 1,000 control chromosomes. Given those measurements one could have predicted, prior to the experiment, that the power to detect disease association at a significance level of 0.05 was quite high - approximately 98% using a test of allelic association. Applying equation (32) one can calculate a minimum value of $r^2$ to indirectly assess disease association assuming that the minor allele at SNP rs200 is truly disease-predisposing for a threshold level of power. If one sets the threshold level of power to be 80%, then $r_T^2 = 0.489$ given the same significance level and chromosome numbers as above. Hence, any SNP with a pairwise $r^2$ value with rs200 greater than 0.489 is expected to have greater than 80% power to detect the disease association. Further, this is assuming the conservative model where the LD SNP is disease-associated only through linkage disequilibrium with the interrogated SNP rs200.

*Imputation*

Genotypes of SNPs can be imputed without actually having to be directly genotyped (referred to as "imputation"), by using known haplotype information. Imputation is a process to provide "missing" data, either missing individual genotypes or missing SNPs and concomitant genotypes, which have not been directly genotyped (i.e., assayed). Imputation is particularly useful for identifying disease associations for specific ungenotyped SNPs by inferring the missing genotypes to these ungenotyped SNPs. Although the process uses similar information to LD, since the phasing and imputation process uses information from multiple SNPs at the same time, the phased haplotype, it is able to infer the genotype and achieve high identifiable accuracy. Genotype information (such as from the HapMap project by The International HapMap Consortium) can be used to infer haplotype phase and impute genotypes for SNPs that are not directly genotyped in a given individual or sample set (such as for a disease association study). In general, imputation uses a reference dataset in which the genotypes of potential SNPs that are to be tested for disease association have been determined in multiple individuals (such as in HapMap); the individuals in the reference dataset are then haplotype phased. This phasing can be done with independent programs such as fastPHASE (Sheet and Stephens, Am J Hum Genet (2006) 76: 629-644) or a combination program such as BEAGLE which does both the phasing and the imputation. The reference phased haplotypes and process can be checked using the children of the HapMap individual parents, among other mechanisms. Once the reference phased haplotypes have been created, the imputation of additional individuals for SNPs genotyped or complete sets of SNPs that have not been directly genotyped can then proceed. The HapMap dataset is particularly useful as the reference dataset, however other datasets can be used. Since the imputation creates new concommitant phased haplotypes for individuals in the association study and these contain other SNPs within the genomic region, these ungenotyped but imputed SNPs can also be tested for disease assocations (or other traits). Certain exemplary methods for haplotype phase inference and imputation of missing genotypes utilize the BEAGLE genetic analysis program, (Browning, Hum Genet (2008) 124:439-450).

Thus, SNPs for which genotypes are imputed can be tested for association with a disease or other trait even though these SNPs are not directly genotyped. The SNPs for which genotypes are imputed have genotype data available in the reference dataset, e.g. HapMap individuals, but they are not directly genotyped in a particular individual or sample set (such as in a particular disease association study).

In addition to using a reference dataset (e.g., HapMap) to impute genotypes of SNPs that are not directly genotyped in a study, imputation can provide genotypes of SNPs that were directly genotyped in a study but for which the genotypes are missing, in some or most of the individuals, for some reason, such as because they failed to pass quality control. Imputation can also be used to combine genotyping results from multiple studies in which different sets of SNPs were genotyped to construct a complete meta-analysis. For example, genotyped and imputed genotyped SNP results from multiple different studies can be combined, and the overlapping SNP genotypes (e.g., genotyped in one study, imputed in another study or imputed in both or genotyped in both) can be analyzed across all of the studies (Browning, Hum Genet (2008) 124:439-450).

For a review of imputation (as well as the BEAGLE program), see Browning, "Missing data imputation and haplotype phase inference for genome-wide association studies", Hum Genet (2008) 124:439-450 and Browning et al. "A unified approach to genotype imputation and haplotype-phase inference for large data sets of trios and unrelated individuals", Am J Hum Genet. (2009) Feb; 84(2):210-23.

The contribution or association of particular SNPs with statin response or disease phenotypes, such as VT, enables the SNPs of the present disclosure to be used to develop superior diagnostic tests capable of identifying individuals who express a detectable trait, such as reduced risk for VT in response to statin treatment, as the result of a specific genotype, or individuals whose genotype places them at an increased or decreased risk of developing a detectable trait at a subsequent time as compared to individuals who do not have that genotype. As described herein, diagnostics may be based on a single SNP or a group of SNPs. Combined detection of a plurality of SNPs (for example, 2, 3, 4, 5, 6, 7, 8, 9, 10, 11, 12, 13, 14, 15, 16, 17, 18, 19. 20, 24, 25, 30, 32, 48, 50, 64, 96, 100, or any other number in-between, or more, of the SNPs provided in Table 1 and/or Table 2) typically increases the probability of an accurate diagnosis. For example, the presence of a single SNP known to correlate with VT might indicate a probability of 20% that an individual has or is at risk of developing VT, whereas detection of five SNPs, each of which correlates with VT, might indicate a probability of 80% that an individual has or is at risk of developing VT. To further increase the accuracy of diagnosis or predisposition screening, analysis of the SNPs of the present invention can be combined with that of other polymorphisms or other risk factors of VT, such as disease symptoms, pathological characteristics, family history, diet, environmental factors, or lifestyle factors.

It will be understood by practitioners skilled in the treatment or diagnosis of VT that the present invention generally does not intend to provide an absolute identification of individuals who benefit from statin treatment or individuals who are at risk (or less at risk) of developing VT, but rather to indicate a certain increased (or decreased) degree or likelihood of responding to statin therapy or developing VT based on statistically significant association results. However, this information is extremely valuable as it can be used to, for example, encourage individuals to comply with their statin regimens as prescribed by their doctors (even though the benefit of maintaining statin therapy may not be overtly apparent, which often leads to lack of compliance with prescribed statin treatment), to initiate preventive treatments or to allow an individual carrying one or more significant SNPs or SNP haplotypes to foresee warning signs such as minor clinical symptoms, or to have regularly scheduled physical exams to monitor for appearance of a condition in order to identify and begin treatment of the condition at an early stage. Particularly with diseases that are extremely debilitating or fatal if not treated on time, the knowledge of a potential predisposition, even if this predisposition is not absolute, would likely contribute in a very significant manner to treatment efficacy.

The diagnostic techniques of the present invention may employ a variety of methodologies to determine whether a test subject has a SNP or combination of SNPs associated with an increased or decreased risk of developing a detectable trait or whether the individual suffers from a detectable trait as a result of a particular polymorphism/mutation, including, for example, methods which enable the analysis of individual chromosomes for haplotyping, family studies, single sperm DNA analysis, or somatic hybrids. The trait analyzed using the diagnostics of the invention may be any detectable trait that is commonly observed in pathologies and disorders related to VT or drug response.

Another aspect disclosed herein relates to a method of determining whether an individual is at risk (or less at risk) of developing one or more traits or whether an individual expresses one or more traits as a consequence of possessing a particular trait-causing or trait-influencing allele. These methods generally involve obtaining a nucleic acid sample from an individual and assaying the nucleic acid sample to determine which nucleotide(s) is/are present at one or more SNP positions, wherein the assayed nucleotide(s) is/are indicative of an increased or decreased risk of developing the trait or indicative that the individual expresses the trait as a result of possessing a particular trait-causing or trait-influencing allele.

In another example, the SNP detection reagents disclosed herein are used to determine whether an individual has one or more SNP allele(s) affecting the level (*e.g.*, the concentration of mRNA or protein in a sample, etc.) or pattern (*e.g.*, the kinetics of expression, rate of decomposition, stability profile, Km, Vmax, etc.) of gene expression (collectively, the "gene response" of a cell or bodily fluid). Such a determination can be accomplished by screening for mRNA or protein expression (*e.g.*, by using nucleic acid arrays, RT-PCR, TaqMan assays, or mass spectrometry), identifying genes having altered expression in an individual, genotyping SNPs disclosed in Table 1 and/or Table 2 that could affect the expression of the genes having altered expression (*e.g.*, SNPs that are in and/or around the gene(s) having altered expression, SNPs in regulatory/control regions, SNPs in and/or around other genes that are involved in pathways that could affect the expression of the gene(s) having altered expression, or all SNPs could be genotyped), and correlating SNP genotypes with altered gene expression. In this manner, specific SNP alleles at particular SNP sites can be identified that affect gene expression.

Therapeutics, Pharmacogenomics, and Drug Development

*Therapeutic Methods and Compositions*

Disclosed herein are methods of assaying (*i.e.*, testing) one or more SNPs disclosed herein in an individual's nucleic acids, and administering a therapeutic or preventive agent to the individual based on the allele(s) present at the SNP(s) having indicated that the individual can benefit from the therapeutic or preventive agent.

Further, disclosed herein are methods of assaying one or more SNPs disclosed herein in an individual's nucleic acids, and administering a diagnostic agent (*e.g.*, an imaging agent), or otherwise carrying out further diagnostic procedures on the individual, based on the allele(s) present at the SNP(s) having indicated that the diagnostic agents or diagnostics procedures are justified in the individual.

In yet another example, disclosed herein is a pharmaceutical pack comprising a therapeutic agent (*e.g.*, a small molecule drug, antibody, peptide, antisense or RNAi nucleic acid molecule, etc.) and a set of instructions for administration of the therapeutic agent to an individual who has been tested for one or more SNPs disclosed herein.

*Pharmacogenomics*

Disclosed herein are methods for assessing the pharmacogenomics of a subject harboring particular SNP alleles or haplotypes to a particular therapeutic agent or pharmaceutical compound, or to a class of such compounds. Pharmacogenomics deals with the roles which clinically significant hereditary variations (*e.g.*, SNPs) play in the response to drugs due to altered drug disposition and/or abnormal action in affected persons. See, *e.g.*, Roses, Nature 405, 857-865 (2000); Gould Rothberg, Nature Biotechnology 19, 209-211 (2001); Eichelbaum, Clin Exp Pharmacol Physiol 23(10-11):983-985 (1996); and Linder, Clin Chem 43(2):254-266 (1997). The clinical outcomes of these variations can result in severe toxicity of therapeutic drugs in certain individuals or therapeutic failure of drugs in certain individuals as a result of individual variation in metabolism. Thus, the SNP genotype of an individual can determine the way a therapeutic compound acts on the body or the way the body metabolizes the compound. For example, SNPs in drug metabolizing enzymes can affect the activity of these enzymes, which in turn can affect both the intensity and duration of drug action, as well as drug metabolism and clearance.

The discovery of SNPs in drug metabolizing enzymes, drug transporters, proteins for pharmaceutical agents, and other drug targets has explained why some patients do not obtain the expected drug effects, show an exaggerated drug effect, or experience serious toxicity from standard drug dosages. SNPs can be expressed in the phenotype of the extensive metabolizer and in the phenotype of the poor metabolizer. Accordingly, SNPs may lead to allelic variants of a protein in which one or more of the protein functions in one population are different from those in another population. SNPs and the encoded variant peptides thus provide targets to ascertain a genetic predisposition that can affect treatment modality. For example, in a ligand-based treatment, SNPs may give rise to amino terminal extracellular domains and/or other ligand-binding regions of a receptor that are more or less active in ligand binding, thereby affecting subsequent protein activation. Accordingly, ligand dosage would necessarily be modified to maximize the therapeutic effect within a given population containing particular SNP alleles or haplotypes.

As an alternative to genotyping, specific variant proteins containing variant amino acid sequences encoded by alternative SNP alleles could be identified. Thus, pharmacogenomic characterization of an individual permits the selection of effective compounds and effective dosages of such compounds for prophylactic or therapeutic uses based on the individual's SNP genotype, thereby enhancing and optimizing the effectiveness of the therapy. Furthermore, the production of recombinant cells and transgenic animals containing particular SNPslhaplotypes allow effective clinical design and testing of treatment compounds and dosage regimens. For example, transgenic animals can be produced that differ only in specific SNP alleles in a gene that is orthologous to a human disease susceptibility gene.

Pharmacogenomic uses of the SNPs of the present invention provide several significant advantages for patient care, particularly in predicting an individual's responsiveness to statin treatment (particularly for reducing the risk of VT) and in predicting an individual's predisposition to VT. Pharmacogenomic characterization of an individual, based on an individual's SNP genotype, can identify those individuals unlikely to respond to treatment with a particular medication and thereby allows physicians to avoid prescribing the ineffective medication to those individuals. On the other hand, SNP genotyping of an individual may enable physicians to select the appropriate medication and dosage regimen that will be most effective based on an individual's SNP genotype. This information increases a physician's confidence in prescribing medications and motivates patients to comply with their drug regimens. Furthermore, pharmacogenomics may identify patients predisposed to toxicity and adverse reactions to particular drugs or drug dosages. Adverse drug reactions lead to more than 100.000 avoidable deaths per year in the United States alone and therefore represent a significant cause of hospitalization and death, as well as a significant economic burden on the healthcare system (Pfost et al., Trends in Biotechnology, Aug. 2000.). Thus, pharmacogenomics based on the SNPs disclosed herein has the potential to both save lives and reduce healthcare costs substantially.

Pharmacogenomics in general is discussed further in Rose et al., "Pharmacogenetic analysis of clinically relevant genetic polymorphisms," Methods Mol Med 85:225-37 (2003). Pharmacogenomics as it relates to Alzheimer's disease and other neurodegenerative disorders is discussed in Cacabelos, "Pharmacogenomics for the treatment of dementia," Ann Med 34(5):357-79 (2002); Maimone et al., "Pharmacogenomics of neurodegenerative diseases," Eur J Pharmacol 413(1):11-29 (Feb. 2001); and Poirier, "Apolipoprotein E: a pharmacogenetic target for the treatment of Alzheimer's disease," Mol Diagn 4(4):335-41 (Dec.1999). Pharmacogenomics as it relates to cardiovascular disorders is discussed in Siest et al., "Pharmacogenomics of drugs affecting the cardiovascular system," Clin Chem Lab Med 41(4):590-9 (Apr. 2003); Mukherjee et al., "Pharmacogenomics in cardiovascular diseases," Prog Cardiovasc Dis 44(6):479-98 (May-Jun. 2002); and Mooser et al., "Cardiovascular pharmacogenetics in the SNP era," J Thromb Haemost 1(7):1398-402 (Jul. 2003). Pharmacogenomics as it relates to cancer is discussed in McLeod et al., "Cancer pharmacogenomics: SNPs, chips, and the individual patient," Cancer Invest 21(4):630-40 (2003); and Watters et al., "Cancer pharmacogenomics: current and future applications," Biochim Biophys Acta 1603(2):99-111 (Mar. 2003).

*Clinical Trials*

In certain aspects of the invention, there are provided methods of using the SNPs disclosed herein to identify or stratify patient populations for clinical trials of a therapeutic, preventive, or diagnostic agent.

For instance, an aspect of the present invention includes selecting individuals for clinical trials based on their SNP genotype, such as selecting individuals for inclusion in a clinical trial and/or assigning individuals to a particular group within a clinical trial (e.g., an "arm" or "cohort" of the trial). For example, individuals with SNP genotypes that indicate that they are likely to positively respond to a drug can be included in the trials, whereas those individuals whose SNP genotypes indicate that they are less likely to or would not respond to the drug, or who are at risk for suffering toxic effects or other adverse reactions, can be excluded from the clinical trials. This not only can improve the safety of clinical trials, but also can enhance the chances that the trial will demonstrate statistically significant efficacy. Further, one can stratify a prospective trial with patients with different SNP variants to determine the impact of differential drug treatment.

Thus, disclosed herein are methods for conducting a clinical trial of a therapeutic agent in which a human is selected for inclusion in the clinical trial and/or assigned to a particular group within a clinical trial based on the presence or absence of one or more SNPs disclosed herein. In certain embodiments, the therapeutic agent is a statin.

In certain examples, the SNPs disclosed herein can be used to select individuals who are unlikely to respond positively to a particular therapeutic agent (or class of therapeutic agents) based on their SNP genotype(s) to participate in a clinical trial of another type of drug that may benefit them. Thus, in certain examples, the SNPs disclosed herein can be used to identify patient populations who do not adequately respond to current treatments and are therefore in need of new therapies. This not only benefits the patients themselves, but also benefits organizations such as pharmaceutical companies by enabling the identification of populations that represent markets for new drugs, and enables the efficacy of these new drugs to be tested during clinical trials directly in individuals within these markets.

The SNP-containing nucleic acid molecules disclosed herein are also useful for monitoring the effectiveness of modulating compounds on the expression or activity of a variant gene, or encoded product, particularly in a treatment regimen or in clinical trials. Thus, the gene expression pattern can serve as an indicator for the continuing effectiveness of treatment with the compound, particularly with compounds to which a patient can develop resistance, as well as an indicator for toxicities. The gene expression pattern can also serve as a marker indicative of a physiological response of the affected cells to the compound. Accordingly, such monitoring would allow either increased administration of the compound or the administration of alternative compounds to which the patient has not become resistant.

Furthermore, the SNPs disclosed herein may have utility in determining why certain previously developed drugs performed poorly in clinical trials and may help identify a subset of the population that would benefit from a drug that had previously performed poorly in clinical trials, thereby "rescuing" previously developed drugs, and enabling the drug to be made available to a particular patient population (*e.g.*, particular VT patients) that can benefit from it.

*Identification, Screening, and Use of Therapeutic Agents*

The SNPs disclosed herein also can be used to identify novel therapeutic targets for VT. For example, genes containing the disease-associated variants ("variant genes") or their products, as well as genes or their products that are directly or indirectly regulated by or interacting with these variant genes or their products, can be targeted for the development of therapeutics that, for example, treat the disease or prevent or delay disease onset. The therapeutics may be composed of, for example, small molecules, proteins, protein fragments or peptides, antibodies, nucleic acids, or their derivatives or mimetics which modulate the functions or levels of the target genes or gene products.

Disclosed herein are also methods for identifying a compound or agent that can be used to treat VT. The SNPs disclosed herein are useful as targets for the identification and/or development of therapeutic agents. A method for identifying a therapeutic agent or compound typically includes assaying the ability of the agent or compound to modulate the activity and/or expression of a SNP-containing nucleic acid or the encoded product and thus identifying an agent or a compound that can be used to treat a disorder characterized by undesired activity or expression of the SNP-containing nucleic acid or the encoded product. The assays can be performed in cell-based and cell-free systems. Cell-based assays can include cells naturally expressing the nucleic acid molecules of interest or recombinant cells genetically engineered to express certain nucleic acid molecules.

Variant gene expression in a VT patient can include, for example, either expression of a SNP-containing nucleic acid sequence (for instance, a gene that contains a SNP can be transcribed into an mRNA transcript molecule containing the SNP, which can in turn be translated into a variant protein) or altered expression of a normal/wild-type nucleic acid sequence due to one or more SNPs (for instance, a regulatory/control region can contain a SNP that affects the level or pattern of expression of a normal transcript).

Assays for variant gene expression can involve direct assays of nucleic acid levels (*e.g.,* mRNA levels), expressed protein levels, or of collateral compounds involved in a signal pathway. Further, the expression of genes that are up- or down-regulated in response to the signal pathway can also be assayed. In this embodiment, the regulatory regions of these genes can be operably linked to a reporter gene such as luciferase.

Modulators of variant gene expression can be identified in a method wherein, for example, a cell is contacted with a candidate compound/agent and the expression of mRNA determined. The level of expression of mRNA in the presence of the candidate compound is compared to the level of expression of mRNA in the absence of the candidate compound. The candidate compound can then be identified as a modulator of variant gene expression based on this comparison and be used to treat a disorder such as VT that is characterized by variant gene expression (*e.g.*, either expression of a SNP-containing nucleic acid or altered expression of a nornml/wild-type nucleic acid molecule due to one or more SNPs that affect expression of the nucleic acid molecule) due to one or more SNPs disclosed herein. When expression of mRNA is statistically significantly greater in the presence of the candidate compound than in its absence, the candidate compound is identified as a stimulator of nucleic acid expression. When nucleic acid expression is statistically significantly less in the presence of the candidate compound than in its absence, the candidate compound is identified as an inhibitor of nucleic acid expression.

Further, disclosed herein are methods of treatment, with the SNP or associated nucleic acid domain (*e.g.*, catalytic domain, ligand/substrate-binding domain, regulatory/control region, etc.) or gene, or the encoded mRNA transcript, as a target, using a compound identified through drug screening as a gene modulator to modulate variant nucleic acid expression. Modulation can include either up-regulation (*i.e.*, activation or agonization) or down-regulation (*i.e.*, suppression or antagonization) of nucleic acid expression.

Expression of mRNA transcripts and encoded proteins, either wild type or variant, may be altered in individuals with a particular SNP allele in a regulatory/control element, such as a promoter or transcription factor binding domain, that regulates expression. In this situation, methods of treatment and compounds can be identified, as discussed herein, that regulate or overcome the variant regulatory/control element, thereby generating normal, or healthy, expression levels of either the wild type or variant protein.

*Pharmaceutical Compositions and Administration Thereof*

Any of the statin response-associated proteins, and encoding nucleic acid molecules, disclosed herein can be used as therapeutic targets (or directly used themselves as therapeutic compounds) for treating or preventing VT, and the present disclosure enables therapeutic compounds (*e.g.*, small molecules, antibodies, therapeutic proteins, RNAi and antisense molecules, etc.) to be developed that target (or are comprised of) any of these therapeutic targets.

In general, a therapeutic compound will be administered in a therapeutically effective amount by any of the accepted modes of administration for agents that serve similar utilities. The actual amount of the therapeutic compound disclosed herein, *i.e.,* the active ingredient, will depend upon numerous factors such as the severity of the disease to be treated, the age and relative health of the subject, the potency of the compound used, the route and form of administration, and other factors.

Therapeutically effective amounts of therapeutic compounds may range from, for example, approximately 0.01-50 mg per kilogram body weight of the recipient per day; preferably about 0.1-20 mg/kg/day. Thus, as an example, for administration to a 70-kg person, the dosage range would most preferably be about 7 mg to 1.4g per day.

In general, therapeutic compounds will be administered as pharmaceutical compositions by any one of the following routes: oral, systemic (*e.g.*, transdermal, intranasal, or by suppository), or parenteral (*e.g.*, intramuscular, intravenous, or subcutaneous) administration. The preferred manner of administration is oral or parenteral using a convenient daily dosage regimen, which can be adjusted according to the degree of affliction. Oral compositions can take the form of tablets, pills, capsules, semisolids, powders, sustained release formulations, solutions, suspensions, elixirs, aerosols, or any other appropriate compositions.

The choice of formulation depends on various factors such as the mode of drug administration (*e.g.*, for oral administration, formulations in the form of tablets, pills, or capsules are preferred) and the bioavailability of the drug substance. Recently, pharmaceutical formulations have been developed especially for drugs that show poor bioavailability based upon the principle that bioavailability can be increased by increasing the surface area, *i.e.,* decreasing particle size. For example, U.S. Patent No. 4, 107,288 describes a pharmaceutical formulation having particles in the size range from 10 to 1,000 nm in which the active material is supported on a cross-linked matrix of macromolecules. U.S. Patent No. 5,145,684 describes the production of a pharmaceutical formulation in which the drug substance is pulverized to nanoparticles (average particle size of 400 nm) in the presence of a surface modifier and then dispersed in a liquid medium to give a pharmaceutical formulation that exhibits remarkably high bioavailability.

Pharmaceutical compositions are comprised of, in general, a therapeutic compound in combination with at least one pharmaceutically acceptable excipient. Acceptable excipients are non-toxic, aid administration, and do not adversely affect the therapeutic benefit of the therapeutic compound. Such excipients may be any solid, liquid, semi-solid or, in the case of an aerosol composition, gaseous excipient that is generally available to one skilled in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, animal, vegetable or synthetic origin, *e.g.,* peanut oil, soybean oil, mineral oil, sesame oil, etc. Preferred liquid carriers, particularly for injectable solutions, include water, saline, aqueous dextrose, and glycols.

Compressed gases may be used to disperse a compound of the present disclosure in aerosol form. Inert gases suitable for this purpose are nitrogen, carbon dioxide, etc.

Other suitable pharmaceutical excipients and their formulations are described in Remington's Pharmaceutical Sciences 18th ed., E.W. Martin, ed., Mack Publishing Company (1990).

The amount of the therapeutic compound in a formulation can vary within the full range employed by those skilled in the art. Typically, the formulation will contain, on a weight percent (wt %) basis, from about 0.01-99.99 wt % of the therapeutic compound based on the total formulation, with the balance being one or more suitable pharmaceutical excipients. Preferably, the compound is present at a level of about 1-80% wt.

Therapeutic compounds can be administered alone or in combination with other therapeutic compounds or in combination with one or more other active ingredients). For example, an inhibitor or stimulator of a VT-associated protein can be administered in combination with another agent that inhibits or stimulates the activity of the same or a different VT-associated protein to thereby counteract the effects of VT.

For further information regarding pharmacology, see Current Protocols in Pharmacology, John Wiley & Sons, Inc., N.Y.

*Nucleic Acid-Based Therapeutic Agents*

The SNP-containing nucleic acid molecules disclosed herein, and their complementary nucleic acid molecules, may be used as antisense constructs to control gene expression in cells, tissues, and organisms. Antisense technology is well established in the art and extensively reviewed in Antisense Drug Technology: Principles, Strategies, and Applications, Crooke, ed., Marcel Dekker, Inc., N.Y. (2001). An antisense nucleic acid molecule is generally designed to be complementary to a region of mRNA expressed by a gene so that the antisense molecule hybridizes to the mRNA and thereby blocks translation of mRNA into protein. Various classes of antisense oligonucleotides are used in the art, two of which are cleavers and blockers. Cleavers, by binding to target RNAs, activate intracellular nucleases (*e.g.*, RNaseH or RNase L) that cleave the target RNA. Blockers, which also bind to target RNAs, inhibit protein translation through steric hindrance of ribosomes. Exemplary blockers include peptide nucleic acids, morpholinos, locked nucleic acids, and methylphosphonates. See, *e.g.*, Thompson, Drug Discovery Today 7(17): 912-917 (2002). Antisense oligonucleotides are directly useful as therapeutic agents, and are also useful for determining and validating gene function (*e.g.*, in gene knock-out or knock-down experiments).

Antisense technology is further reviewed in: Lavery et al., "Antisense and RNAi: powerful tools in drug target discovery and validation," Curr Opin Drug Discov Devel 6(4):561-9 (Jul. 2003); Stephens et al., "Antisense oligonucleotide therapy in cancer," Curr Opin Mol Ther 5(2): 118-22 (Apr. 2003); Kurreck, "Antisense technologies. Improvement through novel chemical modifications," Eur J Biochem 270(8): 1628-44 (Apr. 2003); Dias et al., "Antisense oligonucleotides: basic concepts and mechanisms," Mol Cancer Ther 1(5);347-55 (Mar. 2002); Chen, "Clinical development of antisense oligonucleotides as anti-cancer therapeutics," Methods Mol Med 75:621-36 (2003); Wang et al., "Antisense anticancer oligonucleotide therapeutics," Curr Cancer Drug Targets 1(3):177-96 (Nov. 2001); and Bennett, "Efficiency of antisense oligonucleotide drug discovery," Antisense Nucleic Acid Drug Dev 12(3):215-24 (Jun. 2002).

The SNPs disclosed herein are particularly useful for designing antisense reagents that are specific for particular nucleic acid variants. Based on the SNP information disclosed herein, antisense oligonucleotides can be produced that specifically target mRNA molecules that contain one or more particular SNP nucleotides. In this manner, expression of mRNA molecules that contain one or more undesired polymorphisms (*e.g.*, SNP nucleotides that lead to a defective protein such as an amino acid substitution in a catalytic domain) can be inhibited or completely blocked. Thus, antisense oligonucleotides can be used to specifically bind a particular polymorphic form (*e.g.*, a SNP allele that encodes a defective protein), thereby inhibiting translation of this form, but which do not bind an alternative polymorphic form (*e.g.*, an alternative SNP nucleotide that encodes a protein having normal function).

Antisense molecules can be used to inactivate mRNA in order to inhibit gene expression and production of defective proteins. Accordingly, these molecules can be used to treat a disorder, such as VT, characterized by abnormal or undesired gene expression or expression of certain defective proteins. This technique can involve cleavage by means of ribozymes containing nucleotide sequences complementary to one or more regions in the mRNA that attenuate the ability of the mRNA to be translated. Possible mRNA regions include, for example, protein-coding regions and particularly protein-coding regions corresponding to catalytic activities, substrate/ligand binding, or other functional activities of a protein.

The SNPs disclosed herein are also useful for designing RNA interference reagents that specifically target nucleic acid molecules having particular SNP variants. RNA interference (RNAi), also referred to as gene silencing, is based on using double-stranded RNA (dsRNA) molecules to turn genes off. When introduced into a cell, dsRNAs are processed by the cell into short fragments (generally about 21, 22, or 23 nucleotides in length) known as small interfering RNAs (siRNAs) which the cell uses in a sequence-specific manner to recognize and destroy complementary RNAs. Thompson, Drug Discovery Today 7(17): 912-917 (2002). Accordingly, the present disclosure contemplates isolated nucleic acid molecules that are about 18-26 nucleotides in length, preferably 19-25 nucleotides in length, and more preferably 20, 21, 22, or 23 nucleotides in length, and the use of these nucleic acid molecules for RNAi. Because RNAi molecules, including siRNAs, act in a sequence-specific manner, the SNPs disclosed herein can be used to design RNAi reagents that recognize and destroy nucleic acid molecules having specific SNP alleles/nucleotides (such as deleterious alleles that lead to the production of defective proteins), while not affecting nucleic acid molecules having alternative SNP alleles (such as alleles that encode proteins having normal function). As with antisense reagents, RNAi reagents may be directly useful as therapeutic agents (*e.g.*, for turning off defective, disease-causing genes), and are also useful for characterizing and validating gene function (*e.g.*, in gene knock-out or knock-down experiments).

The following references provide a further review of RNAi: Reynolds et al., "Rational siRNA design for RNA interference," Nat Biotechnol 22(3):326-30 (Mar. 2004); Epub Feb. 1, 2004; Chi et al., "Genomewide view of gene silencing by small interfering RNAs," PNAS 100(11):6343-6346 (2003); Vickers et al, "Efficient Reduction of Target RNAs by Small Interfering RNA and RNase H-dependent Antisense Agents," J Biol Chem 278:7108-7118 (2003); Agami, "RNAi and related mechanisms and their potential use for therapy," Curr Opin Chem Biol 6(6):829-34 (Dec. 2002); Lavery et al., "Antisense and RNAi: powerful tools in drug target discovery and validation," Curr Opin Drug Discov Devel 6(4):561-9 (Jul. 2003); Shi, "Mammalian RNAi for the masses," Trends Genet 19(1):9-12 (Jan. 2003); Shuey et al., "RNAi: gene-silencing in therapeutic intervention," Drug Discovery Today 7(20):104-1046 (Oct. 2002); McManus et al., Nat Rev Genet 3(10):737-47 (Oct. 2002); Xia et al., Nat Biotechnol 20(10):1006-10 (Oct. 2002); Plasterk et al, Curr Opin Genet Dev 10(5):562-7 (Oct. 2000); Bosher et al., Nat Cell Biol 2(2):E31 -6 (Feb. 2000); and Hunter, Curr Biol 17; 9(12): R440-2 (Jun. 1999).

*Other Therapeutic Aspects*

SNPs have many important uses in drug discovery, screening, and development, and thus the SNPs disclosed herein are useful for improving many different aspects of the drug development process.

For example, a high probability exists that, for any gene/protein selected as a potential drug target, variants of that gene/protein will exist in a patient population. Thus, determining the impact of gene/protein variants on the selection and delivery of a therapeutic agent should be an integral aspect of the drug discovery and development process.

Jazwinska, A Trends Guide to Genetic Variation and Genomic Medicine S30-S36 (Mar. 2002).

Knowledge of variants (*e.g.*, SNPs and any corresponding amino acid polymorphisms) of a particular therapeutic target (*e.g.*, a gene, mRNA transcript, or protein) enables parallel screening of the variants in order to identify therapeutic candidates (*e.g.*, small molecule compounds, antibodies, antisense or RNAi nucleic acid compounds, etc.) that demonstrate efficacy across variants. Rothberg, Nat Biotechnol 19(3);209-11 (Mar. 2001). Such therapeutic candidates would be expected to show equal efficacy across a larger segment of the patient population, thereby leading to a larger potential market for the therapeutic candidate.

Furthermore, identifying variants of a potential therapeutic target enables the most common form of the target to be used for selection of therapeutic candidates, thereby helping to ensure that the experimental activity that is observed for the selected candidates reflects the real activity expected in the largest proportion of a patient population. Jazwinska, A Trends Guide Genetic Variation and Genomic Medicine S30-S36 (Mar. 2002).

Additionally, screening therapeutic candidates against all known variants of a target can enable the early identification of potential toxicities and adverse reactions relating to particular variants. For example, variability in drug absorption, distribution, metabolism and excretion (ADME) caused by, for example, SNPs in therapeutic targets or drug metabolizing genes, can be identified, and this information can be utilized during the drug development process to minimize variability in drug disposition and develop therapeutic agents that are safer across a wider range of a patient population. The SNPs disclosed herein including the variant proteins and encoding polymorphic nucleic acid molecules provided in Tables 1 and 2, are useful in conjunction with a variety of toxicology methods established in the art, such as those set forth in Current Protocols in Toxicology, John Wiley & Sons, Inc., N.Y.

Furthermore, therapeutic agents that target any art-known proteins (or nucleic acid molecules, either RNA or DNA) may cross-react with the variant proteins (or polymorphic nucleic acid molecules) disclosed in Table 1. thereby significantly affecting the pharmacokinetic properties of the drug. Consequently, the protein variants and the SNP-containing nucleic acid molecules disclosed in Tables 1 and 2 are useful in developing, screening, and evaluating therapeutic agents that target corresponding art-known protein forms (or nucleic acid molecules). Additionally, as discussed above, knowledge of all polymorphic forms of a particular drug target enables the design of therapeutic agents that are effective against most or all such polymorphic forms of the drug target.

A subject suffering from a pathological condition ascribed to a SNP, such as VT, may be treated so as to correct the genetic defect. See Kren et al., Proc Natl Acad Sci USA 96:10349-10354 (1999). Such a subject can be identified by any method that can detect the polymorphism in a biological sample drawn from the subject. Such a genetic defect may be permanently corrected by administering to such a subject a nucleic acid fragment incorporating a repair sequence that supplies the normal/wild-type nucleotide at the position of the SNP. This site-specific repair sequence can encompass an RNA/DNA oligonucleotide that operates to promote endogenous repair of a subject's genomic DNA. The site-specific repair sequence is administered in an appropriate vehicle, such as a complex with polyethylenimine, encapsulated in anionic liposomes, a viral vector such as an adenovirus, or other pharmaceutical composition that promotes intracellular uptake of the administered nucleic acid. A genetic defect leading to an inborn pathology may then be overcome, as the chimeric oligonucleotides induce incorporation of the normal sequence into the subject's genome. Upon incorporation, the normal gene product is expressed, and the replacement is propagated, thereby engendering a permanent repair and therapeutic enhancement of the clinical condition of the subject.

In cases in which a cSNP results in a variant protein that is ascribed to be the cause of, or a contributing factor to, a pathological condition, a method of treating such a condition can include administering to a subject experiencing the pathology the wild-type/normal cognate of the variant protein. Once administered in an effective dosing regimen, the wild-type cognate provides complementation or remediation of the pathological condition.

## VARIANT PROTEINS, ANTIBODIES, VECTORS, HOST CELLS, & USES THEREOF

### Variant Proteins Encoded bv SNP-Containing Nucleic Acid Molecules

Disclosed herein are also SNP-containing nucleic acid molecules, many of which encode proteins having variant amino acid sequences as compared to the art-known (*i.e.*, wild-type) proteins. Amino acid sequences encoded by the polymorphic nucleic acid molecules disclosed herein are referred to as SEQ ID NOS:85-168 in Table 1 and provided in the Sequence Listing. These variants will generally be referred to herein as variant proteins/peptides/polypeptides, or polymorphic proteins/peptides/polypeptides. The terms "protein," "peptide," and "polypeptide" are used herein interchangeably.

A variant protein may be encoded by, for example, a nonsynonymous nucleotide substitution at any one of the cSNP positions disclosed herein. In addition, variant proteins may also include proteins whose expression, structure, and/or function is altered by a SNP disclosed herein, such as a SNP that creates or destroys a stop codon, a SNP that affects splicing, and a SNP in control/regulatory elements, *e.g.* promoters, enhancers, or transcription factor binding domains.

As used herein, a protein or peptide is said to be "isolated" or "purified" when it is substantially free of cellular material or chemical precursors or other chemicals. The variant proteins disclosed herein can be purified to homogeneity or other lower degrees of purity. The level of purification will be based on the intended use. The key feature is that the preparation allows for the desired function of the variant protein, even if in the presence of considerable amounts of other components.

As used herein, "substantially free of cellular material" includes preparations of the variant protein having less than about 30% (by dry weight) other proteins (*i.e.*, contaminating protein), less than about 20% other proteins, less than about 10% other proteins, or less than about 5% other proteins. When the variant protein is recombinantly produced, it can also be substantially free of culture medium, *i.e.*, culture medium represents less than about 20% of the volume of the protein preparation.

The language "substantially free of chemical precursors or other chemicals" includes preparations of the variant protein in which it is separated from chemical precursors or other chemicals that are involved in its synthesis. In one embodiment, the language "substantially free of chemical precursors or other chemicals" includes preparations of the variant protein having less than about 30% (by dry weight) chemical precursors or other chemicals, less than about 20% chemical precursors or other chemicals, less than about 10% chemical precursors or other chemicals, or less than about 5% chemical precursors or other chemicals.

An isolated variant protein may be purified from cells that naturally express it, purified from cells that have been altered to express it (recombinant host cells), or synthesized using known protein synthesis methods. For example, a nucleic acid molecule containing SNP(s) encoding the variant protein can be cloned into an expression vector, the expression vector introduced into a host cell, and the variant protein expressed in the host cell. The variant protein can then be isolated from the cells by any appropriate purification scheme using standard protein purification techniques. Examples of these techniques are described in detail below, Sambrook and Russell, Molecular Cloning: A Laboratory Manual, Cold Spring Harbor Laboratory Press, N.Y. (2000).

Disclosed herein are isolated variant proteins that comprise, consist of or consist essentially of amino acid sequences that contain one or more variant amino acids encoded by one or more codons that contain a SNP disclosed herein.

Accordingly, the present disclosure discribes variant proteins that consist of amino acid sequences that contain one or more amino acid polymorphisms (or truncations or extensions due to creation or destruction of a stop codon, respectively) encoded by the SNPs provided in Table 1 and/or Table 2. A protein consists of an amino acid sequence when the amino acid sequence is the entire amino acid sequence of the protein.

The present disclosure further describes variant proteins that consist essentially of amino acid sequences that contain one or more amino acid polymorphisms (or truncations or extensions due to creation or destruction of a stop codon, respectively) encoded by the SNPs provided in Table 1 and/or Table 2. A protein consists essentially of an amino acid sequence when such an amino acid sequence is present with only a few additional amino acid residues in the final protein.

Futher, disclosed herein are variant proteins that comprise amino acid sequences that contain one or more amino acid polymorphisms (or truncations or extensions due to creation or destruction of a stop codon, respectively) encoded by the SNPs provided in Table 1 and/or Table 2. A protein comprises an amino acid sequence when the amino acid sequence is at least part of the final amino acid sequence of the protein. In such a fashion, the protein may contain only the variant amino acid sequence or have additional amino acid residues, such as a contiguous encoded sequence that is naturally associated with it or heterologous amino acid residues. Such a protein can have a few additional amino acid residues or can comprise many more additional amino acids. A brief description of how various types of these proteins can be made and isolated is provided below.

The variant proteins disclosed herein can be attached to heterologous sequences to form chimeric or fusion proteins. Such chimeric and fusion proteins comprise a variant protein operatively linked to a heterologous protein having an amino acid sequence not substantially homologous to the variant protein. "Operatively linked" indicates that the coding sequences for the variant protein and the heterologous protein are ligated in-frame. The heterologous protein can be fused to the N-terminus or C-teminus of the variant protein. In another embodiment, the fusion protein is encoded by a fusion polynucleotide that is synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence. See Ausubel et al., Current Protocols in Molecular Biology (1992). Moreover, many expression vectors are commercially available that already encode a fusion moiety (*e.g.,* a GST protein). A variant protein-encoding nucleic acid can be cloned into such an expression vector such that the fusion moiety is linked in-frame to the variant protein.

In many uses, the fusion protein does not affect the activity of the variant protein. The fusion protein can include, but is not limited to, enzymatic fusion proteins, for example, beta-galactosidase fusions, yeast two-hybrid GAL fusions, poly-His fusions, MYC-tagged, HI-tagged and Ig fusions. Such fusion proteins, particularly poly-His fusions, can facilitate their purification following recombinant expression. In certain host cells (*e.g.,* mammalian host cells), expression and/or secretion of a protein can be increased by using a heterologous signal sequence. Fusion proteins are further described in, for example, Terpe, "Overview of tag protein fusions: from molecular and biochemical fundamentals to commercial systems," Appl Microbiol Biotechnol 60(5):523-33 (Jan. 2003); Epub Nov. 07, 2002; Graddis et al., "Designing proteins that work using recombinant technologies," Curr Pharm Biotechnol 3(4):285-97 (Dec. 2002); and Nilsson et al., "Affinity fusion strategies for detection, purification, and immobilization of recombinant proteins," Protein Expr Purif 11(1):1-16 (Oct. 1997).

In certain examples, the compositions disclosed herein also relate to further obvious variants of the variant polypeptides of the present disclosure, such as naturally-occurring mature forms (*e.g.*, allelic variants), non-naturally occurring recombinantly-derived variants, and orthologs and paralogs of such proteins that share sequence homology. Such variants can readily be generated using art-known techniques in the fields of recombinant nucleic acid technology and protein biochemistry.

Further variants of the variant polypeptides disclosed in Table 1 can comprise an amino acid sequence that shares at least 70-80%, 80-85%, 85-90%, 91%, 92%, 93%, 94%, 95%, 96%, 97%, 98%, or 99% sequence identity with an amino acid sequence disclosed in Table 1 (or a fragment thereof) and that includes a novel amino acid residue (allele) disclosed in Table 1 (which is encoded by a novel SNP allele). Thus, the present disclosure specifically contemplates polypeptides that have a certain degree of sequence variation compared with the polypeptide sequences shown in Table 1, but that contain a novel amino acid residue (allele) encoded by a novel SNP allele disclosed herein. In other words, as long as a polypeptide contains a novel amino acid residue disclosed herein, other portions of the polypeptide that flank the novel amino acid residue can vary to some degree from the polypeptide sequences shown in Table 1.

Full-length pre-processed forms, as well as mature processed forms, of proteins that comprise one of the amino acid sequences disclosed herein can readily be identified as having complete sequence identity to one of the variant proteins disclosed herein as well as being encoded by the same genetic locus as the variant proteins provided herein.

Orthologs of a variant peptide can readily be identified as having some degree of significant sequence homology/identity to at least a portion of a variant peptide as well as being encoded by a gene from another organism. Preferred orthologs will be isolated from non-human mammals, preferably primates, for the development of human therapeutic targets and agents. Such orthologs can be encoded by a nucleic acid sequence that hybridizes to a variant peptide-encoding nucleic acid molecule under moderate to stringent conditions depending on the degree of relatedness of the two organisms yielding the homologous proteins.

Variant proteins include, but are not limited to, proteins containing deletions, additions and substitutions in the amino acid sequence caused by the SNPs disclosed herein. One class of substitutions is conserved amino acid substitutions in which a given amino acid in a polypeptide is substituted for another amino acid of like characteristics. Typical conservative substitutions are replacements, one for another, among the aliphatic amino acids Ala, Val, Leu, and Ile; interchange of the hydroxyl residues Ser and Thr; exchange of the acidic residues Asp and Glu; substitution between the amide residues Asn and Gln; exchange of the basic residues Lys and Arg; and replacements among the aromatic residues Phe and Tyr. Guidance concerning which amino acid changes are likely to be phenotypically silent are found, for example, in Bowie et al., Science 247:1306-1310 (1990).

Variant proteins can be fully functional or can lack function in one or more activities, *e.g.* ability to bind another molecule, ability to catalyze a substrate, ability to mediate signaling, etc. Fully functional variants typically contain only conservative variations or variations in non-critical residues or in non-critical regions. Functional variants can also contain substitution of similar amino acids that result in no change or an insignificant change in function. Alternatively, such substitutions may positively or negatively affect function to some degree. Non-functional variants typically contain one or more non-conservative amino acid substitutions, deletions, insertions, inversions, truncations or extensions, or a substitution, insertion, inversion, or deletion of a critical residue or in a critical region.

Amino acids that are essential for function of a protein can be identified by methods known in the art, such as site-directed mutagenesis or alanine-scanning mutagenesis, particularly using the amino acid sequence and polymorphism information provided in Table 1. Cunningham et al., Science 244:1081-1085 (1989). The latter procedure introduces single alanine mutations at every residue in the molecule. The resulting mutant molecules are then tested for biological activity such as enzyme activity or in assays such as an *in vitro* proliferative activity. Sites that are critical for binding partner/substrate binding can also be determined by structural analysis such as crystallization, nuclear magnetic resonance or photoaffinity labeling. Smith et al., J Mol Biol 224:899-904 (1992); de Vos et al., Science 255:306-312 (1992).

Polypeptides can contain amino acids other than the 20 amino acids commonly referred to as the 20 naturally occurring amino acids. Further, many amino acids, including the terminal amino acids, may be modified by natural processes, such as processing and other post-translational modifications, or by chemical modification techniques well known in the art. Accordingly, the variant proteins disclosed herein also encompass derivatives or analogs in which a substituted amino acid residue is not one encoded by the genetic code, in which a substituent group is included, in which the mature polypeptide is fused with another compound, such as a compound to increase the half-life of the polypeptide (*e.g.,* polyethylene glycol), or in which additional amino acids are fused to the mature polypeptide, such as a leader or secretory sequence or a sequence for purification of the mature polypeptide or a pro-protein sequence.

Known protein modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such protein modifications are well known to those of skill in the art and have been described in great detail in the scientific literature. Particularly common modifications, for example glycosylation, lipid attachment, sulfation, gamma-carboxylation of glutamic acid residues, hydroxylation and ADP-ribosylation, are described in most basic texts, such as Proteins - Structure and Molecular Properties 2nd Ed., T.E. Creighton, W.H. Freeman and Company, N.Y. (1993); F. Wold, Posttranslational Covalent Modification of Proteins 1-12, B.C. Johnson, ed., Academic Press, N.Y. (1983); Seifter et al., Meth Enzymol 182:626-646 (1990); and Rattan et al., Ann NY Acad Sci 663:48-62 (1992).

Further, disclosed herein are fragments of the variant proteins in which the fragments contain one or more amino acid sequence variations (*e.g.,* substitutions, or truncations or extensions due to creation or destruction of a stop codon) encoded by one or more SNPs disclosed herein. The fragments to which the present dislcosure pertains, however, are not to be construed as encompassing fragments that have been disclosed in the prior art before the present invention.

As used herein, a fragment may comprise at least about 4, 8, 10, 12, 14, 16, 18, 20, 25, 30, 50, 100 (or any other number in-between) or more contiguous amino acid residues from a variant protein, wherein at least one amino acid residue is affected by a SNP disclosed herein, *e.g.,* a variant amino acid residue encoded by a nonsynonymous nucleotide substitution at a cSNP position disclosed herein. The variant amino acid encoded by a cSNP may occupy any residue position along the sequence of the fragment. Such fragments can be chosen based on the ability to retain one or more of the biological activities of the variant protein or the ability to perform a function, *e.g.,* act as an immunogen. Particularly important fragments are biologically active fragments. Such fragments will typically comprise a domain or motif of a variant protein of the present disclosure, *e.g.,* active site, transmembrane domain, or ligand/substrate binding domain. Other fragments include, but are not limited to, domain or motif-containing fragments, soluble peptide fragments, and fragments containing immunogenic structures. Predicted domains and functional sites are readily identifiable by computer programs well known to those of skill in the art (*e.g.,* PROSITE analysis). Current Protocols in Protein Science, John Wiley & Sons, N.Y. (2002).

Uses of Variant Proteins

The variant proteins disclosed herein can be used in a variety of ways, including but not limited to, in assays to determine the biological activity of a variant protein, such as in a panel of multiple proteins for high-throughput screening; to raise antibodies or to elicit another type of immune response; as a reagent (including the labeled reagent) in assays designed to quantitatively determine levels of the variant protein (or its binding partner) in biological fluids; as a marker for cells or tissues in which it is preferentially expressed (either constitutively or at a particular stage of tissue differentiation or development or in a disease state); as a target for screening for a therapeutic agent; and as a direct therapeutic agent to be administered into a human subject. Any of the variant proteins disclosed herein may be developed into reagent grade or kit format for commercialization as research products. Methods for performing the uses listed above are well known to those skilled in the art. See, *e.g.,* Molecular Cloning: A Laboratory Manual, Sambrook and Russell, Cold Spring Harbor Laboratory Press, N.Y. (2000), and Methods in Enzymology: Guide to Molecular Cloning Techniques, S.L. Berger and A.R. Kimmel, eds., Academic Press (1987).

In a specific example, the methods disclosed herein include detection of one or more variant proteins disclosed herein. Variant proteins are disclosed in Table 1 and in the Sequence Listing as SEQ ID NOS:85-168. Detection of such proteins can be accomplished using, for example, antibodies, small molecule compounds, aptamers, ligands/substrates, other proteins or protein fragments, or other protein-binding agents. Preferably, protein detection agents are specific for a variant protein disclosed herein and can therefore discriminate between a variant protein disclosed herein and the wild-type protein or another variant form. This can generally be accomplished by, for example, selecting or designing detection agents that bind to the region of a protein that differs between the variant and wild-type protein, such as a region of a protein that contains one or more amino acid substitutions that is/are encoded by a non-synonymous cSNP disclosed herein, or a region of a protein that follows a nonsense mutation-type SNP that creates a stop codon thereby leading to a shorter polypeptide, or a region of a protein that follows a read-through mutation-type SNP that destroys a stop codon thereby leading to a longer polypeptide in which a portion of the polypeptide is present in one version of the polypeptide but not the other.

In another example, variant proteins can be used as targets for predicting an individual's response to statin treatment (particularly for reducing the risk of VT), for determining predisposition to VT, for diagnosing VT, or for treating and/or preventing VT, etc. Accordingly, the invention provides methods for detecting the presence of, or levels of, one or more variant proteins disclosed herein in a cell, tissue, or organism. Such methods typically involve contacting a test sample with an agent (*e.g.,* an antibody, small molecule compound, or peptide) capable of interacting with the variant protein such that specific binding of the agent to the variant protein can be detected. Such an assay can be provided in a single detection formal or a multi-detection format such as an array, for example, an antibody or aptamer array (arrays for protein detection may also be referred to as "protein chips"). The variant protein of interest can be isolated from a test sample and assayed for the presence of a variant amino acid sequence encoded by one or more SNPs disclosed herein. The SNPs may cause changes to the protein and the corresponding protein function/activity, such as through non-synonymous substitutions in protein coding regions that can lead to amino acid substitutions, deletions, insertions, and/or rearrangements; formation or destruction of stop codons; or alteration of control elements such as promoters. SNPs may also cause inappropriate post-translational modifications.

One preferred agent for detecting a variant protein in a sample is an antibody capable of selectively binding to a variant form of the protein (antibodies are described in greater detail in the next section). Such samples include, for example, tissues, cells, and biological fluids isolated from a subject, as well as tissues, cells and fluids present within a subject.

*In vitro* methods for detection of the variant proteins associated with statin response that are disclosed herein and fragments thereof include, but are not limited to, enzyme linked immunosorbent assays (ELISAs), radioimmunoassays (RIA), Western blots, immunoprecipitations, immunofluorescence, and protein arrays/chips (*e.g.,* arrays of antibodies or aptamers). For further information regarding immunoassays and related protein detection methods, see Current Protocols in Immunology, John Wiley & Sons, N.Y., and Hage, "Immunoassays," Anal Chem 15;71(12):294R-304R (Jun. 1999).

Additional analytic methods of detecting amino acid variants include, but are not limited to, altered electrophoretic mobility, altered tryptic peptide digest, altered protein activity in cell-based or cell-free assay, alteration in ligand or antibody-binding pattern, altered isoelectric point, and direct amino acid sequencing.

Alternatively, variant proteins can be detected *in vivo* in a subject by introducing into the subject a labeled antibody (or other type of detection reagent) specific for a variant protein. For example, the antibody can be labeled with a radioactive marker whose presence and location in a subject can be detected by standard imaging techniques.

Other uses of the variant peptides disclosed herein are based on the class or action of the protein. For example, proteins isolated from humans and their mammalian orthologs serve as targets for identifying agents (*e.g.,* small molecule drugs or antibodies) for use in therapeutic applications, particularly for modulating a biological or pathological response in a cell or tissue that expresses the protein. Pharmaceutical agents can be developed that modulate protein activity.

As an alternative to modulating gene expression, therapeutic compounds can be developed that modulate protein function. For example, many SNPs disclosed herein affect the amino acid sequence of the encoded protein (*e.g.,* non-synonymous cSNPs and nonsense mutation-type SNPs). Such alterations in the encoded amino acid sequence may affect protein function, particularly if such amino acid sequence variations occur in functional protein domains, such as catalytic domains, ATP-binding domains, or ligand/substrate binding domains. It is well established in the art that variant proteins having amino acid sequence variations in functional domains can cause or influence pathological conditions. In such instances, compounds (*e.g.,* small molecule drugs or antibodies) can be developed that target the variant protein and modulate (*e.g.,* up- or down-regulate) protein function/activity.

The therapeutic methods disclosed herein further include methods that target one or more variant proteins of the present disclosure. Variant proteins can be targeted using, for example, small molecule compounds, antibodies, aptamers, ligands/substrates, other proteins, or other protein-binding agents. Additionally, the skilled artisan will recognize that the novel protein variants (and polymorphic nucleic acid molecules) disclosed in Table 1 may themselves be directly used as therapeutic agents by acting as competitive inhibitors of corresponding art-known proteins (or nucleic acid molecules such as mRNA molecules).

The variant proteins disclosed herein are particularly useful in drug screening assays, in cell-based or cell-free systems. Cell-based systems can utilize cells that naturally express the protein, a biopsy specimen, or cell cultures. In one example, cell-based assays involve recombinant host cells expressing the variant protein. Cell-free assays can be used to detect the ability of a compound to directly bind to a variant protein or to the corresponding SNP-containing nucleic acid fragment that encodes the variant protein.

A variant protein disclosed herein, as well as appropriate fragments thereof, can be used in high-throughput screening assays to test candidate compounds for the ability to bind and/or modulate the activity of the variant protein. These candidate compounds can be further screened against a protein having normal function (*e.g.,* a wild-type/non-variant protein) to further determine the effect of the compound on the protein activity. Furthermore, these compounds can be tested in animal or invertebrate systems to determine *in vivo* activity/effectiveness. Compounds can be identified that activate (agonists) or inactivate (antagonists) the variant protein, and different compounds can be identified that cause various degrees of activation or inactivation of the variant protein.

Further, the variant proteins can be used to screen a compound for the ability to stimulate or inhibit interaction between the variant protein and a target molecule that normally interacts with the protein. The target can be a ligand, a substrate or a binding partner that the protein normally interacts with (for example, epinephrine or norepinephrine). Such assays typically include the steps of combining the variant protein with a candidate compound under conditions that allow the variant protein, or fragment thereof, to interact with the target molecule, and to detect the formation of a complex between the protein and the target or to detect the biochemical consequence of the interaction with the variant protein and the target, such as any of the associated effects of signal transduction.

Candidate compounds include, for example, 1) peptides such as soluble peptides, including Ig-tailed fusion peptides and members of random peptide libraries (see, *e.g.,* Lam et al., Nature 354:82-84 (1991); Houghten et al., Nature 354:84-86 (1991)) and combinatorial chemistry-derived molecular libraries made of D- and/or L- configuration amino acids; 2) phosphopeptides (*e.g.,* members of random and partially degenerate, directed phosphopeptide libraries, see, *e.g.,* Songyang et al., Cell 72:767-778 (1993)); 3) antibodies (*e.g.,* polyclonal, monoclonal, humanized, anti-idiotypic, chimeric, and single chain antibodies as well as Fab, F(ab')$_2$, Fab expression library fragments, and epitope-binding fragments of antibodies); and 4) small organic and inorganic molecules (*e.g.,* molecules obtained from combinatorial and natural product libraries).

One candidate compound is a soluble fragment of the variant protein that competes for ligand binding. Other candidate compounds include mutant proteins or appropriate fragments containing mutations that affect variant protein function and thus compete for ligand. Accordingly, a fragment that competes for ligand, for example with a higher affinity, or a fragment that binds ligand but does not allow release, is encompassed by the invention.

The present disclosure further includes other end point assays to identify compounds that modulate (stimulate or inhibit) variant protein activity. The assays typically involve an assay of events in the signal transduction pathway that indicate protein activity. Thus, the expression of genes that are up or down-regulated in response to the variant protein dependent signal cascade can be assayed. In one example, the regulatory region of such genes can be operably linked to a marker that is easily detectable, such as luciferase. Alternatively, phosphorylation of the variant protein, or a variant protein target, could also be measured. Any of the biological or biochemical functions mediated by the variant protein can be used as an endpoint assay. These include all of the biochemical or biological events described herein, in the references cited herein, and other functions known to those of ordinary skill in the art.

Binding and/or activating compounds can also be screened by using chimeric variant proteins in which an amino terminal extracellular domain or parts thereof, an entire transmembrane domain or subregions, and/or the carboxyl terminal intracellular domain or parts thereof, can be replaced by heterologous domains or subregions. For example, a substrate-binding region can be used that interacts with a different substrate than that which is normally recognized by a variant protein. Accordingly, a different set of signal transduction components is available as an end-point assay for activation. This allows for assays to be performed in other than the specific host cell from which the variant protein is derived.

The variant proteins are also useful in competition binding assays in methods designed to discover compounds that interact with the variant protein. Thus, a compound can be exposed to a variant protein under conditions that allow the compound to bind or to otherwise interact with the variant protein. A binding partner, such as ligand, that normally interacts with the variant protein is also added to the mixture. If the test compound interacts with the variant protein or its binding partner, it decreases the amount of complex formed or activity from the variant protein. This type of assay is particularly useful in screening for compounds that interact with specific regions of the variant protein. Hodgson, Bio/technology, 10(9), 973-80 (Sept. 1992).

To perform cell-free drug screening assays, it is sometimes desirable to immobilize either the variant protein or a fragment thereof, or its target molecule, to facilitate separation of complexes from uncomplexed forms of one or both of the proteins, as well as to accommodate automation of the assay. Any method for immobilizing proteins on matrices can be used in drug screening assays. In one example, a fusion protein containing an added domain allows the protein to be bound to a matrix. For example, glutathione-S-transferase/$^{125}$I fusion proteins can be adsorbed onto glutathione sepharose beads (Sigma Chemical, St. Louis, MO) or glutathione derivatized microtitre plates, which are then combined with the cell lysates (*e.g.,* $^{35}$S-labeled) and a candidate compound, such as a drug candidate, and the mixture incubated under conditions conducive to complex formation (*e.g.,* at physiological conditions for salt and pH). Following incubation, the beads can be washed to remove any unbound label, and the matrix immobilized and radiolabel determined directly, or in the supernatant after the complexes are dissociated. Alternatively, the complexes can be dissociated from the matrix, separated by SDS-PAGE, and the level of bound material found in the bead fraction quantitated from the gel using standard electrophoretic techniques.

Either the variant protein or its target molecule can be immobilized utilizing conjugation of biotin and streptavidin. Alternatively, antibodies reactive with the variant protein but which do not interfere with binding of the variant protein to its target molecule can be derivatized to the wells of the plate, and the variant protein trapped in the wells by antibody conjugation. Preparations of the target molecule and a candidate compound are incubated in the variant protein-presenting wells and the amount of complex trapped in the well can be quantitated. Methods for detecting such complexes, in addition to those described above for the GST-immobilized complexes, include immunodetection of complexes using antibodies reactive with the protein target molecule, or which are reactive with variant protein and compete with the target molecule, and enzyme-linked assays that rely on detecting an enzymatic activity associated with the target molecule.

Modulators of variant protein activity identified according to these drug screening assays can be used to treat a subject with a disorder mediated by the protein pathway, such as VT. These methods of treatment typically include the steps of administering the modulators of protein activity in a pharmaceutical composition to a subject in need of such treatment.

The variant proteins, or fragments thereof, disclosed herein can themselves be directly used to treat a disorder characterized by an absence of, inappropriate, or unwanted expression or activity of the variant protein. Accordingly, methods for treatment include the use of a variant protein disclosed herein or fragments thereof.

In yet another example, variant proteins can be used as "bait proteins" in a two-hybrid assay or three-hybrid assay to identify other proteins that bind to or interact with the variant protein and are involved in variant protein activity. See, *e.g.,* U.S. Patent No. 5,283,317; Zervos et al., Cell 72:223-232 (1993); Madura et al., J Biol Chem 268:12046-12054 (1993); Bartel et al., Biotechniques 14:920-924 (1993); Iwabuchi et al., Oncogene 8:1693-1696 (1993); and Brent, WO 94/10300. Such variant protein-binding proteins are also likely to be involved in the propagation of signals by the variant proteins or variant protein targets as, for example, elements of a protein-mediated signaling pathway. Alternatively, such variant protein-binding proteins are inhibitors of the variant protein.

The two-hybrid system is based on the modular nature of most transcription factors, which typically consist of separable DNA-binding and activation domains. Briefly, the assay typically utilizes two different DNA constructs. In one construct, the gene that codes for a variant protein is fused to a gene encoding the DNA binding domain of a known transcription factor (*e.g.,* GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a variant protein-dependent complex, the DNA-binding and activation domains of the transcription factor are brought into close proximity. This proximity allows transcription of a reporter gene (*e.g.,* LacZ) that is operably linked to a transcriptional regulatory site responsive to the transcription factor. Expression of the reporter gene can be detected, and cell colonies containing the functional transcription factor can be isolated and used to obtain the cloned gene that encodes the protein that interacts with the variant protein.

Antibodies Directed to Variant Proteins

Disclosed herein are also antibodies that selectively bind to the variant proteins disclosed herein and fragments thereof. Such antibodies may be used to quantitatively or qualitatively detect the variant proteins disclosed herein. As used herein, an antibody selectively binds a target variant protein when it binds the variant protein and does not significantly bind to non-variant proteins, *i.e.*, the antibody does not significantly bind to normal, wild-type, or art-known proteins that do not contain a variant amino acid sequence due to one or more SNPs disclosed herein (variant amino acid sequences may be due to, for example, nonsynonymous cSNPs, nonsense SNPs that create a stop codon, thereby causing a truncation of a polypeptide or SNPs that cause read-through mutations resulting in an extension of a polypeptide).

As used herein, an antibody is defined in terms consistent with that recognized in the art: they are multi-subunit proteins produced by an organism in response to an antigen challenge. The antibodies disclosed herein include both monoclonal antibodies and polyclonal antibodies, as well as antigen-reactive proteolytic fragments of such antibodies, such as Fab, F(ab)'$_2$, and Fv fragments. In addition, an antibody of the present disclosure further includes any of a variety of engineered antigen-binding molecules such as a chimeric antibody (U.S. Patent Nos. 4,816,567 and 4,816,397; Morrison et al., Proc Natl Acad Sci USA 81:6851 (1984); Neuberger et al., Nature 312:604 (1984)), a humanized antibody (U.S. Patent Nos. 5,693,762; 5,585,089 and 5,565,332), a single-chain Fv (U.S. Patent No. 4,946,778; Ward et al., Nature 334:544 (1989)), a bispecific antibody with two binding specificities (Segal et al., J Immunol Methods 248:1 (2001); Carter, J Immunol Methods 248:7 (2001)), a diabody, a triabody, and a tetrabody (Todorovska et al., J Immunol Methods 248:47 (2001)), as well as a Fab conjugate (dimer or trimer), and a minibody.

Many methods are known in the art for generating and/or identifying antibodies to a given target antigen. Harlow, Antibodies, Cold Spring Harbor Press, N.Y. (1989). In general, an isolated peptide (*e.g.,* a variant protein of the present disclosure) is used as an immunogen and is administered to a mammalian organism, such as a rat, rabbit, hamster or mouse. Either a full-length protein, an antigenic peptide fragment (*e.g.*, a peptide fragment containing a region that varies between a variant protein and a corresponding wild-type protein), or a fusion protein can be used. A protein used as an immunogen may be naturally-occurring, synthetic or recombinantly produced, and may be administered in combination with an adjuvant, including but not limited to, Freund's (complete and incomplete), mineral gels such as aluminum hydroxide, surface active substance such as lysolecithin, pluronic polyols, polyanions, peptides, oil emulsions, keyhole limpet hemocyanin, dinitrophenol, and the like.

Monoclonal antibodies can be produced by hybridoma technology, which immortalizes cells secreting a specific monoclonal antibody. Kohler and Milstein, Nature 256:495 (1975). The immortalized cell lines can be created *in vitro* by fusing two different cell types, typically lymphocytes, and tumor cells. The hybridoma cells may be cultivated *in vitro* or *in vivo.* Additionally, fully human antibodies can be generated by transgenic animals. He et al., J Immunol 169:595 (2002). Fd phage and Fd phagemid technologies may be used to generate and select recombinant antibodies *in vitro.* Hoogenboom and Chames, Immunol Today 21:371 (2000); Liu et al., J Mol Biol 315:1063 (2002). The complementarity-determining regions of an antibody can be identified, and synthetic peptides corresponding to such regions may be used to mediate antigen binding. U.S. Patent No. 5,637,677.

Antibodies are preferably prepared against regions or discrete fragments of a variant protein containing a variant amino acid sequence as compared to the corresponding wild-type protein (*e.g.,* a region of a variant protein that includes an amino acid encoded by a nonsynonymous cSNP, a region affected by truncation caused by a nonsense SNP that creates a stop codon, or a region resulting from the destruction of a stop codon due to read-through mutation caused by a SNP). Furthermore, preferred regions will include those involved in function/activity and/or protein/binding partner interaction. Such fragments can be selected on a physical property, such as fragments corresponding to regions that are located on the surface of the protein, *e.g.*, hydrophilic regions, or can be selected based on sequence uniqueness, or based on the position of the variant amino acid residue(s) encoded by the SNPs disclosed herein. An antigenic fragment will typically comprise at least about 8-10 contiguous amino acid residues in which at least one of the amino acid residues is an amino acid affected by a SNP disclosed herein. The antigenic peptide can comprise, however, at least 12, 14, 16, 20, 25, 50, 100 (or any other number in-between) or more amino acid residues, provided that at least one amino acid is affected by a SNP disclosed herein.

Detection of an antibody can be facilitated by coupling (*i.e.,* physically linking) the antibody or an antigen-reactive fragment thereof to a detectable substance. Detectable substances include, but are not limited to, various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and aviditin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}$I, $^{131}$I, $^{35}$S or $^{3}$H.

Antibodies, particularly the use of antibodies as therapeutic agents, are reviewed in: Morgan, "Antibody therapy for Alzheimer's disease," Expert Rev Vaccines (1):53-9 (Feb. 2003); Ross et al., "Anticancer antibodies," Am J Clin Pathol 119(4):472-85 (Apr. 2003); Goldenberg, "Advancing role of radiolabeled antibodies in the therapy of cancer," Cancer Immunol Immunother 52(5):281-96 (May 2003); Epub Mar. 11,2003; Ross et al., "Antibody-based therapeutics in oncology," Expert Rev Anticancer Ther 3(1): 107-21 (Feb. 2003); Cao et al., "Bispecific antibody conjugates in therapeutics," Adv Drug Deliv Rev 55(2):171-97 (Feb. 2003); von Mehren et al., "Monoclonal antibody therapy for cancer," Annu Rev Med 54:343-69 (2003); Epub Dec. 3, 2001; Hudson et al., "Engineered antibodies," Nat Med 9(1): 129-34 (Jan. 2003); Brekke et al., "Therapeutic antibodies for human diseases at the dawn of the twenty-first century," Not Rev Drug Discov 2(1):52-62 (Jan. 2003); Erratum in: Nat Rev Drug Discov 2(3):240 (Mar. 2003); Houdebine, "Antibody manufacture in transgenic animals and comparisons with other systems," Curr Opin Biotechnol 13(6):625-9 (Dec. 2002); Andreakos et al., "Monoclonal antibodies in immune and inflammatory diseases," Curr Opin Biotechnol 13(6):615-20 (Dec. 2002); Kellermann et al., "Antibody discovery: the use of transgenic mice to generate human monoclonal antibodies for therapeutics," Curr Opin Biotechnol 13(6):593-7 (Dec. 2002); Pini et al., "Phage display and colony filter screening for high-throughput selection of antibody libraries," Comb Chem High Throughput Screen 5(7):503-10 (Nov. 2002); Batra et al., "Pharmacokinetics and biodistribution of genetically engineered antibodies," Curr Opin Biotechnol 13(6):(603-8 (Dec. 2002); and Tangri et al., "Rationally engineered proteins or antibodies with absent or reduced immunogenicity," Curr Med Chem 9(24):2191-9 (Dec. 2002).

Uses of Antibodies

Antibodies can be used to isolate the variant proteins disclosed herein from a natural cell source or from recombinant host cells by standard techniques, such as affinity chromatography or immunoprecipitation. In addition, antibodies are useful for detecting the presence of a variant protein disclosed herein in cells or tissues to determine the pattern of expression of the variant protein among various tissues in an organism and over the course of normal development or disease progression. Further, antibodies can be used to detect variant protein *in situ*, *in vitro,* in a bodily fluid, or in a cell lysate or supernatant in order to evaluate the amount and pattern of expression. Also, antibodies can be used to assess abnormal tissue distribution, abnormal expression during development, or expression in an abnormal condition, such as in VT, or during statin treatment. Additionally, antibody detection of circulating fragments of the full-length variant protein can be used to identify turnover.

Antibodies to the variant proteins disclosed herein are also useful in pharmacogenomic analysis. Thus, antibodies against variant proteins encoded by alternative SNP alleles can be used to identify individuals that require modified treatment modalities.

Further, antibodies can be used to assess expression of the variant protein in disease states such as in active stages of the disease or in an individual with a predisposition to a disease related to the protein's function, such as VT, or during the course of a treatment regime, such as during statin treatment. Antibodies specific for a variant protein encoded by a SNP-containing nucleic acid molecule disclosed herein can be used to assay for the presence of the variant protein, such as to determine an individual's response to statin treatment (particularly for reducing their risk for VT) or to diagnose VT or predisposition/susceptibility to VT, as indicated by the presence of the variant protein.

Antibodies are also useful as diagnostic tools for evaluating the variant proteins in conjunction with analysis by electrophoretic mobility, isoelectric point, tryptic peptide digest, and other physical assays well known in the art.

Antibodies are also useful for tissue typing. Thus, where a specific variant protein has been correlated with expression in a specific tissue, antibodies that are specific for this protein can be used to identify a tissue type.

Antibodies can also be used to assess aberrant subcellular localization of a variant protein in cells in various tissues. The diagnostic uses can be applied, not only in genetic testing, but also in monitoring a treatment modality. Accordingly, where treatment is ultimately aimed at correcting the expression level or the presence of variant protein or aberrant tissue distribution or developmental expression of a variant protein, antibodies directed against the variant protein or relevant fragments can be used to monitor therapeutic efficacy.

The antibodies are also useful for inhibiting variant protein function, for example, by blocking the binding of a variant protein to a binding partner. These uses can also be applied in a therapeutic context in which treatment involves inhibiting a variant protein's function. An antibody can be used, for example, to block or competitively inhibit binding, thus modulating (agonizing or antagonizing) the activity of a variant protein. Antibodies can be prepared against specific variant protein fragments containing sites required for function or against an intact variant protein that is associated with a cell or cell membrane. For *in vivo* administration, an antibody may be linked with an additional therapeutic payload such as a radionuclide, an enzyme, an immunogenic epitope, or a cytotoxic agent Suitable cytotoxic agents include, but are not limited to, bacterial toxin such as diphtheria, and plant toxin such as ricin. The *in vivo* half-life of an antibody or a fragment thereof may be lengthened by pegylation through conjugation to polyethylene glycol. Leong et al., Cytokine 16:106 (2001).

The present disclosure also describes kits for using antibodies, such as kits for detecting the presence of a variant protein in a test sample. An exemplary kit can comprise antibodies such as a labeled or labelable antibody and a compound or agent for detecting variant proteins in a biological sample: means for determining the amount, or presence/absence of variant protein in the sample; means for comparing the amount of variant protein in the sample with a standard; and instructions for use.

Vectors and Host Cells

Disclosed herein are also vectors containing the SNP-containing nucleic acid molecules described herein. The term "vector" refers to a vehicle, preferably a nucleic acid molecule, which can transport a SNP-containing nucleic acid molecule. When the vector is a nucleic acid molecule, the SNP-containing nucleic acid molecule can be covalently linked to the vector nucleic acid. Such vectors include, but are not limited to, a plasmid, single or double stranded phage, a single or double stranded RNA or DNA viral vector, or artificial chromosome, such as a BAC, PAC, YAC, or MAC.

A vector can be maintained in a host cell as an extrachromosomal element where it replicates and produces additional copies of the SNP-containing nucleic acid molecules. Alternatively, the vector may integrate into the host cell genome and produce additional copies of the SNP-containing nucleic acid molecules when the host cell replicates.

Disclosed herein are vectors for the maintenance (cloning vectors) or vectors for expression (expression vectors) of the SNP-containing nucleic acid molecules. The vectors can function in prokaryotic or eukaryotic cells or in both (shuttle vectors).

Expression vectors typically contain cis-acting regulatory regions that are operably linked in the vector to the SNP-containing nucleic acid molecules such that transcription of the SNP-containing nucleic acid molecules is allowed in a host cell. The SNP-containing nucleic acid molecules can also be introduced into the host cell with a separate nucleic acid molecule capable of affecting transcription. Thus, the second nucleic acid molecule may provide a trans-acting factor interacting with the cis-regulatory control region to allow transcription of the SNP-containing nucleic acid molecules from the vector. Alternatively, a trans-acting factor may be supplied by the host cell. Finally, a trans-acting factor can be produced from the vector itself, It is understood, however, that in some embodiments, transcription and/or translation of the nucleic acid molecules can occur in a cell-free system.

The regulatory sequences to which the SNP-containing nucleic acid molecules described herein can be operably linked include promoters for directing mRNA transcription. These include, but are not limited to, the left promoter from bacteriophage λ, the lac, TRP, and TAC promoters from *E. coli*, the early and late promoters from SV40, the CMV immediate early promoter, the adenovirus early and late promoters, and retrovirus long-terminal repeats.

In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

In addition to containing sites for transcription initiation and control, expression vectors can also contain sequences necessary for transcription termination and, in the transcribed region, a ribosome-binding site for translation. Other regulatory control elements for expression include initiation and termination codons as well as polyadenylation signals. A person of ordinary skill in the art would be aware of the numerous regulatory sequences that are useful in expression vectors. See, *e.g.*, Sambrook and Russell, Molecular Cloning: A Laboratory Manual, Cold Spring Harbor Laboratory Press, N.Y. (2000).

A variety of expression vectors can be used to express a SNP-containing nucleic acid molecule. Such vectors include chromosomal, episomal, and virus-derived vectors, for example, vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses, adenoviruses, poxviruses, pseudorabies viruses, and retroviruses. Vectors can also be derived from combinations of these sources such as those derived from plasmid and bacteriophage genetic elements, *e.g.*, cosmids and phagemids. Appropriate cloning and expression vectors for prokaryotic and eukaryotic hosts are described in Sambrook and Russell, Molecular Cloning: A Laboratory Manual Cold Spring Harbor Laboratory Press, N.Y. (2000).

The regulatory sequence in a vector may provide constitutive expression in one or more host cells (*e.g.,* tissue specific expression) or may provide for inducible expression in one or more cell types such as by temperature, nutrient additive, or exogenous factor, *e.g.*, a hormone or other ligand. A variety of vectors that provide constitutive or inducible expression of a nucleic acid sequence in prokaryotic and eukaryotic host cells are well known to those of ordinary skill in the art

A SNP-containing nucleic acid molecule can be inserted into the vector by methodology well-known in the art. Generally, the SNP-containing nucleic acid molecule that will ultimately be expressed is joined to an expression vector by cleaving the SNP-containing nucleic acid molecule and the expression vector with one or more restriction enzymes and then ligating the fragments together. Procedures for restriction enzyme digestion and ligation are well known to those of ordinary skill in the art.

The vector containing the appropriate nucleic acid molecule can be introduced into an appropriate host cell for propagation or expression using well-known techniques. Bacterial host cells include, but are not limited to, *Escherichia coli, Streptomyces spp.*, and *Salmonella typhimurium.* Eukaryotic host cells include, but are not limited to, yeast, insect cells such as *Drosophila spp.*, animal cells such as COS and CHO cells, and plant cells.

As described herein, it may be desirable to express the variant peptide as a fusion protein. Accordingly, disclosed herein are fusion vectors that allow for the production of the variant peptides. Fusion vectors can, for example, increase the expression of a recombinant protein, increase the solubility of the recombinant protein, and aid in the purification of the protein by acting, for example, as a ligand for affinity purification. A proteolytic cleavage site may be introduced at the junction of the fusion moiety so that the desired variant peptide can ultimately be separated from the fusion moiety. Proteolytic enzymes suitable for such use include, but are not limited to, factor Xa, thrombin, and enterokinase. Typical fusion expression vectors include pGEX (Smith et al., Gene 67:31-40 (1988)), pMAL(New England Biolabs, Beverly, Mass.) and pRIT5 (Pharmacia, Piscataway, NJ.) which fuse glutathione S-transferase (GST), maltose E binding protein, or protein A, respectively, to the target recombinant protein. Examples of suitable inducible non-fusion *E. coli* expression vectors include pTrc (Amann et al., Gene 69:301-315 (1988)) and pET 11d (Studier et al., Gene Expression Technology: Methods in Enzymology 185:60-89 (1990)).

Recombinant protein expression can be maximized in a bacterial host by providing a genetic background wherein the host cell has an impaired capacity to proteolytically cleave the recombinant protein (S. Gottesman, Gene Expression Technology: Methods in Enzymology 185:119-128, Academic Press, Calif. (1990)). Alternatively, the sequence of the SNP-containing nucleic acid molecule of interest can be altered to provide preferential codon usage for a specific host cell, for example, *E. coli.* Wada et al., Nucleic Acids Res 20:2111-2118 (1992).

The SNP-containing nucleic acid molecules can also be expressed by expression vectors that are operative in yeast. Examples of vectors for expression in yeast (*e.g., S. cerevisiae*) include pYepSec1 (Baldari et al., EMBO J 6:229-234 (1987)), pMFa (Kurjan et al., Cell 30:933-943 (1982)), pJRY88 (Schultz et al., Gene 54:113-123 (1987)), and pYES2 (Invitrogen Corporation, San Diego, Calif.).

The SNP-containing nucleic acid molecules can also be expressed in insect cells using, for example, baculovirus expression vectors. Baculovirus vectors available for expression of proteins in cultured insect cells (*e.g.*, Sf 9 cells) include the pAc series (Smith et al., Mol Cell Biol 3:2156-2165 (1983)) and the pVL series (Lucklow et al., Virology 170:31-39 (1989)).

In certain cases, the SNP-containing nucleic acid molecules described herein are expressed in mammalian cells using mammalian expression vectors. Examples of mammalian expression vectors include pCDM8 (B. Seed, Nature 329:840(1987)) and pMT2PC (Kaufman et al., EMBO J 6:187-195 (1987)).

Disclosed herein are also vectors in which the SNP-containing nucleic acid molecules described herein are cloned into the vector in reverse orientation, but operably linked to a regulatory sequence that permits transcription of antisense RNA. Thus, an antisense transcript can be produced to the SNP-containing nucleic acid sequences described herein, including both coding and non-coding regions. Expression of this antisense RNA is subject to each of the parameters described above in relation to expression of the sense RNA (regulatory sequences, constitutive or inducible expression, tissue-specific expression).

Disclosed herein are also recombinant host cells containing the vectors described herein. Host cells therefore include, for example, prokaryotic cells, lower eukaryotic cells such as yeast, other eukaryotic cells such as insect cells, and higher eukaryotic cells such as mammalian cells.

The recombinant host cells can be prepared by introducing the vector constructs described herein into the cells by techniques readily available to persons of ordinary skill in the art. These include, but are not limited to, calcium phosphate transfection, DEAE-dextran-mediated transfection, cationic lipid-mediated transfection, electroporation, transduction, infection, lipofection, and other techniques such as those described in Sambrook and Russell, Molecular Cloning: A Laboratory Manual, Cold Spring Harbor Laboratory. Cold Spring Harbor Laboratory Press, N.Y. (2000).

Host cells can contain more than one vector. Thus, different SNP-containing nucleotide sequences can be introduced in different vectors into the same cell. Similarly, the SNP-containing nucleic acid molecules can be introduced either alone or with other nucleic acid molecules that are not related to the SNP-containing nucleic acid molecules, such as those providing trans-acting factors for expression vectors. When more than one vector is introduced into a cell, the vectors can be introduced independently, co-introduced, or joined to the nucleic acid molecule vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication can occur in host cells that provide functions that complement the defects.

Vectors generally include selectable markers that enable the selection of the subpopulation of cells that contain the recombinant vector constructs. The marker can be inserted in the same vector that contains the SNP-containing nucleic acid molecules described herein or may be in a separate vector. Markers include, for example, tetracycline or ampicillin-resistance genes for prokaryotic host cells, and dihydrofolate reductase or neomycin resistance genes for eukaryotic host cells. However, any marker that provides selection for a phenotypic trait can be effective.

While the mature variant proteins can be produced in bacteria, yeast, mammalian cells, and other cells under the control of the appropriate regulatory sequences, cell-free transcription and translation systems can also be used to produce these variant proteins using RNA derived from the DNA constructs described herein.

Where secretion of the variant protein is desired, which is difficult to achieve with multi-transmembrane domain containing proteins such as G-protein-coupled receptors (GPCRs), appropriate secretion signals can be incorporated into the vector. The signal sequence can be endogenous to the peptides or heterologous to these peptides.

Where the variant protein is not secreted into the medium, the protein can be isolated from the host cell by standard disruption procedures, including freeze/thaw, sonication, mechanical disruption, use of lysing agents, and the like. The variant protein can then be recovered and purified by well-known purification methods including, for example, ammonium sulfate precipitation, acid extraction, anion or cationic exchange chromatography, phosphocellulose chromatography, hydrophobic-interaction chromatography, affinity chromatography, hydroxylapatite chromatography, lectin chromatography, or high performance liquid chromatography.

It is also understood that, depending upon the host cell in which recombinant production of the variant proteins described herein occurs, they can have various glycosylation patterns, or may be non-glycosylated, as when produced in bacteria. In addition, the variant proteins may include an initial modified methionine in some cases as a result of a host-mediated process.

For further information regarding vectors and host cells, see Current Protocols in Molecular Biology, John Wiley & Sons, N.Y.

Uses of Vectors and Host Cells, and Transgenic Animals

Recombinant host cells that express the variant proteins described herein have a variety of uses. For example, the cells are useful for producing a variant protein that can be further purified into a preparation of desired amounts of the variant protein or fragments thereof. Thus, host cells containing expression vectors are useful for variant protein production.

Host cells are also useful for conducting cell-based assays involving the variant protein or variant protein fragments, such as those described above as well as other formats known in the art. Thus, a recombinant host cell expressing a variant protein is useful for assaying compounds that stimulate or inhibit variant protein function. Such an ability of a compound to modulate variant protein function may not be apparent from assays of the compound on the native/wild-type protein, or from cell-free assays of the compound. Recombinant host cells are also useful for assaying functional alterations in the variant proteins as compared with a known function.

Genetically-engineered host cells can be further used to produce non-human transgenic animals. A transgenic animal is preferably a non-human mammal, for example, a rodent, such as a rat or mouse, in which one or more of the cells of the animal include a transgene. A transgene is exogenous DNA containing a SNP disclosed herein which is integrated into the genome of a cell from which a transgenic animal develops and which remains in the genome of the mature animal in one or more of its cell types or tissues. Such animals are useful for studying the function of a variant protein *in vivo*, and identifying and evaluating modulators of variant protein activity. Other examples of transgenic animals include, but are not limited to, non-human primates, sheep, dogs, cows, goats, chickens, and amphibians. Transgenic non-human mammals such as cows and goats can be used to produce variant proteins which can be secreted in the animal's milk and then recovered.

A transgenic animal can be produced by introducing a SNP-containing nucleic acid molecule into the male pronuclei of a fertilized oocyte, *e.g.*, by microinjection or retroviral infection, and allowing the oocyte to develop in a pseudopregnant female foster animal. Any nucleic acid molecules that contain one or more SNPs disclosed herein can potentially be introduced as a transgene into the genome of a non-human animal.

Any of the regulatory or other sequences useful in expression vectors can form part of the transgenic sequence. This includes intronic sequences and polyadenylation signals, if not already included. A tissue-specific regulatory sequence(s) can be operably linked to the transgene to direct expression of the variant protein in particular cells or tissues.

Methods for generating transgenic animals via embryo manipulation and microinjection, particularly animals such as mice, have become conventional in the art and are described, for example, in U.S. Patent Nos. 4,736,866 and 4,870,009, both by Leder et al.; U.S. Patent No. 4,873,191 by Wagner et al.*,* and in B. Hogan, Manipulating the Mouse Embryo, Cold Spring Harbor Laboratory Press, N.Y. (1986). Similar methods are used for production of other transgenic animals. A transgenic founder animal can be identified based upon the presence of the transgene in its genome and/or expression of transgenic mRNA in tissues or cells of the animals. A transgenic founder animal can then be used to breed additional animals carrying the transgene. Moreover, transgenic animals carrying a transgene can further be bred to other transgenic animals carrying other transgenes. A transgenic animal also includes a non-human animal in which the entire animal or tissues in the animal have been produced using the homologously recombinant host cells described herein.

In another example, transgenic non-human animals can be produced which contain selected systems that allow for regulated expression of the transgene. One example of such a system is the cre/loxP recombinase system of bacteriophage P1. Lakso et al., PNAS 89:6232-6236 (1992). Another example of a recombinase system is the FLP recombinase system of *S. cerevisiae.* O'Gorman et al., Science 251:1351-1355 (1991). If a cre/loxP recombinase system is used to regulate expression of the transgene, animals containing transgenes encoding both the Cre recombinase and a selected protein are generally needed. Such animals can be provided through the construction of "double" transgenic animals, *e.g.*, by mating two transgenic animals, one containing a transgene encoding a selected variant protein and the other containing a transgene encoding a recombinase.

Clones of the non-human transgenic animals described herein can also be produced according to the methods described, for example, in I. Wilmut et al., Nature 385:810-813 (1997) and PCT International Publication Nos. WO 97/07668 and WO 97/07669. In brief, a cell (*e.g.*, a somatic cell) from the transgenic animal can be isolated and induced to exit the growth cycle and enter $G_o$ phase. The quiescent cell can then be fused, *e.g.*, through the use of electrical pulses, to an enucleated oocyte from an animal of the same species from which the quiescent cell is isolated. The reconstructed oocyte is then cultured such that it develops to morula or blastocyst and then transferred to pseudopregnant female foster animal. The offspring born of this female foster animal will be a clone of the animal from which the cell (*e.g.*, a somatic cell) is isolated.

Transgenic animals containing recombinant cells that express the variant proteins described herein are useful for conducting the assays described herein in an *in vivo* context Accordingly, the various physiological factors that are present *in vivo* and that could influence ligand or substrate binding, variant protein activation, signal transduction, or other processes or interactions, may not be evident from *in vitro* cell-free or cell-based assays. Thus, non-human transgenic animals may be used to assay *in vivo* variant protein function as well as the activities of a therapeutic agent or compound that modulates variant protein function/activity or expression. Such animals are also suitable for assessing the effects of null mutations (*i.e.*, mutations that substantially or completely eliminate one or more variant protein functions).

For further information regarding transgenic animals, see Houdebine, "Antibody manufacture in transgenic animals and comparisons with other systems,"Curr Opin Biotechnol 13(6):625-9 (Dec. 2002); Petters et al., "Transgenic animals as models for human disease," Transgenic Res 9(4-5):347-51, discussion 345-6 (2000); Wolf et al., "Use of transgenic animals in understanding molecular mechanisms of toxicity," J Pharm Pharmacol 50(6):567-74 (Jun. 1998); Echelard, "Recombinant protein production in transgenic animals," Curr Opin Biotechnol 7(5):536-40 (Oct. 1996); Houdebine, "Transgenic animal bioreactors," Transgenic Res 9(4-5):305-20 (2000); Pirity et al., "Embryonic stem cells, creating transgenic animals," Methods Cell Biol 57:279-93 (1998); and Robl et al., "Artificial chromosome vectors and expression of complex proteins in transgenic animals," Theriogenology 59(1): 107-13 (Jan. 2003).

EXAMPLES

The following examples are offered to illustrate, but not limit, the claimed invention.

Example 1: SNPs associated with response to statins for reducing VT risk

27 SNPs were identified that had a significant p(interaction) for statin*SNP of < 0.05 (Wald test) for the statin*SNP interaction term in the MEGA sample set (ModelFormula: VTE-SNP + statin user or nonuser + SNP*statin + age + sex). These 27 SNPs are provided in Table 4. Further, Table 6 provides additional SNPs with P(int) < 0.1. Thus, the SNPs provided in Tables 4 and 6 can be assayed to determine whether statin treatment will reduce an individual's risk for VT.

*Analysis of SNPs in statin subgroups (statin users vs. statin nonusers)*

75 SNPs genotyped in MEGA had an additive P < 0.05 for VT risk in the statin nonusers subgroup. Comparing the risk of VT in the statin users subgroup for these SNPs identifies individuals at risk for VT that benefit from statin therapy and individuals at risk for VT that do not benefit from statin therapy. These 75 SNPs are provided in Table 5. Thus, the SNPs provided in Table 5 can be assayed to determine whether statin treatment will reduce an individual's risk for VT.

*MEGA sample set*

The sample sets used in the present analysis were from a large population-based case-control study referred to as the Multiple Environmental and Genetic Assessment of risk factors for venous thrombosis (MEGA study) (Koster et al., Lancet 1993; 342(8886-8887): 1503-1506 and Blom et al., JAMA 2005; 293(6):715-722), including both the MEGA-1 and MEGA-2 subsets of the MEGA study. The MEGA study was approved by the Medical Ethics Committee of the Leiden University Medical Center, Leiden, The Netherlands. All participants gave informed consent to participate.

Collection and ascertainment of VT events in MEGA has been described previously (Blom et al., JAMA 2005; 293(6):715-722; van Stralen et al., Arch Intern Med 2008; 168(1):21-26). MEGA enrolled consecutive patients aged 18 to 70 years who presented with their first diagnosis of VT (deep vein thrombosis of the leg, venous thrombosis of the arm, or pulmonary embolism) at any of six anticoagulation clinics in The Netherlands between March 1, 1999 and May 31, 2004. Control subjects included partners of patients and random population control subjects frequency-matched on age and sex to the patient group. Participants completed a questionnaire on risk factors for VT and medication use (including statins), and provided a blood or buccal swab sample. Seven different statins were used by statin users, which are all combined in the current analysis, however 94% of statin users used simvastatin, pravastatin, or atorvastatin. The questionnaire included an item on parent birth country as a proxy for ethnicity,

Two SNPs in particular that were identified in MEGA as being significantly associated with statin response for reducing VT risk were in the *F11* gene: *F11* SNP rs2036914 (see Tables 4 and 5) and *F11* SNP rs2289252 (see Table 5).

Example 2: Association of *F11* SNPs rs2036914 and rs2289252 with response to statin treatment for reducing VT risk

The MEGA study was analyzed to determine whether carriers of the risk alleles of *F11* SNPs rs2289252 and rs2036914, compared with noncarriers, were at increased risk for VT among statin users and also among nonusers.

The MEGA study recruited consecutive patients aged 18 to 70 years with a first diagnosis of VT (deep vein thrombosis of the leg, venous thrombosis of the arm, or pulmonary embolism) from six anticoagulation clinics in the Netherlands between March 1, 1999 and May 31, 2004 (Blom et al., JAMA. 2005; 293: 715-22). Partners of patients were invited to take part as control participants. Additional controls were recruited from the same geographical region by a random digit dialing method and were frequency-matched to patients by age and sex (Chinthammitr et al., J Thromb Haemost. 2006; 4: 2587-92). Information on risk factors for VT and medication use (including statins) prior to their VT event for cases or prior to enrollment for controls was obtained from questionnaires completed by the participants. Seven different statins were used by statin users, which are all combined in the current analysis, however 94% of statin users used simvastatin, pravastatin, or atorvastatin. Participants also provided a blood or buccal swab sample for DNA extraction. Genotypes were determined in a core laboratory that was blinded to case-control status (Germer et al., Genome Res. 2000; 10: 258-66). All study participants provided written informed consent. The MEGA study was approved by the Medical Ethics Committee of the Leiden University Medical Center, Leiden, The Netherlands.

DNA was available for 9803 participants. Because active cancer is a strong risk factor for VT that might mask other associations, participants with a known malignancy or missing malignancy status were excluded from the current analysis (n=708); participants without medication use information were also excluded (n=204); thus, 3698 cases with VT and 4473 controls with no history of VT were investigated in the current study. Of these 8171 study participants, 384 (5%) were self-reported statin users (125 cases and 259 controls). Logistic regression models that adjusted for age and sex were used to assess association between genotype and VT in statin users and nonusers separately using SAS software (version 9.1) (SAS Institute Inc., Cary, NC, USA).

Cases and controls did not differ appreciably in mean age [cases, 47.2 years (standard deviation, 12.9); controls, 47.6 years (standard deviation, 12.3)] or sex (45.6% of cases and 47.2% of controls were male). In the controls of MEGA, the genotypes frequencies for rs2289252 were 17.1% (TT), 47.1% (TC) and 35.8% (CC) and for rs2036914 were 27.4% (CC), 49.3% (CT) and 23.3% (TT). Genotype distributions for the 2 SNPs in MEGA did not deviate from Hardy-Weinberg expectations among controls (P>0.25) (Weir, Genetic Data Analysis II. Sunderland: Sinauer Associates Inc., 1996). The linkage disequilibrium between rs2289252 and rs2036914 was moderate ($r^2$=0.38) in the HapMap CEPH population (Utah residents with ancestry from northern and western Europe) (Frazer et al., Nature. 2007; 449: 851-61).

Among statin nonusers of MEGA, the rs2289252 and rs2036914 SNPs were associated with VT (Figure): for participants carrying two risk alleles, compared with those carrying no risk alleles, the OR for VT was 1.83 (95% CI, 1.60 to 2.08) for rs2289252 and 1.75 (95% CI, 1.54 to 1.98) for rs2036914. For participants with one risk allele, the OR was 1.39 (95% CI, 1.26 to 1.55) for rs2289252 and 1.30 (95% CI, 1.15 to 1.46) for rs2036914, again compared with participants carrying no risk alleles.

In contrast, among statin users, carriers of rs2289252 were not at increased risk for VT. For participants carrying two risk alleles, compared with those carrying no risk alleles, the OR for VT was 1.06 (95% CI, 0.66 to 1.71); for those carrying one risk allele the OR was 1.10 (95% CI, 0.57 to 2.10); and for carriers of 1 or 2 risk alleles, the OR was 1.07 (95% CI, 0.68 to 1.68). Similarly, among statin users, carriers of two rs2036914 risk alleles were also not at increased risk for VT: the OR was 1.03 (95% CI, 0.53 to 1.99).

It was also determined whether the association between factor V Leiden and VT differed according to statin use. For factor V Leiden, the ORs for VT were not appreciably different between statin users and nonusers. Among statin users, for carriers of factor V Leiden, compared with noncarriers, the OR was 4.94 (95% CI, 2.37 to 10.30) and among nonusers the OR was 3.64 (95% CI, 3.09 to 4.29).

Thus, among MEGA participants who were statin nonusers, it was determined that carriers compared with noncarriers of the risk alleles of rs2289252 and rs2036914 had an increased risk for VT. In contrast, among statin users, carriers of two risk alleles were not at increased risk for VT.

Although anticoagulant therapy reduces the risk for VT events by about 80% (Dentali et al., Ann Intern Med. 2007; 146: 278-88), anticoagulant therapy also causes life-threatening bleeding events (Shireman et al., Chest. 2006; 130: 1390-6; Wittkowsky et al., Arch Intern Med. 2005; 165: 703; and Buresly et al., Arch Intern Med. 2005; 165: 784-9). Thus, statin therapy may be a useful treatment option, particularly when there are concerns about bleeding risk or when the risk of VT is modest. The genetic risk for VT from *F11* SNPs rs2036914 and rs2289252 exposes patients to a modest lifelong increase in risk for VT, and in this study of MEGA, the risk for VT in carriers of two alleles of the *F11* variants was attenuated by statin use.

Thus, in conclusion, the association of each of *F11* SNPs rs2036914 and rs2289252 with statin response for reducing VT risk in MEGA is shown in the Figure. The Figure shows risk of VT according to statin use for rs2289252, rs2036914, and Factor V Leiden genotypes. The odds ratios in the Figure (shown with 95% confidence intervals) were adjusted for sex and age.

As shown in the Figure, individuals who were T/T homozygotes or T/C heterozygotes at *F11* SNP rs2289252 and who used statins had a reduced risk for VT relative to individuals of the same genotype who did not use statins (lower odds ratios of 1.06 for statin users vs. 1.83 for statin nonusers for T/T homozygous individuals, and lower odds ratio of 1.10 for statin users vs. 1.39 for statin nonusers for T/C heterozygous individuals).

The Figure also shows that individuals who were C/C homozygotes at *F11* SNP rs2036914 and who used statins had a reduced risk for VT relative to individuals of the same genotype who did not use statins (lower odds ratio of 1.03 for statin users vs. 1.75 for statin nonusers for C/C homozygous individuals).

*Factor XI protein levels*

In addition to being associated with VT risk, *F11* SNPs rs2036914 and rs2289252 are also associated with factor XI protein levels, and increased factor XI protein levels are associated with increased VT risk (although *F11* SNPs rs2036914 and rs2289252 are associated with factor XI protein levels, both SNPs remain significantly associated with VT risk after adjustment for factor XI levels). Since increased factor XI protein levels are associated with increased VT risk, statin therapy may reduce VT risk by inhibiting factor XI levels associated with the risk alleles of *F11* SNPs rs2036914 and rs2289252, or by inhibiting the mechanism by which elevated factor XI levels increase VT risk.

Accordingly, in certain exemplary embodiments, a genetic test that assays one or both of *F11* SNPs rs2036914 or rs2289252 (or one or more other SNPs in high LD with either of these *F11* SNP) is used in conjunction with a test that measures factor XI protein levels (e.g., in serum or plasma) to identify patients who will have a greater likelihood of VT event reduction (i.e., reduced VT risk) from statin therapy (i.e., increased statin benefit). In further embodiments, a test that measures factor XI protein levels can be used in combination with a genetic test that assays any of the SNPs disclosed herein for VT risk and/or response to statin treatment for reducing VT risk.

Example 3: Additional analysis of SNPs associated with response to statins for reducing VT risk

Table 7 provides the results from an additional analysis for SNPs associated with response to statins for reducing risk of VT. Table 7 provides SNPs that were significantly associated with response to statins for reducing risk of VT in the MEGA substudy of statin users.

In this Example, the MEGA study was analyzed to determine whether certain genotypes of SNPs were at increased risk for VT among statin users and also among statin nonusers. The MEGA study is described above in Examples 1 and 2. In the additional analysis described here in Example 3, the results of which are provided in Table 7, a subset of controls were randomly selected rather that using all controls (all cases were used) from MEGA, since controls greatly outnumbered cases in MEGA.

*Description of statin substudy of MEGA*

DNA was available for 9803 participants. Because active cancer is a strong risk factor for VT that might mask other associations, participants with a known malignancy or missing malignancy status were excluded from the current analysis (n=708); participants without medication use information were also excluded (n=204); thus, 3698 cases with VT and 4473 controls with no history of VT were investigated in the current study. Of the 3698 cases with VT, 125 cases were self-reported statin users and, of the 4473 controls, 257 were self-reported statin users. Because only 384 (5%) of the total cohort were statin users, 539 cases and 607 controls were randomly selected from among the statin nonusers to genotype and use in the analysis. Logistic regression models that adjusted for age and sex were used to assess association between genotype and VT in statin users and nonusers separately using SAS software (section of Table 7 labeled "Statin response by genotype group"). The association between genotype and VT was assessed in statin users (section of Table 7 labeled "Risk of VT in statin use group") and nonusers (section of Table 7 labeled "Risk of VT in no statin use group") separately using regression models that adjusted for age and sex using SAS software (version 9.1) (SAS Institute Inc., Cary, NC, USA).

Example 4: SNPs associated with risk for VT, particularly recurrent VT

An analysis was carried out to identify SNPs associated with VT, particularly recurrent VT. These SNPs are provided in Table 8. Specifically, Table 8 provides 33 SNPs associated with VT risk in a MEGA case-control study and also with recurrent VT risk in a MEGA recurrent VT prospective study. The MEGA study/sample set is described above in Examples 1 and 2.

Study Design

*Recurrent VT study*

The effect of genetic variants on the risk of recurrent VT in MEGA was assessed. Patients that had a primary VT (either DVT of the leg, PE, or both) were included in the current study; patients with DVT of the arm only were excluded from the study (Flinterman et al., "Recurrent thrombosis and survival after a first venous thrombosis of the upper extremity", Circulation. 2008; 118: 1366-72). Since active cancer is a risk factor for VT, participants were excluded who had malignancy or who had an unknown malignancy status at baseline of the original MEGA study (no information regarding cancer was available during the follow-up study of recurrent VT). 3,824 patients with a first VT from the MEGA study were followed for recurrent VT events over a mean of five years. Among these patients, 137 patients were lost to follow-up and excluded from the analysis. Of these 3,686 participants included in the current study, 565 had a recurrent VT (Table 10).

*Primary VT study*

The MEGA primary VT study included 3824 cases and 4672 controls (Table 10). Individuals with a history of malignant disorders were excluded.

Table 10

Characteristics of cases and controls in MEGA

| Characteristic | Primary VT | | | Recurrent VT | | |
|---|---|---|---|---|---|---|
| | Case | Control | p Value | Event | No Event | p Value |
| Number of patients | 3824 | 4672 | | 565 | 3121 | |
| Men | 1734 | 2203 | 0.11 | 366 | 1293 | <0.0001 |
| Mean age (SD) in yrs | 48 (13) | 48 (12) | 0.98 | 50 (13) | 47 (13) | <0.0001 |

Examination and Laboratory Measures

Data collection methods for the recurrent VT study are described in Flinterman et al. ("Recurrent thrombosis and survival after a first venous thrombosis of the upper extremity", Circulation. 2008; 118: 1366-72). Briefly, in 2006, an inquiry form was sent to those patients who had a primary VT and who had initially agreed to participate in a follow-up study. The patients were asked if they had had another VT event in any location since their primary VT event and were asked to answer a follow-up questionnaire. Recurrences were included when confirmed by ultrasound, contrast venography, or computed tomography according to the discharge letters (Flinterman et al., "Recurrent thrombosis and survival after a first venous thrombosis of the upper extremity", Circulation. 2008; 118: 1366-72). Information on patients with active cancer at the time of first VT was obtained from the baseline questionnaire and from the discharge letters of the first VT (Blom et al., "Malignancies, prothrombotic mutations, and the risk of venous thrombosis", JAMA. 2005; 293: 715-22).

Genetic Analysis

Blood samples were taken at least three months after discontinuation of vitamin K antagonist treatment for the first thrombotic event. DNA was collected with buccal swabs from patients who were unable to give a blood sample and from all patients who were included beginning in June 2002 (Blom et al., "Malignancies, prothrombotic mutations, and the risk of venous thrombosis", JAMA. 2005; 293: 715-22). SNP genotypes were determined by allele-specific real-time PCR (Germer et al., "High-throughput SNP allele-frequency determination in pooled DNA samples by kinetic PCR", Genome Res. 2000; 10: 258-66) in a core laboratory; genotype distributions did not deviate from Hardy Weinberg expectations among controls ($P_{exact}$>0.01) (Weir, Genetic Data Analysis II. Sunderland: Sinauer Associates Inc., 1996).

Statistical Analysis

*Recurrent VT analysis*

Cumulative incidence was estimated by the Kaplan-Meier technique. Incidence rates were the number of new VT events over the total number of person-years. Person-years were calculated from date of first VT event and from discontinuation of the initial vitamin K antagonist treatment until recurrent VT event, death, or end of study, whichever came first. Participants who died during follow-up of a cause other than VT were censored at the date of death. Patients who were not able to complete the inquiry form were censored at their last contact and considered study withdrawals. The end-of-study date was October 1, 2006. Hazard ratios (HRs) were estimated with a Cox proportional-hazards model after patients had discontinued vitamin K antagonist treatment. Adjustments were made for age and sex. No adjustment was made for race because the follow-up study included 95% whites. False discovery rate estimates were used to control for false-positive associations among the group of SNPs in the recurrent VT study (Benjamini et al., Journal of the Royal Statistical Society. 1995; Serials B: 1289-300). Analyses were done using SAS version 9 (SAS Institute Inc, Cary, North Carolina) and SPSS for Windows, 14.0.2 (SPSS Inc, Chicago, Illinois). False discovery rates were estimated using the 2-sided, unadjusted P value from the additive model.

*Primary VT analysis*

Logistic regression models were used to calculate the odds ratio (OR), 95% confidence interval (95% CI), and 2-sided P value for the association of each SNP with VT and to adjust for age and sex. For each SNP, the OR per genotype was calculated relative to noncarriers of the risk allele. For SNPs on the X chromosome, the analysis was conducted separately in men and women. Analyses were done using SAS version 9 (SAS Institute Inc, Cary, North Carolina) and SPSS for Windows, 14.0.2 (SPSS Inc, Chicago, Illinois).

Results

The SNPs identified as being associated with VT, particularly recurrent VT, are provided in Table 8.

Example 5: SNPs associated with risk for VT

Table 9 provides 10 SNPs that were associated with VT risk in the MEGA-1 subset of the MEGA study. These SNPs were specifically associated with primary VT risk in MEGA-1, and are also useful for determining risk for recurrent VT.

The MEGA study, including the MEGA-1 subset, is described in Blom et al., JAMA 2005; 293(6):715-722, as well as in Examples 1 and 2 above.

Example 6: Four-marker panel for determining risk of VT, particularly recurrent VT

Four of the SNPs identified herein as being associated with recurrent VT, as well as primary VT, were combined into a panel for determining VT risk, particularly recurrent VT risk. The panel (referred to herein as the "four-marker panel", or "GRS" in Tables 11-12) comprised the following four SNPs (genes): rs6025 (F5), rs2066865 (FGG), rs8176719 (ABO), and rs2036914 (F11).

Risk genotypes for each of these four SNPs are AG+AA for rs6025 (F5), GT+GG for rs8176719 (ABO), AG+AA for rs2066865 (FGG), and CT+CC for rs2036914 (F11).

Equally weighting these four SNPs, it was found that the individuals in the top quartile (>90th percentile) had a two-fold increase (HR = 2.04) in risk for recurrent VT compared with the bottom quartile group (<35th percentile) (see Table 11).

Table 11: Association of four-marker panel with recurrent VT

| GRS Percentile | Events | Total | HR | 95%CI | P value |
|---|---|---|---|---|---|
| >=90 | 81 | 361 | 2.04 | 1.56-2.67 | <0.0001 |
| >35 and <90 | 326 | 1998 | 1.42 | 1.18-1.72 | 0.0003 |
| <=35 | 158 | 1327 | Ref | | |
| Percentile >=90: Above 90th percentile (based on number of risk allele carriers) | | | | | |

Further, using the four-marker panel in combination with an individual's gender, it was found that individuals in the top quartile (>84th percentile) had a three-fold increase (HR =3.1) in risk for recurrent VT compared with the bottom quartile group (<43th percentile) (see Table 12).

Table 12: Association of four-marker panel, in combination with gender, with recurrent VT

| GRS Percentile | Events | Total | HR | 95%CI | P value |
|---|---|---|---|---|---|
| >=84 | 155 | 575 | 3.1 | 2.47-3.91 | <0.0001 |
| >43 and <84 | 267 | 1523 | 2.05 | 2.05-1.67 | <0.0001 |
| <=43 | 143 | 1588 | Ref | | |

Thus, this four-marker panel is particularly useful for determining an individual's risk for developing VT, particularly recurrent VT (as well as primary VT).

In further exemplary embodiments of the four-marker panel, additional markers are assayed in combination with the four markers (particularly additional markers selected from those disclosed herein), In further exemplary embodiments of the four-marker panel, any one, two, or three of the four markers (F5 SNP rs6025, *FGG* SNP rs2066865, *ABO* rs8176719, and *F11* SNP rs2036914) are assayed, optionally in combination with additional markers (particularly additional markers selected from those disclosed herein). For example, other markers can be substituted for any one or more markers of the four-marker panel. In certain exemplary embodiments, one or more other SNPs in the *F11* gene (such as SNP rs2289252) are substituted for *F11* SNP rs2()36914 (or assayed in addition to rs2036914). In certain embodiments, *PTPN21* SNP rs2274736 (disclosed herein) is added to the four-marker panel or substituted in place of one of the markers of the four-marker panel. Additionally, in certain embodiments, *F2* SNP rs1799963 is added to the four-marker panel or substituted in place of one of the markers of the four-marker panel.

In additional embodiments, one or more protein biomarkers can be assayed in combination with the four-marker panel, or a subset of the four-marker panel (and/or any of the other SNPs disclosed herein). For example, measurement of factor XI protein levels can be assayed in combination with the four-marker panel, or can be substituted in place of assaying *F11* SNP rs2036914 (or *F11* SNP rs2289252), or can be measured in conjunction with any of the other SNPs disclosed herein.

Similarly, measurement of factor VIII protein levels can be assayed in combination with the four-marker panel or can be substituted in place of assaying *ABO* SNP rs8176719 (or can be measured in conjunction with any of the other SNPs disclosed herein). *ABO* SNP rs8176719 is associated with factor VIII protein levels, and factor VIII protein levels are associated with VT risk.

Fibrinogen gamma and/or fibrinogen gamma primer protein levels can also be measured in conjunction with the four-marker panel or a subset thereof (or can be measured in conjunction with any of the other SNPs disclosed herein).

Example 7: LD SNPs associated with VT risk and statin response

Another investigation was conducted to identify additional SNPs that are calculated to be in linkage disequilibrium (LD) with certain "interrogated SNPs" that have been found to be associated with VT risk and/or response to statin treatment (particularly for reducing the risk of VT), as described herein and shown in the tables. The interrogated SNPs are shown in column 1 (which indicates the hCV identification numbers of each interrogated SNP) and column 2 (which indicates the public rs identification numbers of each interrogated SNP) of Table 3. The methodology is described earlier in the instant application. To summarize briefly, the power threshold (*T*) was set at an appropriate level, such as 51%, for detecting disease association using LD markers. This power threshold is based on equation (31) above, which incorporates allele frequency data from previous disease association studies, the predicted error rate for not detecting truly disease-associated markers, and a significance level of 0.05. Using this power calculation and the sample size, a threshold level of LD, or $r^2$ value, was derived for each interrogated SNP ( $r_T^2$, equations (32) and (33) above). The threshold $r_T^2$ value is the minimum value of linkage disequilibrium between the interrogated SNP and its LD SNPs possible such that the non-interrogated SNP still retains a power greater or equal to *T* for detecting disease association.

Based on the above methodology, LD SNPs were found for the interrogated SNPs. Several exemplary LD SNPs for the interrogated SNPs are listed in Table 3; each LD SNP is associated with its respective interrogated SNP. Also shown are the public SNP IDs (rs numbers) for the interrogated and LD SNPs, when available, and the threshold $r^2$ value and the power used to determine this, and the $r^2$ value of linkage disequilibrium between the interrogated SNP and its corresponding LD SNP. As an example in Table 3, the interrogated SNP rs2066865 (hCV11503414) was calculated to be in LD with rs2066864 (hCV11503416) at an $r^2$ value of 1, based on a 51 % power calculation, thus establishing the latter SNP as a marker associated with statin response as well.

In general, the threshold $r_T^2$ value can be set such that one of ordinary skill in the art would consider that any two SNPs having an $r^2$ value greater than or equal to the threshold $r_T^2$ value would be in sufficient LD with each other such that either SNP is useful for the same utilities, such as determining an individual's response to statin treatment. For example, in various embodiments, the threshold $r_T^2$ value used to classify SNPs as being in sufficient LD with an interrogated SNP (such that these LD SNPs can be used for the same utilities as the interrogated SNP, for example) can be set at, for example, 0.7, 0.75, 0.8, 0.85, 0.9, 0.95, 0.96, 0.97, 0.98, 0.99, 1, *etc.* (or any other $r^2$ value in-between these values). Threshold $r_T^2$ values may be utilized with or without considering power or other calculations.

Table1_CD0000029ORD_reformatted.txt

Gene Number: 1 / Gene Symbol ABO – 28 / Gene Name: ABO blood group (transferase A, alpha 1-3-N-acetylgalactosaminyltransf
erase; transferase B, alpha 1-3-galactosyltransferase) / Public Transcript
Accession: NM_020469 / Public Protein Accession: NP_065202 / Chromosome: 9 /
OMIM NUMBER: 110300 / OMIM Information: [Blood group, ABO system] (3) //
Transcript Sequence (SEQ ID NO: 1): / Protein Sequence (SEQ ID NO: 85): /
SNP Information /
Context (SEQ ID NO: 169):
GGTGGGCCACCGTGTCCACTACTATGTCTTCACCGACCAGCCGGCCGCGGTGCCCCGCGTGACGCTGGGGACCGGTCGGCAGC
TGTCAGTGCTGGAGGTG
S
GCGCCTACAAGCGCTGGCAGGACGTGTCCATGCGCCGCATGGAGATGATCAGTGACTTCTGCGAGCGGCGCTTCCTCAGCGAG
GTGGATTACCTGGTGTG
Celera SNP ID: hCV27859399 / Public SNP ID: rs7853989 / SNP Chromosome
Position: 136131592 / SNP in Transcript Sequence SEQ ID NO: 1 / SNP Position
Transcript: 551 / SNP Source: HGMD; dbSNP; HapMap / Population(Allele,Count):
Gaucasian (C,108|G,12) / SNP Type: Silent Mutation / Protein Coding: SEQ ID
NO: 85, at position 175,(V,GTG) (V,GTC)
Context (SEQ ID NO: 170):
CGCGTCTCGTTGCCAAGGATGGTCTACCCCCAGCCAAAGGTGCTGACACCGTGTAGGAAGGATGTCCTCGTGGTGACCCCTTG
GCTGGCTCCCATTGTCT
M
GGAGGGCACATTCAACATCGACATCCTCAACGAGCAGTTCAGGCTCCAGAACACCACCATTGGGTTAACTGTGTTTGCCATCA
AGAAATACGTGGCTTTC
Celera SNP ID: hDV77258202 / Public SNP ID: rs8176719 / SNP Chromosome
Position: 136132908 / SNP in Transcript Sequence SEQ ID NO: 1 / SNP Position
Transcript: 312 / SNP Source: CDX; dbSNP / Population(Allele,Count): Caucasian
(A,4|C,18) / SNP Type: Frame Shift Indel / Protein Coding: SEQ ID NO: 85, at
position None /
Context (SEQ ID NO: 171):
GGCCCGCCGTCCTGAGGAAGCTGAGGTTCACTGCGGTGCCCAAGAACCACCAGGCGGTCCGGAACCCGTGAGCGGCTGCCAGG
GGCTCTGGGAGGGCTGC
R
GGCAGCCCCGTCCCCCTCCCGCCCTTGGTTTTAGCAGAACGGGTAAACTCTGTTTCCTTTGTCCGTCCTGTTGTGAGTAACTG
AAGCCTAGGCCCCGTCC
Celera SNP ID: hCV25610771 / Public SNP ID: rs8176751 / SNP Chromosome
Position: 136131022 / SNP in Transcript Sequence SEQ ID NO: 1 / SNP Position
Transcript: 1120 / Related Interrogated SNP: hCV25610857 / Related Interrogated
SNP: hCV27859399 / SNP Source: Applera / Population(Allele,Count): Gaucasian
(G,16|A,2) African American (G,0|A,4) total (G,16|A,6) / SNP Type: UTR3 / SNP
Source: Applera / Population(Allele,Count): Gaucasian (G,26|A,4) African
American (G,14|A,0) total (G,40|A,4) / SNP Type: UTR3 / SNP Source: dbSNP;
Applera / Population(Allele,Count): Gaucasian (G,203|A,17) / SNP Type: UTR3 /
Context (SEQ ID NO: 172):
GGCTTCTACGGAAGCAGCCGGGAGGCCTTCACCTACGAGCGCCGGCCCCAGTCCCAGGCCTACATCCCCAAGGACGAGGGCGA
TTTCTACTACCTGGGGG
S
GTTCTTCGGGGGGTCGGTGCAAGAGGTGCAGCGGCTCACCAGGGCCTGCCACCAGGCCATGATGGTCGACCAGGCCAACGGCA
TCGAGGCCGTGTGGCAC
Celera SNP ID: hCV25610773 / Public SNP ID: rs8176747 / SNP Chromosome
Position: 136131315 / SNP in Transcript Sequence SEQ ID NO: 1 / SNP Position
Transcript: 828 / Related Interrogated SNP: hCV25610857 / Related Interrogated
SNP: hCV27859399 / SNP Source: Applera / Population(Allele,Count): Gaucasian
(G,36|C,4) African American (G,25|C,13) total (G,61|C,17) / SNP Type: Missense
Mutation / Protein Coding: SEQ ID NO: 85, at position 268,(G,GGG) (R,CGG) / SNP
Source: Applera / Population(Allele,Count): Gaucasian (G,36|C,4) African
American (G,28|C,10) total (G,64|C,14) / SNP Type: Missense Mutation / Protein
Coding: SEQ ID NO: 85, at position 268,(G,GGG) (R,CGG) / SNP Source: HGMD;
dbSNP; HapMap / Population(Allele,Count): Gaucasian (G,98|C,14) / SNP Type:
Missense Mutation / Protein Coding: SEQ ID NO: 85, at position 268,(G,GGG)
(R,CGG) /

Context          (SEQ ID NO: 173):
TGCAGCGGCTCACCAGGGCCTGCCACCAGGCCATGATGGTCGACCAGGCCAACGGCATCGAGGCCGTGTGGCACGACGAGAGC
CACCTGAACAAGTACCT
R
CTGCGCCACAAACCCACCAAGGTGCTCTCCCCCGAGTACTTGTGGGACCAGCAGCTGCTGGGCTGGCCCGCCGTCCTGAGGAA
GCTGAGGTTCACTGCGG
Celera SNP ID:          hCV25610781 / Public SNP ID:          rs8176749 / SNP Chromosome
Position:  136131188 / SNP in Transcript Sequence          SEQ ID NO: 1 /
SNP Position Transcript:  955 / Related Interrogated SNP:          hCV25610857 / Related
Interrogated SNP:          hCV27859399 / SNP Source:          Applera / Population(Allele,Count):
Gaucasian (G,36|A,4)    African American (G,25|A,13)    total (G,61|A,17) / SNP Type:
Missense Mutation / Protein Coding:          SEQ ID NO: 85, at position 310,(L,CTG)
(P,CCG) / SNP Source:          Applera / Population(Allele,Count):          Gaucasian (G,36|A,4)
African American (G,28|A,10)    total (G,64|A,14) / SNP Type:          Missense Mutation /
Protein Coding:          SEQ ID NO: 85, at position 310,(L,CTG) (P,CCG) / SNP Source:
dbSNP;  HapMap / Population(Allele,Count):          Gaucasian (G,209|A,17) / SNP Type:
Missense Mutation / Protein Coding:          SEQ ID NO: 85, at position 310,(L,CTG)
(P,CCG) /
Context          (SEQ ID NO: 174):
CAGCGAGGTGGATTACCTGGTGTGCGTGGACGTGGACATGGAGTTCCGCGACCACGTGGGCGTGGAGATCCTGACTCCGCTGT
TCGGCACCCTGCACCCC
R
GCTTCTACGGAAGCAGCCGGGAGGCCTTCACCTACGAGCGCCGGCCCCAGTCCCAGGCCTACATCCCCAAGGACGAGGGCGAT
TTCTACTACCTGGGGGGG
Celera SNP ID: hCV25610791 / Public SNP ID:  rs8176743 / SNP Chromosome Position:
136131415 / SNP in Transcript Sequence  SEQ ID NO: 1 / SNP Position Transcript:
728 / Related Interrogated SNP:  hCV25610857 / Related Interrogated SNP:
hCV27859399 / SNP Source:  Applera / Population(Allele,Count):  Gaucasian
(G,36|A,4)  African American (G,27|A,9)  total (G,63|A,13) / SNP Type:  Silent
Mutation / Protein Coding:  SEQ ID NO: 85, at position 234,(P,CCC) (P,CCT) / SNP
Source:  HGMD; dbSNP; Applera / Population(Allele,Count):  Gaucasian (G,205|A,17)
/ SNP Type:  Silent Mutation / Protein Coding:  SEQ ID NO: 85, at position
234,(P,CCC) (P,CCT) /
Context          (SEQ ID NO: 175):
TGCCAAGGATGGTCTACCCCCAGCCAAAGGTGCTGACACCGTGTAGGAAGGATGTCCTCGTGGTGACCCCTTGGCTGGCTCCC
ATTGTCTGGGAGGGCAC
R
TTCAACATCGACATCCTCAACGAGCAGTTCAGGCTCCAGAACACCACCATTGGGTTAACTGTGTTTGCCATCAAGAAATACGT
GGCTTTCCTGAAGCTGT
Celera SNP ID: hCV3183100 / Public SNP ID: rs8176720 / SNP Chromosome Position:
136132873 / SNP in Transcript Sequence  SEQ ID NO: 1 / SNP Position Transcript:
322 / Related Interrogated SNP:  hCV25610857 / Related Interrogated SNP:
hCV27859399 / SNP Source:  Applera / Population(Allele,Count):  Gaucasian
(G,12|A,28)  African American (G,14|A,16)  total (G,26|A,44) // SNP Type:
Missense Mutation / Protein Coding:  SEQ ID NO: 85, at position 99,(T,ACA)
(I,ATA) / SNP Source:  dbSNP; Celera;  HapMap / Population(Allele,Count):
Gaucasian (A,142|G,82) / SNP Type:  Missense Mutation / Protein Coding: SEQ ID NO:
85, at position 99,(T,ACA) (I,ATA) //


Gene Number:  2 / Gene Symbol ACVR2B - 93 / Gene Name:  activin A receptor, type
IIB / Public Transcript Accession:  NM_001106 / Public Protein Accession:
NP_001097 / Chromosome:  3 / OMIM NUMBER:  602730 / OMIM Information:  Left-right
axis malformations (3) // Transcript Sequence (SEQ ID NO: 2) // Protein Sequence
(SEQ ID NO: 86): // SNP Information //

Context          (SEQ ID NO: 176):
GAACAGAGCTACACTGGACTCGGGCACATTCGGAGCAGCATCCTTTAGTATGGAGGCTACTTCTCAGGTAACCAGGAATTGAG
GGGAAGGACCTTGTGGA
R
GCCGAGCATTAACAGCAAGAGCGGGGTTTGGAGAAAGTCTGAGATTGGGTGCAGCCCTGACTTACCTGCTGGCCCTGACCAGT
TTCTTTTCACTAACTTG

Celera SNP ID: hCV32001449 / Public SNP ID: rs12636077 / SNP Chromosome Position: 38525588 / SNP in Transcript Sequence SEQ ID NO: 2 / SNP Position Transcript: 2328 / Related Interrogated SNP: hCV15949414 / SNP Source: dbSNP; HapMap / Population(Allele,Count): Caucasian (G,114|A,6) / SNP Type: UTR3 //

Context (SEQ ID NO: 177):
TTACTGCCCCGGGGTCCTTTCCTCTGGGTGTTGGGGCACAGGGTGCTATGGGAGGCCCATTTGCTTCCCTCTCGGAGCTCAGT
TTTTGCTTCATGGGTCA
R
AATGTGGGCTGGCCAAGTGGTTACAGGAACAGGGTTTCGGTAAGCTATGTTGTCTTTTTTTTTTTTTTTTTTTTTTTTTTTTTAA
TGGTTTGATTTTTGTGCT

Celera SNP ID: hDV71601922 / Public SNP ID: rs17037775 / SNP Chromosome Position: 38527103 / SNP in Transcript Sequence SEQ ID NO: 2 / SNP Position Transcript: 3843 / Related Interrogated SNP: hCV15949414 / SNP Source: dbSNP; HapMap / Population(Allele,Count): Caucasian (A,114|G,6) / SNP Type: UTR3 //

Gene Number: 3 / Gene Symbol APOH - 350 / Gene Name: apolipoprotein H (beta-2-glycoprotein I) / Public Transcript Accession: NM_000042 / Public Protein Accession: NP_000033 / Chromosome: 17 / OMIM NUMBER: 138700 / OMIM Information: [Apolipoprotein H deficiency] (3) // Transcript Sequence (SEQ ID NO: 3): // Protein Sequence (SEQ ID NO: 87): // SNP Information //
Context (SEQ ID NO: 178):
ATAAGGAAAAGAAGTGTAGCTATACAGAGGATGCTCAGTGTATAGATGGCACTATCGAAGTCCCCAAATGCTTCAAGGAACAC
AGTTCTCTGGCTTTTTG
S
AAAACTGATGCATCCGATGTAAAGCCATGCTAAGGTGGTTTTCAGATTCCACACAAAATGTCACACTTGTTTCTTGTTCATCC
AAGGAACCTAATTGAAA

Celera SNP ID: hCV2303891 / Public SNP ID: rs1801690 / SNP Chromosome Position: 64208285 / SNP in Transcript Sequence SEQ ID NO: 3 / SNP Position Transcript: 1064 / SNP Source: HGMD; dbSNP; HapMap / Population(Allele,Count): Gaucasian (G,213|C,13) / SNP Type: Missense Mutation / Protein Coding: SEQ ID NO: 87, at position 335, (W,TGG) (S,TCG) //

Context (SEQ ID NO: 179):
GGCTCTGTCTTTTTAGCAGACGAAAAC
M
ACTTTGGTAGTGCCAGTGTGACTCATCCACAATGATTTCTCCAGTGCTCATCTTGTTCTCGAGTTTTCTCTGCCATGTTGCTA
TTGCAGGACGGACCTGT

Celera SNP ID: hCV27842286 / Public SNP ID: rs8178822 / SNP Chromosome Position: 64225529 / SNP in Transcript Sequence SEQ ID NO: 3 / SNP Position Transcript: 28 / Related Interrogated SNP: hCV2303891 / SNP Source: Applera / Population(Allele,Count): Caucasian (C,37|A,1) African American (C,33|A,5) total (C,70|A,6) / SNP Type: UTR5 / SNP Source: dbSNP; HapMap / Population(Allele,Count): Caucasian (C,212|A,14) / SNP Type: UTR5 //

Gene Number: 4 / Gene Symbol AQP2 - 359 / Gene Name: aquaporin 2 (collecting duct) / Public Transcript Accession: NM_000486 / Public Protein Accession: NP_000477 / Chromosome: 12 / OMIM NUMBER: 107777 / OMIM Information: Diabetes insipidus, nephrogenic, autosomal recessive, 222000 (3);/Diab / etes insipidus, nephrogenic, autosomal dominant, 125800 (3) // / Transcript Sequence (SEQ ID NO: 4): // Protein Sequence (SEQ ID NO: 88): // SNP Information //
Context (SEQ ID NO: 180):
AAATGACTTTGCACCTGCAAATGGTGCTTCAATGTCGTTTTGTATCTTTAAGCTGTGATGTTGTATATATACAATGCCTCCCA
GCTAGACTGTAAGCCCT
Y
TGGGGACAAGGGCTGCTTCCAGTTCTTGGATGGCGTTCTCCTATCTACCTTCCTTCAACTGCTCTGCATATAATAAGCACTCA
ATAAATGCTCATTTGCC

Celera SNP ID: hCV15835026 / Public SNP ID: rs2878772 / SNP Chromosome Position: 50352559 / SNP in Transcript Sequence SEQ ID NO: 4 / SNP Position Transcript: 4074 / Related Interrogated SNP: hCV25597241 / SNP Source: dbSNP; HGBASE / Population(Allele,Count): Caucasian (C,8|T,112) / SNP Type: UTR3 //

Gene Number: 5 / Gene Symbol ASAH1 - 427 / Gene Name: N-acylsphingosine

amidohydrolase (acid ceramidase) 1 / Public Transcript Accession: NM_001127505 / Public Protein Accession: NP_001120977 / Chromosome: 8 / OMIM NUMBER: / OMIM Information: // Transcript Sequence (SEQ ID NO: 5): // Protein Sequence (SEQ ID NO: 89): // SNP Information //
Context (SEQ ID NO: 181):
TCAATTCTGGGATTTGGCAGCTTTGCTGTGAAAGCCCAATGGACAGAGGACTGCAGAAAATCAACCTATCCTCCTTCAGGACC
AACTGTCTTCCCTGCTG
R
TATAAGGTACAGAGGTGCAGTTCCATGGTACACCATAAATCTTGACTTACCACCCTACAAAAGATGGCATGAATTGATGCTTG
ACAAGGCACCAGTGCCT
Celera SNP ID: hCV31495915 / Public SNP ID: rs3753115 / SNP Chromosome Position: 17930772 / SNP in Transcript Sequence SEQ ID NO: 5 / SNP Position Transcript: 385 / Related Interrogated SNP: hCV2442143 / SNP Source: dbSNP; HGBASE / Population(Allele,Count): Gaucasian (G,119|A,107) / SNP Type: Missense Mutation / Protein Coding: SEQ ID NO: 89, at position 63,(V,GTT) (I,ATT) //

Gene Number: 6 / Gene Symbol F2 - 2147 / Gene Name: coagulation factor II (thrombin) / Public Transcript Accession: NM_000506 / Public Protein Accession: NP_000497 / Chromosome: 11 / OMIM NUMBER: 176930 / OMIM Information: Hypoprothrombinemia (3); Dysprothrombinemia (3);/Hyperprothrombinemia / (3) // / Transcript Sequence (SEQ ID NO: 6): // Protein Sequence (SEQ ID NO: 90): // SNP Information //
Context (SEQ ID NO: 182):
GTAGGGGGCCACTCATATTCTGGGCTCCTGGAACCAATCCCGTGAAAGAATTATTTTTGTGTTTCTAAAACTATGGTTCCCAA
TAAAAGTGACTCTCAGC
R
AAAAAAAAA
Celera SNP ID: hCV8726802 / Public SNP ID: rs1799963 / SNP Chromosome Position: 46761055 / SNP in Transcript Sequence SEQ ID NO: 6 / SNP Position Transcript: 2009 / SNP Source: HGBASE;dbSNP / Population(Allele,Count): no_pop (A,-|G,-) / SNP Type: UTR3 //

Gene Number: 7 / Gene Symbol F2RL1 - 2150 / Gene Name: coagulation factor II (thrombin) receptor-like 1 / Public Transcript Accession: NM_005242 / Public Protein Accession: NP_005233 / Chromosome: 5 / OMIM NUMBER: 600933 / OMIM Information: // Transcript Sequence (SEQ ID NO: 7): // Protein Sequence (SEQ ID NO: 91): // SNP Information //
Context (SEQ ID NO: 183):
ACCCGCCCTGGGGAGGCGCGCAGCAGAGGCTCCGATTCGGGGCAGGTGAGAGGCTGACTTTCTCTCGGTGCGTCCAGTGGAGC
TCTGAGTTTCGAATCGG
Y
GGCGGCGGATTCCCCGCGCGCCCGGCGTCGGGGGCTTCCAGGAGGATGCGGAGCCCCAGCGCGGCGTGGCTGCTGGGGGCCGCC
ATCCTGCTAGCAGCCTC
Celera SNP ID: hCV2829819 / Public SNP ID: rs1529505 / SNP Chromosome Position: 76114963 / SNP in Transcript Sequence SEQ ID NO: 7 / SNP Position Transcript: 131 / SNP Source: Applera / Population(Allele,Count): Caucasian (C,20|T,20) African American (C,8|T,28) total (C,28|T,48) / SNP Type: UTR5 / SNP Source: Applera / Population(Allele,Count): Caucasian (C,5|T,5) African American (C,5|T,1) total (C,10|T,6) / SNP Type: UTR5 / SNP Source: dbSNP; Celera; HapMap; ABI_Val; HGBASE / Population(Allele,Count): Caucasian (C,104|T,122) / SNP Type: UTR5 //

Gene Number: 8 / Gene Symbol F5 - 2153 / Gene Name: coagulation factor V (proaccelerin, labile factor) / Public Transcript Accession: NM_000130 / Public Protein Accession: NP_000121 / Chromosome: 1 / OMIM NUMBER: 227400 / OMIM Information: Hemorrhagic diathesis due to factor V deficiency (3);/{Thromboembolism / susceptibility due to factor V Leiden} (3); {Thrombophilia due to factor V Liverpool} (3) // / Transcript Sequence (SEQ ID NO: 8): // Protein Sequence (SEQ ID NO: 92): // SNP Information //
Context (SEQ ID NO: 184):
TGAAATAAACCACCCCAAAACTTAGTGTTAAAAGAGCAACCAAAAAAAATTTATGTGAGATTATGGATTTGTTACTTAGCTTG
ATTTAATCATCCTGTAA
Y

GTGTACATATATCAAAATGTTATGTATACCATAAATATATAAAATTTTATCAACGAAATTCATAACAATCTCTCAGACCACAG
AGAAATCAAATTAGAAC
Celera SNP ID: hCV11975196 / Public SNP ID: rs2040444 / SNP Chromosome
Position: 169482436 / SNP in Transcript Sequence SEQ ID NO: 8 / SNP Position
Transcript: 7935 / Related Interrogated SNP: hCV8919444 / SNP Source: dbSNP;
HapMap / Population(Allele,Count): Caucasian (C,59|T,59) / SNP Type: UTR3 //
Context (SEQ ID NO: 185):
AGAAACTACCAGAGTTACCTGTTGCCCATACCAGTGGGGTAAGCCCTATGAATGTATATGAGAGTTTCAAACATCCACAAAAC
ATTGGCTTTCTAATATT
Y

GTATTCCCACTATTCCTTTCTTTTCATGATTCATGTCATTGTCCCATCAACATTTCTAAGATTTCCATTCCGTTAAGAGCAAA
AGAGAATGTTGGAAGGT
Celera SNP ID: hCV15847759 / Public SNP ID: rs2187952 / SNP Chromosome
Position: 169481950 / SNP in Transcript Sequence SEQ ID NO: 8 / SNP Position
Transcript: 8421 / Related Interrogated SNP: hCV8919444 / SNP Source: dbSNP;
Celera; HapMap; HGBASE / Population(Allele,Count): Caucasian (C,88|T,30) / SNP
Type: UTR3 //

Gene Number: 9 / Gene Symbol F9 - 2158 / Gene Name: coagulation factor IX /
Public Transcript Accession: NM_000133 / Public Protein Accession: NP_000124 /
Chromosome: X / OMIM NUMBER: 306900 / OMIM Information: Hemophilia B (3);
Warfarin sensitivity (3) // Transcript Sequence (SEQ ID NO: 9): // Protein
Sequence (SEQ ID NO: 93): // SNP Information //
Context (SEQ ID NO: 186):
TATCGACTTGCAGAAAACCAGAAGTCCTGTGAACCAGCAGTGCCATTTCCATGTGGAAGAGTTTCTGTTTCACAAACTTCTAA
GCTCACCCGTGCTGAGA
R
TGTTTTTCCTGATGTGGACTATGTAAATTCTACTGAAGCTGAAACCATTTTGGATAACATCACTCAAAGCACCCAATCATTTA
ATGACTTCACTCGGGTT
Celera SNP ID: hCV596331 / Public SNP ID: rs6048 / SNP Chromosome Position:
138633280 / SNP in Transcript Sequence SEQ ID NO: 9 / SNP Position Transcript:
610 / SNP Source: dbSNP; HapMap; / Population(Allele,Count): Caucasian
(A,126|G,41) / SNP Type: Missense Mutation / Protein Coding: SEQ ID NO: 93, at
position 194,(T,ACT) (A,GCT) //
Context (SEQ ID NO: 187):
CCTTTTACCCTCCATGGTCGTTAAAGGAGAGATGGGGAGCATCATTCTGTTATACTTCTGTACACAGTTATACATGTCTATCA
AACCCAGACTTGCTTCC
R
TAGTGGAGACTTGCTTTTCAGAACATAGGGATGAAGTAAGGTGCCTGAAAAGTTTGGGGGAAAAGTTTCTTTCAGAGAGTTAA
GTTATTTTATATATATA
Celera SNP ID: hCV2288124 / Public SNP ID: rs440051 / SNP Chromosome
Position: 138644917 / SNP in Transcript Sequence SEQ ID NO: 9 / SNP Position
Transcript: 2102 / Related Interrogated SNP: hCV596331 / SNP Source: dbSNP;
HapMap; ABI_Val; HGBASE; / Population(Allele,Count): Caucasian (G,142|A,27) / SNP
Type: UTR3 //

Gene Number: 10 / Gene Symbol F11 - 2160 / Gene Name: coagulation factor XI /
Public Transcript Accession: NM_000128 / Public Protein Accession: NP_000119 /
Chromosome: 4 / OMIM NUMBER: 264900 / OMIM Information: Factor XI deficiency,
autosomal recessive (3); Factor XI deficiency,/a / utosomal dominant (3) // /
Transcript Sequence (SEQ ID NO: 10): // Protein Sequence (SEQ ID NO: 94): //
SNP Information //
Context (SEQ ID NO: 188):
AGGCATCTCTGGATACACATTAAGGTTGTGTAAAATGGATAATGAGTGTACCACCAAAATCAAGCCCAGGATCGTTGGAGGAA
CTGCGTCTGTTCGTGGT
Y
AGTGGCCGTGGCAGGTGACCCTGCACACAACCTCACCCACTCAGAGACACCTGTGTGGAGGCTCCATCATTGGAAACCAGTGG
ATATTAACAGCCGCTCA
Celera SNP ID: hCV11786301 / Public SNP ID: rs5970 / SNP Chromosome
Position: 187205301 / SNP in Transcript Sequence SEQ ID NO: 10 / SNP Position
Transcript: 1525 / Related Interrogated SNP: hCV27474895 / Related Interrogated
SNP: hCV3230038 / Related Interrogated SNP: hCV25474413 / SNP Source: Applera
/ Population(Allele,Count): Caucasian (C,4|T,34) African American (C,9|T,29)

total (C,13|T,63) / SNP Type: Silent Rare Codon / Protein Coding: SEQ ID NO: 94, at position 397,(G,GGT) (G,GGC) / SNP Source: Applera / Population(Allele,Count): Caucasian (C,5|T,31) African American (C,6|T,28) total (C,11|T,59) / SNP Type: Silent Rare Codon / Protein Coding: SEQ ID NO: 94, at position 397,(G,GGT) (G,GGC) / SNP Source: dbSNP; Celera; Applera / Population(Allele,Count): Caucasian (T,187|C,33) / SNP Type: Silent Rare Codon / Protein Coding: SEQ ID NO: 94, at position 397,(G,GGT) (G,GGC) // Context (SEQ ID NO: 189): CGGTTGCTTGTTTTTTACCTTCTTTTCCCAGGAATGGCCCAAAGAATCTCAAAGAAATCTTTGTCTCCTTAAAACATCTGAGA GTGGATTGCCCAGTACA

R

GCATTAAAAAGAGCAAAGCTCTTTCTGGTTTCAGTCTACAAAGCTGCAGGCACAGCATCCCAGTGTTCTGCCATTCTTCATTT TACCATGACACTGATTT

Celera SNP ID: hCV3230032 / Public SNP ID: rs5974 / SNP Chromosome Position: 187201211 / SNP in Transcript Sequence SEQ ID NO: 10 / SNP Position Transcript: 1135 / Related Interrogated SNP: hCV27474895 / Related Interrogated SNP: hCV3230038 / Related Interrogated SNP: hCV25474413 / SNP Source: Applera / Population(Allele,Count): Caucasian (A,25|G,5) African American (A,30|G,8) total (A,55|G,13) / SNP Type: Silent Rare Codon / Protein Coding: SEQ ID NO: 94, at position 267,(T,ACA) (T,ACG) / SNP Source: dbSNP; Celera; HapMap / Population(Allele,Count): Caucasian (A,193|G,33) / SNP Type: Silent Rare Codon / Protein Coding: SEQ ID NO: 94, at position 267,(T,ACA) (T,ACG) // Context (SEQ ID NO: 190): CCAAAACTCCCGTTCTATGATCGTTGTAGTTTGTTTGAGCATTCAGTCTCTTTGTTTTTGATCACGCTTCTATGGAGTCCAAG AATTACCATAAGGCAAT

R

TTTCTGAAGATTACTATATAGGCAGATATAGCAGAAAATAACCAAGTAGTGGCAGTGGGGATCAGGCAGAAGAACTGGTAAAA GAAGCCACCATAAATAG

Celera SNP ID: hCV3230118 / Public SNP ID: rs4253429 / SNP Chromosome Position: 187210033 / SNP in Transcript Sequence SEQ ID NO: 10 / SNP Position Transcript: 2476 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV3230038 / Related Interrogated SNP: hCV8241630 / Related Interrogated SNP: hCV3230113 / Related Interrogated SNP: hCV22272267 / Related Interrogated SNP: hCV32291301 / Related Interrogated SNP: hCV15968043 / SNP Source: dbSNP; Celera; HGBASE / Population(Allele,Count): Caucasian (A,186|G,40) / SNP Type: UTR3 // Context (SEQ ID NO: 191): TGTTTGAGCATTCAGTCTCTTTGTTTTTGATCACGCTTCTATGGAGTCCAAGAATTACCATAAGGCAATATTTCTGAAGATTA CTATATAGGCAGATATA

S

CAGAAAATAACCAAGTAGTGGCAGTGGGGATCAGGCAGAAGAACTGGTAAAAGAAGCCACCATAAATAGATTTGTTCGATGAA AGATGAAAACTGGAAGA

Celera SNP ID: hCV3230119 / Public SNP ID: rs4253430 / SNP Chromosome Position: 187210064 / SNP in Transcript Sequence SEQ ID NO: 10 / SNP Position Transcript: 2507 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV8241630 / Related Interrogated SNP: hCV3230038 / Related Interrogated SNP: hCV3230113 / SNP Source: dbSNP; Celera; HapMap; HGBASE / Population(Allele,Count): Caucasian (G,144|C,82) / SNP Type: UTR3 // Context (SEQ ID NO: 192): AGGGAGCATGCTCAAACCTCCCTAAGACAAGCTGCTGCTGTGACTATGGGCTCCCAAAGAGCTAGATCGTATATTTATTTGAC AAAAATCACCATAGACT

R

CATCCATACTACAGAGAAAAAACAATTAGGGCGCAAATGGATAGTTACAGTAAAGTCTTCAGCAAGCAGCTGCCTGTATTCTA AGCACTGGGATTTTCTG / Celera SNP ID: hCV3230121 / Public SNP ID: rs4253431 / SNP Chromosome Position: 187210620 / SNP in Transcript Sequence SEQ ID NO: 10 / SNP Position Transcript: 3063 / Related Interrogated SNP: hCV27474895 / Related Interrogated SNP: hCV3230038 / Related Interrogated SNP: hCV25474413 / SNP Source: dbSNP; Celera; HGBASE / Population(Allele,Count): Caucasian (G,193|A,33) / SNP Type: UTR3 // Context (SEQ ID NO: 193):

AAGATGAAAACTGGAAGAAAGGAGAACAAAGACAGTCTTCACCATTTTGCAGGAATCTACACTCTGCCTATGTGAACACATTT
CTTTTGTAAAGAAAGAA
W
TTGATTGCATTTAATGGCAGATTTTCAGAATAGTCAGGAATTCTTGTCATTTCCATTTTAAAATATATATTAAAAAAAATCAG
TTCGAGTAGACACGAGC /
Celera SNP ID:        hCV11786307 / Public SNP ID:        rs1062547 / SNP Chromosome
Position:   187210247 / SNP in Transcript Sequence      SEQ ID NO: 10 / SNP Position
Transcript:   2690 / Related Interrogated SNP:   hCV12066124 / Related Interrogated
SNP:   hCV25474413 / Related Interrogated SNP:   hCV27474895 / Related Interrogated
SNP:   hCV27477533 / Related Interrogated SNP:   hCV8241630 / Related Interrogated
SNP:   hCV3230038 / Related Interrogated SNP:   hCV3230113 / SNP Source:   dbSNP;
Celera; HGBASE / Population(Allele,Count):   Caucasian (A,76|T,44) / SNP Type:
UTR3 //
Context          (SEQ ID NO: 194):
AGGCACACAGGCAAAAT
S
AAGTTCTACATCTGTCCCTGTGTATGTCACTTGTTTGAATACGAAATAAAATTAAAAAAAATAAATTCAGTGTATTGAGAAAGC
AAGCAATTCTCTCAAGG
 Celera SNP ID:        hCV27473099 / Public SNP ID:        rs3733403 / SNP Chromosome
Position:   187187135 / SNP in Transcript Sequence      SEQ ID NO: 10 / SNP Position
Transcript:   18 / Related Interrogated SNP:   hCV12066124 / Related Interrogated
SNP:   hCV25474413 / Related Interrogated SNP:   hCV8241630 / Related Interrogated
SNP:   hCV22272267 / Related Interrogated SNP:   hCV27474895 / Related Interrogated
SNP:   hCV27477533 / SNP Source:   dbSNP; HapMap; HGBASE /
Population(Allele,Count):   Caucasian (C,101|G,15) / SNP Type:   UTR5 //

Gene Number:   11 / Gene Symbol        FGA - 2243 / Gene Name:   fibrinogen alpha chain
/ Public Transcript Accession:   NM_000508 / Public Protein Accession:   NP_000499 /
Chromosome:   4 / OMIM NUMBER:   134820 / OMIM Information:   Dysfibrinogenemia,
alpha type, causing bleeding diathesis (3);/Dysfibr / inogenemia, alpha type,
causing recurrent thrombosis (3); Amyloidosis, hereditary renal, 105200 (3); /
Afibrinogenemia, 202400 (3) // / Transcript Sequence   (SEQ ID NO: 11): // Protein
Sequence   (SEQ ID NO: 95): // SNP Information //
Context          (SEQ ID NO: 195):
R
GCAATCCTTTCTTTCAGCTGGAGTGCTCCTCAGGAGCCAGCCCCACCCTTAGAAAAGATGTTTTCCATGAGGATCGTCTGCCT
GGTCCTAAGTGTGGTGG
 Celera SNP ID:        hCV2892870 / Public SNP ID:        rs2070011 / SNP Chromosome
Position:   155511897 / SNP in Transcript Sequence      SEQ ID NO: 11 / SNP Position
Transcript:   1 / Related Interrogated SNP:   hCV11503414 / Related Interrogated
SNP:   hCV11503469 / Related Interrogated SNP:   hCV15860433 / Related Interrogated
SNP:   hCV2892877 / Related Interrogated SNP:   hCV11503470 / SNP Source:   Applera
/ Population(Allele,Count):   Gaucasian (G,23|A,15)  African American (G,24|A,12)
total (G,47|A,27) // / SNP Type:   UTR5 / SNP Source:   Applera /
Population(Allele,Count):   Gaucasian (G,24|A,16)  African American (G,20|A,12)
total (G,44|A,28) // / SNP Type:   UTR5 / SNP Source:   dbSNP; Celera; HGBASE /
Population(Allele,Count):   Gaucasian (A,84|G,142) / SNP Type:   UTR5 //
Context          (SEQ ID NO: 196):
TATAAAGATATACCAAGGGCCATTCAGTACATCAGGAAAGTGGCAGACAGAAGCTTCTCTCTGCAACCTTGAAGACTATTGGT
TTGAGAACTTCTCTTCC
Y
ATACCACCCAAAATCATAATGCCATTGGAAAGCAAAAAGTTGTTTTATCCATTTGATTTGAATTGTTTTAAGCCAATATTTTA
AGGTAAAACTCACTGAA
Celera SNP ID:        hCV2892878 / Public SNP ID:        rs2070022 / SNP Chromosome
Position:   155504948 / SNP in Transcript Sequence      SEQ ID NO: 11 / SNP Position
Transcript:   2987 / Related Interrogated SNP:   hCV11503469 / Related Interrogated
SNP:   hCV15860433 / SNP Source:   dbSNP; Celera; HapMap; ABI_Val; HGBASE /
Population(Allele,Count):   Caucasian (C,194|T,32) / SNP Type:   UTR3 //

Gene Number:   11 / Gene Symbol        FGA - 2243 / Gene Name:   fibrinogen alpha chain /
Public Transcript Accession:   NM_021871 / Public Protein Accession:   NP_068657 /
Chromosome:   4 / OMIM NUMBER:   134820 / OMIM Information:   Dysfibrinogenemia,
alpha type, causing bleeding diathesis (3);/Dysfibr / inogenemia, alpha type,

causing recurrent thrombosis (3); Amyloidosis, hereditary renal, 105200 (3); / Afibrinogenemia, 202400 (3) // / Transcript Sequence (SEQ ID NO: 12): // Protein Sequence (SEQ ID NO: 96): // SNP Information //
Context (SEQ ID NO: 197):
R
GCAATCCTTTCTTTCAGCTGGAGTGCTCCTCAGGAGCCAGCCCCACCCTTAGAAAAGATGTTTTCCATGAGGATCGTCTGCCT
GGTCCTAAGTGTGGTGG
Celera SNP ID: hCV2892870 / Public SNP ID: rs2070011 / SNP Chromosome Position: 155511897 / SNP in Transcript Sequence SEQ ID NO: 12 / SNP Position Transcript: 1 / Related Interrogated SNP: hCV11503414 / Related Interrogated SNP: hCV11503469 / Related Interrogated SNP: hCV15860433 / Related Interrogated SNP: hCV2892877 / Related Interrogated SNP: hCV11503470 / SNP Source: Applera / Population(Allele,Count): Gaucasian (G,23|A,15) African American (G,24|A,12) total (G,47|A,27) // / SNP Type: UTR5 / SNP Source: Applera / Population(Allele,Count): Gaucasian (G,24|A,16) African American (G,20|A,12) total (G,44|A,28) // / SNP Type: UTR5 / SNP Source: dbSNP; Celera; HGBASE / Population(Allele,Count): Gaucasian (A,84|G,142) / SNP Type: UTR5 //

Gene Number: 12 / Gene Symbol FGB - 2244 / Gene Name: fibrinogen beta chain / Public Transcript Accession: NM_001184741 / Public Protein Accession: NP_001171670 / Chromosome: 4 / OMIM NUMBER: 134830 / OMIM Information: Dysfibrinogenemia, beta type (3); Afibrinogenemia, congenital,/202400 / (3); Thrombophilia, dysfibrinogenemic (3) // / Transcript Sequence (SEQ ID NO: 13): // Protein Sequence (SEQ ID NO: 97): // SNP Information //
Context (SEQ ID NO: 198):
TTCTTAAATTCAAATTTTTTAGCTGGAGTAAAAATGGTCCCAGTACCATTTCCTGTTCCCTTCACTATAACCTACATTTTTGT
CACATTAAGTTTTTCCC
Y
ATTCCAGGACAGGTCAGGCCCTTTAAAAAATTTCAACAGCTTTATTGAGATATAATTGATATAATTTAAAAAAATCCTGCACATG
TGTCATGCTGGAGCCCT
Celera SNP ID: hCV7429783 / Public SNP ID: rs1044291 / SNP Chromosome Position: 155493352 / SNP in Transcript Sequence SEQ ID NO: 13 / SNP Position Transcript: 2888 / Related Interrogated SNP: hCV15860433 / Related Interrogated SNP: hCV11503469 / Related Interrogated SNP: hCV11503414 / SNP Source: Applera / Population(Allele,Count): Caucasian (C,27|T,7) African American (C,30|T,4) total (C,57|T,11) / SNP Type: UTR3 / SNP Source: dbSNP; HGBASE / Population(Allele,Count): Caucasian (C,151|T,75) / SNP Type: UTR3 //
Context (SEQ ID NO: 199):
AAAGGAAAAACTGATGTTTAAAAGTCCACTTTTAAAACTATATTTATTTATGTAGGATCTGTCAAAGAAAACTTCCAAAAAGA
TTTATTAATTAAACCAG
M
CTCTGTTGCAATAAGTTAATGTTTTCTTGTTTTTGTAATCCACACATTCAATGAGTTAGGCTTTGCACTTGTAAGGAAGGAGAA
GCGTTCACAACCTCAAA
Celera SNP ID: hCV15971616 / Public SNP ID: rs2227421 / SNP Chromosome Position: 155492224 / SNP in Transcript Sequence SEQ ID NO: 13 / SNP Position Transcript: 1760 / Related Interrogated SNP: hCV11503469 / SNP Source: dbSNP; HGBASE / Population(Allele,Count): Caucasian (A,221|C,48) / SNP Type: UTR3 //

Gene Number: 12 / Gene Symbol FGB - 2244 / Gene Name: fibrinogen beta chain / Public Transcript Accession: NM_005141 / Public Protein Accession: NP_005132 / Chromosome: 4 / OMIM NUMBER: 134830 / OMIM Information: Dysfibrinogenemia, beta type (3); Afibrinogenemia, congenital,/202400 / (3); Thrombophilia, dysfibrinogenemic (3) // / Transcript Sequence (SEQ ID NO: 14): // Protein Sequence (SEQ ID NO: 98): // SNP Information //
Context (SEQ ID NO: 200):
TTCTTAAATTCAAATTTTTTAGCTGGAGTAAAAATGGTCCCAGTACCATTTCCTGTTCCCTTCACTATAACCTACATTTTTGT
CACATTAAGTTTTTCCC
Y
ATTCCAGGACAGGTCAGGCCCTTTAAAAAATTTCAACAGCTTTATTGAGATATAATTGATATAATTTAAAAAAATCCTGCACATG
TGTCATGCTGGAGCCCT
Celera SNP ID: hCV7429783 / Public SNP ID: rs1044291 / SNP Chromosome Position: 155493352 / SNP in Transcript Sequence SEQ ID NO: 14 / SNP Position Transcript: 3065 / Related Interrogated SNP: hCV15860433 / Related Interrogated

SNP: hCV11503469 / Related Interrogated SNP: hCV11503414 / SNP Source: Applera / Population(Allele,Count): Caucasian (C,27|T,7) African American (C,30|T,4) total (C,57|T,11) / SNP Type: UTR3 / SNP Source: dbSNP; HGBASE / Population(Allele,Count): Caucasian (C,151|T,75) / SNP Type: UTR3 //
Context (SEQ ID NO: 201):
AAAGGAAAAACTGATGTTTAAAAGTCCACTTTTAAAACTATATTTATTTATGTAGGATCTGTCAAAGAAAACTTCCAAAAAGA
TTTATTAATTAAACCAG
M
CTCTGTTGCAATAAGTTAATGTTTTCTTGTTTTGTAATCCACACATTCAATGAGTTAGGCTTTGCACTTGTAAGGAAGGAGAA
GCGTTCACAACCTCAAA
Celera SNP ID: hCV15971616 / Public SNP ID: rs2227421 / SNP Chromosome Position: 155492224 / SNP in Transcript Sequence SEQ ID NO: 14 / SNP Position Transcript: 1937 / Related Interrogated SNP: hCV11503469 / SNP Source: dbSNP; HGBASE / Population(Allele,Count): Caucasian (A,221|C,48) / SNP Type: UTR3 //

Gene Number: 13 / Gene Symbol FGG - 2266 / Gene Name: fibrinogen gamma chain / Public Transcript Accession: NM_021870 / Public Protein Accession: NP_068656 / Chromosome: 4 / OMIM NUMBER: 134850 / OMIM Information: Dysfibrinogenemia, gamma type (3); Hypofibrinogenemia, gamma/type (3); / Thrombophilia, dysfibrinogenemic (3) // / Transcript Sequence (SEQ ID NO: 15): // Protein Sequence (SEQ ID NO: 99): // SNP Information //
Context (SEQ ID NO: 202):
CCACCTGGGGGGAGCCAAACAGGTCAGACCAGAGCACCCTGCGGAAACAGAATATGACTCACTTTACCCTGAGGATGATTTGT
AGAAAATTAACTGCTAA
Y
TTCTATTGACCCACAAAGTTTCAGAAATTCTCTGAAAGTTTCTTCCTTTTTTTCTCTTACTATATTTATTGATTTCAAGTCTTC
TATTAAGGACATTTAGC
Celera SNP ID: hCV2892863 / Public SNP ID: rs1049636 / SNP Chromosome Position: 155525970 / SNP in Transcript Sequence SEQ ID NO: 15 / SNP Position Transcript: 1519 / Related Interrogated SNP: hCV11503469 / Related Interrogated SNP: hCV11503414 / Related Interrogated SNP: hCV2892877 / SNP Source: Applera / Population(Allele,Count): Caucasian (T,18|C,0) Tfrican Tmerican (T,9|C,5) total (T,27|C,5) / SNP Type: UTR3 / SNP Source: dbSNP; Celera; HapMap; HGBASE / Population(Allele,Count): Caucasian (C,67|T,159) / SNP Type: UTR3 //

Gene Number: 14 / Gene Symbol EIF6 - 3692 / Gene Name: eukaryotic translation initiation factor 6 / Public Transcript Accession: NM_002212 / Public Protein Accession: NP_002203 / Chromosome: 20 / OMIM NUMBER: 602912 / OMIM Information: // Transcript Sequence (SEQ ID NO: 16): // Protein Sequence (SEQ ID NO: 100): // SNP Information //
Context (SEQ ID NO: 203):
CTGTGCTTATCGCCTGGATCTATCATTACTGCAAAAACCTGCTCTGTTGTGCTGGCTGGCAGGCCCTGTGGCTGCTGGCTGAG
GGTTCTGCTGTCCTGTG
S
CACCCCATTAAAGTGCAGTTCCCTCCGGAAAAAAAAAAAAAAAAAAAAAAAAAAAAAAAA
Celera SNP ID: hCV11656916 / Public SNP ID: rs7004 / SNP Chromosome Position: 33866753 / SNP in Transcript Sequence SEQ ID NO: 16 / SNP Position Transcript: 1056 / Related Interrogated SNP: hCV1825046 / SNP Source: dbSNP; HGBASE / Population(Allele,Count): Gaucasian (C,78|G,28) / SNP Type: UTR3 //

Gene Number: 14 / Gene Symbol EIF6 - 3692 / Gene Name: eukaryotic translation initiation factor 6 / Public Transcript Accession: NM_181466 / Public Protein Accession: NP_852131 / Chromosome: 20 / OMIM NUMBER: 602912 / OMIM Information: // Transcript Sequence (SEQ ID NO: 17): // Protein Sequence (SEQ ID NO: 101): // SNP Information //
Context (SEQ ID NO: 204):
CTGTGCTTATCGCCTGGATCTATCATTACTGCAAAAACCTGCTCTGTTGTGCTGGCTGGCAGGCCCTGTGGCTGCTGGCTGAG
GGTTCTGCTGTCCTGTG
S
CACCCCATTAAAGTGCAGTTCCCTCCGGAAAAAAAAAAAAAAAAAAAAAAAAAAAAAAAA
Celera SNP ID: hCV11656916 / Public SNP ID: rs7004 / SNP Chromosome Position: 33866753 / SNP in Transcript Sequence SEQ ID NO: 17 / SNP Position Transcript: 999 / Related Interrogated SNP: hCV1825046 / SNP Source: dbSNP;

HGBASE / Population(Allele,Count): Gaucasian (C,78|G,28) / SNP Type: UTR3 //

Gene Number: 14 / Gene Symbol EIF6 - 3692 / Gene Name: eukaryotic translation initiation factor 6 / Public Transcript Accession: NM_181468 / Public Protein Accession: NP_852133 / Chromosome: 20 / OMIM NUMBER: 602912 / OMIM Information: // Transcript Sequence (SEQ ID NO: 18): // Protein Sequence (SEQ ID NO: 102): // SNP Information //
Context (SEQ ID NO: 205):
CTGTGCTTATCGCCTGGATCTATCATTACTGCAAAAACCTGCTCTGTTGTGCTGGCTGGCAGGCCCTGTGGCTGCTGGCTGAG
GGTTCTGCTGTCCTGTG
S
CACCCCATTAAAGTGCAGTTCCCTCCGGAAAAAAAAAAAAAAAAAAAAAAAAAAAAAAAAAAA
Celera SNP ID: hCV11656916 / Public SNP ID: rs7004 / SNP Chromosome Position: 33866753 / SNP in Transcript Sequence SEQ ID NO: 18 / SNP Position Transcript: 1201 / Related Interrogated SNP: hCV1825046 / SNP Source: dbSNP; HGBASE / Population(Allele,Count): Gaucasian (C,78|G,28) / SNP Type: UTR3 //

Gene Number: 15 / Gene Symbol KLKB1 - 3818 / Gene Name: kallikrein B, plasma (Fletcher factor) 1 / Public Transcript Accession: NM_000892 / Public Protein Accession: NP_000883 / Chromosome: 4 / OMIM NUMBER: 229000 / OMIM Information: Fletcher factor deficiency (1); Prekallikrein deficiency (3) // Transcript Sequence (SEQ ID NO: 19): // Protein Sequence (SEQ ID NO: 103): // SNP Information //
Context (SEQ ID NO: 206):
GCAATTTTTACAACCTGAGTTCAAGTCAAATTCTGAGCCTGGGGGGTCCTCATCTGCAAAGCATGGAGAGTGGCATCTTCTTT
GCATCCTAAGGACGAAA
R
ACACAGTGCACTCAGAGCTGCTGAGGACAATGTCTGGCTGAAGCCCGCTTTCAGCACGCCGTAACCAGGGGCTGACAATGCGA
GGTCGCAACTGAGATCT
Celera SNP ID: hCV15793897 / Public SNP ID: rs3087505 / SNP Chromosome Position: 187179486 / SNP in Transcript Sequence SEQ ID NO: 19 / SNP Position Transcript: 2108 / SNP Source: dbSNP; HapMap; HGBASE / Population(Allele,Count): Caucasian (A,20|G,206) / SNP Type: UTR3 //
Context (SEQ ID NO: 207):
TTCTTAAGATTATCTATGGATGGTTCTCCAACTAGGATTGCGTATGGGACACAAGGGAGCTCTGGTTACTCTTTGAGATTGTG
TAACACTGGGGACAACT
K
TGTCTGCACAACAAAAACAAGCACACGCATTGTTGGAGGAACAAACTCTTCTTGGGGAGAGTGGCCCTGGCAGGTGAGCCTGC
AGGTGAAGCTGACAGCT
Celera SNP ID: hCV3230004 / Public SNP ID: rs4253301 / SNP Chromosome Position: 187173012 / SNP in Transcript Sequence SEQ ID NO: 19 / SNP Position Transcript: 1213 / Related Interrogated SNP: hCV12066124 / Related Interrogated SNP: hCV25474413 / Related Interrogated SNP: hCV22272267 / Related Interrogated SNP: hCV27477533 / Related Interrogated SNP: hCV3230113 / Related Interrogated SNP: hCV8241630 / SNP Source: Applera / Population(Allele,Count): Caucasian (G,1|T,1) African American (G,0|T,30) total (G,1|T,31) / SNP Type: Missense Mutation / Protein Coding: SEQ ID NO: 103, at position 381,(S,TCT) (A,GCT) / SNP Source: Applera / Population(Allele,Count): Caucasian (G,1|T,9) African American (G,0|T,12) total (G,1|T,21) / SNP Type: Missense Mutation / Protein Coding: SEQ ID NO: 103, at position 381,(S,TCT) (A,GCT) / SNP Source: Applera / Population(Allele,Count): Caucasian (G,5|T,25) African American (G,1|T,27) total (G,6|T,52) / SNP Type: Missense Mutation / Protein Coding: SEQ ID NO: 103, at position 381,(S,TCT) (A,GCT) / SNP Source: dbSNP; Celera; HapMap; HGBASE / Population(Allele,Count): Caucasian (T,201|G,25) / SNP Type: Missense Mutation / Protein Coding: SEQ ID NO: 103, at position 381,(S,TCT) (A,GCT) //
Context (SEQ ID NO: 208):
AGAAAGGTGAAATCCAAAATATTCTACAAAAGGTAAATATTCCTTTGGTAACAAATGAAGAATGCCAGAAAAGATATCAAGAT
TATAAAATAACCCAACG
R
ATGGTCTGTGCTGGCTATAAAGAAGGGGGAAAAGATGCTTGTAAGGGAGATTCAGGTGGTCCCTTAGTTTGCAAACACAATGG
AATGTGGCGTTTGGTGG
Celera SNP ID: hCV3230016 / Public SNP ID: rs4253325 / SNP Chromosome Position: 187178473 / SNP in Transcript Sequence SEQ ID NO: 19 / SNP Position

Transcript: 1751 / Related Interrogated SNP: hCV27474895 / Related Interrogated SNP: hCV27477533 / Related Interrogated SNP: hCV8241630 / Related Interrogated SNP: hCV3230038 / Related Interrogated SNP: hCV22272267 / SNP Source: Applera / Population(Allele,Count): Caucasian (A,2|G,38) African American (A,3|G,31) total (A,5|G,69) / SNP Type: Missense Mutation / Protein Coding: SEQ ID NO: 103, at position 560,(R,CGG) (Q,CAG) / SNP Source: dbSNP; Celera; HapMap; ABI_Val; HGBASE / Population(Allele,Count): Caucasian (G,199|A,25) / SNP Type: Missense Mutation / Protein Coding: SEQ ID NO: 103, at position 560,(R,CGG) (Q,CAG) //
Context (SEQ ID NO: 209):
TTATAAAATAACCCAACGGATGGTCTGTGCTGGCTATAAAGAAGGGGGAAAAGATGCTTGTAAGGGAGATTCAGGTGGTCCCT TAGTTTGCAAACACAAT
Y
GAATGTGGCGTTTGGTGGGCATCACCAGCTGGGGTGAAGGCTGTGCCCGCAGGGAGCAACCTGGTGTCTACACCAAAGTCGCT GAGTACATGGACTGGAT
Celera SNP ID: hCV3230018 / Public SNP ID: rs925453 / SNP Chromosome Position: 187179210 / SNP in Transcript Sequence SEQ ID NO: 19 / SNP Position Transcript: 1833 / Related Interrogated SNP: hCV12066124 / Related Interrogated SNP: hCV15968043 / Related Interrogated SNP: hCV22272267 / Related Interrogated SNP: hCV3230113 / Related Interrogated SNP: hCV11786258 / Related Interrogated SNP: hCV25474413 / Related Interrogated SNP: hCV15793897 / Related Interrogated SNP: hCV27477533 / Related Interrogated SNP: hCV8241630 / Related Interrogated SNP: hCV3230096 / SNP Source: Applera / Population(Allele,Count): Caucasian (C,30|T,10) African American (C,22|T,14) total (C,52|T,24) // / SNP Type: Silent Mutation / Protein Coding: SEQ ID NO: 103, at position 587,(N,AAT) (N,AAC) / SNP Source: dbSNP; Celera; HapMap; ABI_Val; HGBASE / Population(Allele,Count): Caucasian (T,64|C,162) / SNP Type: Silent Mutation / Protein Coding: SEQ ID NO: 103, at position 587,(N,AAT) (N,AAC) //

Gene Number: 16 / Gene Symbol PIK3R1 - 5295 / Gene Name: phosphoinositide-3-kinase, regulatory subunit 1 (alpha) / Public Transcript Accession: NM_181504 / Public Protein Accession: NP_852556 / Chromosome: 5 / OMIM NUMBER: 171833 / OMIM Information: // Transcript Sequence (SEQ ID NO: 20): // Protein Sequence (SEQ ID NO: 104): // SNP Information //
Context (SEQ ID NO: 210):
GCCTTATTTTTGCATCTCACAAACATAAGTGCAATAGATCTTTTCATTGAACAGCAAAGTAGGATTCATCATTCCATATGACT TGAGTTACACCAGACCT
R
TTCTGCCCAATGCCTTTTTGATTACAGTGTAGCTTGCCCACCGCATTTGTCGTTTTAGATACTTTGCTAGCCGGCCACTTTGG ATTTCATCAGACAGTCC
Celera SNP ID: hCV27474984 / Public SNP ID: rs3756668 / SNP Chromosome Position: 67596088 / SNP in Transcript Sequence SEQ ID NO: 20 / SNP Position Transcript: 4086 / SNP Source: dbSNP; HapMap; ABI_Val; HGBASE / Population(Allele,Count): Caucasian (G,128|A,96) / SNP Type: UTR3 //

Gene Number: 16 / Gene Symbol PIK3R1 - 5295 / Gene Name: phosphoinositide-3-kinase, regulatory subunit 1 (alpha) / Public Transcript Accession: NM_181523 / Public Protein Accession: NP_852664 / Chromosome: 5 / OMIM NUMBER: 171833 / OMIM Information: // Transcript Sequence (SEQ ID NO: 21): // Protein Sequence (SEQ ID NO: 105): // SNP Information //
Context (SEQ ID NO: 211):
GCCTTATTTTTGCATCTCACAAACATAAGTGCAATAGATCTTTTCATTGAACAGCAAAGTAGGATTCATCATTCCATATGACT TGAGTTACACCAGACCT
R
TTCTGCCCAATGCCTTTTTGATTACAGTGTAGCTTGCCCACCGCATTTGTCGTTTTAGATACTTTGCTAGCCGGCCACTTTGG ATTTCATCAGACAGTCC
Celera SNP ID: hCV27474984 / Public SNP ID: rs3756668 / SNP Chromosome Position: 67596088 / SNP in Transcript Sequence SEQ ID NO: 21 / SNP Position Transcript: 4876 / SNP Source: dbSNP; HapMap; ABI_Val; HGBASE / Population(Allele,Count): Caucasian (G,128|A,96) / SNP Type: UTR3 //

Gene Number: 16 / Gene Symbol PIK3R1 - 5295 / Gene Name: phosphoinositide-3-kinase, regulatory subunit 1 (alpha) / Public Transcript

Accession: NM_181524 / Public Protein Accession: NP_852665 / Chromosome: 5 / OMIM NUMBER: 171833 / OMIM Information: // Transcript Sequence (SEQ ID NO: 22): // Protein Sequence (SEQ ID NO: 106): // SNP Information //
Context (SEQ ID NO: 212):
GCCTTATTTTTGCATCTCACAAACATAAGTGCAATAGATCTTTTCATTGAACAGCAAAGTAGGATTCATCATTCCATATGACT
TGAGTTACACCAGACCT
R
TTCTGCCCAATGCCTTTTTGATTACAGTGTAGCTTGCCCACCGCATTTGTCGTTTTAGATACTTTGCTAGCCGGCCACTTTGG
ATTTCATCAGACAGTCC
Celera SNP ID: hCV27474984 / Public SNP ID: rs3756668 / SNP Chromosome Position: 67596088 / SNP in Transcript Sequence SEQ ID NO: 22 / SNP Position Transcript: 4246 / SNP Source: dbSNP; HapMap; ABI_Val; HGBASE / Population(Allele,Count): Caucasian (G,128|A,96) / SNP Type: UTR3 //

Gene Number: 17 / Gene Symbol PLRG1 - 5356 / Gene Name: pleiotropic regulator 1 (PRL1 homolog, Arabidopsis) / Public Transcript Accession: NM_002669 / Public Protein Accession: NP_002660 / Chromosome: 4 / OMIM NUMBER: 605961 / OMIM Information: // Transcript Sequence (SEQ ID NO: 23): // Protein Sequence (SEQ ID NO: 107): // SNP Information //
Context (SEQ ID NO: 213):
ATCAGTTAGGGCTGTGGTTTTACATCCAAGACATTACACATTTGCATCTGGTTCTCCAGATAACATAAAGCAGTGGAAATTCC
CTGATGGAAGTTTCATT
W
AAAATCTTTCCGGTCATAATGCTATTATTAACACATTGACGGTAAATTCTGATGGAGTGCTTGTATCTGGAGCTGACAATGGC
ACCATGCATCTTTGGGA
Celera SNP ID: hCV25610762 / Public SNP ID: rs7668818 / SNP Chromosome Position: 155459194 / SNP in Transcript Sequence SEQ ID NO: 23 / SNP Position Transcript: 1282 / Related Interrogated SNP: hCV15860433 / Related Interrogated SNP: hCV11503414 / Related Interrogated SNP: hCV11503469 / SNP Source: Applera / Population(Allele,Count): Caucasian (T,30|A,8) Tfrican Tmerican (T,31|A,7) total (T,61|A,15) / SNP Type: Silent Rare Codon / Protein Coding: SEQ ID NO: 107, at position 406,(I,ATT) (I,ATA) / SNP Source: dbSNP; HapMap / Population(Allele,Count): Caucasian (T,84|A,14) / SNP Type: Silent Rare Codon / Protein Coding: SEQ ID NO: 107, at position 406,(I,ATT) (I,ATA) //

Gene Number: 18 / Gene Symbol PRLR - 5618 / Gene Name: prolactin receptor / Public Transcript Accession: NM_000949 / Public Protein Accession: NP_000940 / Chromosome: 5 / OMIM NUMBER: 176761 / OMIM Information: // Transcript Sequence (SEQ ID NO: 24): // Protein Sequence (SEQ ID NO: 108): // SNP Information //
Context (SEQ ID NO: 214):
GAAGGAGAGACACTCATGCATGAATGTCCAGACTACATAACCGGTGGCCCCAACTCCTGCCACTTTGGCAAGCAGTACACCTC
CATGTGGAGGACATACA
Y
CATGATGGTCAATGCCACTAACCAGATGGGAAGCAGTTTCTCGGATGAACTTTATGTGGACGTGACTTACATAGTTCAGCCAG
ACCCTCCTTTGGAGCTG
Celera SNP ID: hCV16171263 / Public SNP ID: rs78705921 / SNP Chromosome Position: 35084647 / SNP in Transcript Sequence SEQ ID NO: 24 / SNP Position Transcript: 829 / SNP Source: CDX; dbSNP / Population(Allele,Count): Caucasian (T,114|C,2) / SNP Type: Missense Mutation / Protein Coding: SEQ ID NO: 108, at position 100,(I,ATC) (V,GTC) //

Gene Number: 19 / Gene Symbol PROC - 5624 / Gene Name: protein C (inactivator of coagulation factors Va and VIIIa) / Public Transcript Accession: NM_000312 / Public Protein Accession: NP_000303 / Chromosome: 2 / OMIM NUMBER: 176860 / OMIM Information: Thrombophilia due to protein C deficiency (3); Purpura fulminans,/neon / atal (1) // / Transcript Sequence (SEQ ID NO: 25): // Protein Sequence (SEQ ID NO: 109): // SNP Information //
Context (SEQ ID NO: 215): ATGGATTAACTCGAACTCCAGGCTGTCATGGCGGCAGGACGGCG
W
ACTTGCAGTATCTCCACGACCCGCCCCTACAGGTGCCAGTGCCTCCAGAATGTGGCAGCTCACAAGCCTCCTGCTGTTCGTGG
CCACCTGGGGAATTTCC
Celera SNP ID: hCV1841975 / Public SNP ID: rs1799810 / SNP Chromosome Position: 128176040 / SNP in Transcript Sequence SEQ ID NO: 25 / SNP Position

Transcript: 45 / SNP Source: Applera / Population(Allele,Count): Caucasian (A,22|T,18) African American (A,9|T,25) total (A,31|T,43) / SNP Type: UTR5 / SNP Source: dbSNP; HapMap; HGBASE / Population(Allele,Count): Caucasian (A,73|T,39) / SNP Type: UTR5 //
Context (SEQ ID NO: 216):
CCCCTGGCAGGTGGTCCTGCTGGACTCAAAGAAGAAGCTGGCCTGCGGGGCAGTGCTCATCCACCCCTCCTGGGTGCTGACAG
CGGCCCACTGCATGGAT
Y
AGTCCAAGAAGCTCCTTGTCAGGCTTGGAGAGTATGACCTGCGGCGCTGGGAGAAGTGGGAGCTGGACCTGGACATCAAGGAG
GTCTTCGTCCACCCCAA
Celera SNP ID: hCV1841983 / Public SNP ID: rs5937 / SNP Chromosome Position: 128184770 / SNP in Transcript Sequence SEQ ID NO: 25 / SNP Position Transcript: 863 / SNP Source: CDX; dbSNP / Population(Allele,Count): Caucasian (T,154|C,72) / SNP Type: Silent Mutation / Protein Coding: SEQ ID NO: 109, at position 256, (D,GAT) (D,GAC) //

Gene Number: 20 / Gene Symbol SELE - 6401 / Gene Name: selectin E / Public Transcript Accession: NM_000450 / Public Protein Accession: NP_000441 / Chromosome: 1 / OMIM NUMBER: 131210 / OMIM Information: {Atherosclerosis, susceptibility to} (2) // Transcript Sequence (SEQ ID NO: 26): // Protein Sequence (SEQ ID NO: 110): // SNP Information //
Context (SEQ ID NO: 217):
GCCTTGAGGAGTGTGAGAATCAAAACTCTCCTACACTTCCATTAACTTAGCATGTGTTGAAAAAAAAGTTTCAGAGAAGTTCT
GGCTGAACACTGGCAAC
R
ACAAAGCCAACAGTCAAAACAGAGATGTGATAAGGATCAGAACAGCAGAGGTTCTTTTAAAGGGGCAGAAAAACTCTGGGAAA
TAAGAGAGAACAACTAC
Celera SNP ID: hCV2459446 / Public SNP ID: rs4786 / SNP Chromosome Position: 169692132 / SNP in Transcript Sequence SEQ ID NO: 26 / SNP Position Transcript: 3524 / Related Interrogated SNP: hCV11975250 / SNP Source: dbSNP; Celera; HapMap; HGBASE / Population(Allele,Count): Gaucasian (A,169|G,53) / SNP Type: UTR3 //
Context (SEQ ID NO: 218):
TGACCTGAGACAGAGGCAGCAGTGATACCCACCTGAGAGATCCTGTGTTTGAACAACTGCTTCCCAAAACGGAAAGTATTTCA
AGCCTAAACCTTTGGGT
K
AAAAGAACTCTTGAAGTCATGATTGCTTCACAGTTTCTCTCAGCTCTCACTTTGGTGCTTCTCATTAAAGAGAGTGGAGCCTG
GTCTTACAACACCTCCA
Celera SNP ID: hCV8919530 / Public SNP ID: rs1805193 / SNP Chromosome Position: 169702772 / SNP in Transcript Sequence SEQ ID NO: 26 / SNP Position Transcript: 139 / Related Interrogated SNP: hCV11975250 / SNP Source: dbSNP; HapMap; HGBASE / Population(Allele,Count): Gaucasian (G,199|T,27) / SNP Type: UTR5 //
Context (SEQ ID NO: 219):
GCAAGGCATGATGTTAACCAGAATAAAGTTCTGAGTGTTTTTACTACAGTTGTTTTTTTGAAAACATGGTAGAATTGGAGAGTA
AAAACTGAATGGAAGGT
Y
TGTATATTGTCAGATATTTTTTTCAGAAATATGTGGTTTCCACGATGAAAAACTTCCATGAGGCCAAACGTTTTGAACTAATAA
AAGCATAAATGCAAACA
Celera SNP ID: hCV11975322 / Public SNP ID: rs5357 / SNP Chromosome Position: 169692713 / SNP in Transcript Sequence SEQ ID NO: 26 / SNP Position Transcript: 2943 / Related Interrogated SNP: hCV11975250 / SNP Source: dbSNP; HapMap; HGBASE / Population(Allele,Count): Caucasian (T,109|C,11) / SNP Type: UTR3 //
Context (SEQ ID NO: 220):
TGTGAGAATCAAAACTCTCCTACACTTCCATTAACTTAGCATGTGTTGAAAAAAAAGTTTCAGAGAAGTTCTGGCTGAACACT
GGCAACAACAAAGCCAA
S
AGTCAAAACAGAGATGTGATAAGGATCAGAACAGCAGAGGTTCTTTTAAAGGGGCAGAAAAACTCTGGGAAATAAGAGAGAAC
AACTACTGTGATCAGGC
Celera SNP ID: hDV76908563 / Public SNP ID: rs3917441 / SNP Chromosome Position: 169692121 / SNP in Transcript Sequence SEQ ID NO: 26 / SNP Position Transcript: 3535 / Related Interrogated SNP: hCV11975250 / SNP Source: CDX;

dbSNP / Population(Allele,Count): Gaucasian (C,109|G,9) / SNP Type: UTR3 //

Gene Number: 21 / Gene Symbol SELL - 6402 / Gene Name: selectin L / Public Transcript Accession: NM_000655 / Public Protein Accession: NP_000646 / Chromosome: 1 / OMIM NUMBER: 153240 / OMIM Information: // Transcript Sequence (SEQ ID NO: 27): // Protein Sequence (SEQ ID NO: 111): // SNP Information // Context (SEQ ID NO: 221):
CAGCTTCCATTTCGCCCCTCATTTATCCCTCAACCCCCAGCCCACAGGTGTTTATACAGCTCAGCTTTTTGTCTTTTCTGAGG
AGAAACAAATAAGACCA
Y
AAAGGGAAAGGATTCATGTGGAATATAAAGATGGCTGACTTTGCTCTTTCTTGACTCTTGTTTTCAGTTTCAATTCAGTGCTG
TACTTGATGACAGACAC
Celera SNP ID: hCV8919501 / Public SNP ID: rs909628 / SNP Chromosome Position: 169660665 / SNP in Transcript Sequence SEQ ID NO: 27 / SNP Position Transcript: 1583 / Related Interrogated SNP: hCV11975250 / SNP Source: dbSNP; Celera; HapMap; ABI_Val; HGBASE / Population(Allele,Count): Caucasian (T,199|C,27) / SNP Type: UTR3 //

Gene Number: 22 / Gene Symbol SURF6 - 6838 / Gene Name: surfeit 6 / Public Transcript Accession: NM_006753 / Public Protein Accession: NP_006744 / Chromosome: 9 / OMIM NUMBER: 185642 / OMIM Information: // Transcript Sequence (SEQ ID NO: 28): // Protein Sequence (SEQ ID NO: 112): // SNP Information // Context (SEQ ID NO: 222):
GGCCAGGGCAGTGCCAAGGAGCTGTCCCCTGCCGCCTTGGAGAAAAGGCGGCGGAGAAAGCAGGAACGGGACCGGAAGAAGAG
GAAGCGAAAGGAGCTGC
Y
GGCGAAAGAGAAGGCCAGGAAGGCTGAGGAGGCCACGGAGGCCCAGGAGGTGGTGGAGGCAACCCCAGAGGGGGCCTGCACGG
AGCCGCGGGAGCCGCCC
Celera SNP ID: hCV8784837 / Public SNP ID: rs886090 / SNP Chromosome Position: 136199503 / SNP in Transcript Sequence SEQ ID NO: 28 / SNP Position Transcript: 566 / Related Interrogated SNP: hCV25610857 / SNP Source: Applera / Population(Allele,Count): Caucasian (T,16|C,24) Tfrican Tmerican (T,3|C,35) total (T,19|C,59) / SNP Type: Missense Mutation / Protein Coding: SEQ ID NO: 112, at position 163,(R,CGG) (W,TGG) / SNP Source: dbSNP; Celera; HapMap; ABI_Val; HGBASE / Population(Allele,Count): Caucasian (C,150|T,76) / SNP Type: Missense Mutation / Protein Coding: SEQ ID NO: 112, at position 163,(R,CGG) (W,TGG)//

Gene Number: 23 / Gene Symbol TSPAN8 - 7103 / Gene Name: tetraspanin 8 / Public Transcript Accession: NM_004616 / Public Protein Accession: NP_004607 / Chromosome: 12 / OMIM NUMBER: / OMIM Information: // Transcript Sequence (SEQ ID NO: 29): // Protein Sequence (SEQ ID NO: 113): // SNP Information // Context (SEQ ID NO: 223):
AAGCAATTTTTGGTTCTGAAGATGTAGGCTCTAGCTCCTACGTTGCTGTGGACATATTGATTGCTGTAGGTGCCATCATCATG
ATTCTGGGCTTCCTGGG
S
TGCTGCGGTGCTATAAAAGAAAGTCGCTGCATGCTTCTGTTGTTTTTCATAGGCTTGCTTCTGATCCTGCTCCTGCAGGTGGC
GACAGGTATCCTAGGAG
Celera SNP ID: hCV11686277 / Public SNP ID: rs3763978 / SNP Chromosome Position: 71533534 / SNP in Transcript Sequence SEQ ID NO: 29 / SNP Position Transcript: 398 / SNP Source: Applera / Population(Allele,Count): Gaucasian (G,20|C,16) African American (G,28|C,4) total (G,48|C,20) / SNP Type: Missense Mutation / Protein Coding: SEQ ID NO: 113, at position 73,(G,GGA) (A,GCA) / SNP Source: dbSNP; HapMap; ABI_Val; HGBASE / Population(Allele,Count): Gaucasian (G,71|C,49) / SNP Type: Missense Mutation / Protein Coding: SEQ ID NO: 113, at position 73,(G,GGA) (A,GCA) //

Gene Number: 24 / Gene Symbol BLZF1 - 8548 / Gene Name: basic leucine zipper nuclear factor 1 / Public Transcript Accession: NM_003666 / Public Protein Accession: NP_003657 / Chromosome: 1 / OMIM NUMBER: / OMIM Information: // Transcript Sequence (SEQ ID NO: 30): // Protein Sequence (SEQ ID NO: 114): // SNP Information //
Context (SEQ ID NO: 224):

TTTTATTTTAAAGTAGAAATCTGTCCGCTTTTCACTTACGGAGGCCTTACAGTGTGTGTTCTGTGTTACTTGAGTTTATACGC
ACTGCAGAATTTGTTTA
  Y
CCTGCTCTTTTGGAAACGTATTCCGGAAGCGAAACCCTGAGTAATCGGAAGTGGTTAGGAGTGAGAGAGCTGCTGGATATGCG
GAGGGACTGGGCGGGTC
  Celera SNP ID:      hCV15878582 / Public SNP ID:      rs2275299 / SNP Chromosome
Position:  169337376 / SNP in Transcript Sequence      SEQ ID NO: 30 / SNP Position
Transcript:   183 / Related Interrogated SNP:    hCV11975250 / SNP Source:    Applera
/ Population(Allele,Count):    Caucasian (C,11|T,29) African American (C,22|T,14)
total (C,33|T,43) //  / SNP Type:    UTR5 / SNP Source:    dbSNP; Celera;  HapMap;
HGBASE / Population(Allele,Count):    Caucasian (C,94|T,132) / SNP Type:    UTR5 //

Gene Number:  25 / Gene Symbol      NR1I2 - 8856 / Gene Name:   nuclear receptor
subfamily 1, group I, member 2 / Public Transcript Accession:  NM_003889 / Public
Protein Accession:   NP_003880 / Chromosome:   3 / OMIM NUMBER:  603065 / OMIM
Information: // Transcript Sequence (SEQ ID NO: 31): // Protein Sequence      (SEQ
ID NO: 115): // SNP Information //
Context          (SEQ ID NO: 225):
AAGCACTGCCTTTACTTCAGTGGGAATCTCGGCCTCAGCCTGCAAGCCAAGTGTTCACAGTGAGAAAAGCAAGAGAATAAGCT
AATACTCCTGTCCTGAA
  M
AAGGCAGCGGCTCCTTGGTAAAGCTACTCCTTGATCGATCCTTTGCACCGGATTGTTCAAAGTGGACCCCAGGGGAGAAGTCG
GAGCAAAGAACTTACCA
  Celera SNP ID:      hCV263841 / Public SNP ID:      rs1523127 / SNP Chromosome
Position:  119501039 / SNP in Transcript Sequence      SEQ ID NO: 31 / SNP Position
Transcript:  1709 / SNP Source:    dbSNP; Celera; HapMap; HGBASE /
Population(Allele,Count):    Caucasian (C,76|A,150) / SNP Type:    UTR5 //
Context         (SEQ ID NO: 226):
GCAGGCCCCAGATATAGCCCCATGCTGTCCTCCTACCCCAGAGCACACTGTTCAGGCTACTTCCACTGGTACTGAAATCCAGT
ATTTCACTTACTCTTTT
  Y
CTTTCCAATATCCTCATGACATTCAATATTTCACTTACTCTAGGTCCTCCCTGCCTAAGGCCCAAGTCAACTTTCTGTCCAGT
GGGATTTGTAATCCAAT
Celera SNP ID:      hCV9152783 / Public SNP ID:      rs1523130 / SNP Chromosome
Position:  119499507 / SNP in Transcript Sequence      SEQ ID NO: 31 / SNP Position
Transcript:   177 / Related Interrogated SNP:    hCV263841 / SNP Source:    dbSNP;
Celera; HapMap; HGBASE / Population(Allele,Count):    Caucasian (T,79|C,147) / SNP
Type:   UTR5 //
Context         (SEQ ID NO: 227):
GTACTTCAAAATAATAACAACTTAAGTCAATAAATAAATGTAAGGAAGTCCAAATGTTCACCTGAAGACAACTGTGGTCATTT
TTTGGCAATCCCAGGTT
  Y
TCTTTTCTACCTGTTTGCTCAATCGTGGTCTCCCTCTCCCTCTCTTGTTGGGGCCCATGCCCCTGCTTTACTGTTGCCAGAGG
CTTGTACTTGTTTGCCT
Celera SNP ID:      hCV27504984 / Public SNP ID:      rs3814055 / SNP Chromosome
Position:  119500035 / SNP in Transcript Sequence      SEQ ID NO: 31 / SNP Position
Transcript:   705 / Related Interrogated SNP:    hCV263841 / SNP Source:    dbSNP;
HapMap; ABI_Val; HGBASE / Population(Allele,Count):    Caucasian (C,150|T,76) / SNP
Type:   UTR5 //

Gene Number:  25 / Gene Symbol      NR1I2 - 8856 / Gene Name:   nuclear receptor
subfamily 1, group I, member 2 / Public Transcript Accession:  NM_033013 / Public
Protein Accession:   NP_148934 / Chromosome:   3 / OMIM NUMBER:  603065 / OMIM
Information: // Transcript Sequence (SEQ ID NO: 32): // Protein Sequence      (SEQ
ID NO: 116): // SNP Information //
Context          (SEQ ID NO: 228):
AAGCACTGCCTTTACTTCAGTGGGAATCTCGGCCTCAGCCTGCAAGCCAAGTGTTCACAGTGAGAAAAGCAAGAGAATAAGCT
AATACTCCTGTCCTGAA
  M
AAGGCAGCGGCTCCTTGGTAAAGCTACTCCTTGATCGATCCTTTGCACCGGATTGTTCAAAGTGGACCCCAGGGGAGAAGTCG
GAGCAAAGAACTTACCA
  Celera SNP ID:      hCV263841 / Public SNP ID:      rs1523127 / SNP Chromosome
Position:  119501039 / SNP in Transcript Sequence      SEQ ID NO: 32 / SNP Position

Transcript: 1709 / SNP Source: dbSNP; Celera; HapMap; HGBASE /
Population(Allele,Count): Caucasian (C,76|A,150) / SNP Type: UTR5 //
Context (SEQ ID NO: 229):
GCAGGCCCCAGATATAGCCCCATGCTGTCCTCCTACCCCAGAGCACACTGTTCAGGCTACTTCCACTGGTACTGAAATCCAGT
ATTTCACTTACTCTTTT
Y
CTTTCCAATATCCTCATGACATTCAATATTTCACTTACTCTAGGTCCTCCCTGCCTAAGGCCCAAGTCAACTTTCTGTCCAGT
GGGATTTGTAATCCAAT
Celera SNP ID: hCV9152783 / Public SNP ID: rs1523130 / SNP Chromosome
Position: 119499507 / SNP in Transcript Sequence SEQ ID NO: 32 / SNP Position
Transcript: 177 / Related Interrogated SNP: hCV263841 / SNP Source: dbSNP;
Celera; HapMap; HGBASE / Population(Allele,Count): Caucasian (T,79|C,147) / SNP
Type: UTR5 //
Context (SEQ ID NO: 230):
GTACTTCAAAATAATAACAACTTAAGTCAATAAATAAATGTAAGGAAGTCCAAATGTTCACCTGAAGACAACTGTGGTCATTT
TTTGGCAATCCCAGGTT
Y
TCTTTTCTACCTGTTTGCTCAATCGTGGTCTCCCTCTCCCTCTCTTGTTGGGGCCCATGCCCCTGCTTTACTGTTGCCAGAGG
CTTGTACTTGTTTGCCT
Celera SNP ID: hCV27504984 / Public SNP ID: rs3814055 / SNP Chromosome
Position: 119500035 / SNP in Transcript Sequence SEQ ID NO: 32 / SNP Position
Transcript: 705 / Related Interrogated SNP: hCV263841 / SNP Source: dbSNP;
HapMap; ABI_val; HGBASE / Population(Allele,Count): Caucasian (C,150|T,76) / SNP
Type: UTR5 //

Gene Number: 26 / Gene Symbol LRAT - 9227 / Gene Name: lecithin retinol
acyltransferase (phosphatidylcholine--retinol O-acylt / ransferase) // Public
Transcript Accession: NM_004744 / Public Protein Accession: NP_004735 /
Chromosome: 4 / OMIM NUMBER: 604863 / OMIM Information: Retinal dystrophy,
early-onset severe (3) // Transcript Sequence (SEQ ID NO: 33): // Protein Sequence
(SEQ ID NO: 117): // SNP Information //
Context (SEQ ID NO: 231):
TGGTAAAACAAGCATTCAAGCACTTTTACAAAATTACCAAATTCTTAAAATGAAGCCACAGCTAGACTTGCATTTCAGGTATT
AAAATTGCTTTCTTAAC
K
GTCAAGAATCACAAAATAACAAATCATATTATGAGTGAATATGGGGAGGGCGGGGCCAATCAGTCAATGATAATCTGAACAAA
TTTTAAGAGCAGATTTT
Celera SNP ID: hCV31863937 / Public SNP ID: rs12507608 / SNP Chromosome
Position: 155671626 / SNP in Transcript Sequence SEQ ID NO: 33 / SNP Position
Transcript: 2244 / Related Interrogated SNP: hCV11503469 / SNP Source: dbSNP;
HapMap / Population(Allele,Count): Caucasian (T,202|G,24) / SNP Type: UTR3 //
Context (SEQ ID NO: 232):
CCTGGGCAACAAAGTGAGACTCCATCTCAAAAAAAAAAAAAAAAAAAAAAAAAAAAAAAAAGAAGAAGAAACCCTGAGCCATTAA
TGTGTCTCGTAACTTGA
K
GAAGTTCATGAATTCTCTGGGCTTCATCCATTAAGATTCATCCTTATGCTCCTGCAAGTTTCTTATATTTATGTATTTCTATT
CTGATTTCTGAAATGAT
Celera SNP ID: hDV70817844 / Public SNP ID: rs17032000 / SNP Chromosome
Position: 155672958 / SNP in Transcript Sequence SEQ ID NO: 33 / SNP Position
Transcript: 3576 / Related Interrogated SNP: hCV11503469 / SNP Source: dbSNP;
HapMap / Population(Allele,Count): Caucasian (G,203|T,23) / SNP Type: UTR3 //

Gene Number: 27 / Gene Symbol XYLB - 9942 / Gene Name: xylulokinase homolog
(H. influenzae) / Public Transcript Accession: NM_005108 / Public Protein
Accession: NP_005099 / Chromosome: 3 / OMIM NUMBER: 604049 / OMIM Information:
// Transcript Sequence (SEQ ID NO: 34): // Protein Sequence (SEQ ID NO: 118):
// SNP Information //
Context (SEQ ID NO: 233):
GTGAGTGGTGTCCTTTGGAGGCATGGTGGATGAAAGGCGCAAAGGAAGCAAGAGGTTAAAGATAATGGCCCATTAGATACAAC
AGCAAGCCCAAGATTCC
R
AAGAAGACAGACAACTCCCCCTAGTACCACCTAGACATTACTTTTTAAAGACATTTTACATTATTGTCCAGCAGAAAAAGGCT
GTCTTGGACCATGAAAC

Celera SNP ID: hCV1845321 / Public SNP ID: rs12636358 / SNP Chromosome Position: 38455901 / SNP in Transcript Sequence SEQ ID NO: 34 / SNP Position Transcript: 3117 / Related Interrogated SNP: hCV15949414 / SNP Source: dbSNP; Celera; HapMap / Population(Allele,Count): Caucasian (G,216|A,10) / SNP Type: UTR3 //

Gene Number: 28 / Gene Symbol AKT3 - 10000 / Gene Name: v-akt murine thymoma viral oncogene homolog 3 (protein kinase B, gamma / ) // Public Transcript Accession: NM_005465 / Public Protein Accession: NP_005456 / Chromosome: 1 / OMIM NUMBER: / OMIM Information: // Transcript Sequence (SEQ ID NO: 35): // Protein Sequence (SEQ ID NO: 119): // SNP Information // Context (SEQ ID NO: 234): TTGATTAGGAAGCATTTGGTAGAAGGACTGAACAACTGTTGGGATATATATATATATATATAATTTTTTTTTTTTTAAATTCCT GGTGGATACTGTAGAAG

M

AGCCCATATCACATGTGGATGTCGAGACTTCACGGGCAATCATGAGCAAGTGAACACTGTTCTACCAAGAACTGAAGGCATAT GCACAGTCAAGGTCACT

Celera SNP ID: hCV30382231 / Public SNP ID: rs9428966 / SNP Chromosome Position: 243667900 / SNP in Transcript Sequence SEQ ID NO: 35 / SNP Position Transcript: 2172 / Related Interrogated SNP: hCV233148 / Related Interrogated SNP: hCV30690780 / Related Interrogated SNP: hCV31523650 / SNP Source: dbSNP; HapMap / Population(Allele,Count): Caucasian (A,164|C,60) / SNP Type: UTR3 //

Gene Number: 29 / Gene Symbol PROCR - 10544 / Gene Name: protein C receptor, endothelial / Public Transcript Accession: NM_006404 / Public Protein Accession: NP_006395 / Chromosome: 20 / OMIM NUMBER: 600646 / OMIM Information: // Transcript Sequence (SEQ ID NO: 36): // Protein Sequence (SEQ ID NO: 120): // SNP Information // Context (SEQ ID NO: 235): TGTGTGCAGTATGTGCAGAAACATATTTCCGCGGAAAACACGAAAGGGAGCCAAACAAGCCGCTCCTACACTTCGCTGGTCCT GGGCGTCCTGGTGGGCA

R

TTTCATCATTGCTGGTGTGGCTGTAGGCATCTTCCTGTGCACAGGTGGACGGCGATGTTAATTACTCTCCAGCCCCCTCAGAA GGGGCTGGATTGATGGA

Celera SNP ID: hCV25620145 / Public SNP ID: rs867186 / SNP Chromosome Position: 33764554 / SNP in Transcript Sequence SEQ ID NO: 36 / SNP Position Transcript: 840 / SNP Source: Applera / Population(Allele,Count): Caucasian (A,38|G,2) African American (A,32|G,6) total (A,70|G,8) / SNP Type: Missense Mutation / Protein Coding: SEQ ID NO: 120, at position 219,(S,AGT) (G,GGT) / SNP Source: dbSNP; Celera; HapMap; HGBASE / Population(Allele,Count): Caucasian (A,204|G,22) / SNP Type: Missense Mutation / Protein Coding: SEQ ID NO: 120, at position 219,(S,AGT) (G,GGT) // Context (SEQ ID NO: 236): GGTCCTGGGCGTCCTGGTGGGCAGTTTCATCATTGCTGGTGTGGCTGTAGGCATCTTCCTGTGCACAGGTGGACGGCGATGTT AATTACTCTCCAGCCCC

S

TCAGAAGGGGCTGGATTGATGGAGGCTGGCAAGGGAAAGTTTCAGCTCACTGTGAAGCCAGACTCCCCAACTGAAACACCAGA AGGTTTGGAGTGACAGC

Celera SNP ID: hCV1825047 / Public SNP ID: rs9574 / SNP Chromosome Position: 33764632 / SNP in Transcript Sequence SEQ ID NO: 36 / SNP Position Transcript: 917 / Related Interrogated SNP: hCV1825046 / SNP Source: dbSNP; Celera; HGBASE / Population(Allele,Count): Caucasian (C,53|G,63) / SNP Type: UTR3 //

Gene Number: 30 / Gene Symbol MAP3K2 - 10746 / Gene Name: mitogen-activated protein kinase kinase kinase 2 / Public Transcript Accession: NM_006609 / Public Protein Accession: NP_006600 / Chromosome: 2 / OMIM NUMBER: / OMIM Information: // Transcript Sequence (SEQ ID NO: 37): // Protein Sequence (SEQ ID NO: 121): // SNP Information // Context (SEQ ID NO: 237): TGAAACAGTGGTTGCCAAAAGTGGAGCGAGGATGATTTCACTAGGCATTTGGCAATTCTTAGAGACATTTCCGGTTGTCACAA TTGGAGGGATACTAGTA

R

CATGAATTGGGTAGAGGCCAGGGATGTTGCTAAATAGATTATAATACACAAGGAAAGCAGCCTCAAAGAATTACCCCTCCCAA
AATGTCAGAAGTGCTGA
Celera SNP ID: hCV12046224 / Public SNP ID: rs10850 / SNP Chromosome
Position: 128063945 / SNP in Transcript Sequence SEQ ID NO: 37 / SNP Position
Transcript: 3135 / Related Interrogated SNP: hCV1841975 / Related Interrogated
SNP: hCV1841983 / SNP Source: dbSNP; HapMap / Population(Allele,Count):
Gaucasian (A,61|G,165) / SNP Type: UTR3 //

Gene Number: 31 / Gene Symbol MMP24 - 10893 / Gene Name: matrix
metallopeptidase 24 (membrane-inserted) / Public Transcript Accession: NM_006690 /
Public Protein Accession: NP_006681 / Chromosome: 20 / OMIM NUMBER: / OMIM
Information: // Transcript Sequence (SEQ ID NO: 38): // Protein Sequence (SEQ
ID NO: 122): // SNP Information //
Context (SEQ ID NO: 238):
TGGCCTCCCTTCCCCCACTTCCTGGTCCCTGTCCACTCCTCAGGTTGGTGCTCTCACTTCTTGAAAGCTCTAGGCACCCCCGC
CTCCCGCCAGGCTCCCC
R
TTGGCTCCTGGCAGGCCAGCTGAGAATGAACAGGAGATGGAGGCAGGCAGCCCAGGCTGCAGAGGTGAGGGATGTGGGGCCAG
GCCCAGAGGGCTCAGCC / Celera SNP ID: hCV1271624 / Public SNP ID: rs7280 / SNP
Chromosome Position: 33864484 / SNP in Transcript Sequence SEQ ID NO: 38 / SNP
Position Transcript: 4013 / SNP Source: dbSNP; Celera; HGBASE /
Population(Allele,Count): Caucasian (A,139|G,85) / SNP Type: UTR3 //
Context (SEQ ID NO: 239):
ACTCTGAGGCCGACTCAATCTCTCGGCTGGAAGCAGTGTTTTCCCAGAGCTTGGCCCTTGCTGACCTCGCTCACTGGGCCCAT
CTTCCCACACTGCTCTT
R
GAAGGACACCCCTACCGGTAGCAGCCCCAAGCTGAGGGGGCTCCCTTTTTGACCTTCACTGGCCCGCCCTTCACTGTCTCCAG
CAGGAGTTCCTAGGGCT
Celera SNP ID: hCV1271625 / Public SNP ID: rs6060341 / SNP Chromosome
Position: 33863633 / SNP in Transcript Sequence SEQ ID NO: 38 / SNP Position
Transcript: 3162 / Related Interrogated SNP: hCV1825046 / SNP Source: dbSNP;
Celera; HapMap; ABI_Val / Population(Allele,Count): Caucasian (A,176|G,50) / SNP
Type: UTR3 //

Gene Number: 32 / Gene Symbol ADAMTS13 - 11093 / Gene Name: ADAM
metallopeptidase with thrombospondin type 1 motif, 13 / Public Transcript
Accession: NM_139025 / Public Protein Accession: NP_620594 / Chromosome: 9 /
OMIM NUMBER: 604134 / OMIM Information: Thrombotic thrombocytopenic purpura,
familial, 274150 (3) // Transcript Sequence (SEQ ID NO: 39): // Protein Sequence
(SEQ ID NO: 123): // SNP Information //

Context (SEQ ID NO: 240):
AGACCTGCCTTTGGGGGGCGTGCATGTGTTGGTGCTGACCTCCAGGCCGAGATGTGCAACACTCAGGCCTGCGAGAAGACCCA
GCTGGAGTTCATGTCGC
S
ACAGTGCGCCAGGACCGACGGCCAGCCGCTGCGCTCCTCCCCTGGCGGCGCCTCCTTCTACCACTGGGGTGCTGCTGTACCAC
ACAGCCAAGGGGATGCT
Celera SNP ID: hCV11571465 / Public SNP ID: rs2301612 / SNP Chromosome
Position: 136301982 / SNP in Transcript Sequence SEQ ID NO: 39 / SNP Position
Transcript: 1787 / Related Interrogated SNP: hCV25610857 / SNP Source: CDX;
dbSNP / Population(Allele,Count): Caucasian (C,74|G,46) / SNP Type: Missense
Mutation / Protein Coding: SEQ ID NO: 123, at position 448,(Q,CAA) (E,GAA) //

Gene Number: 32 / Gene Symbol ADAMTS13 - 11093 / Gene Name: ADAM
metallopeptidase with thrombospondin type 1 motif, 13 / Public Transcript
Accession: NM_139026 / Public Protein Accession: NP_620595 / Chromosome: 9 /
OMIM NUMBER: 604134 / OMIM Information: Thrombotic thrombocytopenic purpura,
familial, 274150 (3) // Transcript Sequence (SEQ ID NO: 40): // Protein Sequence
(SEQ ID NO: 124): // SNP Information //
Context (SEQ ID NO: 241):
AGACCTGCCTTTGGGGGGCGTGCATGTGTTGGTGCTGACCTCCAGGCCGAGATGTGCAACACTCAGGCCTGCGAGAAGACCCA
GCTGGAGTTCATGTCGC
S

ACAGTGCGCCAGGACCGACGGCCAGCCGCTGCGCTCCTCCCCTGGCGGCGCCTCCTTCTACCACTGGGGTGCTGCTGTACCAC
ACAGCCAAGGGGATGCT
Celera SNP ID:      hCV11571465 / Public SNP ID:       rs2301612 / SNP Chromosome
Position:  136301982 / SNP in Transcript Sequence      SEQ ID NO: 40 / SNP Position
Transcript:  1694 / Related Interrogated SNP:   hCV25610857 / SNP Source:   CDX;
dbSNP / Population(Allele,Count):   Caucasian (C,74|G,46) / SNP Type:   Missense
Mutation / Protein Coding:    SEQ ID NO: 124, at position 417,(Q,CAA) (E,GAA) //

Gene Number:  32 / Gene Symbol      ADAMTS13 - 11093 / Gene Name:  ADAM
metallopeptidase with thrombospondin type 1 motif, 13 / Public Transcript
Accession:  NM_139027 / Public Protein Accession:   NP_620596 / Chromosome:   9 /
OMIM NUMBER:   604134 / OMIM Information:  Thrombotic thrombocytopenic purpura,
familial, 274150 (3) // Transcript Sequence  (SEQ ID NO: 41): // Protein Sequence
  (SEQ ID NO: 125): // SNP Information //
Context           (SEQ ID NO: 242):
AGACCTGCCTTTGGGGGGCGTGCATGTGTTGGTGCTGACCTCCAGGCCGAGATGTGCAACACTCAGGCCTGCGAGAAGACCCA
GCTGGAGTTCATGTCGC
S
ACAGTGCGCCAGGACCGACGGCCAGCCGCTGCGCTCCTCCCCTGGCGGCGCCTCCTTCTACCACTGGGGTGCTGCTGTACCAC
ACAGCCAAGGGGATGCT
Celera SNP ID:      hCV11571465 / Public SNP ID:       rs2301612 / SNP Chromosome
Position:  136301982 / SNP in Transcript Sequence      SEQ ID NO: 41 / SNP Position
Transcript:  1787 / Related Interrogated SNP:   hCV25610857 / SNP Source:   CDX;
dbSNP / Population(Allele,Count):   Caucasian (C,74|G,46) / SNP Type:   Missense
Mutation / Protein Coding:    SEQ ID NO: 125, at position 448,(Q,CAA) (E,GAA) //

Gene Number:  33 / Gene Symbol      PTPN21 - 11099 / Gene Name:  protein tyrosine
phosphatase, non-receptor type 21 / Public Transcript Accession:  NM_007039 /
Public Protein Accession:   NP_008970 / Chromosome:   14 / OMIM NUMBER: / OMIM
Information: // Transcript Sequence  (SEQ ID NO: 42): // Protein Sequence      (SEQ
ID NO: 126): // SNP Information //
Context           (SEQ ID NO: 243):
CTTTACAAAAATGCAGTATTATCTATACTCAGACTGAGGTGCTGGGATGGCGAGCAGGGGACCCCACAGCACTGTCCCCAGGG
AATTCAAGTCCTGTGAC
Y
GCCAGTGACTTACTCCGTTTCCTTGGGGCTTTTCCCACCTCAACCTGTATTGTTTCTTTTTCATAAATAAATTCAGCATTATA
GTATCATTAAAAGAAGC
Celera SNP ID:      hCV27520559 / Public SNP ID:       rs3814855 / SNP Chromosome
Position:  88933360 / SNP in Transcript Sequence      SEQ ID NO: 42 / SNP Position
Transcript:  4977 / Related Interrogated SNP:   hCV16182835 / SNP Source:   dbSNP;
HapMap; HGBASE / Population(Allele,Count):   Caucasian (C,164|T,58) / SNP Type:
UTR3 //

Gene Number:  34 / Gene Symbol      KIFAP3 - 22920 / Gene Name:   kinesin-associated
protein 3 / Public Transcript Accession:  NM_014970 / Public Protein Accession:
NP_055785 / Chromosome:   1 / OMIM NUMBER:   601836 / OMIM Information: //
Transcript Sequence  (SEQ ID NO: 43): // Protein Sequence      (SEQ ID NO: 127): //
SNP Information //
Context           (SEQ ID NO: 244):
ATTAACCCCAGCCCCTCTGTCTTCTGTTAAGTACAGTTGATACTGACATTGTTCACTCATCAAACCACATCTTGATGCTAAGT
AACATTTCCCATGAGCC
W
CAAAACTGAATGCTGAAAAGCTACTAGACTGGAAAACAAACACTGCATTATGTATGTTAAGTGACTAATTTAATTTCAATTAA
AAAGCGTAAAGTGAAAA
Celera SNP ID:      hCV8697995 / Public SNP ID:       rs4519 / SNP Chromosome
Position:  169890574 / SNP in Transcript Sequence      SEQ ID NO: 43 / SNP Position
Transcript:  2893 / Related Interrogated SNP:   hCV11975250 / SNP Source:   dbSNP;
HapMap; ABI_Val; HGBASE / Population(Allele,Count):   Caucasian (A,28|T,198) / SNP
Type:   UTR3 //

Gene Number:  35 / Gene Symbol   ATF6 - 22926 / Gene Name:  activating
transcription factor 6 / Public Transcript Accession:  NM_007348 / Public Protein
Accession:   NP_031374 / Chromosome:   1 / OMIM NUMBER:   605537 / OMIM Information:

// Transcript Sequence (SEQ ID NO: 44): // Protein Sequence (SEQ ID NO: 128):
// SNP Information //
Context (SEQ ID NO: 245):
TCTTCTAGTTCTCAGATGTCTCCCCTTTCCTTATATGGTGAAAACTCTAATAGTCTCTCTTCAGCGGAGCCACTGAAGGAAGA
TAAGCCTGTCACTGGTC
Y
TAGGAACAAGACTGAAAATGGACTGACTCCAAAGAAAAAAATTCAGGTGAATTCAAAACCTTCAATTCAGCCCAAGCCTTTAT
TGCTTCCAGCAGCACCC
Celera SNP ID: hCV25631989 / Public SNP ID: rs1135983 / SNP Chromosome
Position: 161761312 / SNP in Transcript Sequence SEQ ID NO: 44 / SNP Position
Transcript: 537 / SNP Source: Applera / Population(Allele,Count): Caucasian
(C,35|T,5) African American (C,28|T,4) total (C,63|T,9) / SNP Type: Missense
Mutation / Protein Coding: SEQ ID NO: 128, at position 157,(P,CCT) (S,TCT) / SNP
Source: dbSNP; Applera / Population(Allele,Count): Caucasian (C,111|T,5) / SNP
Type: Missense Mutation / Protein Coding: SEQ ID NO: 128, at position
157,(P,CCT) (S,TCT) //

Gene Number: 36 / Gene Symbol TRPC4AP - 26133 / Gene Name: transient receptor
potential cation channel, subfamily C, member 4 ass / ociated protein // Public
Transcript Accession: NM_015638 / Public Protein Accession: NP_056453 /
Chromosome: 20 / OMIM NUMBER: 608430 / OMIM Information: // Transcript Sequence
(SEQ ID NO: 45): // Protein Sequence (SEQ ID NO: 129): // SNP Information //
Context (SEQ ID NO: 246):
CATCCTCAAGGAAATTTCTCCTCTTCTCTCCATGGAGGCTATGGCATTTGTTACTGAAGAGAGGAAACTTACCCAAGAAACCA
CTTATCCAAATACTTAC
Y
TTTTTGACTTGTTTGGAGGTGTTGATCTTCTTGTAGAAATTCTTATGAGGCCTACGATCTCTATCCGGGGACAGAAACTGAAA
ATAAGTGATGAAATGTC
Celera SNP ID: hCV11656983 / Public SNP ID: rs1998233 / SNP Chromosome
Position: 33657126 / SNP in Transcript Sequence SEQ ID NO: 45 / SNP Position
Transcript: 422 / Related Interrogated SNP: hCV1825046 / SNP Source: Applera /
Population(Allele,Count): Caucasian (T,28|C,8) Tfrican Tmerican (T,35|C,3)
total (T,63|C,11) / SNP Type: Silent Mutation / Protein Coding: SEQ ID NO:
129, at position 129,(Y,TAC) (Y,TAT) / SNP Source: dbSNP; HapMap; ABI_Val;
HGBASE / Population(Allele,Count): Caucasian (C,62|T,164) / SNP Type: Silent
Mutation / Protein Coding: SEQ ID NO: 129, at position 129,(Y,TAC) (Y,TAT) //
Context (SEQ ID NO: 247):
CTGGGCAGGGGTGGTGGGTACCAGAGAATGCTGCCTTCCCCCAAGCCTGCCCCTCTGCCTCATTTTCCTGTAGCTCCTCTGGT
TCTGTTTGCTCATTGGC
Y
GCTGTGTTCATCCAAGGGGGTTCTCCCAGAAGTGAGGGGCCTTTCCCTCCATCCCTTGGGGCACGGGGCAGCTGTGCCTGCCC
TGCCTCTGCCTGAGGCA
Celera SNP ID: hCV1347919 / Public SNP ID: rs1058003 / SNP Chromosome
Position: 33590417 / SNP in Transcript Sequence SEQ ID NO: 45 / SNP Position
Transcript: 2960 / Related Interrogated SNP: hCV1825046 / SNP Source: dbSNP;
Celera; HapMap; ABI_Val; HGBASE / Population(Allele,Count): Caucasian
(C,83|T,143) / SNP Type: UTR3 //

Gene Number: 36 / Gene Symbol TRPC4AP - 26133 / Gene Name: transient
receptor potential cation channel, subfamily C, member 4 ass / ociated protein //
Public Transcript Accession: NM_199368 / Public Protein Accession: NP_955400 /
Chromosome: 20 / OMIM NUMBER: 608430 / OMIM Information: // Transcript Sequence
(SEQ ID NO: 46): // Protein Sequence (SEQ ID NO: 130): // SNP Information //
Context (SEQ ID NO: 248):
CATCCTCAAGGAAATTTCTCCTCTTCTCTCCATGGAGGCTATGGCATTTGTTACTGAAGAGAGGAAACTTACCCAAGAAACCA
CTTATCCAAATACTTAC
Y
TTTTTGACTTGTTTGGAGGTGTTGATCTTCTTGTAGAAATTCTTATGAGGCCTACGATCTCTATCCGGGGACAGAAACTGAAA
ATAAGTGATGAAATGTC
Celera SNP ID: hCV11656983 / Public SNP ID: rs1998233 / SNP Chromosome
Position: 33657126 / SNP in Transcript Sequence SEQ ID NO: 46 / SNP Position
Transcript: 422 / Related Interrogated SNP: hCV1825046 / SNP Source: Applera /
Population(Allele,Count): Caucasian (T,28|C,8) Tfrican Tmerican (T,35|C,3)

total (T,63|C,11) / SNP Type: Silent Mutation / Protein Coding: SEQ ID NO: 130, at position 129,(Y,TAC) (Y,TAT) / SNP Source: dbSNP; HapMap; ABI_Val; HGBASE / Population(Allele,Count): Caucasian (C,62|T,164) / SNP Type: Silent Mutation / Protein Coding: SEQ ID NO: 130, at position 129,(Y,TAC) (Y,TAT) //
Context (SEQ ID NO: 249):
CTGGGCAGGGGTGGTGGGTACCAGAGAATGCTGCCTTCCCCCAAGCCTGCCCCTCTGCCTCATTTTCCTGTAGCTCCTCTGGT
TCTGTTTGCTCATTGGC
Y

GCTGTGTTCATCCAAGGGGGTTCTCCCAGAAGTGAGGGGCCTTTCCCTCCATCCCTTGGGGCACGGGGCAGCTGTGCCTGCCC
TGCCTCTGCCTGAGGCA / Celera SNP ID: hCV1347919 / Public SNP ID: rs1058003 / SNP Chromosome Position: 33590417 / SNP in Transcript Sequence SEQ ID NO: 46 / SNP Position Transcript: 2936 / Related Interrogated SNP: hCV1825046 / SNP Source: dbSNP; Celera; HapMap; ABI_Val; HGBASE / Population(Allele,Count): Caucasian (C,83|T,143) / SNP Type: UTR3 //

Gene Number: 37 / Gene Symbol OBP2B - 29989 / Gene Name: odorant binding protein 2B / Public Transcript Accession: NM_014581 / Public Protein Accession: NP_055396 / Chromosome: 9 / OMIM NUMBER: 604606 / OMIM Information: //
Transcript Sequence (SEQ ID NO: 47): // Protein Sequence (SEQ ID NO: 131): //
SNP Information //
Context (SEQ ID NO: 250):
TTTCCGGAGGACAGGAGGCCCAGGAAGGTGTCCCCAGTGAAGGTGACAGCCCTGGGCGGTGGGAAGTTGGAAGCCACGTTCAC
CTTCATGAGGGAGGATC
M

GTGCATCCAGAAGAAAATCCTGATGCGGAAGACGGAGGAGCCTGGCAAATACAGCGCCTATGGGGGCAGGAAGCTCATGTACC
TGCAGGAGCTGCCCAGG
Celera SNP ID: hCV27224736 / Public SNP ID: rs2073870 / SNP Chromosome Position: 136083580 / SNP in Transcript Sequence SEQ ID NO: 47 / SNP Position Transcript: 260 / Related Interrogated SNP: hCV25610857 / Related Interrogated SNP: hCV27859399 / SNP Source: dbSNP / Population(Allele,Count): Caucasian (A,32|C,192) / SNP Type: Silent Mutation / Protein Coding: SEQ ID NO: 131, at position 73,(R,CGG) (R,AGG) //

Gene Number: 38 / Gene Symbol GP6 - 51206 / Gene Name: glycoprotein VI (platelet) / Public Transcript Accession: NM_001083899 / Public Protein Accession: NP_001077368 / Chromosome: 19 / OMIM NUMBER: 605546 / OMIM Information: //
Transcript Sequence (SEQ ID NO: 48): // Protein Sequence (SEQ ID NO: 132): //
SNP Information //
Context (SEQ ID NO: 251):
CGGGGTTTCTGGCAGAGGACTGGCACAGCCGGAGGAAGCGCCTGCGGCACAGGGGCAGGGCTGTGCAGAGGCCGCTTCCGCCC
CTCCCGCCCCTCCCGCA
W
ACCCGGAAATCACACGGGGGTCAGGATGGAGGCCGACAGGATGTTCACAGCCGCGGGTTATGTTCATGACCGCTGAACCCCAG
GCACGGTCGTATCCAAG
Celera SNP ID: hCV1376266 / Public SNP ID: rs1654413 / SNP Chromosome Position: 55526359 / SNP in Transcript Sequence SEQ ID NO: 48 / SNP Position Transcript: 983 / SNP Source: Applera / Population(Allele,Count): Caucasian (T,0|A,24) Tfrican Tmerican (T,12|A,16) total (T,12|A,40) / SNP Type: Silent Mutation / Protein Coding: SEQ ID NO: 132, at position 318,(A,GCA) (A,GCT) / SNP Source: dbSNP; Celera; HGBASE / Population(Allele,Count): Caucasian (T,21|A,99) / SNP Type: Silent Mutation / Protein Coding: SEQ ID NO: 132, at position 318,(A,GCA) (A,GCT) //
Context (SEQ ID NO: 252):
GGAACCTCTGTGACCCCCAGCCGGTTACCAACAGAACCACCTTCCCCGGTAGCAGAATTCTCAGAAGCCACCGCTGAACTGAC
CGTCTCATTCACAAACG
R
AGTCTTCACAACTGAGACTTCTAGGAGTATCACCGCCAGTCCAAAGGAGTCAGACTCTCCAGCTGGTGAGTCCTGCCCGCCAG
TACTACACCAAGGGCAA
Celera SNP ID: hCV1376342 / Public SNP ID: rs1654416 / SNP Chromosome Position: 55530035 / SNP in Transcript Sequence SEQ ID NO: 48 / SNP Position Transcript: 738 / SNP Source: Applera / Population(Allele,Count): Gaucasian (G,0|A,16) African American (G,8|A,22) total (G,8|A,38) / SNP Type: Missense Mutation / Protein Coding: SEQ ID NO: 132, at position 237,(E,GAA) (K,AAA) / SNP

Source:   dbSNP; Celera;  HapMap; ABI_Val; HGBASE / Population(Allele,Count):
Gaucasian (G,47|A,179) / SNP Type:   Missense Mutation / Protein Coding:   SEQ ID
NO: 132, at position 237,(E,GAA) (K,AAA) //
Context         (SEQ ID NO: 253):
CCACCTTCCCCGGTAGCAGAATTCTCAGAAGCCACCGCTGAACTGACCGTCTCATTCACAAACGAAGTCTTCACAACTGAGAC
TTCTAGGAGTATCACCG
  R
CAGTCCAAAGGAGTCAGACTCTCCAGCTGGTGAGTCCTGCCCGCCAGTACTACACCAAGGGCAACCTGGTCCGGATATGCCTC
GGGGCTGTGATCCTAAT
Celera SNP ID:      hCV15973734 / Public SNP ID:      rs2304167 / SNP Chromosome
Position: 55527081 / SNP in Transcript Sequence    SEQ ID NO: 48 / SNP Position
Transcript:  774 / SNP Source:   dbSNP; HapMap; ABI_Val /
Population(Allele,Count):   Gaucasian (G,48|A,178) / SNP Type:   Missense Mutation
/ Protein Coding:   SEQ ID NO: 132, at position 249,(A,GCC) (T,ACC) //
 Context         (SEQ ID NO: 254):
AGGGACCCATACCTGTGGTCAGCCCCCAGCGACCCCCTGGAGCTTGTGGTCACAGGAACCTCTGTGACCCCCAGCCGGTTACC
AACAGAACCACCTTCCC
  Y
GGTAGCAGAATTCTCAGAAGCCACCGCTGAACTGACCGTCTCATTCACAAACGAAGTCTTCACAACTGAGACTTCTAGGAGTA
TCACCGCCAGTCCAAAG
 Celera SNP ID:      hCV8717873 / Public SNP ID:      rs1613662 / SNP Chromosome
Position: 55536595 / SNP in Transcript Sequence    SEQ ID NO: 48 / SNP Position
Transcript:  684 / SNP Source:   dbSNP; HapMap; HGBASE / Population(Allele,Count):
 Caucasian (C,43|T,183) / SNP Type:   Missense Mutation / Protein Coding:   SEQ ID
NO: 132, at position 219,(P,CCG) (S,TCG) //

Context         (SEQ ID NO: 255):
GAGGACTGGCACAGCCGGAGGAAGCGCCTGCGGCACAGGGGCAGGGCTGTGCAGAGGCCGCTTCCGCCCCTCCCGCCCCTCCC
GCAGACCCGGAAATCAC
  M
CGGGGGGTCAGGATGGAGGCCGACAGGATGTTCACAGCCGCGGGTTATGTTCATGACCGCTGAACCCCAGGCACGGTCGTATCC
AAGGGAGGGATCATGGC
Celera SNP ID:      hCV1376265 / Public SNP ID:      rs1671152 / SNP Chromosome
Position: 55526345 / SNP in Transcript Sequence    SEQ ID NO: 48 / SNP Position
Transcript:  997 / Related Interrogated SNP:   hCV8717873 / Related Interrogated
SNP:   hCV1376342 / Related Interrogated SNP:   hCV15973734 / Related Interrogated
SNP:   hCV1376266 / Related Interrogated SNP:   hCV8718961 / SNP Source:   Applera
/ Population(Allele,Count):   Caucasian (C,28|A,0)  African American (C,25|A,3)
total (C,53|A,3) / SNP Type:   Missense Mutation / Protein Coding:   SEQ ID NO:
132, at position 323,(T,ACA) (K,AAA) / SNP Source:   dbSNP; Celera;  HapMap; HGBASE
/ Population(Allele,Count):   Caucasian (A,41|C,181) / SNP Type:   Missense
Mutation / Protein Coding:   SEQ ID NO: 132, at position 323,(T,ACA) (K,AAA) //
Context         (SEQ ID NO: 256):
GACGTAACCCTACAGTGTCAGACTCGGTATGGCTTTGACCAATTTGCTCTGTACAAGGAAGGGGACCCTGCGCCCTACAAGAA
TCCCGAGAGATGGTACA
  M
GGCTAGTTTTCCCATCATCACGGTGACCGCCGCCCACAGCGGAACCTACCGATGCTACAGCTTCTCCAGCAGGGACCCATACC
TGTGGTCAGCCCCCAGC
Celera SNP ID:      hCV8717845 / Public SNP ID:      rs892090 / SNP Chromosome
Position: 55539072 / SNP in Transcript Sequence    SEQ ID NO: 48 / SNP Position
Transcript:  513 / Related Interrogated SNP:   hCV8717873 / Related Interrogated
SNP:   hCV1376342 / Related Interrogated SNP:   hCV1376266 / Related Interrogated
SNP:   hCV8718961 / Related Interrogated SNP:   hCV15973734 / SNP Source:   Applera
/ Population(Allele,Count):   Caucasian (C,36|A,2)  African American (C,29|A,5)
total (C,65|A,7) / SNP Type:   Silent Mutation / Protein Coding:   SEQ ID NO: 132,
at position 162,(R,AGG) (R,CGG) / SNP Source:   dbSNP; HapMap; HGBASE /
Population(Allele,Count):   Caucasian (A,43|C,183) / SNP Type:   Silent Mutation /
Protein Coding:   SEQ ID NO: 132, at position 162,(R,AGG) (R,CGG) //
Context         (SEQ ID NO: 257):
ACAGTGTCAGACTCGGTATGGCTTTGACCAATTTGCTCTGTACAAGGAAGGGGACCCTGCGCCCTACAAGAATCCCGAGAGAT
GGTACAGGGCTAGTTTT
  Y
CCATCATCACGGTGACCGCCGCCCACAGCGGAACCTACCGATGCTACAGCTTCTCCAGCAGGGACCCATACCTGTGGTCAGCC

CCCAGCGACCCCCTGGA
Celera SNP ID:       hCV8717846 / Public SNP ID:       rs892089 / SNP Chromosome
Position:  55539061 / SNP in Transcript Sequence       SEQ ID NO: 48 / SNP Position
Transcript:  524 / Related Interrogated SNP:   hCV8717873 / Related Interrogated
SNP:  hCV1376342 / Related Interrogated SNP:   hCV15973734 / Related Interrogated
SNP:  hCV1376266 / Related Interrogated SNP:       hCV8718961 / SNP Source:    Applera
/ Population(Allele,Count):    Caucasian (T,2|C,36) Tfrican Tmerican (T,15|C,21)
total (T,17|C,57) / SNP Type:    Silent Mutation / Protein Coding:     SEQ ID NO:
132, at position 165,(F,TTT) (F,TTC) / SNP Source:    dbSNP; Celera; HapMap;
ABI_Val; HGBASE / Population(Allele,Count):    Caucasian (T,22|C,98) / SNP Type:
Silent Mutation / Protein Coding:     SEQ ID NO: 132, at position 165,(F,TTT)
(F,TTC) //
Context              (SEQ ID NO: 258):
AGGGGGCTGAGGTGGGAGAATGGCTTGAGCCTGGGAGGCAGAGGTTGCAGTGAGCTGAGATCACACCACTGCACTCTAGCTCG
GGTGACGAAGCCTGACT
Y
TGTCTCAAAAAATACAGGGATGAATATGTCAATTACCCTGATTTGATCATAGCACGTTGTATACATGTACTGCAATATTGCTG
TCCACCCCATAAATATG
Celera SNP ID:       hCV1376262 / Public SNP ID:       rs1671150 / SNP Chromosome
Position:  55525497 / SNP in Transcript Sequence       SEQ ID NO: 48 / SNP Position
Transcript:  1845 / Related Interrogated SNP:   hCV1376342 / Related Interrogated
SNP:  hCV15973734 / Related Interrogated SNP:   hCV8717873 / Related Interrogated
SNP:  hCV1376266 / SNP Source:   dbSNP; Celera; HapMap; HGBASE /
Population(Allele,Count):    Caucasian (T,48|C,178) / SNP Type:    Missense Mutation
/ Protein Coding:     SEQ ID NO: 132, at position 606,(F,TTT) (L,CTT) //

Context              (SEQ ID NO: 259):
GCCTCTTTCTTCAACCTCTCTAATATGGGCTCCAGACGGATCTCTAAGGTTCCCAGCTCTCAGGGTTGACTCTGTTCCATCCT
CTGTGCAAAATCCTCCC
Y
TGCTTCCCTTTGGCCCTCTGTGCTCTTGTCTGGTTTTCCCCAGAAACTCTCACCCTCACTCCATCTCCCACTGCGGTCTAACA
AATCTCCTTTCGTCTCT
Celera SNP ID:       hCV1376264 / Public SNP ID:       rs1671151 / SNP Chromosome
Position:  55525894 / SNP in Transcript Sequence       SEQ ID NO: 48 / SNP Position
Transcript:  1448 / Related Interrogated SNP:   hCV1376342 / Related Interrogated
SNP:  hCV15973734 / Related Interrogated SNP:   hCV8717873 / Related Interrogated
SNP:  hCV1376266 / SNP Source:   dbSNP; HapMap / Population(Allele,Count):
Caucasian (C,48|T,178) / SNP Type:    Silent Mutation / Protein Coding:     SEQ ID
NO: 132, at position 473,(P,CCC) (P,CCT) //

Gene Number:  38 / Gene Symbol       GP6 - 51206 / Gene Name:  glycoprotein VI
(platelet) / Public Transcript Accession:  NM_016363 / Public Protein Accession:
NP_057447 / Chromosome:   19 / OMIM NUMBER:   605546 / OMIM Information: //
Transcript Sequence  (SEQ ID NO: 49): // Protein Sequence       (SEQ ID NO: 133): //
SNP Information //
Context              (SEQ ID NO: 260):
CGGGGTTTCTGGCAGAGGACTGGCACAGCCGGAGGAAGCGCCTGCGGCACAGGGGCAGGGCTGTGCAGAGGCCGCTTCCGCCC
CTCCCGCCCCTCCCGCA
W
ACCCGGAAATCACACGGGGGTCAGGATGGAGGCCGACAGGATGTTCACAGCCGCGGGTTATGTTCATGACCGCTGAACCCCAG
GCACGGTCGTATCCAAG
Celera SNP ID:       hCV1376266 / Public SNP ID:       rs1654413 / SNP Chromosome
Position:  55526359 / SNP in Transcript Sequence       SEQ ID NO: 49 / SNP Position
Transcript:  979 / SNP Source:   Applera / Population(Allele,Count):    Caucasian
(T,0|A,24) Tfrican Tmerican (T,12|A,16) total (T,12|A,40) / SNP Type:    Missense
Mutation / Protein Coding:     SEQ ID NO: 133, at position 317,(Q,CAG) (L,CTG) / SNP
Source:   dbSNP; Celera; HGBASE / Population(Allele,Count):    Caucasian
(T,21|A,99) / SNP Type:    Missense Mutation / Protein Coding:     SEQ ID NO: 133, at
position 317,(Q,CAG) (L,CTG) //
Context              (SEQ ID NO: 261):
GGAACCTCTGTGACCCCCAGCCGGTTACCAACAGAACCACCTTCCCCGGTAGCAGAATTCTCAGAAGCCACCGCTGAACTGAC
CGTCTCATTCACAAACG
R

AGTCTTCACAACTGAGACTTCTAGGAGTATCACCGCCAGTCCAAAGGAGTCAGACTCTCCAGCTGGTCCTGCCCGCCAGTACT
ACACCAAGGGCAACCTG
Celera SNP ID:      hCV1376342 / Public SNP ID:      rs1654416 / SNP Chromosome
Position: 55530035 / SNP in Transcript Sequence      SEQ ID NO: 49 / SNP Position
Transcript:  738 / SNP Source:    Applera / Population(Allele,Count): Gaucasian
(G,0|A,16)  African American (G,8|A,22)  total (G,8|A,38) / SNP Type:   Missense
Mutation / Protein Coding:    SEQ ID NO: 133, at position 237,(E,GAA) (K,AAA) / SNP
Source:   dbSNP; Celera; HapMap; ABI_Val; HGBASE / Population(Allele,Count):
Gaucasian (G,47|A,179) / SNP Type:   Missense Mutation / Protein Coding:    SEQ ID
NO: 133, at position 237,(E,GAA) (K,AAA) //
Context              (SEQ ID NO: 262):
CCACCTTCCCCGGTAGCAGAATTCTCAGAAGCCACCGCTGAACTGACCGTCTCATTCACAAACGAAGTCTTCACAACTGAGAC
TTCTAGGAGTATCACCG
     R

CAGTCCAAAGGAGTCAGACTCTCCAGCTGGTCCTGCCCGCCAGTACTACACCAAGGGCAACCTGGTCCGGATATGCCTCGGGG
CTGTGATCCTAATAATC
Celera SNP ID:      hCV15973734 / Public SNP ID:      rs2304167 / SNP Chromosome
Position: 55527081 / SNP in Transcript Sequence      SEQ ID NO: 49 / SNP Position
Transcript:  774 / SNP Source:    dbSNP; HapMap; ABI_Val /
Population(Allele,Count):   Gaucasian (G,48|A,178) / SNP Type:   Missense Mutation
/ Protein Coding:    SEQ ID NO: 133, at position 249,(A,GCC) (T,ACC) //
Context              (SEQ ID NO: 263):
AGGGACCCATACCTGTGGTCAGCCCCCAGCGACCCCCTGGAGCTTGTGGTCACAGGAACCTCTGTGACCCCCAGCCGGTTACC
AACAGAACCACCTTCCC
     Y

GGTAGCAGAATTCTCAGAAGCCACCGCTGAACTGACCGTCTCATTCACAAACGAAGTCTTCACAACTGAGACTTCTAGGAGTA
TCACCGCCAGTCCAAAG
Celera SNP ID:      hCV8717873 / Public SNP ID:      rs1613662 / SNP Chromosome
Position: 55536595 / SNP in Transcript Sequence      SEQ ID NO: 49 / SNP Position
Transcript:  684 / SNP Source:    dbSNP; HapMap; HGBASE / Population(Allele,Count):
Caucasian (C,43|T,183) / SNP Type:   Missense Mutation / Protein Coding:    SEQ ID
NO: 133, at position 219,(P,CCG) (S,TCG) //
Context              (SEQ ID NO: 264):
GAGGACTGGCACAGCCGGAGGAAGCGCCTGCGGCACAGGGGCAGGGCTGTGCAGAGGCCGCTTCCGCCCCTCCCGCCCCTCCC
GCAGACCCGGAAATCAC
     M

CGGGGGTCAGGATGGAGGCCGACAGGATGTTCACAGCCGCGGGTTATGTTCATGACCGCTGAACCCCAGGCACGGTCGTATCC
AAGGGAGGGATCATGGC
Celera SNP ID:      hCV1376265 / Public SNP ID:      rs1671152 / SNP Chromosome
Position: 55526345 / SNP in Transcript Sequence      SEQ ID NO: 49 / SNP Position
Transcript:  993 / Related Interrogated SNP:    hCV1376342 / Related Interrogated
SNP:   hCV15973734 / Related Interrogated SNP:    hCV1376266 / Related Interrogated
SNP:   hCV8718961 / SNP Source:    Applera
/ Population(Allele,Count):   Caucasian (C,28|A,0)  African American (C,25|A,3)
total (C,53|A,3) / SNP Type:   Missense Mutation / Protein Coding:    SEQ ID NO:
133, at position 322,(H,CAC) (N,AAC) / SNP Source:    dbSNP; Celera; HapMap; HGBASE
/ Population(Allele,Count):   Caucasian (A,41|C,181) / SNP Type:   Missense
Mutation / Protein Coding:    SEQ ID NO: 133, at position 322,(H,CAC) (N,AAC) //
Context              (SEQ ID NO: 265):
GACGTAACCCTACAGTGTCAGACTCGGTATGGCTTTGACCAATTTGCTCTGTACAAGGAAGGGGACCCTGCGCCCTACAAGAA
TCCCGAGAGATGGTACA
     M

GGCTAGTTTTCCCATCATCACGGTGACCGCCGCCCACAGCGGAACCTACCGATGCTACAGCTTCTCCAGCAGGGACCCATACC
TGTGGTCAGCCCCCAGC
Celera SNP ID:      hCV8717845 / Public SNP ID:      rs892090 / SNP Chromosome
Position: 55539072 / SNP in Transcript Sequence      SEQ ID NO: 49 / SNP Position
Transcript:  513 / Related Interrogated SNP:    hCV8717873 / Related Interrogated
SNP:   hCV1376342 / Related Interrogated SNP:    hCV1376266 / Related Interrogated
SNP:   hCV8718961 / Related Interrogated SNP:    hCV15973734 / SNP Source:    Applera
/ Population(Allele,Count):   Caucasian (C,36|A,2)  African American (C,29|A,5)
total (C,65|A,7) / SNP Type:   Silent Mutation / Protein Coding:    SEQ ID NO: 133,
at position 162,(R,AGG) (R,CGG) / SNP Source:    dbSNP; HapMap; HGBASE /
Population(Allele,Count):   Caucasian (A,43|C,183) / SNP Type:   Silent Mutation /

Protein Coding: SEQ ID NO: 133, at position 162,(R,AGG) (R,CGG) //
Context (SEQ ID NO: 266):
ACAGTGTCAGACTCGGTATGGCTTTGACCAATTTGCTCTGTACAAGGAAGGGGACCCTGCGCCCTACAAGAATCCCGAGAGAT
GGTACAGGGCTAGTTTT
Y
CCATCATCACGGTGACCGCCGCCCACAGCGGAACCTACCGATGCTACAGCTTCTCCAGCAGGGACCCATACCTGTGGTCAGCC
CCCAGCGACCCCCTGGA
Celera SNP ID: hCV8717846 / Public SNP ID: rs892089 / SNP Chromosome
Position: 55539061 / SNP in Transcript Sequence SEQ ID NO: 49 / SNP Position
Transcript: 524 / Related Interrogated SNP: hCV8717873 / Related Interrogated
SNP: hCV1376342 / Related Interrogated SNP: hCV15973734 / Related Interrogated
SNP: hCV1376266 / Related Interrogated SNP: hCV8718961 / SNP Source: Applera
/ Population(Allele,Count): Caucasian (T,2|C,36) Tfrican Tmerican (T,15|C,21)
total (T,17|C,57) / SNP Type: Silent Mutation / Protein Coding: SEQ ID NO:
133, at position 165,(F,TTT) (F,TTC) / SNP Source: dbSNP; Celera; HapMap;
ABI_Val; HGBASE / Population(Allele,Count): Caucasian (T,22|C,98) / SNP Type:
Silent Mutation / Protein Coding: SEQ ID NO: 133, at position 165,(F,TTT)
(F,TTC) //
Context (SEQ ID NO: 267): /
CAGGGGGCTGAGGTGGGAGAATGGCTTGAGCCTGGGAGGCAGAGGTTGCAGTGAGCTGAGATCACACCACTGCACTCTAGCTC
GGGTGACGAAGCCTGAC
Y
TTGTCTCAAAAAATACAGGGATGAATATGTCAATTACCCTGATTTGATCATAGCACGTTGTATACATGTACTGCAATATTGCT
GTCCACCCCATAAATAT / Celera SNP ID: hCV1376262 / Public SNP ID: rs1671150 /
SNP Chromosome Position: 55525497 / SNP in Transcript Sequence SEQ ID NO: 49 /
SNP Position Transcript: 1840 / Related Interrogated SNP: hCV1376342 / Related
Interrogated SNP: hCV15973734 / Related Interrogated SNP: hCV8717873 / Related
Interrogated SNP: hCV1376266 / SNP Source: dbSNP; Celera; HapMap; HGBASE /
Population(Allele,Count): Caucasian (T,48|C,178) / SNP Type: UTR3 //
Context (SEQ ID NO: 268): /
GGCCTCTTTCTTCAACCTCTCTAATATGGGCTCCAGACGGATCTCTAAGGTTCCCAGCTCTCAGGGTTGACTCTGTTCCATCC
TCTGTGCAAAATCCTCC
Y
GTGCTTCCCTTTGGCCCTCTGTGCTCTTGTCTGGTTTTCCCCAGAAACTCTCACCCTCACTCCATCTCCCACTGCGGTCTAAC
AAATCTCCTTTCGTCTC / Celera SNP ID: hCV1376264 / Public SNP ID: rs1671151 /
SNP Chromosome Position: 55525894 / SNP in Transcript Sequence SEQ ID NO: 49 /
SNP Position Transcript: 1443 / Related Interrogated SNP: hCV1376342 / Related
Interrogated SNP: hCV15973734 / Related Interrogated SNP: hCV8717873 / Related
Interrogated SNP: hCV1376266 / SNP Source: dbSNP; HapMap /
Population(Allele,Count): Caucasian (C,48|T,178) / SNP Type: UTR3 //

Gene Number: 39 / Gene Symbol NAT8B - 51471 / Gene Name: N-acetyltransferase 8B
(GCN5-related, putative, gene/pseudogene) / Public Transcript Accession: NM_016347
/ Public Protein Accession: NP_057431 / Chromosome: 2 / OMIM NUMBER: / OMIM
Information: // Transcript Sequence (SEQ ID NO: 50): // Protein Sequence (SEQ
ID NO: 134): // SNP Information //
Context (SEQ ID NO: 269):
AAAAACCCTGGACGCGGTATGTAGACATAGCATTGCGCACAGACATGTCTGACATCACCAAATCCTACCTGAGTGAGTGTGGC
TCCTGCTTCTGGGTGGG
S
GAATCTGAAGAGAAGGTGGTGGGCACAGTAGGAGCTCTGCCCGTTGATGATCCCACCTTGAGGGAGAAGCGGTTGCAGCTGTT
TCATCTCTCTGTGGACA / Celera SNP ID: hCV11541681 / Public SNP ID: rs2001490 /
SNP Chromosome Position: 73928098 / SNP in Transcript Sequence SEQ ID NO: 50 /
SNP Position Transcript: 371 / SNP Source: Applera / Population(Allele,Count):
Gaucasian (G,10|C,10) African American (G,18|C,14) total (G,28|C,24) // / SNP
Type: Missense Mutation / Protein Coding: SEQ ID NO: 134, at position
112,(G,GGT) (A,GCT) / SNP Source: dbSNP; Celera; HGBASE /
Population(Allele,Count): Gaucasian (G,40|C,80) / SNP Type: Missense Mutation /
Protein Coding: SEQ ID NO: 134, at position 112,(G,GGT) (A,GCT) //

Gene Number: 40 / Gene Symbol DCHS2 - 54798 / Gene Name: dachsous 2
(Drosophila) / Public Transcript Accession: NM_001142552 / Public Protein
Accession: NP_001136024 / Chromosome: 4 / OMIM NUMBER: / OMIM Information: //

Transcript Sequence (SEQ ID NO: 51): // Protein Sequence (SEQ ID NO: 135): // SNP Information //
Context (SEQ ID NO: 270):
TCAACGACGTGAATGACCACTCGCCCCGCTTTCCCCTCGACTCCCTGCAACTCGACGTCTCCGAGCTCAGCCCGCCAGGGACC
GCCTTCCGCCTGCCAGT
K
GCCCACGATCCGGACGCCGGACTGTTCAGCACTCAGGGCTACACCCTGGTGCAACCGTCCGACCTGCCCAAGGACCCCGCAGG
CCCGTTCTTCCAGTTGC / Celera SNP ID: hCV1125378 / Public SNP ID: rs17373874 /
SNP Chromosome Position: 155411939 / SNP in Transcript Sequence SEQ ID NO: 51 /
SNP Position Transcript: 940 / SNP Source: dbSNP; Celera; HapMap /
Population(Allele,Count): Gaucasian (T,63|G,43) / SNP Type: Missense Mutation /
Protein Coding: SEQ ID NO: 135, at position 190,(V,GTT) (G,GGT) //
Context (SEQ ID NO: 271):
GGAGAAGGAAGATGAGGCCACAGGGGAGCTTGGTGTGGGTCTTGGAGACGGGAGCATCTCTCTGTCCTTGGAAGGCGGAGAGG
GAGACTTCGCGTTGCTA
R
CCGGCGGCCCCCCAGGGGTATTTTTTCCTTTGCGTGGAGGGGCCCCTGGACAGAGAGAGCCGCGATCTGTATGAGTTACTACTG
GTGGCCACGGACGCGGG / Celera SNP ID: hCV28954780 / Public SNP ID: rs7656522 /
SNP Chromosome Position: 155411065 / SNP in Transcript Sequence SEQ ID NO: 51 /
SNP Position Transcript: 1814 / Related Interrogated SNP: hCV11503469 / Related
Interrogated SNP: hCV11503414 / SNP Source: dbSNP; HapMap /
Population(Allele,Count): Gaucasian (A,54|G,170) / SNP Type: Silent Rare Codon
/ Protein Coding: SEQ ID NO: 135, at position 481,(L,CTA) (L,CTG) //
Context (SEQ ID NO: 272):
GTCTCCGAGCTCAGCCCGCCAGGGACCGCCTTCCGCCTGCCAGTTGCCCACGATCCGGACGCCGGACTGTTCAGCACTCAGGG
CTACACCCTGGTGCAAC
Y
GTCCGACCTGCCCAAGGACCCCGCAGGCCCGTTCTTCCAGTTGCGCTACCGGACTCCGGGGCCACTACCGTCACCGCTTTTGC
CAGGCTCCTCGTCACCC / Celera SNP ID: hDV70945235 / Public SNP ID: rs17373860
/ SNP Chromosome Position: 155411883 / SNP in Transcript Sequence SEQ ID NO: 51
/ SNP Position Transcript: 996 / Related Interrogated SNP: hCV11503414 / Related
Interrogated SNP: hCV11503469 / Related Interrogated SNP: hCV11503470 / Related
Interrogated SNP: hCV15860433 / Related Interrogated SNP: hCV2892877 / SNP
Source: dbSNP; HapMap / Population(Allele,Count): Caucasian (C,104|T,12) / SNP
Type: Missense Mutation / Protein Coding: SEQ ID NO: 135, at position
209,(P,CCG) (S,TCG) //

Gene Number: 40 / Gene Symbol DCHS2 - 54798 / Gene Name: dachsous 2
(Drosophila) / Public Transcript Accession: NM_001142553 / Public Protein
Accession: NP_001136025 / Chromosome: 4 / OMIM NUMBER: / OMIM Information: //
Transcript Sequence (SEQ ID NO: 52): // Protein Sequence (SEQ ID NO: 136): //
SNP Information //
Context (SEQ ID NO: 273):
TCAACGACGTGAATGACCACTCGCCCCGCTTTCCCCTCGACTCCCTGCAACTCGACGTCTCCGAGCTCAGCCCGCCAGGGACC
GCCTTCCGCCTGCCAGT
K
GCCCACGATCCGGACGCCGGACTGTTCAGCACTCAGGGCTACACCCTGGTGCAACCGTCCGACCTGCCCAAGGACCCCGCAGG
CCCGTTCTTCCAGTTGC / Celera SNP ID: hCV1125378 / Public SNP ID: rs17373874 /
SNP Chromosome Position: 155411939 / SNP in Transcript Sequence SEQ ID NO: 52 /
SNP Position Transcript: 993 / SNP Source: dbSNP; Celera; HapMap /
Population(Allele,Count): Gaucasian (T,63|G,43) / SNP Type: Missense Mutation /
Protein Coding: SEQ ID NO: 136, at position 190,(V,GTT) (G,GGT) //
Context (SEQ ID NO: 274):
GGAGAAGGAAGATGAGGCCACAGGGGAGCTTGGTGTGGGTCTTGGAGACGGGAGCATCTCTCTGTCCTTGGAAGGCGGAGAGG
GAGACTTCGCGTTGCTA
R
CCGGCGGCCCCCCAGGGGTATTTTTTCCTTTGCGTGGAGGGGCCCCTGGACAGAGAGAGCCGCGATCTGTATGAGTTACTACTG
GTGGCCACGGACGCGGG / Celera SNP ID: hCV28954780 / Public SNP ID: rs7656522 /
SNP Chromosome Position: 155411065 / SNP in Transcript Sequence SEQ ID NO: 52 /
SNP Position Transcript: 1867 / Related Interrogated SNP: hCV11503469 / Related
Interrogated SNP: hCV11503414 / SNP Source: dbSNP; HapMap /
Population(Allele,Count): Gaucasian (A,54|G,170) / SNP Type: Silent Rare Codon
/ Protein Coding: SEQ ID NO: 136, at position 481,(L,CTA) (L,CTG) //

Context (SEQ ID NO: 275):
GTCTCCGAGCTCAGCCCGCCAGGGACCGCCTTCCGCCTGCCAGTTGCCCACGATCCGGACGCCGGACTGTTCAGCACTCAGGG
CTACACCCTGGTGCAAC
Y

GTCCGACCTGCCCAAGGACCCCGCAGGCCCGTTCTTCCAGTTGCGCTACCGGACTCCGGGGCCACTACCGTCACCGCTTTTGC
CAGGCTCCTCGTCACCC / Celera SNP ID: hDV70945235 / Public SNP ID: rs17373860
/ SNP Chromosome Position: 155411883 / SNP in Transcript Sequence SEQ ID NO: 52
/ SNP Position Transcript: 1049 / Related Interrogated SNP: hCV11503414 /
Related Interrogated SNP: hCV11503469 / Related Interrogated SNP: hCV11503470 /
Related Interrogated SNP: hCV15860433 / Related Interrogated SNP: hCV2892877 /
SNP Source: dbSNP; HapMap / Population(Allele,Count): Caucasian (C,104|T,12) /
SNP Type: Missense Mutation / Protein Coding: SEQ ID NO: 136, at position
209,(P,CCG) (S,TCG) //

Gene Number: 41 / Gene Symbol GPATCH4 - 54865 / Gene Name: G patch domain
containing 4 / Public Transcript Accession: NM_015590 / Public Protein Accession:
NP_056405 / Chromosome: 1 / OMIM NUMBER: / OMIM Information: // Transcript
Sequence (SEQ ID NO: 53): // Protein Sequence (SEQ ID NO: 137): // SNP
Information //
Context (SEQ ID NO: 276):
GAGCAGAGCATGCAGTGATGGAAGAAGCAGGAAAAGCAAGAAGAAAAGACAGCAGCATCAAGAGGAGGAGGACATCTTGGATG
TAAGGGATGAGAAGGAT
R

GCGGGGCTAGGGAAGCAGAGAGCAGAGCACACACTGGCTCAAGCAGCAGAGGTAAGAGGAAGAGGCAGCAGCATCCCAAGAAG
GAAAGAGCTGGAGTCAG / Celera SNP ID: hCV9102827 / Public SNP ID: rs3795733 /
SNP Chromosome Position: 156564922 / SNP in Transcript Sequence SEQ ID NO: 53 /
SNP Position Transcript: 1250 / SNP Source: Applera / Population(Allele,Count):
Gaucasian (G,10|A,30) African American (G,26|A,10) total (G,36|A,40) // / SNP
Type: UTR3 / SNP Source: dbSNP; Celera; HapMap; HGBASE /
Population(Allele,Count): Gaucasian (G,61|A,165) / SNP Type: UTR3 //
Context (SEQ ID NO: 277):
GGAGTTGAAGCCTCAAAGACCAGGGTTGATGCAGGTCTGCAGGTCTTCTGCACCCCCCTCAATGAGGAGTCCCTCCCAGAAAG
GAAACTGATCTCTGGGA
Y

GTCAGCTGCTGAGAGGAGCAAGCGGTAGTACCACCCCTTAGTTGAGGGAGTCAGCACAGTCCTTTCTGCAGCTTCTAACCCAG
GACCATGAACTCAGGTG / Celera SNP ID: hCV9102829 / Public SNP ID: rs3795732 /
SNP Chromosome Position: 156564640 / SNP in Transcript Sequence SEQ ID NO: 53 /
SNP Position Transcript: 1532 / Related Interrogated SNP: hCV9102827 / SNP
Source: dbSNP; Celera; HapMap; ABI_Val; HGBASE / Population(Allele,Count):
Caucasian (C,56|T,168) / SNP Type: UTR3 //

Gene Number: 41 / Gene Symbol GPATCH4 - 54865 / Gene Name: G
patch domain containing 4 / Public Transcript Accession: NM_182679 / Public
Protein Accession: NP_872620 / Chromosome: 1 / OMIM NUMBER: / OMIM Information:
// Transcript Sequence (SEQ ID NO: 54): // Protein Sequence (SEQ ID NO: 138):
// SNP Information //
Context (SEQ ID NO: 278): /
GAGCAGAGCATGCAGTGATGGAAGAAGCAGGAAAAGCAAGAAGAAAAGACAGCAGCATCAAGAGGAGGAGGACATCTTGGATG
TAAGGGATGAGAAGGAT
R

GCGGGGCTAGGGAAGCAGAGAGCAGAGCACACACTGGCTCAAGCAGCAGAGGTAAGAGGAAGAGGCAGCAGCATCCCAAGAAG
GAAAGAGCTGGAGTCAG / Celera SNP ID: hCV9102827 / Public SNP ID: rs3795733 /
SNP Chromosome Position: 156564922 / SNP in Transcript Sequence SEQ ID NO: 54 /
SNP Position Transcript: 1324 / SNP Source: Applera / Population(Allele,Count):
Gaucasian (G,10|A,30) African American (G,26|A,10) total (G,36|A,40) // / SNP
Type: UTR3 / SNP Source: dbSNP; Celera; HapMap; HGBASE /
Population(Allele,Count): Gaucasian (G,61|A,165) / SNP Type: UTR3 //
Context (SEQ ID NO: 279):
GGAGTTGAAGCCTCAAAGACCAGGGTTGATGCAGGTCTGCAGGTCTTCTGCACCCCCCTCAATGAGGAGTCCCTCCCAGAAAG
GAAACTGATCTCTGGGA
Y

GTCAGCTGCTGAGAGGAGCAAGCGGTAGTACCACCCCTTAGTTGAGGGAGTCAGCACAGTCCTTTCTGCAGCTTCTAACCCAG

GACCATGAACTCAGGTG / Celera SNP ID: hCV9102829 / Public SNP ID: rs3795732 /
SNP Chromosome Position: 156564640 / SNP in Transcript Sequence SEQ ID NO: 54 /
SNP Position Transcript: 1606 / Related Interrogated SNP: hCV9102827 / SNP
Source: dbSNP; Celera; HapMap; ABI_Val; HGBASE / Population(Allele,Count):
Caucasian (C,56|T,168) / SNP Type: UTR3 //

Gene Number: 42 / Gene Symbol CHFR - 55743 / Gene Name: checkpoint with
forkhead and ring finger domains / Public Transcript Accession: NM_001161344 /
Public Protein Accession: NP_001154816 / Chromosome: 12 / OMIM NUMBER: / OMIM
Information: // Transcript Sequence (SEQ ID NO: 55): // Protein Sequence (SEQ
ID NO: 139): // SNP Information //
Context (SEQ ID NO: 280):
CCGTCAGCCTGACGACAGCAGTCCAGGATTACGTGTGCCCTCTGCAAGGAAGCCACGCCCTGTGCACCTGCTGCTTCCAGCCC
ATGCCCGACCGGAGAGC
Y
GAGCGCGAGCAGGACCCGCGTGTCGCCCCTCAGCAGTGTGCGGTCTGCCTGCAGCCTTTCTGCCACCTGTACTGGGGCTGCAC
CCGGACCGGCTGCTACG / Celera SNP ID: hCV1859941 / Public SNP ID: rs2306541 /
SNP Chromosome Position: 133428242 / SNP in Transcript Sequence SEQ ID NO: 55 /
SNP Position Transcript: 1575 / Related Interrogated SNP: hCV1859855 / SNP
Source: Applera / Population(Allele,Count): Caucasian (T,4|C,20) Tfrican
Tmerican (T,9|C,27) total (T,13|C,47) / SNP Type: Missense Mutation / Protein
Coding: SEQ ID NO: 139, at position 497,(A,GCG) (V,GTG) / SNP Source: dbSNP;
Celera; HapMap; ABI_Val; HGBASE / Population(Allele,Count): Caucasian
(C,151|T,75) / SNP Type: Missense Mutation / Protein Coding: SEQ ID NO: 139,
at position 497,(A,GCG) (V,GTG) //
Context (SEQ ID NO: 281):
CTGGGGTGACTCTTCTGTGGAGCTTTACCCTCTGAGTGAGACCCTCCCCAGAGCCCCGGGGGCCGCAGCCCGCCCTCCTGGTG
AGCGCTGGGCAGGGCTC
R
TGGTGGCATCAGCAGCAGAGACGAAGCCTTTCTGTAACATGCGGCCGTCCCGCCGAGAGGGGCAGTTTTGCTCTTTTGTACAT
TTTCCGAAACTACAGTT / Celera SNP ID: hCV25761477 / Public SNP ID: rs3741490 /
SNP Chromosome Position: 133417908 / SNP in Transcript Sequence SEQ ID NO: 55 /
SNP Position Transcript: 2311 / Related Interrogated SNP: hCV1859855 / SNP
Source: Applera / Population(Allele,Count): Gaucasian (G,2|A,2) African
American (G,20|A,0) total (G,22|A,2) / SNP Type: UTR3 / SNP Source: dbSNP;
Celera; HGBASE / Population(Allele,Count): Gaucasian (G,151|A,75) / SNP Type:
UTR3 //
Context (SEQ ID NO: 282):
GCTGCTCCCTTGTGTGAGTGACCGCGGCCCCGAGCCTGGGGCTGGACGCAGGTCCCAGGACGTGCTGCTCCCTTGTGTGAGTG
ACCACGGCCCCAAGCCC
R
GGGCTGGAGGCAGGTCCCAGGACGCGCCGCTCCCTCATGCTGCCCGGGCCCTTCCTCCAAGACCCTACAGAGCCTGAGGGGCA
CCTTGGCTTCCGCCTGT / Celera SNP ID: hCV12052839 / Public SNP ID: rs8021 /
SNP Chromosome Position: 133417285 / SNP in Transcript Sequence SEQ ID NO: 55 /
SNP Position Transcript: 2934 / Related Interrogated SNP: hCV1859855 / SNP
Source: dbSNP; Celera; HapMap; ABI_Val; HGBASE / Population(Allele,Count):
Gaucasian (A,78|G,40) / SNP Type: UTR3 //

Gene Number: 42 / Gene Symbol CHFR - 55743 / Gene Name: checkpoint with
forkhead and ring finger domains / Public Transcript Accession: NM_001161345 /
Public Protein Accession: NP_001154817 / Chromosome: 12 / OMIM NUMBER: / OMIM
Information: // Transcript Sequence (SEQ ID NO: 56): // Protein Sequence (SEQ
ID NO: 140): // SNP Information //
Context (SEQ ID NO: 283):
CGTCCGTCAGCCTGACGACAGCAGTCCAGGATTACGTGTGCCCTCTGCAAGGAAGCCACGCCCTGTGCACCTGCTGCTTCCAGCCC
ATGCCCGACCGGAGAGC
Y
GAGCGCGAGCAGGACCCGCGTGTCGCCCCTCAGCAGTGTGCGGTCTGCCTGCAGCCTTTCTGCCACCTGTACTGGGGCTGCAC
CCGGACCGGCTGCTACG / Celera SNP ID: hCV1859941 / Public SNP ID: rs2306541 /
SNP Chromosome Position: 133428242 / SNP in Transcript Sequence SEQ ID NO: 56 /
SNP Position Transcript: 1572 / Related Interrogated SNP: hCV1859855 / SNP
Source: Applera / Population(Allele,Count): Caucasian (T,4|C,20) Tfrican
Tmerican (T,9|C,27) total (T,13|C,47) / SNP Type: Missense Mutation / Protein

Coding: SEQ ID NO: 140, at position 496,(A,GCG) (V,GTG) / SNP Source: dbSNP; Celera; HapMap; ABI_Val; HGBASE / Population(Allele,Count): Caucasian (C,151|T,75) / SNP Type: Missense Mutation / Protein Coding: SEQ ID NO: 140, at position 496,(A,GCG) (V,GTG) //
Context (SEQ ID NO: 284):
CTGGGGTGACTCTTCTGTGGAGCTTTACCCTCTGAGTGAGACCCTCCCCAGAGCCCCGGGGGCCGCAGCCCGCCCTCCTGGTG
AGCGCTGGGCAGGGCTC
R
TGGTGGCATCAGCAGCAGAGACGAAGCCTTTCTGTAACATGCGGCCGTCCCGCCGAGAGGGGCAGTTTTGCTCTTTTGTACAT
TTTCCGAAACTACAGTT / Celera SNP ID: hCV25761477 / Public SNP ID: rs3741490 / SNP Chromosome Position: 133417908 / SNP in Transcript Sequence SEQ ID NO: 56 / SNP Position Transcript: 2308 / Related Interrogated SNP: hCV1859855 / SNP Source: Applera / Population(Allele,Count): Gaucasian (G,2|A,2) African American (G,20|A,0) total (G,22|A,2) / SNP Type: UTR3 / SNP Source: dbSNP; Celera; HGBASE / Population(Allele,Count): Gaucasian (G,151|A,75) / SNP Type: UTR3 //
Context (SEQ ID NO: 285):
GCTGCTCCCTTGTGTGAGTGACCGCGGCCCCGAGCCTGGGGCTGGACGCAGGTCCCAGGACGTGCTGCTCCCTTGTGTGAGTG
ACCACGGCCCCAAGCCC
R
GGGCTGGAGGCAGGTCCCAGGACGCGCCGCTCCCTCATGCTGCCCGGGCCCTTCCTCCAAGACCCTACAGAGCCTGAGGGGCA
CCTTGGCTTCCGCCTGT / Celera SNP ID: hCV12052839 / Public SNP ID: rs8021 / SNP Chromosome Position: 133417285 / SNP in Transcript Sequence SEQ ID NO: 56 / SNP Position Transcript: 2931 / Related Interrogated SNP: hCV1859855 / SNP Source: dbSNP; Celera; HapMap; ABI_Val; HGBASE / Population(Allele,Count): Gaucasian (A,78|G,40) / SNP Type: UTR3 //

Gene Number: 42 / Gene Symbol CHFR - 55743 / Gene Name: checkpoint with forkhead and ring finger domains / Public Transcript Accession: NM_001161346 / Public Protein Accession: NP_001154818 / Chromosome: 12 / OMIM NUMBER: / OMIM Information: // Transcript Sequence (SEQ ID NO: 57): // Protein Sequence (SEQ ID NO: 141): // SNP Information //
Context (SEQ ID NO: 286):
CCGTCAGCCTGACGACAGCAGTCCAGGATTACGTGTGCCCTCTGCAAGGAAGCCACGCCCTGTGCACCTGCTGCTTCCAGCCC
ATGCCCGACCGGAGAGC
Y
GAGCGCGAGCAGGACCCGCGTGTCGCCCCTCAGCAGTGTGCGGTCTGCCTGCAGCCTTTCTGCCACCTGTACTGGGGCTGCAC
CCGGACCGGCTGCTACG / Celera SNP ID: hCV1859941 / Public SNP ID: rs2306541 / SNP Chromosome Position: 133428242 / SNP in Transcript Sequence SEQ ID NO: 57 / SNP Position Transcript: 1539 / Related Interrogated SNP: hCV1859855 / SNP Source: Applera / Population(Allele,Count): Caucasian (T,4|C,20) Tfrican Tmerican (T,9|C,27) total (T,13|C,47) / SNP Type: Missense Mutation / Protein Coding: SEQ ID NO: 141, at position 485,(A,GCG) (V,GTG) / SNP Source: dbSNP; Celera; HapMap; ABI_Val; HGBASE / Population(Allele,Count): Caucasian (C,151|T,75) / SNP Type: Missense Mutation / Protein Coding: SEQ ID NO: 141, at position 485,(A,GCG) (V,GTG) //
Context (SEQ ID NO: 287):
CTGGGGTGACTCTTCTGTGGAGCTTTACCCTCTGAGTGAGACCCTCCCCAGAGCCCCGGGGGCCGCAGCCCGCCCTCCTGGTG
AGCGCTGGGCAGGGCTC
R
TGGTGGCATCAGCAGCAGAGACGAAGCCTTTCTGTAACATGCGGCCGTCCCGCCGAGAGGGGCAGTTTTGCTCTTTTGTACAT
TTTCCGAAACTACAGTT / Celera SNP ID: hCV25761477 / Public SNP ID: rs3741490 / SNP Chromosome Position: 133417908 / SNP in Transcript Sequence SEQ ID NO: 57 / SNP Position Transcript: 2275 / Related Interrogated SNP: hCV1859855 / SNP Source: Applera / Population(Allele,Count): Gaucasian (G,2|A,2) African American (G,20|A,0) total (G,22|A,2) / SNP Type: UTR3 / SNP Source: dbSNP; Celera; HGBASE / Population(Allele,Count): Gaucasian (G,151|A,75) / SNP Type: UTR3 //
Context (SEQ ID NO: 288):
GCTGCTCCCTTGTGTGAGTGACCGCGGCCCCGAGCCTGGGGCTGGACGCAGGTCCCAGGACGTGCTGCTCCCTTGTGTGAGTG
ACCACGGCCCCAAGCCC
R
GGGCTGGAGGCAGGTCCCAGGACGCGCCGCTCCCTCATGCTGCCCGGGCCCTTCCTCCAAGACCCTACAGAGCCTGAGGGGCA

CCTTGGCTTCCGCCTGT / Celera SNP ID: hCV12052839 / Public SNP ID: rs8021 / SNP Chromosome Position: 133417285 / SNP in Transcript Sequence SEQ ID NO: 57 / SNP Position Transcript: 2898 / Related Interrogated SNP: hCV1859855 / SNP Source: dbSNP; Celera; HapMap; ABI_Val; HGBASE / Population(Allele,Count): Gaucasian (A,78|G,40) / SNP Type: UTR3 //

Gene Number: 42 / Gene Symbol CHFR - 55743 / Gene Name: checkpoint with forkhead and ring finger domains / Public Transcript Accession: NM_001161347 / Public Protein Accession: NP_001154819 / Chromosome: 12 / OMIM NUMBER: / OMIM Information: // Transcript Sequence (SEQ ID NO: 58): // Protein Sequence (SEQ ID NO: 142): // SNP Information //
Context (SEQ ID NO: 289):
CCGTCAGCCTGACGACAGCAGTCCAGGATTACGTGTGCCCTCTGCAAGGAAGCCACGCCCTGTGCACCTGCTGCTTCCAGCCC
ATGCCCGACCGGAGAGC
Y
GAGCGCGAGCAGGACCCGCGTGTCGCCCCCTCAGCAGTGTGCGGTCTGCCTGCAGCCTTTCTGCCACCTGTACTGGGGCTGCAC
CCGGACCGGCTGCTACG / Celera SNP ID: hCV1859941 / Public SNP ID: rs2306541 / SNP Chromosome Position: 133428242 / SNP in Transcript Sequence SEQ ID NO: 58 / SNP Position Transcript: 1299 / Related Interrogated SNP: hCV1859855 / SNP Source: Applera / Population(Allele,Count): Caucasian (T,4|C,20) Tfrican Tmerican (T,9|C,27) total (T,13|C,47) / SNP Type: Missense Mutation / Protein Coding: SEQ ID NO: 142, at position 405,(A,GCG) (V,GTG) / SNP Source: dbSNP; Celera; HapMap; ABI_Val; HGBASE / Population(Allele,Count): Caucasian (C,151|T,75) / SNP Type: Missense Mutation / Protein Coding: SEQ ID NO: 142, at position 405,(A,GCG) (V,GTG) //
Context (SEQ ID NO: 290):
CTGGGGTGACTCTTCTGTGGAGCTTTACCCTCTGAGTGAGACCCTCCCCAGAGCCCCGGGGGCCGCAGCCCGCCCTCCTGGTG
AGCGCTGGGCAGGGCTC
R
TGGTGGCATCAGCAGCAGAGACGAAGCCTTTCTGTAACATGCGGCCGTCCCGCCGAGAGGGGCAGTTTTGCTCTTTTGTACAT
TTTCCGAAACTACAGTT / Celera SNP ID: hCV25761477 / Public SNP ID: rs3741490 / SNP Chromosome Position: 133417908 / SNP in Transcript Sequence SEQ ID NO: 58 / SNP Position Transcript: 2035 / Related Interrogated SNP: hCV1859855 / SNP Source: Applera / Population(Allele,Count): Caucasian (G,2|A,2) African American (G,20|A,0) total (G,22|A,2) / SNP Type: UTR3 / SNP Source: dbSNP; Celera; HGBASE / Population(Allele,Count): Gaucasian (G,151|A,75) / SNP Type: UTR3 //
Context (SEQ ID NO: 291):
GCTGCTCCCTTGTGTGAGTGACCGCGGCCCCGAGCCTGGGGCTGGACGCAGGTCCCAGGACGTGCTGCTCCCTTGTGTGAGTG
ACCACGGCCCCAAGCCC
R
GGGCTGGAGGCAGGTCCCAGGACGCGCCGCTCCCTCATGCTGCCCGGGCCCTTCCTCCAAGACCCTACAGAGCCTGAGGGGCA
CCTTGGCTTCCGCCTGT / Celera SNP ID: hCV12052839 / Public SNP ID: rs8021 / SNP Chromosome Position: 133417285 / SNP in Transcript Sequence SEQ ID NO: 58 / SNP Position Transcript: 2658 / Related Interrogated SNP: hCV1859855 / SNP Source: dbSNP; Celera; HapMap; ABI_Val; HGBASE / Population(Allele,Count): Gaucasian (A,78|G,40) / SNP Type: UTR3 //

Gene Number: 42 / Gene Symbol CHFR - 55743 / Gene Name: checkpoint with forkhead and ring finger domains / Public Transcript Accession: NM_018223 / Public Protein Accession: NP_060693 / Chromosome: 12 / OMIM NUMBER: / OMIM Information: // Transcript Sequence (SEQ ID NO: 59): // Protein Sequence (SEQ ID NO: 143): // SNP Information //
Context (SEQ ID NO: 292): /
CCGTCAGCCTGACGACAGCAGTCCAGGATTACGTGTGCCCTCTGCAAGGAAGCCACGCCCTGTGCACCTGCTGCTTCCAGCCC
ATGCCCGACCGGAGAGC
Y
GAGCGCGAGCAGGACCCGCGTGTCGCCCCCTCAGCAGTGTGCGGTCTGCCTGCAGCCTTTCTGCCACCTGTACTGGGGCTGCAC
CCGGACCGGCTGCTACG / Celera SNP ID: hCV1859941 / Public SNP ID: rs2306541 / SNP Chromosome Position: 133428242 / SNP in Transcript Sequence SEQ ID NO: 59 / SNP Position Transcript: 1452 / Related Interrogated SNP: hCV1859855 / SNP Source: Applera / Population(Allele,Count): Caucasian (T,4|C,20) Tfrican Tmerican (T,9|C,27) total (T,13|C,47) / SNP Type: Missense Mutation / Protein

Coding: SEQ ID NO: 143, at position 456,(A,GCG) (V,GTG) / SNP Source: dbSNP; Celera; HapMap; ABI_Val; HGBASE / Population(Allele,Count): Caucasian (C,151|T,75) / SNP Type: Missense Mutation / Protein Coding: SEQ ID NO: 143, at position 456,(A,GCG) (V,GTG) //
Context (SEQ ID NO: 293):
CTGGGGTGACTCTTCTGTGGAGCTTTACCCTCTGAGTGAGACCCTCCCCAGAGCCCCGGGGGCCGCAGCCCGCCCTCCTGGTG
AGCGCTGGGCAGGGCTC
R
TGGTGGCATCAGCAGCAGAGACGAAGCCTTTCTGTAACATGCGGCCGTCCCGCCGAGAGGGGCAGTTTTGCTCTTTTGTACAT
TTTCCGAAACTACAGTT / Celera SNP ID: hCV25761477 / Public SNP ID: rs3741490 /
SNP Chromosome Position: 133417908 / SNP in Transcript Sequence SEQ ID NO: 59 /
SNP Position Transcript: 2188 / Related Interrogated SNP: hCV1859855 / SNP
Source: Applera / Population(Allele,Count): Gaucasian (G,2|A,2) African
American (G,20|A,0) total (G,22|A,2) / SNP Type: UTR3 / SNP Source: dbSNP;
Celera; HGBASE / Population(Allele,Count): Gaucasian (G,151|A,75) / SNP Type:
UTR3 //
Context (SEQ ID NO: 294):
GCTGCTCCCTTGTGTGAGTGACCGCGGCCCCGAGCCTGGGGCTGGACGCAGGTCCCAGGACGTGCTGCTCCCTTGTGTGAGTG
ACCACGGCCCCAAGCCC
R
GGGCTGGAGGCAGGTCCCAGGACGCGCCGCTCCCTCATGCTGCCCGGGCCCTTCCTCCAAGACCCTACAGAGCCTGAGGGGCA
CCTTGGCTTCCGCCTGT / Celera SNP ID: hCV12052839 / Public SNP ID: rs8021 /
SNP Chromosome Position: 133417285 / SNP in Transcript Sequence SEQ ID NO: 59 /
SNP Position Transcript: 2811 / Related Interrogated SNP: hCV1859855 / SNP
Source: dbSNP; Celera; HapMap; ABI_Val; HGBASE / Population(Allele,Count):
Gaucasian (A,78|G,40) / SNP Type: UTR3 //

Gene Number: 43 / Gene Symbol SPATA7 - 55812 / Gene Name: spermatogenesis
associated 7 / Public Transcript Accession: NM_001040428 / Public Protein
Accession: NP_001035518 / Chromosome: 14 / OMIM NUMBER: / OMIM Information: //
Transcript Sequence (SEQ ID NO: 60): // Protein Sequence (SEQ ID NO: 144): //
SNP Information //
Context (SEQ ID NO: 295):
GCAACCTCTGTCCTTCCCAGATATGGTCCACCGTGCCTATTTAAAGGACACTTGAGCACCAAAAGTAATGCTGCAGTAGACTG
CTCGGTTCCAGTAAGCG
R
GAGTACCAGCATAAAGTATGCAGACCAACAACGAAGAGAGAAACTCAAAAAGGAATTAGCACAATGTGAAAAAGAGTTCAAAT
TAACTAAAACTGCAATG / Celera SNP ID: hCV2485039 / Public SNP ID: rs3179969 /
SNP Chromosome Position: 88862529 / SNP in Transcript Sequence SEQ ID NO: 60 /
SNP Position Transcript: 300 / Related Interrogated SNP: hCV16182835 / SNP
Source: dbSNP; Celera; HapMap; HGBASE / Population(Allele,Count): Caucasian
(G,149|A,77) / SNP Type: Missense Mutation / Protein Coding: SEQ ID NO: 144,
at position 42,(V,GTG) (M,ATG) //

Gene Number: 43 / Gene Symbol SPATA7 - 55812 / Gene Name: spermatogenesis
associated 7 / Public Transcript Accession: NM_018418 / Public Protein Accession:
NP_060888 / Chromosome: 14 / OMIM NUMBER: / OMIM Information: // Transcript
Sequence (SEQ ID NO: 61): // Protein Sequence (SEQ ID NO: 145): // SNP
Information //
Context (SEQ ID NO: 296):
AGACTAAGCACTCTCCAGCTGGTCAAGAATCACATGGCTGTTCACTATAATAAAATCCTTTCAGCCAAAGCTGCAGTAGACTG
CTCGGTTCCAGTAAGCG
R
GAGTACCAGCATAAAGTATGCAGACCAACAACGAAGAGAGAAACTCAAAAAGGAATTAGCACAATGTGAAAAAGAGTTCAAAT
TAACTAAAACTGCAATG / Celera SNP ID: hCV2485039 / Public SNP ID: rs3179969 /
SNP Chromosome Position: 88862529 / SNP in Transcript Sequence SEQ ID NO: 61 /
SNP Position Transcript: 396 / Related Interrogated SNP: hCV16182835 / SNP
Source: dbSNP; Celera; HapMap; HGBASE / Population(Allele,Count): Caucasian
(G,149|A,77) / SNP Type: Missense Mutation / Protein Coding: SEQ ID NO: 145,
at position 74,(V,GTG) (M,ATG) //

Gene Number: 44 / Gene Symbol ACSS2 - 55902 / Gene Name: acyl-CoA synthetase
short-chain family member 2 / Public Transcript Accession: NM_001076552 / Public

Protein Accession: NP_001070020 / Chromosome: 20 / OMIM NUMBER: / OMIM Information: // Transcript Sequence (SEQ ID NO: 62): // Protein Sequence (SEQ ID NO: 146): // SNP Information //
Context (SEQ ID NO: 297):
GGAATTCTGGGGAGACATTGCCAAGGAATTTTACTGGAAGACTCCATGCCCTGGCCCATTCCTTCGGTACAACTTTGATGTGA CTAAAGGGAAAATCTTC
R
TTGAGTGGATGAAAGGAGCAACTACCAACATCTGCTACAATGTACTGGATCGAAATGTCCATGAGAAAAAGCTTGGAGATAAA GTTGCTTTTTACTGGGA / Celera SNP ID: hCV25750225 / Public SNP ID: rs4911163 / SNP Chromosome Position: 33470694 / SNP in Transcript Sequence SEQ ID NO: 62 / SNP Position Transcript: 398 / Related Interrogated SNP: hCV1825046 / SNP Source: Applera / Population(Allele,Count): Caucasian (G,12|A,22) African American (G,23|A,15) total (G,35|A,37) // / SNP Type: Silent Mutation / Protein Coding: SEQ ID NO: 146, at position 92,(F,TTC) (F,TTT) / SNP Source: dbSNP; Celera; HapMap; HGBASE / Population(Allele,Count): Caucasian (G,85|A,141) / SNP Type: Silent Mutation / Protein Coding: SEQ ID NO: 146, at position 92,(F,TTC) (F,TTT) //

Gene Number: 44 / Gene Symbol ACSS2 - 55902 / Gene Name: acyl-CoA synthetase short-chain family member 2 / Public Transcript Accession: NM_018677 / Public Protein Accession: NP_061147 / Chromosome: 20 / OMIM NUMBER: / OMIM Information: // Transcript Sequence (SEQ ID NO: 63): // Protein Sequence (SEQ ID NO: 147): // SNP Information //
Context (SEQ ID NO: 298):
GGAATTCTGGGGAGACATTGCCAAGGAATTTTACTGGAAGACTCCATGCCCTGGCCCATTCCTTCGGTACAACTTTGATGTGA CTAAAGGGAAAATCTTC
R
TTGAGTGGATGAAAGGAGCAACTACCAACATCTGCTACAATGTACTGGATCGAAATGTCCATGAGAAAAAGCTTGGAGATAAA GTTGCTTTTTACTGGGA / Celera SNP ID: hCV25750225 / Public SNP ID: rs4911163 / SNP Chromosome Position: 33470694 / SNP in Transcript Sequence SEQ ID NO: 63 / SNP Position Transcript: 398 / Related Interrogated SNP: hCV1825046 / SNP Source: Applera / Population(Allele,Count): Caucasian (G,12|A,22) African American (G,23|A,15) total (G,35|A,37) // / SNP Type: Silent Mutation / Protein Coding: SEQ ID NO: 147, at position 92,(F,TTC) (F,TTT) / SNP Source: dbSNP; Celera; HapMap; HGBASE / Population(Allele,Count): Caucasian (G,85|A,141) / SNP Type: Silent Mutation / Protein Coding: SEQ ID NO: 147, at position 92,(F,TTC) (F,TTT) //

Gene Number: 45 / Gene Symbol SCYL3 - 57147 / Gene Name: SCY1-like 3 (S. cerevisiae) / Public Transcript Accession: NM_020423 / Public Protein Accession: NP_065156 / Chromosome: 1 / OMIM NUMBER: / OMIM Information: // Transcript Sequence (SEQ ID NO: 64): // Protein Sequence (SEQ ID NO: 148): // SNP Information //
Context (SEQ ID NO: 299):
AACCAAGATCTTCAAACGCACTGCCCCAAGTTTTACTAAAAATACTGACCTTTCTCTAGAAGGCGATCCATTTTCTCAGCCTA TTAAATTTCCCATAAAT
Y
GACTCTCAGATGTAAAAAATACTTCGGAGGACAGTGAAAACTTCCCATCAAGTTCTAAAAAGTCTGAGGAGTGGCCTGACTGG AGTGAACCTGAGGAGCC / Celera SNP ID: hCV32141820 / Public SNP ID: rs12132384 / SNP Chromosome Position: 169824068 / SNP in Transcript Sequence SEQ ID NO: 64 / SNP Position Transcript: 1549 / Related Interrogated SNP: hCV11975250 / SNP Source: dbSNP; HapMap / Population(Allele,Count): Caucasian (T,198|C,28) / SNP Type: Silent Mutation / Protein Coding: SEQ ID NO: 148, at position 450,(N,AAT) (N,AAC) //
Context (SEQ ID NO: 300):
TCTGCTTGGACCAGAGGTGGTTGTGGGAGGAGAACGAACCAAGATCTTCAAACGCACTGCCCCAAGTTTTACTAAAAATACTG ACCTTTCTCTAGAAGGC
Y
ATCCATTTTCTCAGCCTATTAAATTTCCCATAAATGGACTCTCAGATGTAAAAAATACTTCGGAGGACAGTGAAAACTTCCCA TCAAGTTCTAAAAAGTC / Celera SNP ID: hCV32141821 / Public SNP ID: rs12135726 / SNP Chromosome Position: 169824104 / SNP in Transcript Sequence SEQ ID NO: 64 / SNP Position Transcript: 1513 / Related Interrogated SNP: hCV11975250 / SNP Source: dbSNP; HapMap / Population(Allele,Count): Caucasian (C,198|T,28) / SNP

Type:   Silent Rare Codon / Protein Coding:   SEQ ID NO: 148, at position 438,(G,GGC) (G,GGT) //
Context                (SEQ ID NO: 301):
GCTGCTGTCTCACATCGAGGCCTACGTGGAGCACTTCACTCAGGAGCAGCTGAAGAAAGTCATCTTGCCACAGGTTTTGCTGG
GCCTGCGTGATACTAGC
Y
ATTCCATTGTGGCAATTACTCTGCATAGCCTAGCAGTGCTGGTCTCTCTGCTTGGACCAGAGGTGGTTGTGGGAGGAGAACGA
ACCAAGATCTTCAAACG / Celera SNP ID:    hDV70975002 / Public SNP ID:    rs17602701
/ SNP Chromosome Position:  169828327 / SNP in Transcript Sequence    SEQ ID NO: 64
/ SNP Position Transcript:  1366 / Related Interrogated SNP:   hCV11975250 / SNP
Source:   dbSNP; HapMap / Population(Allele,Count):   Caucasian (C,194|T,28) / SNP
Type:   Silent Mutation / Protein Coding:   SEQ ID NO: 148, at position
389,(S,AGC) (S,AGT) //

Gene Number:  45 / Gene Symbol                SCYL3 - 57147 / Gene Name:
SCY1-like 3 (S. cerevisiae) / Public Transcript Accession:  NM_181093 / Public
Protein Accession:   NP_851607 / Chromosome:   1 / OMIM NUMBER: / OMIM Information:
// Transcript Sequence  (SEQ ID NO: 65): // Protein Sequence    (SEQ ID NO: 149):
// SNP Information //
Context                (SEQ ID NO: 302):
CACGCCCATCAACAGCAAGAAGCACATACAGCGAGATTACTACAATACTCTTTTACAGACAGGCGATCCATTTTCTCAGCCTA
TTAAATTTCCCATAAAT
Y
GACTCTCAGATGTAAAAAAATACTTCGGAGGACAGTGAAAACTTCCCATCAAGTTCTAAAAAGTCTGAGGAGTGGCCTGACTGG
AGTGAACCTGAGGAGCC / Celera SNP ID:    hCV32141820 / Public SNP ID:    rs12132384
/ SNP Chromosome Position:  169824068 / SNP in Transcript Sequence    SEQ ID NO: 65
/ SNP Position Transcript:  1711 / Related Interrogated SNP:   hCV11975250 / SNP
Source:   dbSNP; HapMap / Population(Allele,Count):   Caucasian (T,198|C,28) / SNP
Type:   Silent Mutation / Protein Coding:   SEQ ID NO: 149, at position
504,(N,AAT) (N,AAC) //
Context                (SEQ ID NO: 303):
CTCTAGCATATTCCCTAAATGTTTCTTTTCTGGCAGCACGCCCATCAACAGCAAGAAGCACATACAGCGAGATTACTACAATA
CTCTTTTACAGACAGGC
Y
ATCCATTTTCTCAGCCTATTAAATTTCCCATAAATGGACTCTCAGATGTAAAAAAATACTTCGGAGGACAGTGAAAACTTCCCA
TCAAGTTCTAAAAAGTC / Celera SNP ID:    hCV32141821 / Public SNP ID:    rs12135726
/ SNP Chromosome Position:  169824104 / SNP in Transcript Sequence    SEQ ID NO: 65
/ SNP Position Transcript:  1675 / Related Interrogated SNP:   hCV11975250 / SNP
Source:   dbSNP; HapMap / Population(Allele,Count):   Caucasian (C,198|T,28) / SNP
Type:   Silent Rare Codon / Protein Coding:   SEQ ID NO: 149, at position
492,(G,GGC) (G,GGT) //
Context                (SEQ ID NO: 304):
GCTGCTGTCTCACATCGAGGCCTACGTGGAGCACTTCACTCAGGAGCAGCTGAAGAAAGTCATCTTGCCACAGGTTTTGCTGG
GCCTGCGTGATACTAGC
Y
ATTCCATTGTGGCAATTACTCTGCATAGCCTAGCAGTGCTGGTCTCTCTGCTTGGACCAGAGGTGGTTGTGGGAGGAGAACGA
ACCAAGATCTTCAAACG / Celera SNP ID:    hDV70975002 / Public SNP ID:    rs17602701
/ SNP Chromosome Position:  169828327 / SNP in Transcript Sequence    SEQ ID NO: 65
/ SNP Position Transcript:  1366 / Related Interrogated SNP:   hCV11975250 / SNP
Source:   dbSNP; HapMap / Population(Allele,Count):   Caucasian (C,194|T,28) / SNP
Type:   Silent Mutation / Protein Coding:   SEQ ID NO: 149, at position
389,(S,AGC) (S,AGT) //

Gene Number:  46 / Gene Symbol                MYH7B - 57644 / Gene Name:
myosin, heavy chain 7B, cardiac muscle, beta / Public Transcript Accession:
NM_020884 / Public Protein Accession:   NP_065935 / Chromosome:   20 / OMIM NUMBER:
/ OMIM Information: // Transcript Sequence  (SEQ ID NO: 66): // Protein Sequence
(SEQ ID NO: 150): // SNP Information //
Context                (SEQ ID NO: 305):
TGACCAGCGCCTGGCCAAGGTGCTGACGCTGCTGCAGGCGCGGAGCCGTGGCCGCCTCATGCGCCTTGAGTACCAGCGCCTGC
TGGGAGGCAGGGATGCG
R
TGTTCACCATCCAGTGGAACATCCGTGCCTTCAATGCCGTCAAGAACTGGTCATGGATGAAGCTCTTTTTTCAAGATGAAGCCG

CTGCTGCGCTCGGCGCA / Celera SNP ID:     hCV16013546 / Public SNP ID:     rs2425012 / SNP Chromosome Position: 33581955 / SNP in Transcript Sequence    SEQ ID NO: 66 / SNP Position Transcript: 2895 / Related Interrogated SNP:    hCV1825046 / SNP Source:    dbSNP; HapMap; ABI_Val; HGBASE / Population(Allele,Count):    Caucasian (G,125|A,101) / SNP Type:    Silent Rare Codon / Protein Coding:    SEQ ID NO: 150, at position 859,(A,GCG) (A,GCA) //
Context        (SEQ ID NO: 306):
ACCCCAGGGGTCATGGATGCCTTCTTGGTGCTACACCAGCTGCGCTGCAATGGGGTCCTGGAGGGGATCCGGATCTGCCGCCA
AGGGTTCCCCAACAGGT
R

GCTCTACACCGACTTCCGGCAGCGGTACCGTATCCTGAACCCCAGTGCCATCCCGGATGACACCTTCATGGACAGCAGGAAGG
CCACAGAGAAACTGCTG / Celera SNP ID:     hCV16013558 / Public SNP ID:     rs2425009 / SNP Chromosome Position: 33578899 / SNP in Transcript Sequence    SEQ ID NO: 66 / SNP Position Transcript: 2602 / Related Interrogated SNP:    hCV1825046 / SNP Source:    dbSNP; HapMap; HGBASE / Population(Allele,Count):    Caucasian (A,35|G,85) / SNP Type:    Silent Rare Codon / Protein Coding:    SEQ ID NO: 150, at position 762,(L,TTG) (L,CTG) //
Context        (SEQ ID NO: 307):
CTGACCCCCTGGGCTCTAAAGAGGAATGTCTGCTGTTGCACATCTGGCTGAGGCCACCTGCCCCGATCCTGCCATCTCTGCAT
CGCCCCCTGCTGCCTTC
R
GCCTTCCCTGGGCCCTGAATAAACACCACAGCCAGTTTCCTTCTCATTCTTTTTCTTTGGGGTTCAGGAGGAAAAACACAGTCC
TAGGGACAAAAGCCAGG / Celera SNP ID:     hCV27893015 / Public SNP ID:     rs4911167 / SNP Chromosome Position: 33590096 / SNP in Transcript Sequence    SEQ ID NO: 66 / SNP Position Transcript: 6374 / Related Interrogated SNP:    hCV1825046 / SNP Source:    dbSNP; HGBASE / Population(Allele,Count):    Caucasian (A,83|G,143) / SNP Type:    UTR3 //
Context        (SEQ ID NO: 308):
GCCTCCCAAAGTGCTGGAATTACAGACATGAGTGGCAATAAAAGGGGTAGCAGAGCTTCCTGCCCTCACCGTGGTGCCGAGTG
CCTGCTGCCTTGGGCCG
R
CTTGAACCTCCAGGGTTTCCAGCTCCTCCTCCTTCACCCCAGTGCCACTGCCATGATGGATGTGAGTGAACTTGGGGAGTCTG
CCCGCTACCTCCGCCAG / Celera SNP ID:     hCV32066133 / Public SNP ID:     rs11906160 / SNP Chromosome Position: 33565755 / SNP in Transcript Sequence    SEQ ID NO: 66 / SNP Position Transcript: 391 / Related Interrogated SNP:    hCV25620145 / SNP Source:    dbSNP; HapMap / Population(Allele,Count):    Caucasian (G,200|A,26) / SNP Type:    Missense Mutation / Protein Coding:    SEQ ID NO: 150, at position 25,(A,GCC) (T,ACC) //
Context        (SEQ ID NO: 309):
CTTAAGTGATGTGTCCGCCTGGGCCTCCCAAAGTGCTGGAATTACAGACATGAGTGGCAATAAAAGGGGTAGCAGAGCTTCCT
GCCCTCACCGTGGTGCC
Y
AGTGCCTGCTGCCTTGGGCCGCCTTGAACCTCCAGGGTTTCCAGCTCCTCCTCCTTCACCCCAGTGCCACTGCCATGATGGAT
GTGAGTGAACTTGGGGA / Celera SNP ID:     hCV30323913 / Public SNP ID:     rs6060137 / SNP Chromosome Position: 33565480 / SNP in Transcript Sequence    SEQ ID NO: 66 / SNP Position Transcript: 369 / Related Interrogated SNP:    hCV1825046 / SNP Source:    dbSNP; HapMap / Population(Allele,Count):    Caucasian (C,165|T,61) / SNP Type:    Silent Mutation / Protein Coding:    SEQ ID NO: 150, at position 17,(A,GCC) (A,GCT) //

Gene Number: 47 / Gene Symbol        ZNF317 - 57693 / Gene Name: zinc finger protein 317 / Public Transcript Accession: NM_001190791 / Public Protein Accession:    NP_001177720 / Chromosome: 19 / OMIM NUMBER: / OMIM Information: // Transcript Sequence  (SEQ ID NO: 67): // Protein Sequence    (SEQ ID NO: 151): // SNP Information //
Context        (SEQ ID NO: 310):
ATGGGCCAAGGAGGGGTCAGCGGCGAATTCTTTCGGCCTGTTGGGGACCCCTCGTGTCCCCACGCCAGACTGGACCTCCCGTA
TGAACTTCTCTTCGCAT
Y
GGCGGCGGCTTCCGTCACCTCCGCTCCCCCGATCCTATTCCCAGTCGTATGCCAGCTTGGAGAGTCACGTGAGAGCAAGAGAG
TAGCTTTCTGGTTGTCC / Celera SNP ID:     hDV70794769 / Public SNP ID:     rs17002013 / SNP Chromosome Position: 9251220 / SNP in Transcript Sequence    SEQ ID NO: 67 / SNP Position Transcript: 165 / SNP Source:    dbSNP; HapMap /

Population(Allele,Count): Caucasian (C,177|T,49) / SNP Type: UTR5 //

Gene Number: 47 / Gene Symbol ZNF317 - 57693 / Gene Name: zinc finger protein 317 / Public Transcript Accession: NM_020933 / Public Protein Accession: NP_065984 / Chromosome: 19 / OMIM NUMBER: / OMIM Information: // Transcript Sequence (SEQ ID NO: 68): // Protein Sequence (SEQ ID NO: 152): // SNP Information //
Context (SEQ ID NO: 311):
ATGGGCCAAGGAGGGGTCAGCGGCGAATTCTTTCGGCCTGTTGGGGACCCCTCGTGTCCCCACGCCAGACTGGACCTCCCGTA
TGAACTTCTCTTCGCAT
Y
GGCGGCGGCTTCCGTCACCTCCGCTCCCCCGATCCTATTCCCAGTCGTATGCCAGCTTGGAGAGTCACGTGAGAGCAAGAGAG
TAGCTTTCTGGTTGTCC / Celera SNP ID: hDV70794769 / Public SNP ID: rs17002013
/ SNP Chromosome Position: 9251220 / SNP in Transcript Sequence SEQ ID NO: 68 /
SNP Position Transcript: 165 / SNP Source: dbSNP; HapMap /
Population(Allele,Count): Caucasian (C,177|T,49) / SNP Type: UTR5 //

Gene Number: 48 / Gene Symbol C1orf114 - 57821 / Gene Name: chromosome 1 open reading frame 114 / Public Transcript Accession: NM_021179 / Public Protein Accession: NP_067002 / Chromosome: 1 / OMIM NUMBER: / OMIM Information: // Transcript Sequence (SEQ ID NO: 69): // Protein Sequence (SEQ ID NO: 153): // SNP Information //
Context (SEQ ID NO: 312):
AGAATCAAGAACCGGTGAATGATAAAAGGGAGCGAAAACTTAAGTTCAAGGACCAGTTAGTTGATTTGGAAGTTCCTCCACTA
GAAGACACTACTACTTT
Y
AAAAATTATTTTTGAAAACGAAAGGAATATGTTTGGGAAACTGTCACAATTATGTATTTCCAATGATTTTGGACAAGAAGATGT
GCTCCTGTCACTTACTA / Celera SNP ID: hCV2456747 / Public SNP ID: rs3820059 /
SNP Chromosome Position: 169391154 / SNP in Transcript Sequence SEQ ID NO: 69 /
SNP Position Transcript: 684 / SNP Source: Applera / Population(Allele,Count):
Caucasian (T,12|C,22) Tfrican Tmerican (T,18|C,4) total (T,30|C,26) / SNP Type:
Missense Mutation / Protein Coding: SEQ ID NO: 153, at position 172,(F,TTT)
(S,TCT) / SNP Source: Applera / Population(Allele,Count): Caucasian (T,6|C,30)
Tfrican Tmerican (T,6|C,12) total (T,12|C,42) / SNP Type: Missense Mutation /
Protein Coding: SEQ ID NO: 153, at position 172,(F,TTT) (S,TCT) / SNP Source:
Applera / Population(Allele,Count): Caucasian (T,8|C,30) Tfrican Tmerican
(T,9|C,11) total (T,17|C,41) / SNP Type: Missense Mutation / Protein Coding:
SEQ ID NO: 153, at position 172,(F,TTT) (S,TCT) / SNP Source: dbSNP; Celera;
HapMap; ABI_Val; HGBASE / Population(Allele,Count): Caucasian (T,81|C,145) / SNP
Type: Missense Mutation / Protein Coding: SEQ ID NO: 153, at position
172,(F,TTT) (S,TCT) //

Gene Number: 49 / Gene Symbol CFHR5 - 81494 / Gene Name: complement factor H-related 5 / Public Transcript Accession: NM_030787 / Public Protein Accession: NP_110414 / Chromosome: 1 / OMIM NUMBER: / OMIM Information: // Transcript Sequence (SEQ ID NO: 70): // Protein Sequence (SEQ ID NO: 154): // SNP Information //
Context (SEQ ID NO: 313):
AGCTTCCCCTTAGTACATTGAAATTCAAAGTCATGCTTGTAACTGTTAATGAAAGCAGATTTAAAGCAACACCACCATCACTG
GAGTATTTTTAGTTATA
Y
ACGATTGAGACTACCAAGCATGTTGCTCTTATTCAGTGTAATCCTAATCTCATGGGTATCCACTGTTGGGGGAGAAGGAACAC
TTTGTGATTTTCCAAAA / Celera SNP ID: hCV9114656 / Public SNP ID: rs9427662 /
SNP Chromosome Position: 196946775 / SNP in Transcript Sequence SEQ ID NO: 70 /
SNP Position Transcript: 109 / Related Interrogated SNP: hCV2532034 / SNP
Source: Applera / Population(Allele,Count): Caucasian (C,4|T,34) African
American (C,10|T,28) total (C,14|T,62) / SNP Type: UTR5 / SNP Source: dbSNP;
Celera; HapMap / Population(Allele,Count): Caucasian (T,207|C,19) / SNP Type:
UTR5 //

Gene Number: 50 / Gene Symbol RNASE7 - 84659 / Gene Name: ribonuclease, RNase A family, 7 / Public Transcript Accession: NM_032572 / Public Protein Accession: NP_115961 / Chromosome: 14 / OMIM NUMBER: / OMIM

Information: // Transcript Sequence (SEQ ID NO: 71): // Protein Sequence (SEQ ID NO: 155): // SNP Information //
Context (SEQ ID NO: 314):
TGCCAGACCCCCAAAATAGCCTGCAAGAATGGCGATAAAAACTGCCACCAGAGCCACGGGGCCGTGTCCCTGACCATGTGTAA
GCTCACCTCAGGGAAGT
Y
TCCGAACTGCAGGTACAAAGAGAAGCGACAGAACAAGTCTTACGTAGTGGCCTGTAAGCCTCCCCAGAAAAAGGACTCTCAGC
AATTCCACCTGGTTCCT / Celera SNP ID: hCV2434510 / Public SNP ID: rs1243469 /
SNP Chromosome Position: 21511497 / SNP in Transcript Sequence SEQ ID NO: 71 /
SNP Position Transcript: 604 / SNP Source: Applera / Population(Allele,Count):
Caucasian (C,1|T,35) African American (C,5|T,31) total (C,6|T,66) / SNP Type:
Missense Mutation / Protein Coding: SEQ ID NO: 155, at position 116,(Y,TAT)
(H,CAT) / SNP Source: Applera / Population(Allele,Count): Caucasian (C,4|T,36)
African American (C,5|T,31) total (C,9|T,67) / SNP Type: Missense Mutation /
Protein Coding: SEQ ID NO: 155, at position 116,(Y,TAT) (H,CAT) / SNP Source:
dbSNP; Celera; HapMap; ABI_Val; HGBASE / Population(Allele,Count): Caucasian
(C,27|T,199) / SNP Type: Missense Mutation / Protein Coding: SEQ ID NO: 155,
at position 116,(Y,TAT) (H,CAT) //

Gene Number: 51 / Gene Symbol C3orf15 - 89876 / Gene Name:
chromosome 3 open reading frame 15 / Public Transcript Accession: NM_033364 /
Public Protein Accession: NP_203528 / Chromosome: 3 / OMIM NUMBER: / OMIM
Information: // Transcript Sequence (SEQ ID NO: 72): // Protein Sequence (SEQ
ID NO: 156): // SNP Information //
Context (SEQ ID NO: 315):
TACACAGGCAAATATCCAAGCTACCCTGATTCGCAGCAGACTGAGAAAAGTTCCCAGGTTTAAAACCATGTTCAGTAACCTGA
TCCATTATCCAAGATAT
Y
CTCTATATTGGAGCAAGTCAGATCCTGTCCCACCATTTATCAGTCGGGGAATGGAAGGGACATAAGGAGAAACACAGAGAAGCC
CTCCGGCAGCTCACCAC / Celera SNP ID: hCV134278 / Public SNP ID: rs9848716 /
SNP Chromosome Position: 119426307 / SNP in Transcript Sequence SEQ ID NO: 72 /
SNP Position Transcript: 336 / Related Interrogated SNP: hCV263841 / SNP Source:
dbSNP; Celera; HapMap / Population(Allele,Count): Caucasian (T,64|C,160) / SNP
Type: Silent Mutation / Protein Coding: SEQ ID NO: 156, at position 86,(Y,TAT)
(Y,TAC) //
Context (SEQ ID NO: 316):
GTGTGTTCATGCAACAGCCATAGAGATGGTGCTATTGAAAGGCAATCTGCCCATCCTGTGTATTGACAAGCATCAGGATTCCA
GGAGGATTCTTCCTGAT
K
CAAAGACTGTCAAATAGGAAAAAAGAAAACAGTAAATGAACAAAAACATCAACATGGAGTCCAAGTAATGAATTTATAGAGGA
AAATGTATGCTACTCTG / Celera SNP ID: hCV255886 / Public SNP ID: rs10511394 /
SNP Chromosome Position: 119484601 / SNP in Transcript Sequence SEQ ID NO: 72 /
SNP Position Transcript: 3085 / Related Interrogated SNP: hCV263841 / SNP
Source: dbSNP; Celera; HapMap / Population(Allele,Count): Caucasian
(T,172|G,54) / SNP Type: UTR3 //
Context (SEQ ID NO: 317):
TTACTTATAATGGCTTGAGTTAAAATGCTCCAACTTTTCTAATTTTTTGCTTTTGAAAATAAAACACCTATAGTGACAATAAAA
ACACACATTTAAAGCCA / W / AAAAAAAAAAAAAAAA / Celera SNP ID: hCV1834237 / Public
SNP ID: rs9865270 / SNP Chromosome Position: 119485949 / SNP in Transcript
Sequence SEQ ID NO: 72 / SNP Position Transcript: 4433 / Related Interrogated
SNP: hCV263841 / SNP Source: dbSNP; Celera; HapMap / Population(Allele,Count):
Caucasian (A,168|T,58) / SNP Type: UTR3 //

Gene Number: 52 / Gene Symbol RDH13 - 112724 / Gene Name:
retinol dehydrogenase 13 (all-trans/9-cis) / Public Transcript Accession:
NM_001145971 / Public Protein Accession: NP_001139443 / Chromosome: 19 / OMIM
NUMBER: / OMIM Information: // Transcript Sequence (SEQ ID NO: 73): // Protein
Sequence (SEQ ID NO: 157): // SNP Information //
Context (SEQ ID NO: 318):
CACGGTAGCAGGCGCCGCCGTGCTGCTCAAGGACTATGTCACCGGTGGGGCTTGCCCCAGCAAGGCCACCATCCCTGGGAAGA
CGGTCATCGTGACGGGT
Y
CCAACACAGGCATCGGGAAGCAGACCGCCTTGGAACTGGCCAGGAGAGGAGGCAACATCATCCTGGCCTGCCGAGACATGGAG

AAGTGTGAGGCGGCAGC / Celera SNP ID: hCV8718972 / Public SNP ID: rs1654447 / SNP Chromosome Position: 55570574 / SNP in Transcript Sequence SEQ ID NO: 73 / SNP Position Transcript: 321 / Related Interrogated SNP: hCV8717873 / Related Interrogated SNP: hCV8718961 / Related Interrogated SNP: hCV1376266 / Related Interrogated SNP: hCV1376342 / Related Interrogated SNP: hCV15973734 / SNP Source: dbSNP; HapMap; ABI_Val; HGBASE / Population(Allele,Count): Caucasian (T,42|C,184) / SNP Type: Silent Rare Codon / Protein Coding: SEQ ID NO: 157, at position 45,(G,GGT) (G,GGC) //

Gene Number: 52 / Gene Symbol RDH13 - 112724 / Gene Name: retinol dehydrogenase 13 (all-trans/9-cis) / Public Transcript Accession: NM_138412 / Public Protein Accession: NP_612421 / Chromosome: 19 / OMIM NUMBER: / OMIM Information: // Transcript Sequence (SEQ ID NO: 74): // Protein Sequence (SEQ ID NO: 158): // SNP Information //
Context (SEQ ID NO: 319):
GCTTCGGCCTCCTGAGTAGCTGGGACTACAGGGACTATGTCACCGGTGGGGCTTGCCCCAGCAAGGCCACCATCCCTGGGAAG
ACGGTCATCGTGACGGG
Y
GCCAACACAGGCATCGGGAAGCAGACCGCCTTGGAACTGGCCAGGAGAGGAGGCAACATCATCCTGGCCTGCCGAGACATGGA
GAAGTGTGAGGCGGCAG / Celera SNP ID: hCV8718972 / Public SNP ID: rs1654447 / SNP Chromosome Position: 55570574 / SNP in Transcript Sequence SEQ ID NO: 74 / SNP Position Transcript: 412 / Related Interrogated SNP: hCV8717873 / Related Interrogated SNP: hCV8718961 / Related Interrogated SNP: hCV1376266 / Related Interrogated SNP: hCV1376342 / Related Interrogated SNP: hCV15973734 / SNP Source: dbSNP; HapMap; ABI_Val; HGBASE / Population(Allele,Count): Caucasian (T,42|C,184) / SNP Type: UTR5 //

Gene Number: 53 / Gene Symbol STK11IP - 114790 / Gene Name: serine/threonine kinase 11 interacting protein / Public Transcript Accession: NM_052902 / Public Protein Accession: NP_443134 / Chromosome: 2 / OMIM NUMBER: 607172 / OMIM Information: // Transcript Sequence (SEQ ID NO: 75): // Protein Sequence (SEQ ID NO: 159): // SNP Information //
Context (SEQ ID NO: 320):
CTAACTGTGGTAGTGACCACGTGGTTCTCCTCGCTGTGTCTCGGGGAACCCCCAACAGGGAGCGGAAACAGGGAGAGCAGTCT
CTGGCTCCTTCTCCGTC
Y
GCCAGCCCTGTCTGCCACCCTCCTGGCCATGGTGACCACCTTGACAGGGCCAAGAACAGCCCACCTCAGGCACCGAGCACCCG
TGACCATGGTAGTTGGA / Celera SNP ID: hCV2915511 / Public SNP ID: rs627530 / SNP Chromosome Position: 220476443 / SNP in Transcript Sequence SEQ ID NO: 75 / SNP Position Transcript: 2299 / SNP Source: Applera / Population(Allele,Count): Caucasian (C,1|T,39) African American (C,8|T,24) total (C,9|T,63) / SNP Type: Missense Mutation / Protein Coding: SEQ ID NO: 159, at position 752,(S,TCT) (F,TTT) / SNP Source: dbSNP; HapMap; ABI_Val; HGBASE / Population(Allele,Count): Caucasian (C,5|T,219) / SNP Type: Missense Mutation / Protein Coding: SEQ ID NO: 159, at position 752,(S,TCT) (F,TTT) //
Context (SEQ ID NO: 321): GATAGGCGCCGGGCAGCTGAGCTGGTAGGAGGACCAGACGGGGAT
K
TTCGGCTCCGCCCCCCAGCGTCCCGTGGCCATGACGACCGCTCAGAGGGACTCCCTGTTGTGGAAGCTCGCGGGGTTGCTGCG
GGAGTCCGGGGATGTGG / Celera SNP ID: hCV2414147 / Public SNP ID: rs681747 / SNP Chromosome Position: 220462640 / SNP in Transcript Sequence SEQ ID NO: 75 / SNP Position Transcript: 46 / Related Interrogated SNP: hCV2915511 / SNP Source: Applera / Population(Allele,Count): Caucasian (G,0|T,10) African American (G,14|T,20) total (G,14|T,30) / SNP Type: Missense Mutation / Protein Coding: SEQ ID NO: 159, at position 1,(M,ATG) (R,AGG) / SNP Source: Applera / Population(Allele,Count): Caucasian (G,1|T,39) African American (G,15|T,21) total (G,16|T,60) / SNP Type: Missense Mutation / Protein Coding: SEQ ID NO: 159, at position 1,(M,ATG) (R,AGG) / SNP Source: dbSNP; HapMap; ABI_Val; HGBASE / Population(Allele,Count): Caucasian (G,6|T,220) / SNP Type: Missense Mutation / Protein Coding: SEQ ID NO: 159, at position 1,(M,ATG) (R,AGG) //

Gene Number: 54 / Gene Symbol TTC8 - 123016 / Gene Name: tetratricopeptide repeat domain 8 / Public Transcript Accession: NM_144596 /

Public Protein Accession: NP_653197 / Chromosome: 14 / OMIM NUMBER: 608132 / OMIM Information: Bardet-Biedl syndrome 8, 209900 (3) // Transcript Sequence (SEQ ID NO: 76): // Protein Sequence (SEQ ID NO: 160): // SNP Information // Context (SEQ ID NO: 322):
TAAGGGTGACACAGAATGTGTAATGCAAATTTCATAGTAATAGTAACTTTATAAAATAATATTATAAAATACAGGATTTAAAC
CTTTCTAAATAGATCCT
R
AAACTGTCTCTCACATTATATAGTAGATGTTTGTTTATAATGTTTACAAAACATTTTGGTGAATTTCCTCAATGTTTTATAAA
TGTACATTTTTTTAAGTC / Celera SNP ID: hCV7583094 / Public SNP ID: rs1048190 / SNP Chromosome Position: 89343992 / SNP in Transcript Sequence SEQ ID NO: 76 / SNP Position Transcript: 1860 / Related Interrogated SNP: hCV16182835 / SNP Source: dbSNP; HapMap; HGBASE / Population(Allele,Count): Caucasian (G,84|A,32) / SNP Type: UTR3 //

Gene Number: 54 / Gene Symbol TTC8 - 123016 / Gene Name: tetratricopeptide repeat domain 8 / Public Transcript Accession: NM_198309 / Public Protein Accession: NP_938051 / Chromosome: 14 / OMIM NUMBER: 608132 / OMIM Information: Bardet-Biedl syndrome 8, 209900 (3) // Transcript Sequence (SEQ ID NO: 77): // Protein Sequence (SEQ ID NO: 161): // SNP Information // Context (SEQ ID NO: 323):
TAAGGGTGACACAGAATGTGTAATGCAAATTTCATAGTAATAGTAACTTTATAAAATAATATTATAAAATACAGGATTTAAAC
CTTTCTAAATAGATCCT
R
AAACTGTCTCTCACATTATATAGTAGATGTTTGTTTATAATGTTTACAAAACATTTTGGTGAATTTCCTCAATGTTTTATAAA
TGTACATTTTTTTAAGTC / Celera SNP ID: hCV7583094 / Public SNP ID: rs1048190 / SNP Chromosome Position: 89343992 / SNP in Transcript Sequence SEQ ID NO: 77 / SNP Position Transcript: 1830 / Related Interrogated SNP: hCV16182835 / SNP Source: dbSNP; HapMap; HGBASE / Population(Allele,Count): Caucasian (G,84|A,32) / SNP Type: UTR3 //

Gene Number: 54 / Gene Symbol TTC8 - 123016 / Gene Name: tetratricopeptide repeat domain 8 / Public Transcript Accession: NM_198310 / Public Protein Accession: NP_938052 / Chromosome: 14 / OMIM NUMBER: 608132 / OMIM Information: Bardet-Biedl syndrome 8, 209900 (3) // Transcript Sequence (SEQ ID NO: 78): // Protein Sequence (SEQ ID NO: 162): // SNP Information // Context (SEQ ID NO: 324):
TAAGGGTGACACAGAATGTGTAATGCAAATTTCATAGTAATAGTAACTTTATAAAATAATATTATAAAATACAGGATTTAAAC
CTTTCTAAATAGATCCT
R
AAACTGTCTCTCACATTATATAGTAGATGTTTGTTTATAATGTTTACAAAACATTTTGGTGAATTTCCTCAATGTTTTATAAA
TGTACATTTTTTTAAGTC / Celera SNP ID: hCV7583094 / Public SNP ID: rs1048190 / SNP Chromosome Position: 89343992 / SNP in Transcript Sequence SEQ ID NO: 78 / SNP Position Transcript: 1740 / Related Interrogated SNP: hCV16182835 / SNP Source: dbSNP; HapMap; HGBASE / Population(Allele,Count): Caucasian (G,84|A,32) / SNP Type: UTR3 //

Gene Number: 55 / Gene Symbol EML5 - 161436 / Gene Name: echinoderm microtubule associated protein like 5 / Public Transcript Accession: NM_183387 / Public Protein Accession: NP_899243 / Chromosome: 14 / OMIM NUMBER: / OMIM Information: // Transcript Sequence (SEQ ID NO: 79): // Protein Sequence (SEQ ID NO: 163): // SNP Information // Context (SEQ ID NO: 325):
CATGCTGCAGGAATTTTTAGCATGAATGCTTGTGAAGAAGGCTTTGCTACTGGTGGCAGAGATGGTTGTATTCGTCTTTGGGA
TTTAACTTTTAAACCAA
R
TACTGTGATTGATCTCAGGGAAACAGACCAGGGATACAAAGGTTTGTCTGTAAGGAGTGTGTGTTGGCGAGGTGACCACATTC
TAGTTGGAACACAGGAC / Celera SNP ID: hDV70918505 / Public SNP ID: rs17188228 / SNP Chromosome Position: 89205265 / SNP in Transcript Sequence SEQ ID NO: 79 / SNP Position Transcript: 1055 / Related Interrogated SNP: hCV16182835 / SNP Source: dbSNP; HapMap / Population(Allele,Count): Gaucasian (A,89|G,27) / SNP Type: Missense Mutation / Protein Coding: SEQ ID NO: 163, at position 269,(I,ATT) (V,GTT) //

Gene Number: 56 / Gene Symbol                      TTC24 - 164118 / Gene Name:
tetratricopeptide repeat domain 24 / Public Transcript Accession: NM_001105669 /
Public Protein Accession:   NP_001099139 / Chromosome:   1 / OMIM NUMBER: / OMIM
Information: // Transcript Sequence (SEQ ID NO: 80): // Protein Sequence   (SEQ
ID NO: 164): // SNP Information //
Context              (SEQ ID NO: 326):
GGGCTGCGGCAGGATGTATGCTGAAGAGTGGGCGGCATCGGGTGGGGGAAGTTGTGCAGGTGCTGGAGAAAAGCCGGAGGCTT
GCCGAGAGGAGCACTGA
R
AGGCGACTGCTGGGGCACCTCTATAACGATCTAGGCCTGGGCTACTCCCAGCTCCAGCTGTTCCCGCTGGCCGTGGAGGCCTT
CCTGCAGGCCCTGCCCC / Celera SNP ID:    hCV25989540 / Public SNP ID:    rs6682716 /
SNP Chromosome Position: 156551848 / SNP in Transcript Sequence    SEQ ID NO: 80 /
SNP Position Transcript: 729 / Related Interrogated SNP:   hCV9102827 / SNP
Source:   Applera / Population(Allele,Count):   Caucasian (A,10|G,30)  African
American (A,24|G,4)  total (A,34|G,34) / SNP Type:   Missense Mutation / Protein
Coding:   SEQ ID NO: 164, at position 231,(E,GAG) (G,GGG) / SNP Source:   Applera
/ Population(Allele,Count):   Caucasian (A,11|G,27)  African American (A,14|G,2)
total (A,25|G,29) / SNP Type:   Missense Mutation / Protein Coding:   SEQ ID NO:
164, at position 231,(E,GAG) (G,GGG) / SNP Source:   dbSNP; HapMap /
Population(Allele,Count):   Caucasian (A,56|G,170) / SNP Type:   Missense Mutation
/ Protein Coding:   SEQ ID NO: 164, at position 231,(E,GAG) (G,GGG) //

Gene Number: 57 / Gene Symbol                      ASPM - 259266 / Gene Name: asp
(abnormal spindle) homolog, microcephaly associated (Drosophila) / Public
Transcript Accession: NM_018136 / Public Protein Accession:   NP_060606 /
Chromosome:  1 / OMIM NUMBER:  605481 / OMIM Information:  Microcephaly, primary
autosomal recessive, 5, 608716 (3) // Transcript Sequence (SEQ ID NO: 81): //
Protein Sequence    (SEQ ID NO: 165): // SNP Information //
Context            (SEQ ID NO: 327):
TACTTTCAGGATGTACAGAACATATATTACATTTCAGACTTGGAAACATGCTTCAATTCTAATTCAGCAACATTATCGAACAT
ATAGAGCTGCAAAATTA
R
AAAGAGAAAATTATATCAGACAATGGCATTCTGCTGTGGTTATTCAGGCTGCATATAAAGGAATGAAAGCAAGACAACTTTTA
AGGGAAAAACACAAAGC / Celera SNP ID:    hCV2759703 / Public SNP ID:    rs1412640 /
SNP Chromosome Position: 197070815 / SNP in Transcript Sequence    SEQ ID NO: 81 /
SNP Position Transcript: 7824 / Related Interrogated SNP:   hCV2532034 / SNP
Source:   Applera / Population(Allele,Count):   Gaucasian (G,35|A,3)  African
American (G,25|A,11)  total (G,60|A,14) / SNP Type:   Silent Mutation / Protein
Coding:   SEQ ID NO: 165, at position 2522,(L,TTA) (L,TTG) / SNP Source:   dbSNP;
Celera; ABI_Val; HGBASE / Population(Allele,Count):   Gaucasian (A,20|G,206) / SNP
Type:   Silent Mutation / Protein Coding:   SEQ ID NO: 165, at position
2522,(L,TTA) (L,TTG) //
Context            (SEQ ID NO: 328):
TCATGCATCAGAAAATAGGGAACTATTAAATGTACACAGTGCCAACGTTTCAAAAGTTTCTTTTAATGAGAAAGCTGTAACTG
AAACTTCCTTTAATTCC
Y
TAAATGTTAATGGCCAAAGAGGAGAGAATAGTAAACTTAGTCTTACCCCCAACTGTTCTTCAACTTTGAACATTACACAAAGC
CAAATACATTTTCTAAG / Celera SNP ID:    hCV2759679 / Public SNP ID:    rs6677082 /
SNP Chromosome Position: 197112533 / SNP in Transcript Sequence    SEQ ID NO: 81 /
SNP Position Transcript: 1107 / Related Interrogated SNP:   hCV2532034 / SNP
Source:   dbSNP; Celera; HapMap / Population(Allele,Count):   Caucasian
(C,20|T,206) / SNP Type:   Silent Mutation / Protein Coding:   SEQ ID NO: 165, at
position 283,(S,TCC) (S,TCT) //
Context            (SEQ ID NO: 329):
TATATGTCAGCGTACTACTCAAACTGTGGAATGTACGCAAACTGGTTCAGTGGTATTAAATTCATCATCTGAATCTGATGACA
GTTCTCTGGATATGTCT / W /
TTAAAGCATTTGATCATGAAAATACTTCAGAGCTATACAAAGAGCTCCTAGAAAATGAAAAGAAAAATTTTCACTTGGTTAGG
TCTGCAGTTAGAGACCT / Celera SNP ID:    hCV2759691 / Public SNP ID:    rs4915337 /
SNP Chromosome Position: 197091537 / SNP in Transcript Sequence    SEQ ID NO: 81 /
SNP Position Transcript: 3837 / Related Interrogated SNP:   hCV2532034 / SNP
Source:   dbSNP; Celera; HapMap; ABI_Val; HGBASE / Population(Allele,Count):
Caucasian (T,8|A,110) / SNP Type:   Silent Mutation / Protein Coding:   SEQ ID NO:
165, at position 1193,(S,TCT) (S,TCA) //

Context (SEQ ID NO: 330):
CGCTTGGCATTCCTTATTAGTAAATGTAAACATTTTCAGTATGTATAGTGTAAAGAAATATTAAAGCCAATCATGAGTACGTA
AAGTGATTTTTGCTCTC
Y

GTGTACAACTTTTAAAATCTGACTTTGTTTTAAAAAAAACATAAACTGTTCATTACATTCTTCATTTTTATCATTTATAGTTTT
ATGCATGTAATAAACTA / Celera SNP ID: hCV8356417 / Public SNP ID: rs12677 /
SNP Chromosome Position: 197053373 / SNP in Transcript Sequence SEQ ID NO: 81 /
SNP Position Transcript: 10772 / Related Interrogated SNP: hCV2532034 / SNP
Source: dbSNP; Celera; HapMap; HGBASE / Population(Allele,Count): Caucasian
(C,20|T,206) / SNP Type: UTR3 //

Gene Number: 58 / Gene Symbol CYP4V2 - 285440 / Gene Name:
cytochrome P450, family 4, subfamily V, polypeptide 2 / Public Transcript
Accession: NM_207352 / Public Protein Accession: NP_997235 / Chromosome: 4 /
OMIM NUMBER: 608614 / OMIM Information: Bietti crystalline corneoretinal
dystrophy, 210370 (3) // Transcript Sequence (SEQ ID NO: 82): // Protein Sequence
(SEQ ID NO: 166): // SNP Information //
Context (SEQ ID NO: 331):
ATGAGTGAGATGATATTTCGAAGAATAAAGATGCCCTGGCTTTGGCTTGATCTCTGGTACCTTATGTTTAAAGAAGGATGGGA
ACACAAAAAGAGCCTTC
M

GATCCTACATACTTTTACCAACAGTGTCATCGCTGAACGGGCCAATGAAATGAACGCCAATGAAGACTGTAGAGGTGATGGCA
GGGGCTCTGCCCCCTCC / Celera SNP ID: hCV25990131 / Public SNP ID: rs13146272
/ SNP Chromosome Position: 187120211 / SNP in Transcript Sequence SEQ ID NO: 82
/ SNP Position Transcript: 1080 / SNP Source: Applera /
Population(Allele,Count): Caucasian (A,22|C,16) African American (A,7|C,11)
total (A,29|C,27) / SNP Type: Missense Mutation / Protein Coding: SEQ ID NO:
166, at position 259,(Q,CAG) (K,AAG) / SNP Source: dbSNP; /
Population(Allele,Count): Caucasian (C,88|A,130) / SNP Type: Missense Mutation
/ Protein Coding: SEQ ID NO: 166, at position 259,(Q,CAG) (K,AAG) //
Context (SEQ ID NO: 332):
AGTTATGACAAGAATAATCATTATAGTACTTTTCAGATTTTATAACCTGGAGCAGATTATTTTAAGTTGATTAGTAGGTTCTG
TTACAGTTTTTCTTTTG
W

TCGTGCACTTATAGTCTTCATTTAATTCCTCATAGAATCCCAGTCACCTTTATATATCATATTATTGGAAGAGATTCATCTTC
ATAATCTCCAGTTTTTT / Celera SNP ID: hCV3230113 / Public SNP ID: rs1053094 /
SNP Chromosome Position: 187133031 / SNP in Transcript Sequence SEQ ID NO: 82 /
SNP Position Transcript: 3118 / SNP Source: dbSNP; Celera; HapMap; HGBASE; /
Population(Allele,Count): Caucasian (A,60|T,60) / SNP Type: UTR3 //
Context (SEQ ID NO: 333):
CTGGCTTTGGCTTGATCTCTGGTACCTTATGTTTAAAGAAGGATGGGAACACAAAAAGAGCCTTCAGATCCTACATACTTTTA
CCAACAGTGTCATCGCT
K

AACGGGCCAATGAAATGAACGCCAATGAAGACTGTAGAGGTGATGGCAGGGGCTCTGCCCCCTCCAAAAATAAACGCAGGGCC
TTTCTTGACTTGCTTTT / Celera SNP ID: hCV3230097 / Public SNP ID: rs3736455 /
SNP Chromosome Position: 187122319 / SNP in Transcript Sequence SEQ ID NO: 82 /
SNP Position Transcript: 1115 / Related Interrogated SNP: hCV11786258 / Related
Interrogated SNP: hCV12066124 / Related Interrogated SNP: hCV15968043 / Related
Interrogated SNP: hCV25990131 / Related Interrogated SNP: hCV27477533 / Related
Interrogated SNP: hCV27902808 / Related Interrogated SNP: hCV3230096 / Related
Interrogated SNP: hCV8241630 / Related Interrogated SNP: hCV3230113 / Related
Interrogated SNP: hCV3230038 / Related Interrogated SNP: hCV27474895 / Related
Interrogated SNP: hCV25474413 / Related Interrogated SNP: hCV22272267 / Related
Interrogated SNP: hCV32291301 / SNP Source: Applera / Population(Allele,Count):
Caucasian (G,24|T,16) African American (G,7|T,9) total (G,31|T,25) / SNP Type:
Silent Rare Codon / Protein Coding: SEQ ID NO: 166, at position 270,(A,GCT)
(A,GCG) / SNP Source: dbSNP; Celera; HapMap; HGBASE; / Population(Allele,Count):
Caucasian (T,81|G,145) / SNP Type: Silent Rare Codon / Protein Coding: SEQ
ID NO: 166, at position 270,(A,GCT) (A,GCG) //
Context (SEQ ID NO: 334):
ATTTAGTAACCCAGTAATATCTCACTTAGTTTAGGGTTAGATCTTTAGTTAATTCAACCTTATAGATCATACTTATGAAGGTG
ATAACTGACACGTGTTC
M

CTGAATTTTAATTTGATAGGCAATACATCTACCCACTCCATTATTTTTTAAAACTTCATTTAATAGTTTAAACAAGATTGGTT
TTGTTTTCAATTTTTAT / Celera SNP ID: hCV29053261 / Public SNP ID: rs6842047 /
SNP Chromosome Position: 187133576 / SNP in Transcript Sequence SEQ ID NO: 82 /
SNP Position Transcript: 3663 / Related Interrogated SNP: hCV15793897 / Related
Interrogated SNP: hCV12066124 / Related Interrogated SNP: hCV25474413 / Related
Interrogated SNP: hCV27474895 / Related Interrogated SNP: hCV3230113 / Related
Interrogated SNP: hCV8241630 / Related Interrogated SNP: hCV25990131 / Related
Interrogated SNP: hCV27477533 / Related Interrogated SNP: hCV3230038 / SNP
Source: dbSNP; HapMap; / Population(Allele,Count): Caucasian (A,20|C,206) / SNP
Type: UTR3 //

Gene Number: 59 / Gene Symbol CYP27C1 - 339761 / Gene Name:
cytochrome P450, family 27, subfamily C, polypeptide 1 / Public Transcript
Accession: NM_001001665 / Public Protein Accession: NP_001001665 / Chromosome:
2 / OMIM NUMBER: / OMIM Information: // Transcript Sequence (SEQ ID NO: 83): //
Protein Sequence (SEQ ID NO: 167): // SNP Information //
Context (SEQ ID NO: 335):
CTTCCTTAGCCAGGCTCTGACGCTGCAGGAGATCTACGCCAACGTGACTGAGATGCTGCTGGCCGGCGTCGACACGACGTCCT
TCACCTTGTCTTGGACT
K

TGTACCTCCTGGCAAGGCACCCAGAAGTGCAGCAGACGGTGTACCGGGAGATTGTGAAGAATTTAGGGGAAAGGCATGTTCCA
ACTGCAGCTGATGTCCC / Celera SNP ID: hCV1441133 / Public SNP ID: rs7568070 /
SNP Chromosome Position: 127953054 / SNP in Transcript Sequence SEQ ID NO: 83 /
SNP Position Transcript: 708 / Related Interrogated SNP: hCV1841975 / Related
Interrogated SNP: hCV1841983 / SNP Source: Applera / Population(Allele,Count):
Gaucasian (T,21|G,9) Tfrican Tmerican (T,16|G,18) total (T,37|G,27) / SNP Type:
Silent Rare Codon / Protein Coding: SEQ ID NO: 167, at position 192,(T,ACT)
(T,ACG) / SNP Source: dbSNP; Celera; HapMap; ABI_Val / Population(Allele,Count):
Gaucasian (T,174|G,52) / SNP Type: Silent Rare Codon / Protein Coding: SEQ
ID NO: 167, at position 192,(T,ACT) (T,ACG) //

Gene Number: 60 / Gene Symbol NCKAP5 - 344148 / Gene Name:
NCK-associated protein 5 / Public Transcript Accession: NM_207363 / Public Protein
Accession: NP_997246 / Chromosome: 2 / OMIM NUMBER: / OMIM Information: //
Transcript Sequence (SEQ ID NO: 84): // Protein Sequence (SEQ ID NO: 168): //
SNP Information //
Context (SEQ ID NO: 336):
GGCCGCATGGCTCTCAACCTCCAGCTTTCAGACACTGATGACAATGAAACGTTTGATGAGCTGCACATAGAGAGCAGTGATGA
GAAAAGTCCTTCAGACG
R
GTCATTGGCTGCCGACACCGATAAGTCCGTGGAGAACCTGGATGTCCTTGTGGGGTTTGGAAAATCTCTATGTGGGTCTCCTG
AAGAGGAGGAAAAACAA / Celera SNP ID: hCV25748719 / Public SNP ID: rs61995744
/ SNP Chromosome Position: 133542574 / SNP in Transcript Sequence SEQ ID NO: 84
/ SNP Position Transcript: 2185 / SNP Source: Applera /
Population(Allele,Count): Gaucasian (G,35|A,5) African American (G,33|A,5)
total (G,68|A,10) / SNP Type: Missense Mutation / Protein Coding: SEQ ID NO:
168, at position 604,(V,GTG) (M,ATG) / SNP Source: dbSNP; Applera /
Population(Allele,Count): Gaucasian (A,28|G,92) / SNP Type: Missense Mutation /
Protein Coding: SEQ ID NO: 168, at position 604,(V,GTG) (M,ATG) //
Context (SEQ ID NO: 337):
GACATAGGATTACAGACTCCCAGGATCTCTCCTTCAACCCATGAGCCACTGGAAATGACGTCCTCCAAAAGTGTATCTCCAGG
GAGAAAAGGACAATTGA
W
TGATAGCGCCTCCACACCCCCCAAGCCTTCCTTCTTAGGGGTAAATGAGTCACCATCATCTCAGGTCAGCAGTTCCTCATCAT
CCTCATCACCCGCCAAA / Celera SNP ID: hCV25749343 / Public SNP ID: rs16841277
/ SNP Chromosome Position: 133541107 / SNP in Transcript Sequence SEQ ID NO: 84
/ SNP Position Transcript: 3652 / Related Interrogated SNP: hCV25748719 / SNP
Source: Applera / Population(Allele,Count): Caucasian (T,5|A,31) Tfrican
Tmerican (T,5|A,33) total (T,10|A,64) / SNP Type: Missense Mutation / Protein
Coding: SEQ ID NO: 168, at position 1093,(N,AAT) (Y,TAT) / SNP Source: dbSNP;
HapMap / Population(Allele,Count): Caucasian (A,92|T,24) / SNP Type: Missense
Mutation / Protein Coding: SEQ ID NO: 168, at position 1093,(N,AAT) (Y,TAT)

Table2_CD0000290RD_reformatted.txt

Gene Number: 1 / Gene Symbol: ABO - 28 / Gene Name: ABO blood group (transferase A, alpha 1-3-N-acetylgalactosaminyltransf / erase; transferase B, alpha 1-3-galactosyltransferase) // Chromosome: 9 / OMIM NUMBER: 110300 / OMIM Information: [Blood group, ABO system] (3) // Genomic Sequence (SEQ ID NO: 338): // SNP Information //
Context                (SEQ ID NO: 501): /
GGCATTTTGGTTTTCCTGGAAAACAGGAGTAGAAAAATCACATCACTCACCCTCCTTCTCTCACCTGCCCCACTTTACCTGTC
CCGCCTTGTAATTCAGG
Y
ATCACACCCTGCGACGGGCCTTCATCTGCCAAGATGGTCCCTCTGCCCCACAGGTGATGCTCTGAGCCCCACCTGAGCATCTC
CAAACCCAGTCCAGATC / Celera SNP ID: hCV25610857 / Public SNP ID: rs8176693 /
SNP Chromosome Position: 136137657 / SNP in Genomic Sequence: SEQ ID NO: 338 /
 SNP Position Genomic: 69178 / SNP Source: Applera /
Population(Allele,Count): Caucasian (C,36|T,4) African American (C,30|T,8)
total (C,66|T,12) / SNP Type: INTRON / SNP Source: dbSNP; HapMap /
Population(Allele,Count): Caucasian (C,209|T,17) / SNP Type: INTRON //
Context                (SEQ ID NO: 502): /
TGGGCACCTGCTGGAGCTCCAGAGCCCCAGCTCCCTCACCCCCCGCCACCAGTGCCTTGATCAGTGCAGTCCCTGAGGCCACG
TCAGGGAGTGTTTCCAG
R
CCGTGTCCAGAGATGCCCCAGTAGAGGTGACAGGTGACTTCACTCTTGGTTCTGGGACCTTCTGTGTCTGGAAGCGACTAACA
GCTTGGGACCCAGAATT / Celera SNP ID: hCV9326428 / Public SNP ID: rs687289 /
SNP Chromosome Position: 136137106 / SNP in Genomic Sequence: SEQ ID NO: 338 /
 SNP Position Genomic: 68627 / SNP Source: dbSNP; HapMap; ABI_Val; HGBASE /
Population(Allele,Count): Caucasian (G,77|A,43) / SNP Type: INTRON /
Context                (SEQ ID NO: 503): /
TGACTGAACCACAATATCTCAGGGACCCCATAAATATATACACCTACTATGAACCCATAAAATGAAAAACAAAATACTTCTTA
GAAAACAAATATTTTCT
Y
TCAGTCGGTGGCTTATTTATTCATTTTTCCTTAATTTTTCTTTTGAAGGGAAGTGTTAATTTTTATAAACTCTAATTTATCCT
TATAAAAATGAAACCAT / Celera SNP ID: hCV15887091 / Public SNP ID: rs2519093 /
SNP Chromosome Position: 136141870 / SNP in Genomic Sequence: SEQ ID NO: 338 /
 SNP Position Genomic: 73391 / SNP Source: dbSNP; HapMap; HGBASE /
Population(Allele,Count): Caucasian (C,108|T,-) / SNP Type: INTRON /
Context                (SEQ ID NO: 504): /
CACACCAGGTAATCCACCTCGCTGAGGAAGCGCCGCTCGCAGAAGTCACTGATCATCTCCATGCGGCGCATGGACACGTCCTG
CCAGCGCTTGTAGGCGC
S
CACCTCCAGCACTGACAGCTGCCGACCGGTCCCCAGCGTCACGCGGGGCACCGCGGCCGGCTGGTCGGTGAAGACATAGTAGT
GGACACGGTGGCCCACC / Celera SNP ID: hCV27859399 / Public SNP ID: rs7853989 /
SNP Chromosome Position: 136131592 / SNP in Genomic Sequence: SEQ ID NO: 338 /
 SNP Position Genomic: 63113 / SNP Source: HGMD; dbSNP; HapMap /
Population(Allele,Count): Caucasian (G,108|C,12) / SNP Type: TRANSCRIPTION
FACTOR BINDING SITE;MICRORNA;UTR3;INTRON /
Context                (SEQ ID NO: 505): /
AACACAGTTAACCCAATGGTGGTGTTCTGGAGCCTGAACTGCTCGTTGAGGATGTCGATGTTGAATGTGCCCTCCCAGACAAT
GGGAGCCAGCCAAGGGG
K
ACCACGAGGACATCCTTCCTACTGCACATGGAGAGAGGCGTGCGGTCACATGGAGCTGGCAGGGTGCCACCCACATGCGCCTC
TGGCACACGGCCGCCCC / Celera SNP ID: hDV77258202 / Public SNP ID: rs8176719 /
SNP Chromosome Position: 136132908 / SNP in Genomic Sequence: SEQ ID NO: 338 /
 SNP Position Genomic: 64429 / SNP Source: CDX; dbSNP /
Population(Allele,Count): Caucasian (T,4|G,18) / SNP Type: FRAME SHIFT
INDEL;INTRONIC INDEL /
Context                (SEQ ID NO: 506): /
CCCCCGAGTACTTGTGGGACCAGCAGCTGCTGGGCTGGCCCGCCGTCCTGAGGAAGCTGAGGTTCACTGCGGTGCCCAAGAAC
CACCAGGCGGTCCGGAA / C /
CCGTGAGCGGCTGCCAGGGGCTCTGGGAGGGCTGCCGGCAGCCCCGTCCCCCTCCCGCCCTTGGTTTTAGCAGAACGGGTAAA
CTCTGTTTCCTTTGTCC / Celera SNP ID: hDV71075946 / Public SNP ID: rs8176750 /
SNP Chromosome Position: 136131059 / SNP in Genomic Sequence: SEQ ID NO: 338 /
 SNP Position Genomic: 62580 / SNP Source: CDX;dbSNP /

Population(Allele,Count): no_pop (C,-|,-) / SNP Type: UTR3 INDEL;INTRONIC INDEL /
Context (SEQ ID NO: 507): /
GGGACGGGGCCTAGGCTTCAGTTACTCACAACAGGACGGACAAAGGAAACAGAGTTTACCCGTTCTGCTAAAACCAAGGGCGG
GAGGGGGACGGGGCTGC
Y
GGCAGCCCTCCCAGAGCCCCTGGCAGCCGCTCACGGGTTCCGGACCGCCTGGTGGTTCTTGGGCACCGCAGTGAACCTCAGCT
TCCTCAGGACGGCGGGC / Celera SNP ID: hCV25610771 / Public SNP ID: rs8176751 /
SNP Chromosome Position: 136131022 / SNP in Genomic Sequence: SEQ ID NO: 338 /
SNP Position Genomic: 62543 / Related Interrogated SNP: hCV25610857 /
Related Interrogated SNP: hCV27859399 / SNP Source: Applera /
Population(Allele,Count): Caucasian (C,16|T,2) African American (C,0|T,4) total
(C,16|T,6) / SNP Type: UTR3;INTRON / SNP Source: Applera /
Population(Allele,Count): Caucasian (C,26|T,4) African American (C,14|T,0)
total (C,40|T,4) / SNP Type: UTR3;INTRON / SNP Source: dbSNP; Applera /
Population(Allele,Count): Caucasian (C,203|T,17) / SNP Type: UTR3;INTRON /
Context (SEQ ID NO: 508): /
ACGGCCTCGATGCCGTTGGCCTGGTCGACCATCATGGCCTGGTGGCAGGCCCTGGTGAGCCGCTGCACCTCTTGCACCGACCC
CCCGAAGAACCCCCCCA
K
GTAGTAGAAATCGCCCTCGTCCTTGGGGATGTAGGCCTGGGACTGGGGCCGGCGCTCGTAGGTGAAGGCCTCCCGGCTGCTTC
CGTAGAAGCCGGGGTGC / Celera SNP ID: hCV25610772 / Public SNP ID: rs8176746 /
SNP Chromosome Position: 136131322 / SNP in Genomic Sequence: SEQ ID NO: 338 /
SNP Position Genomic: 62843 / Related Interrogated SNP: hCV25610857 /
Related Interrogated SNP: hCV27859399 / SNP Source: Applera /
Population(Allele,Count): Caucasian (G,32|T,4) African American (G,25|T,13)
total (G,57|T,17) / SNP Type: MICRORNA;UTR3;INTRON / SNP Source: Applera /
Population(Allele,Count): Caucasian (G,32|T,6) African American (G,25|T,11)
total (G,57|T,17) / SNP Type: MICRORNA;UTR3;INTRON / SNP Source: HGMD;
dbSNP; Applera / Population(Allele,Count): Caucasian (G,209|T,17) / SNP Type:
MICRORNA;UTR3;INTRON /
Context (SEQ ID NO: 509): /
GTGCCACACGGCCTCGATGCCGTTGGCCTGGTCGACCATCATGGCCTGGTGGCAGGCCCTGGTGAGCCGCTGCACCTCTTGCA
CCGACCCCCCGAAGAAC
S
CCCCCAGGTAGTAGAAATCGCCCTCGTCCTTGGGGATGTAGGCCTGGGACTGGGGCCGGCGCTCGTAGGTGAAGGCCTCCCGG
CTGCTTCCGTAGAAGCC / Celera SNP ID: hCV25610773 / Public SNP ID: rs8176747 /
SNP Chromosome Position: 136131315 / SNP in Genomic Sequence: SEQ ID NO: 338 /
SNP Position Genomic: 62836 / Related Interrogated SNP: hCV25610857 /
Related Interrogated SNP: hCV27859399 / SNP Source: Applera /
Population(Allele,Count): Caucasian (C,36|G,4) African American (C,25|G,13)
total (C,61|G,17) / SNP Type: MICRORNA;UTR3;INTRON / SNP Source: Applera /
Population(Allele,Count): Caucasian (C,36|G,4) African American (C,28|G,10)
total (C,64|G,14) / SNP Type: MICRORNA;UTR3;INTRON / SNP Source: HGMD;
dbSNP; HapMap / Population(Allele,Count): Caucasian (C,98|G,14) / SNP Type:
MICRORNA;UTR3;INTRON /
Context (SEQ ID NO: 510): /
CCGCAGTGAACCTCAGCTTCCTCAGGACGGCGGGCCAGCCCAGCAGCTGCTGGTCCCACAAGTACTCGGGGGAGAGCACCTTG
GTGGGTTTGTGGCGCAG
Y
AGGTACTTGTTCAGGTGGCTCTCGTCGTGCCACACGGCCTCGATGCCGTTGGCCTGGTCGACCATCATGGCCTGGTGGCAGGC
CCTGGTGAGCCGCTGCA / Celera SNP ID: hCV25610781 / Public SNP ID: rs8176749 /
SNP Chromosome Position: 136131188 / SNP in Genomic Sequence: SEQ ID NO: 338 /
SNP Position Genomic: 62709 / Related Interrogated SNP: hCV25610857 /
Related Interrogated SNP: hCV27859399 / SNP Source: Applera /
Population(Allele,Count): Caucasian (C,36|T,4) African American (C,25|T,13)
total (C,61|T,17) / SNP Type: UTR3;INTRON / SNP Source: Applera /
Population(Allele,Count): Caucasian (C,36|T,4) African American (C,28|T,10)
total (C,64|T,14) / SNP Type: UTR3;INTRON / SNP Source: dbSNP; HapMap /
Population(Allele,Count): Caucasian (C,209|T,17) / SNP Type: UTR3;INTRON /
Context (SEQ ID NO: 511): /
CCCCCCAGGTAGTAGAAATCGCCCTCGTCCTTGGGGATGTAGGCCTGGGACTGGGGCCGGCGCTCGTAGGTGAAGGCCTCCCG
GCTGCTTCCGTAGAAGC

Y
GGGGTGCAGGGTGCCGAACAGCGGAGTCAGGATCTCCACGCCCACGTGGTCGCGGAACTCCATGTCCACGTCCACGCACACCA
GGTAATCCACCTCGCTG / Celera SNP ID: hCV25610791 / Public SNP ID: rs8176743 /
SNP Chromosome Position: 136131415 / SNP in Genomic Sequence: SEQ ID NO: 338 /
SNP Position Genomic: 62936 / Related Interrogated SNP: hCV25610857 /
Related Interrogated SNP: hCV27859399 / SNP Source: Applera /
Population(Allele,Count): Caucasian (C,36|T,4) African American (C,27|T,9)
total (C,63|T,13) / SNP Type: MICRORNA;UTR3;INTRON / SNP Source: HGMD;
dbSNP; Applera / Population(Allele,Count): Caucasian (C,205|T,17) / SNP Type:
MICRORNA;UTR3;INTRON /
Context (SEQ ID NO: 512): /
ACCCCACCGAGCTTCAGGATGCCCAGGCTGTTTCCCTCCAAGGCAGGGGGTGACTCTTCCCAACACCCGAAACGTGGATGGGG
CAATGGGCACCAGGTGG
R
TCTGGGGAGGGGAGCGAAAGTGGCCTGAGGGGCCCTGCAGTGGGCAACACTCACATGAAGGTGAACGTGGCTTCCAACTTCCC
ACCGCCCAGGGCTGTCA / Celera SNP ID: hCV25987572 / Public SNP ID: rs4310274 /
SNP Chromosome Position: 136083801 / SNP in Genomic Sequence: SEQ ID NO: 338 /
SNP Position Genomic: 15322 / Related Interrogated SNP: hCV25610857 /
Related Interrogated SNP: hCV27859399 / SNP Source: dbSNP /
Population(Allele,Count): Caucasian (A,33|G,193) / SNP Type: INTRON /
Context (SEQ ID NO: 513): /
GCCACAGTTACAGAGAGGAGGGGGCAGCAGAAGCCACCATCCCTGGGTGAGACGCAGCCTCTGGAGAAGGAGCTGGGTTTTAC
CGACCTGGCGAGCCCAC
R
AGCCCACGAGCCCACATGAGCTCAGTAAGATGCTGCATGAATGACCTTTCCCATCTACCCTCTGGGAGGACAAGGCTGGCCGC
CACCCCACTCTGTCTTG / Celera SNP ID: hCV3183097 / Public SNP ID: rs8176725 /
SNP Chromosome Position: 136132617 / SNP in Genomic Sequence: SEQ ID NO: 338 /
SNP Position Genomic: 64138 / Related Interrogated SNP: hCV25610857 /
Related Interrogated SNP: hCV27859399 / SNP Source: Applera /
Population(Allele,Count): Caucasian (A,5|G,33) African American (A,6|G,20)
total (A,11|G,53) / SNP Type: MICRORNA;UTR3;INTRON / SNP Source: dbSNP;
Celera; HapMap / Population(Allele,Count): Caucasian (G,107|A,11) / SNP Type:
MICRORNA;UTR3;INTRON /
Context (SEQ ID NO: 514): /
GGAGGGGGCAGCAGAAGCCACCATCCCTGGGTGAGACGCAGCCTCTGGAGAAGGAGCTGGGTTTTACCGACCTGGCGAGCCCA
CGAGCCCACGAGCCCAC
R
TGAGCTCAGTAAGATGCTGCATGAATGACCTTTCCCATCTACCCTCTGGGAGGACAAGGCTGGCCGCCACCCCACTCTGTCTT
GAACACAAGGAGAGACC / Celera SNP ID: hCV3183098 / Public SNP ID: rs2073824 /
SNP Chromosome Position: 136132633 / SNP in Genomic Sequence: SEQ ID NO: 338 /
SNP Position Genomic: 64154 / Related Interrogated SNP: hCV27859399 / SNP
Source: Applera / Population(Allele,Count): Caucasian (A,27|G,11) African
American (A,16|G,12) total (A,43|G,23) // / SNP Type: TRANSCRIPTION FACTOR
BINDING SITE;MICRORNA;UTR3;INTRON / SNP Source: dbSNP; Celera; HapMap; ABI_Val;
HGBASE / Population(Allele,Count): Caucasian (A,137|G,81) / SNP Type:
TRANSCRIPTION FACTOR BINDING SITE;MICRORNA;UTR3;INTRON /
Context (SEQ ID NO: 515): /
ATGAATGACCTTTCCCATCTACCCTCTGGGAGGACAAGGCTGGCCGCCACCCCACTCTGTCTTGAACACAAGGAGAGACCTCA
ATGTCCACAGTCACTCG
M
CACTGCCTGGGTCTCTACCCTCGGCCACCTCACTGACTTACTTCTTGATGGCAAACACAGTTAACCCAATGGTGGTGTTCTGG
AGCCTGAACTGCTCGTT / Celera SNP ID: hCV3183099 / Public SNP ID: rs8176722 /
SNP Chromosome Position: 136132754 / SNP in Genomic Sequence: SEQ ID NO: 338 /
SNP Position Genomic: 64275 / Related Interrogated SNP: hCV25610857 /
Related Interrogated SNP: hCV27859399 / SNP Source: Applera /
Population(Allele,Count): Caucasian (A,4|C,36) African American (A,6|C,22)
total (A,10|C,58) / SNP Type: MICRORNA;UTR3;INTRON / SNP Source: dbSNP;
Celera; HapMap / Population(Allele,Count): Caucasian (C,207|A,19) / SNP Type:
MICRORNA;UTR3;INTRON /
Context (SEQ ID NO: 516): /
CCTCGGCCACCTCACTGACTTACTTCTTGATGGCAAACACAGTTAACCCAATGGTGGTGTTCTGGAGCCTGAACTGCTCGTTG
AGGATGTCGATGTTGAA
Y

GTGCCCTCCCAGACAATGGGAGCCAGCCAAGGGGTACCACGAGGACATCCTTCCTACTGCACATGGAGAGAGGCGTGCGGTCA
CATGGAGCTGGCAGGGT / Celera SNP ID: hCV3183100 / Public SNP ID: rs8176720 /
SNP Chromosome Position: 136132873 / SNP in Genomic Sequence: SEQ ID NO: 338 /
SNP Position Genomic: 64394 / Related Interrogated SNP: hCV25610857 /
Related Interrogated SNP: hCV27859399 / SNP Source: Applera /
Population(Allele,Count): Caucasian (C,12|T,28) African American (C,14|T,16)
total (C,26|T,44) // / SNP Type: MISSENSE MUTATION;INTRON / SNP Source:
dbSNP; Celera; HapMap / Population(Allele,Count): Caucasian (T,142|C,82) / SNP
Type: MISSENSE MUTATION;INTRON /
Context (SEQ ID NO: 517): /
GAGGTGGGCAGGAGGGTGGTCCCTGGGATATTGCTCACGTATGGGCACCTGCTGGAGCTCCAGAGCCCCAGCTCCCTCACCCC
CCGCCACCAGTGCCTTG
R

TCAGTGCAGTCCCTGAGGCCACGTCAGGGAGTGTTTCCAGGCCGTGTCCAGAGATGCCCCAGTAGAGGTGACAGGTGACTTCA
CTCTTGGTTCTGGGACC / Celera SNP ID: hCV9326429 / Public SNP ID: rs687621 /
SNP Chromosome Position: 136137065 / SNP in Genomic Sequence: SEQ ID NO: 338 /
SNP Position Genomic: 68586 / Related Interrogated SNP: hCV25610857 / SNP
Source: Applera / Population(Allele,Count): Caucasian (A,27|G,3) African
American (A,18|G,10) total (A,45|G,13) / SNP Type: INTRON / SNP Source:
dbSNP; HapMap; ABI_Val; HGBASE / Population(Allele,Count): Caucasian (A,76|G,42)
/ SNP Type: INTRON /
Context (SEQ ID NO: 518): /
CTTTCAACCCCTGAGCGGGAGCCCAAGTCACCACCCTCAGCTGACACAGGAGGAAAACGACGCTCAGAAGGTCCAGTGGCCCA
GGCAGCAGCAAAGCTGC
R

ATTTCGAGGCCGACTTGGTTCCAAAGCCCAAGCTTTTAACCGTCACGCTATAGGACACCATGCAGTCCTCCAGATTCGTTATA
TTTTTTATTATTTATTT / Celera SNP ID: hCV153353 / Public SNP ID: rs7469576 /
SNP Chromosome Position: 136117964 / SNP in Genomic Sequence: SEQ ID NO: 338 /
SNP Position Genomic: 49485 / Related Interrogated SNP: hCV25610857 /
Related Interrogated SNP: hCV27859399 / SNP Source: dbSNP; Celera; HapMap /
Population(Allele,Count): Caucasian (G,186|A,38) / SNP Type:
INTERGENIC;UNKNOWN /
Context (SEQ ID NO: 519): /
AAATCATTTACAGACATTCAACAAGTTAATTCTGTTATAAATGATAGGCCATATGTATGTTCCAACCTGCTTCCTTTTAGTAC
TAGGACAGTGTAGTACC
R

GCACTTCAGTAAGTGTTAACTTTATTTTCTAAGTGTTTAAATATGTTTTGTTTTGTTTTATGAGGCGGAGTCTTGCTCTGTCG
CCCAGGCTGGAGTGCAG / Celera SNP ID: hCV442675 / Public SNP ID: rs9411471 /
SNP Chromosome Position: 136120091 / SNP in Genomic Sequence: SEQ ID NO: 338 /
SNP Position Genomic: 51612 / Related Interrogated SNP: hCV25610857 /
Related Interrogated SNP: hCV27859399 / SNP Source: dbSNP; Celera; HapMap /
Population(Allele,Count): Caucasian (G,187|A,39) / SNP Type:
INTERGENIC;UNKNOWN /
Context (SEQ ID NO: 520): /
CATCTGTTACTTCTTATCTTATAATGCTTGTGTGTGTTTACCTCCACCTTTCTAAGTCCTGTGACCACGGAGCGATTTATCTC
GAATAGCTTCTTGAAAC
M

GAAACGTGGGAGGCAAAACATTTGAACTTTACATGTCCTAAATTATTTTTTCCATTTTTGTAGTTGGTAGATCGTTTGGCTGT
AGGGAAATCTTGGTGGA / Celera SNP ID: hCV997907 / Public SNP ID: rs657152 / SNP
Chromosome Position: 136139265 / SNP in Genomic Sequence: SEQ ID NO: 338 /
SNP Position Genomic: 70786 / Related Interrogated SNP: hCV27859399 / SNP
Source: dbSNP; HapMap; ABI_Val; HGBASE / Population(Allele,Count): Caucasian
(C,141|A,85) / SNP Type: INTRON /
Context (SEQ ID NO: 521): /
TTTCTTCCCTCAATGGATATCCAGTCATTTCAGCCATTTGAATTGAAAATACTTTCCTTTCTCTATCACATTACCTTAGCACC
CTTATCAAAAAGCAACT
M

TTAAAAAATCAATTGACTATTTCTGTGTAGGTCTATTTCTAAACTCTGCATTCTATTCCATTGAACTATCTGTCTATCCTAAT
ATCAACCTATTCTGATT / Celera SNP ID: hCV997908 / Public SNP ID: rs514659 / SNP
Chromosome Position: 136142203 / SNP in Genomic Sequence: SEQ ID NO: 338 /
SNP Position Genomic: 73724 / Related Interrogated SNP: hCV25610857 / SNP
Source: dbSNP; Celera; HapMap; ABI_Val; HGBASE / Population(Allele,Count):
Caucasian (A,75|C,43) / SNP Type: INTRON;PSEUDOGENE /

Context (SEQ ID NO: 522): /
GGATATCCAGTCATTTCAGCCATTTGAATTGAAAATACTTTCCTTTCTCTATCACATTACCTTAGCACCCTTATCAAAAAGCA
ACTATTAAAAAATCAAT
K
GACTATTTCTGTGTAGGTCTATTTCTAAACTCTGCATTCTATTCCATTGAACTATCTGTCTATCCTAATATCAACCTATTCTG
ATTACTATGTAAAATTT / Celera SNP ID: hCV997909 / Public SNP ID: rs644234 / SNP
Chromosome Position: 136142217 / SNP in Genomic Sequence: SEQ ID NO: 338 /
SNP Position Genomic: 73738 / Related Interrogated SNP: hCV27859399 / SNP
Source: dbSNP; Celera; HapMap; ABI_Val; HGBASE / Population(Allele,Count):
Caucasian (T,142|G,84) / SNP Type: TFBS SYNONYMOUS;INTRON;PSEUDOGENE /
Context (SEQ ID NO: 523): /
CAACTCAATTTTTAAAAAGGCAAAACATTTCAACAGGCATTTCACCAAAAACATATACAAATGGCTAAAAAGCACAAGAAAAG
CCACTTGCCATCATTTG
W
CCTTGGGGAAATGCAAATCAAAACTATAATGAGATACCACCTTTTACCCACAAGAATGGTATGTATTGCTGCAGATGTACAGA
CAGTGGAACCCTCAGCC / Celera SNP ID: hCV997918 / Public SNP ID: rs674302 / SNP
Chromosome Position: 136146664 / SNP in Genomic Sequence: SEQ ID NO: 338 /
SNP Position Genomic: 78185 / Related Interrogated SNP: hCV25610857 / SNP
Source: dbSNP; Celera; HapMap; ABI_Val; HGBASE / Population(Allele,Count):
Caucasian (T,77|A,43) / SNP Type: TRANSCRIPTION FACTOR BINDING SITE;INTRON /
Context (SEQ ID NO: 524): /
TATGTGTCTGCGGTTGCCTGGCTAGCTCCCTCTCTGGCCTGGCCCAGAGTCCCAGGGCCTTGTGGGTCAGCCACTTCCTTTGG
TGTCTGGGGCCAACTGC
Y
TTGCCTGCCCCACCTACATCTGACAGAGAAGTGACCACGGCTCTGCCAGCATCCTCTTTCTAGGGTCCAAGGACAGCAAACAG
GTGTCCCCCTCCTGCTA / Celera SNP ID: hCV3183094 / Public SNP ID: rs8176731 /
SNP Chromosome Position: 136132350 / SNP in Genomic Sequence: SEQ ID NO: 338 /
SNP Position Genomic: 63871 / Related Interrogated SNP: hCV25610857 /
Related Interrogated SNP: hCV27859399 / SNP Source: dbSNP; Celera; HapMap /
Population(Allele,Count): Caucasian (T,75|C,43) / SNP Type: INTRON /
Context (SEQ ID NO: 525): /
AGCAAACAGGTGTCCCCCTCCTGCTATCTCTGGTCAGTGAGCAGGAAACATCTGGAGCCTTGTATTGAGGGGGTGGCTCAGCA
TGACGGCCGGCCACAGT
Y
ACAGAGAGGAGGGGGCAGCAGAAGCCACCATCCCTGGGTGAGACGCAGCCTCTGGAGAAGGAGCTGGGTTTTACCGACCTGGC
GAGCCCACGAGCCCACG / Celera SNP ID: hCV3183096 / Public SNP ID: rs8176730 /
SNP Chromosome Position: 136132525 / SNP in Genomic Sequence: SEQ ID NO: 338 /
SNP Position Genomic: 64046 / Related Interrogated SNP: hCV25610857 /
Related Interrogated SNP: hCV27859399 / SNP Source: dbSNP; Celera; HapMap /
Population(Allele,Count): Caucasian (T,103|C,11) / SNP Type: INTRON /
Context (SEQ ID NO: 526): /
TCTATTCCATTGAACTATCTGTCTATCCTAATATCAACCTATTCTGATTACTATGTAAAATTTTAGAATCAGCTGTCAACTTT
ACAAAAATTTCTTCTGG
R
GTTTTAAGTGAGATTATGTGGACTCTGTAGATCCATCTGGGGAGAAGTGACAGTTTAGCAATGTAAGTCTTCCAATCCATGAG
CATGGTATATTTCTCCA / Celera SNP ID: hCV3183111 / Public SNP ID: rs643434 /
SNP Chromosome Position: 136142355 / SNP in Genomic Sequence: SEQ ID NO: 338 /
SNP Position Genomic: 73876 / Related Interrogated SNP: hCV25610857 /
Related Interrogated SNP: hCV27859399 / SNP Source: dbSNP; Celera; HapMap;
ABI_Val; HGBASE / Population(Allele,Count): Caucasian (G,75|A,45) / SNP Type:
TRANSCRIPTION FACTOR BINDING SITE;INTRON;PSEUDOGENE /
Context (SEQ ID NO: 527): /
CCAGCCTGATTTATTCATTGACTTTGTATCTTGAGACCTTGCTAAGCTGACTTAGCTGATGGGCTCACAGCATAAACATATAT
AATATATATGACAATAA
Y
AGCATAAAGGAGCAAGGAGGCAATGAAGTTATTATATATACTGGAGCAAGGAAAGGTCACCAGACAATAACTTGAATATACAG
GAAGAAATCAAGAGTAA / Celera SNP ID: hCV3183117 / Public SNP ID: rs545971 /
SNP Chromosome Position: 136143372 / SNP in Genomic Sequence: SEQ ID NO: 338 /
SNP Position Genomic: 74893 / Related Interrogated SNP: hCV25610857 / SNP
Source: dbSNP; HapMap / Population(Allele,Count): Caucasian (C,77|T,43) / SNP
Type: INTRON /
Context (SEQ ID NO: 528): /
GGACCAGAGCACTGTCCCAGATCTCTCCAAACCTGTTTTCCCGTGGGTGAAAGAATGACCCGGGAAGTATTTACCGTTCCTTC

CTGAGAACTCAGCGATA
Y
TGAACACAGTGCTGCCTCACAGTAAACACTGACAAATGGTGAGCATTACTGAGGGCAGGGCCTCGACCTACACCATACCCAGT
TTTCTTCATGAATTTTC / Celera SNP ID: hCV3183164 / Public SNP ID: rs529565 /
SNP Chromosome Position: 136149500 / SNP in Genomic Sequence: SEQ ID NO: 338 /
SNP Position Genomic: 81021 / Related Interrogated SNP: hCV25610857 / SNP
Source: dbSNP; HapMap / Population(Allele,Count): Caucasian (T,77|C,43) / SNP
Type: INTRON /
Context (SEQ ID NO: 529): /
CTCTGCTCCTGGCCCTGCCAGAGCCGCAGCTAGGGCTACAGGGAAAGCCCAGAGGGTCCACCCAGGGACAAACCCGTCACACC
TGGTGTTTGGGGTCAGG
K
GTTCACATTTCTAAAGCAAATTGACCAGTGGGAGAGGAAAGTGGGCTTTTAGTGGAGTCAGGGGCAGCAGGCCAAAAGGCCAC
CCCAAAAAGGCACTGGC / Celera SNP ID: hCV7481808 / Public SNP ID: rs886082 /
SNP Chromosome Position: 136101999 / SNP in Genomic Sequence: SEQ ID NO: 338 /
SNP Position Genomic: 33520 / Related Interrogated SNP: hCV25610857 /
Related Interrogated SNP: hCV27859399 / SNP Source: dbSNP; HGBASE /
Population(Allele,Count): Caucasian (T,39|G,185) / SNP Type: INTRON /
Context (SEQ ID NO: 530): /
AAAGAGACTTAGTGTCATGGAAGATAGTACCAACAAAATCAATGAAGACAGAAAAATTAGAAAGAGAAATTGAGGTGTTACAG
ATAGATTCAGTCTCCAT
Y
CTGGAGCTAGTAATAAAATGAGTGGAGGAAACCATCCAGAGTGCAGCAGCTCTGAAAAACACAACACACACAGATATACACACA
CACATGCACACACACAT / Celera SNP ID: hCV7948166 / Public SNP ID: rs9411463 /
SNP Chromosome Position: 136105515 / SNP in Genomic Sequence: SEQ ID NO: 338 /
SNP Position Genomic: 37036 / Related Interrogated SNP: hCV25610857 /
Related Interrogated SNP: hCV27859399 / SNP Source: dbSNP; Celera; HapMap /
Population(Allele,Count): Caucasian (C,111|T,9) / SNP Type:
INTERGENIC;UNKNOWN /
Context (SEQ ID NO: 531): /
TGTGAGGCAAAGCACGGTGCCCGGCTTGGATGCCACATGTGTTGAGCCAGCTGTGCTGTGGACAGGGCGCTGTGGACAGGGCA
CAGCAGACAGGCGGGGG
R
TGTAGGCATCGGGAGGGGGAGTCATGGCTGGTGAGAGCACCCCTGGAGCGGGAAGTGCTGGGCAGCGAGAGGTCAGGGAGGCA
TGGGGGCTTGGGACGGC / Celera SNP ID: hCV7948171 / Public SNP ID: rs4246170 /
SNP Chromosome Position: 136112413 / SNP in Genomic Sequence: SEQ ID NO: 338 /
SNP Position Genomic: 43934 / Related Interrogated SNP: hCV25610857 /
Related Interrogated SNP: hCV27859399 / SNP Source: dbSNP; Celera; HapMap;
ABI_Val / Population(Allele,Count): Caucasian (A,207|G,19) / SNP Type:
INTERGENIC;UNKNOWN /
Context (SEQ ID NO: 532): /
AAAAAGAAGAAGCTCTGAGAACTAACATCAGGAATGGTTAATGAAACGTAGAAGTTAAAGTCATGACAGGGAAGATTGTAGAA
GTATGAACATCACAACA
Y
TGGGAGAAATGAACGAGGTTTCCTATGGTTGACCAGCCAGGAAGCCAGTGCTGCTGAGTTGGCATTTAACTCTTTAGAATATG
CACGCGTTGACAATACA / Celera SNP ID: hCV11840510 / Public SNP ID: rs9411367 /
SNP Chromosome Position: 136118513 / SNP in Genomic Sequence: SEQ ID NO: 338 /
SNP Position Genomic: 50034 / Related Interrogated SNP: hCV25610857 /
Related Interrogated SNP: hCV27859399 / SNP Source: dbSNP; Celera; HapMap /
Population(Allele,Count): Caucasian (C,186|T,40) / SNP Type:
INTERGENIC;UNKNOWN /
Context (SEQ ID NO: 533): /
CTTTGTGCCTCTCTGGCCCAAGATCAGACACTCTCAAGTACTTACTCAATAATACTAATGCACACTCTAATAAACCCTGGGGC
TGTAACGAACGGGCTGG
Y
GTGACCCAGTCCCTCACCCCTCCCACGGCAAGTGCTGCCGAGCAGAAACCCCCCACCCTCATCCCCCCGGGCAGGAAAGAATG
GGGCCTGGGCTCTTCAG / Celera SNP ID: hCV2980259 / Public SNP ID: rs3761821 /
SNP Chromosome Position: 136085783 / SNP in Genomic Sequence: SEQ ID NO: 338 /
SNP Position Genomic: 17304 / Related Interrogated SNP: hCV25610857 /
Related Interrogated SNP: hCV27859399 / SNP Source: dbSNP; Celera; HGBASE /
Population(Allele,Count): Caucasian (T,22|C,94) / SNP Type:
INTERGENIC;UNKNOWN /
Context (SEQ ID NO: 534): /

GGTGGGGGAGGGTGGGGGTGGGAGTGGGGGAGGGGCTCACAGGCGCTGTATTTGCCAGGCTCCTCCGTCTTCCGCATCAGGAT
TTTCTTCTGGATGCACC
K
ATCCTCCCTCCTGGAAAACAGGAGACACGCGGGCAGCGGCTCCCAGGACACCCATGGCCCATCCTCAGCTCACCTGGTGCATG
GCCCTGACCCGGCAACC / Celera SNP ID: hCV27224736 / Public SNP ID: rs2073870 /
SNP Chromosome Position: 136083580 / SNP in Genomic Sequence: SEQ ID NO: 338 /
 SNP Position Genomic: 15101 / Related Interrogated SNP: hCV25610857 /
Related Interrogated SNP: hCV27859399 / SNP Source: dbSNP /
Population(Allele,Count): Caucasian (T,32|G,192) / SNP Type: ACCEPTOR SPLICE
SITE;ESS;ESE;SILENT MUTATION /
Context (SEQ ID NO: 535): /
ATTCCAGGGATGGAAACCTGGTACAATACTCAAAAATCAACCATATTAATATGTTAAGAAGAAAAATTACATGATCATATCGA
TCAATGCAGAAAAAGTA
Y
TTGACTAAATTCATCACGCATTCAGGAAAACAACTCTCAGAAAAGTAGGAATAGAAGAGAACCTCCTTAACTTGACAAAGAGC
ATCTACAAAAAACCTAT / Celera SNP ID: hCV27224742 / Public SNP ID: rs4454354 /
SNP Chromosome Position: 136089529 / SNP in Genomic Sequence: SEQ ID NO: 338 /
 SNP Position Genomic: 21050 / Related Interrogated SNP: hCV25610857 /
Related Interrogated SNP: hCV27859399 / SNP Source: dbSNP; Celera; HapMap /
Population(Allele,Count): Caucasian (T,47|C,179) / SNP Type:
INTERGENIC;UNKNOWN /
Context (SEQ ID NO: 536): /
TTCCCTGCCTGCTGGCCTCTGCCCTGACCGTGGGCCAGCTGTGGCTGGGACAACCCAGCAAACTTCTCCAGCTTCCCTTGGGA
GAACCACACCTTCTCCC
R
TGAGGTCTGAACCCAGACTTGTGGAAAGGATCCCCACCCCTGTTTATTTCTTCCTTAACTGCCCTCGTGCAATCCTGAGCCAT
TCTTTGTGGTTCTCTTT / Celera SNP ID: hCV27224746 / Public SNP ID: rs10793959 /
SNP Chromosome Position: 136093199 / SNP in Genomic Sequence: SEQ ID NO: 338 /
 SNP Position Genomic: 24720 / Related Interrogated SNP: hCV25610857 /
Related Interrogated SNP: hCV27859399 / SNP Source: dbSNP; Celera; HapMap /
Population(Allele,Count): Caucasian (G,40|A,186) / SNP Type:
INTERGENIC;UNKNOWN /
Context (SEQ ID NO: 537): /
GGGAGCTAACGAGCTCAGCTGGATCATGGCAGCTTTATGCCTGGAGGGAATTTCAATCCACCACAGAGAGAGGAACCCAACAG
AGCAAGGAGTCAGACGG
M
GTGGACGGGACACAGATTGGACTCTAGGCATCGCAGACGACTAGAATGTGGGAGACAAGGAAAGAGATAGGAGAGCCCTCTGC
AGAGGAAGAGCTCCAGA / Celera SNP ID: hCV27224748 / Public SNP ID: rs4246169 /
SNP Chromosome Position: 136094150 / SNP in Genomic Sequence: SEQ ID NO: 338 /
 SNP Position Genomic: 25671 / Related Interrogated SNP: hCV25610857 /
Related Interrogated SNP: hCV27859399 / SNP Source: dbSNP; Celera; HapMap /
Population(Allele,Count): Caucasian (C,18|A,102) / SNP Type:
INTERGENIC;UNKNOWN /
Context (SEQ ID NO: 538): /
TTTTTGTCCTTGGGATAGTTTGCTGAACTAGAAATACCATTTGATCCAGCAATCCCATTACTGGGTATATACCCAAAGGATTA
TAAATCATGCTTCCATA
R
AGACACATGCTCACGTATGTTTATTGCAGCACTATTCACAATAGCAAAGACTTGAAACCAACCCAAATGTCCATCAATGATAG
ACTGGATTAAGAAAATG / Celera SNP ID: hCV27224776 / Public SNP ID: rs7852396 /
SNP Chromosome Position: 136108670 / SNP in Genomic Sequence: SEQ ID NO: 338 /
 SNP Position Genomic: 40191 / Related Interrogated SNP: hCV25610857 /
Related Interrogated SNP: hCV27859399 / SNP Source: dbSNP; Celera; HapMap /
Population(Allele,Count): Caucasian (G,17|A,99) / SNP Type: TFBS
SYNONYMOUS;INTERGENIC;UNKNOWN /
Context (SEQ ID NO: 539): /
CTGCACTGCGGCTCCTCCTCTGTCCCTGGGGTTCCGTCCTCCTTGGTGGCCTGCAGGGCCTCTGTCCACTGCCGGGCCACCAA
CTAGGCTACAGAGACAC
R
GTGACGGTCAGGTTTCTCAGGTCCCCCGGGCTAAGGAACACGTGAGCCTTTCAGCCACCACGTTGACCACACATGCGACAAAG
GCAGAGGGGCCGCCTGG / Celera SNP ID: hCV27224778 / Public SNP ID: rs11244041 /
SNP Chromosome Position: 136109279 / SNP in Genomic Sequence: SEQ ID NO: 338 /
 SNP Position Genomic: 40800 / Related Interrogated SNP: hCV25610857 /
Related Interrogated SNP: hCV27859399 / SNP Source: dbSNP; Celera; HapMap /

Population(Allele,Count): Caucasian (G,40|A,186) / SNP Type: INTERGENIC;UNKNOWN /
Context (SEQ ID NO: 540): /
GCCACCATGCTGAAGCATCAGTTACAAAATGGACTTGATTTGCTGTGTGAGTGGGTCAGTGCAGTGCAAGGGGTGGCTGAAGG
CACTGGTGACCCACGCT
S
TCGGCCCACCTGCCACAGTTGTAGATCGTTCCTGGTGCACGGACGTATTCACAGCTTCCCACCTTAAGTGCCTGCACCTTTTC
TTCTGTGCTTCCCCTGA / Celera SNP ID: hCV27478783 / Public SNP ID: rs3761823 /
SNP Chromosome Position: 136086812 / SNP in Genomic Sequence: SEQ ID NO: 338 /
SNP Position Genomic: 18333 / Related Interrogated SNP: hCV25610857 /
Related Interrogated SNP: hCV27859399 / SNP Source: dbSNP; HapMap; ABI_Val;
HGBASE / Population(Allele,Count): Caucasian (C,27|G,93) / SNP Type:
INTERGENIC;UNKNOWN /
Context (SEQ ID NO: 541): /
ATCCTCAGTCTGTAGGGATAGTAACTGATGGGATAAATGGTCTGGACTCTTCCTTCTAAGTGGAACAATCAAATATGGTTCCA
CCCTATTTCTCAGATGG
Y
CCTCAGAAGGACTGAGTCCCAGTTGCCCACAATGGTAACATCATTAACATATTTTGTTGGTTTTTCTACCTTCCAAATCTCAC
TTGATCTCAGACTTCTG / Celera SNP ID: hCV27886018 / Public SNP ID: rs4962104 /
SNP Chromosome Position: 136087047 / SNP in Genomic Sequence: SEQ ID NO: 338 /
SNP Position Genomic: 18568 / Related Interrogated SNP: hCV25610857 /
Related Interrogated SNP: hCV27859399 / SNP Source: dbSNP; HapMap; ABI_Val;
HGBASE / Population(Allele,Count): Caucasian (T,47|C,177) / SNP Type:
INTERGENIC;UNKNOWN /
Context (SEQ ID NO: 542): /
GGTTGCAGGGTGGGGGTACGGGACCCACCCACGAGCTTCGCCCCTCGGATCGGCTTCCCGTGCAGCTCACCCTCACAGTAAAA
GATGTAGTGGTCCTTCA
Y
GTGCGACCTGATGATGTATGCCACGTGCTTGCCCCTGTCTGCAGAAGGACAGCAGCATAGTCCTGAATTAGCCACCGGGGCCT
TCAGAGAGGATCCCGGG / Celera SNP ID: hCV27936941 / Public SNP ID: rs4379511 /
SNP Chromosome Position: 136101557 / SNP in Genomic Sequence: SEQ ID NO: 338 /
SNP Position Genomic: 33078 / Related Interrogated SNP: hCV25610857 /
Related Interrogated SNP: hCV27859399 / SNP Source: dbSNP /
Population(Allele,Count): Caucasian (T,32|C,194) / SNP Type: MISSENSE
MUTATION;ESS;ESE;TRANSCRIPTION FACTOR BINDING SITE;ESE SYNONY / MOUS //
Context (SEQ ID NO: 543): /
CTCACTTTGTTACCCAGGCTGGTCTTGAACTCCTGGGCTCAAGCGATCCTCCCGCCTCCACCTCCTAAAGCGTTGGGATTAAA
GGTGTGAGCCTCCATGC
S
GGGCATTACTTTTTAAGAGCATATATAACTTAGCCACGAGAAAAACAGCAGCATTTTTCAGCAACAACAAAAGGGAGAGAGAT
GAGAGAAAGGCCCCCAG / Celera SNP ID: hCV27936942 / Public SNP ID: rs4424335 /
SNP Chromosome Position: 136113559 / SNP in Genomic Sequence: SEQ ID NO: 338 /
SNP Position Genomic: 45080 / Related Interrogated SNP: hCV25610857 /
Related Interrogated SNP: hCV27859399 / SNP Source: dbSNP; HapMap; HGBASE /
Population(Allele,Count): Caucasian (C,108|G,10) / SNP Type:
INTERGENIC;UNKNOWN /
Context (SEQ ID NO: 544): /
TTCCTGGCTCTGAGAAGTTGCTGAATAAAACCTGTCCTCACCGCTTTAACTAGCATTCAGCTTGGCTCACTGGTGACGACGTA
TCCAAAATGCCGTATTT
M
ACACATTGGCTTGAGCGGTAGAGCAGCTCTCAGATGGCTTCCAGGACTGGCTGAGCTGGTGTTGAGGCCTCATTCACAGGGGC
TGGGACGCCAGGATGGC / Celera SNP ID: hCV28002068 / Public SNP ID: rs4322078 /
SNP Chromosome Position: 136098479 / SNP in Genomic Sequence: SEQ ID NO: 338 /
SNP Position Genomic: 30000 / Related Interrogated SNP: hCV25610857 /
Related Interrogated SNP: hCV27859399 / SNP Source: dbSNP; HapMap; ABI_Val /
Population(Allele,Count): Caucasian (A,18|C,102) / SNP Type:
INTERGENIC;UNKNOWN /
Context (SEQ ID NO: 545): /
CTACCCCCCCACATCCAGCGTGTGTTTGAGTTTAGGCTCTGGCTTAGGCTCCGGGACTCACTGGCCGTGTATTTTCCCGGCTC
GTCAGTTTTCTCCAGGA
Y
GACCTTCACCTCCTGGCACTGGCCACTTATCCTGGAAAACAGAAGCCCCCTGGCAAAGCGGCCCTGACCTGTGCCTCCCCCGG
CCCTGGAGCAGCCACGC / Celera SNP ID: hCV29393501 / Public SNP ID: rs4507838 /

114

SNP Chromosome Position: 136102199 / SNP in Genomic Sequence: SEQ ID NO: 338 / SNP Position Genomic: 33720 / Related Interrogated SNP: hCV25610857 / Related Interrogated SNP: hCV27859399 / SNP Source: dbSNP; HapMap; HGBASE / Population(Allele,Count): Caucasian (T,32|C,190) / SNP Type: MISSENSE MUTATION;ESE SYNONYMOUS /
Context (SEQ ID NO: 546): /
GCTGGGCAGCGAGAGGTCAGGGAGGCATGGGGGCTTGGGACGGCCACCAGCCACATCAGAGCAGCACTGCTGCTGATTCCCAG
CAACATGGAATGAGGCC
Y
CTTGGCAAAGTAACAGAACGTATCTTTGAAATGGCAGCAGCTGATCTTTAAAACAGAGGCAAGCCGTTCCTGTCTCAAAACGT
TTAAATCCTCACCCCAA / Celera SNP ID: hCV29393505 / Public SNP ID: rs4962039 /
SNP Chromosome Position: 136112570 / SNP in Genomic Sequence: SEQ ID NO: 338 / SNP Position Genomic: 44091 / Related Interrogated SNP: hCV25610857 / Related Interrogated SNP: hCV27859399 / SNP Source: dbSNP; HapMap; ABI_Val; HGBASE / Population(Allele,Count): Caucasian (C,195|T,21) / SNP Type: INTERGENIC;UNKNOWN /
Context (SEQ ID NO: 547): /
GGAGGGTAGCAGGAAGTTGCTGTCCACTTGGACTAGCAGAATACACAACTCAAGTGGAGATTTATTCTGACATTTTCCAGAAC
AACTCTAAAACTTCTTA
Y
TTACTCTTTATGCCATCTACAAGACACAACACACATTTATTGTCTCAATGTTAAGAAAACTGAGAAGCCAGCACTAAAGTGCT
AACATGGAAGGGAAAGT / Celera SNP ID: hCV29393508 / Public SNP ID: rs7046863 /
SNP Chromosome Position: 136121441 / SNP in Genomic Sequence: SEQ ID NO: 338 / SNP Position Genomic: 52962 / Related Interrogated SNP: hCV25610857 / Related Interrogated SNP: hCV27859399 / SNP Source: dbSNP; HapMap / Population(Allele,Count): Caucasian (T,187|C,39) / SNP Type: INTERGENIC;UNKNOWN /
Context (SEQ ID NO: 548): /
GTGAAAGGCTAAAGTCCCTTTCTTCCCTCAATGGATATCCAGTCATTTCAGCCATTTGAATTGAAAATACTTTCCTTTCTCTA
TCACATTACCTTAGCAC
Y
CTTATCAAAAAGCAACTATTAAAAAAATCAATTGACTATTTCTGTGTAGGTCTATTTCTAAACTCTGCATTCTATTCCATTGAA
CTATCTGTCTATCCTAA / Celera SNP ID: hCV29597378 / Public SNP ID: rs8176672 /
SNP Chromosome Position: 136142185 / SNP in Genomic Sequence: SEQ ID NO: 338 / SNP Position Genomic: 73706 / Related Interrogated SNP: hCV25610857 / Related Interrogated SNP: hCV27859399 / SNP Source: dbSNP / Population(Allele,Count): Caucasian (C,209|T,17) / SNP Type: TFBS SYNONYMOUS;INTRON;PSEUDOGENE /
Context (SEQ ID NO: 549): /
AAATTTTTAAAAATTTAAATGATATAGAGGGTAGTCTCTGACCACATGGAATCTAACTATAAATCAATGCCAGAAAGATAATG
GAAAATCTTTCTTCAGA
K
GCTTGGAAACAACACACCTGTGAATAATCCACAGGTCAAAGAGGAAGCCTGAAGGTAAATTTAAAAATATACTAGGCTGGATG
AAAATGAAAATACAACA / Celera SNP ID: hCV32126443 / Public SNP ID: rs10793957 /
SNP Chromosome Position: 136087931 / SNP in Genomic Sequence: SEQ ID NO: 338 / SNP Position Genomic: 19452 / Related Interrogated SNP: hCV25610857 / Related Interrogated SNP: hCV27859399 / SNP Source: dbSNP; HapMap / Population(Allele,Count): Caucasian (G,22|T,98) / SNP Type: INTERGENIC;UNKNOWN /
Context (SEQ ID NO: 550): /
TGGGAAGGCCAGGGTGGGGCCAAGGATCTCGGAGACCAGGGTCGGCTCTGCCTTGCTCTGGGGGTGATGATGGAGGCGCCAGT
GTCCCTCTCTCCAGCGC
Y
CCTCTCTCCAGTGCTGCCACTGAGCCCAGGCTGCTAAAATGGGGGAGCTTGGAGGATCCCTCTGAGACCCCTGAGGCGGGAGC
CCTGCGGTGGCCCCACT / Celera SNP ID: hCV32126435 / Public SNP ID: rs11244034 /
SNP Chromosome Position: 136080272 / SNP in Genomic Sequence: SEQ ID NO: 338 / SNP Position Genomic: 11793 / Related Interrogated SNP: hCV25610857 / Related Interrogated SNP: hCV27859399 / SNP Source: dbSNP / Population(Allele,Count): Caucasian (T,33|C,191) / SNP Type: INTERGENIC;UNKNOWN /
Context (SEQ ID NO: 551): /
GGCCAGCACCCAGCTGCCCCTTCTTAGAGCTTTTGCTAAAAAGGGCTTACACCTGTGACTCCTTCCTCTGCCACTTTGAGAAG
TCTGTGTTTCTCCTACA

R
TGCAGAAGTGTATTTCTCAGGCTGGCTGCAGTGGCTCACATCCGTAATCCCAGCACTTTGGGAGGCATAGGTGGGTGGATCAA
TTGAGGTCAGGAGTTCA / Celera SNP ID: hCV32126454 / Public SNP ID: rs13300535 /
SNP Chromosome Position: 136097813 / SNP in Genomic Sequence: SEQ ID NO: 338 /
SNP Position Genomic: 29334 / Related Interrogated SNP: hCV25610857 /
Related Interrogated SNP: hCV27859399 / SNP Source: dbSNP; HapMap /
Population(Allele,Count): Caucasian (A,23|G,97) / SNP Type:
INTERGENIC;UNKNOWN /
Context (SEQ ID NO: 552): /
CCCAGATTCAAGTGATTCTCCTGCCTCCGCCTCCCAAGTAGCTGGGATTACAGATGCACACCACCATACCCAGCTACAGGCTA
CCCTTTCAAGACTCATT
R
TATAGCTAGCAGATGGACAGATGCATAGATCAATGGGACAGACAATAAAGAATCCTCAAACAGACCTGCACGCATATACCCAG
TTAAGTTTTTACAAAGA / Celera SNP ID: hCV32126447 / Public SNP ID: rs6597610 /
SNP Chromosome Position: 136091096 / SNP in Genomic Sequence: SEQ ID NO: 338 /
SNP Position Genomic: 22617 / Related Interrogated SNP: hCV25610857 /
Related Interrogated SNP: hCV27859399 / SNP Source: dbSNP; HapMap /
Population(Allele,Count): Caucasian (G,40|A,186) / SNP Type:
INTERGENIC;UNKNOWN /
Context (SEQ ID NO: 553): /
TGTCTCAAAACGTTTAAATCCTCACCCCAAACCATTATGCGGCTCACTGCACCCGTGGAAAGCCAACATCAATACCTGCGTGC
ATGCTGGCCTCCCTGGC
R
TGGGCATTCTTTGAGGTTGCTGGCTCAAAGCCCCCAGCACTGCCAGAGGGGGAAACCGGCAAACCCAGGAACTCCAAGGCTCA
GCTCAACAGCCACACCC / Celera SNP ID: hCV30613004 / Public SNP ID: rs7855713 /
SNP Chromosome Position: 136112741 / SNP in Genomic Sequence: SEQ ID NO: 338 /
SNP Position Genomic: 44262 / Related Interrogated SNP: hCV25610857 /
Related Interrogated SNP: hCV27859399 / SNP Source: dbSNP /
Population(Allele,Count): Caucasian (A,206|G,20) / SNP Type:
INTERGENIC;UNKNOWN /
Context (SEQ ID NO: 554): /
GCCCACCTGCCACAGTTGTAGATCGTTCCTGGTGCACGGACGTATTCACAGCTTCCCACCTTAAGTGCCTGCACCTTTTCTTC
TGTGCTTCCCCTGACAC
Y
GATGAAACCCAGATGTGCAAGTGAATCAGCATCCTCAGTCTGTAGGGATAGTAACTGATGGGATAAATGGTCTGGACTCTTCC
TTCTAAGTGGAACAATC / Celera SNP ID: hCV32126442 / Public SNP ID: rs7864821 /
SNP Chromosome Position: 136086916 / SNP in Genomic Sequence: SEQ ID NO: 338 /
SNP Position Genomic: 18437 / Related Interrogated SNP: hCV25610857 /
Related Interrogated SNP: hCV27859399 / SNP Source: dbSNP /
Population(Allele,Count): Caucasian (C,40|T,186) / SNP Type:
INTERGENIC;UNKNOWN /
Context (SEQ ID NO: 555): /
CGTTACTGTCACAATATTTTCTGTGGAGTTGAAAAGCAAATGCCCCAAGGCAAGCACAGAGGGTGCAGGGGTTGGGGTGTCCC
GAGGGTGGGGCTGTGGG
Y
GGGCCTGGACCCCCTGCACTGCGGCTCCTCCTCTGTCCCTGGGGTTCCGTCCTCCTTGGTGGCCTGCAGGGCCTCTGTCCACT
GCCGGGCCACCAACTAG / Celera SNP ID: hCV29531061 / Public SNP ID: rs9411464 /
SNP Chromosome Position: 136109165 / SNP in Genomic Sequence: SEQ ID NO: 338 /
SNP Position Genomic: 40686 / Related Interrogated SNP: hCV25610857 /
Related Interrogated SNP: hCV27859399 / SNP Source: dbSNP; HapMap /
Population(Allele,Count): Caucasian (C,95|T,11) / SNP Type:
INTERGENIC;UNKNOWN /
Context (SEQ ID NO: 556): /
GTCTAACTAGTGAAATAGCATTAATGAAACTCAAACAAATTTCCACTGATAACATTTCAGAGTGCACGCTATAGTCAAACAGC
AATGTTATAAATACCTA
Y
GCAGAAAAGAAACAGATAGGGTGATTCCAGAGACCACAGAGTCTTAAGTTATAAGGTAATTTCAAATTTCCCAAGGGTTGGTT
TACAAAATGGTTTGAAA / Celera SNP ID: hCV30504633 / Public SNP ID: rs9919007 /
SNP Chromosome Position: 136119527 / SNP in Genomic Sequence: SEQ ID NO: 338 /
SNP Position Genomic: 51048 / Related Interrogated SNP: hCV25610857 /
Related Interrogated SNP: hCV27859399 / SNP Source: dbSNP; HapMap /
Population(Allele,Count): Caucasian (C,186|T,38) / SNP Type:
INTERGENIC;UNKNOWN /

Context                    (SEQ ID NO: 557): /
CGTCAGATGAACTATGTATACGTGGGAAATTATGATTAAAGCTTTACGTTCAAAGTCAAGTAATAGACTATTTTTAAAAATAC
ATGGTAAGGAGAAAAAA
R
TGTCTATGTTGAACAACTGAGAAACACTTAAGCGAGGTTACAAATGACTAATAACTATGCACAACAATCTTTTCCAGGATCAA
CTTTTTTCCTTTGCAAAA / Celera SNP ID:   hCV29549191 / Public SNP ID:   rs9411468 /
SNP Chromosome Position:   136119888 / SNP in Genomic Sequence:   SEQ ID NO: 338 /
 SNP Position Genomic:   51409 / Related Interrogated SNP:   hCV25610857 /
Related Interrogated SNP:   hCV27859399 / SNP Source:   dbSNP; HapMap /
Population(Allele,Count):   Caucasian (G,84|A,18) / SNP Type:
INTERGENIC;UNKNOWN /
Context                    (SEQ ID NO: 558): /
GAGACCGTATTGCAAAGGTATAAAGTGGTCTACCCAACAAAGTATTTTACAGATGATGGCGATGAAAGCAAGGATTAATTCTA
ATTAGCTAAATCTAATT
Y
GTTCTTCAGAGGAGAGACTTGTCTGCAAGGTTGCAAGGGAGGGTAGCAGGAAGTTGCTGTCCACTTGGACTAGCAGAATACAC
AACTCAAGTGGAGATTT / Celera SNP ID:   hCV29711974 / Public SNP ID:   rs7855466 /
SNP Chromosome Position:   136121303 / SNP in Genomic Sequence:   SEQ ID NO: 338 /
 SNP Position Genomic:   52824 / Related Interrogated SNP:   hCV25610857 /
Related Interrogated SNP:   hCV27859399 / SNP Source:   dbSNP /
Population(Allele,Count):   Caucasian (C,191|T,35) / SNP Type:
INTERGENIC;UNKNOWN /
Context                    (SEQ ID NO: 559): /
ACATGGAAGGGAAAGTTGTAAGAGGTACTAGGGTATGCTAATAACTCCAGTTTTCTACTCTCCATGCCTGCTTCAGAGAGCCA
CTGCTTCTCCTTCTCGG
R
CCACAGGGAAGGCTGGGAGTCCCCCAGCAACAACAAGGGCACTCCCCGGCGATTCTGTCCCTTCCTTAAACTTCGCTCCAGTC
TTGGTCGACGTGGACGC / Celera SNP ID:   hCV32126487 / Public SNP ID:   rs10901250 /
SNP Chromosome Position:   136121626 / SNP in Genomic Sequence:   SEQ ID NO: 338 /
 SNP Position Genomic:   53147 / Related Interrogated SNP:   hCV25610857 /
Related Interrogated SNP:   hCV27859399 / SNP Source:   dbSNP; HapMap /
Population(Allele,Count):   Caucasian (G,101|A,19) / SNP Type:
INTERGENIC;UNKNOWN //

Gene Number:   2 / Gene Symbol:   ACVR2B - 93 / Gene Name:   activin A receptor,
type IIB / Chromosome:   3 / OMIM NUMBER:   602730 / OMIM Information:
Left-right axis malformations (3) // Genomic Sequence (SEQ ID NO: 339): //   /
SNP Information /
Context                    (SEQ ID NO: 560): /
GAGAGAAAGATTTTTTGTTTAAGTTAGAAAAGGTAATTTTCTCTAATACTAACACACGAAATGTCATATAGCTATGTTAACAT
CAGATAAAATGGACTTT
Y
TGGCAAAGAGCATTCATTACAAATGAAGAGGGTTACTATATAGTAAGAAAAATTTTATTACCAAGAATATATAAAATAATTTC
TGTAACTCAAAGGAAAA / Celera SNP ID:   hCV3083980 / Public SNP ID:   rs12637034 /
SNP Chromosome Position:   38512619 / SNP in Genomic Sequence:   SEQ ID NO: 339 /
SNP Position Genomic:   47223 / Related Interrogated SNP:   hCV15949414 / SNP
Source:   dbSNP; Celera; HapMap / Population(Allele,Count):   Caucasian
(T,104|C,6) / SNP Type:   INTRON /
Context                    (SEQ ID NO: 561): /
GACAGAAGGATTTTCTTCCTTTCCTTGAGTTATGGTGGACATCATTGAATAATTCATGGGCTTTTTCCTAATTCTCCTTAGTC
CTTCTAGAACACAGGTC
M
ACAGATGTTTGCGACACCAGTCCCCATGATCTGAGTCTAGGTCCCAGTGGGGATCCATACTGGGGATGGCTTGCTGACCGGTA
GGGAATTTGTCCCTTTC / Celera SNP ID:   hCV11327199 / Public SNP ID:   rs12637760 /
SNP Chromosome Position:   38485396 / SNP in Genomic Sequence:   SEQ ID NO: 339 /
SNP Position Genomic:   20000 / Related Interrogated SNP:   hCV15949414 / SNP
Source:   dbSNP; Celera; HapMap / Population(Allele,Count):   Caucasian
(A,114|C,6) / SNP Type:   INTERGENIC;UNKNOWN /
Context                    (SEQ ID NO: 562): /
TGCTTTCCAGCCTTGAAATGTCATTTCTGTAGTTCTCTCCTGTTCTCTTTGTTCTTACAGGTTTATGCATTAAACTACTTTAT
TGCAGTTTTAGTGGGAC
Y
ACAAGAGGGAGCAAAAAGGAAATGTTTGTGTATAATCCTTGACCTTTACCTGGAAGCTCTCCTTCCAGTGTTTTCCAGGAATG

ATGGGGCCTGGAAAAAG / Celera SNP ID: hCV15961938 / Public SNP ID: rs2284816 /
SNP Chromosome Position: 38510346 / SNP in Genomic Sequence: SEQ ID NO: 339 /
SNP Position Genomic: 44950 / Related Interrogated SNP: hCV15949414 / SNP
Source: dbSNP; HapMap; HGBASE / Population(Allele,Count): Caucasian
(T,114|C,6) / SNP Type: TRANSCRIPTION FACTOR BINDING SITE;INTRON /
Context (SEQ ID NO: 563): /
CTATTGCAGATGACAAACTCTGTCATTCCCAAACGTTGCTGAGTACTGGAGTCATTTGCGGAGCTATTAAAAAAATACAATTAT
GGGTCCTTATCCCCAAC
S
TGGAATTAGCAAACCAGGTCTCCAGTTTCAGAGATTTGGACTTAATCTCACTAAAGGAACTCATGGTTCCCCAATAATAAAAT
AATGATAGTCTTTAATA / Celera SNP ID: hCV27512998 / Public SNP ID: rs3762790 /
SNP Chromosome Position: 38535168 / SNP in Genomic Sequence: SEQ ID NO: 339 /
SNP Position Genomic: 69772 / Related Interrogated SNP: hCV15949414 / SNP
Source: dbSNP; HapMap; HGBASE / Population(Allele,Count): Caucasian
(C,114|G,6) / SNP Type: INTERGENIC;UNKNOWN /
Context (SEQ ID NO: 564): /
GAACAGAGCTACACTGGACTCGGGCACATTCGGAGCAGCATCCTTTAGTATGGAGGCTACTTCTCAGGTAACCAGGAATTGAG
GGGAAGGACCTTGTGGA
R
GCCGAGCATTAACAGCAAGAGCGGGGTTTGGAGAAAGTCTGAGATTGGGTGCAGCCCTGACTTACCTGCTGGCCCTGACCAGT
TTCTTTTCACTAACTTG / Celera SNP ID: hCV32001449 / Public SNP ID: rs12636077 /
SNP Chromosome Position: 38525588 / SNP in Genomic Sequence: SEQ ID NO: 339 /
SNP Position Genomic: 60192 / Related Interrogated SNP: hCV15949414 / SNP
Source: dbSNP; HapMap / Population(Allele,Count): Caucasian (G,114|A,6) / SNP
Type: MICRORNA;UTR3 /
Context (SEQ ID NO: 565): /
TTACTGCCCCGGGGTCCTTTCCTCTGGGTGTTGGGGCACAGGGTGCTATGGGAGGCCCATTTGCTTCCCTCTCGGAGCTCAGT
TTTTGCTTCATGGGTCA
R
AATGTGGGCTGGCCAAGTGGTTACAGGAACAGGGTTTCGGTAAGCTATGTTGTCTTTTTTTTTTTTTTTTTTTTTTTTTTTTTAA
TGGTTTGATTTTGTGCT / Celera SNP ID: hDV71601922 / Public SNP ID: rs17037775 /
SNP Chromosome Position: 38527103 / SNP in Genomic Sequence: SEQ ID NO: 339 /
SNP Position Genomic: 61707 / Related Interrogated SNP: hCV15949414 / SNP
Source: dbSNP; HapMap / Population(Allele,Count): Caucasian (A,114|G,6) / SNP
Type: MICRORNA;UTR3 /
Context (SEQ ID NO: 566): /
GGCGGCCAAGAGGGGTGGGCAGATCTCATTGGGGGTGGCAGGATTGTAGGTGCAGCAGCAGCAGCAGCATGCGGGGAGGGTTG
CATGTCCCTCAGAGTCA
Y
TAAGTGTAGCGAAGCGAAGGGGGCGGCTTCACGCAGCCCAAAGTCCCCTGCATCTCTTGCCACCAGTCTGCGTCGCGTTACCG
GCCCGGCGGGCTGTTGG / Celera SNP ID: hDV76880100 / Public SNP ID: rs3749388 /
SNP Chromosome Position: 38496301 / SNP in Genomic Sequence: SEQ ID NO: 339 /
SNP Position Genomic: 30905 / Related Interrogated SNP: hCV15949414 / SNP
Source: CDX; dbSNP / Population(Allele,Count): Caucasian (C,216|T,10) / SNP
Type: MISSENSE MUTATION;ESS;ESE SYNONYMOUS;INTRON //

Gene Number: 3 / Gene Symbol: APOH - 350 / Gene Name: apolipoprotein H
(beta-2-glycoprotein I) / Chromosome: 17 / OMIM NUMBER: 138700 / OMIM
Information: [Apolipoprotein H deficiency] (3) // Genomic Sequence (SEQ ID NO:
340): // / SNP Information /
Context (SEQ ID NO: 567): /
TTCAATTAGGTTCCTTGGATGAACAAGAAACAAGTGTGACATTTTGTGTGGAATCTGAAAACCACCTTAGCATGGCTTTACAT
CGGATGCATCAGTTTTC
S
AAAAAGCCAGAGAACTGTGTTCTGTTGGGGGAGAAAAAGATAAACATTCTAAGAGAAACAATCATTTCTTAAATGTGGTAAAT
AGCTAAGCCAAACCAAA / Celera SNP ID: hCV2303891 / Public SNP ID: rs1801690 /
SNP Chromosome Position: 64208285 / SNP in Genomic Sequence: SEQ ID NO: 340 /
SNP Position Genomic: 10138 / SNP Source: HGMD; dbSNP; HapMap /
Population(Allele,Count): Caucasian (C,213|G,13) / SNP Type: MISSENSE
MUTATION;ESS;ESE SYNONYMOUS;TFBS SYNONYMOUS;PSEUDOGENE /
Context (SEQ ID NO: 568): /
AATAATTATTATTATAGGAGACTAGAGAGACATGACAACTAAATTAAAGGTGTGATTCTTGATTAGATCCTGCATAAAACAAA
ACTCATGTATAAGAGAT

M
CTTCTGGGTCAATTGGGAAAATTTGCATCTGAGATAAATGTTGCATGATATCGTTGTTGTTATGTTATTAGAATGGTTTGGAT
TATTATTGTTAATAATC / Celera SNP ID: hCV2658414 / Public SNP ID: rs8178851 /
SNP Chromosome Position: 64215239 / SNP in Genomic Sequence: SEQ ID NO: 340 /
SNP Position Genomic: 17092 / Related Interrogated SNP: hCV2303891 / SNP
Source: dbSNP; Celera; HapMap / Population(Allele,Count): Caucasian
(A,111|C,9) / SNP Type: INTRON /
Context (SEQ ID NO: 569): /
TCTCTACTAAAATTATAAAAATTAGCTGGGCGTGGTGGCAGGTGCCTATATTCCCAGCTACTTGGGAGGCTGAGGCAGGAGAA
TTGCTCAAACCTGGGAG
R
TAGATGTTGCAGTGAGCCAAAATTGCACCACTGCACTCCAGCCTGGGTGACAGAGTGAGACACCATTTCAAAATAATAATAAT
AATTATTATTATAGGAG / Celera SNP ID: hCV2658416 / Public SNP ID: rs8178853 /
SNP Chromosome Position: 64215058 / SNP in Genomic Sequence: SEQ ID NO: 340 /
SNP Position Genomic: 16911 / Related Interrogated SNP: hCV2303891 / SNP
Source: dbSNP; Celera; HapMap / Population(Allele,Count): Caucasian
(G,111|A,9) / SNP Type: INTRON /
Context (SEQ ID NO: 570): /
CTGAGGTGTCAAAGTTAGGCATGCTGACTATCTGGTGCTATCAAAATGCAGACCTAATTATTGTGTATATGAGTAGTGCATGT
GTACATAAATATGTGTA
Y
GTATTTCCTAGCTCTGTCTGTTGAAGAAGACCTAGAAGCAGTGATGCCTCTGTATCAATGAGCACTACACCCAGACACAATGC
AGACCTTGATTTCTTTT / Celera SNP ID: hCV2658437 / Public SNP ID: rs11651658 /
SNP Chromosome Position: 64198640 / SNP in Genomic Sequence: SEQ ID NO: 340 /
SNP Position Genomic: 493 / Related Interrogated SNP: hCV2303891 / SNP
Source: dbSNP; Celera; HapMap / Population(Allele,Count): Caucasian
(T,211|C,15) / SNP Type: INTERGENIC;UNKNOWN /
Context (SEQ ID NO: 571): /
TATATTTAAAAGCCTTAACTATTTTCATAGAAATGATAGTTCTTTATTTTCCCAGCCATCATCTGCCCTCCACCATCCATACC
TACGTTTGCAACACTTC
R
TGTTTATAAGCCATCAGCTGGAAACAATTCCCTCTATCGGGACACAGCAGTTTTTGAATGTTTGCCACAACATGCGATGTTTG
GAAATGATACAATTACC / Celera SNP ID: hCV30352818 / Public SNP ID: rs8178847 /
SNP Chromosome Position: 64216815 / SNP in Genomic Sequence: SEQ ID NO: 340 /
SNP Position Genomic: 18668 / Related Interrogated SNP: hCV2303891 / SNP
Source: dbSNP; HapMap / Population(Allele,Count): Caucasian (G,212|A,14) /
SNP Type: MISSENSE MUTATION;ESS;ESE /
Context (SEQ ID NO: 572): /
CTTACTCCGTCCTGCAATAGCAACATGGCAGAGAAAACTCGAGAACAAGATGAGCACTGGAGAAATCATTGTGGATGAGTCAC
ACTGGCACTACCAAAGT
K
GTTTTCGTCTGCTAAAAAGACAGAGCCAAATATGAAAGCGCTTAAACATTAACCATTTTCTGGTGTACTTTTATCTTTGTAAA
TAGAAGATAAAGTATCA / Celera SNP ID: hCV27842286 / Public SNP ID: rs8178822 /
SNP Chromosome Position: 64225529 / SNP in Genomic Sequence: SEQ ID NO: 340 /
SNP Position Genomic: 27382 / Related Interrogated SNP: hCV2303891 / SNP
Source: Applera / Population(Allele,Count): Caucasian (G,37|T,1) African
American (G,33|T,5) total (G,70|T,6) / SNP Type: TRANSCRIPTION FACTOR BINDING
SITE;UTR5 / SNP Source: dbSNP; HapMap / Population(Allele,Count): Caucasian
(G,212|T,14) / SNP Type: TRANSCRIPTION FACTOR BINDING SITE;UTR5 /
Context (SEQ ID NO: 573): /
AGAGTTGGAGCAGTAGCTGGAAGAAAGATGTGAAGTAACAGGGCTTCTTTTTTTAATGGGTGATATTACTGTATATCTGTATG
CTGATTGAAGTGTTCCA
R
TAGAAAGGAAAAAATAAACATGGATAAGCATTAATTAGAGGAGTAAAATCCTTAAGTAGGAGAGAGAAAATGGGACCTAGCAC
ATAAAGAGAGGGTTTGT / Celera SNP ID: hCV29176910 / Public SNP ID: rs7211380 /
SNP Chromosome Position: 64206768 / SNP in Genomic Sequence: SEQ ID NO: 340 /
SNP Position Genomic: 8621 / Related Interrogated SNP: hCV2303891 / SNP
Source: dbSNP; HapMap / Population(Allele,Count): Caucasian (A,111|G,9) / SNP
Type: INTERGENIC;UNKNOWN /
Context (SEQ ID NO: 574): /
GTCAGTATGCTGAGGCTTAAATTGGCTACGTATCTTACAGAGGAGCTTTACACATGGCAATATTAAAGAATCAGTATCAATCC
AGTCCCTCCAGCATCCA
Y

TCTAAAGAGAATTTAGTTATCAGCTGGGCGCAGTTGCTCACACCTGTAATCCCAGCACTTTGGGAGGCTGAGGCGGGTGGATC
ACGAGGTCAGGAGATAG / Celera SNP ID: hCV30443106 / Public SNP ID: rs7213041 /
SNP Chromosome Position: 64224616 / SNP in Genomic Sequence: SEQ ID NO: 340 /
SNP Position Genomic: 26469 / Related Interrogated SNP: hCV2303891 / SNP
Source: dbSNP; HapMap / Population(Allele,Count): Caucasian (C,212|T,14) /
SNP Type: INTRON /
Context (SEQ ID NO: 575): /
GATCTCACTGTTTGCAGAAGTTCCTCTAGTTAAGTGGTGACAGTCCCTGATATACCAATGCCACACATATCCAAGCCACTTCC
AAAGCACTATGAGAGCA
Y

TGGAGTCAACACACCCAGAAATGACTGGCTTAGATAGAGTAATGACCGCGAGGTGGAGAATACAGAATGTGTCCGTGAGTAAA
ATGCTTCCCTTCTGTCA / Celera SNP ID: hCV30082731 / Public SNP ID: rs8178838 /
SNP Chromosome Position: 64219541 / SNP in Genomic Sequence: SEQ ID NO: 340 /
SNP Position Genomic: 21394 / Related Interrogated SNP: hCV2303891 / SNP
Source: dbSNP; HapMap / Population(Allele,Count): Caucasian (T,102|C,6) / SNP
Type: UTR3;INTRON /
Context (SEQ ID NO: 576): /
TTAATAAATGAATGAATGAATTCCAGAGTGGGAGGAAGATGTTTGTTTGATCTCACTGTTTGCAGAAGTTCCTCTAGTTAAGT
GGTGACAGTCCCTGATA
K

ACCAATGCCACACATATCCAAGCCACTTCCAAAGCACTATGAGAGCATTGGAGTCAACACACCCAGAAATGACTGGCTTAGAT
AGAGTAATGACCGCGAG / Celera SNP ID: hCV29992830 / Public SNP ID: rs8178839 /
SNP Chromosome Position: 64219493 / SNP in Genomic Sequence: SEQ ID NO: 340 /
SNP Position Genomic: 21346 / Related Interrogated SNP: hCV2303891 / SNP
Source: dbSNP; HapMap / Population(Allele,Count): Caucasian (T,204|G,22) /
SNP Type: UTR3;INTRON /
Context (SEQ ID NO: 577): /
AACATAAGAACGTCCTTCCCTACATAAGCAAAGCCCAGAGATGACTCTGACAGCTTGGCTGAGGATCCTCTGAAAAAACAACA
GTGCTTTTTGGAGGAAT
R

CCAATCAGCTCCTAGGATGCAAGCCCCTTTGATGGGCTGTTGGCTTACTGTGAGGGTGACTTTCCTCCTCGTCTGTCCCGTAA
GTCACTAGGCTTACTCT / Celera SNP ID: hCV30118779 / Public SNP ID: rs8178841 /
SNP Chromosome Position: 64219197 / SNP in Genomic Sequence: SEQ ID NO: 340 /
SNP Position Genomic: 21050 / Related Interrogated SNP: hCV2303891 / SNP
Source: dbSNP; HapMap / Population(Allele,Count): Caucasian (G,212|A,14) /
SNP Type: UTR3;TFBS SYNONYMOUS;INTRON /
Context (SEQ ID NO: 578): /
GTTTTAAGTGAAAACTGTAGGAACCATCGTGATTCCTACTGCTGAGTGAAAATTTTACTTAATTTGTCCTTTAAAAACTTTCG
TTTTTGCTTGTTCAGGT
Y

TTCTTAAACAATTATTTATTTGAAATTGCTTTACTTTCCACTTGCTTTTTTTTTTTTTTTTTTTTTTGAGATGGAGTTTCACTC
TTGTTGCCCAGGCTGGA / Celera SNP ID: hCV30298770 / Public SNP ID: rs8178842 /
SNP Chromosome Position: 64218640 / SNP in Genomic Sequence: SEQ ID NO: 340 /
SNP Position Genomic: 20493 / Related Interrogated SNP: hCV2303891 / SNP
Source: dbSNP; HapMap / Population(Allele,Count): Caucasian (C,209|T,15) /
SNP Type: TRANSCRIPTION FACTOR BINDING SITE;INTRON /
Context (SEQ ID NO: 579): /
CAGGTGAAGTCCAAAGATATGATGATTCCCCAAATTTTGTTTTTCCAAATGTCTCTTTGGCATCAGTTGACATTTCAAAGCCT
CTCCACTGTTCTTTTAA
Y

TTCTGTAGTGTTTCTTTCTCCTTTTGCCACACCAGATCTCATTTTTAAACCTACCCTAGTTTCAGGCTATGATCTTCTCCTCT
TCTATCAATAGCACCTA / Celera SNP ID: hDV70764335 / Public SNP ID: rs16958979 /
SNP Chromosome Position: 64223859 / SNP in Genomic Sequence: SEQ ID NO: 340 /
SNP Position Genomic: 25712 / Related Interrogated SNP: hCV2303891 / SNP
Source: dbSNP; HapMap / Population(Allele,Count): Caucasian (C,210|T,14) /
SNP Type: INTRON /
Context (SEQ ID NO: 580): /
TGCACCCCAGCCTGGGCGACAGAGTGAGACTCTGTCTCCAAAAAAAAAAAAAAAAAAAAAAAATTAGAACAAAATGCAAACTCCCC
ATCCTGGTCTACTAGGC
S

CTGTGTGATCTGGCTCTGCTGACCTGTATCAGCAGCTTTCTCCCTTTTCCTACTTTTCATCCAAGTATTCATTTACTCCAGGA
CTGTTCTTCAAGGTCAG / Celera SNP ID: hDV70764357 / Public SNP ID: rs16959006 /
SNP Chromosome Position: 64243964 / SNP in Genomic Sequence: SEQ ID NO: 340 /

SNP Position Genomic: 45817 / Related Interrogated SNP: hCV2303891 / SNP Source: dbSNP; HapMap / Population(Allele,Count): Caucasian (C,114|G,6) / SNP Type: INTRON //

Gene Number: 4 / Gene Symbol: AQP2 - 359 / Gene Name: aquaporin 2 (collecting duct) / Chromosome: 12 / OMIM NUMBER: 107777 / OMIM Information: Diabetes insipidus, nephrogenic, autosomal recessive, 222000 (3);/Diab / etes insipidus, nephrogenic, autosomal dominant, 125800 (3) // / Genomic Sequence (SEQ ID NO: 341): // / SNP Information / Context (SEQ ID NO: 581): / ACAAGGGGCAGGTCCTGGGGAATCCCTTGTAAAGGATGAGATGGGAGGGATGGGCTCTGGGTGATGTAGGGAGAGAGATGGAG ACAGAGGCAGAGAGAGA R GCTGGAGCCAGGAACACAGCCACCATAGGAGGGTCAGGATGAAAGGAGCAATGATACCAGAGCAAAGCAGGATGCAGACAGAC TGCTGGCTTCTTCCACC / Celera SNP ID: hCV25597241 / Public SNP ID: rs3782320 / SNP Chromosome Position: 50345099 / SNP in Genomic Sequence: SEQ ID NO: 341 / SNP Position Genomic: 10575 / SNP Source: Applera / Population(Allele,Count): Caucasian (A,5|G,35) African American (A,5|G,33) total (A,10|G,68) / SNP Type: UTR5;INTRON;PSEUDOGENE / SNP Source: dbSNP; HGBASE / Population(Allele,Count): Caucasian (A,8|G,112) / SNP Type: UTR5;INTRON;PSEUDOGENE / Context (SEQ ID NO: 582): / AAATGACTTTGCACCTGCAAATGGTGCTTCAATGTCGTTTTGTATCTTTAAGCTGTGATGTTGTATATATACAATGCCTCCCA GCTAGACTGTAAGCCCT Y TGGGGACAAGGGCTGCTTCCAGTTCTTGGATGGCGTTCTCCTATCTACCTTCCTTCAACTGCTCTGCATATAATAAGCACTCA ATAAATGCTCATTTGCC / Celera SNP ID: hCV15835026 / Public SNP ID: rs2878772 / SNP Chromosome Position: 50352559 / SNP in Genomic Sequence: SEQ ID NO: 341 / SNP Position Genomic: 18035 / Related Interrogated SNP: hCV25597241 / SNP Source: dbSNP; HGBASE / Population(Allele,Count): Caucasian (C,8|T,112) / SNP Type: MICRORNA;UTR3;INTRON / Context (SEQ ID NO: 583): / TCTCCGTCGATCTTCTCCCGGAGGACCCAGAATGCCTGAAGTTGGGAGGGATGGCTGAGGGAAGAGTGCCAGGGCACCAGGTG TCTATGTGTCAGGCCAG R AGTTAGGCACTGGAAATACACAGGAGAACAAACCCCACAGGAATCCCTGCTCTTTTGGAACATCACTTCTGTATGGGTAATTT TTATTCTTTCTTCCTCC / Celera SNP ID: hCV27481297 / Public SNP ID: rs3782318 / SNP Chromosome Position: 50342713 / SNP in Genomic Sequence: SEQ ID NO: 341 / SNP Position Genomic: 8189 / Related Interrogated SNP: hCV25597241 / SNP Source: dbSNP; HGBASE / Population(Allele,Count): Caucasian (A,8|G,112) / SNP Type: INTERGENIC;UNKNOWN / Context (SEQ ID NO: 584): / TGAAGTGGATTGAGGGGTGAGGCCAGTGGTGTCACCTTGGGATGAAGAAATGCTTGCCCACTAGTCCATCAGGATAGGCTTCT TGAAAGAGTAGCAATTT Y GGGAACAATGTGACAGGTGAGACAGAGGGTTTGGTTATAGGAAACGGTTATTGAGCATGTGCTGTGGGCTGGTGCAGCTCTAG AAAATAAGGGTGAAGAG / Celera SNP ID: hDV70885868 / Public SNP ID: rs17124174 / SNP Chromosome Position: 50334781 / SNP in Genomic Sequence: SEQ ID NO: 341 / SNP Position Genomic: 257 / Related Interrogated SNP: hCV25597241 / SNP Source: dbSNP; HapMap / Population(Allele,Count): Caucasian (C,209|T,17) / SNP Type: INTERGENIC;UNKNOWN / Context (SEQ ID NO: 585): / TTGGTGCTCAGCACATGGCTGGTGCTCAATAAATATTTGTTGCATGAATAAATAAATACAATGAACTGCCTCAAGTGTGCTAA AATCTCAAGAAAACAGA Y GTCCATCAAGTAGAACCAGAGGAGAATGGGCCGACTTTGTAGCCAGAAGTGCATGGTTAGATATGGTAAAGGACTTCCCAGAG CTATGGGGCCCTCAGCC / Celera SNP ID: hDV70885870 / Public SNP ID: rs17124176 / SNP Chromosome Position: 50335628 / SNP in Genomic Sequence: SEQ ID NO: 341 / SNP Position Genomic: 1104 / Related Interrogated SNP: hCV25597241 / SNP Source: dbSNP; HapMap / Population(Allele,Count): Caucasian (C,209|T,17) / SNP Type: INTERGENIC;UNKNOWN //

Gene Number: 5 / Gene Symbol: ASAH1 - 427 / Gene Name: N-acylsphingosine

amidohydrolase (acid ceramidase) 1 / Chromosome: 8 / OMIM NUMBER: / OMIM Information: // Genomic Sequence (SEQ ID NO: 342): // / SNP Information / Context (SEQ ID NO: 586): / GTCTCAAAACAAAAAAAAAATCAATCAATAAATAAAAATAAAGAAATAAAATAAAAAATAAAAATAAAGAAATAAAAGATTTA AGCATCATAGCATACCA Y

TGGTGCCTTGTCAAGCATCAATTCATGCCATCTTTTGTAGGGTGGTAAGTCAAGATTTATGGTGTACCATGGAACTGCACCTC TGTACCTGTAATGAGAG / Celera SNP ID: hCV2442143 / Public SNP ID: rs12544854 / SNP Chromosome Position: 17928811 / SNP in Genomic Sequence: SEQ ID NO: 342 / SNP Position Genomic: 24886 / SNP Source: HGMD; dbSNP; HapMap; HGBASE / Population(Allele,Count): Caucasian (C,119|T,107) / SNP Type: MISSENSE MUTATION;ESS;INTRON / Context (SEQ ID NO: 587): / CAGATTGTTTTTCCTGCCTTATTTTTCCATCATTCTCCAGAGAAGGCAGGAACGAGAGTAATTCCACTTGGATGACCTAACCT GACCCACACCACAAAAA Y

ATCATAACAAGCACTCCTTATCTCTCTGCACGAATTCACCCTCTAGCCACTGGCATATACCGTTCTTACCATTTCCTTCCCAC ACTCACAGAGATAAACC / Celera SNP ID: hCV2442136 / Public SNP ID: rs12155668 / SNP Chromosome Position: 17925151 / SNP in Genomic Sequence: SEQ ID NO: 342 / SNP Position Genomic: 21226 / Related Interrogated SNP: hCV2442143 / SNP Source: dbSNP; Celera; HapMap; ABI_Val / Population(Allele,Count): Caucasian (C,119|T,107) / SNP Type: INTRON / Context (SEQ ID NO: 588): / TTGTTTTTCCTGCCTTATTTTTCCATCATTCTCCAGAGAAGGCAGGAACGAGAGTAATTCCACTTGGATGACCTAACCTGACC CACACCACAAAAACATC R

TAACAAGCACTCCTTATCTCTCTGCACGAATTCACCCTCTAGCCACTGGCATATACCGTTCTTACCATTTCCTTCCCACACTC ACAGAGATAAACCTGCA / Celera SNP ID: hCV2442137 / Public SNP ID: rs12155885 / SNP Chromosome Position: 17925155 / SNP in Genomic Sequence: SEQ ID NO: 342 / SNP Position Genomic: 21230 / Related Interrogated SNP: hCV2442143 / SNP Source: dbSNP; Celera; HapMap / Population(Allele,Count): Caucasian (A,56|G,48) / SNP Type: INTRON / Context (SEQ ID NO: 589): / GAGTGTAACACTATTGCCAAACCAATGTCTCCAGTGTTGCCTTCAGGAAGATCACCTTTTAAGCCCCAGATGGCAACATGTAT TATCTTCCCACGATCAG Y

TTCAAGAACCTTCATGATTCTGTCAGCCATCCTAATCTTCCCTACTATATACCTCTTCTCTTCTCATTTTCCTGGACAAAGTC CAAGCCCAAGCTGTATT / Celera SNP ID: hCV2442146 / Public SNP ID: rs966118 / SNP Chromosome Position: 17929404 / SNP in Genomic Sequence: SEQ ID NO: 342 / SNP Position Genomic: 25479 / Related Interrogated SNP: hCV2442143 / SNP Source: dbSNP; Celera; HapMap; HGBASE / Population(Allele,Count): Caucasian (C,61|T,59) / SNP Type: INTRON / Context (SEQ ID NO: 590): / CTAGTCCAAGTGACACAACACACATATACAAATAAGAGAGCAAGTGCTAGGCATCCCGTGAGCAGTGGGAAAGAAATGCTG AATGGGACTCAGATGGA Y

TCAGGGGTGATCTGGCTATCAGTTCCTTTATCTTCAGCATTGATTTGACTAGTCATGCTGGCCAAACAGGTTATCTTTTTCTT CCCTCATATCTGCCCAT / Celera SNP ID: hCV2442155 / Public SNP ID: rs3753117 / SNP Chromosome Position: 17931091 / SNP in Genomic Sequence: SEQ ID NO: 342 / SNP Position Genomic: 27166 / Related Interrogated SNP: hCV2442143 / SNP Source: dbSNP; Celera; HGBASE / Population(Allele,Count): Caucasian (C,119|T,107) / SNP Type: INTRON / Context (SEQ ID NO: 591): / TATACAAATAAGAGAGCAAGTGCTAGGCATCCCGTGAGCAGTGGGAAAGAAATGCTGAATGGGACTCAGATGGACTCAGGGGT GATCTGGCTATCAGTTC Y

TTTATCTTCAGCATTGATTTGACTAGTCATGCTGGCCAAACAGGTTATCTTTTTCTTCCCTCATATCTGCCCATGGATGCTGC CCAAATCTACGCACTTA / Celera SNP ID: hCV2442156 / Public SNP ID: rs35573135 / SNP Chromosome Position: 17931117 / SNP in Genomic Sequence: SEQ ID NO: 342 / SNP Position Genomic: 27192 / Related Interrogated SNP: hCV2442143 / SNP Source: dbSNP; Celera / Population(Allele,Count): Caucasian (C,130|T,96) / SNP Type: INTRON / Context (SEQ ID NO: 592): /

TTCATTATGTCTTTTAGTAAGCATTTTATTTATTTTTCAGCTAAAGGTTATAGTGAATTCTCTGAAGAATATGATAAATACAT
TCGTGCCAAGTGGAAAA
R
TTATGCAGGTGGTGGATGAAAAATTGGTAAGAGATATTATTTCTCACAATAAATGTTAAGCACATAGAAAATGCGCTGTTATT
TATTTAAAGGCATAATT / Celera SNP ID: hCV8947815 / Public SNP ID: rs1049874 /
SNP Chromosome Position: 17927327 / SNP in Genomic Sequence: SEQ ID NO: 342 /
SNP Position Genomic: 23402 / Related Interrogated SNP: hCV2442143 / SNP
Source: dbSNP; HapMap / Population(Allele,Count): Caucasian (A,119|G,107) /
SNP Type: MISSENSE MUTATION;ESE SYNONYMOUS;INTRON /
Context (SEQ ID NO: 593): /
GCCTCATCCTCTTTTGCTCCTGCCAGCATTTTTTTTGTTTTGAGGAAGTCCGGTAAGTATGAACATAAAGGATAGCCTGAAACA
AAAGCTCCGAGAGCATG
M
ACGTGAGGGATAATGGGTTTGGTGCTTGGCAGGTACAAGTGTCATAGGGAGGATGTGGCCACAGCACTCAGCCGCTAGGGCAA
TGATTCTCGACCACGGG / Celera SNP ID: hCV15753018 / Public SNP ID: rs2299606 /
SNP Chromosome Position: 17925986 / SNP in Genomic Sequence: SEQ ID NO: 342 /
SNP Position Genomic: 22061 / Related Interrogated SNP: hCV2442143 / SNP
Source: dbSNP; HapMap; HGBASE / Population(Allele,Count): Caucasian
(C,60|A,60) / SNP Type: INTRON /
Context (SEQ ID NO: 594): /
TAGTCCCATCCCCTTTTACGAAACCATAATTTGCTATGCTGTAACCCTAATATTACCAATTCCCCAAATTTAAGTCCCTCTGA
AGTTCTGACAGTGAGCT
R
GATTCATGCAGATTTGCCCAAGTCCCTGCGAAGAGGTTGCTGAATTATGCCTTTAAATAAATAACAGCGCATTTTCTATGTGC
TTAACATTTATTGTGAG / Celera SNP ID: hCV15844343 / Public SNP ID: rs2427746 /
SNP Chromosome Position: 17927184 / SNP in Genomic Sequence: SEQ ID NO: 342 /
SNP Position Genomic: 23259 / Related Interrogated SNP: hCV2442143 / SNP
Source: dbSNP; HapMap; ABI_Val; HGBASE / Population(Allele,Count): Caucasian
(A,64|G,56) / SNP Type: INTRON /
Context (SEQ ID NO: 595): /
GCTAGGGCAATGATTCTCGACCACGGGAGATTTGGTAACATCTAAGGACATTTTTGGTTGTCACAACTGAGGGGGTGTTACCA
GCATCCAGTGGGTAAGG
R
CCAGGGACACTGCTAAACATCCTGCAATGCACATGACAGCCCCACAAAAAAGAGACACCCACCCCAGGCCGGGCATCCTGAGC
TGCTAAACATCCTGCAA / Celera SNP ID: hCV26696706 / Public SNP ID: rs2299607 /
SNP Chromosome Position: 17926160 / SNP in Genomic Sequence: SEQ ID NO: 342 /
SNP Position Genomic: 22235 / Related Interrogated SNP: hCV2442143 / SNP
Source: dbSNP; HapMap; HGBASE / Population(Allele,Count): Caucasian
(G,61|A,55) / SNP Type: INTRON /
Context (SEQ ID NO: 596): /
CATTTTTGAGACTTAGTGTTTGCAATGTCCATGCCATTCATAATATCAGCAACATCACCTGAACCCACATGGCTACCTAGGTG
GCATGGAAACTAGTCCA
R
GTGACACAACACACACATATACAAATAAGAGAGCAAGTGCTAGGCATCCCGTGAGCAGTGGGAAAGAAATGCTGAATGGGACT
CAGATGGACTCAGGGGT / Celera SNP ID: hCV27474371 / Public SNP ID: rs3753116 /
SNP Chromosome Position: 17930999 / SNP in Genomic Sequence: SEQ ID NO: 342 /
SNP Position Genomic: 27074 / Related Interrogated SNP: hCV2442143 / SNP
Source: dbSNP; HGBASE / Population(Allele,Count): Caucasian (A,119|G,107) /
SNP Type: TRANSCRIPTION FACTOR BINDING SITE;INTRON /
Context (SEQ ID NO: 597): /
CATTAATGCCCAAAATCTGCCATCAAGAGCAATGAGGATCCCTTTAACAGTTCTAGGGGCCAGGCCCTAGGTGTTTCATTGGT
TCTGCGTCAACAAAAAC
R
GGGCTGAGAAATGGAGTCTTCCTGCATGATGAGGATGAAAATGAAATGGTTTGGTGATTGCACAGAAACACTCAGCCCAAATG
AGTTTCAGGGTCAGAGC / Celera SNP ID: hCV31495928 / Public SNP ID: rs12548139 /
SNP Chromosome Position: 17926790 / SNP in Genomic Sequence: SEQ ID NO: 342 /
SNP Position Genomic: 22865 / Related Interrogated SNP: hCV2442143 / SNP
Source: dbSNP; HapMap / Population(Allele,Count): Caucasian (G,61|A,59) / SNP
Type: INTRON /
Context (SEQ ID NO: 598): /
GCACATTTTACCTCTCGTTAAACAGTTTTTCTAAAACATTTCAGTATTTAACTTGTGCGCCTCCATTTCCCCATAAGAATAAAA
GAGTATCCACCTTATAA
Y

AGCAGGGAAGACACTATATGAAAAATGCAAAAATAAAAAAAACACTTAGCAGCATTGGTATTCAATCAACAGTAGTCATCACA
ATTTATTTTCCTACTTC / Celera SNP ID: hCV31495915 / Public SNP ID: rs3753115 /
SNP Chromosome Position: 17930772 / SNP in Genomic Sequence: SEQ ID NO: 342 /
SNP Position Genomic: 26847 / Related Interrogated SNP: hCV2442143 / SNP
Source: dbSNP; HGBASE / Population(Allele,Count): Caucasian (C,119|T,107) /
SNP Type: MISSENSE MUTATION;ESE;INTRON /
Context (SEQ ID NO: 599): /
ACCTAATTTGAAGACTTACTATAAAGCTTTAGTAACTGAGGTGAAATAGTATTGATATAAGGGAAGACAAAAAGACAAATATG
TAAATGAAACTTAATTC
Y

CCAAACAAAACAACATATATACAATCACCTGATTGTATATACAGATACACATTGTGTGTGTGTGTGTGTGTGTGTGTGTGTAT
ATATATATGTATTGTAT / Celera SNP ID: hCV29795393 / Public SNP ID: rs4377998 /
SNP Chromosome Position: 17923339 / SNP in Genomic Sequence: SEQ ID NO: 342 /
SNP Position Genomic: 19414 / SNP Source: dbSNP; HapMap; HGBASE /
Population(Allele,Count): Caucasian (C,101|T,125) / SNP Type: TRANSCRIPTION
FACTOR BINDING SITE;UTR5;INTRON //

Gene Number: 6 / Gene Symbol: F2 - 2147 / Gene Name: coagulation factor II
(thrombin) / Chromosome: 11 / OMIM NUMBER: 176930 / OMIM Information:
Hypoprothrombinemia (3); Dysprothrombinemia (3);/Hyperprothrombinemia / (3) // /
Genomic Sequence (SEQ ID NO: 343): // / SNP Information /
Context (SEQ ID NO: 600): /
GTAGGGGGCCACTCATATTCTGGGCTCCTGGAACCAATCCCGTGAAAGAATTATTTTTGTGTTTCTAAAACTATGGTTCCCAA
TAAAAGTGACTCTCAGC
R
AGCCTCAATGCTCCCAGTGCTATTCATGGGCAGCTCTCTGGGCTCAGGAAGAGCCAGTAATACTACTGGATAAAGAAGACTTA
AGAATCCACCACCTGGT / Celera SNP ID: hCV8726802 / Public SNP ID: rs1799963 /
SNP Chromosome Position: 46761055 / SNP in Genomic Sequence: SEQ ID NO: 343 /
SNP Position Genomic: 30312 / SNP Source: HGBASE;dbSNP /
Population(Allele,Count): no_pop (A,-|G,-) / SNP Type: UTR3;PSEUDOGENE /
Context (SEQ ID NO: 601): /
CTTTCCTATCCTTCAAGGACTGCTTCAAATGTCACCACTTTTGCTGAGACTTCAGGGAGCACCCTCCCTCCTGCACTGTGTTC
TGAAGGCACCTTTAGCA
Y
GACAAAAATGGAACTCTTTGTTTATTTATAAGAGACAGGGTCTCCCTTTTTTGCCCAGGCTGATCTTGAACTCCTGGGCTCAG
GCAATTCTCCCATCTCA / Celera SNP ID: hCV30710896 / Public SNP ID: rs3136520 /
SNP Chromosome Position: 46743232 / SNP in Genomic Sequence: SEQ ID NO: 343 /
SNP Position Genomic: 12489 / SNP Source: dbSNP; HapMap; HGBASE /
Population(Allele,Count): Caucasian (C,219|T,7) / SNP Type: INTRON /
Context (SEQ ID NO: 602): /
GCAGTGAGCCAAGATCGCACAACTACACTCCAGCCTGGGAGACAAGAGTGGAACTCCGTCAGTAAATAAATAAATATAAATAA
AAAATAAAACGCCAGTA
Y
TGAAAAGAACTTTTGGGTTCACTCCAAGAAACCGAGGCCTGGAAAAGGGAAAGGGGCTCTTGCAAGGCTACTTGGTATCAGAG
CCAGGCCCAGAACTTGG / Celera SNP ID: hCV32292446 / Public SNP ID: rs3136524 /
SNP Chromosome Position: 46750525 / SNP in Genomic Sequence: SEQ ID NO: 343 /
SNP Position Genomic: 19782 / Related Interrogated SNP: hCV30710896 / SNP
Source: dbSNP; HapMap; HGBASE; CDX_Heart / Population(Allele,Count): Caucasian
(C,219|T,7) / SNP Type: INTRON //

Gene Number: 7 / Gene Symbol: F2RL1 - 2150 / Gene Name: coagulation factor
II (thrombin) receptor-like 1 / Chromosome: 5 / OMIM NUMBER: 600933 / OMIM
Information: // Genomic Sequence (SEQ ID NO: 344): // / SNP Information /
Context (SEQ ID NO: 603): /
ACCCGCCCTGGGGAGGCGCGCAGCAGAGGCTCCGATTCGGGGCAGGTGAGAGGCTGACTTTCTCTCGGTGCGTCCAGTGGAGC
TCTGAGTTTCGAATCGG
Y
GGCGGCGGATTCCCCGCGCGCCCGGCGTCGGGGCTTCCAGGAGGATGCGGAGCCCCAGCGCGGCGTGGCTGCTGGGGGCCGCC
ATCCTGCTAGCAGCCTC / Celera SNP ID: hCV2829819 / Public SNP ID: rs1529505 /
SNP Chromosome Position: 76114963 / SNP in Genomic Sequence: SEQ ID NO: 344 /
SNP Position Genomic: 10130 / SNP Source: Applera / Population(Allele,Count):
Caucasian (C,20|T,20) African American (C,8|T,28) total (C,28|T,48) / SNP
Type: UTR5 / SNP Source: Applera / Population(Allele,Count): Caucasian

(C,5|T,5) African American (C,5|T,1) total (C,10|T,6) / SNP Type: UTR5 / SNP Source: dbSNP; Celera; HapMap; ABI_Val; HGBASE / Population(Allele,Count): Caucasian (C,104|T,122) / SNP Type: UTR5 //

Gene Number: 8 / Gene Symbol: F5 - 2153 / Gene Name: coagulation factor V (proaccelerin, labile factor) / Chromosome: 1 / OMIM NUMBER: 227400 / OMIM Information: Hemorrhagic diathesis due to factor V deficiency (3);/{Thromboembolism / susceptibility due to factor V Leiden} (3); {Thrombophilia due to factor V Liverpool} (3) // / Genomic Sequence (SEQ ID NO: 345): // / SNP Information /
Context (SEQ ID NO: 604): / CCCAACTTTATGTGCTAGTAATTTCATCCAGGAGAACCTGTGCTTTGCTGCTTGATCTCTTTCTACCTTGGGTCCCTTATGCT TAGCATGTTCTTGACTT Y TGAATTCTCCAGCACCAAGTGAAAGTAGACGTATCCCTGTGACATCTGGCTGTAGAGGATCCTCTATAGGGTCTTCAGAATAT GGGCTGGAATGCTCTGC / Celera SNP ID: hCV8919444 / Public SNP ID: rs4524 / SNP Chromosome Position: 169511755 / SNP in Genomic Sequence: SEQ ID NO: 345 / SNP Position Genomic: 40563 / SNP Source: dbSNP; HapMap / Population(Allele,Count): Caucasian (T,167|C,59) / SNP Type: MISSENSE MUTATION;ESE /
Context (SEQ ID NO: 605): / AATGCCCCATTATTTAGCCAGGAGACCTAACATGTTCTAGCCAGAAGAAATTCTCAGAATTTCTGAAAGGTTACTTCAAGGAC AAAATACCTGTATTCCT Y GCCTGTCCAGGGATCTGCTCTTACAGATTAGAAGTAGTCCTATTAGCCCAGAGGCGATGTCTCTCATGATGTCCACGTCACTG TAGTATGGTCTTGTTAA / Celera SNP ID: hCV11975250 / Public SNP ID: rs6025 / SNP Chromosome Position: 169519049 / SNP in Genomic Sequence: SEQ ID NO: 345 / SNP Position Genomic: 47857 / SNP Source: HGMD; dbSNP; HapMap; ABI_Val / Population(Allele,Count): Caucasian (T,5|C,221) / SNP Type: MISSENSE MUTATION;ESE SYNONYMOUS /
Context (SEQ ID NO: 606): / TCTGTGAATCAGATATGATACCAGTGCTTAGTCCCATTGGCATCCTACAGTCTATGAAAAACAGAAAAAAAATGAATAATTTT TGCTTAGAAAATATATA Y AATAAAGTAAAACTCCATGGTTAGGGATTATGTTTCTGACTCAATAATTAGATATTTTTACCTACCTTGTGTGGCCCTGACTT AAATATATCTGATTATA / Celera SNP ID: hCV22274637 / Public SNP ID: rs2301515 / SNP Chromosome Position: 169494196 / SNP in Genomic Sequence: SEQ ID NO: 345 / SNP Position Genomic: 23004 / Related Interrogated SNP: hCV8919444 / SNP Source: Applera / Population(Allele,Count): Caucasian (C,15|T,5) African American (C,13|T,7) total (C,28|T,12) / SNP Type: INTRON / SNP Source: Applera / Population(Allele,Count): Caucasian (C,23|T,3) African American (C,21|T,5) total (C,44|T,8) / SNP Type: INTRON / SNP Source: dbSNP; HapMap; HGBASE / Population(Allele,Count): Caucasian (C,156|T,70) / SNP Type: INTRON /
Context (SEQ ID NO: 607): / TGGCAGTGTTAAGAGCACACTAACAGGCCTTTAGCTCCTACCATAGTGTTCCTTCCTTTACCATATATGTACCCCAAATGGAG CTGCTTCACTACATTGT M GTAGAGAAAAGCATAAAGAGAAAAACTGATTCATAGATAGGATTGTCATGTATGGTTTCATAGGCTGCATGCTGCACAACTGT AGGGGGTACCATTCACA / Celera SNP ID: hCV2481732 / Public SNP ID: rs12131397 / SNP Chromosome Position: 169493953 / SNP in Genomic Sequence: SEQ ID NO: 345 / SNP Position Genomic: 22761 / Related Interrogated SNP: hCV8919444 / Related Interrogated SNP: hCV11975250 / SNP Source: Applera / Population(Allele,Count): Caucasian (A,12|C,16) African American (A,25|C,9) total (A,37|C,25) / SNP Type: INTRON / SNP Source: Applera / Population(Allele,Count): Caucasian (A,15|C,19) African American (A,25|C,9) total (A,40|C,28) / SNP Type: INTRON / SNP Source: dbSNP; Celera; HapMap / Population(Allele,Count): Caucasian (A,103|C,121) / SNP Type: INTRON /
Context (SEQ ID NO: 608): / TATTCTAGGATTTAAGGTTGATCATGACAAATAATATAAGATACTAGCTTTGCATATTCACCTTTTGGATGGTGTGTGTGTAT ACATGTGTACACACAGT R GGAAAATTAGATCTGTTTATCTGAGCAGTAACCTCCCCTCATCACTTGGCAGGAGCATTGGAGAGAGTGTTTCACTCACACAT

TAAGGTGGAAAATGGAA / Celera SNP ID: hCV2481750 / Public SNP ID: rs10800456 /
SNP Chromosome Position: 169520098 / SNP in Genomic Sequence: SEQ ID NO: 345 /
SNP Position Genomic: 48906 / Related Interrogated SNP: hCV8919444 / SNP
Source: Applera / Population(Allele,Count): Caucasian (A,20|G,6) African
American (A,15|G,11) total (A,35|G,17) / SNP Type: INTRON / SNP Source:
dbSNP; Celera; HapMap / Population(Allele,Count): Caucasian (A,163|G,63) / SNP
Type: INTRON /
Context (SEQ ID NO: 609): /
CTGCATGGAGAGTCCCTGGTTATGATAAATGCAGACTGTTAACCACACCCTTATGCATTCCTCATGAAAAGCAAGACAGACAT
TTGACAAGAAATAACCC
Y
GACTCTTCCATTTGGTGGACTTCAGATTACGAGGTTAGGGGAATGAGAAAAACTTTCAATGAAAGTACCTACTGGGTTCACAG
CTGAGTAGTAGGCCCAA / Celera SNP ID: hCV25617181 / Public SNP ID: rs9332620 /
SNP Chromosome Position: 169499951 / SNP in Genomic Sequence: SEQ ID NO: 345 /
SNP Position Genomic: 28759 / Related Interrogated SNP: hCV8919444 / SNP
Source: Applera / Population(Allele,Count): Caucasian (C,2|T,0) African
American (C,23|T,5) total (C,25|T,5) / SNP Type: INTRON / SNP Source:
dbSNP; Applera / Population(Allele,Count): Caucasian (C,167|T,59) / SNP Type:
INTRON /
Context (SEQ ID NO: 610): /
CTTTTGCATGTCCACCTGCAATATAGGAAAGCCAGTAAACAGAACTGTCCTTGTCAACAAGTCACACACTGCCTAAATTCATT
ATACAATCAGCTTTCTT
Y
AGAACTAAAGAGCTAGCACATAAGATCTTAGCAAACTGATACAATGACTACATGCAATTCATATTTGGGCACAGAGTACCTAA
ATTTGAATCCCAACTCA / Celera SNP ID: hCV25922120 / Public SNP ID: rs9332643 /
SNP Chromosome Position: 169492676 / SNP in Genomic Sequence: SEQ ID NO: 345 /
SNP Position Genomic: 21484 / Related Interrogated SNP: hCV8919444 / SNP
Source: Applera / Population(Allele,Count): Caucasian (C,30|T,4) African
American (C,28|T,4) total (C,58|T,8) / SNP Type: INTRON / SNP Source:
dbSNP; Applera / Population(Allele,Count): Caucasian (C,166|T,60) / SNP Type:
INTRON /
Context (SEQ ID NO: 611): /
AAGGTTTTTTGTTTTTGTTTTTTTTTTAGACAGGGTCTTATTCTGTTGCCCAGGCTTGAGTCCAGTGGCACAGTCATGGCTCAC
TGCAGCCTTAAACTCCT
Y
TCAAGTAATCCTCTTGCCTCAGCCTTCCAAAGTGCTGGCATTGCAGACATGAGCCATCATGCCCAACCCAGAAAATTTTTTAT
CCTATTAGCTCAAAATG / Celera SNP ID: hCV341935 / Public SNP ID: rs4656685 /
SNP Chromosome Position: 169483844 / SNP in Genomic Sequence: SEQ ID NO: 345 /
SNP Position Genomic: 12652 / Related Interrogated SNP: hCV8919444 / SNP
Source: dbSNP; HapMap / Population(Allele,Count): Caucasian (C,166|T,60) /
SNP Type: INTRON /
Context (SEQ ID NO: 612): /
GAGGATGTGAGTCTCCAAGAACTTCGGGACTTCTTTTTCATAGTACCAGCTCTTCTTTTTCATCAAAGGTCATAAATAGTAAGAC
AAATTCTCTCATGTCCA
Y
AGGCATGTTGCTGTCCTTATGTAGTATTCCTTTTTTGGCAGATTAGGAGGGGACCTATCAAGCCTGAGTGAATATCTTTTTTCCT
GGAAAAACAGAGTAAAT / Celera SNP ID: hCV342590 / Public SNP ID: rs6030 / SNP
Chromosome Position: 169498975 / SNP in Genomic Sequence: SEQ ID NO: 345 /
SNP Position Genomic: 27783 / Related Interrogated SNP: hCV8919444 / SNP
Source: Applera / Population(Allele,Count): Caucasian (C,8|T,30) African
American (C,5|T,29) total (C,13|T,59) / SNP Type: MISSENSE MUTATION;ESE / SNP
Source: dbSNP; Celera; HapMap; ABI_Val / Population(Allele,Count): Caucasian
(T,158|C,66) / SNP Type: MISSENSE MUTATION;ESE /
Context (SEQ ID NO: 613): /
CTGGGTGTCTCAGAAGCATCTCATGTCTAATCTTGTGAATATCTAAGGGCAGAAATCACTGTGACCCAGTGTGATTTAATTAG
GAGATTAGATCAAAGTC
Y
GAGGAAAATACCGTGAAACTCATGGGATTTTTTCATTTCTGAGGATGTGAGTCTCCAAGAACTTCGGGACTTCTTTTTCATAGT
ACCAGCTCTTCTTTTCA / Celera SNP ID: hCV8919431 / Public SNP ID: rs6009 / SNP
Chromosome Position: 169498834 / SNP in Genomic Sequence: SEQ ID NO: 345 /
SNP Position Genomic: 27642 / Related Interrogated SNP: hCV11975250 / SNP
Source: Applera / Population(Allele,Count): Caucasian (C,31|T,1) African
American (C,26|T,6) total (C,57|T,7) / SNP Type: INTRON / SNP Source:

dbSNP; HapMap; HGBASE / Population(Allele,Count): Caucasian (T,16|C,210) / SNP Type: INTRON /
Context (SEQ ID NO: 614): /
AGTGTCTTGGCTTAGGTGTCTCCCAACTTTATGTGCTAGTAATTTCATCCAGGAGAACCTGTGCTTTGCTGCTTGATCTCTTT
CTACCTTGGGTCCCTTA
Y
GCTTAGCATGTTCTTGACTTTTGAATTCTCCAGCACCAAGTGAAAGTAGACGTATCCCTGTGACATCTGGCTGTAGAGGATCC
TCTATAGGGTCTTCAGA / Celera SNP ID: hCV8919442 / Public SNP ID: rs4525 / SNP
Chromosome Position: 169511734 / SNP in Genomic Sequence: SEQ ID NO: 345 /
SNP Position Genomic: 40542 / Related Interrogated SNP: hCV8919444 / SNP
Source: Applera / Population(Allele,Count): Caucasian (C,3|T,25) African
American (C,1|T,19) total (C,4|T,44) / SNP Type: MISSENSE MUTATION / SNP
Source: dbSNP; HapMap; HGBASE / Population(Allele,Count): Caucasian
(T,167|C,59) / SNP Type: MISSENSE MUTATION /
Context (SEQ ID NO: 615): /
GGTGAGAAGGGGCTTTCTGAGGTTCTGCAAGGTTATTGACAGTGAACTTACTAATATTACTTGGGGAAGAATAATTTGAACCA
ACAATTATATCTGTGTT
Y
GAAGAAACGAATTCAGTGCCATTCTCCAGAGCTAGGGCAGTAAGATTGAACTCTTCTTCTTCCTGATTCAATGATGAGTTTCG
GAATGACCTGATTCCTA / Celera SNP ID: hCV8919446 / Public SNP ID: rs6021 / SNP
Chromosome Position: 169512027 / SNP in Genomic Sequence: SEQ ID NO: 345 /
SNP Position Genomic: 40835 / Related Interrogated SNP: hCV8919444 / SNP
Source: Applera / Population(Allele,Count): Caucasian (C,8|T,30) African
American (C,4|T,26) total (C,12|T,56) / SNP Type: SILENT RARE CODON;SILENT
MUTATION / SNP Source: dbSNP; Applera / Population(Allele,Count): Caucasian
(T,85|C,29) / SNP Type: SILENT RARE CODON;SILENT MUTATION /
Context (SEQ ID NO: 616): /
AAGAATAATTTGAACCAACAATTATATCTGTGTTTGAAGAAACGAATTCAGTGCCATTCTCCAGAGCTAGGGCAGTAAGATTG
AACTCTTCTTCTTCCTG
R
TTCAATGATGAGTTTCGGAATGACCTGATTCCTAATGCTGCAGCCAGTCTGTTCTGGTAATCATAGTCAGCATCACTCTCTTC
ATCTTCAGGTTCTAAAC / Celera SNP ID: hCV8919450 / Public SNP ID: rs6017 / SNP
Chromosome Position: 169512093 / SNP in Genomic Sequence: SEQ ID NO: 345 /
SNP Position Genomic: 40901 / Related Interrogated SNP: hCV8919444 / SNP
Source: Applera / Population(Allele,Count): Caucasian (A,32|G,8) African
American (A,29|G,5) total (A,61|G,13) / SNP Type: ESE;SILENT MUTATION / SNP
Source: dbSNP; Applera / Population(Allele,Count): Caucasian (A,167|G,59) /
SNP Type: ESE;SILENT MUTATION /
Context (SEQ ID NO: 617): /
CTGTGTTTGAAGAAACGAATTCAGTGCCATTCTCCAGAGCTAGGGCAGTAAGATTGAACTCTTCTTCTTCCTGATTCAATGAT
GAGTTTCGGAATGACCT
R
ATTCCTAATGCTGCAGCCAGTCTGTTCTGGTAATCATAGTCAGCATCACTCTCTTCATCTTCAGGTTCTAAACGATCATGCAT
TTTCCGTGTAGCCATGA / Celera SNP ID: hCV8919451 / Public SNP ID: rs6016 / SNP
Chromosome Position: 169512120 / SNP in Genomic Sequence: SEQ ID NO: 345 /
SNP Position Genomic: 40928 / Related Interrogated SNP: hCV8919444 / SNP
Source: Applera / Population(Allele,Count): Caucasian (A,8|G,32) African
American (A,5|G,27) total (A,13|G,59) / SNP Type: ESS;SILENT MUTATION / SNP
Source: dbSNP; HGBASE / Population(Allele,Count): Caucasian (G,165|A,61) /
SNP Type: ESS;SILENT MUTATION /
Context (SEQ ID NO: 618): /
TCACTGAAAATGTAGTGAATGCTTACACAGGTATAGTAGATTATATTTTAAAATCTAGAATAGCTTACTTTAGAATCAGGGTT
CTTTCTGGGTTTTTGAA
Y
GTGAGCGGTTAGCAAAAATGGCGGAAGTAAACTTTGTTATAAAAGCAAGTTATAATCGTGGGTCTGGACACAAGATTTTGAAA
GAAATTACCAGAACTAG / Celera SNP ID: hCV70275 / Public SNP ID: rs4656687 / SNP
Chromosome Position: 169505158 / SNP in Genomic Sequence: SEQ ID NO: 345 /
SNP Position Genomic: 33966 / Related Interrogated SNP: hCV8919444 / SNP
Source: dbSNP; Celera; HapMap; ABI_Val; HGBASE / Population(Allele,Count):
Caucasian (T,157|C,69) / SNP Type: INTRON /
Context (SEQ ID NO: 619): /
ATTTTTCATAAGAAAATGCTAGAGAGAAATGATATGATTTAGGGTTAAACAATATGACAGTTTGTCTGGGTTGTGTTTCTATG
GTTTTGACTCAACAATT

S
CTACCAGAGGAACGAATCTCCAGAACTTTGGAAACTTACCCACAGGATGAGGGGACAGAATGACCAGTCATGGTTCCCTGTGT
ACTCACCATGTGAATCA / Celera SNP ID: hCV325211 / Public SNP ID: rs3753305 /
SNP Chromosome Position: 169554058 / SNP in Genomic Sequence: SEQ ID NO: 345 /
SNP Position Genomic: 82866 / Related Interrogated SNP: hCV11975250 / SNP
Source: dbSNP; Celera; HapMap; ABI_Val; HGBASE / Population(Allele,Count):
Caucasian (C,112|G,114) / SNP Type: INTRON /
Context (SEQ ID NO: 620): /
AATGGTGGAAGGAGCATGGCCTGTTTACTGGGAGAATGGGATTCTAGTCCTGATTCTGCTACCAAACTAAGCTAAAATTATTT
AATCTCCTGTGTATTTA

S
AATTAAAAGGAAAAATCTAGACCAGATGCTTTGCTCCTTGCTACTGTAACATTCTGTGATTCCATATTGGGCCAGAATGAGTT
TCAAGACACAGATATCA / Celera SNP ID: hCV328321 / Public SNP ID: rs9332667 /
SNP Chromosome Position: 169486501 / SNP in Genomic Sequence: SEQ ID NO: 345 /
SNP Position Genomic: 15309 / Related Interrogated SNP: hCV8919444 / SNP
Source: dbSNP; Celera; HapMap / Population(Allele,Count): Caucasian
(G,87|C,31) / SNP Type: INTRON /
Context (SEQ ID NO: 621): /
TGAGGTGCGAGAATCGCCTGAGCCCAGGAAGTGGAGGTTGCAGTGAGCCATGATCACACCACTGCACTCCAGCCTGGGTGACA
GAGTAAGACCCTTGGTG

R
GGGGGAAAAGCTACTTGCTTGAGAGGCACAGACAAATAGCCAGATCCTGAACTTTTAATAAGGTATGTCTACACTTGCTGAGA
GCACATAAAAGGGATCA / Celera SNP ID: hCV337817 / Public SNP ID: rs9332586 /
SNP Chromosome Position: 169520688 / SNP in Genomic Sequence: SEQ ID NO: 345 /
SNP Position Genomic: 49496 / Related Interrogated SNP: hCV8919444 / Related
Interrogated SNP: hCV11975250 / SNP Source: dbSNP; Celera; HapMap /
Population(Allele,Count): Caucasian (G,127|A,99) / SNP Type: INTRON /
Context (SEQ ID NO: 622): /
CTTTCTTCTGGTCTACCTCCCCTACTAATCCCATCTTTCCAGACTCTGAGCATAACATGCAAACTCACAGAACACAAGGGAGT
GGGTAAAGCAACTCCGA

M
TGCCATAAAAGTGGGTTGTGAGCCTTGAATGGAATACAAGATTTTGAAGGTGGTTCCATCCCTATTCACTCTGGACAGGCCCT
GCATCTCACTCCCTCGG / Celera SNP ID: hCV340605 / Public SNP ID: rs1557572 /
SNP Chromosome Position: 169508576 / SNP in Genomic Sequence: SEQ ID NO: 345 /
SNP Position Genomic: 37384 / Related Interrogated SNP: hCV8919444 / SNP
Source: dbSNP; Celera; HapMap / Population(Allele,Count): Caucasian
(A,81|C,39) / SNP Type: INTRON /
Context (SEQ ID NO: 623): /
TTGCTGCAGGGTTATCTGGGAAATAATTGGATAGCATTCTCTCTCTATTTCACCTGTTTTACAAAACACAACTTCTCTTTCCC
CCTGTTAATGTCAAAGA

K
CAGAAATAGTTCAATTTCTTCTAATATTTCAAATAAATGTAACATTTGAGAGACCTGATAAAACCATAAGTAGAGCTTGATAC
ACAGTACAGTTATTGTC / Celera SNP ID: hCV2481727 / Public SNP ID: rs6670407 /
SNP Chromosome Position: 169485400 / SNP in Genomic Sequence: SEQ ID NO: 345 /
SNP Position Genomic: 14208 / Related Interrogated SNP: hCV8919444 / Related
Interrogated SNP: hCV11975250 / SNP Source: dbSNP; Celera; HapMap /
Population(Allele,Count): Caucasian (G,102|T,124) / SNP Type: INTRON /
Context (SEQ ID NO: 624): /
CTTTCTAGTGGCCTAATTTAAGGATAGGCCCTGGAATTCTATAGAAAGCCAGGAAATTGGAGTGTAGGTATCTTAAGGGAGGG
AGATATGAGGATAGAAA

K
TAGGGAATTTGGATTTAGTTGGCTGAAGGGATCATCAAGTTATTCATGCCATTCTGATATCTCAGGTCATTTAAAGAATTAAT
AAAAATGAGTCAAATAT / Celera SNP ID: hCV2481728 / Public SNP ID: rs9332665 /
SNP Chromosome Position: 169486767 / SNP in Genomic Sequence: SEQ ID NO: 345 /
SNP Position Genomic: 15575 / Related Interrogated SNP: hCV8919444 / SNP
Source: dbSNP; Celera; HapMap / Population(Allele,Count): Caucasian
(T,80|G,38) / SNP Type: INTRON /
Context (SEQ ID NO: 625): /
CTGATAAGACCCTGTGAATCTGGCTGAAATACTACATGTGTGGTCAAACCCAGAAAAGATGACTGAGGAGAGTTTTGGAAATG
GGCGCGTGGTCATAAGA

S
TAAGGTATCTGATCCTTGCCAAATCCCTGCAGAGAGGCTGGGCTTTTTATGTAGAGAAATCAATGACCCCCAGCTATCGGTTT
TTCCTGGAAAAGACTCT / Celera SNP ID: hCV2481731 / Public SNP ID: rs9332640 /

SNP Chromosome Position: 169493726 / SNP in Genomic Sequence: SEQ ID NO: 345 / SNP Position Genomic: 22534 / Related Interrogated SNP: hCV8919444 / Related Interrogated SNP: hCV11975250 / SNP Source: dbSNP; Celera; HapMap / Population(Allele,Count): Caucasian (G,104|C,122) / SNP Type: INTRON / Context (SEQ ID NO: 626): /
GCAAGGAGATATGCAGCATGTCACCTTAAGGCCTTCTAAATTCAGAGAAAGTTTCAGGCAAATTAGGAAGTCTAGGAGTTTAT AAAAATTAGGAGTTTATC
R
TTGGTGTATCCAGCAACCATAGCAAGTCATGTTTATTCTCTTATTGGTATGACAACTCCTCTGCCCACTTCTCAGATTAGATA ACTAGGGTTCTCAGGCT / Celera SNP ID: hCV2481733 / Public SNP ID: rs9332627 / SNP Chromosome Position: 169497820 / SNP in Genomic Sequence: SEQ ID NO: 345 / SNP Position Genomic: 26628 / Related Interrogated SNP: hCV8919444 / SNP Source: dbSNP; Celera; HapMap / Population(Allele,Count): Caucasian (G,163|A,59) / SNP Type: INTRON /
Context (SEQ ID NO: 627): /
AGTAGTGTACCCTGTACCCAATAGGTAGCTTTTCATCCATCCCCCGTTCCCTTTTGAGTCTCCAATGTCCATTATACCAATCT GTCTGCCTTCGCATACC
Y
GCAGTTTATCTCCCATTTGTAAGTGAGAACATACAGTGTCTGGTTTTTGATTCCTGAGTTACTTAGAATAAGTTTTTTAAATA TTGTGTTAAATTATCGT / Celera SNP ID: hCV2481738 / Public SNP ID: rs4656187 / SNP Chromosome Position: 169504829 / SNP in Genomic Sequence: SEQ ID NO: 345 / SNP Position Genomic: 33637 / Related Interrogated SNP: hCV8919444 / SNP Source: dbSNP; Celera; HapMap; HGBASE / Population(Allele,Count): Caucasian (C,89|T,31) / SNP Type: TRANSCRIPTION FACTOR BINDING SITE;INTRON /
Context (SEQ ID NO: 628): /
TATTCACTCTGGACAGGCCCTGCATCTCACTCCCTCGGGGCCTTGCTTAGAAATACTCAGGTAGCTAGTTGTTCTCATGTGGT ATTGAGTGCAACATTTA
W
ATAGGAAGTCATAGGAAAAGGTGTTTTAAACAGAGTTCTAATGTGGAGATGTCAGGCATCAGATTAATGAACTCATATGCATA AGTCACACCATACATTG / Celera SNP ID: hCV2481741 / Public SNP ID: rs3766109 / SNP Chromosome Position: 169508739 / SNP in Genomic Sequence: SEQ ID NO: 345 / SNP Position Genomic: 37547 / Related Interrogated SNP: hCV8919444 / SNP Source: dbSNP; Celera; HapMap; ABI_Val; HGBASE / Population(Allele,Count): Caucasian (A,89|T,31) / SNP Type: TRANSCRIPTION FACTOR BINDING SITE;INTRON /
Context (SEQ ID NO: 629): /
TGAGCTCTATCCCCACAAATAAGAGACATTTAGATGTTTCTAAGTAATAATTCACGAGGACAATTTTGAATCTCTGAAATGGC ATGCATTGCTGGAACAT
Y
AAAGATTTCTTGGTGAGTCTCCAATAGTTACCATTGTTGGTGTCTACTGAGTGCTAGGCACTTGGCGCATATTACTATTTTAA TGGTCAAAGTAACTCTG / Celera SNP ID: hCV2481744 / Public SNP ID: rs9332600 / SNP Chromosome Position: 169512913 / SNP in Genomic Sequence: SEQ ID NO: 345 / SNP Position Genomic: 41721 / Related Interrogated SNP: hCV8919444 / SNP Source: dbSNP; Celera / Population(Allele,Count): Caucasian (C,167|T,59) / SNP Type: INTRON /
Context (SEQ ID NO: 630): /
AGGGACACTGCTGTAGGCAATATTTTTATTTACTTATGTACTAAGCATAGTAAAAAACCAAAGGTATGATTTCTACAAGGGCA GGGCTCATGTCTACCTC
S
ATTACCACTCTTAGCCTTAGTACCAGTATAATGACTGGCATTAACAAATATTTAAATACCTACTTAAATCACTTAAGTGACTT TAATAATTTACATGATG / Celera SNP ID: hCV2481747 / Public SNP ID: rs9332595 / SNP Chromosome Position: 169514355 / SNP in Genomic Sequence: SEQ ID NO: 345 / SNP Position Genomic: 43163 / Related Interrogated SNP: hCV8919444 / SNP Source: dbSNP; Celera / Population(Allele,Count): Caucasian (G,173|C,51) / SNP Type: INTRON /
Context (SEQ ID NO: 631): /
ACATTTCTCTCAGACTGCCATGGCAGTGCTTTTGGCCACTGAACTTTAGAACTGCAGCAAATGCCGGAACCCTCATTCAGGGA ATTCCTTTTGTTCTGAA
M
ATCTTACTGATCACTTGAAATGTCTTCATGCATGCCTTTCCAAGACTCTTGGGTCCCTATACTCATTTTGCTCTACTTTGATG TGTCAATCCATCTTTGG / Celera SNP ID: hCV2481748 / Public SNP ID: rs3766110 / SNP Chromosome Position: 169515183 / SNP in Genomic Sequence: SEQ ID NO: 345 / SNP Position Genomic: 43991 / Related Interrogated SNP: hCV8919444 / SNP Source: dbSNP; Celera; HGBASE / Population(Allele,Count): Caucasian

(A,173|C,51) / SNP Type: INTRON /
Context (SEQ ID NO: 632): /
GATTTTATTTAAATCTTCAAATTTGTGGATAGTTTGATAATTTCTGAATCCTATACTAGGCCTGTGGGCTCAGGATGTAAACT
AATGCCAATAGTCCCTA
Y
CCCTGAGAAAAACATAATTAGGTCAGTTAAACCCACTGAAAAAAAGTCCATGCAAGCTGTTCAATTATTTGTTTAGCCTGAATA
ATTAAAGCTTGACCTCT / Celera SNP ID: hCV8919424 / Public SNP ID: rs974793 /
SNP Chromosome Position: 169478654 / SNP in Genomic Sequence: SEQ ID NO: 345 /
SNP Position Genomic: 7462 / Related Interrogated SNP: hCV8919444 / SNP
Source: dbSNP; Celera; HGBASE / Population(Allele,Count): Caucasian
(C,166|T,60) / SNP Type: INTERGENIC;UNKNOWN /
Context (SEQ ID NO: 633): /
GAGTAGGTATGTCAGAGAAAGCTTAACTGGGCCCTGAATTTGAGCATAAAGCTCTTATTTATCCTGGTTTTTTATTGATCCTTA
TCCCAGTCCTCTCTTCT
Y
TGGAGTTCTGCACAGGTTGCCTTCATACACATCTAATATCAGATGACCTAGGTTAAATCATCTCCAAAAGTTTTCACAGTCTG
ATTCCATCCCACAAGGC / Celera SNP ID: hCV8919425 / Public SNP ID: rs970740 /
SNP Chromosome Position: 169479974 / SNP in Genomic Sequence: SEQ ID NO: 345 /
SNP Position Genomic: 8782 / Related Interrogated SNP: hCV11975250 / SNP
Source: dbSNP; HapMap / Population(Allele,Count): Caucasian (C,9|T,111) / SNP
Type: INTERGENIC;UNKNOWN /
Context (SEQ ID NO: 634): /
TATTGATCCTTATCCCAGTCCTCTCTTCTCTGGAGTTCTGCACAGGTTGCCTTCATACACATCTAATATCAGATGACCTAGGT
TAAATCATCTCCAAAAG
Y
TTTCACAGTCTGATTCCATCCCACAAGGCTACTCATCTAATTGTCTTTTTCTTTACTACTCAGGGTGTAATCTGTTAGGCTTA
ATAGGCTCATAGTGTCT / Celera SNP ID: hCV8919429 / Public SNP ID: rs970741 /
SNP Chromosome Position: 169480045 / SNP in Genomic Sequence: SEQ ID NO: 345 /
SNP Position Genomic: 8853 / Related Interrogated SNP: hCV8919444 / SNP
Source: dbSNP; HapMap / Population(Allele,Count): Caucasian (T,166|C,60) /
SNP Type: INTERGENIC;UNKNOWN /
Context (SEQ ID NO: 635): /
TGAAATGGAGGAGTTGTTTCTCTTTTTAATTGACAGATAAAATTGTATGCATATACTGTGTATGACATATTGTTTTGAAGTAT
ATATACATTGCAGAATG
W
CAGATAAAGGAGTCTTGACTTTGCAGTTCTTTTCATAAAGAAAGAGCAGAACATAGCTAATACTTGTTCAAGAAAATTTCAAA
TAAATGCCATCTTCTGT / Celera SNP ID: hCV8919436 / Public SNP ID: rs916438 /
SNP Chromosome Position: 169499659 / SNP in Genomic Sequence: SEQ ID NO: 345 /
SNP Position Genomic: 28467 / Related Interrogated SNP: hCV8919444 / SNP
Source: dbSNP; Celera; HapMap; HGBASE / Population(Allele,Count): Caucasian
(T,157|A,69) / SNP Type: TRANSCRIPTION FACTOR BINDING SITE;INTRON /
Context (SEQ ID NO: 636): /
GGTTAATTAGCTTGATTTAGCCAATTCACAATTTATAAATATATTAAAACATCATGTTTACACCATAAATGTGTATAAAACAA
CAACAACAAAAAAACTG
K
GGTACACCCATATAATGGAATACATGCAATAACCAGATGAATCTCCAGAGAATTATACTGAGTGAAAAAATACCAGTCCCCAA
AGGTTACATGTTGCCTG / Celera SNP ID: hCV8919438 / Public SNP ID: rs1557570 /
SNP Chromosome Position: 169507844 / SNP in Genomic Sequence: SEQ ID NO: 345 /
SNP Position Genomic: 36652 / Related Interrogated SNP: hCV8919444 / SNP
Source: dbSNP; Celera; HapMap; HGBASE / Population(Allele,Count): Caucasian
(G,73|T,33) / SNP Type: INTRON /
Context (SEQ ID NO: 637): /
ATTTCATAGCTACCTTTCTCAGAAGCCAAATGCCATCTCCCAACCAAAATCTTAGATGAGTTACTTTGTTTTTAAGAGTAACAG
ATCACTAGGAGGGTCCT
Y
CCAGGGCCTCATTCTGGAAGGAGAACCAGTGTCTTGGCTAGGAAGGTCCTCCCAGGGCCTCATTCCGGAAGGAGAACCAGTGT
CTTGGCTTAGGTGTCTC / Celera SNP ID: hCV8919441 / Public SNP ID: rs6032 / SNP
Chromosome Position: 169511555 / SNP in Genomic Sequence: SEQ ID NO: 345 /
SNP Position Genomic: 40363 / Related Interrogated SNP: hCV8919444 / SNP
Source: dbSNP; HapMap; HGBASE / Population(Allele,Count): Caucasian
(T,89|C,31) / SNP Type: MISSENSE MUTATION;ESS;ESE SYNONYMOUS /
Context (SEQ ID NO: 638): /
AACTCAGAATTTTCAAATGAGAGATTATTCTCCTAAATTAGCCAAAGGAGAAATCTCAGGAAAAAGATAAAGCCTTCCATTTA

CATTGATTTAGTCATTC
R
TCAAATGTATAGTAAATATTTTGTGCTCCATTCTCTGCCTTTAAAGATATTCTAATAATTCCACTTGGAGGAAAAGCATAATA
TAAGAAATTCATCTTTT / Celera SNP ID: hCV8919452 / Public SNP ID: rs1018827 /
SNP Chromosome Position: 169514006 / SNP in Genomic Sequence: SEQ ID NO: 345 /
SNP Position Genomic: 42814 / Related Interrogated SNP: hCV11975250 / SNP
Source: dbSNP; HGBASE / Population(Allele,Count): Caucasian (A,17|G,207) /
SNP Type: INTRON /
Context (SEQ ID NO: 639): /
TTCTTTAACTTGGTGTCTGAGGGGTTTTTTGTCTGTGGCTTGTCCTGCTACAATTTGACCTAGCAATTCTATTACTGGGTATAT
AGCCAAAAAAGAAAATA

M
ATTGTTCAGCCGGGCGCAATGGCTCATGCCTGTAATCCCAGCACTTTGGGAAGCTGAGGCAGGCAGATTGCCCGAGGTCAGGA
GTTTGAGACCAGCCTGG / Celera SNP ID: hCV9945852 / Public SNP ID: rs1121789 /
SNP Chromosome Position: 169503616 / SNP in Genomic Sequence: SEQ ID NO: 345 /
SNP Position Genomic: 32424 / Related Interrogated SNP: hCV8919444 / SNP
Source: dbSNP; Celera; HapMap; HGBASE / Population(Allele,Count): Caucasian
(A,79|C,29) / SNP Type: INTRON /
Context (SEQ ID NO: 640): /
AGCTCCTGCACAGCAAAAGAAACTATCAAGAGAGTAAACAGACAACATTCAGAATGGGAGAAAATTTTTGCAAACTATGCATC
TGGCAAAGGTCTAATAT

S
GAGCATTTATAAGGAACTTAAATTTACAAGAAAAAAACAAACAGCCCCATTAAAAAGTAGGCCAAGGACAGGAACAGACACTT
TTCAAAAGAAGACACAC / Celera SNP ID: hCV11342057 / Public SNP ID: rs10919186 /
SNP Chromosome Position: 169516700 / SNP in Genomic Sequence: SEQ ID NO: 345 /
SNP Position Genomic: 45508 / Related Interrogated SNP: hCV8919444 / SNP
Source: dbSNP; Celera / Population(Allele,Count): Caucasian (G,176|C,50) /
SNP Type: MISSENSE MUTATION;ESE;INTRON /
Context (SEQ ID NO: 641): /
GTTCTAATTTGATTTCTCTGTGGTCTGAGAGATTGTTATGAATTTCGTTGATAAAATTTTATATATTTATGGTATACATAACA
TTTTGATATATGTACAC

R
TTACAGGATGATTAAATCAAGCTAAGTAACAAATCCATAATCTCACATAAATTTTTTTTTGGTTGCTCTTTTAACACTAAGTTT
TGGGGTGGTTTATTTCA / Celera SNP ID: hCV11975196 / Public SNP ID: rs2040444 /
SNP Chromosome Position: 169482436 / SNP in Genomic Sequence: SEQ ID NO: 345 /
SNP Position Genomic: 11244 / Related Interrogated SNP: hCV8919444 / SNP
Source: dbSNP; HapMap / Population(Allele,Count): Caucasian (G,59|A,59) / SNP
Type: MICRORNA;UTR3 /
Context (SEQ ID NO: 642): /
ATATGTATTTTAAAAGCAAATTAACTGAATTAGAATGATTATATGAGAAAACTTTTAAGTCTATAACATCCTCTAGTGACATC
TGGGTGGTATCTTAGTG

W
GTCTTCATATCAAGGTTGTACCAGTGATGCAGAGTATAACCAGCTAGAGGTTTTCACAGGCATAAAAGAGGTAGAGACATTTT
AGTTATGGAGAGAACAG / Celera SNP ID: hCV15802102 / Public SNP ID: rs2420369 /
SNP Chromosome Position: 169485859 / SNP in Genomic Sequence: SEQ ID NO: 345 /
SNP Position Genomic: 14667 / Related Interrogated SNP: hCV8919444 / SNP
Source: dbSNP; Celera; HapMap; HGBASE / Population(Allele,Count): Caucasian
(T,58|A,58) / SNP Type: INTRON /
Context (SEQ ID NO: 643): /
AGACTGAAAAAGCCCAAATAAAATCTGCTTTCTCTAACCAAAGTACCAGTAAAGGGCAGCATAGCGAGATATGATAGAAAACT
GGTGGACACTGACTTCT

S
TCCTTTAGTCAAACACCACAGAAAAACCTGAGGCCCCTCCCCAACCCACAACAGCAAAGGCTGAATGGGGAGCCTAGACTTCT
ATGCTCCTGAGGCTAAA / Celera SNP ID: hCV15802103 / Public SNP ID: rs2420370 /
SNP Chromosome Position: 169490392 / SNP in Genomic Sequence: SEQ ID NO: 345 /
SNP Position Genomic: 19200 / Related Interrogated SNP: hCV11975250 / SNP
Source: dbSNP; HapMap; HGBASE / Population(Allele,Count): Caucasian
(G,9|C,109) / SNP Type: INTRON;PSEUDOGENE /
Context (SEQ ID NO: 644): /
GGCCCAGATGGTTTCACTAGTGATTCCTACCAAATATTTAAAGAATTATTACCAATTGCAGACATTTTCTCCTAGAATGTAGA
AAAGAGCATGCTTACCA

R
TTTTATGAAAGCTAGTATAACTGTGGTATCAAAAAACAGAAAAAGACAATTAAAAAAAATAAAACTATATACCAATATCCTCTCA

TGAACATAGACGACAAA / Celera SNP ID: hCV15802110 / Public SNP ID: rs2420371 /
SNP Chromosome Position: 169491555 / SNP in Genomic Sequence: SEQ ID NO: 345 /
SNP Position Genomic: 20363 / Related Interrogated SNP: hCV11975250 / SNP
Source: dbSNP; HapMap; HGBASE / Population(Allele,Count): Caucasian
(G,14|A,210) / SNP Type: INTRON /
Context (SEQ ID NO: 645): /
ACCTTCCAACATTCTCTTTTGCTCTTAACGGAATGGAAATCTTAGAAATGTTGATGGGACAATGACATGAATCATGAAAAGAA
AGGAATAGTGGGAATAC
R
AATATTAGAAAGCCAATGTTTTGTGGATGTTTGAAACTCTCATATACATTCATAGGGCTTACCCCACTGGTATGGGCAACAGG
TAACTCTGGTAGTTTCT / Celera SNP ID: hCV15847759 / Public SNP ID: rs2187952 /
SNP Chromosome Position: 169481950 / SNP in Genomic Sequence: SEQ ID NO: 345 /
SNP Position Genomic: 10758 / Related Interrogated SNP: hCV8919444 / SNP
Source: dbSNP; Celera; HapMap; HGBASE / Population(Allele,Count): Caucasian
(G,88|A,30) / SNP Type: UTR3 /
Context (SEQ ID NO: 646): /
ATTCCAAATTAGAGCCCTTCCTTGGAGAGTTGTGATGTGTCTATATAATCCAGGCACTTTCTTCACAGAGATGCTGTCGGCAC
TCTGATTGGCAGAACCA
Y
TCTTGGTCTAGATCACACTGAGAGTTTACCTGAGTAGAACCTCTGTTTCACAAAGGTTTTCCTAGGAGCCTAAGTCACTGAAA
AGAACTAAAAATTCTAC / Celera SNP ID: hCV15852051 / Public SNP ID: rs2213867 /
SNP Chromosome Position: 169489585 / SNP in Genomic Sequence: SEQ ID NO: 345 /
SNP Position Genomic: 18393 / Related Interrogated SNP: hCV8919444 / SNP
Source: dbSNP; Celera; HapMap; ABI_Val; HGBASE / Population(Allele,Count):
Caucasian (T,93|C,27) / SNP Type: INTRON /
Context (SEQ ID NO: 647): /
TGAGTACCTACACAGGGTATGAAAGAAAATATAAAGAAGAATTGTGGTTTCAGGAAGTTGGGAAGCCAATTCAGATTTCAAAA
ACACTCATTGAACACCA
Y
ATATATGGCAGTGATGTTGCCAGATACTGTCATGACATTGAAGTTTGAGTGACCTGAGGACTTTGGAAGAGTCAGGCCTAGTT
TGAATCTCAGGTAGGTC / Celera SNP ID: hCV15955265 / Public SNP ID: rs2227244 /
SNP Chromosome Position: 169489358 / SNP in Genomic Sequence: SEQ ID NO: 345 /
SNP Position Genomic: 18166 / Related Interrogated SNP: hCV8919444 / SNP
Source: dbSNP; HapMap; HGBASE / Population(Allele,Count): Caucasian
(T,87|C,31) / SNP Type: INTRON /
Context (SEQ ID NO: 648): /
AAAGACATGAAAACACAATAATCCATTGTGGCAGGTAATTTCTAATAAACTTTCGTCAGGAATATTCAAGTATGGGCTGTGGA
CCACTGCTTGTCTGTGA
M
CTATTGATTACTGGGTCAAAATAACACAAGGAGAGAAAAATGAGAATGAAATATTTAGTAAACTTTTGTTTTTCTAAAGCAAT
TTGACATTGCTGTGACT / Celera SNP ID: hCV16175730 / Public SNP ID: rs2239851 /
SNP Chromosome Position: 169512497 / SNP in Genomic Sequence: SEQ ID NO: 345 /
SNP Position Genomic: 41305 / Related Interrogated SNP: hCV8919444 / SNP
Source: dbSNP; HapMap; ABI_Val; HGBASE / Population(Allele,Count): Caucasian
(C,167|A,59) / SNP Type: INTRON /
Context (SEQ ID NO: 649): /
GTGGCAGGTAATTTCTAATAAACTTTCGTCAGGAATATTCAAGTATGGGCTGTGGACCACTGCTTGTCTGTGACCTATTGATT
ACTGGGTCAAAATAACA
Y
AAGGAGAGAAAAATGAGAATGAAATATTTAGTAAACTTTTGTTTTTCTAAAGCAATTTGACATTGCTGTGACTCTAAGCACAT
GACCAGTGCGTTCATCT / Celera SNP ID: hCV16175731 / Public SNP ID: rs2239852 /
SNP Chromosome Position: 169512524 / SNP in Genomic Sequence: SEQ ID NO: 345 /
SNP Position Genomic: 41332 / Related Interrogated SNP: hCV8919444 / SNP
Source: dbSNP; HGBASE / Population(Allele,Count): Caucasian (C,155|T,69) /
SNP Type: INTRON /
Context (SEQ ID NO: 650): /
AGTCCTAGTTAGGCTCTTTAATCTGCAAAAGAGAACCTTAATCCTATCTTCTATTTGGTTGATTGTCAAAGCCTTTGGATCAT
CCTTTGTCTGTAGATTA
W
CTACACTCTAGGATTTTGTCAAAGATTGCAACCTTTAATTCTCTGCTGTCCAATTCTTATCTATCTTGGTGAAGTGACATGAC
ATTTAAGGAGAATTGTC / Celera SNP ID: hCV16191269 / Public SNP ID: rs2298909 /
SNP Chromosome Position: 169520993 / SNP in Genomic Sequence: SEQ ID NO: 345 /
SNP Position Genomic: 49801 / Related Interrogated SNP: hCV8919444 / SNP

Source: dbSNP; HapMap; HGBASE / Population(Allele,Count): Caucasian
(A,81|T,33) / SNP Type: INTRON /
Context (SEQ ID NO: 651): /
CTCTCTAGATGTATTAGTGAGTTACTGGCATGTGAAAAACCAATAAGAACCCTCTTTTTTTCCCTGCTCTTAATCAAACCAGT
CCATTTGGACTTCTGTG
Y
CTGCATCTGAGGCAGATTCACACAACGTCACCCAAAGTGCTGTCGAGGGGCTGCCTTCACTAACTCCTCAGGCAGCATGTGTT
AGGTTGGTGCAAACTAT / Celera SNP ID: hCV27242809 / Public SNP ID: rs9332630 /
SNP Chromosome Position: 169496253 / SNP in Genomic Sequence: SEQ ID NO: 345 /
SNP Position Genomic: 25061 / Related Interrogated SNP: hCV8919444 / SNP
Source: dbSNP; Celera; HapMap / Population(Allele,Count): Caucasian
(C,60|T,60) / SNP Type: INTRON /
Context (SEQ ID NO: 652): /
ATGACAATGATATTTATCCTCCTATGAGGGCAACCTGGTGTAGTGAGAAATAAAACAAACCAAAACAGACAACCAGGAGTTTG
TCTGAGACCAGGCACCT
K
AAGAACTAAGATTTAGAAGACTTAAAGAGGTGGTACATGTCACTGCATATTTGTCAAATGCAAAATACTGTTATTCTCATTAT
AGCACAGTCTTCAGATT / Celera SNP ID: hCV27490260 / Public SNP ID: rs3820060 /
SNP Chromosome Position: 169484552 / SNP in Genomic Sequence: SEQ ID NO: 345 /
SNP Position Genomic: 13360 / Related Interrogated SNP: hCV8919444 / SNP
Source: dbSNP; HGBASE / Population(Allele,Count): Caucasian (T,157|G,69) /
SNP Type: TRANSCRIPTION FACTOR BINDING SITE;INTRON /
Context (SEQ ID NO: 653): /
TTTCACTATAATTTGGCTTTTCTGTAATGTCATATCAATGGAATCATCATTACACACCCTTTTGTGTTGGATTAGTGTTAGCT
GGTATGGTATATAATTT
Y
CTATCCTTTATTTTTTACCTATCTTTGTCTCTCTCATTAAAGTGGATTTCTTAAATTCTGCATACAGTTTAGCATTGCTTTTT
TATTCAATCTTATTTGT / Celera SNP ID: hCV27886241 / Public SNP ID: rs4656690 /
SNP Chromosome Position: 169531571 / SNP in Genomic Sequence: SEQ ID NO: 345 /
SNP Position Genomic: 60379 / Related Interrogated SNP: hCV11975250 / SNP
Source: dbSNP; HapMap / Population(Allele,Count): Caucasian (T,79|C,19) / SNP
Type: INTRON /
Context (SEQ ID NO: 654): /
AATAATGTTTAGAATTTTATCTGTTGGTTTCTCTTAAGTCATGATTCTACCGTGAGTCATTTAACTTCTTTCCAAAGCACCCA
GTTGTAACAAACAGTGG
W
TTATGTCACTGGAGACGTACTATGTAGTCAAACTTGTAATTTCATCTAAGTGAGATCAGAGAGAGAAATAAAAAGTGGGTATC
TTTTTTATTTCAAAATA / Celera SNP ID: hCV29397247 / Public SNP ID: rs6427194 /
SNP Chromosome Position: 169481121 / SNP in Genomic Sequence: SEQ ID NO: 345 /
SNP Position Genomic: 9929 / Related Interrogated SNP: hCV11975250 / SNP
Source: dbSNP; HapMap / Population(Allele,Count): Caucasian (T,9|A,111) / SNP
Type: TRANSCRIPTION FACTOR BINDING SITE;INTERGENIC;UNKNOWN /
Context (SEQ ID NO: 655): /
GTCATTTAACTTCTTTCCAAAGCACCCAGTTGTAACAAACAGTGGTTTATGTCACTGGAGACGTACTATGTAGTCAAACTTGT
AATTTCATCTAAGTGAG
W
TCAGAGAGAGAAATAAAAAGTGGGTATCTTTTTTTATTTCAAAATAACAGGGGTTTTAATATTTCTACACAGCATTAGATAATA
GCCCAAACATATTATTG / Celera SNP ID: hCV29397248 / Public SNP ID: rs6427195 /
SNP Chromosome Position: 169481176 / SNP in Genomic Sequence: SEQ ID NO: 345 /
SNP Position Genomic: 9984 / Related Interrogated SNP: hCV11975250 / SNP
Source: dbSNP; HapMap / Population(Allele,Count): Caucasian (A,16|T,210) /
SNP Type: INTERGENIC;UNKNOWN /
Context (SEQ ID NO: 656): /
TAAAGTACTGACATTATCTACTAATGAAATGAGAGTGATAGGTAGTGGAGATGTGAAGATGAAAGAAGCTTGATTCTTGTTCA
TAAGAAGCTTTTAAGCT
M
CTGCTGAAACAGACATGCACATGGGCATCCACACTACAAATCTGGAGACAACAGGTGCTATATACAAAGTCTAAGTGGGTTCA
GATGACAATGCCCTGGA / Celera SNP ID: hCV29397252 / Public SNP ID: rs6427197 /
SNP Chromosome Position: 169500590 / SNP in Genomic Sequence: SEQ ID NO: 345 /
SNP Position Genomic: 29398 / Related Interrogated SNP: hCV11975250 / SNP
Source: dbSNP; HapMap / Population(Allele,Count): Caucasian (C,16|A,210) /
SNP Type: INTRON /
Context (SEQ ID NO: 657): /

TCTTTGATACCTTCTTGCTTTGGGGAACAGGCTTTATTTCATCCAGCTTGGGAAGAAAACATTTGGTGACTCTCAAGACTCTG
TACAAGTATCCATTTCC
Y

CCAGTCTCAATAGAAGAGTATATAGATACGTTAGCTCAGTTGGTAAAAGCATCATGCCCCAAGTTTAATGCTGTCATCTTATT
TGTATATGAAAGGGGGC / Celera SNP ID: hCV29397255 / Public SNP ID: rs6427202 /
SNP Chromosome Position: 169528830 / SNP in Genomic Sequence: SEQ ID NO: 345 /
SNP Position Genomic: 57638 / Related Interrogated SNP: hCV11975250 / SNP
Source: dbSNP; HapMap / Population(Allele,Count): Caucasian (C,106|T,120) /
SNP Type: INTRON /
Context (SEQ ID NO: 658): /
TTATTTTTATTAACATTGTGAATTTACATTGTTGTTTGTTGCATTCTTGTTAACTAGTATTGAGTTTTATTCACAGTTAAGAT
ACTTGAAATCAAATCTT
R

TTTTTTATTCTGTTGCCTCCTCAGCAGCCTGTAGTCTAATTTTATCCACCCACTGTTTCTAAATCCTTCTGACAATTCTATCC
AGTGCTCATTTATTAGG / Celera SNP ID: hCV32141457 / Public SNP ID: rs6678795 /
SNP Chromosome Position: 169533266 / SNP in Genomic Sequence: SEQ ID NO: 345 /
SNP Position Genomic: 62074 / Related Interrogated SNP: hCV11975250 / SNP
Source: dbSNP; HapMap / Population(Allele,Count): Caucasian (G,117|A,107) /
SNP Type: INTRON /
Context (SEQ ID NO: 659): /
CAAGCAAGTCCTCCTTCCCCTGCCCTTCTCTTTTCAGCTTGGGCCACATCTCATTTTGAATCTGCTTCTCATCTCTAGACCAT
GATCCCCTTCCCCTGCC
Y

GGTAGATTTTTTAGGACACTGTCTTTGAAGTCATCTTCTCAGCTAGGTTCAGTGGCTCAAACCTGTAACCCCAGCACTTTGGG
AGGCTGAAGCAGGTGGA / Celera SNP ID: hCV30144962 / Public SNP ID: rs10158595 /
SNP Chromosome Position: 169520364 / SNP in Genomic Sequence: SEQ ID NO: 345 /
SNP Position Genomic: 49172 / Related Interrogated SNP: hCV8919444 / SNP
Source: dbSNP; HapMap / Population(Allele,Count): Caucasian (C,174|T,52) /
SNP Type: INTRON /
Context (SEQ ID NO: 660): /
ATTAGAGAAATGCAAATCAAAACCACAATGAGATACCATCTCATACCAGTTAGAACTGTGATTATTAAAAAGTCAGGAAACAG
ATGCTGGCAAGGCCGTG
R

AGAAATAGGAATGCTTTTACACTGTGGGTAGGGGTGTAAATTAATTCCACCATTGTGGAAGACAGTGTGGTGATTCCTCAAGG
ATATAGAATCAGAAATA / Celera SNP ID: hCV32141406 / Public SNP ID: rs10737547 /
SNP Chromosome Position: 169476052 / SNP in Genomic Sequence: SEQ ID NO: 345 /
SNP Position Genomic: 4860 / Related Interrogated SNP: hCV11975250 / SNP
Source: dbSNP / Population(Allele,Count): Caucasian (A,8|G,108) / SNP Type:
INTERGENIC;UNKNOWN /
Context (SEQ ID NO: 661): /
AGAGGCACCCTACACCCTCACTGGGGTTGTGTCAGAAAAGGCCATGTAGGGATTTAGGGCTTTCATCTCCAGCCCCTAATGAA
GCTCCTGCCCCTGCAGT
R

TCAGTGGAGGCCAAATGGGGAACTTCCCTTCCCACCCAGCCACCAAGGAACAATCCCTTCTTAGGTGGCAATGGAGTGGGGAA
CCTGGACTTTGACCCCC / Celera SNP ID: hCV30433255 / Public SNP ID: rs9332655 /
SNP Chromosome Position: 169490592 / SNP in Genomic Sequence: SEQ ID NO: 345 /
SNP Position Genomic: 19400 / Related Interrogated SNP: hCV8919444 / SNP
Source: dbSNP; HapMap / Population(Allele,Count): Caucasian (A,82|G,34) / SNP
Type: SILENT RARE CODON;SILENT MUTATION;INTRON /
Context (SEQ ID NO: 662): /
GGAACCTGGACTTTGACCCCCACTTGGCAGTAAACAGGCTGTGCCCTACCCCTTTTCCTGACAGAGCAATGTCAGAAAAAGCC
AACTTTTTAAAACAGAA
R

ATTGGAGCAGGTATCGGTAATACTGAAAACAGAAAAACAATAGAGAAAAATCAATAAAACAAGGCTGGTTCTGTGAAAAGATT
TTTTTTTTTTTTTTTTTT / Celera SNP ID: hCV30036717 / Public SNP ID: rs9332653 /
SNP Chromosome Position: 169490772 / SNP in Genomic Sequence: SEQ ID NO: 345 /
SNP Position Genomic: 19580 / Related Interrogated SNP: hCV8919444 / SNP
Source: dbSNP; HapMap / Population(Allele,Count): Caucasian (G,177|A,47) /
SNP Type: ESE SYNONYMOUS;SILENT MUTATION;INTRON /
Context (SEQ ID NO: 663): /
TGACCTATTGATTACTGGGTCAAAATAACACAAGGAGAGAAAAATGAGAATGAAATATTTAGTAAACTTTTGTTTTTCTAAAG
CAATTTGACATTGCTGT
R

ACTCTAAGCACATGACCAGTGCGTTCATCTCGCTTAACAGAATAAAAAACAAAAGTGTTGCTTAGTGGAGATTAGAGGGGAAA
AAAGGTCTTTCAGCACA / Celera SNP ID: hCV30234691 / Public SNP ID: rs6662593 /
SNP Chromosome Position: 169512594 / SNP in Genomic Sequence: SEQ ID NO: 345 /
SNP Position Genomic: 41402 / Related Interrogated SNP: hCV8919444 / SNP
Source: dbSNP / Population(Allele,Count): Caucasian (G,166|A,60) / SNP Type:
INTRON /
Context (SEQ ID NO: 664): /
GCTTATGATAGAATCTTGTGCAAGAACATAACAATTTTGTAAAAATGCAGTTGTTACACCTTGCAGAATACACAATCTTGAGC
TCATTGTCACAGAGTAG
M
TAACAAAATCATGCCTTAGAGAAACAGTATTTATGATATGAGCCTTTGCCTGAAGGAAAATGATTTTTAGAGGCTGTAAAATC
TACCTTTGGCTTAAGCC / Celera SNP ID: hCV29748285 / Public SNP ID: rs6687813 /
SNP Chromosome Position: 169477574 / SNP in Genomic Sequence: SEQ ID NO: 345 /
SNP Position Genomic: 6382 / Related Interrogated SNP: hCV11975250 / SNP
Source: dbSNP; HapMap / Population(Allele,Count): Caucasian (A,15|C,207) /
SNP Type: INTERGENIC;UNKNOWN /
Context (SEQ ID NO: 665): /
TTTTAAAACGAACCTAGGAAAAGGAATGATCCACAAATGTACTTAAGCTTAACAAAAATCTAAACCACAAAAATGTCAGCATT
ATAAGTCAAAATGGCTG
R
TTATAAAATCGGAGCTAAGATATAAGGTACAAAATAACTTATGTATATTATTTCATTTAATTTTCATAACCATCCTGTGAGAT
ATGGCCCAAGTAGTTTT / Celera SNP ID: hCV29621699 / Public SNP ID: rs9332619 /
SNP Chromosome Position: 169500348 / SNP in Genomic Sequence: SEQ ID NO: 345 /
SNP Position Genomic: 29156 / Related Interrogated SNP: hCV8919444 / SNP
Source: dbSNP / Population(Allele,Count): Caucasian (G,167|A,59) / SNP Type:
INTRON /
Context (SEQ ID NO: 666): /
TGGTAAAGGACTTAAATGCCATATGAGAAGTCTTCACACCCCAGCAGTCAGACCCATGAAGCTCCTCCCAGCCCTTCTATAAC
TTCTAGTGATTAAAGAC
Y
CCCTAATTGTCTGTTCCTTATTTTATTCTACAAAGCCAGCATCACCCTAATAACAAAACCTGGCAAAGACACAATGAATAATG
AAAACTACCGGCCAATA / Celera SNP ID: hCV30018856 / Public SNP ID: rs6701330 /
SNP Chromosome Position: 169501056 / SNP in Genomic Sequence: SEQ ID NO: 345 /
SNP Position Genomic: 29864 / Related Interrogated SNP: hCV8919444 / SNP
Source: dbSNP; HapMap / Population(Allele,Count): Caucasian (T,66|C,36) / SNP
Type: INTRON /
Context (SEQ ID NO: 667): /
GTCACCTGGCTGAGGTCTGGGGAAAGGGACATCTGACCAAAGTTTGGGGAAAGATCTGTCTCACCAAGGTCTGGAGAAAGTGT
CATCTGGTCGAGGTCTG
R
GGTAAGGGGAATTTGACTGAGATCTGCAAAGAGGGGCATCTCACTGAGGTCTGGGGAAAGGTTTGTCTGACTGAGTTCTGGAG
AGAGGTTTGTCTGGCTG / Celera SNP ID: hCV30504827 / Public SNP ID: rs9332608 /
SNP Chromosome Position: 169510118 / SNP in Genomic Sequence: SEQ ID NO: 345 /
SNP Position Genomic: 38926 / Related Interrogated SNP: hCV8919444 / SNP
Source: dbSNP / Population(Allele,Count): Caucasian (G,215|A,11) / SNP Type:
MISSENSE MUTATION;ESE SYNONYMOUS //

Gene Number: 9 / Gene Symbol: F9 - 2158 / Gene Name: coagulation factor IX
/ Chromosome: X / OMIM NUMBER: 306900 / OMIM Information: Hemophilia B
(3); Warfarin sensitivity (3) // Genomic Sequence (SEQ ID NO: 346): // / SNP
Information /
Context (SEQ ID NO: 668): /
TGAGAAATATCAGGTTACTAATTTTTCTTCTATTTTTCTAGTGCCATTTCCATGTGGAAGAGTTTCTGTTTCACAAACTTCTA
AGCTCACCCGTGCTGAG
R
CTGTTTTTCCTGATGTGGACTATGTAAATTCTACTGAAGCTGAAACCATTTTGGATAACATCACTCAAAGCACCCAATCATTT
AATGACTTCACTCGGGT / Celera SNP ID: hCV596331 / Public SNP ID: rs6048 / SNP
Chromosome Position: 138633280 / SNP in Genomic Sequence: SEQ ID NO: 346 /
SNP Position Genomic: 98317 / SNP Source: dbSNP; HapMap; /
Population(Allele,Count): Caucasian (A,126|G,41) / SNP Type: MISSENSE
MUTATION;ESE;INTRON /
Context (SEQ ID NO: 669): /
AGATTATTTCTTTTTCCGACGTTTCAGTAATTGGAGCAGTAAACCCCAGTGTCCCTTACCTACTTGTTTATTACCTCCAGATG

CAATATTACTGGTACTG
W
GATTGAGAAACGCACACAGTGCTAATGAGGAATTCACTTTCTACTCTGACACTCTGGAAGAATAGAGATGCAATCCTAAGGAA
GAATTTAACACCACAGG / Celera SNP ID: hCV2986566 / Public SNP ID: rs4149755 /
SNP Chromosome Position: 138624112 / SNP in Genomic Sequence: SEQ ID NO: 346 /
SNP Position Genomic: 89149 / SNP Source: dbSNP; Celera; HGBASE /
Population(Allele,Count): Caucasian (A,12|T,104) / SNP Type: INTRON /
Context (SEQ ID NO: 670): /
CTTAGAACCTAATGAAAGTTTGCATTCCTCAGTAAAATCAGAGACTGCTGATTGACTTAAATGTTTATAGCTTCAAAGTCCTC
CTCATTATCATGGCCCA
S
AAGCCCTTCCATGATTGTCCTTCCCCACCCTCCCCATTACCCTTCTTGCCTCCTCTGCTACTTCTCTCCTCGCACACTGGGCT
CCAGCCACCCTGGCCTT / Celera SNP ID: hCV596323 / Public SNP ID: rs438601 / SNP
Chromosome Position: 138619919 / SNP in Genomic Sequence: SEQ ID NO: 346 /
SNP Position Genomic: 84956 / Related Interrogated SNP: hCV596331 / SNP
Source: dbSNP; HapMap; ABI_Val; HGBASE / Population(Allele,Count): Caucasian
(C,71|G,19) / SNP Type: INTRON /
Context (SEQ ID NO: 671): /
CATATAATGGGAATTCTCCACATGTACAAACCACTTCATATGCTAAACTTGTTGACAACATTCAAAGCTCATCCCTGAATTTG
ACTATATTGATTACATC
R
AAAATGTTACATAGCAACCTTAGAATCCTTGTGTACCTTTTCTTCTCAAAGCCTAGATTATTTCTTTTTCCGACGTTTCAGTA
ATTGGAGCAGTAAACCC / Celera SNP ID: hCV596326 / Public SNP ID: rs398101 / SNP
Chromosome Position: 138623957 / SNP in Genomic Sequence: SEQ ID NO: 346 /
SNP Position Genomic: 88994 / Related Interrogated SNP: hCV596331 / SNP
Source: dbSNP; HGBASE / Population(Allele,Count): Caucasian (A,69|G,31) / SNP
Type: INTRON /
Context (SEQ ID NO: 672): /
CTAGAAGGCCTTTTAGTCTGCAAAAGAAACCTTCTTAATCATAAGCAGCAGAAGTCCCATTTACCAAATTGGAAAGTTAAAGT
TACAAAGCATCAATCAT
M
AGACTTCCATTCAGGGATGGCAATTGGGAGTAAGACTTTTTAGTAAAGAAACTAAACACAAAGTCATTAGACTCTGTAAAAGT
CTTACCAAATTTGATTC / Celera SNP ID: hCV596330 / Public SNP ID: rs422187 / SNP
Chromosome Position: 138632859 / SNP in Genomic Sequence: SEQ ID NO: 346 /
SNP Position Genomic: 97896 / Related Interrogated SNP: hCV596331 / SNP
Source: dbSNP; HapMap; HGBASE / Population(Allele,Count): Caucasian
(A,125|C,44) / SNP Type: INTRON /
Context (SEQ ID NO: 673): /
GCCAGAGATCAGAGCAGGCTAAGGGACTGCTGGGATCCTGTCCAGCTTTGAGACCCTACAGAGCCATGTTCACCTAGCACGTA
TCCCGTCTGCGGTCACG
S
TCATTTCTTACCTTATTCCAGGGCTTTCACCTCAGCTTGCCAGGCTGGAGCCAAGGGCCAACGCAGCCGCGCCTTGTTCGCGA
TGGTAGCTTCCCAGGAG / Celera SNP ID: hCV596335 / Public SNP ID: rs413957 / SNP
Chromosome Position: 138637516 / SNP in Genomic Sequence: SEQ ID NO: 346 /
SNP Position Genomic: 102553 / Related Interrogated SNP: hCV596331 / SNP
Source: dbSNP; HapMap; ABI_Val; HGBASE / Population(Allele,Count): Caucasian
(C,73|G,17) / SNP Type: INTRON /
Context (SEQ ID NO: 674): /
TACAGAAAATGTCCAGGGAAATGGTCTATTTCTTATTCTATTTTTGACCTAAAGAAAATCTTTAAAATGTCTTAGCATTTTCC
CCAGTCTCCATCCACTT
Y
CCTCAGCTTTGGCCTGAAGCTATCTTTAAAGGTACCCTGTACAGCTCTTGCCCTGTACAGCTAGCTACAGAGATTCAATCCTT
TCTGTTCGATTAGGACA / Celera SNP ID: hCV596336 / Public SNP ID: rs110583 / SNP
Chromosome Position: 138638531 / SNP in Genomic Sequence: SEQ ID NO: 346 /
SNP Position Genomic: 103568 / Related Interrogated SNP: hCV596331 / SNP
Source: dbSNP; HGBASE / Population(Allele,Count): Caucasian (T,141|C,27) /
SNP Type: INTRON /
Context (SEQ ID NO: 675): /
GCCACAGTAGGCCAGTGACAAGGGAAGATTGACACATCATCCCCTGCTGGGGCCCAGTGTCCTGTGGCTGGCAGGCAGGGGAT
CCTAAGGACATGTGGGT
S
TTAAATTGTAGGGTGCACTTCCTGGGCACCTTTGAGGGTCTGCACTGCCCCAGCAAATATCCCCATGCTAGAAGGAGCAAAAT
ATTAAATGGCAAATTTT / Celera SNP ID: hCV596337 / Public SNP ID: rs421766 / SNP

Chromosome Position: 138640592 / SNP in Genomic Sequence: SEQ ID NO: 346 / SNP Position Genomic: 105629 / Related Interrogated SNP: hCV596331 / SNP Source: dbSNP; HapMap; HGBASE / Population(Allele,Count): Caucasian (G,141|C,27) / SNP Type: INTRON / Context (SEQ ID NO: 676): / ATGGTTAAGAGAGAGTGGAAAGAATGAATGAGCCCTGCTATTCCTCACTGCCTGGATGGCTATAAGCACAGCCCTTATGGAGG CCTTAGGTCTTGCTTCA Y

AATATTCCAGTTTGAAAAGGGTTTGAAAAGACCTCCTAGAAAAATCAGTAGTTTTTCTCTTTTGAGTAACATGTAGCAAAAAA AATTTCATCATGTAGGT / Celera SNP ID: hCV596339 / Public SNP ID: rs370713 / SNP Chromosome Position: 138642393 / SNP in Genomic Sequence: SEQ ID NO: 346 / SNP Position Genomic: 107430 / Related Interrogated SNP: hCV596331 / SNP Source: dbSNP; HapMap; ABI_Val; HGBASE / Population(Allele,Count): Caucasian (T,142|C,27) / SNP Type: INTRON / Context (SEQ ID NO: 677): / AAAGGGTTTGAAAAGACCTCCTAGAAAAATCAGTAGTTTTTCTCTTTTGAGTAACATGTAGCAAAAAAAAATTTCATCATGTAG GTACAGGGAACACCCTA R

TAACTATTAATCTCAAGGAGTCAAGCCAGTGTGTTTCCTAATGTATCTGCTGTATCCCCATGAAGCAAATTTTGCCATCAGAG AAACTGACTCATGGGGA / Celera SNP ID: hCV596340 / Public SNP ID: rs413536 / SNP Chromosome Position: 138642509 / SNP in Genomic Sequence: SEQ ID NO: 346 / SNP Position Genomic: 107546 / Related Interrogated SNP: hCV596331 / SNP Source: dbSNP; HapMap; ABI_Val; HGBASE / Population(Allele,Count): Caucasian (A,143|G,24) / SNP Type: INTRON / Context (SEQ ID NO: 678): / GCTAGACAGTAGTTGCTCAATAATTGTTAGCTGAATCAGAATCCATGTTTATCCCAGAGTAGCAATTAGTCTTGCATCGAGTA TCGTGAAAGAAGGCCAC R

CTTAAATAAGAATAATGCCTGGGGTTTAGGTTTTATGAAAAAATGAAAGGAAATTAGTTCTGCTTTTGTTGACTAAAGGAAGG GAAGAGAGAAGAGACTA / Celera SNP ID: hCV596344 / Public SNP ID: rs445691 / SNP Chromosome Position: 138646746 / SNP in Genomic Sequence: SEQ ID NO: 346 / SNP Position Genomic: 111783 / Related Interrogated SNP: hCV596331 / SNP Source: dbSNP; HapMap; HGBASE / Population(Allele,Count): Caucasian (G,142|A,27) / SNP Type: INTERGENIC;UNKNOWN / Context (SEQ ID NO: 679): / TAAAGTGAACAGCTGCAATGAAAATAAGGGAAGAAAGTTTAGTTCATCTCCGTTTCTTTCCTTTCCTTTTTACTTTCCTTTCT CTTCCTTTTTGGAGTTA R

TCAGGAAGTAGTCCCAAATACCCCAGAAAGTTCATCTTATAAGCCCTTGGTCCTCTTGAGATGGTATCAGATATATTGCTAGA CCCTTGAAGAAAGGAAC / Celera SNP ID: hCV596669 / Public SNP ID: rs376165 / SNP Chromosome Position: 138617733 / SNP in Genomic Sequence: SEQ ID NO: 346 / SNP Position Genomic: 82770 / Related Interrogated SNP: hCV596331 / SNP Source: dbSNP; HapMap; ABI_Val; HGBASE / Population(Allele,Count): Caucasian (A,114|G,55) / SNP Type: TFBS SYNONYMOUS;INTRON / Context (SEQ ID NO: 680): / AATTAGAGGGTCACTGATGGTCGAGAGGAAAAGGAGAAAGACAGTTGGCAAACTATTTATTTTTGACTCTAACATGAGACTTT ATAAGTTTCCCATGTCA R

AACTCTTTACAAGGTCATCACTTTGGTCAATTTCAAATTGATTTGTTATTAAACATGTGCTGCCTATACTGTTCCAACATGTA ATTCTTTCTCCTTCTGC / Celera SNP ID: hCV1264349 / Public SNP ID: rs12557491 / SNP Chromosome Position: 138536879 / SNP in Genomic Sequence: SEQ ID NO: 346 / SNP Position Genomic: 1916 / Related Interrogated SNP: hCV2986566 / SNP Source: dbSNP; Celera; HapMap / Population(Allele,Count): Caucasian (G,140|A,25) / SNP Type: INTERGENIC;UNKNOWN / Context (SEQ ID NO: 681): / CCTTTTTACCCTCCATGGTCGTTAAAGGAGAGATGGGGAGCATCATTCTGTTATACTTCTGTACACAGTTATACATGTCTATCA AACCCAGACTTGCTTCC R

TAGTGGAGACTTGCTTTTCAGAACATAGGGATGAAGTAAGGTGCCTGAAAAGTTTGGGGGAAAAGTTTCTTTCAGAGAGTTAA GTTATTTTATATATATA / Celera SNP ID: hCV2288124 / Public SNP ID: rs440051 / SNP Chromosome Position: 138644917 / SNP in Genomic Sequence: SEQ ID NO: 346 / SNP Position Genomic: 109954 / Related Interrogated SNP: hCV596331 / SNP Source: dbSNP; HapMap; ABI_Val; HGBASE; / Population(Allele,Count): Caucasian

(G,142|A,27) / SNP Type: TRANSCRIPTION FACTOR BINDING SITE;UTR3 /
Context (SEQ ID NO: 682): /
ACAGTGGTCTGAATCCACCTGAGACAGAATTGGGTCTAACTAACTGTGAGTATGGCCTTCAATAAGTCACTCTCCATTTGGGA
ATTTGATTTCTCCACTT
S
TATAATGAGAGTATTTGACAGGATGCTCTCCCAAATCCCTTGCAATTTTGTTAGTCTGTGATTTCATGTTTTTATTTTTATTC
CTTCATCCAACAAATAG / Celera SNP ID: hCV2969899 / Public SNP ID: rs434144 /
SNP Chromosome Position: 138646425 / SNP in Genomic Sequence: SEQ ID NO: 346 /
SNP Position Genomic: 111462 / Related Interrogated SNP: hCV596331 / SNP
Source: dbSNP; Celera; HapMap; ABI_Val; HGBASE / Population(Allele,Count):
Caucasian (C,135|G,29) / SNP Type: INTERGENIC;UNKNOWN /
Context (SEQ ID NO: 683): /
CAGGATGCTCTCCCAAATCCCTTGCAATTTTGTTAGTCTGTGATTTCATGTTTTTATTTTTATTCCTTCATCCAACAAATAGT
CAAGGAGTAATTGCTGT
S
TGCCAAATACCAACAGTATTCATTAAATTGTAATTCAGATTTTATATATATATAAATAATGTATAATGTGTATAAATTGCTTT
GTGAGTGCCTACTACAC / Celera SNP ID: hCV2969900 / Public SNP ID: rs434447 /
SNP Chromosome Position: 138646544 / SNP in Genomic Sequence: SEQ ID NO: 346 /
SNP Position Genomic: 111581 / Related Interrogated SNP: hCV596331 / SNP
Source: dbSNP; Celera; HapMap; HGBASE / Population(Allele,Count): Caucasian
(C,73|G,17) / SNP Type: INTERGENIC;UNKNOWN /
Context (SEQ ID NO: 684): /
AGAACTATTTCAAACTCCTGGCCAGGTCATTCCACTCTAATAGGAGAGCTATCCTTCTATTCTCTTGGTTAAGAGAAAGCTGG
AAATAGAAACAGGGATA
Y
TCCAGGCCAGACACAATGGCTCACGCCTGTAATCCCAACACTTTGGGAGGCCGAGGCAGGCAGATCACTTGAGGTCAGGAGTT
CAAGATTAGTCTGGCCA / Celera SNP ID: hCV2986569 / Public SNP ID: rs11095801 /
SNP Chromosome Position: 138648820 / SNP in Genomic Sequence: SEQ ID NO: 346 /
SNP Position Genomic: 113857 / Related Interrogated SNP: hCV596331 / SNP
Source: dbSNP; Celera; HapMap / Population(Allele,Count): Caucasian
(T,73|C,17) / SNP Type: INTERGENIC;UNKNOWN /
Context (SEQ ID NO: 685): /
GACCTCATAAAGATAAAGAGTTCCCATGATTTACACATTACGTGCTTTCATAAATATCTATATATAAAAGCCTATTTTCCTCT
TGGACTATATTACAAAA
R
TAAGTATGCATTTTCATAAGATTCAAACCCAGCTCTAAATGTAAGAAGCCAAATTAAGAATGTAGCAATGTATGAATGAAAAG
GAAGGAAAAAAGCCATT / Celera SNP ID: hCV2986570 / Public SNP ID: rs3117458 /
SNP Chromosome Position: 138649479 / SNP in Genomic Sequence: SEQ ID NO: 346 /
SNP Position Genomic: 114516 / Related Interrogated SNP: hCV596331 / SNP
Source: dbSNP; Celera; HapMap; ABI_Val; HGBASE / Population(Allele,Count):
Caucasian (G,72|A,18) / SNP Type: TRANSCRIPTION FACTOR BINDING
SITE;INTERGENIC;UNKNOWN /
Context (SEQ ID NO: 686): /
ATACTTGATTCAAACCTATTTCTGTCTGATCTGATTCTAAAGTCTGTTTTTTCACTCAACCACACTGTACAGTCAGCTCTCCT
TGTGAGTTCCACAGCCA
M
AGATTCAATTAACTGCAGATCAAAAATATTCAAGAAAAAAATGGATGGTTGCATCTCTACTGAACATGTACAGACTCTTTTAT
CTTTCATTATTCCCTAA / Celera SNP ID: hCV2986572 / Public SNP ID: rs4149670 /
SNP Chromosome Position: 138616642 / SNP in Genomic Sequence: SEQ ID NO: 346 /
SNP Position Genomic: 81679 / Related Interrogated SNP: hCV596331 / SNP
Source: dbSNP; Celera; HGBASE / Population(Allele,Count): Caucasian
(A,31|C,69) / SNP Type: INTRON /
Context (SEQ ID NO: 687): /
TCTCCTTGTGAGTTCCACAGCCACAGATTCAATTAACTGCAGATCAAAAATATTCAAGAAAAAAATGGATGGTTGCATCTCTA
CTGAACATGTACAGACT
Y
TTTTATCTTTCATTATTCCCTAAACAATACAGCATAACAACTATTTACATAGCATTTACATTGTATTAGCTATTAAGAGAAAC
CTAGAGATGATTTAAAG / Celera SNP ID: hCV2986574 / Public SNP ID: rs4149672 /
SNP Chromosome Position: 138616719 / SNP in Genomic Sequence: SEQ ID NO: 346 /
SNP Position Genomic: 81756 / Related Interrogated SNP: hCV596331 / SNP
Source: dbSNP; Celera; HGBASE / Population(Allele,Count): Caucasian
(C,115|T,53) / SNP Type: INTRON /
Context (SEQ ID NO: 688): /

ACTGAACATGTACAGACTCTTTTATCTTTCATTATTCCCTAAACAATACAGCATAACAACTATTTACATAGCATTTACATTGT
ATTAGCTATTAAGAGAA
W
CCTAGAGATGATTTAAAGTACAAAGGAGGATGTGTTTAGGTTATATGCAAATAGTAAGCCATTTTTATATCGGAGACTTGAGC
ATCCACAGATCTTGATA / Celera SNP ID: hCV2986575 / Public SNP ID: rs4149674 /
SNP Chromosome Position: 138616801 / SNP in Genomic Sequence: SEQ ID NO: 346 /
SNP Position Genomic: 81838 / Related Interrogated SNP: hCV596331 / SNP
Source: dbSNP; Celera; HapMap; ABI_Val; HGBASE / Population(Allele,Count):
Caucasian (A,115|T,53) / SNP Type: INTRON /
Context (SEQ ID NO: 689): /
TCATAGAGGGTAAAAGTCAAAGAGCAGAGAACCAAAAATTGGGCTAAATCTTCAATTCACAATACACATGGCCTTAAACTTCT
GCTTCAGAAGAGATTTG
Y
ACATCAAGGTTGGCTCTTCACACTAGCAGAGATACGGAATCAGCCTCCAAATTGTCACTGCTGACATGACATTTAATGGTGGG
GAAAAACCCAAGTGTCC / Celera SNP ID: hCV11780206 / Public SNP ID: rs5931656 /
SNP Chromosome Position: 138576517 / SNP in Genomic Sequence: SEQ ID NO: 346 /
SNP Position Genomic: 41554 / SNP Source: dbSNP; Celera /
Population(Allele,Count): Caucasian (T,38|C,130) / SNP Type:
INTERGENIC;UNKNOWN /
Context (SEQ ID NO: 690): /
CATAGAGTGAACAGACTCTTAAGAAACAAAAGTTGTTCTCATTTACTCTCACCGCCAGGCACACACTTAAGGGTTCTTCAATT
ACCTCACTGGTCTCTCT
Y
TTAAGTGACCTCTGCTAGCTCTTCCTTATCTCCTTGAACTTTAAGTGTTAGAGGGCACCAGGGCCTTGTTTTTGGACATTTTC
CCTTCTCTATGCTCACT / Celera SNP ID: hCV11780209 / Public SNP ID: rs5930009 /
SNP Chromosome Position: 138577105 / SNP in Genomic Sequence: SEQ ID NO: 346 /
SNP Position Genomic: 42142 / SNP Source: dbSNP; Celera; HapMap /
Population(Allele,Count): Caucasian (T,38|C,130) / SNP Type:
INTERGENIC;UNKNOWN /
Context (SEQ ID NO: 691): /
TTTCCCTTCTCTATGCTCACTCCCTTGGTGATCTCATCTGTGTCATTGCTTTAATTTCCATCTACATAGACACAACTTCTAAA
TGTTTGTGCGTGTGTGG
R
AAGGGGGATGTGATCTTTTGAGGCCCAGATATATGCATCAAACTGCCTCCTCAATATCTCCTTTTGGATATCAATAGGTGCCT
CAAATTTAACATATCCA / Celera SNP ID: hCV11780214 / Public SNP ID: rs7878061 /
SNP Chromosome Position: 138577285 / SNP in Genomic Sequence: SEQ ID NO: 346 /
SNP Position Genomic: 42322 / SNP Source: dbSNP; Celera; HapMap /
Population(Allele,Count): Caucasian (A,38|G,131) / SNP Type:
INTERGENIC;UNKNOWN /
Context (SEQ ID NO: 692): /
AGAAACACTCTAACTGCCCTCTTACCAGCAAGAGACAGGGCTCCACTCTGTGCAGAACTTTCACAAGCTGCCCCTACTACAAC
ACGCAACGTGCTCAGTG
W
TCATAACTTCCCACCCTCACTCACTCTGTACCTTTTTCCTCACTCTGATTTCTTTCATCCACTGACCATCAACCATTTCATCT
CTAAATCACTCCCTTAG / Celera SNP ID: hCV26011954 / Public SNP ID: rs4620439 /
SNP Chromosome Position: 138589486 / SNP in Genomic Sequence: SEQ ID NO: 346 /
SNP Position Genomic: 54523 / SNP Source: dbSNP; Celera; HapMap; ABI_Val;
HGBASE / Population(Allele,Count): Caucasian (T,25|A,65) / SNP Type:
INTERGENIC;UNKNOWN /
Context (SEQ ID NO: 693): /
AGGCTTGAGTAGCAGGGCACTAAAGAAGCAAGCCACTCCCCCTGTCAACCACCCTGCGAGAGGGACAAGGGAGCTTTTTCCTT
TTCACCTCTCTCTGTTT
Y
TCATCCCACTTCTTGCATTATCCCAGTGGTAGTTTCTTTTAAGATCAATCTCCACTATTGCCCTAAATTAATGTTCCCTCCAT
AAGTCTTCTTTATCAGT / Celera SNP ID: hCV26011955 / Public SNP ID: rs5930013 /
SNP Chromosome Position: 138590593 / SNP in Genomic Sequence: SEQ ID NO: 346 /
SNP Position Genomic: 55630 / SNP Source: dbSNP; Celera; HapMap /
Population(Allele,Count): Caucasian (T,38|C,131) / SNP Type:
INTERGENIC;UNKNOWN /
Context (SEQ ID NO: 694): /
GGATGGCTGAGCCAAATCTGGGAGGATAAGTCTAACACCATTTCTTCGTAATAGCTCAGTTGTTCCAATACCATTTTCTTCTC
AACCTCCAAAATACAGG
K

TACTTTATTTATTTCAGTATTCTTTGTGCATTTAACCACCATTCTCATTCCACAGGCTTTGGTGAATTAAAGTAACTACAGTT
ACCAACTCTTTTTATTG / Celera SNP ID: hCV26016182 / Public SNP ID: rs11796672 /
SNP Chromosome Position: 138587625 / SNP in Genomic Sequence: SEQ ID NO: 346 /
 SNP Position Genomic: 52662 / SNP Source: dbSNP; Celera; HapMap /
Population(Allele,Count): Caucasian (T,65|G,25) / SNP Type:
INTERGENIC;UNKNOWN /
Context (SEQ ID NO: 695): /
CCAATACCATTTTCTTCTCAACCTCCAAAATACAGGTTACTTTATTTATTTCAGTATTCTTTGTGCATTTAACCACCATTCTC
ATTCCACAGGCTTTGGT
R

AATTAAAGTAACTACAGTTACCAACTCTTTTTATTGAAAAACTCTTTATGAGTTGATTGTAACTGCTTCTCCAATTATAATTG
CTTCCTCTCCACAGGCC / Celera SNP ID: hCV26016183 / Public SNP ID: rs9887617 /
SNP Chromosome Position: 138587689 / SNP in Genomic Sequence: SEQ ID NO: 346 /
 SNP Position Genomic: 52726 / Related Interrogated SNP: hCV596331 / SNP
Source: dbSNP; Celera / Population(Allele,Count): Caucasian (G,26|A,59) / SNP
Type: INTERGENIC;UNKNOWN /
Context (SEQ ID NO: 696): /
ATGTTGTGTAAACTGTGGAAACTATTGAAAGAATCACAGCAGGCAAAGGACTGTGGGACCCCTGCTCTTTTCAATCAATCCAG
GCCCACAAATCACTCTA
S

TCATTTTTTTCCTACGGTAGTTTCGAGGCAAATCTTTTTCATCCAGTCTTTGGGGCTATGGACTGCCTTGAGATTTCTGAGTCA
ATCTCAACTTCTAATAT / Celera SNP ID: hCV26225376 / Public SNP ID: rs3117074 /
SNP Chromosome Position: 138648511 / SNP in Genomic Sequence: SEQ ID NO: 346 /
 SNP Position Genomic: 113548 / Related Interrogated SNP: hCV596331 / SNP
Source: dbSNP; Celera; HapMap; ABI_Val; HGBASE / Population(Allele,Count):
Caucasian (G,73|C,17) / SNP Type: INTERGENIC;UNKNOWN /
Context (SEQ ID NO: 697): /
TGGAAACTATTGAAAGAATCACAGCAGGCAAAGGACTGTGGGACCCCTGCTCTTTTCAATCAATCCAGGCCCACAAATCACTC
TAGTCATTTTTTCCTAC
R

GTAGTTTCGAGGCAAATCTTTTTCATCCAGTCTTTGGGGCTATGGACTGCCTTGAGATTTCTGAGTCAATCTCAACTTCTAAT
ATAGGTATCAAAAGTGA / Celera SNP ID: hCV26225377 / Public SNP ID: rs12008759 /
SNP Chromosome Position: 138648526 / SNP in Genomic Sequence: SEQ ID NO: 346 /
 SNP Position Genomic: 113563 / Related Interrogated SNP: hCV596331 / SNP
Source: dbSNP; Celera; HapMap / Population(Allele,Count): Caucasian
(G,142|A,27) / SNP Type: INTERGENIC;UNKNOWN /
Context (SEQ ID NO: 698): /
CAATGGCCTCAGGATGAGGAGTGGCAATGACACACAGCACCTAAAGATCACTGGTCCATCAACAAAAGGAAGGAAATGAGGCT
GGCATGGTAGGCAGTGT
M

AGGTTACCAACAAGCCTCTTCCACTAAGCAAATAGAAACAACAAAATATCTGCAAATTCTCTACCTGTGATGTTTGGGAAATA
TCTTCAGTGACCTGGGA / Celera SNP ID: hCV28962605 / Public SNP ID: rs5930005 /
SNP Chromosome Position: 138567672 / SNP in Genomic Sequence: SEQ ID NO: 346 /
 SNP Position Genomic: 32709 / SNP Source: dbSNP; HapMap /
Population(Allele,Count): Caucasian (C,38|A,131) / SNP Type:
INTERGENIC;UNKNOWN /
Context (SEQ ID NO: 699): /
GAACATGCAATGTTAGTCTTCCTGTGCCTGGCTTATTTCCCTTAACATAATGATCACCTCTTGCCAGCATTTGAGGTAGGGAG
AGTGTAGGCACTTATGG
R

AACCTATTGCAGTAGGATCTACCCTAGTTTTATTTTACATTTCCAACTGCCTTTTGGCAGTATCTCCATTCAAATGTTTAACC
ACCATTGTAAACCCAAC / Celera SNP ID: hCV30700232 / Public SNP ID: rs12687091 /
SNP Chromosome Position: 138564704 / SNP in Genomic Sequence: SEQ ID NO: 346 /
 SNP Position Genomic: 29741 / SNP Source: dbSNP; HapMap /
Population(Allele,Count): Caucasian (G,38|A,130) / SNP Type:
INTERGENIC;UNKNOWN /
Context (SEQ ID NO: 700): /
CTAGTTCTGTGAGTGATCCTATGTATCTAGTCACCCAGGCTTACAAATTTGCTGTCACGTTTGTTTCCTCTCTACCTTATTTC
CAACATTCAGTGAGTTA
Y

CTAGTATGATGGTTAATTTTAGGTGTCATCTGGATTAAGGAATACCCAGATAACTGGTAAAGTGTGTCTGTGAGGGTGTTTCC
AAAGGAGACTGACTCAT / Celera SNP ID: hCV30201992 / Public SNP ID: rs5931641 /
SNP Chromosome Position: 138564963 / SNP in Genomic Sequence: SEQ ID NO: 346 /

SNP Position Genomic: 30000 / SNP Source: dbSNP; HapMap /
Population(Allele,Count): Caucasian (C,37|T,131) / SNP Type:
INTERGENIC;UNKNOWN /
Context (SEQ ID NO: 701): /
CTATATCTGATCTATATCATCTATATCTACATCCTATCTGTTCTATCTTCATGAAAAACCCTGACACCAAGTCAGAACCAATT
TAGCTCTCCAATATGTC
R
CTCAGTCCTGTCGCCTCTGTTCTCTTTCCACTGCCACCAGAGTAAGTCAGGCTCACATCATCTCTACCTAGACCAGTGCAGTG
GCCTAACTACCTTTCGC / Celera SNP ID: hCV29967889 / Public SNP ID: rs5931645 /
SNP Chromosome Position: 138565455 / SNP in Genomic Sequence: SEQ ID NO: 346 /
SNP Position Genomic: 30492 / SNP Source: dbSNP; HapMap /
Population(Allele,Count): Caucasian (A,130|G,38) / SNP Type:
INTERGENIC;UNKNOWN /
Context (SEQ ID NO: 702): /
TATTAGATTTGGGTGGGGACACACATCCAAACTATATCACTGAGGAAGCCCATTCTTGTGAACGTCCAGCTTACGTTTTGAAA
CTACAAGAGAAGTAGAC
M
GTTCCAATGTACTTATATTTGTGATTTCCAGGTTTACTGCCACATAATTTTGGCCCATAAAGTGAATACTCTTTCTGATTTGG
GATTGTGCATTCATAAA / Celera SNP ID: hCV29732937 / Public SNP ID: rs5931647 /
SNP Chromosome Position: 138570327 / SNP in Genomic Sequence: SEQ ID NO: 346 /
SNP Position Genomic: 35364 / SNP Source: dbSNP; HapMap /
Population(Allele,Count): Caucasian (C,25|A,65) / SNP Type:
INTERGENIC;UNKNOWN /
Context (SEQ ID NO: 703): /
ACGTAAGAGGTGTTCAATGTTAGACTACTTCCTTCTCTCCCTGTAAGTTAGTACAAAAAGGGCAAATGAATGAGTTTATATGT
TACCTTATCATGCTTTA
M
ATCTAAACATTTCACCAAAATTCATAGGAAGAAATATAGTAAAGAAATAAACTGAAAAATCAAGCCATCTTGGAAGAAAGCTG
AAAGGATATTTTTATCT / Celera SNP ID: hCV30291634 / Public SNP ID: rs5931666 /
SNP Chromosome Position: 138589014 / SNP in Genomic Sequence: SEQ ID NO: 346 /
SNP Position Genomic: 54051 / SNP Source: dbSNP; HapMap /
Population(Allele,Count): Caucasian (A,25|C,57) / SNP Type:
INTERGENIC;UNKNOWN /
Context (SEQ ID NO: 704): /
GGATCTGATAATTAAATTGGAATTATGGCCCAGAAAATTAGGCCAGGACCTGTGTGTTAACCCTAAAAAGGGTTACTGCCCGC
TAAATAGGATCCAGAGT
Y
TGCTAACACACTTTACAAAATGTCCAGAATACACATAACAACCACCTTTCATATCAAGACGCAGGAAAATCACAATTGTAATA
AACAGAGATAGTCAAGG / Celera SNP ID: hCV29515877 / Public SNP ID: rs5930015 /
SNP Chromosome Position: 138599528 / SNP in Genomic Sequence: SEQ ID NO: 346 /
SNP Position Genomic: 64565 / SNP Source: dbSNP; HapMap /
Population(Allele,Count): Caucasian (C,130|T,39) / SNP Type:
INTERGENIC;UNKNOWN /
Context (SEQ ID NO: 705): /
TAAGGTCACATTCATAGGTTCTGGGGGTTAGGATGTAAACATATATTTTGGGGGCCACCATTCAACCCATTACATAGTGTTTA
TGTGGGGGAGAGATATC
Y
TCCCTTTTTGTCTTCCTGATTGTAATGTTAATATACGTTCATCATAAAAATATAGTGAATATAGAAAATCACAAAGAAACTTA
AAACTCATCTTTAATTT / Celera SNP ID: hCV30130033 / Public SNP ID: rs5931646 /
SNP Chromosome Position: 138568730 / SNP in Genomic Sequence: SEQ ID NO: 346 /
SNP Position Genomic: 33767 / SNP Source: dbSNP; HapMap /
Population(Allele,Count): Caucasian (C,25|T,65) / SNP Type:
INTERGENIC;UNKNOWN /
Context (SEQ ID NO: 706): /
TGAAAGTTTTACTGTTGTCAAACACGTACACAAGGGGAAAGGTGTCTTACATTGTTTATGTTCCTGTGCTGCTCTAGAAACAG
AAATAGGCTCAAGAGCA
R
AGCCTGTTTTTCTTAATTCAGCAGGTCTAAGCTAACAAGTCCTGAAACATGGTACTTCCTGTTATTGGTATTGCATAGGAGAA
ACAAAGGGAAAGCACAG / Celera SNP ID: hDV76976791 / Public SNP ID: rs4149758 /
SNP Chromosome Position: 138627477 / SNP in Genomic Sequence: SEQ ID NO: 346 /
SNP Position Genomic: 92514 / Related Interrogated SNP: hCV596331 / SNP
Source: CDX; dbSNP / Population(Allele,Count): Caucasian (G,154|A,14) / SNP
Type: INTRON /

Context          (SEQ ID NO: 707): /
TGAAAAATGGCCAAACATAAATCTTTTACCCTCAATTTATGTCATAGCAATCGATAACCAGGGTGGCTGGCTGACTTGTAATT
GGCTGTATTCTACCCAA
W
ATGTCTCTTGATTCTAGAATGCAGGGTTACAGCAACTACTTTTACAAGACCCTGAGATACATATTGAAAACCAGCTACCTATA
TAACACTAGTTTCTAAT / Celera SNP ID:  hDV77022847 / Public SNP ID:  rs4598407 /
SNP Chromosome Position:  138579809 / SNP in Genomic Sequence:  SEQ ID NO: 346 /
 SNP Position Genomic:  44846 / SNP Source:  CDX; dbSNP /
Population(Allele,Count):  Caucasian (A,65|T,25) / SNP Type:
INTERGENIC;UNKNOWN /
Context          (SEQ ID NO: 708): /
AAGGACTTATAGATACTGTGTCACTTATTCATTCATTCATTTATTCATAAATAACTTATTAAATGTTAGACAACCCAAAATAA
GCAAAGCAGAAAACGTT
Y
CTACATTCGCGTAGCTTAAAAATTGAGTGTGAGAGACAGCTATTTAAACATTATCATACTACTTCTGCTTCCAAGTAGAATGTA
ATAAGTCACAGTAAATA / Celera SNP ID:  hDV77148131 / Public SNP ID:  rs5931661 /
SNP Chromosome Position:  138580558 / SNP in Genomic Sequence:  SEQ ID NO: 346 /
 SNP Position Genomic:  45595 / SNP Source:  CDX; dbSNP /
Population(Allele,Count):  Caucasian (C,130|T,38) / SNP Type:
INTERGENIC;UNKNOWN /
Context          (SEQ ID NO: 709): /
GACACTTCTGCTGTAATAATTACTTAAATATGAATAAATTATAAAAATGGTATTTAGATGCCTGAGAGAGCTTTGATAACAAT
TAGGACTAGATGAATTA
W
ATTTCCATAGAGTAGACAACCATTCCAAGCCCTGCACCACAAAAAAATATAAAAGGAATCCTTGAGACAGAAAAAAAAAGATA
CCAGACAGGAAAATGGA / Celera SNP ID:  hDV77150953 / Public SNP ID:  rs5976389 /
SNP Chromosome Position:  138580759 / SNP in Genomic Sequence:  SEQ ID NO: 346 /
 SNP Position Genomic:  45796 / SNP Source:  CDX; dbSNP /
Population(Allele,Count):  Caucasian (A,38|T,126) / SNP Type:
INTERGENIC;UNKNOWN //

Gene Number:  10 / Gene Symbol:  F11 - 2160 / Gene Name:  coagulation factor
XI / Chromosome:  4 / OMIM NUMBER:  264900 / OMIM Information:  Factor XI
deficiency, autosomal recessive (3); Factor XI deficiency,/a / utosomal dominant
(3) // / Genomic Sequence (SEQ ID NO: 347): // / SNP Information /
Context          (SEQ ID NO: 710): /
CTGCTTCCCTGTGGGTTCCGGCTTCTGCAGAGCTGTAAGAGTTGAATGCCACACACAGTCACACTAAGGAATGCTCCAGGATT
GGGAAAGAAAATTCAAC
M
TTATAATGAGAACACTGTGAATGCTATTGAATTAACTACTCCCCTCTCTCCCTATTTCTTGTAAGTCTTAGTGTCAGTAAACT
AATTATAAATTTACATT / Celera SNP ID:  hCV25474413 / Public SNP ID:  rs3822057 /
SNP Chromosome Position:  187188152 / SNP in Genomic Sequence:  SEQ ID NO: 347 /
 SNP Position Genomic:  11034 / SNP Source:  Applera /
Population(Allele,Count):  Caucasian (A,16|C,14)  African American (A,7|C,11)
total (A,23|C,25) / SNP Type:  INTRON / SNP Source:  Applera /
Population(Allele,Count):  Caucasian (A,16|C,18)  African American (A,12|C,20)
total (A,28|C,38) // / SNP Type:  INTRON / SNP Source:  dbSNP; HGBASE /
Population(Allele,Count):  Caucasian (A,109|C,117) / SNP Type:  INTRON /
Context          (SEQ ID NO: 711): /
AGGGATGAAGGATTGAAGGTTAGAACAATTAAGCAACTTGTGCAGGATCAAAGTGAGTTGGATGAGGAGTTAGCGGTGAGGGT
GAGGCTTGTCTCTCTCT
Y
GCCCTCTCATCCTGGCACATGTGCGATATCGTGCTGAACCTGAGGGAGGAAAATACACGACAACAAGGCAAAAAATGAATATA
GTAAACAAAGAAAACAC / Celera SNP ID:  hCV3230038 / Public SNP ID:  rs2289252 /
SNP Chromosome Position:  187207381 / SNP in Genomic Sequence:  SEQ ID NO: 347 /
 SNP Position Genomic:  30263 / SNP Source:  Applera /
Population(Allele,Count):  Caucasian (C,18|T,12)  African American (C,19|T,5)
total (C,37|T,17) / SNP Type:  UTR3;INTRON / SNP Source:  dbSNP; Celera;
HGBASE / Population(Allele,Count):  Caucasian (C,132|T,94) / SNP Type:
UTR3;INTRON /
Context          (SEQ ID NO: 712): /
TATTTAACATTCCTCTCAAGCAAATACGCCTTGAAATGCTTTTTTTAAATCATAGGAATTTAAAAACACTTTACAATAGAGAA
TGATTGATTTTTAAAAT

R
TGTCTGATTTAGCTTTGTAGAGATGTTCCGCTAATATCCATAACTAATCTGAGAGGAAATGTGGAACAACAGAAGAGTAACAG
TGTCTACTCAGTAACAA / Celera SNP ID: hCV8241630 / Public SNP ID: rs925451 /
SNP Chromosome Position: 187187569 / SNP in Genomic Sequence: SEQ ID NO: 347 /
SNP Position Genomic: 10451 / SNP Source: dbSNP; Celera; HapMap; HGBASE /
Population(Allele,Count): Caucasian (G,138|A,88) / SNP Type:
INTRON;PSEUDOGENE /
Context        (SEQ ID NO: 713): /
AGGGTTTGGATAAAGAGACGCAATTAGGAAAGGAAAAAGCAGAAGGCTCGTTCCAGACCTGGATGAGATCCTAAAAAGCAGCA
GCTTTTGCCAGTAAAGA
Y
CCTTGAAATGATTCAATTACCCTCAAAGCACTCCTTGTCTCCAAGACAATCACTCATAAGCACAATTCCATTGAAGCCAACGT
ACCATTTTGTGATTTTC / Celera SNP ID: hCV12066124 / Public SNP ID: rs2036914 /
SNP Chromosome Position: 187192481 / SNP in Genomic Sequence: SEQ ID NO: 347 /
SNP Position Genomic: 15363 / SNP Source: dbSNP; HapMap; HGBASE /
Population(Allele,Count): Caucasian (T,106|C,120) / SNP Type: INTRON /
Context        (SEQ ID NO: 714): /
GCAATTTTTACAACCTGAGTTCAAGTCAAATTCTGAGCCTGGGGGGTCCTCATCTGCAAAGCATGGAGAGTGGCATCTTCTTT
GCATCCTAAGGACGAAA
R
ACACAGTGCACTCAGAGCTGCTGAGGACAATGTCTGGCTGAAGCCCGCTTTCAGCACGCCGTAACCAGGGGCTGACAATGCGA
GGTCGCAACTGAGATCT / Celera SNP ID: hCV15793897 / Public SNP ID: rs3087505 /
SNP Chromosome Position: 187179486 / SNP in Genomic Sequence: SEQ ID NO: 347 /
SNP Position Genomic: 2368 / SNP Source: dbSNP; HapMap; HGBASE /
Population(Allele,Count): Caucasian (A,20|G,206) / SNP Type: MICRORNA;UTR3 /
Context        (SEQ ID NO: 715): /
CATTGGATTCCTTACTTAGGTCACTTTCAGCGTTGGCCAAACAAAAAGGCTCCAGCTGGGGGTATATATATTCCAGGGAAGTT
AAGTTGGCCAAACTTTC
M
GTTTGCTACTTCAGTATCCTCCGAGTTGTTTCCAGACACAGTTTTGTGTGCTTTTTAGTTTTGGTTTTCTTTTTTTAATCAATC
TGCAGCACACTCATGTT / Celera SNP ID: hCV27474895 / Public SNP ID: rs3756011 /
SNP Chromosome Position: 187206249 / SNP in Genomic Sequence: SEQ ID NO: 347 /
SNP Position Genomic: 29131 / SNP Source: dbSNP; HGBASE /
Population(Allele,Count): Caucasian (A,45|C,75) / SNP Type: TRANSCRIPTION
FACTOR BINDING SITE;INTRON /
Context        (SEQ ID NO: 716): /
TTAGAGCCTTTCTGTTTCTCTCAATAGGGTTGGAGAGTTATCCTTATCTTCTTTTTATTGGGGCTTAAGAAGAGAGATGAGGT
TCCATGGAGTAAACAAT
W
CAAGGATATAAGGACCTCATATAATCTCACGTATCCATTTTCCATGAAAGCCATTCTTGGCACGAATTTGCCATTCTATGTTT
GAGCCTCATAAAAGGCA / Celera SNP ID: hCV27477533 / Public SNP ID: rs3756008 /
SNP Chromosome Position: 187185385 / SNP in Genomic Sequence: SEQ ID NO: 347 /
SNP Position Genomic: 8267 / SNP Source: dbSNP; HapMap; ABI_val; HGBASE /
Population(Allele,Count): Caucasian (A,136|T,90) / SNP Type:
INTERGENIC;UNKNOWN /
Context        (SEQ ID NO: 717): /
AAAAGTTGTCCTCCAGACAACTCAGCCAAGGGAGGCCCTGTGTGCCTTGCCTATCACATGAGCCTCACTTTCCACTGAGTGAG
GCTGTCATTTCAGAAGC
R
CCGGGTCTGTCACATGAAAATATATCTGTTACCATCACTTACTAAACAAATTTAGTAGAATTTGTTTGGTGCTTATTATGTAT
CAGGCATTGTTCTGAAG / Celera SNP ID: hCV30562347 / Public SNP ID: rs4253418 /
SNP Chromosome Position: 187199497 / SNP in Genomic Sequence: SEQ ID NO: 347 /
SNP Position Genomic: 22379 / SNP Source: dbSNP; HapMap; HGBASE /
Population(Allele,Count): Caucasian (A,12|G,212) / SNP Type: INTRON /
Context        (SEQ ID NO: 718): /
AAAGGAAGATGTAGGAAGCTGCTCATCACAATGCTTCTGTTGCAGAGTGTACCACCAAAATCAAGCCCAGGATCGTTGGAGGA
ACTGCGTCTGTTCGTGG
Y
GAGTGGCCGTGGCAGGTGACCCTGCACACAACCTCACCCACTCAGAGACACCTGTGTGGAGGCTCCATCATTGGAAACCAGTG
GATATTAACAGCCGCTC / Celera SNP ID: hCV11786301 / Public SNP ID: rs5970 / SNP
Chromosome Position: 187205301 / SNP in Genomic Sequence: SEQ ID NO: 347 /
SNP Position Genomic: 28183 / Related Interrogated SNP: hCV27474895 / Related
Interrogated SNP: hCV3230038 / Related Interrogated SNP: hCV25474413 / SNP

Source: Applera / Population(Allele,Count): Caucasian (C,4|T,34) African American (C,9|T,29) total (C,13|T,63) / SNP Type: ESE SYNONYMOUS;SILENT RARE CODON;SILENT MUTATION / SNP Source: Applera / Population(Allele,Count): Caucasian (C,5|T,31) African American (C,6|T,28) total (C,11|T,59) / SNP Type: ESE SYNONYMOUS;SILENT RARE CODON;SILENT MUTATION / SNP Source: dbSNP; Celera; Applera / Population(Allele,Count): Caucasian (T,187|C,33) / SNP Type: ESE SYNONYMOUS;SILENT RARE CODON;SILENT MUTATION /
Context (SEQ ID NO: 719): /
CGAGTGGTCGATGAAAAACAGAATCGTGATTTACTAAAAAGCTTTTGCCATCAACTTTATGCCAGAATTTATTTTGAACCCCT
AAAAGACATTTCTATAA
W
AGTACTCCTAGTTTTCTTCATGAAAAATACACTTAAAGCCTAATTTGGATGCATTTCATTTATGGTAAGGAGTCTATCTTTTA
ATAACACTGTCAGAAAA / Celera SNP ID: hCV12066129 / Public SNP ID: rs1593 / SNP Chromosome Position: 187195551 / SNP in Genomic Sequence: SEQ ID NO: 347 / SNP Position Genomic: 18433 / Related Interrogated SNP: hCV12066124 / Related Interrogated SNP: hCV15793897 / Related Interrogated SNP: hCV25474413 / Related Interrogated SNP: hCV27477533 / Related Interrogated SNP: hCV3230038 / Related Interrogated SNP: hCV8241630 / Related Interrogated SNP: hCV22272267 / Related Interrogated SNP: hCV27474895 / Related Interrogated SNP: hCV27902808 / Related Interrogated SNP: hCV25990131 / Related Interrogated SNP: hCV30562347 / SNP Source: Applera / Population(Allele,Count): Caucasian (A,35|T,3) African American (A,28|T,6) total (A,63|T,9) / SNP Type: TRANSCRIPTION FACTOR BINDING SITE;MICRORNA;UTR3;INTRON;PSEUDOGENE / SNP Source: dbSNP; HapMap; HGBASE / Population(Allele,Count): Caucasian (T,12|A,104) / SNP Type: TRANSCRIPTION FACTOR BINDING SITE;MICRORNA;UTR3;INTRON;PSEUDOGENE /
Context (SEQ ID NO: 720): /
TACACGACAACAAGGCAAAAAATGAATATAGTAAACAAAGAAAACACAGATAATGTACAGTGGAAGAAGAGTCTCTTCTGGAA
AAGAGGATATATTTTGC
K
TCTCATATTTAAACCACGATTTTTTAAATTTAGATTCTCAACGACCCATATGCCTGCCTTCCAAAGGAGATAGAAATGTAATA
TACACTGATTGCTGGGT / Celera SNP ID: hCV15880920 / Public SNP ID: rs2289253 / SNP Chromosome Position: 187207535 / SNP in Genomic Sequence: SEQ ID NO: 347 / SNP Position Genomic: 30417 / Related Interrogated SNP: hCV27474895 / SNP Source: Applera / Population(Allele,Count): Caucasian (G,36|T,2) African American (G,33|T,5) total (G,69|T,7) / SNP Type: INTRON / SNP Source: dbSNP; HapMap; ABI_Val; HGBASE / Population(Allele,Count): Caucasian (G,215|T,11) / SNP Type: INTRON /
Context (SEQ ID NO: 721): /
GGGGGAAAAGATGCTTGTAAGGTAACTCATGAGATTATGAAAAACACAATAGGCTGCTTGAGAAAATTCATTTCAAAATATAT
TTTCCAATAGCATAATT
Y
AATCATAGTTTTTAAAAAAATTCAGAGACAAATGATCTGATAAATTGATAAGCAACTTTTAACAAATTGAATATACATAATAT
ATATTTATATTATTTAT / Celera SNP ID: hCV22271609 / Public SNP ID: rs4253326 / SNP Chromosome Position: 187178599 / SNP in Genomic Sequence: SEQ ID NO: 347 / SNP Position Genomic: 1481 / Related Interrogated SNP: hCV15793897 / Related Interrogated SNP: hCV22272267 / Related Interrogated SNP: hCV15968043 / Related Interrogated SNP: hCV3230113 / Related Interrogated SNP: hCV27902808 / Related Interrogated SNP: hCV11786258 / SNP Source: Applera / Population(Allele,Count): Caucasian (C,4|T,34) African American (C,4|T,22) total (C,8|T,56) / SNP Type: TRANSCRIPTION FACTOR BINDING SITE;INTRON / SNP Source: dbSNP; HapMap; HGBASE / Population(Allele,Count): Caucasian (C,35|T,187) / SNP Type: TRANSCRIPTION FACTOR BINDING SITE;INTRON /
Context (SEQ ID NO: 722): /
AGGATCTGAACACAGAGAGCGGCGGGGCCGGCGGGGAAGGAGGGAGGAGGGGAGAGCGCTGCTTCCCTGTGGGTTCCGGCTTC
TGCAGAGCTGTAAGAGT
K
GAATGCCACACACAGTCACACTAAGGAATGCTCCAGGATTGGGAAAGAAAATTCAACATTATAATGAGAACACTGTGAATGCT
ATTGAATTAACTACTCC / Celera SNP ID: hCV25474414 / Public SNP ID: rs4253399 / SNP Chromosome Position: 187188094 / SNP in Genomic Sequence: SEQ ID NO: 347 / SNP Position Genomic: 10976 / Related Interrogated SNP: hCV11786258 / Related Interrogated SNP: hCV12066124 / Related Interrogated SNP: hCV15968043 / Related Interrogated SNP: hCV27474895 / Related Interrogated SNP: hCV3230038 / Related Interrogated SNP: hCV27477533 / Related Interrogated SNP:

hCV8241630 / Related Interrogated SNP: hCV25474413 / Related Interrogated SNP: hCV3230113 / Related Interrogated SNP: hCV22272267 / Related Interrogated SNP: hCV3230096 / SNP Source: Applera / Population(Allele,Count): Caucasian (G,12|T,18) African American (G,1|T,15) total (G,13|T,33) / SNP Type: TRANSCRIPTION FACTOR BINDING SITE;INTRON / SNP Source: Applera / Population(Allele,Count): Caucasian (G,16|T,14) African American (G,1|T,25) total (G,17|T,39) / SNP Type: TRANSCRIPTION FACTOR BINDING SITE;INTRON / SNP Source: Applera / Population(Allele,Count): Caucasian (G,7|T,19) African American (G,0|T,14) total (G,7|T,33) / SNP Type: TRANSCRIPTION FACTOR BINDING SITE;INTRON / SNP Source: dbSNP; HapMap; HGBASE / Population(Allele,Count): Caucasian (T,137|G,89) / SNP Type: TRANSCRIPTION FACTOR BINDING SITE;INTRON / Context (SEQ ID NO: 723): /
AACCCATCTCTACAAAAAAATATGAAAGTATCTGTGTGTGTGTGTTGCTGTGTAGTGGACTACAGAACTTTAGAGGCAGTCAC TTATTTGAATCCCATTG
Y
CGTAACTTTCTACTATTTTATTTTTCCACTGTGACTCAGGGAGATTCAGGTGGTCCCTTAGTTTGCAAACACAATGGAATGTG GCGTTTGGTGGGCATCA / Celera SNP ID: hCV25634754 / Public SNP ID: rs4253331 / SNP Chromosome Position: 187179135 / SNP in Genomic Sequence: SEQ ID NO: 347 / SNP Position Genomic: 2017 / Related Interrogated SNP: hCV27477533 / Related Interrogated SNP: hCV8241630 / Related Interrogated SNP: hCV3230038 / Related Interrogated SNP: hCV27474895 / SNP Source: Applera / Population(Allele,Count): Caucasian (C,5|T,35) African American (C,0|T,24) total (C,5|T,59) / SNP Type: INTRON / SNP Source: dbSNP; HapMap; HGBASE / Population(Allele,Count): Caucasian (T,210|C,16) / SNP Type: INTRON / Context (SEQ ID NO: 724): /
CGTAGGCAATAAGAGGACAGTACAGGGTGCCGTCTCTCTCCCTCTTCCTCTCTCTGTCTCTCTCTCTCTGTGTGTGTGTGTGT GTGTGTGTAACACTA
Y
CTTCCCAATTTTTACTGTCTATTTGTATTCAAAGATAAGGTCCTTATGAAAAATACACTGCTCTGATTCACTTTAAAACTTAT TTCCATATTTATTATTT / Celera SNP ID: hCV3230014 / Public SNP ID: rs4861709 / SNP Chromosome Position: 187178186 / SNP in Genomic Sequence: SEQ ID NO: 347 / SNP Position Genomic: 1068 / Related Interrogated SNP: hCV12066124 / Related Interrogated SNP: hCV15968043 / Related Interrogated SNP: hCV22272267 / Related Interrogated SNP: hCV3230113 / Related Interrogated SNP: hCV11786258 / Related Interrogated SNP: hCV25474413 / Related Interrogated SNP: hCV15793897 / Related Interrogated SNP: hCV27477533 / Related Interrogated SNP: hCV8241630 / Related Interrogated SNP: hCV3230096 / SNP Source: Applera / Population(Allele,Count): Caucasian (C,7|T,29) African American (C,5|T,5) total (C,12|T,34) / SNP Type: INTRON / SNP Source: dbSNP; Celera; HapMap; HGBASE / Population(Allele,Count): Caucasian (C,36|T,84) / SNP Type: INTRON / Context (SEQ ID NO: 725): /
CTCACAGGTGAAATCCAAAATATTCTACAAAAGGTAAATATTCCTTTGGTAACAAATGAAGAATGCCAGAAAAGATATCAAGA TTATAAAATAACCCAAC
R
GATGGTCTGTGCTGGCTATAAAGAAGGGGGAAAAGATGCTTGTAAGGTAACTCATGAGATTATGAAAAACACAATAGGCTGCT TGAGAAAATTCATTTCA / Celera SNP ID: hCV3230016 / Public SNP ID: rs4253325 / SNP Chromosome Position: 187178473 / SNP in Genomic Sequence: SEQ ID NO: 347 / SNP Position Genomic: 1355 / Related Interrogated SNP: hCV27474895 / Related Interrogated SNP: hCV27477533 / Related Interrogated SNP: hCV8241630 / Related Interrogated SNP: hCV3230038 / Related Interrogated SNP: hCV22272267 / SNP Source: Applera / Population(Allele,Count): Caucasian (A,2|G,38) African American (A,3|G,31) total (A,5|G,69) / SNP Type: MISSENSE MUTATION;ESE;ESS SYNONYMOUS / SNP Source: dbSNP; Celera; HapMap; ABI_Val; HGBASE / Population(Allele,Count): Caucasian (G,199|A,25) / SNP Type: MISSENSE MUTATION;ESE;ESS SYNONYMOUS /
Context (SEQ ID NO: 726): /
TTGTAAGGTAACTCATGAGATTATGAAAAACACAATAGGCTGCTTGAGAAAATTCATTTCAAAATATATTTTCCAATAGCATA ATTCAATCATAGTTTTT
W
AAAAAATTCAGAGACAAATGATCTGATAAATTGATAAGCAACTTTTAACAAATTGAATATACATAATATATATTTATATTATT TATGATATATGTCACAA / Celera SNP ID: hCV3230017 / Public SNP ID: rs4253327 / SNP Chromosome Position: 187178613 / SNP in Genomic Sequence: SEQ ID NO: 347 / SNP Position Genomic: 1495 / Related Interrogated SNP: hCV15968043 / Related

Interrogated SNP: hCV11786258 / Related Interrogated SNP: hCV27477533 / Related Interrogated SNP: hCV8241630 / Related Interrogated SNP: hCV3230038 / Related Interrogated SNP: hCV3230096 / Related Interrogated SNP: hCV15793897 / Related Interrogated SNP: hCV3230113 / Related Interrogated SNP: hCV27474895 / Related Interrogated SNP: hCV12066124 / Related Interrogated SNP: hCV22272267 / Related Interrogated SNP: hCV25474413 / SNP Source: Applera / Population(Allele,Count): Caucasian (A,10|T,30) African American (A,19|T,19) total (A,29|T,49) // / SNP Type: TFBS SYNONYMOUS;INTRON / SNP Source: dbSNP; Celera; HapMap; HGBASE / Population(Allele,Count): Caucasian (A,34|T,72) / SNP Type: TFBS SYNONYMOUS;INTRON /
Context (SEQ ID NO: 727): /
GCAGTCACTTATTTGAATCCCATTGTCGTAACTTTCTACTATTTTATTTTTCCACTGTGACTCAGGGAGATTCAGGTGGTCCC
TTAGTTTGCAAACACAA
Y

GGAATGTGGCGTTTGGTGGGCATCACCAGCTGGGGTGAAGGCTGTGCCCGCAGGGAGCAACCTGGTGTCTACACCAAAGTCGC
TGAGTACATGGACTGGA / Celera SNP ID: hCV3230018 / Public SNP ID: rs925453 / SNP Chromosome Position: 187179210 / SNP in Genomic Sequence: SEQ ID NO: 347 / SNP Position Genomic: 2092 / Related Interrogated SNP: hCV12066124 / Related Interrogated SNP: hCV15968043 / Related Interrogated SNP: hCV22272267 / Related Interrogated SNP: hCV3230113 / Related Interrogated SNP: hCV11786258 / Related Interrogated SNP: hCV25474413 / Related Interrogated SNP: hCV15793897 / Related Interrogated SNP: hCV27477533 / Related Interrogated SNP: hCV8241630 / Related Interrogated SNP: hCV3230096 / SNP Source: Applera / Population(Allele,Count): Caucasian (C,30|T,10) African American (C,22|T,14) total (C,52|T,24) // / SNP Type: SILENT MUTATION / SNP Source: dbSNP; Celera; HapMap; ABI_Val; HGBASE / Population(Allele,Count): Caucasian (T,64|C,162) / SNP Type: SILENT MUTATION /
Context (SEQ ID NO: 728): /
AATGTTTTTATGTGTTTGATATGATATATTTCTACTTCCCTTTTGTTTTTGTTAGAAATCTTTGTCTCCTTAAAACATCTGAG
AGTGGATTGCCCAGTAC
R

CGCATTAAAAAGAGCAAAGCTCTTTCTGGTTTCAGTCTACAAAGCTGCAGGCACAGCATCCCAGGTAAACTGAGAGTTCTGCA
TTCTGGCTGAGAGTGAC / Celera SNP ID: hCV3230032 / Public SNP ID: rs5974 / SNP Chromosome Position: 187201211 / SNP in Genomic Sequence: SEQ ID NO: 347 / SNP Position Genomic: 24093 / Related Interrogated SNP: hCV27474895 / Related Interrogated SNP: hCV3230038 / Related Interrogated SNP: hCV25474413 / SNP Source: Applera / Population(Allele,Count): Caucasian (A,25|G,5) African American (A,30|G,8) total (A,55|G,13) / SNP Type: SILENT RARE CODON;SILENT MUTATION / SNP Source: dbSNP; Celera; HapMap / Population(Allele,Count): Caucasian (A,193|G,33) / SNP Type: SILENT RARE CODON;SILENT MUTATION /
Context (SEQ ID NO: 729): /
ACTCTCATAAATAATATATAAAAGTCTAGTGATTCATGAATTTGGGAAACAAAGCTCTATGCGGGCCAGCTTCCCATCTAGCC
GGAGACCACTGCCTCAT
S

AGAGTTAGGATGGAATACTGCCTAGCTTGGCAGCTGGCTCTAAATGACTGTGCGATGCGTTAGACTCCTTACATAATTAAAAA
TTATGTAAGGAGTCTAA / Celera SNP ID: hCV1333076 / Public SNP ID: rs7656944 / SNP Chromosome Position: 187294897 / SNP in Genomic Sequence: SEQ ID NO: 347 / SNP Position Genomic: 117779 / Related Interrogated SNP: hCV27474895 / SNP Source: dbSNP; Celera / Population(Allele,Count): Caucasian (C,61|G,165) / SNP Type: INTRON /
Context (SEQ ID NO: 730): /
AGCCTCTGTAACCTAATAACTAGACATGGTATCCTTAGAACTCTCATAAATAATATATAAAAGTCTAGTGATTCATGAATTTG
GGAAACAAAGCTCTATG
S

GGGCCAGCTTCCCATCTAGCCGGAGACCACTGCCTCATCAGAGTTAGGATGGAATACTGCCTAGCTTGGCAGCTGGCTCTAAA
TGACTGTGCGATGCGTT / Celera SNP ID: hCV1333077 / Public SNP ID: rs7656763 / SNP Chromosome Position: 187294858 / SNP in Genomic Sequence: SEQ ID NO: 347 / SNP Position Genomic: 117740 / Related Interrogated SNP: hCV27474895 / SNP Source: dbSNP; Celera; HapMap; ABI_Val / Population(Allele,Count): Caucasian (C,61|G,165) / SNP Type: INTRON /
Context (SEQ ID NO: 731): /
AAAGTATTTATCTTCACGAAGATGATATTCTCACTGGGGAGAGACAGCAAATAAGCATTGCAGAACGGCCTGAAGTTTCAGAA
TGGGTTGTAACCTTAGA

R
CATTGCCTGCTATCCAAATCTCCTTGGAACAAATCCACCTAGCACCAGGAATCAATTTAATCGTGGCTCTAGAATTCACAACA
AAATCGAATAATACAGT / Celera SNP ID: hCV1333078 / Public SNP ID: rs9998003 /
SNP Chromosome Position: 187293933 / SNP in Genomic Sequence: SEQ ID NO: 347 /
SNP Position Genomic: 116815 / Related Interrogated SNP: hCV27474895 / SNP
Source: dbSNP; Celera; HapMap / Population(Allele,Count): Caucasian
(A,66|G,160) / SNP Type: INTRON /
Context (SEQ ID NO: 732): /
CCGAGCAAGATTCCATTTCAAAATGATGATGATGATGATAATAATAATAATAATAATAATAATAATAATAATAATAATGTATT
TGTTGGAGTAAAAACTCA

Y
AATTCCATTGCTTTTGCAAGATAGTTCTGTCAGAGTCACAGTTTTTATTATAGTCAGGCTAGAAGGGAGAAGGCCTTCTCATCA
TTGACCTGTAGTTATTT / Celera SNP ID: hCV1333083 / Public SNP ID: rs10022988 /
SNP Chromosome Position: 187291021 / SNP in Genomic Sequence: SEQ ID NO: 347 /
SNP Position Genomic: 113903 / Related Interrogated SNP: hCV3230038 /
Related Interrogated SNP: hCV8241630 / SNP Source: dbSNP; Celera; HapMap /
Population(Allele,Count): Caucasian (T,30|C,90) / SNP Type: INTRON /
Context (SEQ ID NO: 733): /
GAAGAAGAAAGAGCAAACCAGCCTCACCTGCCATCTGCTTTGTTCTGGGCTTTGTGCTGTGTGCTCTATTTTTGTATGCATTG
CCCATTTAACTGTCCCA

R
CTGCTCAGTGAGGTGGGATATGTATGGATGGTGTTGGGAAAAAAGCTCAGTGTTGGGAGAGAAGCTGAGGCAGGGCTTGCATG
TCTGCTACACTTGCTGG / Celera SNP ID: hCV1333090 / Public SNP ID: rs6816112 /
SNP Chromosome Position: 187285215 / SNP in Genomic Sequence: SEQ ID NO: 347 /
SNP Position Genomic: 108097 / Related Interrogated SNP: hCV3230038 /
Related Interrogated SNP: hCV8241630 / Related Interrogated SNP: hCV27477533 /
SNP Source: dbSNP; Celera; HapMap / Population(Allele,Count): Caucasian
(G,30|A,86) / SNP Type: INTRON /
Context (SEQ ID NO: 734): /
TGGGGACACTCACCAATCCTATCAGATTAGGACCTCACCCTATGACCTAGTTTAACCTTAATTACTTCCCTAAAAACCCAGTC
TCCAAACACAATCACAC

R
GGGGGTTAGTTAGGGCTTCACCATAAGAATTTTGGGATGACACAGTTCAGTCTATAACAAAGACCTTTATTAAAAGCAAGATC
TTGGATCTTTCTTCCCA / Celera SNP ID: hCV1333097 / Public SNP ID: rs4862680 /
SNP Chromosome Position: 187282484 / SNP in Genomic Sequence: SEQ ID NO: 347 /
SNP Position Genomic: 105366 / Related Interrogated SNP: hCV3230038 /
Related Interrogated SNP: hCV8241630 / SNP Source: dbSNP; Celera; HapMap;
HGBASE / Population(Allele,Count): Caucasian (A,30|G,90) / SNP Type: INTRON
/
Context (SEQ ID NO: 735): /
CTCACGCCTGTAATCCCAGCACTTTCGGAGGCCAAGGCAGGCAGATCGCTTGAGGCCAGGAGTTCAAGACCAGCCTGGCCAAC
ATGGTGAAACCCTGCCT

Y
TACCGAAAATACAAAAACTAGCCTGGTGTGTGTTCTGCGAGGGAAATGCTTGAGGGGAGAAGAAAAGACACACACAATACC
TTTAAGGGTAAACAACC / Celera SNP ID: hCV1333099 / Public SNP ID: rs10020635 /
SNP Chromosome Position: 187280138 / SNP in Genomic Sequence: SEQ ID NO: 347 /
SNP Position Genomic: 103020 / Related Interrogated SNP: hCV27477533 /
Related Interrogated SNP: hCV3230038 / Related Interrogated SNP: hCV8241630 /
SNP Source: dbSNP; Celera / Population(Allele,Count): Caucasian (T,27|C,89) /
SNP Type: INTRON /
Context (SEQ ID NO: 736): /
TTTTAGTAAATAATAATCATCCTGGAAATGAAGGTAAACTGTAAGTTGCTCAAAAGAGAATTAAATATTGCTCAAATTCACCA
CAGGACCAAGAAAACTA

R
ATCAAAATAAAGAAGTTAAGAAGTATAATAGAAAAAATAATAGGTATAGAAGATAGGCAAAGAAGATGGAACATATATATAAC
TGGAATCTGCAAAGAAA / Celera SNP ID: hCV1333102 / Public SNP ID: rs10016252 /
SNP Chromosome Position: 187277164 / SNP in Genomic Sequence: SEQ ID NO: 347 /
SNP Position Genomic: 100046 / Related Interrogated SNP: hCV27474895 / SNP
Source: dbSNP; Celera; HapMap / Population(Allele,Count): Caucasian
(G,62|A,164) / SNP Type: INTRON /
Context (SEQ ID NO: 737): /
TTTTAAAAGTTGGAAATGTTCTTGAATAGTTTTGATCCTAAAGCCTCAGAGAAAGTCTGTCTGTGACCCGCTCCAGAGCTGCC
TCACTTTCTGACATGAG

Y
GGTGCTCTTGGACAGCCGGCAACGAAGCCACGCTTGTGAAGGACACAGCGGTTCTCACCTGTGGCACCCGGGGCACCAGTGGC
TGGCCACTGCCCTACAC / Celera SNP ID: hCV2103375 / Public SNP ID: rs12502630 /
SNP Chromosome Position: 187213296 / SNP in Genomic Sequence: SEQ ID NO: 347 /
SNP Position Genomic: 36178 / Related Interrogated SNP: hCV12066124 /
Related Interrogated SNP: hCV25474413 / SNP Source: dbSNP; Celera; HapMap /
Population(Allele,Count): Caucasian (C,112|T,6) / SNP Type: INTRON /
Context (SEQ ID NO: 738): /
CAGAGTAGTGTGATCATTAATCTAATTTTCTATCATCTTCATCACTCCCCCAAAGAAACCTCATACCTATGTGTAGATTTGCC
TCTTCTGAACATTTTAC
R
TAAATGCAATCATACAACATGGGGTGTTTTGTGACCTGTTTCTTTCACTTTCACCTTGTTTTCAAGGAGGAACCATGTTCTAA
AGCCTGTACTTCATTCC / Celera SNP ID: hCV2103388 / Public SNP ID: rs4613610 /
SNP Chromosome Position: 187222271 / SNP in Genomic Sequence: SEQ ID NO: 347 /
SNP Position Genomic: 45153 / Related Interrogated SNP: hCV3230038 / Related
Interrogated SNP: hCV15793897 / Related Interrogated SNP: hCV25474413 /
Related Interrogated SNP: hCV27474895 / Related Interrogated SNP: hCV12066124
/ Related Interrogated SNP: hCV27477533 / Related Interrogated SNP:
hCV8241630 / SNP Source: dbSNP; Celera; HapMap / Population(Allele,Count):
Caucasian (A,21|G,93) / SNP Type: INTRON /
Context (SEQ ID NO: 739): /
AACATTAGAGAAGACTCTCAATATTGCTCATGTTAGAATAAAGCCAGCTTCTTTTCTATTAAAAAAATGCTTGTCCAACTCGC
GTGAGGTTTGTCAGCGA
Y
GCAGAGGAGGGGCTTTTGTTGTTGCAGGGGTCATACTTTTCCATTTGTTTTTCCTCGTGGAATTTTCTCCAGCTCTCCCATTT
AACACTGCGTGCCCCGC / Celera SNP ID: hCV2103391 / Public SNP ID: rs1008728 /
SNP Chromosome Position: 187226673 / SNP in Genomic Sequence: SEQ ID NO: 347 /
SNP Position Genomic: 49555 / Related Interrogated SNP: hCV12066124 /
Related Interrogated SNP: hCV25474413 / Related Interrogated SNP: hCV27477533
/ Related Interrogated SNP: hCV27474895 / Related Interrogated SNP:
hCV3230038 / Related Interrogated SNP: hCV8241630 / Related Interrogated SNP:
hCV15968043 / Related Interrogated SNP: hCV22272267 / Related Interrogated SNP:
hCV11786258 / Related Interrogated SNP: hCV3230113 / SNP Source: dbSNP;
Celera; HapMap; ABI_Val; HGBASE / Population(Allele,Count): Caucasian
(C,82|T,144) / SNP Type: INTRON /
Context (SEQ ID NO: 740): /
GGCACAGCATTTGTCATTGCTTCTGCCTGCCCCTTAATAACCATATCCACCTGCTTGTGTCTTTTAACTGAGTTATCAGTCAC
CTCCTCCATGAAGCTGT
Y
GCAAGCTTTCTCAGGATGACGCCCCTCTTGCTGCATTGTTGCCTGGTCTGTGCCTCTGTGTAGAACTTGCCACATTGCATTAT
GGCCAGAGGCTTGTGTG / Celera SNP ID: hCV2103392 / Public SNP ID: rs12500826 /
SNP Chromosome Position: 187227451 / SNP in Genomic Sequence: SEQ ID NO: 347 /
SNP Position Genomic: 50333 / Related Interrogated SNP: hCV12066124 /
Related Interrogated SNP: hCV25474413 / Related Interrogated SNP: hCV27477533
/ Related Interrogated SNP: hCV8241630 / Related Interrogated SNP: hCV3230038
/ Related Interrogated SNP: hCV27474895 / Related Interrogated SNP:
hCV15968043 / Related Interrogated SNP: hCV22272267 / Related Interrogated SNP:
hCV11786258 / Related Interrogated SNP: hCV3230113 / Related Interrogated
SNP: hCV25990131 / Related Interrogated SNP: hCV27902808 / SNP Source:
dbSNP; Celera; HapMap / Population(Allele,Count): Caucasian (T,40|C,72) / SNP
Type: INTRON /
Context (SEQ ID NO: 741): /
TTTCTGCAAATCTAAAACTGCTCTATAAAATAAAGTCTACTAATTTTTAAAAGTGTACACCGATTTGGAAATAAGTCCTACTA
CACAAAACGTGATGGGT
R
AGCCTGAACTCCGGGTCCAGACTGCTTGAGTGAGCTGGGACAAATGACGTGTGGAAAATAGCAGGAACAGTGCCTGGCCATAG
GGCTGGTTTGAAAGGGC / Celera SNP ID: hCV2103401 / Public SNP ID: rs7687352 /
SNP Chromosome Position: 187239747 / SNP in Genomic Sequence: SEQ ID NO: 347 /
SNP Position Genomic: 62629 / Related Interrogated SNP: hCV12066124 /
Related Interrogated SNP: hCV15968043 / SNP Source: dbSNP; Celera; HapMap /
Population(Allele,Count): Caucasian (A,96|G,130) / SNP Type: INTRON /
Context (SEQ ID NO: 742): /
GCCTGAACTCCGGGTCCAGACTGCTTGAGTGAGCTGGGACAAATGACGTGTGGAAAATAGCAGGAACAGTGCCTGGCCATAGG

GCTGGTTTGAAAGGGCA
K
ATGAAAATCTATGGGGAGCAGATGGCCAAGTGCCTCCAGATGGTTATAACATTAGATAGAGTTCTCATAAGATAATTTCTACT
AGTAAGAACCACTCACA / Celera SNP ID: hCV2103402 / Public SNP ID: rs9993749 /
SNP Chromosome Position: 187239849 / SNP in Genomic Sequence: SEQ ID NO: 347 /
SNP Position Genomic: 62731 / Related Interrogated SNP: hCV12066124 /
Related Interrogated SNP: hCV3230113 / Related Interrogated SNP: hCV15968043 /
Related Interrogated SNP: hCV25474413 / SNP Source: dbSNP; Celera /
Population(Allele,Count): Caucasian (G,178|T,46) / SNP Type: INTRON /
Context (SEQ ID NO: 743): /
TGAATATATAAAGCAAAAAAATGAAAAGTGAGAACTTCCAGGCTTTGGATTGTTGTAGGTGATAAATATAAAATGGGATTTCT
GGGGGGCTGCTACTGAG
W
TGAGGGGATGGCAGAAAACATGGAAGCAAGGTCTCTGGTCAGCCCAGGGTGCTGGGCTTGTCCCAACACCACGTAGGCAATAA
GAGGACAGTACAGGGTG / Celera SNP ID: hCV3230013 / Public SNP ID: rs3775303 /
SNP Chromosome Position: 187178014 / SNP in Genomic Sequence: SEQ ID NO: 347 /
SNP Position Genomic: 896 / Related Interrogated SNP: hCV11786258 / Related
Interrogated SNP: hCV12066124 / Related Interrogated SNP: hCV15968043 /
Related Interrogated SNP: hCV22272267 / Related Interrogated SNP: hCV27477533
/ Related Interrogated SNP: hCV3230038 / Related Interrogated SNP: hCV3230113
/ Related Interrogated SNP: hCV8241630 / Related Interrogated SNP: hCV3230096
/ Related Interrogated SNP: hCV27474895 / Related Interrogated SNP:
hCV25474413 / Related Interrogated SNP: hCV27902808 / Related Interrogated SNP:
hCV25990131 / SNP Source: dbSNP; Celera; HGBASE / Population(Allele,Count):
Caucasian (A,71|T,49) / SNP Type: INTRON /
Context (SEQ ID NO: 744): /
TCCCTCTAATTGCACTGCTTCCTCTGGAACTCAGTATATCTCAAAGATGTAATTTCCTCTCCGTGCTGCACCTGGTCGGCCAC
TGAAACCCACTATTGCC
Y
GCTTCACGTGTGGCAAAGAGCTAGCGGGCTTGGGTTTTGTTCTGCCGAGAGGAAGGGAGAACACCCACTTTTATAAGAAAGAG
ATGGGTTACCTGAACCC / Celera SNP ID: hCV3230019 / Public SNP ID: rs4253332 /
SNP Chromosome Position: 187179803 / SNP in Genomic Sequence: SEQ ID NO: 347 /
SNP Position Genomic: 2685 / Related Interrogated SNP: hCV12066124 / Related
Interrogated SNP: hCV15968043 / Related Interrogated SNP: hCV22272267 /
Related Interrogated SNP: hCV3230113 / Related Interrogated SNP: hCV11786258 /
Related Interrogated SNP: hCV25474413 / Related Interrogated SNP: hCV15793897
/ Related Interrogated SNP: hCV27477533 / Related Interrogated SNP:
hCV8241630 / Related Interrogated SNP: hCV3230096 / SNP Source: dbSNP;
Celera; HapMap; HGBASE / Population(Allele,Count): Caucasian (T,62|C,162) / SNP
Type: INTERGENIC;UNKNOWN /
Context (SEQ ID NO: 745): /
CTTTTATCTCAGTCTTTGAGTATTGATCATGGGGTGTTGGAACAGGACTTTGGAATGCTTGCAGGGTAAACCTTTGGCCTCTG
TTAGTCAGGGAATGACC
Y
AGTTTGGCAAAACAGAGGAGAGTTTTGAAATATGGAACTTTCCCGAGGCATACATTGTCATTTTAAAGTGGTCAATCAAAGCC
CAGTAGGACTGGGCTGG / Celera SNP ID: hCV3230021 / Public SNP ID: rs13135645 /
SNP Chromosome Position: 187181863 / SNP in Genomic Sequence: SEQ ID NO: 347 /
SNP Position Genomic: 4745 / Related Interrogated SNP: hCV12066124 / Related
Interrogated SNP: hCV25474413 / Related Interrogated SNP: hCV27477533 /
Related Interrogated SNP: hCV8241630 / Related Interrogated SNP: hCV27474895 /
Related Interrogated SNP: hCV3230038 / Related Interrogated SNP: hCV15968043
/ SNP Source: dbSNP; Celera; HapMap / Population(Allele,Count): Caucasian
(T,193|C,33) / SNP Type: INTERGENIC;UNKNOWN /
Context (SEQ ID NO: 746): /
TGATATCCGGTTGCTTCTCACTAGGAGAAGGATGAAAGATGACAGAGCATTTATAACCACCATTTGTTATTTTCATTATCAAC
TGACACTGGTGTTTCTC
Y
GCCCGGAGCGATTTTGTCCTCCCTTCACCTCAGGAAACATTCGGCAACGTCTAGAGACACTTTTGGTTGTCCTAATGGGAGAG
GGTATGCAACTGGCTGT / Celera SNP ID: hCV3230022 / Public SNP ID: rs11132383 /
SNP Chromosome Position: 187182660 / SNP in Genomic Sequence: SEQ ID NO: 347 /
SNP Position Genomic: 5542 / Related Interrogated SNP: hCV12066124 / Related
Interrogated SNP: hCV25474413 / Related Interrogated SNP: hCV27474895 /
Related Interrogated SNP: hCV27477533 / Related Interrogated SNP: hCV3230038 /

Related Interrogated SNP: hCV8241630 / Related Interrogated SNP: hCV15968043 / Related Interrogated SNP: hCV3230096 / Related Interrogated SNP: hCV11786258 / Related Interrogated SNP: hCV3230113 / SNP Source: dbSNP; Celera; HapMap / Population(Allele,Count): Caucasian (T,121|C,105) / SNP Type: INTERGENIC;UNKNOWN /
Context          (SEQ ID NO: 747): /
GACTCGTCAGCCAGTGAGTAGGTCTGGGTAGCAGTTGGCATGGATGAACATGCCCTCCATAGGCTTTCAGACCTTCTTCAAGG
CCAAAGGGAAGGCCTTC
R
TGGAAATATAATTATGTGAAACACCCCAGAATTTTTTCACAAACTTTCCTGCCTATAAACGCCAGGTCCTACCACTGTCCAGT
GTCCCACTTCCCACTGT / Celera SNP ID: hCV3230025 / Public SNP ID: rs3756009 / SNP Chromosome Position: 187186111 / SNP in Genomic Sequence: SEQ ID NO: 347 / SNP Position Genomic: 8993 / Related Interrogated SNP: hCV12066124 / Related Interrogated SNP: hCV15968043 / Related Interrogated SNP: hCV27474895 / Related Interrogated SNP: hCV25474413 / Related Interrogated SNP: hCV27477533 / Related Interrogated SNP: hCV3230038 / Related Interrogated SNP: hCV8241630 / Related Interrogated SNP: hCV11786258 / Related Interrogated SNP: hCV3230096 / Related Interrogated SNP: hCV3230113 / Related Interrogated SNP: hCV22272267 / Related Interrogated SNP: hCV25990131 / SNP Source: dbSNP; Celera; HapMap / Population(Allele,Count): Caucasian (A,60|G,40) / SNP Type: INTERGENIC;UNKNOWN /
Context          (SEQ ID NO: 748): /
TAAAATTTACTATTCCAGAACCTAGAGCAGGGATTGGCAAATGTCTTCTTAATAACGCAGAGTAAATATGTTAGGCTTTGTGG
GCAAAACCCACAGTAAA
R
CCAAGGATATTATTTAAGTATTTATGTCACCACTTAAAATGTAACAATTTGAAAATATAAAAATCATTTTGTATAGCTAACAG
GCTAAACAGAAACACAC / Celera SNP ID: hCV3230030 / Public SNP ID: rs4253408 / SNP Chromosome Position: 187193858 / SNP in Genomic Sequence: SEQ ID NO: 347 / SNP Position Genomic: 16740 / Related Interrogated SNP: hCV27477533 / Related Interrogated SNP: hCV8241630 / Related Interrogated SNP: hCV27474895 / Related Interrogated SNP: hCV3230038 / Related Interrogated SNP: hCV12066124 / Related Interrogated SNP: hCV25474413 / SNP Source: dbSNP; Celera; HapMap; HGBASE / Population(Allele,Count): Caucasian (G,111|A,9) / SNP Type: INTRON /
Context          (SEQ ID NO: 749): /
AGATGGAAATGTCAATGACAGAATGTGGAGGATTTCGCAAGCAGCAGAGTGTAGGAGAAGCAATGCCCTTGGCTCATCTAGGG
CAAAGGGTTGAATTGGC
R
ACTGGAAATAGAAGTCAAATTCAGAAGAGAGGTTTATGTGGACATCTGTGTTTAGGTGTCACCAATATACAGCTGATACTTTT
TAAAAATCTAGTGAAAT / Celera SNP ID: hCV3230031 / Public SNP ID: rs4253419 / SNP Chromosome Position: 187199888 / SNP in Genomic Sequence: SEQ ID NO: 347 / SNP Position Genomic: 22770 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV3230038 / Related Interrogated SNP: hCV8241630 / Related Interrogated SNP: hCV3230113 / Related Interrogated SNP: hCV22272267 / Related Interrogated SNP: hCV32291301 / Related Interrogated SNP: hCV15968043 / SNP Source: dbSNP; Celera; HapMap; HGBASE / Population(Allele,Count): Caucasian (A,186|G,40) / SNP Type: INTRON /
Context          (SEQ ID NO: 750): /
CCAAAACTCCCGTTCTATGATCGTTGTAGTTTGTTTGAGCATTCAGTCTCTTTGTTTTTGATCACGCTTCTATGGAGTCCAAG
AATTACCATAAGGCAAT
R
TTTCTGAAGATTACTATATAGGCAGATATAGCAGAAAATAACCAAGTAGTGGCAGTGGGGATCAGGCAGAAGAACTGGTAAAA
GAAGCCACCATAAATAG / Celera SNP ID: hCV3230118 / Public SNP ID: rs4253429 / SNP Chromosome Position: 187210033 / SNP in Genomic Sequence: SEQ ID NO: 347 / SNP Position Genomic: 32915 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV3230038 / Related Interrogated SNP: hCV8241630 / Related Interrogated SNP: hCV3230113 / Related Interrogated SNP: hCV22272267 / Related Interrogated SNP: hCV32291301 / Related Interrogated SNP: hCV15968043 / SNP Source: dbSNP; Celera; HGBASE / Population(Allele,Count): Caucasian (A,186|G,40) / SNP Type:

UTR3;TFBS SYNONYMOUS /
Context (SEQ ID NO: 751): /
TGTTTGAGCATTCAGTCTCTTTGTTTTTGATCACGCTTCTATGGAGTCCAAGAATTACCATAAGGCAATATTTCTGAAGATTA
CTATATAGGCAGATATA
S
CAGAAAATAACCAAGTAGTGGCAGTGGGGATCAGGCAGAAGAACTGGTAAAAGAAGCCACCATAAATAGATTTGTTCGATGAA
AGATGAAAACTGGAAGA / Celera SNP ID: hCV3230119 / Public SNP ID: rs4253430 /
SNP Chromosome Position: 187210064 / SNP in Genomic Sequence: SEQ ID NO: 347 /
SNP Position Genomic: 32946 / Related Interrogated SNP: hCV12066124 /
Related Interrogated SNP: hCV25474413 / Related Interrogated SNP: hCV27474895
/ Related Interrogated SNP: hCV27477533 / Related Interrogated SNP:
hCV8241630 / Related Interrogated SNP: hCV3230038 / Related Interrogated SNP:
hCV3230113 / SNP Source: dbSNP; Celera; HapMap; HGBASE /
Population(Allele,Count): Caucasian (G,144|C,82) / SNP Type: MICRORNA;UTR3 /
Context (SEQ ID NO: 752): /
AGGGAGCATGCTCAAACCTCCCTAAGACAAGCTGCTGCTGTGACTATGGGCTCCCAAAGAGCTAGATCGTATATTTATTTGAC
AAAAATCACCATAGACT
R
CATCCATACTACAGAGAAAAAACAATTAGGGCGCAAATGGATAGTTACAGTAAAGTCTTCAGCAAGCAGCTGCCTGTATTCTA
AGCACTGGGATTTTCTG / Celera SNP ID: hCV3230121 / Public SNP ID: rs4253431 /
SNP Chromosome Position: 187210620 / SNP in Genomic Sequence: SEQ ID NO: 347 /
SNP Position Genomic: 33502 / Related Interrogated SNP: hCV27474895 /
Related Interrogated SNP: hCV3230038 / Related Interrogated SNP: hCV25474413 /
SNP Source: dbSNP; Celera; HGBASE / Population(Allele,Count): Caucasian
(G,193|A,33) / SNP Type: UTR3;INTRON /
Context (SEQ ID NO: 753): /
TCTGTCCATTATGGAAGGCTGGTGTTCACTGCAGGGATTCCACACTTCATTTAACCAGAGTCCCCGTGGACGGACACTTGGGT
TGTTTTCAATCTTTCCC
K
CTTTCATACACACGCAGGAACATTTGTAGGATAAAGTCCAGATGTAGAATTGCTGAAGACAAAACAAAACGAAAACAGCAGAT
GCATTTGTAATTTTGAA / Celera SNP ID: hCV3230125 / Public SNP ID: rs11938564 /
SNP Chromosome Position: 187212134 / SNP in Genomic Sequence: SEQ ID NO: 347 /
SNP Position Genomic: 35016 / Related Interrogated SNP: hCV12066124 /
Related Interrogated SNP: hCV25474413 / Related Interrogated SNP: hCV27477533
/ Related Interrogated SNP: hCV8241630 / Related Interrogated SNP: hCV3230038
/ Related Interrogated SNP: hCV27474895 / Related Interrogated SNP:
hCV15968043 / Related Interrogated SNP: hCV3230113 / Related Interrogated SNP:
hCV11786258 / Related Interrogated SNP: hCV22272267 / Related Interrogated
SNP: hCV3230096 / Related Interrogated SNP: hCV32291301 / SNP Source:
dbSNP; Celera; HapMap / Population(Allele,Count): Caucasian (T,176|G,50) / SNP
Type: MISSENSE MUTATION;ESE;ESE SYNONYMOUS /
Context (SEQ ID NO: 754): /
ACTCCACCATGCCCAGCTAATTATTTTTTTGTATTTTTTAGTAGAAACGGGGTTTCACCGTGTTAGCCAGGATGGTCGGCTTCC
GGTGTTGTTTCAGCATA
Y
GGCTAAATTAAAGGCTTAGATTCTCAGTTTGCTCATTCTAATTTAGCTGAATTCTGAGTATTATGCATTTGTATCTGAGGTTC
CAGGCCTCTCCCTCCTC / Celera SNP ID: hCV3230131 / Public SNP ID: rs13136269 /
SNP Chromosome Position: 187217008 / SNP in Genomic Sequence: SEQ ID NO: 347 /
SNP Position Genomic: 39890 / Related Interrogated SNP: hCV12066124 /
Related Interrogated SNP: hCV25474413 / Related Interrogated SNP: hCV27474895
/ Related Interrogated SNP: hCV27477533 / Related Interrogated SNP:
hCV3230038 / Related Interrogated SNP: hCV8241630 / Related Interrogated SNP:
hCV11786258 / Related Interrogated SNP: hCV3230096 / Related Interrogated SNP:
hCV3230113 / Related Interrogated SNP: hCV15968043 / SNP Source: dbSNP;
Celera; HapMap / Population(Allele,Count): Caucasian (T,155|C,71) / SNP Type:
INTRON /
Context (SEQ ID NO: 755): /
CATAAGCTGTTAGAGGTGAAAGAGACTTCAGCAATCATTCCATAGAAATGAAGCTCCAGGCGGGGCTGTGACCTCTCCCCGCT
CACACGGCCTGTGAGGG
Y
GGACGCTGAGCCCAGGAAATGTAGGTCTCCTGTGCCTCAGTCCTTCAGGGGCCAATTTAAATGAGAAGGGAAGTTGGATGGTA
GAGTTCATGGAATAGCA / Celera SNP ID: hCV3230133 / Public SNP ID: rs12511874 /
SNP Chromosome Position: 187217560 / SNP in Genomic Sequence: SEQ ID NO: 347 /

SNP Position Genomic: 40442 / Related Interrogated SNP: hCV12066124 / Related Interrogated SNP: hCV25474413 / Related Interrogated SNP: hCV3230038 / Related Interrogated SNP: hCV8241630 / Related Interrogated SNP: hCV27474895 / Related Interrogated SNP: hCV27477533 / Related Interrogated SNP: hCV11786258 / Related Interrogated SNP: hCV3230113 / Related Interrogated SNP: hCV15968043 / SNP Source: dbSNP; Celera; HapMap / Population(Allele,Count): Caucasian (T,145|C,73) / SNP Type: TRANSCRIPTION FACTOR BINDING SITE;INTRON / Context (SEQ ID NO: 756): /
CCGCTCACACGGCCTGTGAGGGTGGACGCTGAGCCCAGGAAATGTAGGTCTCCTGTGCCTCAGTCCTTCAGGGGCCAATTTAA ATGAGAAGGGAAGTTGG
R
TGGTAGAGTTCATGGAATAGCAGTTCCTCTGTCAGGCCTGTGTTTCTCTTTGCTGCCACTAACATATAAGAAAGAAACATGTT TCTGAGATGCCACAGTT / Celera SNP ID: hCV3230134 / Public SNP ID: rs12500151 / SNP Chromosome Position: 187217638 / SNP in Genomic Sequence: SEQ ID NO: 347 / SNP Position Genomic: 40520 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV3230038 / Related Interrogated SNP: hCV8241630 / Related Interrogated SNP: hCV11786258 / Related Interrogated SNP: hCV3230096 / Related Interrogated SNP: hCV3230113 / Related Interrogated SNP: hCV15968043 / SNP Source: dbSNP; Celera; HapMap / Population(Allele,Count): Caucasian (A,154|G,72) / SNP Type: INTRON / Context (SEQ ID NO: 757): /
TGCTGACAGCAATGGCAGGCTGCCATCCTTGGAGTGAGGACCTGTGCTTCTACTTCCTGCTTCCTCTGAACCCCAGCAGCTGC CAGCACAGCGTCTAGGA
R
TAGTGGCTGTGGACATAATTAGGAGTTTCTTGAGCAGCCTAGTTAACAGCCCCACAGAGGGCATGATCATATAAGAACGTCAC TCATGTCATTATCATTT / Celera SNP ID: hCV3230136 / Public SNP ID: rs13116273 / SNP Chromosome Position: 187217886 / SNP in Genomic Sequence: SEQ ID NO: 347 / SNP Position Genomic: 40768 / Related Interrogated SNP: hCV12066124 / Related Interrogated SNP: hCV25474413 / Related Interrogated SNP: hCV27477533 / Related Interrogated SNP: hCV27474895 / Related Interrogated SNP: hCV3230038 / Related Interrogated SNP: hCV8241630 / Related Interrogated SNP: hCV11786258 / Related Interrogated SNP: hCV15968043 / Related Interrogated SNP: hCV3230113 / Related Interrogated SNP: hCV22272267 / SNP Source: dbSNP; Celera; HapMap / Population(Allele,Count): Caucasian (G,83|A,37) / SNP Type: INTRON / Context (SEQ ID NO: 758): /
TGAGTAACTAAATTGAGTTGAGACTCTTAACTGATGTTCAGATTCACTGCCAGCACTCCCCACACATACCAGGAAACACCCTG TGTTCCAGTGGGCAGAC
Y
CATGCAAGCCCACCCCCAAAGCCTGGGGGTGCAGAGAGGCCGAGGAAAGAGGCTGACAAATCCAGACTCTCAGAAAGGAGCGT TGAATAGGGACTTATCC / Celera SNP ID: hCV8241628 / Public SNP ID: rs907439 / SNP Chromosome Position: 187220747 / SNP in Genomic Sequence: SEQ ID NO: 347 / SNP Position Genomic: 43629 / Related Interrogated SNP: hCV12066124 / Related Interrogated SNP: hCV3230038 / Related Interrogated SNP: hCV15793897 / Related Interrogated SNP: hCV25474413 / Related Interrogated SNP: hCV8241630 / Related Interrogated SNP: hCV27474895 / Related Interrogated SNP: hCV27477533 / Related Interrogated SNP: hCV15968043 / SNP Source: dbSNP; HapMap; HGBASE / Population(Allele,Count): Caucasian (C,97|T,23) / SNP Type: INTRON / Context (SEQ ID NO: 759): /
TGGCTGAGCTTCCAGGCTGGAGTTTCTCATTCACTGGATCTGAGGTTGGGCTGAAGAATGTGCATTTCTAACACGTTCCCAGG TGACGCTGTTGGTCTGG
R
GACTGCACTTGACAACCACTGGTTTAAAAACACCATTCACGTTATCATTTGAAGGAGGGTAAGACAGCCTTGTAGTACCACAC AAGGAGGGCTACATTCT / Celera SNP ID: hCV11786295 / Public SNP ID: rs4253421 / SNP Chromosome Position: 187204937 / SNP in Genomic Sequence: SEQ ID NO: 347 / SNP Position Genomic: 27819 / Related Interrogated SNP: hCV15793897 / Related Interrogated SNP: hCV27474895 / Related Interrogated SNP: hCV12066124 / Related Interrogated SNP: hCV25474413 / Related Interrogated SNP: hCV3230038 / Related Interrogated SNP: hCV22272267 / Related Interrogated SNP: hCV27477533 / Related Interrogated SNP: hCV8241630 / Related Interrogated SNP:

hCV30562347 / SNP Source: dbSNP; Celera; HapMap; HGBASE / Population(Allele,Count): Caucasian (A,22|G,204) / SNP Type: INTRON / Context (SEQ ID NO: 760): / AAGATGAAAACTGGAAGAAAGGAGAACAAAGACAGTCTTCACCATTTTGCAGGAATCTACACTCTGCCTATGTGAACACATTT CTTTTGTAAAGAAAGAA
W
TTGATTGCATTTAATGGCAGATTTTCAGAATAGTCAGGAATTCTTGTCATTTCCATTTTAAAATATATATTAAAAAAAAATCAG TTCGAGTAGACACGAGC / Celera SNP ID: hCV11786307 / Public SNP ID: rs1062547 / SNP Chromosome Position: 187210247 / SNP in Genomic Sequence: SEQ ID NO: 347 / SNP Position Genomic: 33129 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV8241630 / Related Interrogated SNP: hCV3230038 / Related Interrogated SNP: hCV3230113 / SNP Source: dbSNP; Celera; HGBASE / Population(Allele,Count): Caucasian (A,76|T,44) / SNP Type: UTR3;INTRON / Context (SEQ ID NO: 761): / ATTTCTCCCCGTGGCTCTGGAGGCTGTAATGCTGGAATCTCGGTGTTGGCAGAACTCGGCTCCCTTCGAGGCCTTAGGAAGGA CCCTGCCTTGCCTCTTC
M
TCGTTCCTCGGCTGCCTCCTTCTCCACGTGGGCCTCTCCCACATTTCTTATCCCTTTCTTGTAAGTAAACTAGTAATATTGGT TAAGAACCCAAACTGCT / Celera SNP ID: hCV11786311 / Public SNP ID: rs13145616 / SNP Chromosome Position: 187215016 / SNP in Genomic Sequence: SEQ ID NO: 347 / SNP Position Genomic: 37898 / Related Interrogated SNP: hCV27474895 / Related Interrogated SNP: hCV3230038 / Related Interrogated SNP: hCV25474413 / SNP Source: dbSNP; Celera / Population(Allele,Count): Caucasian (C,193|A,33) / SNP Type: INTRON / Context (SEQ ID NO: 762): / TTGACGTTCTGAATTGGAATCTTCCCTCTACCATTTACTGACTGGGTCATCTTGGGCAACTTACTGACCCATTCCGTGACGCA GTTCCCATAAGACGGGG
M
TGGTGATGACAATAGTTACCTAAGTCACTGTGAGGGTTAAACGAGTCGTAGAAAGTACATTTTGAGGAGTGCTTGGCATATTT TAGGTGTACAAAGTATC / Celera SNP ID: hCV11786327 / Public SNP ID: rs13133050 / SNP Chromosome Position: 187230737 / SNP in Genomic Sequence: SEQ ID NO: 347 / SNP Position Genomic: 53619 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV3230038 / Related Interrogated SNP: hCV8241630 / Related Interrogated SNP: hCV22272267 / Related Interrogated SNP: hCV3230113 / Related Interrogated SNP: hCV15968043 / Related Interrogated SNP: hCV27902808 / SNP Source: dbSNP; Celera; HapMap / Population(Allele,Count): Caucasian (A,69|C,155) / SNP Type: INTRON / Context (SEQ ID NO: 763): / TACTAGGAAGACTGACAATAGAGCAAGCTCAGAAAATTTTTATGGAGGTGATTTAGTTAGAAAATTTGTCTGCAACCTAAGGG CCATGGAGTGTGACTCC
R
TGGTTTATGGGTGTAACTCCGTGGTTTATGAAGAGTACTTTCAAAATAGGAAAATCTTCACAACTAAGTGCTAGCATGAGCTG ACTTTACTTTCTCTAGG / Celera SNP ID: hCV16172935 / Public SNP ID: rs2241817 / SNP Chromosome Position: 187201001 / SNP in Genomic Sequence: SEQ ID NO: 347 / SNP Position Genomic: 23883 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV8241630 / Related Interrogated SNP: hCV3230038 / Related Interrogated SNP: hCV3230113 / SNP Source: dbSNP; HapMap; ABI_Val; HGBASE / Population(Allele,Count): Caucasian (A,143|G,81) / SNP Type: INTRON / Context (SEQ ID NO: 764): / CAAGACGGAAATTAGAACAGAGGTTGCCAGGGGCCGAGGGGCCAGGGGAAGGGCTGAGGGTTGGTGTTTAATGGGCACAGAGC ATCAGCTGGCGAAGTTG
R
AAAGTTCTGGAGGTGGATGGTGCTGATGGTTGCACAGCAATGTAAATACCCTTAGTGCCACAGAACTGTACACTCAAGATGGC TACATGGCACATGGTAT / Celera SNP ID: hCV26038139 / Public SNP ID: rs4253405 / SNP Chromosome Position: 187190810 / SNP in Genomic Sequence: SEQ ID NO: 347 / SNP Position Genomic: 13692 / Related Interrogated SNP: hCV12066124 / Related Interrogated SNP: hCV25474413 / Related Interrogated SNP: hCV27474895

/ Related Interrogated SNP: hCV27477533 / Related Interrogated SNP: hCV3230038 / Related Interrogated SNP: hCV8241630 / Related Interrogated SNP: hCV3230113 / Related Interrogated SNP: hCV3230096 / Related Interrogated SNP: hCV11786258 / Related Interrogated SNP: hCV15968043 / SNP Source: dbSNP; Celera; HapMap; HGBASE / Population(Allele,Count): Caucasian (A,142|G,84) / SNP Type: TRANSCRIPTION FACTOR BINDING SITE;INTRON / Context (SEQ ID NO: 765): / TGCATTTCTACATGAATTTTTGGATCAGCTAGGCTACTTCTGCAATAAAAGACAGCTGAGATTTTGATAAAGATCCATTTGTG GAGAGTAGCCATCTTAA

M

AATATTAAGTCTCCTGATCCATGAATGTAGAATATCTTTCCATTTATTTAGATTTTTAATTTGTTTCATATCTTTAGCATATA AATTTTGTACTTCTTTT / Celera SNP ID: hCV27309991 / Public SNP ID: rs4572916 / SNP Chromosome Position: 187223583 / SNP in Genomic Sequence: SEQ ID NO: 347 / SNP Position Genomic: 46465 / Related Interrogated SNP: hCV12066124 / Related Interrogated SNP: hCV3230038 / Related Interrogated SNP: hCV15793897 / Related Interrogated SNP: hCV25474413 / Related Interrogated SNP: hCV8241630 / Related Interrogated SNP: hCV27474895 / Related Interrogated SNP: hCV27477533 / Related Interrogated SNP: hCV15968043 / SNP Source: dbSNP; Celera; HapMap; ABI_Val; HGBASE / Population(Allele,Count): Caucasian (A,97|C,23) / SNP Type: INTRON / Context (SEQ ID NO: 766): / TAGACTTTGAGATTCAAGGTCAGCTAGAATTAATGGTTAACAGCTGACAGGTGAAAGTGTGGTTAAATGCAGCTTTGGCTAGC AGGCACACAGGCAAAAT

S

AAGTTCTACATCTGTCCCTGTGTATGTCACTTGTTTGAATACGAAATAAAATTAAAAAAAATAAATTCAGTGTATTGAGAAAGC AAGCAATTCTCTCAAGG / Celera SNP ID: hCV27473099 / Public SNP ID: rs3733403 / SNP Chromosome Position: 187187135 / SNP in Genomic Sequence: SEQ ID NO: 347 / SNP Position Genomic: 10017 / Related Interrogated SNP: hCV12066124 / Related Interrogated SNP: hCV25474413 / Related Interrogated SNP: hCV8241630 / Related Interrogated SNP: hCV22272267 / Related Interrogated SNP: hCV27474895 / Related Interrogated SNP: hCV27477533 / SNP Source: dbSNP; HapMap; HGBASE / Population(Allele,Count): Caucasian (C,101|G,15) / SNP Type: UTR5;PSEUDOGENE / Context (SEQ ID NO: 767): / CAAGTAATTCACATTTTTAGATTTTTTTATTGGTAATCTGAGACAAGAAGAAATTTAAAGTAATCTTCACTAAGCCATGAAAGC TCCCAACATTGTTCTCC

R

TGAGAGATGCTGGCCTGCATTTATTCAAAAACAAAAGACCCCTCTGTTGCCAAAGCTCGGAGGGCTTTTCAGAAACGATATAG TTGTAAATTATAATTTT / Celera SNP ID: hCV27482766 / Public SNP ID: rs3775302 / SNP Chromosome Position: 187177814 / SNP in Genomic Sequence: SEQ ID NO: 347 / SNP Position Genomic: 696 / Related Interrogated SNP: hCV22272267 / SNP Source: dbSNP; HapMap; HGBASE / Population(Allele,Count): Caucasian (A,195|G,31) / SNP Type: INTRON / Context (SEQ ID NO: 768): / AGCAACAGAGCAAGACCCTATCTCTAAGTAAATAAATAAAATACAGAACGAGTTCGGTATGCATCCTCACATTGGATTCCTTA CTTAGGTCACTTTCAGC

R

TTGGCCAAACAAAAAGGCTCCAGCTGGGGGTATATATATTCCAGGGAAGTTAAGTTGGCCAAACTTTCCGTTTGCTACTTCAG TATCCTCCGAGTTGTTT / Celera SNP ID: hCV27490984 / Public SNP ID: rs3822058 / SNP Chromosome Position: 187206180 / SNP in Genomic Sequence: SEQ ID NO: 347 / SNP Position Genomic: 29062 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV8241630 / Related Interrogated SNP: hCV3230038 / Related Interrogated SNP: hCV3230113 / SNP Source: dbSNP; HapMap; ABI_Val; HGBASE / Population(Allele,Count): Caucasian (G,143|A,83) / SNP Type: INTRON / Context (SEQ ID NO: 769): / CTGAGATCTCCATGACTGTGTGTTGTGAAATAAAATGGTGAAAGATCACGATTAGCAAGTGTTTTCTTCTGGTTGTGAAACAG AACTGAAAGTAAGTGGT

Y

GAGGTTCCAGCACAGTTCCTGGGATCCCTCTAATTGCACTGCTTCCTCTGGAACTCAGTATATCTCAAAGATGTAATTTCCTC TCCGTGCTGCACCTGGT / Celera SNP ID: hCV27506149 / Public SNP ID: rs3822055 / SNP Chromosome Position: 187179678 / SNP in Genomic Sequence: SEQ ID NO: 347 /

SNP Position Genomic: 2560 / Related Interrogated SNP: hCV22272267 / Related Interrogated SNP: hCV15793897 / Related Interrogated SNP: hCV27902808 / Related Interrogated SNP: hCV15968043 / Related Interrogated SNP: hCV11786258 / Related Interrogated SNP: hCV3230113 / SNP Source: dbSNP; HapMap; HGBASE / Population(Allele,Count): Caucasian (T,39|C,187) / SNP Type: INTERGENIC;UNKNOWN /
Context (SEQ ID NO: 770): /
CAAGAGCCAGGCTCTTAGGAGCTGTCTGTACTTTGCTTCCCATCCGTCCGCTCTCCCCCAGCAGCCAAAGTCTCCTCCCTCCA TTATATTGCAAATCAAT
K
AATCCATTAGACTTTCCATGTTTTCTTTTTAGACTTTGAGATTCAAGGTCAGCTAGAATTAATGGTTAACAGCTGACAGGTGA AAGTGTGGTTAAATGCA / Celera SNP ID: hCV27521729 / Public SNP ID: rs3822056 / SNP Chromosome Position: 187187005 / SNP in Genomic Sequence: SEQ ID NO: 347 / SNP Position Genomic: 9887 / Related Interrogated SNP: hCV12066124 / Related Interrogated SNP: hCV25474413 / Related Interrogated SNP: hCV8241630 / Related Interrogated SNP: hCV27477533 / Related Interrogated SNP: hCV3230038 / Related Interrogated SNP: hCV22272267 / SNP Source: dbSNP; HapMap; HGBASE / Population(Allele,Count): Caucasian (G,198|T,28) / SNP Type: INTERGENIC;UNKNOWN /
Context (SEQ ID NO: 771): /
AACGCTCACCCCACTCCCATCCTCCCATCTAGTGGCATATTGAAAATCAATTTGTCTTGTAAAATTAAATAATCCAATTAGGA GGGGGATATATTCTAAG
R
AAATTAGTGCATGATGCACACACACACACACACAGAACACGTGTGTGCGCATGTGCACATGAGAGAGAGTGAGAGAGAAAC TGGGTCTTGCTCTGTCG / Celera SNP ID: hCV2103343 / Public SNP ID: rs4241824 / SNP Chromosome Position: 187191787 / SNP in Genomic Sequence: SEQ ID NO: 347 / SNP Position Genomic: 14669 / Related Interrogated SNP: hCV12066124 / Related Interrogated SNP: hCV15968043 / Related Interrogated SNP: hCV22272267 / Related Interrogated SNP: hCV25474413 / Related Interrogated SNP: hCV27477533 / Related Interrogated SNP: hCV3230038 / Related Interrogated SNP: hCV8241630 / Related Interrogated SNP: hCV3230113 / Related Interrogated SNP: hCV27474895 / Related Interrogated SNP: hCV11786258 / Related Interrogated SNP: hCV3230096 / Related Interrogated SNP: hCV25990131 / SNP Source: dbSNP / Population(Allele,Count): Caucasian (G,108|A,116) / SNP Type: TRANSCRIPTION FACTOR BINDING SITE;INTRON /
Context (SEQ ID NO: 772): /
TGCTAACAGGCCATGGACCAGGTACTGTTCTGGGCCCCGGGGGGTTGGGAACCCCTGCTCAGAGACACACCGGGTGGTAGGAGG AGCTAAAGGTGGAGAGG
S
TGAAGGAAAATATGAGGTCTGGGCTATCCACAAACCAGATAGCAGGAAACTGAAGAGTTAAACATTTACTTCAGAATTGAATG GCTTTTGTAAAATCTGG / Celera SNP ID: hCV28960679 / Public SNP ID: rs6844764 / SNP Chromosome Position: 187181533 / SNP in Genomic Sequence: SEQ ID NO: 347 / SNP Position Genomic: 4415 / Related Interrogated SNP: hCV11786258 / Related Interrogated SNP: hCV15968043 / Related Interrogated SNP: hCV27477533 / Related Interrogated SNP: hCV3230038 / Related Interrogated SNP: hCV27474895 / Related Interrogated SNP: hCV3230096 / Related Interrogated SNP: hCV8241630 / Related Interrogated SNP: hCV12066124 / Related Interrogated SNP: hCV3230113 / Related Interrogated SNP: hCV25474413 / Related Interrogated SNP: hCV25990131 / Related Interrogated SNP: hCV32291301 / SNP Source: dbSNP; HapMap / Population(Allele,Count): Caucasian (G,61|C,53) / SNP Type: INTERGENIC;UNKNOWN /
Context (SEQ ID NO: 773): /
CTGGAGACTGCACTTGACAACCACTGGTTTAAAAACACCATTCACGTTATCATTTGAAGGAGGGTAAGACAGCCTTGTAGTAC CACACAAGGAGGGCTAC
R
TTCTTAGGGGTGTGTAATTACAAGATGACTTAGTCAATTCCATTTTTTCATGTGCATGTTTTGCTTTGGCAGCTTGATTATAAA GTCTCTGTAACTCAGGG / Celera SNP ID: hCV32291287 / Public SNP ID: rs4253423 / SNP Chromosome Position: 187205033 / SNP in Genomic Sequence: SEQ ID NO: 347 / SNP Position Genomic: 27915 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV3230038 / Related Interrogated SNP: hCV8241630 / Related Interrogated SNP: hCV3230113 / Related Interrogated SNP: hCV22272267 / Related Interrogated SNP:

hCV32291301 / Related Interrogated SNP: hCV15968043 / SNP Source: dbSNP; Celera; HapMap; HGBASE / Population(Allele,Count): Caucasian (A,186|G,40) / SNP Type: INTRON /
Context (SEQ ID NO: 774): /
TTCTAACACGTTCCCAGGTGACGCTGTTGGTCTGGAGACTGCACTTGACAACCACTGGTTTAAAAACACCATTCACGTTATCA
TTTGAAGGAGGGTAAGA
S
AGCCTTGTAGTACCACACAAGGAGGGCTACATTCTTAGGGGTGTGTAATTACAAGATGACTTAGTCAATTCCATTTTTCATGT
GCATGTTTTGCTTTGGC / Celera SNP ID: hCV32291286 / Public SNP ID: rs4253422 /
SNP Chromosome Position: 187205002 / SNP in Genomic Sequence: SEQ ID NO: 347 /
 SNP Position Genomic: 27884 / Related Interrogated SNP: hCV12066124 /
Related Interrogated SNP: hCV25474413 / Related Interrogated SNP: hCV27474895
/ Related Interrogated SNP: hCV27477533 / Related Interrogated SNP:
hCV3230038 / Related Interrogated SNP: hCV8241630 / Related Interrogated SNP:
hCV3230113 / Related Interrogated SNP: hCV22272267 / Related Interrogated SNP:
 hCV32291301 / Related Interrogated SNP: hCV15968043 / SNP Source: dbSNP;
Celera; HapMap; HGBASE / Population(Allele,Count): Caucasian (C,186|G,40) / SNP
Type: INTRON /
Context (SEQ ID NO: 775): /
ATTTCTTTGATTATCAAAAGTACACTAGCTAAGGTTGCTGTCCCTCTTTTATTTATTTATTTATTTGAGACAGGGTCTTGCTC
TGTCACTCAGATTTGGT
Y
GCACTGGGTGTGATCTCAGCCCACTGCAGCCTCCACTTCCTAGGCTCAAGCAATCCACCCGTCTCATCCTCCCAAGTTGCTGG
GACCACAGGTGTGCACC / Celera SNP ID: hCV32291269 / Public SNP ID: rs4253417 /
SNP Chromosome Position: 187199005 / SNP in Genomic Sequence: SEQ ID NO: 347 /
 SNP Position Genomic: 21887 / Related Interrogated SNP: hCV12066124 /
Related Interrogated SNP: hCV25474413 / Related Interrogated SNP: hCV27477533
/ Related Interrogated SNP: hCV8241630 / Related Interrogated SNP:
hCV27474895 / Related Interrogated SNP: hCV3230038 / Related Interrogated SNP:
 hCV11786258 / Related Interrogated SNP: hCV15968043 / Related Interrogated
SNP: hCV3230096 / Related Interrogated SNP: hCV3230113 / Related Interrogated
SNP: hCV22272267 / SNP Source: dbSNP; HapMap; HGBASE /
Population(Allele,Count): Caucasian (T,129|C,97) / SNP Type: INTRON /
Context (SEQ ID NO: 776): /
AAATAAACTTACACAATTCACAGGTGCTTAGCAACACTGCTGGGACCATGCCCAGCCATTCAGCCTCCCAGATGGATGCTTCG
GGGTCTCGCAGGTCCTC
Y
CTCCAAAGGGGACTTTCTTAATATCTCATGTTTTTTTCCTCCTTGCAGTTGGAAGAATAAGACACTTTTCCTTTTTCTTTTTAT
TCAGTAACATTTGTCTA / Celera SNP ID: hCV30695262 / Public SNP ID: rs4253414 /
SNP Chromosome Position: 187196853 / SNP in Genomic Sequence: SEQ ID NO: 347 /
 SNP Position Genomic: 19735 / SNP Source: dbSNP; HapMap /
Population(Allele,Count): Caucasian (T,117|C,3) / SNP Type: INTRON /
Context (SEQ ID NO: 777): /
TCCAAAATTAACCATCACATAAATTATTTGAGCCAATTAATTAAATGAGTAAGAAGTCTCGATACAACGTCAATATACAACAG
CAAGCACTTGCTCGTTC
K
CAAAAGCAGATGAACTAGTTTTTACTGACACTAGAAGCAGCATGTGTTCCATGTGTTCCACATCCATGCACTCAAACAACCTT
GACGTGAAATATTTTTT / Celera SNP ID: hCV32291256 / Public SNP ID: rs4253406 /
SNP Chromosome Position: 187191392 / SNP in Genomic Sequence: SEQ ID NO: 347 /
 SNP Position Genomic: 14274 / Related Interrogated SNP: hCV27477533 /
Related Interrogated SNP: hCV8241630 / Related Interrogated SNP: hCV3230038 /
Related Interrogated SNP: hCV12066124 / Related Interrogated SNP: hCV27474895
/ Related Interrogated SNP: hCV25474413 / SNP Source: dbSNP; HapMap; HGBASE /
 Population(Allele,Count): Caucasian (G,211|T,15) / SNP Type: INTRON /
Context (SEQ ID NO: 778): /
ATAAACAATTGACCCTGAATCAGGGGTTCCACATCCATGGATTTAAACAACATTGAAGTGAAATTTTTTAAAAAAAATTATAT
CTGCACTCAACATGTGC
R
GAGTTTTTTCTTGTCATTATTCCCGAAACAACACAGTATAACAACAATTTGCATAGTATTTACATTGTACTAGGTATTATAAG
TAATCTAGAGATGATTT / Celera SNP ID: hCV32291246 / Public SNP ID: rs4253403 /
SNP Chromosome Position: 187189326 / SNP in Genomic Sequence: SEQ ID NO: 347 /
 SNP Position Genomic: 12208 / Related Interrogated SNP: hCV15793897 / SNP
Source: dbSNP; HapMap; HGBASE / Population(Allele,Count): Caucasian

(A,112|G,2) / SNP Type: INTRON /
Context (SEQ ID NO: 779): /
TAGAAGAGGTTTCACCAGATTTGTATTCACGTTGCTGAATCTCAGTCCACCCATCTCTAGCTGGAAATAGACTACAAAGTCCC
TTTGTGATACTAACAGT
R
TATAGGCACTTACACTTTTGAAATTAGGCAATGTGAGTTCTCCAACTTAATTCTCTTTTTCCATATAATTTTGGCTATTCTTA
TTCTTATTTTACAGAGG / Celera SNP ID: hCV30307525 / Public SNP ID: rs10025152 /
SNP Chromosome Position: 187225304 / SNP in Genomic Sequence: SEQ ID NO: 347 /
SNP Position Genomic: 48186 / Related Interrogated SNP: hCV27474895 /
Related Interrogated SNP: hCV12066124 / Related Interrogated SNP: hCV15793897
/ Related Interrogated SNP: hCV25474413 / Related Interrogated SNP:
hCV3230038 / Related Interrogated SNP: hCV15968043 / SNP Source: dbSNP;
HapMap / Population(Allele,Count): Caucasian (G,189|A,37) / SNP Type: INTRON
/
Context (SEQ ID NO: 780): /
AGCCCCGGGGCCAGGTCCACGGTGCCCACGCGCGCTGTGTTCTGCTGGGGTCCGGCTTAGGGCTGCTTCGGCCCTCGGCGGCT
TCCTCTGCGCTGTTGCT
R
CCCTCCCCACACCCCTACCCCTGCTCACTCAGCTCCTTCACCTCTTCCCTGATGAACTGGGGAACAGTATCCCCAACCTTGAT
CCCGCAATGTGATTGTTA / Celera SNP ID: hCV32209636 / Public SNP ID: rs11132387 /
SNP Chromosome Position: 187218723 / SNP in Genomic Sequence: SEQ ID NO: 347 /
SNP Position Genomic: 41605 / Related Interrogated SNP: hCV12066124 /
Related Interrogated SNP: hCV25474413 / Related Interrogated SNP: hCV27477533
/ Related Interrogated SNP: hCV8241630 / Related Interrogated SNP:
hCV27474895 / Related Interrogated SNP: hCV3230038 / Related Interrogated SNP:
hCV11786258 / Related Interrogated SNP: hCV15968043 / Related Interrogated
SNP: hCV3230096 / Related Interrogated SNP: hCV22272267 / Related
Interrogated SNP: hCV3230113 / SNP Source: dbSNP; HapMap /
Population(Allele,Count): Caucasian (G,68|A,50) / SNP Type: INTRON /
Context (SEQ ID NO: 781): /
AAAAACACCTCATGTGAAGAGAGATCTCTCTGCTACCCTTGAAGATCAGAATAACATAAGTTCAAAGTTTCTCTGGTATCCAG
TGAAATCTGCTCCTTCG
R
TTTTTTTGCATTGACCAAATTGCTAATGGTGAGGAAGTAACGAGACCAATGGAAGATGTTATTTGGCACATTAATTATTGGGG
ACCCATAAGCTGTTAGA / Celera SNP ID: hCV32209638 / Public SNP ID: rs12507040 /
SNP Chromosome Position: 187217373 / SNP in Genomic Sequence: SEQ ID NO: 347 /
SNP Position Genomic: 40255 / Related Interrogated SNP: hCV12066124 /
Related Interrogated SNP: hCV25474413 / Related Interrogated SNP: hCV27474895
/ Related Interrogated SNP: hCV27477533 / Related Interrogated SNP:
hCV3230038 / Related Interrogated SNP: hCV8241630 / Related Interrogated SNP:
hCV11786258 / Related Interrogated SNP: hCV3230096 / Related Interrogated SNP:
hCV3230113 / Related Interrogated SNP: hCV15968043 / SNP Source: dbSNP;
HapMap / Population(Allele,Count): Caucasian (G,155|A,71) / SNP Type: INTRON
/
Context (SEQ ID NO: 782): /
TTTCCCTTTTAATGAACTTTTTTGACTATGCAACGACCAGTTCCGGTGGGTATATGACAGGGGAGCCCTGTCACAGCGCTACC
CAGGACCCAGTTCCGGT
R
GGTATGACAGGGGAGGCCTGCCACAGTGTTGTCCAGGGCTTTGCAGATGGTTTGGAATTTCCCTATCAGTGCACTTGAACACC
TTTATGGAGGTCTGTCC / Celera SNP ID: hCV32209629 / Public SNP ID: rs79593728 /
SNP Chromosome Position: 187225073 / SNP in Genomic Sequence: SEQ ID NO: 347 /
SNP Position Genomic: 47955 / Related Interrogated SNP: hCV25474413 /
Related Interrogated SNP: hCV27474895 / Related Interrogated SNP: hCV3230038 /
Related Interrogated SNP: hCV12066124 / Related Interrogated SNP: hCV8241630
/ Related Interrogated SNP: hCV27477533 / Related Interrogated SNP:
hCV15793897 / SNP Source: dbSNP; HapMap / Population(Allele,Count): Caucasian
(G,25|A,85) / SNP Type: INTRONIC INDEL;INTRON /
Context (SEQ ID NO: 783): /
ATAATAATAATATCATCTTGATGAACAAGAACAGGAAAGAGAATACAGATAAGACTATCGCCATATTCACGTCTTTTGGGATC
TTGCTATGTGCTCTGCA
Y
CATGCAGTGGGCTTTCCTTCTCACCAGCACTGTGCAAGTAGGATATTATTATTATTCCCTTTATAACATAATCCACGTGTTTT
ACAAGTGGAAAAGAAAG / Celera SNP ID: hCV32209637 / Public SNP ID: rs13143773 /

157

SNP Chromosome Position: 187218202 / SNP in Genomic Sequence: SEQ ID NO: 347 / SNP Position Genomic: 41084 / Related Interrogated SNP: hCV12066124 / Related Interrogated SNP: hCV25474413 / Related Interrogated SNP: hCV27474895 / Related Interrogated SNP: hCV27477533 / Related Interrogated SNP: hCV8241630 / Related Interrogated SNP: hCV3230038 / Related Interrogated SNP: hCV3230113 / SNP Source: dbSNP; HapMap / Population(Allele,Count): Caucasian (T,81|C,39) / SNP Type: INTRON /
Context (SEQ ID NO: 784): /
CATGTATGAGTTTTTGTGTAGACACGTTTATCTTTCTGTTGAGTGTGTAGTACCCAGGAGCAGAATTTCTGGGTCATATGGGA
ACACTATGTTTCATATT
Y

TGAAGAATGATGTTTTTGAAAGCAGCTACACCATTTTACATTCCCACCAGCAATGTCTGAAGCCTCCAATGTCACCACATCCT
GGCTAACATCGATGATT / Celera SNP ID: hCV29640635 / Public SNP ID: rs10029715 / SNP Chromosome Position: 187222600 / SNP in Genomic Sequence: SEQ ID NO: 347 / SNP Position Genomic: 45482 / Related Interrogated SNP: hCV27474895 / Related Interrogated SNP: hCV12066124 / Related Interrogated SNP: hCV15793897 / Related Interrogated SNP: hCV25474413 / Related Interrogated SNP: hCV3230038 / Related Interrogated SNP: hCV15968043 / SNP Source: dbSNP; HapMap / Population(Allele,Count): Caucasian (T,189|C,37) / SNP Type: INTRON /
Context (SEQ ID NO: 785): /
TTGCTGCCCTCCCCACACCCCTACCCCTGCTCACTCAGCTCCTTCACCTCTTCCCTGATGAACTGGGGAACAGTATCCCCAAC
CTTGATCCCGCAATGTA
K

TGTTAAGGCTGTGTTGTATTTTTAAAACAAACAAACATAAACATTTGAATGCTGAAGGCTTAGTGATAATAAAAATAGTCTAC
CCACCCCCCCACATCAC / Celera SNP ID: hCV32209635 / Public SNP ID: rs6848311 / SNP Chromosome Position: 187218818 / SNP in Genomic Sequence: SEQ ID NO: 347 / SNP Position Genomic: 41700 / Related Interrogated SNP: hCV27474895 / Related Interrogated SNP: hCV15793897 / SNP Source: dbSNP; HapMap; ABI_Val / Population(Allele,Count): Caucasian (T,44|G,182) / SNP Type: INTRON //

Gene Number: 11 / Gene Symbol: FGA - 2243 / Gene Name: fibrinogen alpha chain / Chromosome: 4 / OMIM NUMBER: 134820 / OMIM Information: Dysfibrinogenemia, alpha type, causing bleeding diathesis (3);/Dysfibr / inogenemia, alpha type, causing recurrent thrombosis (3); Amyloidosis, hereditary renal, 105200 (3); / Afibrinogenemia, 202400 (3) // / Genomic Sequence (SEQ ID NO: 348): // / SNP Information /
Context (SEQ ID NO: 786): /
CTGCCAGGATTCCAGGTTCCGGTACTACCAGGTCTAGGGCTCCCAGGGTTTTGGTTTCCAGTACTTCCAGTTCCAGAGCTCCC
AGAGTTCCAGCTTCCAG
Y

ACTTCCAGGTCCAGAGCTCCCAGGTTTCCAGGTTGCAGTCCCTCCAGTCCCAGAGCTCCCAGGGTTTCGGTTTCCAGTACTTC
CAGGTCCAGAGCTCCCA / Celera SNP ID: hCV2892877 / Public SNP ID: rs6050 / SNP Chromosome Position: 155507590 / SNP in Genomic Sequence: SEQ ID NO: 348 / SNP Position Genomic: 13310 / SNP Source: Applera / Population(Allele,Count): Caucasian (C,10|T,28) African American (C,2|T,22) total (C,12|T,50) / SNP Type: MISSENSE MUTATION;ESE;SILENT MUTATION;INTRON / SNP Source: Applera / Population(Allele,Count): Caucasian (C,13|T,27) African American (C,7|T,29) total (C,20|T,56) / SNP Type: MISSENSE MUTATION;ESE;SILENT MUTATION;INTRON / SNP Source: HGMD; dbSNP; Celera; HapMap / Population(Allele,Count): Caucasian (T,171|C,53) / SNP Type: MISSENSE MUTATION;ESE;SILENT MUTATION;INTRON /
Context (SEQ ID NO: 787): /
ATAACATTTAGCATAAAATGAGGACTCAATTACTACTGATGGTTGCCCAATTGTAATTGGTAAATTGGCAAAAAGTGGTGGTT
TTTAATGGTCAATAAAG
R

TACCATGTATCTAGTCTTAGGAAACAAAAGGTTTATTGAAATGCATGTAGATAAATTATCATCAGCATAAAACTGTTATGGAG
TTTTCAACATGGGGTCT / Celera SNP ID: hCV11503414 / Public SNP ID: rs2066865 / SNP Chromosome Position: 155525276 / SNP in Genomic Sequence: SEQ ID NO: 348 / SNP Position Genomic: 30996 / SNP Source: dbSNP; Celera; HapMap; ABI_Val / Population(Allele,Count): Caucasian (G,174|A,50) / SNP Type: UTR3 /
Context (SEQ ID NO: 788): /
AAGTCAGTTGAATTAAACATTTAAGTATTAGTATTCTTTCAAACAGCTCTACCTCAGATAGCATTTGTAGCAACCTTATATTA
TATGTATACATGCTGTA

W
CCACTGCATTAGATTGTAAACTGATTTCTGTGGCTACGGCATCTAACATATATAGCTGTACTTAACATAGTTGTAGTTAACAC
TCAATAACTATGTAATG / Celera SNP ID: hCV11503469 / Public SNP ID: rs2066854 /
SNP Chromosome Position: 155535181 / SNP in Genomic Sequence: SEQ ID NO: 348 /
SNP Position Genomic: 40901 / SNP Source: dbSNP; HapMap; HGBASE /
Population(Allele,Count): Caucasian (A,32|T,92) / SNP Type: INTRON /
Context (SEQ ID NO: 789): /
ACTTTACTGGACATCCTGTACTTTATCTGGCAATCCTACCTTGCCTCAAATTCTGCTTCCTACATGAAGCCAATCCTAATAGA
GTAATAGCTAATGTGTA
Y
TGAATGTTTACTAAGTCTTGGGTTCTTTGCTAGACATTTTGCAGGGTACCTGATTCAATGTCCAATTAAAAGGAATCTCATGT
TCTTTCATACTTCCTTA / Celera SNP ID: hCV15860433 / Public SNP ID: rs2070006 /
SNP Chromosome Position: 155513866 / SNP in Genomic Sequence: SEQ ID NO: 348 /
SNP Position Genomic: 19586 / SNP Source: dbSNP; HapMap; HGBASE /
Population(Allele,Count): Caucasian (T,86|C,140) / SNP Type:
INTERGENIC;UNKNOWN /
Context (SEQ ID NO: 790): /
GCTAAATGTCCTTAATAGAAGACTTGAAATCAATAAATATAGTAAGAGAAAAAAGGAAGAAACTTTCAGAGAATTTCTGAAAC
TTTGTGGGTCAATAGAA
R
TTAGCAGTTAATTTTCTACAAATCATCCTCAGGGTAAAGTGAGTCATATTCTGTTTCCGCAGGGTGCTCTGGTCTGACCTGTT
TGGCTCCCCCCAGGTGG / Celera SNP ID: hCV2892863 / Public SNP ID: rs1049636 /
SNP Chromosome Position: 155525970 / SNP in Genomic Sequence: SEQ ID NO: 348 /
SNP Position Genomic: 31690 / Related Interrogated SNP: hCV11503469 /
Related Interrogated SNP: hCV11503414 / Related Interrogated SNP: hCV2892877 /
SNP Source: Applera / Population(Allele,Count): Caucasian (A,18|G,0) African
American (A,9|G,5) total (A,27|G,5) / SNP Type: UTR3;INTRON;PSEUDOGENE / SNP
Source: dbSNP; HapMap; HGBASE / Population(Allele,Count): Caucasian
(G,67|A,159) / SNP Type: UTR3;INTRON;PSEUDOGENE /
Context (SEQ ID NO: 791): /
CCACCACACTTAGGACCAGGCAGACGATCCTCATGGAAAACATCTTTTCTAAGGGTGGGGCTGGCTCCTGAGGAGCACTCCAG
CTGAAAGAAAGGATTGC
Y
GCCACTCCTAGTTCCCATCCTATTTAAATCTGCAAGAGGTTTGGTTAATCATTGGCTTTGTCCTGTGTAGACAGTCAACCCTC
CCTCTACGTCCTTTGTT / Celera SNP ID: hCV2892870 / Public SNP ID: rs2070011 /
SNP Chromosome Position: 155511897 / SNP in Genomic Sequence: SEQ ID NO: 348 /
SNP Position Genomic: 17617 / Related Interrogated SNP: hCV11503414 /
Related Interrogated SNP: hCV11503469 / Related Interrogated SNP: hCV15860433
/ Related Interrogated SNP: hCV2892877 / Related Interrogated SNP:
hCV11503470 / SNP Source: Applera / Population(Allele,Count): Caucasian
(C,23|T,15) African American (C,24|T,12) total (C,47|T,27) // / SNP Type:
ESS;ESE;UTR5;SILENT RARE CODON;SILENT MUTATION / SNP Source: Applera /
Population(Allele,Count): Caucasian (C,24|T,16) African American (C,20|T,12)
total (C,44|T,28) // / SNP Type: ESS;ESE;UTR5;SILENT RARE CODON;SILENT
MUTATION / SNP Source: dbSNP; Celera; HGBASE / Population(Allele,Count):
Caucasian (T,84|C,142) / SNP Type: ESS;ESE;UTR5;SILENT RARE CODON;SILENT
MUTATION /
Context (SEQ ID NO: 792): /
GGCTTGATGAGTAATTGGATATAGGGAGGTAGAAATTGGATATGGGGAGGAAGATTGGAGGATGAGTCACTGTTTTTTTTTGCA
TTTGAGGACTGGATGGA
R
TAGAACAGGTTTGGGAGAGTGGAGAACACTAAGCATACCATGTACTATATCCCCAAATCCAACACAATGGCTACCACAAGGAA
GGCATTCAATAAATGTT / Celera SNP ID: hCV286004 / Public SNP ID: rs1118824 /
SNP Chromosome Position: 155524009 / SNP in Genomic Sequence: SEQ ID NO: 348 /
SNP Position Genomic: 29729 / Related Interrogated SNP: hCV11503469 /
Related Interrogated SNP: hCV11503414 / Related Interrogated SNP: hCV2892877 /
SNP Source: dbSNP; Celera; HapMap / Population(Allele,Count): Caucasian
(G,67|A,159) / SNP Type: INTERGENIC;UNKNOWN /
Context (SEQ ID NO: 793): /
CCCCCTAATGAATCACGGTTACCATGAGAAGGATGACCTCATGTTATTGAAGAAAAGTCCTGGTCTGAGATTCAGGAAACAAG
ATTCAGATGGCTTGTGT
Y
CAGTGTTTAGTTCTGGACCCATTTCTGGGGCTAAGTGGCTATGTGATTTTGGTTGTTCAATTTATTTAACTTCTCAGAACCTT

GGTTTTCTCATTTCAGG / Celera SNP ID: hCV2892850 / Public SNP ID: rs10050268 / SNP Chromosome Position: 155549354 / SNP in Genomic Sequence: SEQ ID NO: 348 / SNP Position Genomic: 55074 / Related Interrogated SNP: hCV11503469 / Related Interrogated SNP: hCV11503414 / SNP Source: dbSNP; Celera; HapMap; ABI_Val / Population(Allele,Count): Caucasian (C,190|T,36) / SNP Type: INTRON / Context (SEQ ID NO: 794): / ATACACATTATATATACATATACATACATATGAAACCTTTGCTGTTAGATATGTCAGATAATACAGAATGAGTGGTAATCATT TAACACATTTCATGTTC Y

GTGGTGCCAGAAATGGGTAGGCTGTCTAGTAAATAGATTTTGTTAATTAGTACATTCTGTGATATAATCTAGTTGTGCTGAAT TTCCTGAGATGCTTGGA / Celera SNP ID: hCV2892855 / Public SNP ID: rs6536024 / SNP Chromosome Position: 155543369 / SNP in Genomic Sequence: SEQ ID NO: 348 / SNP Position Genomic: 49089 / Related Interrogated SNP: hCV11503414 / Related Interrogated SNP: hCV11503469 / Related Interrogated SNP: hCV2892877 / SNP Source: dbSNP; Celera; HapMap / Population(Allele,Count): Caucasian (T,99|C,127) / SNP Type: INTRON / Context (SEQ ID NO: 795): / CCTAACCAAACAGTACAACTTTAACAAAGGTCAGTCAAATCCTAGAAGATAGGGAAATGAGGGAGTTTATAGCATTGTGTTGT GCATATTCTCTAGAATA Y

GCAGAACTAGGGCTAGAATGTGTCATAGAGAAAGCCAAGTAACCTTCACCTGGCCCTTTCCTTAGGTTTGTCTGTCTTGGTCC CATGCTTTAGCTCAGCT / Celera SNP ID: hCV2892858 / Public SNP ID: rs12648395 / SNP Chromosome Position: 155541289 / SNP in Genomic Sequence: SEQ ID NO: 348 / SNP Position Genomic: 47009 / Related Interrogated SNP: hCV11503469 / Related Interrogated SNP: hCV11503414 / Related Interrogated SNP: hCV2892877 / SNP Source: dbSNP; Celera; HapMap / Population(Allele,Count): Caucasian (C,67|T,159) / SNP Type: INTRON / Context (SEQ ID NO: 796): / AGACTAAGGAATGATCTGAGGTGCCCTGGATTGAGACAAATTGAACAGAGAAGTTGGATTTAAGTGTTTTGCTGTGCCACATT GGTATCTGCTACTGGTT K

GTTTGTGTTTTTCCATCTTTGTGTAATCTACGATTTTAAATCTCTCACCCCATGAAAGAATTTTCATGACTAAATATGATTAG CAGGAGATTTACAAGAG / Celera SNP ID: hCV2892859 / Public SNP ID: rs13130318 / SNP Chromosome Position: 155538470 / SNP in Genomic Sequence: SEQ ID NO: 348 / SNP Position Genomic: 44190 / Related Interrogated SNP: hCV11503414 / Related Interrogated SNP: hCV11503469 / Related Interrogated SNP: hCV15860433 / Related Interrogated SNP: hCV2892877 / Related Interrogated SNP: hCV11503470 / SNP Source: dbSNP; Celera; HapMap / Population(Allele,Count): Caucasian (T,74|G,30) / SNP Type: INTRON / Context (SEQ ID NO: 797): / GGTTTTTCCACGCAGTACCTTATAACTCCTGGACAATGTGCATTTGGGAAGGTATTTGTTTATTCTGTGTCTTGCCTACACAA AGCTTCCTGAGGCCAAG R

CACAAGGACTGGCACAAATGTCAGGCATAGAGCAATTGCTGCTCAGTATCTGCTGGAGAAATGGAAGGCTGTCACCATATGGT GGTGACAATGCATGGTG / Celera SNP ID: hCV2892869 / Public SNP ID: rs13109457 / SNP Chromosome Position: 155514879 / SNP in Genomic Sequence: SEQ ID NO: 348 / SNP Position Genomic: 20599 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera; HapMap / Population(Allele,Count): Caucasian (G,82|A,32) / SNP Type: INTERGENIC;UNKNOWN / Context (SEQ ID NO: 798): / AAATAATACCTACTTTTAGGTAGATGATGGATATAACACTTTTCTCTGCATATAGTAGACACTCAGTGCATAACTATCGCCTT CCTTTTCCCTCTACTCA R

AAACAAGGACATCTGGGACCACAGCCACATACTTACCTCCAGTCGTTTCATATCAACCAACTGAGCTCTAACATTTTTTCTGCA GAAGCTGGATATGCTGT / Celera SNP ID: hCV2892876 / Public SNP ID: rs2070018 / SNP Chromosome Position: 155508627 / SNP in Genomic Sequence: SEQ ID NO: 348 / SNP Position Genomic: 14347 / Related Interrogated SNP: hCV15860433 / Related Interrogated SNP: hCV11503414 / SNP Source: dbSNP; Celera; HapMap; HGBASE / Population(Allele,Count): Caucasian (G,16|A,104) / SNP Type: INTRON /

Context                    (SEQ ID NO: 799): /
TTCAGTGAGTTTTACCTTAAAATATTGGCTTAAAACAATTCAAATCAAATGGATAAAACAACTTTTTGCTTTCCAATGGCATT
ATGATTTTGGGTGGGTAT
R
GGAAGAGAAGTTCTCAAACCAATAGTCTTCAAGGTTGCAGAGAGAAGCTTCTGTCTGCCACTTTCCTGATGTACTGAATGGCC
CTTGGTATATCTTTATA / Celera SNP ID:    hCV2892878 / Public SNP ID:    rs2070022 /
SNP Chromosome Position:    155504948 / SNP in Genomic Sequence:    SEQ ID NO: 348 /
 SNP Position Genomic:    10668 / Related Interrogated SNP:    hCV11503469 /
Related Interrogated SNP:    hCV15860433 / SNP Source:    dbSNP; Celera; HapMap;
ABI_Val; HGBASE / Population(Allele,Count):    Caucasian (G,194|A,32) / SNP Type:
 UTR3 /
Context                    (SEQ ID NO: 800): /
ACTGACTTCCCCCATGTCCCAGTTCACTATTCCCAGGAATGAATGACCCTGGAAATACATTCCTGTCCCTCTGTGTCAACCAT
GTTCATAGGCTCTCACT
Y
CTCAGGGAGCTTTTGAGCTCAATCTTCCCTTTGTCTGAAGGTGGGCATGGCACCCCATCTGCCACAGAGAAAAGAGCCAGGAG
CCCACTTCATTGTTCCT / Celera SNP ID:    hCV7429780 / Public SNP ID:    rs1800792 /
SNP Chromosome Position:    155534408 / SNP in Genomic Sequence:    SEQ ID NO: 348 /
 SNP Position Genomic:    40128 / Related Interrogated SNP:    hCV11503414 /
Related Interrogated SNP:    hCV11503469 / Related Interrogated SNP:    hCV15860433
 / Related Interrogated SNP:    hCV2892877 / SNP Source:    dbSNP; HapMap; HGBASE /
Population(Allele,Count):    Caucasian (T,117|C,109) / SNP Type:    INTRON /
Context                    (SEQ ID NO: 801): /
GGAGGAGGATGAGATGGTAGCAGGAGAGGCTGGATGTAAGAAAGGGAACAGGAAGTGGGTGATTGCCCTTATCCTGGCAAGAG
ATGTATCTCAACTACAA
W
GGTGACAATCGGTATACAGGAAAAAAGTGAATATGATAAATATATAGGAGGTGAATTCAGCAGGCTTGATGAGTAATTGGATA
TAGGGAGGTAGAAATTG / Celera SNP ID:    hCV7429782 / Public SNP ID:    rs1118823 /
SNP Chromosome Position:    155523846 / SNP in Genomic Sequence:    SEQ ID NO: 348 /
 SNP Position Genomic:    29566 / Related Interrogated SNP:    hCV11503469 /
Related Interrogated SNP:    hCV11503414 / Related Interrogated SNP:    hCV2892877 /
 SNP Source:    dbSNP; Celera; HapMap; HGBASE / Population(Allele,Count):
Caucasian (A,66|T,160) / SNP Type:    TFBS SYNONYMOUS;INTERGENIC;UNKNOWN /
Context                    (SEQ ID NO: 802): /
ATAGTCAAACTCACGGAAGAAAAGTGTAGAATGGTGGTGGCCAGGGCCTGAGGCATAGGAGAAATGGGGGAGATGTTGGTTAG
AGGGCACAAAGTTTGAG
Y
TATGCAATATAAATAAGTTCTGGAGTCCAATGTGCAGCATGGTGACTATAGTTGATAATATTATGCTGTATATTTGAAATTTG
CTAAAAAGGTAGATCTT / Celera SNP ID:    hCV9317206 / Public SNP ID:    rs2070008 /
SNP Chromosome Position:    155513276 / SNP in Genomic Sequence:    SEQ ID NO: 348 /
 SNP Position Genomic:    18996 / Related Interrogated SNP:    hCV15860433 / SNP
Source:    dbSNP; Celera; HapMap / Population(Allele,Count):    Caucasian
(C,33|T,193) / SNP Type:    INTERGENIC;UNKNOWN /
Context                    (SEQ ID NO: 803): /
GTAATAAACAAGGAAAATACCTGGGAATTTGAAACTCTAAAATGTTCTCCTATTTTATTAAGTACATACTAAAATATTTGATA
TAATGAAAATAATTTAC
R
AAGACAAAATAAATGACAAGTGGTCATAAAAATGCAAATAAAGTCAATCATTTTATTATTATATATTTAGGAACAAAGTTGAA
ATGTTATCTCCTCAAAT / Celera SNP ID:    hCV11503416 / Public SNP ID:    rs2066864 /
SNP Chromosome Position:    155525695 / SNP in Genomic Sequence:    SEQ ID NO: 348 /
 SNP Position Genomic:    31415 / Related Interrogated SNP:    hCV11503414 /
Related Interrogated SNP:    hCV11503469 / Related Interrogated SNP:    hCV15860433
 / Related Interrogated SNP:    hCV2892877 / Related Interrogated SNP:
hCV11503470 / SNP Source:    dbSNP; HapMap; HGBASE / Population(Allele,Count):
Caucasian (G,89|A,31) / SNP Type:    TRANSCRIPTION FACTOR BINDING SITE;UTR3;INTRON
/
Context                    (SEQ ID NO: 804): /
CTCATCCAACCCCCATCCTAAAATAAGTGTCTACTGTCCTCAGACTGGTCTCTTCTCTCAAGCTTTCACAAACCCCTGCCCTT
ACCAGTGCTTGCCTTCT
Y
TTTATTCTGTTCCTCCAAACACCTGTTCACTGCGTGTATCTGACACAAAGCTCATGGGAAGCCAGGTTAATGTGTCAACAAGC
ATCCATGTAGTAATGTT / Celera SNP ID:    hCV11503431 / Public SNP ID:    rs2066861 /
SNP Chromosome Position:    155527436 / SNP in Genomic Sequence:    SEQ ID NO: 348 /

161

SNP Position Genomic: 33156 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera; HapMap; HGBASE / Population(Allele,Count): Caucasian (C,176|T,50) / SNP Type: INTRON / Context (SEQ ID NO: 805): /
CCTGTGCTCTTAACCATGAATCTTAATACAACCATGAAGCCCTTCTTAATTTAAAATAATACACAGATACAAGGTGTTATGAG
ACTCAAAGTTATTCACT
R
ATAGAATGGATTTTAAAGATGTGTGTGTGCATCTACATAACTGTGTGCATGATTATTTGAACACACATTTACATCTATCTACC
AACTTATCTATCTTCTT / Celera SNP ID: hCV11853483 / Public SNP ID: rs12644950 /
SNP Chromosome Position: 155537321 / SNP in Genomic Sequence: SEQ ID NO: 348 /
SNP Position Genomic: 43041 / Related Interrogated SNP: hCV11503414 /
Related Interrogated SNP: hCV11503469 / Related Interrogated SNP: hCV15860433
/ Related Interrogated SNP: hCV2892877 / Related Interrogated SNP:
hCV11503470 / SNP Source: dbSNP; Celera; HapMap / Population(Allele,Count):
Caucasian (G,88|A,28) / SNP Type: INTRON /
Context (SEQ ID NO: 806): /
CATTAGACAATCTCAAATCTTGTATACTTCCTCTTTGTTAGAGTATTTAATACTGAAGATATGCCATAATCATTTCTTGTTGC
AAAAAGAATTTAAAAAC
Y
AACGGTATCAGTTTTTTTTCTATTGAAGTTTAAAATTAGATTTTGGTAATTCAGAAACAAAAAGACAAATACAGCATGTTCTCA
GTTATAAGTGGGAGCTA / Celera SNP ID: hCV11853489 / Public SNP ID: rs7681423 /
SNP Chromosome Position: 155542248 / SNP in Genomic Sequence: SEQ ID NO: 348 /
SNP Position Genomic: 47968 / Related Interrogated SNP: hCV11503414 /
Related Interrogated SNP: hCV11503469 / Related Interrogated SNP: hCV15860433
/ Related Interrogated SNP: hCV2892877 / Related Interrogated SNP:
hCV11503470 / SNP Source: dbSNP; Celera; HapMap; ABI_Val /
Population(Allele,Count): Caucasian (C,89|T,31) / SNP Type: INTRON /
Context (SEQ ID NO: 807): /
GCAAACCTGTTAAAGTGCTTCCAAAATAGAGCTCATTGTGTGCTACTCCCACTTCATTATTATGTTTTTTTCCTTGATCAGCC
TCAAAATCCCTTGAAAA
W
CTCATTCTATCAGGTGAGAAAGGCCTCACCTCAGCAGGGAGAAAGAAATGAATGTCATAAACTCTGGTAACTCTGGTTTTTCA
TATGGTCATGGCCCTAA / Celera SNP ID: hCV11853496 / Public SNP ID: rs7654093 /
SNP Chromosome Position: 155545072 / SNP in Genomic Sequence: SEQ ID NO: 348 /
SNP Position Genomic: 50792 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; Celera; HapMap / Population(Allele,Count):
Caucasian (A,176|T,50) / SNP Type: INTRON /
Context (SEQ ID NO: 808): /
TTCCTACCCTATCTCTTCCAGATAAGTTGGCTTAAAAGTAGGTAAGAGGACTAGTTAGAGGTCGCAGAGAAACTCTGGAATGA
GGGTCCACTTAGCCATA
R
ATTAGGTGCCAGGAAATTGAGGCCACAATACTCAGGAGCCTGGTTTTGCCTGAGCATGCAACTGAAATCCTGTCTGTTCACCC
ACTAATGATCCAGAGTT / Celera SNP ID: hCV15860456 / Public SNP ID: rs2070016 /
SNP Chromosome Position: 155510314 / SNP in Genomic Sequence: SEQ ID NO: 348 /
SNP Position Genomic: 16034 / SNP Source: dbSNP; HapMap; ABI_Val; HGBASE /
Population(Allele,Count): Caucasian (A,187|G,35) / SNP Type: INTRON /
Context (SEQ ID NO: 809): /
ATTGTAAGCTTTGGAGGAGTAGGTTTTGTTTAGCTGGAAAACCATGCAAATTAATAGGCATGATACGAGTATCATTTTTTAAA
AAGCATGTGACAATAAG
S
GTATCTTTAAATGTGCAAAACTGGAAAAAAGATGGAAGAAATTTTCAGTTGCTTAAATTATAATCTGCCGTGCATTGGCTTTT
TCTTTTTTGAGATGGAGT / Celera SNP ID: hCV27020269 / Public SNP ID: rs7659613 /
SNP Chromosome Position: 155515416 / SNP in Genomic Sequence: SEQ ID NO: 348 /
SNP Position Genomic: 21136 / Related Interrogated SNP: hCV11503414 /
Related Interrogated SNP: hCV11503469 / Related Interrogated SNP: hCV15860433
/ Related Interrogated SNP: hCV2892877 / Related Interrogated SNP:
hCV11503470 / SNP Source: dbSNP; Celera; HapMap; ABI_Val /
Population(Allele,Count): Caucasian (C,48|G,72) / SNP Type:
INTERGENIC;UNKNOWN /

Context (SEQ ID NO: 810): / TTTCATGCACTGTATAAGGAAGGAGCCAACCTTATGAAACAGAATTCCTAAAATGCAGAGACCACACACATGCACAGAACCAC AGAGTGAGGTCTTTTTA Y

ACACACACACACAAAAACATGATGAAAATCTCATAACTATAATCCCAGTGCTTTGGGAGGCCAAGGCAAGAGGCCAGGAGTTT GAGCCTCCAGTGAGCTA / Celera SNP ID: hCV27020277 / Public SNP ID: rs6825454 / SNP Chromosome Position: 155501188 / SNP in Genomic Sequence: SEQ ID NO: 348 / SNP Position Genomic: 6908 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera; HapMap / Population(Allele,Count): Caucasian (T,172|C,52) / SNP Type: INTERGENIC;UNKNOWN / Context (SEQ ID NO: 811): / ATATACACATATATAATTATATATATGTATATATAATTATACAATTAGGACTGAAAGTCTGCAACAAACTATAATGGATGTAT GCACTTGGGACATAAGC Y

TCTAGTTCCTCTTTATTTATGAACCAAGAGAAACAGCTAACTCAGGGATTGTTTTTAAACTGACTACAGATTCCCACCATGGG GCAAAAATATGGGTGAC / Celera SNP ID: hCV27020280 / Public SNP ID: rs4463047 / SNP Chromosome Position: 155495533 / SNP in Genomic Sequence: SEQ ID NO: 348 / SNP Position Genomic: 1253 / Related Interrogated SNP: hCV11503414 / Related Interrogated SNP: hCV11503469 / Related Interrogated SNP: hCV11503470 / Related Interrogated SNP: hCV2892877 / Related Interrogated SNP: hCV15860433 / SNP Source: dbSNP; Celera; ABI_val; HGBASE / Population(Allele,Count): Caucasian (T,198|C,28) / SNP Type: INTERGENIC;UNKNOWN / Context (SEQ ID NO: 812): / AATGTTGAATGTATATAAATATTATCTACTAAAATATATATAATGAAAATATGTAATTATTGATCATTCACAAAGATTTACAT AGTACACTGATAATTAC R

TCTTGCTAACTTTTTAATATGATTTGAACATGAAAGGTGACTCAGGCATTGCCTTTGCCTTCAAGAAGTGTGTAGCTCAATTG GAGAATGTATAAATAAA / Celera SNP ID: hCV31863979 / Public SNP ID: rs12186294 / SNP Chromosome Position: 155535483 / SNP in Genomic Sequence: SEQ ID NO: 348 / SNP Position Genomic: 41203 / Related Interrogated SNP: hCV11503414 / Related Interrogated SNP: hCV11503469 / Related Interrogated SNP: hCV15860433 / Related Interrogated SNP: hCV2892877 / Related Interrogated SNP: hCV11503470 / SNP Source: dbSNP; HapMap / Population(Allele,Count): Caucasian (A,116|G,106) / SNP Type: INTRON / Context (SEQ ID NO: 813): / TACGTCTTCCAAACTCTGTACTTGAAATGTGAGCAAGGAGGTGTAAGCACAGGAGAGAGTTTAGATATAGATCCCAAGAAAAT CTTATAGAAAAAAGTTG R

CATTTTAATTCTATGGACAACGAATGCTTTCTTTATTAAAGGATCCCAGTATAACTGGCTACCCATTCACAAGAAGATAAAAG TAGATTTTCTTCATGAC / Celera SNP ID: hCV31863989 / Public SNP ID: rs4308349 / SNP Chromosome Position: 155502113 / SNP in Genomic Sequence: SEQ ID NO: 348 / SNP Position Genomic: 7833 / Related Interrogated SNP: hCV15860433 / Related Interrogated SNP: hCV11503414 / SNP Source: dbSNP; HapMap; HGBASE / Population(Allele,Count): Caucasian (G,32|A,194) / SNP Type: TRANSCRIPTION FACTOR BINDING SITE;INTERGENIC;UNKNOWN / Context (SEQ ID NO: 814): / GGGTGTGATAATATACATTCTTTTCACCTATGAAACATTTGCAAAAGTTAACGCAACTTGTTCACAAAGAAAACCTTAAAAAT TCTATACTTTGGGAGAC M

GAGACAGGAGGATTGCTTGAGGCCAGGAGTTCAAGATCAGCCTGGGCAACATAGCAAGACTCTGTCTCTACAAAAAATGAAAA AATTAGCTGGACGAGGT / Celera SNP ID: hCV29582612 / Public SNP ID: rs4550901 / SNP Chromosome Position: 155501533 / SNP in Genomic Sequence: SEQ ID NO: 348 / SNP Position Genomic: 7253 / Related Interrogated SNP: hCV15860433 / Related Interrogated SNP: hCV11503414 / SNP Source: dbSNP; HapMap; ABI_val; HGBASE / Population(Allele,Count): Caucasian (A,16|C,104) / SNP Type: INTERGENIC;UNKNOWN / Context (SEQ ID NO: 815): / TTTCCTTTTCATTCTGCTTTCAAACCACAAATTCATGTGTGGATAGATCTTAAAAACACAATGCTGAAATAAAAAAGTAGTAA GTATGATGAAGACTTCA R CAGTGATTGTCAAAGGATTTGTGGTGAAGGACTAGTTTTATTAAAAATTCTAATCAGCCACAGACCAGGTTTTTATAGAATTCA

ATAAAAATAATTACTAA / Celera SNP ID: hCV31863982 / Public SNP ID: rs7659024 / SNP Chromosome Position: 155520930 / SNP in Genomic Sequence: SEQ ID NO: 348 / SNP Position Genomic: 26650 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; HapMap; ABI_Val / Population(Allele,Count): Caucasian (G,176|A,50) / SNP Type: INTERGENIC;UNKNOWN / Context (SEQ ID NO: 816): / GGATAGAGATAGCATGAAGTTCTGATGTCTGAAAGAAGTGAAGCTTCATTTGATTCTGAAGAAGTTCTTGTCCAAGTATCCAA CCATTTGAAGGTATAGC R

TTTAGTTTGTATCAGAGATTTACAATTCCCCATAAAGAAATGCAAGAACTCTGGGGTTCATCTGTGATGCATTTGCAAGGTTA TTTCCCCTTTGATCCCC / Celera SNP ID: hDV72277158 / Public SNP ID: rs28673871 / SNP Chromosome Position: 155504038 / SNP in Genomic Sequence: SEQ ID NO: 348 / SNP Position Genomic: 9758 / Related Interrogated SNP: hCV11503469 / Related Interrogated SNP: hCV15860433 / SNP Source: dbSNP / Population(Allele,Count): Caucasian (G,193|A,33) / SNP Type: INTERGENIC;UNKNOWN //

Gene Number: 12 / Gene Symbol: FGB - 2244 / Gene Name: fibrinogen beta chain / Chromosome: 4 / OMIM NUMBER: 134830 / OMIM Information: Dysfibrinogenemia, beta type (3); Afibrinogenemia, congenital,/202400 / (3); Thrombophilia, dysfibrinogenemic (3) // / Genomic Sequence (SEQ ID NO: 349): // / SNP Information / Context (SEQ ID NO: 817): / TACTAAGCTTAGATTTGTTTTCTCACATATTCTTTCGGAGCTTGTGTAGTTTCCACATTAATTTACCAGAAACAAGATACACA TCTCTCTTTGAGGAGTG Y

CCTAACTTCCCATCATTTTGTCCAATTAAATGAATTGAAGAAATTTAATGTTTCTAAACTAGACCAACAAAGAATAATAGTTG TATGACAAGTAAATAAG / Celera SNP ID: hCV11503470 / Public SNP ID: rs1800788 / SNP Chromosome Position: 155483914 / SNP in Genomic Sequence: SEQ ID NO: 349 / SNP Position Genomic: 9782 / SNP Source: CDX; dbSNP / Population(Allele,Count): Caucasian (C,165|T,49) / SNP Type: INTERGENIC;UNKNOWN / Context (SEQ ID NO: 818): / TTTAGAGAGTTCCAGAAGAACTCACACACCAAAAATAAGAGAACAACAACAACAACAAAAATGCTAAGTGGATTTTCCCAACA GATCATAATGACATTAC R

GTACATCATAAAAATATCCTTAGCCAGTTGTGTTTTGGACTGGCCTGGTGCATTTGCTGGTTTTGATGAGCAGGATGGGGCAC AGGTAGTCCCAGGGGTG / Celera SNP ID: hCV2892889 / Public SNP ID: rs2227412 / SNP Chromosome Position: 155489095 / SNP in Genomic Sequence: SEQ ID NO: 349 / SNP Position Genomic: 14963 / Related Interrogated SNP: hCV11503469 / SNP Source: Applera / Population(Allele,Count): Caucasian (A,35|G,3) African American (A,29|G,5) total (A,64|G,8) / SNP Type: INTRON / SNP Source: dbSNP; Celera; HapMap; ABI_Val; HGBASE / Population(Allele,Count): Caucasian (A,199|G,27) / SNP Type: INTRON / Context (SEQ ID NO: 819): / TTCTTAAATTCAAATTTTTTAGCTGGAGTAAAAATGGTCCCAGTACCATTTCCTGTTCCCTTCACTATAACCTACATTTTTGT CACATTAAGTTTTTCCC Y

ATTCCAGGACAGGTCAGGCCCTTTAAAAAATTTCAACAGCTTTATTGAGATATAATTGATATAATTTAAAAAAATCCTGCACATG TGTCATGCTGGAGCCCT / Celera SNP ID: hCV7429783 / Public SNP ID: rs1044291 / SNP Chromosome Position: 155493352 / SNP in Genomic Sequence: SEQ ID NO: 349 / SNP Position Genomic: 19220 / Related Interrogated SNP: hCV15860433 / Related Interrogated SNP: hCV11503469 / Related Interrogated SNP: hCV11503414 / SNP Source: Applera / Population(Allele,Count): Caucasian (C,27|T,7) African American (C,30|T,4) total (C,57|T,11) / SNP Type: MISSENSE MUTATION;UTR3 / SNP Source: dbSNP; HGBASE / Population(Allele,Count): Caucasian (C,151|T,75) / SNP Type: MISSENSE MUTATION;UTR3 / Context (SEQ ID NO: 820): / AAAGACAAGGCACATGGACAGGCAGTTCACAGAAAGACCACTGACATTGTGGCTGATATGTAAATTGAAAAGATTCTCAGTGT GCTCATGATGAAAGAAA W

TTTAATCACAATAACAATAAGATAACATATTTCACCTAATAGGTTGGCAAAAAATTTTAAAATTTGACAACAACAAGTGATGA

CAAGGATGAGGGACAAC / Celera SNP ID: hCV2892893 / Public SNP ID: rs12648258 /
SNP Chromosome Position: 155477372 / SNP in Genomic Sequence: SEQ ID NO: 349 /
SNP Position Genomic: 3240 / Related Interrogated SNP: hCV11503414 / Related
Interrogated SNP: hCV11503469 / Related Interrogated SNP: hCV11503470 /
Related Interrogated SNP: hCV15860433 / Related Interrogated SNP: hCV2892877 /
SNP Source: dbSNP; Celera; HapMap / Population(Allele,Count): Caucasian
(T,91|A,27) / SNP Type: INTERGENIC;UNKNOWN /
Context (SEQ ID NO: 821): /
GGCTTCGCCAAGGTCTCCCCGGCCCCCAGCAAACTACCTGCTATGAAGTAGATGCTCTGTAAATAGTTGTTAAGCACAAATTG
CCTTGTGCTATGGAGAC
Y

GGGCCTGACATGAGTTGTAAAAGTATCAACAAGTGTATTGACAATGGGTATCGGCAAGTCTTTGCCCTTCAAGCCAGAGAGCT
GCTGGGCAGGACAGATT / Celera SNP ID: hCV7429793 / Public SNP ID: rs1025154 /
SNP Chromosome Position: 155481363 / SNP in Genomic Sequence: SEQ ID NO: 349 /
SNP Position Genomic: 7231 / Related Interrogated SNP: hCV11503414 / Related
Interrogated SNP: hCV11503469 / Related Interrogated SNP: hCV11503470 /
Related Interrogated SNP: hCV15860433 / Related Interrogated SNP: hCV2892877 /
SNP Source: dbSNP; HGBASE / Population(Allele,Count): Caucasian (T,178|C,48)
/ SNP Type: INTERGENIC;UNKNOWN /
Context (SEQ ID NO: 822): /
AAAGGAAAAACTGATGTTTAAAAGTCCACTTTTAAAACTATATTTATTTATGTAGGATCTGTCAAAGAAAACTTCCAAAAAGA
TTTATTAATTAAACCAG
M

CTCTGTTGCAATAAGTTAATGTTTTCTTGTTTTGTAATCCACACATTCAATGAGTTAGGCTTTGCACTTGTAAGGAAGGAGAA
GCGTTCACAACCTCAAA / Celera SNP ID: hCV15971616 / Public SNP ID: rs2227421 /
SNP Chromosome Position: 155492224 / SNP in Genomic Sequence: SEQ ID NO: 349 /
SNP Position Genomic: 18092 / Related Interrogated SNP: hCV11503469 / SNP
Source: dbSNP; HGBASE / Population(Allele,Count): Caucasian (A,221|C,48) /
SNP Type: MICRORNA;UTR3 /
Context (SEQ ID NO: 823): /
TTTCATGCACTGTATAAGGAAGGAGCCAACCTTATGAAACAGAATTCCTAAAATGCAGAGACCACACACATGCACAGAACCAC
AGAGTGAGGTCTTTTTA
Y

ACACACACACACAAAAACATGATGAAAATCTCATAACTATAATCCCAGTGCTTTGGGAGGCCAAGGCAAGAGGCCAGGAGTTT
GAGCCTCCAGTGAGCTA / Celera SNP ID: hCV27020277 / Public SNP ID: rs6825454 /
SNP Chromosome Position: 155501188 / SNP in Genomic Sequence: SEQ ID NO: 349 /
SNP Position Genomic: 27056 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; Celera; HapMap / Population(Allele,Count):
Caucasian (T,172|C,52) / SNP Type: INTERGENIC;UNKNOWN /
Context (SEQ ID NO: 824): /
ATATACACATATATAATTATATATATGTATATATAATTATACAATTAGGACTGAAAGTCTGCAACAAACTATAATGGATGTAT
GCACTTGGGACATAAGC
Y

TCTAGTTCCTCTTTATTTATGAACCAAGAGAAACAGCTAACTCAGGGATTGTTTTTAAACTGACTACAGATTCCCACCATGGG
GCAAAAATATGGGTGAC / Celera SNP ID: hCV27020280 / Public SNP ID: rs4463047 /
SNP Chromosome Position: 155495533 / SNP in Genomic Sequence: SEQ ID NO: 349 /
SNP Position Genomic: 21401 / Related Interrogated SNP: hCV11503414 /
Related Interrogated SNP: hCV11503469 / Related Interrogated SNP: hCV11503470
/ Related Interrogated SNP: hCV2892877 / Related Interrogated SNP:
hCV15860433 / SNP Source: dbSNP; Celera; ABI_Val; HGBASE /
Population(Allele,Count): Caucasian (T,198|C,28) / SNP Type:
INTERGENIC;UNKNOWN /
Context (SEQ ID NO: 825): /
TGTTAATGCCTGGAAAGACTTTGGGAATAATTAGATTGTACTATCAGTTATAAACTGTCAGTTATAAATATCTTAGCAAGGTG
TATGTGTGTGTGTGAGA
R

AGAGAGAGAGAGTCCTCGGGTCTTACTTATCACAATTTCACCATCCTCTACTACATGAAGCCCCATCTCATGTGTATTAGTTT
GCTAGGGCTGATGTAAC / Celera SNP ID: hCV30711231 / Public SNP ID: rs12642469 /
SNP Chromosome Position: 155474222 / SNP in Genomic Sequence: SEQ ID NO: 349 /
SNP Position Genomic: 90 / Related Interrogated SNP: hCV11503414 / Related
Interrogated SNP: hCV11503469 / Related Interrogated SNP: hCV11503470 /

Related Interrogated SNP: hCV15860433 / Related Interrogated SNP: hCV2892877 / SNP Source: dbSNP; HapMap; ABI_Val / Population(Allele,Count): Caucasian (G,178|A,48) / SNP Type: INTERGENIC;UNKNOWN / Context (SEQ ID NO: 826): / TACGTCTTCCAAACTCTGTACTTGAAATGTGAGCAAGGAGGTGTAAGCACAGGAGAGAGTTTAGATATAGATCCCAAGAAAAT CTTATAGAAAAAAGTTG

R

CATTTTAATTCTATGGACAACGAATGCTTTCTTTATTAAAGGATCCCAGTATAACTGGCTACCCATTCACAAGAAGATAAAAG TAGATTTTCTTCATGAC / Celera SNP ID: hCV31863989 / Public SNP ID: rs4308349 / SNP Chromosome Position: 155502113 / SNP in Genomic Sequence: SEQ ID NO: 349 / SNP Position Genomic: 27981 / Related Interrogated SNP: hCV15860433 / Related Interrogated SNP: hCV11503414 / SNP Source: dbSNP; HapMap; HGBASE / Population(Allele,Count): Caucasian (G,32|A,194) / SNP Type: TRANSCRIPTION FACTOR BINDING SITE;INTERGENIC;UNKNOWN / Context (SEQ ID NO: 827): / GGGTGTGATAATATACATTCTTTTCACCTATGAAACATTTGCAAAAGTTAACGCAACTTGTTCACAAAGAAAACCTTAAAAAT TCTATACTTTGGGAGAC

M

GAGACAGGAGGATTGCTTGAGGCCAGGAGTTCAAGATCAGCCTGGGCAACATAGCAAGACTCTGTCTCTACAAAAAATGAAAA AATTAGCTGGACGAGGT / Celera SNP ID: hCV29582612 / Public SNP ID: rs4550901 / SNP Chromosome Position: 155501533 / SNP in Genomic Sequence: SEQ ID NO: 349 / SNP Position Genomic: 27401 / Related Interrogated SNP: hCV15860433 / Related Interrogated SNP: hCV11503414 / SNP Source: dbSNP; HapMap; ABI_Val; HGBASE / Population(Allele,Count): Caucasian (A,16|C,104) / SNP Type: INTERGENIC;UNKNOWN / Context (SEQ ID NO: 828): / TTCCCCTTCCCCTTCTTCTTCTCCTCCTCCTCCTCCTTCTTCTTCCAGCAAATATCAAATTTGGAGAGACAATCTGGTAGATT TAGCTTGCGTAAAGGGT

Y

TGTCTACCCCTAGCTTGGTCACCTGTGGTTAGAGGGTCAGGGTGTTGTAGTACTGACAGGACCACTGGAGCTTCATACCTCCA TTTTAGGGGTCATTCCA / Celera SNP ID: hCV31863993 / Public SNP ID: rs7673587 / SNP Chromosome Position: 155480434 / SNP in Genomic Sequence: SEQ ID NO: 349 / SNP Position Genomic: 6302 / Related Interrogated SNP: hCV11503414 / Related Interrogated SNP: hCV11503469 / Related Interrogated SNP: hCV15860433 / SNP Source: dbSNP; HapMap; ABI_Val / Population(Allele,Count): Caucasian (C,85|T,35) / SNP Type: INTERGENIC;UNKNOWN //

Gene Number: 13 / Gene Symbol: FGG - 2266 / Gene Name: fibrinogen gamma chain / Chromosome: 4 / OMIM NUMBER: 134850 / OMIM Information: Dysfibrinogenemia, gamma type (3); Hypofibrinogenemia, gamma/type (3); / Thrombophilia, dysfibrinogenemic (3) // / Genomic Sequence (SEQ ID NO: 350): // / SNP Information / Context (SEQ ID NO: 829): / ATAACATTTAGCATAAAATGAGGACTCAATTACTACTGATGGTTGCCCAATTGTAATTGGTAAATTGGCAAAAAGTGGTGGTT TTTAATGGTCAATAAAG

R

TACCATGTATCTAGTCTTAGGAAACAAAAGGTTTATTGAAATGCATGTAGATAAATTATCATCAGCATAAAACTGTTATGGAG TTTTCAACATGGGGTCT / Celera SNP ID: hCV11503414 / Public SNP ID: rs2066865 / SNP Chromosome Position: 155525276 / SNP in Genomic Sequence: SEQ ID NO: 350 / SNP Position Genomic: 9990 / SNP Source: dbSNP; Celera; HapMap; ABI_Val / Population(Allele,Count): Caucasian (G,174|A,50) / SNP Type: UTR3 / Context (SEQ ID NO: 830): / AAGTCAGTTGAATTAAACATTTAAGTATTAGTATTCTTTCAAACAGCTCTACCTCAGATAGCATTTGTAGCAACCTTATATTA TATGTATACATGCTGTA

W

CCACTGCATTAGATTGTAAACTGATTTCTGTGGCTACGGCATCTAACATATATAGCTGTACTTAACATAGTTGTAGTTAACAC TCAATAACTATGTAATG / Celera SNP ID: hCV11503469 / Public SNP ID: rs2066854 / SNP Chromosome Position: 155535181 / SNP in Genomic Sequence: SEQ ID NO: 350 / SNP Position Genomic: 19895 / SNP Source: dbSNP; HapMap; HGBASE / Population(Allele,Count): Caucasian (A,32|T,92) / SNP Type: INTRON / Context (SEQ ID NO: 831): / GCTAAATGTCCTTAATAGAAGACTTGAAATCAATAAATATAGTAAGAGAAAAAAGGAAGAAACTTTCAGAGAATTTCTGAAAC TTTGTGGGTCAATAGAA

R
TTAGCAGTTAATTTTCTACAAATCATCCTCAGGGTAAAGTGAGTCATATTCTGTTTCCGCAGGGTGCTCTGGTCTGACCTGTT
TGGCTCCCCCCAGGTGG / Celera SNP ID: hCV2892863 / Public SNP ID: rs1049636 /
SNP Chromosome Position: 155525970 / SNP in Genomic Sequence: SEQ ID NO: 350 /
SNP Position Genomic: 10684 / Related Interrogated SNP: hCV11503469 /
Related Interrogated SNP: hCV11503414 / Related Interrogated SNP: hCV2892877 /
SNP Source: Applera / Population(Allele,Count): Caucasian (A,18|G,0) African
American (A,9|G,5) total (A,27|G,5) / SNP Type: UTR3;INTRON;PSEUDOGENE / SNP
Source: dbSNP; Celera; HapMap; HGBASE / Population(Allele,Count): Caucasian
(G,67|A,159) / SNP Type: UTR3;INTRON;PSEUDOGENE /
Context (SEQ ID NO: 832): /
GGCTTGATGAGTAATTGGATATAGGGAGGTAGAAATTGGATATGGGGAGGAAGATTGGAGGATGAGTCACTGTTTTTTTTGCA
TTTGAGGACTGGATGGA
R

TAGAACAGGTTTGGGAGAGTGGAGAACACTAAGCATACCATGTACTATATCCCCAAATCCAACACAATGGCTACCACAAGGAA
GGCATTCAATAAATGTT / Celera SNP ID: hCV286004 / Public SNP ID: rs1118824 /
SNP Chromosome Position: 155524009 / SNP in Genomic Sequence: SEQ ID NO: 350 /
SNP Position Genomic: 8723 / Related Interrogated SNP: hCV11503469 / Related
Interrogated SNP: hCV11503414 / Related Interrogated SNP: hCV2892877 / SNP
Source: dbSNP; Celera; HapMap / Population(Allele,Count): Caucasian
(G,67|A,159) / SNP Type: INTERGENIC;UNKNOWN /
Context (SEQ ID NO: 833): /
ATACACATTATATATACATATACATACATATGAAACCTTTGCTGTTAGATATGTCAGATAATACAGAATGAGTGGTAATCATT
TAACACATTTCATGTTC
Y

GTGGTGCCAGAAATGGGTAGGCTGTCTAGTAAATAGATTTTGTTAATTAGTACATTCTGTGATATAATCTAGTTGTGCTGAAT
TTCCTGAGATGCTTGGA / Celera SNP ID: hCV2892855 / Public SNP ID: rs6536024 /
SNP Chromosome Position: 155543369 / SNP in Genomic Sequence: SEQ ID NO: 350 /
SNP Position Genomic: 28083 / Related Interrogated SNP: hCV11503414 /
Related Interrogated SNP: hCV11503469 / Related Interrogated SNP: hCV2892877 /
SNP Source: dbSNP; Celera; HapMap / Population(Allele,Count): Caucasian
(T,99|C,127) / SNP Type: INTRON /
Context (SEQ ID NO: 834): /
CCTAACCAAACAGTACAACTTTAACAAAGGTCAGTCAAATCCTAGAAGATAGGGAAATGAGGGAGTTTATAGCATTGTGTTGT
GCATATTCTCTAGAATA
Y

GCAGAACTAGGGCTAGAATGTGTCATAGAGAAAGCCAAGTAACCTTCACCTGGCCCTTTCCTTAGGTTTGTCTGTCTTGGTCC
CATGCTTTAGCTCAGCT / Celera SNP ID: hCV2892858 / Public SNP ID: rs12648395 /
SNP Chromosome Position: 155541289 / SNP in Genomic Sequence: SEQ ID NO: 350 /
SNP Position Genomic: 26003 / Related Interrogated SNP: hCV11503469 /
Related Interrogated SNP: hCV11503414 / Related Interrogated SNP: hCV2892877 /
SNP Source: dbSNP; Celera; HapMap / Population(Allele,Count): Caucasian
(C,67|T,159) / SNP Type: INTRON /
Context (SEQ ID NO: 835): /
AGACTAAGGAATGATCTGAGGTGCCCTGGATTGAGACAAATTGAACAGAGAAGTTGGATTTAAGTGTTTTGCTGTGCCACATT
GGTATCTGCTACTGGTT
K
GTTTGTGTTTTTCCATCTTTGTGTAATCTACGATTTTAAATCTCTCACCCCATGAAAGAATTTTCATGACTAAATATGATTAG
CAGGAGATTTACAAGAG / Celera SNP ID: hCV2892859 / Public SNP ID: rs13130318 /
SNP Chromosome Position: 155538470 / SNP in Genomic Sequence: SEQ ID NO: 350 /
SNP Position Genomic: 23184 / Related Interrogated SNP: hCV11503414 /
Related Interrogated SNP: hCV11503469 / Related Interrogated SNP: hCV15860433
/ Related Interrogated SNP: hCV2892877 / Related Interrogated SNP:
hCV11503470 / SNP Source: dbSNP; Celera; HapMap / Population(Allele,Count):
Caucasian (T,74|G,30) / SNP Type: INTRON /
Context (SEQ ID NO: 836): /
ACTGACTTCCCCCATGTCCCAGTTCACTATTCCCAGGAATGAATGACCCTGGAAATACATTCCTGTCCCTCTGTGTCAACCAT
GTTCATAGGCTCTCACT
Y
CTCAGGGAGCTTTTGAGCTCAATCTTCCCTTTGTCTGAAGGTGGGCATGGCACCCCATCTGCCACAGAGAAAAGAGCCAGGAG
CCCACTTCATTGTTCCT / Celera SNP ID: hCV7429780 / Public SNP ID: rs1800792 /
SNP Chromosome Position: 155534408 / SNP in Genomic Sequence: SEQ ID NO: 350 /
SNP Position Genomic: 19122 / Related Interrogated SNP: hCV11503414 /

EP 2 635 709 B1

Related Interrogated SNP: hCV11503469 / Related Interrogated SNP: hCV15860433 / Related Interrogated SNP: hCV2892877 / SNP Source: dbSNP; HapMap; HGBASE / Population(Allele,Count): Caucasian (T,117|C,109) / SNP Type: INTRON / Context (SEQ ID NO: 837): /
GGAGGAGGATGAGATGGTAGCAGGAGAGGCTGGATGTAAGAAAGGGAACAGGAAGTGGGTGATTGCCCTTATCCTGGCAAGAG
ATGTATCTCAACTACAA
W
GGTGACAATCGGTATACAGGAAAAAAGTGAATATGATAAATATATAGGAGGTGAATTCAGCAGGCTTGATGAGTAATTGGATA
TAGGGAGGTAGAAATTG / Celera SNP ID: hCV7429782 / Public SNP ID: rs1118823 / SNP Chromosome Position: 155523846 / SNP in Genomic Sequence: SEQ ID NO: 350 / SNP Position Genomic: 8560 / Related Interrogated SNP: hCV11503469 / Related Interrogated SNP: hCV11503414 / Related Interrogated SNP: hCV2892877 / SNP Source: dbSNP; Celera; HapMap; HGBASE / Population(Allele,Count): Caucasian (A,66|T,160) / SNP Type: TFBS SYNONYMOUS;INTERGENIC;UNKNOWN / Context (SEQ ID NO: 838): /
GTAATAAACAAGGAAAATACCTGGGAATTTGAAACTCTAAAATGTTCTCCTATTTTATTAAGTACATACTAAAATATTTGATA
TAATGAAAATAATTTAC
R
AAGACAAAATAAATGACAAGTGGTCATAAAAAATGCAAATAAAGTCAATCATTTTATTATTATATATTTAGGAACAAAGTTGAA
ATGTTATCTCCTCAAAT / Celera SNP ID: hCV11503416 / Public SNP ID: rs2066864 / SNP Chromosome Position: 155525695 / SNP in Genomic Sequence: SEQ ID NO: 350 / SNP Position Genomic: 10409 / Related Interrogated SNP: hCV11503414 / Related Interrogated SNP: hCV11503469 / Related Interrogated SNP: hCV15860433 / Related Interrogated SNP: hCV2892877 / Related Interrogated SNP: hCV11503470 / SNP Source: dbSNP; HapMap; HGBASE / Population(Allele,Count): Caucasian (G,89|A,31) / SNP Type: TRANSCRIPTION FACTOR BINDING SITE;UTR3;INTRON / Context (SEQ ID NO: 839): /
CTCATCCAACCCCCATCCTAAAATAAGTGTCTACTGTCCTCAGACTGGTCTCTTCTCTCAAGCTTTCACAAACCCCTGCCCTT
ACCAGTGCTTGCCTTCT
Y
TTTATTCTGTTCCTCCAAACACCTGTTCACTGCGTGTATCTGACACAAAGCTCATGGGAAGCCAGGTTAATGTGTCAACAAGC
ATCCATGTAGTAATGTT / Celera SNP ID: hCV11503431 / Public SNP ID: rs2066861 / SNP Chromosome Position: 155527436 / SNP in Genomic Sequence: SEQ ID NO: 350 / SNP Position Genomic: 12150 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera; HapMap; HGBASE / Population(Allele,Count): Caucasian (C,176|T,50) / SNP Type: INTRON / Context (SEQ ID NO: 840): /
CCTGTGCTCTTAACCATGAATCTTAATACAACCATGAAGCCCTTCTTAATTTAAAATAATACACAGATACAAGGTGTTATGAG
ACTCAAAGTTATTCACT
R
ATAGAATGGATTTTAAAGATGTGTGTGTGCATCTACATAACTGTGTGCATGATTATTTGAACACACATTTACATCTATCTACC
AACTTATCTATCTTCTT / Celera SNP ID: hCV11853483 / Public SNP ID: rs12644950 / SNP Chromosome Position: 155537321 / SNP in Genomic Sequence: SEQ ID NO: 350 / SNP Position Genomic: 22035 / Related Interrogated SNP: hCV11503414 / Related Interrogated SNP: hCV11503469 / Related Interrogated SNP: hCV15860433 / Related Interrogated SNP: hCV2892877 / Related Interrogated SNP: hCV11503470 / SNP Source: dbSNP; Celera; HapMap / Population(Allele,Count): Caucasian (G,88|A,28) / SNP Type: INTRON / Context (SEQ ID NO: 841): /
CATTAGACAATCTCAAATCTTGTATACTTCCTCTTTGTTAGAGTATTTAATACTGAAGATATGCCATAATCATTTCTTGTTGC
AAAAAGAATTTAAAAAC
Y
AACGGTATCAGTTTTTTTTCTATTGAAGTTTAAAATTAGATTTTGGTAATTCAGAAACAAAAAGACAAATACAGCATGTTCTCA
GTTATAAGTGGGAGCTA / Celera SNP ID: hCV11853489 / Public SNP ID: rs7681423 / SNP Chromosome Position: 155542248 / SNP in Genomic Sequence: SEQ ID NO: 350 / SNP Position Genomic: 26962 / Related Interrogated SNP: hCV11503414 / Related Interrogated SNP: hCV11503469 / Related Interrogated SNP: hCV15860433 / Related Interrogated SNP: hCV2892877 / Related Interrogated SNP: hCV11503470 / SNP Source: dbSNP; Celera; HapMap; ABI_Val / Population(Allele,Count): Caucasian (C,89|T,31) / SNP Type: INTRON /

Context (SEQ ID NO: 842): /
ATTGTAAGCTTTGGAGGAGTAGGTTTTGTTTAGCTGGAAAACCATGCAAATTAATAGGCATGATACGAGTATCATTTTTTAAA
AAGCATGTGACAATAAG
S
GTATCTTTAAATGTGCAAAACTGGAAAAAAGATGGAAGAAATTTTCAGTTGCTTAAATTATAATCTGCCGTGCATTGGCTTTT
TCTTTTTGAGATGGAGT / Celera SNP ID: hCV27020269 / Public SNP ID: rs7659613 /
SNP Chromosome Position: 155515416 / SNP in Genomic Sequence: SEQ ID NO: 350 /
SNP Position Genomic: 130 / Related Interrogated SNP: hCV11503414 / Related
Interrogated SNP: hCV11503469 / Related Interrogated SNP: hCV15860433 /
Related Interrogated SNP: hCV2892877 / Related Interrogated SNP: hCV11503470 /
SNP Source: dbSNP; Celera; HapMap; ABI_val / Population(Allele,Count):
Caucasian (C,48|G,72) / SNP Type: INTERGENIC;UNKNOWN /
Context (SEQ ID NO: 843): /
AATGTTGAATGTATATAAATATTATCTACTAAAATATATATAATGAAAATATGTAATTATTGATCATTCACAAAGATTTACAT
AGTACACTGATAATTAC
R
TCTTGCTAACTTTTTAATATGATTTGAACATGAAAGGTGACTCAGGCATTGCCTTTGCCTTCAAGAAGTGTGTAGCTCAATTG
GAGAATGTATAAATAAA / Celera SNP ID: hCV31863979 / Public SNP ID: rs12186294 /
SNP Chromosome Position: 155535483 / SNP in Genomic Sequence: SEQ ID NO: 350 /
SNP Position Genomic: 20197 / Related Interrogated SNP: hCV11503414 /
Related Interrogated SNP: hCV11503469 / Related Interrogated SNP: hCV15860433
/ Related Interrogated SNP: hCV2892877 / Related Interrogated SNP:
hCV11503470 / SNP Source: dbSNP; HapMap / Population(Allele,Count): Caucasian
(A,116|G,106) / SNP Type: INTRON /
Context (SEQ ID NO: 844): /
TTTCCTTTTCATTCTGCTTTCAAACCACAAATTCATGTGTGGATAGATCTTAAAAACACAATGCTGAAATAAAAAAGTAGTAA
GTATGATGAAGACTTCA
R
CAGTGATTGTCAAAGGATTTGTGGTGAAGGACTAGTTTTATTAAAAATTCTAATCAGCCACAGACCAGGTTTTTATAGAATTCA
ATAAAAATAATTACTAA / Celera SNP ID: hCV31863982 / Public SNP ID: rs7659024 /
SNP Chromosome Position: 155520930 / SNP in Genomic Sequence: SEQ ID NO: 350 /
SNP Position Genomic: 5644 / Related Interrogated SNP: hCV11503414 / Related
Interrogated SNP: hCV11503469 / Related Interrogated SNP: hCV11503470 /
Related Interrogated SNP: hCV15860433 / Related Interrogated SNP: hCV2892877 /
SNP Source: dbSNP; HapMap; ABI_val / Population(Allele,Count): Caucasian
(G,176|A,50) / SNP Type: INTERGENIC;UNKNOWN //

Gene Number: 14 / Gene Symbol: EIF6 - 3692 / Gene Name: eukaryotic
translation initiation factor 6 / Chromosome: 20 / OMIM NUMBER: 602912 /
OMIM Information: // Genomic Sequence (SEQ ID NO: 351): // / SNP Information /
Context (SEQ ID NO: 845): /
TGGCCTCCCTTCCCCCACTTCCTGGTCCCTGTCCACTCCTCAGGTTGGTGCTCTCACTTCTTGAAAGCTCTAGGCACCCCCGC
CTCCCGCCAGGCTCCCC
R
TTGGCTCCTGGCAGGCCAGCTGAGAATGAACAGGAGATGGAGGCAGGCAGCCCAGGCTGCAGAGGTGAGGGATGTGGGGCCAG
GCCCAGAGGGCTCAGCC / Celera SNP ID: hCV1271624 / Public SNP ID: rs7280 / SNP
Chromosome Position: 33864484 / SNP in Genomic Sequence: SEQ ID NO: 351 / SNP
Position Genomic: 7759 / SNP Source: dbSNP; Celera; HGBASE /
Population(Allele,Count): Caucasian (A,139|G,85) / SNP Type: UTR3;INTRON /
Context (SEQ ID NO: 846): /
ACTCTGAGGCCGACTCAATCTCTCGGCTGGAAGCAGTGTTTTCCCAGAGCTTGGCCCTTGCTGACCTCGCTCACTGGGCCCAT
CTTCCCACACTGCTCTT
R
GAAGGACACCCCTACCGGTAGCAGCCCCAAGCTGAGGGGGCTCCCTTTTTGACCTTCACTGGCCCGCCCTTCACTGTCTCCAG
CAGGAGTTCCTAGGGCT / Celera SNP ID: hCV1271625 / Public SNP ID: rs6060341 /
SNP Chromosome Position: 33863633 / SNP in Genomic Sequence: SEQ ID NO: 351 /
SNP Position Genomic: 6908 / Related Interrogated SNP: hCV1825046 / SNP
Source: dbSNP; Celera; HapMap; ABI_val / Population(Allele,Count): Caucasian
(A,176|G,50) / SNP Type: UTR3;INTRON /
Context (SEQ ID NO: 847): /
GCCCACCCCCACCATGTGTCTGATCCATCCCAGTGTTTAGTTCTGGCCTAGACATTTGCCTATGACCTGAGTCCATAGATCCT
AACTCCTGATGTGGCCA
S

GATAGACTCTGTGATGTTCGTAATGGATTTGGATGGGCTGTGAGATGCTCCTTGCTTGAGTATCCCAGAAAACGAGCTGCGTA
AACCAGGTTGCCAGCTT / Celera SNP ID: hCV1271649 / Public SNP ID: rs6120880 /
SNP Chromosome Position: 33829406 / SNP in Genomic Sequence: SEQ ID NO: 351 /
SNP Position Genomic: 27319 / Related Interrogated SNP: hCV1825046 / SNP
Source: dbSNP; Celera; HapMap / Population(Allele,Count): Caucasian
(C,52|G,50) / SNP Type: INTRON /
Context (SEQ ID NO: 848): /
ACTGTATTTTTAATTCATTTTCTCTTATTTTTACTGTGGGGTTTCATATTACTTTAATAGCTGTTTATATTTGTAAGGTGCTT
TGCACTTCCCATATGCC
R

TGTATGTGGCCCTATTTGGTCCTGGGAACAATACCATGAGGTAGATAAGGAAGACATTGTCCTCATTTTATAGGTGAGTAAAA
GGAGACTCAAAGATGAT / Celera SNP ID: hCV1271653 / Public SNP ID: rs2425019 /
SNP Chromosome Position: 33819415 / SNP in Genomic Sequence: SEQ ID NO: 351 /
SNP Position Genomic: 37310 / Related Interrogated SNP: hCV1825046 / SNP
Source: dbSNP; Celera; HapMap; HGBASE / Population(Allele,Count): Caucasian
(A,128|G,98) / SNP Type: INTRON /
Context (SEQ ID NO: 849): /
AATGCTGGAACAACTGGAGAGTGCAGGGATTGTACACAGGCTGAGGGCCCCAAACATCACATTCAGAATGGCCCGGAGGGAAC
TGCACTTTAATGGGGTG
S

CACAGGACAGCAGAACCCTCAGCCAGCAGCCACAGGGCCTGCCAGCCAGCACAACAGAGCAGGTTTTTGCAGTAATGATAGAT
CCAGGCGATAAGCACAG / Celera SNP ID: hCV11656916 / Public SNP ID: rs7004 / SNP
Chromosome Position: 33866753 / SNP in Genomic Sequence: SEQ ID NO: 351 / SNP
Position Genomic: 10028 / Related Interrogated SNP: hCV1825046 / SNP Source:
dbSNP; HGBASE / Population(Allele,Count): Caucasian (G,78|C,28) / SNP Type:
MISSENSE MUTATION;ESS;TRANSCRIPTION FACTOR BINDING SITE;MICRORNA;UTR3; / ESE
SYNONYMOUS;INTRON //
Context (SEQ ID NO: 850): /
ATGGAGTGTGGGCTGACACAGACCGACCACACATTGTAATCACATCCACTTGCATAAACTCTTCACCCAAACGCCGATGTAGA
GACCGGCCATGACCCCC
M

CAACACATACCACAGTGGATAGCACGTGCCCTGGCTCAAGTCTGCAAGGCCCTGAGAGCTGAACGACTGTCACATGGAATATC
CAAGGACATCAACGCTG / Celera SNP ID: hCV16013698 / Public SNP ID: rs2425052 /
SNP Chromosome Position: 33880729 / SNP in Genomic Sequence: SEQ ID NO: 351 /
SNP Position Genomic: 24004 / Related Interrogated SNP: hCV1825046 / SNP
Source: dbSNP; HGBASE / Population(Allele,Count): Caucasian (A,134|C,92) /
SNP Type: INTERGENIC;UNKNOWN /
Context (SEQ ID NO: 851): /
TGTAAGTTGGTCAATCACTTTAGATAATTATCTGGCATCATCTGCAACTACTACTAAATAGTCACATAACCTGTGACTCTGCC
ATGCCACTCTTAGGTAT
Y

ATATACCCAGTAGAAATATGTACATGTGTTCACTAAAAAAAACATAGCAGCACTATTACTTTTTTTTTTTTTTTTTTTGAGACGGAG
TCTTGTTCTGTTGCCCA / Celera SNP ID: hCV16013724 / Public SNP ID: rs2425044 /
SNP Chromosome Position: 33869735 / SNP in Genomic Sequence: SEQ ID NO: 351 /
SNP Position Genomic: 13010 / Related Interrogated SNP: hCV1825046 / SNP
Source: dbSNP; HGBASE / Population(Allele,Count): Caucasian (C,178|T,48) /
SNP Type: INTRON /
Context (SEQ ID NO: 852): /
GCTAATGGACAGTCTCGGTGGAGGGCTGGGCTGTTGGGACCTAAGCCCCACAGCTGCAATCACTGGGTTCGGAGGCAGGGCCT
GACACACCTCTCTCCCC
Y

TCTCCCGCTTCCTCCCAGGATCGCTGGTTCTGGCGTCTGCGCAATAACCGAGTGCAGGAGGGCTACCCCATGCAGATCGAGCA
GTTCTGGAAGGGCCTGC / Celera SNP ID: hCV25472481 / Public SNP ID: rs2275274 /
SNP Chromosome Position: 33857544 / SNP in Genomic Sequence: SEQ ID NO: 351 /
SNP Position Genomic: 819 / Related Interrogated SNP: hCV25620145 / SNP
Source: Applera / Population(Allele,Count): Caucasian (C,33|T,3) African
American (C,31|T,5) total (C,64|T,8) / SNP Type: INTRON / SNP Source:
dbSNP; HapMap; ABI_Val; HGBASE / Population(Allele,Count): Caucasian
(C,203|T,21) / SNP Type: INTRON /
Context (SEQ ID NO: 853): /
GCGGACACACTGCAAGGTCAGGAGGCAAACAGGGCAGGTAGATTTCTGACCTCTGCCCCAACAAAGATTAAAGTCCTGGAGCA
TGTACCGTGTGTCCTTC
R

GAGGATGGTGAGCAGCTTTGCTCTGGGGAAGGGGGTGAGCAGGTGACAGCTGCAGCTGGGGAGCCAGGCTAGGCAGAAGCGGT
CACCACCTTTCCACGGA / Celera SNP ID: hCV29674974 / Public SNP ID: rs6058224 /
SNP Chromosome Position: 33881774 / SNP in Genomic Sequence: SEQ ID NO: 351 /
SNP Position Genomic: 25049 / Related Interrogated SNP: hCV1825046 / SNP
Source: dbSNP; HapMap / Population(Allele,Count): Caucasian (G,178|A,48) /
SNP Type: INTERGENIC;UNKNOWN //

Gene Number: 15 / Gene Symbol: KLKB1 - 3818 / Gene Name: kallikrein B,
plasma (Fletcher factor) 1 / Chromosome: 4 / OMIM NUMBER: 229000 / OMIM
Information: Fletcher factor deficiency (1); Prekallikrein deficiency (3) //
Genomic Sequence (SEQ ID NO: 352): // / SNP Information /
Context (SEQ ID NO: 854): /
GGAGAAAATGTGTCCTATGTATTTCCTTCCCAGTTCTTTGAAAGAGAGTGATAGGAAAAAGGAACACTATTGAAGGAAGGACT
GCCCAGTTTCAAACAGG
W
ATTTATTTTTCTCTCCTAGGCTTCCCCTGCAGGATGTTTGGCGCATCTATAGTGGCATTTTAAATCTGTCAGACATTACAAAA
GATACACCTTTCTCACA / Celera SNP ID: hCV15968043 / Public SNP ID: rs2292423 /
SNP Chromosome Position: 187175722 / SNP in Genomic Sequence: SEQ ID NO: 352 /
SNP Position Genomic: 37050 / SNP Source: Applera /
Population(Allele,Count): Caucasian (A,13|T,27) African American (A,6|T,30)
total (A,19|T,57) / SNP Type: TRANSCRIPTION FACTOR BINDING SITE;INTRON / SNP
Source: dbSNP; HapMap; ABI_Val; HGBASE / Population(Allele,Count): Caucasian
(T,68|A,48) / SNP Type: TRANSCRIPTION FACTOR BINDING SITE;INTRON /
Context (SEQ ID NO: 855): /
GCTTGCCATCGAGACATTTATAAAGGAGTTGATATGAGAGGAGTCAATTTTAATGTGTCTAAGGTTAGCAGTGTTGAAGAATG
CCAAAAAAGGTGCACCA
R
TAACATTCGCTGCCAGTTTTTTTTCATATGCCACGCAAACATTTCACAAGGCAGAGTACCGGTGAGTACAATTCAAGGTGTGTG
TTCTTTGTATTGGTGCC / Celera SNP ID: hCV22272267 / Public SNP ID: rs3733402 /
SNP Chromosome Position: 187158034 / SNP in Genomic Sequence: SEQ ID NO: 352 /
SNP Position Genomic: 19362 / SNP Source: Applera /
Population(Allele,Count): Caucasian (A,11|G,17) African American (A,19|G,3)
total (A,30|G,20) / SNP Type: MISSENSE MUTATION;INTERGENIC;UNKNOWN / SNP
Source: Applera / Population(Allele,Count): Caucasian (A,14|G,18) African
American (A,21|G,7) total (A,35|G,25) / SNP Type: MISSENSE
MUTATION;INTERGENIC;UNKNOWN / SNP Source: Applera / Population(Allele,Count):
Caucasian (A,15|G,21) African American (A,27|G,7) total (A,42|G,28) / SNP Type:
MISSENSE MUTATION;INTERGENIC;UNKNOWN / SNP Source: dbSNP; Applera /
Population(Allele,Count): Caucasian (A,62|G,58) / SNP Type: MISSENSE
MUTATION;INTERGENIC;UNKNOWN /
Context (SEQ ID NO: 856): /
CTGCTTCCCTGTGGGTTCCGGCTTCTGCAGAGCTGTAAGAGTTGAATGCCACACACAGTCACACTAAGGAATGCTCCAGGATT
GGGAAAGAAAATTCAAC
M
TTATAATGAGAACACTGTGAATGCTATTGAATTAACTACTCCCCTCTCTCCCTATTTCTTGTAAGTCTTAGTGTCAGTAAACT
AATTATAAATTTACATT / Celera SNP ID: hCV25474413 / Public SNP ID: rs3822057 /
SNP Chromosome Position: 187188152 / SNP in Genomic Sequence: SEQ ID NO: 352 /
SNP Position Genomic: 49480 / SNP Source: Applera /
Population(Allele,Count): Caucasian (A,16|C,14) African American (A,7|C,11)
total (A,23|C,25) / SNP Type: INTRON / SNP Source: Applera /
Population(Allele,Count): Caucasian (A,16|C,18) African American (A,12|C,20)
total (A,28|C,38) // / SNP Type: INTRON / SNP Source: dbSNP; HGBASE /
Population(Allele,Count): Caucasian (A,109|C,117) / SNP Type: INTRON /
Context (SEQ ID NO: 857): /
AGTTATGACAAGAATAATCATTATAGTACTTTTCAGATTTTATAACCTGGAGCAGATTATTTTAAGTTGATTAGTAGGTTCTG
TTACAGTTTTTCTTTTG
W
TCGTGCACTTATAGTCTTCATTTAATTCCTCATAGAATCCCAGTCACCTTTATATATCATATTATTGGAAGAGATTCATCTTC
ATAATCTCCAGTTTTTT / Celera SNP ID: hCV3230113 / Public SNP ID: rs1053094 /
SNP Chromosome Position: 187133031 / SNP in Genomic Sequence: SEQ ID NO: 352 /
SNP Position Genomic: 5641 / SNP Source: dbSNP; Celera; HapMap; HGBASE; /
Population(Allele,Count): Caucasian (A,60|T,60) / SNP Type:
MICRORNA;UTR3;INTRON /

Context          (SEQ ID NO: 858): /
TATTTAACATTCCTCTCAAGCAAATACGCCTTGAAATGCTTTTTTTAAATCATAGGAATTTAAAAACACTTTACAATAGAGAA
TGATTGATTTTTAAAAT
R
TGTCTGATTTAGCTTTGTAGAGATGTTCCGCTAATATCCATAACTAATCTGAGAGGAAATGTGGAACAACAGAAGAGTAACAG
TGTCTACTCAGTAACAA / Celera SNP ID:   hCV8241630 / Public SNP ID:    rs925451 /
SNP Chromosome Position:   187187569 / SNP in Genomic Sequence:   SEQ ID NO: 352 /
 SNP Position Genomic:   48897 / SNP Source:   dbSNP; Celera; HapMap; HGBASE /
Population(Allele,Count):   Caucasian (G,138|A,88) / SNP Type:
INTRON;PSEUDOGENE /
Context          (SEQ ID NO: 859): /
TAAAGCGGGGATGGTATTCACAACATTTAACTTATAGGGTCCAAGCACTGACCAACCTGACCATTAGAACAGAGTGTGGTCTC
TGTACAGGGCAGATGGC
R
CTGAGTGGGTATTCTCCACAGAAAGAGAAACGAAGACAGTACCCCACTCCTCCAACCCACCACCCACCACCAATCCCACCACC
AATTCCACCACCAATCC / Celera SNP ID:   hCV11786258 / Public SNP ID:    rs4253303 /
SNP Chromosome Position:   187173546 / SNP in Genomic Sequence:   SEQ ID NO: 352 /
 SNP Position Genomic:   34874 / SNP Source:   dbSNP; Celera; HapMap; HGBASE /
Population(Allele,Count):   Caucasian (G,72|A,42) / SNP Type:   INTRON /
Context          (SEQ ID NO: 860): /
GCAATTTTTACAACCTGAGTTCAAGTCAAATTCTGAGCCTGGGGGGGTCCTCATCTGCAAAGCATGGAGAGTGGCATCTTCTTT
GCATCCTAAGGACGAAA
R
ACACAGTGCACTCAGAGCTGCTGAGGACAATGTCTGGCTGAAGCCCGCTTTCAGCACGCCGTAACCAGGGGCTGACAATGCGA
GGTCGCAACTGAGATCT / Celera SNP ID:   hCV15793897 / Public SNP ID:    rs3087505 /
SNP Chromosome Position:   187179486 / SNP in Genomic Sequence:   SEQ ID NO: 352 /
 SNP Position Genomic:   40814 / SNP Source:   dbSNP; HapMap; HGBASE /
Population(Allele,Count):   Caucasian (A,20|G,206) / SNP Type:   MICRORNA;UTR3 /
Context          (SEQ ID NO: 861): /
TTAGAGCCTTTCTGTTTCTCTCAATAGGGTTGGAGAGTTATCCTTATCTTCTTTTTATTGGGGCTTAAGAAGAGAGATGAGGT
TCCATGGAGTAAACAAT
W
CAAGGATATAAGGACCTCATATAATCTCACGTATCCATTTTCCATGAAAGCCATTCTTGGCACGAATTTGCCATTCTATGTTT
GAGCCTCATAAAAGGCA / Celera SNP ID:   hCV27477533 / Public SNP ID:    rs3756008 /
SNP Chromosome Position:   187185385 / SNP in Genomic Sequence:   SEQ ID NO: 352 /
 SNP Position Genomic:   46713 / SNP Source:   dbSNP; HapMap; ABI_Val; HGBASE /
Population(Allele,Count):   Caucasian (A,136|T,90) / SNP Type:
INTERGENIC;UNKNOWN /
Context          (SEQ ID NO: 862): /
ATGGGTGCCAACTTGGAGCCTGGGGTTGCTGGGGCTAACGTGGAGGCTAGATAGAGTCTTGGGGGGCCAGGCTAGAGCTGGAGC
AGGCCTGAAGTCTAGGT
Y
TTGTGTGGCCATCTTGGAGCCTGAAGCCCCAGGGGCTGACCTGGTCTGGGGTGAGCATGGGGCTGAGGCCACAGAGGCTGGTC
TGGCCTGTGGCAGGCCT / Celera SNP ID:   hCV27902808 / Public SNP ID:    rs4253236 /
SNP Chromosome Position:   187148071 / SNP in Genomic Sequence:   SEQ ID NO: 352 /
 SNP Position Genomic:   9399 / SNP Source:   dbSNP; HapMap /
Population(Allele,Count):   Caucasian (T,87|C,139) / SNP Type:   INTRON /
Context          (SEQ ID NO: 863): /
TTGAAGGATCTATGATCAGCTGCTTCACCGCCATGTGACTTTATGAATAGAGACGTGTTAAAGCGGGGATGGTATTCACAACA
TTTAACTTATAGGGTCC
R
AGCACTGACCAACCTGACCATTAGAACAGAGTGTGGTCTCTGTACAGGGCAGATGGCGCTGAGTGGGTATTCTCCACAGAAAG
AGAAACGAAGACAGTAC / Celera SNP ID:   hCV32291301 / Public SNP ID:    rs4253302 /
SNP Chromosome Position:   187173488 / SNP in Genomic Sequence:   SEQ ID NO: 352 /
 SNP Position Genomic:   34816 / SNP Source:   dbSNP; Celera; HapMap; HGBASE /
Population(Allele,Count):   Caucasian (A,97|G,21) / SNP Type:   INTRON /
Context          (SEQ ID NO: 864): /
GGGGGAAAAGATGCTTGTAAGGTAACTCATGAGATTATGAAAAACACAATAGGCTGCTTGAGAAAATTCATTTCAAAATATAT
TTTCCAATAGCATAATT
Y
AATCATAGTTTTTTAAAAAAAATTCAGAGACAAATGATCTGATAAATTGATAAGCAACTTTTAACAAATTGAATATACATAATAT
ATATTTATATTATTTAT / Celera SNP ID:   hCV22271609 / Public SNP ID:    rs4253326 /
SNP Chromosome Position:   187178599 / SNP in Genomic Sequence:   SEQ ID NO: 352 /

SNP Position Genomic: 39927 / Related Interrogated SNP: hCV15793897 / Related Interrogated SNP: hCV22272267 / Related Interrogated SNP: hCV15968043 / Related Interrogated SNP: hCV3230113 / Related Interrogated SNP: hCV27902808 / Related Interrogated SNP: hCV11786258 / SNP Source: Applera / Population(Allele,Count): Caucasian (C,4|T,34) African American (C,4|T,22) total (C,8|T,56) / SNP Type: TRANSCRIPTION FACTOR BINDING SITE;INTRON / SNP Source: dbSNP; HapMap; HGBASE / Population(Allele,Count): Caucasian (C,35|T,187) / SNP Type: TRANSCRIPTION FACTOR BINDING SITE;INTRON / Context (SEQ ID NO: 865): /
AGGATCTGAACACAGAGAGCGGCGGGGCCGGCGGGGAAGGAGGGAGGAGGGGAGAGCGCTGCTTCCCTGTGGGTTCCGGCTTC
TGCAGAGCTGTAAGAGT
K
GAATGCCACACACAGTCACACTAAGGAATGCTCCAGGATTGGGAAAGAAAATTCAACATTATAATGAGAACACTGTGAATGCT
ATTGAATTAACTACTCC / Celera SNP ID: hCV25474414 / Public SNP ID: rs4253399 / SNP Chromosome Position: 187188094 / SNP in Genomic Sequence: SEQ ID NO: 352 / SNP Position Genomic: 49422 / Related Interrogated SNP: hCV11786258 / Related Interrogated SNP: hCV12066124 / Related Interrogated SNP: hCV15968043 / Related Interrogated SNP: hCV27474895 / Related Interrogated SNP: hCV3230038 / Related Interrogated SNP: hCV27477533 / Related Interrogated SNP: hCV8241630 / Related Interrogated SNP: hCV25474413 / Related Interrogated SNP: hCV3230113 / Related Interrogated SNP: hCV22272267 / Related Interrogated SNP: hCV3230096 / SNP Source: Applera / Population(Allele,Count): Caucasian (G,12|T,18) African American (G,1|T,15) total (G,13|T,33) / SNP Type: TRANSCRIPTION FACTOR BINDING SITE;INTRON / SNP Source: Applera / Population(Allele,Count): Caucasian (G,16|T,14) African American (G,1|T,25) total (G,17|T,39) / SNP Type: TRANSCRIPTION FACTOR BINDING SITE;INTRON / SNP Source: Applera / Population(Allele,Count): Caucasian (G,7|T,19) African American (G,0|T,14) total (G,7|T,33) / SNP Type: TRANSCRIPTION FACTOR BINDING SITE;INTRON / SNP Source: dbSNP; HapMap; HGBASE / Population(Allele,Count): Caucasian (T,137|G,89) / SNP Type: TRANSCRIPTION FACTOR BINDING SITE;INTRON / Context (SEQ ID NO: 866): /
AACCCATCTCTACAAAAAAATATGAAAGTATCTGTGTGTGTGTTGCTGTGTAGTGGACTACAGAACTTTAGAGGCAGTCAC
TTATTTGAATCCCATTG
Y
CGTAACTTTCTACTATTTTATTTTTCCACTGTGACTCAGGGAGATTCAGGTGGTCCCTTAGTTTGCAAACACAATGGAATGTG
GCGTTTGGTGGGCATCA / Celera SNP ID: hCV25634754 / Public SNP ID: rs4253331 / SNP Chromosome Position: 187179135 / SNP in Genomic Sequence: SEQ ID NO: 352 / SNP Position Genomic: 40463 / Related Interrogated SNP: hCV27477533 / Related Interrogated SNP: hCV8241630 / Related Interrogated SNP: hCV3230038 / Related Interrogated SNP: hCV27474895 / SNP Source: Applera / Population(Allele,Count): Caucasian (C,5|T,35) African American (C,0|T,24) total (C,5|T,59) / SNP Type: INTRON / SNP Source: dbSNP; HapMap; HGBASE / Population(Allele,Count): Caucasian (T,210|C,16) / SNP Type: INTRON / Context (SEQ ID NO: 867): /
TTTAAAGGACATTTTTTCACTTAGGATGTTTAGTACTATGAATAATAAATAGTCACAATTTCCTTAACTATGGTGACAAAATA
CAAGCAAATTTAGCCTC
R
TGTCATTTCCTAAGGAACATCTTCTCTCTGTGAGTTCACAGGTTGCCACATGAACATCTTCCAGCATCTTGCGTTCTCAGATG
TGGATGTTGCCAGGGTT / Celera SNP ID: hCV25634781 / Public SNP ID: rs4253299 / SNP Chromosome Position: 187171355 / SNP in Genomic Sequence: SEQ ID NO: 352 / SNP Position Genomic: 32683 / Related Interrogated SNP: hCV22272267 / Related Interrogated SNP: hCV15793897 / Related Interrogated SNP: hCV27902808 / Related Interrogated SNP: hCV15968043 / Related Interrogated SNP: hCV11786258 / Related Interrogated SNP: hCV3230113 / SNP Source: Applera / Population(Allele,Count): Caucasian (A,0|G,10) African American (A,1|G,3) total (A,1|G,13) / SNP Type: INTRON / SNP Source: Applera / Population(Allele,Count): Caucasian (A,1|G,21) African American (A,1|G,21) total (A,2|G,42) / SNP Type: INTRON / SNP Source: dbSNP; HapMap; HGBASE / Population(Allele,Count): Caucasian (A,39|G,187) / SNP Type: INTRON / Context (SEQ ID NO: 868): /
ACGGGTGAATCTGAAGCTTTTTTGGTGAATCCATAGTAAATGAAAGACCCACTAAGACATAATCTAGGGTAGTGAGTCTCAAG
CTTGAAGGAGCTCAGGA
Y

TTATCTGGATCAGCTGGAGTACAAATTCTCTGATCCTCCCCAGCTATAGATGTGCAAGGTGGGATCAGGGAGGGAAACTCAAG
ACTCTTCATCTTTAACA / Celera SNP ID: hCV25988221 / Public SNP ID: rs9995366 /
SNP Chromosome Position: 187131384 / SNP in Genomic Sequence: SEQ ID NO: 352 /
SNP Position Genomic: 7288 / Related Interrogated SNP: hCV15793897 / Related
Interrogated SNP: hCV12066124 / Related Interrogated SNP: hCV27474895 /
Related Interrogated SNP: hCV25474413 / Related Interrogated SNP: hCV3230113 /
Related Interrogated SNP: hCV8241630 / Related Interrogated SNP: hCV27477533
/ Related Interrogated SNP: hCV25990131 / Related Interrogated SNP:
hCV3230038 / SNP Source: Applera / Population(Allele,Count): Caucasian
(C,36|T,0) African American (C,15|T,3) total (C,51|T,3) / SNP Type: INTRON /
SNP Source: dbSNP; HapMap / Population(Allele,Count): Caucasian (T,21|C,205) /
SNP Type: INTRON /
Context (SEQ ID NO: 869): /
GGAGAGGTGGCTTGATTTACATTTAATCTCTGCAATTTATTAGAGTCCTGTAGTTGGATTTACTTTGAAGAGAGTTTCCCAGA
AGAATAAAATTTGCTGC
R
TTGCTTTTTGGGTGTGAGCTGCTTTTGTATTTGCCTAATGCCTTTAATGCAAATTTCTTTCTTTCTCTCTTGCTTTTTTTTTAA
AAAAAATAGAAATGTTT / Celera SNP ID: hCV3230003 / Public SNP ID: rs2304595 /
SNP Chromosome Position: 187172280 / SNP in Genomic Sequence: SEQ ID NO: 352 /
SNP Position Genomic: 33608 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV3230038 / Related Interrogated SNP:
hCV3230113 / Related Interrogated SNP: hCV8241630 / Related Interrogated SNP:
hCV3230096 / Related Interrogated SNP: hCV27902808 / Related Interrogated SNP:
hCV27474895 / Related Interrogated SNP: hCV25474413 / Related Interrogated
SNP: hCV25990131 / SNP Source: Applera / Population(Allele,Count):
Caucasian (A,0|G,4) African American (A,1|G,17) total (A,1|G,21) / SNP Type:
UTR5;INTRON / SNP Source: dbSNP; Celera; HapMap; ABI_Val; HGBASE /
Population(Allele,Count): Caucasian (G,134|A,92) / SNP Type: UTR5;INTRON /
Context (SEQ ID NO: 870): /
TTTCTTAAGATTATCTATGGATGGTTCTCCAACTAGGATTGCGTATGGGACACAAGGGAGCTCTGGTTACTCTTTGAGATTGT
GTAACACTGGGGACAAC
K
CTGGTGAGTAACCTCACTTTTTCGTGGACCTGTCAGGGATGTCTGTCATGTTGATAGTTTGCTTAGTCTTAAGGAATTATGTG
TCTTGTTCTCCTTGGTT / Celera SNP ID: hCV3230004 / Public SNP ID: rs4253301 /
SNP Chromosome Position: 187173012 / SNP in Genomic Sequence: SEQ ID NO: 352 /
SNP Position Genomic: 34340 / Related Interrogated SNP: hCV12066124 /
Related Interrogated SNP: hCV25474413 / Related Interrogated SNP: hCV22272267
/ Related Interrogated SNP: hCV27477533 / Related Interrogated SNP:
hCV3230113 / Related Interrogated SNP: hCV8241630 / SNP Source: Applera /
Population(Allele,Count): Caucasian (G,1|T,1) African American (G,0|T,30) total
(G,1|T,31) / SNP Type: MISSENSE MUTATION;ESE;ESE SYNONYMOUS / SNP Source:
Applera / Population(Allele,Count): Caucasian (G,1|T,9) African American
(G,0|T,12) total (G,1|T,21) / SNP Type: MISSENSE MUTATION;ESE;ESE SYNONYMOUS /
SNP Source: Applera / Population(Allele,Count): Caucasian (G,5|T,25) African
American (G,1|T,27) total (G,6|T,52) / SNP Type: MISSENSE MUTATION;ESE;ESE
SYNONYMOUS / SNP Source: dbSNP; Celera; HapMap; HGBASE /
Population(Allele,Count): Caucasian (T,201|G,25) / SNP Type: MISSENSE
MUTATION;ESE;ESE SYNONYMOUS /
Context (SEQ ID NO: 871): /
CGTAGGCAATAAGAGGACAGTACAGGGTGCCGTCTCTCTCCCTCTTCCTCTCTCTGTCTCTCTCTCTCTGTGTGTGTGTGTGT
GTGTGTGTAACACTA
Y
CTTCCCAATTTTTACTGTCTATTTGTATTCAAAGATAAGGTCCTTATGAAAAATACACTGCTCTGATTCACTTTAAAACTTAT
TTCCATATTTATTATTT / Celera SNP ID: hCV3230014 / Public SNP ID: rs4861709 /
SNP Chromosome Position: 187178186 / SNP in Genomic Sequence: SEQ ID NO: 352 /
SNP Position Genomic: 39514 / Related Interrogated SNP: hCV12066124 /
Related Interrogated SNP: hCV15968043 / Related Interrogated SNP: hCV22272267
/ Related Interrogated SNP: hCV3230113 / Related Interrogated SNP:
hCV11786258 / Related Interrogated SNP: hCV25474413 / Related Interrogated SNP:
hCV15793897 / Related Interrogated SNP: hCV27477533 / Related Interrogated
SNP: hCV8241630 / Related Interrogated SNP: hCV3230096 / SNP Source:

Applera / Population(Allele,Count): Caucasian (C,7|T,29) African American (C,5|T,5) total (C,12|T,34) / SNP Type: INTRON / SNP Source: dbSNP; Celera; HapMap; HGBASE / Population(Allele,Count): Caucasian (C,36|T,84) / SNP Type: INTRON /
Context (SEQ ID NO: 872): /
CTCACAGGTGAAATCCAAAATATTCTACAAAAGGTAAATATTCCTTTGGTAACAAATGAAGAATGCCAGAAAAGATATCAAGA
TTATAAAATAACCCAAC
R
GATGGTCTGTGCTGGCTATAAAGAAGGGGGAAAAGATGCTTGTAAGGTAACTCATGAGATTATGAAAAACACAATAGGCTGCT
TGAGAAAATTCATTTCA / Celera SNP ID: hCV3230016 / Public SNP ID: rs4253325 /
SNP Chromosome Position: 187178473 / SNP in Genomic Sequence: SEQ ID NO: 352 /
SNP Position Genomic: 39801 / Related Interrogated SNP: hCV27474895 /
Related Interrogated SNP: hCV27477533 / Related Interrogated SNP: hCV8241630 /
Related Interrogated SNP: hCV3230038 / Related Interrogated SNP: hCV22272267
/ SNP Source: Applera / Population(Allele,Count): Caucasian (A,2|G,38)
African American (A,3|G,31) total (A,5|G,69) / SNP Type: MISSENSE
MUTATION;ESE;ESS SYNONYMOUS / SNP Source: dbSNP; Celera; HapMap; ABI_Val; HGBASE
/ Population(Allele,Count): Caucasian (G,199|A,25) / SNP Type: MISSENSE
MUTATION;ESE;ESS SYNONYMOUS /
Context (SEQ ID NO: 873): /
TTGTAAGGTAACTCATGAGATTATGAAAAACACAATAGGCTGCTTGAGAAAATTCATTTCAAAATATATTTTCCAATAGCATA
ATTCAATCATAGTTTTT
W
AAAAAATTCAGAGACAAATGATCTGATAAATTGATAAGCAACTTTTAACAAATTGAATATACATAATATATATTTATATTATT
TATGATATATGTCACAA / Celera SNP ID: hCV3230017 / Public SNP ID: rs4253327 /
SNP Chromosome Position: 187178613 / SNP in Genomic Sequence: SEQ ID NO: 352 /
SNP Position Genomic: 39941 / Related Interrogated SNP: hCV15968043 /
Related Interrogated SNP: hCV11786258 / Related Interrogated SNP: hCV27477533
/ Related Interrogated SNP: hCV8241630 / Related Interrogated SNP: hCV3230038
/ Related Interrogated SNP: hCV3230096 / Related Interrogated SNP:
hCV15793897 / Related Interrogated SNP: hCV3230113 / Related Interrogated SNP:
hCV27474895 / Related Interrogated SNP: hCV12066124 / Related Interrogated
SNP: hCV22272267 / Related Interrogated SNP: hCV25474413 / SNP Source:
Applera / Population(Allele,Count): Caucasian (A,10|T,30) African American
(A,19|T,19) total (A,29|T,49) // SNP Type: TFBS SYNONYMOUS;INTRON / SNP
Source: dbSNP; Celera; HapMap; HGBASE / Population(Allele,Count): Caucasian
(A,34|T,72) / SNP Type: TFBS SYNONYMOUS;INTRON /
Context (SEQ ID NO: 874): /
GCAGTCACTTATTTGAATCCCATTGTCGTAACTTTCTACTATTTTATTTTTCCACTGTGACTCAGGGAGATTCAGGTGGTCCC
TTAGTTTGCAAACACAA
Y
GGAATGTGGCGTTTGGTGGGCATCACCAGCTGGGGTGAAGGCTGTGCCCGCAGGGAGCAACCTGGTGTCTACACCAAAGTCGC
TGAGTACATGGACTGGA / Celera SNP ID: hCV3230018 / Public SNP ID: rs925453 /
SNP Chromosome Position: 187179210 / SNP in Genomic Sequence: SEQ ID NO: 352 /
SNP Position Genomic: 40538 / Related Interrogated SNP: hCV12066124 /
Related Interrogated SNP: hCV15968043 / Related Interrogated SNP: hCV22272267
/ Related Interrogated SNP: hCV3230113 / Related Interrogated SNP:
hCV11786258 / Related Interrogated SNP: hCV25474413 / Related Interrogated SNP:
hCV15793897 / Related Interrogated SNP: hCV27477533 / Related Interrogated
SNP: hCV8241630 / Related Interrogated SNP: hCV3230096 / SNP Source:
Applera / Population(Allele,Count): Caucasian (C,30|T,10) African American
(C,22|T,14) total (C,52|T,24) // / SNP Type: SILENT MUTATION / SNP Source:
dbSNP; Celera; HapMap; ABI_Val; HGBASE / Population(Allele,Count): Caucasian
(T,64|C,162) / SNP Type: SILENT MUTATION /
Context (SEQ ID NO: 875): /
CCGGCCCATGAAGGCAGCCGTAGGGGCTGTACCCTGCAAAGCCACAGGGACAGAGCTGCCCAAGGCCTTAACAGCCCACACCT
TGCATCAGCATGCCCTG
K
GTGTGAGACAGGGGCTCCAAGGAGATTATTTTGGAGCTTTAAAATTTAATGACTTCCCTGCTAGGTTTTGCATATGCTTGGGA
CCTGTGGCCCCTTTATT / Celera SNP ID: hCV2103337 / Public SNP ID: rs13102931 /
SNP Chromosome Position: 187145626 / SNP in Genomic Sequence: SEQ ID NO: 352 /
SNP Position Genomic: 6954 / Related Interrogated SNP: hCV12066124 / Related
Interrogated SNP: hCV22272267 / Related Interrogated SNP: hCV3230113 / SNP

Source: dbSNP; Celera; HapMap / Population(Allele,Count): Caucasian (G,108|T,10) / SNP Type: INTRON /
Context (SEQ ID NO: 876): /
CTCCATGTTTTGTGCATACATGAAATCTAGGCAATACACTTGTATTCCCAAAAGCTTCCACTTGAAGAGATCTGTGCTCTTTC
CAAATATAAACCTTACC
Y
GAGAGGTGGTCATCTTGGCCACACCTCAGAGAGGGAGAGAGGCAGTCTTGTTGGGTTGGGTGGTCATAATGGGGCTCAGGGCC
AAATCCCCAGGGGGTAG / Celera SNP ID: hCV3229991 / Public SNP ID: rs4241815 /
SNP Chromosome Position: 187152142 / SNP in Genomic Sequence: SEQ ID NO: 352 /
SNP Position Genomic: 13470 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV27902808 / Related Interrogated SNP:
hCV3230096 / Related Interrogated SNP: hCV8241630 / Related Interrogated SNP:
hCV3230113 / Related Interrogated SNP: hCV3230038 / Related Interrogated SNP:
hCV25474413 / Related Interrogated SNP: hCV27474895 / Related Interrogated
SNP: hCV25990131 / Related Interrogated SNP: hCV32291301 / SNP Source:
dbSNP; Celera; HGBASE / Population(Allele,Count): Caucasian (C,58|T,62) / SNP
Type: INTRON /
Context (SEQ ID NO: 877): /
GCAAAATAAATCATGATTATGGCTAGGAAAATCAGGGATGTAAGTAAGCAAAAATTTAGAAACTACATATTGCATGTAGTCCC
CAAATTCAGAAACAGTC
R
TCGTTAAACATCTTTTATTTAGTCTTTCAGATATTTTTCTACATATACCTATTTGCACATTTCACAAAAAAGAGTTATTAACT
GTGGACACTCTTTGACT / Celera SNP ID: hCV3229992 / Public SNP ID: rs3775298 /
SNP Chromosome Position: 187150478 / SNP in Genomic Sequence: SEQ ID NO: 352 /
SNP Position Genomic: 11806 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV27902808 / Related Interrogated SNP:
hCV3230096 / Related Interrogated SNP: hCV8241630 / Related Interrogated SNP:
hCV3230113 / Related Interrogated SNP: hCV3230038 / Related Interrogated SNP:
hCV25474413 / Related Interrogated SNP: hCV27474895 / Related Interrogated
SNP: hCV25990131 / Related Interrogated SNP: hCV32291301 / SNP Source:
dbSNP; Celera; HGBASE / Population(Allele,Count): Caucasian (A,58|G,62) / SNP
Type: INTRON /
Context (SEQ ID NO: 878): /
CCTCCTCCCACATTTTATTATTCATGCCATAGTTTATATCTTTTGTATGTTGTGTATTCATTAGCAAATTGTTGTAGTTATAG
TTATTTGTTAATACTTT
K
GTTTTTTAACTTTTATACTAGAGTTAAAAGTGACTGTTGCACAATAACAGTATTGGAGTATTCTGTATTTGACTGTATAAGAA
TACCTTTAATAGAGTTT / Celera SNP ID: hCV3229995 / Public SNP ID: rs11132382 /
SNP Chromosome Position: 187143802 / SNP in Genomic Sequence: SEQ ID NO: 352 /
SNP Position Genomic: 5130 / Related Interrogated SNP: hCV11786258 / Related
Interrogated SNP: hCV12066124 / Related Interrogated SNP: hCV15968043 /
Related Interrogated SNP: hCV22272267 / Related Interrogated SNP: hCV27477533
/ Related Interrogated SNP: hCV27902808 / Related Interrogated SNP:
hCV3230096 / Related Interrogated SNP: hCV8241630 / Related Interrogated SNP:
hCV3230113 / Related Interrogated SNP: hCV25474413 / Related Interrogated SNP:
hCV27474895 / Related Interrogated SNP: hCV3230038 / Related Interrogated SNP:
hCV25990131 / Related Interrogated SNP: hCV32291301 / SNP Source: dbSNP;
Celera; HapMap / Population(Allele,Count): Caucasian (T,113|G,111) / SNP Type:
INTRON /
Context (SEQ ID NO: 879): /
ACATTTTACATAAATGGAATCATGCAATGCAGCACATTTTGCATCTGGCTTTTTTCACCTGGCGTGTTTTCAAGGCTCATTCG
TATTCTAGCATATATCA
R
TACTTTGTTCCTATTTAGGACTAAATAAGATTCTATTGTATGAATAAAACATATTTTTGTTTATATACTTAGTTTGATGAACAT
TTGAGTTGTTTCTGGAT / Celera SNP ID: hCV3230000 / Public SNP ID: rs4253294 /
SNP Chromosome Position: 187169650 / SNP in Genomic Sequence: SEQ ID NO: 352 /
SNP Position Genomic: 30978 / Related Interrogated SNP: hCV12066124 /
Related Interrogated SNP: hCV15968043 / Related Interrogated SNP: hCV22272267
/ Related Interrogated SNP: hCV3230113 / Related Interrogated SNP:

hCV11786258 / Related Interrogated SNP: hCV25474413 / Related Interrogated SNP: hCV15793897 / Related Interrogated SNP: hCV27477533 / Related Interrogated SNP: hCV8241630 / Related Interrogated SNP: hCV3230096 / SNP Source: dbSNP; Celera / Population(Allele,Count): Caucasian (A,36|G,84) / SNP Type: INTRON / Context (SEQ ID NO: 880): / TACATTTAGGCATTTGATGCATTTTAAATTAAATTTTGTATATGGTGTAAAGTAGCAATCCAACTTAATTCTTGCATGTGGGT ATTCAGTTATTCCATTG Y

CTTGAAACCCTTTTCAAAATCAATTGTCTATAAATGTAAGAGTTTATTTTTGGACCATCAGTTCTATCCTGTTGACCTATATA TGCCTATCCTAATGCCA / Celera SNP ID: hCV3230001 / Public SNP ID: rs4253296 / SNP Chromosome Position: 187170545 / SNP in Genomic Sequence: SEQ ID NO: 352 / SNP Position Genomic: 31873 / Related Interrogated SNP: hCV12066124 / Related Interrogated SNP: hCV22272267 / Related Interrogated SNP: hCV25474413 / Related Interrogated SNP: hCV3230113 / SNP Source: dbSNP; Celera; HGBASE / Population(Allele,Count): Caucasian (T,108|C,12) / SNP Type: INTRON / Context (SEQ ID NO: 881): / TTACACACAGTCTTGATTACCATAACTTTGCAGTAAGTTTTGAACTCAGACAGTCTGAGTGCTTTTATTTTGTCCTTTTTCAA GATTAATTTGGTTATTC Y

GGATCTTTTGCATTTCCATATGAATTTTAGGATGGTTGTCAATTTCTGCAATAGAAAAGCAGCAGGATTTTGATAGAGAGTGC ATTGAATCTGTAGACCA / Celera SNP ID: hCV3230002 / Public SNP ID: rs4253297 / SNP Chromosome Position: 187170747 / SNP in Genomic Sequence: SEQ ID NO: 352 / SNP Position Genomic: 32075 / Related Interrogated SNP: hCV11786258 / Related Interrogated SNP: hCV12066124 / Related Interrogated SNP: hCV15968043 / Related Interrogated SNP: hCV22272267 / Related Interrogated SNP: hCV27477533 / Related Interrogated SNP: hCV3230038 / Related Interrogated SNP: hCV3230113 / Related Interrogated SNP: hCV8241630 / Related Interrogated SNP: hCV3230096 / Related Interrogated SNP: hCV27474895 / Related Interrogated SNP: hCV25474413 / Related Interrogated SNP: hCV25990131 / Related Interrogated SNP: hCV27902808 / SNP Source: dbSNP; Celera; HGBASE / Population(Allele,Count): Caucasian (C,74|T,46) / SNP Type: INTRON / Context (SEQ ID NO: 882): / GAAGATTAAGTGACACATTTAAAATGCTTAGTACTGTGTGTAGAACATAAACACTTCAAAAAATGTAAACTGTGATTTCTATA TTCAATAAGAAATGTAG M

AATGGACAAAGCATATAAAAAGCAAAAGAAATACTAGAAGACACTTGATTTTTTCTCAAAAATAAACACACCAAGTATTTTTGT TTTAGTGAAATTCATGC / Celera SNP ID: hCV3230006 / Public SNP ID: rs4253308 / SNP Chromosome Position: 187174363 / SNP in Genomic Sequence: SEQ ID NO: 352 / SNP Position Genomic: 35691 / Related Interrogated SNP: hCV11786258 / Related Interrogated SNP: hCV12066124 / Related Interrogated SNP: hCV15968043 / Related Interrogated SNP: hCV22272267 / Related Interrogated SNP: hCV27477533 / Related Interrogated SNP: hCV3230038 / Related Interrogated SNP: hCV3230113 / Related Interrogated SNP: hCV8241630 / Related Interrogated SNP: hCV3230096 / Related Interrogated SNP: hCV27474895 / Related Interrogated SNP: hCV25474413 / Related Interrogated SNP: hCV25990131 / Related Interrogated SNP: hCV27902808 / SNP Source: dbSNP; Celera; HapMap; HGBASE / Population(Allele,Count): Caucasian (A,143|C,83) / SNP Type: INTRON / Context (SEQ ID NO: 883): / ACCTTGCATGTGACTTTAAGGTGGTTGGCCTGGAAGAAAAGTCCCAAGATGGGAAATAGTAGGTGTCTTTTTTACTAAATGCA CTCCAATTTGGGACCAA R

AATTTTCATTCTTGAAGGCTCAGTATTGTGAGTTTATAAGAGATAATAGACATAAAAGTGTAATGATTTCATTGCAAATAAAA AAAGGCCCCTTTGCACC / Celera SNP ID: hCV3230007 / Public SNP ID: rs4253311 / SNP Chromosome Position: 187174683 / SNP in Genomic Sequence: SEQ ID NO: 352 / SNP Position Genomic: 36011 / Related Interrogated SNP: hCV11786258 / Related Interrogated SNP: hCV12066124 / Related Interrogated SNP: hCV15968043 / Related Interrogated SNP: hCV22272267 / Related Interrogated SNP: hCV27477533 / Related Interrogated SNP: hCV27902808 / Related Interrogated SNP: hCV3230096 / Related Interrogated SNP: hCV8241630 / Related Interrogated SNP: hCV3230113 / Related Interrogated SNP: hCV3230038 / Related Interrogated SNP: hCV25474413 / Related Interrogated SNP: hCV27474895 / Related Interrogated SNP: hCV25990131 / Related Interrogated SNP: hCV32291301 / SNP Source:

dbSNP; Celera; HapMap; HGBASE / Population(Allele,Count): Caucasian (A,58|G,62) / SNP Type: INTRON /
Context (SEQ ID NO: 884): /
CCTGACTTCTTGACAGTTCTGAATTTAGTGGAGCAATGAGGTTCAGCTTTGGTGAATGAGCTTAATTTTTCCATGATAAACTG
CTAGTTTCTTCCCACTA
Y
AGTGTCTCTCAAAAATGGGACAGCAACATTCTTTTTGTTTTCACTTGCAGTAAGCATGATGCAATTACATAAATGTACACTTT
TCAATTTGTTAAATAGA / Celera SNP ID: hCV3230010 / Public SNP ID: rs4253315 /
SNP Chromosome Position: 187175457 / SNP in Genomic Sequence: SEQ ID NO: 352 /
SNP Position Genomic: 36785 / Related Interrogated SNP: hCV27474895 /
Related Interrogated SNP: hCV27477533 / Related Interrogated SNP: hCV3230038 /
Related Interrogated SNP: hCV8241630 / Related Interrogated SNP: hCV22272267
/ SNP Source: dbSNP; Celera; HapMap; HGBASE / Population(Allele,Count):
Caucasian (C,107|T,13) / SNP Type: INTRON /
Context (SEQ ID NO: 885): /
GCAACACAGTGAGACCCCGTCTCTACATAAAATTAGAAAAAAACAATTAACTGGGTGTGGTGGTGTGCACCTGTAGTCCCAGC
TAGTCAAGAGGCTGAGA
Y
AAGAGGATCTCTTGAGCCCAGGAGCTCAAGGCTGTAGTGAGCCAAGATCGTGCCACTGCACACAAACAATTATGTGACCTCGG
GCAAGTTGCTTTACCTC / Celera SNP ID: hCV3230011 / Public SNP ID: rs4253320 /
SNP Chromosome Position: 187176626 / SNP in Genomic Sequence: SEQ ID NO: 352 /
SNP Position Genomic: 37954 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV3230038 / Related Interrogated SNP:
hCV3230113 / Related Interrogated SNP: hCV8241630 / Related Interrogated SNP:
hCV3230096 / Related Interrogated SNP: hCV27474895 / Related Interrogated SNP:
hCV25474413 / Related Interrogated SNP: hCV25990131 / Related Interrogated
SNP: hCV27902808 / SNP Source: dbSNP; Celera; HGBASE /
Population(Allele,Count): Caucasian (C,74|T,46) / SNP Type: INTRON /
Context (SEQ ID NO: 886): /
CTCTTAATTTCCTTATCTGTAAAATGAGGATGATAATTTCTTCCTGGGTCTGTTGTAATAATTAATACATCAAAGCACTTCAT
GTCTGGAACAGTGAAGA
Y
ACCCTGCTATGACTATTAAGGATAGTATACATGGAATAAGACACAGGAACTTCTAAATGCTTTTGACCATAGATTTAGGTTCT
GAGTTTTAAGAATTTAA / Celera SNP ID: hCV3230012 / Public SNP ID: rs4241821 /
SNP Chromosome Position: 187176834 / SNP in Genomic Sequence: SEQ ID NO: 352 /
SNP Position Genomic: 38162 / Related Interrogated SNP: hCV22272267 /
Related Interrogated SNP: hCV15793897 / Related Interrogated SNP: hCV27902808
/ Related Interrogated SNP: hCV15968043 / Related Interrogated SNP:
hCV11786258 / Related Interrogated SNP: hCV3230113 / SNP Source: dbSNP;
Celera; HapMap; HGBASE / Population(Allele,Count): Caucasian (T,39|C,187) / SNP
Type: INTRON /
Context (SEQ ID NO: 887): /
TGAATATATAAAGCAAAAAAATGAAAAGTGAGAACTTCCAGGCTTTGGATTGTTGTAGGTGATAAATATAAAATGGGATTTCT
GGGGGGCTGCTACTGAG
W
TGAGGGGATGGCAGAAAACATGGAAGCAAGGTCTCTGGTCAGCCCAGGGTGCTGGGCTTGTCCCAACACCACGTAGGCAATAA
GAGGACAGTACAGGGTG / Celera SNP ID: hCV3230013 / Public SNP ID: rs3775303 /
SNP Chromosome Position: 187178014 / SNP in Genomic Sequence: SEQ ID NO: 352 /
SNP Position Genomic: 39342 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV3230038 / Related Interrogated SNP:
hCV3230113 / Related Interrogated SNP: hCV8241630 / Related Interrogated SNP:
hCV3230096 / Related Interrogated SNP: hCV27474895 / Related Interrogated SNP:
hCV25474413 / Related Interrogated SNP: hCV27902808 / Related Interrogated
SNP: hCV25990131 / SNP Source: dbSNP; Celera; HGBASE /
Population(Allele,Count): Caucasian (A,71|T,49) / SNP Type: INTRON /
Context (SEQ ID NO: 888): /
TCCCTCTAATTGCACTGCTTCCTCTGGAACTCAGTATATCTCAAAGATGTAATTTCCTCTCCGTGCTGCACCTGGTCGGCCAC
TGAAACCCACTATTGCC
Y

GCTTCACGTGTGGCAAAGAGCTAGCGGGCTTGGGTTTTGTTCTGCCGAGAGGAAGGGAGAACACCCACTTTTATAAGAAAGAG
ATGGGTTACCTGAACCC / Celera SNP ID: hCV3230019 / Public SNP ID: rs4253332 /
SNP Chromosome Position: 187179803 / SNP in Genomic Sequence: SEQ ID NO: 352 /
SNP Position Genomic: 41131 / Related Interrogated SNP: hCV12066124 /
Related Interrogated SNP: hCV15968043 / Related Interrogated SNP: hCV22272267
/ Related Interrogated SNP: hCV3230113 / Related Interrogated SNP:
hCV11786258 / Related Interrogated SNP: hCV25474413 / Related Interrogated SNP:
hCV15793897 / Related Interrogated SNP: hCV27477533 / Related Interrogated
SNP: hCV8241630 / Related Interrogated SNP: hCV3230096 / SNP Source: dbSNP;
Celera; HapMap; HGBASE / Population(Allele,Count): Caucasian (T,62|C,162) / SNP
Type: INTERGENIC;UNKNOWN /
Context (SEQ ID NO: 889): /
CTTTTATCTCAGTCTTTGAGTATTGATCATGGGGTGTTGGAACAGGACTTTGGAATGCTTGCAGGGTAAACCTTTGGCCTCTG
TTAGTCAGGGAATGACC
Y

AGTTTGGCAAAACAGAGGAGAGTTTTGAAATATGGAACTTTCCCGAGGCATACATTGTCATTTTAAAGTGGTCAATCAAAGCC
CAGTAGGACTGGGCTGG / Celera SNP ID: hCV3230021 / Public SNP ID: rs13135645 /
SNP Chromosome Position: 187181863 / SNP in Genomic Sequence: SEQ ID NO: 352 /
SNP Position Genomic: 43191 / Related Interrogated SNP: hCV12066124 /
Related Interrogated SNP: hCV25474413 / Related Interrogated SNP: hCV27477533
/ Related Interrogated SNP: hCV8241630 / Related Interrogated SNP:
hCV27474895 / Related Interrogated SNP: hCV3230038 / Related Interrogated SNP:
hCV15968043 / SNP Source: dbSNP; Celera; HapMap / Population(Allele,Count):
Caucasian (T,193|C,33) / SNP Type: INTERGENIC;UNKNOWN /
Context (SEQ ID NO: 890): /
TGATATCCGGTTGCTTCTCACTAGGAGAAGGATGAAAGATGACAGAGCATTTATAACCACCATTTGTTATTTTCATTATCAAC
TGACACTGGTGTTTCTC
Y

GCCCGGAGCGATTTTGTCCTCCCTTCACCTCAGGAAACATTCGGCAACGTCTAGAGACACTTTTGGTTGTCCTAATGGGAGAG
GGTATGCAACTGGCTGT / Celera SNP ID: hCV3230022 / Public SNP ID: rs11132383 /
SNP Chromosome Position: 187182660 / SNP in Genomic Sequence: SEQ ID NO: 352 /
SNP Position Genomic: 43988 / Related Interrogated SNP: hCV12066124 /
Related Interrogated SNP: hCV25474413 / Related Interrogated SNP: hCV27474895
/ Related Interrogated SNP: hCV27477533 / Related Interrogated SNP:
hCV3230038 / Related Interrogated SNP: hCV8241630 / Related Interrogated SNP:
hCV15968043 / Related Interrogated SNP: hCV3230096 / Related Interrogated SNP:
hCV11786258 / Related Interrogated SNP: hCV3230113 / SNP Source: dbSNP;
Celera; HapMap / Population(Allele,Count): Caucasian (T,121|C,105) / SNP Type:
INTERGENIC;UNKNOWN /
Context (SEQ ID NO: 891): /
GACTCGTCAGCCAGTGAGTAGGTCTGGGTAGCAGTTGGCATGGATGAACATGCCCTCCATAGGCTTTCAGACCTTCTTCAAGG
CCAAAGGGAAGGCCTTC
R

TGGAAATATAATTATGTGAAACACCCCAGAATTTTTTTCACAAACTTTCCTGCCTATAAACGCCAGGTCCTACCACTGTCCAGT
GTCCCACTTCCCACTGT / Celera SNP ID: hCV3230025 / Public SNP ID: rs3756009 /
SNP Chromosome Position: 187186111 / SNP in Genomic Sequence: SEQ ID NO: 352 /
SNP Position Genomic: 47439 / Related Interrogated SNP: hCV12066124 /
Related Interrogated SNP: hCV15968043 / Related Interrogated SNP: hCV27474895
/ Related Interrogated SNP: hCV25474413 / Related Interrogated SNP:
hCV27477533 / Related Interrogated SNP: hCV3230038 / Related Interrogated SNP:
hCV8241630 / Related Interrogated SNP: hCV11786258 / Related Interrogated SNP:
hCV3230096 / Related Interrogated SNP: hCV3230113 / Related Interrogated SNP:
hCV22272267 / Related Interrogated SNP: hCV25990131 / SNP Source: dbSNP;
Celera; HapMap / Population(Allele,Count): Caucasian (A,60|G,40) / SNP Type:
INTERGENIC;UNKNOWN /
Context (SEQ ID NO: 892): /
TACAATTTTTGAAATAAAAATACTAGATTCAAAGGTGTGAATATTTTTGAAGGTTTTGTGGTGTGGTATCATGAAATTAGGTT
TCAGAAAGTTTACTCCA
Y

AGTTGACTTTTTCTACCAGCTAATAAGAGTGCTCATTTACCCCACTTAGGCCAACTCTAACAGGCTTCTGTTGCTTTGCATCC
TGACATATTTATCTTTC / Celera SNP ID: hCV8241631 / Public SNP ID: rs1511802 /
SNP Chromosome Position: 187150806 / SNP in Genomic Sequence: SEQ ID NO: 352 /
SNP Position Genomic: 12134 / Related Interrogated SNP: hCV11786258 /

Related Interrogated SNP: hCV12066124 / Related Interrogated SNP: hCV15968043 / Related Interrogated SNP: hCV22272267 / Related Interrogated SNP: hCV27477533 / Related Interrogated SNP: hCV3230038 / Related Interrogated SNP: hCV3230113 / Related Interrogated SNP: hCV8241630 / Related Interrogated SNP: hCV3230096 / Related Interrogated SNP: hCV27474895 / Related Interrogated SNP: hCV25474413 / Related Interrogated SNP: hCV25990131 / Related Interrogated SNP: hCV27902808 / SNP Source: dbSNP; Celera; HapMap; ABI_Val; HGBASE / Population(Allele,Count): Caucasian (T,142|C,82) / SNP Type: INTRON / Context (SEQ ID NO: 893): /
CAAATAAGTAATATATTTTGGAGCTTGCTATTCTTCTCTTTTTATGGTATTTTTGTGTGCACATTCTTATGCTCTTATTCAGT
TATTTCTGTAGAATACA
W
TTTTTGAAATAAAAATACTAGATTCAAAGGTGTGAATATTTTTGAAGGTTTTGTGGTGTGGTATCATGAAATTAGGTTTCAGA
AAGTTTACTCCATAGTT / Celera SNP ID: hCV8241632 / Public SNP ID: rs1511801 /
SNP Chromosome Position: 187150710 / SNP in Genomic Sequence: SEQ ID NO: 352 / SNP Position Genomic: 12038 / Related Interrogated SNP: hCV11786258 /
Related Interrogated SNP: hCV12066124 / Related Interrogated SNP: hCV15968043 / Related Interrogated SNP: hCV22272267 / Related Interrogated SNP: hCV27477533 / Related Interrogated SNP: hCV3230038 / Related Interrogated SNP: hCV3230113 / Related Interrogated SNP: hCV8241630 / Related Interrogated SNP: hCV3230096 / Related Interrogated SNP: hCV27902808 / Related Interrogated SNP: hCV25474413 / Related Interrogated SNP: hCV27474895 / Related Interrogated SNP: hCV25990131 / SNP Source: dbSNP; HapMap; HGBASE / Population(Allele,Count): Caucasian (A,52|T,54) / SNP Type: INTRON /
Context (SEQ ID NO: 894): /
CCACCCTACTTGGGAACCCAAGAGCCAGTATATTGCCGTTTGCTTAAAAGGACTGTCTTTCATTTATGACCAAAAGCGAGTTA
GTGTGTGTCAGGTCAGC
Y
GAAGGATGAAGGAAGGGCCCCCATTCTCTGGATATGATCTTAGGTCTTCACCCTATTTTCTTTGTGCTGTTCAATAATTCTCT
GTATTGGATCATCAGCA / Celera SNP ID: hCV8241633 / Public SNP ID: rs1511800 /
SNP Chromosome Position: 187135556 / SNP in Genomic Sequence: SEQ ID NO: 352 / SNP Position Genomic: 3116 / Related Interrogated SNP: hCV15793897 / Related Interrogated SNP: hCV12066124 / Related Interrogated SNP: hCV27474895 /
Related Interrogated SNP: hCV25474413 / Related Interrogated SNP: hCV3230113 / Related Interrogated SNP: hCV8241630 / Related Interrogated SNP: hCV27477533 / Related Interrogated SNP: hCV25990131 / Related Interrogated SNP: hCV3230038 / SNP Source: dbSNP; HGBASE / Population(Allele,Count): Caucasian (C,21|T,205) / SNP Type: NONSENSE MUTATION;TRANSCRIPTION FACTOR BINDING
SITE;INTRON /
Context (SEQ ID NO: 895): /
GTGCTATTTCTGATTGTTGTATCTTGTAGCAAAATTTAAAGAAAAAGTAATTTGTGCCTTTCTCAATATTCCTGTTATTGTTC
ATGTATTCTAAAACTCA
S
TGTTACTCACTTAGCTTGCTTTTAATGTTTTTTTAAAGTGAAAAATTGTTCCCAAGTACATAAAATCTCTACACTCAAGAACAA
TTCTAGTCAAAAGCATT / Celera SNP ID: hCV11786203 / Public SNP ID: rs4253251 /
SNP Chromosome Position: 187156473 / SNP in Genomic Sequence: SEQ ID NO: 352 / SNP Position Genomic: 17801 / Related Interrogated SNP: hCV22272267 /
Related Interrogated SNP: hCV15793897 / Related Interrogated SNP: hCV27902808 / Related Interrogated SNP: hCV15968043 / Related Interrogated SNP: hCV11786258 / Related Interrogated SNP: hCV12066124 / Related Interrogated SNP: hCV3230113 / SNP Source: dbSNP; Celera / Population(Allele,Count): Caucasian (C,24|G,96) / SNP Type: INTRON /
Context (SEQ ID NO: 896): /
TCCTGTAGGAATGCTTGACCACTGCAGTTTAGGCATTTTGAAGTTTTTGTGTGTGCTGGAGATGTGGCTGGGTTTTGTCTCAC
AGCAGAGGCAAGGAATC
R
CAACTCAGAAATACATTGCTACTTGGCTGCCTCTATTATTGTACATCTTGAAGGCGAGGTTAATTAAGTCCTCTTGTGGGGTT
TGAGGGCTGGAATCTAA / Celera SNP ID: hCV11786235 / Public SNP ID: rs4253287 /
SNP Chromosome Position: 187166847 / SNP in Genomic Sequence: SEQ ID NO: 352 / SNP Position Genomic: 28175 / Related Interrogated SNP: hCV27474895 /
Related Interrogated SNP: hCV3230038 / Related Interrogated SNP: hCV27477533 / Related Interrogated SNP: hCV8241630 / Related Interrogated SNP: hCV15968043 / Related Interrogated SNP: hCV3230096 / Related Interrogated SNP:

hCV11786258 / Related Interrogated SNP: hCV12066124 / SNP Source: dbSNP; Celera / Population(Allele,Count): Caucasian (A,50|G,70) / SNP Type: INTRON /
Context (SEQ ID NO: 897): /
TTTAACTTATAGGGTCCAAGCACTGACCAACCTGACCATTAGAACAGAGTGTGGTCTCTGTACAGGGCAGATGGCGCTGAGTG
GGTATTCTCCACAGAAA
S
AGAAACGAAGACAGTACCCCACTCCTCCAACCCACCACCCACCACCAATCCCACCACCAATTCCACCACCAATCCTGCCACCC
ACCACCAATCTCACCAC / Celera SNP ID: hCV11786259 / Public SNP ID: rs4253304 /
SNP Chromosome Position: 187173571 / SNP in Genomic Sequence: SEQ ID NO: 352 /
SNP Position Genomic: 34899 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV3230038 / Related Interrogated SNP:
hCV3230113 / Related Interrogated SNP: hCV8241630 / Related Interrogated SNP:
hCV3230096 / Related Interrogated SNP: hCV27474895 / Related Interrogated SNP:
hCV25474413 / Related Interrogated SNP: hCV27902808 / Related Interrogated
SNP: hCV25990131 / SNP Source: dbSNP; Celera; HGBASE /
Population(Allele,Count): Caucasian (G,71|C,49) / SNP Type: INTRON /
Context (SEQ ID NO: 898): /
GAATGCACATCACCACCCTGCATTATAAACCCTTTTGGTTTTAGTATCTCTAGCATTTTGTCATTTATGTAAATGCAGACATC
GGTAAAATGAGGCCAGA
R
TGAAACGGGTGAATCTGAAGCTTTTTTTGGTGAATCCATAGTAAATGAAAGACCCACTAAGACATAATCTAGGGTAGTGAGTCT
CAAGCTTGAAGGAGCTC / Celera SNP ID: hCV12066116 / Public SNP ID: rs1877320 /
SNP Chromosome Position: 187131279 / SNP in Genomic Sequence: SEQ ID NO: 352 /
SNP Position Genomic: 7393 / Related Interrogated SNP: hCV12066124 / Related
Interrogated SNP: hCV15793897 / Related Interrogated SNP: hCV25474413 /
Related Interrogated SNP: hCV27477533 / Related Interrogated SNP: hCV3230038 /
Related Interrogated SNP: hCV8241630 / Related Interrogated SNP: hCV25990131
/ Related Interrogated SNP: hCV3230113 / Related Interrogated SNP:
hCV27474895 / SNP Source: dbSNP; HapMap; HGBASE / Population(Allele,Count):
Caucasian (G,12|A,106) / SNP Type: INTRON /
Context (SEQ ID NO: 899): /
GGGCCAGGCTAGAGCTGGAGCAGGCCTGAAGTCTAGGTTTTGTGTGGCCATCTTGGAGCCTGAAGCCCCAGGGGCTGACCTGG
TCTGGGGTGAGCATGGG
K
CTGAGGCCACAGAGGCTGGTCTGGCCTGTGGCAGGCCTGAATCCTGGTGCTGGGGTTTACTGGAGTGGGCTTGGTGCTTGGGA
TCTGTGGTGAAGTTAGG / Celera SNP ID: hCV12066118 / Public SNP ID: rs2048 / SNP
Chromosome Position: 187148133 / SNP in Genomic Sequence: SEQ ID NO: 352 /
SNP Position Genomic: 9461 / Related Interrogated SNP: hCV11786258 / Related
Interrogated SNP: hCV12066124 / Related Interrogated SNP: hCV15968043 /
Related Interrogated SNP: hCV22272267 / Related Interrogated SNP: hCV27902808
/ Related Interrogated SNP: hCV27902808 / Related Interrogated SNP:
hCV3230113 / Related Interrogated SNP: hCV8241630 / Related Interrogated SNP:
hCV3230096 / Related Interrogated SNP: hCV25474413 / Related Interrogated SNP:
hCV27474895 / Related Interrogated SNP: hCV3230038 / Related Interrogated SNP:
hCV25990131 / SNP Source: dbSNP; HapMap; HGBASE / Population(Allele,Count):
Caucasian (G,58|T,60) / SNP Type: INTRON /
Context (SEQ ID NO: 900): /
GGCTAAGACAGGAGTAGCCAAGCAAGTGGCACCACCCCTGGAGAAAGCTATTGAACATACAGCTTCGGGGGTGGAGATTGTCC
CTGATGATTCAGGACAC
R
TGTCTATTTAATGTTCCACAACAAGGACCACTTGTCAGGTATATTGCTGTAGACATATGTTGCAGACCAGAGGAAGGAGCTCA
GAAGTAGGAATGTCTTG / Celera SNP ID: hCV12066119 / Public SNP ID: rs1912826 /
SNP Chromosome Position: 187149540 / SNP in Genomic Sequence: SEQ ID NO: 352 /
SNP Position Genomic: 10868 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV27902808 / Related Interrogated SNP:
hCV3230113 / Related Interrogated SNP: hCV8241630 / Related Interrogated SNP:
hCV3230096 / Related Interrogated SNP: hCV25474413 / Related Interrogated
SNP: hCV27474895 / Related Interrogated SNP: hCV3230038 / Related

Interrogated SNP:   hCV25990131 /  SNP Source:   dbSNP; HapMap; ABI_Val; HGBASE /
Population(Allele,Count):   Caucasian (G,111|A,113) /  SNP Type:   INTRON /
Context            (SEQ ID NO: 901): /
TACAAATTCTCTGATCCTCCCCAGCTATAGATGTGCAAGGTGGGATCAGGGAGGGAAACTCAAGACTCTTCATCTTTAACAGG
TGTTCCAAGCCATTCTG
R
TTCTCCTTCCACCTACTGCGAAAATGTAAAGTGGCTCGCTTCCTATGACAGGACCTCTTGTTTCTCACATTTGGGGTAAATCT
ATAACTAAAGCGATGGT /  Celera SNP ID:   hCV15811716 /  Public SNP ID:   rs2102575 /
SNP Chromosome Position:   187131504 /  SNP in Genomic Sequence:   SEQ ID NO: 352 /
 SNP Position Genomic:   7168 /  Related Interrogated SNP:   hCV15793897 /  Related
Interrogated SNP:   hCV12066124 /  Related Interrogated SNP:   hCV25474413 /
Related Interrogated SNP:   hCV27474895 /  Related Interrogated SNP:   hCV22272267
/  Related Interrogated SNP:   hCV25990131 /  Related Interrogated SNP:
hCV27477533 /  Related Interrogated SNP:   hCV3230038 /  Related Interrogated SNP:
 hCV3230113 /  Related Interrogated SNP:   hCV8241630 /  SNP Source:   dbSNP;
HapMap; HGBASE; /  Population(Allele,Count):   Caucasian (G,19|A,207) /  SNP Type:
 INTRON /
Context            (SEQ ID NO: 902): /
CTTTTTAAGTAAATGAAAGTATTTGCAAAAATAATTACCAATTGAGAACATAGTTGCCTGAAAGTTTAAGAACACAGGAAAAA
TCATTAACTCTTTAATA
Y
GGTTGATTTCCTGTACTTAAAAAATGTGAGTGTAAAGAAAACGCAGTGATGGAGTTAGATATTATGGGGTGTTATAAAATTAG
CTCTAAGAGTGTTCTTT /  Celera SNP ID:   hCV15975109 /  Public SNP ID:   rs2304596 /
SNP Chromosome Position:   187154873 /  SNP in Genomic Sequence:   SEQ ID NO: 352 /
 SNP Position Genomic:   16201 /  Related Interrogated SNP:   hCV25990131 /
Related Interrogated SNP:   hCV32291301 /  Related Interrogated SNP:   hCV22272267
/  Related Interrogated SNP:   hCV3230113 /  Related Interrogated SNP:
hCV27902808 /  Related Interrogated SNP:   hCV11786258 /  Related Interrogated SNP:
 hCV3230096 /  Related Interrogated SNP:   hCV12066124 /  Related Interrogated
SNP:   hCV15968043 /  Related Interrogated SNP:   hCV25474413 /  SNP Source:
dbSNP; HapMap; HGBASE /  Population(Allele,Count):   Caucasian (T,98|C,22) /  SNP
Type:   INTRON /
Context            (SEQ ID NO: 903): /
TCTGGAGGTCTCAATTTGTATCTTGGGAAGCATTATAAATTTTCCAACTCCCTAGACACAAATGTACCAAAAAAAAAACCCTTG
TTTTCTATACCAGTAAT
Y
GTGTGCTTTGTCTTGCAATTCAGACATTTACAAGAAAATCTAAATCACCTTAAATTAAGATTTATGTTAAATGTGGTCTAAAA
CCAGCAGAGTTATGTAT /  Celera SNP ID:   hCV25634763 /  Public SNP ID:   rs4253241 /
SNP Chromosome Position:   187149233 /  SNP in Genomic Sequence:   SEQ ID NO: 352 /
 SNP Position Genomic:   10561 /  Related Interrogated SNP:   hCV12066124 /
Related Interrogated SNP:   hCV22272267 /  Related Interrogated SNP:   hCV25474413
/  Related Interrogated SNP:   hCV3230113 /  SNP Source:   dbSNP /
Population(Allele,Count):   Caucasian (C,12|T,108) /  SNP Type:   INTRON /
Context            (SEQ ID NO: 904): /
GTGAGTCAGTTAAACCTCTTTTGTTTATAAATTACCCAGTCTCAGGTAGTATCTTTACAGCAGTGTGAAACTGGACTAATACA
TCTGGGAAATTCTTTTT
S
TCTCCTTCCTTTCTGAAGGACAGCTTTGCCTGATATTATATTCTTGGTTGCCAGGTTTTGTACTTTCAATACTTTGAATATAT
CATCCCACTCTCTCTTG /  Celera SNP ID:   hCV26265197 /  Public SNP ID:   rs10014399 /
SNP Chromosome Position:   187146359 /  SNP in Genomic Sequence:   SEQ ID NO: 352 /
 SNP Position Genomic:   7687 /  Related Interrogated SNP:   hCV22272267 /  Related
Interrogated SNP:   hCV15793897 /  Related Interrogated SNP:   hCV27902808 /
Related Interrogated SNP:   hCV15968043 /  Related Interrogated SNP:   hCV11786258
/  Related Interrogated SNP:   hCV12066124 /  Related Interrogated SNP:
hCV3230113 /  SNP Source:   dbSNP; Celera; HapMap /  Population(Allele,Count):
Caucasian (G,24|C,94) /  SNP Type:   INTRON /
Context            (SEQ ID NO: 905): /
TTGGGAGATGATTGGATCACGAGGTGTTTTCCCCCATTATGTTCTCATGATAGTGAGGGTGTTCTCATGAGATCTGATGGTTT
AAAAGTGGTGGTTACCC
S
TGCGCACATGCTCTCTCTCTCTCCTGCTGCCTTGTGAAGAAGGTACCTGCTTCCCCTTCACCTTCCACCATAATTGTAAGTTT
CCTGAGGCCTCCCCAGC /  Celera SNP ID:   hCV26265199 /  Public SNP ID:   rs2221843 /
SNP Chromosome Position:   187144200 /  SNP in Genomic Sequence:   SEQ ID NO: 352 /

SNP Position Genomic: 5528 / Related Interrogated SNP: hCV22272267 / Related Interrogated SNP: hCV15793897 / Related Interrogated SNP: hCV27902808 / Related Interrogated SNP: hCV15968043 / Related Interrogated SNP: hCV11786258 / Related Interrogated SNP: hCV3230113 / SNP Source: dbSNP; HapMap; HGBASE / Population(Allele,Count): Caucasian (G,39|C,187) / SNP Type: INTRON / Context (SEQ ID NO: 906): /
TAGACTTTGAGATTCAAGGTCAGCTAGAATTAATGGTTAACAGCTGACAGGTGAAAGTGTGGTTAAATGCAGCTTTGGCTAGC
AGGCACACAGGCAAAAT
S
AAGTTCTACATCTGTCCCTGTGTATGTCACTTGTTTGAATACGAAATAAAATTAAAAAAATAAATTCAGTGTATTGAGAAAGC
AAGCAATTCTCTCAAGG / Celera SNP ID: hCV27473099 / Public SNP ID: rs3733403 /
SNP Chromosome Position: 187187135 / SNP in Genomic Sequence: SEQ ID NO: 352 /
SNP Position Genomic: 48463 / Related Interrogated SNP: hCV12066124 /
Related Interrogated SNP: hCV25474413 / Related Interrogated SNP: hCV8241630 /
Related Interrogated SNP: hCV22272267 / Related Interrogated SNP: hCV27474895
/ Related Interrogated SNP: hCV27477533 / SNP Source: dbSNP; HapMap; HGBASE /
Population(Allele,Count): Caucasian (C,101|G,15) / SNP Type: UTR5;PSEUDOGENE
/
Context (SEQ ID NO: 907): /
ATGGTGTGTGTGTGTATGTGTGCTTGTGCTTGTGTGAATATGTATTATTAACTGGAAATTTGTAAAAGTATTGGAAAAATAGT
ACTTACGATTTTGTGTG
Y
GTGTGTGATGGAGTCTCGCTTTGTAGCCCAGGCTGGAGTGCAGTGGCGTGATCTCGGCTCACTGCAACTTCCACCTCCCAGGT
TCAAGCGATTCTCCTGC / Celera SNP ID: hCV27482765 / Public SNP ID: rs3775301 /
SNP Chromosome Position: 187154082 / SNP in Genomic Sequence: SEQ ID NO: 352 /
SNP Position Genomic: 15410 / Related Interrogated SNP: hCV25990131 /
Related Interrogated SNP: hCV32291301 / Related Interrogated SNP: hCV22272267
/ Related Interrogated SNP: hCV3230113 / Related Interrogated SNP:
hCV27902808 / Related Interrogated SNP: hCV11786258 / Related Interrogated SNP:
hCV3230096 / Related Interrogated SNP: hCV12066124 / Related Interrogated
SNP: hCV15968043 / Related Interrogated SNP: hCV25474413 / SNP Source:
dbSNP; HGBASE / Population(Allele,Count): Caucasian (C,22|T,98) / SNP Type:
INTRON /
Context (SEQ ID NO: 908): /
CAAGTAATTCACATTTTTAGATTTTTTTATTGGTAATCTGAGACAAGAAGAAATTTAAAGTAATCTTCACTAAGCCATGAAAGC
TCCCAACATTGTTCTCC
R
TGAGAGATGCTGGCCTGCATTTATTCAAAAACAAAAGACCCCTCTGTTGCCAAAGCTCGGAGGGCTTTTCAGAAACGATATAG
TTGTAAATTATAATTTT / Celera SNP ID: hCV27482766 / Public SNP ID: rs3775302 /
SNP Chromosome Position: 187177814 / SNP in Genomic Sequence: SEQ ID NO: 352 /
SNP Position Genomic: 39142 / Related Interrogated SNP: hCV22272267 / SNP
Source: dbSNP; HapMap; HGBASE / Population(Allele,Count): Caucasian
(A,195|G,31) / SNP Type: INTRON /
Context (SEQ ID NO: 909): /
CTGAGATCTCCATGACTGTGTGTTGTGAAATAAAATGGTGAAAGATCACGATTAGCAAGTGTTTTCTTCTGGTTGTGAAACAG
AACTGAAAGTAAGTGGT
Y
GAGGTTCCAGCACAGTTCCTGGGATCCCTCTAATTGCACTGCTTCCTCTGGAACTCAGTATATCTCAAAGATGTAATTTCCTC
TCCGTGCTGCACCTGGT / Celera SNP ID: hCV27506149 / Public SNP ID: rs3822055 /
SNP Chromosome Position: 187179678 / SNP in Genomic Sequence: SEQ ID NO: 352 /
SNP Position Genomic: 41006 / Related Interrogated SNP: hCV22272267 /
Related Interrogated SNP: hCV15793897 / Related Interrogated SNP: hCV27902808
/ Related Interrogated SNP: hCV15968043 / Related Interrogated SNP:
hCV11786258 / Related Interrogated SNP: hCV3230113 / SNP Source: dbSNP;
HapMap; HGBASE / Population(Allele,Count): Caucasian (T,39|C,187) / SNP Type:
INTERGENIC;UNKNOWN /
Context (SEQ ID NO: 910): /
CAAGAGCCAGGCTCTTAGGAGCTGTCTGTACTTTGCTTCCCATCCGTCCGCTCTCCCCCAGCAGCCAAAGTCTCCTCCCTCCA
TTATATTGCAAATCAAT
K
AATCCATTAGACTTTCCATGTTTTCTTTTTAGACTTTGAGATTCAAGGTCAGCTAGAATTAATGGTTAACAGCTGACAGGTGA
AAGTGTGGTTAAATGCA / Celera SNP ID: hCV27521729 / Public SNP ID: rs3822056 /
SNP Chromosome Position: 187187005 / SNP in Genomic Sequence: SEQ ID NO: 352 /

SNP Position Genomic: 48333 / Related Interrogated SNP: hCV12066124 / Related Interrogated SNP: hCV25474413 / Related Interrogated SNP: hCV8241630 / Related Interrogated SNP: hCV27477533 / Related Interrogated SNP: hCV3230038 / Related Interrogated SNP: hCV22272267 / SNP Source: dbSNP; HapMap; HGBASE / Population(Allele,Count): Caucasian (G,198|T,28) / SNP Type: INTERGENIC;UNKNOWN /
Context                (SEQ ID NO: 911): /
CTCTTTATCCTAGTAGATAAGTTTTTGGCACGTTCTTTCTCCTTCCCATCATTATCTCTCCTCTTTACCCGTCACTTTCTTTC
TGTTGTGGTTTGCATTT
R

TTGATCTCTGCCTTTCCTGATACTACTCAATTTATGTTTCACAGAAATAACGGCTGTACTTAAATGAAACTCAGATTCCTATA
TAAAAATGTTGGGATAA / Celera SNP ID: hCV27902803 / Public SNP ID: rs4862665 / SNP Chromosome Position: 187136142 / SNP in Genomic Sequence: SEQ ID NO: 352 / SNP Position Genomic: 2530 / Related Interrogated SNP: hCV15793897 / Related Interrogated SNP: hCV12066124 / Related Interrogated SNP: hCV27474895 / Related Interrogated SNP: hCV25474413 / Related Interrogated SNP: hCV3230113 / Related Interrogated SNP: hCV8241630 / Related Interrogated SNP: hCV27477533 / Related Interrogated SNP: hCV25990131 / Related Interrogated SNP: hCV3230038 / SNP Source: dbSNP; HGBASE / Population(Allele,Count): Caucasian (A,21|G,205) / SNP Type: INTRON /
Context                (SEQ ID NO: 912): /
TGCTAACAGGCCATGGACCAGGTACTGTTCTGGGCCCCGGGGGGTTGGGAACCCCTGCTCAGAGACACACCGGGTGGTAGGAGG
AGCTAAAGGTGGAGAGG
S

TGAAGGAAAATATGAGGTCTGGGCTATCCACAAACCAGATAGCAGGAAACTGAAGAGTTAAACATTTACTTCAGAATTGAATG
GCTTTTGTAAAATCTGG / Celera SNP ID: hCV28960679 / Public SNP ID: rs6844764 / SNP Chromosome Position: 187181533 / SNP in Genomic Sequence: SEQ ID NO: 352 / SNP Position Genomic: 42861 / Related Interrogated SNP: hCV11786258 / Related Interrogated SNP: hCV15968043 / Related Interrogated SNP: hCV27477533 / Related Interrogated SNP: hCV3230038 / Related Interrogated SNP: hCV27474895 / Related Interrogated SNP: hCV3230096 / Related Interrogated SNP: hCV8241630 / Related Interrogated SNP: hCV12066124 / Related Interrogated SNP: hCV3230113 / Related Interrogated SNP: hCV25474413 / Related Interrogated SNP: hCV25990131 / Related Interrogated SNP: hCV32291301 / SNP Source: dbSNP; HapMap / Population(Allele,Count): Caucasian (G,61|C,53) / SNP Type: INTERGENIC;UNKNOWN /
Context                (SEQ ID NO: 913): /
CAGAGGAAAACTCTGCTTTTAATGGGCTCATGTAATAAGATTTGGCCCACTCTGATTGACTCCATTTTGATTGGCACAAAGCC
AAGTGTTTAGTAATCTT
R

ACCCCATCTGCAGAATTGCTTTTCTCATGTAACATAATCATGGGCAGATGTGATATCTCGTTTTATTCACTGTCCCCAGGATT
TGAGTGGGAGATTTGGG / Celera SNP ID: hCV29053260 / Public SNP ID: rs4861707 / SNP Chromosome Position: 187138092 / SNP in Genomic Sequence: SEQ ID NO: 352 / SNP Position Genomic: 580 / Related Interrogated SNP: hCV3230096 / Related Interrogated SNP: hCV11786258 / Related Interrogated SNP: hCV15968043 / Related Interrogated SNP: hCV27902808 / Related Interrogated SNP: hCV3230113 / SNP Source: dbSNP; HGBASE / Population(Allele,Count): Caucasian (A,82|G,38) / SNP Type: INTRON /
Context                (SEQ ID NO: 914): /
ATTTAGTAACCCAGTAATATCTCACTTAGTTTAGGGTTAGATCTTTAGTTAATTCAACCTTATAGATCATACTTATGAAGGTG
ATAACTGACACGTGTTC
M

CTGAATTTTAATTTGATAGGCAATACATCTACCCACTCCATTATTTTTTAAAACTTCATTTAATAGTTTAAACAAGATTGGTT
TTGTTTTCAATTTTTAT / Celera SNP ID: hCV29053261 / Public SNP ID: rs6842047 / SNP Chromosome Position: 187133576 / SNP in Genomic Sequence: SEQ ID NO: 352 / SNP Position Genomic: 5096 / Related Interrogated SNP: hCV15793897 / Related Interrogated SNP: hCV12066124 / Related Interrogated SNP: hCV25474413 / Related Interrogated SNP: hCV27474895 / Related Interrogated SNP: hCV3230113 / Related Interrogated SNP: hCV8241630 / Related Interrogated SNP: hCV25990131 / Related Interrogated SNP: hCV27477533 / Related Interrogated SNP: hCV3230038 / SNP Source: dbSNP; HapMap; / Population(Allele,Count): Caucasian (A,20|C,206) / SNP Type: MICRORNA;UTR3;INTRON /
Context                (SEQ ID NO: 915): /

TCTTTAAAGTAGTAGAAGCTAGTCATTTTCTATTCAAAGCCTCAAAATGCTTGAATATCATTGGGCTAAGGGATTGTCTCAAG
AAAGAGTCTAACAGGTG
M
ACATTTCATCTGAATAAAGAAACAGATTTAACTGTGTGACCCATGATCACATTAGCGGATAGCACAGTCCAAAGAAAATAACA
TAAGACAAGCATTTTGC / Celera SNP ID: hCV29053265 / Public SNP ID: rs4253244 /
SNP Chromosome Position: 187153775 / SNP in Genomic Sequence: SEQ ID NO: 352 /
SNP Position Genomic: 15103 / Related Interrogated SNP: hCV11786258 /
Related Interrogated SNP: hCV12066124 / Related Interrogated SNP: hCV15968043
/ Related Interrogated SNP: hCV3230113 / Related Interrogated SNP:
hCV22272267 / Related Interrogated SNP: hCV27902808 / Related Interrogated SNP:
hCV25474413 / Related Interrogated SNP: hCV25990131 / Related Interrogated
SNP: hCV3230096 / Related Interrogated SNP: hCV32291301 / Related
Interrogated SNP: hCV27477533 / Related Interrogated SNP: hCV8241630 / SNP
Source: dbSNP; HGBASE / Population(Allele,Count): Caucasian (A,75|C,45) / SNP
Type: INTRON /
Context (SEQ ID NO: 916): /
GAGGATGATAATTTCTTCCTGGGTCTGTTGTAATAATTAATACATCAAAGCACTTCATGTCTGGAACAGTGAAGATACCCTGC
TATGACTATTAAGGATA
R
TATACATGGAATAAGACACACAGGAACTTCTAAATGCTTTTGACCATAGATTTAGGTTCTGAGTTTTAAGAATTTAACTCAGGAA
ATTGTAACACCAAAAAT / Celera SNP ID: hCV32291217 / Public SNP ID: rs4253323 /
SNP Chromosome Position: 187176859 / SNP in Genomic Sequence: SEQ ID NO: 352 /
SNP Position Genomic: 38187 / Related Interrogated SNP: hCV25990131 /
Related Interrogated SNP: hCV32291301 / Related Interrogated SNP: hCV22272267
/ Related Interrogated SNP: hCV3230113 / Related Interrogated SNP:
hCV27902808 / Related Interrogated SNP: hCV11786258 / Related Interrogated SNP:
hCV3230096 / Related Interrogated SNP: hCV12066124 / Related Interrogated
SNP: hCV15968043 / Related Interrogated SNP: hCV25474413 / SNP Source:
dbSNP; HGBASE / Population(Allele,Count): Caucasian (A,22|G,98) / SNP Type:
INTRON /
Context (SEQ ID NO: 917): /
AGAATCATACATACACTTTGGAAAAGATGGGGCTTAATCGCACTTTATAAGACTTGAAGGATGTTTGAGAATCACTATGAAAC
TGCTGAAAATACCAAGA
R
AATTTAATTCTTATGTATATAAATAATGTGTCTGTTTTACATGAATCCCTTCTACAAGCTTGGTATTTAATATGGCATATATT
GTTTTTTTCATAGTAGAT / Celera SNP ID: hCV32291295 / Public SNP ID: rs4253292 /
SNP Chromosome Position: 187168866 / SNP in Genomic Sequence: SEQ ID NO: 352 /
SNP Position Genomic: 30194 / Related Interrogated SNP: hCV25990131 /
Related Interrogated SNP: hCV32291301 / Related Interrogated SNP: hCV12066124
/ Related Interrogated SNP: hCV3230113 / Related Interrogated SNP:
hCV27902808 / Related Interrogated SNP: hCV22272267 / Related Interrogated SNP:
hCV15968043 / Related Interrogated SNP: hCV25474413 / Related Interrogated
SNP: hCV3230096 / Related Interrogated SNP: hCV11786258 / Related
Interrogated SNP: hCV27477533 / Related Interrogated SNP: hCV8241630 / SNP
Source: dbSNP; Celera; HapMap; HGBASE / Population(Allele,Count): Caucasian
(A,178|G,48) / SNP Type: INTRON /
Context (SEQ ID NO: 918): /
ATTCTTTGGGAAAATCTCTGTTTAAATCTTTTGGCCATTAAAAATAATTGGGTTATTTTCATTGTTGAGTTGTATGAACTCTT
TATATACTCTGGATACT
R
CACTCTTATGACATATATTATTTTCAAAAATTTTCTGTGCATCTGCAGGTCATCTTTTCACTTTACTGATGGTGTTCTTTGAA
GCACCAAAGTTTTTAAA / Celera SNP ID: hCV29877725 / Public SNP ID: rs4253295 /
SNP Chromosome Position: 187170222 / SNP in Genomic Sequence: SEQ ID NO: 352 /
SNP Position Genomic: 31550 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV3230038 / Related Interrogated SNP:
hCV3230113 / Related Interrogated SNP: hCV8241630 / Related Interrogated SNP:
hCV3230096 / Related Interrogated SNP: hCV27474895 / Related Interrogated SNP:
hCV25474413 / Related Interrogated SNP: hCV25990131 / Related Interrogated
SNP: hCV27902808 / SNP Source: dbSNP / Population(Allele,Count): Caucasian
(A,143|G,83) / SNP Type: INTRON /
Context (SEQ ID NO: 919): /

TGACTGGGTGATAAAATGCATATTGAGAATAAGAGGCCTTCTGACCCTTCTGGGTCTAGGGCTGTAAAGCGTCTCAGGGTTGC
TGCCAAACGGGCCATGA
M
CTGGGCTGGGTTTTTCATATTTGATGAAAAAGAGCCTAAACGCTAACTGATTTGGGAGAGGTCGGATAAATAAAAAGGAACAT
TAATCTTGACTATGCCT / Celera SNP ID: hDV68550952 / Public SNP ID: rs4253289 /
SNP Chromosome Position: 187167103 / SNP in Genomic Sequence: SEQ ID NO: 352 /
SNP Position Genomic: 28431 / Related Interrogated SNP: hCV27474895 /
Related Interrogated SNP: hCV27477533 / Related Interrogated SNP: hCV3230038 /
Related Interrogated SNP: hCV8241630 / Related Interrogated SNP: hCV22272267
/ SNP Source: CDX; dbSNP / Population(Allele,Count): Caucasian (A,98|C,10) /
SNP Type: INTRON /
Context (SEQ ID NO: 920): /
GAAAAGGGTGCTTAAATAGAAGTTAACACTATTTTAAAGCAAGAATGGAAGTGGTTTCATCATGCGTAAACAACAACTCTCCA
CATTTTGTAATGATTGA
K
CTGGATGCAATTTGTCATCAGACAGGAGAAGTCGAAAGCAAAGAAATAACACTGGGAGATAGAGAAGCTCTTTCATTCAATGC
GAAAGGTCAAAGGCACA / Celera SNP ID: hDV71222711 / Public SNP ID: rs4253252 /
SNP Chromosome Position: 187157458 / SNP in Genomic Sequence: SEQ ID NO: 352 /
SNP Position Genomic: 18786 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV27902808 / Related Interrogated SNP:
hCV3230096 / Related Interrogated SNP: hCV8241630 / Related Interrogated SNP:
hCV3230113 / Related Interrogated SNP: hCV25474413 / Related Interrogated
SNP: hCV27474895 / Related Interrogated SNP: hCV3230038 / Related
Interrogated SNP: hCV25990131 / Related Interrogated SNP: hCV32291301 / SNP
Source: dbSNP; HapMap / Population(Allele,Count): Caucasian (T,113|G,113) /
SNP Type: INTRON /
Context (SEQ ID NO: 921): /
TTATTTTTATTTGCATAGATGTATGGGGTACAAGTGTAATTTTGTTACTTTGATGTATTTTACAGTGGTAAAGTCTGGGCTTT
TGGTATATCCATCACTG
S
AGTCATGTACATTGTACCCACTAAGCTATTTTTCAACGCCCGCCCCCTTCCCACCTCTCTGTCACCTTCCCAGTCTCTACTGT
CTATCGCTCCATGCTCT / Celera SNP ID: hCV32358975 / Public SNP ID: rs4253255 /
SNP Chromosome Position: 187158922 / SNP in Genomic Sequence: SEQ ID NO: 352 /
SNP Position Genomic: 20250 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV27902808 / Related Interrogated SNP:
hCV3230113 / Related Interrogated SNP: hCV8241630 / Related Interrogated SNP:
hCV3230096 / Related Interrogated SNP: hCV25474413 / Related Interrogated
SNP: hCV3230038 / Related Interrogated SNP: hCV27474895 / Related
Interrogated SNP: hCV25990131 / SNP Source: dbSNP; HGBASE /
Population(Allele,Count): Caucasian (C,61|G,59) / SNP Type: TRANSCRIPTION
FACTOR BINDING SITE;INTRON /
Context (SEQ ID NO: 922): /
AATGAGGGTTTAGTGTCACGGAGTCTCCTGCCTCATTCTCACCCTAACTTTTCCTTTACCCCTTTGCGAGGGGAAGGATGTCC
ATTAGGTTAATAATGCA
S
ACCCCTAACCCACTCATTATCAGGGTCATTGTTTTTCCACTGTGCATTTTAATACTAACTGTTACCTGCACTGCTCCCTGCCC
CTCAAAGTGCAGAAAGC / Celera SNP ID: hCV32358984 / Public SNP ID: rs4253256 /
SNP Chromosome Position: 187159282 / SNP in Genomic Sequence: SEQ ID NO: 352 /
SNP Position Genomic: 20610 / Related Interrogated SNP: hCV11786258 /
Related Interrogated SNP: hCV12066124 / Related Interrogated SNP: hCV15968043
/ Related Interrogated SNP: hCV3230113 / Related Interrogated SNP:
hCV22272267 / Related Interrogated SNP: hCV27902808 / Related Interrogated SNP:
hCV25474413 / Related Interrogated SNP: hCV3230096 / Related Interrogated
SNP: hCV25990131 / Related Interrogated SNP: hCV27477533 / Related
Interrogated SNP: hCV8241630 / Related Interrogated SNP: hCV32291301 /
Related Interrogated SNP: hCV3230038 / SNP Source: dbSNP; HGBASE /
Population(Allele,Count): Caucasian (C,74|G,46) / SNP Type: INTRON /
Context (SEQ ID NO: 923): /
GTCTTTATAAATCAACACATACTTAATGAGCCTCTATTATTTATGAGGTTAGCGCTCAAGTGTAAGATTTGCAGAAAATGAAT

CCAAATAATTGTGTCTC
R
TTTCCAGATAAGAATTTTTAAGAAAACACAAGGGAACATCTCTCTCAAGTTCACTTGAGGGTAATTTTTTACATCAGTGATTCT
CAACCAGCAGTGATTTT / Celera SNP ID: hCV32313006 / Public SNP ID: rs4253248 /
SNP Chromosome Position: 187155488 / SNP in Genomic Sequence: SEQ ID NO: 352 /
SNP Position Genomic: 16816 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV27902808 / Related Interrogated SNP:
hCV3230096 / Related Interrogated SNP: hCV8241630 / Related Interrogated SNP:
hCV3230113 / Related Interrogated SNP: hCV25474413 / Related Interrogated
SNP: hCV27474895 / Related Interrogated SNP: hCV3230038 / Related
Interrogated SNP: hCV25990131 / Related Interrogated SNP: hCV32291301 / SNP
Source: dbSNP; Celera; HGBASE / Population(Allele,Count): Caucasian
(G,112|A,114) / SNP Type: INTRON /
Context (SEQ ID NO: 924): /
TCCAGCAAGTTCAATCAATGACATGGAGAAAAGGTAAAAGTTGGTATTTCATTATTGGAGAAGCTGTTTTTCAAAACTGAATC
AGTTTTGTGCAGAAAGG
Y
GTAGTATAACTGAGAGTTCTTCCTCACACGGGGTTCAAGGACCAGCTTCAGCAAAATCCCGTCAAGTGGTTCTTACAAATGCA
GATTCCTAGGCCACAAC / Celera SNP ID: hCV32313014 / Public SNP ID: rs4253243 /
SNP Chromosome Position: 187153511 / SNP in Genomic Sequence: SEQ ID NO: 352 /
SNP Position Genomic: 14839 / Related Interrogated SNP: hCV27477533 /
Related Interrogated SNP: hCV8241630 / Related Interrogated SNP: hCV3230038 /
Related Interrogated SNP: hCV27474895 / SNP Source: dbSNP; HapMap; HGBASE /
Population(Allele,Count): Caucasian (T,210|C,16) / SNP Type: INTRON /
Context (SEQ ID NO: 925): /
CCTAATGTGCTGGGATTACAGGCATGAGCCACCGCACCCAGCGGTAATTACAATTTTTATTAGGTCAGAGAGATGCTTATTAA
TCACGAGCCACAGTTTC
W
TCTTAATGATTTTTTCCTTTTGATTAATATCCAGAGTAAGCTTTTCTTTGTTGTTCCCATTTCCATGTTCATAACTCTTTACTC
ATCTTCACTCTATGTGA / Celera SNP ID: hCV30983907 / Public SNP ID: rs4253246 /
SNP Chromosome Position: 187154424 / SNP in Genomic Sequence: SEQ ID NO: 352 /
SNP Position Genomic: 15752 / Related Interrogated SNP: hCV12066124 /
Related Interrogated SNP: hCV22272267 / Related Interrogated SNP: hCV25474413
/ Related Interrogated SNP: hCV3230113 / SNP Source: dbSNP; HapMap; HGBASE /
Population(Allele,Count): Caucasian (A,108|T,12) / SNP Type: INTRON /
Context (SEQ ID NO: 926): /
GCTGCCCAGGCTTGAAGGTGAGATGACACCGGTAATGTGAAATTGTCCTTCCTATACACTTCTATGTGTCTTTTCTTATTTCT
GTGCTGCAACCAGGTGG
Y
ATAACCTCTCACCTGATTCCTTAGCTCTAGTGAAGTTATTTTCGTGCATGGATACTTGTTCGAATTGATGTTTCTGCAAGGGA
TGAGCGCTAGAAACTCC / Celera SNP ID: hCV29718000 / Public SNP ID: rs4253238 /
SNP Chromosome Position: 187148387 / SNP in Genomic Sequence: SEQ ID NO: 352 /
SNP Position Genomic: 9715 / Related Interrogated SNP: hCV11786258 / Related
Interrogated SNP: hCV12066124 / Related Interrogated SNP: hCV15968043 /
Related Interrogated SNP: hCV22272267 / Related Interrogated SNP: hCV27477533
/ Related Interrogated SNP: hCV27902808 / Related Interrogated SNP:
hCV3230096 / Related Interrogated SNP: hCV8241630 / Related Interrogated SNP:
hCV3230113 / Related Interrogated SNP: hCV25474413 / Related Interrogated SNP:
hCV27474895 / Related Interrogated SNP: hCV3230038 / Related Interrogated SNP:
hCV25990131 / Related Interrogated SNP: hCV32291301 / SNP Source: dbSNP;
HapMap; ABI_Val; HGBASE / Population(Allele,Count): Caucasian (C,112|T,114) /
SNP Type: UTR5;INTRON /
Context (SEQ ID NO: 927): /
GCAACCAGGTGGCATAACCTCTCACCTGATTCCTTAGCTCTAGTGAAGTTATTTTCGTGCATGGATACTTGTTCGAATTGATG
TTTCTGCAAGGGATGAG
Y
GCTAGAAACTCCTGTTCTGACAAACTCCTATTCCTATTCTTGCTGACATCACTCCCTGAAATAGTTAATATACTTAACAGCTG
AACACGGATAGGATGTT / Celera SNP ID: hCV32313024 / Public SNP ID: rs4253239 /
SNP Chromosome Position: 187148475 / SNP in Genomic Sequence: SEQ ID NO: 352 /
SNP Position Genomic: 9803 / Related Interrogated SNP: hCV25990131 / Related
Interrogated SNP: hCV32291301 / Related Interrogated SNP: hCV12066124 /

Related Interrogated SNP: hCV3230113 / Related Interrogated SNP: hCV27902808 / Related Interrogated SNP: hCV22272267 / Related Interrogated SNP: hCV15968043 / Related Interrogated SNP: hCV25474413 / Related Interrogated SNP: hCV3230096 / Related Interrogated SNP: hCV11786258 / Related Interrogated SNP: hCV27477533 / Related Interrogated SNP: hCV8241630 / SNP Source: dbSNP; Celera; HapMap; HGBASE / Population(Allele,Count): Caucasian (C,178|T,48) / SNP Type: UTR5;INTRON /
Context (SEQ ID NO: 928): /
ATAAACAATTGACCCTGAATCAGGGGTTCCACATCCATGGATTTAAACAACATTGAAGTGAAATTTTTTAAAAAAAATTATAT
CTGCACTCAACATGTGC
R

GAGTTTTTTCTTGTCATTATTCCCGAAACAACACAGTATAACAACAATTTGCATAGTATTTACATTGTACTAGGTATTATAAG
TAATCTAGAGATGATTT / Celera SNP ID: hCV32291246 / Public SNP ID: rs4253403 / SNP Chromosome Position: 187189326 / SNP in Genomic Sequence: SEQ ID NO: 352 / SNP Position Genomic: 50654 / Related Interrogated SNP: hCV15793897 / SNP Source: dbSNP; HapMap; HGBASE / Population(Allele,Count): Caucasian (A,112|G,2) / SNP Type: INTRON /
Context (SEQ ID NO: 929): /
CACTCTCTCTCTCGTATCACCTTGTGAAGAAGGTGTCTGCTTCCCCTCTGCCTTCCATCGTGATTATAAGTTTCCTGAGGCCT
CCCCAGCCATGGGTAAC
Y

GTGAGTCAGTTAAACCTCTTTTGTTTATAAATTACCCAGTCTCAGGTAGTATCTTTACAGCAGTGTGAAACTGGACTAATACA
TCTGGGAAATTCTTTTT / Celera SNP ID: hCV29826351 / Public SNP ID: rs10025990 / SNP Chromosome Position: 187146258 / SNP in Genomic Sequence: SEQ ID NO: 352 / SNP Position Genomic: 7586 / Related Interrogated SNP: hCV12066124 / Related Interrogated SNP: hCV15793897 / Related Interrogated SNP: hCV25474413 / Related Interrogated SNP: hCV3230038 / Related Interrogated SNP: hCV27474895 / Related Interrogated SNP: hCV27477533 / Related Interrogated SNP: hCV8241630 / Related Interrogated SNP: hCV22272267 / Related Interrogated SNP: hCV27902808 / Related Interrogated SNP: hCV15968043 / Related Interrogated SNP: hCV30562347 / Related Interrogated SNP: hCV25990131 / SNP Source: dbSNP; HapMap / Population(Allele,Count): Caucasian (T,13|C,101) / SNP Type: INTRON /
Context (SEQ ID NO: 930): /
TCAGTTTACTTTCAGATTCCATATATAGTTTTAAAATACATGTTTTAGCTGAATAGTAAAAAAAAAAAAAAAATATGTAGGCAC
AGGAGTATCTATTGGGC
W

CTCCTGGGAAGGAGAGTAGCCTTTCTTTCTCTTTCTTTCTCTTTCTTTCTCTTTCTTTCCTTCTTTCTTTCCTTCCTTCCTTC
CTTCCTTCTTTCCTTGC / Celera SNP ID: hCV30983902 / Public SNP ID: rs4862668 / SNP Chromosome Position: 187136443 / SNP in Genomic Sequence: SEQ ID NO: 352 / SNP Position Genomic: 2229 / Related Interrogated SNP: hCV12066124 / Related Interrogated SNP: hCV15793897 / Related Interrogated SNP: hCV25474413 / Related Interrogated SNP: hCV27477533 / Related Interrogated SNP: hCV3230038 / Related Interrogated SNP: hCV8241630 / Related Interrogated SNP: hCV25990131 / Related Interrogated SNP: hCV3230113 / Related Interrogated SNP: hCV27474895 / SNP Source: dbSNP; HGBASE / Population(Allele,Count): Caucasian (A,12|T,108) / SNP Type: INTRON /
Context (SEQ ID NO: 931): /
TGAAACTCAGATTCCTATATAAAAATGTTGGGATAACTTTGTTCCTTCAGTAAGGGTCATACAGACATTATAGATCTTATCGG
GAAGAAAAATTATCACA
R

TATTTAGACATGACTGTCTTATTTTAAAAATGTTTTCAGTTTACTTTCAGATTCCATATATAGTTTTAAAATACATGTTTTAG
CTGAATAGTAAAAAAAA / Celera SNP ID: hCV32313007 / Public SNP ID: rs4862666 / SNP Chromosome Position: 187136307 / SNP in Genomic Sequence: SEQ ID NO: 352 / SNP Position Genomic: 2365 / Related Interrogated SNP: hCV15793897 / Related Interrogated SNP: hCV12066124 / Related Interrogated SNP: hCV27474895 / Related Interrogated SNP: hCV25474413 / Related Interrogated SNP: hCV3230113 / Related Interrogated SNP: hCV8241630 / Related Interrogated SNP: hCV27477533 / Related Interrogated SNP: hCV25990131 / Related Interrogated SNP: hCV3230038 / SNP Source: dbSNP; HGBASE / Population(Allele,Count): Caucasian (A,21|G,205) / SNP Type: INTRON /
Context (SEQ ID NO: 932): /
AACTCACTGCAATTTAACAAGAAAGCAAGTACACAGATGAGCCATCCAGGGTGCTCTCCCAGGTGTGGACAGCAGCTGTGACA

GCCCAGAGCCACAGCAC
S
AGCTTGCCTGTTCCCTGGGTAAGTGCAGCAAATCCTTCCAGCAACAGGGCTGGGTACAGAATGGAGCATGTTTGCATCGAGTC
CTGCCTCCTGGCAGCCT / Celera SNP ID: hCV32295028 / Public SNP ID: rs4253260 /
SNP Chromosome Position: 187162077 / SNP in Genomic Sequence: SEQ ID NO: 352 /
SNP Position Genomic: 23405 / Related Interrogated SNP: hCV25990131 /
Related Interrogated SNP: hCV32291301 / Related Interrogated SNP: hCV22272267
/ Related Interrogated SNP: hCV3230113 / Related Interrogated SNP:
hCV27902808 / Related Interrogated SNP: hCV11786258 / Related Interrogated SNP:
hCV3230096 / Related Interrogated SNP: hCV12066124 / Related Interrogated
SNP: hCV15968043 / Related Interrogated SNP: hCV25474413 / SNP Source:
dbSNP; HGBASE / Population(Allele,Count): Caucasian (C,98|G,22) / SNP Type:
INTRON //

Gene Number: 16 / Gene Symbol: PIK3R1 - 5295 / Gene Name:
phosphoinositide-3-kinase, regulatory subunit 1 (alpha) / Chromosome: 5 / OMIM
NUMBER: 171833 / OMIM Information: // Genomic Sequence (SEQ ID NO: 353): // /
SNP Information /
Context (SEQ ID NO: 933): /
GCCTTATTTTTGCATCTCACAAACATAAGTGCAATAGATCTTTTCATTGAACAGCAAAGTAGGATTCATCATTCCATATGACT
TGAGTTACACCAGACCT
R
TTCTGCCCAATGCCTTTTTGATTACAGTGTAGCTTGCCCACCGCATTTGTCGTTTTAGATACTTTGCTAGCCGGCCACTTTGG
ATTTCATCAGACAGTCC / Celera SNP ID: hCV27474984 / Public SNP ID: rs3756668 /
SNP Chromosome Position: 67596088 / SNP in Genomic Sequence: SEQ ID NO: 353 /
SNP Position Genomic: 83626 / SNP Source: dbSNP; HapMap; ABI_Val; HGBASE /
Population(Allele,Count): Caucasian (G,128|A,96) / SNP Type: MISSENSE
MUTATION;UTR3;ESE SYNONYMOUS /
Context (SEQ ID NO: 934): /
GAAGCTACCACTTGTTGCATGCTTACTGCATGTCAGGCATTCTATCTAGCATTTTGCATGCATGATCTCATTTCATTTCTCAA
AGTAACTGTATTACATG
Y
ATTTTACTATTGTCTTCCCTTTAAACCATTTTAAAGATGAAAATACAGTCAGTCAGTGAAGCTGGGTTACCTACCCAAGGCAC
TCGGCCAGGTGTCAGTT / Celera SNP ID: hCV1988068 / Public SNP ID: rs1445760 /
SNP Chromosome Position: 67593016 / SNP in Genomic Sequence: SEQ ID NO: 353 /
SNP Position Genomic: 80554 / Related Interrogated SNP: hCV27474984 / SNP
Source: Applera / Population(Allele,Count): Caucasian (C,16|T,22) African
American (C,31|T,5) total (C,47|T,27) / SNP Type: UTR5;INTRON / SNP Source:
dbSNP; Celera; HapMap; ABI_Val; HGBASE / Population(Allele,Count): Caucasian
(T,55|C,65) / SNP Type: UTR5;INTRON /
Context (SEQ ID NO: 935): /
AGATCACGACTTTAGATCACCTGCTTACTGAAATAGTACTTCTAGCATTTTAACTAAAAACCATTACCATTAATGAGTGATAT
GTTACTTTCTAATTTAT
K
TTCATAATGTAGGCCATAGGAAAAAGAAAACAGATTGATTTGCAGTTTAGGAGGCCCAGTCTGCTCTTTTTATCTGTCTTTAA
CCAGCTGTGGGAAGTGG / Celera SNP ID: hCV3164046 / Public SNP ID: rs9291926 /
SNP Chromosome Position: 67599656 / SNP in Genomic Sequence: SEQ ID NO: 353 /
SNP Position Genomic: 87194 / Related Interrogated SNP: hCV27474984 / SNP
Source: dbSNP; Celera; HapMap; ABI_Val / Population(Allele,Count): Caucasian
(T,58|G,62) / SNP Type: INTERGENIC;UNKNOWN //

Gene Number: 17 / Gene Symbol: PLRG1 - 5356 / Gene Name: pleiotropic
regulator 1 (PRL1 homolog, Arabidopsis) / Chromosome: 4 / OMIM NUMBER: 605961
/ OMIM Information: // Genomic Sequence (SEQ ID NO: 354): // / SNP Information
/
Context (SEQ ID NO: 936): /
TCAGCTTAAAGGGAAAAATCTAGTTACACCTGGTATCCCCTCAGCAGTACAGCTTGACAAACATTTATGAAGCATCATTAGCC
CAAGCATGGGAATACTA
Y
GGTTATAAAAATACATAAGACACAGGCACTCCACCTTCTCTTAATATTGGTTTGTCTACTTTCATTTGGGCTGGGTTCAGAAC
TTATAGATCTTAAAAAG / Celera SNP ID: hCV22273499 / Public SNP ID: rs7668014 /
SNP Chromosome Position: 155458728 / SNP in Genomic Sequence: SEQ ID NO: 354 /
SNP Position Genomic: 61676 / Related Interrogated SNP: hCV15860433 /

Related Interrogated SNP: hCV11503469 / Related Interrogated SNP: hCV11503414 / SNP Source: Applera / Population(Allele,Count): Caucasian (C,27|T,7) African American (C,29|T,7) total (C,56|T,14) / SNP Type: INTRON / SNP Source: dbSNP; HapMap / Population(Allele,Count): Caucasian (C,151|T,75) / SNP Type: INTRON /
Context (SEQ ID NO: 937): /
ATACGTTAATTTACTACCAACTTGAGTTTTAGGGCTATGAAATATGAATGACTAGCTCAGGCTGCTATTTATCTTAATAATTC
CAAAGAAGAGGGAAATA
Y
GGCAACATGTTCCAAAAGTTAGAAACCTGCTATTTGATGCAGAAAAACTTACTGAATTCATCGCTGTAGGCTGTGGACGGTCA
GAAGCCCCAGGATGTCG / Celera SNP ID: hCV22274180 / Public SNP ID: rs11935584 /
SNP Chromosome Position: 155466935 / SNP in Genomic Sequence: SEQ ID NO: 354 /
SNP Position Genomic: 69883 / Related Interrogated SNP: hCV11503414 /
Related Interrogated SNP: hCV11503469 / Related Interrogated SNP: hCV15860433
/ SNP Source: Applera / Population(Allele,Count): Caucasian (C,6|T,28) African American (C,6|T,22) total (C,12|T,50) / SNP Type: INTRON / SNP Source: dbSNP; HapMap / Population(Allele,Count): Caucasian (T,85|C,35) / SNP Type: INTRON /
Context (SEQ ID NO: 938): /
ATATTTATTATAAAAATCTTATTTTACCTCCAGATACAAGCACTCCATCAGAATTTACCGTCAATGTGTTAATAATAGCATTA
TGACCGGAAAGATTTTG
W
ATGAAACTTCCATCAGGGAATTTCCACTGCTTTATGTTATCTGGAGAACCAGATGCAAATGTGTAACTGAAATAATATTAAAA
AGAATTAACGACAAAGT / Celera SNP ID: hCV25610762 / Public SNP ID: rs7668818 /
SNP Chromosome Position: 155459194 / SNP in Genomic Sequence: SEQ ID NO: 354 /
SNP Position Genomic: 62142 / Related Interrogated SNP: hCV15860433 /
Related Interrogated SNP: hCV11503414 / Related Interrogated SNP: hCV11503469
/ SNP Source: Applera / Population(Allele,Count): Caucasian (A,30|T,8) African American (A,31|T,7) total (A,61|T,15) / SNP Type: ESS;TRANSCRIPTION FACTOR BINDING SITE;SILENT RARE CODON;SILENT MUTATIO / N;PSEUDOGENE // SNP Source: dbSNP; HapMap / Population(Allele,Count): Caucasian (A,84|T,14) / SNP Type: ESS;TRANSCRIPTION FACTOR BINDING SITE;SILENT RARE CODON;SILENT MUTATIO / N;PSEUDOGENE //
Context (SEQ ID NO: 939): /
GAAATAATATTTTCAAAGTAATTAATGCCTCCAGTCATGTATTCTGCAAAACTAACCCTTTATTTTAAATTGTTATAAAGTCC
TGATAAAGGAAAATGTC
R
TATAATAGTTATAAAGAAATTTTAAAAAGAAAAAATGTGAACAGTGAAATATGCTTTTAAGATCTTTGTAATTATAATATTTC
ATACTTGGTCTGCAAAG / Celera SNP ID: hCV2892896 / Public SNP ID: rs11940724 /
SNP Chromosome Position: 155467187 / SNP in Genomic Sequence: SEQ ID NO: 354 /
SNP Position Genomic: 70135 / Related Interrogated SNP: hCV15860433 /
Related Interrogated SNP: hCV11503469 / Related Interrogated SNP: hCV11503414
/ SNP Source: Applera / Population(Allele,Count): Caucasian (A,26|G,8) African American (A,25|G,11) total (A,51|G,19) / SNP Type: TRANSCRIPTION FACTOR BINDING SITE;UTR3;INTRON / SNP Source: Applera / Population(Allele,Count): Caucasian (A,31|G,9) African American (A,26|G,12) total (A,57|G,21) / SNP Type: TRANSCRIPTION FACTOR BINDING SITE;UTR3;INTRON / SNP Source: dbSNP; Celera; HapMap / Population(Allele,Count): Caucasian (A,151|G,75) / SNP Type: TRANSCRIPTION FACTOR BINDING SITE;UTR3;INTRON /
Context (SEQ ID NO: 940): /
TGGCTTCCTGTAATGGAAACACATATATCCCTCAAAACATAGACCAGGAAAAATAATGCAAAAGTTAAATAAAATACAAAGCA
TTCTTCCAAAACACATA
Y
GTTCTAAATGAAAGTCATATCACACGCAAATTAGTCTTGGCTAATTTCTTCATAGTTCCAAATTTTTAGGTTGATTTTAATTC
TTTAAAAAAATGCAATA / Celera SNP ID: hCV2892905 / Public SNP ID: rs12642770 /
SNP Chromosome Position: 155460459 / SNP in Genomic Sequence: SEQ ID NO: 354 /
SNP Position Genomic: 63407 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: Applera / Population(Allele,Count): Caucasian
(C,12|T,28) African American (C,6|T,28) total (C,18|T,56) / SNP Type: INTRON
/ SNP Source: dbSNP; Celera; HapMap / Population(Allele,Count): Caucasian
(T,171|C,55) / SNP Type: INTRON /

Context (SEQ ID NO: 941): /
GCCCCTCCCCATCCCATACCTCATCCCCAGCATCTACCCCCAGAAAGAGGCAATATTGAACCCCCAAGTTTGTTTTGAGTATT
CAAAAGTATGGAAAACC
Y

GTGCCCCAATTCCTGTATAGCGCTCTAGGAGAGCGGTTTCAGAGTGGACAGTGTGATACCATAAACAGTAGAGAAAAATAGCT
GCTTCTCAGATTTCAGA / Celera SNP ID: hCV21680 / Public SNP ID: rs7666020 / SNP
Chromosome Position: 155433560 / SNP in Genomic Sequence: SEQ ID NO: 354 /
SNP Position Genomic: 36508 / Related Interrogated SNP: hCV11503414 / Related
Interrogated SNP: hCV11503469 / Related Interrogated SNP: hCV15860433 /
Related Interrogated SNP: hCV2892877 / Related Interrogated SNP: hCV11503470 /
SNP Source: dbSNP; Celera / Population(Allele,Count): Caucasian (C,143|T,83)
/ SNP Type: INTERGENIC;UNKNOWN /
Context (SEQ ID NO: 942): /
ACAGTAGAGAAAAATAGCTGCTTCTCAGATTTCAGACATAAGGAAGAACATGCTTGGATGTCTAGGTGTGTGTGTGAAGGATG
AGGGCCCCAGAAATATT
W

TCGTAAGTTTAAATTCTGACCTGACTGTATTTCAAACCAGAAGCGACTGAGTTTTGTTGAACTGGCAAGGTTGTTTTTCTACC
ACTGGGCCAAAATAGGG / Celera SNP ID: hCV21681 / Public SNP ID: rs6536018 / SNP
Chromosome Position: 155433725 / SNP in Genomic Sequence: SEQ ID NO: 354 /
SNP Position Genomic: 36673 / Related Interrogated SNP: hCV11503414 / Related
Interrogated SNP: hCV11503469 / Related Interrogated SNP: hCV2892877 /
Related Interrogated SNP: hCV11503470 / Related Interrogated SNP: hCV15860433
/ SNP Source: dbSNP; Celera; HapMap; ABI_Val / Population(Allele,Count):
Caucasian (T,51|A,69) / SNP Type: INTERGENIC;UNKNOWN /
Context (SEQ ID NO: 943): /
GGTTTCTAACTTCCAGACAAAGATGCCATTATACTGCAATGACTTCTCAGAGAATGTGAACAAAACTCCTTTAAATGTTCGAC
TAGCTCTTGTAATCACC
R

TCAACAACATTTAAACCTTGGGTAGGGAGTAGTCAGCATGAAATGACATTTCCTCTCTAACTCACCTAATACGATGCCTGGCA
CACAGTAGGTGCTCATT / Celera SNP ID: hCV24834 / Public SNP ID: rs4235247 / SNP
Chromosome Position: 155438339 / SNP in Genomic Sequence: SEQ ID NO: 354 /
SNP Position Genomic: 41287 / Related Interrogated SNP: hCV11503414 / Related
Interrogated SNP: hCV11503469 / Related Interrogated SNP: hCV11503470 /
Related Interrogated SNP: hCV2892877 / Related Interrogated SNP: hCV15860433 /
SNP Source: dbSNP; Celera; HapMap; ABI_Val; HGBASE / Population(Allele,Count):
Caucasian (A,40|G,186) / SNP Type: INTERGENIC;UNKNOWN /
Context (SEQ ID NO: 944): /
GGAGAGGCCACCAGAGAGCCCAGTGACTAAGAGGCTTTTGCAAATCTTGAGGGGCTAGATCGCTGGAATTTCCAGAAATTCTA
GTCAGCACAATTTAATG
Y

TGTCTGCAGCAATACTTGGCAACCTGGAAAGGTTTGTTGCAATTGTTGGAGGTGCAGTCCTACATGTAAGCCAAGCTGAGCTC
ACCATAATGGATCTACC / Celera SNP ID: hCV176753 / Public SNP ID: rs2404478 /
SNP Chromosome Position: 155408038 / SNP in Genomic Sequence: SEQ ID NO: 354 /
SNP Position Genomic: 10986 / Related Interrogated SNP: hCV11503469 /
Related Interrogated SNP: hCV11503414 / SNP Source: dbSNP; Celera; HapMap;
ABI_Val; HGBASE / Population(Allele,Count): Caucasian (C,54|T,172) / SNP Type:
INTRON /
Context (SEQ ID NO: 945): /
CTTTCCTCTGTTGACTGCCAATTCCTTATTTCCCATAACTCTTTGTATCTCAGTGCAAAAAAAAAAAAAAAAAAAAAAAGGAGAG
AAAGAAAAATGGAGGAA
W

GATATTGCAGAAGCTTCTAGCCTCAAAGGATGCATTCAATTAATAAACAGTAATCTTTCAGGAAAGCAACCCTAAATCCCCTG
GTCAACAATGTCCATTC / Celera SNP ID: hCV229029 / Public SNP ID: rs13103792 /
SNP Chromosome Position: 155420340 / SNP in Genomic Sequence: SEQ ID NO: 354 /
SNP Position Genomic: 23288 / Related Interrogated SNP: hCV11503469 /
Related Interrogated SNP: hCV11503414 / SNP Source: dbSNP; Celera /
Population(Allele,Count): Caucasian (A,174|T,48) / SNP Type:
INTERGENIC;UNKNOWN /
Context (SEQ ID NO: 946): /
TGTATTGTAAACATTGCATGAATTCTAAATCCCAAAGACCAATGCACAGAACAATTTCATTTTGCTTTTCAAGATAGCAAAAA
GACTGAGAATAAGTCTC
R

CTCACCCATATGAATTTCCTATGCAGGCATTATTCCCGGGTTATCTAGAGTTGAATTGTTAATGCTATTTAATTTTAATAGCC

TTCTCAATTTTAAATCC / Celera SNP ID: hCV354895 / Public SNP ID: rs11737226 / SNP Chromosome Position: 155442246 / SNP in Genomic Sequence: SEQ ID NO: 354 / SNP Position Genomic: 45194 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera / Population(Allele,Count): Caucasian (G,73|A,153) / SNP Type: INTERGENIC;UNKNOWN / Context (SEQ ID NO: 947): / CAACTATGTTTTTAATGGTCATTAAATGAAGTCTCAAAAGTGAAGATATCCAATCAAGTCCAATCCCTGGAACAATGTAGAAA AGATGGACCTAAGAAAC Y

AAAATTGAAATGTCTTTTTCATGGATTTAGTGCAGATTCCCTTTAAGAAATTGATAAATTTCACTGTGGACTGGTTGCTAGGA TACCCCTCAAAATCTAT / Celera SNP ID: hCV354896 / Public SNP ID: rs7690972 / SNP Chromosome Position: 155442866 / SNP in Genomic Sequence: SEQ ID NO: 354 / SNP Position Genomic: 45814 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera / Population(Allele,Count): Caucasian (C,73|T,153) / SNP Type: INTERGENIC;UNKNOWN / Context (SEQ ID NO: 948): / GTGGGGCAGGGGGAGATATAGTTGGAGAGATCTACTGTTGGTAATATGTATACATGTATGGAAGAGGAAGGGTTAGTTTGGTG CATAGAAGAATTACAAC Y

GAACAGAGAAGGAACTAAAGCAAGGAGGGGGGTGATATACAAGAAGAGAAAAGAAAGAGCAAGTCATCTAGAAAAAAAATCTAG ATTAAAAAATAAATGCA / Celera SNP ID: hCV470979 / Public SNP ID: rs1490672 / SNP Chromosome Position: 155404360 / SNP in Genomic Sequence: SEQ ID NO: 354 / SNP Position Genomic: 7308 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera; HGBASE / Population(Allele,Count): Caucasian (C,170|T,56) / SNP Type: TFBS SYNONYMOUS;INTRON / Context (SEQ ID NO: 949): / CAACTGGAAGAACGGGCCTGCGGGGTCCTTGGGCAGGTCGGACGGTTGCACCAGGGTGTAGCCCTGAGTGCTGAACAGTCCGG CGTCCGGATCGTGGGCA M

CTGGCAGGCGGAAGGCGGTCCCTGGCGGGCTGAGCTCGGAGACGTCGAGTTGCAGGGAGTCGAGGGGAAAGCGGGGCGAGTGG TCATTCACGTCGTTGAC / Celera SNP ID: hCV1125378 / Public SNP ID: rs17373874 / SNP Chromosome Position: 155411939 / SNP in Genomic Sequence: SEQ ID NO: 354 / SNP Position Genomic: 14887 / SNP Source: dbSNP; Celera; HapMap / Population(Allele,Count): Caucasian (A,63|C,43) / SNP Type: MISSENSE MUTATION;TRANSCRIPTION FACTOR BINDING SITE;UTR5 / Context (SEQ ID NO: 950): / AAAGACAAGGCACATGGACAGGCAGTTCACAGAAAGACCACTGACATTGTGGCTGATATGTAAATTGAAAAGATTCTCAGTGT GCTCATGATGAAAGAAA W

TTTAATCACAATAACAATAAGATAACATATTTCACCTAATAGGTTGGCAAAAAATTTTAAAATTTGACAACAACAAGTGATGA CAAGGATGAGGGACAAC / Celera SNP ID: hCV2892893 / Public SNP ID: rs12648258 / SNP Chromosome Position: 155477372 / SNP in Genomic Sequence: SEQ ID NO: 354 / SNP Position Genomic: 80320 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera; HapMap / Population(Allele,Count): Caucasian (T,91|A,27) / SNP Type: INTERGENIC;UNKNOWN / Context (SEQ ID NO: 951): / TTCCAAGCAAATGGTTTCCTGTGGAATCTTGAATTCTCTTTACTTATTAAGATTTCAGTATTTAATTTCATCTTTCTGAATTA CAGACAAGATAGATGGT R

TCCTCACAGGTTAAGAATATTCTCCAATTTTTTCCTTTAATCTCTACTGTGACCTTCTCCACACTCTAAGAATCTCTGCCATC ATTATACTACCATTTTT / Celera SNP ID: hCV2892895 / Public SNP ID: rs12641958 / SNP Chromosome Position: 155468571 / SNP in Genomic Sequence: SEQ ID NO: 354 / SNP Position Genomic: 71519 / Related Interrogated SNP: hCV15860433 / Related Interrogated SNP: hCV11503469 / Related Interrogated SNP: hCV11503414 / SNP Source: dbSNP; Celera; HapMap; ABI_Val / Population(Allele,Count):

Caucasian (G,151|A,75) / SNP Type: INTRON /
Context (SEQ ID NO: 952): /
AGTTCCTTCTATTTATCTTCTAATACAATATTTAAATACTATTTTAACAGGTATACTGCCTGATATACCATCTGTTGTTAGCA
GTCATCTAGTCATCCAA
W
ACATAAATAAGCCCATAAAGTTCCTGCCCTCAAGAAGCTTAAATTATACCCTCATAAGGAGGGTAGAGAGCCTGGACAGAAGA
CGTGCTCTGAATGGAAT / Celera SNP ID: hCV2892899 / Public SNP ID: rs7680155 /
SNP Chromosome Position: 155466254 / SNP in Genomic Sequence: SEQ ID NO: 354 /
SNP Position Genomic: 69202 / Related Interrogated SNP: hCV11503414 /
Related Interrogated SNP: hCV11503469 / Related Interrogated SNP: hCV15860433
/ SNP Source: dbSNP; Celera; HapMap / Population(Allele,Count): Caucasian
(A,85|T,35) / SNP Type: INTRON /
Context (SEQ ID NO: 953): /
GAATATAAATAGGAGACAACTTGCAAGAATTCTAAAAAGCTAAAAGATCAGGGTACTGAAAATCTAAGAATGAGTATGATGGG
ACTACTTTTTAAGACTA
Y
TGAATGAAAGAACTAATGGACAATAGTTGTTACAGAATGGAACGTGAGAATATCACATTTCAGGGGCAGGGCACTAATAACAC
TAACATGGTGAGGCCAT / Celera SNP ID: hCV2892904 / Public SNP ID: rs7698829 /
SNP Chromosome Position: 155461467 / SNP in Genomic Sequence: SEQ ID NO: 354 /
SNP Position Genomic: 64415 / SNP Source: dbSNP; Celera; HapMap; ABI_Val /
Population(Allele,Count): Caucasian (C,32|T,194) / SNP Type: TRANSCRIPTION
FACTOR BINDING SITE;INTRON /
Context (SEQ ID NO: 954): /
AAACAAACTTTCTGAAAGTCCCACACAACATTTCTGATTAAATCTCACTGGCTAGGACTTGCCTTCATAGCCACACATAACTG
AATAAAAGTCTGAGAAA
W
GTAGCATTTTATGTGGATGCATCCCCTACACCTTCCCCACTGACCCCTGCCAAAAAATAAAAATAAAATAAAATAAAGTGAAA
GAGGAGAATGCTTATTG / Celera SNP ID: hCV2892918 / Public SNP ID: rs12511469 /
SNP Chromosome Position: 155451759 / SNP in Genomic Sequence: SEQ ID NO: 354 /
SNP Position Genomic: 54707 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; Celera; HapMap / Population(Allele,Count):
Caucasian (T,86|A,26) / SNP Type: INTERGENIC;UNKNOWN /
Context (SEQ ID NO: 955): /
CATTCTGACCCCAGTGAAAGTGGACTCTGGTACTTTCATTTAGTCACAGGCTCGCTCAGGGAGTCTTGTTTCTTATTCCTCAC
TAATAGTCCCAGTCATA
Y
AAGCACTTGAGATTATTTTGGCTCCATCCTACTTCCCTGTAGCTACTATGGCCTCCCACTCTCTCATGGACATTCATCGTATT
TTGCTCTTGCAACATTC / Celera SNP ID: hCV2892923 / Public SNP ID: rs13435192 /
SNP Chromosome Position: 155450158 / SNP in Genomic Sequence: SEQ ID NO: 354 /
SNP Position Genomic: 53106 / Related Interrogated SNP: hCV11503469 /
Related Interrogated SNP: hCV11503414 / Related Interrogated SNP: hCV11503470
/ Related Interrogated SNP: hCV2892877 / SNP Source: dbSNP; Celera; HapMap /
Population(Allele,Count): Caucasian (C,94|T,130) / SNP Type:
INTERGENIC;UNKNOWN /
Context (SEQ ID NO: 956): /
ACGTTTTGCATATAGTTACACAGGTTTCCCAACTGAAATAACTAAGAGAAATAAAAACAGAGAAAAACCTGCCTCTGTGCTGG
AAGGAGGGAAGGGTGAC
M
TGTCAATAAGTGTTTGTTGAATGAGTGAATGAATCATGCAAAATTCTAATATCTTATTAAAATAGGAGATAACCTGCCATAGC
TAGGACCCAGAAATAAA / Celera SNP ID: hCV2892924 / Public SNP ID: rs13435101 /
SNP Chromosome Position: 155449832 / SNP in Genomic Sequence: SEQ ID NO: 354 /
SNP Position Genomic: 52780 / Related Interrogated SNP: hCV11503469 /
Related Interrogated SNP: hCV11503414 / Related Interrogated SNP: hCV11503470
/ Related Interrogated SNP: hCV2892877 / SNP Source: dbSNP; Celera; HapMap /
Population(Allele,Count): Caucasian (C,95|A,131) / SNP Type:
INTERGENIC;UNKNOWN /
Context (SEQ ID NO: 957): /
AAATGAACTGGCAAAATCTGTAATGTTACAAAGTAATAAAATGTAAAACTAAAACTATGGCACATCTGAAAATCAAGAATGAC
TTGTTTGGCCCACATTA
Y
AATTCCTGTATACTAGAATAATAATATATGTTATGTCATAATTTTTTCATAAATGATTTTTTCAAAATTCAGCAATAATTTTAAA

TAAATGTGTGCATATAT / Celera SNP ID: hCV2892925 / Public SNP ID: rs7689945 /
SNP Chromosome Position: 155449629 / SNP in Genomic Sequence: SEQ ID NO: 354 /
SNP Position Genomic: 52577 / Related Interrogated SNP: hCV11503469 /
Related Interrogated SNP: hCV11503414 / Related Interrogated SNP: hCV11503470
/ Related Interrogated SNP: hCV2892877 / SNP Source: dbSNP; Celera; HapMap;
ABI_Val / Population(Allele,Count): Caucasian (C,94|T,130) / SNP Type:
INTERGENIC;UNKNOWN /
Context (SEQ ID NO: 958): /
CTCAAATCCAAGTCTCTCTTAAAAGATGTTGAACAGAAAGTACTTAAAGGAATATCTTACATTTTTCATCCATAGTTCAAGTT
AAATGATAGGGTAATAC
Y

AACACATTAGCTGTCCTTTATACATATTAATCAATAATGCAGTTTTATTATCAATAATTGATTTAAGTCTCAGTAGTGGACAA
TTTTAAAACAGTCTAAA / Celera SNP ID: hCV2892926 / Public SNP ID: rs7662567 /
SNP Chromosome Position: 155449148 / SNP in Genomic Sequence: SEQ ID NO: 354 /
SNP Position Genomic: 52096 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; Celera; HapMap / Population(Allele,Count):
Caucasian (T,90|C,26) / SNP Type: INTERGENIC;UNKNOWN /
Context (SEQ ID NO: 959): /
GTTAATGAGTGTAGACCTCAGAGATTTAAAATGAACTATCTGCAAATCTGTGACATTACACTAGAATAAATGTTTTATTACCC
CCTCAAATCCAAGTCTC
W

CTTAAAAGATGTTGAACAGAAAGTACTTAAAGGAATATCTTACATTTTTCATCCATAGTTCAAGTTAAATGATAGGGTAATAC
TAACACATTAGCTGTCC / Celera SNP ID: hCV2892927 / Public SNP ID: rs13123551 /
SNP Chromosome Position: 155449064 / SNP in Genomic Sequence: SEQ ID NO: 354 /
SNP Position Genomic: 52012 / Related Interrogated SNP: hCV11503414 /
Related Interrogated SNP: hCV11503469 / Related Interrogated SNP: hCV11503470
/ Related Interrogated SNP: hCV2892877 / SNP Source: dbSNP; Celera; HapMap /
Population(Allele,Count): Caucasian (T,52|A,62) / SNP Type:
INTERGENIC;UNKNOWN /
Context (SEQ ID NO: 960): /
TTTTTTATAAGTCAGTGTTAATGAGTGTAGACCTCAGAGATTTAAAATGAACTATCTGCAAATCTGTGACATTACACTAGAAT
AAATGTTTTATTACCCC
Y

TCAAATCCAAGTCTCTCTTAAAAGATGTTGAACAGAAAGTACTTAAAGGAATATCTTACATTTTTCATCCATAGTTCAAGTTA
AATGATAGGGTAATACT / Celera SNP ID: hCV2892928 / Public SNP ID: rs13147579 /
SNP Chromosome Position: 155449048 / SNP in Genomic Sequence: SEQ ID NO: 354 /
SNP Position Genomic: 51996 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; Celera; HapMap / Population(Allele,Count):
Caucasian (C,93|T,27) / SNP Type: INTERGENIC;UNKNOWN /
Context (SEQ ID NO: 961): /
TAGAGCTTCATGCCCTGGTAAGCTCTTCAGGCTAGATATATTTACTCAAGTTATTTCAGAGTAAATAAATCAGTCTTGGAACT
TTGATTATCTCCCATCA
R

ATAAGAAATAGGAAATTGGCCAGGCAAGGTGGCTTATGCCTGTAATCCCAGCACTTTGGGAGGCTGAGGCGGGAGTATCATTT
GAGCCCAGGAGTTCGAG / Celera SNP ID: hCV7429674 / Public SNP ID: rs871540 /
SNP Chromosome Position: 155409030 / SNP in Genomic Sequence: SEQ ID NO: 354 /
SNP Position Genomic: 11978 / Related Interrogated SNP: hCV11503469 /
Related Interrogated SNP: hCV11503414 / SNP Source: dbSNP; HapMap /
Population(Allele,Count): Caucasian (G,54|A,172) / SNP Type: INTRON /
Context (SEQ ID NO: 962): /
GGCTTCGCCAAGGTCTCCCCGGCCCCCAGCAAACTACCTGCTATGAAGTAGATGCTCTGTAAATAGTTGTTAAGCACAAATTG
CCTTGTGCTATGGAGAC
Y

GGGCCTGACATGAGTTGTAAAAGTATCAACAAGTGTATTGACAATGGGTATCGGCAAGTCTTTGCCCTTCAAGCCAGAGAGCT
GCTGGGCAGGACAGATT / Celera SNP ID: hCV7429793 / Public SNP ID: rs1025154 /
SNP Chromosome Position: 155481363 / SNP in Genomic Sequence: SEQ ID NO: 354 /
SNP Position Genomic: 84311 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:

hCV2892877 / SNP Source: dbSNP; HGBASE / Population(Allele,Count): Caucasian (T,178|C,48) / SNP Type: INTERGENIC;UNKNOWN /
Context (SEQ ID NO: 963): /
TGTAATAGATATTCAATAAGTATTTGTTGGTTAGATGTTGTTGAATTCAATCAATGTTATTGAATTAATGAATGTTGTTGTTT
GGATAAATAAATGAATT
R
ATGAATGAACATAGTACTTGTATTTGAGGGAGTATGAAAGAATACTCTTCATGTTACCACATGTAATTCAGGTCTCAATGCTG
GAGTTATACTTAGTACA / Celera SNP ID: hCV11503378 / Public SNP ID: rs1490655 /
SNP Chromosome Position: 155406865 / SNP in Genomic Sequence: SEQ ID NO: 354 /
SNP Position Genomic: 9813 / Related Interrogated SNP: hCV11503414 / Related
Interrogated SNP: hCV11503469 / SNP Source: dbSNP; HapMap; HGBASE /
Population(Allele,Count): Caucasian (G,24|A,84) / SNP Type: TRANSCRIPTION
FACTOR BINDING SITE;INTRON /
Context (SEQ ID NO: 964): /
ATAAGTATTTGTTGGTTAGATGTTGTTGAATTCAATCAATGTTATTGAATTAATGAATGTTGTTGTTTGGATAAATAAATGAA
TTGATGAATGAACATAG
K
ACTTGTATTTGAGGGAGTATGAAAGAATACTCTTCATGTTACCACATGTAATTCAGGTCTCAATGCTGGAGTTATACTTAGTA
CAGACTAAGATCTGGTT / Celera SNP ID: hCV11503379 / Public SNP ID: rs1490654 /
SNP Chromosome Position: 155406880 / SNP in Genomic Sequence: SEQ ID NO: 354 /
SNP Position Genomic: 9828 / Related Interrogated SNP: hCV11503414 / Related
Interrogated SNP: hCV11503469 / SNP Source: dbSNP; HapMap; HGBASE /
Population(Allele,Count): Caucasian (T,27|G,91) / SNP Type: INTRON /
Context (SEQ ID NO: 965): /
TAGAAATCCATGGAAGAAACTATCAGTGGGCACTGGCATACCCGGGCTCCTCTACATTTCTCAGCCTCCTTTGCAGTACATCT
GACGCTGCATGGCTGGG
M
TCTGGCTAATGTGACATTGGCGGAAGTTGTGTAAGTCACTCTCAGGCCTGGCTCTCTCAGACGGAGGAGAAACAGCCAAGTTG
CTTCACATCAGACTCTG / Celera SNP ID: hCV11503382 / Public SNP ID: rs1873369 /
SNP Chromosome Position: 155408478 / SNP in Genomic Sequence: SEQ ID NO: 354 /
SNP Position Genomic: 11426 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; Celera; HapMap; HGBASE /
Population(Allele,Count): Caucasian (C,76|A,28) / SNP Type: INTRON /
Context (SEQ ID NO: 966): /
TGCCATTCCTGTGAGCTCATCATCATGCCCCTGGCAGAAAAGCTACAAGTTTCTTCTTGTGACTTTCCTATGAATGCCTTCCT
CGCGTTGTTGCCTATAC
R
TGGACCGTTGCTCATTCAACAAAAGCCAACTTCTCACCATTCCTTCGCCTTCTCTCCTGCCCTGAGACTTTGATGAGCTCCCA
TCTGAATTGCCATGGAT / Celera SNP ID: hCV11852898 / Public SNP ID: rs6819508 /
SNP Chromosome Position: 155451187 / SNP in Genomic Sequence: SEQ ID NO: 354 /
SNP Position Genomic: 54135 / Related Interrogated SNP: hCV11503414 /
Related Interrogated SNP: hCV15860433 / SNP Source: dbSNP; Celera; HapMap /
Population(Allele,Count): Caucasian (A,17|G,103) / SNP Type:
INTERGENIC;UNKNOWN /
Context (SEQ ID NO: 967): /
ATGCTGGAGTTATACTTAGTACAGACTAAGATCTGGTTATAAGATAAAAGCTGCAAATGCAGCTCACAACATCAGACTTAGTG
TAGGGTGGAGGATGGAG
M
TCTTGTCTAGAGGTTGAAGTTAATCTTTTTGACAGTTATCTGAGCGAAAATGGAATCTTTCAGGCCAAACATGAGAACTGGAG
GAAAATCTCTGGGTGTC / Celera SNP ID: hCV11853415 / Public SNP ID: rs1490653 /
SNP Chromosome Position: 155407043 / SNP in Genomic Sequence: SEQ ID NO: 354 /
SNP Position Genomic: 9991 / Related Interrogated SNP: hCV11503414 / SNP
Source: dbSNP; Celera; HGBASE / Population(Allele,Count): Caucasian
(C,24|A,90) / SNP Type: INTRON /
Context (SEQ ID NO: 968): /
GCAGCTAGAGGTTATCTATCTCAGTTTGCTAATGGTTCTTGTCCTCTGCATTAATTCCCTCAGTTCTGCCTCACTCATAAGTC
TTTGGAAAGGTCTCGTC
W
GGGCATGAGAATATAAGAGTTCAGCCAACACTCGGAGAGGGGAGGGTGGCCAGCACCATAGTGCCAGCAACAGCAGCAACTAG
AGTGGTAGGACCCCAAA / Celera SNP ID: hCV11853416 / Public SNP ID: rs4346631 /
SNP Chromosome Position: 155407407 / SNP in Genomic Sequence: SEQ ID NO: 354 /

SNP Position Genomic: 10355 / Related Interrogated SNP: hCV11503414 / Related Interrogated SNP: hCV11503469 / SNP Source: dbSNP; Celera; HapMap; HGBASE / Population(Allele,Count): Caucasian (A,26|T,90) / SNP Type: INTRON / Context (SEQ ID NO: 969): /
TGCCAGCAACAGCAGCAACTAGAGTGGTAGGACCCCAAACATTGGGGATGTGGAGTAACCACTAAGACAACTTGAAGTTAAAG
GGTTTGTTTTTATCAGA
Y
GACATGGTATTATCTTACAGGAAATCTAGGCAAGATTTTGTTTTTCCTGTATTTCCTTTTGTTTTTGTTTGATTTTGACAAAG
GCAACTTGAGCAGATAT / Celera SNP ID: hCV11853418 / Public SNP ID: rs12501998 /
SNP Chromosome Position: 155407569 / SNP in Genomic Sequence: SEQ ID NO: 354 /
SNP Position Genomic: 10517 / Related Interrogated SNP: hCV11503469 /
Related Interrogated SNP: hCV11503414 / SNP Source: dbSNP; Celera; HapMap /
Population(Allele,Count): Caucasian (T,54|C,172) / SNP Type: INTRON /
Context (SEQ ID NO: 970): /
TATCTTACAGGAAATCTAGGCAAGATTTTGTTTTTCCTGTATTTCCTTTTGTTTTTGTTTGATTTTGACAAAGGCAACTTGAG
CAGATATAAGCTGTAAA
R
GAGGAAAACTTGTAGATGGCAACAGAAAGAAGGTACAGTTACAAGGAATGTATGTAATGTCATTAAGCAAAGCCATGGAACAC
AGGAGGTGTCATGAGAT / Celera SNP ID: hCV11853419 / Public SNP ID: rs13151559 /
SNP Chromosome Position: 155407680 / SNP in Genomic Sequence: SEQ ID NO: 354 /
SNP Position Genomic: 10628 / Related Interrogated SNP: hCV11503469 /
Related Interrogated SNP: hCV11503414 / SNP Source: dbSNP; Celera; HapMap /
Population(Allele,Count): Caucasian (A,54|G,172) / SNP Type: ACCEPTOR SPLICE
SITE;INTRON /
Context (SEQ ID NO: 971): /
ATGGAGTTGTAACAATGGGATAAACTCACACCTCATAGAAGAATCATCTCACGATGATAGAAACAACTCTAAAGACAGGCTTT
CATCAAAAAAATCATAA
Y
TAATCCACCCTGCTTATTTTCCTAACAATGGAGCCAAGTTGTACTATTCATACCAAGATCTTCAGGGAGTTCCAGGTGGAAGG
AATATTGGAAGTCAACT / Celera SNP ID: hCV11853423 / Public SNP ID: rs3857093 /
SNP Chromosome Position: 155408243 / SNP in Genomic Sequence: SEQ ID NO: 354 /
SNP Position Genomic: 11191 / Related Interrogated SNP: hCV11503469 /
Related Interrogated SNP: hCV11503414 / SNP Source: dbSNP; Celera; HapMap;
HGBASE / Population(Allele,Count): Caucasian (T,54|C,172) / SNP Type: TFBS
SYNONYMOUS;INTRON /
Context (SEQ ID NO: 972): /
GTTGTGTAAGTCACTCTCAGGCCTGGCTCTCTCAGACGGAGGAGAAACAGCCAAGTTGCTTCACATCAGACTCTGCTTGAGAG
AGAAATATACTTTGACA
R
TGTTAGTCACTAATATTTGTGGATTTGTCCATTTCATCAGCAAGCATTAATTCATATCAAGCCACTTTTTAATAATCTAGCAA
CTCAGAGCTGGCAATCA / Celera SNP ID: hCV11853424 / Public SNP ID: rs871541 /
SNP Chromosome Position: 155408604 / SNP in Genomic Sequence: SEQ ID NO: 354 /
SNP Position Genomic: 11552 / Related Interrogated SNP: hCV11503469 /
Related Interrogated SNP: hCV11503414 / SNP Source: dbSNP; Celera; HapMap;
HGBASE / Population(Allele,Count): Caucasian (G,54|A,172) / SNP Type: INTRON
/
Context (SEQ ID NO: 973): /
GAGACCCATCTCAGGAAAATAAAAAAAGTATGATGGGAGCAGTGCCTACTTCACAGAATTGGTCTGAGGATTACATGAGACAA
AGGAAGATCTCACAACA
R
TGCATGGCAAATAATAAAAGCTCAACATATGATGGTTATCACCATTATCATCATTATAGCATTATATTTGACCTTGTAACTAA
TGTGTTTTCTTTATTCC / Celera SNP ID: hCV26019871 / Public SNP ID: rs4547780 /
SNP Chromosome Position: 155432944 / SNP in Genomic Sequence: SEQ ID NO: 354 /
SNP Position Genomic: 35892 / Related Interrogated SNP: hCV11503414 /
Related Interrogated SNP: hCV11503469 / Related Interrogated SNP: hCV2892877 /
Related Interrogated SNP: hCV15860433 / Related Interrogated SNP: hCV11503470
/ SNP Source: dbSNP; Celera; HapMap; HGBASE / Population(Allele,Count):
Caucasian (G,93|A,133) / SNP Type: INTERGENIC;UNKNOWN /
Context (SEQ ID NO: 974): /
GTAGGGAGGTGGGACGGTGGGGGAGGGATTTAAAAAAAATCTATCCCACATCTCCCCTGAACACCATCTCTCCCTCACTCCCC
AAAAAAGTTGACAAAAG
R

GTTTTATTCTTCCCATGTAGCTGTCTGAGGAATTGACCATATCTGGGTGGGGTATGGAATGTGGGCATCCCTAGGTTCCTGGA
AGCAGCTCTTATGCTAC / Celera SNP ID: hCV27313127 / Public SNP ID: rs4482740 /
SNP Chromosome Position: 155446305 / SNP in Genomic Sequence: SEQ ID NO: 354 /
SNP Position Genomic: 49253 / SNP Source: dbSNP; Celera; HapMap; ABI_Val;
HGBASE / Population(Allele,Count): Caucasian (G,155|A,71) / SNP Type:
INTERGENIC;UNKNOWN /
Context (SEQ ID NO: 975): /
TTTCCTGCAGTGAGAACAGTAGAATTTTATTCTGGGATACTCTCTGACCAAGCCGACGTCACAGGTGGGCTGCAGTGCCTCTG
GCTGCCTTCCCACCTCA
Y

TGGCTTGGCATCCTCAGCAAAGTGAAGTCTTCATCTTTGTGATGGGAATGGGACAGTCTCCCTTGTGGACACCAAGAGAGGGA
GAGGTGCCCTTGGCTCA / Celera SNP ID: hCV27313130 / Public SNP ID: rs4634202 /
SNP Chromosome Position: 155445586 / SNP in Genomic Sequence: SEQ ID NO: 354 /
SNP Position Genomic: 48534 / Related Interrogated SNP: hCV11503469 /
Related Interrogated SNP: hCV15860433 / Related Interrogated SNP: hCV11503414
/ Related Interrogated SNP: hCV11503470 / SNP Source: dbSNP; Celera; HapMap;
HGBASE / Population(Allele,Count): Caucasian (C,45|T,59) / SNP Type:
INTERGENIC;UNKNOWN /
Context (SEQ ID NO: 976): /
TTAATAAAATACATCAGGCAAAGGAATTATTTTCTAGTATTAAAGATCCATTGTTACTAACTTTATTTAGAGCAAAGTAAGAT
TCAAATCAGTTCTAAAG
R

TTACTAGTCTCAAGATGTTTTGTCTTCTAACTTCAATTTCTTAGAAAAATAATTTTGACACTATAAAATAATAATTGTTCATA
ACACAGTATTCTAGTCA / Celera SNP ID: hCV27313137 / Public SNP ID: rs12645631 /
SNP Chromosome Position: 155460769 / SNP in Genomic Sequence: SEQ ID NO: 354 /
SNP Position Genomic: 63717 / Related Interrogated SNP: hCV15860433 / SNP
Source: dbSNP; Celera; HapMap / Population(Allele,Count): Caucasian
(A,13|G,101) / SNP Type: INTRON;PSEUDOGENE /
Context (SEQ ID NO: 977): /
TATGAAATTGAGAGAAATTATGAGCAGCTGGAACATTAAGGGCACATTGTCAGAGGGAGTTTTCATTGCCAATTTGGAATGAA
GTTCCAGCATAGTCCAG
Y

GCCATGATTTAAACACATAAGATCCTAGATGCCCCAATCTAGTTAATTTGAGCATATGTAATATCCAATCCTTGGATTATTTG
ATTGACAGAGCCATTAA / Celera SNP ID: hCV27905214 / Public SNP ID: rs4323084 /
SNP Chromosome Position: 155424231 / SNP in Genomic Sequence: SEQ ID NO: 354 /
SNP Position Genomic: 27179 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; HapMap; HGBASE / Population(Allele,Count):
Caucasian (T,51|C,171) / SNP Type: INTERGENIC;UNKNOWN /
Context (SEQ ID NO: 978): /
CCAATCTTAGGTTCTACAAACAGTGCTGTTATCTATAGGAGGAATTGGAGAAGTTACAAACCTTGTGACCTCTAGAACAATGG
TCAGTTATTAATTACAC
Y

TATGTCTTCTCAGAATTCAGGCCCCTATCTCATGGTCCTAACCTTGTGGCTTTTCTTTAGTTTTACAAAGGCAGTTTAGTATT
CGGAAGGATTATTATCA / Celera SNP ID: hCV27937396 / Public SNP ID: rs4634201 /
SNP Chromosome Position: 155435421 / SNP in Genomic Sequence: SEQ ID NO: 354 /
SNP Position Genomic: 38369 / Related Interrogated SNP: hCV11503414 /
Related Interrogated SNP: hCV11503469 / Related Interrogated SNP: hCV11503470
/ Related Interrogated SNP: hCV2892877 / Related Interrogated SNP:
hCV15860433 / SNP Source: dbSNP; HapMap / Population(Allele,Count): Caucasian
(C,42|T,184) / SNP Type: INTERGENIC;UNKNOWN /
Context (SEQ ID NO: 979): /
GCCCCAATTCCTGTATAGCGCTCTAGGAGAGCGGTTTCAGAGTGGACAGTGTGATACCATAAACAGTAGAGAAAAATAGCTGC
TTCTCAGATTTCAGACA
Y

AAGGAAGAACATGCTTGGATGTCTAGGTGTGTGTGTGAAGGATGAGGGCCCCAGAAATATTTTCGTAAGTTTAAATTCTGACC
TGACTGTATTTCAAACC / Celera SNP ID: hCV28953838 / Public SNP ID: rs7690851 /
SNP Chromosome Position: 155433663 / SNP in Genomic Sequence: SEQ ID NO: 354 /
SNP Position Genomic: 36611 / Related Interrogated SNP: hCV11503414 /
Related Interrogated SNP: hCV11503469 / Related Interrogated SNP: hCV2892877 /
Related Interrogated SNP: hCV15860433 / Related Interrogated SNP: hCV11503470
/ SNP Source: dbSNP / Population(Allele,Count): Caucasian (T,92|C,134) / SNP

197

Type:     INTERGENIC;UNKNOWN /
Context                (SEQ ID NO: 980): /
TGTGCTAATAATTAGCCATCTCCCACATTCTTGATCACATGTAAACAGTAGACCAGGCATGGTGGAGAGAGCAGCTAATTAAT
ATCATTCAGATGGAAGG
R
CTTTCATTTTCTGCCTGCTTTTGGGAAATTATTCCCTGGAAGAATTTCTTTGTATGGAGATATTGGACAATCTCCTATGTCCA
TCCTGTGGACAATAGGA / Celera SNP ID:   hCV28953840 / Public SNP ID:   rs6536017 /
SNP Chromosome Position:   155430666 / SNP in Genomic Sequence:   SEQ ID NO: 354 /
 SNP Position Genomic:   33614 / Related Interrogated SNP:   hCV11503414 /
Related Interrogated SNP:   hCV11503469 / Related Interrogated SNP:   hCV15860433
/ Related Interrogated SNP:   hCV11503470 / Related Interrogated SNP:
hCV2892877 / SNP Source:   dbSNP; HapMap; ABI_Val / Population(Allele,Count):
Caucasian (G,140|A,76) / SNP Type:   INTERGENIC;UNKNOWN /
Context                (SEQ ID NO: 981): /
CCGCGTCCGTGGCCACCAGTAGTAACTCATACAGATCGCGGCTCTCTCTGTCCAGGGGCCCCTCCACGCAAAGGAAAAATACC
CCTGGGGGGCCGCCGGG
Y
AGCAACGCGAAGTCTCCCTCTCCGCCTTCCAAGGACAGAGAGATGCTCCCGTCTCCAAGACCCACACCAAGCTCCCCTGTGGC
CTCATCTTCCTTCTCCC / Celera SNP ID:   hCV28954780 / Public SNP ID:   rs7656522 /
SNP Chromosome Position:   155411065 / SNP in Genomic Sequence:   SEQ ID NO: 354 /
 SNP Position Genomic:   14013 / Related Interrogated SNP:   hCV11503469 /
Related Interrogated SNP:   hCV11503414 / SNP Source:   dbSNP; HapMap /
Population(Allele,Count):   Caucasian (T,54|C,170) / SNP Type:   UTR5;SILENT RARE
CODON;SILENT MUTATION /
Context                (SEQ ID NO: 982): /
ATTGGTACCTAGAATGCATGTGGTAAATTGTATTTTTCTTGCCCTCTGGTGTTTGTATGATGAAATGTTTTAGGAGTGAATTA
TTTTGGAATTCTCAGTA
R
TATCAAACTCCGACTTCTTAGCCCCCCAGAAGGTGTGAAGGGGGAGGATATAAAATTCCCACTATCTGGCTGGATGCAGTGGC
TCATGCCTGTAATCCCA / Celera SNP ID:   hCV28954801 / Public SNP ID:   rs4447837 /
SNP Chromosome Position:   155416594 / SNP in Genomic Sequence:   SEQ ID NO: 354 /
 SNP Position Genomic:   19542 / Related Interrogated SNP:   hCV11503469 / SNP
Source:   dbSNP; HapMap; HGBASE / Population(Allele,Count):   Caucasian
(A,177|G,49) / SNP Type:   INTERGENIC;UNKNOWN /
Context                (SEQ ID NO: 983): /
TTGGAGTTGGAACCCTGGAGATGCCGAAGGAAACCCGTCTTCCCTACCGCCCCCTAACGCACCGGCCTGCTTGGAACGCAGTT
GGAGGCTGCGCGGTACC
R
GTCCCATGGGGGCTTCTGCTCCGGGTCCTCCAGTCTCAGTGGTCGCTGCTGAGCCGGCTCCCTCTGACTTTTTTTTTTTAGACG
GAGTCTCGCTCTGTCGT / Celera SNP ID:   hCV29420822 / Public SNP ID:   rs4642230 /
SNP Chromosome Position:   155444758 / SNP in Genomic Sequence:   SEQ ID NO: 354 /
 SNP Position Genomic:   47706 / Related Interrogated SNP:   hCV11503414 /
Related Interrogated SNP:   hCV11503469 / Related Interrogated SNP:   hCV11503470
/ Related Interrogated SNP:   hCV15860433 / Related Interrogated SNP:
hCV2892877 / SNP Source:   dbSNP; HapMap; HGBASE / Population(Allele,Count):
Caucasian (A,24|G,92) / SNP Type:   INTERGENIC;UNKNOWN /
Context                (SEQ ID NO: 984): /
CAATGACTTTGCTTGTGTACACTTGTCAGATCATTCCCATTAGGCAGTTTCCTAGGAATGAACTTAGTAGGCCAAAAGGTTCA
AAGTTTCAATAAATCCC
R
GAAAACTACATTCCTAAAGGCTGTGACAATTATACTCCCACCTATGGCATACAAGATGGGTAATGTAATTGGAAGTGCTTGTT
CAAAATCATGGTGCAAT / Celera SNP ID:   hCV29420827 / Public SNP ID:   rs7654425 /
SNP Chromosome Position:   155456608 / SNP in Genomic Sequence:   SEQ ID NO: 354 /
 SNP Position Genomic:   59556 / Related Interrogated SNP:   hCV15860433 /
Related Interrogated SNP:   hCV11503469 / Related Interrogated SNP:   hCV11503414
/ SNP Source:   dbSNP; HapMap; ABI_Val / Population(Allele,Count):   Caucasian
(A,151|G,75) / SNP Type:   TRANSCRIPTION FACTOR BINDING SITE;UTR3 /
Context                (SEQ ID NO: 985): /
ATGCCTTTGATACAACGTTAAGTGAAAAAAGCAAGATACAAAATTACATACTAAGAATGATTTTTTAAAAGATACACACACAG
GAAAAGAGCTTAGAAGT
R
AAGGTATTGATACTTACAGAAATAAATACTAAAACGAAACTTATCCAAATATCTAACAATGGTAGGATTATATACAGGTCTAT
GTACTTTCCTAATTCTC / Celera SNP ID:   hCV29420828 / Public SNP ID:   rs7660120 /

SNP Chromosome Position: 155462932 / SNP in Genomic Sequence: SEQ ID NO: 354 / SNP Position Genomic: 65880 / Related Interrogated SNP: hCV15860433 / Related Interrogated SNP: hCV11503414 / Related Interrogated SNP: hCV11503469 / SNP Source: dbSNP; HapMap / Population(Allele,Count): Caucasian (G,73|A,27) / SNP Type: UTR5;INTRON /
Context (SEQ ID NO: 986): /
CACATTTTGATTGGTCCACACAATCAAACCAGAAGAATGTTCCAGTTTATTGAATTTGTCTCCTTATTTAAGAAAAAGAAAAT
TAAGCCAGGAGAAGTTA
R
CGCTCCATCCACCAGCTGGGTCTTTGATGAAGGGAAGTGAAATGAGGATTCTGCACAGGTGGGCGGTAGCTGGTGGAGTGAGC
GCCCTGCAGGTGCAAGT / Celera SNP ID: hCV29983641 / Public SNP ID: rs10008078 /
SNP Chromosome Position: 155448553 / SNP in Genomic Sequence: SEQ ID NO: 354 / SNP Position Genomic: 51501 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; HapMap / Population(Allele,Count): Caucasian (G,178|A,48) / SNP Type: INTERGENIC;UNKNOWN /
Context (SEQ ID NO: 987): /
AGAACTAATTCAGCAGCTCAGGATAGAAAGGAGAGCAAAGAACCTGTGACACCATAAAAAACTTCAGCAGCAGTAACCAACCA
TGATGGCAATTAACATA
W
CCAGCAAAATAATAGCTAATATTCGTAGAGTGTTTACTATTTGGTAGGCCCTGCAATGACTTTACTCTTTAATAGGCACAACA
ACTCGATGAGACTAGAA / Celera SNP ID: hCV32212658 / Public SNP ID: rs11099959 /
SNP Chromosome Position: 155452470 / SNP in Genomic Sequence: SEQ ID NO: 354 / SNP Position Genomic: 55418 / Related Interrogated SNP: hCV11503469 / Related Interrogated SNP: hCV11503470 / SNP Source: dbSNP; HapMap / Population(Allele,Count): Caucasian (A,80|T,38) / SNP Type: INTERGENIC;UNKNOWN /
Context (SEQ ID NO: 988): /
TGTTAATGCCTGGAAAGACTTTGGGAATAATTAGATTGTACTATCAGTTATAAACTGTCAGTTATAAATATCTTAGCAAGGTG
TATGTGTGTGTGTGAGA
R
AGAGAGAGAGAGTCCTCGGGTCTTACTTATCACAATTTCACCATCCTCTACTACATGAAGCCCCATCTCATGTGTATTAGTTT
GCTAGGGCTGATGTAAC / Celera SNP ID: hCV30711231 / Public SNP ID: rs12642469 /
SNP Chromosome Position: 155474222 / SNP in Genomic Sequence: SEQ ID NO: 354 / SNP Position Genomic: 77170 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; HapMap; ABI_Val / Population(Allele,Count): Caucasian (G,178|A,48) / SNP Type: INTERGENIC;UNKNOWN /
Context (SEQ ID NO: 989): /
TATGACTCTTAGGTATCAATATTTCTATTTGATATGTTAATTAGCTAACAAATTATTCTCAAGGAAAGAAAGACAGAAATTCT
CCATGAGAATTCTGATT
Y
TCAGCTTTGTAAATTCTCCTAAAGAAGTTTCAGGAGAAACTTCCTCTGACCAAGTTAAGAAAGAAGAGCCGTATTTAATCAAA
GTATCTATCTACTGCTG / Celera SNP ID: hCV32212669 / Public SNP ID: rs12649647 /
SNP Chromosome Position: 155454287 / SNP in Genomic Sequence: SEQ ID NO: 354 / SNP Position Genomic: 57235 / Related Interrogated SNP: hCV15860433 / Related Interrogated SNP: hCV11503414 / Related Interrogated SNP: hCV11503470 / SNP Source: dbSNP; HapMap / Population(Allele,Count): Caucasian (T,141|C,85) / SNP Type: INTERGENIC;UNKNOWN /
Context (SEQ ID NO: 990): /
TTTTGCATTTGGGCCTGAGAGAAGAATTAATTTCCTAGATACCGTTTCTCTCCTTTAGGGAGGACCAAAAGCCCTGCATAGTG
GTTTCTTGCAGCAACAA
Y
AAAATGCCTTTTTAAATGTGCAACGGAGGTGTATGTAATTGCTAATCCACAGCCACACCGTTATCTCTACCTGTGACCAAATA
ACAATAAAATAATTGAC / Celera SNP ID: hCV30679141 / Public SNP ID: rs13111621 /
SNP Chromosome Position: 155414148 / SNP in Genomic Sequence: SEQ ID NO: 354 / SNP Position Genomic: 17096 / Related Interrogated SNP: hCV11503414 / SNP Source: dbSNP; HapMap / Population(Allele,Count): Caucasian (C,95|T,25) / SNP Type: INTERGENIC;UNKNOWN /
Context (SEQ ID NO: 991): /
TTCCTAGATACCGTTTCTCTCCTTTAGGGAGGACCAAAAGCCCTGCATAGTGGTTTCTTGCAGCAACAACAAAATGCCTTTTT

AAATGTGCAACGGAGGT
R
TATGTAATTGCTAATCCACAGCCACACCGTTATCTCTACCTGTGACCAAATAACAATAAAATAATTGACTTTGTAGCATATAT
GTTACCGAAAAATGGGGA / Celera SNP ID: hCV30679140 / Public SNP ID: rs13112066 /
SNP Chromosome Position: 155414179 / SNP in Genomic Sequence: SEQ ID NO: 354 /
SNP Position Genomic: 17127 / Related Interrogated SNP: hCV11503469 /
Related Interrogated SNP: hCV11503414 / SNP Source: dbSNP; HapMap /
Population(Allele,Count): Caucasian (G,174|A,52) / SNP Type:
INTERGENIC;UNKNOWN /
Context (SEQ ID NO: 992): /
ACCGTTTCTCTCCTTTAGGGAGGACCAAAAGCCCTGCATAGTGGTTTCTTGCAGCAACAACAAAATGCCTTTTTAAATGTGCA
ACGGAGGTGTATGTAAT
K
GCTAATCCACAGCCACACCGTTATCTCTACCTGTGACCAAATAACAATAAAATAATTGACTTTGTAGCATATATGTTACCGAA
AATGGGGACATTATTAA / Celera SNP ID: hCV30679139 / Public SNP ID: rs13139082 /
SNP Chromosome Position: 155414188 / SNP in Genomic Sequence: SEQ ID NO: 354 /
SNP Position Genomic: 17136 / Related Interrogated SNP: hCV11503469 /
Related Interrogated SNP: hCV11503414 / SNP Source: dbSNP; HapMap /
Population(Allele,Count): Caucasian (T,91|G,23) / SNP Type:
INTERGENIC;UNKNOWN /
Context (SEQ ID NO: 993): /
AACCAGCCTGGGGCCTGACCTCTTCTGCTCTCAGCCCCTTTCTCTGCATATAGAAAGTCTAAATGCAGTATGTGTAGACTTTT
AAAGTTTTAAAATTCAG
Y
CTCAGGAAGAAAATCAGAAATACTGGCTGGTGAAATAAACAGTTTAGGCATCTTGTAAGCCTGCCTTTTTTGAAAAACAACAA
AACAAAGTAAGTGTTGG / Celera SNP ID: hCV32212663 / Public SNP ID: rs7670827 /
SNP Chromosome Position: 155446896 / SNP in Genomic Sequence: SEQ ID NO: 354 /
SNP Position Genomic: 49844 / Related Interrogated SNP: hCV11503414 /
Related Interrogated SNP: hCV11503469 / Related Interrogated SNP: hCV15860433
/ SNP Source: dbSNP; HapMap / Population(Allele,Count): Caucasian
(C,148|T,78) / SNP Type: INTERGENIC;UNKNOWN /
Context (SEQ ID NO: 994): /
ATTTTTTTCACAACTTCTTGACACTAACAGATGAGAAAGAAGGCCACTTTTGCTCAAGAACCAAAACAGAGTACTGAGTCCAT
CGTCAAGAGATATCAGA
M
ATTGAAAAAGATAGCAGCACAGGTTTCCAGAACTCTTCTTTGAGTAGGAAAGACTTGGGGAAAAAAATGGGCCAATAGGTAGC
AGACATCAACTCCTACA / Celera SNP ID: hCV30562176 / Public SNP ID: rs9284660 /
SNP Chromosome Position: 155403593 / SNP in Genomic Sequence: SEQ ID NO: 354 /
SNP Position Genomic: 6541 / Related Interrogated SNP: hCV11503414 / Related
Interrogated SNP: hCV11503469 / SNP Source: dbSNP / Population(Allele,Count):
Caucasian (C,149|A,77) / SNP Type: INTRON /
Context (SEQ ID NO: 995): /
TGTCTAGTTGGATCTATTAACTTTTTACATATTTAATCATCCTGGAGACATTAATTTAATTTCTCAATAACAACATGGTAATG
ATTGAGGTAAATTGCCT
Y
GAATTTGCTCCTTTTAAAAACACTGTCGTGAATTTATAGAATGATGTCATCCCCTTGAAAGTTTACAATATTTTAGATAAATAT
AGAACTCAAGTGACCCT / Celera SNP ID: hCV29570696 / Public SNP ID: rs9997519 /
SNP Chromosome Position: 155473170 / SNP in Genomic Sequence: SEQ ID NO: 354 /
SNP Position Genomic: 76118 / Related Interrogated SNP: hCV11503414 /
Related Interrogated SNP: hCV11503469 / Related Interrogated SNP: hCV15860433
/ SNP Source: dbSNP; HapMap / Population(Allele,Count): Caucasian
(C,105|T,15) / SNP Type: INTERGENIC;UNKNOWN /
Context (SEQ ID NO: 996): /
TTTAAAGCTGTCTCAAATCATATTACCTCCTGCTTAGAACCTGAAATGATTCCATTATACTTAGATTATAATTCAGACTCCTT
CAATCAAGCTCCTTTTA
R
TAGTACAGGATCCTATCCATCTTTCTGACCTTGCCTCATACCACCACTCTGGACTCAGCCATCTGCCTTCTTTTTTGATCTTGT
ACCTTTGAATTGTTAGA / Celera SNP ID: hDV70817639 / Public SNP ID: rs17031739 /
SNP Chromosome Position: 155414923 / SNP in Genomic Sequence: SEQ ID NO: 354 /
SNP Position Genomic: 17871 / Related Interrogated SNP: hCV11503469 / SNP
Source: dbSNP; HapMap / Population(Allele,Count): Caucasian (G,176|A,48) /
SNP Type: INTERGENIC;UNKNOWN /
Context (SEQ ID NO: 997): /

ATTATTACAACCTCAAAACCTTCATTCTGCTTTATCTCAGCACCTAGAACAGTACCTAGCATGTAGTGTGTCTCTATTTACTA
AGTATGTGTTGGACAGA
Y
GGTTGGTTGAACCTATGGGTAAGTGAATGCATTTGTCTGTTAAAGTAGAAGTTGGAAGCCATAACCAAGAATGAATTGGCCAT
GAGAATCTTCACTGTAA / Celera SNP ID: hDV70817640 / Public SNP ID: rs17031740 /
SNP Chromosome Position: 155415340 / SNP in Genomic Sequence: SEQ ID NO: 354 /
SNP Position Genomic: 18288 / Related Interrogated SNP: hCV11503469 / SNP
Source: dbSNP; HapMap / Population(Allele,Count): Caucasian (C,177|T,49) /
SNP Type: INTERGENIC;UNKNOWN /
Context (SEQ ID NO: 998): /
TCAATTAACTATAATAATCGGTAGAAACTGATCAATGGCCTCTAATAAAGTACGCCTGAGAAAAGACTCTCTGGACTCTTAAA
AATGGCTGACCATATTT
R
GCAAGTGGTTTCTGCAATTTTGCATTTGGGCCTGAGAGAAGAATTAATTTCCTAGATACCGTTTCTCTCCTTTAGGGAGGACC
AAAAGCCCTGCATAGTG / Celera SNP ID: hDV70934991 / Public SNP ID: rs17301943 /
SNP Chromosome Position: 155414030 / SNP in Genomic Sequence: SEQ ID NO: 354 /
SNP Position Genomic: 16978 / Related Interrogated SNP: hCV11503469 /
Related Interrogated SNP: hCV11503414 / SNP Source: dbSNP; HapMap /
Population(Allele,Count): Caucasian (A,172|G,54) / SNP Type: TRANSCRIPTION
FACTOR BINDING SITE;INTERGENIC;UNKNOWN /
Context (SEQ ID NO: 999): /
GGTGACGAGGAGCCTGGCAAAAGCGGTGACGGTAGTGGCCCCGGAGTCCGGTAGCGCAACTGGAAGAACGGGCCTGCGGGGTC
CTTGGGCAGGTCGGACG
R
TTGCACCAGGGTGTAGCCCTGAGTGCTGAACAGTCCGGCGTCCGGATCGTGGGCAACTGGCAGGCGGAAGGCGGTCCCTGGCG
GGCTGAGCTCGGAGACG / Celera SNP ID: hDV70945235 / Public SNP ID: rs17373860 /
SNP Chromosome Position: 155411883 / SNP in Genomic Sequence: SEQ ID NO: 354 /
SNP Position Genomic: 14831 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; HapMap / Population(Allele,Count): Caucasian
(G,104|A,12) / SNP Type: MISSENSE MUTATION;UTR5 /
Context (SEQ ID NO: 1000): /
TTCCCCTTCCCCTTCTTCTTCTCCTCCTCCTCCTTCTTCTTCCAGCAAATATCAAATTTGGAGAGACAATCTGGTAGATT
TAGCTTGCGTAAAGGGT
Y
TGTCTACCCCTAGCTTGGTCACCTGTGGTTAGAGGGTCAGGGTGTTGTAGTACTGACAGGACCACTGGAGCTTCATACCTCCA
TTTTAGGGGTCATTCCA / Celera SNP ID: hCV31863993 / Public SNP ID: rs7673587 /
SNP Chromosome Position: 155480434 / SNP in Genomic Sequence: SEQ ID NO: 354 /
SNP Position Genomic: 83382 / Related Interrogated SNP: hCV11503414 /
Related Interrogated SNP: hCV11503469 / Related Interrogated SNP: hCV15860433
/ SNP Source: dbSNP; HapMap; ABI_Val / Population(Allele,Count): Caucasian
(C,85|T,35) / SNP Type: INTERGENIC;UNKNOWN /
Context (SEQ ID NO: 1001): /
TTACATATAAAGATGAAGATGATTATATAAAATATAAATTATATTAAAAATAAACATACAGTAAATTATGCATAATATATAGA
TATGCCATCCAAGGCAT
Y
TATGTTATTTTCTCCTTGAATGATCAACATGACATTCACCAGAAACTGGATATGTCTAGAGACATCTTGATTTGCACCTAAAA
CATGAAGAGCCGCTTTA / Celera SNP ID: hCV32212659 / Public SNP ID: rs4622984 /
SNP Chromosome Position: 155452233 / SNP in Genomic Sequence: SEQ ID NO: 354 /
SNP Position Genomic: 55181 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; ABI_Val; HGBASE / Population(Allele,Count):
Caucasian (T,80|C,146) / SNP Type: INTERGENIC;UNKNOWN /
Context (SEQ ID NO: 1002): /
ATCTAAAGATGAGCAGTCTAGACTTGGAATAGCAGCACTGTATTCATAATGACCTCAGGCTGCTTCTGTATCCCTGCTACATT
ATCCTAATGCTGGCTTC
Y
ATTTTCAGGATCACCTCTTGTCCTAAGATGGTGGGACAAGCTGTCACATTTATATGCTCCAGCTATCACATTTGCTTTCCTGA
CAGGAGAAAGGGTGAAT / Celera SNP ID: hCV32212662 / Public SNP ID: rs11099958 /
SNP Chromosome Position: 155451527 / SNP in Genomic Sequence: SEQ ID NO: 354 /
SNP Position Genomic: 54475 / Related Interrogated SNP: hCV11503414 /

Related Interrogated SNP: hCV15860433 / SNP Source: dbSNP; HapMap / Population(Allele,Count): Caucasian (C,143|T,83) / SNP Type: INTERGENIC;UNKNOWN / Context (SEQ ID NO: 1003): / GTGGGGTATGGAATGTGGGCATCCCTAGGTTCCTGGAAGCAGCTCTTATGCTACTCATAGAGATGGGACTGATTTTATTTTTT ATAGCGCTTAATTCACC R TTATGGGAAATGCTTCCAATCACAAAAATTCAGCCCAGCTCGTTTTGAGGAAGAGGCAGGACATGTTATAGTTTTATACAACT CATTTCCATTAAACTGA / Celera SNP ID: hCV32212664 / Public SNP ID: rs12642646 / SNP Chromosome Position: 155446452 / SNP in Genomic Sequence: SEQ ID NO: 354 / SNP Position Genomic: 49400 / Related Interrogated SNP: hCV11503469 / Related Interrogated SNP: hCV11503470 / Related Interrogated SNP: hCV11503414 / SNP Source: dbSNP / Population(Allele,Count): Caucasian (G,104|A,122) / SNP Type: INTERGENIC;UNKNOWN / Context (SEQ ID NO: 1004): / AATACCAGCCCCAATATACCACTTTCTCTCCTAAACTTCCTTTCTTTCACTGAGTCCAGTGGACTATATTTTTGATTTAAATT TTTTCTCCATCTCTCCA R CATTTCTTGAATCTGGATAATCAGGTCTTGTTTAAATGATTGGAATTGCTTTTTTGACAAGCCAGCCTACCTCCAATACCTAAA CATGTTCCATCCCTCCA / Celera SNP ID: hCV32287640 / Public SNP ID: rs4367156 / SNP Chromosome Position: 155427052 / SNP in Genomic Sequence: SEQ ID NO: 354 / SNP Position Genomic: 30000 / Related Interrogated SNP: hCV11503469 / SNP Source: dbSNP; HGBASE / Population(Allele,Count): Caucasian (G,177|A,49) / SNP Type: INTERGENIC;UNKNOWN / Context (SEQ ID NO: 1005): / AAAAATAGATAAAGGGAGTAAGATTTAGTGTTTGGTAGCACTAGTTTGGTTAGCTAGTTTGGTAGCACTAGTTTGGCTAGTTA GCTTGGTAGCACAATAG R GTGACTACAGTTAAGAGTAATTTATTGTATATTTCAAAATAACTAGAAGAGTAAAATTGGAATGTTCCTAATACAAAGAAATA ATATCCCAATTACCGTG / Celera SNP ID: hDV77232287 / Public SNP ID: rs7666918 / SNP Chromosome Position: 155464173 / SNP in Genomic Sequence: SEQ ID NO: 354 / SNP Position Genomic: 67121 / Related Interrogated SNP: hCV15860433 / Related Interrogated SNP: hCV11503469 / Related Interrogated SNP: hCV11503414 / SNP Source: CDX; dbSNP / Population(Allele,Count): Caucasian (A,151|G,75) / SNP Type: INTRON //

Gene Number: 18 / Gene Symbol: PRLR - 5618 / Gene Name: prolactin receptor / Chromosome: 5 / OMIM NUMBER: 176761 / OMIM Information: // Genomic Sequence (SEQ ID NO: 355): // / SNP Information / Context (SEQ ID NO: 1006): / TTGCAGAGAGACACTCATGCATGAATGTCCAGACTACATAACCGGTGGCCCCAACTCCTGCCACTTTGGCAAGCAGTACACCT CCATGTGGAGGACATAC R TCATGATGGTCAATGCCACTAACCAGATGGGAAGCAGTTTCTCGGATGAACTTTATGTGGACGTGACTTACATAGGTAAGGGG AAGGAAAGTGGGTGAGG / Celera SNP ID: hCV16171263 / Public SNP ID: rs78705921 / SNP Chromosome Position: 35084647 / SNP in Genomic Sequence: SEQ ID NO: 355 / SNP Position Genomic: 38845 / SNP Source: CDX; dbSNP / Population(Allele,Count): Caucasian (A,114|G,2) / SNP Type: MISSENSE MUTATION;ESS;UTR5;INTRON //

Gene Number: 19 / Gene Symbol: PROC - 5624 / Gene Name: protein C (inactivator of coagulation factors Va and VIIIa) / Chromosome: 2 / OMIM NUMBER: 176860 / OMIM Information: Thrombophilia due to protein C deficiency (3); Purpura fulminans,/neon / atal (1) // / Genomic Sequence (SEQ ID NO: 356): // / SNP Information / Context (SEQ ID NO: 1007): / TGAAAAACAGGGACAACGTTCCTCCCTCAGCCAGCCACTATGGGGCTAAAATGAGACCACATCTGTCAAGGGTTTTGCCCTCA CCTCCCTCCCTGCTGGA Y GGCATCCTTGGTGGGCAGAGGTGGGCTTCGGGCAGAACAAGCCGTGCTGAGCTAGGACCAGGAGTGCTAGTGCCACTGTTTGT CTATGGAGAGGGAGGCC / Celera SNP ID: hCV1841973 / Public SNP ID: rs1799808 / SNP Chromosome Position: 128175862 / SNP in Genomic Sequence: SEQ ID NO: 356 / SNP Position Genomic: 74100 / SNP Source: Applera /

Population(Allele,Count): Caucasian (C,19|T,17) African American (C,18|T,0) total (C,37|T,17) / SNP Type: INTERGENIC;UNKNOWN / SNP Source: dbSNP; HapMap; HGBASE / Population(Allele,Count): Caucasian (C,154|T,72) / SNP Type: INTERGENIC;UNKNOWN /
Context (SEQ ID NO: 1008): /
CAACGTTCCTCCCTCAGCCAGCCACTATGGGGCTAAAATGAGACCACATCTGTCAAGGGTTTTGCCCTCACCTCCCTCCCTGC
TGGACGGCATCCTTGGT
R
GGCAGAGGTGGGCTTCGGGCAGAACAAGCCGTGCTGAGCTAGGACCAGGAGTGCTAGTGCCACTGTTTGTCTATGGAGAGGGA
GGCCTCAGTGCTGAGGG / Celera SNP ID: hCV1841974 / Public SNP ID: rs1799809 /
SNP Chromosome Position: 128175875 / SNP in Genomic Sequence: SEQ ID NO: 356 /
SNP Position Genomic: 74113 / SNP Source: Applera /
Population(Allele,Count): Caucasian (A,14|G,12) African American (A,1|G,17) total (A,15|G,29) / SNP Type: INTERGENIC;UNKNOWN / SNP Source: Applera / Population(Allele,Count): Caucasian (A,15|G,15) African American (A,4|G,26) total (A,19|G,41) / SNP Type: INTERGENIC;UNKNOWN / SNP Source: dbSNP; HGBASE / Population(Allele,Count): Caucasian (A,150|G,119) / SNP Type: INTERGENIC;UNKNOWN /
Context (SEQ ID NO: 1009): /
GTTTGTCTATGGAGAGGGAGGCCTCAGTGCTGAGGGCCAAGCAAATATTTGTGGTTATGGATTAACTCGAACTCCAGGCTGTC
ATGGCGGCAGGACGGCG
W
ACTTGCAGTATCTCCACGACCCGCCCCTGTGAGTCCCCCTCCAGGCAGGTCTATGAGGGGTGTGGAGGGAGGGCTGCCCCCGG
GAGAAGAGAGCTAGGTG / Celera SNP ID: hCV1841975 / Public SNP ID: rs1799810 /
SNP Chromosome Position: 128176040 / SNP in Genomic Sequence: SEQ ID NO: 356 /
SNP Position Genomic: 74278 / SNP Source: Applera /
Population(Allele,Count): Caucasian (A,22|T,18) African American (A,9|T,25) total (A,31|T,43) / SNP Type: MISSENSE MUTATION;ESE;UTR5;SILENT RARE CODON;SILENT MUTATION / SNP Source: dbSNP; HapMap; HGBASE / Population(Allele,Count): Caucasian (A,73|T,39) / SNP Type: MISSENSE MUTATION;ESE;UTR5;SILENT RARE CODON;SILENT MUTATION /
Context (SEQ ID NO: 1010): /
CCCCATTCCAGGTGGTCCTGCTGGACTCAAAGAAGAAGCTGGCCTGCGGGGCAGTGCTCATCCACCCCTCCTGGGTGCTGACA
GCGGCCCACTGCATGGA
Y
GAGTCCAAGAAGCTCCTTGTCAGGCTTGGTATGGGCTGGAGCCAGGCAGAAGGGGGCTGCCAGAGGCCTGGGTAGGGGGACCA
GGCAGGCTGTTCAGGTT / Celera SNP ID: hCV1841983 / Public SNP ID: rs5937 / SNP Chromosome Position: 128184770 / SNP in Genomic Sequence: SEQ ID NO: 356 /
SNP Position Genomic: 83008 / SNP Source: CDX; dbSNP /
Population(Allele,Count): Caucasian (T,154|C,72) / SNP Type: ESS;ESE;SILENT MUTATION;INTRON /
Context (SEQ ID NO: 1011): /
TTAGATTTGACGAAATATGGAATATTACCTGTTGTGCTGATCTTGGGCAAACTATAATATCTCTGGGCAAAAATGTCCCCATC
TGAAAAACAGGGACAAC
R
TTCCTCCCTCAGCCAGCCACTATGGGGCTAAAATGAGACCACATCTGTCAAGGGTTTTGCCCTCACCTCCCTCCCTGCTGGAC
GGCATCCTTGGTGGGCA / Celera SNP ID: hCV15860236 / Public SNP ID: rs2069904 /
SNP Chromosome Position: 128175779 / SNP in Genomic Sequence: SEQ ID NO: 356 /
SNP Position Genomic: 74017 / Related Interrogated SNP: hCV1841975 / Related Interrogated SNP: hCV1841983 / SNP Source: Applera /
Population(Allele,Count): Caucasian (A,10|G,28) African American (A,14|G,22) total (A,24|G,50) // / SNP Type: TRANSCRIPTION FACTOR BINDING SITE;INTERGENIC;UNKNOWN / SNP Source: dbSNP; HapMap; HGBASE / Population(Allele,Count): Caucasian (G,153|A,73) / SNP Type: TRANSCRIPTION FACTOR BINDING SITE;INTERGENIC;UNKNOWN /
Context (SEQ ID NO: 1012): /
TGGAGCTGCTTTCTAGGCAGGCAGTGTGAGCTCAGCCCCACGTAGAGCGGGCAGCCGAGGCCTTCTGAGGCTATGTCTCTAGC
GAACAAGGACCCTCAAT
Y
CCAGCTTCCGCCCTGACGGCCAGCACACAGGGACAGCCCTTTCATTCCGCTTCCACCTGGGGGTGCAGGCAGAGCAGCAGCGG
GGGTAGGCACTGCCCGG / Celera SNP ID: hCV8810750 / Public SNP ID: rs1158867 /
SNP Chromosome Position: 128177377 / SNP in Genomic Sequence: SEQ ID NO: 356 /
SNP Position Genomic: 75615 / Related Interrogated SNP: hCV1841975 / Related

Interrogated SNP: hCV1841983 / SNP Source: Applera /
Population(Allele,Count): Caucasian (C,16|T,22) African American (C,32|T,4)
total (C,48|T,26) / SNP Type: UTR5;INTRON / SNP Source: dbSNP; HapMap;
HGBASE / Population(Allele,Count): Caucasian (C,92|T,134) / SNP Type:
UTR5;INTRON /
Context (SEQ ID NO: 1013): /
TCAGCTCATCGATATTGTTAGTGACTCTGCTTACTGTTTATTTATTGCAGAACTTTGAGACGGCCTTCATTAAGACCACTCTG
GAGCCCACCCTGTGTGC
R
CTTTTTCTCCGACTTCAGCAACTGCTAGATCAATGTATACATCTTATTTTTATTACACACATTCGGGCCCACAGCTCACTGCC
TGGCCCACTGGCTTATG / Celera SNP ID: hCV169044 / Public SNP ID: rs11691088 /
SNP Chromosome Position: 128131853 / SNP in Genomic Sequence: SEQ ID NO: 356 /
 SNP Position Genomic: 30091 / Related Interrogated SNP: hCV1841975 / Related
Interrogated SNP: hCV1841983 / SNP Source: dbSNP; Celera; HapMap /
Population(Allele,Count): Caucasian (A,87|G,33) / SNP Type: INTRON /
Context (SEQ ID NO: 1014): /
TAATTTTTTTCTGTCCAGTACTAGTACATAAAAAGTAAAGGTATCTGAAGCCATCTTTTAAATTTTTTTTAAAGCCAAGAAAA
TGATTTGGCAAAAGATA
Y
CGAAGAGCTTACGTTAGGGAAAACTAAACTGTATCTGTCTATGTGTTTAGAATTTATCCCATTAAAGAAACTGCCCAGTTAGT
CTCATTTTCTAAACGTA / Celera SNP ID: hCV273435 / Public SNP ID: rs7607907 /
SNP Chromosome Position: 128144286 / SNP in Genomic Sequence: SEQ ID NO: 356 /
 SNP Position Genomic: 42524 / Related Interrogated SNP: hCV1841975 / Related
Interrogated SNP: hCV1841983 / SNP Source: dbSNP; Celera; HapMap /
Population(Allele,Count): Caucasian (T,165|C,61) / SNP Type: INTRON /
Context (SEQ ID NO: 1015): /
TCTCCACCCACTCACTCCCTCAACTGTGAAGACCCCAGGCCCAGGCTACCGTCCACACTATCCAGCACAGCCTCCCCTACTCA
AATGCACACTGGCCTCA
Y
GGCTGCCCTGCCCCAACCCCTTTCCTGGTCTCCACAGCCAACGGGAGGAGGCCATGATTCTTGGGGAGGTCCGCAGGACACAT
GGGCCCCTAAAGCCACA / Celera SNP ID: hCV1721256 / Public SNP ID: rs2069910 /
SNP Chromosome Position: 128177974 / SNP in Genomic Sequence: SEQ ID NO: 356 /
 SNP Position Genomic: 76212 / Related Interrogated SNP: hCV1841983 / SNP
Source: dbSNP; Celera; HGBASE / Population(Allele,Count): Caucasian
(C,60|T,44) / SNP Type: INTRON /
Context (SEQ ID NO: 1016): /
CCCCACAGGTTCCTCAGGTGGGTTCTGGGCTAGGCCCTGCCCTGCTGGGGGCTCCACAGGGCTTGGCCTAGATTGGACGAGTC
GGGTGGGCAGCCCCTGG
Y
CTCTCCACTCAGGAGACTCCCCACCCACAGGGCTGCAGTCCTGCCTCAGGGCTCAGCAAGCAAACACGTAAGAGCACTGCACA
GTTGAAAAAATGAGTTT / Celera SNP ID: hCV8807993 / Public SNP ID: rs1473623 /
SNP Chromosome Position: 128196220 / SNP in Genomic Sequence: SEQ ID NO: 356 /
 SNP Position Genomic: 94458 / Related Interrogated SNP: hCV1841983 / Related
Interrogated SNP: hCV1841975 / SNP Source: dbSNP; HapMap; HGBASE /
Population(Allele,Count): Caucasian (T,36|C,64) / SNP Type: INTRON /
Context (SEQ ID NO: 1017): /
CCTAAAACAAAAGTTTACCAAAAAAAAAAAAAAAAAAGAAAGAAAAGAAAAGAAAAGAAAAAAAATTTCAAGACCTAGATCAGA
AATCAGCTCCTTGGCCA
R
GCATACTGGCTCATGCTTGAGCAAGGCAGGAGGATTGCTTGAGCCCAGGAATTTGAGACCAGCCTGGGCAATACTGTGTGACT
TCATCTCAAAAAAAAAA / Celera SNP ID: hCV9468542 / Public SNP ID: rs7599210 /
SNP Chromosome Position: 128164177 / SNP in Genomic Sequence: SEQ ID NO: 356 /
 SNP Position Genomic: 62415 / Related Interrogated SNP: hCV1841975 / Related
Interrogated SNP: hCV1841983 / SNP Source: dbSNP; HapMap /
Population(Allele,Count): Caucasian (G,130|A,92) / SNP Type:
INTERGENIC;UNKNOWN /
Context (SEQ ID NO: 1018): /
TGCACAGTTGAAAAAATGAGTTTTAAAACAATCTGCCAAGAAAAAAGATGTCAAAGTAGTCATCACGGGAAAAGTCAGAATTT
TGGTGGACTTCACTGCA
Y
TTATTCTGTGGCTATTAATTTTAAATTATGTATGTGGGGGAGGGGAGCACACCATAATCTTTCTAGTGCCCTGAGGCTCTTAA
ACCAGGCTCTGTTCACC / Celera SNP ID: hCV11263777 / Public SNP ID: rs11683986 /
SNP Chromosome Position: 128196398 / SNP in Genomic Sequence: SEQ ID NO: 356 /

SNP Position Genomic: 94636 / Related Interrogated SNP: hCV1841983 / Related Interrogated SNP: hCV1841975 / SNP Source: dbSNP; Celera; HapMap / Population(Allele,Count): Caucasian (C,155|T,71) / SNP Type: INTRON / Context (SEQ ID NO: 1019): /
AACAATCTGCCAAGAAAAAAGATGTCAAAGTAGTCATCACGGGAAAAGTCAGAATTTTGGTGGACTTCACTGCACTTATTCTG TGGCTATTAATTTTAAA
K
TATGTATGTGGGGGAGGGGAGCACACCATAATCTTTCTAGTGCCCTGAGGCTCTTAAACCAGGCTCTGTTCACCTGGACCTGT GTTCATGGGCAGGAAAC / Celera SNP ID: hCV11263778 / Public SNP ID: rs6749002 / SNP Chromosome Position: 128196424 / SNP in Genomic Sequence: SEQ ID NO: 356 / SNP Position Genomic: 94662 / Related Interrogated SNP: hCV1841983 / SNP Source: dbSNP; Celera; HapMap / Population(Allele,Count): Caucasian (T,115|G,111) / SNP Type: TRANSCRIPTION FACTOR BINDING SITE;INTRON / Context (SEQ ID NO: 1020): /
AGAGGGCTGGGATTACCGGTGTGAGCAACCATGCTGGACCCTCTAAGTGATCTTAAGAGATGCCTGCTCTTGCCTTCCCATGT TAGCCCCCTAATGTAAC
Y
GGCTCAGAGATGGACACAGCGAATACAGACAGAATGGGGATGTGCCTCCAAATCTGCCTGACTCCAGGAGGGGTCCCTTTAGT TGACATTGGCCCTGGAG / Celera SNP ID: hCV11263786 / Public SNP ID: rs13408910 / SNP Chromosome Position: 128196930 / SNP in Genomic Sequence: SEQ ID NO: 356 / SNP Position Genomic: 95168 / Related Interrogated SNP: hCV1841975 / Related Interrogated SNP: hCV1841983 / SNP Source: dbSNP; Celera / Population(Allele,Count): Caucasian (C,114|T,112) / SNP Type: INTRON / Context (SEQ ID NO: 1021): /
CAGTAGTCACCATGCTAAAATTAAACTAGCAGTGTCTCAATACAAAGCACAACAATTAATCCGCAGGCAAAGTGGATGGTCAC GTTCAAGATTATAGCGC
R
CTTAAGTTACATCACAAGGAGTGACATAATAATGGATGATCCACTATGGTGTACTACAAATCATGGTGGTTGTCCTCAGCAAC AGACACTAAGGAAGGTG / Celera SNP ID: hCV11266746 / Public SNP ID: rs6753288 / SNP Chromosome Position: 128169899 / SNP in Genomic Sequence: SEQ ID NO: 356 / SNP Position Genomic: 68137 / Related Interrogated SNP: hCV1841975 / Related Interrogated SNP: hCV1841983 / SNP Source: dbSNP; Celera; HapMap; ABI_Val / Population(Allele,Count): Caucasian (G,134|A,92) / SNP Type: INTERGENIC;UNKNOWN / Context (SEQ ID NO: 1022): /
TTATTCACCTCTTTCCATACTATACTCTTTGCAAGGAAGTCACTATGCTCAGCCCACACCTAAGGAGTGGGGAGCTATGCTCC ACCTCCTTGTGTGTGAA
R
TATCTGCATAAATTACTAGGAATTCTTCTGCATGTGAGATCTGTCTCTTCTCTCCTGTTTACTTATGTAATCTTTTATTTATA TTAATATAGAATTGTGG / Celera SNP ID: hCV11266765 / Public SNP ID: rs11679414 / SNP Chromosome Position: 128151003 / SNP in Genomic Sequence: SEQ ID NO: 356 / SNP Position Genomic: 49241 / Related Interrogated SNP: hCV1841975 / Related Interrogated SNP: hCV1841983 / SNP Source: dbSNP; Celera; HapMap / Population(Allele,Count): Caucasian (G,86|A,32) / SNP Type: TRANSCRIPTION FACTOR BINDING SITE;INTRON / Context (SEQ ID NO: 1023): /
GATCACATAATTCCCTCACTCATTCTGGATTTACTAGCACTCAGAGAGCTGAGATTGGAGCCTTAATATTAGCCTTAGAAACT TTCTCCACTCAGCTCAT
R
GATATTGTTAGTGACTCTGCTTACTGTTTATTTATTGCAGAACTTTGAGACGGCCTTCATTAAGACCACTCTGGAGCCCACCC TGTGTGCACTTTTTCTC / Celera SNP ID: hCV11266778 / Public SNP ID: rs2139142 / SNP Chromosome Position: 128131762 / SNP in Genomic Sequence: SEQ ID NO: 356 / SNP Position Genomic: 30000 / SNP Source: dbSNP; Celera; HapMap; ABI_Val; HGBASE / Population(Allele,Count): Caucasian (G,166|A,60) / SNP Type: INTRON / Context (SEQ ID NO: 1024): /
GCCTCCCTCCCCTTCTTCCTGAGCCCCTTCATGCAGAGCCCAGGACGCAATGGTCACAATGGCTAAAATCTCTTTGGAGGAGGG AGAGATCAGTGCAGGCT
R
AAGCCATTGGTGAGAGTGGAAGAATTCAGCTCTCCAGCGGAAGGCAGCAATATGCAGAGAGCCCTGTGATGGGGCTGCTGGAG CCGAGGCCTGGGGGTTC / Celera SNP ID: hCV28952331 / Public SNP ID: rs6755028 / SNP Chromosome Position: 128195442 / SNP in Genomic Sequence: SEQ ID NO: 356 / SNP Position Genomic: 93680 / Related Interrogated SNP: hCV1841983 / Related

Interrogated SNP: hCV1841975 / SNP Source: dbSNP; HapMap /
Population(Allele,Count): Caucasian (G,76|A,150) / SNP Type: INTRON /
Context (SEQ ID NO: 1025): /
CTGCAGAGAAGGTACCCAGGCCCTGGGCTCAGTGGCCCTTCCCCAGCTGGTGCCCCCAGCCTTACTGCCTGGCCCTCCTGCAC
CTTCTTTTCCTTGTCTA
R
GAACTGGGCTCATCCCCCTCTATCCCCAGTGGTTGTGGGGATGAAAGGAAGGCCAACCCCTGCAAGGCCTGGTGCCTGGAGCT
GGCCCTGGGTAGACGCC / Celera SNP ID: hCV28952333 / Public SNP ID: rs6754772 /
SNP Chromosome Position: 128195244 / SNP in Genomic Sequence: SEQ ID NO: 356 /
SNP Position Genomic: 93482 / Related Interrogated SNP: hCV1841983 / SNP
Source: dbSNP; HapMap / Population(Allele,Count): Caucasian (G,108|A,112) /
SNP Type: INTRON /
Context (SEQ ID NO: 1026): /
CATAATATCATAGTAGCTCTGTTTTCTTGATTGAGCCCTTAAATGATTCAAGTACTCAAGCTTCCTCCCAATCTACTGACCAA
GAAATCTCTGCAGCTTT
S
CCTCTGAGATCTTCTTGCATATATAACAAAATCTACATCATTTGGTCTCCAATTTGACTGCTTCCAAACAAACCTTGTTTTAT
CAGTTCTGCACTGTCCT / Celera SNP ID: hCV31814195 / Public SNP ID: rs11680949 /
SNP Chromosome Position: 128123562 / SNP in Genomic Sequence: SEQ ID NO: 356 /
SNP Position Genomic: 21800 / Related Interrogated SNP: hCV1841975 / Related
Interrogated SNP: hCV1841983 / SNP Source: dbSNP; HapMap /
Population(Allele,Count): Caucasian (C,91|G,29) / SNP Type: INTRON /
Context (SEQ ID NO: 1027): /
CCACTCTGGAGGAGAATGAGGGCTCTTCTGGTAGTTCTTTCCTTAAAGAACAGGAGCACGTTTCTTCCCCAGAAGTCTCCAAA
CTTATCCTCATATTTCA
Y
TGGGCTGAATTTGATTACAAACCCATTCCTGAAGTAAACCTTGGGCCAGGAAATGCCATGCACAAGTTGGCAAATGCAATGGG
ATCACTCTCTGAAAAAT / Celera SNP ID: hCV31814218 / Public SNP ID: rs6430938 /
SNP Chromosome Position: 128162623 / SNP in Genomic Sequence: SEQ ID NO: 356 /
SNP Position Genomic: 60861 / Related Interrogated SNP: hCV1841983 / Related
Interrogated SNP: hCV1841975 / SNP Source: dbSNP; ABI_Val /
Population(Allele,Count): Caucasian (C,151|T,71) / SNP Type: TRANSCRIPTION
FACTOR BINDING SITE;INTERGENIC;UNKNOWN /
Context (SEQ ID NO: 1028): /
GCAGTGATCTCTGCAGACTTGCGGGGGCGACGCCTGAAGCAAACAGGGACATACAAGCTGGTGCCTTCTGTGGTTGTGCATGG
GGTCTTCATGCTTCCTG
Y
CTGAGTTCCCAGAAGCTTGTCTCTGCTTTTCTAGGCAGCTGCCACAGCCTGTCACAAACAGCTCCTGGTTCTCCACTTCTCAT
AGTCTCGATTTCAAAAT / Celera SNP ID: hDV75209985 / Public SNP ID: rs2069898 /
SNP Chromosome Position: 128174227 / SNP in Genomic Sequence: SEQ ID NO: 356 /
SNP Position Genomic: 72465 / Related Interrogated SNP: hCV1841975 / Related
Interrogated SNP: hCV1841983 / SNP Source: CDX; dbSNP /
Population(Allele,Count): Caucasian (T,85|C,35) / SNP Type:
INTERGENIC;UNKNOWN //

Gene Number: 20 / Gene Symbol: SELE - 6401 / Gene Name: selectin E /
Chromosome: 1 / OMIM NUMBER: 131210 / OMIM Information: {Atherosclerosis,
susceptibility to} (2) // Genomic Sequence (SEQ ID NO: 357): // / SNP
Information /
Context (SEQ ID NO: 1029): /
CACAAGAGGACTTGTAGGTGAATTCTCCAATAGGGGAATGAGCACACCTCACCAAACCCTTCGGGGGCTGGTGGACAGCATCG
CATCTCACAGCTGGAAC
R
CACGAGAGAGCACTTTAGAAGTTTGTTTGCATCTCCAGCAATACGTTTCCCAAGGTAACCAAGTTCCCAAGCTCTTCAATAGT
TCTTTTTATCTTAAAAT / Celera SNP ID: hCV11975325 / Public SNP ID: rs5367 / SNP
Chromosome Position: 169697076 / SNP in Genomic Sequence: SEQ ID NO: 357 /
SNP Position Genomic: 15295 / Related Interrogated SNP: hCV11975250 / SNP
Source: Applera / Population(Allele,Count): Caucasian (A,37|G,3) African
American (A,32|G,2) total (A,69|G,5) / SNP Type: ACCEPTOR SPLICE
SITE;INTRON;PSEUDOGENE / SNP Source: dbSNP; HapMap; ABI_Val; HGBASE /
Population(Allele,Count): Caucasian (A,199|G,27) / SNP Type: ACCEPTOR SPLICE
SITE;INTRON;PSEUDOGENE /
Context (SEQ ID NO: 1030): /

CTCCCATTAGTTCAAATCCTTCTTCACAGTCAAATGTACAGGTTGTGTTCCATGGGAAGCTTCCAGGGTTTTGGAAACATTCC
ACGAACCCATTGGCTGG
R
TTTGTCACAGCATCACACTCAACCACTGAGGATTTTAAAGAGCACCATGAATTTTACAGAAGAATGATCTTTTCACTTCCTAT
TGAGCTGGGTGCCTAAC / Celera SNP ID: hCV11975329 / Public SNP ID: rs5363 / SNP
Chromosome Position: 169698789 / SNP in Genomic Sequence: SEQ ID NO: 357 /
SNP Position Genomic: 17008 / Related Interrogated SNP: hCV11975250 / SNP
Source: Applera / Population(Allele,Count): Caucasian (A,34|G,0) African
American (A,34|G,2) total (A,68|G,2) / SNP Type: ESE;SILENT MUTATION;INTRON /
SNP Source: dbSNP; HapMap; HGBASE / Population(Allele,Count): Caucasian
(A,199|G,27) / SNP Type: ESE;SILENT MUTATION;INTRON /
Context (SEQ ID NO: 1031): /
TATGACACCATCTGCACCAGGGAAGGGAAGGCATGCAGACCTGACTCTAATGCCAGCTAGGACGTGAGATGGTGCTACCATCT
CAAGTGAAGAAAGAGGC
R
AGAACCAGACTTACTTTGCTCACACTTGAGTCCACTGAAGCCAGGGTCACACTTGCAAGTGTAATTATTGATGGTCTCTACAC
ATTCACCGTGGCCACTG / Celera SNP ID: hCV11975331 / Public SNP ID: rs5362 / SNP
Chromosome Position: 169700961 / SNP in Genomic Sequence: SEQ ID NO: 357 /
SNP Position Genomic: 19180 / Related Interrogated SNP: hCV11975250 / SNP
Source: Applera / Population(Allele,Count): Caucasian (A,20|G,4) African
American (A,11|G,5) total (A,31|G,9) / SNP Type: MISSENSE MUTATION;ESS;INTRON
/ SNP Source: dbSNP; HapMap; HGBASE / Population(Allele,Count): Caucasian
(A,199|G,27) / SNP Type: MISSENSE MUTATION;ESS;INTRON /
Context (SEQ ID NO: 1032): /
CAAGAACCAGACTTACTTTGCTCACACTTGAGTCCACTGAAGCCAGGGTCACACTTGCAAGTGTAATTATTGATGGTCTCTAC
ACATTCACCGTGGCCAC
K
GCAGGATGTATTGGTACAGGCAGCTACGGAAAAATACAAAGCATGATGAGGAGGACTATTACTGTGCTTATACTGAGTGCCTTT
GATTTTAGAATCAACAG / Celera SNP ID: hCV11975332 / Public SNP ID: rs5361 / SNP
Chromosome Position: 169701060 / SNP in Genomic Sequence: SEQ ID NO: 357 /
SNP Position Genomic: 19279 / Related Interrogated SNP: hCV11975250 / SNP
Source: Applera / Population(Allele,Count): Caucasian (G,2|T,34) African
American (G,0|T,32) total (G,2|T,66) / SNP Type: MISSENSE MUTATION;INTRON /
SNP Source: dbSNP; HapMap; HGBASE / Population(Allele,Count): Caucasian
(T,199|G,27) / SNP Type: MISSENSE MUTATION;INTRON /
Context (SEQ ID NO: 1033): /
AGGATGCTGCCAGATATCCTGTGCAGGACAGCCCCAGACAAGCAAGGATATTTCAGTCTGAAATATCTATAGTGCGAGAATGA
GAAATCTTGGTCTAATG
R
CACTGACTTACCCAAAGTGAGAGCTGAGAGAAACTGTGAAGCAATCATGACTTCAAGAGTTCTTTTCACCCAAAGGTTTAGGC
TTGAAATACTTTCCTGG / Celera SNP ID: hCV2459459 / Public SNP ID: rs932307 /
SNP Chromosome Position: 169702705 / SNP in Genomic Sequence: SEQ ID NO: 357 /
SNP Position Genomic: 20924 / Related Interrogated SNP: hCV11975250 / SNP
Source: Applera / Population(Allele,Count): Caucasian (A,4|G,10) African
American (A,5|G,17) total (A,9|G,27) / SNP Type: INTRON;PSEUDOGENE / SNP
Source: dbSNP; Celera; HapMap; ABI_Val; HGBASE / Population(Allele,Count):
Caucasian (G,175|A,51) / SNP Type: INTRON;PSEUDOGENE /
Context (SEQ ID NO: 1034): /
TCACACTTGCAAGTGTAATTATTGATGGTCTCTACACATTCACCGTGGCCACTGCAGGATGTATTGGTACAGGCAGCTACGGA
AAATACAAAGCATGATG
R
GGAGGACTATTACTGTGCTTATACTGAGTGCCTTTGATTTTAGAATCAACAGTGTGCAACAGAGACATCAGCAGTCCTACAGA
GTGCCATAGACTTTAAC / Celera SNP ID: hCV25617131 / Public SNP ID: rs3917410 /
SNP Chromosome Position: 169701108 / SNP in Genomic Sequence: SEQ ID NO: 357 /
SNP Position Genomic: 19327 / Related Interrogated SNP: hCV11975250 / SNP
Source: Applera / Population(Allele,Count): Caucasian (A,38|G,2) African
American (A,36|G,2) total (A,74|G,4) / SNP Type: INTRON / SNP Source:
dbSNP; HapMap; HGBASE / Population(Allele,Count): Caucasian (A,189|G,27) / SNP
Type: INTRON /
Context (SEQ ID NO: 1035): /
GAACATTTTACCACTGCAAATGTTAGGTACACAGGCAGAGTTTCAGAAAAATCTACTGGAAAACTTCCAAAACTTGCTTAAAA
GTCAACAATGAATGTAA
W

GTGTAAGCGCTACTTAGTTTTCAGCATGTAGGAAATTAGGACCAAACCCCTTTGGGGCAATCTAGGTTCAGAAACTTTATGAA
GTATTTGACCTGTACCC / Celera SNP ID: hCV25617143 / Public SNP ID: rs3917425 /
SNP Chromosome Position: 169696754 / SNP in Genomic Sequence: SEQ ID NO: 357 /
SNP Position Genomic: 14973 / Related Interrogated SNP: hCV11975250 / SNP
Source: Applera / Population(Allele,Count): Caucasian (A,29|T,1) African
American (A,34|T,2) total (A,63|T,3) / SNP Type: INTRON;PSEUDOGENE / SNP
Source: dbSNP; HapMap; HGBASE / Population(Allele,Count): Caucasian
(A,199|T,27) / SNP Type: INTRON;PSEUDOGENE /
Context (SEQ ID NO: 1036): /
TCCACATGCCCACATCTTTTTCTCTCTTGATGTAGATCTCCACGCAGTCCTCATCTTTTTGCCTATTGTTGGGTTCACCTGGA
GCCCAGTTCTTGGCTTC
Y

TCTGTCAGAGGTTTCTGGGTTCCTACCCAGACCCACACATTGTTGACTTTTCTGATTCCAATCCAGTAATAACTTGGTGAATA
GCTCAATATGGAGTTTA / Celera SNP ID: hCV8919527 / Public SNP ID: rs1800016 /
SNP Chromosome Position: 169701901 / SNP in Genomic Sequence: SEQ ID NO: 357 /
SNP Position Genomic: 20120 / Related Interrogated SNP: hCV11975250 / SNP
Source: Applera / Population(Allele,Count): Caucasian (C,0|T,32) African
American (C,2|T,36) total (C,2|T,68) / SNP Type: TRANSCRIPTION FACTOR BINDING
SITE;ESE SYNONYMOUS;SILENT MUTATION;INTRO / N // SNP Source: Applera /
Population(Allele,Count): Caucasian (C,0|T,4) African American (C,2|T,30) total
(C,2|T,34) / SNP Type: TRANSCRIPTION FACTOR BINDING SITE;ESE SYNONYMOUS;SILENT
MUTATION;INTRO / N // SNP Source: dbSNP; HGBASE / Population(Allele,Count):
Caucasian (T,198|C,28) / SNP Type: TRANSCRIPTION FACTOR BINDING SITE;ESE
SYNONYMOUS;SILENT MUTATION;INTRO / N //
Context (SEQ ID NO: 1037): /
ACATGCCCACATCTTTTTCTCTCTTGATGTAGATCTCCACGCAGTCCTCATCTTTTTGCCTATTGTTGGGTTCACCTGGAGCC
CAGTTCTTGGCTTCTTC
Y

GTCAGAGGTTTCTGGGTTCCTACCCAGACCCACACATTGTTGACTTTTCTGATTCCAATCCAGTAATAACTTGGTGAATAGCT
CAATATGGAGTTTAGGT / Celera SNP ID: hCV8919528 / Public SNP ID: rs1800015 /
SNP Chromosome Position: 169701904 / SNP in Genomic Sequence: SEQ ID NO: 357 /
SNP Position Genomic: 20123 / Related Interrogated SNP: hCV11975250 / SNP
Source: Applera / Population(Allele,Count): Caucasian (C,0|T,2) African
American (C,2|T,28) total (C,2|T,30) / SNP Type: ESE SYNONYMOUS;SILENT RARE
CODON;SILENT MUTATION;INTRON / SNP Source: Applera / Population(Allele,Count):
Caucasian (C,0|T,8) African American (C,2|T,28) total (C,2|T,36) / SNP Type:
ESE SYNONYMOUS;SILENT RARE CODON;SILENT MUTATION;INTRON / SNP Source: dbSNP;
HGBASE / Population(Allele,Count): Caucasian (T,199|C,27) / SNP Type: ESE
SYNONYMOUS;SILENT RARE CODON;SILENT MUTATION;INTRON /
Context (SEQ ID NO: 1038): /
GTAGTTGTTCTCTCTTATTTCCCAGAGTTTTTCTGCCCCTTTAAAAGAACCTCTGCTGTTCTGATCCTTATCACATCTCTGTT
TTGACTGTTGGCTTTGT
Y

GTTGCCAGTGTTCAGCCAGAACTTCTCTGAAACTTTTTTTTTCAACACATGCTAAGTTAATGGAAGTGTAGGAGAGTTTTTGATT
CTCACACTCCTCAAGGC / Celera SNP ID: hCV2459446 / Public SNP ID: rs4786 / SNP
Chromosome Position: 169692132 / SNP in Genomic Sequence: SEQ ID NO: 357 /
SNP Position Genomic: 10351 / Related Interrogated SNP: hCV11975250 / SNP
Source: dbSNP; Celera; HapMap; HGBASE / Population(Allele,Count): Caucasian
(T,169|C,53) / SNP Type: MICRORNA;UTR3;INTRON /
Context (SEQ ID NO: 1039): /
CTTCCATGCTCAGGGGATTCCAGGGCTGTACAGTTCACAACTGAAAAAGAAACCCAAATCAGTTCTGCTCATCTCTCACCTTT
AACAGATAAGAACACTG
R

AAACTAGAACTACAGTTTGGTTTTTTTTTTTTTTTAGTTTAAAAATTTATAAAATTTCTAATGGAATTTGTAAAATTGACTGTA
ATTCTACCCCTTTTCTT / Celera SNP ID: hCV2459453 / Public SNP ID: rs3917419 /
SNP Chromosome Position: 169699819 / SNP in Genomic Sequence: SEQ ID NO: 357 /
SNP Position Genomic: 18038 / Related Interrogated SNP: hCV11975250 / SNP
Source: dbSNP; Celera; HapMap / Population(Allele,Count): Caucasian
(G,127|A,99) / SNP Type: INTRON /
Context (SEQ ID NO: 1040): /
GTTTAAGGCAGCATCCTAAGATTTTGGGCAAAGGACACTAGTGCAATAATCTTTATTTCAGAGTTTAATCAAATAAATAAACA
AATTTTAAGACTTTCAT
Y

ATTTAGGTCAAAGAGAAAAGACAGGTTTTAGCTACAATACAATAAGAGCTTGTACAGATGTGGTTTTTATTAGAAGGCCTTTT
GCATATCTGTGTTTCAT / Celera SNP ID: hCV2459460 / Public SNP ID: rs5353 / SNP
Chromosome Position: 169702974 / SNP in Genomic Sequence: SEQ ID NO: 357 /
SNP Position Genomic: 21193 / Related Interrogated SNP: hCV11975250 / SNP
Source: dbSNP; Celera; HapMap; HGBASE / Population(Allele,Count): Caucasian
(T,165|C,51) / SNP Type: INTRON;PSEUDOGENE /
Context (SEQ ID NO: 1041): /
TATAGTGCGAGAATGAGAAATCTTGGTCTAATGGCACTGACTTACCCAAAGTGAGAGCTGAGAGAAACTGTGAAGCAATCATG
ACTTCAAGAGTTCTTTT
M

ACCCAAAGGTTTAGGCTTGAAATACTTTCCTGGGGAGATAAAACACAAAATGAATTAAAGAAGGAAATCGTGGGTAGCTAGTT
ACATTATTCTACCATGA / Celera SNP ID: hCV8919530 / Public SNP ID: rs1805193 /
SNP Chromosome Position: 169702772 / SNP in Genomic Sequence: SEQ ID NO: 357 /
SNP Position Genomic: 20991 / Related Interrogated SNP: hCV11975250 / SNP
Source: dbSNP; HapMap; HGBASE / Population(Allele,Count): Caucasian
(C,199|A,27) / SNP Type: UTR5;INTRON;PSEUDOGENE /
Context (SEQ ID NO: 1042): /
TGTTTGCATTTATGCTTTTATTAGTTCAAAACGTTTGGCCTCATGGAAGTTTTTCATCGTGGAAACCACATATTTCTGAAAAA
ATATCTGACAATATACA
R

ACCTTCCATTCAGTTTTTACTCTCCAATTCTACCATGTTTTCAAAAAACAACTGTAGTAAAAACACTCAGAACTTTATTCTGG
TTAACATCATGCCTTGC / Celera SNP ID: hCV11975322 / Public SNP ID: rs5357 / SNP
Chromosome Position: 169692713 / SNP in Genomic Sequence: SEQ ID NO: 357 /
SNP Position Genomic: 10932 / Related Interrogated SNP: hCV11975250 / SNP
Source: dbSNP; HapMap; HGBASE / Population(Allele,Count): Caucasian
(A,109|G,11) / SNP Type: MICRORNA;UTR3;INTRON /
Context (SEQ ID NO: 1043): /
ACTGTTGTGGTTTAACTCTGACAACTGTGCTTTTTATTGTCTTATTTTTGGCAATGTTTGTGACATGGCCCAGACTTTTCTCA
TCTTTTCAAAAGTAAGA
R

GTACGTATGAAGAAACAGCGACTTATTGTTTATCTCTTTTGTGACTGCCACCCACTAGGTACCTTATCCACACTCACTCACAA
CATTATAGTATACCCAT / Celera SNP ID: hCV27886249 / Public SNP ID: rs3917406 /
SNP Chromosome Position: 169702278 / SNP in Genomic Sequence: SEQ ID NO: 357 /
SNP Position Genomic: 20497 / Related Interrogated SNP: hCV11975250 / SNP
Source: dbSNP; HapMap; HGBASE / Population(Allele,Count): Caucasian
(A,97|G,23) / SNP Type: INTRON /
Context (SEQ ID NO: 1044): /
CACAACCAATACCACAGCCCAGATCCTCAGCTCTCCAATGACATTTTCCTCCACTAGACTTGAGCTACCTCCTTCCCTAGGCA
CAGCCTCAACCTCGACA
R

CACCTAAGACTGTACCGTCTCTAAAGTCACATGTTCAAACACTTCACTCTTTAACCACTGTCTCCTATTCTTGCAAGTGTATT
GCTCAAGTATCTCATTG / Celera SNP ID: hCV28023624 / Public SNP ID: rs4656704 /
SNP Chromosome Position: 169688228 / SNP in Genomic Sequence: SEQ ID NO: 357 /
SNP Position Genomic: 6447 / Related Interrogated SNP: hCV11975250 / SNP
Source: dbSNP; HapMap; HGBASE / Population(Allele,Count): Caucasian
(A,173|G,53) / SNP Type: INTRON /
Context (SEQ ID NO: 1045): /
AAGTAATAATCTATAATTATTTATTACAATTAAAGGAAGGAAGAGACATATGGATTATGAGGGCATTAAAGAGGAGACCTAGT
GTAAGTAGCCAGTTCTC
R

TGAAGGGACATGTATTAGTTGGAGTTCTCCAGAGAAACAGAACCAATGGTGTGTGTGTGTGTGTGTGCGTGTGTGCGTGTGTG
TGTTGGGGTGTGGGGGT / Celera SNP ID: hCV30036721 / Public SNP ID: rs3917449 /
SNP Chromosome Position: 169691219 / SNP in Genomic Sequence: SEQ ID NO: 357 /
SNP Position Genomic: 9438 / Related Interrogated SNP: hCV11975250 / SNP
Source: dbSNP; HapMap; HGBASE / Population(Allele,Count): Caucasian
(A,96|G,173) / SNP Type: INTRON /
Context (SEQ ID NO: 1046): /
ATCTCAAAATTAAAAAAAAAAAAAAAGTAAAAAGAAAAGATAAGAAATATAGTACCAGCCCCTATCTCAGAGTTCCTAGCTTAGA
AAAATTCCCAGAATATA
R

TAAGTGCAATGTAAGGGTCAGCTATCTTCATTATTATTATCTATCATAAATGAAATTACACAATAAAGCTAGATCCGTTTCTT
TCCTCTCCTTCTACAAA / Celera SNP ID: hCV32141631 / Public SNP ID: rs3917436 /
SNP Chromosome Position: 169694619 / SNP in Genomic Sequence: SEQ ID NO: 357 /

SNP Position Genomic: 12838 / Related Interrogated SNP: hCV11975250 / SNP Source: dbSNP; HGBASE / Population(Allele,Count): Caucasian (A,171|G,53) / SNP Type: INTRON /
Context (SEQ ID NO: 1047): /
GCAGTGATGACAACCATAAAAAAGAAATATTGAGTGATATGGGGAGAGTAGTGTAATTGTGTTTACCTCAAAACTGTTCAAAT
TATATGAACAAACACAG
Y
AAACTTAGGTACCACAACAAATTTCTTGTTACTTTTCTCACAACTGCTAAAAATACTACAGTAAGCTTCCAACCAGGATGAGA
ACCATTCACAAAGCTAT / Celera SNP ID: hCV32398748 / Public SNP ID: rs3917417 /
SNP Chromosome Position: 169700062 / SNP in Genomic Sequence: SEQ ID NO: 357 /
SNP Position Genomic: 18281 / Related Interrogated SNP: hCV11975250 / SNP
Source: dbSNP; HGBASE / Population(Allele,Count): Caucasian (C,106|T,8) / SNP
Type: INTRON;PSEUDOGENE /
Context (SEQ ID NO: 1048): /
TACAGTTTATTGAATGTGTCCTGTGTGCCAGGCACCATGTAACCATGAGCCTATGAAGTTCACACTATTATTATCCTCATTTT
ACAATGAGAAAACTGAC
W
TAGAGAGTTAAACTATCTTGTCAAGGTGCCAAAATAAATAACTGGTGAATCTAGGACTCAAACCCAGCAGGGTCTGACTTCAT
AGTCTCAGCTCACGATC / Celera SNP ID: hCV32141639 / Public SNP ID: rs3917411 /
SNP Chromosome Position: 169700756 / SNP in Genomic Sequence: SEQ ID NO: 357 /
SNP Position Genomic: 18975 / Related Interrogated SNP: hCV11975250 / SNP
Source: dbSNP; HGBASE / Population(Allele,Count): Caucasian (A,199|T,27) /
SNP Type: MICRORNA;UTR3;INTRON /
Context (SEQ ID NO: 1049): /
TTAGTAGTGTCTCTCATGTAGTTGTGGTAGTAATTAGAATTTCAGAAACAGAAGGAAACCAAGAATAGGTTTGTCATCCATAG
TCTACTACCTTCAATTT
M
TCATTCATAGCTGTGGATAACCAATCACTACTCATTTTTTCTTCCTTTTTCACCTGCCAATTCAACATATTTAACATGCACTG
TCTCACAGAGGAATGAC / Celera SNP ID: hCV32141645 / Public SNP ID: rs3917452 /
SNP Chromosome Position: 169703617 / SNP in Genomic Sequence: SEQ ID NO: 357 /
SNP Position Genomic: 21836 / Related Interrogated SNP: hCV11975250 / SNP
Source: dbSNP; HapMap; HGBASE / Population(Allele,Count): Caucasian
(C,197|A,27) / SNP Type: INTRON /
Context (SEQ ID NO: 1050): /
CTATATATATATATTTAATTGGTCAAAATTTTGTTTAAAATTTTTGAAATGTTAATGTGCAAAGAATAAAAATTCTTCCACAA
TGTTAACAGTGACTAAC
Y
CTGGATGGCAGGATTTGGGATAATTTTTATATCCTTCATTATTATTTTCAGGATTTTAAAGTTTTTTTTCAATTTCCCTTTTTT
TCACCTTTATAGTAACA / Celera SNP ID: hCV32398763 / Public SNP ID: rs3917392 /
SNP Chromosome Position: 169704335 / SNP in Genomic Sequence: SEQ ID NO: 357 /
SNP Position Genomic: 22554 / Related Interrogated SNP: hCV11975250 / SNP
Source: dbSNP; HGBASE / Population(Allele,Count): Caucasian (T,199|C,27) /
SNP Type: INTRON /
Context (SEQ ID NO: 1051): /
CTACTCAGATTTAAATCTCAGCAGGGAAGTAAACCTTAGTTTTTACATGAGAAAATGTTACAGCAGCCTTCTCGGCTTCCTTT
ACCCCCATCCCAGTTTC
R
CGAGCTTAGTGCCTTAGATCGGGTTCCTTTAGAAGCAGACCTCGAAATAAGGATGTGGGTGCCAGTCATTTATTGAAAAGATG
ATCCCAAGAAAGCCTAG / Celera SNP ID: hCV30631277 / Public SNP ID: rs10489182 /
SNP Chromosome Position: 169710669 / SNP in Genomic Sequence: SEQ ID NO: 357 /
SNP Position Genomic: 28888 / Related Interrogated SNP: hCV11975250 / SNP
Source: dbSNP; HapMap / Population(Allele,Count): Caucasian (A,99|G,19) / SNP
Type: TRANSCRIPTION FACTOR BINDING SITE;INTRON /
Context (SEQ ID NO: 1052): /
TTACCCATTGTCCACTTTTCCCAAACTACATAGTGTTATATGGTATATGACCCAATCAACGGTGGCAAAGCTCCAGAAATACC
ACATAGACATCAGGGAC
R
CTTTAAACTAATCAGCCTATAGTCCTTTTTCAGTAATTTCCAAACCTGGTTGTGCATCCAAATCACTTGGTAACATTAAAAAA
ACAAAAAAATATACACG / Celera SNP ID: hCV32141621 / Public SNP ID: rs12133642 /
SNP Chromosome Position: 169689800 / SNP in Genomic Sequence: SEQ ID NO: 357 /
SNP Position Genomic: 8019 / Related Interrogated SNP: hCV11975250 / SNP
Source: dbSNP; HapMap / Population(Allele,Count): Caucasian (A,199|G,27) /
SNP Type: INTRON /

Context (SEQ ID NO: 1053): /
GTGTTATATGGTATATGACCCAATCAACGGTGGCAAAGCTCCAGAAATACCACATAGACATCAGGGACACTTTAAACTAATCA
GCCTATAGTCCTTTTTC
W
GTAATTTCCAAACCTGGTTGTGCATCCAAATCACTTGGTAACATTAAAAAAACAAAAAAATATACACGCAACATTCGCTCCCA
ATCCTACTGAATCAGAA / Celera SNP ID: hCV32141622 / Public SNP ID: rs12133666 /
SNP Chromosome Position: 169689832 / SNP in Genomic Sequence: SEQ ID NO: 357 /
SNP Position Genomic: 8051 / Related Interrogated SNP: hCV11975250 / SNP
Source: dbSNP; HapMap / Population(Allele,Count): Caucasian (A,87|T,9) / SNP
Type: INTRON /
Context (SEQ ID NO: 1054): /
TTCCTGGGGAGATAAAACACAAAATGAATTAAAGAAGGAAATCGTGGGTAGCTAGTTACATTATTCTACCATGATGTTTAAGG
CAGCATCCTAAGATTTT
R
GGCAAAGGACACTAGTGCAATAATCTTTATTTCAGAGTTTAATCAAATAAATAAACAAATTTTAAGACTTTCATTATTTAGGT
CAAAGAGAAAAGACAGG / Celera SNP ID: hDV76908547 / Public SNP ID: rs3917400 /
SNP Chromosome Position: 169702899 / SNP in Genomic Sequence: SEQ ID NO: 357 /
SNP Position Genomic: 21118 / Related Interrogated SNP: hCV11975250 / SNP
Source: CDX; dbSNP / Population(Allele,Count): Caucasian (G,109|A,9) / SNP
Type: INTRON;PSEUDOGENE /
Context (SEQ ID NO: 1055): /
TGGACTCAGTGGGAGCTAAGGAAGTAAGAGACGAAGAAAGGTCATGAGGAAGAATTGATGTTAAAGTCTCTCCGTCCTGTCCC
TTTGGCCTTTTTTCTGT
R
CATTCATTACTAGGAGCAGAAGAGCTATCTAGTTTAATACAAGAAGCAGAGATGTGGCATTACAGGCCTTTGAGATCTGCTCC
AAGCCACCTTTGAAGCT / Celera SNP ID: hDV76908557 / Public SNP ID: rs3917427 /
SNP Chromosome Position: 169696033 / SNP in Genomic Sequence: SEQ ID NO: 357 /
SNP Position Genomic: 14252 / Related Interrogated SNP: hCV11975250 / SNP
Source: CDX; dbSNP / Population(Allele,Count): Caucasian (A,199|G,27) / SNP
Type: INTRON /
Context (SEQ ID NO: 1056): /
GCCTGATCACAGTAGTTGTTCTCTCTTATTTCCCAGAGTTTTTCTGCCCCTTTAAAAGAACCTCTGCTGTTCTGATCCTTATC
ACATCTCTGTTTTGACT
S
TTGGCTTTGTTGTTGCCAGTGTTCAGCCAGAACTTCTCTGAAACTTTTTTTTTCAACACATGCTAAGTTAATGGAAGTGTAGGA
GAGTTTTGATTCTCACA / Celera SNP ID: hDV76908563 / Public SNP ID: rs3917441 /
SNP Chromosome Position: 169692121 / SNP in Genomic Sequence: SEQ ID NO: 357 /
SNP Position Genomic: 10340 / Related Interrogated SNP: hCV11975250 / SNP
Source: CDX; dbSNP / Population(Allele,Count): Caucasian (G,109|C,9) / SNP
Type: MICRORNA;UTR3;INTRON /
Context (SEQ ID NO: 1057): /
GACATAGAGAGTTAAACTATCTTGTCAAGGTGCCAAAATAAATAACTGGTGAATCTAGGACTCAAACCCAGCAGGGTCTGACT
TCATAGTCTCAGCTCAC
R
ATCACCATATGACACCATCTGCACCAGGGAAGGGAAGGCATGCAGACCTGACTCTAATGCCAGCTAGGACGTGAGATGGTGCT
ACCATCTCAAGTGAAGA / Celera SNP ID: hDV76908571 / Public SNP ID: rs3917454 /
SNP Chromosome Position: 169700853 / SNP in Genomic Sequence: SEQ ID NO: 357 /
SNP Position Genomic: 19072 / Related Interrogated SNP: hCV11975250 / SNP
Source: CDX; dbSNP / Population(Allele,Count): Caucasian (G,213|A,13) / SNP
Type: MICRORNA;UTR3;INTRON /
Context (SEQ ID NO: 1058): /
TAACTCAAAAATGTTTTAAGTACTCTTAAACATGTAAGCCAAACAAACCATGAGTGTAGTCAGATGTGCTTCCATATTCCTTA
TGAGAGACTCTCAAATT
W
AAGCCTGTACTCCAAATAAATCTCCTTAGGAAGAATTTTATCCATTTTCCTTAGAGTGCTCATCATGGCAGTTCCATTGCACA
ATTCCGGGAGGCATCAT / Celera SNP ID: hDV76908576 / Public SNP ID: rs3917461 /
SNP Chromosome Position: 169695491 / SNP in Genomic Sequence: SEQ ID NO: 357 /
SNP Position Genomic: 13710 / Related Interrogated SNP: hCV11975250 / SNP
Source: CDX; dbSNP / Population(Allele,Count): Caucasian (T,114|A,6) / SNP
Type: INTRON /
Context (SEQ ID NO: 1059): /
GATCTCTCAAGATGACAATATTTATTCTCCACACAGCACATACTTCAGGGTTGGAAGGCAGGGGCAATCTTCTCCTTTATAAT
GAGTGCCTCTTATATAT

S

TTTATTCATCTGCCCTCTTGTAAAACACACACACACACACACACAAAGAAGAAATAAAATAACTCTGCTTCTTTGAAGCTTGT
GACACTGAGATAAACCA / Celera SNP ID: hDV77030725 / Public SNP ID: rs4656701 /
SNP Chromosome Position: 169687903 / SNP in Genomic Sequence: SEQ ID NO: 357 /
SNP Position Genomic: 6122 / Related Interrogated SNP: hCV11975250 / SNP
Source: CDX; dbSNP / Population(Allele,Count): Caucasian (G,173|C,53) / SNP
Type: INTRON /
Context (SEQ ID NO: 1060): /
CTCCTCATGCAAGATTGGCTTTGGCACCTAGTTCCTGATCTTCCTTTCCCTGTAAGCACTTCTCATAGTCTTACGGGACTTCA
CCATCCATGGCACAACC

M

ATACCACAGCCCAGATCCTCAGCTCTCCAATGACATTTTCCTCCACTAGACTTGAGCTACCTCCTTCCCTAGGCACAGCCTCA
ACCTCGACAACACCTAA / Celera SNP ID: hDV77030727 / Public SNP ID: rs4656703 /
SNP Chromosome Position: 169688135 / SNP in Genomic Sequence: SEQ ID NO: 357 /
SNP Position Genomic: 6354 / Related Interrogated SNP: hCV11975250 / SNP
Source: CDX; dbSNP / Population(Allele,Count): Caucasian (A,173|C,53) / SNP
Type: INTRON //

Gene Number: 21 / Gene Symbol: SELL - 6402 / Gene Name: selectin L /
Chromosome: 1 / OMIM NUMBER: 153240 / OMIM Information: // Genomic Sequence
(SEQ ID NO: 358): // / SNP Information /
Context (SEQ ID NO: 1061): /
TTCAGTTTCTTCATCTATATAAGAGGAATAATGAAATTGTGTTATCTTTATCAAATTGATATGGAAACTAAATGTAATTCAAT
TAGCATAAGTCAAGGAC

S

TTAGAACAAAGCCTGACTCATCAGAAATTCTAAGTAAACATTAGCTAGTCTTCATATTATTATCTTCAGCATTATCTGTAGTG
AGAATCCTTAAAGCCAA / Celera SNP ID: hCV25619707 / Public SNP ID: rs4987308 /
SNP Chromosome Position: 169674116 / SNP in Genomic Sequence: SEQ ID NO: 358 /
SNP Position Genomic: 24310 / Related Interrogated SNP: hCV11975250 / SNP
Source: Applera / Population(Allele,Count): Caucasian (C,38|G,2) African
American (C,36|G,2) total (C,74|G,4) / SNP Type: TRANSCRIPTION FACTOR BINDING
SITE;INTRON / SNP Source: dbSNP; HapMap / Population(Allele,Count): Caucasian
(C,109|G,11) / SNP Type: TRANSCRIPTION FACTOR BINDING SITE;INTRON /
Context (SEQ ID NO: 1062): /
TGCCTGCATCTTTGTTTCTCTTGATATAGATCTCCACGCAGTCCTCCTTGTTCTTCTTGTTGTTGGGCTCACCATCTCCCCAG
TTCTCTGCTTCTTCAGT

R

AGAGATTTGTTGGTTCCCACCCACGTCCATATTCCTCCTATCTTCCGGATTCCTATCCAGTAGTAAGAACGACTGAAAGGCAG
AGTCTTCTCCAGATACT / Celera SNP ID: hCV8919509 / Public SNP ID: rs1051091 /
SNP Chromosome Position: 169677709 / SNP in Genomic Sequence: SEQ ID NO: 358 /
SNP Position Genomic: 27903 / Related Interrogated SNP: hCV11975250 / SNP
Source: Applera / Population(Allele,Count): Caucasian (A,27|G,11) African
American (A,29|G,9) total (A,56|G,20) / SNP Type: ESE SYNONYMOUS;SILENT
MUTATION;INTRON / SNP Source: dbSNP; HapMap; HGBASE / Population(Allele,Count):
Caucasian (A,176|G,50) / SNP Type: ESE SYNONYMOUS;SILENT MUTATION;INTRON /
Context (SEQ ID NO: 1063): /
AACTCTATAGACACTGTCTAGCTATATGAACTTAGACAAACTAATATCTCTGAGCTTCAGTTTCTTAAAATTTAAAATGAGGA
CAATACCATCTATGGCC

R

GGGATTAAATGCTATGAGGAATGTAAACCAGATGTCAGGTACCATCTCTCTAAAATCCAGATAAAATGAATTAAAAATACTGG
CCGCAAACCCTCTCTAA / Celera SNP ID: hCV2459408 / Public SNP ID: rs7531806 /
SNP Chromosome Position: 169651044 / SNP in Genomic Sequence: SEQ ID NO: 358 /
SNP Position Genomic: 1238 / Related Interrogated SNP: hCV11975250 / SNP
Source: dbSNP; Celera; HapMap / Population(Allele,Count): Caucasian
(G,94|A,132) / SNP Type: INTRON /
Context (SEQ ID NO: 1064): /
TGGTATTTTTGAGATTTGGCCTAAAACATCATTGCCCTGGTTTCCTTATTTACCAAACAGGGCCAATGGTAGTGACTAATCAG
AAAATGATAATGCCTGG

W

GCACAAAATGTGTCTAGATGAGCCCATGCACAAGGACACATGTTTCTGGAACTGTTCCTTATTCCTTTCCTAAAAGAAAGGAG
GGAAAGTCTCCATACTA / Celera SNP ID: hCV2459420 / Public SNP ID: rs4987351 /
SNP Chromosome Position: 169667355 / SNP in Genomic Sequence: SEQ ID NO: 358 /
SNP Position Genomic: 17549 / Related Interrogated SNP: hCV11975250 / SNP

Source: dbSNP; Celera; HapMap / Population(Allele,Count): Caucasian (T,112|A,110) / SNP Type: INTRON /
Context (SEQ ID NO: 1065): /
TTCCATGATGTGCCAGGAAATCTGCAAGACATCAGTGTGACCTATGCAGACTTACATAATGTTACAGCTAAAAAGAACCTAGC
ACTACTCCAGGCTGAGC
Y
AGACACTTAGAGATGAGGAAACAGAGCCTAAGAGTGTATGTGACCATCTCAGGATCACAGAATAGTTGTTTGCAGATTTGAAG
TAGAACCTAGACCTTCT / Celera SNP ID: hCV2459428 / Public SNP ID: rs4987285 /
SNP Chromosome Position: 169678024 / SNP in Genomic Sequence: SEQ ID NO: 358 /
SNP Position Genomic: 28218 / Related Interrogated SNP: hCV11975250 / SNP
Source: dbSNP; Celera / Population(Allele,Count): Caucasian (T,173|C,53) /
SNP Type: INTRON /
Context (SEQ ID NO: 1066): /
GTGTCTGTCATCAAGTACAGCACTGAATTGAAACTGAAAACAAGAGTCAAGAAAGAGCAAAGTCAGCCATCTTTATATTCCAC
ATGAATCCTTTCCCTTT
R
TGGTCTTATTTGTTTCTCCTCAGAAAAGACAAAAAGCTGAGCTGTATAAACACCTGTGGGCTGGGGGTTGAGGGATAAATGAG
GGGCGAAATGGAAGCTG / Celera SNP ID: hCV8919501 / Public SNP ID: rs909628 /
SNP Chromosome Position: 169660665 / SNP in Genomic Sequence: SEQ ID NO: 358 /
SNP Position Genomic: 10859 / Related Interrogated SNP: hCV11975250 / SNP
Source: dbSNP; Celera; HapMap; ABI_Val; HGBASE / Population(Allele,Count):
Caucasian (A,199|G,27) / SNP Type: TRANSCRIPTION FACTOR BINDING
SITE;MICRORNA;UTR3;INTRON /
Context (SEQ ID NO: 1067): /
TATTTAATATTTACTATGTATATTTTTTAAAGTATTGTTGCTGCTTAATTAACTATTGATGCTTATATTTAATGTTATAGCCT
CACTCTTGATCATAATG
R
GTCAATGCCTCAAATACCTAAAAAAAAAAAAAAAAATTAGATAGCCAGACACCAGGAAAGAAAAGTATTTCTTTTTTTTAATAAAAA
GAAATACCTTTTTGAGC / Celera SNP ID: hCV8919515 / Public SNP ID: rs1569457 /
SNP Chromosome Position: 169680074 / SNP in Genomic Sequence: SEQ ID NO: 358 /
SNP Position Genomic: 30268 / Related Interrogated SNP: hCV11975250 / SNP
Source: dbSNP; HapMap; HGBASE / Population(Allele,Count): Caucasian
(G,109|A,11) / SNP Type: TRANSCRIPTION FACTOR BINDING SITE;INTRON /
Context (SEQ ID NO: 1068): /
AAATGATGACCTGACCTCAATCATTTCTGCATGCAATTATTTCTTGGCAATCCCTTTCTTTATAGAAATCAAAGATTAAAAAG
TCCAAATTTGCTAAAAC
R
GTAGAGTCCAATTTATAAGAGACCAAATTAACTATGGTTCATTATTAAAACATCACTTGGAAAATGCTGGCTGTTTTGGAATT
GTAGAAGATTTTACAGA / Celera SNP ID: hCV11975318 / Public SNP ID: rs1883228 /
SNP Chromosome Position: 169677000 / SNP in Genomic Sequence: SEQ ID NO: 358 /
SNP Position Genomic: 27194 / Related Interrogated SNP: hCV11975250 / SNP
Source: dbSNP; Celera / Population(Allele,Count): Caucasian (G,168|A,46) /
SNP Type: TRANSCRIPTION FACTOR BINDING SITE;INTRON /
Context (SEQ ID NO: 1069): /
TGGCATAGTTTCTTCCAGACCTTAGAGTTCTAATTAATCTAACAAGCTCATTAGATCGTGAGCTTCTTGAGAGCGGGAATCTA
CCATGCTAATTCCTTAT
K
GTAACCCTGACAGCTTTTATCCCAACACTGTGCTTCTTGTGGTACTCAAAAAGACTTGTTGAGAAGTGAGTCGAAACTTCATG
CTGACTTATGAAATCTT / Celera SNP ID: hCV16161169 / Public SNP ID: rs2205847 /
SNP Chromosome Position: 169676223 / SNP in Genomic Sequence: SEQ ID NO: 358 /
SNP Position Genomic: 26417 / Related Interrogated SNP: hCV11975250 / SNP
Source: dbSNP; Celera; HapMap; ABI_Val; HGBASE / Population(Allele,Count):
Caucasian (G,176|T,50) / SNP Type: INTRON /
Context (SEQ ID NO: 1070): /
TTGGGGCCAGCCTTCCTTTCCAACTTCAACTCCACAACTCCTCAATAAGCCATGGGCTCAAGAAAGTTCTGCTCAGTGGCCCC
TGAAAAATGCTTTCATA
K
TCTCACTACCATACCACTGCTTACACAATTTCCTTCCTACAGACTGCCTTCCTTTCCTGCTTTTCTCCATATACCTAAATCCT
ATCTATTCTTCATAAGC / Celera SNP ID: hCV16191220 / Public SNP ID: rs2298902 /
SNP Chromosome Position: 169663079 / SNP in Genomic Sequence: SEQ ID NO: 358 /
SNP Position Genomic: 13273 / SNP Source: dbSNP; HapMap; HGBASE /
Population(Allele,Count): Caucasian (G,206|T,20) / SNP Type: INTRON /
Context (SEQ ID NO: 1071): /

ACAATGTGAAAACTGAGGTGATGTGAGCCTAAGTTTCCAACTGGCCCCAGCTGTCAGCTTCTCCTCCCCTGCCTTATTATCAA
AGGCACTGATTGTCTAG
Y

TCTTCCTCTGTACTTCCTACGTAGATCTATCATTTTGATGTAACTTGATTTAGGGGTATAGCTTTTGTGCACAGGGACAAATC
TTACACACCAAAAATTC / Celera SNP ID: hCV27480806 / Public SNP ID: rs3766129 /
SNP Chromosome Position: 169677293 / SNP in Genomic Sequence: SEQ ID NO: 358 /
SNP Position Genomic: 27487 / Related Interrogated SNP: hCV11975250 / SNP
Source: dbSNP; HapMap; HGBASE / Population(Allele,Count): Caucasian
(C,95|T,23) / SNP Type: INTRON /
Context (SEQ ID NO: 1072): /
AGGAAATGAAAAACCAAGTTCAAAGCTATTGCTGGAGAGTCTTCAAGAATCAGATATAAAATTTGTCACAACAATGGGAGAAG
GACCAAAAAATGATAAA
S

CCCCGTCCCTTAATAAGCTCGTATTGTAATTGTAGAAATGACATTAATGTACACTGAACTATGAATAAAAAATAGAAAATGAG
GTGCTAAATATTTGGTA / Celera SNP ID: hCV27936996 / Public SNP ID: rs4656697 /
SNP Chromosome Position: 169679179 / SNP in Genomic Sequence: SEQ ID NO: 358 /
SNP Position Genomic: 29373 / Related Interrogated SNP: hCV11975250 / SNP
Source: dbSNP; HapMap; HGBASE / Population(Allele,Count): Caucasian
(C,96|G,24) / SNP Type: INTRON /
Context (SEQ ID NO: 1073): /
CACAACCAATACCACAGCCCAGATCCTCAGCTCTCCAATGACATTTTCCTCCACTAGACTTGAGCTACCTCCTTCCCTAGGCA
CAGCCTCAACCTCGACA
R

CACCTAAGACTGTACCGTCTCTAAAGTCACATGTTCAAACACTTCACTCTTTAACCACTGTCTCCTATTCTTGCAAGTGTATT
GCTCAAGTATCTCATTG / Celera SNP ID: hCV28023624 / Public SNP ID: rs4656704 /
SNP Chromosome Position: 169688228 / SNP in Genomic Sequence: SEQ ID NO: 358 /
SNP Position Genomic: 38422 / Related Interrogated SNP: hCV11975250 / SNP
Source: dbSNP; HapMap; HGBASE / Population(Allele,Count): Caucasian
(A,173|G,53) / SNP Type: INTRON /
Context (SEQ ID NO: 1074): /
TTACCCATTGTCCACTTTTCCCAAACTACATAGTGTTATATGGTATATGACCCAATCAACGGTGGCAAAGCTCCAGAAATACC
ACATAGACATCAGGGAC
R

CTTTAAACTAATCAGCCTATAGTCCTTTTTCAGTAATTTCCAAACCTGGTTGTGCATCCAAATCACTTGGTAACATTAAAAAA
ACAAAAAAATATACACG / Celera SNP ID: hCV32141621 / Public SNP ID: rs12133642 /
SNP Chromosome Position: 169689800 / SNP in Genomic Sequence: SEQ ID NO: 358 /
SNP Position Genomic: 39994 / Related Interrogated SNP: hCV11975250 / SNP
Source: dbSNP; HapMap / Population(Allele,Count): Caucasian (A,199|G,27) /
SNP Type: INTRON /
Context (SEQ ID NO: 1075): /
GTGTTATATGGTATATGACCCAATCAACGGTGGCAAAGCTCCAGAAATACCACATAGACATCAGGGACACTTTAAACTAATCA
GCCTATAGTCCTTTTTC
W

GTAATTTCCAAACCTGGTTGTGCATCCAAATCACTTGGTAACATTAAAAAAAACAAAAAAATATACACGCAACATTCGCTCCCA
ATCCTACTGAATCAGAA / Celera SNP ID: hCV32141622 / Public SNP ID: rs12133666 /
SNP Chromosome Position: 169689832 / SNP in Genomic Sequence: SEQ ID NO: 358 /
SNP Position Genomic: 40026 / Related Interrogated SNP: hCV11975250 / SNP
Source: dbSNP; HapMap / Population(Allele,Count): Caucasian (A,87|T,9) / SNP
Type: INTRON /
Context (SEQ ID NO: 1076): /
GCTGTGGGGAGAGGAGTAGCTACTCCAGGCTGCTGCCCTAGCTAAGGTGACCCTCCCCTTCTGCTGGAAGTACCATGCCATAT
GGCCTCTGCATCAAGGG
Y

TCTTATGGGATATTCTCAGAGAATCTCTGCCGTTTCATCTGTTCTGATATCTACCCAAGCATTTTGAAAAACATCCCAATTCA
CTGAAGCAAGTCCAACT / Celera SNP ID: hCV32141586 / Public SNP ID: rs12137905 /
SNP Chromosome Position: 169672867 / SNP in Genomic Sequence: SEQ ID NO: 358 /
SNP Position Genomic: 23061 / Related Interrogated SNP: hCV11975250 / SNP
Source: dbSNP; HapMap / Population(Allele,Count): Caucasian (C,109|T,11) /
SNP Type: INTRON /
Context (SEQ ID NO: 1077): /
TCTCTCATATCACTCTTTTCTCATTCTTTCTACCTGTGATCATTTCTCCTGAGATCCTTCATGAGCACTTATCCAGGACCATA
AACTACTATAATTTCTC
R

GCTTTTGTCTTTTGTATATTGCTCTTCTGACTCTAATCTACATGTATCCTTTGGTCAAACTTACTGACTTTTGCGGACTCACA
AATCTATGTGATCTTTG / Celera SNP ID: hDV71028805 / Public SNP ID: rs4987299 /
SNP Chromosome Position: 169675349 / SNP in Genomic Sequence: SEQ ID NO: 358 /
SNP Position Genomic: 25543 / Related Interrogated SNP: hCV11975250 / SNP
Source: dbSNP; HapMap / Population(Allele,Count): Caucasian (A,199|G,27) /
SNP Type: INTRON /
Context (SEQ ID NO: 1078): /
CTTAACTCCCCCAACATTTATGCAGGGTAACATTCTACCAATTTTTACAACTTAGTGTTTTTCTAATTTGTCTTCCACTTGTC
AACTCTACTACCATTGC
Y
GTCATTATCTTTCTTCTAGATTCTTGCAATAGTTTATTAGATGAGTTCCACAATTTTAAATTAGCTCCCCTCAAATACATTAT
TCATACTACAGGGAGAC / Celera SNP ID: hDV71028807 / Public SNP ID: rs4987302 /
SNP Chromosome Position: 169674983 / SNP in Genomic Sequence: SEQ ID NO: 358 /
SNP Position Genomic: 25177 / Related Interrogated SNP: hCV11975250 / SNP
Source: dbSNP; HapMap / Population(Allele,Count): Caucasian (C,109|T,11) /
SNP Type: INTRON /
Context (SEQ ID NO: 1079): /
AAATTCTTCTGATTCCTTGAGTCCATTCTTCCTTGAAGTCTCTAGAGCAAGGTAGCAATAATGGCTTAAATTTGACAGATGAG
GAATAAGACATGAAAGT
K
GTACTGTTTACTAAATGGCATGGCAGGGTCTAAAATGTGAAAACTTTGATTTCCAAGTCTTAAATTTACCTATTGTACCACCA
ATCAGAACAGGGAAATA / Celera SNP ID: hDV71028808 / Public SNP ID: rs4987304 /
SNP Chromosome Position: 169674561 / SNP in Genomic Sequence: SEQ ID NO: 358 /
SNP Position Genomic: 24755 / Related Interrogated SNP: hCV11975250 / SNP
Source: dbSNP; HapMap / Population(Allele,Count): Caucasian (T,109|G,11) /
SNP Type: INTRON /
Context (SEQ ID NO: 1080): /
TCTTAAATTTACCTATTGTACCACCAATCAGAACAGGGAAATAGATACAGTTTCTGGAGAGGGTTGTCAAAATCAAAAGAGTT
AAAATTCTGGAACTGAG
M
TAGAATAGAAGACAGAATTGAGGTCAAAATGCTTCCTAGACTTTTATATTCTCTCCTTGCAAAGAAGGCTTATTATGGAGACC
CACTCTTTCTCTGAACA / Celera SNP ID: hDV71028809 / Public SNP ID: rs4987307 /
SNP Chromosome Position: 169674403 / SNP in Genomic Sequence: SEQ ID NO: 358 /
SNP Position Genomic: 24597 / Related Interrogated SNP: hCV11975250 / SNP
Source: dbSNP; HapMap / Population(Allele,Count): Caucasian (C,109|A,11) /
SNP Type: INTRON /
Context (SEQ ID NO: 1081): /
ATCAAATCACCTTGAAAAGGTTCATCAAAGTAATGGGAAGAACTAAGGATAGAATCAGACCCTCTGCCAATATGTAAAAAAGT
AGATTCCTTCAAATGTT
M
GTATCAGGAGACAGATTTTTTTTTTATACCAGTATTTTCAAATAGAGGCTCCCAAATGAACTTAGTTTACACTTACAAGAGTAT
CTCTAAACATGCCCTTT / Celera SNP ID: hDV71028811 / Public SNP ID: rs4987318 /
SNP Chromosome Position: 169671478 / SNP in Genomic Sequence: SEQ ID NO: 358 /
SNP Position Genomic: 21672 / Related Interrogated SNP: hCV11975250 / SNP
Source: dbSNP; HapMap / Population(Allele,Count): Caucasian (A,96|C,24) / SNP
Type: INTRON /
Context (SEQ ID NO: 1082): /
TACTTATTCGTGAAGGAAGTAGAGGGAAGTGGTAACTACTGCATTGGAAAGAAATTAGTGCAGGTCCTTGGCATGTAAAAATC
AAGTGCTTATTTTTGCA
S
AACTGCTTTAAAGTAAAAGCATTAATGATTTCCTTTGACTGGTGAATTATCCAGTTCAGATCATTGGCTTTTATTTTTTCCTGA
AGTTGTATTAGGCTTTT / Celera SNP ID: hDV71028814 / Public SNP ID: rs4987323 /
SNP Chromosome Position: 169671277 / SNP in Genomic Sequence: SEQ ID NO: 358 /
SNP Position Genomic: 21471 / Related Interrogated SNP: hCV11975250 / SNP
Source: dbSNP; HapMap / Population(Allele,Count): Caucasian (G,199|C,27) /
SNP Type: TFBS SYNONYMOUS;INTRON /
Context (SEQ ID NO: 1083): /
GCATTGGAAAGAAATTAGTGCAGGTCCTTGGCATGTAAAAATCAAGTGCTTATTTTTGCACAACTGCTTTAAAGTAAAAGCAT
TAATGATTTCCTTTGAC
K
GGTGAATTATCCAGTTCAGATCATTGGCTTTTATTTTTCCTGAAGTTGTATTAGGCTTTTAGCTCTTCTCTGTGTTTCAGAGT
TCCAGTCATTGAACAAG / Celera SNP ID: hDV71028815 / Public SNP ID: rs4987324 /
SNP Chromosome Position: 169671237 / SNP in Genomic Sequence: SEQ ID NO: 358 /

SNP Position Genomic: 21431 / Related Interrogated SNP: hCV11975250 / SNP Source: dbSNP; HapMap / Population(Allele,Count): Caucasian (T,199|G,27) / SNP Type: INTRON /
Context (SEQ ID NO: 1084): /
CAGGTCCTTGGCATGTAAAAATCAAGTGCTTATTTTTGCACAACTGCTTTAAAGTAAAAGCATTAATGATTTCCTTTGACTGG
TGAATTATCCAGTTCAG
M
TCATTGGCTTTTATTTTTCCTGAAGTTGTATTAGGCTTTTAGCTCTTCTCTGTGTTTCAGAGTTCCAGTCATTGAACAAGTCC
ATGGAGCTAAATACAAC / Celera SNP ID: hDV71028816 / Public SNP ID: rs4987325 /
SNP Chromosome Position: 169671217 / SNP in Genomic Sequence: SEQ ID NO: 358 /
SNP Position Genomic: 21411 / Related Interrogated SNP: hCV11975250 / SNP Source: dbSNP; HapMap / Population(Allele,Count): Caucasian (A,109|C,11) /
SNP Type: TRANSCRIPTION FACTOR BINDING SITE;INTRON /
Context (SEQ ID NO: 1085): /
AATTGCTAACCAACTGCTCTGTGTTGCCTTTCTGTGGGCTCAGTTGATTGTCATTTTAATTTTCCTTCCATCAAATTAGTTAG
TACGTATTCTGTTCACT
R
AATATGGTATGTAAAGGTTGCCCCCTGTCCATTTACACTGGCAATTACAAAAATATTATTTATCAGTCCCTAATTGCGTGCCA
CTCTGATCTTATCTTTC / Celera SNP ID: hDV71028819 / Public SNP ID: rs4987340 /
SNP Chromosome Position: 169669143 / SNP in Genomic Sequence: SEQ ID NO: 358 /
SNP Position Genomic: 19337 / Related Interrogated SNP: hCV11975250 / SNP Source: dbSNP; HapMap / Population(Allele,Count): Caucasian (G,109|A,11) /
SNP Type: INTRON /
Context (SEQ ID NO: 1086): /
TATGCAAGAACTTCATTAGCTATGTATAACCTGAGAGCACTGAGCAGTTAAAATGTCTCTGCCCTTGCTATTAGAAATTCTGT
GCTGATTGAATCTCACT
R
TCTTATAGTATTATACAGTACTAAGTACTATACAGTACTAAGTATAAGTATCAAGTACTGCATTAAGGCAAAAGAAGAATGAC
AGTTTTCTCCAACCACT / Celera SNP ID: hDV71028821 / Public SNP ID: rs4987343 /
SNP Chromosome Position: 169668856 / SNP in Genomic Sequence: SEQ ID NO: 358 /
SNP Position Genomic: 19050 / Related Interrogated SNP: hCV11975250 / SNP Source: dbSNP; HapMap / Population(Allele,Count): Caucasian (A,109|G,11) /
SNP Type: INTRON /
Context (SEQ ID NO: 1087): /
TCTTTCTTCCTACCTTGGGCGATGATGGCAGAACTGACGGATAGAGAACAGGGTGGGTTATTACACACTGCCCCCTGGGATAA
GGTGGCTATTGTCTAAA
Y
AATTTTAAGGACTTTGGGGCTGTAAGTCACCTTAATAAGGAAGCTATTTATTTCCGAATTCTAAAATCTTTTAATAAGTGTTT
GTAGAATCAAAAGAATA / Celera SNP ID: hDV71028822 / Public SNP ID: rs4987345 /
SNP Chromosome Position: 169668608 / SNP in Genomic Sequence: SEQ ID NO: 358 /
SNP Position Genomic: 18802 / Related Interrogated SNP: hCV11975250 / SNP Source: dbSNP; HapMap / Population(Allele,Count): Caucasian (T,107|C,11) /
SNP Type: INTRON /
Context (SEQ ID NO: 1088): /
CAAATGTTAGTATCAGGAGACAGATTTTTTTTTTATACCAGTATTTTCAAATAGAGGCTCCCAAATGAACTTAGTTTACACTTA
CAAGAGTATCTCTAAAC
W
TGCCCTTTTACTTATTCGTGAAGGAAGTAGAGGGAAGTGGTAACTACTGCATTGGAAAGAAATTAGTGCAGGTCCTTGGCATG
TAAAAATCAAGTGCTTA / Celera SNP ID: hDV71028828 / Public SNP ID: rs4987395 /
SNP Chromosome Position: 169671386 / SNP in Genomic Sequence: SEQ ID NO: 358 /
SNP Position Genomic: 21580 / Related Interrogated SNP: hCV11975250 / SNP Source: dbSNP; HapMap / Population(Allele,Count): Caucasian (T,109|A,11) /
SNP Type: INTRON /
Context (SEQ ID NO: 1089): /
TTTCAAACATTGCCAAGAGAACCAGTAAGTATCCCCTGGATGCAACAAGGGTGTCAGTGGGGAGACTATTTGCAGTGCAGACA
TCAAGACAGAGGTCAGA
Y
GGCCAAGCATTGAGCATTACTGAGGATGAGAAAGTAGAGACAGAACAAGCAGTCATCTCTTTTAAAATGTTTGGCTGTGAAGG
AGAAGTGGGGCAGGGGT / Celera SNP ID: hDV71070471 / Public SNP ID: rs4987363 /
SNP Chromosome Position: 169663938 / SNP in Genomic Sequence: SEQ ID NO: 358 /
SNP Position Genomic: 14132 / Related Interrogated SNP: hCV11975250 / SNP Source: CDX; dbSNP; HapMap / Population(Allele,Count): Caucasian (C,199|T,27) / SNP Type: INTRON /

Context (SEQ ID NO: 1090): /
GATCTCTCAAGATGACAATATTTATTCTCCACACAGCACATACTTCAGGGTTGGAAGGCAGGGGCAATCTTCTCCTTTATAAT
GAGTGCCTCTTATATAT
S
TTTATTCATCTGCCCTCTTGTAAAACACACACACACACACACACAAAGAAGAAATAAAATAACTCTGCTTCTTTGAAGCTTGT
GACACTGAGATAAACCA / Celera SNP ID: hDV77030725 / Public SNP ID: rs4656701 /
SNP Chromosome Position: 169687903 / SNP in Genomic Sequence: SEQ ID NO: 358 /
SNP Position Genomic: 38097 / Related Interrogated SNP: hCV11975250 / SNP
Source: CDX; dbSNP / Population(Allele,Count): Caucasian (G,173|C,53) / SNP
Type: INTRON /
Context (SEQ ID NO: 1091): /
CTCCTCATGCAAGATTGGCTTTGGCACCTAGTTCCTGATCTTCCTTTCCCTGTAAGCACTTCTCATAGTCTTACGGGACTTCA
CCATCCATGGCACAACC
M
ATACCACAGCCCAGATCCTCAGCTCTCCAATGACATTTTCCTCCACTAGACTTGAGCTACCTCCTTCCCTAGGCACAGCCTCA
ACCTCGACAACACCTAA / Celera SNP ID: hDV77030727 / Public SNP ID: rs4656703 /
SNP Chromosome Position: 169688135 / SNP in Genomic Sequence: SEQ ID NO: 358 /
SNP Position Genomic: 38329 / Related Interrogated SNP: hCV11975250 / SNP
Source: CDX; dbSNP / Population(Allele,Count): Caucasian (A,173|C,53) / SNP
Type: INTRON //

Gene Number: 22 / Gene Symbol: SURF6 - 6838 / Gene Name: surfeit 6 /
Chromosome: 9 / OMIM NUMBER: 185642 / OMIM Information: // Genomic Sequence
(SEQ ID NO: 359): // / SNP Information /
Context (SEQ ID NO: 1092): /
GGCGGCTCCCGCGGCTCCGTGCAGGCCCCCTCTGGGGTTGCCTCCACCACCTCCTGGGCCTCCGTGGCCTCCTCAGCCTTCCT
GGCCTTCTCTTTCGCCC
R
CAGCTCCTTTCGCTTCCTCTTCTTCCGGTCCCGTTCCTGCTTTCTCCGCCGCCTTTTCTCCAAGGCGGCAGGGGACAGCTCCT
TGGCACTGCCCTGGGGG / Celera SNP ID: hCV8784837 / Public SNP ID: rs886090 /
SNP Chromosome Position: 136199503 / SNP in Genomic Sequence: SEQ ID NO: 359 /
SNP Position Genomic: 42340 / Related Interrogated SNP: hCV25610857 / SNP
Source: Applera / Population(Allele,Count): Caucasian (A,16|G,24) African
American (A,3|G,35) total (A,19|G,59) / SNP Type: MISSENSE MUTATION;ESE / SNP
Source: dbSNP; Celera; HapMap; ABI_Val; HGBASE / Population(Allele,Count):
Caucasian (G,150|A,76) / SNP Type: MISSENSE MUTATION;ESE /
Context (SEQ ID NO: 1093): /
GCACTCCAGCCTGGGCAACGAGAGCGAAACTCTGTCTTAAAAAAAAAAAAAGAGAGAGAGAGAATTGCTGAACGCCCACTAAGT
GCCAGGCACCATTCTGG
K
TGCGGCCTCATTGCTGAATAATGCGGGCAGAAACCCCAACTCCGTGGAGTGCCTGCTTGGTGAGAAGACTCCCTTGTAGAACT
CTGTGAGGGTCAGAGAC / Celera SNP ID: hCV998010 / Public SNP ID: rs493014 / SNP
Chromosome Position: 136187163 / SNP in Genomic Sequence: SEQ ID NO: 359 /
SNP Position Genomic: 30000 / Related Interrogated SNP: hCV25610857 / SNP
Source: dbSNP; HapMap; ABI_Val; HGBASE / Population(Allele,Count): Caucasian
(G,84|T,142) / SNP Type: INTERGENIC;UNKNOWN /
Context (SEQ ID NO: 1094): /
ATGTAGTAAATGTGAAATAAAACAATTTCAAAAAGAACAAAGTTGGAAGACTCATGCTACCCGATTTCAACACTTACTATAGA
GCTGCAGTGATTAAGAC
R
GTGTGGCGCTGGCGAAAGGTTCAGGGGAACAGAACACACAGTTTAGAAATAGACCTACACAGATTTATGGCCAATTGATTTCA
GATGAAGGTACAAGGGC / Celera SNP ID: hCV3183246 / Public SNP ID: rs10901263 /
SNP Chromosome Position: 136192872 / SNP in Genomic Sequence: SEQ ID NO: 359 /
SNP Position Genomic: 35709 / Related Interrogated SNP: hCV25610857 /
Related Interrogated SNP: hCV27859399 / SNP Source: dbSNP; Celera; HapMap /
Population(Allele,Count): Caucasian (G,170|A,56) / SNP Type:
INTERGENIC;UNKNOWN /
Context (SEQ ID NO: 1095): /
GATCACAGATAGTAAGAGGAACAGGGAGTGAGCACTCACATATTCAACTAGAGGACAGGGACCGAGATAAAAACAGCAGCAAG
TTGTGATGGGTACGTAC
R
GTGTGTTCCCTGCCAAATCCACACACTGATGTCTTAACCCCAAGCACCACAGAACGTGACCTTGTTTGGAAATGGTGTCACTG
CAAATGTTATTAGTTAA / Celera SNP ID: hCV3183251 / Public SNP ID: rs529309 /

217

SNP Chromosome Position: 136191641 / SNP in Genomic Sequence: SEQ ID NO: 359 / SNP Position Genomic: 34478 / Related Interrogated SNP: hCV25610857 / SNP Source: dbSNP; Celera; HapMap; ABI_Val; HGBASE / Population(Allele,Count): Caucasian (A,45|G,75) / SNP Type: INTERGENIC;UNKNOWN /
Context (SEQ ID NO: 1096): /
GTATCACAAAAGCAGACTGAACTTTTTAGACCTTACAAATGCATGACTGTCCCCCTTGCCTCTGATCTTCTGCCTGGGGTCTG
CCTTTCCCCAATCTTTG
Y

TCACTATACTGAATCCTACATGACACAGTCAACCTATGAAGAAATCCAGAGATAGACTCTCTAAAATAAATGGATTTGGGAAT
AGCAGCTTATCTGGAAT / Celera SNP ID: hCV9327931 / Public SNP ID: rs17150482 /
SNP Chromosome Position: 136194595 / SNP in Genomic Sequence: SEQ ID NO: 359 / SNP Position Genomic: 37432 / Related Interrogated SNP: hCV25610857 /
Related Interrogated SNP: hCV27859399 / SNP Source: dbSNP; Celera; HapMap /
Population(Allele,Count): Caucasian (T,168|C,58) / SNP Type: UTR5 /
Context (SEQ ID NO: 1097): /
AGCCTCCAGCAACAATGGCACGTTACCTGCTCCATTGCAGAACTGGCCGTGGTTTACATCCAGGAAGGGATTGGTCAGTCACC
TGTGCCAGGTCCCCGGG
R

GATCCTGAGCCCAGCGGTATAAAGGGTGGCTGTGGGAGTGGCACACCCTCTCCCAGCCCCAGCAAGCAAACCGTCAGGCGGCC
GTGGACTCAGATCCCGG / Celera SNP ID: hCV26744892 / Public SNP ID: rs11244079 /
SNP Chromosome Position: 136184526 / SNP in Genomic Sequence: SEQ ID NO: 359 / SNP Position Genomic: 27363 / Related Interrogated SNP: hCV25610857 /
Related Interrogated SNP: hCV27859399 / SNP Source: dbSNP; Celera; HapMap /
Population(Allele,Count): Caucasian (G,207|A,17) / SNP Type:
INTERGENIC;UNKNOWN /
Context (SEQ ID NO: 1098): /
CCTCACTGGCCCCTTAGTTCACTATGATGACTTCACCTCTTGGGAACCCATGGGGATGTCTTTCCCTCCAATACATCTACATT
TGCCTGACCCAGAATGT
R

GTTTCAAGATGGGTCCATGCCTGGCTCCAAATCCCCTTATCAGAGTAAGGAAATACCTTAATTCCTAGTTTGCTAAAAACTCG
TTATAAATGGCTGTCGC / Celera SNP ID: hCV26744899 / Public SNP ID: rs10751505 /
SNP Chromosome Position: 136195374 / SNP in Genomic Sequence: SEQ ID NO: 359 / SNP Position Genomic: 38211 / Related Interrogated SNP: hCV25610857 / SNP Source: dbSNP; Celera; HapMap / Population(Allele,Count): Caucasian
(A,149|G,77) / SNP Type: INTERGENIC;UNKNOWN //

Gene Number: 23 / Gene Symbol: TSPAN8 - 7103 / Gene Name: tetraspanin 8 / Chromosome: 12 / OMIM NUMBER: / OMIM Information: // Genomic Sequence (SEQ ID NO: 360): // / SNP Information /
Context (SEQ ID NO: 1099): /
GGAGAGAATCCTCTGGAGTCTGAAGGTTGGACACTGGGATTTGTTTGGAGAACTCACCAACAGAAGCATGCAGCGACTTTCTT
TTATAGCACCGCAGCAT
S
CCAGGAAGCCCAGAATCATGATGATGGCACCTACAGCAATCAATATGTCCACAGCAACGTAGGAGCTAGAGCCTACATCTTCA
GAACCAAAAATCTGAAG / Celera SNP ID: hCV11686277 / Public SNP ID: rs3763978 /
SNP Chromosome Position: 71533534 / SNP in Genomic Sequence: SEQ ID NO: 360 / SNP Position Genomic: 24657 / SNP Source: Applera / Population(Allele,Count): Caucasian (C,20|G,16) African American (C,28|G,4) total (C,48|G,20) / SNP Type: MISSENSE MUTATION;ESS / SNP Source: dbSNP; HapMap; ABI_Val; HGBASE / Population(Allele,Count): Caucasian (C,71|G,49) / SNP Type: MISSENSE MUTATION;ESS //

Gene Number: 24 / Gene Symbol: BLZF1 - 8548 / Gene Name: basic leucine zipper nuclear factor 1 / Chromosome: 1 / OMIM NUMBER: / OMIM Information: // Genomic Sequence (SEQ ID NO: 361): // / SNP Information /
Context (SEQ ID NO: 1100): /
TTTTATTTTAAAGTAGAAATCTGTCCGCTTTTCACTTACGGAGGCCTTACAGTGTGTGTTCTGTGTTACTTGAGTTTATACGC
ACTGCAGAATTTGTTTA
Y
CCTGCTCTTTTGGAAACGTATTCCGGAAGCGAAACCCTGAGTAATCGGAAGTGGTTAGGAGTGAGAGAGCTGCTGGATATGCG
GAGGGACTGGGCGGGTC / Celera SNP ID: hCV15878582 / Public SNP ID: rs2275299 /
SNP Chromosome Position: 169337376 / SNP in Genomic Sequence: SEQ ID NO: 361 / SNP Position Genomic: 10182 / Related Interrogated SNP: hCV11975250 / SNP

218

Source: Applera / Population(Allele,Count): Caucasian (C,11|T,29) African American (C,22|T,14) total (C,33|T,43) // / SNP Type: UTR5;TFBS SYNONYMOUS / SNP Source: dbSNP; Celera; HapMap; HGBASE / Population(Allele,Count): Caucasian (C,94|T,132) / SNP Type: UTR5;TFBS SYNONYMOUS / Context (SEQ ID NO: 1101): / GCTTGTGACTTTTCCATCTCAGACTTTTTTCTGAGTCTGAGAGATTTATATCCAGCCAGGTATTTCCATCTACTTGTTCCACA GATGGCTAAAAATATAA Y

CTCTTTTCTCCTTGCTTTTGCATTTGTACAACCCCCCACCCCTAAAAATCTCCTCCTTTTCCTGTATTCCCTATCTTGATGAA TTATACCTACTCTATAG / Celera SNP ID: hCV221700 / Public SNP ID: rs6677410 / SNP Chromosome Position: 169353295 / SNP in Genomic Sequence: SEQ ID NO: 361 / SNP Position Genomic: 26101 / Related Interrogated SNP: hCV11975250 / SNP Source: dbSNP; Celera; HapMap / Population(Allele,Count): Caucasian (T,94|C,132) / SNP Type: INTRON / Context (SEQ ID NO: 1102): / ATAGTCCTATAGTCTAGCAATATAACTTTAAGATTTACATAACAGAATGAAAGTCAGAATTGTATTTTATGGCATTAAATAAA TTAACTTTATTTCTGTC Y

ATATCAACATCCAAAGTTTATTAGGAATTTATTTAAAGAATAATAAAAGCAATAGGACCTAACATTTAATGAGTGCTTACTGT GTCAAATACCATTCTAA / Celera SNP ID: hCV279320 / Public SNP ID: rs10800441 / SNP Chromosome Position: 169344023 / SNP in Genomic Sequence: SEQ ID NO: 361 / SNP Position Genomic: 16829 / Related Interrogated SNP: hCV11975250 / SNP Source: dbSNP; Celera / Population(Allele,Count): Caucasian (T,94|C,132) / SNP Type: INTRON / Context (SEQ ID NO: 1103): / TCTGCCACTCAGAAAAGCAAAAAGACTCCAAGATCTAGGGAGATTCAGATCAGTGTTGAGACAGTCACATACACACCACCCCA TACTCTTAATAAAGCGC R

TTAATGTGAATCTGTGAACATGAAAAATGCCATGATGAACAATGCAAACACATAATGTTTGTTCAAAACAGGTTATTAGACTC GATGCCTTACCCTAAAT / Celera SNP ID: hCV288901 / Public SNP ID: rs4656671 / SNP Chromosome Position: 169336617 / SNP in Genomic Sequence: SEQ ID NO: 361 / SNP Position Genomic: 9423 / Related Interrogated SNP: hCV8919444 / SNP Source: dbSNP; Celera; HapMap; HGBASE / Population(Allele,Count): Caucasian (G,146|A,80) / SNP Type: INTRON / Context (SEQ ID NO: 1104): / CTACCTCCCTAACCACATTTCATGGTATTTTGCACCATATTTTCCCTACTGCAGCCACCCCTGCACCATCCCACCACCCAGTT TCTATTATTTTATTACA Y

TGAACCATAAGTTCCTAAAATATACCAATCTCTTTTCTGCCTCAGGGTATTTGATCTTGTTGTTAGCGTGGCCTGGACTCCTC CAGATCACTGCACTTTG / Celera SNP ID: hCV475606 / Public SNP ID: rs17349579 / SNP Chromosome Position: 169332898 / SNP in Genomic Sequence: SEQ ID NO: 361 / SNP Position Genomic: 5704 / Related Interrogated SNP: hCV8919444 / SNP Source: dbSNP; Celera; HapMap / Population(Allele,Count): Caucasian (T,78|C,26) / SNP Type: INTRON / Context (SEQ ID NO: 1105): / GGCATTTAGATACAAACAGGTCTAATATTTAAGAAATGAAAAGATGAATATAAAAAAAGGTCTAATATGTAAGAAATGAACAGG TGAATATAATGAATAAA M

AGAGAATCTCAGTAAAGAAATGGAAATTTAAAAAAAATAAACTGATAGGAGGATCCAAGATGGCCAAATAGGAACAGCTCCAGT CTATAGCTCCCAGCGTG / Celera SNP ID: hCV2456767 / Public SNP ID: rs2014061 / SNP Chromosome Position: 169369883 / SNP in Genomic Sequence: SEQ ID NO: 361 / SNP Position Genomic: 42689 / Related Interrogated SNP: hCV8919444 / SNP Source: dbSNP; HapMap; HGBASE / Population(Allele,Count): Caucasian (A,53|C,65) / SNP Type: TRANSCRIPTION FACTOR BINDING SITE;INTRON / Context (SEQ ID NO: 1106): / GGCAAGGTAGAAGGGGGCTGGGTTATTAGGCTTTTGTAACAGCCCAGCCAACAGAAGAAAATAGTGGTTTAGATTAGGGTAGT GGGACTCTGTATATGCA S

TGAAGATAGAACCAACATAATTTGCTCAAAAATTGAATGGATAGTTTTATGTCATGCTTTAAATGATTATTTTTTAAATTATT CCAGGTTTATTAGTATA / Celera SNP ID: hCV2456768 / Public SNP ID: rs6427186 / SNP Chromosome Position: 169368562 / SNP in Genomic Sequence: SEQ ID NO: 361 / SNP Position Genomic: 41368 / Related Interrogated SNP: hCV11975250 / SNP Source: dbSNP; Celera / Population(Allele,Count): Caucasian (C,94|G,132) /

SNP Type: INTRON /
Context (SEQ ID NO: 1107): /
CAGGTTTGCCTTAAATTACCAAAATATGTACCCAAATCTCCTTTGGTCTATACAGATTTTCCTCCTTAGTCCACCTGCCTTTC
TTTTACTGGTAAATGCA
R
CTTTTATTGGTTGCATTTATTTGTAAATTCAGTTTTTCTGAGGGCTATGTAGGAGAAGCTGTAACCAATCTTCAACCCATTTC
TCACAGAAATAAGTAAA / Celera SNP ID: hCV2456774 / Public SNP ID: rs1014965 /
SNP Chromosome Position: 169366076 / SNP in Genomic Sequence: SEQ ID NO: 361 /
SNP Position Genomic: 38882 / Related Interrogated SNP: hCV8919444 / SNP
Source: dbSNP; HapMap / Population(Allele,Count): Caucasian (A,80|G,34) / SNP
Type: UTR3;INTRON /
Context (SEQ ID NO: 1108): /
AGAAGAAGGAAGGAAATTAAGATTAGAGTGCAGACAAATAGAGAATAGAAAAACAAGAGAGATCAAAGAAGTCAACAGTTAGT
TCTTTAAAAAGAAAGAC
Y
AAATTGACAAATCTGTAGCCATACTGACTAAGAAAAATAAAACAGGTAAAACTAAAATCAGAAATGAAAGTGGGGACATTATT
AGTCACCTTACATAAAG / Celera SNP ID: hCV2456776 / Public SNP ID: rs6669741 /
SNP Chromosome Position: 169359654 / SNP in Genomic Sequence: SEQ ID NO: 361 /
SNP Position Genomic: 32460 / Related Interrogated SNP: hCV8919444 / SNP
Source: dbSNP; Celera / Population(Allele,Count): Caucasian (C,54|T,66) / SNP
Type: INTRON /
Context (SEQ ID NO: 1109): /
TCTCCTTTTCTATTCTCTTTGTTGGGAAATTGTGTCACTGCCTACTTAGTTGCTGCCAGTAGCATTTGGTATAGTTGACCACT
CCCTCTTTCTCGAACTA
Y
TCTTTCCGAGGTTTATATAACTTTATAGCTTACAGGCTTTTCTCTTTTATCCAACCATTTTATTTCACTATACAATGTTAGGT
ATCCTCAGGGCTTGATT / Celera SNP ID: hCV2456780 / Public SNP ID: rs7534737 /
SNP Chromosome Position: 169354336 / SNP in Genomic Sequence: SEQ ID NO: 361 /
SNP Position Genomic: 27142 / Related Interrogated SNP: hCV8919444 / SNP
Source: dbSNP; Celera / Population(Allele,Count): Caucasian (T,54|C,66) / SNP
Type: INTRON /
Context (SEQ ID NO: 1110): /
CCAGCTGAGAAAATGGCTGAAACGGTAAAATATTTTCTTTTGTGATCTATGGAACTTCTAACACTGTCCTTTTACTGAAAGGT
GACATATCATGGAAAGC
R
TTTGGCATTAGGAATTAGGAAATGTGGGATCTAAACCACTCATGCTATTGAGTGTGTTATCTTGGAGAAAATTATTTCCTCTT
CTGCGCTTTAATATTTT / Celera SNP ID: hCV8688930 / Public SNP ID: rs3905328 /
SNP Chromosome Position: 169351596 / SNP in Genomic Sequence: SEQ ID NO: 361 /
SNP Position Genomic: 24402 / Related Interrogated SNP: hCV11975250 / SNP
Source: dbSNP; HGBASE / Population(Allele,Count): Caucasian (A,80|G,144) /
SNP Type: INTRON;PSEUDOGENE /
Context (SEQ ID NO: 1111): /
AGTTTTGGAGACATTGGAGTTCCAATCCAACTATAGTGGGAATACTTCATTGCTTACCTTGGTCAACCACTTACAACCCTAAA
AAGGCCATGCTTTGGAA
R
TAAGGGTCATGTCCTAGAGTAAGGACCATGCTCTAGGACTAAGGACAAAAGCAAAATAAACCCATCCTAACAAAGCATAAAAC
AAAGTCTGACAGGATCA / Celera SNP ID: hCV8690976 / Public SNP ID: rs1124843 /
SNP Chromosome Position: 169369458 / SNP in Genomic Sequence: SEQ ID NO: 361 /
SNP Position Genomic: 42264 / Related Interrogated SNP: hCV11975250 / SNP
Source: dbSNP; Celera; ABI_Val; HGBASE / Population(Allele,Count):
Caucasian (A,81|G,145) / SNP Type: INTRON /
Context (SEQ ID NO: 1112): /
ATATTGAAAGCCTAAAAATGCACATGACCTCTCGCACCTAGTTATTTCCTTTTTAATTTATTGTAAGGAAATAATTATGGATG
TCTATAAAAATGTGTTC
R
CAAAAATATGTTAGTATTGTGTTAACAGAACAAATTAATAAATGGATTGGTTAAATCAGAGTAAATGAAAGAATAAAACGTAG
AGCTGTCCCTCAGTATT / Celera SNP ID: hCV11341861 / Public SNP ID: rs10800436 /
SNP Chromosome Position: 169328198 / SNP in Genomic Sequence: SEQ ID NO: 361 /
SNP Position Genomic: 1004 / Related Interrogated SNP: hCV11975250 / SNP
Source: dbSNP; Celera / Population(Allele,Count): Caucasian (G,107|A,5) / SNP
Type: INTRON /
Context (SEQ ID NO: 1113): /
GCTTCATCTTAAATGAGGTGTATGAGAAACATGCTAAACTATGGTTCCCTATCTCTGCTTCACAATATAAAGGTGATTCCAGA

ATTTGGAAGCCTCAACT
R
TAACAAACTAGATGAAGACAGAAGCTGATTAGATAAACTGCAGTGGTCAGGAAGCCTCACCAACAGAGCAAACAACCTCCATC
TTCAGCTGCTTATTGAG / Celera SNP ID: hCV11341869 / Public SNP ID: rs2176473 /
SNP Chromosome Position: 169331802 / SNP in Genomic Sequence: SEQ ID NO: 361 /
SNP Position Genomic: 4608 / Related Interrogated SNP: hCV11975250 / SNP
Source: dbSNP; Celera; HapMap; ABI_Val; HGBASE / Population(Allele,Count):
Caucasian (A,88|G,138) / SNP Type: INTRON /
Context (SEQ ID NO: 1114): /
TCTTTCTATCCTCCAGTCCGTTATCCATAAGTGCTTAAAAGATATGGTAGTTAAGTGAATTAACTAGAACCCTCTCAAACATA
GGGGTGAATTAAAATAA
Y
CTATAGGCTTATTTAAAATGCAAATACCGAGCTCCATTCCTTTCATTCAGAATTTCCAGGGGTGAAGCCCAAACAGTGAATTC
CAGAAGCTCTCCAAGCA / Celera SNP ID: hCV11341876 / Public SNP ID: rs1980198 /
SNP Chromosome Position: 169336259 / SNP in Genomic Sequence: SEQ ID NO: 361 /
SNP Position Genomic: 9065 / Related Interrogated SNP: hCV11975250 / SNP
Source: dbSNP; Celera; HapMap; HGBASE / Population(Allele,Count): Caucasian
(C,94|T,132) / SNP Type: INTRON /
Context (SEQ ID NO: 1115): /
TAGGGGTGAATTAAAATAACCTATAGGCTTATTTAAAATGCAAATACCGAGCTCCATTCCTTTCATTCAGAATTTCCAGGGGT
GAAGCCCAAACAGTGAA
K
TCCAGAAGCTCTCCAAGCAATCCCAGGAGAAATTTCTGTTTAAAAAAAAAAAGAAGAAGAAAGAAAAGAAAAGAGAAAAGAAAA
GCAAGCACAGACCTAGA / Celera SNP ID: hCV11341878 / Public SNP ID: rs4656670 /
SNP Chromosome Position: 169336340 / SNP in Genomic Sequence: SEQ ID NO: 361 /
SNP Position Genomic: 9146 / Related Interrogated SNP: hCV11975250 / SNP
Source: dbSNP; Celera; HapMap; HGBASE / Population(Allele,Count): Caucasian
(T,94|G,132) / SNP Type: INTRON /
Context (SEQ ID NO: 1116): /
AGTGTAAGTGTAGAAAAGTTTGTGACTGTTAAGAAAGTTAGTCGTCGGGTTCACTAATTGAACTGAGTTGTATCTAGTACTGA
GAACGATCTTTTTTTGT
R
GTGAAACCAGTTGTCATCTAGTGTACAGTTTGTCATATTTATATCATTGCAGTAGCATCACCTTCTTATATGTTCTCTGGTAA
ATCTGAGTCTGTACTGT / Celera SNP ID: hCV11341879 / Public SNP ID: rs7527703 /
SNP Chromosome Position: 169338046 / SNP in Genomic Sequence: SEQ ID NO: 361 /
SNP Position Genomic: 10852 / Related Interrogated SNP: hCV8919444 / SNP
Source: dbSNP; Celera; HapMap / Population(Allele,Count): Caucasian
(A,146|G,80) / SNP Type: INTRON /
Context (SEQ ID NO: 1117): /
TTGCTGTAACAAAGTACCACAAACTGCATGGCTTGAACAACAAAATGTATTCTCTCAGTTCTGGAGGCTAGAAGTCCAAGATG
AAAATGTCAACAAGGCT
R
TGCTCCTTCGGAAGGCATTAGAGACACTCTCCTAGCTTCCAGTGGCTTGCTGGCAGTCTTTGGTGCTCCTTGTCTTGTAGACG
TACCACCCTTATCTCCG / Celera SNP ID: hCV11341882 / Public SNP ID: rs12024897 /
SNP Chromosome Position: 169339268 / SNP in Genomic Sequence: SEQ ID NO: 361 /
SNP Position Genomic: 12074 / Related Interrogated SNP: hCV11975250 / SNP
Source: dbSNP; Celera; HapMap / Population(Allele,Count): Caucasian
(G,94|A,132) / SNP Type: INTRON /
Context (SEQ ID NO: 1118): /
TCCTGTTTCCAAATGAAGTCTCATTCTGAGGTAGTGGGGGGTTAGTACTTCAACAAATAAAGATTTGGGGGACACAATTCAAC
CCCTAAATTGCTAAATA
Y
AGTAGTCACTTTTTAGTCCTAGCTGATTTGTCCTCTCTAATATTTAATACTGTTGACCATTCCCTGCCTTTTGAGATACGTTC
TTTTGATGACTTCCATG / Celera SNP ID: hCV11341886 / Public SNP ID: rs7539415 /
SNP Chromosome Position: 169339612 / SNP in Genomic Sequence: SEQ ID NO: 361 /
SNP Position Genomic: 12418 / Related Interrogated SNP: hCV8919444 / SNP
Source: dbSNP; Celera; HapMap / Population(Allele,Count): Caucasian
(C,54|T,66) / SNP Type: INTRON /
Context (SEQ ID NO: 1119): /
TTAAACATGTATCTTTTGTTGACACATGATTGTTTTTGAAAATTACTTTTTCTCTGATTTTTTTTTTTGCATTCTTTTTAGTGCC
AGTAATTTTGAATATAG
R
GTATAGCATGTATTAGCATTTTCTTGGCATTTCTTCTTGGTAAGTAGTTTCTGTTTTGTTTCCTTTTTTGTTAGTCACCGTTAC

TTCATCCCCAATCCGAG / Celera SNP ID: hCV11341898 / Public SNP ID: rs12563090 / SNP Chromosome Position: 169345704 / SNP in Genomic Sequence: SEQ ID NO: 361 / SNP Position Genomic: 18510 / Related Interrogated SNP: hCV11975250 / SNP Source: dbSNP; Celera / Population(Allele,Count): Caucasian (G,93|A,131) / SNP Type: INTRON /
Context (SEQ ID NO: 1120): /
TCAAGTGAAGCCCCCTGAAATACGGAGAATAAGAATCTTAGAGGTAAATCCGAGGACAAGCAGGTATAACTAAGGATGTTGGA
AAGGGACGGAGGTCTGG
Y

TGGAGCTCGGTGGCCAGTAGCTTTGGATTTAGGGAAAGATTGTTCCGGGAAGACTTTTTCAGACAGGGATGAAACTTAGGTTT
CGACTTCCCCGGACAGC / Celera SNP ID: hCV27242515 / Public SNP ID: rs3818844 / SNP Chromosome Position: 169337638 / SNP in Genomic Sequence: SEQ ID NO: 361 / SNP Position Genomic: 10444 / Related Interrogated SNP: hCV8919444 / SNP Source: dbSNP; Celera; HGBASE / Population(Allele,Count): Caucasian (C,51|T,65) / SNP Type: INTRON;PSEUDOGENE /
Context (SEQ ID NO: 1121): /
TAAATGTCAAAGATGTGGAGAAATTGGAACCCCTATGCATTGTTTGTGGGAATGTAAAATGTTGAAAACAGTGTGGCAGATCC
TCAAAATATTAAACATA
S

AGTTACCATATGGCCTAGCAATTCTGCTTCTAGGTAGATTCCCAAAAGAAAGCAGAAACTAGAATAGGTATTTGTACAACAGT
GTTCACAGCTGCATTAT / Celera SNP ID: hCV27242533 / Public SNP ID: rs2138898 / SNP Chromosome Position: 169358811 / SNP in Genomic Sequence: SEQ ID NO: 361 / SNP Position Genomic: 31617 / Related Interrogated SNP: hCV8919444 / SNP Source: dbSNP; Celera; HapMap; ABI_Val; HGBASE / Population(Allele,Count): Caucasian (G,53|C,67) / SNP Type: MISSENSE MUTATION;INTRON /
Context (SEQ ID NO: 1122): /
TATATCCCGCACCTGGCTCAGAGGGTCCCACTCCCACAGAGCCTCACTCACTGCTAACACAGCAGTCTGAGATCAAACTGCAA
TGCCACAGCAAGGCTAG
R

GGAGAGATGTCTGCCATTGCTGAGGCTTAAGTAGGTAAACAAAGCAGCTGGGAAGCTCCAACTGGGTGGAGCTGACCACAGCT
CAAGGAGGCCTGCCTGC / Celera SNP ID: hCV27928247 / Public SNP ID: rs4656182 / SNP Chromosome Position: 169370336 / SNP in Genomic Sequence: SEQ ID NO: 361 / SNP Position Genomic: 43142 / Related Interrogated SNP: hCV8919444 / SNP Source: dbSNP; HapMap / Population(Allele,Count): Caucasian (G,83|A,37) / SNP Type: INTRON /
Context (SEQ ID NO: 1123): /
ATCTTGCTGGATATTTTATATGAACAGTGTTAATTTAGATGCACTAAAGCAAAGGTAGGCAAACTACAACCATGAGTCAAACA
TGGCCACACCCATTCAT
K

TGCTATTGTCTAAGCTGGTTTTGCACTACAACTGCAGAGTTGAATAGATGCAGCAGATCCTTTACAGAAAAAGTTTTCTGACC
TCAATTCTAAAGTAATT / Celera SNP ID: hCV29397237 / Public SNP ID: rs6427185 / SNP Chromosome Position: 169356833 / SNP in Genomic Sequence: SEQ ID NO: 361 / SNP Position Genomic: 29639 / Related Interrogated SNP: hCV11975250 / SNP Source: dbSNP; HapMap / Population(Allele,Count): Caucasian (G,81|T,145) / SNP Type: TRANSCRIPTION FACTOR BINDING SITE;UTR3;INTRON //

Gene Number: 25 / Gene Symbol: NR1I2 - 8856 / Gene Name: nuclear receptor subfamily 1, group I, member 2 / Chromosome: 3 / OMIM NUMBER: 603065 / OMIM Information: // Genomic Sequence (SEQ ID NO: 362): // / SNP Information /
Context (SEQ ID NO: 1124): /
AAGCACTGCCTTTACTTCAGTGGGAATCTCGGCCTCAGCCTGCAAGCCAAGTGTTCACAGTGAGAAAAGCAAGAGAATAAGCT
AATACTCCTGTCCTGAA
M

AAGGCAGCGGCTCCTTGGTAAAGCTACTCCTTGATCGATCCTTTGCACCGGATTGTTCAAAGTGGACCCCAGGGGAGAAGTCG
GAGCAAAGAACTTACCA / Celera SNP ID: hCV263841 / Public SNP ID: rs1523127 / SNP Chromosome Position: 119501039 / SNP in Genomic Sequence: SEQ ID NO: 362 / SNP Position Genomic: 11708 / SNP Source: dbSNP; Celera; HapMap; HGBASE / Population(Allele,Count): Caucasian (C,76|A,150) / SNP Type: UTR5 /
Context (SEQ ID NO: 1125): /
TCTTTACAAACACTTAGATGTCCTAGGAATTCTTGTCATGTTCCTGATTTCCATTTTTATTTTGTACTATGAACTCATTTGGA
AACTTCTCTTTGGGACT
Y

GTTGAGATTGAAATTGTTTTCCTCTAGAGGATTTATTTTTGCTTTTGCCAGGAACCTGAGGACAGAACTAACTGGGACCACCA

TAAGCTTGGGGTCTACT / Celera SNP ID: hCV30747430 / Public SNP ID: rs11712211 / SNP Chromosome Position: 119504140 / SNP in Genomic Sequence: SEQ ID NO: 362 / SNP Position Genomic: 14809 / SNP Source: dbSNP; HapMap / Population(Allele,Count): Caucasian (T,22|C,204) / SNP Type: TFBS SYNONYMOUS;INTRON /
Context (SEQ ID NO: 1126): /
ATATCTACCCCCACCACAATGCTATTTAATACTGTATTAATCGTTCCAACTAATGCAATAAGGAAAGAAAAAAAGCATAAAGA
TCAGAAAAGAAGAAAAC
W
GTCTTTCTTTGCAGAAAACATAATTATTTACATTGAACATCCTCAGTAATACTAAGGAATAACTACTAGAACTATTTAATAAA
GTCACAGTTATATCTCA / Celera SNP ID: hCV192027 / Public SNP ID: rs9821892 / SNP Chromosome Position: 119491949 / SNP in Genomic Sequence: SEQ ID NO: 362 / SNP Position Genomic: 2618 / Related Interrogated SNP: hCV263841 / SNP Source: dbSNP; Celera; HapMap; ABI_Val / Population(Allele,Count): Caucasian (A,84|T,36) / SNP Type: INTERGENIC;UNKNOWN /
Context (SEQ ID NO: 1127): /
GATACAGTATAGGCAGATTGTCAGAAGACAAAGAGAATCTTGAGAACAGTGAGAGAGAGGCAACTCATCCTGTACAAGGGAGC
CTTAATAAGATTAACAG
Y
TGATTTCGCATCAGAAACCATGGAGGCCTGAAAACAGGAGGCCTGATGCCACTTCACACCTACTAGGATGGATATAATTTTTTT
TTAATGGAAAATAACAA / Celera SNP ID: hCV1833991 / Public SNP ID: rs11926554 / SNP Chromosome Position: 119495556 / SNP in Genomic Sequence: SEQ ID NO: 362 / SNP Position Genomic: 6225 / Related Interrogated SNP: hCV263841 / SNP Source: dbSNP; Celera; HapMap / Population(Allele,Count): Caucasian (T,73|C,27) / SNP Type: TRANSCRIPTION FACTOR BINDING SITE;INTERGENIC;UNKNOWN /
Context (SEQ ID NO: 1128): /
ACATATCCAGGAAGCTCAACAAAGCCCAAGCAAAATAAACACGAAGAGATACACCAAGATACAGTATAGGCAGATTGTCAGAA
GACAAAGAGAATCTTGA
R
AACAGTGAGAGAGAGGCAACTCATCCTGTACAAGGGAGCCTTAATAAGATTAACAGTTGATTTCGCATCAGAAACCATGGAGG
CCTGAAAACAGGAGGCC / Celera SNP ID: hCV1834240 / Public SNP ID: rs1581451 / SNP Chromosome Position: 119495499 / SNP in Genomic Sequence: SEQ ID NO: 362 / SNP Position Genomic: 6168 / Related Interrogated SNP: hCV263841 / SNP Source: dbSNP; Celera; HapMap; HGBASE / Population(Allele,Count): Caucasian (G,76|A,146) / SNP Type: INTERGENIC;UNKNOWN /
Context (SEQ ID NO: 1129): /
ATTGCAGGCCTGTTGTTAAGAATTTCAAAGGAGACTTTTCTTTTTGCTCTACCCAGAGCCAAGGCTGAGAAGGCCTGGGATAG
ACCTGTCTCCCTCCATG
R
TTTCTACTGAGGATGTCACCCTTAGGGGATCTTGAGTTTATAGTCTATGATCTGGTTAGGCTCCAGGCTTTGTCTCCTATTCT
ACCATTAAAATCCAGGC / Celera SNP ID: hCV1834252 / Public SNP ID: rs10934498 / SNP Chromosome Position: 119504381 / SNP in Genomic Sequence: SEQ ID NO: 362 / SNP Position Genomic: 15050 / Related Interrogated SNP: hCV263841 / SNP Source: dbSNP; Celera; HapMap / Population(Allele,Count): Caucasian (G,80|A,146) / SNP Type: INTRON /
Context (SEQ ID NO: 1130): /
AGACTCTGTTGCTTCAAATTCAAGTTGCCCACTCTTCTCTTCCCATAATCTCAGTGACCTGGTTCCAAAGCTTGTGCTTTGAC
TCATTTGAAGACTAAAT
K
TATCTGTATGTCCTCCCCACATCCTTTTCCTGGGAAGGAACTATCTCACCCTCAAAGTACATTGACTTTTTAAAAAAATTATTA
TTATTATTACTTTAAGA / Celera SNP ID: hCV8760915 / Public SNP ID: rs1403527 / SNP Chromosome Position: 119506880 / SNP in Genomic Sequence: SEQ ID NO: 362 / SNP Position Genomic: 17549 / Related Interrogated SNP: hCV30747430 / SNP Source: dbSNP; HapMap; HGBASE / Population(Allele,Count): Caucasian (T,22|G,204) / SNP Type: INTRON /
Context (SEQ ID NO: 1131): /
GCAGGCCCCAGATATAGCCCCATGCTGTCCTCCTACCCCAGAGCACACTGTTCAGGCTACTTCCACTGGTACTGAAATCCAGT
ATTTCACTTACTCTTTT
Y
CTTTCCAATATCCTCATGACATTCAATATTTCACTTACTCTAGGTCCTCCCTGCCTAAGGCCCAAGTCAACTTTCTGTCCAGT
GGGATTTGTAATCCAAT / Celera SNP ID: hCV9152783 / Public SNP ID: rs1523130 / SNP Chromosome Position: 119499507 / SNP in Genomic Sequence: SEQ ID NO: 362 / SNP Position Genomic: 10176 / Related Interrogated SNP: hCV263841 / SNP

Source: dbSNP; Celera; HapMap; HGBASE / Population(Allele,Count): Caucasian (T,79|C,147) / SNP Type: UTR5 /
Context (SEQ ID NO: 1132): /
CTTATAAAAATAAAATAAATACATAAAATGTGACTAGCAATTTTAAGACACTTAAATATTTGAATTAATTTATTGAATACCTG
CTCTGGGAATTAGTGTT
R

TGTTTCCATTTACAGAGACAAAACAGGTCTAATTGATTGACTAAACAATAGATGTAAATGTGGTCAGTGTTTGGATGTCTATT
CATAGAATAGGAACAAT / Celera SNP ID: hCV11230788 / Public SNP ID: rs7643038 /
SNP Chromosome Position: 119497045 / SNP in Genomic Sequence: SEQ ID NO: 362 /
SNP Position Genomic: 7714 / Related Interrogated SNP: hCV263841 / SNP
Source: dbSNP; Celera; HapMap; ABI_Val / Population(Allele,Count): Caucasian
(G,76|A,150) / SNP Type: INTERGENIC;UNKNOWN /
Context (SEQ ID NO: 1133): /
GTATTGCACTAGAGGAACTACTAAGACGTTGAATTGCCACATCACAGGATATATGTGGTTCAATTTTTTGGCTGCCAAACAGCC
CTCCACAGTACCCGAAC
Y

GACTGTACTCCCACCAGCAGAGAAGGATAGTTACTGTTCCTCCACACTCCAAACAATGCTTGCTGTTTATTCAGTATTTTATT
ATTTTTTCCATTGAATT / Celera SNP ID: hCV11906384 / Public SNP ID: rs2472670 /
SNP Chromosome Position: 119503005 / SNP in Genomic Sequence: SEQ ID NO: 362 /
SNP Position Genomic: 13674 / Related Interrogated SNP: hCV30747430 / SNP
Source: dbSNP; HapMap; HGBASE / Population(Allele,Count): Caucasian
(C,22|T,204) / SNP Type: INTRON /
Context (SEQ ID NO: 1134): /
ATCACAGGATATATGTGGTTCAATTTTTTGGCTGCCAAACAGCCCTCCACAGTACCCGAACCGACTGTACTCCCACCAGCAGAG
AAGGATAGTTACTGTTC
Y

TCCACACTCCAAACAATGCTTGCTGTTTATTCAGTATTTTATTATTTTTTTCCATTGAATTAGTGCAAATGACAACCCATTATT
TCAATATTTTAATTTCC / Celera SNP ID: hCV11906385 / Public SNP ID: rs2472671 /
SNP Chromosome Position: 119503045 / SNP in Genomic Sequence: SEQ ID NO: 362 /
SNP Position Genomic: 13714 / Related Interrogated SNP: hCV30747430 / SNP
Source: dbSNP; HapMap; ABI_Val; HGBASE / Population(Allele,Count): Caucasian
(C,22|T,204) / SNP Type: INTRON /
Context (SEQ ID NO: 1135): /
TCACCAAACGTCTCCCTTCACTGAACAACGTCAATGGTTTTATAGGCATCACTTGCCTGCATCTTAGAAATTCAGTATTCTTA
TATTACATTCCCCTTAC
Y

TGCTTTTTTCTCTTTATCCTCTGGCCAGTTCTGCTATTAATATGTGTGTGTGTGTGTGTGTGTGTGTGTGTGTGTGTGTGTGT
GTGTGTGTGGTGTGTAT / Celera SNP ID: hCV11906386 / Public SNP ID: rs2056530 /
SNP Chromosome Position: 119506462 / SNP in Genomic Sequence: SEQ ID NO: 362 /
SNP Position Genomic: 17131 / Related Interrogated SNP: hCV30747430 / SNP
Source: dbSNP; HapMap; HGBASE / Population(Allele,Count): Caucasian
(T,22|C,204) / SNP Type: INTRON /
Context (SEQ ID NO: 1136): /
AAGATGTGTGTGAACACAAATATACCTTCTGTTTGAGGTCAGCATCATAGTGGGTCGTGAATCATGTTGGCCTTGCTGCTGTC
TCCTCATTTCTAGGGTG
R

AAAAAAAAAAAGCATGAAAACAATCACTTAATGTTGAGCCCCATTACTGATGCTCTCTGGTCCTGCACTAGCCTCCTAGAAAAA
TCACCACAGGAGAAGCC / Celera SNP ID: hCV15882316 / Public SNP ID: rs2276706 /
SNP Chromosome Position: 119501307 / SNP in Genomic Sequence: SEQ ID NO: 362 /
SNP Position Genomic: 11976 / Related Interrogated SNP: hCV263841 / SNP
Source: dbSNP; HapMap; HGBASE / Population(Allele,Count): Caucasian
(G,72|A,48) / SNP Type: INTRON /
Context (SEQ ID NO: 1137): /
TTAAATATATGTAATCGTGTGTATGTATATGTATGTATACACACACACATAATTAAATATAATCCTGCTATCTCCAAGAGTTT
CTATCCCAAAGATTATG
R

TTTTTGTTTTTTTATCCATTTACCCCCTTTTACTTAGACTCTGTTGCTTCAAATTCAAGTTGCCCACTCTTCTCTTCCCATAA
TCTCAGTGACCTGGTTC / Celera SNP ID: hCV16090105 / Public SNP ID: rs2873951 /
SNP Chromosome Position: 119506744 / SNP in Genomic Sequence: SEQ ID NO: 362 /
SNP Position Genomic: 17413 / Related Interrogated SNP: hCV30747430 / SNP
Source: dbSNP; HapMap / Population(Allele,Count): Caucasian (A,22|G,204) /
SNP Type: INTRON /
Context (SEQ ID NO: 1138): /

GGAGGATTTTCTGTAACCAACTTCTCCATCATAGTGCCAGCAATAAAATCTTTATTTTTTATTTTAATGAGATTGATTCTAAT
GCTTCTCCAATAAGATA
S
CTAGTAAAAGTGTTTAGTAACTATTCCATATCAAGTTAGTGAAGCTCTCTTCTATTCCTCTTGGTTAACCAAGAATTTTAAAT
CATGAATGAATATTGAG / Celera SNP ID: hCV26079834 / Public SNP ID: rs2472672 /
SNP Chromosome Position: 119504763 / SNP in Genomic Sequence: SEQ ID NO: 362 /
 SNP Position Genomic: 15432 / Related Interrogated SNP: hCV30747430 / SNP
Source: dbSNP; HapMap; HGBASE / Population(Allele,Count): Caucasian
(G,13|C,107) / SNP Type: INTRON /
Context (SEQ ID NO: 1139): /
GTACTTCAAAATAATAACAACTTAAGTCAATAAATAAATGTAAGGAAGTCCAAATGTTCACCTGAAGACAACTGTGGTCATTT
TTTGGCAATCCCAGGTT
Y
TCTTTTCTACCTGTTTGCTCAATCGTGGTCTCCCTCTCCCTCTCTTGTTGGGGCCCATGCCCCTGCTTTACTGTTGCCAGAGG
CTTGTACTTGTTTGCCT / Celera SNP ID: hCV27504984 / Public SNP ID: rs3814055 /
SNP Chromosome Position: 119500035 / SNP in Genomic Sequence: SEQ ID NO: 362 /
 SNP Position Genomic: 10704 / Related Interrogated SNP: hCV263841 / SNP
Source: dbSNP; HapMap; ABI_Val; HGBASE / Population(Allele,Count): Caucasian
(C,150|T,76) / SNP Type: UTR5 /
Context (SEQ ID NO: 1140): /
GGAAAAGTTCCCTGCAACACCTCCTGAACCTCTTTGAAACATCGATATAACAAACTGAATATCCAACTGGAAATTGACATAAA
GAGACATATCCAAATCC
S
TCATGTAATGTTTGAAATAATTTTCCTGGATCTGTGTAAGTTCCAAAGAGTCAAACTATAAAACCACAGGCAACACTGGCACT
ACACATGCATTGATAAG / Celera SNP ID: hCV28031759 / Public SNP ID: rs4234666 /
SNP Chromosome Position: 119505317 / SNP in Genomic Sequence: SEQ ID NO: 362 /
 SNP Position Genomic: 15986 / Related Interrogated SNP: hCV30747430 / SNP
Source: dbSNP; HapMap; ABI_Val; HGBASE / Population(Allele,Count): Caucasian
(C,9|G,101) / SNP Type: INTRON /
Context (SEQ ID NO: 1141): /
AAAGGATGCTAAGGGAGGCCTCAGAGGCTTATTTAATCAGTTGGTTAATGGGAAAAAGTATTTTGTGGTACACAAATACGCTT
TAAAAATATTTTAGTTA
Y
GTCATAAAAGTCTAAACCTGGGGTTGACAAACTATGGCTCACAGGCTAACTCTGGCCCACCAGCTGTTTTTATAAATGAAGCT
TTATTGGAACACAGCCA / Celera SNP ID: hCV30747432 / Public SNP ID: rs12488820 /
SNP Chromosome Position: 119502069 / SNP in Genomic Sequence: SEQ ID NO: 362 /
 SNP Position Genomic: 12738 / Related Interrogated SNP: hCV263841 / SNP
Source: dbSNP; HapMap / Population(Allele,Count): Caucasian (T,80|C,146) /
SNP Type: INTRON /
Context (SEQ ID NO: 1142): /
AAGAGTTTTGCTCCTTCCTTTCCAATCTTTACAAACCTTAATTTTTTGTGTCTTATTGGTTTTTCTTTGCCATTCAGCATAAT
GCTGAGTAGTAGCCATG
R
AAGTAGATAGTCTCAGAAGTGATGCATACAGTTGTGTGGGTTCTATACTGCACAATTCTAGGAGATACTCTTTACAAACACTT
AGATGTCCTAGGAATTC / Celera SNP ID: hCV30747431 / Public SNP ID: rs13071341 /
SNP Chromosome Position: 119503971 / SNP in Genomic Sequence: SEQ ID NO: 362 /
 SNP Position Genomic: 14640 / Related Interrogated SNP: hCV30747430 / SNP
Source: dbSNP; HapMap / Population(Allele,Count): Caucasian (A,22|G,204) /
SNP Type: INTRON //

Gene Number: 26 / Gene Symbol: LRAT - 9227 / Gene Name: lecithin retinol
acyltransferase (phosphatidylcholine--retinol O-acylt / ransferase) //
Chromosome: 4 / OMIM NUMBER: 604863 / OMIM Information: Retinal dystrophy,
early-onset severe (3) // Genomic Sequence (SEQ ID NO: 363): // / SNP
Information /
Context (SEQ ID NO: 1143): /
CCTTAACAAAATTATACACTTTTCAAAGGAGTTTCTTAGTGTCTAGTAGTCACAGTGTACATAGCACTCATAAATTATAAAGC
GTGAATAGAATTTCATG
K
TCTCCCAGAGTTATTTTATTTTTTATAAAGTTCTACAGACAGGCAATCAAGGTAAGGAAGATCTTTCATTTCTAGCTGCTCAG
GAGATTTACGTTCAAAC / Celera SNP ID: hCV2407252 / Public SNP ID: rs149225 /
SNP Chromosome Position: 155683216 / SNP in Genomic Sequence: SEQ ID NO: 363 /
 SNP Position Genomic: 62806 / Related Interrogated SNP: hCV11503414 /

Related Interrogated SNP: hCV11503469 / SNP Source: dbSNP; HapMap; ABI_Val; HGBASE / Population(Allele,Count): Caucasian (T,64|G,52) / SNP Type: INTERGENIC;UNKNOWN /
Context (SEQ ID NO: 1144): /
TGGAGGAGAAGCAGGTATGATTGGAGCAATGAGGATTGTAGGAGCTGATGGAAAGAAAGTTGAATAGGTGGAGTAGCTCCAGG
TAATACTAAGGGGAAGG
K
GTGAGTTGCTGATATCTGTGTGGAGATGAAGGTCAGTGATGTTGAAGGTAAGGCACTGGGTATTGGAAAGGTCATCCACACGG
ACACTGAAGACACCAGG / Celera SNP ID: hCV2407354 / Public SNP ID: rs276166 /
SNP Chromosome Position: 155618542 / SNP in Genomic Sequence: SEQ ID NO: 363 /
SNP Position Genomic: 1868 / Related Interrogated SNP: hCV11503414 / Related
Interrogated SNP: hCV15860433 / SNP Source: dbSNP; HapMap; HGBASE /
Population(Allele,Count): Caucasian (G,91|T,11) / SNP Type: INTRON /
Context (SEQ ID NO: 1145): /
ATCAGAGGCAAAAATATCCTTCAAGAAACTATGGACACTCCGCGCCCTATTCATTTCCATGGCAGCAGAGTATCTGCATCTTG
AGCCACCTATACAGATT
S
ATGCCTCGTATCGCTCTCACCTCCTTTCTTTTTGAAGTAAAGCCCTTTCCCAAGAAGGCGGCCAGAAAGTGGACCCCACCGGG
GGAAAAAGAAAAATGAA / Celera SNP ID: hCV2891552 / Public SNP ID: rs1876031 /
SNP Chromosome Position: 155662736 / SNP in Genomic Sequence: SEQ ID NO: 363 /
SNP Position Genomic: 42326 / Related Interrogated SNP: hCV11503469 / SNP
Source: dbSNP; Celera; HapMap; ABI_Val; HGBASE / Population(Allele,Count):
Caucasian (C,201|G,23) / SNP Type: UTR3;INTRON /
Context (SEQ ID NO: 1146): /
GTGTGCCTCCAGGGAGCCATTAAAAACTGACCTCTCAACCACAGAAATAGATGAGATTTTGAGAACATTGAGAAGCTGCCTTT
TGCAAAGTAAATTTGCA
R
TGGTCCTTGACGAAGGGGGGTCGGGGGCGGGGAGAAGTCCAGCCGAGAGAGGAGCTCATTCCACGCTATATTTTTGCAGTTGA
AAAGCTGCCTAATCATC / Celera SNP ID: hCV2891554 / Public SNP ID: rs12501328 /
SNP Chromosome Position: 155662331 / SNP in Genomic Sequence: SEQ ID NO: 363 /
SNP Position Genomic: 41921 / Related Interrogated SNP: hCV11503469 / SNP
Source: dbSNP; Celera; HapMap; ABI_Val / Population(Allele,Count): Caucasian
(A,195|G,23) / SNP Type: UTR3;INTRON /
Context (SEQ ID NO: 1147): /
CCCCCTAATGAATCACGGTTACCATGAGAAGGATGACCTCATGTTATTGAAGAAAAGTCCTGGTCTGAGATTCAGGAAACAAG
ATTCAGATGGCTTGTGT
Y
CAGTGTTTAGTTCTGGACCCATTTCTGGGGCTAAGTGGCTATGTGATTTTGGTTGTTCAATTTATTTAACTTCTCAGAACCTT
GGTTTTCTCATTTCAGG / Celera SNP ID: hCV2892850 / Public SNP ID: rs10050268 /
SNP Chromosome Position: 155549354 / SNP in Genomic Sequence: SEQ ID NO: 363 /
SNP Position Genomic: 71056 / Related Interrogated SNP: hCV11503469 /
Related Interrogated SNP: hCV11503414 / SNP Source: dbSNP; Celera; HapMap;
ABI_Val / Population(Allele,Count): Caucasian (C,190|T,36) / SNP Type:
INTRON /
Context (SEQ ID NO: 1148): /
TGACTCTAGTGATATCTTCTTCTCGAGTCTGGATTCTGGTGCTGCTATTTGCTCCCATTTAGCTTATGATGGCTACTCGCGTG
GCGGCCCACCTACTGCC
R
CTAACTCACACTGCACCATTGTTTCTGCTGCCTTGTGGTTTTCATTACACTTCTACCCTGTTACTTTTAGCTTCTGCTCCACT
GTACCTGGTCGCCGTCT / Celera SNP ID: hCV8938834 / Public SNP ID: rs1500372 /
SNP Chromosome Position: 155676300 / SNP in Genomic Sequence: SEQ ID NO: 363 /
SNP Position Genomic: 55890 / Related Interrogated SNP: hCV11503414 / SNP
Source: dbSNP; HapMap; HGBASE / Population(Allele,Count): Caucasian
(G,112|A,8) / SNP Type: INTERGENIC;UNKNOWN /
Context (SEQ ID NO: 1149): /
GGCTTTAATTGACCCTCCCACCTAAGCCTCCAAAAGCACTAGGATTATGGGTGTGAGCCACCAGGCCCAGCGACAACATGGGT
TTTAACTGGGTGGGTCC
R
TTTACGTGCAAAATTTTTCAACCAAATGTGGACAGAAAACCAAGTATTCCTAGGATCTGAAACCTGCTTATATAGGGAGGGCC
AACTTCTGTATATGTGG / Celera SNP ID: hCV8938838 / Public SNP ID: rs1392546 /
SNP Chromosome Position: 155674933 / SNP in Genomic Sequence: SEQ ID NO: 363 /
SNP Position Genomic: 54523 / Related Interrogated SNP: hCV11503414 / SNP
Source: dbSNP; Celera; HapMap; HGBASE / Population(Allele,Count): Caucasian

(A,112|G,8) / SNP Type: INTERGENIC;UNKNOWN /
Context (SEQ ID NO: 1150): /
TTCTATTATTTTTACTCAAGATTAGAGACACCTCTTTGCTCATCTAATTTTAAAATATGCTATTGTTATTGATAAGCAGTTTG
ATGTTTTAAACCTTTAG
Y
CCTGAATAACCATGTGGTTATACTTTCTATAGTTTTCTATAAATGCTGTATTTACATAGGCAACTGCCATTATTAGTTGCCTA
AGGGATCATTTCTGCTA / Celera SNP ID: hCV27479909 / Public SNP ID: rs3775785 /
SNP Chromosome Position: 155669854 / SNP in Genomic Sequence: SEQ ID NO: 363 /
SNP Position Genomic: 49444 / Related Interrogated SNP: hCV11503414 /
Related Interrogated SNP: hCV11503469 / SNP Source: dbSNP; HapMap; HGBASE /
Population(Allele,Count): Caucasian (T,111|C,7) / SNP Type: INTRON /
Context (SEQ ID NO: 1151): /
GTTGGGTTTAGGATTTTACTTCTTCAGAATCTACTTTACAAACTAAAGTGGGTAGGAACAAACCCTTTTTGACTTCACTTCCA
AATGTTTCTGGAGCTAC
W
CTTCTTAATCTCCTTCCTCCTTTCCTGTCTCAGAGGAACAGAAGTCCTTCCTCCTCATCAGTTCTAACTTCCATCTGCAGTTT
TGATCCCATGGCTTCCC / Celera SNP ID: hCV29636755 / Public SNP ID: rs10517602 /
SNP Chromosome Position: 155653240 / SNP in Genomic Sequence: SEQ ID NO: 363 /
SNP Position Genomic: 32830 / Related Interrogated SNP: hCV11503469 / SNP
Source: dbSNP; HapMap / Population(Allele,Count): Caucasian (A,202|T,24) /
SNP Type: INTRON /
Context (SEQ ID NO: 1152): /
TGGTAAAACAAGCATTCAAGCACTTTTACAAAATTACCAAATTCTTAAAATGAAGCCACAGCTAGACTTGCATTTCAGGTATT
AAAATTGCTTTCTTAAC
K
GTCAAGAATCACAAAATAACAAATCATATTATGAGTGAATATGGGGAGGGCGGGGCCAATCAGTCAATGATAATCTGAACAAA
TTTTAAGAGCAGATTTT / Celera SNP ID: hCV31863937 / Public SNP ID: rs12507608 /
SNP Chromosome Position: 155671626 / SNP in Genomic Sequence: SEQ ID NO: 363 /
SNP Position Genomic: 51216 / Related Interrogated SNP: hCV11503469 / SNP
Source: dbSNP; HapMap / Population(Allele,Count): Caucasian (T,202|G,24) /
SNP Type: MICRORNA;UTR3;TFBS SYNONYMOUS /
Context (SEQ ID NO: 1153): /
AAGAATTGCTGGCAACATATGCACTAAACTTCGTTTTGAAAAATCCCCTTGATGCCGATCACTGAGAAGTGATCCACAGGTGA
GCGTCTAAGCATTGAAC
R
TTTTCTATTCTGTGCTTTTTTTCTTTCGTGTGTTGTTGATGTCAGGCTGTTATCAACACATTCTATGAAACAGGACCCTAGGTA
TATGCATTATAACACGA / Celera SNP ID: hDV70817803 / Public SNP ID: rs17031951 /
SNP Chromosome Position: 155649957 / SNP in Genomic Sequence: SEQ ID NO: 363 /
SNP Position Genomic: 29547 / Related Interrogated SNP: hCV11503469 / SNP
Source: dbSNP; HapMap / Population(Allele,Count): Caucasian (A,201|G,25) /
SNP Type: INTRON /
Context (SEQ ID NO: 1154): /
TTGAGGCAAAGAAAGTTATTATCTTATTAATATATGGTCCAGAGTCAGCTAAAGTCAGATACTATGGAAGTGGGTTGACATGT
GCCTGGGTCATCCTAAG
M
AGGAAATAAAATCAGTACTTTAGCTCAGTCCATTGTATCACTTAGCCTTAACATTTGCAAATGATTTTGAAACCTTGCTATAT
TTAGCTCTTCTTAATTA / Celera SNP ID: hDV70817805 / Public SNP ID: rs17031954 /
SNP Chromosome Position: 155650410 / SNP in Genomic Sequence: SEQ ID NO: 363 /
SNP Position Genomic: 30000 / Related Interrogated SNP: hCV11503469 / SNP
Source: dbSNP; HapMap / Population(Allele,Count): Caucasian (A,201|C,25) /
SNP Type: TRANSCRIPTION FACTOR BINDING SITE;INTRON /
Context (SEQ ID NO: 1155): /
CCTGGGCAACAAAGTGAGACTCCATCTCAAAAAAAAAAAAAAAAAAAAAAAAAAAAAAAAAAAGAAGAAGAAACCCTGAGCCATTAA
TGTGTCTCGTAACTTGA
K
GAAGTTCATGAATTCTCTGGGCTTCATCCATTAAGATTCATCCTTATGCTCCTGCAAGTTTCTTATATTTATGTATTTCTATT
CTGATTTCTGAAATGAT / Celera SNP ID: hDV70817844 / Public SNP ID: rs17032000 /
SNP Chromosome Position: 155672958 / SNP in Genomic Sequence: SEQ ID NO: 363 /
SNP Position Genomic: 52548 / Related Interrogated SNP: hCV11503469 / SNP
Source: dbSNP; HapMap / Population(Allele,Count): Caucasian (G,203|T,23) /
SNP Type: MICRORNA;UTR3 //

Gene Number: 27 / Gene Symbol: XYLB - 9942 / Gene Name: xylulokinase

homolog (H. influenzae) / Chromosome: 3 / OMIM NUMBER: 604049 / OMIM Information: // Genomic Sequence (SEQ ID NO: 364): // / SNP Information / Context (SEQ ID NO: 1156): /
GCCCTTTTCAGAGGTTGTGAAGTTAGCTCCAAATCCCAGACTAGCTGCTACCCCAAGCCCGGGAGCTTCTCAGGTGAGAGACC
ATCGGAATTTGTTTGTA
R
CATTTGCATTATGAAAGCCCGCTAGGGTTTTTTCCCCCACCAAAAGGTCACCTACATTGAACGTGATGTGCTCAACTAAAGGA
GAAATTCTGCTTTATTG / Celera SNP ID: hCV15949414 / Public SNP ID: rs2234628 /
SNP Chromosome Position: 38442504 / SNP in Genomic Sequence: SEQ ID NO: 364 /
SNP Position Genomic: 64253 / SNP Source: dbSNP; HapMap; HGBASE /
Population(Allele,Count): Caucasian (G,214|A,10) / SNP Type: MISSENSE
MUTATION;INTRON /
Context (SEQ ID NO: 1157): /
GTGAGTGGTGTCCTTTGGAGGCATGGTGGATGAAAGGCGCAAAGGAAGCAAGAGGTTAAAGATAATGGCCCATTAGATACAAC
AGCAAGCCCAAGATTCC
R
AAGAAGACAGACAACTCCCCCTAGTACCACCTAGACATTACTTTTTAAAGACATTTTACATTATTGTCCAGCAGAAAAAGGCT
GTCTTGGACCATGAAAC / Celera SNP ID: hCV1845321 / Public SNP ID: rs12636358 /
SNP Chromosome Position: 38455901 / SNP in Genomic Sequence: SEQ ID NO: 364 /
SNP Position Genomic: 77650 / Related Interrogated SNP: hCV15949414 / SNP
Source: dbSNP; Celera; HapMap / Population(Allele,Count): Caucasian
(G,216|A,10) / SNP Type: MICRORNA;UTR3;INTRON //

Gene Number: 28 / Gene Symbol: AKT3 - 10000 / Gene Name: v-akt murine thymoma viral oncogene homolog 3 (protein kinase B, gamma / ) // Chromosome: 1 / OMIM NUMBER: / OMIM Information: // Genomic Sequence (SEQ ID NO: 365): // / SNP Information /
Context (SEQ ID NO: 1158): /
ACTTGAAAGGCATCAAAACAGGCCGCTGACAATCACAGTGTTCCTCCTGTCAGCTTTCCTGGTTTCCTGTATTTCAAAGATCA
CAGATGATCAGCACCCT
S
TTCTCATAAATGTCCTGTGATGACAGTCCTGTGCTGTTGGAAGCTCTTCCTGGTATGTGGCCACGTGGTCAGCCACTCAGTCA
ACATGCGCTCCCACGCA / Celera SNP ID: hCV233148 / Public SNP ID: rs1417121 /
SNP Chromosome Position: 243669350 / SNP in Genomic Sequence: SEQ ID NO: 365 /
SNP Position Genomic: 27815 / SNP Source: dbSNP; Celera; HapMap; HGBASE /
Population(Allele,Count): Caucasian (G,86|C,30) / SNP Type: INTRON;PSEUDOGENE
/
Context (SEQ ID NO: 1159): /
ATACAGTAACACTATGGACTAGCCAATCTGGTGCTTGAGCAGAATTGAAAATAGGGTGATTCAAATTGGCAGAACTCTCGGTG
CCTCTCCTAAAGAGACG
R
CTTCTCAGGCACACGGGAGGACAAAAACGCCACTGAGAGACTATCAGCCCTGCTCCCTGCTTTTCCAGGGGACAAAGTGACCG
GCCATCGCCCAGAACTG / Celera SNP ID: hCV30690777 / Public SNP ID: rs12045585 /
SNP Chromosome Position: 243673099 / SNP in Genomic Sequence: SEQ ID NO: 365 /
SNP Position Genomic: 31564 / SNP Source: dbSNP; HapMap /
Population(Allele,Count): Caucasian (G,194|A,30) / SNP Type: ESE;SILENT
MUTATION;INTRON /
Context (SEQ ID NO: 1160): /
AAAAATTATAGCTTACACAGACTAAATAACCTCCCTACAGTGGATCCAAGAGATAGTGATAGGGCCAGAATTTAAACTCAACT
TTTAACCAGTACTGTTC
Y
GCTGATGTCTCTGATATGTCTGAGTGCCTAAATGTATCATGAGTTGTGCTAAGTGCAATAAAAAAAGATAAGGTGCCCTTAAG
GAATTCGTATATCTAAT / Celera SNP ID: hCV31523650 / Public SNP ID: rs12048930 /
SNP Chromosome Position: 243940076 / SNP in Genomic Sequence: SEQ ID NO: 365 /
SNP Position Genomic: 298541 / SNP Source: dbSNP; HapMap /
Population(Allele,Count): Caucasian (C,186|T,40) / SNP Type: TRANSCRIPTION
FACTOR BINDING SITE;INTRON /
Context (SEQ ID NO: 1161): /
TAGCATTATTACTTTGATATTCATCCATGTCACCACATGTATCAATAGTTTACTCACTTTGCTGGGTAATATTTCAGTACATA
GATGTATCACAATGGAC
M
CACTCATCTGTCAATGAACACAGGGCCTGCTCCCAGTTTGGGGCAATTACAAACAAAATTGCTATTAACTTTTATGTCCACGT
CTTTGTGTAGACATATG / Celera SNP ID: hCV30690780 / Public SNP ID: rs10737888 /

SNP Chromosome Position: 243678483 / SNP in Genomic Sequence: SEQ ID NO: 365 /
SNP Position Genomic: 36948 / SNP Source: dbSNP; HapMap /
Population(Allele,Count): Caucasian (A,180|C,46) / SNP Type: INTRON /
Context (SEQ ID NO: 1162): /
CTGTGCACGTGGTAACTAATTGACTGATATTCATATATAAAGTAGGTACTTTATATTGCAGTTTGACAAATTTAGTCTAATTC
CATCATCAGAAAGAATG
R
GGCAGTGGAACTCATTTAATGAGCTTCAATATACCAATATGTTGGCTGGGCATGGTGGCTCATGCCTGTAATCCCAGCATTTT
GGAAGGTCGAGGCAGGC / Celera SNP ID: hCV31523608 / Public SNP ID: rs12744297 /
SNP Chromosome Position: 243734296 / SNP in Genomic Sequence: SEQ ID NO: 365 /
SNP Position Genomic: 92761 / SNP Source: dbSNP; HapMap /
Population(Allele,Count): Caucasian (G,35|A,83) / SNP Type: INTRON /
Context (SEQ ID NO: 1163): /
ATCTTCACTTTCAGGGTGTTCACAATCCCCTTGTGGCCAGACCATGGTCCCAAGATTAAGACCTCTTGGCCCACATGACCCCT
TGTTTGCCTCTCGACAG
K
AGAATCTGCTGCCTGTGACCATCTCTGGCACAGCATTAGCCTCCAATCGCCCCTCTCCCGGACACTGAGTCCACATCAGCACT
ATGGATAAATCTGCCTC / Celera SNP ID: hCV97631 / Public SNP ID: rs1538773 / SNP
Chromosome Position: 243674682 / SNP in Genomic Sequence: SEQ ID NO: 365 /
SNP Position Genomic: 33147 / Related Interrogated SNP: hCV233148 / Related
Interrogated SNP: hCV30690780 / Related Interrogated SNP: hCV31523650 /
Related Interrogated SNP: hCV30690777 / Related Interrogated SNP: hCV31523608
/ SNP Source: dbSNP; Celera; HapMap; ABI_Val; HGBASE /
Population(Allele,Count): Caucasian (T,180|G,46) / SNP Type: UTR5;INTRON /
Context (SEQ ID NO: 1164): /
GAGTTGACTAATTAAAATTTTAAGTGAAAATTTCTCCCATTTCTTTAACCATTCTTAGTGATCCAGAATGTCATACTGGGGAT
GGCAATAGCCCCTATTC
Y
TTGGCATTTCTAAATAGCATCTCATGTGGATTCAGAAAAAGGTAGTCGATGATATTTGTTGGTTTGACGTCTAACTGTGGGGA
AGGGACTGGGAAGAATA / Celera SNP ID: hCV804118 / Public SNP ID: rs320342 / SNP
Chromosome Position: 243867832 / SNP in Genomic Sequence: SEQ ID NO: 365 /
SNP Position Genomic: 226297 / Related Interrogated SNP: hCV31523608 / SNP
Source: dbSNP; HGBASE / Population(Allele,Count): Caucasian (T,26|C,200) /
SNP Type: TFBS SYNONYMOUS;INTRON /
Context (SEQ ID NO: 1165): /
TACTGCACTTATTCAGAAGTATTTACATAAATGAGGCAACGATGAATTTTTTAAGTCTCTTTGGCTAGTGTCAGGTATATAAA
ACATGTATTTTATGTAA
R
GAAATGAAGAGGTTATCCAACATCTAAACACAGTATTAATTTGACTAATACTTTTAGTTCTTTTTGAATATCAGGATATCAGAG
GGGTAAACTAGTCTTCC / Celera SNP ID: hCV804120 / Public SNP ID: rs320344 / SNP
Chromosome Position: 243866036 / SNP in Genomic Sequence: SEQ ID NO: 365 /
SNP Position Genomic: 224501 / Related Interrogated SNP: hCV31523608 / SNP
Source: dbSNP; HapMap; HGBASE / Population(Allele,Count): Caucasian
(G,24|A,202) / SNP Type: INTRON /
Context (SEQ ID NO: 1166): /
TCAGAATGCTGTATTTTAAATATTTAATTTTTCATGTTTAATCACAAGCAAAGTCACAAAGTTTTTACCATGTATGATTATTTG
TCCTTATACATCTCTGA
R
GAAAAAAATGTATAGATTACATCTATACTTAATGCATGCGTTAATGAAAAAATAACCTCTTTCGCAATCTTTATTATTTACAG
AGGGAGAGAGCCATAGA / Celera SNP ID: hCV804121 / Public SNP ID: rs320345 / SNP
Chromosome Position: 243865303 / SNP in Genomic Sequence: SEQ ID NO: 365 /
SNP Position Genomic: 223768 / Related Interrogated SNP: hCV31523608 / SNP
Source: dbSNP; HapMap; HGBASE / Population(Allele,Count): Caucasian
(A,24|G,202) / SNP Type: INTRON /
Context (SEQ ID NO: 1167): /
GAGCAAATTTACAAGACTCAGGCAAGCTAAATAACTTGCCCCAAAACACACAACTGTGAGTCATTATATAAGGATTTATTCCA
AGTCTATCTCAATCCCA
R
TCCCATGCTCTTCTGAAGAGAATACACTGCTAGGAAAAATGCCTTAAAAATCAGTAATCATCTGTCAAAGCCATAGGAATGTC
TGTTCCCTCTTCTATAC / Celera SNP ID: hCV804126 / Public SNP ID: rs320320 / SNP
Chromosome Position: 243835186 / SNP in Genomic Sequence: SEQ ID NO: 365 /
SNP Position Genomic: 193651 / Related Interrogated SNP: hCV30690780 /
Related Interrogated SNP: hCV31523650 / Related Interrogated SNP: hCV233148 /

Related Interrogated SNP: hCV31523608 / SNP Source: dbSNP; HapMap; HGBASE / Population(Allele,Count): Caucasian (A,102|G,18) / SNP Type: INTRON / Context (SEQ ID NO: 1168): /
TTCTTTAAATATGGATATATTTAAAACAAGAAGTAAATAATTGAGAACTACAGAGTTCTATTTTAAACAAAACCCAGTGCGAA CTCACAGCTTTCTATGG
W
TCTCCACTCAAGATTATTTACAGCCTGACCTTAGTCTTCCTTTCCAATCCTTCACCCATTCTGGCCACATTAACCACCTCAAC TGGACTATATAATTTTC / Celera SNP ID: hCV804128 / Public SNP ID: rs68193059 / SNP Chromosome Position: 243828306 / SNP in Genomic Sequence: SEQ ID NO: 365 / SNP Position Genomic: 186771 / Related Interrogated SNP: hCV31523608 / SNP Source: dbSNP; HapMap; ABI_Val; HGBASE / Population(Allele,Count): Caucasian (T,18|A,102) / SNP Type: INTRONIC_INDEL;INTRON / Context (SEQ ID NO: 1169): /
GTTGGTAAGGACTTTGGATTTAATTATAGATAGGCTCCCCAGTAGCTCTGCATCGACTGACACCTGATGTCTGATGACTCTCA AACCTGCTTCTTCAGTC
M
CGGCCCTTCTCATCAAGCTCCATCCCCATATGGCAAAACTAGCATCTCCTTTGATGCCCTATACTGCCTCATCCTCAAGATAT CCAAAAGGGTTTTGTTT / Celera SNP ID: hCV804132 / Public SNP ID: rs406323 / SNP Chromosome Position: 243839798 / SNP in Genomic Sequence: SEQ ID NO: 365 / SNP Position Genomic: 198263 / Related Interrogated SNP: hCV31523608 / SNP Source: dbSNP; HapMap; HGBASE / Population(Allele,Count): Caucasian (A,25|C,201) / SNP Type: INTRON / Context (SEQ ID NO: 1170): /
ACAAACATTTGACAAGGGTACTGAAAATACATAAGGGAGAAAGAACAATCTCTTCAATAAATAGTGCTAGGAAAACTGGATAT ACACATGCAAAAGAATG
Y
AATGGAGTCTTATCTTTCATCATATCAAAAAAACCAACTCAAAATGGATTAAAAAACTTAAACGTAAGACCTGAAACTATAAAAC TCCTAGAAGAAAACATA / Celera SNP ID: hCV26719140 / Public SNP ID: rs320323 / SNP Chromosome Position: 243752002 / SNP in Genomic Sequence: SEQ ID NO: 365 / SNP Position Genomic: 110467 / Related Interrogated SNP: hCV31523608 / SNP Source: dbSNP; HGBASE / Population(Allele,Count): Caucasian (T,202|C,24) / SNP Type: INTRON / Context (SEQ ID NO: 1171): /
GCTAAAATAGGGAGCTTATCTTTATCAAGTTAAGTTCCTTTATAAAGGCAAAGACTACATAGTTTTTTTTTTATTGTTAAGCTT AGCCATGCTATAGGAAT
R
CAGTAATGTAAAATGCATTAACTCAAACTACTAAATATACAAGCATATTTCATCATTCTATTAGGAAGAAATTTTCTCCATAA CTCTCTCTCATAATAGC / Celera SNP ID: hCV804139 / Public SNP ID: rs320302 / SNP Chromosome Position: 243761735 / SNP in Genomic Sequence: SEQ ID NO: 365 / SNP Position Genomic: 120200 / Related Interrogated SNP: hCV31523608 / SNP Source: dbSNP; HapMap; ABI_Val; HGBASE / Population(Allele,Count): Caucasian (A,202|G,24) / SNP Type: INTRON / Context (SEQ ID NO: 1172): /
TCAATTGTGTCTGAACAGAAAGTATAATTAACAACGAGTGAAGGAAAGCATAAACATGAAATCATCTCAGTCTTATTTGTTTT ATAGGAGTCAGTTTTAA
R
TGCTCAATTTGAAAGCCAATGTCATATCGTACTATAGTTCCTAAGTTAATTAGAATAGCAAACATTGTAAAACAAAATAAATC AGAAGCAGAAATGACAC / Celera SNP ID: hCV804146 / Public SNP ID: rs320308 / SNP Chromosome Position: 243780198 / SNP in Genomic Sequence: SEQ ID NO: 365 / SNP Position Genomic: 138663 / Related Interrogated SNP: hCV31523608 / SNP Source: dbSNP; HapMap; HGBASE / Population(Allele,Count): Caucasian (G,206|A,20) / SNP Type: INTRON / Context (SEQ ID NO: 1173): /
AACCCCAAGAGAGAAGTGAGGATGGCTTGGACTACTATGGTGGCAGCGGAAAAAGAAAAAGGGGATAGTTCTGAGATATAAAG TTAACAGTGCTAATATA
R
AAGTTACATGCCTCCAACATGAGAGTGAATCACAGACATTTTAATAGGCAGTTCAGAAAGAAATTCACAGAAGATAACATCTA CCTGTTGTGGTTTAAGA / Celera SNP ID: hCV804147 / Public SNP ID: rs320309 / SNP Chromosome Position: 243781174 / SNP in Genomic Sequence: SEQ ID NO: 365 / SNP Position Genomic: 139639 / Related Interrogated SNP: hCV31523608 / SNP Source: dbSNP; HapMap; ABI_Val; HGBASE / Population(Allele,Count): Caucasian (A,202|G,24) / SNP Type: INTRON / Context (SEQ ID NO: 1174): /

ATAATATACTACACTGATGACATTATGACGGTATCAGAAACCTAAGAACAAGCTAGGGCTAACTTGAAAATAGTGGTGGCTCA
CAGGAATACCACAGGCT
R
GAGGATAAATCCAGCAAATACCCCAGGGTCTAACCAAACTGTGAAATTCTTACGAATAACCTGGGCAGGAACCACCCATAACA
TTCTACAGGCAACTAAA / Celera SNP ID: hCV804156 / Public SNP ID: rs320316 / SNP
Chromosome Position: 243790947 / SNP in Genomic Sequence: SEQ ID NO: 365 /
SNP Position Genomic: 149412 / Related Interrogated SNP: hCV31523608 / SNP
Source: dbSNP; HapMap; HGBASE / Population(Allele,Count): Caucasian
(G,24|A,202) / SNP Type: INTRON /
Context (SEQ ID NO: 1175): /
TTAAAAGTAGATTAACAGCTACTATTAAAAGTAAATTAACAACAATAATAAAATAGAAAACTTATAACAATATATTGTGATAC
AAGTTATTGTGATGCAT
R
TGGTCTCTCTCTCAAAATATCTTATTTTACTATACTCATCTATATTTAGGCTGTGGTTGATTGGGGGTAACTCAAACCATGAA
AAGCAAAACTGCAGCTA / Celera SNP ID: hCV804160 / Public SNP ID: rs320331 / SNP
Chromosome Position: 243797817 / SNP in Genomic Sequence: SEQ ID NO: 365 /
SNP Position Genomic: 156282 / Related Interrogated SNP: hCV31523608 / SNP
Source: dbSNP; Celera; HapMap; HGBASE / Population(Allele,Count): Caucasian
(A,19|G,101) / SNP Type: INTRON /
Context (SEQ ID NO: 1176): /
ACATAAACATCACTCTAAAACATCTTCTCTTAGAATTTTTTACTCCAACTGAGTCTCCAAAAAGAAAAATCCATAGAGGTATT
TTGAATTTGGGGTTCAA
W
TACTTAACCATATCAGATTTACACTATTCTTCATTTCATTTTAAATGAAATAATTTACACATGTACAAAAGAAGCTTTCTTAT
CTCTGAGTAGGTAGGTG / Celera SNP ID: hCV804166 / Public SNP ID: rs320334 / SNP
Chromosome Position: 243808032 / SNP in Genomic Sequence: SEQ ID NO: 365 /
SNP Position Genomic: 166497 / Related Interrogated SNP: hCV31523608 / SNP
Source: dbSNP; HapMap; HGBASE / Population(Allele,Count): Caucasian
(A,18|T,102) / SNP Type: INTRON /
Context (SEQ ID NO: 1177): /
GTGTGAAAGAGCAAGAAAAAAAAGGTCACAAAAGGGTCCTAGAGACAAACTTTTCCTACTTTGTAGTATTTCTTACTATTAGT
CCCATCTAACAGAATAA
S
CTCTACATAAATTCATTGAAAATTCATAAATTCATTAGTAAACCAGTATATCAATTTAAGTTTATTTTTTACACTGTGAAATA
TAAACACCTGCCATAGT / Celera SNP ID: hCV1678656 / Public SNP ID: rs1458024 /
SNP Chromosome Position: 243937445 / SNP in Genomic Sequence: SEQ ID NO: 365 /
SNP Position Genomic: 295910 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap;
HGBASE / Population(Allele,Count): Caucasian (G,102|C,18) / SNP Type: INTRON
/
Context (SEQ ID NO: 1178): /
TCTCTCCTCTATGTCTCTTTTTGACTGCACCTAGCACAGTGCCTGAAGGTGAGAAGCACTCAATAAAGATTTGTGAGTTGAAC
AGCCAACTCCATCAATA
R
TAAAATGATAGAATTTCTGGGGTAGGAAAAGCCTTAGAGGGCATTATCCTATCCCATTATTTAATAAACAGGAAAACTGAAAC
CTAAGAGAGGTTAACTG / Celera SNP ID: hCV1678658 / Public SNP ID: rs897960 /
SNP Chromosome Position: 243933568 / SNP in Genomic Sequence: SEQ ID NO: 365 /
SNP Position Genomic: 292033 / Related Interrogated SNP: hCV31523608 / SNP
Source: dbSNP; Celera; HapMap; HGBASE / Population(Allele,Count): Caucasian
(G,24|A,202) / SNP Type: INTRON /
Context (SEQ ID NO: 1179): /
AACTCTAAAATTCAGCTTTAATGCAGGTATCAAAAACTTCAAAAAAACAAATTCCCTATGTGGAATCAAAAAGATCTTTCAGG
TTCATTCGATGATTTTT
Y
ACTTTGCTTTGTTTCTGTTTCCATTTAGCCCTATTTTAATGCTTATTTTACTAAACTCAAACATAGGGAGTACTTTATTTTCT
AACATGTTTGTCTCCGT / Celera SNP ID: hCV1678668 / Public SNP ID: rs1379700 /
SNP Chromosome Position: 243909402 / SNP in Genomic Sequence: SEQ ID NO: 365 /
SNP Position Genomic: 267867 / Related Interrogated SNP: hCV31523608 /
Related Interrogated SNP: hCV30690780 / SNP Source: dbSNP; Celera; HapMap;
ABI_Val; HGBASE / Population(Allele,Count): Caucasian (T,57|C,169) / SNP Type:
INTRON /
Context (SEQ ID NO: 1180): /

TTCATCACTATTCATAGATGCCTGTCACTATTCAGAAAAAGAAGAAAGAAAGAGCCATACTTTCAAAGCTATTTTCTTTTTCT
ATATGTCCTAGTAGAGG
Y
GAATATAGAATTTATCACTTTGAAGCCAATGTAGTCTGAATCAACCGGTCCTCCATCTCCAGATCTAAGAATGCAAGTTAAAT
GTTCTCTAGAAATTATA / Celera SNP ID: hCV1678674 / Public SNP ID: rs1458023 /
SNP Chromosome Position: 243886673 / SNP in Genomic Sequence: SEQ ID NO: 365 /
SNP Position Genomic: 245138 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap;
HGBASE / Population(Allele,Count): Caucasian (T,184|C,36) / SNP Type: INTRON
/
Context (SEQ ID NO: 1181): /
CCTTCCACTGAATTTACTACTAAACATTGCAATGTTTCTTTCAAATTATAAGAAAGTTACTCCATACAAAAAGAAAAAAGGAC
ACAACTCATTATTCTGG / G /
TTTTTTTAATTTTAGATTCAGAAGGTACATGTGCAGGTTTGTTACATGGATATACAGCATGATGCTGAACACCTCATTATTTT
GACATCAGGCCTTCCCC / Celera SNP ID: hDV90784784 / Public SNP ID: rs320339 /
SNP Chromosome Position: 243870910 / SNP in Genomic Sequence: SEQ ID NO: 365 /
SNP Position Genomic: 229375 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap;
HGBASE / Population(Allele,Count): Caucasian (G,189|T,35) / SNP Type: INTRON
/
Context (SEQ ID NO: 1182): /
TGATTTTGTGTTGTGCTTCCGAGATGTCTTAAAGGCTTTCCAGATGTACCCACATTCCTCTGCTTTGCCACATATGTTCCCTC
ATGATTATTTTGAAAAG
Y
ATATTATAGTCAAGAAACTCTCATTCTAATGCATAATGCATGAACTTGTCATTTTTACATTAAGTACAGTTTTCACTTCATCT
TAGCCAAAAGTCCGAGA / Celera SNP ID: hCV1678683 / Public SNP ID: rs1486475 /
SNP Chromosome Position: 243868398 / SNP in Genomic Sequence: SEQ ID NO: 365 /
SNP Position Genomic: 226863 / Related Interrogated SNP: hCV31523608 /
Related Interrogated SNP: hCV30690780 / SNP Source: dbSNP; Celera; HapMap;
ABI_Val; HGBASE / Population(Allele,Count): Caucasian (T,57|C,169) / SNP Type:
INTRON /
Context (SEQ ID NO: 1183): /
TCCATGATGCTACAAGAGCTAGAAGGCTATCTGGTATTGATTCAGTTCTCTACTGGTTATTTCTACCTGCTGATTAAATCTCA
AATGCTTAGGCAAAGAA
K
TACAAAAAAATTGCAAACAGTTCAATTTTCCCTACAAATCAGCTTCTTCTTGTGACCCAAACTCAAACCCAACATCTGAAGGT
TCCCCTCCTTATCACAG / Celera SNP ID: hCV1678687 / Public SNP ID: rs320305 /
SNP Chromosome Position: 243859909 / SNP in Genomic Sequence: SEQ ID NO: 365 /
SNP Position Genomic: 218374 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HGBASE /
Population(Allele,Count): Caucasian (G,195|T,31) / SNP Type: INTRON /
Context (SEQ ID NO: 1184): /
CTAGGTAGTCTGCAAAGATGGCTGCCACAAATTCCCATTTCTCCCTGTATGCACATTCCACTCTTCCCATGAAGGGGTGGAGT
CAACTTTCTCTCTCCCC
R
GGCTAACCCTGTATCTTGCTTTGAACACAGAATATGATCAAACTGATACTATGCCAGTAACAGACAAAGCCTTTAAGCCTGGC
AGTTTATGCGTTCTCTC / Celera SNP ID: hCV1678723 / Public SNP ID: rs1486472 /
SNP Chromosome Position: 243803714 / SNP in Genomic Sequence: SEQ ID NO: 365 /
SNP Position Genomic: 162179 / Related Interrogated SNP: hCV31523608 /
Related Interrogated SNP: hCV30690780 / SNP Source: dbSNP; Celera; HapMap;
HGBASE / Population(Allele,Count): Caucasian (G,71|A,155) / SNP Type: INTRON
/
Context (SEQ ID NO: 1185): /
GCTCTGTGAGTGCCCTGGGGACTTCAGGCAGTAAGTGCTTGGAATGATTAATATGAAAAATGCATCTGGAAATGAAAACGAAG
AGGAGTCTCATTCTCCT
Y
ACAAAATGACCCTGTGCTCAAGGAACCTGTAAGTACTGACTTTTCCCACTCGGTCCAAAATCCCTCTGCCAAACATGTGTTCA
CAGACAGTAAATGAGCA / Celera SNP ID: hCV8688079 / Public SNP ID: rs884808 /
SNP Chromosome Position: 243653439 / SNP in Genomic Sequence: SEQ ID NO: 365 /
SNP Position Genomic: 11904 / Related Interrogated SNP: hCV233148 / Related

Interrogated SNP: hCV30690780 / SNP Source: dbSNP; Celera; HapMap; HGBASE /
Population(Allele,Count): Caucasian (C,169|T,57) / SNP Type: INTRON /
Context (SEQ ID NO: 1186): /
CAGGGAGGGGCCCCAGCAGCACCTGCTTTAGGCCTGGCCAGCCAAAGATCAGGACCACTGGAGCCACTAGGTCTCCTGGCAGGT
GACTAGGCTCTTGTCAC
R
TAGGAGGGAGGGACCGTGACTCCTGGCATGAGTGAATCTTCAGCCTCTAAAGCCAAGAACTCTGTCAGACCTCAGGTTGTGGG
GAGTCTTCCGTGGAAGC / Celera SNP ID: hCV8688080 / Public SNP ID: rs884328 /
SNP Chromosome Position: 243654278 / SNP in Genomic Sequence: SEQ ID NO: 365 /
SNP Position Genomic: 12743 / Related Interrogated SNP: hCV233148 / Related
Interrogated SNP: hCV30690780 / SNP Source: dbSNP; Celera; HapMap; HGBASE /
Population(Allele,Count): Caucasian (A,169|G,57) / SNP Type: INTRON /
Context (SEQ ID NO: 1187): /
CAGCCTCCAGAACTGTGAGAAAACACCTAGACTATGAAATTTTGTTATGGCAGCTTGAACAGACTAATTCAGATGGAAACTAT
TATTTCCCCCATTTTAC
R
GAAGAAAAAATTATAGCTTACACAGACTAAATAACCTCCCTACAGTGGATCCAAGAGATAGTGATAGGGCCAGAATTTAAACT
CAACTTTTAACCAGTAC / Celera SNP ID: hCV8688098 / Public SNP ID: rs1531244 /
SNP Chromosome Position: 243939970 / SNP in Genomic Sequence: SEQ ID NO: 365 /
SNP Position Genomic: 298435 / Related Interrogated SNP: hCV31523608 /
Related Interrogated SNP: hCV30690780 / Related Interrogated SNP: hCV31523650
/ SNP Source: dbSNP; HGBASE / Population(Allele,Count): Caucasian
(A,62|G,164) / SNP Type: INTRON /
Context (SEQ ID NO: 1188): /
TAGTGTCATTAACTGCAAACAACATATTTTTCTGACAAATGCACTAAACTTGAAACAAATCTTTATTTGAAAACCAATTTGCT
TTCAGAAAATGTAGTAT
Y
CAGTGATCTTAAACAAGAGGATAAAGTTTCTGGGTTCAAGACATCAATTTCATCTCACTGAAAATATGCAACTTCAAGTCCGT
ATGGTGACAGCAACGTT / Celera SNP ID: hCV8688110 / Public SNP ID: rs946824 /
SNP Chromosome Position: 243684019 / SNP in Genomic Sequence: SEQ ID NO: 365 /
SNP Position Genomic: 42484 / Related Interrogated SNP: hCV31523608 / SNP
Source: dbSNP; HapMap; HGBASE / Population(Allele,Count): Caucasian
(T,24|C,202) / SNP Type: INTRON /
Context (SEQ ID NO: 1189): /
TCGTTTTCAGTCCATTCAAAATACTTTCTCATTTCCCTTTGATTTCTTCTTTGATCCATGGATTAATATTTAGTTTCCAAGAA
CTTGGGGATTTTCCATA
S
ATCTGTGTTTTTTTAATTTCATTTCCTTCAAAGAGAAAATGTTTTACAGTACTTAAATCCTTTAAAATTCATGGAGTTGTTTT
GCTCAACATATGGTACA / Celera SNP ID: hCV8688111 / Public SNP ID: rs1578275 /
SNP Chromosome Position: 243680442 / SNP in Genomic Sequence: SEQ ID NO: 365 /
SNP Position Genomic: 38907 / Related Interrogated SNP: hCV30690780 /
Related Interrogated SNP: hCV233148 / Related Interrogated SNP: hCV31523650 /
Related Interrogated SNP: hCV30690777 / SNP Source: dbSNP; Celera; HapMap;
HGBASE / Population(Allele,Count): Caucasian (G,184|C,40) / SNP Type:
INTRON;PSEUDOGENE /
Context (SEQ ID NO: 1190): /
TGTAAATACACTCATTGAGAAATTGAGAAAAAGAGCAGAATAATCACATCATTTAAATCAACTTGTCTATCTCAGCTATATCT
TATTGAGCCCACTTCTT
Y
GAATTCATATAAATTACATGCAACTATGATGAAATTCCATTGTACCAATACGACCGTAAGAAGTTAGGGCTGATTTTTTCACAG
CCAACATTTCTTTCACA / Celera SNP ID: hCV8688770 / Public SNP ID: rs3856231 /
SNP Chromosome Position: 243768906 / SNP in Genomic Sequence: SEQ ID NO: 365 /
SNP Position Genomic: 127371 / Related Interrogated SNP: hCV31523608 /
Related Interrogated SNP: hCV30690780 / Related Interrogated SNP: hCV31523650
/ SNP Source: dbSNP; Celera / Population(Allele,Count): Caucasian
(T,61|C,165) / SNP Type: INTRON /
Context (SEQ ID NO: 1191): /
CTTTGTATAGCAGCATGACATTTTTTGAAATATGAAAGTTAAAAAACACAAAATATATATTCCACAAAAAATAAGCAAAAATA
TTGTTCATTTGAAAGAC
K
TAAGTTTTAAAATACCAAAGACATTATACTAATTTATACTAATAAAGAGCCAACCAATTCCTATGACTGACTCTGCCCATCAG
TCTGATTCTCTTTGAAG / Celera SNP ID: hCV8688837 / Public SNP ID: rs320318 /
SNP Chromosome Position: 243832330 / SNP in Genomic Sequence: SEQ ID NO: 365 /

SNP Position Genomic: 190795 / Related Interrogated SNP: hCV31523608 / SNP Source: dbSNP; Celera; HGBASE / Population(Allele,Count): Caucasian (T,24|G,202) / SNP Type: INTRON /
Context (SEQ ID NO: 1192): /
TTGGCAATTAATCCTATTTTTCCTCCCACTGCTATATGTTCTATGTGAATGTCTTCCATAGTCAATGACAAATTCTTACATAT
AAAAATTTTATTATATA
Y
TCCTTTCACTATCAGGTATGGGTATAATGATCAATCAACACTTCGAAGGTGAATTATTATGATCTCTCATAATTCATGTACAT
TTATATAGTATATATTT / Celera SNP ID: hCV8688866 / Public SNP ID: rs1531243 /
SNP Chromosome Position: 243852498 / SNP in Genomic Sequence: SEQ ID NO: 365 /
SNP Position Genomic: 210963 / Related Interrogated SNP: hCV31523608 / SNP Source: dbSNP; HGBASE / Population(Allele,Count): Caucasian (T,24|C,202) /
SNP Type: INTRON /
Context (SEQ ID NO: 1193): /
AGGCCACTTTTCATTTAAACATTAACATTTATTATTTTCATTTAAAATGTTCAATGTGAAATTGTTAATATGTTAATATGTGT
TTTAATACATTAGTTTA
Y
AAGTAATAATAAGGTCTGAAAAACAATTTATAAAAATGACTATATGCTGTTATGCTCAAAATAAATCATAAACATTTTTCCCA
GCTCACACCAGGCCCTT / Celera SNP ID: hCV8689005 / Public SNP ID: rs1458022 /
SNP Chromosome Position: 243886295 / SNP in Genomic Sequence: SEQ ID NO: 365 /
SNP Position Genomic: 244760 / Related Interrogated SNP: hCV31523608 / SNP Source: dbSNP; HGBASE / Population(Allele,Count): Caucasian (C,24|T,202) /
SNP Type: TRANSCRIPTION FACTOR BINDING SITE;INTRON /
Context (SEQ ID NO: 1194): /
TTCTGAGAAATGCATCATTAGGCAATTTCCTCACTGAGTGAACATCATAGAAACACCTAGTTTTTAGGCTACAAATCTGAACA
TGTTACTGTACTGAATA
Y
GCAGTTCTAACACAATGGTATCTGTGTATCTAGACAAATCTAAACATAGAGAAGGTACAGTAAAAATACAGTACACAGGGTAA
AAAAAAAAGTGGCACAC / Celera SNP ID: hCV8689016 / Public SNP ID: rs897959 /
SNP Chromosome Position: 243897774 / SNP in Genomic Sequence: SEQ ID NO: 365 /
SNP Position Genomic: 256239 / Related Interrogated SNP: hCV31523608 /
Related Interrogated SNP: hCV30690780 / Related Interrogated SNP: hCV31523650
/ SNP Source: dbSNP; HapMap; HGBASE / Population(Allele,Count): Caucasian (C,63|T,163) / SNP Type: INTRON /
Context (SEQ ID NO: 1195): /
TATATTATCAAATTTGAAAATGTTTAGAGAATTTATGTGCTCAAAAGTATACATAAATGTCTCCAGTCCCACTTCCCACTTAG
TACTCGTTGAGGATGAA
Y
AGATAATGACACGGAAATAAAGATGGTAAACCATTACACACTGTAAAATTTAGTGTTCAGTTACAATCTAAGAAATGCAAAAC
TATGTAACTCTAAAGAA / Celera SNP ID: hCV8689017 / Public SNP ID: rs1458021 /
SNP Chromosome Position: 243901970 / SNP in Genomic Sequence: SEQ ID NO: 365 /
SNP Position Genomic: 260435 / Related Interrogated SNP: hCV31523608 / SNP Source: dbSNP; HapMap; ABI_Val; HGBASE / Population(Allele,Count): Caucasian (C,24|T,202) / SNP Type: INTRON /
Context (SEQ ID NO: 1196): /
GTTCTCCAAATGTCCCATATTTCAAAATTTAAGCCCTATATCACCATCCGATTTCACAGGCAACACAAAGAATGCGAACTGCT
TCTAAGGAGTAAGTGAA
W
GAACATGCCTAAACCTTTTTAAAAGTAATTAAGTTCCATGACTATGAACTTAAATAAATGTATAATCTATGGGGTCTTAAGCA
GTGTAAAAAATTAAAGT / Celera SNP ID: hCV8689027 / Public SNP ID: rs1545654 /
SNP Chromosome Position: 243916478 / SNP in Genomic Sequence: SEQ ID NO: 365 /
SNP Position Genomic: 274943 / Related Interrogated SNP: hCV31523608 / SNP Source: dbSNP; HapMap; ABI_Val; HGBASE / Population(Allele,Count): Caucasian (A,24|T,200) / SNP Type: INTRON /
Context (SEQ ID NO: 1197): /
CAATTATATCAATGTTTGTTTTACGGCTAAAAAACTGAAAACTTCACTGGTAAGATATGAACACATGTTTAGATCCTATGCTT
TTCTAGAAATGTTTTGT
R
AATCACAAACTAACAAAAAAACATTTTCTGGAAGAACAACAAATTCTAGTTTTCATCATCAGCGCAAAGAATGTCATATTTTT
ACTTGTTTGAAATGAGT / Celera SNP ID: hCV9115290 / Public SNP ID: rs1352162 /
SNP Chromosome Position: 243717682 / SNP in Genomic Sequence: SEQ ID NO: 365 /
SNP Position Genomic: 76147 / Related Interrogated SNP: hCV31523608 /
Related Interrogated SNP: hCV30690780 / Related Interrogated SNP: hCV31523650

/ SNP Source: dbSNP; Celera; HapMap; HGBASE / Population(Allele,Count): Caucasian (A,63|G,157) / SNP Type: TFBS SYNONYMOUS;INTRON / Context (SEQ ID NO: 1198): /
GTCAGACTGCAGTCCAGCCATCCTACCCATCTACATCACCCACGTCTAAGTTTGTTAATTTGCCATGACATGTTGTTAGAAAT ACACTCTAAGAAAGGAA
R
TATGCAGAGCAGTACATTATCAGGAAGACGTTAATGAAAAGCTACATTCCTTCAGATTCTGGGTCCAAACCGTGTGTTGTATA GATGATTCGGGTCTGAA / Celera SNP ID: hCV9493073 / Public SNP ID: rs1058305 /
SNP Chromosome Position: 243664642 / SNP in Genomic Sequence: SEQ ID NO: 365 / SNP Position Genomic: 23107 / Related Interrogated SNP: hCV233148 / Related Interrogated SNP: hCV30690780 / Related Interrogated SNP: hCV31523650 / SNP Source: dbSNP; Celera; HGBASE / Population(Allele,Count): Caucasian (A,166|G,60) / SNP Type: UTR3;INTRON /
Context (SEQ ID NO: 1199): /
GAGACCTCCTTCATCCCTGGCTTCACAGTACATCCATCCTAAATCCAGTGCTGAGAGGTCAGCGTTTCCCCTCAGAAGCAGCC GTTCATCAAAGTTTGCA
Y
AACCGCACTACTGCCATTTCACTGAAGTAAAAATGATCCCCTATGTGTGTGTGTGTGAGCTACAAGGAATTATTTCTATATTT TCCTGTCACATTTTATA / Celera SNP ID: hCV9493081 / Public SNP ID: rs1058304 /
SNP Chromosome Position: 243664857 / SNP in Genomic Sequence: SEQ ID NO: 365 / SNP Position Genomic: 23322 / Related Interrogated SNP: hCV233148 / Related Interrogated SNP: hCV30690780 / Related Interrogated SNP: hCV31523650 / SNP Source: dbSNP; Celera; HapMap; HGBASE / Population(Allele,Count): Caucasian (C,166|T,60) / SNP Type: UTR3;INTRON /
Context (SEQ ID NO: 1200): /
TATATATATATATATAACATTTCCCAACATACCTTAGAAATACCTTGTAAAATTATCTTTTTAAAAATGTACTAAAGGGGGCT AGGGCAAAGGAAAAAGC
R
ATGGTGAAGTAACCCTCTCTTGCACCAATTGTTCCTTCCTTAATTCCTCATAAAGACATTCTTCATTGCCCTCTGCCTGGAAT ATTCTTCTTCTCTGTTT / Celera SNP ID: hCV12073160 / Public SNP ID: rs1973284 /
SNP Chromosome Position: 243856681 / SNP in Genomic Sequence: SEQ ID NO: 365 / SNP Position Genomic: 215146 / Related Interrogated SNP: hCV31523608 / Related Interrogated SNP: hCV30690780 / Related Interrogated SNP: hCV31523650 / SNP Source: dbSNP; HapMap; ABI_Val; HGBASE / Population(Allele,Count): Caucasian (A,60|G,164) / SNP Type: INTRON /
Context (SEQ ID NO: 1201): /
TGTTAGTTCAAGACAGAACTAAGAGTCCCTACTAAAGCTTGAAGTAATCTGTCTTTAGAAGAAAGACTGTGACTCCACCCAGG AAATTGGGCATAATTAC
R
TTAAGAAGGAATGGCTTCATCCAGAAAGTACCACTAATAATTTTCTACAATAAATCTCCATTTTGGCTTAACAGAAATAAACT TGGTCACCCACTACTAG / Celera SNP ID: hCV12073167 / Public SNP ID: rs2034915 /
SNP Chromosome Position: 243878503 / SNP in Genomic Sequence: SEQ ID NO: 365 / SNP Position Genomic: 236968 / Related Interrogated SNP: hCV31523608 / Related Interrogated SNP: hCV30690780 / Related Interrogated SNP: hCV31523650 / SNP Source: dbSNP; HapMap; HGBASE / Population(Allele,Count): Caucasian (A,61|G,163) / SNP Type: INTRON /
Context (SEQ ID NO: 1202): /
GCGGAGCTGGGACCATCTTACTGTGCCAGAGAGTAAGAAGGTGCTCAGAGAATGATGGGGACATGTTAAAGGGCACAGAAGTT TGAAGGGAATCCCGCTG
R
CCACATCTGAGACAATCTGGATATCAAAATACATAATACGAAAACACTATGACTCATTTGTTGAATAAAACAAGAATCCATGA GCCCATATTGATAAAAA / Celera SNP ID: hCV12073172 / Public SNP ID: rs971285 /
SNP Chromosome Position: 243919773 / SNP in Genomic Sequence: SEQ ID NO: 365 / SNP Position Genomic: 278238 / Related Interrogated SNP: hCV31523608 / Related Interrogated SNP: hCV30690780 / Related Interrogated SNP: hCV31523650 / SNP Source: dbSNP; HapMap; HGBASE / Population(Allele,Count): Caucasian (G,63|A,163) / SNP Type: INTRON /
Context (SEQ ID NO: 1203): /
AATATCATAATTTAGACAGAGGAATGCTTATCTTCAGTGAGTAAAAGAAAATAAACCCTGACCTAGAACTGTCTTACTCAGTA AAAACATCCTTTAAAAA
W
ATGGAAAAAATGAATGCATTTTCGCACAAATAAAAACTAAAAGACCATCAATGGATAAATGGATAAACAATATGTGGCATATA CATACAATGGAACATTA / Celera SNP ID: hCV12073180 / Public SNP ID: rs1870272 /

SNP Chromosome Position: 243924743 / SNP in Genomic Sequence: SEQ ID NO: 365 / SNP Position Genomic: 283208 / Related Interrogated SNP: hCV31523608 / SNP Source: dbSNP; Celera; HapMap; HGBASE / Population(Allele,Count): Caucasian (T,36|A,84) / SNP Type: TRANSCRIPTION FACTOR BINDING SITE;UTR5;INTRON / Context (SEQ ID NO: 1204): /
AGGCTGAGATCAAGTGCTCAGGATCCTTGGCCTCTCCACTTGGAGTCATGAGTGTAGATTTTAAAAATCATGCAGGCGGAACA
TTTCCAGTCAGGAGAGC
R
CTGAGCCCAGCCCTGGGTCACACCAGCATTTGAAGGTCTGATGGAGGAGGAAGAGGGACCAAAGGGGAGTGAGAGACCTGTAC
AGAGTCTAAGAAGGAGA / Celera SNP ID: hCV12073836 / Public SNP ID: rs1008173 /
SNP Chromosome Position: 243644497 / SNP in Genomic Sequence: SEQ ID NO: 365 / SNP Position Genomic: 2962 / Related Interrogated SNP: hCV233148 / Related Interrogated SNP: hCV30690780 / SNP Source: dbSNP; Celera; HGBASE / Population(Allele,Count): Caucasian (A,180|G,46) / SNP Type: INTRON / Context (SEQ ID NO: 1205): /
TATCTGTACACAGATAATTATTTGTCTAAAATAGTATTTCTACTATACACAATTAAGCCCTCAAATGCTTTAAAGTAATAAAA
ACGGACACTGGACATAC
Y
GTGTTGTATCTGAGAATGACAAACACTAAAGGCAAGGCTGCAGTTAGTTAGGACAGGTCCCTGACCTTCGGCTGCCCTGCCTG
GCCAGCGAGCCATCATC / Celera SNP ID: hCV12073840 / Public SNP ID: rs14403 /
SNP Chromosome Position: 243663893 / SNP in Genomic Sequence: SEQ ID NO: 365 / SNP Position Genomic: 22358 / Related Interrogated SNP: hCV233148 / Related Interrogated SNP: hCV30690780 / SNP Source: dbSNP; Celera; HGBASE / Population(Allele,Count): Caucasian (C,172|T,54) / SNP Type: UTR3;INTRON / Context (SEQ ID NO: 1206): /
GCCTTCTTACATTAAACAGCTATCCTCCCCCTCACTTTCTATCTTCACATTGCTTTTTCTTCAAGACTTTGCGACATCTGATA
CACAGGCACGTATACAT
R
CACACATATGATATACATGTATTTGTTACTGTCAATGTTCATCACCAATAAAATATAAGTTCCACGAGGGCAAGGACTTTAAA
AAAAAGTTTATTGATGC / Celera SNP ID: hCV15760229 / Public SNP ID: rs3006939 /
SNP Chromosome Position: 243677834 / SNP in Genomic Sequence: SEQ ID NO: 365 / SNP Position Genomic: 36299 / Related Interrogated SNP: hCV233148 / Related Interrogated SNP: hCV30690780 / Related Interrogated SNP: hCV31523650 / Related Interrogated SNP: hCV30690777 / Related Interrogated SNP: hCV31523608 / SNP Source: dbSNP; HGBASE / Population(Allele,Count): Caucasian (G,180|A,46) / SNP Type: INTRON / Context (SEQ ID NO: 1207): /
CTCTCGACAGTAGAATCTGCTGCCTGTGACCATCTCTGGCACAGCATTAGCCTCCAATCGCCCCTCTCCCGGACACTGAGTCC
ACATCAGCACTATGGAT
R
AATCTGCCTCCAAAAAAGGAATGAGAGGAGAAGACATATAGGGAAGGAATCAAAAAGAAAAGGAAAGAAAAACTATGATGTGA
ACCCAAAGATGTGTAAC / Celera SNP ID: hCV15760238 / Public SNP ID: rs3006936 /
SNP Chromosome Position: 243674772 / SNP in Genomic Sequence: SEQ ID NO: 365 / SNP Position Genomic: 33237 / Related Interrogated SNP: hCV233148 / Related Interrogated SNP: hCV30690780 / Related Interrogated SNP: hCV31523650 / SNP Source: dbSNP; Celera; HapMap; HGBASE / Population(Allele,Count): Caucasian (A,96|G,24) / SNP Type: UTR5;INTRON / Context (SEQ ID NO: 1208): /
TGTGGCGCAGTGAGCCCAGGGGCTCAAGGTGTTAGCAGTCATCAACAGTGCTATTTGAAACTGTGGGTGGATGAAGAGGTGAA
CTCAACACCACAAATAT
S
ACAGAGCAGTAACCACCATGGAAAAGAAAAGAGAGCTTATGTGTCTAGGACACCCCAAGAAATCTCTTCTGAATGAATGATGG
CAATGAAATAGCCATTA / Celera SNP ID: hCV15760239 / Public SNP ID: rs3006923 /
SNP Chromosome Position: 243648757 / SNP in Genomic Sequence: SEQ ID NO: 365 / SNP Position Genomic: 7222 / Related Interrogated SNP: hCV233148 / Related Interrogated SNP: hCV30690780 / SNP Source: dbSNP; HapMap; HGBASE / Population(Allele,Count): Caucasian (G,163|C,59) / SNP Type: INTRON;PSEUDOGENE / Context (SEQ ID NO: 1209): /
AAGCTTTGGCAGAAAAACTTCTGGGAAAGCTTCACCAGAGAGTCCTCCTCCACCAAGATAATGCTCCTGCTAAATCCCTCTCA
TTAAACAGGGGTATTTC
R
TGAGAGCTTTCATGAGAAATCATTAGGCATCCACCTTACAGTCCTGATTTGGCTCCTTCTGACTTATTTTTTCCCCTAAACTTA

AAAAAGCTTTTAAAGAG / Celera SNP ID: hCV15760280 / Public SNP ID: rs3006940 / SNP Chromosome Position: 243683001 / SNP in Genomic Sequence: SEQ ID NO: 365 / SNP Position Genomic: 41466 / Related Interrogated SNP: hCV233148 / Related Interrogated SNP: hCV30690780 / Related Interrogated SNP: hCV31523650 / Related Interrogated SNP: hCV30690777 / Related Interrogated SNP: hCV31523608 / SNP Source: dbSNP; Celera; HapMap / Population(Allele,Count): Caucasian (A,180|G,46) / SNP Type: INTRON / Context (SEQ ID NO: 1210): / AAAATCGCCATTTTAAAGTATACAAGCCATAGTTTTTATTACATTCACAAGGCAGTGCAACTACCACTACTAATTCCAGAACT TTTCATCGCCCCAAATA Y

AAATCCAGAACTCATTAGCAGTCATTCTCCATTCCCTCATCCCCTCATCTCCTGACCATGAAACCACTTTCTGTCCCTAAGGA TTTTCCTATTTTGGATA / Celera SNP ID: hCV15776869 / Public SNP ID: rs2345994 / SNP Chromosome Position: 243932275 / SNP in Genomic Sequence: SEQ ID NO: 365 / SNP Position Genomic: 290740 / Related Interrogated SNP: hCV31523608 / Related Interrogated SNP: hCV30690780 / Related Interrogated SNP: hCV31523650 / SNP Source: dbSNP; HapMap; HGBASE / Population(Allele,Count): Caucasian (T,64|C,162) / SNP Type: INTRON / Context (SEQ ID NO: 1211): / GTGTGTAATTAGCAAGAGAACTGCCTAGGTTTAATTTATTGGCCTTGCACTGCAATCTAGTAAAATGCAAAACAAAATTAAAC AATGTGTCCACTTTGTA M

AGATATTTTTTCTTTAACTCAACAAAACACTAATGAATACAAGTTCATTGGAGTGTTTCTAAGTTAAGGCACAGTGTATTTTG AAAATTCCAACAGAATA / Celera SNP ID: hCV15823016 / Public SNP ID: rs2125229 / SNP Chromosome Position: 243877648 / SNP in Genomic Sequence: SEQ ID NO: 365 / SNP Position Genomic: 236113 / Related Interrogated SNP: hCV31523608 / SNP Source: dbSNP; HapMap; HGBASE / Population(Allele,Count): Caucasian (C,24|A,202) / SNP Type: TFBS SYNONYMOUS;INTRON / Context (SEQ ID NO: 1212): / GAAAACAACATAATTGCCAAAATTTGTTTTATAAAAGGCATTTCCCCCATCACATAACTTCACCATTTCTAGTAATCATCACA CCCCCAGACCAAAGCAC R

AAGTTAAAGAAGGCCCTTATAATACACATAAAAAGTCAACAACTTCCTTCAGACTAAGAAGAACAAATTCTAAACTAGAGAAC CCTGGATGAGAATGACC / Celera SNP ID: hCV15823024 / Public SNP ID: rs2125230 / SNP Chromosome Position: 243885848 / SNP in Genomic Sequence: SEQ ID NO: 365 / SNP Position Genomic: 244313 / Related Interrogated SNP: hCV30690780 / Related Interrogated SNP: hCV31523650 / Related Interrogated SNP: hCV233148 / Related Interrogated SNP: hCV31523608 / SNP Source: dbSNP; Celera; HapMap; ABI_Val; HGBASE / Population(Allele,Count): Caucasian (G,189|A,37) / SNP Type: TRANSCRIPTION FACTOR BINDING SITE;INTRON / Context (SEQ ID NO: 1213): / TAATACATACTTTTGATTGAATGAGCTAATCTTATTTGGGGAACATACCTGTTCTTGAAGATTTAGTTCAAATGCACCTTTCC TCCTAAAATCTCCTGTA Y

AGGAGAACATTTGTGGGGATGTTATTTTTATCGTGGTGATGATTTCATGGGTCAAAAGTTATCAGATTGTACACTTTAAATAT GTACAAATTATTTATAA / Celera SNP ID: hCV15823033 / Public SNP ID: rs2125231 / SNP Chromosome Position: 243762299 / SNP in Genomic Sequence: SEQ ID NO: 365 / SNP Position Genomic: 120764 / Related Interrogated SNP: hCV31523608 / Related Interrogated SNP: hCV30690780 / Related Interrogated SNP: hCV31523650 / SNP Source: dbSNP; HapMap; HGBASE / Population(Allele,Count): Caucasian (T,61|C,165) / SNP Type: INTRON / Context (SEQ ID NO: 1214): / CAATGAGGAACATAAAGAACTTCCAAGAATACCACAGTATTAGCATATTTGGTCAAAAAAAGATAAATTCCAGATACATTTAC AATGTAACAGCAAAACC M

AGTAAGTAGAAAGAGGAAAAGAGCTCTGACGAAGTTCACTCCTTTGCCTCCATCTCTGCCCACTTCTCCTGGTGCTCTTTCTC ACGGGAAGAGGTGGAGC / Celera SNP ID: hCV15885425 / Public SNP ID: rs2290754 / SNP Chromosome Position: 243806268 / SNP in Genomic Sequence: SEQ ID NO: 365 / SNP Position Genomic: 164733 / Related Interrogated SNP: hCV30690780 / Related Interrogated SNP: hCV31523650 / Related Interrogated SNP: hCV233148 / Related Interrogated SNP: hCV31523608 / SNP Source: dbSNP; Celera; HapMap; ABI_Val; HGBASE / Population(Allele,Count): Caucasian (A,187|C,39) / SNP Type: INTRON /

Context          (SEQ ID NO: 1215): /
TGGTGCTCTTTCTCACGGGAAGAGGTGGAGCTAATGTTATTAGTCAACTTCTACATTGGTTACGTCGCTTCTCCCTTTTAAAT
AAGCAGATTCATGCAGT
R
TGAGATCATGAGATTTCAGCAGGTCTGGCCATTGTTCTTTTTTTCCTTCTCTTTTTCTCTGGATACTGGCCACTGGTATGAGT
TACTACAGCCAGGAATT / Celera SNP ID:   hCV15885435 / Public SNP ID:   rs2290753 /
SNP Chromosome Position:   243806438 / SNP in Genomic Sequence:   SEQ ID NO: 365 /
 SNP Position Genomic:   164903 / Related Interrogated SNP:   hCV31523608 /
Related Interrogated SNP:   hCV30690780 / Related Interrogated SNP:   hCV31523650
/ SNP Source:   dbSNP; Celera; HGBASE / Population(Allele,Count):   Caucasian
(G,63|A,163) / SNP Type:   INTRON /
Context          (SEQ ID NO: 1216): /
TCACTGGCCTGGAAGCTTCTCTGAACACTTTGCAGGCCGAGGCATGGTTGTAACCTGTTTGTAGCACCCAGTCTGAGAAGCTA
GGCTTGAATGTAGGCCA
R
ACTAAATATTTGAAAGTTTAAAAACACCATATATATGGTATCATTTTAATTAAACGACTGACATTGTGATTAAACAAAGGAAT
GAAAATAGCAGTTCCTA / Celera SNP ID:   hCV15965328 / Public SNP ID:   rs2291409 /
SNP Chromosome Position:   243732197 / SNP in Genomic Sequence:   SEQ ID NO: 365 /
 SNP Position Genomic:   90662 / Related Interrogated SNP:   hCV31523608 /
Related Interrogated SNP:   hCV30690780 / Related Interrogated SNP:   hCV31523650
/ SNP Source:   dbSNP; HapMap; ABI_Val; HGBASE / Population(Allele,Count):
Caucasian (G,62|A,160) / SNP Type:   TFBS SYNONYMOUS;INTRON /
Context          (SEQ ID NO: 1217): /
TATTTTTTCTTAGGCCTACTGACTTTTTTCATTCTGGTTCCACCCATGTGGACTTCAAGTAACAACTCAGAGCTAGGGTGTGCT
GATTATAGAGCACATAA / N /
GTGCACTGTACATTCTTGCCTTAAGAAGAACTAAGTAATACAAATTAAACAGTGAAAGACAGTAGTAAACAAAGGCAATGTAA
TGGAGAAGTCTACAGGG / Celera SNP ID:   hCV15965338 / Public SNP ID:   rs2291410 /
SNP Chromosome Position:   243716654 / SNP in Genomic Sequence:   SEQ ID NO: 365 /
 SNP Position Genomic:   75119 / Related Interrogated SNP:   hCV30690780 /
Related Interrogated SNP:   hCV31523650 / Related Interrogated SNP:   hCV233148 /
Related Interrogated SNP:   hCV31523608 / SNP Source:   dbSNP; Celera; HapMap;
ABI_Val; HGBASE / Population(Allele,Count):   no_pop (G,-|T,-) / SNP Type:
INTRON;INTERGENIC;UNKNOWN /
Context          (SEQ ID NO: 1218): /
GAATGATCGGGTTCAAAAATGCTGAAGTGAGGAAACCATTCCATGAAGTCAAGAACCCTATTTTGACTTACAGTCTTCCAGCA
GAGCAGCTTGATATGTA
S
GAAATCATCAATAAATATTTGCCAAATTAATAAATGTATCACTCAAGTCCTTTTTAATTATTATTGGTTTTTTTTGGAGACAGA
GTCTCGCTGTGTCCCCC / Celera SNP ID:   hCV16082410 / Public SNP ID:   rs2881275 /
SNP Chromosome Position:   243918313 / SNP in Genomic Sequence:   SEQ ID NO: 365 /
 SNP Position Genomic:   276778 / Related Interrogated SNP:   hCV30690780 /
Related Interrogated SNP:   hCV31523650 / Related Interrogated SNP:   hCV233148 /
Related Interrogated SNP:   hCV31523608 / SNP Source:   dbSNP; Celera; HapMap;
HGBASE / Population(Allele,Count):   Caucasian (G,102|C,18) / SNP Type:   INTRON
/
Context          (SEQ ID NO: 1219): /
ACTCTAATTATGGGATGCTCTACAAAATAACTGGCATGTAATCTTTAAAAGCATAAAAGCCATAAAAGGAAAGACTATGAAGC
CATTCCACATGAAGAGA
S
TAAAGAGCCCTGACAATCAAATGCACAGATCCTGGGTTAGAATTTTGCAATGAAAGACATTATCGAAAGAATTGGCAAAATCT
GAATGGGGTCTGATGAT / Celera SNP ID:   hCV16082411 / Public SNP ID:   rs2881274 /
SNP Chromosome Position:   243920382 / SNP in Genomic Sequence:   SEQ ID NO: 365 /
 SNP Position Genomic:   278847 / Related Interrogated SNP:   hCV31523608 / SNP
Source:   dbSNP; HapMap; HGBASE / Population(Allele,Count):   Caucasian
(G,24|C,200) / SNP Type:   UTR3;INTRON /
Context          (SEQ ID NO: 1220): /
ACTTTTTCTCATTTTTCATAAAAGGCAACAGGCTGATGTTCATATATTTCTCTTGAATGCGGATTCAGTTTCACTCATTGCCC
TGTCTTGGAAATCCCCA
Y
GTTAATGATGGCTTAAATTAGGTTGATAGAGTCCTCCCTTGGAGTCCAGCTTGGAAAATCCTCACTGGAGAGGGGCAGGCCCA
CTTCTCTTTTTGGAAGA / Celera SNP ID:   hCV26034142 / Public SNP ID:   rs9428576 /
SNP Chromosome Position:   243662027 / SNP in Genomic Sequence:   SEQ ID NO: 365 /
 SNP Position Genomic:   20492 / Related Interrogated SNP:   hCV233148 / Related

Interrogated SNP: hCV30690780 / SNP Source: dbSNP; Celera; HapMap; ABI_Val / Population(Allele,Count): Caucasian (T,119|C,107) / SNP Type: UTR3;INTRON / Context (SEQ ID NO: 1221): /
ATCAAGAATGACAAATGCTCCCGGCTCTCGGGCGGTTCCCGGCCAATGGAGGAGAGACTCACTTGTAAAAAAAACATGCTCAA CTCAAACAGGACACTGA
R
GCATGAAGAGAAATGGCCAATATTGCAGATGAGAAGCCTGGGAGGGCTTGCGCCTGCTTAGACATGGCTCATGATCGTCGTAT GCTGAGCACTTTTATGT / Celera SNP ID: hCV26034157 / Public SNP ID: rs2994329 /
SNP Chromosome Position: 243647921 / SNP in Genomic Sequence: SEQ ID NO: 365 / SNP Position Genomic: 6386 / Related Interrogated SNP: hCV233148 / Related Interrogated SNP: hCV30690780 / SNP Source: dbSNP; HapMap / Population(Allele,Count): Caucasian (G,180|A,46) / SNP Type: UTR5;UTR3;INTRON /
Context (SEQ ID NO: 1222): /
GTGTCTTTAAACTGACAAAATATTCACATTGAAAATAATCTTATAGATCTAGTCCAATCCCCTCATTTTAAGATGAAGAAAAC ACTGAGCCCCTAAATAT
Y
CATACTAAAGTCATACTAAAGCATCACAATATCAACCACCACCCAATTAATTTTAATCCAATCCTAACTGCAGCTTCTATAAC AGGTCCTTTACTCTTAA / Celera SNP ID: hCV26034158 / Public SNP ID: rs4515770 /
SNP Chromosome Position: 243686913 / SNP in Genomic Sequence: SEQ ID NO: 365 / SNP Position Genomic: 45378 / Related Interrogated SNP: hCV233148 / Related Interrogated SNP: hCV30690780 / Related Interrogated SNP: hCV31523650 / Related Interrogated SNP: hCV30690777 / Related Interrogated SNP: hCV31523608 / SNP Source: dbSNP; Celera; HapMap; ABI_Val; HGBASE / Population(Allele,Count): Caucasian (T,180|C,46) / SNP Type: INTRON /
Context (SEQ ID NO: 1223): /
GATGAGGTGGGGTGTTGGAGCATCACCATATATATACATCACACACACAGGGAAACACTCCTACTTGGATGGAGGATCTTTGC TGTTCTGTTGAGGCCAG
Y
CTGATCCTGGAGGCAGGGCAGGGCCTGGCTGTTGCCCAGGCTCCAAAGAGGCGCCAGGGCGTCCCTTAGTCCTTACACTTTCT CCAGAACCTGCGGGCAG / Celera SNP ID: hCV26034160 / Public SNP ID: rs2994327 /
SNP Chromosome Position: 243650858 / SNP in Genomic Sequence: SEQ ID NO: 365 / SNP Position Genomic: 9323 / Related Interrogated SNP: hCV233148 / Related Interrogated SNP: hCV30690780 / SNP Source: dbSNP; HGBASE / Population(Allele,Count): Caucasian (C,167|T,59) / SNP Type: INTRON /
Context (SEQ ID NO: 1224): /
GTGGTACCCCCTCCCCACTGCACGATCCTCAGGAAGAATTCCTTTATTGAAACCATTATTGAATAGGGTAGATTTAGGCACGG GAAATGTAAACAGCAAA
Y
CAGCCACTAAAAGTGCGTTAATGTCACGCCTGTAATCCCAGCACTTTGGGAAGCTGAGGTGGGCGGATCATGAGATCAAGAGA TCGAGACCATCCTGGCC / Celera SNP ID: hCV26338513 / Public SNP ID: rs3006917 /
SNP Chromosome Position: 243641971 / SNP in Genomic Sequence: SEQ ID NO: 365 / SNP Position Genomic: 436 / Related Interrogated SNP: hCV233148 / SNP Source: dbSNP; HapMap; HGBASE / Population(Allele,Count): Caucasian (C,182|T,44) / SNP Type: TRANSCRIPTION FACTOR BINDING SITE;UTR3;INTRON /
Context (SEQ ID NO: 1225): /
ATTTATTTGTTAGCATATATATATGTGTTTGCAATATCCACTCAAAACCAAATTTTACCAATTTATAAGCACATTTAAATGAA AAAGAATCAGACTGGCA
Y
TGGCTGTCTCATTAGCAACCATGGATGCTGAAAATAATTTAAGCAGTTCTCAGATGTTGGAGAAAATGATTTTGAACCTAGAA TTCCTCAGCGAAATTAT / Celera SNP ID: hCV26719082 / Public SNP ID: rs10927046 /
SNP Chromosome Position: 243801190 / SNP in Genomic Sequence: SEQ ID NO: 365 / SNP Position Genomic: 159655 / Related Interrogated SNP: hCV30690780 / Related Interrogated SNP: hCV31523650 / Related Interrogated SNP: hCV233148 / Related Interrogated SNP: hCV31523608 / SNP Source: dbSNP; Celera; HapMap / Population(Allele,Count): Caucasian (T,193|C,31) / SNP Type: INTRON /
Context (SEQ ID NO: 1226): /
AAAAAATCAGGGAATACTACATAACAAGCTATTTGTGCTAGGTAGTCTGCAAAGATGGCTGCCACAAATTCCCATTTCTCCCT GTATGCACATTCCACTC
W
TCCCATGAAGGGGTGGAGTCAACTTTCTCTCTCCCCGGGCTAACCCTGTATCTTGCTTTGAACACAGAATATGATCAAACTGA TACTATGCCAGTAACAG / Celera SNP ID: hCV26719085 / Public SNP ID: rs10927047 /
SNP Chromosome Position: 243803677 / SNP in Genomic Sequence: SEQ ID NO: 365 /

SNP Position Genomic: 162142 / Related Interrogated SNP: hCV30690780 / Related Interrogated SNP: hCV31523650 / Related Interrogated SNP: hCV233148 / Related Interrogated SNP: hCV31523608 / SNP Source: dbSNP; Celera; HapMap / Population(Allele,Count): Caucasian (T,193|A,31) / SNP Type: INTRON / Context (SEQ ID NO: 1227): /
AAGCTCTAGAAAGCCAGCTCTAATGCTGTCAGTTAGTCCATATTATCCTGCTGGAGAGAGAGGCCATGTGGAGAGACCCTGGA
AGATAAATTACCACTCC
R
AGAGAGAGACCAAGTGGAGGAGAACCAAGACACCATAGCCAACAGTCAGCACCAAGGCCCCAGACACATAAGGAAGGTTTTTGT
TGGATTTTTCCAGCCCAC / Celera SNP ID: hCV26719086 / Public SNP ID: rs4658585 /
SNP Chromosome Position: 243803922 / SNP in Genomic Sequence: SEQ ID NO: 365 /
SNP Position Genomic: 162387 / Related Interrogated SNP: hCV31523608 /
Related Interrogated SNP: hCV30690780 / Related Interrogated SNP: hCV31523650
/ SNP Source: dbSNP; Celera; HapMap; HGBASE / Population(Allele,Count):
Caucasian (G,61|A,163) / SNP Type: INTRON /
Context (SEQ ID NO: 1228): /
GAGAGGCCATGTGGAGAGACCCTGGAAGATAAATTACCACTCCGAGAGAGAGACCAAGTGGAGGAGAACCAAGACACCATAGC
CAACAGTCAGCACCAAG
K
CCCCAGACACATAAGGAAGGTTTTTGTTGGATTTTTCCAGCCCACCCATGTATAAGCTGTAGGCAGCTGATTGAGAGCCATGCAT
GCAAGACCAACAGAGTG / Celera SNP ID: hCV26719087 / Public SNP ID: rs4658401 /
SNP Chromosome Position: 243803979 / SNP in Genomic Sequence: SEQ ID NO: 365 /
SNP Position Genomic: 162444 / Related Interrogated SNP: hCV31523608 /
Related Interrogated SNP: hCV30690780 / SNP Source: dbSNP; Celera; HapMap;
HGBASE / Population(Allele,Count): Caucasian (G,32|T,68) / SNP Type: INTRON
/
Context (SEQ ID NO: 1229): /
ACAGGACAAACACTGTATGATTCCACTTATGTGATGTGTCTAAAGTAGTCGAATTCACAGAAGCAGAAAGTAGAACGGTGGTT
TCCAGGGGCTGGGGGTA
Y
GGGAATGGAGAGCTACTGTTCAATGGGAACAGAGGTTCAGTCATGCCAGATGAAAACGTTCTAGAGATCTGCTGTACAATAAT
ATGCACATAGTTAACAA / Celera SNP ID: hCV26719102 / Public SNP ID: rs10927056 /
SNP Chromosome Position: 243822570 / SNP in Genomic Sequence: SEQ ID NO: 365 /
SNP Position Genomic: 181035 / Related Interrogated SNP: hCV31523608 / SNP
Source: dbSNP; Celera; HapMap / Population(Allele,Count): Caucasian
(T,34|C,86) / SNP Type: INTRON /
Context (SEQ ID NO: 1230): /
CAGTGAAAAACTTACCAACAAAGACAAAACTCCAGGTCTAGGGAGAGCTTCACTAGTAAATCCCACCAAATATTTCATTAATA
AATAATACAAATCCCAT
R
CAAACAATTCAATAAAATTAAAGAGAAAGGAGGAGTACTTCCCCTTTTGTTTTTAAAAAGCAAACATGATCCTGAAGTGAAAA
TTATACAAAGACATTTT / Celera SNP ID: hCV26719107 / Public SNP ID: rs7538011 /
SNP Chromosome Position: 243703344 / SNP in Genomic Sequence: SEQ ID NO: 365 /
SNP Position Genomic: 61809 / Related Interrogated SNP: hCV30690780 /
Related Interrogated SNP: hCV233148 / Related Interrogated SNP: hCV31523650 /
Related Interrogated SNP: hCV31523608 / SNP Source: dbSNP; Celera; HapMap /
Population(Allele,Count): Caucasian (A,193|G,33) / SNP Type: TFBS
SYNONYMOUS;INTRON /
Context (SEQ ID NO: 1231): /
TCTTGTATAGCCATAGTATGAATATCAAAACAAAGAAATTAACATCAGTGCAATACTATTAACTAAACTACCAACTTTATTTC
AATTTCACCAGTTTTTC
Y
ACCAATGCCCCTGCCTAGTCCAGGACCCAACCCAGGATCCCACATCGCAGTTAAGTCATCATGCCACTTTAGTCTCTCCAATC
TATGAGTGTTCCATCAT / Celera SNP ID: hCV26719108 / Public SNP ID: rs10927035 /
SNP Chromosome Position: 243703982 / SNP in Genomic Sequence: SEQ ID NO: 365 /
SNP Position Genomic: 62447 / Related Interrogated SNP: hCV31523608 /
Related Interrogated SNP: hCV30690780 / Related Interrogated SNP: hCV233148 /
SNP Source: dbSNP; Celera; HapMap / Population(Allele,Count): Caucasian
(C,70|T,156) / SNP Type: INTRON /
Context (SEQ ID NO: 1232): /
TTGTAAATTAGTCACCTGATTCTCACAGAAAACTTGTTAATTTCTGCTGTACGCCAAAGCCACTAATGGATGAAACTTACGAT
TAGTTCCAACATGAATA
Y

TGGTTGACATTTTTGTTTATGTTAAAGTCAAAGCTGAAAGTGATACAATGGAGACTTCAGAACTTCACTTGTCAGTTATGTGA
GTGATTTCTTTGCTAAA / Celera SNP ID: hCV26719113 / Public SNP ID: rs7517340 /
SNP Chromosome Position: 243710190 / SNP in Genomic Sequence: SEQ ID NO: 365 /
SNP Position Genomic: 68655 / Related Interrogated SNP: hCV30690780 /
Related Interrogated SNP: hCV233148 / Related Interrogated SNP: hCV31523650 /
Related Interrogated SNP: hCV31523608 / SNP Source: dbSNP; Celera /
Population(Allele,Count): Caucasian (T,16|C,100) / SNP Type:
INTRON;PSEUDOGENE /
Context (SEQ ID NO: 1233): /
TCCTTATCCACTACCCTTGCCACTACTGCCTTGGCTCTAGCTACCTCCTAGATATAGTTATGGTTCCCAATCCATGTAGTCAG
TTACTGTGTTTCTTTTA
M

GCATTCATTCTATTATTTCCAGCTGCTCACTTGACATTTCCATCTTGGTTTTATATAGAAACTTCAGACTCAGTGTACCCAAA
GTTATTATCAACATCTA / Celera SNP ID: hCV26719114 / Public SNP ID: rs7549780 /
SNP Chromosome Position: 243712455 / SNP in Genomic Sequence: SEQ ID NO: 365 /
SNP Position Genomic: 70920 / Related Interrogated SNP: hCV31523608 /
Related Interrogated SNP: hCV30690780 / Related Interrogated SNP: hCV31523650
/ SNP Source: dbSNP; Celera; HapMap / Population(Allele,Count): Caucasian
(A,61|C,165) / SNP Type: INTRON /
Context (SEQ ID NO: 1234): /
AATGACTTTGAGCAAATCTTTGTATCAAAATAGTTGTCAAAATATTCTCATCTATTTCAGCTATTTTAAAAATGTTACATTAT
ATGCAAGATATTAAAGA
Y

AGTAGGTTTCAAAGAAAAAATCAGGAAAATTTTGGCAAGCAAAACATTTTCTAAGAATGGGGTTCTAAAAAAACTAAAAAGAAT
GCATCAAATACTGCACT / Celera SNP ID: hCV26719116 / Public SNP ID: rs10927039 /
SNP Chromosome Position: 243721726 / SNP in Genomic Sequence: SEQ ID NO: 365 /
SNP Position Genomic: 80191 / Related Interrogated SNP: hCV30690780 /
Related Interrogated SNP: hCV233148 / Related Interrogated SNP: hCV31523650 /
Related Interrogated SNP: hCV31523608 / SNP Source: dbSNP; Celera /
Population(Allele,Count): Caucasian (T,98|C,18) / SNP Type: INTRON /
Context (SEQ ID NO: 1235): /
ATTACAGATCTGATACACAACTACTACTTCTAGTACTGTCACGAAAAGCCCAGCATTAATAATGGTAATGTACTAGAAATTTC
AAGGTCCAAAAAAAGTG
M

GAGACACCATCTGAGGTAAAAGGAAGAGGCTGGCTTCAGCTCCTGCCAATCGCTCCCTAACACTGACCATGTAAATCTGATCA
CAGTATCGCCTTGCTCT / Celera SNP ID: hCV26719117 / Public SNP ID: rs12144559 /
SNP Chromosome Position: 243723214 / SNP in Genomic Sequence: SEQ ID NO: 365 /
SNP Position Genomic: 81679 / Related Interrogated SNP: hCV31523608 /
Related Interrogated SNP: hCV30690780 / Related Interrogated SNP: hCV31523650
/ SNP Source: dbSNP; Celera; HapMap / Population(Allele,Count): Caucasian
(C,61|A,165) / SNP Type: INTRON /
Context (SEQ ID NO: 1236): /
ATGCTTAATGGATGCATCCACTAACAAAAGTAGTGATGAAGTCAAATATGAAGTCATAAGAAACAAGATTGTATATTAAAAAT
CTAAGTAATGCTTACTA
Y

TTCTATCAGAGGGTTGTTATCTAAAAGGTCATGCTTTTGAGGAGAGAGAAAAAAAAAAAACACTAGACATAATACATCATAGAA
TTTCCACCAAAAAAAAG / Celera SNP ID: hCV26719120 / Public SNP ID: rs10927040 /
SNP Chromosome Position: 243731693 / SNP in Genomic Sequence: SEQ ID NO: 365 /
SNP Position Genomic: 90158 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap /
Population(Allele,Count): Caucasian (T,188|C,38) / SNP Type: INTRON /
Context (SEQ ID NO: 1237): /
AAAGGGTAACATTTTTTGCCCAGTTGAAGTGGACAGGCATTACTTGACAGAGGGTCTTGATTCAGTGTGTATATGAAGAAATG
GTAGATTTTTAAATCTT
Y

TTTCCTGTGCTTAGTGGGCACAAAATTGTAACAAAAATTACTATGTTTCCTCCTGTTCTTGCTGATGGCTAGCATAAAATTAA
ACTTTAAAAATCCTAGC / Celera SNP ID: hCV26719121 / Public SNP ID: rs10927041 /
SNP Chromosome Position: 243731890 / SNP in Genomic Sequence: SEQ ID NO: 365 /
SNP Position Genomic: 90355 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap /
Population(Allele,Count): Caucasian (T,188|C,38) / SNP Type: INTRON /

Context (SEQ ID NO: 1238): /
AAAAATAACTAAAATCAGAGCAGAATGGAACGAAATTAAGACCTAAAAATCCACATAATGAAATGATGAAGCCAAAAGTTGGT
TCTTTGAAAGGATAAAG
R
AGATTGCTAGACTGCTAGTGAGATTAACAAAGGAAAAGAGAGAAGATATAAATAACCACAATTAGAAATGACAAAGATTACAT
TACAACTTATCCCACAG / Celera SNP ID: hCV26719137 / Public SNP ID: rs12136847 /
SNP Chromosome Position: 243747358 / SNP in Genomic Sequence: SEQ ID NO: 365 /
SNP Position Genomic: 105823 / Related Interrogated SNP: hCV31523608 /
Related Interrogated SNP: hCV30690780 / Related Interrogated SNP: hCV31523650
/ SNP Source: dbSNP; Celera / Population(Allele,Count): Caucasian
(A,61|G,165) / SNP Type: INTRON /
Context (SEQ ID NO: 1239): /
AGTTGCCATCATAAGTAGGACTCATTAAAGCATGGCAAAAACATTGTTTCAAAAAAGGAAGAATGACAGGGCAGACAATGACA
AACTCTGATGCCTAAAT
R
GGTATACAGTCACCAATGGCATGAGACGGCAGCTGTACTGTGCTGTTACTACATATAGAAGTTGTCAATGGAATTTAGAAATG
ACAGACCAAAATCTCAA / Celera SNP ID: hCV26719149 / Public SNP ID: rs6675851 /
SNP Chromosome Position: 243787494 / SNP in Genomic Sequence: SEQ ID NO: 365 /
SNP Position Genomic: 145959 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap /
Population(Allele,Count): Caucasian (G,189|A,37) / SNP Type: INTRON /
Context (SEQ ID NO: 1240): /
GTATGTTGTCTTGGCATATATGAAGAAAATCCAGCCTCATGCAGATATGAATAGAAAAAGGGAGAAAAATGTTAACAAACTTT
TTGGATTACTGTTGATA
M
TATTCAAATCGTTTGCTGTTACACACAATAGTTGTAGGAATCACCTTGTATATATCACATAACAAACATTCCTAAGTTTACCT
ATAGGATCCACTTCCAG / Celera SNP ID: hCV26719161 / Public SNP ID: rs6682456 /
SNP Chromosome Position: 243942463 / SNP in Genomic Sequence: SEQ ID NO: 365 /
SNP Position Genomic: 300928 / Related Interrogated SNP: hCV31523608 / SNP
Source: dbSNP; Celera / Population(Allele,Count): Caucasian (C,24|A,202) /
SNP Type: INTRON /
Context (SEQ ID NO: 1241): /
AAATAAAAGAATTAAAAATAAAGGAAAGTTAATACTATTTTCCACTAAGAAGAACCAGAGCTCCCTAGAGAGCTGCTTCTAGA
GGTAGGAAAGTATATGA
M
GAGACTGGAATATCTTGTTGTCCCAGCAAGTCCTCAAAAATTGATGGAGACATGTCAAAAGAATTTAAAAGCCGGCCTGAAAG
TGCTTTCATGGGCCCAA / Celera SNP ID: hCV26719162 / Public SNP ID: rs4132509 /
SNP Chromosome Position: 243943084 / SNP in Genomic Sequence: SEQ ID NO: 365 /
SNP Position Genomic: 301549 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap;
HGBASE / Population(Allele,Count): Caucasian (C,187|A,39) / SNP Type:
TRANSCRIPTION FACTOR BINDING SITE;INTRON /
Context (SEQ ID NO: 1242): /
ATATTTTATATTCCTTAAAATAAGCAAAATTCCAACAAGATTTTTGATGAAACCTGGAAAACTATTCTAAAATTCACATGGA
TTGATGGATTTATTTAA
Y
CATTCCTTTTTGAGACAGGGTCTTGCTCAGTCACCCAGGCTGCTGCACAGTGGCACAATCACAGCTCACTGCAGCCTCGACCT
TCTGAGCCCAAGCAATC / Celera SNP ID: hCV26719163 / Public SNP ID: rs6429435 /
SNP Chromosome Position: 243945131 / SNP in Genomic Sequence: SEQ ID NO: 365 /
SNP Position Genomic: 303596 / Related Interrogated SNP: hCV31523608 /
Related Interrogated SNP: hCV30690780 / Related Interrogated SNP: hCV31523650
/ SNP Source: dbSNP; Celera / Population(Allele,Count): Caucasian
(T,63|C,163) / SNP Type: INTRON /
Context (SEQ ID NO: 1243): /
GATCACTTATCTCTGTAGGAAGAAAAGCAATGGCCATTCCTAGCATACTTTCACTCAAAAACAGGTTTCGTAGCTAGAACTAT
TTTCCAATAATAAAGCC
W
AACATCAGCTATCAATCTAATAATCTCCAATCATCTCAAGTCCTCCAAACACATTAAAAATGAATTTATAACAGTAAATGTATC
ACAAGCCTCAACACCCC / Celera SNP ID: hCV26719171 / Public SNP ID: rs10927075 /
SNP Chromosome Position: 243956120 / SNP in Genomic Sequence: SEQ ID NO: 365 /
SNP Position Genomic: 314585 / Related Interrogated SNP: hCV31523608 /

Related Interrogated SNP: hCV30690780 / Related Interrogated SNP: hCV31523650 / SNP Source: dbSNP; Celera; HapMap / Population(Allele,Count): Caucasian (A,63|T,161) / SNP Type: INTRON /
Context (SEQ ID NO: 1244): /
TCACGCACCAGGTTCTCAGCTTCCAATATTTAGTTGACGTTGTTTGCCTGCTGTCATCTCTCATCTCGTTCTCTTTACCCTCA TGGATTTCTGCTTTTAT
R
TGTGTTCACTATCATTTAAGTGGGGAAGGAAATACATGTCTTCTCTGATAATACTGGAGATTTTTAAAGTTTTCTTTTGATCC CTTATGGTATCTGTTTT / Celera SNP ID: hCV26719176 / Public SNP ID: rs10927076 /
SNP Chromosome Position: 243958293 / SNP in Genomic Sequence: SEQ ID NO: 365 /
SNP Position Genomic: 316758 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap /
Population(Allele,Count): Caucasian (A,187|G,39) / SNP Type: UTR3;INTRON /
Context (SEQ ID NO: 1245): /
TCCATCTGCTTATTTGAAAGAGGTATAGCAGCTTCTGTCTCCTGTTGTAACCTCTCCCAGTCTCTGATTTCTTTCTTCCATTT AGGCAGGGATTAGGAAA
Y
AAGAAGAGATGTACAGGTGTAGCCAATCTGTCACTTTTGGTCAGAAGCCCTGAGAGAGCTCTTTTGAAATCAGCTATCTCAGG ATACTGACTTAAGGACT / Celera SNP ID: hCV26719179 / Public SNP ID: rs6672195 /
SNP Chromosome Position: 243959543 / SNP in Genomic Sequence: SEQ ID NO: 365 /
SNP Position Genomic: 318008 / Related Interrogated SNP: hCV31523608 /
Related Interrogated SNP: hCV30690780 / Related Interrogated SNP: hCV31523650 / SNP Source: dbSNP; Celera; HapMap; ABI_Val / Population(Allele,Count): Caucasian (T,63|C,163) / SNP Type: TRANSCRIPTION FACTOR BINDING SITE;UTR5;INTRON /
Context (SEQ ID NO: 1246): /
CCCAATAACTATGTCCAGGTGCAAGGAGCACGTTTTATTCTTTTTGTTTCTTTAGTTGCTATAACATGATAGGCATGTACTTG GTAAGTATTTATTAGGG
R
AAAAAAAACACTTGAAAGGTCCCAGTTCAGAATGTCAGAAGCATAATCAAGACACTTCATATACCTGCAGTGCTTTGCAAGCA TCAAAGTTGAAAGTGCT / Celera SNP ID: hCV26719184 / Public SNP ID: rs68140928 /
SNP Chromosome Position: 243969663 / SNP in Genomic Sequence: SEQ ID NO: 365 /
SNP Position Genomic: 328128 / Related Interrogated SNP: hCV31523608 / SNP
Source: dbSNP; Celera; HapMap / Population(Allele,Count): Caucasian (G,19|A,101) / SNP Type: INTRONIC INDEL;INTRON /
Context (SEQ ID NO: 1247): /
ACCTATTAACCACATCAAATGACTTCTTACTATAAAGATTGAGAAATTAAGATACCCTCTGCCCTTTCTTCCCAATTTTTGTT ACTTTCATTTTCATTTT
Y
AGCTTTTCTACTGGTTTGTATTCCACATTAAAAAAAATACACTTAAGCCTCTATTTCTTGACCCAGGAATTTTAGATGGAAATG TCTTAGCTCCTAACTGT / Celera SNP ID: hCV26719192 / Public SNP ID: rs10803161 /
SNP Chromosome Position: 243990770 / SNP in Genomic Sequence: SEQ ID NO: 365 /
SNP Position Genomic: 349235 / Related Interrogated SNP: hCV30690780 /
Related Interrogated SNP: hCV233148 / Related Interrogated SNP: hCV31523650 /
Related Interrogated SNP: hCV31523608 / SNP Source: dbSNP; Celera; HapMap /
Population(Allele,Count): Caucasian (T,99|C,17) / SNP Type: INTRON /
Context (SEQ ID NO: 1248): /
GAACACTTACAGACTTTTTTCTTGTCATTATTCCCTAAACAGGACAGTGTAACAACCACTTACATAGAATCTTTACATGGCAT TTGCAGTGGATTAGGTA
W
TGTAAGTAATTTAAAGATGATTTAAAGCATACAGGAGGGTGTGTGCAGGTTGTATGCAAATACTACACCATTTTTATGTAAGGG ATTTGAGCATCAGCAGA / Celera SNP ID: hCV26719193 / Public SNP ID: rs6429439 /
SNP Chromosome Position: 243993530 / SNP in Genomic Sequence: SEQ ID NO: 365 /
SNP Position Genomic: 351995 / Related Interrogated SNP: hCV31523608 / SNP
Source: dbSNP; Celera; HapMap / Population(Allele,Count): Caucasian (A,18|T,102) / SNP Type: INTRON /
Context (SEQ ID NO: 1249): /
CTTACCTAATTTAGATACCTTATTTACTAGTCCATTCTGGGACAATCAGGGTACAACCCACTATCTTTGTCAGGAATAACCAT TCAGTCAAATCTACCAA
Y
TCCTACCCTGTCAGTTCTGTTTTGCTACACAAAAATTTGACAGATATACAACATGGTTCTAGAATTCCAGTGCTGCCCTTACC GAAACTCTCTCCTGAAC / Celera SNP ID: hCV26719194 / Public SNP ID: rs10927081 /

SNP Chromosome Position: 243994216 / SNP in Genomic Sequence: SEQ ID NO: 365 / SNP Position Genomic: 352681 / Related Interrogated SNP: hCV31523608 / Related Interrogated SNP: hCV30690780 / Related Interrogated SNP: hCV31523650 / SNP Source: dbSNP; Celera; HapMap / Population(Allele,Count): Caucasian (T,63|C,163) / SNP Type: INTRON / Context (SEQ ID NO: 1250): / CAGAGATGCCTCCTATGCTGATGTGCAACCAAGTTGTAAGGAGCTGTCCTGGATCCATCAAGGAGGAAAACGCAGACAATACT TTACTTGATGTTTGCTC Y GTGATGTTTCTTTTATTGCCATTTCTAACACCAACAATACTATGGTAATGTGTTATCTCAATTAATACTTATCATTTTCAACT GTCCAGAAAAGTGTAAA / Celera SNP ID: hCV26719197 / Public SNP ID: rs4590656 / SNP Chromosome Position: 244002773 / SNP in Genomic Sequence: SEQ ID NO: 365 / SNP Position Genomic: 361238 / Related Interrogated SNP: hCV31523608 / Related Interrogated SNP: hCV30690780 / Related Interrogated SNP: hCV31523650 / SNP Source: dbSNP; Celera; HapMap; ABI_Val; HGBASE / Population(Allele,Count): Caucasian (C,63|T,163) / SNP Type: INTRON / Context (SEQ ID NO: 1251): / TGTTTGTCCTCCAAAGATGTTAGTAACATTAAATATATCTTACTAACACACACATATATGTGTATGTATGTACATATATATAT GTATGTAAAATACGAGG Y AGAAGGTACTCAACAAGTGGTAATTTATTACTATACTACTCCACAATATACTCAATATTTCAAGAAATAATTATCTGCTGCTG ATAGGCAAACCACTGAA / Celera SNP ID: hCV26719201 / Public SNP ID: rs4478795 / SNP Chromosome Position: 243844576 / SNP in Genomic Sequence: SEQ ID NO: 365 / SNP Position Genomic: 203041 / Related Interrogated SNP: hCV30690780 / Related Interrogated SNP: hCV31523650 / Related Interrogated SNP: hCV233148 / SNP Source: dbSNP; Celera; HapMap; HGBASE / Population(Allele,Count): Caucasian (C,100|T,10) / SNP Type: INTRON / Context (SEQ ID NO: 1252): / TCTTGATGATTCTGTCTTTAATGATACCTCTTTTTCCTAAATTGTAACTGCATGTATTAATTGCAAAACTCCCTTCAAAACTT AGCCATAAAACATCTCA R AATACCAATCCCAAGGGTCTGTGGCATATTTTTGCTACTAGACTATAAATTCTTTAAAGCCAAGGGCTAGATCTTGTATCTGT GGAATAAGAAAATGAAC / Celera SNP ID: hCV26719202 / Public SNP ID: rs4658588 / SNP Chromosome Position: 243846271 / SNP in Genomic Sequence: SEQ ID NO: 365 / SNP Position Genomic: 204736 / Related Interrogated SNP: hCV31523608 / Related Interrogated SNP: hCV30690780 / Related Interrogated SNP: hCV31523650 / SNP Source: dbSNP; Celera; HGBASE / Population(Allele,Count): Caucasian (A,61|G,163) / SNP Type: INTRON / Context (SEQ ID NO: 1253): / TGTCAAAATATAATTAAAAGCTAAGTATAATATAATCTTCATGTGTTTTATCAATACTACATAGAGTTCTATGATTTTTATCA TAAATAGGATCTCAGCC K GACTTGGCCTGTCTGCTCTGGTCTAAAAGATGTGAGGAGAAAAAAAGAGACTTGTTAATATCATAATATTATCTTAAACCAGT AATTCGAATAAAGAGAT / Celera SNP ID: hCV26719215 / Public SNP ID: rs12144546 / SNP Chromosome Position: 243883877 / SNP in Genomic Sequence: SEQ ID NO: 365 / SNP Position Genomic: 242342 / Related Interrogated SNP: hCV31523608 / Related Interrogated SNP: hCV30690780 / Related Interrogated SNP: hCV31523650 / SNP Source: dbSNP; Celera; HapMap / Population(Allele,Count): Caucasian (T,61|G,165) / SNP Type: INTRON / Context (SEQ ID NO: 1254): / ATCAACGATTCAATAGAAAGCCCCAACAGTATGTAAGATTTTTTGAATCCCTTGCTGTTGCATAATGGTCATCATCCAATTTT AATCAAACACACACACA Y ACCCAGTATTCACTCAACCTCAACCAAACTTTGATTCTCAGTACATACTATCCTTGCTTTCTTCCCTCCAAGAAACCGCCAAA GTTCTAACCAGGTGGTG / Celera SNP ID: hCV26719217 / Public SNP ID: rs7548254 / SNP Chromosome Position: 243891570 / SNP in Genomic Sequence: SEQ ID NO: 365 / SNP Position Genomic: 250035 / Related Interrogated SNP: hCV31523608 / Related Interrogated SNP: hCV30690780 / Related Interrogated SNP: hCV31523650 / SNP Source: dbSNP; Celera / Population(Allele,Count): Caucasian (C,63|T,163) / SNP Type: INTRON / Context (SEQ ID NO: 1255): / CAAAAGCCCTATTAAATTTCCAGCCAGTAATTCACTAGCTATATTACTTAAAAGTTAGGAATTGTGTGCACAGTTCTTCTTAT AGCACCTAGGACAGTGA

Y
AGGACATAGGAGTATACAATAAATACCCTGATTGGGCAACTGATTGGCTGGCTTTAAACGTAAAGAATGGAGGACAGACACTT
AAATGAGGGGCGTGCCC / Celera SNP ID: hCV26719218 / Public SNP ID: rs4658403 /
SNP Chromosome Position: 243832560 / SNP in Genomic Sequence: SEQ ID NO: 365 /
SNP Position Genomic: 191025 / Related Interrogated SNP: hCV31523608 / SNP
Source: dbSNP; Celera; HapMap; HGBASE / Population(Allele,Count): Caucasian
(C,19|T,99) / SNP Type: INTRON /
Context (SEQ ID NO: 1256): /
AAAAGATCTGAAATCTATCTTAACTGTCTAATGATCAGGGAGAACTGTTAGGTAAATAATTATAAAATGCTATTAAAAGTCCT
TAATGATATGGGAAAAC

S
TTCATAATATTAAGAGAAAAAAAGCAAGATATAAAATTCCAAATGTTGTATTTTAAAAATAAAAATATGTGCATAGACAAAAG
ATTAAGGAGACATAAAA / Celera SNP ID: hCV26719219 / Public SNP ID: rs9782958 /
SNP Chromosome Position: 243892972 / SNP in Genomic Sequence: SEQ ID NO: 365 /
SNP Position Genomic: 251437 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap /
Population(Allele,Count): Caucasian (G,182|C,38) / SNP Type: INTRON /
Context (SEQ ID NO: 1257): /
TACTCATCTGCAAAATGGGGGTGATAGCGCTTTCCTCATAAGGTTGCTATGAGAATTAAATGAGATTAATTCAGGTAAAACAC
CTAAAATAGTGCTTGGC

R
TGTAGTAACAGCTCAGTAAGGCATGGGTGTAATTGTTATTCCCTGACTCCTAAATCCAGGTTAGGAGCCCCTTCTGTTATTCC
TTCTTCTTCTTCTTACT / Celera SNP ID: hCV26719222 / Public SNP ID: rs4553169 /
SNP Chromosome Position: 243900640 / SNP in Genomic Sequence: SEQ ID NO: 365 /
SNP Position Genomic: 259105 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera; HapMap;
HGBASE / Population(Allele,Count): Caucasian (G,189|A,37) / SNP Type: INTRON
/
Context (SEQ ID NO: 1258): /
CATAACCTCTGCATATTCAATGCTTTCATATTCAATTGTTTGTTTTTAAAGAAATTAGTTTATTAGAGACAGAGAGAGAACGA
ATCTTTCCCTTGATAAT

Y
TCCACTAAACTTTATAAATATGCTTAGAATGGAAATGAATGAGAAGAAACAGAGGATGAAGGAGACATCAGAAATTTTCAGCC
CTGTGTTTTACCTCTGG / Celera SNP ID: hCV26719225 / Public SNP ID: rs11586029 /
SNP Chromosome Position: 243910067 / SNP in Genomic Sequence: SEQ ID NO: 365 /
SNP Position Genomic: 268532 / Related Interrogated SNP: hCV31523608 /
Related Interrogated SNP: hCV30690780 / Related Interrogated SNP: hCV31523650
/ SNP Source: dbSNP; Celera / Population(Allele,Count): Caucasian
(C,63|T,163) / SNP Type: TRANSCRIPTION FACTOR BINDING SITE;INTRON /
Context (SEQ ID NO: 1259): /
AGAACAATATTTGAAGCCTCTATACAACTTTTATAAAACATTTGCCACAAGGCAAGTTTTGGTGTGTGCAAAAATCCATTTGT
AGACCAGCTGTGACAGA

M
TCTTTAAAGCATATCCAAGGCAATGATAAATGAAGCAGACATTCATGCCAAACGCTTGGATTTAGATTTACGTATCAAGAAAG
CTTTAATAAGTAATCCT / Celera SNP ID: hCV26719227 / Public SNP ID: rs10927065 /
SNP Chromosome Position: 243914611 / SNP in Genomic Sequence: SEQ ID NO: 365 /
SNP Position Genomic: 273076 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera /
Population(Allele,Count): Caucasian (C,192|A,32) / SNP Type: INTRON /
Context (SEQ ID NO: 1260): /
AAATATCAGATTGAATAATTCAGATGAATACTCCACTGAGAACAATTTAAAATGTAAGACAAAATTAGGACTCTGATAAGTCA
ATTGCCACATTAAGCTG

R
AATCACAAATGGCTGATCGTTAATGTCAATGGGGAACCAAAAAATAAACTAAATCTTGCACAGATCTGCACCCTAGATTCATG
TCACCTGAGTGGACCAG / Celera SNP ID: hCV26719232 / Public SNP ID: rs10803158 /
SNP Chromosome Position: 243923229 / SNP in Genomic Sequence: SEQ ID NO: 365 /
SNP Position Genomic: 281694 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; Celera /
Population(Allele,Count): Caucasian (A,186|G,38) / SNP Type: INTRON /

Context          (SEQ ID NO: 1261): /
TAATTCAGATGAATACTCCACTGAGAACAATTTAAAATGTAAGACAAAATTAGGACTCTGATAAGTCAATTGCCACATTAAGC
TGAAATCACAAATGGCT
R
ATCGTTAATGTCAATGGGGAACCAAAAAATAAACTAAATCTTGCACAGATCTGCACCCTAGATTCATGTCACCTGAGTGGACC
AGGAAACTTTAGGCTTT / Celera SNP ID:  hCV26719233 / Public SNP ID:  rs10927067 /
SNP Chromosome Position:  243923244 / SNP in Genomic Sequence:  SEQ ID NO: 365 /
 SNP Position Genomic:  281709 / Related Interrogated SNP:  hCV30690780 /
Related Interrogated SNP:  hCV31523650 / Related Interrogated SNP:  hCV233148 /
Related Interrogated SNP:  hCV31523608 / SNP Source:  dbSNP; Celera; HapMap /
Population(Allele,Count):  Caucasian (G,187|A,39) / SNP Type:  INTRON /
Context          (SEQ ID NO: 1262): /
GTAAATACATAACCTAAACCAATGAGTTTCACTCATTTGTCAGCTTCAACCACTGACAAAACCCAGTGGTTGCCTAGTGATGT
ATAGTTCTACTAAAAGA
Y
ATATATGACCATGCTTTACCAAATACGGAACACATGTCTAAACTGTTGAGACAACTGCCAATGTATTAACTACAAAAAGCCAT
TATTTTTTACTTTAGCC / Celera SNP ID:  hCV27171311 / Public SNP ID:  rs4322213 /
SNP Chromosome Position:  244011942 / SNP in Genomic Sequence:  SEQ ID NO: 365 /
 SNP Position Genomic:  370407 / Related Interrogated SNP:  hCV31523608 / SNP
Source:  dbSNP; Celera; HapMap; ABI_Val; HGBASE / Population(Allele,Count):
Caucasian (C,24|T,202) / SNP Type:  INTRON /
Context          (SEQ ID NO: 1263): /
TTTATTTAAATAACGTTGTTCTTTCTCTTAGACTATGCCGGTATGAATTTGGTAGAATCTGCCGATTGTGGTATGTCGGTACT
CCATACAGAGTGTGTGG
Y
AGATTTCTTCTCTATTTTGTTATCACATCCAGTAATATGGAGTGCAGCCAGCATGTTCATCCCTGGGAATGTCCCTCCTAAAT
TTTGCAAAGAAAGCCTT / Celera SNP ID:  hCV27171350 / Public SNP ID:  rs4430311 /
SNP Chromosome Position:  244015993 / SNP in Genomic Sequence:  SEQ ID NO: 365 /
 SNP Position Genomic:  374458 / Related Interrogated SNP:  hCV31523608 /
Related Interrogated SNP:  hCV30690780 / Related Interrogated SNP:  hCV31523650
/ SNP Source:  dbSNP; Celera; HapMap; HGBASE / Population(Allele,Count):
Caucasian (C,68|T,158) / SNP Type:  INTERGENIC;UNKNOWN /
Context          (SEQ ID NO: 1264): /
GTACTTTCAAAAGCAAACTCTGGCTAGCACAGGAAAAATCCTTTGACAAGAGAAAGGCTAAATGGATACAATAATGATGATTA
GATCATACTTACACATA
Y
CTTCCAAGAAATTTCTATACTTCTTTTTTTATTCTGAAGCAATAGACATTATGGGATTCCAAGAAACTGAGCCTTCATAACCCA
AGTAACAATAGCCATTG / Celera SNP ID:  hCV27498250 / Public SNP ID:  rs3766673 /
SNP Chromosome Position:  243720473 / SNP in Genomic Sequence:  SEQ ID NO: 365 /
 SNP Position Genomic:  78938 / Related Interrogated SNP:  hCV30690780 /
Related Interrogated SNP:  hCV31523650 / Related Interrogated SNP:  hCV233148 /
Related Interrogated SNP:  hCV31523608 / SNP Source:  dbSNP; HapMap; HGBASE /
Population(Allele,Count):  Caucasian (T,187|C,39) / SNP Type:  INTRON /
Context          (SEQ ID NO: 1265): /
TTTTTCTAATTTGCCATAAGAAACAAATTAAGTTCACTTAAAACTCAAGTTTATTTTAAAACCTGCCATGTATCTGACATTGG
ACATTTAATAAACTGGC
Y
TTAAACTGATTTCTAACACTCTTTGTGAAGTATCTTCAAAATACCCTTCACCTGTCTTTATACCAGATTAAAACAACGTAAAA
GGCGAAGGAACACTATT / Celera SNP ID:  hCV27511819 / Public SNP ID:  rs3753549 /
SNP Chromosome Position:  243722892 / SNP in Genomic Sequence:  SEQ ID NO: 365 /
 SNP Position Genomic:  81357 / Related Interrogated SNP:  hCV31523608 / SNP
Source:  dbSNP; HapMap; HGBASE / Population(Allele,Count):  Caucasian
(T,202|C,24) / SNP Type:  INTRON /
Context          (SEQ ID NO: 1266): /
AGAAACTCAAATTTAAAAGTGCTGCTATAATAAAGTTGCTCCTTCAAGTATATATGGGGCGGCAGAGAACACCAGAGCCTCGC
GGGTCACACAGGACACC
R
TGAGTAAGCCTGTAGATGGCACCAATCCACGACCAATCTATTGTTTACAGAAACAGTCATTTTGGGTTGTGCTTCTTTATGCA
AGATATTTAACACCTTT / Celera SNP ID:  hCV29210363 / Public SNP ID:  rs6656918 /
SNP Chromosome Position:  243706224 / SNP in Genomic Sequence:  SEQ ID NO: 365 /
 SNP Position Genomic:  64689 / Related Interrogated SNP:  hCV233148 / Related
Interrogated SNP:  hCV30690780 / Related Interrogated SNP:  hCV31523650 /
Related Interrogated SNP:  hCV30690777 / Related Interrogated SNP:  hCV31523608

/ SNP Source: dbSNP; HapMap / Population(Allele,Count): Caucasian
(G,46|A,180) / SNP Type: INTRON /
Context (SEQ ID NO: 1267): /
CTTTCTTCCCAATTTTTGTTACTTTCATTTTCATTTTTAGCTTTTCTACTGGTTTGTATTCCACATTAAAAAAATACACTTAA
GCCTCTATTTCTTGACC
Y
AGGAATTTTAGATGGAAATGTCTTAGCTCCTAACTGTGTAAGATAAGGAAATTAGTATTCTTACACAGCAAGCATTTTTAATC
ATAAAGTTTAACCAAAG / Celera SNP ID: hCV29210367 / Public SNP ID: rs4518884 /
SNP Chromosome Position: 243990833 / SNP in Genomic Sequence: SEQ ID NO: 365 /
SNP Position Genomic: 349298 / Related Interrogated SNP: hCV31523608 / SNP
Source: dbSNP; HapMap; ABI_Val; HGBASE / Population(Allele,Count): Caucasian
(C,36|T,84) / SNP Type: INTRON /
Context (SEQ ID NO: 1268): /
CTTAGCTCCTAACTGTGTAAGATAAGGAAATTAGTATTCTTACACAGCAAGCATTTTTAATCATAAAGTTTAACCAAAGAATT
AAAATATGACTATTTGG
Y
CTTACCCAATCCTGAGTGAGAGGAAAAAAATGTATGTACGTACTTGTCGCTTTAGTACTATTTCATTTAAGAAAAAGTGTTGA
AAAATATAGGAGCAGTA / Celera SNP ID: hCV29210368 / Public SNP ID: rs4313380 /
SNP Chromosome Position: 243990955 / SNP in Genomic Sequence: SEQ ID NO: 365 /
SNP Position Genomic: 349420 / Related Interrogated SNP: hCV31523608 / SNP
Source: dbSNP; HapMap; HGBASE / Population(Allele,Count): Caucasian
(T,26|C,200) / SNP Type: INTRON /
Context (SEQ ID NO: 1269): /
TGGCATACTGCTCGCCTCCAAACTTGGTAGGGATATTAGGATTGACTATGAAACTGTAAATACATGGACATAATCCTACACAC
AAATATGGATATATTCA
Y
GGCCACCTATATAAAAGAAATAAATACATGAGACAAAGGAAAAGTCAAGGTGGCTGATGATTTTCTATGCCTTTTCAGCAAAC
ACGGATTAGAGTCACTT / Celera SNP ID: hCV29210370 / Public SNP ID: rs6676779 /
SNP Chromosome Position: 243875486 / SNP in Genomic Sequence: SEQ ID NO: 365 /
SNP Position Genomic: 233951 / Related Interrogated SNP: hCV31523608 / SNP
Source: dbSNP; HapMap / Population(Allele,Count): Caucasian (C,24|T,202) /
SNP Type: INTRON /
Context (SEQ ID NO: 1270): /
CAAAGGAGGTAGAAAAGATAGGGAGATTTTTATTTTTCATTTTATACCTTTGTGTGTTATTCAAATTTTTATGTTTATGCAAC
TATTCATCTCAAAATAA
R
CACATACACATTATTAGAAACACATACACATACACATCTCAAAATAAACACATACACATTATTAGAAAAATGATGGCAAATTC
TTATTCATTTTTTTTTTC / Celera SNP ID: hCV30228080 / Public SNP ID: rs4593807 /
SNP Chromosome Position: 244004392 / SNP in Genomic Sequence: SEQ ID NO: 365 /
SNP Position Genomic: 362857 / Related Interrogated SNP: hCV31523608 / SNP
Source: dbSNP; HGBASE / Population(Allele,Count): Caucasian (G,18|A,98) / SNP
Type: INTRON /
Context (SEQ ID NO: 1271): /
AAACTGAAAAGAAATTTACAGATCAATACTCTTGTAAATGCAAAAAACCCTTAGCAAAATATCAGTAAGTCCAAATTGGTAAT
ATATTTTTTTAAATACA
Y
CATGACCAAATGGAATTTATTCCAGCAATGAAAGTAATTCACCATATTAAGAGAATAAAAGGAAAAAATCAAATGACCATTTC
AATAGAAGCAGTAAAAT / Celera SNP ID: hCV29958244 / Public SNP ID: rs4614244 /
SNP Chromosome Position: 244000874 / SNP in Genomic Sequence: SEQ ID NO: 365 /
SNP Position Genomic: 359339 / Related Interrogated SNP: hCV31523608 / SNP
Source: dbSNP; HapMap; HGBASE / Population(Allele,Count): Caucasian
(C,20|T,206) / SNP Type: INTRON /
Context (SEQ ID NO: 1272): /
TGTATATATCTCAATCTACAATCCTTAGAAATCCATCAAACCATAATTGTGATTAGAGATGTACTATGCTTGAAAAACCTTAT
TTTTACTTACATTTCTA
Y
AGCACTACATTGGCACCTATATATTAATAGTTCTTATGTGATCCACCCAAAGAAGGGTGAACGTCAAATATTGCTATCCACAT
TCTACAGGCCTTGTGGA / Celera SNP ID: hCV30462455 / Public SNP ID: rs6686591 /
SNP Chromosome Position: 243853830 / SNP in Genomic Sequence: SEQ ID NO: 365 /
SNP Position Genomic: 212295 / Related Interrogated SNP: hCV31523608 / SNP
Source: dbSNP / Population(Allele,Count): Caucasian (C,25|T,199) / SNP Type:
INTRON /
Context (SEQ ID NO: 1273): /

AAGAGCTTACTTGTGTAACCAAATACCACCTGTTCCCCAAACGCCTATGGAAATAAAAAAAAAAGAAAAAAAGTACATAATAA
AAAAAAAATTAGAATCA
R

TGTCATAGAAAGCAATATAACGTTAGATTTTTATTTTCTAGAATAAAACAGTCTCAGGTGACATAACCAAATATAGTGTAAGG
ACCCTGGATTTTAAAAA / Celera SNP ID:   hCV30048215 / Public SNP ID:    rs6703013 /
SNP Chromosome Position:   243976837 / SNP in Genomic Sequence:    SEQ ID NO: 365 /
 SNP Position Genomic:   335302 / Related Interrogated SNP:   hCV31523608 / SNP
Source:   dbSNP; HapMap / Population(Allele,Count):   Caucasian (G,24|A,202) /
SNP Type:    INTRON /
Context           (SEQ ID NO: 1274): /
GACATACTTTCTGCCATGACTGATTTGCTTGTATTTTAAAGAATTTTATGCAAATGGATCATAGAGTATGTACTCTTTATCTG
GCTTGTTCATTTAGCAT
K

ATTACTTTGATATTCATCCATGTCACCACATGTATCAATAGTTTACTCACTTTGCTGGGTAATATTTCAGTACATAGATGTAT
CACAATGGACACACTCA / Celera SNP ID:   hCV29859556 / Public SNP ID:   rs6704286 /
SNP Chromosome Position:   243678389 / SNP in Genomic Sequence:   SEQ ID NO: 365 /
 SNP Position Genomic:   36854 / Related Interrogated SNP:   hCV31523608 / SNP
Source:   dbSNP; HapMap / Population(Allele,Count):   Caucasian (T,24|G,200) /
SNP Type:    INTRON /
Context           (SEQ ID NO: 1275): /
GACTATAAGCAGCACTCAGCTACATCTCTCAAAAATAAGGGAAAGCCATTTTAAGAGCTTGAAGTAGTATGTTGTAGTCCTAT
GACATTAATAGTTTAAC
R

GAAACCACATACAACTTGAAGACTAAAACTATCAACTCCCTAAACAACTCACAGTGTACGTTAACATGACTTGACTATTCAGG
GAGAGCCTCACCCTGGC / Celera SNP ID:   hCV30264437 / Public SNP ID:   rs7514510 /
SNP Chromosome Position:   243891327 / SNP in Genomic Sequence:   SEQ ID NO: 365 /
 SNP Position Genomic:   249792 / Related Interrogated SNP:   hCV31523608 / SNP
Source:   dbSNP; HapMap; ABI_Val / Population(Allele,Count):   Caucasian
(A,24|G,202) / SNP Type:   INTRON /
Context           (SEQ ID NO: 1276): /
TAAATTCACCAAGAAAACAATCCTTAATCTAAATATGTATGCAAGTAACAATGAGGCTTCAAAACACATGAGGCAAAACCTAA
AAGATAAAGTCCACGAT
Y

ATAGTTGACGACATCAACTATCTTTTTCAGAAACCAATAGAACAAGTAGGCAGAAAATCAGTAAGAATATAGAAGACATAAAC
AATACTACCAGCTGTCT / Celera SNP ID:   hCV29831992 / Public SNP ID:   rs7523198 /
SNP Chromosome Position:   243702266 / SNP in Genomic Sequence:   SEQ ID NO: 365 /
 SNP Position Genomic:   60731 / Related Interrogated SNP:   hCV31523608 / SNP
Source:   dbSNP; HapMap / Population(Allele,Count):   Caucasian (C,24|T,202) /
SNP Type:   INTRON /
Context           (SEQ ID NO: 1277): /
GTGGACTGGGTAAAAGCCACTGTAAAATTTGTTTACCCTGAAAAGGGAAATGGTCCTTCCCCTCCTTTAAATGCCAAGTGGCA
CTCCTCCATAGATGACA
W

AGCTGGTAAGCTGCATATGCAAACCATGCTGGATTAACTTCATGGTCATCAGGATGTTTGTTCATCCTTAATATGCCCTTTAA
ATCATGGTAAATACTGT / Celera SNP ID:   hCV29795761 / Public SNP ID:   rs7528450 /
SNP Chromosome Position:   243789305 / SNP in Genomic Sequence:   SEQ ID NO: 365 /
 SNP Position Genomic:   147770 / Related Interrogated SNP:   hCV31523608 /
Related Interrogated SNP:   hCV30690780 / SNP Source:   dbSNP; HapMap /
Population(Allele,Count):   Caucasian (T,33|A,77) / SNP Type:    TRANSCRIPTION
FACTOR BINDING SITE;UTR5;INTRON /
Context           (SEQ ID NO: 1278): /
TACATACAATCATGCACCACATAACAATGTTTCAGTCAATGATGGACTGCATATACGATGGCAGTCCCCTAAGATTATTACAC
TGTATTTTTACTGTACC
Y

TTCCTATGCTTATATGTTTAGATACATAAATACTTACCATTGCATTACAATTGTCTATAGTATTCAGTACACTAACATGCTGT
ACAGGTTTGTAAACTGT / Celera SNP ID:   hCV29669242 / Public SNP ID:   rs7547861 /
SNP Chromosome Position:   243810582 / SNP in Genomic Sequence:   SEQ ID NO: 365 /
 SNP Position Genomic:   169047 / Related Interrogated SNP:   hCV31523608 / SNP
Source:   dbSNP / Population(Allele,Count):   Caucasian (C,24|T,200) / SNP Type:
 INTRON /
Context           (SEQ ID NO: 1279): /
AAAAAGTCTATGCATGTTCAGTATGAACGTAACCATTCTTTTTTTTTTTTTTCTCAAGTATTTTTGACACACAGTTGATAGAACC
ACAGATGCAGAACCCAC

R
ATGGGTATGAGAGGCGAACTGCATACCTATTATCTCAACTCCACTCCACTGGATAGAACATACTTCTCTTTGCTTCCAAAATT
AAGTCCATCCTCAAAAA / Celera SNP ID: hCV30390695 / Public SNP ID: rs7553458 /
SNP Chromosome Position: 243954652 / SNP in Genomic Sequence: SEQ ID NO: 365 /
SNP Position Genomic: 313117 / Related Interrogated SNP: hCV31523608 / SNP
Source: dbSNP; HapMap; ABI_Val / Population(Allele,Count): Caucasian
(A,19|G,101) / SNP Type: INTRON /
Context (SEQ ID NO: 1280): /
AGGCCTGTACTTTAAATCATCTCTAGATTATTTAAAACACCTAATACAATGTAGATACTATATAAATAGTCACACTGTATTGT
TTAGGGGAAAATAAGGG

R
AAAAAGTCTATGCATGTTCAGTATGAACGTAACCATTCTTTTTTTTTTTTTCTCAAGTATTTTTGACACACAGTTGATAGAACC
ACAGATGCAGAACCCAC / Celera SNP ID: hCV30084348 / Public SNP ID: rs9287269 /
SNP Chromosome Position: 243954551 / SNP in Genomic Sequence: SEQ ID NO: 365 /
SNP Position Genomic: 313016 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (G,185|A,39) / SNP Type: INTRON /
Context (SEQ ID NO: 1281): /
AGTGACCTTGACTGTGCATATGCCTTCAGTTCTTGGTAGAACAGTGTTCACTTGCTCATGATTGCCCGTGAAGTCTCGACATC
CACATGTGATATGGGCT

K
CTTCTACAGTATCCACCAGGAATTTAAAAAAAAAAAAAATTATATATATATATATATATCCCAACAGTTGTTCAGTCCTTCTACC
AAATGCTTCCTAATCAA / Celera SNP ID: hCV30382231 / Public SNP ID: rs9428966 /
SNP Chromosome Position: 243667900 / SNP in Genomic Sequence: SEQ ID NO: 365 /
SNP Position Genomic: 26365 / Related Interrogated SNP: hCV233148 / Related
Interrogated SNP: hCV30690780 / Related Interrogated SNP: hCV31523650 / SNP
Source: dbSNP; HapMap / Population(Allele,Count): Caucasian (T,164|G,60) /
SNP Type: MICRORNA;UTR3;INTRON /
Context (SEQ ID NO: 1282): /
TGGAATTCACTGATACACAAAGAAGATTTGTAGCCATGACAGCAGAAGAGCTAAGAATTTGCAGAGGGCAGAAAAGGCTCAAG
AACAGAGTAAGGATGGG

W
TATAGTCACATTATGTAGGTATCAAAAGAAAGTATGTCAGTAAAAGAGCACAGTCCAATGCAGCAATGAAACAGGAAAGCCAA
ACATCAGGGAAGCCATG / Celera SNP ID: hCV30210344 / Public SNP ID: rs9428970 /
SNP Chromosome Position: 243930449 / SNP in Genomic Sequence: SEQ ID NO: 365 /
SNP Position Genomic: 288914 / Related Interrogated SNP: hCV31523608 / SNP
Source: dbSNP; HapMap; ABI_Val / Population(Allele,Count): Caucasian
(T,24|A,200) / SNP Type: INTRON /
Context (SEQ ID NO: 1283): /
GCAGAAAATTAACTACAAGGTGAGAATATGAAAGATGCCAGTGTGGACAAACCATGCAATAAAATTTTATTAAAAGAGAAGCA
GAAATAGAGGCAGTAGC

R
GAATGGGAAGCAATATGTCAAGAGGGGGGCTTTTCTTTTTAGGATGGAAGATACTATTGCACCTTTATATGCCGGTGTGAATGA
TAAAGCAGAGAGGGAGA / Celera SNP ID: hCV30372886 / Public SNP ID: rs9782883 /
SNP Chromosome Position: 243892532 / SNP in Genomic Sequence: SEQ ID NO: 365 /
SNP Position Genomic: 250997 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (A,189|G,37) / SNP Type: INTRON /
Context (SEQ ID NO: 1284): /
AGAGAAAACACAAGTTCAAGAGGTACAGCTTCTCTGCATTCTCCACTGGGAAGTAGAGCAGTGAATAATATTACTCACACAGT
GTGAGGATTCCACAAGG

Y
GGAAGATACTCCCCCTTTTCCATACTCAGAGGATTTTAAATCTTTCCACCCAAAACTTCTGAGCCCCTCCCCCCCAACAAGAT
ACAAAATATTTTTTGAA / Celera SNP ID: hCV29633221 / Public SNP ID: rs10157763 /
SNP Chromosome Position: 243995041 / SNP in Genomic Sequence: SEQ ID NO: 365 /
SNP Position Genomic: 353506 / Related Interrogated SNP: hCV31523608 / SNP
Source: dbSNP; HapMap / Population(Allele,Count): Caucasian (T,35|C,83) / SNP
Type: INTRON /
Context (SEQ ID NO: 1285): /
TGCATGCAAGACCAACAGAGTGGCCCAAACTACAAAATCATGAGATATTTACATGGCTATTGTTTTAAGACATTAAATTTGAG
GTAGTTTGTTCCACAGC

R
TGAGGGTTAATACTGAGTGTCAATTTGATTGTATTGAAAGATGCAAAATATTGATCCTGGGTGTATCTGTGAGGGTGTTGCCA
AAGGAGATTAATATTTG / Celera SNP ID: hCV31523557 / Public SNP ID: rs10754807 /
SNP Chromosome Position: 243804158 / SNP in Genomic Sequence: SEQ ID NO: 365 /
SNP Position Genomic: 162623 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (G,187|A,37) / SNP Type: INTRON /
Context (SEQ ID NO: 1286): /
GAAATAATTTTTCAAATTAATGCTATTCTTGTTACCTGGAAAGTAACTTTATCTTTTGAAATCTTACTATTTCTCCAAACAAT
CTCAATGTCCAAAGAAG

M
TTTTCTTGATGATTCTGTCTTTAATGATACCTCTTTTTCCTAAATTGTAACTGCATGTATTAATTGCAAAACTCCCTTCAAAA
CTTAGCCATAAAACATC / Celera SNP ID: hCV31523707 / Public SNP ID: rs10803152 /
SNP Chromosome Position: 243846167 / SNP in Genomic Sequence: SEQ ID NO: 365 /
SNP Position Genomic: 204632 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (C,194|A,32) / SNP Type: INTRON /
Context (SEQ ID NO: 1287): /
ACTAAGGGGCAAGAAGAGGTAATGGGCAAGCTCTGAGTCACTCTTACTGGATGGTCTCAGTACAGCAACAGTAACTAGGATAA
GCCAAGGCGTCCTCTAC

R
CACATAGTGAGGCCAGCTTGAAACAAGGAAGTACATAGTTGCAGACTTGGAAGCTAGCCACCGCAGCTATTTAAAAATCAATA
GAGAGATTATTTTTTAA / Celera SNP ID: hCV31523731 / Public SNP ID: rs10803155 /
SNP Chromosome Position: 243894782 / SNP in Genomic Sequence: SEQ ID NO: 365 /
SNP Position Genomic: 253247 / Related Interrogated SNP: hCV31523608 / SNP
Source: dbSNP; HapMap; ABI_Val / Population(Allele,Count): Caucasian
(A,24|G,202) / SNP Type: INTRON /
Context (SEQ ID NO: 1288): /
CTCCATGTTGGTCAGGCTGGTCTCAAACTCCTGGCCTCAGGTGATCCACCCACCTCAGCCTCCCATAGTGCTGAGATTCCAAG
CATGAGCCACCACATCC

R
ACAACCTTATCATTTTTATAAGTGAATTACTGAAAACCTTCTAACTGCAGCAACCTCCCAAGTATTTTCCTTGCTTCCACTTT
TGTCCTCTCTACTTCAA / Celera SNP ID: hCV31523624 / Public SNP ID: rs10927044 /
SNP Chromosome Position: 243767493 / SNP in Genomic Sequence: SEQ ID NO: 365 /
SNP Position Genomic: 125958 / Related Interrogated SNP: hCV31523608 /
Related Interrogated SNP: hCV30690780 / Related Interrogated SNP: hCV31523650
/ SNP Source: dbSNP / Population(Allele,Count): Caucasian (G,61|A,163) / SNP
Type: INTRON /
Context (SEQ ID NO: 1289): /
CTCCTGACCTTGTGATCCGCCCACCTTGGCCTCCCAAAGTGCTGGGATTACAGGCGTGAGCCACCACGCCCAGCCACAGTAAG
TCTTTAATAAAAATATA

S
GAGTATGAATACTCTCCAGAAAAATAATCCTCCTCCAAAGCCTGATCTTAAGAATTTATTGGGGAAATACTTTACAGTTACAC
TCTTCCCTTTACATGTT / Celera SNP ID: hCV31523563 / Public SNP ID: rs10927051 /
SNP Chromosome Position: 243805936 / SNP in Genomic Sequence: SEQ ID NO: 365 /
SNP Position Genomic: 164401 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (G,90|C,14) / SNP Type: INTRON /
Context (SEQ ID NO: 1290): /
AAAGAAAAAAGGAGAAAAGTCAGAGCTTCTCTTTTTTGTTTTCTAGTTATTCCATTCATATTGATTGGTTCTGAAATGTTACA
AAAATTTTTGGTGTTCT

R
GAAAATAAGTAATATGGAAAATTTTTACCTAAGGTCACTGTTTCTTCTCAATTATTTAAGGTGAAAGTAATAAACCAGACATT
TAAGTAAACATGCTCAC / Celera SNP ID: hCV31523725 / Public SNP ID: rs10927060 /
SNP Chromosome Position: 243875989 / SNP in Genomic Sequence: SEQ ID NO: 365 /
SNP Position Genomic: 234454 / Related Interrogated SNP: hCV31523608 / SNP
Source: dbSNP; HapMap / Population(Allele,Count): Caucasian (A,36|G,84) / SNP
Type: TFBS SYNONYMOUS;INTRON /
Context (SEQ ID NO: 1291): /
TATAAGGAAACAGCAAGTCAAAATAACAACAGGTGACTTTTCATCAGAAACCATGGCAGCCAGAAGAAAATGAAATGACATAT

TTAAAATGATAAAAAAA
M
CACAATTATCAAATTAGAATCCTATATACAATTTAAAAAAATCCTTCAAAAATGAAGATGAGCCCAGCCACAGTGGCTCACACC
TGCAATCCTAGCACTTT / Celera SNP ID: hCV31523692 / Public SNP ID: rs10927082 /
SNP Chromosome Position: 243997019 / SNP in Genomic Sequence: SEQ ID NO: 365 /
SNP Position Genomic: 355484 / Related Interrogated SNP: hCV31523608 / SNP
Source: dbSNP; HapMap / Population(Allele,Count): Caucasian (C,17|A,101) /
SNP Type: INTRONIC INDEL;INTRON /
Context (SEQ ID NO: 1292): /
CCTCTCTTCTAATAATGTGACATAGGTTAGCGCTTAATGTATATACTGTCTTGAATTATTCTCTGTGTGTTTCATATACTGGT
CTTATTCTCTGGGTAGA
Y
AATGAAAGCCTGGAAGTAGATTATGTACAATTTGCCTCATCTCTATTTATTTTCCCAGTTTCCTTATTATAGAGCACGCTTTC
ACTCTCAACAGGGTACT / Celera SNP ID: hCV31523744 / Public SNP ID: rs12031994 /
SNP Chromosome Position: 243917309 / SNP in Genomic Sequence: SEQ ID NO: 365 /
SNP Position Genomic: 275774 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (T,193|C,33) / SNP Type: TRANSCRIPTION
FACTOR BINDING SITE;INTRON /
Context (SEQ ID NO: 1293): /
TGTTTAGATTAAAATGTTTAAAGGTTTAATTTCAACCAACCTCTTTAGCCATTTAAACACTAATTTTATTCTTTCTAAAATAA
CATCCAATGTGAAAAAC
R
AAGGCATAATAATAGATTCCTTTTTTTTTTTTTTTTTTTTTTGAGACGGAGTCTCGCTCTGTTGCCCAGGCTGGAGTGCAGTGGCGC
AATCTCGGCTCATGCAA / Celera SNP ID: hCV31523740 / Public SNP ID: rs12032342 /
SNP Chromosome Position: 243834360 / SNP in Genomic Sequence: SEQ ID NO: 365 /
SNP Position Genomic: 192825 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (G,102|A,18) / SNP Type: INTRON /
Context (SEQ ID NO: 1294): /
CCATTGGAATCTCCCTAAAAACTCAGCTGAATCTTCTGAGGCCTCTTTCATCCATCGCTACTTAGGTCACTCTTCAATTTCTC
AGGATGGTTGTTTCAAA
W
CTTTCCCAGGCCTTCTTCCTTGCCTGGTAGGTATACTTAAATGTACACACTAACCCTTACCTAGTCAAGCTACTAAGCTAAGA
CTAGAACCCAGGTCTGT / Celera SNP ID: hCV31523639 / Public SNP ID: rs12034588 /
SNP Chromosome Position: 243784392 / SNP in Genomic Sequence: SEQ ID NO: 365 /
SNP Position Genomic: 142857 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (T,99|A,17) / SNP Type: TRANSCRIPTION
FACTOR BINDING SITE;INTRON /
Context (SEQ ID NO: 1295): /
GAATTACCCAAAATGTGACACAGAGGCACAACATAAGCACATGCTCTTGGAAAAAATGGCACTGATAGACTGGCTCGACTCAAG
GTTGCCACAAATCTTCA
M
TTTGTAAGAAATGCAGTATTAGCAAAGTGCAATAAAACAAGCTAGGCCTGTACTTTAAATCATCTCTAGATTATTTAAAACAC
CTAATACAATGTAGATA / Celera SNP ID: hCV31523658 / Public SNP ID: rs12047209 /
SNP Chromosome Position: 243954407 / SNP in Genomic Sequence: SEQ ID NO: 365 /
SNP Position Genomic: 312872 / Related Interrogated SNP: hCV30690780 /
Related Interrogated SNP: hCV233148 / Related Interrogated SNP: hCV31523650 /
Related Interrogated SNP: hCV31523608 / SNP Source: dbSNP; HapMap /
Population(Allele,Count): Caucasian (A,104|C,16) / SNP Type: INTRON /
Context (SEQ ID NO: 1296): /
AAAATTTCAGGGGAGGGGTCTCATCAAATATTTGTTCAAGCTATGAAATACCTGGAAATCTGATTTTTGAAGAAGTGTTAGCC
CTACATCTGGGAAATTT
R
TATCATTAATGTTAACTTCATCACATATATAACTGTATGTTCCATATATCGTCAATAAAGAACACTAACAGTATAAACTCCAG
CCTAGTTCTTCAAAGAC / Celera SNP ID: hCV31523736 / Public SNP ID: rs12124113 /
SNP Chromosome Position: 243904366 / SNP in Genomic Sequence: SEQ ID NO: 365 /
SNP Position Genomic: 262831 / Related Interrogated SNP: hCV30690780 /
Related Interrogated SNP: hCV31523650 / Related Interrogated SNP: hCV233148 /

Related Interrogated SNP: hCV31523608 / SNP Source: dbSNP; HapMap / Population(Allele,Count): Caucasian (A,193|G,33) / SNP Type: INTRON / Context (SEQ ID NO: 1297): /
TCATCATTATCTGTACCTCAGTAAGCATAATAATGCTGTTTTCAAATGTTTCCAGTGACTTTGAAAGGAAAGATTTTCTTAAA
ACCAAGGCATCAGGAAG
R

AAAATGCATGGAATCATCAGTATCACTGAAGTCATCCAAAATACAATAAAATAAAAAAGTATATAAGAATTTTACATTGCATA
AATAATCTAAGTTCCTG / Celera SNP ID: hCV31523723 / Public SNP ID: rs12140040 /
SNP Chromosome Position: 243871922 / SNP in Genomic Sequence: SEQ ID NO: 365 /
SNP Position Genomic: 230387 / Related Interrogated SNP: hCV30690780 /
Related Interrogated SNP: hCV233148 / Related Interrogated SNP: hCV31523650 /
Related Interrogated SNP: hCV31523608 / SNP Source: dbSNP; HapMap /
Population(Allele,Count): Caucasian (G,106|A,14) / SNP Type: INTRON /
Context (SEQ ID NO: 1298): /
GATTTCTACTCAGCGCTGAGCTACTTGCCCATGACTCCACAGGCTTTCCAACATTGTCTGTGACTCTTAGTGATTCTAAAATT
CTAGCAATGGCACAAGA
Y

TGAGTGGAAACTCTGCCTCACCACAGGACTGACCATGAGTAGCTCACTTCAATGGATTGGTATTTCACAACTTAGCCTGGACA
TCGGTCAAGTTGGTAAG / Celera SNP ID: hCV30690778 / Public SNP ID: rs12140414 /
SNP Chromosome Position: 243674387 / SNP in Genomic Sequence: SEQ ID NO: 365 /
SNP Position Genomic: 32852 / Related Interrogated SNP: hCV30690780 /
Related Interrogated SNP: hCV233148 / Related Interrogated SNP: hCV31523650 /
Related Interrogated SNP: hCV30690777 / SNP Source: dbSNP; HapMap /
Population(Allele,Count): Caucasian (T,185|C,41) / SNP Type: UTR5;INTRON /
Context (SEQ ID NO: 1299): /
CACACAAAAATGAGTAGGAAACTCTATGAGCTCATGGTACACACCATACTACTGATCATTCTACCCATGTAATTATAAAGTAG
TATTTCATTTATGCTAT
M

AAGGGTTTAATCAGGTCCTGTGAACCCTCTAGTCCAACAAAAATAATGTTACGTGTAATTACAAATAAACAGTATAATAGAAA
TAAACCTATATTTTTTA / Celera SNP ID: hCV30012351 / Public SNP ID: rs10158245 /
SNP Chromosome Position: 243833397 / SNP in Genomic Sequence: SEQ ID NO: 365 /
SNP Position Genomic: 191862 / Related Interrogated SNP: hCV31523608 /
Related Interrogated SNP: hCV30690780 / SNP Source: dbSNP; HapMap /
Population(Allele,Count): Caucasian (A,31|C,81) / SNP Type: INTRON /
Context (SEQ ID NO: 1300): /
CAGCCTCAACAACAGAGCAAAACTGTCTCAAAAAAAAAAAAAAAAGGGTGAAGAAAACCGACTATTTATTGACTCCCTAGGGTC
TAGGAGCTCGACTGAGT
R

GCTCATCTACTTTAACATCCATTAATCATCACAACTACCCTCTGATTAGATATTATTCTCATTCTACTGAGACCCAGAAACAT
TAGAAATTTACTTATGG / Celera SNP ID: hCV31523659 / Public SNP ID: rs10733129 /
SNP Chromosome Position: 243957150 / SNP in Genomic Sequence: SEQ ID NO: 365 /
SNP Position Genomic: 315615 / Related Interrogated SNP: hCV31523608 / SNP
Source: dbSNP; HapMap / Population(Allele,Count): Caucasian (A,20|G,206) /
SNP Type: INTRON /
Context (SEQ ID NO: 1301): /
TTAAGGATGTATCCCCTCCACTGCACACTATAACCAGGTACATCCTTTTAAAAAAATTGAATACTGATTTGGAAACCGGATGT
TCAAAAATTTCATCACA
Y

TGATATTTAAGACTTTCCATAATCTGATTACTAACTCTCTAATCTGAGCTTTTTTTTTTTCCTCCAGCATAAATTCTCCATCCA
TCTAGGATATTGCCTAT / Celera SNP ID: hCV31523710 / Public SNP ID: rs10927059 /
SNP Chromosome Position: 243852079 / SNP in Genomic Sequence: SEQ ID NO: 365 /
SNP Position Genomic: 210544 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (T,187|C,39) / SNP Type: ESE;SILENT RARE
CODON;SILENT MUTATION;INTRON /
Context (SEQ ID NO: 1302): /
TTGGGGATTTTCCCACTATCTTTGTTTTTTTAATAGTCTAATGAGATTAGGGATTTTCTTCATATTTTTCCTACTTGAGATTTA
TAGGACTTTTGAAACTA
Y

GGTTTGGTATCTTTAACTATTTTTTAAAAAAATTTCAATCATTATATTTTCAAATACTTTTTGCTCTATTCTCTCCTCTCCTTC
TAAGGCTCCAGTTATAT / Celera SNP ID: hCV31523573 / Public SNP ID: rs11589907 /
SNP Chromosome Position: 243818260 / SNP in Genomic Sequence: SEQ ID NO: 365 /

SNP Position Genomic: 176725 / Related Interrogated SNP: hCV31523608 /
Related Interrogated SNP: hCV30690780 / SNP Source: dbSNP; HapMap /
Population(Allele,Count): Caucasian (T,34|C,84) / SNP Type: INTRON /
Context (SEQ ID NO: 1303): /
AGTTCCAAATATTCCTTAGATGAAGGCCAAATGAACTCCTTTGACTTCCATCATGATATAATCACTTTGGACTCCAGTCTTTC
TTCTGCCTCTCTCATAC
R
CGGTATTGGGACAAGTTCATCTCTACCCTCTTAACTCTATATTTCACATCTAAAGATAAATTTACACAAACATATTGACTAAC
ACCATTGGAATCTCCCT / Celera SNP ID: hCV31523638 / Public SNP ID: rs12037013 /
SNP Chromosome Position: 243784207 / SNP in Genomic Sequence: SEQ ID NO: 365 /
SNP Position Genomic: 142672 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (G,194|A,32) / SNP Type: INTRON /
Context (SEQ ID NO: 1304): /
AGAGTCAGAGGTTTTGTAGGAAAAATAACATTTTAAGCTCAGATCTGAAAAGATACATAGGAATTAGCCCAACTTAGTGATTA
TGGATTATGGGTGGACA
Y
ATCAACAAATTGAAAGCTCAGTATTAATTATCAAGGGAGAAAAGGCCCAGGATCAATGGTGGGGAGGGGAGGGTGTGCGGGGA
GGGAGGCAGTGCAGGGA / Celera SNP ID: hCV31523737 / Public SNP ID: rs12117580 /
SNP Chromosome Position: 243826674 / SNP in Genomic Sequence: SEQ ID NO: 365 /
SNP Position Genomic: 185139 / Related Interrogated SNP: hCV31523608 /
Related Interrogated SNP: hCV30690780 / SNP Source: dbSNP /
Population(Allele,Count): Caucasian (T,56|C,170) / SNP Type: INTRON /
Context (SEQ ID NO: 1305): /
TTGGGGGCGAGGCATACGTGTGCGCATGCACATGTGTCTCTGTATGTGAGTGTGAGTGTCGTGGCCATGGTTGTGACAGTGAA
AGTAGTTAGCTGTTAAG
R
AAGAAAAATGACCTTTGTCTATATTGTGGATGGTCATGTGCAGCATAATAAAGTACATATACAAATCATATTTAACAGTAAAT
TAGTGGTGTATCCAATA / Celera SNP ID: hCV31523576 / Public SNP ID: rs12691548 /
SNP Chromosome Position: 243820128 / SNP in Genomic Sequence: SEQ ID NO: 365 /
SNP Position Genomic: 178593 / Related Interrogated SNP: hCV31523608 /
Related Interrogated SNP: hCV30690780 / Related Interrogated SNP: hCV31523650
/ SNP Source: dbSNP; HapMap / Population(Allele,Count): Caucasian
(A,61|G,165) / SNP Type: INTRON /
Context (SEQ ID NO: 1306): /
GCAGGCTATTATCTAAGTTGCAGTGGCTCAATTAATGCCTTTGGGTCCTTACGATATACACTCAGATATGATTTTAGAAGTAT
CTGCAACTCAGACTGGA
S
CTTATGGCAAAAAGCCCATGAATGCTGTCCAGCAGTAACCACTGGGACCATGGGCCAGACAAGAAAGTATAGAACACATCATT
AGAGAGACGATTATTGG / Celera SNP ID: hCV31523552 / Public SNP ID: rs12739344 /
SNP Chromosome Position: 243791312 / SNP in Genomic Sequence: SEQ ID NO: 365 /
SNP Position Genomic: 149777 / Related Interrogated SNP: hCV31523608 /
Related Interrogated SNP: hCV30690780 / Related Interrogated SNP: hCV31523650
/ SNP Source: dbSNP; HapMap / Population(Allele,Count): Caucasian
(G,63|C,161) / SNP Type: INTRON /
Context (SEQ ID NO: 1307): /
GGACATGTTGATGGTCATCAAAAGAACTTCTTTCAGGATCAGGAGATGACTGGAACCAGCAAGTGGATGTCCCAGCATGCTTG
TTTGAGGTGGCTATCTG
R
GTCCCCAAAATAAGTAGATATGGGAGATATTGGCCGGGCACAGTGGCTCATGCCTGTAATCCCAGCACTTTGGGAGGCCAAGG
CGGGTGGATCATGAGGT / Celera SNP ID: hCV31523555 / Public SNP ID: rs12749316 /
SNP Chromosome Position: 243792781 / SNP in Genomic Sequence: SEQ ID NO: 365 /
SNP Position Genomic: 151246 / Related Interrogated SNP: hCV31523608 /
Related Interrogated SNP: hCV30690780 / SNP Source: dbSNP; HapMap /
Population(Allele,Count): Caucasian (G,37|A,83) / SNP Type: INTRON /
Context (SEQ ID NO: 1308): /
CTCTGGGACAGAAATCACAGAGGAAGGAGTAGGCAGCCATCTTTGCTGTTCTGCAGCCCCCTCTGGTGACATCTCCAGGTGTG
GGAGGGATCCAAACGCA
Y
AGGGTCTGGAACGGGCCCCCAGCAAACTGCAGCAGCCCTACGGGAGAGGGGCCTGTTTTTTTTGTTTTGTTTTGAGATGGGGTC
TTGCTCTGTCACCCAGG / Celera SNP ID: hCV31523680 / Public SNP ID: rs4484910 /
SNP Chromosome Position: 243983166 / SNP in Genomic Sequence: SEQ ID NO: 365 /

SNP Position Genomic: 341631 / Related Interrogated SNP: hCV31523608 / SNP Source: dbSNP; HapMap / Population(Allele,Count): Caucasian (C,24|T,202) / SNP Type: INTRON / Context (SEQ ID NO: 1309): / CTGCACGCCTCAGCCACTTGGACATAGCAAGATAGCAGATAAAGATAAACTCTGTGAGCTTTAATTCAAGAAGGAAAACAGGA ATCTACTGGAATCATGA M GTACACTCCAGATCCCAGAGAGGAGAAGGCCAGCAAACAGCCCCACTGACGGCATCCAGCTGATAAAAGTTAAGTGAAGCCCC AGTGCATGAGAGAGGCA / Celera SNP ID: hCV30606791 / Public SNP ID: rs6688135 / SNP Chromosome Position: 243743474 / SNP in Genomic Sequence: SEQ ID NO: 365 / SNP Position Genomic: 101939 / Related Interrogated SNP: hCV31523608 / SNP Source: dbSNP; HapMap / Population(Allele,Count): Caucasian (C,24|A,202) / SNP Type: INTRON / Context (SEQ ID NO: 1310): / TGAGAATTCTGAGGTTAGGGAAACTCTTTGAAACAGAGTTCAAGAAGAGCTACTTAGAAATAAAGAAACAAATTCAGATCATG ATAGCCATAAAGCAGAA Y CTGCAGAAGGGAGCATCAAAAAACCAAGGGCAGTTTTGCATATTTACCAATTTAATCTACTATTTCAAAAAAGCTGAAGCACT AGTCTTATAGTAGTGAC / Celera SNP ID: hCV29723686 / Public SNP ID: rs7512207 / SNP Chromosome Position: 243964992 / SNP in Genomic Sequence: SEQ ID NO: 365 / SNP Position Genomic: 323457 / Related Interrogated SNP: hCV31523608 / SNP Source: dbSNP; HapMap; ABI_Val / Population(Allele,Count): Caucasian (C,18|T,98) / SNP Type: INTRON / Context (SEQ ID NO: 1311): / CACACAAAACCAACTCAAAATGGATCAAAGAATAAATATAAGACATGAAACTACAAACTCCTAGGAAAAAAACCACAGGAGAAA AGCTTCATAATTTGGTC Y TTAGAAATGATTTCTTGATGCTGACAAAAGCAGTAATAGATGAGTAGAAGTATATCAAAATAAAAAGCTTCTGCACAGCAAAC AACAGAACAAAACAGAA / Celera SNP ID: hCV30354518 / Public SNP ID: rs7517732 / SNP Chromosome Position: 243821364 / SNP in Genomic Sequence: SEQ ID NO: 365 / SNP Position Genomic: 179829 / Related Interrogated SNP: hCV31523608 / SNP Source: dbSNP; HapMap / Population(Allele,Count): Caucasian (T,23|C,201) / SNP Type: INTRON / Context (SEQ ID NO: 1312): / GCTTTGAGGGTAGCTCCCAGGAAAGGCTGCCTCAGCCCCAGCGTGATGGGGACGGGTAGGGGCCGATGAGGCTTCCCAGAGCA GCGATGGCCTTAGGTAT Y GCCAGAAGGAAACCTACTTTTCCTCCAAGATCAGAGGGAAGGATGAGGTGGGGTGTTGGAGCATCACCATATATATACATCAC ACACACAGGGAAACACT / Celera SNP ID: hCV30690784 / Public SNP ID: rs4658574 / SNP Chromosome Position: 243650716 / SNP in Genomic Sequence: SEQ ID NO: 365 / SNP Position Genomic: 9181 / Related Interrogated SNP: hCV233148 / Related Interrogated SNP: hCV30690780 / SNP Source: dbSNP; HapMap; ABI_Val; HGBASE / Population(Allele,Count): Caucasian (T,167|C,59) / SNP Type: INTRON / Context (SEQ ID NO: 1313): / AACACTACTGATGTTTATCCAGCCAACAAAAAAGTATAATAGCACAAAGACTGCCTTCTGAATACTCCCTAGAGGCCAATCAG CCAGGTGTGAGAATTCT K AGGTTAGGGAAACTCTTTGAAACAGAGTTCAAGAAGAGCTACTTAGAAATAAAGAAACAAATTCAGATCATGATAGCCATAAA GCAGAACCTGCAGAAGG / Celera SNP ID: hCV29542869 / Public SNP ID: rs7534117 / SNP Chromosome Position: 243964902 / SNP in Genomic Sequence: SEQ ID NO: 365 / SNP Position Genomic: 323367 / Related Interrogated SNP: hCV30690780 / Related Interrogated SNP: hCV31523650 / Related Interrogated SNP: hCV233148 / Related Interrogated SNP: hCV31523608 / SNP Source: dbSNP / Population(Allele,Count): Caucasian (G,186|T,38) / SNP Type: INTRON / Context (SEQ ID NO: 1314): / TAAGAAAGTAGAACCCTTCACATATTAGTGCTAGGAATGTAAACTGGTAACATCTGGACAATAAACTGACTTTATAAATGATT TATATAATCAAAACATA R TAAAAGTTCCCTTAAAGAGTAAAACTGAGAAAAAGTGGCTACAGCCTTTAATCGCACCGAAGGCCCCTACAGTGACAGACTAT GAGAAGAGGCTAACAGT / Celera SNP ID: hCV29560960 / Public SNP ID: rs7519673 / SNP Chromosome Position: 243993123 / SNP in Genomic Sequence: SEQ ID NO: 365 / SNP Position Genomic: 351588 / Related Interrogated SNP: hCV30690780 / Related Interrogated SNP: hCV31523650 / Related Interrogated SNP: hCV233148 /

Related Interrogated SNP: hCV31523608 / SNP Source: dbSNP; HapMap /
Population(Allele,Count): Caucasian (A,193|G,33) / SNP Type: INTRON /
Context (SEQ ID NO: 1315): /
ACTATCTTTTTGAAAAATTTAAATCAGGAGGGAAGCAATTCAAAATTTAATTAAGATTGGAAGTTCTCAAACCCCTTTACAGT
TCTCATACTCTGTTCTT
Y
TCATATAAATAAAAAGCCTATTTTGGTAAAATTTCATTAAAACCCATATTGATTAAATATGAAGTAATATCTTTAATTATCAG
AAAAATGAGGGATATAT / Celera SNP ID: hCV29741723 / Public SNP ID: rs7517921 /
SNP Chromosome Position: 243856420 / SNP in Genomic Sequence: SEQ ID NO: 365 /
 SNP Position Genomic: 214885 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (T,186|C,40) / SNP Type: INTRON /
Context (SEQ ID NO: 1316): /
TTATAAATATGAATGAATGTATAAACATGAATAAAAATACTGTAGCTCATTGAACAAAGCAGCAAGAGCCATAGAGTTCTTCA
ATTCATAGCTGTAGCTA
M
AAAAACAGAAATTAAAAGTGTGTACAATTATTTTTTGCCATCAACTCTTTCAGCTATGCATAGAAAAGATTATTTTCAAATGA
CAAAAAAGCAGTTAGAG / Celera SNP ID: hCV29994467 / Public SNP ID: rs6694738 /
SNP Chromosome Position: 243715210 / SNP in Genomic Sequence: SEQ ID NO: 365 /
 SNP Position Genomic: 73675 / Related Interrogated SNP: hCV30690780 /
Related Interrogated SNP: hCV233148 / Related Interrogated SNP: hCV31523650 /
Related Interrogated SNP: hCV31523608 / SNP Source: dbSNP; HapMap /
Population(Allele,Count): Caucasian (C,193|A,33) / SNP Type: INTRON /
Context (SEQ ID NO: 1317): /
GTTATTAATAGATACACAATACATGTCTGTGAAAAAAATGAACCAATCAGCCAATGTACCCTTGACTGCAAGATCAGAAATTT
TTCCCTTACATGGATCA
Y
TGACTCATAATGTTTCTACAAAATTGGTGAAAATTATGGTTAACTAAGATTTGTTCTGCATACACCAAAGTCATTATTTCAAG
TAACATAAAACAGGTTC / Celera SNP ID: hCV30048213 / Public SNP ID: rs7552982 /
SNP Chromosome Position: 243737428 / SNP in Genomic Sequence: SEQ ID NO: 365 /
 SNP Position Genomic: 95893 / Related Interrogated SNP: hCV31523608 / SNP
Source: dbSNP; HapMap; ABI_Val / Population(Allele,Count): Caucasian
(T,202|C,24) / SNP Type: INTRON /
Context (SEQ ID NO: 1318): /
CTGAAAAGGGAAATGGTCCTTCCCCTCCTTTAAATGCCAAGTGGCACTCCTCCATAGATGACATAGCTGGTAAGCTGCATATG
CAAACCATGCTGGATTA
R
CTTCATGGTCATCAGGATGTTTGTTCATCCTTAATATGCCCTTTAAATCATGGTAAATACTGTGACTGAAGGGGCCCCGCTAG
GCAGGCATCTGATATAA / Celera SNP ID: hCV31523643 / Public SNP ID: rs6671475 /
SNP Chromosome Position: 243789342 / SNP in Genomic Sequence: SEQ ID NO: 365 /
 SNP Position Genomic: 147807 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (A,186|G,40) / SNP Type: TRANSCRIPTION
FACTOR BINDING SITE;UTR5;INTRON /
Context (SEQ ID NO: 1319): /
AAGAAACAAATCTGAAGTATCTAAGATCAGCAAAAAAGAAATCTCAGGTACTAATCCTGGCTCTATACCTAACTTGAAATAT
GGCCTCAGTTTTGAAAT
M
TGTCAGATGAATGGATATAAGACTAGAGAGGCCACCAACAGTGGCTCCTGCCTGTAATCCCAACATTTTGGGAGGCCGAGGCA
TGAGGACTCCTGAAGGC / Celera SNP ID: hCV31523688 / Public SNP ID: rs12049228 /
SNP Chromosome Position: 243995271 / SNP in Genomic Sequence: SEQ ID NO: 365 /
 SNP Position Genomic: 353736 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP /
Population(Allele,Count): Caucasian (C,101|A,17) / SNP Type: INTRON /
Context (SEQ ID NO: 1320): /
ATAAGTGATTCAAACTCGGTTTAAACTATTAGTTTCATACACCTCATAACTTGTAGTACTCATAATTTCTGATAAATTTTTAT
AAACTGTACTCCTACTT
Y
TCACAAACTTCTTCTCCTCATAATTTAAGAATTAAATTGGAATACAGCAGTATGTGAATCATGAAGCTTAAATAAATTTGTGT
ATCTATACATTAAAACA / Celera SNP ID: hCV31523691 / Public SNP ID: rs12021907 /

SNP Chromosome Position: 243995640 / SNP in Genomic Sequence: SEQ ID NO: 365 /
SNP Position Genomic: 354105 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP /
Population(Allele,Count): Caucasian (C,193|T,33) / SNP Type: INTRON /
Context (SEQ ID NO: 1321): /
TTATTGTTTTATCTTTTTCTTAAAAAAAATACAGTAGCCATTCTAACCCACTAATAAGGGCTAACCTGAATTAAACAATAGGA
GTAAAAAAAGATCACGG
Y
TTTTATAATGGTAAAATGAAATTTGTAACATTCAAAGACTTATTTTCTTGAATTTTTTGTTATATTATTGCATTTTTTTAGCTT
CAAATTTCCCTTGTATC / Celera SNP ID: hDV71836703 / Public SNP ID: rs6429433 /
SNP Chromosome Position: 243857994 / SNP in Genomic Sequence: SEQ ID NO: 365 /
SNP Position Genomic: 216459 / Related Interrogated SNP: hCV30690780 /
Related Interrogated SNP: hCV233148 / Related Interrogated SNP: hCV31523650 /
Related Interrogated SNP: hCV31523608 / SNP Source: dbSNP; HapMap /
Population(Allele,Count): Caucasian (C,103|T,17) / SNP Type: INTRON /
Context (SEQ ID NO: 1322): /
CTAATTAATTCTGTGGATTACAGAACATACACCATATAACTTTCATCTTCCAAAATGTACTCAGAATTATTTTATGACCAAGA
ATATGTTATGTGTATAT
R
TGTGTGTGTGTGTGTGTGTGTGTGTGTGTGTGTGTGTGTGTGTGTATGTTGGGTGTGTGTGTGTGTATATATATATGTTATGTGT
ATATATATATACACATA / Celera SNP ID: hDV69368808 / Public SNP ID: rs12145558 /
SNP Chromosome Position: 243812617 / SNP in Genomic Sequence: SEQ ID NO: 365 /
SNP Position Genomic: 171082 / Related Interrogated SNP: hCV31523608 /
Related Interrogated SNP: hCV30690780 / Related Interrogated SNP: hCV31523650
/ SNP Source: CDX; dbSNP / Population(Allele,Count): Caucasian (G,63|A,163) /
SNP Type: INTRONIC INDEL;INTRON //

Gene Number: 29 / Gene Symbol: PROCR - 10544 / Gene Name: protein C
receptor, endothelial / Chromosome: 20 / OMIM NUMBER: 600646 / OMIM
Information: // Genomic Sequence (SEQ ID NO: 366): // / SNP Information /
Context (SEQ ID NO: 1323): /
ACTCCCCTCTCCTCACAGCACTGACTCTTGCCTTCTCATGTTCTTTTCCCCTTGGTGGGCCTCGCCCCACACCTGGCACCCTC
TCTGCACAGTCCCCTGA
Y
CCTGACTGTCTATCCACAGTTCCTCTGACCATCCGCTGCTTCCTGGGCTGTGAGCTGCCTCCCGAGGGCTCTAGAGCCCATGT
CTTCTTCGAAGTGGCTG / Celera SNP ID: hCV1825046 / Public SNP ID: rs2069952 /
SNP Chromosome Position: 33763951 / SNP in Genomic Sequence: SEQ ID NO: 366 /
SNP Position Genomic: 46620 / SNP Source: Applera / Population(Allele,Count):
Caucasian (C,9|T,13) African American (C,4|T,28) total (C,13|T,41) / SNP Type:
INTRON / SNP Source: dbSNP; Celera; HapMap; ABI_Val; HGBASE /
Population(Allele,Count): Caucasian (C,53|T,63) / SNP Type: INTRON /
Context (SEQ ID NO: 1324): /
CTCCTTGGGGGCCTATTCTTCGGGCTAACTCTTTGCATGTTCTGCAGGGAGCCAAACAAGCCGCTCCTACACTTCGCTGGTCC
TGGGCGTCCTGGTGGGC
R
GTTTCATCATTGCTGGTGTGGCTGTAGGCATCTTCCTGTGCACAGGTGGACGGCGATGTTAATTACTCTCCAGCCCCCTCAGA
AGGGGCTGGATTGATGG / Celera SNP ID: hCV25620145 / Public SNP ID: rs867186 /
SNP Chromosome Position: 33764554 / SNP in Genomic Sequence: SEQ ID NO: 366 /
SNP Position Genomic: 47223 / SNP Source: Applera / Population(Allele,Count):
Caucasian (A,38|G,2) African American (A,32|G,6) total (A,70|G,8) / SNP Type:
MISSENSE MUTATION;MICRORNA;UTR3;INTRON / SNP Source: dbSNP; Celera; HapMap;
HGBASE / Population(Allele,Count): Caucasian (A,204|G,22) / SNP Type:
MISSENSE MUTATION;MICRORNA;UTR3;INTRON /
Context (SEQ ID NO: 1325): /
ATAAGCACTGAATCGACATTGGCTATGATTATTTTTGATTAATGAAGGGGAGGGGGTTATGGCACTGGAAGATTTTAAGTAGG
AAAAGGACATGATCTCA
Y
CCCTGGGTCAGGTGGAGGTCGGAATAGAGAACGGGGAGATGAAGTAGAAAGTTACTACCCCAGTCTAGATGAGACGGATGAAT
CCTGAATCAGGGCAGTG / Celera SNP ID: hCV15860324 / Public SNP ID: rs2069946 /
SNP Chromosome Position: 33762035 / SNP in Genomic Sequence: SEQ ID NO: 366 /
SNP Position Genomic: 44704 / SNP Source: HGBASE;dbSNP /
Population(Allele,Count): no_pop (C,-|T,-) / SNP Type: TRANSCRIPTION FACTOR

BINDING SITE;INTRON /
Context (SEQ ID NO: 1326): /
ACAGGGCGGAAAGTGGGAATCACTAGCTGATTTCTCCCTGGGACCTAGCGGCTGTGTCGGAGCAAGTCGGGGTCTGGGATGGA
CGGAAAGGGAAAGGACC
S
GGTTCACGCTTCCCATTCCCCAACTGCGAAAGGGCGGGTCACAGTTCACCTGTAGTGGGCGGGATCTGGCGCGGAGCCGTCGG
GACCTGGCGCACCATGG / Celera SNP ID: hCV1825005 / Public SNP ID: rs945959 /
SNP Chromosome Position: 33734905 / SNP in Genomic Sequence: SEQ ID NO: 366 /
SNP Position Genomic: 17574 / Related Interrogated SNP: hCV1825046 / SNP
Source: dbSNP; Celera; HapMap; HGBASE / Population(Allele,Count): Caucasian
(G,64|C,56) / SNP Type: UTR5;INTRON /
Context (SEQ ID NO: 1327): /
GGTCCTGGGCGTCCTGGTGGGCAGTTTCATCATTGCTGGTGTGGCTGTAGGCATCTTCCTGTGCACAGGTGGACGGCGATGTT
AATTACTCTCCAGCCCC
S
TCAGAAGGGGCTGGATTGATGGAGGCTGGCAAGGGAAAGTTTCAGCTCACTGTGAAGCCAGACTCCCCAACTGAAACACCAGA
AGGTTTGGAGTGACAGC / Celera SNP ID: hCV1825047 / Public SNP ID: rs9574 / SNP
Chromosome Position: 33764632 / SNP in Genomic Sequence: SEQ ID NO: 366 / SNP
Position Genomic: 47301 / Related Interrogated SNP: hCV1825046 / SNP Source:
dbSNP; Celera; HGBASE / Population(Allele,Count): Caucasian (C,53|G,63) / SNP
Type: MICRORNA;UTR3;INTRON /
Context (SEQ ID NO: 1328): /
CTGAGAGAGAGAAAGACACACGGTCCCAACGGGAAGGCCGATGGCCAAAGAAGGATCTACTCACCCCCAACCCTGACTGCCCA
GGGAGATGCAGGGCAGG
Y
GCCCCAGTGCTTCTTGGGAAACATGCAGACCCTGAGAGGGAAGGGCAATGCTGGATCATGGCCAGCCTTCCTGTACATCTGCA
TAGTAGAGATGCATCTC / Celera SNP ID: hCV7593267 / Public SNP ID: rs1033797 /
SNP Chromosome Position: 33719687 / SNP in Genomic Sequence: SEQ ID NO: 366 /
SNP Position Genomic: 2356 / Related Interrogated SNP: hCV25620145 / SNP
Source: Applera / Population(Allele,Count): Caucasian (C,2|T,32) African
American (C,9|T,13) total (C,11|T,45) / SNP Type: INTRON / SNP Source:
dbSNP; Celera; HapMap; HGBASE / Population(Allele,Count): Caucasian (T,103|C,7)
/ SNP Type: INTRON /
Context (SEQ ID NO: 1329): /
ATTTTTCTCAGCAAAACAAAAGCCCTGTAGTTTTTCTTATTTCCTTAAACATGGTGTTTTATTTCATCCAAATGCAAGTGAAG
AGGAGATTAGATTTGGT
Y
TGTGGAATTCCAGGGAACAGATTAATGAATGAGAGGAAGTCAGAGAGAAATAAATGTTAGTTTAATGTAACTTTCTGACCCAG
CAATTCAATAATGGAAC / Celera SNP ID: hCV599565 / Public SNP ID: rs633198 / SNP
Chromosome Position: 33778434 / SNP in Genomic Sequence: SEQ ID NO: 366 / SNP
Position Genomic: 61103 / Related Interrogated SNP: hCV1825046 / SNP Source:
dbSNP; Celera; HapMap; HGBASE / Population(Allele,Count): Caucasian (T,66|C,54)
/ SNP Type: INTRON /
Context (SEQ ID NO: 1330): /
CCCAGTCCAGTTCTCTGCCCGCCCCACCCAAGTGGCACCCAAGTGCCTTCTGGTTCAAGACGAGAACTGGACAACCAGCCAGA
GGCAGAGATTTCCTCTC
Y
GGGCCTAAAGGTCAAACAACTCCAGAGCAGCCTATGCGTCACCAAACTGTGAACAAAGGTGTCGACACTTCCAAGAACCACAG
GGTCTAGCAACTGTGTC / Celera SNP ID: hCV624502 / Public SNP ID: rs666210 / SNP
Chromosome Position: 33723810 / SNP in Genomic Sequence: SEQ ID NO: 366 / SNP
Position Genomic: 6479 / Related Interrogated SNP: hCV1825046 / SNP Source:
dbSNP; HapMap; HGBASE / Population(Allele,Count): Caucasian (T,30|C,90) / SNP
Type: INTRON /
Context (SEQ ID NO: 1331): /
GGTAGGGCTGAGTATCTTTGATTCCAAAGCCCATGCTCATTCCATTTGTCACTCTGTCTGTCTCCCAGTCACCTGTCATACCT
GGGGGGAAGGGGTAATG
R
TGGTTATTACCTTTATTTTACTAATTTATCTATTTCAACTTATTATGCAATATGTAGAACTTTTTTTTTTTTTTTTTTTGAGACG
GAGTCTCGCTCTGTCAC / Celera SNP ID: hCV624503 / Public SNP ID: rs633784 / SNP
Chromosome Position: 33726323 / SNP in Genomic Sequence: SEQ ID NO: 366 / SNP
Position Genomic: 8992 / Related Interrogated SNP: hCV1825046 / SNP Source:
dbSNP; HapMap; HGBASE / Population(Allele,Count): Caucasian (A,30|G,90) / SNP
Type: TFBS SYNONYMOUS;INTRON /

Context          (SEQ ID NO: 1332): /
ACTGTATTTTTAATTCATTTTCTCTTATTTTTACTGTGGGGTTTCATATTACTTTAATAGCTGTTTATATTTGTAAGGTGCTT
TGCACTTCCCATATGCC
R
TGTATGTGGCCCTATTTGGTCCTGGGAACAATACCATGAGGTAGATAAGGAAGACATTGTCCTCATTTTATAGGTGAGTAAAA
GGAGACTCAAAGATGAT / Celera SNP ID:   hCV1271653 / Public SNP ID:    rs2425019 /
SNP Chromosome Position:   33819415 / SNP in Genomic Sequence:   SEQ ID NO: 366 /
SNP Position Genomic:   102084 / Related Interrogated SNP:   hCV1825046 / SNP
Source:   dbSNP; Celera; HapMap; HGBASE / Population(Allele,Count):   Caucasian
(A,128|G,98) / SNP Type:   INTRON /
Context          (SEQ ID NO: 1333): /
TTTCAGGTCCAGGTACCTCCCACCCCTTCAAGGGACTGTCTGATACTATTCTATGGCTCCTGGAATGAAGAACCAGTCATCAG
GACCTGCTGGCCTCTAT
K
AAATTAAGAGTCCAGTCAGATTTAACAACCTGAAGATGAGGTGTAACTGTGAAGCAATATATGGCTGATTTTTTAACACACCC
ATCCAAATACATCCACT / Celera SNP ID:   hCV1271661 / Public SNP ID:    rs6088765 /
SNP Chromosome Position:   33799280 / SNP in Genomic Sequence:   SEQ ID NO: 366 /
SNP Position Genomic:   81949 / Related Interrogated SNP:   hCV1825046 / SNP
Source:   dbSNP; Celera; HapMap; ABI_Val / Population(Allele,Count):   Caucasian
(T,132|G,94) / SNP Type:   TRANSCRIPTION FACTOR BINDING SITE;INTRON /
Context          (SEQ ID NO: 1334): /
CTCTCTACTGCAATCTGTTCCACAGTCGTATAGCCAAAATCCTCTTGCTCTGTGTCTGTTTATTCAACAAATATTTATCTTAA
CTGACTCTAATCACATT
S
TCTCCTGTTTGTCCTGCAGTGAAGCCCTTTTACATCTGCTTATCTCTTTTGATCCAAGGGAAGGAGACTTAACCCTGACTTCC
AGAGAAGGAAATAGTCT / Celera SNP ID:   hCV1271665 / Public SNP ID:    rs17092456 /
SNP Chromosome Position:   33796192 / SNP in Genomic Sequence:   SEQ ID NO: 366 /
SNP Position Genomic:   78861 / Related Interrogated SNP:   hCV25620145 / SNP
Source:   dbSNP; Celera; HapMap / Population(Allele,Count):   Caucasian
(G,204|C,22) / SNP Type:   INTRON /
Context          (SEQ ID NO: 1335): /
GTTATTAGACATAATGTTATTGCACATTTATTAGACTATAATATAGTATAAACATAACTTTTATTTATTATTGTTTTTTCTTTT
TAGACGAACTCTTGGGC
R
TGATAGTGCGCGCCTGTAATCTCAGCTATTCAGGAGGCTGAGGCAGGAGAATTGCTTGAATCCAGGAGGTGGAGGTTGCAGTG
AGCTGAGATCGCACCAC / Celera SNP ID:   hCV1271671 / Public SNP ID:    rs2093058 /
SNP Chromosome Position:   33791281 / SNP in Genomic Sequence:   SEQ ID NO: 366 /
SNP Position Genomic:   73950 / Related Interrogated SNP:   hCV1825046 / SNP
Source:   dbSNP; Celera; HapMap / Population(Allele,Count):   Caucasian
(G,122|A,104) / SNP Type:   INTRON /
Context          (SEQ ID NO: 1336): /
ACCCCAAAAGGATAAAAGCAATGAAAACCCCACCCACCCTAATCAAACTTCTGAAAACCAAATATTAGGAAAAAAATTTTAAG
AGTAGCCACCAAAAAAT
S
ACATGTTACATATGGGGGAACAATAATTCTTATGACTGTAGATTTTTCATCAGAGACCATGGAAGCCAGAAGACAATGGAATA
ACACCTTTAAAGTGCTG / Celera SNP ID:   hCV1271676 / Public SNP ID:    rs1577924 /
SNP Chromosome Position:   33784288 / SNP in Genomic Sequence:   SEQ ID NO: 366 /
SNP Position Genomic:   66957 / Related Interrogated SNP:   hCV1825046 / SNP
Source:   dbSNP; Celera; HGBASE / Population(Allele,Count):   Caucasian
(C,122|G,104) / SNP Type:   INTRON /
Context          (SEQ ID NO: 1337): /
CTGTTTTAGGACGTGGGAATAGAGCAATATAGCAGAGAACAAAAACAAAGTCTGTCTTAGAAGCTACAGTTTACTGGGAGGGA
CAGACCCTAAACAAATA
W
TTTTTTTTTTAATGTCAGAGATAATTAGTAGGAAATAGGTAGAAAAATAAGGAAGGTCGAAGGGAGAATAACTGAGTTGCTGTT
TTAGACAGCATAATCAG / Celera SNP ID:   hCV1271685 / Public SNP ID:    rs663550 /
SNP Chromosome Position:   33779268 / SNP in Genomic Sequence:   SEQ ID NO: 366 /
SNP Position Genomic:   61937 / Related Interrogated SNP:   hCV1825046 / SNP
Source:   dbSNP; Celera; HGBASE / Population(Allele,Count):   Caucasian
(A,64|T,52) / SNP Type:   INTRON /
Context          (SEQ ID NO: 1338): /
GATGTTATTATTCTCATTTCCTGGCAACGTTATTATTCTCACACCCAGTAAAAAGAAACCAAAACTCTATTTCTTCTAATATC
TCTTGGTATATCCGATG

R
TCTGTTGTATGTGTACTGGCATATAACAGGGTTCTCATGGGTAGCCTTAATCTTGCATCACACTCACTATAATCAGGATTTCT
ACATATCCCAAAAAATA / Celera SNP ID: hCV1271688 / Public SNP ID: rs6058202 /
SNP Chromosome Position: 33777983 / SNP in Genomic Sequence: SEQ ID NO: 366 /
SNP Position Genomic: 60652 / Related Interrogated SNP: hCV1825046 / SNP
Source: dbSNP; Celera; HapMap; ABI_Val / Population(Allele,Count): Caucasian
(A,103|G,121) / SNP Type: INTRON /
Context (SEQ ID NO: 1339): /
GAAGTGAGGCAGGGCAAGGGAGGCGGCATTTAAGGGGTATGCCATCAGGCAGGTTTCCACCATGGGAACTGGAGTTCAATCTT
GCTGGGGAGCTTAGGAA
Y
TCAGTGTAGAGCTCCAGCTTCGGAGTGATCCCACCCAAGGGGCAGGGAACTGGAATGTTTATCCACCAACTCCCATCAGTATC
AGTTACCGGCTTGAGAG / Celera SNP ID: hCV1348030 / Public SNP ID: rs11908683 /
SNP Chromosome Position: 33720920 / SNP in Genomic Sequence: SEQ ID NO: 366 /
SNP Position Genomic: 3589 / Related Interrogated SNP: hCV25620145 / SNP
Source: dbSNP; Celera; HapMap / Population(Allele,Count): Caucasian
(T,110|C,10) / SNP Type: INTRON /
Context (SEQ ID NO: 1340): /
GACAAATTATGATCCCGGACAGGAGCAGGGGGATAGGGATAGTTCTGATACACGCCCAAAGCCTGGGACCTTAGCCAGCACTT
CCCTCTTTCTCCTGGGT
R
TCCTGCTAGAGTCTGAGCCAGAGAAAGATAAATGTCATAACTGGAGGGCCCTGAGCAGCCACCCAGCCCAGATGCTGTCAAAC
ACTGCTCTGCATAACCT / Celera SNP ID: hCV1348034 / Public SNP ID: rs2295888 /
SNP Chromosome Position: 33722863 / SNP in Genomic Sequence: SEQ ID NO: 366 /
SNP Position Genomic: 5532 / Related Interrogated SNP: hCV25620145 / SNP
Source: dbSNP; Celera; HapMap; ABI_Val; HGBASE / Population(Allele,Count):
Caucasian (A,110|G,10) / SNP Type: INTRON;PSEUDOGENE /
Context (SEQ ID NO: 1341): /
GGGCTGAGCTCAGCGATCCAGCCCACCCGGCTAATCCAGGACTTTTTTTTTCACTGCGAGAGGCTAAGTGGATCCTGGAGGTG
ATAGTAAGAATTGCGGG
R
AGCGCGGCTCTAGCTCAGCTGGGAGCTTTCTGGTGCCGGTGAGGAGACCATGAAGCCACCAGCCCCAGCTGACCCGCCCGCCG
AGGCCCAGAGAGTCAGA / Celera SNP ID: hCV1825004 / Public SNP ID: rs1415771 /
SNP Chromosome Position: 33734493 / SNP in Genomic Sequence: SEQ ID NO: 366 /
SNP Position Genomic: 17162 / Related Interrogated SNP: hCV1825046 / SNP
Source: dbSNP; Celera; HapMap; ABI_Val; HGBASE / Population(Allele,Count):
Caucasian (G,118|A,100) / SNP Type: INTRON /
Context (SEQ ID NO: 1342): /
TCTCCATTCCCCTTATTCCCAAGTTTCTAAAATTTGGTCAGCACCTTAGACCACAGCAACACCCATGACTTTGGTCAGTGTGA
CATGCGGTAGAACCAAC
M
CTGGATTTGAAGTCAGATCTAGGATCCAGTTAATTACAACTCTGTCACTCATCTATAACCTGTGTATGTGACCCTCTCCTTCT
CTGGGGCTAGCATCCTT / Celera SNP ID: hCV1825006 / Public SNP ID: rs1124511 /
SNP Chromosome Position: 33736697 / SNP in Genomic Sequence: SEQ ID NO: 366 /
SNP Position Genomic: 19366 / Related Interrogated SNP: hCV1825046 / SNP
Source: dbSNP; Celera; HapMap; HGBASE / Population(Allele,Count): Caucasian
(A,64|C,56) / SNP Type: INTRON /
Context (SEQ ID NO: 1343): /
GGCTGGCGGGGAGAGGGGTGGTTGGTAAATGGGAAAAGGGTGCTTCCTTTGCTGATATCCAAAGCAGATACTTTCTCTGCCTA
TTTGATGATACAAAACT
M
GCCCTACCACACCAGGCTAAGGGAGAGAAGAGGATCCGTGTCCTACAGAATTCCCCCTATTAGCCTACCTGCCCCATAGCCCC
CAAACTCTGCTGCTAAT / Celera SNP ID: hCV1825018 / Public SNP ID: rs11696967 /
SNP Chromosome Position: 33743609 / SNP in Genomic Sequence: SEQ ID NO: 366 /
SNP Position Genomic: 26278 / Related Interrogated SNP: hCV1825046 / SNP
Source: dbSNP; Celera; HapMap / Population(Allele,Count): Caucasian
(A,179|C,47) / SNP Type: INTRON /
Context (SEQ ID NO: 1344): /
CTGCCTGGTAGAGAAGATAATCCCAAAGGTTATGATATTATTACTCTGTAGGACATTAACCCATTTAGTATTTAATTTAGCAA
TCTTACTTCAATATTTT
Y
CTTTTCCATTGACTATAAAAACTCAAGTTCTTTTTTTCTTTTTTTTTGAGACAGGTCTCACATTGTTGCCCAGGCTGGAGTGCAG
TGGTTCGAACACGGCTC / Celera SNP ID: hCV1825019 / Public SNP ID: rs6088732 /

259

SNP Chromosome Position: 33744134 / SNP in Genomic Sequence: SEQ ID NO: 366 / SNP Position Genomic: 26803 / Related Interrogated SNP: hCV1825046 / SNP Source: dbSNP; Celera; HapMap / Population(Allele,Count): Caucasian (C,177|T,47) / SNP Type: INTRON / Context (SEQ ID NO: 1345): /
GACTGGTCTTGAACTCCTGACTTCAAGTGATCCATCCACTTTGGCCTCCCAAAGTGCCGGGATTTACAGGAGTGAGCCACTGC GCCTGGCCAAGAATTAC
R
TTTTTAACATGCTGACAGTTGTCCTTGACACTAAGTGTCAGACCCTGGATTAAGTAATTTATAGACCCATGATACTCCTTTGA GATAGTTTTTGTCCCCA / Celera SNP ID: hCV1825021 / Public SNP ID: rs6088733 / SNP Chromosome Position: 33744904 / SNP in Genomic Sequence: SEQ ID NO: 366 / SNP Position Genomic: 27573 / Related Interrogated SNP: hCV1825046 / SNP Source: dbSNP; Celera; HapMap; ABI_Val / Population(Allele,Count): Caucasian (A,95|G,25) / SNP Type: INTRON / Context (SEQ ID NO: 1346): /
CAAAACTTCATCAACGTGATAAAAAAGATTCCAAAATTTAGGTGTAGACGTTCAAATATTCAAAAATAGCTAAGACACTCTTG AAGAACAAAGAGGAAAG
R
CTTGATCTACAAGATATAAATACATTATGAAATAATACTAATCGAGGGCCAGGCACTGTGGCTCACGCCTGTAATCCCAGCAC TTTGGAAGGCCAAGGCA / Celera SNP ID: hCV1825025 / Public SNP ID: rs6088738 / SNP Chromosome Position: 33747331 / SNP in Genomic Sequence: SEQ ID NO: 366 / SNP Position Genomic: 30000 / Related Interrogated SNP: hCV1825046 / SNP Source: dbSNP; Celera; HapMap / Population(Allele,Count): Caucasian (G,177|A,49) / SNP Type: INTRON / Context (SEQ ID NO: 1347): /
TCAGGAAACCAGGAGGAATGGATTTGGCTGAAACTGGAATACATGGATAGATAAGGCTAGAGAGATAGGCAGGAGCCAAGCCA ACAATACTAGGCTCCCA
Y
CAATTGCTGGGGCAGGCAAGAGCAGGCATCATCCTGAGTGTTTGGAGCTTCTTTTCATGTTCCCTAAGTTGGGGGGTGTTGGG GGGATGATACAGGGGGA / Celera SNP ID: hCV1825040 / Public SNP ID: rs6060278 / SNP Chromosome Position: 33753262 / SNP in Genomic Sequence: SEQ ID NO: 366 / SNP Position Genomic: 35931 / Related Interrogated SNP: hCV1825046 / SNP Source: dbSNP; Celera; HapMap; ABI_Val / Population(Allele,Count): Caucasian (T,179|C,47) / SNP Type: INTRON / Context (SEQ ID NO: 1348): /
AAGGAGCCCAGTCTTTTCTTGGGAAGATAAAACATACAAAAGAGAACAACACAAGGCAAGATGCATTGTATAACTAGAAGAAA CACACAAATAAAAATGT
K
ATGTTATGTGGCCGGGCATGGTGGCTCACGCCTGTAATCCCAGCACTTTGGGAGGCTGAGGTGGGCAGTTCACCTGAGGTCAG GTGTTCGAGACCAGCCT / Celera SNP ID: hCV1825056 / Public SNP ID: rs6060285 / SNP Chromosome Position: 33770487 / SNP in Genomic Sequence: SEQ ID NO: 366 / SNP Position Genomic: 53156 / Related Interrogated SNP: hCV1825046 / SNP Source: dbSNP; Celera; HapMap; ABI_Val / Population(Allele,Count): Caucasian (G,103|T,123) / SNP Type: INTRON / Context (SEQ ID NO: 1349): /
TTTGTACTCTTTCCCTTTATTTCTCAGACCAGCCAACACTTAGGGAAAATAGAAAAGAACCCACGTTGAAATATCGGGGGCTG GTTCCCCCAATACCCTT
S
TTCATCTTTGTATCCTTAGTATCTACAACAGTGCCAGGAATTTAGAAGTTAGTCAATAAATGGCTGGATGAATGAATGAATTG AAGAGTTTTGAGGAAGG / Celera SNP ID: hCV1825062 / Public SNP ID: rs6087685 / SNP Chromosome Position: 33777612 / SNP in Genomic Sequence: SEQ ID NO: 366 / SNP Position Genomic: 60281 / Related Interrogated SNP: hCV1825046 / Related Interrogated SNP: hCV25620145 / SNP Source: dbSNP; Celera; HapMap / Population(Allele,Count): Caucasian (G,93|C,27) / SNP Type: INTRON / Context (SEQ ID NO: 1350): /
AGCCACTGCGCCTGGTCTTCTCACTCATTCTTAGACCCAGTGCAATCTGACTTCTCTATAAACTACTCTGAGATCACCAGTAA CCTCTAATTGTCAAACC
R
TCACCCTACATGGTATCTGCAAATTTGCGGACTAGAACTCTCTTTTTGCCTTAACTTCTGAGATACCATACTTCAATTTTTAA AACTGTTCTGTCTACTT / Celera SNP ID: hCV7499886 / Public SNP ID: rs1415774 / SNP Chromosome Position: 33765616 / SNP in Genomic Sequence: SEQ ID NO: 366 / SNP Position Genomic: 48285 / Related Interrogated SNP: hCV1825046 / SNP Source: dbSNP; HGBASE / Population(Allele,Count): Caucasian (A,104|G,122) /

SNP Type:    INTRON /
Context              (SEQ ID NO: 1351): /
CCCAGCATGTGATTCCACTATCTGAAGACACAGACGTGCTTTACGTATTTCCATAAATTAACTCAATAAGAACATCCACCAAG
AAGCTGACAGAGTGGTT
M
TAAGGAGAGAAACCGAATAGCTGGAGACAGGGGCAAAAGGGGACTTCACCAATGTCACTGAGTACCCTTTTTTGTATCCTTTG
ACTTTTTTTTTTTTTAAT / Celera SNP ID:   hCV7593265 / Public SNP ID:    rs1033799 /
SNP Chromosome Position:   33720033 /  SNP in Genomic Sequence:   SEQ ID NO: 366 /
SNP Position Genomic:   2702 /  Related Interrogated SNP:   hCV25620145 /  SNP
Source:   dbSNP; HapMap; HGBASE /  Population(Allele,Count):   Caucasian
(C,202|A,24) /  SNP Type:    INTRON /
Context              (SEQ ID NO: 1352): /
ATTGCTAGTGTCTAAGGGCCTGGCGGGTAAGATGTATTTATTCAACAGGTATTAATCTATTACCTACCACATGTAAAGCACTG
TGGGGAACATAAGATGA
R
TAAGGCATGGATTCTGCATTCAATTTGTAGAGCTGTAACTGTTGATGAGAGCTAACTGCAAGAAAGATGCTGAAGGTCCCAAA
TTCTGTAGAAGGATCAA / Celera SNP ID:   hCV7593276 / Public SNP ID:    rs1535466 /
SNP Chromosome Position:   33718706 /  SNP in Genomic Sequence:   SEQ ID NO: 366 /
SNP Position Genomic:   1375 /  Related Interrogated SNP:   hCV1825046 /  SNP
Source:   dbSNP; HapMap; ABI_Val; HGBASE /  Population(Allele,Count):   Caucasian
(G,62|A,164) /  SNP Type:   TRANSCRIPTION FACTOR BINDING SITE;INTRON /
Context              (SEQ ID NO: 1353): /
TTAACCTTTATTTACTCCCTCTTATTTATCCCCATCTACCAACTACAGTATCTATAAAACACATTCACTCCTTTCTACCCCAT
TGCTTTTGTTCAAGATA
Y
CATAATTCCTCTCTTTCTTTGTGGTAAACATGAAGGTGTACCATCCAGTTCTACCTCCAAAGAAAGACTTCCTGTTGGCCGGG
CATGATGGTTCACGCTT / Celera SNP ID:   hCV11189130 / Public SNP ID:    rs2065979 /
SNP Chromosome Position:   33767690 /  SNP in Genomic Sequence:   SEQ ID NO: 366 /
SNP Position Genomic:   50359 /  Related Interrogated SNP:   hCV1825046 /  SNP
Source:   dbSNP; HapMap; HGBASE /  Population(Allele,Count):   Caucasian
(T,54|C,66) /  SNP Type:    INTRON /
Context              (SEQ ID NO: 1354): /
ATCTAGGATCCAGTTAATTACAACTCTGTCACTCATCTATAACCTGTGTATGTGACCCTCTCCTTCTCTGGGGCTAGCATCCT
TGTCTATAAATAATGAG
K
AGTTTGAACAAATGGATCTTTAAGATCCTTTTCGGTCGAGCTTTCTATGATTCTAGGATTGGCTATCTCTTATTTGCTGAAAA
AGTCAGATGGAAGTGAT / Celera SNP ID:   hCV11189159 / Public SNP ID:    rs6060270 /
SNP Chromosome Position:   33736814 /  SNP in Genomic Sequence:   SEQ ID NO: 366 /
SNP Position Genomic:   19483 /  Related Interrogated SNP:   hCV1825046 /  SNP
Source:   dbSNP; Celera; HapMap /  Population(Allele,Count):   Caucasian
(G,179|T,47) /  SNP Type:    INTRON /
Context              (SEQ ID NO: 1355): /
GCCCTCACTAATTCCACTTAAGACCTCCAGTTTCTGTCCTAGGATTGGTGACAGCAGAAGAAAACACTGAGAGCTAGAAATCA
TCTAGAGACTTCATACC
R
AGAGACTGGTTAAGTAAATTCCTAGGTCCCAATTAAGAGTCACGATGTTGTCATTTAAATGGACACAATATAAAGAACGCAAA
CTACGCATAGAAGTCTT / Celera SNP ID:   hCV11189164 / Public SNP ID:    rs3746427 /
SNP Chromosome Position:   33730464 /  SNP in Genomic Sequence:   SEQ ID NO: 366 /
SNP Position Genomic:   13133 /  Related Interrogated SNP:   hCV1825046 /  SNP
Source:   dbSNP; HapMap /  Population(Allele,Count):   Caucasian (A,103|G,123) /
SNP Type:   INTRON /
Context              (SEQ ID NO: 1356): /
CTTTTATGGATCATATGTCTGGAATATAAGTTTCATTGCAGAAAACATACAAAATAATGGGGAAAAATAGAACTCTGCCATCC
AGAGGTGACAGCTGTTA
M
GTGGTCTAGAGGTTCGATGCAAATAAGGGCTGTGCTGGCTTCACAAGAATCACTGGAAGTCTGCTAAAAATACTGATTCTAGG
ACCCAACACAGAGATTC / Celera SNP ID:   hCV11189166 / Public SNP ID:    rs11906318 /
SNP Chromosome Position:   33729442 /  SNP in Genomic Sequence:   SEQ ID NO: 366 /
SNP Position Genomic:   12111 /  Related Interrogated SNP:   hCV25620145 /  SNP
Source:   dbSNP; Celera; HapMap /  Population(Allele,Count):   Caucasian
(A,204|C,22) /  SNP Type:    INTRON /
Context              (SEQ ID NO: 1357): /
GGGAGGCAGTATAAGATTGCAGCTTAAAGCACAGGCCTTTCACCAATTTTCACCTCTAGGAATTTAATTCACAGTGAACAGCT

GTTTGAGAGTGTTCATG
R
CAGTATTGCTTAGAACAGCAAGAGGCTGGAAAGAACCCAACTGTCCACCCATTAATGGGTGATTAAACTGTGTTATAAACAGT
GCAAAACTATGAGTTAT / Celera SNP ID: hCV11656971 / Public SNP ID: rs2050652 /
SNP Chromosome Position: 33733180 / SNP in Genomic Sequence: SEQ ID NO: 366 /
SNP Position Genomic: 15849 / Related Interrogated SNP: hCV1825046 / SNP
Source: dbSNP / Population(Allele,Count): Caucasian (G,170|A,54) / SNP Type:
INTRON /
Context (SEQ ID NO: 1358): /
TAGTTTATGAAGGGTCGAGAAGGCGGGCGGCCAGCCTCGAGGTAGGGGGTTATTATCTTCCGCTGCCCGCCGCCCCCTCCCAC
GCCGGCCCAGGCTGAAG
Y
TGACTCTGCCCGCAGGCCTCCAAAGACTTCATATGCTCCAGATCTCCTACTTCCGCGACCCCTATCACGTGTGGTACCAGGGC
AACGCGTCGCTGGGGGG / Celera SNP ID: hCV15860322 / Public SNP ID: rs2069948 /
SNP Chromosome Position: 33762489 / SNP in Genomic Sequence: SEQ ID NO: 366 /
SNP Position Genomic: 45158 / Related Interrogated SNP: hCV1825046 / SNP
Source: dbSNP; Celera; HGBASE / Population(Allele,Count): Caucasian
(C,104|T,122) / SNP Type: MISSENSE MUTATION;ESE;TRANSCRIPTION FACTOR BINDING
SITE;INTRON /
Context (SEQ ID NO: 1359): /
AATCCATGGGTCAGAGAAAAAATCTTAAAGTAAATCAGAAAATATTTGAACTGACTGAACATCAAAACAAAACATATCAAAAT
TTATGGGTTGCAGCTAA
Y
GCAGCGTGTACAGGAACATTTATAGCTTTACATGCCTATGATAGAAAAAAGAAAGGCCTCAAATCATACTCTAAGCTTCTACC
TTAAGAAACTAGAGAAA / Celera SNP ID: hCV15870054 / Public SNP ID: rs2224320 /
SNP Chromosome Position: 33791590 / SNP in Genomic Sequence: SEQ ID NO: 366 /
SNP Position Genomic: 74259 / Related Interrogated SNP: hCV1825046 / SNP
Source: dbSNP; HGBASE / Population(Allele,Count): Caucasian (T,63|C,163) /
SNP Type: INTRON /
Context (SEQ ID NO: 1360): /
AGTCCTTCGCCATTATTGAATTGGGTCATAGGTCTTTTTCTTATTGGTTTATAACAGCTGTTTTACGTTAAAAAAAATAATAAT
AAATATTTTACCTAGTT
Y
ATAGTTTGTTGCTTTATTTCTGCTTTTTTTTTTGGCCATGCAGATGAAGGCCTGAATTTTTGAAACCTAAGTGAACTGTTACTG
CTTTCACTGTATACCAG / Celera SNP ID: hCV27167646 / Public SNP ID: rs11699306 /
SNP Chromosome Position: 33801283 / SNP in Genomic Sequence: SEQ ID NO: 366 /
SNP Position Genomic: 83952 / Related Interrogated SNP: hCV25620145 / SNP
Source: dbSNP; Celera; HapMap / Population(Allele,Count): Caucasian
(T,201|C,25) / SNP Type: INTRON /
Context (SEQ ID NO: 1361): /
CCAACTAAACAAGGAAGAGGAGGGGGATGGGGAGGGGGACAAGAAGAAGAAGGAGAAAATAGTCACGTGTATTGATAATAGCG
AACATTTACATAGCAAG
W
AATGTTCTTTGTACTTTACATCCATCAACACCATTAATCCTTACCACAACCCTCTGAGAAAGTGTCATTATCACCATTTTATA
AACCAAAAACCTGAAGC / Celera SNP ID: hCV27833500 / Public SNP ID: rs17092385 /
SNP Chromosome Position: 33739254 / SNP in Genomic Sequence: SEQ ID NO: 366 /
SNP Position Genomic: 21923 / Related Interrogated SNP: hCV1825046 / SNP
Source: Applera / Population(Allele,Count): Caucasian (A,6|T,32) African
American (A,4|T,30) total (A,10|T,62) / SNP Type: INTRON / SNP Source:
dbSNP; HapMap / Population(Allele,Count): Caucasian (T,107|A,13) / SNP Type:
INTRON /
Context (SEQ ID NO: 1362): /
TCCCTCACCCACTGGGGCTGCACCAGCACTGGCCCCAGCTCAGCTTCCTGTCTCCCTTATCTTCCTCCAGCATGTGCTCTCTG
GAGTAGGAAACACCACC
R
CTTGCTTCCTTCATTTCCCTTTGCTCAACGGGACCCTCATTCTTTAGGTCTCAGTCAAATCCCAGCTGCTGGCCTGGTGCCCA
CTCTGTGATTTTCTACA / Celera SNP ID: hCV29373046 / Public SNP ID: rs8119351 /
SNP Chromosome Position: 33754405 / SNP in Genomic Sequence: SEQ ID NO: 366 /
SNP Position Genomic: 37074 / Related Interrogated SNP: hCV25620145 / SNP
Source: dbSNP; HapMap / Population(Allele,Count): Caucasian (G,204|A,22) /
SNP Type: INTRON /
Context (SEQ ID NO: 1363): /
AGTGATCCACCTACCTGGGCCTGCCAAAGTGCTGGGATTACAGGCATGAGCCACCACACCTGGCCTGAACACGCTTATACTAA

TGGTGAAATAATGTTGG
R
TAGCTGTAAGTGGCTTGCCCAAAGTTCCACAGTGATGTGGTACAAGAACTGGCACTAGAGCTTGGGACTTCTGACTTCTGATC
CTGAGCTATTGCTGCTA / Celera SNP ID: hCV32066684 / Public SNP ID: rs11167260 /
SNP Chromosome Position: 33775200 / SNP in Genomic Sequence: SEQ ID NO: 366 /
SNP Position Genomic: 57869 / Related Interrogated SNP: hCV25620145 / SNP
Source: dbSNP; HapMap / Population(Allele,Count): Caucasian (G,204|A,22) /
SNP Type: INTRON /
Context (SEQ ID NO: 1364): /
CTAAGGGATAATCCGTTCCTTGCCTCTCTAGATTCTCCTGACTGCTGGCATTCCTTGGCTTATGGTTGTATCACTCCAGTATC
TGCCCGTGCCTTCATAT
Y
GCCTTCTCTGTGGATGTGTCACATCTCCTTCTGCTTTTCTCTTATAAGGACATTTATAATTGCATTTAGGACCCCCCCCGCGC
CCCCCCCAACCCAGATA / Celera SNP ID: hCV32066710 / Public SNP ID: rs11907010 /
SNP Chromosome Position: 33737661 / SNP in Genomic Sequence: SEQ ID NO: 366 /
SNP Position Genomic: 20330 / Related Interrogated SNP: hCV25620145 / SNP
Source: dbSNP; HapMap / Population(Allele,Count): Caucasian (C,204|T,22) /
SNP Type: INTRON /
Context (SEQ ID NO: 1365): /
AGAGTGGCATGGGGTAGATTCAAACAGCCTAGCAAAGACTCTGAAAATGAACTGACACTGCAACCACCCACAGCAGGTGAGAC
AAAGCTTGTAGTCTGAA
M
CTAATTGAGTTATTTGCCTGCTAAAAATAAACTAGTCACCATCCAGATAATTTAAACAGGACTCAGAGTCCCACAATCTAACAC
TCAAGATGGGCAAGATA / Celera SNP ID: hCV30450320 / Public SNP ID: rs4911478 /
SNP Chromosome Position: 33782625 / SNP in Genomic Sequence: SEQ ID NO: 366 /
SNP Position Genomic: 65294 / Related Interrogated SNP: hCV1825046 / SNP
Source: dbSNP; HapMap; HGBASE / Population(Allele,Count): Caucasian
(A,66|C,50) / SNP Type: INTRON /
Context (SEQ ID NO: 1366): /
CTTGTCTAATTTTTGAAGCTAGGCAGGGTCAGGCCTGGTTAGGATTTGGAGGGAGACCACCTGGGAATACTGGGTAATGTAGG
CTGTCAATTAAAAAAAT
K
TTTTTAAACAATCAGACACCTACTATGTACCGATAAAATTTTGTAATAAAAAAAATTCAAAAAGTAAACGAGAATAAATAAGAA
TAAAGAATGTGATGTTG / Celera SNP ID: hCV30270039 / Public SNP ID: rs6060301 /
SNP Chromosome Position: 33783242 / SNP in Genomic Sequence: SEQ ID NO: 366 /
SNP Position Genomic: 65911 / Related Interrogated SNP: hCV1825046 / SNP
Source: dbSNP; HapMap; ABI_Val / Population(Allele,Count): Caucasian
(G,60|T,162) / SNP Type: INTRON /
Context (SEQ ID NO: 1367): /
TGACTCCTGCTTACCTCCTCATATCACTTCTTCCTTCCTATCACCTACTCCTATTTGGCCATACTTAAGTATTACTGCTTTCT
CAAGTATGTTATATTCT
Y
TCTCCTTTCCTATTATATTCTTTTGTACCTTCTTCACCCTGTACTTGAAACAACTTTTTCCTCACCCCTACTTGCCCCTTAGG
CATTATCGGGGTTTTTT / Celera SNP ID: hCV30468101 / Public SNP ID: rs6088735 /
SNP Chromosome Position: 33745676 / SNP in Genomic Sequence: SEQ ID NO: 366 /
SNP Position Genomic: 28345 / Related Interrogated SNP: hCV1825046 / SNP
Source: dbSNP; HapMap / Population(Allele,Count): Caucasian (C,179|T,47) /
SNP Type: INTRON /
Context (SEQ ID NO: 1368): /
CACCTCAGCCACCCAGGCCAGATGCAGTGTAGGCCATGGCTCCAACAGCAGCATTTCTCTTTTCTTCCCCACCCCCATCCCTG
GGTTTAGAATGAGAAAA
K
GCTCAAGTTGCCCCCCTCCCAACTTCCCTACCCGCAACACTCTTTTTATTTTCTCTGTTCCCAGCTCCATACTTCCATGCTTT
TTTTTTTTTTTTTTTTT / Celera SNP ID: hCV29530377 / Public SNP ID: rs6088747 /
SNP Chromosome Position: 33754604 / SNP in Genomic Sequence: SEQ ID NO: 366 /
SNP Position Genomic: 37273 / Related Interrogated SNP: hCV1825046 / SNP
Source: dbSNP; HapMap / Population(Allele,Count): Caucasian (G,102|T,122) /
SNP Type: INTRON /
Context (SEQ ID NO: 1369): /
GAGCAAGACTCCATCTCAATAATAATAATAATAATAAATAAATGAGCATAATTCACCATATTGGCAGACTAAGGAAGAAAAAA
ACACATGATAATCCTAA
M
TGATGAAGAAAATGCATTAACAAAATTTAAAACCCATTCATGATAAAAAAAAAAAAAAATCTCTCAGCAAACTAGAGAAGGAAAC

EP 2 635 709 B1

TTCGTTAATCCAAAAAT / Celera SNP ID: hCV30198062 / Public SNP ID: rs6088764 / SNP Chromosome Position: 33793159 / SNP in Genomic Sequence: SEQ ID NO: 366 / SNP Position Genomic: 75828 / Related Interrogated SNP: hCV1825046 / SNP Source: dbSNP / Population(Allele,Count): Caucasian (C,166|A,58) / SNP Type: INTRON /
Context (SEQ ID NO: 1370): /
TGTGTATTATTATCCCTAGATCAACAGCTAAAAAAAAAAAAACCACAAAAGATATAGACAACCAATATATTAAATTGGAATATA
AAACATATTCAAATAAT
Y
CAAAATATAACAGGAAAGGGGACATTGAGAGGCAAAAAAATGGAGGAGACAAGCAGAAAAATAACAAATTGTAGACCAAAATC
CACTCTTATCAATAACT / Celera SNP ID: hCV29909897 / Public SNP ID: rs6142324 / SNP Chromosome Position: 33789043 / SNP in Genomic Sequence: SEQ ID NO: 366 / SNP Position Genomic: 71712 / Related Interrogated SNP: hCV1825046 / SNP Source: dbSNP; HapMap / Population(Allele,Count): Caucasian (C,122|T,104) / SNP Type: INTRON /
Context (SEQ ID NO: 1371): /
CCGTCAGTTCTAGGAATTCATGACACTTCTCTCCTCCCAATTTGGGATGTGGTTACACAATCATGGTCCCTTCAGTCTCCATC
TCAAACCGGAAGCTTTA
Y
CCCTGTCAGCCCTGTAGAATGTCTTGGGTAAGAATGAATACCCTGGTTGGGCGCGGTGGCTCACGCCTGTAATCCCAACACTT
TGGGAGGTTGAGGTGCG / Celera SNP ID: hDV70862720 / Public SNP ID: rs17092378 / SNP Chromosome Position: 33736188 / SNP in Genomic Sequence: SEQ ID NO: 366 / SNP Position Genomic: 18857 / Related Interrogated SNP: hCV1825046 / SNP Source: dbSNP; HapMap / Population(Allele,Count): Caucasian (C,172|T,54) / SNP Type: MISSENSE MUTATION;INTRON /
Context (SEQ ID NO: 1372): /
TGGGGCCAGTGGGCCCTAATGAGATTGCACCTCCATTTTAAAAGCGTGAAAGGTATAAGGAGATTCAGAGATAGGTGTAAATG
GCTTGCGGGAGCCTCCA
M
AATTTTCAGGTCCAGGTACCTCCCACCCCTTCAAGGGACTGTCTGATACTATTCTATGGCTCCTGGAATGAAGAACCAGTCAT
CAGGACCTGCTGGCCTC / Celera SNP ID: hDV70949473 / Public SNP ID: rs17406518 / SNP Chromosome Position: 33799176 / SNP in Genomic Sequence: SEQ ID NO: 366 / SNP Position Genomic: 81845 / Related Interrogated SNP: hCV25620145 / SNP Source: dbSNP; HapMap / Population(Allele,Count): Caucasian (A,201|C,25) / SNP Type: TRANSCRIPTION FACTOR BINDING SITE;INTRON /
Context (SEQ ID NO: 1373): /
CAGGTATTCTCTGACCCACTTAATGCACCTGGGAGGGTTGGCTGATGTAGCTCAATTGTAATTAAATACATCACCTATAGTCT
GGCATTCTCCTCTACCA
Y
TGGGAGGCAGTATAAGATTGCAGCTTAAAGCACAGGCCTTTCACCAATTTTCACCTCTAGGAATTTAATTCACAGTGAACAGC
TGTTTGAGAGTGTTCAT / Celera SNP ID: hCV29674976 / Public SNP ID: rs6060266 / SNP Chromosome Position: 33733078 / SNP in Genomic Sequence: SEQ ID NO: 366 / SNP Position Genomic: 15747 / Related Interrogated SNP: hCV1825046 / SNP Source: dbSNP; HapMap / Population(Allele,Count): Caucasian (T,86|C,16) / SNP Type: INTRON /
Context (SEQ ID NO: 1374): /
TAGGCAAAGAAGATGCCATGTGCAATGGCCAGGAGATAAGAAATCCAGGGCTACATACACAGAATGCAGCACGCTTCAGTTGG
CTTTTGCAAGAAGGCAT
S
AAGTGCTGTAGTGGGCATGAAGTGGGCAGTGTAAGTCCATAGTGACTCTGAATTCAGAACTGATTCTGCAGGCACTCAGGCCA
GGCGTGGTGGCTCACAC / Celera SNP ID: hCV30576442 / Public SNP ID: rs6120843 / SNP Chromosome Position: 33718993 / SNP in Genomic Sequence: SEQ ID NO: 366 / SNP Position Genomic: 1662 / Related Interrogated SNP: hCV25620145 / SNP Source: dbSNP; HapMap / Population(Allele,Count): Caucasian (C,202|G,24) / SNP Type: INTRON /
Context (SEQ ID NO: 1375): /
ATCTGAATGACAAGCAGCAGCCAGTGATGGGAGGAAAATGGGGAGTGTATTTCAGGCACGGGGCACACCAACTACAAAGACTT
AAGTTTTAGGAGTGTCA
S
ACTCCTTCCTGGATCTGAATGCCCACCTACATAATGAAAGGGGTGGGTTTTCTAATTCTTGGAGCCCTTCCACGGTATCTCGT
TACTCCAGAAATTGCCT / Celera SNP ID: hCV29693169 / Public SNP ID: rs6058192 / SNP Chromosome Position: 33734261 / SNP in Genomic Sequence: SEQ ID NO: 366 / SNP Position Genomic: 16930 / Related Interrogated SNP: hCV1825046 / SNP

264

Source: dbSNP / Population(Allele,Count): Caucasian (C,86|G,30) / SNP Type: INTRON /
Context (SEQ ID NO: 1376): /
GGGCAACAAGAGTGAAACTCCATCTCAAAAAAAAAAAAAAAAAAAAATGATAAGTAAAAGAAGCCAGTCACAAAGAACCAGGTA
TTATATGATTCAACACA
Y
GTGAAACATCCAGAATAGGCAATCTGACCAGGCACAGTGGCTCATGCCTGTAATCCCAGCAATTTGGGAGGCCAAGGCAGGTG
GATCACAAGGTCAGGAG / Celera SNP ID: hCV29855681 / Public SNP ID: rs6087683 /
SNP Chromosome Position: 33774444 / SNP in Genomic Sequence: SEQ ID NO: 366 /
SNP Position Genomic: 57113 / Related Interrogated SNP: hCV1825046 / SNP
Source: dbSNP / Population(Allele,Count): Caucasian (C,104|T,122) / SNP Type:
INTRON /
Context (SEQ ID NO: 1377): /
GAGACAGGGGCAAAAGGGGACTTCACCAATGTCACTGAGTACCCTTTTTTGTATCCTTTGACTTTTTTTTTTTTTAATTGTTCA
GTCTCTGTAGAGACTGT
K
AAAAATTGGCAATGCCGGCCAGGCGCGGTGGCTCATGCCTATAATCCCAGCACGTTGGGAGGCTGAGGGGGGCAAAATCACTG
GAGGTCAGAAGTCCGAG / Celera SNP ID: hCV30270043 / Public SNP ID: rs6060257 /
SNP Chromosome Position: 33720157 / SNP in Genomic Sequence: SEQ ID NO: 366 /
SNP Position Genomic: 2826 / Related Interrogated SNP: hCV25620145 / SNP
Source: dbSNP; HapMap / Population(Allele,Count): Caucasian (G,202|T,24) /
SNP Type: INTRON /
Context (SEQ ID NO: 1378): /
CAACTCCCAAAGCAGGGCTTGCACAATACATACTTTTGACTTGCCAAAAAATAAGTTTATCCCTGGGAAATTTCCCATCCATC
TGTTGCTTTGTAGAAAT
R
CTTGAACTTTGCATGCAGTATGTCCAAACATTTCGATCGACTCCCTGGTTAGAGTCTCAGACCTCGTTATTATTATTATTATT
ATTTGAGATGAGAGTTT / Celera SNP ID: hCV30360384 / Public SNP ID: rs6058194 /
SNP Chromosome Position: 33739831 / SNP in Genomic Sequence: SEQ ID NO: 366 /
SNP Position Genomic: 22500 / Related Interrogated SNP: hCV1825046 / SNP
Source: dbSNP; HapMap / Population(Allele,Count): Caucasian (G,95|A,25) / SNP
Type: INTRON /
Context (SEQ ID NO: 1379): /
AATAAAAAAGAGACCTGACAATGAGGCTCTTACATGCATGGAGGCAGATTCAGAGTCTGGGATCCTATGTTGATGTGGCCCTC
ACTAATTCCACTTAAGA
Y
CTCCAGTTTCTGTCCTAGGATTGGTGACAGCAGAAGAAAACACTGAGAGCTAGAAATCATCTAGAGACTTCATACCAAGAGAC
TGGTTAAGTAAATTCCT / Celera SNP ID: hCV30396340 / Public SNP ID: rs6120849 /
SNP Chromosome Position: 33730387 / SNP in Genomic Sequence: SEQ ID NO: 366 /
SNP Position Genomic: 13056 / Related Interrogated SNP: hCV1825046 / SNP
Source: dbSNP; HapMap; ABI_Val / Population(Allele,Count): Caucasian
(C,178|T,48) / SNP Type: INTRON /
Context (SEQ ID NO: 1380): /
CATCTTGCACCTCAAACTCTGTCTCAGCCTCTGCTTCCAGATAACCCAACTTGTGTGTGTGTGTCTCTCTCTCTTTCAAAATT
GAGAAATTTTCTCAATC
Y
TGTCTGGACCATGGCAGCAGCCTCCTCCTAAGAAATTGCCTCTAATTTCTCCATATTTTAATTCATCTTCTACATGGCAGTAA
GAAACACAGATCTAATT / Celera SNP ID: hCV32066690 / Public SNP ID: rs7265317 /
SNP Chromosome Position: 33768523 / SNP in Genomic Sequence: SEQ ID NO: 366 /
SNP Position Genomic: 51192 / Related Interrogated SNP: hCV25620145 / SNP
Source: dbSNP; HapMap / Population(Allele,Count): Caucasian (T,204|C,22) /
SNP Type: INTRON /
Context (SEQ ID NO: 1381): /
CAGAATGGATAAACAAAATATAATACACTATTTGCAAAAGATGTATCTAATCATAAAACTACAGAAAGATGGAAAGGATAGAA
AAAGATACATGGGAAAA
K
GTCAACCAAAAGAAAGCTGGTATCACTTATATTCCTATCAGAAATAAAGGTATAAGAATGACCTTCATTTACAGATGACAAAC
TGGGCTCAGAAGCTGGG / Celera SNP ID: hDV71898298 / Public SNP ID: rs7361656 /
SNP Chromosome Position: 33729147 / SNP in Genomic Sequence: SEQ ID NO: 366 /
SNP Position Genomic: 11816 / Related Interrogated SNP: hCV1825046 / SNP
Source: dbSNP; HapMap / Population(Allele,Count): Caucasian (T,173|G,53) /
SNP Type: TRANSCRIPTION FACTOR BINDING SITE;INTRON /
Context (SEQ ID NO: 1382): /

TCCAAAATATAACAGGAAAGGGGACATTGAGAGGCAAAAAAATGGAGGAGACAAGCAGAAAAATAACAAATTGTAGACCAAAA
TCCACTCTTATCAATAA
M
TACATTAAATGTAAATGGTCTTTAAAAATGTAAATGGTCTAAACACACCAATTAAAAGACAAAGATGGCTGGGTGCGGTGGCT
CACGCCTGCAATCCCAG / Celera SNP ID: hDV72053898 / Public SNP ID: rs8114671 /
SNP Chromosome Position: 33789142 / SNP in Genomic Sequence: SEQ ID NO: 366 /
SNP Position Genomic: 71811 / Related Interrogated SNP: hCV1825046 / SNP
Source: dbSNP; HapMap / Population(Allele,Count): Caucasian (C,66|A,54) / SNP
Type: INTRON /
Context (SEQ ID NO: 1383): /
CTATTTCATTTTTTAAAAATGGTAACCATGACCTGCTAAATTGATTTCATTGTCCACTAATAAATTATGACCTCAGTTTCAAA
AAGATTGCTTTAGGTAA
S
CAATCATCTTCTGAGATTTATACAGATTGCTCATAATTCTCTCCTATTTTTTAAAAACATGCTGCAGTGAACTGCTTTACACT
CATTTTATGACTACTTC / Celera SNP ID: hDV75209987 / Public SNP ID: rs2069940 /
SNP Chromosome Position: 33759272 / SNP in Genomic Sequence: SEQ ID NO: 366 /
SNP Position Genomic: 41941 / Related Interrogated SNP: hCV25620145 / SNP
Source: CDX; dbSNP / Population(Allele,Count): Caucasian (C,201|G,23) / SNP
Type: INTRON //

Gene Number: 30 / Gene Symbol: MAP3K2 - 10746 / Gene Name:
mitogen-activated protein kinase kinase kinase 2 / Chromosome: 2 / OMIM NUMBER:
/ OMIM Information: // Genomic Sequence (SEQ ID NO: 367): // / SNP Information
/
Context (SEQ ID NO: 1384): /
ACTCTGGGGTAAAGATACTGCTTCCACTACTAGTGCTTAAGGAATGATCAGTAGGACTGAAACTCACAGGAGACCGTAAGCTG
GTCCCCTGGGTCCTTCT
R
GCTCTTGGAAAAGTTTTACGACCTAAAAAAATGAAAAGAGAATGACCTTATACCTTTTATTAATATGTTATACTTTTAAAGTAT
TTAAAATTTGTAATGTA / Celera SNP ID: hCV25630050 / Public SNP ID: rs3732209 /
SNP Chromosome Position: 128079806 / SNP in Genomic Sequence: SEQ ID NO: 367 /
SNP Position Genomic: 27667 / Related Interrogated SNP: hCV1841983 / Related
Interrogated SNP: hCV1841975 / SNP Source: Applera /
Population(Allele,Count): Caucasian (A,28|G,12) African American (A,22|G,14)
total (A,50|G,26) // / SNP Type: ESE;ESE SYNONYMOUS;SILENT MUTATION / SNP
Source: dbSNP; HapMap; ABI_Val; HGBASE / Population(Allele,Count): Caucasian
(A,158|G,68) / SNP Type: ESE;ESE SYNONYMOUS;SILENT MUTATION /
Context (SEQ ID NO: 1385): /
TCAGCTCATCGATATTGTTAGTGACTCTGCTTACTGTTTATTTATTGCAGAACTTTGAGACGGCCTTCATTAAGACCACTCTG
GAGCCCACCCTGTGTGC
R
CTTTTTCTCCGACTTCAGCAACTGCTAGATCAATGTATACATCTTATTTTTTATTACACACATTCGGGCCCACAGCTCACTGCC
TGGCCCACTGGCTTATG / Celera SNP ID: hCV169044 / Public SNP ID: rs11691088 /
SNP Chromosome Position: 128131853 / SNP in Genomic Sequence: SEQ ID NO: 367 /
SNP Position Genomic: 79714 / Related Interrogated SNP: hCV1841975 / Related
Interrogated SNP: hCV1841983 / SNP Source: dbSNP; Celera; HapMap /
Population(Allele,Count): Caucasian (A,87|G,33) / SNP Type: INTRON /
Context (SEQ ID NO: 1386): /
TAATTTTTTTCTGTCCAGTACTAGTACATAAAAAGTAAAGGTATCTGAAGCCATCTTTTAAATTTTTTTTTAAAGCCAAGAAAA
TGATTTGGCAAAAGATA
Y
CGAAGAGCTTACGTTAGGGAAAACTAAACTGTATCTGTCTATGTGTTTAGAATTTATCCCATTAAAGAAACTGCCCAGTTAGT
CTCATTTTCTAAACGTA / Celera SNP ID: hCV273435 / Public SNP ID: rs7607907 /
SNP Chromosome Position: 128144286 / SNP in Genomic Sequence: SEQ ID NO: 367 /
SNP Position Genomic: 92147 / Related Interrogated SNP: hCV1841975 / Related
Interrogated SNP: hCV1841983 / SNP Source: dbSNP; Celera; HapMap /
Population(Allele,Count): Caucasian (T,165|C,61) / SNP Type: INTRON /
Context (SEQ ID NO: 1387): /
GATCACATAATTCCCTCACTCATTCTGGATTTACTAGCACTCAGAGAGCTGAGATTGGAGCCTTAATATTAGCCTTAGAAACT
TTCTCCACTCAGCTCAT
R
GATATTGTTAGTGACTCTGCTTACTGTTTATTTATTGCAGAACTTTGAGACGGCCTTCATTAAGACCACTCTGGAGCCCACCC
TGTGTGCACTTTTTCTC / Celera SNP ID: hCV11266778 / Public SNP ID: rs2139142 /

SNP Chromosome Position: 128131762 / SNP in Genomic Sequence: SEQ ID NO: 367 /
SNP Position Genomic: 79623 / SNP Source: dbSNP; Celera; HapMap; ABI_Val;
HGBASE / Population(Allele,Count): Caucasian (G,166|A,60) / SNP Type: INTRON
/
Context (SEQ ID NO: 1388): /
TCAGCACTTCTGACATTTTGGGAGGGGTAATTCTTTGAGGCTGCTTTCCTTGTGTATTATAATCTATTTAGCAACATCCCTGG
CCTCTACCCAATTCATG
Y
TACTAGTATCCCTCCAATTGTGACAACCGGAAATGTCTCTAAGAATTGCCAAATGCCTAGTGAAATCATCCTCGCTCCACTTT
TGGCAACCACTGTTTCA / Celera SNP ID: hCV12046224 / Public SNP ID: rs10850 /
SNP Chromosome Position: 128063945 / SNP in Genomic Sequence: SEQ ID NO: 367 /
SNP Position Genomic: 11806 / Related Interrogated SNP: hCV1841975 / Related
Interrogated SNP: hCV1841983 / SNP Source: dbSNP; HapMap /
Population(Allele,Count): Caucasian (T,61|C,165) / SNP Type: UTR3 /
Context (SEQ ID NO: 1389): /
ATGTGGGCTGGCATGAGAGTATTGAACCCTTGGGAGATCTAGACACAGGTGAGCTCACACCTGCTCTAAGTCTTTTCTGTGGA
CCTCTACCATGCACTCA
Y

AGGAGCAGGAGACACCAGGGAAAGCCTCCCTTGGTGGTCAAGGCCTGGAAAAGGGGAATGGCCCACTGTTGTGGGAAACGCAG
AAAGTCCTTCCAAACCC / Celera SNP ID: hCV28026949 / Public SNP ID: rs4662720 /
SNP Chromosome Position: 128076607 / SNP in Genomic Sequence: SEQ ID NO: 367 /
SNP Position Genomic: 24468 / Related Interrogated SNP: hCV1841975 / Related
Interrogated SNP: hCV1841983 / SNP Source: dbSNP; HapMap; HGBASE /
Population(Allele,Count): Caucasian (T,61|C,165) / SNP Type: INTRON /
Context (SEQ ID NO: 1390): /
CCTGGAGGGGTGCCTGGTGCAGCAGCTTAGGCACTTGCCTACAGACCTAATGTGATCCTGCCTGTGTTCTAAGGTTACCTATT
ACAAACCGTAAAATGAG
K
ACTAAGAGTCATGAACAAGTCCCAGGTTATCTACATTACACTAAATAAATCAGATAATGTTTTCTGGCTTGGTGAATTCATTT
CAGTCTCATTCATGAAA / Celera SNP ID: hCV29636350 / Public SNP ID: rs10496661 /
SNP Chromosome Position: 128090144 / SNP in Genomic Sequence: SEQ ID NO: 367 /
SNP Position Genomic: 38005 / Related Interrogated SNP: hCV1841975 / Related
Interrogated SNP: hCV1841983 / SNP Source: dbSNP; HapMap /
Population(Allele,Count): Caucasian (G,165|T,61) / SNP Type: INTRON /
Context (SEQ ID NO: 1391): /
CATAATATCATAGTAGCTCTGTTTTCTTGATTGAGCCCTTAAATGATTCAAGTACTCAAGCTTCCTCCCAATCTACTGACCAA
GAAATCTCTGCAGCTTT
S
CCTCTGAGATCTTCTTGCATATATAACAAAATCTACATCATTTGGTCTCCAATTTGACTGCTTCCAAACAAACCTTGTTTTAT
CAGTTCTGCACTGTCCT / Celera SNP ID: hCV31814195 / Public SNP ID: rs11680949 /
SNP Chromosome Position: 128123562 / SNP in Genomic Sequence: . SEQ ID NO: 367 /
SNP Position Genomic: 71423 / Related Interrogated SNP: hCV1841975 / Related
Interrogated SNP: hCV1841983 / SNP Source: dbSNP; HapMap /
Population(Allele,Count): Caucasian (C,91|G,29) / SNP Type: INTRON /
Context (SEQ ID NO: 1392): /
GAGCTTGCAGTGAGCCGAGATCATGCCACTGCACTCCAGCCTGGGCGACAGAGCAAGACTCTGTCTCAAAATAAATAAATAAA
TAAATAAATAATCTCCA
R
CACCATGTGAGAGACCATTCCTTATACTCAGTAGGTTTGATAGTAGGGTTTGTGTTTGTTTTTAATCATTAGTTCTGATTTAG
GTAATCAAGATGTCCAG / Celera SNP ID: hCV30166207 / Public SNP ID: rs7590030 /
SNP Chromosome Position: 128055967 / SNP in Genomic Sequence: SEQ ID NO: 367 /
SNP Position Genomic: 3828 / Related Interrogated SNP: hCV1841983 / Related
Interrogated SNP: hCV1841975 / SNP Source: dbSNP / Population(Allele,Count):
Caucasian (G,68|A,158) / SNP Type: INTERGENIC;UNKNOWN /
Context (SEQ ID NO: 1393): /
GGTGTGTGGCTTATAGACCCAATCACCCATCTCCACAGAAACCTGGACAGTTTTGACTAGGGTACATAGATGGGATGAAATGA
AGGAAGGCTGTTGGACT
Y
CCAGGATTCCACAGGTAGGAAAGGGATTGGCAGGGGCAGTTCAGAGGCCAGTGAGGCTGCTTCCGCCACCACAGGACTGAAAG
GGACAGGCTTGGGTTTG / Celera SNP ID: hCV30598525 / Public SNP ID: rs7556675 /
SNP Chromosome Position: 128053747 / SNP in Genomic Sequence: SEQ ID NO: 367 /
SNP Position Genomic: 1608 / Related Interrogated SNP: hCV1841975 / Related
Interrogated SNP: hCV1841983 / SNP Source: dbSNP; HapMap /

Population(Allele,Count): Caucasian (C,61|T,165) / SNP Type: INTERGENIC;UNKNOWN /
Context (SEQ ID NO: 1394): /
ACAGCTTCATCTATGACGTATCAGATAAAAAAAGGCTGAATGTGAATCTAATTTAGACCTCAGATCTAACTTGGAGTTTATAGT AAAAAAGGGATAAAGAA
R
CAAATTAAAATCCGCATGCTCATTTCGGCAGCACATATACTAAAATTAGAACGATACAGAGAAGATTAGCACGGCCCCTGTGC AAGGATGACACGCAAAT / Celera SNP ID: hDV75133748 / Public SNP ID: rs13429925 /
SNP Chromosome Position: 128074433 / SNP in Genomic Sequence: SEQ ID NO: 367 /
SNP Position Genomic: 22294 / SNP Source: CDX; dbSNP /
Population(Allele,Count): Caucasian (A,166|G,60) / SNP Type: INTRON //

Gene Number: 31 / Gene Symbol: MMP24 - 10893 / Gene Name: matrix
metallopeptidase 24 (membrane-inserted) / Chromosome: 20 / OMIM NUMBER: / OMIM
Information: // Genomic Sequence (SEQ ID NO: 368): // / SNP Information /
Context (SEQ ID NO: 1395): /
TGGCCTCCCTTCCCCCACTTCCTGGTCCCTGTCCACTCCTCAGGTTGGTGCTCTCACTTCTTGAAAGCTCTAGGCACCCCCGC CTCCCGCCAGGCTCCCC
R
TTGGCTCCTGGCAGGCCAGCTGAGAATGAACAGGAGATGGAGGCAGGCAGCCCAGGCTGCAGAGGTGAGGGATGTGGGGCCAG GCCCAGAGGGCTCAGCC / Celera SNP ID: hCV1271624 / Public SNP ID: rs7280 / SNP
Chromosome Position: 33864484 / SNP in Genomic Sequence: SEQ ID NO: 368 / SNP
Position Genomic: 59945 / SNP Source: dbSNP; Celera; HGBASE /
Population(Allele,Count): Caucasian (A,139|G,85) / SNP Type: UTR3;INTRON /
Context (SEQ ID NO: 1396): /
ACTCTGAGGCCGACTCAATCTCTCGGCTGGAAGCAGTGTTTCCCAGAGCTTGGCCCTTGCTGACCTCGCTCACTGGGCCCAT CTTCCCACACTGCTCTT
R
GAAGGACACCCCTACCGGTAGCAGCCCCAAGCTGAGGGGGCTCCCTTTTTGACCTTCACTGGCCCGCCCTTCACTGTCTCCAG CAGGAGTTCCTAGGGCT / Celera SNP ID: hCV1271625 / Public SNP ID: rs6060341 /
SNP Chromosome Position: 33863633 / SNP in Genomic Sequence: SEQ ID NO: 368 /
SNP Position Genomic: 59094 / Related Interrogated SNP: hCV1825046 / SNP
Source: dbSNP; Celera; HapMap; ABI_Val / Population(Allele,Count): Caucasian
(A,176|G,50) / SNP Type: UTR3;INTRON /
Context (SEQ ID NO: 1397): /
GCCCACCCCCACCATGTGTCTGATCCATCCCAGTGTTTAGTTCTGGCCTAGACATTTGCCTATGACCTGAGTCCATAGATCCT AACTCCTGATGTGGCCA
S
GATAGACTCTGTGATGTTCGTAATGGATTTGGATGGGCTGTGAGATGCTCCTTGCTTGAGTATCCCAGAAAACGAGCTGCGTA AACCAGGTTGCCAGCTT / Celera SNP ID: hCV1271649 / Public SNP ID: rs6120880 /
SNP Chromosome Position: 33829406 / SNP in Genomic Sequence: SEQ ID NO: 368 /
SNP Position Genomic: 24867 / Related Interrogated SNP: hCV1825046 / SNP
Source: dbSNP; Celera; HapMap / Population(Allele,Count): Caucasian
(C,52|G,50) / SNP Type: INTRON /
Context (SEQ ID NO: 1398): /
ACTGTATTTTTAATTCATTTTCTCTTATTTTTACTGTGGGTTTCATATTACTTTAATAGCTGTTTATATTTGTAAGGTGCTT TGCACTTCCCATATGCC
R
TGTATGTGGCCCTATTTGGTCCTGGGAACAATACCATGAGGTAGATAAGGAAGACATTGTCCTCATTTTATAGGTGAGTAAAA GGAGACTCAAAGATGAT / Celera SNP ID: hCV1271653 / Public SNP ID: rs2425019 /
SNP Chromosome Position: 33819415 / SNP in Genomic Sequence: SEQ ID NO: 368 /
SNP Position Genomic: 14876 / Related Interrogated SNP: hCV1825046 / SNP
Source: dbSNP; Celera; HapMap; HGBASE / Population(Allele,Count): Caucasian
(A,128|G,98) / SNP Type: INTRON /
Context (SEQ ID NO: 1399): /
AATGCTGGAACAACTGGAGAGTGCAGGGATTGTACACAGGCTGAGGGCCCCAAACATCACATTCAGAATGGCCCGGAGGGAAC TGCACTTTAATGGGGTG
S
CACAGGACAGCAGAACCCTCAGCCAGCAGCCACAGGGCCTGCCAGCCAGCACAACAGAGCAGGTTTTTGCAGTAATGATAGAT CCAGGCGATAAGCACAG / Celera SNP ID: hCV11656916 / Public SNP ID: rs7004 / SNP
Chromosome Position: 33866753 / SNP in Genomic Sequence: SEQ ID NO: 368 / SNP
Position Genomic: 62214 / Related Interrogated SNP: hCV1825046 / SNP Source:
dbSNP; HGBASE / Population(Allele,Count): Caucasian (G,78|C,28) / SNP Type:

MISSENSE MUTATION;ESS;TRANSCRIPTION FACTOR BINDING SITE;MICRORNA;UTR3; / ESE SYNONYMOUS;INTRON //
Context                    (SEQ ID NO: 1400): /
TGTAAGTTGGTCAATCACTTTAGATAATTATCTGGCATCATCTGCAACTACTACTAAATAGTCACATAACCTGTGACTCTGCC ATGCCACTCTTAGGTAT
Y
ATATACCCAGTAGAAATATGTACATGTGTTCACTAAAAAAAACATAGCAGCACTATTACTTTTTTTTTTTTTTTTTTTGAGACGGAG TCTTGTTCTGTTGCCCA / Celera SNP ID:    hCV16013724 / Public SNP ID:    rs2425044 /
SNP Chromosome Position:    33869735 / SNP in Genomic Sequence:    SEQ ID NO: 368 /
SNP Position Genomic:    65196 / Related Interrogated SNP:    hCV1825046 / SNP
Source:    dbSNP; HGBASE / Population(Allele,Count):    Caucasian (C,178|T,48) /
SNP Type:    INTRON /
Context                    (SEQ ID NO: 1401): /
GCTAATGGACAGTCTCGGTGGAGGGCTGGGCTGTTGGGACCTAAGCCCCACAGCTGCAATCACTGGGTTCGGAGGCAGGGCCT GACACACCTCTCTCCCC
Y
TCTCCCGCTTCCTCCCAGGATCGCTGGTTCTGGCGTCTGCGCAATAACCGAGTGCAGGAGGGCTACCCCATGCAGATCGAGCA GTTCTGGAAGGGCCTGC / Celera SNP ID:    hCV25472481 / Public SNP ID:    rs2275274 /
SNP Chromosome Position:    33857544 / SNP in Genomic Sequence:    SEQ ID NO: 368 /
SNP Position Genomic:    53005 / Related Interrogated SNP:    hCV25620145 / SNP
Source:    Applera / Population(Allele,Count):    Caucasian (C,33|T,3) African
American (C,31|T,5) total (C,64|T,8) / SNP Type:    INTRON / SNP Source:
dbSNP; HapMap; ABI_Val; HGBASE / Population(Allele,Count):    Caucasian
(C,203|T,21) / SNP Type:    INTRON //

Gene Number:    32 / Gene Symbol:    ADAMTS13 - 11093 / Gene Name:    ADAM
metallopeptidase with thrombospondin type 1 motif, 13 / Chromosome:    9 / OMIM
NUMBER:    604134 / OMIM Information:    Thrombotic thrombocytopenic purpura,
familial, 274150 (3) // Genomic Sequence (SEQ ID NO: 369): // / SNP Information
/
Context                    (SEQ ID NO: 1402): /
TTTGCGTGCCTAGTCACTAATGGGGTCTGGCTCTTGGGGTGGGGGTGACACGCAATGTCTTGACTTCGGAAGGCCATCCTTCC AAGACCTGCCAGCCCCT
Y
TCCTGTTAGCTTTCCACTGCTTGCTCTCTAGAACCATCGCCCTCTGCTCTCCCTCTCCCCCTCCAGGCCGCCCTCCTTCCCCT GGCTTCCAGAGGCAGAG / Celera SNP ID:    hCV3183341 / Public SNP ID:    rs2285489 /
SNP Chromosome Position:    136289374 / SNP in Genomic Sequence:    SEQ ID NO: 369 /
SNP Position Genomic:    19915 / Related Interrogated SNP:    hCV25610857 / SNP
Source:    dbSNP; Celera; ABI_Val; HGBASE / Population(Allele,Count):    Caucasian
(T,47|C,71) / SNP Type:    INTRON /
Context                    (SEQ ID NO: 1403): /
TTTCTGGAGTGTGTCTGCACCTAACCTTTGAAGCCTTGGTTGCCGGCACTTGCCATGGGGTCCCTGAGCCCTGAGCCTGTTGA GTTCTGTGCGTGAGTGC
R
CTTGGTCATAGCACTCACCAGGTTGTGGAAAGAGGCCTAGAGCCTCCGCTGTGGGGAAGCCTCTAGCTCAGATGCCTGTGGCT CCTTAGAGGAGGGCTGG / Celera SNP ID:    hCV3183366 / Public SNP ID:    rs2073933 /
SNP Chromosome Position:    136305275 / SNP in Genomic Sequence:    SEQ ID NO: 369 /
SNP Position Genomic:    35816 / Related Interrogated SNP:    hCV25610857 / SNP
Source:    dbSNP; Celera; ABI_Val; HGBASE / Population(Allele,Count):    Caucasian
(A,149|G,77) / SNP Type:    INTRON /
Context                    (SEQ ID NO: 1404): /
GCCAAGATGCCCTCACTTCTGACATCACCCGCAAGTTCAGGGGGTTCCCCAAACCACCCTCAGGCTTGATAGTTGACTAGGAA GACTCCCAGAGCTCACT
S
AGAGCTGTGGCACATGGCTGCGGCTCCTTCCAGAAGAACACAGGTTAGAATTGTCCAAGGGAAGAGATGTAGGCAGAGTCTGG GAGGGTCCAACCAGGAG / Celera SNP ID:    hCV15862346 / Public SNP ID:    rs2073934 /
SNP Chromosome Position:    136304728 / SNP in Genomic Sequence:    SEQ ID NO: 369 /
SNP Position Genomic:    35269 / Related Interrogated SNP:    hCV25610857 / SNP
Source:    dbSNP; ABI_Val; HGBASE / Population(Allele,Count):    Caucasian
(G,81|C,39) / SNP Type:    INTRON /
Context                    (SEQ ID NO: 1405): /
TGTCTAGTTTCAGAACGTTTCCAGTACTCTGGAGTACTCTGGAGTGAACCCCATATGGTAGGCTGTCACTCCCCATTTCTCCT CCGCCACTCAGCGGCCA

Y
TGGTTTCCCTTCTGTCTCTGTGGATTGACCTGTTCTAGACATGCCACGTACCTGAGGCCAGACAACAGGTGTGCTTCCTGCCT
GCCTTCCTCCCCCAGCG / Celera SNP ID: hDV72329597 / Public SNP ID: rs28793911 /
SNP Chromosome Position: 136299937 / SNP in Genomic Sequence: SEQ ID NO: 369 /
SNP Position Genomic: 30478 / Related Interrogated SNP: hCV25610857 / SNP
Source: dbSNP / Population(Allele,Count): Caucasian (T,149|C,77) / SNP Type:
INTRON /
Context        (SEQ ID NO: 1406): /
GTCTCCCAGTGACAACACCCGCCCCCCGCCCCACCGCCATCCCCCTCCTCTGCCTCCTCCTGGCCAGGCCTGCGAGAAGACCC
AGCTGGAGTTCATGTCG
S
AACAGTGCGCCAGGACCGACGGCCAGCCGCTGCGCTCCTCCCCTGGCGGCGCCTCCTTCTACCACTGGGGTGCTGCTGTACCA
CACAGCCAAGGTGGGGC / Celera SNP ID: hCV11571465 / Public SNP ID: rs2301612 /
SNP Chromosome Position: 136301982 / SNP in Genomic Sequence: SEQ ID NO: 369 /
SNP Position Genomic: 32523 / Related Interrogated SNP: hCV25610857 / SNP
Source: CDX; dbSNP / Population(Allele,Count): Caucasian (C,74|G,46) / SNP
Type: MISSENSE MUTATION;ESE;MICRORNA;UTR3;INTRON //

Gene Number: 33 / Gene Symbol: PTPN21 - 11099 / Gene Name: protein tyrosine
phosphatase, non-receptor type 21 / Chromosome: 14 / OMIM NUMBER: / OMIM
Information: // Genomic Sequence (SEQ ID NO: 370): // / SNP Information /
Context        (SEQ ID NO: 1407): /
GCATGTGCTGTTGATTACTTGAGGCGTTCCTCTCACCTTAATATGTGATGCGTTGATGTAACCAGTGTTGTTTTCTTTAGTTG
GGACCAACTCCACTCTC
R
CATCATCATAAGGAAGAACATCTTGGAATCGATTTCTTTCTGCATTTTCAGGGAGTCGTGCTGTTGAGCACTCCCCATCAACT
AGCCGTTTCTTAAGAAT / Celera SNP ID: hCV16182835 / Public SNP ID: rs2274736 /
SNP Chromosome Position: 88938652 / SNP in Genomic Sequence: SEQ ID NO: 370 /
SNP Position Genomic: 36936 / SNP Source: Applera / Population(Allele,Count):
Caucasian (A,23|G,13) African American (A,18|G,20) total (A,41|G,33) // /
SNP Type: MISSENSE MUTATION;PSEUDOGENE / SNP Source: dbSNP; HapMap; ABI_Val;
HGBASE / Population(Allele,Count): Caucasian (A,151|G,75) / SNP Type:
MISSENSE MUTATION;PSEUDOGENE /
Context        (SEQ ID NO: 1408): /
TCTGGAGAATATTCAGTTTTTTCAACCTGTTTGTTGGTCTGAACCAGTGTTGCACTGGTATTATTTCAGTAAAACTACAAAAC
CTTACCAAAATCCTTTC
R
GGACACTTAACTCTAAGATTAAATTACATTTCAATAAATATTAATCACTGTAAGTAACAAAATATTTGTCTCATTCAATGGTC
CATTAATTTCAAGTGAT / Celera SNP ID: hCV2485050 / Public SNP ID: rs6575009 /
SNP Chromosome Position: 88928413 / SNP in Genomic Sequence: SEQ ID NO: 370 /
SNP Position Genomic: 26697 / SNP Source: dbSNP; Celera; HapMap; ABI_Val /
Population(Allele,Count): Caucasian (A,207|G,17) / SNP Type: INTRON /
Context        (SEQ ID NO: 1409): /
TACACCCAGGCCCCTAGCAGAAACCGCTTCCCATATTTACAAAGAAAGCAGGTTCCTCCTCCCTGTCAGTGCTTACGAAAATC
CCATCACCAGACCAGCG
R
TGGGGACTCGGATGCAAACATGGTGCAGGGGAAGGCAGAGAAGAGACAGGGACTGGGCCATACATGCAAACCACTGGCCAGCT
CCCCGTCCGGGTGGGCA / Celera SNP ID: hCV27484761 / Public SNP ID: rs3783886 /
SNP Chromosome Position: 88958847 / SNP in Genomic Sequence: SEQ ID NO: 370 /
SNP Position Genomic: 57131 / SNP Source: dbSNP; HapMap; HGBASE /
Population(Allele,Count): Caucasian (A,107|G,9) / SNP Type: INTRON /
Context        (SEQ ID NO: 1410): /
AAAGTTAAAGTCACTCACATAAAAATCCCTTGAGGTCTTCTGGACAGCCATGTTCAGGCCAGTCTGTGTATTGGAGGTGCCAG
ACGGTCCTCTCTTGCCC
R
GTAAGGAGGTGCTTCATCTTCAGGCCTGTGGTGGCATAGCAGCCAGAGTCTGTGCGGAACCGGGTCGTGATCTTAAACCTTCC
ATAGGTGACAGTGTTGT / Celera SNP ID: hCV16185886 / Public SNP ID: rs2297129 /
SNP Chromosome Position: 88935925 / SNP in Genomic Sequence: SEQ ID NO: 370 /
SNP Position Genomic: 34209 / Related Interrogated SNP: hCV16182835 / SNP
Source: Applera / Population(Allele,Count): Caucasian (A,27|G,13) African
American (A,16|G,20) total (A,43|G,33) // / SNP Type: MICRORNA;UTR3;SILENT
MUTATION;INTRON / SNP Source: dbSNP; HGBASE / Population(Allele,Count):
Caucasian (A,151|G,75) / SNP Type: MICRORNA;UTR3;SILENT MUTATION;INTRON /

Context                    (SEQ ID NO: 1411): /
ATACTTCCTTCCAAGATATCTTTCTTCAGTTGCAGATAATACTGATACCTATAAAAAATGTCAGTTGTATAAGGTCAATGTCA
ATATTATCTCATTGAGA
Y
ACAATGCATTTAACCATAATTTGGTAGAGAAGCATAAACCAGTGAAATAAAACCTTAATCCCACATGGTTGTGTGTGTTTTTT
TTTTTTTTTTTTAGATAG / Celera SNP ID:   hCV25933483 / Public SNP ID:    rs10143744 /
SNP Chromosome Position:   88974417 /  SNP in Genomic Sequence:   SEQ ID NO: 370 /
SNP Position Genomic:   72701 /  Related Interrogated SNP:   hCV16182835 /  SNP
Source:  Applera / Population(Allele,Count):   Caucasian (C,4|T,20)  African
American (C,8|T,14)  total (C,12|T,34) /  SNP Type:   TRANSCRIPTION FACTOR BINDING
SITE;INTRON /  SNP Source:   dbSNP; HapMap /  Population(Allele,Count):   Caucasian
(T,157|C,69) /  SNP Type:   TRANSCRIPTION FACTOR BINDING SITE;INTRON /
Context                    (SEQ ID NO: 1412): /
CTTGTTGTAGTACCAGAGGCTGAAGTAAGTGACCTGGAAAGACAGAAGGTCATTGAAGGAGGCAATAACATACAAAAGGATTT
CAATGACTTCAGTCGCA
M
TAATCCCTGACCTGTGACCAAATGACAGCAGTCTCTTATTGCCTTTCTTCTCAGAGAAGAACAACAGCAAAAACTGAAGGAAA
AAAAGGAGGTAAGACCT / Celera SNP ID:   hCV25935678 / Public SNP ID:    rs4904452 /
SNP Chromosome Position:   88983673 /  SNP in Genomic Sequence:   SEQ ID NO: 370 /
SNP Position Genomic:   81957 /  Related Interrogated SNP:   hCV16182835 /  SNP
Source:  Applera / Population(Allele,Count):   Caucasian (A,25|C,13)  African
American (A,9|C,21)  total (A,34|C,34) /  SNP Type:   INTRON /  SNP Source:
dbSNP; HGBASE /  Population(Allele,Count):   Caucasian (C,37|A,77) /  SNP Type:
INTRON /
Context                    (SEQ ID NO: 1413): /
TTGGACGACATCGGCGAGGGCTGCAAGTAGGGCTGAGGATTATTTAAGGAGTTGGTGCTGTGTGCACTGTAGACACTGCCATT
ACGGATCCGACCGTTGA
R
GTCAATCTGGGCTCTATCCAAGCTTGTCTGAGAGTGACAGTAGTATCCGTTCTGGTTGGGCACAAAGAGGTTATCTAGAAAAA
ATGAGTTCAAAATGAGA / Celera SNP ID:   hCV25942539 / Public SNP ID:    rs2401751 /
SNP Chromosome Position:   88946622 /  SNP in Genomic Sequence:   SEQ ID NO: 370 /
SNP Position Genomic:   44906 /  Related Interrogated SNP:   hCV16182835 /  SNP
Source:  Applera / Population(Allele,Count):   Caucasian (A,12|G,28)  African
American (A,14|G,22)  total (A,26|G,50) //  / SNP Type:   MISSENSE MUTATION;ESE
/  SNP Source:   dbSNP; HapMap; ABI_Val; HGBASE /  Population(Allele,Count):
Caucasian (G,151|A,75) /  SNP Type:   MISSENSE MUTATION;ESE /
Context                    (SEQ ID NO: 1414): /
AAGGCAAATTTCTGAAGAAAGTCCTGGGATTCATACTGATCAAAGTCACCAAAATCCGCTATATAGAATGACAAACATTCACG
TTAATTATATTTAAATT
K
CATTGCAAAATATGCACAACAAAATTCAATACGCAGTATATCTAGATGGTATGCTAATATTTCAGAAGTCAAATTTGTTATAG
CTATATAAAGTATATTT / Celera SNP ID:   hCV3211540 / Public SNP ID:    rs2274735 /
SNP Chromosome Position:   88971758 /  SNP in Genomic Sequence:   SEQ ID NO: 370 /
SNP Position Genomic:   70042 /  Related Interrogated SNP:   hCV16182835 /  SNP
Source:  Applera / Population(Allele,Count):   Caucasian (G,13|T,27)  African
American (G,20|T,18)  total (G,33|T,45) //  / SNP Type:   TRANSCRIPTION FACTOR
BINDING SITE;TFBS SYNONYMOUS;INTRON /  SNP Source:   dbSNP; Celera; HapMap; HGBASE
/  Population(Allele,Count):   Caucasian (T,150|G,76) /  SNP Type:   TRANSCRIPTION
FACTOR BINDING SITE;TFBS SYNONYMOUS;INTRON /
Context                    (SEQ ID NO: 1415): /
ACTTAGGGTTAGGGAATGGCAGACCTGGAGAAAAGGGTTCCGCTACTACCCTCGGCTTTGCCAAACTTAACATTTTGAAATTC
CATTCCACAAATGTGTA
Y
TGCGTCTCTGGGAACAAGACAGCGTAATGAGCATCGTGAGAAATCTAAGACGAATCCCTAAACCGTCGCTGTCTTTAAAGAGA
TTACAACCATGCTATAA / Celera SNP ID:   hCV3211566 / Public SNP ID:    rs930181 /
SNP Chromosome Position:   89018708 /  SNP in Genomic Sequence:   SEQ ID NO: 370 /
SNP Position Genomic:   116992 /  Related Interrogated SNP:   hCV16182835 /  SNP
Source:  Applera / Population(Allele,Count):   Caucasian (C,27|T,11)  African
American (C,4|T,12)  total (C,31|T,23) /  SNP Type:   UTR3;INTRON /  SNP Source:
dbSNP; Celera; HapMap; ABI_Val; HGBASE /  Population(Allele,Count):   Caucasian
(T,77|C,149) /  SNP Type:   UTR3;INTRON /
Context                    (SEQ ID NO: 1416): /
ATCTTGTAACCATCCCTGAAGAAAACACACAGTGGTAAGTATGTGACAATTCACCCCAATTTCTTAGAAGGAATCATGGCCAT

TGACTGTTAATGACTCT
Y
GTCACTATCAGGCAAAATTATGACTATTGTTACTATATGGGAGCTTTGTTTACATAGGTAATCATCTTTCTAAGGAATTAACT
GGCTTCTCACAAGAGCC / Celera SNP ID: hCV3211539 / Public SNP ID: rs1998670 /
SNP Chromosome Position: 88970925 / SNP in Genomic Sequence: SEQ ID NO: 370 /
SNP Position Genomic: 69209 / Related Interrogated SNP: hCV16182835 / SNP
Source: dbSNP; Celera; HapMap; ABI_Val; HGBASE / Population(Allele,Count):
Caucasian (C,161|T,57) / SNP Type: INTRON /
Context (SEQ ID NO: 1417): /
ACTTAAACTGTAAGCGAATATAACCTCATCAAACTCACTGACTGGCATATCTTTCTTCTTAAAATATGACCTATTAGCAGACA
AAGTCTTTGAACTAGAT
R
AATACATAGCATAGTATTAATAAATGTATATGTATATAGTCATGTGTCAAGGATGGGAATGTGTTGAGAAAGGCATCATCAGA
TTTTGTCACTGTGCAAA / Celera SNP ID: hCV3211544 / Public SNP ID: rs9323830 /
SNP Chromosome Position: 88973604 / SNP in Genomic Sequence: SEQ ID NO: 370 /
SNP Position Genomic: 71888 / Related Interrogated SNP: hCV16182835 / SNP
Source: dbSNP; Celera; HapMap / Population(Allele,Count): Caucasian
(G,85|A,35) / SNP Type: TFBS SYNONYMOUS;INTRON /
Context (SEQ ID NO: 1418): /
ACAAAGTCTTTGAACTAGATGAATACATAGCATAGTATTAATAAATGTATATGTATATAGTCATGTGTCAAGGATGGGAATGT
GTTGAGAAAGGCATCAT
Y
AGATTTTGTCACTGTGCAAACATCAGAGTATACTTACACAAACCTAGGTGGTGCAGCCTACTCCACACCTAGGCTATATTGTA
TAGCCTACTACACACCT / Celera SNP ID: hCV3211545 / Public SNP ID: rs7160647 /
SNP Chromosome Position: 88973684 / SNP in Genomic Sequence: SEQ ID NO: 370 /
SNP Position Genomic: 71968 / Related Interrogated SNP: hCV16182835 / SNP
Source: dbSNP; Celera; HapMap / Population(Allele,Count): Caucasian
(C,153|T,69) / SNP Type: INTRON /
Context (SEQ ID NO: 1419): /
AACACAATGGTATTTGTGTATCTAAACACAGAAAAGGTACGCTAAAAATAGTGTTATAATTTTATGGGACCACTGTCCTATAT
GAGGTCTTTTGTTGAAC
R
AAATGTCATTCGGCATATGATTGTACATATGTGTGTGTATACACACATATATACATACTTCTATTTATTGTACTGTTTGCTCT
GTTGACCTATAACATGA / Celera SNP ID: hCV3211546 / Public SNP ID: rs7143642 /
SNP Chromosome Position: 88973960 / SNP in Genomic Sequence: SEQ ID NO: 370 /
SNP Position Genomic: 72244 / Related Interrogated SNP: hCV16182835 / SNP
Source: dbSNP; Celera; HapMap / Population(Allele,Count): Caucasian
(A,85|G,35) / SNP Type: TFBS SYNONYMOUS;INTRON /
Context (SEQ ID NO: 1420): /
CATTATTCTAGATGCTGGGGATACCGTACTAAATGGAAATCACAGACACTCTGCCAGTGCAGTCTATGGAGCCATCTGGAATC
CAAACTGTCCTCCGTAT
Y
TGGTGCCTGTGAAGTGCAACCCAGTTACAGTTCATGTTTGAATTCCTCTGACAGCCCACCCCTTTAATCATTTCATATGAGTC
TTTATCATCTTATTTCA / Celera SNP ID: hCV3211548 / Public SNP ID: rs7151164 /
SNP Chromosome Position: 88975673 / SNP in Genomic Sequence: SEQ ID NO: 370 /
SNP Position Genomic: 73957 / Related Interrogated SNP: hCV16182835 / SNP
Source: dbSNP; Celera; HapMap / Population(Allele,Count): Caucasian
(C,85|T,35) / SNP Type: INTRON /
Context (SEQ ID NO: 1421): /
AATCATTTCATATGAGTCTTTATCATCTTATTTCAGCCACAGACATGTAAACCTCTGTTCTTTATTACCAAAGAACCTGACCA
AAACACCATCCATTCAA
K
TAAGAAGAATTTGGATCCAGCTTTTCTCCTTCCACCTGCTAACAGCCAAACCCATGGATTTCCCTCTATAGCTAATGGGATGC
TGGCAGTTCTGGGATCC / Celera SNP ID: hCV3211549 / Public SNP ID: rs12433026 /
SNP Chromosome Position: 88975839 / SNP in Genomic Sequence: SEQ ID NO: 370 /
SNP Position Genomic: 74123 / Related Interrogated SNP: hCV16182835 / SNP
Source: dbSNP; Celera; HapMap / Population(Allele,Count): Caucasian
(G,155|T,69) / SNP Type: INTRON /
Context (SEQ ID NO: 1422): /
AAATAACTCCAAACATTAATTAGTCATGTATTCAACAACAATCACTGTTCCTAAAAGCAGAGGTAGTATCCTTTATTTACGAC
AGATAGAAATTTATAAA
Y
TGTTTCCAAAACTTAGCACTCCAAATAGTTTGGACAAAGGAGTCAACCTTGTTTATCAGGCAAAGTTAGTTATGAAGCGCTTC

ATTTCCCAAAGAAATAA / Celera SNP ID: hCV3211559 / Public SNP ID: rs2004329 /
SNP Chromosome Position: 88999309 / SNP in Genomic Sequence: SEQ ID NO: 370 /
SNP Position Genomic: 97593 / Related Interrogated SNP: hCV16182835 / SNP
Source: dbSNP; Celera; HapMap; HGBASE / Population(Allele,Count): Caucasian
(T,89|C,27) / SNP Type: INTRON /
Context (SEQ ID NO: 1423): /
TTACTGATTTATGCATTTATTATACTTTTTATTTAGAGTGTACTCCTATTTATAAAAAAGTTAACTGTAGGCCAGGTGCAGTA
GCTCCCATCTGTAATCC
Y
AGTGCTTTGGGAGACTGAGGCCAGAGGATCACTTGAGCCAAGGAGTTCAAGAACAGCCTGGGCAACATAGTGAGACCCCATCA
CAACAAAAAATACAAAA / Celera SNP ID: hCV3211560 / Public SNP ID: rs12436326 /
SNP Chromosome Position: 88999976 / SNP in Genomic Sequence: SEQ ID NO: 370 /
SNP Position Genomic: 98260 / Related Interrogated SNP: hCV16182835 / SNP
Source: dbSNP; Celera; HapMap / Population(Allele,Count): Caucasian
(C,92|T,28) / SNP Type: INTRON /
Context (SEQ ID NO: 1424): /
ATAGATATTTTTGTATAGCTGTACAATGTGTCTGTGTTTTAACCTATGTTATTACAAGAGTCAAAAGTTTTAAAAATTTAAAA
GTTTGTAAAGCAAAAAC
R
TTATAGTAAGCTGAGGTTAATTTATTATCGAAGGCCAGGTGTGGTGGCTCATGCCTGTAATCCCAGCACTTTAGGAGGCCGAG
GTGGGAGGATCACCTGA / Celera SNP ID: hCV3211561 / Public SNP ID: rs8017811 /
SNP Chromosome Position: 89000903 / SNP in Genomic Sequence: SEQ ID NO: 370 /
SNP Position Genomic: 99187 / Related Interrogated SNP: hCV16182835 / SNP
Source: dbSNP; Celera; HapMap / Population(Allele,Count): Caucasian
(G,75|A,147) / SNP Type: INTRON /
Context (SEQ ID NO: 1425): /
GTTTTTAAAGAGTAAACTAGGATTAAAGGGGAAATTTTAGTAGTCACGTGTCCCTTAACGATGTGGGAATGCATCATTAGGCT
ATTTCATTGTGTGAACA
K
TATGGAGTGTACTTACACAAACCTAAATGGTATAGCCTCCTACACATTTAGGGCATGTGATATAGCCTACTGCTGCTAGGCTA
CAAACCTGTACAGCACA / Celera SNP ID: hCV3211562 / Public SNP ID: rs4904454 /
SNP Chromosome Position: 89004121 / SNP in Genomic Sequence: SEQ ID NO: 370 /
SNP Position Genomic: 102405 / Related Interrogated SNP: hCV16182835 / SNP
Source: dbSNP; Celera; HapMap; ABI_Val; HGBASE / Population(Allele,Count):
Caucasian (T,39|G,81) / SNP Type: INTRON /
Context (SEQ ID NO: 1426): /
CAGTGGATCCCTTAAGCCCGGGAGTCTGAGGTTACAGTGAGCTATGATCACACCACCACACTCCAGCCTGGAAGACAGAGCAA
GACCCCGTCTCTTTAAA
W
AAATAAATAAATAAAAGTGTTAGATGGTGGAAATACCTAGCAAATGTTTGTCTCAATGACTTACACCTTTTGAGCTCACTATA
GTAAGGGTAAGGAAGAA / Celera SNP ID: hCV3211568 / Public SNP ID: rs816075 /
SNP Chromosome Position: 88956000 / SNP in Genomic Sequence: SEQ ID NO: 370 /
SNP Position Genomic: 54284 / Related Interrogated SNP: hCV16182835 / SNP
Source: dbSNP; Celera; HapMap; HGBASE / Population(Allele,Count): Caucasian
(A,37|T,83) / SNP Type: INTRONIC INDEL;INTRON /
Context (SEQ ID NO: 1427): /
CTAACTCTTAAAGTGATTGATTACTGTGGAGATGTGTGATGACAGATTGGCACCTACTAAGGAAACCTCTCTTGCCTGGTACA
TTATTAAAAATAATCCA
R
TGGGGAAATAGGACTAATTTAATTACATGCCTTTATGCAGTAAGATCCACAGTTGAAAAATAGAGGCAGTAGCTTTAATTGCA
CTGTAATGTTCACACAC / Celera SNP ID: hCV9595827 / Public SNP ID: rs845757 /
SNP Chromosome Position: 88921716 / SNP in Genomic Sequence: SEQ ID NO: 370 /
SNP Position Genomic: 20000 / Related Interrogated SNP: hCV16182835 / SNP
Source: dbSNP; HapMap; ABI_Val; HGBASE / Population(Allele,Count): Caucasian
(G,78|A,148) / SNP Type: INTRON /
Context (SEQ ID NO: 1428): /
AGAGACAGGGTCTGGCCCTGTGGTCCAGGCTGAAGATAAACTTCTTAAGATGTGGAAATACTCATCTGCATAAACCTCCTTAG
CATTTGGTATAGCCCCA
Y
ACAGAGAGAGTATAGAGTAAATGTTAATTGACTGCTTAAACTTCAGCTTGGGGGTTTGTTATAAATACAATTCCTTATACTTC
TCATGTGAAAACTGCAG / Celera SNP ID: hCV9595840 / Public SNP ID: rs816072 /
SNP Chromosome Position: 88950679 / SNP in Genomic Sequence: SEQ ID NO: 370 /
SNP Position Genomic: 48963 / Related Interrogated SNP: hCV16182835 / SNP

Source: dbSNP; Celera; HapMap; ABI_Val; HGBASE / Population(Allele,Count): Caucasian (T,35|C,81) / SNP Type: INTRON /
Context (SEQ ID NO: 1429): /
AAAAAAAAAAAAAAAAAAAAAAGAAGAGTGTCAGTGTCAGGATCTGATTCAGGTTAAACTAAGATCATTCTGGCTGACATATGGAA
AATAAGCTACAGGCAGC
R
AAGGCACAGAAACAGGAAAGTCAGAAGGCTACCTCAGTATTCTGATGAGAAGTAGTGGCAGCCAGCACTAAGCTGTTAACACG
AGTGGTAAAAATGGTCA / Celera SNP ID: hCV9595849 / Public SNP ID: rs1152376 /
SNP Chromosome Position: 88961465 / SNP in Genomic Sequence: SEQ ID NO: 370 /
SNP Position Genomic: 59749 / Related Interrogated SNP: hCV16182835 / SNP
Source: dbSNP; HGBASE / Population(Allele,Count): Caucasian (G,76|A,150) /
SNP Type: INTRON /
Context (SEQ ID NO: 1430): /
AAGAAACACCCAGGAAGCAGATGCAGAGGAACTGCTGCTAGAGTTCCCTGGACTGGTTTCCCAGATGTAGAAAGTTCCGAAGA
CCTACAGAGTAACTATT
M
AGGAAGACAAAAGTCTGAATGAGATCTATGATCTTATAGGTAAGTATAAAAAATTGAAAAAGTTTGGCCCTCCTTAGGAACTCT
ATTTCAAGACCATTTCA / Celera SNP ID: hCV9595856 / Public SNP ID: rs816069 /
SNP Chromosome Position: 88967336 / SNP in Genomic Sequence: SEQ ID NO: 370 /
SNP Position Genomic: 65620 / Related Interrogated SNP: hCV16182835 / SNP
Source: dbSNP; HapMap; ABI_Val; HGBASE / Population(Allele,Count): Caucasian
(C,76|A,148) / SNP Type: INTRON;PSEUDOGENE /
Context (SEQ ID NO: 1431): /
CCTCCTTGCCCCTAATAGATCTGCACTTCCTTTCACATTTCCTCTCTGTTATAGCCCCACCATCTACTGAAAACCTGAATATC
AACCAGGACCCATGCTA
W
CCCTTCTCTATTACTCGGTAGGTCCTGATCCAGATTCCAACCACCTCTCTCCAACCCTATCCAACTCCAGGTGGCTACCATCT
GTACCTGAACCACTCCC / Celera SNP ID: hCV9595863 / Public SNP ID: rs1344747 /
SNP Chromosome Position: 89014435 / SNP in Genomic Sequence: SEQ ID NO: 370 /
SNP Position Genomic: 112719 / Related Interrogated SNP: hCV16182835 / SNP
Source: dbSNP; HapMap; ABI_Val; HGBASE / Population(Allele,Count): Caucasian
(A,39|T,81) / SNP Type: INTRON /
Context (SEQ ID NO: 1432): /
GGCAGGGTGGAATTTTATCTCAGAGTTGTGCTAGGCTCAGGGTCAGACTCCACCCTTCACTGTATCCTACAAATAGGGATAGC
AAACTGGGCCATCCTCA
R
GATTAAATGAGATAATAGCCAGCACTACTATAATCCCAAATTTTGTAGTTAGTAATTGTGATCCTCTTCCTATTTTGGATAAG
GTGTTGTTTTTTCATAG / Celera SNP ID: hCV9595868 / Public SNP ID: rs891750 /
SNP Chromosome Position: 89017200 / SNP in Genomic Sequence: SEQ ID NO: 370 /
SNP Position Genomic: 115484 / Related Interrogated SNP: hCV16182835 / SNP
Source: dbSNP; Celera; HapMap; HGBASE / Population(Allele,Count): Caucasian
(G,168|A,58) / SNP Type: INTRON /
Context (SEQ ID NO: 1433): /
CAGGGTCAGACTCCACCCTTCACTGTATCCTACAAATAGGGATAGCAAACTGGGCCATCCTCAGGATTAAATGAGATAATAGC
CAGCACTACTATAATCC
Y
AAATTTTGTAGTTAGTAATTGTGATCCTCTTCCTATTTTGGATAAGGTGTTGTTTTTTCATAGGTAACTCTTTCAGGTTGTAA
TACTCCAAATGTCAGAG / Celera SNP ID: hCV9595869 / Public SNP ID: rs891749 /
SNP Chromosome Position: 89017237 / SNP in Genomic Sequence: SEQ ID NO: 370 /
SNP Position Genomic: 115521 / Related Interrogated SNP: hCV16182835 / SNP
Source: dbSNP; Celera; HapMap; HGBASE / Population(Allele,Count): Caucasian
(C,168|T,58) / SNP Type: INTRON /
Context (SEQ ID NO: 1434): /
ATTGCCTCTGTATGTTCTGTCAATTCATTAAAAGATAAAGGAAATCTAAGTGACATTTGTTTATGAACAAATTGCTCAGAGAA
CTGAATAGGAAAATATT
Y
GCTAAATTGGCATACTGTTAAAAGAATGATGAATCCTTGTTAATAGAAAAGAAAATGCCATATTCACATAAAGTGTGTATCTG
ACAAGAAGAATGATACA / Celera SNP ID: hCV11295871 / Public SNP ID: rs17203789 /
SNP Chromosome Position: 89007706 / SNP in Genomic Sequence: SEQ ID NO: 370 /
SNP Position Genomic: 105990 / Related Interrogated SNP: hCV16182835 / SNP
Source: dbSNP; Celera; HapMap / Population(Allele,Count): Caucasian
(C,86|T,26) / SNP Type: INTRON /
Context (SEQ ID NO: 1435): /

TCTGTCTCAAAACAAAAAACAAAAAACATGAAAAAGCAACATACTTGGACCCAATTAATGTAGCCTTCCACAAAAATATTTTT
GCCTTACATTATACTGT
Y
ATGAGAATTGACTCTTCCATATTTGCTCTAACTTATTCATTTCTGGTTCAATGTCTTATATAAAAAATCACACCTCAAAGTTT
CTGTTTAAATGCTATAC / Celera SNP ID: hCV11474667 / Public SNP ID: rs10150311 /
SNP Chromosome Position: 88976472 / SNP in Genomic Sequence: SEQ ID NO: 370 /
SNP Position Genomic: 74756 / Related Interrogated SNP: hCV16182835 / SNP
Source: dbSNP; Celera / Population(Allele,Count): Caucasian (T,153|C,69) /
SNP Type: INTRON /
Context (SEQ ID NO: 1436): /
GGTTGCAGTGAGCTAAGATGGTGCCATTGCACTCCAGCCTGGGCATCACAGAGAGAGTCTGTCTCAAAACAAAAAACAAAAAA
CATGAAAAAGCAACATA
Y
TTGGACCCAATTAATGTAGCCTTCCACAAAAATATTTTTGCCTTACATTATACTGTTATGAGAATTGACTCTTCCATATTTGC
TCTAACTTATTCATTTC / Celera SNP ID: hCV11474668 / Public SNP ID: rs10138002 /
SNP Chromosome Position: 88976415 / SNP in Genomic Sequence: SEQ ID NO: 370 /
SNP Position Genomic: 74699 / Related Interrogated SNP: hCV16182835 / SNP
Source: dbSNP; Celera; HapMap / Population(Allele,Count): Caucasian
(C,153|T,69) / SNP Type: INTRON /
Context (SEQ ID NO: 1437): /
AGTGATACTTAAGAAAATCTAAAGTACCTTGAAAAGGAATGTGGCTCCATGTGCATCAGGCATTTGTTTAAAAATGATACATG
TCTCCATGCCTTCTTAT
R
GCTGAAGGAGTTTTATTAATAATTATTCTGATTAAATTTCACTCCCAAGGCTAAATGCCAGTTATTGTTTGAAATAATGCCCC
ATCAGAAATCTTCCCAG / Celera SNP ID: hCV11474679 / Public SNP ID: rs2778936 /
SNP Chromosome Position: 88972303 / SNP in Genomic Sequence: SEQ ID NO: 370 /
SNP Position Genomic: 70587 / Related Interrogated SNP: hCV16182835 / SNP
Source: dbSNP; HapMap; HGBASE / Population(Allele,Count): Caucasian
(A,77|G,149) / SNP Type: INTRON /
Context (SEQ ID NO: 1438): /
CGGACATGAACCACCACACAGAGCCCCTAGACAGTTTCAAACAAGGATGAGAAAGTCCTCAACCACATGACACTTACACATAT
TAAAGTATATGTGATGA
R
TTACTTATCCCAGATTATAGTTATTTCAAAATTAAAACGATCCTATAAGACAATTAGTCATCTGCTTCATGCCCTGTAAAACA
GTCCCCACAATTTCTTT / Celera SNP ID: hCV11666712 / Public SNP ID: rs1864747 /
SNP Chromosome Position: 88974867 / SNP in Genomic Sequence: SEQ ID NO: 370 /
SNP Position Genomic: 73151 / Related Interrogated SNP: hCV16182835 / SNP
Source: dbSNP; Celera; HapMap; HGBASE / Population(Allele,Count): Caucasian
(A,85|G,35) / SNP Type: INTRON /
Context (SEQ ID NO: 1439): /
TGCCCAGGCTGTTCTCTAACTCCTGGGCTCAGGCAATCCTAGTGCTTGGATTCAAAATGTTGGGACTACGGACATGAACCACC
ACACAGAGCCCCTAGAC
R
GTTTCAAACAAGGATGAGAAAGTCCTCAACCACATGACACTTACACATATTAAAGTATATGTGATGAATTACTTATCCCAGAT
TATAGTTATTTCAAAAT / Celera SNP ID: hCV11666713 / Public SNP ID: rs1864746 /
SNP Chromosome Position: 88974799 / SNP in Genomic Sequence: SEQ ID NO: 370 /
SNP Position Genomic: 73083 / Related Interrogated SNP: hCV16182835 / SNP
Source: dbSNP; Celera; HGBASE / Population(Allele,Count): Caucasian
(A,85|G,35) / SNP Type: INTRON /
Context (SEQ ID NO: 1440): /
GTTCATGGCTGTAGTGCACTACGATCCCATCTGCGAATTCCCACCATATTCTACCTGGGAAACTGGGATACTAGGACCTGGTG
TCTTTAAAAAAAAGAAA
M
AAGTCTACCAGCCCAAGTAAAATCAATATTATTTATTGAAACTGTGCTAAGGACTGTACATGATTATCTCGTGGAATGTGAAC
ACCAAACCCATAAGGGA / Celera SNP ID: hCV11666722 / Public SNP ID: rs1864748 /
SNP Chromosome Position: 88955191 / SNP in Genomic Sequence: SEQ ID NO: 370 /
SNP Position Genomic: 53475 / Related Interrogated SNP: hCV16182835 / SNP
Source: dbSNP; Celera; HGBASE / Population(Allele,Count): Caucasian
(A,168|C,58) / SNP Type: TRANSCRIPTION FACTOR BINDING SITE;INTRON /
Context (SEQ ID NO: 1441): /
GAACCAGCTTGATAGTTTCCCATTGGCCAAATTTGAGACCAGTGGAGCATCAAAATTTGATACTGCATGTTTTTACATACATT
CAAGAACCCAAATATAC
S

TGGATCATCTGAGGTCAGGAGTTTGAGACCAGTCTGGCCAACATGGTGAAACCCCATGTCTACTAAAAAATACAAAAATTAGC
CAGGCGTGGTGGCGTGC / Celera SNP ID: hCV11666724 / Public SNP ID: rs1864744 /
SNP Chromosome Position: 88951006 / SNP in Genomic Sequence: SEQ ID NO: 370 /
SNP Position Genomic: 49290 / Related Interrogated SNP: hCV16182835 / SNP
Source: dbSNP; Celera; HGBASE / Population(Allele,Count): Caucasian
(C,149|G,73) / SNP Type: INTRON /
Context (SEQ ID NO: 1442): /
GCCGCGTTTCTGGACGGACGCGCTCTAAAAGGACCGGTCCCTCGGCTCCCGAAGGGAACATTTAAAGGGACCAGCGCGTGCCC
TACTGGCGGTGGAGAGG
Y

TGGGCGGGCCCCATAGGATGCCCGCTTTGGAGCCACAGGACCTCAAAATACGCTCCTTACAACGTGTTTTATAGAGCGAAGTC
TCGCTATGCCACCCAGG / Celera SNP ID: hCV27202543 / Public SNP ID: rs7146241 /
SNP Chromosome Position: 89017966 / SNP in Genomic Sequence: SEQ ID NO: 370 /
SNP Position Genomic: 116250 / Related Interrogated SNP: hCV16182835 / SNP
Source: dbSNP; Celera; HapMap; ABI_Val / Population(Allele,Count): Caucasian
(T,77|C,149) / SNP Type: ESS;SILENT RARE CODON;SILENT MUTATION;INTRON /
Context (SEQ ID NO: 1443): /
GCTTCTTTTAATGATACTATAATGCTGAATTTATTTATGAAAAAGAAACAATACAGGTTGAGGTGGGAAAAGCCCCAAGGAAA
CGGAGTAAGTCACTGGC
R

GTCACAGGACTTGAATTCCCTGGGGACAGTGCTGTGGGGTCCCCTGCTCGCCATCCCAGCACCTCAGTCTGAGTATAGATAAT
ACTGCATTTTTGTAAAG / Celera SNP ID: hCV27520559 / Public SNP ID: rs3814855 /
SNP Chromosome Position: 88933360 / SNP in Genomic Sequence: SEQ ID NO: 370 /
SNP Position Genomic: 31644 / Related Interrogated SNP: hCV16182835 / SNP
Source: dbSNP; HapMap; HGBASE / Population(Allele,Count): Caucasian
(G,164|A,58) / SNP Type: UTR3;INTRON /
Context (SEQ ID NO: 1444): /
AGAATGTGTTGGCGCTGATTGCTCTGGACTGACCATACCAGGTACACAGTATGCCTTTTCAATAGAGAGCTTCAAGTCATTTT
CCTTTCAAGGAAGTTTT
Y

AGAATGTGTTATAGCTAGAATATACAATTTGTTATATAGTTTTTGGAATTTGTTCTTTGCCAATGTTTTGCCTTAGATCATAC
TGTTAGAAGAATGTTTT / Celera SNP ID: hCV29385782 / Public SNP ID: rs7141608 /
SNP Chromosome Position: 88980389 / SNP in Genomic Sequence: SEQ ID NO: 370 /
SNP Position Genomic: 78673 / Related Interrogated SNP: hCV16182835 / SNP
Source: dbSNP; HapMap; ABI_Val / Population(Allele,Count): Caucasian
(T,77|C,149) / SNP Type: UTR5;INTRON /
Context (SEQ ID NO: 1445): /
TAGAACCTCGATCTGGCCTGTAAGAATATAGTCAATGAATTTTACTCAATGAATGTTGAAAGAACGAAGTCAAGAGCCGGACA
TGGTGGCACATGCCTAT
Y

GCGGGAGGATCGCTTGAGCCTAGGAATTTGAGACCAACCTGGGCAACATAGCTAGACCTACCTTGACTCTAAAAAAATTAAAA
TGAACAAAAAGAAAGA / Celera SNP ID: hCV30414829 / Public SNP ID: rs10134008 /
SNP Chromosome Position: 89015527 / SNP in Genomic Sequence: SEQ ID NO: 370 /
SNP Position Genomic: 113811 / Related Interrogated SNP: hCV16182835 / SNP
Source: dbSNP; HapMap / Population(Allele,Count): Caucasian (C,92|T,28) / SNP
Type: INTRON /
Context (SEQ ID NO: 1446): /
CCCCTTTGTTTTCTTCCAATATCTTTGAGTGGGTTTTTTTAGAACACAATTTTTGAGGTATAATTGGCATACGAATAAGCTGT
TCAAATTTCAAGTGTAC
M

ATTTAGACACATATATATATCCATGAAACCATCACTATAAAATGAGGAACATACTGACCAACTGCAAAAGTTTCCTTGCACCC
CTCATAATCTTTCTCTC / Celera SNP ID: hCV29729918 / Public SNP ID: rs10143767 /
SNP Chromosome Position: 88981211 / SNP in Genomic Sequence: SEQ ID NO: 370 /
SNP Position Genomic: 79495 / Related Interrogated SNP: hCV16182835 / SNP
Source: dbSNP; HapMap / Population(Allele,Count): Caucasian (A,164|C,56) /
SNP Type: INTRON;PSEUDOGENE /
Context (SEQ ID NO: 1447): /
GCAAATGCCATTATTTCATTCCTGAGTAGTATTCCATGGTGTGTGCGTGTTTGTGTGTGTCACATTTTCCCTTATCCACTAGT
TGGTTGATGGACATTTA
R

GCTGGTTCCATATTTTTTGCAATTGTGAATTGTGCTACTATAAACATGTGTGTGCAAATGTCTTTTTCATACAGTGGCTTCTTT
TCCTCCTAGTAGATACC / Celera SNP ID: hCV30414828 / Public SNP ID: rs7144432 /
SNP Chromosome Position: 88993414 / SNP in Genomic Sequence: SEQ ID NO: 370 /

SNP Position Genomic: 91698 / Related Interrogated SNP: hCV16182835 / SNP Source: dbSNP; HapMap; ABI_Val / Population(Allele,Count): Caucasian (G,39|A,81) / SNP Type: INTRON /
Context (SEQ ID NO: 1448): /
GTGTCTTGGTTTCCTCATCTACAAAATAGAGATAATAGCACCTACATCACAGGATTTGTTATGAGAATTAAATTAATAAAGTA
CTTGGGACAGTGTCTAA
Y
ATACAGTAAAAAACATAAGTGGTTGTTATTATATGTTATTGTATTATCCCTCAAATACTTTAATGGAAGCAACTAAGGATGGA
TGAATGGATGGATGGAT / Celera SNP ID: hCV32095402 / Public SNP ID: rs11159857 /
SNP Chromosome Position: 88977140 / SNP in Genomic Sequence: SEQ ID NO: 370 /
SNP Position Genomic: 75424 / Related Interrogated SNP: hCV16182835 / SNP Source: dbSNP; HapMap / Population(Allele,Count): Caucasian (C,85|T,35) / SNP Type: INTRON /
Context (SEQ ID NO: 1449): /
GCTGAAATTACAGGCATGAGCCACTGTGCCTGGCTTTTACTGGCGTAAGTGAACAGACATGTTAGTGAACCCTTTGGCAAAGA
ACTGTGGGCAGCCTCTA
S
GACTTGAGAGTGGCTTCCAGATGATAGCCAGAAAAAAAATCTGGGTCCTTACTCATAAAGCCACAAGGAAATGAATTATTTTTT
TTTTTCCTGAGACAGGG / Celera SNP ID: hCV32095430 / Public SNP ID: rs11159859 /
SNP Chromosome Position: 89027891 / SNP in Genomic Sequence: SEQ ID NO: 370 /
SNP Position Genomic: 126175 / Related Interrogated SNP: hCV16182835 / SNP Source: dbSNP / Population(Allele,Count): Caucasian (G,70|C,152) / SNP Type: INTERGENIC;UNKNOWN /
Context (SEQ ID NO: 1450): /
AAAACTTGTCAGCATAGTAACACCATAATGAAATCATATTCAAATCACTCCCCCCTTGTAGTTTTTTTCTTTTTGAGCCAATT
ACACTTAAAATGTCATT
R
ATGAAGATGTGAAGTCCAGGGTTTACAAAATATTTTTTATCTTCTTAGTATTCAGTGCGAGGAAATAGAAATGCTCATAAAAC
ATTTTTATTGTATACTG / Celera SNP ID: hCV32095431 / Public SNP ID: rs11629164 /
SNP Chromosome Position: 89031084 / SNP in Genomic Sequence: SEQ ID NO: 370 /
SNP Position Genomic: 129368 / Related Interrogated SNP: hCV16182835 / SNP Source: dbSNP; HapMap; ABI_Val / Population(Allele,Count): Caucasian (G,36|A,84) / SNP Type: TRANSCRIPTION FACTOR BINDING SITE;INTRON /
Context (SEQ ID NO: 1451): /
TAAGCTAGAAAAGAAATGGTTCATGTGGTTCTGCTGTCCAGCAGAAGGTAAAGCTGGGAAGGCAGGATTTAACCAACTTGTAG
GAGGCCTTTAATCCCAG
R
GTAATGAACTTGGGCTAGTAGAAAGCATGAAAATACCCATGAGCACGTGGAAGGAGAAGGAGAGAGGTAAGAGGAAACTGATA
TTCTAGGGAGATTAATC / Celera SNP ID: hCV32095396 / Public SNP ID: rs11845147 /
SNP Chromosome Position: 88969148 / SNP in Genomic Sequence: SEQ ID NO: 370 /
SNP Position Genomic: 67432 / Related Interrogated SNP: hCV16182835 / SNP Source: dbSNP; HapMap / Population(Allele,Count): Caucasian (G,85|A,35) / SNP Type: INTRON /
Context (SEQ ID NO: 1452): /
AAAGGTCTTGTTCTTAACCACGACAAATCTACAGTTATAGATATTTCTAGTATTTTTATTCATTTAGTATGTTACATGACAGT
CCACAAAACACAATGGT
Y
GAGAGCAAGGACCCAGGAGCCAGCCTGCTTGAGTCCCAGTTCCAGTATATACTAGCAGTGTGACCTTGGGCAAGGTGTTCAAC
CCTTCTGTGTCTTGGTT / Celera SNP ID: hCV32095401 / Public SNP ID: rs11847417 /
SNP Chromosome Position: 88976950 / SNP in Genomic Sequence: SEQ ID NO: 370 /
SNP Position Genomic: 75234 / Related Interrogated SNP: hCV16182835 / SNP Source: dbSNP; HapMap / Population(Allele,Count): Caucasian (C,153|T,69) /
SNP Type: INTRON /
Context (SEQ ID NO: 1453): /
CAACATGAATTAGAAAATCAAATAGAACTGAACAAATACATTTGTTGAAATTGCATCATAATTAGTTTTTCACAAATTATAGA
CCATAGAAGAGAGTAGC
R
AGAACTTTATCTAAAGGTACAAACAGCTGAAAAGAATGGTGGGCTTAGGCCGGGCGTGGTGGATTGTGCCTGTAATCCCAGCA
CTTTGGGAGGCTGAGGC / Celera SNP ID: hCV32095415 / Public SNP ID: rs12050316 /
SNP Chromosome Position: 88988441 / SNP in Genomic Sequence: SEQ ID NO: 370 /
SNP Position Genomic: 86725 / Related Interrogated SNP: hCV16182835 / SNP Source: dbSNP; HapMap / Population(Allele,Count): Caucasian (G,81|A,137) /
SNP Type: INTRON /

Context (SEQ ID NO: 1454): /
CTGCCACCACGCCCGGTTAATTTTTTTTTTTTTTTTTTGAGACATAGTTTTGCTCTTGTTGCCCAGGCTGGAGTGCAATGGCATG
ATCTTGACTCAATGTAA
S
CTCCACCTCTCAGGTTCAAGCGATTCTCCTGCCTCAGCCTCCTGACTAGCTGGGATTACAGGCATGCACCACCATGGCCAGCA
ATATTGTATTTTTAGTA / Celera SNP ID: hCV32095422 / Public SNP ID: rs2033418 /
SNP Chromosome Position: 89005675 / SNP in Genomic Sequence: SEQ ID NO: 370 /
SNP Position Genomic: 103959 / Related Interrogated SNP: hCV16182835 / SNP
Source: dbSNP; HapMap; HGBASE / Population(Allele,Count): Caucasian
(C,37|G,71) / SNP Type: INTRON /
Context (SEQ ID NO: 1455): /
TACTTGGGACAGTGTCTAACATACAGTAAAAAACATAAGTGGTTGTTATTATATGTTATTGTATTATCCCTCAAATACTTTAA
TGGAAGCAACTAAGGAT
R
GATGAATGGATGGATGGATGGATACACAGACAGATAGATTTTTTTTTTTTTTTTTGAGACAGGGTCTTGCTCTATAGCCCAGGCT
GGAGTGCAATGGCGTAA / Celera SNP ID: hCV32095404 / Public SNP ID: rs4301952 /
SNP Chromosome Position: 88977221 / SNP in Genomic Sequence: SEQ ID NO: 370 /
SNP Position Genomic: 75505 / Related Interrogated SNP: hCV16182835 / SNP
Source: dbSNP; HapMap; HGBASE / Population(Allele,Count): Caucasian
(G,85|A,35) / SNP Type: INTRON /
Context (SEQ ID NO: 1456): /
GTTATGAGAATTAAATTAATAAAGTACTTGGGACAGTGTCTAACATACAGTAAAAAACATAAGTGGTTGTTATTATATGTTAT
TGTATTATCCCTCAAAT
M
CTTTAATGGAAGCAACTAAGGATGGATGAATGGATGGATGGATGGATACACAGACAGATAGATTTTTTTTTTTTTTTTGAGACA
GGGTCTTGCTCTATAGC / Celera SNP ID: hCV32095403 / Public SNP ID: rs4390529 /
SNP Chromosome Position: 88977197 / SNP in Genomic Sequence: SEQ ID NO: 370 /
SNP Position Genomic: 75481 / Related Interrogated SNP: hCV16182835 / SNP
Source: dbSNP; HapMap; ABI_Val / Population(Allele,Count): Caucasian
(A,84|C,34) / SNP Type: TRANSCRIPTION FACTOR BINDING SITE;INTRON /
Context (SEQ ID NO: 1457): /
GACCTTGTCTGCAATATCTTTAATTTTGTTTTTCTCACTTTTCCTGCAATATCTAAAGCCTCAAGGACCTTTCAGTTGTCAGT
GACCCAGGGAATTCTGT
Y
AGCTGGCTGAGATGTTGTCTCTTGTTGTGAGTGCAAACATTAGTCAGACCTGTCTGAAGCCCTCTCCTACCCTTGTAGCTCA
TGAATGAGACCCTTACT / Celera SNP ID: hDV70886264 / Public SNP ID: rs17124700 /
SNP Chromosome Position: 88905629 / SNP in Genomic Sequence: SEQ ID NO: 370 /
SNP Position Genomic: 3913 / Related Interrogated SNP: hCV16182835 / SNP
Source: dbSNP; HapMap / Population(Allele,Count): Caucasian (C,91|T,29) / SNP
Type: INTRON /
Context (SEQ ID NO: 1458): /
ATTACTCGGTAGGTCCTGATCCAGATTCCAACCACCTCTCTCCAACCCTATCCAACTCCAGGTGGCTACCATCTGTACCTGAA
CCACTCCCCTCAAATCA
Y
TCTCCAAAGGGCTCACTCTTCACAGAAACAATCGTACCACACCTGTTTACACCCTTCATTAACTTCCCCACTACCCTCAGGAT
AAATCTAAATCCTTTAA / Celera SNP ID: hDV71008979 / Public SNP ID: rs17798341 /
SNP Chromosome Position: 89014545 / SNP in Genomic Sequence: SEQ ID NO: 370 /
SNP Position Genomic: 112829 / Related Interrogated SNP: hCV16182835 / SNP
Source: dbSNP; HapMap / Population(Allele,Count): Caucasian (T,92|C,28) / SNP
Type: INTRON /
Context (SEQ ID NO: 1459): /
CAACCATTTTATGGATAGATTCCCCTGGACATCAGAGAAATGATTATCTTGGCAGTGTGTTTGTTGAAATGGAGAAGCATGTC
TGCCTCAGTAGCAAGTG
R
CTGCTGTGACTGTAATTCCCAACACTTAGGGCTTTTGATCCAAGTATGTCTATGCCATGTGATAATCACTATTGGAAAACACT
TGTCCATTCAGCCCACA / Celera SNP ID: hCV32095429 / Public SNP ID: rs12436642 /
SNP Chromosome Position: 89025376 / SNP in Genomic Sequence: SEQ ID NO: 370 /
SNP Position Genomic: 123660 / Related Interrogated SNP: hCV16182835 / SNP
Source: dbSNP; HapMap; ABI_Val / Population(Allele,Count): Caucasian
(A,76|G,150) / SNP Type: INTERGENIC;UNKNOWN /
Context (SEQ ID NO: 1460): /
GCTCACTACAGCCTCAACCTCCTGGACTCAAGTGATCCTTGAGCCTCAGCCTCCTGAGTAGTTGGGACCACAGGTGTACACCA
CCACAGCTGGCTAAATT

K
TTTTTAATTTTTTTGTAGAAACAGGGTCTCTCTATGTTGCCTGGGCTGGTCTCAAACTCCTGAGCTCAAGCGATCCTCCCACTT
CGGCCATCCAAAGTGCT / Celera SNP ID: hDV77012938 / Public SNP ID: rs4514599 /
SNP Chromosome Position: 88977427 / SNP in Genomic Sequence: SEQ ID NO: 370 /
SNP Position Genomic: 75711 / Related Interrogated SNP: hCV16182835 / SNP
Source: CDX; dbSNP / Population(Allele,Count): Caucasian (T,80|G,34) / SNP
Type: INTRON //

Gene Number: 34 / Gene Symbol: KIFAP3 - 22920 / Gene Name:
kinesin-associated protein 3 / Chromosome: 1 / OMIM NUMBER: 601836 / OMIM
Information: // Genomic Sequence (SEQ ID NO: 371): // / SNP Information /
Context (SEQ ID NO: 1461): /
GCTTCAATCACCTGGAATATTAACTAATATGGAATACATTTCTTAATGAAGCAATATCAGTGAGTTATTGCTATTATAAAATC
TTTATTCAGAATTATCT
Y
GGTAGGGGAATAAAGTGTTTAATTTTTTTTCAAAATATTCTAACTTGAAAATGGCAAAAATGAACTATTTCAAGAAAAAAGCTTT
GTAAGTTACCATATCAT / Celera SNP ID: hCV25921520 / Public SNP ID: rs12132173 /
SNP Chromosome Position: 169993466 / SNP in Genomic Sequence: SEQ ID NO: 371 /
SNP Position Genomic: 112996 / Related Interrogated SNP: hCV11975250 / SNP
Source: Applera / Population(Allele,Count): Caucasian (C,39|T,1) African
American (C,34|T,0) total (C,73|T,1) / SNP Type: INTRON / SNP Source:
dbSNP; HapMap / Population(Allele,Count): Caucasian (C,197|T,27) / SNP Type:
INTRON /
Context (SEQ ID NO: 1462): /
GTTTACATTAAATGGTTTGTGAATTCTCTGGAGAATATTTAAATGCTACACTCTCAATTTAAGAAATCTGAGTAAGAAAGAAA
TAAAGAGCTGGAAAATA
Y
ATGAAGTCTTAAGTTTTGTATAAATAACTGTAACTTATTCCTCTATTATAATTAAACATGTTTTATAGTAATCACCATTCAAT
AGTAGTTCTTCCAAGTT / Celera SNP ID: hCV25922175 / Public SNP ID: rs12120229 /
SNP Chromosome Position: 170007355 / SNP in Genomic Sequence: SEQ ID NO: 371 /
SNP Position Genomic: 126885 / Related Interrogated SNP: hCV11975250 / SNP
Source: Applera / Population(Allele,Count): Caucasian (C,1|T,39) African
American (C,0|T,34) total (C,1|T,73) / SNP Type: INTRON / SNP Source:
dbSNP; HapMap / Population(Allele,Count): Caucasian (T,199|C,27) / SNP Type:
INTRON /
Context (SEQ ID NO: 1463): /
AAACACACTTTTAGGAAAAAGAGAACATATATGTAAGAGGTGATCCAGTGACTACCTTTCTGGGAGAAGAGAGCTGTGAGAAG
AAATGGCTTTTCCTAAC
R
TAATAATAGTCACACAATACTGCTCTAGCAGCCTTATCCATAACAGAAAGAAATAACTTAGTGATTATACAAAAGTGAAATAC
TTAGCACAAACCTGGTT / Celera SNP ID: hCV574764 / Public SNP ID: rs638486 / SNP
Chromosome Position: 169951748 / SNP in Genomic Sequence: SEQ ID NO: 371 /
SNP Position Genomic: 71278 / Related Interrogated SNP: hCV11975250 / SNP
Source: Applera / Population(Allele,Count): Caucasian (A,1|G,29) African
American (A,0|G,32) total (A,1|G,61) / SNP Type: INTRON / SNP Source:
dbSNP; HapMap; HGBASE / Population(Allele,Count): Caucasian (A,27|G,199) / SNP
Type: INTRON /
Context (SEQ ID NO: 1464): /
GCCAAAATACATATAACACATACTGGTCCAGTGACTCTGCCCTTTAGGAGAATGGAAGTTCTGAAATACAAGTTTCAGGTTAG
CATTTGTACTGAGCGAT
Y
CTGGACAAAATTTGTTGTTTTGGGGTCCTTCTATATAAAAACTCTGTCTATAGTTTCTCAAAGTTGGTTGAAATTTTTAAGTC
TACATTTTATTTTGAAA / Celera SNP ID: hCV574681 / Public SNP ID: rs575147 / SNP
Chromosome Position: 169892284 / SNP in Genomic Sequence: SEQ ID NO: 371 /
SNP Position Genomic: 11814 / Related Interrogated SNP: hCV11975250 / SNP
Source: dbSNP; HapMap; HGBASE / Population(Allele,Count): Caucasian
(T,26|C,198) / SNP Type: UTR5;INTRON /
Context (SEQ ID NO: 1465): /
AAGCACTAAGGCTAACAAGTAATCATGTATCTAAAGAACTCTCAATTATATAACTGTTGATTACATAGCTTGGATTTCAACTT
GTTCCTTCTTCATCTTT
Y
GCTTATTACCTGTCTTCAACCAGTCCTTATCTTTTTTTAGCAACTCCTCTGAGGCCAAATAGCAGTAGACTTAGGTTAAACTCA
AGTTAATCTTATGCTTG / Celera SNP ID: hCV574682 / Public SNP ID: rs590181 / SNP

Chromosome Position: 169892661 / SNP in Genomic Sequence: SEQ ID NO: 371 /
SNP Position Genomic: 12191 / Related Interrogated SNP: hCV11975250 / SNP
Source: dbSNP; HapMap; HGBASE / Population(Allele,Count): Caucasian
(T,28|C,198) / SNP Type: UTR5;INTRON /
Context (SEQ ID NO: 1466): /
ATCCACCTGATGGTTATAACTTTCATTGTACATTAACAAATGGATTTTTCTTTTTAAAATATGTCATGCACATTGTTGATAAA
AACCTGCAGTCCCAAGT
M
AGTGAAAGACTGGCCCTTGAGCACTGATGAGTCTGAGTTTCTGAACCTGTAAATCTCTGGTCTTGAACAGTATGGTCTTTAGG
TTACTATGGTAAAGATC / Celera SNP ID: hCV574683 / Public SNP ID: rs544008 / SNP
Chromosome Position: 169893330 / SNP in Genomic Sequence: SEQ ID NO: 371 /
SNP Position Genomic: 12860 / Related Interrogated SNP: hCV11975250 / SNP
Source: dbSNP; Celera; HapMap; HGBASE / Population(Allele,Count): Caucasian
(C,28|A,198) / SNP Type: UTR5;INTRON /
Context (SEQ ID NO: 1467): /
TGAGTTTATTTTCAAAGTAGTAAATCAAGGGTAAGGTTTTTAGTTATCTCATGCAAATCATCACTTCCCACATTGTACGCATT
CCTATACTCAGGTTTAC
Y
GCCAGTGTTGGCTTCTCTTAGAAAAGTGCACACAGGTCATGAAAACGTTTAGGGTGGAGTATTTTCATTCTGCGATGTGTGTT
GTCTTTGGGTTTGTGCC / Celera SNP ID: hCV574693 / Public SNP ID: rs601355 / SNP
Chromosome Position: 169903688 / SNP in Genomic Sequence: SEQ ID NO: 371 /
SNP Position Genomic: 23218 / Related Interrogated SNP: hCV11975250 / SNP
Source: dbSNP; HapMap; ABI_Val; HGBASE / Population(Allele,Count): Caucasian
(T,28|C,198) / SNP Type: INTRON /
Context (SEQ ID NO: 1468): /
CGTCTGGATGAGGGAAACACAGAGGCGCCCAGAAACCTGGAGAAACTGGGAACCGCAGAGCCCCACAGAGGGTGTCACATCCC
TGGCTCAGGAGCAACTA
S
GTCTGGGCTCTTGGAAGAGCCACAGCTCTTCCCTCCTTCTCGTTTCCCACAACATGGTGAGTGGGGGCGACGTGTTTCAGCCC
CGTTTGTGTTACAGCTC / Celera SNP ID: hCV574707 / Public SNP ID: rs565397 / SNP
Chromosome Position: 169913597 / SNP in Genomic Sequence: SEQ ID NO: 371 /
SNP Position Genomic: 33127 / Related Interrogated SNP: hCV11975250 / SNP
Source: dbSNP; HapMap; HGBASE / Population(Allele,Count): Caucasian
(C,28|G,198) / SNP Type: INTRON /
Context (SEQ ID NO: 1469): /
TCCTGCCTTGGCCTCTCAAAGTGCTGGCATTAGAGCTGTGAGCCACCATGCCCGACCTCTTTTTATTTCTTAAAGTCAAATGA
AAATATAATATTGGGCT
Y
TTTGGTGAACTAGCTAAAGGTGAAATTGTGACGGTATGGGAGAAAATAGATGTCAATAGTGTAGAGTATGAGAAAAAGAAATA
ACTAAAATGATGAATCT / Celera SNP ID: hCV574726 / Public SNP ID: rs664962 / SNP
Chromosome Position: 169927576 / SNP in Genomic Sequence: SEQ ID NO: 371 /
SNP Position Genomic: 47106 / Related Interrogated SNP: hCV11975250 / SNP
Source: dbSNP; HapMap; HGBASE / Population(Allele,Count): Caucasian
(T,28|C,198) / SNP Type: INTRON /
Context (SEQ ID NO: 1470): /
CATAAATTCTAAAGTGTCGTGAAAGACTGATGGTTTGATTTTACATGGAATGTGCCATCCTTTTTGAAGTCCTACATCATTTT
GTTTACTAGTTAACCAC
Y
GAAACATTATGAAGCCTTAACTAGCCTATTATCAGAGCCTGTTCACTATCAGGAAAACTCTTTGTCACTGGCTTCCGTCACAC
CTTGTATCTGATTAACA / Celera SNP ID: hCV574743 / Public SNP ID: rs545963 / SNP
Chromosome Position: 169937418 / SNP in Genomic Sequence: SEQ ID NO: 371 /
SNP Position Genomic: 56948 / Related Interrogated SNP: hCV11975250 / SNP
Source: dbSNP; HapMap; HGBASE / Population(Allele,Count): Caucasian
(C,27|T,195) / SNP Type: INTRON /
Context (SEQ ID NO: 1471): /
TATATGTAGTAAAAGAGTTAGCAGATATCAAAATTTTCAGCTTAAGAGGCTGTGTAGATGACAATGTTGTTATTCACAATGAA
TACAGATGGCAGGACTG
Y
TTTGCTTATCAGGAGAGGATGTTACCAATATTGAGTACCCTTTGGATGCGAGAATGATAAACTACCAGGTACTAAGTACCTCT
GTGTATTGGGAACTATT / Celera SNP ID: hCV574757 / Public SNP ID: rs654664 / SNP
Chromosome Position: 169946436 / SNP in Genomic Sequence: SEQ ID NO: 371 /
SNP Position Genomic: 65966 / Related Interrogated SNP: hCV11975250 / SNP
Source: dbSNP; HapMap; HGBASE / Population(Allele,Count): Caucasian

(C,27|T,199) / SNP Type: INTRON /
Context (SEQ ID NO: 1472): /
GCCCAACACTGTGAAAACAAAGTGAGAACACAAGTTATGGGCCACATTATTAGCCAGATTCTCATTCCCTTCTATACTCTATG
TCTTCTTCTTTCAAAGT
R
GAACACTCCCTTTGATGTCACAGAGGCTCCATGAAAAGATAATAGAAGTGGGAATATAAGCACACCTATATTAGGATAGAATA
GCATTAAGGTGATATAT / Celera SNP ID: hCV574785 / Public SNP ID: rs511609 / SNP
Chromosome Position: 169969444 / SNP in Genomic Sequence: SEQ ID NO: 371 /
SNP Position Genomic: 88974 / Related Interrogated SNP: hCV11975250 / SNP
Source: dbSNP; HapMap; HGBASE / Population(Allele,Count): Caucasian
(G,29|A,193) / SNP Type: INTRON /
Context (SEQ ID NO: 1473): /
TTATTTATAGGATAAATGAGGCCTATTAAAAATTGAGAAACAGTGTAGTAGTCTATGAGGAGTTGTGTTCTGAAACAGATGTGT
GAAATTCCATAACAGTT
Y
GGTAATAAACAGAACAACCAAACCAATTTATTTTTTAAAAAATCTACTTTAAGTTTCTTAAAGAAACTATCATAAAGGCAACC
TAGTTTCAAAAAAAATGG / Celera SNP ID: hCV574788 / Public SNP ID: rs629408 / SNP
Chromosome Position: 169970822 / SNP in Genomic Sequence: SEQ ID NO: 371 /
SNP Position Genomic: 90352 / Related Interrogated SNP: hCV11975250 / SNP
Source: dbSNP; HapMap; ABI_Val; HGBASE / Population(Allele,Count): Caucasian
(C,27|T,197) / SNP Type: INTRON /
Context (SEQ ID NO: 1474): /
AATGAGGCCTATTAAAAATTGAGAAACAGTGTAGTAGTCTATGAGGAGTTGTGTTCTGAAACAGATGTGTGAAATTCCATAACA
GTTCGGTAATAAACAGA
M
CAACCAAACCAATTTATTTTTTAAAAAATCTACTTTAAGTTTCTTAAAGAAACTATCATAAAGGCAACCTAGTTTCAAAAAAA
TGGAAAACACTTCATTT / Celera SNP ID: hCV574789 / Public SNP ID: rs629421 / SNP
Chromosome Position: 169970836 / SNP in Genomic Sequence: SEQ ID NO: 371 /
SNP Position Genomic: 90366 / Related Interrogated SNP: hCV11975250 / SNP
Source: dbSNP; HapMap; HGBASE / Population(Allele,Count): Caucasian
(A,27|C,197) / SNP Type: INTRON /
Context (SEQ ID NO: 1475): /
TTTTCACTTTACGCTTTTTAATTGAAATTAAATTAGTCACTTAACATACATAATGCAGTGTTTGTTTTCCAGTCTAGTAGCTT
TTCAGCATTCAGTTTTG
W
GGCTCATGGGAAATGTTACTTAGCATCAAGATGTGGTTTGATGAGTGAACAATGTCAGTATCAACTGTACTTAACAGAAGACA
GAGGGGCTGGGGTTAAT / Celera SNP ID: hCV8697995 / Public SNP ID: rs4519 / SNP
Chromosome Position: 169890574 / SNP in Genomic Sequence: SEQ ID NO: 371 /
SNP Position Genomic: 10104 / Related Interrogated SNP: hCV11975250 / SNP
Source: dbSNP; HapMap; ABI_Val; HGBASE / Population(Allele,Count): Caucasian
(T,28|A,198) / SNP Type: MICRORNA;UTR3 /
Context (SEQ ID NO: 1476): /
GATAATTCTCTTAGGAAAAGATTCTCTGAAGAAGGAATATTTGAACTGAGACTTGAAAGGTAAGAAGAAGCCAGTTATTTAAA
AAGTCAAAAGACTATTC
M
AGTTAAGCACAAAGGTGACAAAGTGCGAAAGAACTTGGTGAATGTCTGGGGAAGAGAAGGGAGTCCAGAGTGGGTAGAATTAA
CAAGCAATTATAACTGG / Celera SNP ID: hCV8698056 / Public SNP ID: rs488488 /
SNP Chromosome Position: 169907997 / SNP in Genomic Sequence: SEQ ID NO: 371 /
SNP Position Genomic: 27527 / Related Interrogated SNP: hCV11975250 / SNP
Source: dbSNP; HapMap; HGBASE / Population(Allele,Count): Caucasian
(C,8|A,110) / SNP Type: INTRON /
Context (SEQ ID NO: 1477): /
CTAACAGTGAAAATGGAAATAAATAGCCAAATTTCAGATCTATTCCAGAAATGAAATGAAAACAACAGGGCATGCAGATGGTT
TGCAGTGGAGAGAGAGG
S
TAATGGTGAAGAAAAGGGAGATAATAAGGAAGATTCCAAAGTTTAAAGCTTGAACTACTAGGTGGGATGGTGGTGACACATAC
TGAGACGAGAAGATTAA / Celera SNP ID: hCV8698071 / Public SNP ID: rs673789 /
SNP Chromosome Position: 169908455 / SNP in Genomic Sequence: SEQ ID NO: 371 /
SNP Position Genomic: 27985 / Related Interrogated SNP: hCV11975250 / SNP
Source: dbSNP; HapMap; HGBASE / Population(Allele,Count): Caucasian
(G,28|C,198) / SNP Type: INTRON /
Context (SEQ ID NO: 1478): /
TTGATAACATAAGAGCAATTTGGATTATAAATTAATATTTGACGAGACCTCAGTAAGAGCTGTATTTCCAAATGTAGCAATTT

CAATTCCCAGTCTATTT
K
AATAGAAAGCCAAAATGATACTTACAATAAACAGATTTTTAGTTGGTCCATCATGCTGAGAAATGTTTCTAATCATTTTCATC
AGCAATGGATCCTTAAA / Celera SNP ID: hCV11975488 / Public SNP ID: rs2057249 /
SNP Chromosome Position: 169952385 / SNP in Genomic Sequence: SEQ ID NO: 371 /
SNP Position Genomic: 71915 / Related Interrogated SNP: hCV11975250 / SNP
Source: dbSNP; HapMap; HGBASE / Population(Allele,Count): Caucasian
(G,27|T,199) / SNP Type: TRANSCRIPTION FACTOR BINDING SITE;INTRON /
Context (SEQ ID NO: 1479): /
GGAAACACAGATGATATACACCATAACACTCTAAGCTATAGAGTATTATACACATTTACAGTACTCAATTATATAAAACCTTT
TAGACCACAGTTATAAA
S
CACACCTTTCTGAGATGCCCCAAGTAAGATGATAAAGGCAGTTATGAACAAAACAGAGCATATACTAGAACAGTGATGAAACC
AAAGCAAATTTGTTGTC / Celera SNP ID: hCV15858911 / Public SNP ID: rs2806392 /
SNP Chromosome Position: 169926512 / SNP in Genomic Sequence: SEQ ID NO: 371 /
SNP Position Genomic: 46042 / Related Interrogated SNP: hCV11975250 / SNP
Source: dbSNP; HapMap; HGBASE / Population(Allele,Count): Caucasian
(G,28|C,198) / SNP Type: INTRON /
Context (SEQ ID NO: 1480): /
AATGTCTATGTTCCCCCAAAATTCTTATGTTGAAACATAATCTTGAATGTGATAATATTTGGGGCTGGGTCCTTTGAGAGGTG
GTTACGTCATGAAAGCA
R
AGCCATCATGTACGGGATTGGTGCCTGTGACAGTCTGTTGGCATGCTGCTAATACAGACATACCCAAGACGGGGTAATTTATA
AAGAAAAAAAGACTGGG / Celera SNP ID: hCV32141971 / Public SNP ID: rs12118305 /
SNP Chromosome Position: 169981815 / SNP in Genomic Sequence: SEQ ID NO: 371 /
SNP Position Genomic: 101345 / Related Interrogated SNP: hCV11975250 / SNP
Source: dbSNP; HapMap / Population(Allele,Count): Caucasian (G,93|A,9) / SNP
Type: INTRON /
Context (SEQ ID NO: 1481): /
TGCTCATACCTTGATTTCAGACTTCTAGGCTCCAGAAATGTGAGACAATACATTACCATGCCTGACTCGTATGTTTTATACCA
TCTGGTTTTGGTGCTTA
R
CAACCCTAGGAAATGACTACATGTGTGTTTCATACATGTGTTTGATTTGGAGCATTGCACATTACCATGCCGGACTCGTATGT
TTTATACCATCTGGTTT / Celera SNP ID: hCV32141903 / Public SNP ID: rs12131192 /
SNP Chromosome Position: 169882833 / SNP in Genomic Sequence: SEQ ID NO: 371 /
SNP Position Genomic: 2363 / Related Interrogated SNP: hCV11975250 / SNP
Source: dbSNP / Population(Allele,Count): Caucasian (A,198|G,28) / SNP Type:
INTERGENIC;UNKNOWN /
Context (SEQ ID NO: 1482): /
ATACACATGTGAGGGTTGGGCTACACTGTAAAATACATATTTAAAATAATATACTTTTTAACCTTAGGAAAAATATATCTTAC
TACGGATTGAGGTAAAA
M
AAGTGTGAAAGTCAGTGCTCTAAATAAATTTTTCTTGTCCTTTTCAAATTCATGTCCTTTATAACACAGTTTCCTTACTATAT
CTTTTGTATAAGCATTG / Celera SNP ID: hCV32141968 / Public SNP ID: rs12124907 /
SNP Chromosome Position: 169980811 / SNP in Genomic Sequence: SEQ ID NO: 371 /
SNP Position Genomic: 100341 / Related Interrogated SNP: hCV11975250 / SNP
Source: dbSNP; HapMap / Population(Allele,Count): Caucasian (C,199|A,27) /
SNP Type: INTRON /
Context (SEQ ID NO: 1483): /
TGGATAAGCCAAAAAAATCTTTAAACACTTTTAAAAACGTAACACTATAAAAATCCCCAAAAAGACTGCATCTAACAATTAAG
TAAAGCCAAGTTTTGTA
R
GTCCAATTCAATTTGCAGATAATCACATTTGCTGAAGCCAATGGCTATGATAGATCTCCCAGATGGTAGCATTAAGTTACCAG
ACATAAGAAACTATCTG / Celera SNP ID: hDV70695296 / Public SNP ID: rs16862919 /
SNP Chromosome Position: 169979101 / SNP in Genomic Sequence: SEQ ID NO: 371 /
SNP Position Genomic: 98631 / Related Interrogated SNP: hCV11975250 / SNP
Source: dbSNP; HapMap / Population(Allele,Count): Caucasian (A,199|G,25) /
SNP Type: INTRON /
Context (SEQ ID NO: 1484): /
TGAACTATGTTCTTGTTCCTCATTTTCAGTTCGGTACGAGTATCCTCAGCAAGATTCAGAAGCAAATAAAGAGCAACTAGAAA
AGTAAAATATATGAGAG
W
TAAAGAATTAGTATACTGAATTAATAATTTAGAAAATCATTTCTACTCACCAAGGCAACAGAGATAGCCCTAAAAAGGTAATA

TATTATTTTGAAAAATTT / Celera SNP ID: hDV70695328 / Public SNP ID: rs16862956 / SNP Chromosome Position: 169993762 / SNP in Genomic Sequence: SEQ ID NO: 371 / SNP Position Genomic: 113292 / Related Interrogated SNP: hCV11975250 / SNP Source: dbSNP; HapMap / Population(Allele,Count): Caucasian (A,98|T,8) / SNP Type: INTRON;PSEUDOGENE /
Context (SEQ ID NO: 1485): /
TTAAATTGAAGCCTCAGGGTTTCCAGAGGCCAAAATTAATATAAAAGTAAGGTCCACTTTTGCACAAAGCAGAGTTAAAAATG
TATGATATTCTGCAAAA
Y

AGGAATAAAGTTTTCACTTTTTTAAAAGACATAATAAACACTTGATTCAAAGGAAAGTTACCAGCATGCCTAGTTGATCTGCAA
AACATATCATGGTTGAA / Celera SNP ID: hDV70695338 / Public SNP ID: rs16862968 / SNP Chromosome Position: 170002561 / SNP in Genomic Sequence: SEQ ID NO: 371 / SNP Position Genomic: 122091 / Related Interrogated SNP: hCV11975250 / SNP Source: dbSNP; HapMap / Population(Allele,Count): Caucasian (T,199|C,27) / SNP Type: INTRON //

Gene Number: 35 / Gene Symbol: ATF6 - 22926 / Gene Name: activating transcription factor 6 / Chromosome: 1 / OMIM NUMBER: 605537 / OMIM Information: // Genomic Sequence (SEQ ID NO: 372): // / SNP Information /
Context (SEQ ID NO: 1486): /
GTCTTCTAGTTCTCAGATGTCTCCCCTTTCCTTATATGGTGAAAACTCTAATAGTCTCTCTTCAGCGGAGCCACTGAAGGAAG
ATAAGCCTGTCACTGGT
Y

CTAGGAACAAGACTGGTATTACTCTATCTCCTAACTTCTGTTATTTCTATTTCAGATTGAGCTTAGGTTCCATTTCTTAAAAG
AAAATGCTGGATTAAAT / Celera SNP ID: hCV25631989 / Public SNP ID: rs1135983 / SNP Chromosome Position: 161761312 / SNP in Genomic Sequence: SEQ ID NO: 372 / SNP Position Genomic: 35228 / SNP Source: Applera /
Population(Allele,Count): Caucasian (C,35|T,5) African American (C,28|T,4) total (C,63|T,9) / SNP Type: MISSENSE MUTATION;ESS;TRANSCRIPTION FACTOR BINDING SITE / SNP Source: dbSNP; Applera / Population(Allele,Count): Caucasian (C,111|T,5) / SNP Type: MISSENSE MUTATION;ESS;TRANSCRIPTION FACTOR BINDING SITE //

Gene Number: 36 / Gene Symbol: TRPC4AP - 26133 / Gene Name: transient receptor potential cation channel, subfamily C, member 4 ass / ociated protein // Chromosome: 20 / OMIM NUMBER: 608430 / OMIM Information: // Genomic Sequence (SEQ ID NO: 373): // / SNP Information /
Context (SEQ ID NO: 1487): /
CCCATTTCAAAAGTTAAATTCCCAAACCATTAAGCAGTAGTAACAGCAGTAGTAATAATAAATAGCTACTTACATCAACACCT
CCAAACAAGTCAAAAAT
R
TAAGTATTTGGATAAGTGGTTTCTTGGGTAAGTTTCCTCTCTTCAGTAACAAATGCCATAGCCTCCATGGAGAGAAGAGGAGA
AATTTCCTAGTTTTTTT / Celera SNP ID: hCV11656983 / Public SNP ID: rs1998233 / SNP Chromosome Position: 33657126 / SNP in Genomic Sequence: SEQ ID NO: 373 / SNP Position Genomic: 76919 / Related Interrogated SNP: hCV1825046 / SNP Source: Applera / Population(Allele,Count): Caucasian (A,28|G,8) African American (A,35|G,3) total (A,63|G,11) / SNP Type: ESS SYNONYMOUS;SILENT MUTATION;INTRON / SNP Source: dbSNP; HapMap; ABI_Val; HGBASE / Population(Allele,Count): Caucasian (G,62|A,164) / SNP Type: ESS SYNONYMOUS;SILENT MUTATION;INTRON /
Context (SEQ ID NO: 1488): /
CCTGAATGGGATAGGAAGGAAAAAGTATTAAGACAAATGTGCTTGAAAAATCTAGCAAAACCTCGTGCTCAGTACACACGATA
ATGATATTCAGGACAAG
Y
ATCAGTACTGTCAGTAAGGAGGGCCAGGGTCTGAAGAAAACATCCGTGTATCCCCTATGCCCAGCACACACAAACCCACTTTC
TAACTTTGCTCATTAAT / Celera SNP ID: hCV1347930 / Public SNP ID: rs3736802 / SNP Chromosome Position: 33604042 / SNP in Genomic Sequence: SEQ ID NO: 373 / SNP Position Genomic: 23835 / Related Interrogated SNP: hCV1825046 / SNP Source: Applera / Population(Allele,Count): Caucasian (C,16|T,16) African American (C,17|T,21) total (C,33|T,37) // / SNP Type: INTRON / SNP Source: dbSNP; Celera; HapMap; HGBASE / Population(Allele,Count): Caucasian (T,104|C,122) / SNP Type: INTRON /
Context (SEQ ID NO: 1489): /

CCACTAGTATAAGGGGTAATCAAGTTTTATACTTGAATACATCTGAGTAACAAGACATAATTTAACACTTTCTTTTAAAAAAG
CTCTGTATATTATTCAG
K
TTTGAGAATCCAATGGTCTGAGACACCCTTCCCCATCTGGTCAGCTAGAGGACACAGCTCACCTTTTTAACACCCAAAATATC
TTCAATGAGGGTTGCTG / Celera SNP ID: hCV15876219 / Public SNP ID: rs2281626 /
SNP Chromosome Position: 33637606 / SNP in Genomic Sequence: SEQ ID NO: 373 /
SNP Position Genomic: 57399 / Related Interrogated SNP: hCV1825046 / SNP
Source: dbSNP; HapMap; ABI_Val; HGBASE / Population(Allele,Count): Caucasian
(T,61|G,163) / SNP Type: INTRON /
Context (SEQ ID NO: 1490): /
TGCTGGGAAGGCAAAACAAGTGAACTGTGCTGAGTGTCACCATTTGTGACGGGCAGCACTGAGCCACCTTCACAGCTTGGCTG
CCCCTCCCTAGGGATGC
R
CTGTTCACCATCCAGTGGAACATCCGTGCCTTCAATGCCGTCAAGAACTGGTCATGGATGAAGCTCTTTTTCAAGATGAAGCC
GCTGCTGCGCTCGGCGC / Celera SNP ID: hCV16013546 / Public SNP ID: rs2425012 /
SNP Chromosome Position: 33581955 / SNP in Genomic Sequence: SEQ ID NO: 373 /
SNP Position Genomic: 1748 / Related Interrogated SNP: hCV1825046 / SNP
Source: dbSNP; HapMap; ABI_Val; HGBASE / Population(Allele,Count): Caucasian
(G,125|A,101) / SNP Type: ESE;SILENT RARE CODON;SILENT MUTATION /
Context (SEQ ID NO: 1491): /
CAGTGGCCCGGCTGACCAAGGAGAAGAAGGCGTTGCAGGAGGCCCACCAACAGGCCCTGGGTGACCTGCAGGCCGAGGAGGAC
CGTGTGAGCGCGCTGAC
R
AAGGCCAAGCTCCGGCTGGAGCAACAGGTGGAGGACGTGAGTCAGGGCCACCCCGAGACTGGGTGGCGGGGCCGGGGTGCCGG
CTGGCTTGGGCCATGGC / Celera SNP ID: hCV2142616 / Public SNP ID: rs3746438 /
SNP Chromosome Position: 33584289 / SNP in Genomic Sequence: SEQ ID NO: 373 /
SNP Position Genomic: 4082 / Related Interrogated SNP: hCV1825046 / SNP
Source: dbSNP; Celera; HGBASE / Population(Allele,Count): Caucasian
(G,124|A,102) / SNP Type: ESS;ESE;TRANSCRIPTION FACTOR BINDING SITE;SILENT
MUTATION /
Context (SEQ ID NO: 1492): /
ACTCCCCGTCGTCTCTAACGTAAAACCTGTGCCTAAAGCCTGGCAGACCAGGACTCCAGGTGAACTTCTGGACAACCCAGCA
CACTCCACAGAGCCCTG
K
GTTAACTTTACCTTTGCATCGGTGTTGATATATTTATTGAATCTCTTGAATGCATCAACGTTGAACTTCATCAGCTCCCCCAG
GAGGTCAAAGTAACTCT / Celera SNP ID: hCV25619953 / Public SNP ID: rs6060151 /
SNP Chromosome Position: 33594226 / SNP in Genomic Sequence: SEQ ID NO: 373 /
SNP Position Genomic: 14019 / Related Interrogated SNP: hCV1825046 / SNP
Source: Applera / Population(Allele,Count): Caucasian (G,6|T,18) African
American (G,21|T,7) total (G,27|T,25) / SNP Type: UTR5;INTRON / SNP Source:
dbSNP; HapMap / Population(Allele,Count): Caucasian (G,83|T,143) / SNP Type:
UTR5;INTRON /
Context (SEQ ID NO: 1493): /
GATTTTCAGGCACACATAAAATAAGCCCGATTCCCCCACTGTCTGAAAGCAGCTCTTATGTCTGCCATTCTACTCGAATATTC
CCATCTTAAACATTGCA
K
ACCTGGTAGGGGAGATAAACAGAGCCCCAGTGGCAGGGGTACTTACACAGACACAGGTATTATACAGGATACTCAAGCAGTCC
TTGGACATTTCATCACT / Celera SNP ID: hCV25619954 / Public SNP ID: rs4911462 /
SNP Chromosome Position: 33642732 / SNP in Genomic Sequence: SEQ ID NO: 373 /
SNP Position Genomic: 62525 / Related Interrogated SNP: hCV1825046 / SNP
Source: Applera / Population(Allele,Count): Caucasian (G,10|T,28) African
American (G,18|T,20) total (G,28|T,48) // / SNP Type: TRANSCRIPTION FACTOR
BINDING SITE;INTRON / SNP Source: dbSNP; HGBASE / Population(Allele,Count):
Caucasian (G,82|T,142) / SNP Type: TRANSCRIPTION FACTOR BINDING SITE;INTRON /
Context (SEQ ID NO: 1494): /
TTCATCCAAGTAAGACGTAAGATGCTCAAGTGCATAGAATGTATTTAAAATAAATTAACTTCAAATGGAAGATAGACTATGTC
AGTGAATAAAAGTTCCT
S
AATGCCAAGCAATGGCTTACACCTGGCCCACAATTACTGAAAAGCTGGAAGATTTTTACCTACAAATCTAGATTTCCCATAAC
CTCTCAGGGAAACACTC / Celera SNP ID: hCV624499 / Public SNP ID: rs717593 / SNP
Chromosome Position: 33652964 / SNP in Genomic Sequence: SEQ ID NO: 373 / SNP
Position Genomic: 72757 / Related Interrogated SNP: hCV25620145 / SNP Source:
dbSNP; HapMap; ABI_Val; HGBASE / Population(Allele,Count): Caucasian

(G,110|C,10) / SNP Type: INTRON /
Context (SEQ ID NO: 1495): /
TGCCTCAGGCAGAGGCAGGGCAGGCACAGCTGCCCCGTGCCCCAAGGGATGGAGGGAAAGGCCCCTCACTTCTGGGAGAACCC
CCTTGGATGAACACAGC
R
GCCAATGAGCAAACAGAACCAGAGGAGCTACAGGAAAATGAGGCAGAGGGGCAGGCTTGGGGGAAGGCAGCATTCTCTGGTAC
CCACCACCCCTGCCCAG / Celera SNP ID: hCV1347919 / Public SNP ID: rs1058003 /
SNP Chromosome Position: 33590417 / SNP in Genomic Sequence: SEQ ID NO: 373 /
SNP Position Genomic: 10210 / Related Interrogated SNP: hCV1825046 / SNP
Source: dbSNP; Celera; HapMap; ABI_Val; HGBASE / Population(Allele,Count):
Caucasian (G,83|A,143) / SNP Type: TRANSCRIPTION FACTOR BINDING
SITE;MICRORNA;UTR3;INTRON;PSEUDOGENE /
Context (SEQ ID NO: 1496): /
GTGGAGGTTGCAGTGAGCCAAGATCATGCCACTGCACTCCAGCCTGGGCAATAGAGCAAGATTCCGTCTTGAAAAAAAGAAAA
AGAAAGAAAACACATTT
W
ATATATCTAACCCACTGAACCTCCTAGCTTAGCCTAGCCTACCTGGAATGTGCTCAGAACACTGACTTCAGCCTACAGTTGGC
CATAATCACTGGCAACA / Celera SNP ID: hCV1347925 / Public SNP ID: rs6120790 /
SNP Chromosome Position: 33599090 / SNP in Genomic Sequence: SEQ ID NO: 373 /
SNP Position Genomic: 18883 / Related Interrogated SNP: hCV1825046 / SNP
Source: dbSNP; Celera; HapMap / Population(Allele,Count): Caucasian
(A,91|T,19) / SNP Type: TRANSCRIPTION FACTOR BINDING SITE;INTRON /
Context (SEQ ID NO: 1497): /
TGTTTGTGATCTCCTGTTTGACCCACTGGTTATTTAGAAGTATACTGCTTAATTTGCATGTATTTATTAATTTCCCAAGTTTG
TTATTTATTTCTAACTC
R
CCCCACTGGGATTTGAGGACATACTTTTGTACAGTTTTAATCCTTTTAAATTTGCTGAACTTTTTTTTTAACAGCCTAGCATAT
GGTCTGTCCTGGAGAAT / Celera SNP ID: hCV1347943 / Public SNP ID: rs6060164 /
SNP Chromosome Position: 33616314 / SNP in Genomic Sequence: SEQ ID NO: 373 /
SNP Position Genomic: 36107 / Related Interrogated SNP: hCV1825046 / SNP
Source: dbSNP; Celera / Population(Allele,Count): Caucasian (A,45|G,75) / SNP
Type: UTR3;INTRON /
Context (SEQ ID NO: 1498): /
TGAGAAGAACGTGCATTCTGTAGTAGTTGGGTGGAGGGTTCTACAGTTCTCTGTTAGTCCTTGTTGGTATACAGTGTTATTTA
AGTCTTCTGTTTCCCTG
Y
TGATCTTCTGCTTAGTTTTATCCATTATTAAAAGTAGTATGTTAAGGTCTCCAGCTGTTGTTTTTGAACTACTGCTCTCTTCC
AATCTGTTAGTTTTTGC / Celera SNP ID: hCV1347944 / Public SNP ID: rs6087660 /
SNP Chromosome Position: 33616528 / SNP in Genomic Sequence: SEQ ID NO: 373 /
SNP Position Genomic: 36321 / Related Interrogated SNP: hCV1825046 / SNP
Source: dbSNP; Celera; HapMap / Population(Allele,Count): Caucasian
(T,83|C,143) / SNP Type: MISSENSE MUTATION;ESE;INTRON /
Context (SEQ ID NO: 1499): /
TGGAGACAGGGTCTCACTATTGTTGCCCAGGCTGGAACTCCTGGACTCAAGCAATCCTCCCACCTTGGCCTCCTAAACTGCTG
GGATTACATGTATGAGC
Y
ACTGTGCCTGACCCTTTCTTCTTTTAATACATATTTAGGTAAACTACAGTTTTTCACTATTAAACAATGTTGCAATGAACATT
CTTACAGGTACTCTCTG / Celera SNP ID: hCV1347962 / Public SNP ID: rs6088691 /
SNP Chromosome Position: 33633758 / SNP in Genomic Sequence: SEQ ID NO: 373 /
SNP Position Genomic: 53551 / SNP Source: dbSNP; Celera; HapMap /
Population(Allele,Count): Caucasian (C,179|T,43) / SNP Type: INTRON /
Context (SEQ ID NO: 1500): /
AGTGAATGTGCCCAAAACGAGTGCATGATTCCTAGAAGCTCTCATGAATCCCATACTCGCCTCTATTTCTAGTAATGGAGGAC
AGGAATTTGCTGTGCAG
R
GGGCATGGGGAACAACTGCTTCCACTTTTCCTTTTCCCTAGTACAAAACAAATGTAAGCTACTACTATTATTGGCAACTGTA
ACTTGTAGCTCACCAGT / Celera SNP ID: hCV1347963 / Public SNP ID: rs13042358 /
SNP Chromosome Position: 33634479 / SNP in Genomic Sequence: SEQ ID NO: 373 /
SNP Position Genomic: 54272 / Related Interrogated SNP: hCV1825046 / SNP
Source: dbSNP; Celera; HapMap / Population(Allele,Count): Caucasian
(G,126|A,100) / SNP Type: INTRON /
Context (SEQ ID NO: 1501): /
AAAACTCTGTCTCAAAAAAAAAGAAAAAAAATTCTGTGTGTGTGTATTTCTCATTCTTTTTGATGCTCAAATTGTCCCAAATT

TGACCATTGGTAGTCCT
Y
TCAAATTAATTCCTGTGTCATCTTTGGGACAATCTTAATTTTAAATGGGCCCTGTGGGACTTAAACTTGTCCAACACTATGAT
CGGTTCTGAGTTCAGAA / Celera SNP ID: hCV1348012 / Public SNP ID: rs6060230 /
SNP Chromosome Position: 33689308 / SNP in Genomic Sequence: SEQ ID NO: 373 /
SNP Position Genomic: 109101 / Related Interrogated SNP: hCV25620145 / SNP
Source: dbSNP; Celera / Population(Allele,Count): Caucasian (C,202|T,22) /
SNP Type: INTERGENIC;UNKNOWN /
Context (SEQ ID NO: 1502): /
ACTTAATACCTGTCCTCTCTTCCTGTCCTGAACCCCAGCACCCTAGCCTTAACCCCTGACCTTTCTACCACATAATCCTGACA
GCTGACCTCTGACCATT
R
TCCCCAGGTGACTCCGCAGCTACCATCCCAACTCTCACCCTGACCCACTGCCCTGGGACTGGCCCAGCCCTGTCAGTTCTGAC
ACCTGTTAAGCCCAGCA / Celera SNP ID: hCV3249293 / Public SNP ID: rs6058154 /
SNP Chromosome Position: 33585834 / SNP in Genomic Sequence: SEQ ID NO: 373 /
SNP Position Genomic: 5627 / Related Interrogated SNP: hCV1825046 / SNP
Source: dbSNP; Celera; HapMap / Population(Allele,Count): Caucasian
(G,126|A,100) / SNP Type: INTRON /
Context (SEQ ID NO: 1503): /
TGCCCTCATGGGGCTTATACTTCTGGGTAAGATCTACCCTTTATTGAGCACTTACCATTGGCCAAGCACTTTCTTTGCACTAT
CTTATTATTCTCGCAGC
W
TTTTTGGGGAAGTTTTTCAAATCTACAGAAAGTTAAAAGATACACCATTAACACTCATATACCCTTTGCTTAGACTTGTCAGC
TACTAAAAACTATATTTT / Celera SNP ID: hCV11189205 / Public SNP ID: rs7261312 /
SNP Chromosome Position: 33684909 / SNP in Genomic Sequence: SEQ ID NO: 373 /
SNP Position Genomic: 104702 / Related Interrogated SNP: hCV25620145 / SNP
Source: dbSNP; Celera / Population(Allele,Count): Caucasian (A,110|T,10) /
SNP Type: INTERGENIC;UNKNOWN /
Context (SEQ ID NO: 1504): /
ACACATACAAAAAGGATTATACTCCACGCATGATGGCTTATGCCTGTAATCCCAGCACTTTGAGACGTCAAGGCAGGAGGACT
ACTTAAGCCCAAGAGTA
Y
GCAACCAGCCTGGGCAACATAGTGAGACCCTGTCTCTACAAAAAATTAAAACCAAAAATTAGCCAAGCATGGTAGCATGCTCCT
GTAGTCCCAGCTACTCA / Celera SNP ID: hCV11189240 / Public SNP ID: rs2038504 /
SNP Chromosome Position: 33648187 / SNP in Genomic Sequence: SEQ ID NO: 373 /
SNP Position Genomic: 67980 / Related Interrogated SNP: hCV1825046 / SNP
Source: dbSNP; Celera; HapMap; HGBASE / Population(Allele,Count): Caucasian
(T,69|C,49) / SNP Type: INTRON /
Context (SEQ ID NO: 1505): /
CGGCCTGGGATCCTTTATCACGAAATGCAGGTTACAGGGGAAGACAGCAGATCCATACACAAAGAAACGCAGTCTCAAACTGG
ATAAGTTGTGTAACTGG
R
TAGGTTGTTGTGAAATTAAATAATGGAAGGGAAAGGGATTAGTAAATTGTGACATCATATTCTCTGTCAGATGAAAACCCTGT
CCTAATTTCTATCCACC / Celera SNP ID: hCV11656982 / Public SNP ID: rs1885115 /
SNP Chromosome Position: 33681256 / SNP in Genomic Sequence: SEQ ID NO: 373 /
SNP Position Genomic: 101049 / Related Interrogated SNP: hCV1825046 / SNP
Source: dbSNP; Celera; HapMap; HGBASE / Population(Allele,Count): Caucasian
(G,36|A,80) / SNP Type: MISSENSE MUTATION /
Context (SEQ ID NO: 1506): /
GGTTCTAGTCCCAGCTTGGCCACCTACTTGCCCTTCTCTGGCCTCAGCTTTCTCATTTTTGAAAAGAAGTATTTTAGATTAGG
TAATCTCTGGGATCTAA
Y
GCGGCTTTAATTTCTGAAAAGCTGCAGAACATTATTACTATGATTCTTCTGTTTAAAGAACATTTGTAAGTTTATTTTAAAAG
GACTCAAAAGATACACC / Celera SNP ID: hCV11656986 / Public SNP ID: rs1885119 /
SNP Chromosome Position: 33645649 / SNP in Genomic Sequence: SEQ ID NO: 373 /
SNP Position Genomic: 65442 / Related Interrogated SNP: hCV1825046 / SNP
Source: dbSNP; Celera; HapMap; HGBASE / Population(Allele,Count): Caucasian
(T,83|C,143) / SNP Type: TRANSCRIPTION FACTOR BINDING SITE;INTRON /
Context (SEQ ID NO: 1507): /
GAATTGAAAGTGTGTTTTCTATAGGCTGCATATAACTGGATCTTGTTTTCTGATCCAATATAACAATCTGTCTTTTGAATGAA
TTGTTAAATCTATTCAC
R
TTTAATGTTACTATTGATAAGGCTGGACTGACTTCTGCCATTTAACTTTTTTGTTTTCTGTATGTCTTGTGTGTTTTTTTGTTC

CTCTATATCTCCTTTAG / Celera SNP ID:    hCV16003843 / Public SNP ID:    rs2378332 /
SNP Chromosome Position:    33617250 /   SNP in Genomic Sequence:    SEQ ID NO: 373 /
SNP Position Genomic:    37043 /   Related Interrogated SNP:    hCV1825046 /   SNP
Source:    dbSNP; Celera; HapMap; HGBASE /   Population(Allele,Count):    Caucasian
(A,45|G,75) /   SNP Type:    INTRON /
Context                (SEQ ID NO: 1508): /
AAACACAAATTACTAATATGTGGTACATAAGAGGAGTTATTATTACTGACTCCATGGACTTCGAAGGATGATAAAGGAGTATT
ATGAACCGTTTGCCCCA
R

TATTTGCCTAGCACTTTGGGAGGCTAAGGCGGGAGGATTGCTTCAGCTCAGGAGTTCAAGACCAGCCTGAGCAACATAGCGAG
AGCTCGCCTCTACTAAA /  Celera SNP ID:    hCV16076405 /  Public SNP ID:    rs2145557 /
SNP Chromosome Position:    33667560 /   SNP in Genomic Sequence:    SEQ ID NO: 373 /
SNP Position Genomic:    87353 /   Related Interrogated SNP:    hCV1825046 /   SNP
Source:    dbSNP; HapMap; HGBASE /   Population(Allele,Count):    Caucasian
(A,84|G,142) /   SNP Type:    INTRON /
Context                (SEQ ID NO: 1509): /
ACTTCAAGACTCATCTTAGATGTATCTAAGGACCTCAGAGAAGGTCTCCCTGTATTCACCCAGCCCCCAGCCCTAGCAGCGGA
AGAGGCTTTCCTTTGCT
S

CTATAGCTCCTGGGCCAACCCTGGCTCCATTCCTTCCCACACTGCATTGAAACAGCACAGCTCTAGGGAATTCATGGCAGGGC
CCGTATCCTAGTTCTCT / Celera SNP ID:    hCV16190708 /  Public SNP ID:    rs2295701 /
SNP Chromosome Position:    33596117 /   SNP in Genomic Sequence:    SEQ ID NO: 373 /
SNP Position Genomic:    15910 /   Related Interrogated SNP:    hCV1825046 /   SNP
Source:    dbSNP; HapMap; ABI_Val; HGBASE /   Population(Allele,Count):    Caucasian
(G,61|C,165) /   SNP Type:    UTR5;INTRON /
Context                (SEQ ID NO: 1510): /
CAAAAACATTATGCCAAGTGAAAGAAACCAGATTCAGAAGGCCACATGCTATATGATTCCATTTACAGTTATGCACTGCATTA
ACGCTGTTGCGGTCAAC
R

ACGACCACACAGAAGACGGTGGTGCCATCAGATTATAATACTGTATTTTTACTATACCTTTTTTTCTATGTTTAGATACACAAA
TACTTACCATTATGGTA /  Celera SNP ID:    hCV27166951 /  Public SNP ID:    rs6087663 /
SNP Chromosome Position:    33625526 /   SNP in Genomic Sequence:    SEQ ID NO: 373 /
SNP Position Genomic:    45319 /   Related Interrogated SNP:    hCV1825046 /   SNP
Source:    dbSNP; Celera; HapMap /   Population(Allele,Count):    Caucasian
(A,35|G,85) /   SNP Type:    INTRON /
Context                (SEQ ID NO: 1511): /
CTTGACCTCCCAAAGTGCTGGGATTACAGGCATGAGCCACCGCACCCGGCCAAAGAAAATTGTTTCTAATCGATAACCTAATC
TTCTACCTTAAGACACT
R

GAAAAAGAGCAGACTAAACCCAAAGCAAGCAAAAGGGAGAATAAGAGTTGAAACAAATGAAACAGAGAATAGAAAATAACAGA
AAATGAACAAAACCAAA /  Celera SNP ID:    hCV27167730 /  Public SNP ID:    rs2889873 /
SNP Chromosome Position:    33646841 /   SNP in Genomic Sequence:    SEQ ID NO: 373 /
SNP Position Genomic:    66634 /   Related Interrogated SNP:    hCV25620145 /   SNP
Source:    dbSNP; Celera; HapMap; HGBASE /   Population(Allele,Count):    Caucasian
(G,103|A,9) /   SNP Type:    INTRON /
Context                (SEQ ID NO: 1512): /
GGAGCTGTTGATCTGCTTCAGGAATACCTGGAACTATACAGAAACCAAGCTCACAGCAGGCAGTGGGCTGCTGGCCAGGTATG
GCCATGGCCCGGGGGGA
Y

AGTCACTACAAGGGGCATCAGCGACCTCTACCAGCCCCACTGCTTCAGATAGGAAGACAGAGGCTCAGATAAGCTGAGGGACC
TCCCCTCACCACCCAGG / Celera SNP ID:    hCV27472681 /  Public SNP ID:    rs3746430 /
SNP Chromosome Position:    33593674 /   SNP in Genomic Sequence:    SEQ ID NO: 373 /
SNP Position Genomic:    13467 /   Related Interrogated SNP:    hCV1825046 /   SNP
Source:    dbSNP; HapMap /   Population(Allele,Count):    Caucasian (T,35|C,85) /   SNP
Type:    UTR5;INTRON /
Context                (SEQ ID NO: 1513): /
ATCCCCTTACATACGGGGCTTAGTGGCTAGATCACTTAGGTTCCTATCCCAGCTCTGTAACTTGCAGCTCTGAAACTTTGGTT
AAGTTACAAAAACCTTT
M

GAGTCTTTGCTTGTTTATCTGTAAAACGGAACTAATCTACCTCACAGGATTAGTCTGAGGACTAAATTTACCTTTTTGAGCACC
ACTGTTTTCAATTGTTC / Celera SNP ID:    hCV27486123 /  Public SNP ID:    rs3803937 /
SNP Chromosome Position:    33632687 /   SNP in Genomic Sequence:    SEQ ID NO: 373 /
SNP Position Genomic:    52480 /   Related Interrogated SNP:    hCV1825046 /   SNP

Source: dbSNP; HapMap / Population(Allele,Count): Caucasian (C,42|A,66) / SNP Type: INTRON /
Context (SEQ ID NO: 1514): /
CTAGTCGGATCATTTCCTACAGAAGACAAAAATGAAACAATCCCCATGCTCAATTCACTCAAGAGATTGGATGTGCCTCTCTT
TCGCATCCCCTTACATA
Y
GGGGCTTAGTGGCTAGATCACTTAGGTTCCTATCCCAGCTCTGTAACTTGCAGCTCTGAAACTTTGGTTAAGTTACAAAAACC
TTTCGAGTCTTTGCTTG / Celera SNP ID: hCV27503616 / Public SNP ID: rs3803938 /
SNP Chromosome Position: 33632600 / SNP in Genomic Sequence: SEQ ID NO: 373 /
SNP Position Genomic: 52393 / Related Interrogated SNP: hCV1825046 / SNP
Source: dbSNP; HapMap; HGBASE / Population(Allele,Count): Caucasian
(C,84|T,142) / SNP Type: INTRON /
Context (SEQ ID NO: 1515): /
CTGACCCCCTGGGCTCTAAAGAGGAATGTCTGCTGTTGCACATCTGGCTGAGGCCACCTGCCCCGATCCTGCCATCTCTGCAT
CGCCCCCTGCTGCCTTC
R
GCCTTCCCTGGGCCCTGAATAAACACCACAGCCAGTTTCCTTCTCATTCTTTTCTTTGGGGTTCAGGAGGAAAAAACACAGTCC
TAGGGACAAAAGCCAGG / Celera SNP ID: hCV27893015 / Public SNP ID: rs4911167 /
SNP Chromosome Position: 33590096 / SNP in Genomic Sequence: SEQ ID NO: 373 /
SNP Position Genomic: 9889 / Related Interrogated SNP: hCV1825046 / SNP
Source: dbSNP; HGBASE / Population(Allele,Count): Caucasian (A,83|G,143) /
SNP Type: UTR3 /
Context (SEQ ID NO: 1516): /
GGAACCATGTGAATGTTACACCTAATTAAAAATTAATAAATAACTAGATATTTCAGGATATCAAGGGATTACTATTAATTTTT
GATACAGTGGCAGTTAC
R
TTTCTTAAAAGTCGTTATCATGTAGAAACACTGAAATATATGCAGGTGAAATTATACGATGTCTGGGGTTTACTTCAAAATAA
CCCAAGGTGAAGTAGGG / Celera SNP ID: hCV27982387 / Public SNP ID: rs4911460 /
SNP Chromosome Position: 33624330 / SNP in Genomic Sequence: SEQ ID NO: 373 /
SNP Position Genomic: 44123 / Related Interrogated SNP: hCV1825046 / SNP
Source: dbSNP; HapMap; HGBASE / Population(Allele,Count): Caucasian
(G,45|A,75) / SNP Type: TRANSCRIPTION FACTOR BINDING SITE;INTRON /
Context (SEQ ID NO: 1517): /
AATGAACGTCTGCGAAGAAAGAGACAGGGCAGCCTGACCCCTCTCCAAGTCACCACCAACTTTTGGTGCCTGGCTTGCGCAGG
CCTGACAAGACTTTCTG
Y
GATAAATATTTTCAAGAAACAGTCTCCAAAGCAATGGCCAGAGCTTAGCAACAAGGTCAGTCCCAAGGATGAGGATGGGAAAA
CCAGCTCACGAGGCCTG / Celera SNP ID: hCV29372788 / Public SNP ID: rs6060163 /
SNP Chromosome Position: 33611738 / SNP in Genomic Sequence: SEQ ID NO: 373 /
SNP Position Genomic: 31531 / Related Interrogated SNP: hCV1825046 / SNP
Source: dbSNP; HapMap / Population(Allele,Count): Caucasian (T,60|C,166) /
SNP Type: INTRON /
Context (SEQ ID NO: 1518): /
TGTAGGTTATGGGCCCCTCCTCCTCCGTCTTGGAACTACTTCAAGGCTCAGGGAACCTTGCACATCTGGAGCTCCCTGTACTT
GCCCAGTTTTCCTAAGC
R
GACACAGCTCTGGGCTCTGTCCCACTGCCCTGAGCCCACATCAGGTTGGCAACTCCTCTAGCATGTCCACAGGGCCAGGCTTG
CCAGCTTTCAACTCTTT / Celera SNP ID: hCV29372789 / Public SNP ID: rs7274866 /
SNP Chromosome Position: 33608616 / SNP in Genomic Sequence: SEQ ID NO: 373 /
SNP Position Genomic: 28409 / Related Interrogated SNP: hCV25620145 / SNP
Source: dbSNP; HapMap / Population(Allele,Count): Caucasian (A,110|G,10) /
SNP Type: INTRON /
Context (SEQ ID NO: 1519): /
CAGCCTGGATCTACCTTGCCCTGCTTAACTGGGGGTGGGGAGGTACGGCTGACATCTATGGTGTTCACCAAGCCATGTGCCTT
CCTGTCTCACTCCAAAG
R
GCCCTAAGCCAGGCCCAGGTCACACAGCTATAAAATGTTCCAACAATGGCCCAAAAAAATTCATCCAGTGCAATTGCCTCACT
GCACAGACTAGAAACCC / Celera SNP ID: hCV29372790 / Public SNP ID: rs6579211 /
SNP Chromosome Position: 33605857 / SNP in Genomic Sequence: SEQ ID NO: 373 /
SNP Position Genomic: 25650 / Related Interrogated SNP: hCV25620145 / SNP
Source: dbSNP; HapMap / Population(Allele,Count): Caucasian (G,204|A,22) /
SNP Type: TRANSCRIPTION FACTOR BINDING SITE;INTRON /
Context (SEQ ID NO: 1520): /

AAACCCCCACAAAATCAAGAACAGTTGATCCAAAGATGTCCGAAGATGGGATAGGCTCCTACTAGAGAGCCACATTTCCACTG
CAAATAGACACAGGCTA
S
AGACCTACATTATTATTTTTCCTCCCATAAATACATCAGTTCTTACTAAGTAGATTTCTTGATGAAACAATTCAAAAGTAAAG
GGTCTCTTGAGCTAGCC / Celera SNP ID: hCV29373055 / Public SNP ID: rs6060196 /
SNP Chromosome Position: 33653617 / SNP in Genomic Sequence: SEQ ID NO: 373 /
SNP Position Genomic: 73410 / Related Interrogated SNP: hCV1825046 / SNP
Source: dbSNP; HapMap / Population(Allele,Count): Caucasian (C,36|G,84) / SNP
Type: INTRON /
Context (SEQ ID NO: 1521): /
CTGCTCAATATATGGGCGGGGCTTCTGGTTCTCATTTTGTTTTCTAACAAGGAAAATGAAGAAATAGGCATAATGGAGTTAAA
ATGAGTCTTAAAGGTCA
Y
AGTCTGCCAACACTTGTCACCTTTTGACAAGTGTCCAGTTTCTACTTGGACAGCTCTAAGGCCAAAGGAGTTTACTACCTGCT
GCAGCAGTCCTGGCTCT / Celera SNP ID: hCV29373059 / Public SNP ID: rs8117847 /
SNP Chromosome Position: 33642480 / SNP in Genomic Sequence: SEQ ID NO: 373 /
SNP Position Genomic: 62273 / Related Interrogated SNP: hCV25620145 / SNP
Source: dbSNP; HapMap / Population(Allele,Count): Caucasian (C,204|T,22) /
SNP Type: INTRON /
Context (SEQ ID NO: 1522): /
TTTATATGTCGTGAACTGAACAATTGCCGAGAGATAATACTGGGAAAGCAAGTGTGCTCATGCTGTATTACTGATTGTGTAAA
AAAAGAGGAATACAATT
Y
GCCTTTGTAAGCATAAAAATGTCTCTAGAAGGATACACTAATAACTGGTAAAATTAGCTGCTTCATGGAACCATTAGAATAGA
ATGTCAAGGATCAGAGG / Celera SNP ID: hCV30342001 / Public SNP ID: rs10485508 /
SNP Chromosome Position: 33605098 / SNP in Genomic Sequence: SEQ ID NO: 373 /
SNP Position Genomic: 24891 / Related Interrogated SNP: hCV25620145 / SNP
Source: dbSNP; HapMap / Population(Allele,Count): Caucasian (C,110|T,10) /
SNP Type: INTRON /
Context (SEQ ID NO: 1523): /
CCCAAAGTGCTGGGATTACAGGCGTTAAGCCACCGTGCCTGGCCTCAGTGAATAATCTTGTACAGTAGTCCTCCCTTAGTCAA
GACCCTCACAGTGGATA
Y
CTGAAATGGCAGATAGCACCAAACCCTATATATACTATGTTTTTTCTTATATACACACATAACTATGATAAAGCTTAATTTAT
AAATTAGGTACACTAAG / Celera SNP ID: hCV32066111 / Public SNP ID: rs10875492 /
SNP Chromosome Position: 33631407 / SNP in Genomic Sequence: SEQ ID NO: 373 /
SNP Position Genomic: 51200 / Related Interrogated SNP: hCV1825046 / SNP
Source: dbSNP; HapMap / Population(Allele,Count): Caucasian (C,45|T,75) / SNP
Type: UTR3;INTRON /
Context (SEQ ID NO: 1524): /
TACAAATATTAATTTCCCTCCCCTTTAACTTCACCTCAATCTCCTGAGATTTTGCAAGAAGCAAATAATGTGCAACTCTTTGA
AAAGCATTTTTAAACAA
R
TTTAAGAAGCTATAATTATTTCTATCAGACTGTGAGATTCAAAATATGAAAATGTCTCTTCTTAAACGTAAAGACTAAGTTTA
ATTAGTTTCTATTACAT / Celera SNP ID: hCV29909901 / Public SNP ID: rs6060205 /
SNP Chromosome Position: 33658869 / SNP in Genomic Sequence: SEQ ID NO: 373 /
SNP Position Genomic: 78662 / Related Interrogated SNP: hCV1825046 / SNP
Source: dbSNP / Population(Allele,Count): Caucasian (G,84|A,142) / SNP Type:
INTRON /
Context (SEQ ID NO: 1525): /
GATTTGTTTTTTTTGTTTTTTTGTGTTTTTTTGGCTATTTAAGTAGACCACAAATGAACAACTTAATATATATGTGTATCCTTTT
CATGTTTGTATTAAACT
R
GTTCCTCAAGATACATTTTCAAAAGTAGAACTACTAGAATCTAAAGTATAGGCATCTTTTTTTTTGTTCATTTGTTTTTGGTTTT
TTAAATGAGGTCTTACT / Celera SNP ID: hCV29819450 / Public SNP ID: rs4142034 /
SNP Chromosome Position: 33643256 / SNP in Genomic Sequence: SEQ ID NO: 373 /
SNP Position Genomic: 63049 / Related Interrogated SNP: hCV1825046 / SNP
Source: dbSNP; HapMap; ABI_Val; HGBASE / Population(Allele,Count): Caucasian
(A,62|G,164) / SNP Type: INTRON;PSEUDOGENE /
Context (SEQ ID NO: 1526): /
TTCCCTCTTAGATCAGGAACATGCTACTTCTATTCATCATACTGGAGGTCTCAGGCAGCAGAGTAAGGCAAGATATATAGAAG
ACATAAGATTGGTAAGG
R

TAAGATAAAAGATTCATTTGCAGATGATATAATATTCACAGATGACTGTACAGTATACAATCCAAAAGAACCTAAATGTAAAT
TATCAACTATTACAGCA / Celera SNP ID: hCV30000150 / Public SNP ID: rs4911465 /
SNP Chromosome Position: 33670856 / SNP in Genomic Sequence: SEQ ID NO: 373 /
SNP Position Genomic: 90649 / Related Interrogated SNP: hCV1825046 / SNP
Source: dbSNP; HapMap; HGBASE / Population(Allele,Count): Caucasian
(G,36|A,84) / SNP Type: UTR3;INTRON /
Context (SEQ ID NO: 1527): /
ACTGCGCCCGGCCTAAAATTGTTTTAATTAAAAAAAAAAAAAAGAAGAAGAAAGAAGGTACAGAATAGTGTAGCCGTGTATATG
GTATATCACTCTATGTG
S

GGAGAAAATAAACTAAGGTGGAGGTGGTGGGGAATATTTACACACACACACACACACACACACACACACAAAAAAAAACAAA
ACATCTTAAGCAGGACA / Celera SNP ID: hCV30450323 / Public SNP ID: rs6058166 /
SNP Chromosome Position: 33656710 / SNP in Genomic Sequence: SEQ ID NO: 373 /
SNP Position Genomic: 76503 / Related Interrogated SNP: hCV1825046 / SNP
Source: dbSNP / Population(Allele,Count): Caucasian (C,44|G,72) / SNP Type:
INTRON /
Context (SEQ ID NO: 1528): /
AAGAAAAAAAAATTCTGTGTGTGTGTATTTCTCATTCTTTTTGATGCTCAAATTGTCCCAAATTTGACCATTGGTAGTCCTCTC
AAATTAATTCCTGTGTC
R

TCTTTGGGACAATCTTAATTTTAAATGGGCCCTGTGGGACTTAAACTTGTCCAACACTATGATCGGTTCTGAGTTCAGAAAAT
AGTCTCTCTAAATTTTT / Celera SNP ID: hCV29530378 / Public SNP ID: rs6058179 /
SNP Chromosome Position: 33689328 / SNP in Genomic Sequence: SEQ ID NO: 373 /
SNP Position Genomic: 109121 / Related Interrogated SNP: hCV1825046 / SNP
Source: dbSNP / Population(Allele,Count): Caucasian (A,64|G,162) / SNP Type:
INTERGENIC;UNKNOWN /
Context (SEQ ID NO: 1529): /
TCCCATCAGCTGGCCACAATGGTCTTCCCTCGTTCAGGGGCTTCTGGCTAGAACACACACCCTCTCACATGCCTGTTCACTCA
ATTCACTACAAGCTGAC
M

CTTCAGGTCTTGGCTGGCAAGCCACTTCTGCAGGGGAGAGTTCCTGAGTGTCCTCTGGCCAGTCCTGGACCCTGTCAAATGTC
TCTCACAGACCCCGTTC / Celera SNP ID: hCV29638959 / Public SNP ID: rs6060154 /
SNP Chromosome Position: 33599601 / SNP in Genomic Sequence: SEQ ID NO: 373 /
SNP Position Genomic: 19394 / Related Interrogated SNP: hCV1825046 / SNP
Source: dbSNP; HapMap / Population(Allele,Count): Caucasian (A,61|C,165) /
SNP Type: INTRON /
Context (SEQ ID NO: 1530): /
ATATTTATTCTCCTGTACCTTGGGCAAAAAACTATAGTTACTCAATTTCCATGTTAGCACTCCTTGGCCCAGCTTCCAGAACA
GTTAAGTGTCAGCTAAT
S

TGTGAAGTGAACCTGAAGAACTATCACGCAGAATCCTGCCTCTGTCGTGCACTGCTGTGAGACCTTGCGTGCTTGGCATCCTC
GGCACCCAGCCCATGGT / Celera SNP ID: hCV29584663 / Public SNP ID: rs6060162 /
SNP Chromosome Position: 33611461 / SNP in Genomic Sequence: SEQ ID NO: 373 /
SNP Position Genomic: 31254 / Related Interrogated SNP: hCV1825046 / SNP
Source: dbSNP; HapMap; ABI_Val / Population(Allele,Count): Caucasian
(C,45|G,75) / SNP Type: INTRON /
Context (SEQ ID NO: 1531): /
ATATATGCAGGTGAAATTATACGATGTCTGGGGTTTACTTCAAAATAACCCAAGGTGAAGTAGGGTATAGTGAATGGGAGTAG
AGATAAAGTAAGACAAG
S

TGGCCAGGCACGGTGGCTCATGCCTGTAATCCTAGCACTTTGGGAGGCCAAGGTGGGTGGATCACTTGAAGCTAGGAGTTTAA
GACCAGCCTGGTCAACA / Celera SNP ID: hCV29982069 / Public SNP ID: rs6060170 /
SNP Chromosome Position: 33624466 / SNP in Genomic Sequence: SEQ ID NO: 373 /
SNP Position Genomic: 44259 / Related Interrogated SNP: hCV1825046 / SNP
Source: dbSNP; HapMap / Population(Allele,Count): Caucasian (C,43|G,75) / SNP
Type: INTRONIC INDEL;INTRON /
Context (SEQ ID NO: 1532): /
TTAAGGTTAAATGAGGTTACAAAGTGTGGGGCCCTAAACCGAGAGGACTGGTGTCCTTCTAAGAAGAGGAAGAGACACCAGAG
ATCTCCGTCTCTCCTCA
R

TGACAGAGAGGAAAATTCATATGTGGATACAATAAAAAGGTGGCCTTCTACAAGCCAGGAAGAAAGGCCTCAACCAGGAACCA
ATCCTGACAGCACCTTG / Celera SNP ID: hCV29566578 / Public SNP ID: rs6060172 /
SNP Chromosome Position: 33628805 / SNP in Genomic Sequence: SEQ ID NO: 373 /

SNP Position Genomic: 48598 / Related Interrogated SNP: hCV1825046 / SNP Source: dbSNP; HapMap / Population(Allele,Count): Caucasian (A,45|G,75) / SNP Type: INTRON /
Context (SEQ ID NO: 1533): /
GGACAAATAATCAAATCGAAAAAGGTGCAAAACAGAAAACTGGCAGAAAAGGAACTACTTATAAACACAAGAAAAAATATCCA ATCTCAGTCATAGAAGA
R
TGAATTTTAAAAACTATGCAGAGAATTTATAAAGAACTCTTACAACACAATATTCAAAAGAGATATATCTCAACTAAGAAATT GAAAAAGGAGAGACAGA / Celera SNP ID: hCV29747445 / Public SNP ID: rs6060199 / SNP Chromosome Position: 33654773 / SNP in Genomic Sequence: SEQ ID NO: 373 / SNP Position Genomic: 74566 / Related Interrogated SNP: hCV1825046 / SNP Source: dbSNP; HapMap / Population(Allele,Count): Caucasian (A,70|G,46) / SNP Type: INTRON /
Context (SEQ ID NO: 1534): /
TTACTAAATACCTGAAAATTCAAACAACTGTGAAAGTGCCTCGAAAAAGTAATAGCACTCAATGCACAGAAGCTGTCCCTACA ATTATTTCACATATAAG
Y
AACTGCGAAAGTGCCTCAAAAAAAGTAATAGCACTCAATGCATAAAAGCTGTCCCTAAAACTATTATTTTTTATTAAAAAACATG TTCCGTATTGAAGGAAG / Celera SNP ID: hCV30053849 / Public SNP ID: rs6088677 / SNP Chromosome Position: 33607464 / SNP in Genomic Sequence: SEQ ID NO: 373 / SNP Position Genomic: 27257 / Related Interrogated SNP: hCV1825046 / SNP Source: dbSNP / Population(Allele,Count): Caucasian (C,61|T,165) / SNP Type: INTRON /
Context (SEQ ID NO: 1535): /
GAGGCATGAGAATTGCTTGAACCTGGGATGCAGAGGTTGCAGTGAGCTGAGACTGCACCACTGTACTCCAGCCTGGGCAAGAG TGATACTCTGTCTCAAC
R
CGTGCGCACACAAACACACACACACACACACCCCTCCCATAATTAGTGAGTGGCTATAAGATGATGTTTGACCTTAGTTAT AAAATAAAACTACATTG / Celera SNP ID: hCV30053848 / Public SNP ID: rs6088687 / SNP Chromosome Position: 33624750 / SNP in Genomic Sequence: SEQ ID NO: 373 / SNP Position Genomic: 44543 / Related Interrogated SNP: hCV1825046 / SNP Source: dbSNP; HapMap / Population(Allele,Count): Caucasian (G,45|A,75) / SNP Type: TRANSCRIPTION FACTOR BINDING SITE;INTRON /
Context (SEQ ID NO: 1536): /
CCTTGGCAACAGAGGGAGACCCTAACTCAAAAAAAAAAACAAAACAAAAAAACCAACAACAACAAAAAAACAAAACCCCCAAG GGGTATGAAGGAAGTTA
R
GGAAACTAGCTGGTAAAAGACCTTAAAACTTAGGTTCAAGAGTTTGGATTTAAAACTGCAGTATTGTCAAGTGCAGTGGCTCA CACCTGTAATCCCAGCT / Celera SNP ID: hCV30035910 / Public SNP ID: rs6088692 / SNP Chromosome Position: 33638588 / SNP in Genomic Sequence: SEQ ID NO: 373 / SNP Position Genomic: 58381 / Related Interrogated SNP: hCV1825046 / SNP Source: dbSNP / Population(Allele,Count): Caucasian (A,83|G,143) / SNP Type: INTRON /
Context (SEQ ID NO: 1537): /
TCAGCAGCTGTGGGCAGTGTGGAGAGGTGGAGACAAGACCTAGTCACACTAAGGCTTGGGTTTTAATGCCCTGCAGGGACTAG ACAGGGTGTCAGGTCCC
R
GTAAAGCCTGGGGAGCATGTACACTTGGTGAAAGAAGGGTTAGAAAGGCCTGACCCACCAGCCCAGGGAGCCTGGCTCTGCCA TGGTGCTGCTGGTGGGT / Celera SNP ID: hCV30144027 / Public SNP ID: rs6119559 / SNP Chromosome Position: 33601601 / SNP in Genomic Sequence: SEQ ID NO: 373 / SNP Position Genomic: 21394 / Related Interrogated SNP: hCV1825046 / SNP Source: dbSNP; HapMap / Population(Allele,Count): Caucasian (A,35|G,85) / SNP Type: INTRON /
Context (SEQ ID NO: 1538): /
TTCTCCAAAAGTCCAATAATAAACAGATGATCAACATTAGTCATCAGGGAAATGCAAATCAAAACCACAATGAGATACCTCTT CATACCTACTACGATGG
Y
TATAATGAAAAAGACAAATAGTAACTATTGGCAAAGATGTGGAGAAATTAGAACCTTCATACACTGCTGGTAGATATGTAAAA TGGTGCAGCTGCTTCAG / Celera SNP ID: hCV30503955 / Public SNP ID: rs6060194 / SNP Chromosome Position: 33649930 / SNP in Genomic Sequence: SEQ ID NO: 373 / SNP Position Genomic: 69723 / Related Interrogated SNP: hCV1825046 / SNP Source: dbSNP; HapMap / Population(Allele,Count): Caucasian (T,61|C,165) / SNP Type: INTRON /

Context                    (SEQ ID NO: 1539): /
GAAAAGAAGTATTTTAGATTAGGTAATCTCTGGGATCTAATGCGGCTTTAATTTCTGAAAAGCTGCAGAACATTATTACTATG
ATTCTTCTGTTTAAAGA
R
CATTTGTAAGTTTATTTTAAAAGGACTCAAAAGATACACCATAAATTGTTAACACTGATTTCTACAGGTTTGGGGGTTATATG
CATGTGTTTTTTATTTT / Celera SNP ID:    hCV30180149 / Public SNP ID:    rs9941751 /
SNP Chromosome Position:   33645709 /  SNP in Genomic Sequence:   SEQ ID NO: 373 /
SNP Position Genomic:   65502 / Related Interrogated SNP:   hCV25620145 / SNP
Source:   dbSNP; HapMap / Population(Allele,Count):   Caucasian (A,110|G,10) /
SNP Type:    INTRON /
Context                    (SEQ ID NO: 1540): /
TCCCTCAGCCCTCCCCAGACTGGCTCCAGACCCGTGCCCTCCATATCACACACAGAAGAGTTCCCTCCACAGGAAGATGGATC
CATGACGCTGCATTAAG
R
AAAACAACGCAAAAGTTCCACTCCTTCTGCAGAGGTCCTAGCGCAACTGGTTTTCCACACCAGCTCTCAGGTGAGTCCGGCCC
AGTGCTGAGACCACAAG / Celera SNP ID:    hDV70862585 / Public SNP ID:    rs17092209 /
SNP Chromosome Position:   33592721 /  SNP in Genomic Sequence:   SEQ ID NO: 373 /
SNP Position Genomic:   12514 / Related Interrogated SNP:   hCV1825046 / SNP
Source:   dbSNP; HapMap / Population(Allele,Count):   Caucasian (A,193|G,33) /
SNP Type:    UTR5;INTRON /
Context                    (SEQ ID NO: 1541): /
ACACATGCAGACAGGCCACTGGGCTCACAGACGATGGGCTGGCCTAGGGAAGTAGTGGTTGATGCTGACCGAGGGACCCAGAG
GGCACAGCGGGTATCCA
Y
GACAGGGAGTTTGGACAAGGCACTGAAGTGACAAAGGCCCATGTGGGCATGAAGCCTCCTGACAGCTCACGCCCGGCCCTGAT
GGAACTCTACCTGTCCA / Celera SNP ID:    hDV70862590 / Public SNP ID:    rs17092215 /
SNP Chromosome Position:   33595913 /  SNP in Genomic Sequence:   SEQ ID NO: 373 /
SNP Position Genomic:   15706 / Related Interrogated SNP:   hCV25620145 / SNP
Source:   dbSNP / Population(Allele,Count):   Caucasian (C,204|T,22) / SNP Type:
  MISSENSE MUTATION;INTRON /
Context                    (SEQ ID NO: 1542): /
AGCGAGACTCCATCTCAAAAAAAAAAAAAAAATTACAGGAAAATAACTTCCAATTTATACTTCTATACCCAGTCAAACTAAATAT
ATGTGTCAGAATAAGAC
R
TTTTCTGATCTGCAAGCTTTAAAAAAAAAAAAAAAAGTTACCTCCCATGTACTCTTTCTTGGGAGGCTACCAGAGAGTGTGCCC
CAGGAAAAAGGAAGACA / Celera SNP ID:    hCV29729418 / Public SNP ID:    rs6142294 /
SNP Chromosome Position:   33671497 /  SNP in Genomic Sequence:   SEQ ID NO: 373 /
SNP Position Genomic:   91290 / Related Interrogated SNP:   hCV1825046 / SNP
Source:   dbSNP; HapMap / Population(Allele,Count):   Caucasian (A,68|G,46) / SNP
Type:    INTRON /
Context                    (SEQ ID NO: 1543): /
AAAGACCAAGTTAGAATACACAGCTGAGACATTAAAAATGAATATAAATCCTCTTAATAACCACTACTGCATGCACAAATGAG
GCCTTATACAGTATGTC
S
TATTGGGGTAAAAAAAAAAATCTTTTAAATTAAAAAAACGCAAAGTTGGTAGGAGGGCATTCACCAAACATTAACTGAAGTCAT
CTCTGGCTTTTAACACC / Celera SNP ID:    hCV29855684 / Public SNP ID:    rs6120816 /
SNP Chromosome Position:   33644358 /  SNP in Genomic Sequence:   SEQ ID NO: 373 /
SNP Position Genomic:   64151 / Related Interrogated SNP:   hCV1825046 / SNP
Source:   dbSNP / Population(Allele,Count):   Caucasian (G,83|C,143) / SNP Type:
  INTRON /
Context                    (SEQ ID NO: 1544): /
AGATTGCGCCATTCCACTCCAGCCGGGCAACAGTGCGAGACTCCGTCCCAAAAAAAAAAAAAAGGTATACACAAGGCAAAACAAT
TTAAAAGGATGCATTAT
W
CCTGACTTCACCACCATGCGATACACCAATGTAGCAAAATTTCACTTGTACCCCATTAATACAAATTTAAAAAACAGTAAAATA
AATTAAAGGATGCACTA / Celera SNP ID:    hCV30126097 / Public SNP ID:    rs6120827 /
SNP Chromosome Position:   33672123 /  SNP in Genomic Sequence:   SEQ ID NO: 373 /
SNP Position Genomic:   91916 / Related Interrogated SNP:   hCV1825046 / SNP
Source:   dbSNP; HapMap; ABI_Val / Population(Allele,Count):   Caucasian
(A,36|T,84) / SNP Type:    INTRON /
Context                    (SEQ ID NO: 1545): /
TAAATTAAAAAAAAAAAAAAAACTAAGAAAAGAAATATTTGAAAGATGAAGGTCCCCATTTTATCCTCACTATAGCTCCAGGCAG
GGGGTCTCTACTTCAAC

Y
TCTGAAATAGCTCCCACACCTATATACAACCATCAGTTACCACAATAACCTGCCTTGCTTTTGTGGCTCCAGAATATTCCAGC
AGCCTTGTATCTGGTGA / Celera SNP ID: hCV30180146 / Public SNP ID: rs6060216 /
SNP Chromosome Position: 33678078 / SNP in Genomic Sequence: SEQ ID NO: 373 /
SNP Position Genomic: 97871 / Related Interrogated SNP: hCV1825046 / SNP
Source: dbSNP; ABI_Val / Population(Allele,Count): Caucasian (T,46|C,74) /
SNP Type: INTRON /
Context (SEQ ID NO: 1546): /
AAAAAGTTTGATAATGCTCTGTACTGGGAAAGGCTACAGACAACACGGTGGGGAAGATGAACAAGTAATTTCTTTGGAGGGTT
AATTTGTCAACACTTAC

S
AATTTCCATTAATGGGGGACTAATTAAATTACAGTATGCCTGTATAATAGAATATTATATAATTACAAAAAGGAATCATATAA
TTCTATATATACTAGAG / Celera SNP ID: hCV30540259 / Public SNP ID: rs6087664 /
SNP Chromosome Position: 33626216 / SNP in Genomic Sequence: SEQ ID NO: 373 /
SNP Position Genomic: 46009 / Related Interrogated SNP: hCV1825046 / SNP
Source: dbSNP / Population(Allele,Count): Caucasian (C,84|G,142) / SNP Type:
INTRON /
Context (SEQ ID NO: 1547): /
AACATGGGAGACAGAGGTTGCAGTGAGCCGAGATTGTGCCACTGCCCTCCAACCTGGGCGACAAGAGACTCCATCTCAAAAAA
GGACATTTTAAGTAATA

Y
ACTGTAGCAACTCTGGATACTGATTCCCCCTCCTTCAGGGTTTGTTGTTGTTATTTGCTTATTTATTAGTTTAGTGACTTGGT
TAGACTATTTTCGTAAA / Celera SNP ID: hCV30612241 / Public SNP ID: rs6060168 /
SNP Chromosome Position: 33621017 / SNP in Genomic Sequence: SEQ ID NO: 373 /
SNP Position Genomic: 40810 / Related Interrogated SNP: hCV1825046 / SNP
Source: dbSNP / Population(Allele,Count): Caucasian (T,126|C,100) / SNP Type:
INTRON /
Context (SEQ ID NO: 1548): /
ACTGATTTTCAAATGTTGAACCAAACTTGCATAAATCCATTTGGTCATGGTATACAATCCTTGTATGTTGCTATACTTGGTGT
GCTAGTATTTTGTTGAA

K
ATTTTTGCACCTATAATCATAAGAGATACTGGTCTTGTGATGTCTTTATCTGGTTTTTGGTATCATGATAATATGACCTCAAAG
AGTAAGTTGGGAACTGT / Celera SNP ID: hCV30630342 / Public SNP ID: rs6141526 /
SNP Chromosome Position: 33615255 / SNP in Genomic Sequence: SEQ ID NO: 373 /
SNP Position Genomic: 35048 / Related Interrogated SNP: hCV1825046 / SNP
Source: dbSNP; HapMap / Population(Allele,Count): Caucasian (G,69|T,51) / SNP
Type: INTRON /
Context (SEQ ID NO: 1549): /
CGTCATGTTAAAATCACAACCTTGGAAGACGACAAGCTGCTGAATGAAGTGTGACTGAGACACAGTGTGACACGGTAGTTAAG
AGCACTGACCCTGAAGC

Y
AGACTACTGGGTTTGAACCCCATTCACTAGCCGGGTGGTCTTCAGTAAATCAGTGGACCTCTCTGTGCTTCAGTATCCATCCT
CCTTTGTAAAACAGCAA / Celera SNP ID: hCV32066118 / Public SNP ID: rs7271729 /
SNP Chromosome Position: 33610992 / SNP in Genomic Sequence: SEQ ID NO: 373 /
SNP Position Genomic: 30785 / Related Interrogated SNP: hCV25620145 / SNP
Source: dbSNP / Population(Allele,Count): Caucasian (C,110|T,10) / SNP Type:
INTRON /
Context (SEQ ID NO: 1550): /
AAGAGTATTGAACTGTTTATCATGAGCCCAGGAAAAGATCAAAATTCAAAATTCTACTTCTGTACCATTGTGAAGTTGAAAAA
TTCTAAGTGGACCATCT

S
TATATAAAATCCAGGCCTGTGACTGTGACCCACAACACCCTGTGACTGTGACCCACAACGTCCTGTGCAGCCCAGCCCAGAGC
CAAGCCCACATCACTCC / Celera SNP ID: hCV32066123 / Public SNP ID: rs7273734 /
SNP Chromosome Position: 33599403 / SNP in Genomic Sequence: SEQ ID NO: 373 /
SNP Position Genomic: 19196 / Related Interrogated SNP: hCV25620145 / SNP
Source: dbSNP / Population(Allele,Count): Caucasian (G,204|C,22) / SNP Type:
INTRON /
Context (SEQ ID NO: 1551): /
GCAAGCTGATCCTGAAATTCACATGCAAATATAAGGGATGCATAACAGCCAAAACAATCTTGATCTGTAAATACAAAAATAAA
CCCGTGTCTATGATCAG

Y
TGATTTTTGACAAGGTTGCCAAGACAATTCAATGATGAAAGAGTAGTTTTTTTCAATAAGTGGTGCTGTGACAACTGGATAGTC
ACATGCAAAAGAATGAA / Celera SNP ID: hCV32066768 / Public SNP ID: rs7263253 /

293

SNP Chromosome Position: 33649376 / SNP in Genomic Sequence: SEQ ID NO: 373 / SNP Position Genomic: 69169 / Related Interrogated SNP: hCV25620145 / SNP Source: dbSNP; HapMap / Population(Allele,Count): Caucasian (T,106|C,8) / SNP Type: TRANSCRIPTION FACTOR BINDING SITE;INTRON /
Context (SEQ ID NO: 1552): /
AGTGTAAAACCTCTGTACATGAGCAAGCTAGGGCAAAGGTGATCAGAGCCATAGAATTCTTGGCCTCAAGTATGTGGGATAGA
GCTTCCACACCGTGAGT
M
CAGTATGGAGCTGGGTGGAGGAAGGAAGCCTCTGACCTCTTTGCCATACTCACCGAGAACTTAGTCTCTTCAATTTGGAGCTG
GAGGGAAGAAGAAAAGC / Celera SNP ID: hDV71833331 / Public SNP ID: rs6142280 / SNP Chromosome Position: 33622242 / SNP in Genomic Sequence: SEQ ID NO: 373 / SNP Position Genomic: 42035 / Related Interrogated SNP: hCV1825046 / SNP Source: dbSNP; HapMap / Population(Allele,Count): Caucasian (A,126|C,100) / SNP Type: TRANSCRIPTION FACTOR BINDING SITE;INTRON //

Gene Number: 37 / Gene Symbol: OBP2B - 29989 / Gene Name: odorant binding protein 2B / Chromosome: 9 / OMIM NUMBER: 604606 / OMIM Information: // Genomic Sequence (SEQ ID NO: 374): // / SNP Information /
Context (SEQ ID NO: 1553): /
ACCCCACCGAGCTTCAGGATGCCCAGGCTGTTTCCCTCCAAGGCAGGGGGTGACTCTTCCCAACACCCGAAACGTGGATGGGG
CAATGGGCACCAGGTGG
R
TCTGGGGAGGGGAGCGAAAGTGGCCTGAGGGGCCCTGCAGTGGGCAACACTCACATGAAGGTGAACGTGGCTTCCAACTTCCC
ACCGCCCAGGGCTGTCA / Celera SNP ID: hCV25987572 / Public SNP ID: rs4310274 / SNP Chromosome Position: 136083801 / SNP in Genomic Sequence: SEQ ID NO: 374 / SNP Position Genomic: 13135 / Related Interrogated SNP: hCV25610857 / Related Interrogated SNP: hCV27859399 / SNP Source: dbSNP / Population(Allele,Count): Caucasian (A,33|G,193) / SNP Type: INTRON /
Context (SEQ ID NO: 1554): /
CTTTGTGCCTCTCTGGCCCAAGATCAGACACTCTCAAGTACTTACTCAATAATACTAATGCACACTCTAATAAACCCTGGGGC
TGTAACGAACGGGCTGG
Y
GTGACCCAGTCCCTCACCCCTCCCACGGCAAGTGCTGCCGAGCAGAAACCCCCCACCCTCATCCCCCCGGGCAGGAAAGAATG
GGGCCTGGGCTCTTCAG / Celera SNP ID: hCV2980259 / Public SNP ID: rs3761821 / SNP Chromosome Position: 136085783 / SNP in Genomic Sequence: SEQ ID NO: 374 / SNP Position Genomic: 15117 / Related Interrogated SNP: hCV25610857 / Related Interrogated SNP: hCV27859399 / SNP Source: dbSNP; Celera; HGBASE / Population(Allele,Count): Caucasian (T,22|C,94) / SNP Type: INTERGENIC;UNKNOWN /
Context (SEQ ID NO: 1555): /
GGTGGGGGAGGGTGGGGGTGGGAGTGGGGGAGGGGCTCACAGGCGCTGTATTTGCCAGGCTCCTCCGTCTTCCGCATCAGGAT
TTTCTTCTGGATGCACC
K
ATCCTCCCTCCTGGAAAACAGGAGACACGCGGGCAGCGGCTCCCAGGACACCCATGGCCCATCCTCAGCTCACCTGGTGCATG
GCCCTGACCCGGCAACC / Celera SNP ID: hCV27224736 / Public SNP ID: rs2073870 / SNP Chromosome Position: 136083580 / SNP in Genomic Sequence: SEQ ID NO: 374 / SNP Position Genomic: 12914 / Related Interrogated SNP: hCV25610857 / Related Interrogated SNP: hCV27859399 / SNP Source: dbSNP / Population(Allele,Count): Caucasian (T,32|G,192) / SNP Type: ACCEPTOR SPLICE SITE;ESS;ESE;SILENT MUTATION /
Context (SEQ ID NO: 1556): /
ATTCCAGGGATGGAAACCTGGTACAATACTCAAAAATCAACCATATTAATATGTTAAGAAGAAAAATTACATGATCATATCGA
TCAATGCAGAAAAAGTA
Y
TTGACTAAATTCATCACGCATTCAGGAAAACAACTCTCAGAAAAGTAGGAATAGAAGAGAACCTCCTTAACTTGACAAAGAGC
ATCTACAAAAAACCTAT / Celera SNP ID: hCV27224742 / Public SNP ID: rs4454354 / SNP Chromosome Position: 136089529 / SNP in Genomic Sequence: SEQ ID NO: 374 / SNP Position Genomic: 18863 / Related Interrogated SNP: hCV25610857 / Related Interrogated SNP: hCV27859399 / SNP Source: dbSNP; Celera; HapMap / Population(Allele,Count): Caucasian (T,47|C,179) / SNP Type: INTERGENIC;UNKNOWN /
Context (SEQ ID NO: 1557): /
TTCCCTGCCTGCTGGCCTCTGCCCTGACCGTGGGCCAGCTGTGGCTGGGACAACCCAGCAAACTTCTCCAGCTTCCCTTGGGA

294

GAACCACACCTTCTCCC
R
TGAGGTCTGAACCCAGACTTGTGGAAAGGATCCCCACCCCTGTTTATTTCTTCCTTAACTGCCCTCGTGCAATCCTGAGCCAT
TCTTTGTGGTTCTCTTT / Celera SNP ID: hCV27224746 / Public SNP ID: rs10793959 /
SNP Chromosome Position: 136093199 / SNP in Genomic Sequence: SEQ ID NO: 374 /
SNP Position Genomic: 22533 / Related Interrogated SNP: hCV25610857 /
Related Interrogated SNP: hCV27859399 / SNP Source: dbSNP; Celera; HapMap /
Population(Allele,Count): Caucasian (G,40|A,186) / SNP Type:
INTERGENIC;UNKNOWN /
Context (SEQ ID NO: 1558): /
GGGAGCTAACGAGCTCAGCTGGATCATGGCAGCTTTATGCCTGGAGGGAATTTCAATCCACCACAGAGAGAGGAACCCAACAG
AGCAAGGAGTCAGACGG
M
GTGGACGGGACACAGATTGGACTCTAGGCATCGCAGACGACTAGAATGTGGGAGACAAGGAAAGAGATAGGAGAGCCCTCTGC
AGAGGAAGAGCTCCAGA / Celera SNP ID: hCV27224748 / Public SNP ID: rs4246169 /
SNP Chromosome Position: 136094150 / SNP in Genomic Sequence: SEQ ID NO: 374 /
SNP Position Genomic: 23484 / Related Interrogated SNP: hCV25610857 /
Related Interrogated SNP: hCV27859399 / SNP Source: dbSNP; Celera; HapMap /
Population(Allele,Count): Caucasian (C,18|A,102) / SNP Type:
INTERGENIC;UNKNOWN /
Context (SEQ ID NO: 1559): /
GCCACCATGCTGAAGCATCAGTTACAAAATGGACTTGATTTGCTGTGTGAGTGGGTCAGTGCAGTGCAAGGGGTGGCTGAAGG
CACTGGTGACCCACGCT
S
TCGGCCCACCTGCCACAGTTGTAGATCGTTCCTGGTGCACGGACGTATTCACAGCTTCCCACCTTAAGTGCCTGCACCTTTTC
TTCTGTGCTTCCCCTGA / Celera SNP ID: hCV27478783 / Public SNP ID: rs3761823 /
SNP Chromosome Position: 136086812 / SNP in Genomic Sequence: SEQ ID NO: 374 /
SNP Position Genomic: 16146 / Related Interrogated SNP: hCV25610857 /
Related Interrogated SNP: hCV27859399 / SNP Source: dbSNP; HapMap; ABI_Val;
HGBASE / Population(Allele,Count): Caucasian (C,27|G,93) / SNP Type:
INTERGENIC;UNKNOWN /
Context (SEQ ID NO: 1560): /
ATCCTCAGTCTGTAGGGATAGTAACTGATGGGATAAATGGTCTGGACTCTTCCTTCTAAGTGGAACAATCAAATATGGTTCCA
CCCTATTTCTCAGATGG
Y
CCTCAGAAGGACTGAGTCCCAGTTGCCCACAATGGTAACATCATTAACATATTTTGTTGGTTTTTCTACCTTCCAAATCTCAC
TTGATCTCAGACTTCTG / Celera SNP ID: hCV27886018 / Public SNP ID: rs4962104 /
SNP Chromosome Position: 136087047 / SNP in Genomic Sequence: SEQ ID NO: 374 /
SNP Position Genomic: 16381 / Related Interrogated SNP: hCV25610857 /
Related Interrogated SNP: hCV27859399 / SNP Source: dbSNP; HapMap; ABI_Val;
HGBASE / Population(Allele,Count): Caucasian (T,47|C,177) / SNP Type:
INTERGENIC;UNKNOWN /
Context (SEQ ID NO: 1561): /
AAATTTTTAAAAATTTAAATGATATAGAGGGTAGTCTCTGACCACATGGAATCTAACTATAAATCAATGCCAGAAAGATAATG
GAAAATCTTTCTTCAGA
K
GCTTGGAAACAACACACCTGTGAATAATCCACAGGTCAAAGAGGAAGCCTGAAGGTAAATTTAAAAATATACTAGGCTGGATG
AAAATGAAAATACAACA / Celera SNP ID: hCV32126443 / Public SNP ID: rs10793957 /
SNP Chromosome Position: 136087931 / SNP in Genomic Sequence: SEQ ID NO: 374 /
SNP Position Genomic: 17265 / Related Interrogated SNP: hCV25610857 /
Related Interrogated SNP: hCV27859399 / SNP Source: dbSNP; HapMap /
Population(Allele,Count): Caucasian (G,22|T,98) / SNP Type:
INTERGENIC;UNKNOWN /
Context (SEQ ID NO: 1562): /
TGGGAAGGCCAGGGTGGGGCCAAGGATCTCGGAGACCAGGGTCGGCTCTGCCTTGCTCTGGGGGTGATGATGGAGGCGCCAGT
GTCCCTCTCTCCAGCGC
Y
CCTCTCTCCAGTGCTGCCACTGAGCCCAGGCTGCTAAAATGGGGGAGCTTGGAGGATCCCTCTGAGACCCCTGAGGCGGGAGC
CCTGCGGTGGCCCCACT / Celera SNP ID: hCV32126435 / Public SNP ID: rs11244034 /
SNP Chromosome Position: 136080272 / SNP in Genomic Sequence: SEQ ID NO: 374 /
SNP Position Genomic: 9606 / Related Interrogated SNP: hCV25610857 / Related
Interrogated SNP: hCV27859399 / SNP Source: dbSNP / Population(Allele,Count):
Caucasian (T,33|C,191) / SNP Type: INTERGENIC;UNKNOWN /

Context (SEQ ID NO: 1563): /
CCCAGATTCAAGTGATTCTCCTGCCTCCGCCTCCCAAGTAGCTGGGATTACAGATGCACACCACCATACCCAGCTACAGGCTA
CCCTTTCAAGACTCATT
R
TATAGCTAGCAGATGGACAGATGCATAGATCAATGGGACAGACAATAAAGAATCCTCAAACAGACCTGCACGCATATACCCAG
TTAAGTTTTTACAAAGA / Celera SNP ID: hCV32126447 / Public SNP ID: rs6597610 /
SNP Chromosome Position: 136091096 / SNP in Genomic Sequence: SEQ ID NO: 374 /
SNP Position Genomic: 20430 / Related Interrogated SNP: hCV25610857 /
Related Interrogated SNP: hCV27859399 / SNP Source: dbSNP; HapMap /
Population(Allele,Count): Caucasian (G,40|A,186) / SNP Type:
INTERGENIC;UNKNOWN /
Context (SEQ ID NO: 1564): /
GCCCACCTGCCACAGTTGTAGATCGTTCCTGGTGCACGGACGTATTCACAGCTTCCCACCTTAAGTGCCTGCACCTTTTCTTC
TGTGCTTCCCCTGACAC
Y
GATGAAACCCAGATGTGCAAGTGAATCAGCATCCTCAGTCTGTAGGGATAGTAACTGATGGGATAAATGGTCTGGACTCTTCC
TTCTAAGTGGAACAATC / Celera SNP ID: hCV32126442 / Public SNP ID: rs7864821 /
SNP Chromosome Position: 136086916 / SNP in Genomic Sequence: SEQ ID NO: 374 /
SNP Position Genomic: 16250 / Related Interrogated SNP: hCV25610857 /
Related Interrogated SNP: hCV27859399 / SNP Source: dbSNP /
Population(Allele,Count): Caucasian (C,40|T,186) / SNP Type:
INTERGENIC;UNKNOWN //

Gene Number: 38 / Gene Symbol: GP6 - 51206 / Gene Name: glycoprotein VI
(platelet) / Chromosome: 19 / OMIM NUMBER: 605546 / OMIM Information: //
Genomic Sequence (SEQ ID NO: 375): // / SNP Information /
Context (SEQ ID NO: 1565): /
TTGGATACGACCGTGCCTGGGGTTCAGCGGTCATGAACATAACCCGCGGCTGTGAACATCCTGTCGGCCTCCATCCTGACCCC
CGTTTGATTTCCGGGTC
W
GCGGGAGGGGCGGGAGGGGCGGAAGCGGCCTCTGCACAGCCCTGCCCCTGTGCCGCAGGCGCTTCCTCCGGCTGTGCCAGTCC
TCTGCCAGAAACCCCGC / Celera SNP ID: hCV1376266 / Public SNP ID: rs1654413 /
SNP Chromosome Position: 55526359 / SNP in Genomic Sequence: SEQ ID NO: 375 /
SNP Position Genomic: 11286 / SNP Source: Applera / Population(Allele,Count):
Caucasian (A,0|T,24) African American (A,12|T,16) total (A,12|T,40) / SNP
Type: MISSENSE MUTATION;SILENT MUTATION;INTRON;PSEUDOGENE / SNP Source:
dbSNP; Celera; HGBASE / Population(Allele,Count): Caucasian (A,21|T,99) / SNP
Type: MISSENSE MUTATION;SILENT MUTATION;INTRON;PSEUDOGENE /
Context (SEQ ID NO: 1566): /
TAAAAGCCTACAGGCTTAGATAATGGAAGAGAGAGCTCCGTCCTCACACTCCTTTCTGCTGAGCATGAAATGCCTGGTTACTC
ACCAGTTGTGAAGACTT
Y
GTTTGTGAATGAGACGGTCAGTTCAGCGGTGGCTTCTGAGAATTCTAAGAAAGCAAAACAATGTTAGGTCTTCCCCGTGGTTC
CCTATATCCTCTAGATA / Celera SNP ID: hCV1376342 / Public SNP ID: rs1654416 /
SNP Chromosome Position: 55530035 / SNP in Genomic Sequence: SEQ ID NO: 375 /
SNP Position Genomic: 14962 / SNP Source: Applera / Population(Allele,Count):
Caucasian (C,0|T,16) African American (C,8|T,22) total (C,8|T,38) / SNP Type:
MISSENSE MUTATION;ESE;UTR5;ESE SYNONYMOUS;INTRON;PSEUDOGENE / SNP Source:
dbSNP; Celera; HapMap; ABI_Val; HGBASE / Population(Allele,Count): Caucasian
(C,47|T,179) / SNP Type: MISSENSE MUTATION;ESE;UTR5;ESE
SYNONYMOUS;INTRON;PSEUDOGENE /
Context (SEQ ID NO: 1567): /
GACAAAGGAGTTGGCTTTGGTGAAGAGACGGGTGAGAAGGAAGGGGGTCTGGAGAGGATGACTTACTCACCAGCTGGAGAGTC
TGACTCCTTTGGACTGG
Y
GGTGATACTCCTAGAAGTCTCTGGGAACCAAACAAAGGCTAAGTGTGAAATGAAACCATATTCCCGCCCCCTGTCACTGTGCC
TACTCCGAACACACACA / Celera SNP ID: hCV15973734 / Public SNP ID: rs2304167 /
SNP Chromosome Position: 55527081 / SNP in Genomic Sequence: SEQ ID NO: 375 /
SNP Position Genomic: 12008 / SNP Source: dbSNP; HapMap; ABI_Val /
Population(Allele,Count): Caucasian (C,48|T,178) / SNP Type: MISSENSE
MUTATION;ESE;UTR5;INTRON;PSEUDOGENE /
Context (SEQ ID NO: 1568): /
ATTAAATCGAGAAGTCTAGGCAGAGAGGAGAGAGAGAAGGGGTCCGTGTACCTCATACGCTGTGCACCAGAATGGACCCTGCA

GAACCTACCTGCTACCG
R
GGAAGGTGGTTCTGTTGGTAACCGGCTGGGGGTCACAGAGGTTCCTGGGAAATCAGAAAATGAGATAAATCTGTGCTCTGTCG
CTGTGGGTCCTGAACAA / Celera SNP ID: hCV8717873 / Public SNP ID: rs1613662 /
SNP Chromosome Position: 55536595 / SNP in Genomic Sequence: SEQ ID NO: 375 /
SNP Position Genomic: 21522 / SNP Source: dbSNP; HapMap; HGBASE /
Population(Allele,Count): Caucasian (G,43|A,183) / SNP Type: MISSENSE
MUTATION;UTR5;INTRON /
Context (SEQ ID NO: 1569): /
CCATGATCCCTCCCTTGGATACGACCGTGCCTGGGGTTCAGCGGTCATGAACATAACCCGCGGCTGTGAACATCCTGTCGGCC
TCCATCCTGACCCCCGT
K
TGATTTCCGGGTCAGCGGGAGGGGCGGGAGGGGCGGAAGCGGCCTCTGCACAGCCCTGCCCCTGTGCCGCAGGCGCTTCCTCC
GGCTGTGCCAGTCCTCT / Celera SNP ID: hCV1376265 / Public SNP ID: rs1671152 /
SNP Chromosome Position: 55526345 / SNP in Genomic Sequence: SEQ ID NO: 375 /
SNP Position Genomic: 11272 / Related Interrogated SNP: hCV8717873 / Related
Interrogated SNP: hCV1376342 / Related Interrogated SNP: hCV15973734 /
Related Interrogated SNP: hCV1376266 / Related Interrogated SNP: hCV8718961 /
SNP Source: Applera / Population(Allele,Count): Caucasian (G,28|T,0) African
American (G,25|T,3) total (G,53|T,3) / SNP Type: MISSENSE
MUTATION;INTRON;PSEUDOGENE / SNP Source: dbSNP; Celera; HapMap; HGBASE /
Population(Allele,Count): Caucasian (T,41|G,181) / SNP Type: MISSENSE
MUTATION;INTRON;PSEUDOGENE /
Context (SEQ ID NO: 1570): /
TAACCTAACCACAAGACTAACCTACTGTCACTTCTGCCCTATCCTACTGCTAGAAGCCAGTCGCTACATCTCCCCCACACTCA
AAGGGAGGTGGTCGCAC
Y
GTGGGGTCCACTGGAAGTTGCCTACCAGACTCAGGTCATCCCAAACACACCCTCTAGCCATTTGGTGTGGCATCGGAAAGAAA
ACTAAGGCCAGGTACGG / Celera SNP ID: hCV8717751 / Public SNP ID: rs1671218 /
SNP Chromosome Position: 55554399 / SNP in Genomic Sequence: SEQ ID NO: 375 /
SNP Position Genomic: 39326 / Related Interrogated SNP: hCV8717873 / SNP
Source: Applera / Population(Allele,Count): Caucasian (C,13|T,17) African
American (C,12|T,24) total (C,25|T,41) // / SNP Type: INTRON;PSEUDOGENE /
SNP Source: Applera / Population(Allele,Count): Caucasian (C,16|T,24) African
American (C,12|T,24) total (C,28|T,48) // / SNP Type: INTRON;PSEUDOGENE /
SNP Source: dbSNP; HapMap; HGBASE / Population(Allele,Count): Caucasian
(C,38|T,82) / SNP Type: INTRON;PSEUDOGENE /
Context (SEQ ID NO: 1571): /
ACGCACGTGGTTGTTGGTGTGATTCTGTTTCTCCAGATTGTCTCCATTCCTCCCTGGCTTCTCCACAGGGCCGCTCACCATGG
CAGCCGGCTCCATCACC
R
CCAGCCAGCGAGAGGGCAAGACAAGAGGGCTGACGAGGGACGCTACCATCACAGAGGTCAGTTTTGTAACCTAACCACAAGAC
TAACCTACTGTCACTTC / Celera SNP ID: hCV8717752 / Public SNP ID: rs1671217 /
SNP Chromosome Position: 55554232 / SNP in Genomic Sequence: SEQ ID NO: 375 /
SNP Position Genomic: 39159 / Related Interrogated SNP: hCV8717873 / Related
Interrogated SNP: hCV1376342 / Related Interrogated SNP: hCV8718961 / Related
Interrogated SNP: hCV1376266 / Related Interrogated SNP: hCV15973734 / SNP
Source: Applera / Population(Allele,Count): Caucasian (A,1|G,17) African
American (A,2|G,26) total (A,3|G,43) / SNP Type: MISSENSE
MUTATION;ESE;INTRON;PSEUDOGENE / SNP Source: Applera /
Population(Allele,Count): Caucasian (A,3|G,37) African American (A,4|G,30)
total (A,7|G,67) / SNP Type: MISSENSE MUTATION;ESE;INTRON;PSEUDOGENE / SNP
Source: dbSNP; HapMap; HGBASE / Population(Allele,Count): Caucasian
(A,21|G,99) / SNP Type: MISSENSE MUTATION;ESE;INTRON;PSEUDOGENE /
Context (SEQ ID NO: 1572): /
TAAAAACCACTTCCTTTGCTCATTTATCAAACTCAAACGCTAGGAGGGCCACCTAACATCCTCCGTCCCACGCAATGGGGTGT
TTCTGGAGCACTCCGGT
K
TATCAGGGACCCTGTCAGTTGCCATCGCACATGTATATGGGGCCAGCCCCTGTGCCACCGAAGAGGGGGGATATTGAAAACAT
GTTACAGCCAGGAGCGG / Celera SNP ID: hCV8717761 / Public SNP ID: rs1654439 /
SNP Chromosome Position: 55553647 / SNP in Genomic Sequence: SEQ ID NO: 375 /
SNP Position Genomic: 38574 / Related Interrogated SNP: hCV8717873 / Related
Interrogated SNP: hCV1376342 / Related Interrogated SNP: hCV8718961 / Related

Interrogated SNP: hCV1376266 / Related Interrogated SNP: hCV15973734 / SNP Source: Applera / Population(Allele,Count): Caucasian (G,33|T,3) African American (G,25|T,7) total (G,58|T,10) / SNP Type: INTRON;PSEUDOGENE / SNP Source: dbSNP; HapMap; HGBASE / Population(Allele,Count): Caucasian (T,45|G,179) / SNP Type: INTRON;PSEUDOGENE /
Context (SEQ ID NO: 1573): /
CTGGGGGCTGACCACAGGTATGGGTCCCTGCTGGAGAAGCTGTAGCATCGGTAGGTTCCGCTGTGGGCGGCGGTCACCGTGAT
GATGGGAAAACTAGCCC
K
GTACCATCTCTCGGGATTCTTGTAGGGCGCAGGGTCCCCTTCCTTGTACAGAGCAAATTGGTCAAAGCCATACCGAGTCTGAC
ACTGTAGGGTTACGTCC / Celera SNP ID: hCV8717845 / Public SNP ID: rs892090 /
SNP Chromosome Position: 55539072 / SNP in Genomic Sequence: SEQ ID NO: 375 /
SNP Position Genomic: 23999 / Related Interrogated SNP: hCV8717873 / Related Interrogated SNP: hCV1376342 / Related Interrogated SNP: hCV1376266 / Related Interrogated SNP: hCV8718961 / Related Interrogated SNP: hCV15973734 / SNP Source: Applera / Population(Allele,Count): Caucasian (G,36|T,2) African American (G,29|T,5) total (G,65|T,7) / SNP Type: UTR5;SILENT MUTATION;INTRON;PSEUDOGENE / SNP Source: dbSNP; HapMap; HGBASE / Population(Allele,Count): Caucasian (T,43|G,183) / SNP Type: UTR5;SILENT MUTATION;INTRON;PSEUDOGENE /
Context (SEQ ID NO: 1574): /
CCAGGGGGTCGCTGGGGGCTGACCACAGGTATGGGTCCCTGCTGGAGAAGCTGTAGCATCGGTAGGTTCCGCTGTGGGCGGCG
GTCACCGTGATGATGGG
R
AAACTAGCCCTGTACCATCTCTCGGGATTCTTGTAGGGCGCAGGGTCCCCTTCCTTGTACAGAGCAAATTGGTCAAAGCCATA
CCGAGTCTGACACTGTA / Celera SNP ID: hCV8717846 / Public SNP ID: rs892089 /
SNP Chromosome Position: 55539061 / SNP in Genomic Sequence: SEQ ID NO: 375 /
SNP Position Genomic: 23988 / Related Interrogated SNP: hCV8717873 / Related Interrogated SNP: hCV1376342 / Related Interrogated SNP: hCV15973734 / Related Interrogated SNP: hCV1376266 / Related Interrogated SNP: hCV8718961 /
SNP Source: Applera / Population(Allele,Count): Caucasian (A,2|G,36) African American (A,15|G,21) total (A,17|G,57) / SNP Type: ESS;UTR5;SILENT MUTATION;INTRON;PSEUDOGENE / SNP Source: dbSNP; Celera; HapMap; ABI_val; HGBASE / Population(Allele,Count): Caucasian (A,22|G,98) / SNP Type: ESS;UTR5;SILENT MUTATION;INTRON;PSEUDOGENE /
Context (SEQ ID NO: 1575): /
AAACGCTCCTCCTTCTGAACCCCAGAGCTCCACTCTGCACCCATGCTCTAGCCTCACACCAAGGACTTTCTTGGTAAGAGACG
GACAGTTCGGTGAAGTG
R
TTAAAAGCCTACAGGCTTAGATAATGGAAGAGAGAGCTCCGTCCTCACACTCCTTTCTGCTGAGCATGAAATGCCTGGTTACT
CACCAGTTGTGAAGACT / Celera SNP ID: hCV8717893 / Public SNP ID: rs1671192 /
SNP Chromosome Position: 55529933 / SNP in Genomic Sequence: SEQ ID NO: 375 /
SNP Position Genomic: 14860 / Related Interrogated SNP: hCV8717873 / Related Interrogated SNP: hCV1376342 / Related Interrogated SNP: hCV15973734 / Related Interrogated SNP: hCV1376266 / Related Interrogated SNP: hCV8718961 /
SNP Source: Applera / Population(Allele,Count): Caucasian (A,1|G,21) African American (A,12|G,18) total (A,13|G,39) / SNP Type: INTRON;PSEUDOGENE / SNP Source: dbSNP; HapMap; HGBASE / Population(Allele,Count): Caucasian (A,19|G,91) / SNP Type: INTRON;PSEUDOGENE /
Context (SEQ ID NO: 1576): /
TCCTCTGTCTTCTGATAGCATCTCAGTATGATTTTCCACTATGTTAAGACGAGGAATTGACCCATTCTTACATTTGGAGGCTT
CTCTAAGCAACTTTCCC
R
TTCCCCCTTCACCCAAGCTGTGTGCTCACTAGCCCTGATTCACAGCCGTCGTCCTGGAACTTCTTGGTGCCATCCTTCTGTCT
TTTCCCAAGTGACTCAC / Celera SNP ID: hCV1376257 / Public SNP ID: rs10416380 /
SNP Chromosome Position: 55524905 / SNP in Genomic Sequence: SEQ ID NO: 375 /
SNP Position Genomic: 9832 / Related Interrogated SNP: hCV1376342 / Related Interrogated SNP: hCV15973734 / Related Interrogated SNP: hCV8717873 / Related Interrogated SNP: hCV1376266 / SNP Source: dbSNP; Celera; HapMap / Population(Allele,Count): Caucasian (A,49|G,173) / SNP Type: INTRON /
Context (SEQ ID NO: 1577): /
ATATTTATGGGGTGGACAGCAATATTGCAGTACATGTATACAACGTGCTATGATCAAATCAGGGTAATTGACATATTCATCCC
TGTATTTTTTGAGACAA

R
GTCAGGCTTCGTCACCCGAGCTAGAGTGCAGTGGTGTGATCTCAGCTCACTGCAACCTCTGCCTCCCAGGCTCAAGCCATTCT
CCCACCTCAGCCCCCTG / Celera SNP ID: hCV1376262 / Public SNP ID: rs1671150 /
SNP Chromosome Position: 55525497 / SNP in Genomic Sequence: SEQ ID NO: 375 /
SNP Position Genomic: 10424 / Related Interrogated SNP: hCV1376342 / Related
Interrogated SNP: hCV15973734 / Related Interrogated SNP: hCV8717873 /
Related Interrogated SNP: hCV1376266 / SNP Source: dbSNP; Celera; HapMap;
HGBASE / Population(Allele,Count): Caucasian (A,48|G,178) / SNP Type:
MISSENSE MUTATION;ESE;TRANSCRIPTION FACTOR BINDING SITE;MICRORNA;UTR3; / INTRON //

Context (SEQ ID NO: 1578): /
GAGACGAAAGGAGATTTGTTAGACCGCAGTGGGAGATGGAGTGAGGGTGAGAGTTTCTGGGGAAAACCAGACAAGAGCACAGA
GGGCCAAAGGGAAGCAC
R
GGAGGATTTTGCACAGAGGATGGAACAGAGTCAACCCTGAGAGCTGGGAACCTTAGAGATCCGTCTGGAGCCCATATTAGAGA
GGTTGAAGAAAGAGGCC / Celera SNP ID: hCV1376264 / Public SNP ID: rs1671151 /
SNP Chromosome Position: 55525894 / SNP in Genomic Sequence: SEQ ID NO: 375 /
SNP Position Genomic: 10821 / Related Interrogated SNP: hCV1376342 / Related
Interrogated SNP: hCV15973734 / Related Interrogated SNP: hCV8717873 /
Related Interrogated SNP: hCV1376266 / SNP Source: dbSNP; HapMap /
Population(Allele,Count): Caucasian (G,48|A,178) / SNP Type: ESE;UTR3;SILENT
MUTATION;INTRON /
Context (SEQ ID NO: 1579): /
GTATATCCATGTTGTAGTATGTGTCAGAATTTCCTTCTTTTTTAAGGCTGCATAATATTCCATTGCATGTATATAACCACATT
ATGAGGTATGCTGCTCT
Y
TTTTGAAAGAAACCCCCTTTAAGAATGGTAGTCAAGTCCGACGCGGTGGCTCACGCCTGTAATCCCAGCACTTTGGGAGGCCG
AGGCGGGCAGATCATGA / Celera SNP ID: hCV1376359 / Public SNP ID: rs2886412 /
SNP Chromosome Position: 55537942 / SNP in Genomic Sequence: SEQ ID NO: 375 /
SNP Position Genomic: 22869 / Related Interrogated SNP: hCV8717873 / Related
Interrogated SNP: hCV1376342 / Related Interrogated SNP: hCV15973734 /
Related Interrogated SNP: hCV1376266 / Related Interrogated SNP: hCV8718961 /
SNP Source: dbSNP; HapMap / Population(Allele,Count): Caucasian (T,18|C,90) /
SNP Type: INTRON /
Context (SEQ ID NO: 1580): /
GTGCTCCTGGCATGTGGTCGGTCGAAGCCAAGGACACTGCTCAGCATTCTGCAGTGCACAGGACGGCCCCGCCCAGGCGGAGA
ATGATCCGGTGACACAT
R
TAGGTGGGAAGGATGCACGAATGATGGCGTTTAGGAAGAATATTATCACTTCTTTCCCGTAAGAGCAACTTAGAGCAAGAAAA
TGGTATTATTCTTAGGG / Celera SNP ID: hCV1376386 / Public SNP ID: rs1671214 /
SNP Chromosome Position: 55552823 / SNP in Genomic Sequence: SEQ ID NO: 375 /
SNP Position Genomic: 37750 / Related Interrogated SNP: hCV8717873 / SNP
Source: dbSNP; Celera; HapMap; HGBASE / Population(Allele,Count): Caucasian
(A,75|G,151) / SNP Type: UTR3;INTRON /
Context (SEQ ID NO: 1581): /
TAGGGCCTTCTCTCTTATGGAGGCTCCAAGCCAGGGTTGCCATGGCAGAAGATGCTGGGCTTGCTTTTTCCTTGAGAGAACTG
TACTCAAGATGATGTAA
M
TGTCACCCCGGGTGCCACTTGGGTGCTTTGGAGAAGCGCTCAGACGCGACACGCCGTGACGACTCCGCGGCAGGCAGCCGGAC
CTGTCCTCTGGCGTGCG / Celera SNP ID: hCV1376388 / Public SNP ID: rs1671215 /
SNP Chromosome Position: 55553019 / SNP in Genomic Sequence: SEQ ID NO: 375 /
SNP Position Genomic: 37946 / Related Interrogated SNP: hCV8717873 / SNP
Source: dbSNP; Celera; HapMap; HGBASE / Population(Allele,Count): Caucasian
(C,75|A,151) / SNP Type: TRANSCRIPTION FACTOR BINDING
SITE;UTR3;INTRON;PSEUDOGENE /
Context (SEQ ID NO: 1582): /
CTGCCTTCAGGGGCCTGGTGGCCCTGGAGACAAATCTCCCTCTGTATCTGAGCCTCACTGCCTTGTTCTGTTAAAATGGGGAT
GACTGAATGAGACAGTA
Y
ACAGTAATTTGCAGAGTGCCTGTTGCCTAGCAAGCGCTGGAGTAAGTAAATAGCTTAAGCTTATACTGTGCTGTAAGCTTGTA
TTGCCACATACAATTGT / Celera SNP ID: hCV7841075 / Public SNP ID: rs1671196 /
SNP Chromosome Position: 55539548 / SNP in Genomic Sequence: SEQ ID NO: 375 /
SNP Position Genomic: 24475 / Related Interrogated SNP: hCV8717873 / Related

Interrogated SNP: hCV1376342 / Related Interrogated SNP: hCV1376266 / Related Interrogated SNP: hCV15973734 / Related Interrogated SNP: hCV8718961 / SNP Source: dbSNP; Celera; HapMap; ABI_Val; HGBASE / Population(Allele,Count): Caucasian (C,50|T,176) / SNP Type: INTRON / Context (SEQ ID NO: 1583): / CCCAAAGTGCTGGGATTACAGGCATGAGCCACTGCACCCGACCGGCTATAATTTTTTTTTAATGGAAAACAGCAGATATTGGTG AGTATGCAGAGAAATTG R ACTGCGCGTGCATTGCTGGCAGGGACGTAACATGGCGCCCCTGCTGTGGAAAACAGTTCCAGCAGCTCCTCCAGAAGTTAAAC GTGGGATTGCCATAAAA / Celera SNP ID: hCV8717793 / Public SNP ID: rs1654433 / SNP Chromosome Position: 55550312 / SNP in Genomic Sequence: SEQ ID NO: 375 / SNP Position Genomic: 35239 / Related Interrogated SNP: hCV8717873 / Related Interrogated SNP: hCV8718961 / Related Interrogated SNP: hCV1376266 / Related Interrogated SNP: hCV1376342 / Related Interrogated SNP: hCV15973734 / SNP Source: dbSNP; HapMap; HGBASE / Population(Allele,Count): Caucasian (A,44|G,182) / SNP Type: INTRON / Context (SEQ ID NO: 1584): / CCTGACCTCAGTTGATCCACCTGCCTCGGTCTCCCAAAGTGCTGGGATTACAGGCATGAGCCACTGCACCCGACCGGCTATAA TTTTTTTTAATGGAAAA Y AGCAGATATTGGTGAGTATGCAGAGAAATTGAACTGCGCGTGCATTGCTGGCAGGGACGTAACATGGCGCCCCTGCTGTGGAA AACAGTTCCAGCAGCTC / Celera SNP ID: hCV8717794 / Public SNP ID: rs1654432 / SNP Chromosome Position: 55550280 / SNP in Genomic Sequence: SEQ ID NO: 375 / SNP Position Genomic: 35207 / Related Interrogated SNP: hCV8717873 / Related Interrogated SNP: hCV8718961 / Related Interrogated SNP: hCV1376266 / Related Interrogated SNP: hCV1376342 / Related Interrogated SNP: hCV15973734 / SNP Source: dbSNP; HapMap; HGBASE / Population(Allele,Count): Caucasian (C,44|T,182) / SNP Type: INTRON / Context (SEQ ID NO: 1585): / TCCCCCCAAAAAAAAAAAAAAAAAAAAAAAAAAGCAGCAGCATTTGTAAAGCACACCTGGCACATTCTGGGCTATTAACAAGGAAAT GCATGCAGCTCCCGTCC R CCTTTTTCAACCTCAGTTCTATTTCTTCTGGATTCCTGTGTCCTACCCCTCACTGTGACCCTGGGGGCAAAACAGATTTTTTCT ACCAAAAACTAAATGAT / Celera SNP ID: hCV8717871 / Public SNP ID: rs1654421 / SNP Chromosome Position: 55537462 / SNP in Genomic Sequence: SEQ ID NO: 375 / SNP Position Genomic: 22389 / Related Interrogated SNP: hCV8717873 / Related Interrogated SNP: hCV1376266 / Related Interrogated SNP: hCV1376342 / Related Interrogated SNP: hCV15973734 / SNP Source: dbSNP; HapMap / Population(Allele,Count): Caucasian (A,54|G,164) / SNP Type: INTRON / Context (SEQ ID NO: 1586): / GACCTACTGAAGCAGAAGCCCTGGGGGGCTCAGAAATCTATTCTTTAAGCCTCCAGGTGATTCTTATGCTCATGGAAGTTTGAG AACCGCTGATCAATGCA W TCAGTGACTCAGAAACAGAGTCCCGGACTCTACAGGTTTGTTGGTTGGTTGGTTGGTTGGTTGGTTGGTTGGTTAGTTTGTTTGTTT TTGTCACCCATATTCAA / Celera SNP ID: hCV8717881 / Public SNP ID: rs1654420 / SNP Chromosome Position: 55536206 / SNP in Genomic Sequence: SEQ ID NO: 375 / SNP Position Genomic: 21133 / Related Interrogated SNP: hCV8717873 / Related Interrogated SNP: hCV1376342 / Related Interrogated SNP: hCV1376266 / Related Interrogated SNP: hCV15973734 / Related Interrogated SNP: hCV8718961 / SNP Source: dbSNP; HGBASE / Population(Allele,Count): Caucasian (T,50|A,174) / SNP Type: INTRON / Context (SEQ ID NO: 1587): / ATAATAGCCCTCACTCTGCCCTCAACCCCGCAAATCTCATCCTTATCAACCTCGGCTCTTTCCAGCATGTTTCTCCTGCCTTG GTGCTTCACTCTGAGAC R CAGGGAATGTTAGACACGCCCAGCCTCCAGCCTAGCGTATGATATTCTTAAAGTGCAGGCCGTAGTCTGGTACACCGTATTCA GCTGAGATGTTTGTGAA / Celera SNP ID: hCV9490926 / Public SNP ID: rs1654419 / SNP Chromosome Position: 55535881 / SNP in Genomic Sequence: SEQ ID NO: 375 / SNP Position Genomic: 20808 / Related Interrogated SNP: hCV8717873 / Related Interrogated SNP: hCV1376342 / Related Interrogated SNP: hCV1376266 / Related Interrogated SNP: hCV15973734 / Related Interrogated SNP: hCV8718961 / SNP Source: dbSNP; Celera; HapMap; ABI_Val; HGBASE / Population(Allele,Count): Caucasian (A,50|G,174) / SNP Type: INTRON /

Context (SEQ ID NO: 1588): /
TTATAAATCTTTAATACATAAGCTGGCTTTAAAATTATTGGTAAAATAAGATTAGAAATGTCTTAAGAATTGTTGGCGTTTTT
GTTTGCACTTATTGAAC
R
AGTGGTTTCATGCTTATCCCTGCAGAATACTATGAGATTTGTCATAAGGGTTATAAAACTATAAACCCGGCTGGGCGTGGTGG
CTCACGCCTGTAATCCC / Celera SNP ID: hCV16044361 / Public SNP ID: rs2569513 /
SNP Chromosome Position: 55552144 / SNP in Genomic Sequence: SEQ ID NO: 375 /
SNP Position Genomic: 37071 / Related Interrogated SNP: hCV8717873 / Related
Interrogated SNP: hCV8718961 / Related Interrogated SNP: hCV1376266 / Related
Interrogated SNP: hCV1376342 / Related Interrogated SNP: hCV15973734 / SNP
Source: dbSNP; HapMap; HGBASE / Population(Allele,Count): Caucasian
(G,44|A,182) / SNP Type: UTR3;INTRON /
Context (SEQ ID NO: 1589): /
CTCCTAGAAGTCTCTGGGAACCAAACAAAGGCTAAGTGTGAAATGAAACCATATTCCCGCCCCCTGTCACTGTGCCTACTCCG
AACACACACACACATGG
K
GAGGCACAATTCCACAGCATTTAAGAAAAGCATGGGCCGGGCACGGTGCCTCATGCCTATAATCCCAGCACTTTGGGAGGCTG
AGGTAGGAGGCTGGCTT / Celera SNP ID: hCV26895244 / Public SNP ID: rs1671153 /
SNP Chromosome Position: 55527189 / SNP in Genomic Sequence: SEQ ID NO: 375 /
SNP Position Genomic: 12116 / Related Interrogated SNP: hCV1376342 / Related
Interrogated SNP: hCV15973734 / Related Interrogated SNP: hCV8717873 /
Related Interrogated SNP: hCV1376266 / SNP Source: dbSNP; Celera; HapMap;
HGBASE / Population(Allele,Count): Caucasian (G,48|T,178) / SNP Type: INTRON
/
Context (SEQ ID NO: 1590): /
GGAGAATCACTTGAACCTAGGAGGCAGAGGTTGCAGTGATCTGAGATCGCACCACTGCACTCCAGCCTGGGTGACGCAGTAAG
ACTCCATCTGAAAAAAA
M
AGGCTTAGCCAGGCGTGGTGGCTCACACCTGTAATCCCAGCACTTTGAGAGGCCGAGGCAGGCAGATCACCTGAGGTCAAGAG
TTCAAGACCAGCCTGGC / Celera SNP ID: hCV26895257 / Public SNP ID: rs2886415 /
SNP Chromosome Position: 55537081 / SNP in Genomic Sequence: SEQ ID NO: 375 /
SNP Position Genomic: 22008 / Related Interrogated SNP: hCV8717873 / Related
Interrogated SNP: hCV1376342 / Related Interrogated SNP: hCV15973734 /
Related Interrogated SNP: hCV1376266 / Related Interrogated SNP: hCV8718961 /
SNP Source: dbSNP; Celera; HapMap; HGBASE / Population(Allele,Count):
Caucasian (A,19|C,89) / SNP Type: INTRON /
Context (SEQ ID NO: 1591): /
AGCTCTCTTCCTCTTGGTCAGCCCCAGTCCTCACCCTACCCCATTTCTCCTTTAATAACATCTTATTAAATGCACCTGGCACC
AGCCTCAGGTTAAGGAT
Y
TTTTTTTGGCCAGGCGAGGTGGCTCAGGCCTGTAATCCCAGCACTTTGGGAGGCCGAGGCGGGCGGATTACCTGGGGTCGGGA
GTTCCAGACCAGCCTGG / Celera SNP ID: hCV29271569 / Public SNP ID: rs1626971 /
SNP Chromosome Position: 55557024 / SNP in Genomic Sequence: SEQ ID NO: 375 /
SNP Position Genomic: 41951 / Related Interrogated SNP: hCV8717873 / Related
Interrogated SNP: hCV1376342 / Related Interrogated SNP: hCV8718961 / Related
Interrogated SNP: hCV1376266 / Related Interrogated SNP: hCV15973734 / SNP
Source: dbSNP; HapMap; HGBASE / Population(Allele,Count): Caucasian
(C,21|T,99) / SNP Type: INTRON /
Context (SEQ ID NO: 1592): /
GGAGAAGGGTGTAGGTGTGAATATAAAAGGGTAGCCCAAGGGAGGCCCTTGTGAGACGGAAGAGTTCTGTACAGTGACTGCGG
TGATGGTGACGCGAATC
Y
ACAACTGTGACAAATTGGCATAGAACTAGACACCTACTTTATGCCAATGTCAAATTCCTGGTTTTTATGTTGTACTCTAATTA
CGTAAGATGTAACCATT / Celera SNP ID: hCV31722832 / Public SNP ID: rs11084381 /
SNP Chromosome Position: 55535412 / SNP in Genomic Sequence: SEQ ID NO: 375 /
SNP Position Genomic: 20339 / Related Interrogated SNP: hCV8717873 / Related
Interrogated SNP: hCV1376342 / Related Interrogated SNP: hCV1376266 / Related
Interrogated SNP: hCV15973734 / Related Interrogated SNP: hCV8718961 / SNP
Source: dbSNP; HapMap / Population(Allele,Count): Caucasian (T,50|C,176) /
SNP Type: INTRON /
Context (SEQ ID NO: 1593): /
ATAAAAGGGTAGCCCAAGGGAGGCCCTTGTGAGACGGAAGAGTTCTGTACAGTGACTGCGGTGATGGTGACGCGAATCTACAA
CTGTGACAAATTGGCAT

R
GAACTAGACACCTACTTTATGCCAATGTCAAATTCCTGGTTTTTATGTTGTACTCTAATTACGTAAGATGTAACCATTAGAGG
AAACTGGAAAAAGAGCA / Celera SNP ID:    hCV31722834 / Public SNP ID:    rs11084382 /
SNP Chromosome Position:    55535434 / SNP in Genomic Sequence:    SEQ ID NO: 375 /
SNP Position Genomic:    20361 / Related Interrogated SNP:    hCV8717873 / Related
Interrogated SNP:    hCV1376342 / Related Interrogated SNP:    hCV15973734 /
Related Interrogated SNP:    hCV1376266 / Related Interrogated SNP:    hCV8718961 /
SNP Source:    dbSNP / Population(Allele,Count):    Caucasian (A,56|G,168) / SNP
Type:    INTRON /
Context                (SEQ ID NO: 1594): /
ACAGTGACTGCGGTGATGGTGACGCGAATCTACAACTGTGACAAATTGGCATAGAACTAGACACCTACTTTATGCCAATGTCA
AATTCCTGGTTTTTATG
Y
TGTACTCTAATTACGTAAGATGTAACCATTAGAGGAAACTGGAAAAAGAGCACATGGGATTCTTCTGTTCTATCATTGTAGAC
TTCCTGTGACTCTAGAA / Celera SNP ID:    hCV31722835 / Public SNP ID:    rs11668169 /
SNP Chromosome Position:    55535482 / SNP in Genomic Sequence:    SEQ ID NO: 375 /
SNP Position Genomic:    20409 / Related Interrogated SNP:    hCV8717873 / Related
Interrogated SNP:    hCV1376342 / Related Interrogated SNP:    hCV1376266 / Related
Interrogated SNP:    hCV15973734 / Related Interrogated SNP:    hCV8718961 / SNP
Source:    dbSNP; HapMap / Population(Allele,Count):    Caucasian (T,50|C,174) /
SNP Type:    TRANSCRIPTION FACTOR BINDING SITE;INTRON /
Context        (SEQ ID NO: 1595): /
ACAAAAGGATATCTCAAACGGTTGCATACTGGATGATCCCATTTACATCAGATTCTAGAAATGGAAGATTATAGAGATGGAGA
ACAAATTAATGGATACC
R
GGAGTTAGGGATGGCAAGGGAAGGAGAAGGGTGTAGGTGTGAATATAAAAGGGTAGCCCAAGGGAGGCCCTTGTGAGACGGAA
GAGTTCTGTACAGTGAC / Celera SNP ID:    hCV31722831 / Public SNP ID:    rs11671922 /
SNP Chromosome Position:    55535289 / SNP in Genomic Sequence:    SEQ ID NO: 375 /
SNP Position Genomic:    20216 / Related Interrogated SNP:    hCV8717873 / Related
Interrogated SNP:    hCV1376342 / Related Interrogated SNP:    hCV15973734 /
Related Interrogated SNP:    hCV1376266 / Related Interrogated SNP:    hCV8718961 /
SNP Source:    dbSNP / Population(Allele,Count):    Caucasian (A,22|G,98) / SNP
Type:    INTRON /
Context                (SEQ ID NO: 1596): /
AACTGTGACAAATTGGCATAGAACTAGACACCTACTTTATGCCAATGTCAAATTCCTGGTTTTTATGTTGTACTCTAATTACG
TAAGATGTAACCATTAG
R
GGAAACTGGAAAAAGAGCACATGGGATTCTTCTGTTCTATCATTGTAGACTTCCTGTGACTCTAGAACCATTTCAAAAGAGAA
AGTTCAAAAATTCAGTC / Celera SNP ID:    hCV31722836 / Public SNP ID:    rs11672026 /
SNP Chromosome Position:    55535515 / SNP in Genomic Sequence:    SEQ ID NO: 375 /
SNP Position Genomic:    20442 / Related Interrogated SNP:    hCV8717873 / Related
Interrogated SNP:    hCV1376342 / Related Interrogated SNP:    hCV1376266 / Related
Interrogated SNP:    hCV15973734 / Related Interrogated SNP:    hCV8718961 / SNP
Source:    dbSNP / Population(Allele,Count):    Caucasian (A,47|G,173) / SNP Type:
INTRON //

Gene Number:    39 / Gene Symbol:    NAT8B - 51471 / Gene Name:
N-acetyltransferase 8B (GCN5-related, putative, gene/pseudogene) / Chromosome:    2
/ OMIM NUMBER: / OMIM Information: // Genomic Sequence (SEQ ID NO: 376): //    /
SNP Information /
Context                (SEQ ID NO: 1597): /
GTCCACAGAGAGATGAAACAGCTGCAACCGCTTCTCCCTCAAGGTGGGATCATCAACGGGCAGAGCTCCTACTGTGCCCACCA
CCTTCTCTTCAGATTCA
S
CCACCCAGAAGCAGGAGCCACACTCACTCAGGTAGGATTTGGTGATGTCAGACATGTCTGTGCGCAATGCTATGTCTACATAC
CGCGTCCAGGGTTTTTT / Celera SNP ID:    hCV11541681 / Public SNP ID:    rs2001490 /
SNP Chromosome Position:    73928098 / SNP in Genomic Sequence:    SEQ ID NO: 376 /
SNP Position Genomic:    10462 / SNP Source:    Applera / Population(Allele,Count):
Caucasian (C,10|G,10)  African American (C,18|G,14) total (C,28|G,24) //    /
SNP Type:    MISSENSE MUTATION;TRANSCRIPTION FACTOR BINDING SITE;UTR3 / SNP
Source:    dbSNP; Celera; HGBASE / Population(Allele,Count):    Caucasian
(C,40|G,80) / SNP Type:    MISSENSE MUTATION;TRANSCRIPTION FACTOR BINDING
SITE;UTR3 /

Context (SEQ ID NO: 1598): /
TGAGTGACAGTGAGCACTGCCCACAGAGGGGGTGCCCTGCAGTCAGGAGGGGTGGGAAACGGGTGCAGATCGGCAGCCAACAG
AGGCCCACCCACATGAG
Y
ACAGGTGGAGTTGCTGGTGCGATGAAGAGTGTGCTGGTTGGGAGTGGAGGGCTGGGGGCCGGTAGCCGGTTTGGGAAACCCAA
GCTGAGCGAAAGGTGTG / Celera SNP ID: hCV95670 / Public SNP ID: rs4852975 / SNP
Chromosome Position: 73932465 / SNP in Genomic Sequence: SEQ ID NO: 376 / SNP
Position Genomic: 14829 / Related Interrogated SNP: hCV11541681 / SNP Source:
dbSNP; Celera; HapMap; HGBASE / Population(Allele,Count): Caucasian
(C,86|T,140) / SNP Type: INTERGENIC;UNKNOWN /
Context (SEQ ID NO: 1599): /
CTTGTTGAAAAGCCACCTAAAATTGTCATAAGTAAGAGAGGTGATCTAATGGCCCATGAAACTGCCAACTCCAGGAAGACAGA
CAGGGTGACCTGAAAGT
R
GGTTTGACCTGTTGGTTTTTCTGCCATTCTGTGGGTGGCATCAGCTTCCTGCCAAGACTGGCACTGCATAGGATGTCGGGTGT
TTGCCAGCCCAGTTGAA / Celera SNP ID: hCV95671 / Public SNP ID: rs11126414 /
SNP Chromosome Position: 73932044 / SNP in Genomic Sequence: SEQ ID NO: 376 /
SNP Position Genomic: 14408 / Related Interrogated SNP: hCV11541681 / SNP
Source: dbSNP; Celera; HapMap; ABI_Val / Population(Allele,Count): Caucasian
(G,86|A,140) / SNP Type: INTERGENIC;UNKNOWN /
Context (SEQ ID NO: 1600): /
AGGAACTGGAGCTGAGTGCAGAGGACACCATGGAGCAGTTTTGCCACAGAGTGAAACAGGGAGATGCCTGGCAGCTGCAGAGG
AGGGTGCAGTCAAAAAG
R
TTTGTTACAGAACTGCCGTTTGACTCAGCAATCCTATTACTAGGTATATATCCAGAGAAATAGAAATCGTTCTACCATAGAGA
TGCATGTGAATGTTCAT / Celera SNP ID: hCV112099 / Public SNP ID: rs12052539 /
SNP Chromosome Position: 73937153 / SNP in Genomic Sequence: SEQ ID NO: 376 /
SNP Position Genomic: 19517 / Related Interrogated SNP: hCV11541681 / SNP
Source: dbSNP; Celera; HapMap / Population(Allele,Count): Caucasian
(A,83|G,143) / SNP Type: INTERGENIC;UNKNOWN /
Context (SEQ ID NO: 1601): /
TCAGCCAGTCCTACCCAGAACACCCTATTTCACAGTGCACTTCACCCCTAGCCTACCTGGCCATTCCCTATCCCCATAGCTGG
CTCTAAGTTTTCTTATC
M
CCTCTAACCTGCTCTAAATGCTAAGGATATGCATCTGTTGTTGATTTGTATTTAGTAGGCTCTGCAAGCAGGGATCTTTGGCT
CTTCGGTTCACTCATGT / Celera SNP ID: hCV112100 / Public SNP ID: rs17350125 /
SNP Chromosome Position: 73936184 / SNP in Genomic Sequence: SEQ ID NO: 376 /
SNP Position Genomic: 18548 / Related Interrogated SNP: hCV11541681 / SNP
Source: dbSNP; Celera; HapMap / Population(Allele,Count): Caucasian
(A,38|C,82) / SNP Type: INTERGENIC;UNKNOWN /
Context (SEQ ID NO: 1602): /
GTGCAGAGGACACCATGGAGCAGTTTTGCCACAGAGTGAAACAGGGAGATGCCTGGCAGCTGCAGAGGAGGGTGCAGTCAAAA
AGATTTGTTACAGAACT
R
CCGTTTGACTCAGCAATCCTATTACTAGGTATATATCCAGAGAAATAGAAATCGTTCTACCATAGAGATGCATGTGAATGTTC
ATTGCAGCAGTATTCAC / Celera SNP ID: hCV133926 / Public SNP ID: rs12053242 /
SNP Chromosome Position: 73937168 / SNP in Genomic Sequence: SEQ ID NO: 376 /
SNP Position Genomic: 19532 / Related Interrogated SNP: hCV11541681 / SNP
Source: dbSNP; Celera; HapMap / Population(Allele,Count): Caucasian
(G,38|A,82) / SNP Type: INTERGENIC;UNKNOWN /
Context (SEQ ID NO: 1603): /
GAGAACTTATGAACACAAAGAAGGAAACAACAGACACTGGGGCCTTCCCCAGGGTGGAGAGTGGGAGGAGGGAGAGGAGCAGA
AAAATAATTTTGAGTAC
K
AGTCTTAGTACCTGGGTAATGAAATAATCTGTACCATAAACCCCCATGACATGAATTCACCAATATAACAAACCTGCACCTGT
ACCCCTGAACCTAAAAT / Celera SNP ID: hCV133927 / Public SNP ID: rs7599453 /
SNP Chromosome Position: 73937901 / SNP in Genomic Sequence: SEQ ID NO: 376 /
SNP Position Genomic: 20265 / Related Interrogated SNP: hCV11541681 / SNP
Source: dbSNP; Celera; HapMap / Population(Allele,Count): Caucasian
(T,36|G,80) / SNP Type: UTR3 /
Context (SEQ ID NO: 1604): /
GGGACTGACAGTGTGTTATGTGCTGGGCTTGGCAGAAGTTCTCTCCATTGCCTCTTCTGTTGTGTCTTGGTTTTTAACAATGA
ACTAGGAAAGGATATTA

Y
CAACTAAGAGTGAGGGTCAGAGAAGAGGCATTGTTCTTTGAGGGGGAGAGAAATGATAAGCCGTTGTCCAGTAGTTGCCTCTT
CTGTTCAGTAAGGACAG / Celera SNP ID: hCV133928 / Public SNP ID: rs4852977 /
SNP Chromosome Position: 73938295 / SNP in Genomic Sequence: SEQ ID NO: 376 /
SNP Position Genomic: 20659 / Related Interrogated SNP: hCV11541681 / SNP
Source: dbSNP; Celera; HapMap; HGBASE / Population(Allele,Count): Caucasian
(C,38|T,82) / SNP Type: UTR5 /
Context (SEQ ID NO: 1605): /
CAGCCGACCTGGGGCTCCACAGCTCTTGCTGCCGCTGGAAGTTGGGCCAAGGCTGGTGGCACACACAGGTGTGTTCCTGCTGT
TGGGATCACCAGACAAG

K
CTGCTTCGCCTTGGGCCGATCTTTTGCCCCGGACCACTCAGCCCTAGGTACTTTAGGCCAATCTGCTGATCAAGGCCATAAGT
GCCTCATAACCAATCAG / Celera SNP ID: hCV11541694 / Public SNP ID: rs12619258 /
SNP Chromosome Position: 73935915 / SNP in Genomic Sequence: SEQ ID NO: 376 /
SNP Position Genomic: 18279 / Related Interrogated SNP: hCV11541681 / SNP
Source: dbSNP; Celera; HapMap / Population(Allele,Count): Caucasian
(G,40|T,80) / SNP Type: INTERGENIC;UNKNOWN /
Context (SEQ ID NO: 1606): /
CTCAGCTTCTTGGTGCATTGAGAGAGTACCTACCTCAGGCAGGCCACATGCCCAACCCTTTGGTGCTTCTCCAGGGACCATAC
ATCGCAGAAATCCTGTG

W
CCAAGCACAAGTTTCAGGAAAGGCTCCCTCCAGCTTTATTGTAAGTTTTTAGGTTGCTGAGGTGGCATAAGTGCCAAGCATAA
AAAACAGCCCAGCCTCC / Celera SNP ID: hCV11941453 / Public SNP ID: rs2001436 /
SNP Chromosome Position: 73928965 / SNP in Genomic Sequence: SEQ ID NO: 376 /
SNP Position Genomic: 11329 / Related Interrogated SNP: hCV11541681 / SNP
Source: dbSNP; Celera; HGBASE / Population(Allele,Count): Caucasian
(A,40|T,76) / SNP Type: INTERGENIC;UNKNOWN /
Context (SEQ ID NO: 1607): /
TAAACTTTACTATTGTTGAACTGCCTATGTTGCCTGAACTTTGACTGGTTTAAGTTGGTTTCATTTATTTCATAAGAACTCTT
GTTAAGGGGTGCACCTT

K
GTATTTTGATATTATTCTCTTGATAGTCATAATAATAGTTCCCTGGTGTGCTGCATCCTCTCAAGTCATAAATGTTTTGTATG
CAGCCATACATTGAGAA / Celera SNP ID: hCV26996674 / Public SNP ID: rs13006448 /
SNP Chromosome Position: 73924424 / SNP in Genomic Sequence: SEQ ID NO: 376 /
SNP Position Genomic: 6788 / Related Interrogated SNP: hCV11541681 / SNP
Source: dbSNP; Celera; HapMap / Population(Allele,Count): Caucasian
(G,40|T,80) / SNP Type: INTRON /
Context (SEQ ID NO: 1608): /
ACAATTCCGCTAATGCCAATGCCTCTCCATGTTGCCAATGAGAGTCACCCCAAAATTCCAAGGGAAGGATAGAAAGCTATCCT
TGATATTCTGAACACCA

Y
TGCTACTTGTTTTCCTGCACTCACTAAAAGCAACACTTTAACTTTTCCTTTTTTTTTTTTTTTTTTCTGAGGCAGTCTCACTCTGT
TGCCTAGGTTGGAGTGC / Celera SNP ID: hCV26996679 / Public SNP ID: rs6732812 /
SNP Chromosome Position: 73922475 / SNP in Genomic Sequence: SEQ ID NO: 376 /
SNP Position Genomic: 4839 / Related Interrogated SNP: hCV11541681 / SNP
Source: dbSNP; Celera; HapMap; ABI_Val / Population(Allele,Count): Caucasian
(T,84|C,140) / SNP Type: INTRON /
Context (SEQ ID NO: 1609): /
ATGATTTTGGCATGATAGGGAAACTCTAAGGGTTTCTCACTTCTAGTGGGATCACCATCAAAAATGGACTCCAGTTAGATAAA
CTCCTAGTTTGCTGTTA

K
TGATCGAAGCTCATACCTGCAGAACTGAACCTGAATATCAAGGGAATGCTTAGCAGATATTTATGCTAAATCAGCTAGTACTG
AAACTGTTCAGATATGC / Celera SNP ID: hCV26996688 / Public SNP ID: rs13015885 /
SNP Chromosome Position: 73920482 / SNP in Genomic Sequence: SEQ ID NO: 376 /
SNP Position Genomic: 2846 / Related Interrogated SNP: hCV11541681 / SNP
Source: dbSNP; Celera; HapMap / Population(Allele,Count): Caucasian
(G,39|T,79) / SNP Type: INTRON /
Context (SEQ ID NO: 1610): /
CATGCCACTGTATTACTGCTGCCTGATGATGGTGAAGACCACAATTGCATAAGTGTAGCATCAGAAATAGTGGCCCCTCATGT
TAATTTACAAGTTAGTC

S
TTTGGACAATCCTGAGTTAATACTTTTGTTGATGGGTCCTATGCCAAAAGCTCAGAAGGAAAATATCAGCTAGGATATGCTGT
TACCAAAATGAGTTAAT / Celera SNP ID: hCV26996689 / Public SNP ID: rs13014700 /

SNP Chromosome Position: 73920121 / SNP in Genomic Sequence: SEQ ID NO: 376 / SNP Position Genomic: 2485 / Related Interrogated SNP: hCV11541681 / SNP Source: dbSNP; Celera; HapMap / Population(Allele,Count): Caucasian (C,39|G,79) / SNP Type: INTRON /
Context (SEQ ID NO: 1611): /
CCTAAAAAGTTAAAAACTATTCAAAATTTTCCTTGGCCTGCAACCAAAAGATAATTAAATAGTTTTCTTGGACTTGCAGGATA
TGTTTTTAGACTTGCAG
R
ATATAAAATTCCTGGGTTCTGAATTTTTCCTTAATAGCCTCACCATTTCATGAGCTCCCTAAAAAATGCTGTACCAGAGCCTT
TATCTTGGGATGATAGT / Celera SNP ID: hCV26996690 / Public SNP ID: rs2421575 /
SNP Chromosome Position: 73919522 / SNP in Genomic Sequence: SEQ ID NO: 376 /
SNP Position Genomic: 1886 / Related Interrogated SNP: hCV11541681 / SNP
Source: dbSNP; Celera; HapMap; ABI_Val / Population(Allele,Count): Caucasian (G,40|A,80) / SNP Type: INTRON /
Context (SEQ ID NO: 1612): /
AGCCTCACCATTTCATGAGCTCCCTAAAAAATGCTGTACCAGAGCCTTTATCTTGGGATGATAGTTACGAGCAGGCTTTTAGC
TAAATAAAATTGACCTT
R
TAACAGCCTCCAGCTTTAGGACTTCCAAATTGCACTAAACTGTTTACCTAATTGGCTCATGAAAGTAATAATCAGGCATTTAG
GAGTTCTTACCCAGGAA / Celera SNP ID: hCV29307907 / Public SNP ID: rs4852316 /
SNP Chromosome Position: 73919658 / SNP in Genomic Sequence: SEQ ID NO: 376 /
SNP Position Genomic: 2022 / Related Interrogated SNP: hCV11541681 / SNP
Source: dbSNP; HapMap / Population(Allele,Count): Caucasian (G,83|A,143) /
SNP Type: INTRON /
Context (SEQ ID NO: 1613): /
AGACACGTTTTATTGGGAAGCTAACAAATCTTAAGCTTCAAAGTTCCTCTTTCCCCTGCACCAGCACCTTACAAAAAAAAAAA
AACCTACTGTGGTTTTA
Y
AATTTATATTTTGCAAACAGGCTCTGAAATGTATTTACCTCAGGCTCCAAAAAACCTGGATCTTCCTTGAGGGCAGTGCTGCC
TACTGGATGAAATACAG / Celera SNP ID: hCV31840146 / Public SNP ID: rs11126415 /
SNP Chromosome Position: 73936500 / SNP in Genomic Sequence: SEQ ID NO: 376 /
SNP Position Genomic: 18864 / Related Interrogated SNP: hCV11541681 / SNP
Source: dbSNP; HapMap / Population(Allele,Count): Caucasian (C,38|T,82) / SNP
Type: INTERGENIC;UNKNOWN /
Context (SEQ ID NO: 1614): /
TCTACTGTGACTTCTGCATTTTTGTAGCTTTCCTGATAAAAGCCTGGGCTTTCCTTGTCAGACCTGAAAGATTCATTTATTCA
TTCAACAAACAGTTCCC
R
AGGACCTGAGGTGTGCCGGGCCTGGACTTGGCATGAAGGCACCAGATGTTGGAAGCGAGGCTGCCGCCCAGGAGGACACACCT
GATGGGGCTCTGGGAAC / Celera SNP ID: hCV31840149 / Public SNP ID: rs12233112 /
SNP Chromosome Position: 73931764 / SNP in Genomic Sequence: SEQ ID NO: 376 /
SNP Position Genomic: 14128 / Related Interrogated SNP: hCV11541681 / SNP
Source: dbSNP; HapMap / Population(Allele,Count): Caucasian (G,40|A,80) / SNP
Type: INTERGENIC;UNKNOWN /
Context (SEQ ID NO: 1615): /
ATAAACAGTGCAATGCACCTCATTTGGAAAAACAAAACTGGTATCTAAAAGGATATAAATTCAATGTTAAGTGCAGACTCACA
GAGGGCCTAGATGGCTG
Y
CTGGTCTTTCCTGAGTCTTTGAAGCTTCTATTTTCAAAAGCTCTGTACTCCACAACTCATCATGGAACAGAAAAATACGTCCA
AATTGTGAAAAAATACT / Celera SNP ID: hCV31840166 / Public SNP ID: rs4513320 /
SNP Chromosome Position: 73920741 / SNP in Genomic Sequence: SEQ ID NO: 376 /
SNP Position Genomic: 3105 / Related Interrogated SNP: hCV11541681 / SNP
Source: dbSNP; HapMap / Population(Allele,Count): Caucasian (T,39|C,79) / SNP
Type: TRANSCRIPTION FACTOR BINDING SITE;INTRON /
Context (SEQ ID NO: 1616): /
AGATCCCTCAGAGTTAAAAAGGCTATTTCTGTGGTTGGGGTTTCAAATCAAATTCAAGAGGTTCCCATATCTGAACCCGTCCA
ATTGACTTTGGGGCACT
Y
TTCAGAAAATCACACTTTTTTACTGTATGATAGTGCTCCAGTAAATTTGCTAGAGGGAGATTTACTTTCAAAGCTGAAAGGGC
ATAGAAGACTACCTATT / Celera SNP ID: hDV69785784 / Public SNP ID: rs13000788 /
SNP Chromosome Position: 73918312 / SNP in Genomic Sequence: SEQ ID NO: 376 /
SNP Position Genomic: 676 / Related Interrogated SNP: hCV11541681 / SNP
Source: dbSNP; HapMap / Population(Allele,Count): Caucasian (C,39|T,79) / SNP

Type:    INTRON /
Context                (SEQ ID NO: 1617): /
CTGCCCAACTTGCAAAATATTTATTTTTCTTGCTCAATTACTCTGTGAAATTTAATTTGTCTAATGGATTTCTTTTAACAGAA
CTTATAATTAAAATGGA
R
AGCGGGTGTAAATATTTATAAAACTTGCAGCCTGGCCATGTGGTAGACAAGGAGGAATCCAAGCAGCCTGCTGAGCAACTACT
TGCTGGAGACATTAGCA / Celera SNP ID:  hCV31840159 / Public SNP ID:   rs13013228 /
SNP Chromosome Position:   73925216 / SNP in Genomic Sequence:   SEQ ID NO: 376 /
SNP Position Genomic:  7580 / Related Interrogated SNP:   hCV11541681 /  SNP
Source:   dbSNP; HapMap / Population(Allele,Count):   Caucasian (G,40|A,80) /  SNP
Type:    INTRON /
Context                (SEQ ID NO: 1618): /
CTCCCAAAGTTCTGGGATTACAGGCGTGAGCCACCATGCCTGGCCTTAACTGTTTCAATTAATAACCTGATCAATACCAAAAT
ATAGAAAACCAGTCCAA
R
TGATGCCTGCAAAAGATATATTGTGCTTTCAGGCATCATGCACTCAAGTTACGGGAATTACCTATGTGAGTACAAGTAATTGC
TTGTATAATATCACCAG / Celera SNP ID:  hDV70942181 / Public SNP ID:   rs17350056 /
SNP Chromosome Position:   73922877 / SNP in Genomic Sequence:   SEQ ID NO: 376 /
SNP Position Genomic:  5241 / Related Interrogated SNP:   hCV11541681 / SNP
Source:   dbSNP; HapMap / Population(Allele,Count):   Caucasian (G,40|A,80) / SNP
Type:    INTRON /
Context                (SEQ ID NO: 1619): /
CTGTTTCAATTAATAACCTGATCAATACCAAAATATAGAAAACCAGTCCAAGTGATGCCTGCAAAAGATATATTGTGCTTTCA
GGCATCATGCACTCAAG
W
TACGGGAATTACCTATGTGAGTACAAGTAATTGCTTGTATAATATCACCAGATTAAATTCAACAGGGTCTCTTTTTACTAAAT
GTGTTATACACCCTTAC / Celera SNP ID:  hDV70953030 / Public SNP ID:   rs17434634 /
SNP Chromosome Position:   73922926 / SNP in Genomic Sequence:   SEQ ID NO: 376 /
SNP Position Genomic:  5290 / Related Interrogated SNP:   hCV11541681 / SNP
Source:   dbSNP; HapMap / Population(Allele,Count):   Caucasian (T,40|A,80) /  SNP
Type:    INTRON /
Context                (SEQ ID NO: 1620): /
CAGGGTCTCTTTTTACTAAATGTGTTATACACCCTTACAACATACTACAGGGCGAGCACAAAAAGGTCAATTTCCCAGTGGAT
TTTGTTCAGGAGCTGTG
Y
AGGTTTATTAATGAACAAATCTAACTGACCCCTGCTCAAATGCAACTATGTGGCCCTGTTTTTCAACTCCCAAGGGCCTATAC
TGGGTCTGTGGATAATC / Celera SNP ID:  hDV70953035 / Public SNP ID:   rs17434655 /
SNP Chromosome Position:   73923089 / SNP in Genomic Sequence:   SEQ ID NO: 376 /
SNP Position Genomic:  5453 / Related Interrogated SNP:   hCV11541681 / SNP
Source:   dbSNP; HapMap / Population(Allele,Count):   Caucasian (T,86|C,140) /
SNP Type:    INTRON /
Context                (SEQ ID NO: 1621): /
GTGTGTGTGTGTGTGTGTGTGTGTGCGCGCTTCACTCTGGCTGCAGGTGGACAATGGATCTGAGGAGGTGGGAATGGAAGC
AGCTACCCCTTAAGGCT
M
GTGCCGCATCCAGGCAACAGCAGGGCCCCCAGTGCAGGGATGCAGGTGCTCACTGTACAAGAGCAGCTGGCCCAGAGGGGCTG
GTAGGAGCTAAAATCGG / Celera SNP ID:  hCV31840152 / Public SNP ID:   rs12998980 /
SNP Chromosome Position:   73930726 / SNP in Genomic Sequence:   SEQ ID NO: 376 /
SNP Position Genomic:   13090 / Related Interrogated SNP:   hCV11541681 / SNP
Source:   dbSNP / Population(Allele,Count):   Caucasian (A,40|C,80) / SNP Type:
 INTERGENIC;UNKNOWN /
Context                (SEQ ID NO: 1622): /
TTGTTGATTTGTATTTAGTAGGCTCTGCAAGCAGGGATCTTTGGCTCTTCGGTTCACTCATGTATGTTAAGTGCCCAGAACAA
GCCTGACAGATATTAGG
Y
GCTCGGTAAGTGCGTGTTGAGTGAGTAAACAACAGACAGCCAGTTTGGGTATTCCCAGTAGGGCAGGAACAGAGAAGAGACAC
GTTTTATTGGGAAGCTA / Celera SNP ID:  hDV77051912 / Public SNP ID:   rs4852976 /
SNP Chromosome Position:   73936322 / SNP in Genomic Sequence:   SEQ ID NO: 376 /
SNP Position Genomic:   18686 / Related Interrogated SNP:   hCV11541681 / SNP
Source:   CDX; dbSNP / Population(Allele,Count):   Caucasian (T,83|C,143) / SNP
Type:    INTERGENIC;UNKNOWN //

Gene Number:   40 / Gene Symbol:   DCHS2 - 54798 / Gene Name:   dachsous 2

(Drosophila) / Chromosome: 4 / OMIM NUMBER: / OMIM Information: // Genomic Sequence (SEQ ID NO: 377): // / SNP Information /
Context (SEQ ID NO: 1623): /
TGTAAAAATCAATAATTATAAAAGTATTTAGGTAAAGTAATTATGAAAACATTGTATGCATAGAAGTGCATGAGTGAATAATT
CTAGAGTTTTATGTTTG
W
TATTCCTAAGCAATGCCAACATTTTCTTATTGATGACTGACTTGAGAGAAACAGATTTTTCAAACATTTGGATGATTCTGAATT
ATCTGTTATCACTTAAG / Celera SNP ID: hCV36809 / Public SNP ID: rs10517590 /
SNP Chromosome Position: 155197683 / SNP in Genomic Sequence: SEQ ID NO: 377 /
SNP Position Genomic: 52156 / Related Interrogated SNP: hCV11503414 /
Related Interrogated SNP: hCV15860433 / SNP Source: dbSNP; Celera; HapMap /
Population(Allele,Count): Caucasian (T,48|A,72) / SNP Type: INTRON /
Context (SEQ ID NO: 1624): /
CATGTATAAGACAGTGGTAACAAGTCACAAGAGGAAAGTAAGAGGAAACATCTAAAAGGAACAGGAGCTGACATGGTGTCAGA
GATGAGTACAGAGCAGG
K
CTCATCATCCCATATGCATTTGGGTAAAAGCAAGAAGCAGGAACAAATGTAGAGGTCAAATGGTGGAGGAGGCCAGAAAATGT
CTCAGACAGTCACTGTC / Celera SNP ID: hCV37878 / Public SNP ID: rs4235241 / SNP
Chromosome Position: 155195184 / SNP in Genomic Sequence: SEQ ID NO: 377 /
SNP Position Genomic: 49657 / Related Interrogated SNP: hCV11503469 / SNP
Source: dbSNP; Celera; HapMap; HGBASE / Population(Allele,Count): Caucasian
(G,93|T,131) / SNP Type: INTRON /
Context (SEQ ID NO: 1625): /
AGTCATAGAAGAGTACGTAGCATGATTCTATTATCATACTTAAGTCAAGGTAAAAGTAAATGACATATTGTTTAGAAATTCAA
ATGCAGTGGAAAAATTA
W
AACCAAAAATGTCAGCGATCAGTTGGGTGCAGTGGCTCATGCCTATAATCCCAGAACTTCAGGAGGCCAAGGCAAGGAGATTG
CTCGAGTTCAGGAGTTT / Celera SNP ID: hCV99436 / Public SNP ID: rs10015747 /
SNP Chromosome Position: 155186628 / SNP in Genomic Sequence: SEQ ID NO: 377 /
SNP Position Genomic: 41101 / Related Interrogated SNP: hCV11503414 / SNP
Source: dbSNP; Celera; HapMap / Population(Allele,Count): Caucasian
(T,80|A,36) / SNP Type: INTRON /
Context (SEQ ID NO: 1626): /
GGAGAGGCCACCAGAGAGCCCAGTGACTAAGAGGCTTTTGCAAATCTTGAGGGGCTAGATCGCTGGAATTTCCAGAAATTCTA
GTCAGCACAATTTAATG
Y
TGTCTGCAGCAATACTTGGCAACCTGGAAAGGTTTGTTGCAATTGTTGGAGGTGCAGTCCTACATGTAAGCCAAGCTGAGCTC
ACCATAATGGATCTACC / Celera SNP ID: hCV176753 / Public SNP ID: rs2404478 /
SNP Chromosome Position: 155408038 / SNP in Genomic Sequence: SEQ ID NO: 377 /
SNP Position Genomic: 262511 / Related Interrogated SNP: hCV11503469 /
Related Interrogated SNP: hCV11503414 / SNP Source: dbSNP; Celera; HapMap;
ABI_Val; HGBASE / Population(Allele,Count): Caucasian (C,54|T,172) / SNP Type:
INTRON /
Context (SEQ ID NO: 1627): /
CTTTCCTCTGTTGACTGCCAATTCCTTATTTCCCATAACTCTTTGTATCTCAGTGCAAAAAAAAAAAAAAAAAAAAAAGGAGAG
AAAGAAAAATGGAGGAA
W
GATATTGCAGAAGCTTCTAGCCTCAAAGGATGCATTCAATTAATAAACAGTAATCTTTCAGGAAAGCAACCCTAAATCCCCTG
GTCAACAATGTCCATTC / Celera SNP ID: hCV229029 / Public SNP ID: rs13103792 /
SNP Chromosome Position: 155420340 / SNP in Genomic Sequence: SEQ ID NO: 377 /
SNP Position Genomic: 274813 / Related Interrogated SNP: hCV11503469 /
Related Interrogated SNP: hCV11503414 / SNP Source: dbSNP; Celera /
Population(Allele,Count): Caucasian (A,174|T,48) / SNP Type:
INTERGENIC;UNKNOWN /
Context (SEQ ID NO: 1628): /
CTTCATTCCTTTTTATGACCCCCAAATTATTTTTCATAGTTGAATAATTGGTTGTCTCTCCTGCTAAAATGTAAGTCCTACAA
AACCAGAAACCATGTCT
Y
ATTGCTTCCCATATCACACAGAGAGGTGATCAAAAATGATTTGTTGAACTACTAACAAATCAAAAAAGTGAACTTGGCTCAAA
GCCTATGCAATACATCA / Celera SNP ID: hCV265748 / Public SNP ID: rs12500118 /
SNP Chromosome Position: 155344634 / SNP in Genomic Sequence: SEQ ID NO: 377 /
SNP Position Genomic: 199107 / Related Interrogated SNP: hCV11503414 /
Related Interrogated SNP: hCV11503469 / Related Interrogated SNP: hCV2892877 /

SNP Source: dbSNP; Celera; HapMap / Population(Allele,Count): Caucasian (C,79|T,41) / SNP Type: INTRON /
Context (SEQ ID NO: 1629): /
GAGCCATGATTGCACCACTGTACTTCAGCCTGGGTGACAGAGCAAGACCCTGTCTCAAAAAAAAAAAAAAATGTCAAACAGCTG
TGACTAATATTAAATAA
S
ATCTGTCAGCTAATTCTGATATTACCTTGCATGAGGTTTTTAGACTTCAATGGCTTATACACAATTTTAACCAATATTTACTT
AGAGTACAAAGTTTTAA / Celera SNP ID: hCV400532 / Public SNP ID: rs11099956 /
SNP Chromosome Position: 155391684 / SNP in Genomic Sequence: SEQ ID NO: 377 /
SNP Position Genomic: 246157 / Related Interrogated SNP: hCV11503414 /
Related Interrogated SNP: hCV11503469 / SNP Source: dbSNP; Celera; HapMap /
Population(Allele,Count): Caucasian (G,78|C,42) / SNP Type: INTRON /
Context (SEQ ID NO: 1630): /
GCATAGCCCTCATCCAAGTAGACATTCCAAGCTGAACATACACATAAAACCTTTTCTTTTTCTTGTGACAGGAAGTCCTACTT
TTTTCTAAATCCCAGGA
Y
TGCTCTGCCTTTCCACATACTTGTTTTAAAGTAGTCACACATTTCACCAGAAACAAAGTCCTGGTTGCTTTCTGTCACAGCTG
AGAGCACTGACTCTGAA / Celera SNP ID: hCV426162 / Public SNP ID: rs10857275 /
SNP Chromosome Position: 155328698 / SNP in Genomic Sequence: SEQ ID NO: 377 /
SNP Position Genomic: 183171 / Related Interrogated SNP: hCV11503414 / SNP
Source: dbSNP; Celera; HapMap / Population(Allele,Count): Caucasian
(C,80|T,36) / SNP Type: INTRON /
Context (SEQ ID NO: 1631): /
GCCACAATAGGCTCATAAGCTCCTGAGCACAGGACTCTGGCTCCACAGCCACTCTGAGCATTTGTGTCCTGGAACCCAGCACC
AATGCCGTTGCTTAAGG
S
TCATGTCAGACATGACACCAAGAGGGATCCCCTCAACTAAGATTACCCATTATAGGGAAAACAAGAAGAAAAGGTCCCAAAAG
CCCTCACCTCTGAGGAC / Celera SNP ID: hCV426165 / Public SNP ID: rs990185 / SNP
Chromosome Position: 155330882 / SNP in Genomic Sequence: SEQ ID NO: 377 /
SNP Position Genomic: 185355 / Related Interrogated SNP: hCV11503414 / SNP
Source: dbSNP; Celera; HapMap; HGBASE / Population(Allele,Count): Caucasian
(G,81|C,39) / SNP Type: INTRON /
Context (SEQ ID NO: 1632): /
TGTTGTTAGTGCCAGTATTATTTGAGTCTGAGCTTGATGAGAAACCTGAAATAGGCTCAGTGACAGCAAAAAGTGAAGGTCAA
GGCAGAGTGGAAAGTAG
K
ATAGCTAAGTGTTGAAGGCATACAATATAATCATATATAACATCGTGATAAACATCCTTATTCACAAACTTTGTAAACCTCTC
TGCTTCTTTTGCTAAAT / Celera SNP ID: hCV426167 / Public SNP ID: rs1388087 /
SNP Chromosome Position: 155357085 / SNP in Genomic Sequence: SEQ ID NO: 377 /
SNP Position Genomic: 211558 / Related Interrogated SNP: hCV11503469 /
Related Interrogated SNP: hCV11503414 / SNP Source: dbSNP; Celera; HapMap;
ABI_val; HGBASE / Population(Allele,Count): Caucasian (G,88|T,138) / SNP Type:
INTRON /
Context (SEQ ID NO: 1633): /
ATATATAACATCGTGATAAACATCCTTATTCACAAACTTTGTAAACCTCTCTGCTTCTTTTGCTAAATTCCCCAAAGTAGAAA
CAATGCTGGGTCAAAGC
R
TAACCTTCTTTACTGCTCTCTGTAGGGAAATCCTGTCAGGGGGTTCCCCAAAGACTGAACTAACCTGCCCTCTACCTTCATGT
TATTTTTCCCACAGTAA / Celera SNP ID: hCV426168 / Public SNP ID: rs1388088 /
SNP Chromosome Position: 155357218 / SNP in Genomic Sequence: SEQ ID NO: 377 /
SNP Position Genomic: 211691 / Related Interrogated SNP: hCV11503414 /
Related Interrogated SNP: hCV11503469 / SNP Source: dbSNP; Celera; HapMap;
HGBASE / Population(Allele,Count): Caucasian (A,47|G,73) / SNP Type: INTRON
/
Context (SEQ ID NO: 1634): /
CTCTACCTTCATGTTATTTTTCCCACAGTAATGCTCAATTGATAAATCTCACTCCTAGTTTTAATGTTTTTATCAATAATAAG
GCTGAATTTTTAAAATT
K
TATTGGTCCTTTGTGTGTCTTTCAACAAATTATTATATCACTTACACTGCCCATTTTTCTTAAAAATATTAAGTAGCTCTTTA
TAGCTTATTATCTATAC / Celera SNP ID: hCV426169 / Public SNP ID: rs1388066 /
SNP Chromosome Position: 155357388 / SNP in Genomic Sequence: SEQ ID NO: 377 /
SNP Position Genomic: 211861 / Related Interrogated SNP: hCV11503414 /
Related Interrogated SNP: hCV11503469 / SNP Source: dbSNP; Celera; HapMap;

HGBASE / Population(Allele,Count): Caucasian (G,45|T,67) / SNP Type: INTRON /
Context (SEQ ID NO: 1635): /
AATTTTTAAAATTGTATTGGTCCTTTGTGTGTCTTTCAACAAATTATTATATCACTTACACTGCCCATTTTTCTTAAAAATAT
TAAGTAGCTCTTTATAG
Y
TTATTATCTATACTATATGTTGTAAGTACTTTTTTTCCATCATGATTGCTTGATTTCTAATTTGGGATGCAGTGTTTCTGATTA
ATTAAATGTTAGATTTG / Celera SNP ID: hCV426170 / Public SNP ID: rs1388067 /
SNP Chromosome Position: 155357475 / SNP in Genomic Sequence: SEQ ID NO: 377 /
SNP Position Genomic: 211948 / Related Interrogated SNP: hCV11503414 /
Related Interrogated SNP: hCV11503469 / SNP Source: dbSNP; Celera; HapMap;
HGBASE / Population(Allele,Count): Caucasian (C,47|T,73) / SNP Type: INTRON /
Context (SEQ ID NO: 1636): /
CCAAGTGACTCCTTTGCACAATAAAATGAGAACCACTGCTTCCATCAATAAACGTCCAAAACTTGGCCATCTCACTCCTACCA
CTAACCACTCTCATGAT
R
AAAAAAGGATGGGAAACATAAATTCAGTGACAGGGAAGGGAAGGCAGCGACTACTGAAAGCCTAAAACAAAACAAGCCCAATC
TAGGCTTTCTTTGGGTA / Celera SNP ID: hCV426172 / Public SNP ID: rs7670027 /
SNP Chromosome Position: 155358502 / SNP in Genomic Sequence: SEQ ID NO: 377 /
SNP Position Genomic: 212975 / Related Interrogated SNP: hCV11503414 /
Related Interrogated SNP: hCV11503469 / SNP Source: dbSNP; Celera; HapMap /
Population(Allele,Count): Caucasian (G,54|A,64) / SNP Type: INTRON /
Context (SEQ ID NO: 1637): /
AGGCTTTCTTTGGGTAGAGAGAACTCCCTAGATTACTCCAAAATCAGGTAATTCAACAAAACAAAGACCTTTGCTTATTGGGT
TTATGTTTTTAATCAAG
M
AATATAAATACTATATGTATTTTTATAGGTTTCTTTGGAGGGGAAATCTTTTAGAATCTATACAACCAATTTGTGCTATGGCCC
CTGACATTTTTTCCATT / Celera SNP ID: hCV426173 / Public SNP ID: rs12504201 /
SNP Chromosome Position: 155358687 / SNP in Genomic Sequence: SEQ ID NO: 377 /
SNP Position Genomic: 213160 / Related Interrogated SNP: hCV11503414 /
Related Interrogated SNP: hCV11503469 / Related Interrogated SNP: hCV2892877 /
Related Interrogated SNP: hCV15860433 / SNP Source: dbSNP; Celera; HapMap /
Population(Allele,Count): Caucasian (C,68|A,42) / SNP Type: INTRON /
Context (SEQ ID NO: 1638): /
ATTTTTTGTTCTTGCAGAACTTTAGGATAATCTTGCCAAGTTTAATTTCCCATGGGTTTTGACTAGATTTGCGCTAACATCGG
TAAACTTATATTGAATC
R
TTCAATTCACGAACATGTTACAACTCTCCATTTATTCAGGTCTTCTTTTATGTACCTCCATAAAATTTAATAGTTCTCTTTCC
AAAAGTCCTGCACATTT / Celera SNP ID: hCV426175 / Public SNP ID: rs9884952 /
SNP Chromosome Position: 155359761 / SNP in Genomic Sequence: SEQ ID NO: 377 /
SNP Position Genomic: 214234 / Related Interrogated SNP: hCV11503414 /
Related Interrogated SNP: hCV11503469 / Related Interrogated SNP: hCV2892877 /
SNP Source: dbSNP; Celera; HapMap / Population(Allele,Count): Caucasian
(A,57|G,63) / SNP Type: INTRON /
Context (SEQ ID NO: 1639): /
TATTCAGGTCTTCTTTTATGTACCTCCATAAAATTTAATAGTTCTCTTTCCAAAAGTCCTGCACATTTCCTAGTCGCTTTATT
CCTAAGTTGTCTATATT
K
CTGTTGTTATAGCAGATGTAATCATTTTTAAATTTTTTTTCCTTTTACTTAATTATGCAGCAATACATGAATACAAAGTCATTA
CAAAACATTACAGAAGA / Celera SNP ID: hCV426176 / Public SNP ID: rs9884775 /
SNP Chromosome Position: 155359894 / SNP in Genomic Sequence: SEQ ID NO: 377 /
SNP Position Genomic: 214367 / Related Interrogated SNP: hCV11503414 /
Related Interrogated SNP: hCV11503469 / Related Interrogated SNP: hCV2892877 /
SNP Source: dbSNP; Celera; HapMap / Population(Allele,Count): Caucasian
(T,57|G,63) / SNP Type: INTRON /
Context (SEQ ID NO: 1640): /
TTATAGCAGATGTAATCATTTTTAAATTTTTTTTCCTTTTACTTAATTATGCAGCAATACATGAATACAAAGTCATTACAAAAC
ATTACAGAAGAGTAAAA
M
CAAACACTCTGGTCATTAATCCTACAAACCCTCACTCTTCCCACCAAAGGGGACATCTATAAAGAGTTGGGCTCATGTCCATC
AAGACAATTTTCCATGC / Celera SNP ID: hCV426178 / Public SNP ID: rs9884570 /
SNP Chromosome Position: 155360001 / SNP in Genomic Sequence: SEQ ID NO: 377 /

SNP Position Genomic: 214474 / Related Interrogated SNP: hCV11503414 /
Related Interrogated SNP: hCV11503469 / Related Interrogated SNP: hCV2892877 /
SNP Source: dbSNP; Celera; HapMap / Population(Allele,Count): Caucasian
(C,56|A,62) / SNP Type: INTRON /
Context (SEQ ID NO: 1641): /
GTGCATAGATATGGCTATACCTGTAGTTGCATATTATGCTATTATAAGAATTTGACAATACATTTAATACATTTCCCTACTAA
TGGACATTTAAGTCACC
W
GAGGCTTTTCACACTGAAAGCACTACTTCACCAAACATACATATACATAGATTTATATACATATTTATGCCAGTATTTTTTGTA
GGTGAGATTTATAAAGA / Celera SNP ID: hCV426181 / Public SNP ID: rs11099955 /
SNP Chromosome Position: 155360315 / SNP in Genomic Sequence: SEQ ID NO: 377 /
SNP Position Genomic: 214788 / Related Interrogated SNP: hCV11503414 /
Related Interrogated SNP: hCV11503469 / Related Interrogated SNP: hCV2892877 /
SNP Source: dbSNP; Celera; HapMap / Population(Allele,Count): Caucasian
(A,57|T,63) / SNP Type: INTRON /
Context (SEQ ID NO: 1642): /
CATATGAGCATTTTAAGAAATTGAAAGCTACTATCAAATTGCCCTGAAAGAAGGATTTACCACCTGTGCCAACATTCTGAAAA
AGTCCATTTTCCCATAC
Y
GTTTATATTTTTACATTTTCACTCTTAATTTTTGGCAATTTGCCAAAATGTTATGAAATACTTGTTTTTTAGTTAATATTACAC
TGAAGAACTGAGGAAGA / Celera SNP ID: hCV426182 / Public SNP ID: rs10014536 /
SNP Chromosome Position: 155360533 / SNP in Genomic Sequence: SEQ ID NO: 377 /
SNP Position Genomic: 215006 / Related Interrogated SNP: hCV11503414 /
Related Interrogated SNP: hCV11503469 / Related Interrogated SNP: hCV2892877 /
SNP Source: dbSNP; Celera; HapMap / Population(Allele,Count): Caucasian
(C,53|T,59) / SNP Type: INTRON /
Context (SEQ ID NO: 1643): /
CACCTGTGCCAACATTCTGAAAAAGTCCATTTTCCCATACCGTTTATATTTTTACATTTTCACTCTTAATTTTTGGCAATTTGC
CAAAATGTTATGAAATA
M
TTGTTTTTTAGTTAATATTACACTGAAGAACTGAGGAAGATATTTATATTTGCATTGGCTATATCAATATTTTCTCCTACAGT
CTGTCTTTTCCTAGCTC / Celera SNP ID: hCV426183 / Public SNP ID: rs10014635 /
SNP Chromosome Position: 155360593 / SNP in Genomic Sequence: SEQ ID NO: 377 /
SNP Position Genomic: 215066 / Related Interrogated SNP: hCV11503414 /
Related Interrogated SNP: hCV11503469 / Related Interrogated SNP: hCV2892877 /
SNP Source: dbSNP; Celera; HapMap / Population(Allele,Count): Caucasian
(C,55|A,59) / SNP Type: INTRON /
Context (SEQ ID NO: 1644): /
TTGTGCTGTCCCTTGTCAACAGAATTTTTTTCATTTTATATAGTTAAATCTGAGCTTTACAAAATATGTGCTTTATTAACAAAA
TGTTAAGTAACAAAATC
Y
AGAGGTGTATCTAGTAACTATTATAATTTCAAAGTAGTTATGACCACAAACAATATTTCAAGATATCTGCAACAACCATAATG
CTCTACGAAAATAGCTA / Celera SNP ID: hCV426184 / Public SNP ID: rs1032336 /
SNP Chromosome Position: 155360919 / SNP in Genomic Sequence: SEQ ID NO: 377 /
SNP Position Genomic: 215392 / Related Interrogated SNP: hCV11503414 /
Related Interrogated SNP: hCV11503469 / Related Interrogated SNP: hCV2892877 /
SNP Source: dbSNP; Celera; HapMap; HGBASE / Population(Allele,Count):
Caucasian (C,57|T,63) / SNP Type: INTRON /
Context (SEQ ID NO: 1645): /
CCAATCTAACAGCTCTAAGAGAAGAGCTGTTCTGTACCACACTTCTCTTTTCCGTATGTCACTTTCCTTTTTCCATCCATGAC
TATTGCCCGACCACGCA
R
CAACCCCAGAGCCACCCTGAACCTATTCTGGTTCTGAGGGCTGCCCAATTTGCGAATCATTCTTTGCTCAATTAACTCTGTTA
AATTTAATTTGTCCAAA / Celera SNP ID: hCV437164 / Public SNP ID: rs7685964 /
SNP Chromosome Position: 155348639 / SNP in Genomic Sequence: SEQ ID NO: 377 /
SNP Position Genomic: 203112 / Related Interrogated SNP: hCV11503414 /
Related Interrogated SNP: hCV11503469 / SNP Source: dbSNP; Celera; HapMap /
Population(Allele,Count): Caucasian (G,45|A,69) / SNP Type: INTRON /
Context (SEQ ID NO: 1646): /
GTGGGGCAGGGGGAGATATAGTTGGAGAGATCTACTGTTGGTAATATGTATACATGTATGGAAGAGGAAGGGTTAGTTTGGTG
CATAGAAGAATTACAAC
Y
GAACAGAGAAGGAACTAAAGCAAGGAGGGGGGTGATATACAAGAAGAGAAAAGAAAGAGCAAGTCATCTAGAAAAAAAATCTAG

ATTAAAAAAATAAATGCA / Celera SNP ID: hCV470979 / Public SNP ID: rs1490672 / SNP Chromosome Position: 155404360 / SNP in Genomic Sequence: SEQ ID NO: 377 / SNP Position Genomic: 258833 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera; HGBASE / Population(Allele,Count): Caucasian (C,170|T,56) / SNP Type: TFBS SYNONYMOUS;INTRON / Context (SEQ ID NO: 1647): / GTTCTGCATGAAACCCTTCCATGATCATAATATGCATCTGCTTCTTGAATGCTTATTAAAGAGTCATCACGCATGCGGTTCCA TCAGCCTGGAATGGCTC
M
CACAGCGATTCTTCCTCAGGGATTTTTCCAGATCACCATCCCTGTACCATCACCTCCCCAAAGTCTCTTACAAAGCCTCTGCA TTTCCCAGCAGCTGCAT / Celera SNP ID: hCV489970 / Public SNP ID: rs11734901 / SNP Chromosome Position: 155181767 / SNP in Genomic Sequence: SEQ ID NO: 377 / SNP Position Genomic: 36240 / Related Interrogated SNP: hCV11503469 / SNP Source: dbSNP; Celera; HapMap; ABI_Val / Population(Allele,Count): Caucasian (A,96|C,130) / SNP Type: INTRON / Context (SEQ ID NO: 1648): / CACACTGCCCCAAAACACTGGAGGTGGGAGTGGGAAAGGAGGCTATGAGAGCATAAAGAAAAGAAATATTCCCTCAACCAGCT TCCGATCTAGCTGTAGG
W
AAAAGCAACCCAACTAGAGAGTAACCAGCAACTTAACTGCAAGACATGAATTAATTCAAGATTTGTTCAGATAGAGCAGGGGC AAAGAGATAACTTTAAG / Celera SNP ID: hCV501681 / Public SNP ID: rs4076040 / SNP Chromosome Position: 155192032 / SNP in Genomic Sequence: SEQ ID NO: 377 / SNP Position Genomic: 46505 / Related Interrogated SNP: hCV11503469 / SNP Source: dbSNP; Celera; HapMap; HGBASE / Population(Allele,Count): Caucasian (A,94|T,132) / SNP Type: INTRON / Context (SEQ ID NO: 1649): / TTGAGAATCAAGCATGTTATTTTATGTATAAAAATTCATACAATTGTTATTGCAGAGTAATATTCTATTAAATGGAAAACCAC AATTTCTTTTTCATTTA
M
CTAGGGATGGGCATTTGAATTATTTCCAGTTTTTGGCTATTATGAAAAAAACTGCTATGAACATTCATACACAAGTGTGTGTG TGGACCCATGTTTTCAC / Celera SNP ID: hCV501682 / Public SNP ID: rs4403033 / SNP Chromosome Position: 155193315 / SNP in Genomic Sequence: SEQ ID NO: 377 / SNP Position Genomic: 47788 / Related Interrogated SNP: hCV11503414 / SNP Source: dbSNP; Celera; HapMap; HGBASE / Population(Allele,Count): Caucasian (C,45|A,75) / SNP Type: ACCEPTOR SPLICE SITE;INTRON / Context (SEQ ID NO: 1650): / AATTTCTTTTTCATTTACCTAGGGATGGGCATTTGAATTATTTCCAGTTTTTGGCTATTATGAAAAAAACTGCTATGAACATT CATACACAAGTGTGTGT
R
TGGACCCATGTTTTCACTGATCAAGGCCTAAATACCTACGGTAAGTATACATTTAACCTTATAAGAAAATGCAAAACTATTTT TCCAAAGTAGTATCATT / Celera SNP ID: hCV501683 / Public SNP ID: rs4312742 / SNP Chromosome Position: 155193398 / SNP in Genomic Sequence: SEQ ID NO: 377 / SNP Position Genomic: 47871 / Related Interrogated SNP: hCV11503414 / SNP Source: dbSNP; Celera; HapMap / Population(Allele,Count): Caucasian (G,37|A,71) / SNP Type: INTRON;PSEUDOGENE / Context (SEQ ID NO: 1651): / CACAGGTAGTATCTTTATAGCAGTGTGAGAACGGACTGATAACATCCACATTGATTGGTATTATCAATCTTTTAAATTGTAGC CATCCTAGTGGATATGT
R
GTATCTTACAGTGGTTTTAATTTAATTTATCTGGTGTCTGGTGATATTGTGCATCTTTTCATGTGCTTATTGGCCACTTCCTC CTCTTTTTAATCCATCT / Celera SNP ID: hCV501686 / Public SNP ID: rs4327464 / SNP Chromosome Position: 155193969 / SNP in Genomic Sequence: SEQ ID NO: 377 / SNP Position Genomic: 48442 / Related Interrogated SNP: hCV11503414 / SNP Source: dbSNP; Celera; HapMap; HGBASE / Population(Allele,Count): Caucasian (A,44|G,74) / SNP Type: INTRON / Context (SEQ ID NO: 1652): / CAACTGGAAGAACGGGCCTGCGGGGTCCTTGGGCAGGTCGGACGGTTGCACCAGGGTGTAGCCCTGAGTGCTGAACAGTCCGG CGTCCGGATCGTGGGCA
M
CTGGCAGGCGGAAGGCGGTCCCTGGCGGGCTGAGCTCGGAGACGTCGAGTTGCAGGGAGTCGAGGGGAAAGCGGGGCGAGTGG TCATTCACGTCGTTGAC / Celera SNP ID: hCV1125378 / Public SNP ID: rs17373874 /

SNP Chromosome Position: 155411939 / SNP in Genomic Sequence: SEQ ID NO: 377 / SNP Position Genomic: 266412 / SNP Source: dbSNP; Celera; HapMap / Population(Allele,Count): Caucasian (A,63|C,43) / SNP Type: MISSENSE MUTATION;TRANSCRIPTION FACTOR BINDING SITE;UTR5 /

Context (SEQ ID NO: 1653): /
ATTACTCCATATGTCATTAGAGAAAATACAGGATGCCTGGAGTTATAATGTGCAAAAGGCATACACATATATCTCACCAGTAA
GTTGAGAGTTACTATGA
M
GTCTCTTTGAACTTTGCCAGCATTTTAGAAAAATGACTCAAACTTTTGCTGGCATGGGCAAAAATAAAGCAGAGTCACAGCAC
TGACAGTCCTTTGATAT / Celera SNP ID: hCV1190562 / Public SNP ID: rs1490684 /
SNP Chromosome Position: 155362154 / SNP in Genomic Sequence: SEQ ID NO: 377 / SNP Position Genomic: 216627 / Related Interrogated SNP: hCV11503469 / Related Interrogated SNP: hCV11503414 / SNP Source: dbSNP; Celera; HapMap; HGBASE / Population(Allele,Count): Caucasian (C,107|A,119) / SNP Type: INTRON /

Context (SEQ ID NO: 1654): /
AGCAGGAAGCTACACAGGTCTCCGCATAAGGTTATATATGAAGAATTGTAATACAAAGTGCAGGTCCACAAAGGGGAGAAAAA
TGCCATGTAATCAGTAC
M
AATATATAACTAGATAGAATAAGATTGAGTATTTGACAGCACAACTGGGTGACTATAGTCAACAATAACTTATTGTACATTTT
AAAATAACTGGTCAGGT / Celera SNP ID: hCV1190563 / Public SNP ID: rs4696565 /
SNP Chromosome Position: 155347711 / SNP in Genomic Sequence: SEQ ID NO: 377 / SNP Position Genomic: 202184 / Related Interrogated SNP: hCV11503414 / Related Interrogated SNP: hCV11503469 / SNP Source: dbSNP; Celera; HGBASE / Population(Allele,Count): Caucasian (A,47|C,73) / SNP Type: INTRON /

Context (SEQ ID NO: 1655): /
TGCATCTTTGCTTGGCCCACCCACAGTCTGAGAGCTGTTCTGGCCCTGGGCAAAAAAATGAGGAAGAATTCAGTATCACACTC
AATTTTACTGTTAAAGC
R
TTCCATTATCCTGCCTGCACCTTTAGATTTGGATGCTTTGGAGCCCAGGCTACATGGATCCCTGGGCCTTTCTTTGATCGCCC
TCCCCTGCCCTATTTTT / Celera SNP ID: hCV1190567 / Public SNP ID: rs4696210 /
SNP Chromosome Position: 155347076 / SNP in Genomic Sequence: SEQ ID NO: 377 / SNP Position Genomic: 201549 / Related Interrogated SNP: hCV11503414 / Related Interrogated SNP: hCV11503469 / SNP Source: dbSNP; Celera; HapMap; HGBASE / Population(Allele,Count): Caucasian (A,47|G,73) / SNP Type: INTRON /

Context (SEQ ID NO: 1656): /
AACAAAATCCAGAGGTGTATCTAGTAACTATTATAATTTCAAAGTAGTTATGACCACAAACAATATTTCAAGATATCTGCAAC
AACCATAATGCTCTACG
R
AAATAGCTAGTATTTCTACTGTTGACAAAATCCAGGTCCTGCTAATACTACTGAGGTTTGCCACATTGTAATTAAAGGAAACA
GTAAATTTTAGATGTGG / Celera SNP ID: hCV1190572 / Public SNP ID: rs1032335 /
SNP Chromosome Position: 155361010 / SNP in Genomic Sequence: SEQ ID NO: 377 / SNP Position Genomic: 215483 / Related Interrogated SNP: hCV11503414 / Related Interrogated SNP: hCV11503469 / Related Interrogated SNP: hCV2892877 / SNP Source: dbSNP; Celera; HapMap; HGBASE / Population(Allele,Count): Caucasian (A,57|G,63) / SNP Type: INTRON /

Context (SEQ ID NO: 1657): /
ACAGAAAATTTCCAAGGTGTTGGTTTCTTAAGCTGGGTAAAGGGTACATGAGTGTTTTTTTTATTATAACTAACCTGTACCTA
TATGTATTCTGTGTACT
S
TTATGAAGATAATGTGCTTCCCAATAAAAATTGTAAGGTTAAAAAGTGAGTTAACATTATCAGTGATAAGCCATGTTGATAGC
ATGTACTTCTGATATGA / Celera SNP ID: hCV1190580 / Public SNP ID: rs9998926 /
SNP Chromosome Position: 155355804 / SNP in Genomic Sequence: SEQ ID NO: 377 / SNP Position Genomic: 210277 / Related Interrogated SNP: hCV11503414 / Related Interrogated SNP: hCV11503469 / SNP Source: dbSNP; Celera; HapMap / Population(Allele,Count): Caucasian (C,87|G,135) / SNP Type: INTRON /

Context (SEQ ID NO: 1658): /
GAGTAATCATCAAAACTACCAAAGTCATCAAAACCAAGGAAAGTCTGGGAAACTGTCACAGACCAGAGGAACCCAGGGAGACA
TGCTGCATAAAGGTAAC
R
TGGTGTCCTGAATGGGATCTCGCAATGGAGAAAGGACAAGAGGGAAAACTAGGGATATTAAGAATGGAGTTCAATCCATAGTA
ATGCACCAAGGTTTGTT / Celera SNP ID: hCV1190581 / Public SNP ID: rs6856249 /

SNP Chromosome Position: 155356127 / SNP in Genomic Sequence: SEQ ID NO: 377 /
SNP Position Genomic: 210600 / Related Interrogated SNP: hCV11503414 /
Related Interrogated SNP: hCV11503469 / SNP Source: dbSNP; Celera; HapMap /
Population(Allele,Count): Caucasian (G,47|A,73) / SNP Type: INTRON /
Context (SEQ ID NO: 1659): /
AAACTAGGGATATTAAGAATGGAGTTCAATCCATAGTAATGCACCAAGGTTTGTTTCTTTGTTTTGACAAATGCACCATGGAA
ATATACGAGGTTAACAA
Y
AGGGAAAACTAGATGAGGGGTACACAGAGAATCTGTACTATCCTTGCAATTCTTCTGTAAATCTAAAACAATTCTAAAAAAAT
TATAAAACTTATTAGAA / Celera SNP ID: hCV1190582 / Public SNP ID: rs10013533 /
SNP Chromosome Position: 155356273 / SNP in Genomic Sequence: SEQ ID NO: 377 /
SNP Position Genomic: 210746 / Related Interrogated SNP: hCV11503414 /
Related Interrogated SNP: hCV11503469 / SNP Source: dbSNP; Celera; HapMap /
Population(Allele,Count): Caucasian (T,47|C,73) / SNP Type: INTRON /
Context (SEQ ID NO: 1660): /
TAGAGCTTCATGCCCTGGTAAGCTCTTCAGGCTAGATATATTTACTCAAGTTATTTCAGAGTAAATAAATCAGTCTTGGAACT
TTGATTATCTCCCATCA
R
ATAAGAAATAGGAAATTGGCCAGGCAAGGTGGCTTATGCCTGTAATCCCAGCACTTTGGGAGGCTGAGGCGGGAGTATCATTT
GAGCCCAGGAGTTCGAG / Celera SNP ID: hCV7429674 / Public SNP ID: rs871540 /
SNP Chromosome Position: 155409030 / SNP in Genomic Sequence: SEQ ID NO: 377 /
SNP Position Genomic: 263503 / Related Interrogated SNP: hCV11503469 /
Related Interrogated SNP: hCV11503414 / SNP Source: dbSNP; HapMap /
Population(Allele,Count): Caucasian (G,54|A,172) / SNP Type: INTRON /
Context (SEQ ID NO: 1661): /
GAAAAAATGTTATGTTGTACCCTTCTTATCCCTACAGGCTAAGAAGTTGTAAGACCAAAGAACACAGTATTAAAGAAGATATA
AAGAGAGGCATTTAAAA
W
AATCACAATTGAACCCTACACTCCTTGCCACATAACTCAGACTCTTTCAGTTTCACCAAATAATGTCTTTCACATGTTTATTA
CTCCATATGTCATTAGA / Celera SNP ID: hCV7430148 / Public SNP ID: rs1490685 /
SNP Chromosome Position: 155361974 / SNP in Genomic Sequence: SEQ ID NO: 377 /
SNP Position Genomic: 216447 / Related Interrogated SNP: hCV11503414 /
Related Interrogated SNP: hCV11503469 / Related Interrogated SNP: hCV2892877 /
SNP Source: dbSNP; Celera; HapMap; HGBASE / Population(Allele,Count):
Caucasian (A,57|T,63) / SNP Type: INTRON /
Context (SEQ ID NO: 1662): /
ATTTGGGCTAAAATTTGTATAAATCAGAAAGGATTCTTTTACTGTTTTATGGTTCAATATGGTAGCTAGTAGCCACACTTGGT
TACTAAGTGCTTGAAAT
R
TGGCTGTATTACAATGCACATGGAGGTATATTGTAAGTATAAAATACACAGGGGATTTCAAAACTTAATTTGAAAACAAAGTA
TGTAAAATATCTCAATA / Celera SNP ID: hCV7430149 / Public SNP ID: rs1490649 /
SNP Chromosome Position: 155357937 / SNP in Genomic Sequence: SEQ ID NO: 377 /
SNP Position Genomic: 212410 / Related Interrogated SNP: hCV11503414 /
Related Interrogated SNP: hCV11503469 / SNP Source: dbSNP; HapMap; HGBASE /
Population(Allele,Count): Caucasian (G,50|A,68) / SNP Type: INTRON /
Context (SEQ ID NO: 1663): /
TTTATTTTGGAGTATGTGATGTGATATATACATATACAATAAACATATACTGTAAACATGTATTTGGCTCGTATACATGAAGT
AGGAACCAGCATGTCCA
R
ATTTTTATTTGGGCTAAAATTTGTATAAATCAGAAAGGATTCTTTTACTGTTTTATGGTTCAATATGGTAGCTAGTAGCCACA
CTTGGTTACTAAGTGCT / Celera SNP ID: hCV7430150 / Public SNP ID: rs1490648 /
SNP Chromosome Position: 155357830 / SNP in Genomic Sequence: SEQ ID NO: 377 /
SNP Position Genomic: 212303 / Related Interrogated SNP: hCV11503414 /
Related Interrogated SNP: hCV11503469 / SNP Source: dbSNP; HapMap; HGBASE /
Population(Allele,Count): Caucasian (G,50|A,70) / SNP Type: INTRON /
Context (SEQ ID NO: 1664): /
AATGGAGTAATAGAATGAAAAAAAAAAAGATGAGAAAAAGACAGAAAATCTCAATCAATCCAACAGCTGTGCCCCAGATCCAG
TCCTAACCCTTCTCCAC
Y
AGGATCTGTGCCCTTCAAGCCTAATCTTGCATGGACCACAGACTGAGGTTCCTTTGCTTCTGGAGTGAGCTCAGCCATCCAAT
AGGAGAAAGGATAAGAA / Celera SNP ID: hCV7430152 / Public SNP ID: rs1490656 /
SNP Chromosome Position: 155353882 / SNP in Genomic Sequence: SEQ ID NO: 377 /
SNP Position Genomic: 208355 / Related Interrogated SNP: hCV11503414 /

Related Interrogated SNP: hCV11503469 / SNP Source: dbSNP; Celera; HapMap; HGBASE / Population(Allele,Count): Caucasian (T,47|C,71) / SNP Type: INTRON /
Context (SEQ ID NO: 1665): /
CATGTCTTCTTCAATTAGTTTTTAGTGGCAGACAGTGTAAGGCTGTAGTGAATACCTAACAGAGAAAAGTTGCCATCAGTTGG TGACAGTAATAGTACCA
W
AGATTCTTTAAAGTCACCTGTTTTCTCACTTTGCAGTCTTTAGATTGGGAGGCATTCCATTCATCATGATGATCTAGGTAACA TAAGGGAATAAGCAGGA / Celera SNP ID: hCV7430153 / Public SNP ID: rs1388077 / SNP Chromosome Position: 155347517 / SNP in Genomic Sequence: SEQ ID NO: 377 / SNP Position Genomic: 201990 / Related Interrogated SNP: hCV11503414 / Related Interrogated SNP: hCV11503469 / SNP Source: dbSNP; Celera; HapMap; HGBASE / Population(Allele,Count): Caucasian (T,47|A,73) / SNP Type: INTRON /
Context (SEQ ID NO: 1666): /
ACTGCCAACCCCCACCCCAGACCCCGCTTCTACTGGCCTATTGCCACCAAAGTCCTTGAGTGACTAACAGGTGACAAAAAATT CTAGTTCATTTTATCGC
W
TCTAAAAATACACTGATATATCTCATGTCTTCTTCAATTAGTTTTTAGTGGCAGACAGTGTAAGGCTGTAGTGAATACCTAAC AGAGAAAAGTTGCCATC / Celera SNP ID: hCV7430158 / Public SNP ID: rs1466662 / SNP Chromosome Position: 155347393 / SNP in Genomic Sequence: SEQ ID NO: 377 / SNP Position Genomic: 201866 / Related Interrogated SNP: hCV11503414 / Related Interrogated SNP: hCV11503469 / Related Interrogated SNP: hCV2892877 / SNP Source: dbSNP; Celera; HapMap; ABI_Val; HGBASE / Population(Allele,Count): Caucasian (A,79|T,41) / SNP Type: INTRON /
Context (SEQ ID NO: 1667): /
TGTAATAGATATTCAATAAGTATTTGTTGGTTAGATGTTGTTGAATTCAATCAATGTTATTGAATTAATGAATGTTGTTGTTT GGATAAATAAATGAATT
R
ATGAATGAACATAGTACTTGTATTTGAGGGAGTATGAAAGAATACTCTTCATGTTACCACATGTAATTCAGGTCTCAATGCTG GAGTTATACTTAGTACA / Celera SNP ID: hCV11503378 / Public SNP ID: rs1490655 / SNP Chromosome Position: 155406865 / SNP in Genomic Sequence: SEQ ID NO: 377 / SNP Position Genomic: 261338 / Related Interrogated SNP: hCV11503414 / Related Interrogated SNP: hCV11503469 / SNP Source: dbSNP; HapMap; HGBASE / Population(Allele,Count): Caucasian (G,24|A,84) / SNP Type: TRANSCRIPTION FACTOR BINDING SITE;INTRON /
Context (SEQ ID NO: 1668): /
ATAAGTATTTGTTGGTTAGATGTTGTTGAATTCAATCAATGTTATTGAATTAATGAATGTTGTTGTTTGGATAAATAAATGAA TTGATGAATGAACATAG
K
ACTTGTATTTGAGGGAGTATGAAAGAATACTCTTCATGTTACCACATGTAATTCAGGTCTCAATGCTGGAGTTATACTTAGTA CAGACTAAGATCTGGTT / Celera SNP ID: hCV11503379 / Public SNP ID: rs1490654 / SNP Chromosome Position: 155406880 / SNP in Genomic Sequence: SEQ ID NO: 377 / SNP Position Genomic: 261353 / Related Interrogated SNP: hCV11503414 / Related Interrogated SNP: hCV11503469 / SNP Source: dbSNP; HapMap; HGBASE / Population(Allele,Count): Caucasian (T,27|G,91) / SNP Type: INTRON /
Context (SEQ ID NO: 1669): /
TAGAAATCCATGGAAGAAACTATCAGTGGGCACTGGCATACCCGGGCTCCTCTACATTTCTCAGCCTCCTTTGCAGTACATCT GACGCTGCATGGCTGGG
M
TCTGGCTAATGTGACATTGGCGGAAGTTGTGTAAGTCACTCTCAGGCCTGGCTCTCTCAGACGGAGGAGAAACAGCCAAGTTG CTTCACATCAGACTCTG / Celera SNP ID: hCV11503382 / Public SNP ID: rs1873369 / SNP Chromosome Position: 155408478 / SNP in Genomic Sequence: SEQ ID NO: 377 / SNP Position Genomic: 262951 / Related Interrogated SNP: hCV11503414 / Related Interrogated SNP: hCV11503469 / Related Interrogated SNP: hCV11503470 / Related Interrogated SNP: hCV15860433 / Related Interrogated SNP: hCV2892877 / SNP Source: dbSNP; Celera; HapMap; HGBASE / Population(Allele,Count): Caucasian (C,76|A,28) / SNP Type: INTRON /
Context (SEQ ID NO: 1670): /
TTAGAATGATCTGAACCTGTGTTGGCAGTCATGCCATTGGGGAACACAGCCATTCAACAGTGGAAGTACTTGCCTTCCTTGAC AGCTGCTAAGAAGGTGA
Y
GCCGCGGCTACAGCTCCCAGATGAGCTCATGTGGGTATGGCAAAAGGTTCACCCCTGTAACATACTGTCCTCTTTAGCAGCTG

314

TTCAACTGCCTAACACA / Celera SNP ID: hCV11853342 / Public SNP ID: rs7660343 / SNP Chromosome Position: 155336123 / SNP in Genomic Sequence: SEQ ID NO: 377 / SNP Position Genomic: 190596 / Related Interrogated SNP: hCV11503469 / SNP Source: dbSNP; Celera; HapMap / Population(Allele,Count): Caucasian (T,180|C,46) / SNP Type: INTRON / Context (SEQ ID NO: 1671): / AAAACTAGAAAAAGGCATTCATTTCAAGCCTGGGCAGCATCAAAAATCCAAAAGCACTCCCCAGGAACATGAGTCTGTTTCCA TGTGCCCTAGGGATTCT R

GTCAGGCATATGGAGACCCAAGGCAGGATAAAATTAAAATATGACCCCACAGGATCCTCCCTGGGGAACGTGGGAAATAATTC CCCTGGATGAAGAGTTA / Celera SNP ID: hCV11853353 / Public SNP ID: rs9995943 / SNP Chromosome Position: 155351598 / SNP in Genomic Sequence: SEQ ID NO: 377 / SNP Position Genomic: 206071 / Related Interrogated SNP: hCV11503469 / Related Interrogated SNP: hCV11503414 / SNP Source: dbSNP; Celera; HapMap / Population(Allele,Count): Caucasian (A,88|G,136) / SNP Type: INTRON / Context (SEQ ID NO: 1672): / CTGGGCAGCATCAAAAATCCAAAAGCACTCCCCAGGAACATGAGTCTGTTTCCATGTGCCCTAGGGATTCTAGTCAGGCATAT GGAGACCCAAGGCAGGA Y

AAAATTAAAATATGACCCCACAGGATCCTCCCTGGGGAACGTGGGAAATAATTCCCCTGGATGAAGAGTTAACTCCAAAAGGA GTCTCTTCCGTAGAGCA / Celera SNP ID: hCV11853354 / Public SNP ID: rs10030235 / SNP Chromosome Position: 155351627 / SNP in Genomic Sequence: SEQ ID NO: 377 / SNP Position Genomic: 206100 / Related Interrogated SNP: hCV11503414 / Related Interrogated SNP: hCV11503469 / SNP Source: dbSNP; Celera; HapMap / Population(Allele,Count): Caucasian (T,88|C,134) / SNP Type: INTRON / Context (SEQ ID NO: 1673): / CCCACTTATCTAAAACAATTACTTATAGGAATTCAATTTCAGGATCATCTAGTACATTGCTTTTGGGGATTCAATTTCATCTT TTCACTCTAAATTCCCA S

TTGTTCCAGCACCATTTTTTACAAAAGACCATCCTACCCTGTGGCCCTGCAGAGCCACTTCTGAAATAAATCAAGTGTCCACA CGTGCAAGTGCTTCTGG / Celera SNP ID: hCV11853357 / Public SNP ID: rs10033383 / SNP Chromosome Position: 155351951 / SNP in Genomic Sequence: SEQ ID NO: 377 / SNP Position Genomic: 206424 / Related Interrogated SNP: hCV11503414 / Related Interrogated SNP: hCV11503469 / SNP Source: dbSNP; Celera; HapMap / Population(Allele,Count): Caucasian (G,47|C,71) / SNP Type: INTRON / Context (SEQ ID NO: 1674): / AGGATCATCTAGTACATTGCTTTTGGGGATTCAATTTCATCTTTTCACTCTAAATTCCCAGTTGTTCCAGCACCATTTTTTAC AAAAGACCATCCTACCC Y

GTGGCCCTGCAGAGCCACTTCTGAAATAAATCAAGTGTCCACACGTGCAAGTGCTTCTGGGCTCTCTTCTGTTCCATTGATTG ATCTGTCTATTCCCACA / Celera SNP ID: hCV11853358 / Public SNP ID: rs10000511 / SNP Chromosome Position: 155351991 / SNP in Genomic Sequence: SEQ ID NO: 377 / SNP Position Genomic: 206464 / Related Interrogated SNP: hCV11503414 / SNP Source: dbSNP; Celera; HapMap / Population(Allele,Count): Caucasian (T,47|C,69) / SNP Type: INTRON / Context (SEQ ID NO: 1675): / GATTACAGGCATGAATCACTGCGCCAGGCCAGTACCCGTTGTTTTAAGCCACTCAGTTTGTGATAATTTGTTGCATAGCCCTA GGCAACTAACGCACTCC Y

CTCACTAGGCTCCTGCAGTGGCCTCGCTGCTCTCACTGGCTCCAGGCCACCTTCTATCTCTTGTGGTTTTCCTACACTTTGTC CATGCCTTCATATATAG / Celera SNP ID: hCV11853362 / Public SNP ID: rs4696572 / SNP Chromosome Position: 155354916 / SNP in Genomic Sequence: SEQ ID NO: 377 / SNP Position Genomic: 209389 / Related Interrogated SNP: hCV11503469 / Related Interrogated SNP: hCV11503414 / SNP Source: dbSNP; Celera; HapMap; HGBASE / Population(Allele,Count): Caucasian (T,145|C,81) / SNP Type: INTRON / Context (SEQ ID NO: 1676): / GGAAAAAGAAACACAAAAATCTTGACAAAGAGAAAGTGCATATGATGATAGATGAAGATAGTCAATCCAAATATCAGTCATA GACATGGATAAACGTCA W

GAAGTAAAAACTGAGCAAAATAAATCAAGTTACAGAGAATACATACAAAATTATATGTTTACATGAAATTCAAAAACTGGCAAA TAATGGGCTGGGCATGA / Celera SNP ID: hCV11853363 / Public SNP ID: rs4696573 / SNP Chromosome Position: 155355215 / SNP in Genomic Sequence: SEQ ID NO: 377 /

SNP Position Genomic: 209688 / Related Interrogated SNP: hCV11503469 / Related Interrogated SNP: hCV11503414 / SNP Source: dbSNP; HapMap / Population(Allele,Count): Caucasian (T,88|A,138) / SNP Type: INTRON / Context (SEQ ID NO: 1677): /
TTCAATTTTGCAAACTCCTTGGTCCAAAGTCATCTATTTATTGAATTCTTTCCTAACCACCTTCTGGCAAAGTTAACTATTCT
TCCTCTGTGATCCCACA
K

CTGTCTACACTTGTACTCACAGAATGCTCCGTGCCAAGTGCTTCACTTTCCTTATCTCATTTCCCTCAAAAGACAGCTCCTGG
AGGAAAGGACTAGAGTG / Celera SNP ID: hCV11853373 / Public SNP ID: rs1907155 /
SNP Chromosome Position: 155361244 / SNP in Genomic Sequence: SEQ ID NO: 377 /
SNP Position Genomic: 215717 / Related Interrogated SNP: hCV11503469 /
Related Interrogated SNP: hCV11503414 / SNP Source: dbSNP; Celera; HapMap;
ABI_Val; HGBASE / Population(Allele,Count): Caucasian (T,107|G,119) / SNP Type:
INTRON /
Context (SEQ ID NO: 1678): /
ATCTATTTATTGAATTCTTTCCTAACCACCTTCTGGCAAAGTTAACTATTCTTCCTCTGTGATCCCACATCTGTCTACACTTG
TACTCACAGAATGCTCC
R

TGCCAAGTGCTTCACTTTCCTTATCTCATTTCCCTCAAAAGACAGCTCCTGGAGGAAAGGACTAGAGTGAGACGAAGCTGATG
GAGCCAGTGCCCTGCCT / Celera SNP ID: hCV11853378 / Public SNP ID: rs1907154 /
SNP Chromosome Position: 155361275 / SNP in Genomic Sequence: SEQ ID NO: 377 /
SNP Position Genomic: 215748 / Related Interrogated SNP: hCV11503414 /
Related Interrogated SNP: hCV11503469 / Related Interrogated SNP: hCV2892877 /
SNP Source: dbSNP; Celera; HapMap; ABI_Val; HGBASE / Population(Allele,Count):
Caucasian (G,57|A,63) / SNP Type: INTRON /
Context (SEQ ID NO: 1679): /
TCTTTGAGAGCAAAAAAGATAACATTCTGAATCAGCATTTTGGGAAGCAAAGGGATTACACAGTTCATGAATTAATTAAACAC
AGTAAATAATGTTCTTT
W

TCCCCAACCTTCTTAAATCAAATACATGTAAAGTCTATTTTTTAAAAGCACACAGTATGCAAAATATTTGTCATAAAATAAAA
GTAAACTGAAAATTGGT / Celera SNP ID: hCV11853384 / Public SNP ID: rs12646456 /
SNP Chromosome Position: 155362549 / SNP in Genomic Sequence: SEQ ID NO: 377 /
SNP Position Genomic: 217022 / Related Interrogated SNP: hCV11503414 /
Related Interrogated SNP: hCV11503469 / Related Interrogated SNP: hCV2892877 /
SNP Source: dbSNP; Celera; HapMap / Population(Allele,Count): Caucasian
(T,57|A,63) / SNP Type: INTRON /
Context (SEQ ID NO: 1680): /
ATTAAAAAAAAAAAACTATAACACAACATTTATCAGTTAGCAAAACTCAATTTAATATAGTTTAAAACTATTAAATTTGTACTG
AATGAGCTACGTCACCA
Y

TTATTAGGCTTTTTTTTTTAACTTGAAAGTCTCAGTGTTTATAAAGCACTTCTTGTTTTATAAAAACAGCTATTTATTAATAAA
CATAAATGTACACATAT / Celera SNP ID: hCV11853387 / Public SNP ID: rs67693758 /
SNP Chromosome Position: 155362876 / SNP in Genomic Sequence: SEQ ID NO: 377 /
SNP Position Genomic: 217349 / Related Interrogated SNP: hCV11503414 /
Related Interrogated SNP: hCV11503469 / Related Interrogated SNP: hCV2892877 /
Related Interrogated SNP: hCV15860433 / SNP Source: dbSNP; Celera; HapMap;
ABI_Val; HGBASE / Population(Allele,Count): Caucasian (T,73|C,45) / SNP Type:
INTRONIC INDEL; INTRON /
Context (SEQ ID NO: 1681): /
ATGCTGGAGTTATACTTAGTACAGACTAAGATCTGGTTATAAGATAAAAGCTGCAAATGCAGCTCACAACATCAGACTTAGTG
TAGGGTGGAGGATGGAG
M

TCTTGTCTAGAGGTTGAAGTTAATCTTTTTGACAGTTATCTGAGCGAAAATGGAATCTTTCAGGCCAAACATGAGAACTGGAG
GAAAATCTCTGGGTGTC / Celera SNP ID: hCV11853415 / Public SNP ID: rs1490653 /
SNP Chromosome Position: 155407043 / SNP in Genomic Sequence: SEQ ID NO: 377 /
SNP Position Genomic: 261516 / Related Interrogated SNP: hCV11503414 / SNP
Source: dbSNP; Celera; HGBASE / Population(Allele,Count): Caucasian
(C,24|A,90) / SNP Type: INTRON /
Context (SEQ ID NO: 1682): /
GCAGCTAGAGGTTATCTATCTCAGTTTGCTAATGGTTCTTGTCCTCTGCATTAATTCCCTCAGTTCTGCCTCACTCATAAGTC
TTTGGAAAGGTCTCGTC
W

GGGCATGAGAATATAAGAGTTCAGCCAACACTCGGAGAGGGGAGGGTGGCCAGCACCATAGTGCCAGCAACAGCAGCAACTAG

AGTGGTAGGACCCCAAA / Celera SNP ID: hCV11853416 / Public SNP ID: rs4346631 / SNP Chromosome Position: 155407407 / SNP in Genomic Sequence: SEQ ID NO: 377 / SNP Position Genomic: 261880 / Related Interrogated SNP: hCV11503414 / Related Interrogated SNP: hCV11503469 / SNP Source: dbSNP; Celera; HapMap; HGBASE / Population(Allele,Count): Caucasian (A,26|T,90) / SNP Type: INTRON /

Context (SEQ ID NO: 1683): /
TGCCAGCAACAGCAGCAACTAGAGTGGTAGGACCCCAAACATTGGGGATGTGGAGTAACCACTAAGACAACTTGAAGTTAAAG GGTTTGTTTTTATCAGA
Y

GACATGGTATTATCTTACAGGAAATCTAGGCAAGATTTTGTTTTTCCTGTATTTCCTTTTTGTTTTTGTTTGATTTTGACAAAG GCAACTTGAGCAGATAT / Celera SNP ID: hCV11853418 / Public SNP ID: rs12501998 / SNP Chromosome Position: 155407569 / SNP in Genomic Sequence: SEQ ID NO: 377 / SNP Position Genomic: 262042 / Related Interrogated SNP: hCV11503469 / Related Interrogated SNP: hCV11503414 / SNP Source: dbSNP; Celera; HapMap / Population(Allele,Count): Caucasian (T,54|C,172) / SNP Type: INTRON /
Context (SEQ ID NO: 1684): /
TATCTTACAGGAAATCTAGGCAAGATTTTGTTTTTCCTGTATTTCCTTTTTGTTTTTGTTTGATTTTGACAAAGGCAACTTGAG CAGATATAAGCTGTAAA
R

GAGGAAAACTTGTAGATGGCAACAGAAAGAAGGTACAGTTACAAGGAATGTATGTAATGTCATTAAGCAAAGCCATGGAACAC AGGAGGTGTCATGAGAT / Celera SNP ID: hCV11853419 / Public SNP ID: rs13151559 / SNP Chromosome Position: 155407680 / SNP in Genomic Sequence: SEQ ID NO: 377 / SNP Position Genomic: 262153 / Related Interrogated SNP: hCV11503469 / Related Interrogated SNP: hCV11503414 / SNP Source: dbSNP; Celera; HapMap / Population(Allele,Count): Caucasian (A,54|G,172) / SNP Type: ACCEPTOR SPLICE SITE;INTRON /
Context (SEQ ID NO: 1685): /
ATGGAGTTGTAACAATGGGATAAACTCACACCTCATAGAAGAATCATCTCACGATGATAGAAACAACTCTAAAGACAGGCTTT CATCAAAAAAATCATAA
Y

TAATCCACCCTGCTTATTTTCCTAACAATGGAGCCAAGTTGTACTATTCATACCAAGATCTTCAGGGAGTTCCAGGTGGAAGG AATATTGGAAGTCAACT / Celera SNP ID: hCV11853423 / Public SNP ID: rs3857093 / SNP Chromosome Position: 155408243 / SNP in Genomic Sequence: SEQ ID NO: 377 / SNP Position Genomic: 262716 / Related Interrogated SNP: hCV11503469 / Related Interrogated SNP: hCV11503414 / SNP Source: dbSNP; Celera; HapMap; HGBASE / Population(Allele,Count): Caucasian (T,54|C,172) / SNP Type: TFBS SYNONYMOUS;INTRON /
Context (SEQ ID NO: 1686): /
GTTGTGTAAGTCACTCTCAGGCCTGGCTCTCTCAGACGGAGGAGAAACAGCCAAGTTGCTTCACATCAGACTCTGCTTGAGAG AGAAATATACTTTGACA
R

TGTTAGTCACTAATATTTGTGGATTTGTCCATTTCATCAGCAAGCATTAATTCATATCAAGCCACTTTTTAATAATCTAGCAA CTCAGAGCTGGCAATCA / Celera SNP ID: hCV11853424 / Public SNP ID: rs871541 / SNP Chromosome Position: 155408604 / SNP in Genomic Sequence: SEQ ID NO: 377 / SNP Position Genomic: 263077 / Related Interrogated SNP: hCV11503469 / Related Interrogated SNP: hCV11503414 / SNP Source: dbSNP; Celera; HapMap; HGBASE / Population(Allele,Count): Caucasian (G,54|A,172) / SNP Type: INTRON /
Context (SEQ ID NO: 1687): /
AAACCTTCTCCTTCATCCTGTTCCATCTTGAGCATATAACCAGAATGTGACTTTTGGAGCTATTTTGAAGTAGTCTAGCTTAG TGGTTCTCAACTAGGGA
M

AAGTTTGCCCCCCAAGAAGACATTTAGTATGTCTAGAGACACTTTTGGTTGTCACAACTGGGGAAGGGTTTCTACTTTCATTT AATGGGTTAATTTAATG / Celera SNP ID: hCV11853631 / Public SNP ID: rs12651106 / SNP Chromosome Position: 155301059 / SNP in Genomic Sequence: SEQ ID NO: 377 / SNP Position Genomic: 155532 / Related Interrogated SNP: hCV11503414 / Related Interrogated SNP: hCV11503469 / Related Interrogated SNP: hCV2892877 / SNP Source: dbSNP; Celera; HapMap / Population(Allele,Count): Caucasian (C,189|A,37) / SNP Type: INTRON /
Context (SEQ ID NO: 1688): /
ATTAAGGTTTGGAAACCTATTCTCAATTTAGCAGCTTTGCTTCATGGAGGAAAAGAGAAAACCATGCACATAGATCTAACTAT GGAAGTGTGGGATGCAC

Y
GTTTTGTATACCAGCTCCCAATATAATCACTTTTACAGAACTGTATATTTATTGTAAACATACTATTTAGGGGACTTCTTATT
ACATTTTAGAGTCCCCT / Celera SNP ID: hCV11853650 / Public SNP ID: rs9307922 /
SNP Chromosome Position: 155313844 / SNP in Genomic Sequence: SEQ ID NO: 377 /
SNP Position Genomic: 168317 / Related Interrogated SNP: hCV11503414 / SNP
Source: dbSNP; Celera; HapMap / Population(Allele,Count): Caucasian
(C,39|T,81) / SNP Type: INTRON /
Context (SEQ ID NO: 1689): /
CTGTATTTACATGGCTGTTAAATGATATTCCATGTTACAATCCAAATATCTACATATACTATACAAATATGTGCAGATATATG
CTGAAAACTCTAAAGAA
R
ATAAAACAAATGGAATTCTCTGTTTTCAAGTGTTGATAATTAGAATGTTCACTGATTCTAAAGTTTATTAGACTGGTGGTAGT
ATCAGTTCAAATACTGT / Celera SNP ID: hCV26024202 / Public SNP ID: rs11731813 /
SNP Chromosome Position: 155379883 / SNP in Genomic Sequence: SEQ ID NO: 377 /
SNP Position Genomic: 234356 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; Celera; HapMap; ABI_Val /
Population(Allele,Count): Caucasian (A,170|G,54) / SNP Type: INTRON /
Context (SEQ ID NO: 1690): /
AGATAGAGCAGGGGCAAAGAGATAACTTTAAGGGAGAAACAGAAATTCAGTTAGGTATAGGAATTGGAAAGACAAAAGAGAAG
GAAGTCATTTTAAGTTA
Y
GGGAACAGACTAAATACCAACATGAAAGAAGAATTAGCAAGGACTGATAGAGGCAAGAAGACAATCCTTGTAATTAGATCTAG
AACCAAATAAGGCAGGA / Celera SNP ID: hCV26024285 / Public SNP ID: rs11726919 /
SNP Chromosome Position: 155192201 / SNP in Genomic Sequence: SEQ ID NO: 377 /
SNP Position Genomic: 46674 / Related Interrogated SNP: hCV11503414 / SNP
Source: dbSNP; Celera; HapMap / Population(Allele,Count): Caucasian
(C,45|T,75) / SNP Type: INTRON /
Context (SEQ ID NO: 1691): /
ATTAAAGACATTTGACACACTGCCCCAAAACACTGGAGGTGGGAGTGGGAAAGGAGGCTATGAGAGCATAAAGAAAAGAAATA
TTCCCTCAACCAGCTTC
Y
GATCTAGCTGTAGGAAAAAGCAACCCAACTAGAGAGTAACCAGCAACTTAACTGCAAGACATGAATTAATTCAAGATTTGTTC
AGATAGAGCAGGGGCAA / Celera SNP ID: hCV26024286 / Public SNP ID: rs11726850 /
SNP Chromosome Position: 155192017 / SNP in Genomic Sequence: SEQ ID NO: 377 /
SNP Position Genomic: 46490 / Related Interrogated SNP: hCV11503414 / SNP
Source: dbSNP; Celera; HapMap / Population(Allele,Count): Caucasian
(C,45|T,75) / SNP Type: INTRON /
Context (SEQ ID NO: 1692): /
TTTTTGAATTCAGACTCAGTGTCAATGATCTCACTACTCTATCTCCAATTTTCCCAAATACATGGGTAAATTGTAAGTGGAAG
CTAATTTATCTTCAAAA
R
TTTGAAGGGCAGGATAGACACTGTCTAGTTTGGAAATATTAAGAATATAATTCTTAGTGAATATAAATAAATAGTTTTTGATT
TCCCATAATTTTTCATA / Celera SNP ID: hCV26024287 / Public SNP ID: rs7666541 /
SNP Chromosome Position: 155190818 / SNP in Genomic Sequence: SEQ ID NO: 377 /
SNP Position Genomic: 45291 / Related Interrogated SNP: hCV11503414 /
Related Interrogated SNP: hCV11503469 / SNP Source: dbSNP; Celera; HapMap /
Population(Allele,Count): Caucasian (A,41|G,69) / SNP Type: INTRON /
Context (SEQ ID NO: 1693): /
TTCTTCTTGTGATGATGTGAGATGACAAAATGCCCACATGATAAGGTAAAGTGAGGTAAATGACATGGGCACTGTGACATAGC
AGTAGGCTACTACTGAA
Y
GTCTGATGGTATGTCAGGAGAAGGATCATCATCTGCTTCAAGTGGTCCTGGATCAACGAGCCACGATGATGTCGATGGTTGGA
TGTCAAGAGCAGATGAT / Celera SNP ID: hCV26024294 / Public SNP ID: rs11731663 /
SNP Chromosome Position: 155187341 / SNP in Genomic Sequence: SEQ ID NO: 377 /
SNP Position Genomic: 41814 / Related Interrogated SNP: hCV11503414 / SNP
Source: dbSNP; Celera; HapMap / Population(Allele,Count): Caucasian
(T,45|C,75) / SNP Type: INTRON /
Context (SEQ ID NO: 1694): /
GCACTTCGAGATCAGTAATTGCAAATCTCTTGTATTACTCAGGAAGAGAATATTGATTAATGTTGGACTTTATATATGTTAAA
GTAACGAGGCTAGCCAC
Y

GAAATAAGAAAAATAACTATATAACTTCAGACAGGGGAAAGTGGTCATTTTCAGATATGATTTTATGTGTCAAATATACAGAA
AAATGTCAGTCTCTAGT / Celera SNP ID: hCV26024295 / Public SNP ID: rs12643125 /
SNP Chromosome Position: 155186191 / SNP in Genomic Sequence: SEQ ID NO: 377 /
SNP Position Genomic: 40664 / Related Interrogated SNP: hCV11503469 / SNP
Source: dbSNP; Celera / Population(Allele,Count): Caucasian (C,92|T,130) /
SNP Type: INTRON /
Context (SEQ ID NO: 1695): /
TGTGGCTTTGAGTACAGAAAATGGGAAATTCCATGCAAGTGGATCTTCAAGAGGAAAACAGTTAAAGAACTATACTCCTCATG
GGCTGAACATGCAGAAG
R

AGCCGACCCCTGGGAGAAGGTTACCCTGGGGAAACAGCACAGAGGTTGCTGAGAATCCAAAGAGAAAGGCGACAGTGGGAAAG
GGAGGATAACATAGATG / Celera SNP ID: hCV27907560 / Public SNP ID: rs4696576 /
SNP Chromosome Position: 155371246 / SNP in Genomic Sequence: SEQ ID NO: 377 /
SNP Position Genomic: 225719 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; HapMap; ABI_Val; HGBASE /
Population(Allele,Count): Caucasian (G,153|A,73) / SNP Type: INTRON /
Context (SEQ ID NO: 1696): /
CCGCGTCCGTGGCCACCAGTAGTAACTCATACAGATCGCGGCTCTCTCTGTCCAGGGGCCCCTCCACGCAAAGGAAAAATACC
CCTGGGGGGCCGCCGGG
Y

AGCAACGCGAAGTCTCCCTCTCCGCCTTCCAAGGACAGAGAGATGCTCCCGTCTCCAAGACCCACACCAAGCTCCCCTGTGGC
CTCATCTTCCTTCTCCC / Celera SNP ID: hCV28954780 / Public SNP ID: rs7656522 /
SNP Chromosome Position: 155411065 / SNP in Genomic Sequence: SEQ ID NO: 377 /
SNP Position Genomic: 265538 / Related Interrogated SNP: hCV11503469 /
Related Interrogated SNP: hCV11503414 / SNP Source: dbSNP; HapMap /
Population(Allele,Count): Caucasian (T,54|C,170) / SNP Type: UTR5;SILENT RARE
CODON;SILENT MUTATION /
Context (SEQ ID NO: 1697): /
GCAGAATGATATTCAATATCATAATCCCTGTAGTCCGATGAAATCATTCTGTCATATGTTCATGCCAAGATTATTGAATAGCA
TGAAACAGTTTTGAAGA
W

ACATGCACATTTAGTCATTGTGATGTGTAAAATCTGGCAGGTACACATTTAAATGAGTCTGACTAGGTAGTTGGTTTCAATGC
CTGACCACCATTACCCT / Celera SNP ID: hCV28954790 / Public SNP ID: rs7662783 /
SNP Chromosome Position: 155389095 / SNP in Genomic Sequence: SEQ ID NO: 377 /
SNP Position Genomic: 243568 / Related Interrogated SNP: hCV11503469 / SNP
Source: dbSNP; HapMap / Population(Allele,Count): Caucasian (T,90|A,20) / SNP
Type: INTRON /
Context (SEQ ID NO: 1698): /
ATTGGTACCTAGAATGCATGTGGTAAATTGTATTTTTTCTTGCCCTCTGGTGTTTGTATGATGAAATGTTTTAGGAGTGAATTA
TTTTGGAATTCTCAGTA
R

TATCAAACTCCGACTTCTTAGCCCCCCAGAAGGTGTGAAGGGGGAGGATATAAAATTCCCACTATCTGGCTGGATGCAGTGGC
TCATGCCTGTAATCCCA / Celera SNP ID: hCV28954801 / Public SNP ID: rs4447837 /
SNP Chromosome Position: 155416594 / SNP in Genomic Sequence: SEQ ID NO: 377 /
SNP Position Genomic: 271067 / Related Interrogated SNP: hCV11503469 / SNP
Source: dbSNP; HapMap; HGBASE / Population(Allele,Count): Caucasian
(A,177|G,49) / SNP Type: INTERGENIC;UNKNOWN /
Context (SEQ ID NO: 1699): /
GGGATTGCATTGCATCTAAAGATCAGTTTAGGGCAGCATTAGCATCTTTACAATATTGAAAACTAGAGTGAACAGACCTCAGC
TTGGATGCAGGCGGGGG
S

AGATTTAACACTAAATTTTCTAAGTTTGGACAATTACAGGAGGGTGGGGATCCTGTTTACTGAAACAAGGCAGTGTGTATGAC
TTAAAGAAATCATAAAA / Celera SNP ID: hCV28966638 / Public SNP ID: rs7676857 /
SNP Chromosome Position: 155352374 / SNP in Genomic Sequence: SEQ ID NO: 377 /
SNP Position Genomic: 206847 / Related Interrogated SNP: hCV11503414 /
Related Interrogated SNP: hCV11503469 / Related Interrogated SNP: hCV15860433
/ Related Interrogated SNP: hCV2892877 / SNP Source: dbSNP; HapMap /
Population(Allele,Count): Caucasian (G,42|C,74) / SNP Type: INTRON /
Context (SEQ ID NO: 1700): /
CTAGAAACAACAGATCCATAAAGCTTCAGATATTAAAGCTGCATTGTCAGACAGAGGCTTTAAAAGCTGTATATTTTTTGTTG
CTGTTGATAAGGAAAAG

R
TGATAAATTTTAACAGATAACCGCCCAAAAATAATTAATTAATTTAAATTAATTAAGTTTCAAATTTCTACTTCCCAGCATGC
TGGTGTAATTGGGACTG / Celera SNP ID: hCV30679164 / Public SNP ID: rs12649437 /
SNP Chromosome Position: 155387171 / SNP in Genomic Sequence: SEQ ID NO: 377 /
SNP Position Genomic: 241644 / Related Interrogated SNP: hCV11503414 /
Related Interrogated SNP: hCV11503469 / Related Interrogated SNP: hCV2892877 /
SNP Source: dbSNP; HapMap / Population(Allele,Count): Caucasian (A,147|G,79)
/ SNP Type: INTRON /
Context (SEQ ID NO: 1701): /
TTTTGCATTTGGGCCTGAGAGAAGAATTAATTTCCTAGATACCGTTTCTCTCCTTTAGGGAGGACCAAAAGCCCTGCATAGTG
GTTTCTTGCAGCAACAA
Y
AAAATGCCTTTTTAAATGTGCAACGGAGGTGTATGTAATTGCTAATCCACAGCCACACCGTTATCTCTACCTGTGACCAAATA
ACAATAAAATAATTGAC / Celera SNP ID: hCV30679141 / Public SNP ID: rs13111621 /
SNP Chromosome Position: 155414148 / SNP in Genomic Sequence: SEQ ID NO: 377 /
SNP Position Genomic: 268621 / Related Interrogated SNP: hCV11503414 / SNP
Source: dbSNP; HapMap / Population(Allele,Count): Caucasian (C,95|T,25) / SNP
Type: INTERGENIC;UNKNOWN /
Context (SEQ ID NO: 1702): /
TTCCTAGATACCGTTTCTCTCCTTTAGGGAGGACCAAAAGCCCTGCATAGTGGTTTCTTGCAGCAACAACAAAATGCCTTTTT
AAATGTGCAACGGAGGT
R
TATGTAATTGCTAATCCACAGCCACACCGTTATCTCTACCTGTGACCAAATAACAATAAAATAATTGACTTTGTAGCATATAT
GTTACCGAAAATGGGGA / Celera SNP ID: hCV30679140 / Public SNP ID: rs13112066 /
SNP Chromosome Position: 155414179 / SNP in Genomic Sequence: SEQ ID NO: 377 /
SNP Position Genomic: 268652 / Related Interrogated SNP: hCV11503469 /
Related Interrogated SNP: hCV11503414 / SNP Source: dbSNP; HapMap /
Population(Allele,Count): Caucasian (G,174|A,52) / SNP Type:
INTERGENIC;UNKNOWN /
Context (SEQ ID NO: 1703): /
ACCGTTTCTCTCCTTTAGGGAGGACCAAAAGCCCTGCATAGTGGTTTCTTGCAGCAACAACAAAATGCCTTTTTTAAATGTGCA
ACGGAGGTGTATGTAAT
K
GCTAATCCACAGCCACACCGTTATCTCTACCTGTGACCAAATAACAATAAAATAATTGACTTTGTAGCATATATGTTACCGAA
AATGGGGACATTATTAA / Celera SNP ID: hCV30679139 / Public SNP ID: rs13139082 /
SNP Chromosome Position: 155414188 / SNP in Genomic Sequence: SEQ ID NO: 377 /
SNP Position Genomic: 268661 / Related Interrogated SNP: hCV11503469 /
Related Interrogated SNP: hCV11503414 / SNP Source: dbSNP; HapMap /
Population(Allele,Count): Caucasian (T,91|G,23) / SNP Type:
INTERGENIC;UNKNOWN /
Context (SEQ ID NO: 1704): /
AATAATTAAAAAAACTAGCAGGGTAAAATATCTACAGAACATGTAAAAACCCTATTTTCAGCCTCCACTTTCAACTGAAAGACA
TAATTGAATCACTGTAT
R
CCATTGTTCCCAAAATGGGCTTCCAGACCAAGAAAAGCTCAAATGTGTTTAAAAGGAGAAGCAATCCAGTGAGCAGAGGAATA
ACATATGAATCACAAGC / Celera SNP ID: hCV30679170 / Public SNP ID: rs13148992 /
SNP Chromosome Position: 155382108 / SNP in Genomic Sequence: SEQ ID NO: 377 /
SNP Position Genomic: 236581 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP / Population(Allele,Count): Caucasian
(G,171|A,55) / SNP Type: INTRON /
Context (SEQ ID NO: 1705): /
AGCCATCTCATTGAACCCTCATACACAAGCCCCAGAGACCCCAACTTGAAACCCATCTTGCTAGAGGTAATAACAAAAATGAC
CCCTAAACCAAGCCAAA
W
TGTTATTTTTTTTTTTCTGGATATGTAAAAGGATTTTACTGTAAAGAACAGAAAAAAGAAAAGGCTCTGGAACAAATAGTAGTT
TTCCTTAGAAGCCTTTC / Celera SNP ID: hCV30679242 / Public SNP ID: rs4235243 /
SNP Chromosome Position: 155196191 / SNP in Genomic Sequence: SEQ ID NO: 377 /
SNP Position Genomic: 50664 / Related Interrogated SNP: hCV11503414 / SNP
Source: dbSNP; HapMap; HGBASE / Population(Allele,Count): Caucasian
(T,48|A,72) / SNP Type: INTRON /
Context (SEQ ID NO: 1706): /

GGTAAAAAAAAGAACTCCATTAGGATAAAAAAAATTTCATGGAAACACAAGAGAAAATACTGATTATTTATTATTTGCCTACTAT
GTTTTCACTTGTTCATT
S

ATTCAACAATAACTTATTGTCTTCAATGAGCCAGATCCCATGCTAGGTGCTAGGGATATACTAGCAAACAAGATAGAAAAGAT
CTCTGTCCTCATGGTGG / Celera SNP ID: hCV30679245 / Public SNP ID: rs4386583 /
SNP Chromosome Position: 155191278 / SNP in Genomic Sequence: SEQ ID NO: 377 /
SNP Position Genomic: 45751 / Related Interrogated SNP: hCV11503414 / SNP
Source: dbSNP; HapMap; HGBASE / Population(Allele,Count): Caucasian
(G,45|C,75) / SNP Type: TFBS SYNONYMOUS;INTRON /
Context (SEQ ID NO: 1707): /
ATTCTCATTCGTATGTGGGAGCTAAGCTAAGAGGATGCAAAGGCATAAGAATGATACAATGAACTTTGGGGACTTGGGGGAAA
AGGTGGGGAAGTGAAGG
R

TAAAAGACTATACAATAGATACTATATACACTGTTTGGGTGACGGGTGCACCAAAATCTCAGAAATCACCACTAAATAAGTTA
TTCATGTAACCAAAGAC / Celera SNP ID: hCV30679244 / Public SNP ID: rs4575978 /
SNP Chromosome Position: 155194629 / SNP in Genomic Sequence: SEQ ID NO: 377 /
SNP Position Genomic: 49102 / Related Interrogated SNP: hCV11503414 / SNP
Source: dbSNP; HapMap; HGBASE / Population(Allele,Count): Caucasian
(A,45|G,75) / SNP Type: INTRON /
Context (SEQ ID NO: 1708): /
CAACAGTTCGCTATTTTCATTACTTTAATTTTAGGGTAGCTTTAATATCTGGTTGTACAGGCATACTCTTACAATTCTTTTTC
AAGATTTTCTTGGTTAT
K

CTCACCTACATTTTTTGTTCTTGCAGAACTTTAGGATAATCTTGCCAAGTTTAATTTCCCATGGGTTTTGACTAGATTTGCGC
TAACATCGGTAAACTTA / Celera SNP ID: hCV29751345 / Public SNP ID: rs6811271 /
SNP Chromosome Position: 155359651 / SNP in Genomic Sequence: SEQ ID NO: 377 /
SNP Position Genomic: 214124 / Related Interrogated SNP: hCV11503469 /
Related Interrogated SNP: hCV11503414 / SNP Source: dbSNP /
Population(Allele,Count): Caucasian (T,108|G,118) / SNP Type: INTRON /
Context (SEQ ID NO: 1709): /
TGTCTTCTTGAGACCCTGGTTAATATATCTGCTTTATTTTCCTTCTTGTACTTAAGATCTAAAATAATCTTATTCATTTATTT
GCTTACTGGGGTTTATT
W

TCAAGACTCTTCTCCTCCCTACAAAAAAAATAACAATAAATTCCATATGGCAGGCCTTTTTTCTTATTCACCACTATCTCCTCA
GCTTCTAGAATAGGGCA / Celera SNP ID: hCV30004073 / Public SNP ID: rs6832957 /
SNP Chromosome Position: 155335174 / SNP in Genomic Sequence: SEQ ID NO: 377 /
SNP Position Genomic: 189647 / Related Interrogated SNP: hCV11503414 / SNP
Source: dbSNP; HapMap / Population(Allele,Count): Caucasian (A,92|T,28) / SNP
Type: INTRON /
Context (SEQ ID NO: 1710): /
TCAAGATTTTCTTGGTTATTCTCACCTACATTTTTTGTTCTTGCAGAACTTTAGGATAATCTTGCCAAGTTTAATTTCCCATG
GGTTTTGACTAGATTTG
R

GCTAACATCGGTAAACTTATATTGAATCATTCAATTCACGAACATGTTACAACTCTCCATTTATTCAGGTCTTCTTTTATGTA
CCTCCATAAAATTTAAT / Celera SNP ID: hCV29859776 / Public SNP ID: rs6834312 /
SNP Chromosome Position: 155359732 / SNP in Genomic Sequence: SEQ ID NO: 377 /
SNP Position Genomic: 214205 / SNP Source: dbSNP; HapMap /
Population(Allele,Count): Caucasian (G,57|A,57) / SNP Type: INTRON /
Context (SEQ ID NO: 1711): /
ATTTTTTTTCACAACTTCTTGACACTAACAGATGAGAAAGAAGGCCACTTTTGCTCAAGAACCAAAACAGAGTACTGAGTCCAT
CGTCAAGAGATATCAGA
M

ATTGAAAAAGATAGCAGCACAGGTTTCCAGAACTCTTCTTTGAGTAGGAAAGACTTGGGGAAAAAAATGGGCCAATAGGTAGC
AGACATCAACTCCTACA / Celera SNP ID: hCV30562176 / Public SNP ID: rs9284660 /
SNP Chromosome Position: 155403593 / SNP in Genomic Sequence: SEQ ID NO: 377 /
SNP Position Genomic: 258066 / Related Interrogated SNP: hCV11503414 /
Related Interrogated SNP: hCV11503469 / SNP Source: dbSNP /
Population(Allele,Count): Caucasian (C,149|A,77) / SNP Type: INTRON /
Context (SEQ ID NO: 1712): /
TTTAAAGCTGTCTCAAATCATATTACCTCCTGCTTAGAACCTGAAATGATTCCATTATACTTAGATTATAATTCAGACTCCTT
CAATCAAGCTCCTTTTA
R

TAGTACAGGATCCTATCCATCTTTCTGACCTTGCCTCATACCACCACTCTGGACTCAGCCATCTGCCTTCTTTTTGATCTTGT

ACCTTTGAATTGTTAGA / Celera SNP ID: hDV70817639 / Public SNP ID: rs17031739 /
SNP Chromosome Position: 155414923 / SNP in Genomic Sequence: SEQ ID NO: 377 /
SNP Position Genomic: 269396 / Related Interrogated SNP: hCV11503469 / SNP
Source: dbSNP; HapMap / Population(Allele,Count): Caucasian (G,176|A,48) /
SNP Type: INTERGENIC;UNKNOWN /
Context (SEQ ID NO: 1713): /
ATTATTACAACCTCAAAACCTTCATTCTGCTTTATCTCAGCACCTAGAACAGTACCTAGCATGTAGTGTGTCTCTATTTACTA
AGTATGTGTTGGACAGA
Y
GGTTGGTTGAACCTATGGGTAAGTGAATGCATTTGTCTGTTAAAGTAGAAGTTGGAAGCCATAACCAAGAATGAATTGGCCAT
GAGAATCTTCACTGTAA / Celera SNP ID: hDV70817640 / Public SNP ID: rs17031740 /
SNP Chromosome Position: 155415340 / SNP in Genomic Sequence: SEQ ID NO: 377 /
SNP Position Genomic: 269813 / Related Interrogated SNP: hCV11503469 / SNP
Source: dbSNP; HapMap / Population(Allele,Count): Caucasian (C,177|T,49) /
SNP Type: INTERGENIC;UNKNOWN /
Context (SEQ ID NO: 1714): /
TCAATTAACTATAATAATCGGTAGAAACTGATCAATGGCCTCTAATAAAGTACGCCTGAGAAAAGACTCTCTGGACTCTTAAA
AATGGCTGACCATATTT
R
GCAAGTGGTTTCTGCAATTTTGCATTTGGGCCTGAGAGAAGAATTAATTTCCTAGATACCGTTTCTCTCCTTTAGGGAGGACC
AAAAGCCCTGCATAGTG / Celera SNP ID: hDV70934991 / Public SNP ID: rs17301943 /
SNP Chromosome Position: 155414030 / SNP in Genomic Sequence: SEQ ID NO: 377 /
SNP Position Genomic: 268503 / Related Interrogated SNP: hCV11503469 /
Related Interrogated SNP: hCV11503414 / SNP Source: dbSNP; HapMap /
Population(Allele,Count): Caucasian (A,172|G,54) / SNP Type: TRANSCRIPTION
FACTOR BINDING SITE;INTERGENIC;UNKNOWN /
Context (SEQ ID NO: 1715): /
GGTGACGAGGAGCCTGGCAAAAGCGGTGACGGTAGTGGCCCCGGAGTCCGGTAGCGCAACTGGAAGAACGGGCCTGCGGGGTC
CTTGGGCAGGTCGGACG
R
TTGCACCAGGGTGTAGCCCTGAGTGCTGAACAGTCCGGCGTCCGGATCGTGGGCAACTGGCAGGCGGAAGGCGGTCCCTGGCG
GGCTGAGCTCGGAGACG / Celera SNP ID: hDV70945235 / Public SNP ID: rs17373860 /
SNP Chromosome Position: 155411883 / SNP in Genomic Sequence: SEQ ID NO: 377 /
SNP Position Genomic: 266356 / Related Interrogated SNP: hCV11503414 /
Related Interrogated SNP: hCV11503469 / Related Interrogated SNP: hCV11503470
/ Related Interrogated SNP: hCV15860433 / Related Interrogated SNP:
hCV2892877 / SNP Source: dbSNP; HapMap / Population(Allele,Count): Caucasian
(G,104|A,12) / SNP Type: MISSENSE MUTATION;UTR5 /
Context (SEQ ID NO: 1716): /
CAAGATTTTCTTGGTTATTCTCACCTACATTTTTTGTTCTTGCAGAACTTTAGGATAATCTTGCCAAGTTTAATTTCCCATGG
GTTTTGACTAGATTTGC
R
CTAACATCGGTAAACTTATATTGAATCATTCAATTCACGAACATGTTACAACTCTCCATTTATTCAGGTCTTCTTTTTATGTAC
CTCCATAAAATTTAATA / Celera SNP ID: hDV96226316 / Public SNP ID: rs6834312 /
SNP Chromosome Position: 155359733 / SNP in Genomic Sequence: SEQ ID NO: 377 /
SNP Position Genomic: 214206 / Related Interrogated SNP: hCV11503414 /
Related Interrogated SNP: hCV11503469 / SNP Source: CDX /
Population(Allele,Count): no_pop (A,-|G,-) / SNP Type: //

Gene Number: 41 / Gene Symbol: GPATCH4 - 54865 / Gene Name: G patch domain
containing 4 / Chromosome: 1 / OMIM NUMBER: / OMIM Information: // Genomic
Sequence (SEQ ID NO: 378): // / SNP Information /
Context (SEQ ID NO: 1717): /
CTGACTCCAGCTCTTTCCTTCTTGGGATGCTGCTGCCTCTTCCTCTTACCTCTGCTGCTTGAGCCAGTGTGTGCTCTGCTCTC
TGCTTCCCTAGCCCCGC
Y
ATCCTTCTCATCCCTTACATCCAAGATGTCCTCCTCCTCTTGATGCTGCTGTCTTTTCTTCTTGCTTTTCCTGCTTCTTCCAT
CACTGCATGCTCTGCTC / Celera SNP ID: hCV9102827 / Public SNP ID: rs3795733 /
SNP Chromosome Position: 156564922 / SNP in Genomic Sequence: SEQ ID NO: 378 /
SNP Position Genomic: 10643 / SNP Source: Applera /
Population(Allele,Count): Caucasian (C,10|T,30) African American (C,26|T,10)
total (C,36|T,40) // / SNP Type: MICRORNA;UTR3 / SNP Source: dbSNP;
Celera; HapMap; HGBASE / Population(Allele,Count): Caucasian (C,61|T,165) / SNP

Type:    MICRORNA;UTR3 /
Context              (SEQ ID NO: 1718): /
TGGGGAGCTGCCTATCTTCAGGCAATAGATGGGTAGGTAATAGGAATCATTTCCCTGGGGTAAAAAGAACCAAGAGGTGGCAC
AGGCCATGGTCAAAGGT
S
GGGAGAAAAGGGAGCTCAAGGAGCTGACCACTACTTACTTGTGTGCTGTTCGCCCCTCACAGGCTTGGAGCAGCATCTCATCA
GTCAGACTGTTAGGGGA / Celera SNP ID:   hCV9102823 / Public SNP ID:   rs12024215 /
SNP Chromosome Position:   156565834 / SNP in Genomic Sequence:   SEQ ID NO: 378 /
 SNP Position Genomic:   11555 / Related Interrogated SNP:   hCV9102827 / SNP
Source:   Applera / Population(Allele,Count):   Caucasian (C,9|G,29) African
American (C,6|G,30) total (C,15|G,59) / SNP Type:   UTR3;INTRON / SNP Source:
dbSNP; Celera; HapMap / Population(Allele,Count):   Caucasian (G,92|C,28) / SNP
Type:   UTR3;INTRON /
Context              (SEQ ID NO: 1719): /
TTCCAGTCCTGTTTGCCATTACTAGCCAGCTGTGGGCTCTTCAGCAAACCACTTTTCACCCTTTTAGCCACATCTGCAAAGCA
GGGATGGTCCAGATAAT
Y
TCTCAGGTCTCCTCTGGCTTCTAAGTCCTTTGAATTAGATGTCCCCTGGCCCCCCCAAAATTTGGCAGCTCCTGACTTAAGAG
ACTGTACCCTACCTCCC / Celera SNP ID:   hCV9102814 / Public SNP ID:   rs879461 /
SNP Chromosome Position:   156567496 / SNP in Genomic Sequence:   SEQ ID NO: 378 /
 SNP Position Genomic:   13217 / Related Interrogated SNP:   hCV9102827 / SNP
Source:   dbSNP; Celera; HapMap; HGBASE / Population(Allele,Count):   Caucasian
(C,56|T,170) / SNP Type:   MICRORNA;UTR5;UTR3;INTRON /
Context              (SEQ ID NO: 1720): /
TCCTTGAGGAGCCACAGGGAGGTGTGCCATCCTGCATACTCGAACTTGGATAGGAGATGGGCTGCTGCTGCTGGGATGTGCAG
CAGCTTTGGCCTCTCTC
R
CCCACAGCTGGCTGGTGAGCAGAACAAGAAGAGGAAATGCTCTGGTTCATCAGGTCTTCACAGTGGACAAGTGCCAGTGACTG
ACAGTAAAGATGCCCCG / Celera SNP ID:   hCV9102822 / Public SNP ID:   rs4661052 /
SNP Chromosome Position:   156566491 / SNP in Genomic Sequence:   SEQ ID NO: 378 /
 SNP Position Genomic:   12212 / Related Interrogated SNP:   hCV9102827 / SNP
Source:   dbSNP; Celera; HapMap; HGBASE / Population(Allele,Count):   Caucasian
(A,170|G,56) / SNP Type:   UTR5;UTR3;INTRON /
Context              (SEQ ID NO: 1721): /
CACCTGAGTTCATGGTCCTGGGTTAGAAGCTGCAGAAAGGACTGTGCTGACTCCCTCAACTAAGGGGTGGTACTACCGCTTGC
TCCTCTCAGCAGCTGAC
R
TCCCAGAGATCAGTTTCCTTTCTGGGAGGGACTCCTCATTGAGGGGGGTGCAGAAGACCTGCAGACCTGCATCAACCCTGGTC
TTTGAGGCTTCAACTCC / Celera SNP ID:   hCV9102829 / Public SNP ID:   rs3795732 /
SNP Chromosome Position:   156564640 / SNP in Genomic Sequence:   SEQ ID NO: 378 /
 SNP Position Genomic:   10361 / Related Interrogated SNP:   hCV9102827 / SNP
Source:   dbSNP; Celera; HapMap; ABI_Val; HGBASE / Population(Allele,Count):
Caucasian (G,56|A,168) / SNP Type:   TRANSCRIPTION FACTOR BINDING
SITE;MICRORNA;UTR3 /
Context              (SEQ ID NO: 1722): /
AAAAACAAAAAAAAAACCTTCCCTTTACCTGTGTCTTAGCTTGGGCTATCATAATAAAATATCATAGATTGGATGCTCAAGCAA
CAAATTAATTTCCTGAC
R
GTTATGGGGACTGGAAGTCAGATTAGGGGTTCCAGTATGGTGACAGAGAGGAGGGGCAGTGAGAGGAAGAAAGTCTTATAAGGG
CACTAATTCTACCAGTT / Celera SNP ID:   hCV9102841 / Public SNP ID:   rs4661188 /
SNP Chromosome Position:   156560502 / SNP in Genomic Sequence:   SEQ ID NO: 378 /
 SNP Position Genomic:   6223 / Related Interrogated SNP:   hCV9102827 / SNP
Source:   dbSNP; Celera; HapMap; HGBASE / Population(Allele,Count):   Caucasian
(A,56|G,170) / SNP Type:   INTERGENIC;UNKNOWN /
Context              (SEQ ID NO: 1723): /
GCAATGCTTACCTATTCGAATATTATTCACTTTTCTTTCTGTTGAGGAAAAAAAAAAGGTGAGCCTGTGTCTCACAGGATTCA
ATCCTTAGCCTAGGCAG
S
TTCTAATTTTCTGACTTCATTAACCCTTTGAGTTCATGATAATAAAGATATTATGATCCCTTGCCAACAGTACTTTACTATTT
AAAATAATTTTCACACG / Celera SNP ID:   hCV11258640 / Public SNP ID:   rs6427323 /
SNP Chromosome Position:   156577789 / SNP in Genomic Sequence:   SEQ ID NO: 378 /
 SNP Position Genomic:   23510 / Related Interrogated SNP:   hCV9102827 / SNP
Source:   dbSNP; Celera; HapMap; ABI_Val / Population(Allele,Count):   Caucasian

(G,92|C,26) / SNP Type: INTERGENIC;UNKNOWN /
Context (SEQ ID NO: 1724): /
AGGGAAAGAAGATACAGCAAAAGGGACTGCACATGCCCCTACCCCTGCAAGCTGTTCCTTGGCCTCACTGAGCCACCCAAGAA
CTAAAACCCGAGAAAAG
Y
ATCCTTTTCTGCCTCCCCATATCGGTATTTCCGTTGACTTAGCGCCCCCTAGAAACTTCCAAACAGAAAAATCGCCATTTCAC
AGGAGAGCCTTGTCATA / Celera SNP ID: hCV31431620 / Public SNP ID: rs12023410 /
SNP Chromosome Position: 156572658 / SNP in Genomic Sequence: SEQ ID NO: 378 /
 SNP Position Genomic: 18379 / Related Interrogated SNP: hCV9102827 / SNP
Source: dbSNP; HapMap / Population(Allele,Count): Caucasian (T,173|C,47) /
SNP Type: INTERGENIC;UNKNOWN /
Context (SEQ ID NO: 1725): /
GTGTGCCAGAACTTGTCCCTTTGGTACTGGGGGATAAAAAGACGCATAAGACACAGGTCTTGATTTAGGGGAACACCCAGTCT
AGCCTGGGAAACTTGGG
R
CCTTGAGTGGGATTAGATGGAGGACATGCCAGTGGGACTTGGAGAAAGATACAGGATGGGTGAGACGCGGTGGCTCATGCCTG
TAATCCCAGCACTTTGG / Celera SNP ID: hCV31431621 / Public SNP ID: rs11576266 /
SNP Chromosome Position: 156572159 / SNP in Genomic Sequence: SEQ ID NO: 378 /
 SNP Position Genomic: 17880 / Related Interrogated SNP: hCV9102827 / SNP
Source: dbSNP; HapMap / Population(Allele,Count): Caucasian (A,61|G,165) /
SNP Type: INTERGENIC;UNKNOWN //

Gene Number: 42 / Gene Symbol: CHFR - 55743 / Gene Name: checkpoint with
forkhead and ring finger domains / Chromosome: 12 / OMIM NUMBER: / OMIM
Information: // Genomic Sequence (SEQ ID NO: 379): // / SNP Information /
Context (SEQ ID NO: 1726): /
TGAAACCTGACCCCTGAAGGACAGATAGGAACCCGCCTGCGTGCGGTGGCGCGGGCACTCACACTGCTGAGGGGCGACACGCG
GGTCCTGCTCGCGCTCC
R
CTCTCCGGTCGGGCATGGGCTGGAAGCAGCAGGTGCACAGGGCGTGGCTTCCTTGCAGAGGGCACACGTAATCCTGGACTGCT
GAAGCACACGCACATTC / Celera SNP ID: hCV1859941 / Public SNP ID: rs2306541 /
SNP Chromosome Position: 133428242 / SNP in Genomic Sequence: SEQ ID NO: 379 /
 SNP Position Genomic: 21304 / Related Interrogated SNP: hCV1859855 / SNP
Source: Applera / Population(Allele,Count): Caucasian (A,4|G,20) African
American (A,9|G,27) total (A,13|G,47) / SNP Type: MISSENSE MUTATION;PSEUDOGENE
/ SNP Source: dbSNP; Celera; HapMap; ABI_Val; HGBASE /
Population(Allele,Count): Caucasian (G,151|A,75) / SNP Type: MISSENSE
MUTATION;PSEUDOGENE /
Context (SEQ ID NO: 1727): /
AACTGTAGTTTCGGAAAATGTACAAAAGAGCAAAACTGCCCCTCTCGGCGGGACGGCCGCATGTTACAGAAAGGCTTCGTCTC
TGCTGCTGATGCCACCA
Y
GAGCCCTGCCCAGCGCTCACCAGGAGGGCGGGCTGCGGCCCCCGGGGCTCTGGGGAGGGTCTCACTCAGAGGGTAAAGCTCCA
CAGAAGAGTCACCCCAG / Celera SNP ID: hCV25761477 / Public SNP ID: rs3741490 /
SNP Chromosome Position: 133417908 / SNP in Genomic Sequence: SEQ ID NO: 379 /
 SNP Position Genomic: 10970 / Related Interrogated SNP: hCV1859855 / SNP
Source: Applera / Population(Allele,Count): Caucasian (C,2|T,2) African
American (C,20|T,0) total (C,22|T,2) / SNP Type: TRANSCRIPTION FACTOR BINDING
SITE;MICRORNA;UTR3;PSEUDOGENE / SNP Source: dbSNP; Celera; HGBASE /
Population(Allele,Count): Caucasian (C,151|T,75) / SNP Type: TRANSCRIPTION
FACTOR BINDING SITE;MICRORNA;UTR3;PSEUDOGENE /
Context (SEQ ID NO: 1728): /
AGGCTGGGATTGTCTTGGCCTGGTGATGGTGCCTCTTCTGTAATAACGTCCTGACAAATGATAAACATTGCAATTTATACTAT
AGGACTTTATAGTCGCT
W
GATCAACTACAAAGAATACATGTGCTCATGTGGGAAAAAATAACATGGAATGATGTCCCTCTGTCACAGCCAGAGATGCCACA
CCCATGACGAGTCGAAG / Celera SNP ID: hCV1859948 / Public SNP ID: rs7297261 /
SNP Chromosome Position: 133432653 / SNP in Genomic Sequence: SEQ ID NO: 379 /
 SNP Position Genomic: 25715 / Related Interrogated SNP: hCV1859855 / SNP
Source: dbSNP; Celera / Population(Allele,Count): Caucasian (A,80|T,40) / SNP
Type: INTRON /
Context (SEQ ID NO: 1729): /
TCCTGTCACAGTGCCCCAGTGGCTGGCTGCTCTCTGGACTGCTATACAGCAATGCTTCTACCATAACATTCCCAAATGTTTTC

TAATAATCTTAACTTTG
R
CTTTCAAAGTGCAGACTTCTCCCTAAATCATCTTCAATGTGAAGATTTAAGCTACATTTTTAGAAATTATTGTCCTACCAGGA
CAGAGGGCTTTCTTTTT / Celera SNP ID: hCV1859956 / Public SNP ID: rs7969859 /
SNP Chromosome Position: 133437240 / SNP in Genomic Sequence: SEQ ID NO: 379 /
SNP Position Genomic: 30302 / Related Interrogated SNP: hCV1859855 / SNP
Source: dbSNP; Celera; HapMap / Population(Allele,Count): Caucasian
(G,149|A,75) / SNP Type: TRANSCRIPTION FACTOR BINDING SITE;INTRON /
Context (SEQ ID NO: 1730): /
ACAGGCGGAAGCCAAGGTGCCCCTCAGGCTCTGTAGGGTCTTGGAGGAAGGGCCCGGGCAGCATGAGGGAGCGGCGCGTCCTG
GGACCTGCCTCCAGCCC
Y
GGGCTTGGGGCCGTGGTCACTCACACAAGGGAGCAGCACGTCCTGGGACCTGCGTCCAGCCCCAGGCTCGGGGCCGCGGTCAC
TCACACAAGGGAGCAGC / Celera SNP ID: hCV12052839 / Public SNP ID: rs8021 / SNP
Chromosome Position: 133417285 / SNP in Genomic Sequence: SEQ ID NO: 379 /
SNP Position Genomic: 10347 / Related Interrogated SNP: hCV1859855 / SNP
Source: dbSNP; Celera; HapMap; ABI_Val; HGBASE / Population(Allele,Count):
Caucasian (T,78|C,40) / SNP Type: MICRORNA;UTR3;PSEUDOGENE /
Context (SEQ ID NO: 1731): /
ACTCTGGAAATACTCATAGGAACAGTGTAAATACGTAACGATGCACACATCCACGCACCCCTCACCATTCTCTCAAGAAATGG
CTCGCCCACTTCTCCCT
R
ACTGACAGCCTAACCAGCCACTACACCTCCTCACGTGCTTGGCAGCTCTGCTGCTGCAGGGCTGGGTTCTCTCCCCGTTTGCC
TCCTAGTGGACCCGGAG / Celera SNP ID: hCV31631014 / Public SNP ID: rs12811327 /
SNP Chromosome Position: 133449177 / SNP in Genomic Sequence: SEQ ID NO: 379 /
SNP Position Genomic: 42239 / Related Interrogated SNP: hCV1859855 / SNP
Source: dbSNP; HapMap / Population(Allele,Count): Caucasian (G,78|A,36) / SNP
Type: INTRON //

Gene Number: 43 / Gene Symbol: SPATA7 - 55812 / Gene Name: spermatogenesis
associated 7 / Chromosome: 14 / OMIM NUMBER: / OMIM Information: // Genomic
Sequence (SEQ ID NO: 380): // / SNP Information /
Context (SEQ ID NO: 1732): /
CTCAGCCCTTACCACTTTATTGGATGCCAATCCCAACTTCAACAGCCAGTGTGTGCACCTTACTGCTTACAGCTTTCTCTGAA
CATGGAAACTTACTTAC
W
TGCCAATAGGGCAAGTCAGAAGGCCAGGGAATTAGCCACCTAAGGCCCTTACAGCCCTTAGACAAGTACAGTTGTTAGATGGT
GGATCAATGCCCTAGCT / Celera SNP ID: hCV211940 / Public SNP ID: rs12587386 /
SNP Chromosome Position: 88827764 / SNP in Genomic Sequence: SEQ ID NO: 380 /
SNP Position Genomic: 34788 / Related Interrogated SNP: hCV16182835 / SNP
Source: dbSNP; Celera; HapMap / Population(Allele,Count): Caucasian
(A,91|T,29) / SNP Type: INTERGENIC;UNKNOWN /
Context (SEQ ID NO: 1733): /
GTCAATAAAAGTAGTTTCTAGGAGACTCCATTTTCATAGTCTAGTTTGAACACTACAAAAAGTCTGACTGTGATTGGCTAGGA
TTATGTATAACTGTAAG
K
AATGGAACATTTAGTTCTCTCTTCTGAAAGTCTGAAGATAGTAGCCCTTCTAGGTTGGTATGAGCTTCCATAGTATCAGGGAC
TCAGATTCTGATGTAGT / Celera SNP ID: hCV342703 / Public SNP ID: rs12433464 /
SNP Chromosome Position: 88824015 / SNP in Genomic Sequence: SEQ ID NO: 380 /
SNP Position Genomic: 31039 / Related Interrogated SNP: hCV16182835 / SNP
Source: dbSNP; Celera; HapMap / Population(Allele,Count): Caucasian
(T,167|G,59) / SNP Type: INTERGENIC;UNKNOWN /
Context (SEQ ID NO: 1734): /
TTTTAAAGGTTAGGAAACTGAGACTCAGAGATGTATCTATTCAAACTCATACAACCACTTAATACTGTAAAAGCCAAGGATTT
TGATCTCATAATTCTTT
R
CTGTGTAATGTGTCCATGCAATTGCCAAACCTTTAGCATTATTAGAACAATTATTCTGGATAATCTCAAGAGCAGAGAATATA
ATGGGGAAAAGGCAACC / Celera SNP ID: hCV342704 / Public SNP ID: rs1955598 /
SNP Chromosome Position: 88823655 / SNP in Genomic Sequence: SEQ ID NO: 380 /
SNP Position Genomic: 30679 / Related Interrogated SNP: hCV16182835 / SNP
Source: dbSNP; Celera; HapMap; ABI_Val; HGBASE / Population(Allele,Count):
Caucasian (A,88|G,134) / SNP Type: INTERGENIC;UNKNOWN /
Context (SEQ ID NO: 1735): /

CATAGGCTTGTATTATGCACAGAGTCTCTGGAAGGATGCATTTAAACAGTAACATAGTATATTTCTGGGAAGGGAGACTAAAG
GTCAGGGTAGAGAAATC
R
CTTCTTTTTCAAACTCCATATGCCATTTTAGCTGTTGAATTTTTTTTAACCACATTGCGTGTATTACTTCTCAAAAACTGTTTT
TAATGTTTAAAAAAGCT / Celera SNP ID: hCV2231821 / Public SNP ID: rs453112 /
SNP Chromosome Position: 88859001 / SNP in Genomic Sequence: SEQ ID NO: 380 /
SNP Position Genomic: 66025 / Related Interrogated SNP: hCV16182835 / SNP
Source: dbSNP; Celera; HapMap; HGBASE / Population(Allele,Count): Caucasian
(A,38|G,82) / SNP Type: INTRON /
Context (SEQ ID NO: 1736): /
CTTTTAAAAATACCAGGCTTTTCATAGAAATAAAGGACTGGAAGCCACATTCATTGCAATTTAACTCTTTTATTTTAAAGGTT
AGGAAACTGAGACTCAG
M
GATGTATCTATTCAAACTCATACAACCACTTAATACTGTAAAAGCCAAGGATTTTGATCTCATAATTCTTTACTGTGTAATGT
GTCCATGCAATTGCCAA / Celera SNP ID: hCV2485030 / Public SNP ID: rs12589467 /
SNP Chromosome Position: 88823583 / SNP in Genomic Sequence: SEQ ID NO: 380 /
SNP Position Genomic: 30607 / Related Interrogated SNP: hCV16182835 / SNP
Source: dbSNP; Celera; HapMap / Population(Allele,Count): Caucasian
(A,91|C,29) / SNP Type: INTERGENIC;UNKNOWN /
Context (SEQ ID NO: 1737): /
TACTTCTTTTTTCTATATTTAGTCATCCATATATCTTCTAGACTTTCTATGTGTCCTCCTTGTTATAGCTTTTAACAGCCCAC
CTGATAGCAAACTAGCC
R
TCTTACCCTCTTAAAACATCTCTGTTTGGGGGATAAAGCATTTATATTTTGCATATTAAATTACATTTAATGCTGTAACTCAG
ACTTCCTGTTTTGGAGA / Celera SNP ID: hCV2485038 / Public SNP ID: rs865285 /
SNP Chromosome Position: 88857482 / SNP in Genomic Sequence: SEQ ID NO: 380 /
SNP Position Genomic: 64506 / Related Interrogated SNP: hCV16182835 / SNP
Source: dbSNP; Celera; HapMap; ABI_Val; HGBASE / Population(Allele,Count):
Caucasian (A,82|G,144) / SNP Type: INTRON /
Context (SEQ ID NO: 1738): /
GATTTCCACATTTGGTATTTGTCATTTATAAATACTGACAATATTATTAAACTATTACCTTTCTCTTTCAGCTGCAGTAGACT
GCTCGGTTCCAGTAAGC
R
TGAGTACCAGCATAAAGTGTAAGTAATTTTTGGACATTATTACCTTTTTAAAAAAAAAATTAAGGTAAATATACATAACATAAA
ATTTACTGTCTTAACCA / Celera SNP ID: hCV2485039 / Public SNP ID: rs3179969 /
SNP Chromosome Position: 88862529 / SNP in Genomic Sequence: SEQ ID NO: 380 /
SNP Position Genomic: 69553 / Related Interrogated SNP: hCV16182835 / SNP
Source: dbSNP; Celera; HapMap; HGBASE / Population(Allele,Count): Caucasian
(G,149|A,77) / SNP Type: MISSENSE MUTATION;DONOR SPLICE SITE;UTR5;UTR3 /
Context (SEQ ID NO: 1739): /
TTTTCATTGGTAGCTTACATAAATCATGGCTTTTAAAAATTGATTTGTGCGTGGCAGTTTGTGAATAGTAGATGCACTTATAA
GAGCTTTTCAAAAATTC
W
TTAACTGTTAAAACTTCAGATTCTATTTTTTTTAAGTGGAAAAGATATTATCAGGATAGATGAACAGATTGGTTAAGCTTTTTG
TTTTGCTTTGTTTTTCA / Celera SNP ID: hCV7583060 / Public SNP ID: rs1028455 /
SNP Chromosome Position: 88829975 / SNP in Genomic Sequence: SEQ ID NO: 380 /
SNP Position Genomic: 36999 / Related Interrogated SNP: hCV16182835 / SNP
Source: dbSNP; Celera; HapMap; ABI_Val; HGBASE / Population(Allele,Count):
Caucasian (T,82|A,36) / SNP Type: INTERGENIC;UNKNOWN /
Context (SEQ ID NO: 1740): /
ATGGAAAGTTTGCAAAACCAGATTAGCTATTGCATAGCTTAAGGAAACTGTGTGTCCTGAATTTTGCTTGAACATTTATGTAT
CTAAGATTCATTGAGCA
Y
TGAGCTCTACTTTGTGTTATGTAACCTAGGACCCTATTATGTATACTACTTGCTTTCTCTGTCATAATAATATGCCAAGCATA
GACATTTTTAATAAATT / Celera SNP ID: hCV9595812 / Public SNP ID: rs845758 /
SNP Chromosome Position: 88900090 / SNP in Genomic Sequence: SEQ ID NO: 380 /
SNP Position Genomic: 107114 / Related Interrogated SNP: hCV16182835 / SNP
Source: dbSNP; HapMap; ABI_Val; HGBASE / Population(Allele,Count): Caucasian
(C,80|T,144) / SNP Type: INTRON /
Context (SEQ ID NO: 1741): /
AGTGTTATAAAGAGCCCCTGTCACCCAGTGAGGAGGGGACTGTGTTAGAGCAGCGTTGAAAATAGTGGTTGGGAAAAATAAGT
GTTCTTTGTTTTTACCC
R

CAAATGAATTGAGACTCCTTCCTCAATGAACCAAATTTGTCCTTTAGTCAAAATCACACCAGTGAAGCAGCCTATGCCAATTT
GAACAGATCCTTGTCTA / Celera SNP ID: hCV11666737 / Public SNP ID: rs1955600 /
SNP Chromosome Position: 88825384 / SNP in Genomic Sequence: SEQ ID NO: 380 /
SNP Position Genomic: 32408 / Related Interrogated SNP: hCV16182835 / SNP
Source: dbSNP; HapMap; ABI_Val; HGBASE / Population(Allele,Count): Caucasian
(G,89|A,29) / SNP Type: INTERGENIC;UNKNOWN /
Context (SEQ ID NO: 1742): /
TACAGCTATTTAGACTGTGAAGAATAATCTGTTGGTGGCACTTATCATACATGATATAGCCAGTATACAGAAGGTCTGCCTAT
TTACAAATGGTTATAGC
K

CTCTCTGAATGGCCAGGTGGATCCTGCCAAATCTGGGAAGTCATTTGTTACTAACATCTTTCCTAGATGATTGGTTGGCTGGC
TGGACAAATGGAAGGAT / Celera SNP ID: hCV16189259 / Public SNP ID: rs2295135 /
SNP Chromosome Position: 88894210 / SNP in Genomic Sequence: SEQ ID NO: 380 /
SNP Position Genomic: 101234 / Related Interrogated SNP: hCV16182835 / SNP
Source: dbSNP; HapMap; ABI_Val; HGBASE / Population(Allele,Count): Caucasian
(T,167|G,59) / SNP Type: UTR3;INTRON /
Context (SEQ ID NO: 1743): /
TTACCCAAAAGTCATTCAGGAGCAAGTTGGTTTAATTTCCATGTAAAATTATGTGGTTTTGAGGAGTACTCTTGGTATTGATT
TCTACCTTTTTTTCCAC
Y

GTGGTCCAAAAATATGTTTGGCGTGGTTTCAATTTTTTTTACATTTATTGAAACTTGCTTTGTCTTCGAGCATGTGGTTGATCT
TAGAGTATGTTCCACAT / Celera SNP ID: hCV27202496 / Public SNP ID: rs1099698 /
SNP Chromosome Position: 88881248 / SNP in Genomic Sequence: SEQ ID NO: 380 /
SNP Position Genomic: 88272 / Related Interrogated SNP: hCV16182835 / SNP
Source: dbSNP; HapMap; HGBASE / Population(Allele,Count): Caucasian
(C,32|T,80) / SNP Type: INTRON /
Context (SEQ ID NO: 1744): /
CAAGTTGGTTTAATTTCCATGTAAAATTATGTGGTTTTGAGGAGTACTCTTGGTATTGATTTCTACCTTTTTTTTCCACCGTGG
TCCAAAAATATGTTTGG
Y

GTGGTTTCAATTTTTTTTACATTTATTGAAACTTGCTTTGTCTTCGAGCATGTGGTTGATCTTAGAGTATGTTCCACATGCAGA
TGAGAAGAATGTTTATT / Celera SNP ID: hCV27202497 / Public SNP ID: rs12589480 /
SNP Chromosome Position: 88881270 / SNP in Genomic Sequence: SEQ ID NO: 380 /
SNP Position Genomic: 88294 / Related Interrogated SNP: hCV16182835 / SNP
Source: dbSNP; Celera; HapMap / Population(Allele,Count): Caucasian
(C,85|T,29) / SNP Type: INTRON /
Context (SEQ ID NO: 1745): /
TCTGTGTCCCCACCCAAATCTCATCTTGAATTGTAACCCAAATTGTAATCCCCACGTGTTGGGGAAGGGACCTCGTGGGAGGT
GACTGACTTGACCAGGG
M

AGTTCCTTTGTGCTGTTCTCATGATAGTGAGATCTCGTGAGATCTGATGGTTTTGTAATAGGCCTTTCCCCCCCTTCACTCTGC
ACTTCTCTAATTCTTCT / Celera SNP ID: hCV32095372 / Public SNP ID: rs12586714 /
SNP Chromosome Position: 88826841 / SNP in Genomic Sequence: SEQ ID NO: 380 /
SNP Position Genomic: 33865 / Related Interrogated SNP: hCV16182835 / SNP
Source: dbSNP; HapMap / Population(Allele,Count): Caucasian (C,80|A,140) /
SNP Type: INTERGENIC;UNKNOWN /
Context (SEQ ID NO: 1746): /
TGGATTTTATACTGTAGTCTACTGGTTTCCAAGGAAAGAAAAAATAGGATACAAGGTAGAAATTATAAGATACAAAAATGGGA
AAAGTTTCATGCTTTCT
R

TAAGTTCAGAAGCAATTTTTTCCATCCCATTTCAGAGAGATAGCTTAACATATACTTCTACCTTTTAAAAAGACTTTCTTCAGT
GTTTCATTTTGTTTATT / Celera SNP ID: hDV70886228 / Public SNP ID: rs17124652 /
SNP Chromosome Position: 88890728 / SNP in Genomic Sequence: SEQ ID NO: 380 /
SNP Position Genomic: 97752 / Related Interrogated SNP: hCV16182835 / SNP
Source: dbSNP; HapMap / Population(Allele,Count): Caucasian (A,91|G,29) / SNP
Type: INTRON /
Context (SEQ ID NO: 1747): /
GACCTTGTCTGCAATATCTTTAATTTTGTTTTTCTCACTTTTCCTGCAATATCTAAAGCCTCAAGGACCTTTCAGTTGTCAGT
GACCCAGGGAATTCTGT
Y

AGCTGGCTGAGATGTTGTCTCTTGTTGTGAGTGCAAACATTTAGTCAGACCTGTCTGAAGCCCTCTCCTACCCTTGTAGCTCA
TGAATGAGACCCTTACT / Celera SNP ID: hDV70886264 / Public SNP ID: rs17124700 /
SNP Chromosome Position: 88905629 / SNP in Genomic Sequence: SEQ ID NO: 380 /

SNP Position Genomic: 112653 / Related Interrogated SNP: hCV16182835 / SNP Source: dbSNP; HapMap / Population(Allele,Count): Caucasian (C,91|T,29) / SNP Type: INTRON /
Context (SEQ ID NO: 1748): /
TTCTTAACAAGCAATGAGTTCTGGCCACCAACTCCTTTAGGTCATGGGTTATCACTTGGTGTTTATATGCAATGTGCAGCCTG
GGGGTGTGTTTAATTAT
S
CCTGTTACTGAAAAGGCATAGTACCTGGGCAGTGAATACACCTGGTAACAATACTATTAGCACTTATTGTCCTGATACAATTA
TCTCAACTCCCTACCCA / Celera SNP ID: hDV70991668 / Public SNP ID: rs17698817 /
SNP Chromosome Position: 88822976 / SNP in Genomic Sequence: SEQ ID NO: 380 /
SNP Position Genomic: 30000 / Related Interrogated SNP: hCV16182835 / SNP Source: dbSNP / Population(Allele,Count): Caucasian (G,159|C,61) / SNP Type: INTERGENIC;UNKNOWN /
Context (SEQ ID NO: 1749): /
GAAAACTCCTGACCTGGTCTCGAACTCCTGACCTCAGGTGATCTGCCCGCCTCGACCTCCCAAAGTGCTGGGATTATAGGCCT
GACCATGCCCCACCGGC
Y
GAGATTCTTAACAAGCAATGAGTTCTGGCCACCAACTCCTTTAGGTCATGGGTTATCACTTGGTGTTTATATGCAATGTGCAG
CCTGGGGGGTGTGTTTAA / Celera SNP ID: hDV71004484 / Public SNP ID: rs17772064 /
SNP Chromosome Position: 88822871 / SNP in Genomic Sequence: SEQ ID NO: 380 /
SNP Position Genomic: 29895 / Related Interrogated SNP: hCV16182835 / SNP Source: dbSNP / Population(Allele,Count): Caucasian (C,164|T,58) / SNP Type: INTERGENIC;UNKNOWN /
Context (SEQ ID NO: 1750): /
CTTTCTCCTTGATGTAATTAGGGTTGAAAGAATTGCAAAGTGAATGACTTTCTTATGGGGTAATTCAAAGTTTTTTAATGCTG
TGACTGAGCAATAATGT
R
TGAAATTAAAAAACCTCACCTGAGAGGAAGCATGTTCTGCTATATAATGATGCCAGCAGTACATACCTGCCTCCAACATCTTG
CTAAGCAGCTTTTATAA / Celera SNP ID: hDV71004511 / Public SNP ID: rs17772222 /
SNP Chromosome Position: 88826482 / SNP in Genomic Sequence: SEQ ID NO: 380 /
SNP Position Genomic: 33506 / Related Interrogated SNP: hCV16182835 / SNP Source: dbSNP; HapMap / Population(Allele,Count): Caucasian (A,164|G,58) /
SNP Type: INTERGENIC;UNKNOWN /
Context (SEQ ID NO: 1751): /
CTAGCAGCTATGTCCCAGGGAGGTTGCCGATAGGATGGTGTGCTGACCAGGGCCCCACACCCACCTGGAATGAATGCCTGAAG
CCAGCTTGCCTGGGAGT
R
TTCTCCTGGATCCCATTCCCATTTCCTGTGATAATAGAGAGCTAGAAGAAAAGAAGGGACTGGGAAGGAGGGAAGATCAAGAC
CTAGGATGAATTTCCCA / Celera SNP ID: hDV71004521 / Public SNP ID: rs17772288 /
SNP Chromosome Position: 88827366 / SNP in Genomic Sequence: SEQ ID NO: 380 /
SNP Position Genomic: 34390 / Related Interrogated SNP: hCV16182835 / SNP Source: dbSNP; HapMap / Population(Allele,Count): Caucasian (G,90|A,28) / SNP Type: INTERGENIC;UNKNOWN //

Gene Number: 44 / Gene Symbol: ACSS2 - 55902 / Gene Name: acyl-CoA synthetase short-chain family member 2 / Chromosome: 20 / OMIM NUMBER: / OMIM Information: // Genomic Sequence (SEQ ID NO: 381): // / SNP Information /
Context (SEQ ID NO: 1752): /
AGGATTGCAGGAGCTACACCAAAGAATGGGAAAGTCTAGAAGCAAACGGGAGACAGAACCCAAGAAGAGGAATGAGGCAACTC
TGACTCAACTCCCAAGG
R
GCCCCAGAACCCCAAATTCTTCACACATGTCAGTCCAGTTCTCCACTATCTAACTAGGCCAAGTGCCCCAAGACACCATCCCT
GCCTAGCCCAAGACCAG / Celera SNP ID: hCV11189332 / Public SNP ID: rs2273683 /
SNP Chromosome Position: 33509523 / SNP in Genomic Sequence: SEQ ID NO: 381 /
SNP Position Genomic: 56757 / Related Interrogated SNP: hCV1825046 / SNP Source: dbSNP; Celera; HapMap; HGBASE / Population(Allele,Count): Caucasian (G,83|A,141) / SNP Type: UTR3;INTRON;PSEUDOGENE /
Context (SEQ ID NO: 1753): /
ACCCATCAACCCTGAGGCCTGGCTATGGTACCACCGGGTGGTAGGTGCCCAGCGCTGCCCCATCGTGGACACCTTCTGGCAAA
CAGAGACAGTGAGTGAA
R
GGTACAGAAGGCTGGGGCCCAGGGACAATGTGGGAAGATTGACTCAAATTTCTCATCTCTGACTTCCCCAGGGTGGCCACATG
TTGACTCCCCTTCCTGG / Celera SNP ID: hCV1361222 / Public SNP ID: rs3818273 /

SNP Chromosome Position: 33509275 / SNP in Genomic Sequence: SEQ ID NO: 381 / SNP Position Genomic: 56509 / Related Interrogated SNP: hCV1825046 / SNP Source: dbSNP; Celera; HapMap; HGBASE / Population(Allele,Count): Caucasian (G,85|A,141) / SNP Type: UTR3;INTRON / Context (SEQ ID NO: 1754): /
AAAAAATGGTCAGCAGTATGCTGGAACTGGCTTATATCAACCAGTGAGAGCTGATTGTTGTTAAACATTTGCCAGCACATCTC TGGAAACAGTGTAAATG
K
CTGAAGCTCTGTGGATTCCTGAGGATTCAAATTCTAGAAGACTGTGTAGAGTGATAGGTAGCAAAAGGCTTGAGGGAGGATAG GAGGTTCTAGCAGCAAG / Celera SNP ID: hCV2142575 / Public SNP ID: rs2236270 / SNP Chromosome Position: 33523155 / SNP in Genomic Sequence: SEQ ID NO: 381 / SNP Position Genomic: 70389 / Related Interrogated SNP: hCV1825046 / SNP Source: Applera / Population(Allele,Count): Caucasian (G,6|T,4) African American (G,4|T,0) total (G,10|T,4) / SNP Type: TFBS SYNONYMOUS;INTRON / SNP Source: dbSNP; Celera; HapMap; HGBASE / Population(Allele,Count): Caucasian (G,133|T,93) / SNP Type: TFBS SYNONYMOUS;INTRON / Context (SEQ ID NO: 1755): /
ACCAGTAAAAAGCAACTTTATCTCCAAGCTTTTTCTCATGGACATTTCGATCCAGTACATTGTAGCAGATGTTGGTAGTTGCT CCTTTCATCCACTCAAT
R
AAGATTTTCCCTTTAGTCACATCAAAGTTGTACCGAAGGAATGGGCCAGGGCATGGAGTCTTCCAGTAAAATTCCTTGGCAAT GTCTCCCCAGAATTCTG / Celera SNP ID: hCV25750225 / Public SNP ID: rs4911163 / SNP Chromosome Position: 33470694 / SNP in Genomic Sequence: SEQ ID NO: 381 / SNP Position Genomic: 17928 / Related Interrogated SNP: hCV1825046 / SNP Source: Applera / Population(Allele,Count): Caucasian (G,12|A,22) African American (G,23|A,15) total (G,35|A,37) // / SNP Type: TRANSCRIPTION FACTOR BINDING SITE;UTR5;SILENT MUTATION;PSEUDOGENE / SNP Source: dbSNP; Celera; HapMap; HGBASE / Population(Allele,Count): Caucasian (G,85|A,141) / SNP Type: TRANSCRIPTION FACTOR BINDING SITE;UTR5;SILENT MUTATION;PSEUDOGENE / Context (SEQ ID NO: 1756): /
GGCCAATGGTGCCACCAGTGTTTTGGTGAGAAGGGAGCCACCTGGCCTAGCTGGGGGATGGACAAGATTATCCTGGGTGACTA CCTCCCAAGGCTGCTTG
K
TCTAGAGGGAGGGGGACTTATACAATCATCTGTTTTTCTGGAGAAAGAGAAGTCCTGAGGGGGTTGCGTATCCTGACTAGGATG GTATCTTGCTTGTCTG / Celera SNP ID: hCV1361223 / Public SNP ID: rs3746450 / SNP Chromosome Position: 33508588 / SNP in Genomic Sequence: SEQ ID NO: 381 / SNP Position Genomic: 55822 / Related Interrogated SNP: hCV1825046 / SNP Source: dbSNP; Celera; HapMap; HGBASE / Population(Allele,Count): Caucasian (G,42|T,78) / SNP Type: MISSENSE MUTATION;UTR3;INTRON / Context (SEQ ID NO: 1757): /
GATAGAGATAAATGCTGTTAAGAAAAATAAACAGGGTAGCATGATAGTGTGTGCAGGGATGGCTACTGTACACAGGATCGTCG GAGAAGACTTCTCTGAA
K
TGACATTTGAGCAGAGGCCTGAAGGGTGAGAAGGAAACAGGCATGGGAAAATCTAGAGTAAGAGTGTTTTGTGCAGAGGGACA CAGCTTGAGATGTCTGA / Celera SNP ID: hCV2142560 / Public SNP ID: rs4911449 / SNP Chromosome Position: 33512236 / SNP in Genomic Sequence: SEQ ID NO: 381 / SNP Position Genomic: 59470 / Related Interrogated SNP: hCV1825046 / SNP Source: dbSNP; Celera; HapMap / Population(Allele,Count): Caucasian (T,85|G,141) / SNP Type: INTRON / Context (SEQ ID NO: 1758): /
TGTGCAGAGGGACACAGCTTGAGATGTCTGAAGAATGGACTGAAGCCTGTGTGGCTGGAGCGTAGCAAGCAAAGGGAAGAATA TTACATGACGTTGGAGA
Y
GTACATAATACAGGGAGGGTCTTGTGGGCCATGGTGAGGAGTTTGAATTTTATTCAAAGTACAGTGGGAAACCTTTTTTTTTGT TGTTGTTGTTGTTGTTG / Celera SNP ID: hCV2142561 / Public SNP ID: rs4911450 / SNP Chromosome Position: 33512406 / SNP in Genomic Sequence: SEQ ID NO: 381 / SNP Position Genomic: 59640 / Related Interrogated SNP: hCV1825046 / SNP Source: dbSNP; Celera; HapMap / Population(Allele,Count): Caucasian (C,42|T,78) / SNP Type: INTRON / Context (SEQ ID NO: 1759): /
CGTAGCAAGCAAAGGGAAGAATATTACATGACGTTGGAGACGTACATAATACAGGGAGGGTCTTGTGGGCCATGGTGAGGAGT TTGAATTTTATTCAAAG
K

ACAGTGGGAAACCTTTTTTTTTGTTGTTGTTGTTGTTGTTGTTTGTTTTTTTTTTGAGACAGAATCTCACTCTGTCACCCAGGC
TGGAGTGCGGTGGCACG / Celera SNP ID: hCV2142562 / Public SNP ID: rs4911451 /
SNP Chromosome Position: 33512466 / SNP in Genomic Sequence: SEQ ID NO: 381 /
SNP Position Genomic: 59700 / Related Interrogated SNP: hCV1825046 / SNP
Source: dbSNP; Celera; HapMap; HGBASE / Population(Allele,Count): Caucasian
(T,41|G,77) / SNP Type: INTRON /
Context (SEQ ID NO: 1760): /
TCTTATTGTTACCTTCATGCTAAAGATGATGAAACTGAGACTCATGGAAGTTAAATCACTTGCCCAAGGCCACACTGCTAGAA
AGGTGGCATTTAAACTC
Y

AGCAGGCTCATTCTACAGCCTGAGTTCCATAAGATAAAATGAGAGAGTAAAGAGACAAGTGTGTTGAGAGCAAGAGAAGCAGT
GAACCTGAGGCTGTCTC / Celera SNP ID: hCV2142566 / Public SNP ID: rs6088650 /
SNP Chromosome Position: 33514465 / SNP in Genomic Sequence: SEQ ID NO: 381 /
SNP Position Genomic: 61699 / Related Interrogated SNP: hCV1825046 / SNP
Source: dbSNP; Celera; HapMap / Population(Allele,Count): Caucasian
(T,85|C,141) / SNP Type: INTRON /
Context (SEQ ID NO: 1761): /
AAGCCAGTTCACAACTGTTATGTATGTCCCATCTTGGTTGCCTTCCCTCCTGGGCTCCTGCCAGGGACTCTCATTCCTCCCTG
TGATCCAGTAAGAGCAT
Y

AGTGGGTGGCTAAGGATTGAAACTTCAGGGTTCCACCTGTAGAGACTGGTCTAGGGGGACCCTGATTACAGACCAGTCACTTC
CCTGCTCTAATACATCT / Celera SNP ID: hCV2142567 / Public SNP ID: rs725521 /
SNP Chromosome Position: 33516071 / SNP in Genomic Sequence: SEQ ID NO: 381 /
SNP Position Genomic: 63305 / Related Interrogated SNP: hCV1825046 / SNP
Source: dbSNP; Celera; HapMap; ABI_Val; HGBASE / Population(Allele,Count):
Caucasian (T,85|C,141) / SNP Type: INTERGENIC;UNKNOWN /
Context (SEQ ID NO: 1762): /
ATACAAATGTTGGCTCGAAATTACCGAGCAGCAGAATCATACTGCCATGTGTAGTAAAACACACTTTATATTTCCACTGCTTA
AAGCAGCTGAAACCAGA
M

TGAAAAAATTTTTAATGGCAGGGGTGAAGGTGAAAACGCAAACATGTAAAATGATGAGTCTGATTAAGACACAGGCTTTTTAA
ATGTCTTTTCCTTTTCT / Celera SNP ID: hCV2142576 / Public SNP ID: rs2236271 /
SNP Chromosome Position: 33523840 / SNP in Genomic Sequence: SEQ ID NO: 381 /
SNP Position Genomic: 71074 / Related Interrogated SNP: hCV1825046 / SNP
Source: dbSNP; Celera; HGBASE / Population(Allele,Count): Caucasian
(A,85|C,141) / SNP Type: INTRON /
Context (SEQ ID NO: 1763): /
AAAACATGTCAGGCAGTTCCAATCTCTAGGTCAAGAGAGACAGGCATTCTCTGTTGAGCTGGAAGAGGAAACTAAGGCCCAGG
ACAGGGTAGGTGTGCCT
R

TTGTCGCTTGGTGAGTCAGGGCTGTATTAGGACCAGAAGTCTCACTCCTGACTACTGAATAAAAGGCCTAGCTTCAGGCTGAT
ATCCATCATGACACATC / Celera SNP ID: hCV3249260 / Public SNP ID: rs7263157 /
SNP Chromosome Position: 33459127 / SNP in Genomic Sequence: SEQ ID NO: 381 /
SNP Position Genomic: 6361 / Related Interrogated SNP: hCV1825046 / SNP
Source: dbSNP; Celera; HapMap / Population(Allele,Count): Caucasian
(G,85|A,141) / SNP Type: UTR3;INTRON /
Context (SEQ ID NO: 1764): /
TAATGGAAATTACGCGGTATAAGACTGAACGAGTTACTACGATGTCAAGTTGTTTTATTTATTTTTTTAACTTGGGCTGGGAT
TGTATTAGTAATAGTTC
M

GAAGTTCTGATAGATGGTTCCATGTCTGTTAAAATGTATGAGGAAAATCGGCCAGGAGCAGTGGCTCACGCCTGTAATCCCAG
CACTTTGGGAGGCCGAG / Celera SNP ID: hCV3249262 / Public SNP ID: rs6120750 /
SNP Chromosome Position: 33465289 / SNP in Genomic Sequence: SEQ ID NO: 381 /
SNP Position Genomic: 12523 / Related Interrogated SNP: hCV1825046 / SNP
Source: dbSNP; Celera / Population(Allele,Count): Caucasian (A,134|C,92) /
SNP Type: INTRON /
Context (SEQ ID NO: 1765): /
GTTGGGATTTGAACCAAGACAGTTTGACTGCAGAATCCATATCCTAATAGCAATGCTATTCCTCTTCCATGACCCCACAGAAT
GCATTTCACCAAATTCC
R

TCAAATACAGAATTTCTACCAGGGAAGCAGCCTCCTAATTTGTCCTCTCTTGCCTAGTTTCGCCAACTTGAGTCTTTCCAGTA
GATTTATCAAGACACCA / Celera SNP ID: hCV3249263 / Public SNP ID: rs6088635 /
SNP Chromosome Position: 33466501 / SNP in Genomic Sequence: SEQ ID NO: 381 /

SNP Position Genomic: 13735 / Related Interrogated SNP: hCV1825046 / SNP Source: dbSNP; Celera; HapMap; ABI_Val / Population(Allele,Count): Caucasian (A,85|G,141) / SNP Type: INTRON / Context (SEQ ID NO: 1766): / TATGCCTACTATTATCACTTGAAAATATTTTTGAGTATTTGTACTCTCGTTATTAGCTGCGTGGTCCTTGGCAGAGTTTCCTG TGCTTCCTGAGCTTCAG Y

TTTCTCATCTATAAAATTAGGATAATTAATGTATACTTTGTAAGACCATAATGTCATAAAGATTAAATGAGATTAGTCCAGTC CCTGATACATGAATGGC / Celera SNP ID: hCV3249264 / Public SNP ID: rs6087641 / SNP Chromosome Position: 33467717 / SNP in Genomic Sequence: SEQ ID NO: 381 / SNP Position Genomic: 14951 / Related Interrogated SNP: hCV1825046 / SNP Source: dbSNP; Celera; HapMap / Population(Allele,Count): Caucasian (T,85|C,141) / SNP Type: INTRON / Context (SEQ ID NO: 1767): / AAGCTAAGTGATTTGCCTAAGGGTCATACACCTGTTTGAAGACAGAACTGAGACTAATCTCCAGATACTCTCACTCCTAGTCA ACTATTTTTTTTCACTC R

TGACACTACCTCATTATTCTAGTAGTCCAGCACTTGGCTGTGACAGATAAAAAGAGAAATTAGCCACATTAGCCAAACTGATT TAAATTAACTTTAAAAT / Celera SNP ID: hCV3249268 / Public SNP ID: rs6058137 / SNP Chromosome Position: 33475074 / SNP in Genomic Sequence: SEQ ID NO: 381 / SNP Position Genomic: 22308 / Related Interrogated SNP: hCV1825046 / SNP Source: dbSNP; Celera; HapMap; ABI_Val / Population(Allele,Count): Caucasian (G,85|A,141) / SNP Type: INTRON / Context (SEQ ID NO: 1768): / GGAGGTTATAGTCCAGCTTGAGGAGACAGACAATAAACATAGAAACCAGTAAGTATATGACAGGTAGTGATGAGTACTATAGA AAAATATAGAGCAGAGT S

AGGGGAAAGGGAAGTGACAGTGCAGAGTGGGAGCTGATAAGGAAAGAACCTTTTGATAAATATGACATTTGAGCAGAGATCTG AAGGAAAGTATGGAGTG / Celera SNP ID: hCV3249269 / Public SNP ID: rs8116657 / SNP Chromosome Position: 33476474 / SNP in Genomic Sequence: SEQ ID NO: 381 / SNP Position Genomic: 23708 / Related Interrogated SNP: hCV1825046 / SNP Source: dbSNP; Celera; HapMap / Population(Allele,Count): Caucasian (C,42|G,78) / SNP Type: INTRON / Context (SEQ ID NO: 1769): / TGCCTGAGGGTTGTTTGTGGCTGGTAGCATAAATACTCAAAGCTAATGGGAGATACATGGGAAATACCTGTTGTTGGGAACAG TCTTTCTCCACTTGAAT S

AGGTGAGCAGATTGTGGAGGTGAGCTGGAGGATTTCATGTTTAAGGTGGATTTCTAACCTGCTTCTCTCATTTCTCCCTGAGT TTTATGACCCTTGACAC / Celera SNP ID: hCV3249271 / Public SNP ID: rs4911164 / SNP Chromosome Position: 33479488 / SNP in Genomic Sequence: SEQ ID NO: 381 / SNP Position Genomic: 26722 / Related Interrogated SNP: hCV1825046 / SNP Source: dbSNP; Celera; HapMap; ABI_Val; HGBASE / Population(Allele,Count): Caucasian (G,42|C,78) / SNP Type: TRANSCRIPTION FACTOR BINDING SITE;INTRON / Context (SEQ ID NO: 1770): / AGTATTCCAGCTATGGTCTGACTTGAACAGGGTACTATTACCATTCTTTCACTTTACATATCTAAGATTATATTAGCCTCATT GACAGCCACACTCTCCA S

TCAGTTTATAATCTCTCATAATACTTAAGATTTTCCTTTTCTTTCTCTCTTTCTCTCTCCTTTTCCTTTTTCCTCTTTCTCCT TTCATTTCCTTCCCTTT / Celera SNP ID: hCV3249272 / Public SNP ID: rs6087644 / SNP Chromosome Position: 33480917 / SNP in Genomic Sequence: SEQ ID NO: 381 / SNP Position Genomic: 28151 / Related Interrogated SNP: hCV1825046 / SNP Source: dbSNP; Celera; HapMap / Population(Allele,Count): Caucasian (G,41|C,79) / SNP Type: INTRON / Context (SEQ ID NO: 1771): / CTGCAAATTTAGCTCATATAATTTATAGAAGACATCTACCATACAACCTGTTTTGCATAGCCAGTTATCACCCAACATAATCA GCCAAATCAGATCTACT K

TGTTAGAAATTGCCTAACTTCTTTCTTAAGATAAATATGTTAATATATGACTCTGTGATAACTGTTTCACTTCTGAGTTGTAA TTCTAAGAGATATAAGG / Celera SNP ID: hCV3249275 / Public SNP ID: rs6088642 / SNP Chromosome Position: 33483186 / SNP in Genomic Sequence: SEQ ID NO: 381 / SNP Position Genomic: 30420 / Related Interrogated SNP: hCV1825046 / SNP Source: dbSNP; Celera; HapMap / Population(Allele,Count): Caucasian (G,85|T,141) / SNP Type: TRANSCRIPTION FACTOR BINDING SITE;INTRON /

Context              (SEQ ID NO: 1772): /
TTTCCTTATGCTAATTCTGAATTTGGAAACAATACCCCCCTTTTGGAGGCCATGTCATTTAGGAGGAGAAGGCCCTGAACTAG
ACATTTAGACTCAAGAA
Y

TGTGCTATCCAACATGGAAGCCACTAGCTACATGTAACTATTGATATTTATAGTAATTAAAATTAAGTAAAATAAATAATCCA
GTTCTTCCGCCACATTT / Celera SNP ID:   hCV3249278 / Public SNP ID:   rs6120757 /
SNP Chromosome Position:   33488771 / SNP in Genomic Sequence:   SEQ ID NO: 381 /
SNP Position Genomic:   36005 / Related Interrogated SNP:   hCV1825046 / SNP
Source:   dbSNP; Celera; HapMap; ABI_Val / Population(Allele,Count):   Caucasian
(C,85|T,141) / SNP Type:   INTRON;PSEUDOGENE /
Context              (SEQ ID NO: 1773): /
GAGGCTGAGGTGGGAGAATCATTTGAGCCCGAGAGTTTAAGACCAGCCTGGGCAACATAGTGAGACATTGTCTCAAAAAAAAA
AAAAAAAAAAAAAAGTTG
K

GGGGTAGCACCAGTCTTCCCTGTCATTAGTCAATTACCAGTCTTTCAATTATTCAACCATGACCCTCGAATACCTATTGTGCC
AATTGTGCTTGGAGGAG / Celera SNP ID:   hCV3249279 / Public SNP ID:   rs926734 /
SNP Chromosome Position:   33491700 / SNP in Genomic Sequence:   SEQ ID NO: 381 /
SNP Position Genomic:   38934 / Related Interrogated SNP:   hCV1825046 / SNP
Source:   dbSNP; Celera; HGBASE / Population(Allele,Count):   Caucasian
(G,85|T,141) / SNP Type:   INTRON /
Context              (SEQ ID NO: 1774): /
AGATGTGAAGAGAGGTAGTCTAATTCTAAATGGATTTTGAATCAATAATGGGTTAAAGTAAATTTCGAGAGTGTGAGAATGAA
GTAGTTGGAGAAGACTC
Y

TTGGAGGAGGTTCCCTAGGGGAAGAAGATGAGAAGGACTAGTGACTGTGTACCATGGGTTGTCACACCAACCATATGGCCTCC
AGGTGAAGGAAAAGTAG / Celera SNP ID:   hCV3249280 / Public SNP ID:   rs6120758 /
SNP Chromosome Position:   33492523 / SNP in Genomic Sequence:   SEQ ID NO: 381 /
SNP Position Genomic:   39757 / Related Interrogated SNP:   hCV1825046 / SNP
Source:   dbSNP; Celera; HapMap / Population(Allele,Count):   Caucasian
(C,85|T,141) / SNP Type:   INTRON /
Context              (SEQ ID NO: 1775): /
AATTCAAACTTCTTACTATTGCCTACAAGGCTCTACGTGATTTGGCTGCTGCCTACCTCTCATTCTTACCCAACTTCTTGCTC
ACTGAAATCTAGCCATA
Y

TGTCTTTCTTTCTTTCTTTGAAAAGGTCTCAGTGCCTTTTCACTTGATGCTCCTTTTCCCCAGAATACTCTTCCCAAGAATTA
GTATGATTGCCTACCTC / Celera SNP ID:   hCV3249282 / Public SNP ID:   rs2064454 /
SNP Chromosome Position:   33496169 / SNP in Genomic Sequence:   SEQ ID NO: 381 /
SNP Position Genomic:   43403 / Related Interrogated SNP:   hCV1825046 / SNP
Source:   dbSNP; Celera; HapMap; HGBASE / Population(Allele,Count):   Caucasian
(C,85|T,141) / SNP Type:   TRANSCRIPTION FACTOR BINDING SITE;INTRON /
Context              (SEQ ID NO: 1776): /
GGCGTGAGCCACCGCGCCCAGCCCCACACTCTTAAGTTTAACTCCCAGTAGTCACTGAACTAACCTCTGTCTCTCCTGACATT
CATCAGTCTTCCTCATC
Y

CACCCCAACCTCTACCCTTGGGGCAATCCAAGGCTTGATGTTTACTAGATAATACTTTTTTTCTTTTGGTGTTTACCACATCCT
GTGAGTCTGTTAAGGCA / Celera SNP ID:   hCV3249286 / Public SNP ID:   rs6088646 /
SNP Chromosome Position:   33505937 / SNP in Genomic Sequence:   SEQ ID NO: 381 /
SNP Position Genomic:   53171 / Related Interrogated SNP:   hCV1825046 / SNP
Source:   dbSNP; Celera; HapMap; ABI_Val / Population(Allele,Count):   Caucasian
(T,85|C,141) / SNP Type:   INTRON /
Context              (SEQ ID NO: 1777): /
TTCCTGCTGAATGGAACCTGTCCCCTCAAGCAAAAGTCTCTATGGGAAACCCTATTGGTACCAGTGGTGTAAAGGCAGGAGCA
GAGCTAGGGTAGGAAAC
M

CGCAGATGTGGATTGGGTCTGGACATGTAGGCAAAAGTGCTTGGACCAAGATCTGTTGACTTATTTTACTACTAACAGGGTCA
GGTGCCCACCTCACCTG / Celera SNP ID:   hCV3249289 / Public SNP ID:   rs2223881 /
SNP Chromosome Position:   33506463 / SNP in Genomic Sequence:   SEQ ID NO: 381 /
SNP Position Genomic:   53697 / Related Interrogated SNP:   hCV1825046 / SNP
Source:   dbSNP; Celera; HapMap; HGBASE / Population(Allele,Count):   Caucasian
(A,42|C,78) / SNP Type:   INTRON /
Context              (SEQ ID NO: 1778): /
TGATCAATAATAATAGCAGGGTTGGGGGTAGCAATCTATCAGATACTGTTCAGGTAAAGGCTTTCCAAGAAGGTGACATTTCA
GCTCAGAATTGAAGGAT

R
GGAAGGGACCAGCCAAGGGTTCCTCCTCAGCCTGAGGCCAGGTGAGAGGAGTTCTGGGGTAGAGATGGGGGTAGGATGGCAGG
GTGGTTGGAGTACTCAG / Celera SNP ID: hCV3249290 / Public SNP ID: rs2076667 /
SNP Chromosome Position: 33506964 / SNP in Genomic Sequence: SEQ ID NO: 381 /
SNP Position Genomic: 54198 / Related Interrogated SNP: hCV1825046 / SNP
Source: dbSNP; Celera; HapMap; HGBASE / Population(Allele,Count): Caucasian
(G,85|A,141) / SNP Type: INTRON /
Context (SEQ ID NO: 1779): /
GCTAGTTGAAGGAACACTGGAGGACTGTCGGGGATTTGGGGGATTAAGTAAAAGGTTTATAAGGAGAGGGTGGAAAGAGTGGT
TGATGGCTGATATTCTG
R
TCATTTATACTAGCTACTCAGTCTCTACTTTTTCTAGAAGTCCCCAGTAACCAAAGCCCAACACATTTGGTATCGTTGATTCC
TTCCATTGTTTGCTTTG / Celera SNP ID: hCV7593320 / Public SNP ID: rs1060615 /
SNP Chromosome Position: 33478381 / SNP in Genomic Sequence: SEQ ID NO: 381 /
SNP Position Genomic: 25615 / Related Interrogated SNP: hCV1825046 / SNP
Source: dbSNP; HGBASE / Population(Allele,Count): Caucasian (A,86|G,140) /
SNP Type: INTRON;PSEUDOGENE /
Context (SEQ ID NO: 1780): /
AATTGTGTAACCTGAAACAAATGACTTAGCTTCTATGAATTTTATTTCTTATGAAATGAAATGAGGGGGTGATAATCTGTAAG
GTTACTTCCATTGTTAC
R
GTTCTAATTGCTTCTCTTTCCTCCAAATTTTTTTTTTTTTTTTTTTTTTTTTTTTTGAGATAGAGTCTTGTTCTGTTGCCTAGGC
TGGAGTACAGTGGTGCA / Celera SNP ID: hCV7593321 / Public SNP ID: rs1013677 /
SNP Chromosome Position: 33468793 / SNP in Genomic Sequence: SEQ ID NO: 381 /
SNP Position Genomic: 16027 / Related Interrogated SNP: hCV1825046 / SNP
Source: dbSNP; Celera; HapMap; HGBASE / Population(Allele,Count): Caucasian
(G,85|A,141) / SNP Type: TFBS SYNONYMOUS;INTRON /
Context (SEQ ID NO: 1781): /
CTTCAACCCCACCCCATTTCACTTTCCCAAGAGCAAGTTAGTAACAGGGACAGGGCTTACATCCAGGCCTTCTGACTCAGCCT
AGAATTCCATCCATTCC
R
TCGATCTGCTGCCAAAGAATGTTACTGATTGGGCAATAATTATTTACTTGGTGTGTGGGCACCACTGTTTTTTAAATGAGTGT
TTTCATTTGTATCAAAC / Celera SNP ID: hCV11189331 / Public SNP ID: rs6087649 /
SNP Chromosome Position: 33510662 / SNP in Genomic Sequence: SEQ ID NO: 381 /
SNP Position Genomic: 57896 / Related Interrogated SNP: hCV1825046 / SNP
Source: dbSNP; Celera / Population(Allele,Count): Caucasian (A,85|G,141) /
SNP Type: UTR5;INTRON /
Context (SEQ ID NO: 1782): /
CTCATGCTGTCTTCCCCCTGGTGTGGCAGATAAATATGTTAGTGGGGGTGCATGTGTATATGTATGGGAAAATGATCAGTGAC
AAGGTATTGGGATAGTC
M
TTCAGGGAGAGAGAGTCAGTGAGGGTGTTAGAGGTAGCTGAAGGAGTGGAAGGGAGTTAGCTAGTTGAAGGAACACTGGAGGA
CTGTCGGGGATTTGGGG / Celera SNP ID: hCV16189181 / Public SNP ID: rs2295097 /
SNP Chromosome Position: 33478221 / SNP in Genomic Sequence: SEQ ID NO: 381 /
SNP Position Genomic: 25455 / Related Interrogated SNP: hCV25620145 / SNP
Source: dbSNP; HGBASE / Population(Allele,Count): Caucasian (A,212|C,14) /
SNP Type: INTRON;PSEUDOGENE /
Context (SEQ ID NO: 1783): /
TAAAAAGATATCCTGTCTGCCATTAAGGAACTCATAATCCAGTGAGGGAAACAGATGTCTACATAGGACTCTAGTGCTAGAAA
AAAATAAGTGCCCAAGT
K
TTGTCTTTAGAAATAAACAGGCTGGGCGTGGTGGCTCACGCCTGCAATCCTAGCACTTTGGGAGGCCAAGGCGGGCGGATCAC
TTGAGGTCAAGAGCCTG / Celera SNP ID: hCV27166987 / Public SNP ID: rs6119542 /
SNP Chromosome Position: 33484545 / SNP in Genomic Sequence: SEQ ID NO: 381 /
SNP Position Genomic: 31779 / Related Interrogated SNP: hCV1825046 / SNP
Source: dbSNP; Celera; HapMap; ABI_Val / Population(Allele,Count): Caucasian
(G,42|T,78) / SNP Type: INTRON /
Context (SEQ ID NO: 1784): /
TTATACAAGTGAGCGCACATAGGTGAGTTGGTATGTGGTTTGCATATGGGGACACATGTGCTTGGGTATGTATATGTTGGCAT
TCAGCCATAACCGGAGA
Y
GTTCTGAAGTTCCTGTGGCACTTGTCAGTGAGCCAGTTAAGGAAATGGCTTTTCTGCCTCTGGCATTCAGGGCCGTTTGCCAG
CTTCCTCTCTCCCTGTT / Celera SNP ID: hCV29372811 / Public SNP ID: rs7266550 /

SNP Chromosome Position: 33495510 / SNP in Genomic Sequence: SEQ ID NO: 381 /
SNP Position Genomic: 42744 / Related Interrogated SNP: hCV1825046 / SNP
Source: dbSNP; HapMap / Population(Allele,Count): Caucasian (C,85|T,141) /
SNP Type: INTRON /
Context (SEQ ID NO: 1785): /
TACCATTGCTTAGTTGGGGGCCAGTTTGTCACTGAAATTCACTGTCCATTGTGTCTGTTGAGACTCTCTTTAGCTTCTGCTCT
AGAGCTTTATGCTTGAG
Y
TCCAGCCAACACTTGGGCTCATTTCCCAGGCTCTTTAAAGATCAGAAAACCTTTAAAGATCTTCAAAGGAGGCCGGGCATGGT
GGCTCATGCCTGTAATC / Celera SNP ID: hCV29747405 / Public SNP ID: rs6088640 /
SNP Chromosome Position: 33472509 / SNP in Genomic Sequence: SEQ ID NO: 381 /
SNP Position Genomic: 19743 / Related Interrogated SNP: hCV1825046 / SNP
Source: dbSNP; HapMap / Population(Allele,Count): Caucasian (C,85|T,141) /
SNP Type: INTRON /
Context (SEQ ID NO: 1786): /
TCAGATGAAACTCTGTTTTCTTGCCCCAGTCAGGCTCAAGCCCTGTGGTTGTAGGAATAAAGCCTGTGATCTCAAGAAGTGGT
GACTTGTTTTCTTAGGT
W
TCTGTGCCCTGAGGCTGTATATTCATTAGTGGCTCTGATTTGTGTGGCAGATGGGAAGGGAGGGGTATTTCCTAGGACTATCC
CCAATATCTTCCTTCTA / Celera SNP ID: hDV70936222 / Public SNP ID: rs17309872 /
SNP Chromosome Position: 33515788 / SNP in Genomic Sequence: SEQ ID NO: 381 /
SNP Position Genomic: 63022 / Related Interrogated SNP: hCV25620145 / SNP
Source: dbSNP; HapMap / Population(Allele,Count): Caucasian (A,114|T,6) / SNP
Type: INTERGENIC;UNKNOWN /
Context (SEQ ID NO: 1787): /
TGGTCAACAAGAGCAAAACTCTGTCTCAAAAAAAAAAAAAAAGTCTACCTCTTTAAACACTACTTTCCTTCCTTGTTATCTCAA
ACTCTTCTAAGTTTTCT
Y
TGTCGTGTTTTGAAATCTAGACCCATTTCTTTTTTTTTTTTTTTTTGAGACAGAGTCTCGCTCTGTCAGCCAGGCTGGAGTGCAGT
GGCATGATCTCAACTTA / Celera SNP ID: hCV30485907 / Public SNP ID: rs6087653 /
SNP Chromosome Position: 33522054 / SNP in Genomic Sequence: SEQ ID NO: 381 /
SNP Position Genomic: 69288 / Related Interrogated SNP: hCV1825046 / SNP
Source: dbSNP; HapMap; ABI_Val / Population(Allele,Count): Caucasian
(T,85|C,141) / SNP Type: INTRON /
Context (SEQ ID NO: 1788): /
GCCTCCATCTCCAAAAAAAAAAAAAGTGGTGGGTGCTAAGATTAGACAGGTAAAGAAGGGACCCAGGTGGTGGGTCCTAGGGCA
AGGAAGTTATAATTTGG
Y
CCTGTGTATAGACTGGTAAGCCTTCTGAGGCTAAATATGTCTCAGAAGATTAATGATATCTGGGCTTCCATTTCTGTTGCAGA
ATTCTGGGGAGACATTG / Celera SNP ID: hCV30558455 / Public SNP ID: rs6088638 /
SNP Chromosome Position: 33470514 / SNP in Genomic Sequence: SEQ ID NO: 381 /
SNP Position Genomic: 17748 / Related Interrogated SNP: hCV1825046 / SNP
Source: dbSNP; HapMap; ABI_Val / Population(Allele,Count): Caucasian
(T,185|C,39) / SNP Type: INTRON //

Gene Number: 45 / Gene Symbol: SCYL3 - 57147 / Gene Name: SCYL1-like 3 (S.
cerevisiae) / Chromosome: 1 / OMIM NUMBER: / OMIM Information: // Genomic
Sequence (SEQ ID NO: 382): // / SNP Information /
Context (SEQ ID NO: 1789): /
AGAGTAAGTTAGACTTCTATGTATTGGGAGACAGGTCCCTGATATGTTGTTACTTGAAAACAAATTATAGATTTGTGCATGGC
ATTTTCTTACCATTTAA
R
CAAAAATCCTACACAGTCTTGTGTCACTTAACAATGGGGACATGTTCTGAGAAATGCATCTTAGACGATTTTGTCCTTGTATA
AACATAGATGGTATAGC / Celera SNP ID: hCV29397289 / Public SNP ID: rs4656198 /
SNP Chromosome Position: 169826867 / SNP in Genomic Sequence: SEQ ID NO: 382 /
SNP Position Genomic: 15063 / Related Interrogated SNP: hCV11975250 / SNP
Source: dbSNP; HapMap; HGBASE / Population(Allele,Count): Caucasian
(G,181|A,45) / SNP Type: TRANSCRIPTION FACTOR BINDING SITE;INTRON /
Context (SEQ ID NO: 1790): /
CGCCTGTAGGGAAAATAATTATTCTCATAAAATACTGGTTAAAGGTAAGAACTACACTTATGGGAAGGATAGCATTAAGGAAG
TGTAGTAGTATAGGTCT
R
ATTACTTGGAAATATCCATGCATACTAAACAAAAGAGATCCCAGCAGAACATTACTCAAGATCAACGATGTCACATGTTATCC

334

AAATAATTACATAATAC / Celera SNP ID: hCV29585595 / Public SNP ID: rs10489173 / SNP Chromosome Position: 169824203 / SNP in Genomic Sequence: SEQ ID NO: 382 / SNP Position Genomic: 12399 / Related Interrogated SNP: hCV11975250 / SNP Source: dbSNP; HapMap / Population(Allele,Count): Caucasian (A,198|G,28) / SNP Type: ESE;SILENT MUTATION;INTRON / Context (SEQ ID NO: 1791): / TCTACCAAATATTTTCTTTTCCCAGCAATTACTCGAGACTACCTTGGTCCTAGCTCCCTTAGCTTGAAAGTTCCAAAGATAAT GGCTTGCACTCTGAGTA Y TAAGGATGCACTGTACTGGCTGATATTCTGGTATCCAGCGGTAACGTTACTCCGGCCCTTGCTTTCGTGTTTCAGGTATATAT TCCCTCTGCGGAACTCA / Celera SNP ID: hCV32141874 / Public SNP ID: rs12121045 / SNP Chromosome Position: 169862071 / SNP in Genomic Sequence: SEQ ID NO: 382 / SNP Position Genomic: 50267 / Related Interrogated SNP: hCV11975250 / SNP Source: dbSNP; HapMap / Population(Allele,Count): Caucasian (T,198|C,28) / SNP Type: INTRON / Context (SEQ ID NO: 1792): / AAATGTTTCTACCAAATATTTTCTTTTCCCAGCAATTACTCGAGACTACCTTGGTCCTAGCTCCCTTAGCTTGAAAGTTCCAA AGATAATGGCTTGCACT S TGAGTATTAAGGATGCACTGTACTGGCTGATATTCTGGTATCCAGCGGTAACGTTACTCCGGCCCTTGCTTTCGTGTTTCAGG TATATATTCCCTCTGCG / Celera SNP ID: hCV32141873 / Public SNP ID: rs12131357 / SNP Chromosome Position: 169862064 / SNP in Genomic Sequence: SEQ ID NO: 382 / SNP Position Genomic: 50260 / Related Interrogated SNP: hCV11975250 / SNP Source: dbSNP; HapMap / Population(Allele,Count): Caucasian (C,196|G,26) / SNP Type: INTRON / Context (SEQ ID NO: 1793): / GCTCCTCAGGTTCACTCCAGTCAGGCCACTCCTCAGACTTTTTAGAACTTGATGGGAAGTTTTCACTGTCCTCCGAAGTATTT TTTACATCTGAGAGTCC R TTTATGGGAAATTTAATAGGCTGAGAAAATGGATCGCCTGTAGGGAAAATAATTATTCTCATAAAATACTGGTTAAAGGTAAG AACTACACTTATGGGAA / Celera SNP ID: hCV32141820 / Public SNP ID: rs12132384 / SNP Chromosome Position: 169824068 / SNP in Genomic Sequence: SEQ ID NO: 382 / SNP Position Genomic: 12264 / Related Interrogated SNP: hCV11975250 / SNP Source: dbSNP; HapMap / Population(Allele,Count): Caucasian (A,198|G,28) / SNP Type: ESS;ESE;SILENT MUTATION / Context (SEQ ID NO: 1794): / AAGAATCTCCATAAACGTGCTAAGTGCCCTGGAAGCACTGGGGTGACTAATTTTGTTGGGCAGGGGAATGCCTAGGGGAAGGA GTCACAGAAAGGAGATT R AAGCCAGATCTTAGATGAGTTCACATTTTCCAGAAGCCAATGGGAAGAAATGCATTCCAGCAACAGAATGGCCTACAAAGAAG AAGAGTGTGAAGGAAGA / Celera SNP ID: hCV32141844 / Public SNP ID: rs12142093 / SNP Chromosome Position: 169843609 / SNP in Genomic Sequence: SEQ ID NO: 382 / SNP Position Genomic: 31805 / Related Interrogated SNP: hCV11975250 / SNP Source: dbSNP; HapMap / Population(Allele,Count): Caucasian (A,198|G,28) / SNP Type: INTRON / Context (SEQ ID NO: 1795): / ACTTTTTAGAACTTGATGGGAAGTTTTCACTGTCCTCCGAAGTATTTTTTTACATCTGAGAGTCCATTTATGGGAAATTTAATA GGCTGAGAAAATGGATC R CCTGTAGGGAAAATAATTATTCTCATAAAATACTGGTTAAAGGTAAGAACTACACTTATGGGAAGGATAGCATTAAGGAAGTG TAGTAGTATAGGTCTAA / Celera SNP ID: hCV32141821 / Public SNP ID: rs12135726 / SNP Chromosome Position: 169824104 / SNP in Genomic Sequence: SEQ ID NO: 382 / SNP Position Genomic: 12300 / Related Interrogated SNP: hCV11975250 / SNP Source: dbSNP; HapMap / Population(Allele,Count): Caucasian (G,198|A,28) / SNP Type: ESE;TRANSCRIPTION FACTOR BINDING SITE;SILENT RARE CODON;SILENT MUTATIO / N // Context (SEQ ID NO: 1796): / TTTCTGTGGTCTCTTCCACACTGCTACCAAAGTGAACTTTCTAGCACACACAAATTGGCCCACTCTTCTTTAGGGGAAGCAGT TTCCTAAGTAATGAGAG S TTTCATTGTCTTCCACATGCTCAACATTTACTCTTTGACCTCAGTCCAATTCTGAGACCTATGATGACAACTCCATTTCAGAG TTAAAGTGCTTCATTCC / Celera SNP ID: hCV32141828 / Public SNP ID: rs12136425 / SNP Chromosome Position: 169830104 / SNP in Genomic Sequence: SEQ ID NO: 382 /

SNP Position Genomic: 18300 / Related Interrogated SNP: hCV11975250 / SNP Source: dbSNP; HapMap / Population(Allele,Count): Caucasian (G,198|C,28) / SNP Type: INTRON /
Context (SEQ ID NO: 1797): /
GGGAGGCAGAGGTTGCAGTAAGCCGAGATCGCACCACTGCACTCCAGCCTGGGTGACAGAGAGAAATTCCGTTTCCCAGCACT
CCCCAGAAAAACCTAAA
Y
GGTAAATGAGGCTGAAGGTAGGCTGCAGAAAGATTGTGAAGGGCCTTATATCCCATGGCAAAGAATTTGGAATAAACAGCACA
GAAAGGATTACATGCTA / Celera SNP ID: hCV32141847 / Public SNP ID: rs12143057 /
SNP Chromosome Position: 169844093 / SNP in Genomic Sequence: SEQ ID NO: 382 /
SNP Position Genomic: 32289 / Related Interrogated SNP: hCV11975250 / SNP Source: dbSNP; HapMap / Population(Allele,Count): Caucasian (T,198|C,28) / SNP Type: INTRON /
Context (SEQ ID NO: 1798): /
TAAAAAAAAAGTATGAAAGGATTCCCACTGAAAATCCCCCCGAGAAGTTCAGAAAGAGAAATTAACTTTTTATTTATACTTCT
TTATATCATTTGACTGA
R
TATGACCCTCCTACATTACTTATATAATACTAAAAATCTAAAAAAATGACACACAAAAAGTTAGACCTTTTAATAATTCTTAC
CTTCTAGAGAAAGGTCA / Celera SNP ID: hDV70966798 / Public SNP ID: rs17543370 /
SNP Chromosome Position: 169828098 / SNP in Genomic Sequence: SEQ ID NO: 382 /
SNP Position Genomic: 16294 / Related Interrogated SNP: hCV11975250 / SNP Source: dbSNP / Population(Allele,Count): Caucasian (A,194|G,28) / SNP Type: INTRON /
Context (SEQ ID NO: 1799): /
TAAACTTTTCAAGAAATTCACAACTTCCAGAAAATCATTTCTAAATGCCAAAAAGAGAAGGAATTCAGCAGAACAGATTAGAG
AATGTTAAATATTGAGG
Y
CATAATCACAGACTACATCAATATAATTTTTCAACCAAGAAACATTCTCCTTTGCCATTTAAAGATTCTATTACAACTAAGTAG
CTTATGAGACAAATACT / Celera SNP ID: hDV70966830 / Public SNP ID: rs17543611 /
SNP Chromosome Position: 169836175 / SNP in Genomic Sequence: SEQ ID NO: 382 /
SNP Position Genomic: 24371 / Related Interrogated SNP: hCV11975250 / SNP Source: dbSNP; HapMap / Population(Allele,Count): Caucasian (T,198|C,28) / SNP Type: INTRON /
Context (SEQ ID NO: 1800): /
GTTTGAAGATCTTGGTTCGTTCTCCTCCCACAACCACCTCTGGTCCAAGCAGAGAGACCAGCACTGCTAGGCTATGCAGAGTA
ATTGCCACAATGGAATC
R
CTAGTATCACGCAGGCCCAGCAAAACCTAGGAGCAAATGAGGTAATAAGGGTTGACAACCACAATACCTATCTCTTTCTTCCC
CTGTAAATGAGCAATTG / Celera SNP ID: hDV70975002 / Public SNP ID: rs17602701 /
SNP Chromosome Position: 169828327 / SNP in Genomic Sequence: SEQ ID NO: 382 /
SNP Position Genomic: 16523 / Related Interrogated SNP: hCV11975250 / SNP Source: dbSNP; HapMap / Population(Allele,Count): Caucasian (G,194|A,28) / SNP Type: SILENT MUTATION;PSEUDOGENE /
Context (SEQ ID NO: 1801): /
TCTCACATTATTTTGTGGCCAGAGCTTATACCTGCATTTCATCTTGGTGTATACTAGGATTGTTGTTTTTTACTTGTAACAGT
GAAGAAAGTGTATATTG
Y
TATCCAGTAACAGAAAACACTATATATCTGCTTGAAATAATCGTTAAAACTACCAAAGGTGTGACCAACAACCCAAACTCTAG
AGAAAGTGAGGCAAGCT / Celera SNP ID: hDV70975134 / Public SNP ID: rs17603666 /
SNP Chromosome Position: 169856028 / SNP in Genomic Sequence: SEQ ID NO: 382 /
SNP Position Genomic: 44224 / Related Interrogated SNP: hCV11975250 / SNP Source: dbSNP; HapMap / Population(Allele,Count): Caucasian (T,198|C,28) / SNP Type: INTRON /
Context (SEQ ID NO: 1802): /
AACATCTCTGGAATAATACACGAGTTACCACCATTGCCTCTGAAGAGAGGAATTAGGGCACTGGATTCAAGGATGGGAGAGGG
ACACCTCACCCACATAC
Y
GTACAAACTTTTTAACCATGTGTATGCACTTTTGTCAACAAAGAAGATATTTCTCCCCACTGAACCACTAATAATTCTCGTTA
TCATGCGAACTTGTAAA / Celera SNP ID: hCV30126935 / Public SNP ID: rs6692451 /
SNP Chromosome Position: 169846883 / SNP in Genomic Sequence: SEQ ID NO: 382 /
SNP Position Genomic: 35079 / Related Interrogated SNP: hCV11975250 / SNP Source: dbSNP; HapMap / Population(Allele,Count): Caucasian (T,183|C,43) / SNP Type: INTRON //

EP 2 635 709 B1

--- --- --- ---

Gene Number: 46 / Gene Symbol: MYH7B - 57644 / Gene Name: myosin, heavy chain 7B, cardiac muscle, beta / Chromosome: 20 / OMIM NUMBER: / OMIM Information: // Genomic Sequence (SEQ ID NO: 383): // / SNP Information / Context (SEQ ID NO: 1803): /
GGAGGTACCTTTCCTCTATCCCACCAAGTCAGCTGACACAGTAACACCTCACGGCTCTGCAATCTTCCAGTTCCTTGGCTCTG
GCAGCTCCTGGCCCCCC
M
ATGCTTCACTGTCCCCTACCTGGAAAGAGTTTGCTCCAGGAAGGCAGCGTTCTGGCTGACAGCATCCACTAGCAGGTTGAAGT
CCATCTGCACAGCATAG / Celera SNP ID: hCV1064756 / Public SNP ID: rs734111 /
SNP Chromosome Position: 33533736 / SNP in Genomic Sequence: SEQ ID NO: 383 /
SNP Position Genomic: 32 / Related Interrogated SNP: hCV1825046 / SNP Source: dbSNP; Celera; HapMap; HGBASE / Population(Allele,Count): Caucasian (A,85|C,141) / SNP Type: INTRON /
Context (SEQ ID NO: 1804): /
TGCTGGGAAGGCAAAACAAGTGAACTGTGCTGAGTGTCACCATTTGTGACGGGCAGCACTGAGCCACCTTCACAGCTTGGCTG
CCCCTCCCTAGGGATGC
R
CTGTTCACCATCCAGTGGAACATCCGTGCCTTCAATGCCGTCAAGAACTGGTCATGGATGAAGCTCTTTTTCAAGATGAAGCC
GCTGCTGCGCTCGGCGC / Celera SNP ID: hCV16013546 / Public SNP ID: rs2425012 /
SNP Chromosome Position: 33581955 / SNP in Genomic Sequence: SEQ ID NO: 383 /
SNP Position Genomic: 48251 / Related Interrogated SNP: hCV1825046 / SNP Source: dbSNP; HapMap; ABI_Val; HGBASE / Population(Allele,Count): Caucasian (G,125|A,101) / SNP Type: ESE;SILENT RARE CODON;SILENT MUTATION /
Context (SEQ ID NO: 1805): /
GCTCTGATATGTGTGTCCTGCCCAGCCCCTCAGGCCTCCACCTGGAGCAGCAGGCAGGGGGAAGGGGTCACCCACCGCTGCCG
GAAGTCGGTGTAGAGCA
R
CCTGTTGGGGAACCCCTTGGCGGCAGATCCGGATCCCCTCCAGGACCCCATTGCAGCGCAGCTGGTGTAGCACCAAGAAGGCAT
CCATGACCCCTGGACGT / Celera SNP ID: hCV16013558 / Public SNP ID: rs2425009 /
SNP Chromosome Position: 33578899 / SNP in Genomic Sequence: SEQ ID NO: 383 /
SNP Position Genomic: 45195 / Related Interrogated SNP: hCV1825046 / SNP Source: dbSNP; HapMap; HGBASE / Population(Allele,Count): Caucasian (A,35|G,85) / SNP Type: SILENT RARE CODON;SILENT MUTATION /
Context (SEQ ID NO: 1806): /
AGTGGGTACAGAGATGCCAGCTTGGGTTTGCCATTGTTAGAAGTGAAGATGACACCTCAGTGCTGCAGATTTTGCAGGAGGGG
ACTGGAGCCAAGCCACA
R
ACTTTTCTCTGTTAGGAGATGGGTTCTTGGAGTGGGTGTGGTGTCTGACCATCCGGGGAGATGGCTGAAGCTGGGGATCTGGG
AAGGCTCCCAGAAGGGA / Celera SNP ID: hCV2142611 / Public SNP ID: rs1885114 /
SNP Chromosome Position: 33577361 / SNP in Genomic Sequence: SEQ ID NO: 383 /
SNP Position Genomic: 43657 / Related Interrogated SNP: hCV1825046 / SNP Source: Applera / Population(Allele,Count): Caucasian (A,10|G,8) African American (A,19|G,5) total (A,29|G,13) / SNP Type: UTR3;INTRON / SNP Source: dbSNP; Celera; HapMap; ABI_Val; HGBASE / Population(Allele,Count): Caucasian (A,126|G,100) / SNP Type: UTR3;INTRON /
Context (SEQ ID NO: 1807): /
CAGTGGCCCGGCTGACCAAGGAGAAGAAGGCGTTGCAGGAGGCCCACCAACAGGCCCTGGGTGACCTGCAGGCCGAGGAGGAC
CGTGTGAGCGCGCTGAC
R
AAGGCCAAGCTCCGGCTGGAGCAACAGGTGGAGGACGTGAGTCAGGGCCACCCCGAGACTGGGTGGCGGGGCCGGGGTGCCGG
CTGGCTTGGGCCATGGC / Celera SNP ID: hCV2142616 / Public SNP ID: rs3746438 /
SNP Chromosome Position: 33584289 / SNP in Genomic Sequence: SEQ ID NO: 383 /
SNP Position Genomic: 50585 / Related Interrogated SNP: hCV1825046 / SNP Source: dbSNP; Celera; HGBASE / Population(Allele,Count): Caucasian (G,124|A,102) / SNP Type: ESS;ESE;TRANSCRIPTION FACTOR BINDING SITE;SILENT MUTATION /
Context (SEQ ID NO: 1808): /
ACTCCCCCGTCGTCTCTAACGTAAAACCTGTGCCTAAAGCCTGGCAGACCAGGACTCCAGGTGAACTTCTGGACAACCCAGCA
CACTCCACAGAGCCCTG
K
GTTAACTTTACCTTTGCATCGGTGTTGATATATTTATTGAATCTCTTGAATGCATCAACGTTGAACTTCATCAGCTCCCCCAG
GAGGTCAAAGTAACTCT / Celera SNP ID: hCV25619953 / Public SNP ID: rs6060151 /

337

SNP Chromosome Position: 33594226 / SNP in Genomic Sequence: SEQ ID NO: 383 / SNP Position Genomic: 60522 / Related Interrogated SNP: hCV1825046 / SNP Source: Applera / Population(Allele,Count): Caucasian (G,6|T,18) African American (G,21|T,7) total (G,27|T,25) / SNP Type: UTR5;INTRON / SNP Source: dbSNP; HapMap / Population(Allele,Count): Caucasian (G,83|T,143) / SNP Type: UTR5;INTRON /
Context (SEQ ID NO: 1809): /
TGCCTCAGGCAGAGGCAGGGCAGGCACAGCTGCCCCGTGCCCCAAGGGATGGAGGGAAAGGCCCCTCACTTCTGGGAGAACCC CCTTGGATGAACACAGC
R
GCCAATGAGCAAACAGAACCAGAGGAGCTACAGGAAAATGAGGCAGAGGGGCAGGCTTGGGGGAAGGCAGCATTCTCTGGTAC CCACCACCCCTGCCCAG / Celera SNP ID: hCV1347919 / Public SNP ID: rs1058003 /
SNP Chromosome Position: 33590417 / SNP in Genomic Sequence: SEQ ID NO: 383 / SNP Position Genomic: 56713 / Related Interrogated SNP: hCV1825046 / SNP Source: dbSNP; Celera; HapMap; ABI_Val; HGBASE / Population(Allele,Count): Caucasian (G,83|A,143) / SNP Type: TRANSCRIPTION FACTOR BINDING SITE;MICRORNA;UTR3;INTRON;PSEUDOGENE /
Context (SEQ ID NO: 1810): /
GTGGAGGTTGCAGTGAGCCAAGATCATGCCACTGCACTCCAGCCTGGGCAATAGAGCAAGATTCCGTCTTGAAAAAAAAGAAAA AGAAAGAAAACACATTT
W
ATATATCTAACCCACTGAACCTCCTAGCTTAGCCTAGCCTACCTGGAATGTGCTCAGAACACTGACTTCAGCCTACAGTTGGC CATAATCACTGGCAACA / Celera SNP ID: hCV1347925 / Public SNP ID: rs6120790 /
SNP Chromosome Position: 33599090 / SNP in Genomic Sequence: SEQ ID NO: 383 / SNP Position Genomic: 65386 / Related Interrogated SNP: hCV1825046 / SNP Source: dbSNP; Celera; HapMap / Population(Allele,Count): Caucasian (A,91|T,19) / SNP Type: TRANSCRIPTION FACTOR BINDING SITE;INTRON /
Context (SEQ ID NO: 1811): /
CAGGGGCTGAGCCTGTATTAGAACCCAGGCTTGTTTCCCAGCCCAGCCAAGTCCCAGAAAAATCCCCCTGATTTTATCTATTT TTTTTTAACCTGGGATA
S
CAGCAGGGAAAGCATTAGAGGCAAAAGGAAAGGATTTTCATTTTTCCAGGAATGCTGAAGTCAGGACTGTTTGAGGGGAGCCC TGTGTTTCTCAGACTCT / Celera SNP ID: hCV2142584 / Public SNP ID: rs3761144 /
SNP Chromosome Position: 33544075 / SNP in Genomic Sequence: SEQ ID NO: 383 / SNP Position Genomic: 10371 / Related Interrogated SNP: hCV1825046 / SNP Source: dbSNP; Celera; HapMap; ABI_Val; HGBASE / Population(Allele,Count): Caucasian (C,45|G,71) / SNP Type: INTRON /
Context (SEQ ID NO: 1812): /
CCATGACTGACCCCCCAACCCAAGAAGTAAAGACTCTCCTGATTTGGTGTTTATTATTCCTATGCAATGTTTATTGAGATATA ATTCATGCACCATAAAA
Y
TCCCCCTTTAAAGTGTGCAATTCAGTGGTTCTTGGTATATTTACAAGATTGTACAACCATTACCACTGTCTCATTCCAGAATA TTTTCATCACCACAGAA / Celera SNP ID: hCV2142586 / Public SNP ID: rs6060130 /
SNP Chromosome Position: 33549319 / SNP in Genomic Sequence: SEQ ID NO: 383 / SNP Position Genomic: 15615 / Related Interrogated SNP: hCV1825046 / SNP Source: dbSNP; Celera; HapMap / Population(Allele,Count): Caucasian (C,46|T,74) / SNP Type: INTRON /
Context (SEQ ID NO: 1813): /
ATTGTTTTCAAATATTTTCAAGCTGCAGTTGGTTGAATACACAGATGTGGAACCCATGGATATGGAGGTCCCACTGTACTTGT CTGATTGTGGCCATCCT
R
GTTGGTCATTGTGGTTTTGATTTGCATTTTCCTAATGACAAATAATGCGCATATCTTTTCATGTGCTTATTGGTAGTTTGTAT ATCTTCTTTGGAGAAGG / Celera SNP ID: hCV2142587 / Public SNP ID: rs6088664 /
SNP Chromosome Position: 33551100 / SNP in Genomic Sequence: SEQ ID NO: 383 / SNP Position Genomic: 17396 / Related Interrogated SNP: hCV1825046 / SNP Source: dbSNP; Celera; HapMap / Population(Allele,Count): Caucasian (G,46|A,72) / SNP Type: INTRON /
Context (SEQ ID NO: 1814): /
CCAATCTCAGTAGGCATTCTGTGGTTTCCCCTCTGAGTGTCCTTGCTCTCTGTATCTGGAGGTGGGGTATTTTTCTGGGTGTG TCAGACTCTGCGTGACT
Y
GGTTTCTCCCCGTGAGCTCTTCAGTCTCCTGAACCTCTTACTGCCTTGTCCCTGTCCCCGTCAGTCTCTGCACCCAGCCTCCC TGTCTCTCAGTGGAGTG / Celera SNP ID: hCV2142597 / Public SNP ID: rs6120778 /

SNP Chromosome Position: 33565169 / SNP in Genomic Sequence: SEQ ID NO: 383 / SNP Position Genomic: 31465 / Related Interrogated SNP: hCV1825046 / SNP Source: dbSNP; Celera; HapMap / Population(Allele,Count): Caucasian (T,100|C,126) / SNP Type: INTRON / Context (SEQ ID NO: 1815): / ATGACTTTCCATGCATCATCTCAACAACCTGGGGAGGTGGGGCCAGCACCAGACCTAGCTTCACAGGGCGCCAGGCTAAGAGG GACCTTGTGTTTGATTC R ACACTCTGCTGTTGCCGTCTTGAAAGTTTTAATTGTTTTCAACAAGGGGACCCACATCGTCATTTTGTACTGGGGTCTGTAGG TTATGTAGATGGTCCTA / Celera SNP ID: hCV2142599 / Public SNP ID: rs6060140 / SNP Chromosome Position: 33566507 / SNP in Genomic Sequence: SEQ ID NO: 383 / SNP Position Genomic: 32803 / Related Interrogated SNP: hCV1825046 / SNP Source: dbSNP; Celera; HapMap / Population(Allele,Count): Caucasian (G,100|A,126) / SNP Type: TRANSCRIPTION FACTOR BINDING SITE;INTRON / Context (SEQ ID NO: 1816): / ACTTAATACCTGTCCTCTCTTCCTGTCCTGAACCCCAGCACCCTAGCCTTAACCCCTGACCTTTCTACCACATAATCCTGACA GCTGACCTCTGACCATT R TCCCCAGGTGACTCCGCAGCTACCATCCCAACTCTCACCCTGACCCACTGCCCTGGGACTGGCCCAGCCCTGTCAGTTCTGAC ACCTGTTAAGCCCAGCA / Celera SNP ID: hCV3249293 / Public SNP ID: rs6058154 / SNP Chromosome Position: 33585834 / SNP in Genomic Sequence: SEQ ID NO: 383 / SNP Position Genomic: 52130 / Related Interrogated SNP: hCV1825046 / SNP Source: dbSNP; Celera; HapMap / Population(Allele,Count): Caucasian (G,126|A,100) / SNP Type: INTRON / Context (SEQ ID NO: 1817): / CATTTTGGAATGACAGTTTTAGAAACAGAGGACAGACTGGTGGTTGCCAGGGGTTAAAGATGGGAGGGAAGTTAGTGTGGTTC TATAAGGGCAACACCTT Y TTATTGTTAGAACTGTTCAGTGTCTTGGTGGCAGTGGAAGATCTGTGAATTGACACGGGTGATGAAGTCGTATAGAACTTAAT ACATGCAGATGTGCGTG / Celera SNP ID: hCV11189318 / Public SNP ID: rs7263251 / SNP Chromosome Position: 33554320 / SNP in Genomic Sequence: SEQ ID NO: 383 / SNP Position Genomic: 20616 / Related Interrogated SNP: hCV1825046 / SNP Source: dbSNP; Celera; HapMap / Population(Allele,Count): Caucasian (T,158|C,68) / SNP Type: INTRON / Context (SEQ ID NO: 1818): / AGGACCGCTGTGGCGTCCAGGCGGCAGGATGCTGGGCACCTGCCCTGCCGCTCCTGACTTGCTGTGTGGCCTTGAGCAGATCC ACTCCCTGCCCAGAGCT W AGTCTCCTCATTTGCAAATTAGGGCTAATAATGGGCACCTGGCAGGGCCTTTGTGCGCACTGGTAGGTATACAGGCAGACAAA CAATGAAGACACACCCA / Celera SNP ID: hCV16013570 / Public SNP ID: rs2077574 / SNP Chromosome Position: 33571919 / SNP in Genomic Sequence: SEQ ID NO: 383 / SNP Position Genomic: 38215 / Related Interrogated SNP: hCV1825046 / SNP Source: dbSNP; HapMap; ABI_Val; HGBASE / Population(Allele,Count): Caucasian (A,35|T,85) / SNP Type: INTRON / Context (SEQ ID NO: 1819): / ACTTCAAGACTCATCTTAGATGTATCTAAGGACCTCAGAGAAGGTCTCCCTGTATTCACCCAGCCCCCAGCCCTAGCAGCGGA AGAGGCTTTCCTTTGCT S CTATAGCTCCTGGGCCAACCCTGGCTCCATTCCTTCCCACACTGCATTGAAACAGCACAGCTCTAGGGAATTCATGGCAGGGC CCGTATCCTAGTTCTCT / Celera SNP ID: hCV16190708 / Public SNP ID: rs2295701 / SNP Chromosome Position: 33596117 / SNP in Genomic Sequence: SEQ ID NO: 383 / SNP Position Genomic: 62413 / Related Interrogated SNP: hCV1825046 / SNP Source: dbSNP; HapMap; ABI_Val; HGBASE / Population(Allele,Count): Caucasian (G,61|C,165) / SNP Type: UTR5;INTRON / Context (SEQ ID NO: 1820): / GGAGCTGTTGATCTGCTTCAGGAATACCTGGAACTATACAGAAACCAAGCTCACAGCAGGCAGTGGGCTGCTGGCCAGGTATG GCCATGGCCCGGGGGGA Y AGTCACTACAAGGGGCATCAGCGACCTCTACCAGCCCCACTGCTTCAGATAGGAAGACAGAGGCTCAGATAAGCTGAGGGACC TCCCCTCACCACCCAGG / Celera SNP ID: hCV27472681 / Public SNP ID: rs3746430 / SNP Chromosome Position: 33593674 / SNP in Genomic Sequence: SEQ ID NO: 383 / SNP Position Genomic: 59970 / Related Interrogated SNP: hCV1825046 / SNP Source: dbSNP; HapMap / Population(Allele,Count): Caucasian (T,35|C,85) / SNP

Type: UTR5;INTRON /
Context (SEQ ID NO: 1821): /
CTGACCCCCTGGGCTCTAAAGAGGAATGTCTGCTGTTGCACATCTGGCTGAGGCCACCTGCCCCGATCCTGCCATCTCTGCAT
CGCCCCCTGCTGCCTTC
R
GCCTTCCCTGGGCCCTGAATAAACACCACAGCCAGTTTCCTTCTCATTCTTTTCTTTGGGGTTCAGGAGGAAAAACACAGTCC
TAGGGACAAAAGCCAGG / Celera SNP ID: hCV27893015 / Public SNP ID: rs4911167 /
SNP Chromosome Position: 33590096 / SNP in Genomic Sequence: SEQ ID NO: 383 /
SNP Position Genomic: 56392 / Related Interrogated SNP: hCV1825046 / SNP
Source: dbSNP; HGBASE / Population(Allele,Count): Caucasian (A,83|G,143) /
SNP Type: UTR3 /
Context (SEQ ID NO: 1822): /
TGGGATTACAGGCATGAGCCACCGCACCTGGCTCATTCCTGTTTTATAGATGAGGAATCAGAAGATCAAAGAGACAGAGCAAC
TCTGAGGGTTTGCACAT
M
GGCAGTTGGACTCCAGAACTCTTAGGCATCATGCTGTGTGGTTAACCTGGCTCAGCCGAATCCACTAGAGCAAAGAAAAGAGA
GGCTTGCCCAGGCTTGG / Celera SNP ID: hCV28004288 / Public SNP ID: rs4911455 /
SNP Chromosome Position: 33553062 / SNP in Genomic Sequence: SEQ ID NO: 383 /
SNP Position Genomic: 19358 / Related Interrogated SNP: hCV1825046 / SNP
Source: dbSNP; HGBASE / Population(Allele,Count): Caucasian (A,158|C,68) /
SNP Type: INTRON /
Context (SEQ ID NO: 1823): /
GTGTGTGGTGAGCAAGCAGGGATGGATGACCATGGCTTGCATGGAAGCCTGCAAGTGTGCATGGAAGCGTGTGACTGTGCGTT
GCAGTAAGCATGCATGT
R
AGTGTAGTTGTGACAGCGTTTATGTGCTTGTGTGTGAAAGGGCACGTGTGTGAGTGGGTGTGTTGGAGTTGACCATGAGTGAG
CCTTGGTGAGTGTGTGA / Celera SNP ID: hCV29372796 / Public SNP ID: rs7261167 /
SNP Chromosome Position: 33576205 / SNP in Genomic Sequence: SEQ ID NO: 383 /
SNP Position Genomic: 42501 / Related Interrogated SNP: hCV25620145 / SNP
Source: dbSNP; HapMap / Population(Allele,Count): Caucasian (G,203|A,21) /
SNP Type: INTRON /
Context (SEQ ID NO: 1824): /
TCGTATAGAACTTAATACATGCAGATGTGCGTGCACACACACGCTCACAACTAGTACATGTAAAACTGGGAAAATCTGAATAA
GATCGGTACATTATATC
R
ATGTCAATAGTACAGAGTTACTGTACTATTGCTTACAATTGCATGCGAATATACAATTATCTCAATAGAAATTTTAATTAAGA
AATGAAATTTGGCTAGG / Celera SNP ID: hCV29372800 / Public SNP ID: rs6058149 /
SNP Chromosome Position: 33554488 / SNP in Genomic Sequence: SEQ ID NO: 383 /
SNP Position Genomic: 20784 / Related Interrogated SNP: hCV1825046 / SNP
Type: INTRON /
Context (SEQ ID NO: 1825): /
TTTGGGGAAAGAGTAAATGGCAGATGCAGAGTCCCTGAGACAGAAAGGAGCTGGTTTAGTTGTAAACTCTCAGAAGGCCACTG
TATCTGGAGTGATATGA
R
TGAGAGGGTGGGGCTACTGTAGATGGGCTGGTCAGTTGGCAGGGCCAGGTCAGGCTGGGTCTCCAGGCCAGGGTCAACATATA
GGTCTATAGCCAACATG / Celera SNP ID: hCV29372802 / Public SNP ID: rs6579204 /
SNP Chromosome Position: 33553677 / SNP in Genomic Sequence: SEQ ID NO: 383 /
SNP Position Genomic: 19973 / Related Interrogated SNP: hCV1825046 / SNP
Type: INTRON /
Context (SEQ ID NO: 1826): /
CAATACTTTCCTACCTTCAAACTAGAATATCCAATTTTTTCCTGCGAGTCTCCATACAACCAGAATAGAATTTGTTGTTGTCT
CCCACAAATCTGCTTCT
Y
CTCTGATGAACCCCACCCAGGTACCCAAGCCTTGAGTACTTGTTTCCTACATCTCACTCTCCCCAAATCCTATGGATTTTGCT
GCCTAAGTATATAGTAC / Celera SNP ID: hCV29372803 / Public SNP ID: rs6088659 /
SNP Chromosome Position: 33542605 / SNP in Genomic Sequence: SEQ ID NO: 383 /
SNP Position Genomic: 8901 / Related Interrogated SNP: hCV1825046 / SNP
SNP Type: INTRON /
Context (SEQ ID NO: 1827): /
CAGAGGGAGTCGCCTAGTATCTGGTCTTGTGGGTTGGGGGATCGTGCCCTAGTTCAGCTCCCTTGTCACTGCCATCTTCCAGT

GCCTGCTGCCTTGGGCC
R
CCTTGAACCTCCAGGGTTTCCAGCTCCTCCTCCTTCACCCCAGTGCCACTGCCATGATGGATGTGAGTGAACTTGGGGAGTCT
GCCCGCTACCTCCGCCA / Celera SNP ID: hCV32066133 / Public SNP ID: rs11906160 /
SNP Chromosome Position: 33565755 / SNP in Genomic Sequence: SEQ ID NO: 383 /
SNP Position Genomic: 32051 / Related Interrogated SNP: hCV25620145 / SNP
Source: dbSNP; HapMap / Population(Allele,Count): Caucasian (G,200|A,26) /
SNP Type: MISSENSE MUTATION;ESE;UTR5 /
Context (SEQ ID NO: 1828): /
TAGACCCCAGGCGCCTAGGCCACACTCAAGGGGGCCAGGGACACAGAGGGGCCCCCACGTTGGCCACTCCCCACATTGGGCTG
CATGGCTAGGTCTTGAC
R
GATGAGCGCAGGCAGGCAGTGACGGGGACAGGGATAGGCTGTAATTCACACCCTGACCCCTACACAGACACACACAGACACAC
ACGGAGGCTCGGCCACA / Celera SNP ID: hCV30630345 / Public SNP ID: rs4911456 /
SNP Chromosome Position: 33573500 / SNP in Genomic Sequence: SEQ ID NO: 383 /
SNP Position Genomic: 39796 / Related Interrogated SNP: hCV1825046 / SNP
Source: dbSNP; HGBASE / Population(Allele,Count): Caucasian (G,61|A,165) /
SNP Type: TRANSCRIPTION FACTOR BINDING SITE;INTRON /
Context (SEQ ID NO: 1829): /
ATTTTTCAAGACAAGTTGGGTTGCTAGATTTTTTTGTTTTAATTTCATGATTTTTTTTTTTTAAATGTTGGCTCTGACTTTTTAA
AAATACTCTGGTGAAAA
M
AACTGTTAACAATGCTTGCCCCTGGTCAGGGAAACAGTGGCTGGAGGAAGGGGACTTACTCTGTACTGAATACCCTTTTTGAAT
TTTGCGCCCTGTTCAAA / Celera SNP ID: hCV30323918 / Public SNP ID: rs6060124 /
SNP Chromosome Position: 33536897 / SNP in Genomic Sequence: SEQ ID NO: 383 /
SNP Position Genomic: 3193 / SNP Source: dbSNP; HapMap /
Population(Allele,Count): Caucasian (C,165|A,59) / SNP Type: INTRON /
Context (SEQ ID NO: 1830): /
TCCCATCAGCTGGCCACAATGGTCTTCCCTCGTTCAGGGGCTTCTGGCTAGAACACACACCCTCTCACATGCCTGTTCACTCA
ATTCACTACAAGCTGAC
M
CTTCAGGTCTTGGCTGGCAAGCCACTTCTGCAGGGGAGAGTTCCTGAGTGTCCTCTGGCCAGTCCTGGACCCTGTCAAATGTC
TCTCACAGACCCCGTTC / Celera SNP ID: hCV29638959 / Public SNP ID: rs6060154 /
SNP Chromosome Position: 33599601 / SNP in Genomic Sequence: SEQ ID NO: 383 /
SNP Position Genomic: 65897 / Related Interrogated SNP: hCV1825046 / SNP
Source: dbSNP; HapMap / Population(Allele,Count): Caucasian (A,61|C,165) /
SNP Type: INTRON /
Context (SEQ ID NO: 1831): /
TCCCTCAGCCCTCCCCAGACTGGCTCCAGACCCGTGCCCTCCATATCACACACAGAAGAGTTCCCTCCACAGGAAGATGGATC
CATGACGCTGCATTAAG
R
AAAACAACGCAAAAGTTCCACTCCTTCTGCAGAGGTCCTAGCGCAACTGGTTTTCCACACCAGCTCTCAGGTGAGTCCGGCCC
AGTGCTGAGACCACAAG / Celera SNP ID: hDV70862585 / Public SNP ID: rs17092209 /
SNP Chromosome Position: 33592721 / SNP in Genomic Sequence: SEQ ID NO: 383 /
SNP Position Genomic: 59017 / Related Interrogated SNP: hCV1825046 / SNP
Source: dbSNP; HapMap / Population(Allele,Count): Caucasian (A,193|G,33) /
SNP Type: UTR5;INTRON /
Context (SEQ ID NO: 1832): /
ACACATGCAGACAGGCCACTGGGCTCACAGACGATGGGCTGGCCTAGGGAAGTAGTGGTTGATGCTGACCGAGGGACCCAGAG
GGCACAGCGGGTATCCA
Y
GACAGGGAGTTTGGACAAGGCACTGAAGTGACAAAGGCCCATGTGGGCATGAAGCCTCCTGACAGCTCACGCCCGGCCCTGAT
GGAACTCTACCTGTCCA / Celera SNP ID: hDV70862590 / Public SNP ID: rs17092215 /
SNP Chromosome Position: 33595913 / SNP in Genomic Sequence: SEQ ID NO: 383 /
SNP Position Genomic: 62209 / Related Interrogated SNP: hCV25620145 / SNP
Source: dbSNP / Population(Allele,Count): Caucasian (C,204|T,22) / SNP Type:
MISSENSE MUTATION;INTRON /
Context (SEQ ID NO: 1833): /
CTCTTGAGCTCAGGTGATCCACCCGCCCCAGCCTCCCAGAGTGCTAGGATTACAGGCGTTAGCCACTGCGCCCGGCCCCTTTT
GACTCTTTAAATAAACC
R
TGTACATTATCTGACTACAGGACAGGCTGAGTCCTCCAGTCTCCCGTGTGTGGTCACGGGATGGGGGGAGATGGTGGCAGATA
GGTGTCTGGTTATTCCC / Celera SNP ID: hDV70936327 / Public SNP ID: rs17310467 /

SNP Chromosome Position: 33545616 / SNP in Genomic Sequence: SEQ ID NO: 383 / SNP Position Genomic: 11912 / Related Interrogated SNP: hCV25620145 / SNP Source: dbSNP; HapMap / Population(Allele,Count): Caucasian (A,203|G,21) / SNP Type: INTRON /
Context (SEQ ID NO: 1834): /
GAGTCTTCTGAATCTGGAGTGGAGGAAGCCCTGGTGTTTGAGGGTGTGCATGTATGCTGGGCACTCACCCAAGGAGGCTGCAT
TGGGAGCCAGAGGGAGT
Y
GCCTAGTATCTGGTCTTGTGGGTTGGGGGATCGTGCCCTAGTTCAGCTCCCTTGTCACTGCCATCTTCCAGTGCCTGCTGCCT
TGGGCCGCCTTGAACCT / Celera SNP ID: hDV70936356 / Public SNP ID: rs17310782 /
SNP Chromosome Position: 33565665 / SNP in Genomic Sequence: SEQ ID NO: 383 /
SNP Position Genomic: 31961 / Related Interrogated SNP: hCV1825046 / SNP
Source: dbSNP / Population(Allele,Count): Caucasian (C,165|T,61) / SNP Type:
INTRON /
Context (SEQ ID NO: 1835): /
TGAGATCCCACCTCTACAAAAAATTTTTGTAAAATTAAAAAAACAAGGGAATTGCCTAAGGATGAGAGGTCACAGTGGCCTAAG
TGACCCTTAGGAATGAC
M
CTTTTTCTGGGACTTTTTTCATGATCTTTATATTGGTGTGACATGAACTTTCCTTGTTTAATCTACTGAGTTTGGAGATGGTCT
TAATGATAATAAGAAAA / Celera SNP ID: hDV70948869 / Public SNP ID: rs17401737 /
SNP Chromosome Position: 33552642 / SNP in Genomic Sequence: SEQ ID NO: 383 /
SNP Position Genomic: 18938 / Related Interrogated SNP: hCV25620145 / SNP
Type: INTRON /
Context (SEQ ID NO: 1836): /
ATCACCTAAATAGACATGAGTAGAGGGAAGCTAAACTGTGATACACCCACATCTGGAATACTATGCCTCAGAATGAAGATGAC
CCTAATAGAAGGCATTA
S
AAAGTTCTAAGACAGACAAATAAAAAACCAAAAACATATCATAGAATAATATATATGATACAATTTGTTTTTTTAAAAAAAGACG
TATTTCTATATATACCT / Celera SNP ID: hCV30162176 / Public SNP ID: rs6060127 /
SNP Chromosome Position: 33538999 / SNP in Genomic Sequence: SEQ ID NO: 383 /
SNP Position Genomic: 5295 / Related Interrogated SNP: hCV1825046 / SNP
Source: dbSNP; HapMap; ABI_Val / Population(Allele,Count): Caucasian
(G,83|C,37) / SNP Type: TFBS SYNONYMOUS;INTRON /
Context (SEQ ID NO: 1837): /
GGGGCTGTGACACGTGGAGATTGCTGACATAGCTCTCCTCCTCTGACCTTGACAGTGGCAATAAAAGGGGTAGCAGAGCTTCC
TGCCCTCACCGTGGTGC
Y
GAGGTAGGTGATCAGGGCTGGGGTTGGAGCCGAAGGGAGGGCAGGAGGGTGGGCGGGTGGGTGAGGGTGCCCATGGGTGGCCG
CGAGTCTTCTGAATCTG / Celera SNP ID: hCV30323913 / Public SNP ID: rs6060137 /
SNP Chromosome Position: 33565480 / SNP in Genomic Sequence: SEQ ID NO: 383 /
SNP Position Genomic: 31776 / Related Interrogated SNP: hCV1825046 / SNP
Source: dbSNP; HapMap / Population(Allele,Count): Caucasian (C,165|T,61) /
SNP Type: ESE;UTR5;SILENT MUTATION /
Context (SEQ ID NO: 1838): /
GTCAGGTGTCTTCAGTGACCAGGATATGTTGGCCTCTGTCCCCCTTCCAGGACTTGGCACGTGCTGTCTTCTCTTTGGAACTC
TCGCCACCACTCCTGAT
Y
TGGGCTCGCGATTTCGTTCCGGAAGCTGTCCCTGTTCTCCGCCCCGGGCTTCTACATTCTACAGCCCATCACTTTTCCCTGTA
CCGTAGGAACAGTGTTT / Celera SNP ID: hCV30053852 / Public SNP ID: rs6088660 /
SNP Chromosome Position: 33542896 / SNP in Genomic Sequence: SEQ ID NO: 383 /
SNP Position Genomic: 9192 / Related Interrogated SNP: hCV1825046 / SNP
Source: dbSNP / Population(Allele,Count): Caucasian (C,159|T,67) / SNP Type:
INTRON;PSEUDOGENE /
Context (SEQ ID NO: 1839): /
AGTGGTTCTGGAAGCCGATATGAGCAGCAGCAACTGCTGGCATTATCTAGGCTCTAAGAATAGCTTGGGATGAAGAGTCAGAG
GTTGAGGCCAGGGAAGA
K
GACAGGAGCCTGTTCCTACTGCCCCCTCCTGCTGCTTGGCCTGGAAGCTGGGAAGCCTGCCTCCTGGCTCACCATTTGCTCTG
GGATGGTTTTGCATAGA / Celera SNP ID: hCV29855628 / Public SNP ID: rs6058150 /
SNP Chromosome Position: 33556817 / SNP in Genomic Sequence: SEQ ID NO: 383 /
SNP Position Genomic: 23113 / Related Interrogated SNP: hCV1825046 / SNP
Source: dbSNP; HapMap; ABI_Val / Population(Allele,Count): Caucasian

(G,158|T,68) / SNP Type: INTRON /
Context (SEQ ID NO: 1840): /
GTGCTCCCACTTCCAAATGCACAAGTGTGCCAGATAAAATATAGAGAAATAAGTGCAAAGCTACATTTTCAGCCATATACATT
CAAAACAGGCAAATCTC
R
CCTATGGTGATAGAAGTGGCAGAGTGGACCTCTGAGGTAGGGTACAGAGTGGAGGCACCAGAGAGCCTCTGGGAGGCAGGCGA
TGCCCTATACCCTGAGT / Celera SNP ID: hCV30323914 / Public SNP ID: rs6087657 /
SNP Chromosome Position: 33564689 / SNP in Genomic Sequence: SEQ ID NO: 383 /
SNP Position Genomic: 30985 / Related Interrogated SNP: hCV1825046 / SNP
Source: dbSNP; HapMap; ABI_val / Population(Allele,Count): Caucasian
(G,85|A,35) / SNP Type: INTRON /
Context (SEQ ID NO: 1841): /
CTCACTGCTCTGTGCTGCCTCCCAAGCTTGAGACATGTTGAGACTCACATCATGCCCTCCCTCTTCCTAAGGGCCAAGGTAAG
CCATGTACGGTGAACAC
Y
TACCACGTACCAGGCTGAGGCTGGGCTACTGCAGTGTAAGGGAGACCAGGCCTCTGTCCTCATGGAACTGTCATCCCAGTGCC
CATGAGTGTCTGCCAGC / Celera SNP ID: hCV30342005 / Public SNP ID: rs6060133 /
SNP Chromosome Position: 33558011 / SNP in Genomic Sequence: SEQ ID NO: 383 /
SNP Position Genomic: 24307 / Related Interrogated SNP: hCV1825046 / SNP
Source: dbSNP; HapMap / Population(Allele,Count): Caucasian (T,79|C,35) / SNP
Type: INTRON /
Context (SEQ ID NO: 1842): /
AACATCCAAGGATACTTAAGTCCCTTATATAAAATGATGCAGTGTTTCATATATGCTTAAGTCCCTTATATAAAATGGTGTAG
TGTTTCATATAAACTAT
M
TGCATCCTCTCATATGCTCTTTAATTTTGTTTTATTTTATTTTAAATTTAAATTTATTTTTATTTATTTTGAGACAAGGTCTC
ACTCTGTCAGGCTGGAG / Celera SNP ID: hCV30503905 / Public SNP ID: rs4911165 /
SNP Chromosome Position: 33550382 / SNP in Genomic Sequence: SEQ ID NO: 383 /
SNP Position Genomic: 16678 / Related Interrogated SNP: hCV1825046 / SNP
Source: dbSNP; HapMap; ABI_val; HGBASE / Population(Allele,Count): Caucasian
(A,82|C,38) / SNP Type: INTRON /
Context (SEQ ID NO: 1843): /
AAGAGTATTGAACTGTTTATCATGAGCCCAGGAAAAGATCAAAATTCAAAATTCTACTTCTGTACCATTGTGAAGTTGAAAAA
TTCTAAGTGGACCATCT
S
TATATAAAATCCAGGCCTGTGACTGTGACCCACAACACCCTGTGACTGTGACCCACAACGTCCTGTGCAGCCCAGCCCAGAGC
CAAGCCCACATCACTCC / Celera SNP ID: hCV32066123 / Public SNP ID: rs7273734 /
SNP Chromosome Position: 33599403 / SNP in Genomic Sequence: SEQ ID NO: 383 /
SNP Position Genomic: 65699 / Related Interrogated SNP: hCV25620145 / SNP
Source: dbSNP / Population(Allele,Count): Caucasian (G,204|C,22) / SNP Type:
INTRON //

Gene Number: 47 / Gene Symbol: ZNF317 - 57693 / Gene Name: zinc finger
protein 317 / Chromosome: 19 / OMIM NUMBER: / OMIM Information: // Genomic
Sequence (SEQ ID NO: 384): // / SNP Information /
Context (SEQ ID NO: 1844): /
ATGGGCCAAGGAGGGGTCAGCGGCGAATTCTTTCGGCCTGTTGGGGACCCCTCGTGTCCCCACGCCAGACTGGACCTCCCGTA
TGAACTTCTCTTCGCAT
Y
GGCGGCGGCTTCCGTCACCTCCGCTCCCCCGATCCTATTCCCAGTCGTGTAAGCCCTGCTTTCCTTAACCCTTCTCCCCCTCA
GTTCCAGGGGTCACGGG / Celera SNP ID: hDV70794769 / Public SNP ID: rs17002013 /
SNP Chromosome Position: 9251220 / SNP in Genomic Sequence: SEQ ID NO: 384 /
SNP Position Genomic: 10164 / SNP Source: dbSNP; HapMap /
Population(Allele,Count): Caucasian (C,177|T,49) / SNP Type: UTR5;PSEUDOGENE
/
Context (SEQ ID NO: 1845): /
ATAATACATAATTATAATAAATATGATATAATATATATTCCATTGTATAAAACATATAATATATATAACGGAATATTATTCAA
CCTTGAAATAGAAGGAG
R
TCCTGACATTTATCACAAGATGGGTGGACCTGGAGGACATTACGCTAAGTGAAATAAGCCAGACACAAAAAGAAAAATATTGC
ATGACGTCACTTACATG / Celera SNP ID: hDV72050312 / Public SNP ID: rs8103016 /
SNP Chromosome Position: 9250370 / SNP in Genomic Sequence: SEQ ID NO: 384 /
SNP Position Genomic: 9314 / SNP Source: dbSNP; HapMap /

Population(Allele,Count): Caucasian (G,170|A,50) / SNP Type: INTERGENIC;UNKNOWN //

Gene Number: 48 / Gene Symbol: C1orf114 - 57821 / Gene Name: chromosome 1 open reading frame 114 / Chromosome: 1 / OMIM NUMBER: / OMIM Information: // Genomic Sequence (SEQ ID NO: 385): // / SNP Information /
Context (SEQ ID NO: 1846): /
AGTAAGTGACAGGAGCACATCTTCTTGTCCAAAATCATTGGAAATACATAATTGTGACAGTTTCCCAAACATATTCCTTTCGT
TTTCAAAATAATTTTTA
R
AAGTAGTAGTGTCTTCTAGTGGAGGAACTTCCAAATCAACTAACTGGTCCTTGAACTTAAGTTTTCGCTCCCTTTTATCATTC
ACCGGTTCTTGATTCTG / Celera SNP ID: hCV2456747 / Public SNP ID: rs3820059 /
SNP Chromosome Position: 169391154 / SNP in Genomic Sequence: SEQ ID NO: 385 /
SNP Position Genomic: 37040 / SNP Source: Applera /
Population(Allele,Count): Caucasian (A,12|G,22) African American (A,18|G,4)
total (A,30|G,26) / SNP Type: MISSENSE MUTATION;ESS;TFBS SYNONYMOUS / SNP
Source: Applera / Population(Allele,Count): Caucasian (A,6|G,30) African
American (A,6|G,12) total (A,12|G,42) / SNP Type: MISSENSE MUTATION;ESS;TFBS
SYNONYMOUS / SNP Source: Applera / Population(Allele,Count): Caucasian
(A,8|G,30) African American (A,9|G,11) total (A,17|G,41) / SNP Type: MISSENSE
MUTATION;ESS;TFBS SYNONYMOUS / SNP Source: dbSNP; Celera; HapMap; ABI_Val;
HGBASE / Population(Allele,Count): Caucasian (A,81|G,145) / SNP Type:
MISSENSE MUTATION;ESS;TFBS SYNONYMOUS /
Context (SEQ ID NO: 1847): /
CTTCACTTTTCTTTGAATCAGTATCTTTATTTTCATTCATTTTCTGTTTGTGAAGGAAATATGCTGTAAGATTTTAAAAGATA
AGGTATATGTTATGCCC
R
CTCTAGAGGAGGAATGGAGTATGGAGATTAATGCTGTAGGTAAGCTAATTAACACAGAGAAACACAAAATAGAATGTTCAAAA
TGAACATGCACTTGAAT / Celera SNP ID: hCV25616192 / Public SNP ID: rs10919168 /
SNP Chromosome Position: 169394226 / SNP in Genomic Sequence: SEQ ID NO: 385 /
SNP Position Genomic: 40112 / Related Interrogated SNP: hCV11975250 / SNP
Source: Applera / Population(Allele,Count): Caucasian (A,1|G,7) African
American (A,6|G,22) total (A,7|G,29) / SNP Type: INTRON / SNP Source:
dbSNP; HapMap / Population(Allele,Count): Caucasian (A,70|G,156) / SNP Type:
INTRON /
Context (SEQ ID NO: 1848): /
AAAAGCACAGTTTCCCCTTCTGGGTAGCGTGCTCATTCACTGCCTCCCTTTGCTGGGGGTAGGGGGTTCTCCTTTCCCATGTA
GCTCTCAGGTGGGCCAC
Y
CACTACACTGCTCTTCCTTCTGTCCATGGGTCACGCCAGCCTTCTAGTCAAGTTTGATGAGAGAACCTGGATACCTTGGTTGC
CAGTGAAGGATTCACAT / Celera SNP ID: hCV2217923 / Public SNP ID: rs2014878 /
SNP Chromosome Position: 169385506 / SNP in Genomic Sequence: SEQ ID NO: 385 /
SNP Position Genomic: 31392 / Related Interrogated SNP: hCV11975250 / SNP
Source: dbSNP; Celera; HapMap; HGBASE / Population(Allele,Count): Caucasian
(C,112|T,6) / SNP Type: INTRON /
Context (SEQ ID NO: 1849): /
TACATGAAAGAGCTCACAGACTGAAAAAGAAAATAGGCTTAAACAAAGGGAGCACAGTGTGACACACAAGGTACAGATTTAGC
TTGAAAAATGGAGGAAT
K
AATTAGGAGGAGAGTGACAATAAAATGATTTCTACAAAGTGTTGACATCAGAGCTGACATTTTTTTTTTTTGGCGGGAGGGAGA
CCTGAGTTTTATTATTA / Celera SNP ID: hCV2456729 / Public SNP ID: rs12143708 /
SNP Chromosome Position: 169406169 / SNP in Genomic Sequence: SEQ ID NO: 385 /
SNP Position Genomic: 52055 / Related Interrogated SNP: hCV8919444 / SNP
Source: dbSNP; Celera; HapMap / Population(Allele,Count): Caucasian
(T,83|G,37) / SNP Type: INTRON /
Context (SEQ ID NO: 1850): /
TCTCAAAGGTTGACAGACTGACATGCAGAAAACAAAAAATAAAAAGAATGAAAAGCAAAGACGACCTAGACTAAAAGAAAATT
TTTTTGTCTCTTCTAAC
R
GCAAGCAGAGACAAACATTAAATTGCAATATCGTACAATCATTCACCATCTGTAATTCACAATCTATAGTGACAATCTTGAGC
TTCTAAAAAAGGTTGAT / Celera SNP ID: hCV2456730 / Public SNP ID: rs961404 /
SNP Chromosome Position: 169405850 / SNP in Genomic Sequence: SEQ ID NO: 385 /
SNP Position Genomic: 51736 / Related Interrogated SNP: hCV11975250 / SNP

Source: dbSNP; Celera; HapMap; HGBASE / Population(Allele,Count): Caucasian (G,94|A,132) / SNP Type: INTRON /
Context (SEQ ID NO: 1851): /
CAGTATGGCAATTCCTCAAAAAAATTAAAAAATTGAATTACCATATGATCCAGCAATCCCATTTCCGAGCATATACCCAAAATAA
TCACAAGCAGGATCTTG
R
AAAAAATATTTGTACAGTCATGTTCATAGAAGCATTCTTCACAATTGCTAAAATGTTGAAGCAACCCAAGTGACCATTGACAG
ATGAATGGATAAGCAAA / Celera SNP ID: hCV2456733 / Public SNP ID: rs12021580 /
SNP Chromosome Position: 169403703 / SNP in Genomic Sequence: SEQ ID NO: 385 /
SNP Position Genomic: 49589 / Related Interrogated SNP: hCV11975250 / SNP
Source: dbSNP; Celera; HapMap / Population(Allele,Count): Caucasian
(A,130|G,96) / SNP Type: INTRON /
Context (SEQ ID NO: 1852): /
TGGAAATATCAATAAATTGAAAACTAAAAAAGAAATCAAAGAAATTCTAGAGCTGAAAAGTACAACTGAAATGAAAATCTTAC
TGTTGGGATTCAAAAGC
R
TATTTGGTCAGGCAGAAGAAAGAATCAGTGAACTTAAAGATAAGACAATTGAAATTGTTGTATCTGAGAAACAAAAAAATTAA
AAAGTTAAAAGTGAAGA / Celera SNP ID: hCV2456735 / Public SNP ID: rs7522896 /
SNP Chromosome Position: 169401469 / SNP in Genomic Sequence: SEQ ID NO: 385 /
SNP Position Genomic: 47355 / SNP Source: dbSNP; Celera; HapMap /
Population(Allele,Count): Caucasian (A,78|G,32) / SNP Type: INTRON /
Context (SEQ ID NO: 1853): /
CCAAGAGAGGCAATGTAAAATTAAATTTTTAGATATTCTTCAAGTTATAGACTTAAGGTCTTTTAAAATGGAAGGTTAAAATG
AATTGTAATATTGAGTG
Y
ACTTCCTAGATGTGGTGTAGTTGTAATGACCTAATGTTGCTACTGCTTCAGCGCTTGAAAAAAGACGCTCCCAAAAAAGGTTA
TTCACTGCTAAAGGTTG / Celera SNP ID: hCV2456741 / Public SNP ID: rs6696810 /
SNP Chromosome Position: 169397484 / SNP in Genomic Sequence: SEQ ID NO: 385 /
SNP Position Genomic: 43370 / Related Interrogated SNP: hCV11975250 / SNP
Source: dbSNP; Celera / Population(Allele,Count): Caucasian (C,94|T,132) /
SNP Type: INTRON /
Context (SEQ ID NO: 1854): /
GGCATTTAGATACAAACAGGTCTAATATTTAAGAAATGAAAAGATGAATATAAAAAAGGTCTAATATGTAAGAAATGAACAGG
TGAATATAATGAATAAA
M
AGAGAATCTCAGTAAAGAAATGGAAATTTAAAAAAAATAAACTGATAGGAGGATCCAAGATGGCCAAATAGGAACAGCTCCAGT
CTATAGCTCCCAGCGTG / Celera SNP ID: hCV2456767 / Public SNP ID: rs2014061 /
SNP Chromosome Position: 169369883 / SNP in Genomic Sequence: SEQ ID NO: 385 /
SNP Position Genomic: 15769 / Related Interrogated SNP: hCV8919444 / SNP
Source: dbSNP; HapMap; HGBASE / Population(Allele,Count): Caucasian
(A,53|C,65) / SNP Type: TRANSCRIPTION FACTOR BINDING SITE;INTRON /
Context (SEQ ID NO: 1855): /
GGCAAGGTAGAAGGGGGCTGGGTTATTAGGCTTTTGTAACAGCCCAGCCAACAGAAGAAAATAGTGGTTTAGATTAGGGTAGT
GGGACTCTGTATATGCA
S
TGAAGATAGAACCAACATAATTTGCTCAAAAAATTGAATGGATAGTTTTATGTCATGCTTTAAATGATTATTTTTTAAATTATT
CCAGGTTTATTAGTATA / Celera SNP ID: hCV2456768 / Public SNP ID: rs6427186 /
SNP Chromosome Position: 169368562 / SNP in Genomic Sequence: SEQ ID NO: 385 /
SNP Position Genomic: 14448 / Related Interrogated SNP: hCV11975250 / SNP
Source: dbSNP; Celera / Population(Allele,Count): Caucasian (C,94|G,132) /
SNP Type: INTRON /
Context (SEQ ID NO: 1856): /
CAGGTTTGCCTTAAATTACCAAAATATGTACCCAAATCTCCTTTGGTCTATACAGATTTTCCTCCTTAGTCCACCTGCCTTTC
TTTTACTGGTAAATGCA
R
CTTTTATTGGTTGCATTTATTTGTAAATTCAGTTTTTTCTGAGGGCTATGTAGGAGAAGCTGTAACCAATCTTCAACCCATTTC
TCACAGAAATAAGTAAA / Celera SNP ID: hCV2456774 / Public SNP ID: rs1014965 /
SNP Chromosome Position: 169366076 / SNP in Genomic Sequence: SEQ ID NO: 385 /
SNP Position Genomic: 11962 / Related Interrogated SNP: hCV8919444 / SNP
Source: dbSNP; HapMap / Population(Allele,Count): Caucasian (A,80|G,34) / SNP
Type: UTR3;INTRON /
Context (SEQ ID NO: 1857): /
AGAAGAAGGAAGGAAATTAAGATTAGAGTGCAGACAAATAGAGAATAGAAAAACAAGAGAGATCAAAGAAGTCAACAGTTAGT

TCTTTAAAAAGAAAGAC
Y
AAATTGACAAATCTGTAGCCATACTGACTAAGAAAAATAAAACAGGTAAAACTAAAATCAGAAATGAAAGTGGGGACATTATT
AGTCACCTTACATAAAG / Celera SNP ID: hCV2456776 / Public SNP ID: rs6669741 /
SNP Chromosome Position: 169359654 / SNP in Genomic Sequence: SEQ ID NO: 385 /
SNP Position Genomic: 5540 / Related Interrogated SNP: hCV8919444 / SNP
Source: dbSNP; Celera / Population(Allele,Count): Caucasian (C,54|T,66) / SNP
Type: INTRON /
Context (SEQ ID NO: 1858): /
TCTCCTTTTCTATTCTCTTTGTTGGGAAATTGTGTCACTGCCTACTTAGTTGCTGCCAGTAGCATTTGGTATAGTTGACCACT
CCCTCTTTCTCGAACTA
Y
TCTTTCCGAGGTTTATATAACTTTATAGCTTACAGGCTTTTCTCTTTTATCCAACCATTTTATTTCACTATACAATGTTAGGT
ATCCTCAGGGCTTGATT / Celera SNP ID: hCV2456780 / Public SNP ID: rs7534737 /
SNP Chromosome Position: 169354336 / SNP in Genomic Sequence: SEQ ID NO: 385 /
SNP Position Genomic: 222 / Related Interrogated SNP: hCV8919444 / SNP
Source: dbSNP; Celera / Population(Allele,Count): Caucasian (T,54|C,66) / SNP
Type: INTRON /
Context (SEQ ID NO: 1859): /
AGTTTTGGAGACATTGGAGTTCCAATCCAACTATAGTGGGAATACTTCATTGCTTACCTTGGTCAACCACTTACAACCCTAAA
AAGGCCATGCTTTGGAA
R
TAAGGGTCATGTCCTAGAGTAAGGACCATGCTCTAGGACTAAGGACAAAAGCAAAATAAACCCATCCTAACAAAGCATAAAAC
AAAGTCTGACAGGATCA / Celera SNP ID: hCV8690976 / Public SNP ID: rs1124843 /
SNP Chromosome Position: 169369458 / SNP in Genomic Sequence: SEQ ID NO: 385 /
SNP Position Genomic: 15344 / Related Interrogated SNP: hCV11975250 / SNP
Source: dbSNP; Celera; HapMap; ABI_Val; HGBASE / Population(Allele,Count):
Caucasian (A,81|G,145) / SNP Type: INTRON /
Context (SEQ ID NO: 1860): /
ATATTTTTGACCCTCAGCTTCTTTTTTTTCCTAATCAAAACCAAAACTTTACCCAAGAGAGTTGCCAAAGAAAATAGGATTTT
GCTCTAGCTTTGAGGCA
Y
GTCAAAATGGTCAACTAGAATTTTCTCAAATTCTGCTTATCTTTATGTATAAAGGCAAAGCAAGATGAATTTAGATTGTGGTG
AAAGTTGAGATGATTTT / Celera SNP ID: hCV8697031 / Public SNP ID: rs1400836 /
SNP Chromosome Position: 169397137 / SNP in Genomic Sequence: SEQ ID NO: 385 /
SNP Position Genomic: 43023 / Related Interrogated SNP: hCV11975250 / SNP
Source: dbSNP; HapMap; ABI_Val; HGBASE / Population(Allele,Count): Caucasian
(T,81|C,145) / SNP Type: INTRON /
Context (SEQ ID NO: 1861): /
TGAAAGCAATAATGCAAAGCTAATACAGTTTGTGAACTAAGCTTGAGCCATTTTTAGGACAGTCTTTCACAGTCCTTTCACTT
ACTTTAGTCTCATAAAC
Y
CAGGTCTCTGAATTCCCAGCCCAGAAATTAGGACGTGATAAATTTCAAAAAAGAAAAACAGTTGTGTTGTTAAAAGTAGGTACT
CTGACACCAGAAAAAAA / Celera SNP ID: hCV8697038 / Public SNP ID: rs961403 /
SNP Chromosome Position: 169405366 / SNP in Genomic Sequence: SEQ ID NO: 385 /
SNP Position Genomic: 51252 / Related Interrogated SNP: hCV8919444 / SNP
Source: dbSNP; Celera; HapMap; ABI_Val; HGBASE / Population(Allele,Count):
Caucasian (C,54|T,66) / SNP Type: INTRON /
Context (SEQ ID NO: 1862): /
TAAATGTCAAAGATGTGGAGAAATTGGAACCCCTATGCATTGTTTGTGGGAATGTAAAATGTTGAAAACAGTGTGGCAGATCC
TCAAAATATTAAACATA
S
AGTTACCATATGGCCTAGCAATTCTGCTTCTAGGTAGATTCCCAAAAGAAAGCAGAAACTAGAATAGGTATTTGTACAACAGT
GTTCACAGCTGCATTAT / Celera SNP ID: hCV27242533 / Public SNP ID: rs2138898 /
SNP Chromosome Position: 169358811 / SNP in Genomic Sequence: SEQ ID NO: 385 /
SNP Position Genomic: 4697 / Related Interrogated SNP: hCV8919444 / SNP
Source: dbSNP; Celera; HapMap; ABI_Val; HGBASE / Population(Allele,Count):
Caucasian (G,53|C,67) / SNP Type: MISSENSE MUTATION;INTRON /
Context (SEQ ID NO: 1863): /
ACTCTCACACACTGTTGGAGGAACTATAAATTCATATTATCTTTTGGGAGGTTACTTTTGCATCTTTAACGAAACATTTCACT
CTAAGCCTTCAAACATT
W
TAAATTCACCTCTGAAGAACTTCTCCTACAGATTACTGATGTTCCCATAGAATGTACCTCTTCACTACAGCACTGTTAACTAA

CGCAACATTGGAACCAA / Celera SNP ID: hCV27242639 / Public SNP ID: rs7544221 /
SNP Chromosome Position: 169387381 / SNP in Genomic Sequence: SEQ ID NO: 385 /
SNP Position Genomic: 33267 / Related Interrogated SNP: hCV11975250 / SNP
Source: dbSNP; Celera; HapMap / Population(Allele,Count): Caucasian
(T,82|A,142) / SNP Type: TRANSCRIPTION FACTOR BINDING SITE;INTRON /
Context (SEQ ID NO: 1864): /
TATATCCCGCACCTGGCTCAGAGGGTCCCACTCCCACAGAGCCTCACTCACTGCTAACACAGCAGTCTGAGATCAAACTGCAA
TGCCACAGCAAGGCTAG
R
GGAGAGATGTCTGCCATTGCTGAGGCTTAAGTAGGTAAACAAAGCAGCTGGGAAGCTCCAACTGGGTGGAGCTGACCACAGCT
CAAGGAGGCCTGCCTGC / Celera SNP ID: hCV27928247 / Public SNP ID: rs4656182 /
SNP Chromosome Position: 169370336 / SNP in Genomic Sequence: SEQ ID NO: 385 /
SNP Position Genomic: 16222 / Related Interrogated SNP: hCV8919444 / SNP
Source: dbSNP; HapMap / Population(Allele,Count): Caucasian (G,83|A,37) / SNP
Type: INTRON /
Context (SEQ ID NO: 1865): /
GTTAGAAAGACAGAGTATAGCAGCCCAACCCAGAAAATATGGGCATAAAAAAGCAAAGCCTTCCCCTGAAACCAACTAAAACA
TGTTAAAAGCCTTAGTA
S
TTGAAGACCATGCCTTGAGCCAAGTTAGTTATAGGATACAATAAAAGTGTATTTCAGAACAAAGAGTGTGTGACATTCAGAAA
AGAGAATCCAAATGAAA / Celera SNP ID: hCV27972646 / Public SNP ID: rs4656677 /
SNP Chromosome Position: 169389194 / SNP in Genomic Sequence: SEQ ID NO: 385 /
SNP Position Genomic: 35080 / Related Interrogated SNP: hCV8919444 / SNP
Source: dbSNP; HapMap; HGBASE / Population(Allele,Count): Caucasian
(C,146|G,80) / SNP Type: INTRON /
Context (SEQ ID NO: 1866): /
ATCTTGCTGGATATTTTATATGAACAGTGTTAATTTAGATGCACTAAAGCAAAGGTAGGCAAACTACAACCATGAGTCAAACA
TGGCCACACCCATTCAT
K
TGCTATTGTCTAAGCTGGTTTTGCACTACAACTGCAGAGTTGAATAGATGCAGCAGATCCTTTACAGAAAAAGTTTTCTGACC
TCAATTCTAAAGTAATT / Celera SNP ID: hCV29397237 / Public SNP ID: rs6427185 /
SNP Chromosome Position: 169356833 / SNP in Genomic Sequence: SEQ ID NO: 385 /
SNP Position Genomic: 2719 / Related Interrogated SNP: hCV11975250 / SNP
Source: dbSNP; HapMap / Population(Allele,Count): Caucasian (G,81|T,145) /
SNP Type: TRANSCRIPTION FACTOR BINDING SITE;UTR3;INTRON /
Context (SEQ ID NO: 1867): /
AGATCAGATGAGCCAGTTTATTGATCTGAGTGGTGCCAGCTGATCCATCAAGTGCAGGGTCTGCAAAATGTCTCAGGTACTGA
TCTTAAGGGCAGTGTAT
Y
CTTAAATATTGTAGCCTCTAGCTGCATGATTCCTAAACCATAATTTCTAATCCTGTGGCTAATGTTAGTCCTACAAAGGCAGT
CTAGCGCCCAGGCAAGA / Celera SNP ID: hCV30577322 / Public SNP ID: rs7516248 /
SNP Chromosome Position: 169406533 / SNP in Genomic Sequence: SEQ ID NO: 385 /
SNP Position Genomic: 52419 / Related Interrogated SNP: hCV8919444 / SNP
Source: dbSNP; HapMap / Population(Allele,Count): Caucasian (C,54|T,66) / SNP
Type: INTRON /
Context (SEQ ID NO: 1868): /
CTATTTACTATGTACAAATACAGCACAATTATGTGCAAATATTGCACAATATACTGTACATTTATTTTTGCAAATACCCCATT
TAAAAAATTATAATACT
Y
GGAATGCCATGAGGCCTGAAGGCCCATCATCGTCCAAGAAGCTATCAGCAGTATGGCCATAGCTGTGTTTTCCGTGGTCACTG
ACTGAAACATAAACTCC / Celera SNP ID: hCV32141337 / Public SNP ID: rs10919164 /
SNP Chromosome Position: 169384437 / SNP in Genomic Sequence: SEQ ID NO: 385 /
SNP Position Genomic: 30323 / Related Interrogated SNP: hCV8919444 / SNP
Source: dbSNP; HapMap / Population(Allele,Count): Caucasian (T,53|C,63) / SNP
Type: INTRON /
Context (SEQ ID NO: 1869): /
AATCCTTTACAGACAAGCAAATGCTGAGAGATTTTGTCACCACCAGGCCTGCCTTACAAGAGCTCCTGAAGGAAGGACTAAAT
ATGTAAAGGAAAAACAG
R
TACCAACCACTGCCGAAACATGCCAATTGTAAAGACCATTGACACTATGAAGAAACTGCATCAACTAACAGGCAAAATAACCA
ACTAGCATCATAATGAC / Celera SNP ID: hCV32141333 / Public SNP ID: rs10800446 /
SNP Chromosome Position: 169379150 / SNP in Genomic Sequence: SEQ ID NO: 385 /
SNP Position Genomic: 25036 / Related Interrogated SNP: hCV8919444 / SNP

Source: dbSNP; HapMap / Population(Allele,Count): Caucasian (A,54|G,64) / SNP Type: TRANSCRIPTION FACTOR BINDING SITE;INTRON //

Gene Number: 49 / Gene Symbol: CFHR5 - 81494 / Gene Name: complement factor H-related 5 / Chromosome: 1 / OMIM NUMBER: / OMIM Information: // Genomic Sequence (SEQ ID NO: 386): // / SNP Information /
Context (SEQ ID NO: 1870): /
AGCTTCCCCTTAGTACATTGAAATTCAAAGTCATGCTTGTAACTGTTAATGAAAGCAGATTTAAAGCAACACCACCATCACTG
GAGTATTTTTAGTTATA
Y
ACGATTGAGACTACCAAGCATGTTGCTCTTATTCAGTGTAATCCTAATCTCATGGGTATCCACTGTTGGGGGAGAAGGTAAGT
TGAAAACAGATCCGAAT / Celera SNP ID: hCV9114656 / Public SNP ID: rs9427662 /
SNP Chromosome Position: 196946775 / SNP in Genomic Sequence: SEQ ID NO: 386 /
SNP Position Genomic: 10108 / Related Interrogated SNP: hCV2532034 / SNP
Source: Applera / Population(Allele,Count): Caucasian (C,4|T,34) African
American (C,10|T,28) total (C,14|T,62) / SNP Type: MISSENSE MUTATION;UTR5 /
SNP Source: dbSNP; Celera; HapMap / Population(Allele,Count): Caucasian
(T,207|C,19) / SNP Type: MISSENSE MUTATION;UTR5 /
Context (SEQ ID NO: 1871): /
GTGAATATACCAAGACCCTCACATAGCCATTCTCGAAGTCCCACCTGAACAAAATGATTTTGAATTATAATTCTGTTCTTCAA
ACATTTCAGGAAACGTT
W
CTTGGCAAAAATTATTCTTTTTTTTTTTTTTTTTTTTTTTTTTTTGAGACAGAGTCTCGCTCTGTCGCCCAGGCTGGAGTGCAGTGGC
GCGATCTCCACTCACGG / Celera SNP ID: hCV1648949 / Public SNP ID: rs6692162 /
SNP Chromosome Position: 196945912 / SNP in Genomic Sequence: SEQ ID NO: 386 /
SNP Position Genomic: 9245 / Related Interrogated SNP: hCV2532034 / SNP
Source: dbSNP; Celera; HapMap / Population(Allele,Count): Caucasian
(A,111|T,9) / SNP Type: INTERGENIC;UNKNOWN /
Context (SEQ ID NO: 1872): /
TTAATTCTCTCCGTTCTCTAGTTTGCTAAGGGTAAATGGTCACCAAATAGATAATGTAAAGAGAAATCAAAGGAGAGAAAATC
TGCTATAATAAAGATGA
Y
AAGGTGAAATATTAGTGTAATTCTGCATAAACTCTGCAATGCCACTGACTAGAAAAATCAACCCCAAAAGATTTGGCCACCTT
CATTATGTTTTGAGAAT / Celera SNP ID: hCV2759661 / Public SNP ID: rs12731209 /
SNP Chromosome Position: 196947139 / SNP in Genomic Sequence: SEQ ID NO: 386 /
SNP Position Genomic: 10472 / Related Interrogated SNP: hCV2532034 / SNP
Source: dbSNP; Celera; HapMap / Population(Allele,Count): Caucasian
(C,109|T,9) / SNP Type: TRANSCRIPTION FACTOR BINDING SITE;INTRON /
Context (SEQ ID NO: 1873): /
GGCCACCTTCATTATGTTTTGAGAATTTTATTTTCATAGATTTGATAATTTACATTTTAAATGTTATTAAAATGAAGGAATTC
TCATTTCAGCCTAATTA
Y
CATTCAACAGGGAAATATATTTTAATGAAACAATAGAAAATATTATTTTTATATATAAACCAGTGAACTGCCAAAAAATTGTT
TCTTCTGAGCTCTTCCA / Celera SNP ID: hCV2759662 / Public SNP ID: rs1170880 /
SNP Chromosome Position: 196947314 / SNP in Genomic Sequence: SEQ ID NO: 386 /
SNP Position Genomic: 10647 / Related Interrogated SNP: hCV2532034 / SNP
Source: dbSNP; Celera; HapMap; ABI_Val; HGBASE / Population(Allele,Count):
Caucasian (T,204|C,18) / SNP Type: INTRON /
Context (SEQ ID NO: 1874): /
AAAGAGAAATTTCCCTTGATTTGTCAGCTGAATTTCTGCATGCTTTTTCTAAATAAGGTGAAAAACAAAACAATTTCTTATGC
AGCAATGCTATATTCAG
R
AAATATCTTGAGCTAAAAAATGAGCACACTATATTGAGGAAATATCTCGAGCTAAAACAGCATTCAAAGATAAAGTACATCAA
ATCCATAGAATGTACAA / Celera SNP ID: hCV2759663 / Public SNP ID: rs928440 /
SNP Chromosome Position: 196950350 / SNP in Genomic Sequence: SEQ ID NO: 386 /
SNP Position Genomic: 13683 / Related Interrogated SNP: hCV2532034 / SNP
Source: dbSNP; Celera; HapMap; ABI_Val; HGBASE / Population(Allele,Count):
Caucasian (G,111|A,9) / SNP Type: INTRON /
Context (SEQ ID NO: 1875): /
TATTGAGGAAATATCTCGAGCTAAAACAGCATTCAAAGATAAAGTACATCAAATCCATAGAATGTACAATATCAAGAGTAAAC
CCTAATGTAAACTATGG
R
CTTTAATGACAATAATACTGATAGTGAAGGGGGCTATTCATGTGGCAGGGAAGAGGAAGTGGTATATAGGAAATCTCTGTACC

TTCCACTCAATTTTACA / Celera SNP ID: hCV2759664 / Public SNP ID: rs928439 / SNP Chromosome Position: 196950482 / SNP in Genomic Sequence: SEQ ID NO: 386 / SNP Position Genomic: 13815 / Related Interrogated SNP: hCV2532034 / SNP Source: dbSNP; Celera; HapMap; HGBASE / Population(Allele,Count): Caucasian (G,109|A,9) / SNP Type: INTRON /
Context (SEQ ID NO: 1876): /
ACAGAAGACTTCCATGATAAAATGGAAATGATTCTTATAGGATCTTGCTACTTGGGGAGCACAAGGAGGAGATTCTAAGAATC
AGGGAGCTATTTTTTCC
Y
CTAGGACTGACTTTTTCCCCTGTGATTCAATTCCCACTTGACTTATGGATGACAATTTAAAGGTGAACAAACGACATCCTGTT
TAGAAGCTGCTACTCTG / Celera SNP ID: hCV2759725 / Public SNP ID: rs6670056 / SNP Chromosome Position: 196982776 / SNP in Genomic Sequence: SEQ ID NO: 386 / SNP Position Genomic: 46109 / Related Interrogated SNP: hCV2532034 / SNP Source: dbSNP; Celera / Population(Allele,Count): Caucasian (T,9|C,111) / SNP Type: INTERGENIC;UNKNOWN /
Context (SEQ ID NO: 1877): /
TTTGTTAAAAGCAGACACAAACACACACATTAACCTCGGCCTACGGAGGGTCAGGATCATCAGTATCACTGTCTTCCTCCTCA
CTCTTGTTCCACTAGAA
R
GTCTTTAGGGCAGTAACATGCATGGAGCTGTCCTCTCCTATGATAACAAAGCCTTCTTCTGGAATAGTTCCTGAAGGACCCGC
CTGAGGATGTTTTACAG / Celera SNP ID: hCV8356520 / Public SNP ID: rs1170881 / SNP Chromosome Position: 196949271 / SNP in Genomic Sequence: SEQ ID NO: 386 / SNP Position Genomic: 12604 / Related Interrogated SNP: hCV2532034 / SNP Source: dbSNP; HapMap; HGBASE / Population(Allele,Count): Caucasian (G,111|A,9) / SNP Type: INTRON /
Context (SEQ ID NO: 1878): /
TATATAACACTCAAAATGACTAAATTACAGTTATGTAGAACATACTAGTGATGACTAGCAATGAGGGGTGGGGGAATGTGGGG
ACCATAAAAGGACAGCA
Y
ACGGGAGTTTTCTGGAGGTGATAGAATAGTTCTGTATACGACTGAGTAGTTCTACATATGACTGATTGTGGTGATGGTTACAT
GAGTGCATACATGTTGC / Celera SNP ID: hCV9114658 / Public SNP ID: rs13376702 / SNP Chromosome Position: 196942621 / SNP in Genomic Sequence: SEQ ID NO: 386 / SNP Position Genomic: 5954 / Related Interrogated SNP: hCV2532034 / SNP Source: dbSNP; Celera; HapMap / Population(Allele,Count): Caucasian (C,202|T,22) / SNP Type: INTERGENIC;UNKNOWN /
Context (SEQ ID NO: 1879): /
AATTTTATGGAGACTTTCTGGAAAAAAATATTTCCCAACATTATGCTGTTTGGCCAATTTCCAGAGACAACCAATGGTTACTTT
TAAATTTTTATCCAGTT
K
TATTGCTGCCTTTTGGTTGGGAGTGAATATACCAAGACCCTCACATAGCCATTCTCGAAGTCCCACCTGAACAAAATGATTTT
GAATTATAATTCTGTTC / Celera SNP ID: hCV11888484 / Public SNP ID: rs6694672 / SNP Chromosome Position: 196945789 / SNP in Genomic Sequence: SEQ ID NO: 386 / SNP Position Genomic: 9122 / Related Interrogated SNP: hCV2532034 / SNP Source: dbSNP; Celera; HapMap / Population(Allele,Count): Caucasian (T,207|G,19) / SNP Type: INTERGENIC;UNKNOWN /
Context (SEQ ID NO: 1880): /
ACATTTTCAGCATGTTTGTAGGTTGGTGAATGATTAAGTGGAAAGGGAGACATTGATGTTACAGAAGAAAGGAGATAATTCAT
GAAGAAGGATCTTTTAC
W
AGTTTAAAGGTGAAAGAATCTAGTGTCATCATGAAGAAGTCAATCTGAGAAAGCAAGGACAGTAACTCAATGAAACTAGAGGA
TGTACAAGCAATTTGGG / Celera SNP ID: hCV11888496 / Public SNP ID: rs7554757 / SNP Chromosome Position: 196973054 / SNP in Genomic Sequence: SEQ ID NO: 386 / SNP Position Genomic: 36387 / Related Interrogated SNP: hCV2532034 / SNP Source: dbSNP; Celera; HapMap / Population(Allele,Count): Caucasian (A,6|T,104) / SNP Type: INTRON /
Context (SEQ ID NO: 1881): /
CAGGTAGAGAATTTATTTGCAAAGGATTGAGGTTTTCTAAGACTTGGATTACAGATAGAAAGGAACGAATGGGCAGGAGAGTG
TGTGCAAGGGACTGACT
K
ACAGTGATGAGCCATGGAATCTAATTTAGATGTGGTGGGAAGAGAGAAAATGAAGGGAAGAGAATAGTGAAAAAGTGTCAGGG
TCATTGAATGTTTGGAA / Celera SNP ID: hCV30373286 / Public SNP ID: rs6702340 / SNP Chromosome Position: 196945050 / SNP in Genomic Sequence: SEQ ID NO: 386 / SNP Position Genomic: 8383 / Related Interrogated SNP: hCV2532034 / SNP

349

Source: dbSNP; HapMap / Population(Allele,Count): Caucasian (G,111|T,9) / SNP Type: INTERGENIC;UNKNOWN /
Context (SEQ ID NO: 1882): /
TATTTTTATGACTTATTATATGAATCTAAGATTATTTCAAAACAAAAATTATTTGGATTATATAGGCTGGGATGTTTTACTTT TATAGCACTTTTGGGGA
W
GAGAATTTTCAATATTCAAGAGTTATAACCAATTTCAGCAATTTTTCTTTGAGATACTTAAATCTATCTCAAATTTTATCAGT AATAACTTCTACAAGCC / Celera SNP ID: hCV30391085 / Public SNP ID: rs9427656 /
SNP Chromosome Position: 196943020 / SNP in Genomic Sequence: SEQ ID NO: 386 /
SNP Position Genomic: 6353 / Related Interrogated SNP: hCV2532034 / SNP Source: dbSNP; HapMap / Population(Allele,Count): Caucasian (A,107|T,13) /
SNP Type: INTERGENIC;UNKNOWN /
Context (SEQ ID NO: 1883): /
TTTAAAAGGGAATTTACAACTACAAAAGTGATATTGTTGTATTAAGATGAGGACTGATGACTTGCACTTGCTTGCCTTTTGAA ACAGCTGAACCCTTGGC
W
AGAAAAAAAAAAAAAAACCCAGGGGGCCGGCGGTGGGGAGCTTCCTCTCTCCTCTAAACAGTCCATTTCTGATTGTTCCAAACT CAGAGACTCGGTGACTA / Celera SNP ID: hCV29796191 / Public SNP ID: rs9427660 /
SNP Chromosome Position: 196946420 / SNP in Genomic Sequence: SEQ ID NO: 386 /
SNP Position Genomic: 9753 / Related Interrogated SNP: hCV2532034 / SNP Source: dbSNP; HapMap / Population(Allele,Count): Caucasian (T,109|A,9) / SNP Type: INTERGENIC;UNKNOWN /
Context (SEQ ID NO: 1884): /
CCGGCGGTGGGGAGCTTCCTCTCTCCTCTAAACAGTCCATTTCTGATTGTTCCAAACTCAGAGACTCGGTGACTACAACAAGG AAATTGAACAGCAGTTA
Y
TTTGCTTAAATAAATATTACATAACGAGCTGAGAATATTATTGTGAAGACACTGATTAGATACTATTTGCTTATTTCACACAA CCCTCCATGAACTTTGA / Celera SNP ID: hCV29724082 / Public SNP ID: rs9427661 /
SNP Chromosome Position: 196946546 / SNP in Genomic Sequence: SEQ ID NO: 386 /
SNP Position Genomic: 9879 / Related Interrogated SNP: hCV2532034 / SNP Source: dbSNP; HapMap / Population(Allele,Count): Caucasian (T,207|C,19) /
SNP Type: INTERGENIC;UNKNOWN /
Context (SEQ ID NO: 1885): /
TGATATTGTTGTATTAAGATGAGGACTGATGACTTGCACTTGCTTGCCTTTTGAAACAGCTGAACCCTTGGCTAGAAAAAAAA AAAAAACCCAGGGGGCC
R
GCGGTGGGGAGCTTCCTCTCTCCTCTAAACAGTCCATTTCTGATTGTTCCAAACTCAGAGACTCGGTGACTACAACAAGGAAA TTGAACAGCAGTTATTT / Celera SNP ID: hCV30391086 / Public SNP ID: rs9427942 /
SNP Chromosome Position: 196946448 / SNP in Genomic Sequence: SEQ ID NO: 386 /
SNP Position Genomic: 9781 / Related Interrogated SNP: hCV2532034 / SNP Source: dbSNP; HapMap / Population(Allele,Count): Caucasian (G,206|A,18) /
SNP Type: INTERGENIC;UNKNOWN /
Context (SEQ ID NO: 1886): /
TCTGACTTACTGAGTCAGAGTTTTTGCTGAGCTTTTGAAATGTGAGTCATTGAACCTCAAGGGGGACCCAGATCTATCTCTTA CTGATGCTTTGCTTCCT
R
CAGACTTACCCATGTATTGCCTCTTTTTATTATTTAAATGTTTTGAAGATATTTAAGATAGTTTAGTGCAAAACAATCATACA TGTGTACATTTTCAGAG / Celera SNP ID: hCV31565509 / Public SNP ID: rs12092294 /
SNP Chromosome Position: 196966853 / SNP in Genomic Sequence: SEQ ID NO: 386 /
SNP Position Genomic: 30186 / Related Interrogated SNP: hCV2532034 / SNP Source: dbSNP; HapMap / Population(Allele,Count): Caucasian (G,19|A,207) /
SNP Type: INTRON /
Context (SEQ ID NO: 1887): /
ACCTGGAATCCACAAAAAAAAGAGGTCCCAGTTTCCTAGATTTATAATGCTGTGTAAGAAACTTATTGGCATAAAACAGCAAT CATTTTATTATACTTAA
R
GATACTGTTAGGAATTGACGCAGGGCACTGAGGTGATGACTTATAACTGTTCCACAATATTTGAGCCTCAGGTGAAAAGATTT AAATGGGTTAGGACTCA / Celera SNP ID: hCV30589276 / Public SNP ID: rs9427657 /
SNP Chromosome Position: 196943321 / SNP in Genomic Sequence: SEQ ID NO: 386 /
SNP Position Genomic: 6654 / Related Interrogated SNP: hCV2532034 / SNP Source: dbSNP; HapMap / Population(Allele,Count): Caucasian (A,203|G,23) /
SNP Type: INTERGENIC;UNKNOWN /
Context (SEQ ID NO: 1888): /

TTCCTAGATTTATAATGCTGTGTAAGAAACTTATTGGCATAAAACAGCAATCATTTTATTATACTTAAAGATACTGTTAGGAA
TTGACGCAGGGCACTGA
K
GTGATGACTTATAACTGTTCCACAATATTTGAGCCTCAGGTGAAAAGATTTAAATGGGTTAGGACTCAAATAACTGAGATTTG
GAATCTGCAAATGTCAC / Celera SNP ID: hCV29922678 / Public SNP ID: rs9427940 /
SNP Chromosome Position: 196943353 / SNP in Genomic Sequence: SEQ ID NO: 386 /
SNP Position Genomic: 6686 / Related Interrogated SNP: hCV2532034 / SNP
Source: dbSNP; HapMap / Population(Allele,Count): Caucasian (G,107|T,11) /
SNP Type: INTERGENIC;UNKNOWN //

Gene Number: 50 / Gene Symbol: RNASE7 - 84659 / Gene Name: ribonuclease,
RNase A family, 7 / Chromosome: 14 / OMIM NUMBER: / OMIM Information: //
Genomic Sequence (SEQ ID NO: 387): // / SNP Information /
Context (SEQ ID NO: 1889): /
CTGCCAGACCCCCAAAATAGCCTGCAAGAATGGCGATAAAAACTGCCACCAGAGCCACGGGGCCGTGTCCCTGACCATGTGTA
AGCTCACCTCAGGGAAG
Y
ATCCGAACTGCAGGTACAAAGAGAAGCGACAGAACAAGTCTTACGTAGTGGCCTGTAAGCCTCCCCAGAAAAAGGACTCTCAG
CAATTCCACCTGGTTCC / Celera SNP ID: hCV2434510 / Public SNP ID: rs1243469 /
SNP Chromosome Position: 21511497 / SNP in Genomic Sequence: SEQ ID NO: 387 /
SNP Position Genomic: 11112 / SNP Source: Applera / Population(Allele,Count):
Caucasian (C,1|T,35) African American (C,5|T,31) total (C,6|T,66) / SNP Type:
MISSENSE MUTATION / SNP Source: Applera / Population(Allele,Count):
Caucasian (C,4|T,36) African American (C,5|T,31) total (C,9|T,67) / SNP Type:
MISSENSE MUTATION / SNP Source: dbSNP; Celera; HapMap; ABI_Val; HGBASE /
Population(Allele,Count): Caucasian (C,27|T,199) / SNP Type: MISSENSE
MUTATION //

Gene Number: 51 / Gene Symbol: C3orf15 - 89876 / Gene Name: chromosome 3
open reading frame 15 / Chromosome: 3 / OMIM NUMBER: / OMIM Information: //
Genomic Sequence (SEQ ID NO: 388): // / SNP Information /
Context (SEQ ID NO: 1890): /
TGGGACCCTGGGTCAGCAGAGCTACAGGGACAGGACACTTACCTAGAGCCATGGGGATGGGGCATTGTAGAGTCAAAGGGGCA
GAGCCATGGGAATGATG
Y
TGCCACCCCAGTGGGCTTAGAAAGCAAGACACTAAGCCAAAGAAGACTATACTCAAGAATTAAGATCCAGTGGAATTTGCCTT
GCCAGGTCGTGGACTTG / Celera SNP ID: hCV105917 / Public SNP ID: rs9289134 /
SNP Chromosome Position: 119473719 / SNP in Genomic Sequence: SEQ ID NO: 388 /
SNP Position Genomic: 61850 / Related Interrogated SNP: hCV263841 / SNP
Source: dbSNP; Celera; HapMap; ABI_Val / Population(Allele,Count): Caucasian
(T,168|C,58) / SNP Type: INTRON /
Context (SEQ ID NO: 1891): /
TTTTTTAAAAATAACAACCCTCAAATTCTAATGTGTAATTTGCTAGTTTATTATGTTTGTTGTTTGTTGTCTCTCTCTCATGA
AGTCAAAGATTTTTGTC
R
TTTTGTTGATGTATTCTAAGCACCTAGATAGAGTAGCCAGTCAATAAACATGTGTTGAATGAAGGAGCAAAAAGAGCCTGAGC
CTCTTGCAGCTTTTCAG / Celera SNP ID: hCV134275 / Public SNP ID: rs9847068 /
SNP Chromosome Position: 119424613 / SNP in Genomic Sequence: SEQ ID NO: 388 /
SNP Position Genomic: 12744 / Related Interrogated SNP: hCV263841 / SNP
Source: dbSNP; Celera; HapMap; ABI_Val / Population(Allele,Count): Caucasian
(G,64|A,162) / SNP Type: TRANSCRIPTION FACTOR BINDING SITE;UTR5;INTRON /
Context (SEQ ID NO: 1892): /
CTGATGGTATAATTTTGTCCTTTGCCTCCCTTCTCTAACATTAGAGAAAAGTTCCCAGGTTTAAAACCATGTTCAGTAACCTG
ATCCATTATCCAAGATA
Y
TCTCTATATTGGAGCAAGTCAGATCCTGTCCCACCATTTATCAGTCGGGAATGGAAGGGACATAAGGAGAAACACAGAGAAGC
CCTCCGGCAGCTCACCA / Celera SNP ID: hCV134278 / Public SNP ID: rs9848716 /
SNP Chromosome Position: 119426307 / SNP in Genomic Sequence: SEQ ID NO: 388 /
SNP Position Genomic: 14438 / Related Interrogated SNP: hCV263841 / SNP
Source: dbSNP; Celera; HapMap / Population(Allele,Count): Caucasian
(T,64|C,160) / SNP Type: UTR5;SILENT MUTATION /
Context (SEQ ID NO: 1893): /
CAAGAGTCTCACTTCACAGTTTAAGGACTCACATAGACTGAAAGTGAAAGGATGAAAAAAGATACTCAATGCAAATGGAAACC

AAAATAGAACAAGTGTA
R
CTAAACTTACATCAGATAAAATAGGTTTTAAGTAAAAAACTATAAAATGAGACCAAAAAGGTCATTGTGTGGTGATAAAGGGG
TCGATTCATCAAGAGGC / Celera SNP ID: hCV178227 / Public SNP ID: rs13070374 /
SNP Chromosome Position: 119479079 / SNP in Genomic Sequence: SEQ ID NO: 388 /
SNP Position Genomic: 67210 / Related Interrogated SNP: hCV263841 / SNP
Source: dbSNP; Celera; HapMap / Population(Allele,Count): Caucasian
(G,66|A,52) / SNP Type: INTRON /
Context (SEQ ID NO: 1894): /
TGAAATAGAAACTAGAAAAATAATAGAAAAGATCAACAAAACTAAAATTCGGTATTTTGAAAAGATAAAATTGACAAACCTTT
AACTAGACTACTTAAGG
M
CAAAGAGGGAAGACTTAAGGAAAATCAGAAGTGAAAGAGGAGACATTACAACTGATACCACAGAAATGCAAAAGATAAGACTA
CTATAAACAATTATATA / Celera SNP ID: hCV178228 / Public SNP ID: rs4687882 /
SNP Chromosome Position: 119479987 / SNP in Genomic Sequence: SEQ ID NO: 388 /
SNP Position Genomic: 68118 / Related Interrogated SNP: hCV263841 / SNP
Source: dbSNP; Celera; HapMap / Population(Allele,Count): Caucasian
(C,167|A,59) / SNP Type: INTRON /
Context (SEQ ID NO: 1895): /
ATATCTACCCCCACCACAATGCTATTTAATACTGTATTAATCGTTCCAACTAATGCAATAAGGAAAGAAAAAAAGCATAAAGA
TCAGAAAAGAAGAAAAC
W
GTCTTTCTTTGCAGAAAACATAATTATTTACATTGAACATCCTCAGTAATACTAAGGAATAACTACTAGAACTATTTAATAAA
GTCACAGTTATATCTCA / Celera SNP ID: hCV192027 / Public SNP ID: rs9821892 /
SNP Chromosome Position: 119491949 / SNP in Genomic Sequence: SEQ ID NO: 388 /
SNP Position Genomic: 80080 / Related Interrogated SNP: hCV263841 / SNP
Source: dbSNP; Celera; HapMap; ABI_Val / Population(Allele,Count): Caucasian
(A,84|T,36) / SNP Type: INTERGENIC;UNKNOWN /
Context (SEQ ID NO: 1896): /
GTGTGTTCATGCAACAGCCATAGAGATGGTGCTATTGAAAGGCAATCTGCCCATCCTGTGTATTGACAAGCATCAGGATTCCA
GGAGGATTCTTCCTGAT
K
CAAAGACTGTCAAATAGGAAAAAAGAAAACAGTAAATGAACAAAAACATCAACATGGAGTCCAAGTAATGAATTTATAGAGGA
AAATGTATGCTACTCTG / Celera SNP ID: hCV255886 / Public SNP ID: rs10511394 /
SNP Chromosome Position: 119484601 / SNP in Genomic Sequence: SEQ ID NO: 388 /
SNP Position Genomic: 72732 / Related Interrogated SNP: hCV263841 / SNP
Source: dbSNP; Celera; HapMap / Population(Allele,Count): Caucasian
(T,172|G,54) / SNP Type: MICRORNA;UTR3 /
Context (SEQ ID NO: 1897): /
ATATTGTCTATAAAATGTAAATTCAAGGACCAAAGCATTTGGCTACCTTATTATTGTGATAAAAGCAAGCCTTAAGGTTGGGG
TAGTCATTTTTCTGACA
Y
GCAAATTTAATAAGATTAATAATTTACACAATTTGCATTGAATGCATCTGCCTTTACTTCTGATTATGGTACCTACTCTATGA
CATCCATAGTGACCTGT / Celera SNP ID: hCV278948 / Public SNP ID: rs1464599 /
SNP Chromosome Position: 119471473 / SNP in Genomic Sequence: SEQ ID NO: 388 /
SNP Position Genomic: 59604 / Related Interrogated SNP: hCV263841 / SNP
Source: dbSNP; Celera; HGBASE / Population(Allele,Count): Caucasian
(T,172|C,54) / SNP Type: INTRON /
Context (SEQ ID NO: 1898): /
GATACAGTATAGGCAGATTGTCAGAAGACAAAGAGAATCTTGAGAACAGTGAGAGAGAGGCAACTCATCCTGTACAAGGGAGC
CTTAATAAGATTAACAG
Y
TGATTTCGCATCAGAAACCATGGAGGCCTGAAAACAGGAGGCCTGATGCCACTTCACACCTACTAGGATGGATATAATTTTTT
TTAATGGAAAATAACAA / Celera SNP ID: hCV1833991 / Public SNP ID: rs11926554 /
SNP Chromosome Position: 119495556 / SNP in Genomic Sequence: SEQ ID NO: 388 /
SNP Position Genomic: 83687 / Related Interrogated SNP: hCV263841 / SNP
Source: dbSNP; Celera; HapMap / Population(Allele,Count): Caucasian
(T,73|C,27) / SNP Type: TRANSCRIPTION FACTOR BINDING SITE;INTERGENIC;UNKNOWN /
Context (SEQ ID NO: 1899): /
TTACTTATAATGGCTTGAGTTAAAATGCTCCAACTTTTCTAATTTTTGCTTTTGAAAATAAAACACCTATAGTGACAATAAAA
ACACACATTTAAAGCCA
W
GTGGTCATAGTGACTGGAGCCATGTAGTGAATTGGGGCTAGACTAGTACGATCAATAGCGATACAAAATCACAATGAACTGTC

TGAGGCAGAAAACTTTT / Celera SNP ID: hCV1834237 / Public SNP ID: rs9865270 / SNP Chromosome Position: 119485949 / SNP in Genomic Sequence: SEQ ID NO: 388 / SNP Position Genomic: 74080 / Related Interrogated SNP: hCV263841 / SNP Source: dbSNP; Celera; HapMap / Population(Allele,Count): Caucasian (A,168|T,58) / SNP Type: INTERGENIC;UNKNOWN / Context (SEQ ID NO: 1900): / ACATATCCAGGAAGCTCAACAAAGCCCAAGCAAAATAAACACGAAGAGATACACCAAGATACAGTATAGGCAGATTGTCAGAA GACAAAGAGAATCTTGA R

AACAGTGAGAGAGAGGCAACTCATCCTGTACAAGGGAGCCTTAATAAGATTAACAGTTGATTTCGCATCAGAAACCATGGAGG CCTGAAAACAGGAGGCC / Celera SNP ID: hCV1834240 / Public SNP ID: rs1581451 / SNP Chromosome Position: 119495499 / SNP in Genomic Sequence: SEQ ID NO: 388 / SNP Position Genomic: 83630 / Related Interrogated SNP: hCV263841 / SNP Source: dbSNP; Celera; HapMap; HGBASE / Population(Allele,Count): Caucasian (G,76|A,146) / SNP Type: INTERGENIC;UNKNOWN / Context (SEQ ID NO: 1901): / AGCATCATATCATATGAAAATATATAAAGTTGTAAATTAGGCTAACATACTGTTATATAAAATTTATTCTACCCACCTACTTT CACAAACATATCTTTGA R

ATGATAACATTGAAAAACGGGTAAGCAGCTATGGGCTTTATGTAATTGAAAGATGGCAAGAACCAAAGATGGTAAGCTTAGTT ATTATTGTACATGTCTG / Celera SNP ID: hCV1834242 / Public SNP ID: rs11712308 / SNP Chromosome Position: 119483267 / SNP in Genomic Sequence: SEQ ID NO: 388 / SNP Position Genomic: 71398 / Related Interrogated SNP: hCV263841 / SNP Source: dbSNP; Celera; HapMap / Population(Allele,Count): Caucasian (G,109|A,111) / SNP Type: TRANSCRIPTION FACTOR BINDING SITE;INTRON / Context (SEQ ID NO: 1902): / GGTATTTGCAAGACCCAGGTCAAGAGGATGAATTGAAGCATCATATCATATGAAAATATATAAAGTTGTAAATTAGGCTAACA TACTGTTATATAAAATT K

ATTCTACCCACCTACTTTCACAAACATATCTTTGAGATGATAACATTGAAAAACGGGTAAGCAGCTATGGGCTTTATGTAATT GAAAGATGGCAAGAACC / Celera SNP ID: hCV1834243 / Public SNP ID: rs9682652 / SNP Chromosome Position: 119483231 / SNP in Genomic Sequence: SEQ ID NO: 388 / SNP Position Genomic: 71362 / Related Interrogated SNP: hCV263841 / SNP Source: dbSNP; Celera; HapMap; ABI_Val / Population(Allele,Count): Caucasian (T,85|G,35) / SNP Type: INTRON / Context (SEQ ID NO: 1903): / CTAAGGCTCAAAGAGAAGTATAAGACTAGGTCTTTATATTTGTAAGTTACTTACACTCTAGATATCAAACGAAATGTAACTCA CATGAAGTGCTTCAATG Y

TGAATAGGTTGGTACAAACTCAAGTGCGGTAAGTGGTAAATCTGGGCTGATGTAGTCAGAGGAGACTTCCTGGAGGATCTTGG ACATGAGCTTTCCAGGT / Celera SNP ID: hCV1834256 / Public SNP ID: rs2472662 / SNP Chromosome Position: 119469439 / SNP in Genomic Sequence: SEQ ID NO: 388 / SNP Position Genomic: 57570 / Related Interrogated SNP: hCV263841 / SNP Source: dbSNP; Celera; HGBASE / Population(Allele,Count): Caucasian (C,116|T,110) / SNP Type: INTRON;PSEUDOGENE / Context (SEQ ID NO: 1904): / AAATTCAGCTCTGGTCTTCCCGAGTGTAAAGCCTTAGCCAGATTATTTGTGTTGCTGGAGCAGGCACACTTCACTTAAAAAAT GGACTGATATAAAGCCC Y

AGTTAATTTTTTTTTCCCAGATAGCTATAACTCATAAGAGATTCAGATATGATGAGTAAATCATCTGGATGAACAACTTGTGTT TAGCACTGTTCTTTACT / Celera SNP ID: hCV1834260 / Public SNP ID: rs4688033 / SNP Chromosome Position: 119458851 / SNP in Genomic Sequence: SEQ ID NO: 388 / SNP Position Genomic: 46982 / Related Interrogated SNP: hCV263841 / SNP Source: dbSNP; Celera; HapMap; HGBASE / Population(Allele,Count): Caucasian (C,170|T,54) / SNP Type: INTRON / Context (SEQ ID NO: 1905): / ATTAGAAAGAAGCAGCAAAACTATCCTTATTTGCACACGTGCTCTCTTAGAAAAATAAATTATCAGAACTAAAAAGAGAGTTC AACAAGAAGTCAACATA S

ATCAGTCGCTTTCCTATATGAGAGAAATATCCAATTAGAAAAATTGTTGAGGCCAGGTGCAGTGGCTCACACCTGTAATCCCA GCACTTTGGGATGCCAA / Celera SNP ID: hCV27986929 / Public SNP ID: rs4688030 / SNP Chromosome Position: 119440690 / SNP in Genomic Sequence: SEQ ID NO: 388 / SNP Position Genomic: 28821 / Related Interrogated SNP: hCV30747430 / SNP

Source: dbSNP; HapMap; HGBASE / Population(Allele,Count): Caucasian (C,55|G,171) / SNP Type: INTRON /
Context (SEQ ID NO: 1906): /
GCCTTGGCACTAGTCCTAGCTCTTACCCACCCCCCACCCCGACCCAAGACACTGGGTTCACAGTCAGGATGCCTTGCGTGCTA
GCTAGAGCTGATGGGGC
M
TTTCCTAGTTAAAAGACAAATTATGGAATTCTTCCATTAAGAGATGAGTATATAACAATATACATAAATAACAATATGCTGAG
GTACTTGTGGTTGGGTT / Celera SNP ID: hCV30699687 / Public SNP ID: rs11711386 /
SNP Chromosome Position: 119445735 / SNP in Genomic Sequence: SEQ ID NO: 388 /
SNP Position Genomic: 33866 / Related Interrogated SNP: hCV263841 / SNP
Source: dbSNP; HapMap / Population(Allele,Count): Caucasian (A,116|C,110) /
SNP Type: TFBS SYNONYMOUS;INTRON /
Context (SEQ ID NO: 1907): /
GCCGAGATCGTGCCATTGCACTCCAGCCTGGGTGACAAGAGCAAGACTTCATCTCAAAAAAAAAAAAAAAAGTGCTTGGCATA
ATAAAAATGCTTAAGTA
R
TGTTAGGTTTTATTATTATTATAATTCATAGACACATAAATTTGTATATAAAATGTTAAGATTGAATAGAAAACCACAAATTC
CTTTTTGTGTTTACTTG / Celera SNP ID: hCV29841665 / Public SNP ID: rs7623217 /
SNP Chromosome Position: 119443061 / SNP in Genomic Sequence: SEQ ID NO: 388 /
SNP Position Genomic: 31192 / Related Interrogated SNP: hCV263841 / SNP
Source: dbSNP; HapMap / Population(Allele,Count): Caucasian (A,56|G,62) / SNP
Type: INTRON /
Context (SEQ ID NO: 1908): /
TGTAACTATAAGGTGTTTTATATATTCCTCATGGTAAGTACAAAACAAAAAACCTATATTAAATACACAAAAGGTGAAGAGTA
AGAAATCAAGGCATGGC
R
CTAGAGAAAGTCACCTAATTACAAAGGAATACAGCAAGAGAGGAAGAAAGGAATAAGGACCTACAAAACAACCATAAAACAAT
GAACCAGATGATAGTAG / Celera SNP ID: hCV30562884 / Public SNP ID: rs9815093 /
SNP Chromosome Position: 119478763 / SNP in Genomic Sequence: SEQ ID NO: 388 /
SNP Position Genomic: 66894 / Related Interrogated SNP: hCV263841 / SNP
Source: dbSNP; HapMap; ABI_Val / Population(Allele,Count): Caucasian
(G,168|A,58) / SNP Type: INTRON /
Context (SEQ ID NO: 1909): /
ATATTAAATAAACATCAGTACTGTGTCTTGCTTCTATAGAATTCATTTTTGGTTCATATTTGCTTTTACTAGACAGGAAGTAT
AGTATAGTGCACAAAAA
Y
ACGAGGTTCAGACTGCCATGTGCAAGTTACTTAGCCTTTTAGCCCTTCAGTATCTTTATTTGTAAAACAGGCGTAATTATGTT
TATGTGATAGGGATTTG / Celera SNP ID: hCV29787215 / Public SNP ID: rs9830785 /
SNP Chromosome Position: 119442572 / SNP in Genomic Sequence: SEQ ID NO: 388 /
SNP Position Genomic: 30703 / SNP Source: dbSNP; HapMap /
Population(Allele,Count): Caucasian (T,170|C,56) / SNP Type: INTRON /
Context (SEQ ID NO: 1910): /
TGAGGGCAATTTTAATACATGTATTTTGAGTCCCTTTTACATGCTAGACTCTGTGCTGTGAGTTGGGAAAATAGGGATGAATA
GGAAACTGCTCTTCCTG
R
CAGAGCTCCTTATGGTCCACTGGGGCAGGGGGAGCAGGGAAGGTGGACAAGGAAGGGACAAAAGATCTTAGTTAGATCAACCC
CTCTCAGGCTGTGTCTA / Celera SNP ID: hCV30526128 / Public SNP ID: rs9841230 /
SNP Chromosome Position: 119446441 / SNP in Genomic Sequence: SEQ ID NO: 388 /
SNP Position Genomic: 34572 / Related Interrogated SNP: hCV30747430 / SNP
Source: dbSNP / Population(Allele,Count): Caucasian (A,171|G,55) / SNP Type:
INTRON //

Gene Number: 52 / Gene Symbol: RDH13 - 112724 / Gene Name: retinol
dehydrogenase 13 (all-trans/9-cis) / Chromosome: 19 / OMIM NUMBER: / OMIM
Information: // Genomic Sequence (SEQ ID NO: 389): // / SNP Information /
Context (SEQ ID NO: 1911): /
TGCAGTGAGTCGAAATGGTGCCAGCCTGGGCAACAGAGCGAGACTCTGTCTCAAAAAAATTTAAATTTAAATTTAAAATGCCC
GCTGCACGAGATTCCCA
W
GGCTGCTGTACGCATTACCACAGACTTAGTGGCTTAAAACCACATAAGTGCATCCTCCTCCAGTTCGGCAGGTCAAGAGTCCA
AAACATGTCTCACTGGA / Celera SNP ID: hCV8718961 / Public SNP ID: rs1654451 /
SNP Chromosome Position: 55572240 / SNP in Genomic Sequence: SEQ ID NO: 389 /
SNP Position Genomic: 26548 / SNP Source: dbSNP; HGBASE /

Population(Allele,Count): Caucasian (A,40|T,182) / SNP Type: INTRON /
Context (SEQ ID NO: 1912): /
ACTATATCAGCCTATTCCCTCATCTGGAAATGTGTGTTTACCTTCTTCATAGACTTTTGGAGATAATTTGAGAATTTCCATGC
ACAGAAACAAGGATCTA
R
TAGCCTGTGGGTACCCAAGCTCCTGGGGTCCTGCAGGAGAAGGCGGCTGGGGGCCTGGACTCCTGGGTCTGAGGGAGGAGGGG
CTGGGGGCCTGGACTCC / Celera SNP ID: hCV11977629 / Public SNP ID: rs1654459 /
SNP Chromosome Position: 55574907 / SNP in Genomic Sequence: SEQ ID NO: 389 /
SNP Position Genomic: 29215 / Related Interrogated SNP: hCV8717873 / Related
Interrogated SNP: hCV8718961 / Related Interrogated SNP: hCV1376266 / Related
Interrogated SNP: hCV1376342 / Related Interrogated SNP: hCV15973734 / SNP
Source: Applera / Population(Allele,Count): Caucasian (A,31|G,3) African
American (A,6|G,2) total (A,37|G,5) / SNP Type: UTR5;INTRON / SNP Source:
dbSNP; HGBASE / Population(Allele,Count): Caucasian (G,40|A,186) / SNP Type:
UTR5;INTRON /
Context (SEQ ID NO: 1913): /
TAACCTAACCACAAGACTAACCTACTGTCACTTCTGCCCTATCCTACTGCTAGAAGCCAGTCGCTACATCTCCCCCACACTCA
AAGGGAGGTGGTCGCAC
Y
GTGGGGTCCACTGGAAGTTGCCTACCAGACTCAGGTCATCCCAAACACACCCTCTAGCCATTTGGTGTGGCATCGGAAAGAAA
ACTAAGGCCAGGTACGG / Celera SNP ID: hCV8717751 / Public SNP ID: rs1671218 /
SNP Chromosome Position: 55554399 / SNP in Genomic Sequence: SEQ ID NO: 389 /
SNP Position Genomic: 8707 / Related Interrogated SNP: hCV8717873 / SNP
Source: Applera / Population(Allele,Count): Caucasian (C,13|T,17) African
American (C,12|T,24) total (C,25|T,41) / SNP Type: INTRON;PSEUDOGENE /
SNP Source: Applera / Population(Allele,Count): Caucasian (C,16|T,24) African
American (C,12|T,24) total (C,28|T,48) / SNP Type: INTRON;PSEUDOGENE /
SNP Source: dbSNP; HapMap; HGBASE / Population(Allele,Count): Caucasian
(C,38|T,82) / SNP Type: INTRON;PSEUDOGENE /
Context (SEQ ID NO: 1914): /
ACGCACGTGGTTGTTGGTGTGATTCTGTTTCTCCAGATTGTCTCCATTCCTCCCTGGCTTCTCCACAGGGCCGCTCACCATGG
CAGCCGGCTCCATCACC
R
CCAGCCAGCGAGAGGGCAAGACAAGAGGGCTGACGAGGGACGCTACCATCACAGAGGTCAGTTTTGTAACCTAACCACAAGAC
TAACCTACTGTCACTTC / Celera SNP ID: hCV8717752 / Public SNP ID: rs1671217 /
SNP Chromosome Position: 55554232 / SNP in Genomic Sequence: SEQ ID NO: 389 /
SNP Position Genomic: 8540 / Related Interrogated SNP: hCV8717873 / Related
Interrogated SNP: hCV1376342 / Related Interrogated SNP: hCV8718961 / Related
Interrogated SNP: hCV1376266 / Related Interrogated SNP: hCV15973734 / SNP
Source: Applera / Population(Allele,Count): Caucasian (A,1|G,17) African
American (A,2|G,26) total (A,3|G,43) / SNP Type: MISSENSE
MUTATION;ESE;INTRON;PSEUDOGENE / SNP Source: Applera /
Population(Allele,Count): Caucasian (A,3|G,37) African American (A,4|G,30)
total (A,7|G,67) / SNP Type: MISSENSE MUTATION;ESE;INTRON;PSEUDOGENE / SNP
Source: dbSNP; HapMap; HGBASE / Population(Allele,Count): Caucasian
(A,21|G,99) / SNP Type: MISSENSE MUTATION;ESE;INTRON;PSEUDOGENE /
Context (SEQ ID NO: 1915): /
TAAAAACCACTTCCTTTGCTCATTTATCAAACTCAAACGCTAGGAGGGCCACCTAACATCCTCCGTCCCACGCAATGGGGTGT
TTCTGGAGCACTCCGGT
K
TATCAGGGACCCTGTCAGTTGCCATCGCACATGTATATGGGGCCAGCCCCTGTGCCACCGAAGAGGGGGGATATTGAAAACAT
GTTACAGCCAGGAGCGG / Celera SNP ID: hCV8717761 / Public SNP ID: rs1654439 /
SNP Chromosome Position: 55553647 / SNP in Genomic Sequence: SEQ ID NO: 389 /
SNP Position Genomic: 7955 / Related Interrogated SNP: hCV8717873 / Related
Interrogated SNP: hCV1376342 / Related Interrogated SNP: hCV8718961 / Related
Interrogated SNP: hCV1376266 / Related Interrogated SNP: hCV15973734 / SNP
Source: Applera / Population(Allele,Count): Caucasian (G,33|T,3) African
American (G,25|T,7) total (G,58|T,10) / SNP Type: INTRON;PSEUDOGENE / SNP
Source: dbSNP; HapMap; HGBASE / Population(Allele,Count): Caucasian
(T,45|G,179) / SNP Type: INTRON;PSEUDOGENE /
Context (SEQ ID NO: 1916): /
GTGCTCCTGGCATGTGGTCGGTCGAAGCCAAGGACACTGCTCAGCATTCTGCAGTGCACAGGACGGCCCCGCCCAGGCGGAGA
ATGATCCGGTGACACAT

R
TAGGTGGGAAGGATGCACGAATGATGGCGTTTAGGAAGAATATTATCACTTCTTTCCCGTAAGAGCAACTTAGAGCAAGAAAA
TGGTATTATTCTTAGGG / Celera SNP ID:   hCV1376386 / Public SNP ID:   rs1671214 /
SNP Chromosome Position:   55552823 / SNP in Genomic Sequence:   SEQ ID NO: 389 /
SNP Position Genomic:   7131 / Related Interrogated SNP:   hCV8717873 / SNP
Source:   dbSNP; Celera; HapMap; HGBASE / Population(Allele,Count):   Caucasian
(A,75|G,151) / SNP Type:   UTR3;INTRON /
Context        (SEQ ID NO: 1917): /
TAGGGCCTTCTCTCTTATGGAGGCTCCAAGCCAGGGTTGCCATGGCAGAAGATGCTGGGCTTGCTTTTTCCTTGAGAGAACTG
TACTCAAGATGATGTAA

M
TGTCACCCCGGGTGCCACTTGGGTGCTTTGGAGAAGCGCTCAGACGCGACACGCCGTGACGACTCCGCGGCAGGCAGCCGGAC
CTGTCCTCTGGCGTGCG / Celera SNP ID:   hCV1376388 / Public SNP ID:   rs1671215 /
SNP Chromosome Position:   55553019 / SNP in Genomic Sequence:   SEQ ID NO: 389 /
SNP Position Genomic:   7327 / Related Interrogated SNP:   hCV8717873 / SNP
Source:   dbSNP; Celera; HapMap; HGBASE / Population(Allele,Count):   Caucasian
(C,75|A,151) / SNP Type:   TRANSCRIPTION FACTOR BINDING
SITE;UTR3;INTRON;PSEUDOGENE /
Context        (SEQ ID NO: 1918): /
TGGCATGAACCCGGGAGGCGGAGCTTGGAGTGAGCCGAGATGTGCCACTGTCCTCCAGCCTGGGCGACAAAGCAAGATACCGT
CTCAGAAAAAAAAAAAA

M
CCCCTCTAGAGAATCCCAGAAAATAGAAGGAATTATTCCATTTCCCGGAAGAGGAACGTGTGGCTAAGAGAGGAGGCATCACC
TGCCCAGGTGTGTCCAG / Celera SNP ID:   hCV1376414 / Public SNP ID:   rs61445435 /
SNP Chromosome Position:   55565038 / SNP in Genomic Sequence:   SEQ ID NO: 389 /
SNP Position Genomic:   19346 / Related Interrogated SNP:   hCV8717873 / SNP
Source:   dbSNP; Celera; HGBASE / Population(Allele,Count):   Caucasian
(C,69|A,157) / SNP Type:   INTRONIC INDEL;INTRON /
Context        (SEQ ID NO: 1919): /
GTGTATTCTTGAGTCTATTTAAAGATGAACAAGAATAGAAAAGCTAGAGGATGGTCCCAGTTTTACATAAAAATATATAAATA
CACACACAAACCTATTA

K
AAACAAGACTAGAAAGATCCATAAAAGTGATTCTCCTGGGGCTGGTGCAGATCAAAGTTGTTTAGTTCTGTCTTCTTTTTTAT
TGAGACAGAGTCTCACT / Celera SNP ID:   hCV8703249 / Public SNP ID:   rs1654444 /
SNP Chromosome Position:   55566454 / SNP in Genomic Sequence:   SEQ ID NO: 389 /
SNP Position Genomic:   20762 / Related Interrogated SNP:   hCV8717873 / SNP
Interrogated SNP:   hCV8718961 / Related Interrogated SNP:   hCV1376266 / Related
Interrogated SNP:   hCV1376342 / Related Interrogated SNP:   hCV15973734 / SNP
Source:   dbSNP; HapMap; ABI_Val; HGBASE / Population(Allele,Count):   Caucasian
(T,42|G,182) / SNP Type:   INTRON /
Context        (SEQ ID NO: 1920): /
CCCAAAGTGCTGGGATTACAGGCATGAGCCACTGCACCCGACCGGCTATAATTTTTTTTTAATGGAAAACAGCAGATATTGGTG
AGTATGCAGAGAAATTG

R
ACTGCGCGTGCATTGCTGGCAGGGACGTAACATGGCGCCCCTGCTGTGGAAAACAGTTCCAGCAGCTCCTCCAGAAGTTAAAC
GTGGGATTGCCATAAAA / Celera SNP ID:   hCV8717793 / Public SNP ID:   rs1654433 /
SNP Chromosome Position:   55550312 / SNP in Genomic Sequence:   SEQ ID NO: 389 /
SNP Position Genomic:   4620 / Related Interrogated SNP:   hCV8717873 / Related
Interrogated SNP:   hCV8718961 / Related Interrogated SNP:   hCV1376266 / Related
Interrogated SNP:   hCV1376342 / Related Interrogated SNP:   hCV15973734 / SNP
Source:   dbSNP; HapMap; HGBASE / Population(Allele,Count):   Caucasian
(A,44|G,182) / SNP Type:   INTRON /
Context        (SEQ ID NO: 1921): /
CCTGACCTCAGTTGATCCACCTGCCTCGGTCTCCCAAAGTGCTGGGATTACAGGCATGAGCCACTGCACCCGACCGGCTATAA
TTTTTTTTTAATGGAAAA

Y
AGCAGATATTGGTGAGTATGCAGAGAAATTGAACTGCGCGTGCATTGCTGGCAGGGACGTAACATGGCGCCCCTGCTGTGGAA
AACAGTTCCAGCAGCTC / Celera SNP ID:   hCV8717794 / Public SNP ID:   rs1654432 /
SNP Chromosome Position:   55550280 / SNP in Genomic Sequence:   SEQ ID NO: 389 /
SNP Position Genomic:   4588 / Related Interrogated SNP:   hCV8717873 / Related
Interrogated SNP:   hCV8718961 / Related Interrogated SNP:   hCV1376266 / Related
Interrogated SNP:   hCV1376342 / Related Interrogated SNP:   hCV15973734 / SNP
Source:   dbSNP; HapMap; HGBASE / Population(Allele,Count):   Caucasian

(C,44|T,182) / SNP Type: INTRON /
Context (SEQ ID NO: 1922): /
TCTGTCTCAAAAAAATTTAAATTTAAATTTAAAATGCCCGCTGCACGAGATTCCCAAGGCTGCTGTACGCATTACCACAGACT
TAGTGGCTTAAAACCAG
R
TAAGTGCATCCTCCTCCAGTTCGGCAGGTCAAGAGTCCAAAACATGTCTCACTGGAATAAATCAAGGTATTGGTAGAGTCAGG
TTCCTTCTGGAGGCTCT / Celera SNP ID: hCV8718960 / Public SNP ID: rs1654452 /
SNP Chromosome Position: 55572284 / SNP in Genomic Sequence: SEQ ID NO: 389 /
SNP Position Genomic: 26592 / SNP Source: dbSNP; HGBASE /
Population(Allele,Count): Caucasian (A,95|G,127) / SNP Type: INTRON /
Context (SEQ ID NO: 1923): /
ACTCTCACCCCACGTGCCCCTGACTGAATGATCTCAGGCAACCTTGTCTGAGCTCACTCACATACCCCAACTGAAACACAGAC
ATCATCACATCACACCA
M
GGGACCTCTGTCATGTTCTCCATAAGTGGCTCCACCCAGTGTCTGGCGTGTGGAACGCCTTCAGCAAGTGACAGTCATTATTT
TATAAATGCTCACTGCA / Celera SNP ID: hCV8718968 / Public SNP ID: rs1671176 /
SNP Chromosome Position: 55570782 / SNP in Genomic Sequence: SEQ ID NO: 389 /
SNP Position Genomic: 25090 / Related Interrogated SNP: hCV8717873 / SNP
Source: dbSNP; HapMap; ABI_Val; HGBASE / Population(Allele,Count): Caucasian
(A,72|C,152) / SNP Type: INTRON /
Context (SEQ ID NO: 1924): /
AGCATGGATGTACAGATATCTCTCTGAGAGCCAAAGCAGGGGAGATTTTACCTCTCCTGGCCAGTTCCAAGGCGGTCTGCTTC
CCGATGCCTGTGTTGGC
R
CCCGTCACGATGACCGTCTTCCCAGGGATGGTGGCCTTGCTGGGGCAAGCCCCACCGGTGACATAGTCCCTGAGGGTGAGAAG
CGGCACGGTCAGTCCTG / Celera SNP ID: hCV8718972 / Public SNP ID: rs1654447 /
SNP Chromosome Position: 55570574 / SNP in Genomic Sequence: SEQ ID NO: 389 /
SNP Position Genomic: 24882 / Related Interrogated SNP: hCV8717873 / Related
Interrogated SNP: hCV8718961 / Related Interrogated SNP: hCV1376266 / Related
Interrogated SNP: hCV1376342 / Related Interrogated SNP: hCV15973734 / SNP
Source: dbSNP; HapMap; ABI_Val; HGBASE / Population(Allele,Count): Caucasian
(A,42|G,184) / SNP Type: UTR5;SILENT RARE CODON;SILENT MUTATION;INTRON /
Context (SEQ ID NO: 1925): /
TTATAAATCTTTAATACATAAGCTGGCTTTAAAATTATTGGTAAAATAAGATTAGAAATGTCTTAAGAATTGTTGGCGTTTTT
GTTTGCACTTATTGAAC
R
AGTGGTTTCATGCTTATCCCTGCAGAATACTATGAGATTTGTCATAAGGGTTATAAAACTATAAACCCGGCTGGGCGTGGTGG
CTCACGCCTGTAATCCC / Celera SNP ID: hCV16044361 / Public SNP ID: rs2569513 /
SNP Chromosome Position: 55552144 / SNP in Genomic Sequence: SEQ ID NO: 389 /
SNP Position Genomic: 6452 / Related Interrogated SNP: hCV8717873 / Related
Interrogated SNP: hCV8718961 / Related Interrogated SNP: hCV1376266 / Related
Interrogated SNP: hCV1376342 / Related Interrogated SNP: hCV15973734 / SNP
Source: dbSNP; HapMap; HGBASE / Population(Allele,Count): Caucasian
(G,44|A,182) / SNP Type: UTR3;INTRON /
Context (SEQ ID NO: 1926): /
AGCTCTCTTCCTCTTGGTCAGCCCCAGTCCTCACCCTACCCCATTTCTCCTTTAATAACATCTTATTAAATGCACCTGGCACC
AGCCTCAGGTTAAGGAT
Y
TTTTTTTGGCCAGGCGAGGTGGCTCAGGCCTGTAATCCCAGCACTTTGGGAGGCCGAGGCGGGCGGATTACCTGGGGTCGGGA
GTTCCAGACCAGCCTGG / Celera SNP ID: hCV29271569 / Public SNP ID: rs1626971 /
SNP Chromosome Position: 55557024 / SNP in Genomic Sequence: SEQ ID NO: 389 /
SNP Position Genomic: 11332 / Related Interrogated SNP: hCV8717873 / Related
Interrogated SNP: hCV1376342 / Related Interrogated SNP: hCV8718961 / Related
Interrogated SNP: hCV1376266 / Related Interrogated SNP: hCV15973734 / SNP
Source: dbSNP; HapMap; HGBASE / Population(Allele,Count): Caucasian
(C,21|T,99) / SNP Type: INTRON /
Context (SEQ ID NO: 1927): /
GGCATGAACCCGGGAGGCGGAGCTTGGAGTGAGCCGAGATGTGCCACTGTCCTCCAGCCTGGGCGACAAAGCAAGATACCGTC
TCAGAAAAAAAAAAAAA / A //
CCCTCTAGAGAATCCCAGAAAATAGAAGGAATTATTCCATTTCCCGGAAGAGGAACGTGTGGCTAAGAGAGGAGGCATCACCT
GCCCAGGTGTGTCCAGC // Celera SNP ID: hDV91225183 / Public SNP ID: rs1671171 /
SNP Chromosome Position: 55565039 / SNP in Genomic Sequence: SEQ ID NO: 389 /
SNP Position Genomic: 19347 / Related Interrogated SNP: hCV8717873 / SNP

Source: CDX / Population(Allele,Count): no_pop (A,-|,-) / SNP Type: //

Gene Number: 53 / Gene Symbol: STK11IP - 114790 / Gene Name: serine/threonine kinase 11 interacting protein / Chromosome: 2 / OMIM NUMBER: 607172 / OMIM Information: // Genomic Sequence (SEQ ID NO: 390): // / SNP Information /
Context (SEQ ID NO: 1928): /
CCTAACTGTGGTAGTGACCACGTGGTTCTCCTCGCTGTGTCTCGGGGAACCCCCAACAGGGAGCGGAAACAGGGAGAGCAGTC
TCTGGCTCCTTCTCCGT
Y
TGCCAGCCCTGTCTGCCACCCTCCTGGCCATGGTGACCACCTTGACAGGGCCAAGAACAGCCCACCTCAGGCACCGAGCACCC
GTGACCATGGTAGTTGG / Celera SNP ID: hCV2915511 / Public SNP ID: rs627530 /
SNP Chromosome Position: 220476443 / SNP in Genomic Sequence: SEQ ID NO: 390 /
SNP Position Genomic: 23847 / SNP Source: Applera /
Population(Allele,Count): Caucasian (C,1|T,39) African American (C,8|T,24)
total (C,9|T,63) / SNP Type: MISSENSE MUTATION;UTR3;INTRON / SNP Source:
dbSNP; HapMap; ABI_Val; HGBASE / Population(Allele,Count): Caucasian (C,5|T,219)
/ SNP Type: MISSENSE MUTATION;UTR3;INTRON /
Context (SEQ ID NO: 1929): /
TTCCGGTCGTCCCTTGGCTCGATTTTCTTCCGGGGCGGGACTTCCTTTCCATCATTGATAGGCGCCGGGCAGCTGAGCTGGTA
GGAGGACCAGACGGGGA
K
GTTCGGTATGTCTGACCAGGACTTATGTTCCTCGAAAGGGCGGCCAGGAGGATGCTCTTCCTCTCTTTTCTTTCTCGCCTTTT
TCTCCGCATGACGCCTC / Celera SNP ID: hCV2414147 / Public SNP ID: rs681747 /
SNP Chromosome Position: 220462640 / SNP in Genomic Sequence: SEQ ID NO: 390 /
SNP Position Genomic: 10044 / Related Interrogated SNP: hCV2915511 / SNP
Source: Applera / Population(Allele,Count): Caucasian (G,0|T,10) African
American (G,14|T,20) total (G,14|T,30) / SNP Type: MISSENSE
MUTATION;UTR5;INTRON / SNP Source: Applera / Population(Allele,Count):
Caucasian (G,1|T,39) African American (G,15|T,21) total (G,16|T,60) / SNP Type:
MISSENSE MUTATION;UTR5;INTRON / SNP Source: dbSNP; HapMap; ABI_Val; HGBASE /
Population(Allele,Count): Caucasian (G,6|T,220) / SNP Type: MISSENSE
MUTATION;UTR5;INTRON /
Context (SEQ ID NO: 1930): /
CTCCCCTGTTATTCTTCAGCTTCAGTTTCTCTTCGATGTGCTGCAGAAAACACTTTCACTCAAGGTTCTGGGTGTGGGGAAGA
GGCAGGATGTGCAAGGA
K
CCCAAGAGAATGATGGAAGTTGGGGATAGGGTAGGGGTGCAGAAGTCTGCAGTGCCAGGATCCAGAGGGTGTCAGAGGTACCA
GTGGTTGTGCTAAGGAA / Celera SNP ID: hCV2414148 / Public SNP ID: rs620698 /
SNP Chromosome Position: 220466199 / SNP in Genomic Sequence: SEQ ID NO: 390 /
SNP Position Genomic: 13603 / Related Interrogated SNP: hCV2915511 / SNP
Source: Applera / Population(Allele,Count): Caucasian (G,1|T,35) African
American (G,15|T,19) total (G,16|T,54) / SNP Type: MISSENSE MUTATION;INTRON /
SNP Source: dbSNP; ABI_Val; HGBASE / Population(Allele,Count): Caucasian
(G,6|T,220) / SNP Type: MISSENSE MUTATION;INTRON /
Context (SEQ ID NO: 1931): /
GAACATGGCTGTTGTGTGCCCTGCACTGGGCTGGGCATTTCTTTCAGTCTCTCCTTGGCACTAGGAGCCGAGGAGGGGAGCCT
CAGAGGTTGCAAGCACT
Y
GGGAAGCTGGGCTGAGGAGCAGGGATTCTGTGCTGGAGCTTGGACGTCAGTACAGGCCTTTGACAGGCTTCAGAGGTCCTGCC
ATCCTCCATGCTCTCAG / Celera SNP ID: hCV2414150 / Public SNP ID: rs675291 /
SNP Chromosome Position: 220473653 / SNP in Genomic Sequence: SEQ ID NO: 390 /
SNP Position Genomic: 21057 / Related Interrogated SNP: hCV2915511 / SNP
Source: Applera / Population(Allele,Count): Caucasian (C,1|T,35) African
American (C,19|T,19) total (C,20|T,54) / SNP Type: UTR5;INTRON / SNP Source:
dbSNP; HapMap; ABI_Val; HGBASE / Population(Allele,Count): Caucasian
(C,2|T,118) / SNP Type: UTR5;INTRON /
Context (SEQ ID NO: 1932): /
AGTGATCGTCCTGTGTCCACTCTTCTTCCCCTTTCCTGCCCAGTCCTCCCCCACCTTGCACCCTCTTGCCATCCCTGGGTGAG
GGGCCAAGGACCTGGAC
S
CTGCTTACTGTGTTCTTAATACCTGGAGCTCAGCAGGATGGGCCTACCACATACTCCCTCCCCCTTTTTGTCTTTGCTCAGTT
CCTTCTCGATGGCAAGG / Celera SNP ID: hCV7540 / Public SNP ID: rs633200 / SNP

Chromosome Position: 220471116 / SNP in Genomic Sequence: SEQ ID NO: 390 /
SNP Position Genomic: 18520 / Related Interrogated SNP: hCV2915511 / SNP
Source: dbSNP; HapMap; ABI_Val; HGBASE / Population(Allele,Count): Caucasian
(C,2|G,118) / SNP Type: MISSENSE MUTATION;INTRON /
Context (SEQ ID NO: 1933): /
TGAGGAACCAATGAGAATATGAATATTAAAGTGGCTTGTAAGCTGGAAAGCACTACCTGCATTTAAAATAATGAAGTTATCAT
TGTTGTTATCCTTAAGA
R
CACTGGATGGAGATGATGGCGAATGACACAGTAATCATAAGACAGGCAGGGGCTACCCAGGCCTGTTAACAGGAGGGCCATTC
CCTCACATCCTCTCTGC / Celera SNP ID: hCV8840562 / Public SNP ID: rs668034 /
SNP Chromosome Position: 220488716 / SNP in Genomic Sequence: SEQ ID NO: 390 /
SNP Position Genomic: 36120 / Related Interrogated SNP: hCV2915511 / SNP
Source: dbSNP; Celera; HapMap; ABI_Val; HGBASE / Population(Allele,Count):
Caucasian (G,2|A,118) / SNP Type: INTERGENIC;UNKNOWN /
Context (SEQ ID NO: 1934): /
GTTTTGGACTCAGTGACTTTTCCATTCTCCACAAAAACATTCTTCAGTTTATTAGTTACTATAATTACTTGTGCACTTGCTTT
CAATTCCACAGACATTT
R
TTGAGCTCTTAGAACGTGCCAGGATGTATGTGAGAAGATGGGGGCAGGGATCCAAGGATGAATCAGATGTGCTCCTGCCTCCA
AGGAGCTTATGGTCCAG / Celera SNP ID: hCV11470250 / Public SNP ID: rs678276 /
SNP Chromosome Position: 220485452 / SNP in Genomic Sequence: SEQ ID NO: 390 /
SNP Position Genomic: 32856 / Related Interrogated SNP: hCV2915511 / SNP
Source: dbSNP; HapMap; ABI_Val; HGBASE / Population(Allele,Count): Caucasian
(A,1|G,119) / SNP Type: INTERGENIC;UNKNOWN //

Gene Number: 54 / Gene Symbol: TTC8 - 123016 / Gene Name: tetratricopeptide
repeat domain 8 / Chromosome: 14 / OMIM NUMBER: 608132 / OMIM Information:
Bardet-Biedl syndrome 8, 209900 (3) // Genomic Sequence (SEQ ID NO: 391): // /
SNP Information /
Context (SEQ ID NO: 1935): /
AGGGAAATCTTCATGAAGGATGTGGAGCCTGAACAGGGCCTTGAAGGTGGACTTGAGAAATATAACATTCATTGATAACATTA
GATGCATACATTTATGA
Y
TATCAGTATTTTAGAAAGAATATATAATAACTGTAGAAGGTTTATTTAGTTTTTCATTTTGTATGTATTTCTAAGCTTGGTCT
TGGTTGCTGTCTTTTCT / Celera SNP ID: hCV2796701 / Public SNP ID: rs9323834 /
SNP Chromosome Position: 89335613 / SNP in Genomic Sequence: SEQ ID NO: 391 /
SNP Position Genomic: 54635 / Related Interrogated SNP: hCV16182835 / SNP
Source: dbSNP; Celera; HapMap / Population(Allele,Count): Caucasian
(T,147|C,77) / SNP Type: INTRON /
Context (SEQ ID NO: 1936): /
TTTCTGTGTCCATTAATTAAGTATACAAGTATTCAAAAGTAATTTTATAGAACCCAAAGTCTTGAACCAACTGGCTTATTGAG
GCTTGAGCCTTCTTTGC
R
TTGGGCGTTGCCACCTTTGGAAGCTGTAACACTGAAGCCTAGCACTGTGGGATGCTGTTGGCTTTGAAAAAGAATTTCAAATA
TAAAGCACATGAAGAAA / Celera SNP ID: hCV2796704 / Public SNP ID: rs10134036 /
SNP Chromosome Position: 89334166 / SNP in Genomic Sequence: SEQ ID NO: 391 /
SNP Position Genomic: 53188 / Related Interrogated SNP: hCV16182835 / SNP
Source: dbSNP; Celera; HapMap; ABI_Val / Population(Allele,Count): Caucasian
(A,147|G,79) / SNP Type: INTRON /
Context (SEQ ID NO: 1937): /
CTTTAAATTGTAAGATCTTGTTCATAGGTCCTCTTGGATATTCTAAGTAAATAAAACAATCATCATTTGCAAATACAGATGGT
TTCATCTCTTCCTTTCA
R
TTATTTTACCACTTTTAAAAAATATGTCTTACTGCTTAGGGCTTCCAATATAATATTGAACAGACATATCTCTGTTTTCTTGAA
ATTCCTTGAAAATGCTA / Celera SNP ID: hCV2796706 / Public SNP ID: rs9671813 /
SNP Chromosome Position: 89330291 / SNP in Genomic Sequence: SEQ ID NO: 391 /
SNP Position Genomic: 49313 / Related Interrogated SNP: hCV16182835 / SNP
Source: dbSNP; Celera / Population(Allele,Count): Caucasian (A,148|G,78) /
SNP Type: INTRON /
Context (SEQ ID NO: 1938): /
TAAGGGTGACACAGAATGTGTAATGCAAATTTCATAGTAATAGTAACTTTATAAAATAATATTATAAAATACAGGATTTAAAC
CTTTCTAAATAGATCCT
R

AAACTGTCTCTCACATTATATAGTAGATGTTTGTTTATAATGTTTACAAAACATTTTGGTGAATTTCCTCAATGTTTTATAAA
TGTACATTTTTTAAGTC / Celera SNP ID: hCV7583094 / Public SNP ID: rs1048190 /
SNP Chromosome Position: 89343992 / SNP in Genomic Sequence: SEQ ID NO: 391 /
SNP Position Genomic: 63014 / Related Interrogated SNP: hCV16182835 / SNP
Source: dbSNP; HapMap; HGBASE / Population(Allele,Count): Caucasian
(G,84|A,32) / SNP Type: MICRORNA;UTR3;PSEUDOGENE /
Context (SEQ ID NO: 1939): /
TAATAAGGGAATATTAACACAGCAAAAAATAAATGATTCCATTTTCCTAAGGTGATATGGAGGGATTTTGTCTTGAATTTATG
TGAATTCAGTGTGAGCA
Y

GAAGGCAAAGAATGAGTCACAGTGGCTCAGGGCAGAATGATGACATAGCACCGTGGATGACTGTATCAGAAGCGACATTCAGG
ATGTCTTTGTAACAACA / Celera SNP ID: hCV27202682 / Public SNP ID: rs10142228 /
SNP Chromosome Position: 89345510 / SNP in Genomic Sequence: SEQ ID NO: 391 /
SNP Position Genomic: 64532 / Related Interrogated SNP: hCV16182835 / SNP
Source: dbSNP; Celera; HapMap / Population(Allele,Count): Caucasian
(T,144|C,82) / SNP Type: INTERGENIC;UNKNOWN /
Context (SEQ ID NO: 1940): /
CCAAGTGGCAGTTGCCATGTAGTTGTCATAGTCTGTTTATATGGAAACTTGGGTAATTACTCTTGGGAGCATGGTGAACCTGG
TAATTATTACTGGTTGT
W

GCCTGAAAGCTTTTGTTGACACCTGCTGGTAAGATCAAGAAAATCAAGAAAACACAGGTATCAGCATAGCAGAATGACTGTCC
ATAGGTTGGAAGAAAAT / Celera SNP ID: hCV29549024 / Public SNP ID: rs10137225 /
SNP Chromosome Position: 89335098 / SNP in Genomic Sequence: SEQ ID NO: 391 /
SNP Position Genomic: 54120 / Related Interrogated SNP: hCV16182835 / SNP
Source: dbSNP; HapMap; ABI_Val / Population(Allele,Count): Caucasian
(A,141|T,77) / SNP Type: TRANSCRIPTION FACTOR BINDING SITE;INTRON /
Context (SEQ ID NO: 1941): /
GCCATGATTGTGAGGCCTCCCCAGCCACATGGAAGTGTGAGTGAATTAAGCCTCTTAACTTTATAAATTGTTCAGTCTCAGGT
ATGTCTTTGTTAGTAGT
R

TGAGAACAGGCTAATACAAAAAATTAAAAAATTGGCCTGGCACAGTGGTGCATGCCTGTGGTCCCAGCTACTCAGGAGTCTGA
GGTAGAAGGATCACTTG / Celera SNP ID: hCV29910416 / Public SNP ID: rs10139817 /
SNP Chromosome Position: 89348612 / SNP in Genomic Sequence: SEQ ID NO: 391 /
SNP Position Genomic: 67634 / Related Interrogated SNP: hCV16182835 / SNP
Source: dbSNP; HapMap / Population(Allele,Count): Caucasian (A,145|G,81) /
SNP Type: INTERGENIC;UNKNOWN /
Context (SEQ ID NO: 1942): /
TAATCTCCCCCTTCTCTGACTCTCTTATCTTCCATGTCTTCTGCTTTTCTTTTGTTCACCTAAGTCATCTATAATTTGACCAC
AATGTGAGTATAGTATT
W

GACACATTGTATTGTAATTTAGGTGCCTCCCCCTCTAGAATGTTGTGGAATTAATGTGTTCTTGGCCTCATACCCACTTAGGC
CTGGTCTCCATTTTCCT / Celera SNP ID: hCV29567112 / Public SNP ID: rs10484010 /
SNP Chromosome Position: 89298288 / SNP in Genomic Sequence: SEQ ID NO: 391 /
SNP Position Genomic: 17310 / Related Interrogated SNP: hCV16182835 / SNP
Source: dbSNP; HapMap / Population(Allele,Count): Caucasian (T,171|A,51) /
SNP Type: INTRON /
Context (SEQ ID NO: 1943): /
GTTAAGAAATCAGAGGTGTTTTCATATTTAGATACCTTGGGTGGCCTCCAAATTTCTCTTTAAATAAAGTATTTAACACAAAT
ACAGAGAATATTATAGA
S

AAATATAAGAAATACCTTGAAATGGTGTTTAAATATTAAGAAAGTATAAAATTGATTGTCCATGGAAAACGATTTTTCTGTCT
TTGAGCTTTTAGTGCTT / Celera SNP ID: hDV70991980 / Public SNP ID: rs17700521 /
SNP Chromosome Position: 89315875 / SNP in Genomic Sequence: SEQ ID NO: 391 /
SNP Position Genomic: 34897 / Related Interrogated SNP: hCV16182835 / SNP
Source: dbSNP; HapMap / Population(Allele,Count): Caucasian (C,88|G,32) / SNP
Type: INTRON //

Gene Number: 55 / Gene Symbol: EML5 - 161436 / Gene Name: echinoderm
microtubule associated protein like 5 / Chromosome: 14 / OMIM NUMBER: / OMIM
Information: // Genomic Sequence (SEQ ID NO: 392): // / SNP Information /
Context (SEQ ID NO: 1944): /
AAAATGGAAGTCATCAGACAGTACTTCCTTATTTTTTCCATCCCCAAATCTACCAGTCTTCTTATATTTGTACAAGAAAGAATG
TCCAATAAATTTCACTC

S
TTATATCTGTGGATAATTTCTTCCTGTCAAACTGATTTCTGTCAAAGTATTCCCCATCCCATTTGGCTCACTGTTCAAGCAAA
AAGTTCAACTGGCCTTG / Celera SNP ID: hCV1262727 / Public SNP ID: rs12587200 /
SNP Chromosome Position: 89098404 / SNP in Genomic Sequence: SEQ ID NO: 392 /
SNP Position Genomic: 27230 / Related Interrogated SNP: hCV16182835 / SNP
Source: dbSNP; Celera; HapMap / Population(Allele,Count): Caucasian
(C,92|G,26) / SNP Type: INTRON /
Context (SEQ ID NO: 1945): /
GAAAAAAGAAATATACATGCCAGGCACTATTCAATGTACCAGGCATAGACTTATGAACAGAACAGGCAAGACAACAAAATTCC
CTAGAGCTTCATGGTGA

R
AGACAGTCAATAAATGTAACAAGTAAAATATACTGTGCTAGATGATGATGGGTGCTTTGGAGAAAATAGAAGCATAAAAAGGA
GACAGGAAGTGGGAGGA / Celera SNP ID: hCV1262753 / Public SNP ID: rs12436982 /
SNP Chromosome Position: 89156206 / SNP in Genomic Sequence: SEQ ID NO: 392 /
SNP Position Genomic: 85032 / Related Interrogated SNP: hCV16182835 / SNP
Source: dbSNP; Celera; HapMap / Population(Allele,Count): Caucasian
(G,94|A,26) / SNP Type: INTRON /
Context (SEQ ID NO: 1946): /
CTATGAACTGGATGTCTGTCTTCCCCCAAAATTCATATACTGAAACCCTAATCACCAGTGTGATGGTATTAGGAGGTGTGAAG
GTTATGAGGATGGAGCC

Y
TCACAATGAGATTAGTGTTCTTATAAGAGACAGGAGAAAGATAATCTTTTTTTCACCATGTGAGGATATAATGAGAAGACAGCC
ATCTGCAAATACAGAGA / Celera SNP ID: hCV3211521 / Public SNP ID: rs12431548 /
SNP Chromosome Position: 89102170 / SNP in Genomic Sequence: SEQ ID NO: 392 /
SNP Position Genomic: 30996 / Related Interrogated SNP: hCV16182835 / SNP
Source: dbSNP; Celera; HapMap / Population(Allele,Count): Caucasian
(C,93|T,25) / SNP Type: INTRON /
Context (SEQ ID NO: 1947): /
CCGTAAAAGGGATAAACAATACAGTACAAGGCTGAAGATTCCAAGTGCCACAAGAGAGTTGGGATCAAAATGCTATGGGAATT
CAGAGCAGAAAAGATTA

R
TCTCATCATGGGAATATGGTACTCCTGGACTGACCCAGATAGATTTTACAAATGTAAAAGTCAGTGGGGAAGGGCCTTCTAGA
CAGAGGAACACTTTAAG / Celera SNP ID: hCV9595897 / Public SNP ID: rs1287825 /
SNP Chromosome Position: 89105536 / SNP in Genomic Sequence: SEQ ID NO: 392 /
SNP Position Genomic: 34362 / Related Interrogated SNP: hCV16182835 / SNP
Source: dbSNP; HapMap; ABI_Val; HGBASE / Population(Allele,Count): Caucasian
(A,115|G,111) / SNP Type: INTRON /
Context (SEQ ID NO: 1948): /
AAATATGTATACAGCATTTACACTGTATTAGGTATCATAAGTAATCTACAGATGGTTTAAAGTATGCAGGAGAATGTGCATAG
GTTATATGCAAATTACT

R
TGCCATTTTATATAAGGCACTTGAGCACCTGTGGATTTTGGCATCCCCAGGGGGAATCTATCCCCCATGGACATAGAGGGACT
GTATTTTTTGTGTCCAA / Celera SNP ID: hCV11295918 / Public SNP ID: rs12586348 /
SNP Chromosome Position: 89109867 / SNP in Genomic Sequence: SEQ ID NO: 392 /
SNP Position Genomic: 38693 / Related Interrogated SNP: hCV16182835 / SNP
Source: dbSNP; Celera; HapMap / Population(Allele,Count): Caucasian
(A,92|G,26) / SNP Type: INTRON /
Context (SEQ ID NO: 1949): /
ATTGACTTCCCCAAGCACTTTTGTCAAAAATCAATTGACCATATCTATATTACTTTTCCACAAATTGCACCCTGTATTTTACA
CCATTAACCTATGTATC

Y
ATCCTTTCACCAATACCACACTGCCTTCATGAGTATAGCTTTATTATAAGCCTTAAAATTGAATACTGTGAGTCCTCTACCTT
TGTTCTTTTTCCAAATT / Celera SNP ID: hCV11454301 / Public SNP ID: rs11159868 /
SNP Chromosome Position: 89226872 / SNP in Genomic Sequence: SEQ ID NO: 392 /
SNP Position Genomic: 155698 / Related Interrogated SNP: hCV16182835 / SNP
Source: dbSNP; Celera; HapMap; ABI_Val / Population(Allele,Count): Caucasian
(T,170|C,56) / SNP Type: INTRON /
Context (SEQ ID NO: 1950): /
TCCTCAAGGCCAACTGCATGCCTCATGATGTTACCACCTCCATCTTCCAATCAGCAACGGCACATTGAGTTCTTCTCAAGCTT
TGAATCTTTCTGACTTC

Y
GCTGCATGTTTTCTCATTCCAGCTAAAGAAAATTATTTGCTTTTAAGGGCTCACATAATTAGACTGGGCCCATCTAGATAATT
CAAGATACTCTCAATAT / Celera SNP ID: hCV11454302 / Public SNP ID: rs7157149 /

SNP Chromosome Position: 89231845 / SNP in Genomic Sequence: SEQ ID NO: 392 / SNP Position Genomic: 160671 / Related Interrogated SNP: hCV16182835 / SNP Source: dbSNP; Celera / Population(Allele,Count): Caucasian (T,170|C,56) / SNP Type: INTRON /
Context (SEQ ID NO: 1951): /
AAATGACGATAGCACCATAAACAGAAGAAAGGAAACAGCAGAATATACTTTTTCATATTCTTTCCAAATATACATGAAATATC
ATGAAGATCACAAAAAT
Y
TGGATTACAAAGTGTCAATAAATTTTAAAAAATTAAAATACAGAGTATGTTCTCTGACAAAAACAGAATTAGAGTAACAGAAT
GTTATCCAGAAAATCTC / Celera SNP ID: hCV11657898 / Public SNP ID: rs1956406 /
SNP Chromosome Position: 89216055 / SNP in Genomic Sequence: SEQ ID NO: 392 /
SNP Position Genomic: 144881 / Related Interrogated SNP: hCV16182835 / SNP Source: dbSNP; Celera; HapMap; ABI_Val; HGBASE / Population(Allele,Count): Caucasian (T,173|C,45) / SNP Type: INTRON /
Context (SEQ ID NO: 1952): /
AGCTATGTGTCATTTTTTTAATAACCAAGTAAAGCAGAAACTGCTGGAACAAATCTGTTTTTAAGAAATGTTATGAATACATA
ATAGTTATACATTGGAA
Y
GAATCTTGAAAGAGGCTAGTTTTGTGTACCAATAATGACATGACTCATTTTCTCATCTTCTTCCTGCTCTTCCTTTTCCCCTG
GGCATACACAGTCTTTT / Celera SNP ID: hCV11657912 / Public SNP ID: rs1950806 /
SNP Chromosome Position: 89240756 / SNP in Genomic Sequence: SEQ ID NO: 392 /
SNP Position Genomic: 169582 / Related Interrogated SNP: hCV16182835 / SNP Source: dbSNP; Celera; HapMap; ABI_Val / Population(Allele,Count): Caucasian (C,170|T,56) / SNP Type: INTRON /
Context (SEQ ID NO: 1953): /
GTGACCCACCCGCCTGGGCCTCCCAAAGTGCTGGGATTACAGGCGTGAGACATCGCACCTGGCCTATAATGTGATTCTCTTTT
TTCTCTCTTTTCTTCAA
Y
ATTTATTAATTAGAATTCTACTACAAGGAAGTTATCTTTTCTATTTATTTATTTAACCCATCTATTTATATCAGCTTGAACTC
ATGAATATTTTATTTCA / Celera SNP ID: hCV15870067 / Public SNP ID: rs2224333 /
SNP Chromosome Position: 89235092 / SNP in Genomic Sequence: SEQ ID NO: 392 /
SNP Position Genomic: 163918 / Related Interrogated SNP: hCV16182835 / SNP Source: dbSNP; Celera; HapMap; HGBASE / Population(Allele,Count): Caucasian (C,93|T,27) / SNP Type: INTRON /
Context (SEQ ID NO: 1954): /
CATTTTTGACTAAGATACAGTGGTACTTTACCATACACTATACTCATGGTATGGTATTCAATAAAAAGAGAATCTGTTTTAGA
TCCTCCAGCCAAAGCAG
Y
TTTAATTAAGAACCAAAGGATTTATAGTAGTAGTTGCCAGGGACTGGGAGAGGTAGAGGTTGGTGGGGGCCGGGGTGGATATA
AGGAGATATAAGTTTCA / Celera SNP ID: hCV29385806 / Public SNP ID: rs8020072 /
SNP Chromosome Position: 89180676 / SNP in Genomic Sequence: SEQ ID NO: 392 /
SNP Position Genomic: 109502 / Related Interrogated SNP: hCV16182835 / SNP Source: dbSNP; HapMap / Population(Allele,Count): Caucasian (T,90|C,24) / SNP Type: INTRON /
Context (SEQ ID NO: 1955): /
ACCAGACGCCAACGCCCAAGGCAGAAGCAGGTCACAGCACACCCGGCCCAGCCACAGGCTGAGCATGGATCCTGCAGCAAGTG
TGGGATCCAGCCAGAGT
R
TAAGCCAAGCGCAGCCTGCCAGGCCAAGTGGGCAGGGTACCTCCTGGCGAGCCTGGAGCCCAGTGCAGCCCGGGACAGGGGCA
TTGCGCCCGCCATGGAG / Celera SNP ID: hCV30468559 / Public SNP ID: rs10132509 /
SNP Chromosome Position: 89203781 / SNP in Genomic Sequence: SEQ ID NO: 392 /
SNP Position Genomic: 132607 / Related Interrogated SNP: hCV16182835 / SNP Source: dbSNP / Population(Allele,Count): Caucasian (A,114|G,108) / SNP Type: INTRON /
Context (SEQ ID NO: 1956): /
CGTGTCATAATGTAAATAACAGCACAAGAGTAGATTGTTTATAAACAAATGTGAAAATGTTAATTTAACATACTCAAATATTT
CTTGATAGGTACATTAA
Y
AACAAAGCCTGGATTACTGTTCTAAAGTATCTAACAGAGTATCTTCACATGGCAGGTATTGAATACATCCTTCTTGAAAGTGA
ATTTTCGTAATATTTCT / Celera SNP ID: hCV32095460 / Public SNP ID: rs12434935 /
SNP Chromosome Position: 89111793 / SNP in Genomic Sequence: SEQ ID NO: 392 /
SNP Position Genomic: 40619 / Related Interrogated SNP: hCV16182835 / SNP Source: dbSNP; HapMap / Population(Allele,Count): Caucasian (C,93|T,27) / SNP

Type:   INTRON /
Context          (SEQ ID NO: 1957): /
TCCTGAGGCTTCTCCAGCCCTGCAGAACTGTGAGTCAATTAAACCTCTTTCCTTTACAAATTACCCAGTCTTGATTATGTTTT
TATTAGCAGTGTCAGAA
S
GAACTAATTCAGTAAATTGGTAAAGATACCCAAAAATGTGGAAGTGACTTTGTAACTGGGTAACAGGCAGAGGCTAGAACAGT
TTGGAGGGCTCAGAAGA / Celera SNP ID:   hCV32095533 / Public SNP ID:   rs12588535 /
SNP Chromosome Position:   89248244 /  SNP in Genomic Sequence:   SEQ ID NO: 392 /
SNP Position Genomic:   177070 / Related Interrogated SNP:   hCV16182835 / SNP
Source:   dbSNP; HapMap / Population(Allele,Count):   Caucasian (C,93|G,27) / SNP
Type:   INTRON /
Context          (SEQ ID NO: 1958): /
ACCTTCCTCTGGGTGCTGTTCTCATGATAGTGAGTTCTCATGAGATCTCCCCACCTCGCTTCCTTCTACTCTAGCCATGTGAA
GTGCCTTGCCTCCCCTT
K
GCCTTCTGCCATGACTGGAAGCTTCCTGAGCCTCCCCAGAAGCAGAAACTGCTATGCTTCCTATACAACCTACAGAACTGTGA
GCCAGCTAAACCTCTTT / Celera SNP ID:   hCV32095525 / Public SNP ID:   rs12590826 /
SNP Chromosome Position:   89235701 /  SNP in Genomic Sequence:   SEQ ID NO: 392 /
SNP Position Genomic:   164527 / Related Interrogated SNP:   hCV16182835 / SNP
Source:   dbSNP / Population(Allele,Count):   Caucasian (T,168|G,50) / SNP Type:
   INTRON /
Context          (SEQ ID NO: 1959): /
GATAATCTTTGGGAAGTTTAGAGTTTGCCTAGTAATAAGACATTTGTTTTGTTATTACCTTTGTATCCCTGGTCTGTTTCCCT
GAGATCAATCACAGTAA
Y
TGGTTTAAAAGTTAAATCCCAAAGACGAATACAACCATCTCTGCCACCAGTAGCAAAGCCTTCTTCACAAGCATTCATGCTAA
AAATTCCTGCCTAGAAG / Celera SNP ID:   hDV70918505 / Public SNP ID:   rs17188228 /
SNP Chromosome Position:   89205265 /  SNP in Genomic Sequence:   SEQ ID NO: 392 /
SNP Position Genomic:   134091 / Related Interrogated SNP:   hCV16182835 / SNP
Source:   dbSNP; HapMap / Population(Allele,Count):   Caucasian (T,89|C,27) / SNP
Type:   MISSENSE MUTATION;ESE SYNONYMOUS /
Context          (SEQ ID NO: 1960): /
AACATAGGATCAATTTAGTGTATGTGTGTGTATATTTATACACAGTTTTATTTAGAATAAAGCTAAAGATACCATTCAATAAT
ATATGATAATAGGATAC
Y
GAATAGTGAGTAAATCTATCAAAACTTTTCTGTTTATCTTTGGGAAACACAATAAAATACCTCTGTAACAACTTTAGCTCCAC
AATTACCATATAATCAA / Celera SNP ID:   hDV70929207 / Public SNP ID:   rs17260380 /
SNP Chromosome Position:   89148486 /  SNP in Genomic Sequence:   SEQ ID NO: 392 /
SNP Position Genomic:   77312 / Related Interrogated SNP:   hCV16182835 / SNP
Source:   dbSNP / Population(Allele,Count):   Caucasian (C,170|T,56) /
SNP Type:   TRANSCRIPTION FACTOR BINDING SITE;INTRON /
Context          (SEQ ID NO: 1961): /
AATGTAATAGTAATACGTAATTTTAAATCCACACAAGCAACATTATTTTTCACCTTAACCATAATACATATGTAACAAATTAG
TGGTAAACCAAACTAAA
S
AGACCCTAGGCTCAAACCATTCAATTTTTAAATTGAGATTTGAATAATGGGTGTCTGCATATGTCAAACCCATCAAACTTCAC
ACCTTAAATATGTGCAT / Celera SNP ID:   hDV70929214 / Public SNP ID:   rs17260415 /
SNP Chromosome Position:   89211973 /  SNP in Genomic Sequence:   SEQ ID NO: 392 /
SNP Position Genomic:   140799 / Related Interrogated SNP:   hCV16182835 / SNP
Source:   dbSNP / Population(Allele,Count):   Caucasian (C,167|G,55) / SNP Type:
   INTRON /
Context          (SEQ ID NO: 1962): /
CACCTGCCTCAGCCTCCCAAAGTGATAAGATTACAGGTGTGAGCCACTGTGCCTGGCCTACATGTCATGTTTCAACATGCATA
TGACTATGTTGGTGACA
W
ATCAAATCATAAGTATCTGGTTACTGTTGGGAGATTTGAAAATCACTCAGAAGAGACCTCTTCTCAAATTTTGAGGTCTTGTA
TAAAACAGTTTAAATTT / Celera SNP ID:   hDV71605687 / Public SNP ID:   rs17188046 /
SNP Chromosome Position:   89090761 /  SNP in Genomic Sequence:   SEQ ID NO: 392 /
SNP Position Genomic:   19587 / Related Interrogated SNP:   hCV16182835 / SNP
Source:   dbSNP; HapMap / Population(Allele,Count):   Caucasian (A,167|T,55) /
SNP Type:   UTR5;INTRON;PSEUDOGENE /
Context          (SEQ ID NO: 1963): /
ATAGGGTAATACGCATAACCATCACCTCAGACATTTATCATTTCTTTGTGATGGAAACTTTCAAAATCCTCTCTTGTAAATAC

CTGAAAATACATAAATA
Y
GTGATTCTTAACTATAGTCATCCTACAGTACTACAGAATACTAAAACATACTATTCCTATCTGGCTGTGTAAACTTGTATCCT
TTAACCAGTCCTTCCCT / Celera SNP ID: hDV77027209 / Public SNP ID: rs4635267 /
SNP Chromosome Position: 89088266 / SNP in Genomic Sequence: SEQ ID NO: 392 /
SNP Position Genomic: 17092 / Related Interrogated SNP: hCV16182835 / SNP
Source: CDX; dbSNP / Population(Allele,Count): Caucasian (C,169|T,57) / SNP
Type: INTRON /
Context (SEQ ID NO: 1964): /
GATCCTGCCACTGCACTCCAGCCTGGGTGACAGGGCGAGACTCCGTCTCAAAACAACAACAACAACAACAACAACAACAACAA
AACAAATTAAAGGAGCA
Y
AGTAATAGGATCAAAACCAATTAAAAGGTTACTGCAGATGATGGTTTCTCAGACTTCAGAAAAAAGTGAGCTTCTTTAAAATA
CAATGTAGTATTATACT / Celera SNP ID: hDV77248933 / Public SNP ID: rs8021690 /
SNP Chromosome Position: 89184150 / SNP in Genomic Sequence: SEQ ID NO: 392 /
SNP Position Genomic: 112976 / Related Interrogated SNP: hCV16182835 / SNP
Source: CDX; dbSNP / Population(Allele,Count): Caucasian (T,170|C,56) / SNP
Type: INTRON //

Gene Number: 56 / Gene Symbol: TTC24 - 164118 / Gene Name:
tetratricopeptide repeat domain 24 / Chromosome: 1 / OMIM NUMBER: / OMIM
Information: // Genomic Sequence (SEQ ID NO: 393): // / SNP Information /
Context (SEQ ID NO: 1965): /
CTGACTCCAGCTCTTTCCTTCTTGGGATGCTGCTGCCTCTTCCTCTTACCTCTGCTGCTTGAGCCAGTGTGTGCTCTGCTCTC
TGCTTCCCTAGCCCCGC
Y
ATCCTTCTCATCCCTTACATCCAAGATGTCCTCCTCCTCTTGATGCTGCTGTCTTTTCTTCTTGCTTTTCCTGCTTCTTCCAT
CACTGCATGCTCTGCTC / Celera SNP ID: hCV9102827 / Public SNP ID: rs3795733 /
SNP Chromosome Position: 156564922 / SNP in Genomic Sequence: SEQ ID NO: 393 /
SNP Position Genomic: 25403 / SNP Source: Applera /
Population(Allele,Count): Caucasian (C,10|T,30) African American (C,26|T,10)
total (C,36|T,40) // / SNP Type: MICRORNA;UTR3 / SNP Source: dbSNP;
Celera; HapMap; HGBASE / Population(Allele,Count): Caucasian (C,61|T,165) / SNP
Type: MICRORNA;UTR3 /
Context (SEQ ID NO: 1966): /
GGGGCTGCGGCAGGATGTATGCTGAAGAGTGGGCGGCATCGGGTGGGGGAAGTTGTGCAGGTGCTGGAGAAAAGCCGGAGGCT
TGCCGAGAGGAGCACTG
R
GAGGCGACTGCTGGGTGAGACCTTCGGGCAGGGAAGGCATGGGATCTGGGGAGACACAGAGCCTGATGATACTCAGAGGGCTG
GGTTTGGGGAACCCTGG / Celera SNP ID: hCV25989540 / Public SNP ID: rs6682716 /
SNP Chromosome Position: 156551848 / SNP in Genomic Sequence: SEQ ID NO: 393 /
SNP Position Genomic: 12329 / Related Interrogated SNP: hCV9102827 / SNP
Source: Applera / Population(Allele,Count): Caucasian (A,10|G,30) African
American (A,24|G,4) total (A,34|G,34) / SNP Type: MISSENSE MUTATION;ESS;ESE
SYNONYMOUS / SNP Source: Applera / Population(Allele,Count): Caucasian
(A,11|G,27) African American (A,14|G,2) total (A,25|G,29) / SNP Type:
MISSENSE MUTATION;ESS;ESE SYNONYMOUS / SNP Source: dbSNP; HapMap /
Population(Allele,Count): Caucasian (A,56|G,170) / SNP Type: MISSENSE
MUTATION;ESS;ESE SYNONYMOUS /
Context (SEQ ID NO: 1967): /
TGGGGAGCTGCCTATCTTCAGGCAATAGATGGGTAGGTAATAGGAATCATTTCCCTGGGGTAAAAAGAACCAAGAGGTGGCAC
AGGCCATGGTCAAAGGT
S
GGGAGAAAAGGGAGCTCAAGGAGCTGACCACTACTTACTTGTGTGCTGTTCGCCCCTCACAGGCTTGGAGCAGCATCTCATCA
GTCAGACTGTTAGGGGA / Celera SNP ID: hCV9102823 / Public SNP ID: rs12024215 /
SNP Chromosome Position: 156565834 / SNP in Genomic Sequence: SEQ ID NO: 393 /
SNP Position Genomic: 26315 / Related Interrogated SNP: hCV9102827 / SNP
Source: Applera / Population(Allele,Count): Caucasian (C,9|G,29) African
American (C,6|G,30) total (C,15|G,59) / SNP Type: UTR3;INTRON / SNP Source:
dbSNP; Celera; HapMap / Population(Allele,Count): Caucasian (G,92|C,28) / SNP
Type: UTR3;INTRON /
Context (SEQ ID NO: 1968): /
TCCTTGAGGAGCCACAGGGAGGTGTGCCATCCTGCATACTCGAACTTGGATAGGAGATGGGCTGCTGCTGCTGGGATGTGCAG

CAGCTTTGGCCTCTCTC
R
CCCACAGCTGGCTGGTGAGCAGAACAAGAAGAGGAAATGCTCTGGTTCATCAGGTCTTCACAGTGGACAAGTGCCAGTGACTG
ACAGTAAAGATGCCCCG / Celera SNP ID: hCV9102822 / Public SNP ID: rs4661052 /
SNP Chromosome Position: 156566491 / SNP in Genomic Sequence: SEQ ID NO: 393 /
SNP Position Genomic: 26972 / Related Interrogated SNP: hCV9102827 / SNP
Source: dbSNP; Celera; HapMap; HGBASE / Population(Allele,Count): Caucasian
(A,170|G,56) / SNP Type: UTR5;UTR3;INTRON /
Context (SEQ ID NO: 1969): /
CACCTGAGTTCATGGTCCTGGGTTAGAAGCTGCAGAAAGGACTGTGCTGACTCCCTCAACTAAGGGGTGGTACTACCGCTTGC
TCCTCTCAGCAGCTGAC
R
TCCCAGAGATCAGTTTCCTTTCTGGGAGGGACTCCTCATTGAGGGGGGTGCAGAAGACCTGCAGACCTGCATCAACCCTGGTC
TTTGAGGCTTCAACTCC / Celera SNP ID: hCV9102829 / Public SNP ID: rs3795732 /
SNP Chromosome Position: 156564640 / SNP in Genomic Sequence: SEQ ID NO: 393 /
SNP Position Genomic: 25121 / Related Interrogated SNP: hCV9102827 / SNP
Source: dbSNP; Celera; HapMap; ABI_Val; HGBASE / Population(Allele,Count):
Caucasian (G,56|A,168) / SNP Type: TRANSCRIPTION FACTOR BINDING
SITE;MICRORNA;UTR3 /
Context (SEQ ID NO: 1970): /
AAAAACAAAAAAAAACCTTCCCTTTACCTGTGTCTTAGCTTGGGCTATCATAATAAAATATCATAGATTGGATGCTCAAGCAA
CAAATTAATTTCCTGAC
R
GTTATGGGGACTGGAAGTCAGATTAGGGTTCCAGTATGGTGACAGAGAGGAGGGGCAGTGAGAGGAAGAAAGTCTTATAAGGG
CACTAATTCTACCAGTT / Celera SNP ID: hCV9102841 / Public SNP ID: rs4661188 /
SNP Chromosome Position: 156560502 / SNP in Genomic Sequence: SEQ ID NO: 393 /
SNP Position Genomic: 20983 / Related Interrogated SNP: hCV9102827 / SNP
Source: dbSNP; Celera; HapMap; HGBASE / Population(Allele,Count): Caucasian
(A,56|G,170) / SNP Type: INTERGENIC;UNKNOWN /
Context (SEQ ID NO: 1971): /
ACGTCTTGAGATTAGATAGCAGGAGTACCTAGATGATGAGTGAGGGGGGTGTCTGTGGAGGCACAGTGTGAAGGAAGTTATGG
AGGAGAGGCCCTGCATA
Y
GCGGTTCCTTCATCTATTTATTTGCAGCTTTTTTTTGAGACAGAGTTTCTCTGTCACCCAGGCTGGAGTGCAATGGCGCGATCT
CAGCTCACTGCAACCTC / Celera SNP ID: hCV9102976 / Public SNP ID: rs10908509 /
SNP Chromosome Position: 156550254 / SNP in Genomic Sequence: SEQ ID NO: 393 /
SNP Position Genomic: 10735 / Related Interrogated SNP: hCV9102827 / SNP
Source: dbSNP; Celera; HapMap / Population(Allele,Count): Caucasian
(T,173|C,53) / SNP Type: INTERGENIC;UNKNOWN //

Gene Number: 57 / Gene Symbol: ASPM - 259266 / Gene Name: asp (abnormal
spindle) homolog, microcephaly associated (Drosophila) / Chromosome: 1 / OMIM
NUMBER: 605481 / OMIM Information: Microcephaly, primary autosomal recessive,
5, 608716 (3) // Genomic Sequence (SEQ ID NO: 394): // / SNP Information /
Context (SEQ ID NO: 1972): /
TAGCCTTAGCTCTTAACTATTTAATATAAGAAGTTTGTTGAATAACTATTAAATGTCCAAAGCTACAGTATAATGTTATTTCT
TTATTTACCAAGACTGC
Y
TCTTTTAAATTTACCAAACATTCCATTCTTATTCATATGTTGTTTCTCCACTGAAAAGCACATCTTGTTTATGGTAAGTGCTC
AATAAATATTTGTTAAA / Celera SNP ID: hCV201028 / Public SNP ID: rs10733087 /
SNP Chromosome Position: 197055782 / SNP in Genomic Sequence: SEQ ID NO: 394 /
SNP Position Genomic: 12525 / Related Interrogated SNP: hCV2532034 / SNP
Source: Applera / Population(Allele,Count): Caucasian (C,26|T,4) African
American (C,17|T,9) total (C,43|T,13) / SNP Type: INTRON / SNP Source:
dbSNP; Celera; HapMap / Population(Allele,Count): Caucasian (T,8|C,112) / SNP
Type: INTRON /
Context (SEQ ID NO: 1973): /
TTTCTCCACTGAAAAGCACATCTTGTTTATGGTAAGTGCTCAATAAATATTTGTTAAATGAATGAATGAACAGTGACACAAAT
TTATGGACTATATTTAA
Y
TGCTTACCTTGAAACTATTCTGGTCCTTACAGGTGTTTCTGGGATAAAAGGAATGCTTATAGAAGAATTCTTCTTTTGCTTGT
AAAGTATTCTTTCAGTA / Celera SNP ID: hCV201029 / Public SNP ID: rs10754213 /
SNP Chromosome Position: 197055925 / SNP in Genomic Sequence: SEQ ID NO: 394 /

SNP Position Genomic: 12668 / Related Interrogated SNP: hCV2532034 / SNP Source: Applera / Population(Allele,Count): Caucasian (C,24|T,4) African American (C,12|T,4) total (C,36|T,8) / SNP Type: DONOR SPLICE SITE;INTRON / SNP Source: dbSNP; Celera; HapMap / Population(Allele,Count): Caucasian (T,19|C,207) / SNP Type: DONOR SPLICE SITE;INTRON /
Context (SEQ ID NO: 1974): /
CGATAATGGGTTTTGTCCTTTTGTTTGTTTTAGAAATTGGAGTTCTGAATATTGATAAATCTAAAATAAATTAGAAAACAAAA
CTAAGAGCATTAAAATA
Y

AAATTCCCTAAATAGGTCAGTGATTTGAAAGTTAAAGTTTGGGTTCTTCACAATCATATTCATTTAGCAAACACTTAATAACC
ACTGTACTCCACGCACT / Celera SNP ID: hCV247773 / Public SNP ID: rs1332663 /
SNP Chromosome Position: 197109042 / SNP in Genomic Sequence: SEQ ID NO: 394 /
SNP Position Genomic: 65785 / Related Interrogated SNP: hCV2532034 / SNP Source: Applera / Population(Allele,Count): Caucasian (C,1|T,21) African American (C,12|T,22) total (C,13|T,43) / SNP Type: INTRON / SNP Source: dbSNP; Celera; HapMap; HGBASE / Population(Allele,Count): Caucasian (C,20|T,206) / SNP Type: INTRON /
Context (SEQ ID NO: 1975): /
CTTTGTGTTTTTCCCTTAAAAGTTGTCTTGCTTTCATTCCTTTATATGCAGCCTGAATAACCACAGCAGAATGCCATTGTCTG
ATATAATTTTCTCTTTG
Y

AATTTTGCAGCTCTATATGTTCGATAATGTTGCTGAATTAGAATTGAAGCATGTTTCCAAGTCTGAAATGTAATATATGTTCT
GTACATCCTGAAAGTAG / Celera SNP ID: hCV2759703 / Public SNP ID: rs1412640 /
SNP Chromosome Position: 197070815 / SNP in Genomic Sequence: SEQ ID NO: 394 /
SNP Position Genomic: 27558 / Related Interrogated SNP: hCV2532034 / SNP Source: Applera / Population(Allele,Count): Caucasian (C,35|T,3) African American (C,25|T,11) total (C,60|T,14) / SNP Type: SILENT MUTATION;INTRON / SNP Source: dbSNP; Celera; ABI_Val; HGBASE / Population(Allele,Count): Caucasian (T,20|C,206) / SNP Type: SILENT MUTATION;INTRON /
Context (SEQ ID NO: 1976): /
ACATGAATTTTAACATGAAGACTGCCTGCAATTAATTTTAGCTACAATACAAGCAATTTAAAATCTGACAAAAATGTACCAGG
GTCAGCTAAAGAGTTTG
K

TAAGTTTTACTGTAGCAGAGATATTTTATAAAGACCATTAAATCTGTGCTCCCCATACGTCAACATTTAACTAGCTGTTTTGA
GGCATAGCAACTTCCTA / Celera SNP ID: hCV65774 / Public SNP ID: rs7534353 / SNP Chromosome Position: 197096480 / SNP in Genomic Sequence: SEQ ID NO: 394 /
SNP Position Genomic: 53223 / Related Interrogated SNP: hCV2532034 / SNP Source: dbSNP; Celera; ABI_Val / Population(Allele,Count): Caucasian (T,9|G,107) / SNP Type: INTRON /
Context (SEQ ID NO: 1977): /
GTGAGTTGTATAAAATAATTAAAGACAAAATAATAACATGTCAACAATCTCTTCCAGAAAACATAAAAGAACGCTGAAACTTC
CCAACTCATTACACAGA
Y

GCAAAACTCTTCAATAAAATACTAGCAAGTGGAATTAATGAAATATCAATGCATATAAAAAATACATAACTAATATTTGAATT
TTATCCTTGGAATGTAA / Celera SNP ID: hCV240531 / Public SNP ID: rs6703400 /
SNP Chromosome Position: 197078400 / SNP in Genomic Sequence: SEQ ID NO: 394 /
SNP Position Genomic: 35143 / Related Interrogated SNP: hCV2532034 / SNP Source: dbSNP; Celera; HapMap / Population(Allele,Count): Caucasian (T,20|C,206) / SNP Type: INTRON /
Context (SEQ ID NO: 1978): /
TAGAATGAAAAATTATAAAACTTTTAGAGTACTTTGATATGACGCTAAAAGCATAATCCATAAAAGAAGAAAAGTGATAACTG
GATTCCAATAAAATGAA
M

TACTTCTGCTGAACAAAAAGCCCTGTTACAAGAATAAAAAGACAAGCTGCAGATTGGGAGAAATTGTGTGAAATCACTAACAA
AGAAGGAGGATTACCTA / Celera SNP ID: hCV240532 / Public SNP ID: rs2026429 /
SNP Chromosome Position: 197079671 / SNP in Genomic Sequence: SEQ ID NO: 394 /
SNP Position Genomic: 36414 / Related Interrogated SNP: hCV2532034 / SNP Source: dbSNP; Celera; HapMap; ABI_Val; HGBASE / Population(Allele,Count): Caucasian (C,20|A,206) / SNP Type: INTRON /
Context (SEQ ID NO: 1979): /
TAGCAAAAAACTAAAGAATACAGACGCACAAAAGATACTTTTAATAAAAATGTCTTTAAACAGCCAATTACTAATAGTATCTT
TCTAATTCAGTCAGTTT
M

AACATTCTAGGTATTACAGAGCCAAGAACTATGTAAAGAATGCAAATCCTGATGACATTTTTATGAGTACTATTTAATCAGAT
TGGTATAGGTTCAACTC / Celera SNP ID: hCV489039 / Public SNP ID: rs6679189 /
SNP Chromosome Position: 197100556 / SNP in Genomic Sequence: SEQ ID NO: 394 /
SNP Position Genomic: 57299 / Related Interrogated SNP: hCV2532034 / SNP
Source: dbSNP; Celera; HapMap / Population(Allele,Count): Caucasian
(C,9|A,111) / SNP Type: INTRON /
Context (SEQ ID NO: 1980): /
AAGAGCACGGTGATCATAAATAGCTATGACAGAAGCTGTCTGTAGCTTGAAAAAACATAGCATACCACTAGCACACCTTAGAT
TCCTCAATTACGTAACA
W
CCTGTGAGAATTACCAAAATGAGACAGAGCCACAAAGTGAGCACATGATGCTGGAAATATGGCACCAACAGACTTGCTCAACA
CAGGGTTGACAGAAACC / Celera SNP ID: hCV2759676 / Public SNP ID: rs4915156 /
SNP Chromosome Position: 197062820 / SNP in Genomic Sequence: SEQ ID NO: 394 /
SNP Position Genomic: 19563 / Related Interrogated SNP: hCV2532034 / SNP
Source: dbSNP; Celera; HapMap; HGBASE / Population(Allele,Count): Caucasian
(A,8|T,112) / SNP Type: INTRON /
Context (SEQ ID NO: 1981): /
TGTGAGAATTACCAAAATGAGACAGAGCCACAAAGTGAGCACATGATGCTGGAAATATGGCACCAACAGACTTGCTCAACACA
GGGTTGACAGAAACCTT
Y
AATTTATAAAAAATGCAATATCTGCAAAGCACCATAAAGCGAAGTACAATAAAACAAGGTATGCCTGTACAGTTATATATTTT
TAAAAACTAGCAAATAG / Celera SNP ID: hCV2759677 / Public SNP ID: rs10801588 /
SNP Chromosome Position: 197062923 / SNP in Genomic Sequence: SEQ ID NO: 394 /
SNP Position Genomic: 19666 / Related Interrogated SNP: hCV2532034 / SNP
Source: dbSNP; Celera; HapMap / Population(Allele,Count): Caucasian
(T,6|C,108) / SNP Type: INTRON /
Context (SEQ ID NO: 1982): /
TTGTAGCTATTGAATATTAAATAAGCAATATTACTTCAAGCCTGTTATCAAAATAAGTTTTATTTATTATTAAACAATTTCTT
AGAGTAAATCACAGAAT
R
GTTAAAACATTACCTTTTTCTTTTTCTGCTCTTCTGCATTTCCTAGTAATATAGCTTGGTGTTTCAGAACATCATTTACAAGA
AATGTCATAATCTCTCT / Celera SNP ID: hCV2759678 / Public SNP ID: rs1571964 /
SNP Chromosome Position: 197113073 / SNP in Genomic Sequence: SEQ ID NO: 394 /
SNP Position Genomic: 69816 / Related Interrogated SNP: hCV2532034 / SNP
Source: dbSNP; Celera; HapMap; ABI_Val; HGBASE / Population(Allele,Count):
Caucasian (G,20|A,206) / SNP Type: INTRON /
Context (SEQ ID NO: 1983): /
TTAGAAAATGTATTTGGCTTTGTGTAATGTTCAAAGTTGAAGAACAGTTGGGGGTAAGACTAAGTTTACTATTCTCTCCTCTT
TGGCCATTAACATTTAC
R
GAATTAAAGGAAGTTTCAGTTACAGCTTTCTCATTAAAAGAAACTTTTGAAACGTTGGCACTGTGTACATTTAATAGTTCCCT
ATTTTCTGATGCATGAA / Celera SNP ID: hCV2759679 / Public SNP ID: rs6677082 /
SNP Chromosome Position: 197112533 / SNP in Genomic Sequence: SEQ ID NO: 394 /
SNP Position Genomic: 69276 / Related Interrogated SNP: hCV2532034 / SNP
Source: dbSNP; Celera; HapMap / Population(Allele,Count): Caucasian
(G,20|A,206) / SNP Type: SILENT MUTATION;INTRON /
Context (SEQ ID NO: 1984): /
TGGTGTTTAGTTTCCTCATAATCCTATAGCTTTCCAACATACTTCTTAAACTAAGGTATTGAACAGTGGGGACAATATGCCCA
AAGTGGGCAGGCCACAC
R
GGACTGATTGCATTGACTGATGAGAGAGAATAGAGAACAAAAAGTATATTTCAGTACTTTCGGTAAAGAAAATGTACATATAT
AAATTTATGTAAATAAA / Celera SNP ID: hCV2759685 / Public SNP ID: rs877897 /
SNP Chromosome Position: 197103975 / SNP in Genomic Sequence: SEQ ID NO: 394 /
SNP Position Genomic: 60718 / Related Interrogated SNP: hCV2532034 / SNP
Source: dbSNP; Celera; HapMap; ABI_Val; HGBASE / Population(Allele,Count):
Caucasian (G,9|A,111) / SNP Type: INTRON /
Context (SEQ ID NO: 1985): /
CATCTAAGCTTCTGTTTCGTTAGTGAAGTGGAAATAATAATACATACTTCACTGGGTTTTAAGAAATTTAAATTAGAAAATGT
ATTCACAGCAGCCTCGT
Y
TTGGCATTTGTAAATTTGCAAAAATGAGTTTACTATAGTTGTCATTACGTGCAACACCAAATTATGTTTAAAGAGACTGTACA
GTAAAATGTTGTATTAC / Celera SNP ID: hCV2759688 / Public SNP ID: rs10922169 /
SNP Chromosome Position: 197101105 / SNP in Genomic Sequence: SEQ ID NO: 394 /

SNP Position Genomic: 57848 / Related Interrogated SNP: hCV2532034 / SNP Source: dbSNP; Celera; HapMap / Population(Allele,Count): Caucasian (T,9|C,111) / SNP Type: TFBS SYNONYMOUS;INTRON / Context (SEQ ID NO: 1986): /
ATGCAGGAGGAAAGGAGAAATTAGCCGTAGCTTCAAATCAGACATGGCAAAAAATTGGAAAAGTAACCAAAAGGGACTAACCATGATCAAATGCTTTAAG
W
GACATATCCAGAGAACTGTCATCAGATTCAGATGATGAATTTAATACCACTGAACCAGTTTGCGTACATTCCACAGTTTGAGTAGTACGCTGACATATAG / Celera SNP ID: hCV2759691 / Public SNP ID: rs4915337 / SNP Chromosome Position: 197091537 / SNP in Genomic Sequence: SEQ ID NO: 394 / SNP Position Genomic: 48280 / Related Interrogated SNP: hCV2532034 / SNP Source: dbSNP; Celera; HapMap; ABI_Val; HGBASE / Population(Allele,Count): Caucasian (A,8|T,110) / SNP Type: UTR3;ESS SYNONYMOUS;SILENT MUTATION;INTRON / Context (SEQ ID NO: 1987): /
TTCCAGGACTCAAAGCAAAGATAAAAAAGAACCTGGTTGGTTATGTGGATCTACAAAGTAGAATGGTTCAGTAGGCCAGCATGTCCCACAGCGTAGAAGA
Y
GCAACAAAGAAGAGAGCAACAGGTACAGAATTAGAATGTTTGCTTTTTTCAGAGCAAATGCATTTCAATTATGTTTCAGCCTTCTGCTGAACACCATAACG / Celera SNP ID: hCV2759693 / Public SNP ID: rs10754215 / SNP Chromosome Position: 197086669 / SNP in Genomic Sequence: SEQ ID NO: 394 / SNP Position Genomic: 43412 / Related Interrogated SNP: hCV2532034 / SNP Source: dbSNP; Celera; HapMap / Population(Allele,Count): Caucasian (C,20|T,204) / SNP Type: INTRON / Context (SEQ ID NO: 1988): /
TGAGCATATCCTGTTGGAAAAAGTGTTGCAGACTTGCTTAACACAGGGTTGCCACAAACCTTCAGTTCGTAAAACACTCAATAGCTGCAAAGCACAATAA
W
GCAAAGAGCAATAAAACGAGGTATGCCTATATGCCTTAAGAACAATTGTACTCCTAGTACAATTGAAGAGACTGAATACATTTCACACCAAAAGACATGT / Celera SNP ID: hCV2759696 / Public SNP ID: rs7411719 / SNP Chromosome Position: 197085676 / SNP in Genomic Sequence: SEQ ID NO: 394 / SNP Position Genomic: 42419 / Related Interrogated SNP: hCV2532034 / SNP Source: dbSNP; Celera; HapMap / Population(Allele,Count): Caucasian (T,9|A,111) / SNP Type: TRANSCRIPTION FACTOR BINDING SITE;INTRON / Context (SEQ ID NO: 1989): /
TTCAAAGAAAAAAAGAATGAGTAAGAAAACAAATTTTGCCAGTAGTTAAGTCACCCAGCAAAATGGCCCAAAGCACTAATAAGAAAACCAAGTCTTAACT
R
AATGATTTTATTCACTTCCTACGTCTCTGTCCTTACTCTAATCCTTATGAACCTGAATTAATCAAGTATATAATATTTTTAGATTATATTAATTAAAGGC / Celera SNP ID: hCV2759704 / Public SNP ID: rs1953064 / SNP Chromosome Position: 197064826 / SNP in Genomic Sequence: SEQ ID NO: 394 / SNP Position Genomic: 21569 / Related Interrogated SNP: hCV2532034 / SNP Source: dbSNP; Celera; HapMap; HGBASE / Population(Allele,Count): Caucasian (A,8|G,112) / SNP Type: INTRON / Context (SEQ ID NO: 1990): /
TTGCAAGTGTTCCCCAAAGTTCGTATGTTGGAAACGTAATCTCCAATGTAAAGGTGTTGGGAGGTGGGACATTTAAGATATAATTAGGTCGTGAGATCTC
K
ATCCTCATGGAAGGATTAATGCTATTTTCAAGGGGGGTAGGTTAGTTATTGTGGGTGTTAGTTCCTAATAAAAGGAAGCATTTGGCCCCCATGATGGTTA / Celera SNP ID: hCV2759709 / Public SNP ID: rs4915313 / SNP Chromosome Position: 197049355 / SNP in Genomic Sequence: SEQ ID NO: 394 / SNP Position Genomic: 6098 / Related Interrogated SNP: hCV2532034 / SNP Source: dbSNP; Celera; HapMap / Population(Allele,Count): Caucasian (G,9|T,111) / SNP Type: INTERGENIC;UNKNOWN / Context (SEQ ID NO: 1991): /
AAATTATCACAAATCTCCAAAAAGTTTTCCAACATATATATTGAAAAAAAAAATCTGCATAGGAGTGCACTTACATGGTTTAAACCCATGTTATTAAAGAG
W
CAACTGTACTGCTTTTGAAAATTCTTTCATCAGTGAACACTTGTTTCCATATCTTGACTACTGTAAATAATCCTGCAATGAACATTGGAGTACAGACACC / Celera SNP ID: hCV2759711 / Public SNP ID: rs4915309 / SNP Chromosome Position: 197048781 / SNP in Genomic Sequence: SEQ ID NO: 394 / SNP Position Genomic: 5524 / Related Interrogated SNP: hCV2532034 / SNP Source: dbSNP; Celera; HapMap / Population(Allele,Count): Caucasian (A,9|T,109) / SNP Type: INTERGENIC;UNKNOWN /

Context          (SEQ ID NO: 1992): /
TTTTCATTTTCCACTTGGAAAAACTCCCCAAGGCTAGTGACGGCCTCTGACCATCCTTTTACCAACTTTCACTTCATCCATACT
CCCTACCACAGCAAAGG
R
TAACAGCCCATGTTTTTGGCCGGGCGCGGTGGCTCACGCCTGAAATCCCAGCACCCTGGGAGGCGAGGGGAGGGATCACGAGG
TCAGGAGATTGCCCGAC / Celera SNP ID:  hCV8348876 / Public SNP ID:   rs1332662 /
SNP Chromosome Position:   197108427 / SNP in Genomic Sequence:   SEQ ID NO: 394 /
 SNP Position Genomic:  65170 / Related Interrogated SNP:   hCV2532034 / SNP
Source:  dbSNP; HapMap; ABI_Val; HGBASE / Population(Allele,Count):   Caucasian
(A,9|G,111) / SNP Type:   INTRON /
Context          (SEQ ID NO: 1993): /
TAGTTTATTACATGCATAAAACTATAAATGATAAAAATGAAGAATGTAATGAACAGTTTATGTTTTTTTAAAACAAAGTCAGA
TTTTAAAAGTTGTACAC
R
GAGAGCAAAAATCACTTTACGTACTCATGATTGGCTTTAATATTTCTTTACACTATACATACTGAAAATGTTTACATTTACTA
ATAAGGAATGCCAAGCG / Celera SNP ID:  hCV8356417 / Public SNP ID:   rs12677 / SNP
Chromosome Position:   197053373 / SNP in Genomic Sequence:   SEQ ID NO: 394 /
SNP Position Genomic:   10116 / Related Interrogated SNP:   hCV2532034 / SNP
Source:   dbSNP; Celera; HapMap; HGBASE / Population(Allele,Count):   Caucasian
(G,20|A,206) / SNP Type:   UTR3 /
Context          (SEQ ID NO: 1994): /
CCCAGGGATTCTAACTCCCATGGGCAGACTTTGAGACTCTATTCAGATTTCAGCCATAAGAATTAGACATCTGTGGCCCTGAA
ACCACAGATACTAGTAA
Y
AGTGACTCAAAGAGTAGAAAAAAAAATCACATTTCAATACTGAATCATTTTCAGGTCTGTGGTGTATAACTACACAACAAGATT
CTATTATAAGCAATAGC / Celera SNP ID:  hCV8356418 / Public SNP ID:   rs1537319 /
SNP Chromosome Position:   197052804 / SNP in Genomic Sequence:   SEQ ID NO: 394 /
 SNP Position Genomic:  9547 / Related Interrogated SNP:   hCV2532034 / SNP
Source:   dbSNP; Celera; ABI_Val / Population(Allele,Count):   Caucasian
(T,8|C,112) / SNP Type:   INTERGENIC;UNKNOWN /
Context          (SEQ ID NO: 1995): /
CTTACTTAATATTTATAAATATCTTATAAAATGGTTAAGATCTCTGTTTTATAAAAAAGGAAAAAAATCCAAAATTGAAATTA
AGAAGGACTTGTTAGCA
Y
ATAGCTGTTAAGTGACAGAGTCAAGATTTGAACCCAAGTCTATTTGACTCCAACATCCCTACAGGAAGAGGAATTAAAAGAAA
TTAAAGACTGTGTAAGC / Celera SNP ID:  hCV9114466 / Public SNP ID:   rs3891964 /
SNP Chromosome Position:   197066074 / SNP in Genomic Sequence:   SEQ ID NO: 394 /
 SNP Position Genomic:  22817 / Related Interrogated SNP:   hCV2532034 / SNP
Source:  dbSNP; Celera; HapMap / Population(Allele,Count):   Caucasian
(T,19|C,207) / SNP Type:   INTRON /
Context          (SEQ ID NO: 1996): /
CTCTTGCCTTGACCCCAAAAGTGCTTCCTTGATAAGGATATAATATATATTTTGGAGAGTTCCCTAGAAATAAAATGAGTATC
TCCAACCCAGATACCCT
R
AAGAGGCCAATCATGGCAAAATGCTAAAGGCCATTCCTTCTAAGCAAAACTGGCTATCCATCATACATACCAAAGCCAATTTT
GAGCATTCCAGTTTGTT / Celera SNP ID:  hCV11888556 / Public SNP ID:   rs7542397 /
SNP Chromosome Position:   197096720 / SNP in Genomic Sequence:   SEQ ID NO: 394 /
 SNP Position Genomic:  53463 / Related Interrogated SNP:   hCV2532034 / SNP
Source:  dbSNP; Celera; HapMap / Population(Allele,Count):   Caucasian
(G,9|A,111) / SNP Type:   INTRON /
Context          (SEQ ID NO: 1997): /
TGCAACCATTTTCTTCTTTAGGAAGTCTTTCTTGGAACCATCTCATACTTCCTTGCAATTTACACACAAACCCTCTGCTTCTG
TGCTTCCTTAATTTCCT
R
AGCATAGCATAATCACAGGACTGAACGCAACTTGAGGACAGGAATTATCCCTTTCCTCCCTGTGTACACAGTGCCTAGAAAAA
TGCACATCACATAGTAG / Celera SNP ID:  hCV11888566 / Public SNP ID:   rs1888991 /
SNP Chromosome Position:   197110822 / SNP in Genomic Sequence:   SEQ ID NO: 394 /
 SNP Position Genomic:  67565 / Related Interrogated SNP:   hCV2532034 / SNP
Source:  dbSNP; Celera; HapMap; HGBASE / Population(Allele,Count):   Caucasian
(A,21|G,203) / SNP Type:   INTRON /
Context          (SEQ ID NO: 1998): /
TAAGATAATGATAAAGGCAGGATCTAATTAGTTACTCTATTACATGCTCCTTGAGAGGAAGAAGAGCTTGCTCGAAAATAAAA
CAAAATACATATGATGC

369

Y
AGTAAGTCTAGGATATATTTTCCACGCCTCATTTTTCTAATTGTATTATTAGATTCATAGTGAAATTTGCATGGTAACAAATA
ATGATACTAAGATAAAA / Celera SNP ID: hCV15832928 / Public SNP ID: rs2151133 /
SNP Chromosome Position: 197058036 / SNP in Genomic Sequence: SEQ ID NO: 394 /
SNP Position Genomic: 14779 / Related Interrogated SNP: hCV2532034 / SNP
Source: dbSNP; HapMap; HGBASE / Population(Allele,Count): Caucasian
(T,8|C,112) / SNP Type: INTRON /
Context (SEQ ID NO: 1999): /
ATGAATATATAAAGCTCTCTGGTAAAGGTAAATATATAAACAAGCATAAAAACAGTATTATTGTAATTTTGGTTTGTAACTCC
GCTTTTTATTTTCTACA

K
GATTTAAAAGGCAAATGCATAAAATGTAATTATAAATATGTTAGGTGGTGTACAATGAATAAAGATATAATCTGTGACATCAA
TAACCTAAAAAGAGTAG / Celera SNP ID: hCV15832929 / Public SNP ID: rs2151134 /
SNP Chromosome Position: 197105536 / SNP in Genomic Sequence: SEQ ID NO: 394 /
SNP Position Genomic: 62279 / Related Interrogated SNP: hCV2532034 / SNP
Source: dbSNP; Celera; HapMap / Population(Allele,Count): Caucasian
(T,9|G,107) / SNP Type: INTRON /
Context (SEQ ID NO: 2000): /
CCAATAAAAACCCTAGAAGAAAACCTAGGAAATACTACTCCGGATATCAGCCTTGGGAAAGAATTTATAACAAAGTCCCCAAA
AGCAATTGCAACAAAAA

M
CATAATTGACAAATGGGACCTAATTAAACTAAAGAGATTCTGCACAGCAAAATAAACTATCAACAGAATAAACAGACAACCTA
CAGAATGGGAGAAAATA / Celera SNP ID: hCV26753641 / Public SNP ID: rs10801590 /
SNP Chromosome Position: 197080203 / SNP in Genomic Sequence: SEQ ID NO: 394 /
SNP Position Genomic: 36946 / Related Interrogated SNP: hCV2532034 / SNP
Source: dbSNP; Celera; HapMap / Population(Allele,Count): Caucasian
(A,9|C,109) / SNP Type: INTRON /
Context (SEQ ID NO: 2001): /
AATGAAATTATGGTAGTATAGTGCTTATGAGTTATTCTACCGGCTAATGCGTTAGCTTTTTAAAATGGTAACTATGTTTTTAT
AGTGTTAGATATCATGT

R
GATGAATTGCAAACTAAAGTTTTGAACTAAAACTATACAAGTTTCAATTACCTGAATTTTAATGATTCCACTAGTGAATTTTT
CCTGCTTTTTACGGAGG / Celera SNP ID: hCV27898326 / Public SNP ID: rs4915316 /
SNP Chromosome Position: 197059928 / SNP in Genomic Sequence: SEQ ID NO: 394 /
SNP Position Genomic: 16671 / Related Interrogated SNP: hCV2532034 / SNP
Source: dbSNP; HapMap; HGBASE / Population(Allele,Count): Caucasian
(A,18|G,206) / SNP Type: INTRON /
Context (SEQ ID NO: 2002): /
CTAACAAAAGCTGATGATGAACAACTCAAAGATCTAAATAAATGGAGATATGTATCACCTTTATGAATCAGAATAATCAACA
TAGTAAAGTTGTCAGTG

Y
ACCTCAAAATATCTGTAAATGTGATGCAATTTCAATCAAAATTTGACAGTATTTTTTGAGGACATAGAAAAACTAACCTAAAA
CTTAAATGGAAAGACAA / Celera SNP ID: hCV27898327 / Public SNP ID: rs4915327 /
SNP Chromosome Position: 197079130 / SNP in Genomic Sequence: SEQ ID NO: 394 /
SNP Position Genomic: 35873 / Related Interrogated SNP: hCV2532034 / SNP
Source: dbSNP; HapMap; HGBASE / Population(Allele,Count): Caucasian
(T,9|C,111) / SNP Type: INTRON /
Context (SEQ ID NO: 2003): /
CTTCTTGATTATAAACCTCCTCTAGAGCATTTGAAAATAAAGAGCTTAGCAATGAAATTATGGTAGTATAGTGCTTATGAGTT
ATTCTACCGGCTAATGC

R
TTAGCTTTTTAAAATGGTAACTATGTTTTTATAGTGTTAGATATCATGTAGATGAATTGCAAACTAAAGTTTTGAACTAAAAC
TATACAAGTTTCAATTA / Celera SNP ID: hCV29222813 / Public SNP ID: rs4915315 /
SNP Chromosome Position: 197059878 / SNP in Genomic Sequence: SEQ ID NO: 394 /
SNP Position Genomic: 16621 / Related Interrogated SNP: hCV2532034 / SNP
Source: dbSNP; HapMap; HGBASE / Population(Allele,Count): Caucasian
(G,11|A,109) / SNP Type: INTRON /
Context (SEQ ID NO: 2004): /
ACTGCCCTATTAGAGAAGGGTATAAATATTAAACTCAAAAAATGTTACAGAATTCCAAGCAGAACTCTTGGAAATGGTGATAT
TAAATCAGAAAAAGTAT

R
TGAATTAACTTCTCTACTATTAAATAGTACAGTGCAACTCCAGCATAAGACCCTGCAGAAGGAGCTTTCTACAGTTGCTGTTG
GGCTTCAGACTTTCACT / Celera SNP ID: hCV29222814 / Public SNP ID: rs6428387 /

SNP Chromosome Position: 197061557 / SNP in Genomic Sequence: SEQ ID NO: 394 / SNP Position Genomic: 18300 / Related Interrogated SNP: hCV2532034 / SNP Source: dbSNP; HapMap / Population(Allele,Count): Caucasian (A,8|G,112) / SNP Type: INTRON /
Context (SEQ ID NO: 2005): /
TAGAACAGATAGTCTCTAAATCATTTTGTTTGAGTGGTTTCAAAAGATTTAATTGTACATTTCTAATTTTTAGATGTGTAGAG
TACTTCTACTTCAAACA
Y
AAGGACCCTGCATTATGAAGACTTTATTTAAAAAGAAATGTAAAGATGATCAGCTAAATTCCTTCTTCCTTACAAATTCCTCT
AAGGTCCACATCATCAT / Celera SNP ID: hCV29222817 / Public SNP ID: rs6656858 /
SNP Chromosome Position: 197113573 / SNP in Genomic Sequence: SEQ ID NO: 394 / SNP Position Genomic: 70316 / Related Interrogated SNP: hCV2532034 / SNP Source: dbSNP; HapMap / Population(Allele,Count): Caucasian (C,20|T,206) / SNP Type: INTRON /
Context (SEQ ID NO: 2006): /
GTGAATGGTCATAAACACCTGGTGGGTTTTAAATCTTTACAAGTACTTTAAAAAACTCTTGGATAGTTGAAATATATACAGGC
ATATCTCGAAGATATTG
Y
AGGTTCAGTTCCAGACCACCACAATAAAGCAAATATTACAATAAAGTGAGTCACACAATTTTTTTTGGTTTCCCAATACATACC
GAAGTTATCTTTACACT / Celera SNP ID: hCV31565488 / Public SNP ID: rs6671696 /
SNP Chromosome Position: 197083196 / SNP in Genomic Sequence: SEQ ID NO: 394 / SNP Position Genomic: 39939 / Related Interrogated SNP: hCV2532034 / SNP Source: dbSNP; HapMap / Population(Allele,Count): Caucasian (T,7|C,103) / SNP Type: INTRON /
Context (SEQ ID NO: 2007): /
ATATTACAATAAAGTGAGTCACACAATTTTTTTGGTTTCCCAATACATACCGAAGTTATCTTTACACTATACTGCAGTCTATT
AAGTGTGCAAAACCATT
R
TGTCTAAAACACTACAAGGTACATACCTAAATTTTAAAAATACTAAATTGCTAAAAAAATGCTAAGAACCATGTGAGCCTTCAGC
AAATTGTAATCTTTTTG / Celera SNP ID: hCV29904681 / Public SNP ID: rs6680497 /
SNP Chromosome Position: 197083329 / SNP in Genomic Sequence: SEQ ID NO: 394 / SNP Position Genomic: 40072 / Related Interrogated SNP: hCV2532034 / SNP Source: dbSNP; HapMap / Population(Allele,Count): Caucasian (G,9|A,111) / SNP Type: INTRON /
Context (SEQ ID NO: 2008): /
GTACTTCCTTTCATTTGAAAGGAAAAATAGATATCATCAAAATATCTAAGTAGAGATATTTCGTAAACAGAACTGGAGAAAAA
TTTTAAAAAATCTGAGC
R
CTAGAGATTAAATTTTAGGGATCAACATAATACTAAGAGATAAAATGTGAGAATGGACAGGTTTTCTACTCTGTTCTTGATAA
TGTGGAAAAAGTACAAT / Celera SNP ID: hCV29633649 / Public SNP ID: rs7539642 /
SNP Chromosome Position: 197063841 / SNP in Genomic Sequence: SEQ ID NO: 394 / SNP Position Genomic: 20584 / Related Interrogated SNP: hCV2532034 / SNP Source: dbSNP; HapMap / Population(Allele,Count): Caucasian (A,8|G,112) / SNP Type: INTRON /
Context (SEQ ID NO: 2009): /
ACCTATAATAAGCTACTGAGCTGGGATTCAAACTCAAGCAGTTTGGCCTCAGATCTGTGTTCTTAACCACTACCTCTCAAAAT
AAAAATATTGGGTAAAG
Y
GCTTTAAGTTGTCACTGAAAGGTTGAGTAGGAAAGAGAAATCTTTCAACCTTTGGTTGCATACAATATAGTTTGGAATAGTCC
AAGGCCAAAAGACAAAT / Celera SNP ID: hCV31565477 / Public SNP ID: rs10801587 /
SNP Chromosome Position: 197058380 / SNP in Genomic Sequence: SEQ ID NO: 394 / SNP Position Genomic: 15123 / Related Interrogated SNP: hCV2532034 / SNP Source: dbSNP; HapMap / Population(Allele,Count): Caucasian (C,8|T,108) / SNP Type: INTRON /
Context (SEQ ID NO: 2010): /
ACCTAAAACCACCTGATCTTTGACATAGTTGACAATAACAAACAATGGGGAAAGGATATGCTATTCAATAAACGGTCAGTGCA
GAAGATTAAAATTGGAC
S
CTTTCCTTTCACCACATACAAAAAATTAATGCAAGATGGATTAAAGACTTAAATGTAAGACCCAAACCAATAAAAACCCTAGA
AGAAAACCTAGGAAATA / Celera SNP ID: hCV31565485 / Public SNP ID: rs10922166 /
SNP Chromosome Position: 197080036 / SNP in Genomic Sequence: SEQ ID NO: 394 / SNP Position Genomic: 36779 / Related Interrogated SNP: hCV2532034 / SNP Source: dbSNP; HapMap / Population(Allele,Count): Caucasian (G,9|C,111) / SNP

Type: INTRON /
Context (SEQ ID NO: 2011): /
ACCTCCTCTAGAGCATTTGAAAATAAAGAGCTTAGCAATGAAATTATGGTAGTATAGTGCTTATGAGTTATTCTACCGGCTAA
TGCGTTAGCTTTTTAAA
R
TGGTAACTATGTTTTTATAGTGTTAGATATCATGTAGATGAATTGCAAACTAAAGTTTTTGAACTAAAAACTATACAAGTTTCAA
TTACCTGAATTTTAATG / Celera SNP ID: hCV31565478 / Public SNP ID: rs10754214 /
SNP Chromosome Position: 197059892 / SNP in Genomic Sequence: SEQ ID NO: 394 /
SNP Position Genomic: 16635 / Related Interrogated SNP: hCV2532034 / SNP
Source: dbSNP; HapMap / Population(Allele,Count): Caucasian (A,19|G,207) /
SNP Type: TFBS SYNONYMOUS;INTRON //

Gene Number: 58 / Gene Symbol: CYP4V2 - 285440 / Gene Name: cytochrome
P450, family 4, subfamily V, polypeptide 2 / Chromosome: 4 / OMIM NUMBER:
608614 / OMIM Information: Bietti crystalline corneoretinal dystrophy, 210370
(3) // Genomic Sequence (SEQ ID NO: 395): // / SNP Information /
Context (SEQ ID NO: 2012): /
AATGAGTGAGATGATATTTCGAAGAATAAAGATGCCCTGGCTTTGGCTTGATCTCTGGTACCTTATGTTTAAAGAAGGATGGG
AACACAAAAAGAGCCTT
M
AGATCCTACATACTTTTACCAACAGTGTAAGTCCCTGACTTTTACAATTGTGGTAAAATAGACATAACATAAAATTTCCCTTT
ATAACCATTTTAACTGT / Celera SNP ID: hCV25990131 / Public SNP ID: rs13146272 /
SNP Chromosome Position: 187120211 / SNP in Genomic Sequence: SEQ ID NO: 395 /
SNP Position Genomic: 17537 / SNP Source: Applera /
Population(Allele,Count): Caucasian (A,22|C,16) African American (A,7|C,11)
total (A,29|C,27) / SNP Type: MISSENSE MUTATION;UTR5;ESE SYNONYMOUS;PSEUDOGENE
/ SNP Source: dbSNP; / Population(Allele,Count): Caucasian (C,88|A,130) /
SNP Type: MISSENSE MUTATION;UTR5;ESE SYNONYMOUS;PSEUDOGENE /
Context (SEQ ID NO: 2013): /
CTTAGAAAAATAAATGAAAGAAACTAGCATATTTTATAAGAAAATGTGTTAACTAGGGTGCATCCAAGTCCAAACAGAAGCAT
GTGATTATCATTCAAAT
Y
ATACAGGTCATCGCTGAACGGGCCAATGAAATGAACGCCAATGAAGACTGTAGAGGTGATGGCAGGGGCTCTGCCCCCTCCAA
AAATAAACGCAGGGCCT / Celera SNP ID: hCV3230096 / Public SNP ID: rs3817184 /
SNP Chromosome Position: 187122304 / SNP in Genomic Sequence: SEQ ID NO: 395 /
SNP Position Genomic: 19630 / SNP Source: Applera /
Population(Allele,Count): Caucasian (C,28|T,12) African American (C,13|T,3)
total (C,41|T,15) / SNP Type: ACCEPTOR SPLICE SITE;INTRON / SNP Source:
dbSNP; Celera; HGBASE; / Population(Allele,Count): Caucasian (C,140|T,86) / SNP
Type: ACCEPTOR SPLICE SITE;INTRON /
Context (SEQ ID NO: 2014): /
AGTTATGACAAGAATAATCATTATAGTACTTTTCAGATTTTATAACCTGGAGCAGATTATTTTAAGTTGATTAGTAGGTTCTG
TTACAGTTTTTCTTTTG
W
TCGTGCACTTATAGTCTTCATTTAATTCCTCATAGAATCCCAGTCACCTTTATATATCATATTATTGGAAGAGATTCATCTTC
ATAATCTCCAGTTTTTT / Celera SNP ID: hCV3230113 / Public SNP ID: rs1053094 /
SNP Chromosome Position: 187133031 / SNP in Genomic Sequence: SEQ ID NO: 395 /
SNP Position Genomic: 30357 / SNP Source: dbSNP; Celera; HapMap; HGBASE; /
Population(Allele,Count): Caucasian (A,60|T,60) / SNP Type:
MICRORNA;UTR3;INTRON /
Context (SEQ ID NO: 2015): /
AGAAGAATTCTGAAATATACAAGGACAAAGCAGGATGTACGTCTTTAGTGTCTGCCGCTGCAAAATAAACACGAGATAACTAA
CGTGCGTGAATTGAATG
K
TTGCTTCTCACCCATATTTTATAGAAACAGCTATGGGGAAGAATATTGGTGCTCAAAGTAATGATGATTCCGAGTATGTCCGT
GCAGTTTATAGGTAAAT / Celera SNP ID: hCV11786147 / Public SNP ID: rs4862662 /
SNP Chromosome Position: 187118662 / SNP in Genomic Sequence: SEQ ID NO: 395 /
SNP Position Genomic: 15988 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV25474413 / Related Interrogated SNP: hCV3230038 / Related Interrogated SNP:
hCV3230113 / Related Interrogated SNP: hCV8241630 / Related Interrogated SNP:
hCV3230096 / Related Interrogated SNP: hCV25990131 / Related Interrogated SNP:

hCV27474895 / Related Interrogated SNP: hCV27477533 / Related Interrogated SNP: hCV27902808 / SNP Source: Applera / Population(Allele,Count): Caucasian (G,27|T,11) African American (G,23|T,13) total (G,50|T,24) // / SNP Type: TRANSCRIPTION FACTOR BINDING SITE;UTR5;INTRON / SNP Source: dbSNP; Celera; HGBASE; / Population(Allele,Count): Caucasian (G,140|T,86) / SNP Type: TRANSCRIPTION FACTOR BINDING SITE;UTR5;INTRON /
Context (SEQ ID NO: 2016) /
ACGGGTGAATCTGAAGCTTTTTTGGTGAATCCATAGTAAATGAAAGACCCACTAAGACATAATCTAGGGTAGTGAGTCTCAAG
CTTGAAGGAGCTCAGGA
Y

TTATCTGGATCAGCTGGAGTACAAATTCTCTGATCCTCCCCAGCTATAGATGTGCAAGGTGGGATCAGGGAGGGAAACTCAAG
ACTCTTCATCTTTAACA / Celera SNP ID: hCV25988221 / Public SNP ID: rs9995366 /
SNP Chromosome Position: 187131384 / SNP in Genomic Sequence: SEQ ID NO: 395 /
SNP Position Genomic: 28710 / Related Interrogated SNP: hCV15793897 /
Related Interrogated SNP: hCV12066124 / Related Interrogated SNP: hCV27474895
/ Related Interrogated SNP: hCV25474413 / Related Interrogated SNP:
hCV3230113 / Related Interrogated SNP: hCV8241630 / Related Interrogated SNP:
hCV27477533 / Related Interrogated SNP: hCV25990131 / Related Interrogated SNP:
hCV3230038 / SNP Source: Applera / Population(Allele,Count): Caucasian
(C,36|T,0) African American (C,15|T,3) total (C,51|T,3) / SNP Type: INTRON /
SNP Source: dbSNP; HapMap / Population(Allele,Count): Caucasian (T,21|C,205) /
SNP Type: INTRON /
Context (SEQ ID NO: 2017): /
CTCTTTGTGCCTTAGATATCATCTGTGGTGAGTCTCATCGATCTGTTTCTAAATTTATGGCATAAAGAGAAAATGGCCCAAAC
AGGAAATCACACTCCAC
Y

GGGAAGGCAAATGGGGGGACGGGAAAGGGTGACGGGCTCTTCAGCGCGGTTCTACGACCGCACTGGCCTTCTGAGGAGCAGCA
GCCACGCCCAGGGCTGT / Celera SNP ID: hCV25989001 / Public SNP ID: rs62350518 /
SNP Chromosome Position: 187118358 / SNP in Genomic Sequence: SEQ ID NO: 395 /
SNP Position Genomic: 15684 / Related Interrogated SNP: hCV22272267 /
Related Interrogated SNP: hCV32291301 / Related Interrogated SNP: hCV3230113 /
Related Interrogated SNP: hCV25990131 / Related Interrogated SNP: hCV27902808
/ Related Interrogated SNP: hCV15968043 / Related Interrogated SNP:
hCV3230096 / Related Interrogated SNP: hCV11786258 / Related Interrogated SNP:
hCV12066124 / SNP Source: Applera / Population(Allele,Count): Caucasian
(C,29|T,9) African American (C,24|T,0) total (C,53|T,9) / SNP Type:
UTR5;INTRON / SNP Source: dbSNP; / Population(Allele,Count): Caucasian
(C,97|T,23) / SNP Type: UTR5;INTRON /
Context (SEQ ID NO: 2018): /
CCTCTAACAGTAACATATTACAGAATTAGTATATTCATCAAAATGAGAAAACAAACCTTTGTCCAATACTGGTCACAACTTTC
TCATCTTGATTGAATTT
M

AAATTTGATGTTTTTCCCCAGAATTTTTTTCAGCAGATCATTGAGTACACAGAGGAATACCGCCACATGCCGCTGCTGAAGCTC
TGGGTCGGGCCAGTGCC / Celera SNP ID: hCV3230083 / Public SNP ID: rs10013653 /
SNP Chromosome Position: 187115632 / SNP in Genomic Sequence: SEQ ID NO: 395 /
SNP Position Genomic: 12958 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV27477533 / Related Interrogated SNP: hCV3230038 / Related Interrogated SNP:
hCV3230113 / Related Interrogated SNP: hCV8241630 / Related Interrogated SNP:
hCV3230096 / Related Interrogated SNP: hCV27474895 / Related Interrogated SNP:
hCV25474413 / Related Interrogated SNP: hCV25990131 / Related Interrogated
SNP: hCV27902808 / SNP Source: Applera / Population(Allele,Count):
Caucasian (A,12|C,24) African American (A,20|C,6) total (A,32|C,30) / SNP Type:
INTRON / SNP Source: dbSNP; Celera; / Population(Allele,Count): Caucasian
(C,126|A,94) / SNP Type: INTRON /
Context (SEQ ID NO: 2019): /
GAAAGAAACTAGCATATTTTATAAGAAAATGTGTTAACTAGGGTGCATCCAAGTCCAAACAGAAGCATGTGATTATCATTCAA
ATCATACAGGTCATCGC
K

GAACGGGCCAATGAAATGAACGCCAATGAAGACTGTAGAGGTGATGGCAGGGGCTCTGCCCCCTCCAAAAATAAACGCAGGGC
CTTTCTTGACTTGCTTT / Celera SNP ID: hCV3230097 / Public SNP ID: rs3736455 /
SNP Chromosome Position: 187122319 / SNP in Genomic Sequence: SEQ ID NO: 395 /

SNP Position Genomic: 19645 / Related Interrogated SNP: hCV11786258 / Related Interrogated SNP: hCV12066124 / Related Interrogated SNP: hCV15968043 / Related Interrogated SNP: hCV25990131 / Related Interrogated SNP: hCV27477533 / Related Interrogated SNP: hCV27902808 / Related Interrogated SNP: hCV3230096 / Related Interrogated SNP: hCV8241630 / Related Interrogated SNP: hCV3230113 / Related Interrogated SNP: hCV3230038 / Related Interrogated SNP: hCV27474895 / Related Interrogated SNP: hCV25474413 / Related Interrogated SNP: hCV22272267 / Related Interrogated SNP: hCV32291301 / SNP Source: Applera / Population(Allele,Count): Caucasian (G,24|T,16) African American (G,7|T,9) total (G,31|T,25) / SNP Type: UTR5;SILENT RARE CODON;SILENT MUTATION / SNP Source: dbSNP; Celera; HapMap; HGBASE; / Population(Allele,Count): Caucasian (T,81|G,145) / SNP Type: UTR5;SILENT RARE CODON;SILENT MUTATION / Context (SEQ ID NO: 2020): /
TTCTTTGTTGGGTATTTGATGGGTATTTAGCATGCCATGCCTTGATCCACCTGTTCTTTTTAGATGTCTGCACCCCCAGCCCC CACTGCTCTTTCAGGTC
R
TCTTATCTACTTGCTTTCATCAGGGAAGTCTGACCGTCCCGCTACAGTAGAAGACCTGAAGAAACTTCGGTATCTGGAATGTG TTATTAAGGAGACCCTT / Celera SNP ID: hCV3230110 / Public SNP ID: rs2276917 / SNP Chromosome Position: 187129995 / SNP in Genomic Sequence: SEQ ID NO: 395 / SNP Position Genomic: 27321 / Related Interrogated SNP: hCV11786258 / Related Interrogated SNP: hCV12066124 / Related Interrogated SNP: hCV15968043 / Related Interrogated SNP: hCV22272267 / Related Interrogated SNP: hCV25474413 / Related Interrogated SNP: hCV3230113 / Related Interrogated SNP: hCV3230096 / Related Interrogated SNP: hCV27477533 / Related Interrogated SNP: hCV8241630 / Related Interrogated SNP: hCV3230038 / Related Interrogated SNP: hCV32291301 / SNP Source: Applera / Population(Allele,Count): Caucasian (A,12|G,18) African American (A,9|G,3) total (A,21|G,21) / SNP Type: INTRON / SNP Source: dbSNP; Celera; HapMap; ABI_Val; HGBASE; / Population(Allele,Count): Caucasian (A,129|G,97) / SNP Type: INTRON / Context (SEQ ID NO: 2021): /
CCTCCTCCCACATTTTATTATTCATGCCATAGTTTATATCTTTTGTATGTTGTGTATTCATTAGCAAATTGTTGTAGTTATAG TTATTTGTTAATACTTT
K
GTTTTTTAACTTTTATACTAGAGTTAAAAGTGACTGTTGCACAATAACAGTATTGGAGTATTCTGTATTTGACTGTATAAGAA TACCTTTAATAGAGTTT / Celera SNP ID: hCV3229995 / Public SNP ID: rs11132382 / SNP Chromosome Position: 187143802 / SNP in Genomic Sequence: SEQ ID NO: 395 / SNP Position Genomic: 41128 / Related Interrogated SNP: hCV11786258 / Related Interrogated SNP: hCV12066124 / Related Interrogated SNP: hCV15968043 / Related Interrogated SNP: hCV22272267 / Related Interrogated SNP: hCV27477533 / Related Interrogated SNP: hCV27902808 / Related Interrogated SNP: hCV3230096 / Related Interrogated SNP: hCV8241630 / Related Interrogated SNP: hCV3230113 / Related Interrogated SNP: hCV25474413 / Related Interrogated SNP: hCV27474895 / Related Interrogated SNP: hCV3230038 / Related Interrogated SNP: hCV25990131 / Related Interrogated SNP: hCV32291301 / SNP Source: dbSNP; Celera; HapMap / Population(Allele,Count): Caucasian (T,113|G,111) / SNP Type: INTRON / Context (SEQ ID NO: 2022): /
TACCCTAAGTCTACTGATTTAAACGTTAACCACTTCTTAAAAATACCTTGACAGCAATATCCAGATTGGTGTTTGACCACATA ACCTGGCCCCATAACCA
R
ATAGGCACATAAAATCCATTATAGTAGGTAAGACTTCGTCAAAGAGGTCTAGAAAACATTTCTGGGAGAAATTAACGAAGACC CAAATAAATGAAGCGAT / Celera SNP ID: hCV3230066 / Public SNP ID: rs10027488 / SNP Chromosome Position: 187107849 / SNP in Genomic Sequence: SEQ ID NO: 395 / SNP Position Genomic: 5175 / SNP Source: dbSNP; Celera; HapMap; ABI_Val / Population(Allele,Count): Caucasian (A,86|G,140) / SNP Type: INTERGENIC;UNKNOWN / Context (SEQ ID NO: 2023): /
CACAACATAGGAAAACTGGAAACAACTGAAACATCCATTGGCAGTAAAATGAATAATAAATTATAATATATTCCAACAATGGA ATAATAAACAGCTGTGA
R
AAATAAGGGAAAATATTACACATGGCAATATAGTCAATATAGATTGTCATAAATCAGGTGATTGTACACATACATATGATCTG TTTTATTGCTGTTTTCC / Celera SNP ID: hCV3230070 / Public SNP ID: rs7689823 / SNP Chromosome Position: 187109866 / SNP in Genomic Sequence: SEQ ID NO: 395 /

SNP Position Genomic: 7192 / SNP Source: dbSNP; Celera / Population(Allele,Count): Caucasian (A,85|G,139) / SNP Type: INTERGENIC;UNKNOWN /
Context (SEQ ID NO: 2024): /
TGGACATTCTAAGGTGTGACACTGTGTACTCTGCTGTCAGGAGGCTTTCCGGGGAGAAGGGTCTTGCCAAGGATTTCTTGCAC TCCCAGCCACCTGACTG
R
TTTAGTCTGAAATTTCAGTATTCCAGGGTAGTATGAGAAAGGCCCTATCTGGTAACAAAGAAAGCATAAGAAAGATGGCTAAG GGAACAAAGGGACAAGG / Celera SNP ID: hCV3230072 / Public SNP ID: rs1877319 / SNP Chromosome Position: 187110782 / SNP in Genomic Sequence: SEQ ID NO: 395 / SNP Position Genomic: 8108 / SNP Source: dbSNP; Celera; HapMap; ABI_val; HGBASE / Population(Allele,Count): Caucasian (G,86|A,140) / SNP Type: UTR3;PSEUDOGENE /
Context (SEQ ID NO: 2025): /
CGTGCTCTCCGGGGCTCCGGCTTCCTCGGTCTGGTGCCTGGTGCGTATTTTGGAAAATACCTGGCTCATAACCACCTCATTGA GCTCCCAGTGTCCCAGT
R
GGCCAGCGGACACACACGGGTAGCTGACTTCCCTAACGTGTCCCCAAAGCCTCCCTGGATTACAGCGCCCGCTGCTCCATGTG ACCCCGCGGGAGCCAGG / Celera SNP ID: hCV3230079 / Public SNP ID: rs35641294 / SNP Chromosome Position: 187112310 / SNP in Genomic Sequence: SEQ ID NO: 395 / SNP Position Genomic: 9636 / Related Interrogated SNP: hCV3230096 / Related Interrogated SNP: hCV3230113 / SNP Source: dbSNP; Celera; / Population(Allele,Count): Caucasian (A,41|G,79) / SNP Type: MISSENSE MUTATION /
Context (SEQ ID NO: 2026): /
AAAAATATAGATTTTCCCCTATCCCCTACCCCTGGAAAGTAATATACTGAAGTCTCATCATACTGTTTTGGGGATTCCAGTAA TTAAAATCTCTAGTGAA
Y
AAAGACCTGTTTCAAAACAACCTGTGAGCTGACTGGACTATTTAAAGTAATTCTCCTTGTAGTCACTTTCAGAGTGAAGACAA TGACGAATACTGTCTTT / Celera SNP ID: hCV3230081 / Public SNP ID: rs10866290 / SNP Chromosome Position: 187114479 / SNP in Genomic Sequence: SEQ ID NO: 395 / SNP Position Genomic: 11805 / Related Interrogated SNP: hCV3230113 / SNP Source: dbSNP; Celera / Population(Allele,Count): Caucasian (T,153|C,73) / SNP Type: MICRORNA;UTR5;UTR3;TFBS SYNONYMOUS;INTRON /
Context (SEQ ID NO: 2027): /
CCCTTTATAATGCAGAAAATGTGGAGGTGGGTACATGTGAATATGATCAGTATTGTACTGTGTATCTGACAGTGTGAGAATCT CACCAGAATCAATATAA
Y
GTGTCCTTATATTTCAGCCATTTCAGCACAAAAAACATTCCACTAAGTATTTTAGACTATATAGGTGCAATTTAATGCTTTCC AGGTATAGCCCGAATCC / Celera SNP ID: hCV3230084 / Public SNP ID: rs7682918 / SNP Chromosome Position: 187115841 / SNP in Genomic Sequence: SEQ ID NO: 395 / SNP Position Genomic: 13167 / Related Interrogated SNP: hCV11786258 / Related Interrogated SNP: hCV12066124 / Related Interrogated SNP: hCV15968043 / Related Interrogated SNP: hCV22272267 / Related Interrogated SNP: hCV25474413 / Related Interrogated SNP: hCV3230038 / Related Interrogated SNP: hCV3230113 / Related Interrogated SNP: hCV8241630 / Related Interrogated SNP: hCV3230096 / Related Interrogated SNP: hCV25990131 / Related Interrogated SNP: hCV27474895 / Related Interrogated SNP: hCV27477533 / Related Interrogated SNP: hCV27902808 / SNP Source: dbSNP; Celera; / Population(Allele,Count): Caucasian (C,139|T,87) / SNP Type: INTRON /
Context (SEQ ID NO: 2028): /
AATGATTGCATTCACTCTACCACTTAAACTTTTTCTCATTAGCGCAATCCAATCTCTAGGGTACTATCTAGTAGACTATGATT CTATTCTTGTGTTGGAA
R
GACTGCAAAAATAGCACTTGAAAATTAGGAATTTGCGTGACATTAAATTTGTTTTTAAAAGTTTCACCAGATAATCCCCAAAA TATTAATGAGGCTTTAC / Celera SNP ID: hCV3230094 / Public SNP ID: rs7687818 / SNP Chromosome Position: 187122060 / SNP in Genomic Sequence: SEQ ID NO: 395 / SNP Position Genomic: 19386 / Related Interrogated SNP: hCV11786258 / Related Interrogated SNP: hCV12066124 / Related Interrogated SNP: hCV15968043 / Related Interrogated SNP: hCV22272267 / Related Interrogated SNP: hCV27477533 / Related Interrogated SNP: hCV3230038 / Related Interrogated SNP: hCV3230113 / Related Interrogated SNP: hCV8241630 / Related Interrogated SNP: hCV3230096 / Related Interrogated SNP: hCV27474895 / Related Interrogated SNP:

hCV25474413 / Related Interrogated SNP: hCV25990131 / Related Interrogated SNP: hCV27902808 / SNP Source: dbSNP; Celera; HapMap / Population(Allele,Count): Caucasian (A,131|G,95) / SNP Type: INTRON / Context (SEQ ID NO: 2029): /
TAAGTGTGTGGGCTCTAAGATCAAATCCCAGCTAACATGCTGGGACCATGACCTCAAGCACCTCACCTAGCCGTTCTGGGCCT GAATATTCTTATTTTAA
K

AGCACAAGCCTCATGGAATAGTTATGATGATTAGATTTGTAAAGGTGCTCTGAGAATCGTGATTGGCCCATAAGTATGCGGCA CATACCGGGTGTTATTA / Celera SNP ID: hCV3230101 / Public SNP ID: rs6835839 / SNP Chromosome Position: 187123582 / SNP in Genomic Sequence: SEQ ID NO: 395 / SNP Position Genomic: 20908 / Related Interrogated SNP: hCV11786258 / Related Interrogated SNP: hCV15968043 / Related Interrogated SNP: hCV22272267 / Related Interrogated SNP: hCV3230113 / Related Interrogated SNP: hCV3230096 / Related Interrogated SNP: hCV12066124 / Related Interrogated SNP: hCV27477533 / Related Interrogated SNP: hCV8241630 / Related Interrogated SNP: hCV25474413 / Related Interrogated SNP: hCV32291301 / Related Interrogated SNP: hCV3230038 / SNP Source: dbSNP; Celera / Population(Allele,Count): Caucasian (G,48|T,72) / SNP Type: UTR5;INTRON / Context (SEQ ID NO: 2030): /
GCAGGGAAAGAAAACTAAAGGAATAAAGCTGGAGAGAGAAGGCATTCTAGAAAGAGAGGAAGCAGATGAGCAACGGCACAGAA GTGGAAAGGTTTCTGGC
R

TGGAGGGCAATAGTGGAGAGTGAGAGACCGCGAGAACTCATCTCTCATTCACTGGGAGATGAGGATGGAAAAGCAGTTTTGTG TGCAGATTGTGCTGGGA / Celera SNP ID: hCV3230106 / Public SNP ID: rs1473597 / SNP Chromosome Position: 187127817 / SNP in Genomic Sequence: SEQ ID NO: 395 / SNP Position Genomic: 25143 / Related Interrogated SNP: hCV11786258 / Related Interrogated SNP: hCV12066124 / Related Interrogated SNP: hCV15968043 / Related Interrogated SNP: hCV3230096 / Related Interrogated SNP: hCV22272267 / Related Interrogated SNP: hCV3230113 / Related Interrogated SNP: hCV25474413 / Related Interrogated SNP: hCV27477533 / Related Interrogated SNP: hCV8241630 / Related Interrogated SNP: hCV3230038 / Related Interrogated SNP: hCV32291301 / SNP Source: dbSNP; Celera; HapMap; ABI_Val; HGBASE / Population(Allele,Count): Caucasian (A,126|G,96) / SNP Type: INTRON / Context (SEQ ID NO: 2031): /
CCACCCTACTTGGGAACCCAAGAGCCAGTATATTGCCGTTTGCTTAAAAGGACTGTCTTTCATTTATGACCAAAAGCGAGTTA GTGTGTGTCAGGTCAGC
Y

GAAGGATGAAGGAAGGGCCCCCATTCTCTGGATATGATCTTAGGTCTTCACCCTATTTTCTTTGTGCTGTTCAATAATTCTCT GTATTGGATCATCAGCA / Celera SNP ID: hCV8241633 / Public SNP ID: rs1511800 / SNP Chromosome Position: 187135556 / SNP in Genomic Sequence: SEQ ID NO: 395 / SNP Position Genomic: 32882 / Related Interrogated SNP: hCV15793897 / Related Interrogated SNP: hCV12066124 / Related Interrogated SNP: hCV27474895 / Related Interrogated SNP: hCV25474413 / Related Interrogated SNP: hCV3230113 / Related Interrogated SNP: hCV8241630 / Related Interrogated SNP: hCV27477533 / Related Interrogated SNP: hCV25990131 / Related Interrogated SNP: hCV3230038 / SNP Source: dbSNP; HGBASE / Population(Allele,Count): Caucasian (C,21|T,205) / SNP Type: NONSENSE MUTATION;TRANSCRIPTION FACTOR BINDING SITE;INTRON / Context (SEQ ID NO: 2032): /
GAATGCACATCACCACCCTGCATTATAAACCCTTTTGGTTTTAGTATCTCTAGCATTTTGTCATTTATGTAAATGCAGACATC GGTAAAATGAGGCCAGA
R

TGAAACGGGTGAATCTGAAGCTTTTTTGGTGAATCCATAGTAAATGAAAGACCCACTAAGACATAATCTAGGGTAGTGAGTCT CAAGCTTGAAGGAGCTC / Celera SNP ID: hCV12066116 / Public SNP ID: rs1877320 / SNP Chromosome Position: 187131279 / SNP in Genomic Sequence: SEQ ID NO: 395 / SNP Position Genomic: 28605 / Related Interrogated SNP: hCV12066124 / Related Interrogated SNP: hCV15793897 / Related Interrogated SNP: hCV25474413 / Related Interrogated SNP: hCV27477533 / Related Interrogated SNP: hCV3230038 / Related Interrogated SNP: hCV8241630 / Related Interrogated SNP: hCV25990131 / Related Interrogated SNP: hCV3230113 / Related Interrogated SNP: hCV27474895 / SNP Source: dbSNP; HapMap; HGBASE / Population(Allele,Count): Caucasian (G,12|A,106) / SNP Type: INTRON / Context (SEQ ID NO: 2033): /

TACAAATTCTCTGATCCTCCCCAGCTATAGATGTGCAAGGTGGGATCAGGGAGGGAAACTCAAGACTCTTCATCTTTAACAGG
TGTTCCAAGCCATTCTG
R
TTCTCCTTCCACCTACTGCGAAAATGTAAAGTGGCTCGCTTCCTATGACAGGACCTCTTGTTTCTCACATTTGGGGTAAATCT
ATAACTAAAGCGATGGT / Celera SNP ID: hCV15811716 / Public SNP ID: rs2102575 /
SNP Chromosome Position: 187131504 / SNP in Genomic Sequence: SEQ ID NO: 395 /
SNP Position Genomic: 28830 / Related Interrogated SNP: hCV15793897 /
Related Interrogated SNP: hCV12066124 / Related Interrogated SNP: hCV25474413
/ Related Interrogated SNP: hCV27474895 / Related Interrogated SNP:
hCV22272267 / Related Interrogated SNP: hCV25990131 / Related Interrogated SNP:
hCV27477533 / Related Interrogated SNP: hCV3230038 / Related Interrogated
SNP: hCV3230113 / Related Interrogated SNP: hCV8241630 / SNP Source: dbSNP;
HapMap; HGBASE; / Population(Allele,Count): Caucasian (G,19|A,207) / SNP Type:
INTRON /
Context (SEQ ID NO: 2034): /
TAATGAGTAGGACATGTGTATATGGTCCGTACCTGAAAGGAAGTTATTCTAGTAGGAGAGGTGATCTATCAACACATAATTAC
AACATGTGATATGAGCT
R
TGAACACTTATGAACAAACAGGGTGCTGTGTAAAAGAATAAAGGAACAAAGATCTATGTATAGGAGTTTTCTGGAAAATGTTT
GGATTCGGCAGTCATTT / Celera SNP ID: hCV15968025 / Public SNP ID: rs2292425 /
SNP Chromosome Position: 187120697 / SNP in Genomic Sequence: SEQ ID NO: 395 /
SNP Position Genomic: 18023 / Related Interrogated SNP: hCV11786258 /
Related Interrogated SNP: hCV12066124 / Related Interrogated SNP: hCV25474413
/ Related Interrogated SNP: hCV25990131 / Related Interrogated SNP:
hCV27477533 / Related Interrogated SNP: hCV27474895 / Related Interrogated SNP:
hCV3230096 / Related Interrogated SNP: hCV8241630 / Related Interrogated SNP:
hCV15968043 / Related Interrogated SNP: hCV27902808 / Related Interrogated
SNP: hCV3230038 / Related Interrogated SNP: hCV22272267 / Related
Interrogated SNP: hCV32291301 / Related Interrogated SNP: hCV3230113 / SNP
Source: dbSNP; HGBASE / Population(Allele,Count): Caucasian (G,89|A,137) /
SNP Type: INTRON;PSEUDOGENE /
Context (SEQ ID NO: 2035): /
AGAGGTGATCTATCAACACATAATTACAACATGTGATATGAGCTGTGAACACTTATGAACAAACAGGGTGCTGTGTAAAAGAA
TAAAGGAACAAAGATCT
R
TGTATAGGAGTTTTCTGGAAAATGTTTGGATTCGGCAGTCATTTTCAAAGGCAGAGGGCATTGATAGCAGTATCTTAACATGG
AAAACATTAAAACTAAC / Celera SNP ID: hCV15968026 / Public SNP ID: rs2292426 /
SNP Chromosome Position: 187120753 / SNP in Genomic Sequence: SEQ ID NO: 395 /
SNP Position Genomic: 18079 / Related Interrogated SNP: hCV11786258 /
Related Interrogated SNP: hCV12066124 / Related Interrogated SNP: hCV15968043
/ Related Interrogated SNP: hCV25474413 / Related Interrogated SNP:
hCV27474895 / Related Interrogated SNP: hCV27477533 / Related Interrogated SNP:
hCV3230038 / Related Interrogated SNP: hCV3230096 / Related Interrogated SNP:
hCV8241630 / Related Interrogated SNP: hCV25990131 / Related Interrogated
SNP: hCV22272267 / Related Interrogated SNP: hCV27902808 / Related
Interrogated SNP: hCV3230113 / Related Interrogated SNP: hCV32291301 /
Related Interrogated SNP: hCV15793897 / SNP Source: dbSNP; HGBASE /
Population(Allele,Count): Caucasian (A,42|G,78) / SNP Type: INTRON;PSEUDOGENE
/
Context (SEQ ID NO: 2036): /
GAAAATATACATCAAATTATAATTTCAGTGTATTAAAAAAACTGCCTGTTTAAATATGTCCTTTCTTTGCTGTAAATTTTGGTT
AAAATCTATTGGAGTTA
Y
GTCCTTGTGGTGAAGTACACCCTACCCCCAAGAGAGCAAATGATGAATAAATCAGTAGATGTTCCATGAATGCAATGTTGGCT
GAGCTGGCCACAGTGGA / Celera SNP ID: hCV15968034 / Public SNP ID: rs2292428 /
SNP Chromosome Position: 187121070 / SNP in Genomic Sequence: SEQ ID NO: 395 /
SNP Position Genomic: 18396 / Related Interrogated SNP: hCV11786258 /
Related Interrogated SNP: hCV12066124 / Related Interrogated SNP: hCV15968043
/ Related Interrogated SNP: hCV22272267 / Related Interrogated SNP:
hCV25474413 / Related Interrogated SNP: hCV3230113 / Related Interrogated SNP:
hCV3230096 / Related Interrogated SNP: hCV27477533 / Related Interrogated SNP:
hCV8241630 / Related Interrogated SNP: hCV3230038 / Related Interrogated SNP:
hCV32291301 / SNP Source: dbSNP; HGBASE / Population(Allele,Count):

Caucasian (T,128|C,98) / SNP Type: INTRON;PSEUDOGENE / Context (SEQ ID NO: 2037): / TTGGGAGATGATTGGATCACGAGGTGTTTTCCCCCATTATGTTCTCATGATAGTGAGGGTGTTCTCATGAGATCTGATGGTTT AAAAGTGGTGGTTACCC

S TGCGCACATGCTCTCTCTCTCCTGCTGCCTTGTGAAGAAGGTACCTGCTTCCCCTTCACCTTCCACCATAATTGTAAGTTT CCTGAGGCCTCCCCAGC / Celera SNP ID: hCV26265199 / Public SNP ID: rs2221843 / SNP Chromosome Position: 187144200 / SNP in Genomic Sequence: SEQ ID NO: 395 / SNP Position Genomic: 41526 / Related Interrogated SNP: hCV22272267 / Related Interrogated SNP: hCV15793897 / Related Interrogated SNP: hCV27902808 / Related Interrogated SNP: hCV15968043 / Related Interrogated SNP: hCV11786258 / Related Interrogated SNP: hCV3230113 / SNP Source: dbSNP; HapMap; HGBASE / Population(Allele,Count): Caucasian (G,39|C,187) / SNP Type: INTRON / Context (SEQ ID NO: 2038): / CTCAAGTTGCTCAGGGTCTCATATCAATGATAAACTTAGTCCAAATTTAAAAGACAATGCAAATTTGATATTTATGGGATAAA TATTACAAACCCATGCT

R TTGAAATTAGTCCTCCAAAAAGCTGTAGTCTTTTTACTTGTTGAAAGGTCTGGGGAAATCATCTTGTAGTATGGTGAGAAAAG GATTTGGGTCAGGAAAC / Celera SNP ID: hCV26265231 / Public SNP ID: rs7684025 / SNP Chromosome Position: 187116227 / SNP in Genomic Sequence: SEQ ID NO: 395 / SNP Position Genomic: 13553 / Related Interrogated SNP: hCV11786258 / Related Interrogated SNP: hCV12066124 / Related Interrogated SNP: hCV15968043 / Related Interrogated SNP: hCV22272267 / Related Interrogated SNP: hCV27477533 / Related Interrogated SNP: hCV3230038 / Related Interrogated SNP: hCV3230113 / Related Interrogated SNP: hCV8241630 / Related Interrogated SNP: hCV3230096 / Related Interrogated SNP: hCV27474895 / Related Interrogated SNP: hCV25474413 / Related Interrogated SNP: hCV25990131 / Related Interrogated SNP: hCV27902808 / SNP Source: dbSNP; Celera; HapMap; ABI_Val / Population(Allele,Count): Caucasian (G,127|A,99) / SNP Type: INTRON / Context (SEQ ID NO: 2039): / CTCTTTATCCTAGTAGATAAGTTTTTGGCACGTTCTTTCTCCTTCCCATCATTATCTCTCCTCTTTACCCGTCACTTTCTTTC TGTTGTGGTTTGCATTT

R TTGATCTCTGCCTTTCCTGATACTACTCAATTTATGTTTCACAGAAATAACGGCTGTACTTAAATGAAACTCAGATTCCTATA TAAAAATGTTGGGATAA / Celera SNP ID: hCV27902803 / Public SNP ID: rs4862665 / SNP Chromosome Position: 187136142 / SNP in Genomic Sequence: SEQ ID NO: 395 / SNP Position Genomic: 33468 / Related Interrogated SNP: hCV15793897 / Related Interrogated SNP: hCV12066124 / Related Interrogated SNP: hCV27474895 / Related Interrogated SNP: hCV25474413 / Related Interrogated SNP: hCV3230113 / Related Interrogated SNP: hCV8241630 / Related Interrogated SNP: hCV27477533 / Related Interrogated SNP: hCV25990131 / Related Interrogated SNP: hCV3230038 / SNP Source: dbSNP; HGBASE / Population(Allele,Count): Caucasian (A,21|G,205) / SNP Type: INTRON / Context (SEQ ID NO: 2040): / AATGTGACTCCTCTTTCTCGGACTTCCCAGCCTCCAGAACCGTACCAGTCTGTGATATTTGGTTATAGGAGCACAAAGTGAAC TATGACAACATCATTAA

S AGGGTGATAAGGCAAACTACAGAGAGGAAGAAACTTGGCAATACATAGCTGGCAAAGGATTTGTATTCATATATAATTTATTA TGTAATAATTGCTCATT / Celera SNP ID: hCV27985656 / Public SNP ID: rs4586997 / SNP Chromosome Position: 187108980 / SNP in Genomic Sequence: SEQ ID NO: 395 / SNP Position Genomic: 6306 / SNP Source: dbSNP; HGBASE / Population(Allele,Count): Caucasian (G,86|C,140) / SNP Type: INTERGENIC;UNKNOWN / Context (SEQ ID NO: 2041): / CAGAGGAAAACTCTGCTTTTAATGGGCTCATGTAATAAGATTTGGCCCACTCTGATTGACTCCATTTTGATTGGCACAAAGCC AAGTGTTTAGTAATCTT

R ACCCCATCTGCAGAATTGCTTTTCTCATGTAACATAATCATGGGCAGATGTGATATCTCGTTTTATTCACTGTCCCCAGGATT TGAGTGGGAGATTTGGG / Celera SNP ID: hCV29053260 / Public SNP ID: rs4861707 / SNP Chromosome Position: 187138092 / SNP in Genomic Sequence: SEQ ID NO: 395 / SNP Position Genomic: 35418 / Related Interrogated SNP: hCV3230096 / Related Interrogated SNP: hCV11786258 / Related Interrogated SNP: hCV15968043 /

Related Interrogated SNP: hCV27902808 / Related Interrogated SNP: hCV3230113 / SNP Source: dbSNP; HGBASE / Population(Allele,Count): Caucasian (A,82|G,38) / SNP Type: INTRON /
Context (SEQ ID NO: 2042): /
ATTTAGTAACCCAGTAATATCTCACTTAGTTTAGGGTTAGATCTTTAGTTAATTCAACCTTATAGATCATACTTATGAAGGTG ATAACTGACACGTGTTC
M
CTGAATTTTTAATTTGATAGGCAATACATCTACCCACTCCATTATTTTTTTAAAAACTTCATTTAATAGTTTAAACAAGATTGGTT TTGTTTTTCAATTTTTAT / Celera SNP ID: hCV29053261 / Public SNP ID: rs6842047 /
SNP Chromosome Position: 187133576 / SNP in Genomic Sequence: SEQ ID NO: 395 / SNP Position Genomic: 30902 / Related Interrogated SNP: hCV15793897 /
Related Interrogated SNP: hCV12066124 / Related Interrogated SNP: hCV25474413 / Related Interrogated SNP: hCV27474895 / Related Interrogated SNP:
hCV3230113 / Related Interrogated SNP: hCV8241630 / Related Interrogated SNP: hCV25990131 / Related Interrogated SNP: hCV27477533 / Related Interrogated SNP:
hCV3230038 / SNP Source: dbSNP; HapMap; / Population(Allele,Count): Caucasian (A,20|C,206) / SNP Type: MICRORNA;UTR3;INTRON /
Context (SEQ ID NO: 2043): /
CCACCTGACAGATGGGGGAAGGCAGAGAAGTACAGCCCTGACAAGCAGGCTCGTGAAAATCAGACTTTGTTCTGTTCACAAAG GCGCAGCTCACCTCGTG
S
CCATGGAACGCCGCCTTTATTAACACAGCACAGGCACGTCCCACCAGCGTGCAACACCGTGCGGCGTGGAACTGCCATCTCTA GGCATGAACCTCCGCTT / Celera SNP ID: hCV29053264 / Public SNP ID: rs7667777 /
SNP Chromosome Position: 187126077 / SNP in Genomic Sequence: SEQ ID NO: 395 / SNP Position Genomic: 23403 / Related Interrogated SNP: hCV11786258 /
Related Interrogated SNP: hCV12066124 / Related Interrogated SNP: hCV15968043 / Related Interrogated SNP: hCV22272267 / Related Interrogated SNP:
hCV25474413 / Related Interrogated SNP: hCV3230038 / Related Interrogated SNP: hCV3230113 / Related Interrogated SNP: hCV8241630 / Related Interrogated SNP:
hCV3230096 / Related Interrogated SNP: hCV25990131 / Related Interrogated SNP: hCV27474895 / Related Interrogated SNP: hCV27477533 / Related Interrogated
SNP: hCV27902808 / SNP Source: dbSNP / Population(Allele,Count): Caucasian (C,71|G,49) / SNP Type: UTR5;INTRON /
Context (SEQ ID NO: 2044): /
TTCTTTTCTCCTCTACAACATGTAATTAAGTCTATAATTAGACTGTTGTATAATGATTGCATTCACTCTACCACTTAAACTTT TTCTCATTAGCGCAATC
M
AATCTCTAGGGTACTATCTAGTAGACTATGATTCTATTCTTGTGTTGGAAAGACTGCAAAAATAGCACTTGAAAATTAGGAAT TTGCGTGACATTAAATT / Celera SNP ID: hCV29053266 / Public SNP ID: rs7687961 /
SNP Chromosome Position: 187122009 / SNP in Genomic Sequence: SEQ ID NO: 395 / SNP Position Genomic: 19335 / Related Interrogated SNP: hCV15793897 /
Related Interrogated SNP: hCV25990131 / Related Interrogated SNP: hCV27474895 / Related Interrogated SNP: hCV3230096 / SNP Source: dbSNP /
Population(Allele,Count): Caucasian (C,42|A,184) / SNP Type: INTRON /
Context (SEQ ID NO: 2045): /
CTCCCAGTGTCCCAGTGGGCCAGCGGACACACACGGGTAGCTGACTTCCCTAACGTGTCCCCAAAGCCTCCCTGGATTACAGC GCCCGCTGCTCCATGTG
W
CCCCGCGGGAGCCAGGACGCGCCCCGCCTCCCGGGGCTGCAAGTTGCGCAGGGAGCGAGCGATGCGCAGCAGAGCTGGGCTCC GAGCCGGATGCGCCTTC / Celera SNP ID: hCV29053271 / Public SNP ID: rs6814261 /
SNP Chromosome Position: 187112394 / SNP in Genomic Sequence: SEQ ID NO: 395 / SNP Position Genomic: 9720 / Related Interrogated SNP: hCV3230096 / Related
Interrogated SNP: hCV3230113 / SNP Source: dbSNP; / Population(Allele,Count): Caucasian (A,77|T,39) / SNP Type: MISSENSE MUTATION;TFBS SYNONYMOUS /
Context (SEQ ID NO: 2046): /
CTTCAGGCTTCCTTCTAACCTCCCAGACATGCAAGTCCACTTCCTCACCACCTGCTTTCTGAGGTTCCAGCAGATGAGCACTT AGCTGCTGCCATGAGCT
R
CTCTACCTAGAGGACAGTTAAGAGCCCTCCAACAGAGAATCAGCCAGTTCATGATTCTAAAAAGATCTCTGATCTCAAATTTA TACATACATATGTGAAG / Celera SNP ID: hCV30492573 / Public SNP ID: rs10471184 /
SNP Chromosome Position: 187122904 / SNP in Genomic Sequence: SEQ ID NO: 395 / SNP Position Genomic: 20230 / Related Interrogated SNP: hCV15793897 /
Related Interrogated SNP: hCV12066124 / Related Interrogated SNP: hCV25474413

/ Related Interrogated SNP: hCV27474895 / Related Interrogated SNP: hCV3230113 / Related Interrogated SNP: hCV8241630 / Related Interrogated SNP: hCV25990131 / Related Interrogated SNP: hCV27477533 / Related Interrogated SNP: hCV3230038 / SNP Source: dbSNP / Population(Allele,Count): Caucasian (A,20|G,206) / SNP Type: UTR5;INTRON / Context (SEQ ID NO: 2047): / TGCATGTCGGAGTAGAGAGATCTCACATCTTCACCTTGCCCATGTACTACACACACACGCATACACTCATGTAAACCCATG CACACACATGCACACAC Y

TACACACACACATATGCTCATGCACATACACATGCACAGACACATGTATATACCAACATACACACATTCACATATACACACAT ACACAAACACATGCACA / Celera SNP ID: hCV30983927 / Public SNP ID: rs6552962 / SNP Chromosome Position: 187123784 / SNP in Genomic Sequence: SEQ ID NO: 395 / SNP Position Genomic: 21110 / Related Interrogated SNP: hCV15793897 / Related Interrogated SNP: hCV25990131 / Related Interrogated SNP: hCV27474895 / Related Interrogated SNP: hCV27477533 / Related Interrogated SNP: hCV3230038 / Related Interrogated SNP: hCV8241630 / Related Interrogated SNP: hCV3230096 / Related Interrogated SNP: hCV11786258 / Related Interrogated SNP: hCV25474413 / Related Interrogated SNP: hCV12066124 / SNP Source: dbSNP / Population(Allele,Count): Caucasian (C,97|T,23) / SNP Type: UTR5;INTRON / Context (SEQ ID NO: 2048): / CTCATTAATTATTTATAATTCCTACAAATCACTAAAAATCCAGACAACTACAACGTTCAACACGGAAAATGATTTAATAGGTG CTCTCTGAAATAGCCAA Y

AATCTTCTGAAATGATGCTGACTATCCTAGGTCACTAGGAAAATTCATACTAAACTTTCAACGCTCAGCCTAGGCAACATGGC GAAACCCTGTCTCTACT / Celera SNP ID: hCV29934557 / Public SNP ID: rs9991842 / SNP Chromosome Position: 187109175 / SNP in Genomic Sequence: SEQ ID NO: 395 / SNP Position Genomic: 6501 / SNP Source: dbSNP; HapMap / Population(Allele,Count): Caucasian (C,86|T,140) / SNP Type: INTERGENIC;UNKNOWN / Context (SEQ ID NO: 2049): / TCAGTTTACTTTCAGATTCCATATATAGTTTTAAAATACATGTTTTAGCTGAATAGTAAAAAAAAAAAAAAAATATGTAGGCAC AGGAGTATCTATTGGGC W

CTCCTGGGAAGGAGAGTAGCCTTTCTTTCTCTTTCTTTCTCTTTCTTTCTCTTTCTTTCCTTCTTTCTTTCCTTCCTTCCTTC CTTCCTTCTTTCCTTGC / Celera SNP ID: hCV30983902 / Public SNP ID: rs4862668 / SNP Chromosome Position: 187136443 / SNP in Genomic Sequence: SEQ ID NO: 395 / SNP Position Genomic: 33769 / Related Interrogated SNP: hCV12066124 / Related Interrogated SNP: hCV15793897 / Related Interrogated SNP: hCV25474413 / Related Interrogated SNP: hCV27477533 / Related Interrogated SNP: hCV3230038 / Related Interrogated SNP: hCV8241630 / Related Interrogated SNP: hCV25990131 / Related Interrogated SNP: hCV3230113 / Related Interrogated SNP: hCV27474895 / SNP Source: dbSNP; HGBASE / Population(Allele,Count): Caucasian (A,12|T,108) / SNP Type: INTRON / Context (SEQ ID NO: 2050): / TGAAACTCAGATTCCTATATAAAAATGTTGGGATAACTTTGTTCCTTCAGTAAGGGTCATACAGACATTATAGATCTTATCGG GAAGAAAAATTATCACA R

TATTTAGACATGACTGTCTTATTTTAAAAATGTTTTCAGTTTACTTTCAGATTCCATATATAGTTTTAAAATACATGTTTTTAG CTGAATAGTAAAAAAAA / Celera SNP ID: hCV32313007 / Public SNP ID: rs4862666 / SNP Chromosome Position: 187136307 / SNP in Genomic Sequence: SEQ ID NO: 395 / SNP Position Genomic: 33633 / Related Interrogated SNP: hCV15793897 / Related Interrogated SNP: hCV12066124 / Related Interrogated SNP: hCV27474895 / Related Interrogated SNP: hCV25474413 / Related Interrogated SNP: hCV3230113 / Related Interrogated SNP: hCV8241630 / Related Interrogated SNP: hCV27477533 / Related Interrogated SNP: hCV25990131 / Related Interrogated SNP: hCV3230038 / SNP Source: dbSNP; HGBASE / Population(Allele,Count): Caucasian (A,21|G,205) / SNP Type: INTRON / Context (SEQ ID NO: 2051): / AAAGTGCTTAGTATTTTGCCTAAAAACTCAGCAAGTATGAAATGAGGTTGTTGTCAATTTTTACTGCAGAGGATATGACTTAGA ACCACACCTCTACCCCC K

GGTGCCCGTCATCAACAGGTGAGGCCCACCTGACAGATGGGGGAAGGCAGAGAAGTACAGCCCTGACAAGCAGGCTCGTGAAA ATCAGACTTTGTTCTGT / Celera SNP ID: hDV76175111 / Public SNP ID: rs35079309 /

SNP Chromosome Position: 187125951 / SNP in Genomic Sequence: SEQ ID NO: 395 / SNP Position Genomic: 23277 / Related Interrogated SNP: hCV3230113 / Related Interrogated SNP: hCV15968043 / Related Interrogated SNP: hCV3230096 / Related Interrogated SNP: hCV11786258 / Related Interrogated SNP: hCV25990131 / SNP Source: CDX; dbSNP / Population(Allele,Count): Caucasian (G,26|T,94) / SNP Type: UTR5 INDEL;UTR5;INTRONIC INDEL;INTRON //

Gene Number: 59 / Gene Symbol: CYP27C1 - 339761 / Gene Name: cytochrome P450, family 27, subfamily C, polypeptide 1 / Chromosome: 2 / OMIM NUMBER: / OMIM Information: // Genomic Sequence (SEQ ID NO: 396): // / SNP Information / Context (SEQ ID NO: 2052): /
GGACATCAGCTGCAGTTGGAACATGCCTTTCCCCTAAATTCTTCACAATCTCCCGGTACACCGTCTGCTGCACTTCTGGGTGC
CTTGCCAGGAGGTACAC
M
GTCCAAGACAAGGTGAAGGACGTCTAAGGAGAGAAAGTGGCAGACACAGGCAGGCAGTGAGTAACACCAGGGACTGAAACAGA
GGCGGTGGCAGGTAGGA / Celera SNP ID: hCV1441133 / Public SNP ID: rs7568070 /
SNP Chromosome Position: 127953054 / SNP in Genomic Sequence: SEQ ID NO: 396 / SNP Position Genomic: 21642 / Related Interrogated SNP: hCV1841975 / Related Interrogated SNP: hCV1841983 / SNP Source: Applera / Population(Allele,Count): Caucasian (A,21|C,9) African American (A,16|C,18) total (A,37|C,27) / SNP Type: ESE SYNONYMOUS;SILENT RARE CODON;SILENT MUTATION / SNP Source: dbSNP; Celera; HapMap; ABI_Val / Population(Allele,Count): Caucasian (A,174|C,52) / SNP Type: ESE SYNONYMOUS;SILENT RARE CODON;SILENT MUTATION /
Context (SEQ ID NO: 2053): /
CTGCCTGGGTCCCTCCCCGGGACCAGTCCCTGGAGACCCTGCCCGCCGCCTCTCTCGGGCTACCACTCTCCGAAACTCCATCC
CTGACTCCCCTCCCCGC
Y
ACCTCCTCCCCAGGGGTCCCGGCTGGGGCCCCTCCAGGGGACCCCTCCCTGCCGCCACCTCCCGAGACACCCATTTCCCCCGC
CTCTTCCCAGCCGTTCC / Celera SNP ID: hCV8807983 / Public SNP ID: rs1504136 /
SNP Chromosome Position: 127977118 / SNP in Genomic Sequence: SEQ ID NO: 396 / SNP Position Genomic: 45706 / Related Interrogated SNP: hCV1841975 / Related Interrogated SNP: hCV1841983 / SNP Source: dbSNP; HapMap; ABI_Val; HGBASE / Population(Allele,Count): Caucasian (T,71|C,155) / SNP Type: INTRON /
Context (SEQ ID NO: 2054): /
AGAACAAATCTGCATCATTAAGTGAGGCGTGACTGCATACACAGAGTTTTCTCCTCCTGGAGCCCCCCAGCAGTGCAGCAGGC
CTAGTACAAACATGAAC
R
GTGTGCTAAACAAATCTGCCTTAAAAGTAGACAACGGCCAGTCGCAGTGGCTCATGCCTGTAATCCCAGCACTTTGGGAGGCC
AAGGCGGGTGGATCACC / Celera SNP ID: hCV9465822 / Public SNP ID: rs11683427 /
SNP Chromosome Position: 127956543 / SNP in Genomic Sequence: SEQ ID NO: 396 / SNP Position Genomic: 25131 / Related Interrogated SNP: hCV1841975 / Related Interrogated SNP: hCV1841983 / SNP Source: dbSNP; Celera; HapMap /
Population(Allele,Count): Caucasian (A,169|G,55) / SNP Type: INTRON /
Context (SEQ ID NO: 2055): /
AAAGGGAAAAGGGGCAGAATCACTCAAGAGCAGGGCAGGGCCCAGAGCCCTAGCCGGACAGTCTCCCGGGGTGCTCCCGCCTG
GGGGCCGCCCGGGTCCA
Y
TCACACCCAGGATGGCAGCCGCTCCCCCCTTCCCACTCCACACTGCACCCCAAATGTCACTGCAGCCACTGAGAAGACCGTCG
AGCCCTTCAGAGACTCC / Celera SNP ID: hCV27907596 / Public SNP ID: rs4321325 /
SNP Chromosome Position: 127950997 / SNP in Genomic Sequence: SEQ ID NO: 396 / SNP Position Genomic: 19585 / Related Interrogated SNP: hCV1841975 / SNP Source: dbSNP; HapMap; HGBASE / Population(Allele,Count): Caucasian (C,102|T,18) / SNP Type: INTRON /
Context (SEQ ID NO: 2056): /
AGGGTCGTGGCAGGACTGAGCCTCAAGTGCCCACGAGCGTCACCCACACCTCCACACCTCCGCATGGCTTGCCTCCCAACCCT
GCTCCACTTTCGCGTTC
Y
CAAGGAGACCACCTGCACTCAAAGCCTTGCCTCCAGATGGACTTTGGAGGACCCAAGCTAAGCCAGCCCGGCGCCTGGGCCTA
GGCACTGCCCTTCCCTT / Celera SNP ID: hCV28966787 / Public SNP ID: rs7585314 /
SNP Chromosome Position: 127962493 / SNP in Genomic Sequence: SEQ ID NO: 396 / SNP Position Genomic: 31081 / Related Interrogated SNP: hCV1841975 / Related Interrogated SNP: hCV1841983 / SNP Source: dbSNP; HapMap /

Population(Allele,Count): Caucasian (T,69|C,157) / SNP Type: INTRON /
Context (SEQ ID NO: 2057): /
TAATTTCTTTATTTTTGGCAATGCTTTTATAGTAAAAATTTTAGTCACATAAGAAAACAAATCTCTTTGTAATCTCACCTTGG
ATTTGAACAAAAATTGT
M
ACATTCAAATGAATGTGCGTGGACCTGAAACTCTACATTTTAAGCACTGACCAATTTATTTGGAGTAAAGACATCATCTTGCA
TGGACATTTACTTGTAC / Celera SNP ID: hCV30711743 / Public SNP ID: rs11890243 /
SNP Chromosome Position: 127958041 / SNP in Genomic Sequence: SEQ ID NO: 396 /
 SNP Position Genomic: 26629 / Related Interrogated SNP: hCV1841975 / Related
Interrogated SNP: hCV1841983 / SNP Source: dbSNP; HapMap; ABI_Val /
Population(Allele,Count): Caucasian (C,61|A,165) / SNP Type: INTRON //

Gene Number: 60 / Gene Symbol: NCKAP5 - 344148 / Gene Name: NCK-associated
protein 5 / Chromosome: 2 / OMIM NUMBER: / OMIM Information: // Genomic
Sequence (SEQ ID NO: 397): // / SNP Information /
Context (SEQ ID NO: 2058): /
TGTTTTTCCTCCTCTTCAGGAGACCCACATAGAGATTTTCCAAACCCCACAAGGACATCCAGGTTCTCCACGGACTTATCGGT
GTCGGCAGCCAATGACA
Y
GTCTGAAGGACTTTTCTCATCACTGCTCTCTATGTGCAGCTCATCAAACGTTTCATTGTCATCAGTGTCTGAAAGCTGGAGGT
TGAGAGCCATGCGGCCA / Celera SNP ID: hCV25748719 / Public SNP ID: rs61995744 /
SNP Chromosome Position: 133542574 / SNP in Genomic Sequence: SEQ ID NO: 397 /
 SNP Position Genomic: 123202 / SNP Source: Applera /
Population(Allele,Count): Caucasian (C,35|T,5) African American (C,33|T,5)
total (C,68|T,10) / SNP Type: MISSENSE MUTATION;ESS;CODING REGION;INTRON / SNP
Source: dbSNP; Applera / Population(Allele,Count): Caucasian (T,28|C,92) /
SNP Type: MISSENSE MUTATION;ESS;CODING REGION;INTRON /
Context (SEQ ID NO: 2059): /
TTGGCGGGTGATGAGGATGATGAGGAACTGCTGACCTGAGATGATGGTGACTCATTTACCCCTAAGAAGGAAGGCTTGGGGGG
TGTGGAGGCGCTATCAT
W
CAATTGTCCTTTTCTCCCTGGAGATACACTTTTGGAGGACGTCATTTCCAGTGGCTCATGGGTTGAAGGAGAGATCCTGGGAG
TCTGTAATCCTATGTCC / Celera SNP ID: hCV25749343 / Public SNP ID: rs16841277 /
SNP Chromosome Position: 133541107 / SNP in Genomic Sequence: SEQ ID NO: 397 /
 SNP Position Genomic: 121735 / Related Interrogated SNP: hCV25748719 / SNP
Source: Applera / Population(Allele,Count): Caucasian (A,5|T,31) African
American (A,5|T,33) total (A,10|T,64) / SNP Type: MISSENSE MUTATION;INTRON /
SNP Source: dbSNP; HapMap / Population(Allele,Count): Caucasian (T,92|A,24) /
SNP Type: MISSENSE MUTATION;INTRON /
Context (SEQ ID NO: 2060): /
TATTTTTATTTTTCTATTAGTTTGTTAAATATATTCTATATTTAAACTTCAATTTTGCAGGTTTCTGTTTCAGGCATATTAGA
GGAAGCTTCTGGAAACA
S
AATCCGAGATAGAAGTGATCTCAAGACTTTCAACTCAAAGCTGCTCGTCGATTTCCCCAGCAAATCTTCTGAGTTGACTAAAG
ATGAGACTGTAACATCT / Celera SNP ID: hCV2163177 / Public SNP ID: rs12618525 /
SNP Chromosome Position: 133572214 / SNP in Genomic Sequence: SEQ ID NO: 397 /
 SNP Position Genomic: 152842 / Related Interrogated SNP: hCV25748719 / SNP
Source: dbSNP; Celera; HapMap / Population(Allele,Count): Caucasian
(G,176|C,50) / SNP Type: INTRON /
Context (SEQ ID NO: 2061): /
TTCACATCAATGTGCTGTGGTGTTAAAATACATCTCATTTTGCATCTAATCTCCTTCCTATATAGGCTGATAACATATAATAA
ATGAGTTTTCCCTCCTA
W
TCTTGTACCAGTCACAAAGGAGTCTATAGCAACGTCCTTTGTGAAATTAGTAATTTTAGCCCAATCCATGCTGATGATTATGG
AGATGAAACAAAAAGCA / Celera SNP ID: hCV3212676 / Public SNP ID: rs4143562 /
SNP Chromosome Position: 133536434 / SNP in Genomic Sequence: SEQ ID NO: 397 /
 SNP Position Genomic: 117062 / Related Interrogated SNP: hCV25748719 / SNP
Source: dbSNP; Celera; HapMap; HGBASE / Population(Allele,Count): Caucasian
(T,92|A,28) / SNP Type: INTRON /
Context (SEQ ID NO: 2062): /
GAATATCTCATGTAATTTATTGAATACTGTGCTGAAAATGAACATCAGAATGGTTGTATGGGTGCTCACAGTACAGTATCTAC
TGAATGTGTATTTTGGC
Y

CCATCACAAAGTTAAAAAATCCTGTCGAACTACCCTAAGCTGCAGATTGTCTGTATGGTATGGTATGATAATTACGGGCAGAC
TTTGGAGTCAAATTTGG / Celera SNP ID: hCV7479717 / Public SNP ID: rs1367392 /
SNP Chromosome Position: 133548150 / SNP in Genomic Sequence: SEQ ID NO: 397 /
SNP Position Genomic: 128778 / Related Interrogated SNP: hCV25748719 / SNP
Source: dbSNP; Celera; HapMap; HGBASE / Population(Allele,Count): Caucasian
(T,92|C,28) / SNP Type: INTRON /
Context (SEQ ID NO: 2063): /
AAGATGGTGGTATAGTACGTCATAAGGTTTCAAACAGTGGTTTTATTTAGAAAAAGAGTGTGATACGTAGTGCATTGCATATA
TACATTGTTTTCATCCA
R

AATAACTTCTATGAAAACAATTCTTTGCAACACAGTTTAAAATAAGCAGACAGTGAATTTACCTTGAGCCCTAGTGGGGGGAA
ATTCTCTTCAACTCTGA / Celera SNP ID: hCV11828144 / Public SNP ID: rs11679975 /
SNP Chromosome Position: 133536212 / SNP in Genomic Sequence: SEQ ID NO: 397 /
SNP Position Genomic: 116840 / Related Interrogated SNP: hCV25748719 / SNP
Source: dbSNP; Celera; HapMap / Population(Allele,Count): Caucasian
(A,92|G,28) / SNP Type: INTRON /
Context (SEQ ID NO: 2064): /
ATGGCGCTACATAGCTCTTTTAAAATACATTCCAGACAGCCACCTAGGCAGCCAATGATAATAACGTGCAATGCAATGGGTGT
CCCGCTTAGGCACGTAC
R

TTAGAGGTTGCCAGTGGATTTGCTACAGGGCTTGAGTTGAATGCCAAGAAATTATTATTTTATACTGTCAATGTGAAAGTCAT
ATGGCTGAATACTGACC / Celera SNP ID: hCV11828147 / Public SNP ID: rs10496693 /
SNP Chromosome Position: 133532709 / SNP in Genomic Sequence: SEQ ID NO: 397 /
SNP Position Genomic: 113337 / Related Interrogated SNP: hCV25748719 / SNP
Source: dbSNP; Celera; HapMap; ABI_Val / Population(Allele,Count): Caucasian
(G,171|A,55) / SNP Type: INTRON /
Context (SEQ ID NO: 2065): /
CTCTACTCTTGAATGACCCCAGAAAATAATGCAGGGAAATATTTCAAGTTTTCCCTCAGAAAATTGTAGTATGTCCTATATTT
TAATAATCTCTTTATTA
S

AGTACAAAAAGATATGGATATAGTAATTCCATAAAGAGAATATTTTGTGTTTAAGTGCTCTGGAACTTGCCAAAAGCAACAAT
GAATACTATTAAATTAT / Celera SNP ID: hDV70679361 / Public SNP ID: rs16841210 /
SNP Chromosome Position: 133537506 / SNP in Genomic Sequence: SEQ ID NO: 397 /
SNP Position Genomic: 118134 / Related Interrogated SNP: hCV25748719 / SNP
Source: dbSNP; HapMap / Population(Allele,Count): Caucasian (G,91|C,27) / SNP
Type: INTRON //

Gene Number: 61 / Gene Symbol: ADCY9 - 115 / Gene Name: adenylate cyclase 9
/ Chromosome: 16 / OMIM NUMBER: 603302 / OMIM Information: // Genomic
Sequence (SEQ ID NO: 398): // / SNP Information /
Context (SEQ ID NO: 2066): /
TGACTTCTTCGATCTGCTGCATCTTAAAAGGGCGGAAGGCTATAGGAGCTTTTTGGATGGAAGACTTTTTTCTTCCTGTTCTTG
GGGCTCGAGGTGGCATG
Y

CTCTTGACAGAATTCTCACTCTCCTCATCTCCCTGCTTCATTAAGTCATCGGCTATGATTCTTGGCATCACGGAATGAATCAT
CCTCTCTTTGAGGGCTT / Celera SNP ID: hCV2590858 / Public SNP ID: rs2230738 /
SNP Chromosome Position: 4164364 / SNP in Genomic Sequence: SEQ ID NO: 398 /
SNP Position Genomic: 161712 / SNP Source: Applera /
Population(Allele,Count): Caucasian (C,32|T,4) African American (C,32|T,4)
total (C,64|T,8) / SNP Type: SILENT MUTATION / SNP Source: dbSNP; Celera;
HGBASE / Population(Allele,Count): Caucasian (C,175|T,51) / SNP Type: SILENT
MUTATION //

Gene Number: 62 / Gene Symbol: AKT2 - 208 / Gene Name: v-akt murine thymoma
viral oncogene homolog 2 / Chromosome: 19 / OMIM NUMBER: 164731 / OMIM
Information: Diabetes mellitus, type II, 125853 (3) // Genomic Sequence (SEQ ID
NO: 399): // / SNP Information /
Context (SEQ ID NO: 2067): /
CATAAAATAAGGCAGTGTCAAGGATGGGCCTGCCCTGGGGGAAGCACTCCCTAAGTGCCACTGTGGACGTTTTCAGGGGCTGT
GTGGGGACGACACACTG
Y

GACCCTACAAGCGAGCACCCTTGTGGACGCTGCCCCCTCCAGGCCGCAGGGACAGTGGCAGCAGCTGGCGCTGGGCTGGGTGG
GGCCGACGCCAGCCCTC / Celera SNP ID: hCV7831336 / Public SNP ID: rs3730055 /

SNP Chromosome Position: 40741694 / SNP in Genomic Sequence: SEQ ID NO: 399 / SNP Position Genomic: 15470 / SNP Source: Celera;HGBASE;dbSNP / Population(Allele,Count): no_pop (C,-|T,-) / SNP Type: INTRON //

Gene Number: 63 / Gene Symbol: SERPINC1 - 462 / Gene Name: serpin peptidase inhibitor, clade C (antithrombin), member 1 / Chromosome: 1 / OMIM NUMBER: / OMIM Information: // Genomic Sequence (SEQ ID NO: 400): // / SNP Information / Context (SEQ ID NO: 2068): / TTTTGTGACCTCCAAAGGACTCACAGGAATGACCTCCAACACCTTTGAGAAGACCAGGCCCTCTCCCTGGTAGTTACAGTCAA AGACCTGTTTGGAAGAC R TCATTTCAAGTGCTCTCCCTCCCACCCCACCTCTTGGGGTAAGGCCTTTCCTAAGCTACCCCTTGGGTCCCTAGCCTAAGAAA CAAGGGGGATGTCATCC / Celera SNP ID: hCV16180170 / Public SNP ID: rs2227589 / SNP Chromosome Position: 173886216 / SNP in Genomic Sequence: SEQ ID NO: 400 / SNP Position Genomic: 23274 / SNP Source: Applera / Population(Allele,Count): Caucasian (G,38|A,2) African American (G,35|A,3) total (G,73|A,5) / SNP Type: INTRON / SNP Source: dbSNP; HapMap; HGBASE / Population(Allele,Count): Caucasian (G,203|A,23) / SNP Type: INTRON / Context (SEQ ID NO: 2069): / TGGTTTTCCTGATTTTGCTCCAATAGGCACTTTTTTTAGGGGGGGGATTATGCTTTTGATCAAAAGCTTGGTGCTGAATCTCTC TTTTCTCCAGAGAGATT Y AGTCTCACCCTGCTTTCCCAACACATGGCGTTTGTGTAGAGGGGAGGACCAAAGGAACATGTGTGGTTTGGAAGCAGTGTTCT GTTCTGTTCTTCTTGTA / Celera SNP ID: hCV8911768 / Public SNP ID: rs941988 / SNP Chromosome Position: 173881893 / SNP in Genomic Sequence: SEQ ID NO: 400 / SNP Position Genomic: 18951 / SNP Source: dbSNP; HapMap; HGBASE / Population(Allele,Count): Caucasian (C,203|T,23) / SNP Type: INTRON / Context (SEQ ID NO: 2070): / GGAATGCATCTGAGACATAGAGGTCATCTCGGCCTTCTGCAACAATACCTGGAAGGAAGACCGGAGAAGTCTTTGTGAGATGG GAGAAAGTTGGCTTCAA Y CCACAGATGGGAATTCAGTTTGGATTAAGAGATCCTTTGGGGCTTTTTGAAACATAAGAAGAAATCCTAAATCTTCACCTAAT CATACTCTCACAATGAA / Celera SNP ID: hCV16290208 / Public SNP ID: rs2759328 / SNP Chromosome Position: 173876705 / SNP in Genomic Sequence: SEQ ID NO: 400 / SNP Position Genomic: 13763 / Related Interrogated SNP: hCV16180170 / Related Interrogated SNP: hCV8911768 / SNP Source: Applera / Population(Allele,Count): Caucasian (C,21|T,1) African American (C,25|T,1) total (C,46|T,2) / SNP Type: TFBS SYNONYMOUS;INTRON / SNP Source: dbSNP; HGBASE / Population(Allele,Count): Caucasian (C,203|T,23) / SNP Type: TFBS SYNONYMOUS;INTRON / Context (SEQ ID NO: 2071): / AAAGAATGAGGATGCTCTATATGGATGTACTGATATGGAAGGGTCCTTAGGATAATTATGTGGGGAAAAAGCAAGCTGCAGAA AGTGTTTATAGTATGCT R TGCTTTTTGTAACACAAAGTGAGGATGATGGGGTGAGGGGTGGCTAGAGACAAGGGTAGGAGTAGACTTATCAATATGTACCA TCTTATTTAGTTTTCTA / Celera SNP ID: hCV11342529 / Public SNP ID: rs1951627 / SNP Chromosome Position: 173891817 / SNP in Genomic Sequence: SEQ ID NO: 400 / SNP Position Genomic: 28875 / Related Interrogated SNP: hCV16180170 / Related Interrogated SNP: hCV8911768 / SNP Source: dbSNP; Celera; HGBASE / Population(Allele,Count): Caucasian (G,168|A,58) / SNP Type: INTERGENIC;UNKNOWN / Context (SEQ ID NO: 2072): / CAGCGGCACCAAGGGAGATGTGGAAGGCAGGCTGAAGAAGCAGAGAGAGAGAGAGGAGAGGACCCTGCCATTCATCTTCCCATCT CCACTGCCTTCGGTTGC Y CACTCTGTCCTTCAACTACATTTGGAAATGCAATTTGAGGCTGAGAGGACCCTGGGAGGGCTATGAAGCACAAATTAAAACCA AGACAGTTGTGCCACCA / Celera SNP ID: hCV15956059 / Public SNP ID: rs2227592 / SNP Chromosome Position: 173885712 / SNP in Genomic Sequence: SEQ ID NO: 400 / SNP Position Genomic: 22770 / Related Interrogated SNP: hCV16180170 / Related Interrogated SNP: hCV8911768 / SNP Source: dbSNP; HapMap; ABI_Val; HGBASE / Population(Allele,Count): Caucasian (C,203|T,23) / SNP Type: UTR5;INTRON / Context (SEQ ID NO: 2073): /

AAGTAACTCCCAAATGAGGTTCCAGATTCATTTTTCTCATCCCAGGTAGTAAACGTGTTTTAATGGGTTTGTTTCTTGCCCAT
GGCAACCTCAACACAGA
S
GGGAGAACTGTTTTATTACCCTAAAATAGTGAGAACCACTGTTCAAACTAACTAGCTACCAAGTGACTTCTAACTCATGAAGA
AAATTTGTATTTAAGAG / Celera SNP ID: hCV16135173 / Public SNP ID: rs2146372 /
SNP Chromosome Position: 173869569 / SNP in Genomic Sequence: SEQ ID NO: 400 /
SNP Position Genomic: 6627 / Related Interrogated SNP: hCV16180170 / Related
Interrogated SNP: hCV8911768 / SNP Source: dbSNP; HapMap; ABI_Val; HGBASE /
Population(Allele,Count): Caucasian (G,110|C,10) / SNP Type: PSEUDOGENE /
Context (SEQ ID NO: 2074): /
CTGGAGGGAGATATTGTCATCCATCCAGTTTCCTCTCTATACCAAATACAAAGTACACATAAATAAGCAAAACAAATTTTCAA
AGTTCCTTCTCTAGAAT
S
CTCTTTCTTTTGCATATGTAGTTTCAGTGGAGATTTCTCAACTAGCTGAAATGGAGCACTTGAATATATTAAAAGAGCCCAAA
CTGCTTACAGCAATTTC / Celera SNP ID: hDV70683187 / Public SNP ID: rs16846561 /
SNP Chromosome Position: 173869111 / SNP in Genomic Sequence: SEQ ID NO: 400 /
SNP Position Genomic: 6169 / Related Interrogated SNP: hCV16180170 / Related
Interrogated SNP: hCV8911768 / SNP Source: dbSNP / Population(Allele,Count):
Caucasian (C,201|G,23) / SNP Type: PSEUDOGENE /
Context (SEQ ID NO: 2075): /
TAATCCCTAGTGGTCATGGCAGGATAACCTCAATCCAGTCCTCTTTGACCTGAAAATATCTTTATTGATATTCCATGCATGGC
TCTCAATTCAAAGTGTA
Y
AGTGGGTCAAGGCTTAGGTAGTAAAATGGATCATCCATTTGTCCTCAGAATAATTCTGGAGGGAGATATTGTCATCCATCCAG
TTTCCTCTCTATACCAA / Celera SNP ID: hCV30404194 / Public SNP ID: rs6691053 /
SNP Chromosome Position: 173868955 / SNP in Genomic Sequence: SEQ ID NO: 400 /
SNP Position Genomic: 6013 / Related Interrogated SNP: hCV16180170 / Related
Interrogated SNP: hCV8911768 / SNP Source: dbSNP; HapMap /
Population(Allele,Count): Caucasian (C,179|T,47) / SNP Type: PSEUDOGENE //

Gene Number: 64 / Gene Symbol: ATP1B1 - 481 / Gene Name: ATPase, Na+/K+
transporting, beta 1 polypeptide / Chromosome: 1 / OMIM NUMBER: 182330 /
OMIM Information: // Genomic Sequence (SEQ ID NO: 401): // / SNP Information /
Context (SEQ ID NO: 2076): /
CACCATCAATATTTCTGTACAGGATTTACCTCTTGCACAAAGGGTATCTCTTCTCTAAATTCCCATTGTATCTCGTTTGTGAT
ATTCATTGTGAAACACA
R
TCATTTAGCACCTTGCATTGTTGTTCACTCATTTCTGTGTATTTTGCTCCCAAACCAGAATGTTTGCTGCTAAAATGTCCAGA
CTACATCTCAGATGTCA / Celera SNP ID: hCV8919133 / Public SNP ID: rs1200121 /
SNP Chromosome Position: 169068156 / SNP in Genomic Sequence: SEQ ID NO: 401 /
SNP Position Genomic: 2209 / SNP Source: dbSNP; HapMap; ABI_Val; HGBASE /
Population(Allele,Count): Caucasian (A,207|G,19) / SNP Type:
INTERGENIC;UNKNOWN /
Context (SEQ ID NO: 2077): /
ACAAAGTCACTCTTACTTCTCAATATTTATACCCATTGATTTAATAAGCATCTCATTTGCCAGCTTTCTGGTCCCACTTTGTA
GTTTAATTAACTGTCTC
Y
TTTAGCTCTTAAAAACTTGGGTGAACAAAAAGATTATCATAAGAAATGCAAACTGCCACACGAGTCCCTGCTGTGACTTGGGT
GCCATCAGTGTAGAGCT / Celera SNP ID: hCV8919166 / Public SNP ID: rs1200139 /
SNP Chromosome Position: 169081409 / SNP in Genomic Sequence: SEQ ID NO: 401 /
SNP Position Genomic: 15462 / Related Interrogated SNP: hCV8919444 / SNP
Source: dbSNP; HapMap; HGBASE / Population(Allele,Count): Caucasian
(T,22|C,96) / SNP Type: TRANSCRIPTION FACTOR BINDING SITE;INTRON //

Gene Number: 65 / Gene Symbol: ATP6V0A1 - 535 / Gene Name: ATPase, H+
transporting, lysosomal V0 subunit a1 / Chromosome: 17 / OMIM NUMBER: / OMIM
Information: // Genomic Sequence (SEQ ID NO: 402): // / SNP Information /
Context (SEQ ID NO: 2078): /
GCAAAAGGTCACTTCTAGTCTGGTATCCATTATCATTAGGAAGAGTGAAAAACTGGTCTATGGGGACCCAGGGCCTCCTCAAC
CCAGACAGAGAACCCAC
M
AACTCAGAAAGGAGACCGACAGTGCAGGGACTGGGTTAGTGCCCTAACAACATTGTAGGGTTTTTTAAGGGTTTACAACACCA
TATTTGTTATCCGAAAT / Celera SNP ID: hCV1952126 / Public SNP ID: rs7223784 /

SNP Chromosome Position: 40577580 / SNP in Genomic Sequence: SEQ ID NO: 402 / SNP Position Genomic: 5327 / SNP Source: Applera / Population(Allele,Count): Caucasian (A,21|C,11) African American (A,25|C,13) total (A,46|C,24) // / SNP Type: INTERGENIC;UNKNOWN / SNP Source: dbSNP; Celera; HapMap; ABI_Val / Population(Allele,Count): Caucasian (A,164|C,62) / SNP Type: INTERGENIC;UNKNOWN /
Context (SEQ ID NO: 2079): /
ACTTCTTTCTGCCCCAGGAACCTTGGGTAGAGGGGGCATTGGACCTCAAAGCCAGCTGGCAGCCTCAGCATTTCAGATCAAAG TGGCCTGCCTCTTCAAC
R
TCTCAGCCTACCCAAGGCATGTCCTAGGTGTAGGCTGCTCTCTGGTGGCCAGATAGGGAAGGACTCATTCATCCTTTGGCAAG GTGGGCTGCGATCTGAG / Celera SNP ID: hCV587962 / Public SNP ID: rs647397 / SNP Chromosome Position: 40679398 / SNP in Genomic Sequence: SEQ ID NO: 402 / SNP Position Genomic: 107145 / Related Interrogated SNP: hCV1952126 / SNP Source: dbSNP; HapMap; ABI_Val; HGBASE / Population(Allele,Count): Caucasian (G,166|A,60) / SNP Type: INTERGENIC;UNKNOWN /
Context (SEQ ID NO: 2080): /
GTGAGCCAAGATCACGTAACTGCACTCCGGCCTGGGTGACAGAGTGAAGACCCTGTCTCAAAAAAAAAAAAAAAAAAAAAAATGG TTTGGACACAGATCAGA
K
AAATTTGAATATTGACTATATATTTTATGATATTAAAGAATCATTGTTAGCTGGGCACGGTGGCTCACACCTGTAATCCCAGC ACTTTGGGAGGCCGAGG / Celera SNP ID: hCV3140239 / Public SNP ID: rs36023314 / SNP Chromosome Position: 40649360 / SNP in Genomic Sequence: SEQ ID NO: 402 / SNP Position Genomic: 77107 / Related Interrogated SNP: hCV1952126 / SNP Source: dbSNP; Celera / Population(Allele,Count): Caucasian (G,164|T,58) / SNP Type: INTRON /
Context (SEQ ID NO: 2081): /
CCCAGCTGTTGGGATTACAAGTGTCAGCCACTATGCCCGGACTATTTAAAAAAAATTTTTTTTGGAGAGTTTTCTGGTAAAAAC TGGAAAACCTGCTAGAC
R
GATTCTAAAAGAGCTGTAATACTTGATTTCTTTGACATTTTAATTTTTAGTCTGAAATTTTCATTTCCAAATTTTTCAATTTCT ATGCACAAAATTCTTTT / Celera SNP ID: hCV3140264 / Public SNP ID: rs6503704 / SNP Chromosome Position: 40592253 / SNP in Genomic Sequence: SEQ ID NO: 402 / SNP Position Genomic: 20000 / Related Interrogated SNP: hCV1952126 / SNP Source: dbSNP; Celera; HapMap / Population(Allele,Count): Caucasian (G,88|A,32) / SNP Type: INTERGENIC;UNKNOWN /
Context (SEQ ID NO: 2082): /
GTGGTGGGAAGGAAGCTCACTGAGACTTTTGCCTAGGAAGAACATTTTTGTACCGTTGTGTTTTACCTAATTTGCCTCCATTC CAGTAATGTTGCAGCAA
Y
GAGGGAGGAAGTTTTTATCCTTAGCTGGTGTGACTGGCCATAAAGCAGTTTTTTTTGGATCAGTAGACAGTTTTGATTAGTGTT GCATCATGCATTGACAG / Celera SNP ID: hCV11626701 / Public SNP ID: rs1032070 / SNP Chromosome Position: 40618251 / SNP in Genomic Sequence: SEQ ID NO: 402 / SNP Position Genomic: 45998 / Related Interrogated SNP: hCV1952126 / SNP Source: dbSNP; Celera; HapMap; ABI_Val; HGBASE / Population(Allele,Count): Caucasian (C,159|T,67) / SNP Type: INTRON /
Context (SEQ ID NO: 2083): /
GCATGAGACAGGGATTTGATTGTAGTTATCACTGTAAACATCAAACTGATTTTTATGCAGAACTATTGCCCCAGGGCATACCT TCCAAGGAACCTGGTCT
S
TCCTTACCTAGCGAATTCCACATACACATAGATGTACACCTACCTGTACACTCACAGATTGGGTGGTGATTAAAGGGACAGAA TCGGGGACCAGCTTCTT / Celera SNP ID: hCV27485323 / Public SNP ID: rs3785897 / SNP Chromosome Position: 40670230 / SNP in Genomic Sequence: SEQ ID NO: 402 / SNP Position Genomic: 97977 / Related Interrogated SNP: hCV1952126 / SNP Source: dbSNP; HapMap; ABI_Val; HGBASE / Population(Allele,Count): Caucasian (C,168|G,58) / SNP Type: UTR5;INTRON //

Gene Number: 66 / Gene Symbol: CASP5 - 838 / Gene Name: caspase 5, apoptosis-related cysteine peptidase / Chromosome: 11 / OMIM NUMBER: 602665 / OMIM Information: // Genomic Sequence (SEQ ID NO: 403): // / SNP Information / Context (SEQ ID NO: 2084): /
TTTTGGTCCATATTGAGAAGTGTTTGGGTAAACATTTGATGAGCCACGCGATTCTTTCGCAAAGAGTCTACCAAGATCAGGGC CTTGTCTTCAATTTTGG

Y
ATCATAATATTTTTTCTTTTCCTCTTCCTTCAATGTCAGAACATCGTGTTTTGCCAAATAATTAAAAACACCATGAAGAACAT
CTTTGCCCAGGTATTCC / Celera SNP ID: hCV12092542 / Public SNP ID: rs507879 /
SNP Chromosome Position: 104877927 / SNP in Genomic Sequence: SEQ ID NO: 403 /
SNP Position Genomic: 22960 / SNP Source: Applera /
Population(Allele,Count): Caucasian (C,3|T,5) African American (C,4|T,4) total
(C,7|T,9) / SNP Type: MISSENSE MUTATION;ESS;TRANSCRIPTION FACTOR BINDING
SITE;UTR5;CODING RE / GION // SNP Source: dbSNP; Celera; HapMap; HGBASE /
Population(Allele,Count): Caucasian (T,120|C,102) / SNP Type: MISSENSE
MUTATION;ESS;TRANSCRIPTION FACTOR BINDING SITE;UTR5;CODING RE / GION // /

Gene Number: 67 / Gene Symbol: ERCC3 - 2071 / Gene Name: excision repair
cross-complementing rodent repair deficiency, compleme / ntation group 3
(xeroderma pigmentosum group B complementing) // Chromosome: 2 / OMIM NUMBER:
133510 / OMIM Information: Xeroderma pigmentosum, group B (3);
Trichothiodystrophy (3) // Genomic Sequence (SEQ ID NO: 404): // / SNP
Information /
Context (SEQ ID NO: 2085): /
GGACATCAGCTGCAGTTGGAACATGCCTTTCCCCTAAATTCTTCACAATCTCCCGGTACACCGTCTGCTGCACTTCTGGGTGC
CTTGCCAGGAGGTACAC
M
GTCCAAGACAAGGTGAAGGACGTCTAAGGAGAGAAAGTGGCAGACACAGGCAGGCAGTGAGTAACACCAGGGACTGAAACAGA
GGCGGTGGCAGGTAGGA / Celera SNP ID: hCV1441133 / Public SNP ID: rs7568070 /
SNP Chromosome Position: 127953054 / SNP in Genomic Sequence: SEQ ID NO: 404 /
SNP Position Genomic: 409 / Related Interrogated SNP: hCV1841975 / Related
Interrogated SNP: hCV1841983 / SNP Source: Applera /
Population(Allele,Count): Caucasian (A,21|C,9) African American (A,16|C,18)
total (A,37|C,27) / SNP Type: ESE SYNONYMOUS;SILENT RARE CODON;SILENT MUTATION
/ SNP Source: dbSNP; Celera; HapMap; ABI_Val / Population(Allele,Count):
Caucasian (A,174|C,52) / SNP Type: ESE SYNONYMOUS;SILENT RARE CODON;SILENT
MUTATION /
Context (SEQ ID NO: 2086): /
GTGTAGCGGCTGGGGTAATCAGTCGTTATGCTCCCCACAGGAAATCTGAATGGCACATTCTCATGGCTGCCACAGGCTGACAA
CATGGAGCACCTATGCC
Y
ATTGTTACATTAGCAGGGCAGGTGGAATTGCTGGTCTCAGCTGTCACTTGCCTTAATAAACTGCATAATTCCATCAGGGACTC
CAGTCTTGCTGAGCTTC / Celera SNP ID: hCV25960135 / Public SNP ID: rs4150402 /
SNP Chromosome Position: 128050134 / SNP in Genomic Sequence: SEQ ID NO: 404 /
SNP Position Genomic: 97489 / Related Interrogated SNP: hCV1841975 / Related
Interrogated SNP: hCV1841983 / SNP Source: Applera /
Population(Allele,Count): Caucasian (C,27|T,11) African American (C,15|T,11)
total (C,42|T,22) // / SNP Type: MISSENSE MUTATION;INTRON / SNP Source:
dbSNP; HapMap; HGBASE / Population(Allele,Count): Caucasian (T,61|C,165) / SNP
Type: MISSENSE MUTATION;INTRON /
Context (SEQ ID NO: 2087): /
ATTTGAAAAAGTAAAACTATATCTAGCAGATGATACGAGTGTACATACAAAACCCTAGAAAATCAATAAAACTAACAATTCGA
TAAGGTAGCAAGCTGTA
R
AATTATCTTATAGAACTTGATAGCCTTCATATACACAATGATAACCAGTTAGAGTATATAATGGCATGGAAAATGCCATTTAT
ATGGCAAAAAAGAAGAT / Celera SNP ID: hCV1441173 / Public SNP ID: rs4536600 /
SNP Chromosome Position: 128002572 / SNP in Genomic Sequence: SEQ ID NO: 404 /
SNP Position Genomic: 49927 / Related Interrogated SNP: hCV1841975 / Related
Interrogated SNP: hCV1841983 / SNP Source: dbSNP; Celera; HapMap; HGBASE /
Population(Allele,Count): Caucasian (G,76|A,150) / SNP Type:
INTERGENIC;UNKNOWN /
Context (SEQ ID NO: 2088): /
GGATTCCGTGACACTGTCATGGAAAACACCTGCATATTCACCCCCAAGGTATGAGAAATAATGATGGGCCATAAGGATCTAGG
TCTTTGCTTTGGGATTC
Y
CTCCCTCCAGAGTCTATTTAGCCCCAGGGCACATGGCAGCTCTCACCCCTATCGTCTTCCTAACTAAAGTGGTTTAAGAGGAG
TGACCTCCTGCAACAGA / Celera SNP ID: hCV1441189 / Public SNP ID: rs4150499 /
SNP Chromosome Position: 128028792 / SNP in Genomic Sequence: SEQ ID NO: 404 /
SNP Position Genomic: 76147 / Related Interrogated SNP: hCV1841975 / Related

Interrogated SNP: hCV1841983 / SNP Source: dbSNP; Celera; HGBASE / Population(Allele,Count): Caucasian (T,26|C,90) / SNP Type: TRANSCRIPTION FACTOR BINDING SITE;INTRON /
Context (SEQ ID NO: 2089): /
GGATGGGGAGAGAATAGTTCTTAAATAAGGCAAAGTAAATATAGGAACTGTCTCATTGCCCTGCTCAAATTGGCACTAATGTG ACAGAACACAAACCCAC
M
AAGAAAACAGGAGAAGAAATAACAGCAGATGAGAGAAGTGGGTAACATTTCAGAAGCTGAAAAGCAGACAGATGCAGGCACAG TACCTGAGCTAACAGAC / Celera SNP ID: hCV1441196 / Public SNP ID: rs4150474 / SNP Chromosome Position: 128033327 / SNP in Genomic Sequence: SEQ ID NO: 404 / SNP Position Genomic: 80682 / Related Interrogated SNP: hCV1841975 / Related Interrogated SNP: hCV1841983 / SNP Source: dbSNP; Celera; HapMap; HGBASE / Population(Allele,Count): Caucasian (C,28|A,90) / SNP Type: TRANSCRIPTION FACTOR BINDING SITE;INTRON /
Context (SEQ ID NO: 2090): /
GATTTTTAAATGCAATCACATCCAGGGAGTTATTTTCACACCAGCTGCAAGTGTGCGAAACCAGATTCTCAATTGGTAGTGTC AGCAATTCTTCCATTTA
R
GCCACACTTCCACACACGAGTCAGCACTGCTACTGAGTACCGCCACCTCTTCACACACTTCCCAGCAGGGCTGGGAGCATATC AGTTAGGCGCTTGGGGC / Celera SNP ID: hCV8806682 / Public SNP ID: rs1011019 / SNP Chromosome Position: 128037560 / SNP in Genomic Sequence: SEQ ID NO: 404 / SNP Position Genomic: 84915 / Related Interrogated SNP: hCV1841975 / Related Interrogated SNP: hCV1841983 / SNP Source: dbSNP; Celera; HapMap; ABI_Val; HGBASE / Population(Allele,Count): Caucasian (A,61|G,165) / SNP Type: INTRON /
Context (SEQ ID NO: 2091): /
CTGCCTGGGTCCCTCCCCGGGACCAGTCCCTGGAGACCCTGCCCGCCGCCTCTCTCGGGCTACCACTCTCCGAAACTCCATCC CTGACTCCCCTCCCCGC
Y
ACCTCCTCCCCAGGGGTCCCGGCTGGGGCCCCTCCAGGGGACCCCTCCCTGCCGCCACCTCCCGAGACACCCATTTCCCCCGC CTCTTCCCAGCCGTTCC / Celera SNP ID: hCV8807983 / Public SNP ID: rs1504136 / SNP Chromosome Position: 127977118 / SNP in Genomic Sequence: SEQ ID NO: 404 / SNP Position Genomic: 24473 / Related Interrogated SNP: hCV1841975 / Related Interrogated SNP: hCV1841983 / SNP Source: dbSNP; HapMap; ABI_Val; HGBASE / Population(Allele,Count): Caucasian (T,71|C,155) / SNP Type: INTRON /
Context (SEQ ID NO: 2092): /
ATTTGTGGAAGTGGATTCACCCTTTCTGCCTTCCGCCCTGTGAGGACACAGAAAGAAGGCCCTCATCTGATGCTGGTGCCTTA ATCTTGGACTTCCCAAC
S
TCCAGAACTGTGAGAATACAAATTTCTGTTCCTTAAAAAAATGACTCAGTCTGTGGTGTCCTGTTATAGCAGCACAAACAGACT AAGATAGTACCCTTTCC / Celera SNP ID: hCV8807988 / Public SNP ID: rs3893313 / SNP Chromosome Position: 127988387 / SNP in Genomic Sequence: SEQ ID NO: 404 / SNP Position Genomic: 35742 / Related Interrogated SNP: hCV1841975 / SNP Source: dbSNP; HapMap; ABI_Val; HGBASE / Population(Allele,Count): Caucasian (C,82|G,36) / SNP Type: INTERGENIC;UNKNOWN /
Context (SEQ ID NO: 2093): /
ACTCTGCAGAGTCCCAAGACAGTGCAGGGTATCATATAGCAAGGGGGCTAAGCATGCTAACATACTAAATCAGGTCTCTCCCA TCTGATAAAGCCACCAG
Y
TCCACTCCAATGATAACCCATTAATCCATTAACCCATGATTAATCCATGTGTGAGGGCAGAGCCCTCATGATCCAATGACCTC TTAAAGGCCCCACCTTT / Celera SNP ID: hCV8810479 / Public SNP ID: rs1604817 / SNP Chromosome Position: 127999921 / SNP in Genomic Sequence: SEQ ID NO: 404 / SNP Position Genomic: 47276 / Related Interrogated SNP: hCV1841975 / Related Interrogated SNP: hCV1841983 / SNP Source: dbSNP; HapMap; ABI_Val; HGBASE / Population(Allele,Count): Caucasian (C,35|T,83) / SNP Type: INTERGENIC;UNKNOWN /
Context (SEQ ID NO: 2094): /
AGAACAAATCTGCATCATTAAGTGAGGCGTGACTGCATACACAGAGTTTTCTCCTCCTGGAGCCCCCCAGCAGTGCAGCAGGC CTAGTACAAACATGAAC
R
GTGTGCTAAACAAATCTGCCTTAAAAGTAGACAACGGCCAGTCGCAGTGGCTCATGCCTGTAATCCCAGCACTTTGGGAGGCC AAGGCGGGTGGATCACC / Celera SNP ID: hCV9465822 / Public SNP ID: rs11683427 / SNP Chromosome Position: 127956543 / SNP in Genomic Sequence: SEQ ID NO: 404 /

SNP Position Genomic: 3898 / Related Interrogated SNP: hCV1841975 / Related Interrogated SNP: hCV1841983 / SNP Source: dbSNP; Celera; HapMap / Population(Allele,Count): Caucasian (A,169|G,55) / SNP Type: INTRON / Context (SEQ ID NO: 2095): /
CAGACATAGAACTCATGGTGCCAAAGCGCCGAGATGCCTGCCGGGAAGGGGGAACCAGCCCATGTTAGTCTCCAGAAGAAAAG
ATACTCCTTTTATAATA
Y
TTATAATCACAGCAAATTTTATAAGACTTACATAAATTTAATACATCATTTTTAATAATGTTTACAGATCGCTTACTGTCTCA
GGCATTGTTCTAAGCAT / Celera SNP ID: hCV11268771 / Public SNP ID: rs4662718 /
SNP Chromosome Position: 128015367 / SNP in Genomic Sequence: SEQ ID NO: 404 /
 SNP Position Genomic: 62722 / Related Interrogated SNP: hCV1841975 / Related Interrogated SNP: hCV1841983 / SNP Source: dbSNP; Celera; HapMap; HGBASE / Population(Allele,Count): Caucasian (C,61|T,165) / SNP Type: TRANSCRIPTION FACTOR BINDING SITE;INTRON;PSEUDOGENE /
Context (SEQ ID NO: 2096): /
GACAACACACTGAGAGGAAGCAAAGGGCATCAGACTGCCAAACTGCTGCACTGGAAGCTCCAATGTTAGTTCTAGGACCTGGC
CAAACTACTGATCAAGT
R
TAAAGTCGAGTAAAGACATCTGTAAACATGTGAAGTCTCCAAAGCTTCTCCTCCTACATCCCTTCTCTCAGGAACCTACTGGA
ACATGTGCTCCATCAAA / Celera SNP ID: hCV26980926 / Public SNP ID: rs4150471 /
SNP Chromosome Position: 128034539 / SNP in Genomic Sequence: SEQ ID NO: 404 /
 SNP Position Genomic: 81894 / Related Interrogated SNP: hCV1841975 / Related Interrogated SNP: hCV1841983 / SNP Source: dbSNP; Celera; HapMap; ABI_Val; HGBASE / Population(Allele,Count): Caucasian (A,28|G,92) / SNP Type: UTR5;INTRON;PSEUDOGENE /
Context (SEQ ID NO: 2097): /
TGCTAAGAGCTCTAATAGAAAAAATGAACCATATGCAATGAGAGATGGATACTGCAAGCATAGTGATGGAAACAATAAGGAAG
AATCAAAAGGAAAGAAA
Y
GCTAGAAATAAAGAACACTGTCATGGAAATAATGCATTTGATGGGCTTTTAAATAGACTGGACACATGGCCGGGCGCAGTGGC
TCATGTCTGTAATCCCA / Celera SNP ID: hCV27964958 / Public SNP ID: rs4662713 /
SNP Chromosome Position: 127994103 / SNP in Genomic Sequence: SEQ ID NO: 404 /
 SNP Position Genomic: 41458 / Related Interrogated SNP: hCV1841975 / Related Interrogated SNP: hCV1841983 / SNP Source: dbSNP; HapMap; HGBASE / Population(Allele,Count): Caucasian (C,60|T,164) / SNP Type: INTERGENIC;UNKNOWN /
Context (SEQ ID NO: 2098): /
ATTCCTTCAAGAAAACCAGCTTGTTGGCAGTAATAGAGAAGAAATGAGCTTCTCCCAACAACAGCCAACAACAGCCAATGACA
ACAGCAAAATAGGAAAA
W
GGGTTGCCCTCTGAAAGGTAAATAAACTCTATACACAATAAATGACCATCTGTGGAAGTAAAACCGAGAGACAGGCTTTACCA
CCTCCCAGAACTCCAAT / Celera SNP ID: hCV28955091 / Public SNP ID: rs7567389 /
SNP Chromosome Position: 127982645 / SNP in Genomic Sequence: SEQ ID NO: 404 /
 SNP Position Genomic: 30000 / Related Interrogated SNP: hCV1841983 / Related Interrogated SNP: hCV1841975 / SNP Source: dbSNP; HapMap; ABI_Val / Population(Allele,Count): Caucasian (A,24|T,84) / SNP Type: INTERGENIC;UNKNOWN /
Context (SEQ ID NO: 2099): /
CCTTCTGTTCCTGTCTCAGTACAAAGTAAGGATGATGTCTATGAGGCTTTCATGAAAGAGATGGAAGGGCTGCTGTGACAGCT
TTTGATGCCCGAACAGG
M
TTTTGTTCACAACAGTGGCCCATGGAGAAAGAGGCTCTTATTAAACTTAGAAGAAAGAACCGCTTCCGTTGTCAGGGTGTTTT
CTAATTTCAGTTCAAGG / Celera SNP ID: hCV28955092 / Public SNP ID: rs6430936 /
SNP Chromosome Position: 128007686 / SNP in Genomic Sequence: SEQ ID NO: 404 /
 SNP Position Genomic: 55041 / Related Interrogated SNP: hCV1841975 / Related Interrogated SNP: hCV1841983 / SNP Source: dbSNP / Population(Allele,Count): Caucasian (A,60|C,166) / SNP Type: MISSENSE MUTATION;TFBS SYNONYMOUS;PSEUDOGENE /
Context (SEQ ID NO: 2100): /
AGGGTCGTGGCAGGACTGAGCCTCAAGTGCCCACGAGCGTCACCCACACCTCCACACCTCCGCATGGCTTGCCTCCCAACCCT
GCTCCACTTTCGCGTTC
Y
CAAGGAGACCACCTGCACTCAAAGCCTTGCCTCCAGATGGACTTTGGAGGACCCAAGCTAAGCCAGCCCGGCGCCTGGGCCTA

GGCACTGCCCTTCCCTT / Celera SNP ID: hCV28966787 / Public SNP ID: rs7585314 / SNP Chromosome Position: 127962493 / SNP in Genomic Sequence: SEQ ID NO: 404 / SNP Position Genomic: 9848 / Related Interrogated SNP: hCV1841975 / Related Interrogated SNP: hCV1841983 / SNP Source: dbSNP; HapMap / Population(Allele,Count): Caucasian (T,69|C,157) / SNP Type: INTRON / Context (SEQ ID NO: 2101): / TAATTTCTTTATTTTTGGCAATGCTTTTATAGTAAAAATTTTAGTCACATAAGAAAACAAATCTCTTTGTAATCTCACCTTGG ATTTGAACAAAAATTGT M

ACATTCAAATGAATGTGCGTGGACCTGAAACTCTACATTTTAAGCACTGACCAATTTATTTGGAGTAAAGACATCATCTTGCA TGGACATTTACTTGTAC / Celera SNP ID: hCV30711743 / Public SNP ID: rs11890243 / SNP Chromosome Position: 127958041 / SNP in Genomic Sequence: SEQ ID NO: 404 / SNP Position Genomic: 5396 / Related Interrogated SNP: hCV1841975 / Related Interrogated SNP: hCV1841983 / SNP Source: dbSNP; HapMap; ABI_Val / Population(Allele,Count): Caucasian (C,61|A,165) / SNP Type: INTRON / Context (SEQ ID NO: 2102): / GGGGGCAGTTTCCCCCATGCTATTCTCATGATAGTGAATTCTCATGAGCTGATAATTTTATAAGGGGCTCTTCCCCCTTTGCT CAGCACTTCTCCTTTCT R

CCTCCTTGTGAAGAAGGCACCTTGCTTCCCCTTTGCCTTCTGCCATGATTGTAAGTTTCCTGAAGCCTCCCCAATCGTGCTGA ACTGTGAGTCAATTAAA / Celera SNP ID: hCV30418053 / Public SNP ID: rs6757492 / SNP Chromosome Position: 127995332 / SNP in Genomic Sequence: SEQ ID NO: 404 / SNP Position Genomic: 42687 / Related Interrogated SNP: hCV1841975 / Related Interrogated SNP: hCV1841983 / SNP Source: dbSNP; HapMap; ABI_Val / Population(Allele,Count): Caucasian (G,31|A,87) / SNP Type: INTERGENIC;UNKNOWN / Context (SEQ ID NO: 2103): / GAGCTTGCAGTGAGCCGAGATCATGCCACTGCACTCCAGCCTGGGCGACAGAGCAAGACTCTGTCTCAAAATAAATAAATAAA TAAATAAATAATCTCCA R

CACCATGTGAGAGACCATTCCTTATACTCAGTAGGTTTGATAGTAGGGTTTGTGTTTGTTTTTAATCATTAGTTCTGATTTAG GTAATCAAGATGTCCAG / Celera SNP ID: hCV30166207 / Public SNP ID: rs7590030 / SNP Chromosome Position: 128055967 / SNP in Genomic Sequence: SEQ ID NO: 404 / SNP Position Genomic: 103322 / Related Interrogated SNP: hCV1841983 / Related Interrogated SNP: hCV1841975 / SNP Source: dbSNP / Population(Allele,Count): Caucasian (G,68|A,158) / SNP Type: INTERGENIC;UNKNOWN / Context (SEQ ID NO: 2104): / GCCTGTAATCCCAACTACTCAGGAGGCTGAGGCAGGAGAATCACTTGAACCCGGGAGGCAGAGGTTGCAGTGAGCTGAGATTG TGCCATGACACTCCAGC Y

GGGGCGACAATGCAAGACTCCGAGAGAGAGAGAGAGAAAAAAAGAAAGAAAGAAAAAGAAAGAAAGAAAGAAAAAAAGAAAGAAAG AAAGAAAGAAGGAAGGA / Celera SNP ID: hCV29570359 / Public SNP ID: rs6738690 / SNP Chromosome Position: 127992725 / SNP in Genomic Sequence: SEQ ID NO: 404 / SNP Position Genomic: 40080 / Related Interrogated SNP: hCV1841975 / Related Interrogated SNP: hCV1841983 / SNP Source: dbSNP / Population(Allele,Count): Caucasian (T,27|C,91) / SNP Type: INTERGENIC;UNKNOWN / Context (SEQ ID NO: 2105): / GGTGTGTGGCTTATAGACCCAATCACCCATCTCCACAGAAACCTGGACAGTTTTGACTAGGGTACATAGATGGGATGAAATGA AGGAAGGCTGTTGGACT Y

CCAGGATTCCACAGGTAGGAAAGGGATTGGCAGGGGCAGTTCAGAGGCCAGTGAGGCTGCTTCCGCCACCACAGGACTGAAAG GGACAGGCTTGGGTTTG / Celera SNP ID: hCV30598525 / Public SNP ID: rs7556675 / SNP Chromosome Position: 128053747 / SNP in Genomic Sequence: SEQ ID NO: 404 / SNP Position Genomic: 101102 / Related Interrogated SNP: hCV1841975 / Related Interrogated SNP: hCV1841983 / SNP Source: dbSNP; HapMap / Population(Allele,Count): Caucasian (C,61|T,165) / SNP Type: INTERGENIC;UNKNOWN //

Gene Number: 68 / Gene Symbol: F13B - 2165 / Gene Name: coagulation factor XIII, B polypeptide / Chromosome: 1 / OMIM NUMBER: 134580 / OMIM Information: Factor XIIIB deficiency (3) // Genomic Sequence (SEQ ID NO: 405): // / SNP Information /

Context                    (SEQ ID NO: 2106): /
TAAATAATTTTTTTTTCCCATAGAAAAAATGCACTAAGCCTGACCTGAGTAATGGTTACATCTCTGATGTAAAGTTATTGTATAA
AATTCAAGAGAACATGC
R

TTATGGTTGCGCTTCAGGGTACAAAACCACTGGAGGGAAGGATGAAGAAGTGGTTCAATGTCTCTCTGATGGATGGTCTTCTC
AACCAACCTGTAGGAAA / Celera SNP ID:    hCV2532034 / Public SNP ID:    rs6003 /  SNP
Chromosome Position:    197031021 / SNP in Genomic Sequence:    SEQ ID NO: 405 /
SNP Position Genomic:    32700 / SNP Source:    Applera / Population(Allele,Count):
  Caucasian (G,2|A,32) African American (G,13|A,23) total (G,15|A,55) / SNP
Type:    MISSENSE MUTATION / SNP Source:    Applera / Population(Allele,Count):
Caucasian (G,5|A,35) African American (G,14|A,18) total (G,19|A,53) / SNP Type:
  MISSENSE MUTATION / SNP Source:    dbSNP; Celera; HapMap; HGBASE /
Population(Allele,Count):    Caucasian (G,20|A,206) / SNP Type:    MISSENSE
MUTATION /
Context                    (SEQ ID NO: 2107): /
TTATCATGGACGTTCTAAACATTTCTCATGAATGAATGTTGATATAGCACAGTGCTGATCTCACCACCTGCCACAAAGTAAAA
ACTCTCTAAATGTCAAA
Y

ACTACTATTATAAGTAATACTATTCTATCATTGAGACAACTATGGTTCTGTATCTCTTTAAGGAGTGCAGTCTAGGCAACAAC
ATATTTCAACATCAGGT / Celera SNP ID:    hCV2532031 / Public SNP ID:    rs1412632 /
SNP Chromosome Position:    197036868 / SNP in Genomic Sequence:    SEQ ID NO: 405 /
 SNP Position Genomic:    38547 / Related Interrogated SNP:    hCV2532034 / SNP
Source:    Applera / Population(Allele,Count):    Caucasian (T,34|C,4) African
American (T,23|C,13) total (T,57|C,17) / SNP Type:    INTERGENIC;UNKNOWN / SNP
Source:    dbSNP; Celera; HGBASE / Population(Allele,Count):    Caucasian
(C,20|T,206) / SNP Type:    INTERGENIC;UNKNOWN /
Context                    (SEQ ID NO: 2108): /
CATGTATTCTTAAGATTTGTAAAACCATATTTTGAGATTTTTTATAATCTAGGTTATTAAAAAAGTTTATAAAATAAATAATT
TTTATAATTTTAGAAAC
R

TGTTTGGCTCCTGAATTATATAATGGAAATTATTCCACAACACAGAAAACATTCAAAGTGAAGGACAAAGTACAATACGAATG
TGCTACTGGCTACTACA / Celera SNP ID:    hCV3091554 / Public SNP ID:    rs5997 /  SNP
Chromosome Position:    197030201 / SNP in Genomic Sequence:    SEQ ID NO: 405 /
SNP Position Genomic:    31880 / Related Interrogated SNP:    hCV2532034 / SNP
Source:    Applera / Population(Allele,Count):    Caucasian (G,29|A,3) African
American (G,8|A,4) total (G,37|A,7) / SNP Type:    ESS;ESE SYNONYMOUS;SILENT RARE
CODON;SILENT MUTATION / SNP Source:    dbSNP; Celera; HapMap; HGBASE /
Population(Allele,Count):    Caucasian (A,20|G,206) / SNP Type:    ESS;ESE
SYNONYMOUS;SILENT RARE CODON;SILENT MUTATION /
Context                    (SEQ ID NO: 2109): /
TTATAGTCAGATTGCCCAATGTGTAAGATTAGTGTGCGAGTGGAAGCTAGGCAGTGTACATGGGTATGTTGTTCAGGAACATT
ATCAATATTATCAGGCC
R

TAGGATGAAGAATCCAGGGAGGACACTTAGCATTAGCAAGAGTGAACATACAAAGAAGGAAGCTACAGCAATACAGCTTTTCT
AAAATAAACATTTTTGG / Celera SNP ID:    hCV2532032 / Public SNP ID:    rs4915148 /
SNP Chromosome Position:    197035538 / SNP in Genomic Sequence:    SEQ ID NO: 405 /
 SNP Position Genomic:    37217 / Related Interrogated SNP:    hCV2532034 / SNP
Source:    dbSNP; Celera; HapMap; ABI_Val; HGBASE / Population(Allele,Count):
Caucasian (A,20|G,206) / SNP Type:    INTRON /
Context                    (SEQ ID NO: 2110): /
GTTGAAGGGAATAGGGTAATAACTGAAATTATCATATTGCAAGGCAAAGAGAGGTAATTTACTTTAAGTAACAAGAAACTTAT
TTTGATAATTAAGCATA
R

GATGCCAGAAAAATGCTTGAAACTCTGGCCTCTGGAAGGGCAAGATTGCTACTCTAATGCTTTATCATTTGTAAGACTCATCT
CTCTTCATGTGCCAACT / Celera SNP ID:    hCV2532033 / Public SNP ID:    rs1759006 /
SNP Chromosome Position:    197031664 / SNP in Genomic Sequence:    SEQ ID NO: 405 /
 SNP Position Genomic:    33343 / Related Interrogated SNP:    hCV2532034 / SNP
Source:    dbSNP; HapMap; HGBASE / Population(Allele,Count):    Caucasian
(A,9|G,111) / SNP Type:    INTRON /
Context                    (SEQ ID NO: 2111): /
GTGGCTGTTAACTGAAGGCAGCCCTCTGCCCCTTGCCTTGCAGCCGTTCTGCTTTGGAAGCTCATGATGTAGCCTCTTGTTTC
TTCAAAACCAGCAAGGA
K

```
AGAGAGTCTCTTGGCAAGAATGGCGTTAAAAACTGATGTTACATAATTATGTGTGCATAATCACGTACTAATCCATCACCTTT
GCTGTATTTTGTGGGCT / Celera SNP ID:  hCV2532038 / Public SNP ID:  rs1759008 /
SNP Chromosome Position:  197026895 / SNP in Genomic Sequence:  SEQ ID NO: 405 /
 SNP Position Genomic:  28574 / Related Interrogated SNP:  hCV2532034 / SNP
Source:  dbSNP; Celera; HapMap; ABI_Val; HGBASE / Population(Allele,Count):
Caucasian (T,20|G,206) / SNP Type:  INTRON /
Context               (SEQ ID NO: 2112): /
TCATGGAAAGGGGTATCACACAAGGAAGTTAACATCAGGAGGTGAGGATCAAAGGAATGCTCCTAGAATCAGTCTGCCATTCT
CTCTATGATGCTTGACA
Y

GATGAATATTTTAATTTAATCTTTATTATTTTTTGTAATTATATGACTAAAGGTGCTGATTAATTTTTTCCATAAGGAAGAAG
AAACAGATGTCCTCCTC / Celera SNP ID:  hCV2532039 / Public SNP ID:  rs1759009 /
SNP Chromosome Position:  197026672 / SNP in Genomic Sequence:  SEQ ID NO: 405 /
 SNP Position Genomic:  28351 / Related Interrogated SNP:  hCV2532034 / SNP
Source:  dbSNP; Celera; HapMap; HGBASE / Population(Allele,Count):  Caucasian
(T,20|C,206) / SNP Type:  INTRON /
Context               (SEQ ID NO: 2113): /
CCAGGACTCTTGGCATTTTTACATTTTACACTTTCAAGTCTTTTTTTATATCAAATAATTTTTGGAATTATGCAGACTACAAA
ATCTGAAATAAGAGAGT
Y

CTACAAGCATCAAAAAAATGAGTGTCAGTATTATCAAGGTCTGTTAGAGACATGAAACAGCAACACTGTCACTTTTTCATTTGA
AGTGAGAGTTATACTTC / Celera SNP ID:  hCV2532061 / Public SNP ID:  rs857021 /
SNP Chromosome Position:  197007479 / SNP in Genomic Sequence:  SEQ ID NO: 405 /
 SNP Position Genomic:  9158 / Related Interrogated SNP:  hCV2532034 / SNP
Source:  dbSNP; Celera; HapMap; HGBASE / Population(Allele,Count):  Caucasian
(T,19|C,205) / SNP Type:  INTERGENIC;UNKNOWN /
Context               (SEQ ID NO: 2114): /
CTCAGTGATTTCTCTTTGCCCACACTTTTTGCCCTTCAGATCAGTCTCTCCATATTACAGTCAGTGATGTCTTTTAAAAATTC
CCAGTAAGCATCGCATT
Y

TATTTAGTGACTTCCAAAATCTTTAATTTGACCTACAAAGGCCTTCACAATCAGGGCTCCAACCTCTCCAAGTTTCGCATACT
CCTCTCTGTCCTTCACT / Celera SNP ID:  hCV2532062 / Public SNP ID:  rs1332669 /
SNP Chromosome Position:  197005883 / SNP in Genomic Sequence:  SEQ ID NO: 405 /
 SNP Position Genomic:  7562 / Related Interrogated SNP:  hCV2532034 / SNP
Source:  dbSNP; Celera; HapMap; HGBASE / Population(Allele,Count):  Caucasian
(C,19|T,207) / SNP Type:  INTERGENIC;UNKNOWN /
Context               (SEQ ID NO: 2115): /
TTAGAATTATTTATTCTCATTTTTATATTGATTGATATATTTGAAGCTAATATTTTACTCTGATTACTGCTTTAGTTAATATC
CTATAAATTCTGATATG
Y

GATGTTTTCTTTATTGTTTTCTAGAAATTCTGTCATTTTAGTTTATATTTATTTTTTTGACCCCAGAGTTGCTTAATAGAAAGT
TTGTACATTTCCAGGGA / Celera SNP ID:  hCV2759671 / Public SNP ID:  rs2336595 /
SNP Chromosome Position:  197000476 / SNP in Genomic Sequence:  SEQ ID NO: 405 /
 SNP Position Genomic:  2155 / Related Interrogated SNP:  hCV2532034 / SNP
Source:  dbSNP; Celera; HapMap; HGBASE / Population(Allele,Count):  Caucasian
(T,9|C,111) / SNP Type:  INTERGENIC;UNKNOWN /
Context               (SEQ ID NO: 2116): /
GTTTCTCTGGGCAGAACCAGGTAGCATCCTTCTAAGCTTTGGAATACCCTGGGGCAATCACCATGTTAGTGATTCTTTGATCT
TTGACTTAGTGTCCTAT
R

CTACAAGGATGGCACATCTTCTAAGCAAAGTTTGCTTTCATATTCATCAGAATAAAATTTATCTCACAAAATAAAACTATTTA
AAAAGCAAAATGGTAAT / Celera SNP ID:  hCV2759673 / Public SNP ID:  rs1412639 /
SNP Chromosome Position:  197003593 / SNP in Genomic Sequence:  SEQ ID NO: 405 /
 SNP Position Genomic:  5272 / Related Interrogated SNP:  hCV2532034 / SNP
Source:  dbSNP; Celera; HapMap; HGBASE / Population(Allele,Count):  Caucasian
(G,9|A,109) / SNP Type:  INTERGENIC;UNKNOWN /
Context               (SEQ ID NO: 2117): /
CAAGGGTGTGTGTTCAGGGGTGGACAATACCCCTTTCACACTTTTCACAGTTTGGGCATTCACAGTATGTGGGCTATTTTCTG
GGTCATGCAGGAGCAAT
R

TACTTTCTTCAGAGGGTCTGTGGATTATCTTGGCTTTCCTGTTATATCCTTGCAGCAGTTCTTGGAGCAAAAGTTCACAATGC
GAGTCTCCACATCCTGC / Celera SNP ID:  hCV8356419 / Public SNP ID:  rs1412631 /
SNP Chromosome Position:  197037237 / SNP in Genomic Sequence:  SEQ ID NO: 405 /
```

SNP Position Genomic: 38916 / Related Interrogated SNP: hCV2532034 / SNP Source: dbSNP; Celera; HapMap; HGBASE / Population(Allele,Count): Caucasian (A,20|G,206) / SNP Type: INTERGENIC;UNKNOWN / Context (SEQ ID NO: 2118): / GCCAAGTATTGCTTTGTTTTACTTGGTTGTCTGTTTTTTCTTTTAATGCTATTTATTTCAAAATTTTTTATGAGTTAGCATAT GTTAAGGAGAAACTTTA Y

CTATTCCTGAACTGTACTGTCCAATACAGTGGCCATCAGCCACATATAGCTATTGGAGACAGTACTTAAAATGTCACTAGTTC AAGATCAGATATTCTGT / Celera SNP ID: hCV8356425 / Public SNP ID: rs1627765 / SNP Chromosome Position: 197033043 / SNP in Genomic Sequence: SEQ ID NO: 405 / SNP Position Genomic: 34722 / Related Interrogated SNP: hCV2532034 / SNP Source: dbSNP; HapMap; HGBASE / Population(Allele,Count): Caucasian (C,21|T,205) / SNP Type: INTRON / Context (SEQ ID NO: 2119): / AGCCACAGGTTGAAATGTTTTGAGGAAGGGGTTGTGAGCCAAGGAATACAGGTGATTTCAAGAAGCTAGAAAAGGCAAAGTAG CTGATTCTCCTCCAAAG Y

CTCCAGCAGGAATGTGGCTCTCCTGATATCTCGATTTTAGTCCAGTGAAACTGACTCTGGACTTCTGGCCTCTAGGACTGTAG GAGGATAAATCTGTGCT / Celera SNP ID: hCV8356430 / Public SNP ID: rs1759007 / SNP Chromosome Position: 197027606 / SNP in Genomic Sequence: SEQ ID NO: 405 / SNP Position Genomic: 29285 / Related Interrogated SNP: hCV2532034 / SNP Source: dbSNP; HapMap; HGBASE / Population(Allele,Count): Caucasian (T,20|C,206) / SNP Type: INTRON / Context (SEQ ID NO: 2120): / AATCATTGTTATCAGGGCAAGAGAAAGGGGCTTTGTATATCTTGGTAACTATTGAAACTGGAAAGGCTATCAGTACAAATTCA CAACAGTATGATAAAGC R

TAGAATTTTCTCTGTAGGAATTATTATTCCAGACATTAAGAGTCTCTCTAACCTCTCCATACTCTCTTCCCTGCTCTAATCAC TCCAGTCTTTCTGGTCT / Celera SNP ID: hCV8356446 / Public SNP ID: rs1412634 / SNP Chromosome Position: 197023156 / SNP in Genomic Sequence: SEQ ID NO: 405 / SNP Position Genomic: 24835 / Related Interrogated SNP: hCV2532034 / SNP Source: dbSNP; Celera; HapMap; ABI_Val; HGBASE / Population(Allele,Count): Caucasian (A,8|G,110) / SNP Type: INTRON / Context (SEQ ID NO: 2121): / AATCTCACTTCTACTGTGTTCCCCCAAAAAGCACCGAATTTGTTTCCAGGCAGTAGGGGAGCAGGATTGAGAACTTGCCCCAG GCTACTAGCCTCCCAGC Y

GAGAATGCAAGCCAGACTTTTGTGCCTCCCTACCTGTTAAGTCTGCACACCAGATTCACGCCCTTTCCTGAGTTCTGGACAGG AAAGTTTGTGTTCGGTT / Celera SNP ID: hCV11888533 / Public SNP ID: rs1115247 / SNP Chromosome Position: 197037667 / SNP in Genomic Sequence: SEQ ID NO: 405 / SNP Position Genomic: 39346 / Related Interrogated SNP: hCV2532034 / SNP Source: dbSNP; Celera; HapMap / Population(Allele,Count): Caucasian (T,9|C,111) / SNP Type: INTERGENIC;UNKNOWN //

Gene Number: 69 / Gene Symbol: FAT1 - 2195 / Gene Name: FAT tumor suppressor homolog 1 (Drosophila) / Chromosome: 4 / OMIM NUMBER: / OMIM Information: // Genomic Sequence (SEQ ID NO: 406): // / SNP Information / Context (SEQ ID NO: 2122): / CCCAGCTGATCCCTCTGCTCTTCAATCCATCCCTGCAGTACCCTAGTGTCTCACCTCCCAGCTGACTCCTGTGCTCTTCAACC CATTCCTGCGGTATCCT R

GTCTCTCGCCGCCCAGCTGACTCCTCTGCTCTTCAATCCATCCCCGCGGTATCCTAGTGTCTCACTGCCCAGGTGACTCCTCT GCTTTTCAATCCATCCC / Celera SNP ID: hCV2729963 / Public SNP ID: rs13106096 / SNP Chromosome Position: 187552811 / SNP in Genomic Sequence: SEQ ID NO: 406 / SNP Position Genomic: 53874 / SNP Source: dbSNP; Celera; HapMap / Population(Allele,Count): Caucasian (A,106|G,4) / SNP Type: INTRON //

Gene Number: 70 / Gene Symbol: GGT7 - 2686 / Gene Name: gamma-glutamyltransferase 7 / Chromosome: 20 / OMIM NUMBER: / OMIM Information: // Genomic Sequence (SEQ ID NO: 407): // / SNP Information / Context (SEQ ID NO: 2123): / CTTATTTTACAAATGAGAAAACTGAGGCTCAGAGTTGAGACATGGATTTCCCAAGGCCACACAACCAGGAAATGGCAAGACTG GTTCCAATCCCCAGATC

R
TTCACTGCTCCAATAGAAATTTGCCAAATAAAACCAAACTTCACTGGCAGTTGGTGCTGGTTGAGGCATGGGCACCCTTGGGA
GGAGTCCTTTCTCTACC / Celera SNP ID: hCV2521759 / Public SNP ID: rs2076668 /
SNP Chromosome Position: 33437621 / SNP in Genomic Sequence: SEQ ID NO: 407 /
SNP Position Genomic: 15098 / Related Interrogated SNP: hCV1825046 / SNP
Source: dbSNP; Celera; HapMap; ABI_Val; HGBASE / Population(Allele,Count):
Caucasian (G,67|A,53) / SNP Type: UTR3;INTRON /
Context (SEQ ID NO: 2124): /
AGCCCTTTTCTTAGAGGAAAGTCCGTCTCCTCACCCTAAGGTCCACGATCCGGCGCCTGCCTGCCTCTCTGATCTAATTTCAT
CCTTGCTTACCTTGGTC

W
CCTTTTTTAGTCCTCAAACTCACCAAACCCACTCCTGCCCTGAGTTCTTTGTACTTGCTCCCCTGACCTACAAAGCTCTGCACC
AATTCTTTGTATAGTGG / Celera SNP ID: hCV2521760 / Public SNP ID: rs6088624 /
SNP Chromosome Position: 33436852 / SNP in Genomic Sequence: SEQ ID NO: 407 /
SNP Position Genomic: 14329 / Related Interrogated SNP: hCV1825046 / SNP
Source: dbSNP; Celera; HapMap / Population(Allele,Count): Caucasian
(T,62|A,52) / SNP Type: INTRON /
Context (SEQ ID NO: 2125): /
GCTGCTGTACCATTTTACATTTCCACCAACAACGTATGAAGATTCCACTTTTTCACATCCTGAGCAACATTTGTTATTTCCTT
TTTAAAAAATTATTGTT

K
TGGGCGCACAGTGGCTCACACCTGTAATCCCAACACTTTGGGAGGCTGAGGCAGGAGGATTGCTTGAGGCCAGGAGTTTGAGA
CCAACCTGAGCCACATA / Celera SNP ID: hCV2521763 / Public SNP ID: rs12625149 /
SNP Chromosome Position: 33425812 / SNP in Genomic Sequence: SEQ ID NO: 407 /
SNP Position Genomic: 3289 / Related Interrogated SNP: hCV1825046 / SNP
Source: dbSNP; Celera; HapMap / Population(Allele,Count): Caucasian
(G,128|T,90) / SNP Type: INTERGENIC;UNKNOWN /
Context (SEQ ID NO: 2126): /
CTGTTTTCCATAGCTGCTGTACCATTTTACATTTCCACCAACAACGTATGAAGATTCCACTTTTTCACATCCTGAGCAACATT
TGTTATTTCCTTTTTAA

R
AAATTATTGTTGTGGGCGCACAGTGGCTCACACCTGTAATCCCAACACTTTGGGAGGCTGAGGCAGGAGGATTGCTTGAGGCC
AGGAGTTTGAGACCAAC / Celera SNP ID: hCV2521764 / Public SNP ID: rs12626122 /
SNP Chromosome Position: 33425800 / SNP in Genomic Sequence: SEQ ID NO: 407 /
SNP Position Genomic: 3277 / Related Interrogated SNP: hCV1825046 / SNP
Source: dbSNP; Celera / Population(Allele,Count): Caucasian (A,133|G,93) /
SNP Type: INTERGENIC;UNKNOWN /
Context (SEQ ID NO: 2127): /
AAAACATGTCAGGCAGTTCCAATCTCTAGGTCAAGAGAGACAGGCATTCTCTGTTGAGCTGGAAGAGGAAACTAAGGCCCAGG
ACAGGGTAGGTGTGCCT

R
TTGTCGCTTGGTGAGTCAGGGCTGTATTAGGACCAGAAGTCTCACTCCTGACTACTGAATAAAAGGCCTAGCTTCAGGCTGAT
ATCCATCATGACACATC / Celera SNP ID: hCV3249260 / Public SNP ID: rs7263157 /
SNP Chromosome Position: 33459127 / SNP in Genomic Sequence: SEQ ID NO: 407 /
SNP Position Genomic: 36604 / Related Interrogated SNP: hCV1825046 / SNP
Source: dbSNP; Celera; HapMap / Population(Allele,Count): Caucasian
(G,85|A,141) / SNP Type: UTR3;INTRON /
Context (SEQ ID NO: 2128): /
TAATGGAAATTACGCGGTATAAGACTGAACGAGTTACTACGATGTCAAGTTGTTTTATTTATTTTTTTAACTTGGGCTGGGAT
TGTATTAGTAATAGTTC

M
GAAGTTCTGATAGATGGTTCCATGTCTGTTAAAATGTATGAGGAAAATCGGCCAGGAGCAGTGGCTCACGCCTGTAATCCCAG
CACTTTGGGAGGCCGAG / Celera SNP ID: hCV3249262 / Public SNP ID: rs6120750 /
SNP Chromosome Position: 33465289 / SNP in Genomic Sequence: SEQ ID NO: 407 /
SNP Position Genomic: 42766 / Related Interrogated SNP: hCV1825046 / SNP
Source: dbSNP; Celera / Population(Allele,Count): Caucasian (A,134|C,92) /
SNP Type: INTRON /
Context (SEQ ID NO: 2129): /
GTTGGGATTTGAACCAAGACAGTTTGACTGCAGAATCCATATCCTAATAGCAATGCTATTCCTCTTCCATGACCCCACAGAAT
GCATTTCACCAAATTCC

R
TCAAATACAGAATTTCTACCAGGGAAGCAGCCTCCTAATTTGTCCTCTCTTGCCTAGTTTCGCCAACTTGAGTCTTTCCAGTA
GATTTATCAAGACACCA / Celera SNP ID: hCV3249263 / Public SNP ID: rs6088635 /

SNP Chromosome Position: 33466501 / SNP in Genomic Sequence: SEQ ID NO: 407 / SNP Position Genomic: 43978 / Related Interrogated SNP: hCV1825046 / SNP Source: dbSNP; Celera; HapMap; ABI_Val / Population(Allele,Count): Caucasian (A,85|G,141) / SNP Type: INTRON / Context (SEQ ID NO: 2130): /
TATGCCTACTATTATCACTTGAAAATATTTTTGAGTATTTGTACTCTCGTTATTAGCTGCGTGGTCCTTGGCAGAGTTTCCTG
TGCTTCCTGAGCTTCAG
Y
TTTCTCATCTATAAAATTAGGATAATTAATGTATACTTTGTAAGACCATAATGTCATAAAGATTAAATGAGATTAGTCCAGTC
CCTGATACATGAATGGC / Celera SNP ID: hCV3249264 / Public SNP ID: rs6087641 /
SNP Chromosome Position: 33467717 / SNP in Genomic Sequence: SEQ ID NO: 407 / SNP Position Genomic: 45194 / Related Interrogated SNP: hCV1825046 / SNP Source: dbSNP; Celera; HapMap / Population(Allele,Count): Caucasian (T,85|C,141) / SNP Type: INTRON / Context (SEQ ID NO: 2131): /
AATTGTGTAACCTGAAACAAATGACTTAGCTTCTATGAATTTTATTTCTTATGAAATGAAATGAGGGGGTGATAATCTGTAAG
GTTACTTCCATTGTTAC
R
GTTCTAATTGCTTCTCTTTCCTCCAAATTTTTTTTTTTTTTTTTTTTTTTTTTTTGAGATAGAGTCTTGTTCTGTTGCCTAGGC
TGGAGTACAGTGGTGCA / Celera SNP ID: hCV7593321 / Public SNP ID: rs1013677 /
SNP Chromosome Position: 33468793 / SNP in Genomic Sequence: SEQ ID NO: 407 / SNP Position Genomic: 46270 / Related Interrogated SNP: hCV1825046 / SNP Source: dbSNP; Celera; HapMap; HGBASE / Population(Allele,Count): Caucasian (G,85|A,141) / SNP Type: TFBS SYNONYMOUS;INTRON / Context (SEQ ID NO: 2132): /
CTTTTGTTTGGTATTGAGCTTTCTTTTCCTACTCAAAAGGAGGAGCAGATGGGCTTATATAGGTGCTATGCTAGCCTCAAAAA
AATTTAAGAGCCACATG
M
AGTAAGCCAACTCATTGTGCTGATAGGAAAGGTGAGGTGCAGGGAGAGGCACCCAGTGTCACGTTGTGAGTTGGGGGTTGAGCT
GAAATTGACTTAGGATT / Celera SNP ID: hCV11189369 / Public SNP ID: rs6119535 /
SNP Chromosome Position: 33442138 / SNP in Genomic Sequence: SEQ ID NO: 407 / SNP Position Genomic: 19615 / Related Interrogated SNP: hCV1825046 / SNP Source: dbSNP; Celera; HapMap; ABI_Val / Population(Allele,Count): Caucasian (A,133|C,93) / SNP Type: INTRON / Context (SEQ ID NO: 2133): /
TGTGTGTGTGTGTGTGTGTGTGTGTGTTTTAGAGCAAAGTTTGCATTGACCTTAGAAACAATATGTAATGCTCCAGACTTT
TAAAAATTCAACTGAGG
Y
GGGAGGCTGAAGCAGGAGAATTGCTTGAACCTGGGAGGCAGAGGTTACAGTGAGCCAAGATCATGACACTGCACTCCAGCCTG
GGCGACAGAGCGAGACT / Celera SNP ID: hCV29693115 / Public SNP ID: rs6119534 /
SNP Chromosome Position: 33441257 / SNP in Genomic Sequence: SEQ ID NO: 407 / SNP Position Genomic: 18734 / Related Interrogated SNP: hCV1825046 / SNP Source: dbSNP / Population(Allele,Count): Caucasian (C,133|T,93) / SNP Type: INTRON / Context (SEQ ID NO: 2134): /
GAAGAGGAAGTGGTAGAAACTACGGCAAACTGGACAGTGCATGCCCATCATGGGAAGCAGCTTCTATTCCACGCCAGGTAACT
TCAGCCATGTGAGAATG
Y
GGGCCCAAGTTGTTATCGGATTTCCCGATTGTTCAAGAGATGTTGGAAATCTGGATTTTTATGAAAATTCTCAACTTTTTAAC
AAAAGCAAACAAAATTT / Celera SNP ID: hCV29711231 / Public SNP ID: rs6119536 /
SNP Chromosome Position: 33450041 / SNP in Genomic Sequence: SEQ ID NO: 407 / SNP Position Genomic: 27518 / Related Interrogated SNP: hCV1825046 / SNP Source: dbSNP; HapMap / Population(Allele,Count): Caucasian (C,42|T,78) / SNP Type: TRANSCRIPTION FACTOR BINDING SITE;INTRON / Context (SEQ ID NO: 2135): /
CACCCTGGACCAGAGCTGGACCCCTGCCAGCTTCCTGCACCTGTCTACTTGGTATGTCCCTTGCCCCTCCCCCAGGACTGGAT
TATGTAAGGATAGAACA
Y
GCAAAACCAAAAGAGGAATTTCTTTGGTACGGCTTGGAGAGCTGGTCTTACAGGAGCCAAGATAGGAACTTTGTCAGCTTGTT
CCTTTTCTGTAGCTTAT / Celera SNP ID: hCV30090036 / Public SNP ID: rs6120747 /
SNP Chromosome Position: 33449769 / SNP in Genomic Sequence: SEQ ID NO: 407 / SNP Position Genomic: 27246 / Related Interrogated SNP: hCV1825046 / SNP Source: dbSNP; HapMap; ABI_Val / Population(Allele,Count): Caucasian

(T,133|C,93) / SNP Type: INTRON /
Context (SEQ ID NO: 2136): /
GCCTCCATCTCCAAAAAAAAAAAAAGTGGTGGGTGCTAAGATTAGACAGGTAAAGAAGGGACCCAGGTGGTGGGTCCTAGGGCA
AGGAAGTTATAATTTGG
Y
CCTGTGTATAGACTGGTAAGCCTTCTGAGGCTAAATATGTCTCAGAAGATTAATGATATCTGGGCTTCCATTTCTGTTGCAGA
ATTCTGGGGAGACATTG / Celera SNP ID: hCV30558455 / Public SNP ID: rs6088638 /
SNP Chromosome Position: 33470514 / SNP in Genomic Sequence: SEQ ID NO: 407 /
SNP Position Genomic: 47991 / Related Interrogated SNP: hCV1825046 / SNP
Source: dbSNP; HapMap; ABI_Val / Population(Allele,Count): Caucasian
(T,185|C,39) / SNP Type: INTRON //

Gene Number: 71 / Gene Symbol: GOLGA3 - 2802 / Gene Name: golgin A3 /
Chromosome: 12 / OMIM NUMBER: 602581 / OMIM Information: // Genomic Sequence
(SEQ ID NO: 408): // / SNP Information /
Context (SEQ ID NO: 2137): /
AGGACCCTGCATAGATTTCTAGGCACTGGAGCGTCCTCTGGAACGCCCTGTGAGACGGAAGGTGGCACGCACCTGCTCCTTGA
GGCTGTTCACCTTCTCC
Y
TCTCCTTCTCTAGTGAGGCCTGCAGGGCCTGGACAAGATGCTTCATCTGGCGATCCTCCTCTTCTTTGCGCTGCAAAACTGCC
TGCACCTAGATCAGATT / Celera SNP ID: hCV1859855 / Public SNP ID: rs2291260 /
SNP Chromosome Position: 133357412 / SNP in Genomic Sequence: SEQ ID NO: 408 /
SNP Position Genomic: 21917 / SNP Source: dbSNP; Celera; HapMap; HGBASE /
Population(Allele,Count): Caucasian (T,167|C,59) / SNP Type: MISSENSE
MUTATION;ESE;ESE SYNONYMOUS;INTRON /
Context (SEQ ID NO: 2138): /
GCAGATCATCCCACTGCACTCCAGCCTGGGCAACACAGCGAGACTGTTTCAAAAAAAAAAAGAAAGAAAGAAAGAAGCAAATAA
TCAGCATCATTCCTGCA
M
GGAGGGTTCTGATTCAAAACAAAACACAGCAGAATGCATTACCTGGCCGGATTTGATAGCTAATTCTTCTCTTAACTTCTCTA
AAGTTTCCGACGTTTCC / Celera SNP ID: hCV1859872 / Public SNP ID: rs4758905 /
SNP Chromosome Position: 133372395 / SNP in Genomic Sequence: SEQ ID NO: 408 /
SNP Position Genomic: 36900 / Related Interrogated SNP: hCV1859855 / SNP
Source: dbSNP; HapMap / Population(Allele,Count): Caucasian (A,77|C,149) /
SNP Type: INTRON /
Context (SEQ ID NO: 2139): /
TATAAATCTCGCATAGCTAATCAAGAAACTACTGTAAACAAAGGATCCTAGATGTCTTGACTCAGGGTGCCTGGCACATAAAA
ATATTTAAAGATTAAAG
M
AATGTCTGAAGCACAGAAGTGAAGGAAGTTACCAAGGCTGGTCAAAAGCTAATCCTGTGTGCAACGAATTCTCAATCTCATGT
TTGCGTCTATGATCATC / Celera SNP ID: hCV1859912 / Public SNP ID: rs12303977 /
SNP Chromosome Position: 133401184 / SNP in Genomic Sequence: SEQ ID NO: 408 /
SNP Position Genomic: 65689 / Related Interrogated SNP: hCV1859855 / SNP
Source: dbSNP; Celera / Population(Allele,Count): Caucasian (C,149|A,77) /
SNP Type: TRANSCRIPTION FACTOR BINDING SITE;INTRON /
Context (SEQ ID NO: 2140): /
CATCCTGTCTACCGACTGCAGACTGAGGGCCCAGGATGGGCAGCTTGGCCGCCCCCCAACCAGGAGACCGAGGCCAGGCCCAG
CCCAGGCCAGCAACCCC
R
TGAGAAGGACTTGGACCCACTGGTGAGCAGAGCCTCCCCGACTTTCTCGTGTGGCGACAGCTGCCTCCTTTCGGGAGTGAGAC
CCGGACATGCTCTCACA / Celera SNP ID: hCV16174004 / Public SNP ID: rs2242291 /
SNP Chromosome Position: 133373504 / SNP in Genomic Sequence: SEQ ID NO: 408 /
SNP Position Genomic: 38009 / Related Interrogated SNP: hCV1859855 / SNP
Source: dbSNP; HapMap; ABI_Val; HGBASE / Population(Allele,Count): Caucasian
(G,149|A,77) / SNP Type: INTRON /
Context (SEQ ID NO: 2141): /
CCCGGATGCTCCGGCGGAGACGTGGCCGTGAGAGGGGCGGGGCGAGCGGTGCATCGGCCTCGGGTCCCCGGAAACAGCCTGCA
CGCGCTACTTTCGTTCC
R
GAACAGAGAACGTCCTCCTCTTCTTCAAGTGGCCACCCTAGGGCCGGGGACCCTCTGCCCACAGGGTCCGGATCGAGCTCACC
TCCGACTGTGATGAGGC / Celera SNP ID: hCV27522090 / Public SNP ID: rs3825109 /
SNP Chromosome Position: 133405522 / SNP in Genomic Sequence: SEQ ID NO: 408 /
SNP Position Genomic: 70027 / Related Interrogated SNP: hCV1859855 / SNP

Source:    dbSNP; HapMap; HGBASE / Population(Allele,Count):    Caucasian
(G,147|A,75) / SNP Type:    TFBS SYNONYMOUS;INTRON /
Context                (SEQ ID NO: 2142): /
CTCGGCCGGCTTTCTCTTTCCTAGTATTAGAATGTAGCCTCTGTGACGCCAGCTTGTCACCTTCCTCTGTGTCATCCAAGACC
CAGTCTCACAGCCTCAT
R
GGCGGACCGGCTCACATGGAGTCAGCCCTCACAGCGGCTGCACCAGGGACCCCTGGCTCAGGAGATCCCAAGACCTGAGAGAT
GAGCCCACCAAGCGGCC / Celera SNP ID:    hCV27964264 / Public SNP ID:    rs4758939 /
SNP Chromosome Position:    133380349 / SNP in Genomic Sequence:    SEQ ID NO: 408 /
 SNP Position Genomic:    44854 / Related Interrogated SNP:    hCV1859855 / SNP
Source:    dbSNP; HapMap; HGBASE / Population(Allele,Count):    Caucasian
(A,77|G,149) / SNP Type:    INTRON /
Context                (SEQ ID NO: 2143): /
GAAGTGGAGGTTGCAGTGAAACGAGATCTCGCCATTGCACTCCAGCCCGGGCACCCAGAGCAAAACTCCACGTCGAAAAAAAA
AAAAGGTAGAATTTTAG
S
CAGGGAACATGATAAAGACCCATAGATCCCTTCAGGGAAACACTGCTGCTGCCCCACACGTTTAATGGAGTTCTGTTTAGCAG
GAATGTGCCTGATCTAA / Celera SNP ID:    hCV30960162 / Public SNP ID:    rs11147095 /
SNP Chromosome Position:    133398020 / SNP in Genomic Sequence:    SEQ ID NO: 408 /
 SNP Position Genomic:    62525 / Related Interrogated SNP:    hCV1859855 / SNP
Source:    dbSNP; HapMap / Population(Allele,Count):    Caucasian (G,149|C,77) /
SNP Type:    INTRON //

Gene Number:    72 / Gene Symbol:    GSS - 2937 / Gene Name:    glutathione
synthetase / Chromosome:    20 / OMIM NUMBER:    601002 / OMIM Information:
Hemolytic anemia due to glutathione synthetase deficiency, 231900/(3); /
5-oxoprolinuria, 266130 (3) // / Genomic Sequence (SEQ ID NO: 409): // / SNP
Information /
Context                (SEQ ID NO: 2144): /
GGAGGTACCTTTCCTCTATCCCACCAAGTCAGCTGACACAGTAACACCTCACGGCTCTGCAATCTTCCAGTTCCTTGGCTCTG
GCAGCTCCTGGCCCCCC
M
ATGCTTCACTGTCCCCTACCTGGAAAGAGTTTGCTCCAGGAAGGCAGCGTTCTGGCTGACAGCATCCACTAGCAGGTTGAAGT
CCATCTGCACAGCATAG / Celera SNP ID:    hCV1064756 / Public SNP ID:    rs734111 /
SNP Chromosome Position:    33533736 / SNP in Genomic Sequence:    SEQ ID NO: 409 /
SNP Position Genomic:    27500 / Related Interrogated SNP:    hCV1825046 / SNP
Source:    dbSNP; Celera; HapMap; HGBASE / Population(Allele,Count):    Caucasian
(A,85|C,141) / SNP Type:    INTRON /
Context                (SEQ ID NO: 2145): /
AGGATTGCAGGAGCTACACCAAAGAATGGGAAAGTCTAGAAGCAAACGGGAGACAGAACCCAAGAAGAGGAATGAGGCAACTC
TGACTCAACTCCCAAGG
R
GCCCCAGAACCCCAAATTCTTCACACATGTCAGTCCAGTTCTCCACTATCTAACTAGGCCAAGTGCCCCAAGACACCATCCCT
GCCTAGCCCAAGACCAG / Celera SNP ID:    hCV11189332 / Public SNP ID:    rs2273683 /
SNP Chromosome Position:    33509523 / SNP in Genomic Sequence:    SEQ ID NO: 409 /
SNP Position Genomic:    3287 / Related Interrogated SNP:    hCV1825046 / SNP
Source:    dbSNP; Celera; HapMap; HGBASE / Population(Allele,Count):    Caucasian
(G,83|A,141) / SNP Type:    UTR3;INTRON;PSEUDOGENE /
Context                (SEQ ID NO: 2146): /
ACCCATCAACCCTGAGGCCTGGCTATGGTACCACCGGGTGGTAGGTGCCCAGCGCTGCCCCATCGTGGACACCTTCTGGCAAA
CAGAGACAGTGAGTGAA
R
GGTACAGAAGGCTGGGGCCCAGGGACAATGTGGGAAGATTGACTCAAATTTCTCATCTCTGACTTCCCCAGGGTGGCCACATG
TTGACTCCCCTTCCTGG / Celera SNP ID:    hCV1361222 / Public SNP ID:    rs3818273 /
SNP Chromosome Position:    33509275 / SNP in Genomic Sequence:    SEQ ID NO: 409 /
SNP Position Genomic:    3039 / Related Interrogated SNP:    hCV1825046 / SNP
Source:    dbSNP; Celera; HapMap; HGBASE / Population(Allele,Count):    Caucasian
(G,85|A,141) / SNP Type:    UTR3;INTRON /
Context                (SEQ ID NO: 2147): /
AAAAAATGGTCAGCAGTATGCTGGAACTGGCTTATATCAACCAGTGAGAGCTGATTGTTGTTAAACATTTGCCAGCACATCTC
TGGAAACAGTGTAAATG
K
CTGAAGCTCTGTGGATTCCTGAGGATTCAAATTCTAGAAGACTGTGTAGAGTGATAGGTAGCAAAAGGCTTGAGGGAGGATAG

GAGGTTCTAGCAGCAAG / Celera SNP ID: hCV2142575 / Public SNP ID: rs2236270 /
SNP Chromosome Position: 33523155 / SNP in Genomic Sequence: SEQ ID NO: 409 /
SNP Position Genomic: 16919 / Related Interrogated SNP: hCV1825046 / SNP
Source: Applera / Population(Allele,Count): Caucasian (G,6|T,4) African
American (G,4|T,0) total (G,10|T,4) / SNP Type: TFBS SYNONYMOUS;INTRON / SNP
Source: dbSNP; Celera; HapMap; HGBASE / Population(Allele,Count): Caucasian
(G,133|T,93) / SNP Type: TFBS SYNONYMOUS;INTRON /
Context (SEQ ID NO: 2148): /
GGCCAATGGTGCCACCAGTGTTTTGGTGAGAAGGGAGCCACCTGGCCTAGCTGGGGGATGGACAAGATTATCCTGGGTGACTA
CCTCCCAAGGCTGCTTG
K
TCTAGAGGGAGGGGGACTTATACAATCATCTGTTTTCTGGAGAAAGAGAAGTCCTGAGGGGGTTGCGTATCCTGACTAGGATG
GTATCTTGGCTTGTCTG / Celera SNP ID: hCV1361223 / Public SNP ID: rs3746450 /
SNP Chromosome Position: 33508588 / SNP in Genomic Sequence: SEQ ID NO: 409 /
SNP Position Genomic: 2352 / Related Interrogated SNP: hCV1825046 / SNP
Source: dbSNP; Celera; HapMap; HGBASE / Population(Allele,Count): Caucasian
(G,42|T,78) / SNP Type: MISSENSE MUTATION;UTR3;INTRON /
Context (SEQ ID NO: 2149): /
GATAGAGATAAATGCTGTTAAGAAAAATAAACAGGGTAGCATGATAGTGTGTGCAGGGATGGCTACTGTACACAGGATCGTCG
GAGAAGACTTCTCTGAA
K
TGACATTTGAGCAGAGGCCTGAAGGGTGAGAAGGAAACAGGCATGGGAAAATCTAGAGTAAGAGTGTTTTGTGCAGAGGGACA
CAGCTTGAGATGTCTGA / Celera SNP ID: hCV2142560 / Public SNP ID: rs4911449 /
SNP Chromosome Position: 33512236 / SNP in Genomic Sequence: SEQ ID NO: 409 /
SNP Position Genomic: 6000 / Related Interrogated SNP: hCV1825046 / SNP
Source: dbSNP; Celera; HapMap / Population(Allele,Count): Caucasian
(T,85|G,141) / SNP Type: INTRON /
Context (SEQ ID NO: 2150): /
TGTGCAGAGGGACACAGCTTGAGATGTCTGAAGAATGGACTGAAGCCTGTGTGGCTGGAGCGTAGCAAGCAAAGGGAAGAATA
TTACATGACGTTGGAGA
Y
GTACATAATACAGGGAGGGTCTTGTGGGCCATGGTGAGGAGTTTGAATTTTATTCAAAGTACAGTGGGAAACCTTTTTTTTTGT
TGTTGTTGTTGTTGTTG / Celera SNP ID: hCV2142561 / Public SNP ID: rs4911450 /
SNP Chromosome Position: 33512406 / SNP in Genomic Sequence: SEQ ID NO: 409 /
SNP Position Genomic: 6170 / Related Interrogated SNP: hCV1825046 / SNP
Source: dbSNP; Celera; HapMap / Population(Allele,Count): Caucasian
(C,42|T,78) / SNP Type: INTRON /
Context (SEQ ID NO: 2151): /
CGTAGCAAGCAAAGGGAAGAATATTACATGACGTTGGAGACGTACATAATACAGGGAGGGTCTTGTGGGCCATGGTGAGGAGT
TTGAATTTTATTCAAAG
K
ACAGTGGGAAACCTTTTTTTTTGTTGTTGTTGTTGTTGTTGTTTGTTTTTTTTTTTGAGACAGAATCTCACTCTGTCACCCAGGC
TGGAGTGCGGTGGCACG / Celera SNP ID: hCV2142562 / Public SNP ID: rs4911451 /
SNP Chromosome Position: 33512466 / SNP in Genomic Sequence: SEQ ID NO: 409 /
SNP Position Genomic: 6230 / Related Interrogated SNP: hCV1825046 / SNP
Source: dbSNP; Celera; HapMap; HGBASE / Population(Allele,Count): Caucasian
(T,41|G,77) / SNP Type: INTRON /
Context (SEQ ID NO: 2152): /
TCTTATTGTTACCTTCATGCTAAAGATGATGAAACTGAGACTCATGGAAGTTAAATCACTTGCCCAAGGCCACACTGCTAGAA
AGGTGGCATTTAAACTC
Y
AGCAGGCTCATTCTACAGCCTGAGTTCCATAAGATAAAATGAGAGAGTAAAGAGACAAGTGTGTTGAGAGCAAGAGAAGCAGT
GAACCTGAGGCTGTCTC / Celera SNP ID: hCV2142566 / Public SNP ID: rs6088650 /
SNP Chromosome Position: 33514465 / SNP in Genomic Sequence: SEQ ID NO: 409 /
SNP Position Genomic: 8229 / Related Interrogated SNP: hCV1825046 / SNP
Source: dbSNP; Celera; HapMap / Population(Allele,Count): Caucasian
(T,85|C,141) / SNP Type: INTRON /
Context (SEQ ID NO: 2153): /
AAGCCAGTTCACAACTGTTATGTATGTCCCATCTTGGTTGCCTTCCCTCCTGGGCTCCTGCCAGGGACTCTCATTCCTCCCTG
TGATCCAGTAAGAGCAT
Y
AGTGGGTGGCTAAGGATTGAAACTTCAGGGTTCCACCTGTAGAGACTGGTCTAGGGGGACCCTGATTACAGACCAGTCACTTC
CCTGCTCTAATACATCT / Celera SNP ID: hCV2142567 / Public SNP ID: rs725521 /

SNP Chromosome Position: 33516071 / SNP in Genomic Sequence: SEQ ID NO: 409 /
SNP Position Genomic: 9835 / Related Interrogated SNP: hCV1825046 / SNP
Source: dbSNP; Celera; HapMap; ABI_Val; HGBASE / Population(Allele,Count):
Caucasian (T,85|C,141) / SNP Type: INTERGENIC;UNKNOWN /
Context (SEQ ID NO: 2154): /
ATACAAATGTTGGCTCGAAATTACCGAGCAGCAGAATCATACTGCCATGTGTAGTAAAACACACTTTATATTTCCACTGCTTA
AAGCAGCTGAAACCAGA
M
TGAAAAAATTTTTAATGGCAGGGGTGAAGGTGAAAACGCAAACATGTAAAATGATGAGTCTGATTAAGACACAGGCTTTTTAA
ATGTCTTTTCCTTTTCT / Celera SNP ID: hCV2142576 / Public SNP ID: rs2236271 /
SNP Chromosome Position: 33523840 / SNP in Genomic Sequence: SEQ ID NO: 409 /
SNP Position Genomic: 17604 / Related Interrogated SNP: hCV1825046 / SNP
Source: dbSNP; Celera; HGBASE / Population(Allele,Count): Caucasian
(A,85|C,141) / SNP Type: INTRON /
Context (SEQ ID NO: 2155): /
GCTGGGATTAGAGGTGTGAGCCACTGCACCCGGCCTATTATTATTATTGAAAGTACCACATAATGATCCACACAGATTTTTAA
AAACATTTTTCAGATCC
R
TGATCTCATTTAATTCTCACTAACACCCCTTTAGGTATTATTATTACTCCTATTTTACAAATGAGGAAACTGAAGCTCAGAGA
AGAACTAACTTGCCAAG / Celera SNP ID: hCV2142578 / Public SNP ID: rs6088655 /
SNP Chromosome Position: 33527838 / SNP in Genomic Sequence: SEQ ID NO: 409 /
SNP Position Genomic: 21602 / Related Interrogated SNP: hCV1825046 / SNP
Source: dbSNP; Celera; HapMap / Population(Allele,Count): Caucasian
(A,85|G,141) / SNP Type: INTRON /
Context (SEQ ID NO: 2156): /
CAGGGGCTGAGCCTGTATTAGAACCCAGGCTTGTTTCCCAGCCCAGCCAAGTCCCAGAAAAATCCCCCTGATTTTATCTATTT
TTTTTTAACCTGGGATA
S
CAGCAGGGAAAGCATTAGAGGCAAAAGGAAAGGATTTTCATTTTTTCCAGGAATGCTGAAGTCAGGACTGTTTGAGGGGAGCCC
TGTGTTTCTCAGACTCT / Celera SNP ID: hCV2142584 / Public SNP ID: rs3761144 /
SNP Chromosome Position: 33544075 / SNP in Genomic Sequence: SEQ ID NO: 409 /
SNP Position Genomic: 37839 / Related Interrogated SNP: hCV1825046 / SNP
Source: dbSNP; Celera; HapMap; ABI_Val; HGBASE / Population(Allele,Count):
Caucasian (C,45|G,71) / SNP Type: INTRON /
Context (SEQ ID NO: 2157): /
CCATGACTGACCCCCCAACCCAAGAAGTAAAGACTCTCCTGATTTGGTGTTTATTATTCCTATGCAATGTTTATTGAGATATA
ATTCATGCACCATAAAA
Y
TCCCCCTTTAAAGTGTGCAATTCAGTGGTTCTTGGTATATTTACAAGATTGTACAACCATTACCACTGTCTCATTCCAGAATA
TTTTCATCACCACAGAA / Celera SNP ID: hCV2142586 / Public SNP ID: rs6060130 /
SNP Chromosome Position: 33549319 / SNP in Genomic Sequence: SEQ ID NO: 409 /
SNP Position Genomic: 43083 / Related Interrogated SNP: hCV1825046 / SNP
Source: dbSNP; Celera; HapMap / Population(Allele,Count): Caucasian
(C,46|T,74) / SNP Type: INTRON /
Context (SEQ ID NO: 2158): /
ATTGTTTTCAAATATTTTCAAGCTGCAGTTGGTTGAATACACAGATGTGGAACCCATGGATATGGAGGTCCCACTGTACTTGT
CTGATTGTGGCCATCCT
R
GTTGGTCATTGTGGTTTTTGATTTGCATTTTCCTAATGACAAATAATGCGCATATCTTTTCATGTGCTTATTGGTAGTTTGTAT
ATCTTCTTTGGAGAAGG / Celera SNP ID: hCV2142587 / Public SNP ID: rs6088664 /
SNP Chromosome Position: 33551100 / SNP in Genomic Sequence: SEQ ID NO: 409 /
SNP Position Genomic: 44864 / Related Interrogated SNP: hCV1825046 / SNP
Source: dbSNP; Celera; HapMap / Population(Allele,Count): Caucasian
(G,46|A,72) / SNP Type: INTRON /
Context (SEQ ID NO: 2159): /
TTCCTGCTGAATGGAACCTGTCCCCTCAAGCAAAAGTCTCTATGGGAAACCCTATTGGTACCAGTGGTGTAAAGGCAGGAGCA
GAGCTAGGGTAGGAAAC
M
CGCAGATGTGGATTGGGTCTGGACATGTAGGCAAAAGTGCTTGGACCAAGATCTGTTGACTTATTTTACTACTAACAGGGTCA
GGTGCCCACCTCACCTG / Celera SNP ID: hCV3249289 / Public SNP ID: rs2223881 /
SNP Chromosome Position: 33506463 / SNP in Genomic Sequence: SEQ ID NO: 409 /
SNP Position Genomic: 227 / Related Interrogated SNP: hCV1825046 / SNP
Source: dbSNP; Celera; HapMap; HGBASE / Population(Allele,Count): Caucasian

(A,42|C,78) / SNP Type: INTRON /
Context (SEQ ID NO: 2160): /
TGATCAATAATAATAGCAGGGTTGGGGGTAGCAATCTATCAGATACTGTTCAGGTAAAGGCTTTCCAAGAAGGTGACATTTCA
GCTCAGAATTGAAGGAT
R
GGAAGGGACCAGCCAAGGGTTCCTCCTCAGCCTGAGGCCAGGTGAGAGGAGTTCTGGGGTAGAGATGGGGGTAGGATGGCAGG
GTGGTTGGAGTACTCAG / Celera SNP ID: hCV3249290 / Public SNP ID: rs2076667 /
SNP Chromosome Position: 33506964 / SNP in Genomic Sequence: SEQ ID NO: 409 /
SNP Position Genomic: 728 / Related Interrogated SNP: hCV1825046 / SNP
Source: dbSNP; Celera; HapMap; HGBASE / Population(Allele,Count): Caucasian
(G,85|A,141) / SNP Type: INTRON /
Context (SEQ ID NO: 2161): /
CTTCAACCCCACCCCATTTCACTTTCCCAAGAGCAAGTTAGTAACAGGGACAGGGCTTACATCCAGGCCTTCTGACTCAGCCT
AGAATTCCATCCATTCC
R
TCGATCTGCTGCCAAAGAATGTTACTGATTGGGCAATAATTATTTACTTGGTGTGTGGGCACCACTGTTTTTTTAAATGAGTGT
TTTCATTTGTATCAAAC / Celera SNP ID: hCV11189331 / Public SNP ID: rs6087649 /
SNP Chromosome Position: 33510662 / SNP in Genomic Sequence: SEQ ID NO: 409 /
SNP Position Genomic: 4426 / Related Interrogated SNP: hCV1825046 / SNP
Source: dbSNP; Celera / Population(Allele,Count): Caucasian (A,85|G,141) /
SNP Type: UTR5;INTRON /
Context (SEQ ID NO: 2162): /
ATGGACCTGGAAGACCCCTCTGCCCATGACCTCATATATACAGCAACATGACTCTGAGAATCAGCTGAGCACTAAAAAGCATC
CTCCCATCACATTCCTG
K
AAGATCCACTTCAGGTTGGAATATGCTGGGCTGCCCTAACCTTGGCCCCTGGTGTATATGCCTGGAGGAGACCTGAGAGCCTC
TGTCCCCACCCCAACCT / Celera SNP ID: hCV16179579 / Public SNP ID: rs2273684 /
SNP Chromosome Position: 33529766 / SNP in Genomic Sequence: SEQ ID NO: 409 /
SNP Position Genomic: 23530 / Related Interrogated SNP: hCV1825046 / SNP
Source: dbSNP; HapMap; ABI_Val; HGBASE / Population(Allele,Count): Caucasian
(T,118|G,108) / SNP Type: TRANSCRIPTION FACTOR BINDING SITE;INTRON /
Context (SEQ ID NO: 2163): /
TGGGATTACAGGCATGAGCCACCGCACCTGGCTCATTCCTGTTTTATAGATGAGGAATCAGAAGATCAAAGAGACAGAGCAAC
TCTGAGGGTTTGCACAT
M
GGCAGTTGGACTCCAGAACTCTTAGGCATCATGCTGTGTGGTTAACCTGGCTCAGCCGAATCCACTAGAGCAAAGAAAAGAGA
GGCTTGCCCAGGCTTGG / Celera SNP ID: hCV28004288 / Public SNP ID: rs4911455 /
SNP Chromosome Position: 33553062 / SNP in Genomic Sequence: SEQ ID NO: 409 /
SNP Position Genomic: 46826 / Related Interrogated SNP: hCV1825046 / SNP
Source: dbSNP; HGBASE / Population(Allele,Count): Caucasian (A,158|C,68) /
SNP Type: INTRON /
Context (SEQ ID NO: 2164): /
CAATACTTTCCTACCTTCAAACTAGAATATCCAATTTTTTCCTGCGAGTCTCCATACAACCAGAATAGAATTTGTTGTTGTCT
CCCACAAATCTGCTTCT
Y
CTCTGATGAACCCCACCCAGGTACCCAAGCCTTGAGTACTTGTTTCCTACATCTCACTCTCCCCAAATCCTATGGATTTTGCT
GCCTAAGTATATAGTAC / Celera SNP ID: hCV29372803 / Public SNP ID: rs6088659 /
SNP Chromosome Position: 33542605 / SNP in Genomic Sequence: SEQ ID NO: 409 /
SNP Position Genomic: 36369 / Related Interrogated SNP: hCV1825046 / SNP
Source: dbSNP; HapMap / Population(Allele,Count): Caucasian (T,38|C,188) /
SNP Type: INTRON /
Context (SEQ ID NO: 2165): /
ATTTTTCAAGACAAGTTGGGTTGCTAGATTTTTTGTTTTAATTTCATGATTTTTTTTTTTTAAATGTTGGCTCTGACTTTTTAA
AAATACTCTGGTGAAAA
M
AACTGTTAACAATGCTTGCCCCTGGTCAGGGAAACAGTGGCTGGAGGAAGGGGACTTACTCTGTACTGAATACCCTTTTGAAT
TTTGCGCCCTGTTCAAA / Celera SNP ID: hCV30323918 / Public SNP ID: rs6060124 /
SNP Chromosome Position: 33536897 / SNP in Genomic Sequence: SEQ ID NO: 409 /
SNP Position Genomic: 30661 / SNP Source: dbSNP; HapMap /
Population(Allele,Count): Caucasian (C,165|A,59) / SNP Type: INTRON /
Context (SEQ ID NO: 2166): /
TCAGATGAAACTCTGTTTTCTTGCCCCAGTCAGGCTCAAGCCCTGTGGTTGTAGGAATAAAGCCTGTGATCTCAAGAAGTGGT
GACTTGTTTTCTTAGGT

W
TCTGTGCCCTGAGGCTGTATATTCATTAGTGGCTCTGATTTGTGTGGCAGATGGGAAGGGAGGGGTATTTCCTAGGACTATCC
CCAATATCTTCCTTCTA / Celera SNP ID:    hDV70936222 / Public SNP ID:    rs17309872 /
SNP Chromosome Position:    33515788 / SNP in Genomic Sequence:    SEQ ID NO: 409 /
SNP Position Genomic:    9552 / Related Interrogated SNP:    hCV25620145 / SNP
Source:    dbSNP; HapMap / Population(Allele,Count):    Caucasian (A,114|T,6) / SNP
Type:    INTERGENIC;UNKNOWN /
Context        (SEQ ID NO: 2167): /
CTCTTGAGCTCAGGTGATCCACCCGCCCCAGCCTCCCAGAGTGCTAGGATTACAGGCGTTAGCCACTGCGCCCGGCCCCTTTT
GACTCTTTAAATAAACC

R
TGTACATTATCTGACTACAGGACAGGCTGAGTCCTCCAGTCTCCCGTGTGTGGTCACGGGATGGGGGGAGATGGTGGCAGATA
GGTGTCTGGTTATTCCC / Celera SNP ID:    hDV70936327 / Public SNP ID:    rs17310467 /
SNP Chromosome Position:    33545616 / SNP in Genomic Sequence:    SEQ ID NO: 409 /
SNP Position Genomic:    39380 / Related Interrogated SNP:    hCV25620145 / SNP
Source:    dbSNP; HapMap / Population(Allele,Count):    Caucasian (A,203|G,21) /
SNP Type:    INTRON /
Context        (SEQ ID NO: 2168): /
TGAGATCCCACCTCTACAAAAAATTTTTGTAAAATTAAAAAACAAGGGAATTGCCTAAGGATGAGAGGTCACAGTGGCCTAAG
TGACCCTTAGGAATGAC

M
CTTTTTCTGGGACTTTTTCATGATCTTTATATTGGTGTGACATGAACTTTCCTTGTTTAATCTACTGAGTTTGGAGATGGTCT
TAATGATAATAAGAAAA / Celera SNP ID:    hDV70948869 / Public SNP ID:    rs17401737 /
SNP Chromosome Position:    33552642 / SNP in Genomic Sequence:    SEQ ID NO: 409 /
SNP Position Genomic:    46406 / Related Interrogated SNP:    hCV25620145 / SNP
Type:    INTRON /
Context        (SEQ ID NO: 2169): /
ATCACCTAAATAGACATGAGTAGAGGGAAGCTAAACTGTGATACACCCACATCTGGAATACTATGCCTCAGAATGAAGATGAC
CCTAATAGAAGGCATTA

S
AAAGTTCTAAGACAGACAAATAAAAAAACCAAAAACATATCATAGAATAATATATATGATACAATTTGTTTTTTAAAAAAGACG
TATTTCTATATATACCT / Celera SNP ID:    hCV30162176 / Public SNP ID:    rs6060127 /
SNP Chromosome Position:    33538999 / SNP in Genomic Sequence:    SEQ ID NO: 409 /
SNP Position Genomic:    32763 / Related Interrogated SNP:    hCV1825046 / SNP
Source:    dbSNP; HapMap; ABI_Val / Population(Allele,Count):    Caucasian
(G,83|C,37) / SNP Type:    TFBS SYNONYMOUS;INTRON /
Context        (SEQ ID NO: 2170): /
TGGTCAACAAGAGCAAAACTCTGTCTCAAAAAAAAAAAAAAAGTCTACCTCTTTAAACACTACTTTCCTTCCTTGTTATCTCAA
ACTCTTCTAAGTTTTCT

Y
TGTCGTGTTTTGAAATCTAGACCCATTTCTTTTTTTTTTTTTTTTGAGACAGAGTCTCGCTCTGTCAGCCAGGCTGGAGTGCAGT
GGCATGATCTCAACTTA / Celera SNP ID:    hCV30485907 / Public SNP ID:    rs6087653 /
SNP Chromosome Position:    33522054 / SNP in Genomic Sequence:    SEQ ID NO: 409 /
SNP Position Genomic:    15818 / Related Interrogated SNP:    hCV1825046 / SNP
Source:    dbSNP; HapMap; ABI_Val / Population(Allele,Count):    Caucasian
(T,85|C,141) / SNP Type:    INTRON /
Context        (SEQ ID NO: 2171): /
GTCAGGTGTCTTCAGTGACCAGGATATGTTGGCCTCTGTCCCCCTTCCAGGACTTGGCACGTGCTGTCTTCTCTTTGGAACTC
TCGCCACCACTCCTGAT

Y
TGGGCTCGCGATTTCGTTCCGGAAGCTGTCCCTGTTCTCCGCCCCGGGCTTCTACATTCTACAGCCCATCACTTTTCCCTGTA
CCGTAGGAACAGTGTTT / Celera SNP ID:    hCV30053852 / Public SNP ID:    rs6088660 /
SNP Chromosome Position:    33542896 / SNP in Genomic Sequence:    SEQ ID NO: 409 /
SNP Position Genomic:    36660 / Related Interrogated SNP:    hCV1825046 / SNP
Source:    dbSNP / Population(Allele,Count):    Caucasian (C,159|T,67) / SNP Type:
INTRON;PSEUDOGENE /
Context        (SEQ ID NO: 2172): /
AACATCCAAGGATACTTAAGTCCCTTATATAAAATGATGCAGTGTTTCATATATGCTTAAGTCCCTTATATAAAATGGTGTAG
TGTTTCATATAAACTAT

M
TGCATCCTCTCATATGCTCTTTAATTTTTGTTTTTATTTTTATTTTAAATTTAAATTTATTTTTATTTATTTTGAGACAAGGTCTC
ACTCTGTCAGGCTGGAG / Celera SNP ID:    hCV30503905 / Public SNP ID:    rs4911165 /

SNP Chromosome Position: 33550382 / SNP in Genomic Sequence: SEQ ID NO: 409 / SNP Position Genomic: 44146 / Related Interrogated SNP: hCV1825046 / SNP Source: dbSNP; HapMap; ABI_Val; HGBASE / Population(Allele,Count): Caucasian (A,82|C,38) / SNP Type: INTRON //

Gene Number: 73 / Gene Symbol: MET - 4233 / Gene Name: met proto-oncogene (hepatocyte growth factor receptor) / Chromosome: 7 / OMIM NUMBER: 164860 / OMIM Information: Renal cell carcinoma, papillary, familial and sporadic, 605074 (3);/He / patocellular carcinoma, childhood type, 114550 (3) // / Genomic Sequence (SEQ ID NO: 410): // / SNP Information /
Context (SEQ ID NO: 2173): /
AAATCCTATAAACAGCATGTGATCATATTGCTTCTAGAATTTATGATTGTTTTCTTCCAAAAGGAAGCTAAATTTAGCCTAGT
AATTCTACATGCGCTCA
R
GAAAACAATGCCTGCTGTGATTTCTAGAATAAATGAATGTGAACCACAGTTCCTTTACTTGACTAACAGAGAAAGTTTAAATA
TCAACCTAGTCATTAAC / Celera SNP ID: hCV16170613 / Public SNP ID: rs2237712 /
SNP Chromosome Position: 116338658 / SNP in Genomic Sequence: SEQ ID NO: 410 /
SNP Position Genomic: 36199 / SNP Source: Applera /
Population(Allele,Count): Caucasian (A,32|G,6) African American (A,31|G,3)
total (A,63|G,9) / SNP Type: INTRON / SNP Source: dbSNP; HapMap; ABI_Val;
HGBASE / Population(Allele,Count): Caucasian (A,215|G,11) / SNP Type: INTRON
//

Gene Number: 74 / Gene Symbol: MTHFR - 4524 / Gene Name:
methylenetetrahydrofolate reductase (NAD(P)H) / Chromosome: 1 / OMIM NUMBER:
607093 / OMIM Information: Homocystinuria due to MTHFR deficiency, 236250 (3) //
Genomic Sequence (SEQ ID NO: 411): // / SNP Information /
Context (SEQ ID NO: 2174): /
CCCGGGGACGATGGGGCAAGTGATGCCCATGTCGGTGCATGCCTTCACAAAGCGGAAGAATGTGTCAGCCTCAAAGAAAAGCT
GCGTGATGATGAAATCG
R
CTCCCGCAGACACCTTCTCCTTCAAGTGCTTCAGGTCAGCCTCAAAGCTCCCTGCTTCGGGGTGGCCTTTGGGGTAACCTGCC
AATAGGGATGACAGTCA / Celera SNP ID: hCV1202883 / Public SNP ID: rs1801133 /
SNP Chromosome Position: 11856378 / SNP in Genomic Sequence: SEQ ID NO: 411 /
SNP Position Genomic: 20591 / SNP Source: Applera / Population(Allele,Count):
Caucasian (A,14|G,24) African American (A,8|G,30) total (A,22|G,54) / SNP
Type: MISSENSE MUTATION;ESE;UTR5 / SNP Source: HGMD; dbSNP; Celera; HapMap;
ABI_Val; HGBASE / Population(Allele,Count): Caucasian (G,156|A,70) / SNP Type:
MISSENSE MUTATION;ESE;UTR5 //

Gene Number: 75 / Gene Symbol: MTNR1A - 4543 / Gene Name: melatonin
receptor 1A / Chromosome: 4 / OMIM NUMBER: 600665 / OMIM Information: //
Genomic Sequence (SEQ ID NO: 412): // / SNP Information /
Context (SEQ ID NO: 2175): /
GCGTTGCAGGCTCGTGAACAAGAACAGGAAGCGTGCTCGCTTCGGCAGCGCATGGACTAACATTGGAACGGTACAGGGAAGAT
CAGCAGGGCCCCTGTGC
M
AGGACGGCACGCGAATTCCTGAAGTGTTCCAAATAAATAAATGAGTAAGAACAGGAAGTGGTACTGGGCGTTTCAGGAATAAT
AACGAGCAGCGGGGAAC / Celera SNP ID: hCV1332964 / Public SNP ID: rs10013543 /
SNP Chromosome Position: 187422331 / SNP in Genomic Sequence: SEQ ID NO: 412 /
SNP Position Genomic: 65127 / SNP Source: dbSNP; Celera /
Population(Allele,Count): Caucasian (C,117|A,109) / SNP Type:
INTRON;PSEUDOGENE /
Context (SEQ ID NO: 2176): /
GAAGGTAACACGGCGAAGCCCCGCCCAGCTGGGATGGAAGGCGCCGTTGGAAGGAACGTGAGGCACAGTTGGAAGCAGGCAAC
GGTGGGCGTTGCAGGCT
S
GTGAACAAGAACAGGAAGCGTGCTCGCTTCGGCAGCGCATGGACTAACATTGGAACGGTACAGGGAAGATCAGCAGGGCCCCT
GTGCCAGGACGGCACGC / Celera SNP ID: hCV1332965 / Public SNP ID: rs4861717 /
SNP Chromosome Position: 187422243 / SNP in Genomic Sequence: SEQ ID NO: 412 /
SNP Position Genomic: 65039 / SNP Source: dbSNP; Celera; HapMap; HGBASE /
Population(Allele,Count): Caucasian (C,75|G,45) / SNP Type: INTRON /
Context (SEQ ID NO: 2177): /

TTTATGTTATCTCTCCTTCCTAGCTTTTTTTTTTTTTTTTTTTTTTTTTTTGGCTTAGGCAGCTAGGAAGCCCAGAGCTCTA
TCTTCACCTGAAGCCAC
R
AGCATTTGAGGAATTATGTGCTCAATATTGTTAGATGCCGCATATGCACAGAAATAGAGGAAGTTACACGGGTTTCAGCCTCG
TTGAAGAAAAGCATCGA / Celera SNP ID: hCV1332967 / Public SNP ID: rs6553000 /
SNP Chromosome Position: 187421850 / SNP in Genomic Sequence: SEQ ID NO: 412 /
SNP Position Genomic: 64646 / SNP Source: dbSNP; Celera; HapMap /
Population(Allele,Count): Caucasian (G,30|A,90) / SNP Type: INTRON /
Context (SEQ ID NO: 2178): /
AGCCACTGCGCCCAGGCTCAAAATAGTCCTTCTTTTAGCTTTTATGTTATCTCTCCTTCCTAGCTTTTTTTTTTTTTTTTTTTT
TTTTTTTTTGGCTTAGG
Y
AGCTAGGAAGCCCAGAGCTCTATCTTCACCTGAAGCCACGAGCATTTGAGGAATTATGTGCTCAATATTGTTAGATGCCGCAT
ATGCACAGAAATAGAGG / Celera SNP ID: hCV1332968 / Public SNP ID: rs11729609 /
SNP Chromosome Position: 187421810 / SNP in Genomic Sequence: SEQ ID NO: 412 /
SNP Position Genomic: 64606 / SNP Source: dbSNP; Celera; HapMap /
Population(Allele,Count): Caucasian (C,192|T,34) / SNP Type: INTRON /
Context (SEQ ID NO: 2179): /
AAGTGCTGGGATTATAGGTATGAGCCACTGTGCCTAGCCAGACAGTTTTTTTTAAAAGAAATTCATCTTCACATTGTGACTATC
AATGTATCATACTAATA
Y
CTGAAAAGCTCATACAGATTCAGAGTTCAACCACCTCACTCCCCTAACCGCACCCCCCTCCCCCCACCCGCCAAAAAAAAAGAA
TAAAAAAAGAACAGTGG / Celera SNP ID: hCV1332991 / Public SNP ID: rs11723770 /
SNP Chromosome Position: 187387204 / SNP in Genomic Sequence: SEQ ID NO: 412 /
SNP Position Genomic: 30000 / Related Interrogated SNP: hCV22272267 / SNP
Source: dbSNP; Celera; HapMap / Population(Allele,Count): Caucasian
(T,149|C,77) / SNP Type: INTRON /
Context (SEQ ID NO: 2180): /
CAGTACTTCTGAAAAACATTCTCCATTCTTCAAGCAAGCTTGTACTGGGCATGCACTGTTCTCAGGGCTGCCAGTATAAAGTG
TAGGGGAGAAAAACATT
M
TCTGACTTTCTACCCATCTTAGTTTCATGGGTTAGGGACTGCCAATTAGACAGACAAAAGATAGATTAGCAAGAGATAAACAA
ACAGAAATTCATTAACG / Celera SNP ID: hCV1332992 / Public SNP ID: rs12506228 /
SNP Chromosome Position: 187382294 / SNP in Genomic Sequence: SEQ ID NO: 412 /
SNP Position Genomic: 25090 / Related Interrogated SNP: hCV22272267 / SNP
Source: dbSNP; Celera; HapMap / Population(Allele,Count): Caucasian
(C,159|A,67) / SNP Type: INTRON /
Context (SEQ ID NO: 2181): /
AGTAACCCTCCCTCCCCTGAGCTGTGAAAACCAAAAATGTCTTCAGATACTGCCAAATATCTCTTTGGGGACAAAATCATCTC
GTTGAGATATCATTCTC
K
ATGTCTATCATAGAAATAGAATGACTAAAAAGAAGAAATAATAGAAAAATGTGGCATTACTAAAAACAATCGTAGTATGTCCG
TCTCTGTACATTTTGAG / Celera SNP ID: hCV11791555 / Public SNP ID: rs2001362 /
SNP Chromosome Position: 187421298 / SNP in Genomic Sequence: SEQ ID NO: 412 /
SNP Position Genomic: 64094 / SNP Source: dbSNP; Celera; HapMap; HGBASE /
Population(Allele,Count): Caucasian (T,46|G,170) / SNP Type: INTRON /
Context (SEQ ID NO: 2182): /
ACTTTATTTTTTTTTTTATGATTGTGGTCCCATCAATTTAAATTTTCCTTTACACTTTCAAACAAGGTGGCCACACACTCGACT
CCATTGTTCTTCTGGGG
K
TAGACAAGTGTGAGGTGACCCCAGTTAGGGATGCCCCAACAGGCATTAAGTTGACAGCACAAGAGCTTCCATCGCAACCCTCT
AGACACTGCAACCGCCA / Celera SNP ID: hCV12065198 / Public SNP ID: rs1946979 /
SNP Chromosome Position: 187449285 / SNP in Genomic Sequence: SEQ ID NO: 412 /
SNP Position Genomic: 92081 / SNP Source: dbSNP; HapMap; HGBASE /
Population(Allele,Count): Caucasian (T,4|G,116) / SNP Type: INTRON /
Context (SEQ ID NO: 2183): /
CTTACTCTCACCCCAGCCCTGAACCGCATTCCTCAGTTAACATGTAACTCTCCATACATTCTGTACTTCTCTTGACTAGTAGA
ATTGGAGGGGTTTATAA
Y
GCTGACAGCACGTTAGCTCAGAAACTATGGCAGCAGCCAGGTAAAAGTTGGAAGTTTCCTCCACCACTCTGCTTTCCCAGACA
TCTGTCAAGAGCGAGGC / Celera SNP ID: hCV16148229 / Public SNP ID: rs2165668 /
SNP Chromosome Position: 187454701 / SNP in Genomic Sequence: SEQ ID NO: 412 /
SNP Position Genomic: 97497 / SNP Source: dbSNP; HapMap; ABI_Val; HGBASE /

Population(Allele,Count): Caucasian (T,4|C,114) / SNP Type: UTR3;INTRON /
Context (SEQ ID NO: 2184): /
GTTGTAAATAATCTTCTGGCAGCCAAACCCCTTTTTCGTTCCCTCATTAAGGAGGGTTTATACTTAGGAAGCAGCCACCACTT
CCACCACTGAGCTGGCA
Y

TGGCGATACGACAATGAACAAGATACAAATGGCCTTTACCCTTATGGAGCACGCAGTCCGTGGTAAAGAAAGCAAGAAGATAA
TCAATTACAGACCAACC / Celera SNP ID: hCV27309972 / Public SNP ID: rs13101296 /
SNP Chromosome Position: 187412671 / SNP in Genomic Sequence: SEQ ID NO: 412 /
SNP Position Genomic: 55467 / Related Interrogated SNP: hCV27477533 /
Related Interrogated SNP: hCV3230038 / Related Interrogated SNP: hCV8241630 /
SNP Source: dbSNP; Celera; HapMap / Population(Allele,Count): Caucasian
(T,98|C,22) / SNP Type: INTRON /
Context (SEQ ID NO: 2185): /
TGGAGGCCGTTTCCCAGGAGGGCCAGTCCACTGCCGCTCCTGACACATCGAATCCGCTGTTCACACATTCCCACCTCCCCAGA
ACAGGGCCTGTTCCATG
Y

TCAAAACTGCTGTCTTTCAAACCGTTCGGCTTCTCATCTCTTAAAAAGTCCTGAGATCAGAATGCTTGCACACCATCTGTTGT
TCTGGAGCCTTCTACAT / Celera SNP ID: hCV29099171 / Public SNP ID: rs6553008 /
SNP Chromosome Position: 187455946 / SNP in Genomic Sequence: SEQ ID NO: 412 /
SNP Position Genomic: 98742 / SNP Source: dbSNP; HapMap /
Population(Allele,Count): Caucasian (T,5|C,221) / SNP Type: INTRON /
Context (SEQ ID NO: 2186): /
AAAATTAACATACAGAGATCAATTTTAATTTCCTGATGAGGCCAACAGTAAAAAAAATTAAATGTTAAATAGACACACTCAAGA
GCAGCAAAAACAAAGGC
R

TGTAATTAGAAATAATCTAATAGAAGATAGGGAGGAAATTATACTTTATTGAGTGACATTAAAGAAAAGTTCATAAATCAAGA
GACATGCTTCTTCATGG / Celera SNP ID: hCV29316369 / Public SNP ID: rs6830298 /
SNP Chromosome Position: 187433342 / SNP in Genomic Sequence: SEQ ID NO: 412 /
SNP Position Genomic: 76138 / SNP Source: dbSNP; HapMap /
Population(Allele,Count): Caucasian (A,92|G,22) / SNP Type: INTRON /
Context (SEQ ID NO: 2187): /
AGGAACTCAGCTACAGGCGATGCCCGATCAGGTTGAGGTTTGGAATGGGCCCTGGATACTGGCATTTTTAAAACTCTTCCCAG
GTGGCCGGGCATGGTGG
Y

TCTTGTAATCCCAGCACTTTGGGAGGCTGAAATGGGAGGATGGCTGGAGGCCAGGAGTTTGAGAACAGCCTGAGCAACATAGC
AAGATCTCGTCTCAATA / Celera SNP ID: hCV29419315 / Public SNP ID: rs6841024 /
SNP Chromosome Position: 187416939 / SNP in Genomic Sequence: SEQ ID NO: 412 /
SNP Position Genomic: 59735 / Related Interrogated SNP: hCV12066124 / SNP
Source: dbSNP; HapMap / Population(Allele,Count): Caucasian (C,96|T,12) / SNP
Type: INTRON /
Context (SEQ ID NO: 2188): /
GGTTTATTTTGCCAAGGTTGAGGAAGCACCTGGCAAACAGAGACACAAGCCACTGTAGGATCTGTGGCACCCCCTTTTTCTGA
AGAGGATTTTGAGGGCT
W

CAATATGAAAAGGGGAAAAGGAGGCAGGAGGGGAAAAGAAGGAAAAGGAGAGGGAGGGCTGGTCACGTTCTTTAGAATCCACA
TGAAAAGGAGCGGGTAG / Celera SNP ID: hCV32209660 / Public SNP ID: rs12498245 /
SNP Chromosome Position: 187392009 / SNP in Genomic Sequence: SEQ ID NO: 412 /
SNP Position Genomic: 34805 / SNP Source: dbSNP / Population(Allele,Count):
Caucasian (A,36|T,190) / SNP Type: INTRON /
Context (SEQ ID NO: 2189): /
ATAGTGAACCTTGTATCTCAGAATGTAAGTTATAGGATATCAAATGGGGAATGTGACTCAACTAGAATAATAAAAATGATGTA
AAGTTGCATAAAGAGAC
Y

GTATTTTCTTTTACAAAGAGGGTTAATGTATTTTCAATTTTACACGGGATAGTTGCTTTGTTAGATGGAAGCATAATTTTGAT
GTTTCCTTTATTTCGAA / Celera SNP ID: hCV29781189 / Public SNP ID: rs6820205 /
SNP Chromosome Position: 187464867 / SNP in Genomic Sequence: SEQ ID NO: 412 /
SNP Position Genomic: 107663 / SNP Source: dbSNP; HapMap; ABI_Val /
Population(Allele,Count): Caucasian (T,5|C,115) / SNP Type: INTRON /
Context (SEQ ID NO: 2190): /
AACTCCCCAAACTATAATACCCTCTTCATGTGACACGCCATCCACATCACACCCCATTAACGGGATATGCTGTCCACACCACA
CCCTGTTCATGAGACAC
K

CTGCTCACCTTGCACCCTGTTCATACAACAACACACCATCCGCCTCGCTCCCTGTTCATGCCACCCACATCACACCCTGTTCA

TGCCACCCACACCACAC / Celera SNP ID: hCV31861423 / Public SNP ID: rs13128153 / SNP Chromosome Position: 187467748 / SNP in Genomic Sequence: SEQ ID NO: 412 / SNP Position Genomic: 110544 / SNP Source: dbSNP; HapMap / Population(Allele,Count): Caucasian (G,4|T,116) / SNP Type: INTRON / Context (SEQ ID NO: 2191): / GACAGATCTTAAACAGGATGTAAAGCCGGCAGTCTTGCTGAACAGGTCATATTGTGGCTGACTCTGACACAGCATCCTTATCT TTTTTTCCTTTTTTTAT

K

CTCCACAGGGCAAGTCCTAAGCATCCCTATCTTAACTTCAACAGTCCATTCTGCTAACTCCAAATTTTTAGACAAAGTTTTGT TCCTTTTCACCAACTACA / Celera SNP ID: hDV71381398 / Public SNP ID: rs10866292 / SNP Chromosome Position: 187404608 / SNP in Genomic Sequence: SEQ ID NO: 412 / SNP Position Genomic: 47404 / SNP Source: dbSNP; HapMap / Population(Allele,Count): Caucasian (G,46|T,180) / SNP Type: INTRON //

Gene Number: 76 / Gene Symbol: MYO7B - 4648 / Gene Name: myosin VIIB / Chromosome: 2 / OMIM NUMBER: 606541 / OMIM Information: // Genomic Sequence (SEQ ID NO: 413): // / SNP Information / Context (SEQ ID NO: 2192): / GCATTGCCCATTTTGCCGGCGAGGTGTACTACCAAGCAGAAGGTGGGTGCAGCTCTCCTCTCATGTCCCTTCCAAATCTGGAC CGGGTTCCAGGGAGACC

R

TGGAAAGCAGGCTCAGAGGATGAGGCTATTTTGCAGCTCTAGCAATGGGGCAGAGGGATGAGTAGTATGGCTTCTACTGGAGA AAGAAAATGGAGTGGAC / Celera SNP ID: hCV25971425 / Public SNP ID: rs4662741 / SNP Chromosome Position: 128342547 / SNP in Genomic Sequence: SEQ ID NO: 413 / SNP Position Genomic: 59169 / Related Interrogated SNP: hCV1841983 / SNP Source: Applera / Population(Allele,Count): Caucasian (A,31|G,1) African American (A,32|G,0) total (A,63|G,1) / SNP Type: INTRON / SNP Source: dbSNP; HapMap; ABI_Val; HGBASE / Population(Allele,Count): Caucasian (G,17|A,103) / SNP Type: INTRON / Context (SEQ ID NO: 2193): / AAGGTGGGCTCCCAGGCACCCTCCTGGGTCTGTCACCCCTGATGGCTACAGGGCCAGACAGACATGTACAGGAAAGACTGTTT TACTCACCTCTGGGTCC

M

CAGCATGCAGTTGGCACTTGGGAATCCTGCAATGGATGGATGGATGGGTGAATGGATGGGTGGGTGGATAGATGGGTGAGTGG GTGGATGGGTAGTGGGT / Celera SNP ID: hCV25993019 / Public SNP ID: rs11673952 / SNP Chromosome Position: 128346228 / SNP in Genomic Sequence: SEQ ID NO: 413 / SNP Position Genomic: 62850 / Related Interrogated SNP: hCV1841983 / SNP Source: Applera / Population(Allele,Count): Caucasian (A,3|C,27) African American (A,2|C,18) total (A,5|C,45) / SNP Type: INTRON / SNP Source: dbSNP; HapMap / Population(Allele,Count): Caucasian (A,17|C,103) / SNP Type: INTRON / Context (SEQ ID NO: 2194): / CATTCTCAAAGCAATGGGAAGGGATAGACTTTTCATAAACTGTTCTAGAGCAATGGGGTGTCCATATAGAAAACAAAAGCTTG GGCCACCATCTCACGCC

R

TGTGAGAATATTGCTTCCAGGCAAGCTGCAGCTGGGCTGATCTGTCGTCTATCAGCCAGACTGCACACTTCGGCCAGGGAAAG GTGGGTGCTAACCAGAG / Celera SNP ID: hCV1023669 / Public SNP ID: rs1075774 / SNP Chromosome Position: 128336957 / SNP in Genomic Sequence: SEQ ID NO: 413 / SNP Position Genomic: 53579 / Related Interrogated SNP: hCV1841983 / SNP Source: dbSNP; HapMap; HGBASE / Population(Allele,Count): Caucasian (A,16|G,100) / SNP Type: INTRON / Context (SEQ ID NO: 2195): / CTAGAGAAGTTCTGCTTATTTTGCTCAAGAAAAGAAGCAGCCAAGCTTGAAAATTTAGACAGCGAATGGGAAACTTTAAAGAC ATTATAGATTTATAAAC

R

AACCACATAGAAATTCCATTCTAAAATTAAAAAAAAAAAATAACCAAAAGGAAGAACCCAATGGATGGTTTAAGGGATTCTGTG CCTGTCTAGGAGATGGA / Celera SNP ID: hCV8808000 / Public SNP ID: rs891514 / SNP Chromosome Position: 128336551 / SNP in Genomic Sequence: SEQ ID NO: 413 / SNP Position Genomic: 53173 / Related Interrogated SNP: hCV1841983 / SNP Source: dbSNP; HapMap / Population(Allele,Count): Caucasian (A,17|G,103) / SNP Type: INTRON / Context (SEQ ID NO: 2196): / GATACAAGGTGGGCTGAAAAGGTAGGCAGGCTATGATGCTGAAGATCCTTGAGTGTCATAAAGATATGGCACATTCTTAGCTG

TGGGAGGCATGACACGG
Y
CAACTTTTGCATCTTCTAAAGATCACTTGATTGGTGTGATGGAGGATGATTGTTCTGAGAGTCCAGCAGCAGCAAGGCCACCT
GGGATGCTTCCAACCTC / Celera SNP ID: hCV8837013 / Public SNP ID: rs1019842 /
SNP Chromosome Position: 128305196 / SNP in Genomic Sequence: SEQ ID NO: 413 /
SNP Position Genomic: 21818 / Related Interrogated SNP: hCV1841983 / SNP
Source: dbSNP; HapMap; ABI_val; HGBASE / Population(Allele,Count): Caucasian
(T,38|C,186) / SNP Type: INTRON /
Context (SEQ ID NO: 2197): /
AGGCTCTCCAAGAGCAGGGTCTCTTCCCCGGGGCTGCCTTGCCGTCGCTGACTGCCTCCTAAGGAAGCTGGATTTCTCTAGCC
AAATTGTGTCATGTTGT
M
GGATTTTTGGCAGTGGGTTGGCTGGCCCTGTGCCCACTGGGAGAAGACAGGATGGGAGGACTGTAACTTCTCATTCTTACCCTG
GGCTTGCCTGGGCAGAA / Celera SNP ID: hCV12047693 / Public SNP ID: rs1864552 /
SNP Chromosome Position: 128299527 / SNP in Genomic Sequence: SEQ ID NO: 413 /
SNP Position Genomic: 16149 / Related Interrogated SNP: hCV1841983 / SNP
Source: dbSNP; HapMap; HGBASE / Population(Allele,Count): Caucasian
(C,39|A,185) / SNP Type: INTRON /
Context (SEQ ID NO: 2198): /
TGTAAACCAAGCATTTTAGTGCAAAGCAGTGAACGTCACTACAAAGTGCTGTACAGGATCCACTGGTAGCAGAGGGTGTCAGA
CTTCAGTTCTGATTGAG
R
GGAATAGCATCAATGAGTCTGAAGGAAACAGTTGAACCAGGTCTAAAACTGCAGGCTGGACTAGAAGCTAAATAGGCCGACAC
TTGCCCATACCTACACG / Celera SNP ID: hCV27271075 / Public SNP ID: rs2163348 /
SNP Chromosome Position: 128299079 / SNP in Genomic Sequence: SEQ ID NO: 413 /
SNP Position Genomic: 15701 / Related Interrogated SNP: hCV1841983 / SNP
Source: dbSNP; HapMap; ABI_val; HGBASE / Population(Allele,Count): Caucasian
(A,39|G,187) / SNP Type: INTRON /
Context (SEQ ID NO: 2199): /
CTTTTAGCTGGGACCCAAAAGGGCTTCCCCCTTAAGGTTCAGGTAAGCCAGAGTAAACAAATCCACGTGTGAACTTGCAAACC
AGGTTGTGAGCAAGGGG
Y
GGTCTGGGGATTAAGTTGTCCTGACCTGTGAACACCCCCAAGCACCTAACGGCAGTTATTGACAGTCCTCGCTGGAAAAAAGC
ACCAGCAAGTTAGATTA / Celera SNP ID: hCV29404615 / Public SNP ID: rs6709113 /
SNP Chromosome Position: 128336189 / SNP in Genomic Sequence: SEQ ID NO: 413 /
SNP Position Genomic: 52811 / Related Interrogated SNP: hCV1841983 / SNP
Source: dbSNP; HapMap / Population(Allele,Count): Caucasian (T,39|C,187) /
SNP Type: INTRON /
Context (SEQ ID NO: 2200): /
AAACCAGGTTGTGAGCAAGGGGTGGTCTGGGGATTAAGTTGTCCTGACCTGTGAACACCCCCAAGCACCTAACGGCAGTTATT
GACAGTCCTCGCTGGAA
R
AAAGCACCAGCAAGTTAGATTACAAATGATTCCTGTGAGTAGTTTTTCAAACACAACATCCAGCATGAAGCCAGGAAGAAATA
GGAACAAGGAAATGAGA / Celera SNP ID: hCV29404616 / Public SNP ID: rs6706077 /
SNP Chromosome Position: 128336267 / SNP in Genomic Sequence: SEQ ID NO: 413 /
SNP Position Genomic: 52889 / Related Interrogated SNP: hCV1841983 / SNP
Source: dbSNP; HapMap / Population(Allele,Count): Caucasian (A,17|G,103) /
SNP Type: INTRON /
Context (SEQ ID NO: 2201): /
CACTTGAGCCCAGGAGGTCAAGGCTGCAGTGAACTATGATCACACCACTGCACTTCAGCCTGGGTGACAGAGCAAGACCTGTC
TCTAAAAAAATAAAATG
R
GGCTTGCCTTATGAAATCTAGGTTGCATCTTCCTTGAAGGCAAAATAGGCAGATGTGAGATAACCTATCTTGTTCCTTAACTG
CTGGGAGAAGTAGCTGG / Celera SNP ID: hCV29404621 / Public SNP ID: rs7600934 /
SNP Chromosome Position: 128348778 / SNP in Genomic Sequence: SEQ ID NO: 413 /
SNP Position Genomic: 65400 / Related Interrogated SNP: hCV1841983 / SNP
Source: dbSNP; HapMap / Population(Allele,Count): Caucasian (A,17|G,103) /
SNP Type: INTRON /
Context (SEQ ID NO: 2202): /
GCAAAGAGCCCAGCACAGAGAAAATCCTTATCACATGCCAGCTCTTTCTGAGACTGAGAATTCAAAAATGTTCTAAAGAGTAG
TTCAGTCGCAGAGGTCT
R
TGCTGCCAACCTTGCTGTTTCATAGGGTTAATGGATAATTTGGCTGCTGTAGCCTAGATGGGAATATTAGGGCCACCATAAGC

AAGTGACTTCAGTTAGC / Celera SNP ID: hCV29404688 / Public SNP ID: rs7582598 / SNP Chromosome Position: 128289856 / SNP in Genomic Sequence: SEQ ID NO: 413 / SNP Position Genomic: 6478 / Related Interrogated SNP: hCV1841983 / SNP Source: dbSNP; HapMap; ABI_val / Population(Allele,Count): Caucasian (G,38|A,188) / SNP Type: INTERGENIC;UNKNOWN //

Gene Number: 77 / Gene Symbol: NAGLU - 4669 / Gene Name: N-acetylglucosaminidase, alpha / Chromosome: 17 / OMIM NUMBER: 252920 / OMIM Information: Sanfilippo syndrome, type B (3) // Genomic Sequence (SEQ ID NO: 414): // / SNP Information /
Context (SEQ ID NO: 2203): /
GGCATCAACCTGGCACTGGCCTGGAGCGGCCAGGAGGCCATCTGGCAGCGGGTGCGTGCCCACTGTCCCTTCCCCACCCTCCT
CTATGGCGGGAGCCACC
S
TAGGTGTTTTCACCCGCCCCCCAGCATGGGCGCAGTGTCTCTCTCTAGAAGTGCTTTTCAGCGTGCACAGTGGCTTGGGCCTCC
TAAAAACTGAGGCTTCC / Celera SNP ID: hCV2769165 / Public SNP ID: rs2071046 / SNP Chromosome Position: 40689613 / SNP in Genomic Sequence: SEQ ID NO: 414 / SNP Position Genomic: 11662 / Related Interrogated SNP: hCV1952126 / SNP Source: Applera / Population(Allele,Count): Caucasian (C,|G,) African American (C,11|G,5) total (C,11|G,5) / SNP Type: MISSENSE MUTATION;INTRON / SNP Source: dbSNP; Celera; HapMap; ABI_val; HGBASE / Population(Allele,Count): Caucasian (G,34|C,86) / SNP Type: MISSENSE MUTATION;INTRON /
Context (SEQ ID NO: 2204): /
ACTTCTTTCTGCCCCAGGAACCTTGGGTAGAGGGGGCATTGGACCTCAAAGCCAGCTGGCAGCCTCAGCATTTCAGATCAAAG
TGGCCTGCCTCTTCAAC
R
TCTCAGCCTACCCAAGGCATGTCCTAGGTGTAGGCTGCTCTCTGGTGGCCAGATAGGGAAGGACTCATTCATCCTTTGGCAAG
GTGGGCTGCGATCTGAG / Celera SNP ID: hCV587962 / Public SNP ID: rs647397 / SNP Chromosome Position: 40679398 / SNP in Genomic Sequence: SEQ ID NO: 414 / SNP Position Genomic: 1447 / Related Interrogated SNP: hCV1952126 / SNP Source: dbSNP; HapMap; ABI_val; HGBASE / Population(Allele,Count): Caucasian (G,166|A,60) / SNP Type: INTERGENIC;UNKNOWN /
Context (SEQ ID NO: 2205): /
TTATAAGACAAAAAAGAAGGGGAGTGTTAAATTCCAAACAGCTGCTTTACTTAAGATGCGATTACGCTTCAGAGATACCAATG
GGCATTGCTGTGTTCAG
Y
AGAAAGAAATGTAAATGAAGGCTGACTTGATTGTGGCATTTAGAGGGAACTCCTGGTACCTGGAAATGTGAATCTGGACTCTT
ACCTGTGTCATCAAAGT / Celera SNP ID: hCV2977462 / Public SNP ID: rs4793099 / SNP Chromosome Position: 40686040 / SNP in Genomic Sequence: SEQ ID NO: 414 / SNP Position Genomic: 8089 / Related Interrogated SNP: hCV1952126 / SNP Source: dbSNP / Population(Allele,Count): Caucasian (C,67|T,159) / SNP Type: UTR3;SILENT MUTATION;PSEUDOGENE //

Gene Number: 78 / Gene Symbol: PCM1 - 5108 / Gene Name: pericentriolar material 1 / Chromosome: 8 / OMIM NUMBER: 156535 / OMIM Information: // Genomic Sequence (SEQ ID NO: 415): // / SNP Information /
Context (SEQ ID NO: 2206): /
AGCTACTGACCCATTGCTTATTATACGTTACAGTTAAATCGAGTTGTCAGAGCATATCCAAACCATCTAAAAGCAGTGACAGC
AGCAGCTAAATTGATAG
R
AACTTTATCTGACCTAGTATTAGGGAATAAATGTTACCTACAAGTTACACGTGCAGTTGAAAGCTCATTACCTTCCAGTCAGA
TCCAACATGTCTTAATA / Celera SNP ID: hCV16233239 / Public SNP ID: rs2237852 / SNP Chromosome Position: 17877824 / SNP in Genomic Sequence: SEQ ID NO: 415 / SNP Position Genomic: 107458 / SNP Source: dbSNP; HapMap; HGBASE / Population(Allele,Count): Caucasian (G,34|A,86) / SNP Type: INTRON //

Gene Number: 79 / Gene Symbol: RALGDS - 5900 / Gene Name: ral guanine nucleotide dissociation stimulator / Chromosome: 9 / OMIM NUMBER: 601619 / OMIM Information: // Genomic Sequence (SEQ ID NO: 416): // / SNP Information /
Context (SEQ ID NO: 2207): /
GTCCAGGAAGCAGATAGCAGAGGCCGAGGCCTGGCTTTTGCTCCTCTTTCCTGCTGCTAACTTGACTCAGGGAGCTGCTGGCC
AGACTCGGTGTCCAGCT
S

GGGGGACACTCCAGCTCCCCTCCCCCTGGCTTCTGGGAACCTGAGGCCCCGTCAAGTCGCTCAACACACATTCAGGCCCAGCC
GGCTCTGCTAGGTAGTG / Celera SNP ID: hCV2535958 / Public SNP ID: rs2519198 /
SNP Chromosome Position: 136009452 / SNP in Genomic Sequence: SEQ ID NO: 416 /
SNP Position Genomic: 46345 / Related Interrogated SNP: hCV25610857 / SNP
Source: dbSNP; Celera; HapMap; HGBASE / Population(Allele,Count): Caucasian
(G,64|C,34) / SNP Type: INTRON //

Gene Number: 80 / Gene Symbol: RGS7 - 6000 / Gene Name: regulator of
G-protein signaling 7 / Chromosome: 1 / OMIM NUMBER: / OMIM Information: //
Genomic Sequence (SEQ ID NO: 417): // / SNP Information /
Context (SEQ ID NO: 2208): /
TAAGCCATTATTTCTTCAAGTACTTTTTTTTTTTTAAGTTCAGCTTTTTATTGAACACATTATAAAAGAGGTTTCGTCAAAAAG
ACCAAAGCCCATGTCAC
Y
ATCAGACTTCTCGGATTCTTCTTTCTTTGCTTCCACTTTCTTCTCCTCAGCTGGAGCAGCAGCAGCAGAGGCGGGCAGAAGCT
CCTGCTGGTACAGATAG / Celera SNP ID: hCV26887450 / Public SNP ID: rs670659 /
SNP Chromosome Position: 241161775 / SNP in Genomic Sequence: SEQ ID NO: 417 /
SNP Position Genomic: 232958 / SNP Source: dbSNP; HapMap /
Population(Allele,Count): Caucasian (C,78|T,40) / SNP Type: INTRON;PSEUDOGENE
/
Context (SEQ ID NO: 2209): /
ACCATAAAATTTATATAAATGTTAAAAGCACAGTTCAAACTGAATTATAATTTTTCAGGAGGTTTGCTTGCGTGCCATATTAA
AATATCCCTGATGAAAC
R
TATTTCTTAAAACCACTAAGAAAAAAGTTCTTGCAATGGGAAAATGATTTATTTACCTGGATCTTCTATAGTTAAGTTCTTTA
TCAACCATTGAACAATG / Celera SNP ID: hCV25653735 / Public SNP ID: rs7520707 /
SNP Chromosome Position: 241146322 / SNP in Genomic Sequence: SEQ ID NO: 417 /
SNP Position Genomic: 217505 / Related Interrogated SNP: hCV26887450 / SNP
Source: Applera / Population(Allele,Count): Caucasian (A,9|G,11) African
American (A,15|G,3) total (A,24|G,14) / SNP Type: INTRON / SNP Source:
dbSNP; Celera; HapMap / Population(Allele,Count): Caucasian (A,71|G,47) / SNP
Type: INTRON /
Context (SEQ ID NO: 2210): /
TGTCTGCAGTTTTCAAATGTTTATTAAGTGGCTTGGTATGCATTTCGTTGGGCTTATTTATCCTATTTGGGCTTCTCTCAGCT
TCTTCAACTGAAGGTTT
R
TATCTTTGCTATGCTTGTGATGTTTTTAAGCCATTATTTCTTCAAGTACTTTTTTTTTTTTAAGTTCAGCTTTTTATTGAACAC
ATTATAAAAGAGGTTTC / Celera SNP ID: hCV916106 / Public SNP ID: rs575226 / SNP
Chromosome Position: 241161648 / SNP in Genomic Sequence: SEQ ID NO: 417 /
SNP Position Genomic: 232831 / Related Interrogated SNP: hCV26887450 / SNP
Source: dbSNP; HapMap; HGBASE / Population(Allele,Count): Caucasian
(A,143|G,83) / SNP Type: INTRON;PSEUDOGENE /
Context (SEQ ID NO: 2211): /
TGGGTGGCTTGAAGACACAGCATGGGTTGAAAAGTGATCTGAGCAGATGCAATGATATAAATATGGCTATATCAAAAAGGGTT
TTAAATATAAGCTAAAA
Y
GCTTGTTTGAACATTATCCTGAGGAAAATGGGGGGGATCTATTAGTTATTGAACTTCAGAAGAGACATGATTAGATCTGAGTTT
TGGAAAGATCATTATAG / Celera SNP ID: hCV1703881 / Public SNP ID: rs3912107 /
SNP Chromosome Position: 241126234 / SNP in Genomic Sequence: SEQ ID NO: 417 /
SNP Position Genomic: 197417 / SNP Source: dbSNP; HapMap; HGBASE /
Population(Allele,Count): Caucasian (C,139|T,87) / SNP Type: INTRON /
Context (SEQ ID NO: 2212): /
CCCATCCACTGAGTTTTCCTTTTTGATGTTGTCCTTTTCAGTCCTAAAGTTTCCATTTTTTTTATAACTTCTATTTCTTTGCTG
AGAATTTCAGTTTATTT
M
CTGAGGCCTTCTAGTTTGTAATATGCTTAAAGCATATTATTATTATTATTATTAAATAATTTTTATCATAGATCAGGGGTGTC
CAATCTTTTTTTGGCTTC / Celera SNP ID: hCV1874947 / Public SNP ID: rs494075 /
SNP Chromosome Position: 241162500 / SNP in Genomic Sequence: SEQ ID NO: 417 /
SNP Position Genomic: 233683 / Related Interrogated SNP: hCV26887450 / SNP
Source: dbSNP; Celera; HapMap; HGBASE / Population(Allele,Count): Caucasian
(C,124|A,102) / SNP Type: INTRON /
Context (SEQ ID NO: 2213): /
AAATAATAGTAACAGTATTAGGTGATTATAAAATATGGATAAGTGAAATGAACAACAGCAGTGTCGTAAAGGAAGAAAGGGAT

GAATTGGGAATATTCTG
Y
TACAAGGTACCTTCATGGTATATGAAACAGTATAGTGTAATTTGAACATGGAGTTAGATTAATTAAAATGTATATCGTAAACT
CTAGAACAGCTGCTAAA / Celera SNP ID: hCV8857379 / Public SNP ID: rs984402 /
SNP Chromosome Position: 241115683 / SNP in Genomic Sequence: SEQ ID NO: 417 /
SNP Position Genomic: 186866 / SNP Source: dbSNP; HapMap; HGBASE /
Population(Allele,Count): Caucasian (T,137|C,85) / SNP Type: INTRON /
Context (SEQ ID NO: 2214): /
ATTTGAAAATCGTATCAGATGTTACAATTGAGGTTTTTAGACTCATTATTTTTAAAAGGGTGACCTTTTACTGTACAGCAGGA
ACCTGTCTTGTGTGGTT
W
GAGACAAATGACAATGAAGAGATGGATATGTTCCTAGGATCATATTTAGGCTATAAAACATCTATCAGTGCCCTTATCACACT
TTTTGTACTGCTGTAGT / Celera SNP ID: hCV26887401 / Public SNP ID: rs10802916 /
SNP Chromosome Position: 241132016 / SNP in Genomic Sequence: SEQ ID NO: 417 /
SNP Position Genomic: 203199 / Related Interrogated SNP: hCV26887450 / SNP
Source: dbSNP; Celera; HapMap / Population(Allele,Count): Caucasian
(T,127|A,97) / SNP Type: INTRON /
Context (SEQ ID NO: 2215): /
GAGAGATGAACTCCAGCCCAGCTACAGACTATGTTCTCTAACCCCTTGATCTAAGAAATCTCAGCAATGGCAAAAATCTGGAT
ATCACCAATTAATGGCC
R
GTGTCCTAGTTCTGTCTGCCTTTGGCCAAAAGCGGGTACTGAAAAAAGAGAAAAGCAGCCACTGGCTTCCAGGAACTGGCTGG
GCACTCACAGCTATGTC / Celera SNP ID: hCV26887441 / Public SNP ID: rs9786932 /
SNP Chromosome Position: 241152090 / SNP in Genomic Sequence: SEQ ID NO: 417 /
SNP Position Genomic: 223273 / Related Interrogated SNP: hCV26887450 / SNP
Source: dbSNP; Celera; HapMap / Population(Allele,Count): Caucasian
(G,133|A,93) / SNP Type: INTRON /
Context (SEQ ID NO: 2216): /
GGAAAATCTTTTATTTGAAATAAGAGTAAGAAGTCAATTGAGAGAATTCCAAAAGGTGCCCCAAAGCAAAAACAAAAGATTGA
GGTTTAAATGAACTGTA
Y
GGTATAAAAAAGGCAACAATTTTAAAAGTAAGATAGTAAGGATAAATAGTAAAATAGTAAGGCTTTCTAATTAAGATACAAAA
CCTAGCAGCCATAAAAC / Celera SNP ID: hCV26887461 / Public SNP ID: rs4660023 /
SNP Chromosome Position: 241164670 / SNP in Genomic Sequence: SEQ ID NO: 417 /
SNP Position Genomic: 235853 / Related Interrogated SNP: hCV26887450 / SNP
Source: dbSNP; Celera; HapMap; HGBASE / Population(Allele,Count): Caucasian
(C,134|T,92) / SNP Type: INTRON /
Context (SEQ ID NO: 2217): /
AGTGGGGAAGCCCAGCAACGCCTGTCCCTCTAGATTCTTCTGGCCTCTCTGAGCAGCATTCCTTCCTTCTGGGTATGGGGCAG
AGCTCTCTCTGGACTGG
R
GGTCTTAGGACCTACAGTCCAACAAGGCAGGTCAGATAACTTCTTTATGACTACTTTTACGGCTGGCTTTGAGGAGAAGGGGT
TCTGATTTCCATGACCC / Celera SNP ID: hCV26887463 / Public SNP ID: rs6680767 /
SNP Chromosome Position: 241168080 / SNP in Genomic Sequence: SEQ ID NO: 417 /
SNP Position Genomic: 239263 / Related Interrogated SNP: hCV26887450 / SNP
Source: dbSNP; Celera; HapMap / Population(Allele,Count): Caucasian
(G,76|A,42) / SNP Type: INTRON /
Context (SEQ ID NO: 2218): /
GAGCAGCATTCCTTCCTTCTGGGTATGGGGCAGAGCTCTCTCTGGACTGGGGGTCTTAGGACCTACAGTCCAACAAGGCAGGT
CAGATAACTTCTTTATG
R
CTACTTTTACGGCTGGCTTTGAGGAGAAGGGGTTCTGATTTCCATGACCCGCCTCGGGCCAGAGGGATTCTAGTCTCTGGGGA
GAATGGAACAGGTCAGG / Celera SNP ID: hCV26887464 / Public SNP ID: rs6669640 /
SNP Chromosome Position: 241168130 / SNP in Genomic Sequence: SEQ ID NO: 417 /
SNP Position Genomic: 239313 / Related Interrogated SNP: hCV26887450 / SNP
Source: dbSNP; Celera / Population(Allele,Count): Caucasian (A,72|G,48) / SNP
Type: INTRON /
Context (SEQ ID NO: 2219): /
CATTCTTTATGGTGACTGTTCCAATCTTTCACCATGGTTCAATTTCTCAATCACTTCTATGAAGTTGCTCAAAACAAAAGCAA
TATACAGAAGTTTAACT
R
AAAGGTATAGCCCTATTTGACAGAGGGATAATTTATACATACAGTTTTGCACAGATTATATGCACAGAATTTTAGTCCAAAAC
TTGCAAAGTTCTGACAA / Celera SNP ID: hCV26887465 / Public SNP ID: rs10802919 /

409

SNP Chromosome Position: 241168535 / SNP in Genomic Sequence: SEQ ID NO: 417 / SNP Position Genomic: 239718 / Related Interrogated SNP: hCV26887450 / SNP Source: dbSNP; Celera; HapMap / Population(Allele,Count): Caucasian (A,139|G,77) / SNP Type: INTRON /
Context (SEQ ID NO: 2220): /
GTGAATCCAGGAGACAGAGGTTGCAGTGAGCGGAGATCGTGCCACTGCACTCCAGCCTGGGCAACAGAGCAAGACTTTGTCTC
AGGAAAAAACAAAATTT
K
CAGAAAATCAGAGAAGATAAGTCTATTGTATTTCTTACATGTTTGCTTACCACGTTTTGTTCTTATCTGATATTCCAAGGTTC
CTTTTCTTATCGTTTCC / Celera SNP ID: hCV31714470 / Public SNP ID: rs10926390 /
SNP Chromosome Position: 241161141 / SNP in Genomic Sequence: SEQ ID NO: 417 / SNP Position Genomic: 232324 / Related Interrogated SNP: hCV26887450 / SNP Source: dbSNP; HapMap / Population(Allele,Count): Caucasian (T,63|G,55) / SNP Type: TRANSCRIPTION FACTOR BINDING SITE;INTRON /
Context (SEQ ID NO: 2221): /
AAAATGTTTTAAGAAAAATGTATACATTATAAGTAACTACATGTGACATTTGACTCCTGTCCCTAGGCAGGGGCTGCTGAAAC
ATCTACCAACCAATATC
Y
ATATCCAAACATCCAATATCCAAACAACCAATATCCAATATCCCAGGAAGACTTGGCAGATCAGTTCGTGAGGGCCAAGGGTT
CACTGATGGTGCTGCCA / Celera SNP ID: hCV31714442 / Public SNP ID: rs12758552 /
SNP Chromosome Position: 241131301 / SNP in Genomic Sequence: SEQ ID NO: 417 / SNP Position Genomic: 202484 / Related Interrogated SNP: hCV26887450 / SNP Source: dbSNP / Population(Allele,Count): Caucasian (C,75|T,45) / SNP Type: INTRON /
Context (SEQ ID NO: 2222): /
CTCTCTCCCAGTCTTCTCTGCTTAAGCTCTGGTGAATCTCCCTTCCTGGAGCCCTCTATGTGGCTGCTGTCACGCTCTGTCTA
CTCTGCTATCTTTCTCT
S
CGGCTCCTGACCTAGTTTTCCCTACCTTCTGACTCCTGCTCTCTATGGTTAAAAGTCTCTTAAAAATCTATCTCTGCTGTAGC
TTCCCCTTTCCTTTGTT / Celera SNP ID: hCV31714435 / Public SNP ID: rs12143076 /
SNP Chromosome Position: 241125609 / SNP in Genomic Sequence: SEQ ID NO: 417 / SNP Position Genomic: 196792 / Related Interrogated SNP: hCV26887450 / SNP Source: dbSNP; HapMap / Population(Allele,Count): Caucasian (C,68|G,40) / SNP Type: INTRON /
Context (SEQ ID NO: 2223): /
GACCTTTCAGTACATTCTAGGTTTGGGGACTTTGTTGTGCAGAGCAATTGGATAATTATACAAGTCAATGGAAACAACAGTCA
CTCGTTGCATTACAGAA
Y
GCAGTATTTAAATGCAAACGCAACACCAAAAACAAATAAGGTGGGGAGTAAAAGAAAAGAGTGTGGCTAGCTTCTAGGTTAAA
TAAGACAGTTGGTGCCCA / Celera SNP ID: hCV31714447 / Public SNP ID: rs10926387 /
SNP Chromosome Position: 241133736 / SNP in Genomic Sequence: SEQ ID NO: 417 / SNP Position Genomic: 204919 / Related Interrogated SNP: hCV26887450 / SNP Source: dbSNP; HapMap / Population(Allele,Count): Caucasian (T,70|C,48) / SNP Type: UTR3;INTRON /
Context (SEQ ID NO: 2224): /
GAGACAGAGGTTGCAGTGAGCGGAGATCGTGCCACTGCACTCCAGCCTGGGCAACAGAGCAAGACTTTGTCTCAGGAAAAAAC
AAAATTTTCAGAAAATC
R
GAGAAGATAAGTCTATTGTATTTCTTACATGTTTGCTTACCACGTTTTGTTCTTATCTGATATTCCAAGGTTCCTTTTCTTAT
CGTTTCCCTTTTATTTA / Celera SNP ID: hCV31714471 / Public SNP ID: rs10926391 /
SNP Chromosome Position: 241161151 / SNP in Genomic Sequence: SEQ ID NO: 417 / SNP Position Genomic: 232334 / Related Interrogated SNP: hCV26887450 / SNP Source: dbSNP; HapMap / Population(Allele,Count): Caucasian (A,119|G,107) /
SNP Type: INTRON /
Context (SEQ ID NO: 2225): /
GCTCTCTATGGTTAAAAGTCTCTTAAAAATCTATCTCTGCTGTAGCTTCCCCTTTCCTTTGTTTCTCTCTATATTCTTTCTTC
CTTGGCAAGCTCATCTA
W
TTTAAAAGGTCTGCTTACCAAATGTGTACCTCAGTCATCATTTATTCAATGTAGGAGAACAAAACCCACTAGACAAGGTGCCA
GCTCTGTCTATAGGAAG / Celera SNP ID: hCV31714436 / Public SNP ID: rs12132113 /
SNP Chromosome Position: 241125747 / SNP in Genomic Sequence: SEQ ID NO: 417 / SNP Position Genomic: 196930 / Related Interrogated SNP: hCV26887450 / SNP Source: dbSNP; HapMap / Population(Allele,Count): Caucasian (A,77|T,43) / SNP

Type: INTRON /
Context (SEQ ID NO: 2226): /
AATTTCGTCCATTTAGATTTGTTCAGTCTATGAGATGCCCATGAAACGTCCAAGTAGAGATCTATAGCAGACAAGACAGTTGG
CCAGGAGCTCAGAAGAG
R
GTCCAGAGGTATATTTGTAGTGGTTATCATACCAACAATGGGCAAAATAAGGAAGTGCATAAACTGTGAAGAGCCAAGAAATA
AGAAATCTCATGCCTGG / Celera SNP ID: hCV31714438 / Public SNP ID: rs12731839 /
SNP Chromosome Position: 241126616 / SNP in Genomic Sequence: SEQ ID NO: 417 /
SNP Position Genomic: 197799 / Related Interrogated SNP: hCV26887450 / SNP
Source: dbSNP; HapMap / Population(Allele,Count): Caucasian (A,75|G,43) / SNP
Type: INTRON /
Context (SEQ ID NO: 2227): /
AAATGTTTTAAGAAAAATGTATACATTATAAGTAACTACATGTGACATTTGACTCCTGTCCCTAGGCAGGGGCTGCTGAAACA
TCTACCAACCAATATCC
R
TATCCAAACATCCAATATCCAAACAACCAATATCCAATATCCCAGGAAGACTTGGCAGATCAGTTCGTGAGGGCCAAGGGTTC
ACTGATGGTGCTGCCAA / Celera SNP ID: hCV31714443 / Public SNP ID: rs61062329 /
SNP Chromosome Position: 241131302 / SNP in Genomic Sequence: SEQ ID NO: 417 /
SNP Position Genomic: 202485 / Related Interrogated SNP: hCV26887450 / SNP
Source: dbSNP; HapMap / Population(Allele,Count): Caucasian (A,73|G,43) / SNP
Type: INTRONIC INDEL;INTRON //

Gene Number: 81 / Gene Symbol: SELP - 6403 / Gene Name: selectin P (granule
membrane protein 140kDa, antigen CD62) / Chromosome: 1 / OMIM NUMBER: 173610
/ OMIM Information: Platelet alpha/delta storage pool deficiency (1);
{Atopy,/susceptibili / ty to}, 147050 (3) // / Genomic Sequence (SEQ ID NO:
418): // / SNP Information /
Context (SEQ ID NO: 2228): /
AAGTACATATTATTACCTTTGCAGGTTGGTGGAGTAGCTGACCATCTTCCAGAAGTTGTGCATTCCACATTATTGGGCCCCTC
CAGCTTAAAGCCGTTGT
Y
ACAAGAGAAATGGCAGGTGGAGCCAACATTGAATTCTCCACGAGTGTCAGAACAATCCAGGCTGCCCTGCTCTGGGGCAAAGA
GTTCTGGGCACTTGATG / Celera SNP ID: hCV11975285 / Public SNP ID: rs6127 / SNP
Chromosome Position: 169566313 / SNP in Genomic Sequence: SEQ ID NO: 418 /
SNP Position Genomic: 18226 / Related Interrogated SNP: hCV11975250 / SNP
Source: Applera / Population(Allele,Count): Caucasian (C,19|T,21) African
American (C,9|T,29) total (C,28|T,50) / SNP Type: MISSENSE MUTATION;INTRON /
SNP Source: dbSNP; HapMap / Population(Allele,Count): Caucasian (C,109|T,117)
/ SNP Type: MISSENSE MUTATION;INTRON /
Context (SEQ ID NO: 2229): /
GTCCAAGCCCCTCACTCTGTAGCCGGGCTGGCACTCAAATTTACAGCTGGAGCCATAGGCAAAAGCAGTGAGCGGATGAACAC
AGTCCATGGTTCCTTCA
Y
TGGGGGCTTCCAGGTGCTGACACTGCACAGCTGGAGAGAATAACCAAGGATAAAGAGAAAGATACTGAGAAAGGAGAAAAGCC
ACACAGAGAGCAATGGT / Celera SNP ID: hCV11975296 / Public SNP ID: rs6131 / SNP
Chromosome Position: 169580885 / SNP in Genomic Sequence: SEQ ID NO: 418 /
SNP Position Genomic: 32798 / Related Interrogated SNP: hCV11975250 / SNP
Source: Applera / Population(Allele,Count): Caucasian (C,8|T,2) African
American (C,9|T,7) total (C,17|T,9) / SNP Type: MISSENSE
MUTATION;ESE;TRANSCRIPTION FACTOR BINDING SITE;ESE SYNONYMOUS / ;INTRON // SNP
Source: dbSNP; HapMap; HGBASE / Population(Allele,Count): Caucasian
(C,176|T,50) / SNP Type: MISSENSE MUTATION;ESE;TRANSCRIPTION FACTOR BINDING
SITE;ESE SYNONYMOUS / ;INTRON //
Context (SEQ ID NO: 2230): /
AAGTTTAAAAGTACTCACAAAATCTAATAGGCAATTCAACATAAAACTCCATGGCTATCGCTGTTCCTCACTTTCTGAACCTT
TACCTGCCTGACTTTAC
Y
CCATACCACTCCAACTCACTTCAACCACTTCCCATGCAGAAAAAAATAAACTCACCATCTCCTCTGTGACTCTGCTGGTTTTC
TGCCTTCTGCCCAACCC / Celera SNP ID: hCV2480416 / Public SNP ID: rs732314 /
SNP Chromosome Position: 169599254 / SNP in Genomic Sequence: SEQ ID NO: 418 /
SNP Position Genomic: 51167 / Related Interrogated SNP: hCV11975250 / SNP
Source: Applera / Population(Allele,Count): Caucasian (C,16|T,22) African
American (C,21|T,15) total (C,37|T,37) // / SNP Type: INTRON / SNP Source:

Applera / Population(Allele,Count): Caucasian (C,2|T,8) African American (C,4|T,6) total (C,6|T,14) / SNP Type: INTRON / SNP Source: dbSNP; Celera; HapMap; HGBASE / Population(Allele,Count): Caucasian (T,104|C,122) / SNP Type: INTRON /
Context (SEQ ID NO: 2231): /
ATTTTTCATAAGAAAATGCTAGAGAGAAATGATATGATTTAGGGTTAAACAATATGACAGTTTGTCTGGGTTGTGTTTCTATG GTTTTGACTCAACAATT
S
CTACCAGAGGAACGAATCTCCAGAACTTTGGAAACTTACCCACAGGATGAGGGGACAGAATGACCAGTCATGGTTCCCTGTGT ACTCACCATGTGAATCA / Celera SNP ID: hCV325211 / Public SNP ID: rs3753305 /
SNP Chromosome Position: 169554058 / SNP in Genomic Sequence: SEQ ID NO: 418 / SNP Position Genomic: 5971 / Related Interrogated SNP: hCV11975250 / SNP Source: dbSNP; Celera; HapMap; ABI_Val; HGBASE / Population(Allele,Count): Caucasian (C,112|G,114) / SNP Type: INTRON /
Context (SEQ ID NO: 2232): /
ATATGTGGATATGCTGTGCTACATGATCTCTATGTTTCCTCTCAATTCTGAGATACTAAAATTCTATAAACCATGCAAGGTTC TATAGGAAGAAGTTCCT
R
TGGCAGCCAGTGAATTTGGTGAAGCTGCACTGAGACACTGGGAAGGGTTCAAATAAAGAGAAGATGTAGGAGGCAATAGCAGC TAAATGGATACAGGTTT / Celera SNP ID: hCV325253 / Public SNP ID: rs2236868 /
SNP Chromosome Position: 169583996 / SNP in Genomic Sequence: SEQ ID NO: 418 / SNP Position Genomic: 35909 / Related Interrogated SNP: hCV11975250 / SNP Source: dbSNP; Celera; HapMap; HGBASE / Population(Allele,Count): Caucasian (G,111|A,111) / SNP Type: TRANSCRIPTION FACTOR BINDING SITE;INTRON /
Context (SEQ ID NO: 2233): /
TGGAAGTCATCTAATTTACTCCTGTCCTTTTATAAAAGAAGGAGACATAAGTAATTTGCCCAAGCTCAGATTAAATGACATAT AAAATGTCTTAAGGGCA
R
GCTCTACCTCTTCACATGGAAAATAACATGCTTTATGACCTGGAGCCTACCAGTTGGTCCAGCCTCATTGCCCACTGTATGTA CTTTCTCAGCAAAACAG / Celera SNP ID: hCV2480424 / Public SNP ID: rs2244529 /
SNP Chromosome Position: 169587032 / SNP in Genomic Sequence: SEQ ID NO: 418 / SNP Position Genomic: 38945 / Related Interrogated SNP: hCV11975250 / SNP Source: dbSNP; Celera; HapMap; HGBASE / Population(Allele,Count): Caucasian (A,155|G,71) / SNP Type: INTRON /
Context (SEQ ID NO: 2234): /
CCTTCCCACCTTAACTAAGGGTAGAAGCCCCTGCAATGTGCTCCCACAGCCTCTGTTCTTGCGCTGTTCATACATGATTGTCA TTCTTCCCCATTATACC
R
TGGTCTCCTCGAAGTTGGGCTCAGTCATTCATAGTTTTATTCTGGAGCTGAGCAGAGTGTTTTGTACATTGAAAGTGCTCATT AAATATTGACATTATCT / Celera SNP ID: hCV2480428 / Public SNP ID: rs3917740 /
SNP Chromosome Position: 169579266 / SNP in Genomic Sequence: SEQ ID NO: 418 / SNP Position Genomic: 31179 / Related Interrogated SNP: hCV11975250 / SNP Source: dbSNP; Celera; HapMap; ABI_Val; HGBASE / Population(Allele,Count): Caucasian (G,169|A,57) / SNP Type: INTRON /
Context (SEQ ID NO: 2235): /
ACCTTTTTTTTTTTAATAAGTTATCCAGTCTCGGGTATGTCTCTATCAGCATCATGAGAACAGAATAATACACTCAGCAATGT GAATAATAAGGATAATA
R
TCACCAAATACATAGACATGTAAGGACCATTAAATGAATTTAATGTGTTTGGAACAATACCTATCACGCTGTTAAGTGTAAGT TTAAGTGTTGGCTATTA / Celera SNP ID: hCV8919485 / Public SNP ID: rs1800808 /
SNP Chromosome Position: 169601129 / SNP in Genomic Sequence: SEQ ID NO: 418 / SNP Position Genomic: 53042 / Related Interrogated SNP: hCV11975250 / SNP Source: dbSNP; HapMap; HGBASE / Population(Allele,Count): Caucasian (A,105|G,15) / SNP Type: INTRON /
Context (SEQ ID NO: 2236): /
CAATGAAAGTGCTCCCTCCCATGTTCCTCTCTGAATTCTATTTATCTCATTACACTGGTGCATTCAAATACACAAGAAATAGC TAAGTCCCTTTGAGACT
Y
CTGAGAGTGTGCTAGCATAGGGATTCAGTCATGAGTATATCTCATGAAGCCCATGGTTTTGTTGGGGGTGGGGGAGCAGATAT TAAATAAAAATCACACA / Celera SNP ID: hCV8919492 / Public SNP ID: rs1569476 /
SNP Chromosome Position: 169608917 / SNP in Genomic Sequence: SEQ ID NO: 418 / SNP Position Genomic: 60830 / Related Interrogated SNP: hCV11975250 / SNP Source: dbSNP; HapMap / Population(Allele,Count): Caucasian (C,127|T,99) /

SNP Type: INTRON /
Context (SEQ ID NO: 2237): /
TACCAAAAAAATCTATTTAGAGATTTTTGTGAAGACATTATTTGTAAGAGCGGAATATTGAAAACAAACACAAATGCCATTAAT
TGAGGGTGGAAATTAAT
K
ATGGTGGATCCATGAAAAGAATGTTAAAGGTCACTAAAAATAATATATTTTAACTTGTAAAGATAGGAGAGAGGTCTCAATGT
ATGATGTTAAATAAAAA / Celera SNP ID: hCV9945935 / Public SNP ID: rs3917750 /
SNP Chromosome Position: 169576753 / SNP in Genomic Sequence: SEQ ID NO: 418 /
SNP Position Genomic: 28666 / Related Interrogated SNP: hCV11975250 / SNP
Source: dbSNP; Celera; HapMap; ABI_Val; HGBASE / Population(Allele,Count):
Caucasian (G,77|T,43) / SNP Type: TFBS SYNONYMOUS;INTRON /
Context (SEQ ID NO: 2238): /
GCTTGAATAATCCCTTATGATCCGTTTTAAGTGTGTCAAAGAGCTCATTGTGTCTTTTTCTCCCAGATAACATATACAATACA
TTCCATAACTCAGACTA
R
GTGTCAGCATCATGGTGTATAGCCATCTATTCTATTGCCTTCTTGTTTTTTCTTTTTTTTTTTTTTTGCTGTTCAATTTTTTA
TTATAGTGAGATATACG / Celera SNP ID: hCV11342138 / Public SNP ID: rs2142760 /
SNP Chromosome Position: 169577333 / SNP in Genomic Sequence: SEQ ID NO: 418 /
SNP Position Genomic: 29246 / Related Interrogated SNP: hCV11975250 / SNP
Source: dbSNP; Celera; HapMap / Population(Allele,Count): Caucasian
(A,130|G,96) / SNP Type: INTRON /
Context (SEQ ID NO: 2239): /
TTTTAGTAAAAAAATATGTATCTGCCCACTTTCACTCAAATGTTGAAAGTCTATACAAAGTGAAAGGTATGGCAAATAATGTAG
AACTGTCTAGTCTTTTA
Y
CCATTCAACTTTATTGTTAAAATTGTATATACATCATAAAAATATTAATTGCATAGGAGGAAAAGCGGAGAAAATATTGGAAG
AATTCATACCAGAAGCC / Celera SNP ID: hCV15868017 / Public SNP ID: rs2223303 /
SNP Chromosome Position: 169574887 / SNP in Genomic Sequence: SEQ ID NO: 418 /
SNP Position Genomic: 26800 / Related Interrogated SNP: hCV11975250 / SNP
Source: dbSNP; HapMap / Population(Allele,Count): Caucasian (C,77|T,43) / SNP
Type: INTRON /
Context (SEQ ID NO: 2240): /
ACTTTCTTAAAAACAATCTGCCTAAAATACACCTTTCTCCCACTTTATAAGAGAAAAATTATACCTTTTACTAATTTCTTATT
TATTCCAGAGCTTTGCC
Y
TTGGATATAAATACATTCGTATGCCAGAGATGATTCGAAATATTGGTATAGGTATTAAGAATTTGAATATTGTAAATGATATT
GCAGCAAATCCGTTTTC / Celera SNP ID: hCV27497504 / Public SNP ID: rs3917683 /
SNP Chromosome Position: 169591373 / SNP in Genomic Sequence: SEQ ID NO: 418 /
SNP Position Genomic: 43286 / Related Interrogated SNP: hCV11975250 / SNP
Source: dbSNP; HapMap; HGBASE / Population(Allele,Count): Caucasian
(T,92|C,134) / SNP Type: INTRON /
Context (SEQ ID NO: 2241): /
AGGAATTGAGACATTGCTATAATAAAACTTCTAATTCCATTTACTTATTCATATGAATGAAGTTTCTCAATGTTTAAATCCTT
GAAAATGAAATATGGGA
R
TAATATTGATGCCAAACTTGTCTAATTTTGTTGATAAGTAATATTCATTCATGGATATATGAACAAATGAGGGAAAGCTCCTC
CTGTCTTAGAGATGAAT / Celera SNP ID: hCV27523232 / Public SNP ID: rs3917681 /
SNP Chromosome Position: 169591703 / SNP in Genomic Sequence: SEQ ID NO: 418 /
SNP Position Genomic: 43616 / Related Interrogated SNP: hCV11975250 / SNP
Source: dbSNP; HapMap / Population(Allele,Count): Caucasian (A,105|G,15) /
SNP Type: INTRON /
Context (SEQ ID NO: 2242): /
CATGAAAAGGACTTCAGGTCCTGAAGGGCAAAGGCAGAGACAAGGCTGAAAAATAACCGACTCTCTGTATCCAAGGGTTCTGC
ATCTACGGATTCAACTC
R
TTGTGGATCAAAAACATTCAGAAAAAAAAATAAATTATATAAAGTTCCAAAAAGCAAAACTTGAATTTGCCATGTGCTGAGTAT
GTTGAATCCATGCAGAT / Celera SNP ID: hCV29397262 / Public SNP ID: rs3917786 /
SNP Chromosome Position: 169569445 / SNP in Genomic Sequence: SEQ ID NO: 418 /
SNP Position Genomic: 21358 / Related Interrogated SNP: hCV11975250 / SNP
Source: dbSNP; HapMap; HGBASE / Population(Allele,Count): Caucasian
(G,106|A,120) / SNP Type: INTRON /
Context (SEQ ID NO: 2243): /
ACACCCCAATGACCTTACCAGAAAACAATGACAAATGATTGGAAACACAGAAATGGAGGAAAATACTTTCTTCCCCCTTTCCC

AACCAAGTGGTGGAATC
K
GCGCCCAAATTTTGGCTCTGTTACTTGCTAGCAATCTATCACTAGACAATAACCTTTCTGAGCCTCAGTTGCTTTATCTTCGA
AATGGGAAAAAGGAAAA / Celera SNP ID:    hCV32141484 /  Public SNP ID:    rs3917768 /
SNP Chromosome Position:    169572947 /  SNP in Genomic Sequence:    SEQ ID NO: 418 /
 SNP Position Genomic:    24860 /  Related Interrogated SNP:    hCV11975250 /  SNP
Source:    dbSNP; HGBASE /  Population(Allele,Count):    Caucasian (T,135|G,91) /
SNP Type:    INTRON /
Context              (SEQ ID NO: 2244): /
TGATTAATAGTGCTATGAACATGGGTGCACAAATATCTCTTCAAGACCTGCTTTCAAACCACTGCCTCATTTTAAAATCAAGG
TATACTTAGGAACCCAG
R
GGAGTCACATAGATTGCTCAGAGGTCTCCGTAAATGAAAACACAAAATGTCAATATTACACTTCTGGAACATGACTTTAGTGT
GAGGTACTCCTCGTAAA / Celera SNP ID:    hCV32141485 /  Public SNP ID:    rs3917744 /
SNP Chromosome Position:    169577990 /  SNP in Genomic Sequence:    SEQ ID NO: 418 /
 SNP Position Genomic:    29903 /  Related Interrogated SNP:    hCV11975250 /  SNP
Source:    dbSNP; HapMap; HGBASE /  Population(Allele,Count):    Caucasian
(A,84|G,142) /  SNP Type:    INTRON /
Context              (SEQ ID NO: 2245): /
TAGCATTTATCTGCTGTTCTGCAGGAAGTCTTTCAGACTTCGAATGCCTAAGATGGGGCAGCCACCACTCCCCACCACTGAGA
CTCAGTGAGCGTAAATG
R
TTTGCCCAAGCTTGCTCACATGAATGTGCATAGGGTTAACACAGTTATTCTTATTCTTCCAGGGCTTATGAATTTTGAAAATC
TGATAAAAGCCGTGGTC / Celera SNP ID:    hCV32141499 /  Public SNP ID:    rs3917862 /
SNP Chromosome Position:    169593113 /  SNP in Genomic Sequence:    SEQ ID NO: 418 /
 SNP Position Genomic:    45026 /  Related Interrogated SNP:    hCV11975250 /  SNP
Source:    dbSNP; HapMap; ABI_val; HGBASE /  Population(Allele,Count):    Caucasian
(A,209|G,17) /  SNP Type:    INTRON /
Context              (SEQ ID NO: 2246): /
CTTTTCAGCAAAATCTTATTTAATCCTCATGATGAAACTATAGAGTATTATTACTATATCCATTTTATAGAAGAAGAAACTGA
GTCATTAACAAGATTGA
R
TCTTAGGGCAGAAAAGGAAACTATGTGGATATTCCAGATCAGTCCCAGAAGATTCATAGGCTCACAGAAAGAAATGGGAGGGA
TGGGGATGAGAAACTAT / Celera SNP ID:    hCV32141505 /  Public SNP ID:    rs3917657 /
SNP Chromosome Position:    169598742 /  SNP in Genomic Sequence:    SEQ ID NO: 418 /
 SNP Position Genomic:    50655 /  Related Interrogated SNP:    hCV11975250 /  SNP
Source:    dbSNP; HapMap; HGBASE /  Population(Allele,Count):    Caucasian
(G,101|A,9) /  SNP Type:    INTRON /
Context              (SEQ ID NO: 2247): /
TCAAGAGACCCTAATGATGTTTGTCCAGACTTGGATTACCAGTTCCCAGGGTGATCTGAGTCTGTTAAAAACAAAGATTCCTC
CAAGCTGTATTCTTAAT
R
TCCTCTATTCCCTATACTTCTATAAACCTGTCTCAGTAGGTCTTCTAGTGTCCACTTATAGATGTAGAGGCCACCCTTTCTAA
TCTTATGGGGAAATTAT / Celera SNP ID:    hDV76908651 /  Public SNP ID:    rs3917729 /
SNP Chromosome Position:    169581130 /  SNP in Genomic Sequence:    SEQ ID NO: 418 /
 SNP Position Genomic:    33043 /  Related Interrogated SNP:    hCV11975250 /  SNP
Source:    CDX; dbSNP /  Population(Allele,Count):    Caucasian (G,104|A,16) /  SNP
Type:    INTRON //

Gene Number:    82 /  Gene Symbol:    SLC4A3 - 6508 /  Gene Name:    solute carrier
family 4, anion exchanger, member 3 /  Chromosome:    2 /  OMIM NUMBER: /  OMIM
Information: //  Genomic Sequence (SEQ ID NO: 419): //   /  SNP Information /
Context              (SEQ ID NO: 2248): /
CATGAAGAGTGAGTGAGACCTTGTGGCAGCCCCCATGGTCCACTGCGACGGACTCCCAGCCTGCGAGTGACCTTGGAGAGGCT
CTGAGCTGTCCCCTGCT
M
AGGGCTGAGTCCTCTCTGAATCCCTGTCTGCTGGGATGTGGCCAGTGATGGGGACCTCACTACCTCACCCAGCCACCCTCTCT
GACGGCCAGAGCTGAGA / Celera SNP ID:    hCV2414166 /  Public SNP ID:    rs612078 /
SNP Chromosome Position:    220495167 /  SNP in Genomic Sequence:    SEQ ID NO: 419 /
 SNP Position Genomic:    12875 /  SNP Source:    Applera /
Population(Allele,Count):    Caucasian (A,0|C,40)  African American (A,1|C,37)
total (A,1|C,77) /  SNP Type:    INTRON /  SNP Source:    dbSNP; HapMap; ABI_val;
HGBASE /  Population(Allele,Count):    Caucasian (A,3|C,223) /  SNP Type:    INTRON

/
Context (SEQ ID NO: 2249): /
TGAGGAACCAATGAGAATATGAATATTAAAGTGGCTTGTAAGCTGGAAAGCACTACCTGCATTTAAAATAATGAAGTTATCAT
TGTTGTTATCCTTAAGA
R
CACTGGATGGAGATGATGGCGAATGACACAGTAATCATAAGACAGGCAGGGGCTACCCAGGCCTGTTAACAGGAGGGCCATTC
CCTCACATCCTCTCTGC / Celera SNP ID: hCV8840562 / Public SNP ID: rs668034 /
SNP Chromosome Position: 220488716 / SNP in Genomic Sequence: SEQ ID NO: 419 /
SNP Position Genomic: 6424 / Related Interrogated SNP: hCV2915511 / SNP
Source: dbSNP; Celera; HapMap; ABI_val; HGBASE / Population(Allele,Count):
Caucasian (G,2|A,118) / SNP Type: INTERGENIC;UNKNOWN /
Context (SEQ ID NO: 2250): /
GTTTTGGACTCAGTGACTTTTCCATTCTCCACAAAAACATTCTTCAGTTTATTAGTTACTATAATTACTTGTGCACTTGCTTT
CAATTCCACAGACATTT
R
TTGAGCTCTTAGAACGTGCCAGGATGTATGTGAGAAGATGGGGGCAGGGATCCAAGGATGAATCAGATGTGCTCCTGCCTCCA
AGGAGCTTATGGTCCAG / Celera SNP ID: hCV11470250 / Public SNP ID: rs678276 /
SNP Chromosome Position: 220485452 / SNP in Genomic Sequence: SEQ ID NO: 419 /
SNP Position Genomic: 3160 / Related Interrogated SNP: hCV2915511 / SNP
Source: dbSNP; HapMap; ABI_val; HGBASE / Population(Allele,Count): Caucasian
(A,1|G,119) / SNP Type: INTERGENIC;UNKNOWN /
Context (SEQ ID NO: 2251): /
AATTTAATAAATTGAATACTGATTTTCAAAACCCCAGTAAATCTCAGAGTGGTCTTTTCAGTGCGCAAAAGCCACCCTCAAGG
ACATAAGACATAAAATA
Y
TTTCCTAACCAGTGAGTCTTCAGCAGTGCAGGGGGAGAGAACGGAAGTGGGAAAGGGAGAAAGGAAAGTAAGATTTTAGTGTC
ATTGGATTGTCTTGTTC / Celera SNP ID: hCV31716873 / Public SNP ID: rs13403516 /
SNP Chromosome Position: 220514979 / SNP in Genomic Sequence: SEQ ID NO: 419 /
SNP Position Genomic: 32687 / Related Interrogated SNP: hCV2915511 / SNP
Source: dbSNP; HapMap / Population(Allele,Count): Caucasian (T,119|C,1) / SNP
Type: INTERGENIC;UNKNOWN //

Gene Number: 83 / Gene Symbol: SURF4 - 6836 / Gene Name: surfeit 4 /
Chromosome: 9 / OMIM NUMBER: 185660 / OMIM Information: // Genomic Sequence
(SEQ ID NO: 420): // / SNP Information /
Context (SEQ ID NO: 2252): /
TTTTGGTATATTTGCATTATATACTTATTGGCTGAGCATCCCAAATCTGAAAATCCAAAACCTGAAATGCTCCAATGAGCACT
TCCTTTGAGCGTCATGT
Y
GGTGCCCAAGATTTTTCCCATTTTGGAGCATTTCAGACTTTGGATTTGCAATGCTCAACCTGTAGAAGGAGCGTATACTAAAA
CCAACCAGAATGCACAT / Celera SNP ID: hCV25757025 / Public SNP ID: rs2269894 /
SNP Chromosome Position: 136232758 / SNP in Genomic Sequence: SEQ ID NO: 420 /
SNP Position Genomic: 14418 / Related Interrogated SNP: hCV25610857 / SNP
Source: Applera / Population(Allele,Count): Caucasian (C,17|T,19) African
American (C,2|T,36) total (C,19|T,55) / SNP Type: MICRORNA;UTR5;UTR3;INTRON /
SNP Source: dbSNP; Applera / Population(Allele,Count): Caucasian (T,70|C,46) /
SNP Type: MICRORNA;UTR5;UTR3;INTRON //

Gene Number: 84 / Gene Symbol: THBS2 - 7058 / Gene Name: thrombospondin 2 /
Chromosome: 6 / OMIM NUMBER: 188061 / OMIM Information: // Genomic Sequence
(SEQ ID NO: 421): // / SNP Information /
Context (SEQ ID NO: 2253): /
CCTGGCACGCAGAGTGCTGGAGCCAAGATAACTCTGCCTGTGCTGCAAATCACCCCTCAGGTAACAGGCACAGTGAGGGTCGT
GGTGGGGGTGACAGTTG
K
AGCTGGAATGAGGGTTGGTCTTAGAACACCCTTCTCCCTGCATTGATGATCAAATGTCTCCTTGTTCTCCTTCCTCACAGGAC
GTGCACAAGGACCCGGA / Celera SNP ID: hCV470708 / Public SNP ID: rs10945408 /
SNP Chromosome Position: 169644934 / SNP in Genomic Sequence: SEQ ID NO: 421 /
SNP Position Genomic: 39059 / SNP Source: Celera;dbSNP /
Population(Allele,Count): no_pop (G,-|T,-) / SNP Type: MISSENSE
MUTATION;ESE;INTRON;PSEUDOGENE //

Gene Number: 85 / Gene Symbol: TLE4 - 7091 / Gene Name: transducin-like

enhancer of split 4 (E(sp1) homolog, Drosophila) / Chromosome: 9 / OMIM NUMBER: / OMIM Information: // Genomic Sequence (SEQ ID NO: 422): // / SNP Information /
Context (SEQ ID NO: 2254): /
AATTTGTTCATAATAATTTTCCATAGATGTTTGTTAAATACTATGCTCTGGGATATTTTCTCATTTGGATATTCTCCGTACAA
TTTTGAGCCAAGATAGA
R
AAGGAAGCGCATGGACTGGTAGAAAATATACAGTTCTGGGTTTCACTTATAGTATGATATTAGGAATTTCATTTAGGTTTTTT
CATCTGTAGAATGCAAG / Celera SNP ID: hCV31671070 / Public SNP ID: rs11138314 /
SNP Chromosome Position: 82251896 / SNP in Genomic Sequence: SEQ ID NO: 422 /
SNP Position Genomic: 75018 / SNP Source: dbSNP; HapMap /
Population(Allele,Count): Caucasian (A,197|G,29) / SNP Type: INTRON //

Gene Number: 86 / Gene Symbol: TLR3 - 7098 / Gene Name: toll-like receptor
3 / Chromosome: 4 / OMIM NUMBER: 603029 / OMIM Information: // Genomic
Sequence (SEQ ID NO: 423): // / SNP Information /
Context (SEQ ID NO: 2255): /
TTAGAGTTGTCATCGAATCAAATTAAAGAGGTAAGAAGTAAGGTAAAATTATTTTGCATTCTGCCTTTAAGGTGGATAGTCCC
TATCTGTGTCACATACA
Y
AGGAATGTAATGCTCTCTAGCCTCTGCCTGTCTTCCAGCAATCCTTCCCACCTACTGCTTGGGGGCATTGGTGTCATCCTCCT
GAGAGCTCAGGCAGCCC / Celera SNP ID: hCV447407 / Public SNP ID: rs5743312 /
SNP Chromosome Position: 187000256 / SNP in Genomic Sequence: SEQ ID NO: 423 /
SNP Position Genomic: 19947 / SNP Source: Applera /
Population(Allele,Count): Caucasian (C,37|T,3) African American (C,35|T,3)
total (C,72|T,6) / SNP Type: INTRON / SNP Source: dbSNP; Celera; HapMap /
Population(Allele,Count): Caucasian (C,193|T,33) / SNP Type: INTRON /
Context (SEQ ID NO: 2256): /
ATAAATCTTGGTATTTAGGTTGTACCTAAAAATGATAACTTTTAGACTGTAATCAGAGGTAGATGCTATCAATTTGTTAGTGA
GGAGGGGAGGAGTTTTC
R
ATGCCACTTAGTTTACAATGTCTAATTGTTCAAATATACATCTAGGTTGTAAAGGAGACTCAACGGTAAGTTATAGTGACCAT
TGAAAAATGTAAAGGGG / Celera SNP ID: hCV79084 / Public SNP ID: rs1519312 / SNP
Chromosome Position: 187008844 / SNP in Genomic Sequence: SEQ ID NO: 423 /
SNP Position Genomic: 28535 / Related Interrogated SNP: hCV3230113 / SNP
Source: dbSNP; Celera; HGBASE / Population(Allele,Count): Caucasian
(G,86|A,140) / SNP Type: INTERGENIC;UNKNOWN /
Context (SEQ ID NO: 2257): /
TCACGTACTCAGGACATCTGTTTGCTTATGGTGTTATGTTACAGATGTCACAAATGAAAATCAGAGACATGTAGCCCTGAGCC
CAGTAACTATAAAGCGG
W
CTAGCTGAAGCTGGAGCCCACAAGAGGGCACTTTGCACCATGATGCACGCTTGTTTCTTGCCATCAGCTCTGCAATGAGTTCA
AGCTAATGCCATGTCCC / Celera SNP ID: hCV393058 / Public SNP ID: rs5743305 /
SNP Chromosome Position: 186989333 / SNP in Genomic Sequence: SEQ ID NO: 423 /
SNP Position Genomic: 9024 / SNP Source: dbSNP; Celera; HapMap /
Population(Allele,Count): Caucasian (T,153|A,73) / SNP Type:
INTERGENIC;UNKNOWN /
Context (SEQ ID NO: 2258): /
TATTTTCACAAACCATTCTTTATTCTTTGTGTGCATTGGGCAAGAGTCATCAGACTTTTCAATGGTGGAACTCTTCCACTTTT
GTTTGAGGACTTCTGGG
Y
AGACAGGGTTGGCTTATTTAGCTTCCTTTTCTCTTAGGTCAGTTCCCCAGGAAACAGGTTTTGGGATGGAGGTTTGCATGAAG
GAAGTTTCAGGGGTGCT / Celera SNP ID: hCV11786002 / Public SNP ID: rs4862633 /
SNP Chromosome Position: 187009972 / SNP in Genomic Sequence: SEQ ID NO: 423 /
SNP Position Genomic: 29663 / Related Interrogated SNP: hCV3230113 / SNP
Source: dbSNP; Celera; HapMap; HGBASE / Population(Allele,Count): Caucasian
(T,50|C,176) / SNP Type: INTERGENIC;UNKNOWN /
Context (SEQ ID NO: 2259): /
TCTTAGGTCAGTTCCCCAGGAAACAGGTTTTGGGATGGAGGTTTGCATGAAGGAAGTTTCAGGGGTGCTCTGATTTATAAGTG
AGTGAAATAAGCTGGAT
Y
GGGAAGAGAGAGAATAATTAACTCTGATGCAATTCACAATTGCAGCTGAGTTCTTAGCCAGCCCCATGGGGAGCTCTGGAGCT
GGAATGACCCTAGAGAA / Celera SNP ID: hCV11786003 / Public SNP ID: rs4608848 /

SNP Chromosome Position: 187010104 / SNP in Genomic Sequence: SEQ ID NO: 423 / SNP Position Genomic: 29795 / Related Interrogated SNP: hCV3230113 / SNP Source: dbSNP; Celera; HapMap; HGBASE / Population(Allele,Count): Caucasian (C,85|T,141) / SNP Type: INTERGENIC;UNKNOWN / Context (SEQ ID NO: 2260): /
CAGATTTTGGATGGGCCAGCGCATGAGGTGGGGAAGCTCAAATACCAGACCAAGGAAGTGAGTTAGTGGAAATGAGGAGCCAT AAAGATGCTTTTATGGT
R
GAAGGAGCATGGGTTGAAGAGCTGGGTTTTAGGAAGAGTGAGATAGTGGGATCGATTAGGAACAGACTGAAGTTCTTGAGATG CCCCTATTTACATAGGA / Celera SNP ID: hCV11786022 / Public SNP ID: rs2090628 / SNP Chromosome Position: 187015824 / SNP in Genomic Sequence: SEQ ID NO: 423 / SNP Position Genomic: 35515 / Related Interrogated SNP: hCV3230113 / SNP Source: dbSNP; Celera; HGBASE / Population(Allele,Count): Caucasian (G,68|A,156) / SNP Type: INTERGENIC;UNKNOWN / Context (SEQ ID NO: 2261): /
AGATGGTATACTACCTTCTCCTCTCAGAAATACTCAATGCGTACCAGCATATTGAAAAACTAAGATGCCGGCTATAAAGAAAG CTATTTAACCTTCATTA
Y
TGAGTCAGTTGCCAAACCAACTTGATTATAGAACGCTTCTAGTGGTAACTGTTATCAGCATCGTGACAAAGGCATGTTCTGCA TAGCACAATTTTGGGAA / Celera SNP ID: hCV12066105 / Public SNP ID: rs1519309 / SNP Chromosome Position: 187015089 / SNP in Genomic Sequence: SEQ ID NO: 423 / SNP Position Genomic: 34780 / Related Interrogated SNP: hCV3230113 / SNP Source: dbSNP; HapMap / Population(Allele,Count): Caucasian (C,69|T,157) / SNP Type: INTERGENIC;UNKNOWN / Context (SEQ ID NO: 2262): /
GATGCCGGCTATAAAGAAAGCTATTTAACCTTCATTACTGAGTCAGTTGCCAAACCAACTTGATTATAGAACGCTTCTAGTGG TAACTGTTATCAGCATC
S
TGACAAAGGCATGTTCTGCATAGCACAATTTTGGGAACACTCTAGGTAAACTTAATTATTCTAGTTCTAGCACCTCCTAAGAA ATTCTCCTTTTCATACC / Celera SNP ID: hCV12066106 / Public SNP ID: rs1914926 / SNP Chromosome Position: 187015152 / SNP in Genomic Sequence: SEQ ID NO: 423 / SNP Position Genomic: 34843 / Related Interrogated SNP: hCV3230113 / Related Interrogated SNP: hCV11786258 / Related Interrogated SNP: hCV3230096 / SNP Source: dbSNP; HapMap; HGBASE / Population(Allele,Count): Caucasian (G,28|C,92) / SNP Type: INTERGENIC;UNKNOWN / Context (SEQ ID NO: 2263): /
TCCTACTTCATGCATACACAACAATTAACTTAGGAGAGGTCACACACCTTCACATAGAAGCTAACACCATAAAGCTTTTTAGA GGAGAGTATCTAAGTAA
W
GGGAGTAGGAAAATTTCTTAGAGGAGACACAGAAATGATAAAAATAAAAGAGAAGATGATAAACTAGATTTCATCATAATTAA GAAGACATCAGTAAGAA / Celera SNP ID: hCV27310253 / Public SNP ID: rs13108688 / SNP Chromosome Position: 186994832 / SNP in Genomic Sequence: SEQ ID NO: 423 / SNP Position Genomic: 14523 / Related Interrogated SNP: hCV3230113 / SNP Source: dbSNP; Celera; HapMap / Population(Allele,Count): Caucasian (T,96|A,24) / SNP Type: INTRON / Context (SEQ ID NO: 2264): /
GGAGACGCAGAGGCAAACCATATCGGCTATGTATTCAACATATATTTATTTCTTCAGAGCCAACAAAGTGCCAGGTCTTGTTC TAGGCACAGACAACACC
R
TATGGAATAAGAACTATGTCCTCTACTATTATAGCTTCACATTCTAGTGAGAAGCAATAATTAATAATAAATAATACACAATA TTGGATAATTTCCATCA / Celera SNP ID: hCV27310255 / Public SNP ID: rs7657186 / SNP Chromosome Position: 186994039 / SNP in Genomic Sequence: SEQ ID NO: 423 / SNP Position Genomic: 13730 / Related Interrogated SNP: hCV3230113 / SNP Source: dbSNP; Celera; HapMap / Population(Allele,Count): Caucasian (G,191|A,35) / SNP Type: INTRON / Context (SEQ ID NO: 2265): /
GTGAATGCTGAGCAGAAGTTAGCCCGTGGCCAGGGGAGGTCAGAGGCTGGAGTAAGAGGTGGGCGCTGCAGGAACCGAGTAAG GAAGGACTCGTGCATTA
S
AGGAAGGGGGAGAAATGCTCTGCCCATGGGATGTGGTGAAGGGTGAGCGGAGATGGAGTAAAGAACTGCATCTGGCCGGGTGC AGCGGCTCAGGCCTATA / Celera SNP ID: hCV27482764 / Public SNP ID: rs3775292 / SNP Chromosome Position: 187003025 / SNP in Genomic Sequence: SEQ ID NO: 423 / SNP Position Genomic: 22716 / SNP Source: dbSNP; HapMap /

Population(Allele,Count): Caucasian (C,41|G,185) / SNP Type: UTR5;INTRON //

Gene Number: 87 / Gene Symbol: TUBA4A - 7277 / Gene Name: tubulin, alpha 4a / Chromosome: 2 / OMIM NUMBER: / OMIM Information: // Genomic Sequence (SEQ ID NO: 424): // / SNP Information /
Context (SEQ ID NO: 2266): /
GCCCTCAATGTGGACCTGACAGAGTTCCAGACCAACCTGGTGTCCTACCTCACATCCACTTCCCCCTGGCCACCTATGCACCA
GTCATCTCTGCAGAAAA
R
GTATACCACGAGCAGCTGTTGGTGGCAGAGATTACCAATGCCTGCTTTGAGCCTGCCAACCAGATGGTGAAGTGTGATCCCCG
GCACGGCAAGTACATGG / Celera SNP ID: hCV26175114 / Public SNP ID: rs3731892 /
SNP Chromosome Position: 220136351 / SNP in Genomic Sequence: SEQ ID NO: 424 /
SNP Position Genomic: 31350 / SNP Source: dbSNP; Celera; HapMap /
Population(Allele,Count): Caucasian (G,29|A,197) / SNP Type: MISSENSE
MUTATION;UTR3;INTRON //

Gene Number: 88 / Gene Symbol: LUZP1 - 7798 / Gene Name: leucine zipper protein 1 / Chromosome: 1 / OMIM NUMBER: 601422 / OMIM Information: //
Genomic Sequence (SEQ ID NO: 425): // / SNP Information /
Context (SEQ ID NO: 2267): /
GGCTTGGAAGTCCCCTTCCACGCTTTACTGTGCTCCTGAGCAGCCGGGGGGTAGCGACTCAGCACTGAGGGCTGCTCCCTAGA
CTTCTTCCCTTCGCTCT
K
GGAGGAGCCGGGTACAAACCGGTCTTCAGTTTTCTTTGTCTCCTGAGTCCCACTGTCTGTACTCACCCCCATGTGAGAAGCTT
TTGCTGCCCTCCTGTTG / Celera SNP ID: hCV2494846 / Public SNP ID: rs3765407 /
SNP Chromosome Position: 23419374 / SNP in Genomic Sequence: SEQ ID NO: 425 /
SNP Position Genomic: 18858 / SNP Source: Applera / Population(Allele,Count):
Caucasian (G,8|T,30) African American (G,15|T,23) total (G,23|T,53) / SNP
Type: MISSENSE MUTATION;TFBS SYNONYMOUS / SNP Source: dbSNP; Celera; HapMap;
HGBASE / Population(Allele,Count): Caucasian (G,36|T,190) / SNP Type:
MISSENSE MUTATION;TFBS SYNONYMOUS //

Gene Number: 89 / Gene Symbol: DUSP11 - 8446 / Gene Name: dual specificity
phosphatase 11 (RNA/RNP complex 1-interacting) / Chromosome: 2 / OMIM NUMBER:
603092 / OMIM Information: // Genomic Sequence (SEQ ID NO: 426): // / SNP
Information /
Context (SEQ ID NO: 2268): /
GTCTTAACCTGTGTTGGGCGTTGCACCGGGCGACCTCAGTTTCTTCCTGTACAAGGAAAAGTACTGACCAAAATGAGTTCTAC
ATACATTTCCGCTGCTG
S
AGATTTCTTTGGTTTTTGAGACAGAGTCTTGCTCTGTTACTCAGGCTGGAGTGCTGTGGTGCAATCTCGGCTCACTGCAACCT
CCGCGTCCCGGTTCAAG / Celera SNP ID: hCV303807 / Public SNP ID: rs17350188 /
SNP Chromosome Position: 73964842 / SNP in Genomic Sequence: SEQ ID NO: 426 /
SNP Position Genomic: 7233 / Related Interrogated SNP: hCV11541681 / SNP
Source: dbSNP; Celera; HapMap / Population(Allele,Count): Caucasian
(G,80|C,146) / SNP Type: TRANSCRIPTION FACTOR BINDING SITE;INTERGENIC;UNKNOWN /
Context (SEQ ID NO: 2269): /
ATGGGCTTAAAGAATGTGAACGATGTGCTCACAGAGGCCACAGAAGAGAAATTATAGCCAGGAGAACAACCTGAAAGACAAAG
GACACGGTGGCATAAGC
R
CATGTAACACAATGTACTCAGGAAATGGCTGGCATCCTGAGATATGGAGTGGAATACAGTACAGGGCTTTGTAAACTCAGCTT
GGAGTCAGATCACAGAA / Celera SNP ID: hCV505733 / Public SNP ID: rs11126416 /
SNP Chromosome Position: 73973877 / SNP in Genomic Sequence: SEQ ID NO: 426 /
SNP Position Genomic: 16268 / Related Interrogated SNP: hCV11541681 / SNP
Source: dbSNP; Celera; HapMap; ABI_val / Population(Allele,Count): Caucasian
(A,38|G,82) / SNP Type: INTERGENIC;UNKNOWN /
Context (SEQ ID NO: 2270): /
CTCCTCAGAAGTTGCCTGCCCAGGGGCTCTTTAAAACAGACTAGGTGGATTTCCATGTCTGCCCCTGCTTGTTCTTTTTGCCT
CGCCAGTTGTCTTGCTC
Y
GATTTCTTCCCAAGTGGGCTTTCATGATGTATTCATGATGAGCCTTCATGACGTACTCTACAAGACTAGCCTTTTAGCAAGAA
CTTTTATATCTCTGTTA / Celera SNP ID: hCV2050088 / Public SNP ID: rs2272178 /
SNP Chromosome Position: 73977609 / SNP in Genomic Sequence: SEQ ID NO: 426 /

SNP Position Genomic: 20000 / Related Interrogated SNP: hCV11541681 / SNP Source: dbSNP; Celera; HapMap; ABI_Val; HGBASE / Population(Allele,Count): Caucasian (C,38|T,82) / SNP Type: INTERGENIC;UNKNOWN / Context (SEQ ID NO: 2271): / TCAGTGGGAAAACAATATCTTTGGAAAAACAATAATCCCCCCTATCCCAACCCAAGCAAATGGCTGGGCTATTCTTGTCTATA TTTCTGACCTACCACCC R ACAAGGGCAGATACTCACTTTGTTAAAGTGCTGCTCACAGCAGAAGAAAGGCATGTAGTCACAGAAAAGGCTAGGGAAGAAGC AAATCGGCTCCATCTGG / Celera SNP ID: hCV2050091 / Public SNP ID: rs35791379 / SNP Chromosome Position: 73979482 / SNP in Genomic Sequence: SEQ ID NO: 426 / SNP Position Genomic: 21873 / Related Interrogated SNP: hCV11541681 / SNP Source: dbSNP; Celera / Population(Allele,Count): Caucasian (G,80|A,146) / SNP Type: INTERGENIC;UNKNOWN / Context (SEQ ID NO: 2272): / TGGCTAGATAGGACAATATGCTCTGGGATTCTCACAGGTCCAGGGCCATTTGGCAAAAACCAACGGACAACCTGGAAATTTCC CAAATGTGTTGCATCAA Y GGACCAAGTCAGTTTTCCACTGGTATGACCATCCCCTCTCCATTTTTGTTCCCCAAGTTAGTTTAAAAAATGTTATCTGACAT GTTGAGATCCTAACAAA / Celera SNP ID: hCV2050092 / Public SNP ID: rs12624267 / SNP Chromosome Position: 73986931 / SNP in Genomic Sequence: SEQ ID NO: 426 / SNP Position Genomic: 29322 / Related Interrogated SNP: hCV11541681 / SNP Source: dbSNP; Celera; HapMap; ABI_Val / Population(Allele,Count): Caucasian (C,80|T,146) / SNP Type: TRANSCRIPTION FACTOR BINDING SITE;INTERGENIC;UNKNOWN / Context (SEQ ID NO: 2273): / AATCTTTTCTTCTACTTCTCACTTCTTAACCAATATAGAAGACAAAAATAACATTTTAATTTTATATATTTCTCATTCAAATT AAAAAAGAAAGATGAGC Y AGTTCAGTTCATATTTTGCACTAAAAACTAATTTAACATTCCAAAGTTAAATTATTTGTGATCAACAAAAGGAAAAAACATCTTA CCCTATTAGCACCATCT / Celera SNP ID: hCV2050096 / Public SNP ID: rs2116367 / SNP Chromosome Position: 74000701 / SNP in Genomic Sequence: SEQ ID NO: 426 / SNP Position Genomic: 43092 / Related Interrogated SNP: hCV11541681 / SNP Source: dbSNP; Celera; HapMap; HGBASE / Population(Allele,Count): Caucasian (C,38|T,82) / SNP Type: INTRON / Context (SEQ ID NO: 2274): / TATTATCCCAAGGGCCTGGGTAGTCAGGAAATCACAGAATTTTAAAGCTGCAAGGAACTTATGCAAATAGTAAAATTTTTTTA AATAACATTAATTGAGG K CTAATATTATGGGCATGATCATATTGCAAACATTTTACTTGTAATATTTTCTTTAATCCTCAAAACTACCCTATGAAATTGAC ACTATTATTAGTAGTAG / Celera SNP ID: hCV11537012 / Public SNP ID: rs12992607 / SNP Chromosome Position: 73959960 / SNP in Genomic Sequence: SEQ ID NO: 426 / SNP Position Genomic: 2351 / Related Interrogated SNP: hCV11541681 / SNP Source: dbSNP; Celera; HapMap / Population(Allele,Count): Caucasian (G,38|T,82) / SNP Type: INTRON / Context (SEQ ID NO: 2275): / AAACTTAAATGTGTGCGGCAGGAGGAAAGTAAACAAAAACCAAGCTGACTAACCACCTTATTACAAGGCCACCATGACAAGAC TAGAGCGTCCATCATCA R CTTTTAGTGCCTCAATGTTAAGTATGGACAAATGAGCAGTAAACATGGTCATGAGGTATTTCAAGAACACTTCTAAGAGCAGA GACAGAGGTATAAACAA / Celera SNP ID: hCV31840129 / Public SNP ID: rs11126417 / SNP Chromosome Position: 73974315 / SNP in Genomic Sequence: SEQ ID NO: 426 / SNP Position Genomic: 16706 / Related Interrogated SNP: hCV11541681 / SNP Source: dbSNP / Population(Allele,Count): Caucasian (G,80|A,146) / SNP Type: TFBS SYNONYMOUS;INTERGENIC;UNKNOWN / Context (SEQ ID NO: 2276): / GATAGGCCGGCTCGGGGTGGCCATCTGTTTCCGGGTCCTGGTAGGAGGGGTGCTTCCGCCCATGGTCCCGCCCATTCTTCCGC CTCCCCAACCTGGGTCC Y GTCAACGGACGCGAAGGAGAACAGGGGCTGTATATCACTTCCGGCGAAGGAAATGGAAGAATCTATGGGCTGGGACCGGAAGC TGGGGTCTGGTTTTGAG / Celera SNP ID: hCV31840134 / Public SNP ID: rs11894953 / SNP Chromosome Position: 73964631 / SNP in Genomic Sequence: SEQ ID NO: 426 / SNP Position Genomic: 7022 / Related Interrogated SNP: hCV11541681 / SNP Source: dbSNP / Population(Allele,Count): Caucasian (C,80|T,146) / SNP Type: INTERGENIC;UNKNOWN /

Context (SEQ ID NO: 2277): /
AAAAAAAAAAAAAAACAAAAAACAACTATTATCCCAAGGGCCTGGGTAGTCAGGAAATCACAGAATTTTAAAGCTGCAAGGAAC
TTATGCAAATAGTAAAA
W
TTTTTTAAATAACATTAATTGAGGGCTAATATTATGGGCATGATCATATTGCAAACATTTTACTTGTAATATTTTCTTTAATC
CTCAAAACTACCCTATG / Celera SNP ID: hCV31840136 / Public SNP ID: rs12713795 /
SNP Chromosome Position: 73959935 / SNP in Genomic Sequence: SEQ ID NO: 426 /
SNP Position Genomic: 2326 / Related Interrogated SNP: hCV11541681 / SNP
Source: dbSNP; HapMap / Population(Allele,Count): Caucasian (T,38|A,82) / SNP
Type: INTRON /
Context (SEQ ID NO: 2278): /
TGTGCTAACACGAGGAAATGTAAGGCTATGGCCTGGCATTAATATAACCCTTCACAACCCAGGTCACAGAAAATCTTTTACTA
TATATCAGACTGTTCAA
Y
GAACTTTTGAATACACTGCTCCTCCTCTCCTGAAGGAATTTCCCTTGGCAGCCCCCCAAATTTTGGCTATTAATAGCCACTAT
ACTGAGGAAGGTTTTTA / Celera SNP ID: hCV31840120 / Public SNP ID: rs12713798 /
SNP Chromosome Position: 73986421 / SNP in Genomic Sequence: SEQ ID NO: 426 /
SNP Position Genomic: 28812 / Related Interrogated SNP: hCV11541681 / SNP
Source: dbSNP; HapMap / Population(Allele,Count): Caucasian (C,80|T,146) /
SNP Type: INTERGENIC;UNKNOWN /
Context (SEQ ID NO: 2279): /
TGAAACTCCCTCTCAAAAAAAAATAAATAAATAAAAATTAAAATTAAAAAAAATATATATATATATATATACATATATATATG
AAGGACATTCTATTTCT
R
GGAGACACTGGAACAGGGCCCTGGCTGGTCTGGTTAGTCTCTCTCTATCACAGGATGCTACATTCCCAGCACATTCTACAGTT
ATCCTAGAGAACCACAA / Celera SNP ID: hCV31840132 / Public SNP ID: rs2421676 /
SNP Chromosome Position: 73966087 / SNP in Genomic Sequence: SEQ ID NO: 426 /
SNP Position Genomic: 8478 / Related Interrogated SNP: hCV11541681 / SNP
Source: dbSNP; HapMap; ABI_Val / Population(Allele,Count): Caucasian
(G,80|A,146) / SNP Type: INTERGENIC;UNKNOWN //

Gene Number: 90 / Gene Symbol: SORBS2 - 8470 / Gene Name: sorbin and SH3
domain containing 2 / Chromosome: 4 / OMIM NUMBER: / OMIM Information: //
Genomic Sequence (SEQ ID NO: 427): // / SNP Information /
Context (SEQ ID NO: 2280): /
ATGCGATGATTGACATGCTTCTTATAATGTAGATTTGAAGGGGAAAAGTGGCAATGAAATGCCCAGGTTTGGGAAACTTAAAG
ATTAGACTGAACATCTC
Y
AGGTTTTTCTACTATAAATCCAAGTTCTGGAGATTCGATTGCATCTGCTCCTAATCCAGGGAGATCTCTGCCGTAGCTTTCTC
CACAGCTCCGCCCCTTT / Celera SNP ID: hCV1474481 / Public SNP ID: rs7693361 /
SNP Chromosome Position: 186694576 / SNP in Genomic Sequence: SEQ ID NO: 427 /
SNP Position Genomic: 197978 / Related Interrogated SNP: hCV15968043 / SNP
Source: dbSNP; Celera; HapMap / Population(Allele,Count): Caucasian
(T,152|C,74) / SNP Type: INTRON /
Context (SEQ ID NO: 2281): /
TGGGATATTTTTAGCATAGGCTGGATGACACCTCCATGGCAGGTACGTCAGCCACCTCTGCTTTAGTCACCAGCATAGGGCTG
TGCTTAGCAGAGAGCCT
R
GCATAGAGAGGATGCTTATTAAATATTGGGTAAAAGAATAAAATGATAAATAAGGGCCACTATAAAATGAATGTTACACAAAA
ACGTGTTTTCCCCAACA / Celera SNP ID: hCV1474587 / Public SNP ID: rs2648107 /
SNP Chromosome Position: 186794181 / SNP in Genomic Sequence: SEQ ID NO: 427 /
SNP Position Genomic: 297583 / SNP Source: dbSNP; Celera; HapMap; HGBASE /
Population(Allele,Count): Caucasian (A,20|G,94) / SNP Type: INTRON /
Context (SEQ ID NO: 2282): /
TCTCAGAGTTACGATCCCATGGGTCAAGCAGGTATTAGGCCCAAATGAAATTTTAATTACAAAACATATATGCAATATAGAGT
CCGGGTGCAGGGTGACA
R
GGCGCTATAAGAAAGAACTAACTAGGGACTCAATCCATATCACAGAGCATAGAAGTCTCCTCTGGCAAGACAGGACTTAAGCT
GAGAGGTCAAAGATAAG / Celera SNP ID: hCV7750713 / Public SNP ID: rs4862596 /
SNP Chromosome Position: 186846999 / SNP in Genomic Sequence: SEQ ID NO: 427 /
SNP Position Genomic: 350401 / Related Interrogated SNP: hCV22272267 / SNP
Source: dbSNP; Celera; HapMap; HGBASE / Population(Allele,Count): Caucasian
(G,134|A,92) / SNP Type: INTRON /

Context            (SEQ ID NO: 2283): /
AAGAAATGGAAAGTATTTTAATTGATCAGGAGTTCAAGACTAGCCTGGCTGATACAACAAGACCCTGTCTCTGAAAAAACAAA
GAAGGACGAAGTAGAAA
R
TATATTTCTGAGAAGATACAGGTTATAGAGATCCTCAGTTCACAAAGCAATGGATATTTACTTTTCCACTATACAGCCCTATC
AGCCGACCACAACGCTG / Celera SNP ID: hCV7750737 / Public SNP ID: rs13140248 /
SNP Chromosome Position:  186842789 / SNP in Genomic Sequence:  SEQ ID NO: 427 /
 SNP Position Genomic:  346191 / Related Interrogated SNP:  hCV22272267 / SNP
Source:  dbSNP; Celera; HapMap / Population(Allele,Count):  Caucasian
(G,132|A,90) / SNP Type:  INTRON /
Context            (SEQ ID NO: 2284): /
ATTATTTAACCTTAAGAAGGAAGGAAATCCTGGCACATGTCGCAACATGGATGAATTTTGTGGACAGTGTGCATGGTGAAGTA
AGCCAGACACAGAAGGA
M
AAATCCTGTATGATTCCAATCATAATGAAGTACCTGGAGAAGTCAAACTCGCAGAGACAGAAAGTAGAATGGTGGTTGCCAGA
GGCTAGGGTGAGGTGGC / Celera SNP ID: hCV7750905 / Public SNP ID: rs6833156 /
SNP Chromosome Position:  186813831 / SNP in Genomic Sequence:  SEQ ID NO: 427 /
 SNP Position Genomic:  317233 / SNP Source:  dbSNP; Celera; HapMap; ABI_val /
Population(Allele,Count):  Caucasian (A,64|C,160) / SNP Type:  INTRON /
Context            (SEQ ID NO: 2285): /
CTTCTTTCCACCCAATCTGTGCTTCTGTCCTCCTCCTCCGCCTGACAGTTTTACGCCCTGAACATCTATATAATTTATTGTTA
CTGTTACTCCATTGGAT
R
TAATGAGGCTCTCATCACTCACTTGCCACCTGAGCTATTGCAGTTTCCTCCTGACACTCATTCCACCTCCTGCCTTCCTTCAT
TCCACTCACTCCCCCAC / Celera SNP ID: hCV7750941 / Public SNP ID: rs7656292 /
SNP Chromosome Position:  186810921 / SNP in Genomic Sequence:  SEQ ID NO: 427 /
 SNP Position Genomic:  314323 / SNP Source:  dbSNP; Celera; HapMap /
Population(Allele,Count):  Caucasian (A,27|G,85) / SNP Type:  INTRON /
Context            (SEQ ID NO: 2286): /
TTCCTTTCCATTTTCTTAGAAAATCTTTTGTAGACTTTCTGTATTTCTTAACATTCAGCAATTGTTATAGTTCGCAGAGTGCT
TTATGGATTACAGAATG
R
TTTCATTTAGCTTTTTCCTTTAAAAAATTGACATGCTCATATGGCAGATATTATTATTCTCATTTCTAGGGGAGGAGAGGAAGAC
CTAGGAAATATAGTAAC / Celera SNP ID: hCV11684747 / Public SNP ID: rs2034787 /
SNP Chromosome Position:  186843924 / SNP in Genomic Sequence:  SEQ ID NO: 427 /
 SNP Position Genomic:  347326 / SNP Source:  dbSNP; HapMap; HGBASE /
Population(Allele,Count):  Caucasian (G,56|A,166) / SNP Type:  TRANSCRIPTION
FACTOR BINDING SITE;INTRON /
Context            (SEQ ID NO: 2287): /
AACCATAAATCCCATATTTCTGATCATTCCCAGTTTTGAGTATTCTGTCTCTTTGTCAAGTCACATGTCCCAATTTTAGGGCT
AGAAAACATGGTCATCA
Y
GTTGATTAGAAATGCAATTTACAGAAGATTTCCCTTTCCGTCTGCTTCTAAGACTATGCAAGAGTCTCCTTTCACCTCCCACA
CTCAGCTTCTGCTCTCA / Celera SNP ID: hCV15966712 / Public SNP ID: rs2278939 /
SNP Chromosome Position:  186857890 / SNP in Genomic Sequence:  SEQ ID NO: 427 /
 SNP Position Genomic:  361292 / SNP Source:  dbSNP; HapMap; ABI_val; HGBASE /
Population(Allele,Count):  Caucasian (C,52|T,174) / SNP Type:  INTRON /
Context            (SEQ ID NO: 2288): /
CTTGATAAACATGCCTTTTAATTTATCTTACACAAGGTGCTGAGATTTTTCTTTTAATGATAAAACAGGGTTGGCTCTGAAAT
ACCAACTGTCCACAAAC
Y
GGTTTCAAATGGCATGATGTAACTAGGCTTTTTGCCATGGGTCTCTGTGAACTCTCGCCCAGGAAATGCAGGTTTTGATATGT
ACAAATTGCCAAAGGAA / Celera SNP ID: hCV16054124 / Public SNP ID: rs2648117 /
SNP Chromosome Position:  186787096 / SNP in Genomic Sequence:  SEQ ID NO: 427 /
 SNP Position Genomic:  290498 / SNP Source:  dbSNP; HapMap; HGBASE /
Population(Allele,Count):  Caucasian (T,64|C,162) / SNP Type:  INTRON /
Context            (SEQ ID NO: 2289): /
GGCTTTCTAGATCTGAGTTTACATTTCCCTGTGCAGAATACCTTGACAAATGGCAGCTTTCTGTGACTGATGACAGTGCCTGG
TTTGTATGAGATGTGAC
Y
TCCTTCTCTCAGGACTGTCATCCATCTCTAATGTCATACTGCTCATCAAAGCAAACCATACAGACTTTCTTTGGAAAACACTG
AAATTGGCATGCATTTA / Celera SNP ID: hCV16259100 / Public SNP ID: rs2603723 /
SNP Chromosome Position:  186793054 / SNP in Genomic Sequence:  SEQ ID NO: 427 /

SNP Position Genomic: 296456 / SNP Source: dbSNP; HapMap; HGBASE / Population(Allele,Count): Caucasian (T,40|C,186) / SNP Type: INTRON / Context (SEQ ID NO: 2290): /
CACATAATAAACACACACCTTGGTAAACCACTTGGAGGAAGGGGCCCCAAGATCATGAGGAAATGAGCTGACTCAGAAGCAGC
TGAACAGCAATGCTTGC
R
GGATTAGGAGGCATTTGTCAGAGGGGCAGCTACGAAGGGGGCAGTGGAGGGAAAAAAGGATGGATCCCCTGATATACTGAAGA
AATCTTGCAAAGGACAG / Celera SNP ID: hCV27310160 / Public SNP ID: rs10022762 /
SNP Chromosome Position: 186853358 / SNP in Genomic Sequence: SEQ ID NO: 427 /
SNP Position Genomic: 356760 / SNP Source: dbSNP; Celera; HapMap /
Population(Allele,Count): Caucasian (G,21|A,89) / SNP Type: INTRON /
Context (SEQ ID NO: 2291): /
TGTCTCAGGGAACGGCAGATGTCTGAAGCCAGGCAAGGAACACAGTTGGTGTAATTCACATATGGGATATTCTTAGCATTGAA
TACTGTTTTCTTTCAGT
Y
GCTTAGGGTATAGTATGGTTCTTTGTCAAAAGATGACAAAGCGCTGCCTTGGTTATTCAGTTTGTGAAATGAATCACAATGCT
TTAATAGAATGAAATCT / Celera SNP ID: hCV27310199 / Public SNP ID: rs13112022 /
SNP Chromosome Position: 186775688 / SNP in Genomic Sequence: SEQ ID NO: 427 /
SNP Position Genomic: 279090 / SNP Source: dbSNP; Celera; HapMap /
Population(Allele,Count): Caucasian (C,87|T,33) / SNP Type: INTRON /
Context (SEQ ID NO: 2292): /
TCAGAATTTAACATTATCCAAATTCCCTTCCAGACTTTTCTCAGGTGCGTGGTTTCAGAGTAGGACTCGTTTCTTATATACAA
TGCGGTGTTTGATTTTT
W
AAAAAAAAAAAAACTTCCCTTGGAAAATACTAAAAACTATTATGCATTGAGTCATTTAATATTCAGAATAACCTCACAAAGCAGG
TGTTAACAACTTCCATT / Celera SNP ID: hCV29640638 / Public SNP ID: rs75112415 /
SNP Chromosome Position: 186832114 / SNP in Genomic Sequence: SEQ ID NO: 427 /
SNP Position Genomic: 335516 / SNP Source: dbSNP; HapMap /
Population(Allele,Count): Caucasian (T,31|A,89) / SNP Type: INTRON //

Gene Number: 91 / Gene Symbol: VGLL4 - 9686 / Gene Name: vestigial like 4
(Drosophila) / Chromosome: 3 / OMIM NUMBER: / OMIM Information: // Genomic
Sequence (SEQ ID NO: 428): // / SNP Information /
Context (SEQ ID NO: 2293): /
AGTACATCCACACATTTTACATTTCATGCCAGGGTTCGTGCTGTTTCAAACTCAATACCAACCAGGCTGAAATCATCCTCCTT
ACTCTGGGCTTCTGCTC
R
TCCTCTAACACAGCAAAGGCTGCATCCCTAGACTGTGATTCATATTTAAATGTCTTTTTCTCCTCTGCTGGGCAGTGTGAGCC
TCAGGGGCAGAACCATA / Celera SNP ID: hDV70820190 / Public SNP ID: rs17035071 /
SNP Chromosome Position: 11710471 / SNP in Genomic Sequence: SEQ ID NO: 428 /
SNP Position Genomic: 122927 / SNP Source: dbSNP / Population(Allele,Count):
Caucasian (G,184|A,42) / SNP Type: INTRON //

Gene Number: 92 / Gene Symbol: RABGAP1L - 9910 / Gene Name: RAB GTPase
activating protein 1-like / Chromosome: 1 / OMIM NUMBER: / OMIM Information:
// Genomic Sequence (SEQ ID NO: 429): // / SNP Information /
Context (SEQ ID NO: 2294): /
TAAGCGATATTTCTTCAATATTTCCCTTCTTGGACTTCACATTGATGATAGTTTTTAGGTTGTAATAATTTAGAGTTTGGATG
GTCTACATTTACCATCT
R
CAGTTGTTTACTCCCTCTTTTTTTAATGCACAAGTGATGAAAATGTTGTCATGTAATATCTAAACAATGATAAATACTTAGAC
TATATATAAAAATATAT / Celera SNP ID: hCV25932979 / Public SNP ID: rs16846809 /
SNP Chromosome Position: 174219398 / SNP in Genomic Sequence: SEQ ID NO: 429 /
SNP Position Genomic: 100764 / Related Interrogated SNP: hCV16180170 /
Related Interrogated SNP: hCV8911768 / SNP Source: Applera /
Population(Allele,Count): Caucasian (A,39|G,1) African American (A,33|G,3)
total (A,72|G,4) / SNP Type: INTRON / SNP Source: dbSNP; HapMap /
Population(Allele,Count): Caucasian (A,209|G,17) / SNP Type: INTRON /
Context (SEQ ID NO: 2295): /
TCCCTTTTGAAATACATTATATTGAACTGACTGAAAAAGGGAGAAAAACATGTTTCAGTGGTTGAAGAACATGGTAGATGAAG
TAGTTCATTTTGAATGG
Y
TTTAGTTCCTTAACAAAAAAGTCAGTGGTGAAGTTGCATATTTTAGTATAATATTTAGGAGCTGCAGTTGGGGAATTTGAAAT

GGCTTTTGTGAATTATT / Celera SNP ID: hCV30205817 / Public SNP ID: rs10489254 / SNP Chromosome Position: 174197801 / SNP in Genomic Sequence: SEQ ID NO: 429 / SNP Position Genomic: 79167 / Related Interrogated SNP: hCV16180170 / Related Interrogated SNP: hCV8911768 / SNP Source: dbSNP; HapMap / Population(Allele,Count): Caucasian (C,209|T,17) / SNP Type: INTRON / Context (SEQ ID NO: 2296): /
TTCACTACACTTATTTAAAGTTATATTTAGTAAAAACTCTAAGTAAAACATATCAATTCCAACAGTGTATTAAATAATAAATA
CATCTATGAATAATTAG
R
ATTTGTACTTTGAATACTAGCTTATAAGTCAAGATGCTTTTGGGTGCAAAAATAAATAAAACCAAAACCAGCTCCAACTTCAG
CTGGCTTAAACACCACA / Celera SNP ID: hDV70683343 / Public SNP ID: rs16846769 / SNP Chromosome Position: 174190745 / SNP in Genomic Sequence: SEQ ID NO: 429 / SNP Position Genomic: 72111 / SNP Source: dbSNP; HapMap / Population(Allele,Count): Caucasian (G,114|A,4) / SNP Type: INTRON / Context (SEQ ID NO: 2297): /
AAAATTGTTTTTAACAGTAACTGATAATATATGGTTTGACCACCACTTAATTAACCATTTCTTTTTTTTTTTGTACATTTAGAA
TGTTTTCCTCCAGTTCA
S
ATATAGACAGTTAATAGGAAACAGTTATATTATTTAATGCTCAACTTTGTTCCTTAGCTGTGCCTTTAATTCTAAGAGTAGTT
GCTAGCTAGGTATTTTA / Celera SNP ID: hDV70683382 / Public SNP ID: rs16846815 / SNP Chromosome Position: 174220542 / SNP in Genomic Sequence: SEQ ID NO: 429 / SNP Position Genomic: 101908 / Related Interrogated SNP: hCV16180170 / Related Interrogated SNP: hCV8911768 / SNP Source: dbSNP; HapMap / Population(Allele,Count): Caucasian (G,206|C,18) / SNP Type: INTRON / Context (SEQ ID NO: 2298): /
AAAATACATTACGCTTAAACTCAGAACTGAATTAGACTTCCAGTTGTAGTGCTAACTTGATAGGTAGCTTGCACAAGTTATTT
AACATTTTTAGGTTCCA
Y
TTTTCCAGTGAAGCTTTTATAAACCTGCTCATGTATGCATGCTTTGTCTTTCAGAAGTGGAATAATGTTTGCAAAGTTTTTTT
GTGTGTTTTGTTTTTCT / Celera SNP ID: hDV70934851 / Public SNP ID: rs17301125 / SNP Chromosome Position: 174343893 / SNP in Genomic Sequence: SEQ ID NO: 429 / SNP Position Genomic: 225259 / Related Interrogated SNP: hCV16180170 / Related Interrogated SNP: hCV8911768 / SNP Source: dbSNP; HapMap / Population(Allele,Count): Caucasian (T,104|C,14) / SNP Type: INTRON / Context (SEQ ID NO: 2299): /
GGATTTAATTGGTAAATAAGAGACAGTGTTCTTAAACTCTTAAGACTTGGACAATTTCTGAGGAATAGTGGGACAGAAGTCAG
ATTGCAGTTGATTTAGC
R
TTAGTTGATTTAACTCCCAGCCCTCTGTGCATCTGGAGACAAAAGAGATGCTGGGTACAAATCCAAAGTATTAGGATAGATTT
TACTTTATTTTATGTAA / Celera SNP ID: hCV30205819 / Public SNP ID: rs7545388 / SNP Chromosome Position: 174265423 / SNP in Genomic Sequence: SEQ ID NO: 429 / SNP Position Genomic: 146789 / SNP Source: dbSNP; HapMap / Population(Allele,Count): Caucasian (G,201|A,25) / SNP Type: INTRON //

Gene Number: 93 / Gene Symbol: EXOG - 9941 / Gene Name: endo/exonuclease (5'-3'), endonuclease G-like / Chromosome: 3 / OMIM NUMBER: / OMIM Information: // Genomic Sequence (SEQ ID NO: 430): // / SNP Information / Context (SEQ ID NO: 2300): /
CCTTAAGCTTTGTGTTGGAAATATTTAGTTCTTAGAATTCACGTTATAGGTTTGGATTTTATAATTGATAAGCCTACCTGACT
ACTTACTTTATAGTCCT
Y
ATTTTGAATAATTATTATAAGAGCTTTCTGTCCTGGAATAATTATGGCTTTCATTTATTGAAACAAGTAGTCAATAATTGCCT
AGCACTAATGTTTAAAT / Celera SNP ID: hCV16189344 / Public SNP ID: rs2298422 / SNP Chromosome Position: 38544916 / SNP in Genomic Sequence: SEQ ID NO: 430 / SNP Position Genomic: 17153 / Related Interrogated SNP: hCV15949414 / SNP Source: Applera / Population(Allele,Count): Caucasian (C,37|T,1) African American (C,24|T,10) total (C,61|T,11) / SNP Type: TRANSCRIPTION FACTOR BINDING SITE;INTRON / SNP Source: dbSNP; HapMap; HGBASE / Population(Allele,Count): Caucasian (C,99|T,7) / SNP Type: TRANSCRIPTION FACTOR BINDING SITE;INTRON / Context (SEQ ID NO: 2301): /
CACCATCACCAGCGACGTGTGGGTGCTGCCTCAACAGGTATTGGGAACTTGCTCTTGTTTACAGCTTGGCTCCTCATGGTAGC
TGTAGGAGGCAGGATCT

R
GGTGGATGGGACCTGGGATTCATTCTTAGAATAGGTCCCCAAATGGTCATCCATTTTGCTTGCCGAGGGCTCTGTGGGGCAGA
CGTAGGAGGCCTGAGGG / Celera SNP ID: hCV247719 / Public SNP ID: rs3796387 /
SNP Chromosome Position: 38579207 / SNP in Genomic Sequence: SEQ ID NO: 430 /
SNP Position Genomic: 51444 / SNP Source: dbSNP; HapMap; HGBASE /
Population(Allele,Count): Caucasian (G,188|A,38) / SNP Type:
INTRON;PSEUDOGENE /
Context (SEQ ID NO: 2302): /
CTATTGCAGATGACAAACTCTGTCATTCCCAAACGTTGCTGAGTACTGGAGTCATTTGCGGAGCTATTAAAAAATACAATTAT
GGGTCCTTATCCCCAAC
S
TGGAATTAGCAAACCAGGTCTCCAGTTTCAGAGATTTGGACTTAATCTCACTAAAGGAACTCATGGTTCCCCAATAATAAAAT
AATGATAGTCTTTAATA / Celera SNP ID: hCV27512998 / Public SNP ID: rs3762790 /
SNP Chromosome Position: 38535168 / SNP in Genomic Sequence: SEQ ID NO: 430 /
SNP Position Genomic: 7405 / Related Interrogated SNP: hCV15949414 / SNP
Source: dbSNP; HapMap; HGBASE / Population(Allele,Count): Caucasian
(C,114|G,6) / SNP Type: INTERGENIC;UNKNOWN /
Context (SEQ ID NO: 2303): /
ACTTCCCATTAGCAGCCCATCCCTGAACCTAACCCTGCACCCAGGGTGCTGTTTGTTCCCTGCCCACCCCTTCATACCTTCAC
TCTGTGTGTCCTTCCCC
R
TCCTGGCACACTTCCAAGTGCCTTCTGTATCCTCACTATAGTTCATTCTGATGGATTTGGTCAGGGCTGGGCTCACATCAGGT
GTGAAACTCCTGCTCCT / Celera SNP ID: hCV30700451 / Public SNP ID: rs12631864 /
SNP Chromosome Position: 38569764 / SNP in Genomic Sequence: SEQ ID NO: 430 /
SNP Position Genomic: 42001 / Related Interrogated SNP: hCV15949414 / SNP
Source: dbSNP; HapMap / Population(Allele,Count): Caucasian (G,114|A,6) / SNP
Type: INTRON /
Context (SEQ ID NO: 2304): /
GATGGCTGTTACGTATTTTGTAAATGAAGAAACAAGAAATGGGTGGACGCGACTTGCAGCTTAGTGGCAAAGCCAGGTTTGAC
CAGGTCTCTGGGCTTCC
R
GTCCACCAAACCTCTAGTAGTGTTTAGAAACATTTCTGGAAGTCTCCCAATTTTTTCCAAAAGTAGGAAAAATTTATTATGTAG
ATTGAGTTGTCTTTAAA / Celera SNP ID: hCV30700457 / Public SNP ID: rs12635900 /
SNP Chromosome Position: 38562392 / SNP in Genomic Sequence: SEQ ID NO: 430 /
SNP Position Genomic: 34629 / Related Interrogated SNP: hCV15949414 / SNP
Source: dbSNP; HapMap / Population(Allele,Count): Caucasian (A,215|G,11) /
SNP Type: TRANSCRIPTION FACTOR BINDING SITE;INTRON /
Context (SEQ ID NO: 2305): /
GGGGAGGAAATTGAAAATGCCCTCAGGAGTCTTGAGACATCATTTGAAGTGATATGGGAGTAATATTTTTTTTTAAGTCTCTTG
CTTTATATTTAGGGAAA
R
CTTTTGTTCATTTCCATATCTATTTGTTTTAATGAAAGTTAAATTATTTTACTGGTTGTCTCCCTACCCTCACCCTAAATTTT
CAAGTACCATTGACAGC / Celera SNP ID: hDV70822211 / Public SNP ID: rs17037809 /
SNP Chromosome Position: 38561079 / SNP in Genomic Sequence: SEQ ID NO: 430 /
SNP Position Genomic: 33316 / Related Interrogated SNP: hCV15949414 / SNP
Source: dbSNP; HapMap / Population(Allele,Count): Caucasian (G,114|A,6) / SNP
Type: TFBS SYNONYMOUS;INTRON /
Context (SEQ ID NO: 2306): /
GACTTAGTAGGCTGCTACCCTTTGGCCTGGAACAGGTACCCCAGTGTGAGAAGTACCGTCTTCCAGGGTTTGTGCTGCCACAA
AAGCATCTGCTGAGGCT
Y
TATAGATAACACAGGGGGCGACCACATTCTTGTTGACTTTACCACACTTTGTGACTCCCTCACTTTTCATGTTCCTTCCCCCC
CTCAGGTTCGGATTTCC / Celera SNP ID: hDV70822215 / Public SNP ID: rs17037814 /
SNP Chromosome Position: 38561307 / SNP in Genomic Sequence: SEQ ID NO: 430 /
SNP Position Genomic: 33544 / Related Interrogated SNP: hCV15949414 / SNP
Source: dbSNP; HapMap / Population(Allele,Count): Caucasian (C,114|T,6) / SNP
Type: TRANSCRIPTION FACTOR BINDING SITE;INTRON /
Context (SEQ ID NO: 2307): /
TTGCTGACTCACTGATTAACTAGACTTGGTTTATGTTAAGCCTTTGAGTCCTTTGTTACAGACATCATTTGTGCCCAAGTTGC
TTTCTCCTCCTGAAACC
Y
CATTGCTGACCTGTAACTAGTCTAATCTTGCTTGATTATACGTTTCCCCCGCCCCCATCCCTCTGTAAAACCATCTTTTGTAC
CAAATGCCGTTGTATCT / Celera SNP ID: hDV70822219 / Public SNP ID: rs17037819 /

SNP Chromosome Position: 38562048 / SNP in Genomic Sequence: SEQ ID NO: 430 / SNP Position Genomic: 34285 / Related Interrogated SNP: hCV15949414 / SNP Source: dbSNP; HapMap / Population(Allele,Count): Caucasian (C,114|T,6) / SNP Type: TRANSCRIPTION FACTOR BINDING SITE;INTRON //

Gene Number: 94 / Gene Symbol: ODZ1 - 10178 / Gene Name: odz, odd Oz/ten-m homolog 1(Drosophila) / Chromosome: X / OMIM NUMBER: / OMIM Information: // Genomic Sequence (SEQ ID NO: 431): // / SNP Information / Context (SEQ ID NO: 2308): / AGAGATAATTGTAAAAGCAACAACAACAGCAACAGCAATCATGGCTGTATGCTTATTTGAACAAGACTAGAAATGAGAGCAGA AATTTGAAAAGTTAAAG
Y
ATTTGAATGACCACCAGATGGCAGTGTGGGGCCACCTGGAAGCTGAAGCCAAAGTCGTCGTTCTGTCTCTCTAGGCAGCCTCT GAAAGTGTCAGCCCCAA / Celera SNP ID: hCV16177220 / Public SNP ID: rs2266911 / SNP Chromosome Position: 123522600 / SNP in Genomic Sequence: SEQ ID NO: 431 / SNP Position Genomic: 22847 / SNP Source: dbSNP; HapMap; ABI_Val; HGBASE / Population(Allele,Count): Caucasian (C,136|T,31) / SNP Type: INTRON / Context (SEQ ID NO: 2309): / AGGGCTATGTCTTAAGTACTTGAAATACTCTAGACTCTCAATAGGTGGTTGTTGATTGCTTTTTCCCCAGCTCAAGCCTTAAG TGCAGTGCCACAGTTTT
Y
AGTAATTATGTGATCTTATTTTATGTAAGTCAGTTGCCTACTAAGGAGTGGGTATTCCTTCACCCTTTTCACCAGTGAAGTGG CCATAACATAAAAGGGT / Celera SNP ID: hCV2451164 / Public SNP ID: rs2357252 / SNP Chromosome Position: 123526488 / SNP in Genomic Sequence: SEQ ID NO: 431 / SNP Position Genomic: 26735 / Related Interrogated SNP: hCV16177220 / SNP Source: dbSNP; Celera; HapMap; HGBASE / Population(Allele,Count): Caucasian (C,140|T,29) / SNP Type: INTRON / Context (SEQ ID NO: 2310): / TACCACTGCAGATGAACAGTAAGATCTAATTATCTCAGTTATACCAATTTTGTGAAATCCTCAATCCTGCTGTTCAGAATCTA TTGTATTATAAGAAACA
R
GAACTGCAAATAATGATAGCCACACAAAATTTGGCTGGAAATCTGAGAGTTATGCCTGTCAATGCTTTCAGGATCTGTTTCAG GACTTTCCAGTCCTACT / Celera SNP ID: hCV2451180 / Public SNP ID: rs2072886 / SNP Chromosome Position: 123554957 / SNP in Genomic Sequence: SEQ ID NO: 431 / SNP Position Genomic: 55204 / Related Interrogated SNP: hCV16177220 / SNP Source: dbSNP; Celera; HGBASE / Population(Allele,Count): Caucasian (A,93|G,11) / SNP Type: INTRON / Context (SEQ ID NO: 2311): / TATATATAACAGAAACCTTCCCGGTATATCAGGCGTGACACAGGTGCTCAAAGCAGATCTGGCCTCAATAGTGAGTGATTATT TCCCGCAGGCTGGCAGT
Y
GCTCTAGGGCTGATATTGAATGTTACTACATGTAGAAAGCTGGCCACATCATCCTAACACTGGTTAACGTTCAAGAGGAGGCA GGTAGACAGAGTGCTAT / Celera SNP ID: hCV29241293 / Public SNP ID: rs7061257 / SNP Chromosome Position: 123538104 / SNP in Genomic Sequence: SEQ ID NO: 431 / SNP Position Genomic: 38351 / Related Interrogated SNP: hCV16177220 / SNP Source: dbSNP; HapMap / Population(Allele,Count): Caucasian (T,134|C,35) / SNP Type: INTRON / Context (SEQ ID NO: 2312): / GAGCCTCTTTATCCCTGCTCTGCCCCTGTCATAGTGGGAGAATGTGCCACAAAGGCCATATTAATGAGAGGACTGGAAAGAAC ACAAGGAGATACAAAAC
Y
TCAAGTCAGTGAAAAAGTTACATGTTACATGGGATTTTGGTAAATTCAATGTTCTTTCCCTTTTTCCCTCTAGAATCACAATT TCAAAGATGCTTTAATT / Celera SNP ID: hCV32361087 / Public SNP ID: rs4825898 / SNP Chromosome Position: 123523350 / SNP in Genomic Sequence: SEQ ID NO: 431 / SNP Position Genomic: 23597 / Related Interrogated SNP: hCV16177220 / SNP Source: dbSNP; HapMap; HGBASE / Population(Allele,Count): Caucasian (C,73|T,17) / SNP Type: INTRON //

Gene Number: 95 / Gene Symbol: SLC19A2 - 10560 / Gene Name: solute carrier family 19 (thiamine transporter), member 2 / Chromosome: 1 / OMIM NUMBER: 603941 / OMIM Information: Thiamine-responsive megaloblastic anemia syndrome, 249270 (3) // Genomic Sequence (SEQ ID NO: 432): // / SNP Information / Context (SEQ ID NO: 2313): /

CTACATTCACCTGTATAACCTGTCTAAAAGCCAGCATACTGTTTGGAAGATACTATTCAATAAATGCTTTGAAAACGGAATCA
CTGTCAATTCTTACTTC
Y
AGACACAAGATAGGGAGCCTTTATCCTACTGTTTCATTAAGCTTCTCTGACTTTAAGTTTTGTTTCTTTTGGGGCAGAGGAAC
AAATAAAGATGGTCAAT / Celera SNP ID: hCV2456680 / Public SNP ID: rs6427193 /
SNP Chromosome Position: 169448377 / SNP in Genomic Sequence: SEQ ID NO: 432 /
SNP Position Genomic: 25230 / Related Interrogated SNP: hCV8919444 / SNP
Source: dbSNP; Celera; HapMap / Population(Allele,Count): Caucasian
(T,146|C,80) / SNP Type: INTRON;PSEUDOGENE /
Context (SEQ ID NO: 2314): /
GTATGGACAAGTCACTTAACATCTCTCAGACTCATCTTCTTCATGTCTAATACAGCAACCCTACAAATCCTATATAAAGCACT
TGACACGTAGAGGCACT
S
AATGCACATTGTCTATTATTACTATTTCTCTCTTAGATCGCTTCAGTAAAAACAAAGTTAATTACGGCTAATATGTTGGGAAG
AAATCATTTGGGTTGGG / Celera SNP ID: hCV2456690 / Public SNP ID: rs6692649 /
SNP Chromosome Position: 169436603 / SNP in Genomic Sequence: SEQ ID NO: 432 /
SNP Position Genomic: 13456 / Related Interrogated SNP: hCV8919444 / SNP
Source: dbSNP; Celera; HapMap / Population(Allele,Count): Caucasian
(C,76|G,38) / SNP Type: INTRON /
Context (SEQ ID NO: 2315): /
GACTCTGCTAACGTATCACAGCTTTGAGTGGCAAACCTAACATTTTGAACCCCCTTCTGTAGTATTACTTACTGAAAAAAGTA
GAGGAACTAGTATTTCT
M
CAAGGTCCTTTTCAAGAATCAAAGGAGATGATGTTAATAAATGCCCTTCCTAAAGTATGTTAAATATAAGGTATTAATTATGG
GGAATATGACATAATAG / Celera SNP ID: hCV2456692 / Public SNP ID: rs12128350 /
SNP Chromosome Position: 169431218 / SNP in Genomic Sequence: SEQ ID NO: 432 /
SNP Position Genomic: 8071 / Related Interrogated SNP: hCV8919444 / SNP
Source: dbSNP; Celera; HapMap / Population(Allele,Count): Caucasian
(A,82|C,38) / SNP Type: INTERGENIC;UNKNOWN /
Context (SEQ ID NO: 2316): /
TGAGGTATCATACCATTTACAGATGAGAAAACTGAGGCTCAATAGAGTGACTCTGCTAACGTATCACAGCTTTGAGTGGCAAA
CCTAACATTTTGAACCC
Y
CTTCTGTAGTATTACTTACTGAAAAAAGTAGAGGAACTAGTATTTCTACAAGGTCCTTTTCAAGAATCAAAGGAGATGATGTT
AATAAATGCCCTTCCTA / Celera SNP ID: hCV2456693 / Public SNP ID: rs6672589 /
SNP Chromosome Position: 169431170 / SNP in Genomic Sequence: SEQ ID NO: 432 /
SNP Position Genomic: 8023 / Related Interrogated SNP: hCV8919444 / Related
Interrogated SNP: hCV11975250 / SNP Source: dbSNP; Celera; HapMap /
Population(Allele,Count): Caucasian (C,40|T,80) / SNP Type:
INTERGENIC;UNKNOWN /
Context (SEQ ID NO: 2317): /
GCTTTCTGAGCAAGGCCTACTCCCCCAACCCTTCCCCACTGCGGTTAATATCTTACAAACTTGACAGTGGAACTGGGGCCAAA
GTGACCATTAATTTGCC
R
GGTAGGGTGGGAATCAGAATCCTGGTTTATCCGCTTCATAGATGTGTAATCTTGGCAGGTTATTTAAACTTTCTAAGCTACTT
TTTGAAACGTAAACACA / Celera SNP ID: hCV2456695 / Public SNP ID: rs10919173 /
SNP Chromosome Position: 169430112 / SNP in Genomic Sequence: SEQ ID NO: 432 /
SNP Position Genomic: 6965 / Related Interrogated SNP: hCV8919444 / Related
Interrogated SNP: hCV11975250 / SNP Source: dbSNP; Celera; HapMap /
Population(Allele,Count): Caucasian (G,40|A,80) / SNP Type:
INTERGENIC;UNKNOWN /
Context (SEQ ID NO: 2318): /
ATTGGGGGATTAATCAGACATAAGGAAGAAATAAAAAACTTACAATGAAGGGAGGCAATTATTAAATTGCTTCTGCTCAGCAG
AAAATGTTTTCTTCAGG
R
TCTCAACCATGAATACCTAAATTCTGGGCAGGGAAAGCAGGAGTATAATAGAAATACTAATCAAAATTCACAGGACGTTCACA
CTGCTTTTTCTTTTAAA / Celera SNP ID: hCV2456708 / Public SNP ID: rs1517745 /
SNP Chromosome Position: 169423795 / SNP in Genomic Sequence: SEQ ID NO: 432 /
SNP Position Genomic: 648 / Related Interrogated SNP: hCV11975250 / SNP
Source: dbSNP; Celera; HapMap; HGBASE / Population(Allele,Count): Caucasian
(A,69|G,155) / SNP Type: UTR5;INTRON;PSEUDOGENE /
Context (SEQ ID NO: 2319): /
ATCTACAAACAAAGGTTTATTTCTATTACATCAAAACTCTGAGCTGCTCATAGTTAATCATGCATGCTCTAATTTAATAAGTG

GCCTTCACTGTGTTCCA
Y
GGAGCTTTAAGAGTTTCATGTGGGATGAATAACAGGCTCAAGTATGTAAAATTATCAGACCTGCATTCCTGTTTCAATCAGAA
AGGCTCAATTTTTATTT / Celera SNP ID:  hCV8006091 / Public SNP ID:   rs6656463 /
SNP Chromosome Position:  169442211 / SNP in Genomic Sequence:  SEQ ID NO: 432 /
 SNP Position Genomic:  19064 / Related Interrogated SNP:  hCV8919444 / SNP
Source:  dbSNP; Celera; HapMap / Population(Allele,Count):  Caucasian
(T,147|C,79) / SNP Type:  INTRON /
Context          (SEQ ID NO: 2320): /
CTATTCCTTGCCTTATTATGCCATGGAATAAAAATACTCCTACTCATTTTCCCTCTTTAAATAATGTAAAGTTATGTATAGAT
AATTCAACACAAAACTA
Y
TTGCCAACTCAAAAAAGATTTTACAAAAGATATAATATCTTAATATGTGGTGTTTTATTCATATATTTTGCAGAGCTGCTCAA
TTAATTGAAGTTTATAT / Celera SNP ID:  hCV8697043 / Public SNP ID:   rs1517747 /
SNP Chromosome Position:  169423596 / SNP in Genomic Sequence:  SEQ ID NO: 432 /
 SNP Position Genomic:  449 / Related Interrogated SNP:  hCV8919444 / Related
Interrogated SNP:  hCV11975250 / SNP Source:  dbSNP; HapMap; ABI_Val; HGBASE /
Population(Allele,Count):  Caucasian (C,38|T,82) / SNP Type:  INTRON /
Context          (SEQ ID NO: 2321): /
AGACATAAGGAAGAAATAAAAAACTTACAATGAAGGGAGGCAATTATTAAATTGCTTCTGCTCAGCAGAAAATGTTTTCTTCA
GGATCTCAACCATGAAT
R
CCTAAATTCTGGGCAGGGAAAGCAGGAGTATAATAGAAATACTAATCAAAATTCACAGGACGTTCACACTGCTTTTTCTTTTA
AAAAATGTTGTATTTGA / Celera SNP ID:  hCV8697049 / Public SNP ID:   rs1517744 /
SNP Chromosome Position:  169423810 / SNP in Genomic Sequence:  SEQ ID NO: 432 /
 SNP Position Genomic:  663 / Related Interrogated SNP:  hCV11975250 / SNP
Source:  dbSNP; HapMap; HGBASE / Population(Allele,Count):  Caucasian
(A,68|G,158) / SNP Type:  UTR5;INTRON;PSEUDOGENE /
Context          (SEQ ID NO: 2322): /
ATAACTGGGAGTTAATTACAAGGGTTGGAGATAAAAACAGACCTTGAGGCCTATTGTTGGTTACTTGGATAATTTCCCACTAT
GGCTATTCTCTGGGATA
Y
GGCCTTTTACTTAGTCAAGCATAAGACACAAGCAAGAATTTCCAGGTAGCTGTTTTCTCCAAGTGCTCGATCCTAGAGATTAG
TACTGACCTAGAATTTA / Celera SNP ID:  hCV8697055 / Public SNP ID:   rs1208134 /
SNP Chromosome Position:  169428944 / SNP in Genomic Sequence:  SEQ ID NO: 432 /
 SNP Position Genomic:  5797 / Related Interrogated SNP:  hCV11975250 / SNP
Source:  dbSNP; HapMap; ABI_Val; HGBASE / Population(Allele,Count):  Caucasian
(C,18|T,208) / SNP Type:  INTRON /
Context          (SEQ ID NO: 2323): /
AACACCTAACTACGCTACGGTTTATAGTTAAAAGGAAGGTGGAAGATCCTGAGTCATTACAGCAATCACAAAACATGATGGAT
TTCTTCATTTAAAAACA
M
AAAAGAGGTCTTGGCATAGTTTTCAAGGGCAAAGCATGACAAGACTCTCCAGTGCTTTCTTTCATCCCCCTCTGTGTGCCTTT
TTCTGCCTGACTCACAG / Celera SNP ID:  hCV11975194 / Public SNP ID:   rs2038024 /
SNP Chromosome Position:  169455982 / SNP in Genomic Sequence:  SEQ ID NO: 432 /
 SNP Position Genomic:  32835 / Related Interrogated SNP:  hCV11975250 / SNP
Source:  dbSNP; Celera; HapMap; ABI_Val; HGBASE / Population(Allele,Count):
Caucasian (C,40|A,186) / SNP Type:  TRANSCRIPTION FACTOR BINDING
SITE;UTR3;PSEUDOGENE /
Context          (SEQ ID NO: 2324): /
TTCTTTGTTTCTTAAATGCTTGACCCAGAATTTTGCATGTAGTCATCAAGACATCGCCCCACAGCTGAGCTGTGTCATGGAAC
AGCCAGCCCATCTAAAA
R
GCTGCCTACTCATCAGCCTGCTCAGCCTGTGTGGGCTGGGAATGTGGTGCCATGCAGGGGAGGCATTCCCCCGTTGCATGGT
ACCACATACCCTTTGGG / Celera SNP ID:  hCV15962928 / Public SNP ID:   rs2285211 /
SNP Chromosome Position:  169461406 / SNP in Genomic Sequence:  SEQ ID NO: 432 /
 SNP Position Genomic:  38259 / Related Interrogated SNP:  hCV11975250 / SNP
Source:  dbSNP; HapMap; ABI_Val; HGBASE / Population(Allele,Count):  Caucasian
(G,99|A,127) / SNP Type:  INTERGENIC;UNKNOWN /
Context          (SEQ ID NO: 2325): /
ATTTTAGGTAGTATCTTCCCATACAATTCATCATATCCATCTTTGCCAGATTAAATTTCTTAAAAATAAGCTTAGTCATTACT
CCCCATCCCCATCTCCA
R

TTAATAATCATTACAAAATGACTCACGCTGTCTTCCCAACTCTATCTCATCCTACTCTTCTACACATATTCCTTGTTGACTGT
ACCCATCTCCTGCACTT / Celera SNP ID: hCV27242706 / Public SNP ID: rs7524348 /
SNP Chromosome Position: 169430438 / SNP in Genomic Sequence: SEQ ID NO: 432 /
SNP Position Genomic: 7291 / Related Interrogated SNP: hCV8919444 / Related
Interrogated SNP: hCV11975250 / SNP Source: dbSNP; Celera; HapMap /
Population(Allele,Count): Caucasian (G,40|A,80) / SNP Type: TFBS
SYNONYMOUS;INTERGENIC;UNKNOWN /
Context (SEQ ID NO: 2326): /
ATCACAAAACATGATGGATTTCTTCATTTAAAAACACAAAAGAGGTCTTGGCATAGTTTTCAAGGGCAAAGCATGACAAGACT
CTCCAGTGCTTTCTTTC
R
TCCCCCTCTGTGTGCCTTTTTCTGCCTGACTCACAGCCTGGCTCAATCCTAATCTTTCTACTGCAAAGATGCAGCATTTAAAG
CAATTTTTCCTGTGCAG / Celera SNP ID: hCV27242742 / Public SNP ID: rs12408451 /
SNP Chromosome Position: 169456046 / SNP in Genomic Sequence: SEQ ID NO: 432 /
SNP Position Genomic: 32899 / Related Interrogated SNP: hCV11975250 / SNP
Source: dbSNP; Celera / Population(Allele,Count): Caucasian (A,67|G,159) /
SNP Type: UTR3;PSEUDOGENE /
Context (SEQ ID NO: 2327): /
TAAGTTTTGGGGTTAGATGTTGGTTTAACTCTAAGCTCTGGAACACTACTAGTGATGGTATGGACAAGTCACTTAACATCTCT
CAGACTCATCTTCTTCA
Y
GTCTAATACAGCAACCCTACAAATCCTATATAAAGCACTTGACACGTAGAGGCACTCAATGCACATTGTCTATTATTACTATT
TCTCTCTTAGATCGCTT / Celera SNP ID: hCV29397245 / Public SNP ID: rs6656822 /
SNP Chromosome Position: 169436546 / SNP in Genomic Sequence: SEQ ID NO: 432 /
SNP Position Genomic: 13399 / Related Interrogated SNP: hCV11975250 / SNP
Source: dbSNP; HapMap / Population(Allele,Count): Caucasian (T,67|C,159) /
SNP Type: INTRON /
Context (SEQ ID NO: 2328): /
AGAAATGATGGAATTCAATAAAAAATAATTATAATCATAAACTGTTAATTTTAGAAAAAAACATTGAAATTGAGACCAAGAAA
TGTAAATCAGCTTACCA
R
AATAACCTAGAGAGTTAGCAGCAAATTCTAAATAGTCTTTGGTCATGCAAACTTACAATTCAATACTATTCCTCCATGCCATG
TAACCTCTTAAGTGCTT / Celera SNP ID: hCV29820280 / Public SNP ID: rs6696217 /
SNP Chromosome Position: 169460726 / SNP in Genomic Sequence: SEQ ID NO: 432 /
SNP Position Genomic: 37579 / Related Interrogated SNP: hCV11975250 / SNP
Source: dbSNP / Population(Allele,Count): Caucasian (A,19|G,207) / SNP Type:
TRANSCRIPTION FACTOR BINDING SITE;INTERGENIC;UNKNOWN /
Context (SEQ ID NO: 2329): /
CCTTCCTAAAGTATGTTAAATATAAGGTATTAATTATGGGGAATATGACATAATAGGTAAAACAAATAGATATCATACCATTT
CTATCATTTAAGCACAA
Y
AGTACCTTGAATAAGGCATCCAGTGGACTAGAGATCAACAGGCATTTTAATCTTAATATTTTACACTGATGTGCTTTTTTTTTT
TCTGAGACAGTCTCTCT / Celera SNP ID: hCV32141371 / Public SNP ID: rs10800447 /
SNP Chromosome Position: 169431363 / SNP in Genomic Sequence: SEQ ID NO: 432 /
SNP Position Genomic: 8216 / Related Interrogated SNP: hCV11975250 / SNP
Source: dbSNP; HapMap / Population(Allele,Count): Caucasian (C,70|T,156) /
SNP Type: TRANSCRIPTION FACTOR BINDING SITE;INTERGENIC;UNKNOWN /
Context (SEQ ID NO: 2330): /
TTAAAAATGACAACTCCAATTTTGTAGGCTGCATTTAAAAATACAGTAAGAATAAGGCTGGGCAAGGTGGCTCATGCCTGTAA
TCCCAATAATTTGGGAG
R
CAAAGGTAGGAGGATCGCTTGAGGTCAGGAGTTCAAGACCAGCCTGGACAACATAGTGAGACCCCATCTCTACAAAATAACAA
TTCACTGGGTGTGTTGG / Celera SNP ID: hCV32141374 / Public SNP ID: rs10919174 /
SNP Chromosome Position: 169431793 / SNP in Genomic Sequence: SEQ ID NO: 432 /
SNP Position Genomic: 8646 / Related Interrogated SNP: hCV8919444 / Related
Interrogated SNP: hCV11975250 / SNP Source: dbSNP; HapMap /
Population(Allele,Count): Caucasian (A,38|G,82) / SNP Type:
INTERGENIC;UNKNOWN //

Gene Number: 96 / Gene Symbol: STAG2 - 10735 / Gene Name: stromal antigen 2
/ Chromosome: X / OMIM NUMBER: / OMIM Information: // Genomic Sequence (SEQ
ID NO: 433): // / SNP Information /
Context (SEQ ID NO: 2331): /

AGAGATAATTGTAAAAGCAACAACAACAGCAACAGCAATCATGGCTGTATGCTTATTTGAACAAGACTAGAAATGAGAGCAGA
AATTTGAAAAGTTAAAG
Y
ATTTGAATGACCACCAGATGGCAGTGTGGGGCCACCTGGAAGCTGAAGCCAAAGTCGTCGTTCTGTCTCTCTAGGCAGCCTCT
GAAAGTGTCAGCCCCAA / Celera SNP ID: hCV16177220 / Public SNP ID: rs2266911 /
SNP Chromosome Position: 123522600 / SNP in Genomic Sequence: SEQ ID NO: 433 /
SNP Position Genomic: 438125 / SNP Source: dbSNP; HapMap; ABI_Val; HGBASE /
Population(Allele,Count): Caucasian (C,136|T,31) / SNP Type: INTRON /
Context (SEQ ID NO: 2332): /
AGGGCTATGTCTTAAGTACTTGAAATACTCTAGACTCTCAATAGGTGGTTGTTGATTGCTTTTTCCCCAGCTCAAGCCTTAAG
TGCAGTGCCACAGTTTT
Y
AGTAATTATGTGATCTTATTTTATGTAAGTCAGTTGCCTACTAAGGAGTGGGTATTCCTTCACCCTTTTCACCAGTGAAGTGG
CCATAACATAAAAGGGT / Celera SNP ID: hCV2451164 / Public SNP ID: rs2357252 /
SNP Chromosome Position: 123526488 / SNP in Genomic Sequence: SEQ ID NO: 433 /
SNP Position Genomic: 442013 / Related Interrogated SNP: hCV16177220 / SNP
Source: dbSNP; Celera; HapMap; HGBASE / Population(Allele,Count): Caucasian
(C,140|T,29) / SNP Type: INTRON /
Context (SEQ ID NO: 2333): /
TACCACTGCAGATGAACAGTAAGATCTAATTATCTCAGTTATACCAATTTTGTGAAATCCTCAATCCTGCTGTTCAGAATCTA
TTGTATTATAAGAAACA
R
GAACTGCAAATAATGATAGCCACACAAAATTTGGCTGGAAATCTGAGAGTTATGCCTGTCAATGCTTTCAGGATCTGTTTCAG
GACTTTCCAGTCCTACT / Celera SNP ID: hCV2451180 / Public SNP ID: rs2072886 /
SNP Chromosome Position: 123554957 / SNP in Genomic Sequence: SEQ ID NO: 433 /
SNP Position Genomic: 470482 / Related Interrogated SNP: hCV16177220 / SNP
Source: dbSNP; Celera; HGBASE / Population(Allele,Count): Caucasian
(A,93|G,11) / SNP Type: INTRON /
Context (SEQ ID NO: 2334): /
TATATATAACAGAAACCTTCCCGGTATATCAGGCGTGACACAGGTGCTCAAAGCAGATCTGGCCTCAATAGTGAGTGATTATT
TCCCGCAGGCTGGCAGT
Y
GCTCTAGGGCTGATATTGAATGTTACTACATGTAGAAAGCTGGCCACATCATCCTAACACTGGTTAACGTTCAAGAGGAGGCA
GGTAGACAGAGTGCTAT / Celera SNP ID: hCV29241293 / Public SNP ID: rs7061257 /
SNP Chromosome Position: 123538104 / SNP in Genomic Sequence: SEQ ID NO: 433 /
SNP Position Genomic: 453629 / Related Interrogated SNP: hCV16177220 / SNP
Source: dbSNP; HapMap / Population(Allele,Count): Caucasian (T,134|C,35) /
SNP Type: INTRON /
Context (SEQ ID NO: 2335): /
GAGCCTCTTTATCCCTGCTCTGCCCCTGTCATAGTGGGAGAATGTGCCACAAAGGCCATATTAATGAGAGGACTGGAAAGAAC
ACAAGGAGATACAAAAC
Y
TCAAGTCAGTGAAAAAGTTACATGTTACATGGGATTTTGGTAAATTCAATGTTCTTTCCCTTTTTTCCCTCTAGAATCACAATT
TCAAAGATGCTTTAATT / Celera SNP ID: hCV32361087 / Public SNP ID: rs4825898 /
SNP Chromosome Position: 123523350 / SNP in Genomic Sequence: SEQ ID NO: 433 /
SNP Position Genomic: 438875 / Related Interrogated SNP: hCV16177220 / SNP
Source: dbSNP; HapMap; HGBASE / Population(Allele,Count): Caucasian
(C,73|T,17) / SNP Type: INTRON //

Gene Number: 97 / Gene Symbol: SDCCAG8 - 10806 / Gene Name: serologically
defined colon cancer antigen 8 / Chromosome: 1 / OMIM NUMBER: / OMIM
Information: // Genomic Sequence (SEQ ID NO: 434): // / SNP Information /
Context (SEQ ID NO: 2336): /
ACTTGAAAGGCATCAAAACAGGCCGCTGACAATCACAGTGTTCCTCCTGTCAGCTTTCCTGGTTTCCTGTATTTCAAAGATCA
CAGATGATCAGCACCCT
S
TTCTCATAAATGTCCTGTGATGACAGTCCTGTGCTGTTGGAAGCTCTTCCTGGTATGTGGCCACGTGGTCAGCCACTCAGTCA
ACATGCGCTCCCACGCA / Celera SNP ID: hCV233148 / Public SNP ID: rs1417121 /
SNP Chromosome Position: 243669350 / SNP in Genomic Sequence: SEQ ID NO: 434 /
SNP Position Genomic: 260030 / SNP Source: dbSNP; Celera; HapMap; HGBASE /
Population(Allele,Count): Caucasian (G,86|C,30) / SNP Type: INTRON;PSEUDOGENE
/
Context (SEQ ID NO: 2337): /

ATACAGTAACACTATGGACTAGCCAATCTGGTGCTTGAGCAGAATTGAAAATAGGGTGATTCAAATTGGCAGAACTCTCGGTG
CCTCTCCTAAAGAGACG
R
CTTCTCAGGCACACGGGAGGACAAAAACGCCACTGAGAGACTATCAGCCCTGCTCCCTGCTTTTCCAGGGGACAAAGTGACCG
GCCATCGCCCAGAACTG / Celera SNP ID: hCV30690777 / Public SNP ID: rs12045585 /
SNP Chromosome Position: 243673099 / SNP in Genomic Sequence: SEQ ID NO: 434 /
SNP Position Genomic: 263779 / SNP Source: dbSNP; HapMap /
Population(Allele,Count): Caucasian (G,194|A,30) / SNP Type: ESE;SILENT
MUTATION;INTRON /
Context (SEQ ID NO: 2338): /
GCTCTGTGAGTGCCCTGGGGACTTCAGGCAGTAAGTGCTTGGAATGATTAATATGAAAAATGCATCTGGAAATGAAAACGAAG
AGGAGTCTCATTCTCCT
Y
ACAAAATGACCCTGTGCTCAAGGAACCTGTAAGTACTGACTTTTCCCACTCGGTCCAAAATCCCTCTGCCAAACATGTGTTCA
CAGACAGTAAATGAGCA / Celera SNP ID: hCV8688079 / Public SNP ID: rs884808 /
SNP Chromosome Position: 243653439 / SNP in Genomic Sequence: SEQ ID NO: 434 /
SNP Position Genomic: 244119 / Related Interrogated SNP: hCV233148 / Related
Interrogated SNP: hCV30690780 / SNP Source: dbSNP; Celera; HapMap; HGBASE /
Population(Allele,Count): Caucasian (C,169|T,57) / SNP Type: INTRON /
Context (SEQ ID NO: 2339): /
CAGGGAGGGCCCCAGCAGCACCTGCTTTAGGCCTGGCCAGCCAAAGATCAGGACCACTGGAGCCACTAGGTCTCCTGGCAGGT
GACTAGGCTCTTGTCAC
R
TAGGAGGGAGGGACCGTGACTCCTGGCATGAGTGAATCTTCAGCCTCTAAAGCCAAGAACTCTGTCAGACCTCAGGTTGTGGG
GAGTCTTCCGTGGAAGC / Celera SNP ID: hCV8688080 / Public SNP ID: rs884328 /
SNP Chromosome Position: 243654278 / SNP in Genomic Sequence: SEQ ID NO: 434 /
SNP Position Genomic: 244958 / Related Interrogated SNP: hCV233148 / Related
Interrogated SNP: hCV30690780 / SNP Source: dbSNP; Celera; HapMap; HGBASE /
Population(Allele,Count): Caucasian (A,169|G,57) / SNP Type: INTRON /
Context (SEQ ID NO: 2340): /
GTCAGACTGCAGTCCAGCCATCCTACCCATCTACATCACCCACGTCTAAGTTTGTTAATTTGCCATGACATGTTGTTAGAAAT
ACACTCTAAGAAAGGAA
R
TATGCAGAGCAGTACATTATCAGGAAGACGTTAATGAAAAGCTACATTCCTTCAGATTCTGGGTCCAAACCGTGTGTTGTATA
GATGATTCGGGTCTGAA / Celera SNP ID: hCV9493073 / Public SNP ID: rs1058305 /
SNP Chromosome Position: 243664642 / SNP in Genomic Sequence: SEQ ID NO: 434 /
SNP Position Genomic: 255322 / Related Interrogated SNP: hCV233148 / Related
Interrogated SNP: hCV30690780 / Related Interrogated SNP: hCV31523650 / SNP
Source: dbSNP; Celera; HGBASE / Population(Allele,Count): Caucasian
(A,166|G,60) / SNP Type: UTR3;INTRON /
Context (SEQ ID NO: 2341): /
GAGACCTCCTTCATCCCTGGCTTCACAGTACATCCATCCTAAATCCAGTGCTGAGAGGTCAGCGTTTCCCCTCAGAAGCAGCC
GTTCATCAAAGTTTGCA
Y
AACCGCACTACTGCCATTTCACTGAAGTAAAAATGATCCCCTATGTGTGTGTGTGTGAGCTACAAGGAATTATTTCTATATTT
TCCTGTCACATTTTATA / Celera SNP ID: hCV9493081 / Public SNP ID: rs1058304 /
SNP Chromosome Position: 243664857 / SNP in Genomic Sequence: SEQ ID NO: 434 /
SNP Position Genomic: 255537 / Related Interrogated SNP: hCV233148 / Related
Interrogated SNP: hCV30690780 / Related Interrogated SNP: hCV31523650 / SNP
Source: dbSNP; Celera; HapMap; HGBASE / Population(Allele,Count): Caucasian
(C,166|T,60) / SNP Type: UTR3;INTRON /
Context (SEQ ID NO: 2342): /
AGGCTGAGATCAAGTGCTCAGGATCCTTGGCCTCTCCACTTGGAGTCATGAGTGTAGATTTTAAAAATCATGCAGGCGGAACA
TTTCCAGTCAGGAGAGC
R
CTGAGCCCAGCCCTGGGTCACACCAGCATTTGAAGGTCTGATGGAGGAGGAAGAGGGACCAAAGGGGAGTGAGAGACCTGTAC
AGAGTCTAAGAAGGAGA / Celera SNP ID: hCV12073836 / Public SNP ID: rs1008173 /
SNP Chromosome Position: 243644497 / SNP in Genomic Sequence: SEQ ID NO: 434 /
SNP Position Genomic: 235177 / Related Interrogated SNP: hCV233148 / Related
Interrogated SNP: hCV30690780 / SNP Source: dbSNP; Celera; HGBASE /
Population(Allele,Count): Caucasian (A,180|G,46) / SNP Type: INTRON /
Context (SEQ ID NO: 2343): /
TATCTGTACACAGATAATTATTTGTCTAAAATAGTATTTCTACTATACACAATTAAGCCCTCAAATGCTTTAAAGTAATAAAA

ACGGACACTGGACATAC
Y
GTGTTGTATCTGAGAATGACAAACACTAAAGGCAAGGCTGCAGTTAGTTAGGACAGGTCCCTGACCTTCGGCTGCCCTGCCTG
GCCAGCGAGCCATCATC / Celera SNP ID: hCV12073840 / Public SNP ID: rs14403 /
SNP Chromosome Position: 243663893 / SNP in Genomic Sequence: SEQ ID NO: 434 /
SNP Position Genomic: 254573 / Related Interrogated SNP: hCV233148 / Related
Interrogated SNP: hCV30690780 / SNP Source: dbSNP; Celera; HGBASE /
Population(Allele,Count): Caucasian (C,172|T,54) / SNP Type: UTR3;INTRON /
Context (SEQ ID NO: 2344): /
TGTGGCGCAGTGAGCCCAGGGGCTCAAGGTGTTAGCAGTCATCAACAGTGCTATTTGAAACTGTGGGTGGATGAAGAGGTGAA
CTCAACACCACAAATAT
S
ACAGAGCAGTAACCACCATGGAAAAGAAAAGAGAGCTTATGTGTCTAGGACACCCCAAGAAATCTCTTCTGAATGAATGATGG
CAATGAAATAGCCATTA / Celera SNP ID: hCV15760239 / Public SNP ID: rs3006923 /
SNP Chromosome Position: 243648757 / SNP in Genomic Sequence: SEQ ID NO: 434 /
SNP Position Genomic: 239437 / Related Interrogated SNP: hCV233148 / Related
Interrogated SNP: hCV30690780 / SNP Source: dbSNP; HapMap; HGBASE /
Population(Allele,Count): Caucasian (G,163|C,59) / SNP Type:
INTRON;PSEUDOGENE /
Context (SEQ ID NO: 2345): /
AGATCAATGTGGCTCAGAGTGTGGTTTCCAGAGTACGGTCCTCAAACTGTTACTGGTCTGGGATGAGGCAAGAACTGAAATTG
AGTCAGAAATTTTCAAA
R
CAATCAAACACTGCTTCCTCCTTTTTATTGTACTCCACAAAACCATTAGTCTACAATAGATTGGAAATTTTAAAAGAATTAAA
ATCAAGTCTGGTTCTCC / Celera SNP ID: hCV16189408 / Public SNP ID: rs2994320 /
SNP Chromosome Position: 243641247 / SNP in Genomic Sequence: SEQ ID NO: 434 /
SNP Position Genomic: 231927 / Related Interrogated SNP: hCV233148 / SNP
Source: dbSNP; HapMap; HGBASE / Population(Allele,Count): Caucasian
(A,179|G,45) / SNP Type: TFBS SYNONYMOUS;SILENT MUTATION;INTRON /
Context (SEQ ID NO: 2346): /
ACTTTTTCTCATTTTTCATAAAAGGCAACAGGCTGATGTTCATATATTTCTCTTGAATGCGGATTCAGTTTCACTCATTGCCC
TGTCTTGGAAATCCCCA
Y
GTTAATGATGGCTTAAATTAGGTTGATAGAGTCCTCCCTTGGAGTCCAGCTTGGAAAATCCTCACTGGAGAGGGGCAGGCCCA
CTTCTCTTTTTGGAAGA / Celera SNP ID: hCV26034142 / Public SNP ID: rs9428576 /
SNP Chromosome Position: 243662027 / SNP in Genomic Sequence: SEQ ID NO: 434 /
SNP Position Genomic: 252707 / Related Interrogated SNP: hCV233148 / Related
Interrogated SNP: hCV30690780 / SNP Source: dbSNP; Celera; HapMap; ABI_Val /
Population(Allele,Count): Caucasian (T,119|C,107) / SNP Type: UTR3;INTRON /
Context (SEQ ID NO: 2347): /
ATCAAGAATGACAAATGCTCCCGGCTCTCGGGCGGTTCCCGGCCAATGGAGGAGAGACTCACTTGTAAAAAAAAACATGCTCAA
CTCAAACAGGACACTGA
R
GCATGAAGAGAAATGGCCAATATTGCAGATGAGAAGCCTGGGAGGGCTTGCGCCTGCTTAGACATGGCTCATGATCGTCGTAT
GCTGAGCACTTTTATGT / Celera SNP ID: hCV26034157 / Public SNP ID: rs2994329 /
SNP Chromosome Position: 243647921 / SNP in Genomic Sequence: SEQ ID NO: 434 /
SNP Position Genomic: 238601 / Related Interrogated SNP: hCV233148 / Related
Interrogated SNP: hCV30690780 / SNP Source: dbSNP; HapMap /
Population(Allele,Count): Caucasian (G,180|A,46) / SNP Type: UTR5;UTR3;INTRON
/
Context (SEQ ID NO: 2348): /
GATGAGGTGGGGTGTTGGAGCATCACCATATATATACATCACACACACAGGGAAACACTCCTACTTGGATGGAGGATCTTTGC
TGTTCTGTTGAGGCCAG
Y
CTGATCCTGGAGGCAGGGCAGGGCCTGGCTGTTGCCCAGGCTCCAAAGAGGCGCCAGGGCGTCCCTTAGTCCTTACACTTTCT
CCAGAACCTGCGGGCAG / Celera SNP ID: hCV26034160 / Public SNP ID: rs2994327 /
SNP Chromosome Position: 243650858 / SNP in Genomic Sequence: SEQ ID NO: 434 /
SNP Position Genomic: 241538 / Related Interrogated SNP: hCV233148 / Related
Interrogated SNP: hCV30690780 / SNP Source: dbSNP; HGBASE /
Population(Allele,Count): Caucasian (C,167|T,59) / SNP Type: INTRON /
Context (SEQ ID NO: 2349): /
GGTGTGCCTTCTTCCACTGCACTTTCTGGCCATTTCTAAGTTTTCTGCACTAAGCATGTGTTAGTTGTGTAAAAGGAAATGCA
AACAGTAACAGGCTCAG

Y
TGGGGTTTTTCGTCTTCCCCTTTTTGTTACCTCCCCTAGTAACCCTTGCAAACAGAGTTAGACTCCTCTATCTCTTTCTCTGT
CATACCTTTTTGACTTC / Celera SNP ID: hCV26338482 / Public SNP ID: rs10803143 /
SNP Chromosome Position: 243615589 / SNP in Genomic Sequence: SEQ ID NO: 434 /
SNP Position Genomic: 206269 / Related Interrogated SNP: hCV233148 / SNP
Source: dbSNP; Celera; HapMap / Population(Allele,Count): Caucasian
(C,167|T,57) / SNP Type: INTRON /
Context (SEQ ID NO: 2350): /
AAAGAATTAAAATCAAGTCTGGTTCTCCTCACTGAGCTCATTGGGGAATCCCTGGCCTAGGGATTTCCCTCTTCTCCCGCCCC
CAGGCCTCGGAAAATGA
R
CTGGTGTCAGCTGCTGCCCAGAAGGTTACCCAGCGTGTTCTCTCTCTCAGGGTCCTTCCCTTAAATTCTCAAGTTTGACATGG
AATAGAAGAATGTCGGG / Celera SNP ID: hCV26338512 / Public SNP ID: rs2994339 /
SNP Chromosome Position: 243641420 / SNP in Genomic Sequence: SEQ ID NO: 434 /
SNP Position Genomic: 232100 / Related Interrogated SNP: hCV233148 / SNP
Source: dbSNP; HapMap; HGBASE / Population(Allele,Count): Caucasian
(A,182|G,44) / SNP Type: UTR3;INTRON /
Context (SEQ ID NO: 2351): /
GTGGTACCCCCTCCCCACTGCACGATCCTCAGGAAGAATTCCTTTATTGAAACCATTATTGAATAGGGTAGATTTAGGCACGG
GAAATGTAAACAGCAAA
Y
CAGCCACTAAAAGTGCGTTAATGTCACGCCTGTAATCCCAGCACTTTGGGAAGCTGAGGTGGGCGGATCATGAGATCAAGAGA
TCGAGACCATCCTGGCC / Celera SNP ID: hCV26338513 / Public SNP ID: rs3006917 /
SNP Chromosome Position: 243641971 / SNP in Genomic Sequence: SEQ ID NO: 434 /
SNP Position Genomic: 232651 / Related Interrogated SNP: hCV233148 / SNP
Source: dbSNP; HapMap; HGBASE / Population(Allele,Count): Caucasian
(C,182|T,44) / SNP Type: TRANSCRIPTION FACTOR BINDING SITE;UTR3;INTRON /
Context (SEQ ID NO: 2352): /
AGTGACCTTGACTGTGCATATGCCTTCAGTTCTTGGTAGAACAGTGTTCACTTGCTCATGATTGCCCGTGAAGTCTCGACATC
CACATGTGATATGGGCT
K
CTTCTACAGTATCCACCAGGAATTTAAAAAAAAAAAAATTATATATATATATATATATATCCCAACAGTTGTTCAGTCCTTCTACC
AAATGCTTCCTAATCAA / Celera SNP ID: hCV30382231 / Public SNP ID: rs9428966 /
SNP Chromosome Position: 243667900 / SNP in Genomic Sequence: SEQ ID NO: 434 /
SNP Position Genomic: 258580 / Related Interrogated SNP: hCV233148 / Related
Interrogated SNP: hCV30690780 / Related Interrogated SNP: hCV31523650 / SNP
Source: dbSNP; HapMap / Population(Allele,Count): Caucasian (T,164|G,60) /
SNP Type: MICRORNA;UTR3;INTRON /
Context (SEQ ID NO: 2353): /
ATGCAGCAGTTTCTGCAATAAATCTAATTTGGTGCTGTCAAAAAATTAGACTTATTTGATATAGCAGCACATTAGTAAATGAT
TGTGGCAGATTTAAAGT
Y
GTCATTTAGTCTCTTCTTACTCTTAACAGAATCTCGCAAATTCATTTTCCCCCTTGATCAACTCTATGAAAATATCCCAGTTC
TTTGTCCTGCAGCCCTG / Celera SNP ID: hCV31056133 / Public SNP ID: rs10927006 /
SNP Chromosome Position: 243557659 / SNP in Genomic Sequence: SEQ ID NO: 434 /
SNP Position Genomic: 148339 / Related Interrogated SNP: hCV233148 / SNP
Source: dbSNP; HapMap / Population(Allele,Count): Caucasian (T,196|C,30) /
SNP Type: INTRON /
Context (SEQ ID NO: 2354): /
CTGTTCTAGAGAAAGAAAATCTATAAAATGGAAGTTCCTGAGATAAGAAAGCATAGTACTGGAGGAAGAGCAAGAACTGTGTG
TCCAACATAAAGGAAAA
M
AGAAAGTGTGGCATACAATGAGGTTGGAGAGGTAGGAAAGGGCTGGACCTAAAGGCAGGGTCTTTATGTTTTTTGTTTTTATTT
TATTTTATTTATTTATT / Celera SNP ID: hCV31056162 / Public SNP ID: rs12049318 /
SNP Chromosome Position: 243593660 / SNP in Genomic Sequence: SEQ ID NO: 434 /
SNP Position Genomic: 184340 / Related Interrogated SNP: hCV233148 / SNP
Source: dbSNP; HapMap / Population(Allele,Count): Caucasian (C,195|A,31) /
SNP Type: INTRON /
Context (SEQ ID NO: 2355): /
GCTTTGAGGGTAGCTCCCAGGAAAGGCTGCCTCAGCCCCAGCGTGATGGGGACGGGTAGGGGCCGATGAGGCTTCCCAGAGCA
GCGATGGCCTTAGGTAT
Y
GCCAGAAGGAAACCTACTTTTCCTCCAAGATCAGAGGGAAGGATGAGGTGGGGTGTTGGAGCATCACCATATATATACATCAC

ACACACAGGGAAACACT / Celera SNP ID: hCV30690784 / Public SNP ID: rs4658574 / SNP Chromosome Position: 243650716 / SNP in Genomic Sequence: SEQ ID NO: 434 / SNP Position Genomic: 241396 / Related Interrogated SNP: hCV233148 / Related Interrogated SNP: hCV30690780 / SNP Source: dbSNP; HapMap; ABI_val; HGBASE / Population(Allele,Count): Caucasian (T,167|C,59) / SNP Type: INTRON //

Gene Number: 98 / Gene Symbol: CFHR4 - 10877 / Gene Name: complement factor H-related 4 / Chromosome: 1 / OMIM NUMBER: / OMIM Information: // Genomic Sequence (SEQ ID NO: 435): // / SNP Information /
Context (SEQ ID NO: 2356): /
TTCTACATGTTTGTTGAGGGGCTTCCTGCATTGTTTTCATTTATTGTTTTTGTCTGAATATAGTAGGCTATGTCTGTGTCAAG AATTAGCCTGTAGCATA
K
ACTTTAGGTTGTCTCCAGTGTTTTCCAAGCCTGCACTTTTTGCTAAGTGTTCATAGTCATTTTCTAATTTTCCCCATTCATGC GGTTGCTTTGGAATATC / Celera SNP ID: hCV209646 / Public SNP ID: rs12734260 / SNP Chromosome Position: 196865417 / SNP in Genomic Sequence: SEQ ID NO: 435 / SNP Position Genomic: 18205 / Related Interrogated SNP: hCV2532034 / SNP Source: dbSNP; Celera; HapMap / Population(Allele,Count): Caucasian (G,102|T,12) / SNP Type: INTRON //

Gene Number: 99 / Gene Symbol: NCOA6 - 23054 / Gene Name: nuclear receptor coactivator 6 / Chromosome: 20 / OMIM NUMBER: 605299 / OMIM Information: // Genomic Sequence (SEQ ID NO: 436): // / SNP Information /
Context (SEQ ID NO: 2357): /
ATTATTCCAGAAGCATATGCCAAGAGGACAATAAGAAAACTTCTGAAAAGAGAAGATGCAAAAGAGCATTACAATGCATAGTT ATGCATTCAACAATGTA
Y
AATTCATAGTTCTATGAAAATTATAATTTATTTAATAAGAAAATACTATAAAAATTAAGAAATTACAGAGAAGATAAGCTATT TTAATTATGACATTCTA / Celera SNP ID: hCV11189450 / Public SNP ID: rs6060048 / SNP Chromosome Position: 33370266 / SNP in Genomic Sequence: SEQ ID NO: 436 / SNP Position Genomic: 132067 / Related Interrogated SNP: hCV1825046 / SNP Source: dbSNP; Celera; HapMap / Population(Allele,Count): Caucasian (T,128|C,98) / SNP Type: INTRON /
Context (SEQ ID NO: 2358): /
TTTGTTTATAGACAGCTATATAAGTGCTTTAGTTTTCTGTGCTTTTGAAGAATTATGGTTAGTTTACCACCAGAAAAATGGTG GGTTCAATGTAGATGGG
Y
CTTTCTTCAACCCATCTAAGCAGGTTTCATGAAGCACAAGGAGATATAACCAATTCTTACTTGTAATAGGGAAGCAGGGTGAC CATGAATGATGCTTAGT / Celera SNP ID: hCV1207879 / Public SNP ID: rs2295354 / SNP Chromosome Position: 33356541 / SNP in Genomic Sequence: SEQ ID NO: 436 / SNP Position Genomic: 118342 / Related Interrogated SNP: hCV1825046 / SNP Source: Applera / Population(Allele,Count): Caucasian (C,11|T,25) African American (C,6|T,30) total (C,17|T,55) / SNP Type: INTRON / SNP Source: Applera / Population(Allele,Count): Caucasian (C,12|T,26) African American (C,7|T,31) total (C,19|T,57) / SNP Type: INTRON / SNP Source: dbSNP; Celera; HGBASE / Population(Allele,Count): Caucasian (T,133|C,93) / SNP Type: INTRON /
Context (SEQ ID NO: 2359): /
AAAAGGAAATCATTAGAATAAAATGCAAATTTTGTTTATAGACAGCTATATAAGTGCTTTAGTTTTCTGTGCTTTTGAAGAAT TATGGTTAGTTTACCAC
Y
AGAAAAATGGTGGGTTCAATGTAGATGGGTCTTTCTTCAACCCATCTAAGCAGGTTTCATGAAGCACAAGGAGATATAACCAA TTCTTACTTGTAATAGG / Celera SNP ID: hCV1207880 / Public SNP ID: rs2295353 / SNP Chromosome Position: 33356511 / SNP in Genomic Sequence: SEQ ID NO: 436 / SNP Position Genomic: 118312 / Related Interrogated SNP: hCV1825046 / SNP Source: dbSNP; Celera; HapMap; ABI_val; HGBASE / Population(Allele,Count): Caucasian (C,131|T,95) / SNP Type: INTRON /
Context (SEQ ID NO: 2360): /
GTTACTGCCCTGAAAGAATAATTATTTATGGGCTTGAAATTAAGGGGAGAGACCAAGACTTATTACTTATCTGTATACTCCTG TTGTCTATCAAGTCTAC
R
ACACAATGCTCAATAAATGCTGGCTAAACAAATCACAGAAAACAAACCTGCATGCTGGACATTTTGATTTGCTTGCAGCTGAG GGGCTCCTGAATTCCCA / Celera SNP ID: hCV1207890 / Public SNP ID: rs6087625 /

433

SNP Chromosome Position: 33342477 / SNP in Genomic Sequence: SEQ ID NO: 436 / SNP Position Genomic: 104278 / Related Interrogated SNP: hCV1825046 / SNP Source: dbSNP; Celera; Applera / Population(Allele,Count): Caucasian (G,132|A,94) / SNP Type: INTRON /
Context (SEQ ID NO: 2361): /
ACTCATGAGGCTGAGGTGAAACAATCCCTTGAGCCTGAGGGGAGTGAGGGGAAGGGTGGAGGTTGTGGTGAGCTGAAATTGTG
CTACTGCACTATTGCTG
Y
TCTGAGCAACAGAGTGAGACTCTGTCTCAAAAAAAATTAAAAATGACTATAAATACTCGGTAAATGTCAAAGTGTTATAGTCAC
ACACAGCATAACAATAA / Celera SNP ID: hCV1207858 / Public SNP ID: rs6141514 /
SNP Chromosome Position: 33377565 / SNP in Genomic Sequence: SEQ ID NO: 436 /
SNP Position Genomic: 139366 / Related Interrogated SNP: hCV1825046 / SNP
Source: dbSNP; Celera / Population(Allele,Count): Caucasian (C,118|T,100) /
SNP Type: INTRON /
Context (SEQ ID NO: 2362): /
GTCAGTCTATGAAAAATTGCTGGCTTTCCCTCCAGAAAAAATAATTTTTAAAGAAACTTCTTTGGTTGGTAGGCTATTTATTA
CTGCCTCAATTTCAGAA
Y
TTGTTAATTGGTATACTCAGAGATTTAATTTTTTTCCTGGTTCAGTCTTCCTGGTTCAGCTGAGAGTGTATGTGTCCAGGAATT
TATCCATTTCTTCTAGA / Celera SNP ID: hCV1207862 / Public SNP ID: rs6119524 /
SNP Chromosome Position: 33373813 / SNP in Genomic Sequence: SEQ ID NO: 436 /
SNP Position Genomic: 135614 / Related Interrogated SNP: hCV1825046 / SNP
Source: dbSNP; Celera / Population(Allele,Count): Caucasian (C,132|T,94) /
SNP Type: INTRON /
Context (SEQ ID NO: 2363): /
GCAATCTAGATCTTAGAGAAGTGGCCTTTGTAATTCTGGATATACAGAGCAAAATCTGCTGGTACTATTCGATAAAATAAATG
CTTGTGAATACCTTCTA
Y
GTATGAAGCAACATACCAAGATGATGAATTCTGTCTTAAAAAGAGCTTGGTCAGTAGTGGACATTAAGAGCGAAGTGTAAAAC
ATGGAGACCAGTATTTA / Celera SNP ID: hCV1207887 / Public SNP ID: rs959829 /
SNP Chromosome Position: 33346047 / SNP in Genomic Sequence: SEQ ID NO: 436 /
SNP Position Genomic: 107848 / Related Interrogated SNP: hCV1825046 / SNP
Source: dbSNP; HapMap; ABI_Val; HGBASE / Population(Allele,Count): Caucasian
(T,139|C,87) / SNP Type: INTRON;PSEUDOGENE /
Context (SEQ ID NO: 2364): /
GCATTATAAAACTATAAAAGTCAATGATTTCTGAACTTATTCCCCTTTAATACTTTAACCAAACTTAACAAGTACCATGCTTA
CTATTTTATTAACTAGG
W
TTATCAAAATTGATAACTAAAAGGGCCTTTTCTTCAATGAGACCCTGTTCTTTTTATTTTGCTCTATTGCATAATCTTAATTT
TTCAGCAACTGAGAAGT / Celera SNP ID: hCV1207889 / Public SNP ID: rs1998028 /
SNP Chromosome Position: 33344595 / SNP in Genomic Sequence: SEQ ID NO: 436 /
SNP Position Genomic: 106396 / Related Interrogated SNP: hCV1825046 / SNP
Source: dbSNP; Celera; HapMap; HGBASE / Population(Allele,Count): Caucasian
(A,67|T,53) / SNP Type: INTRON /
Context (SEQ ID NO: 2365): /
GTGTGAACTTTTTTTGTAAAAGTCAGATTTTTGTCTCATTCCTTAGTGATTTACAATTAAAGTTGGAGTCACATTACTAAATT
AACTCCAGGAGGGATGG
R
AATTCAGAAACTAGTTGCACTATCTCAGTCCAAACTGCTTCAATGAGAAAGGGTAAGAGGATTAGTCATGTGGTCCTTAAGCA
ACCAGAAACATGAAGTT / Celera SNP ID: hCV1207891 / Public SNP ID: rs2378259 /
SNP Chromosome Position: 33341688 / SNP in Genomic Sequence: SEQ ID NO: 436 /
SNP Position Genomic: 103489 / Related Interrogated SNP: hCV1825046 / SNP
Source: dbSNP; Celera; HapMap; ABI_Val / Population(Allele,Count): Caucasian
(G,127|A,99) / SNP Type: INTRON /
Context (SEQ ID NO: 2366): /
TACCGAGAGAATAAAATATTTATTAAATAATTATTCCCATAGGTTGATGCTAGTAAAGAATTTAACTGAGAAAGAGCTATCAG
AAAAATTAGATAATTAA
S
TTTGAATTCAGGAAAATATAGGTTATCATTAATAATACACCATGATTAGCTTTTAAAGCATAAGATTCAAGAAGAGGCAAGTG
AGCATGATATAATACAG / Celera SNP ID: hCV1207893 / Public SNP ID: rs6087624 /
SNP Chromosome Position: 33336085 / SNP in Genomic Sequence: SEQ ID NO: 436 /
SNP Position Genomic: 97886 / Related Interrogated SNP: hCV1825046 / SNP
Source: dbSNP; Celera; HapMap / Population(Allele,Count): Caucasian

(C,72|G,48) / SNP Type: INTRON /
Context (SEQ ID NO: 2367): /
TTGTACTTTTCTTTTCTATCTTTAGTTTCCCTTTGCCCTGATTTTGTTCACCCACTTTTAGGTAGTGTTTTGTCTCATTATAC
TCTGTACAGTTTCATAA
R
CCACAAAAATCCACTTTAAAAGCCTCCCAGCAAATGGAAATTTAGAACCTGAAGCAGATCGTCAGCACATCCAGACCTATAAC
AATTTGCTCCTTGTAAG / Celera SNP ID: hCV1207895 / Public SNP ID: rs6120708 /
SNP Chromosome Position: 33335526 / SNP in Genomic Sequence: SEQ ID NO: 436 /
SNP Position Genomic: 97327 / Related Interrogated SNP: hCV1825046 / SNP
Source: dbSNP; Celera; HapMap; ABI_Val / Population(Allele,Count): Caucasian
(G,132|A,94) / SNP Type: INTRON /
Context (SEQ ID NO: 2368): /
TGCCTTTAGGGGCCAGGCAGGTAGTAGAGAATAGAAGTGGACTGGTATAAGACAGCAGGAGTCCTGTCAACAGTGCCAAACTG
CAGAGCAGATGCCCTAT
W
TAAAGGCATTTAAACTCGGACTTTTGTTTAAGACACTGCATAGCAACTCAAAACCATTATAGGCCAAATTCAGCCTGCAGGCC
CCCAGTTTGCAGAAAGA / Celera SNP ID: hCV1207897 / Public SNP ID: rs3787222 /
SNP Chromosome Position: 33333040 / SNP in Genomic Sequence: SEQ ID NO: 436 /
SNP Position Genomic: 94841 / Related Interrogated SNP: hCV1825046 / SNP
Source: dbSNP; Celera; HapMap; HGBASE / Population(Allele,Count): Caucasian
(T,65|A,53) / SNP Type: TRANSCRIPTION FACTOR BINDING SITE;INTRON /
Context (SEQ ID NO: 2369): /
ATAAAATATGTAATGCCCTCTAGAAGGGAACCATCCTGGAAGATAAAGATTATGGCATGGATATGCCACAGTCACATCTTGAC
ATCTCAGTCTAAAATCC
K
AGATAAACTCTGCAACAACATGTTTTACTCTCTTGTGGAAACTGAATTTTGTATATTTCCCTCCCTGCTTAGCAAAGCAGTAA
ATTATGCATTTGTGTTT / Celera SNP ID: hCV1207898 / Public SNP ID: rs1018503 /
SNP Chromosome Position: 33325516 / SNP in Genomic Sequence: SEQ ID NO: 436 /
SNP Position Genomic: 87317 / Related Interrogated SNP: hCV1825046 / SNP
Source: dbSNP; Celera; HapMap / Population(Allele,Count): Caucasian
(T,127|G,99) / SNP Type: INTRON /
Context (SEQ ID NO: 2370): /
TTTGGGTCAGATCCTGCCTGGCTTTCTCCTTCTTTTGCTTTCAGGGTCAAACAACTGTTCTTTAGAAGACAGCTGCAAGGACT
ATTATTAATATTAATAT
S
AATGGTAACAACAGCTCCCATATATTGAACATTTACTATTTCCTCATTATGCAGAACATGTTATCTCACTCAGCTTTGAAAAC
AACCATAAAAGGTAGAA / Celera SNP ID: hCV1207902 / Public SNP ID: rs2295352 /
SNP Chromosome Position: 33320055 / SNP in Genomic Sequence: SEQ ID NO: 436 /
SNP Position Genomic: 81856 / Related Interrogated SNP: hCV1825046 / SNP
Source: dbSNP; Celera; HapMap; HGBASE / Population(Allele,Count): Caucasian
(C,78|G,42) / SNP Type: INTRON /
Context (SEQ ID NO: 2371): /
ACAGACCATAACCTGACTGTCAACTATCCCTACCCTTCCACTGTTCACTCCTGCCAGAGGACCCTTTGCTAGTAGCACCGTGG
TTGGGAACTGGGACACA
S
CATTCACTCCCATAGCTTTATGTATAACAGCCGCCCGACACCCAACCATAAGCCAAGGTAAGTCAAGGAGAGGTGAGGCTGGA
GCTGGGGTGGAATTACA / Celera SNP ID: hCV1207903 / Public SNP ID: rs6087623 /
SNP Chromosome Position: 33317662 / SNP in Genomic Sequence: SEQ ID NO: 436 /
SNP Position Genomic: 79463 / Related Interrogated SNP: hCV1825046 / SNP
Source: dbSNP; Celera; HapMap; ABI_Val / Population(Allele,Count): Caucasian
(C,65|G,51) / SNP Type: INTRON /
Context (SEQ ID NO: 2372): /
ACTCTAGCAGTGTAGTCAAATCATTTATATCCATGTCTTAGAACCAACTGGGTGGTAAAGAACTGGGTCAATAATAGTCTTTT
AAGTGACTGTCCAACTA
Y
GTCACTGGGGGAACATTTAAATTTATAACCTAATAAAGGGATTTGTTTTTCAGCCTGGTTGCCTCATTTTGACCTTAAGCAAA
TTTGACCAAATCTACAG / Celera SNP ID: hCV1207909 / Public SNP ID: rs6119512 /
SNP Chromosome Position: 33306904 / SNP in Genomic Sequence: SEQ ID NO: 436 /
SNP Position Genomic: 68705 / Related Interrogated SNP: hCV1825046 / SNP
Source: dbSNP; Celera; HapMap; ABI_Val / Population(Allele,Count): Caucasian
(T,67|C,53) / SNP Type: TRANSCRIPTION FACTOR BINDING SITE;INTRON /
Context (SEQ ID NO: 2373): /
GATCCCCTCCCTCCGGCTCCAGACTCCGCCCCCAGGGACTAACAGAATAACATCCTCCTAATCCCTGATTTGTGTGCAGCGCC

TTAGCTGATGTCACTCC
Y
AGACCCAAAATGAAGGGTCTTAGACCCCAGTGCATGGAAGGGGCAACAGGCCCAGAGAGGAGGATTAGCTCTCTCGAGGTCAC
ATAACCAATTAGAGTCA / Celera SNP ID: hCV1207914 / Public SNP ID: rs910870 /
SNP Chromosome Position: 33292893 / SNP in Genomic Sequence: SEQ ID NO: 436 /
SNP Position Genomic: 54694 / Related Interrogated SNP: hCV1825046 / SNP
Source: dbSNP; HapMap; ABI_val / Population(Allele,Count): Caucasian
(T,67|C,53) / SNP Type: INTRON /
Context (SEQ ID NO: 2374): /
TCTTTGCAACCTGAGAAGGAGTCAGACGGCGCTGCTGGGCCTAGGTTGAGGGGGTCGTCAGGAGTTCAGGGCTGCATGGGACT
CCAGGTGGTTGGGACAG
Y
ACCAGATGGTGGTGGGATCCCCTCCCTCCGGCTCCAGACTCCGCCCCCAGGGACTAACAGAATAACATCCTCCTAATCCCTGA
TTTGTGTGCAGCGCCTT / Celera SNP ID: hCV1207915 / Public SNP ID: rs910869 /
SNP Chromosome Position: 33292777 / SNP in Genomic Sequence: SEQ ID NO: 436 /
SNP Position Genomic: 54578 / Related Interrogated SNP: hCV1825046 / SNP
Source: dbSNP; HapMap; HGBASE / Population(Allele,Count): Caucasian
(C,130|T,96) / SNP Type: INTRON /
Context (SEQ ID NO: 2375): /
TACTAAAAATACAAAAAATTTAGCTGGGCAAGGTGGCGGGCACCTGTAATCCCAGCTACTTGGGAGGCTGAGACAGGAGAGTC
ACTTCGACCCGAGGATC
R
GCGGTTGCAGTGAGCCGAGATTGCACCACTGCACTCCAGCCTGGGCGACAGAGCAAGACTGTCTCAAAAAAAAAGAAAAAAGA
AAAAGAAAAAAATGGGA / Celera SNP ID: hCV1265078 / Public SNP ID: rs6088569 /
SNP Chromosome Position: 33268199 / SNP in Genomic Sequence: SEQ ID NO: 436 /
SNP Position Genomic: 30000 / Related Interrogated SNP: hCV1825046 / SNP
Source: dbSNP; Celera; HapMap / Population(Allele,Count): Caucasian
(G,65|A,37) / SNP Type: UTR3 /
Context (SEQ ID NO: 2376): /
GATCGGCGGTTGCAGTGAGCCGAGATTGCACCACTGCACTCCAGCCTGGGCGACAGAGCAAGACTGTCTCAAAAAAAAAGAAA
AAAGAAAAAGAAAAAAA
Y
GGGAGTGGGATTCTAAAAGAATGCAAAAATAAACCCTGGGGCTGATAGCTTTCTGGACCAGTGAAAGGGGTAGGGTCTCCTTT
GGGGGAGGGGATCATGA / Celera SNP ID: hCV1265079 / Public SNP ID: rs6088570 /
SNP Chromosome Position: 33268295 / SNP in Genomic Sequence: SEQ ID NO: 436 /
SNP Position Genomic: 30096 / Related Interrogated SNP: hCV1825046 / SNP
Source: dbSNP; Celera / Population(Allele,Count): Caucasian (T,141|C,85) /
SNP Type: UTR3 /
Context (SEQ ID NO: 2377): /
ATTTTGGTCAACGTTGGACTGCATATACCACAGTGGTCCCCTACGATTATAGTGGAGCTGAAAAATTCCTCTTGCCTACTGAT
GTTGTAGCCATCATAAC
R
TCGTGGCTTAATTTGTTATCTTTTCTATGTTTCCATATGTTTAGATACACAAATACTTACCACTGTGTTACAATTGCCTGTAG
TATCCAGTATAGTAACA / Celera SNP ID: hCV1265082 / Public SNP ID: rs6088575 /
SNP Chromosome Position: 33273403 / SNP in Genomic Sequence: SEQ ID NO: 436 /
SNP Position Genomic: 35204 / Related Interrogated SNP: hCV1825046 / SNP
Source: dbSNP; Celera; HapMap / Population(Allele,Count): Caucasian
(A,141|G,85) / SNP Type: INTERGENIC;UNKNOWN /
Context (SEQ ID NO: 2378): /
GGCTCAACTTTCCTGCTGCCTTCTACGCCAACTACCATTTCACTTTATTCACCGTTTTGGATTTGAAGCTGCTGCTTGATACT
GACATTTTGTAGATGTA
R
TATGGCTATGCCGGTACGTTTCTGCCTATTGAAGAAGATCATGTTCTTTTAGTATTAAACAGTACAGTTGACTTCCAATCAAT
TAGTTTATATATAGGAA / Celera SNP ID: hCV1265086 / Public SNP ID: rs2378251 /
SNP Chromosome Position: 33276750 / SNP in Genomic Sequence: SEQ ID NO: 436 /
SNP Position Genomic: 38551 / Related Interrogated SNP: hCV1825046 / SNP
Source: dbSNP; Celera; HapMap; ABI_val; HGBASE / Population(Allele,Count):
Caucasian (G,141|A,85) / SNP Type: INTERGENIC;UNKNOWN /
Context (SEQ ID NO: 2379): /
TGGCTATGCCGGTACGTTTCTGCCTATTGAAGAAGATCATGTTCTTTTAGTATTAAACAGTACAGTTGACTTCCAATCAATTA
GTTTATATATAGGAATC
Y
TGTGTAGCCTGCAGTGGGGTTGTGGGGAGCAAGGCAGAAGCAGCCACTCCAGAGAGGATTTACATTTATTTCTTCCAGGCATC

TGAGAGATCATTTCAGA / Celera SNP ID: hCV1265087 / Public SNP ID: rs2889855 / SNP Chromosome Position: 33276853 / SNP in Genomic Sequence: SEQ ID NO: 436 / SNP Position Genomic: 38654 / Related Interrogated SNP: hCV1825046 / SNP Source: dbSNP; Celera; HGBASE / Population(Allele,Count): Caucasian (T,139|C,85) / SNP Type: INTERGENIC;UNKNOWN / Context (SEQ ID NO: 2380): / GGGTTGTATCTCCAGAAGCCCCATCGATGTCAGGTTGGGCAGAGCCAGCCCAGGAAACATGGAACATCTGCCTCTGGCCTCTC ACCTCTTTGTTCTCCTA Y

GAAGCCCGGCTTTTTTTTTCTTTTTTTTGAGACGGAGTCTGCTCTGTCACCCAGGCTGGAGTGCAGTGGCGCGGTCTCAGCTCAC TGCAAGCTCCGCCTCCC / Celera SNP ID: hCV1265092 / Public SNP ID: rs6088578 / SNP Chromosome Position: 33281604 / SNP in Genomic Sequence: SEQ ID NO: 436 / SNP Position Genomic: 43405 / Related Interrogated SNP: hCV1825046 / SNP Source: dbSNP; Celera; HapMap; ABI_Val / Population(Allele,Count): Caucasian (C,143|T,83) / SNP Type: INTERGENIC;UNKNOWN / Context (SEQ ID NO: 2381): / ACCTCAGTTTCCTCACCTATAAAATGAGGGGTTTGGATGAGTTGAGGTCTGAAGCCTCCTCCAGCCCAGGTAATAAATCTTTG TAGTGGACAGGAGGAAT R

GGTCCTAGGGGTTTCAGGAGGGGACTTCCATGGGTACCTCTGACTCTACAGAGAAATGGAAGACTGAGATCTGACTTAGCAGC CTAGAAAGACTTAGCAG / Celera SNP ID: hCV1265109 / Public SNP ID: rs6058108 / SNP Chromosome Position: 33288655 / SNP in Genomic Sequence: SEQ ID NO: 436 / SNP Position Genomic: 50456 / Related Interrogated SNP: hCV1825046 / SNP Source: dbSNP / Population(Allele,Count): Caucasian (A,130|G,96) / SNP Type: INTRON / Context (SEQ ID NO: 2382): / CGAGCGCTCTGAGTATCCTTGGACATGGCAACTCCAAGCCTCAGTTTACCCATCTGAAATATGGGGAAAATCATAGTGCTTGT GAAGAGCAAATGGAGGT R

GGCTTGGAAAGCACTTGTCACCGTGCCTGACACATACTGTCTAATAATAAGCTTAATGGGATATAGATAAAAGCTTTCATTAT TATTTGCCTCTAAATCC / Celera SNP ID: hCV2521776 / Public SNP ID: rs6087634 / SNP Chromosome Position: 33413809 / SNP in Genomic Sequence: SEQ ID NO: 436 / SNP Position Genomic: 175610 / Related Interrogated SNP: hCV1825046 / SNP Source: dbSNP; Celera / Population(Allele,Count): Caucasian (G,87|A,139) / SNP Type: INTERGENIC;UNKNOWN / Context (SEQ ID NO: 2383): / CAGCTCCGGGAAAGACTCCTTCCCTCTGCTTGAGGAGAGGAGAGGGAAGAGTAAAAGAGGATTTTGTCTTGCAACTTGGATAC CAGCTCAGCCACAGTAG S

AGAGGGCAGAGTCCTGAAGTCCCCATTCTAGACCCTAGCTCCTGGATGACATTTCTAGACATACCCTGGGCCAGAAGGGAACC CACTGCCTTGAAAGGAA / Celera SNP ID: hCV2969302 / Public SNP ID: rs6120730 / SNP Chromosome Position: 33385102 / SNP in Genomic Sequence: SEQ ID NO: 436 / SNP Position Genomic: 146903 / Related Interrogated SNP: hCV1825046 / SNP Source: dbSNP; Celera / Population(Allele,Count): Caucasian (G,61|C,49) / SNP Type: UTR3;INTRON / Context (SEQ ID NO: 2384): / TATCTTTTTGAATAATGTTTTTCTTTTGAACCATGTGAATATATTACCTATTGAAAACAAAAGTTAGCTATAAATAAAACCTC TTAAACGCTTAGTATCA R

TATTTCTACTCTTACCCCATCTCAGATATGCAACAACAAAAATGTCTCCTGCTTTTTATCTCCTTTTTTTGAAGGGGGCTCCTC TGGCTACATTTCCCCAC / Celera SNP ID: hCV2969304 / Public SNP ID: rs2424997 / SNP Chromosome Position: 33384805 / SNP in Genomic Sequence: SEQ ID NO: 436 / SNP Position Genomic: 146606 / Related Interrogated SNP: hCV1825046 / SNP Source: dbSNP; Celera; HapMap; ABI_Val; HGBASE / Population(Allele,Count): Caucasian (A,99|G,127) / SNP Type: UTR3;INTRON / Context (SEQ ID NO: 2385): / CTCACACTAATGAGGAGTGTGTTACAAGAACACAGGAGCCAGTTTTAAGGGTTTTCCACTGGGCAAATTTGAGACAATTTGAG TACCAAAAGAGAATAAT K

ACTAAAATTGATATCATGAATAAGAATCCATGAATCTCCAGCAATAACTCAGAGAGTGAAAAACTTTAAAAAAAAAACACTTCT TCCCATCATTGGAGGTA / Celera SNP ID: hCV2969305 / Public SNP ID: rs6060052 / SNP Chromosome Position: 33382665 / SNP in Genomic Sequence: SEQ ID NO: 436 / SNP Position Genomic: 144466 / Related Interrogated SNP: hCV1825046 / SNP

Source: dbSNP; Celera; HapMap / Population(Allele,Count): Caucasian (T,99|G,125) / SNP Type: UTR5;TFBS SYNONYMOUS;INTRON / Context (SEQ ID NO: 2386): / ACCCTATGAGATTACTATTATCCTTAAAGACTGAGGCGCAATGAGGTTTTATTTGTCCAAGGTCACACAATCAGCAAGCAGCA GTGCTTAATAAGGACCT S

AGACAGACTCCACCAAAAAGAGCTCACAATTTGAAAGCAAGCCCCATTCAATAAATGATGCTGGGATCACTGGTTATACATAG GGAAAAAAAATGAAACT / Celera SNP ID: hCV2988252 / Public SNP ID: rs2425005 / SNP Chromosome Position: 33407715 / SNP in Genomic Sequence: SEQ ID NO: 436 / SNP Position Genomic: 169516 / Related Interrogated SNP: hCV1825046 / SNP Source: dbSNP; Celera; HGBASE / Population(Allele,Count): Caucasian (G,42|C,78) / SNP Type: UTR5;INTRON / Context (SEQ ID NO: 2387): / ATCCTCACAAAACCCTATGAGATTACTATTATCCTTAAAGACTGAGGCGCAATGAGGTTTTATTTGTCCAAGGTCACACAATC AGCAAGCAGCAGTGCTT M

ATAAGGACCTGAGACAGACTCCACCAAAAAGAGCTCACAATTTGAAAGCAAGCCCCATTCAATAAATGATGCTGGGATCACTG GTTATACATAGGGAAAA / Celera SNP ID: hCV2988253 / Public SNP ID: rs6087632 / SNP Chromosome Position: 33407704 / SNP in Genomic Sequence: SEQ ID NO: 436 / SNP Position Genomic: 169505 / Related Interrogated SNP: hCV1825046 / SNP Source: dbSNP; Celera / Population(Allele,Count): Caucasian (A,132|C,94) / SNP Type: TRANSCRIPTION FACTOR BINDING SITE;UTR5;INTRON / Context (SEQ ID NO: 2388): / TTTTTTTAAGTTTTATTTATTTTAGGTTTTATCTATTATTGTTACTATTGTTCATCTCCATTTCCACCCTTAATTCCAACAAC ACCCTTATAACAATCCT S

TTCTCCAAGCAAAGTGGACTCTTTACCATCTCCTAAATACCCACCTCTGTGTTTATGCTTTCACTCAGCCTGGAAAATACAGC ACTGTCTCATCTCCAAG / Celera SNP ID: hCV2988254 / Public SNP ID: rs6060064 / SNP Chromosome Position: 33406339 / SNP in Genomic Sequence: SEQ ID NO: 436 / SNP Position Genomic: 168140 / Related Interrogated SNP: hCV1825046 / SNP Source: dbSNP; Celera; HapMap; ABI_Val / Population(Allele,Count): Caucasian (C,87|G,139) / SNP Type: TRANSCRIPTION FACTOR BINDING SITE;INTRON / Context (SEQ ID NO: 2389): / CACATATTATAAACATATTATAAACCGTAAGTCAGAGGCTCAGTGAGATTTTTCCCACGTTGTTACATAAAAAAAAACAAAGAA ATAAATCTGTGCCCAAA Y

TGCAACAAAGAGAAAAAAAATTACAAATGAATAATACAAAAACAGAAATAAAATCTAGAAGAGGTCAGGCGTGGTAGCTCACG CCTGTAATCCCAGCACC / Celera SNP ID: hCV27166997 / Public SNP ID: rs6088615 / SNP Chromosome Position: 33400474 / SNP in Genomic Sequence: SEQ ID NO: 436 / SNP Position Genomic: 162275 / Related Interrogated SNP: hCV1825046 / SNP Source: dbSNP; Celera / Population(Allele,Count): Caucasian (T,131|C,95) / SNP Type: INTRON / Context (SEQ ID NO: 2390): / CTCACCCAATCTAGTGAGAAGTAAGAGAGTAAACTAGCTCCATATCTCTCCTTTCTCCCCATGCTGATCCTACACTTCACAAT CAGGCTTCCTTTCCTAA Y

GTACAGCTTTCATCACATCACTGGCGAAAAACATTTGATGACTTCCCACTACCTAGTCAATCAAGTTCAAACTTCCCAGCTTG GAATAGAAATGGGTATT / Celera SNP ID: hCV3010271 / Public SNP ID: rs2889861 / SNP Chromosome Position: 33405664 / SNP in Genomic Sequence: SEQ ID NO: 436 / SNP Position Genomic: 167465 / Related Interrogated SNP: hCV1825046 / SNP Source: dbSNP; Celera; HGBASE / Population(Allele,Count): Caucasian (C,87|T,139) / SNP Type: INTRON / Context (SEQ ID NO: 2391): / AGCCCACAAAACTGAAGAACTGCTATTATACTAGAAGAAAAAATTTATGAAATTCAATTTTAACTAGTAGCTAGTGGCACTAT AAACTGGTAGTTGTTCT R

AAGAATAATCTGACAATGTGGCTAAAAAGTGATAGCCTCAGACCTAATGAAATCATTTTAGGGAATATATTCTAAGAAAAAAA CTGGAAGAAAAAGATT / Celera SNP ID: hCV7593328 / Public SNP ID: rs1018447 / SNP Chromosome Position: 33401707 / SNP in Genomic Sequence: SEQ ID NO: 436 / SNP Position Genomic: 163508 / Related Interrogated SNP: hCV1825046 / SNP Source: dbSNP; HapMap / Population(Allele,Count): Caucasian (G,85|A,139) / SNP Type: TRANSCRIPTION FACTOR BINDING SITE;INTRON / Context (SEQ ID NO: 2392): /

TATAGTTAATTTATTAACTGATAATGAATATTATAACTGACATAGAATACACAATTCATCACAAAGTAAAGGTATGGTCCTGG
CCTTCTGCTTTATAGAG
W
TTGTATATCAAATAAAGCATATGACCAACTCTAGGGCTGCATTCCTCCTTCCATCAAAGCCATAGTATTTCATTTGTGTATAC
ATCTAAATGTAACTCTA / Celera SNP ID: hCV16013581 / Public SNP ID: rs2253484 /
SNP Chromosome Position: 33405263 / SNP in Genomic Sequence: SEQ ID NO: 436 /
SNP Position Genomic: 167064 / Related Interrogated SNP: hCV1825046 / SNP
Source: dbSNP; HGBASE / Population(Allele,Count): Caucasian (A,85|T,139) /
SNP Type: INTRON /
Context (SEQ ID NO: 2393): /
CACCAATTACTCCAGCCTCCTGGTGTTCATGCCTTTGTGTAATCTCTTCCTCCTGAGTATGGACTGGCCTGGTGATTTGCTTC
TAACTAACGGACTATGG
W
AAATGTGATAGGATGCCACTCCATGATTATAAAAAATTGTGATTTCCATCTTCCTAGGAGACTTGCTATTCCTTTCTCAGCTT
TGTGATGTTGAAGAAGC / Celera SNP ID: hCV16013593 / Public SNP ID: rs2425001 /
SNP Chromosome Position: 33394868 / SNP in Genomic Sequence: SEQ ID NO: 436 /
SNP Position Genomic: 156669 / Related Interrogated SNP: hCV1825046 / SNP
Source: dbSNP; HapMap; HGBASE / Population(Allele,Count): Caucasian
(A,48|T,72) / SNP Type: INTRON /
Context (SEQ ID NO: 2394): /
GTGCTGGCTTTAGGTCTGACCCAGTGTAGTTTCAGTGGTGGGGTGGCCACAGGGGTGCTTGTGTAACCTCTCCCCTAGCTCCA
GGTAGCTCAGCATAGAC
R
GACAAATTCCAGTTGTTTGGGAGAGAGTAAGAGGAGAGAACAAGAGTCTCTCCCTGGTAATCCAGAGAATTATTCTGGATTTT
ACCTAAAACCACCAAGG / Celera SNP ID: hCV16013594 / Public SNP ID: rs2424999 /
SNP Chromosome Position: 33386430 / SNP in Genomic Sequence: SEQ ID NO: 436 /
SNP Position Genomic: 148231 / Related Interrogated SNP: hCV1825046 / SNP
Source: dbSNP / Population(Allele,Count): Caucasian (G,86|A,140) / SNP Type:
TRANSCRIPTION FACTOR BINDING SITE;INTRON /
Context (SEQ ID NO: 2395): /
TCTGCTGACACCTCTGATTTCCTTAACCAAAAACTACACCACACTTGACAGCTTTGCAATGGTTTCCAAAACTCTATTACTCT
GCCAAAAAAACCTTTTC
R
CCCTTATTATCAATGAGAATTCTCCCCTCATTTTCCTTTCACTTTTTTTTTTTTTTTTTGAGACGGAGTCACGCTCTGTTGCCT
GGGCTGGAGTGCAGTGG / Celera SNP ID: hCV27166995 / Public SNP ID: rs6088618 /
SNP Chromosome Position: 33409350 / SNP in Genomic Sequence: SEQ ID NO: 436 /
SNP Position Genomic: 171151 / Related Interrogated SNP: hCV1825046 / SNP
Source: dbSNP; Celera; HapMap / Population(Allele,Count): Caucasian
(G,61|A,59) / SNP Type: UTR5;INTRON /
Context (SEQ ID NO: 2396): /
CTATTTCTACTATTCCAGCTCCTCTTACCTAAGAAAAAAAAATCACATATTAATAAACAGAATTTCCACACTATACTGCTATCA
TTCCCCTATTTACCAAC
W
GCATATATGCTAATTTGTGTCAAAGAGAACACTACCGGCTCCCAAACTTGCCAATTAGGGGTCCACAAACTACGTCCTGTGGG
CCAAATGCAGCCCACTG / Celera SNP ID: hCV27167007 / Public SNP ID: rs2180276 /
SNP Chromosome Position: 33360785 / SNP in Genomic Sequence: SEQ ID NO: 436 /
SNP Position Genomic: 122586 / Related Interrogated SNP: hCV1825046 / SNP
Source: dbSNP; Celera; HapMap; ABI_Val; HGBASE / Population(Allele,Count):
Caucasian (T,65|A,51) / SNP Type: INTRON /
Context (SEQ ID NO: 2397): /
AATTATTCTTTAATTTGGCTCCAAGAGCTTTAATACTGAAAAACCAAAAGAGCTACAGTACTCATTTCTGATTTAAAAGGTAA
CTGTGATACTACAATAT
R
TATTTTTTTGGTTTTTGTCCCACGTTCCTGGCTCACAGCTCCTATAACTCTTGTAATTTCCTAAGTGAGCCATCTTTTTGTTAAA
ATATTTGGCCTTTTGCC / Celera SNP ID: hCV27167022 / Public SNP ID: rs6060013 /
SNP Chromosome Position: 33308809 / SNP in Genomic Sequence: SEQ ID NO: 436 /
SNP Position Genomic: 70610 / Related Interrogated SNP: hCV1825046 / SNP
Source: dbSNP; Celera / Population(Allele,Count): Caucasian (A,126|G,98) /
SNP Type: INTRON /
Context (SEQ ID NO: 2398): /
CCATATACTCAGCAGGCTGAGGAACAAAGAATTGCTCGGGCCCAGGAGGCAGAGGTTGCAGTGAGCCGAGATTGCACTTGGGT
AACACAGCAAGACTCTT
Y

CTCAAAAAAAAAAAAAAAAAAAAAAAAAAAAAAAAAATCGGCCGGGCGCGGTGGCTCACACCTGTAATCCCAGCACTTTGGGAGGCCG
AGGCAGGCGGATCACGA / Celera SNP ID: hCV27167045 / Public SNP ID: rs2378252 /
SNP Chromosome Position: 33283403 / SNP in Genomic Sequence: SEQ ID NO: 436 /
SNP Position Genomic: 45204 / Related Interrogated SNP: hCV1825046 / SNP
Source: dbSNP; Celera; HGBASE / Population(Allele,Count): Caucasian
(T,115|C,105) / SNP Type: INTERGENIC;UNKNOWN /
Context (SEQ ID NO: 2399) /
AGGAAACAAATCTTTTTACAGAGCTCCACACATAAGGAAGTTAAAACATTTTATTTGTACTAGTGAAAACAGAAGACCTAAAT
ACCTAACGATAGTAGAT
Y

AGTTAGCTAAATCATGGCACATCCATATGGAGTCATGAAAAATCATGTTGTAGATTATTTAATGACAAAACGATTTATGACAC
ATTACCAAGTGGGAAAA / Celera SNP ID: hCV27893018 / Public SNP ID: rs4911441 /
SNP Chromosome Position: 33316546 / SNP in Genomic Sequence: SEQ ID NO: 436 /
SNP Position Genomic: 78347 / Related Interrogated SNP: hCV1825046 / SNP
Source: dbSNP; HapMap; HGBASE / Population(Allele,Count): Caucasian
(T,62|C,42) / SNP Type: INTRON /
Context (SEQ ID NO: 2400): /
TGCCCTCTGAATTTGGTGCTCATTAATATTAACTGATATAGTTAATTTATTAACTGATAATGAATATTATAACTGACATAGAA
TACACAATTCATCACAA
R

GTAAAGGTATGGTCCTGGCCTTCTGCTTTATAGAGATTGTATATCAAATAAAGCATATGACCAACTCTAGGGCTGCATTCCTC
CTTCCATCAAAGCCATA / Celera SNP ID: hCV29372820 / Public SNP ID: rs6120739 /
SNP Chromosome Position: 33405227 / SNP in Genomic Sequence: SEQ ID NO: 436 /
SNP Position Genomic: 167028 / Related Interrogated SNP: hCV1825046 / SNP
Source: dbSNP / Population(Allele,Count): Caucasian (A,87|G,139) / SNP Type:
INTRON /
Context (SEQ ID NO: 2401): /
TCAAGGCCTCGATATTCCCTTCCTTCAAGAGGGGCCAAACTTTCTGGGCTTTTGGTAATGGAGTCACTAGCTCCCTGTGGCTA
CATATGGGTTGGGATAC
R

TGAATTGTGCTGGGTGGACACTAGTTGGGGGTGGCCCCACCTTCTTAATCCCTGTAGATCTCCCACCCCCATCCCACCAGAGA
AGCCAGCATCTTAAGCC / Celera SNP ID: hCV29372834 / Public SNP ID: rs6060001 /
SNP Chromosome Position: 33294353 / SNP in Genomic Sequence: SEQ ID NO: 436 /
SNP Position Genomic: 56154 / Related Interrogated SNP: hCV1825046 / SNP
Source: dbSNP / Population(Allele,Count): Caucasian (A,119|G,107) / SNP Type:
INTRON /
Context (SEQ ID NO: 2402): /
GACTCCATCTTGGGGAAAAAAAAAATTGTAAAATATAAATAAAAACGAACTGAAAATCACCCAAATGCCAAATAACCAGAGTA
TTTTAGACTTTGGTTGT
Y

ATATATCCATTAAAAATGATAAATATACAGATGTGTAGAAAAGTACGTCAATATATTGCCAAATGAAAAAAGACTAAAAAAAC
CTTTATATGAATTAAAT / Celera SNP ID: hCV30503911 / Public SNP ID: rs4911161 /
SNP Chromosome Position: 33402231 / SNP in Genomic Sequence: SEQ ID NO: 436 /
SNP Position Genomic: 164032 / Related Interrogated SNP: hCV1825046 / SNP
Source: dbSNP; HGBASE / Population(Allele,Count): Caucasian (C,87|T,139) /
SNP Type: INTRON /
Context (SEQ ID NO: 2403): /
TCTGCAGTAGGGGGCCTCCCTCTTCATCTTCACCCAGGGAAGGATTGGTTAAGGGCTTACCTCGGTCTGGGTCCAACCCTCAG
TGCTCATCCTTAAGGGC
Y

CCTGGGAACTGACAGCTCCCTCCCTTCATCATCACCTCCCACTGACCTGCTGTCTCACTGAGCCCAAGCAACCTGATCTTCCT
CCTACCCCTGGATGCTG / Celera SNP ID: hCV29693120 / Public SNP ID: rs6060003 /
SNP Chromosome Position: 33294945 / SNP in Genomic Sequence: SEQ ID NO: 436 /
SNP Position Genomic: 56746 / Related Interrogated SNP: hCV1825046 / SNP
Source: dbSNP; HapMap / Population(Allele,Count): Caucasian (T,119|C,107) /
SNP Type: TRANSCRIPTION FACTOR BINDING SITE;INTRON /
Context (SEQ ID NO: 2404): /
GATTTTGCCATGTTGGCCAGGCTGGTATCGAACTCCTGGGCTTAAGCAATCCTCCTGCCTTGGCCTCCCAAAGTGCTATGATT
ACTGGCCTGCACTGCCG
Y

GCCCAGCTCTGCTGCCTCTTTTTACAGCCTGCCGGCCCAGACCAGAGTCTCAGAATACTAAGCCCACTTTCTGGGGGCCAGGGT
CAGTAGATAGCTTTGCT / Celera SNP ID: hCV29674936 / Public SNP ID: rs6087618 /
SNP Chromosome Position: 33288407 / SNP in Genomic Sequence: SEQ ID NO: 436 /

440

SNP Position Genomic:    50208 / Related Interrogated SNP:    hCV1825046 /  SNP Source:    dbSNP / Population(Allele,Count):    Caucasian (C,120|T,98) /  SNP Type:    INTRON /
Context                    (SEQ ID NO: 2405): /
CAGCTCTGCTGCCTCTTTTTTACAGCCTGCCGGCCCAGACCAGAGTCTCAGAATACTAAGCCCACTTTCTGGGGCCAGGGTCAG
TAGATAGCTTTGCTGCT
S
TATGATTCTAAGCAAGTATCTTGACCTGCATGTGTCTCAGTGCACCTCAGTTTCCTCACCTATAAAATGAGGGGTTTGGATGA
GTTGAGGTCTGAAGCCT / Celera SNP ID:    hCV29783257 / Public SNP ID:    rs6087619 /
SNP Chromosome Position:    33288511 / SNP in Genomic Sequence:    SEQ ID NO: 436 /
SNP Position Genomic:    50312 / Related Interrogated SNP:    hCV1825046 / SNP
Source:    dbSNP; HapMap; ABI_val / Population(Allele,Count):    Caucasian
(G,130|C,96) / SNP Type:    TRANSCRIPTION FACTOR BINDING SITE;INTRON /
Context                    (SEQ ID NO: 2406): /
GAAGCCCCCTACTCCTTCCATTCCACTTCCTCCCTCTTATTGCAATTGTGGTTAACAGTTCTGTTCTTGTTTATTGACCTTTT
CTGTATCTGTAGGTAAA
Y
TGCACTTTTTTCTGTAGATAAACATTTTTTTGTATGCTTTTAATCTTTGTATAAATGGCACCTTACTATATGCATTCTTTGTCAG
CTTCCTTTTTCACTTAA / Celera SNP ID:    hCV29945782 / Public SNP ID:    rs6087626 /
SNP Chromosome Position:    33347328 / SNP in Genomic Sequence:    SEQ ID NO: 436 /
SNP Position Genomic:    109129 / Related Interrogated SNP:    hCV1825046 / SNP
Source:    dbSNP / Population(Allele,Count):    Caucasian (C,132|T,94) / SNP Type:
INTRON /
Context                    (SEQ ID NO: 2407): /
CCCAAAGTGCTGGGATTACAGGCGTGAGCCACCGCACCTGGCCCTTAGCTCTCTTTAATGAATGCAAACTTAACATGGGATTT
AAAAGCATATTTTCCAA
Y
GTACTTTCAATAAATAAAACTACTGCTATTGCCATTTAGCCCATTCTCTTTCCTCCAAGTAAAAAAAGGAGTCATGCTTTCTCT
TGTGGGATTAGGTCATC / Celera SNP ID:    hCV30072029 / Public SNP ID:    rs6119516 /
SNP Chromosome Position:    33328024 / SNP in Genomic Sequence:    SEQ ID NO: 436 /
SNP Position Genomic:    89825 / Related Interrogated SNP:    hCV1825046 / SNP
Source:    dbSNP; HapMap / Population(Allele,Count):    Caucasian (C,132|T,94) /
SNP Type:    INTRON /
Context                    (SEQ ID NO: 2408): /
ATAGTACCTGGCACACCACAGGCAATGAACGGAAAGCCGTAAGATGGAGAAAGGACCATAAGAAAGAAGAGAGAGGTTAAGTT
TAAATTAATAGAATAGG
Y
TCATGCCTATAATCCCAGCACTTCGGGAGGCCGAGGCAGGTGGATCACGAGGTCAGGAGTTCAAGAATAGCCCAGCCAACATA
ATGAAACCCCGTCTCTA / Celera SNP ID:    hCV30162181 / Public SNP ID:    rs6120723 /
SNP Chromosome Position:    33364655 / SNP in Genomic Sequence:    SEQ ID NO: 436 /
SNP Position Genomic:    126456 / Related Interrogated SNP:    hCV1825046 / SNP
Source:    dbSNP / Population(Allele,Count):    Caucasian (C,132|T,94) / SNP Type:
INTRON /
Context                    (SEQ ID NO: 2409): /
TCATCTATAAGACAGAATTTACTTTCCTAATAGATACTGTGAAAATTAGATGAGATAACACATCTAGGGCACTTGTAGCTCAA
TCTATTAAGGATGCATG
R
CATGGAACAACATTCAAATAAATGGTGGCTATTAACCTCCTCACAGGGATGATATGAAGATTAAATGAAAAAAAATATTGTGAA
AGTGTCTTATGAAGTTG / Celera SNP ID:    hCV30378399 / Public SNP ID:    rs6141509 /
SNP Chromosome Position:    33319388 / SNP in Genomic Sequence:    SEQ ID NO: 436 /
SNP Position Genomic:    81189 / Related Interrogated SNP:    hCV1825046 / SNP
Source:    dbSNP; HapMap / Population(Allele,Count):    Caucasian (G,127|A,99) /
SNP Type:    INTRON /
Context                    (SEQ ID NO: 2410): /
ACAGATGCGTTTTGGAGTAGTAGACTGATTTATTAGCTAGTTCAACAAGTCGCTTAACCCTTGTGTGCTGAAAAATGTGCTGA
TTATAGGCTAACTAGGG
M
CAGTTGAGGAAGGCTTAGAAATTTTTTTTTTTGTTTTGGAAACGGAGTTTCGCTCTTATTGCCCGGGTTGGAGTGCAATGGCGC
GATCTCAGCTCACTGCA / Celera SNP ID:    hCV30360331 / Public SNP ID:    rs6088568 /
SNP Chromosome Position:    33265971 / SNP in Genomic Sequence:    SEQ ID NO: 436 /
SNP Position Genomic:    27772 / Related Interrogated SNP:    hCV1825046 / SNP
Source:    dbSNP; HapMap; ABI_val / Population(Allele,Count):    Caucasian
(A,141|C,85) / SNP Type:    INTERGENIC;UNKNOWN /

Context                    (SEQ ID NO: 2411): /
GGAGACGGAGCTTGCAGAGAGCCGAGATCGCACCACTGCACTCCAGCCTGGGTGACAGAGCAAGACTCCGTTTCAAAAAAAAA
AAAAAAAAAAAAAAAAAA
R
ACAAGACAAGACTTAGGGCGACACATGGTGGTGATTGTTGCACAACATTATGAGTACATTTAATTTCCACTAAACTGTATACT
TAATAATGGTTAACATG / Celera SNP ID:   hCV29837701 /  Public SNP ID:   rs6060045 /
SNP Chromosome Position:   33366788 /  SNP in Genomic Sequence:   SEQ ID NO: 436 /
SNP Position Genomic:   128589 /  Related Interrogated SNP:   hCV1825046 /  SNP
Source:   dbSNP /  Population(Allele,Count):   Caucasian (G,127|A,99) /  SNP Type:
   TRANSCRIPTION FACTOR BINDING SITE;INTRON /
Context                    (SEQ ID NO: 2412): /
GAGATAAAATGGGCAGTTTTTTGTGACGAGTTTGATATAAGGGTGAGGGAGAGAGGTATCAAGGAGGACTTGTCTAATGGATA
GATGGTGGTGCCATTTA
Y
TGAGATGGGGTACAATGGCAGAGGACCTCACTGTTGGAGGTGAAAAATCACAAGTTTGGTTTTAGATATGTTGAGTCTGAGGT
TCCAGTAAGGATGTGCA / Celera SNP ID:   hCV29963933 /  Public SNP ID:   rs6088590 /
SNP Chromosome Position:   33313566 /  SNP in Genomic Sequence:   SEQ ID NO: 436 /
SNP Position Genomic:   75367 /  Related Interrogated SNP:   hCV1825046 /  SNP
Source:   dbSNP; HapMap; ABI_val /  Population(Allele,Count):   Caucasian
(T,132|C,94) /  SNP Type:   INTRON /
Context                    (SEQ ID NO: 2413): /
ACCACTCTCAACTCTCAGCTTGTAGAGGTCCCTCTTGGGTCCTCCTCTTGTGGCCCTCTCCACACATAGCATTTGGTCCTTCA
TGGGAGCACCAGCTGCT
S
GAAAAAATGATTCTGTTAGGTAAAAATACTATACATTTTTGAAACATGCACCAATTATTTAGCTCTGTGATTGTGGGCAATTT
CTTCAAGCAGCCTGAGC / Celera SNP ID:   hCV29982073 /  Public SNP ID:   rs6088580 /
SNP Chromosome Position:   33285053 /  SNP in Genomic Sequence:   SEQ ID NO: 436 /
SNP Position Genomic:   46854 /  Related Interrogated SNP:   hCV1825046 /  SNP
Source:   dbSNP; HapMap /  Population(Allele,Count):   Caucasian (G,48|C,48) /  SNP
Type:   ACCEPTOR SPLICE SITE;INTRON /
Context                    (SEQ ID NO: 2414): /
AACAGAATGTTTCTTGCACTTAATCCAAAATTTTGACTATTGTTATTGTAAGAGAGCACCACCTTGTGGAGAGTTAAAATATA
GCTAGTCTGTGGCTGCT
R
AGCTGAAAGGCTGCACTCACCCAAGTCCTTCTAAACTGTAGGGTTCTGATTCACAGGGTCTGATGGTAAAACAGAAAGTTAAT
GAGGTATGGCAGTTTCC / Celera SNP ID:   hCV30594383 /  Public SNP ID:   rs6060038 /
SNP Chromosome Position:   33357715 /  SNP in Genomic Sequence:   SEQ ID NO: 436 /
SNP Position Genomic:   119516 /  Related Interrogated SNP:   hCV1825046 /  SNP
Source:   dbSNP; HapMap /  Population(Allele,Count):   Caucasian (G,127|A,99) /
SNP Type:   INTRON //

Gene Number:   100 /  Gene Symbol:   CRB1 - 23418 /  Gene Name:   crumbs homolog 1
(Drosophila) /  Chromosome:   1 /  OMIM NUMBER:   604210 /  OMIM Information:
Retinitis pigmentosa-12, autosomal recessive, 600105 (3);/Leber congen / ital
amaurosis, 204000 (3); Pigmented paravenous chorioretinal atrophy, 172870 (3) //
/  Genomic Sequence (SEQ ID NO: 437): //   /  SNP Information /
Context                    (SEQ ID NO: 2415): /
CATTTGGTAGGATATTGCTTCGGTAGTTACAAATATTCTCCTCCAGATTCGATACGGTCTTGTGCTGCTTAACAAATCTTAAA
AGCAAGACTTAAAAGAA
Y
CAAACTGTTTCTAAAAAACTGCATTGCAGAACACAGCTCAAGAAGGCATGCATAGAAACAGGAAAATAAGACCCAAATGAGGA
GAAAAATAAATCAATCA / Celera SNP ID:   hCV836489 /  Public SNP ID:   rs616675 /  SNP
Chromosome Position:   197257556 /  SNP in Genomic Sequence:   SEQ ID NO: 437 /
SNP Position Genomic:   30222 /  Related Interrogated SNP:   hCV2532034 /  SNP
Source:   dbSNP; HapMap /  Population(Allele,Count):   Caucasian (C,10|T,100) /
SNP Type:   INTRON /
Context                    (SEQ ID NO: 2416): /
TTAAATTCTATGTTTTCTCATCTGCCTAATGGGAATAATTATTTTAAACTGCATAGGAGTGTTGTGAGGATTAAATGAAAAAT
GAATGTGTGACACTCTA
Y
GACGTCTTAACTTGATTTGATTTATAGTCAGACTTGAGATTCACAATAGACTTCATCAAGCACCAATTTTACTTGTCTTGAAG
AAAATGCACAGGACCAG / Celera SNP ID:   hCV1742489 /  Public SNP ID:   rs476390 /
SNP Chromosome Position:   197253267 /  SNP in Genomic Sequence:   SEQ ID NO: 437 /

SNP Position Genomic: 25933 / Related Interrogated SNP: hCV2532034 / SNP Source: dbSNP; Celera; HapMap; ABI_Val; HGBASE / Population(Allele,Count): Caucasian (T,10|C,110) / SNP Type: INTRON /
Context (SEQ ID NO: 2417): /
TCTGTGCACCATACTTGCTCATGTAGTCAGGACTGTTGACAACCAGAGGTGATCACTTGGTGGCTTTTGCAGCTCTGGCACCC
TTTAGCCTACTCTTCAC
R
TGTTGGGAAGTGCCATGAAGGGGGGTTGAAAGCCACTGAAGGACTTTAAATAGGAACCCTGGCATGCTCAGACTTGGCCAGAA
TGAGAAGGGAATAAAGA / Celera SNP ID: hCV8356383 / Public SNP ID: rs1764800 /
SNP Chromosome Position: 197250615 / SNP in Genomic Sequence: SEQ ID NO: 437 /
SNP Position Genomic: 23281 / Related Interrogated SNP: hCV2532034 / SNP Source: dbSNP; HapMap; HGBASE / Population(Allele,Count): Caucasian (G,21|A,203) / SNP Type: INTRON //

Gene Number: 101 / Gene Symbol: FAM149A - 25854 / Gene Name: family with sequence similarity 149, member A / Chromosome: 4 / OMIM NUMBER: / OMIM Information: // Genomic Sequence (SEQ ID NO: 438): // / SNP Information /
Context (SEQ ID NO: 2418): /
CCAGGCCATCTCACCGCTTCTGGGAAGAGAACCCAGGCAAGTGTGGAGAGGAGGAGAGGAGAGGTCCCAGGCAAGCAGGACAA
TGAGGAGCCATGATGCC
Y
GGCCTGTGCTTCTCCCAGTTCTCTCTCTGCCTTCCTCACCCTGGAGTTACCAGAGGCTGATACACGTTGACCACACCAGTGCT
CCCTTGCCCTCCAACCT / Celera SNP ID: hCV2103346 / Public SNP ID: rs11733307 /
SNP Chromosome Position: 187083516 / SNP in Genomic Sequence: SEQ ID NO: 438 /
SNP Position Genomic: 94083 / SNP Source: dbSNP; Celera; HapMap; ABI_Val /
Population(Allele,Count): Caucasian (C,90|T,134) / SNP Type: INTRON /
Context (SEQ ID NO: 2419): /
TTAGAGTTGTCATCGAATCAAATTAAAGAGGTAAGAAGTAAGGTAAAATTATTTTGCATTCTGCCTTTAAGGTGGATAGTCCC
TATCTGTGTCACATACA
Y
AGGAATGTAATGCTCTCTAGCCTCTGCCTGTCTTCCAGCAATCCTTCCCACCTACTGCTTGGGGGCATTGGTGTCATCCTCCT
GAGAGCTCAGGCAGCCC / Celera SNP ID: hCV447407 / Public SNP ID: rs5743312 /
SNP Chromosome Position: 187000256 / SNP in Genomic Sequence: SEQ ID NO: 438 /
SNP Position Genomic: 10823 / SNP Source: Applera /
Population(Allele,Count): Caucasian (C,37|T,3) African American (C,35|T,3) total (C,72|T,6) / SNP Type: INTRON / SNP Source: dbSNP; Celera; HapMap /
Population(Allele,Count): Caucasian (C,193|T,33) / SNP Type: INTRON /
Context (SEQ ID NO: 2420): /
ATAAATCTTGGTATTTAGGTTGTACCTAAAAATGATAACTTTTAGACTGTAATCAGAGGTAGATGCTATCAATTTGTTAGTGA
GGAGGGGAGGAGTTTTC
R
ATGCCACTTAGTTTACAATGTCTAATTGTTCAAATATACATCTAGGTTGTAAAGGAGACTCAACGGTAAGTTATAGTGACCAT
TGAAAAATGTAAAGGGG / Celera SNP ID: hCV79084 / Public SNP ID: rs1519312 / SNP
Chromosome Position: 187008844 / SNP in Genomic Sequence: SEQ ID NO: 438 /
SNP Position Genomic: 19411 / Related Interrogated SNP: hCV3230113 / SNP Source: dbSNP; Celera; HGBASE / Population(Allele,Count): Caucasian (G,86|A,140) / SNP Type: INTERGENIC;UNKNOWN /
Context (SEQ ID NO: 2421): /
AGATGGCTGGTGCTGAGGGTGACACCTTGGAAACACCAGGAAACAAGCCATACACCCTGACAATGTGTGACTGAAAGAGAATG
AGCTTGAGTGTCTCTGA
Y
GTGGGTAATTCACCGCAGCAATCCCAGAAGCAGTGAATTGTGGGCTTCCTACCCTGTGAGCTACTTAAGCATCTTGAATGCTC
AAGCTAAGGCCAGCGAG / Celera SNP ID: hCV153086 / Public SNP ID: rs12645085 /
SNP Chromosome Position: 187019906 / SNP in Genomic Sequence: SEQ ID NO: 438 /
SNP Position Genomic: 30473 / SNP Source: dbSNP; Celera; HapMap /
Population(Allele,Count): Caucasian (C,148|T,78) / SNP Type: INTERGENIC;UNKNOWN /
Context (SEQ ID NO: 2422): /
GCCTCCGTGGTCTGGTCCTCCGCCTGCATCTGCTATAGTACTCGGGGAACGGGGTCATGGTCCATCAGGAATCGCTGTGATGT
AACACTGCCTTTGATCA
S
TTTAAAATTGTTCTTATTGTTATTTTATTTTATTTTTTTGAGACAGTTTCCCTCCTGTCCCCCAGGCTGGAGTGCAATGGCATG
ATCTCGGCTGACTGCAA / Celera SNP ID: hCV153087 / Public SNP ID: rs6552953 /

SNP Chromosome Position: 187020145 / SNP in Genomic Sequence: SEQ ID NO: 438 /
SNP Position Genomic: 30712 / SNP Source: dbSNP; Celera; HapMap /
Population(Allele,Count): Caucasian (C,79|G,147) / SNP Type:
INTERGENIC;UNKNOWN /
Context (SEQ ID NO: 2423): /
TTCCTGGTGTCTCTGCGTTGTGACGGAGTTGGCACTGCTTCCCTGGTTTGCCCCCAACCCTCTCCCTCTTGTTACCATTGCTG
CTCTCAGAGGTGTCTGT
W
CTTAATTCCCTTTTTTCTCTCATTTTGATTCTTCGCTGTGTGCTGGTGCTTCACACGCTGACGACTGCGTGCTCTCGCCTCTCC
GAACATCCAAGGGCCTT / Celera SNP ID: hCV194962 / Public SNP ID: rs6552954 /
SNP Chromosome Position: 187024308 / SNP in Genomic Sequence: SEQ ID NO: 438 /
SNP Position Genomic: 34875 / Related Interrogated SNP: hCV27474895 /
Related Interrogated SNP: hCV3230038 / SNP Source: dbSNP; Celera; HapMap;
ABI_Val / Population(Allele,Count): Caucasian (T,59|A,61) / SNP Type:
INTERGENIC;UNKNOWN /
Context (SEQ ID NO: 2424): /
CACCCTGCTCCGTCTATTTCTCTTTCTGATGGTTAGTTCAGCTGTGGACTTTTGACCTGGTTATAGCCAATGGGACTCAATTG
GAGGGGCTTCTGGAAAG
Y
AGTTTGTTCTTTAATGAGAGAGGGTGACCTGAGCCGCCTTCTTACCTGGGATGTAGCTATTTATTTATTTATTTTTGAGACAG
AGTCTCGCTCTGTCACC / Celera SNP ID: hCV441385 / Public SNP ID: rs1983369 /
SNP Chromosome Position: 187019433 / SNP in Genomic Sequence: SEQ ID NO: 438 /
SNP Position Genomic: 30000 / Related Interrogated SNP: hCV3230113 / SNP
Source: dbSNP; HapMap / Population(Allele,Count): Caucasian (C,46|T,180) /
SNP Type: INTERGENIC;UNKNOWN /
Context (SEQ ID NO: 2425): /
TATCCTTTGCCCACTTGTTGATGGGCTTGTCATTTTTATGGAACACAGTGTGGTCATGTGTCCACAGCCATACAAGTCAATGA
CTGGACTATTGGAAATC
Y
TTGCTGTTGGCATCTCACAAACCTTTTCCTTTCTCTCAGTACTTTTTCTCTAAGTAATGATGCAGTTCAGGCCCTCAGCTTGT
TGTTTTGTGATATGTGC / Celera SNP ID: hCV1589303 / Public SNP ID: rs11730434 /
SNP Chromosome Position: 187046337 / SNP in Genomic Sequence: SEQ ID NO: 438 /
SNP Position Genomic: 56904 / Related Interrogated SNP: hCV3230113 / SNP
Source: dbSNP; Celera; HapMap / Population(Allele,Count): Caucasian
(C,179|T,47) / SNP Type: INTRON /
Context (SEQ ID NO: 2426): /
AACTCTTCTGCCCTCAGACATTGAAGGGTCTGGTGTTTGGGTCTTTAGACTCCAGGACTCCACCCTCGAATTCTCAAGTCTTT
GACCTTGGACTGGGGGA
R
GTCACGCCATCGTCACCCCATTGACCCCCCTGGTTCTCAGATCTTCACACTCGGGCTGGATCACACCACTGGCTTTCCTGGTT
CTTCAGCTCGCAGATGG / Celera SNP ID: hCV1589308 / Public SNP ID: rs9998530 /
SNP Chromosome Position: 187047812 / SNP in Genomic Sequence: SEQ ID NO: 438 /
SNP Position Genomic: 58379 / Related Interrogated SNP: hCV3230113 / SNP
Source: dbSNP; Celera; HapMap / Population(Allele,Count): Caucasian
(A,179|G,47) / SNP Type: INTRON /
Context (SEQ ID NO: 2427): /
TTCACGCAGGGTCCCCTAAACTGTATTAACCGTCACCACCAGTTTTCCCCAGTGCCCTTCCTCTCCTCCAGGGTCCACTTGAC
ATTACATGGAGTTGTCA
Y
GTCTCCTCAGTGTCCTCTGATCCGAGACAGTTTCTCAGACTTTCTTGTTTTGATGATCACGACAGTTAGGGGAGTACTGGTC
ATGTGTTTTGTAGAAGG / Celera SNP ID: hCV1589332 / Public SNP ID: rs7660462 /
SNP Chromosome Position: 187059719 / SNP in Genomic Sequence: SEQ ID NO: 438 /
SNP Position Genomic: 70286 / SNP Source: dbSNP; Celera; HapMap; ABI_Val /
Population(Allele,Count): Caucasian (C,148|T,74) / SNP Type: INTRON /
Context (SEQ ID NO: 2428): /
CTCCCCATTTATTTATTTATTTGTTTATTTCTATCAATATGGGCTCATAGAAATGTATTTTGTACTTTAGTTATAATCCGATA
GCACATGATGTATTTTG
W
TACTCAGGTTGTTCTAGCTTTAGCCATTTGGGGCTCTTTCAGGATGGCTCCTGTGACATGCTCTCATTCTTCTGTTGTTTTGG
TTTTTGTTTTTTTGAGCA / Celera SNP ID: hCV1589334 / Public SNP ID: rs7686244 /
SNP Chromosome Position: 187060255 / SNP in Genomic Sequence: SEQ ID NO: 438 /
SNP Position Genomic: 70822 / SNP Source: dbSNP; Celera /
Population(Allele,Count): Caucasian (T,149|A,77) / SNP Type: INTRON /

444

Context                    (SEQ ID NO: 2429): /
AATTAAATTTATTTCAGAAATGCAAGTTTGATGTACCATGTGAAAATCATTCAGTGCAACAAATGATGTAAGGAAACAAGCAT
GGGATCTTCTCGAAAGA
Y
GAAGAAAATGTCTTTATAAAAATTCTTAGTCAACCAGGAAGATAAGAATATCACTGTTATCTCTTCATGAGTATCTAGGGGAA
AAAAACAACTGCAAACT / Celera SNP ID:   hCV2103348 / Public SNP ID:   rs11931515 /
SNP Chromosome Position:   187100931 /  SNP in Genomic Sequence:   SEQ ID NO: 438 /
 SNP Position Genomic:   111498 /  Related Interrogated SNP:   hCV11786258 /
Related Interrogated SNP:   hCV27477533 /  Related Interrogated SNP:   hCV8241630 /
 SNP Source:   dbSNP; Celera; HapMap /  Population(Allele,Count):   Caucasian
(T,43|C,57) /  SNP Type:   INTERGENIC;UNKNOWN /
Context                    (SEQ ID NO: 2430): /
CTTTAAAGAAATATAGCATCACCTAGAATTTCTATTATTTTTATATACAATGTACAGCATTCAATCTAAAATTAAGAGACAGC
CCAGGATATAGGGCCAA
R
TGACTGAAAGCCAAGAGAGAAAACAAAATTATAATGGACCCACACGTGATTAAAAATAGTAGCACTATCAGGTACAAGCTAAGA
AACAATTTGATTTGCAC / Celera SNP ID:   hCV3230048 / Public SNP ID:   rs2036912 /
SNP Chromosome Position:   187099043 /  SNP in Genomic Sequence:   SEQ ID NO: 438 /
 SNP Position Genomic:   109610 /  SNP Source:   dbSNP; Celera; HapMap; ABI_Val;
HGBASE /  Population(Allele,Count):   Caucasian (G,88|A,138) /  SNP Type:
INTERGENIC;UNKNOWN /
Context                    (SEQ ID NO: 2431): /
ATAAGAAACACAAGATAGAAAGCAAAACATACTCAGGTCCATCACAAACTGCTAGAAACCCCAAATCAAAAAGAACCAAACAG
CAGTCAGAGGGAGGGGG
R
AAAGACTCATTATCTTCAAAGAAGCAACAATAAGACTAATATTTGACTTTTCAACACAAGTGATGGAAGCTGAAAGACAATAT
AAAAAGCATCTTTAAAG / Celera SNP ID:   hCV3230051 / Public SNP ID:   rs4862658 /
SNP Chromosome Position:   187099609 /  SNP in Genomic Sequence:   SEQ ID NO: 438 /
 SNP Position Genomic:   110176 /  Related Interrogated SNP:   hCV27477533 /
Related Interrogated SNP:   hCV8241630 /  SNP Source:   dbSNP; Celera; HapMap /
Population(Allele,Count):   Caucasian (G,165|A,61) /  SNP Type:
INTERGENIC;UNKNOWN /
Context                    (SEQ ID NO: 2432): /
AAACATATACCTCAAAAATGAGGGCAAAAGAGCAGTATTTTCAGATAAACCAAAATTGGGAATTTATCACCGGCAAATCTGTA
CTAAAGGAAATACAAAC
R
AGAGCTCTTCACAGAGTAGGAAAATTATCCCAGAAGGAAGTACAATGATAAAAGAAGAAGTAATAAAGATCAGAATGTATACA
TACGTGGGAAAATAGAA / Celera SNP ID:   hCV3230052 / Public SNP ID:   rs4861705 /
SNP Chromosome Position:   187099852 /  SNP in Genomic Sequence:   SEQ ID NO: 438 /
 SNP Position Genomic:   110419 /  SNP Source:   dbSNP; Celera; HapMap; HGBASE /
Population(Allele,Count):   Caucasian (G,48|A,62) /  SNP Type:   TRANSCRIPTION
FACTOR BINDING SITE;INTERGENIC;UNKNOWN /
Context                    (SEQ ID NO: 2433): /
AAGAAGAAATAGAAAATTTGAAAAGGGTTATGTCTATTATAAGACTTAAATGTATTATTAAAAGCCTTCTCACAAAGACAACT
CCAAGTCCTGCTGACTT
Y
ATTGGTTAATCCTTCCAAATATTTAAGGAAAAACTAATACCACTAGTAGCAATATCAAAAAGCTCTTCAGGCCTGGGCATAGT
GGCTCATGCCTATAATC / Celera SNP ID:   hCV3230055 / Public SNP ID:   rs6816903 /
SNP Chromosome Position:   187100384 /  SNP in Genomic Sequence:   SEQ ID NO: 438 /
 SNP Position Genomic:   110951 /  SNP Source:   dbSNP; Celera; HapMap /
Population(Allele,Count):   Caucasian (C,86|T,140) /  SNP Type:
INTERGENIC;UNKNOWN /
Context                    (SEQ ID NO: 2434): /
CAGTCAATATAAAAATTACGTGCATTTCTATATAACAACAGATAATACAATTTATAAAAATACAATATAATAGATTCAAAAAT
CAAATACCTAGGAATAA
R
TTTAATAAAAATGAGTATATTAGAGTTCTTCAGAGCAACAAAATTATTAGGAGATATTAGATTATGTAATATAATGTATATCT
GGAGGGAGAGAGTTTTT / Celera SNP ID:   hCV7749547 / Public SNP ID:   rs7666666 /
SNP Chromosome Position:   187101406 /  SNP in Genomic Sequence:   SEQ ID NO: 438 /
 SNP Position Genomic:   111973 /  SNP Source:   dbSNP; Celera; HapMap; ABI_Val /
Population(Allele,Count):   Caucasian (A,50|G,68) /  SNP Type:
INTERGENIC;UNKNOWN /
Context                    (SEQ ID NO: 2435): /

GGCTTGGGAGGGCACTGTCGGAGACCAAAGCAGTGTGAGCAAAGCCGTGGAGGCCCAGAGCCACATGGCGTGAGGCTTTCCAT
TTGTTAGGAGCACAGCT
K
TTGTGAAAAGCGTTAAAACTAGGGAGAGCCTGGATACTGGGCTCTCTTTAGTAAACACTGGAAAAGCCAGGATTTAGGTAGAG
AAGTGGCAGGAGCAGGC / Celera SNP ID: hCV8241661 / Public SNP ID: rs1715051 /
SNP Chromosome Position: 187038801 / SNP in Genomic Sequence: SEQ ID NO: 438 /
SNP Position Genomic: 49368 / Related Interrogated SNP: hCV3230113 / SNP
Source: dbSNP; Celera; HapMap; ABI_Val; HGBASE / Population(Allele,Count):
Caucasian (G,47|T,179) / SNP Type: INTRON /
Context (SEQ ID NO: 2436): /
GCCCATGGAACAGAGGCTGCCCCTTTTGCTCCACAGGGCGTACTGACTGCACATCTGAGCCAGAGGCAGAGGATACTGATCTC
TCTGTCTTCACCTCACT
K
TCCTGCATCCCTGGATACGTGATCCAACACCTTTGATCTTCACCTTGGCTTCCCTGTCCATTTCATGTATATATTATGCCTCA
GTGTTTTTCAGTCTCAC / Celera SNP ID: hCV8241668 / Public SNP ID: rs1401570 /
SNP Chromosome Position: 187029821 / SNP in Genomic Sequence: SEQ ID NO: 438 /
SNP Position Genomic: 40388 / Related Interrogated SNP: hCV27474895 / SNP
Source: dbSNP; HapMap / Population(Allele,Count): Caucasian (T,66|G,160) /
SNP Type: INTRON /
Context (SEQ ID NO: 2437): /
TATTTTCACAAACCATTCTTTATTCTTTGTGTGCATTGGGCAAGAGTCATCAGACTTTTCAATGGTGGAACTCTTCCACTTTT
GTTTGAGGACTTCTGGG
Y
AGACAGGGTTGGCTTATTTAGCTTCCTTTTCTCTTAGGTCAGTTCCCCAGGAAACAGGTTTTGGGATGGAGGTTTGCATGAAG
GAAGTTTCAGGGGTGCT / Celera SNP ID: hCV11786002 / Public SNP ID: rs4862633 /
SNP Chromosome Position: 187009972 / SNP in Genomic Sequence: SEQ ID NO: 438 /
SNP Position Genomic: 20539 / Related Interrogated SNP: hCV3230113 / SNP
Source: dbSNP; Celera; HapMap; HGBASE / Population(Allele,Count): Caucasian
(T,50|C,176) / SNP Type: INTERGENIC;UNKNOWN /
Context (SEQ ID NO: 2438): /
TCTTAGGTCAGTTCCCCAGGAAACAGGTTTTGGGATGGAGGTTTGCATGAAGGAAGTTTCAGGGGTGCTCTGATTTATAAGTG
AGTGAAATAAGCTGGAT
Y
GGGAAGAGAGAGAATAATTAACTCTGATGCAATTCACAATTGCAGCTGAGTTCTTAGCCAGCCCCATGGGGAGCTCTGGAGCT
GGAATGACCCTAGAGAA / Celera SNP ID: hCV11786003 / Public SNP ID: rs4608848 /
SNP Chromosome Position: 187010104 / SNP in Genomic Sequence: SEQ ID NO: 438 /
SNP Position Genomic: 20671 / Related Interrogated SNP: hCV3230113 / SNP
Source: dbSNP; Celera; HapMap; HGBASE / Population(Allele,Count): Caucasian
(C,85|T,141) / SNP Type: INTERGENIC;UNKNOWN /
Context (SEQ ID NO: 2439): /
CAGATTTTGGATGGGCCAGCGCATGAGGTGGGGAAGCTCAAATACCAGACCAAGGAAGTGAGTTAGTGGAAATGAGGAGCCAT
AAAGATGCTTTTATGGT
R
GAAGGAGCATGGGTTGAAGAGCTGGGTTTTAGGAAGAGTGAGATAGTGGGATCGATTAGGAACAGACTGAAGTTCTTGAGATG
CCCCTATTTACATAGGA / Celera SNP ID: hCV11786022 / Public SNP ID: rs2090628 /
SNP Chromosome Position: 187015824 / SNP in Genomic Sequence: SEQ ID NO: 438 /
SNP Position Genomic: 26391 / Related Interrogated SNP: hCV3230113 / SNP
Source: dbSNP; Celera; HGBASE / Population(Allele,Count): Caucasian
(G,68|A,156) / SNP Type: INTERGENIC;UNKNOWN /
Context (SEQ ID NO: 2440): /
ATAAAACCCTTCATTCCTTCCTTGCTTTGTTTGTGTGTTTTTTCCAATTCTTTGTTCAAGACGCGAAGAACCTGGACACCCTC
CACCATTAACATATTGA
M
CTAGTGTGACCTTCAAGACCCTTCGCAGCTGGGTCGAGTTTATCTCCCTGCTTGATTTTGTTTGGTTTTGTTTTACCACATCT
CCTATGCTTGGTGCCAT / Celera SNP ID: hCV11786028 / Public SNP ID: rs6848963 /
SNP Chromosome Position: 187016610 / SNP in Genomic Sequence: SEQ ID NO: 438 /
SNP Position Genomic: 27177 / Related Interrogated SNP: hCV3230113 / SNP
Source: dbSNP; Celera / Population(Allele,Count): Caucasian (A,46|C,180) /
SNP Type: INTERGENIC;UNKNOWN /
Context (SEQ ID NO: 2441): /
AGATGGTATACTACCTTCTCCTCTCAGAAATACTCAATGCGTACCAGCATATTGAAAAACTAAGATGCCGGCTATAAAGAAAG
CTATTTAACCTTCATTA
Y

TGAGTCAGTTGCCAAACCAACTTGATTATAGAACGCTTCTAGTGGTAACTGTTATCAGCATCGTGACAAAGGCATGTTCTGCA
TAGCACAATTTTGGGAA / Celera SNP ID: hCV12066105 / Public SNP ID: rs1519309 /
SNP Chromosome Position: 187015089 / SNP in Genomic Sequence: SEQ ID NO: 438 /
SNP Position Genomic: 25656 / Related Interrogated SNP: hCV3230113 / SNP
Source: dbSNP; HapMap / Population(Allele,Count): Caucasian (C,69|T,157) /
SNP Type: INTERGENIC;UNKNOWN /
Context (SEQ ID NO: 2442): /
GATGCCGGCTATAAAGAAAGCTATTTAACCTTCATTACTGAGTCAGTTGCCAAACCAACTTGATTATAGAACGCTTCTAGTGG
TAACTGTTATCAGCATC
S
TGACAAAGGCATGTTCTGCATAGCACAATTTTGGGAACACTCTAGGTAAACTTAATTATTCTAGTTCTAGCACCTCCTAAGAA
ATTCTCCTTTTCATACC / Celera SNP ID: hCV12066106 / Public SNP ID: rs1914926 /
SNP Chromosome Position: 187015152 / SNP in Genomic Sequence: SEQ ID NO: 438 /
SNP Position Genomic: 25719 / Related Interrogated SNP: hCV3230113 / Related
Interrogated SNP: hCV11786258 / Related Interrogated SNP: hCV3230096 / SNP
Source: dbSNP; HapMap; HGBASE / Population(Allele,Count): Caucasian
(G,28|C,92) / SNP Type: INTERGENIC;UNKNOWN /
Context (SEQ ID NO: 2443): /
TGTCTGTCACCTCAGTTTGTCGTTCTGTTAAACTGCAGTACTGAGATATTTGGGGCTGCTTTCCACGCTGCCCGCAGAGGGGT
CCATGCAGGCTTTCCAT
R
GTAACTTAAAGGGAAACCTTCACAGTGTCACAATGCCTGGAGACCTGGACGTCCTGCCTATGAAGGACGAGGATGTGCTCAAG
TTCCTTGCGGTAGGAGC / Celera SNP ID: hCV12086148 / Public SNP ID: rs1877321 /
SNP Chromosome Position: 187079017 / SNP in Genomic Sequence: SEQ ID NO: 438 /
SNP Position Genomic: 89584 / Related Interrogated SNP: hCV12066124 /
Related Interrogated SNP: hCV25474413 / SNP Source: dbSNP; HapMap; HGBASE /
Population(Allele,Count): Caucasian (G,164|A,58) / SNP Type: INTRON /
Context (SEQ ID NO: 2444): /
TATCTATGCAAATTCTTGAGTCTCATAACTTCCGTGGCAGAAGTTTTCTCTATTTAACAGTAAAAGAATCCGAGGCTCAGAAT
GAGATGGCATTTCTCAG
R
AAGGTAACAGCTAGAGCACAGGTGTGTCTGACTGCAAAGCCACCATCTTTTTACCAAGTGATGCACCCTCCTACATAGTGGGT
GACAGTAAATGTATAAG / Celera SNP ID: hCV15882886 / Public SNP ID: rs2276916 /
SNP Chromosome Position: 187072887 / SNP in Genomic Sequence: SEQ ID NO: 438 /
SNP Position Genomic: 83454 / Related Interrogated SNP: hCV25990131 / SNP
Source: dbSNP; HGBASE / Population(Allele,Count): Caucasian (A,95|G,25) / SNP
Type: INTRON /
Context (SEQ ID NO: 2445): /
CCGCACGTCCTCGTTCCACACGCTCACGCCGATGGAGCCAGTGGCCCCCCGTCCGGACACGCCGAGGCTCACGGCATCTCCCT
GGCTTCTCGTCTGAACC
Y
GCCCCAGGTCGGTGCTTTCACACCCTTCTCCCTCTTGCCTTCACTCTGTGTGTCTGTCACCTCAGTTTGTCGTTCTGTTAAAC
TGCAGTACTGAGATATT / Celera SNP ID: hCV15882904 / Public SNP ID: rs2276922 /
SNP Chromosome Position: 187078866 / SNP in Genomic Sequence: SEQ ID NO: 438 /
SNP Position Genomic: 89433 / SNP Source: dbSNP; HapMap; ABI_Val; HGBASE /
Population(Allele,Count): Caucasian (C,175|T,51) / SNP Type: MISSENSE
MUTATION;ESE /
Context (SEQ ID NO: 2446): /
CCCAGCCTACCTGGGCCCCCTGTCACACCTTCATCAAATAGAAACCCATTTATGTTTCCTACTGAGATACATTCAGATGCCAC
TAACTGTACAAATCCCT
S
TGTGTTCTGACCCCTCCCTCCCTGTGGGCTGCTGATTTTTCAGCTTTCTTTTTATTATTGCCACCCTGAGGAGCCCTTTTCGA
CCATTCTTCCCAATTGC / Celera SNP ID: hCV16172925 / Public SNP ID: rs2241818 /
SNP Chromosome Position: 187085945 / SNP in Genomic Sequence: SEQ ID NO: 438 /
SNP Position Genomic: 96512 / Related Interrogated SNP: hCV3230038 / Related
Interrogated SNP: hCV27474895 / Related Interrogated SNP: hCV12066124 /
Related Interrogated SNP: hCV8241630 / Related Interrogated SNP: hCV27477533 /
Related Interrogated SNP: hCV25474413 / Related Interrogated SNP: hCV3230113
/ SNP Source: dbSNP; HapMap; ABI_Val; HGBASE / Population(Allele,Count):
Caucasian (C,98|G,22) / SNP Type: INTRON /
Context (SEQ ID NO: 2447): /
TTTTTCTATAATTTTCTATAAGCTTGTGGACCTTATTGATTACCTAGGACTTTCTATGCTTTTTATACTCTTCATATTAAAAG
TCTAAGTATTTTTCATC

Y
TTTTTTGGATACAACATTTATAGCAATTTCAAAATGTCCTGATAATCTGTGCCATGTATAGGAGCTTTAGCGTCTCTGTATGA
TTTGTAGGGAGCTGTGG / Celera SNP ID: hCV27310170 / Public SNP ID: rs4862644 /
SNP Chromosome Position: 187057812 / SNP in Genomic Sequence: SEQ ID NO: 438 /
SNP Position Genomic: 68379 / Related Interrogated SNP: hCV3230113 / SNP
Source: dbSNP; Celera; HGBASE / Population(Allele,Count): Caucasian
(C,65|T,49) / SNP Type: INTRON /
Context (SEQ ID NO: 2448): /
GCCTGTGTCCCTGCTTTTAATTCTTTCAGTGTATACCAAGAAGTGGGATTGCTGGATCCTATGGTAATTTTGTGTTTCACTTT
TGAGGAATCACAAACGA

W
TTTTTTATACTAGCTGCACCATTTTACGTTTCCACCGGTGATGCACGAGGATTCCCATTCCTCTACATCTTCACCAAAACTTA
CTTTCTTTATCTCTTTT / Celera SNP ID: hCV27310180 / Public SNP ID: rs11722584 /
SNP Chromosome Position: 187055207 / SNP in Genomic Sequence: SEQ ID NO: 438 /
SNP Position Genomic: 65774 / Related Interrogated SNP: hCV3230113 / SNP
Source: dbSNP; Celera; HapMap / Population(Allele,Count): Caucasian
(T,178|A,48) / SNP Type: INTRON /
Context (SEQ ID NO: 2449): /
ATAGTGTTACACAGACACACATTTAACAGGACATGATTTTCTAGACACCATGTACTACGTAGTACTCGTCAAGCAAATGGATA
AATACTATTTATTATGT

W
TTGAAAAAAGAGTTGCCCCTTTTTGAGAGGTAGAAATAATGAAGATGCCATCTTCTGAAAAGCACAGAAACAAAATAAAGGCT
TTGTCATGATTAATGAT / Celera SNP ID: hCV27310216 / Public SNP ID: rs10018625 /
SNP Chromosome Position: 187040464 / SNP in Genomic Sequence: SEQ ID NO: 438 /
SNP Position Genomic: 51031 / Related Interrogated SNP: hCV3230113 / SNP
Source: dbSNP; Celera; HapMap / Population(Allele,Count): Caucasian
(A,90|T,26) / SNP Type: INTRON /
Context (SEQ ID NO: 2450): /
CACTGTCGGAGACCAAAGCAGTGTGAGCAAAGCCGTGGAGGCCCAGAGCCACATGGCGTGAGGCTTTCCATTTGTTAGGAGCA
CAGCTGTTGTGAAAAGC

R
TTAAAACTAGGGAGAGCCTGGATACTGGGCTCTCTTTAGTAAACACTGGAAAAGCCAGGATTTAGGTAGAGAAGTGGCAGGAG
CAGGCTGCACTTTAGGA / Celera SNP ID: hCV27310218 / Public SNP ID: rs9992614 /
SNP Chromosome Position: 187038813 / SNP in Genomic Sequence: SEQ ID NO: 438 /
SNP Position Genomic: 49380 / Related Interrogated SNP: hCV3230113 / SNP
Source: dbSNP; Celera; HapMap / Population(Allele,Count): Caucasian
(G,173|A,49) / SNP Type: INTRON /
Context (SEQ ID NO: 2451): /
TCCTACTTCATGCATACACAACAATTAACTTAGGAGAGGTCACACACCTTCACATAGAAGCTAACACCATAAAGCTTTTTAGA
GGAGAGTATCTAAGTAA

W
GGGAGTAGGAAAATTTCTTAGAGGAGACACAGAAATGATAAAAATAAAAGAGAAGATGATAAACTAGATTTCATCATAATTAA
GAAGACATCAGTAAGAA / Celera SNP ID: hCV27310253 / Public SNP ID: rs13108688 /
SNP Chromosome Position: 186994832 / SNP in Genomic Sequence: SEQ ID NO: 438 /
SNP Position Genomic: 5399 / Related Interrogated SNP: hCV3230113 / SNP
Source: dbSNP; Celera; HapMap / Population(Allele,Count): Caucasian
(T,96|A,24) / SNP Type: INTRON /
Context (SEQ ID NO: 2452): /
GGAGACGCAGAGGCAAACCATATCGGCTATGTATTCAACATATATTTATTTCTTCAGAGCCAACAAAGTGCCAGGTCTTGTTC
TAGGCACAGACAACACC

R
TATGGAATAAGAACTATGTCCTCTACTATTATAGCTTCACATTCTAGTGAGAAGCAATAATTAATAATAAATAATACACAATA
TTGGATAATTTCCATCA / Celera SNP ID: hCV27310255 / Public SNP ID: rs7657186 /
SNP Chromosome Position: 186994039 / SNP in Genomic Sequence: SEQ ID NO: 438 /
SNP Position Genomic: 4606 / Related Interrogated SNP: hCV3230113 / SNP
Source: dbSNP; Celera; HapMap / Population(Allele,Count): Caucasian
(G,191|A,35) / SNP Type: INTRON /
Context (SEQ ID NO: 2453): /
GTGAATGCTGAGCAGAAGTTAGCCCGTGGCCAGGGGAGGTCAGAGGCTGGAGTAAGAGGTGGGCGCTGCAGGAACCGAGTAAG
GAAGGACTCGTGCATTA

S
AGGAAGGGGGAGAAATGCTCTGCCCATGGGATGTGGTGAAGGGTGAGCGGAGATGGAGTAAAGAACTGCATCTGGCCGGGTGC
AGCGGCTCAGGCCTATA / Celera SNP ID: hCV27482764 / Public SNP ID: rs3775292 /

SNP Chromosome Position: 187003025 / SNP in Genomic Sequence: SEQ ID NO: 438 / SNP Position Genomic: 13592 / SNP Source: dbSNP; HapMap / Population(Allele,Count): Caucasian (C,41|G,185) / SNP Type: UTR5;INTRON / Context (SEQ ID NO: 2454): /
CAGTGATCACAAACTATACAACAGATGACATCACACACCACGCAAGATGGGAGTGCTCTTTACTGTTGCTGTGGGAGTTTCAA
GACCCTCTGAAATGAGG
R
GCGTCTCCTTTCTCTGCTGGAACAGTGCTTCTCAATGTGTGTGCCATTAGCTACCTGCTTGCAGTTACCAGGTAGCTACTTGT
TCCCTGTCCATGAAGCG / Celera SNP ID: hCV32209919 / Public SNP ID: rs11730526 /
SNP Chromosome Position: 187039417 / SNP in Genomic Sequence: SEQ ID NO: 438 / SNP Position Genomic: 49984 / Related Interrogated SNP: hCV3230113 / SNP Source: dbSNP; HapMap / Population(Allele,Count): Caucasian (A,93|G,27) / SNP Type: INTRON /
Context (SEQ ID NO: 2455): /
CGGGGTCATGGTCCATCAGGAATCGCTGTGATGTAACACTGCCTTTGATCACTTTAAAATTGTTCTTATTGTTATTTTATTTT
ATTTTTTGAGACAGTTT
M
CCTCCTGTCCCCCAGGCTGGAGTGCAATGGCATGATCTCGGCTGACTGCAACCTCCGCCTCCCAGGTTCAGGCTTCTCCTGCC
TCAGCCTCCCAAGTAGC / Celera SNP ID: hCV32209928 / Public SNP ID: rs13148663 /
SNP Chromosome Position: 187020194 / SNP in Genomic Sequence: SEQ ID NO: 438 / SNP Position Genomic: 30761 / Related Interrogated SNP: hCV3230113 / SNP Source: dbSNP; HapMap / Population(Allele,Count): Caucasian (C,92|A,26) / SNP Type: INTERGENIC;UNKNOWN /
Context (SEQ ID NO: 2456): /
GCCTTGACCACCTGGCTCCGGGAGTGCTTGTCAGGCTGCTCCCCTGTAAAGTCACCCCCTGCTTCCATACTGCACTCTTGGAG
TCAAGCTTCCATACTGC
M
CTCTTGGAGAAGAAGGTATGATGCCCGCCTCCTGGAAGGGGAGACACACACCACTGGGGTAGAGTTCTTCTGTGGGAAATGTG
CCTCTTCTCCCCATTTA / Celera SNP ID: hCV32209815 / Public SNP ID: rs7660915 /
SNP Chromosome Position: 187060065 / SNP in Genomic Sequence: SEQ ID NO: 438 / SNP Position Genomic: 70632 / Related Interrogated SNP: hCV25990131 / SNP Source: dbSNP / Population(Allele,Count): Caucasian (A,85|C,35) / SNP Type: INTRON //

Gene Number: 102 / Gene Symbol: KLHL20 - 27252 / Gene Name: kelch-like 20 (Drosophila) / Chromosome: 1 / OMIM NUMBER: / OMIM Information: // Genomic Sequence (SEQ ID NO: 439): // SNP Information /
Context (SEQ ID NO: 2457): /
TTGCTACAGTAATTCTACCTATCACCCATTTGCACAAAGAGAAATATATAAGGATGAACATTGTTTTTAATAGGAAAATATCA
GAAACAGCCTAAGGGGT
R
TATCAGTAGGAATATAATTAAATAATGTATAACACATATAATAGTAAAATAATTCAAAAGAAAAATCTAGAGCTATATTAGCA
TAAATAGATCTCAAAAA / Celera SNP ID: hCV27483572 / Public SNP ID: rs3791022 /
SNP Chromosome Position: 173734769 / SNP in Genomic Sequence: SEQ ID NO: 439 / SNP Position Genomic: 60689 / Related Interrogated SNP: hCV16180170 /
Related Interrogated SNP: hCV8911768 / SNP Source: dbSNP; HapMap; ABI_Val; HGBASE / Population(Allele,Count): Caucasian (A,110|G,10) / SNP Type: INTRON //

Gene Number: 103 / Gene Symbol: NME7 - 29922 / Gene Name: non-metastatic cells 7, protein expressed in (nucleoside-diphosphate k / inase) // Chromosome: 1 / OMIM NUMBER: / OMIM Information: // Genomic Sequence (SEQ ID NO: 440): // / SNP Information /
Context (SEQ ID NO: 2458): /
TTTTATTTTAAAGTAGAAATCTGTCCGCTTTTCACTTACGGAGGCCTTACAGTGTGTGTTCTGTGTTACTTGAGTTTATACGC
ACTGCAGAATTTGTTTA
Y
CCTGCTCTTTTGGAAACGTATTCCGGAAGCGAAACCCTGAGTAATCGGAAGTGGTTAGGAGTGAGAGAGCTGCTGGATATGCG
GAGGGACTGGGCGGGTC / Celera SNP ID: hCV15878582 / Public SNP ID: rs2275299 /
SNP Chromosome Position: 169337376 / SNP in Genomic Sequence: SEQ ID NO: 440 / SNP Position Genomic: 245607 / Related Interrogated SNP: hCV11975250 / SNP Source: Applera / Population(Allele,Count): Caucasian (C,11|T,29) African American (C,22|T,14) total (C,33|T,43) // / SNP Type: UTR5;TFBS SYNONYMOUS /

SNP Source: dbSNP; Celera; HapMap; HGBASE / Population(Allele,Count): Caucasian (C,94|T,132) / SNP Type: UTR5;TFBS SYNONYMOUS /
Context (SEQ ID NO: 2459): /
TATCAAAACCTCAAATTATACTACATATTTGTTTATTCTGCATCTCCCCAGAAATTTTAGCTCCATAAGGGTGGGACTTTGTC
ACTGATTGCTCAGAACA
S
TGCCTGGCACTCAAGAGAAACTTAAAAATAATTGTTGAATGAATGAAGCTAGAGCTCTCTCTCAGAATAAAAGAGTCCACTGT
TTTTGACCCATAATTCT / Celera SNP ID: hCV275164 / Public SNP ID: rs12140572 /
SNP Chromosome Position: 169325127 / SNP in Genomic Sequence: SEQ ID NO: 440 /
SNP Position Genomic: 233358 / Related Interrogated SNP: hCV8919444 / SNP
Source: dbSNP; Celera; HapMap / Population(Allele,Count): Caucasian
(G,88|C,32) / SNP Type: INTRON /
Context (SEQ ID NO: 2460): /
ATAGTCCTATAGTCTAGCAATATAACTTTAAGATTTACATAACAGAATGAAAGTCAGAATTGTATTTTATGGCATTAAATAAA
TTAACTTTATTTCTGTC
Y
ATATCAACATCCAAAGTTTATTAGGAATTTATTTAAAGAATAATAAAAGCAATAGGACCTAACATTTAATGAGTGCTTACTGT
GTCAAATACCATTCTAA / Celera SNP ID: hCV279320 / Public SNP ID: rs10800441 /
SNP Chromosome Position: 169344023 / SNP in Genomic Sequence: SEQ ID NO: 440 /
SNP Position Genomic: 252254 / Related Interrogated SNP: hCV11975250 / SNP
Source: dbSNP; Celera / Population(Allele,Count): Caucasian (T,94|C,132) /
SNP Type: INTRON /
Context (SEQ ID NO: 2461): /
TCTGCCACTCAGAAAAGCAAAAAGACTCCAAGATCTAGGGAGATTCAGATCAGTGTTGAGACAGTCACATACACACCACCCCA
TACTCTTAATAAAGCGC
R
TTAATGTGAATCTGTGAACATGAAAAATGCCATGATGAACAATGCAAACACATAATGTTTGTTCAAAACAGGTTATTAGACTC
GATGCCTTACCCTAAAT / Celera SNP ID: hCV288901 / Public SNP ID: rs4656671 /
SNP Chromosome Position: 169336617 / SNP in Genomic Sequence: SEQ ID NO: 440 /
SNP Position Genomic: 244848 / Related Interrogated SNP: hCV8919444 / SNP
Source: dbSNP; Celera; HapMap; HGBASE / Population(Allele,Count): Caucasian
(G,146|A,80) / SNP Type: INTRON /
Context (SEQ ID NO: 2462): /
CTACCTCCCTAACCACATTTCATGGTATTTTGCACCATATTTTCCCTACTGCAGCCACCCCTGCACCATCCCACCACCCAGTT
TCTATTATTTTATTACA
Y
TGAACCATAAGTTCCTAAAATATACCAATCTCTTTTCTGCCTCAGGGTATTTGATCTTGTTGTTAGCGTGGCCTGGACTCCTC
CAGATCACTGCACTTTG / Celera SNP ID: hCV475606 / Public SNP ID: rs17349579 /
SNP Chromosome Position: 169332898 / SNP in Genomic Sequence: SEQ ID NO: 440 /
SNP Position Genomic: 241129 / Related Interrogated SNP: hCV8919444 / SNP
Source: dbSNP; Celera; HapMap / Population(Allele,Count): Caucasian
(T,78|C,26) / SNP Type: INTRON /
Context (SEQ ID NO: 2463): /
CCTCCCAAAGTGCTGGGATTACCACAGCACCTGTCCCAATTTTCTTTAACATAGATAAAATCACCACTCGCAGGACTCAACTA
GTTTACATTCCTCTTTG
R
CCTATGAAATGTGATATATGTTTTCCATATCAAGTTTGAATAAAATTATCAATCAAAGACTCCATAAGCATGTGTAACTTAGC
CCAGTTGCTCCCAACCA / Celera SNP ID: hCV1264276 / Public SNP ID: rs17345170 /
SNP Chromosome Position: 169124402 / SNP in Genomic Sequence: SEQ ID NO: 440 /
SNP Position Genomic: 32633 / Related Interrogated SNP: hCV8919444 / SNP
Source: dbSNP; Celera / Population(Allele,Count): Caucasian (A,157|G,69) /
SNP Type: INTRON /
Context (SEQ ID NO: 2464): /
TTCAAATTTTCATTTTATGAGGTTCATTCTAATGGCAATAAGAAAATGGCATGGAGCAATCTTGAGGTAGGAAATGAGCCATC
AAGGAATAGAAGGTAGT
R
AGTCAAGAAGTAGACAAATCAGTCAGTAGCCTATGTTGATACGTTGAAAGCCTGAATATGAGCCTATAAAATGCCTAAACTAA
GGGGCTGCAATAGGAAT / Celera SNP ID: hCV2520857 / Public SNP ID: rs12118611 /
SNP Chromosome Position: 169275630 / SNP in Genomic Sequence: SEQ ID NO: 440 /
SNP Position Genomic: 183861 / Related Interrogated SNP: hCV8919444 / SNP
Source: dbSNP; Celera; HapMap / Population(Allele,Count): Caucasian
(A,151|G,71) / SNP Type: TRANSCRIPTION FACTOR BINDING SITE;INTRON /
Context (SEQ ID NO: 2465): /

CATGTTATATGTCAGCTCCCCTCAGTACTTCCTTTTTAAAACACTCCCATTCATTTTGAAGAATATTTCTAAAAAACAGCATG
TACAAAAAAGTATTCTT
R
TCTTCTATCCTAAGAGTTCTATAATACCTCCTCATACATTTTCTGCCAGACCTTTGCTGCCAAAACAAACAAAACCCCCAAGA
CCAACCACTAGATTTAA / Celera SNP ID: hCV2520872 / Public SNP ID: rs3766090 /
SNP Chromosome Position: 169269009 / SNP in Genomic Sequence: SEQ ID NO: 440 /
SNP Position Genomic: 177240 / Related Interrogated SNP: hCV8919444 / SNP
Source: dbSNP; Celera; HGBASE / Population(Allele,Count): Caucasian
(G,145|A,71) / SNP Type: INTRON /
Context (SEQ ID NO: 2466): /
TAGGAAAAGTCAAATCTCTGTAGATGTGAGTTTCACCTCTTGCAAATACTGCATTTTCAATCCTTCTGTTGAAAAAAATCCAC
ATATAAGTAGACTCGTG
M
AGTTCAAACCTGTGTTGTTTATGGATCAACTGTATTTATTTTTCATTAAATTTAAAATGAAAAGATTATAGAATGTGTTAATG
TGCCACACTGATGAGCA / Celera SNP ID: hCV2520887 / Public SNP ID: rs10442644 /
SNP Chromosome Position: 169257750 / SNP in Genomic Sequence: SEQ ID NO: 440 /
SNP Position Genomic: 165981 / Related Interrogated SNP: hCV8919444 / SNP
Source: dbSNP; Celera; HapMap / Population(Allele,Count): Caucasian
(C,155|A,71) / SNP Type: INTRON /
Context (SEQ ID NO: 2467): /
GTAAAACCAAATATAGTAGGATAAGTAATTGAGTGTTAGGCTGTTACAACTATAAATGTGGGTGGGAAAAGGTAGGCTTTGAT
CTCTTTGAGAATGAGAT
W
TTCAAGCACAAATACTACTAAGCACAAATAACTGAGGTAACTAAGTACCAATTTGGATTTCTGCGAATAAATTGTGGATGATA
TATGGATGGTGTTTCAT / Celera SNP ID: hCV2521003 / Public SNP ID: rs2040446 /
SNP Chromosome Position: 169165266 / SNP in Genomic Sequence: SEQ ID NO: 440 /
SNP Position Genomic: 73497 / Related Interrogated SNP: hCV8919444 / SNP
Source: dbSNP; Celera; HapMap; HGBASE / Population(Allele,Count): Caucasian
(A,157|T,69) / SNP Type: INTRON /
Context (SEQ ID NO: 2468): /
GATTTGTTAGCCCTGGTACTCCTTAGACTAATATAGATAGAAAGAGTTAACCAGTTTTTCTTTTCTTATATTATTTCTGATTG
GCCTGACTCTGAGTACA
S
TATGCTTAACAAATAAAATTCTCTGCATATGAAACAAAGACTTAAAAAGACAAATTACTGTTTTGGCAAATATTCATTCTCAA
TTAAAATTCATGTGAGT / Celera SNP ID: hCV8919279 / Public SNP ID: rs1200079 /
SNP Chromosome Position: 169158904 / SNP in Genomic Sequence: SEQ ID NO: 440 /
SNP Position Genomic: 67135 / Related Interrogated SNP: hCV8919444 / SNP
Source: dbSNP; HapMap; ABI_Val; HGBASE / Population(Allele,Count): Caucasian
(C,29|G,91) / SNP Type: INTRON /
Context (SEQ ID NO: 2469): /
CACCTTTACTCAAGTGCCTTCAAAGAAAAAAAATGGAAATACTAAAAGTATACTTTATTTTCTATACATAAAGTACATCAAGA
AAATAAACATTGCTCTC
Y
TATTAACAAAATGTACTGAATATAGTGTATTCAACATACCTTCACTGACAGCATGGGGTTTAACAATGCAACAGGTACAATTA
GTAAATTTAGCAGTGTT / Celera SNP ID: hCV11341772 / Public SNP ID: rs4589164 /
SNP Chromosome Position: 169256501 / SNP in Genomic Sequence: SEQ ID NO: 440 /
SNP Position Genomic: 164732 / Related Interrogated SNP: hCV8919444 / SNP
Source: dbSNP; Celera; HapMap; HGBASE / Population(Allele,Count): Caucasian
(C,155|T,71) / SNP Type: INTRON /
Context (SEQ ID NO: 2470): /
ATATTGAAAGCCTAAAAATGCACATGACCTCTCGCACCTAGTTATTTCCTTTTTAATTTATTGTAAGGAAATAATTATGGATG
TCTATAAAAATGTGTTC
R
CAAAAATATGTTAGTATTGTGTTAACAGAACAAATTAATAAATGGATTGGTTAAATCAGAGTAAATGAAAGAATAAAACGTAG
AGCTGTCCCTCAGTATT / Celera SNP ID: hCV11341861 / Public SNP ID: rs10800436 /
SNP Chromosome Position: 169328198 / SNP in Genomic Sequence: SEQ ID NO: 440 /
SNP Position Genomic: 236429 / Related Interrogated SNP: hCV11975250 / SNP
Source: dbSNP; Celera / Population(Allele,Count): Caucasian (G,107|A,5) / SNP
Type: INTRON /
Context (SEQ ID NO: 2471): /
GCTTCATCTTAAATGAGGTGTATGAGAAACATGCTAAACTATGGTTCCCTATCTCTGCTTCACAATATAAAGGTGATTCCAGA
ATTTGGAAGCCTCAACT
R

TAACAAACTAGATGAAGACAGAAGCTGATTAGATAAACTGCAGTGGTCAGGAAGCCTCACCAACAGAGCAAACAACCTCCATC
TTCAGCTGCTTATTGAG / Celera SNP ID: hCV11341869 / Public SNP ID: rs2176473 /
SNP Chromosome Position: 169331802 / SNP in Genomic Sequence: SEQ ID NO: 440 /
SNP Position Genomic: 240033 / Related Interrogated SNP: hCV11975250 / SNP
Source: dbSNP; Celera; HapMap; ABI_Val; HGBASE / Population(Allele,Count):
Caucasian (A,88|G,138) / SNP Type: INTRON /
Context (SEQ ID NO: 2472): /
TCTTTCTATCCTCCAGTCCGTTATCCATAAGTGCTTAAAAGATATGGTAGTTAAGTGAATTAACTAGAACCCTCTCAAACATA
GGGGTGAATTAAAATAA
Y

CTATAGGCTTATTTAAAATGCAAATACCGAGCTCCATTCCTTTCATTCAGAATTTCCAGGGGTGAAGCCCAAACAGTGAATTC
CAGAAGCTCTCCAAGCA / Celera SNP ID: hCV11341876 / Public SNP ID: rs1980198 /
SNP Chromosome Position: 169336259 / SNP in Genomic Sequence: SEQ ID NO: 440 /
SNP Position Genomic: 244490 / Related Interrogated SNP: hCV11975250 / SNP
Source: dbSNP; Celera; HapMap; HGBASE / Population(Allele,Count): Caucasian
(C,94|T,132) / SNP Type: INTRON /
Context (SEQ ID NO: 2473): /
TAGGGGTGAATTAAAATAACCTATAGGCTTATTTAAAATGCAAATACCGAGCTCCATTCCTTTCATTCAGAATTTCCAGGGGT
GAAGCCCAAACAGTGAA
K

TCCAGAAGCTCTCCAAGCAATCCCAGGAGAAATTTCTGTTTAAAAAAAAAAGAAGAAGAAAGAAAAGAAAAGAGAAAAGAAAA
GCAAGCACAGACCTAGA / Celera SNP ID: hCV11341878 / Public SNP ID: rs4656670 /
SNP Chromosome Position: 169336340 / SNP in Genomic Sequence: SEQ ID NO: 440 /
SNP Position Genomic: 244571 / Related Interrogated SNP: hCV11975250 / SNP
Source: dbSNP; Celera; HapMap; HGBASE / Population(Allele,Count): Caucasian
(T,94|G,132) / SNP Type: INTRON /
Context (SEQ ID NO: 2474): /
AGTGTAAGTGTAGAAAAGTTTGTGACTGTTAAGAAAGTTAGTCGTCGGGTTCACTAATTGAACTGAGTTGTATCTAGTACTGA
GAACGATCTTTTTTTGT
R

GTGAAACCAGTTGTCATCTAGTGTACAGTTTGTCATATTTATATCATTGCAGTAGCATCACCTTCTTATATGTTCTCTGGTAA
ATCTGAGTCTGTACTGT / Celera SNP ID: hCV11341879 / Public SNP ID: rs7527703 /
SNP Chromosome Position: 169338046 / SNP in Genomic Sequence: SEQ ID NO: 440 /
SNP Position Genomic: 246277 / Related Interrogated SNP: hCV8919444 / SNP
Source: dbSNP; Celera; HapMap / Population(Allele,Count): Caucasian
(A,146|G,80) / SNP Type: INTRON /
Context (SEQ ID NO: 2475): /
TTGCTGTAACAAAGTACCACAAACTGCATGGCTTGAACAACAAAATGTATTCTCTCAGTTCTGGAGGCTAGAAGTCCAAGATG
AAAATGTCAACAAGGCT
R

TGCTCCTTCGGAAGGCATTAGAGACACTCTCCTAGCTTCCAGTGGCTTGCTGGCAGTCTTTGGTGCTCCTTGTCTTGTAGACG
TACCACCCTTATCTCCG / Celera SNP ID: hCV11341882 / Public SNP ID: rs12024897 /
SNP Chromosome Position: 169339268 / SNP in Genomic Sequence: SEQ ID NO: 440 /
SNP Position Genomic: 247499 / Related Interrogated SNP: hCV11975250 / SNP
Source: dbSNP; Celera; HapMap / Population(Allele,Count): Caucasian
(G,94|A,132) / SNP Type: INTRON /
Context (SEQ ID NO: 2476): /
TCCTGTTTCCAAATGAAGTCTCATTCTGAGGTAGTGGGGGGTTAGTACTTCAACAAATAAAGATTTGGGGGACACAATTCAAC
CCCTAAATTGCTAAATA
Y

AGTAGTCACTTTTTAGTCCTAGCTGATTTGTCCTCTCTAATATTTAATACTGTTGACCATTCCCTGCCTTTTGAGATACGTTC
TTTTGATGACTTCCATG / Celera SNP ID: hCV11341886 / Public SNP ID: rs7539415 /
SNP Chromosome Position: 169339612 / SNP in Genomic Sequence: SEQ ID NO: 440 /
SNP Position Genomic: 247843 / Related Interrogated SNP: hCV8919444 / SNP
Source: dbSNP; Celera; HapMap / Population(Allele,Count): Caucasian
(C,54|T,66) / SNP Type: INTRON /
Context (SEQ ID NO: 2477): /
TTAAACATGTATCTTTTGTTGACACATGATTGTTTTGAAAATTACTTTTTCTCTGATTTTTTTTTTTGCATTCTTTTTAGTGCC
AGTAATTTTGAATATAG
R

GTATAGCATGTATTAGCATTTTCTTGGCATTTCTTCTTGGTAAGTAGTTTCTGTTTTGTTTCCTTTTTGTTAGTCACCGTTAC
TTCATCCCCAATCCGAG / Celera SNP ID: hCV11341898 / Public SNP ID: rs12563090 /
SNP Chromosome Position: 169345704 / SNP in Genomic Sequence: SEQ ID NO: 440 /

SNP Position Genomic: 253935 / Related Interrogated SNP: hCV11975250 / SNP Source: dbSNP; Celera / Population(Allele,Count): Caucasian (G,93|A,131) / SNP Type: INTRON /
Context (SEQ ID NO: 2478): /
ATAAATTTGAAGACATTCAATAGAAACTATTCAAAAATAAGCATGGAGAGAAAGAAAAATGAAGAAGAAGAAAAAAGCCCTGC
TGTGCAACCATGTCAAG
Y

TGTCTATTATATGTGTAATTAGAATCTCAGAAGAGAAGAAAAGGTGATAAACATAAATATATTTTTTTAAAATAAGTGCTAAAA
ACTTTCCAAATTTGCTG / Celera SNP ID: hCV16141160 / Public SNP ID: rs2157597 /
SNP Chromosome Position: 169201567 / SNP in Genomic Sequence: SEQ ID NO: 440 /
SNP Position Genomic: 109798 / Related Interrogated SNP: hCV8919444 / SNP
Source: dbSNP; HapMap; HGBASE / Population(Allele,Count): Caucasian
(C,155|T,71) / SNP Type: INTRON /
Context (SEQ ID NO: 2479): /
GTTCCCATTGGTCTGTTCCCTTCACAACAAAATTCAAAAGAAATGTCTTTCTTTTCCATTTCCATTTTTACTTCCTCTCCTAC
AGTCTCTTTTAAATCCA
Y

AACCAATTAGGTTTTCATTCCACCAAAGCTGCTCATTAAAATCCCTTACTACCTCCATGTTGCTAAGTCCACTGGTTAATTAT
TCTCAGCCCTCACCTTA / Celera SNP ID: hCV27242356 / Public SNP ID: rs12121994 /
SNP Chromosome Position: 169258306 / SNP in Genomic Sequence: SEQ ID NO: 440 /
SNP Position Genomic: 166537 / Related Interrogated SNP: hCV8919444 / SNP
Source: dbSNP; Celera; HapMap / Population(Allele,Count): Caucasian
(C,153|T,73) / SNP Type: INTRON /
Context (SEQ ID NO: 2480): /
TCAAGTGAAGCCCCCTGAAATACGGAGAATAAGAATCTTAGAGGTAAATCCGAGGACAAGCAGGTATAACTAAGGATGTTGGA
AAGGGACGGAGGTCTGG
Y

TGGAGCTCGGTGGCCAGTAGCTTTGGATTTAGGGAAAGATTGTTCCGGGAAGACTTTTTCAGACAGGGATGAAACTTAGGTTT
CGACTTCCCCGGACAGC / Celera SNP ID: hCV27242515 / Public SNP ID: rs3818844 /
SNP Chromosome Position: 169337638 / SNP in Genomic Sequence: SEQ ID NO: 440 /
SNP Position Genomic: 245869 / Related Interrogated SNP: hCV8919444 / SNP
Source: dbSNP; Celera; HGBASE / Population(Allele,Count): Caucasian
(C,51|T,65) / SNP Type: INTRON;PSEUDOGENE /
Context (SEQ ID NO: 2481): /
TTTCAAAAGGCATGGGTGGAATTAACAAGACTTAAGGTTTCAAAGAAACTAGCAAATGTATGCCCTATGGTGCTCTTGAAGTG
TTTGCTGGTTTTAGCAT
Y

TTTCTTCATGTGCTATAGGGCCATTATGATAAACAAGTAGCATCTTTACCACAGGAAGTTAGATACCAAGTCATCAGACACTA
GAAGTATAGAGACACTA / Celera SNP ID: hCV32141254 / Public SNP ID: rs10732287 /
SNP Chromosome Position: 169276816 / SNP in Genomic Sequence: SEQ ID NO: 440 /
SNP Position Genomic: 185047 / SNP Source: dbSNP; HapMap /
Population(Allele,Count): Caucasian (T,69|C,157) / SNP Type: INTRON /
Context (SEQ ID NO: 2482): /
AAGAAGCTGGTCAGGCAGGCGGGCAGGGAGGGTGGGTCCTCAGTTGAATCCTTTCAAACAAAAGAACAGCCTGCAGGCACAGG
TAAGGGAACTGCACAGG
R

TGGCTTGCCTAAGACATGCCCACAGCCCCACTGATAAGAAAGGCTACACAAGTGACTTGCCCAGTCAGGCTTGCAATGGAAAA
TTCCATCCCCTGACACA / Celera SNP ID: hCV32141090 / Public SNP ID: rs12039443 /
SNP Chromosome Position: 169172290 / SNP in Genomic Sequence: SEQ ID NO: 440 /
SNP Position Genomic: 80521 / Related Interrogated SNP: hCV8919444 / SNP
Source: dbSNP; HapMap / Population(Allele,Count): Caucasian (A,58|G,36) / SNP
Type: INTRON /
Context (SEQ ID NO: 2483): /
AAGGGCATCCTTGACTATCTCAGCACTCTTTATTTCATTTTATTGATAAAAGAAGTGAGGCATGGAAAGAAAATGTGACTTAT
TCAAGTTCATATCAAGA
M

AATAATCAGCCATATAAGAGGAAGCTACTGATGCTCTAACTTCTGATTTTTGATTAATTCTCTAAACCACACAGTCATTAATG
TACACTGAAATCTCCAG / Celera SNP ID: hDV70694593 / Public SNP ID: rs16861990 /
SNP Chromosome Position: 169135127 / SNP in Genomic Sequence: SEQ ID NO: 440 /
SNP Position Genomic: 43358 / Related Interrogated SNP: hCV11975250 / SNP
Source: dbSNP; HapMap / Population(Allele,Count): Caucasian (A,208|C,18) /
SNP Type: INTRON /
Context (SEQ ID NO: 2484): /

CATGTTTTAGACTCCTTTCTAAGTCACCTCCTAGAATTTTTAATCAGTCACCTAAGAGTTACTTATTAACTCAAAAACTGCTT
AGTCACTGACTGAAAAA
Y
GAGGCAGGGAGCAGACCAAAGTCAAGAGTTCAGGGTCAAACTGCAGAGAGCAAAGCACAATGAGTGAATTGGTTTCATCTTTC
TCCTCAGTCTTCCAGAG / Celera SNP ID:    hDV70942075 / Public SNP ID:    rs17349271 /
SNP Chromosome Position:    169117882 / SNP in Genomic Sequence:    SEQ ID NO: 440 /
 SNP Position Genomic:    26113 / Related Interrogated SNP:    hCV8919444 / SNP
Source:    dbSNP; HapMap / Population(Allele,Count):    Caucasian (T,157|C,69) /
SNP Type:    INTRON /
Context          (SEQ ID NO: 2485): /
TCCCTATAACATAATTACTATCCAGTCTTGCTTTACTATTTTAAAGAATCCTACAATTCAATGCTCAGAAACTTCCTCAAAGG
AATAAAATAATGAAATA
R
CTGGGACCCACTCTTGGCACTTCTAACATCCAATTCTTGGCAAATCCTGTCAATACAACTACTAATATATACCCTGTATCAGT
CGGCTTCTATATCCACT / Celera SNP ID:    hDV70942101 / Public SNP ID:    rs17349439 /
SNP Chromosome Position:    169146435 / SNP in Genomic Sequence:    SEQ ID NO: 440 /
 SNP Position Genomic:    54666 / Related Interrogated SNP:    hCV8919444 / SNP
Source:    dbSNP; HapMap / Population(Allele,Count):    Caucasian (G,157|A,69) /
SNP Type:    INTRON /
Context          (SEQ ID NO: 2486): /
GCCATCATATTGGACAGTAGAGACCTAGAGAATAGTAGCTCTCTCCCAGGACCGAGGTTTAACTTCTGAGCAACATTCTACAT
TAAGGGCTGAGCCAAGA
S
TAATAATATCTGCAGTATTAACCTCTCTCATGCTAATGGAAACCTTGAACATAAGCTTGAGGGCAGTCAATCTAACCATATTT
CTCATGCTGCCATCCTA / Celera SNP ID:    hCV32398607 / Public SNP ID:    rs4656658 /
SNP Chromosome Position:    169150253 / SNP in Genomic Sequence:    SEQ ID NO: 440 /
 SNP Position Genomic:    58484 / Related Interrogated SNP:    hCV8919444 / SNP
Source:    dbSNP; HapMap; HGBASE / Population(Allele,Count):    Caucasian
(C,157|G,69) / SNP Type:    INTRON /
Context          (SEQ ID NO: 2487): /
AAATTTTGATTAATTAATTACTATTTGTCTAAAGTCTTGGTTTCCTATTTTAGTAACAATGAATAGATTATAACGTGTTGCTA
TCATGATTTACTTAGAT
R
CTCAAATTGGTTTCGATTCAACTAGTGGAACCCCTTCAAGCTGGCTTCTGTGTCCTATTGATATGTCACCATTACTTTGGCAT
AAAAAGATTTTCCAAGA / Celera SNP ID:    hDV77030721 / Public SNP ID:    rs4656664 /
SNP Chromosome Position:    169192760 / SNP in Genomic Sequence:    SEQ ID NO: 440 /
 SNP Position Genomic:    100991 / Related Interrogated SNP:    hCV8919444 / SNP
Source:    CDX; dbSNP / Population(Allele,Count):    Caucasian (G,157|A,69) / SNP
Type:    INTRON //

Gene Number:    104 / Gene Symbol:    TPRKB - 51002 / Gene Name:    TP53RK binding
protein / Chromosome:    2 / OMIM NUMBER: / OMIM Information: // Genomic
Sequence (SEQ ID NO: 441): // / SNP Information /
Context          (SEQ ID NO: 2488): /
GTCCACAGAGAGATGAAACAGCTGCAACCGCTTCTCCCTCAAGGTGGGATCATCAACGGGCAGAGCTCCTACTGTGCCCACCA
CCTTCTCTTCAGATTCA
S
CCACCCAGAAGCAGGAGCCACACTCACTCAGGTAGGATTTGGTGATGTCAGACATGTCTGTGCGCAATGCTATGTCTACATAC
CGCGTCCAGGGTTTTTT / Celera SNP ID:    hCV11541681 / Public SNP ID:    rs2001490 /
SNP Chromosome Position:    73928098 / SNP in Genomic Sequence:    SEQ ID NO: 441 /
SNP Position Genomic:    44079 / SNP Source:    Applera / Population(Allele,Count):
 Caucasian (C,10|G,10) African American (C,18|G,14) total (C,28|G,24) // /
SNP Type:    MISSENSE MUTATION;TRANSCRIPTION FACTOR BINDING SITE;UTR3 / SNP
Source:    dbSNP; Celera; HGBASE / Population(Allele,Count):    Caucasian
(C,40|G,80) / SNP Type:    MISSENSE MUTATION;TRANSCRIPTION FACTOR BINDING
SITE;UTR3 /
Context          (SEQ ID NO: 2489): /
AATGTTTTCTTTAATGCTGAGAATTTTTGTTAATATTTCTGACATTTCATAAAACATCTTTTGTTGACATTCTACAAATGATA
GCATCCAATAATGTCCC
R
ATACTTTCTTCTTGTGAAGAGAGTTTATATATCTGTAAAAATGAAAGACAAATTATGACTTGCTGATATCGTCATTCACTACT
AATTGTCATTATACAAA / Celera SNP ID:    hCV11938725 / Public SNP ID:    rs7210 / SNP
Chromosome Position:    73957124 / SNP in Genomic Sequence:    SEQ ID NO: 441 / SNP

Position Genomic: 73105 / SNP Source: Applera / Population(Allele,Count): Caucasian (A,17|G,23) African American (A,25|G,13) total (A,42|G,36) // / SNP Type: ESE;SILENT MUTATION;INTRON;PSEUDOGENE / SNP Source: dbSNP; HapMap; ABI_Val; HGBASE / Population(Allele,Count): Caucasian (A,80|G,136) / SNP Type: ESE;SILENT MUTATION;INTRON;PSEUDOGENE /
Context (SEQ ID NO: 2490): /
ACTTTCTTCTTGTGAAGAGAGTTTATATATCTGTAAAAATGAAAGACAAATTATGACTTGCTGATATCGTCATTCACTACTAA
TTGTCATTATACAAACA
W
GTGTAAGTGAAATTATTCCTCATTCAATTATTGGGTTCATGGTATAACAAACAACAAATGTGATTAAAATATTCAAAGTAGAG
GGCAATTCATTACTTGT / Celera SNP ID: hCV25924555 / Public SNP ID: rs13003035 /
SNP Chromosome Position: 73957227 / SNP in Genomic Sequence: SEQ ID NO: 441 /
SNP Position Genomic: 73208 / Related Interrogated SNP: hCV11541681 / SNP
Source: Applera / Population(Allele,Count): Caucasian (A,12|T,4) African
American (A,2|T,0) total (A,14|T,4) / SNP Type: TRANSCRIPTION FACTOR BINDING
SITE;INTRON / SNP Source: dbSNP; HapMap / Population(Allele,Count): Caucasian
(T,38|A,82) / SNP Type: TRANSCRIPTION FACTOR BINDING SITE;INTRON /
Context (SEQ ID NO: 2491): /
TGAGTGACAGTGAGCACTGCCCACAGAGGGGGTGCCCTGCAGTCAGGAGGGGTGGGAAACGGGTGCAGATCGGCAGCCAACAG
AGGCCCACCCACATGAG
Y
ACAGGTGGAGTTGCTGGTGCGATGAAGAGTGTGCTGGTTGGGAGTGGAGGGCTGGGGGCCGGTAGCCGGTTTGGGAAACCCAA
GCTGAGCGAAAGGTGTG / Celera SNP ID: hCV95670 / Public SNP ID: rs4852975 / SNP
Chromosome Position: 73932465 / SNP in Genomic Sequence: SEQ ID NO: 441 / SNP
Position Genomic: 48446 / Related Interrogated SNP: hCV11541681 / SNP Source:
dbSNP; Celera; HapMap; HGBASE / Population(Allele,Count): Caucasian
(C,86|T,140) / SNP Type: INTERGENIC;UNKNOWN /
Context (SEQ ID NO: 2492): /
CTTGTTGAAAAGCCACCTAAAATTGTCATAAGTAAGAGAGGTGATCTAATGGCCCATGAAACTGCCAACTCCAGGAAGACAGA
CAGGGTGACCTGAAAGT
R
GGTTTGACCTGTTGGTTTTTCTGCCATTCTGTGGGTGGCATCAGCTTCCTGCCAAGACTGGCACTGCATAGGATGTCGGGTGT
TTGCCAGCCCAGTTGAA / Celera SNP ID: hCV95671 / Public SNP ID: rs11126414 /
SNP Chromosome Position: 73932044 / SNP in Genomic Sequence: SEQ ID NO: 441 /
SNP Position Genomic: 48025 / Related Interrogated SNP: hCV11541681 / SNP
Source: dbSNP; Celera; HapMap; ABI_Val / Population(Allele,Count): Caucasian
(G,86|A,140) / SNP Type: INTERGENIC;UNKNOWN /
Context (SEQ ID NO: 2493): /
GTGCAGATCGGCAGCCGGCAGAGGCCCACCCAGGTGAGCACAGGTGGACCTGATGGTGGATGGGGACAAAGAGTGTGCTGGTT
GGGAGGAGAAGGCGGGG
R
CCTGTAGCTGGTCTGGGAATTCCCAAGCTGAGCAAACGGTATGTCAAGGCCTGTGGTTGGGCTTGTTAACATAAACACCACAC
TCTTAAATGATCTGTAA / Celera SNP ID: hCV95672 / Public SNP ID: rs6750515 / SNP
Chromosome Position: 73946533 / SNP in Genomic Sequence: SEQ ID NO: 441 / SNP
Position Genomic: 62514 / Related Interrogated SNP: hCV11541681 / SNP Source:
dbSNP; Celera; HapMap / Population(Allele,Count): Caucasian (G,37|A,81) / SNP
Type: INTERGENIC;UNKNOWN /
Context (SEQ ID NO: 2494): /
AGGAACTGGAGCTGAGTGCAGAGGACACCATGGAGCAGTTTTGCCACAGAGTGAAACAGGGAGATGCCTGGCAGCTGCAGAGG
AGGGTGCAGTCAAAAAG
R
TTTGTTACAGAACTGCCGTTTGACTCAGCAATCCTATTACTAGGTATATATCCAGAGAAATAGAAATCGTTCTACCATAGAGA
TGCATGTGAATGTTCAT / Celera SNP ID: hCV112099 / Public SNP ID: rs12052539 /
SNP Chromosome Position: 73937153 / SNP in Genomic Sequence: SEQ ID NO: 441 /
SNP Position Genomic: 53134 / Related Interrogated SNP: hCV11541681 / SNP
Source: dbSNP; Celera; HapMap / Population(Allele,Count): Caucasian
(A,83|G,143) / SNP Type: INTERGENIC;UNKNOWN /
Context (SEQ ID NO: 2495): /
TCAGCCAGTCCTACCCAGAACACCCTATTTCACAGTGCACTTCACCCCTAGCCTACCTGGCCATTCCCTATCCCCATAGCTGG
CTCTAAGTTTTCTTATC
M
CCTCTAACCTGCTCTAAATGCTAAGGATATGCATCTGTTGTTGATTTGTATTTAGTAGGCTCTGCAAGCAGGGATCTTTGGCT
CTTCGGTTCACTCATGT / Celera SNP ID: hCV112100 / Public SNP ID: rs17350125 /

SNP Chromosome Position: 73936184 / SNP in Genomic Sequence: SEQ ID NO: 441 / SNP Position Genomic: 52165 / Related Interrogated SNP: hCV11541681 / SNP Source: dbSNP; Celera; HapMap / Population(Allele,Count): Caucasian (A,38|C,82) / SNP Type: INTERGENIC;UNKNOWN / Context (SEQ ID NO: 2496): / GTGCAGAGGACACCATGGAGCAGTTTTGCCACAGAGTGAAACAGGGAGATGCCTGGCAGCTGCAGAGGAGGGTGCAGTCAAAA AGATTTGTTACAGAACT R CCGTTTGACTCAGCAATCCTATTACTAGGTATATATCCAGAGAAATAGAAATCGTTCTACCATAGAGATGCATGTGAATGTTC ATTGCAGCAGTATTCAC / Celera SNP ID: hCV133926 / Public SNP ID: rs12053242 / SNP Chromosome Position: 73937168 / SNP in Genomic Sequence: SEQ ID NO: 441 / SNP Position Genomic: 53149 / Related Interrogated SNP: hCV11541681 / SNP Source: dbSNP; Celera; HapMap / Population(Allele,Count): Caucasian (G,38|A,82) / SNP Type: INTERGENIC;UNKNOWN / Context (SEQ ID NO: 2497): / GAGAACTTATGAACACAAAGAAGGAAACAACAGACACTGGGGCCTTCCCCAGGGTGGAGAGTGGGAGGAGGGAGAGGAGCAGA AAAATAATTTTGAGTAC K AGTCTTAGTACCTGGGTAATGAAATAATCTGTACCATAAACCCCCATGACATGAATTCACCAATATAACAAACCTGCACCTGT ACCCCTGAACCTAAAAT / Celera SNP ID: hCV133927 / Public SNP ID: rs7599453 / SNP Chromosome Position: 73937901 / SNP in Genomic Sequence: SEQ ID NO: 441 / SNP Position Genomic: 53882 / Related Interrogated SNP: hCV11541681 / SNP Source: dbSNP; Celera; HapMap / Population(Allele,Count): Caucasian (T,36|G,80) / SNP Type: UTR3 / Context (SEQ ID NO: 2498): / GGGACTGACAGTGTGTTATGTGCTGGGCTTGGCAGAAGTTCTCTCCATTGCCTCTTCTGTTGTGTCTTGGTTTTTAACAATGA ACTAGGAAAGGATATTA Y CAACTAAGAGTGAGGGTCAGAGAAGAGGCATTGTTCTTTGAGGGGGAGAGAAATGATAAGCCGTTGTCCAGTAGTTGCCTCTT CTGTTCAGTAAGGACAG / Celera SNP ID: hCV133928 / Public SNP ID: rs4852977 / SNP Chromosome Position: 73938295 / SNP in Genomic Sequence: SEQ ID NO: 441 / SNP Position Genomic: 54276 / Related Interrogated SNP: hCV11541681 / SNP Source: dbSNP; Celera; HapMap; HGBASE / Population(Allele,Count): Caucasian (C,38|T,82) / SNP Type: UTR5 / Context (SEQ ID NO: 2499): / ATGCTTTATAGTCCTCAAACATGCTGAATGCTGAGGGAGTTCCTCCATTCATACAGCTGCTTGCAGTACCTGGGTGCTATGGA CTGAATATGTCCCCCCA R AACTCCTATGTTGAAACCTCATCCCCAAGGTGATGGTATTCAGAGGTGGGGTCTTGGGAGGTGATTAGGTCGTGAGGATGAAG TCTTCACATATGGGATC / Celera SNP ID: hCV133930 / Public SNP ID: rs1815028 / SNP Chromosome Position: 73938926 / SNP in Genomic Sequence: SEQ ID NO: 441 / SNP Position Genomic: 54907 / Related Interrogated SNP: hCV11541681 / SNP Source: dbSNP; Celera; HapMap; ABI_Val; HGBASE / Population(Allele,Count): Caucasian (A,38|G,82) / SNP Type: INTERGENIC;UNKNOWN / Context (SEQ ID NO: 2500): / TGTTTTGACCAATCAAAGGTGGCCCAGTGTTCAAACCATGTTCAAATAAGGCAAACGTCGAGCTGGAACCAATCCGGCTGTTT CTGTGGCTTACTTCCAC Y TTCCATACACTGCTTATCATTTTGTGTCCATAAATCATCTTCCACCACATGGGTGCACTGGAGTCTCTGAGCCTATTCTGGCT CAGGAGGCCGCCTGATT / Celera SNP ID: hCV180709 / Public SNP ID: rs7591112 / SNP Chromosome Position: 73944272 / SNP in Genomic Sequence: SEQ ID NO: 441 / SNP Position Genomic: 60253 / Related Interrogated SNP: hCV11541681 / SNP Source: dbSNP; Celera; HapMap / Population(Allele,Count): Caucasian (C,39|T,81) / SNP Type: TRANSCRIPTION FACTOR BINDING SITE;INTERGENIC;UNKNOWN / Context (SEQ ID NO: 2501): / AGGTCAGTTTCTCCTTCCAGATTTTGTGGCAGAGAAACTAGTGAATTTCAAGTTCCTTGTGTTAAGTAAGTTTAGCCTACTGC TTCCTTACATATTTTAA R TTCGTCCTAAAGGGTTCTCTGTACTCCACAAACTGTAACCTAAATGGGCTTGTAAATGGACTATAGCCTACTCTTTGCCACTC ACCATGTTTTGACCAAT / Celera SNP ID: hCV180710 / Public SNP ID: rs11891140 / SNP Chromosome Position: 73944084 / SNP in Genomic Sequence: SEQ ID NO: 441 / SNP Position Genomic: 60065 / Related Interrogated SNP: hCV11541681 / SNP Source: dbSNP; Celera; HapMap / Population(Allele,Count): Caucasian

(A,39|G,81) / SNP Type: INTERGENIC;UNKNOWN /
Context (SEQ ID NO: 2502): /
GTCTTAACCTGTGTTGGGCGTTGCACCGGGCGACCTCAGTTTCTTCCTGTACAAGGAAAAGTACTGACCAAAATGAGTTCTAC
ATACATTTCCGCTGCTG
S
AGATTTCTTTGGTTTTTTGAGACAGAGTCTTGCTCTGTTACTCAGGCTGGAGTGCTGTGGTGCAATCTCGGCTCACTGCAACCT
CCGCGTCCCGGTTCAAG / Celera SNP ID: hCV303807 / Public SNP ID: rs17350188 /
SNP Chromosome Position: 73964842 / SNP in Genomic Sequence: SEQ ID NO: 441 /
SNP Position Genomic: 80823 / Related Interrogated SNP: hCV11541681 / SNP
Source: dbSNP; Celera; HapMap / Population(Allele,Count): Caucasian
(G,80|C,146) / SNP Type: TRANSCRIPTION FACTOR BINDING SITE;INTERGENIC;UNKNOWN /
Context (SEQ ID NO: 2503): /
ATGGGCTTAAAGAATGTGAACGATGTGCTCACAGAGGCCACAGAAGAGAAATTATAGCCAGGAGAACAACCTGAAAGACAAAG
GACACGGTGGCATAAGC
R
CATGTAACACAATGTACTCAGGAAATGGCTGGCATCCTGAGATATGGAGTGGAATACAGTACAGGGCTTTGTAAACTCAGCTT
GGAGTCAGATCACAGAA / Celera SNP ID: hCV505733 / Public SNP ID: rs11126416 /
SNP Chromosome Position: 73973877 / SNP in Genomic Sequence: SEQ ID NO: 441 /
SNP Position Genomic: 89858 / Related Interrogated SNP: hCV11541681 / SNP
Source: dbSNP; Celera; HapMap; ABI_Val / Population(Allele,Count): Caucasian
(A,38|G,82) / SNP Type: INTERGENIC;UNKNOWN /
Context (SEQ ID NO: 2504): /
AACAATTACAGATACATGGCAGCATGTTCACATTGTCTCAGGCTTCCCCCAACTACATCTGAACAGTTTGAATTGGATTAAAA
AGGGCTCCTGGAGACTT
R
TAGGCTTGCTTATCCTGTGGGTAACTGACATTGGTTGCAGTAATGATACCAAGGTTTCTTTTCTTTTTCCAGGTAGGAAAACA
TGTGATTTGCTCTATTT / Celera SNP ID: hCV512569 / Public SNP ID: rs6755500 /
SNP Chromosome Position: 73942233 / SNP in Genomic Sequence: SEQ ID NO: 441 /
SNP Position Genomic: 58214 / Related Interrogated SNP: hCV11541681 / SNP
Source: dbSNP; Celera; HapMap / Population(Allele,Count): Caucasian
(G,78|A,144) / SNP Type: INTERGENIC;UNKNOWN /
Context (SEQ ID NO: 2505): /
CCTTCCACCCAGTCCCCTCACCTGTCACCACAAGAGCCTTGAGCGTCCATGGAGCTTCAGCTCTCAGCCACTCATTGGCATCC
AGGAGATAGTGTCCAGG
R
CCCGTCCATCCCTGGAAAGCAGGCTGGCTGCCCCCTCATTGCTTGGCTCCTTAATTTTTGATAATCATAAACCCTAGGGTCAA
AGAGCTGGGCTGTAACA / Celera SNP ID: hCV1835582 / Public SNP ID: rs12713789 /
SNP Chromosome Position: 73945600 / SNP in Genomic Sequence: SEQ ID NO: 441 /
SNP Position Genomic: 61581 / Related Interrogated SNP: hCV11541681 / SNP
Source: dbSNP; Celera; HapMap / Population(Allele,Count): Caucasian
(A,38|G,80) / SNP Type: INTERGENIC;UNKNOWN /
Context (SEQ ID NO: 2506): /
AGGTGATCTAATGGCTCAAAAAAACCACCAACTCTAGGAAGACAGACAGGGTGACCTGAAAGTGGGTTTCACCTGGTGTTTTTC
TGCCATTCTGTGCATGG
Y
GTCAGCTTCCTCCCAAGACTGGCACTGCATAGGACATCGGGTGTTTGCCAACCCAGTTGAAGCTCTCTGCTTCCTTGCTCACC
ACCAGCAGAAAGGTGAG / Celera SNP ID: hCV1835584 / Public SNP ID: rs6749841 /
SNP Chromosome Position: 73946097 / SNP in Genomic Sequence: SEQ ID NO: 441 /
SNP Position Genomic: 62078 / Related Interrogated SNP: hCV11541681 / SNP
Source: dbSNP; Celera; HapMap / Population(Allele,Count): Caucasian
(C,81|T,145) / SNP Type: INTERGENIC;UNKNOWN /
Context (SEQ ID NO: 2507): /
TATTATCCCAAGGGCCTGGGTAGTCAGGAAATCACAGAATTTTAAAGCTGCAAGGAACTTATGCAAATAGTAAAATTTTTTTA
AATAACATTAATTGAGG
K
CTAATATTATGGGCATGATCATATTGCAAACATTTTACTTGTAATATTTTCTTTAATCCTCAAAACTACCCTATGAAATTGAC
ACTATTATTAGTAGTAG / Celera SNP ID: hCV11537012 / Public SNP ID: rs12992607 /
SNP Chromosome Position: 73959960 / SNP in Genomic Sequence: SEQ ID NO: 441 /
SNP Position Genomic: 75941 / Related Interrogated SNP: hCV11541681 / SNP
Source: dbSNP; Celera; HapMap / Population(Allele,Count): Caucasian
(G,38|T,82) / SNP Type: INTRON /
Context (SEQ ID NO: 2508): /
GCCAGGAAGAAGGGTGGGAGCTCTGCCGAGGTGTCCCTCTGAGTGACCACCCTCAGCTGGAGCCGCCAGGCCTCTGCACAGTA

GCTGCCCCTGGGCCTTG
Y
GTGTAAAATGGGGGTAATAACAGTATCTACCTCATAAGGTTGCTGCAAGAACTAAATGAGTTCCTAGAAGTGAAAGACACTTA
GAACCAGGATCCAGAGG / Celera SNP ID: hCV11537013 / Public SNP ID: rs12713793 /
SNP Chromosome Position: 73956114 / SNP in Genomic Sequence: SEQ ID NO: 441 /
SNP Position Genomic: 72095 / Related Interrogated SNP: hCV11541681 / SNP
Source: dbSNP; Celera; HapMap / Population(Allele,Count): Caucasian
(T,81|C,145) / SNP Type: INTERGENIC;UNKNOWN /
Context (SEQ ID NO: 2509): /
CAGCCGACCTGGGGCTCCACAGCTCTTGCTGCCGCTGGAAGTTGGGCCAAGGCTGGTGGCACACACAGGTGTGTTCCTGCTGT
TGGGATCACCAGACAAG
K
CTGCTTCGCCTTGGGCCGATCTTTTGCCCCGGACCACTCAGCCCTAGGTACTTTAGGCCAATCTGCTGATCAAGGCCATAAGT
GCCTCATAACCAATCAG / Celera SNP ID: hCV11541694 / Public SNP ID: rs12619258 /
SNP Chromosome Position: 73935915 / SNP in Genomic Sequence: SEQ ID NO: 441 /
SNP Position Genomic: 51896 / Related Interrogated SNP: hCV11541681 / SNP
Source: dbSNP; Celera; HapMap / Population(Allele,Count): Caucasian
(G,40|T,80) / SNP Type: INTERGENIC;UNKNOWN /
Context (SEQ ID NO: 2510): /
TTTCTGTCTCTATGGATTTATTTATCGTGGATGTTTCATATAAATGGAATTGTACAATATCTGACCTTTTATGTGTGGCTTCT
TTCAGTTAATGTTTTCA
R
AATTCATTCGTGTTGCATCATGCATCAGTATTTCATTCCTTTTTAGGGCTCAAAATTCCATGTATGTTTATACCGCACTTTGT
TTATCCATTCATTCATT / Celera SNP ID: hCV11541701 / Public SNP ID: rs6748233 /
SNP Chromosome Position: 73941002 / SNP in Genomic Sequence: SEQ ID NO: 441 /
SNP Position Genomic: 56983 / Related Interrogated SNP: hCV11541681 / SNP
Source: dbSNP; Celera; HapMap / Population(Allele,Count): Caucasian
(G,38|A,82) / SNP Type: INTERGENIC;UNKNOWN /
Context (SEQ ID NO: 2511): /
TTAAGCTATAGATACTTGCATATGTGCAAAATAGTGTACAAAATTAACGACTGAAGCAGTTTGTTATAGCAGAAAATTAAAAC
AGTTTATCTGTCCATCA
R
TAGACAAACTGTTAAATAAATTACTGTATATCCAGATAATGAAGTCCTACATAGAATGAGGAAGGTCTGTCTATACCAGTATG
GAAAGATCTCCAAGACA / Celera SNP ID: hCV11541702 / Public SNP ID: rs4852978 /
SNP Chromosome Position: 73943110 / SNP in Genomic Sequence: SEQ ID NO: 441 /
SNP Position Genomic: 59091 / Related Interrogated SNP: hCV11541681 / SNP
Source: dbSNP; Celera; HapMap; ABI_Val; HGBASE / Population(Allele,Count):
Caucasian (G,38|A,82) / SNP Type: INTERGENIC;UNKNOWN /
Context (SEQ ID NO: 2512): /
CCCTCATTGCTTGGCTCCTTAATTTTTTGATAATCATAAACCCTAGGGTCAAAGAGCTGGGCTGTAACACAATAGGGAGTGCAT
TCCCCAGTGTATTCCCC
Y
GTGTGGTCACAGGGAATGAGCCTGGAGCAAGGGAGCCCAGCAGGCTGAGAATCTGCAGTGTGCTGGGCCTGGGCTTGGCATGA
AGGCACGAGATGTCGGA / Celera SNP ID: hCV11541712 / Public SNP ID: rs12713791 /
SNP Chromosome Position: 73945733 / SNP in Genomic Sequence: SEQ ID NO: 441 /
SNP Position Genomic: 61714 / Related Interrogated SNP: hCV11541681 / SNP
Source: dbSNP; Celera; HapMap / Population(Allele,Count): Caucasian
(C,81|T,145) / SNP Type: TRANSCRIPTION FACTOR BINDING SITE;INTERGENIC;UNKNOWN /
Context (SEQ ID NO: 2513): /
ACAGAGGCTCCTGTCACTGAGACTGCCCTCAGTGGGACAGAGGGAAGAGACTATTGATGCAGTTCGTCTGCCTCTCTGTGACA
CTGATGTGTAGGCCCAG
Y
AATTAAAGTCCACTCCTGGCAGCCAGCCTTGAACATAGAACTGATCGGCCACACAGAAAACCTGAAACAAAGCCTCTAAAATG
CAGCCTCTCAAAAACGC / Celera SNP ID: hCV11541719 / Public SNP ID: rs12615807 /
SNP Chromosome Position: 73947583 / SNP in Genomic Sequence: SEQ ID NO: 441 /
SNP Position Genomic: 63564 / Related Interrogated SNP: hCV11541681 / SNP
Source: dbSNP; Celera; HapMap / Population(Allele,Count): Caucasian
(T,38|C,82) / SNP Type: TFBS SYNONYMOUS;INTERGENIC;UNKNOWN /
Context (SEQ ID NO: 2514): /
TGGGTGCTATTGTTCCCACTAAAAGCCCAGACCTCTGCCTCAGGAGACAGGGCTCAGCTGCAGCCTCCTCAACTAAAACATGA
AGGGCTGCTTCGTGGGC
R
CCAAAGCGGCTCCTTAAACTCGCAATGGTGTGCAAATTTATGGGCTCACTTTCTACATCAGATCTTCAAAGGTCTCTGTGATT

CCAAAAGCTTGGGAACT / Celera SNP ID: hCV11541721 / Public SNP ID: rs2006997 /
SNP Chromosome Position: 73947846 / SNP in Genomic Sequence: SEQ ID NO: 441 /
SNP Position Genomic: 63827 / Related Interrogated SNP: hCV11541681 / SNP
Source: dbSNP; Celera; ABI_Val; HGBASE / Population(Allele,Count): Caucasian
(G,38|A,82) / SNP Type: INTERGENIC;UNKNOWN /
Context (SEQ ID NO: 2515): /
CTCAGCTTCTTGGTGCATTGAGAGAGTACCTACCTCAGGCAGGCCACATGCCCAACCCTTTGGTGCTTCTCCAGGGACCATAC
ATCGCAGAAATCCTGTG
W
CCAAGCACAAGTTTCAGGAAAGGCTCCCTCCAGCTTTATTGTAAGTTTTTAGGTTGCTGAGGTGGCATAAGTGCCAAGCATAA
AAAACAGCCCAGCCTCC / Celera SNP ID: hCV11941453 / Public SNP ID: rs2001436 /
SNP Chromosome Position: 73928965 / SNP in Genomic Sequence: SEQ ID NO: 441 /
SNP Position Genomic: 44946 / Related Interrogated SNP: hCV11541681 / SNP
Source: dbSNP; Celera; HGBASE / Population(Allele,Count): Caucasian
(A,40|T,76) / SNP Type: INTERGENIC;UNKNOWN /
Context (SEQ ID NO: 2516): /
AGTTTAGAACCCATGCCTAGGCCTTCCACACTCTACAGGCACCCAGTTCATCCATCAGTAGCAGAACTTAGAGTCTTCCTGGG
ACTCCATACTGCCTGGA
R
AGAGGAATTCGTTCTCTTTCATTGCTGACATTCCTGGGTGAATGCCTGTGGCAGTTATACTTTACCCATCCCATTTTGTACCT
CCTTCCACTCCAGGGGA / Celera SNP ID: hCV15804221 / Public SNP ID: rs2421674 /
SNP Chromosome Position: 73955432 / SNP in Genomic Sequence: SEQ ID NO: 441 /
SNP Position Genomic: 71413 / Related Interrogated SNP: hCV11541681 / SNP
Source: dbSNP; HapMap; ABI_Val / Population(Allele,Count): Caucasian
(G,80|A,146) / SNP Type: INTERGENIC;UNKNOWN /
Context (SEQ ID NO: 2517): /
GCCTCTTCAATGATGAAAGTGTTTTCTTCCTCTTCTACCTCTGCAGAGAGGCTTGCTGGGATAGATTTTGTTTGTAATTCTAT
TTCCCCACCGCCAGAGG
M
CGGACTTTGGGGGTCAGGCAGCTGAGAAGGAGTTTAGAACCCATGCCTAGGCCTTCCACACTCTACAGGCACCCAGTTCATCC
ATCAGTAGCAGAACTTA / Celera SNP ID: hCV15804228 / Public SNP ID: rs2421675 /
SNP Chromosome Position: 73955301 / SNP in Genomic Sequence: SEQ ID NO: 441 /
SNP Position Genomic: 71282 / Related Interrogated SNP: hCV11541681 / SNP
Source: dbSNP; Celera; HapMap; HGBASE / Population(Allele,Count): Caucasian
(C,38|A,82) / SNP Type: INTERGENIC;UNKNOWN /
Context (SEQ ID NO: 2518): /
GTCCCCTCACCTGTCACCACAAGAGCCTTGAGCGTCCATGGAGCTTCAGCTCTCAGCCACTCATTGGCATCCAGGAGATAGTG
TCCAGGACCCGTCCATC
Y
CTGGAAAGCAGGCTGGCTGCCCCCTCATTGCTTGGCTCCTTAATTTTTGATAATCATAAACCCTAGGGTCAAAGAGCTGGGCT
GTAACACAATAGGGAGT / Celera SNP ID: hCV26996655 / Public SNP ID: rs12713790 /
SNP Chromosome Position: 73945611 / SNP in Genomic Sequence: SEQ ID NO: 441 /
SNP Position Genomic: 61592 / Related Interrogated SNP: hCV11541681 / SNP
Source: dbSNP; Celera; HapMap / Population(Allele,Count): Caucasian
(C,39|T,79) / SNP Type: INTERGENIC;UNKNOWN /
Context (SEQ ID NO: 2519): /
TAGGTTTTACCTATCATTATGGTAAAGAATTAAAAGAATGGTATGAGCTAATGTAGGACAAGCACAAAGAAATGTGCAGACAC
ACACACTTTTGATGGAA
Y
ACTGAAATGGGAGATCCTTGCTGGAGAATCATTACACAGCATGTCAAAATCCTTTTTTTTAAATCAAAATAGAGTCTCACCCCA
TTGCTCAGGCCAGAGTG / Celera SNP ID: hCV26996656 / Public SNP ID: rs1806683 /
SNP Chromosome Position: 73939812 / SNP in Genomic Sequence: SEQ ID NO: 441 /
SNP Position Genomic: 55793 / Related Interrogated SNP: hCV11541681 / SNP
Source: dbSNP; Celera; HapMap; ABI_Val / Population(Allele,Count): Caucasian
(T,83|C,143) / SNP Type: TRANSCRIPTION FACTOR BINDING SITE;INTERGENIC;UNKNOWN /
Context (SEQ ID NO: 2520): /
TAAACTTTACTATTGTTGAACTGCCTATGTTGCCTGAACTTTGACTGGTTTAAGTTGGTTTCATTTATTTCATAAGAACTCTT
GTTAAGGGGTGCACCTT
K
GTATTTTGATATTATTCTCTTGATAGTCATAATAATAGTTCCCTGGTGTGCTGCATCCTCTCAAGTCATAAATGTTTTTGTATG
CAGCCATACATTGAGAA / Celera SNP ID: hCV26996674 / Public SNP ID: rs13006448 /
SNP Chromosome Position: 73924424 / SNP in Genomic Sequence: SEQ ID NO: 441 /
SNP Position Genomic: 40405 / Related Interrogated SNP: hCV11541681 / SNP

Source: dbSNP; Celera; HapMap / Population(Allele,Count): Caucasian (G,40|T,80) / SNP Type: INTRON /
Context (SEQ ID NO: 2521): /
ACAATTCCGCTAATGCCAATGCCTCTCCATGTTGCCAATGAGAGTCACCCCAAAAATTCCAAGGGAAGGATAGAAAGCTATCCT
TGATATTCTGAACACCA
Y
TGCTACTTGTTTTCCTGCACTCACTAAAAGCAACACTTTAACTTTTCCTTTTTTTTTTTTTTTTTTTCTGAGGCAGTCTCACTCTGT
TGCCTAGGTTGGAGTGC / Celera SNP ID: hCV26996679 / Public SNP ID: rs6732812 /
SNP Chromosome Position: 73922475 / SNP in Genomic Sequence: SEQ ID NO: 441 /
SNP Position Genomic: 38456 / Related Interrogated SNP: hCV11541681 / SNP
Source: dbSNP; Celera; HapMap; ABI_Val / Population(Allele,Count): Caucasian
(T,84|C,140) / SNP Type: INTRON /
Context (SEQ ID NO: 2522): /
ATGATTTTGGCATGATAGGGAAACTCTAAGGGTTTCTCACTTCTAGTGGGATCACCATCAAAAATGGACTCCAGTTAGATAAA
CTCCTAGTTTGCTGTTA
K
TGATCGAAGCTCATACCTGCAGAACTGAACCTGAATATCAAGGGAATGCTTAGCAGATATTTATGCTAAATCAGCTAGTACTG
AAACTGTTCAGATATGC / Celera SNP ID: hCV26996688 / Public SNP ID: rs13015885 /
SNP Chromosome Position: 73920482 / SNP in Genomic Sequence: SEQ ID NO: 441 /
SNP Position Genomic: 36463 / Related Interrogated SNP: hCV11541681 / SNP
Source: dbSNP; Celera; HapMap / Population(Allele,Count): Caucasian
(G,39|T,79) / SNP Type: INTRON /
Context (SEQ ID NO: 2523): /
CATGCCACTGTATTACTGCTGCCTGATGATGGTGAAGACCACAATTGCATAAGTGTAGCATCAGAAATAGTGGCCCCTCATGT
TAATTTACAAGTTAGTC
S
TTTGGACAATCCTGAGTTAATACTTTTGTTGATGGGTCCTATGCCAAAAGCTCAGAAGGAAAATATCAGCTAGGATATGCTGT
TACCAAAATGAGTTAAT / Celera SNP ID: hCV26996689 / Public SNP ID: rs13014700 /
SNP Chromosome Position: 73920121 / SNP in Genomic Sequence: SEQ ID NO: 441 /
SNP Position Genomic: 36102 / Related Interrogated SNP: hCV11541681 / SNP
Source: dbSNP; Celera; HapMap / Population(Allele,Count): Caucasian
(C,39|G,79) / SNP Type: INTRON /
Context (SEQ ID NO: 2524): /
CCTAAAAAGTTAAAAACTATTCAAAATTTTCCTTGGCCTGCAACCAAAAGATAATTAAATAGTTTTCTTGGACTTGCAGGATA
TGTTTTTAGACTTGCAG
R
ATATAAAATTCCTGGGTTCTGAATTTTTTCCTTAATAGCCTCACCATTTCATGAGCTCCCTAAAAAAATGCTGTACCAGAGCCTT
TATCTTGGGATGATAGT / Celera SNP ID: hCV26996690 / Public SNP ID: rs2421575 /
SNP Chromosome Position: 73919522 / SNP in Genomic Sequence: SEQ ID NO: 441 /
SNP Position Genomic: 35503 / Related Interrogated SNP: hCV11541681 / SNP
Source: dbSNP; Celera; HapMap; ABI_Val / Population(Allele,Count): Caucasian
(G,40|A,80) / SNP Type: INTRON /
Context (SEQ ID NO: 2525): /
TTTTTTTTTTTTTTTTTTTGATAATGGGTTTGTCTCAATCCAAAGATTCTGGGACTCTACCTTCTGGGACTCCATCTAATTTG
ATATGTAAAATTATGGA
Y
CCAGAATATGTGCATTTTTAGAAAAATAGATTAACCTTACTAGAGAAAAGATGGCCACAATGGGGAAGTTTTAATTTGTATAA
AATTGTTTATTTGCATG / Celera SNP ID: hCV26996697 / Public SNP ID: rs12611487 /
SNP Chromosome Position: 73916453 / SNP in Genomic Sequence: SEQ ID NO: 441 /
SNP Position Genomic: 32434 / Related Interrogated SNP: hCV11541681 / SNP
Source: dbSNP; Celera; HapMap / Population(Allele,Count): Caucasian
(C,34|T,62) / SNP Type: INTRON /
Context (SEQ ID NO: 2526): /
ATATCCAGTGCCTCAGTGCGAAGTTCCAGTTTAACTTCCTGTTACCTCGAGCCCACTATCTTGCCCCAATAATACCCTCCCCC
AATTCACAAACACAGAC
R
CATTCCCTCCTACAGCTTTGGGCCTCCTATCTGAGTCCTTCAGGAAAGAAGAGCTTGTAACTCCCTTAGCAGTGAGTGTAGAC
TTGGTCCAAGGAAGATG / Celera SNP ID: hCV26996701 / Public SNP ID: rs7608328 /
SNP Chromosome Position: 73914019 / SNP in Genomic Sequence: SEQ ID NO: 441 /
SNP Position Genomic: 30000 / Related Interrogated SNP: hCV11541681 / SNP
Source: dbSNP; Celera; HapMap / Population(Allele,Count): Caucasian
(G,90|A,134) / SNP Type: INTRON /
Context (SEQ ID NO: 2527): /

AAATTACTTATTAGCCGAAGAAAGGTGAAAGGAAGTTTGGAATTAACAGTCTAGATTTTCTCACTGATTGGTCCAAGTCACTG
ACCTCTAAAATTATCTT
R
GATCATGCCCTCCCTCCTTCCTACTTTCTGGAAAACAATCAGTCTCCTACCCTATGCTTGCCCAAAGAGTTTCCCATACAAAT
CACAAATCTGCACTTGC / Celera SNP ID: hCV26996705 / Public SNP ID: rs12997018 /
SNP Chromosome Position: 73912899 / SNP in Genomic Sequence: SEQ ID NO: 441 /
SNP Position Genomic: 28880 / Related Interrogated SNP: hCV11541681 / SNP
Source: dbSNP; Celera; HapMap / Population(Allele,Count): Caucasian
(A,32|G,68) / SNP Type: INTRON /
Context (SEQ ID NO: 2528): /
AGCCTCACCATTTCATGAGCTCCCTAAAAAATGCTGTACCAGAGCCTTTATCTTGGGATGATAGTTACGAGCAGGCTTTTAGC
TAAATAAAATTGACCTT
R
TAACAGCCTCCAGCTTTAGGACTTCCAAATTGCACTAAACTGTTTACCTAATTGGCTCATGAAAGTAATAATCAGGCATTTAG
GAGTTCTTACCCAGGAA / Celera SNP ID: hCV29307907 / Public SNP ID: rs4852316 /
SNP Chromosome Position: 73919658 / SNP in Genomic Sequence: SEQ ID NO: 441 /
SNP Position Genomic: 35639 / Related Interrogated SNP: hCV11541681 / SNP
Source: dbSNP; HapMap / Population(Allele,Count): Caucasian (G,83|A,143) /
SNP Type: INTRON /
Context (SEQ ID NO: 2529): /
AGACACGTTTTATTGGGAAGCTAACAAATCTTAAGCTTCAAAGTTCCTCTTTCCCCTGCACCAGCACCTTACAAAAAAAAAAA
AACCTACTGTGGTTTTA
Y
AATTTATATTTTGCAAACAGGCTCTGAAATGTATTTACCTCAGGCTCCAAAAAACCTGGATCTTCCTTGAGGGCAGTGCTGCC
TACTGGATGAAATACAG / Celera SNP ID: hCV31840146 / Public SNP ID: rs11126415 /
SNP Chromosome Position: 73936500 / SNP in Genomic Sequence: SEQ ID NO: 441 /
SNP Position Genomic: 52481 / Related Interrogated SNP: hCV11541681 / SNP
Source: dbSNP; HapMap / Population(Allele,Count): Caucasian (C,38|T,82) / SNP
Type: INTERGENIC;UNKNOWN /
Context (SEQ ID NO: 2530): /
AAACTTAAATGTGTGCGGCAGGAGGAAAGTAAACAAAAACCAAGCTGACTAACCACCTTATTACAAGGCCACCATGACAAGAC
TAGAGCGTCCATCATCA
R
CTTTTAGTGCCTCAATGTTAAGTATGGACAAATGAGCAGTAAACATGGTCATGAGGTATTTCAAGAACACTTCTAAGAGCAGA
GACAGAGGTATAAACAA / Celera SNP ID: hCV31840129 / Public SNP ID: rs11126417 /
SNP Chromosome Position: 73974315 / SNP in Genomic Sequence: SEQ ID NO: 441 /
SNP Position Genomic: 90296 / Related Interrogated SNP: hCV11541681 / SNP
Source: dbSNP / Population(Allele,Count): Caucasian (G,80|A,146) / SNP Type:
TFBS SYNONYMOUS;INTERGENIC;UNKNOWN /
Context (SEQ ID NO: 2531): /
GATAGGCCGGCTCGGGGTGGCCATCTGTTTCCGGGTCCTGGTAGGAGGGGTGCTTCCGCCCATGGTCCCGCCCATTCTTCCGC
CTCCCCAACCTGGGTCC
Y
GTCAACGGACGCGAAGGAGAACAGGGGCTGTATATCACTTCCGGCGAAGGAAATGGAAGAATCTATGGGCTGGGACCGGAAGC
TGGGGTCTGGTTTTGAG / Celera SNP ID: hCV31840134 / Public SNP ID: rs11894953 /
SNP Chromosome Position: 73964631 / SNP in Genomic Sequence: SEQ ID NO: 441 /
SNP Position Genomic: 80612 / Related Interrogated SNP: hCV11541681 / SNP
Source: dbSNP / Population(Allele,Count): Caucasian (C,80|T,146) / SNP Type:
INTERGENIC;UNKNOWN /
Context (SEQ ID NO: 2532): /
TCTACTGTGACTTCTGCATTTTTGTAGCTTTCCTGATAAAAGCCTGGGCTTTCCTTGTCAGACCTGAAAGATTCATTTATTCA
TTCAACAAACAGTTCCC
R
AGGACCTGAGGTGTGCCGGGCCTGGACTTGGCATGAAGGCACCAGATGTTGGAAGCGAGGCTGCCGCCCAGGAGGACACACCT
GATGGGGCTCTGGGAAC / Celera SNP ID: hCV31840149 / Public SNP ID: rs12233112 /
SNP Chromosome Position: 73931764 / SNP in Genomic Sequence: SEQ ID NO: 441 /
SNP Position Genomic: 47745 / Related Interrogated SNP: hCV11541681 / SNP
Source: dbSNP; HapMap / Population(Allele,Count): Caucasian (G,40|A,80) / SNP
Type: INTERGENIC;UNKNOWN /
Context (SEQ ID NO: 2533): /
AAAAAAAAAAAAAAACAAAAAACAACTATTATCCCAAGGGCCTGGGTAGTCAGGAAATCACAGAATTTTAAAGCTGCAAGGAAC
TTATGCAAATAGTAAAA
W

EP 2 635 709 B1

TTTTTTAAATAACATTAATTGAGGGCTAATATTATGGGCATGATCATATTGCAAACATTTTACTTGTAATATTTTCTTTAATC
CTCAAAACTACCCTATG / Celera SNP ID: hCV31840136 / Public SNP ID: rs12713795 /
SNP Chromosome Position: 73959935 / SNP in Genomic Sequence: SEQ ID NO: 441 /
SNP Position Genomic: 75916 / Related Interrogated SNP: hCV11541681 / SNP
Source: dbSNP; HapMap / Population(Allele,Count): Caucasian (T,38|A,82) / SNP
Type: INTRON /
Context (SEQ ID NO: 2534): /
ATAAACAGTGCAATGCACCTCATTTGGAAAAACAAACTGGTATCTAAAAGGATATAAATTCAATGTTAAGTGCAGACTCACA
GAGGGCCTAGATGGCTG
Y

CTGGTCTTTCCTGAGTCTTTGAAGCTTCTATTTTCAAAAGCTCTGTACTCCACAACTCATCATGGAACAGAAAAAATACGTCCA
AATTGTGAAAAAATACT / Celera SNP ID: hCV31840166 / Public SNP ID: rs4513320 /
SNP Chromosome Position: 73920741 / SNP in Genomic Sequence: SEQ ID NO: 441 /
SNP Position Genomic: 36722 / Related Interrogated SNP: hCV11541681 / SNP
Source: dbSNP; HapMap / Population(Allele,Count): Caucasian (T,39|C,79) / SNP
Type: TRANSCRIPTION FACTOR BINDING SITE;INTRON /
Context (SEQ ID NO: 2535): /
AGATCCCTCAGAGTTAAAAAGGCTATTTCTGTGGTTGGGGTTTCAAATCAAATTCAAGAGGTTCCCATATCTGAACCCGTCCA
ATTGACTTTGGGGCACT
Y

TTCAGAAAATCACACTTTTTTACTGTATGATAGTGCTCCAGTAAATTTGCTAGAGGGAGATTTACTTTCAAAGCTGAAAGGGC
ATAGAAGACTACCTATT / Celera SNP ID: hDV69785784 / Public SNP ID: rs13000788 /
SNP Chromosome Position: 73918312 / SNP in Genomic Sequence: SEQ ID NO: 441 /
SNP Position Genomic: 34293 / Related Interrogated SNP: hCV11541681 / SNP
Source: dbSNP; HapMap / Population(Allele,Count): Caucasian (C,39|T,79) / SNP
Type: INTRON /
Context (SEQ ID NO: 2536): /
CTGCCCAACTTGCAAAATATTTATTTTTCTTGCTCAATTACTCTGTGAAATTTAATTTGTCTAATGGATTTCTTTTAACAGAA
CTTATAATTAAAATGGA
R

AGCGGGTGTAAATATTTATAAAACTTGCAGCCTGGCCATGTGGTAGACAAGGAGGAATCCAAGCAGCCTGCTGAGCAACTACT
TGCTGGAGACATTAGCA / Celera SNP ID: hCV31840159 / Public SNP ID: rs13013228 /
SNP Chromosome Position: 73925216 / SNP in Genomic Sequence: SEQ ID NO: 441 /
SNP Position Genomic: 41197 / Related Interrogated SNP: hCV11541681 / SNP
Source: dbSNP; HapMap / Population(Allele,Count): Caucasian (G,40|A,80) / SNP
Type: INTRON /
Context (SEQ ID NO: 2537): /
TGAAACTCCCTCTCAAAAAAAAATAAATAAATAAAAATTAAAATTAAAAAAAAATATATATATATATATATACATATATATATG
AAGGACATTCTATTTCT
R

GGAGACACTGGAACAGGGCCCTGGCTGGTCTGGTTAGTCTCTCTCTATCACAGGATGCTACATTCCCAGCACATTCTACAGTT
ATCCTAGAGAACCACAA / Celera SNP ID: hCV31840132 / Public SNP ID: rs2421676 /
SNP Chromosome Position: 73966087 / SNP in Genomic Sequence: SEQ ID NO: 441 /
SNP Position Genomic: 82068 / Related Interrogated SNP: hCV11541681 / SNP
Source: dbSNP; HapMap; ABI_Val / Population(Allele,Count): Caucasian
(G,80|A,146) / SNP Type: INTERGENIC;UNKNOWN /
Context (SEQ ID NO: 2538): /
CTCCCAAAGTTCTGGGATTACAGGCGTGAGCCACCATGCCTGGCCTTAACTGTTTCAATTAATAACCTGATCAATACCAAAAT
ATAGAAAACCAGTCCAA
R

TGATGCCTGCAAAAGATATATTGTGCTTTCAGGCATCATGCACTCAAGTTACGGGAATTACCTATGTGAGTACAAGTAATTGC
TTGTATAATATCACCAG / Celera SNP ID: hDV70942181 / Public SNP ID: rs17350056 /
SNP Chromosome Position: 73922877 / SNP in Genomic Sequence: SEQ ID NO: 441 /
SNP Position Genomic: 38858 / Related Interrogated SNP: hCV11541681 / SNP
Source: dbSNP; HapMap / Population(Allele,Count): Caucasian (G,40|A,80) / SNP
Type: INTRON /
Context (SEQ ID NO: 2539): /
CTGTTTCAATTAATAACCTGATCAATACCAAAATATAGAAAACCAGTCCAAGTGATGCCTGCAAAAGATATATTGTGCTTTCA
GGCATCATGCACTCAAG
W

TACGGGAATTACCTATGTGAGTACAAGTAATTGCTTGTATAATATCACCAGATTAAATTCAACAGGGTCTCTTTTTTACTAAAT
GTGTTATACACCCTTAC / Celera SNP ID: hDV70953030 / Public SNP ID: rs17434634 /
SNP Chromosome Position: 73922926 / SNP in Genomic Sequence: SEQ ID NO: 441 /

462

SNP Position Genomic: 38907 / Related Interrogated SNP: hCV11541681 / SNP Source: dbSNP; HapMap / Population(Allele,Count): Caucasian (T,40|A,80) / SNP Type: INTRON /
Context (SEQ ID NO: 2540): /
CAGGGTCTCTTTTTACTAAATGTGTTATACACCCTTACAACATACTACAGGGCGAGCACAAAAAGGTCAATTTCCCAGTGGAT TTTGTTCAGGAGCTGTG
Y
AGGTTTATTAATGAACAAATCTAACTGACCCCTGCTCAAATGCAACTATGTGGCCCTGTTTTTCAACTCCCAAGGGCCTATAC TGGGTCTGTGGATAATC / Celera SNP ID: hDV70953035 / Public SNP ID: rs17434655 / SNP Chromosome Position: 73923089 / SNP in Genomic Sequence: SEQ ID NO: 441 / SNP Position Genomic: 39070 / Related Interrogated SNP: hCV11541681 / SNP Source: dbSNP; HapMap / Population(Allele,Count): Caucasian (T,86|C,140) / SNP Type: INTRON /
Context (SEQ ID NO: 2541): /
GTGTGTGTGTGTGTGTGTGTGTGTGCGCGCTTCACTCTGGCTGCAGGTGGACAATGGATCTGAGGAGGTGGGAATGGAAGC AGCTACCCCTTAAGGCT
M
GTGCCGCATCCAGGCAACAGCAGGGCCCCCAGTGCAGGGATGCAGGTGCTCACTGTACAAGAGCAGCTGGCCCAGAGGGGCTG GTAGGAGCTAAAATCGG / Celera SNP ID: hCV31840152 / Public SNP ID: rs12998980 / SNP Chromosome Position: 73930726 / SNP in Genomic Sequence: SEQ ID NO: 441 / SNP Position Genomic: 46707 / Related Interrogated SNP: hCV11541681 / SNP Source: dbSNP / Population(Allele,Count): Caucasian (A,40|C,80) / SNP Type: INTERGENIC;UNKNOWN /
Context (SEQ ID NO: 2542): /
GTCAAAACCCCACAAAAACAGTGGGATATAGTCTTTCATTGGTACATGGAAGCCTCTAAAAGACAAAATGAATGAAAACTTGC CTCCTTAAAAGATTCTT
Y
GCAAAAGCAAGTGAACAGCTTAAGTAACAGGCTAAGGACATGATGAAAGAGAACTGTACTCTGACTGAACTAACCCCAACTGA TTCTTCTCTTTATGCAT / Celera SNP ID: hDV77051911 / Public SNP ID: rs4852972 / SNP Chromosome Position: 73916672 / SNP in Genomic Sequence: SEQ ID NO: 441 / SNP Position Genomic: 32653 / Related Interrogated SNP: hCV11541681 / SNP Source: CDX; dbSNP / Population(Allele,Count): Caucasian (C,84|T,138) / SNP Type: INTRON /
Context (SEQ ID NO: 2543): /
TTGTTGATTTGTATTTAGTAGGCTCTGCAAGCAGGGATCTTTGGCTCTTCGGTTCACTCATGTATGTTAAGTGCCCAGAACAA GCCTGACAGATATTAGG
Y
GCTCGGTAAGTGCGTGTTGAGTGAGTAAACAACAGACAGCCAGTTTGGGTATTCCCAGTAGGGCAGGAACAGAGAAGAGACAC GTTTTATTGGGAAGCTA / Celera SNP ID: hDV77051912 / Public SNP ID: rs4852976 / SNP Chromosome Position: 73936322 / SNP in Genomic Sequence: SEQ ID NO: 441 / SNP Position Genomic: 52303 / Related Interrogated SNP: hCV11541681 / SNP Source: CDX; dbSNP / Population(Allele,Count): Caucasian (T,83|C,143) / SNP Type: INTERGENIC;UNKNOWN //

Gene Number: 105 / Gene Symbol: KLF3 - 51274 / Gene Name: Kruppel-like factor 3 (basic) / Chromosome: 4 / OMIM NUMBER: / OMIM Information: // Genomic Sequence (SEQ ID NO: 442): // / SNP Information /
Context (SEQ ID NO: 2544): /
CTTACCTCAAAAAAAACCTTCCCAGGTGTTTTCAAAGGAATTATTTTATTCCTCCACAACAAGCCTGTGGGAGTCGGAGCAAA AGGCAAAGTGATTACCT
R
AGACATTAGATAACTCGCCAATATTCACCCTGCTAACAACTGAGGGGCCTTGGGCTTGATCTCCTGCTTCCGAATCAGGGCTT TTCCTGCCATCTTGTCA / Celera SNP ID: hCV27502514 / Public SNP ID: rs3796533 / SNP Chromosome Position: 38692806 / SNP in Genomic Sequence: SEQ ID NO: 442 / SNP Position Genomic: 37016 / SNP Source: dbSNP; HapMap; ABI_Val; HGBASE / Population(Allele,Count): Caucasian (G,198|A,28) / SNP Type: MICRORNA;UTR3;INTRON //

Gene Number: 106 / Gene Symbol: DARS2 - 55157 / Gene Name: aspartyl-tRNA synthetase 2, mitochondrial / Chromosome: 1 / OMIM NUMBER: / OMIM Information: // Genomic Sequence (SEQ ID NO: 443): // / SNP Information /
Context (SEQ ID NO: 2545): /
TTTTAGTTTTGAAAGACCCAAGTGTCATTCTCAGTAGACATTTTGAAAGACCCAAATGTCATTCTCAGTAGGTACATCTCAGT

AACATCTGTTGAAGGGT
R
TAACAACGAAGCTTGATCCACCAGTGGACTGAAATGATACTGGGCTTACTAAGACTGAAGAGTTCCTATAACCAGTAATTAAA
AATGGGCCAGCCTCCTA / Celera SNP ID: hCV15864094 / Public SNP ID: rs2068871 /
SNP Chromosome Position: 173796169 / SNP in Genomic Sequence: SEQ ID NO: 443 /
SNP Position Genomic: 12372 / Related Interrogated SNP: hCV16180170 /
Related Interrogated SNP: hCV8911768 / SNP Source: dbSNP; HapMap; ABI_Val;
HGBASE / Population(Allele,Count): Caucasian (A,205|G,21) / SNP Type: INTRON
/
Context (SEQ ID NO: 2546): /
TAGTGAAATTAGTCATATAACCATGAAAAAATATTTTCAGTGTCAAGATTTCACAAATGAAGAAAATTAGGAGTCTCAAATTT
TTATACATCATGCATTC
R
TAAATTGTGGAGGGACGGATCAGACCACTGGCGAGCTGGAGGGCTGGCAGACACCCCTGGACCAACTTGGTCAAAGTGGGACC
CTTTTTCCTCAAGAACA / Celera SNP ID: hDV70683090 / Public SNP ID: rs16846433 /
SNP Chromosome Position: 173820593 / SNP in Genomic Sequence: SEQ ID NO: 443 /
SNP Position Genomic: 36796 / Related Interrogated SNP: hCV16180170 /
Related Interrogated SNP: hCV8911768 / SNP Source: dbSNP; HapMap /
Population(Allele,Count): Caucasian (G,205|A,21) / SNP Type: INTRON //

Gene Number: 107 / Gene Symbol: UQCC - 55245 / Gene Name:
ubiquinol-cytochrome c reductase complex chaperone / Chromosome: 20 / OMIM
NUMBER: / OMIM Information: // Genomic Sequence (SEQ ID NO: 444): // / SNP
Information /
Context (SEQ ID NO: 2547): /
ATGGAGTGTGGGCTGACACAGACCGACCACACATTGTAATCACATCCACTTGCATAAACTCTTCACCCAAACGCCGATGTAGA
GACCGGCCATGACCCCC
M
CAACACATACCACAGTGGATAGCACGTGCCCTGGCTCAAGTCTGCAAGGCCCTGAGAGCTGAACGACTGTCACATGGAATATC
CAAGGACATCAACGCTG / Celera SNP ID: hCV16013698 / Public SNP ID: rs2425052 /
SNP Chromosome Position: 33880729 / SNP in Genomic Sequence: SEQ ID NO: 444 /
SNP Position Genomic: 360 / Related Interrogated SNP: hCV1825046 / SNP
Source: dbSNP; HGBASE / Population(Allele,Count): Caucasian (A,134|C,92) /
SNP Type: INTERGENIC;UNKNOWN /
Context (SEQ ID NO: 2548): /
TAAACATTACCCAAAGACCCAGGATCACTTTATTCGACATTTTATTGTGGTCTGTCCTCTTGGGCATTGTGGTATGTGAGGTC
TGGCTCCAGGCCTGCTG
Y
CAACAAGGGCCATGTCCAGTATGGGGTGCTGACAGTTTGGTAGAAAAACATTCCACCTGTTCCAAATGCTCTCTGATAAACCC
TCCTTACGGTTCCACAG / Celera SNP ID: hCV29711285 / Public SNP ID: rs6058227 /
SNP Chromosome Position: 33895947 / SNP in Genomic Sequence: SEQ ID NO: 444 /
SNP Position Genomic: 15578 / SNP Source: dbSNP; HapMap /
Population(Allele,Count): Caucasian (C,212|T,14) / SNP Type: INTRON /
Context (SEQ ID NO: 2549): /
GCGGACACACTGCAAGGTCAGGAGGCAAACAGGGCAGGTAGATTTCTGACCTCTGCCCCAACAAAGATTAAAGTCCTGGAGCA
TGTACCGTGTGTCCTTC
R
GAGGATGGTGAGCAGCTTTGCTCTGGGGAAGGGGGTGAGCAGGTGACAGCTGCAGCTGGGGAGCCAGGCTAGGCAGAAGCGGT
CACCACCTTTCCACGGA / Celera SNP ID: hCV29674974 / Public SNP ID: rs6058224 /
SNP Chromosome Position: 33881774 / SNP in Genomic Sequence: SEQ ID NO: 444 /
SNP Position Genomic: 1405 / Related Interrogated SNP: hCV1825046 / SNP
Source: dbSNP; HapMap / Population(Allele,Count): Caucasian (G,178|A,48) /
SNP Type: INTERGENIC;UNKNOWN /
Context (SEQ ID NO: 2550): /
AAGCGATGCTCTTGCCTCCGCCTCCCAAAGTGTTGAGATTACAGGTGTGAGCCACTGCACCAAGCCAAAATCTGCTATTGTAA
CCATTTTTAAGTGGTAC
R
CTTTAAGTGTCGTAAGTGCATTCACACTGTGGTGTAACCCTCACCACCATCCATCTCTAGAACTCTTCTCACTTTGCAAAACT
AGAGCTCTGTACCCACT / Celera SNP ID: hCV32066659 / Public SNP ID: rs7272884 /
SNP Chromosome Position: 33883015 / SNP in Genomic Sequence: SEQ ID NO: 444 /
SNP Position Genomic: 2646 / Related Interrogated SNP: hCV1825046 / SNP
Source: dbSNP / Population(Allele,Count): Caucasian (G,178|A,48) / SNP Type:
INTERGENIC;UNKNOWN //

464

Gene Number: 108 / Gene Symbol: CPPED1 - 55313 / Gene Name: calcineurin-like phosphoesterase domain containing 1 / Chromosome: 16 / OMIM NUMBER: / OMIM Information: // Genomic Sequence (SEQ ID NO: 445): // / SNP Information /
Context (SEQ ID NO: 2551): /
TTGGCTGTCCCTGCTTCTCCAGGCAAGCTTTTGGAATATTCTGAATAAGAGCTGGAGAGTCAAAGAAAGAGAAAACAAAGCCC CTTTCTATCTCCCATAC
K
TTTCCTTCCTGCAGGAAAAGGGACACAGTACCATGCAGTTGAACACCATGTCAAGTGAGGATAAAGGAAAACAGCCATGACAT CACTCCATTACTGCCAC / Celera SNP ID: hCV1396435 / Public SNP ID: rs7193741 /
SNP Chromosome Position: 12851942 / SNP in Genomic Sequence: SEQ ID NO: 445 /
SNP Position Genomic: 108286 / SNP Source: dbSNP; Celera /
Population(Allele,Count): Caucasian (T,74|G,152) / SNP Type: INTRON //

Gene Number: 109 / Gene Symbol: STAB2 - 55576 / Gene Name: stabilin 2 /
Chromosome: 12 / OMIM NUMBER: / OMIM Information: // Genomic Sequence (SEQ ID NO: 446): // / SNP Information /
Context (SEQ ID NO: 2552): /
AAACTTATTCTACCCTCTTAGGGTCTGTGGCTGGGCCTGAGAATTAAATAGACATACGATAAATAGGAGAGAAGCATACACAT TTTATTTAATCTTTTAT
K
TGAATCAAGGGAACCCTCATATGGAGAATAGAGACCCAAAGAACAGTTGGGATCAAGAGCTTATTTACTTTTTTAAAGAAATGA TACATTTGTGGAAAATT / Celera SNP ID: hCV3188431 / Public SNP ID: rs12229292 /
SNP Chromosome Position: 104153633 / SNP in Genomic Sequence: SEQ ID NO: 446 /
SNP Position Genomic: 182564 / SNP Source: dbSNP; Celera; HapMap /
Population(Allele,Count): Caucasian (G,90|T,30) / SNP Type: INTRON /
Context (SEQ ID NO: 2553): /
TGTGAAGCATTTAGACCTGTGGTCTGTACATGGAAAATTCTATGTTCTTGTGAGCTGGTATTATCATCTCTGTTTGTATGTGG AACAGTTTAAGAGCATG
R
TCTACACAAATGAGACACTCTTTAATCCTCTTCATGGATGTAGTTAGCTAAGCCTGCCTGGTGATCACCCAGTGAGCCAAATT AACATGGAGTCAATTTG / Celera SNP ID: hCV3188439 / Public SNP ID: rs4981022 /
SNP Chromosome Position: 104149874 / SNP in Genomic Sequence: SEQ ID NO: 446 /
SNP Position Genomic: 178805 / SNP Source: dbSNP; Celera; HapMap; HGBASE /
Population(Allele,Count): Caucasian (G,79|A,147) / SNP Type: INTRON //

Gene Number: 110 / Gene Symbol: NLRP2 - 55655 / Gene Name: NLR family, pyrin domain containing 2 / Chromosome: 19 / OMIM NUMBER: / OMIM Information: // Genomic Sequence (SEQ ID NO: 447): // / SNP Information /
Context (SEQ ID NO: 2554): /
CAGTAAGACCTGGGAAGCTGAAACACGATCGCGTTTGTTGGAAATCTATAAATACATACAAAGCGGGGAAGGGTAAGCTTGGC CTTTGAATCTGGATAAG
S
TAGAGACTTTTCTTTTTTGAGATGGAGGCTTGCTCTGTCACCTAGGCTGAAGTGCAGTGGTACGACCTCGGCTGACTGCAACC TCTACCTCCTGGGTTCA / Celera SNP ID: hCV8704962 / Public SNP ID: rs775893 /
SNP Chromosome Position: 55486167 / SNP in Genomic Sequence: SEQ ID NO: 447 /
SNP Position Genomic: 19515 / Related Interrogated SNP: hCV8717873 / Related Interrogated SNP: hCV1376266 / Related Interrogated SNP: hCV1376342 / SNP Source: dbSNP; HGBASE / Population(Allele,Count): Caucasian (G,60|C,166) /
SNP Type: INTRON //

Gene Number: 111 / Gene Symbol: IWS1 - 55677 / Gene Name: IWS1 homolog (S. cerevisiae) / Chromosome: 2 / OMIM NUMBER: / OMIM Information: // Genomic Sequence (SEQ ID NO: 448): // / SNP Information /
Context (SEQ ID NO: 2555): /
CAAAGCATCTTAATTGTAGTTTAAGGGTTAAGTATCAAAAAAAGGGCATCCAATAAACTCAGTGGGAGGAAAAAAAGGTGAAC CTAGAAGCGACCCAGCT
Y
ACCTCAGCAGCTTCATTCATCTTGACGATCATGGCACTCACGACGTCGTCTGCATCACTAATAAAGGTGCCACCATCGCGGTT CCGTCTGCGCTTGCCAC / Celera SNP ID: hCV1023653 / Public SNP ID: rs334151 /
SNP Chromosome Position: 128253571 / SNP in Genomic Sequence: SEQ ID NO: 448 /
SNP Position Genomic: 58780 / Related Interrogated SNP: hCV1841983 / SNP

Source: Applera / Population(Allele,Count): Caucasian (C,36|T,4) African American (C,35|T,3) total (C,71|T,7) / SNP Type: DONOR SPLICE SITE;INTRON / SNP Source: dbSNP; HapMap; ABI_Val; HGBASE / Population(Allele,Count): Caucasian (C,187|T,39) / SNP Type: DONOR SPLICE SITE;INTRON /
Context (SEQ ID NO: 2556): /
ATATTTAAGGTGGTATCTAAAATTCCTATATCTAGAGCCCTAAGTCTATTACTATTGTCATCATTACTGTTACCTTTTAATGT
TATTTTTGTGTATAATA
M
GTACATATGAAAAACTTAAACCACTGTAATCTTATTTCTGAGACTGAGATTCTCTAGTCTGCCAGAAGATTCAAAGATGGCCT
AAAATCTGAGCAAAGGC / Celera SNP ID: hCV822512 / Public SNP ID: rs334144 / SNP
Chromosome Position: 128259657 / SNP in Genomic Sequence: SEQ ID NO: 448 /
SNP Position Genomic: 64866 / Related Interrogated SNP: hCV1841983 / SNP
Source: dbSNP; HapMap; HGBASE / Population(Allele,Count): Caucasian
(A,18|C,102) / SNP Type: MISSENSE MUTATION;ESS SYNONYMOUS;INTRON /
Context (SEQ ID NO: 2557): /
TTACTCTCTAGGATACTGATAAAGGAAGGGACTTCCATGTCAATTTCTAAATATTCAAGAGGCCTTGAATATCCCAAGGTGCC
TGTTAGGGTCATTATTT
Y
GAAACTAAAAGCAGACCTGGCCTCTTCTGATTAAGAAAGCACACACACACACACTTTTCACTCTGACTGTTGACAATTCAAGC
CCATGCACAGTACATGC / Celera SNP ID: hCV1023645 / Public SNP ID: rs334160 /
SNP Chromosome Position: 128243335 / SNP in Genomic Sequence: SEQ ID NO: 448 /
SNP Position Genomic: 48544 / Related Interrogated SNP: hCV1841983 / SNP
Source: dbSNP; HapMap; ABI_Val; HGBASE / Population(Allele,Count): Caucasian
(T,39|C,187) / SNP Type: INTRON /
Context (SEQ ID NO: 2558): /
CTGATTAAGAAAGCACACACACACACACTTTTCACTCTGACTGTTGACAATTCAAGCCCATGCACAGTACATGCTTCCTTGGG
AATACCTCTCGTGTATA
Y
ACTCTGGTAGGGCCAGGCCTATAGGGGCATCCTGTTGCCTTCAATGCTGAACTTGACAGCACGATGCACTTTGCTGAGTCGTT
TCTGTTCTGCAAATCGC / Celera SNP ID: hCV1023646 / Public SNP ID: rs334159 /
SNP Chromosome Position: 128243462 / SNP in Genomic Sequence: SEQ ID NO: 448 /
SNP Position Genomic: 48671 / Related Interrogated SNP: hCV1841983 / SNP
Source: dbSNP; HapMap; HGBASE / Population(Allele,Count): Caucasian
(T,17|C,101) / SNP Type: ESS;ESS SYNONYMOUS;SILENT RARE CODON;SILENT
MUTATION;INTRON /
Context (SEQ ID NO: 2559): /
AGATACCACAACAATTTTCTTTTGGTCCATGTTTACACAAAGTCCCCTTTCATACACCTTTATTTTCAGCTTTACCTTCTGTT
TCAGACAAATCTCTTGA
M
ATCAGTATATGGCTGGTTTTTAAGATCCTGTCTGGCAATTGTTGTCCTTTAACTGGAATATTTACTATTTACATTTAATTTAA
TTTCTGATATTATCAAA / Celera SNP ID: hCV1023659 / Public SNP ID: rs334146 /
SNP Chromosome Position: 128258208 / SNP in Genomic Sequence: SEQ ID NO: 448 /
SNP Position Genomic: 63417 / Related Interrogated SNP: hCV1841983 / SNP
Source: dbSNP; HapMap; ABI_Val; HGBASE / Population(Allele,Count): Caucasian
(C,18|A,102) / SNP Type: INTRON /
Context (SEQ ID NO: 2560): /
ACCATTAATAATAAAACATTAAATTTTTTCTGTGATTTTTTTAAAATACAGGATTATGTGCACCTAAATGGAGAATTCTTGTC
CTATACCCATATAGAAA
Y
ACCCCCACAGAAAGCCAATCAGTGAAATGGTTTTATTTGGATCCCCAAATGGGGAAATATAACCAGCCTTCATCAAAACAGAT
TTGTAAAGCATGCCCTC / Celera SNP ID: hCV1023661 / Public SNP ID: rs334143 /
SNP Chromosome Position: 128263476 / SNP in Genomic Sequence: SEQ ID NO: 448 /
SNP Position Genomic: 68685 / Related Interrogated SNP: hCV1841983 / SNP
Source: dbSNP; HapMap; HGBASE / Population(Allele,Count): Caucasian
(C,17|T,97) / SNP Type: INTRON /
Context (SEQ ID NO: 2561): /
TCACACACCCCTTGCCATTCCATACCTGTCTTGCCTGCAGAGGCCGGTAGCAAAAACCAAGTGCAGCCTGCCAGGCCGAGTGG
GCAGGGTGAGTCCAGGC
R
AATCCAAGTGAGCCCAGAGCCCAGCAAGGCCCAAGCAGGGGCACTGCTGACCCCAGAGGTTTCCTGCCAACCGGAAGTGGCAC
TCTCCAAAAAAATCCTG / Celera SNP ID: hCV1023665 / Public SNP ID: rs334138 /
SNP Chromosome Position: 128272468 / SNP in Genomic Sequence: SEQ ID NO: 448 /
SNP Position Genomic: 77677 / Related Interrogated SNP: hCV1841983 / SNP

Source: dbSNP; HapMap; HGBASE / Population(Allele,Count): Caucasian (A,17|G,101) / SNP Type: ESE;SILENT MUTATION;INTRON / Context (SEQ ID NO: 2562): / CAAGTAACATGTTCAATTCTCCATGTCACTAAATATTCTTTGAAAGCAATTCCAATAGCTGCATTGCCATTTTTATAAATGTT CTATTTACAGTTTAATC R

CTCATTTTCTACATTGTTGAGTATTTGTTTCCTTTCTTATGTTTATTACACCTCATTTGTCCATTTCAGTGTTTTCAACCCAA GGGCGGCTCTGGGTCTT / Celera SNP ID: hCV1023666 / Public SNP ID: rs334137 / SNP Chromosome Position: 128272674 / SNP in Genomic Sequence: SEQ ID NO: 448 / SNP Position Genomic: 77883 / Related Interrogated SNP: hCV1841983 / SNP Source: dbSNP; HapMap; HGBASE / Population(Allele,Count): Caucasian (A,40|G,184) / SNP Type: INTRON / Context (SEQ ID NO: 2563): / TGCTGGAAATTATACAGTTAAGCTTAAAAGTTATCAAAACAATATCCAAAGTTTTTAGAGTGGTTTTGGCAATTAAATTGCAA GCTTAAAAAAAATCCAA M

AGACAGTTTTTTTAAAAGTCAGATGAGTACAACAAACCAACAGTTGAAACTGACATTTAATGTCATTTCTAAAATAATGTAACA CATTTCCTAGTTGTAAT / Celera SNP ID: hCV3212726 / Public SNP ID: rs12621149 / SNP Chromosome Position: 128266844 / SNP in Genomic Sequence: SEQ ID NO: 448 / SNP Position Genomic: 72053 / Related Interrogated SNP: hCV1841983 / SNP Source: dbSNP; Celera; HapMap / Population(Allele,Count): Caucasian (A,46|C,178) / SNP Type: INTRON / Context (SEQ ID NO: 2564): / CCCCACAGGTTCCTCAGGTGGGTTCTGGGCTAGGCCCTGCCCTGCTGGGGGCTCCACAGGGCTTGGCCTAGATTGGACGAGTC GGGTGGGCAGCCCCTGG Y

CTCTCCACTCAGGAGACTCCCCACCCACAGGGCTGCAGTCCTGCCTCAGGGCTCAGCAAGCAAACACGTAAGAGCACTGCACA GTTGAAAAAATGAGTTT / Celera SNP ID: hCV8807993 / Public SNP ID: rs1473623 / SNP Chromosome Position: 128196220 / SNP in Genomic Sequence: SEQ ID NO: 448 / SNP Position Genomic: 1429 / Related Interrogated SNP: hCV1841983 / Related Interrogated SNP: hCV1841975 / SNP Source: dbSNP; HapMap; HGBASE / Population(Allele,Count): Caucasian (T,36|C,64) / SNP Type: INTRON / Context (SEQ ID NO: 2565): / ATAGTCATTTCTGTAGTTGGACTATGCTGGTTTTAATTTTTTTCTTTAAACTTCACAGATGCAGGAGTGCCTCAACTTATTAT GGGGTTACAGCCTGATA M

ACCCATAGTAAGCTGAAAATATAATTAAGCCAAAAACGCATTTCATACATCTAATCTACCAAACATCACAGCTTAGCCTAGCC TACCTTAGATGTGCTCC / Celera SNP ID: hCV8836422 / Public SNP ID: rs777554 / SNP Chromosome Position: 128279047 / SNP in Genomic Sequence: SEQ ID NO: 448 / SNP Position Genomic: 84256 / Related Interrogated SNP: hCV1841983 / SNP Source: dbSNP; HapMap; ABI_Val; HGBASE / Population(Allele,Count): Caucasian (C,40|A,186) / SNP Type: INTRON / Context (SEQ ID NO: 2566): / CTACATTGATTGACTTCCAGGAGAGCCTTCGAAACACCAGTGCTCACTGACCTAACCTTCTCTGCTGGTCTTACATAGAACTG GTCTGGGGAACTCACTC W

ATTTCCATGCTATCTCTAGCCAAAATACTTAACTTCTTGCACAAAAATCATAAAATAAAGAATTACAAATAAATCACTCCCCC TGCAAACTCAAAGATCA / Celera SNP ID: hCV8837014 / Public SNP ID: rs777569 / SNP Chromosome Position: 128229148 / SNP in Genomic Sequence: SEQ ID NO: 448 / SNP Position Genomic: 34357 / Related Interrogated SNP: hCV1841983 / SNP Source: dbSNP; HapMap; ABI_Val; HGBASE / Population(Allele,Count): Caucasian (A,18|T,102) / SNP Type: INTRON / Context (SEQ ID NO: 2567): / TGCACAGTTGAAAAAATGAGTTTTAAAACAATCTGCCAAGAAAAAAGATGTCAAAGTAGTCATCACGGGAAAAGTCAGAATTT TGGTGGACTTCACTGCA Y

TTATTCTGTGGCTATTAATTTTAAATTATGTATGTGGGGGAGGGGAGCACACCATAATCTTTCTAGTGCCCTGAGGCTCTTAA ACCAGGCTCTGTTCACC / Celera SNP ID: hCV11263777 / Public SNP ID: rs11683986 / SNP Chromosome Position: 128196398 / SNP in Genomic Sequence: SEQ ID NO: 448 / SNP Position Genomic: 1607 / Related Interrogated SNP: hCV1841983 / Related Interrogated SNP: hCV1841975 / SNP Source: dbSNP; Celera; HapMap / Population(Allele,Count): Caucasian (C,155|T,71) / SNP Type: INTRON / Context (SEQ ID NO: 2568): /

AACAATCTGCCAAGAAAAAAGATGTCAAAGTAGTCATCACGGGAAAAGTCAGAATTTTGGTGGACTTCACTGCACTTATTCTG
TGGCTATTAATTTTAAA
K
TATGTATGTGGGGGAGGGGAGCACACCATAATCTTTCTAGTGCCCTGAGGCTCTTAAACCAGGCTCTGTTCACCTGGACCTGT
GTTCATGGGCAGGAAAC / Celera SNP ID: hCV11263778 / Public SNP ID: rs6749002 /
SNP Chromosome Position: 128196424 / SNP in Genomic Sequence: SEQ ID NO: 448 /
SNP Position Genomic: 1633 / Related Interrogated SNP: hCV1841983 / SNP
Source: dbSNP; Celera; HapMap / Population(Allele,Count): Caucasian
(T,115|G,111) / SNP Type: TRANSCRIPTION FACTOR BINDING SITE;INTRON /
Context (SEQ ID NO: 2569): /
AGAGGGCTGGGATTACCGGTGTGAGCAACCATGCTGGACCCTCTAAGTGATCTTAAGAGATGCCTGCTCTTGCCTTCCCATGT
TAGCCCCCTAATGTAAC
Y
GGCTCAGAGATGGACACAGCGAATACAGACAGAATGGGGATGTGCCTCCAAATCTGCCTGACTCCAGGAGGGGTCCCTTTAGT
TGACATTGGCCCTGGAG / Celera SNP ID: hCV11263786 / Public SNP ID: rs13408910 /
SNP Chromosome Position: 128196930 / SNP in Genomic Sequence: SEQ ID NO: 448 /
SNP Position Genomic: 2139 / Related Interrogated SNP: hCV1841975 / Related
Interrogated SNP: hCV1841983 / SNP Source: dbSNP; Celera /
Population(Allele,Count): Caucasian (C,114|T,112) / SNP Type: INTRON /
Context (SEQ ID NO: 2570): /
CTGAATAGCATACTTAATTATATTGAGGTTTTTTTTTAAAGTCTAAGAGAAAATGCTAGTACCTGTAAGAAGTTATTCAAAAA
TTTCTATTCCACTTTAC
Y
GACTCGTTTAAAACAAATACTGGTAAAAATACAGCTTTTATATATACGCATCTCTCTCTTATCTATACACATAAACACAACAA
ACAAAAAGGAAAATTTA / Celera SNP ID: hCV12046142 / Public SNP ID: rs334152 /
SNP Chromosome Position: 128249441 / SNP in Genomic Sequence: SEQ ID NO: 448 /
SNP Position Genomic: 54650 / Related Interrogated SNP: hCV1841983 / SNP
Source: dbSNP; HapMap; HGBASE / Population(Allele,Count): Caucasian
(C,18|T,102) / SNP Type: INTRON /
Context (SEQ ID NO: 2571): /
ATTATTTTGTCCATAAAATTGCATTTCCATTTTTTTTCAAATAATTGCACCCCAGTGTGAGGGGATATTCTAGACATTTAGAAT
ATTCTTTCCTTGAAGAG
Y
CCATTATTTCAGAGTAATTTATAGGATAAACAAAGCATAATGCCAGTGTACTGACTGACTCTGGCTGATTCAGAAAATTTAAT
GTAAAAGATCACCACAG / Celera SNP ID: hCV12046143 / Public SNP ID: rs334156 /
SNP Chromosome Position: 128247640 / SNP in Genomic Sequence: SEQ ID NO: 448 /
SNP Position Genomic: 52849 / Related Interrogated SNP: hCV1841983 / SNP
Source: dbSNP; HapMap; HGBASE / Population(Allele,Count): Caucasian
(T,18|C,102) / SNP Type: INTRON /
Context (SEQ ID NO: 2572): /
TCTTCTCTCCCACTAAGTCACCAACATATGACAGCTTTGCCTCTGCAATGTCTTCTCACCCCTCTGTTTCCCAACTACCACCT
CTGTTTAGCTGTATCAC
Y
GCCTCAGTCCCACTGCCCTCCAATCCATCCTACATGATGCCATTGGCATCAGCACCCTAAATCAAATCAAACCAGATGGCTTC
ACATCCAGCCTCAAAAA / Celera SNP ID: hCV12046144 / Public SNP ID: rs334158 /
SNP Chromosome Position: 128246152 / SNP in Genomic Sequence: SEQ ID NO: 448 /
SNP Position Genomic: 51361 / Related Interrogated SNP: hCV1841983 / SNP
Source: dbSNP; HapMap; HGBASE / Population(Allele,Count): Caucasian
(T,40|C,186) / SNP Type: INTRON;PSEUDOGENE /
Context (SEQ ID NO: 2573): /
CTTCCCTATCTGTCCTACCACAAAAGGTGTGAAGCCATCTAACCCAAACTTAATTATTCAGCATTACCTTCCATTAAGCTATT
CCATTTTCTAGCCACAA
Y
AATCATTCCTGAAATATATCAAGTCCTTCTTAATGTTAGGCCCTGTACCTGTTGCTCTTCTAGCTAGACAGAATTACCACCAC
CCCTATCGCCACCCCTA / Celera SNP ID: hCV15917574 / Public SNP ID: rs2679409 /
SNP Chromosome Position: 128270109 / SNP in Genomic Sequence: SEQ ID NO: 448 /
SNP Position Genomic: 75318 / Related Interrogated SNP: hCV1841983 / SNP
Source: dbSNP; HapMap; ABI_val; HGBASE / Population(Allele,Count): Caucasian
(C,18|T,102) / SNP Type: INTRON /
Context (SEQ ID NO: 2574): /
CCCCCAAATATCCACAACTGGCTTGCTAAAAATCTCTTGTTCAGGAAAGCAACACAAGGCTGCTCTCTCTGGAGGTGTGGCCA
CAGGCAGGACCCGGAGG
W

CCTCACCCATACACACATAGAGGAAACTCCATGAGATGAAAGAGCTCAATGGGCCCAATACAGATTGCGCTAACAGACAAGAG
AAGCATAATAGGTCCCT / Celera SNP ID: hCV16241157 / Public SNP ID: rs2460106 /
SNP Chromosome Position: 128273004 / SNP in Genomic Sequence: SEQ ID NO: 448 /
SNP Position Genomic: 78213 / Related Interrogated SNP: hCV1841983 / SNP
Source: dbSNP; HapMap; ABI_Val; HGBASE / Population(Allele,Count): Caucasian
(T,18|A,102) / SNP Type: INTRON /
Context (SEQ ID NO: 2575): /
GCCTCCCTCCCCTTCTTCCTGAGCCCTTCATGCAGAGCCCAGGACGCAATGGTCACAATGGCTAAAATCTCTTTGGAGGAGGG
AGAGATCAGTGCAGGCT
R
AAGCCATTGGTGAGAGTGGAAGAATTCAGCTCTCCAGCGGAAGGCAGCAATATGCAGAGAGCCCTGTGATGGGGCTGCTGGAG
CCGAGGCCTGGGGGTTC / Celera SNP ID: hCV28952331 / Public SNP ID: rs6755028 /
SNP Chromosome Position: 128195442 / SNP in Genomic Sequence: SEQ ID NO: 448 /
SNP Position Genomic: 651 / Related Interrogated SNP: hCV1841983 / Related
Interrogated SNP: hCV1841975 / SNP Source: dbSNP; HapMap /
Population(Allele,Count): Caucasian (G,76|A,150) / SNP Type: INTRON /
Context (SEQ ID NO: 2576): /
CTGCAGAGAAGGTACCCAGGCCCTGGGCTCAGTGGCCCTTCCCCAGCTGGTGCCCCCAGCCTTACTGCCTGGCCCTCCTGCAC
CTTCTTTTCCTTGTCTA
R
GAACTGGGCTCATCCCCCTCTATCCCCAGTGGTTGTGGGGATGAAAGGAAGGCCAACCCCTGCAAGGCCTGGTGCCTGGAGCT
GGCCCTGGGTAGACGCC / Celera SNP ID: hCV28952333 / Public SNP ID: rs6754772 /
SNP Chromosome Position: 128195244 / SNP in Genomic Sequence: SEQ ID NO: 448 /
SNP Position Genomic: 453 / Related Interrogated SNP: hCV1841983 / SNP
Source: dbSNP; HapMap / Population(Allele,Count): Caucasian (G,108|A,112) /
SNP Type: INTRON /
Context (SEQ ID NO: 2577): /
GCAAAGAGCCCAGCACAGAGAAAATCCTTATCACATGCCAGCTCTTTCTGAGACTGAGAATTCAAAAATGTTCTAAAGAGTAG
TTCAGTCGCAGAGGTCT
R
TGCTGCCAACCTTGCTGTTTCATAGGGTTAATGGATAATTTGGCTGCTGTAGCCTAGATGGGAATATTAGGGCCACCATAAGC
AAGTGACTTCAGTTAGC / Celera SNP ID: hCV29404688 / Public SNP ID: rs7582598 /
SNP Chromosome Position: 128289856 / SNP in Genomic Sequence: SEQ ID NO: 448 /
SNP Position Genomic: 95065 / Related Interrogated SNP: hCV1841983 / SNP
Source: dbSNP; HapMap; ABI_Val / Population(Allele,Count): Caucasian
(G,38|A,188) / SNP Type: INTERGENIC;UNKNOWN /
Context (SEQ ID NO: 2578): /
CAGATCAAAAACATAACAGTCACTTGGAGGATAACCACAATGAGGGAGCCCTACCTATTAGGCACCTACTGGATGCTGGGCAT
CCTGCTGGGCACATCCA
W
CCCCATGGCCTCTCTGTGCACTGACATCTGACGTCTTTCTCAGGGAGAAGAAAACTGAAGCTCATGGGGGTGAAGTGACTTTT
GCCCTAGGCCAGATAGC / Celera SNP ID: hDV70929450 / Public SNP ID: rs17261845 /
SNP Chromosome Position: 128223757 / SNP in Genomic Sequence: SEQ ID NO: 448 /
SNP Position Genomic: 28966 / Related Interrogated SNP: hCV1841983 / SNP
Source: dbSNP; HapMap / Population(Allele,Count): Caucasian (T,104|A,16) /
SNP Type: INTRON /
Context (SEQ ID NO: 2579): /
ATTTTACCCACACACTGATAGAAGAAAATGCTTCAAGGAGAACTCATTTACCCAGATAACTTCCTTTAATTAGGAAATTTCAA
AAAATTCCCTGAATGGT
K
TAGATAATATGTTTGTCCAATCATGACTTCACAAGGGATCTGAAGCAACGTAGTTCTGGTCCAGAACTGTCAGTGCTGAGGAG
ATTCCCAGTGTCCACAG / Celera SNP ID: hDV70929452 / Public SNP ID: rs17261859 /
SNP Chromosome Position: 128224791 / SNP in Genomic Sequence: SEQ ID NO: 448 /
SNP Position Genomic: 30000 / Related Interrogated SNP: hCV1841983 / SNP
Source: dbSNP; HapMap / Population(Allele,Count): Caucasian (T,104|G,16) /
SNP Type: INTRON //

Gene Number: 112 / Gene Symbol: C1orf112 - 55732 / Gene Name: chromosome 1
open reading frame 112 / Chromosome: 1 / OMIM NUMBER: / OMIM Information: //
Genomic Sequence (SEQ ID NO: 449): // / SNP Information /
Context (SEQ ID NO: 2580): /
CACAAGAGGACTTGTAGGTGAATTCTCCAATAGGGGAATGAGCACACCTCACCAAACCCTTCGGGGGCTGGTGGACAGCATCG
CATCTCACAGCTGGAAC

R
CACGAGAGAGCACTTTAGAAGTTTGTTTGCATCTCCAGCAATACGTTTCCCAAGGTAACCAAGTTCCCAAGCTCTTCAATAGT
TCTTTTTATCTTAAAAT / Celera SNP ID: hCV11975325 / Public SNP ID: rs5367 / SNP
Chromosome Position: 169697076 / SNP in Genomic Sequence: SEQ ID NO: 449 /
SNP Position Genomic: 57474 / Related Interrogated SNP: hCV11975250 / SNP
Source: Applera / Population(Allele,Count): Caucasian (A,37|G,3) African
American (A,32|G,2) total (A,69|G,5) / SNP Type: ACCEPTOR SPLICE
SITE;INTRON;PSEUDOGENE / SNP Source: dbSNP; HapMap; ABI_Val; HGBASE /
Population(Allele,Count): Caucasian (A,199|G,27) / SNP Type: ACCEPTOR SPLICE
SITE;INTRON;PSEUDOGENE /
Context (SEQ ID NO: 2581): /
CTCCCATTAGTTCAAATCCTTCTTCACAGTCAAATGTACAGGTTGTGTTCCATGGGAAGCTTCCAGGGTTTTGGAAACATTCC
ACGAACCCATTGGCTGG

R
TTTGTCACAGCATCACACTCAACCACTGAGGATTTTAAAGAGCACCATGAATTTTACAGAAGAATGATCTTTTCACTTCCTAT
TGAGCTGGGTGCCTAAC / Celera SNP ID: hCV11975329 / Public SNP ID: rs5363 / SNP
Chromosome Position: 169698789 / SNP in Genomic Sequence: SEQ ID NO: 449 /
SNP Position Genomic: 55761 / Related Interrogated SNP: hCV11975250 / SNP
Source: Applera / Population(Allele,Count): Caucasian (A,34|G,0) African
American (A,34|G,2) total (A,68|G,2) / SNP Type: ESE;SILENT MUTATION;INTRON /
SNP Source: dbSNP; HapMap; HGBASE / Population(Allele,Count): Caucasian
(A,199|G,27) / SNP Type: ESE;SILENT MUTATION;INTRON /
Context (SEQ ID NO: 2582): /
TATGACACCATCTGCACCAGGGAAGGGAAGGCATGCAGACCTGACTCTAATGCCAGCTAGGACGTGAGATGGTGCTACCATCT
CAAGTGAAGAAAGAGGC

R
AGAACCAGACTTACTTTGCTCACACTTGAGTCCACTGAAGCCAGGGTCACACTTGCAAGTGTAATTATTGATGGTCTCTACAC
ATTCACCGTGGCCACTG / Celera SNP ID: hCV11975331 / Public SNP ID: rs5362 / SNP
Chromosome Position: 169700961 / SNP in Genomic Sequence: SEQ ID NO: 449 /
SNP Position Genomic: 53589 / Related Interrogated SNP: hCV11975250 / SNP
Source: Applera / Population(Allele,Count): Caucasian (A,20|G,4) African
American (A,11|G,5) total (A,31|G,9) / SNP Type: MISSENSE MUTATION;ESS;INTRON
/ SNP Source: dbSNP; HapMap; HGBASE / Population(Allele,Count): Caucasian
(A,199|G,27) / SNP Type: MISSENSE MUTATION;ESS;INTRON /
Context (SEQ ID NO: 2583): /
CAAGAACCAGACTTACTTTGCTCACACTTGAGTCCACTGAAGCCAGGGTCACACTTGCAAGTGTAATTATTGATGGTCTCTAC
ACATTCACCGTGGCCAC

K
GCAGGATGTATTGGTACAGGCAGCTACGGAAAAATACAAAGCATGATGAGGAGGACTATTACTGTGCTTATACTGAGTGCCTTT
GATTTTAGAATCAACAG / Celera SNP ID: hCV11975332 / Public SNP ID: rs5361 / SNP
Chromosome Position: 169701060 / SNP in Genomic Sequence: SEQ ID NO: 449 /
SNP Position Genomic: 53490 / Related Interrogated SNP: hCV11975250 / SNP
Source: Applera / Population(Allele,Count): Caucasian (G,2|T,34) African
American (G,0|T,32) total (G,2|T,66) / SNP Type: MISSENSE MUTATION;INTRON /
SNP Source: dbSNP; HapMap; HGBASE / Population(Allele,Count): Caucasian
(T,199|G,27) / SNP Type: MISSENSE MUTATION;INTRON /
Context (SEQ ID NO: 2584): /
CTCCTTATGTTTCTGAGTAATTGTATAAACATAGTTGTCTTCTCTTTTATATTGCAGATATGGGACTGCTGAACTGTGTGCAC
ACCATGTCACCATAGTG

K
CTCATCTGGTGCGTAGAATTACAAGGCCTAAAACACCCACTTTCTATGCACTTCCTTTGGGGATATGATAATCTTTAAGAATG
TGAATATCGGCGGGAGA / Celera SNP ID: hCV16177404 / Public SNP ID: rs2272920 /
SNP Chromosome Position: 169799880 / SNP in Genomic Sequence: SEQ ID NO: 449 /
SNP Position Genomic: 45330 / Related Interrogated SNP: hCV11975250 / SNP
Source: Applera / Population(Allele,Count): Caucasian (G,35|T,1) African
American (G,38|T,0) total (G,73|T,1) / SNP Type: MISSENSE MUTATION;ESS
SYNONYMOUS / SNP Source: dbSNP; HapMap; ABI_Val; HGBASE /
Population(Allele,Count): Caucasian (G,198|T,28) / SNP Type: MISSENSE
MUTATION;ESS SYNONYMOUS /
Context (SEQ ID NO: 2585): /
AGGATGCTGCCAGATATCCTGTGCAGGACAGCCCCAGACAAGCAAGGATATTTCAGTCTGAAATATCTATAGTGCGAGAATGA
GAAATCTTGGTCTAATG

R

CACTGACTTACCCAAAGTGAGAGCTGAGAGAAACTGTGAAGCAATCATGACTTCAAGAGTTCTTTTCACCCAAAGGTTTAGGC
TTGAAATACTTTCCTGG / Celera SNP ID: hCV2459459 / Public SNP ID: rs932307 /
SNP Chromosome Position: 169702705 / SNP in Genomic Sequence: SEQ ID NO: 449 /
SNP Position Genomic: 51845 / Related Interrogated SNP: hCV11975250 / SNP
Source: Applera / Population(Allele,Count): Caucasian (A,4|G,10) African
American (A,5|G,17) total (A,9|G,27) / SNP Type: INTRON;PSEUDOGENE / SNP
Source: dbSNP; Celera; HapMap; ABI_Val; HGBASE / Population(Allele,Count):
Caucasian (G,175|A,51) / SNP Type: INTRON;PSEUDOGENE /
Context (SEQ ID NO: 2586): /
TCACACTTGCAAGTGTAATTATTGATGGTCTCTACACATTCACCGTGGCCACTGCAGGATGTATTGGTACAGGCAGCTACGGA
AAATACAAAGCATGATG
R

GGAGGACTATTACTGTGCTTATACTGAGTGCCTTTGATTTTAGAATCAACAGTGTGCAACAGAGACATCAGCAGTCCTACAGA
GTGCCATAGACTTTAAC / Celera SNP ID: hCV25617131 / Public SNP ID: rs3917410 /
SNP Chromosome Position: 169701108 / SNP in Genomic Sequence: SEQ ID NO: 449 /
SNP Position Genomic: 53442 / Related Interrogated SNP: hCV11975250 / SNP
Source: Applera / Population(Allele,Count): Caucasian (A,38|G,2) African
American (A,36|G,2) total (A,74|G,4) / SNP Type: INTRON / SNP Source:
dbSNP; HapMap; HGBASE / Population(Allele,Count): Caucasian (A,189|G,27) / SNP
Type: INTRON /
Context (SEQ ID NO: 2587): /
GAACATTTTACCACTGCAAATGTTAGGTACACAGGCAGAGTTTCAGAAAAATCTACTGGAAAACTTCCAAAACTTGCTTAAAA
GTCAACAATGAATGTAA
W

GTGTAAGCGCTACTTAGTTTTTCAGCATGTAGGAAATTAGGACCAAACCCCTTTGGGGCAATCTAGGTTCAGAAACTTTATGAA
GTATTTGACCTGTACCC / Celera SNP ID: hCV25617143 / Public SNP ID: rs3917425 /
SNP Chromosome Position: 169696754 / SNP in Genomic Sequence: SEQ ID NO: 449 /
SNP Position Genomic: 57796 / Related Interrogated SNP: hCV11975250 / SNP
Source: Applera / Population(Allele,Count): Caucasian (A,29|T,1) African
American (A,34|T,2) total (A,63|T,3) / SNP Type: INTRON;PSEUDOGENE / SNP
Source: dbSNP; HapMap; HGBASE / Population(Allele,Count): Caucasian
(A,199|T,27) / SNP Type: INTRON;PSEUDOGENE /
Context (SEQ ID NO: 2588): /
TTCAGTTTCTTCATCTATATAAGAGGAATAATGAAATTGTGTTATCTTTATCAAATTGATATGGAAACTAAATGTAATTCAAT
TAGCATAAGTCAAGGAC
S

TTAGAACAAAGCCTGACTCATCAGAAATTCTAAGTAAACATTAGCTAGTCTTCATATTATTATCTTCAGCATTATCTGTAGTG
AGAATCCTTAAAGCCAA / Celera SNP ID: hCV25619707 / Public SNP ID: rs4987308 /
SNP Chromosome Position: 169674116 / SNP in Genomic Sequence: SEQ ID NO: 449 /
SNP Position Genomic: 80434 / Related Interrogated SNP: hCV11975250 / SNP
Source: Applera / Population(Allele,Count): Caucasian (C,38|G,2) African
American (C,36|G,2) total (C,74|G,4) / SNP Type: TRANSCRIPTION FACTOR BINDING
SITE;INTRON / SNP Source: dbSNP; HapMap / Population(Allele,Count): Caucasian
(C,109|G,11) / SNP Type: TRANSCRIPTION FACTOR BINDING SITE;INTRON /
Context (SEQ ID NO: 2589): /
TGCCTGCATCTTTGTTTCTCTTGATATAGATCTCCACGCAGTCCTCCTTGTTCTTCTTGTTGTTGGGCTCACCATCTCCCCAG
TTCTCTGCTTCTTCAGT
R

AGAGATTTGTTGGTTCCCACCCACGTCCATATTCCTCCTATCTTCCGGATTCCTATCCAGTAGTAAGAACGACTGAAAGGCAG
AGTCTTCTCCAGATACT / Celera SNP ID: hCV8919509 / Public SNP ID: rs1051091 /
SNP Chromosome Position: 169677709 / SNP in Genomic Sequence: SEQ ID NO: 449 /
SNP Position Genomic: 76841 / Related Interrogated SNP: hCV11975250 / SNP
Source: Applera / Population(Allele,Count): Caucasian (A,27|G,11) African
American (A,29|G,9) total (A,56|G,20) / SNP Type: ESE SYNONYMOUS;SILENT
MUTATION;INTRON / SNP Source: dbSNP; HapMap; HGBASE / Population(Allele,Count):
Caucasian (A,176|G,50) / SNP Type: ESE SYNONYMOUS;SILENT MUTATION;INTRON /
Context (SEQ ID NO: 2590): /
TCCACATGCCCACATCTTTTTCTCTCTTGATGTAGATCTCCACGCAGTCCTCATCTTTTTGCCTATTGTTGGGTTCACCTGGA
GCCCAGTTCTTGGCTTC
Y

TCTGTCAGAGGTTTCTGGGTTCCTACCCAGACCCACACATTGTTGACTTTTCTGATTCCAATCCAGTAATAACTTGGTGAATA
GCTCAATATGGAGTTTA / Celera SNP ID: hCV8919527 / Public SNP ID: rs1800016 /
SNP Chromosome Position: 169701901 / SNP in Genomic Sequence: SEQ ID NO: 449 /

SNP Position Genomic: 52649 / Related Interrogated SNP: hCV11975250 / SNP Source: Applera / Population(Allele,Count): Caucasian (C,0|T,32) African American (C,2|T,36) total (C,2|T,68) / SNP Type: TRANSCRIPTION FACTOR BINDING SITE;ESE SYNONYMOUS;SILENT MUTATION;INTRO / N // SNP Source: Applera / Population(Allele,Count): Caucasian (C,0|T,4) African American (C,2|T,30) total (C,2|T,34) / SNP Type: TRANSCRIPTION FACTOR BINDING SITE;ESE SYNONYMOUS;SILENT MUTATION;INTRO / N // SNP Source: dbSNP; HGBASE / Population(Allele,Count): Caucasian (T,198|C,28) / SNP Type: TRANSCRIPTION FACTOR BINDING SITE;ESE SYNONYMOUS;SILENT MUTATION;INTRO / N //
Context          (SEQ ID NO: 2591): /
ACATGCCCACATCTTTTTCTCTCTTGATGTAGATCTCCACGCAGTCCTCATCTTTTTGCCTATTGTTGGGTTCACCTGGAGCC
CAGTTCTTGGCTTCTTC
Y
GTCAGAGGTTTCTGGGTTCCTACCCAGACCCACACATTGTTGACTTTTCTGATTCCAATCCAGTAATAACTTGGTGAATAGCT
CAATATGGAGTTTAGGT / Celera SNP ID: hCV8919528 / Public SNP ID: rs1800015 /
SNP Chromosome Position: 169701904 / SNP in Genomic Sequence: SEQ ID NO: 449 /
SNP Position Genomic: 52646 / Related Interrogated SNP: hCV11975250 / SNP Source: Applera / Population(Allele,Count): Caucasian (C,0|T,2) African American (C,2|T,28) total (C,2|T,30) / SNP Type: ESE SYNONYMOUS;SILENT RARE CODON;SILENT MUTATION;INTRON / SNP Source: Applera / Population(Allele,Count): Caucasian (C,0|T,8) African American (C,2|T,28) total (C,2|T,36) / SNP Type: ESE SYNONYMOUS;SILENT RARE CODON;SILENT MUTATION;INTRON / SNP Source: dbSNP; HGBASE / Population(Allele,Count): Caucasian (T,199|C,27) / SNP Type: ESE SYNONYMOUS;SILENT RARE CODON;SILENT MUTATION;INTRON /
Context          (SEQ ID NO: 2592): /
TCCAATTTCTATATTGACAGACATACCTTCCTAATGAGCTGGGGTTCGAATTTAGAAATCTTTGATGCTTCAGAGTCCACACT
GAAATGTGGAGGCACAT
R
GTGAGTTGGTCCCCAGCCTTCAGTCCACCCACCTTCTCTTTACTAAATCACCTTTCACATACATGTATGAACACCCCAGCCTC
CAAGTCCAAACCCTAAA / Celera SNP ID: hCV2459402 / Public SNP ID: rs12045330 /
SNP Chromosome Position: 169648341 / SNP in Genomic Sequence: SEQ ID NO: 449 /
SNP Position Genomic: 106209 / Related Interrogated SNP: hCV11975250 / SNP Source: dbSNP; Celera; HapMap / Population(Allele,Count): Caucasian (A,97|G,23) / SNP Type: INTRON /
Context          (SEQ ID NO: 2593): /
CCCACCTTCTCTTTACTAAATCACCTTTCACATACATGTATGAACACCCCAGCCTCCAAGTCCAAACCCTAAACAAAATGGGA
CACCCTTGTGCATACAC
R
GAGACACAGCCCATCCTCAGGAAAACCTGGAAAAGTCCATACAAGTTCTGGAAGCAAGCTTGGGACGGTTTCAGTAGTGTGGT
CTATAAGGGAGGCCTCA / Celera SNP ID: hCV2459404 / Public SNP ID: rs6663862 /
SNP Chromosome Position: 169648469 / SNP in Genomic Sequence: SEQ ID NO: 449 /
SNP Position Genomic: 106081 / Related Interrogated SNP: hCV11975250 / SNP Source: dbSNP; Celera; HapMap / Population(Allele,Count): Caucasian (A,172|G,54) / SNP Type: INTRON /
Context          (SEQ ID NO: 2594): /
AACTCTATAGACACTGTCTAGCTATATGAACTTAGACAAACTAATATCTCTGAGCTTCAGTTTCTTAAAATTTAAAATGAGGA
CAATACCATCTATGGCC
R
GGGATTAAATGCTATGAGGAATGTAAACCAGATGTCAGGTACCATCTCTCTAAAATCCAGATAAAATGAATTAAAAATACTGG
CCGCAAACCCTCTCTAA / Celera SNP ID: hCV2459408 / Public SNP ID: rs7531806 /
SNP Chromosome Position: 169651044 / SNP in Genomic Sequence: SEQ ID NO: 449 /
SNP Position Genomic: 103506 / Related Interrogated SNP: hCV11975250 / SNP Source: dbSNP; Celera; HapMap / Population(Allele,Count): Caucasian (G,94|A,132) / SNP Type: INTRON /
Context          (SEQ ID NO: 2595): /
TGGTATTTTTGAGATTTGGCCTAAAACATCATTGCCCTGGTTTCCTTATTTACCAAACAGGGCCAATGGTAGTGACTAATCAG
AAAATGATAATGCCTGG
W
GCACAAAATGTGTCTAGATGAGCCCATGCACAAGGACACATGTTTCTGGAACTGTTCCTTATTCCTTTCCTAAAAGAAAGGAG
GGAAAGTCTCCATACTA / Celera SNP ID: hCV2459420 / Public SNP ID: rs4987351 /
SNP Chromosome Position: 169667355 / SNP in Genomic Sequence: SEQ ID NO: 449 /
SNP Position Genomic: 87195 / Related Interrogated SNP: hCV11975250 / SNP Source: dbSNP; Celera; HapMap / Population(Allele,Count): Caucasian

(T,112|A,110) / SNP Type: INTRON /
Context (SEQ ID NO: 2596): /
TTCCATGATGTGCCAGGAAATCTGCAAGACATCAGTGTGACCTATGCAGACTTACATAATGTTACAGCTAAAAAGAACCTAGC
ACTACTCCAGGCTGAGC
Y
AGACACTTAGAGATGAGGAAACAGAGCCTAAGAGTGTATGTGACCATCTCAGGATCACAGAATAGTTGTTTGCAGATTTGAAG
TAGAACCTAGACCTTCT / Celera SNP ID: hCV2459428 / Public SNP ID: rs4987285 /
SNP Chromosome Position: 169678024 / SNP in Genomic Sequence: SEQ ID NO: 449 /
SNP Position Genomic: 76526 / Related Interrogated SNP: hCV11975250 / SNP
Source: dbSNP; Celera / Population(Allele,Count): Caucasian (T,173|C,53) /
SNP Type: INTRON /
Context (SEQ ID NO: 2597): /
GTAGTTGTTCTCTCTTATTTCCCAGAGTTTTTCTGCCCCTTTAAAAGAACCTCTGCTGTTCTGATCCTTATCACATCTCTGTT
TTGACTGTTGGCTTTGT
Y
GTTGCCAGTGTTCAGCCAGAACTTCTCTGAAACTTTTTTTTTCAACACATGCTAAGTTAATGGAAGTGTAGGAGAGTTTTGATT
CTCACACTCCTCAAGGC / Celera SNP ID: hCV2459446 / Public SNP ID: rs4786 / SNP
Chromosome Position: 169692132 / SNP in Genomic Sequence: SEQ ID NO: 449 /
SNP Position Genomic: 62418 / Related Interrogated SNP: hCV11975250 / SNP
Source: dbSNP; Celera; HapMap; HGBASE / Population(Allele,Count): Caucasian
(T,169|C,53) / SNP Type: MICRORNA;UTR3;INTRON /
Context (SEQ ID NO: 2598): /
CTTCCATGCTCAGGGGATTCCAGGGCTGTACAGTTCACAACTGAAAAAGAAACCCAAATCAGTTCTGCTCATCTCTCACCTTT
AACAGATAAGAACACTG
R
AAACTAGAACTACAGTTTGGTTTTTTTTTTTTTTTTAGTTTAAAAATTTATAAAATTTCTAATGGAATTTGTAAAATTGACTGTA
ATTCTACCCCTTTTCTT / Celera SNP ID: hCV2459453 / Public SNP ID: rs3917419 /
SNP Chromosome Position: 169699819 / SNP in Genomic Sequence: SEQ ID NO: 449 /
SNP Position Genomic: 54731 / Related Interrogated SNP: hCV11975250 / SNP
Source: dbSNP; Celera; HapMap / Population(Allele,Count): Caucasian
(G,127|A,99) / SNP Type: INTRON /
Context (SEQ ID NO: 2599): /
GTTTAAGGCAGCATCCTAAGATTTTGGGCAAAGGACACTAGTGCAATAATCTTTATTTCAGAGTTTAATCAAATAAATAAACA
AATTTTAAGACTTTCAT
Y
ATTTAGGTCAAAGAGAAAAGACAGGTTTTAGCTACAATACAATAAGAGCTTGTACAGATGTGGTTTTTATTAGAAGGCCTTTT
GCATATCTGTGTTTCAT / Celera SNP ID: hCV2459460 / Public SNP ID: rs5353 / SNP
Chromosome Position: 169702974 / SNP in Genomic Sequence: SEQ ID NO: 449 /
SNP Position Genomic: 51576 / Related Interrogated SNP: hCV11975250 / SNP
Source: dbSNP; Celera; HapMap; HGBASE / Population(Allele,Count): Caucasian
(T,165|C,51) / SNP Type: INTRON;PSEUDOGENE /
Context (SEQ ID NO: 2600): /
TCTTCTGGCTTGCTGAAAGTTTTTTCTTTCCATTTTAAAAATCGTGAATTCCTTTTTGCAATATTGAGGTGGTTATATGGTTT
CTCTTCTCTAATCTGTT
R
ATATGGTGATTTAATGGTTAGAAATTTTCTAATGTAAATTCCACTCATATTGCAGAAATAAACCTAAACTGAGCATGAGGCTA
TATTTTTTATTTGCTTC / Celera SNP ID: hCV8919494 / Public SNP ID: rs1011267 /
SNP Chromosome Position: 169646861 / SNP in Genomic Sequence: SEQ ID NO: 449 /
SNP Position Genomic: 107689 / Related Interrogated SNP: hCV11975250 / SNP
Source: dbSNP; HapMap; HGBASE / Population(Allele,Count): Caucasian
(A,110|G,110) / SNP Type: INTRON /
Context (SEQ ID NO: 2601): /
ATTTTTTATTTGCTTCTATATTTGGTTGCTATACAGTATTATGTTTAAGATTTGTTCACATATATTTGTGAATGGGATTGGAC
TATTTTTCCTTCTTGCC
R
ATTTTTATCTGGTTTTTTAAATTAAGGATATTTTAGACTTATGAAATATTTGGCAAACAATCCTTGGCAAGTAATTTTTTTGGGG
AATTTGTTTTGGCTATT / Celera SNP ID: hCV8919500 / Public SNP ID: rs1011266 /
SNP Chromosome Position: 169647046 / SNP in Genomic Sequence: SEQ ID NO: 449 /
SNP Position Genomic: 107504 / Related Interrogated SNP: hCV11975250 / SNP
Source: dbSNP; HapMap; ABI_Val; HGBASE / Population(Allele,Count): Caucasian
(G,191|A,35) / SNP Type: INTRON /
Context (SEQ ID NO: 2602): /
GTGTCTGTCATCAAGTACAGCACTGAATTGAAACTGAAAACAAGAGTCAAGAAAGAGCAAAGTCAGCCATCTTTATATTCCAC

ATGAATCCTTTCCCTTT
R
TGGTCTTATTTGTTTCTCCTCAGAAAAGACAAAAAGCTGAGCTGTATAAACACCTGTGGGCTGGGGGTTGAGGGATAAATGAG
GGGCGAAATGGAAGCTG / Celera SNP ID: hCV8919501 / Public SNP ID: rs909628 /
SNP Chromosome Position: 169660665 / SNP in Genomic Sequence: SEQ ID NO: 449 /
SNP Position Genomic: 93885 / Related Interrogated SNP: hCV11975250 / SNP
Source: dbSNP; Celera; HapMap; ABI_Val; HGBASE / Population(Allele,Count):
Caucasian (A,199|G,27) / SNP Type: TRANSCRIPTION FACTOR BINDING
SITE;MICRORNA;UTR3;INTRON /
Context (SEQ ID NO: 2603): /
TATTTAATATTTACTATGTATATTTTTTAAAGTATTGTTGCTGCTTAATTAACTATTGATGCTTATATTTAATGTTATAGCCT
CACTCTTGATCATAATG
R
GTCAATGCCTCAAATACCTAAAAAAAAAAAAAAATTAGATAGCCAGACACCAGGAAAGAAAAGTATTTCTTTTTTTAATAAAAA
GAAATACCTTTTTGAGC / Celera SNP ID: hCV8919515 / Public SNP ID: rs1569457 /
SNP Chromosome Position: 169680074 / SNP in Genomic Sequence: SEQ ID NO: 449 /
SNP Position Genomic: 74476 / Related Interrogated SNP: hCV11975250 / SNP
Source: dbSNP; HapMap; HGBASE / Population(Allele,Count): Caucasian
(G,109|A,11) / SNP Type: TRANSCRIPTION FACTOR BINDING SITE;INTRON /
Context (SEQ ID NO: 2604): /
TATAGTGCGAGAATGAGAAATCTTGGTCTAATGGCACTGACTTACCCAAAGTGAGAGCTGAGAGAAACTGTGAAGCAATCATG
ACTTCAAGAGTTCTTTT
M
ACCCAAAGGTTTAGGCTTGAAATACTTTCCTGGGGAGATAAAACACAAAATGAATTAAAGAAGGAAATCGTGGGTAGCTAGTT
ACATTATTCTACCATGA / Celera SNP ID: hCV8919530 / Public SNP ID: rs1805193 /
SNP Chromosome Position: 169702772 / SNP in Genomic Sequence: SEQ ID NO: 449 /
SNP Position Genomic: 51778 / Related Interrogated SNP: hCV11975250 / SNP
Source: dbSNP; HapMap; HGBASE / Population(Allele,Count): Caucasian
(C,199|A,27) / SNP Type: UTR5;INTRON;PSEUDOGENE /
Context (SEQ ID NO: 2605): /
AAATGATGACCTGACCTCAATCATTTCTGCATGCAATTATTTCTTGGCAATCCCTTTCTTTATAGAAATCAAAGATTAAAAAG
TCCAAATTTGCTAAAAC
R
GTAGAGTCCAATTTATAAGAGACCAAATTAACTATGGTTCATTATTAAAACATCACTTGGAAAATGCTGGCTGTTTTGGAATT
GTAGAAGATTTTACAGA / Celera SNP ID: hCV11975318 / Public SNP ID: rs1883228 /
SNP Chromosome Position: 169677000 / SNP in Genomic Sequence: SEQ ID NO: 449 /
SNP Position Genomic: 77550 / Related Interrogated SNP: hCV11975250 / SNP
Source: dbSNP; Celera / Population(Allele,Count): Caucasian (G,168|A,46) /
SNP Type: TRANSCRIPTION FACTOR BINDING SITE;INTRON /
Context (SEQ ID NO: 2606): /
TGTTTGCATTTATGCTTTTATTAGTTCAAAACGTTTGGCCTCATGGAAGTTTTTCATCGTGGAAACCACATATTTCTGAAAAA
ATATCTGACAATATACA
R
ACCTTCCATTCAGTTTTTACTCTCCAATTCTACCATGTTTTCAAAAAACAACTGTAGTAAAAACACTCAGAACTTTATTCTGG
TTAACATCATGCCTTGC / Celera SNP ID: hCV11975322 / Public SNP ID: rs5357 / SNP
Chromosome Position: 169692713 / SNP in Genomic Sequence: SEQ ID NO: 449 /
SNP Position Genomic: 61837 / Related Interrogated SNP: hCV11975250 / SNP
Source: dbSNP; HapMap; HGBASE / Population(Allele,Count): Caucasian
(A,109|G,11) / SNP Type: MICRORNA;UTR3;INTRON /
Context (SEQ ID NO: 2607): /
TGGCATAGTTTCTTCCAGACCTTAGAGTTCTAATTAATCTAACAAGCTCATTAGATCGTGAGCTTCTTGAGAGCGGGAATCTA
CCATGCTAATTCCTTAT
K
GTAACCCTGACAGCTTTTATCCCAACACTGTGCTTCTTGTGGTACTCAAAAAGACTTGTTGAGAAGTGAGTCGAAACTTCATG
CTGACTTATGAAATCTT / Celera SNP ID: hCV16161169 / Public SNP ID: rs2205847 /
SNP Chromosome Position: 169676223 / SNP in Genomic Sequence: SEQ ID NO: 449 /
SNP Position Genomic: 78327 / Related Interrogated SNP: hCV11975250 / SNP
Source: dbSNP; Celera; HapMap; ABI_Val; HGBASE / Population(Allele,Count):
Caucasian (G,176|T,50) / SNP Type: INTRON /
Context (SEQ ID NO: 2608): /
TTGGGGCCAGCCTTCCTTTCCAACTTCAACTCCACAACTCCTCAATAAGCCATGGGCTCAAGAAAGTTCTGCTCAGTGGCCCC
TGAAAAATGCTTTCATA
K

TCTCACTACCATACCACTGCTTACACAATTTCCTTCCTACAGACTGCCTTCCTTTCCTGCTTTTCTCCATATACCTAAATCCT
ATCTATTCTTCATAAGC / Celera SNP ID: hCV16191220 / Public SNP ID: rs2298902 /
SNP Chromosome Position: 169663079 / SNP in Genomic Sequence: SEQ ID NO: 449 /
SNP Position Genomic: 91471 / SNP Source: dbSNP; HapMap; HGBASE /
Population(Allele,Count): Caucasian (G,206|T,20) / SNP Type: INTRON /
Context (SEQ ID NO: 2609): /
CAATGCTTGGTCAGGGCTTGAACCCTCTGAAGCAATGGCCTGAGCTGTACGTTGACACCTTTTAGCCTAGACATCTAGGACAC
AGGGCACCATGACCCGA
R
GCTTCATAAAGTGGGAGGGCCTTGGGACTAGCTGAGGAAACCATTTTTCCATCCTAGGCCTCCAGGCCTGTGATGGGAAGGGC
AGCCATGAAGGTGCCTG / Celera SNP ID: hCV27243253 / Public SNP ID: rs2420505 /
SNP Chromosome Position: 169728137 / SNP in Genomic Sequence: SEQ ID NO: 449 /
SNP Position Genomic: 26413 / Related Interrogated SNP: hCV11975250 / SNP
Source: dbSNP; Celera; HapMap; HGBASE / Population(Allele,Count): Caucasian
(A,181|G,45) / SNP Type: INTRON /
Context (SEQ ID NO: 2610): /
AACTGTATCTTCAGATACACAGGTAAGAAGAATGCCATATGATTATTAAGATACAACAATGCCATCTGTGCCATGCAGTGCTT
AAGTACCTACCATTTTA
Y
GATAAAACAGAATAATTTTTCTTAATGTTTTAGTAATTTTTATCTGCTAGCTTGAAATAATTGGAGATAGAGGCTTTTGAAAG
ATACATTCCACATGAGT / Celera SNP ID: hCV27478380 / Public SNP ID: rs3766141 /
SNP Chromosome Position: 169792692 / SNP in Genomic Sequence: SEQ ID NO: 449 /
SNP Position Genomic: 38142 / Related Interrogated SNP: hCV11975250 / SNP
Source: dbSNP; HapMap; HGBASE / Population(Allele,Count): Caucasian
(T,198|C,28) / SNP Type: INTRON /
Context (SEQ ID NO: 2611): /
ACAATGTGAAAACTGAGGTGATGTGAGCCTAAGTTTCCAACTGGCCCCAGCTGTCAGCTTCTCCTCCCCTGCCTTATTATCAA
AGGCACTGATTGTCTAG
Y
TCTTCCTCTGTACTTCCTACGTAGATCTATCATTTTGATGTAACTTGATTTAGGGGTATAGCTTTTGTGCACAGGGACAAATC
TTACACACCAAAAATTC / Celera SNP ID: hCV27480806 / Public SNP ID: rs3766129 /
SNP Chromosome Position: 169677293 / SNP in Genomic Sequence: SEQ ID NO: 449 /
SNP Position Genomic: 77257 / Related Interrogated SNP: hCV11975250 / SNP
Source: dbSNP; HapMap; HGBASE / Population(Allele,Count): Caucasian
(C,95|T,23) / SNP Type: INTRON /
Context (SEQ ID NO: 2612): /
ACTGTTGTGGTTTAACTCTGACAACTGTGCTTTTTATTGTCTTATTTTTGGCAATGTTTGTGACATGGCCCAGACTTTTCTCA
TCTTTTCAAAAGTAAGA
R
GTACGTATGAAGAAACAGCGACTTATTGTTTATCTCTTTTGTGACTGCCACCCACTAGGTACCTTATCCACACTCACTCACAA
CATTATAGTATACCCAT / Celera SNP ID: hCV27886249 / Public SNP ID: rs3917406 /
SNP Chromosome Position: 169702278 / SNP in Genomic Sequence: SEQ ID NO: 449 /
SNP Position Genomic: 52272 / Related Interrogated SNP: hCV11975250 / SNP
Source: dbSNP; HapMap; HGBASE / Population(Allele,Count): Caucasian
(A,97|G,23) / SNP Type: INTRON /
Context (SEQ ID NO: 2613): /
AGGAAATGAAAAACCAAGTTCAAAGCTATTGCTGGAGAGTCTTCAAGAATCAGATATAAAATTTGTCACAACAATGGGAGAAG
GACCAAAAAATGATAAA
S
CCCCGTCCCTTAATAAGCTCGTATTGTAATTGTAGAAATGACATTAATGTACACTGAACTATGAATAAAAAATAGAAAATGAG
GTGCTAAATATTTGGTA / Celera SNP ID: hCV27936996 / Public SNP ID: rs4656697 /
SNP Chromosome Position: 169679179 / SNP in Genomic Sequence: SEQ ID NO: 449 /
SNP Position Genomic: 75371 / Related Interrogated SNP: hCV11975250 / SNP
Source: dbSNP; HapMap; HGBASE / Population(Allele,Count): Caucasian
(C,96|G,24) / SNP Type: INTRON /
Context (SEQ ID NO: 2614): /
CACAACCAATACCACAGCCCAGATCCTCAGCTCTCCAATGACATTTTCCTCCACTAGACTTGAGCTACCTCCTTCCCTAGGCA
CAGCCTCAACCTCGACA
R
CACCTAAGACTGTACCGTCTCTAAAGTCACATGTTCAAACACTTCACTCTTTAACCACTGTCTCCTATTCTTGCAAGTGTATT
GCTCAAGTATCTCATTG / Celera SNP ID: hCV28023624 / Public SNP ID: rs4656704 /
SNP Chromosome Position: 169688228 / SNP in Genomic Sequence: SEQ ID NO: 449 /
SNP Position Genomic: 66322 / Related Interrogated SNP: hCV11975250 / SNP

Source: dbSNP; HapMap; HGBASE / Population(Allele,Count): Caucasian (A,173|G,53) / SNP Type: INTRON /
Context (SEQ ID NO: 2615): /
AGAGTAAGTTAGACTTCTATGTATTGGGAGACAGGTCCCTGATATGTTGTTACTTGAAAACAAATTATAGATTTGTGCATGGC
ATTTTCTTACCATTTAA
R
CAAAAATCCTACACAGTCTTGTGTGTCACTTAACAATGGGGACATGTTCTGAGAAATGCATCTTAGACGATTTTGTCCTTGTATA
AACATAGATGGTATAGC / Celera SNP ID: hCV29397289 / Public SNP ID: rs4656198 /
SNP Chromosome Position: 169826867 / SNP in Genomic Sequence: SEQ ID NO: 449 /
 SNP Position Genomic: 72317 / Related Interrogated SNP: hCV11975250 / SNP
Source: dbSNP; HapMap; HGBASE / Population(Allele,Count): Caucasian
(G,181|A,45) / SNP Type: TRANSCRIPTION FACTOR BINDING SITE;INTRON /
Context (SEQ ID NO: 2616): /
AAGTAATAATCTATAATTATTTATTACAATTAAAGGAAGGAAGAGACATATGGATTATGAGGGCATTAAAGAGGAGACCTAGT
GTAAGTAGCCAGTTCTC
R
TGAAGGGACATGTATTAGTTGGAGTTCTCCAGAGAAACAGAACCAATGGTGTGTGTGTGTGTGTGTGCGTGTGTGCGTGTGTG
TGTTGGGGTGTGGGGGT / Celera SNP ID: hCV30036721 / Public SNP ID: rs3917449 /
SNP Chromosome Position: 169691219 / SNP in Genomic Sequence: SEQ ID NO: 449 /
 SNP Position Genomic: 63331 / Related Interrogated SNP: hCV11975250 / SNP
Source: dbSNP; HapMap; HGBASE / Population(Allele,Count): Caucasian
(A,96|G,173) / SNP Type: INTRON /
Context (SEQ ID NO: 2617): /
ATCTCAAAATTAAAAAAAAAAAAAAGTAAAAAGAAAAGATAAGAAATATAGTACCAGCCCCTATCTCAGAGTTCCTAGCTTAGA
AAAATTCCCAGAATATA
R
TAAGTGCAATGTAAGGGTCAGCTATCTTCATTATTATTATCTATCATAAATGAAATTACACAATAAAGCTAGATCCGTTTCTT
TCCTCTCCTTCTACAAA / Celera SNP ID: hCV32141631 / Public SNP ID: rs3917436 /
SNP Chromosome Position: 169694619 / SNP in Genomic Sequence: SEQ ID NO: 449 /
 SNP Position Genomic: 59931 / Related Interrogated SNP: hCV11975250 / SNP
Source: dbSNP; HGBASE / Population(Allele,Count): Caucasian (A,171|G,53) /
SNP Type: INTRON /
Context (SEQ ID NO: 2618): /
GCAGTGATGACAACCATAAAAAAGAAATATTGAGTGATATGGGGAGAGTAGTGTAATTGTGTTTACCTCAAAACTGTTCAAAT
TATATGAACAAACACAG
Y
AAACTTAGGTACCACAACAAATTTCTTGTTACTTTTCTCACAACTGCTAAAAATACTACAGTAAGCTTCCAACCAGGATGAGA
ACCATTCACAAAGCTAT / Celera SNP ID: hCV32398748 / Public SNP ID: rs3917417 /
SNP Chromosome Position: 169700062 / SNP in Genomic Sequence: SEQ ID NO: 449 /
 SNP Position Genomic: 54488 / Related Interrogated SNP: hCV11975250 / SNP
Source: dbSNP; HGBASE / Population(Allele,Count): Caucasian (C,106|T,8) / SNP
Type: INTRON;PSEUDOGENE /
Context (SEQ ID NO: 2619): /
TACAGTTTATTGAATGTGTCCTGTGTGCCAGGCACCATGTAACCATGAGCCTATGAAGTTCACACTATTATTATCCTCATTTT
ACAATGAGAAAACTGAC
W
TAGAGAGTTAAACTATCTTGTCAAGGTGCCAAAATAAATAACTGGTGAATCTAGGACTCAAACCCAGCAGGGTCTGACTTCAT
AGTCTCAGCTCACGATC / Celera SNP ID: hCV32141639 / Public SNP ID: rs3917411 /
SNP Chromosome Position: 169700756 / SNP in Genomic Sequence: SEQ ID NO: 449 /
 SNP Position Genomic: 53794 / Related Interrogated SNP: hCV11975250 / SNP
Source: dbSNP; HGBASE / Population(Allele,Count): Caucasian (A,199|T,27) /
SNP Type: MICRORNA;UTR3;INTRON /
Context (SEQ ID NO: 2620): /
TTAGTAGTGTCTCTCATGTAGTTGTGGTAGTAATTAGAATTTCAGAAACAGAAGGAAACCAAGAATAGGTTTGTCATCCATAG
TCTACTACCTTCAATTT
M
TCATTCATAGCTGTGGATAACCAATCACTACTCATTTTTTTCTTCCTTTTTCACCTGCCAATTCAACATATTTAACATGCACTG
TCTCACAGAGGAATGAC / Celera SNP ID: hCV32141645 / Public SNP ID: rs3917452 /
SNP Chromosome Position: 169703617 / SNP in Genomic Sequence: SEQ ID NO: 449 /
 SNP Position Genomic: 50933 / Related Interrogated SNP: hCV11975250 / SNP
Source: dbSNP; HapMap; HGBASE / Population(Allele,Count): Caucasian
(C,197|A,27) / SNP Type: INTRON /
Context (SEQ ID NO: 2621): /

CTATATATATATATTTAATTGGTCAAAATTTTGTTTAAAATTTTTGAAATGTTAATGTGCAAAGAATAAAAATTCTTCCACAA
TGTTAACAGTGACTAAC
Y
CTGGATGGCAGGATTTGGGATAATTTTTATATCCTTCATTATTATTTTCAGGATTTTAAAGTTTTTTTTCAATTTCCCTTTTTT
TCACCTTTATAGTAACA / Celera SNP ID: hCV32398763 / Public SNP ID: rs3917392 /
SNP Chromosome Position: 169704335 / SNP in Genomic Sequence: SEQ ID NO: 449 /
 SNP Position Genomic: 50215 / Related Interrogated SNP: hCV11975250 / SNP
Source: dbSNP; HGBASE / Population(Allele,Count): Caucasian (T,199|C,27) /
SNP Type: INTRON /
Context (SEQ ID NO: 2622): /
CGCCTGTAGGGAAAATAATTATTCTCATAAAATACTGGTTAAAGGTAAGAACTACACTTATGGGAAGGATAGCATTAAGGAAG
TGTAGTAGTATAGGTCT
R
ATTACTTGGAAATATCCATGCATACTAAACAAAAGAGATCCCAGCAGAACATTACTCAAGATCAACGATGTCACATGTTATCC
AAATAATTACATAATAC / Celera SNP ID: hCV29585595 / Public SNP ID: rs10489173 /
SNP Chromosome Position: 169824203 / SNP in Genomic Sequence: SEQ ID NO: 449 /
 SNP Position Genomic: 69653 / Related Interrogated SNP: hCV11975250 / SNP
Source: dbSNP; HapMap / Population(Allele,Count): Caucasian (A,198|G,28) /
SNP Type: ESE;SILENT MUTATION;INTRON /
Context (SEQ ID NO: 2623): /
CTACTCAGATTTAAATCTCAGCAGGGAAGTAAACCTTAGTTTTTACATGAGAAAATGTTACAGCAGCCTTCTCGGCTTCCTTT
ACCCCCATCCCAGTTTC
R
CGAGCTTAGTGCCTTAGATCGGGTTCCTTTAGAAGCAGACCTCGAAATAAGGATGTGGGTGCCAGTCATTTATTGAAAAGATG
ATCCCAAGAAAGCCTAG / Celera SNP ID: hCV30631277 / Public SNP ID: rs10489182 /
SNP Chromosome Position: 169710669 / SNP in Genomic Sequence: SEQ ID NO: 449 /
 SNP Position Genomic: 43881 / Related Interrogated SNP: hCV11975250 / SNP
Source: dbSNP; HapMap / Population(Allele,Count): Caucasian (A,99|G,19) / SNP
Type: TRANSCRIPTION FACTOR BINDING SITE;INTRON /
Context (SEQ ID NO: 2624): /
CTCTAGAATATTCATTTAATGCTCCAGAAGTAATTAGCACAATTGTGCAAGTCTGTGCATCATCAACTATACATTCTGCCTGT
TTACTCCAAATCCACAT
R
AAACTGATTATACAGTCAAAGGCGAGCCCAGTGGAGAGGCATTTTTGGAGACTTCCTGGTACATTGAGACAGGGTCGGCCAGT
CTGCGTTAGGGTCTTGG / Celera SNP ID: hCV30324835 / Public SNP ID: rs10489183 /
SNP Chromosome Position: 169649531 / SNP in Genomic Sequence: SEQ ID NO: 449 /
 SNP Position Genomic: 105019 / Related Interrogated SNP: hCV11975250 / SNP
Source: dbSNP; HapMap / Population(Allele,Count): Caucasian (G,108|A,12) /
SNP Type: INTRON /
Context (SEQ ID NO: 2625): /
CTATCGTAGGTTAAGATTTCTTTTCTTTTCAGTAATGTAGAATTTAATTTAATACCTCTGTTGTCATTTGTTATTTGTATCGC
ACCTAATAATTCAAAGA
R
GAGTTATATTTTCTCGGAAAAATTTTATACTGTGTTTAAGAGGGGTTTTTTTTGTTTTTGTTTTTAGAAAATATTGTCTCATT
TGTAAGCTTAGCTAATT / Celera SNP ID: hCV32141779 / Public SNP ID: rs12122767 /
SNP Chromosome Position: 169790668 / SNP in Genomic Sequence: SEQ ID NO: 449 /
 SNP Position Genomic: 36118 / Related Interrogated SNP: hCV11975250 / SNP
Source: dbSNP / Population(Allele,Count): Caucasian (G,183|A,33) / SNP Type:
 INTRON /
Context (SEQ ID NO: 2626): /
GCTCCTCAGGTTCACTCCAGTCAGGCCACTCCTCAGACTTTTTAGAACTTGATGGGAAGTTTTCACTGTCCTCCGAAGTATTT
TTTACATCTGAGAGTCC
R
TTTATGGGAAATTTAATAGGCTGAGAAAATGGATCGCCTGTAGGGAAAATAATTATTCTCATAAAATACTGGTTAAAGGTAAG
AACTACACTTATGGGAA / Celera SNP ID: hCV32141820 / Public SNP ID: rs12132384 /
SNP Chromosome Position: 169824068 / SNP in Genomic Sequence: SEQ ID NO: 449 /
 SNP Position Genomic: 69518 / Related Interrogated SNP: hCV11975250 / SNP
Source: dbSNP; HapMap / Population(Allele,Count): Caucasian (A,198|G,28) /
SNP Type: ESS;ESE;SILENT MUTATION /
Context (SEQ ID NO: 2627): /
TTACCCATTGTCCACTTTTCCCAAACTACATAGTGTTATATGGTATATGACCCAATCAACGGTGGCAAAGCTCCAGAAATACC
ACATAGACATCAGGGAC
R

CTTTAAACTAATCAGCCTATAGTCCTTTTTCAGTAATTTCCAAACCTGGTTGTGCATCCAAATCACTTGGTAACATTAAAAAAA
ACAAAAAAATATACACG / Celera SNP ID: hCV32141621 / Public SNP ID: rs12133642 /
SNP Chromosome Position: 169689800 / SNP in Genomic Sequence: SEQ ID NO: 449 /
SNP Position Genomic: 64750 / Related Interrogated SNP: hCV11975250 / SNP
Source: dbSNP; HapMap / Population(Allele,Count): Caucasian (A,199|G,27) /
SNP Type: INTRON /
Context (SEQ ID NO: 2628): /
TTTCTTACAAATGATTTCCCAGGAGATCTCATCAAATGCACGAGGATACCTTCTCAGTTTCACCTAGTGAGTAAAAGACTGG
TAACATAGCTCACTTAC
R

ATTTGGATAAACAAAACTAAACAAACAACATCAAAATTTCAGAAAAAATAATAGCAAAACAGAAATCAAACACTCAAATTTT
GGTCCTTCTGTTTATTT / Celera SNP ID: hCV32141741 / Public SNP ID: rs12135361 /
SNP Chromosome Position: 169761591 / SNP in Genomic Sequence: SEQ ID NO: 449 /
SNP Position Genomic: 7041 / Related Interrogated SNP: hCV11975250 / SNP
Source: dbSNP; HapMap / Population(Allele,Count): Caucasian (A,198|G,28) /
SNP Type: INTRON /
Context (SEQ ID NO: 2629): /
GCATAAACTATATACAGGCATACCTTGGAGATACTATGGGTTTGGTTCCCACAATATCTCCAAAACCACATTCGGTTTTATGA
CCACTGCCATAAAACCA
R

CCACATGAATTTTTTGGTTTCCCAATGTATATCAAAGTTACATTTTTACTATACCATAGTCTATTATATATACAATAGCATTA
TATCTAAAAAACAACGT / Celera SNP ID: hCV32141663 / Public SNP ID: rs12142587 /
SNP Chromosome Position: 169716214 / SNP in Genomic Sequence: SEQ ID NO: 449 /
SNP Position Genomic: 38336 / Related Interrogated SNP: hCV11975250 / SNP
Source: dbSNP; HapMap / Population(Allele,Count): Caucasian (G,107|A,11) /
SNP Type: INTRON /
Context (SEQ ID NO: 2630): /
AGAAGGTTTTCAGTTGGCTTTGCCCGGATCCATCAGACGAATCCCTATCTATGGAAGCTATAGATTTATAAAATGTATTTCTT
TTTTTGTGGGGGCATAG
S

GTCTCACCCTGTCACCCAACCTGGAATGCAGTGGCACAGTCATAACTCACTGAAGACTCAAACTCCTGGGCTCAAGTGATTCT
TCCACCTTGGCCTCCCA / Celera SNP ID: hCV32141665 / Public SNP ID: rs10800470 /
SNP Chromosome Position: 169717272 / SNP in Genomic Sequence: SEQ ID NO: 449 /
SNP Position Genomic: 37278 / Related Interrogated SNP: hCV11975250 / SNP
Source: dbSNP; HapMap / Population(Allele,Count): Caucasian (C,92|G,18) / SNP
Type: INTRON /
Context (SEQ ID NO: 2631): /
AGGCCTGAGTAGAGGGAGGGAGATGGGGAAACAGCCAGTCATCAGAGCACACACAACATTTATCAATTAAGTTTATCACCTTG
AGGGCACAGGTCATGAT
R

CTTCAAAACAATTACAATAATAAAATAAAAAATCATTGATCGCAGATCACCATAACAGATATAATGATAATGAAAAATTTGAA
GTATTGTGAGAATTACC / Celera SNP ID: hCV32141669 / Public SNP ID: rs10800472 /
SNP Chromosome Position: 169719017 / SNP in Genomic Sequence: SEQ ID NO: 449 /
SNP Position Genomic: 35533 / Related Interrogated SNP: hCV11975250 / SNP
Source: dbSNP; HapMap / Population(Allele,Count): Caucasian (A,98|G,18) / SNP
Type: INTRON /
Context (SEQ ID NO: 2632): /
GTGTTATATGGTATATGACCCAATCAACGGTGGCAAAGCTCCAGAAATACCACATAGACATCAGGGACACTTTAAACTAATCA
GCCTATAGTCCTTTTTC
W

GTAATTTCCAAACCTGGTTGTGCATCCAAATCACTTGGTAACATTAAAAAAACAAAAAAATATACACGCAACATTCGCTCCCA
ATCCTACTGAATCAGAA / Celera SNP ID: hCV32141622 / Public SNP ID: rs12133666 /
SNP Chromosome Position: 169689832 / SNP in Genomic Sequence: SEQ ID NO: 449 /
SNP Position Genomic: 64718 / Related Interrogated SNP: hCV11975250 / SNP
Source: dbSNP; HapMap / Population(Allele,Count): Caucasian (A,87|T,9) / SNP
Type: INTRON /
Context (SEQ ID NO: 2633): /
ACTTTTTAGAACTTGATGGGAAGTTTTCACTGTCCTCCGAAGTATTTTTTACATCTGAGAGTCCATTTATGGGAAATTTAATA
GGCTGAGAAAATGGATC
R

CCTGTAGGGAAAATAATTATTCTCATAAAATACTGGTTAAAGGTAAGAACTACACTTATGGGAAGGATAGCATTAAGGAAGTG
TAGTAGTATAGGTCTAA / Celera SNP ID: hCV32141821 / Public SNP ID: rs12135726 /
SNP Chromosome Position: 169824104 / SNP in Genomic Sequence: SEQ ID NO: 449 /

SNP Position Genomic: 69554 / Related Interrogated SNP: hCV11975250 / SNP Source: dbSNP; HapMap / Population(Allele,Count): Caucasian (G,198|A,28) / SNP Type: ESE;TRANSCRIPTION FACTOR BINDING SITE;SILENT RARE CODON;SILENT MUTATIO / N //

Context (SEQ ID NO: 2634): /
TTTCTGTGGTCTCTTCCACACTGCTACCAAAGTGAACTTTCTAGCACACACAAATTGGCCCACTCTTCTTTAGGGGAAGCAGT
TTCCTAAGTAATGAGAG
S

TTTCATTGTCTTCCACATGCTCAACATTTACTCTTTGACCTCAGTCCAATTCTGAGACCTATGATGACAACTCCATTTCAGAG
TTAAAGTGCTTCATTCC / Celera SNP ID: hCV32141828 / Public SNP ID: rs12136425 /
SNP Chromosome Position: 169830104 / SNP in Genomic Sequence: SEQ ID NO: 449 /
SNP Position Genomic: 75554 / Related Interrogated SNP: hCV11975250 / SNP Source: dbSNP; HapMap / Population(Allele,Count): Caucasian (G,198|C,28) / SNP Type: INTRON /

Context (SEQ ID NO: 2635): /
GCTGTGGGGAGAGGAGTAGCTACTCCAGGCTGCTGCCCTAGCTAAGGTGACCCTCCCCTTCTGCTGGAAGTACCATGCCATAT
GGCCTCTGCATCAAGGG
Y

TCTTATGGGATATTCTCAGAGAATCTCTGCCGTTTCATCTGTTCTGATATCTACCCAAGCATTTTGAAAAACATCCCAATTCA
CTGAAGCAAGTCCAACT / Celera SNP ID: hCV32141586 / Public SNP ID: rs12137905 /
SNP Chromosome Position: 169672867 / SNP in Genomic Sequence: SEQ ID NO: 449 /
SNP Position Genomic: 81683 / Related Interrogated SNP: hCV11975250 / SNP Source: dbSNP; HapMap / Population(Allele,Count): Caucasian (C,109|T,11) / SNP Type: INTRON /

Context (SEQ ID NO: 2636): /
AGTTGAGTAAATCAAAGATGGGACTGTTAAGTTATTTGTGTTATTTACCTGCTTTTTTGAAAATGTAAAAATAAAACTCTAGGT
TTAATTAGTAGTATGCT
R

TTTAGTAATGAAGTAAAGCTAGAGGCTTCGAACAAATCTTGTGTAATTTCCTCTTGAATGAGAGAGAAAATTTAAAGTAAGCA
AACAAATAAGTTGTGTG / Celera SNP ID: hDV70965007 / Public SNP ID: rs17529304 /
SNP Chromosome Position: 169781291 / SNP in Genomic Sequence: SEQ ID NO: 449 /
SNP Position Genomic: 26741 / Related Interrogated SNP: hCV11975250 / SNP Source: dbSNP; HapMap / Population(Allele,Count): Caucasian (A,198|G,28) / SNP Type: INTRON /

Context (SEQ ID NO: 2637): /
TAAAAAAAAAGTATGAAAGGATTCCCACTGAAAATCCCCCCGAGAAGTTCAGAAAGAGAAATTAACTTTTTATTTATACTTCT
TTATATCATTTGACTGA
R

TATGACCCTCCTACATTACTTATATAATACTAAAAATCTAAAAAAAATGACACACAAAAAGTTAGACCTTTTAATAATTCTTAC
CTTCTAGAGAAAGGTCA / Celera SNP ID: hDV70966798 / Public SNP ID: rs17543370 /
SNP Chromosome Position: 169828098 / SNP in Genomic Sequence: SEQ ID NO: 449 /
SNP Position Genomic: 73548 / Related Interrogated SNP: hCV11975250 / SNP Source: dbSNP / Population(Allele,Count): Caucasian (A,194|G,28) / SNP Type: INTRON /

Context (SEQ ID NO: 2638): /
GATCAGATCCAGTGAGGTGTGTAATAACCTGAACAGCTGACATCTATGTCTCCATGGCTGATGGTCTGGATTATCACTTACAC
ACTTTACTAAATCCTTA
R

CATATCTTTCACAAAGCAGATAAAGAGTGATTTTTATGTTATGTGTTTTATGTCTGAAGTTTCCTTTAAATAAAAGCTTTCTT
AGTGCATTATGTTGTAG / Celera SNP ID: hDV70974851 / Public SNP ID: rs17601631 /
SNP Chromosome Position: 169800690 / SNP in Genomic Sequence: SEQ ID NO: 449 /
SNP Position Genomic: 46140 / Related Interrogated SNP: hCV11975250 / SNP Source: dbSNP / Population(Allele,Count): Caucasian (A,198|G,28) / SNP Type: INTRON /

Context (SEQ ID NO: 2639): /
GTTTGAAGATCTTGGTTCGTTCTCCTCCCACAACCACCTCTGGTCCAAGCAGAGAGACCAGCACTGCTAGGCTATGCAGAGTA
ATTGCCACAATGGAATC
R

CTAGTATCACGCAGGCCCAGCAAAACCTAGGAGCAAATGAGGTAATAAGGGTTGACAACCACAATACCTATCTCTTTCTTCCC
CTGTAAATGAGCAATTG / Celera SNP ID: hDV70975002 / Public SNP ID: rs17602701 /
SNP Chromosome Position: 169828327 / SNP in Genomic Sequence: SEQ ID NO: 449 /
SNP Position Genomic: 73777 / Related Interrogated SNP: hCV11975250 / SNP Source: dbSNP; HapMap / Population(Allele,Count): Caucasian (G,194|A,28) /

SNP Type:    SILENT MUTATION;PSEUDOGENE /
Context            (SEQ ID NO: 2640): /
TCTCTCATATCACTCTTTTCTCATTCTTTCTACCTGTGATCATTTCTCCTGAGATCCTTCATGAGCACTTATCCAGGACCATA
AACTACTATAATTTCTC
R
GCTTTTGTCTTTTGTATATTGCTCTTCTGACTCTAATCTACATGTATCCTTTGGTCAAACTTACTGACTTTTGCGGACTCACA
AATCTATGTGATCTTTG / Celera SNP ID:  hDV71028805 / Public SNP ID:    rs4987299 /
SNP Chromosome Position:    169675349 / SNP in Genomic Sequence:   SEQ ID NO: 449 /
 SNP Position Genomic:   79201 / Related Interrogated SNP:   hCV11975250 / SNP
Source:   dbSNP; HapMap / Population(Allele,Count):   Caucasian (A,199|G,27) /
SNP Type:    INTRON /
Context            (SEQ ID NO: 2641): /
CTTAACTCCCCCAACATTTATGCAGGGTAACATTCTACCAATTTTTACAACTTAGTGTTTTTCTAATTTGTCTTCCACTTGTC
AACTCTACTACCATTGC
Y
GTCATTATCTTTCTTCTAGATTCTTGCAATAGTTTATTAGATGAGTTCCACAATTTTAAATTAGCTCCCCTCAAATACATTAT
TCATACTACAGGGAGAC / Celera SNP ID:  hDV71028807 / Public SNP ID:    rs4987302 /
SNP Chromosome Position:    169674983 / SNP in Genomic Sequence:   SEQ ID NO: 449 /
 SNP Position Genomic:   79567 / Related Interrogated SNP:   hCV11975250 / SNP
Source:   dbSNP; HapMap / Population(Allele,Count):   Caucasian (C,109|T,11) /
SNP Type:    INTRON /
Context            (SEQ ID NO: 2642): /
AAATTCTTCTGATTCCTTGAGTCCATTCTTCCTTGAAGTCTCTAGAGCAAGGTAGCAATAATGGCTTAAATTTGACAGATGAG
GAATAAGACATGAAAGT
K
GTACTGTTTACTAAATGGCATGGCAGGGTCTAAAATGTGAAAACTTTGATTTCCAAGTCTTAAATTTACCTATTGTACCACCA
ATCAGAACAGGGAAATA / Celera SNP ID:  hDV71028808 / Public SNP ID:    rs4987304 /
SNP Chromosome Position:    169674561 / SNP in Genomic Sequence:   SEQ ID NO: 449 /
 SNP Position Genomic:   79989 / Related Interrogated SNP:   hCV11975250 / SNP
Source:   dbSNP; HapMap / Population(Allele,Count):   Caucasian (T,109|G,11) /
SNP Type:    INTRON /
Context            (SEQ ID NO: 2643): /
TCTTAAATTTACCTATTGTACCACCAATCAGAACAGGGAAATAGATACAGTTTCTGGAGAGGGTTGTCAAAATCAAAAGAGTT
AAAATTCTGGAACTGAG
M
TAGAATAGAAGACAGAATTGAGGTCAAAATGCTTCCTAGACTTTTATATTCTCTCCTTGCAAAGAAGGCTTATTATGGAGACC
CACTCTTTCTCTGAACA / Celera SNP ID:  hDV71028809 / Public SNP ID:    rs4987307 /
SNP Chromosome Position:    169674403 / SNP in Genomic Sequence:   SEQ ID NO: 449 /
 SNP Position Genomic:   80147 / Related Interrogated SNP:   hCV11975250 / SNP
Source:   dbSNP; HapMap / Population(Allele,Count):   Caucasian (C,109|A,11) /
SNP Type:    INTRON /
Context            (SEQ ID NO: 2644): /
ATCAAATCACCTTGAAAAGGTTCATCAAAGTAATGGGAAGAACTAAGGATAGAATCAGACCCTCTGCCAATATGTAAAAAAGT
AGATTCCTTCAAATGTT
M
GTATCAGGAGACAGATTTTTTTTTTATACCAGTATTTTCAAATAGAGGCTCCCAAATGAACTTAGTTTACACTTACAAGAGTAT
CTCTAAACATGCCCTTT / Celera SNP ID:  hDV71028811 / Public SNP ID:    rs4987318 /
SNP Chromosome Position:    169671478 / SNP in Genomic Sequence:   SEQ ID NO: 449 /
 SNP Position Genomic:   83072 / Related Interrogated SNP:   hCV11975250 / SNP
Source:   dbSNP; HapMap / Population(Allele,Count):   Caucasian (A,96|C,24) / SNP
Type:    INTRON /
Context            (SEQ ID NO: 2645): /
TACTTATTCGTGAAGGAAGTAGAGGGAAGTGGTAACTACTGCATTGGAAAGAAATTAGTGCAGGTCCTTGGCATGTAAAAATC
AAGTGCTTATTTTTGCA
S
AACTGCTTTAAAGTAAAAGCATTAATGATTTCCTTTGACTGGTGAATTATCCAGTTCAGATCATTGGCTTTTATTTTTTCCTGA
AGTTGTATTAGGCTTTT / Celera SNP ID:  hDV71028814 / Public SNP ID:    rs4987323 /
SNP Chromosome Position:    169671277 / SNP in Genomic Sequence:   SEQ ID NO: 449 /
 SNP Position Genomic:   83273 / Related Interrogated SNP:   hCV11975250 / SNP
Source:   dbSNP; HapMap / Population(Allele,Count):   Caucasian (G,199|C,27) /
SNP Type:    TFBS SYNONYMOUS;INTRON /
Context            (SEQ ID NO: 2646): /
GCATTGGAAAGAAATTAGTGCAGGTCCTTGGCATGTAAAAATCAAGTGCTTATTTTTGCACAACTGCTTTAAAGTAAAAGCAT

TAATGATTTCCTTTGAC

K

GGTGAATTATCCAGTTCAGATCATTGGCTTTTATTTTTCCTGAAGTTGTATTAGGCTTTTAGCTCTTCTCTGTGTTTCAGAGT
TCCAGTCATTGAACAAG / Celera SNP ID: hDV71028815 / Public SNP ID: rs4987324 /
SNP Chromosome Position: 169671237 / SNP in Genomic Sequence: SEQ ID NO: 449 /
SNP Position Genomic: 83313 / Related Interrogated SNP: hCV11975250 / SNP
Source: dbSNP; HapMap / Population(Allele,Count): Caucasian (T,199|G,27) /
SNP Type: INTRON /
Context (SEQ ID NO: 2647): /
CAGGTCCTTGGCATGTAAAAATCAAGTGCTTATTTTTGCACAACTGCTTTAAAGTAAAAGCATTAATGATTTCCTTTGACTGG
TGAATTATCCAGTTCAG

M

TCATTGGCTTTTATTTTTCCTGAAGTTGTATTAGGCTTTTAGCTCTTCTCTGTGTTTCAGAGTTCCAGTCATTGAACAAGTCC
ATGGAGCTAAATACAAC / Celera SNP ID: hDV71028816 / Public SNP ID: rs4987325 /
SNP Chromosome Position: 169671217 / SNP in Genomic Sequence: SEQ ID NO: 449 /
SNP Position Genomic: 83333 / Related Interrogated SNP: hCV11975250 / SNP
Source: dbSNP; HapMap / Population(Allele,Count): Caucasian (A,109|C,11) /
SNP Type: TRANSCRIPTION FACTOR BINDING SITE;INTRON /
Context (SEQ ID NO: 2648): /
AATTGCTAACCAACTGCTCTGTGTTGCCTTTCTGTGGGCTCAGTTGATTGTCATTTTAATTTTCCTTCCATCAAATTAGTTAG
TACGTATTCTGTTCACT

R

AATATGGTATGTAAAGGTTGCCCCCTGTCCATTTACACTGGCAATTACAAAAATATTATTTATCAGTCCCTAATTGCGTGCCA
CTCTGATCTTATCTTTC / Celera SNP ID: hDV71028819 / Public SNP ID: rs4987340 /
SNP Chromosome Position: 169669143 / SNP in Genomic Sequence: SEQ ID NO: 449 /
SNP Position Genomic: 85407 / Related Interrogated SNP: hCV11975250 / SNP
Source: dbSNP; HapMap / Population(Allele,Count): Caucasian (G,109|A,11) /
SNP Type: INTRON /
Context (SEQ ID NO: 2649): /
TATGCAAGAACTTCATTAGCTATGTATAACCTGAGAGCACTGAGCAGTTAAAATGTCTCTGCCCTTGCTATTAGAAATTCTGT
GCTGATTGAATCTCACT

R

TCTTATAGTATTATACAGTACTAAGTACTATACAGTACTAAGTATAAGTATCAAGTACTGCATTAAGGCAAAAGAAGAATGAC
AGTTTTCTCCAACCACT / Celera SNP ID: hDV71028821 / Public SNP ID: rs4987343 /
SNP Chromosome Position: 169668856 / SNP in Genomic Sequence: SEQ ID NO: 449 /
SNP Position Genomic: 85694 / Related Interrogated SNP: hCV11975250 / SNP
Source: dbSNP; HapMap / Population(Allele,Count): Caucasian (A,109|G,11) /
SNP Type: INTRON /
Context (SEQ ID NO: 2650): /
TCTTTCTTCCTACCTTGGGCGATGATGGCAGAACTGACGGATAGAGAACAGGGTGGGTTATTACACACTGCCCCCTGGGATAA
GGTGGCTATTGTCTAAA

Y

AATTTTAAGGACTTTGGGGCTGTAAGTCACCTTAATAAGGAAGCTATTTATTTCCGAATTCTAAAATCTTTTAATAAGTGTTT
GTAGAATCAAAAGAATA / Celera SNP ID: hDV71028822 / Public SNP ID: rs4987345 /
SNP Chromosome Position: 169668608 / SNP in Genomic Sequence: SEQ ID NO: 449 /
SNP Position Genomic: 85942 / Related Interrogated SNP: hCV11975250 / SNP
Source: dbSNP; HapMap / Population(Allele,Count): Caucasian (T,107|C,11) /
SNP Type: INTRON /
Context (SEQ ID NO: 2651): /
CAAATGTTAGTATCAGGAGACAGATTTTTTTTTTATACCAGTATTTTCAAATAGAGGCTCCCAAATGAACTTAGTTTACACTTA
CAAGAGTATCTCTAAAC

W

TGCCCTTTTACTTATTCGTGAAGGAAGTAGAGGGAAGTGGTAACTACTGCATTGGAAAGAAATTAGTGCAGGTCCTTGGCATG
TAAAAATCAAGTGCTTA / Celera SNP ID: hDV71028828 / Public SNP ID: rs4987395 /
SNP Chromosome Position: 169671386 / SNP in Genomic Sequence: SEQ ID NO: 449 /
SNP Position Genomic: 83164 / Related Interrogated SNP: hCV11975250 / SNP
Source: dbSNP; HapMap / Population(Allele,Count): Caucasian (T,109|A,11) /
SNP Type: INTRON /
Context (SEQ ID NO: 2652): /
CAGCCTCACCAGCATCTGTTGTTTGACTTTTTTAATAACGGCCATTGTGACTGGTGTGAGATAGTATCTCGTTGTGATTTTGAT
TTGCATTTCCCTGATAG

Y

TTTTTCATATGATGGTTGGCCTCTTGTGTGTCTTGTTTTGAGAAGCGTCTGTTCATGTCTTTTGCCCATTTTCTAATGGGGTT

GTTTTTTACTTGTTCAG / Celera SNP ID: hCV32141799 / Public SNP ID: rs12133074 / SNP Chromosome Position: 169808651 / SNP in Genomic Sequence: SEQ ID NO: 449 / SNP Position Genomic: 54101 / Related Interrogated SNP: hCV11975250 / SNP Source: dbSNP; HapMap / Population(Allele,Count): Caucasian (C,198|T,28) / SNP Type: INTRON /
Context (SEQ ID NO: 2653): /
TTTCAAACATTGCCAAGAGAACCAGTAAGTATCCCCTGGATGCAACAAGGGTGTCAGTGGGGAGACTATTTGCAGTGCAGACA TCAAGACAGAGGTCAGA
Y

GGCCAAGCATTGAGCATTACTGAGGATGAGAAAGTAGAGACAGAACAAGCAGTCATCTCTTTTAAAATGTTTGGCTGTGAAGG AGAAGTGGGGCAGGGGT / Celera SNP ID: hDV71070471 / Public SNP ID: rs4987363 / SNP Chromosome Position: 169663938 / SNP in Genomic Sequence: SEQ ID NO: 449 / SNP Position Genomic: 90612 / Related Interrogated SNP: hCV11975250 / SNP Source: CDX; dbSNP; HapMap / Population(Allele,Count): Caucasian (C,199|T,27) / SNP Type: INTRON /
Context (SEQ ID NO: 2654): /
TTCCTGGGGAGATAAAACACAAAATGAATTAAAGAAGGAAATCGTGGGTAGCTAGTTACATTATTCTACCATGATGTTTAAGG CAGCATCCTAAGATTTT
R

GGCAAAGGACACTAGTGCAATAATCTTTATTTCAGAGTTTAATCAAATAAATAAACAAATTTTAAGACTTTCATTATTTAGGT CAAAGAGAAAAGACAGG / Celera SNP ID: hDV76908547 / Public SNP ID: rs3917400 / SNP Chromosome Position: 169702899 / SNP in Genomic Sequence: SEQ ID NO: 449 / SNP Position Genomic: 51651 / Related Interrogated SNP: hCV11975250 / SNP Source: CDX; dbSNP / Population(Allele,Count): Caucasian (G,109|A,9) / SNP Type: INTRON;PSEUDOGENE /
Context (SEQ ID NO: 2655): /
TGGACTCAGTGGGAGCTAAGGAAGTAAGAGACGAAGAAAGGTCATGAGGAAGAATTGATGTTAAAGTCTCTCCGTCCTGTCCC TTTGGCCTTTTTTCTGT
R

CATTCATTACTAGGAGCAGAAGAGCTATCTAGTTTAATACAAGAAGCAGAGATGTGGCATTACAGGCCTTTGAGATCTGCTCC AAGCCACCTTTGAAGCT / Celera SNP ID: hDV76908557 / Public SNP ID: rs3917427 / SNP Chromosome Position: 169696033 / SNP in Genomic Sequence: SEQ ID NO: 449 / SNP Position Genomic: 58517 / Related Interrogated SNP: hCV11975250 / SNP Source: CDX; dbSNP / Population(Allele,Count): Caucasian (A,199|G,27) / SNP Type: INTRON /
Context (SEQ ID NO: 2656): /
GCCTGATCACAGTAGTTGTTCTCTCTTATTTCCCAGAGTTTTTCTGCCCCTTTAAAAGAACCTCTGCTGTTCTGATCCTTATC ACATCTCTGTTTTGACT
S

TTGGCTTTGTTGTTGCCAGTGTTCAGCCAGAACTTCTCTGAAACTTTTTTTTTCAACACATGCTAAGTTAATGGAAGTGTAGGA GAGTTTTGATTCTCACA / Celera SNP ID: hDV76908563 / Public SNP ID: rs3917441 / SNP Chromosome Position: 169692121 / SNP in Genomic Sequence: SEQ ID NO: 449 / SNP Position Genomic: 62429 / Related Interrogated SNP: hCV11975250 / SNP Source: CDX; dbSNP / Population(Allele,Count): Caucasian (G,109|C,9) / SNP Type: MICRORNA;UTR3;INTRON /
Context (SEQ ID NO: 2657): /
GACATAGAGAGTTAAACTATCTTGTCAAGGTGCCAAAATAAATAACTGGTGAATCTAGGACTCAAACCCAGCAGGGTCTGACT TCATAGTCTCAGCTCAC
R

ATCACCATATGACACCATCTGCACCAGGGAAGGGAAGGCATGCAGACCTGACTCTAATGCCAGCTAGGACGTGAGATGGTGCT ACCATCTCAAGTGAAGA / Celera SNP ID: hDV76908571 / Public SNP ID: rs3917454 / SNP Chromosome Position: 169700853 / SNP in Genomic Sequence: SEQ ID NO: 449 / SNP Position Genomic: 53697 / Related Interrogated SNP: hCV11975250 / SNP Source: CDX; dbSNP / Population(Allele,Count): Caucasian (G,213|A,13) / SNP Type: MICRORNA;UTR3;INTRON /
Context (SEQ ID NO: 2658): /
TAACTCAAAAATGTTTTAAGTACTCTTAAACATGTAAGCCAAACAAACCATGAGTGTAGTCAGATGTGCTTCCATATTCCTTA TGAGAGACTCTCAAATT
W

AAGCCTGTACTCCAAATAAATCTCCTTAGGAAGAATTTTATCCATTTTCCTTAGAGTGCTCATCATGGCAGTTCCATTGCACA ATTCCGGGAGGCATCAT / Celera SNP ID: hDV76908576 / Public SNP ID: rs3917461 / SNP Chromosome Position: 169695491 / SNP in Genomic Sequence: SEQ ID NO: 449 / SNP Position Genomic: 59059 / Related Interrogated SNP: hCV11975250 / SNP

Source: CDX; dbSNP / Population(Allele,Count): Caucasian (T,114|A,6) / SNP Type: INTRON /
Context (SEQ ID NO: 2659): /
GATCTCTCAAGATGACAATATTTATTCTCCACACAGCACATACTTCAGGGTTGGAAGGCAGGGGCAATCTTCTCCTTTATAAT
GAGTGCCTCTTATATAT
S
TTTATTCATCTGCCCTCTTGTAAAACACACACACACACACACACAAAGAAGAAATAAAATAACTCTGCTTCTTTGAAGCTTGT
GACACTGAGATAAACCA / Celera SNP ID: hDV77030725 / Public SNP ID: rs4656701 /
SNP Chromosome Position: 169687903 / SNP in Genomic Sequence: SEQ ID NO: 449 /
SNP Position Genomic: 66647 / Related Interrogated SNP: hCV11975250 / SNP
Source: CDX; dbSNP / Population(Allele,Count): Caucasian (G,173|C,53) / SNP
Type: INTRON /
Context (SEQ ID NO: 2660): /
CTCCTCATGCAAGATTGGCTTTGGCACCTAGTTCCTGATCTTCCTTTCCCTGTAAGCACTTCTCATAGTCTTACGGGACTTCA
CCATCCATGGCACAACC
M
ATACCACAGCCCAGATCCTCAGCTCTCCAATGACATTTTCCTCCACTAGACTTGAGCTACCTCCTTCCCTAGGCACAGCCTCA
ACCTCGACAACACCTAA / Celera SNP ID: hDV77030727 / Public SNP ID: rs4656703 /
SNP Chromosome Position: 169688135 / SNP in Genomic Sequence: SEQ ID NO: 449 /
SNP Position Genomic: 66415 / Related Interrogated SNP: hCV11975250 / SNP
Source: CDX; dbSNP / Population(Allele,Count): Caucasian (A,173|C,53) / SNP
Type: INTRON //

Gene Number: 113 / Gene Symbol: EDEM2 - 55741 / Gene Name: ER degradation
enhancer, mannosidase alpha-like 2 / Chromosome: 20 / OMIM NUMBER: / OMIM
Information: // Genomic Sequence (SEQ ID NO: 450): // / SNP Information /
Context (SEQ ID NO: 2661): /
GAAGACTGGCATGGACACAGTCCCCTTGTACATCTGAACCCACAGGTACCAGTCATCGAAGCGGGTGTAGTTCCGGATGGCTT
TGTTATACTCTGCAGTG
R
GGGAGATGGGATAATGGACATATGAGTGATTAGGGAGAAAAAAGGCAGGCCTGGACAGCGGAAGCGAGAACTTACACTTTGGA
AAGAGCACCAGGATGCA / Celera SNP ID: hCV16191203 / Public SNP ID: rs2295887 /
SNP Chromosome Position: 33714187 / SNP in Genomic Sequence: SEQ ID NO: 450 /
SNP Position Genomic: 41926 / Related Interrogated SNP: hCV1825046 / SNP
Source: Applera / Population(Allele,Count): Caucasian (A,19|G,5) African
American (A,6|G,16) total (A,25|G,21) / SNP Type: ACCEPTOR SPLICE SITE;INTRON
/ SNP Source: dbSNP; HapMap; HGBASE / Population(Allele,Count): Caucasian
(G,85|A,141) / SNP Type: ACCEPTOR SPLICE SITE;INTRON /
Context (SEQ ID NO: 2662): /
CAATTTGGCAGAACTCTAAATCTATCTAATTCACTCTAGGTCTCTCTTCCATCCTCAACCTGTCCCTCCAAAAAAAGTACTCA
AGCAATTAACAAAGTAC
M
TTATTTGACTTATACATCAGAATGAGCTTGCTTTGCCAGACTCATAGAAAGATGATCACATTCTTTGATCATTCCTACCTGAA
GACCAGGCCAGTAGGCC / Celera SNP ID: hCV16191204 / Public SNP ID: rs2295886 /
SNP Chromosome Position: 33713975 / SNP in Genomic Sequence: SEQ ID NO: 450 /
SNP Position Genomic: 41714 / Related Interrogated SNP: hCV1825046 / SNP
Source: Applera / Population(Allele,Count): Caucasian (A,11|C,27) African
American (A,5|C,33) total (A,16|C,60) / SNP Type: INTRON / SNP Source:
dbSNP; HapMap; HGBASE / Population(Allele,Count): Caucasian (A,63|C,163) / SNP
Type: INTRON /
Context (SEQ ID NO: 2663): /
GTTCCTGACACTCAATTTGGCAGAACTCTAAATCTATCTAATTCACTCTAGGTCTCTCTTCCATCCTCAACCTGTCCCTCCAA
AAAAAGTACTCAAGCAA
Y
TAACAAAGTACATTATTTGACTTATACATCAGAATGAGCTTGCTTTGCCAGACTCATAGAAAGATGATCACATTCTTTGATCA
TTCCTACCTGAAGACCA / Celera SNP ID: hCV16191205 / Public SNP ID: rs2295885 /
SNP Chromosome Position: 33713963 / SNP in Genomic Sequence: SEQ ID NO: 450 /
SNP Position Genomic: 41702 / Related Interrogated SNP: hCV1825046 / SNP
Source: dbSNP; HapMap / Population(Allele,Count): Caucasian (T,63|C,161) /
SNP Type: INTRON /
Context (SEQ ID NO: 2664): /
ACAGGGCGGAAAGTGGGAATCACTAGCTGATTTCTCCCTGGGACCTAGCGGCTGTGTCGGAGCAAGTCGGGGTCTGGGATGGA
CGGAAAGGGAAAGGACC

S
GGTTCACGCTTCCCATTCCCCAACTGCGAAAGGGCGGGTCACAGTTCACCTGTAGTGGGCGGGATCTGGCGCGGAGCCGTCGG
GACCTGGCGCACCATGG / Celera SNP ID: hCV1825005 / Public SNP ID: rs945959 /
SNP Chromosome Position: 33734905 / SNP in Genomic Sequence: SEQ ID NO: 450 /
SNP Position Genomic: 62644 / Related Interrogated SNP: hCV1825046 / SNP
Source: dbSNP; Celera; HapMap; HGBASE / Population(Allele,Count): Caucasian
(G,64|C,56) / SNP Type: UTR5;INTRON /
Context (SEQ ID NO: 2665): /
CATGGGGATAATTAACTCCTAGTAAGAAAACCACCCAGAGCCACTGAAAGGAACAGATGGAAAGAAATGAACTTGTGGGCAGT
TGTGGAGGATGGAGCCT

W
GTAGAGGGGATTTGGGGTTTCAGCAACCAGAGCCTGTGGGCAGATGACAAGCCAGAAATGCTGCTCACCTGGCCGAAGTGGGT
AGCCCTCTCGCTTCTCC / Celera SNP ID: hCV25619982 / Public SNP ID: rs6120838 /
SNP Chromosome Position: 33711624 / SNP in Genomic Sequence: SEQ ID NO: 450 /
SNP Position Genomic: 39363 / Related Interrogated SNP: hCV1825046 / SNP
Source: Applera / Population(Allele,Count): Caucasian (A,12|T,12) African
American (A,10|T,10) total (A,22|T,22) // / SNP Type: INTRON / SNP Source:
dbSNP; HapMap / Population(Allele,Count): Caucasian (T,40|A,64) / SNP Type:
INTRON /
Context (SEQ ID NO: 2666): /
AGCTAACCGCATTTAGCCGTCTCTCCAGCTGCATGAGAAAATTAACTAGAGTCACAATTGCCCGTAGCCACCAATCCTTTCTT
TGAATGAAATCTCATGA

R
ACAGCCCAATATGTAAAACATGATAACCAAAATATGATAGCCAAAAGCAATTTATTATAGTTTAGCCTCAAAAAAATAAAAAT
AAAAAAATTATCCAGTG / Celera SNP ID: hCV25952685 / Public SNP ID: rs17092297 /
SNP Chromosome Position: 33703134 / SNP in Genomic Sequence: SEQ ID NO: 450 /
SNP Position Genomic: 30873 / Related Interrogated SNP: hCV25620145 / SNP
Source: Applera / Population(Allele,Count): Caucasian (A,0|G,14) African
American (A,7|G,3) total (A,7|G,17) / SNP Type: INTERGENIC;UNKNOWN / SNP
Source: dbSNP; Applera / Population(Allele,Count): Caucasian (G,204|A,22) /
SNP Type: INTERGENIC;UNKNOWN /
Context (SEQ ID NO: 2667): /
CTGAGAGAGAGAAAGACACACGGTCCCAACGGGAAGGCCGATGGCCAAAGAAGGATCTACTCACCCCCAACCCTGACTGCCCA
GGGAGATGCAGGGCAGG

Y
GCCCCAGTGCTTCTTGGGAAACATGCAGACCCTGAGAGGGAAGGGCAATGCTGGATCATGGCCAGCCTTCCTGTACATCTGCA
TAGTAGAGATGCATCTC / Celera SNP ID: hCV7593267 / Public SNP ID: rs1033797 /
SNP Chromosome Position: 33719687 / SNP in Genomic Sequence: SEQ ID NO: 450 /
SNP Position Genomic: 47426 / Related Interrogated SNP: hCV25620145 / SNP
Source: Applera / Population(Allele,Count): Caucasian (C,2|T,32) African
American (C,9|T,13) total (C,11|T,45) / SNP Type: INTRON / SNP Source:
dbSNP; Celera; HapMap; HGBASE / Population(Allele,Count): Caucasian (T,103|C,7)
/ SNP Type: INTRON /
Context (SEQ ID NO: 2668): /
CCCAGTCCAGTTCTCTGCCCGCCCCACCCAAGTGGCACCCAAGTGCCTTCTGGTTCAAGACGAGAACTGGACAACCAGCCAGA
GGCAGAGATTTCCTCTC

Y
GGGCCTAAAGGTCAAACAACTCCAGAGCAGCCTATGCGTCACCAAACTGTGAACAAAGGTGTCGACACTTCCAAGAACCACAG
GGTCTAGCAACTGTGTC / Celera SNP ID: hCV624502 / Public SNP ID: rs666210 / SNP
Chromosome Position: 33723810 / SNP in Genomic Sequence: SEQ ID NO: 450 / SNP
Position Genomic: 51549 / Related Interrogated SNP: hCV1825046 / SNP Source:
dbSNP; HapMap; HGBASE / Population(Allele,Count): Caucasian (T,30|C,90) / SNP
Type: INTRON /
Context (SEQ ID NO: 2669): /
GGTAGGGCTGAGTATCTTTGATTCCAAAGCCCATGCTCATTCCATTTGTCACTCTGTCTGTCTCCCAGTCACCTGTCATACCT
GGGGGGAAGGGGTAATG

R
TGGTTATTACCTTTATTTTACTAATTTATCTATTTCAACTTATTATGCAATATGTAGAACTTTTTTTTTTTTTTTTTTTGAGACG
GAGTCTCGCTCTGTCAC / Celera SNP ID: hCV624503 / Public SNP ID: rs633784 / SNP
Chromosome Position: 33726323 / SNP in Genomic Sequence: SEQ ID NO: 450 / SNP
Position Genomic: 54062 / Related Interrogated SNP: hCV1825046 / SNP Source:
dbSNP; HapMap; HGBASE / Population(Allele,Count): Caucasian (A,30|G,90) / SNP
Type: TFBS SYNONYMOUS;INTRON /

Context          (SEQ ID NO: 2670): /
AAAACTCTGTCTCAAAAAAAAAGAAAAAAAATTCTGTGTGTGTGTATTTCTCATTCTTTTTGATGCTCAAATTGTCCCAAATT
TGACCATTGGTAGTCCT
Y
TCAAATTAATTCCTGTGTCATCTTTGGGACAATCTTAATTTTAAATGGGCCCTGTGGGACTTAAACTTGTCCAACACTATGAT
CGGTTCTGAGTTCAGAA / Celera SNP ID:   hCV1348012 / Public SNP ID:   rs6060230 /
SNP Chromosome Position:   33689308 / SNP in Genomic Sequence:   SEQ ID NO: 450 /
SNP Position Genomic:   17047 / Related Interrogated SNP:   hCV25620145 / SNP
Source:   dbSNP; Celera / Population(Allele,Count):   Caucasian (C,202|T,22) /
SNP Type:   INTERGENIC;UNKNOWN /
Context          (SEQ ID NO: 2671): /
GAATCCTAAATAATCTAGTGTGGATATAAATGAAATAAGGTTGGCCATGAATTCATCATCTTTGAAGCTAGTTTGTTGAAATT
GGGTGATGAGCACACAA
R
GGATGGATGTCCTGTTCGCTCCATTTTTGTTTATTTAAAATTTTACTTAGCCAGGCATGGTGGCATGCACCTGTGGTCCCAGC
TTCTTGGGACGCTGGGG / Celera SNP ID:   hCV1348016 / Public SNP ID:   rs6060246 /
SNP Chromosome Position:   33701107 / SNP in Genomic Sequence:   SEQ ID NO: 450 /
SNP Position Genomic:   28846 / Related Interrogated SNP:   hCV25620145 / SNP
Source:   dbSNP; Celera / Population(Allele,Count):   Caucasian (A,198|G,22) /
SNP Type:   INTERGENIC;UNKNOWN /
Context          (SEQ ID NO: 2672): /
AACTCCATCTCAAAAAGAAAAAAGCAGAAAATAATTGATGTGGTTTGGCTGTGTCCCCACCTAAATCTCACCTTGAATTGTAG
CTCCCATATTTCTCACG
Y
GTTGTGGAAGGGACCTGGTGGGAGATAATTGAATCATGGGGGCCGTTTCCCCCATACTGTTCTCATGGTAGTGAATAAGTCTC
ACAAGATCTGATGGTTT / Celera SNP ID:   hCV1348023 / Public SNP ID:   rs6087677 /
SNP Chromosome Position:   33714805 / SNP in Genomic Sequence:   SEQ ID NO: 450 /
SNP Position Genomic:   42544 / Related Interrogated SNP:   hCV1825046 / SNP
Source:   dbSNP; Celera; HapMap / Population(Allele,Count):   Caucasian
(T,47|C,71) / SNP Type:   INTRON /
Context          (SEQ ID NO: 2673): /
GAAGTGAGGCAGGGCAAGGGAGGCGGCATTTAAGGGGTATGCCATCAGGCAGGTTTCCACCATGGGAACTGGAGTTCAATCTT
GCTGGGGAGCTTAGGAA
Y
TCAGTGTAGAGCTCCAGCTTCGGAGTGATCCCACCCAAGGGGCAGGGAACTGGAATGTTTATCCACCAACTCCCATCAGTATC
AGTTACCGGCTTGAGAG / Celera SNP ID:   hCV1348030 / Public SNP ID:   rs11908683 /
SNP Chromosome Position:   33720920 / SNP in Genomic Sequence:   SEQ ID NO: 450 /
SNP Position Genomic:   48659 / Related Interrogated SNP:   hCV25620145 / SNP
Source:   dbSNP; Celera; HapMap / Population(Allele,Count):   Caucasian
(T,110|C,10) / SNP Type:   INTRON /
Context          (SEQ ID NO: 2674): /
GACAAATTATGATCCCGGACAGGAGCAGGGGGATAGGGATAGTTCTGATACACGCCCAAAGCCTGGGACCTTAGCCAGCACTT
CCCTCTTTCTCCTGGGT
R
TCCTGCTAGAGTCTGAGCCAGAGAAAGATAAATGTCATAACTGGAGGGCCCTGAGCAGCCACCCAGCCCAGATGCTGTCAAAC
ACTGCTCTGCATAACCT / Celera SNP ID:   hCV1348034 / Public SNP ID:   rs2295888 /
SNP Chromosome Position:   33722863 / SNP in Genomic Sequence:   SEQ ID NO: 450 /
SNP Position Genomic:   50602 / Related Interrogated SNP:   hCV25620145 / SNP
Source:   dbSNP; Celera; HapMap; ABI_Val; HGBASE / Population(Allele,Count):
Caucasian (A,110|G,10) / SNP Type:   INTRON;PSEUDOGENE /
Context          (SEQ ID NO: 2675): /
GGGCTGAGCTCAGCGATCCAGCCCACCCGGCTAATCCAGGACTTTTTTTTTTCACTGCGAGAGGCTAAGTGGATCCTGGAGGTG
ATAGTAAGAATTGCGGG
R
AGCGCGGCTCTAGCTCAGCTGGGAGCTTTCTGGTGCCGGTGAGGAGACCATGAAGCCACCAGCCCCAGCTGACCCGCCCGCCG
AGGCCCAGAGAGTCAGA / Celera SNP ID:   hCV1825004 / Public SNP ID:   rs1415771 /
SNP Chromosome Position:   33734493 / SNP in Genomic Sequence:   SEQ ID NO: 450 /
SNP Position Genomic:   62232 / Related Interrogated SNP:   hCV1825046 / SNP
Source:   dbSNP; Celera; HapMap; ABI_Val; HGBASE / Population(Allele,Count):
Caucasian (G,118|A,100) / SNP Type:   INTRON /
Context          (SEQ ID NO: 2676): /
TCTCCATTCCCCTTATTCCCAAGTTTCTAAAATTTGGTCAGCACCTTAGACCACAGCAACACCCATGACTTTGGTCAGTGTGA
CATGCGGTAGAACCAAC

M
CTGGATTTGAAGTCAGATCTAGGATCCAGTTAATTACAACTCTGTCACTCATCTATAACCTGTGTATGTGACCCTCTCCTTCT
CTGGGGCTAGCATCCTT / Celera SNP ID: hCV1825006 / Public SNP ID: rs1124511 /
SNP Chromosome Position: 33736697 / SNP in Genomic Sequence: SEQ ID NO: 450 /
SNP Position Genomic: 64436 / Related Interrogated SNP: hCV1825046 / SNP
Source: dbSNP; Celera; HapMap; HGBASE / Population(Allele,Count): Caucasian
(A,64|C,56) / SNP Type: INTRON /
Context (SEQ ID NO: 2677): /
GGCTGGCGGGGAGAGGGGTGGTTGGTAAATGGGAAAAGGGTGCTTCCTTTGCTGATATCCAAAGCAGATACTTTCTCTGCCTA
TTTGATGATACAAAACT

M
GCCCTACCACACCAGGCTAAGGGAGAGAAGAGGATCCGTGTCCTACAGAATTCCCCCTATTAGCCTACCTGCCCCATAGCCCC
CAAACTCTGCTGCTAAT / Celera SNP ID: hCV1825018 / Public SNP ID: rs11696967 /
SNP Chromosome Position: 33743609 / SNP in Genomic Sequence: SEQ ID NO: 450 /
SNP Position Genomic: 71348 / Related Interrogated SNP: hCV1825046 / SNP
Source: dbSNP; Celera; HapMap / Population(Allele,Count): Caucasian
(A,179|C,47) / SNP Type: INTRON /
Context (SEQ ID NO: 2678): /
CTGCCTGGTAGAGAAGATAATCCCAAAGGTTATGATATTATTACTCTGTAGGACATTAACCCATTTAGTATTTAATTTAGCAA
TCTTACTTCAATATTTT

Y
CTTTTTCCATTGACTATAAAAACTCAAGTTCTTTTTTTCTTTTTTTTTGAGACAGGTCTCACATTGTTGCCCAGGCTGGAGTGCAG
TGGTTCGAACACGGCTC / Celera SNP ID: hCV1825019 / Public SNP ID: rs6088732 /
SNP Chromosome Position: 33744134 / SNP in Genomic Sequence: SEQ ID NO: 450 /
SNP Position Genomic: 71873 / Related Interrogated SNP: hCV1825046 / SNP
Source: dbSNP; Celera; HapMap / Population(Allele,Count): Caucasian
(C,177|T,47) / SNP Type: INTRON /
Context (SEQ ID NO: 2679): /
GACTGGTCTTGAACTCCTGACTTCAAGTGATCCATCCACTTTGGCCTCCCAAAGTGCCGGGATTTACAGGAGTGAGCCACTGC
GCCTGGCCAAGAATTAC

R
TTTTTAACATGCTGACAGTTGTCCTTGACACTAAGTGTCAGACCCTGGATTAAGTAATTTATAGACCCATGATACTCCTTTGA
GATAGTTTTTGTCCCCA / Celera SNP ID: hCV1825021 / Public SNP ID: rs6088733 /
SNP Chromosome Position: 33744904 / SNP in Genomic Sequence: SEQ ID NO: 450 /
SNP Position Genomic: 72643 / Related Interrogated SNP: hCV1825046 / SNP
Source: dbSNP; Celera; HapMap; ABI_Val / Population(Allele,Count): Caucasian
(A,95|G,25) / SNP Type: INTRON /
Context (SEQ ID NO: 2680): /
CCCAGCATGTGATTCCACTATCTGAAGACACAGACGTGCTTTACGTATTTCCATAAATTAACTCAATAAGAACATCCACCAAG
AAGCTGACAGAGTGGTT

M
TAAGGAGAGAAACCGAATAGCTGGAGACAGGGGCAAAAGGGGACTTCACCAATGTCACTGAGTACCCTTTTTTTGTATCCTTTG
ACTTTTTTTTTTTTTAAT / Celera SNP ID: hCV7593265 / Public SNP ID: rs1033799 /
SNP Chromosome Position: 33720033 / SNP in Genomic Sequence: SEQ ID NO: 450 /
SNP Position Genomic: 47772 / Related Interrogated SNP: hCV25620145 / SNP
Source: dbSNP; HapMap; HGBASE / Population(Allele,Count): Caucasian
(C,202|A,24) / SNP Type: INTRON /
Context (SEQ ID NO: 2681): /
ATTGCTAGTGTCTAAGGGCCTGGCGGGTAAGATGTATTTATTCAACAGGTATTAATCTATTACCTACCACATGTAAAGCACTG
TGGGGAACATAAGATGA

R
TAAGGCATGGATTCTGCATTCAATTTGTAGAGCTGTAACTGTTGATGAGAGCTAACTGCAAGAAAGATGCTGAAGGTCCCAAA
TTCTGTAGAAGGATCAA / Celera SNP ID: hCV7593276 / Public SNP ID: rs1535466 /
SNP Chromosome Position: 33718706 / SNP in Genomic Sequence: SEQ ID NO: 450 /
SNP Position Genomic: 46445 / Related Interrogated SNP: hCV1825046 / SNP
Source: dbSNP; HapMap; ABI_Val; HGBASE / Population(Allele,Count): Caucasian
(G,62|A,164) / SNP Type: TRANSCRIPTION FACTOR BINDING SITE;INTRON /
Context (SEQ ID NO: 2682): /
ATCTAGGATCCAGTTAATTACAACTCTGTCACTCATCTATAACCTGTGTATGTGACCCTCTCCTTCTCTGGGGCTAGCATCCT
TGTCTATAAATAATGAG

K
AGTTTGAACAAATGGATCTTTAAGATCCTTTTCGGTCGAGCTTTCTATGATTCTAGGATTGGCTATCTCTTATTTGCTGAAAA
AGTCAGATGGAAGTGAT / Celera SNP ID: hCV11189159 / Public SNP ID: rs6060270 /

SNP Chromosome Position: 33736814 / SNP in Genomic Sequence: SEQ ID NO: 450 / SNP Position Genomic: 64553 / Related Interrogated SNP: hCV1825046 / SNP Source: dbSNP; Celera; HapMap / Population(Allele,Count): Caucasian (G,179|T,47) / SNP Type: INTRON /
Context (SEQ ID NO: 2683): /
GCCCTCACTAATTCCACTTAAGACCTCCAGTTTCTGTCCTAGGATTGGTGACAGCAGAAGAAAACACTGAGAGCTAGAAATCA
TCTAGAGACTTCATACC
R
AGAGACTGGTTAAGTAAATTCCTAGGTCCCAATTAAGAGTCACGATGTTGTCATTTAAATGGACACAATATAAAGAACGCAAA
CTACGCATAGAAGTCTT / Celera SNP ID: hCV11189164 / Public SNP ID: rs3746427 /
SNP Chromosome Position: 33730464 / SNP in Genomic Sequence: SEQ ID NO: 450 /
SNP Position Genomic: 58203 / Related Interrogated SNP: hCV1825046 / SNP
Source: dbSNP; HapMap / Population(Allele,Count): Caucasian (A,103|G,123) /
SNP Type: INTRON /
Context (SEQ ID NO: 2684): /
CTTTTATGGATCATATGTCTGGAATATAAGTTTCATTGCAGAAAACATACAAAATAATGGGGAAAAATAGAACTCTGCCATCC
AGAGGTGACAGCTGTTA
M
GTGGTCTAGAGGTTCGATGCAAATAAGGGCTGTGCTGGCTTCACAAGAATCACTGGAAGTCTGCTAAAAATACTGATTCTAGG
ACCCAACACAGAGATTC / Celera SNP ID: hCV11189166 / Public SNP ID: rs11906318 /
SNP Chromosome Position: 33729442 / SNP in Genomic Sequence: SEQ ID NO: 450 /
SNP Position Genomic: 57181 / Related Interrogated SNP: hCV25620145 / SNP
Source: dbSNP; Celera; HapMap / Population(Allele,Count): Caucasian
(A,204|C,22) / SNP Type: INTRON /
Context (SEQ ID NO: 2685): /
TGCCCTCATGGGGCTTATACTTCTGGGTAAGATCTACCCTTTATTGAGCACTTACCATTGGCCAAGCACTTTCTTTGCACTAT
CTTATTATTCTCGCAGC
W
TTTTTGGGGAAGTTTTTCAAATCTACAGAAAGTTAAAAGATACACCATTAACACTCATATACCCTTTGCTTAGACTTGTCAGC
TACTAAAACTATATTTT / Celera SNP ID: hCV11189205 / Public SNP ID: rs7261312 /
SNP Chromosome Position: 33684909 / SNP in Genomic Sequence: SEQ ID NO: 450 /
SNP Position Genomic: 12648 / Related Interrogated SNP: hCV25620145 / SNP
Source: dbSNP; Celera / Population(Allele,Count): Caucasian (A,110|T,10) /
SNP Type: INTERGENIC;UNKNOWN /
Context (SEQ ID NO: 2686): /
GGGAGGCAGTATAAGATTGCAGCTTAAAGCACAGGCCTTTCACCAATTTTCACCTCTAGGAATTTAATTCACAGTGAACAGCT
GTTTGAGAGTGTTCATG
R
CAGTATTGCTTAGAACAGCAAGAGGCTGGAAAGAACCCAACTGTCCACCCATTAATGGGTGATTAAACTGTGTTATAAACAGT
GCAAAACTATGAGTTAT / Celera SNP ID: hCV11656971 / Public SNP ID: rs2050652 /
SNP Chromosome Position: 33733180 / SNP in Genomic Sequence: SEQ ID NO: 450 /
SNP Position Genomic: 60919 / Related Interrogated SNP: hCV1825046 / SNP
Source: dbSNP / Population(Allele,Count): Caucasian (G,170|A,54) / SNP Type:
INTRON /
Context (SEQ ID NO: 2687): /
GGCTGACCAGTGACTTCCACCACAAGGGAGAGAAAGGTGGCCAGGTTCTGGCATAGAGAAGGTGGCGGAGGTTGTTGCCTAAT
TCCCAATATCTGTTCCC
Y
GCTTCCTCCTTAGTAGCAGGATTCCTAACTTTAGCTAAGCACAGGCTGCCTGGCTGCCAACACTAACTATTCTAACAGCTAGG
TGGGATTAAGGACACCA / Celera SNP ID: hCV11656979 / Public SNP ID: rs2065108 /
SNP Chromosome Position: 33706822 / SNP in Genomic Sequence: SEQ ID NO: 450 /
SNP Position Genomic: 34561 / Related Interrogated SNP: hCV1825046 / SNP
Source: dbSNP; HapMap; HGBASE / Population(Allele,Count): Caucasian
(C,81|T,141) / SNP Type: INTRON /
Context (SEQ ID NO: 2688): /
CGGCCTGGGATCCTTTATCACGAAATGCAGGTTACAGGGGAAGACAGCAGATCCATACACAAAGAAACGCAGTCTCAAACTGG
ATAAGTTGTGTAACTGG
R
TAGGTTGTTGTGAAATTAAATAATGGAAGGGAAAGGGATTAGTAAATTGTGACATCATATTCTCTGTCAGATGAAAACCCTGT
CCTAATTTCTATCCACC / Celera SNP ID: hCV11656982 / Public SNP ID: rs1885115 /
SNP Chromosome Position: 33681256 / SNP in Genomic Sequence: SEQ ID NO: 450 /
SNP Position Genomic: 8995 / Related Interrogated SNP: hCV1825046 / SNP
Source: dbSNP; Celera; HapMap; HGBASE / Population(Allele,Count): Caucasian

(G,36|A,80) / SNP Type: MISSENSE MUTATION /
Context (SEQ ID NO: 2689): /
TGGGATCCCCCGTGGCACGGTAGAGGTACATTGCGCTTTCAATAAGTTCTGCAAAGTCCAAAGCAGACCCTCCTCACTCAGTG
GCTGCACAGAGATGGCC
W
GGGGCCTCTTAGGGCCGTGAGGGACTACTCCCCTCCATATCTTGTCCATAAGGCCCCAACTAGCAGGAACACAGGATACCTCA
CCCAGTGCTGAGTTTCA / Celera SNP ID: hCV16179908 / Public SNP ID: rs2273805 /
SNP Chromosome Position: 33706575 / SNP in Genomic Sequence: SEQ ID NO: 450 /
SNP Position Genomic: 34314 / Related Interrogated SNP: hCV1825046 / SNP
Source: dbSNP; HapMap; HGBASE / Population(Allele,Count): Caucasian
(T,47|A,73) / SNP Type: INTRON /
Context (SEQ ID NO: 2690): /
TTATTGCTTTACTTTCCTGTGCATCTGACCAACTCTGTCGAGCAGTGGTCCCCAACCGTTTTGGCACCAGGGACCAGTTTCAT
GGAAGACAATTTTTCCA
K
GAACTGGGCTTGTAGGAGATGGTTTTGGGATAATTCATGTGCATTACATTTATTGTGCACTTTATTTATATTATTATTACATT
GTAATATATAATGAAAT / Celera SNP ID: hCV27167691 / Public SNP ID: rs2378333 /
SNP Chromosome Position: 33708890 / SNP in Genomic Sequence: SEQ ID NO: 450 /
SNP Position Genomic: 36629 / Related Interrogated SNP: hCV1825046 / SNP
Source: dbSNP / Population(Allele,Count): Caucasian (T,85|G,141) / SNP Type:
TRANSCRIPTION FACTOR BINDING SITE;INTRON /
Context (SEQ ID NO: 2691): /
ACCCCATCTAAACAAAAAATTTAAAAATTAGCCAGGTGTGGTGGTGTGGGCCTGTAGTCCCAGCTATTCGGGAGGCTGAGGCG
GGAGAATCACTTGTGCC
Y
GTGAGGCTGCAGTGAACTGTGATCACCCCACTGGGTGACAGGGTGAGACGCTGTCTCAAAATACATAAATACACAAATAAAAA
TAAAAACAAAAGTAAAA / Celera SNP ID: hCV27167696 / Public SNP ID: rs6088716 /
SNP Chromosome Position: 33700499 / SNP in Genomic Sequence: SEQ ID NO: 450 /
SNP Position Genomic: 28238 / Related Interrogated SNP: hCV1825046 / SNP
Source: dbSNP; Celera; HapMap / Population(Allele,Count): Caucasian
(C,43|T,69) / SNP Type: INTERGENIC;UNKNOWN /
Context (SEQ ID NO: 2692): /
CCAACTAAACAAGGAAGAGGAGGGGGATGGGGAGGGGGACAAGAAGAAGAAGGAGAAAATAGTCACGTGTATTGATAATAGCG
AACATTTACATAGCAAG
W
AATGTTCTTTGTACTTTACATCCATCAACACCATTAATCCTTACCACAACCCTCTGAGAAAGTGTCATTATCACCATTTTATA
AACCAAAAACCTGAAGC / Celera SNP ID: hCV27833500 / Public SNP ID: rs17092385 /
SNP Chromosome Position: 33739254 / SNP in Genomic Sequence: SEQ ID NO: 450 /
SNP Position Genomic: 66993 / Related Interrogated SNP: hCV1825046 / SNP
Source: Applera / Population(Allele,Count): Caucasian (A,6|T,32) African
American (A,4|T,30) total (A,10|T,62) / SNP Type: INTRON / SNP Source:
dbSNP; HapMap / Population(Allele,Count): Caucasian (T,107|A,13) / SNP Type:
INTRON /
Context (SEQ ID NO: 2693): /
CGCTAAACTCACCTTTTCAAAGTCACCTATTCAAACAGATCAAAGATCTCCACTGTGGGAGGAATGCCAAGGGCCGGGGTCAA
GATCCCAAGGAACTAAT
Y
CTAGCTCTACCTCTACTAGCTGTGAGATCTTTAGCAAGGCACTTAGCTTCTCTGTTCTTTGGTCTCCCCATCTGTAAAATATG
AGCATTATGTATATCTC / Celera SNP ID: hCV29373050 / Public SNP ID: rs6088722 /
SNP Chromosome Position: 33706187 / SNP in Genomic Sequence: SEQ ID NO: 450 /
SNP Position Genomic: 33926 / Related Interrogated SNP: hCV1825046 / SNP
Source: dbSNP; HapMap / Population(Allele,Count): Caucasian (T,63|C,163) /
SNP Type: TRANSCRIPTION FACTOR BINDING SITE;UTR3;INTRON /
Context (SEQ ID NO: 2694): /
CCTTTGACCCTTTCAATCTATTCTTCACAAAGCAGCCAAAGCGATCTTTCTAAAATACAAATCAGATCATGTCACTGCTCTGC
TGTAAATCCTACCAAGG
S
TTCACATTCCAAGTAGAAAAAAAAACCCAAACTCTTTCCTGTGGCTTACACAAAACCCTACATGATCTAGCCCCTGCCTACCT
CTGGGACCTTATCTCTT / Celera SNP ID: hCV29373051 / Public SNP ID: rs6142300 /
SNP Chromosome Position: 33705708 / SNP in Genomic Sequence: SEQ ID NO: 450 /
SNP Position Genomic: 33447 / Related Interrogated SNP: hCV1825046 / SNP
Source: dbSNP; HapMap / Population(Allele,Count): Caucasian (G,63|C,163) /
SNP Type: INTRON /

Context (SEQ ID NO: 2695): /
CTAAGGGATAATCCGTTCCTTGCCTCTCTAGATTCTCCTGACTGCTGGCATTCCTTGGCTTATGGTTGTATCACTCCAGTATC
TGCCCGTGCCTTCATAT
Y
GCCTTCTCTGTGGATGTGTCACATCTCCTTCTGCTTTTCTCTTATAAGGACATTTATAATTGCATTTAGGACCCCCCCCGCGC
CCCCCCCAACCCAGATA / Celera SNP ID: hCV32066710 / Public SNP ID: rs11907010 /
SNP Chromosome Position: 33737661 / SNP in Genomic Sequence: SEQ ID NO: 450 /
SNP Position Genomic: 65400 / Related Interrogated SNP: hCV25620145 / SNP
Source: dbSNP; HapMap / Population(Allele,Count): Caucasian (C,204|T,22) /
SNP Type: INTRON /
Context (SEQ ID NO: 2696): /
AAGAAAAAAAATTCTGTGTGTGTGTATTTCTCATTCTTTTTGATGCTCAAATTGTCCCAAATTTGACCATTGGTAGTCCTCTC
AAATTAATTCCTGTGTC
R
TCTTTGGGACAATCTTAATTTTTAAAATGGGCCCTGTGGGACTTAAACTTGTCCAACACTATGATCGGTTCTGAGTTCAGAAAAT
AGTCTCTCTAAATTTTT / Celera SNP ID: hCV29530378 / Public SNP ID: rs6058179 /
SNP Chromosome Position: 33689328 / SNP in Genomic Sequence: SEQ ID NO: 450 /
SNP Position Genomic: 17067 / Related Interrogated SNP: hCV1825046 / SNP
Source: dbSNP / Population(Allele,Count): Caucasian (A,64|G,162) / SNP Type:
INTERGENIC;UNKNOWN /
Context (SEQ ID NO: 2697): /
CATGTTGCCCAGGCTGGTCTCAAATCCCTGGGCTCAAGCAATTCCTCCTACCTTGGCCTCCCAAAGTGCCAGGATAACAGGCG
TGAGACACACACCAAGC
Y
CCTACAGTATCTGTATAACATAAGTCTTCCTGTCAATTCTCTACTCACAACTTTCTGTGAGCCCTCCATTTCATGCAGGATAA
AAATGCAAGTCATGACC / Celera SNP ID: hCV30612290 / Public SNP ID: rs6060250 /
SNP Chromosome Position: 33704108 / SNP in Genomic Sequence: SEQ ID NO: 450 /
SNP Position Genomic: 31847 / Related Interrogated SNP: hCV1825046 / SNP
Source: dbSNP; HapMap / Population(Allele,Count): Caucasian (T,36|C,82) / SNP
Type: UTR5;INTRON /
Context (SEQ ID NO: 2698): /
GAACTAGTTCAATTAAAATGCTACAATTATTGCACATCCAAAATTTTTTATCTGATTATAAACTTTCTGAAAAACTGCACTGC
AAATTCCTAACCAAAAC
Y
CAGTTGAAAGTCATGGCAATATTGCAAAAAAAAAAAAAAAAAAAAAAAAAAAATTCCACTCTTCAATTCACAACTAACTACAAACT
ATTTTGATATAAAATTA / Celera SNP ID: hCV30378437 / Public SNP ID: rs6088713 /
SNP Chromosome Position: 33693857 / SNP in Genomic Sequence: SEQ ID NO: 450 /
SNP Position Genomic: 21596 / Related Interrogated SNP: hCV1825046 / SNP
Source: dbSNP; HapMap / Population(Allele,Count): Caucasian (T,63|C,163) /
SNP Type: INTERGENIC;UNKNOWN /
Context (SEQ ID NO: 2699): /
GCAAGGCCACTGCTGTAACCCTTGGGCCTAGCAGAGTGCCTGGCATGTGAAAGACATTCAAGAAATATCTGTGAGACTAAGCA
GGACTGCTTTGTTAAGG
M
GTCCCAGGTTTGAATATAGGGCTATGGGACTCTGGATTCCCCTTAAAATCTATTTTAGTGTCTCACAAAACTTTTCGCTAAAC
TCACCTTTTCAAAGTCA / Celera SNP ID: hCV29837748 / Public SNP ID: rs6088721 /
SNP Chromosome Position: 33706011 / SNP in Genomic Sequence: SEQ ID NO: 450 /
SNP Position Genomic: 33750 / Related Interrogated SNP: hCV1825046 / SNP
Source: dbSNP; HapMap / Population(Allele,Count): Caucasian (A,86|C,140) /
SNP Type: UTR3;INTRON /
Context (SEQ ID NO: 2700): /
CACAGGGAAGCAGCTGACCCTGACATCTTCATTGGATGTCTCATGGACAGAACAAACTTGGCACTTCCTAAACAAAATTCTTG
GTTCCCTTACTTTCCCA
Y
GAAATTGCCTCCCTACTTTGGCCTTTTTCCCTTCTTGGTAAATGACACAATGTCTACCTGGTTATTCTGGATAGAAACCCGGT
GGCCACTTTTTTTTTTTT / Celera SNP ID: hCV29620866 / Public SNP ID: rs6088724 /
SNP Chromosome Position: 33712915 / SNP in Genomic Sequence: SEQ ID NO: 450 /
SNP Position Genomic: 40654 / Related Interrogated SNP: hCV1825046 / SNP
Source: dbSNP; HapMap / Population(Allele,Count): Caucasian (C,47|T,73) / SNP
Type: INTRON /
Context (SEQ ID NO: 2701): /
CTACTTTTTTCTTAAAACTTTTAAAAAACGTAGATTATAAAAACATTTTGGTACATGCACTATTTTGGAACACCAAAGTGGGGTA
GATTAGATAAGCTCTAA

EP 2 635 709 B1

R
ATCCTTCCAGCTGCAACTCTACCAGACCATGTAATGGAAAAACAACGAGATGGCACAGCGGCACAAGCTGCTTCCTGCCCTTC
CTGAGGCCCACTGTCAA / Celera SNP ID: hCV30342055 / Public SNP ID: rs6088727 /
SNP Chromosome Position: 33713639 / SNP in Genomic Sequence: SEQ ID NO: 450 /
SNP Position Genomic: 41378 / Related Interrogated SNP: hCV1825046 / SNP
Source: dbSNP; HapMap; ABI_Val / Population(Allele,Count): Caucasian
(G,85|A,141) / SNP Type: INTRON /
Context (SEQ ID NO: 2702): /
TAAAAACATTTTGGTACATGCACTATTTTGGAACACCAAAGTGGGGTAGATTAGATAAGCTCTAAGATCCTTCCAGCTGCAAC
TCTACCAGACCATGTAA

Y
GGAAAAACAACGAGATGGCACAGCGGCACAAGCTGCTTCCTGCCCTTCCTGAGGCCCACTGTCAAGAATGAGATTGGACAGAA
ACGCCCAGTGGAGAAAC / Celera SNP ID: hCV29837747 / Public SNP ID: rs6088728 /
SNP Chromosome Position: 33713674 / SNP in Genomic Sequence: SEQ ID NO: 450 /
SNP Position Genomic: 41413 / Related Interrogated SNP: hCV1825046 / SNP
Source: dbSNP / Population(Allele,Count): Caucasian (T,85|C,141) / SNP Type:
INTRON /
Context (SEQ ID NO: 2703): /
CCGTCAGTTCTAGGAATTCATGACACTTCTCTCCTCCCAATTTGGGATGTGGTTACACAATCATGGTCCCTTCAGTCTCCATC
TCAAACCGGAAGCTTTA

Y
CCCTGTCAGCCCTGTAGAATGTCTTGGGTAAGAATGAATACCCTGGTTGGGCGCGGTGGCTCACGCCTGTAATCCCAACACTT
TGGGAGGTTGAGGTGCG / Celera SNP ID: hDV70862720 / Public SNP ID: rs17092378 /
SNP Chromosome Position: 33736188 / SNP in Genomic Sequence: SEQ ID NO: 450 /
SNP Position Genomic: 63927 / Related Interrogated SNP: hCV1825046 / SNP
Source: dbSNP; HapMap / Population(Allele,Count): Caucasian (C,172|T,54) /
SNP Type: MISSENSE MUTATION;INTRON /
Context (SEQ ID NO: 2704): /
TCCACACAAGGCACTCTAATATTTTCGAATCCATTCTATTTCATTATTTAAAAAGTTAGTGGGACACATTAAATTTATTTCTT
GACCCACTAAAGGGTCA

Y
GAGCAAAGTCTCAAAAACACTACTGTAAGACACTAAACTATTAACTATTGCAAGGGAGTTTGTAAAAAATACAATAAACAGAA
TAGCTTACCTCTCTGCA / Celera SNP ID: hCV30342057 / Public SNP ID: rs6060239 /
SNP Chromosome Position: 33694580 / SNP in Genomic Sequence: SEQ ID NO: 450 /
SNP Position Genomic: 22319 / Related Interrogated SNP: hCV25620145 / SNP
Source: dbSNP; HapMap; ABI_Val / Population(Allele,Count): Caucasian
(T,194|C,32) / SNP Type: INTERGENIC;UNKNOWN /
Context (SEQ ID NO: 2705): /
TATCCCTGCCTATCAAGGTAGCCAGAGGTTTGCCATGGTACTGAAGAAAATGATCAGCAGCTGAATTAGCCAAGGTTCATGGG
CAAAGATGAGGCTGGCA

R
ATAAAGCCAGACAAATTCCTGGTTAAAAGTCACCTCCTGAGCTATTTAGAGAAACAGTTGATTCAGGGCTGGGATAGGAACGG
GATCAGCTGAGCTTGGA / Celera SNP ID: hCV30540318 / Public SNP ID: rs6060244 /
SNP Chromosome Position: 33699435 / SNP in Genomic Sequence: SEQ ID NO: 450 /
SNP Position Genomic: 27174 / Related Interrogated SNP: hCV25620145 / SNP
Source: dbSNP; HapMap; ABI_Val / Population(Allele,Count): Caucasian
(G,202|A,22) / SNP Type: TRANSCRIPTION FACTOR BINDING SITE;INTERGENIC;UNKNOWN /
Context (SEQ ID NO: 2706): /
CAGGTATTCTCTGACCCACTTAATGCACCTGGGAGGGTTGGCTGATGTAGCTCAATTGTAATTAAATACATCACCTATAGTCT
GGCATTCTCCTCTACCA

Y
TGGGAGGCAGTATAAGATTGCAGCTTAAAGCACAGGCCTTTCACCAATTTTCACCTCTAGGAATTTAATTCACAGTGAACAGC
TGTTTGAGAGTGTTCAT / Celera SNP ID: hCV29674976 / Public SNP ID: rs6060266 /
SNP Chromosome Position: 33733078 / SNP in Genomic Sequence: SEQ ID NO: 450 /
SNP Position Genomic: 60817 / Related Interrogated SNP: hCV1825046 / SNP
Source: dbSNP; HapMap / Population(Allele,Count): Caucasian (T,86|C,16) / SNP
Type: INTRON /
Context (SEQ ID NO: 2707): /
TAGGCAAAGAAGATGCCATGTGCAATGGCCAGGAGATAAGAAATCCAGGGCTACATACACAGAATGCAGCACGCTTCAGTTGG
CTTTTGCAAGAAGGCAT

S
AAGTGCTGTAGTGGGCATGAAGTGGGCAGTGTAAGTCCATAGTGACTCTGAATTCAGAACTGATTCTGCAGGCACTCAGGCCA
GGCGTGGTGGCTCACAC / Celera SNP ID: hCV30576442 / Public SNP ID: rs6120843 /

490

SNP Chromosome Position: 33718993 / SNP in Genomic Sequence: SEQ ID NO: 450 / SNP Position Genomic: 46732 / Related Interrogated SNP: hCV25620145 / SNP Source: dbSNP; HapMap / Population(Allele,Count): Caucasian (C,202|G,24) / SNP Type: INTRON /
Context (SEQ ID NO: 2708): /
ATCTGAATGACAAGCAGCAGCCAGTGATGGGAGGAAAATGGGGAGTGTATTTCAGGCACGGGGCACACCAACTACAAAGACTT
AAGTTTTAGGAGTGTCA
S
ACTCCTTCCTGGATCTGAATGCCCACCTACATAATGAAAGGGGTGGGTTTTCTAATTCTTGGAGCCCTTCCACGGTATCTCGT
TACTCCAGAAATTGCCT / Celera SNP ID: hCV29693169 / Public SNP ID: rs6058192 /
SNP Chromosome Position: 33734261 / SNP in Genomic Sequence: SEQ ID NO: 450 /
SNP Position Genomic: 62000 / Related Interrogated SNP: hCV1825046 / SNP
Source: dbSNP / Population(Allele,Count): Caucasian (C,86|G,30) / SNP Type:
INTRON /
Context (SEQ ID NO: 2709): /
ACTACTGTAAGACACTAAACTATTAACTATTGCAAGGGAGTTTGTAAAAAATACAATAAACAGAATAGCTTACCTCTCTGCAT
GCAGAATGTTTTCTTTG
M
TAACTTTATTTTTCAAGGCTGGATTTATACCACACTGGGCTTGCAAACTTGCTGCTTCATGGGTTGGTGGGGAGGGGTAGGAG
AGGGGTGTGTGGGGTAT / Celera SNP ID: hCV29729417 / Public SNP ID: rs6060240 /
SNP Chromosome Position: 33694699 / SNP in Genomic Sequence: SEQ ID NO: 450 /
SNP Position Genomic: 22438 / Related Interrogated SNP: hCV25620145 / SNP
Source: dbSNP; HapMap / Population(Allele,Count): Caucasian (C,101|A,13) /
SNP Type: INTERGENIC;UNKNOWN /
Context (SEQ ID NO: 2710): /
AACTTTATTTTTCAAGGCTGGATTTATACCACACTGGGCTTGCAAACTTGCTGCTTCATGGGTTGGTGGGGAGGGGTAGGAGA
GGGGTGTGTGGGGTATA
Y
ATGTTTTCTTTTTTATTTTATGGTCTTTTTAAAAAAATTGAGATATAAATTCATACACCATAAAATTCACACTATTAAAGTACAA
TTCAGTGGTTTTTAGTA / Celera SNP ID: hCV29765455 / Public SNP ID: rs6058181 /
SNP Chromosome Position: 33694801 / SNP in Genomic Sequence: SEQ ID NO: 450 /
SNP Position Genomic: 22540 / Related Interrogated SNP: hCV25620145 / SNP
Source: dbSNP; HapMap / Population(Allele,Count): Caucasian (T,105|C,15) /
SNP Type: INTERGENIC;UNKNOWN /
Context (SEQ ID NO: 2711): /
TAAATTAAAAAAAAAAAAAAACTAAGAAAAGAAATATTTGAAAGATGAAGGTCCCCATTTTATCCTCACTATAGCTCCAGGCAG
GGGGTCTCTACTTCAAC
Y
TCTGAAATAGCTCCCACACCTATATACAACCATCAGTTACCACAATAACCTGCCTTGCTTTTGTGGCTCCAGAATATTCCAGC
AGCCTTGTATCTGGTGA / Celera SNP ID: hCV30180146 / Public SNP ID: rs6060216 /
SNP Chromosome Position: 33678078 / SNP in Genomic Sequence: SEQ ID NO: 450 /
SNP Position Genomic: 5817 / Related Interrogated SNP: hCV1825046 / SNP
Source: dbSNP; ABI_Val / Population(Allele,Count): Caucasian (T,46|C,74) /
SNP Type: INTRON /
Context (SEQ ID NO: 2712): /
GAGACAGGGGCAAAAGGGGACTTCACCAATGTCACTGAGTACCCTTTTTTTGTATCCTTTGACTTTTTTTTTTTTTTAATTGTTCA
GTCTCTGTAGAGACTGT
K
AAAAATTGGCAATGCCGGCCAGGCGCGGTGGCTCATGCCTATAATCCCAGCACGTTGGGAGGCTGAGGGGGGCAAAATCACTG
GAGGTCAGAAGTCCGAG / Celera SNP ID: hCV30270043 / Public SNP ID: rs6060257 /
SNP Chromosome Position: 33720157 / SNP in Genomic Sequence: SEQ ID NO: 450 /
SNP Position Genomic: 47896 / Related Interrogated SNP: hCV25620145 / SNP
Source: dbSNP; HapMap / Population(Allele,Count): Caucasian (G,202|T,24) /
SNP Type: INTRON /
Context (SEQ ID NO: 2713): /
CTGAGCTTGGAACATCTTGTAATACCAGAAAGCAGGACACTATCAAAGACTATTGGGGTTGTGTTAACAGGACTCAGGGCTAA
CTTGAAGAGAGGCTTCC
R
CTGGCCAGAAATGGGACAATTTGAGCTTTGATAAGAAAAATAATTTCAATGAACTGAAAACCATCAAATGAGTTTAAATCCAA
GAGTTCACAATACTTGA / Celera SNP ID: hCV30342056 / Public SNP ID: rs6060245 /
SNP Chromosome Position: 33699625 / SNP in Genomic Sequence: SEQ ID NO: 450 /
SNP Position Genomic: 27364 / Related Interrogated SNP: hCV25620145 / SNP
Source: dbSNP; HapMap / Population(Allele,Count): Caucasian (A,109|G,9) / SNP

Type:    TRANSCRIPTION FACTOR BINDING SITE;INTERGENIC;UNKNOWN /
Context                    (SEQ ID NO: 2714): /
CAACTCCCAAAGCAGGGCTTGCACAATACATACTTTTGACTTGCCAAAAAATAAGTTTATCCCTGGGAAATTTCCCATCCATC
TGTTGCTTTGTAGAAAT
R
CTTGAACTTTGCATGCAGTATGTCCAAACATTTCGATCGACTCCCTGGTTAGAGTCTCAGACCTCGTTATTATTATTATTATT
ATTTGAGATGAGAGTTT / Celera SNP ID:    hCV30360384 / Public SNP ID:    rs6058194 /
SNP Chromosome Position:    33739831 / SNP in Genomic Sequence:    SEQ ID NO: 450 /
SNP Position Genomic:    67570 / Related Interrogated SNP:    hCV1825046 / SNP
Source:    dbSNP; HapMap / Population(Allele,Count):    Caucasian (G,95|A,25) / SNP
Type:    INTRON /
Context                    (SEQ ID NO: 2715): /
AATAAAAAAGAGACCTGACAATGAGGCTCTTACATGCATGGAGGCAGATTCAGAGTCTGGGATCCTATGTTGATGTGGCCCTC
ACTAATTCCACTTAAGA
Y
CTCCAGTTTCTGTCCTAGGATTGGTGACAGCAGAAGAAAACACTGAGAGCTAGAAATCATCTAGAGACTTCATACCAAGAGAC
TGGTTAAGTAAATTCCT / Celera SNP ID:    hCV30396340 / Public SNP ID:    rs6120849 /
SNP Chromosome Position:    33730387 / SNP in Genomic Sequence:    SEQ ID NO: 450 /
SNP Position Genomic:    58126 / Related Interrogated SNP:    hCV1825046 / SNP
Source:    dbSNP; HapMap; ABI_Val / Population(Allele,Count):    Caucasian
(C,178|T,48) / SNP Type:    INTRON /
Context                    (SEQ ID NO: 2716): /
CAGAATGGATAAACAAAATATAATACACTATTTGCAAAGATGTATCTAATCATAAAACTACAGAAAGATGGAAAGGATAGAA
AAAGATACATGGGAAAA
K
GTCAACCAAAAGAAAGCTGGTATCACTTATATTCCTATCAGAAATAAAGGTATAAGAATGACCTTCATTTACAGATGACAAAC
TGGGCTCAGAAGCTGGG / Celera SNP ID:    hDV71898298 / Public SNP ID:    rs7361656 /
SNP Chromosome Position:    33729147 / SNP in Genomic Sequence:    SEQ ID NO: 450 /
SNP Position Genomic:    56886 / Related Interrogated SNP:    hCV1825046 / SNP
Source:    dbSNP; HapMap / Population(Allele,Count):    Caucasian (T,173|G,53) /
SNP Type:    TRANSCRIPTION FACTOR BINDING SITE;INTRON /
Context                    (SEQ ID NO: 2717): /
GAATGCTTGGACAGGCTCAGTATGTTGGAACAACTTCTAATTTAATTCTCTAAGGATGTAAAAAAAAAAAATCTTGGTTAGATT
GGCCAACATTGATATGA
Y
TGGCCAATTTCAATATGCTAAAGTAAATGATGTGGTCAGCTGTAATCCCTGAAAATTTGTTTTCAAGAAGTTGATTATCACTG
TTTTTCCCCCAACATAT / Celera SNP ID:    hDV72054460 / Public SNP ID:    rs8117100 /
SNP Chromosome Position:    33692261 / SNP in Genomic Sequence:    SEQ ID NO: 450 /
SNP Position Genomic:    20000 / Related Interrogated SNP:    hCV25620145 / SNP
Source:    dbSNP; HapMap / Population(Allele,Count):    Caucasian (T,110|C,10) /
SNP Type:    INTERGENIC;UNKNOWN //

Gene Number:    114 / Gene Symbol:    TP53INP2 - 58476 / Gene Name:    tumor protein
p53 inducible nuclear protein 2 / Chromosome:    20 / OMIM NUMBER: / OMIM
Information: // Genomic Sequence (SEQ ID NO: 451): // / SNP Information /
Context                    (SEQ ID NO: 2718): /
ACTCTAGCAGTGTAGTCAAATCATTTATATCCATGTCTTAGAACCAACTGGGTGGTAAAGAACTGGGTCAATAATAGTCTTTT
AAGTGACTGTCCAACTA
Y
GTCACTGGGGGAACATTTAAATTTATAACCTAATAAAGGGATTTGTTTTTCAGCCTGGTTGCCTCATTTTGACCTTAAGCAAA
TTTGACCAAATCTACAG / Celera SNP ID:    hCV1207909 / Public SNP ID:    rs6119512 /
SNP Chromosome Position:    33306904 / SNP in Genomic Sequence:    SEQ ID NO: 451 /
SNP Position Genomic:    24756 / Related Interrogated SNP:    hCV1825046 / SNP
Source:    dbSNP; Celera; HapMap; ABI_Val / Population(Allele,Count):    Caucasian
(T,67|C,53) / SNP Type:    TRANSCRIPTION FACTOR BINDING SITE;INTRON /
Context                    (SEQ ID NO: 2719): /
GATCCCCTCCCTCCGGCTCCAGACTCCGCCCCCAGGGACTAACAGAATAACATCCTCCTAATCCCTGATTTGTGTGCAGCGCC
TTAGCTGATGTCACTCC
Y
AGACCCAAAATGAAGGGTCTTAGACCCCAGTGCATGGAAGGGGCAACAGGCCCAGAGAGGAGGATTAGCTCTCTCGAGGTCAC
ATAACCAATTAGAGTCA / Celera SNP ID:    hCV1207914 / Public SNP ID:    rs910870 /
SNP Chromosome Position:    33292893 / SNP in Genomic Sequence:    SEQ ID NO: 451 /
SNP Position Genomic:    10745 / Related Interrogated SNP:    hCV1825046 / SNP

Source: dbSNP; HapMap; ABI_Val / Population(Allele,Count): Caucasian (T,67|C,53) / SNP Type: INTRON /
Context (SEQ ID NO: 2720): /
TCTTTGCAACCTGAGAAGGAGTCAGACGGCGCTGCTGGGCCTAGGTTGAGGGGGTCGTCAGGAGTTCAGGGCTGCATGGGACT
CCAGGTGGTTGGGACAG
Y
ACCAGATGGTGGTGGGATCCCCTCCCTCCGGCTCCAGACTCCGCCCCCAGGGACTAACAGAATAACATCCTCCTAATCCCTGA
TTTGTGTGCAGCGCCTT / Celera SNP ID: hCV1207915 / Public SNP ID: rs910869 /
SNP Chromosome Position: 33292777 / SNP in Genomic Sequence: SEQ ID NO: 451 /
SNP Position Genomic: 10629 / Related Interrogated SNP: hCV1825046 / SNP
Source: dbSNP; HapMap; HGBASE / Population(Allele,Count): Caucasian (C,130|T,96) / SNP Type: INTRON /
Context (SEQ ID NO: 2721): /
ACCTCAGTTTCCTCACCTATAAAATGAGGGGTTTGGATGAGTTGAGGTCTGAAGCCTCCTCCAGCCCAGGTAATAAATCTTTG
TAGTGGACAGGAGGAAT
R
GGTCCTAGGGGTTTCAGGAGGGGACTTCCATGGGTACCTCTGACTCTACAGAGAAATGGAAGACTGAGATCTGACTTAGCAGC
CTAGAAAGACTTAGCAG / Celera SNP ID: hCV1265109 / Public SNP ID: rs6058108 /
SNP Chromosome Position: 33288655 / SNP in Genomic Sequence: SEQ ID NO: 451 /
SNP Position Genomic: 6507 / Related Interrogated SNP: hCV1825046 / SNP
Source: dbSNP / Population(Allele,Count): Caucasian (A,130|G,96) / SNP Type: INTRON /
Context (SEQ ID NO: 2722): /
AATTATTCTTTAATTTGGCTCCAAGAGCTTTAATACTGAAAAACCAAAAGAGCTACAGTACTCATTTCTGATTTAAAAGGTAA
CTGTGATACTACAATAT
R
TATTTTTTGGTTTTTGTCCCACGTTCCTGGCTCACAGCTCCTATAACTCTTGTAATTTCCTAAGTGAGCCATCTTTTGTTAAA
ATATTTGGCCTTTTGCC / Celera SNP ID: hCV27167022 / Public SNP ID: rs6060013 /
SNP Chromosome Position: 33308809 / SNP in Genomic Sequence: SEQ ID NO: 451 /
SNP Position Genomic: 26661 / Related Interrogated SNP: hCV1825046 / SNP
Source: dbSNP; Celera / Population(Allele,Count): Caucasian (A,126|G,98) / SNP Type: INTRON /
Context (SEQ ID NO: 2723): /
CCATATACTCAGCAGGCTGAGGAACAAAGAATTGCTCGGGCCCAGGAGGCAGAGGTTGCAGTGAGCCGAGATTGCACTTGGGT
AACACAGCAAGACTCTT
Y
CTCAAAAAAAAAAAAAAAAAAAAAAAAAAAAAAAAATCGGCCGGGCGCGGTGGCTCACACCTGTAATCCCAGCACTTTGGGAGGCCG
AGGCAGGCGGATCACGA / Celera SNP ID: hCV27167045 / Public SNP ID: rs2378252 /
SNP Chromosome Position: 33283403 / SNP in Genomic Sequence: SEQ ID NO: 451 /
SNP Position Genomic: 1255 / Related Interrogated SNP: hCV1825046 / SNP
Source: dbSNP; Celera; HGBASE / Population(Allele,Count): Caucasian (T,115|C,105) / SNP Type: INTERGENIC;UNKNOWN /
Context (SEQ ID NO: 2724): /
TCAAGGCCTCGATATTCCCTTCCTTCAAGAGGGGCCAAACTTTCTGGGCTTTTGGTAATGGAGTCACTAGCTCCCTGTGGCTA
CATATGGGTTGGGATAC
R
TGAATTGTGCTGGGTGGACACTAGTTGGGGGTGGCCCCACCTTCTTAATCCCTGTAGATCTCCCACCCCCATCCCACCAGAGA
AGCCAGCATCTTAAGCC / Celera SNP ID: hCV29372834 / Public SNP ID: rs6060001 /
SNP Chromosome Position: 33294353 / SNP in Genomic Sequence: SEQ ID NO: 451 /
SNP Position Genomic: 12205 / Related Interrogated SNP: hCV1825046 / SNP
Source: dbSNP / Population(Allele,Count): Caucasian (A,119|G,107) / SNP Type: INTRON /
Context (SEQ ID NO: 2725): /
TCTGCAGTAGGGGGCCTCCCTCTTCATCTTCACCCAGGGAAGGATTGGTTAAGGGCTTACCTCGGTCTGGGTCCAACCCTCAG
TGCTCATCCTTAAGGGC
Y
CCTGGGAACTGACAGCTCCCTCCCTTCATCATCACCTCCCACTGACCTGCTGTCTCACTGAGCCCAAGCAACCTGATCTTCCT
CCTACCCCTGGATGCTG / Celera SNP ID: hCV29693120 / Public SNP ID: rs6060003 /
SNP Chromosome Position: 33294945 / SNP in Genomic Sequence: SEQ ID NO: 451 /
SNP Position Genomic: 12797 / Related Interrogated SNP: hCV1825046 / SNP
Source: dbSNP; HapMap / Population(Allele,Count): Caucasian (T,119|C,107) /
SNP Type: TRANSCRIPTION FACTOR BINDING SITE;INTRON /
Context (SEQ ID NO: 2726): /

GATTTTGCCATGTTGGCCAGGCTGGTATCGAACTCCTGGGCTTAAGCAATCCTCCTGCCTTGGCCTCCCAAAGTGCTATGATT
ACTGGCCTGCACTGCCG
Y
GCCCAGCTCTGCTGCCTCTTTTTACAGCCTGCCGGCCCAGACCAGAGTCTCAGAATACTAAGCCCACTTTCTGGGGCCAGGGT
CAGTAGATAGCTTTGCT / Celera SNP ID:  hCV29674936 / Public SNP ID:  rs6087618 /
SNP Chromosome Position:  33288407 /  SNP in Genomic Sequence:  SEQ ID NO: 451 /
SNP Position Genomic:  6259 / Related Interrogated SNP:  hCV1825046 / SNP
Source:  dbSNP / Population(Allele,Count):  Caucasian (C,120|T,98) / SNP Type:
  INTRON /
Context            (SEQ ID NO: 2727): /
CAGCTCTGCTGCCTCTTTTTACAGCCTGCCGGCCCAGACCAGAGTCTCAGAATACTAAGCCCACTTTCTGGGGCCAGGGTCAG
TAGATAGCTTTGCTGCT
S
TATGATTCTAAGCAAGTATCTTGACCTGCATGTGTCTCAGTGCACCTCAGTTTCCTCACCTATAAAATGAGGGGTTTGGATGA
GTTGAGGTCTGAAGCCT / Celera SNP ID:  hCV29783257 / Public SNP ID:  rs6087619 /
SNP Chromosome Position:  33288511 /  SNP in Genomic Sequence:  SEQ ID NO: 451 /
SNP Position Genomic:  6363 / Related Interrogated SNP:  hCV1825046 / SNP
Source:  dbSNP; HapMap; ABI_Val / Population(Allele,Count):  Caucasian
(G,130|C,96) / SNP Type:  TRANSCRIPTION FACTOR BINDING SITE;INTRON /
Context            (SEQ ID NO: 2728): /
ACCACTCTCAACTCTCAGCTTGTAGAGGTCCCTCTTGGGTCCTCCTCTTGTGGCCCTCTCCACACATAGCATTTGGTCCTTCA
TGGGAGCACCAGCTGCT
S
GAAAAAATGATTCTGTTAGGTAAAAATACTATACATTTTTGAAACATGCACCAATTATTTAGCTCTGTGATTGTGGGCAATTT
CTTCAAGCAGCCTGAGC / Celera SNP ID:  hCV29982073 / Public SNP ID:  rs6088580 /
SNP Chromosome Position:  33285053 / SNP in Genomic Sequence:  SEQ ID NO: 451 /
SNP Position Genomic:  2905 / Related Interrogated SNP:  hCV1825046 / SNP
Source:  dbSNP; HapMap / Population(Allele,Count):  Caucasian (G,48|C,48) / SNP
Type:  ACCEPTOR SPLICE SITE;INTRON //

Gene Number:  115 / Gene Symbol:  HAPLN2 - 60484 / Gene Name:  hyaluronan and
proteoglycan link protein 2 / Chromosome:  1 / OMIM NUMBER: / OMIM Information:
// Genomic Sequence (SEQ ID NO: 452): //  / SNP Information /
Context            (SEQ ID NO: 2729): /
AGGGGAGATGGGGATGCTGCTTAGATGAGGCGCATATGGAAAGGGAGGCAGCTCGAGCTTGGCAGTTATGGATCTTTGAGCCA
CATTTAGAGTTCATCCC
Y
ATCCCCATCAGTCCAACACCTTCATATCCACCGATCAATCTACACCTCTCTGGTTCAAAGGCATCGAGAAAAAAATGAAGCACC
TCACTTTCTGCACTCTT / Celera SNP ID:  hCV26627664 / Public SNP ID:  rs3795727 /
SNP Chromosome Position:  156589450 / SNP in Genomic Sequence:  SEQ ID NO: 452 /
 SNP Position Genomic:  10364 / Related Interrogated SNP:  hCV9102827 / SNP
Source:  dbSNP; Celera; HapMap; ABI_Val; HGBASE / Population(Allele,Count):
Caucasian (C,178|T,48) / SNP Type:  MISSENSE MUTATION;ESS;SILENT MUTATION;INTRON
/
Context            (SEQ ID NO: 2730): /
GTACTTTAAAAAGGTTATCTATTTGTGCTGAGGGAAGAGGTAGAATATTAGGGAACCAATAAGATAACTTCTCTTTTGGAGAG
GTTTCCAAAGTCCTTCT
R
TCCTCAGAAATTCTCATGTTCCTCCCCAGGGACAGAAAAATCCTCCTTGCTGTCTAATCTCCATCTCTCCTATTGCAATGCTA
GCCACTCCCTCTGCCCT / Celera SNP ID:  hCV26627665 / Public SNP ID:  rs2365714 /
SNP Chromosome Position:  156588749 / SNP in Genomic Sequence:  SEQ ID NO: 452 /
 SNP Position Genomic:  9663 / Related Interrogated SNP:  hCV9102827 / SNP
Source:  dbSNP; Celera; HapMap; HGBASE / Population(Allele,Count):  Caucasian
(G,178|A,48) / SNP Type:  UTR5;UTR3;INTRON /
Context            (SEQ ID NO: 2731): /
CCAGGTGATCCCTTCAATCAATCAGTAGCTGTCTGTGTTTTGATTGATTCTGTTCCCTTTGAAACTTCTTCCCCTACCAGTGA
TAATGCCTAAAATGTAT
Y
GATTACTTTCTATGAGCCAAGCACTGTTCTGCTTATCAATGCTGTAGTTATCTAATATTCCTTCGTTAGTAGGTAGCAGGGCT
GAGATTTATGCTTAGAC / Celera SNP ID:  hCV26627679 / Public SNP ID:  rs7536235 /
SNP Chromosome Position:  156581534 / SNP in Genomic Sequence:  SEQ ID NO: 452 /
 SNP Position Genomic:  2448 / Related Interrogated SNP:  hCV9102827 / SNP
Source:  dbSNP; Celera; HapMap / Population(Allele,Count):  Caucasian

(T,178|C,48) / SNP Type: INTERGENIC;UNKNOWN /
Context (SEQ ID NO: 2732): /
AGCACCTACTATGTACCAGAAACTATGCTGGGAACTGGAGATAACAGCTAACACATAGACAGCACTTATTACATTCCAGACAC
TATTTCAAGCATTTTGC .
R
TATATCAATTTATTTATTCCTCACAACTACTCAGTGAGGTAGGTGTGCTATTATTCCCCACATTTCACTGATGAGGACACTGA
GGCAGAGAGAGGTTAAG / Celera SNP ID: hCV31431603 / Public SNP ID: rs11264508 /
SNP Chromosome Position: 156590696 / SNP in Genomic Sequence: SEQ ID NO: 452 /
SNP Position Genomic: 11610 / Related Interrogated SNP: hCV9102827 / SNP
Source: dbSNP / Population(Allele,Count): Caucasian (G,178|A,48) / SNP Type:
INTRON /
Context (SEQ ID NO: 2733): /
CTTCACCTCCGCGCTGGCGGGTGAGGCGCGGGACGAAGGCAGGGTCTTGGGGAGGGGTCTGAAAAATGTGGGGGACGAGGAGT
GGACCTCAGCTTGAGAT
M
AGGGCAGGGTCTCCGGGTCCCTCCAAGGCACCGCCCCCCCCATCAGCCCGCCCGCCCGCCCAGGCTCCAGCTCACCTCTCCAAA
CCCTCCCTGCACTTTTG / Celera SNP ID: hCV31431594 / Public SNP ID: rs12567958 /
SNP Chromosome Position: 156594656 / SNP in Genomic Sequence: SEQ ID NO: 452 /
SNP Position Genomic: 15570 / Related Interrogated SNP: hCV9102827 / SNP
Source: dbSNP; HapMap / Population(Allele,Count): Caucasian (C,57|A,169) /
SNP Type: TRANSCRIPTION FACTOR BINDING SITE;INTRON /
Context (SEQ ID NO: 2734): /
ATTAGATATGGCCCAAGTGAAAACCTGGAGCTTGTCTGAATTGGACAGCCAAGGAGAGAGGCAGAAGAGGATTAGTGGAAAAA
GCAGAAACTTCTAGGCT
Y
GACTCAATGACTGTATTGTGCGTGATTTGGGGCAAGCCACCTTCCTCTCCATCTCCTCCTCTAGTAAATGTTCAGCATCCTGA
GGTCTCCAGTTCTGAAA / Celera SNP ID: hCV31431609 / Public SNP ID: rs12742817 /
SNP Chromosome Position: 156587032 / SNP in Genomic Sequence: SEQ ID NO: 452 /
SNP Position Genomic: 7946 / Related Interrogated SNP: hCV9102827 / SNP
Source: dbSNP; HapMap / Population(Allele,Count): Caucasian (T,178|C,48) /
SNP Type: INTRON //

Gene Number: 116 / Gene Symbol: EPS8L2 - 64787 / Gene Name: EPS8-like 2 /
Chromosome: 11 / OMIM NUMBER: / OMIM Information: // Genomic Sequence (SEQ ID
NO: 453): // / SNP Information /
Context (SEQ ID NO: 2735): /
CATCACGTCTGTGGACGACGCCATCCGGAAGCTGGTGCAGCTGAGCTCCAAGGAGAAGATCTGGACCCAGGAGATGCTGCTGC
AGGTGAACGACCAGTCG
Y
TGCGGCTGCTGGACATCGAGTCACAGGTGGGGCCCAGCGCCACGGGGGACAGGGAGCACAGTGGGTAGAGGCGGTGGCAGCCA
CCGGCTGCAGCCCGGAT / Celera SNP ID: hCV491830 / Public SNP ID: rs3087546 /
SNP Chromosome Position: 720197 / SNP in Genomic Sequence: SEQ ID NO: 453 /
SNP Position Genomic: 24077 / SNP Source: Applera / Population(Allele,Count):
Caucasian (C,11|T,23) African American (C,13|T,19) total (C,24|T,42) // /
SNP Type: ESS;UTR5;SILENT RARE CODON;SILENT MUTATION;CODING REGION / SNP
Source: dbSNP; Celera; Applera / Population(Allele,Count): Caucasian
(C,103|T,121) / SNP Type: ESS;UTR5;SILENT RARE CODON;SILENT MUTATION;CODING
REGION //

Gene Number: 117 / Gene Symbol: C11orf49 - 79096 / Gene Name: chromosome 11
open reading frame 49 / Chromosome: 11 / OMIM NUMBER: / OMIM Information: //
Genomic Sequence (SEQ ID NO: 454): // / SNP Information /
Context (SEQ ID NO: 2736): /
TTAACAGAAAATGACAAACCAGGAATGGATGAGGGAGGCGCCAGCACAGCAGCGGGATGTCTTTTGATTTGCTTCTGTTATTT
GCATGTCTCTCTTGTGA
Y
GTCTGTGAAATGTTTAGAATGACAAAGTTTTTAAAAGACCTGCTGACTCCGCCTGAAAAATGTGAAAAACCTCCCATTTAGAG
ACCTATTATGTCCAGCT / Celera SNP ID: hCV31699798 / Public SNP ID: rs11039049 /
SNP Chromosome Position: 46998915 / SNP in Genomic Sequence: SEQ ID NO: 454 /
SNP Position Genomic: 50664 / Related Interrogated SNP: hCV30710896 / SNP
Source: dbSNP; HapMap / Population(Allele,Count): Caucasian (T,219|C,7) / SNP
Type: TRANSCRIPTION FACTOR BINDING SITE;INTRON /
Context (SEQ ID NO: 2737): /

AGATGATCTGCCCACCTTGGCTTCCCAAAGTGCTGGGATTACAGGCGTGAGCCACCATGCCCGGCCTTATCCCCATCTTAATG
ATGATGGAATTGAAGCT
Y
AAAAAATTTAAGTAACTAACTTGCCCCAGATCATTTGTCTAGTAACCAGTGGAGCTGAGCTATGAACCCAGAACCCAGGATTG
ACTAACTCCAAAAAATG / Celera SNP ID: hCV31699705 / Public SNP ID: rs11039099 /
SNP Chromosome Position: 47141496 / SNP in Genomic Sequence: SEQ ID NO: 454 /
SNP Position Genomic: 193245 / Related Interrogated SNP: hCV30710896 / SNP
Source: dbSNP; HapMap / Population(Allele,Count): Caucasian (C,219|T,7) / SNP
Type: INTRON /
Context (SEQ ID NO: 2738): /
GTGGGAATTCACTGTGCCAGGTGTTTTTCATCCATAGACGAAGGAAAAGCAGGTTGGGTTCCCAGAGACTTACAGCAAAAAGA
AAAGGACATCATAGGGA
R
CCAGTCTGTGTAGCAGAGAAAGGCCAGCCTTTGAATGAGAAGGCTTGATCTGATCCAGGAACTACCACTTATGCGGCAATTTT
GTCCCTTGCTTGAGCCT / Celera SNP ID: hCV31699700 / Public SNP ID: rs11039103 /
SNP Chromosome Position: 47148663 / SNP in Genomic Sequence: SEQ ID NO: 454 /
SNP Position Genomic: 200412 / Related Interrogated SNP: hCV30710896 / SNP
Source: dbSNP; HapMap; ABI_Val / Population(Allele,Count): Caucasian
(G,220|A,6) / SNP Type: INTRON /
Context (SEQ ID NO: 2739): /
TTGTGGTTCCTTTGTTAAAAATAAATAAATAAATTGACCATGTATTAATATGTGCATATCTATCTCTGGATTCTCTATTCTGT
TCCATTGATCTAAGGTA
R
TGTATGCCCTTCAACTTTTTTCTTTTTTCAAGATTGTTTTATTCATTCAAGGCCCTTTGCATTTCCATATAAAATTTAGAATAAA
CTGGTTCATTTCTACAA / Celera SNP ID: hCV32368695 / Public SNP ID: rs7938933 /
SNP Chromosome Position: 47106314 / SNP in Genomic Sequence: SEQ ID NO: 454 /
SNP Position Genomic: 158063 / Related Interrogated SNP: hCV30710896 / SNP
Source: dbSNP; HapMap; ABI_Val / Population(Allele,Count): Caucasian
(G,219|A,7) / SNP Type: INTRON //

Gene Number: 118 / Gene Symbol: ZC3H14 - 79882 / Gene Name: zinc finger
CCCH-type containing 14 / Chromosome: 14 / OMIM NUMBER: / OMIM Information: //
Genomic Sequence (SEQ ID NO: 455): // / SNP Information /
Context (SEQ ID NO: 2740): /
GCTGAAATTACAGGCATGAGCCACTGTGCCTGGCTTTTACTGGCGTAAGTGAACAGACATGTTAGTGAACCCTTTGGCAAAGA
ACTGTGGGCAGCCTCTA
S
GACTTGAGAGTGGCTTCCAGATGATAGCCAGAAAAAAATCTGGGTCCTTACTCATAAAGCCACAAGGAAATGAATTATTTTTT
TTTTTCCTGAGACAGGG / Celera SNP ID: hCV32095430 / Public SNP ID: rs11159859 /
SNP Chromosome Position: 89027891 / SNP in Genomic Sequence: SEQ ID NO: 455 /
SNP Position Genomic: 8638 / Related Interrogated SNP: hCV16182835 / SNP
Source: dbSNP / Population(Allele,Count): Caucasian (G,70|C,152) / SNP Type:
INTERGENIC;UNKNOWN /
Context (SEQ ID NO: 2741): /
AAAACTTGTCAGCATAGTAACACCATAATGAAATCATATTCAAATCACTCCCCCCTTGTAGTTTTTTTTCTTTTTGAGCCAATT
ACACTTAAAATGTCATT
R
ATGAAGATGTGAAGTCCAGGGTTTACAAAATATTTTTTATCTTCTTAGTATTCAGTGCGAGGAAATAGAAATGCTCATAAAAC
ATTTTTATTGTATACTG / Celera SNP ID: hCV32095431 / Public SNP ID: rs11629164 /
SNP Chromosome Position: 89031084 / SNP in Genomic Sequence: SEQ ID NO: 455 /
SNP Position Genomic: 11831 / Related Interrogated SNP: hCV16182835 / SNP
Source: dbSNP; HapMap; ABI_Val / Population(Allele,Count): Caucasian
(G,36|A,84) / SNP Type: TRANSCRIPTION FACTOR BINDING SITE;INTRON /
Context (SEQ ID NO: 2742): /
CAACCATTTTATGGATAGATTCCCCTGGACATCAGAGAAATGATTATCTTGGCAGTGTGTTTGTTGAAATGGAGAAGCATGTC
TGCCTCAGTAGCAAGTG
R
CTGCTGTGACTGTAATTCCCAACACTTAGGGCTTTTGATCCAAGTATGTCTATGCCATGTGATAATCACTATTGGAAAACACT
TGTCCATTCAGCCCACA / Celera SNP ID: hCV32095429 / Public SNP ID: rs12436642 /
SNP Chromosome Position: 89025376 / SNP in Genomic Sequence: SEQ ID NO: 455 /
SNP Position Genomic: 6123 / Related Interrogated SNP: hCV16182835 / SNP
Source: dbSNP; HapMap; ABI_Val / Population(Allele,Count): Caucasian
(A,76|G,150) / SNP Type: INTERGENIC;UNKNOWN /

Context (SEQ ID NO: 2743): /
ATAGGGTAATACGCATAACCATCACCTCAGACATTTATCATTTCTTTGTGATGGAAACTTTCAAAATCCTCTCTTGTAAATAC
CTGAAAATACATAAATA
Y
GTGATTCTTAACTATAGTCATCCTACAGTACTACAGAATACTAAAACATACTATTCCTATCTGGCTGTGTAAACTTGTATCCT
TTAACCAGTCCTTCCCT / Celera SNP ID: hDV77027209 / Public SNP ID: rs4635267 /
SNP Chromosome Position: 89088266 / SNP in Genomic Sequence: SEQ ID NO: 455 /
SNP Position Genomic: 69013 / Related Interrogated SNP: hCV16182835 / SNP
Source: CDX; dbSNP / Population(Allele,Count): Caucasian (C,169|T,57) / SNP
Type: INTRON //

Gene Number: 119 / Gene Symbol: ZBTB37 - 84614 / Gene Name: zinc finger and
BTB domain containing 37 / Chromosome: 1 / OMIM NUMBER: / OMIM Information: //
Genomic Sequence (SEQ ID NO: 456): // / SNP Information /
Context (SEQ ID NO: 2744): /
TGCTTGCTTTTCTTTGCCCCCAACTACCAAATTAGAGGAAGTATGGTACCAGCAGTAATTGTTCTTTTCAAATTAGTAAAATA
GTAATTCTGAAGAAAAG
Y
GTTATGGATATTTTTCATCTCTTTTTTTCCCTTGTCACGTGATGGTATCTTCCCTGTTTTCTTTCATGGATGACTGCTGCTACC
ACAGGCCTGACAATCTC / Celera SNP ID: hCV8911729 / Public SNP ID: rs941987 /
SNP Chromosome Position: 173840799 / SNP in Genomic Sequence: SEQ ID NO: 456 /
SNP Position Genomic: 13306 / Related Interrogated SNP: hCV16180170 /
Related Interrogated SNP: hCV8911768 / SNP Source: dbSNP; Celera; HapMap;
HGBASE / Population(Allele,Count): Caucasian (C,206|T,20) / SNP Type: INTRON
/
Context (SEQ ID NO: 2745): /
TACTGTATCTTGTCATTTATTTTCTTCCTGAATATTTTGTTAGACTAACACATTCTAGAAAGGATAGGAATGATGGGTTTTTT
TCTATTTTCTTTGCAAC
Y
CTCCTTAGCAACTAGTATGAGTTTGCTCATTTTTTTTTTTCTCACAGGTCTGATTGAATGATTTTTTTTAAAGCTCATGAACTC
ACCAAGTAGAAGACAAT / Celera SNP ID: hCV11975630 / Public SNP ID: rs2065170 /
SNP Chromosome Position: 173845232 / SNP in Genomic Sequence: SEQ ID NO: 456 /
SNP Position Genomic: 17739 / Related Interrogated SNP: hCV16180170 /
Related Interrogated SNP: hCV8911768 / SNP Source: dbSNP; HapMap /
Population(Allele,Count): Caucasian (T,203|C,23) / SNP Type: INTRON /
Context (SEQ ID NO: 2746): /
TAATGTGTTAGGCTGACTACCAAAGTTTCATCTAGTCTCTTTGGCTCCTATTTTCACGTTCAGCAATATTGTAATCAAAAATG
TCACTTTCCAGTGAAAT
R
AGCCTGGATTGTATTTTTAAATCCACAGGTCTCCATATTTGATAACTTTTATATGAAGTTTTAAACATGTTTTTTTCTGCAGTA
TTGTGGTTGAAGAAATT / Celera SNP ID: hDV70683162 / Public SNP ID: rs16846526 /
SNP Chromosome Position: 173856237 / SNP in Genomic Sequence: SEQ ID NO: 456 /
SNP Position Genomic: 28744 / Related Interrogated SNP: hCV16180170 /
Related Interrogated SNP: hCV8911768 / SNP Source: dbSNP /
Population(Allele,Count): Caucasian (G,203|A,23) / SNP Type:
INTRON;PSEUDOGENE /
Context (SEQ ID NO: 2747): /
CATTTTGCCTTTATAATAGTTTGGTTGAAGTTTTAAATTCATGTTGTCACATATACATGTTTTCTTGAAAGAGTTTGCCAACT
TTTTCTAGATCCTCTTA
Y
CATAACTCTTTTTGTGCTCCTTCATGATTTTTTTTGTGCTCCTTCATGATTCCCATTACTTTAAAAGCATTTAGAATTGGCCTG
TTGTTTGTCCTTGTCTT / Celera SNP ID: hDV70683177 / Public SNP ID: rs16846546 /
SNP Chromosome Position: 173860182 / SNP in Genomic Sequence: SEQ ID NO: 456 /
SNP Position Genomic: 32689 / Related Interrogated SNP: hCV16180170 /
Related Interrogated SNP: hCV8911768 / SNP Source: dbSNP; HapMap /
Population(Allele,Count): Caucasian (T,203|C,23) / SNP Type: INTRON //

Gene Number: 120 / Gene Symbol: CSMD2 - 114784 / Gene Name: CUB and Sushi
multiple domains 2 / Chromosome: 1 / OMIM NUMBER: 608398 / OMIM Information:
// Genomic Sequence (SEQ ID NO: 457): // / SNP Information /
Context (SEQ ID NO: 2748): /
TGTAACACAATCTGTAAAAGCAAGACATGTGGTCTTGAAAAAAGATGAGCATATCCAAGGCTGGCTGTCAAAATGCAGTTTGA
GTATAAATTGTTTGACT

M
TTTGAAAAAGCCTGGAGATCTCACTTAAACCAACAAATAATTATGCTTTATGGAGTGCGCAAAACCAAATCAGAATTCTGAAA
TTTGCTGTCAATTCTCT / Celera SNP ID: hCV1948599 / Public SNP ID: rs504527 /
SNP Chromosome Position: 34473608 / SNP in Genomic Sequence: SEQ ID NO: 457 /
SNP Position Genomic: 503999 / SNP Source: dbSNP; Celera; HapMap; ABI_Val;
HGBASE / Population(Allele,Count): Caucasian (A,93|C,133) / SNP Type: INTRON
//

Gene Number: 121 / Gene Symbol: TC2N - 123036 / Gene Name: tandem C2
domains, nuclear / Chromosome: 14 / OMIM NUMBER: / OMIM Information: //
Genomic Sequence (SEQ ID NO: 458): // / SNP Information /
Context (SEQ ID NO: 2749): /
TCTGAAAAAGCAGGGGCCTCCATGACTCTGCTCCAATATTACAGTCAGTGCTCATGCACGACAAGTTGTTAAAATACTTTTAA
TATCACTTCCTTTTGAG
R
TTCCCAAAGGGCTTATTTGCCTCTGCTGGTCTGACATAGTCTAGGAACAGCCTGTCTATGGTATTCTCACAAATAAAGGACAG
TACATCATCACGACTTT / Celera SNP ID: hCV27960688 / Public SNP ID: rs4900088 /
SNP Chromosome Position: 92290681 / SNP in Genomic Sequence: SEQ ID NO: 458 /
SNP Position Genomic: 54415 / SNP Source: dbSNP; HapMap; HGBASE /
Population(Allele,Count): Caucasian (A,37|G,83) / SNP Type: INTRON //

Gene Number: 122 / Gene Symbol: IQGAP3 - 128239 / Gene Name: IQ motif
containing GTPase activating protein 3 / Chromosome: 1 / OMIM NUMBER: / OMIM
Information: // Genomic Sequence (SEQ ID NO: 459): // / SNP Information /
Context (SEQ ID NO: 2750): /
GGGGCTGCGGCAGGATGTATGCTGAAGAGTGGGCGGCATCGGGTGGGGGGAAGTTGTGCAGGTGCTGGAGAAAAGCCGGAGGCT
TGCCGAGAGGAGCACTG
R
GAGGCGACTGCTGGGTGAGACCTTCGGGCAGGGAAGGCATGGGATCTGGGGAGACACAGAGCCTGATGATACTCAGAGGGCTG
GGTTTGGGGAACCCTGG / Celera SNP ID: hCV25989540 / Public SNP ID: rs6682716 /
SNP Chromosome Position: 156551848 / SNP in Genomic Sequence: SEQ ID NO: 459 /
SNP Position Genomic: 66651 / Related Interrogated SNP: hCV9102827 / SNP
Source: Applera / Population(Allele,Count): Caucasian (A,10|G,30) African
American (A,24|G,4) total (A,34|G,34) / SNP Type: MISSENSE MUTATION;ESS;ESE
SYNONYMOUS / SNP Source: Applera / Population(Allele,Count): Caucasian
(A,11|G,27) African American (A,14|G,2) total (A,25|G,29) / SNP Type:
MISSENSE MUTATION;ESS;ESE SYNONYMOUS / SNP Source: dbSNP; HapMap /
Population(Allele,Count): Caucasian (A,56|G,170) / SNP Type: MISSENSE
MUTATION;ESS;ESE SYNONYMOUS /
Context (SEQ ID NO: 2751): /
ACGTCTTGAGATTAGATAGCAGGAGTACCTAGATGATGAGTGAGGGGGGTGTCTGTGGAGGCACAGTGTGAAGGAAGTTATGG
AGGAGAGGCCCTGCATA
Y
GCGGTTCCTTCATCTATTTATTTGCAGCTTTTTTTTGAGACAGAGTTTCTCTGTCACCCAGGCTGGAGTGCAATGGCGCGATCT
CAGCTCACTGCAACCTC / Celera SNP ID: hCV9102976 / Public SNP ID: rs10908509 /
SNP Chromosome Position: 156550254 / SNP in Genomic Sequence: SEQ ID NO: 459 /
SNP Position Genomic: 65057 / Related Interrogated SNP: hCV9102827 / SNP
Source: dbSNP; Celera; HapMap / Population(Allele,Count): Caucasian
(T,173|C,53) / SNP Type: INTERGENIC;UNKNOWN /
Context (SEQ ID NO: 2752): /
GCCAAACCAACCTTTGGAACCTCCAGTCCAATCTCATTCTGTTCTACATTCCAGCACCCCTCTCAGGCTTCCCTGAAGATCAT
CCCCCAAGAACCTCTTC
Y
CCAGTCTTCTCTTCGGAATTCTCATCATTCTTCCAAGTTCCTTTCCTATTTCTCTAAAATCACTGATAAATTATGGTTTCTCT
AAGAAGCAATTTTGGAT / Celera SNP ID: hCV9103068 / Public SNP ID: rs4661183 /
SNP Chromosome Position: 156532733 / SNP in Genomic Sequence: SEQ ID NO: 459 /
SNP Position Genomic: 47536 / SNP Source: dbSNP; Celera; HapMap; HGBASE /
Population(Allele,Count): Caucasian (T,79|C,147) / SNP Type: INTRON //

Gene Number: 123 / Gene Symbol: RC3H1 - 149041 / Gene Name: ring finger and
CCCH-type domains 1 / Chromosome: 1 / OMIM NUMBER: / OMIM Information: //
Genomic Sequence (SEQ ID NO: 460): // / SNP Information /
Context (SEQ ID NO: 2753): /

AAAGAATGAGGATGCTCTATATGGATGTACTGATATGGAAGGGTCCTTAGGATAATTATGTGGGGAAAAAGCAAGCTGCAGAA
AGTGTTTATAGTATGCT
R
TGCTTTTTGTAACACAAAGTGAGGATGATGGGGTGAGGGGTGGCTAGAGACAAGGGTAGGAGTAGACTTATCAATATGTACCA
TCTTATTTAGTTTTCTA / Celera SNP ID: hCV11342529 / Public SNP ID: rs1951627 /
SNP Chromosome Position: 173891817 / SNP in Genomic Sequence: SEQ ID NO: 460 /
SNP Position Genomic: 1465 / Related Interrogated SNP: hCV16180170 / Related
Interrogated SNP: hCV8911768 / SNP Source: dbSNP; Celera; HGBASE /
Population(Allele,Count): Caucasian (G,168|A,58) / SNP Type:
INTERGENIC;UNKNOWN /
Context (SEQ ID NO: 2754): /
TAAGTCTTCCTTGGATACCATCTCACTGCTCCTTGCTCTTCTGAGGACAAGTATGGTTGAAAAATTCTTTGGTAATTCTGTTT
AGTGTTTTGTCAAGTTC
S
AGAATTAAAGTTCATTGCGGGTGGAGGCAGTATTTTCTAACTCTTTGTCCTCTACCGGGCTGTGCATATGGGAAGTACTCAAT
ACATTTCTTTAGTAAAT / Celera SNP ID: hDV70683212 / Public SNP ID: rs16846593 /
SNP Chromosome Position: 173898217 / SNP in Genomic Sequence: SEQ ID NO: 460 /
SNP Position Genomic: 7865 / Related Interrogated SNP: hCV16180170 / Related
Interrogated SNP: hCV8911768 / SNP Source: dbSNP / Population(Allele,Count):
Caucasian (C,209|G,17) / SNP Type: INTRON /
Context (SEQ ID NO: 2755): /
ATTGTTGCTATGGTTTTACCTTTTACAGATATCACATAATTGGAATCATACAGTATGTGGCCTTTTCATACTGGTATCTTTCA
CTTAGCAATGGTATACA
Y
TGAGATATATCTATGTCTTTTTGTGACTTGACAGCTATGTCTTTTCATTATTTCATTTTATACAATGTGCCATTGTATGGATA
TACCACTGTTTATCCAT / Celera SNP ID: hCV29989899 / Public SNP ID: rs6685043 /
SNP Chromosome Position: 173957513 / SNP in Genomic Sequence: SEQ ID NO: 460 /
SNP Position Genomic: 67161 / Related Interrogated SNP: hCV16180170 /
Related Interrogated SNP: hCV8911768 / SNP Source: dbSNP; HapMap /
Population(Allele,Count): Caucasian (T,210|C,16) / SNP Type: INTRON //

Gene Number: 124 / Gene Symbol: RBM46 - 166863 / Gene Name: RNA binding
motif protein 46 / Chromosome: 4 / OMIM NUMBER: / OMIM Information: //
Genomic Sequence (SEQ ID NO: 461): // / SNP Information /
Context (SEQ ID NO: 2756): /
CCATGTCTGTTATGAATGGAAAATGCATTGATGGAGCAAGTATTGAGGTAACACTAGCTAAACCAGTAAATAAAGAAAACACT
TGGAGACAGCATCTTAA
Y
GGTCAGATTAGTCCAAATTCTGAAAATCTGATTGTGTTTGCTAACAAAGAAGAGAGCCACCCAAAAACTCTAGGCAAGCTGCC
AACTCTTCCTGCTCGTC / Celera SNP ID: hCV2407223 / Public SNP ID: rs156502 /
SNP Chromosome Position: 155720274 / SNP in Genomic Sequence: SEQ ID NO: 461 /
SNP Position Genomic: 63894 / Related Interrogated SNP: hCV11503469 / SNP
Source: dbSNP; HapMap; ABI_Val; HGBASE / Population(Allele,Count): Caucasian
(T,130|C,96) / SNP Type: SILENT MUTATION /
Context (SEQ ID NO: 2757): /
CCACACACTTTTGATTAACTCTTCTCGTTTTCCAAGAACGAGAAGTGTAGTGGCTGTAGTGCAGTCTGTCACTAAAATGAATT
GCTGCTTAAAAACAAAC
K
GAAAACCTCCATTGACCTGGCAACCATTGACCTGGCAACCATGGTTTCCATGTACCATACTCAATGACAACAGTGCTAACAAA
TCAGGAGTTTAGTACCA / Celera SNP ID: hCV2407232 / Public SNP ID: rs156550 /
SNP Chromosome Position: 155705144 / SNP in Genomic Sequence: SEQ ID NO: 461 /
SNP Position Genomic: 48764 / Related Interrogated SNP: hCV11503469 / SNP
Source: dbSNP; HapMap; HGBASE / Population(Allele,Count): Caucasian
(T,130|G,92) / SNP Type: TFBS SYNONYMOUS;INTRON /
Context (SEQ ID NO: 2758): /
CTTTCTGGTGTTTGCAGAGATCCCCTTCTATTTTCTCCCTTGTATGCCAAGTATCTTTCGACACTTCTTTTTAGTCACAAACA
CCTTTACATTGCTTATC
R
TCTTCCTTCTTACTCATTTGGGTCACCTTTTCCCATAAGCTCAAATAATCTTAAAACAAATCCAATTTGGAATCTGTTACTGA
AGAATTCTAGCTGATAA / Celera SNP ID: hCV2407238 / Public SNP ID: rs156543 /
SNP Chromosome Position: 155700233 / SNP in Genomic Sequence: SEQ ID NO: 461 /
SNP Position Genomic: 43853 / Related Interrogated SNP: hCV11503469 / SNP
Source: dbSNP; HapMap; ABI_Val; HGBASE / Population(Allele,Count): Caucasian

(A,162|G,64) / SNP Type: INTERGENIC;UNKNOWN /
Context (SEQ ID NO: 2759): /
CCTTAACAAAATTATACACTTTTCAAAGGAGTTTCTTAGTGTCTAGTAGTCACAGTGTACATAGCACTCATAAATTATAAAGC
GTGAATAGAATTTCATG
K
TCTCCCAGAGTTATTTTATTTTTTTATAAAGTTCTACAGACAGGCAATCAAGGTAAGGAAGATCTTTCATTTCTAGCTGCTCAG
GAGATTTACGTTCAAAC / Celera SNP ID: hCV2407252 / Public SNP ID: rs149225 /
SNP Chromosome Position: 155683216 / SNP in Genomic Sequence: SEQ ID NO: 461 /
SNP Position Genomic: 26836 / Related Interrogated SNP: hCV11503414 /
Related Interrogated SNP: hCV11503469 / SNP Source: dbSNP; HapMap; ABI_Val;
HGBASE / Population(Allele,Count): Caucasian (T,64|G,52) / SNP Type:
INTERGENIC;UNKNOWN /
Context (SEQ ID NO: 2760): /
GAGCCTGTTTTCTTATCTGTAAAATATGAATACTAGCAATGTATTAGGCTATTCTTGCATTGCTATAAAGAAATACCTGAGAC
TGGGTAATTTATAAAGA
R
AAGGGGATGGTGCTAAACCATTAATGAGAAATCTGCCCTCATAATCCAGTCACCTCCTACCAGGCCCCACCTCCAACACTAAG
GGTTACATTTCAGTGTG / Celera SNP ID: hCV2891425 / Public SNP ID: rs1948714 /
SNP Chromosome Position: 155783261 / SNP in Genomic Sequence: SEQ ID NO: 461 /
SNP Position Genomic: 126881 / Related Interrogated SNP: hCV11503414 / SNP
Source: dbSNP; Celera; HapMap / Population(Allele,Count): Caucasian
(G,30|A,66) / SNP Type: INTERGENIC;UNKNOWN /
Context (SEQ ID NO: 2761): /
TTGGGAGAAGGATGGACAGGGTTTTCATCAATCCTCTGAGGCCTTGGAAAGCAGCTTGATGATATCAGATGCTCTCATTTGAA
TTGATATATTGGAGAAT
K
ATTTCATTGCCTTTAGATTTAATCAGAATTTTTCTGTAATGATTTTTAATTTTAGTTTTTTTTATTCTGAAATTATTTATAATG
AGTTTTCTATGACAAAA / Celera SNP ID: hCV2891496 / Public SNP ID: rs156584 /
SNP Chromosome Position: 155730513 / SNP in Genomic Sequence: SEQ ID NO: 461 /
SNP Position Genomic: 74133 / Related Interrogated SNP: hCV11503469 / SNP
Source: dbSNP; Celera; HapMap; ABI_Val; HGBASE / Population(Allele,Count):
Caucasian (G,130|T,96) / SNP Type: INTRON /
Context (SEQ ID NO: 2762): /
TCCAAAGTCCCTGCTAATTCCTCACTGTTGTAGTATATTTAAAAATTAAAAATATACTTTAACAAAATTTAAATTGGTAGACA
TTCATTAGTTCCTTGAA
Y
ATATGGAGAAAATACATTAGGAGTTAATGTGGAAGAAATCACCTTGAGGTTGGGAATAAGGAGATCTGTGTTACTCGGTGAAT
ATTCTGGGCATGAAGTT / Celera SNP ID: hCV2891515 / Public SNP ID: rs11940892 /
SNP Chromosome Position: 155701520 / SNP in Genomic Sequence: SEQ ID NO: 461 /
SNP Position Genomic: 45140 / Related Interrogated SNP: hCV11503469 / SNP
Source: dbSNP; Celera; HapMap / Population(Allele,Count): Caucasian
(T,162|C,64) / SNP Type: INTERGENIC;UNKNOWN /
Context (SEQ ID NO: 2763): /
CAGCAGCTTTGCAGTGACAATACGGTTTCTTAAATATGGTGGCTATTTTGCCTCTGGTGTCTCTTCCAGAGCTTGCTACCAGG
ATAGCGTGAGTCCAGTA
R
GCGTACCAAGTCCTTCAATACTACTAGTCTCATGGGGAGGAAAACAAAAACCAGTCCCACACACCAAGCAATTAAGTGCTCAC
TGTGTTGTTTTTACGGT / Celera SNP ID: hCV2891530 / Public SNP ID: rs7662464 /
SNP Chromosome Position: 155686380 / SNP in Genomic Sequence: SEQ ID NO: 461 /
SNP Position Genomic: 30000 / Related Interrogated SNP: hCV11503469 / SNP
Source: dbSNP; Celera; HapMap / Population(Allele,Count): Caucasian
(G,162|A,64) / SNP Type: INTERGENIC;UNKNOWN /
Context (SEQ ID NO: 2764): /
CCAGGAATACAAACCAAAGCATGTTTTATAAATGAACACCTCTTTCTCTGAAATCAGAACTTAGAAAAGTAAAATAACTGTTC
AGTTATAATTAGCAATC
R
TACTGATAATTCTGAGGTTTTACATCATTCAAGATTCCACCTGAAAGATTCACTTGTCTTTCCACCTGAAAGATTCGCACAGA
TCAATCTGTGTGTGTGT / Celera SNP ID: hCV2891532 / Public SNP ID: rs13110294 /
SNP Chromosome Position: 155684990 / SNP in Genomic Sequence: SEQ ID NO: 461 /
SNP Position Genomic: 28610 / Related Interrogated SNP: hCV11503414 /
Related Interrogated SNP: hCV11503469 / SNP Source: dbSNP; Celera /
Population(Allele,Count): Caucasian (G,113|A,5) / SNP Type:
INTERGENIC;UNKNOWN /

Context            (SEQ ID NO: 2765): /
ATCAGAGGCAAAAATATCCTTCAAGAAACTATGGACACTCCGCGCCCTATTCATTTCCATGGCAGCAGAGTATCTGCATCTTG
AGCCACCTATACAGATT
S
ATGCCTCGTATCGCTCTCACCTCCTTTCTTTTTGAAGTAAAGCCCTTTCCCAAGAAGGCGGCCAGAAAGTGGACCCCACCGGG
GGAAAAAGAAAAATGAA / Celera SNP ID: hCV2891552 / Public SNP ID: rs1876031 /
SNP Chromosome Position: 155662736 / SNP in Genomic Sequence: SEQ ID NO: 461 /
 SNP Position Genomic: 6356 / Related Interrogated SNP: hCV11503469 / SNP
Source: dbSNP; Celera; HapMap; ABI_Val; HGBASE / Population(Allele,Count):
Caucasian (C,201|G,23) / SNP Type: UTR3;INTRON /
Context            (SEQ ID NO: 2766): /
GTGTGCCTCCAGGGAGCCATTAAAAAACTGACCTCTCAACCACAGAAATAGATGAGATTTTGAGAACATTGAGAAGCTGCCTTT
TGCAAAGTAAATTTGCA
R
TGGTCCTTGACGAAGGGGGGTCGGGGGCGGGGAGAAGTCCAGCCGAGAGAGGAGCTCATTCCACGCTATATTTTTGCAGTTGA
AAAGCTGCCTAATCATC / Celera SNP ID: hCV2891554 / Public SNP ID: rs12501328 /
SNP Chromosome Position: 155662331 / SNP in Genomic Sequence: SEQ ID NO: 461 /
 SNP Position Genomic: 5951 / Related Interrogated SNP: hCV11503469 / SNP
Source: dbSNP; Celera; HapMap; ABI_Val / Population(Allele,Count): Caucasian
(A,195|G,23) / SNP Type: UTR3;INTRON /
Context            (SEQ ID NO: 2767): /
TGACTCTAGTGATATCTTCTTCTCGAGTCTGGATTCTGGTGCTGCTATTTGCTCCCATTTAGCTTATGATGGCTACTCGCGTG
GCGGCCCACCTACTGCC
R
CTAACTCACACTGCACCATTGTTTCTGCTGCCTTGTGGTTTTCATTACACTTCTACCCTGTTACTTTTAGCTTCTGCTCCACT
GTACCTGGTCGCCGTCT / Celera SNP ID: hCV8938834 / Public SNP ID: rs1500372 /
SNP Chromosome Position: 155676300 / SNP in Genomic Sequence: SEQ ID NO: 461 /
 SNP Position Genomic: 19920 / Related Interrogated SNP: hCV11503414 / SNP
Source: dbSNP; HapMap; HGBASE / Population(Allele,Count): Caucasian
(G,112|A,8) / SNP Type: INTERGENIC;UNKNOWN /
Context            (SEQ ID NO: 2768): /
GGCTTTAATTGACCCTCCCACCTAAGCCTCCAAAAGCACTAGGATTATGGGTGTGAGCCACCAGGCCCAGCGACAACATGGGT
TTTAACTGGGTGGGTCC
R
TTTACGTGCAAAATTTTTCAACCAAATGTGGACAGAAAACCAAGTATTCCTAGGATCTGAAACCTGCTTATATAGGGAGGGCC
AACTTCTGTATATGTGG / Celera SNP ID: hCV8938838 / Public SNP ID: rs1392546 /
SNP Chromosome Position: 155674933 / SNP in Genomic Sequence: SEQ ID NO: 461 /
 SNP Position Genomic: 18553 / Related Interrogated SNP: hCV11503414 / SNP
Source: dbSNP; Celera; HapMap; HGBASE / Population(Allele,Count): Caucasian
(A,112|G,8) / SNP Type: INTERGENIC;UNKNOWN /
Context            (SEQ ID NO: 2769): /
TGCTTTTCTCTTAGCCTGGAATGCCCTCCTCCCAGATCTTTGCTGGCTGACTCTTCTTGTTATTTAGACTGACTTTCTCCTCG
GCTGCCCTCTCTGTAAT
W
GCGATCAACCAGTCACTTTCCTTCCCACCACCCCTCTCAATTTTCCCTTTAGTGTTTGTCTCTCTTTGAAATTATTCTTTCCA
TTGATTTGCTTGTTTAT / Celera SNP ID: hCV9317142 / Public SNP ID: rs12186175 /
SNP Chromosome Position: 155823166 / SNP in Genomic Sequence: SEQ ID NO: 461 /
 SNP Position Genomic: 166786 / Related Interrogated SNP: hCV11503414 / SNP
Source: dbSNP; Celera; HapMap / Population(Allele,Count): Caucasian
(A,63|T,57) / SNP Type: INTERGENIC;UNKNOWN /
Context            (SEQ ID NO: 2770): /
TATGGGATTCTCACTACAAATTCTGGAAATAGTCACCTGCACCCTTCAGCACATTCATTAGCCAACATTTCTAAAATAGGGTT
AGTCAATTTGGCTATTT
K
CCTGAGTTTCCAGATTCCTTATAGAGTCTGTCAGCTTACCCAAGTTGACAGACTCTATAAGTCAATAAACTGATAGCTGAAAC
AAAACAAAACAAAGAAA / Celera SNP ID: hCV11281035 / Public SNP ID: rs4583739 /
SNP Chromosome Position: 155686903 / SNP in Genomic Sequence: SEQ ID NO: 461 /
 SNP Position Genomic: 30523 / Related Interrogated SNP: hCV11503469 /
Related Interrogated SNP: hCV11503414 / SNP Source: dbSNP; Celera; HapMap;
HGBASE / Population(Allele,Count): Caucasian (T,55|G,47) / SNP Type:
INTERGENIC;UNKNOWN /
Context            (SEQ ID NO: 2771): /
AGTGGCTCCTCGGGGCACTCCTGTACTTCCTTGGTCTTAACCTTGGGGGCCTATCTGGTTAGCCAGGGGCTTCTTGCAGGCCT

TCTCTGCCTTCAGCTTG
Y
GGATGTGTTTCAGGAGAATCTGCTTGATTCTCATTCACCTAAAAGATAATGTAATTTTTAAGAGAAAAAAAGTCAGATATGTT
ATTTTTCTTTGAGTACC / Celera SNP ID: hCV27020184 / Public SNP ID: rs47379 /
SNP Chromosome Position: 155691608 / SNP in Genomic Sequence: SEQ ID NO: 461 /
SNP Position Genomic: 35228 / Related Interrogated SNP: hCV11503469 / SNP
Source: dbSNP / Population(Allele,Count): Caucasian (C,132|T,94) / SNP Type:
INTERGENIC;UNKNOWN /
Context (SEQ ID NO: 2772): /
CCTCTGTGGCTTTGCAGGGTACAGACTCCCTCCCAGCTGCTTTCATGGATTGGCTTTGAGTGTCTGTGGCTTTTCCAGGTGCA
TGGTGTAAGCTGTCAGC
R
GATGTACCATTCTGGGCTCTGGAGGACAGTGACCCTCTTCTCACAGCTCCACTAAGCAGTGCCCCAGTAGGGACTCTGTGTGG
GGGCTCCAACCCCACAT / Celera SNP ID: hCV27020284 / Public SNP ID: rs1846707 /
SNP Chromosome Position: 155805881 / SNP in Genomic Sequence: SEQ ID NO: 461 /
SNP Position Genomic: 149501 / Related Interrogated SNP: hCV11503469 / SNP
Source: dbSNP; Celera; HGBASE / Population(Allele,Count): Caucasian
(G,114|A,112) / SNP Type: INTERGENIC;UNKNOWN /
Context (SEQ ID NO: 2773): /
CAAACTTTTACACTCTTCTTCCCTTTTAAATATAACCTACAACTTTAAGTCATGTTTTTGCTCCTGCACTTGAAATAGCTTGC
TAGAAGCAGCCAGGCCA
Y
ATCTTGATCACTTTACTGCTTATAAATTTCTTCCACCAAATACCTTAAGTCATCACTCTTAAGTTCAGACTTTCACAGTTTGA
ACTTAAGTTCCCTAGGG / Celera SNP ID: hCV27020304 / Public SNP ID: rs13101534 /
SNP Chromosome Position: 155784630 / SNP in Genomic Sequence: SEQ ID NO: 461 /
SNP Position Genomic: 128250 / Related Interrogated SNP: hCV11503414 / SNP
Source: dbSNP; HapMap / Population(Allele,Count): Caucasian (C,94|T,24) / SNP
Type: INTERGENIC;UNKNOWN /
Context (SEQ ID NO: 2774): /
TTCTATTATTTTTACTCAAGATTAGAGACACCTCTTTGCTCATCTAATTTTAAAATATGCTATTGTTATTGATAAGCAGTTTG
ATGTTTTAAACCTTTAG
Y
CCTGAATAACCATGTGGTTATACTTTCTATAGTTTTCTATAAATGCTGTATTTACATAGGCAACTGCCATTATTAGTTGCCTA
AGGGATCATTTCTGCTA / Celera SNP ID: hCV27479909 / Public SNP ID: rs3775785 /
SNP Chromosome Position: 155669854 / SNP in Genomic Sequence: SEQ ID NO: 461 /
SNP Position Genomic: 13474 / Related Interrogated SNP: hCV11503414 /
Related Interrogated SNP: hCV11503469 / SNP Source: dbSNP; HapMap; HGBASE /
Population(Allele,Count): Caucasian (T,111|C,7) / SNP Type: INTRON /
Context (SEQ ID NO: 2775): /
CTAGAAATTACTTGTTTTAAATTGCCACTAATATGCCTAACCTCATTTCAGATAATTTAACCTTAAACACATGTATTTAGAAT
TAATTACTAGGAATTGA
R
AAGGTACCGACATGAAAATGAAGAATGAGGAGAATCTTTTTCTGGAGAAATAAAAGAGCAGCAAAAAATAGTAATCAATATAA
CAATCACAGTTCCCGAG / Celera SNP ID: hCV29317506 / Public SNP ID: rs7686002 /
SNP Chromosome Position: 155700846 / SNP in Genomic Sequence: SEQ ID NO: 461 /
SNP Position Genomic: 44466 / Related Interrogated SNP: hCV11503414 / SNP
Source: dbSNP; HapMap / Population(Allele,Count): Caucasian (G,86|A,34) / SNP
Type: INTERGENIC;UNKNOWN /
Context (SEQ ID NO: 2776): /
TGGTAAAACAAGCATTCAAGCACTTTTACAAAATTACCAAATTCTTAAAATGAAGCCACAGCTAGACTTGCATTTCAGGTATT
AAAATTGCTTTCTTAAC
K
GTCAAGAATCACAAAATAACAAATCATATTATGAGTGAATATGGGGAGGGCGGGGCCAATCAGTCAATGATAATCTGAACAAA
TTTTAAGAGCAGATTTT / Celera SNP ID: hCV31863937 / Public SNP ID: rs12507608 /
SNP Chromosome Position: 155671626 / SNP in Genomic Sequence: SEQ ID NO: 461 /
SNP Position Genomic: 15246 / Related Interrogated SNP: hCV11503469 / SNP
Source: dbSNP; HapMap / Population(Allele,Count): Caucasian (T,202|G,24) /
SNP Type: MICRORNA;UTR3;TFBS SYNONYMOUS /
Context (SEQ ID NO: 2777): /
CTAAGCTATAGACTATGTACGTCATAGGCAGTTGAATTTTACCCAGTGACATTAGGATAATTAACTCACCATCTCTGTCTGTA
TTGACCAAATTATGTAA
M
CTCATTTGTGTAGCTTGTTGTAGCTTGGCTTCACATAATATTTGGAAAGCCTCAAGATCCTTGTTAAATTATTAAATTTGATG

502

ATAATAAAAATTAAAAA / Celera SNP ID: hCV31863942 / Public SNP ID: rs13101382 / SNP Chromosome Position: 155821855 / SNP in Genomic Sequence: SEQ ID NO: 461 / SNP Position Genomic: 165475 / Related Interrogated SNP: hCV11503414 / SNP Source: dbSNP; HapMap / Population(Allele,Count): Caucasian (C,62|A,56) / SNP Type: INTERGENIC;UNKNOWN /
Context (SEQ ID NO: 2778): /
CCTGGGCAACAAAGTGAGACTCCATCTCAAAAAAAAAAAAAAAAAAAAAAAAAAAAAAAGAAGAAGAAACCCTGAGCCATTAA
TGTGTCTCGTAACTTGA
K
GAAGTTCATGAATTCTCTGGGCTTCATCCATTAAGATTCATCCTTATGCTCCTGCAAGTTTCTTATATTTATGTATTTCTATT
CTGATTTCTGAAATGAT / Celera SNP ID: hDV70817844 / Public SNP ID: rs17032000 / SNP Chromosome Position: 155672958 / SNP in Genomic Sequence: SEQ ID NO: 461 / SNP Position Genomic: 16578 / Related Interrogated SNP: hCV11503469 / SNP Source: dbSNP; HapMap / Population(Allele,Count): Caucasian (G,203|T,23) / SNP Type: MICRORNA;UTR3 //

Gene Number: 125 / Gene Symbol: CCDC46 - 201134 / Gene Name: coiled-coil domain containing 46 / Chromosome: 17 / OMIM NUMBER: / OMIM Information: // Genomic Sequence (SEQ ID NO: 462): // / SNP Information /
Context (SEQ ID NO: 2779): /
CTGGGCCTGATGGAGCCCCAGGGAAATCACTACCGCTGCCGGTGTTTGCAGGTAGCAGGGAGCCAGGCCTGGGCGAGGTGTGT
CTCGATGCCAGAGCTGG
Y
AGTACAGAAGGTGGTGGGCCATCCCCGGTGCTGCTCAGCGTGGTGGATCATTTCAACCGAATCGGCAAGGTTGGAATCCAGAA
ATGCGTCATTGGTCTGT / Celera SNP ID: hCV2658444 / Public SNP ID: rs7209242 / SNP Chromosome Position: 64193824 / SNP in Genomic Sequence: SEQ ID NO: 462 / SNP Position Genomic: 572166 / Related Interrogated SNP: hCV2303891 / SNP Source: Applera / Population(Allele,Count): Caucasian (C,4|T,28) African American (C,9|T,27) total (C,13|T,55) / SNP Type: PSEUDOGENE / SNP Source: Applera / Population(Allele,Count): Caucasian (C,5|T,27) African American (C,5|T,23) total (C,10|T,50) / SNP Type: PSEUDOGENE / SNP Source: dbSNP; Celera; ABI_Val / Population(Allele,Count): Caucasian (T,204|C,22) / SNP Type: PSEUDOGENE /
Context (SEQ ID NO: 2780): /
ACATTATCACTGAATGTCTATCTACCCTTGAACAACTCAGTGTAAACAGTCCTATGTCAATGGCAAAAATTGTTACTAGAGAA
ATTCAGTTTTTCTTCCA
K
GTTGATAGTTCATTGGTTTGACTAAATAATATAAAGAAAAAAATTAATCTTAAGAATCTAATCTTCAAACATAATCTTACCTG
TCCTATGAGGTAATTTT / Celera SNP ID: hCV2658455 / Public SNP ID: rs11658189 / SNP Chromosome Position: 64179231 / SNP in Genomic Sequence: SEQ ID NO: 462 / SNP Position Genomic: 557573 / Related Interrogated SNP: hCV2303891 / SNP Source: dbSNP; Celera; HapMap; ABI_Val / Population(Allele,Count): Caucasian (T,214|G,12) / SNP Type: INTRON /
Context (SEQ ID NO: 2781): /
CAGTATAAAGATTGCAGAAACTAAGAATGAGGAGGCATATTTTCTTTTTTTTCACTACTAAGAAAAGAGTCACTGCCTTTAGCA
CAGTGACTTTGGATTTC
W
TCCTTATGCTAGAACTAAGCAATATTCACCATTTCATTCCTCCTTCTGCTGCTTTTTCTATCTTAGCCTCTTGGGCAATTCAT
GCTATAGGATATTAACA / Celera SNP ID: hCV26589423 / Public SNP ID: rs1014399 / SNP Chromosome Position: 64196331 / SNP in Genomic Sequence: SEQ ID NO: 462 / SNP Position Genomic: 574673 / Related Interrogated SNP: hCV2303891 / SNP Source: dbSNP; Celera; HapMap; HGBASE / Population(Allele,Count): Caucasian (T,112|A,8) / SNP Type: INTERGENIC;UNKNOWN /
Context (SEQ ID NO: 2782): /
GCAGCATAAACTAAGTGCCTAGGGGCAGCTGAAACTAAAAAAGCAAGTAATAACCAAAAAAGGATTGAGCCAGTGTGCTACTA
GCTCCAGAAGGCCCATG
R
TACAAGACACAGAATAAGAAGGAACAAGAGATTATGATCTCCTAAAAACAGAAACCAGAAAATCCCACTATTTAGAAATTTAC
ACACAGAAGTCCAGAGA / Celera SNP ID: hCV29866571 / Public SNP ID: rs9891968 / SNP Chromosome Position: 64132846 / SNP in Genomic Sequence: SEQ ID NO: 462 / SNP Position Genomic: 511188 / Related Interrogated SNP: hCV2303891 / SNP Source: dbSNP; HapMap / Population(Allele,Count): Caucasian (G,115|A,5) / SNP Type: INTRON /

Context          (SEQ ID NO: 2783): /
TACAGTCAGCAAACATAACCAAACCACAAGTATTAAAAGGTCTGGGAAAAAAAGGAGTCACAACTGCCAAGCAAAGAATACAC
TTCATTCATATCACACT
Y
CTTGCCAAACAAAACAAACGTTCTTGGAAGTTGTGTAAAACCCATTTTGTTCAATCAAATCATATTTTAAATATATATTGTGGGT
CATTAAAGAAAACTGGA / Celera SNP ID:   hCV30064665 / Public SNP ID:   rs9902706 /
SNP Chromosome Position:   64186988 / SNP in Genomic Sequence:   SEQ ID NO: 462 /
SNP Position Genomic:   565330 / Related Interrogated SNP:   hCV2303891 / SNP
Source:   dbSNP; HapMap; ABI_val / Population(Allele,Count):   Caucasian
(C,214|T,12) / SNP Type:   INTRON /
Context          (SEQ ID NO: 2784): /
CCATAAACATCCATTTTATGGGACAACTTGGATGGTTTCATACCACAAAATAATTTGTTAATGAATACAGAATATTAAAAGGT
GAAAATCGGGACATTAA
K
AACAAAGTGTGTGAGTAAAAGTATTGAATTTTTTTAATGCAATCGAAGTTATCAGCTTAAAATAGGTTGTTACAACTATAAGAT
GATTTATGTAAGCCTTA / Celera SNP ID:   hCV30551147 / Public SNP ID:   rs9908597 /
SNP Chromosome Position:   64134683 / SNP in Genomic Sequence:   SEQ ID NO: 462 /
SNP Position Genomic:   513025 / Related Interrogated SNP:   hCV2303891 / SNP
Source:   dbSNP; HapMap / Population(Allele,Count):   Caucasian (T,115|G,5) / SNP
Type:   INTRON /
Context          (SEQ ID NO: 2785): /
CTTATAAGCACAACCTACAATTGGTACAGGGGACCTGATCACAGTGAGCTCATAATATGGTACTTGTGCCAAGCAGAAAAGCG
GTATTGTGCCTCAATTA
R
TTTCAGGATGCCAAAACCAACATGGAAAGAAAAAATTCAGTACATTTGTAACATTCCCTGACATGCCCTTTACTACTCTTAAG
CCATAAAACTAGGAGAA / Celera SNP ID:   hCV29577360 / Public SNP ID:   rs9910950 /
SNP Chromosome Position:   64131710 / SNP in Genomic Sequence:   SEQ ID NO: 462 /
SNP Position Genomic:   510052 / Related Interrogated SNP:   hCV2303891 / SNP
Source:   dbSNP; HapMap / Population(Allele,Count):   Caucasian (A,214|G,12) /
SNP Type:   INTRON /
Context          (SEQ ID NO: 2786): /
GCTTCCAGTGTTGAGTTCATTTATATTTAATGTAATTATTTATGGTTCTATTTAGTCTGTCATTTTGCTATTGGTTTTGTCAC
AGCTCTTCTTTGCTCCT
Y
GTTTTTCCTTCCCTGCCTTATTTTATGTTCACTGAATATTTTATAGTATTCCATTTTAATCCTTCTAGTTACACATCCTTCTA
GCTATACCTAGCTATAT / Celera SNP ID:   hCV31400900 / Public SNP ID:   rs7216660 /
SNP Chromosome Position:   64147376 / SNP in Genomic Sequence:   SEQ ID NO: 462 /
SNP Position Genomic:   525718 / Related Interrogated SNP:   hCV2303891 / SNP
Source:   dbSNP; HapMap; ABI_val / Population(Allele,Count):   Caucasian
(C,214|T,12) / SNP Type:   INTRON //

Gene Number:   126 / Gene Symbol:   KIF6 – 221458 / Gene Name:   kinesin family
member 6 / Chromosome:   6 / OMIM NUMBER: / OMIM Information: // Genomic
Sequence (SEQ ID NO: 463): // / SNP Information /
Context          (SEQ ID NO: 2787): /
ACAGGAATAGGTTAAACAGAAAGGTAGGGAGCCTTTTCTGGGAACTCTAACACCTCCGGTGAGTTCTCACCTTACCTTTTGTT
AGAGAGGAGTTGGGACC
R
TTCATGCTGGGAGTCAGATGTCTGGAGAAATGGCTTCGTGTGATCGAGTGAATTCACTGCTGGAGAATTTACCTGTTGGCCCC
AGAAGGAGTTTCACAGT / Celera SNP ID:   hCV3054799 / Public SNP ID:   rs20455 / SNP
Chromosome Position:   39325078 / SNP in Genomic Sequence:   SEQ ID NO: 463 / SNP
Position Genomic:   32202 / SNP Source:   Applera / Population(Allele,Count):
Caucasian (A,23|G,13) African American (A,10|G,26) total (A,33|G,39) // / SNP
Type:   MISSENSE MUTATION;ESS;ESE / SNP Source:   dbSNP; Celera; HapMap; ABI_val;
HGBASE / Population(Allele,Count):   Caucasian (A,142|G,84) / SNP Type:
MISSENSE MUTATION;ESS;ESE /
Context          (SEQ ID NO: 2788): /
GTCTTTGAAGCCAAAGGAAAAATGAGAGACAGGGCTTTCTACTCGCTGAGCAGGATATTGCTACCAGTAGAAGCTACCCAAGA
GATCAGAAAAGAGGGTA
S
AGAAATAGCCAGGGGTTCTGAGCCTCACAGCTGAAAGGAGAGCTTATTCTCATGGGGCAGATACACAAGGGCTCAGCACAGAG
CCAGAGCAATGATTGAG / Celera SNP ID:   hCV3054805 / Public SNP ID:   rs2894424 /
SNP Chromosome Position:   39319535 / SNP in Genomic Sequence:   SEQ ID NO: 463 /

SNP Position Genomic: 26659 / SNP Source: dbSNP; Celera; HapMap; HGBASE /
Population(Allele,Count): Caucasian (C,130|G,96) / SNP Type: INTRON /
Context (SEQ ID NO: 2789): /
AGCAGTGGAGAGACTTTCCACGAGGTGCCCTTCATGGTGGGAAAGCAGAGATCCCTGTGCTGGGTCTAGTGAGGACCGATGTA
GGACCAGAGGTAGCCAC
M

AGGTGGCAGGCTCTGCACGCTTTTCTTCAGAGAACAGTAACCAATTCTCAAGGCTCCTGCTGAGTGGTTCCTGGTGCAGCCTG
GAGAAGGGAAGGAGAAA / Celera SNP ID: hCV3054808 / Public SNP ID: rs9462535 /
SNP Chromosome Position: 39315792 / SNP in Genomic Sequence: SEQ ID NO: 463 /
SNP Position Genomic: 22916 / SNP Source: dbSNP; Celera; HapMap /
Population(Allele,Count): Caucasian (C,136|A,90) / SNP Type: TRANSCRIPTION
FACTOR BINDING SITE;INTRON /
Context (SEQ ID NO: 2790): /
GGAGGAATGATGGTTGGTTTCTATGGATTTGTTACAGGGGAAGGGATATCTCAGCTCAAAGGATATCCTGCGTATCCAAGCCT
TCCTCCACTTGTGCCCC
R

CACACTGTCCCCTCTACTTTCTCAAGGAAGTTTTTCCTGCAGCTCTGCTCCCCTCTCCCACCACAGCATCAGTTTCCCTCTCT
ATGCATGTTTCAGCACG / Celera SNP ID: hCV3054813 / Public SNP ID: rs9471077 /
SNP Chromosome Position: 39308742 / SNP in Genomic Sequence: SEQ ID NO: 463 /
SNP Position Genomic: 15866 / SNP Source: dbSNP; Celera; HapMap /
Population(Allele,Count): Caucasian (A,73|G,47) / SNP Type: INTRON /
Context (SEQ ID NO: 2791): /
TCAGAAACTGCATCTTTCTTTTTTTTTAAGGAGCAGGATTCCTCTTGCACATGAAATCTTTCCCAGAGCCCCAATCTGAAGCCT
ACTTAAGAGTGGAGCTC
W

GGTTGAAGTAGGGGCAGAGGGCTCCCATTTCCACCAGCCCAGAGAGTGTGATTTCAAGAGCCCTTACCCGTAATTCTGAAATG
GATGCCCTTGTCTAAGT / Celera SNP ID: hCV3054822 / Public SNP ID: rs11751357 /
SNP Chromosome Position: 39296280 / SNP in Genomic Sequence: SEQ ID NO: 463 /
SNP Position Genomic: 3404 / SNP Source: dbSNP; Celera; HapMap /
Population(Allele,Count): Caucasian (T,90|A,28) / SNP Type: INTRON /
Context (SEQ ID NO: 2792): /
CCTCTAATTTCCCCCACATGATGGCAGCCTTGATTCTGAGACTGAAAACTATTAAACTCATTTTTCACCCCTAAACATCTCAC
TGACAGATGGCTGCATG
M

GGAAGGAGCTGGGATTATGTGTCCTCTCAAGATATTTCTAAATAAATTCGTGTCCTCCGGACCCAAAATATACAGTGTTTTTC
ATGACAGTTGACTTCAC / Celera SNP ID: hCV3078064 / Public SNP ID: rs2480089 /
SNP Chromosome Position: 39632196 / SNP in Genomic Sequence: SEQ ID NO: 463 /
SNP Position Genomic: 339320 / SNP Source: dbSNP; Celera; HapMap; ABI_Val;
HGBASE / Population(Allele,Count): Caucasian (C,69|A,157) / SNP Type: INTRON
//

Gene Number: 127 / Gene Symbol: PTRF - 284119 / Gene Name: polymerase I and
transcript release factor / Chromosome: 17 / OMIM NUMBER: / OMIM Information:
// Genomic Sequence (SEQ ID NO: 464): // / SNP Information /
Context (SEQ ID NO: 2793): /
GCAAAAGGTCACTTCTAGTCTGGTATCCATTATCATTAGGAAGAGTGAAAAACTGGTCTATGGGGACCCAGGGCCTCCTCAAC
CCAGACAGAGAACCCAC
M

AACTCAGAAAGGAGACCGACAGTGCAGGGACTGGGTTAGTGCCCTAACAACATTGTAGGGTTTTTTAAGGGTTTACAACACCA
TATTTGTTATCCGAAAT / Celera SNP ID: hCV1952126 / Public SNP ID: rs7223784 /
SNP Chromosome Position: 40577580 / SNP in Genomic Sequence: SEQ ID NO: 464 /
SNP Position Genomic: 33113 / SNP Source: Applera / Population(Allele,Count):
Caucasian (A,21|C,11) African American (A,25|C,13) total (A,46|C,24) // /
SNP Type: INTERGENIC;UNKNOWN / SNP Source: dbSNP; Celera; HapMap; ABI_Val /
Population(Allele,Count): Caucasian (A,164|C,62) / SNP Type:
INTERGENIC;UNKNOWN /
Context (SEQ ID NO: 2794): /
CAGTAGCACAATAGGGTGATAGAGTTAACAGAAATTATTGCGTATTTCAAAATAATTAAAGGAGTGGAATTGGGATGTTCCTA
ACACAAGGAAATGATAA
M

TGCTTGATATGACGGATACTCTAATTACCCTGATTTGATCATTATACATTGCATACTTGTCTCGAAATATCACATGTACTCCA
AAAATATGTACAACTAT / Celera SNP ID: hCV31652026 / Public SNP ID: rs11871801 /
SNP Chromosome Position: 40570772 / SNP in Genomic Sequence: SEQ ID NO: 464 /

SNP Position Genomic: 26305 / Related Interrogated SNP: hCV1952126 / SNP Source: dbSNP / Population(Allele,Count): Caucasian (A,164|C,62) / SNP Type: TFBS SYNONYMOUS;INTRON /
Context (SEQ ID NO: 2795): /
GCCTCCCAAAGTGTTGGGATTACAGGTGTGAGCCACCGCGCCCAGCCCCGTATTTCCATTTTATCCCCACTTTCTGGATCTAA
AAGAGCCCTACTCTCCA
M
ATTGTTGCCTGGAGGGATGGTTAATGGGTACAAAAATACAGTTAGATAGAAGGAATAAGATCTAACGTTCAGTAGCACAATAG
GGTGATAGAGTTAACAG / Celera SNP ID: hCV31652022 / Public SNP ID: rs12948909 /
SNP Chromosome Position: 40570602 / SNP in Genomic Sequence: SEQ ID NO: 464 /
SNP Position Genomic: 26135 / Related Interrogated SNP: hCV1952126 / SNP
Source: dbSNP / Population(Allele,Count): Caucasian (A,164|C,62) / SNP Type:
INTRON //

Gene Number: 128 / Gene Symbol: SLC9A11 - 284525 / Gene Name: solute carrier family 9, member 11 / Chromosome: 1 / OMIM NUMBER: / OMIM Information: // Genomic Sequence (SEQ ID NO: 465): // / SNP Information /
Context (SEQ ID NO: 2796): /
GATAATAAATTATGGTGAAATTCAACAAAAATGTTAAGAGCAATTAATCTGGTCCAGTGATGCTCAACTTTAACATGCATACA
AACTACCTGAAGAGCAT
K
TTAAAACTATTTCCTGGATCTCATATTTAGAGATTTGATTCAGTAGGTTGAGATGGGCTCATGAATATGCATTTCTGTCTAGC
TTATGGGTGACGCCGAT / Celera SNP ID: hCV1681325 / Public SNP ID: rs898657 /
SNP Chromosome Position: 173542017 / SNP in Genomic Sequence: SEQ ID NO: 465 /
SNP Position Genomic: 82413 / Related Interrogated SNP: hCV16180170 /
Related Interrogated SNP: hCV8911768 / SNP Source: dbSNP; Celera; HapMap;
HGBASE / Population(Allele,Count): Caucasian (G,168|T,58) / SNP Type: INTRON
/
Context (SEQ ID NO: 2797): /
GCTGGTAAATACTATCTTATAACCCATCATTTAAAATTGATGACACTTAACACTAATTGCATAAACGAAGAAATAAGCAAAAA
GAAGACTGATAAAAAAC
K
ATACACTTTGACTTTACCCCCCTGCTATTTAACTTTTTGTTGTTTCTTTATATCTTATTGTATGTACTGTCTATGTCTTGAAA
AGTTGTTGAAGTAATTA / Celera SNP ID: hCV1681328 / Public SNP ID: rs10912647 /
SNP Chromosome Position: 173537093 / SNP in Genomic Sequence: SEQ ID NO: 465 /
SNP Position Genomic: 77489 / Related Interrogated SNP: hCV16180170 /
Related Interrogated SNP: hCV8911768 / SNP Source: dbSNP; Celera; HapMap /
Population(Allele,Count): Caucasian (G,168|T,58) / SNP Type: INTRON /
Context (SEQ ID NO: 2798): /
TTTAACTGAATTTACTTTAAATAACCAATGACAATAAAACCAGGAGTGCCGCAAACTGTGATGACAGGGAAACTATCTTTCAA
TTTCCAATTCTCTATAA
Y
TGGTATTTTATAAAGAAGGGACAGTGGAAGTGATAAGCCCCAGTTACAGGCAACAAGGGGATGCATTGTTTGTAGACTTTTAA
AACAATTATAAAGATGA / Celera SNP ID: hCV9575253 / Public SNP ID: rs1031751 /
SNP Chromosome Position: 173547126 / SNP in Genomic Sequence: SEQ ID NO: 465 /
SNP Position Genomic: 87522 / Related Interrogated SNP: hCV16180170 /
Related Interrogated SNP: hCV8911768 / SNP Source: dbSNP; HapMap; ABI_Val;
HGBASE / Population(Allele,Count): Caucasian (T,112|C,4) / SNP Type: INTRON
/
Context (SEQ ID NO: 2799): /
CTACCATCAGTGGGCAATGCCATTCTAGAATAAGGATGCTCATGGAGTCAGGTTTAAATACCAAATTTGCTCTTATGAGTTGA
GTGACTCTAGGCAGGTT
Y
TTACATTCTCTGGGCTTCGTCTGTGAAGTGATGCAATAACACATACATCACAAAGTGATATTCACATCAATGGCATACAGTAC
ATGGTAACTGCATTATC / Celera SNP ID: hCV9575263 / Public SNP ID: rs898658 /
SNP Chromosome Position: 173541768 / SNP in Genomic Sequence: SEQ ID NO: 465 /
SNP Position Genomic: 82164 / Related Interrogated SNP: hCV16180170 /
Related Interrogated SNP: hCV8911768 / SNP Source: dbSNP; Celera; HapMap;
ABI_Val; HGBASE / Population(Allele,Count): Caucasian (T,168|C,58) / SNP Type:
INTRON /
Context (SEQ ID NO: 2800): /
GTTTTAAAACCTACTGATAATTTCTTTGGGGAATCCTTTCATTATTCTGAAGCTTCAAAAGTTGTGTCTTATCCTGAGAAAAT
AAGGATAGCCACAATCA

Y

GGCAAAAACTCACTTCACTGAGCATTTACTATGTGCCAGGCATGGTGCTAAATTCTTGTACTTGCATTACCTCATTGAATCCT
CACAGCAACCCTCTGAG / Celera SNP ID: hCV9575269 / Public SNP ID: rs898659 /
SNP Chromosome Position: 173541076 / SNP in Genomic Sequence: SEQ ID NO: 465 /
SNP Position Genomic: 81472 / SNP Source: dbSNP; Celera; HapMap; HGBASE /
Population(Allele,Count): Caucasian (T,85|C,33) / SNP Type: INTRON /
Context (SEQ ID NO: 2801): /
CTTGAAGATAAACTATAAACCCCTCCACCACCTGCCAAACCTTAGCTATTCCAAATGGAGGCAGTTCTAACAAGAGGTAGCAA
GGCCTTGGAATAGTCTC

K

TTAGGGATCTTTAATGTTTCCCCTTATTGGTAAACAAACTCACCAACTTAGAATATCTACCTTGTGGCTAAGAATTCACTGGA
ACAGAATAATAATGCCA / Celera SNP ID: hCV28998001 / Public SNP ID: rs6425251 /
SNP Chromosome Position: 173556428 / SNP in Genomic Sequence: SEQ ID NO: 465 /
SNP Position Genomic: 96824 / Related Interrogated SNP: hCV16180170 /
Related Interrogated SNP: hCV8911768 / SNP Source: dbSNP; HapMap /
Population(Allele,Count): Caucasian (T,168|G,58) / SNP Type: INTRON /
Context (SEQ ID NO: 2802): /
ATCTGGTTAAGATGATGCCACACATTGACTGGAAGCATCTCCATTTTTTTAGAAGGAAAAGGGAATAGGAACTCATATTTATTG
ACTGCCCTACCTTGAGC

Y

CAGTGATGTGCTGGTAAATGTTTAAAGCCGGCTCTTTTCTGCAATTATACAGGCAGTAAGGGAAAAAAGCTTTTTAAAACCCA
GTTCAAGGTTGGGGCGG / Celera SNP ID: hCV30804119 / Public SNP ID: rs10912651 /
SNP Chromosome Position: 173549157 / SNP in Genomic Sequence: SEQ ID NO: 465 /
SNP Position Genomic: 89553 / Related Interrogated SNP: hCV16180170 /
Related Interrogated SNP: hCV8911768 / SNP Source: dbSNP; HapMap /
Population(Allele,Count): Caucasian (C,87|T,31) / SNP Type: INTRON /
Context (SEQ ID NO: 2803): /
GAGGCTGGCTTATTTCTCTTAGCACAATGCATTTTAAATTCTTCCTTGTTGTGCCTATCGATACTCCATTCCTTTTTTTATTGC
TGAGTAATATTTCATTG

W

AAGATGTTCCACGATTTGTTTATTCATTCACCAATGGAAGAATGTTTGTTTCCAGACATTTGAGATTATAAATAAAGCTGCTA
TACAGATTCATATAAAG / Celera SNP ID: hCV30804135 / Public SNP ID: rs12078293 /
SNP Chromosome Position: 173565287 / SNP in Genomic Sequence: SEQ ID NO: 465 /
SNP Position Genomic: 105683 / Related Interrogated SNP: hCV16180170 /
Related Interrogated SNP: hCV8911768 / SNP Source: dbSNP; HapMap /
Population(Allele,Count): Caucasian (A,165|T,61) / SNP Type:
UTR3;INTRON;PSEUDOGENE /
Context (SEQ ID NO: 2804): /
GCACACATTGTTTATGCCCAAGTATAAGAAAGTATTTATTTTTCAAAGTCATCCTCAGGAAAAAAAATAGTTGTGTTATTCAA
TCCTTTTCTTATTTTCC

R

TCTCTGTCTTTTGTTTTACTTTCTGTGAGGTTTTCACAATTTGACCATTAGGTCCTTTTATTTCTTTATCTATTGTAACGTCA
TATGGTTTTTCTTCTTA / Celera SNP ID: hCV30804128 / Public SNP ID: rs10798290 /
SNP Chromosome Position: 173557584 / SNP in Genomic Sequence: SEQ ID NO: 465 /
SNP Position Genomic: 97980 / SNP Source: dbSNP; HapMap /
Population(Allele,Count): Caucasian (A,85|G,33) / SNP Type: INTRON /
Context (SEQ ID NO: 2805): /
CACAACCTGATAGTCAAGGAGCATCTGTACTTCTTACAGGTAAGACCAGATAAAGATATGTTTTTTACACTCTTTCTGCAATG
CTCTTTACTACTGGAGC

R

TTTGTCTCTATAAAGGATATAGAGCTAGTCCATGGTCATCAGCAACTTGTGATATCAAAAGTGACCAAGGTCATTAGTGAGTA
TATTAAAGATTCCCAAA / Celera SNP ID: hCV30804139 / Public SNP ID: rs12089930 /
SNP Chromosome Position: 173569875 / SNP in Genomic Sequence: SEQ ID NO: 465 /
SNP Position Genomic: 110271 / Related Interrogated SNP: hCV16180170 /
Related Interrogated SNP: hCV8911768 / SNP Source: dbSNP /
Population(Allele,Count): Caucasian (G,163|A,61) / SNP Type: INTRON /
Context (SEQ ID NO: 2806): /
TTTTTAATGGAGTTGTTTTCTTGTAAATTTGTCTAAATTCCTTATAGATGCTAGATATTAGACCTTTGTCACATGCATAGTTT
GCAAATATTTTATCCTA

Y

TCTATAGGCTGTTTCCTCTGTTGATAATTTCTTTTGCTGTGCATAAGCCCTTAAGTTTAATTAAATCCTATTTGTTAATTTTT
GCTTTTGTTGCAGTTGC / Celera SNP ID: hCV29517287 / Public SNP ID: rs2901747 /
SNP Chromosome Position: 173560948 / SNP in Genomic Sequence: SEQ ID NO: 465 /

SNP Position Genomic: 101344 / Related Interrogated SNP: hCV16180170 / Related Interrogated SNP: hCV8911768 / SNP Source: dbSNP; HapMap / Population(Allele,Count): Caucasian (T,80|C,30) / SNP Type: INTRON / Context (SEQ ID NO: 2807): /
TATTTTTATTGAGTTTTTCTGAATATTTTTGATCTGCAGTTGGTTGAATCCGTGGATGCAGATGGTCAACTATGATTTTTTTC TTGTTACTTCATATATC
R
TTGTAATCAGCCCAAAAGTAAAGTATAGAGAAGGCAGCAAAGTTAAAACATTTGAATGTATTTTAATTTTAATGAGAATGATT TTAGAAAATATACAGAC / Celera SNP ID: hCV29644010 / Public SNP ID: rs7541082 / SNP Chromosome Position: 173552529 / SNP in Genomic Sequence: SEQ ID NO: 465 / SNP Position Genomic: 92925 / SNP Source: dbSNP; HapMap / Population(Allele,Count): Caucasian (A,85|G,33) / SNP Type: INTRON / Context (SEQ ID NO: 2808): /
TCATAGACCTTTAATGAAAAGGACACAAAATCACAGCTAGATAGGAGTAAGTTCTAGTGTTCTATAACACTGCAGGATGACTA TAGTTAACAGCAATATA
Y
TATATAGTTTCACATAGCTAGAAGGAAGACATTGAATGTTCCCTACATGAAGAAATAATGTTTCAGAAGATAAATATGCTAAT TACCCTGATCTGATCAT / Celera SNP ID: hCV30804131 / Public SNP ID: rs12090597 / SNP Chromosome Position: 173563418 / SNP in Genomic Sequence: SEQ ID NO: 465 / SNP Position Genomic: 103814 / SNP Source: dbSNP; HapMap / Population(Allele,Count): Caucasian (C,87|T,31) / SNP Type: INTRON //

Gene Number: 129 / Gene Symbol: SCARA5 - 286133 / Gene Name: scavenger receptor class A, member 5 (putative) / Chromosome: 8 / OMIM NUMBER: / OMIM Information: // Genomic Sequence (SEQ ID NO: 466): // / SNP Information / Context (SEQ ID NO: 2809): /
TGTCTGCAGCCACCTTACATTGCAGCGGTCAGAGAGGAAAACAGCAATGAAGAGGGTTAGAAAAGCCACATTTGATGACCACC TTTGTAATAACTGACTC
R
GGCAAGAATTGTAAGTGGATGCTAAAAATCATGGAGTGAAGGATTGCTGGCAATCAGTGTATTTACACAGCCTCAGAGTGTCTC CCCACAGATCACTTCTT / Celera SNP ID: hCV16282389 / Public SNP ID: rs2726953 / SNP Chromosome Position: 27801305 / SNP in Genomic Sequence: SEQ ID NO: 466 / SNP Position Genomic: 83569 / SNP Source: dbSNP; HapMap; HGBASE / Population(Allele,Count): Caucasian (G,77|A,29) / SNP Type: INTRON //

Gene Number: 130 / Gene Symbol: ANKRD45 - 339416 / Gene Name: ankyrin repeat domain 45 / Chromosome: 1 / OMIM NUMBER: / OMIM Information: // Genomic Sequence (SEQ ID NO: 467): // / SNP Information / Context (SEQ ID NO: 2810): /
CTGTGACTGTGGTTGTATCTACAAACAAAACACTGTGAAAAAAGGTGACATATCCTGTAACTCATAGGAGAAAAACTCATCAC ATCAGTCAGTTTGCTAA
Y
CCCTTGTGAGCTTCCTTACCTGCCCAGTCCAGGAATTCAACACACTCAGTCTGAGAATATCTAGCAGCAACATCTCGAGCCCT TTCTTCCCGGAAGTTCA / Celera SNP ID: hCV25600635 / Public SNP ID: rs7539322 / SNP Chromosome Position: 173615965 / SNP in Genomic Sequence: SEQ ID NO: 467 / SNP Position Genomic: 48490 / Related Interrogated SNP: hCV16180170 / Related Interrogated SNP: hCV8911768 / SNP Source: Applera / Population(Allele,Count): Caucasian (C,36|T,4) African American (C,34|T,2) total (C,70|T,6) / SNP Type: INTRON / SNP Source: dbSNP; HapMap / Population(Allele,Count): Caucasian (C,207|T,19) / SNP Type: INTRON / Context (SEQ ID NO: 2811): /
CACAACCTGATAGTCAAGGAGCATCTGTACTTCTTACAGGTAAGACCAGATAAAGATATGTTTTTTACACTCTTTCTGCAATG CTCTTTACTACTGGAGC
R
TTTGTCTCTATAAAGGATATAGAGCTAGTCCATGGTCATCAGCAACTTGTGATATCAAAAGTGACCAAGGTCATTAGTGAGTA TATTAAAGATTCCCAAA / Celera SNP ID: hCV30804139 / Public SNP ID: rs12089930 / SNP Chromosome Position: 173569875 / SNP in Genomic Sequence: SEQ ID NO: 467 / SNP Position Genomic: 2400 / Related Interrogated SNP: hCV16180170 / Related Interrogated SNP: hCV8911768 / SNP Source: dbSNP / Population(Allele,Count): Caucasian (G,163|A,61) / SNP Type: INTRON //

Gene Number: 131 / Gene Symbol: OTOG - 340990 / Gene Name: otogelin / Chromosome: 11 / OMIM NUMBER: 604487 / OMIM Information: // Genomic Sequence

(SEQ ID NO: 468): // / SNP Information /
Context (SEQ ID NO: 2812): /
AGCAGGCCAGGTCTTCGTGAACTGCAGCGACCTGCACACGGACCTGGAGCTGAGCAGGGAGAGGACGTGTGAGCAGCAACTGC
TGAACCTGAGCGTGTCA
R
CCCGTGGCCCCTGCCTCTCGGGCTGCGCCTGTCCCCAGGGGTAAGTACCCATGGTGTCGTGGGCCCGTGATCCTGAAGGCTGG
CAGAACCAAGGGCCACA / Celera SNP ID: hCV15990789 / Public SNP ID: rs2355466 /
SNP Chromosome Position: 17600033 / SNP in Genomic Sequence: SEQ ID NO: 468 /
SNP Position Genomic: 41113 / SNP Source: Applera / Population(Allele,Count):
Caucasian (A,10|G,26) African American (A,7|G,23) total (A,17|G,49) / SNP
Type: MISSENSE MUTATION;UTR5 / SNP Source: dbSNP; HapMap; HGBASE /
Population(Allele,Count): Caucasian (G,77|A,41) / SNP Type: MISSENSE
MUTATION;UTR5 /
Context (SEQ ID NO: 2813): /
CATAACTGTGGGGATGAGGGAGACAAAGCATGCACCACTCCCCCCTCACTGCAGCACTGTCCTGTGAGGCCTCCAAGGAGTAT
AGCCCCTGCGTGGCCCC
R
TGTGGACGTACCTGCCAGGACCTGGCCAGCCCTGAGGCCTGTGGGGTTGATGGTGGCGATGACCTGAGCAGAGACGAGTGTGT
GGAGGGCTGTGCCTGCC / Celera SNP ID: hCV25743768 / Public SNP ID: rs4757548 /
SNP Chromosome Position: 17596313 / SNP in Genomic Sequence: SEQ ID NO: 468 /
SNP Position Genomic: 37393 / Related Interrogated SNP: hCV15990789 / SNP
Source: Applera / Population(Allele,Count): Caucasian (A,9|G,29) African
American (A,15|G,23) total (A,24|G,52) / SNP Type: ESS;ESE;SILENT RARE
CODON;SILENT MUTATION / SNP Source: dbSNP; HapMap; HGBASE /
Population(Allele,Count): Caucasian (G,138|A,86) / SNP Type: ESS;ESE;SILENT
RARE CODON;SILENT MUTATION /
Context (SEQ ID NO: 2814): /
ATTTTTTTATAGGAACCAGTGCTCCTGCCACTTCCAGGGAGTGGACTATCCCCCCGGAGACAGTGACATCCCATCCCTGGGCC
ACTGGTGAGCTCCGTAG
R
TAGCAGCCTTCTTGTCCTCTCTTTAAAGGGAGGCTGCTGACTGAAGACAGTGCAGCTGATGGAACTTTCTGTGAAGGGACACC
CAGCCCTGCTCTGTGCA / Celera SNP ID: hCV25768364 / Public SNP ID: rs4756902 /
SNP Chromosome Position: 17598191 / SNP in Genomic Sequence: SEQ ID NO: 468 /
SNP Position Genomic: 39271 / SNP Source: Applera / Population(Allele,Count):
Caucasian (A,10|G,28) African American (A,14|G,22) total (A,24|G,50) // /
SNP Type: INTRON / SNP Source: dbSNP; HapMap; HGBASE /
Population(Allele,Count): Caucasian (G,134|A,86) / SNP Type: INTRON //

Gene Number: 132 / Gene Symbol: ZBTB41 - 360023 / Gene Name: zinc finger
and BTB domain containing 41 / Chromosome: 1 / OMIM NUMBER: / OMIM
Information: // Genomic Sequence (SEQ ID NO: 469): // / SNP Information /
Context (SEQ ID NO: 2815): /
AGGCAGATCAGAAGTAGCCAGAAACAAAACAAAGTCCTCTCATATTCTATTTGCTTGTGAGAAAAAATTTAAATCTAACTGGA
AGAAACACATCTTCAAG
R
GCTTCTGTAATTTTTAACTCACAAAATCTAAAGTTCAATACAAAGTGACCCAAATGATCAAATGCCAAGAGGGGAAAAAATAC
AATACACACACTCAAAG / Celera SNP ID: hCV268763 / Public SNP ID: rs4350226 /
SNP Chromosome Position: 197132378 / SNP in Genomic Sequence: SEQ ID NO: 469 /
SNP Position Genomic: 19564 / Related Interrogated SNP: hCV2532034 / SNP
Source: dbSNP; Celera; HapMap; ABI_Val; HGBASE / Population(Allele,Count):
Caucasian (G,10|A,110) / SNP Type: INTRON /
Context (SEQ ID NO: 2816): /
GAACTCCTGGGCTCAAGCAAATCATCCACCTCGACTTCCCAAAGCGCTGGGATTACAGGGGTTTGTGAGCCATCGCACCAAGC
ATGAACAGGATATATTA
Y
ATCTTCAATACACCATGAACAGGATAAACAAGATAATATATCATTAGCAAGATACTATATCAAACCAGTCTGGGTTTATTCCA
GAAATGAGGTTTAATAT / Celera SNP ID: hCV356522 / Public SNP ID: rs10732295 /
SNP Chromosome Position: 197136645 / SNP in Genomic Sequence: SEQ ID NO: 469 /
SNP Position Genomic: 23831 / Related Interrogated SNP: hCV2532034 / SNP
Source: dbSNP; Celera; HapMap; ABI_Val / Population(Allele,Count): Caucasian
(T,21|C,205) / SNP Type: INTRON /
Context (SEQ ID NO: 2817): /
CATTATTCCTTCCCATCATATTAGATCCTAAATCTAGTTCACTGAATCTATCAAATGTCTAAGGTTATTATCTAGCTGACTGG

CCAAGACTCAAAAATAT

M

AAAAGTAGGAGTAATCAGAAAGGAATTAGAATCAGACAAATGAAGTAAGTGTACAAGTGACTAGGCATATCATGATGATAGCA AATGATACAGTAAATGG / Celera SNP ID: hCV1739697 / Public SNP ID: rs10429911 / SNP Chromosome Position: 197146144 / SNP in Genomic Sequence: SEQ ID NO: 469 / SNP Position Genomic: 33330 / Related Interrogated SNP: hCV2532034 / SNP Source: dbSNP; Celera; HapMap / Population(Allele,Count): Caucasian (A,10|C,110) / SNP Type: INTRON / Context (SEQ ID NO: 2818): / TTAATTCACATCAAGCACTGTGTTAAGTATTATGAAAAATCTCTCCACCTCCTCTGAACCAAAAAAAGGCTCTAAGAAATTGA GAAAACGAAAGTCAGTA

Y

TTACATAGCTGAAAAAAAAAGGTGAAGTAAATACACGTGCCAAAATAAGGTTATTATGCAAAATGTGTGGTTAGTCAAAGAAT GAAATATCTACTAGTAA / Celera SNP ID: hCV1739698 / Public SNP ID: rs1415217 / SNP Chromosome Position: 197151246 / SNP in Genomic Sequence: SEQ ID NO: 469 / SNP Position Genomic: 38432 / Related Interrogated SNP: hCV2532034 / SNP Source: dbSNP; Celera; HapMap; ABI_Val; HGBASE / Population(Allele,Count): Caucasian (C,10|T,110) / SNP Type: INTRON / Context (SEQ ID NO: 2819): / ATAGCAGGGGAGCTAAGCCTTACTGCCTAACATTTCTGGAACACCTCTCATTATTGGTATGCTGCAGCATGATAGAAGAATCT CTTAACAAGTCATAAAA

R

TCTTCCAGTTCAACCACTTCTGAAGAATGCCCTCTTCACCAGCTCTAACAGTTAGTAAGGTAAGTTCAGCATATGCTTGTATA CTTGTATAATTTCACTG / Celera SNP ID: hCV1739699 / Public SNP ID: rs7520503 / SNP Chromosome Position: 197152989 / SNP in Genomic Sequence: SEQ ID NO: 469 / SNP Position Genomic: 40175 / Related Interrogated SNP: hCV2532034 / SNP Source: dbSNP; Celera; HapMap / Population(Allele,Count): Caucasian (A,21|G,203) / SNP Type: INTRON / Context (SEQ ID NO: 2820): / TCATAAACCCACCCCAAAGAAGCCAAGTTAGAAGCTTAAAGAAGGCTAATTGGCTTCTTACAAGCCCAAAGTCAAGCCAGATA CCAACTTTTCAACTTAG

Y

TCAAATAAACAAGTGCTTACTAAAAATTTATTTGAAATGACCTGAGATGTTACAAAGAATACTAGCCAAGAGTAAGATACGTA ACCTCCAGGCTCACAAT / Celera SNP ID: hCV1742540 / Public SNP ID: rs10754219 / SNP Chromosome Position: 197165186 / SNP in Genomic Sequence: SEQ ID NO: 469 / SNP Position Genomic: 52372 / Related Interrogated SNP: hCV2532034 / SNP Source: dbSNP; Celera; HapMap; ABI_Val / Population(Allele,Count): Caucasian (C,9|T,109) / SNP Type: INTRON / Context (SEQ ID NO: 2821): / TTGTAGCTATTGAATATTAAAATAAGCAATATTACTTCAAGCCTGTTATCAAAATAAGTTTTATTTATTATTAAACAATTTCTT AGAGTAAATCACAGAAT

R

GTTAAAACATTACCTTTTTCTTTTTCTGCTCTTCTGCATTTCCTAGTAATATAGCTTGGTGTTTCAGAACATCATTTACAAGA AATGTCATAATCTCTCT / Celera SNP ID: hCV2759678 / Public SNP ID: rs1571964 / SNP Chromosome Position: 197113073 / SNP in Genomic Sequence: SEQ ID NO: 469 / SNP Position Genomic: 259 / Related Interrogated SNP: hCV2532034 / SNP Source: dbSNP; Celera; HapMap; ABI_Val; HGBASE / Population(Allele,Count): Caucasian (G,20|A,206) / SNP Type: INTRON / Context (SEQ ID NO: 2822): / GCTTGTGATATATACCAAACTGCATGAAGCACTTATCTGAAGTGTACAGTGATTCAAGGGAACAGAAACATAGAGACGGAATG CAAAAGAAGCTCTCT

M

TATATATATAATATTGCTTTGAGTATTTTATATATGCATGTATACATATATGTATACATATGTACACACACACAAATACAGAA AATACTTATAACAATAC / Celera SNP ID: hCV8350843 / Public SNP ID: rs1556763 / SNP Chromosome Position: 197152149 / SNP in Genomic Sequence: SEQ ID NO: 469 / SNP Position Genomic: 39335 / Related Interrogated SNP: hCV2532034 / SNP Source: dbSNP; Celera; HapMap; HGBASE / Population(Allele,Count): Caucasian (A,21|C,203) / SNP Type: INTRON / Context (SEQ ID NO: 2823): / AAAGACAGAGAGAGAGAGAGTGTGTGTTATAAAGACTAAAAAGGGGAAAATGAACCTGTCATTATTCACAGATAACTTGTTTG CATGCTTTGAAAGTTCA

R

AAAAAGCTAGGGGTAAACTATAAAATTTAATAGAAATTTAGCAAGGTCACTGGATAAAAAGTCAATAAACAAACTCAATTCTA

TTCCTGTATACCAGAAA / Celera SNP ID: hCV26961419 / Public SNP ID: rs10732296 / SNP Chromosome Position: 197137151 / SNP in Genomic Sequence: SEQ ID NO: 469 / SNP Position Genomic: 24337 / Related Interrogated SNP: hCV2532034 / SNP Source: dbSNP; Celera; HapMap / Population(Allele,Count): Caucasian (A,10|G,110) / SNP Type: INTRON / Context (SEQ ID NO: 2824): / AATTCTCTGAAACTTAAATTTTTAAATGTATTTTTAAACTTAAAATTAACTTTTAGCAAAAGATCAATTCATCATTTAAGATT TATTATGTTCATATTTT Y

GGTATCTCTGTGGGTCCATACACCTGGATATGGATACTGGCTCTCTAAATAGAGGGTTACGTGACCTTGGACAAGTAACCTCT ATATGCCTCAAATCTTC / Celera SNP ID: hCV26961484 / Public SNP ID: rs4915379 / SNP Chromosome Position: 197161428 / SNP in Genomic Sequence: SEQ ID NO: 469 / SNP Position Genomic: 48614 / Related Interrogated SNP: hCV2532034 / SNP Source: dbSNP; Celera; HapMap; HGBASE / Population(Allele,Count): Caucasian (T,21|C,201) / SNP Type: INTRON / Context (SEQ ID NO: 2825): / AGCAGAAGGAAATAAACAATACTCAAAGAGATGTAAACTAATAGTTAAAATCGTAGAATCTATGAGCCTTTAGAATTTAAAAA TGTCATCCACAGAATCA Y

CTTTCTTTAGATATTAAAAATTGGTATGTGAAAAATGTGCATTGGGAGACTTATTTCAGACTTATTTCAGACTTACATCATAT CATGGCTCTTACTGATG / Celera SNP ID: hCV28005188 / Public SNP ID: rs4342879 / SNP Chromosome Position: 197131655 / SNP in Genomic Sequence: SEQ ID NO: 469 / SNP Position Genomic: 18841 / Related Interrogated SNP: hCV2532034 / SNP Source: dbSNP; HapMap / Population(Allele,Count): Caucasian (T,10|C,110) / SNP Type: INTRON / Context (SEQ ID NO: 2826): / TAGAACAGATAGTCTCTAAATCATTTTGTTTGAGTGGTTTCAAAAGATTTAATTGTACATTTCTAATTTTTAGATGTGTAGAG TACTTCTACTTCAAACA Y

AAGGACCCTGCATTATGAAGACTTTATTTAAAAAGAAATGTAAAGATGATCAGCTAAATTCCTTCTTCCTTACAAATTCCTCT AAGGTCCACATCATCAT / Celera SNP ID: hCV29222817 / Public SNP ID: rs6656858 / SNP Chromosome Position: 197113573 / SNP in Genomic Sequence: SEQ ID NO: 469 / SNP Position Genomic: 759 / Related Interrogated SNP: hCV2532034 / SNP Source: dbSNP; HapMap / Population(Allele,Count): Caucasian (C,20|T,206) / SNP Type: INTRON / Context (SEQ ID NO: 2827): / CAACATAAATATGTACAAAACACTTTAACTTCCTCACAGTGGACTTAAGATAAATTCTGTAGGGCCTTGCATGGCTAAGACCC TATGAAAATATACCTTG Y

AATTACCACATGGATGCTAAGCCATACAGATTTTCAAGGAAGGAGAGGATAAACAATTTGAACTGAAGCAGAGATGTCAGTAA AAAAAGAACTTAAAAAT / Celera SNP ID: hCV29295007 / Public SNP ID: rs6656448 / SNP Chromosome Position: 197143278 / SNP in Genomic Sequence: SEQ ID NO: 469 / SNP Position Genomic: 30464 / Related Interrogated SNP: hCV2532034 / SNP Source: dbSNP / Population(Allele,Count): Caucasian (C,9|T,107) / SNP Type: INTRON / Context (SEQ ID NO: 2828): / TGATGGGAAAGTAACTATTGTGTAAGACAAGTTACTTTAGTTTGTAAGTTCCAACACTTAAAAAGTTATTATAGTTCCCTCTG ATGTTTCTACAAGGTAG R

ACCAAAAAGATTACCTAAAATTTTTCAAATCAAAAAGAAGGAAGTGCACCAATTCTCCAATAAAAACTACTTTTTCTGGAACTA AACTCTAAAGAAATTTC / Celera SNP ID: hCV30321245 / Public SNP ID: rs7523013 / SNP Chromosome Position: 197153188 / SNP in Genomic Sequence: SEQ ID NO: 469 / SNP Position Genomic: 40374 / Related Interrogated SNP: hCV2532034 / SNP Source: dbSNP; HapMap / Population(Allele,Count): Caucasian (A,10|G,110) / SNP Type: INTRON / Context (SEQ ID NO: 2829): / GATTTAGCAAACTTATATACACATCCAAATCCACACACAAAACTAGAAAAAAGTTTACGTAAATTTCTACCTCTGATAAACCT AATGACTTACTATTTTG K

GCTTCCTGTTCAAAGTGTACTTTTCCTCAAAAACTTGAGTCATTAAAAAAAAAATATTCATGATCACACATTTTAAAAATTAAG AGGTTTGATACATTTTC / Celera SNP ID: hCV32375831 / Public SNP ID: rs4915378 / SNP Chromosome Position: 197161212 / SNP in Genomic Sequence: SEQ ID NO: 469 / SNP Position Genomic: 48398 / Related Interrogated SNP: hCV2532034 / SNP

Source: dbSNP; HapMap; HGBASE / Population(Allele,Count): Caucasian (T,8|G,104) / SNP Type: INTRON /
Context (SEQ ID NO: 2830): /
TGCATACTAAAGTTTTTGGGAAAGCGGGTAATAAAAAACTTTGGAAGAATGAATGGAGGACTTAAGAGAGTTTATAGTTTGGA
AATATTTCTATTTGGTG
R
TACAAAATTAAGCCCAGGCCTCTCATAGTAACACTGACATTAGTTGGATACTGAATTTCACTATGATTTTACCAAATAAAAAA
CTGGAGACAAGACAGTG / Celera SNP ID: hCV29491389 / Public SNP ID: rs7513826 /
SNP Chromosome Position: 197150638 / SNP in Genomic Sequence: SEQ ID NO: 469 /
SNP Position Genomic: 37824 / Related Interrogated SNP: hCV2532034 / SNP
Source: dbSNP; HapMap / Population(Allele,Count): Caucasian (A,10|G,110) /
SNP Type: INTRON //

Gene Number: 133 / Gene Symbol: LOC400499 - 400499 / Gene Name:
hypothetical protein LOC400499 / Chromosome: 16 / OMIM NUMBER: / OMIM
Information: // Genomic Sequence (SEQ ID NO: 470): // / SNP Information /
Context (SEQ ID NO: 2831): /
GCCCAGCGTTAGCTCCAGGACAGCCTGGAACACAGCTCGGTGTGGCCAGTATAGCCTGTGAGCTGCAGAGACCATCAGCCATG
GACTATCCAGGTTCAAG
R
CTCCTGCAAGCAACAAAGAAGGAGAATCTGGTACACGGGGTAGAGAGGAAGCTTCTAGCAGGCAGCTGGAGGTCCCGAGAGTC
TCATCACAGCCGCAATG / Celera SNP ID: hCV11286902 / Public SNP ID: rs12932948 /
SNP Chromosome Position: 11544673 / SNP in Genomic Sequence: SEQ ID NO: 470 /
SNP Position Genomic: 64286 / SNP Source: Applera / Population(Allele,Count):
Caucasian (A,16|G,14) African American (A,8|G,20) total (A,24|G,34) / SNP
Type: MISSENSE MUTATION;ESE SYNONYMOUS / SNP Source: Applera /
Population(Allele,Count): Caucasian (A,22|G,18) African American (A,9|G,29)
total (A,31|G,47) / SNP Type: MISSENSE MUTATION;ESE SYNONYMOUS / SNP Source:
dbSNP; Celera; HapMap / Population(Allele,Count): Caucasian (G,88|A,138) / SNP
Type: MISSENSE MUTATION;ESE SYNONYMOUS //

Gene Number: 134 / Gene Symbol: LOC100505872 - 100505872 / Gene Name:
hypothetical protein LOC100505872 / Chromosome: 1 / OMIM NUMBER: / OMIM
Information: // Genomic Sequence (SEQ ID NO: 471): // / SNP Information /
Context (SEQ ID NO: 2832): /
CCCGGGGACGATGGGGCAAGTGATGCCCATGTCGGTGCATGCCTTCACAAAGCGGAAGAATGTGTCAGCCTCAAAGAAAAGCT
GCGTGATGATGAAATCG
R
CTCCCGCAGACACCTTCTCCTTCAAGTGCTTCAGGTCAGCCTCAAAGCTCCCTGCTTCGGGGTGGCCTTTGGGGTAACCTGCC
AATAGGGATGACAGTCA / Celera SNP ID: hCV1202883 / Public SNP ID: rs1801133 /
SNP Chromosome Position: 11856378 / SNP in Genomic Sequence: SEQ ID NO: 471 /
SNP Position Genomic: 227693440 / SNP Source: Applera /
Population(Allele,Count): Caucasian (A,14|G,24) African American (A,8|G,30)
total (A,22|G,54) / SNP Type: MISSENSE MUTATION;ESE;UTR5 / SNP Source: HGMD;
dbSNP; Celera; HapMap; ABI_Val; HGBASE / Population(Allele,Count): Caucasian
(G,156|A,70) / SNP Type: MISSENSE MUTATION;ESE;UTR5 /
Context (SEQ ID NO: 2833): /
TTTTGTGACCTCCAAAGGACTCACAGGAATGACCTCCAACACCTTTGAGAAGACCAGGCCCTCTCCCTGGTAGTTACAGTCAA
AGACCTGTTTGGAAGAC
R
TCATTTCAAGTGCTCTCCCTCCCACCCCACCTCTTGGGGTAAGGCCTTTCCTAAGCTACCCCTTGGGTCCCTAGCCTAAGAAA
CAAGGGGGATGTCATCC / Celera SNP ID: hCV16180170 / Public SNP ID: rs2227589 /
SNP Chromosome Position: 173886216 / SNP in Genomic Sequence: SEQ ID NO: 471 /
SNP Position Genomic: 65663602 / SNP Source: Applera /
Population(Allele,Count): Caucasian (G,38|A,2) African American (G,35|A,3)
total (G,73|A,5) / SNP Type: INTRON / SNP Source: dbSNP; HapMap; HGBASE /
Population(Allele,Count): Caucasian (G,203|A,23) / SNP Type: INTRON /
Context (SEQ ID NO: 2834): /
AGTAAGTGACAGGAGCACATCTTCTTGTCCAAAATCATTGGAAATACATAATTGTGACAGTTTCCCAAACATATTCCTTTCGT
TTTCAAAATAATTTTTA
R
AAGTAGTAGTGTCTTCTAGTGGAGGAACTTCCAAATCAACTAACTGGTCCTTGAACTTAAGTTTTCGCTCCCTTTTATCATTC
ACCGGTTCTTGATTCTG / Celera SNP ID: hCV2456747 / Public SNP ID: rs3820059 /

512

SNP Chromosome Position: 169391154 / SNP in Genomic Sequence: SEQ ID NO: 471 / SNP Position Genomic: 70158664 / SNP Source: Applera / Population(Allele,Count): Caucasian (A,12|G,22) African American (A,18|G,4) total (A,30|G,26) / SNP Type: MISSENSE MUTATION;ESS;TFBS SYNONYMOUS / SNP Source: Applera / Population(Allele,Count): Caucasian (A,6|G,30) African American (A,6|G,12) total (A,12|G,42) / SNP Type: MISSENSE MUTATION;ESS;TFBS SYNONYMOUS / SNP Source: Applera / Population(Allele,Count): Caucasian (A,8|G,30) African American (A,9|G,11) total (A,17|G,41) / SNP Type: MISSENSE MUTATION;ESS;TFBS SYNONYMOUS / SNP Source: dbSNP; Celera; HapMap; ABI_Val; HGBASE / Population(Allele,Count): Caucasian (A,81|G,145) / SNP Type: MISSENSE MUTATION;ESS;TFBS SYNONYMOUS /
Context (SEQ ID NO: 2835): /
GGCTTGGAAGTCCCCTTCCACGCTTTACTGTGCTCCTGAGCAGCCGGGGGGTAGCGACTCAGCACTGAGGGCTGCTCCCTAGA
CTTCTTCCCTTCGCTCT
K
GGAGGAGCCGGGTACAAACCGGTCTTCAGTTTTTCTTTGTCTCCTGAGTCCCACTGTCTGTACTCACCCCCATGTGAGAAGCTT
TTGCTGCCCTCCTGTTG / Celera SNP ID: hCV2494846 / Public SNP ID: rs3765407 /
SNP Chromosome Position: 23419374 / SNP in Genomic Sequence: SEQ ID NO: 471 / SNP Position Genomic: 216130444 / SNP Source: Applera / Population(Allele,Count): Caucasian (G,8|T,30) African American (G,15|T,23) total (G,23|T,53) / SNP Type: MISSENSE MUTATION;TFBS SYNONYMOUS / SNP Source: dbSNP; Celera; HapMap; HGBASE / Population(Allele,Count): Caucasian (G,36|T,190) / SNP Type: MISSENSE MUTATION;TFBS SYNONYMOUS /
Context (SEQ ID NO: 2836): /
TAAATAATTTTTTTTCCCATAGAAAAATGCACTAAGCCTGACCTGAGTAATGGTTACATCTCTGATGTAAAGTTATTGTATAA
AATTCAAGAGAACATGC
R
TTATGGTTGCGCTTCAGGGTACAAAACCACTGGAGGGAAGGATGAAGAAGTGGTTCAATGTCTCTCTGATGGATGGTCTTCTC
AACCAACCTGTAGGAAA / Celera SNP ID: hCV2532034 / Public SNP ID: rs6003 / SNP Chromosome Position: 197031021 / SNP in Genomic Sequence: SEQ ID NO: 471 / SNP Position Genomic: 42518797 / SNP Source: Applera / Population(Allele,Count): Caucasian (G,2|A,32) African American (G,13|A,23) total (G,15|A,55) / SNP Type: MISSENSE MUTATION / SNP Source: Applera / Population(Allele,Count): Caucasian (G,5|A,35) African American (G,14|A,18) total (G,19|A,53) / SNP Type: MISSENSE MUTATION / SNP Source: dbSNP; Celera; HapMap; HGBASE / Population(Allele,Count): Caucasian (G,20|A,206) / SNP Type: MISSENSE MUTATION /
Context (SEQ ID NO: 2837): /
GTCTTCTAGTTCTCAGATGTCTCCCCTTTCCTTATATGGTGAAAACTCTAATAGTCTCTCTTCAGCGGAGCCACTGAAGGAAG
ATAAGCCTGTCACTGGT
Y
CTAGGAACAAGACTGGTATTACTCTATCTCCTAACTTCTGTTATTTCTATTTCAGATTGAGCTTAGGTTCCATTTCTTAAAAG
AAAATGCTGGATTAAAT / Celera SNP ID: hCV25631989 / Public SNP ID: rs1135983 /
SNP Chromosome Position: 161761312 / SNP in Genomic Sequence: SEQ ID NO: 471 / SNP Position Genomic: 77788506 / SNP Source: Applera / Population(Allele,Count): Caucasian (C,35|T,5) African American (C,28|T,4) total (C,63|T,9) / SNP Type: MISSENSE MUTATION;ESS;TRANSCRIPTION FACTOR BINDING SITE / SNP Source: dbSNP; Applera / Population(Allele,Count): Caucasian (C,111|T,5) / SNP Type: MISSENSE MUTATION;ESS;TRANSCRIPTION FACTOR BINDING SITE /
Context (SEQ ID NO: 2838): /
CTGACTCCAGCTCTTTCCTTCTTGGGATGCTGCTGCCTCTTCCTCTTACCTCTGCTGCTTGAGCCAGTGTGTGCTCTGCTCTC
TGCTTCCCTAGCCCCGC
Y
ATCCTTCTCATCCCTTACATCCAAGATGTCCTCCTCCTCTTGATGCTGCTGTCTTTTCTTCTTGCTTTTCCTGCTTCTTCCAT
CACTGCATGCTCTGCTC / Celera SNP ID: hCV9102827 / Public SNP ID: rs3795733 /
SNP Chromosome Position: 156564922 / SNP in Genomic Sequence: SEQ ID NO: 471 / SNP Position Genomic: 82984896 / SNP Source: Applera / Population(Allele,Count): Caucasian (C,10|T,30) African American (C,26|T,10) total (C,36|T,40) // / SNP Type: MICRORNA;UTR3 / SNP Source: dbSNP; Celera; HapMap; HGBASE / Population(Allele,Count): Caucasian (C,61|T,165) / SNP Type: MICRORNA;UTR3 /
Context (SEQ ID NO: 2839): /

TGTAACACAATCTGTAAAAGCAAGACATGTGGTCTTGAAAAAAGATGAGCATATCCAAGGCTGGCTGTCAAAATGCAGTTTGA
GTATAAATTGTTTGACT
M

TTTGAAAAAGCCTGGAGATCTCACTTAAACCAACAAATAATTATGCTTTATGGAGTGCGCAAAACCAAATCAGAATTCTGAAA
TTTGCTGTCAATTCTCT / Celera SNP ID:  hCV1948599 / Public SNP ID:  rs504527 /
SNP Chromosome Position:  34473608 / SNP in Genomic Sequence:  SEQ ID NO: 471 /
SNP Position Genomic:  205076210 / SNP Source:  dbSNP; Celera; HapMap; ABI_Val;
HGBASE / Population(Allele,Count):  Caucasian (A,93|C,133) / SNP Type:   INTRON
/
Context          (SEQ ID NO: 2840): /
TGGTTTTCCTGATTTTGCTCCAATAGGCACTTTTTTTAGGGGGGGATTATGCTTTTGATCAAAAGCTTGGTGCTGAATCTCTC
TTTTCTCCAGAGAGATT
Y

AGTCTCACCCTGCTTTCCCAACACATGGCGTTTGTGTAGAGGGGAGGACCAAAGGAACATGTGTGGTTTGGAAGCAGTGTTCT
GTTCTGTTCTTCTTGTA / Celera SNP ID:  hCV8911768 / Public SNP ID:  rs941988 /
SNP Chromosome Position:  173881893 / SNP in Genomic Sequence:  SEQ ID NO: 471 /
 SNP Position Genomic:  65667925 / SNP Source:  dbSNP; HapMap; HGBASE /
Population(Allele,Count):  Caucasian (C,203|T,23) / SNP Type:   INTRON /
Context          (SEQ ID NO: 2841): /
CCCAACTTTATGTGCTAGTAATTTCATCCAGGAGAACCTGTGCTTTGCTGCTTGATCTCTTTCTACCTTGGGTCCCTTATGCT
TAGCATGTTCTTGACTT
Y

TGAATTCTCCAGCACCAAGTGAAAGTAGACGTATCCCTGTGACATCTGGCTGTAGAGGATCCTCTATAGGGTCTTCAGAATAT
GGGCTGGAATGCTCTGC / Celera SNP ID:  hCV8919444 / Public SNP ID:  rs4524 / SNP
Chromosome Position:  169038063 / SNP in Genomic Sequence:  SEQ ID NO: 471 /
SNP Position Genomic:  70038063 / SNP Source:  dbSNP; HapMap /
Population(Allele,Count):  Caucasian (T,167|C,59) / SNP Type:   MISSENSE
MUTATION;ESE /
Context          (SEQ ID NO: 2842): /
ATTTTTGGTGGGCAGCAACTGGCTGGAGTAAAGGGCAGCTGTCCCTTACTGAGAGCACCCATTCTCCAGTTCACACAGTCCCC
AGAGGTCTACTATCTGA
Y

TCACGGAGCGGACTTCAGATACGTCATCCACCTGGCCCTTGGTAGGCATTTGTGTTTGTGACTACAGCTGAAGTCAATCTAAG
TCATGAACTGCTACATG / Celera SNP ID:  hCV11633415 / Public SNP ID:  rs4262503 /
SNP Chromosome Position:  182252309 / SNP in Genomic Sequence:  SEQ ID NO: 471 /
 SNP Position Genomic:  57297509 / SNP Source:  dbSNP; Celera; HapMap; HGBASE /
Population(Allele,Count):  Caucasian (T,207|C,19) / SNP Type:   INTRON /
Context          (SEQ ID NO: 2843): /
AATGCCCCATTATTTAGCCAGGAGACCTAACATGTTCTAGCCAGAAGAAATTCTCAGAATTTCTGAAAGGTTACTTCAAGGAC
AAAATACCTGTATTCCT
Y

GCCTGTCCAGGGATCTGCTCTTACAGATTAGAAGTAGTCCTATTAGCCCAGAGGCGATGTCTCTCATGATGTCCACGTCACTG
TAGTATGGTCTTGTTAA / Celera SNP ID:  hCV11975250 / Public SNP ID:  rs6025 / SNP
Chromosome Position:  169519049 / SNP in Genomic Sequence:  SEQ ID NO: 471 /
SNP Position Genomic:  70030769 / SNP Source:  HGMD; dbSNP; HapMap; ABI_Val /
Population(Allele,Count):  Caucasian (T,5|C,221) / SNP Type:   MISSENSE
MUTATION;ESE SYNONYMOUS /
Context          (SEQ ID NO: 2844): /
AAGTACATATTATTACCTTTGCAGGTTGGTGGAGTAGCTGACCATCTTCCAGAAGTTGTGCATTCCACATTATTGGGCCCCTC
CAGCTTAAAGCCGTTGT
Y

ACAAGAGAAATGGCAGGTGGAGCCAACATTGAATTCTCCACGAGTGTCAGAACAATCCAGGCTGCCCTGCTCTGGGGCAAAGA
GTTCTGGGCACTTGATG / Celera SNP ID:  hCV11975285 / Public SNP ID:  rs6127 / SNP
Chromosome Position:  169566313 / SNP in Genomic Sequence:  SEQ ID NO: 471 /
SNP Position Genomic:  69983505 / Related Interrogated SNP:  hCV11975250 / SNP
Source:  Applera / Population(Allele,Count):  Caucasian (C,19|T,21) African
American (C,9|T,29) total (C,28|T,50) / SNP Type:   MISSENSE MUTATION;INTRON /
SNP Source:  dbSNP; HapMap / Population(Allele,Count):  Caucasian (C,109|T,117)
/ SNP Type:   MISSENSE MUTATION;INTRON /
Context          (SEQ ID NO: 2845): /
GTCCAAGCCCCTCACTCTGTAGCCGGGCTGGCACTCAAATTTACAGCTGGAGCCATAGGCAAAAGCAGTGAGCGGATGAACAC
AGTCCATGGTTCCTTCA
Y

TGGGGGCTTCCAGGTGCTGACACTGCACAGCTGGAGAGAATAACCAAGGATAAAGAGAAAGATACTGAGAAAGGAGAAAAGCC
ACACAGAGAGCAATGGT / Celera SNP ID: hCV11975296 / Public SNP ID: rs6131 / SNP
Chromosome Position: 169580885 / SNP in Genomic Sequence: SEQ ID NO: 471 /
SNP Position Genomic: 69968933 / Related Interrogated SNP: hCV11975250 / SNP
Source: Applera / Population(Allele,Count): Caucasian (C,8|T,2) African
American (C,9|T,7) total (C,17|T,9) / SNP Type: MISSENSE
MUTATION;ESE;TRANSCRIPTION FACTOR BINDING SITE;ESE SYNONYMOUS / ;INTRON // SNP
Source: dbSNP; HapMap; HGBASE / Population(Allele,Count): Caucasian
(C,176|T,50) / SNP Type: MISSENSE MUTATION;ESE;TRANSCRIPTION FACTOR BINDING
SITE;ESE SYNONYMOUS / ;INTRON //
Context (SEQ ID NO: 2846): /
CACAAGAGGACTTGTAGGTGAATTCTCCAATAGGGGAATGAGCACACCTCACCAAACCCTTCGGGGGCTGGTGGACAGCATCG
CATCTCACAGCTGGAAC
R
CACGAGAGAGCACTTTAGAAGTTTGTTTGCATCTCCAGCAATACGTTTCCCAAGGTAACCAAGTTCCCAAGCTCTTCAATAGT
TCTTTTTATCTTAAAAT / Celera SNP ID: hCV11975325 / Public SNP ID: rs5367 / SNP
Chromosome Position: 169697076 / SNP in Genomic Sequence: SEQ ID NO: 471 /
SNP Position Genomic: 69852742 / Related Interrogated SNP: hCV11975250 / SNP
Source: Applera / Population(Allele,Count): Caucasian (A,37|G,3) African
American (A,32|G,2) total (A,69|G,5) / SNP Type: ACCEPTOR SPLICE
SITE;INTRON;PSEUDOGENE / SNP Source: dbSNP; HapMap; ABI_Val; HGBASE /
Population(Allele,Count): Caucasian (A,199|G,27) / SNP Type: ACCEPTOR SPLICE
SITE;INTRON;PSEUDOGENE /
Context (SEQ ID NO: 2847): /
CTCCCATTAGTTCAAATCCTTCTTCACAGTCAAATGTACAGGTTGTGTTCCATGGGAAGCTTCCAGGGTTTTGGAAACATTCC
ACGAACCCATTGGCTGG
R
TTTGTCACAGCATCACACTCAACCACTGAGGATTTTAAAGAGCACCATGAATTTTACAGAAGAATGATCTTTTCACTTCCTAT
TGAGCTGGGTGCCTAAC / Celera SNP ID: hCV11975329 / Public SNP ID: rs5363 / SNP
Chromosome Position: 169698789 / SNP in Genomic Sequence: SEQ ID NO: 471 /
SNP Position Genomic: 69851029 / Related Interrogated SNP: hCV11975250 / SNP
Source: Applera / Population(Allele,Count): Caucasian (A,34|G,0) African
American (A,34|G,2) total (A,68|G,2) / SNP Type: ESE;SILENT MUTATION;INTRON /
SNP Source: dbSNP; HapMap; HGBASE / Population(Allele,Count): Caucasian
(A,199|G,27) / SNP Type: ESE;SILENT MUTATION;INTRON /
Context (SEQ ID NO: 2848): /
TATGACACCATCTGCACCAGGGAAGGGAAGGCATGCAGACCTGACTCTAATGCCAGCTAGGACGTGAGATGGTGCTACCATCT
CAAGTGAAGAAAGAGGC
R
AGAACCAGACTTACTTTGCTCACACTTGAGTCCACTGAAGCCAGGGTCACACTTGCAAGTGTAATTATTGATGGTCTCTACAC
ATTCACCGTGGCCACTG / Celera SNP ID: hCV11975331 / Public SNP ID: rs5362 / SNP
Chromosome Position: 169700961 / SNP in Genomic Sequence: SEQ ID NO: 471 /
SNP Position Genomic: 69848857 / Related Interrogated SNP: hCV11975250 / SNP
Source: Applera / Population(Allele,Count): Caucasian (A,20|G,4) African
American (A,11|G,5) total (A,31|G,9) / SNP Type: MISSENSE MUTATION;ESS;INTRON
/ SNP Source: dbSNP; HapMap; HGBASE / Population(Allele,Count): Caucasian
(A,199|G,27) / SNP Type: MISSENSE MUTATION;ESS;INTRON /
Context (SEQ ID NO: 2849): /
CAAGAACCAGACTTACTTTGCTCACACTTGAGTCCACTGAAGCCAGGGTCACACTTGCAAGTGTAATTATTGATGGTCTCTAC
ACATTCACCGTGGCCAC
K
GCAGGATGTATTGGTACAGGCAGCTACGGAAAATACAAAGCATGATGAGGAGGACTATTACTGTGCTTATACTGAGTGCCTTT
GATTTTAGAATCAACAG / Celera SNP ID: hCV11975332 / Public SNP ID: rs5361 / SNP
Chromosome Position: 169701060 / SNP in Genomic Sequence: SEQ ID NO: 471 /
SNP Position Genomic: 69848758 / Related Interrogated SNP: hCV11975250 / SNP
Source: Applera / Population(Allele,Count): Caucasian (G,2|T,34) African
American (G,0|T,32) total (G,2|T,66) / SNP Type: MISSENSE MUTATION;INTRON /
SNP Source: dbSNP; HapMap; HGBASE / Population(Allele,Count): Caucasian
(T,199|G,27) / SNP Type: MISSENSE MUTATION;INTRON /
Context (SEQ ID NO: 2850): /
CTCCTTATGTTTCTGAGTAATTGTATAAACATAGTTGTCTTCTCTTTTATATTGCAGATATGGGACTGCTGAACTGTGTGCAC
ACCATGTCACCATAGTG
K

CTCATCTGGTGCGTAGAATTACAAGGCCTAAAACACCCACTTTCTATGCACTTCCTTTGGGGATATGATAATCTTTAAGAATG
TGAATATCGGCGGGAGA / Celera SNP ID: hCV16177404 / Public SNP ID: rs2272920 /
SNP Chromosome Position: 169799880 / SNP in Genomic Sequence: SEQ ID NO: 471 /
SNP Position Genomic: 69749938 / Related Interrogated SNP: hCV11975250 / SNP
Source: Applera / Population(Allele,Count): Caucasian (G,35|T,1) African
American (G,38|T,0) total (G,73|T,1) / SNP Type: MISSENSE MUTATION;ESS
SYNONYMOUS / SNP Source: dbSNP; HapMap; ABI_Val; HGBASE /
Population(Allele,Count): Caucasian (G,198|T,28) / SNP Type: MISSENSE
MUTATION;ESS SYNONYMOUS /
Context (SEQ ID NO: 2851): /
GGAATGCATCTGAGACATAGAGGTCATCTCGGCCTTCTGCAACAATACCTGGAAGGAAGACCGGAGAAGTCTTTGTGAGATGG
GAGAAAGTTGGCTTCAA
Y

CCACAGATGGGAATTCAGTTTGGATTAAGAGATCCTTTGGGGCTTTTTGAAACATAAGAAGAAATCCTAAATCTTCACCTAAT
CATACTCTCACAATGAA / Celera SNP ID: hCV16290208 / Public SNP ID: rs2759328 /
SNP Chromosome Position: 173876705 / SNP in Genomic Sequence: SEQ ID NO: 471 /
SNP Position Genomic: 65673113 / Related Interrogated SNP: hCV16180170 /
Related Interrogated SNP: hCV8911768 / SNP Source: Applera /
Population(Allele,Count): Caucasian (C,21|T,1) African American (C,25|T,1)
total (C,46|T,2) / SNP Type: TFBS SYNONYMOUS;INTRON / SNP Source: dbSNP;
HGBASE / Population(Allele,Count): Caucasian (C,203|T,23) / SNP Type: TFBS
SYNONYMOUS;INTRON /
Context (SEQ ID NO: 2852): /
TTTCTCCACTGAAAAGCACATCTTGTTTATGGTAAGTGCTCAATAAATATTTGTTAAATGAATGAATGAACAGTGACACAAAT
TTATGGACTATATTTAA
Y

TGCTTACCTTGAAACTATTCTGGTCCTTACAGGTGTTTCTGGGATAAAAGGAATGCTTATAGAAGAATTCTTCTTTTGCTTGT
AAAGTATTCTTTCAGTA / Celera SNP ID: hCV201029 / Public SNP ID: rs10754213 /
SNP Chromosome Position: 197055925 / SNP in Genomic Sequence: SEQ ID NO: 471 /
SNP Position Genomic: 42493893 / Related Interrogated SNP: hCV2532034 / SNP
Source: Applera / Population(Allele,Count): Caucasian (C,24|T,4) African
American (C,12|T,4) total (C,36|T,8) / SNP Type: DONOR SPLICE SITE;INTRON /
SNP Source: dbSNP; Celera; HapMap / Population(Allele,Count): Caucasian
(T,19|C,207) / SNP Type: DONOR SPLICE SITE;INTRON /
Context (SEQ ID NO: 2853): /
AGGATGCTGCCAGATATCCTGTGCAGGACAGCCCCAGACAAGCAAGGATATTTCAGTCTGAAATATCTATAGTGCGAGAATGA
GAAATCTTGGTCTAATG
R

CACTGACTTACCCAAAGTGAGAGCTGAGAGAAACTGTGAAGCAATCATGACTTCAAGAGTTCTTTTCACCCAAAGGTTTAGGC
TTGAAATACTTTCCTGG / Celera SNP ID: hCV2459459 / Public SNP ID: rs932307 /
SNP Chromosome Position: 169702705 / SNP in Genomic Sequence: SEQ ID NO: 471 /
SNP Position Genomic: 69847113 / Related Interrogated SNP: hCV11975250 / SNP
Source: Applera / Population(Allele,Count): Caucasian (A,4|G,10) African
American (A,5|G,17) total (A,9|G,27) / SNP Type: INTRON;PSEUDOGENE / SNP
Source: dbSNP; Celera; HapMap; ABI_Val; HGBASE / Population(Allele,Count):
Caucasian (G,175|A,51) / SNP Type: INTRON;PSEUDOGENE /
Context (SEQ ID NO: 2854): /
CGATAATGGGTTTTGTCCTTTTGTTTGTTTTAGAAATTGGAGTTCTGAATATTGATAAATCTAAAATAAATTAGAAAACAAAA
CTAAGAGCATTAAAATA
Y

AAATTCCCTAAATAGGTCAGTGATTTGAAAGTTAAAGTTTGGGTTCTTCACAATCATATTCATTTAGCAAACACTTAATAACC
ACTGTACTCCACGCACT / Celera SNP ID: hCV247773 / Public SNP ID: rs1332663 /
SNP Chromosome Position: 197109042 / SNP in Genomic Sequence: SEQ ID NO: 471 /
SNP Position Genomic: 42440776 / Related Interrogated SNP: hCV2532034 / SNP
Source: Applera / Population(Allele,Count): Caucasian (C,1|T,21) African
American (C,12|T,22) total (C,13|T,43) / SNP Type: INTRON / SNP Source:
dbSNP; Celera; HapMap; HGBASE / Population(Allele,Count): Caucasian (C,20|T,206)
/ SNP Type: INTRON /
Context (SEQ ID NO: 2855): /
CTGTGACTGTGGTTGTATCTACAAACAAAACACTGTGAAAAAAGGTGACATATCCTGTAACTCATAGGAGAAAAACTCATCAC
ATCAGTCAGTTTGCTAA
Y

CCCTTGTGAGCTTCCTTACCTGCCCAGTCCAGGAATTCAACACACTCAGTCTGAGAATATCTAGCAGCAACATCTCGAGCCCT

TTCTTCCCGGAAGTTCA / Celera SNP ID: hCV25600635 / Public SNP ID: rs7539322 /
SNP Chromosome Position: 173615965 / SNP in Genomic Sequence: SEQ ID NO: 471 /
SNP Position Genomic: 65933853 / Related Interrogated SNP: hCV16180170 /
Related Interrogated SNP: hCV8911768 / SNP Source: Applera /
Population(Allele,Count): Caucasian (C,36|T,4) African American (C,34|T,2)
total (C,70|T,6) / SNP Type: INTRON / SNP Source: dbSNP; HapMap /
Population(Allele,Count): Caucasian (C,207|T,19) / SNP Type: INTRON /
Context (SEQ ID NO: 2856): /
TCACACTTGCAAGTGTAATTATTGATGGTCTCTACACATTCACCGTGGCCACTGCAGGATGTATTGGTACAGGCAGCTACGGA
AAATACAAAGCATGATG
R

GGAGGACTATTACTGTGCTTATACTGAGTGCCTTTGATTTTAGAATCAACAGTGTGCAACAGAGACATCAGCAGTCCTACAGA
GTGCCATAGACTTTAAC / Celera SNP ID: hCV25617131 / Public SNP ID: rs3917410 /
SNP Chromosome Position: 169701108 / SNP in Genomic Sequence: SEQ ID NO: 471 /
SNP Position Genomic: 69848710 / Related Interrogated SNP: hCV11975250 / SNP
Source: Applera / Population(Allele,Count): Caucasian (A,38|G,2) African
American (A,36|G,2) total (A,74|G,4) / SNP Type: INTRON / SNP Source:
dbSNP; HapMap; HGBASE / Population(Allele,Count): Caucasian (A,189|G,27) / SNP
Type: INTRON /
Context (SEQ ID NO: 2857): /
GAACATTTTACCACTGCAAATGTTAGGTACACAGGCAGAGTTTCAGAAAAATCTACTGGAAAACTTCCAAAACTTGCTTAAAA
GTCAACAATGAATGTAA
W

GTGTAAGCGCTACTTAGTTTTCAGCATGTAGGAAATTAGGACCAAACCCCTTTGGGGCAATCTAGGTTCAGAAACTTTATGAA
GTATTTGACCTGTACCC / Celera SNP ID: hCV25617143 / Public SNP ID: rs3917425 /
SNP Chromosome Position: 169696754 / SNP in Genomic Sequence: SEQ ID NO: 471 /
SNP Position Genomic: 69853064 / Related Interrogated SNP: hCV11975250 / SNP
Source: Applera / Population(Allele,Count): Caucasian (A,29|T,1) African
American (A,34|T,2) total (A,63|T,3) / SNP Type: INTRON;PSEUDOGENE / SNP
Source: dbSNP; HapMap; HGBASE / Population(Allele,Count): Caucasian
(A,199|T,27) / SNP Type: INTRON;PSEUDOGENE /
Context (SEQ ID NO: 2858): /
TTCAGTTTCTTCATCTATATAAGAGGAATAATGAAATTGTGTTATCTTTATCAAATTGATATGGAAACTAAATGTAATTCAAT
TAGCATAAGTCAAGGAC
S

TTAGAACAAAGCCTGACTCATCAGAAATTCTAAGTAAACATTAGCTAGTCTTCATATTATTATCTTCAGCATTATCTGTAGTG
AGAATCCTTAAAGCCAA / Celera SNP ID: hCV25619707 / Public SNP ID: rs4987308 /
SNP Chromosome Position: 169674116 / SNP in Genomic Sequence: SEQ ID NO: 471 /
SNP Position Genomic: 69875702 / Related Interrogated SNP: hCV11975250 / SNP
Source: Applera / Population(Allele,Count): Caucasian (C,38|G,2) African
American (C,36|G,2) total (C,74|G,4) / SNP Type: TRANSCRIPTION FACTOR BINDING
SITE;INTRON / SNP Source: dbSNP; HapMap / Population(Allele,Count): Caucasian
(C,109|G,11) / SNP Type: TRANSCRIPTION FACTOR BINDING SITE;INTRON /
Context (SEQ ID NO: 2859): /
TAAGCGATATTTCTTCAATATTTCCCTTCTTGGACTTCACATTGATGATAGTTTTTAGGTTGTAATAATTTAGAGTTTGGATG
GTCTACATTTACCATCT
R

CAGTTGTTTACTCCCTCTTTTTTTTAATGCACAAGTGATGAAAATGTTGTCATGTAATATCTAAACAATGATAAATACTTAGAC
TATATATAAAAATATAT / Celera SNP ID: hCV25932979 / Public SNP ID: rs16846809 /
SNP Chromosome Position: 174219398 / SNP in Genomic Sequence: SEQ ID NO: 471 /
SNP Position Genomic: 65330420 / Related Interrogated SNP: hCV16180170 /
Related Interrogated SNP: hCV8911768 / SNP Source: Applera /
Population(Allele,Count): Caucasian (A,39|G,1) African American (A,33|G,3)
total (A,72|G,4) / SNP Type: INTRON / SNP Source: dbSNP; HapMap /
Population(Allele,Count): Caucasian (A,209|G,17) / SNP Type: INTRON /
Context (SEQ ID NO: 2860): /
GGGGCTGCGGCAGGATGTATGCTGAAGAGTGGGCGGCATCGGGTGGGGGAAGTTGTGCAGGTGCTGGAGAAAAGCCGGAGGCT
TGCCGAGAGGAGCACTG
R

GAGGCGACTGCTGGGTGAGACCTTCGGGCAGGGAAGGCATGGGATCTGGGGAGACACAGAGCCTGATGATACTCAGAGGGCTG
GGTTTGGGGAACCCTGG / Celera SNP ID: hCV25989540 / Public SNP ID: rs6682716 /
SNP Chromosome Position: 156551848 / SNP in Genomic Sequence: SEQ ID NO: 471 /
SNP Position Genomic: 82997970 / Related Interrogated SNP: hCV9102827 / SNP

Source: Applera / Population(Allele,Count): Caucasian (A,10|G,30) African American (A,24|G,4) total (A,34|G,34) / SNP Type: MISSENSE MUTATION;ESS;ESE SYNONYMOUS / SNP Source: Applera / Population(Allele,Count): Caucasian (A,11|G,27) African American (A,14|G,2) total (A,25|G,29) / SNP Type: MISSENSE MUTATION;ESS;ESE SYNONYMOUS / SNP Source: dbSNP; HapMap / Population(Allele,Count): Caucasian (A,56|G,170) / SNP Type: MISSENSE MUTATION;ESS;ESE SYNONYMOUS /
Context (SEQ ID NO: 2861): /
CTTTGTGTTTTTCCCTTAAAAGTTGTCTTGCTTTCATTCCTTTATATGCAGCCTGAATAACCACAGCAGAATGCCATTGTCTG
ATATAATTTTCTCTTTG
Y

AATTTTGCAGCTCTATATGTTCGATAATGTTGCTGAATTAGAATTGAAGCATGTTTCCAAGTCTGAAATGTAATATATGTTCT
GTACATCCTGAAAGTAG / Celera SNP ID: hCV2759703 / Public SNP ID: rs1412640 /
SNP Chromosome Position: 197070815 / SNP in Genomic Sequence: SEQ ID NO: 471 /
SNP Position Genomic: 42479003 / Related Interrogated SNP: hCV2532034 / SNP
Source: Applera / Population(Allele,Count): Caucasian (C,35|T,3) African
American (C,25|T,11) total (C,60|T,14) / SNP Type: SILENT MUTATION;INTRON /
SNP Source: dbSNP; Celera; ABI_Val; HGBASE / Population(Allele,Count):
Caucasian (T,20|C,206) / SNP Type: SILENT MUTATION;INTRON /
Context (SEQ ID NO: 2862): /
AAGGTTTTTTGTTTTTGTTTTTTTTTAGACAGGGTCTTATTCTGTTGCCCAGGCTTGAGTCCAGTGGCACAGTCATGGCTCAC
TGCAGCCTTAAACTCCT
Y

TCAAGTAATCCTCTTGCCTCAGCCTTCCAAAGTGCTGGCATTGCAGACATGAGCCATCATGCCCAACCCAGAAAATTTTTTAT
CCTATTAGCTCAAAATG / Celera SNP ID: hCV341935 / Public SNP ID: rs4656685 /
SNP Chromosome Position: 169483844 / SNP in Genomic Sequence: SEQ ID NO: 471 /
SNP Position Genomic: 70065974 / Related Interrogated SNP: hCV8919444 / SNP
Source: dbSNP; HapMap / Population(Allele,Count): Caucasian (C,166|T,60) /
SNP Type: INTRON /
Context (SEQ ID NO: 2863): /
GAGGATGTGAGTCTCCAAGAACTTCGGGACTTCTTTTCATAGTACCAGCTCTTCTTTTCATCAAAGGTCATAAATAGTAAGAC
AAATTCTCTCATGTCCA
Y

AGGCATGTTGCTGTCCTTATGTAGTATTCCTTTTTGGCAGATTAGGAGGGGACCTATCAAGCCTGAGTGAATATCTTTTTCCT
GGAAAAACAGAGTAAAT / Celera SNP ID: hCV342590 / Public SNP ID: rs6030 / SNP
Chromosome Position: 169498975 / SNP in Genomic Sequence: SEQ ID NO: 471 /
SNP Position Genomic: 70050843 / Related Interrogated SNP: hCV8919444 / SNP
Source: Applera / Population(Allele,Count): Caucasian (C,8|T,30) African
American (C,5|T,29) total (C,13|T,59) / SNP Type: MISSENSE MUTATION;ESE / SNP
Source: dbSNP; Celera; HapMap; ABI_Val / Population(Allele,Count): Caucasian
(T,158|C,66) / SNP Type: MISSENSE MUTATION;ESE /
Context (SEQ ID NO: 2864): /
AGTGTCTTGGCTTAGGTGTCTCCCAACTTTATGTGCTAGTAATTTCATCCAGGAGAACCTGTGCTTTGCTGCTTGATCTCTTT
CTACCTTGGGTCCCTTA
Y

GCTTAGCATGTTCTTGACTTTTGAATTCTCCAGCACCAAGTGAAAGTAGACGTATCCCTGTGACATCTGGCTGTAGAGGATCC
TCTATAGGGTCTTCAGA / Celera SNP ID: hCV8919442 / Public SNP ID: rs4525 / SNP
Chromosome Position: 169511734 / SNP in Genomic Sequence: SEQ ID NO: 471 /
SNP Position Genomic: 70038084 / Related Interrogated SNP: hCV8919444 / SNP
Source: Applera / Population(Allele,Count): Caucasian (C,3|T,25) African
American (C,1|T,19) total (C,4|T,44) / SNP Type: MISSENSE MUTATION / SNP
Source: dbSNP; HapMap; HGBASE / Population(Allele,Count): Caucasian
(T,167|C,59) / SNP Type: MISSENSE MUTATION /
Context (SEQ ID NO: 2865): /
GGTGAGAAGGGGCTTTCTGAGGTTCTGCAAGGTTATTGACAGTGAACTTACTAATATTACTTGGGGAAGAATAATTTGAACCA
ACAATTATATCTGTGTT
Y

GAAGAAACGAATTCAGTGCCATTCTCCAGAGCTAGGGCAGTAAGATTGAACTCTTCTTCTTCCTGATTCAATGATGAGTTTCG
GAATGACCTGATTCCTA / Celera SNP ID: hCV8919446 / Public SNP ID: rs6021 / SNP
Chromosome Position: 169512027 / SNP in Genomic Sequence: SEQ ID NO: 471 /
SNP Position Genomic: 70037791 / Related Interrogated SNP: hCV8919444 / SNP
Source: Applera / Population(Allele,Count): Caucasian (C,8|T,30) African
American (C,4|T,26) total (C,12|T,56) / SNP Type: SILENT RARE CODON;SILENT

MUTATION / SNP Source: dbSNP; Applera / Population(Allele,Count): Caucasian (T,85|C,29) / SNP Type: SILENT RARE CODON;SILENT MUTATION / Context (SEQ ID NO: 2866): / AAGAATAATTTGAACCAACAATTATATCTGTGTTTGAAGAAACGAATTCAGTGCCATTCTCCAGAGCTAGGGCAGTAAGATTG AACTCTTCTTCTTCCTG
R
TTCAATGATGAGTTTCGGAATGACCTGATTCCTAATGCTGCAGCCAGTCTGTTCTGGTAATCATAGTCAGCATCACTCTCTTC ATCTTCAGGTTCTAAAC / Celera SNP ID: hCV8919450 / Public SNP ID: rs6017 / SNP Chromosome Position: 169512093 / SNP in Genomic Sequence: SEQ ID NO: 471 / SNP Position Genomic: 70037725 / Related Interrogated SNP: hCV8919444 / SNP Source: Applera / Population(Allele,Count): Caucasian (A,32|G,8) African American (A,29|G,5) total (A,61|G,13) / SNP Type: ESE;SILENT MUTATION / SNP Source: dbSNP; Applera / Population(Allele,Count): Caucasian (A,167|G,59) / SNP Type: ESE;SILENT MUTATION / Context (SEQ ID NO: 2867): / CTGTGTTTGAAGAAACGAATTCAGTGCCATTCTCCAGAGCTAGGGCAGTAAGATTGAACTCTTCTTCTTCCTGATTCAATGAT GAGTTTCGGAATGACCT
R
ATTCCTAATGCTGCAGCCAGTCTGTTCTGGTAATCATAGTCAGCATCACTCTCTTCATCTTCAGGTTCTAAACGATCATGCAT TTTCCGTGTAGCCATGA / Celera SNP ID: hCV8919451 / Public SNP ID: rs6016 / SNP Chromosome Position: 169512120 / SNP in Genomic Sequence: SEQ ID NO: 471 / SNP Position Genomic: 70037698 / Related Interrogated SNP: hCV8919444 / SNP Source: Applera / Population(Allele,Count): Caucasian (A,8|G,32) African American (A,5|G,27) total (A,13|G,59) / SNP Type: ESS;SILENT MUTATION / SNP Source: dbSNP; HGBASE / Population(Allele,Count): Caucasian (G,165|A,61) / SNP Type: ESS;SILENT MUTATION / Context (SEQ ID NO: 2868): / TGCCTGCATCTTTGTTTCTCTTGATATAGATCTCCACGCAGTCCTCCTTGTTCTTCTTGTTGTTGGGCTCACCATCTCCCCAG TTCTCTGCTTCTTCAGT
R
AGAGATTTGTTGGTTCCCACCCACGTCCATATTCCTCCTATCTTCCGGATTCCTATCCAGTAGTAAGAACGACTGAAAGGCAG AGTCTTCTCCAGATACT / Celera SNP ID: hCV8919509 / Public SNP ID: rs1051091 / SNP Chromosome Position: 169677709 / SNP in Genomic Sequence: SEQ ID NO: 471 / SNP Position Genomic: 69872109 / Related Interrogated SNP: hCV11975250 / SNP Source: Applera / Population(Allele,Count): Caucasian (A,27|G,11) African American (A,29|G,9) total (A,56|G,20) / SNP Type: ESE SYNONYMOUS;SILENT MUTATION;INTRON / SNP Source: dbSNP; HapMap; HGBASE / Population(Allele,Count): Caucasian (A,176|G,50) / SNP Type: ESE SYNONYMOUS;SILENT MUTATION;INTRON / Context (SEQ ID NO: 2869): / TGGGGAGCTGCCTATCTTCAGGCAATAGATGGGTAGGTAATAGGAATCATTTCCCTGGGGTAAAAAGAACCAAGAGGTGGCAC AGGCCATGGTCAAAGGT
S
GGGAGAAAAGGGAGCTCAAGGAGCTGACCACTACTTACTTGTGTGCTGTTCGCCCCTCACAGGCTTGGAGCAGCATCTCATCA GTCAGACTGTTAGGGGA / Celera SNP ID: hCV9102823 / Public SNP ID: rs12024215 / SNP Chromosome Position: 156565834 / SNP in Genomic Sequence: SEQ ID NO: 471 / SNP Position Genomic: 82983984 / Related Interrogated SNP: hCV9102827 / SNP Source: Applera / Population(Allele,Count): Caucasian (C,9|G,29) African American (C,6|G,30) total (C,15|G,59) / SNP Type: UTR3;INTRON / SNP Source: dbSNP; Celera; HapMap / Population(Allele,Count): Caucasian (G,92|C,28) / SNP Type: UTR3;INTRON / Context (SEQ ID NO: 2870): / AGCTTCCCCTTAGTACATTGAAATTCAAAGTCATGCTTGTAACTGTTAATGAAAGCAGATTTAAAGCAACACCACCATCACTG GAGTATTTTTAGTTATA
Y
ACGATTGAGACTACCAAGCATGTTGCTCTTATTCAGTGTAATCCTAATCTCATGGGTATCCACTGTTGGGGGAGAAGGTAAGT TGAAAACAGATCCGAAT / Celera SNP ID: hCV9114656 / Public SNP ID: rs9427662 / SNP Chromosome Position: 196946775 / SNP in Genomic Sequence: SEQ ID NO: 471 / SNP Position Genomic: 42603043 / Related Interrogated SNP: hCV2532034 / SNP Source: Applera / Population(Allele,Count): Caucasian (C,4|T,34) African American (C,10|T,28) total (C,14|T,62) / SNP Type: MISSENSE MUTATION;UTR5 / SNP Source: dbSNP; Celera; HapMap / Population(Allele,Count): Caucasian (T,207|C,19) / SNP Type: MISSENSE MUTATION;UTR5 / Context (SEQ ID NO: 2871): /

ACATGAATTTTAACATGAAGACTGCCTGCAATTAATTTTAGCTACAATACAAGCAATTTAAAATCTGACAAAAATGTACCAGG
GTCAGCTAAAGAGTTTG
K
TAAGTTTTACTGTAGCAGAGATATTTTATAAAGACCATTAAATCTGTGCTCCCCATACGTCAACATTTAACTAGCTGTTTTGA
GGCATAGCAACTTCCTA / Celera SNP ID: hCV65774 / Public SNP ID: rs7534353 / SNP
Chromosome Position: 197096480 / SNP in Genomic Sequence: SEQ ID NO: 471 /
SNP Position Genomic: 42453338 / Related Interrogated SNP: hCV2532034 / SNP
Source: dbSNP; Celera; ABI_Val / Population(Allele,Count): Caucasian
(T,9|G,107) / SNP Type: INTRON /
Context (SEQ ID NO: 2872): /
TCGGTAAATGTTCTGAACTTTCCCCCTCAATCACTAATTACTAGTACCTCCTGGCTCAGTTTTCTTCCTCACTCCAAATCCTT
TCATCTCTGTTAGCATT
R
ATATCTGCTTTGTTGTACCACTCAACAAATTTGCTTCTCAATCCTTTATTGCCTCATCTCTGTTGCCACTTTTCTCGACTTTA
TGTTCCTATTGCCAAAG / Celera SNP ID: hCV87892 / Public SNP ID: rs2336597 / SNP
Chromosome Position: 196993339 / SNP in Genomic Sequence: SEQ ID NO: 471 /
SNP Position Genomic: 42556479 / Related Interrogated SNP: hCV2532034 / SNP
Source: dbSNP; Celera; HapMap / Population(Allele,Count): Caucasian
(A,19|G,207) / SNP Type: INTERGENIC;UNKNOWN /
Context (SEQ ID NO: 2873): /
TAGAATGAAAAATTATAAAACTTTTAGAGTACTTTGATATGACGCTAAAAGCATAATCCATAAAAGAAGAAAAGTGATAACTG
GATTCCAATAAAATGAA
M
TACTTCTGCTGAACAAAAAGCCCTGTTACAAGAATAAAAAGACAAGCTGCAGATTGGGAGAAATTGTGTGAAATCACTAACAA
AGAAGGAGGATTACCTA / Celera SNP ID: hCV240532 / Public SNP ID: rs2026429 /
SNP Chromosome Position: 197079671 / SNP in Genomic Sequence: SEQ ID NO: 471 /
SNP Position Genomic: 42470147 / Related Interrogated SNP: hCV2532034 / SNP
Source: dbSNP; Celera; HapMap; ABI_Val; HGBASE / Population(Allele,Count):
Caucasian (C,20|A,206) / SNP Type: INTRON /
Context (SEQ ID NO: 2874): /
ATTTTTCATAAGAAAATGCTAGAGAGAAATGATATGATTTAGGGTTAAACAATATGACAGTTTGTCTGGGTTGTGTTTCTATG
GTTTTGACTCAACAATT
S
CTACCAGAGGAACGAATCTCCAGAACTTTGGAAACTTACCCACAGGATGAGGGGACAGAATGACCAGTCATGGTTCCCTGTGT
ACTCACCATGTGAATCA / Celera SNP ID: hCV325211 / Public SNP ID: rs3753305 /
SNP Chromosome Position: 169554058 / SNP in Genomic Sequence: SEQ ID NO: 471 /
SNP Position Genomic: 69995760 / Related Interrogated SNP: hCV11975250 / SNP
Source: dbSNP; Celera; HapMap; ABI_Val; HGBASE / Population(Allele,Count):
Caucasian (C,112|G,114) / SNP Type: INTRON /
Context (SEQ ID NO: 2875): /
CTTTCTTCTGGTCTACCTCCCCTACTAATCCCATCTTTCCAGACTCTGAGCATAACATGCAAACTCACAGAACACAAGGGAGT
GGGTAAAGCAACTCCGA
M
TGCCATAAAAGTGGGTTGTGAGCCTTGAATGGAATACAAGATTTTGAAGGTGGTTCCATCCCTATTCACTCTGGACAGGCCCT
GCATCTCACTCCCTCGG / Celera SNP ID: hCV340605 / Public SNP ID: rs1557572 /
SNP Chromosome Position: 169508576 / SNP in Genomic Sequence: SEQ ID NO: 471 /
SNP Position Genomic: 70041242 / Related Interrogated SNP: hCV8919444 / SNP
Source: dbSNP; Celera; HapMap / Population(Allele,Count): Caucasian
(A,81|C,39) / SNP Type: INTRON /
Context (SEQ ID NO: 2876): /
GAACTCCTGGGCTCAAGCAAATCATCCACCTCGACTTCCCAAAGCGCTGGGATTACAGGGGTTTGTGAGCCATCGCACCAAGC
ATGAACAGGATATATTA
Y
ATCTTCAATACACCATGAACAGGATAAACAAGATAATATATCATTAGCAAGATACTATATCAAACCAGTCTGGGTTTATTCCA
GAAATGAGGTTTAATAT / Celera SNP ID: hCV356522 / Public SNP ID: rs10732295 /
SNP Chromosome Position: 197136645 / SNP in Genomic Sequence: SEQ ID NO: 471 /
SNP Position Genomic: 42413173 / Related Interrogated SNP: hCV2532034 / SNP
Source: dbSNP; Celera; HapMap; ABI_Val / Population(Allele,Count): Caucasian
(T,21|C,205) / SNP Type: INTRON /
Context (SEQ ID NO: 2877): /
ATGGAGAATCTCTACTAGGGCAATGCAGAAGGGAAACATGGGGTTGGAGCTTCCACACAGAGTCCCCACTGGGAGACTGCCTA
GTGGAGCTGTGAGAATA
S

GGTTACCACCCTCTAGATCCCAGAATGGTATATCCACCAACAGCTTGCATTGTGCACCAGAAAAGTCGCAGGCACTCAACACC
AGCCTGTGAAATAAGCT / Celera SNP ID: hCV406628 / Public SNP ID: rs1576879 /
SNP Chromosome Position: 196992318 / SNP in Genomic Sequence: SEQ ID NO: 471 /
SNP Position Genomic: 42557500 / Related Interrogated SNP: hCV2532034 / SNP
Source: dbSNP; Celera; HapMap; HGBASE / Population(Allele,Count): Caucasian
(C,9|G,111) / SNP Type: INTERGENIC;UNKNOWN /
Context (SEQ ID NO: 2878): /
GCCAAAATACATATAACACATACTGGTCCAGTGACTCTGCCCTTTAGGAGAATGGAAGTTCTGAAATACAAGTTTCAGGTTAG
CATTTGTACTGAGCGAT
Y

CTGGACAAAATTTGTTGTTTTGGGGTCCTTCTATATAAAAACTCTGTCTATAGTTTCTCAAAGTTGGTTGAAATTTTTAAGTC
TACATTTTATTTTGAAA / Celera SNP ID: hCV574681 / Public SNP ID: rs575147 / SNP
Chromosome Position: 169892284 / SNP in Genomic Sequence: SEQ ID NO: 471 /
SNP Position Genomic: 69657534 / Related Interrogated SNP: hCV11975250 / SNP
Source: dbSNP; HapMap; HGBASE / Population(Allele,Count): Caucasian
(T,26|C,198) / SNP Type: UTR5;INTRON /
Context (SEQ ID NO: 2879): /
AAGCACTAAGGCTAACAAGTAATCATGTATCTAAAGAACTCTCAATTATATAACTGTTGATTACATAGCTTGGATTTCAACTT
GTTCCTTCTTCATCTTT
Y

GCTTATTACCTGTCTTCAACCAGTCCTTATCTTTTTTAGCAACTCCTCTGAGGCCAAATAGCAGTAGACTTAGGTTAAAACTCA
AGTTAATCTTATGCTTG / Celera SNP ID: hCV574682 / Public SNP ID: rs590181 / SNP
Chromosome Position: 169892661 / SNP in Genomic Sequence: SEQ ID NO: 471 /
SNP Position Genomic: 69657157 / Related Interrogated SNP: hCV11975250 / SNP
Source: dbSNP; HapMap; HGBASE / Population(Allele,Count): Caucasian
(T,28|C,198) / SNP Type: UTR5;INTRON /
Context (SEQ ID NO: 2880): /
ATCCACCTGATGGTTATAACTTTCATTGTACATTAACAAATGGATTTTTCTTTTTAAAATATGTCATGCACATTGTTGATAAA
AACCTGCAGTCCCAAGT
M

AGTGAAAGACTGGCCCTTGAGCACTGATGAGTCTGAGTTTCTGAACCTGTAAATCTCTGGTCTTGAACAGTATGGTCTTTAGG
TTACTATGGTAAAGATC / Celera SNP ID: hCV574683 / Public SNP ID: rs544008 / SNP
Chromosome Position: 169893330 / SNP in Genomic Sequence: SEQ ID NO: 471 /
SNP Position Genomic: 69656488 / Related Interrogated SNP: hCV11975250 / SNP
Source: dbSNP; Celera; HapMap; HGBASE / Population(Allele,Count): Caucasian
(C,28|A,198) / SNP Type: UTR5;INTRON /
Context (SEQ ID NO: 2881): /
CATAAATTCTAAAGTGTCGTGAAAGACTGATGGTTTGATTTTACATGGAATGTGCCATCCTTTTTGAAGTCCTACATCATTTT
GTTTACTAGTTAACCAC
Y

GAAACATTATGAAGCCTTAACTAGCCTATTATCAGAGCCTGTTCACTATCAGGAAAACTCTTTGTCACTGGCTTCCGTCACAC
CTTGTATCTGATTAACA / Celera SNP ID: hCV574743 / Public SNP ID: rs545963 / SNP
Chromosome Position: 169937418 / SNP in Genomic Sequence: SEQ ID NO: 471 /
SNP Position Genomic: 69612400 / Related Interrogated SNP: hCV11975250 / SNP
Source: dbSNP; HapMap; HGBASE / Population(Allele,Count): Caucasian
(C,27|T,195) / SNP Type: INTRON /
Context (SEQ ID NO: 2882): /
TTATTTATAGGATAAATGAGGCCTATTAAAATTGAGAAACAGTGTAGTAGTCTATGAGGAGTTGTGTTCTGAAACAGATGTGT
GAAATTCCATAACAGTT
Y

GGTAATAAACAGAACAACCAAACCAATTTATTTTTTAAAAAATCTACTTTAAGTTTCTTAAAGAAACTATCATAAAGGCAACC
TAGTTTCAAAAAAATGG / Celera SNP ID: hCV574788 / Public SNP ID: rs629408 / SNP
Chromosome Position: 169970822 / SNP in Genomic Sequence: SEQ ID NO: 471 /
SNP Position Genomic: 69578996 / Related Interrogated SNP: hCV11975250 / SNP
Source: dbSNP; HapMap; ABI_Val; HGBASE / Population(Allele,Count): Caucasian
(C,27|T,197) / SNP Type: INTRON /
Context (SEQ ID NO: 2883): /
GCTGGTAAATACTATCTTATAACCCATCATTTAAAATTGATGACACTTAACACTAATTGCATAAACGAAGAAATAAGCAAAAA
GAAGACTGATAAAAAAC
K

ATACACTTTGACTTTACCCCCCTGCTATTTAACTTTTTTGTTGTTTCTTTATATCTTATTGTATGTACTGTCTATGTCTTGAAA
AGTTGTTGAAGTAATTA / Celera SNP ID: hCV1681328 / Public SNP ID: rs10912647 /
SNP Chromosome Position: 173537093 / SNP in Genomic Sequence: SEQ ID NO: 471 /

SNP Position Genomic: 66012725 / Related Interrogated SNP: hCV16180170 / Related Interrogated SNP: hCV8911768 / SNP Source: dbSNP; Celera; HapMap / Population(Allele,Count): Caucasian (G,168|T,58) / SNP Type: INTRON / Context (SEQ ID NO: 2884): /
GTTTAAGGCAGCATCCTAAGATTTTGGGCAAAGGACACTAGTGCAATAATCTTTATTTCAGAGTTTAATCAAATAAATAAACA
AATTTTAAGACTTTCAT
Y
ATTTAGGTCAAAGAGAAAAGACAGGTTTTAGCTACAATACAATAAGAGCTTGTACAGATGTGGTTTTTATTAGAAGGCCTTTT
GCATATCTGTGTTTCAT / Celera SNP ID: hCV2459460 / Public SNP ID: rs5353 / SNP Chromosome Position: 169702974 / SNP in Genomic Sequence: SEQ ID NO: 471 / SNP Position Genomic: 69846844 / Related Interrogated SNP: hCV11975250 / SNP Source: dbSNP; Celera; HapMap; HGBASE / Population(Allele,Count): Caucasian (T,165|C,51) / SNP Type: INTRON;PSEUDOGENE /
Context (SEQ ID NO: 2885): /
CCTTCCCACCTTAACTAAGGGTAGAAGCCCCTGCAATGTGCTCCCACAGCCTCTGTTCTTGCGCTGTTCATACATGATTGTCA
TTCTTCCCCATTATACC
R
TGGTCTCCTCGAAGTTGGGCTCAGTCATTCATAGTTTTATTCTGGAGCTGAGCAGAGTGTTTTGTACATTGAAAGTGCTCATT
AAATATTGACATTATCT / Celera SNP ID: hCV2480428 / Public SNP ID: rs3917740 / SNP Chromosome Position: 169579266 / SNP in Genomic Sequence: SEQ ID NO: 471 / SNP Position Genomic: 69970552 / Related Interrogated SNP: hCV11975250 / SNP Source: dbSNP; Celera; HapMap; ABI_Val; HGBASE / Population(Allele,Count): Caucasian (G,169|A,57) / SNP Type: INTRON /
Context (SEQ ID NO: 2886): /
CTTTCTAGTGGCCTAATTTAAGGATAGGCCCTGGAATTCTATAGAAAGCCAGGAAATTGGAGTGTAGGTATCTTAAGGGAGGG
AGATATGAGGATAGAAA
K
TAGGGAATTTGGATTTAGTTGGCTGAAGGGATCATCAAGTTATTCATGCCATTCTGATATCTCAGGTCATTTAAAGAATTAAT
AAAAATGAGTCAAATAT / Celera SNP ID: hCV2481728 / Public SNP ID: rs9332665 / SNP Chromosome Position: 169486767 / SNP in Genomic Sequence: SEQ ID NO: 471 / SNP Position Genomic: 70063051 / Related Interrogated SNP: hCV8919444 / SNP Source: dbSNP; Celera; HapMap / Population(Allele,Count): Caucasian (T,80|G,38) / SNP Type: INTRON /
Context (SEQ ID NO: 2887): /
TATTCACTCTGGACAGGCCCTGCATCTCACTCCCTCGGGGCCTTGCTTAGAAATACTCAGGTAGCTAGTTGTTCTCATGTGGT
ATTGAGTGCAACATTTA
W
ATAGGAAGTCATAGGAAAAGGTGTTTTAAACAGAGTTCTAATGTGGAGATGTCAGGCATCAGATTAATGAACTCATATGCATA
AGTCACACCATACATTG / Celera SNP ID: hCV2481741 / Public SNP ID: rs3766109 / SNP Chromosome Position: 169508739 / SNP in Genomic Sequence: SEQ ID NO: 471 / SNP Position Genomic: 70041079 / Related Interrogated SNP: hCV8919444 / SNP Source: dbSNP; Celera; HapMap; ABI_Val; HGBASE / Population(Allele,Count): Caucasian (A,89|T,31) / SNP Type: TRANSCRIPTION FACTOR BINDING SITE;INTRON /
Context (SEQ ID NO: 2888): /
TGAGCTCTATCCCCACAAATAAGAGACATTTAGATGTTTCTAAGTAATAATTCACGAGGACAATTTTGAATCTCTGAAATGGC
ATGCATTGCTGGAACAT
Y
AAAGATTTCTTGGTGAGTCTCCAATAGTTACCATTGTTGGTGTCTACTGAGTGCTAGGCACTTGGCGCATATTACTATTTTAA
TGGTCAAAGTAACTCTG / Celera SNP ID: hCV2481744 / Public SNP ID: rs9332600 / SNP Chromosome Position: 169512913 / SNP in Genomic Sequence: SEQ ID NO: 471 / SNP Position Genomic: 70036905 / Related Interrogated SNP: hCV8919444 / SNP Source: dbSNP; Celera / Population(Allele,Count): Caucasian (C,167|T,59) / SNP Type: INTRON /
Context (SEQ ID NO: 2889): /
TTATAGTCAGATTGCCCAATGTGTAAGATTAGTGTGCGAGTGGAAGCTAGGCAGTGTACATGGGTATGTTGTTCAGGAACATT
ATCAATATTATCAGGCC
R
TAGGATGAAGAATCCAGGGAGGACACTTAGCATTAGCAAGAGTGAACATACAAAGAAGGAAGCTACAGCAATACAGCTTTTCT
AAAAATAAACATTTTTGG / Celera SNP ID: hCV2532032 / Public SNP ID: rs4915148 / SNP Chromosome Position: 197035538 / SNP in Genomic Sequence: SEQ ID NO: 471 / SNP Position Genomic: 42514280 / Related Interrogated SNP: hCV2532034 / SNP Source: dbSNP; Celera; HapMap; ABI_Val; HGBASE / Population(Allele,Count): Caucasian (A,20|G,206) / SNP Type: INTRON /

Context        (SEQ ID NO: 2890): /
TCATGGAAAGGGGTATCACACAAGGAAGTTAACATCAGGAGGTGAGGATCAAAGGAATGCTCCTAGAATCAGTCTGCCATTCT
CTCTATGATGCTTGACA
Y

GATGAATATTTTAATTTAATCTTTATTATTTTTTGTAATTATATGACTAAAGGTGCTGATTAATTTTTTTCCATAAGGAAGAAG
AAACAGATGTCCTCCTC / Celera SNP ID:   hCV2532039 / Public SNP ID:   rs1759009 /
SNP Chromosome Position:   197026672 /  SNP in Genomic Sequence:   SEQ ID NO: 471 /
 SNP Position Genomic:   42523146 / Related Interrogated SNP:   hCV2532034 / SNP
Source:   dbSNP; Celera; HapMap; HGBASE / Population(Allele,Count):   Caucasian
(T,20|C,206) / SNP Type:   INTRON /
Context        (SEQ ID NO: 2891): /
CCAGGACTCTTGGCATTTTTACATTTTACACTTTCAAGTCTTTTTTTTATATCAAATAATTTTTGGAATTATGCAGACTACAAA
ATCTGAAATAAGAGAGT
Y

CTACAAGCATCAAAAAATGAGTGTCAGTATTATCAAGGTCTGTTAGAGACATGAAACAGCAACACTGTCACTTTTTCATTTGA
AGTGAGAGTTATACTTC / Celera SNP ID:   hCV2532061 / Public SNP ID:   rs857021 /
SNP Chromosome Position:   197007479 /  SNP in Genomic Sequence:   SEQ ID NO: 471 /
 SNP Position Genomic:   42542339 / Related Interrogated SNP:   hCV2532034 / SNP
Source:   dbSNP; Celera; HapMap; HGBASE / Population(Allele,Count):   Caucasian
(T,19|C,205) / SNP Type:   INTERGENIC;UNKNOWN /
Context        (SEQ ID NO: 2892): /
CTCAGTGATTTCTCTTTGCCCACACTTTTTGCCCTTCAGATCAGTCTCTCCATATTACAGTCAGTGATGTCTTTTAAAAATTC
CCAGTAAGCATCGCATT
Y

TATTTAGTGACTTCCAAAATCTTTAATTTGACCTACAAAGGCCTTCACAATCAGGGCTCCAACCTCTCCAAGTTTCGCATACT
CCTCTCTGTCCTTCACT / Celera SNP ID:   hCV2532062 / Public SNP ID:   rs1332669 /
SNP Chromosome Position:   197005883 /  SNP in Genomic Sequence:   SEQ ID NO: 471 /
 SNP Position Genomic:   42543935 / Related Interrogated SNP:   hCV2532034 / SNP
Source:   dbSNP; Celera; HapMap; HGBASE / Population(Allele,Count):   Caucasian
(C,19|T,207) / SNP Type:   INTERGENIC;UNKNOWN /
Context        (SEQ ID NO: 2893): /
TTAATTCTCTCCGTTCTCTAGTTTGCTAAGGGTAAATGGTCACCAAATAGATAATGTAAAGAGAAATCAAAGGAGAGAAAATC
TGCTATAATAAAGATGA
Y

AAGGTGAAATATTAGTGTAATTCTGCATAAACTCTGCAATGCCACTGACTAGAAAAATCAACCCCAAAAGATTTGGCCACCTT
CATTATGTTTTGAGAAT / Celera SNP ID:   hCV2759661 / Public SNP ID:   rs12731209 /
SNP Chromosome Position:   196947139 /  SNP in Genomic Sequence:   SEQ ID NO: 471 /
 SNP Position Genomic:   42602679 / Related Interrogated SNP:   hCV2532034 / SNP
Source:   dbSNP; Celera; HapMap / Population(Allele,Count):   Caucasian
(C,109|T,9) / SNP Type:   TRANSCRIPTION FACTOR BINDING SITE;INTRON /
Context        (SEQ ID NO: 2894): /
GGCCACCTTCATTATGTTTTGAGAATTTTATTTTCATAGATTTGATAATTTACATTTTAAATGTTATTAAAATGAAGGAATTC
TCATTTCAGCCTAATTA
Y

CATTCAACAGGGAAATATATTTTAATGAAACAATAGAAAATATTATTTTTATATATAAACCAGTGAACTGCCAAAAAATTGTT
TCTTCTGAGCTCTTCCA / Celera SNP ID:   hCV2759662 / Public SNP ID:   rs1170880 /
SNP Chromosome Position:   196947314 /  SNP in Genomic Sequence:   SEQ ID NO: 471 /
 SNP Position Genomic:   42602504 / Related Interrogated SNP:   hCV2532034 / SNP
Source:   dbSNP; Celera; HapMap; ABI_Val; HGBASE / Population(Allele,Count):
Caucasian (T,204|C,18) / SNP Type:   INTRON /
Context        (SEQ ID NO: 2895): /
AAAGAGAAATTTCCCTTGATTTGTCAGCTGAATTTCTGCATGCTTTTTCTAAATAAGGTGAAAAACAAAACAATTTCTTATGC
AGCAATGCTATATTCAG
R

AAATATCTTGAGCTAAAAAATGAGCACACTATATTGAGGAAATATCTCGAGCTAAAACAGCATTCAAAGATAAAGTACATCAA
ATCCATAGAATGTACAA / Celera SNP ID:   hCV2759663 / Public SNP ID:   rs928440 /
SNP Chromosome Position:   196950350 /  SNP in Genomic Sequence:   SEQ ID NO: 471 /
 SNP Position Genomic:   42599468 / Related Interrogated SNP:   hCV2532034 / SNP
Source:   dbSNP; Celera; HapMap; ABI_Val; HGBASE / Population(Allele,Count):
Caucasian (G,111|A,9) / SNP Type:   INTRON /
Context        (SEQ ID NO: 2896): /
TATTGAGGAAATATCTCGAGCTAAAACAGCATTCAAAGATAAAGTACATCAAATCCATAGAATGTACAATATCAAGAGTAAAC
CCTAATGTAAACTATGG

R
CTTTAATGACAATAATACTGATAGTGAAGGGGGCTATTCATGTGGCAGGGAAGAGGAAGTGGTATATAGGAAATCTCTGTACC
TTCCACTCAATTTTACA / Celera SNP ID: hCV2759664 / Public SNP ID: rs928439 /
SNP Chromosome Position: 196950482 / SNP in Genomic Sequence: SEQ ID NO: 471 /
SNP Position Genomic: 42599336 / Related Interrogated SNP: hCV2532034 / SNP
Source: dbSNP; Celera; HapMap; HGBASE / Population(Allele,Count): Caucasian
(G,109|A,9) / SNP Type: INTRON /
Context (SEQ ID NO: 2897): /
GTTTCTCTGGGCAGAACCAGGTAGCATCCTTCTAAGCTTTGGAATACCCTGGGGCAATCACCATGTTAGTGATTCTTTGATCT
TTGACTTAGTGTCCTAT

R
CTACAAGGATGGCACATCTTCTAAGCAAAGTTTGCTTTCATATTCATCAGAATAAAATTTATCTCACAAAATAAAACTATTTA
AAAAGCAAAATGGTAAT / Celera SNP ID: hCV2759673 / Public SNP ID: rs1412639 /
SNP Chromosome Position: 197003593 / SNP in Genomic Sequence: SEQ ID NO: 471 /
SNP Position Genomic: 42546225 / Related Interrogated SNP: hCV2532034 / SNP
Source: dbSNP; Celera; HapMap; HGBASE / Population(Allele,Count): Caucasian
(G,9|A,109) / SNP Type: INTERGENIC;UNKNOWN /
Context (SEQ ID NO: 2898): /
TTGTAGCTATTGAATATTAAATAAGCAATATTACTTCAAGCCTGTTATCAAAATAAGTTTTATTTATTATTAAACAATTTCTT
AGAGTAAATCACAGAAT

R
GTTAAAACATTACCTTTTTCTTTTTCTGCTCTTCTGCATTTCCTAGTAATATAGCTTGGTGTTTCAGAACATCATTTACAAGA
AATGTCATAATCTCTCT / Celera SNP ID: hCV2759678 / Public SNP ID: rs1571964 /
SNP Chromosome Position: 197113073 / SNP in Genomic Sequence: SEQ ID NO: 471 /
SNP Position Genomic: 42436745 / Related Interrogated SNP: hCV2532034 / SNP
Source: dbSNP; Celera; HapMap; ABI_Val; HGBASE / Population(Allele,Count):
Caucasian (G,20|A,206) / SNP Type: INTRON /
Context (SEQ ID NO: 2899): /
TTAGAAAATGTATTTGGCTTTGTGTAATGTTCAAAGTTGAAGAACAGTTGGGGGTAAGACTAAGTTTACTATTCTCTCCTCTT
TGGCCATTAACATTTAC

R
GAATTAAAGGAAGTTTCAGTTACAGCTTTCTCATTAAAAGAAACTTTTGAAACGTTGGCACTGTGTACATTTAATAGTTCCCT
ATTTTCTGATGCATGAA / Celera SNP ID: hCV2759679 / Public SNP ID: rs6677082 /
SNP Chromosome Position: 197112533 / SNP in Genomic Sequence: SEQ ID NO: 471 /
SNP Position Genomic: 42437285 / Related Interrogated SNP: hCV2532034 / SNP
Source: dbSNP; Celera; HapMap / Population(Allele,Count): Caucasian
(G,20|A,206) / SNP Type: SILENT MUTATION;INTRON /
Context (SEQ ID NO: 2900): /
TGGTGTTTAGTTTCCTCATAATCCTATAGCTTTCCAACATACTTCTTAAACTAAGGTATTGAACAGTGGGGACAATATGCCCA
AAGTGGGCAGGCCACAC

R
GGACTGATTGCATTGACTGATGAGAGAGAATAGAGAACAAAAAGTATATTTCAGTACTTTCGGTAAAGAAAATGTACATATAT
AAATTTATGTAAATAAA / Celera SNP ID: hCV2759685 / Public SNP ID: rs877897 /
SNP Chromosome Position: 197103975 / SNP in Genomic Sequence: SEQ ID NO: 471 /
SNP Position Genomic: 42445843 / Related Interrogated SNP: hCV2532034 / SNP
Source: dbSNP; Celera; HapMap; ABI_Val; HGBASE / Population(Allele,Count):
Caucasian (G,9|A,111) / SNP Type: INTRON /
Context (SEQ ID NO: 2901): /
CATCTAAGCTTCTGTTTCGTTAGTGAAGTGGAAATAATAATACATACTTCACTGGGTTTTAAGAAATTTAAATTAGAAAATGT
ATTCACAGCAGCCTCGT

Y
TTGGCATTTGTAAATTTGCAAAAATGAGTTTACTATAGTTGTCATTACGTGCAACACCAAATTATGTTTAAAGAGACTGTACA
GTAAAATGTTGTATTAC / Celera SNP ID: hCV2759688 / Public SNP ID: rs10922169 /
SNP Chromosome Position: 197101105 / SNP in Genomic Sequence: SEQ ID NO: 471 /
SNP Position Genomic: 42448713 / Related Interrogated SNP: hCV2532034 / SNP
Source: dbSNP; Celera; HapMap / Population(Allele,Count): Caucasian
(T,9|C,111) / SNP Type: TFBS SYNONYMOUS;INTRON /
Context (SEQ ID NO: 2902): /
ATGCAGGAGGAAAGGAGAAATTAGCCGTAGCTTCAAATCAGACATGGCAAAAAATTGGAAAAGTAACCAAAAGGGACTAACCA
TGATCAAATGCTTTAAG

W
GACATATCCAGAGAACTGTCATCAGATTCAGATGATGAATTTAATACCACTGAACCAGTTTGCGTACATTCCACAGTTTGAGT
AGTACGCTGACATATAG / Celera SNP ID: hCV2759691 / Public SNP ID: rs4915337 /

SNP Chromosome Position: 197091537 / SNP in Genomic Sequence: SEQ ID NO: 471 / SNP Position Genomic: 42458281 / Related Interrogated SNP: hCV2532034 / SNP Source: dbSNP; Celera; HapMap; ABI_Val; HGBASE / Population(Allele,Count): Caucasian (A,8|T,110) / SNP Type: UTR3;ESS SYNONYMOUS;SILENT MUTATION;INTRON / Context (SEQ ID NO: 2903): /
TTCCAGGACTCAAAGCAAAGATAAAAAAGAACCTGGTTGGTTATGTGGATCTACAAAGTAGAATGGTTCAGTAGGCCAGCATG
TCCCACAGCGTAGAAGA
Y
GCAACAAAGAAGAGAGCAACAGGTACAGAATTAGAATGTTTGCTTTTTCAGAGCAAATGCATTTCAATTATGTTTCAGCCTTC
TGCTGAACACCATAACG / Celera SNP ID: hCV2759693 / Public SNP ID: rs10754215 /
SNP Chromosome Position: 197086669 / SNP in Genomic Sequence: SEQ ID NO: 471 / SNP Position Genomic: 42463149 / Related Interrogated SNP: hCV2532034 / SNP Source: dbSNP; Celera; HapMap / Population(Allele,Count): Caucasian (C,20|T,204) / SNP Type: INTRON /
Context (SEQ ID NO: 2904): /
TGAGCATATCCTGTTGGAAAAAGTGTTGCAGACTTGCTTAACACAGGGTTGCCACAAACCTTCAGTTCGTAAAACACTCAATA
GCTGCAAAGCACAATAA
W
GCAAAGAGCAATAAAACGAGGTATGCCTATATGCCTTAAGAACAATTGTACTCCTAGTACAATTGAAGAGACTGAATACATTT
CACACCAAAAGACATGT / Celera SNP ID: hCV2759696 / Public SNP ID: rs7411719 /
SNP Chromosome Position: 197085676 / SNP in Genomic Sequence: SEQ ID NO: 471 / SNP Position Genomic: 42464142 / Related Interrogated SNP: hCV2532034 / SNP Source: dbSNP; Celera; HapMap / Population(Allele,Count): Caucasian (T,9|A,111) / SNP Type: TRANSCRIPTION FACTOR BINDING SITE;INTRON /
Context (SEQ ID NO: 2905): /
TTGCAAGTGTTCCCCAAAGTTCGTATGTTGGAAACGTAATCTCCAATGTAAAGGTGTTGGGAGGTGGGACATTTAAGATATAA
TTAGGTCGTGAGATCTC
K
ATCCTCATGGAAGGATTAATGCTATTTTCAAGGGGGGTAGGTTAGTTATTGTGGGTGTTAGTTCCTAATAAAAGGAAGCATTT
GGCCCCCATGATGGTTA / Celera SNP ID: hCV2759709 / Public SNP ID: rs4915313 /
SNP Chromosome Position: 197049355 / SNP in Genomic Sequence: SEQ ID NO: 471 / SNP Position Genomic: 42500463 / Related Interrogated SNP: hCV2532034 / SNP Source: dbSNP; Celera; HapMap / Population(Allele,Count): Caucasian (G,9|T,111) / SNP Type: INTERGENIC;UNKNOWN /
Context (SEQ ID NO: 2906): /
AAATTATCACAAATCTCCAAAAAGTTTTCCAACATATATATTGAAAAAAAAATCTGCATAGGAGTGCACTTACATGGTTTAAA
CCCATGTTATTAAAGAG
W
CAACTGTACTGCTTTTGAAAATTCTTTCATCAGTGAACACTTGTTTCCATATCTTGACTACTGTAAATAATCCTGCAATGAAC
ATTGGAGTACAGACACC / Celera SNP ID: hCV2759711 / Public SNP ID: rs4915309 /
SNP Chromosome Position: 197048781 / SNP in Genomic Sequence: SEQ ID NO: 471 / SNP Position Genomic: 42501037 / Related Interrogated SNP: hCV2532034 / SNP Source: dbSNP; Celera; HapMap / Population(Allele,Count): Caucasian (A,9|T,109) / SNP Type: INTERGENIC;UNKNOWN /
Context (SEQ ID NO: 2907): /
TTTTCATTTTCCACTTGGAAAACTCCCCAAGGCTAGTGACGGCCTCTGACCATCCTTTTACCAACTTTCACTTCATCCATACT
CCCTACCACAGCAAAGG
R
TAACAGCCCATGTTTTTGGCCGGGCGCGGTGGCTCACGCCTGAAATCCCAGCACCCTGGGAGGCGAGGGGAGGGATCACGAGG
TCAGGAGATTGCCCGAC / Celera SNP ID: hCV8348876 / Public SNP ID: rs1332662 /
SNP Chromosome Position: 197108427 / SNP in Genomic Sequence: SEQ ID NO: 471 / SNP Position Genomic: 42441391 / Related Interrogated SNP: hCV2532034 / SNP Source: dbSNP; HapMap; ABI_Val; HGBASE / Population(Allele,Count): Caucasian (A,9|G,111) / SNP Type: INTRON /
Context (SEQ ID NO: 2908): /
TCTGTGCACCATACTTGCTCATGTAGTCAGGACTGTTGACAACCAGAGGTGATCACTTGGTGGCTTTTGCAGCTCTGGCACCC
TTTAGCCTACTCTTCAC
R
TGTTGGGAAGTGCCATGAAGGGGGGGTTGAAAGCCACTGAAGGACTTTAAATAGGAACCCTGGCATGCTCAGACTTGGCCAGAA
TGAGAAGGGAATAAAGA / Celera SNP ID: hCV8356383 / Public SNP ID: rs1764800 /
SNP Chromosome Position: 197250615 / SNP in Genomic Sequence: SEQ ID NO: 471 / SNP Position Genomic: 42299203 / Related Interrogated SNP: hCV2532034 / SNP Source: dbSNP; HapMap; HGBASE / Population(Allele,Count): Caucasian

(G,21|A,203) / SNP Type: INTRON /
Context (SEQ ID NO: 2909): /
TAGTTTATTACATGCATAAAACTATAAATGATAAAAATGAAGAATGTAATGAACAGTTTATGTTTTTTTAAAACAAAGTCAGA
TTTTAAAAGTTGTACAC
R
GAGAGCAAAAATCACTTTACGTACTCATGATTGGCTTTAATATTTCTTTACACTATACATACTGAAAATGTTTACATTTACTA
ATAAGGAATGCCAAGCG / Celera SNP ID: hCV8356417 / Public SNP ID: rs12677 / SNP
Chromosome Position: 197053373 / SNP in Genomic Sequence: SEQ ID NO: 471 /
SNP Position Genomic: 42496445 / Related Interrogated SNP: hCV2532034 / SNP
Source: dbSNP; Celera; HapMap; HGBASE / Population(Allele,Count): Caucasian
(G,20|A,206) / SNP Type: UTR3 /
Context (SEQ ID NO: 2910): /
GCCAAGTATTGCTTTGTTTTACTTGGTTGTCTGTTTTTTTCTTTTAATGCTATTTATTTCAAAATTTTTTATGAGTTAGCATAT
GTTAAGGAGAAACTTTA
Y
CTATTCCTGAACTGTACTGTCCAATACAGTGGCCATCAGCCACATATAGCTATTGGAGACAGTACTTAAAATGTCACTAGTTC
AAGATCAGATATTCTGT / Celera SNP ID: hCV8356425 / Public SNP ID: rs1627765 /
SNP Chromosome Position: 197033043 / SNP in Genomic Sequence: SEQ ID NO: 471 /
SNP Position Genomic: 42516775 / Related Interrogated SNP: hCV2532034 / SNP
Source: dbSNP; HapMap; HGBASE / Population(Allele,Count): Caucasian
(C,21|T,205) / SNP Type: INTRON /
Context (SEQ ID NO: 2911): /
AGCCACAGGTTGAAATGTTTTGAGGAAGGGGTTGTGAGCCAAGGAATACAGGTGATTTCAAGAAGCTAGAAAAGGCAAAGTAG
CTGATTCTCCTCCAAAG
Y
CTCCAGCAGGAATGTGGCTCTCCTGATATCTCGATTTTAGTCCAGTGAAACTGACTCTGGACTTCTGGCCTCTAGGACTGTAG
GAGGATAAATCTGTGCT / Celera SNP ID: hCV8356430 / Public SNP ID: rs1759007 /
SNP Chromosome Position: 197027606 / SNP in Genomic Sequence: SEQ ID NO: 471 /
SNP Position Genomic: 42522212 / Related Interrogated SNP: hCV2532034 / SNP
Source: dbSNP; HapMap; HGBASE / Population(Allele,Count): Caucasian
(T,20|C,206) / SNP Type: INTRON /
Context (SEQ ID NO: 2912): /
AATCATTGTTATCAGGGCAAGAGAAAGGGGCTTTGTATATCTTGGTAACTATTGAAACTGGAAAGGCTATCAGTACAAATTCA
CAACAGTATGATAAAGC
R
TAGAATTTTCTCTGTAGGAATTATTATTCCAGACATTAAGAGTCTCTCTAACCTCTCCATACTCTCTTCCCTGCTCTAATCAC
TCCAGTCTTTCTGGTCT / Celera SNP ID: hCV8356446 / Public SNP ID: rs1412634 /
SNP Chromosome Position: 197023156 / SNP in Genomic Sequence: SEQ ID NO: 471 /
SNP Position Genomic: 42526662 / Related Interrogated SNP: hCV2532034 / SNP
Source: dbSNP; Celera; HapMap; ABI_Val; HGBASE / Population(Allele,Count):
Caucasian (A,8|G,110) / SNP Type: INTRON /
Context (SEQ ID NO: 2913): /
TTTGTTAAAAGCAGACACAAACACACACATTAACCTCGGCCTACGGAGGGTCAGGATCATCAGTATCACTGTCTTCCTCCTCA
CTCTTGTTCCACTAGAA
R
GTCTTTAGGGCAGTAACATGCATGGAGCTGTCCTCTCCTATGATAACAAAGCCTTCTTCTGGAATAGTTCCTGAAGGACCCGC
CTGAGGATGTTTTACAG / Celera SNP ID: hCV8356520 / Public SNP ID: rs1170881 /
SNP Chromosome Position: 196949271 / SNP in Genomic Sequence: SEQ ID NO: 471 /
SNP Position Genomic: 42600547 / Related Interrogated SNP: hCV2532034 / SNP
Source: dbSNP; HapMap; HGBASE / Population(Allele,Count): Caucasian
(G,111|A,9) / SNP Type: INTRON /
Context (SEQ ID NO: 2914): /
AGTTTTGGAGACATTGGAGTTCCAATCCAACTATAGTGGGAATACTTCATTGCTTACCTTGGTCAACCACTTACAACCCTAAA
AAGGCCATGCTTTGGAA
R
TAAGGGTCATGTCCTAGAGTAAGGACCATGCTCTAGGACTAAGGACAAAAGCAAAATAAACCCATCCTAACAAAGCATAAAAC
AAAGTCTGACAGGATCA / Celera SNP ID: hCV8690976 / Public SNP ID: rs1124843 /
SNP Chromosome Position: 169369458 / SNP in Genomic Sequence: SEQ ID NO: 471 /
SNP Position Genomic: 70180360 / Related Interrogated SNP: hCV11975250 / SNP
Source: dbSNP; Celera; HapMap; ABI_Val; HGBASE / Population(Allele,Count):
Caucasian (A,81|G,145) / SNP Type: INTRON /
Context (SEQ ID NO: 2915): /
ATATTTTTGACCCTCAGCTTCTTTTTTTTCCTAATCAAAACCAAAACTTTACCCAAGAGAGTTGCCAAAGAAAATAGGATTTTT

GCTCTAGCTTTGAGGCA
Y
GTCAAAATGGTCAACTAGAATTTTCTCAAATTCTGCTTATCTTTATGTATAAAGGCAAAGCAAGATGAATTTAGATTGTGGTG
AAAGTTGAGATGATTTT / Celera SNP ID: hCV8697031 / Public SNP ID: rs1400836 /
SNP Chromosome Position: 169397137 / SNP in Genomic Sequence: SEQ ID NO: 471 /
 SNP Position Genomic: 70152681 / Related Interrogated SNP: hCV11975250 / SNP
Source: dbSNP; HapMap; ABI_Val; HGBASE / Population(Allele,Count): Caucasian
(T,81|C,145) / SNP Type: INTRON /
Context (SEQ ID NO: 2916): /
TTTTCACTTTACGCTTTTTAATTGAAATTAAATTAGTCACTTAACATACATAATGCAGTGTTTGTTTTCCAGTCTAGTAGCTT
TTCAGCATTCAGTTTTG
W
GGCTCATGGGAAATGTTACTTAGCATCAAGATGTGGTTTGATGAGTGAACAATGTCAGTATCAACTGTACTTAACAGAAGACA
GAGGGGCTGGGGTTAAT / Celera SNP ID: hCV8697995 / Public SNP ID: rs4519 / SNP
Chromosome Position: 169890574 / SNP in Genomic Sequence: SEQ ID NO: 471 /
SNP Position Genomic: 69659244 / Related Interrogated SNP: hCV11975250 / SNP
Source: dbSNP; HapMap; ABI_Val; HGBASE / Population(Allele,Count): Caucasian
(T,28|A,198) / SNP Type: MICRORNA;UTR3 /
Context (SEQ ID NO: 2917): /
GATTTTATTTAAATCTTCAAATTTGTGGATAGTTTGATAATTTCTGAATCCTATACTAGGCCTGTGGGCTCAGGATGTAAACT
AATGCCAATAGTCCCTA
Y
CCCTGAGAAAAACATAATTAGGTCAGTTAAACCCACTGAAAAAAAGTCCATGCAAGCTGTTCAATTATTTGTTTAGCCTGAATA
ATTAAAGCTTGACCTCT / Celera SNP ID: hCV8919424 / Public SNP ID: rs974793 /
SNP Chromosome Position: 169478654 / SNP in Genomic Sequence: SEQ ID NO: 471 /
 SNP Position Genomic: 70071164 / Related Interrogated SNP: hCV8919444 / SNP
Source: dbSNP; Celera; HGBASE / Population(Allele,Count): Caucasian
(C,166|T,60) / SNP Type: INTERGENIC;UNKNOWN /
Context (SEQ ID NO: 2918): /
TATTGATCCTTATCCCAGTCCTCTCTTCTCTGGAGTTCTGCACAGGTTGCCTTCATACACATCTAATATCAGATGACCTAGGT
TAAATCATCTCCAAAAG
Y
TTTCACAGTCTGATTCCATCCCACAAGGCTACTCATCTAATTGTCTTTTTCTTTACTACTCAGGGTGTAATCTGTTAGGCTTA
ATAGGCTCATAGTGTCT / Celera SNP ID: hCV8919429 / Public SNP ID: rs970741 /
SNP Chromosome Position: 169480045 / SNP in Genomic Sequence: SEQ ID NO: 471 /
 SNP Position Genomic: 70069773 / Related Interrogated SNP: hCV8919444 / SNP
Source: dbSNP; HapMap / Population(Allele,Count): Caucasian (T,166|C,60) /
SNP Type: INTERGENIC;UNKNOWN /
Context (SEQ ID NO: 2919): /
TGAAATGGAGGAGTTGTTTCTCTTTTTAATTGACAGATAAAATTGTATGCATATACTGTGTATGACATATTGTTTTGAAGTAT
ATATACATTGCAGAATG
W
CAGATAAAGGAGTCTTGACTTTGCAGTTCTTTTCATAAAGAAAGAGCAGAACATAGCTAATACTTGTTCAAGAAAATTTCAAA
TAAATGCCATCTTCTGT / Celera SNP ID: hCV8919436 / Public SNP ID: rs916438 /
SNP Chromosome Position: 169499659 / SNP in Genomic Sequence: SEQ ID NO: 471 /
 SNP Position Genomic: 70050159 / Related Interrogated SNP: hCV8919444 / SNP
Source: dbSNP; Celera; HapMap; HGBASE / Population(Allele,Count): Caucasian
(T,157|A,69) / SNP Type: TRANSCRIPTION FACTOR BINDING SITE;INTRON /
Context (SEQ ID NO: 2920): /
ATTTCATAGCTACCTTTCTCAGAAGCCAAATGCCATCTCCCAACCAAAATCTTAGATGAGTTACTTTGTTTTAAGAGTAACAG
ATCACTAGGAGGGTCCT
Y
CCAGGGCCTCATTCTGGAAGGAGAACCAGTGTCTTGGCTAGGAAGGTCCTCCCAGGGCCTCATTCCGGAAGGAGAACCAGTGT
CTTGGCTTAGGTGTCTC / Celera SNP ID: hCV8919441 / Public SNP ID: rs6032 / SNP
Chromosome Position: 169511555 / SNP in Genomic Sequence: SEQ ID NO: 471 /
SNP Position Genomic: 70038263 / Related Interrogated SNP: hCV8919444 / SNP
Source: dbSNP; HapMap; HGBASE / Population(Allele,Count): Caucasian
(T,89|C,31) / SNP Type: MISSENSE MUTATION;ESS;ESE SYNONYMOUS /
Context (SEQ ID NO: 2921): /
ACCTTTTTTTTTTTAATAAGTTATCCAGTCTCGGGTATGTCTCTATCAGCATCATGAGAACAGAATAATACACTCAGCAATGT
GAATAATAAGGATAATA
R
TCACCAAATACATAGACATGTAAGGACCATTAAATGAATTTAATGTGTTTGGAACAATACCTATCACGCTGTTAAGTGTAAGT

527

TTAAGTGTTGGCTATTA / Celera SNP ID: hCV8919485 / Public SNP ID: rs1800808 / SNP Chromosome Position: 169601129 / SNP in Genomic Sequence: SEQ ID NO: 471 / SNP Position Genomic: 69948689 / Related Interrogated SNP: hCV11975250 / SNP Source: dbSNP; HapMap; HGBASE / Population(Allele,Count): Caucasian (A,105|G,15) / SNP Type: INTRON / Context (SEQ ID NO: 2922): / GTGTCTGTCATCAAGTACAGCACTGAATTGAAACTGAAAACAAGAGTCAAGAAAGAGCAAAGTCAGCCATCTTTATATTCCAC ATGAATCCTTTCCCTTT R TGGTCTTATTTGTTTCTCCTCAGAAAAGACAAAAAGCTGAGCTGTATAAACACCTGTGGGCTGGGGGTTGAGGGATAAATGAG GGGCGAAATGGAAGCTG / Celera SNP ID: hCV8919501 / Public SNP ID: rs909628 / SNP Chromosome Position: 169660665 / SNP in Genomic Sequence: SEQ ID NO: 471 / SNP Position Genomic: 69889153 / Related Interrogated SNP: hCV11975250 / SNP Source: dbSNP; Celera; HapMap; ABI_Val; HGBASE / Population(Allele,Count): Caucasian (A,199|G,27) / SNP Type: TRANSCRIPTION FACTOR BINDING SITE;MICRORNA;UTR3;INTRON / Context (SEQ ID NO: 2923): / TATTTAATATTTACTATGTATATTTTTTAAAGTATTGTTGCTGCTTAATTAACTATTGATGCTTATATTTAATGTTATAGCCT CACTCTTGATCATAATG R GTCAATGCCTCAAATACCTAAAAAAAAAAAAAAATTAGATAGCCAGACACCAGGAAAGAAAAGTATTTCTTTTTTTTAATAAAAA GAAATACCTTTTTGAGC / Celera SNP ID: hCV8919515 / Public SNP ID: rs1569457 / SNP Chromosome Position: 169680074 / SNP in Genomic Sequence: SEQ ID NO: 471 / SNP Position Genomic: 69869744 / Related Interrogated SNP: hCV11975250 / SNP Source: dbSNP; HapMap; HGBASE / Population(Allele,Count): Caucasian (G,109|A,11) / SNP Type: TRANSCRIPTION FACTOR BINDING SITE;INTRON / Context (SEQ ID NO: 2924): / TATAGTGCGAGAATGAGAAATCTTGGTCTAATGGCACTGACTTACCCAAAGTGAGAGCTGAGAGAAACTGTGAAGCAATCATG ACTTCAAGAGTTCTTTT M ACCCAAAGGTTTAGGCTTGAAATACTTTCCTGGGGAGATAAAACACAAAATGAATTAAAGAAGGAAATCGTGGGTAGCTAGTT ACATTATTCTACCATGA / Celera SNP ID: hCV8919530 / Public SNP ID: rs1805193 / SNP Chromosome Position: 169702772 / SNP in Genomic Sequence: SEQ ID NO: 471 / SNP Position Genomic: 69847046 / Related Interrogated SNP: hCV11975250 / SNP Source: dbSNP; HapMap; HGBASE / Population(Allele,Count): Caucasian (C,199|A,27) / SNP Type: UTR5;INTRON;PSEUDOGENE / Context (SEQ ID NO: 2925): / CACCTGAGTTCATGGTCCTGGGTTAGAAGCTGCAGAAAGGACTGTGCTGACTCCCTCAACTAAGGGGTGGTACTACCGCTTGC TCCTCTCAGCAGCTGAC R TCCCAGAGATCAGTTTCCTTTCTGGGAGGGACTCCTCATTGAGGGGGGTGCAGAAGACCTGCAGACCTGCATCAACCCTGGTC TTTGAGGCTTCAACTCC / Celera SNP ID: hCV9102829 / Public SNP ID: rs3795732 / SNP Chromosome Position: 156564640 / SNP in Genomic Sequence: SEQ ID NO: 471 / SNP Position Genomic: 82985178 / Related Interrogated SNP: hCV9102827 / SNP Source: dbSNP; Celera; HapMap; ABI_Val; HGBASE / Population(Allele,Count): Caucasian (G,56|A,168) / SNP Type: TRANSCRIPTION FACTOR BINDING SITE;MICRORNA;UTR3 / Context (SEQ ID NO: 2926): / AATAAGCTGCGGGGGCTGTACCCTGCAAAGCCACAGGGGCAGAGCAGCCCAAGGCCTTGGGAGCCCACCTCTTGCATCATTGT GTTTTGGATGTGAGACA Y GGAATCAAAGGAGTTTATTTTGGTGCTTTAAGATTTAATAAGTGCCCTGCCAGTTTTCAGATTTGCATGGGGCCTGCAGCCCC TTTGGTTTGGCCAAGTT / Celera SNP ID: hCV9114630 / Public SNP ID: rs1576880 / SNP Chromosome Position: 196992511 / SNP in Genomic Sequence: SEQ ID NO: 471 / SNP Position Genomic: 42557307 / Related Interrogated SNP: hCV2532034 / SNP Source: dbSNP; Celera; HapMap; HGBASE / Population(Allele,Count): Caucasian (C,9|T,109) / SNP Type: INTERGENIC;UNKNOWN / Context (SEQ ID NO: 2927): / CTACCATCAGTGGGCAATGCCATTCTAGAATAAGGATGCTCATGGAGTCAGGTTTAAATACCAAATTTGCTCTTATGAGTTGA GTGACTCTAGGCAGGTT Y TTACATTCTCTGGGCTTCGTCTGTGAAGTGATGCAATAACACATACATCACAAAGTGATATTCACATCAATGGCATACAGTAC ATGGTAACTGCATTATC / Celera SNP ID: hCV9575263 / Public SNP ID: rs898658 /

528

SNP Chromosome Position: 173541768 / SNP in Genomic Sequence: SEQ ID NO: 471 / SNP Position Genomic: 66008050 / Related Interrogated SNP: hCV16180170 / Related Interrogated SNP: hCV8911768 / SNP Source: dbSNP; Celera; HapMap; ABI_Val; HGBASE / Population(Allele,Count): Caucasian (T,168|C,58) / SNP Type: INTRON /
Context (SEQ ID NO: 2928): /
TTCTTTAACTTGGTGTCTGAGGGGTTTTTGTCTGTGGCTTGTCCTGCTACAATTTGACCTAGCAATTCTATTACTGGGTATAT
AGCCAAAAAAGAAAATA
M
ATTGTTCAGCCGGGCGCAATGGCTCATGCCTGTAATCCCAGCACTTTGGGAAGCTGAGGCAGGCAGATTGCCCGAGGTCAGGA
GTTTGAGACCAGCCTGG / Celera SNP ID: hCV9945852 / Public SNP ID: rs1121789 /
SNP Chromosome Position: 169503616 / SNP in Genomic Sequence: SEQ ID NO: 471 / SNP Position Genomic: 70046202 / Related Interrogated SNP: hCV8919444 / SNP Source: dbSNP; Celera; HapMap; HGBASE / Population(Allele,Count): Caucasian (A,79|C,29) / SNP Type: INTRON /
Context (SEQ ID NO: 2929): /
CACCTTTACTCAAGTGCCTTCAAAGAAAAAAAATGGAAATACTAAAAGTATACTTTATTTTCTATACATAAAGTACATCAAGA
AAATAAACATTGCTCTC
Y
TATTAACAAAATGTACTGAATATAGTGTATTCAACATACCTTCACTGACAGCATGGGGTTTAACAATGCAACAGGTACAATTA
GTAAATTTAGCAGTGTT / Celera SNP ID: hCV11341772 / Public SNP ID: rs4589164 /
SNP Chromosome Position: 169256501 / SNP in Genomic Sequence: SEQ ID NO: 471 / SNP Position Genomic: 70293317 / Related Interrogated SNP: hCV8919444 / SNP Source: dbSNP; Celera; HapMap; HGBASE / Population(Allele,Count): Caucasian (C,155|T,71) / SNP Type: INTRON /
Context (SEQ ID NO: 2930): /
AGCTCCTGCACAGCAAAAGAAACTATCAAGAGAGTAAACAGACAACATTCAGAATGGGAGAAAATTTTTGCAAACTATGCATC
TGGCAAAGGTCTAATAT
S
GAGCATTTATAAGGAACTTAAATTTACAAGAAAAAAAACAAACAGCCCCATTAAAAAGTAGGCCAAGGACAGGAACAGACACTT
TTCAAAAGAAGACACAC / Celera SNP ID: hCV11342057 / Public SNP ID: rs10919186 /
SNP Chromosome Position: 169516700 / SNP in Genomic Sequence: SEQ ID NO: 471 / SNP Position Genomic: 70033118 / Related Interrogated SNP: hCV8919444 / SNP Source: dbSNP; Celera / Population(Allele,Count): Caucasian (G,176|C,50) /
SNP Type: MISSENSE MUTATION;ESE;INTRON /
Context (SEQ ID NO: 2931): /
AATTTTATGGAGACTTTCTGGAAAAAATATTTCCCAACATTATGCTGTTTGGCCAATTTCCAGAGACAACCAATGGTTACTTT
TAAATTTTTATCCAGTT
K
TATTGCTGCCTTTTGGTTGGGAGTGAATATACCAAGACCCTCACATAGCCATTCTCGAAGTCCCACCTGAACAAAATGATTTT
GAATTATAATTCTGTTC / Celera SNP ID: hCV11888484 / Public SNP ID: rs6694672 /
SNP Chromosome Position: 196945789 / SNP in Genomic Sequence: SEQ ID NO: 471 / SNP Position Genomic: 42604029 / Related Interrogated SNP: hCV2532034 / SNP Source: dbSNP; Celera; HapMap / Population(Allele,Count): Caucasian (T,207|G,19) / SNP Type: INTERGENIC;UNKNOWN /
Context (SEQ ID NO: 2932): /
ACATTTTCAGCATGTTTGTAGGTTGGTGAATGATTAAGTGGAAAGGGAGACATTGATGTTACAGAAGAAAGGAGATAATTCAT
GAAGAAGGATCTTTTAC
W
AGTTTAAAGGTGAAAGAATCTAGTGTCATCATGAAGAAGTCAATCTGAGAAAGCAAGGACAGTAACTCAATGAAACTAGAGGA
TGTACAAGCAATTTGGG / Celera SNP ID: hCV11888496 / Public SNP ID: rs7554757 /
SNP Chromosome Position: 196973054 / SNP in Genomic Sequence: SEQ ID NO: 471 / SNP Position Genomic: 42576764 / Related Interrogated SNP: hCV2532034 / SNP Source: dbSNP; Celera; HapMap / Population(Allele,Count): Caucasian (A,6|T,104) / SNP Type: INTRON /
Context (SEQ ID NO: 2933): /
CTCTTGCCTTGACCCCAAAAGTGCTTCCTTGATAAGGATATAATATATATTTTGGAGAGTTCCCTAGAAATAAAATGAGTATC
TCCAACCCAGATACCCT
R
AAGAGGCCAATCATGGCAAAATGCTAAAGGCCATTCCTTCTAAGCAAAACTGGCTATCCATCATACATACCAAAGCCAATTTT
GAGCATTCCAGTTTGTT / Celera SNP ID: hCV11888556 / Public SNP ID: rs7542397 /
SNP Chromosome Position: 197096720 / SNP in Genomic Sequence: SEQ ID NO: 471 / SNP Position Genomic: 42453098 / Related Interrogated SNP: hCV2532034 / SNP

Source: dbSNP; Celera; HapMap / Population(Allele,Count): Caucasian (G,9|A,111) / SNP Type: INTRON /
Context (SEQ ID NO: 2934): /
TGCAACCATTTTCTTCTTTAGGAAGTCTTTCTTGGAACCATCTCATACTTCCTTGCAATTTACACACAAACCCTCTGCTTCTG
TGCTTCCTTAATTTCCT
R

AGCATAGCATAATCACAGGACTGAACGCAACTTGAGGACAGGAATTATCCCTTTCCTCCCTGTGTACACAGTGCCTAGAAAAA
TGCACATCACATAGTAG / Celera SNP ID: hCV11888566 / Public SNP ID: rs1888991 /
SNP Chromosome Position: 197110822 / SNP in Genomic Sequence: SEQ ID NO: 471 /
SNP Position Genomic: 42438996 / Related Interrogated SNP: hCV2532034 / SNP
Source: dbSNP; Celera; HapMap; HGBASE / Population(Allele,Count): Caucasian
(A,21|G,203) / SNP Type: INTRON /
Context (SEQ ID NO: 2935): /
CAAGGTTGGGGGCTTCTCAGGGGGTAAGAATGAGGAGCAGAAGGCTGGAGACAAGAGTTTTCCCCAGGGTTTGTAAGTATTGT
TGATGAACCATGGGTGT
R

TGCTGCTTAGAGAAGGAAGGGAAGCCATGAGGGGACCCAGAGGTATCATGTAATGAAGGGACTGAGAGAGCTGGGTATCTCCA
TGAAATGTGAGAGTGGG / Celera SNP ID: hCV11975195 / Public SNP ID: rs1894692 /
SNP Chromosome Position: 169467654 / SNP in Genomic Sequence: SEQ ID NO: 471 /
SNP Position Genomic: 70082164 / Related Interrogated SNP: hCV11975250 / SNP
Source: dbSNP; HapMap / Population(Allele,Count): Caucasian (G,1|A,119) / SNP
Type: INTERGENIC;UNKNOWN /
Context (SEQ ID NO: 2936): /
GGCCCAGATGGTTTCACTAGTGATTCCTACCAAATATTTAAAGAATTATTACCAATTGCAGACATTTTCTCCTAGAATGTAGA
AAAGAGCATGCTTACCA
R

TTTTATGAAAGCTAGTATAACTGTGGTATCAAAAACAGAAAAAGACAATTAAAAAAAATAAAACTATATACCAATATCCTCTCA
TGAACATAGACGACAAA / Celera SNP ID: hCV15802110 / Public SNP ID: rs2420371 /
SNP Chromosome Position: 169491555 / SNP in Genomic Sequence: SEQ ID NO: 471 /
SNP Position Genomic: 70058263 / Related Interrogated SNP: hCV11975250 / SNP
Source: dbSNP; HapMap; HGBASE / Population(Allele,Count): Caucasian
(G,14|A,210) / SNP Type: INTRON /
Context (SEQ ID NO: 2937): /
ATGAATATATAAAGCTCTCTGGTAAAGGTAAATATATAAACAAGCATAAAAACAGTATTATTGTAATTTTGGTTTGTAACTCC
GCTTTTTATTTTCTACA
K

GATTTAAAAGGCAAATGCATAAAATGTAATTATAAATATGTTAGGTGGTGTACAATGAATAAAGATATAATCTGTGACATCAA
TAACCTAAAAAGAGTAG / Celera SNP ID: hCV15832929 / Public SNP ID: rs2151134 /
SNP Chromosome Position: 197105536 / SNP in Genomic Sequence: SEQ ID NO: 471 /
SNP Position Genomic: 42444282 / Related Interrogated SNP: hCV2532034 / SNP
Source: dbSNP; Celera; HapMap / Population(Allele,Count): Caucasian
(T,9|G,107) / SNP Type: INTRON /
Context (SEQ ID NO: 2938): /
ACCTTCCAACATTCTCTTTTGCTCTTAACGGAATGGAAATCTTAGAAATGTTGATGGGACAATGACATGAATCATGAAAAGAA
AGGAATAGTGGGAATAC
R

AATATTAGAAAGCCAATGTTTTGTGGATGTTTGAAACTCTCATATACATTCATAGGGCTTACCCCACTGGTATGGGCAACAGG
TAACTCTGGTAGTTTCT / Celera SNP ID: hCV15847759 / Public SNP ID: rs2187952 /
SNP Chromosome Position: 169481950 / SNP in Genomic Sequence: SEQ ID NO: 471 /
SNP Position Genomic: 70067868 / Related Interrogated SNP: hCV8919444 / SNP
Source: dbSNP; Celera; HapMap; HGBASE / Population(Allele,Count): Caucasian
(G,88|A,30) / SNP Type: UTR3 /
Context (SEQ ID NO: 2939): /
TTTTAGTTTTGAAAGACCCAAGTGTCATTCTCAGTAGACATTTTGAAAGACCCAAATGTCATTCTCAGTAGGTACATCTCAGT
AACATCTGTTGAAGGGT
R

TAACAACGAAGCTTGATCCACCAGTGGACTGAAATGATACTGGGCTTACTAAGACTGAAGAGTTCCTATAACCAGTAATTAAA
AATGGGCCAGCCTCCTA / Celera SNP ID: hCV15864094 / Public SNP ID: rs2068871 /
SNP Chromosome Position: 173796169 / SNP in Genomic Sequence: SEQ ID NO: 471 /
SNP Position Genomic: 65753649 / Related Interrogated SNP: hCV16180170 /
Related Interrogated SNP: hCV8911768 / SNP Source: dbSNP; HapMap; ABI_Val;
HGBASE / Population(Allele,Count): Caucasian (A,205|G,21) / SNP Type: INTRON
/

Context (SEQ ID NO: 2940): /
TGAGTACCTACACAGGGTATGAAAGAAAATATAAAGAAGAATTGTGGTTTCAGGAAGTTGGGAAGCCAATTCAGATTTCAAAA
ACACTCATTGAACACCA
Y
ATATATGGCAGTGATGTTGCCAGATACTGTCATGACATTGAAGTTTGAGTGACCTGAGGACTTTGGAAGAGTCAGGCCTAGTT
TGAATCTCAGGTAGGTC / Celera SNP ID: hCV15955265 / Public SNP ID: rs2227244 /
SNP Chromosome Position: 169489358 / SNP in Genomic Sequence: SEQ ID NO: 471 /
 SNP Position Genomic: 70060460 / Related Interrogated SNP: hCV8919444 / SNP
Source: dbSNP; HapMap; HGBASE / Population(Allele,Count): Caucasian
(T,87|C,31) / SNP Type: INTRON /
Context (SEQ ID NO: 2941): /
CAGCGGCACCAAGGGAGATGTGGAAGGCAGGCTGAAGAAGCAGAGAGAGAGAGGAGAGGACCCTGCCATTCATCTTCCCATCT
CCACTGCCTTCGGTTGC
Y
CACTCTGTCCTTCAACTACATTTGGAAATGCAATTTGAGGCTGAGAGGACCCTGGGAGGGCTATGAAGCACAAATTAAAACCA
AGACAGTTGTGCCACCA / Celera SNP ID: hCV15956059 / Public SNP ID: rs2227592 /
SNP Chromosome Position: 173885712 / SNP in Genomic Sequence: SEQ ID NO: 471 /
 SNP Position Genomic: 65664106 / Related Interrogated SNP: hCV16180170 /
Related Interrogated SNP: hCV8911768 / SNP Source: dbSNP; HapMap; ABI_Val;
HGBASE / Population(Allele,Count): Caucasian (C,203|T,23) / SNP Type:
UTR5;INTRON /
Context (SEQ ID NO: 2942): /
AAGTAACTCCCAAATGAGGTTCCAGATTCATTTTTCTCATCCCAGGTAGTAAACGTGTTTTAATGGGTTTGTTTCTTGCCCAT
GGCAACCTCAACACAGA
S
GGGAGAACTGTTTTATTACCCTAAAATAGTGAGAACCACTGTTCAAACTAACTAGCTACCAAGTGACTTCTAACTCATGAAGA
AAATTTGTATTTAAGAG / Celera SNP ID: hCV16135173 / Public SNP ID: rs2146372 /
SNP Chromosome Position: 173869569 / SNP in Genomic Sequence: SEQ ID NO: 471 /
 SNP Position Genomic: 65680249 / Related Interrogated SNP: hCV16180170 /
Related Interrogated SNP: hCV8911768 / SNP Source: dbSNP; HapMap; ABI_Val;
HGBASE / Population(Allele,Count): Caucasian (G,110|C,10) / SNP Type:
PSEUDOGENE /
Context (SEQ ID NO: 2943): /
AAAGACATGAAAACACAATAATCCATTGTGGCAGGTAATTTCTAATAAACTTTCGTCAGGAATATTCAAGTATGGGCTGTGGA
CCACTGCTTGTCTGTGA
M
CTATTGATTACTGGGTCAAAATAACACAAGGAGAGAAAAATGAGAATGAAATATTTAGTAAACTTTTGTTTTTCTAAAGCAAT
TTGACATTGCTGTGACT / Celera SNP ID: hCV16175730 / Public SNP ID: rs2239851 /
SNP Chromosome Position: 169512497 / SNP in Genomic Sequence: SEQ ID NO: 471 /
 SNP Position Genomic: 70037321 / Related Interrogated SNP: hCV8919444 / SNP
Source: dbSNP; HapMap; ABI_Val; HGBASE / Population(Allele,Count): Caucasian
(C,167|A,59) / SNP Type: INTRON /
Context (SEQ ID NO: 2944): /
AGGGGAGATGGGGATGCTGCTTAGATGAGGCGCATATGGAAAGGGAGGCAGCTCGAGCTTGGCAGTTATGGATCTTTGAGCCA
CATTTAGAGTTCATCCC
Y
ATCCCCATCAGTCCAACACCTTCATATCCACCGATCAATCTACACCTCTCTGGTTCAAAGGCATCGAGAAAAAATGAAGCACC
TCACTTTCTGCACTCTT / Celera SNP ID: hCV26627664 / Public SNP ID: rs3795727 /
SNP Chromosome Position: 156589450 / SNP in Genomic Sequence: SEQ ID NO: 471 /
 SNP Position Genomic: 82960368 / Related Interrogated SNP: hCV9102827 / SNP
Source: dbSNP; Celera; HapMap; ABI_Val; HGBASE / Population(Allele,Count):
Caucasian (C,178|T,48) / SNP Type: MISSENSE MUTATION;ESS;SILENT MUTATION;INTRON
/
Context (SEQ ID NO: 2945): /
CCAATAAAAACCCTAGAAGAAAACCTAGGAAATACTACTCCGGATATCAGCCTTGGGAAAGAATTTATAACAAAGTCCCCAAA
AGCAATTGCAACAAAAA
M
CATAATTGACAAATGGGACCTAATTAAACTAAAGAGATTCTGCACAGCAAAATAAACTATCAACAGAATAAACAGACAACCTA
CAGAATGGGAGAAAATA / Celera SNP ID: hCV26753641 / Public SNP ID: rs10801590 /
SNP Chromosome Position: 197080203 / SNP in Genomic Sequence: SEQ ID NO: 471 /
 SNP Position Genomic: 42469615 / Related Interrogated SNP: hCV2532034 / SNP
Source: dbSNP; Celera; HapMap / Population(Allele,Count): Caucasian
(A,9|C,109) / SNP Type: INTRON /

Context          (SEQ ID NO: 2946): /
TAAATGTCAAAGATGTGGAGAAATTGGAACCCCTATGCATTGTTTGTGGGAATGTAAAATGTTGAAAACAGTGTGGCAGATCC
TCAAAATATTAAACATA
S
AGTTACCATATGGCCTAGCAATTCTGCTTCTAGGTAGATTCCCAAAAGAAAGCAGAAACTAGAATAGGTATTTGTACAACAGT
GTTCACAGCTGCATTAT / Celera SNP ID: hCV27242533 / Public SNP ID: rs2138898 /
SNP Chromosome Position: 169358811 / SNP in Genomic Sequence: SEQ ID NO: 471 /
 SNP Position Genomic: 70191007 / Related Interrogated SNP: hCV8919444 / SNP
Source: dbSNP; Celera; HapMap; ABI_Val; HGBASE / Population(Allele,Count):
Caucasian (G,53|C,67) / SNP Type: MISSENSE MUTATION;INTRON /
Context          (SEQ ID NO: 2947): /
ACTCTCACACACTGTTGGAGGAACTATAAATTCATATTATCTTTTGGGAGGTTACTTTTGCATCTTTAACGAAACATTTCACT
CTAAGCCTTCAAACATT
W
TAAATTCACCTCTGAAGAACTTCTCCTACAGATTACTGATGTTCCCATAGAATGTACCTCTTCACTACAGCACTGTTAACTAA
CGCAACATTGGAACCAA / Celera SNP ID: hCV27242639 / Public SNP ID: rs7544221 /
SNP Chromosome Position: 169387381 / SNP in Genomic Sequence: SEQ ID NO: 471 /
 SNP Position Genomic: 70162437 / Related Interrogated SNP: hCV11975250 / SNP
Source: dbSNP; Celera; HapMap / Population(Allele,Count): Caucasian
(T,82|A,142) / SNP Type: TRANSCRIPTION FACTOR BINDING SITE;INTRON /
Context          (SEQ ID NO: 2948): /
AACTGTATCTTCAGATACACAGGTAAGAAGAATGCCATATGATTATTAAGATACAACAATGCCATCTGTGCCATGCAGTGCTT
AAGTACCTACCATTTTA
Y
GATAAAACAGAATAATTTTTTCTTAATGTTTTAGTAATTTTTATCTGCTAGCTTGAAATAATTGGAGATAGAGGCTTTTGAAAG
ATACATTCCACATGAGT / Celera SNP ID: hCV27478380 / Public SNP ID: rs3766141 /
SNP Chromosome Position: 169792692 / SNP in Genomic Sequence: SEQ ID NO: 471 /
 SNP Position Genomic: 69757126 / Related Interrogated SNP: hCV11975250 / SNP
Source: dbSNP; HapMap; HGBASE / Population(Allele,Count): Caucasian
(T,198|C,28) / SNP Type: INTRON /                    .
Context          (SEQ ID NO: 2949): /
ATGACAATGATATTTATCCTCCTATGAGGGCAACCTGGTGTAGTGAGAAATAAAACAAACCAAAACAGACAACCAGGAGTTTG
TCTGAGACCAGGCACCT
K
AAGAACTAAGATTTAGAAGACTTAAAGAGGTGGTACATGTCACTGCATATTTGTCAAATGCAAAATACTGTTATTCTCATTAT
AGCACAGTCTTCAGATT / Celera SNP ID: hCV27490260 / Public SNP ID: rs3820060 /
SNP Chromosome Position: 169484552 / SNP in Genomic Sequence: SEQ ID NO: 471 /
 SNP Position Genomic: 70065266 / Related Interrogated SNP: hCV8919444 / SNP
Source: dbSNP; HGBASE / Population(Allele,Count): Caucasian (T,157|G,69) /
SNP Type: TRANSCRIPTION FACTOR BINDING SITE;INTRON /
Context          (SEQ ID NO: 2950): /
CTAACAAAAAGCTGATGATGAACAACTCAAAGATCTAAATAAATGGAGATATGTATCACCTTTATGAATCAGAATAATCAACA
TAGTAAAGTTGTCAGTG
Y
ACCTCAAAATATCTGTAAATGTGATGCAATTTCAATCAAAATTTGACAGTATTTTTTGAGGACATAGAAAAACTAACCTAAAA
CTTAAATGGAAAGACAA / Celera SNP ID: hCV27898327 / Public SNP ID: rs4915327 /
SNP Chromosome Position: 197079130 / SNP in Genomic Sequence: SEQ ID NO: 471 /
 SNP Position Genomic: 42470688 / Related Interrogated SNP: hCV2532034 / SNP
Source: dbSNP; HapMap; HGBASE / Population(Allele,Count): Caucasian
(T,9|C,111) / SNP Type: INTRON /
Context          (SEQ ID NO: 2951): /
CTTGAAGATAAACTATAAACCCCTCCACCACCTGCCAAACCTTAGCTATTCCAAATGGAGGCAGTTCTAACAAGAGGTAGCAA
GGCCTTGGAATAGTCTC
K
TTAGGGATCTTTAATGTTTCCCCTTATTGGTAAACAAACTCACCAACTTAGAATATCTACCTTGTGGCTAAGAATTCACTGGA
ACAGAATAATAATGCCA / Celera SNP ID: hCV28998001 / Public SNP ID: rs6425251 /
SNP Chromosome Position: 173556428 / SNP in Genomic Sequence: SEQ ID NO: 471 /
 SNP Position Genomic: 65993390 / Related Interrogated SNP: hCV16180170 /
Related Interrogated SNP: hCV8911768 / SNP Source: dbSNP; HapMap /
Population(Allele,Count): Caucasian (T,168|G,58) / SNP Type: INTRON /
Context          (SEQ ID NO: 2952): /
ATCTTGCTGGATATTTTATATGAACAGTGTTAATTTAGATGCACTAAAGCAAAGGTAGGCAAACTACAACCATGAGTCAAACA
TGGCCACACCCATTCAT

K
TGCTATTGTCTAAGCTGGTTTTGCACTACAACTGCAGAGTTGAATAGATGCAGCAGATCCTTTACAGAAAAAGTTTTCTGACC
TCAATTCTAAAGTAATT / Celera SNP ID: hCV29397237 / Public SNP ID: rs6427185 /
SNP Chromosome Position: 169356833 / SNP in Genomic Sequence: SEQ ID NO: 471 /
SNP Position Genomic: 70192985 / Related Interrogated SNP: hCV11975250 / SNP
Source: dbSNP; HapMap / Population(Allele,Count): Caucasian (G,81|T,145) /
SNP Type: TRANSCRIPTION FACTOR BINDING SITE;UTR3;INTRON /
Context (SEQ ID NO: 2953): /
TGATTAATAGTGCTATGAACATGGGTGCACAAATATCTCTTCAAGACCTGCTTTCAAACCACTGCCTCATTTTAAAATCAAGG
TATACTTAGGAACCCAG
R
GGAGTCACATAGATTGCTCAGAGGTCTCCGTAAATGAAAACACAAAATGTCAATATTACACTTCTGGAACATGACTTTAGTGT
GAGGTACTCCTCGTAAA / Celera SNP ID: hCV32141485 / Public SNP ID: rs3917744 /
SNP Chromosome Position: 169577990 / SNP in Genomic Sequence: SEQ ID NO: 471 /
SNP Position Genomic: 69971828 / Related Interrogated SNP: hCV11975250 / SNP
Source: dbSNP; HapMap; HGBASE / Population(Allele,Count): Caucasian
(A,84|G,142) / SNP Type: INTRON /
Context (SEQ ID NO: 2954): /
TAGCATTTATCTGCTGTTCTGCAGGAAGTCTTTCAGACTTCGAATGCCTAAGATGGGGCAGCCACCACTCCCCACCACTGAGA
CTCAGTGAGCGTAAATG
R
TTTGCCCAAGCTTGCTCACATGAATGTGCATAGGGTTAACACAGTTATTCTTATTCTTCCAGGGCTTATGAATTTTGAAAATC
TGATAAAAGCCGTGGTC / Celera SNP ID: hCV32141499 / Public SNP ID: rs3917862 /
SNP Chromosome Position: 169593113 / SNP in Genomic Sequence: SEQ ID NO: 471 /
SNP Position Genomic: 69956705 / Related Interrogated SNP: hCV11975250 / SNP
Source: dbSNP; HapMap; ABI_Val; HGBASE / Population(Allele,Count): Caucasian
(A,209|G,17) / SNP Type: INTRON /
Context (SEQ ID NO: 2955): /
GCAGTGATGACAACCATAAAAAAGAAATATTGAGTGATATGGGGAGAGTAGTGTAATTGTGTTTACCTCAAAACTGTTCAAAT
TATATGAACAAACACAG
Y
AAACTTAGGTACCACAACAAATTTCTTGTTACTTTTCTCACAACTGCTAAAAATACTACAGTAAGCTTCCAACCAGGATGAGA
ACCATTCACAAAGCTAT / Celera SNP ID: hCV32398748 / Public SNP ID: rs3917417 /
SNP Chromosome Position: 169700062 / SNP in Genomic Sequence: SEQ ID NO: 471 /
SNP Position Genomic: 69849756 / Related Interrogated SNP: hCV11975250 / SNP
Source: dbSNP; HGBASE / Population(Allele,Count): Caucasian (C,106|T,8) / SNP
Type: INTRON;PSEUDOGENE /
Context (SEQ ID NO: 2956): /
TACAGTTTATTGAATGTGTCCTGTGTGCCAGGCACCATGTAACCATGAGCCTATGAAGTTCACACTATTATTATCCTCATTTT
ACAATGAGAAAACTGAC
W
TAGAGAGTTAAACTATCTTGTCAAGGTGCCAAAATAAATAACTGGTGAATCTAGGACTCAAACCCAGCAGGGTCTGACTTCAT
AGTCTCAGCTCACGATC / Celera SNP ID: hCV32141639 / Public SNP ID: rs3917411 /
SNP Chromosome Position: 169700756 / SNP in Genomic Sequence: SEQ ID NO: 471 /
SNP Position Genomic: 69849062 / Related Interrogated SNP: hCV11975250 / SNP
Source: dbSNP; HGBASE / Population(Allele,Count): Caucasian (A,199|T,27) /
SNP Type: MICRORNA;UTR3;INTRON /
Context (SEQ ID NO: 2957): /
TTAGTAGTGTCTCTCATGTAGTTGTGGTAGTAATTAGAATTTCAGAAACAGAAGGAAACCAAGAATAGGTTTGTCATCCATAG
TCTACTACCTTCAATTT
M
TCATTCATAGCTGTGGATAACCAATCACTACTCATTTTTTCTTCCTTTTTCACCTGCCAATTCAACATATTTAACATGCACTG
TCTCACAGAGGAATGAC / Celera SNP ID: hCV32141645 / Public SNP ID: rs3917452 /
SNP Chromosome Position: 169703617 / SNP in Genomic Sequence: SEQ ID NO: 471 /
SNP Position Genomic: 69846201 / Related Interrogated SNP: hCV11975250 / SNP
Source: dbSNP; HapMap; HGBASE / Population(Allele,Count): Caucasian
(C,197|A,27) / SNP Type: INTRON /
Context (SEQ ID NO: 2958): /
GTGAATGGTCATAAACACCTGGTGGGTTTTAAATCTTTACAAGTACTTTAAAAAACTCTTGGATAGTTGAAATATATACAGGC
ATATCTCGAAGATATTG
Y
AGGTTCAGTTCCAGACCACCACAATAAAGCAAATATTACAATAAAGTGAGTCACACAATTTTTTTTGGTTTCCCAATACATACC
GAAGTTATCTTTACACT / Celera SNP ID: hCV31565488 / Public SNP ID: rs6671696 /

SNP Chromosome Position: 197083196 / SNP in Genomic Sequence: SEQ ID NO: 471 / SNP Position Genomic: 42466622 / Related Interrogated SNP: hCV2532034 / SNP Source: dbSNP; HapMap / Population(Allele,Count): Caucasian (T,7|C,103) / SNP Type: INTRON /
Context (SEQ ID NO: 2959): /
ATATTACAATAAAGTGAGTCACACAATTTTTTTTGGTTTCCCAATACATACCGAAGTTATCTTTACACTATACTGCAGTCTATT AAGTGTGCAAAACCATT
R
TGTCTAAAACACTACAAGGTACATACCTAAATTTAAAAAATACTAAATTGCTAAAAAATGCTAAGAACCATGTGAGCCTTCAGC AAATTGTAATCTTTTTG / Celera SNP ID: hCV29904681 / Public SNP ID: rs6680497 /
SNP Chromosome Position: 197083329 / SNP in Genomic Sequence: SEQ ID NO: 471 / SNP Position Genomic: 42466489 / Related Interrogated SNP: hCV2532034 / SNP Source: dbSNP; HapMap / Population(Allele,Count): Caucasian (G,9|A,111) / SNP Type: INTRON /
Context (SEQ ID NO: 2960): /
CAGGTAGAGAATTTATTTGCAAAGGATTGAGGTTTTCTAAGACTTGGATTACAGATAGAAAGGAACGAATGGGCAGGAGAGTG TGTGCAAGGGACTGACT
K
ACAGTGATGAGCCATGGAATCTAATTTAGATGTGGTGGGAAGAGAGAAAATGAAGGGAAGAGAATAGTGAAAAAGTGTCAGGG TCATTGAATGTTTGGAA / Celera SNP ID: hCV30373286 / Public SNP ID: rs6702340 /
SNP Chromosome Position: 196945050 / SNP in Genomic Sequence: SEQ ID NO: 471 / SNP Position Genomic: 42604768 / Related Interrogated SNP: hCV2532034 / SNP Source: dbSNP; HapMap / Population(Allele,Count): Caucasian (G,111|T,9) / SNP Type: INTERGENIC;UNKNOWN /
Context (SEQ ID NO: 2961): /
CCGGCGGTGGGGAGCTTCCTCTCTCCTCTAAACAGTCCATTTCTGATTGTTCCAAACTCAGAGACTCGGTGACTACAACAAGG AAATTGAACAGCAGTTA
Y
TTTGCTTAAATAAATATTACATAACGAGCTGAGAATATTATTGTGAAGACACTGATTAGATACTATTTGCTTATTTCACACAA CCCTCCATGAACTTTGA / Celera SNP ID: hCV29724082 / Public SNP ID: rs9427661 /
SNP Chromosome Position: 196946546 / SNP in Genomic Sequence: SEQ ID NO: 471 / SNP Position Genomic: 42603272 / Related Interrogated SNP: hCV2532034 / SNP Source: dbSNP; HapMap / Population(Allele,Count): Caucasian (T,207|C,19) / SNP Type: INTERGENIC;UNKNOWN /
Context (SEQ ID NO: 2962): /
TCCCTTTTGAAATACATTATATTGAACTGACTGAAAAAAGGGAGAAAAACATGTTTCAGTGGTTGAAGAACATGGTAGATGAAG TAGTTCATTTTGAATGG
Y
TTTAGTTCCTTAACAAAAAAGTCAGTGGTGAAGTTGCATATTTTAGTATAATATTTAGGAGCTGCAGTTGGGGAATTTGAAAT GGCTTTTGTGAATTATT / Celera SNP ID: hCV30205817 / Public SNP ID: rs10489254 /
SNP Chromosome Position: 174197801 / SNP in Genomic Sequence: SEQ ID NO: 471 / SNP Position Genomic: 65352017 / Related Interrogated SNP: hCV16180170 / Related Interrogated SNP: hCV8911768 / SNP Source: dbSNP; HapMap / Population(Allele,Count): Caucasian (C,209|T,17) / SNP Type: INTRON /
Context (SEQ ID NO: 2963): /
ATCTGGTTAAGATGATGCCACACATTGACTGGAAGCATCTCCATTTTTTTAGAAGGAAAAGGGAATAGGAACTCATATTTATTG ACTGCCCTACCTTGAGC
Y
CAGTGATGTGCTGGTAAATGTTTAAAGCCGGCTCTTTTCTGCAATTATACAGGCAGTAAGGGAAAAAAGCTTTTTAAAACCCA GTTCAAGGTTGGGGCGG / Celera SNP ID: hCV30804119 / Public SNP ID: rs10912651 /
SNP Chromosome Position: 173549157 / SNP in Genomic Sequence: SEQ ID NO: 471 / SNP Position Genomic: 66000661 / Related Interrogated SNP: hCV16180170 / Related Interrogated SNP: hCV8911768 / SNP Source: dbSNP; HapMap / Population(Allele,Count): Caucasian (C,87|T,31) / SNP Type: INTRON /
Context (SEQ ID NO: 2964): /
ACCTAAAACCACCTGATCTTTGACATAGTTGACAATAACAAACAATGGGGAAAGGATATGCTATTCAATAAACGGTCAGTGCA GAAGATTAAAATTGGAC
S
CTTTCCTTTCACCACATACAAAAAAATTAATGCAAGATGGATTAAAGACTTAAATGTAAGACCCAAACCAATAAAAACCCTAGA AGAAAACCTAGGAAATA / Celera SNP ID: hCV31565485 / Public SNP ID: rs10922166 /
SNP Chromosome Position: 197080036 / SNP in Genomic Sequence: SEQ ID NO: 471 / SNP Position Genomic: 42469782 / Related Interrogated SNP: hCV2532034 / SNP Source: dbSNP; HapMap / Population(Allele,Count): Caucasian (G,9|C,111) / SNP

Type:    INTRON /
Context            (SEQ ID NO: 2965): /
GAGGCTGGCTTATTTCTCTTAGCACAATGCATTTTAAATTCTTCCTTGTTGTGCCTATCGATACTCCATTCCTTTTTTATTGC
TGAGTAATATTTCATTG
W
AAGATGTTCCACGATTTGTTTATTCATTCACCAATGGAAGAATGTTTGTTTCCAGACATTTGAGATTATAAATAAAGCTGCTA
TACAGATTCATATAAAG / Celera SNP ID:    hCV30804135 / Public SNP ID:    rs12078293 /
SNP Chromosome Position:    173565287 / SNP in Genomic Sequence:    SEQ ID NO: 471 /
SNP Position Genomic:    65984531 / Related Interrogated SNP:    hCV16180170 /
Related Interrogated SNP:    hCV8911768 / SNP Source:    dbSNP; HapMap /
Population(Allele,Count):    Caucasian (A,165|T,61) / SNP Type:
UTR3;INTRON;PSEUDOGENE /
Context            (SEQ ID NO: 2966): /
TCTGACTTACTGAGTCAGAGTTTTTGCTGAGCTTTTGAAATGTGAGTCATTGAACCTCAAGGGGGACCCAGATCTATCTCTTA
CTGATGCTTTGCTTCCT
R
CAGACTTACCCATGTATTGCCTCTTTTTATTATTTAAATGTTTTGAAGATATTTAAGATAGTTTAGTGCAAAACAATCATACA
TGTGTACATTTTCAGAG / Celera SNP ID:    hCV31565509 / Public SNP ID:    rs12092294 /
SNP Chromosome Position:    196966853 / SNP in Genomic Sequence:    SEQ ID NO: 471 /
SNP Position Genomic:    42582965 / Related Interrogated SNP:    hCV2532034 / SNP
Source:    dbSNP; HapMap / Population(Allele,Count):    Caucasian (G,19|A,207) /
SNP Type:    INTRON /
Context            (SEQ ID NO: 2967): /
TCTACCAAATATTTTCTTTTCCCAGCAATTACTCGAGACTACCTTGGTCCTAGCTCCCTTAGCTTGAAAGTTCCAAAGATAAT
GGCTTGCACTCTGAGTA
Y
TAAGGATGCACTGTACTGGCTGATATTCTGGTATCCAGCGGTAACGTTACTCCGGCCCTTGCTTTCGTGTTTCAGGTATATAT
TCCCTCTGCGGAACTCA / Celera SNP ID:    hCV32141874 / Public SNP ID:    rs12121045 /
SNP Chromosome Position:    169862071 / SNP in Genomic Sequence:    SEQ ID NO: 471 /
SNP Position Genomic:    69687747 / Related Interrogated SNP:    hCV11975250 / SNP
Source:    dbSNP; HapMap / Population(Allele,Count):    Caucasian (T,198|C,28) /
SNP Type:    INTRON /
Context            (SEQ ID NO: 2968): /
TCATAGTGGTTCTGTTTTTCTGATCAAACTCTGACTGATACAAGCCCAATTAAAAAGAGGTGCCCCCATTAGTCTCAGAATTT
TCTTTAGTGGCATTACT
Y
ACTGGATTCTGGATCTTAGATCTGTGGATGATCCCTATCAGGTCAGGAAAGCTTCAGGAGCCACCAGTGGGATTTTGATGTAA
GGGATTCCAGGGGATAC / Celera SNP ID:    hCV32141892 / Public SNP ID:    rs12125595 /
SNP Chromosome Position:    169876537 / SNP in Genomic Sequence:    SEQ ID NO: 471 /
SNP Position Genomic:    69673281 / Related Interrogated SNP:    hCV11975250 / SNP
Source:    dbSNP; HapMap / Population(Allele,Count):    Caucasian (T,198|C,28) /
SNP Type:    INTERGENIC;UNKNOWN /
Context            (SEQ ID NO: 2969): /
TCTGAAGGATGGCTGTGTCAACTGAGATCAAAGGGGAACCTGTGATAAGAGGAAAATACCCTGGTAGGATTTACAGTGGAAAC
ACAAAGACTACTAGTGC
R
TCACGGATAGCAATGCCACCATAACCTGAAATAAAGTTAAATGCTAGGCTCTGCCTTCCCACCTACATGCCCTCAGAAAAGAA
GGACCAAGCAAATTGGC / Celera SNP ID:    hCV32141894 / Public SNP ID:    rs12128308 /
SNP Chromosome Position:    169876994 / SNP in Genomic Sequence:    SEQ ID NO: 471 /
SNP Position Genomic:    69672824 / Related Interrogated SNP:    hCV11975250 / SNP
Source:    dbSNP; HapMap / Population(Allele,Count):    Caucasian (G,197|A,29) /
SNP Type:    INTERGENIC;UNKNOWN /
Context            (SEQ ID NO: 2970): /
GCTCCTCAGGTTCACTCCAGTCAGGCCACTCCTCAGACTTTTTAGAACTTGATGGGAAGTTTTCACTGTCCTCCGAAGTATTT
TTTACATCTGAGAGTCC
R
TTTATGGGAAATTTAATAGGCTGAGAAAATGGATCGCCTGTAGGGAAAATAATTATTCTCATAAAATACTGGTTAAAGGTAAG
AACTACACTTATGGGAA / Celera SNP ID:    hCV32141820 / Public SNP ID:    rs12132384 /
SNP Chromosome Position:    169824068 / SNP in Genomic Sequence:    SEQ ID NO: 471 /
SNP Position Genomic:    69725750 / Related Interrogated SNP:    hCV11975250 / SNP
Source:    dbSNP; HapMap / Population(Allele,Count):    Caucasian (A,198|G,28) /
SNP Type:    ESS;ESE;SILENT MUTATION /
Context            (SEQ ID NO: 2971): /

TTACCCATTGTCCACTTTTCCCAAACTACATAGTGTTATATGGTATATGACCCAATCAACGGTGGCAAAGCTCCAGAAATACC
ACATAGACATCAGGGAC
R
CTTTAAAACTAATCAGCCTATAGTCCTTTTTCAGTAATTTCCAAACCTGGTTGTGCATCCAAATCACTTGGTAACATTAAAAAA
ACAAAAAAAATATACACG / Celera SNP ID: hCV32141621 / Public SNP ID: rs12133642 /
SNP Chromosome Position: 169689800 / SNP in Genomic Sequence: SEQ ID NO: 471 /
SNP Position Genomic: 69860018 / Related Interrogated SNP: hCV11975250 / SNP
Source: dbSNP; HapMap / Population(Allele,Count): Caucasian (A,199|G,27) /
SNP Type: INTRON /
Context (SEQ ID NO: 2972): /
AAGAATCTCCATAAACGTGCTAAGTGCCCTGGAAGCACTGGGGTGACTAATTTTGTTGGGCAGGGGAATGCCTAGGGGAAGGA
GTCACAGAAAGGAGATT
R
AAGCCAGATCTTAGATGAGTTCACATTTTCCAGAAGCCAATGGGAAGAAATGCATTCCAGCAACAGAATGGCCTACAAAGAAG
AAGAGTGTGAAGGAAGA / Celera SNP ID: hCV32141844 / Public SNP ID: rs12142093 /
SNP Chromosome Position: 169843609 / SNP in Genomic Sequence: SEQ ID NO: 471 /
SNP Position Genomic: 69706209 / Related Interrogated SNP: hCV11975250 / SNP
Source: dbSNP; HapMap / Population(Allele,Count): Caucasian (A,198|G,28) /
SNP Type: INTRON /
Context (SEQ ID NO: 2973): /
GCACACATTGTTTATGCCCAAGTATAAGAAAGTATTTATTTTTTCAAAGTCATCCTCAGGAAAAAAAAATAGTTGTGTTATTCAA
TCCTTTTTCTTATTTTCC
R
TCTCTGTCTTTTGTTTTACTTTCTGTGAGGTTTTCACAATTTGACCATTAGGTCCTTTTTATTTCTTTATCTATTGTAACGTCA
TATGGTTTTTCTTCTTA / Celera SNP ID: hCV30804128 / Public SNP ID: rs10798290 /
SNP Chromosome Position: 173557584 / SNP in Genomic Sequence: SEQ ID NO: 471 /
SNP Position Genomic: 65992234 / SNP Source: dbSNP; HapMap /
Population(Allele,Count): Caucasian (A,85|G,33) / SNP Type: INTRON /
Context (SEQ ID NO: 2974): /
GGAACCTGGACTTTGACCCCCACTTGGCAGTAAACAGGCTGTGCCCTACCCCTTTTCCTGACAGAGCAATGTCAGAAAAAGCC
AACTTTTTAAAACAGAA
R
ATTGGAGCAGGTATCGGTAATACTGAAAACAGAAAAACAATAGAGAAAAATCAATAAAACAAGGCTGGTTCTGTGAAAAGATT
TTTTTTTTTTTTTTTTTT / Celera SNP ID: hCV30036717 / Public SNP ID: rs9332653 /
SNP Chromosome Position: 169490772 / SNP in Genomic Sequence: SEQ ID NO: 471 /
SNP Position Genomic: 70059046 / Related Interrogated SNP: hCV8919444 / SNP
Source: dbSNP; HapMap / Population(Allele,Count): Caucasian (G,177|A,47) /
SNP Type: ESE SYNONYMOUS;SILENT MUTATION;INTRON /
Context (SEQ ID NO: 2975): /
GGATTTCCTTCTTTTTTAAGGCTGAATAATATTTAATTGTATGTATATGTTACATTTTTCTTTCTCTTTTATTTTATTTATTT
TTTTTTTTTTGGAGACT
S
GGTTTCACTCTGTTGCAGTGTAGTCGTATGATCTTAGCTCACTGCAGCCTTGAACTCCCAGGCTCAATCAATCCTCCCTCCTC
AGCCTCCTGAGTAGCTG / Celera SNP ID: hCV31565527 / Public SNP ID: rs12039586 /
SNP Chromosome Position: 196997452 / SNP in Genomic Sequence: SEQ ID NO: 471 /
SNP Position Genomic: 42552366 / Related Interrogated SNP: hCV2532034 / SNP
Source: dbSNP; HapMap / Population(Allele,Count): Caucasian (C,9|G,111) / SNP
Type: INTERGENIC;UNKNOWN /
Context (SEQ ID NO: 2976): /
GTGCCTACATCGTAGCAACAAAGCTTCTAACTATATTTTGAAACAGATTAAACTGCGAACAGTCCAGAGTGATGGCTCAGATC
TGGAGCGAATTAAAATA
W
CAATCGTACTTTAAGGAAGGGGCCAAAATATTACACTCACAAGAAGGCGGCATGGCAGACACTGGGCCTCCACTATTCTGAAG
GATGGCTGTGTCAACTG / Celera SNP ID: hCV32141893 / Public SNP ID: rs12125679 /
SNP Chromosome Position: 169876817 / SNP in Genomic Sequence: SEQ ID NO: 471 /
SNP Position Genomic: 69673001 / Related Interrogated SNP: hCV11975250 / SNP
Source: dbSNP; HapMap / Population(Allele,Count): Caucasian (T,198|A,28) /
SNP Type: INTERGENIC;UNKNOWN /
Context (SEQ ID NO: 2977): /
GTGTTATATGGTATATGACCCAATCAACGGTGGCAAAGCTCCAGAAATACCACATAGACATCAGGGACACTTTAAACTAATCA
GCCTATAGTCCTTTTTC
W
GTAATTTCCAAACCTGGTTGTGCATCCAAATCACTTGGTAACATTAAAAAAAACAAAAAAAATATACACGCAACATTCGCTCCCA

ATCCTACTGAATCAGAA / Celera SNP ID: hCV32141622 / Public SNP ID: rs12133666 / SNP Chromosome Position: 169689832 / SNP in Genomic Sequence: SEQ ID NO: 471 / SNP Position Genomic: 69859986 / Related Interrogated SNP: hCV11975250 / SNP Source: dbSNP; HapMap / Population(Allele,Count): Caucasian (A,87|T,9) / SNP Type: INTRON /
Context (SEQ ID NO: 2978): /
ACTTTTTAGAACTTGATGGGAAGTTTTCACTGTCCTCCGAAGTATTTTTTACATCTGAGAGTCCATTTATGGGAAATTTAATA
GGCTGAGAAAATGGATC
R
CCTGTAGGGGAAAATAATTATTCTCATAAAATACTGGTTAAAAGGTAAGAACTACACTTATGGGAAGGATAGCATTAAGGAAGTG
TAGTAGTATAGGTCTAA / Celera SNP ID: hCV32141821 / Public SNP ID: rs12135726 / SNP Chromosome Position: 169824104 / SNP in Genomic Sequence: SEQ ID NO: 471 / SNP Position Genomic: 69725714 / Related Interrogated SNP: hCV11975250 / SNP Source: dbSNP; HapMap / Population(Allele,Count): Caucasian (G,198|A,28) / SNP Type: ESE;TRANSCRIPTION FACTOR BINDING SITE;SILENT RARE CODON;SILENT MUTATIO / N //
Context (SEQ ID NO: 2979): /
TTTCTGTGGTCTCTTCCACACTGCTACCAAAGTGAACTTTCTAGCACACACAAATTGGCCCACTCTTCTTTAGGGGAAGCAGT
TTCCTAAGTAATGAGAG
S
TTTCATTGTCTTCCACATGCTCAACATTTACTCTTTGACCTCAGTCCAATTCTGAGACCTATGATGACAACTCCATTTCAGAG
TTAAAGTGCTTCATTCC / Celera SNP ID: hCV32141828 / Public SNP ID: rs12136425 / SNP Chromosome Position: 169830104 / SNP in Genomic Sequence: SEQ ID NO: 471 / SNP Position Genomic: 69719714 / Related Interrogated SNP: hCV11975250 / SNP Source: dbSNP; HapMap / Population(Allele,Count): Caucasian (G,198|C,28) / SNP Type: INTRON /
Context (SEQ ID NO: 2980): /
GCTGTGGGGAGAGGAGTAGCTACTCCAGGCTGCTGCCCTAGCTAAGGTGACCCTCCCCTTCTGCTGGAAGTACCATGCCATAT
GGCCTCTGCATCAAGGG
Y
TCTTATGGGATATTCTCAGAGAATCTCTGCCGTTTCATCTGTTCTGATATCTACCCAAGCATTTTGAAAAACATCCCAATTCA
CTGAAGCAAGTCCAACT / Celera SNP ID: hCV32141586 / Public SNP ID: rs12137905 / SNP Chromosome Position: 169672867 / SNP in Genomic Sequence: SEQ ID NO: 471 / SNP Position Genomic: 69876951 / Related Interrogated SNP: hCV11975250 / SNP Source: dbSNP; HapMap / Population(Allele,Count): Caucasian (C,109|T,11) / SNP Type: INTRON /
Context (SEQ ID NO: 2981): /
GGGAGGCAGAGGTTGCAGTAAGCCGAGATCGCACCACTGCACTCCAGCCTGGGTGACAGAGAGAAATTCCGTTTCCCAGCACT
CCCCAGAAAAACCTAAA
Y
GGTAAATGAGGCTGAAGGTAGGCTGCAGAAAGATTGTGAAGGGCCTTATATCCCATGGCAAAGAATTTGGAATAAACAGCACA
GAAAGGATTACATGCTA / Celera SNP ID: hCV32141847 / Public SNP ID: rs12143057 / SNP Chromosome Position: 169844093 / SNP in Genomic Sequence: SEQ ID NO: 471 / SNP Position Genomic: 69705725 / Related Interrogated SNP: hCV11975250 / SNP Source: dbSNP; HapMap / Population(Allele,Count): Caucasian (T,198|C,28) / SNP Type: INTRON /
Context (SEQ ID NO: 2982): /
TTTTTAATGGAGTTGTTTTCTTGTAAATTTGTCTAAATTCCTTATAGATGCTAGATATTAGACCTTTGTCACATGCATAGTTT
GCAAATATTTTATCCTA
Y
TCTATAGGCTGTTTCCTCTGTTGATAATTTCTTTTGCTGTGCATAAGCCCTTAAGTTTAATTAAATCCTATTTGTTAATTTTT
GCTTTTGTTGCAGTTGC / Celera SNP ID: hCV29517287 / Public SNP ID: rs2901747 / SNP Chromosome Position: 173560948 / SNP in Genomic Sequence: SEQ ID NO: 471 / SNP Position Genomic: 65988870 / Related Interrogated SNP: hCV16180170 / Related Interrogated SNP: hCV8911768 / SNP Source: dbSNP; HapMap / Population(Allele,Count): Caucasian (T,80|C,30) / SNP Type: INTRON /
Context (SEQ ID NO: 2983): /
TATTAAAAGTCAAATTTGAGAAAACTAAAAATTGAGGATACTCATAAAGACATTAGAAATGTTAAAAAATAAAAACAAGTAGC
TAGACTTGGTAATTTCA
R
TGAGAAATGATTGATAGGCCCTGATGTGGTTTGGCTCTGTGTCCCCACCCAAATCGCCTGTCAAATTCTAATTCCCAGTGTTG
GGGGAGGGACCTGGTGG / Celera SNP ID: hDV70670007 / Public SNP ID: rs16828222 / SNP Chromosome Position: 169873570 / SNP in Genomic Sequence: SEQ ID NO: 471 /

SNP Position Genomic: 69676248 / Related Interrogated SNP: hCV11975250 / SNP Source: dbSNP; HapMap / Population(Allele,Count): Caucasian (G,198|A,28) / SNP Type: INTERGENIC;UNKNOWN /
Context (SEQ ID NO: 2984): /
TAGTGAAATTAGTCATATAACCATGAAAAAATATTTTCAGTGTCAAGATTTCACAAATGAAGAAAATTAGGAGTCTCAAATTT
TTATACATCATGCATTC
R
TAAATTGTGGAGGGACGGATCAGACCACTGGCGAGCTGGAGGGCTGGCAGACACCCCTGGACCAACTTGGTCAAAGTGGGACC
CTTTTTCCTCAAGAACA / Celera SNP ID: hDV70683090 / Public SNP ID: rs16846433 /
SNP Chromosome Position: 173820593 / SNP in Genomic Sequence: SEQ ID NO: 471 /
SNP Position Genomic: 65729225 / Related Interrogated SNP: hCV16180170 /
Related Interrogated SNP: hCV8911768 / SNP Source: dbSNP; HapMap /
Population(Allele,Count): Caucasian (G,205|A,21) / SNP Type: INTRON /
Context (SEQ ID NO: 2985): /
CTGGAGGGAGATATTGTCATCCATCCAGTTTCCTCTCTATACCAAATACAAAGTACACATAAATAAGCAAAACAAATTTTCAA
AGTTCCTTCTCTAGAAT
S
CTCTTTCTTTTGCATATGTAGTTTCAGTGGAGATTTCTCAACTAGCTGAAATGGAGCACTTGAATATATTAAAAGAGCCCAAA
CTGCTTACAGCAATTTC / Celera SNP ID: hDV70683187 / Public SNP ID: rs16846561 /
SNP Chromosome Position: 173869111 / SNP in Genomic Sequence: SEQ ID NO: 471 /
SNP Position Genomic: 65680707 / Related Interrogated SNP: hCV16180170 /
Related Interrogated SNP: hCV8911768 / SNP Source: dbSNP /
Population(Allele,Count): Caucasian (C,201|G,23) / SNP Type: PSEUDOGENE /
Context (SEQ ID NO: 2986): /
TAAGTCTTCCTTGGATACCATCTCACTGCTCCTTGCTCTTCTGAGGACAAGTATGGTTGAAAAATTCTTTGGTAATTCTGTTT
AGTGTTTTGTCAAGTTC
S
AGAATTAAAGTTCATTGCGGGTGGAGGCAGTATTTTCTAACTCTTTGTCCTCTACCGGGCTGTGCATATGGGAAGTACTCAAT
ACATTTCTTTAGTAAAT / Celera SNP ID: hDV70683212 / Public SNP ID: rs16846593 /
SNP Chromosome Position: 173898217 / SNP in Genomic Sequence: SEQ ID NO: 471 /
SNP Position Genomic: 65651601 / Related Interrogated SNP: hCV16180170 /
Related Interrogated SNP: hCV8911768 / SNP Source: dbSNP /
Population(Allele,Count): Caucasian (C,209|G,17) / SNP Type: INTRON /
Context (SEQ ID NO: 2987): /
AAAATTGTTTTTAACAGTAACTGATAATATATGGTTTGACCACCACTTAATTAACCATTTCTTTTTTTTTTTGTACATTTAGAA
TGTTTTCCTCCAGTTCA
S
ATATAGACAGTTAATAGGAAACAGTTATATTATTTAATGCTCAACTTTGTTCCTTAGCTGTGCCTTTAATTCTAAGAGTAGTT
GCTAGCTAGGTATTTTA / Celera SNP ID: hDV70683382 / Public SNP ID: rs16846815 /
SNP Chromosome Position: 174220542 / SNP in Genomic Sequence: SEQ ID NO: 471 /
SNP Position Genomic: 65329276 / Related Interrogated SNP: hCV16180170 /
Related Interrogated SNP: hCV8911768 / SNP Source: dbSNP; HapMap /
Population(Allele,Count): Caucasian (G,206|C,18) / SNP Type: INTRON /
Context (SEQ ID NO: 2988): /
AGTTGAGTAAATCAAAGATGGGACTGTTAAGTTATTTGTGTTATTTACCTGCTTTTTGAAAATGTAAAAATAAAACTCTAGGT
TTAATTAGTAGTATGCT
R
TTTAGTAATGAAGTAAAGCTAGAGGCTTCGAACAAATCTTGTGTAATTTCCTCTTGAATGAGAGAGAAAATTTAAAGTAAGCA
AACAAATAAGTTGTGTG / Celera SNP ID: hDV70965007 / Public SNP ID: rs17529304 /
SNP Chromosome Position: 169781291 / SNP in Genomic Sequence: SEQ ID NO: 471 /
SNP Position Genomic: 69768527 / Related Interrogated SNP: hCV11975250 / SNP
Source: dbSNP; HapMap / Population(Allele,Count): Caucasian (A,198|G,28) /
SNP Type: INTRON /
Context (SEQ ID NO: 2989): /
TAAAAAAAAAGTATGAAAGGATTCCCACTGAAAATCCCCCCGAGAAGTTCAGAAAGAGAAATTAACTTTTTATTTATACTTCT
TTATATCATTTGACTGA
R
TATGACCCTCCTACATTACTTATATAATACTAAAAATCTAAAAAAAATGACACACAAAAAGTTAGACCTTTTAATAATTCTTAC
CTTCTAGAGAAAGGTCA / Celera SNP ID: hDV70966798 / Public SNP ID: rs17543370 /
SNP Chromosome Position: 169828098 / SNP in Genomic Sequence: SEQ ID NO: 471 /
SNP Position Genomic: 69721720 / Related Interrogated SNP: hCV11975250 / SNP
Source: dbSNP / Population(Allele,Count): Caucasian (A,194|G,28) / SNP Type:
INTRON /

Context (SEQ ID NO: 2990): /
TAAACTTTTCAAGAAATTCACAACTTCCAGAAAATCATTTCTAAATGCCAAAAAGAGAAGGAATTCAGCAGAACAGATTAGAG
AATGTTAAATATTGAGG
Y
CATAATCACAGACTACATCAATATAATTTTTCAACCAAGAAACATTCTCCTTTGCCATTTAAAGATTCTATTACAACTAAGTAG
CTTATGAGACAAATACT / Celera SNP ID: hDV70966830 / Public SNP ID: rs17543611 /
SNP Chromosome Position: 169836175 / SNP in Genomic Sequence: SEQ ID NO: 471 /
SNP Position Genomic: 69713643 / Related Interrogated SNP: hCV11975250 / SNP
Source: dbSNP / HapMap / Population(Allele,Count): Caucasian (T,198|C,28) /
SNP Type: INTRON
Context (SEQ ID NO: 2991): /
GATCAGATCCAGTGAGGTGTGTAATAACCTGAACAGCTGACATCTATGTCTCCATGGCTGATGGTCTGGATTATCACTTACAC
ACTTTACTAAATCCTTA
R
CATATCTTTCACAAAGCAGATAAAGAGTGATTTTTATGTTATGTGTTTTATGTCTGAAGTTTCCTTTAAATAAAAGCTTTCTT
AGTGCATTATGTTGTAG / Celera SNP ID: hDV70974851 / Public SNP ID: rs17601631 /
SNP Chromosome Position: 169800690 / SNP in Genomic Sequence: SEQ ID NO: 471 /
SNP Position Genomic: 69749128 / Related Interrogated SNP: hCV11975250 / SNP
Source: dbSNP / Population(Allele,Count): Caucasian (A,198|G,28) / SNP Type:
INTRON /
Context (SEQ ID NO: 2992): /
GTTTGAAGATCTTGGTTCGTTCTCCTCCCACAACCACCTCTGGTCCAAGCAGAGAGACCAGCACTGCTAGGCTATGCAGAGTA
ATTGCCACAATGGAATC
R
CTAGTATCACGCAGGCCCAGCAAAACCTAGGAGCAAATGAGGTAATAAGGGTTGACAACCACAATACCTATCTCTTTCTTCCC
CTGTAAATGAGCAATTG / Celera SNP ID: hDV70975002 / Public SNP ID: rs17602701 /
SNP Chromosome Position: 169828327 / SNP in Genomic Sequence: SEQ ID NO: 471 /
SNP Position Genomic: 69721491 / Related Interrogated SNP: hCV11975250 / SNP
Source: dbSNP; HapMap / Population(Allele,Count): Caucasian (G,194|A,28) /
SNP Type: SILENT MUTATION;PSEUDOGENE /
Context (SEQ ID NO: 2993): /
TCTCACATTATTTTGTGGCCAGAGCTTATACCTGCATTTCATCTTGGTGTATACTAGGATTGTTGTTTTTTACTTGTAACAGT
GAAGAAAGTGTATATTG
Y
TATCCAGTAACAGAAAACACTATATATCTGCTTGAAATAATCGTTAAAACTACCAAAGGTGTGACCAACAACCCAAACTCTAG
AGAAAGTGAGGCAAGCT / Celera SNP ID: hDV70975134 / Public SNP ID: rs17603666 /
SNP Chromosome Position: 169856028 / SNP in Genomic Sequence: SEQ ID NO: 471 /
SNP Position Genomic: 69693790 / Related Interrogated SNP: hCV11975250 / SNP
Source: dbSNP; HapMap / Population(Allele,Count): Caucasian (T,198|C,28) /
SNP Type: INTRON /
Context (SEQ ID NO: 2994): /
TCTCTCATATCACTCTTTTCTCATTCTTTCTACCTGTGATCATTTCTCCTGAGATCCTTCATGAGCACTTATCCAGGACCATA
AACTACTATAATTTCTC
R
GCTTTTGTCTTTTGTATATTGCTCTTCTGACTCTAATCTACATGTATCCTTTGGTCAAACTTACTGACTTTTGCGGACTCACA
AATCTATGTGATCTTTG / Celera SNP ID: hDV71028805 / Public SNP ID: rs4987299 /
SNP Chromosome Position: 169675349 / SNP in Genomic Sequence: SEQ ID NO: 471 /
SNP Position Genomic: 69874469 / Related Interrogated SNP: hCV11975250 / SNP
Source: dbSNP; HapMap / Population(Allele,Count): Caucasian (A,199|G,27) /
SNP Type: INTRON /
Context (SEQ ID NO: 2995): /
CTTAACTCCCCCAACATTTATGCAGGGTAACATTCTACCAATTTTTACAACTTAGTGTTTTTCTAATTTGTCTTCCACTTGTC
AACTCTACTACCATTGC
Y
GTCATTATCTTTCTTCTAGATTCTTGCAATAGTTTATTAGATGAGTTCCACAATTTTAAATTAGCTCCCCTCAAATACATTAT
TCATACTACAGGGAGAC / Celera SNP ID: hDV71028807 / Public SNP ID: rs4987302 /
SNP Chromosome Position: 169674983 / SNP in Genomic Sequence: SEQ ID NO: 471 /
SNP Position Genomic: 69874835 / Related Interrogated SNP: hCV11975250 / SNP
Source: dbSNP; HapMap / Population(Allele,Count): Caucasian (C,109|T,11) /
SNP Type: INTRON /
Context (SEQ ID NO: 2996): /
AAATTCTTCTGATTCCTTGAGTCCATTCTTCCTTGAAGTCTCTAGAGCAAGGTAGCAATAATGGCTTAAATTTGACAGATGAG
GAATAAGACATGAAAGT

K
GTACTGTTTACTAAATGGCATGGCAGGGTCTAAAAATGTGAAAACTTTGATTTCCAAGTCTTAAATTTACCTATTGTACCACCA
ATCAGAACAGGGAAATA / Celera SNP ID: hDV71028808 / Public SNP ID: rs4987304 /
SNP Chromosome Position: 169674561 / SNP in Genomic Sequence: SEQ ID NO: 471 /
SNP Position Genomic: 69875257 / Related Interrogated SNP: hCV11975250 / SNP
Source: dbSNP; HapMap / Population(Allele,Count): Caucasian (T,109|G,11) /
SNP Type: INTRON /
Context (SEQ ID NO: 2997): /
TCTTAAATTTACCTATTGTACCACCAATCAGAACAGGGAAATAGATACAGTTTCTGGAGAGGGTTGTCAAAATCAAAAGAGTT
AAAATTCTGGAACTGAG

M
TAGAATAGAAGACAGAATTGAGGTCAAAATGCTTCCTAGACTTTTATATTCTCTCCTTGCAAAGAAGGCTTATTATGGAGACC
CACTCTTTCTCTGAACA / Celera SNP ID: hDV71028809 / Public SNP ID: rs4987307 /
SNP Chromosome Position: 169674403 / SNP in Genomic Sequence: SEQ ID NO: 471 /
SNP Position Genomic: 69875415 / Related Interrogated SNP: hCV11975250 / SNP
Source: dbSNP; HapMap / Population(Allele,Count): Caucasian (C,109|A,11) /
SNP Type: INTRON /
Context (SEQ ID NO: 2998): /
TACTTATTCGTGAAGGAAGTAGAGGGAAGTGGTAACTACTGCATTGGAAAGAAATTAGTGCAGGTCCTTGGCATGTAAAAAATC
AAGTGCTTATTTTTGCA

S
AACTGCTTTAAAGTAAAAGCATTAATGATTTCCTTTGACTGGTGAATTATCCAGTTCAGATCATTGGCTTTTATTTTTCCTGA
AGTTGTATTAGGCTTTT / Celera SNP ID: hDV71028814 / Public SNP ID: rs4987323 /
SNP Chromosome Position: 169671277 / SNP in Genomic Sequence: SEQ ID NO: 471 /
SNP Position Genomic: 69878541 / Related Interrogated SNP: hCV11975250 / SNP
Source: dbSNP; HapMap / Population(Allele,Count): Caucasian (G,199|C,27) /
SNP Type: TFBS SYNONYMOUS;INTRON /
Context (SEQ ID NO: 2999): /
GCATTGGAAAGAAATTAGTGCAGGTCCTTGGCATGTAAAAAATCAAGTGCTTATTTTTGCACAACTGCTTTAAAGTAAAAGCAT
TAATGATTTCCTTTGAC

K
GGTGAATTATCCAGTTCAGATCATTGGCTTTTATTTTTCCTGAAGTTGTATTAGGCTTTTAGCTCTTCTCTGTGTTTCAGAGT
TCCAGTCATTGAACAAG / Celera SNP ID: hDV71028815 / Public SNP ID: rs4987324 /
SNP Chromosome Position: 169671237 / SNP in Genomic Sequence: SEQ ID NO: 471 /
SNP Position Genomic: 69878581 / Related Interrogated SNP: hCV11975250 / SNP
Source: dbSNP; HapMap / Population(Allele,Count): Caucasian (T,199|G,27) /
SNP Type: INTRON /
Context (SEQ ID NO: 3000): /
AATTGCTAACCAACTGCTCTGTGTTGCCTTTCTGTGGGCTCAGTTGATTGTCATTTTAATTTTCCTTCCATCAAATTAGTTAG
TACGTATTCTGTTCACT

R
AATATGGTATGTAAAGGTTGCCCCCTGTCCATTTACACTGGCAATTACAAAAATATTATTTATCAGTCCCTAATTGCGTGCCA
CTCTGATCTTATCTTTC / Celera SNP ID: hDV71028819 / Public SNP ID: rs4987340 /
SNP Chromosome Position: 169669143 / SNP in Genomic Sequence: SEQ ID NO: 471 /
SNP Position Genomic: 69880675 / Related Interrogated SNP: hCV11975250 / SNP
Source: dbSNP; HapMap / Population(Allele,Count): Caucasian (G,109|A,11) /
SNP Type: INTRON /
Context (SEQ ID NO: 3001): /
TATGCAAGAACTTCATTAGCTATGTATAACCTGAGAGCACTGAGCAGTTAAAATGTCTCTGCCCTTGCTATTAGAAATTCTGT
GCTGATTGAATCTCACT

R
TCTTATAGTATTATACAGTACTAAGTACTATACAGTACTAAGTATAAGTATCAAGTACTGCATTAAGGCAAAAGAAGAATGAC
AGTTTTCTCCAACCACT / Celera SNP ID: hDV71028821 / Public SNP ID: rs4987343 /
SNP Chromosome Position: 169668856 / SNP in Genomic Sequence: SEQ ID NO: 471 /
SNP Position Genomic: 69880962 / Related Interrogated SNP: hCV11975250 / SNP
Source: dbSNP; HapMap / Population(Allele,Count): Caucasian (A,109|G,11) /
SNP Type: INTRON /
Context (SEQ ID NO: 3002): /
TCTTTCTTCCTACCTTGGGCGATGATGGCAGAACTGACGGATAGAGAACAGGGTGGGTTATTACACACTGCCCCCTGGGATAA
GGTGGCTATTGTCTAAA

Y
AATTTTAAGGACTTTGGGGCTGTAAGTCACCTTAATAAGGAAGCTATTTATTTCCGAATTCTAAAATCTTTTAATAAGTGTTT
GTAGAATCAAAAGAATA / Celera SNP ID: hDV71028822 / Public SNP ID: rs4987345 /

SNP Chromosome Position: 169668608 / SNP in Genomic Sequence: SEQ ID NO: 471 / SNP Position Genomic: 69881210 / Related Interrogated SNP: hCV11975250 / SNP Source: dbSNP; HapMap / Population(Allele,Count): Caucasian (T,107|C,11) / SNP Type: INTRON /
Context (SEQ ID NO: 3003): /
CAAATGTTAGTATCAGGAGACAGATTTTTTTTTTATACCAGTATTTTCAAATAGAGGCTCCCAAATGAACTTAGTTTACACTTA
CAAGAGTATCTCTAAAC
W
TGCCCTTTTACTTATTCGTGAAGGAAGTAGAGGGAAGTGGTAACTACTGCATTGGAAAGAAATTAGTGCAGGTCCTTGGCATG
TAAAAATCAAGTGCTTA / Celera SNP ID: hDV71028828 / Public SNP ID: rs4987395 /
SNP Chromosome Position: 169671386 / SNP in Genomic Sequence: SEQ ID NO: 471 / SNP Position Genomic: 69878432 / Related Interrogated SNP: hCV11975250 / SNP Source: dbSNP; HapMap / Population(Allele,Count): Caucasian (T,109|A,11) / SNP Type: INTRON /
Context (SEQ ID NO: 3004): /
TAATCCCTAGTGGTCATGGCAGGATAACCTCAATCCAGTCCTCTTTGACCTGAAAATATCTTTATTGATATTCCATGCATGGC
TCTCAATTCAAAGTGTA
Y
AGTGGGTCAAGGCTTAGGTAGTAAAATGGATCATCCATTTGTCCTCAGAATAATTCTGGAGGGAGATATTGTCATCCATCCAG
TTTCCTCTCTATACCAA / Celera SNP ID: hCV30404194 / Public SNP ID: rs6691053 /
SNP Chromosome Position: 173868955 / SNP in Genomic Sequence: SEQ ID NO: 471 / SNP Position Genomic: 65680863 / Related Interrogated SNP: hCV16180170 / Related Interrogated SNP: hCV8911768 / SNP Source: dbSNP; HapMap / Population(Allele,Count): Caucasian (C,179|T,47) / SNP Type: PSEUDOGENE /
Context (SEQ ID NO: 3005): /
TATTTTTATTGAGTTTTTCTGAATATTTTTGATCTGCAGTTGGTTGAATCCGTGGATGCAGATGGTCAACTATGATTTTTTTC
TTGTTACTTCATATATC
R
TTGTAATCAGCCCAAAAGTAAAGTATAGAGAAGGCAGCAAAGTTAAAACATTTGAATGTATTTTAATTTTAATGAGAATGATT
TTAGAAAATATACAGAC / Celera SNP ID: hCV29644010 / Public SNP ID: rs7541082 /
SNP Chromosome Position: 173552529 / SNP in Genomic Sequence: SEQ ID NO: 471 / SNP Position Genomic: 65997289 / SNP Source: dbSNP; HapMap / Population(Allele,Count): Caucasian (A,85|G,33) / SNP Type: INTRON /
Context (SEQ ID NO: 3006): /
GGATTTAATTGGTAAATAAGAGACAGTGTTCTTAAACTCTTAAGACTTGGACAATTTCTGAGGAATAGTGGGACAGAAGTCAG
ATTGCAGTTGATTTAGC
R
TTAGTTGATTTAACTCCCAGCCCTCTGTGCATCTGGAGACAAAAGAGATGCTGGGTACAAATCCAAAGTATTAGGATAGATTT
TACTTTATTTTATGTAA / Celera SNP ID: hCV30205819 / Public SNP ID: rs7545388 /
SNP Chromosome Position: 174265423 / SNP in Genomic Sequence: SEQ ID NO: 471 / SNP Position Genomic: 65284395 / SNP Source: dbSNP; HapMap / Population(Allele,Count): Caucasian (G,201|A,25) / SNP Type: INTRON /
Context (SEQ ID NO: 3007): /
ATTGTTGCTATGGTTTTACCTTTTACAGATATCACATAATTGGAATCATACAGTATGTGGCCTTTTCATACTGGTATCTTTCA
CTTAGCAATGGTATACA
Y
TGAGATATATCTATGTCTTTTTGTGACTTGACAGCTATGTCTTTTCATTATTTCATTTTATACAATGTGCCATTGTATGGATA
TACCACTGTTTATCCAT / Celera SNP ID: hCV29989899 / Public SNP ID: rs6685043 /
SNP Chromosome Position: 173957513 / SNP in Genomic Sequence: SEQ ID NO: 471 / SNP Position Genomic: 65592305 / Related Interrogated SNP: hCV16180170 / SNP Source: dbSNP; HapMap / Population(Allele,Count): Caucasian (T,210|C,16) / SNP Type: INTRON /
Context (SEQ ID NO: 3008): /
TGGTAAAGGACTTAAATGCCATATGAGAAGTCTTCACACCCCAGCAGTCAGACCCATGAAGCTCCTCCCAGCCCTTCTATAAC
TTCTAGTGATTAAAGAC
Y
CCCTAATTGTCTGTTCCTTATTTTATTCTACAAAGCCAGCATCACCCTAATAACAAAACCTGGCAAAGACACAATGAATAATG
AAAACTACCGGCCAATA / Celera SNP ID: hCV30018856 / Public SNP ID: rs6701330 /
SNP Chromosome Position: 169501056 / SNP in Genomic Sequence: SEQ ID NO: 471 / SNP Position Genomic: 70048762 / Related Interrogated SNP: hCV8919444 / SNP Source: dbSNP; HapMap / Population(Allele,Count): Caucasian (T,66|C,36) / SNP Type: INTRON /
Context (SEQ ID NO: 3009): /

ACCCATATGTGGGCCCCTGCACAAGGAAGTACAAATGCAAGACTTCTTCCATCTTCAACCAACAGGAGTAAGACGGTGGACAC
CACCAGATGAGAGAGTT
K
ATGTCGTGGAGAAAGGAGGTTTGTCAGCTTCATGTTAACCACTGAAGAGATGTTACATGTTACATACATATATATATGTACAT
TTTTTTTTTTTTTTAGACA / Celera SNP ID: hCV30472885 / Public SNP ID: rs7520441 /
SNP Chromosome Position: 173392031 / SNP in Genomic Sequence: SEQ ID NO: 471 /
SNP Position Genomic: 66157787 / Related Interrogated SNP: hCV16180170 /
Related Interrogated SNP: hCV8911768 / SNP Source: dbSNP; HapMap /
Population(Allele,Count): Caucasian (G,117|T,3) / SNP Type: MISSENSE
MUTATION;ESS;INTRON /
Context (SEQ ID NO: 3010): /
GTCACCTGGCTGAGGTCTGGGGAAAGGGACATCTGACCAAAGTTTGGGGAAAGATCTGTCTCACCAAGGTCTGGAGAAAGTGT
CATCTGGTCGAGGTCTG
R
GGTAAGGGGAATTTGACTGAGATCTGCAAAGAGGGGCATCTCACTGAGGTCTGGGGAAAGGTTTGTCTGACTGAGTTCTGGAG
AGAGGTTTGTCTGGCTG / Celera SNP ID: hCV30504827 / Public SNP ID: rs9332608 /
SNP Chromosome Position: 169510118 / SNP in Genomic Sequence: SEQ ID NO: 471 /
SNP Position Genomic: 70039700 / Related Interrogated SNP: hCV8919444 / SNP
Source: dbSNP / Population(Allele,Count): Caucasian (G,215|A,11) / SNP Type:
MISSENSE MUTATION;ESE SYNONYMOUS /
Context (SEQ ID NO: 3011): /
TCATAGACCTTTAATGAAAAGGACACAAAATCACAGCTAGATAGGAGTAAGTTCTAGTGTTCTATAACACTGCAGGATGACTA
TAGTTAACAGCAATATA
Y
TATATAGTTTCACATAGCTAGAAGGAAGACATTGAATGTTCCCTACATGAAGAAATAATGTTTCAGAAGATAAATATGCTAAT
TACCCTGATCTGATCAT / Celera SNP ID: hCV30804131 / Public SNP ID: rs12090597 /
SNP Chromosome Position: 173563418 / SNP in Genomic Sequence: SEQ ID NO: 471 /
SNP Position Genomic: 65986400 / SNP Source: dbSNP; HapMap /
Population(Allele,Count): Caucasian (C,87|T,31) / SNP Type: INTRON /
Context (SEQ ID NO: 3012): /
CAGCCTCACCAGCATCTGTTGTTTGACTTTTTAATAACGGCCATTGTGACTGGTGTGAGATAGTATCTCGTTGTGATTTTGAT
TTGCATTTCCCTGATAG
Y
TTTTTCATATGATGGTTGGCCTCTTGTGTGTCTTGTTTTGAGAAGCGTCTGTTCATGTCTTTTGCCCATTTTCTAATGGGGTT
GTTTTTTTACTTGTTCAG / Celera SNP ID: hCV32141799 / Public SNP ID: rs12133074 /
SNP Chromosome Position: 169808651 / SNP in Genomic Sequence: SEQ ID NO: 471 /
SNP Position Genomic: 69741167 / Related Interrogated SNP: hCV11975250 / SNP
Source: dbSNP; HapMap / Population(Allele,Count): Caucasian (C,198|T,28) /
SNP Type: INTRON /
Context (SEQ ID NO: 3013): /
TGACAGTTAAGCTAACCAAAAGGTGATGTTTCCTTGACTCACCCTCCTGGAGTCTAAGCCTTAGTGGGCAGCTCCATGTCCCA
CCTGGTGCAAGCTGTAA
Y
AGATATAATCTGAGGGGTTCAGCTTCATATTCATGTACTAGTGCAGTGCTGATTACACAGCAGACTTGAAACAGTAGTAAATT
GTAGAGAAGAAGGAAAA / Celera SNP ID: hCV32141888 / Public SNP ID: rs12124561 /
SNP Chromosome Position: 169875679 / SNP in Genomic Sequence: SEQ ID NO: 471 /
SNP Position Genomic: 69674139 / Related Interrogated SNP: hCV11975250 / SNP
Source: dbSNP; HapMap / Population(Allele,Count): Caucasian (T,198|C,28) /
SNP Type: INTERGENIC;UNKNOWN /
Context (SEQ ID NO: 3014): /
TTTCAAACATTGCCAAGAGAACCAGTAAGTATCCCCTGGATGCAACAAGGGTGTCAGTGGGGAGACTATTTGCAGTGCAGACA
TCAAGACAGAGGTCAGA
Y
GGCCAAGCATTGAGCATTACTGAGGATGAGAAAGTAGAGACAGAACAAGCAGTCATCTCTTTTAAAATGTTTGGCTGTGAAGG
AGAAGTGGGGCAGGGGT / Celera SNP ID: hDV71070471 / Public SNP ID: rs4987363 /
SNP Chromosome Position: 169663938 / SNP in Genomic Sequence: SEQ ID NO: 471 /
SNP Position Genomic: 69885880 / Related Interrogated SNP: hCV11975250 / SNP
Source: CDX; dbSNP; HapMap / Population(Allele,Count): Caucasian (C,199|T,27)
/ SNP Type: INTRON //

Gene Number: 135 / Gene Symbol: LOC100506975 - 100506975 / Gene Name:
hypothetical protein LOC100506975 / Chromosome: 1 / OMIM NUMBER: / OMIM
Information: // Genomic Sequence (SEQ ID NO: 472): // / SNP Information /

Context (SEQ ID NO: 3015): /
TCAATTGTGTCTGAACAGAAAGTATAATTAACAACGAGTGAAGGAAAGCATAAACATGAAATCATCTCAGTCTTATTTGTTTT
ATAGGAGTCAGTTTTAA
R
TGCTCAATTTGAAAGCCAATGTCATATCGTACTATAGTTCCTAAGTTAATTAGAATAGCAAACATTGTAAAACAAAATAAATC
AGAAGCAGAAATGACAC / Celera SNP ID: hCV804146 / Public SNP ID: rs320308 / SNP
Chromosome Position: 243780198 / SNP in Genomic Sequence: SEQ ID NO: 472 /
SNP Position Genomic: 1768 / Related Interrogated SNP: hCV31523608 / SNP
Source: dbSNP; HapMap; HGBASE / Population(Allele,Count): Caucasian
(G,206|A,20) / SNP Type: INTRON /
Context (SEQ ID NO: 3016): /
AACCCCAAGAGAGAAGTGAGGATGGCTTGGACTACTATGGTGGCAGCGGAAAAAGAAAAAGGGGATAGTTCTGAGATATAAAG
TTAACAGTGCTAATATA
R
AAGTTACATGCCTCCAACATGAGAGTGAATCACAGACATTTTAATAGGCAGTTCAGAAAGAAATTCACAGAAGATAACATCTA
CCTGTTGTGGTTTAAGA / Celera SNP ID: hCV804147 / Public SNP ID: rs320309 / SNP
Chromosome Position: 243781174 / SNP in Genomic Sequence: SEQ ID NO: 472 /
SNP Position Genomic: 2744 / Related Interrogated SNP: hCV31523608 / SNP
Source: dbSNP; HapMap; ABI_Val; HGBASE / Population(Allele,Count): Caucasian
(A,202|G,24) / SNP Type: INTRON /
Context (SEQ ID NO: 3017): /
ATAATATACTACACTGATGACATTATGACGGTATCAGAAACCTAAGAACAAGCTAGGGCTAACTTGAAAATAGTGGTGGCTCA
CAGGAATACCACAGGCT
R
GAGGATAAATCCAGCAAATACCCCAGGGTCTAACCAAACTGTGAAATTCTTACGAATAACCTGGGCAGGAACCACCCATAACA
TTCTACAGGCAACTAAA / Celera SNP ID: hCV804156 / Public SNP ID: rs320316 / SNP
Chromosome Position: 243790947 / SNP in Genomic Sequence: SEQ ID NO: 472 /
SNP Position Genomic: 12517 / Related Interrogated SNP: hCV31523608 / SNP
Source: dbSNP; HapMap; HGBASE / Population(Allele,Count): Caucasian
(G,24|A,202) / SNP Type: INTRON /
Context (SEQ ID NO: 3018): /
TTAAAAGTAGATTAACAGCTACTATTAAAAGTAAATTAACAACAATAATAAAATAGAAAACTTATAACAATATATTGTGATAC
AAGTTATTGTGATGCAT
R
TGGTCTCTCTCTCAAAAATATCTTATTTTACTATACTCATCTATATTTAGGCTGTGGTTGATTGGGGGTAACTCAAACCATGAA
AAGCAAAACTGCAGCTA / Celera SNP ID: hCV804160 / Public SNP ID: rs320331 / SNP
Chromosome Position: 243797817 / SNP in Genomic Sequence: SEQ ID NO: 472 /
SNP Position Genomic: 19387 / Related Interrogated SNP: hCV31523608 / SNP
Source: dbSNP; Celera; HapMap; HGBASE / Population(Allele,Count): Caucasian
(A,19|G,101) / SNP Type: INTRON /
Context (SEQ ID NO: 3019): /
AGTTGCCATCATAAGTAGGACTCATTAAAGCATGGCAAAAACATTGTTTCAAAAAAGGAAGAATGACAGGGCAGACAATGACA
AACTCTGATGCCTAAAT
R
GGTATACAGTCACCAATGGCATGAGACGGCAGCTGTACTGTGCTGTTACTACATATAGAAGTTGTCAATGGAATTTAGAAATG
ACAGACCAAAATCTCAA / Celera SNP ID: hCV26719149 / Public SNP ID: rs6675851 /
SNP Chromosome Position: 243787494 / SNP in Genomic Sequence: SEQ ID NO: 472 /
 SNP Position Genomic: 9064 / Related Interrogated SNP: hCV30690780 / Related
Interrogated SNP: hCV31523650 / Related Interrogated SNP: hCV233148 / Related
Interrogated SNP: hCV31523608 / SNP Source: dbSNP; Celera; HapMap /
Population(Allele,Count): Caucasian (G,189|A,37) / SNP Type: INTRON /
Context (SEQ ID NO: 3020): /
GTGGACTGGGTAAAAGCCACTGTAAAATTTGTTTACCCTGAAAAGGGAAATGGTCCTTCCCCTCCTTTAAATGCCAAGTGGCA
CTCCTCCATAGATGACA
W
AGCTGGTAAGCTGCATATGCAAACCATGCTGGATTAACTTCATGGTCATCAGGATGTTTGTTCATCCTTAATATGCCCTTTAA
ATCATGGTAAATACTGT / Celera SNP ID: hCV29795761 / Public SNP ID: rs7528450 /
SNP Chromosome Position: 243789305 / SNP in Genomic Sequence: SEQ ID NO: 472 /
 SNP Position Genomic: 10875 / Related Interrogated SNP: hCV31523608 /
Related Interrogated SNP: hCV30690780 / SNP Source: dbSNP; HapMap /
Population(Allele,Count): Caucasian (T,33|A,77) / SNP Type: TRANSCRIPTION
FACTOR BINDING SITE;UTR5;INTRON /
Context (SEQ ID NO: 3021): /

CCATTGGAATCTCCCTAAAAACTCAGCTGAATCTTCTGAGGCCTCTTTCATCCATCGCTACTTAGGTCACTCTTCAATTTCTC
AGGATGGTTGTTTCAAA
W
CTTTCCCAGGCCTTCTTCCTTGCCTGGTAGGTATACTTAAATGTACACACTAACCCTTACCTAGTCAAGCTACTAAGCTAAGA
CTAGAACCCAGGTCTGT / Celera SNP ID: hCV31523639 / Public SNP ID: rs12034588 /
SNP Chromosome Position: 243784392 / SNP in Genomic Sequence: SEQ ID NO: 472 /
SNP Position Genomic: 5962 / Related Interrogated SNP: hCV30690780 / Related
Interrogated SNP: hCV31523650 / Related Interrogated SNP: hCV233148 / Related
Interrogated SNP: hCV31523608 / SNP Source: dbSNP; HapMap /
Population(Allele,Count): Caucasian (T,99|A,17) / SNP Type: TRANSCRIPTION
FACTOR BINDING SITE;INTRON /
Context (SEQ ID NO: 3022): /
AGTTCCAAATATTCCTTAGATGAAGGCCAAATGAACTCCTTTGACTTCCATCATGATATAATCACTTTGGACTCCAGTCTTTC
TTCTGCCTCTCTCATAC
R
CGGTATTGGGACAAGTTCATCTCTACCCTCTTAACTCTATATTTCACATCTAAAGATAAATTTACACAAACATATTGACTAAC
ACCATTGGAATCTCCCT / Celera SNP ID: hCV31523638 / Public SNP ID: rs12037013 /
SNP Chromosome Position: 243784207 / SNP in Genomic Sequence: SEQ ID NO: 472 /
SNP Position Genomic: 5777 / Related Interrogated SNP: hCV30690780 / Related
Interrogated SNP: hCV31523650 / Related Interrogated SNP: hCV233148 / Related
Interrogated SNP: hCV31523608 / SNP Source: dbSNP; HapMap /
Population(Allele,Count): Caucasian (G,194|A,32) / SNP Type: INTRON /
Context (SEQ ID NO: 3023): /
GCAGGCTATTATCTAAGTTGCAGTGGCTCAATTAATGCCTTTGGGTCCTTACGATATACACTCAGATATGATTTTAGAAGTAT
CTGCAACTCAGACTGGA
S
CTTATGGCAAAAAGCCCATGAATGCTGTCCAGCAGTAACCACTGGGACCATGGGCCAGACAAGAAAGTATAGAACACATCATT
AGAGAGACGATTATTGG / Celera SNP ID: hCV31523552 / Public SNP ID: rs12739344 /
SNP Chromosome Position: 243791312 / SNP in Genomic Sequence: SEQ ID NO: 472 /
SNP Position Genomic: 12882 / Related Interrogated SNP: hCV31523608 /
Related Interrogated SNP: hCV30690780 / Related Interrogated SNP: hCV31523650
/ SNP Source: dbSNP; HapMap / Population(Allele,Count): Caucasian
(G,63|C,161) / SNP Type: INTRON /
Context (SEQ ID NO: 3024): /
GGACATGTTGATGGTCATCAAAAGAACTTCTTTCAGGATCAGGAGATGACTGGAACCAGCAAGTGGATGTCCCAGCATGCTTG
TTTGAGGTGGCTATCTG
R
GTCCCCAAAATAAGTAGATATGGGAGATATTGGCCGGGCACAGTGGCTCATGCCTGTAATCCCAGCACTTTGGGAGGCCAAGG
CGGGTGGATCATGAGGT / Celera SNP ID: hCV31523555 / Public SNP ID: rs12749316 /
SNP Chromosome Position: 243792781 / SNP in Genomic Sequence: SEQ ID NO: 472 /
SNP Position Genomic: 14351 / Related Interrogated SNP: hCV31523608 /
Related Interrogated SNP: hCV30690780 / SNP Source: dbSNP; HapMap /
Population(Allele,Count): Caucasian (G,37|A,83) / SNP Type: INTRON /
Context (SEQ ID NO: 3025): /
CTGAAAAGGGAAATGGTCCTTCCCCTCCTTTAAATGCCAAGTGGCACTCCTCCATAGATGACATAGCTGGTAAGCTGCATATG
CAAACCATGCTGGATTA
R
CTTCATGGTCATCAGGATGTTTGTTCATCCTTAATATGCCCTTTAAATCATGGTAAATACTGTGACTGAAGGGGCCCCGCTAG
GCAGGCATCTGATATAA / Celera SNP ID: hCV31523643 / Public SNP ID: rs6671475 /
SNP Chromosome Position: 243789342 / SNP in Genomic Sequence: SEQ ID NO: 472 /
SNP Position Genomic: 10912 / Related Interrogated SNP: hCV30690780 /
Related Interrogated SNP: hCV31523650 / Related Interrogated SNP: hCV233148 /
Related Interrogated SNP: hCV31523608 / SNP Source: dbSNP; HapMap /
Population(Allele,Count): Caucasian (A,186|G,40) / SNP Type: TRANSCRIPTION
FACTOR BINDING SITE;UTR5;INTRON //

Gene Number: 136 / Gene Symbol: ENSG00000213062 / Gene Name: / Chromosome:
1 / OMIM NUMBER: / OMIM Information: // Genomic Sequence (SEQ ID NO: 473): //
/ SNP Information /
Context (SEQ ID NO: 3026): /
AACACCTAACTACGCTACGGTTTATAGTTAAAAGGAAGGTGGAAGATCCTGAGTCATTACAGCAATCACAAAACATGATGGAT
TTCTTCATTTAAAAACA
M

AAAAGAGGTCTTGGCATAGTTTTCAAGGGCAAAGCATGACAAGACTCTCCAGTGCTTTCTTTCATCCCCCTCTGTGTGCCTTT
TTCTGCCTGACTCACAG / Celera SNP ID: hCV11975194 / Public SNP ID: rs2038024 /
SNP Chromosome Position: 169455982 / SNP in Genomic Sequence: SEQ ID NO: 473 /
SNP Position Genomic: 602 / Related Interrogated SNP: hCV11975250 / SNP
Source: dbSNP; Celera; HapMap; ABI_Val; HGBASE / Population(Allele,Count):
Caucasian (C,40|A,186) / SNP Type: TRANSCRIPTION FACTOR BINDING
SITE;UTR3;PSEUDOGENE /
Context (SEQ ID NO: 3027): /
ATCACAAACATGATGGATTTCTTCATTTAAAAACACAAAGAGGTCTTGGCATAGTTTTCAAGGGCAAAGCATGACAAGACT
CTCCAGTGCTTTCTTTC
R
TCCCCCTCTGTGTGCCTTTTTTCTGCCTGACTCACAGCCTGGCTCAATCCTAATCTTTCTACTGCAAAGATGCAGCATTTAAAG
CAATTTTTCCTGTGCAG / Celera SNP ID: hCV27242742 / Public SNP ID: rs12408451 /
SNP Chromosome Position: 169456046 / SNP in Genomic Sequence: SEQ ID NO: 473 /
SNP Position Genomic: 666 / Related Interrogated SNP: hCV11975250 / SNP
Source: dbSNP; Celera / Population(Allele,Count): Caucasian (A,67|G,159) /
SNP Type: UTR3;PSEUDOGENE //

Gene Number: 137 / Gene Symbol: Chr1:244003667..244043667 / Gene Name: /
Chromosome: 1 / OMIM NUMBER: / OMIM Information: // Genomic Sequence (SEQ ID
NO: 474): // / SNP Information /
Context (SEQ ID NO: 3028): /
CTGTCCAGACAAATTGCTCCCATGCAAGTCACTCTCCCCACTGCCTTCCCTCCAGAGCATCATCACAATCCCAGGCAGATAGG
AACTTTCCAACCACGCA
R
CAAAATGGAGGCCCAAAATAGAAATGGAGTTGAGTTCTAGAAATAATGTTACATTTCCTACACACCTGAATTTGGGAAATGAA
TGATAGCCAGAAAGGGA / Celera SNP ID: hCV15755277 / Public SNP ID: rs3008657 /
SNP Chromosome Position: 244023667 / SNP in Genomic Sequence: SEQ ID NO: 474 /
SNP Position Genomic: 20000 / Related Interrogated SNP: hCV31523608 /
Related Interrogated SNP: hCV30690780 / SNP Source: dbSNP; HapMap; ABI_Val;
HGBASE / Population(Allele,Count): Caucasian (G,67|A,157) / SNP Type:
INTERGENIC;UNKNOWN /
Context (SEQ ID NO: 3029): /
AAAAATAGGTCACATACTAAGGAATGATTCTGAAGGATATGGGAGGAAAATCAACAGAAAGGGATACACCTTGAAGGGAGGGT
GTACACCTCTGGAACAC
K
AGTGAATGTGAAAAGGATGTGCGTAGATAGAGTTCTGTAGAGGGCTGGGAGAAATGTGACAGGTTTCATGCTTCATGGCATGG
AGGAGGAAGGGCCACGT / Celera SNP ID: hCV15953062 / Public SNP ID: rs2953330 /
SNP Chromosome Position: 244024121 / SNP in Genomic Sequence: SEQ ID NO: 474 /
SNP Position Genomic: 20454 / Related Interrogated SNP: hCV31523608 /
Related Interrogated SNP: hCV30690780 / SNP Source: dbSNP; Celera; HapMap;
HGBASE / Population(Allele,Count): Caucasian (G,74|T,152) / SNP Type:
INTERGENIC;UNKNOWN /
Context (SEQ ID NO: 3030): /
GGGAGAAATGTGACAGGTTTCATGCTTCATGGCATGGAGGAGGAAGGGCCACGTGGTAATGCTTGTTGGATGCCTGTTGGAAA
TAATGTAGGATGAAGAC
R
GAACCATAACACAGTTTACTTCCTTGAGCGTTAGGTGGTATATGTATTGCATTCGACTTCCTTCATTGAAGTTGTGGATGCTG
CAGTCACTGCCGGGATC / Celera SNP ID: hCV15953063 / Public SNP ID: rs2953331 /
SNP Chromosome Position: 244024268 / SNP in Genomic Sequence: SEQ ID NO: 474 /
SNP Position Genomic: 20601 / Related Interrogated SNP: hCV31523608 /
Related Interrogated SNP: hCV30690780 / SNP Source: dbSNP; Celera; HapMap;
HGBASE / Population(Allele,Count): Caucasian (A,68|G,154) / SNP Type:
INTERGENIC;UNKNOWN /
Context (SEQ ID NO: 3031): /
AAGGATGGCACTGAAATTTTTCTGTAGCAAGAAGTTAGGTATGATTTGGTTTGTTTTGAGAATAAACAAGATTAAGAAAAATT
TACATTTTAGACCAGTC
R
AGTACTTTAGGTGTCATCTTTTACAGATTTCACATTTAATATAATTCTACTCCTGACATTAAATTTTATTACTCTACAAACTA
GCCAGAAGTAATAAGGT / Celera SNP ID: hCV15953071 / Public SNP ID: rs2953329 /
SNP Chromosome Position: 244025999 / SNP in Genomic Sequence: SEQ ID NO: 474 /
SNP Position Genomic: 22332 / Related Interrogated SNP: hCV31523608 /
Related Interrogated SNP: hCV233148 / SNP Source: dbSNP; HapMap; HGBASE /

Population(Allele,Count):   Caucasian (G,84|A,140) /   SNP Type:
INTERGENIC;UNKNOWN /
Context                (SEQ ID NO: 3032): /
GAGTGGTCAGCAAGCTAAGATTCCTTTACCATAAATGTGAATATATTTATGTTAAACTAACAATAGCTGCTACAAACAAATAC
AAAAATCTATATTGGCA
Y
TCTCTGCCACAGTTGGTTCTGAAATAACCACGTTAAGTCTACAGCTGTAATTAGTGAATATATTCTAGATGATTAATAGAACA
ATTTCTAAAGGAGTATA / Celera SNP ID:   hCV27170898 /   Public SNP ID:   rs12753750 /
SNP Chromosome Position:   244017365 /   SNP in Genomic Sequence:   SEQ ID NO: 474 /
 SNP Position Genomic:   13698 /   Related Interrogated SNP:   hCV31523608 /
Related Interrogated SNP:   hCV30690780 /   Related Interrogated SNP:   hCV31523650
/   SNP Source:   dbSNP; Celera; HapMap /   Population(Allele,Count):   Caucasian
(C,67|T,159) /   SNP Type:   INTERGENIC;UNKNOWN /
Context                (SEQ ID NO: 3033): /
GTAAATACATAACCTAAACCAATGAGTTTCACTCATTTGTCAGCTTCAACCACTGACAAAACCCAGTGGTTGCCTAGTGATGT
ATAGTTCTACTAAAAGA
Y
ATATATGACCATGCTTTACCAAATACGGAACACATGTCTAAACTGTTGAGACAACTGCCAATGTATTAACTACAAAAAGCCAT
TATTTTTTACTTTAGCC / Celera SNP ID:   hCV27171311 /   Public SNP ID:   rs4322213 /
SNP Chromosome Position:   244011942 /   SNP in Genomic Sequence:   SEQ ID NO: 474 /
 SNP Position Genomic:   8275 /   Related Interrogated SNP:   hCV31523608 /   SNP
Source:   dbSNP; Celera; HapMap; ABI_Val; HGBASE /   Population(Allele,Count):
Caucasian (C,24|T,202) /   SNP Type:   INTRON /
Context                (SEQ ID NO: 3034): /
TTTATTTAAATAACGTTGTTCTTTCTCTTAGACTATGCCGGTATGAATTTGGTAGAATCTGCCGATTGTGGTATGTCGGTACT
CCATACAGAGTGTGTGG
Y
AGATTTCTTCTCTATTTTGTTATCACATCCAGTAATATGGAGTGCAGCCAGCATGTTCATCCCTGGGAATGTCCCTCCTAAAT
TTTGCAAAGAAAGCCTT / Celera SNP ID:   hCV27171350 /   Public SNP ID:   rs4430311 /
SNP Chromosome Position:   244015993 /   SNP in Genomic Sequence:   SEQ ID NO: 474 /
 SNP Position Genomic:   12326 /   Related Interrogated SNP:   hCV31523608 /
Related Interrogated SNP:   hCV30690780 /   Related Interrogated SNP:   hCV31523650
/   SNP Source:   dbSNP; Celera; HapMap; HGBASE /   Population(Allele,Count):
Caucasian (C,68|T,158) /   SNP Type:   INTERGENIC;UNKNOWN /
Context                (SEQ ID NO: 3035): /
CAAAGGAGGTAGAAAAGATAGGGAGATTTTTATTTTTCATTTTATACCTTTGTGTGTTATTCAAATTTTTATGTTTATGCAAC
TATTCATCTCAAAATAA
R
CACATACACATTATTAGAAACACATACACATACACATCTCAAAATAAACACATACACATTATTAGAAAAATGATGGCAAATTC
TTATTCATTTTTTTTTC / Celera SNP ID:   hCV30228080 /   Public SNP ID:   rs4593807 /
SNP Chromosome Position:   244004392 /   SNP in Genomic Sequence:   SEQ ID NO: 474 /
 SNP Position Genomic:   725 /   Related Interrogated SNP:   hCV31523608 /   SNP
Source:   dbSNP; HGBASE /   Population(Allele,Count):   Caucasian (G,18|A,98) /   SNP
Type:   INTRON //

Gene Number:   138 /   Gene Symbol:   Chr11:129087579..129107579 /   Gene Name: /
Chromosome:   11 /   OMIM NUMBER: /   OMIM Information: //   Genomic Sequence (SEQ ID
NO: 475): //   / SNP Information /
Context                (SEQ ID NO: 3036): /
TTTCATGAAAACAATACCAAAAGGAGAGATGAAAATGGCTACACTAACATCTAACAAAATAAGACTTTAAGAAAAAAACTGTT
ACTAGAGACAAGGGATA
Y
TTTAGAATGATAGAGGTAAATTTGTAGCAGACATCCAACAACAGACACCCAAAATACATGAAACAAAACTGACAGATTTCAGA
GGACAAATAGACAATGC / Celera SNP ID:   hCV30922162 /   Public SNP ID:   rs4334028 /
SNP Chromosome Position:   129097579 /   SNP in Genomic Sequence:   SEQ ID NO: 475 /
 SNP Position Genomic:   10000 /   SNP Source:   dbSNP; HapMap; ABI_Val /
Population(Allele,Count):   Caucasian (C,166|T,58) /   SNP Type:   INTRON //

Gene Number:   139 /   Gene Symbol:   Chr12:71447204..71467204 /   Gene Name: /
Chromosome:   12 /   OMIM NUMBER: /   OMIM Information: //   Genomic Sequence (SEQ ID
NO: 476): //   / SNP Information /
Context                (SEQ ID NO: 3037): /
ACAGATTCAGAAGGAAGGACGGTAAAGGGAAAACAAAAGACAGCCTCTGAACAATCTAAAACAAGGACTCAAATTTCATGCAC

TTTTTCCAGTTTGAACC
K
CTGAAGCCTTCCCTAAGACTGTTTACTTTTCCTTATATAGAAATTTAACAACACTAATAATATTGTTGCCAACCCATAATAAG
TTAGAAAAAAAGAAAGG / Celera SNP ID: hCV2690378 / Public SNP ID: rs11178579 /
SNP Chromosome Position: 71457204 / SNP in Genomic Sequence: SEQ ID NO: 476 /
SNP Position Genomic: 10000 / SNP Source: dbSNP; Celera; HapMap /
Population(Allele,Count): Caucasian (G,68|T,40) / SNP Type: INTRON //

Gene Number: 140 / Gene Symbol: Chr12:133456559..133496559 / Gene Name: /
Chromosome: 12 / OMIM NUMBER: / OMIM Information: // Genomic Sequence (SEQ ID
NO: 477): // / SNP Information /
Context (SEQ ID NO: 3038): /
CAGCTCAGGTCATGCAGTAATTTGGTGGGCCATGCTTAATTATTCAGTCCCTAATACCGTCCTGTAGCAAGCCAAAAGCTGGT
TGTCAAAAGAAGAGTAC
R
GGTGGAAGAGAGCGCTTCTCAGACCTCGGCTGCGCGGCCCTAGGAGTGCCGAGCTCACGGAACCACAGCCATGACTGAGGCCA
ATGTGAATCTGAAGGCC / Celera SNP ID: hCV1859996 / Public SNP ID: rs12815354 /
SNP Chromosome Position: 133476501 / SNP in Genomic Sequence: SEQ ID NO: 477 /
SNP Position Genomic: 19942 / Related Interrogated SNP: hCV1859855 / SNP
Source: dbSNP; Celera; HapMap / Population(Allele,Count): Caucasian
(G,79|A,39) / SNP Type: TFBS SYNONYMOUS;INTRON /
Context (SEQ ID NO: 3039): /
GTCCTGTAGCAAGCCAAAAGCTGGTTGTCAAAAGAAGAGTACGGGTGGAAGAGAGCGCTTCTCAGACCTCGGCTGCGCGGCCC
TAGGAGTGCCGAGCTCA
Y
GGAACCACAGCCATGACTGAGGCCAATGTGAATCTGAAGGCCTAGCCCCTCGCCGATGCCCACCTCACCAAGAAACCACTGGA
GTTCATTCAGTAGTCGT / Celera SNP ID: hCV1859997 / Public SNP ID: rs12815207 /
SNP Chromosome Position: 133476559 / SNP in Genomic Sequence: SEQ ID NO: 477 /
SNP Position Genomic: 20000 / Related Interrogated SNP: hCV1859855 / SNP
Source: dbSNP; Celera; HapMap / Population(Allele,Count): Caucasian
(C,80|T,36) / SNP Type: INTRON;PSEUDOGENE /
Context (SEQ ID NO: 3040): /
GCGGGATGAACCAGCCCCTGTGGAAATTTAGAAGTAACGACGTCCGTCAATTTCTGTATGAAAAATATACAGATCGTGTGACA
CGTCTTCTGGGAATGAA
R
GTCTGCTAAACTCGCCGTCACGAGTAGATGAACACATTTAAAGTTTGTAGTTAAGAGGAAAACAACGCCAACGACACAAATGC
CCCAAGCGCTGGATGAA / Celera SNP ID: hCV16089184 / Public SNP ID: rs2873193 /
SNP Chromosome Position: 133484722 / SNP in Genomic Sequence: SEQ ID NO: 477 /
SNP Position Genomic: 28163 / Related Interrogated SNP: hCV1859855 / SNP
Source: dbSNP; Celera; HGBASE / Population(Allele,Count): Caucasian
(A,150|G,76) / SNP Type: INTRON //

Gene Number: 141 / Gene Symbol: Chr13:42898648..42938648 / Gene Name: /
Chromosome: 13 / OMIM NUMBER: / OMIM Information: // Genomic Sequence (SEQ ID
NO: 478): // / SNP Information /
Context (SEQ ID NO: 3041): /
CTGGCTGAGGTAGTCTTGACAGGTTTTTCTACTGTAAAGTGATTCTTTTTTCCACCTTTTCATACTTTACTCTTTGGAGGGGA
GTCACTGTGGATAGCCC
K
CACTTAAGGAGTGGGAGTTGTACTCCACCTCCCTGAGGGGGAGTAGCTACATACATGATGGAATTCTTCTGTCTAGAGATTTA
TCTATTCTCCCCCACTT / Celera SNP ID: hCV931685 / Public SNP ID: rs238250 / SNP
Chromosome Position: 42914646 / SNP in Genomic Sequence: SEQ ID NO: 478 / SNP
Position Genomic: 15998 / SNP Source: dbSNP; HapMap; HGBASE /
Population(Allele,Count): Caucasian (G,196|T,30) / SNP Type: INTRON /
Context (SEQ ID NO: 3042): /
GTAATCTTTTTTGAACAGTTATTCAAATATTGTGCCAGACAGTGTGAGGGTGGTGGGGCAAATAATGGAAAGTGTTCTTGTTC
CCCTGGGACCTACATTT
Y
GAGGGAGAGGTAGATAGGCATTTTCATAATCTTCATTTTCTTTGCATTCTTTATTTGTGTTTACCTTTCTATTGAGTGGACTG
TTAAGGAAGCACCAGAG / Celera SNP ID: hCV29883689 / Public SNP ID: rs9525613 /
SNP Chromosome Position: 42918648 / SNP in Genomic Sequence: SEQ ID NO: 478 /
SNP Position Genomic: 20000 / SNP Source: dbSNP; HapMap /
Population(Allele,Count): Caucasian (C,174|T,52) / SNP Type: MISSENSE

MUTATION;ESE;ESS SYNONYMOUS //

Gene Number: 142 / Gene Symbol: Chr14:101790124..101810124 / Gene Name: /
Chromosome: 14 / OMIM NUMBER: / OMIM Information: // Genomic Sequence (SEQ ID
NO: 479): // / SNP Information /
Context (SEQ ID NO: 3043): /
AGCCCCACTTCCCATCCAGCACACACTGCTGGCACCCCCACTCAGCCCCCGGCACTGCCCCTGAATTTCTCTCATAGCTAGCT
AGCTCTCCATTGGCAAC
R
CTGGTGCCTGTCCCTGCCAGCAGCCCCAAGTCAGCACCATGCCCGGCTGTTCATTATTAAATTGAAAAAAGGAGGAGGGTCCT
GCCTTGATTCCCTTCCA / Celera SNP ID: hCV1772768 / Public SNP ID: rs945020 /
SNP Chromosome Position: 101800124 / SNP in Genomic Sequence: SEQ ID NO: 479 /
SNP Position Genomic: 10000 / SNP Source: dbSNP; Celera; HapMap; ABI_Val;
HGBASE / Population(Allele,Count): Caucasian (G,141|A,85) / SNP Type:
INTERGENIC;UNKNOWN //

Gene Number: 143 / Gene Symbol: Chr15:62727452..62747452 / Gene Name: /
Chromosome: 15 / OMIM NUMBER: / OMIM Information: // Genomic Sequence (SEQ ID
NO: 480): // / SNP Information /
Context (SEQ ID NO: 3044): /
GAAATGCCCAGGCTGCAGCACCTGCAGGTGTGACCCAGCTGACTCCAGGCCTTCAGAGCAGTCGTTCTTATCCCTGCCAGTGG
ATAGGAATCACCTGTGG
Y
TTTACAGTGACCTCCACCTCCACCTTTGGAGGTCTTGAATCTGTATTAGCAAATAACAACTCCCTTTCTCCTGCCCCCAAGCA
ACTCTAATGTATATGCT / Celera SNP ID: hCV7543812 / Public SNP ID: rs1068705 /
SNP Chromosome Position: 62737452 / SNP in Genomic Sequence: SEQ ID NO: 480 /
SNP Position Genomic: 10000 / SNP Source: dbSNP; HapMap; HGBASE /
Population(Allele,Count): Caucasian (C,116|T,110) / SNP Type:
INTERGENIC;UNKNOWN //

Gene Number: 144 / Gene Symbol: Chr16:49340689..49360689 / Gene Name: /
Chromosome: 16 / OMIM NUMBER: / OMIM Information: // Genomic Sequence (SEQ ID
NO: 481): // / SNP Information /
Context (SEQ ID NO: 3045): /
ATAAATTCAGTCGAATTGAAGCTTATATTGGGAAGAAAACAGCCGGCTACTAGCTGTAATATAGACGTTCATGGCCATATGTA
AAGTGCAGCCATGTTAC
W
CAAGGATCCATTTAATTTGTTCTGGGCTCTGATGTCACCATGCACCCACTGAGGTGTTTTTAAAACACATTGAGATCATACAT
ATAGTTAAAAACAATCA / Celera SNP ID: hCV11778561 / Public SNP ID: rs2883894 /
SNP Chromosome Position: 49350689 / SNP in Genomic Sequence: SEQ ID NO: 481 /
SNP Position Genomic: 10000 / SNP Source: dbSNP; Celera; HapMap; ABI_Val;
HGBASE / Population(Allele,Count): Caucasian (T,56|A,64) / SNP Type:
INTERGENIC;UNKNOWN //

Gene Number: 145 / Gene Symbol: Chr17:64261724..64281724 / Gene Name: /
Chromosome: 17 / OMIM NUMBER: / OMIM Information: // Genomic Sequence (SEQ ID
NO: 482): // / SNP Information /
Context (SEQ ID NO: 3046): /
AAATAAAATAAAAATAATAAAAATAAGAAATAAATATATCACCTCGTTAGTCCTTACAACAAAGCCAATAGCCTTTGTTCACA
TAAGTGGAATCCCACTC
Y
CATTTCATAGCTAAGAAAACTGAGGCTCAGAAGAGAATAGGATTTACCCAAAGTCTCAGGAATTCATCTGAGCCCTATGGACC
ACCCCACAGCAGTGCAG / Celera SNP ID: hCV3286482 / Public SNP ID: rs6504406 /
SNP Chromosome Position: 64271724 / SNP in Genomic Sequence: SEQ ID NO: 482 /
SNP Position Genomic: 10000 / SNP Source: dbSNP; HapMap /
Population(Allele,Count): Caucasian (T,186|C,40) / SNP Type:
INTERGENIC;UNKNOWN //

Gene Number: 146 / Gene Symbol: Chr2:57355335..57375335 / Gene Name: /
Chromosome: 2 / OMIM NUMBER: / OMIM Information: // Genomic Sequence (SEQ ID
NO: 483): // / SNP Information /
Context (SEQ ID NO: 3047): /
CACTGCACTCTAGCCTGGGCGACAGAGTGAGACTCTGTCTCAAAAAAAGCCAAAAAACAGAAACAAAAGTAAAAGCACTCATT

TATTATTCAAAACATGT
K
CAAGAGTCCTGGTTCAAAAATACCATGAAGTTGGTGCAAAGAGAAGTTTAAAGTGTTGAAGTGAAGCTTGAAATAATAAAATT
TTTTAAAAAAGATAGAA / Celera SNP ID: hDV70830411 / Public SNP ID: rs17048681 /
SNP Chromosome Position: 57365335 / SNP in Genomic Sequence: SEQ ID NO: 483 /
SNP Position Genomic: 10000 / SNP Source: dbSNP; HapMap /
Population(Allele,Count): Caucasian (G,78|T,148) / SNP Type:
INTERGENIC;UNKNOWN //

Gene Number: 147 / Gene Symbol: Chr2:73848120..73868120 / Gene Name: /
Chromosome: 2 / OMIM NUMBER: / OMIM Information: // Genomic Sequence (SEQ ID
NO: 484): // / SNP Information /
Context (SEQ ID NO: 3048): /
CTCTCCTGAAGATAATCAGCCATTGAAGCGGCTCTGAGGGATTTCACAGTATATTGCTCCTCATAATACCCTTAAAAGAATATG
GAGAAACAGAGGTTACA
R
TAACTGGGGACTCATGTATAGTCTTGGTGGATACCAGAGCTACCTTATCTGCCACTACCATAAAACACACTTTCATAAGCCAA
CAGATCCCTCAGAGTTA / Celera SNP ID: hDV68778390 / Public SNP ID: rs71337715 /
SNP Chromosome Position: 73858120 / SNP in Genomic Sequence: SEQ ID NO: 484 /
SNP Position Genomic: 10000 / Related Interrogated SNP: hCV11541681 / SNP
Source: dbSNP; HapMap / Population(Allele,Count): Caucasian (G,77|A,35) / SNP
Type: INTERGENIC;UNKNOWN //

Gene Number: 148 / Gene Symbol: Chr2:220537189..220590407 / Gene Name: /
Chromosome: 2 / OMIM NUMBER: / OMIM Information: // Genomic Sequence (SEQ ID
NO: 485): // / SNP Information /
Context (SEQ ID NO: 3049): /
CAACCTTATGGATGTGTTTTGTAAAAGTCTCATTTAGAACCTTTGGGGCCTCACTCAGTCACCCCTGAATCCTCATCTTTTCA
TGTACACACCTCTGTCC
R
TTTTCTCCTCTTTTCTTCCTAATTAGCTGCCTGTATAATTAAACCTTTCCTCATTTAGAGGTTCTGAGCCCTCACTGATACAC
TAATCACCCTGTGGCCC / Celera SNP ID: hCV2889230 / Public SNP ID: rs11686314 /
SNP Chromosome Position: 220556298 / SNP in Genomic Sequence: SEQ ID NO: 485 /
SNP Position Genomic: 19109 / SNP Source: dbSNP; Celera; HapMap /
Population(Allele,Count): Caucasian (G,102|A,16) / SNP Type: TRANSCRIPTION
FACTOR BINDING SITE;INTRON /
Context (SEQ ID NO: 3050): /
CCTAGTTGGCTAGACTAAGTATTTTAGCTGTCTCCAATTGGTAACTGTTTAAGAGGTTTAAAGACACAAATTAAGTTGGTAAA
ATTTGAATATGTGAATC
Y
GGGTGAAGGATATATGGTGGTTTGGTGACACTATTTTTGCAGCTTTTCTGTTGGTTTGAAGTTCGTTGAAAATGAAAAGGTCC
ATTAATTAAGAAGGTGG / Celera SNP ID: hCV31716902 / Public SNP ID: rs12999640 /
SNP Chromosome Position: 220557189 / SNP in Genomic Sequence: SEQ ID NO: 485 /
SNP Position Genomic: 20000 / SNP Source: dbSNP; HapMap /
Population(Allele,Count): Caucasian (C,187|T,39) / SNP Type: INTRON /
Context (SEQ ID NO: 3051): /
AGAGAAGAAGGAGATGGAGTGATTTGGGGAATCAAAAGTCAAATCTAGGTTTTTGGGTTTTGCACCAAATTTGATATTGCCTG
CATTGAAAAGAACCCCT
S
TTCTTGGATTAGATTGAATTTATTTGGTGCAAACCCACGCTCTATAAATACTAGTATATTTTTACTTTTTAATACCCTGCATG
AAAGTCCAACGATGATC / Celera SNP ID: hCV2406318 / Public SNP ID: rs661315 /
SNP Chromosome Position: 220570407 / SNP in Genomic Sequence: SEQ ID NO: 485 /
SNP Position Genomic: 33218 / Related Interrogated SNP: hCV2915511 / SNP
Source: dbSNP; HapMap; HGBASE / Population(Allele,Count): Caucasian
(C,111|G,1) / SNP Type: INTRON //

Gene Number: 149 / Gene Symbol: Chr20:34000556..34020556 / Gene Name: /
Chromosome: 20 / OMIM NUMBER: / OMIM Information: // Genomic Sequence (SEQ ID
NO: 486): // / SNP Information /
Context (SEQ ID NO: 3052): /
TTTCAGAATATATATACTCTATGACTACAGGACAATATTTGCCAATGGGATCAGTTATGATGTCTGTTTCTCTTTGAGGAACA
GTCTATGCCCTTTACCT
R

TCAATTAACCCAGCAGAGGGCTGGTTTCTGTGTCTTATTGGCCCCCACACAAAGTTGTTTTCTTTGCCCCTTAGTGCAGTGCA
GACAATTCTATTAAGAA / Celera SNP ID: hCV599157 / Public SNP ID: rs224320 / SNP
Chromosome Position: 34010556 / SNP in Genomic Sequence: SEQ ID NO: 486 / SNP
Position Genomic: 10000 / SNP Source: dbSNP; HapMap; ABI_Val; HGBASE /
Population(Allele,Count): Caucasian (A,214|G,12) / SNP Type:
INTERGENIC;UNKNOWN //

Gene Number: 150 / Gene Symbol: Chr22:24286175..24306175 / Gene Name: /
Chromosome: 22 / OMIM NUMBER: / OMIM Information: // Genomic Sequence (SEQ ID
NO: 487): // / SNP Information /
Context (SEQ ID NO: 3053): /
AGCATCCCTTGGGCATCCCGGGAGGTGTCCCTGCCCTCCATGGCTCACCGTCTGCCACATGGCCCAGCAGCAGCTACTCTGCA
GGGCCATGTGCTGCCAT
S
ACAGGTACTGATCCCCATGGGCGCGGGCCTGCAGGTCCTGGAGGTACCAGTGGTCAGGTGCTTTGTACATGCAGCTCATATAG
AGCAAGCTAGCCACACT / Celera SNP ID: hCV2211618 / Public SNP ID: rs71320057 /
SNP Chromosome Position: 24296175 / SNP in Genomic Sequence: SEQ ID NO: 487 /
SNP Position Genomic: 10000 / SNP Source: dbSNP; HGBASE /
Population(Allele,Count): Caucasian (G,71|C,45) / SNP Type: INTRON //

Gene Number: 151 / Gene Symbol: Chr3:22679328..22699328 / Gene Name: /
Chromosome: 3 / OMIM NUMBER: / OMIM Information: // Genomic Sequence (SEQ ID
NO: 488): // / SNP Information /
Context (SEQ ID NO: 3054): /
CTTCTTCCTTTCCAATTTGGATGCCCTTTATATCTTTCTGTTGTTGGAATGCTCGAGGTAGGACTTCCAGTATTAGGTTGAAT
ATCACTGGTGAAAGTGA
R
CATCCTTGTTGTGTTCCAGATCTTAGAGGAAAGGCTTTCCATTTTTCCCCACTCATTATGATACTAGCTGTGGGTATGTCATA
TACAGTTTTTATTATGT / Celera SNP ID: hCV29260019 / Public SNP ID: rs1851830 /
SNP Chromosome Position: 22689328 / SNP in Genomic Sequence: SEQ ID NO: 488 /
SNP Position Genomic: 10000 / SNP Source: dbSNP; HapMap; HGBASE /
Population(Allele,Count): Caucasian (A,34|G,70) / SNP Type:
INTERGENIC;UNKNOWN //

Gene Number: 152 / Gene Symbol: Chr3:146344136..146364136 / Gene Name: /
Chromosome: 3 / OMIM NUMBER: / OMIM Information: // Genomic Sequence (SEQ ID
NO: 489): // / SNP Information /
Context (SEQ ID NO: 3055): /
TTGCAAACATTTCATGTCCCGCTACTTGGGGACCTCCCTGGGTCAATGTGGCTTATAAGGAGTATACATACACATGCTTGCCC
ATCTCACACCCATCATT
Y
ATATATCAAGATGCCTCCTGTTGCATTTTTAATTTTCACACATTTCCAGTTGAGAATCAGGCACTGACTCTAGCAACCCATGA
ACTCCCAGAATCTGAGA / Celera SNP ID: hCV9540478 / Public SNP ID: rs3852001 /
SNP Chromosome Position: 146354136 / SNP in Genomic Sequence: SEQ ID NO: 489 /
SNP Position Genomic: 10000 / SNP Source: dbSNP; HapMap; ABI_Val; HGBASE /
Population(Allele,Count): Caucasian (T,170|C,56) / SNP Type: INTRON //

Gene Number: 153 / Gene Symbol: Chr4:155608542..155628542 / Gene Name: /
Chromosome: 4 / OMIM NUMBER: / OMIM Information: // Genomic Sequence (SEQ ID
NO: 490): // / SNP Information /
Context (SEQ ID NO: 3056): /
TGGAGGAGAAGCAGGTATGATTGGAGCAATGAGGATTGTAGGAGCTGATGGAAAGAAAGTTGAATAGGTGGAGTAGCTCCAGG
TAATACTAAGGGGAAGG
K
GTGAGTTGCTGATATCTGTGTGGAGATGAAGGTCAGTGATGTTGAAGGTAAGGCACTGGGTATTGGAAAGGTCATCCACACGG
ACACTGAAGACACCAGG / Celera SNP ID: hCV2407354 / Public SNP ID: rs276166 /
SNP Chromosome Position: 155618542 / SNP in Genomic Sequence: SEQ ID NO: 490 /
SNP Position Genomic: 10000 / Related Interrogated SNP: hCV11503414 /
Related Interrogated SNP: hCV15860433 / SNP Source: dbSNP; HapMap; HGBASE /
Population(Allele,Count): Caucasian (G,91|T,11) / SNP Type: INTRON //

Gene Number: 154 / Gene Symbol: Chr4:155966072..156015049 / Gene Name: /
Chromosome: 4 / OMIM NUMBER: / OMIM Information: // Genomic Sequence (SEQ ID

NO: 491): // / SNP Information /
Context (SEQ ID NO: 3057): /
GTGTTTGCATCATTGGAGTCCTCCTCTCACTAAATAAGAGCATGGAAAGTCAAGAGTATTATTTTAAAAGGTTACAGTTCAAG
AGCCCCTTCATTTCTCA
R
ATTTGTTATTGGCATGAGCCAAGAACAATTCCTCCAGTTTCTCAGTATGAGACGGAAGAAATTGCTATTTAGCTTGTCTCTGT
AGCCCAAGGTCATTTCC / Celera SNP ID: hCV7428370 / Public SNP ID: rs1456450 /
SNP Chromosome Position: 155995049 / SNP in Genomic Sequence: SEQ ID NO: 491 /
SNP Position Genomic: 28977 / Related Interrogated SNP: hCV15860433 / SNP
Source: dbSNP; Celera; HapMap; ABI_Val; HGBASE / Population(Allele,Count):
Caucasian (G,50|A,66) / SNP Type: INTERGENIC;UNKNOWN /
Context (SEQ ID NO: 3058): /
TAAACCCTTGCATTTATGACCAACTGATTTTTGACAAAGGTGTCATGGCCATTTAATGTGGACAGAACAGACTTTTATCAAGT
GATTCTGGGGAAAATGG
W
TATCCACAAGCAAGAGAATGAACTTGGACCTCTCTGCCTTACACCAGACACAAAAATTACTCAAAATGCATCAAAGACCTAAA
TGTAAGTGCTAAAACTA / Celera SNP ID: hCV30285831 / Public SNP ID: rs10013914 /
SNP Chromosome Position: 155976072 / SNP in Genomic Sequence: SEQ ID NO: 491 /
SNP Position Genomic: 10000 / Related Interrogated SNP: hCV15860433 / SNP
Source: dbSNP; HapMap / Population(Allele,Count): Caucasian (T,57|A,63) / SNP
Type: INTERGENIC;UNKNOWN //

Gene Number: 155 / Gene Symbol: Chr4:187488104..187508104 / Gene Name: /
Chromosome: 4 / OMIM NUMBER: / OMIM Information: // Genomic Sequence (SEQ ID
NO: 492): // / SNP Information /
Context (SEQ ID NO: 3059): /
GAAGAGAAAAATACTCATTTCAAATTAAAATTTTAAGAACACCAATCACAAAACAAGAGCATCATTTAAAAAATGCGTTTGTAA
TCCTACTTGGCAGGTGG
S
CTAACATTTCGAACTTGACAAAGACGTGGAACTGTGCTTCCAAGGCTGACAGGCAGGAAGAGCCCACACAGCCTCCGGGGCCA
GCCCTTGTTCCTCTCTC / Celera SNP ID: hCV28038101 / Public SNP ID: rs4241833 /
SNP Chromosome Position: 187498104 / SNP in Genomic Sequence: SEQ ID NO: 492 /
SNP Position Genomic: 10000 / SNP Source: dbSNP; HapMap; HGBASE /
Population(Allele,Count): Caucasian (G,137|C,89) / SNP Type:
INTERGENIC;UNKNOWN //

Gene Number: 156 / Gene Symbol: Chr5:34268426..34288426 / Gene Name: /
Chromosome: 5 / OMIM NUMBER: / OMIM Information: // Genomic Sequence (SEQ ID
NO: 493): // / SNP Information /
Context (SEQ ID NO: 3060): /
CATCACAGTAATCTCAAGAGGAATAGGAAAAACATGAAAGAAAAAAGGAATAAGCCACTGATACAGTGGGCTTAAGCTTTGAT
GTGCAATGTGGAATGCT
Y
ACTGTGATATTTATTCTCTGTCCCAATGAACTGGGAAATGGGGATGGAGTCCATAAATCTACGTGGTTTTAAACCACCTCAAA
GGTGAGAGATAATTTAA / Celera SNP ID: hDV77026147 / Public SNP ID: rs4626316 /
SNP Chromosome Position: 34278426 / SNP in Genomic Sequence: SEQ ID NO: 493 /
SNP Position Genomic: 10000 / SNP Source: CDX; dbSNP /
Population(Allele,Count): Caucasian (T,4|C,222) / SNP Type:
INTERGENIC;UNKNOWN //

Gene Number: 157 / Gene Symbol: Chr5:67594395..67634395 / Gene Name: /
Chromosome: 5 / OMIM NUMBER: / OMIM Information: // Genomic Sequence (SEQ ID
NO: 494): // / SNP Information /
Context (SEQ ID NO: 3061): /
GCCTTATTTTTGCATCTCACAAACATAAGTGCAATAGATCTTTTCATTGAACAGCAAAGTAGGATTCATCATTCCATATGACT
TGAGTTACACCAGACCT
R
TTCTGCCCAATGCCTTTTTGATTACAGTGTAGCTTGCCCACCGCATTTGTCGTTTTAGATACTTTGCTAGCCGGCCACTTTGG
ATTTCATCAGACAGTCC / Celera SNP ID: hCV27474984 / Public SNP ID: rs3756668 /
SNP Chromosome Position: 67596088 / SNP in Genomic Sequence: SEQ ID NO: 494 /
SNP Position Genomic: 1693 / SNP Source: dbSNP; HapMap; ABI_Val; HGBASE /
Population(Allele,Count): Caucasian (G,128|A,96) / SNP Type: MISSENSE
MUTATION;UTR3;ESE SYNONYMOUS /

Context (SEQ ID NO: 3062): /
TGTCTCCAAATTCTGTCAGCATTCCTGAAAGTCTTTAGATGTGTGGGAATTTTCAATTTTCCTTTGAAACAGTTGTATCATTT
TAACTGTTCCTTAATGA
S
TTAAACTAAATCTGTGATATGTTTATTTTATATTTATATGCAGGTCGTGGCTTTAGCACTAAAACAAAGCCCTTAACAAAGAG
CATGAAGTTCATTGGGT / Celera SNP ID: hCV977979 / Public SNP ID: rs256508 / SNP
Chromosome Position: 67614395 / SNP in Genomic Sequence: SEQ ID NO: 494 / SNP
Position Genomic: 20000 / Related Interrogated SNP: hCV27474984 / SNP Source:
dbSNP; Celera; HapMap; ABI_Val; HGBASE / Population(Allele,Count): Caucasian
(G,64|C,56) / SNP Type: INTERGENIC;UNKNOWN /
Context (SEQ ID NO: 3063): /
AGATCACGACTTTAGATCACCTGCTTACTGAAATAGTACTTCTAGCATTTTAACTAAAAACCATTACCATTAATGAGTGATAT
GTTACTTTCTAATTTAT
K
TTCATAATGTAGGCCATAGGAAAAAGAAAACAGATTGATTTGCAGTTTAGGAGGCCCAGTCTGCTCTTTTTATCTGTCTTTAA
CCAGCTGTGGGAAGTGG / Celera SNP ID: hCV3164046 / Public SNP ID: rs9291926 /
SNP Chromosome Position: 67599656 / SNP in Genomic Sequence: SEQ ID NO: 494 /
SNP Position Genomic: 5261 / Related Interrogated SNP: hCV27474984 / SNP
Source: dbSNP; Celera; HapMap; ABI_Val / Population(Allele,Count): Caucasian
(T,58|G,62) / SNP Type: INTERGENIC;UNKNOWN /
Context (SEQ ID NO: 3064): /
TCTTTGCACATTCAAGTCTAATGTGCATAAGAGCCGAAAATATAAGTAACATTTTTGAATTTTATTTTTGTAATTTGGTCTTC
ACTTTAACCAACTACTG
R
ACTTTTTCTCTCTAAAAATAAAAACATCTTAATTTATAATAAAACAAATAACCAAATCAGGCTCCTATAACCACTGCAGCCTG
TAGGAATCTCTTTTGGC / Celera SNP ID: hCV3164066 / Public SNP ID: rs34292 / SNP
Chromosome Position: 67623082 / SNP in Genomic Sequence: SEQ ID NO: 494 / SNP
Position Genomic: 28687 / Related Interrogated SNP: hCV27474984 / SNP Source:
dbSNP; Celera; HapMap; HGBASE / Population(Allele,Count): Caucasian
(G,57|A,49) / SNP Type: TRANSCRIPTION FACTOR BINDING SITE;INTERGENIC;UNKNOWN /
Context (SEQ ID NO: 3065): /
AAATGAATATCAATGAAGTAGCTATCTAGTGATGTAGGGGTGGAGAGAACGCAGGTGGTTAAGAAGGGTAGAAACTGTTCTGA
GGTGGAAGGCTACAAAG
K
AGGGGCTCTGAGGATCAAAGGGAATTTTTTTGTTGTAGAAGGTATCAGGAAACATTTCAAAAGACTGAAAGGGGCAAAAACAA
TGACTGGAGTCTGGGAT / Celera SNP ID: hCV11824867 / Public SNP ID: rs256507 /
SNP Chromosome Position: 67614043 / SNP in Genomic Sequence: SEQ ID NO: 494 /
SNP Position Genomic: 19648 / Related Interrogated SNP: hCV27474984 / SNP
Source: dbSNP; Celera; HapMap; HGBASE / Population(Allele,Count): Caucasian
(G,62|T,52) / SNP Type: INTERGENIC;UNKNOWN //

Gene Number: 158 / Gene Symbol: Chr6:11895508..11971861 / Gene Name: /
Chromosome: 6 / OMIM NUMBER: / OMIM Information: // Genomic Sequence (SEQ ID
NO: 495): // / SNP Information /
Context (SEQ ID NO: 3066): /
GTGTCTGTGACATAGTGAACAGTCGTCTAGGATGACTAGTCAATGCATCTAGTCAATGCTAGCAGCTACTATCATTATTATTC
GCACTCTACACAGCCAG
Y
GTGGGGATTAATGGCATGATGTGCTTTGAGAGGGAAATTTCTCACTAAGCCTATTTTTTGTTTATTTGTTTGTTTAAGAGACA
AGATCTCCCTCTGGAAC / Celera SNP ID: hCV2499170 / Public SNP ID: rs169713 /
SNP Chromosome Position: 11920517 / SNP in Genomic Sequence: SEQ ID NO: 495 /
SNP Position Genomic: 25009 / SNP Source: dbSNP; HapMap; HGBASE /
Population(Allele,Count): Caucasian (T,182|C,44) / SNP Type: INTRON /
Context (SEQ ID NO: 3067): /
CAAATAGAATATTTCATAGAGCCCAAAGTAAGGAAGATGAACATTTTTGCCTCGGGATTTCCAAAGAATAAATGTGAAATGTG
TCTCCATTCTGTGGTCA
Y
GGCCAAAGTCTGCAGCCTAGCTCTGTCAGCCAGTCATACTTTCCTTCAAACGCTTTGGCAGGTACATTGTATAGAAACTACTG
GAGAGAGATCCCTCATC / Celera SNP ID: hCV2238240 / Public SNP ID: rs209773 /
SNP Chromosome Position: 11913828 / SNP in Genomic Sequence: SEQ ID NO: 495 /
SNP Position Genomic: 18320 / Related Interrogated SNP: hCV2499170 / SNP
Source: dbSNP; HapMap; ABI_Val; HGBASE / Population(Allele,Count): Caucasian
(T,180|C,46) / SNP Type: INTRON /

Context                    (SEQ ID NO: 3068): /
ACCCTAGTTTCATTGGTTCTCATGGCAGCCTCTCAAATTATACAATTGGGTATTTTTGTAACTCCATCTCTTCATCTCTTATT
TTTTAGTCTTTTTTTTTT
M
TCTATTGGAGTTTGGATTTTTAGCTTTTCAATTTCATGGAAAAAACAGATTTTAAATAATATTTATGAAACAGTATAAGCCTT
GCAGTTTAGACTATTTT / Celera SNP ID: hCV2238245 / Public SNP ID: rs23805 / SNP
Chromosome Position: 11922362 / SNP in Genomic Sequence: SEQ ID NO: 495 / SNP
Position Genomic: 26854 / Related Interrogated SNP: hCV2499170 / SNP Source:
dbSNP; HapMap / Population(Allele,Count): Caucasian (A,86|C,28) / SNP Type:
INTERGENIC;UNKNOWN /
Context                    (SEQ ID NO: 3069): /
CTTTTTTTTTTATCTATTGGAGTTTGGATTTTTAGCTTTTCAATTTCATGGAAAAAACAGATTTTAAATAATATTTATGAAACA
GTATAAGCCTTGCAGTT
Y
AGACTATTTTAGAAACATCAGATTATATTAGTTATATGGGCAACTGGTAATTTTATTGTCTACATTTTATAAAGTCTAAAAAG
AGAAACAAGGAATAATA / Celera SNP ID: hCV2238247 / Public SNP ID: rs209778 /
SNP Chromosome Position: 11922452 / SNP in Genomic Sequence: SEQ ID NO: 495 /
SNP Position Genomic: 26944 / Related Interrogated SNP: hCV2499170 / SNP
Source: dbSNP; HapMap; HGBASE / Population(Allele,Count): Caucasian
(T,194|C,30) / SNP Type: INTERGENIC;UNKNOWN /
Context                    (SEQ ID NO: 3070): /
GAAATAGAGATGAAATACCAAGAAATTAAATCTATTTTGCAATTTTTGAAAAATGGCTTGAAATAAGTTGTATAATGTATTAG
TGGATCCACAGTGGTAA
Y
TGAATAAACTAGTTCACTATTCTGGATATACCAGTTTAATAATGTCATAATTTGTGCAGCCATTTGCTTGCTTTTTCCCTCTA
AAATATTTAAGGCACTC / Celera SNP ID: hCV2238250 / Public SNP ID: rs209780 /
SNP Chromosome Position: 11923194 / SNP in Genomic Sequence: SEQ ID NO: 495 /
SNP Position Genomic: 27686 / Related Interrogated SNP: hCV2499170 / SNP
Source: dbSNP; HapMap; HGBASE / Population(Allele,Count): Caucasian
(C,178|T,46) / SNP Type: INTERGENIC;UNKNOWN /
Context                    (SEQ ID NO: 3071): /
TTCCTAGGGCAAAATCTTTTTCCCTTGAAGACTGGAAATATTATCCATGTTGTCCTCCGGAATATTTTCAATGACTTGTGCCC
TGCCAGCTCTAGCTTTT
S
AAGGGTCTACACTCATCATCAACAGGTAGACTGGTTTTTACAAATGTTTTTACCATAACTTCATTTCCTGTTTCAAATGTGAA
ATGGATCAAAAATGAGA / Celera SNP ID: hCV2238261 / Public SNP ID: rs209814 /
SNP Chromosome Position: 11951861 / SNP in Genomic Sequence: SEQ ID NO: 495 /
SNP Position Genomic: 56353 / Related Interrogated SNP: hCV2499170 / SNP
Source: dbSNP; HapMap; ABI_Val; HGBASE / Population(Allele,Count): Caucasian
(G,192|C,34) / SNP Type: INTERGENIC;UNKNOWN /
Context                    (SEQ ID NO: 3072): /
ATGAGACACTGAAGCAGTTTAAAGATCGGGTTTTCAATGCGTGACATATAACTGTCCATTTTCAGGCTGGAAAAAATGAATGGC
TTTCTAGGAGGCATACT
R
TTCAACTCACCTTTACAACTTTCAATCACTGTGGCTGAAATAGAGTCATGAAAGACAAGATGTTGATAATACTTACAAGAGAA
AGAAGAAATAAAATCCC / Celera SNP ID: hCV2238263 / Public SNP ID: rs209812 /
SNP Chromosome Position: 11953887 / SNP in Genomic Sequence: SEQ ID NO: 495 /
SNP Position Genomic: 58379 / Related Interrogated SNP: hCV2499170 / SNP
Source: dbSNP; Celera; HapMap; HGBASE / Population(Allele,Count): Caucasian
(G,186|A,34) / SNP Type: INTERGENIC;UNKNOWN /
Context                    (SEQ ID NO: 3073): /
TGTAAAGAAACAGTTTCTTTCTTTCCTCTATTTTTCCTACCATTCAACTTTTATAAGACAATTGAATTTTCTCTACAAAAAGC
AAATAAATTACATTTAC
W
GAGTAGAATTCAAAAGGTAAAGGTCAAAGAAAATATGAGGCTATTTAATAATGGTGGTTCTTAGCTTTTCTTTTAGATGTGTA
TCAGCATATATAAGTTT / Celera SNP ID: hCV2499165 / Public SNP ID: rs209774 /
SNP Chromosome Position: 11915508 / SNP in Genomic Sequence: SEQ ID NO: 495 /
SNP Position Genomic: 20000 / Related Interrogated SNP: hCV2499170 / SNP
Source: dbSNP; Celera; HapMap; HGBASE / Population(Allele,Count): Caucasian
(A,93|T,27) / SNP Type: INTRON /
Context                    (SEQ ID NO: 3074): /
TTCCTGTTTAGTCTTGGGTCCCATCCCCAAGATATCTCATTATGCATATGCAAATATTCCAAAATCCAAAAAAATCCCAAAGT
CAAATCCAGACGCTTTT

R
GTTCCAAGCATTTCGGATAAGGGATTCTCAACCTGAAACTGTTTTTAAAGAAAATTTCGAGGAGAAAAAAGCTGAGCAAATTC
CACACTCCTTAAAAAAA / Celera SNP ID:   hCV2499169 / Public SNP ID:    rs85219 /  SNP
Chromosome Position:   11919929 /  SNP in Genomic Sequence:   SEQ ID NO: 495 /  SNP
Position Genomic:   24421 /  Related Interrogated SNP:   hCV2499170 /  SNP Source:
 dbSNP; HapMap; HGBASE /  Population(Allele,Count):   Caucasian (G,93|A,27) /  SNP
Type:   INTRON /
Context                  (SEQ ID NO: 3075): /
AGCCTGGGTGACAGAGAGAGACTCCATCTCCAAAAAAAAAAGAAAATGATTTTCACACAGCTGGAACATGTTTTTTTAAGAAC
ATTCATAAAAGTAGTAT
Y
AAGAACTACTATTAGTAAGCTGTGTTCACAAAGAAAAATATTCCGCTATATCTGCACCTAAAGTCCTGTCCATACAAGATCCA
AAAAACAAGACAGGAAG / Celera SNP ID:   hCV2499176 / Public SNP ID:    rs9380643 /
SNP Chromosome Position:   11930487 /  SNP in Genomic Sequence:   SEQ ID NO: 495 /
SNP Position Genomic:   34979 /  Related Interrogated SNP:   hCV2499170 /  SNP
Source:   dbSNP; Celera; HapMap /  Population(Allele,Count):   Caucasian
(C,168|T,54) /  SNP Type:    INTERGENIC;UNKNOWN /
Context                  (SEQ ID NO: 3076): /
TAGCCATTATAATGTCATAACACAACACATTATTTCTATGTTTAGTATGTTTTGATGCACATATGCTTAACACTGATTACAAT
TGCCTAGCCTATTCAGT
R
TAGCAACATATTGTACAACATCTTTGTAGCCCAGGAGAAATAAGCTGTACCATCTAGCCTAGGTGTGTAGTAGGCTTGTACCA
TCTAGACTTATGTAAAT / Celera SNP ID:   hCV2499198 / Public SNP ID:    rs1205883 /
SNP Chromosome Position:   11947911 /  SNP in Genomic Sequence:   SEQ ID NO: 495 /
SNP Position Genomic:   52403 /  Related Interrogated SNP:   hCV2499170 /  SNP
Source:   dbSNP; Celera; HapMap; HGBASE /  Population(Allele,Count):    Caucasian
(A,192|G,34) /  SNP Type:    INTERGENIC;UNKNOWN /
Context                  (SEQ ID NO: 3077): /
CAACACATTATTTCTATGTTTAGTATGTTTTGATGCACATATGCTTAACACTGATTACAATTGCCTAGCCTATTCAGTATAGC
AACATATTGTACAACAT
Y
TTTGTAGCCCAGGAGAAATAAGCTGTACCATCTAGCCTAGGTGTGTAGTAGGCTTGTACCATCTAGACTTATGTAAATACACT
CTATGATGTTTGCACAA / Celera SNP ID:   hCV2499199 / Public SNP ID:    rs1205884 /
SNP Chromosome Position:   11947933 /  SNP in Genomic Sequence:   SEQ ID NO: 495 /
SNP Position Genomic:   52425 /  Related Interrogated SNP:   hCV2499170 /  SNP
Source:   dbSNP; Celera; HapMap; HGBASE /  Population(Allele,Count):   Caucasian
(C,192|T,34) /  SNP Type:    INTERGENIC;UNKNOWN /
Context                  (SEQ ID NO: 3078): /
ACAGAATTGCCCAATGGCATATTTCTTAGAACATATTCCCTTTGGTTAAGTGACACATGACTGTTTTTATGGCTTTTTTACCC
AGACTTGGATTTGAGCA
Y
GAGAATTACCTTGTATGCACCTTTCACTTAGTAGGCTATTAGTTATTTTTGTTCTCCGGTTTAAATTAAAAATAAATTGTTAT
ATACATATATGTATATA / Celera SNP ID:   hCV2499201 / Public SNP ID:    rs1205887 /
SNP Chromosome Position:   11948136 /  SNP in Genomic Sequence:   SEQ ID NO: 495 /
SNP Position Genomic:   52628 /  Related Interrogated SNP:   hCV2499170 /  SNP
Source:   dbSNP; Celera; HapMap; ABI_Val; HGBASE /  Population(Allele,Count):
Caucasian (C,192|T,34) /  SNP Type:    INTERGENIC;UNKNOWN /
Context                  (SEQ ID NO: 3079): /
CCATGGTTACAAATAAACACCCATATTTCAATAGCTTAACACAACCAAGATTTATTATGTGTTCATGCTGTATATCCAGTGCT
TATTAGACGGGAACTCG
K
TTCCACTGATTCATTCAGGTGGATCCAGGATAACGGGTATTGCACCATGTTGTGATGCTGCCACCTCAACATGGAGCTGTATG
AGAGGAAGAAAGAGCAT / Celera SNP ID:   hCV7465311 / Public SNP ID:    rs1205863 /
SNP Chromosome Position:   11943526 /  SNP in Genomic Sequence:   SEQ ID NO: 495 /
SNP Position Genomic:   48018 /  Related Interrogated SNP:   hCV2499170 /  SNP
Source:   dbSNP; HapMap; HGBASE /  Population(Allele,Count):   Caucasian
(T,192|G,34) /  SNP Type:    INTERGENIC;UNKNOWN /
Context                  (SEQ ID NO: 3080): /
AAAAAAAAAAGGATAAGGTATCCATGGTTACAAATAAACACCCATATTTCAATAGCTTAACACAACCAAGATTTATTATGTGT
TCATGCTGTATATCCAG
Y
GCTTATTAGACGGGAACTCGTTTCCACTGATTCATTCAGGTGGATCCAGGATAACGGGTATTGCACCATGTTGTGATGCTGCC
ACCTCAACATGGAGCTG / Celera SNP ID:   hCV7465312 / Public SNP ID:    rs864245 /

554

SNP Chromosome Position: 11943505 / SNP in Genomic Sequence: SEQ ID NO: 495 / SNP Position Genomic: 47997 / Related Interrogated SNP: hCV2499170 / SNP Source: dbSNP; HapMap; HGBASE / Population(Allele,Count): Caucasian (T,192|C,34) / SNP Type: INTERGENIC;UNKNOWN /
Context (SEQ ID NO: 3081): /
ATTTTATTTCTTAAGACTTATTAAAGCATGGGACTAATGAAATGATTATTTCTTAGAATCTGAATGCAGGCATCTATAACAAT
TTTTGGAAAAATACTCC
R
CGATTTTCTTGGCAGGTTTTGGAACCTTGAAATGTGAAAAAGCAGCACACAGGTGCGCATTAAACAGGTGCTTTAGACAGTAC
GTATGAGTAGTTCAGAG / Celera SNP ID: hCV7465347 / Public SNP ID: rs1205852 /
SNP Chromosome Position: 11942371 / SNP in Genomic Sequence: SEQ ID NO: 495 / SNP Position Genomic: 46863 / Related Interrogated SNP: hCV2499170 / SNP Source: dbSNP; HapMap; HGBASE / Population(Allele,Count): Caucasian (A,192|G,34) / SNP Type: INTERGENIC;UNKNOWN /
Context (SEQ ID NO: 3082): /
CAAGAACTGGAAGGCTTTTTCTACAAAGGACCAGAAAGTCAATATTTTAAGGTTGTGTAGGCCAGATAATCCCTCTCATAACT
ACTCCACTCTGCCATTA
S
AGCATGCAAGCAGCCATGATAATTCATAAAGTAATGATCATGGGTATGCTTCAATTTTGTATTTATGAAAACGGGTAACAAGC
AGAATTTGATGCACAGG / Celera SNP ID: hCV7465349 / Public SNP ID: rs1205850 /
SNP Chromosome Position: 11940593 / SNP in Genomic Sequence: SEQ ID NO: 495 / SNP Position Genomic: 45085 / Related Interrogated SNP: hCV2499170 / SNP Source: dbSNP; HapMap; HGBASE / Population(Allele,Count): Caucasian (C,173|G,43) / SNP Type: INTERGENIC;UNKNOWN /
Context (SEQ ID NO: 3083): /
TATTTACTGTAGCAAGCGTAGCCTGTTCTAATACAGAGAAGAATGGGTTTCAGTTTTGAGAGCTAACCAAAAATCTAGTTAGA
CTCTTAAACATTTCAAC
S
TTTTAATTAAAGGCTTAGTAGCATGTATCAAGAACTGGAAGGCTTTTTCTACAAAGGACCAGAAAGTCAATATTTTAAGGTTG
TGTAGGCCAGATAATCC / Celera SNP ID: hCV7465354 / Public SNP ID: rs1205849 /
SNP Chromosome Position: 11940464 / SNP in Genomic Sequence: SEQ ID NO: 495 / SNP Position Genomic: 44956 / Related Interrogated SNP: hCV2499170 / SNP Source: dbSNP; HapMap; HGBASE / Population(Allele,Count): Caucasian (G,94|C,26) / SNP Type: INTERGENIC;UNKNOWN /
Context (SEQ ID NO: 3084): /
TATAGAGAGAAGAGACGAGTTATCCATAAAGGCAGAATAAATAGGTTTGCCATTGGCATGTTCCTAAGACATTAATAGAGTAA
GCTATATTTTCCCTAGA
M
TGTTTATACTTGTGTCATAAACACGTATGTGTCACAATACTGTGAACCACTGCATCACACCCTCCAACACACTCACACACACA
CACACACATACACACAC / Celera SNP ID: hCV7465364 / Public SNP ID: rs864244 /
SNP Chromosome Position: 11929602 / SNP in Genomic Sequence: SEQ ID NO: 495 / SNP Position Genomic: 34094 / Related Interrogated SNP: hCV2499170 / SNP Source: dbSNP; HapMap; HGBASE / Population(Allele,Count): Caucasian (C,95|A,25) / SNP Type: INTERGENIC;UNKNOWN /
Context (SEQ ID NO: 3085): /
TCTTGCTGGGTCTTTCCCAAGAAATCTCTTTGGAAATGGGAAGTTATGTCTGGAATTCAGGTGGCAGAAGGCCACCTGTGTGC
CTACTGTTTGGAAGTCA
R
GGATGGAGGAGGGTGCAGGAAAGCATATTGCATTGGCACCACCAAAATGATGCACTCTAACCTGAGGCCACACACACCACACA
CACACATGCTTCTACAG / Celera SNP ID: hCV7465377 / Public SNP ID: rs1210621 /
SNP Chromosome Position: 11927921 / SNP in Genomic Sequence: SEQ ID NO: 495 / SNP Position Genomic: 32413 / Related Interrogated SNP: hCV2499170 / SNP Source: dbSNP; HapMap; ABI_Val; HGBASE / Population(Allele,Count): Caucasian (A,93|G,23) / SNP Type: INTERGENIC;UNKNOWN /
Context (SEQ ID NO: 3086): /
AGAAAGCCTCAGGCAGTGAAAGGAAGGTTCTCTGGCCCTTGGAGGGAGGGCTAGTGTGCACGCAACTGGCAAAGGCATGCGGA
TGCAGGTGGGACCCAGA
S
AGCCAGGGGTCTCCACTGCTTCTGATGTCAAAACGTGTTCCTTCCAATTGGCCAACTGCCCTCTCCATGTCCCAGGGCAGATT
CCTGCTTCTCTAGAAAC / Celera SNP ID: hCV7465418 / Public SNP ID: rs876828 /
SNP Chromosome Position: 11918435 / SNP in Genomic Sequence: SEQ ID NO: 495 / SNP Position Genomic: 22927 / Related Interrogated SNP: hCV2499170 / SNP Source: dbSNP; Celera; HapMap; HGBASE / Population(Allele,Count): Caucasian

(G,86|C,34) / SNP Type: INTRON /
Context (SEQ ID NO: 3087): /
AATTTGGGAAAATGTAAGTAAGGTAGAATCCAGTTTATGGGATAATTTTTGGATGCGAGAATACTTTTAAACCTTCACTTATG
ATTGTTATTGCACAAAC
R
AGATTGGTCTGCCCTAGCAAGTCTCACCGAGGTTTTGCAACATATTTACAAGTACAAATCTGTGTGGCAAAGAGTACTTTTTG
TACTATGGGCAGCTTTG / Celera SNP ID: hCV31001553 / Public SNP ID: rs10947661 /
SNP Chromosome Position: 11934964 / SNP in Genomic Sequence: SEQ ID NO: 495 /
SNP Position Genomic: 39456 / Related Interrogated SNP: hCV2499170 / SNP
Source: dbSNP / Population(Allele,Count): Caucasian (G,182|A,44) / SNP Type:
INTERGENIC;UNKNOWN /
Context (SEQ ID NO: 3088): /
TTTAAGCCAGTGCTTCCAAAGAGGCCCACCTTCTCCACCTGCCTGTCTAAAATTCATCATATTTGAGCTTTTCTTGGACTACA
AGCGGCCATTTGAAAAT
M
AGTATCTTCTGATTGCATCGGAATTACCTGGGAGGTTTTTTAAATATGAAGGTTCCCAGGCCTCATACTAGACCTAAAGAATC
AATAGCACAGAGCTGAA / Celera SNP ID: hCV30060798 / Public SNP ID: rs9394412 /
SNP Chromosome Position: 11931886 / SNP in Genomic Sequence: SEQ ID NO: 495 /
SNP Position Genomic: 36378 / SNP Source: dbSNP / Population(Allele,Count):
Caucasian (A,182|C,44) / SNP Type: INTERGENIC;UNKNOWN /
Context (SEQ ID NO: 3089): /
TTAAGCCAGTGCTTCCAAAGAGGCCCACCTTCTCCACCTGCCTGTCTAAAATTCATCATATTTGAGCTTTTCTTGGACTACAA
GCGGCCATTTGAAAATA
M
GTATCTTCTGATTGCATCGGAATTACCTGGGAGGTTTTTTAAATATGAAGGTTCCCAGGCCTCATACTAGACCTAAAGAATCA
ATAGCACAGAGCTGAAA / Celera SNP ID: hDV101721202 / Public SNP ID: rs9394412 /
SNP Chromosome Position: 11931887 / SNP in Genomic Sequence: SEQ ID NO: 495 /
SNP Position Genomic: 36379 / Related Interrogated SNP: hCV2499170 / SNP
Source: CDX / Population(Allele,Count): no_pop (A,-|C,-) / SNP Type: //

Gene Number: 159 / Gene Symbol: Chr6:22191831..22211831 / Gene Name: /
Chromosome: 6 / OMIM NUMBER: / OMIM Information: // Genomic Sequence (SEQ ID
NO: 496): // / SNP Information /
Context (SEQ ID NO: 3090): /
AGGAATCTTTCCACCACTTCGATTTCATCTTACTCCTGAAACAGTCAGCCAGAACTGTCTAATTCAGTCTATACTTCTGATAC
AAAACTCTACCCAGCAA
Y
CACTATGGTTTTGTTGCTTTAGGTACGTGTTAGAAAGCACCCACATCAATTTACAGAAAATCCTTAACTGAATCTCTAGTCCC
AGAGGAGTCATATTTTA / Celera SNP ID: hCV29245634 / Public SNP ID: rs7765440 /
SNP Chromosome Position: 22201831 / SNP in Genomic Sequence: SEQ ID NO: 496 /
SNP Position Genomic: 10000 / SNP Source: dbSNP; HapMap /
Population(Allele,Count): Caucasian (C,106|T,12) / SNP Type: INTRON //

Gene Number: 160 / Gene Symbol: Chr6:33049669..33069669 / Gene Name: /
Chromosome: 6 / OMIM NUMBER: / OMIM Information: // Genomic Sequence (SEQ ID
NO: 497): // / SNP Information /
Context (SEQ ID NO: 3091): /
TTGGTCTTTGAGACAATGGTGCCATTAAGGACCCCCGCCAGGCCCACCAGCAGGCCGAGGGCACAGACCAGCATCTCCATGGT
CTCAGGCACCTGGATTA
K
TTCATGGACCTCTGGGGCACCAAGGGAAGACAGAGTTATAAGGTACAGAGAGCAGGGGCTGGCCTTGGATGTGGGAGGTGTTG
GGTATTCGAAACCATGA / Celera SNP ID: hDV92976008 / Public SNP ID: rs2281390 /
SNP Chromosome Position: 33059669 / SNP in Genomic Sequence: SEQ ID NO: 497 /
SNP Position Genomic: 10000 / SNP Source: dbSNP; Celera; HGBASE /
Population(Allele,Count): Caucasian (G,185|T,41) / SNP Type: TRANSCRIPTION
FACTOR BINDING SITE;PSEUDOGENE //

Gene Number: 161 / Gene Symbol: Chr6:130247753..130287753 / Gene Name: /
Chromosome: 6 / OMIM NUMBER: / OMIM Information: // Genomic Sequence (SEQ ID
NO: 498): // / SNP Information /
Context (SEQ ID NO: 3092): /
AGGTCTCCTCTGAACTGTGTTGCTTTGCTTCCCCAAGTAGTCCCTTCTCCAAAGCAAGACCTAATTGTGTGCTATATAAAGTC
GGCAGGGCATTCTGACC

R
CCAAGTTCCATTCTGAGCAGGAAGAAGTCAGGCAGGCTGGTGACCTCCCCTGTCCAGTTACCAAAATCGAGAGGGCTTTAATC
TGGGGTGTAGCCCATTG / Celera SNP ID: hCV7422169 / Public SNP ID: rs1538185 /
SNP Chromosome Position: 130267753 / SNP in Genomic Sequence: SEQ ID NO: 498 /
SNP Position Genomic: 20000 / SNP Source: dbSNP; Celera; HapMap; HGBASE /
Population(Allele,Count): Caucasian (G,185|A,39) / SNP Type:
INTERGENIC;UNKNOWN /
Context (SEQ ID NO: 3093): /
TCCAACCTTTACTGAGCACCTACTGTATGCTTTAGCATGTTGTCCCCATCATTTTGGAGAGGACATAGTCAATCTAGTACATG
GTACAATCTTTTTTCCT
Y
CCACTCAGCAACAGCAAAATACATCTGGCTCCCGAAACAGTATGGTTTTCCCTCAGATATTTTTCTAAATCACATTGATTTGA
ATTAAATAGTTCAGAAA / Celera SNP ID: hCV29948033 / Public SNP ID: rs9375683 /
SNP Chromosome Position: 130263591 / SNP in Genomic Sequence: SEQ ID NO: 498 /
SNP Position Genomic: 15838 / SNP Source: dbSNP; HapMap /
Population(Allele,Count): Caucasian (T,178|C,44) / SNP Type:
INTERGENIC;UNKNOWN //

Gene Number: 162 / Gene Symbol: Chr9:136459322..136479322 / Gene Name: /
Chromosome: 9 / OMIM NUMBER: / OMIM Information: // Genomic Sequence (SEQ ID
NO: 499): // / SNP Information /
Context (SEQ ID NO: 3094): /
CCAAAGTGCTGGGATTATAGGCATGGGCCACTGCGCCTGGCCTATTTATTTCTATTCACACTGCACATCCCTCATCCAACTAT
GAAATCCTTCCCTGGAG
K
GATTACTTGAGTGAACAAGTATATAAAATGAACACACACAAAAAAATAAAACCTGTGGATGGCCTACATGACTGTTTACTGATA
TGGGTCCAGGTAAATCA / Celera SNP ID: hCV16199728 / Public SNP ID: rs3025336 /
SNP Chromosome Position: 136469322 / SNP in Genomic Sequence: SEQ ID NO: 499 /
SNP Position Genomic: 10000 / Related Interrogated SNP: hCV27859399 / SNP
Source: dbSNP; HapMap; HGBASE / Population(Allele,Count): Caucasian
(T,206|G,20) / SNP Type: TRANSCRIPTION FACTOR BINDING SITE;INTERGENIC;UNKNOWN
//

Gene Number: 163 / Gene Symbol: ChrX:138497825..138517825 / Gene Name: /
Chromosome: X / OMIM NUMBER: / OMIM Information: // Genomic Sequence (SEQ ID
NO: 500): // / SNP Information /
Context (SEQ ID NO: 3095): /
AATTAGAGGGTCACTGATGGTCGAGAGGAAAAGGAGAAAGACAGTTGGCAAACTATTTATTTTTGACTCTAACATGAGACTTT
ATAAGTTTCCCATGTCA
R
AACTCTTTACAAGGTCATCACTTTGGTCAATTTCAAATTGATTTGTTATTAAACATGTGCTGCCTATACTGTTCCAACATGTA
ATTCTTTCTCCTTCTGC / Celera SNP ID: hCV1264349 / Public SNP ID: rs12557491 /
SNP Chromosome Position: 138536879 / SNP in Genomic Sequence: SEQ ID NO: 500 /
SNP Position Genomic: 39054 / Related Interrogated SNP: hCV2986566 / SNP
Source: dbSNP; Celera; HapMap / Population(Allele,Count): Caucasian
(G,140|A,25) / SNP Type: INTERGENIC;UNKNOWN /
Context (SEQ ID NO: 3096): /
CCCCAAGTGTGTGTAGGGATTGAGTGGGATAGGGTGAGAGAGGTGTGTGTCTTCACATGAGTCTATGCATGCAAGTGTGTGTG
TAGGAAGAGGAGAGGAG
K
GATTAATTGATGTTCCCTCCTCTCTGCTGGGAGCCCTTAGAGATAAGGGAAAAGAAGAGACAGCAACAGGTTTGAACACCAAT
TAGATAAGACTTCATGA / Celera SNP ID: hCV29394017 / Public SNP ID: rs7058459 /
SNP Chromosome Position: 138507825 / SNP in Genomic Sequence: SEQ ID NO: 500 /
SNP Position Genomic: 10000 / Related Interrogated SNP: hCV2986566 / SNP
Source: dbSNP; HapMap / Population(Allele,Count): Caucasian (T,75|G,14) / SNP
Type: INTERGENIC;UNKNOWN /
Context (SEQ ID NO: 3097): /
TCTTCTGATGCCAGATCTGACATTCAAATACGACTGTCCTTTCTGTTGGTTACCTTTTAACATTGCAATGAAATTCAGTGGAT
ATTTAGTTTTAGACCCC
R
AGCTGCATTAATCTAATGGATCTCCACATTTTAGAATATCCTTCATGTATATATCATAAGTGAGAGAAATAAGTGATAAAGAA
TGAAGAGGGCAGATTTG / Celera SNP ID: hDV70794854 / Public SNP ID: rs17002122 /
SNP Chromosome Position: 138531768 / SNP in Genomic Sequence: SEQ ID NO: 500 /

SNP Position Genomic: 33943 / Related Interrogated SNP: hCV596331 / SNP Source: dbSNP; HapMap / Population(Allele,Count): Caucasian (A,154|G,15) / SNP Type: INTERGENIC;UNKNOWN / Context (SEQ ID NO: 3098): / AGCTGCCCTCACTGGCCCTGCCGCTCTACCAGTACACCAGTGAGTCCACCCCGCGCCTCCGCAGCGCGCCCAGCTGCATCCTC CTGGCCATGTTCCTCGT Y CACTACTGGCATCGGTGCTTAATTTACCCATTTCTGATGCGAGGAGGAAAGCCTGTGCCACTGTTGGCGTGCACAATGGCGAT TATGTTCTGTACCTGTA / Celera SNP ID: hDV71066592 / Public SNP ID: rs17002116 / SNP Chromosome Position: 138529334 / SNP in Genomic Sequence: SEQ ID NO: 500 / SNP Position Genomic: 31509 / Related Interrogated SNP: hCV596331 / SNP Source: dbSNP / Population(Allele,Count): Caucasian (C,154|T,12) / SNP Type: PSEUDOGENE /

# TABLE 3, page 1 of 71

| Interrogated SNP | Interrogated rs | LD SNP | LD SNP rs | Power | Threshold r² | r² |
|---|---|---|---|---|---|---|
| hCV11503414 | rs2066865 | hCV11281035 | rs4583739 | 0.51 | 0.048174697 | 0.0695 |
| hCV11503414 | rs2066865 | hCV11503378 | rs1490655 | 0.51 | 0.048174697 | 0.0612 |
| hCV11503414 | rs2066865 | hCV11503379 | rs1490654 | 0.51 | 0.048174697 | 0.0677 |
| hCV11503414 | rs2066865 | hCV11503382 | rs1873369 | 0.51 | 0.048174697 | 0.257 |
| hCV11503414 | rs2066865 | hCV11503416 | rs2066864 | 0.51 | 0.048174697 | 1 |
| hCV11503414 | rs2066865 | hCV11503431 | rs2066861 | 0.51 | 0.048174697 | 1 |
| hCV11503414 | rs2066865 | hCV11503469 | rs2066854 | 0.51 | 0.048174697 | 0.9559 |
| hCV11503414 | rs2066865 | hCV11503470 | rs1800788 | 0.51 | 0.048174697 | 0.4341 |
| hCV11503414 | rs2066865 | hCV11852898 | rs6819508 | 0.51 | 0.048174697 | 0.0566 |
| hCV11503414 | rs2066865 | hCV11853353 | rs9995943 | 0.51 | 0.048174697 | 0.0864 |
| hCV11503414 | rs2066865 | hCV11853354 | rs10030235 | 0.51 | 0.048174697 | 0.0832 |
| hCV11503414 | rs2066865 | hCV11853357 | rs10033383 | 0.51 | 0.048174697 | 0.1091 |
| hCV11503414 | rs2066865 | hCV11853358 | rs10000511 | 0.51 | 0.048174697 | 0.0909 |
| hCV11503414 | rs2066865 | hCV11853362 | rs4696572 | 0.51 | 0.048174697 | 0.1012 |
| hCV11503414 | rs2066865 | hCV11853363 | rs4696573 | 0.51 | 0.048174697 | 0.0905 |
| hCV11503414 | rs2066865 | hCV11853373 | rs1907155 | 0.51 | 0.048174697 | 0.0947 |
| hCV11503414 | rs2066865 | hCV11853378 | rs1907154 | 0.51 | 0.048174697 | 0.163 |
| hCV11503414 | rs2066865 | hCV11853384 | rs12646456 | 0.51 | 0.048174697 | 0.163 |
| hCV11503414 | rs2066865 | hCV11853387 | rs1490683 | 0.51 | 0.048174697 | 0.217 |
| hCV11503414 | rs2066865 | hCV11853415 | rs1490653 | 0.51 | 0.048174697 | 0.0593 |
| hCV11503414 | rs2066865 | hCV11853416 | rs4346631 | 0.51 | 0.048174697 | 0.0664 |
| hCV11503414 | rs2066865 | hCV11853418 | rs12501998 | 0.51 | 0.048174697 | 0.0542 |
| hCV11503414 | rs2066865 | hCV11853419 | rs13151559 | 0.51 | 0.048174697 | 0.0542 |
| hCV11503414 | rs2066865 | hCV11853423 | rs3857093 | 0.51 | 0.048174697 | 0.0542 |
| hCV11503414 | rs2066865 | hCV11853424 | rs871541 | 0.51 | 0.048174697 | 0.0542 |
| hCV11503414 | rs2066865 | hCV11853483 | rs12644950 | 0.51 | 0.048174697 | 1 |
| hCV11503414 | rs2066865 | hCV11853489 | rs7681423 | 0.51 | 0.048174697 | 1 |
| hCV11503414 | rs2066865 | hCV11853496 | rs7654093 | 0.51 | 0.048174697 | 1 |
| hCV11503414 | rs2066865 | hCV11853631 | rs12651106 | 0.51 | 0.048174697 | 0.1612 |
| hCV11503414 | rs2066865 | hCV11853650 | rs9307922 | 0.51 | 0.048174697 | 0.1074 |
| hCV11503414 | rs2066865 | hCV1190562 | rs1490684 | 0.51 | 0.048174697 | 0.0947 |
| hCV11503414 | rs2066865 | hCV1190563 | rs4696565 | 0.51 | 0.048174697 | 0.114 |
| hCV11503414 | rs2066865 | hCV1190567 | rs4696210 | 0.51 | 0.048174697 | 0.114 |
| hCV11503414 | rs2066865 | hCV1190572 | rs1032335 | 0.51 | 0.048174697 | 0.163 |
| hCV11503414 | rs2066865 | hCV1190580 | rs9998926 | 0.51 | 0.048174697 | 0.0874 |
| hCV11503414 | rs2066865 | hCV1190581 | rs6856249 | 0.51 | 0.048174697 | 0.114 |
| hCV11503414 | rs2066865 | hCV1190582 | rs10013533 | 0.51 | 0.048174697 | 0.114 |
| hCV11503414 | rs2066865 | hCV15860433 | rs2070006 | 0.51 | 0.048174697 | 0.4534 |
| hCV11503414 | rs2066865 | hCV176753 | rs2404478 | 0.51 | 0.048174697 | 0.0542 |
| hCV11503414 | rs2066865 | hCV21680 | rs7666020 | 0.51 | 0.048174697 | 0.153 |
| hCV11503414 | rs2066865 | hCV21681 | rs6536018 | 0.51 | 0.048174697 | 0.3185 |
| hCV11503414 | rs2066865 | hCV22273499 | rs7668014 | 0.51 | 0.048174697 | 0.0903 |
| hCV11503414 | rs2066865 | hCV22274180 | rs11935584 | 0.51 | 0.048174697 | 0.1032 |
| hCV11503414 | rs2066865 | hCV229029 | rs13103792 | 0.51 | 0.048174697 | 0.0486 |
| hCV11503414 | rs2066865 | hCV2407252 | rs149225 | 0.51 | 0.048174697 | 0.1 |
| hCV11503414 | rs2066865 | hCV2407354 | rs276166 | 0.51 | 0.048174697 | 0.0534 |
| hCV11503414 | rs2066865 | hCV24834 | rs4235247 | 0.51 | 0.048174697 | 0.4263 |
| hCV11503414 | rs2066865 | hCV25610762 | rs7668818 | 0.51 | 0.048174697 | 0.0707 |
| hCV11503414 | rs2066865 | hCV26019871 | rs4547780 | 0.51 | 0.048174697 | 0.3146 |
| hCV11503414 | rs2066865 | hCV26024202 | rs11731813 | 0.51 | 0.048174697 | 0.2237 |
| hCV11503414 | rs2066865 | hCV26024285 | rs11726919 | 0.51 | 0.048174697 | 0.1063 |
| hCV11503414 | rs2066865 | hCV26024286 | rs11726850 | 0.51 | 0.048174697 | 0.1063 |
| hCV11503414 | rs2066865 | hCV26024287 | rs7666541 | 0.51 | 0.048174697 | 0.1357 |
| hCV11503414 | rs2066865 | hCV26024294 | rs11731663 | 0.51 | 0.048174697 | 0.1063 |
| hCV11503414 | rs2066865 | hCV265748 | rs12500118 | 0.51 | 0.048174697 | 0.1669 |

# TABLE 3, page 2 of 71

| Interrogated SNP | Interrogated rs | LD SNP | LD SNP rs | Power | Threshold r² | r² |
|---|---|---|---|---|---|---|
| hCV11503414 | rs2066865 | hCV27020269 | rs7659613 | 0.51 | 0.048174697 | 0.5249 |
| hCV11503414 | rs2066865 | hCV27020277 | rs6825454 | 0.51 | 0.048174697 | 0.8713 |
| hCV11503414 | rs2066865 | hCV27020280 | rs4463047 | 0.51 | 0.048174697 | 0.2252 |
| hCV11503414 | rs2066865 | hCV27020304 | rs13101534 | 0.51 | 0.048174697 | 0.1091 |
| hCV11503414 | rs2066865 | hCV27313130 | rs4634202 | 0.51 | 0.048174697 | 0.103 |
| hCV11503414 | rs2066865 | hCV27479909 | rs3775785 | 0.51 | 0.048174697 | 0.1072 |
| hCV11503414 | rs2066865 | hCV27905214 | rs4323084 | 0.51 | 0.048174697 | 0.2956 |
| hCV11503414 | rs2066865 | hCV27907560 | rs4696576 | 0.51 | 0.048174697 | 0.135 |
| hCV11503414 | rs2066865 | hCV27937396 | rs4634201 | 0.51 | 0.048174697 | 0.4298 |
| hCV11503414 | rs2066865 | hCV286004 | rs1118824 | 0.51 | 0.048174697 | 0.1213 |
| hCV11503414 | rs2066865 | hCV2891425 | rs1948714 | 0.51 | 0.048174697 | 0.1065 |
| hCV11503414 | rs2066865 | hCV2891532 | rs13110294 | 0.51 | 0.048174697 | 0.1006 |
| hCV11503414 | rs2066865 | hCV2892850 | rs10050268 | 0.51 | 0.048174697 | 0.0552 |
| hCV11503414 | rs2066865 | hCV2892855 | rs6536024 | 0.51 | 0.048174697 | 0.2222 |
| hCV11503414 | rs2066865 | hCV2892858 | rs12648395 | 0.51 | 0.048174697 | 0.1213 |
| hCV11503414 | rs2066865 | hCV2892859 | rs13130318 | 0.51 | 0.048174697 | 0.859 |
| hCV11503414 | rs2066865 | hCV2892863 | rs1049636 | 0.51 | 0.048174697 | 0.1213 |
| hCV11503414 | rs2066865 | hCV2892869 | rs13109457 | 0.51 | 0.048174697 | 0.955 |
| hCV11503414 | rs2066865 | hCV2892870 | rs2070011 | 0.51 | 0.048174697 | 0.439 |
| hCV11503414 | rs2066865 | hCV2892876 | rs2070018 | 0.51 | 0.048174697 | 0.0566 |
| hCV11503414 | rs2066865 | hCV2892877 | rs6050 | 0.51 | 0.048174697 | 0.873 |
| hCV11503414 | rs2066865 | hCV2892893 | rs12648258 | 0.51 | 0.048174697 | 0.4009 |
| hCV11503414 | rs2066865 | hCV2892895 | rs12641958 | 0.51 | 0.048174697 | 0.0903 |
| hCV11503414 | rs2066865 | hCV2892896 | rs11940724 | 0.51 | 0.048174697 | 0.0903 |
| hCV11503414 | rs2066865 | hCV2892899 | rs7680155 | 0.51 | 0.048174697 | 0.1032 |
| hCV11503414 | rs2066865 | hCV2892905 | rs12642770 | 0.51 | 0.048174697 | 0.3619 |
| hCV11503414 | rs2066865 | hCV2892918 | rs12511469 | 0.51 | 0.048174697 | 0.3888 |
| hCV11503414 | rs2066865 | hCV2892923 | rs13435192 | 0.51 | 0.048174697 | 0.1113 |
| hCV11503414 | rs2066865 | hCV2892924 | rs13435101 | 0.51 | 0.048174697 | 0.1105 |
| hCV11503414 | rs2066865 | hCV2892925 | rs7689945 | 0.51 | 0.048174697 | 0.1063 |
| hCV11503414 | rs2066865 | hCV2892926 | rs7662567 | 0.51 | 0.048174697 | 0.3986 |
| hCV11503414 | rs2066865 | hCV2892927 | rs13123551 | 0.51 | 0.048174697 | 0.1327 |
| hCV11503414 | rs2066865 | hCV2892928 | rs13147579 | 0.51 | 0.048174697 | 0.4128 |
| hCV11503414 | rs2066865 | hCV28953838 | rs7690851 | 0.51 | 0.048174697 | 0.3221 |
| hCV11503414 | rs2066865 | hCV28953840 | rs6536017 | 0.51 | 0.048174697 | 0.1155 |
| hCV11503414 | rs2066865 | hCV28954780 | rs7656522 | 0.51 | 0.048174697 | 0.0537 |
| hCV11503414 | rs2066865 | hCV28966638 | rs7676857 | 0.51 | 0.048174697 | 0.1625 |
| hCV11503414 | rs2066865 | hCV29317506 | rs7686002 | 0.51 | 0.048174697 | 0.0551 |
| hCV11503414 | rs2066865 | hCV29420822 | rs4642230 | 0.51 | 0.048174697 | 0.4837 |
| hCV11503414 | rs2066865 | hCV29420827 | rs7654425 | 0.51 | 0.048174697 | 0.0903 |
| hCV11503414 | rs2066865 | hCV29420828 | rs7660120 | 0.51 | 0.048174697 | 0.0796 |
| hCV11503414 | rs2066865 | hCV29570696 | rs9997519 | 0.51 | 0.048174697 | 0.0523 |
| hCV11503414 | rs2066865 | hCV29582612 | rs4550901 | 0.51 | 0.048174697 | 0.0566 |
| hCV11503414 | rs2066865 | hCV29751345 | rs6811271 | 0.51 | 0.048174697 | 0.108 |
| hCV11503414 | rs2066865 | hCV29983641 | rs10008078 | 0.51 | 0.048174697 | 0.461 |
| hCV11503414 | rs2066865 | hCV30004073 | rs6832957 | 0.51 | 0.048174697 | 0.049 |
| hCV11503414 | rs2066865 | hCV30562176 | rs9284660 | 0.51 | 0.048174697 | 0.1006 |
| hCV11503414 | rs2066865 | hCV30679139 | rs13139082 | 0.51 | 0.048174697 | 0.0593 |
| hCV11503414 | rs2066865 | hCV30679140 | rs13112066 | 0.51 | 0.048174697 | 0.0499 |
| hCV11503414 | rs2066865 | hCV30679141 | rs13111621 | 0.51 | 0.048174697 | 0.0629 |
| hCV11503414 | rs2066865 | hCV30679164 | rs12649437 | 0.51 | 0.048174697 | 0.1051 |
| hCV11503414 | rs2066865 | hCV30679170 | rs13148992 | 0.51 | 0.048174697 | 0.2324 |
| hCV11503414 | rs2066865 | hCV30679242 | rs4235243 | 0.51 | 0.048174697 | 0.1248 |
| hCV11503414 | rs2066865 | hCV30679244 | rs4575978 | 0.51 | 0.048174697 | 0.1063 |
| hCV11503414 | rs2066865 | hCV30679245 | rs4386583 | 0.51 | 0.048174697 | 0.1063 |

# TABLE 3, page 3 of 71

| Interrogated SNP | Interrogated rs | LD SNP | LD SNP rs | Power | Threshold r² | r² |
|---|---|---|---|---|---|---|
| hCV11503414 | rs2066865 | hCV30711231 | rs12642469 | 0.51 | 0.048174697 | 0.461 |
| hCV11503414 | rs2066865 | hCV31863942 | rs13101382 | 0.51 | 0.048174697 | 0.1052 |
| hCV11503414 | rs2066865 | hCV31863979 | rs12186294 | 0.51 | 0.048174697 | 0.2778 |
| hCV11503414 | rs2066865 | hCV31863982 | rs7659024 | 0.51 | 0.048174697 | 1 |
| hCV11503414 | rs2066865 | hCV31863989 | rs4308349 | 0.51 | 0.048174697 | 0.0513 |
| hCV11503414 | rs2066865 | hCV31863993 | rs7673587 | 0.51 | 0.048174697 | 0.1032 |
| hCV11503414 | rs2066865 | hCV32212659 | rs4622984 | 0.51 | 0.048174697 | 0.1879 |
| hCV11503414 | rs2066865 | hCV32212662 | rs11099958 | 0.51 | 0.048174697 | 0.0527 |
| hCV11503414 | rs2066865 | hCV32212663 | rs7670827 | 0.51 | 0.048174697 | 0.0974 |
| hCV11503414 | rs2066865 | hCV32212664 | rs12642646 | 0.51 | 0.048174697 | 0.0491 |
| hCV11503414 | rs2066865 | hCV32212669 | rs12649647 | 0.51 | 0.048174697 | 0.0577 |
| hCV11503414 | rs2066865 | hCV354895 | rs11737226 | 0.51 | 0.048174697 | 0.2322 |
| hCV11503414 | rs2066865 | hCV354896 | rs7690972 | 0.51 | 0.048174697 | 0.2322 |
| hCV11503414 | rs2066865 | hCV36809 | rs10517590 | 0.51 | 0.048174697 | 0.133 |
| hCV11503414 | rs2066865 | hCV400532 | rs11099956 | 0.51 | 0.048174697 | 0.1095 |
| hCV11503414 | rs2066865 | hCV426162 | rs10857275 | 0.51 | 0.048174697 | 0.1132 |
| hCV11503414 | rs2066865 | hCV426165 | rs990185 | 0.51 | 0.048174697 | 0.1074 |
| hCV11503414 | rs2066865 | hCV426167 | rs1388087 | 0.51 | 0.048174697 | 0.0905 |
| hCV11503414 | rs2066865 | hCV426168 | rs1388088 | 0.51 | 0.048174697 | 0.114 |
| hCV11503414 | rs2066865 | hCV426169 | rs1388066 | 0.51 | 0.048174697 | 0.1336 |
| hCV11503414 | rs2066865 | hCV426170 | rs1388067 | 0.51 | 0.048174697 | 0.114 |
| hCV11503414 | rs2066865 | hCV426172 | rs7670027 | 0.51 | 0.048174697 | 0.1443 |
| hCV11503414 | rs2066865 | hCV426173 | rs12504201 | 0.51 | 0.048174697 | 0.2207 |
| hCV11503414 | rs2066865 | hCV426175 | rs9884952 | 0.51 | 0.048174697 | 0.163 |
| hCV11503414 | rs2066865 | hCV426176 | rs9884775 | 0.51 | 0.048174697 | 0.163 |
| hCV11503414 | rs2066865 | hCV426178 | rs9884570 | 0.51 | 0.048174697 | 0.1519 |
| hCV11503414 | rs2066865 | hCV426181 | rs11099955 | 0.51 | 0.048174697 | 0.163 |
| hCV11503414 | rs2066865 | hCV426182 | rs10014536 | 0.51 | 0.048174697 | 0.1769 |
| hCV11503414 | rs2066865 | hCV426183 | rs10014635 | 0.51 | 0.048174697 | 0.1772 |
| hCV11503414 | rs2066865 | hCV426184 | rs1032336 | 0.51 | 0.048174697 | 0.163 |
| hCV11503414 | rs2066865 | hCV437164 | rs7685964 | 0.51 | 0.048174697 | 0.1071 |
| hCV11503414 | rs2066865 | hCV470979 | rs1490672 | 0.51 | 0.048174697 | 0.2211 |
| hCV11503414 | rs2066865 | hCV501682 | rs4403033 | 0.51 | 0.048174697 | 0.1063 |
| hCV11503414 | rs2066865 | hCV501683 | rs4312742 | 0.51 | 0.048174697 | 0.1248 |
| hCV11503414 | rs2066865 | hCV501686 | rs4327464 | 0.51 | 0.048174697 | 0.1026 |
| hCV11503414 | rs2066865 | hCV7429674 | rs871540 | 0.51 | 0.048174697 | 0.0542 |
| hCV11503414 | rs2066865 | hCV7429780 | rs1800792 | 0.51 | 0.048174697 | 0.2745 |
| hCV11503414 | rs2066865 | hCV7429782 | rs1118823 | 0.51 | 0.048174697 | 0.1185 |
| hCV11503414 | rs2066865 | hCV7429783 | rs1044291 | 0.51 | 0.048174697 | 0.0903 |
| hCV11503414 | rs2066865 | hCV7429793 | rs1025154 | 0.51 | 0.048174697 | 0.461 |
| hCV11503414 | rs2066865 | hCV7430148 | rs1490685 | 0.51 | 0.048174697 | 0.163 |
| hCV11503414 | rs2066865 | hCV7430149 | rs1490649 | 0.51 | 0.048174697 | 0.1131 |
| hCV11503414 | rs2066865 | hCV7430150 | rs1490648 | 0.51 | 0.048174697 | 0.1182 |
| hCV11503414 | rs2066865 | hCV7430152 | rs1490656 | 0.51 | 0.048174697 | 0.1029 |
| hCV11503414 | rs2066865 | hCV7430153 | rs1388077 | 0.51 | 0.048174697 | 0.114 |
| hCV11503414 | rs2066865 | hCV7430158 | rs1466662 | 0.51 | 0.048174697 | 0.1669 |
| hCV11503414 | rs2066865 | hCV8938834 | rs1500372 | 0.51 | 0.048174697 | 0.076 |
| hCV11503414 | rs2066865 | hCV8938838 | rs1392546 | 0.51 | 0.048174697 | 0.076 |
| hCV11503414 | rs2066865 | hCV9317142 | rs12186175 | 0.51 | 0.048174697 | 0.1052 |
| hCV11503414 | rs2066865 | hCV99436 | rs10015747 | 0.51 | 0.048174697 | 0.1308 |
| hCV11503414 | rs2066865 | hDV70934991 | rs17301943 | 0.51 | 0.048174697 | 0.0542 |
| hCV11503414 | rs2066865 | hDV70945235 | rs17373860 | 0.51 | 0.048174697 | 0.16 |
| hCV11503414 | rs2066865 | hDV77232287 | rs7666918 | 0.51 | 0.048174697 | 0.0903 |
| hCV11503414 | rs2066865 | hDV96226316 | rs6834312 | 0.51 | 0.048174697 | 0.1334 |
| hCV11503469 | rs2066854 | hCV11281035 | rs4583739 | 0.51 | 0.048166678 | 0.094 |

| Interrogated SNP | Interrogated rs | LD SNP | LD SNP rs | Power | Threshold r² | r² |
|---|---|---|---|---|---|---|
| hCV11503469 | rs2066854 | hCV11503378 | rs1490655 | 0.51 | 0.048166678 | 0.068 |
| hCV11503469 | rs2066854 | hCV11503379 | rs1490654 | 0.51 | 0.048166678 | 0.0512 |
| hCV11503469 | rs2066854 | hCV11503382 | rs1873369 | 0.51 | 0.048166678 | 0.1718 |
| hCV11503469 | rs2066854 | hCV11503414 | rs2066865 | 0.51 | 0.048166678 | 0.9559 |
| hCV11503469 | rs2066854 | hCV11503416 | rs2066864 | 0.51 | 0.048166678 | 0.9579 |
| hCV11503469 | rs2066854 | hCV11503431 | rs2066861 | 0.51 | 0.048166678 | 0.9559 |
| hCV11503469 | rs2066854 | hCV11503470 | rs1800788 | 0.51 | 0.048166678 | 0.3765 |
| hCV11503469 | rs2066854 | hCV11853342 | rs7660343 | 0.51 | 0.048166678 | 0.0674 |
| hCV11503469 | rs2066854 | hCV11853353 | rs9995943 | 0.51 | 0.048166678 | 0.0981 |
| hCV11503469 | rs2066854 | hCV11853354 | rs10030235 | 0.51 | 0.048166678 | 0.0868 |
| hCV11503469 | rs2066854 | hCV11853357 | rs10033383 | 0.51 | 0.048166678 | 0.0595 |
| hCV11503469 | rs2066854 | hCV11853362 | rs4696572 | 0.51 | 0.048166678 | 0.1483 |
| hCV11503469 | rs2066854 | hCV11853363 | rs4696573 | 0.51 | 0.048166678 | 0.0981 |
| hCV11503469 | rs2066854 | hCV11853373 | rs1907155 | 0.51 | 0.048166678 | 0.1398 |
| hCV11503469 | rs2066854 | hCV11853378 | rs1907154 | 0.51 | 0.048166678 | 0.0869 |
| hCV11503469 | rs2066854 | hCV11853384 | rs12646456 | 0.51 | 0.048166678 | 0.0869 |
| hCV11503469 | rs2066854 | hCV11853387 | rs1490683 | 0.51 | 0.048166678 | 0.1451 |
| hCV11503469 | rs2066854 | hCV11853416 | rs4346631 | 0.51 | 0.048166678 | 0.05 |
| hCV11503469 | rs2066854 | hCV11853418 | rs12501998 | 0.51 | 0.048166678 | 0.0786 |
| hCV11503469 | rs2066854 | hCV11853419 | rs13151559 | 0.51 | 0.048166678 | 0.0786 |
| hCV11503469 | rs2066854 | hCV11853423 | rs3857093 | 0.51 | 0.048166678 | 0.0786 |
| hCV11503469 | rs2066854 | hCV11853424 | rs871541 | 0.51 | 0.048166678 | 0.0786 |
| hCV11503469 | rs2066854 | hCV11853483 | rs12644950 | 0.51 | 0.048166678 | 0.9545 |
| hCV11503469 | rs2066854 | hCV11853489 | rs7681423 | 0.51 | 0.048166678 | 0.9579 |
| hCV11503469 | rs2066854 | hCV11853496 | rs7654093 | 0.51 | 0.048166678 | 0.9559 |
| hCV11503469 | rs2066854 | hCV11853631 | rs12651106 | 0.51 | 0.048166678 | 0.1768 |
| hCV11503469 | rs2066854 | hCV1190562 | rs1490684 | 0.51 | 0.048166678 | 0.1398 |
| hCV11503469 | rs2066854 | hCV1190563 | rs4696565 | 0.51 | 0.048166678 | 0.063 |
| hCV11503469 | rs2066854 | hCV1190567 | rs4696210 | 0.51 | 0.048166678 | 0.063 |
| hCV11503469 | rs2066854 | hCV1190572 | rs1032335 | 0.51 | 0.048166678 | 0.0869 |
| hCV11503469 | rs2066854 | hCV1190580 | rs9998926 | 0.51 | 0.048166678 | 0.0915 |
| hCV11503469 | rs2066854 | hCV1190581 | rs6856249 | 0.51 | 0.048166678 | 0.063 |
| hCV11503469 | rs2066854 | hCV1190582 | rs10013533 | 0.51 | 0.048166678 | 0.063 |
| hCV11503469 | rs2066854 | hCV15860433 | rs2070006 | 0.51 | 0.048166678 | 0.5293 |
| hCV11503469 | rs2066854 | hCV15971616 | rs2227421 | 0.51 | 0.048166678 | 0.1143 |
| hCV11503469 | rs2066854 | hCV176753 | rs2404478 | 0.51 | 0.048166678 | 0.0786 |
| hCV11503469 | rs2066854 | hCV21680 | rs7666020 | 0.51 | 0.048166678 | 0.1247 |
| hCV11503469 | rs2066854 | hCV21681 | rs6536018 | 0.51 | 0.048166678 | 0.2928 |
| hCV11503469 | rs2066854 | hCV22273499 | rs7668014 | 0.51 | 0.048166678 | 0.1071 |
| hCV11503469 | rs2066854 | hCV22274180 | rs11935584 | 0.51 | 0.048166678 | 0.1125 |
| hCV11503469 | rs2066854 | hCV229029 | rs13103792 | 0.51 | 0.048166678 | 0.062 |
| hCV11503469 | rs2066854 | hCV2407223 | rs156502 | 0.51 | 0.048166678 | 0.0621 |
| hCV11503469 | rs2066854 | hCV2407232 | rs156550 | 0.51 | 0.048166678 | 0.0563 |
| hCV11503469 | rs2066854 | hCV2407238 | rs156543 | 0.51 | 0.048166678 | 0.0615 |
| hCV11503469 | rs2066854 | hCV2407252 | rs149225 | 0.51 | 0.048166678 | 0.105 |
| hCV11503469 | rs2066854 | hCV24834 | rs4235247 | 0.51 | 0.048166678 | 0.4128 |
| hCV11503469 | rs2066854 | hCV25610762 | rs7668818 | 0.51 | 0.048166678 | 0.0634 |
| hCV11503469 | rs2066854 | hCV26019871 | rs4547780 | 0.51 | 0.048166678 | 0.3094 |
| hCV11503469 | rs2066854 | hCV26024202 | rs11731813 | 0.51 | 0.048166678 | 0.225 |
| hCV11503469 | rs2066854 | hCV26024287 | rs7666541 | 0.51 | 0.048166678 | 0.1353 |
| hCV11503469 | rs2066854 | hCV26024295 | rs12643125 | 0.51 | 0.048166678 | 0.1125 |
| hCV11503469 | rs2066854 | hCV265748 | rs12500118 | 0.51 | 0.048166678 | 0.1103 |
| hCV11503469 | rs2066854 | hCV27020184 | rs47379 | 0.51 | 0.048166678 | 0.0776 |
| hCV11503469 | rs2066854 | hCV27020269 | rs7659613 | 0.51 | 0.048166678 | 0.5455 |
| hCV11503469 | rs2066854 | hCV27020277 | rs6825454 | 0.51 | 0.048166678 | 0.8694 |

## TABLE 3, page 5 of 71

| Interrogated SNP | Interrogated rs | LD SNP | LD SNP rs | Power | Threshold $r^2$ | $r^2$ |
|---|---|---|---|---|---|---|
| hCV11503469 | rs2066854 | hCV27020280 | rs4463047 | 0.51 | 0.048166678 | 0.2409 |
| hCV11503469 | rs2066854 | hCV27020284 | rs1846707 | 0.51 | 0.048166678 | 0.1139 |
| hCV11503469 | rs2066854 | hCV27313130 | rs4634202 | 0.51 | 0.048166678 | 0.1555 |
| hCV11503469 | rs2066854 | hCV27479909 | rs3775785 | 0.51 | 0.048166678 | 0.0578 |
| hCV11503469 | rs2066854 | hCV27905214 | rs4323084 | 0.51 | 0.048166678 | 0.325 |
| hCV11503469 | rs2066854 | hCV27907560 | rs4696576 | 0.51 | 0.048166678 | 0.0999 |
| hCV11503469 | rs2066854 | hCV27937396 | rs4634201 | 0.51 | 0.048166678 | 0.4472 |
| hCV11503469 | rs2066854 | hCV286004 | rs1118824 | 0.51 | 0.048166678 | 0.1531 |
| hCV11503469 | rs2066854 | hCV2891496 | rs156584 | 0.51 | 0.048166678 | 0.0621 |
| hCV11503469 | rs2066854 | hCV2891515 | rs11940892 | 0.51 | 0.048166678 | 0.0615 |
| hCV11503469 | rs2066854 | hCV2891530 | rs7662464 | 0.51 | 0.048166678 | 0.0615 |
| hCV11503469 | rs2066854 | hCV2891532 | rs13110294 | 0.51 | 0.048166678 | 0.0626 |
| hCV11503469 | rs2066854 | hCV2891552 | rs1876031 | 0.51 | 0.048166678 | 0.1011 |
| hCV11503469 | rs2066854 | hCV2891554 | rs12501328 | 0.51 | 0.048166678 | 0.059 |
| hCV11503469 | rs2066854 | hCV2892850 | rs10050268 | 0.51 | 0.048166678 | 0.0638 |
| hCV11503469 | rs2066854 | hCV2892855 | rs6536024 | 0.51 | 0.048166678 | 0.2667 |
| hCV11503469 | rs2066854 | hCV2892858 | rs12648395 | 0.51 | 0.048166678 | 0.1531 |
| hCV11503469 | rs2066854 | hCV2892859 | rs13130318 | 0.51 | 0.048166678 | 0.8253 |
| hCV11503469 | rs2066854 | hCV2892863 | rs1049636 | 0.51 | 0.048166678 | 0.1531 |
| hCV11503469 | rs2066854 | hCV2892869 | rs13109457 | 0.51 | 0.048166678 | 0.9149 |
| hCV11503469 | rs2066854 | hCV2892870 | rs2070011 | 0.51 | 0.048166678 | 0.5068 |
| hCV11503469 | rs2066854 | hCV2892877 | rs6050 | 0.51 | 0.048166678 | 0.8287 |
| hCV11503469 | rs2066854 | hCV2892878 | rs2070022 | 0.51 | 0.048166678 | 0.0592 |
| hCV11503469 | rs2066854 | hCV2892889 | rs2227412 | 0.51 | 0.048166678 | 0.0547 |
| hCV11503469 | rs2066854 | hCV2892893 | rs12648258 | 0.51 | 0.048166678 | 0.4044 |
| hCV11503469 | rs2066854 | hCV2892895 | rs12641958 | 0.51 | 0.048166678 | 0.1071 |
| hCV11503469 | rs2066854 | hCV2892896 | rs11940724 | 0.51 | 0.048166678 | 0.1071 |
| hCV11503469 | rs2066854 | hCV2892899 | rs7680155 | 0.51 | 0.048166678 | 0.1125 |
| hCV11503469 | rs2066854 | hCV2892905 | rs12642770 | 0.51 | 0.048166678 | 0.3381 |
| hCV11503469 | rs2066854 | hCV2892918 | rs12511469 | 0.51 | 0.048166678 | 0.3613 |
| hCV11503469 | rs2066854 | hCV2892923 | rs13435192 | 0.51 | 0.048166678 | 0.1375 |
| hCV11503469 | rs2066854 | hCV2892924 | rs13435101 | 0.51 | 0.048166678 | 0.1375 |
| hCV11503469 | rs2066854 | hCV2892925 | rs7689945 | 0.51 | 0.048166678 | 0.1375 |
| hCV11503469 | rs2066854 | hCV2892926 | rs7662567 | 0.51 | 0.048166678 | 0.3671 |
| hCV11503469 | rs2066854 | hCV2892927 | rs13123551 | 0.51 | 0.048166678 | 0.1434 |
| hCV11503469 | rs2066854 | hCV2892928 | rs13147579 | 0.51 | 0.048166678 | 0.3836 |
| hCV11503469 | rs2066854 | hCV28953838 | rs7690851 | 0.51 | 0.048166678 | 0.3242 |
| hCV11503469 | rs2066854 | hCV28953840 | rs6536017 | 0.51 | 0.048166678 | 0.1025 |
| hCV11503469 | rs2066854 | hCV28954780 | rs7656522 | 0.51 | 0.048166678 | 0.0782 |
| hCV11503469 | rs2066854 | hCV28954790 | rs7662783 | 0.51 | 0.048166678 | 0.0496 |
| hCV11503469 | rs2066854 | hCV28954801 | rs4447837 | 0.51 | 0.048166678 | 0.062 |
| hCV11503469 | rs2066854 | hCV28966638 | rs7676857 | 0.51 | 0.048166678 | 0.1179 |
| hCV11503469 | rs2066854 | hCV29420822 | rs4642230 | 0.51 | 0.048166678 | 0.4022 |
| hCV11503469 | rs2066854 | hCV29420827 | rs7654425 | 0.51 | 0.048166678 | 0.1071 |
| hCV11503469 | rs2066854 | hCV29420828 | rs7660120 | 0.51 | 0.048166678 | 0.0906 |
| hCV11503469 | rs2066854 | hCV29570696 | rs9997519 | 0.51 | 0.048166678 | 0.0519 |
| hCV11503469 | rs2066854 | hCV29636755 | rs10517602 | 0.51 | 0.048166678 | 0.0706 |
| hCV11503469 | rs2066854 | hCV29751345 | rs6811271 | 0.51 | 0.048166678 | 0.1681 |
| hCV11503469 | rs2066854 | hCV29983641 | rs10008078 | 0.51 | 0.048166678 | 0.3893 |
| hCV11503469 | rs2066854 | hCV30562176 | rs9284660 | 0.51 | 0.048166678 | 0.0785 |
| hCV11503469 | rs2066854 | hCV30679139 | rs13139082 | 0.51 | 0.048166678 | 0.0616 |
| hCV11503469 | rs2066854 | hCV30679140 | rs13112066 | 0.51 | 0.048166678 | 0.0674 |
| hCV11503469 | rs2066854 | hCV30679164 | rs12649437 | 0.51 | 0.048166678 | 0.0849 |
| hCV11503469 | rs2066854 | hCV30679170 | rs13148992 | 0.51 | 0.048166678 | 0.2399 |
| hCV11503469 | rs2066854 | hCV30711231 | rs12642469 | 0.51 | 0.048166678 | 0.3893 |

# TABLE 3, page 6 of 71

| Interrogated SNP | Interrogated rs | LD SNP | LD SNP rs | Power | Threshold r² | r² |
|---|---|---|---|---|---|---|
| hCV11503469 | rs2066854 | hCV31863937 | rs12507608 | 0.51 | 0.048166678 | 0.0706 |
| hCV11503469 | rs2066854 | hCV31863979 | rs12186294 | 0.51 | 0.048166678 | 0.3086 |
| hCV11503469 | rs2066854 | hCV31863982 | rs7659024 | 0.51 | 0.048166678 | 0.9559 |
| hCV11503469 | rs2066854 | hCV31863993 | rs7673587 | 0.51 | 0.048166678 | 0.1125 |
| hCV11503469 | rs2066854 | hCV32212658 | rs11099959 | 0.51 | 0.048166678 | 0.0536 |
| hCV11503469 | rs2066854 | hCV32212659 | rs4622984 | 0.51 | 0.048166678 | 0.195 |
| hCV11503469 | rs2066854 | hCV32212663 | rs7670827 | 0.51 | 0.048166678 | 0.1002 |
| hCV11503469 | rs2066854 | hCV32212664 | rs12642646 | 0.51 | 0.048166678 | 0.0849 |
| hCV11503469 | rs2066854 | hCV32287640 | rs4367156 | 0.51 | 0.048166678 | 0.062 |
| hCV11503469 | rs2066854 | hCV354895 | rs11737226 | 0.51 | 0.048166678 | 0.2251 |
| hCV11503469 | rs2066854 | hCV354896 | rs7690972 | 0.51 | 0.048166678 | 0.2251 |
| hCV11503469 | rs2066854 | hCV37878 | rs4235241 | 0.51 | 0.048166678 | 0.1157 |
| hCV11503469 | rs2066854 | hCV400532 | rs11099956 | 0.51 | 0.048166678 | 0.0951 |
| hCV11503469 | rs2066854 | hCV426167 | rs1388087 | 0.51 | 0.048166678 | 0.0981 |
| hCV11503469 | rs2066854 | hCV426168 | rs1388088 | 0.51 | 0.048166678 | 0.063 |
| hCV11503469 | rs2066854 | hCV426169 | rs1388066 | 0.51 | 0.048166678 | 0.0794 |
| hCV11503469 | rs2066854 | hCV426170 | rs1388067 | 0.51 | 0.048166678 | 0.063 |
| hCV11503469 | rs2066854 | hCV426172 | rs7670027 | 0.51 | 0.048166678 | 0.083 |
| hCV11503469 | rs2066854 | hCV426173 | rs12504201 | 0.51 | 0.048166678 | 0.1597 |
| hCV11503469 | rs2066854 | hCV426175 | rs9884952 | 0.51 | 0.048166678 | 0.0816 |
| hCV11503469 | rs2066854 | hCV426176 | rs9884775 | 0.51 | 0.048166678 | 0.0869 |
| hCV11503469 | rs2066854 | hCV426178 | rs9884570 | 0.51 | 0.048166678 | 0.078 |
| hCV11503469 | rs2066854 | hCV426181 | rs11099955 | 0.51 | 0.048166678 | 0.0869 |
| hCV11503469 | rs2066854 | hCV426182 | rs10014536 | 0.51 | 0.048166678 | 0.1101 |
| hCV11503469 | rs2066854 | hCV426183 | rs10014635 | 0.51 | 0.048166678 | 0.0914 |
| hCV11503469 | rs2066854 | hCV426184 | rs1032336 | 0.51 | 0.048166678 | 0.0869 |
| hCV11503469 | rs2066854 | hCV437164 | rs7685964 | 0.51 | 0.048166678 | 0.0537 |
| hCV11503469 | rs2066854 | hCV470979 | rs1490672 | 0.51 | 0.048166678 | 0.2217 |
| hCV11503469 | rs2066854 | hCV489970 | rs11734901 | 0.51 | 0.048166678 | 0.1235 |
| hCV11503469 | rs2066854 | hCV501681 | rs4076040 | 0.51 | 0.048166678 | 0.1157 |
| hCV11503469 | rs2066854 | hCV7429674 | rs871540 | 0.51 | 0.048166678 | 0.0786 |
| hCV11503469 | rs2066854 | hCV7429780 | rs1800792 | 0.51 | 0.048166678 | 0.3086 |
| hCV11503469 | rs2066854 | hCV7429782 | rs1118823 | 0.51 | 0.048166678 | 0.1531 |
| hCV11503469 | rs2066854 | hCV7429783 | rs1044291 | 0.51 | 0.048166678 | 0.1141 |
| hCV11503469 | rs2066854 | hCV7429793 | rs1025154 | 0.51 | 0.048166678 | 0.3893 |
| hCV11503469 | rs2066854 | hCV7430148 | rs1490685 | 0.51 | 0.048166678 | 0.0869 |
| hCV11503469 | rs2066854 | hCV7430149 | rs1490649 | 0.51 | 0.048166678 | 0.0623 |
| hCV11503469 | rs2066854 | hCV7430150 | rs1490648 | 0.51 | 0.048166678 | 0.0661 |
| hCV11503469 | rs2066854 | hCV7430152 | rs1490656 | 0.51 | 0.048166678 | 0.0539 |
| hCV11503469 | rs2066854 | hCV7430153 | rs1388077 | 0.51 | 0.048166678 | 0.063 |
| hCV11503469 | rs2066854 | hCV7430158 | rs1466662 | 0.51 | 0.048166678 | 0.1103 |
| hCV11503469 | rs2066854 | hDV70817639 | rs17031739 | 0.51 | 0.048166678 | 0.0603 |
| hCV11503469 | rs2066854 | hDV70817640 | rs17031740 | 0.51 | 0.048166678 | 0.062 |
| hCV11503469 | rs2066854 | hDV70817803 | rs17031951 | 0.51 | 0.048166678 | 0.0706 |
| hCV11503469 | rs2066854 | hDV70817805 | rs17031954 | 0.51 | 0.048166678 | 0.0706 |
| hCV11503469 | rs2066854 | hDV70817844 | rs17032000 | 0.51 | 0.048166678 | 0.0706 |
| hCV11503469 | rs2066854 | hDV70934991 | rs17301943 | 0.51 | 0.048166678 | 0.0786 |
| hCV11503469 | rs2066854 | hDV70945235 | rs17373860 | 0.51 | 0.048166678 | 0.129 |
| hCV11503469 | rs2066854 | hDV72277158 | rs28673871 | 0.51 | 0.048166678 | 0.0592 |
| hCV11503469 | rs2066854 | hDV77232287 | rs7666918 | 0.51 | 0.048166678 | 0.1071 |
| hCV11503469 | rs2066854 | hDV96226316 | rs6834312 | 0.51 | 0.048166678 | 0.0607 |
| hCV11503470 | rs1800788 | hCV11503382 | rs1873369 | 0.51 | 0.150481176 | 0.5598 |
| hCV11503470 | rs1800788 | hCV11503414 | rs2066865 | 0.51 | 0.150481176 | 0.4341 |
| hCV11503470 | rs1800788 | hCV11503416 | rs2066864 | 0.51 | 0.150481176 | 0.4007 |
| hCV11503470 | rs1800788 | hCV11503431 | rs2066861 | 0.51 | 0.150481176 | 0.4356 |

## TABLE 3, page 7 of 71

| Interrogated SNP | Interrogated rs | LD SNP | LD SNP rs | Power | Threshold r² | r² |
|---|---|---|---|---|---|---|
| hCV11503470 | rs1800788 | hCV11503469 | rs2066854 | 0.51 | 0.150481176 | 0.3765 |
| hCV11503470 | rs1800788 | hCV11853483 | rs12644950 | 0.51 | 0.150481176 | 0.3743 |
| hCV11503470 | rs1800788 | hCV11853489 | rs7681423 | 0.51 | 0.150481176 | 0.4007 |
| hCV11503470 | rs1800788 | hCV11853496 | rs7654093 | 0.51 | 0.150481176 | 0.4356 |
| hCV11503470 | rs1800788 | hCV15860433 | rs2070006 | 0.51 | 0.150481176 | 0.2862 |
| hCV11503470 | rs1800788 | hCV21680 | rs7666020 | 0.51 | 0.150481176 | 0.168 |
| hCV11503470 | rs1800788 | hCV21681 | rs6536018 | 0.51 | 0.150481176 | 0.2707 |
| hCV11503470 | rs1800788 | hCV24834 | rs4235247 | 0.51 | 0.150481176 | 0.6801 |
| hCV11503470 | rs1800788 | hCV26019871 | rs4547780 | 0.51 | 0.150481176 | 0.2046 |
| hCV11503470 | rs1800788 | hCV26024202 | rs11731813 | 0.51 | 0.150481176 | 0.4748 |
| hCV11503470 | rs1800788 | hCV27020269 | rs7659613 | 0.51 | 0.150481176 | 0.3134 |
| hCV11503470 | rs1800788 | hCV27020277 | rs6825454 | 0.51 | 0.150481176 | 0.4968 |
| hCV11503470 | rs1800788 | hCV27020280 | rs4463047 | 0.51 | 0.150481176 | 0.4485 |
| hCV11503470 | rs1800788 | hCV27313130 | rs4634202 | 0.51 | 0.150481176 | 0.3826 |
| hCV11503470 | rs1800788 | hCV27905214 | rs4323084 | 0.51 | 0.150481176 | 0.5288 |
| hCV11503470 | rs1800788 | hCV27907560 | rs4696576 | 0.51 | 0.150481176 | 0.2691 |
| hCV11503470 | rs1800788 | hCV27937396 | rs4634201 | 0.51 | 0.150481176 | 0.6797 |
| hCV11503470 | rs1800788 | hCV2892859 | rs13130318 | 0.51 | 0.150481176 | 0.2782 |
| hCV11503470 | rs1800788 | hCV2892869 | rs13109457 | 0.51 | 0.150481176 | 0.4255 |
| hCV11503470 | rs1800788 | hCV2892870 | rs2070011 | 0.51 | 0.150481176 | 0.3019 |
| hCV11503470 | rs1800788 | hCV2892877 | rs6050 | 0.51 | 0.150481176 | 0.5042 |
| hCV11503470 | rs1800788 | hCV2892893 | rs12648258 | 0.51 | 0.150481176 | 1 |
| hCV11503470 | rs1800788 | hCV2892905 | rs12642770 | 0.51 | 0.150481176 | 0.8219 |
| hCV11503470 | rs1800788 | hCV2892918 | rs12511469 | 0.51 | 0.150481176 | 1 |
| hCV11503470 | rs1800788 | hCV2892923 | rs13435192 | 0.51 | 0.150481176 | 0.2139 |
| hCV11503470 | rs1800788 | hCV2892924 | rs13435101 | 0.51 | 0.150481176 | 0.2119 |
| hCV11503470 | rs1800788 | hCV2892925 | rs7689945 | 0.51 | 0.150481176 | 0.2079 |
| hCV11503470 | rs1800788 | hCV2892926 | rs7662567 | 0.51 | 0.150481176 | 1 |
| hCV11503470 | rs1800788 | hCV2892927 | rs13123551 | 0.51 | 0.150481176 | 0.2674 |
| hCV11503470 | rs1800788 | hCV2892928 | rs13147579 | 0.51 | 0.150481176 | 1 |
| hCV11503470 | rs1800788 | hCV28953838 | rs7690851 | 0.51 | 0.150481176 | 0.211 |
| hCV11503470 | rs1800788 | hCV28953840 | rs6536017 | 0.51 | 0.150481176 | 0.1546 |
| hCV11503470 | rs1800788 | hCV29420822 | rs4642230 | 0.51 | 0.150481176 | 0.9 |
| hCV11503470 | rs1800788 | hCV29983641 | rs10008078 | 0.51 | 0.150481176 | 0.9719 |
| hCV11503470 | rs1800788 | hCV30679170 | rs13148992 | 0.51 | 0.150481176 | 0.4826 |
| hCV11503470 | rs1800788 | hCV30711231 | rs12642469 | 0.51 | 0.150481176 | 0.9719 |
| hCV11503470 | rs1800788 | hCV31863979 | rs12186294 | 0.51 | 0.150481176 | 0.1637 |
| hCV11503470 | rs1800788 | hCV31863982 | rs7659024 | 0.51 | 0.150481176 | 0.4356 |
| hCV11503470 | rs1800788 | hCV32212658 | rs11099959 | 0.51 | 0.150481176 | 0.163 |
| hCV11503470 | rs1800788 | hCV32212659 | rs4622984 | 0.51 | 0.150481176 | 0.4821 |
| hCV11503470 | rs1800788 | hCV32212664 | rs12642646 | 0.51 | 0.150481176 | 0.2273 |
| hCV11503470 | rs1800788 | hCV32212669 | rs12649647 | 0.51 | 0.150481176 | 0.1508 |
| hCV11503470 | rs1800788 | hCV354895 | rs11737226 | 0.51 | 0.150481176 | 0.5659 |
| hCV11503470 | rs1800788 | hCV354896 | rs7690972 | 0.51 | 0.150481176 | 0.5659 |
| hCV11503470 | rs1800788 | hCV470979 | rs1490672 | 0.51 | 0.150481176 | 0.4671 |
| hCV11503470 | rs1800788 | hCV7429793 | rs1025154 | 0.51 | 0.150481176 | 0.9719 |
| hCV11503470 | rs1800788 | hDV70945235 | rs17373860 | 0.51 | 0.150481176 | 0.2419 |
| hCV11541681 | rs2001490 | hCV112099 | rs12052539 | 0.51 | 0.847343426 | 0.9243 |
| hCV11541681 | rs2001490 | hCV112100 | rs17350125 | 0.51 | 0.847343426 | 0.9268 |
| hCV11541681 | rs2001490 | hCV11537012 | rs12992607 | 0.51 | 0.847343426 | 0.8544 |
| hCV11541681 | rs2001490 | hCV11537013 | rs12713793 | 0.51 | 0.847343426 | 0.849 |
| hCV11541681 | rs2001490 | hCV11541694 | rs12619258 | 0.51 | 0.847343426 | 1 |
| hCV11541681 | rs2001490 | hCV11541701 | rs6748233 | 0.51 | 0.847343426 | 0.9268 |
| hCV11541681 | rs2001490 | hCV11541702 | rs4852978 | 0.51 | 0.847343426 | 0.9268 |
| hCV11541681 | rs2001490 | hCV11541712 | rs12713791 | 0.51 | 0.847343426 | 0.8856 |

## TABLE 3, page 8 of 71

| Interrogated SNP | Interrogated rs | LD SNP | LD SNP rs | Power | Threshold $r^2$ | $r^2$ |
|---|---|---|---|---|---|---|
| hCV11541681 | rs2001490 | hCV11541719 | rs12615807 | 0.51 | 0.847343426 | 0.8544 |
| hCV11541681 | rs2001490 | hCV11541721 | rs2006997 | 0.51 | 0.847343426 | 0.8544 |
| hCV11541681 | rs2001490 | hCV11941453 | rs2001436 | 0.51 | 0.847343426 | 1 |
| hCV11541681 | rs2001490 | hCV133926 | rs12053242 | 0.51 | 0.847343426 | 0.9268 |
| hCV11541681 | rs2001490 | hCV133927 | rs7599453 | 0.51 | 0.847343426 | 0.9237 |
| hCV11541681 | rs2001490 | hCV133928 | rs4852977 | 0.51 | 0.847343426 | 0.9268 |
| hCV11541681 | rs2001490 | hCV133930 | rs1815028 | 0.51 | 0.847343426 | 0.9268 |
| hCV11541681 | rs2001490 | hCV15804221 | rs2421674 | 0.51 | 0.847343426 | 0.849 |
| hCV11541681 | rs2001490 | hCV15804228 | rs2421675 | 0.51 | 0.847343426 | 0.8544 |
| hCV11541681 | rs2001490 | hCV180709 | rs7591112 | 0.51 | 0.847343426 | 0.8898 |
| hCV11541681 | rs2001490 | hCV180710 | rs11891140 | 0.51 | 0.847343426 | 0.8898 |
| hCV11541681 | rs2001490 | hCV1835582 | rs12713789 | 0.51 | 0.847343426 | 0.8874 |
| hCV11541681 | rs2001490 | hCV1835584 | rs6749841 | 0.51 | 0.847343426 | 0.8856 |
| hCV11541681 | rs2001490 | hCV2050088 | rs2272178 | 0.51 | 0.847343426 | 0.8544 |
| hCV11541681 | rs2001490 | hCV2050091 | rs35791379 | 0.51 | 0.847343426 | 0.849 |
| hCV11541681 | rs2001490 | hCV2050092 | rs12624267 | 0.51 | 0.847343426 | 0.849 |
| hCV11541681 | rs2001490 | hCV2050096 | rs2116367 | 0.51 | 0.847343426 | 0.8544 |
| hCV11541681 | rs2001490 | hCV25924555 | rs13003035 | 0.51 | 0.847343426 | 0.8544 |
| hCV11541681 | rs2001490 | hCV26996655 | rs12713790 | 0.51 | 0.847343426 | 0.8889 |
| hCV11541681 | rs2001490 | hCV26996656 | rs1806683 | 0.51 | 0.847343426 | 0.9243 |
| hCV11541681 | rs2001490 | hCV26996674 | rs13006448 | 0.51 | 0.847343426 | 1 |
| hCV11541681 | rs2001490 | hCV26996679 | rs6732812 | 0.51 | 0.847343426 | 1 |
| hCV11541681 | rs2001490 | hCV26996688 | rs13015885 | 0.51 | 0.847343426 | 1 |
| hCV11541681 | rs2001490 | hCV26996689 | rs13014700 | 0.51 | 0.847343426 | 1 |
| hCV11541681 | rs2001490 | hCV26996690 | rs2421575 | 0.51 | 0.847343426 | 1 |
| hCV11541681 | rs2001490 | hCV26996697 | rs12611487 | 0.51 | 0.847343426 | 1 |
| hCV11541681 | rs2001490 | hCV26996701 | rs7608328 | 0.51 | 0.847343426 | 0.8545 |
| hCV11541681 | rs2001490 | hCV26996705 | rs12997018 | 0.51 | 0.847343426 | 0.9547 |
| hCV11541681 | rs2001490 | hCV29307907 | rs4852316 | 0.51 | 0.847343426 | 0.9243 |
| hCV11541681 | rs2001490 | hCV303807 | rs17350188 | 0.51 | 0.847343426 | 0.849 |
| hCV11541681 | rs2001490 | hCV31840120 | rs12713798 | 0.51 | 0.847343426 | 0.849 |
| hCV11541681 | rs2001490 | hCV31840129 | rs11126417 | 0.51 | 0.847343426 | 0.849 |
| hCV11541681 | rs2001490 | hCV31840132 | rs2421676 | 0.51 | 0.847343426 | 0.849 |
| hCV11541681 | rs2001490 | hCV31840134 | rs11894953 | 0.51 | 0.847343426 | 0.849 |
| hCV11541681 | rs2001490 | hCV31840136 | rs12713795 | 0.51 | 0.847343426 | 0.8544 |
| hCV11541681 | rs2001490 | hCV31840146 | rs11126415 | 0.51 | 0.847343426 | 0.9268 |
| hCV11541681 | rs2001490 | hCV31840149 | rs12233112 | 0.51 | 0.847343426 | 1 |
| hCV11541681 | rs2001490 | hCV31840152 | rs12998980 | 0.51 | 0.847343426 | 1 |
| hCV11541681 | rs2001490 | hCV31840159 | rs13013228 | 0.51 | 0.847343426 | 1 |
| hCV11541681 | rs2001490 | hCV31840166 | rs4513320 | 0.51 | 0.847343426 | 1 |
| hCV11541681 | rs2001490 | hCV505733 | rs11126416 | 0.51 | 0.847343426 | 0.8544 |
| hCV11541681 | rs2001490 | hCV512569 | rs6755500 | 0.51 | 0.847343426 | 0.9236 |
| hCV11541681 | rs2001490 | hCV95670 | rs4852975 | 0.51 | 0.847343426 | 1 |
| hCV11541681 | rs2001490 | hCV95671 | rs11126414 | 0.51 | 0.847343426 | 1 |
| hCV11541681 | rs2001490 | hCV95672 | rs6750515 | 0.51 | 0.847343426 | 0.8515 |
| hCV11541681 | rs2001490 | hDV68778390 | rs10188074 | 0.51 | 0.847343426 | 0.9596 |
| hCV11541681 | rs2001490 | hDV69785784 | rs13000788 | 0.51 | 0.847343426 | 1 |
| hCV11541681 | rs2001490 | hDV70942181 | rs17350056 | 0.51 | 0.847343426 | 1 |
| hCV11541681 | rs2001490 | hDV70953030 | rs17434634 | 0.51 | 0.847343426 | 1 |
| hCV11541681 | rs2001490 | hDV70953035 | rs17434655 | 0.51 | 0.847343426 | 1 |
| hCV11541681 | rs2001490 | hDV77051911 | rs4852972 | 0.51 | 0.847343426 | 1 |
| hCV11541681 | rs2001490 | hDV77051912 | rs4852976 | 0.51 | 0.847343426 | 0.9243 |
| hCV11786258 | rs4253303 | hCV11786147 | rs4862662 | 0.51 | 0.09882857 | 0.6957 |
| hCV11786258 | rs4253303 | hCV11786203 | rs4253251 | 0.51 | 0.09882857 | 0.1395 |
| hCV11786258 | rs4253303 | hCV11786235 | rs4253287 | 0.51 | 0.09882857 | 0.116 |

# TABLE 3, page 9 of 71

| Interrogated SNP | Interrogated rs | LD SNP | LD SNP rs | Power | Threshold r² | r² |
|---|---|---|---|---|---|---|
| hCV11786258 | rs4253303 | hCV11786259 | rs4253304 | 0.51 | 0.09882857 | 0.8944 |
| hCV11786258 | rs4253303 | hCV12066106 | rs1914926 | 0.51 | 0.09882857 | 0.1036 |
| hCV11786258 | rs4253303 | hCV12066118 | rs2048 | 0.51 | 0.09882857 | 0.5556 |
| hCV11786258 | rs4253303 | hCV12066119 | rs1912826 | 0.51 | 0.09882857 | 0.4905 |
| hCV11786258 | rs4253303 | hCV12066124 | rs2036914 | 0.51 | 0.09882857 | 0.3227 |
| hCV11786258 | rs4253303 | hCV15968025 | rs2292425 | 0.51 | 0.09882857 | 0.3145 |
| hCV11786258 | rs4253303 | hCV15968026 | rs2292426 | 0.51 | 0.09882857 | 0.2823 |
| hCV11786258 | rs4253303 | hCV15968034 | rs2292428 | 0.51 | 0.09882857 | 0.337 |
| hCV11786258 | rs4253303 | hCV15968043 | rs2292423 | 0.51 | 0.09882857 | 0.8913 |
| hCV11786258 | rs4253303 | hCV15975109 | rs2304596 | 0.51 | 0.09882857 | 0.1395 |
| hCV11786258 | rs4253303 | hCV2103343 | rs4241824 | 0.51 | 0.09882857 | 0.255 |
| hCV11786258 | rs4253303 | hCV2103348 | rs11931515 | 0.51 | 0.09882857 | 0.116 |
| hCV11786258 | rs4253303 | hCV2103391 | rs1008728 | 0.51 | 0.09882857 | 0.1419 |
| hCV11786258 | rs4253303 | hCV2103392 | rs12500826 | 0.51 | 0.09882857 | 0.1267 |
| hCV11786258 | rs4253303 | hCV22271609 | rs4253326 | 0.51 | 0.09882857 | 0.1138 |
| hCV11786258 | rs4253303 | hCV22272267 | rs3733402 | 0.51 | 0.09882857 | 0.5632 |
| hCV11786258 | rs4253303 | hCV25474413 | rs3822057 | 0.51 | 0.09882857 | 0.2622 |
| hCV11786258 | rs4253303 | hCV25474414 | rs4253399 | 0.51 | 0.09882857 | 0.2697 |
| hCV11786258 | rs4253303 | hCV25634781 | rs4253299 | 0.51 | 0.09882857 | 0.1325 |
| hCV11786258 | rs4253303 | hCV25989001 | hCV25989001 | 0.51 | 0.09882857 | 0.1474 |
| hCV11786258 | rs4253303 | hCV25990131 | rs13146272 | 0.51 | 0.09882857 | 0.3213 |
| hCV11786258 | rs4253303 | hCV26038139 | rs4253405 | 0.51 | 0.09882857 | 0.1069 |
| hCV11786258 | rs4253303 | hCV26265197 | rs10014399 | 0.51 | 0.09882857 | 0.1412 |
| hCV11786258 | rs4253303 | hCV26265199 | rs2221843 | 0.51 | 0.09882857 | 0.1325 |
| hCV11786258 | rs4253303 | hCV26265231 | rs7684025 | 0.51 | 0.09882857 | 0.5918 |
| hCV11786258 | rs4253303 | hCV27474895 | rs3756011 | 0.51 | 0.09882857 | 0.1518 |
| hCV11786258 | rs4253303 | hCV27477533 | rs3756008 | 0.51 | 0.09882857 | 0.315 |
| hCV11786258 | rs4253303 | hCV27482765 | rs3775301 | 0.51 | 0.09882857 | 0.1395 |
| hCV11786258 | rs4253303 | hCV27506149 | rs3822055 | 0.51 | 0.09882857 | 0.1325 |
| hCV11786258 | rs4253303 | hCV27902808 | rs4253236 | 0.51 | 0.09882857 | 0.366 |
| hCV11786258 | rs4253303 | hCV28960679 | rs6844764 | 0.51 | 0.09882857 | 0.3907 |
| hCV11786258 | rs4253303 | hCV29053260 | rs4861707 | 0.51 | 0.09882857 | 0.1962 |
| hCV11786258 | rs4253303 | hCV29053264 | rs7667777 | 0.51 | 0.09882857 | 0.7578 |
| hCV11786258 | rs4253303 | hCV29053265 | rs4253244 | 0.51 | 0.09882857 | 0.3533 |
| hCV11786258 | rs4253303 | hCV29718000 | rs4253238 | 0.51 | 0.09882857 | 0.5569 |
| hCV11786258 | rs4253303 | hCV29877725 | rs4253295 | 0.51 | 0.09882857 | 1 |
| hCV11786258 | rs4253303 | hCV30983927 | rs6552962 | 0.51 | 0.09882857 | 0.1072 |
| hCV11786258 | rs4253303 | hCV32209636 | rs11132387 | 0.51 | 0.09882857 | 0.2106 |
| hCV11786258 | rs4253303 | hCV32209638 | rs12507040 | 0.51 | 0.09882857 | 0.1024 |
| hCV11786258 | rs4253303 | hCV32291217 | rs4253323 | 0.51 | 0.09882857 | 0.1395 |
| hCV11786258 | rs4253303 | hCV32291269 | rs4253417 | 0.51 | 0.09882857 | 0.2035 |
| hCV11786258 | rs4253303 | hCV32291295 | rs4253292 | 0.51 | 0.09882857 | 0.1404 |
| hCV11786258 | rs4253303 | hCV32291301 | rs4253302 | 0.51 | 0.09882857 | 0.1385 |
| hCV11786258 | rs4253303 | hCV32295028 | rs4253260 | 0.51 | 0.09882857 | 0.1395 |
| hCV11786258 | rs4253303 | hCV3229991 | rs4241815 | 0.51 | 0.09882857 | 0.5632 |
| hCV11786258 | rs4253303 | hCV3229992 | rs3775298 | 0.51 | 0.09882857 | 0.5632 |
| hCV11786258 | rs4253303 | hCV3229995 | rs11132382 | 0.51 | 0.09882857 | 0.5569 |
| hCV11786258 | rs4253303 | hCV3230000 | rs4253294 | 0.51 | 0.09882857 | 0.2479 |
| hCV11786258 | rs4253303 | hCV3230002 | rs4253297 | 0.51 | 0.09882857 | 1 |
| hCV11786258 | rs4253303 | hCV3230003 | rs2304595 | 0.51 | 0.09882857 | 0.8848 |
| hCV11786258 | rs4253303 | hCV3230006 | rs4253308 | 0.51 | 0.09882857 | 1 |
| hCV11786258 | rs4253303 | hCV3230007 | rs4253311 | 0.51 | 0.09882857 | 0.5632 |
| hCV11786258 | rs4253303 | hCV3230011 | rs4253320 | 0.51 | 0.09882857 | 1 |
| hCV11786258 | rs4253303 | hCV3230012 | rs4241821 | 0.51 | 0.09882857 | 0.1325 |
| hCV11786258 | rs4253303 | hCV3230013 | rs3775303 | 0.51 | 0.09882857 | 0.8944 |

# TABLE 3, page 10 of 71

| Interrogated SNP | Interrogated rs | LD SNP | LD SNP rs | Power | Threshold r² | r² |
|---|---|---|---|---|---|---|
| hCV11786258 | rs4253303 | hCV3230014 | rs4861709 | 0.51 | 0.09882857 | 0.2479 |
| hCV11786258 | rs4253303 | hCV3230017 | rs4253327 | 0.51 | 0.09882857 | 0.2534 |
| hCV11786258 | rs4253303 | hCV3230018 | rs925453 | 0.51 | 0.09882857 | 0.2319 |
| hCV11786258 | rs4253303 | hCV3230019 | rs4253332 | 0.51 | 0.09882857 | 0.2319 |
| hCV11786258 | rs4253303 | hCV3230022 | rs11132383 | 0.51 | 0.09882857 | 0.1658 |
| hCV11786258 | rs4253303 | hCV3230025 | rs3756009 | 0.51 | 0.09882857 | 0.2464 |
| hCV11786258 | rs4253303 | hCV3230038 | rs2289252 | 0.51 | 0.09882857 | 0.1956 |
| hCV11786258 | rs4253303 | hCV3230083 | rs10013653 | 0.51 | 0.09882857 | 0.4797 |
| hCV11786258 | rs4253303 | hCV3230084 | rs7682918 | 0.51 | 0.09882857 | 0.5961 |
| hCV11786258 | rs4253303 | hCV3230094 | rs7687818 | 0.51 | 0.09882857 | 0.6447 |
| hCV11786258 | rs4253303 | hCV3230096 | rs3817184 | 0.51 | 0.09882857 | 0.7346 |
| hCV11786258 | rs4253303 | hCV3230097 | rs3736455 | 0.51 | 0.09882857 | 0.2761 |
| hCV11786258 | rs4253303 | hCV3230101 | rs6835839 | 0.51 | 0.09882857 | 0.3578 |
| hCV11786258 | rs4253303 | hCV3230106 | rs1473597 | 0.51 | 0.09882857 | 0.3534 |
| hCV11786258 | rs4253303 | hCV3230110 | rs2276917 | 0.51 | 0.09882857 | 0.337 |
| hCV11786258 | rs4253303 | hCV3230113 | rs1053094 | 0.51 | 0.09882857 | 0.491 |
| hCV11786258 | rs4253303 | hCV3230125 | rs11938564 | 0.51 | 0.09882857 | 0.1367 |
| hCV11786258 | rs4253303 | hCV3230131 | rs13136269 | 0.51 | 0.09882857 | 0.1024 |
| hCV11786258 | rs4253303 | hCV3230133 | rs12511874 | 0.51 | 0.09882857 | 0.1024 |
| hCV11786258 | rs4253303 | hCV3230134 | rs12500151 | 0.51 | 0.09882857 | 0.1024 |
| hCV11786258 | rs4253303 | hCV3230136 | rs13116273 | 0.51 | 0.09882857 | 0.1243 |
| hCV11786258 | rs4253303 | hCV32313006 | rs4253248 | 0.51 | 0.09882857 | 0.5569 |
| hCV11786258 | rs4253303 | hCV32313024 | rs4253239 | 0.51 | 0.09882857 | 0.1404 |
| hCV11786258 | rs4253303 | hCV32358975 | rs4253255 | 0.51 | 0.09882857 | 0.5556 |
| hCV11786258 | rs4253303 | hCV32358984 | rs4253256 | 0.51 | 0.09882857 | 0.3667 |
| hCV11786258 | rs4253303 | hCV8241630 | rs925451 | 0.51 | 0.09882857 | 0.2889 |
| hCV11786258 | rs4253303 | hCV8241631 | rs1511802 | 0.51 | 0.09882857 | 1 |
| hCV11786258 | rs4253303 | hCV8241632 | rs1511801 | 0.51 | 0.09882857 | 0.5625 |
| hCV11786258 | rs4253303 | hDV71222711 | rs4253252 | 0.51 | 0.09882857 | 0.5569 |
| hCV11786258 | rs4253303 | hDV76175111 | rs35079309 | 0.51 | 0.09882857 | 0.1206 |
| hCV11975250 | rs6025 | hCV11341861 | rs10800436 | 0.51 | 0.015514847 | 0.1922 |
| hCV11975250 | rs6025 | hCV11341869 | rs2176473 | 0.51 | 0.015514847 | 0.0375 |
| hCV11975250 | rs6025 | hCV11341876 | rs1980198 | 0.51 | 0.015514847 | 0.0327 |
| hCV11975250 | rs6025 | hCV11341878 | rs4656670 | 0.51 | 0.015514847 | 0.0327 |
| hCV11975250 | rs6025 | hCV11341882 | rs12024897 | 0.51 | 0.015514847 | 0.0327 |
| hCV11975250 | rs6025 | hCV11341898 | rs12563090 | 0.51 | 0.015514847 | 0.0332 |
| hCV11975250 | rs6025 | hCV11342138 | rs2142760 | 0.51 | 0.015514847 | 0.0175 |
| hCV11975250 | rs6025 | hCV11975194 | rs2038024 | 0.51 | 0.015514847 | 0.0613 |
| hCV11975250 | rs6025 | hCV11975195 | rs1894692 | 0.51 | 0.015514847 | 1 |
| hCV11975250 | rs6025 | hCV11975285 | rs6127 | 0.51 | 0.015514847 | 0.026 |
| hCV11975250 | rs6025 | hCV11975296 | rs6131 | 0.51 | 0.015514847 | 0.0848 |
| hCV11975250 | rs6025 | hCV11975318 | rs1883228 | 0.51 | 0.015514847 | 0.0768 |
| hCV11975250 | rs6025 | hCV11975322 | rs5357 | 0.51 | 0.015514847 | 0.0827 |
| hCV11975250 | rs6025 | hCV11975325 | rs5367 | 0.51 | 0.015514847 | 0.1728 |
| hCV11975250 | rs6025 | hCV11975329 | rs5363 | 0.51 | 0.015514847 | 0.1728 |
| hCV11975250 | rs6025 | hCV11975331 | rs5362 | 0.51 | 0.015514847 | 0.1728 |
| hCV11975250 | rs6025 | hCV11975332 | rs5361 | 0.51 | 0.015514847 | 0.1728 |
| hCV11975250 | rs6025 | hCV11975488 | rs2057249 | 0.51 | 0.015514847 | 0.1728 |
| hCV11975250 | rs6025 | hCV15802103 | rs2420370 | 0.51 | 0.015514847 | 0.117 |
| hCV11975250 | rs6025 | hCV15802110 | rs2420371 | 0.51 | 0.015514847 | 0.3415 |
| hCV11975250 | rs6025 | hCV15858911 | rs2806392 | 0.51 | 0.015514847 | 0.1655 |
| hCV11975250 | rs6025 | hCV15868017 | rs2223303 | 0.51 | 0.015514847 | 0.0183 |
| hCV11975250 | rs6025 | hCV15878582 | rs2275299 | 0.51 | 0.015514847 | 0.0327 |
| hCV11975250 | rs6025 | hCV15962928 | rs2285211 | 0.51 | 0.015514847 | 0.0303 |
| hCV11975250 | rs6025 | hCV16161169 | rs2205847 | 0.51 | 0.015514847 | 0.0872 |

# TABLE 3, page 11 of 71

| Interrogated SNP | Interrogated rs | LD SNP | LD SNP rs | Power | Threshold r² | r² |
|---|---|---|---|---|---|---|
| hCV11975250 | rs6025 | hCV16177404 | rs2272920 | 0.51 | 0.015514847 | 0.1655 |
| hCV11975250 | rs6025 | hCV221700 | rs6677410 | 0.51 | 0.015514847 | 0.0327 |
| hCV11975250 | rs6025 | hCV2217923 | rs2014878 | 0.51 | 0.015514847 | 0.159 |
| hCV11975250 | rs6025 | hCV2456693 | rs6672589 | 0.51 | 0.015514847 | 0.0169 |
| hCV11975250 | rs6025 | hCV2456695 | rs10919173 | 0.51 | 0.015514847 | 0.0169 |
| hCV11975250 | rs6025 | hCV2456708 | rs1517745 | 0.51 | 0.015514847 | 0.0544 |
| hCV11975250 | rs6025 | hCV2456730 | rs961404 | 0.51 | 0.015514847 | 0.0327 |
| hCV11975250 | rs6025 | hCV2456733 | rs12021580 | 0.51 | 0.015514847 | 0.0168 |
| hCV11975250 | rs6025 | hCV2456741 | rs6696810 | 0.51 | 0.015514847 | 0.0327 |
| hCV11975250 | rs6025 | hCV2456747 | rs3820059 | 0.51 | 0.015514847 | 0.0423 |
| hCV11975250 | rs6025 | hCV2456768 | rs6427186 | 0.51 | 0.015514847 | 0.0327 |
| hCV11975250 | rs6025 | hCV2459402 | rs12045330 | 0.51 | 0.015514847 | 0.0369 |
| hCV11975250 | rs6025 | hCV2459404 | rs6663862 | 0.51 | 0.015514847 | 0.0763 |
| hCV11975250 | rs6025 | hCV2459408 | rs7531806 | 0.51 | 0.015514847 | 0.0171 |
| hCV11975250 | rs6025 | hCV2459420 | rs4987351 | 0.51 | 0.015514847 | 0.0246 |
| hCV11975250 | rs6025 | hCV2459428 | rs4987285 | 0.51 | 0.015514847 | 0.0804 |
| hCV11975250 | rs6025 | hCV2459446 | rs4786 | 0.51 | 0.015514847 | 0.0799 |
| hCV11975250 | rs6025 | hCV2459453 | rs3917419 | 0.51 | 0.015514847 | 0.0192 |
| hCV11975250 | rs6025 | hCV2459459 | rs932307 | 0.51 | 0.015514847 | 0.0872 |
| hCV11975250 | rs6025 | hCV2459460 | rs5353 | 0.51 | 0.015514847 | 0.0839 |
| hCV11975250 | rs6025 | hCV2480400 | rs1569474 | 0.51 | 0.015514847 | 0.0436 |
| hCV11975250 | rs6025 | hCV2480404 | rs7551819 | 0.51 | 0.015514847 | 0.0183 |
| hCV11975250 | rs6025 | hCV2480416 | rs732314 | 0.51 | 0.015514847 | 0.0196 |
| hCV11975250 | rs6025 | hCV2480424 | rs2244529 | 0.51 | 0.015514847 | 0.0523 |
| hCV11975250 | rs6025 | hCV2480428 | rs3917740 | 0.51 | 0.015514847 | 0.0725 |
| hCV11975250 | rs6025 | hCV2481727 | rs6670407 | 0.51 | 0.015514847 | 0.0281 |
| hCV11975250 | rs6025 | hCV2481731 | rs9332640 | 0.51 | 0.015514847 | 0.0271 |
| hCV11975250 | rs6025 | hCV2481732 | rs12131397 | 0.51 | 0.015514847 | 0.0273 |
| hCV11975250 | rs6025 | hCV25616192 | rs10919168 | 0.51 | 0.015514847 | 0.0534 |
| hCV11975250 | rs6025 | hCV25617131 | rs3917410 | 0.51 | 0.015514847 | 0.1718 |
| hCV11975250 | rs6025 | hCV25617143 | rs3917425 | 0.51 | 0.015514847 | 0.1728 |
| hCV11975250 | rs6025 | hCV25619707 | rs4987308 | 0.51 | 0.015514847 | 0.0827 |
| hCV11975250 | rs6025 | hCV25921520 | rs12132173 | 0.51 | 0.015514847 | 0.1726 |
| hCV11975250 | rs6025 | hCV25922175 | rs12120229 | 0.51 | 0.015514847 | 0.1728 |
| hCV11975250 | rs6025 | hCV27242639 | rs7544221 | 0.51 | 0.015514847 | 0.0413 |
| hCV11975250 | rs6025 | hCV27242706 | rs7524348 | 0.51 | 0.015514847 | 0.0169 |
| hCV11975250 | rs6025 | hCV27242742 | rs12408451 | 0.51 | 0.015514847 | 0.0278 |
| hCV11975250 | rs6025 | hCV27243253 | rs2420505 | 0.51 | 0.015514847 | 0.1007 |
| hCV11975250 | rs6025 | hCV27478380 | rs3766141 | 0.51 | 0.015514847 | 0.1655 |
| hCV11975250 | rs6025 | hCV27480806 | rs3766129 | 0.51 | 0.015514847 | 0.0347 |
| hCV11975250 | rs6025 | hCV27497504 | rs3917683 | 0.51 | 0.015514847 | 0.0162 |
| hCV11975250 | rs6025 | hCV27523232 | rs3917681 | 0.51 | 0.015514847 | 0.0583 |
| hCV11975250 | rs6025 | hCV27886241 | rs4656690 | 0.51 | 0.015514847 | 0.0449 |
| hCV11975250 | rs6025 | hCV27886249 | rs3917406 | 0.51 | 0.015514847 | 0.0349 |
| hCV11975250 | rs6025 | hCV279320 | rs10800441 | 0.51 | 0.015514847 | 0.0327 |
| hCV11975250 | rs6025 | hCV27936996 | rs4656697 | 0.51 | 0.015514847 | 0.033 |
| hCV11975250 | rs6025 | hCV28023624 | rs4656704 | 0.51 | 0.015514847 | 0.0804 |
| hCV11975250 | rs6025 | hCV29397237 | rs6427185 | 0.51 | 0.015514847 | 0.0423 |
| hCV11975250 | rs6025 | hCV29397245 | rs6656822 | 0.51 | 0.015514847 | 0.0571 |
| hCV11975250 | rs6025 | hCV29397247 | rs6427194 | 0.51 | 0.015514847 | 0.1171 |
| hCV11975250 | rs6025 | hCV29397248 | rs6427195 | 0.51 | 0.015514847 | 0.2959 |
| hCV11975250 | rs6025 | hCV29397252 | rs6427197 | 0.51 | 0.015514847 | 0.2959 |
| hCV11975250 | rs6025 | hCV29397255 | rs6427202 | 0.51 | 0.015514847 | 0.0281 |
| hCV11975250 | rs6025 | hCV29397262 | rs3917786 | 0.51 | 0.015514847 | 0.0276 |
| hCV11975250 | rs6025 | hCV29397289 | rs4656198 | 0.51 | 0.015514847 | 0.1007 |

## TABLE 3, page 12 of 71

| Interrogated SNP | Interrogated rs | LD SNP | LD SNP rs | Power | Threshold $r^2$ | $r^2$ |
|---|---|---|---|---|---|---|
| hCV11975250 | rs6025 | hCV29585595 | rs10489173 | 0.51 | 0.015514847 | 0.1655 |
| hCV11975250 | rs6025 | hCV29748285 | rs6687813 | 0.51 | 0.015514847 | 0.3169 |
| hCV11975250 | rs6025 | hCV29820280 | rs6696217 | 0.51 | 0.015514847 | 0.2454 |
| hCV11975250 | rs6025 | hCV30036721 | rs3917449 | 0.51 | 0.015514847 | 0.0183 |
| hCV11975250 | rs6025 | hCV30126935 | rs6692451 | 0.51 | 0.015514847 | 0.1071 |
| hCV11975250 | rs6025 | hCV30324835 | rs10489183 | 0.51 | 0.015514847 | 0.0751 |
| hCV11975250 | rs6025 | hCV30631277 | rs10489182 | 0.51 | 0.015514847 | 0.0439 |
| hCV11975250 | rs6025 | hCV32141371 | rs10800447 | 0.51 | 0.015514847 | 0.0534 |
| hCV11975250 | rs6025 | hCV32141374 | rs10919174 | 0.51 | 0.015514847 | 0.0183 |
| hCV11975250 | rs6025 | hCV32141406 | rs10737547 | 0.51 | 0.015514847 | 0.1348 |
| hCV11975250 | rs6025 | hCV32141457 | rs6678795 | 0.51 | 0.015514847 | 0.0226 |
| hCV11975250 | rs6025 | hCV32141484 | rs3917768 | 0.51 | 0.015514847 | 0.0159 |
| hCV11975250 | rs6025 | hCV32141485 | rs3917744 | 0.51 | 0.015514847 | 0.0407 |
| hCV11975250 | rs6025 | hCV32141499 | rs3917862 | 0.51 | 0.015514847 | 0.1954 |
| hCV11975250 | rs6025 | hCV32141505 | rs3917657 | 0.51 | 0.015514847 | 0.1023 |
| hCV11975250 | rs6025 | hCV32141519 | rs12131631 | 0.51 | 0.015514847 | 0.1222 |
| hCV11975250 | rs6025 | hCV32141520 | rs12123695 | 0.51 | 0.015514847 | 0.0578 |
| hCV11975250 | rs6025 | hCV32141521 | rs10800462 | 0.51 | 0.015514847 | 0.0178 |
| hCV11975250 | rs6025 | hCV32141522 | rs12126695 | 0.51 | 0.015514847 | 0.0631 |
| hCV11975250 | rs6025 | hCV32141523 | rs10919204 | 0.51 | 0.015514847 | 0.0631 |
| hCV11975250 | rs6025 | hCV32141527 | rs10919207 | 0.51 | 0.015514847 | 0.0631 |
| hCV11975250 | rs6025 | hCV32141586 | rs12137905 | 0.51 | 0.015514847 | 0.0827 |
| hCV11975250 | rs6025 | hCV32141621 | rs12133642 | 0.51 | 0.015514847 | 0.1728 |
| hCV11975250 | rs6025 | hCV32141622 | rs12133666 | 0.51 | 0.015514847 | 0.1011 |
| hCV11975250 | rs6025 | hCV32141631 | rs3917436 | 0.51 | 0.015514847 | 0.0801 |
| hCV11975250 | rs6025 | hCV32141639 | rs3917411 | 0.51 | 0.015514847 | 0.1728 |
| hCV11975250 | rs6025 | hCV32141645 | rs3917452 | 0.51 | 0.015514847 | 0.1726 |
| hCV11975250 | rs6025 | hCV32141663 | rs12142587 | 0.51 | 0.015514847 | 0.0826 |
| hCV11975250 | rs6025 | hCV32141665 | rs10800470 | 0.51 | 0.015514847 | 0.0462 |
| hCV11975250 | rs6025 | hCV32141669 | rs10800472 | 0.51 | 0.015514847 | 0.0467 |
| hCV11975250 | rs6025 | hCV32141741 | rs12135361 | 0.51 | 0.015514847 | 0.1655 |
| hCV11975250 | rs6025 | hCV32141779 | rs12122767 | 0.51 | 0.015514847 | 0.14 |
| hCV11975250 | rs6025 | hCV32141799 | rs12133074 | 0.51 | 0.015514847 | 0.1655 |
| hCV11975250 | rs6025 | hCV32141820 | rs12132384 | 0.51 | 0.015514847 | 0.1655 |
| hCV11975250 | rs6025 | hCV32141821 | rs12135726 | 0.51 | 0.015514847 | 0.1655 |
| hCV11975250 | rs6025 | hCV32141828 | rs12136425 | 0.51 | 0.015514847 | 0.1655 |
| hCV11975250 | rs6025 | hCV32141844 | rs12142093 | 0.51 | 0.015514847 | 0.1655 |
| hCV11975250 | rs6025 | hCV32141847 | rs12143057 | 0.51 | 0.015514847 | 0.1655 |
| hCV11975250 | rs6025 | hCV32141873 | rs12131357 | 0.51 | 0.015514847 | 0.1803 |
| hCV11975250 | rs6025 | hCV32141874 | rs12121045 | 0.51 | 0.015514847 | 0.1655 |
| hCV11975250 | rs6025 | hCV32141888 | rs12124561 | 0.51 | 0.015514847 | 0.1655 |
| hCV11975250 | rs6025 | hCV32141892 | rs12125595 | 0.51 | 0.015514847 | 0.1655 |
| hCV11975250 | rs6025 | hCV32141893 | rs12125679 | 0.51 | 0.015514847 | 0.1655 |
| hCV11975250 | rs6025 | hCV32141894 | rs12128308 | 0.51 | 0.015514847 | 0.1587 |
| hCV11975250 | rs6025 | hCV32141903 | rs12131192 | 0.51 | 0.015514847 | 0.1655 |
| hCV11975250 | rs6025 | hCV32141968 | rs12124907 | 0.51 | 0.015514847 | 0.1728 |
| hCV11975250 | rs6025 | hCV32141971 | rs12118305 | 0.51 | 0.015514847 | 0.1018 |
| hCV11975250 | rs6025 | hCV32398748 | rs3917417 | 0.51 | 0.015514847 | 0.1167 |
| hCV11975250 | rs6025 | hCV32398763 | rs3917392 | 0.51 | 0.015514847 | 0.1728 |
| hCV11975250 | rs6025 | hCV325211 | rs3753305 | 0.51 | 0.015514847 | 0.0251 |
| hCV11975250 | rs6025 | hCV325253 | rs2236868 | 0.51 | 0.015514847 | 0.0246 |
| hCV11975250 | rs6025 | hCV337817 | rs9332586 | 0.51 | 0.015514847 | 0.0178 |
| hCV11975250 | rs6025 | hCV474695 | rs10800463 | 0.51 | 0.015514847 | 0.0244 |
| hCV11975250 | rs6025 | hCV574681 | rs575147 | 0.51 | 0.015514847 | 0.1072 |
| hCV11975250 | rs6025 | hCV574682 | rs590181 | 0.51 | 0.015514847 | 0.1655 |

## TABLE 3, page 13 of 71

| Interrogated SNP | Interrogated rs | LD SNP | LD SNP rs | Power | Threshold r² | r² |
|---|---|---|---|---|---|---|
| hCV11975250 | rs6025 | hCV574683 | rs544008 | 0.51 | 0.015514847 | 0.1655 |
| hCV11975250 | rs6025 | hCV574693 | rs601355 | 0.51 | 0.015514847 | 0.1655 |
| hCV11975250 | rs6025 | hCV574707 | rs565397 | 0.51 | 0.015514847 | 0.1655 |
| hCV11975250 | rs6025 | hCV574726 | rs664962 | 0.51 | 0.015514847 | 0.1655 |
| hCV11975250 | rs6025 | hCV574743 | rs545963 | 0.51 | 0.015514847 | 0.1724 |
| hCV11975250 | rs6025 | hCV574757 | rs654664 | 0.51 | 0.015514847 | 0.1728 |
| hCV11975250 | rs6025 | hCV574764 | rs638486 | 0.51 | 0.015514847 | 0.1728 |
| hCV11975250 | rs6025 | hCV574785 | rs511609 | 0.51 | 0.015514847 | 0.1583 |
| hCV11975250 | rs6025 | hCV574788 | rs629408 | 0.51 | 0.015514847 | 0.1726 |
| hCV11975250 | rs6025 | hCV574789 | rs629421 | 0.51 | 0.015514847 | 0.1726 |
| hCV11975250 | rs6025 | hCV8688930 | rs3905328 | 0.51 | 0.015514847 | 0.043 |
| hCV11975250 | rs6025 | hCV8690976 | rs1124843 | 0.51 | 0.015514847 | 0.0423 |
| hCV11975250 | rs6025 | hCV8697031 | rs1400836 | 0.51 | 0.015514847 | 0.0423 |
| hCV11975250 | rs6025 | hCV8697043 | rs1517747 | 0.51 | 0.015514847 | 0.0183 |
| hCV11975250 | rs6025 | hCV8697049 | rs1517744 | 0.51 | 0.015514847 | 0.0559 |
| hCV11975250 | rs6025 | hCV8697055 | rs1208134 | 0.51 | 0.015514847 | 0.1939 |
| hCV11975250 | rs6025 | hCV8697995 | rs4519 | 0.51 | 0.015514847 | 0.1655 |
| hCV11975250 | rs6025 | hCV8698056 | rs488488 | 0.51 | 0.015514847 | 0.117 |
| hCV11975250 | rs6025 | hCV8698071 | rs673789 | 0.51 | 0.015514847 | 0.1655 |
| hCV11975250 | rs6025 | hCV8919425 | rs970740 | 0.51 | 0.015514847 | 0.1171 |
| hCV11975250 | rs6025 | hCV8919431 | rs6009 | 0.51 | 0.015514847 | 0.2959 |
| hCV11975250 | rs6025 | hCV8919452 | rs1018827 | 0.51 | 0.015514847 | 0.2769 |
| hCV11975250 | rs6025 | hCV8919485 | rs1800808 | 0.51 | 0.015514847 | 0.0583 |
| hCV11975250 | rs6025 | hCV8919492 | rs1569476 | 0.51 | 0.015514847 | 0.0303 |
| hCV11975250 | rs6025 | hCV8919494 | rs1011267 | 0.51 | 0.015514847 | 0.0194 |
| hCV11975250 | rs6025 | hCV8919500 | rs1011266 | 0.51 | 0.015514847 | 0.131 |
| hCV11975250 | rs6025 | hCV8919501 | rs909628 | 0.51 | 0.015514847 | 0.1728 |
| hCV11975250 | rs6025 | hCV8919509 | rs1051091 | 0.51 | 0.015514847 | 0.0872 |
| hCV11975250 | rs6025 | hCV8919515 | rs1569457 | 0.51 | 0.015514847 | 0.0827 |
| hCV11975250 | rs6025 | hCV8919527 | rs1800016 | 0.51 | 0.015514847 | 0.1655 |
| hCV11975250 | rs6025 | hCV8919528 | rs1800015 | 0.51 | 0.015514847 | 0.1728 |
| hCV11975250 | rs6025 | hCV8919530 | rs1805193 | 0.51 | 0.015514847 | 0.1728 |
| hCV11975250 | rs6025 | hCV9945935 | rs3917750 | 0.51 | 0.015514847 | 0.0183 |
| hCV11975250 | rs6025 | hDV70670007 | rs16828222 | 0.51 | 0.015514847 | 0.1655 |
| hCV11975250 | rs6025 | hDV70694593 | rs16861990 | 0.51 | 0.015514847 | 0.1939 |
| hCV11975250 | rs6025 | hDV70695296 | rs16862919 | 0.51 | 0.015514847 | 0.189 |
| hCV11975250 | rs6025 | hDV70695328 | rs16862956 | 0.51 | 0.015514847 | 0.116 |
| hCV11975250 | rs6025 | hDV70695338 | rs16862968 | 0.51 | 0.015514847 | 0.1728 |
| hCV11975250 | rs6025 | hDV70965007 | rs17529304 | 0.51 | 0.015514847 | 0.1655 |
| hCV11975250 | rs6025 | hDV70966798 | rs17543370 | 0.51 | 0.015514847 | 0.1651 |
| hCV11975250 | rs6025 | hDV70966830 | rs17543611 | 0.51 | 0.015514847 | 0.1655 |
| hCV11975250 | rs6025 | hDV70974851 | rs17601631 | 0.51 | 0.015514847 | 0.1655 |
| hCV11975250 | rs6025 | hDV70975002 | rs17602701 | 0.51 | 0.015514847 | 0.1651 |
| hCV11975250 | rs6025 | hDV70975134 | rs17603666 | 0.51 | 0.015514847 | 0.1655 |
| hCV11975250 | rs6025 | hDV71028805 | rs4987299 | 0.51 | 0.015514847 | 0.1728 |
| hCV11975250 | rs6025 | hDV71028807 | rs4987302 | 0.51 | 0.015514847 | 0.0827 |
| hCV11975250 | rs6025 | hDV71028808 | rs4987304 | 0.51 | 0.015514847 | 0.0827 |
| hCV11975250 | rs6025 | hDV71028809 | rs4987307 | 0.51 | 0.015514847 | 0.0827 |
| hCV11975250 | rs6025 | hDV71028811 | rs4987318 | 0.51 | 0.015514847 | 0.033 |
| hCV11975250 | rs6025 | hDV71028814 | rs4987323 | 0.51 | 0.015514847 | 0.1728 |
| hCV11975250 | rs6025 | hDV71028815 | rs4987324 | 0.51 | 0.015514847 | 0.1728 |
| hCV11975250 | rs6025 | hDV71028816 | rs4987325 | 0.51 | 0.015514847 | 0.0827 |
| hCV11975250 | rs6025 | hDV71028819 | rs4987340 | 0.51 | 0.015514847 | 0.0827 |
| hCV11975250 | rs6025 | hDV71028821 | rs4987343 | 0.51 | 0.015514847 | 0.0827 |
| hCV11975250 | rs6025 | hDV71028822 | rs4987345 | 0.51 | 0.015514847 | 0.0826 |

# TABLE 3, page 14 of 71

| Interrogated SNP | Interrogated rs | LD SNP | LD SNP rs | Power | Threshold r² | r² |
|---|---|---|---|---|---|---|
| hCV11975250 | rs6025 | hDV71028828 | rs4987395 | 0.51 | 0.015514847 | 0.0827 |
| hCV11975250 | rs6025 | hDV71070471 | rs4987363 | 0.51 | 0.015514847 | 0.1728 |
| hCV11975250 | rs6025 | hDV76908547 | rs3917400 | 0.51 | 0.015514847 | 0.103 |
| hCV11975250 | rs6025 | hDV76908557 | rs3917427 | 0.51 | 0.015514847 | 0.1728 |
| hCV11975250 | rs6025 | hDV76908563 | rs3917441 | 0.51 | 0.015514847 | 0.103 |
| hCV11975250 | rs6025 | hDV76908571 | rs3917454 | 0.51 | 0.015514847 | 0.25 |
| hCV11975250 | rs6025 | hDV76908576 | rs3917461 | 0.51 | 0.015514847 | 0.1592 |
| hCV11975250 | rs6025 | hDV76908651 | rs3917729 | 0.51 | 0.015514847 | 0.0583 |
| hCV11975250 | rs6025 | hDV77030725 | rs4656701 | 0.51 | 0.015514847 | 0.0804 |
| hCV11975250 | rs6025 | hDV77030727 | rs4656703 | 0.51 | 0.015514847 | 0.0804 |
| hCV12066124 | rs2036914 | hCV11786147 | rs4862662 | 0.51 | 0.050680687 | 0.2824 |
| hCV12066124 | rs2036914 | hCV11786203 | rs4253251 | 0.51 | 0.050680687 | 0.0507 |
| hCV12066124 | rs2036914 | hCV11786235 | rs4253287 | 0.51 | 0.050680687 | 0.0572 |
| hCV12066124 | rs2036914 | hCV11786258 | rs4253303 | 0.51 | 0.050680687 | 0.3227 |
| hCV12066124 | rs2036914 | hCV11786259 | rs4253304 | 0.51 | 0.050680687 | 0.3572 |
| hCV12066124 | rs2036914 | hCV11786295 | rs4253421 | 0.51 | 0.050680687 | 0.1004 |
| hCV12066124 | rs2036914 | hCV11786307 | rs1062547 | 0.51 | 0.050680687 | 0.4099 |
| hCV12066124 | rs2036914 | hCV11786327 | rs13133050 | 0.51 | 0.050680687 | 0.1901 |
| hCV12066124 | rs2036914 | hCV12066116 | rs1877320 | 0.51 | 0.050680687 | 0.1385 |
| hCV12066124 | rs2036914 | hCV12066118 | rs2048 | 0.51 | 0.050680687 | 0.3579 |
| hCV12066124 | rs2036914 | hCV12066119 | rs1912826 | 0.51 | 0.050680687 | 0.3713 |
| hCV12066124 | rs2036914 | hCV12066129 | rs1593 | 0.51 | 0.050680687 | 0.1505 |
| hCV12066124 | rs2036914 | hCV12086148 | rs1877321 | 0.51 | 0.050680687 | 0.0621 |
| hCV12066124 | rs2036914 | hCV15793897 | rs3087505 | 0.51 | 0.050680687 | 0.1103 |
| hCV12066124 | rs2036914 | hCV15811716 | rs2102575 | 0.51 | 0.050680687 | 0.1039 |
| hCV12066124 | rs2036914 | hCV15968025 | rs2292425 | 0.51 | 0.050680687 | 0.175 |
| hCV12066124 | rs2036914 | hCV15968026 | rs2292426 | 0.51 | 0.050680687 | 0.2128 |
| hCV12066124 | rs2036914 | hCV15968034 | rs2292428 | 0.51 | 0.050680687 | 0.181 |
| hCV12066124 | rs2036914 | hCV15968043 | rs2292423 | 0.51 | 0.050680687 | 0.3742 |
| hCV12066124 | rs2036914 | hCV15975109 | rs2304596 | 0.51 | 0.050680687 | 0.0738 |
| hCV12066124 | rs2036914 | hCV16172925 | rs2241818 | 0.51 | 0.050680687 | 0.0795 |
| hCV12066124 | rs2036914 | hCV16172935 | rs2241817 | 0.51 | 0.050680687 | 0.4102 |
| hCV12066124 | rs2036914 | hCV2103337 | rs13102931 | 0.51 | 0.050680687 | 0.0611 |
| hCV12066124 | rs2036914 | hCV2103343 | rs4241824 | 0.51 | 0.050680687 | 0.9265 |
| hCV12066124 | rs2036914 | hCV2103375 | rs12502630 | 0.51 | 0.050680687 | 0.0643 |
| hCV12066124 | rs2036914 | hCV2103388 | rs4613610 | 0.51 | 0.050680687 | 0.0917 |
| hCV12066124 | rs2036914 | hCV2103391 | rs1008728 | 0.51 | 0.050680687 | 0.2419 |
| hCV12066124 | rs2036914 | hCV2103392 | rs12500826 | 0.51 | 0.050680687 | 0.3937 |
| hCV12066124 | rs2036914 | hCV2103401 | rs7687352 | 0.51 | 0.050680687 | 0.0531 |
| hCV12066124 | rs2036914 | hCV2103402 | rs9993749 | 0.51 | 0.050680687 | 0.0695 |
| hCV12066124 | rs2036914 | hCV22272267 | rs3733402 | 0.51 | 0.050680687 | 0.3605 |
| hCV12066124 | rs2036914 | hCV25474413 | rs3822057 | 0.51 | 0.050680687 | 0.9449 |
| hCV12066124 | rs2036914 | hCV25474414 | rs4253399 | 0.51 | 0.050680687 | 0.5632 |
| hCV12066124 | rs2036914 | hCV25634763 | rs4253241 | 0.51 | 0.050680687 | 0.0841 |
| hCV12066124 | rs2036914 | hCV25988221 | rs9995366 | 0.51 | 0.050680687 | 0.0931 |
| hCV12066124 | rs2036914 | hCV25989001 | hCV25989001 | 0.51 | 0.050680687 | 0.0578 |
| hCV12066124 | rs2036914 | hCV25990131 | rs13146272 | 0.51 | 0.050680687 | 0.1776 |
| hCV12066124 | rs2036914 | hCV26038139 | rs4253405 | 0.51 | 0.050680687 | 0.5831 |
| hCV12066124 | rs2036914 | hCV26265197 | rs10014399 | 0.51 | 0.050680687 | 0.0507 |
| hCV12066124 | rs2036914 | hCV26265231 | rs7684025 | 0.51 | 0.050680687 | 0.3217 |
| hCV12066124 | rs2036914 | hCV27309991 | rs4572916 | 0.51 | 0.050680687 | 0.1646 |
| hCV12066124 | rs2036914 | hCV27473099 | rs3733403 | 0.51 | 0.050680687 | 0.1015 |
| hCV12066124 | rs2036914 | hCV27474895 | rs3756011 | 0.51 | 0.050680687 | 0.4851 |
| hCV12066124 | rs2036914 | hCV27477533 | rs3756008 | 0.51 | 0.050680687 | 0.5443 |
| hCV12066124 | rs2036914 | hCV27482765 | rs3775301 | 0.51 | 0.050680687 | 0.0738 |

## TABLE 3, page 15 of 71

| Interrogated SNP | Interrogated rs | LD SNP | LD SNP rs | Power | Threshold r² | r² |
|---|---|---|---|---|---|---|
| hCV12066124 | rs2036914 | hCV27490984 | rs3822058 | 0.51 | 0.050680687 | 0.4255 |
| hCV12066124 | rs2036914 | hCV27521729 | rs3822056 | 0.51 | 0.050680687 | 0.1148 |
| hCV12066124 | rs2036914 | hCV27902803 | rs4862665 | 0.51 | 0.050680687 | 0.0931 |
| hCV12066124 | rs2036914 | hCV27902808 | rs4253236 | 0.51 | 0.050680687 | 0.1725 |
| hCV12066124 | rs2036914 | hCV28960679 | rs6844764 | 0.51 | 0.050680687 | 0.1096 |
| hCV12066124 | rs2036914 | hCV29053261 | rs6842047 | 0.51 | 0.050680687 | 0.1103 |
| hCV12066124 | rs2036914 | hCV29053264 | rs7667777 | 0.51 | 0.050680687 | 0.2682 |
| hCV12066124 | rs2036914 | hCV29053265 | rs4253244 | 0.51 | 0.050680687 | 0.1619 |
| hCV12066124 | rs2036914 | hCV29419315 | rs6841024 | 0.51 | 0.050680687 | 0.1051 |
| hCV12066124 | rs2036914 | hCV29640635 | rs10029715 | 0.51 | 0.050680687 | 0.099 |
| hCV12066124 | rs2036914 | hCV29718000 | rs4253238 | 0.51 | 0.050680687 | 0.4135 |
| hCV12066124 | rs2036914 | hCV29826351 | rs10025990 | 0.51 | 0.050680687 | 0.1626 |
| hCV12066124 | rs2036914 | hCV29877725 | rs4253295 | 0.51 | 0.050680687 | 0.3398 |
| hCV12066124 | rs2036914 | hCV30307525 | rs10025152 | 0.51 | 0.050680687 | 0.099 |
| hCV12066124 | rs2036914 | hCV30492573 | rs10471184 | 0.51 | 0.050680687 | 0.1103 |
| hCV12066124 | rs2036914 | hCV30562347 | rs4253418 | 0.51 | 0.050680687 | 0.0632 |
| hCV12066124 | rs2036914 | hCV30983902 | rs4862668 | 0.51 | 0.050680687 | 0.1385 |
| hCV12066124 | rs2036914 | hCV30983907 | rs4253246 | 0.51 | 0.050680687 | 0.0841 |
| hCV12066124 | rs2036914 | hCV30983927 | rs6552962 | 0.51 | 0.050680687 | 0.0526 |
| hCV12066124 | rs2036914 | hCV32209629 | rs12715865 | 0.51 | 0.050680687 | 0.1168 |
| hCV12066124 | rs2036914 | hCV32209636 | rs11132387 | 0.51 | 0.050680687 | 0.4117 |
| hCV12066124 | rs2036914 | hCV32209637 | rs13143773 | 0.51 | 0.050680687 | 0.3327 |
| hCV12066124 | rs2036914 | hCV32209638 | rs12507040 | 0.51 | 0.050680687 | 0.387 |
| hCV12066124 | rs2036914 | hCV32291217 | rs4253323 | 0.51 | 0.050680687 | 0.0738 |
| hCV12066124 | rs2036914 | hCV32291256 | rs4253406 | 0.51 | 0.050680687 | 0.0631 |
| hCV12066124 | rs2036914 | hCV32291269 | rs4253417 | 0.51 | 0.050680687 | 0.389 |
| hCV12066124 | rs2036914 | hCV32291286 | rs4253422 | 0.51 | 0.050680687 | 0.2525 |
| hCV12066124 | rs2036914 | hCV32291287 | rs4253423 | 0.51 | 0.050680687 | 0.2525 |
| hCV12066124 | rs2036914 | hCV32291295 | rs4253292 | 0.51 | 0.050680687 | 0.1224 |
| hCV12066124 | rs2036914 | hCV32291301 | rs4253302 | 0.51 | 0.050680687 | 0.0694 |
| hCV12066124 | rs2036914 | hCV32295028 | rs4253260 | 0.51 | 0.050680687 | 0.0738 |
| hCV12066124 | rs2036914 | hCV3229991 | rs4241815 | 0.51 | 0.050680687 | 0.3605 |
| hCV12066124 | rs2036914 | hCV3229992 | rs3775298 | 0.51 | 0.050680687 | 0.3605 |
| hCV12066124 | rs2036914 | hCV3229995 | rs11132382 | 0.51 | 0.050680687 | 0.3958 |
| hCV12066124 | rs2036914 | hCV3230000 | rs4253294 | 0.51 | 0.050680687 | 0.1496 |
| hCV12066124 | rs2036914 | hCV3230001 | rs4253296 | 0.51 | 0.050680687 | 0.0841 |
| hCV12066124 | rs2036914 | hCV3230002 | rs4253297 | 0.51 | 0.050680687 | 0.3058 |
| hCV12066124 | rs2036914 | hCV3230003 | rs2304595 | 0.51 | 0.050680687 | 0.4092 |
| hCV12066124 | rs2036914 | hCV3230004 | rs4253301 | 0.51 | 0.050680687 | 0.1069 |
| hCV12066124 | rs2036914 | hCV3230006 | rs4253308 | 0.51 | 0.050680687 | 0.3398 |
| hCV12066124 | rs2036914 | hCV3230007 | rs4253311 | 0.51 | 0.050680687 | 0.3605 |
| hCV12066124 | rs2036914 | hCV3230011 | rs4253320 | 0.51 | 0.050680687 | 0.3058 |
| hCV12066124 | rs2036914 | hCV3230013 | rs3775303 | 0.51 | 0.050680687 | 0.3572 |
| hCV12066124 | rs2036914 | hCV3230014 | rs4861709 | 0.51 | 0.050680687 | 0.1496 |
| hCV12066124 | rs2036914 | hCV3230017 | rs4253327 | 0.51 | 0.050680687 | 0.0613 |
| hCV12066124 | rs2036914 | hCV3230018 | rs925453 | 0.51 | 0.050680687 | 0.1526 |
| hCV12066124 | rs2036914 | hCV3230019 | rs4253332 | 0.51 | 0.050680687 | 0.1452 |
| hCV12066124 | rs2036914 | hCV3230021 | rs13135645 | 0.51 | 0.050680687 | 0.154 |
| hCV12066124 | rs2036914 | hCV3230022 | rs11132383 | 0.51 | 0.050680687 | 0.1678 |
| hCV12066124 | rs2036914 | hCV3230025 | rs3756009 | 0.51 | 0.050680687 | 0.5789 |
| hCV12066124 | rs2036914 | hCV3230030 | rs4253408 | 0.51 | 0.050680687 | 0.0667 |
| hCV12066124 | rs2036914 | hCV3230031 | rs4253419 | 0.51 | 0.050680687 | 0.2525 |
| hCV12066124 | rs2036914 | hCV3230038 | rs2289252 | 0.51 | 0.050680687 | 0.3834 |
| hCV12066124 | rs2036914 | hCV3230083 | rs10013653 | 0.51 | 0.050680687 | 0.3086 |
| hCV12066124 | rs2036914 | hCV3230084 | rs7682918 | 0.51 | 0.050680687 | 0.2285 |

## TABLE 3, page 16 of 71

| Interrogated SNP | Interrogated rs | LD SNP | LD SNP rs | Power | Threshold r² | r² |
|---|---|---|---|---|---|---|
| hCV12066124 | rs2036914 | hCV3230094 | rs7687818 | 0.51 | 0.050680687 | 0.3495 |
| hCV12066124 | rs2036914 | hCV3230096 | rs3817184 | 0.51 | 0.050680687 | 0.2824 |
| hCV12066124 | rs2036914 | hCV3230097 | rs3736455 | 0.51 | 0.050680687 | 0.2379 |
| hCV12066124 | rs2036914 | hCV3230101 | rs6835839 | 0.51 | 0.050680687 | 0.1143 |
| hCV12066124 | rs2036914 | hCV3230106 | rs1473597 | 0.51 | 0.050680687 | 0.1783 |
| hCV12066124 | rs2036914 | hCV3230110 | rs2276917 | 0.51 | 0.050680687 | 0.1882 |
| hCV12066124 | rs2036914 | hCV3230113 | rs1053094 | 0.51 | 0.050680687 | 0.3142 |
| hCV12066124 | rs2036914 | hCV3230118 | rs4253429 | 0.51 | 0.050680687 | 0.2525 |
| hCV12066124 | rs2036914 | hCV3230119 | rs4253430 | 0.51 | 0.050680687 | 0.4139 |
| hCV12066124 | rs2036914 | hCV3230125 | rs11938564 | 0.51 | 0.050680687 | 0.3091 |
| hCV12066124 | rs2036914 | hCV3230131 | rs13136269 | 0.51 | 0.050680687 | 0.387 |
| hCV12066124 | rs2036914 | hCV3230133 | rs12511874 | 0.51 | 0.050680687 | 0.3354 |
| hCV12066124 | rs2036914 | hCV3230134 | rs12500151 | 0.51 | 0.050680687 | 0.3713 |
| hCV12066124 | rs2036914 | hCV3230136 | rs13116273 | 0.51 | 0.050680687 | 0.3869 |
| hCV12066124 | rs2036914 | hCV32313006 | rs4253248 | 0.51 | 0.050680687 | 0.4015 |
| hCV12066124 | rs2036914 | hCV32313007 | rs4862666 | 0.51 | 0.050680687 | 0.0931 |
| hCV12066124 | rs2036914 | hCV32313024 | rs4253239 | 0.51 | 0.050680687 | 0.1224 |
| hCV12066124 | rs2036914 | hCV32358975 | rs4253255 | 0.51 | 0.050680687 | 0.3463 |
| hCV12066124 | rs2036914 | hCV32358984 | rs4253256 | 0.51 | 0.050680687 | 0.1734 |
| hCV12066124 | rs2036914 | hCV8241628 | rs907439 | 0.51 | 0.050680687 | 0.1646 |
| hCV12066124 | rs2036914 | hCV8241630 | rs925451 | 0.51 | 0.050680687 | 0.5632 |
| hCV12066124 | rs2036914 | hCV8241631 | rs1511802 | 0.51 | 0.050680687 | 0.3604 |
| hCV12066124 | rs2036914 | hCV8241632 | rs1511801 | 0.51 | 0.050680687 | 0.3736 |
| hCV12066124 | rs2036914 | hCV8241633 | rs1511800 | 0.51 | 0.050680687 | 0.0931 |
| hCV12066124 | rs2036914 | hDV71222711 | rs4253252 | 0.51 | 0.050680687 | 0.4015 |
| hCV1376266 | rs1654413 | hCV11977629 | rs1654459 | 0.51 | 0.544795666 | 0.7222 |
| hCV1376266 | rs1654413 | hCV1376257 | rs10416380 | 0.51 | 0.544795666 | 0.9433 |
| hCV1376266 | rs1654413 | hCV1376262 | rs1671150 | 0.51 | 0.544795666 | 1 |
| hCV1376266 | rs1654413 | hCV1376264 | rs1671151 | 0.51 | 0.544795666 | 1 |
| hCV1376266 | rs1654413 | hCV1376265 | rs1671152 | 0.51 | 0.544795666 | 0.8286 |
| hCV1376266 | rs1654413 | hCV1376342 | rs1654416 | 0.51 | 0.544795666 | 1 |
| hCV1376266 | rs1654413 | hCV1376359 | rs2886412 | 0.51 | 0.544795666 | 1 |
| hCV1376266 | rs1654413 | hCV15973734 | rs2304167 | 0.51 | 0.544795666 | 1 |
| hCV1376266 | rs1654413 | hCV16044361 | rs2569513 | 0.51 | 0.544795666 | 0.7222 |
| hCV1376266 | rs1654413 | hCV26895244 | rs1671153 | 0.51 | 0.544795666 | 1 |
| hCV1376266 | rs1654413 | hCV26895257 | rs2886415 | 0.51 | 0.544795666 | 1 |
| hCV1376266 | rs1654413 | hCV29271569 | rs1626971 | 0.51 | 0.544795666 | 0.7269 |
| hCV1376266 | rs1654413 | hCV31722831 | rs11671922 | 0.51 | 0.544795666 | 1 |
| hCV1376266 | rs1654413 | hCV31722832 | rs11084381 | 0.51 | 0.544795666 | 1 |
| hCV1376266 | rs1654413 | hCV31722834 | rs11084382 | 0.51 | 0.544795666 | 0.8448 |
| hCV1376266 | rs1654413 | hCV31722835 | rs11668169 | 0.51 | 0.544795666 | 1 |
| hCV1376266 | rs1654413 | hCV31722836 | rs11672026 | 0.51 | 0.544795666 | 1 |
| hCV1376266 | rs1654413 | hCV7841075 | rs1671196 | 0.51 | 0.544795666 | 1 |
| hCV1376266 | rs1654413 | hCV8703249 | rs1654444 | 0.51 | 0.544795666 | 0.7222 |
| hCV1376266 | rs1654413 | hCV8704962 | rs775893 | 0.51 | 0.544795666 | 0.5627 |
| hCV1376266 | rs1654413 | hCV8717752 | rs1671217 | 0.51 | 0.544795666 | 0.7269 |
| hCV1376266 | rs1654413 | hCV8717761 | rs1654439 | 0.51 | 0.544795666 | 0.675 |
| hCV1376266 | rs1654413 | hCV8717793 | rs1654433 | 0.51 | 0.544795666 | 0.7222 |
| hCV1376266 | rs1654413 | hCV8717794 | rs1654432 | 0.51 | 0.544795666 | 0.7222 |
| hCV1376266 | rs1654413 | hCV8717845 | rs892090 | 0.51 | 0.544795666 | 0.8292 |
| hCV1376266 | rs1654413 | hCV8717846 | rs892089 | 0.51 | 0.544795666 | 1 |
| hCV1376266 | rs1654413 | hCV8717871 | rs1654421 | 0.51 | 0.544795666 | 1 |
| hCV1376266 | rs1654413 | hCV8717873 | rs1613662 | 0.51 | 0.544795666 | 0.8292 |
| hCV1376266 | rs1654413 | hCV8717881 | rs1654420 | 0.51 | 0.544795666 | 1 |
| hCV1376266 | rs1654413 | hCV8717893 | rs1671192 | 0.51 | 0.544795666 | 1 |

# TABLE 3, page 17 of 71

| Interrogated SNP | Interrogated rs | LD SNP | LD SNP rs | Power | Threshold r² | r² |
|---|---|---|---|---|---|---|
| hCV1376266 | rs1654413 | hCV8718961 | rs1654451 | 0.51 | 0.544795666 | 0.7211 |
| hCV1376266 | rs1654413 | hCV8718972 | rs1654447 | 0.51 | 0.544795666 | 0.7222 |
| hCV1376266 | rs1654413 | hCV9490926 | rs1654419 | 0.51 | 0.544795666 | 1 |
| hCV1376342 | rs1654416 | hCV11977629 | rs1654459 | 0.51 | 0.409514099 | 0.5842 |
| hCV1376342 | rs1654416 | hCV1376257 | rs10416380 | 0.51 | 0.409514099 | 0.9457 |
| hCV1376342 | rs1654416 | hCV1376262 | rs1671150 | 0.51 | 0.409514099 | 0.9724 |
| hCV1376342 | rs1654416 | hCV1376264 | rs1671151 | 0.51 | 0.409514099 | 0.9724 |
| hCV1376342 | rs1654416 | hCV1376265 | rs1671152 | 0.51 | 0.409514099 | 0.7822 |
| hCV1376342 | rs1654416 | hCV1376266 | rs1654413 | 0.51 | 0.409514099 | 1 |
| hCV1376342 | rs1654416 | hCV1376359 | rs2886412 | 0.51 | 0.409514099 | 1 |
| hCV1376342 | rs1654416 | hCV15973734 | rs2304167 | 0.51 | 0.409514099 | 0.9724 |
| hCV1376342 | rs1654416 | hCV16044361 | rs2569513 | 0.51 | 0.409514099 | 0.6123 |
| hCV1376342 | rs1654416 | hCV26895244 | rs1671153 | 0.51 | 0.409514099 | 0.9724 |
| hCV1376342 | rs1654416 | hCV26895257 | rs2886415 | 0.51 | 0.409514099 | 1 |
| hCV1376342 | rs1654416 | hCV29271569 | rs1626971 | 0.51 | 0.409514099 | 0.7325 |
| hCV1376342 | rs1654416 | hCV31722831 | rs11671922 | 0.51 | 0.409514099 | 1 |
| hCV1376342 | rs1654416 | hCV31722832 | rs11084381 | 0.51 | 0.409514099 | 0.9207 |
| hCV1376342 | rs1654416 | hCV31722834 | rs11084382 | 0.51 | 0.409514099 | 0.8079 |
| hCV1376342 | rs1654416 | hCV31722835 | rs11668169 | 0.51 | 0.409514099 | 0.9205 |
| hCV1376342 | rs1654416 | hCV31722836 | rs11672026 | 0.51 | 0.409514099 | 0.9163 |
| hCV1376342 | rs1654416 | hCV7841075 | rs1671196 | 0.51 | 0.409514099 | 0.9207 |
| hCV1376342 | rs1654416 | hCV8703249 | rs1654444 | 0.51 | 0.409514099 | 0.633 |
| hCV1376342 | rs1654416 | hCV8704962 | rs775893 | 0.51 | 0.409514099 | 0.4637 |
| hCV1376342 | rs1654416 | hCV8717752 | rs1671217 | 0.51 | 0.409514099 | 0.7325 |
| hCV1376342 | rs1654416 | hCV8717761 | rs1654439 | 0.51 | 0.409514099 | 0.5468 |
| hCV1376342 | rs1654416 | hCV8717793 | rs1654433 | 0.51 | 0.409514099 | 0.6123 |
| hCV1376342 | rs1654416 | hCV8717794 | rs1654432 | 0.51 | 0.409514099 | 0.6123 |
| hCV1376342 | rs1654416 | hCV8717845 | rs892090 | 0.51 | 0.409514099 | 0.7313 |
| hCV1376342 | rs1654416 | hCV8717846 | rs892089 | 0.51 | 0.409514099 | 1 |
| hCV1376342 | rs1654416 | hCV8717871 | rs1654421 | 0.51 | 0.409514099 | 0.7784 |
| hCV1376342 | rs1654416 | hCV8717873 | rs1613662 | 0.51 | 0.409514099 | 0.7313 |
| hCV1376342 | rs1654416 | hCV8717881 | rs1654420 | 0.51 | 0.409514099 | 0.9205 |
| hCV1376342 | rs1654416 | hCV8717893 | rs1671192 | 0.51 | 0.409514099 | 1 |
| hCV1376342 | rs1654416 | hCV8718961 | rs1654451 | 0.51 | 0.409514099 | 0.5824 |
| hCV1376342 | rs1654416 | hCV8718972 | rs1654447 | 0.51 | 0.409514099 | 0.6338 |
| hCV1376342 | rs1654416 | hCV9490926 | rs1654419 | 0.51 | 0.409514099 | 0.9205 |
| hCV15793897 | rs3087505 | hCV11786203 | rs4253251 | 0.51 | 0.201010916 | 0.4643 |
| hCV15793897 | rs3087505 | hCV11786295 | rs4253421 | 0.51 | 0.201010916 | 0.6968 |
| hCV15793897 | rs3087505 | hCV12066116 | rs1877320 | 0.51 | 0.201010916 | 0.8216 |
| hCV15793897 | rs3087505 | hCV12066129 | rs1593 | 0.51 | 0.201010916 | 0.7273 |
| hCV15793897 | rs3087505 | hCV15811716 | rs2102575 | 0.51 | 0.201010916 | 0.9425 |
| hCV15793897 | rs3087505 | hCV15968026 | rs2292426 | 0.51 | 0.201010916 | 0.2078 |
| hCV15793897 | rs3087505 | hCV2103388 | rs4613610 | 0.51 | 0.201010916 | 0.4687 |
| hCV15793897 | rs3087505 | hCV22271609 | rs4253326 | 0.51 | 0.201010916 | 0.4122 |
| hCV15793897 | rs3087505 | hCV25634781 | rs4253299 | 0.51 | 0.201010916 | 0.4656 |
| hCV15793897 | rs3087505 | hCV25988221 | rs9995366 | 0.51 | 0.201010916 | 0.838 |
| hCV15793897 | rs3087505 | hCV26265197 | rs10014399 | 0.51 | 0.201010916 | 0.4643 |
| hCV15793897 | rs3087505 | hCV26265199 | rs2221843 | 0.51 | 0.201010916 | 0.4656 |
| hCV15793897 | rs3087505 | hCV27309991 | rs4572916 | 0.51 | 0.201010916 | 0.4643 |
| hCV15793897 | rs3087505 | hCV27506149 | rs3822055 | 0.51 | 0.201010916 | 0.4656 |
| hCV15793897 | rs3087505 | hCV27902803 | rs4862665 | 0.51 | 0.201010916 | 0.838 |
| hCV15793897 | rs3087505 | hCV29053261 | rs6842047 | 0.51 | 0.201010916 | 0.8875 |
| hCV15793897 | rs3087505 | hCV29053266 | rs7687961 | 0.51 | 0.201010916 | 0.4508 |
| hCV15793897 | rs3087505 | hCV29640635 | rs10029715 | 0.51 | 0.201010916 | 0.362 |
| hCV15793897 | rs3087505 | hCV29826351 | rs10025990 | 0.51 | 0.201010916 | 0.9133 |

# TABLE 3, page 18 of 71

| Interrogated SNP | Interrogated rs | LD SNP | LD SNP rs | Power | Threshold r² | r² |
|---|---|---|---|---|---|---|
| hCV15793897 | rs3087505 | hCV30307525 | rs10025152 | 0.51 | 0.201010916 | 0.362 |
| hCV15793897 | rs3087505 | hCV30492573 | rs10471184 | 0.51 | 0.201010916 | 0.8875 |
| hCV15793897 | rs3087505 | hCV30562347 | rs4253418 | 0.51 | 0.201010916 | 0.2672 |
| hCV15793897 | rs3087505 | hCV30983902 | rs4862668 | 0.51 | 0.201010916 | 0.8216 |
| hCV15793897 | rs3087505 | hCV30983927 | rs6552962 | 0.51 | 0.201010916 | 0.4909 |
| hCV15793897 | rs3087505 | hCV32209629 | rs12715865 | 0.51 | 0.201010916 | 0.4136 |
| hCV15793897 | rs3087505 | hCV32209635 | rs6848311 | 0.51 | 0.201010916 | 0.2855 |
| hCV15793897 | rs3087505 | hCV32291246 | rs4253403 | 0.51 | 0.201010916 | 0.2073 |
| hCV15793897 | rs3087505 | hCV3230000 | rs4253294 | 0.51 | 0.201010916 | 0.2753 |
| hCV15793897 | rs3087505 | hCV3230012 | rs4241821 | 0.51 | 0.201010916 | 0.4656 |
| hCV15793897 | rs3087505 | hCV3230014 | rs4861709 | 0.51 | 0.201010916 | 0.2753 |
| hCV15793897 | rs3087505 | hCV3230017 | rs4253327 | 0.51 | 0.201010916 | 0.2509 |
| hCV15793897 | rs3087505 | hCV3230018 | rs925453 | 0.51 | 0.201010916 | 0.2553 |
| hCV15793897 | rs3087505 | hCV3230019 | rs4253332 | 0.51 | 0.201010916 | 0.2614 |
| hCV15793897 | rs3087505 | hCV32313007 | rs4862666 | 0.51 | 0.201010916 | 0.838 |
| hCV15793897 | rs3087505 | hCV8241628 | rs907439 | 0.51 | 0.201010916 | 0.4643 |
| hCV15793897 | rs3087505 | hCV8241633 | rs1511800 | 0.51 | 0.201010916 | 0.838 |
| hCV15860433 | rs2070006 | hCV11503382 | rs1873369 | 0.51 | 0.09197249 | 0.2934 |
| hCV15860433 | rs2070006 | hCV11503414 | rs2066865 | 0.51 | 0.09197249 | 0.4534 |
| hCV15860433 | rs2070006 | hCV11503416 | rs2066864 | 0.51 | 0.09197249 | 0.506 |
| hCV15860433 | rs2070006 | hCV11503431 | rs2066861 | 0.51 | 0.09197249 | 0.446 |
| hCV15860433 | rs2070006 | hCV11503469 | rs2066854 | 0.51 | 0.09197249 | 0.5293 |
| hCV15860433 | rs2070006 | hCV11503470 | rs1800788 | 0.51 | 0.09197249 | 0.2862 |
| hCV15860433 | rs2070006 | hCV11852898 | rs6819508 | 0.51 | 0.09197249 | 0.1131 |
| hCV15860433 | rs2070006 | hCV11853387 | rs1490683 | 0.51 | 0.09197249 | 0.0995 |
| hCV15860433 | rs2070006 | hCV11853483 | rs12644950 | 0.51 | 0.09197249 | 0.4932 |
| hCV15860433 | rs2070006 | hCV11853489 | rs7681423 | 0.51 | 0.09197249 | 0.506 |
| hCV15860433 | rs2070006 | hCV11853496 | rs7654093 | 0.51 | 0.09197249 | 0.446 |
| hCV15860433 | rs2070006 | hCV21680 | rs7666020 | 0.51 | 0.09197249 | 0.1304 |
| hCV15860433 | rs2070006 | hCV21681 | rs6536018 | 0.51 | 0.09197249 | 0.1435 |
| hCV15860433 | rs2070006 | hCV22273499 | rs7668014 | 0.51 | 0.09197249 | 0.2505 |
| hCV15860433 | rs2070006 | hCV22274180 | rs11935584 | 0.51 | 0.09197249 | 0.2386 |
| hCV15860433 | rs2070006 | hCV2407354 | rs276166 | 0.51 | 0.09197249 | 0.1025 |
| hCV15860433 | rs2070006 | hCV24834 | rs4235247 | 0.51 | 0.09197249 | 0.2186 |
| hCV15860433 | rs2070006 | hCV25610762 | rs7668818 | 0.51 | 0.09197249 | 0.1508 |
| hCV15860433 | rs2070006 | hCV26019871 | rs4547780 | 0.51 | 0.09197249 | 0.174 |
| hCV15860433 | rs2070006 | hCV26024202 | rs11731813 | 0.51 | 0.09197249 | 0.2684 |
| hCV15860433 | rs2070006 | hCV27020269 | rs7659613 | 0.51 | 0.09197249 | 0.9639 |
| hCV15860433 | rs2070006 | hCV27020277 | rs6825454 | 0.51 | 0.09197249 | 0.4782 |
| hCV15860433 | rs2070006 | hCV27020280 | rs4463047 | 0.51 | 0.09197249 | 0.1824 |
| hCV15860433 | rs2070006 | hCV27313130 | rs4634202 | 0.51 | 0.09197249 | 0.443 |
| hCV15860433 | rs2070006 | hCV27313137 | rs12645631 | 0.51 | 0.09197249 | 0.1049 |
| hCV15860433 | rs2070006 | hCV27905214 | rs4323084 | 0.51 | 0.09197249 | 0.3386 |
| hCV15860433 | rs2070006 | hCV27907560 | rs4696576 | 0.51 | 0.09197249 | 0.1561 |
| hCV15860433 | rs2070006 | hCV27937396 | rs4634201 | 0.51 | 0.09197249 | 0.239 |
| hCV15860433 | rs2070006 | hCV2892859 | rs13130318 | 0.51 | 0.09197249 | 0.4434 |
| hCV15860433 | rs2070006 | hCV2892869 | rs13109457 | 0.51 | 0.09197249 | 0.5189 |
| hCV15860433 | rs2070006 | hCV2892870 | rs2070011 | 0.51 | 0.09197249 | 0.9612 |
| hCV15860433 | rs2070006 | hCV2892876 | rs2070018 | 0.51 | 0.09197249 | 0.2139 |
| hCV15860433 | rs2070006 | hCV2892877 | rs6050 | 0.51 | 0.09197249 | 0.4576 |
| hCV15860433 | rs2070006 | hCV2892878 | rs2070022 | 0.51 | 0.09197249 | 0.104 |
| hCV15860433 | rs2070006 | hCV2892893 | rs12648258 | 0.51 | 0.09197249 | 0.2624 |
| hCV15860433 | rs2070006 | hCV2892895 | rs12641958 | 0.51 | 0.09197249 | 0.2505 |
| hCV15860433 | rs2070006 | hCV2892896 | rs11940724 | 0.51 | 0.09197249 | 0.2505 |
| hCV15860433 | rs2070006 | hCV2892899 | rs7680155 | 0.51 | 0.09197249 | 0.2386 |

## TABLE 3, page 19 of 71

| Interrogated SNP | Interrogated rs | LD SNP | LD SNP rs | Power | Threshold $r^2$ | $r^2$ |
|---|---|---|---|---|---|---|
| hCV15860433 | rs2070006 | hCV2892905 | rs12642770 | 0.51 | 0.09197249 | 0.3701 |
| hCV15860433 | rs2070006 | hCV2892918 | rs12511469 | 0.51 | 0.09197249 | 0.2997 |
| hCV15860433 | rs2070006 | hCV2892926 | rs7662567 | 0.51 | 0.09197249 | 0.2596 |
| hCV15860433 | rs2070006 | hCV2892928 | rs13147579 | 0.51 | 0.09197249 | 0.2712 |
| hCV15860433 | rs2070006 | hCV28953838 | rs7690851 | 0.51 | 0.09197249 | 0.1824 |
| hCV15860433 | rs2070006 | hCV28953840 | rs6536017 | 0.51 | 0.09197249 | 0.1839 |
| hCV15860433 | rs2070006 | hCV28966638 | rs7676857 | 0.51 | 0.09197249 | 0.1018 |
| hCV15860433 | rs2070006 | hCV29420822 | rs4642230 | 0.51 | 0.09197249 | 0.2828 |
| hCV15860433 | rs2070006 | hCV29420827 | rs7654425 | 0.51 | 0.09197249 | 0.2505 |
| hCV15860433 | rs2070006 | hCV29420828 | rs7660120 | 0.51 | 0.09197249 | 0.2053 |
| hCV15860433 | rs2070006 | hCV29570696 | rs9997519 | 0.51 | 0.09197249 | 0.1033 |
| hCV15860433 | rs2070006 | hCV29582612 | rs4550901 | 0.51 | 0.09197249 | 0.2139 |
| hCV15860433 | rs2070006 | hCV29983641 | rs10008078 | 0.51 | 0.09197249 | 0.2893 |
| hCV15860433 | rs2070006 | hCV30285831 | rs10013914 | 0.51 | 0.09197249 | 0.1353 |
| hCV15860433 | rs2070006 | hCV30679170 | rs13148992 | 0.51 | 0.09197249 | 0.2746 |
| hCV15860433 | rs2070006 | hCV30711231 | rs12642469 | 0.51 | 0.09197249 | 0.2893 |
| hCV15860433 | rs2070006 | hCV31863979 | rs12186294 | 0.51 | 0.09197249 | 0.5331 |
| hCV15860433 | rs2070006 | hCV31863982 | rs7659024 | 0.51 | 0.09197249 | 0.446 |
| hCV15860433 | rs2070006 | hCV31863989 | rs4308349 | 0.51 | 0.09197249 | 0.2709 |
| hCV15860433 | rs2070006 | hCV31863993 | rs7673587 | 0.51 | 0.09197249 | 0.2386 |
| hCV15860433 | rs2070006 | hCV32212659 | rs4622984 | 0.51 | 0.09197249 | 0.385 |
| hCV15860433 | rs2070006 | hCV32212662 | rs11099958 | 0.51 | 0.09197249 | 0.1092 |
| hCV15860433 | rs2070006 | hCV32212663 | rs7670827 | 0.51 | 0.09197249 | 0.1457 |
| hCV15860433 | rs2070006 | hCV32212669 | rs12649647 | 0.51 | 0.09197249 | 0.1198 |
| hCV15860433 | rs2070006 | hCV354895 | rs11737226 | 0.51 | 0.09197249 | 0.2725 |
| hCV15860433 | rs2070006 | hCV354896 | rs7690972 | 0.51 | 0.09197249 | 0.2725 |
| hCV15860433 | rs2070006 | hCV36809 | rs10517590 | 0.51 | 0.09197249 | 0.1026 |
| hCV15860433 | rs2070006 | hCV426173 | rs12504201 | 0.51 | 0.09197249 | 0.1421 |
| hCV15860433 | rs2070006 | hCV470979 | rs1490672 | 0.51 | 0.09197249 | 0.2591 |
| hCV15860433 | rs2070006 | hCV7428370 | rs1456450 | 0.51 | 0.09197249 | 0.1012 |
| hCV15860433 | rs2070006 | hCV7429780 | rs1800792 | 0.51 | 0.09197249 | 0.5074 |
| hCV15860433 | rs2070006 | hCV7429783 | rs1044291 | 0.51 | 0.09197249 | 0.2505 |
| hCV15860433 | rs2070006 | hCV7429793 | rs1025154 | 0.51 | 0.09197249 | 0.2893 |
| hCV15860433 | rs2070006 | hCV9317206 | rs2070008 | 0.51 | 0.09197249 | 0.252 |
| hCV15860433 | rs2070006 | hDV70945235 | rs17373860 | 0.51 | 0.09197249 | 0.1588 |
| hCV15860433 | rs2070006 | hDV72277158 | rs28673871 | 0.51 | 0.09197249 | 0.1082 |
| hCV15860433 | rs2070006 | hDV77232287 | rs7666918 | 0.51 | 0.09197249 | 0.2505 |
| hCV15949414 | rs2234628 | hCV11327199 | rs12637760 | 0.51 | 0.263389785 | 1 |
| hCV15949414 | rs2234628 | hCV15961938 | rs2284816 | 0.51 | 0.263389785 | 1 |
| hCV15949414 | rs2234628 | hCV16189344 | rs2298422 | 0.51 | 0.263389785 | 0.8478 |
| hCV15949414 | rs2234628 | hCV1845321 | rs12636358 | 0.51 | 0.263389785 | 1 |
| hCV15949414 | rs2234628 | hCV27512998 | rs3762790 | 0.51 | 0.263389785 | 1 |
| hCV15949414 | rs2234628 | hCV30700451 | rs12631864 | 0.51 | 0.263389785 | 1 |
| hCV15949414 | rs2234628 | hCV30700457 | rs12635900 | 0.51 | 0.263389785 | 0.9045 |
| hCV15949414 | rs2234628 | hCV3083980 | rs12637034 | 0.51 | 0.263389785 | 1 |
| hCV15949414 | rs2234628 | hCV32001449 | rs12636077 | 0.51 | 0.263389785 | 1 |
| hCV15949414 | rs2234628 | hDV70822211 | rs17037809 | 0.51 | 0.263389785 | 1 |
| hCV15949414 | rs2234628 | hDV70822215 | rs17037814 | 0.51 | 0.263389785 | 1 |
| hCV15949414 | rs2234628 | hDV70822219 | rs17037819 | 0.51 | 0.263389785 | 1 |
| hCV15949414 | rs2234628 | hDV71601922 | rs17037775 | 0.51 | 0.263389785 | 1 |
| hCV15949414 | rs2234628 | hDV76880100 | rs3749388 | 0.51 | 0.263389785 | 1 |
| hCV15968043 | rs2292423 | hCV11786147 | rs4862662 | 0.51 | 0.095896459 | 0.6131 |
| hCV15968043 | rs2292423 | hCV11786203 | rs4253251 | 0.51 | 0.095896459 | 0.1841 |
| hCV15968043 | rs2292423 | hCV11786235 | rs4253287 | 0.51 | 0.095896459 | 0.1251 |
| hCV15968043 | rs2292423 | hCV11786258 | rs4253303 | 0.51 | 0.095896459 | 0.8913 |

## TABLE 3, page 20 of 71

| Interrogated SNP | Interrogated rs | LD SNP | LD SNP rs | Power | Threshold r² | r² |
|---|---|---|---|---|---|---|
| hCV15968043 | rs2292423 | hCV11786259 | rs4253304 | 0.51 | 0.095896459 | 1 |
| hCV15968043 | rs2292423 | hCV11786327 | rs13133050 | 0.51 | 0.095896459 | 0.1279 |
| hCV15968043 | rs2292423 | hCV12066118 | rs2048 | 0.51 | 0.095896459 | 0.6531 |
| hCV15968043 | rs2292423 | hCV12066119 | rs1912826 | 0.51 | 0.095896459 | 0.594 |
| hCV15968043 | rs2292423 | hCV12066124 | rs2036914 | 0.51 | 0.095896459 | 0.3742 |
| hCV15968043 | rs2292423 | hCV1474481 | rs7693361 | 0.51 | 0.095896459 | 0.1013 |
| hCV15968043 | rs2292423 | hCV15968025 | rs2292425 | 0.51 | 0.095896459 | 0.2221 |
| hCV15968043 | rs2292423 | hCV15968026 | rs2292426 | 0.51 | 0.095896459 | 0.3124 |
| hCV15968043 | rs2292423 | hCV15968034 | rs2292428 | 0.51 | 0.095896459 | 0.3976 |
| hCV15968043 | rs2292423 | hCV15975109 | rs2304596 | 0.51 | 0.095896459 | 0.1471 |
| hCV15968043 | rs2292423 | hCV2103343 | rs4241824 | 0.51 | 0.095896459 | 0.3048 |
| hCV15968043 | rs2292423 | hCV2103391 | rs1008728 | 0.51 | 0.095896459 | 0.2022 |
| hCV15968043 | rs2292423 | hCV2103392 | rs12500826 | 0.51 | 0.095896459 | 0.179 |
| hCV15968043 | rs2292423 | hCV2103401 | rs7687352 | 0.51 | 0.095896459 | 0.1006 |
| hCV15968043 | rs2292423 | hCV2103402 | rs9993749 | 0.51 | 0.095896459 | 0.1071 |
| hCV15968043 | rs2292423 | hCV22271609 | rs4253326 | 0.51 | 0.095896459 | 0.1576 |
| hCV15968043 | rs2292423 | hCV22272267 | rs3733402 | 0.51 | 0.095896459 | 0.6588 |
| hCV15968043 | rs2292423 | hCV25474413 | rs3822057 | 0.51 | 0.095896459 | 0.313 |
| hCV15968043 | rs2292423 | hCV25474414 | rs4253399 | 0.51 | 0.095896459 | 0.338 |
| hCV15968043 | rs2292423 | hCV25634781 | rs4253299 | 0.51 | 0.095896459 | 0.179 |
| hCV15968043 | rs2292423 | hCV25989001 | hCV25989001 | 0.51 | 0.095896459 | 0.156 |
| hCV15968043 | rs2292423 | hCV25990131 | rs13146272 | 0.51 | 0.095896459 | 0.2261 |
| hCV15968043 | rs2292423 | hCV26038139 | rs4253405 | 0.51 | 0.095896459 | 0.1127 |
| hCV15968043 | rs2292423 | hCV26265197 | rs10014399 | 0.51 | 0.095896459 | 0.1871 |
| hCV15968043 | rs2292423 | hCV26265199 | rs2221843 | 0.51 | 0.095896459 | 0.179 |
| hCV15968043 | rs2292423 | hCV26265231 | rs7684025 | 0.51 | 0.095896459 | 0.6916 |
| hCV15968043 | rs2292423 | hCV27309991 | rs4572916 | 0.51 | 0.095896459 | 0.1062 |
| hCV15968043 | rs2292423 | hCV27474895 | rs3756011 | 0.51 | 0.095896459 | 0.1967 |
| hCV15968043 | rs2292423 | hCV27477533 | rs3756008 | 0.51 | 0.095896459 | 0.3624 |
| hCV15968043 | rs2292423 | hCV27482765 | rs3775301 | 0.51 | 0.095896459 | 0.1471 |
| hCV15968043 | rs2292423 | hCV27506149 | rs3822055 | 0.51 | 0.095896459 | 0.179 |
| hCV15968043 | rs2292423 | hCV27902808 | rs4253236 | 0.51 | 0.095896459 | 0.4376 |
| hCV15968043 | rs2292423 | hCV28960679 | rs6844764 | 0.51 | 0.095896459 | 0.2857 |
| hCV15968043 | rs2292423 | hCV29053260 | rs4861707 | 0.51 | 0.095896459 | 0.1391 |
| hCV15968043 | rs2292423 | hCV29053264 | rs7667777 | 0.51 | 0.095896459 | 0.6735 |
| hCV15968043 | rs2292423 | hCV29053265 | rs4253244 | 0.51 | 0.095896459 | 0.4158 |
| hCV15968043 | rs2292423 | hCV29640635 | rs10029715 | 0.51 | 0.095896459 | 0.1031 |
| hCV15968043 | rs2292423 | hCV29718000 | rs4253238 | 0.51 | 0.095896459 | 0.6615 |
| hCV15968043 | rs2292423 | hCV29826351 | rs10025990 | 0.51 | 0.095896459 | 0.0963 |
| hCV15968043 | rs2292423 | hCV29877725 | rs4253295 | 0.51 | 0.095896459 | 0.8892 |
| hCV15968043 | rs2292423 | hCV30307525 | rs10025152 | 0.51 | 0.095896459 | 0.1031 |
| hCV15968043 | rs2292423 | hCV32209636 | rs11132387 | 0.51 | 0.095896459 | 0.2244 |
| hCV15968043 | rs2292423 | hCV32209638 | rs12507040 | 0.51 | 0.095896459 | 0.108 |
| hCV15968043 | rs2292423 | hCV32291217 | rs4253323 | 0.51 | 0.095896459 | 0.1471 |
| hCV15968043 | rs2292423 | hCV32291269 | rs4253417 | 0.51 | 0.095896459 | 0.2504 |
| hCV15968043 | rs2292423 | hCV32291286 | rs4253422 | 0.51 | 0.095896459 | 0.0995 |
| hCV15968043 | rs2292423 | hCV32291287 | rs4253423 | 0.51 | 0.095896459 | 0.0995 |
| hCV15968043 | rs2292423 | hCV32291295 | rs4253292 | 0.51 | 0.095896459 | 0.1503 |
| hCV15968043 | rs2292423 | hCV32291301 | rs4253302 | 0.51 | 0.095896459 | 0.1455 |
| hCV15968043 | rs2292423 | hCV32295028 | rs4253260 | 0.51 | 0.095896459 | 0.1471 |
| hCV15968043 | rs2292423 | hCV3229991 | rs4241815 | 0.51 | 0.095896459 | 0.6588 |
| hCV15968043 | rs2292423 | hCV3229992 | rs3775298 | 0.51 | 0.095896459 | 0.6588 |
| hCV15968043 | rs2292423 | hCV3229995 | rs11132382 | 0.51 | 0.095896459 | 0.6615 |
| hCV15968043 | rs2292423 | hCV3230000 | rs4253294 | 0.51 | 0.095896459 | 0.3176 |
| hCV15968043 | rs2292423 | hCV3230002 | rs4253297 | 0.51 | 0.095896459 | 0.8979 |

## TABLE 3, page 21 of 71

| Interrogated SNP | Interrogated rs | LD SNP | LD SNP rs | Power | Threshold r² | r² |
|---|---|---|---|---|---|---|
| hCV15968043 | rs2292423 | hCV3230003 | rs2304595 | 0.51 | 0.095896459 | 1 |
| hCV15968043 | rs2292423 | hCV3230006 | rs4253308 | 0.51 | 0.095896459 | 0.8892 |
| hCV15968043 | rs2292423 | hCV3230007 | rs4253311 | 0.51 | 0.095896459 | 0.6588 |
| hCV15968043 | rs2292423 | hCV3230011 | rs4253320 | 0.51 | 0.095896459 | 0.8979 |
| hCV15968043 | rs2292423 | hCV3230012 | rs4241821 | 0.51 | 0.095896459 | 0.179 |
| hCV15968043 | rs2292423 | hCV3230013 | rs3775303 | 0.51 | 0.095896459 | 1 |
| hCV15968043 | rs2292423 | hCV3230014 | rs4861709 | 0.51 | 0.095896459 | 0.3176 |
| hCV15968043 | rs2292423 | hCV3230017 | rs4253327 | 0.51 | 0.095896459 | 0.3086 |
| hCV15968043 | rs2292423 | hCV3230018 | rs925453 | 0.51 | 0.095896459 | 0.304 |
| hCV15968043 | rs2292423 | hCV3230019 | rs4253332 | 0.51 | 0.095896459 | 0.304 |
| hCV15968043 | rs2292423 | hCV3230021 | rs13135645 | 0.51 | 0.095896459 | 0.1067 |
| hCV15968043 | rs2292423 | hCV3230022 | rs11132383 | 0.51 | 0.095896459 | 0.2177 |
| hCV15968043 | rs2292423 | hCV3230025 | rs3756009 | 0.51 | 0.095896459 | 0.3012 |
| hCV15968043 | rs2292423 | hCV3230031 | rs4253419 | 0.51 | 0.095896459 | 0.0995 |
| hCV15968043 | rs2292423 | hCV3230038 | rs2289252 | 0.51 | 0.095896459 | 0.2462 |
| hCV15968043 | rs2292423 | hCV3230083 | rs10013653 | 0.51 | 0.095896459 | 0.5543 |
| hCV15968043 | rs2292423 | hCV3230084 | rs7682918 | 0.51 | 0.095896459 | 0.5227 |
| hCV15968043 | rs2292423 | hCV3230094 | rs7687818 | 0.51 | 0.095896459 | 0.7508 |
| hCV15968043 | rs2292423 | hCV3230096 | rs3817184 | 0.51 | 0.095896459 | 0.6453 |
| hCV15968043 | rs2292423 | hCV3230097 | rs3736455 | 0.51 | 0.095896459 | 0.3107 |
| hCV15968043 | rs2292423 | hCV3230101 | rs6835839 | 0.51 | 0.095896459 | 0.4126 |
| hCV15968043 | rs2292423 | hCV3230106 | rs1473597 | 0.51 | 0.095896459 | 0.4173 |
| hCV15968043 | rs2292423 | hCV3230110 | rs2276917 | 0.51 | 0.095896459 | 0.3976 |
| hCV15968043 | rs2292423 | hCV3230113 | rs1053094 | 0.51 | 0.095896459 | 0.59 |
| hCV15968043 | rs2292423 | hCV3230118 | rs4253429 | 0.51 | 0.095896459 | 0.0995 |
| hCV15968043 | rs2292423 | hCV3230125 | rs11938564 | 0.51 | 0.095896459 | 0.163 |
| hCV15968043 | rs2292423 | hCV3230131 | rs13136269 | 0.51 | 0.095896459 | 0.108 |
| hCV15968043 | rs2292423 | hCV3230133 | rs12511874 | 0.51 | 0.095896459 | 0.108 |
| hCV15968043 | rs2292423 | hCV3230134 | rs12500151 | 0.51 | 0.095896459 | 0.108 |
| hCV15968043 | rs2292423 | hCV3230136 | rs13116273 | 0.51 | 0.095896459 | 0.129 |
| hCV15968043 | rs2292423 | hCV32313006 | rs4253248 | 0.51 | 0.095896459 | 0.6615 |
| hCV15968043 | rs2292423 | hCV32313024 | rs4253239 | 0.51 | 0.095896459 | 0.1503 |
| hCV15968043 | rs2292423 | hCV32358975 | rs4253255 | 0.51 | 0.095896459 | 0.6531 |
| hCV15968043 | rs2292423 | hCV32358984 | rs4253256 | 0.51 | 0.095896459 | 0.4314 |
| hCV15968043 | rs2292423 | hCV8241628 | rs907439 | 0.51 | 0.095896459 | 0.1062 |
| hCV15968043 | rs2292423 | hCV8241630 | rs925451 | 0.51 | 0.095896459 | 0.338 |
| hCV15968043 | rs2292423 | hCV8241631 | rs1511802 | 0.51 | 0.095896459 | 0.8892 |
| hCV15968043 | rs2292423 | hCV8241632 | rs1511801 | 0.51 | 0.095896459 | 0.6519 |
| hCV15968043 | rs2292423 | hDV71222711 | rs4253252 | 0.51 | 0.095896459 | 0.6615 |
| hCV15968043 | rs2292423 | hDV76175111 | rs35079309 | 0.51 | 0.095896459 | 0.1621 |
| hCV15990789 | rs2355466 | hCV25743768 | rs4757548 | 0.51 | 0.951959625 | 0.9603 |
| hCV16177220 | rs2266911 | hCV2451164 | rs2357252 | 0.51 | 0.395362464 | 0.7006 |
| hCV16177220 | rs2266911 | hCV2451180 | rs2072886 | 0.51 | 0.395362464 | 0.4412 |
| hCV16177220 | rs2266911 | hCV29241293 | rs7061257 | 0.51 | 0.395362464 | 0.7143 |
| hCV16177220 | rs2266911 | hCV32361087 | rs4825898 | 0.51 | 0.395362464 | 0.893 |
| hCV16180170 | rs2227589 | hCV11342529 | rs1951627 | 0.51 | 0.229511448 | 0.3108 |
| hCV16180170 | rs2227589 | hCV11975630 | rs2065170 | 0.51 | 0.229511448 | 1 |
| hCV16180170 | rs2227589 | hCV15864094 | rs2068871 | 0.51 | 0.229511448 | 0.9425 |
| hCV16180170 | rs2227589 | hCV15956059 | rs2227592 | 0.51 | 0.229511448 | 1 |
| hCV16180170 | rs2227589 | hCV16135173 | rs2146372 | 0.51 | 0.229511448 | 1 |
| hCV16180170 | rs2227589 | hCV16290208 | rs2759328 | 0.51 | 0.229511448 | 1 |
| hCV16180170 | rs2227589 | hCV1681325 | rs898657 | 0.51 | 0.229511448 | 0.288 |
| hCV16180170 | rs2227589 | hCV1681328 | rs10912647 | 0.51 | 0.229511448 | 0.2457 |
| hCV16180170 | rs2227589 | hCV25600635 | rs7539322 | 0.51 | 0.229511448 | 0.8856 |
| hCV16180170 | rs2227589 | hCV25932979 | rs16846809 | 0.51 | 0.229511448 | 0.5549 |

## TABLE 3, page 22 of 71

| Interrogated SNP | Interrogated rs | LD SNP | LD SNP rs | Power | Threshold r² | r² |
|---|---|---|---|---|---|---|
| hCV16180170 | rs2227589 | hCV27483572 | rs3791022 | 0.51 | 0.229511448 | 1 |
| hCV16180170 | rs2227589 | hCV28998001 | rs6425251 | 0.51 | 0.229511448 | 0.2457 |
| hCV16180170 | rs2227589 | hCV29517287 | rs2901747 | 0.51 | 0.229511448 | 0.2436 |
| hCV16180170 | rs2227589 | hCV29989899 | rs6685043 | 0.51 | 0.229511448 | 0.6095 |
| hCV16180170 | rs2227589 | hCV30205817 | rs10489254 | 0.51 | 0.229511448 | 0.5549 |
| hCV16180170 | rs2227589 | hCV30404194 | rs6691053 | 0.51 | 0.229511448 | 0.3572 |
| hCV16180170 | rs2227589 | hCV30472885 | rs7520441 | 0.51 | 0.229511448 | 0.315 |
| hCV16180170 | rs2227589 | hCV30804119 | rs10912651 | 0.51 | 0.229511448 | 0.2376 |
| hCV16180170 | rs2227589 | hCV30804135 | rs12078293 | 0.51 | 0.229511448 | 0.2457 |
| hCV16180170 | rs2227589 | hCV30804139 | rs12089930 | 0.51 | 0.229511448 | 0.245 |
| hCV16180170 | rs2227589 | hCV8911729 | rs941987 | 0.51 | 0.229511448 | 0.8292 |
| hCV16180170 | rs2227589 | hCV8911768 | rs941988 | 0.51 | 0.229511448 | 1 |
| hCV16180170 | rs2227589 | hCV9575253 | rs1031751 | 0.51 | 0.229511448 | 0.3146 |
| hCV16180170 | rs2227589 | hCV9575263 | rs898658 | 0.51 | 0.229511448 | 0.2457 |
| hCV16180170 | rs2227589 | hDV70683090 | rs16846433 | 0.51 | 0.229511448 | 0.9425 |
| hCV16180170 | rs2227589 | hDV70683162 | rs16846526 | 0.51 | 0.229511448 | 1 |
| hCV16180170 | rs2227589 | hDV70683177 | rs16846546 | 0.51 | 0.229511448 | 1 |
| hCV16180170 | rs2227589 | hDV70683187 | rs16846561 | 0.51 | 0.229511448 | 1 |
| hCV16180170 | rs2227589 | hDV70683212 | rs16846593 | 0.51 | 0.229511448 | 0.5549 |
| hCV16180170 | rs2227589 | hDV70683382 | rs16846815 | 0.51 | 0.229511448 | 0.5078 |
| hCV16180170 | rs2227589 | hDV70934851 | rs17301125 | 0.51 | 0.229511448 | 0.2534 |
| hCV16182835 | rs2274736 | hCV11295871 | rs17203789 | 0.51 | 0.445188644 | 0.6809 |
| hCV16182835 | rs2274736 | hCV11295918 | rs12586348 | 0.51 | 0.445188644 | 0.6574 |
| hCV16182835 | rs2274736 | hCV11454301 | rs11159868 | 0.51 | 0.445188644 | 0.6481 |
| hCV16182835 | rs2274736 | hCV11454302 | rs7157149 | 0.51 | 0.445188644 | 0.6481 |
| hCV16182835 | rs2274736 | hCV11474667 | rs10150311 | 0.51 | 0.445188644 | 0.9163 |
| hCV16182835 | rs2274736 | hCV11474668 | rs10138002 | 0.51 | 0.445188644 | 0.9163 |
| hCV16182835 | rs2274736 | hCV11474679 | rs2778936 | 0.51 | 0.445188644 | 0.9591 |
| hCV16182835 | rs2274736 | hCV11657898 | rs1956406 | 0.51 | 0.445188644 | 0.5421 |
| hCV16182835 | rs2274736 | hCV11657912 | rs1950806 | 0.51 | 0.445188644 | 0.6481 |
| hCV16182835 | rs2274736 | hCV11666712 | rs1864747 | 0.51 | 0.445188644 | 0.919 |
| hCV16182835 | rs2274736 | hCV11666713 | rs1864746 | 0.51 | 0.445188644 | 0.919 |
| hCV16182835 | rs2274736 | hCV11666722 | rs1864748 | 0.51 | 0.445188644 | 0.6812 |
| hCV16182835 | rs2274736 | hCV11666724 | rs1864744 | 0.51 | 0.445188644 | 1 |
| hCV16182835 | rs2274736 | hCV11666737 | rs1955600 | 0.51 | 0.445188644 | 0.6562 |
| hCV16182835 | rs2274736 | hCV1262727 | rs12587200 | 0.51 | 0.445188644 | 0.6574 |
| hCV16182835 | rs2274736 | hCV1262753 | rs12436982 | 0.51 | 0.445188644 | 0.6322 |
| hCV16182835 | rs2274736 | hCV15870067 | rs2224333 | 0.51 | 0.445188644 | 0.6651 |
| hCV16182835 | rs2274736 | hCV16185886 | rs2297129 | 0.51 | 0.445188644 | 1 |
| hCV16182835 | rs2274736 | hCV16189259 | rs2295135 | 0.51 | 0.445188644 | 0.6601 |
| hCV16182835 | rs2274736 | hCV211940 | rs12587386 | 0.51 | 0.445188644 | 0.6583 |
| hCV16182835 | rs2274736 | hCV2231821 | rs453112 | 0.51 | 0.445188644 | 0.9596 |
| hCV16182835 | rs2274736 | hCV2485030 | rs12589467 | 0.51 | 0.445188644 | 0.6583 |
| hCV16182835 | rs2274736 | hCV2485038 | rs865285 | 0.51 | 0.445188644 | 0.8835 |
| hCV16182835 | rs2274736 | hCV2485039 | rs3179969 | 0.51 | 0.445188644 | 0.9793 |
| hCV16182835 | rs2274736 | hCV25933483 | rs10143744 | 0.51 | 0.445188644 | 0.879 |
| hCV16182835 | rs2274736 | hCV25935678 | rs4904452 | 0.51 | 0.445188644 | 0.9573 |
| hCV16182835 | rs2274736 | hCV25942539 | rs2401751 | 0.51 | 0.445188644 | 1 |
| hCV16182835 | rs2274736 | hCV27202496 | rs1099698 | 0.51 | 0.445188644 | 0.9558 |
| hCV16182835 | rs2274736 | hCV27202497 | rs12589480 | 0.51 | 0.445188644 | 0.6692 |
| hCV16182835 | rs2274736 | hCV27202543 | rs7146241 | 0.51 | 0.445188644 | 0.9591 |
| hCV16182835 | rs2274736 | hCV27202682 | rs10142228 | 0.51 | 0.445188644 | 0.4551 |
| hCV16182835 | rs2274736 | hCV27520559 | rs3814855 | 0.51 | 0.445188644 | 0.6697 |
| hCV16182835 | rs2274736 | hCV2796701 | rs9323834 | 0.51 | 0.445188644 | 0.4697 |
| hCV16182835 | rs2274736 | hCV2796704 | rs10134036 | 0.51 | 0.445188644 | 0.4645 |

| Interrogated SNP | Interrogated rs | LD SNP | LD SNP rs | Power | Threshold r² | r² |
|---|---|---|---|---|---|---|
| hCV16182835 | rs2274736 | hCV2796706 | rs9671813 | 0.51 | 0.445188644 | 0.4777 |
| hCV16182835 | rs2274736 | hCV29385782 | rs7141608 | 0.51 | 0.445188644 | 0.9591 |
| hCV16182835 | rs2274736 | hCV29385806 | rs8020072 | 0.51 | 0.445188644 | 0.6704 |
| hCV16182835 | rs2274736 | hCV29549024 | rs10137225 | 0.51 | 0.445188644 | 0.4489 |
| hCV16182835 | rs2274736 | hCV29567112 | rs10484010 | 0.51 | 0.445188644 | 0.4622 |
| hCV16182835 | rs2274736 | hCV29729918 | rs10143767 | 0.51 | 0.445188644 | 0.6878 |
| hCV16182835 | rs2274736 | hCV29910416 | rs10139817 | 0.51 | 0.445188644 | 0.4677 |
| hCV16182835 | rs2274736 | hCV30414828 | rs7144432 | 0.51 | 0.445188644 | 0.9596 |
| hCV16182835 | rs2274736 | hCV30414829 | rs10134008 | 0.51 | 0.445188644 | 0.6988 |
| hCV16182835 | rs2274736 | hCV30468559 | rs10132509 | 0.51 | 0.445188644 | 0.496 |
| hCV16182835 | rs2274736 | hCV32095372 | rs12586714 | 0.51 | 0.445188644 | 0.881 |
| hCV16182835 | rs2274736 | hCV32095396 | rs11845147 | 0.51 | 0.445188644 | 0.919 |
| hCV16182835 | rs2274736 | hCV32095401 | rs11847417 | 0.51 | 0.445188644 | 0.9163 |
| hCV16182835 | rs2274736 | hCV32095402 | rs11159857 | 0.51 | 0.445188644 | 0.919 |
| hCV16182835 | rs2274736 | hCV32095403 | rs4390529 | 0.51 | 0.445188644 | 0.917 |
| hCV16182835 | rs2274736 | hCV32095404 | rs4301952 | 0.51 | 0.445188644 | 0.919 |
| hCV16182835 | rs2274736 | hCV32095415 | rs12050316 | 0.51 | 0.445188644 | 0.8616 |
| hCV16182835 | rs2274736 | hCV32095422 | rs2033418 | 0.51 | 0.445188644 | 0.9135 |
| hCV16182835 | rs2274736 | hCV32095429 | rs12436642 | 0.51 | 0.445188644 | 0.9381 |
| hCV16182835 | rs2274736 | hCV32095430 | rs11159859 | 0.51 | 0.445188644 | 0.8539 |
| hCV16182835 | rs2274736 | hCV32095431 | rs11629164 | 0.51 | 0.445188644 | 0.3395 |
| hCV16182835 | rs2274736 | hCV32095460 | rs12434935 | 0.51 | 0.445188644 | 0.6651 |
| hCV16182835 | rs2274736 | hCV32095525 | rs12590826 | 0.51 | 0.445188644 | 0.6121 |
| hCV16182835 | rs2274736 | hCV32095533 | rs12588535 | 0.51 | 0.445188644 | 0.6651 |
| hCV16182835 | rs2274736 | hCV3211521 | rs12431548 | 0.51 | 0.445188644 | 0.6512 |
| hCV16182835 | rs2274736 | hCV3211539 | rs1998670 | 0.51 | 0.445188644 | 0.6891 |
| hCV16182835 | rs2274736 | hCV3211540 | rs2274735 | 0.51 | 0.445188644 | 0.9793 |
| hCV16182835 | rs2274736 | hCV3211544 | rs9323830 | 0.51 | 0.445188644 | 0.919 |
| hCV16182835 | rs2274736 | hCV3211545 | rs7160647 | 0.51 | 0.445188644 | 0.9163 |
| hCV16182835 | rs2274736 | hCV3211546 | rs7143642 | 0.51 | 0.445188644 | 0.919 |
| hCV16182835 | rs2274736 | hCV3211548 | rs7151164 | 0.51 | 0.445188644 | 0.919 |
| hCV16182835 | rs2274736 | hCV3211549 | rs12433026 | 0.51 | 0.445188644 | 0.8973 |
| hCV16182835 | rs2274736 | hCV3211559 | rs2004329 | 0.51 | 0.445188644 | 0.6919 |
| hCV16182835 | rs2274736 | hCV3211560 | rs12436326 | 0.51 | 0.445188644 | 0.6988 |
| hCV16182835 | rs2274736 | hCV3211561 | rs8017811 | 0.51 | 0.445188644 | 0.9581 |
| hCV16182835 | rs2274736 | hCV3211562 | rs4904454 | 0.51 | 0.445188644 | 0.9596 |
| hCV16182835 | rs2274736 | hCV3211566 | rs930181 | 0.51 | 0.445188644 | 0.9591 |
| hCV16182835 | rs2274736 | hCV3211568 | rs816075 | 0.51 | 0.445188644 | 1 |
| hCV16182835 | rs2274736 | hCV342703 | rs12433464 | 0.51 | 0.445188644 | 0.6601 |
| hCV16182835 | rs2274736 | hCV342704 | rs1955598 | 0.51 | 0.445188644 | 0.7953 |
| hCV16182835 | rs2274736 | hCV7583060 | rs1028455 | 0.51 | 0.445188644 | 0.8774 |
| hCV16182835 | rs2274736 | hCV7583094 | rs1048190 | 0.51 | 0.445188644 | 0.6083 |
| hCV16182835 | rs2274736 | hCV9595812 | rs845758 | 0.51 | 0.445188644 | 0.822 |
| hCV16182835 | rs2274736 | hCV9595827 | rs845757 | 0.51 | 0.445188644 | 0.9591 |
| hCV16182835 | rs2274736 | hCV9595840 | rs816072 | 0.51 | 0.445188644 | 1 |
| hCV16182835 | rs2274736 | hCV9595849 | rs1152376 | 0.51 | 0.445188644 | 0.9793 |
| hCV16182835 | rs2274736 | hCV9595856 | rs816069 | 0.51 | 0.445188644 | 0.9586 |
| hCV16182835 | rs2274736 | hCV9595863 | rs1344747 | 0.51 | 0.445188644 | 0.9596 |
| hCV16182835 | rs2274736 | hCV9595868 | rs891750 | 0.51 | 0.445188644 | 0.6812 |
| hCV16182835 | rs2274736 | hCV9595869 | rs891749 | 0.51 | 0.445188644 | 0.6812 |
| hCV16182835 | rs2274736 | hCV9595897 | rs1287825 | 0.51 | 0.445188644 | 0.4565 |
| hCV16182835 | rs2274736 | hDV70886228 | rs17124652 | 0.51 | 0.445188644 | 0.6583 |
| hCV16182835 | rs2274736 | hDV70886264 | rs17124700 | 0.51 | 0.445188644 | 0.6583 |
| hCV16182835 | rs2274736 | hDV70918505 | rs17188228 | 0.51 | 0.445188644 | 0.6141 |
| hCV16182835 | rs2274736 | hDV70929207 | rs17260380 | 0.51 | 0.445188644 | 0.6481 |

# TABLE 3, page 24 of 71

| Interrogated SNP | Interrogated rs | LD SNP | LD SNP rs | Power | Threshold r² | r² |
|---|---|---|---|---|---|---|
| hCV16182835 | rs2274736 | hDV70929214 | rs17260415 | 0.51 | 0.445188644 | 0.6571 |
| hCV16182835 | rs2274736 | hDV70991668 | rs17698817 | 0.51 | 0.445188644 | 0.6223 |
| hCV16182835 | rs2274736 | hDV70991980 | rs17700521 | 0.51 | 0.445188644 | 0.5853 |
| hCV16182835 | rs2274736 | hDV71004484 | rs17772064 | 0.51 | 0.445188644 | 0.6697 |
| hCV16182835 | rs2274736 | hDV71004511 | rs17772222 | 0.51 | 0.445188644 | 0.6697 |
| hCV16182835 | rs2274736 | hDV71004521 | rs17772288 | 0.51 | 0.445188644 | 0.65 |
| hCV16182835 | rs2274736 | hDV71008979 | rs17798341 | 0.51 | 0.445188644 | 0.6988 |
| hCV16182835 | rs2274736 | hDV71605687 | rs17188046 | 0.51 | 0.445188644 | 0.6571 |
| hCV16182835 | rs2274736 | hDV77012938 | rs4514599 | 0.51 | 0.445188644 | 0.8712 |
| hCV16182835 | rs2274736 | hDV77027209 | rs4635267 | 0.51 | 0.445188644 | 0.6646 |
| hCV16182835 | rs2274736 | hDV77248933 | rs8021690 | 0.51 | 0.445188644 | 0.6481 |
| hCV1825046 | rs2069952 | hCV1064756 | rs734111 | 0.51 | 0.131481735 | 0.5093 |
| hCV1825046 | rs2069952 | hCV11189130 | rs2065979 | 0.51 | 0.131481735 | 1 |
| hCV1825046 | rs2069952 | hCV11189159 | rs6060270 | 0.51 | 0.131481735 | 0.2033 |
| hCV1825046 | rs2069952 | hCV11189164 | rs3746427 | 0.51 | 0.131481735 | 1 |
| hCV1825046 | rs2069952 | hCV11189240 | rs2038504 | 0.51 | 0.131481735 | 0.7662 |
| hCV1825046 | rs2069952 | hCV11189318 | rs7263251 | 0.51 | 0.131481735 | 0.1718 |
| hCV1825046 | rs2069952 | hCV11189331 | rs6087649 | 0.51 | 0.131481735 | 0.5093 |
| hCV1825046 | rs2069952 | hCV11189332 | rs2273683 | 0.51 | 0.131481735 | 0.4987 |
| hCV1825046 | rs2069952 | hCV11189369 | rs6119535 | 0.51 | 0.131481735 | 0.2714 |
| hCV1825046 | rs2069952 | hCV11189450 | rs6060048 | 0.51 | 0.131481735 | 0.3343 |
| hCV1825046 | rs2069952 | hCV11656916 | rs7004 | 0.51 | 0.131481735 | 0.1338 |
| hCV1825046 | rs2069952 | hCV11656971 | rs2050652 | 0.51 | 0.131481735 | 0.3132 |
| hCV1825046 | rs2069952 | hCV11656979 | rs2065108 | 0.51 | 0.131481735 | 0.4684 |
| hCV1825046 | rs2069952 | hCV11656982 | rs1885115 | 0.51 | 0.131481735 | 0.3304 |
| hCV1825046 | rs2069952 | hCV11656983 | rs1998233 | 0.51 | 0.131481735 | 0.2712 |
| hCV1825046 | rs2069952 | hCV11656986 | rs1885119 | 0.51 | 0.131481735 | 0.4132 |
| hCV1825046 | rs2069952 | hCV1207858 | rs6141514 | 0.51 | 0.131481735 | 0.2888 |
| hCV1825046 | rs2069952 | hCV1207862 | rs6119524 | 0.51 | 0.131481735 | 0.2714 |
| hCV1825046 | rs2069952 | hCV1207879 | rs2295354 | 0.51 | 0.131481735 | 0.2537 |
| hCV1825046 | rs2069952 | hCV1207880 | rs2295353 | 0.51 | 0.131481735 | 0.2714 |
| hCV1825046 | rs2069952 | hCV1207887 | rs959829 | 0.51 | 0.131481735 | 0.5093 |
| hCV1825046 | rs2069952 | hCV1207889 | rs1998028 | 0.51 | 0.131481735 | 0.3128 |
| hCV1825046 | rs2069952 | hCV1207890 | rs6087625 | 0.51 | 0.131481735 | 0.2714 |
| hCV1825046 | rs2069952 | hCV1207891 | rs2378259 | 0.51 | 0.131481735 | 0.3343 |
| hCV1825046 | rs2069952 | hCV1207893 | rs6087624 | 0.51 | 0.131481735 | 0.3703 |
| hCV1825046 | rs2069952 | hCV1207895 | rs6120708 | 0.51 | 0.131481735 | 0.2714 |
| hCV1825046 | rs2069952 | hCV1207897 | rs3787222 | 0.51 | 0.131481735 | 0.3012 |
| hCV1825046 | rs2069952 | hCV1207898 | rs1018503 | 0.51 | 0.131481735 | 0.3343 |
| hCV1825046 | rs2069952 | hCV1207902 | rs2295352 | 0.51 | 0.131481735 | 0.5351 |
| hCV1825046 | rs2069952 | hCV1207903 | rs6087623 | 0.51 | 0.131481735 | 0.3128 |
| hCV1825046 | rs2069952 | hCV1207909 | rs6119512 | 0.51 | 0.131481735 | 0.3128 |
| hCV1825046 | rs2069952 | hCV1207914 | rs910870 | 0.51 | 0.131481735 | 0.3128 |
| hCV1825046 | rs2069952 | hCV1207915 | rs910869 | 0.51 | 0.131481735 | 0.2471 |
| hCV1825046 | rs2069952 | hCV1265078 | rs6088569 | 0.51 | 0.131481735 | 0.4047 |
| hCV1825046 | rs2069952 | hCV1265079 | rs6088570 | 0.51 | 0.131481735 | 0.4069 |
| hCV1825046 | rs2069952 | hCV1265082 | rs6088575 | 0.51 | 0.131481735 | 0.4069 |
| hCV1825046 | rs2069952 | hCV1265086 | rs2378251 | 0.51 | 0.131481735 | 0.4069 |
| hCV1825046 | rs2069952 | hCV1265087 | rs2889855 | 0.51 | 0.131481735 | 0.4069 |
| hCV1825046 | rs2069952 | hCV1265092 | rs6088578 | 0.51 | 0.131481735 | 0.4403 |
| hCV1825046 | rs2069952 | hCV1265109 | rs6058108 | 0.51 | 0.131481735 | 0.2714 |
| hCV1825046 | rs2069952 | hCV1271625 | rs6060341 | 0.51 | 0.131481735 | 0.1454 |
| hCV1825046 | rs2069952 | hCV1271649 | rs6120880 | 0.51 | 0.131481735 | 0.3283 |
| hCV1825046 | rs2069952 | hCV1271653 | rs2425019 | 0.51 | 0.131481735 | 0.1786 |
| hCV1825046 | rs2069952 | hCV1271661 | rs6088765 | 0.51 | 0.131481735 | 0.2649 |

| Interrogated SNP | Interrogated rs | LD SNP | LD SNP rs | Power | Threshold r² | r² |
|---|---|---|---|---|---|---|
| hCV1825046 | rs2069952 | hCV1271671 | rs2093058 | 0.51 | 0.131481735 | 1 |
| hCV1825046 | rs2069952 | hCV1271676 | rs1577924 | 0.51 | 0.131481735 | 1 |
| hCV1825046 | rs2069952 | hCV1271685 | rs663550 | 0.51 | 0.131481735 | 0.9658 |
| hCV1825046 | rs2069952 | hCV1271688 | rs6058202 | 0.51 | 0.131481735 | 1 |
| hCV1825046 | rs2069952 | hCV1347919 | rs1058003 | 0.51 | 0.131481735 | 0.4132 |
| hCV1825046 | rs2069952 | hCV1347925 | rs6120790 | 0.51 | 0.131481735 | 0.1339 |
| hCV1825046 | rs2069952 | hCV1347930 | rs3736802 | 0.51 | 0.131481735 | 0.4897 |
| hCV1825046 | rs2069952 | hCV1347943 | rs6060164 | 0.51 | 0.131481735 | 0.3948 |
| hCV1825046 | rs2069952 | hCV1347944 | rs6087660 | 0.51 | 0.131481735 | 0.4132 |
| hCV1825046 | rs2069952 | hCV1347963 | rs13042358 | 0.51 | 0.131481735 | 0.7561 |
| hCV1825046 | rs2069952 | hCV1348023 | rs6087677 | 0.51 | 0.131481735 | 0.4741 |
| hCV1825046 | rs2069952 | hCV1361222 | rs3818273 | 0.51 | 0.131481735 | 0.5093 |
| hCV1825046 | rs2069952 | hCV1361223 | rs3746450 | 0.51 | 0.131481735 | 0.5351 |
| hCV1825046 | rs2069952 | hCV15860322 | rs2069948 | 0.51 | 0.131481735 | 1 |
| hCV1825046 | rs2069952 | hCV15870054 | rs2224320 | 0.51 | 0.131481735 | 0.429 |
| hCV1825046 | rs2069952 | hCV15876219 | rs2281626 | 0.51 | 0.131481735 | 0.2747 |
| hCV1825046 | rs2069952 | hCV16003843 | rs2378332 | 0.51 | 0.131481735 | 0.3948 |
| hCV1825046 | rs2069952 | hCV16013546 | rs2425012 | 0.51 | 0.131481735 | 0.7218 |
| hCV1825046 | rs2069952 | hCV16013558 | rs2425009 | 0.51 | 0.131481735 | 0.2569 |
| hCV1825046 | rs2069952 | hCV16013570 | rs2077574 | 0.51 | 0.131481735 | 0.2569 |
| hCV1825046 | rs2069952 | hCV16013581 | rs2253484 | 0.51 | 0.131481735 | 0.4987 |
| hCV1825046 | rs2069952 | hCV16013593 | rs2425001 | 0.51 | 0.131481735 | 0.3727 |
| hCV1825046 | rs2069952 | hCV16013594 | rs2424999 | 0.51 | 0.131481735 | 0.5093 |
| hCV1825046 | rs2069952 | hCV16013698 | rs2425052 | 0.51 | 0.131481735 | 0.1551 |
| hCV1825046 | rs2069952 | hCV16013724 | rs2425044 | 0.51 | 0.131481735 | 0.158 |
| hCV1825046 | rs2069952 | hCV16076405 | rs2145557 | 0.51 | 0.131481735 | 0.4321 |
| hCV1825046 | rs2069952 | hCV16179579 | rs2273684 | 0.51 | 0.131481735 | 0.3893 |
| hCV1825046 | rs2069952 | hCV16179908 | rs2273805 | 0.51 | 0.131481735 | 0.4805 |
| hCV1825046 | rs2069952 | hCV16190708 | rs2295701 | 0.51 | 0.131481735 | 0.2554 |
| hCV1825046 | rs2069952 | hCV16191203 | rs2295887 | 0.51 | 0.131481735 | 0.4684 |
| hCV1825046 | rs2069952 | hCV16191204 | rs2295886 | 0.51 | 0.131481735 | 0.3062 |
| hCV1825046 | rs2069952 | hCV16191205 | rs2295885 | 0.51 | 0.131481735 | 0.3062 |
| hCV1825046 | rs2069952 | hCV1825004 | rs1415771 | 0.51 | 0.131481735 | 0.7302 |
| hCV1825046 | rs2069952 | hCV1825005 | rs945959 | 0.51 | 0.131481735 | 0.7327 |
| hCV1825046 | rs2069952 | hCV1825006 | rs1124511 | 0.51 | 0.131481735 | 0.7327 |
| hCV1825046 | rs2069952 | hCV1825018 | rs11696967 | 0.51 | 0.131481735 | 0.2033 |
| hCV1825046 | rs2069952 | hCV1825019 | rs6088732 | 0.51 | 0.131481735 | 0.2033 |
| hCV1825046 | rs2069952 | hCV1825021 | rs6088733 | 0.51 | 0.131481735 | 0.2195 |
| hCV1825046 | rs2069952 | hCV1825025 | rs6088738 | 0.51 | 0.131481735 | 0.2033 |
| hCV1825046 | rs2069952 | hCV1825040 | rs6060278 | 0.51 | 0.131481735 | 0.2033 |
| hCV1825046 | rs2069952 | hCV1825047 | rs9574 | 0.51 | 0.131481735 | 1 |
| hCV1825046 | rs2069952 | hCV1825056 | rs6060285 | 0.51 | 0.131481735 | 0.9635 |
| hCV1825046 | rs2069952 | hCV1825062 | rs6087685 | 0.51 | 0.131481735 | 0.2311 |
| hCV1825046 | rs2069952 | hCV2142560 | rs4911449 | 0.51 | 0.131481735 | 0.5093 |
| hCV1825046 | rs2069952 | hCV2142561 | rs4911450 | 0.51 | 0.131481735 | 0.5351 |
| hCV1825046 | rs2069952 | hCV2142562 | rs4911451 | 0.51 | 0.131481735 | 0.5486 |
| hCV1825046 | rs2069952 | hCV2142566 | rs6088650 | 0.51 | 0.131481735 | 0.5093 |
| hCV1825046 | rs2069952 | hCV2142567 | rs725521 | 0.51 | 0.131481735 | 0.5093 |
| hCV1825046 | rs2069952 | hCV2142575 | rs2236270 | 0.51 | 0.131481735 | 0.2714 |
| hCV1825046 | rs2069952 | hCV2142576 | rs2236271 | 0.51 | 0.131481735 | 0.5093 |
| hCV1825046 | rs2069952 | hCV2142578 | rs6088655 | 0.51 | 0.131481735 | 0.5093 |
| hCV1825046 | rs2069952 | hCV2142584 | rs3761144 | 0.51 | 0.131481735 | 0.6325 |
| hCV1825046 | rs2069952 | hCV2142586 | rs6060130 | 0.51 | 0.131481735 | 0.6325 |
| hCV1825046 | rs2069952 | hCV2142587 | rs6088664 | 0.51 | 0.131481735 | 0.6283 |
| hCV1825046 | rs2069952 | hCV2142597 | rs6120778 | 0.51 | 0.131481735 | 0.7561 |

# TABLE 3, page 26 of 71

| Interrogated SNP | Interrogated rs | LD SNP | LD SNP rs | Power | Threshold r² | r² |
|---|---|---|---|---|---|---|
| hCV1825046 | rs2069952 | hCV2142599 | rs6060140 | 0.51 | 0.131481735 | 0.7561 |
| hCV1825046 | rs2069952 | hCV2142611 | rs1885114 | 0.51 | 0.131481735 | 0.7561 |
| hCV1825046 | rs2069952 | hCV2142616 | rs3746438 | 0.51 | 0.131481735 | 0.6906 |
| hCV1825046 | rs2069952 | hCV2521759 | rs2076668 | 0.51 | 0.131481735 | 0.3128 |
| hCV1825046 | rs2069952 | hCV2521760 | rs6088624 | 0.51 | 0.131481735 | 0.3189 |
| hCV1825046 | rs2069952 | hCV2521763 | rs12625149 | 0.51 | 0.131481735 | 0.255 |
| hCV1825046 | rs2069952 | hCV2521764 | rs12626122 | 0.51 | 0.131481735 | 0.2714 |
| hCV1825046 | rs2069952 | hCV2521776 | rs6087634 | 0.51 | 0.131481735 | 0.5093 |
| hCV1825046 | rs2069952 | hCV25619953 | rs6060151 | 0.51 | 0.131481735 | 0.4132 |
| hCV1825046 | rs2069952 | hCV25619954 | rs4911462 | 0.51 | 0.131481735 | 0.4132 |
| hCV1825046 | rs2069952 | hCV25619982 | rs6120838 | 0.51 | 0.131481735 | 0.5278 |
| hCV1825046 | rs2069952 | hCV25750225 | rs4911163 | 0.51 | 0.131481735 | 0.5093 |
| hCV1825046 | rs2069952 | hCV27166951 | rs6087663 | 0.51 | 0.131481735 | 0.2569 |
| hCV1825046 | rs2069952 | hCV27166987 | rs6119542 | 0.51 | 0.131481735 | 0.5351 |
| hCV1825046 | rs2069952 | hCV27166995 | rs6088618 | 0.51 | 0.131481735 | 0.4255 |
| hCV1825046 | rs2069952 | hCV27166997 | rs6088615 | 0.51 | 0.131481735 | 0.2714 |
| hCV1825046 | rs2069952 | hCV27167007 | rs2180276 | 0.51 | 0.131481735 | 0.3128 |
| hCV1825046 | rs2069952 | hCV27167022 | rs6060013 | 0.51 | 0.131481735 | 0.3242 |
| hCV1825046 | rs2069952 | hCV27167045 | rs2378252 | 0.51 | 0.131481735 | 0.2702 |
| hCV1825046 | rs2069952 | hCV27167691 | rs2378333 | 0.51 | 0.131481735 | 0.4684 |
| hCV1825046 | rs2069952 | hCV27167696 | rs6088716 | 0.51 | 0.131481735 | 0.4698 |
| hCV1825046 | rs2069952 | hCV27472681 | rs3746430 | 0.51 | 0.131481735 | 0.2569 |
| hCV1825046 | rs2069952 | hCV27486123 | rs3803937 | 0.51 | 0.131481735 | 0.4167 |
| hCV1825046 | rs2069952 | hCV27503616 | rs3803938 | 0.51 | 0.131481735 | 0.4132 |
| hCV1825046 | rs2069952 | hCV27833500 | rs17092385 | 0.51 | 0.131481735 | 0.15 |
| hCV1825046 | rs2069952 | hCV27893015 | rs4911167 | 0.51 | 0.131481735 | 0.4132 |
| hCV1825046 | rs2069952 | hCV27893018 | rs4911441 | 0.51 | 0.131481735 | 0.3592 |
| hCV1825046 | rs2069952 | hCV27982387 | rs4911460 | 0.51 | 0.131481735 | 0.3948 |
| hCV1825046 | rs2069952 | hCV28004288 | rs4911455 | 0.51 | 0.131481735 | 0.1718 |
| hCV1825046 | rs2069952 | hCV29372788 | rs6060163 | 0.51 | 0.131481735 | 0.2554 |
| hCV1825046 | rs2069952 | hCV29372800 | rs6058149 | 0.51 | 0.131481735 | 0.1519 |
| hCV1825046 | rs2069952 | hCV29372802 | rs6579204 | 0.51 | 0.131481735 | 0.2141 |
| hCV1825046 | rs2069952 | hCV29372803 | rs6088659 | 0.51 | 0.131481735 | 0.1833 |
| hCV1825046 | rs2069952 | hCV29372811 | rs7266550 | 0.51 | 0.131481735 | 0.5093 |
| hCV1825046 | rs2069952 | hCV29372820 | rs6120739 | 0.51 | 0.131481735 | 0.5093 |
| hCV1825046 | rs2069952 | hCV29372834 | rs6060001 | 0.51 | 0.131481735 | 0.3893 |
| hCV1825046 | rs2069952 | hCV29373050 | rs6088722 | 0.51 | 0.131481735 | 0.3062 |
| hCV1825046 | rs2069952 | hCV29373051 | rs6142300 | 0.51 | 0.131481735 | 0.3062 |
| hCV1825046 | rs2069952 | hCV29373055 | rs6060196 | 0.51 | 0.131481735 | 0.2707 |
| hCV1825046 | rs2069952 | hCV29530377 | rs6088747 | 0.51 | 0.131481735 | 1 |
| hCV1825046 | rs2069952 | hCV29530378 | rs6058179 | 0.51 | 0.131481735 | 0.3062 |
| hCV1825046 | rs2069952 | hCV29566578 | rs6060172 | 0.51 | 0.131481735 | 0.3948 |
| hCV1825046 | rs2069952 | hCV29584663 | rs6060162 | 0.51 | 0.131481735 | 0.3948 |
| hCV1825046 | rs2069952 | hCV29620866 | rs6088724 | 0.51 | 0.131481735 | 0.4805 |
| hCV1825046 | rs2069952 | hCV29638959 | rs6060154 | 0.51 | 0.131481735 | 0.2554 |
| hCV1825046 | rs2069952 | hCV29674936 | rs6087618 | 0.51 | 0.131481735 | 0.307 |
| hCV1825046 | rs2069952 | hCV29674974 | rs6058224 | 0.51 | 0.131481735 | 0.158 |
| hCV1825046 | rs2069952 | hCV29674976 | rs6060266 | 0.51 | 0.131481735 | 0.1983 |
| hCV1825046 | rs2069952 | hCV2969302 | rs6120730 | 0.51 | 0.131481735 | 0.3454 |
| hCV1825046 | rs2069952 | hCV2969304 | rs2424997 | 0.51 | 0.131481735 | 0.3343 |
| hCV1825046 | rs2069952 | hCV2969305 | rs6060052 | 0.51 | 0.131481735 | 0.323 |
| hCV1825046 | rs2069952 | hCV29693115 | rs6119534 | 0.51 | 0.131481735 | 0.2714 |
| hCV1825046 | rs2069952 | hCV29693120 | rs6060003 | 0.51 | 0.131481735 | 0.3893 |
| hCV1825046 | rs2069952 | hCV29693169 | rs6058192 | 0.51 | 0.131481735 | 0.2935 |
| hCV1825046 | rs2069952 | hCV29711231 | rs6119536 | 0.51 | 0.131481735 | 0.5351 |

# TABLE 3, page 27 of 71

| Interrogated SNP | Interrogated rs | LD SNP | LD SNP rs | Power | Threshold r² | r² |
|---|---|---|---|---|---|---|
| hCV1825046 | rs2069952 | hCV29729418 | rs6142294 | 0.51 | 0.131481735 | 0.8242 |
| hCV1825046 | rs2069952 | hCV29747405 | rs6088640 | 0.51 | 0.131481735 | 0.5093 |
| hCV1825046 | rs2069952 | hCV29747445 | rs6060199 | 0.51 | 0.131481735 | 0.7956 |
| hCV1825046 | rs2069952 | hCV29783257 | rs6087619 | 0.51 | 0.131481735 | 0.2714 |
| hCV1825046 | rs2069952 | hCV29819450 | rs4142034 | 0.51 | 0.131481735 | 0.2554 |
| hCV1825046 | rs2069952 | hCV29837701 | rs6060045 | 0.51 | 0.131481735 | 0.3343 |
| hCV1825046 | rs2069952 | hCV29837747 | rs6088728 | 0.51 | 0.131481735 | 0.4684 |
| hCV1825046 | rs2069952 | hCV29837748 | rs6088721 | 0.51 | 0.131481735 | 0.4684 |
| hCV1825046 | rs2069952 | hCV29855628 | rs6058150 | 0.51 | 0.131481735 | 0.1718 |
| hCV1825046 | rs2069952 | hCV29855681 | rs6087683 | 0.51 | 0.131481735 | 1 |
| hCV1825046 | rs2069952 | hCV29855684 | rs6120816 | 0.51 | 0.131481735 | 0.4132 |
| hCV1825046 | rs2069952 | hCV2988252 | rs2425005 | 0.51 | 0.131481735 | 0.5351 |
| hCV1825046 | rs2069952 | hCV2988253 | rs6087632 | 0.51 | 0.131481735 | 0.2714 |
| hCV1825046 | rs2069952 | hCV2988254 | rs6060064 | 0.51 | 0.131481735 | 0.5093 |
| hCV1825046 | rs2069952 | hCV29909897 | rs6142324 | 0.51 | 0.131481735 | 1 |
| hCV1825046 | rs2069952 | hCV29909901 | rs6060205 | 0.51 | 0.131481735 | 0.4332 |
| hCV1825046 | rs2069952 | hCV29945782 | rs6087626 | 0.51 | 0.131481735 | 0.2714 |
| hCV1825046 | rs2069952 | hCV29963933 | rs6088590 | 0.51 | 0.131481735 | 0.2714 |
| hCV1825046 | rs2069952 | hCV29982069 | rs6060170 | 0.51 | 0.131481735 | 0.3857 |
| hCV1825046 | rs2069952 | hCV29982073 | rs6088580 | 0.51 | 0.131481735 | 0.4112 |
| hCV1825046 | rs2069952 | hCV30000150 | rs4911465 | 0.51 | 0.131481735 | 0.2707 |
| hCV1825046 | rs2069952 | hCV30035910 | rs6088692 | 0.51 | 0.131481735 | 0.4132 |
| hCV1825046 | rs2069952 | hCV30053848 | rs6088687 | 0.51 | 0.131481735 | 0.3948 |
| hCV1825046 | rs2069952 | hCV30053849 | rs6088677 | 0.51 | 0.131481735 | 0.2554 |
| hCV1825046 | rs2069952 | hCV30053852 | rs6088660 | 0.51 | 0.131481735 | 0.1575 |
| hCV1825046 | rs2069952 | hCV30072029 | rs6119516 | 0.51 | 0.131481735 | 0.2714 |
| hCV1825046 | rs2069952 | hCV30090036 | rs6120747 | 0.51 | 0.131481735 | 0.2714 |
| hCV1825046 | rs2069952 | hCV3010271 | rs2889861 | 0.51 | 0.131481735 | 0.5093 |
| hCV1825046 | rs2069952 | hCV30126097 | rs6120827 | 0.51 | 0.131481735 | 0.2707 |
| hCV1825046 | rs2069952 | hCV30144027 | rs6119559 | 0.51 | 0.131481735 | 0.2569 |
| hCV1825046 | rs2069952 | hCV30162176 | rs6060127 | 0.51 | 0.131481735 | 0.1698 |
| hCV1825046 | rs2069952 | hCV30162181 | rs6120723 | 0.51 | 0.131481735 | 0.2714 |
| hCV1825046 | rs2069952 | hCV30180146 | rs6060216 | 0.51 | 0.131481735 | 0.4122 |
| hCV1825046 | rs2069952 | hCV30198062 | rs6088764 | 0.51 | 0.131481735 | 0.2291 |
| hCV1825046 | rs2069952 | hCV30270039 | rs6060301 | 0.51 | 0.131481735 | 0.4086 |
| hCV1825046 | rs2069952 | hCV30323913 | rs6060137 | 0.51 | 0.131481735 | 0.2554 |
| hCV1825046 | rs2069952 | hCV30323914 | rs6087657 | 0.51 | 0.131481735 | 0.2569 |
| hCV1825046 | rs2069952 | hCV30342005 | rs6060133 | 0.51 | 0.131481735 | 0.198 |
| hCV1825046 | rs2069952 | hCV30342055 | rs6088727 | 0.51 | 0.131481735 | 0.4684 |
| hCV1825046 | rs2069952 | hCV30360331 | rs6088568 | 0.51 | 0.131481735 | 0.4069 |
| hCV1825046 | rs2069952 | hCV30360384 | rs6058194 | 0.51 | 0.131481735 | 0.2195 |
| hCV1825046 | rs2069952 | hCV30378399 | rs6141509 | 0.51 | 0.131481735 | 0.3343 |
| hCV1825046 | rs2069952 | hCV30378437 | rs6088713 | 0.51 | 0.131481735 | 0.3062 |
| hCV1825046 | rs2069952 | hCV30396340 | rs6120849 | 0.51 | 0.131481735 | 0.216 |
| hCV1825046 | rs2069952 | hCV30450320 | rs4911478 | 0.51 | 0.131481735 | 1 |
| hCV1825046 | rs2069952 | hCV30450323 | rs6058166 | 0.51 | 0.131481735 | 0.4122 |
| hCV1825046 | rs2069952 | hCV30468101 | rs6088735 | 0.51 | 0.131481735 | 0.2033 |
| hCV1825046 | rs2069952 | hCV30485907 | rs6087653 | 0.51 | 0.131481735 | 0.5093 |
| hCV1825046 | rs2069952 | hCV30503905 | rs4911165 | 0.51 | 0.131481735 | 0.1823 |
| hCV1825046 | rs2069952 | hCV30503911 | rs4911161 | 0.51 | 0.131481735 | 0.5093 |
| hCV1825046 | rs2069952 | hCV30503955 | rs6060194 | 0.51 | 0.131481735 | 0.2554 |
| hCV1825046 | rs2069952 | hCV30540259 | rs6087664 | 0.51 | 0.131481735 | 0.4132 |
| hCV1825046 | rs2069952 | hCV30558455 | rs6088638 | 0.51 | 0.131481735 | 0.1462 |
| hCV1825046 | rs2069952 | hCV30594383 | rs6060038 | 0.51 | 0.131481735 | 0.3343 |
| hCV1825046 | rs2069952 | hCV30612241 | rs6060168 | 0.51 | 0.131481735 | 0.7561 |

# TABLE 3, page 28 of 71

| Interrogated SNP | Interrogated rs | LD SNP | LD SNP rs | Power | Threshold r² | r² |
|---|---|---|---|---|---|---|
| hCV1825046 | rs2069952 | hCV30612290 | rs6060250 | 0.51 | 0.131481735 | 0.3355 |
| hCV1825046 | rs2069952 | hCV30630342 | rs6141526 | 0.51 | 0.131481735 | 0.7713 |
| hCV1825046 | rs2069952 | hCV30630345 | rs4911456 | 0.51 | 0.131481735 | 0.2554 |
| hCV1825046 | rs2069952 | hCV32066111 | rs10875492 | 0.51 | 0.131481735 | 0.3927 |
| hCV1825046 | rs2069952 | hCV32066659 | rs7272884 | 0.51 | 0.131481735 | 0.158 |
| hCV1825046 | rs2069952 | hCV3249260 | rs7263157 | 0.51 | 0.131481735 | 0.5093 |
| hCV1825046 | rs2069952 | hCV3249262 | rs6120750 | 0.51 | 0.131481735 | 0.2537 |
| hCV1825046 | rs2069952 | hCV3249263 | rs6088635 | 0.51 | 0.131481735 | 0.5093 |
| hCV1825046 | rs2069952 | hCV3249264 | rs6087641 | 0.51 | 0.131481735 | 0.5093 |
| hCV1825046 | rs2069952 | hCV3249268 | rs6058137 | 0.51 | 0.131481735 | 0.5093 |
| hCV1825046 | rs2069952 | hCV3249269 | rs8116657 | 0.51 | 0.131481735 | 0.5351 |
| hCV1825046 | rs2069952 | hCV3249271 | rs4911164 | 0.51 | 0.131481735 | 0.5351 |
| hCV1825046 | rs2069952 | hCV3249272 | rs6087644 | 0.51 | 0.131481735 | 0.5123 |
| hCV1825046 | rs2069952 | hCV3249275 | rs6088642 | 0.51 | 0.131481735 | 0.5093 |
| hCV1825046 | rs2069952 | hCV3249278 | rs6120757 | 0.51 | 0.131481735 | 0.5093 |
| hCV1825046 | rs2069952 | hCV3249279 | rs926734 | 0.51 | 0.131481735 | 0.5093 |
| hCV1825046 | rs2069952 | hCV3249280 | rs6120758 | 0.51 | 0.131481735 | 0.5093 |
| hCV1825046 | rs2069952 | hCV3249282 | rs2064454 | 0.51 | 0.131481735 | 0.5093 |
| hCV1825046 | rs2069952 | hCV3249286 | rs6088646 | 0.51 | 0.131481735 | 0.5093 |
| hCV1825046 | rs2069952 | hCV3249289 | rs2223881 | 0.51 | 0.131481735 | 0.5351 |
| hCV1825046 | rs2069952 | hCV3249290 | rs2076667 | 0.51 | 0.131481735 | 0.5093 |
| hCV1825046 | rs2069952 | hCV3249293 | rs6058154 | 0.51 | 0.131481735 | 0.7561 |
| hCV1825046 | rs2069952 | hCV599565 | rs633198 | 0.51 | 0.131481735 | 1 |
| hCV1825046 | rs2069952 | hCV624502 | rs666210 | 0.51 | 0.131481735 | 0.2804 |
| hCV1825046 | rs2069952 | hCV624503 | rs633784 | 0.51 | 0.131481735 | 0.2804 |
| hCV1825046 | rs2069952 | hCV7499886 | rs1415774 | 0.51 | 0.131481735 | 1 |
| hCV1825046 | rs2069952 | hCV7593276 | rs1535466 | 0.51 | 0.131481735 | 0.3224 |
| hCV1825046 | rs2069952 | hCV7593320 | rs1060615 | 0.51 | 0.131481735 | 0.4769 |
| hCV1825046 | rs2069952 | hCV7593321 | rs1013677 | 0.51 | 0.131481735 | 0.5093 |
| hCV1825046 | rs2069952 | hCV7593328 | rs1018447 | 0.51 | 0.131481735 | 0.5093 |
| hCV1825046 | rs2069952 | hDV70862585 | rs17092209 | 0.51 | 0.131481735 | 0.1405 |
| hCV1825046 | rs2069952 | hDV70862720 | rs17092378 | 0.51 | 0.131481735 | 0.3132 |
| hCV1825046 | rs2069952 | hDV70936356 | rs17310782 | 0.51 | 0.131481735 | 0.2554 |
| hCV1825046 | rs2069952 | hDV71833331 | rs6142280 | 0.51 | 0.131481735 | 0.7559 |
| hCV1825046 | rs2069952 | hDV71898298 | rs7361656 | 0.51 | 0.131481735 | 0.2982 |
| hCV1825046 | rs2069952 | hDV72053898 | rs8114671 | 0.51 | 0.131481735 | 1 |
| hCV1841975 | rs1799810 | hCV11263777 | rs11683986 | 0.51 | 0.254914511 | 0.4647 |
| hCV1841975 | rs1799810 | hCV11263786 | rs13408910 | 0.51 | 0.254914511 | 0.429 |
| hCV1841975 | rs1799810 | hCV11266746 | rs6753288 | 0.51 | 0.254914511 | 0.834 |
| hCV1841975 | rs1799810 | hCV11266765 | rs11679414 | 0.51 | 0.254914511 | 0.5751 |
| hCV1841975 | rs1799810 | hCV11268771 | rs4662718 | 0.51 | 0.254914511 | 0.5598 |
| hCV1841975 | rs1799810 | hCV12046224 | rs10850 | 0.51 | 0.254914511 | 0.5598 |
| hCV1841975 | rs1799810 | hCV1441133 | rs7568070 | 0.51 | 0.254914511 | 0.356 |
| hCV1841975 | rs1799810 | hCV1441173 | rs4536600 | 0.51 | 0.254914511 | 0.641 |
| hCV1841975 | rs1799810 | hCV1441189 | rs4150499 | 0.51 | 0.254914511 | 0.5379 |
| hCV1841975 | rs1799810 | hCV1441196 | rs4150474 | 0.51 | 0.254914511 | 0.5354 |
| hCV1841975 | rs1799810 | hCV15860236 | rs2069904 | 0.51 | 0.254914511 | 0.7263 |
| hCV1841975 | rs1799810 | hCV169044 | rs11691088 | 0.51 | 0.254914511 | 0.5832 |
| hCV1841975 | rs1799810 | hCV1841983 | rs5937 | 0.51 | 0.254914511 | 0.6119 |
| hCV1841975 | rs1799810 | hCV25630050 | rs3732209 | 0.51 | 0.254914511 | 0.5787 |
| hCV1841975 | rs1799810 | hCV25960135 | rs4150402 | 0.51 | 0.254914511 | 0.5598 |
| hCV1841975 | rs1799810 | hCV26980926 | rs4150471 | 0.51 | 0.254914511 | 0.5379 |
| hCV1841975 | rs1799810 | hCV273435 | rs7607907 | 0.51 | 0.254914511 | 0.5598 |
| hCV1841975 | rs1799810 | hCV27907596 | rs4321325 | 0.51 | 0.254914511 | 0.335 |
| hCV1841975 | rs1799810 | hCV27964958 | rs4662713 | 0.51 | 0.254914511 | 0.5598 |

# TABLE 3, page 29 of 71

| Interrogated SNP | Interrogated rs | LD SNP | LD SNP rs | Power | Threshold r² | r² |
|---|---|---|---|---|---|---|
| hCV1841975 | rs1799810 | hCV28026949 | rs4662720 | 0.51 | 0.254914511 | 0.5598 |
| hCV1841975 | rs1799810 | hCV28952331 | rs6755028 | 0.51 | 0.254914511 | 0.5319 |
| hCV1841975 | rs1799810 | hCV28955091 | rs7567389 | 0.51 | 0.254914511 | 0.3991 |
| hCV1841975 | rs1799810 | hCV28955092 | rs6430936 | 0.51 | 0.254914511 | 0.5598 |
| hCV1841975 | rs1799810 | hCV28966787 | rs7585314 | 0.51 | 0.254914511 | 0.5343 |
| hCV1841975 | rs1799810 | hCV29570359 | rs6738690 | 0.51 | 0.254914511 | 0.5288 |
| hCV1841975 | rs1799810 | hCV29636350 | rs10496661 | 0.51 | 0.254914511 | 0.5598 |
| hCV1841975 | rs1799810 | hCV30166207 | rs7590030 | 0.51 | 0.254914511 | 0.5787 |
| hCV1841975 | rs1799810 | hCV30418053 | rs6757492 | 0.51 | 0.254914511 | 0.545 |
| hCV1841975 | rs1799810 | hCV30598525 | rs7556675 | 0.51 | 0.254914511 | 0.5598 |
| hCV1841975 | rs1799810 | hCV30711743 | rs11890243 | 0.51 | 0.254914511 | 0.4437 |
| hCV1841975 | rs1799810 | hCV31814195 | rs11680949 | 0.51 | 0.254914511 | 0.5659 |
| hCV1841975 | rs1799810 | hCV31814218 | rs6430938 | 0.51 | 0.254914511 | 0.6123 |
| hCV1841975 | rs1799810 | hCV8806682 | rs1011019 | 0.51 | 0.254914511 | 0.5598 |
| hCV1841975 | rs1799810 | hCV8807983 | rs1504136 | 0.51 | 0.254914511 | 0.5343 |
| hCV1841975 | rs1799810 | hCV8807988 | rs3893313 | 0.51 | 0.254914511 | 0.2563 |
| hCV1841975 | rs1799810 | hCV8807993 | rs1473623 | 0.51 | 0.254914511 | 0.5478 |
| hCV1841975 | rs1799810 | hCV8810479 | rs1604817 | 0.51 | 0.254914511 | 0.6417 |
| hCV1841975 | rs1799810 | hCV8810750 | rs1158867 | 0.51 | 0.254914511 | 1 |
| hCV1841975 | rs1799810 | hCV9465822 | rs11683427 | 0.51 | 0.254914511 | 0.3486 |
| hCV1841975 | rs1799810 | hCV9468542 | rs7599210 | 0.51 | 0.254914511 | 0.8322 |
| hCV1841975 | rs1799810 | hDV75209985 | rs2069898 | 0.51 | 0.254914511 | 0.7164 |
| hCV1841983 | rs5937 | hCV1023645 | rs334160 | 0.51 | 0.289879478 | 0.3788 |
| hCV1841983 | rs5937 | hCV1023646 | rs334159 | 0.51 | 0.289879478 | 0.3408 |
| hCV1841983 | rs5937 | hCV1023653 | rs334151 | 0.51 | 0.289879478 | 0.3788 |
| hCV1841983 | rs5937 | hCV1023659 | rs334146 | 0.51 | 0.289879478 | 0.3564 |
| hCV1841983 | rs5937 | hCV1023661 | rs334143 | 0.51 | 0.289879478 | 0.3543 |
| hCV1841983 | rs5937 | hCV1023665 | rs334138 | 0.51 | 0.289879478 | 0.3395 |
| hCV1841983 | rs5937 | hCV1023666 | rs334137 | 0.51 | 0.289879478 | 0.4012 |
| hCV1841983 | rs5937 | hCV1023669 | rs1075774 | 0.51 | 0.289879478 | 0.3374 |
| hCV1841983 | rs5937 | hCV11263777 | rs11683986 | 0.51 | 0.289879478 | 0.8536 |
| hCV1841983 | rs5937 | hCV11263778 | rs6749002 | 0.51 | 0.289879478 | 0.3378 |
| hCV1841983 | rs5937 | hCV11263786 | rs13408910 | 0.51 | 0.289879478 | 0.4521 |
| hCV1841983 | rs5937 | hCV11266746 | rs6753288 | 0.51 | 0.289879478 | 0.5815 |
| hCV1841983 | rs5937 | hCV11266765 | rs11679414 | 0.51 | 0.289879478 | 0.6966 |
| hCV1841983 | rs5937 | hCV11268771 | rs4662718 | 0.51 | 0.289879478 | 0.7374 |
| hCV1841983 | rs5937 | hCV12046142 | rs334152 | 0.51 | 0.289879478 | 0.3564 |
| hCV1841983 | rs5937 | hCV12046143 | rs334156 | 0.51 | 0.289879478 | 0.3564 |
| hCV1841983 | rs5937 | hCV12046144 | rs334158 | 0.51 | 0.289879478 | 0.3788 |
| hCV1841983 | rs5937 | hCV12046224 | rs10850 | 0.51 | 0.289879478 | 0.7374 |
| hCV1841983 | rs5937 | hCV12047693 | rs1864552 | 0.51 | 0.289879478 | 0.3779 |
| hCV1841983 | rs5937 | hCV1441133 | rs7568070 | 0.51 | 0.289879478 | 0.3758 |
| hCV1841983 | rs5937 | hCV1441173 | rs4536600 | 0.51 | 0.289879478 | 0.7198 |
| hCV1841983 | rs5937 | hCV1441189 | rs4150499 | 0.51 | 0.289879478 | 0.605 |
| hCV1841983 | rs5937 | hCV1441196 | rs4150474 | 0.51 | 0.289879478 | 0.6229 |
| hCV1841983 | rs5937 | hCV15860236 | rs2069904 | 0.51 | 0.289879478 | 0.9367 |
| hCV1841983 | rs5937 | hCV15917574 | rs2679409 | 0.51 | 0.289879478 | 0.3564 |
| hCV1841983 | rs5937 | hCV16241157 | rs2460106 | 0.51 | 0.289879478 | 0.3564 |
| hCV1841983 | rs5937 | hCV169044 | rs11691088 | 0.51 | 0.289879478 | 0.704 |
| hCV1841983 | rs5937 | hCV1721256 | rs2069910 | 0.51 | 0.289879478 | 0.3227 |
| hCV1841983 | rs5937 | hCV1841975 | rs1799810 | 0.51 | 0.289879478 | 0.6119 |
| hCV1841983 | rs5937 | hCV25630050 | rs3732209 | 0.51 | 0.289879478 | 0.7933 |
| hCV1841983 | rs5937 | hCV25960135 | rs4150402 | 0.51 | 0.289879478 | 0.7374 |
| hCV1841983 | rs5937 | hCV25971425 | rs4662741 | 0.51 | 0.289879478 | 0.3564 |
| hCV1841983 | rs5937 | hCV25993019 | rs11673952 | 0.51 | 0.289879478 | 0.3564 |

# TABLE 3, page 30 of 71

| Interrogated SNP | Interrogated rs | LD SNP | LD SNP rs | Power | Threshold r² | r² |
|---|---|---|---|---|---|---|
| hCV1841983 | rs5937 | hCV26980926 | rs4150471 | 0.51 | 0.289879478 | 0.6251 |
| hCV1841983 | rs5937 | hCV27271075 | rs2163348 | 0.51 | 0.289879478 | 0.3793 |
| hCV1841983 | rs5937 | hCV273435 | rs7607907 | 0.51 | 0.289879478 | 0.7374 |
| hCV1841983 | rs5937 | hCV27964958 | rs4662713 | 0.51 | 0.289879478 | 0.7178 |
| hCV1841983 | rs5937 | hCV28026949 | rs4662720 | 0.51 | 0.289879478 | 0.7374 |
| hCV1841983 | rs5937 | hCV28952331 | rs6755028 | 0.51 | 0.289879478 | 0.7962 |
| hCV1841983 | rs5937 | hCV28952333 | rs6754772 | 0.51 | 0.289879478 | 0.3767 |
| hCV1841983 | rs5937 | hCV28955091 | rs7567389 | 0.51 | 0.289879478 | 0.4991 |
| hCV1841983 | rs5937 | hCV28955092 | rs6430936 | 0.51 | 0.289879478 | 0.7189 |
| hCV1841983 | rs5937 | hCV28966787 | rs7585314 | 0.51 | 0.289879478 | 0.5885 |
| hCV1841983 | rs5937 | hCV29404615 | rs6709113 | 0.51 | 0.289879478 | 0.3311 |
| hCV1841983 | rs5937 | hCV29404616 | rs6706077 | 0.51 | 0.289879478 | 0.3564 |
| hCV1841983 | rs5937 | hCV29404621 | rs7600934 | 0.51 | 0.289879478 | 0.3564 |
| hCV1841983 | rs5937 | hCV29404688 | rs7582598 | 0.51 | 0.289879478 | 0.365 |
| hCV1841983 | rs5937 | hCV29570359 | rs6738690 | 0.51 | 0.289879478 | 0.6251 |
| hCV1841983 | rs5937 | hCV29636350 | rs10496661 | 0.51 | 0.289879478 | 0.7374 |
| hCV1841983 | rs5937 | hCV30166207 | rs7590030 | 0.51 | 0.289879478 | 0.7933 |
| hCV1841983 | rs5937 | hCV30418053 | rs6757492 | 0.51 | 0.289879478 | 0.6961 |
| hCV1841983 | rs5937 | hCV30598525 | rs7556675 | 0.51 | 0.289879478 | 0.7374 |
| hCV1841983 | rs5937 | hCV30711743 | rs11890243 | 0.51 | 0.289879478 | 0.5169 |
| hCV1841983 | rs5937 | hCV31814195 | rs11680949 | 0.51 | 0.289879478 | 0.6251 |
| hCV1841983 | rs5937 | hCV31814218 | rs6430938 | 0.51 | 0.289879478 | 0.8723 |
| hCV1841983 | rs5937 | hCV3212726 | rs12621149 | 0.51 | 0.289879478 | 0.292 |
| hCV1841983 | rs5937 | hCV822512 | rs334144 | 0.51 | 0.289879478 | 0.3564 |
| hCV1841983 | rs5937 | hCV8806682 | rs1011019 | 0.51 | 0.289879478 | 0.7374 |
| hCV1841983 | rs5937 | hCV8807983 | rs1504136 | 0.51 | 0.289879478 | 0.6258 |
| hCV1841983 | rs5937 | hCV8807993 | rs1473623 | 0.51 | 0.289879478 | 0.7761 |
| hCV1841983 | rs5937 | hCV8808000 | rs891514 | 0.51 | 0.289879478 | 0.3564 |
| hCV1841983 | rs5937 | hCV8810479 | rs1604817 | 0.51 | 0.289879478 | 0.6728 |
| hCV1841983 | rs5937 | hCV8810750 | rs1158867 | 0.51 | 0.289879478 | 0.6851 |
| hCV1841983 | rs5937 | hCV8836422 | rs777554 | 0.51 | 0.289879478 | 0.3932 |
| hCV1841983 | rs5937 | hCV8837013 | rs1019842 | 0.51 | 0.289879478 | 0.3723 |
| hCV1841983 | rs5937 | hCV8837014 | rs777569 | 0.51 | 0.289879478 | 0.3564 |
| hCV1841983 | rs5937 | hCV9465822 | rs11683427 | 0.51 | 0.289879478 | 0.4044 |
| hCV1841983 | rs5937 | hCV9468542 | rs7599210 | 0.51 | 0.289879478 | 0.5771 |
| hCV1841983 | rs5937 | hDV70929450 | rs17261845 | 0.51 | 0.289879478 | 0.2956 |
| hCV1841983 | rs5937 | hDV70929452 | rs17261859 | 0.51 | 0.289879478 | 0.2956 |
| hCV1841983 | rs5937 | hDV75209985 | rs2069898 | 0.51 | 0.289879478 | 0.8713 |
| hCV1859855 | rs2291260 | hCV12052839 | rs8021 | 0.51 | 0.469041298 | 0.5002 |
| hCV1859855 | rs2291260 | hCV16089184 | rs2873193 | 0.51 | 0.469041298 | 0.6229 |
| hCV1859855 | rs2291260 | hCV16174004 | rs2242291 | 0.51 | 0.469041298 | 0.6816 |
| hCV1859855 | rs2291260 | hCV1859872 | rs4758905 | 0.51 | 0.469041298 | 0.6816 |
| hCV1859855 | rs2291260 | hCV1859912 | rs12303977 | 0.51 | 0.469041298 | 0.6816 |
| hCV1859855 | rs2291260 | hCV1859941 | rs2306541 | 0.51 | 0.469041298 | 0.637 |
| hCV1859855 | rs2291260 | hCV1859948 | rs7297261 | 0.51 | 0.469041298 | 0.5354 |
| hCV1859855 | rs2291260 | hCV1859956 | rs7969859 | 0.51 | 0.469041298 | 0.6554 |
| hCV1859855 | rs2291260 | hCV1859996 | rs12815354 | 0.51 | 0.469041298 | 0.5246 |
| hCV1859855 | rs2291260 | hCV1859997 | rs12815207 | 0.51 | 0.469041298 | 0.5308 |
| hCV1859855 | rs2291260 | hCV25761477 | rs3741490 | 0.51 | 0.469041298 | 0.637 |
| hCV1859855 | rs2291260 | hCV27522090 | rs3825109 | 0.51 | 0.469041298 | 0.6918 |
| hCV1859855 | rs2291260 | hCV27964264 | rs4758939 | 0.51 | 0.469041298 | 0.6816 |
| hCV1859855 | rs2291260 | hCV30960162 | rs11147095 | 0.51 | 0.469041298 | 0.6816 |
| hCV1859855 | rs2291260 | hCV31631014 | rs12811327 | 0.51 | 0.469041298 | 0.5728 |
| hCV1952126 | rs7223784 | hCV11626701 | rs1032070 | 0.51 | 0.792412162 | 0.9128 |
| hCV1952126 | rs7223784 | hCV27485323 | rs3785897 | 0.51 | 0.792412162 | 0.8875 |

# TABLE 3, page 31 of 71

| Interrogated SNP | Interrogated rs | LD SNP | LD SNP rs | Power | Threshold $r^2$ | $r^2$ |
|---|---|---|---|---|---|---|
| hCV1952126 | rs7223784 | hCV2769165 | rs2071046 | 0.51 | 0.792412162 | 0.9129 |
| hCV1952126 | rs7223784 | hCV2977462 | rs4793099 | 0.51 | 0.792412162 | 0.9128 |
| hCV1952126 | rs7223784 | hCV3140239 | rs36023314 | 0.51 | 0.792412162 | 0.8867 |
| hCV1952126 | rs7223784 | hCV3140264 | rs6503704 | 0.51 | 0.792412162 | 1 |
| hCV1952126 | rs7223784 | hCV31652022 | rs12948909 | 0.51 | 0.792412162 | 1 |
| hCV1952126 | rs7223784 | hCV31652026 | rs11871801 | 0.51 | 0.792412162 | 1 |
| hCV1952126 | rs7223784 | hCVS87962 | rs647397 | 0.51 | 0.792412162 | 0.8421 |
| hCV22272267 | rs3733402 | hCV11786147 | rs4862662 | 0.51 | 0.093086244 | 0.352 |
| hCV22272267 | rs3733402 | hCV11786203 | rs4253251 | 0.51 | 0.093086244 | 0.2672 |
| hCV22272267 | rs3733402 | hCV11786258 | rs4253303 | 0.51 | 0.093086244 | 0.5632 |
| hCV22272267 | rs3733402 | hCV11786259 | rs4253304 | 0.51 | 0.093086244 | 0.6456 |
| hCV22272267 | rs3733402 | hCV11786295 | rs4253421 | 0.51 | 0.093086244 | 0.1162 |
| hCV22272267 | rs3733402 | hCV11786327 | rs13133050 | 0.51 | 0.093086244 | 0.2482 |
| hCV22272267 | rs3733402 | hCV12066118 | rs2048 | 0.51 | 0.093086244 | 1 |
| hCV22272267 | rs3733402 | hCV12066119 | rs1912826 | 0.51 | 0.093086244 | 0.9284 |
| hCV22272267 | rs3733402 | hCV12066124 | rs2036914 | 0.51 | 0.093086244 | 0.3605 |
| hCV22272267 | rs3733402 | hCV12066129 | rs1593 | 0.51 | 0.093086244 | 0.1325 |
| hCV22272267 | rs3733402 | hCV1332991 | rs11723770 | 0.51 | 0.093086244 | 0.1232 |
| hCV22272267 | rs3733402 | hCV1332992 | rs12506228 | 0.51 | 0.093086244 | 0.1088 |
| hCV22272267 | rs3733402 | hCV15793897 | rs3087505 | 0.51 | 0.093086244 | 0.12 |
| hCV22272267 | rs3733402 | hCV15811716 | rs2102575 | 0.51 | 0.093086244 | 0.1089 |
| hCV22272267 | rs3733402 | hCV15968025 | rs2292425 | 0.51 | 0.093086244 | 0.18 |
| hCV22272267 | rs3733402 | hCV15968026 | rs2292426 | 0.51 | 0.093086244 | 0.2195 |
| hCV22272267 | rs3733402 | hCV15968034 | rs2292428 | 0.51 | 0.093086244 | 0.3058 |
| hCV22272267 | rs3733402 | hCV15968043 | rs2292423 | 0.51 | 0.093086244 | 0.6588 |
| hCV22272267 | rs3733402 | hCV15975109 | rs2304596 | 0.51 | 0.093086244 | 0.24 |
| hCV22272267 | rs3733402 | hCV2103337 | rs13102931 | 0.51 | 0.093086244 | 0.1025 |
| hCV22272267 | rs3733402 | hCV2103343 | rs4241824 | 0.51 | 0.093086244 | 0.3019 |
| hCV22272267 | rs3733402 | hCV2103391 | rs1008728 | 0.51 | 0.093086244 | 0.2065 |
| hCV22272267 | rs3733402 | hCV2103392 | rs12500826 | 0.51 | 0.093086244 | 0.1775 |
| hCV22272267 | rs3733402 | hCV22271609 | rs4253326 | 0.51 | 0.093086244 | 0.2299 |
| hCV22272267 | rs3733402 | hCV25474413 | rs3822057 | 0.51 | 0.093086244 | 0.312 |
| hCV22272267 | rs3733402 | hCV25474414 | rs4253399 | 0.51 | 0.093086244 | 0.1628 |
| hCV22272267 | rs3733402 | hCV25634763 | rs4253241 | 0.51 | 0.093086244 | 0.1188 |
| hCV22272267 | rs3733402 | hCV25634781 | rs4253299 | 0.51 | 0.093086244 | 0.2584 |
| hCV22272267 | rs3733402 | hCV25989001 | hCV25989001 | 0.51 | 0.093086244 | 0.2535 |
| hCV22272267 | rs3733402 | hCV25990131 | rs13146272 | 0.51 | 0.093086244 | 0.1915 |
| hCV22272267 | rs3733402 | hCV26265197 | rs10014399 | 0.51 | 0.093086244 | 0.2641 |
| hCV22272267 | rs3733402 | hCV26265199 | rs2221843 | 0.51 | 0.093086244 | 0.2584 |
| hCV22272267 | rs3733402 | hCV26265231 | rs7684025 | 0.51 | 0.093086244 | 0.3837 |
| hCV22272267 | rs3733402 | hCV27473099 | rs3733403 | 0.51 | 0.093086244 | 0.1386 |
| hCV22272267 | rs3733402 | hCV27477533 | rs3756008 | 0.51 | 0.093086244 | 0.1577 |
| hCV22272267 | rs3733402 | hCV27482765 | rs3775301 | 0.51 | 0.093086244 | 0.24 |
| hCV22272267 | rs3733402 | hCV27482766 | rs3775302 | 0.51 | 0.093086244 | 0.1546 |
| hCV22272267 | rs3733402 | hCV27506149 | rs3822055 | 0.51 | 0.093086244 | 0.2584 |
| hCV22272267 | rs3733402 | hCV27521729 | rs3822056 | 0.51 | 0.093086244 | 0.1313 |
| hCV22272267 | rs3733402 | hCV27902808 | rs4253236 | 0.51 | 0.093086244 | 0.6716 |
| hCV22272267 | rs3733402 | hCV29053264 | rs7667777 | 0.51 | 0.093086244 | 0.3951 |
| hCV22272267 | rs3733402 | hCV29053265 | rs4253244 | 0.51 | 0.093086244 | 0.6414 |
| hCV22272267 | rs3733402 | hCV29718000 | rs4253238 | 0.51 | 0.093086244 | 1 |
| hCV22272267 | rs3733402 | hCV29826351 | rs10025990 | 0.51 | 0.093086244 | 0.1333 |
| hCV22272267 | rs3733402 | hCV29877725 | rs4253295 | 0.51 | 0.093086244 | 0.5775 |
| hCV22272267 | rs3733402 | hCV30983907 | rs4253246 | 0.51 | 0.093086244 | 0.1188 |
| hCV22272267 | rs3733402 | hCV32209636 | rs11132387 | 0.51 | 0.093086244 | 0.1446 |
| hCV22272267 | rs3733402 | hCV32291217 | rs4253323 | 0.51 | 0.093086244 | 0.24 |

# TABLE 3, page 32 of 71

| Interrogated SNP | Interrogated rs | LD SNP | LD SNP rs | Power | Threshold $r^2$ | $r^2$ |
|---|---|---|---|---|---|---|
| hCV22272267 | rs3733402 | hCV32291269 | rs4253417 | 0.51 | 0.093086244 | 0.1165 |
| hCV22272267 | rs3733402 | hCV32291286 | rs4253422 | 0.51 | 0.093086244 | 0.168 |
| hCV22272267 | rs3733402 | hCV32291287 | rs4253423 | 0.51 | 0.093086244 | 0.168 |
| hCV22272267 | rs3733402 | hCV32291295 | rs4253292 | 0.51 | 0.093086244 | 0.2444 |
| hCV22272267 | rs3733402 | hCV32291301 | rs4253302 | 0.51 | 0.093086244 | 0.2397 |
| hCV22272267 | rs3733402 | hCV32295028 | rs4253260 | 0.51 | 0.093086244 | 0.24 |
| hCV22272267 | rs3733402 | hCV3229991 | rs4241815 | 0.51 | 0.093086244 | 1 |
| hCV22272267 | rs3733402 | hCV3229992 | rs3775298 | 0.51 | 0.093086244 | 1 |
| hCV22272267 | rs3733402 | hCV3229995 | rs11132382 | 0.51 | 0.093086244 | 1 |
| hCV22272267 | rs3733402 | hCV3230000 | rs4253294 | 0.51 | 0.093086244 | 0.4581 |
| hCV22272267 | rs3733402 | hCV3230001 | rs4253296 | 0.51 | 0.093086244 | 0.1188 |
| hCV22272267 | rs3733402 | hCV3230002 | rs4253297 | 0.51 | 0.093086244 | 0.5815 |
| hCV22272267 | rs3733402 | hCV3230003 | rs2304595 | 0.51 | 0.093086244 | 0.6471 |
| hCV22272267 | rs3733402 | hCV3230004 | rs4253301 | 0.51 | 0.093086244 | 0.1089 |
| hCV22272267 | rs3733402 | hCV3230006 | rs4253308 | 0.51 | 0.093086244 | 0.5775 |
| hCV22272267 | rs3733402 | hCV3230007 | rs4253311 | 0.51 | 0.093086244 | 1 |
| hCV22272267 | rs3733402 | hCV3230010 | rs4253315 | 0.51 | 0.093086244 | 0.1137 |
| hCV22272267 | rs3733402 | hCV3230011 | rs4253320 | 0.51 | 0.093086244 | 0.5815 |
| hCV22272267 | rs3733402 | hCV3230012 | rs4241821 | 0.51 | 0.093086244 | 0.2584 |
| hCV22272267 | rs3733402 | hCV3230013 | rs3775303 | 0.51 | 0.093086244 | 0.6456 |
| hCV22272267 | rs3733402 | hCV3230014 | rs4861709 | 0.51 | 0.093086244 | 0.4581 |
| hCV22272267 | rs3733402 | hCV3230016 | rs4253325 | 0.51 | 0.093086244 | 0.112 |
| hCV22272267 | rs3733402 | hCV3230017 | rs4253327 | 0.51 | 0.093086244 | 0.1008 |
| hCV22272267 | rs3733402 | hCV3230018 | rs925453 | 0.51 | 0.093086244 | 0.4359 |
| hCV22272267 | rs3733402 | hCV3230019 | rs4253332 | 0.51 | 0.093086244 | 0.4359 |
| hCV22272267 | rs3733402 | hCV3230025 | rs3756009 | 0.51 | 0.093086244 | 0.149 |
| hCV22272267 | rs3733402 | hCV3230031 | rs4253419 | 0.51 | 0.093086244 | 0.168 |
| hCV22272267 | rs3733402 | hCV3230038 | rs2289252 | 0.51 | 0.093086244 | 0.1192 |
| hCV22272267 | rs3733402 | hCV3230083 | rs10013653 | 0.51 | 0.093086244 | 0.3018 |
| hCV22272267 | rs3733402 | hCV3230084 | rs7682918 | 0.51 | 0.093086244 | 0.2829 |
| hCV22272267 | rs3733402 | hCV3230094 | rs7687818 | 0.51 | 0.093086244 | 0.4367 |
| hCV22272267 | rs3733402 | hCV3230096 | rs3817184 | 0.51 | 0.093086244 | 0.3722 |
| hCV22272267 | rs3733402 | hCV3230097 | rs3736455 | 0.51 | 0.093086244 | 0.2406 |
| hCV22272267 | rs3733402 | hCV3230101 | rs6835839 | 0.51 | 0.093086244 | 0.3515 |
| hCV22272267 | rs3733402 | hCV3230106 | rs1473597 | 0.51 | 0.093086244 | 0.318 |
| hCV22272267 | rs3733402 | hCV3230110 | rs2276917 | 0.51 | 0.093086244 | 0.3058 |
| hCV22272267 | rs3733402 | hCV3230113 | rs1053094 | 0.51 | 0.093086244 | 0.5831 |
| hCV22272267 | rs3733402 | hCV3230118 | rs4253429 | 0.51 | 0.093086244 | 0.168 |
| hCV22272267 | rs3733402 | hCV3230125 | rs11938564 | 0.51 | 0.093086244 | 0.1034 |
| hCV22272267 | rs3733402 | hCV3230136 | rs13116273 | 0.51 | 0.093086244 | 0.0947 |
| hCV22272267 | rs3733402 | hCV32313006 | rs4253248 | 0.51 | 0.093086244 | 1 |
| hCV22272267 | rs3733402 | hCV32313024 | rs4253239 | 0.51 | 0.093086244 | 0.2444 |
| hCV22272267 | rs3733402 | hCV32358975 | rs4253255 | 0.51 | 0.093086244 | 1 |
| hCV22272267 | rs3733402 | hCV32358984 | rs4253256 | 0.51 | 0.093086244 | 0.6645 |
| hCV22272267 | rs3733402 | hCV7750713 | rs4862596 | 0.51 | 0.093086244 | 0.1067 |
| hCV22272267 | rs3733402 | hCV7750737 | rs13140248 | 0.51 | 0.093086244 | 0.1031 |
| hCV22272267 | rs3733402 | hCV8241630 | rs925451 | 0.51 | 0.093086244 | 0.1476 |
| hCV22272267 | rs3733402 | hCV8241631 | rs1511802 | 0.51 | 0.093086244 | 0.5775 |
| hCV22272267 | rs3733402 | hCV8241632 | rs1511801 | 0.51 | 0.093086244 | 1 |
| hCV22272267 | rs3733402 | hDV68550952 | rs4253289 | 0.51 | 0.093086244 | 0.102 |
| hCV22272267 | rs3733402 | hDV71222711 | rs4253252 | 0.51 | 0.093086244 | 1 |
| hCV22273419 | rs2304167 | hCV11977629 | rs1654459 | 0.51 | 0.488273752 | 0.5665 |
| hCV22273419 | rs2304167 | hCV1376257 | rs10416380 | 0.51 | 0.488273752 | 0.9727 |
| hCV22273419 | rs2304167 | hCV1376262 | rs1671150 | 0.51 | 0.488273752 | 1 |
| hCV22273419 | rs2304167 | hCV1376264 | rs1671151 | 0.51 | 0.488273752 | 1 |

# TABLE 3, page 33 of 71

| Interrogated SNP | Interrogated rs | LD SNP | LD SNP rs | Power | Threshold r² | r² |
|---|---|---|---|---|---|---|
| hCV22273419 | rs2304167 | hCV1376265 | rs1671152 | 0.51 | 0.488273752 | 0.8131 |
| hCV22273419 | rs2304167 | hCV1376266 | rs1654413 | 0.51 | 0.488273752 | 1 |
| hCV22273419 | rs2304167 | hCV1376342 | rs1654416 | 0.51 | 0.488273752 | 0.9724 |
| hCV22273419 | rs2304167 | hCV1376359 | rs2886412 | 0.51 | 0.488273752 | 1 |
| hCV22273419 | rs2304167 | hCV16044361 | rs2569513 | 0.51 | 0.488273752 | 0.639 |
| hCV22273419 | rs2304167 | hCV26895244 | rs1671153 | 0.51 | 0.488273752 | 1 |
| hCV22273419 | rs2304167 | hCV26895257 | rs2886415 | 0.51 | 0.488273752 | 1 |
| hCV22273419 | rs2304167 | hCV29271569 | rs1626971 | 0.51 | 0.488273752 | 0.7325 |
| hCV22273419 | rs2304167 | hCV31722831 | rs11671922 | 0.51 | 0.488273752 | 1 |
| hCV22273419 | rs2304167 | hCV31722832 | rs11084381 | 0.51 | 0.488273752 | 0.8942 |
| hCV22273419 | rs2304167 | hCV31722834 | rs11084382 | 0.51 | 0.488273752 | 0.783 |
| hCV22273419 | rs2304167 | hCV31722835 | rs11668169 | 0.51 | 0.488273752 | 0.8939 |
| hCV22273419 | rs2304167 | hCV31722836 | rs11672026 | 0.51 | 0.488273752 | 0.8884 |
| hCV22273419 | rs2304167 | hCV7841075 | rs1671196 | 0.51 | 0.488273752 | 0.8942 |
| hCV22273419 | rs2304167 | hCV8703249 | rs1654444 | 0.51 | 0.488273752 | 0.633 |
| hCV22273419 | rs2304167 | hCV8717752 | rs1671217 | 0.51 | 0.488273752 | 0.7325 |
| hCV22273419 | rs2304167 | hCV8717761 | rs1654439 | 0.51 | 0.488273752 | 0.5719 |
| hCV22273419 | rs2304167 | hCV8717793 | rs1654433 | 0.51 | 0.488273752 | 0.639 |
| hCV22273419 | rs2304167 | hCV8717794 | rs1654432 | 0.51 | 0.488273752 | 0.639 |
| hCV22273419 | rs2304167 | hCV8717845 | rs892090 | 0.51 | 0.488273752 | 0.7101 |
| hCV22273419 | rs2304167 | hCV8717846 | rs892089 | 0.51 | 0.488273752 | 1 |
| hCV22273419 | rs2304167 | hCV8717871 | rs1654421 | 0.51 | 0.488273752 | 0.754 |
| hCV22273419 | rs2304167 | hCV8717873 | rs1613662 | 0.51 | 0.488273752 | 0.7101 |
| hCV22273419 | rs2304167 | hCV8717881 | rs1654420 | 0.51 | 0.488273752 | 0.8939 |
| hCV22273419 | rs2304167 | hCV8717893 | rs1671192 | 0.51 | 0.488273752 | 1 |
| hCV22273419 | rs2304167 | hCV8718961 | rs1654451 | 0.51 | 0.488273752 | 0.5646 |
| hCV22273419 | rs2304167 | hCV8718972 | rs1654447 | 0.51 | 0.488273752 | 0.6147 |
| hCV22273419 | rs2304167 | hCV9490926 | rs1654419 | 0.51 | 0.488273752 | 0.8939 |
| hCV2303891 | rs1801690 | hCV2658414 | rs8178851 | 0.51 | 0.431588444 | 0.7358 |
| hCV2303891 | rs1801690 | hCV2658416 | rs8178853 | 0.51 | 0.431588444 | 0.7358 |
| hCV2303891 | rs1801690 | hCV2658437 | rs11651658 | 0.51 | 0.431588444 | 0.858 |
| hCV2303891 | rs1801690 | hCV2658444 | rs7209242 | 0.51 | 0.431588444 | 0.497 |
| hCV2303891 | rs1801690 | hCV2658455 | rs11658189 | 0.51 | 0.431588444 | 0.9185 |
| hCV2303891 | rs1801690 | hCV26589423 | rs1014399 | 0.51 | 0.431588444 | 0.574 |
| hCV2303891 | rs1801690 | hCV27842286 | rs8178822 | 0.51 | 0.431588444 | 0.778 |
| hCV2303891 | rs1801690 | hCV29176910 | rs7211380 | 0.51 | 0.431588444 | 0.7358 |
| hCV2303891 | rs1801690 | hCV29577360 | rs9910950 | 0.51 | 0.431588444 | 0.9185 |
| hCV2303891 | rs1801690 | hCV29866571 | rs9891968 | 0.51 | 0.431588444 | 0.8255 |
| hCV2303891 | rs1801690 | hCV29992830 | rs8178839 | 0.51 | 0.431588444 | 0.497 |
| hCV2303891 | rs1801690 | hCV30064665 | rs9902706 | 0.51 | 0.431588444 | 0.9185 |
| hCV2303891 | rs1801690 | hCV30082731 | rs8178838 | 0.51 | 0.431588444 | 0.8247 |
| hCV2303891 | rs1801690 | hCV30118779 | rs8178841 | 0.51 | 0.431588444 | 0.778 |
| hCV2303891 | rs1801690 | hCV30298770 | rs8178842 | 0.51 | 0.431588444 | 0.7215 |
| hCV2303891 | rs1801690 | hCV30352818 | rs8178847 | 0.51 | 0.431588444 | 0.778 |
| hCV2303891 | rs1801690 | hCV30443106 | rs7213041 | 0.51 | 0.431588444 | 0.778 |
| hCV2303891 | rs1801690 | hCV30551147 | rs9908597 | 0.51 | 0.431588444 | 0.8255 |
| hCV2303891 | rs1801690 | hCV31400900 | rs7216660 | 0.51 | 0.431588444 | 0.9185 |
| hCV2303891 | rs1801690 | hDV70764335 | rs16958979 | 0.51 | 0.431588444 | 0.7777 |
| hCV2303891 | rs1801690 | hDV70764357 | rs16959006 | 0.51 | 0.431588444 | 1 |
| hCV233148 | rs1417121 | hCV12073836 | rs1008173 | 0.51 | 0.233111365 | 0.4803 |
| hCV233148 | rs1417121 | hCV12073840 | rs14403 | 0.51 | 0.233111365 | 0.8606 |
| hCV233148 | rs1417121 | hCV15760229 | rs3006939 | 0.51 | 0.233111365 | 0.6414 |
| hCV233148 | rs1417121 | hCV15760238 | rs3006936 | 0.51 | 0.233111365 | 0.6656 |
| hCV233148 | rs1417121 | hCV15760239 | rs3006923 | 0.51 | 0.233111365 | 0.7633 |
| hCV233148 | rs1417121 | hCV15760280 | rs3006940 | 0.51 | 0.233111365 | 0.6414 |

# TABLE 3, page 34 of 71

| Interrogated SNP | Interrogated rs | LD SNP | LD SNP rs | Power | Threshold r² | r² |
|---|---|---|---|---|---|---|
| hCV233148 | rs1417121 | hCV15823024 | rs2125230 | 0.51 | 0.233111365 | 0.4131 |
| hCV233148 | rs1417121 | hCV15885425 | rs2290754 | 0.51 | 0.233111365 | 0.4131 |
| hCV233148 | rs1417121 | hCV15953071 | rs2953329 | 0.51 | 0.233111365 | 0.238 |
| hCV233148 | rs1417121 | hCV15965338 | rs2291410 | 0.51 | 0.233111365 | 0.4485 |
| hCV233148 | rs1417121 | hCV16082410 | rs2881275 | 0.51 | 0.233111365 | 0.4499 |
| hCV233148 | rs1417121 | hCV16189408 | rs2994320 | 0.51 | 0.233111365 | 0.4425 |
| hCV233148 | rs1417121 | hCV1678656 | rs1458024 | 0.51 | 0.233111365 | 0.4499 |
| hCV233148 | rs1417121 | hCV1678674 | rs1458023 | 0.51 | 0.233111365 | 0.4131 |
| hCV233148 | rs1417121 | hCV1678687 | rs320305 | 0.51 | 0.233111365 | 0.3115 |
| hCV233148 | rs1417121 | hCV26034142 | rs9428576 | 0.51 | 0.233111365 | 0.4375 |
| hCV233148 | rs1417121 | hCV26034157 | rs2994329 | 0.51 | 0.233111365 | 0.4814 |
| hCV233148 | rs1417121 | hCV26034158 | rs4515770 | 0.51 | 0.233111365 | 0.6414 |
| hCV233148 | rs1417121 | hCV26034160 | rs2994327 | 0.51 | 0.233111365 | 0.7257 |
| hCV233148 | rs1417121 | hCV26338482 | rs10803143 | 0.51 | 0.233111365 | 0.2626 |
| hCV233148 | rs1417121 | hCV26338512 | rs2994339 | 0.51 | 0.233111365 | 0.4425 |
| hCV233148 | rs1417121 | hCV26338513 | rs3006917 | 0.51 | 0.233111365 | 0.4425 |
| hCV233148 | rs1417121 | hCV26719082 | rs10927046 | 0.51 | 0.233111365 | 0.3102 |
| hCV233148 | rs1417121 | hCV26719085 | rs10927047 | 0.51 | 0.233111365 | 0.3441 |
| hCV233148 | rs1417121 | hCV26719107 | rs7538011 | 0.51 | 0.233111365 | 0.3776 |
| hCV233148 | rs1417121 | hCV26719108 | rs10927035 | 0.51 | 0.233111365 | 0.2612 |
| hCV233148 | rs1417121 | hCV26719113 | rs7517340 | 0.51 | 0.233111365 | 0.3991 |
| hCV233148 | rs1417121 | hCV26719116 | rs10927039 | 0.51 | 0.233111365 | 0.49 |
| hCV233148 | rs1417121 | hCV26719120 | rs10927040 | 0.51 | 0.233111365 | 0.4485 |
| hCV233148 | rs1417121 | hCV26719121 | rs10927041 | 0.51 | 0.233111365 | 0.4485 |
| hCV233148 | rs1417121 | hCV26719149 | rs6675851 | 0.51 | 0.233111365 | 0.4131 |
| hCV233148 | rs1417121 | hCV26719162 | rs4132509 | 0.51 | 0.233111365 | 0.4131 |
| hCV233148 | rs1417121 | hCV26719176 | rs10927076 | 0.51 | 0.233111365 | 0.4131 |
| hCV233148 | rs1417121 | hCV26719192 | rs10803161 | 0.51 | 0.233111365 | 0.4774 |
| hCV233148 | rs1417121 | hCV26719201 | rs4478795 | 0.51 | 0.233111365 | 0.2532 |
| hCV233148 | rs1417121 | hCV26719219 | rs9782958 | 0.51 | 0.233111365 | 0.4131 |
| hCV233148 | rs1417121 | hCV26719222 | rs4553169 | 0.51 | 0.233111365 | 0.4131 |
| hCV233148 | rs1417121 | hCV26719227 | rs10927065 | 0.51 | 0.233111365 | 0.3115 |
| hCV233148 | rs1417121 | hCV26719232 | rs10803158 | 0.51 | 0.233111365 | 0.4131 |
| hCV233148 | rs1417121 | hCV26719233 | rs10927067 | 0.51 | 0.233111365 | 0.4131 |
| hCV233148 | rs1417121 | hCV27498250 | rs3766673 | 0.51 | 0.233111365 | 0.4485 |
| hCV233148 | rs1417121 | hCV29210363 | rs6656918 | 0.51 | 0.233111365 | 0.6414 |
| hCV233148 | rs1417121 | hCV29542869 | rs7534117 | 0.51 | 0.233111365 | 0.4131 |
| hCV233148 | rs1417121 | hCV29560960 | rs7519673 | 0.51 | 0.233111365 | 0.3115 |
| hCV233148 | rs1417121 | hCV29741723 | rs7517921 | 0.51 | 0.233111365 | 0.3711 |
| hCV233148 | rs1417121 | hCV29994467 | rs6694738 | 0.51 | 0.233111365 | 0.3776 |
| hCV233148 | rs1417121 | hCV30084348 | rs9287269 | 0.51 | 0.233111365 | 0.4111 |
| hCV233148 | rs1417121 | hCV30372886 | rs9782883 | 0.51 | 0.233111365 | 0.4131 |
| hCV233148 | rs1417121 | hCV30382231 | rs9428966 | 0.51 | 0.233111365 | 1 |
| hCV233148 | rs1417121 | hCV30690777 | rs12045585 | 0.51 | 0.233111365 | 0.3015 |
| hCV233148 | rs1417121 | hCV30690778 | rs12140414 | 0.51 | 0.233111365 | 0.5616 |
| hCV233148 | rs1417121 | hCV30690780 | rs10737888 | 0.51 | 0.233111365 | 0.6414 |
| hCV233148 | rs1417121 | hCV30690784 | rs4658574 | 0.51 | 0.233111365 | 0.7257 |
| hCV233148 | rs1417121 | hCV31056133 | rs10927006 | 0.51 | 0.233111365 | 0.2564 |
| hCV233148 | rs1417121 | hCV31056162 | rs12049318 | 0.51 | 0.233111365 | 0.2564 |
| hCV233148 | rs1417121 | hCV31523557 | rs10754807 | 0.51 | 0.233111365 | 0.4131 |
| hCV233148 | rs1417121 | hCV31523563 | rs10927051 | 0.51 | 0.233111365 | 0.4633 |
| hCV233148 | rs1417121 | hCV31523638 | rs12037013 | 0.51 | 0.233111365 | 0.3441 |
| hCV233148 | rs1417121 | hCV31523639 | rs12034588 | 0.51 | 0.233111365 | 0.4326 |
| hCV233148 | rs1417121 | hCV31523643 | rs6671475 | 0.51 | 0.233111365 | 0.4485 |
| hCV233148 | rs1417121 | hCV31523650 | rs12048930 | 0.51 | 0.233111365 | 0.3711 |

# TABLE 3, page 35 of 71

| Interrogated SNP | Interrogated rs | LD SNP | LD SNP rs | Power | Threshold r² | r² |
|---|---|---|---|---|---|---|
| hCV233148 | rs1417121 | hCV31523658 | rs12047209 | 0.51 | 0.233111365 | 0.317 |
| hCV233148 | rs1417121 | hCV31523688 | rs12049228 | 0.51 | 0.233111365 | 0.4351 |
| hCV233148 | rs1417121 | hCV31523691 | rs12021907 | 0.51 | 0.233111365 | 0.3115 |
| hCV233148 | rs1417121 | hCV31523707 | rs10803152 | 0.51 | 0.233111365 | 0.3441 |
| hCV233148 | rs1417121 | hCV31523710 | rs10927059 | 0.51 | 0.233111365 | 0.4131 |
| hCV233148 | rs1417121 | hCV31523723 | rs12140040 | 0.51 | 0.233111365 | 0.3202 |
| hCV233148 | rs1417121 | hCV31523736 | rs12124113 | 0.51 | 0.233111365 | 0.3115 |
| hCV233148 | rs1417121 | hCV31523740 | rs12032342 | 0.51 | 0.233111365 | 0.4499 |
| hCV233148 | rs1417121 | hCV31523744 | rs12031994 | 0.51 | 0.233111365 | 0.3115 |
| hCV233148 | rs1417121 | hCV804126 | rs320320 | 0.51 | 0.233111365 | 0.4499 |
| hCV233148 | rs1417121 | hCV8688079 | rs884808 | 0.51 | 0.233111365 | 0.7257 |
| hCV233148 | rs1417121 | hCV8688080 | rs884328 | 0.51 | 0.233111365 | 0.7257 |
| hCV233148 | rs1417121 | hCV8688111 | rs1578275 | 0.51 | 0.233111365 | 0.5616 |
| hCV233148 | rs1417121 | hCV9493073 | rs1058305 | 0.51 | 0.233111365 | 1 |
| hCV233148 | rs1417121 | hCV9493081 | rs1058304 | 0.51 | 0.233111365 | 1 |
| hCV233148 | rs1417121 | hCV97631 | rs1538773 | 0.51 | 0.233111365 | 0.6414 |
| hCV233148 | rs1417121 | hDV71836703 | rs6429433 | 0.51 | 0.233111365 | 0.4157 |
| hCV233148 | rs1417121 | hDV90784784 | rs320339 | 0.51 | 0.233111365 | 0.3409 |
| hCV2442143 | rs12544854 | hCV15753018 | rs2299606 | 0.51 | 0.805894186 | 0.9649 |
| hCV2442143 | rs12544854 | hCV15844343 | rs2427746 | 0.51 | 0.805894186 | 0.8982 |
| hCV2442143 | rs12544854 | hCV2442136 | rs12155668 | 0.51 | 0.805894186 | 1 |
| hCV2442143 | rs12544854 | hCV2442137 | rs12155885 | 0.51 | 0.805894186 | 1 |
| hCV2442143 | rs12544854 | hCV2442146 | rs966118 | 0.51 | 0.805894186 | 1 |
| hCV2442143 | rs12544854 | hCV2442155 | rs3753117 | 0.51 | 0.805894186 | 1 |
| hCV2442143 | rs12544854 | hCV2442156 | rs35573135 | 0.51 | 0.805894186 | 0.841 |
| hCV2442143 | rs12544854 | hCV26696706 | rs2299607 | 0.51 | 0.805894186 | 1 |
| hCV2442143 | rs12544854 | hCV27474371 | rs3753116 | 0.51 | 0.805894186 | 1 |
| hCV2442143 | rs12544854 | hCV31495915 | rs3753115 | 0.51 | 0.805894186 | 1 |
| hCV2442143 | rs12544854 | hCV31495928 | rs12548139 | 0.51 | 0.805894186 | 1 |
| hCV2442143 | rs12544854 | hCV8947815 | rs1049874 | 0.51 | 0.805894186 | 1 |
| hCV2499170 | rs169713 | hCV2238240 | rs209773 | 0.51 | 0.626344353 | 0.9402 |
| hCV2499170 | rs169713 | hCV2238245 | rs23805 | 0.51 | 0.626344353 | 0.8916 |
| hCV2499170 | rs169713 | hCV2238247 | rs209778 | 0.51 | 0.626344353 | 0.6678 |
| hCV2499170 | rs169713 | hCV2238250 | rs209780 | 0.51 | 0.626344353 | 0.8809 |
| hCV2499170 | rs169713 | hCV2238261 | rs209814 | 0.51 | 0.626344353 | 0.6439 |
| hCV2499170 | rs169713 | hCV2238263 | rs209812 | 0.51 | 0.626344353 | 0.6422 |
| hCV2499170 | rs169713 | hCV2499165 | rs209774 | 0.51 | 0.626344353 | 1 |
| hCV2499170 | rs169713 | hCV2499169 | rs85219 | 0.51 | 0.626344353 | 1 |
| hCV2499170 | rs169713 | hCV2499176 | rs9380643 | 0.51 | 0.626344353 | 0.6493 |
| hCV2499170 | rs169713 | hCV2499198 | rs1205883 | 0.51 | 0.626344353 | 0.6439 |
| hCV2499170 | rs169713 | hCV2499199 | rs1205884 | 0.51 | 0.626344353 | 0.6439 |
| hCV2499170 | rs169713 | hCV2499201 | rs1205887 | 0.51 | 0.626344353 | 0.6439 |
| hCV2499170 | rs169713 | hCV31001553 | rs10947661 | 0.51 | 0.626344353 | 0.8779 |
| hCV2499170 | rs169713 | hCV7465311 | rs1205863 | 0.51 | 0.626344353 | 0.6439 |
| hCV2499170 | rs169713 | hCV7465312 | rs864245 | 0.51 | 0.626344353 | 0.6439 |
| hCV2499170 | rs169713 | hCV7465347 | rs1205852 | 0.51 | 0.626344353 | 0.6439 |
| hCV2499170 | rs169713 | hCV7465349 | rs1205850 | 0.51 | 0.626344353 | 0.8424 |
| hCV2499170 | rs169713 | hCV7465354 | rs1205849 | 0.51 | 0.626344353 | 0.8932 |
| hCV2499170 | rs169713 | hCV7465364 | rs864244 | 0.51 | 0.626344353 | 0.9459 |
| hCV2499170 | rs169713 | hCV7465377 | rs1210621 | 0.51 | 0.626344353 | 0.9438 |
| hCV2499170 | rs169713 | hCV7465418 | rs876828 | 0.51 | 0.626344353 | 0.7396 |
| hCV2499170 | rs169713 | hDV101721202 | rs9394412 | 0.51 | 0.626344353 | 0.8779 |
| hCV2532034 | rs6003 | hCV11888484 | rs6694672 | 0.51 | 0.719447218 | 0.9425 |
| hCV2532034 | rs6003 | hCV11888496 | rs7554757 | 0.51 | 0.719447218 | 1 |
| hCV2532034 | rs6003 | hCV11888533 | rs1115247 | 0.51 | 0.719447218 | 1 |

# TABLE 3, page 36 of 71

| Interrogated SNP | Interrogated rs | LD SNP | LD SNP rs | Power | Threshold r² | r² |
|---|---|---|---|---|---|---|
| hCV2532034 | rs6003 | hCV11888556 | rs7542397 | 0.51 | 0.719447218 | 1 |
| hCV2532034 | rs6003 | hCV11888566 | rs1888991 | 0.51 | 0.719447218 | 0.9451 |
| hCV2532034 | rs6003 | hCV15832928 | rs2151133 | 0.51 | 0.719447218 | 0.8803 |
| hCV2532034 | rs6003 | hCV15832929 | rs2151134 | 0.51 | 0.719447218 | 1 |
| hCV2532034 | rs6003 | hCV1648949 | rs6692162 | 0.51 | 0.719447218 | 1 |
| hCV2532034 | rs6003 | hCV1739697 | rs10429911 | 0.51 | 0.719447218 | 0.8913 |
| hCV2532034 | rs6003 | hCV1739698 | rs1415217 | 0.51 | 0.719447218 | 0.8913 |
| hCV2532034 | rs6003 | hCV1739699 | rs7520503 | 0.51 | 0.719447218 | 0.945 |
| hCV2532034 | rs6003 | hCV1739712 | rs510135 | 0.51 | 0.719447218 | 0.8913 |
| hCV2532034 | rs6003 | hCV1742489 | rs476390 | 0.51 | 0.719447218 | 0.8913 |
| hCV2532034 | rs6003 | hCV1742520 | rs615647 | 0.51 | 0.719447218 | 0.8913 |
| hCV2532034 | rs6003 | hCV1742521 | rs518149 | 0.51 | 0.719447218 | 0.8913 |
| hCV2532034 | rs6003 | hCV1742540 | rs10754219 | 0.51 | 0.719447218 | 0.8802 |
| hCV2532034 | rs6003 | hCV201028 | rs10733087 | 0.51 | 0.719447218 | 0.8803 |
| hCV2532034 | rs6003 | hCV201029 | rs10754213 | 0.51 | 0.719447218 | 0.9425 |
| hCV2532034 | rs6003 | hCV209646 | rs12734260 | 0.51 | 0.719447218 | 0.8013 |
| hCV2532034 | rs6003 | hCV240531 | rs6703400 | 0.51 | 0.719447218 | 1 |
| hCV2532034 | rs6003 | hCV240532 | rs2026429 | 0.51 | 0.719447218 | 1 |
| hCV2532034 | rs6003 | hCV247773 | rs1332663 | 0.51 | 0.719447218 | 1 |
| hCV2532034 | rs6003 | hCV2532031 | rs1412632 | 0.51 | 0.719447218 | 1 |
| hCV2532034 | rs6003 | hCV2532032 | rs4915148 | 0.51 | 0.719447218 | 1 |
| hCV2532034 | rs6003 | hCV2532033 | rs1759006 | 0.51 | 0.719447218 | 1 |
| hCV2532034 | rs6003 | hCV2532038 | rs1759008 | 0.51 | 0.719447218 | 1 |
| hCV2532034 | rs6003 | hCV2532039 | rs1759009 | 0.51 | 0.719447218 | 1 |
| hCV2532034 | rs6003 | hCV2532061 | rs857021 | 0.51 | 0.719447218 | 0.9424 |
| hCV2532034 | rs6003 | hCV2532062 | rs1332669 | 0.51 | 0.719447218 | 0.9425 |
| hCV2532034 | rs6003 | hCV26753641 | rs10801590 | 0.51 | 0.719447218 | 1 |
| hCV2532034 | rs6003 | hCV268763 | rs4350226 | 0.51 | 0.719447218 | 0.8913 |
| hCV2532034 | rs6003 | hCV26961419 | rs10732296 | 0.51 | 0.719447218 | 0.8913 |
| hCV2532034 | rs6003 | hCV26961484 | rs4915379 | 0.51 | 0.719447218 | 0.945 |
| hCV2532034 | rs6003 | hCV2759661 | rs12731209 | 0.51 | 0.719447218 | 1 |
| hCV2532034 | rs6003 | hCV2759662 | rs1170880 | 0.51 | 0.719447218 | 0.9395 |
| hCV2532034 | rs6003 | hCV2759663 | rs928440 | 0.51 | 0.719447218 | 1 |
| hCV2532034 | rs6003 | hCV2759664 | rs928439 | 0.51 | 0.719447218 | 1 |
| hCV2532034 | rs6003 | hCV2759671 | rs2336595 | 0.51 | 0.719447218 | 1 |
| hCV2532034 | rs6003 | hCV2759673 | rs1412639 | 0.51 | 0.719447218 | 1 |
| hCV2532034 | rs6003 | hCV2759676 | rs4915156 | 0.51 | 0.719447218 | 0.8803 |
| hCV2532034 | rs6003 | hCV2759677 | rs10801588 | 0.51 | 0.719447218 | 0.8486 |
| hCV2532034 | rs6003 | hCV2759678 | rs1571964 | 0.51 | 0.719447218 | 1 |
| hCV2532034 | rs6003 | hCV2759679 | rs6677082 | 0.51 | 0.719447218 | 1 |
| hCV2532034 | rs6003 | hCV2759685 | rs877897 | 0.51 | 0.719447218 | 1 |
| hCV2532034 | rs6003 | hCV2759688 | rs10922169 | 0.51 | 0.719447218 | 1 |
| hCV2532034 | rs6003 | hCV2759691 | rs4915337 | 0.51 | 0.719447218 | 1 |
| hCV2532034 | rs6003 | hCV2759693 | rs10754215 | 0.51 | 0.719447218 | 1 |
| hCV2532034 | rs6003 | hCV2759696 | rs7411719 | 0.51 | 0.719447218 | 1 |
| hCV2532034 | rs6003 | hCV2759703 | rs1412640 | 0.51 | 0.719447218 | 1 |
| hCV2532034 | rs6003 | hCV2759704 | rs1953064 | 0.51 | 0.719447218 | 0.8803 |
| hCV2532034 | rs6003 | hCV2759709 | rs4915313 | 0.51 | 0.719447218 | 1 |
| hCV2532034 | rs6003 | hCV2759711 | rs4915309 | 0.51 | 0.719447218 | 1 |
| hCV2532034 | rs6003 | hCV2759725 | rs6670056 | 0.51 | 0.719447218 | 1 |
| hCV2532034 | rs6003 | hCV27898326 | rs4915316 | 0.51 | 0.719447218 | 0.9396 |
| hCV2532034 | rs6003 | hCV27898327 | rs4915327 | 0.51 | 0.719447218 | 1 |
| hCV2532034 | rs6003 | hCV28005188 | rs4342879 | 0.51 | 0.719447218 | 0.8913 |
| hCV2532034 | rs6003 | hCV29222813 | rs4915315 | 0.51 | 0.719447218 | 0.8023 |
| hCV2532034 | rs6003 | hCV29222814 | rs6428387 | 0.51 | 0.719447218 | 0.8803 |

# TABLE 3, page 37 of 71

| Interrogated SNP | Interrogated rs | LD SNP | LD SNP rs | Power | Threshold r² | r² |
|---|---|---|---|---|---|---|
| hCV2532034 | rs6003 | hCV29222817 | rs6656858 | 0.51 | 0.719447218 | 1 |
| hCV2532034 | rs6003 | hCV29295007 | rs6656448 | 0.51 | 0.719447218 | 0.88 |
| hCV2532034 | rs6003 | hCV29491389 | rs7513826 | 0.51 | 0.719447218 | 0.8913 |
| hCV2532034 | rs6003 | hCV29633649 | rs7539642 | 0.51 | 0.719447218 | 0.8803 |
| hCV2532034 | rs6003 | hCV29724082 | rs9427661 | 0.51 | 0.719447218 | 0.9425 |
| hCV2532034 | rs6003 | hCV29796191 | rs9427660 | 0.51 | 0.719447218 | 1 |
| hCV2532034 | rs6003 | hCV29904681 | rs6680497 | 0.51 | 0.719447218 | 1 |
| hCV2532034 | rs6003 | hCV29922678 | rs9427940 | 0.51 | 0.719447218 | 0.8911 |
| hCV2532034 | rs6003 | hCV30321245 | rs7523013 | 0.51 | 0.719447218 | 0.8913 |
| hCV2532034 | rs6003 | hCV30373286 | rs6702340 | 0.51 | 0.719447218 | 1 |
| hCV2532034 | rs6003 | hCV30391085 | rs9427656 | 0.51 | 0.719447218 | 0.7282 |
| hCV2532034 | rs6003 | hCV30391086 | rs9427942 | 0.51 | 0.719447218 | 0.9396 |
| hCV2532034 | rs6003 | hCV30589276 | rs9427657 | 0.51 | 0.719447218 | 0.7525 |
| hCV2532034 | rs6003 | hCV3091554 | rs5997 | 0.51 | 0.719447218 | 1 |
| hCV2532034 | rs6003 | hCV31565477 | rs10801587 | 0.51 | 0.719447218 | 0.8802 |
| hCV2532034 | rs6003 | hCV31565478 | rs10754214 | 0.51 | 0.719447218 | 0.9425 |
| hCV2532034 | rs6003 | hCV31565485 | rs10922166 | 0.51 | 0.719447218 | 1 |
| hCV2532034 | rs6003 | hCV31565488 | rs6671696 | 0.51 | 0.719447218 | 1 |
| hCV2532034 | rs6003 | hCV31565509 | rs12092294 | 0.51 | 0.719447218 | 0.9425 |
| hCV2532034 | rs6003 | hCV31565527 | rs12039586 | 0.51 | 0.719447218 | 1 |
| hCV2532034 | rs6003 | hCV31795582 | rs6678066 | 0.51 | 0.719447218 | 0.8913 |
| hCV2532034 | rs6003 | hCV32375831 | rs4915378 | 0.51 | 0.719447218 | 0.7347 |
| hCV2532034 | rs6003 | hCV356522 | rs10732295 | 0.51 | 0.719447218 | 0.9451 |
| hCV2532034 | rs6003 | hCV406628 | rs1576879 | 0.51 | 0.719447218 | 1 |
| hCV2532034 | rs6003 | hCV489039 | rs6679189 | 0.51 | 0.719447218 | 1 |
| hCV2532034 | rs6003 | hCV65774 | rs7534353 | 0.51 | 0.719447218 | 1 |
| hCV2532034 | rs6003 | hCV8348876 | rs1332662 | 0.51 | 0.719447218 | 1 |
| hCV2532034 | rs6003 | hCV8350834 | rs1764629 | 0.51 | 0.719447218 | 0.9451 |
| hCV2532034 | rs6003 | hCV8350843 | rs1556763 | 0.51 | 0.719447218 | 0.945 |
| hCV2532034 | rs6003 | hCV8356383 | rs1764800 | 0.51 | 0.719447218 | 0.945 |
| hCV2532034 | rs6003 | hCV8356417 | rs12677 | 0.51 | 0.719447218 | 1 |
| hCV2532034 | rs6003 | hCV8356418 | rs1537319 | 0.51 | 0.719447218 | 0.8803 |
| hCV2532034 | rs6003 | hCV8356419 | rs1412631 | 0.51 | 0.719447218 | 1 |
| hCV2532034 | rs6003 | hCV8356425 | rs1627765 | 0.51 | 0.719447218 | 0.9451 |
| hCV2532034 | rs6003 | hCV8356430 | rs1759007 | 0.51 | 0.719447218 | 1 |
| hCV2532034 | rs6003 | hCV8356446 | rs1412634 | 0.51 | 0.719447218 | 1 |
| hCV2532034 | rs6003 | hCV8356520 | rs1170881 | 0.51 | 0.719447218 | 1 |
| hCV2532034 | rs6003 | hCV836489 | rs616675 | 0.51 | 0.719447218 | 0.8903 |
| hCV2532034 | rs6003 | hCV87892 | rs2336597 | 0.51 | 0.719447218 | 0.9425 |
| hCV2532034 | rs6003 | hCV9114466 | rs3891964 | 0.51 | 0.719447218 | 0.9425 |
| hCV2532034 | rs6003 | hCV9114630 | rs1576880 | 0.51 | 0.719447218 | 1 |
| hCV2532034 | rs6003 | hCV9114656 | rs9427662 | 0.51 | 0.719447218 | 0.9425 |
| hCV2532034 | rs6003 | hCV9114658 | rs13376702 | 0.51 | 0.719447218 | 0.7419 |
| hCV25474413 | rs3822057 | hCV11786147 | rs4862662 | 0.51 | 0.057574841 | 0.2412 |
| hCV25474413 | rs3822057 | hCV11786258 | rs4253303 | 0.51 | 0.057574841 | 0.2622 |
| hCV25474413 | rs3822057 | hCV11786259 | rs4253304 | 0.51 | 0.057574841 | 0.2992 |
| hCV25474413 | rs3822057 | hCV11786295 | rs4253421 | 0.51 | 0.057574841 | 0.0942 |
| hCV25474413 | rs3822057 | hCV11786301 | rs5970 | 0.51 | 0.057574841 | 0.0764 |
| hCV25474413 | rs3822057 | hCV11786307 | rs1062547 | 0.51 | 0.057574841 | 0.4723 |
| hCV25474413 | rs3822057 | hCV11786311 | rs13145616 | 0.51 | 0.057574841 | 0.0838 |
| hCV25474413 | rs3822057 | hCV11786327 | rs13133050 | 0.51 | 0.057574841 | 0.2298 |
| hCV25474413 | rs3822057 | hCV12066116 | rs1877320 | 0.51 | 0.057574841 | 0.1289 |
| hCV25474413 | rs3822057 | hCV12066118 | rs2048 | 0.51 | 0.057574841 | 0.3088 |
| hCV25474413 | rs3822057 | hCV12066119 | rs1912826 | 0.51 | 0.057574841 | 0.3323 |
| hCV25474413 | rs3822057 | hCV12066124 | rs2036914 | 0.51 | 0.057574841 | 0.9449 |

# TABLE 3, page 38 of 71

| Interrogated SNP | Interrogated rs | LD SNP | LD SNP rs | Power | Threshold $r^2$ | $r^2$ |
|---|---|---|---|---|---|---|
| hCV25474413 | rs3822057 | hCV12066129 | rs1593 | 0.51 | 0.057574841 | 0.1397 |
| hCV25474413 | rs3822057 | hCV12086148 | rs1877321 | 0.51 | 0.057574841 | 0.0612 |
| hCV25474413 | rs3822057 | hCV15793897 | rs3087505 | 0.51 | 0.057574841 | 0.1042 |
| hCV25474413 | rs3822057 | hCV15811716 | rs2102575 | 0.51 | 0.057574841 | 0.0982 |
| hCV25474413 | rs3822057 | hCV15968025 | rs2292425 | 0.51 | 0.057574841 | 0.169 |
| hCV25474413 | rs3822057 | hCV15968026 | rs2292426 | 0.51 | 0.057574841 | 0.1853 |
| hCV25474413 | rs3822057 | hCV15968034 | rs2292428 | 0.51 | 0.057574841 | 0.1541 |
| hCV25474413 | rs3822057 | hCV15968043 | rs2292423 | 0.51 | 0.057574841 | 0.313 |
| hCV25474413 | rs3822057 | hCV15975109 | rs2304596 | 0.51 | 0.057574841 | 0.0626 |
| hCV25474413 | rs3822057 | hCV16172925 | rs2241818 | 0.51 | 0.057574841 | 0.0581 |
| hCV25474413 | rs3822057 | hCV16172935 | rs2241817 | 0.51 | 0.057574841 | 0.4618 |
| hCV25474413 | rs3822057 | hCV2103343 | rs4241824 | 0.51 | 0.057574841 | 0.9811 |
| hCV25474413 | rs3822057 | hCV2103375 | rs12502630 | 0.51 | 0.057574841 | 0.0598 |
| hCV25474413 | rs3822057 | hCV2103388 | rs4613610 | 0.51 | 0.057574841 | 0.1442 |
| hCV25474413 | rs3822057 | hCV2103391 | rs1008728 | 0.51 | 0.057574841 | 0.2797 |
| hCV25474413 | rs3822057 | hCV2103392 | rs12500826 | 0.51 | 0.057574841 | 0.4611 |
| hCV25474413 | rs3822057 | hCV2103402 | rs9993749 | 0.51 | 0.057574841 | 0.0612 |
| hCV25474413 | rs3822057 | hCV22272267 | rs3733402 | 0.51 | 0.057574841 | 0.312 |
| hCV25474413 | rs3822057 | hCV25474414 | rs4253399 | 0.51 | 0.057574841 | 0.596 |
| hCV25474413 | rs3822057 | hCV25634763 | rs4253241 | 0.51 | 0.057574841 | 0.0739 |
| hCV25474413 | rs3822057 | hCV25988221 | rs9995366 | 0.51 | 0.057574841 | 0.0873 |
| hCV25474413 | rs3822057 | hCV25990131 | rs13146272 | 0.51 | 0.057574841 | 0.1706 |
| hCV25474413 | rs3822057 | hCV26038139 | rs4253405 | 0.51 | 0.057574841 | 0.6414 |
| hCV25474413 | rs3822057 | hCV26265231 | rs7684025 | 0.51 | 0.057574841 | 0.2834 |
| hCV25474413 | rs3822057 | hCV27309991 | rs4572916 | 0.51 | 0.057574841 | 0.1491 |
| hCV25474413 | rs3822057 | hCV27473099 | rs3733403 | 0.51 | 0.057574841 | 0.1093 |
| hCV25474413 | rs3822057 | hCV27474895 | rs3756011 | 0.51 | 0.057574841 | 0.5232 |
| hCV25474413 | rs3822057 | hCV27477533 | rs3756008 | 0.51 | 0.057574841 | 0.577 |
| hCV25474413 | rs3822057 | hCV27482765 | rs3775301 | 0.51 | 0.057574841 | 0.0626 |
| hCV25474413 | rs3822057 | hCV27490984 | rs3822058 | 0.51 | 0.057574841 | 0.477 |
| hCV25474413 | rs3822057 | hCV27521729 | rs3822056 | 0.51 | 0.057574841 | 0.1222 |
| hCV25474413 | rs3822057 | hCV27902803 | rs4862665 | 0.51 | 0.057574841 | 0.0873 |
| hCV25474413 | rs3822057 | hCV27902808 | rs4253236 | 0.51 | 0.057574841 | 0.1514 |
| hCV25474413 | rs3822057 | hCV28960679 | rs6844764 | 0.51 | 0.057574841 | 0.1108 |
| hCV25474413 | rs3822057 | hCV29053261 | rs6842047 | 0.51 | 0.057574841 | 0.1042 |
| hCV25474413 | rs3822057 | hCV29053264 | rs7667777 | 0.51 | 0.057574841 | 0.2192 |
| hCV25474413 | rs3822057 | hCV29053265 | rs4253244 | 0.51 | 0.057574841 | 0.1369 |
| hCV25474413 | rs3822057 | hCV29640635 | rs10029715 | 0.51 | 0.057574841 | 0.0904 |
| hCV25474413 | rs3822057 | hCV29718000 | rs4253238 | 0.51 | 0.057574841 | 0.3736 |
| hCV25474413 | rs3822057 | hCV29826351 | rs10025990 | 0.51 | 0.057574841 | 0.1507 |
| hCV25474413 | rs3822057 | hCV29877725 | rs4253295 | 0.51 | 0.057574841 | 0.2932 |
| hCV25474413 | rs3822057 | hCV30307525 | rs10025152 | 0.51 | 0.057574841 | 0.0904 |
| hCV25474413 | rs3822057 | hCV30492573 | rs10471184 | 0.51 | 0.057574841 | 0.1042 |
| hCV25474413 | rs3822057 | hCV30562347 | rs4253418 | 0.51 | 0.057574841 | 0.0597 |
| hCV25474413 | rs3822057 | hCV30983902 | rs4862668 | 0.51 | 0.057574841 | 0.1289 |
| hCV25474413 | rs3822057 | hCV30983907 | rs4253246 | 0.51 | 0.057574841 | 0.0739 |
| hCV25474413 | rs3822057 | hCV30983927 | rs6552962 | 0.51 | 0.057574841 | 0.0626 |
| hCV25474413 | rs3822057 | hCV32209629 | rs12715865 | 0.51 | 0.057574841 | 0.1687 |
| hCV25474413 | rs3822057 | hCV32209636 | rs11132387 | 0.51 | 0.057574841 | 0.4496 |
| hCV25474413 | rs3822057 | hCV32209637 | rs13143773 | 0.51 | 0.057574841 | 0.302 |
| hCV25474413 | rs3822057 | hCV32209638 | rs12507040 | 0.51 | 0.057574841 | 0.3609 |
| hCV25474413 | rs3822057 | hCV32291217 | rs4253323 | 0.51 | 0.057574841 | 0.0626 |
| hCV25474413 | rs3822057 | hCV32291256 | rs4253406 | 0.51 | 0.057574841 | 0.0668 |
| hCV25474413 | rs3822057 | hCV32291269 | rs4253417 | 0.51 | 0.057574841 | 0.419 |
| hCV25474413 | rs3822057 | hCV32291286 | rs4253422 | 0.51 | 0.057574841 | 0.2386 |

| Interrogated SNP | Interrogated rs | LD SNP | LD SNP rs | Power | Threshold $r^2$ | $r^2$ |
|---|---|---|---|---|---|---|
| hCV25474413 | rs3822057 | hCV32291287 | rs4253423 | 0.51 | 0.057574841 | 0.2386 |
| hCV25474413 | rs3822057 | hCV32291295 | rs4253292 | 0.51 | 0.057574841 | 0.1106 |
| hCV25474413 | rs3822057 | hCV32291301 | rs4253302 | 0.51 | 0.057574841 | 0.0583 |
| hCV25474413 | rs3822057 | hCV32295028 | rs4253260 | 0.51 | 0.057574841 | 0.0626 |
| hCV25474413 | rs3822057 | hCV3229991 | rs4241815 | 0.51 | 0.057574841 | 0.312 |
| hCV25474413 | rs3822057 | hCV3229992 | rs3775298 | 0.51 | 0.057574841 | 0.312 |
| hCV25474413 | rs3822057 | hCV3229995 | rs11132382 | 0.51 | 0.057574841 | 0.3554 |
| hCV25474413 | rs3822057 | hCV3230000 | rs4253294 | 0.51 | 0.057574841 | 0.1258 |
| hCV25474413 | rs3822057 | hCV3230001 | rs4253296 | 0.51 | 0.057574841 | 0.0739 |
| hCV25474413 | rs3822057 | hCV3230002 | rs4253297 | 0.51 | 0.057574841 | 0.2517 |
| hCV25474413 | rs3822057 | hCV3230003 | rs2304595 | 0.51 | 0.057574841 | 0.3609 |
| hCV25474413 | rs3822057 | hCV3230004 | rs4253301 | 0.51 | 0.057574841 | 0.0995 |
| hCV25474413 | rs3822057 | hCV3230006 | rs4253308 | 0.51 | 0.057574841 | 0.2932 |
| hCV25474413 | rs3822057 | hCV3230007 | rs4253311 | 0.51 | 0.057574841 | 0.312 |
| hCV25474413 | rs3822057 | hCV3230011 | rs4253320 | 0.51 | 0.057574841 | 0.2517 |
| hCV25474413 | rs3822057 | hCV3230013 | rs3775303 | 0.51 | 0.057574841 | 0.2992 |
| hCV25474413 | rs3822057 | hCV3230014 | rs4861709 | 0.51 | 0.057574841 | 0.1258 |
| hCV25474413 | rs3822057 | hCV3230017 | rs4253327 | 0.51 | 0.057574841 | 0.0594 |
| hCV25474413 | rs3822057 | hCV3230018 | rs925453 | 0.51 | 0.057574841 | 0.1356 |
| hCV25474413 | rs3822057 | hCV3230019 | rs4253332 | 0.51 | 0.057574841 | 0.1286 |
| hCV25474413 | rs3822057 | hCV3230021 | rs13135645 | 0.51 | 0.057574841 | 0.1443 |
| hCV25474413 | rs3822057 | hCV3230022 | rs11132383 | 0.51 | 0.057574841 | 0.1929 |
| hCV25474413 | rs3822057 | hCV3230025 | rs3756009 | 0.51 | 0.057574841 | 0.6037 |
| hCV25474413 | rs3822057 | hCV3230030 | rs4253408 | 0.51 | 0.057574841 | 0.0716 |
| hCV25474413 | rs3822057 | hCV3230031 | rs4253419 | 0.51 | 0.057574841 | 0.2386 |
| hCV25474413 | rs3822057 | hCV3230032 | rs5974 | 0.51 | 0.057574841 | 0.0838 |
| hCV25474413 | rs3822057 | hCV3230038 | rs2289252 | 0.51 | 0.057574841 | 0.4122 |
| hCV25474413 | rs3822057 | hCV3230083 | rs10013653 | 0.51 | 0.057574841 | 0.3369 |
| hCV25474413 | rs3822057 | hCV3230084 | rs7682918 | 0.51 | 0.057574841 | 0.2521 |
| hCV25474413 | rs3822057 | hCV3230094 | rs7687818 | 0.51 | 0.057574841 | 0.3057 |
| hCV25474413 | rs3822057 | hCV3230096 | rs3817184 | 0.51 | 0.057574841 | 0.2412 |
| hCV25474413 | rs3822057 | hCV3230097 | rs3736455 | 0.51 | 0.057574841 | 0.2152 |
| hCV25474413 | rs3822057 | hCV3230101 | rs6835839 | 0.51 | 0.057574841 | 0.0843 |
| hCV25474413 | rs3822057 | hCV3230106 | rs1473597 | 0.51 | 0.057574841 | 0.1509 |
| hCV25474413 | rs3822057 | hCV3230110 | rs2276917 | 0.51 | 0.057574841 | 0.1608 |
| hCV25474413 | rs3822057 | hCV3230113 | rs1053094 | 0.51 | 0.057574841 | 0.2648 |
| hCV25474413 | rs3822057 | hCV3230118 | rs4253429 | 0.51 | 0.057574841 | 0.2386 |
| hCV25474413 | rs3822057 | hCV3230119 | rs4253430 | 0.51 | 0.057574841 | 0.4654 |
| hCV25474413 | rs3822057 | hCV3230121 | rs4253431 | 0.51 | 0.057574841 | 0.0838 |
| hCV25474413 | rs3822057 | hCV3230125 | rs11938564 | 0.51 | 0.057574841 | 0.2911 |
| hCV25474413 | rs3822057 | hCV3230131 | rs13136269 | 0.51 | 0.057574841 | 0.3609 |
| hCV25474413 | rs3822057 | hCV3230133 | rs12511874 | 0.51 | 0.057574841 | 0.3083 |
| hCV25474413 | rs3822057 | hCV3230134 | rs12500151 | 0.51 | 0.057574841 | 0.3453 |
| hCV25474413 | rs3822057 | hCV3230136 | rs13116273 | 0.51 | 0.057574841 | 0.3534 |
| hCV25474413 | rs3822057 | hCV32313006 | rs4253248 | 0.51 | 0.057574841 | 0.3617 |
| hCV25474413 | rs3822057 | hCV32313007 | rs4862666 | 0.51 | 0.057574841 | 0.0873 |
| hCV25474413 | rs3822057 | hCV32313024 | rs4253239 | 0.51 | 0.057574841 | 0.1106 |
| hCV25474413 | rs3822057 | hCV32358975 | rs4253255 | 0.51 | 0.057574841 | 0.2992 |
| hCV25474413 | rs3822057 | hCV32358984 | rs4253256 | 0.51 | 0.057574841 | 0.1464 |
| hCV25474413 | rs3822057 | hCV8241628 | rs907439 | 0.51 | 0.057574841 | 0.1491 |
| hCV25474413 | rs3822057 | hCV8241630 | rs925451 | 0.51 | 0.057574841 | 0.596 |
| hCV25474413 | rs3822057 | hCV8241631 | rs1511802 | 0.51 | 0.057574841 | 0.3119 |
| hCV25474413 | rs3822057 | hCV8241632 | rs1511801 | 0.51 | 0.057574841 | 0.3142 |
| hCV25474413 | rs3822057 | hCV8241633 | rs1511800 | 0.51 | 0.057574841 | 0.0873 |
| hCV25474413 | rs3822057 | hDV71222711 | rs4253252 | 0.51 | 0.057574841 | 0.3617 |

# TABLE 3, page 40 of 71

| Interrogated SNP | Interrogated rs | LD SNP | LD SNP rs | Power | Threshold r² | r² |
|---|---|---|---|---|---|---|
| hCV25597241 | rs3782320 | hCV15835026 | rs2878772 | 0.51 | 0.904876352 | 1 |
| hCV25597241 | rs3782320 | hCV27481297 | rs3782318 | 0.51 | 0.904876352 | 1 |
| hCV25597241 | rs3782320 | hDV70885868 | rs17124174 | 0.51 | 0.904876352 | 1 |
| hCV25597241 | rs3782320 | hDV70885870 | rs17124176 | 0.51 | 0.904876352 | 1 |
| hCV25610857 | rs8176693 | hCV11571465 | rs2301612 | 0.51 | 0.148365232 | 0.1634 |
| hCV25610857 | rs8176693 | hCV11840510 | rs9411367 | 0.51 | 0.148365232 | 0.4123 |
| hCV25610857 | rs8176693 | hCV153353 | rs7469576 | 0.51 | 0.148365232 | 0.4257 |
| hCV25610857 | rs8176693 | hCV15862346 | rs2073934 | 0.51 | 0.148365232 | 0.207 |
| hCV25610857 | rs8176693 | hCV2535958 | rs2519198 | 0.51 | 0.148365232 | 0.1685 |
| hCV25610857 | rs8176693 | hCV25610771 | rs8176751 | 0.51 | 0.148365232 | 0.8731 |
| hCV25610857 | rs8176693 | hCV25610772 | rs8176746 | 0.51 | 0.148365232 | 1 |
| hCV25610857 | rs8176693 | hCV25610773 | rs8176747 | 0.51 | 0.148365232 | 0.6839 |
| hCV25610857 | rs8176693 | hCV25610781 | rs8176749 | 0.51 | 0.148365232 | 1 |
| hCV25610857 | rs8176693 | hCV25610791 | rs8176743 | 0.51 | 0.148365232 | 1 |
| hCV25610857 | rs8176693 | hCV25757025 | rs2269894 | 0.51 | 0.148365232 | 0.1542 |
| hCV25610857 | rs8176693 | hCV25987572 | rs4310274 | 0.51 | 0.148365232 | 0.3888 |
| hCV25610857 | rs8176693 | hCV26744892 | rs11244079 | 0.51 | 0.148365232 | 0.8127 |
| hCV25610857 | rs8176693 | hCV26744899 | rs10751505 | 0.51 | 0.148365232 | 0.1768 |
| hCV25610857 | rs8176693 | hCV27224736 | rs2073870 | 0.51 | 0.148365232 | 0.3707 |
| hCV25610857 | rs8176693 | hCV27224742 | rs4454354 | 0.51 | 0.148365232 | 0.234 |
| hCV25610857 | rs8176693 | hCV27224746 | rs10793959 | 0.51 | 0.148365232 | 0.2969 |
| hCV25610857 | rs8176693 | hCV27224748 | rs4246169 | 0.51 | 0.148365232 | 0.3912 |
| hCV25610857 | rs8176693 | hCV27224776 | rs7852396 | 0.51 | 0.148365232 | 0.3577 |
| hCV25610857 | rs8176693 | hCV27224778 | rs11244041 | 0.51 | 0.148365232 | 0.3526 |
| hCV25610857 | rs8176693 | hCV27478783 | rs3761823 | 0.51 | 0.148365232 | 0.2233 |
| hCV25610857 | rs8176693 | hCV27859399 | rs7853989 | 0.51 | 0.148365232 | 0.817 |
| hCV25610857 | rs8176693 | hCV27886018 | rs4962104 | 0.51 | 0.148365232 | 0.2333 |
| hCV25610857 | rs8176693 | hCV27936941 | rs4379511 | 0.51 | 0.148365232 | 0.4056 |
| hCV25610857 | rs8176693 | hCV27936942 | rs4424335 | 0.51 | 0.148365232 | 0.7914 |
| hCV25610857 | rs8176693 | hCV28002068 | rs4322078 | 0.51 | 0.148365232 | 0.3912 |
| hCV25610857 | rs8176693 | hCV29393501 | rs4507838 | 0.51 | 0.148365232 | 0.4037 |
| hCV25610857 | rs8176693 | hCV29393505 | rs4962039 | 0.51 | 0.148365232 | 0.773 |
| hCV25610857 | rs8176693 | hCV29393508 | rs7046863 | 0.51 | 0.148365232 | 0.4262 |
| hCV25610857 | rs8176693 | hCV29531061 | rs9411464 | 0.51 | 0.148365232 | 0.6098 |
| hCV25610857 | rs8176693 | hCV29549191 | rs9411468 | 0.51 | 0.148365232 | 0.4483 |
| hCV25610857 | rs8176693 | hCV29597378 | rs8176672 | 0.51 | 0.148365232 | 1 |
| hCV25610857 | rs8176693 | hCV29711974 | rs7855466 | 0.51 | 0.148365232 | 0.4729 |
| hCV25610857 | rs8176693 | hCV2980259 | rs3761821 | 0.51 | 0.148365232 | 0.2969 |
| hCV25610857 | rs8176693 | hCV30504633 | rs9919007 | 0.51 | 0.148365232 | 0.4404 |
| hCV25610857 | rs8176693 | hCV30613004 | rs7855713 | 0.51 | 0.148365232 | 0.8222 |
| hCV25610857 | rs8176693 | hCV3183094 | rs8176731 | 0.51 | 0.148365232 | 0.1683 |
| hCV25610857 | rs8176693 | hCV3183096 | rs8176730 | 0.51 | 0.148365232 | 0.8013 |
| hCV25610857 | rs8176693 | hCV3183097 | rs8176725 | 0.51 | 0.148365232 | 0.802 |
| hCV25610857 | rs8176693 | hCV3183099 | rs8176722 | 0.51 | 0.148365232 | 0.8856 |
| hCV25610857 | rs8176693 | hCV3183100 | rs8176720 | 0.51 | 0.148365232 | 0.1485 |
| hCV25610857 | rs8176693 | hCV3183111 | rs643434 | 0.51 | 0.148365232 | 0.1634 |
| hCV25610857 | rs8176693 | hCV3183117 | rs545971 | 0.51 | 0.148365232 | 0.1765 |
| hCV25610857 | rs8176693 | hCV3183164 | rs529565 | 0.51 | 0.148365232 | 0.1765 |
| hCV25610857 | rs8176693 | hCV3183246 | rs10901263 | 0.51 | 0.148365232 | 0.2682 |
| hCV25610857 | rs8176693 | hCV3183251 | rs529309 | 0.51 | 0.148365232 | 0.1698 |
| hCV25610857 | rs8176693 | hCV3183341 | rs2285489 | 0.51 | 0.148365232 | 0.15 |
| hCV25610857 | rs8176693 | hCV3183366 | rs2073933 | 0.51 | 0.148365232 | 0.1731 |
| hCV25610857 | rs8176693 | hCV32126435 | rs11244034 | 0.51 | 0.148365232 | 0.3883 |
| hCV25610857 | rs8176693 | hCV32126442 | rs7864821 | 0.51 | 0.148365232 | 0.2969 |
| hCV25610857 | rs8176693 | hCV32126443 | rs10793957 | 0.51 | 0.148365232 | 0.2998 |

# TABLE 3, page 41 of 71

| Interrogated SNP | Interrogated rs | LD SNP | LD SNP rs | Power | Threshold r² | r² |
|---|---|---|---|---|---|---|
| hCV25610857 | rs8176693 | hCV32126447 | rs6597610 | 0.51 | 0.148365232 | 0.2969 |
| hCV25610857 | rs8176693 | hCV32126454 | rs13300535 | 0.51 | 0.148365232 | 0.2819 |
| hCV25610857 | rs8176693 | hCV32126487 | rs10901250 | 0.51 | 0.148365232 | 0.4799 |
| hCV25610857 | rs8176693 | hCV442675 | rs9411471 | 0.51 | 0.148365232 | 0.4262 |
| hCV25610857 | rs8176693 | hCV7481808 | rs886082 | 0.51 | 0.148365232 | 0.3072 |
| hCV25610857 | rs8176693 | hCV7948166 | rs9411463 | 0.51 | 0.148365232 | 0.8913 |
| hCV25610857 | rs8176693 | hCV7948171 | rs4246170 | 0.51 | 0.148365232 | 0.876 |
| hCV25610857 | rs8176693 | hCV8784837 | rs886090 | 0.51 | 0.148365232 | 0.1807 |
| hCV25610857 | rs8176693 | hCV9326428 | rs687289 | 0.51 | 0.148365232 | 0.1765 |
| hCV25610857 | rs8176693 | hCV9326429 | rs687621 | 0.51 | 0.148365232 | 0.1821 |
| hCV25610857 | rs8176693 | hCV9327931 | rs17150482 | 0.51 | 0.148365232 | 0.2489 |
| hCV25610857 | rs8176693 | hCV997908 | rs514659 | 0.51 | 0.148365232 | 0.175 |
| hCV25610857 | rs8176693 | hCV997918 | rs674302 | 0.51 | 0.148365232 | 0.1765 |
| hCV25610857 | rs8176693 | hCV998010 | rs493014 | 0.51 | 0.148365232 | 0.1498 |
| hCV25610857 | rs8176693 | hDV72329597 | rs28793911 | 0.51 | 0.148365232 | 0.1731 |
| hCV25620145 | rs867186 | hCV11189166 | rs11906318 | 0.51 | 0.329132176 | 1 |
| hCV25620145 | rs867186 | hCV11189205 | rs7261312 | 0.51 | 0.329132176 | 1 |
| hCV25620145 | rs867186 | hCV1271665 | rs17092456 | 0.51 | 0.329132176 | 1 |
| hCV25620145 | rs867186 | hCV1348012 | rs6060230 | 0.51 | 0.329132176 | 1 |
| hCV25620145 | rs867186 | hCV1348016 | rs6060246 | 0.51 | 0.329132176 | 1 |
| hCV25620145 | rs867186 | hCV1348030 | rs11908683 | 0.51 | 0.329132176 | 1 |
| hCV25620145 | rs867186 | hCV1348034 | rs2295888 | 0.51 | 0.329132176 | 1 |
| hCV25620145 | rs867186 | hCV16189181 | rs2295097 | 0.51 | 0.329132176 | 0.576 |
| hCV25620145 | rs867186 | hCV1825062 | rs6087685 | 0.51 | 0.329132176 | 0.3794 |
| hCV25620145 | rs867186 | hCV25472481 | rs2275274 | 0.51 | 0.329132176 | 0.5913 |
| hCV25620145 | rs867186 | hCV25952685 | rs17092297 | 0.51 | 0.329132176 | 1 |
| hCV25620145 | rs867186 | hCV27167646 | rs11699306 | 0.51 | 0.329132176 | 0.6217 |
| hCV25620145 | rs867186 | hCV27167730 | rs2889873 | 0.51 | 0.329132176 | 1 |
| hCV25620145 | rs867186 | hCV29372789 | rs7274866 | 0.51 | 0.329132176 | 1 |
| hCV25620145 | rs867186 | hCV29372790 | rs6579211 | 0.51 | 0.329132176 | 1 |
| hCV25620145 | rs867186 | hCV29372796 | rs7261167 | 0.51 | 0.329132176 | 1 |
| hCV25620145 | rs867186 | hCV29373046 | rs8119351 | 0.51 | 0.329132176 | 1 |
| hCV25620145 | rs867186 | hCV29373059 | rs8117847 | 0.51 | 0.329132176 | 1 |
| hCV25620145 | rs867186 | hCV29729417 | rs6060240 | 0.51 | 0.329132176 | 0.6395 |
| hCV25620145 | rs867186 | hCV29765455 | rs6058181 | 0.51 | 0.329132176 | 0.6863 |
| hCV25620145 | rs867186 | hCV30180149 | rs9941751 | 0.51 | 0.329132176 | 1 |
| hCV25620145 | rs867186 | hCV30270043 | rs6060257 | 0.51 | 0.329132176 | 0.8996 |
| hCV25620145 | rs867186 | hCV30342001 | rs10485508 | 0.51 | 0.329132176 | 1 |
| hCV25620145 | rs867186 | hCV30342056 | rs6060245 | 0.51 | 0.329132176 | 1 |
| hCV25620145 | rs867186 | hCV30342057 | rs6060239 | 0.51 | 0.329132176 | 0.6573 |
| hCV25620145 | rs867186 | hCV30540318 | rs6060244 | 0.51 | 0.329132176 | 1 |
| hCV25620145 | rs867186 | hCV30576442 | rs6120843 | 0.51 | 0.329132176 | 0.8996 |
| hCV25620145 | rs867186 | hCV32066118 | rs7271729 | 0.51 | 0.329132176 | 1 |
| hCV25620145 | rs867186 | hCV32066123 | rs7273734 | 0.51 | 0.329132176 | 1 |
| hCV25620145 | rs867186 | hCV32066133 | rs11906160 | 0.51 | 0.329132176 | 0.816 |
| hCV25620145 | rs867186 | hCV32066684 | rs11167260 | 0.51 | 0.329132176 | 1 |
| hCV25620145 | rs867186 | hCV32066690 | rs7265317 | 0.51 | 0.329132176 | 1 |
| hCV25620145 | rs867186 | hCV32066710 | rs11907010 | 0.51 | 0.329132176 | 1 |
| hCV25620145 | rs867186 | hCV32066768 | rs7263253 | 0.51 | 0.329132176 | 1 |
| hCV25620145 | rs867186 | hCV624499 | rs717593 | 0.51 | 0.329132176 | 1 |
| hCV25620145 | rs867186 | hCV7593265 | rs1033799 | 0.51 | 0.329132176 | 0.8996 |
| hCV25620145 | rs867186 | hCV7593267 | rs1033797 | 0.51 | 0.329132176 | 1 |
| hCV25620145 | rs867186 | hDV70862590 | rs17092215 | 0.51 | 0.329132176 | 1 |
| hCV25620145 | rs867186 | hDV70936222 | rs17309872 | 0.51 | 0.329132176 | 0.5774 |
| hCV25620145 | rs867186 | hDV70936327 | rs17310467 | 0.51 | 0.329132176 | 0.9424 |

# TABLE 3, page 42 of 71

| Interrogated SNP | Interrogated rs | LD SNP | LD SNP rs | Power | Threshold r² | r² |
|---|---|---|---|---|---|---|
| hCV25620145 | rs867186 | hDV70948869 | rs17401737 | 0.51 | 0.329132176 | 1 |
| hCV25620145 | rs867186 | hDV70949473 | rs17406518 | 0.51 | 0.329132176 | 0.709 |
| hCV25620145 | rs867186 | hDV72054460 | rs8117100 | 0.51 | 0.329132176 | 1 |
| hCV25620145 | rs867186 | hDV75209987 | rs2069940 | 0.51 | 0.329132176 | 0.9474 |
| hCV25748719 | hCV25748719 | hCV11828144 | rs11679975 | 0.51 | 0.912579748 | 1 |
| hCV25748719 | hCV25748719 | hCV11828147 | rs10496693 | 0.51 | 0.912579748 | 0.9529 |
| hCV25748719 | hCV25748719 | hCV2163177 | rs12618525 | 0.51 | 0.912579748 | 0.9518 |
| hCV25748719 | hCV25748719 | hCV25749343 | rs16841277 | 0.51 | 0.912579748 | 1 |
| hCV25748719 | hCV25748719 | hCV3212676 | rs4143562 | 0.51 | 0.912579748 | 1 |
| hCV25748719 | hCV25748719 | hCV7479717 | rs1367392 | 0.51 | 0.912579748 | 1 |
| hCV25748719 | hCV25748719 | hDV70679361 | rs16841210 | 0.51 | 0.912579748 | 1 |
| hCV25990131 | rs13146272 | hCV11786147 | rs4862662 | 0.51 | 0.143358157 | 0.4248 |
| hCV25990131 | rs13146272 | hCV11786258 | rs4253303 | 0.51 | 0.143358157 | 0.3213 |
| hCV25990131 | rs13146272 | hCV11786259 | rs4253304 | 0.51 | 0.143358157 | 0.2413 |
| hCV25990131 | rs13146272 | hCV12066116 | rs1877320 | 0.51 | 0.143358157 | 0.1908 |
| hCV25990131 | rs13146272 | hCV12066118 | rs2048 | 0.51 | 0.143358157 | 0.1915 |
| hCV25990131 | rs13146272 | hCV12066119 | rs1912826 | 0.51 | 0.143358157 | 0.1697 |
| hCV25990131 | rs13146272 | hCV12066124 | rs2036914 | 0.51 | 0.143358157 | 0.1776 |
| hCV25990131 | rs13146272 | hCV12066129 | rs1593 | 0.51 | 0.143358157 | 0.1539 |
| hCV25990131 | rs13146272 | hCV15793897 | rs3087505 | 0.51 | 0.143358157 | 0.1528 |
| hCV25990131 | rs13146272 | hCV15811716 | rs2102575 | 0.51 | 0.143358157 | 0.144 |
| hCV25990131 | rs13146272 | hCV15882886 | rs2276916 | 0.51 | 0.143358157 | 0.1604 |
| hCV25990131 | rs13146272 | hCV15968025 | rs2292425 | 0.51 | 0.143358157 | 1 |
| hCV25990131 | rs13146272 | hCV15968026 | rs2292426 | 0.51 | 0.143358157 | 0.8904 |
| hCV25990131 | rs13146272 | hCV15968043 | rs2292423 | 0.51 | 0.143358157 | 0.2261 |
| hCV25990131 | rs13146272 | hCV15975109 | rs2304596 | 0.51 | 0.143358157 | 0.3895 |
| hCV25990131 | rs13146272 | hCV2103343 | rs4241824 | 0.51 | 0.143358157 | 0.1563 |
| hCV25990131 | rs13146272 | hCV2103392 | rs12500826 | 0.51 | 0.143358157 | 0.1597 |
| hCV25990131 | rs13146272 | hCV22272267 | rs3733402 | 0.51 | 0.143358157 | 0.1915 |
| hCV25990131 | rs13146272 | hCV25474413 | rs3822057 | 0.51 | 0.143358157 | 0.1706 |
| hCV25990131 | rs13146272 | hCV25988221 | rs9995366 | 0.51 | 0.143358157 | 0.1617 |
| hCV25990131 | rs13146272 | hCV25989001 | hCV25989001 | 0.51 | 0.143358157 | 0.3491 |
| hCV25990131 | rs13146272 | hCV26265231 | rs7684025 | 0.51 | 0.143358157 | 0.3297 |
| hCV25990131 | rs13146272 | hCV27477533 | rs3756008 | 0.51 | 0.143358157 | 0.1634 |
| hCV25990131 | rs13146272 | hCV27482765 | rs3775301 | 0.51 | 0.143358157 | 0.3895 |
| hCV25990131 | rs13146272 | hCV27902803 | rs4862665 | 0.51 | 0.143358157 | 0.1617 |
| hCV25990131 | rs13146272 | hCV27902808 | rs4253236 | 0.51 | 0.143358157 | 0.3974 |
| hCV25990131 | rs13146272 | hCV28960679 | rs6844764 | 0.51 | 0.143358157 | 0.1752 |
| hCV25990131 | rs13146272 | hCV29053261 | rs6842047 | 0.51 | 0.143358157 | 0.1528 |
| hCV25990131 | rs13146272 | hCV29053264 | rs7667777 | 0.51 | 0.143358157 | 0.4279 |
| hCV25990131 | rs13146272 | hCV29053265 | rs4253244 | 0.51 | 0.143358157 | 0.3445 |
| hCV25990131 | rs13146272 | hCV29053266 | rs7687961 | 0.51 | 0.143358157 | 0.2979 |
| hCV25990131 | rs13146272 | hCV29718000 | rs4253238 | 0.51 | 0.143358157 | 0.2008 |
| hCV25990131 | rs13146272 | hCV29826351 | rs10025990 | 0.51 | 0.143358157 | 0.1633 |
| hCV25990131 | rs13146272 | hCV29877725 | rs4253295 | 0.51 | 0.143358157 | 0.3626 |
| hCV25990131 | rs13146272 | hCV30492573 | rs10471184 | 0.51 | 0.143358157 | 0.1528 |
| hCV25990131 | rs13146272 | hCV30983902 | rs4862668 | 0.51 | 0.143358157 | 0.1908 |
| hCV25990131 | rs13146272 | hCV30983927 | rs6552962 | 0.51 | 0.143358157 | 0.3677 |
| hCV25990131 | rs13146272 | hCV32209815 | rs7660915 | 0.51 | 0.143358157 | 0.1573 |
| hCV25990131 | rs13146272 | hCV32291217 | rs4253323 | 0.51 | 0.143358157 | 0.3895 |
| hCV25990131 | rs13146272 | hCV32291295 | rs4253292 | 0.51 | 0.143358157 | 0.4332 |
| hCV25990131 | rs13146272 | hCV32291301 | rs4253302 | 0.51 | 0.143358157 | 0.3944 |
| hCV25990131 | rs13146272 | hCV32295028 | rs4253260 | 0.51 | 0.143358157 | 0.3895 |
| hCV25990131 | rs13146272 | hCV3229991 | rs4241815 | 0.51 | 0.143358157 | 0.1915 |
| hCV25990131 | rs13146272 | hCV3229992 | rs3775298 | 0.51 | 0.143358157 | 0.1915 |

# TABLE 3, page 43 of 71

| Interrogated SNP | Interrogated rs | LD SNP | LD SNP rs | Power | Threshold $r^2$ | $r^2$ |
|---|---|---|---|---|---|---|
| hCV25990131 | rs13146272 | hCV3229995 | rs11132382 | 0.51 | 0.143358157 | 0.1909 |
| hCV25990131 | rs13146272 | hCV3230002 | rs4253297 | 0.51 | 0.143358157 | 0.3314 |
| hCV25990131 | rs13146272 | hCV3230003 | rs2304595 | 0.51 | 0.143358157 | 0.2642 |
| hCV25990131 | rs13146272 | hCV3230006 | rs4253308 | 0.51 | 0.143358157 | 0.3626 |
| hCV25990131 | rs13146272 | hCV3230007 | rs4253311 | 0.51 | 0.143358157 | 0.1915 |
| hCV25990131 | rs13146272 | hCV3230011 | rs4253320 | 0.51 | 0.143358157 | 0.3314 |
| hCV25990131 | rs13146272 | hCV3230013 | rs3775303 | 0.51 | 0.143358157 | 0.2413 |
| hCV25990131 | rs13146272 | hCV3230025 | rs3756009 | 0.51 | 0.143358157 | 0.2436 |
| hCV25990131 | rs13146272 | hCV3230083 | rs10013653 | 0.51 | 0.143358157 | 0.3137 |
| hCV25990131 | rs13146272 | hCV3230084 | rs7682918 | 0.51 | 0.143358157 | 0.4336 |
| hCV25990131 | rs13146272 | hCV3230094 | rs7687818 | 0.51 | 0.143358157 | 0.3187 |
| hCV25990131 | rs13146272 | hCV3230096 | rs3817184 | 0.51 | 0.143358157 | 0.4248 |
| hCV25990131 | rs13146272 | hCV3230097 | rs3736455 | 0.51 | 0.143358157 | 0.8444 |
| hCV25990131 | rs13146272 | hCV3230113 | rs1053094 | 0.51 | 0.143358157 | 0.1593 |
| hCV25990131 | rs13146272 | hCV32313006 | rs4253248 | 0.51 | 0.143358157 | 0.1954 |
| hCV25990131 | rs13146272 | hCV32313007 | rs4862666 | 0.51 | 0.143358157 | 0.1617 |
| hCV25990131 | rs13146272 | hCV32313024 | rs4253239 | 0.51 | 0.143358157 | 0.4332 |
| hCV25990131 | rs13146272 | hCV32358975 | rs4253255 | 0.51 | 0.143358157 | 0.1791 |
| hCV25990131 | rs13146272 | hCV32358984 | rs4253256 | 0.51 | 0.143358157 | 0.3717 |
| hCV25990131 | rs13146272 | hCV8241630 | rs925451 | 0.51 | 0.143358157 | 0.1564 |
| hCV25990131 | rs13146272 | hCV8241631 | rs1511802 | 0.51 | 0.143358157 | 0.3665 |
| hCV25990131 | rs13146272 | hCV8241632 | rs1511801 | 0.51 | 0.143358157 | 0.193 |
| hCV25990131 | rs13146272 | hCV8241633 | rs1511800 | 0.51 | 0.143358157 | 0.1617 |
| hCV25990131 | rs13146272 | hDV71222711 | rs4253252 | 0.51 | 0.143358157 | 0.1954 |
| hCV25990131 | rs13146272 | hDV76175111 | rs35079309 | 0.51 | 0.143358157 | 0.1697 |
| hCV263841 | rs1523127 | hCV105917 | rs9289134 | 0.51 | 0.401557164 | 0.4905 |
| hCV263841 | rs1523127 | hCV11230788 | rs7643038 | 0.51 | 0.401557164 | 1 |
| hCV263841 | rs1523127 | hCV134275 | rs9847068 | 0.51 | 0.401557164 | 0.4525 |
| hCV263841 | rs1523127 | hCV134278 | rs9848716 | 0.51 | 0.401557164 | 0.4627 |
| hCV263841 | rs1523127 | hCV15882316 | rs2276706 | 0.51 | 0.401557164 | 1 |
| hCV263841 | rs1523127 | hCV178227 | rs13070374 | 0.51 | 0.401557164 | 0.5348 |
| hCV263841 | rs1523127 | hCV178228 | rs4687882 | 0.51 | 0.401557164 | 0.5054 |
| hCV263841 | rs1523127 | hCV1833991 | rs11926554 | 0.51 | 0.401557164 | 0.5461 |
| hCV263841 | rs1523127 | hCV1834237 | rs9865270 | 0.51 | 0.401557164 | 0.4905 |
| hCV263841 | rs1523127 | hCV1834240 | rs1581451 | 0.51 | 0.401557164 | 1 |
| hCV263841 | rs1523127 | hCV1834242 | rs11712308 | 0.51 | 0.401557164 | 0.4291 |
| hCV263841 | rs1523127 | hCV1834243 | rs9682652 | 0.51 | 0.401557164 | 0.5871 |
| hCV263841 | rs1523127 | hCV1834252 | rs10934498 | 0.51 | 0.401557164 | 0.9212 |
| hCV263841 | rs1523127 | hCV1834256 | rs2472662 | 0.51 | 0.401557164 | 0.4402 |
| hCV263841 | rs1523127 | hCV1834260 | rs4688033 | 0.51 | 0.401557164 | 0.5676 |
| hCV263841 | rs1523127 | hCV192027 | rs9821892 | 0.51 | 0.401557164 | 0.5871 |
| hCV263841 | rs1523127 | hCV255886 | rs10511394 | 0.51 | 0.401557164 | 0.569 |
| hCV263841 | rs1523127 | hCV27504984 | rs3814055 | 0.51 | 0.401557164 | 1 |
| hCV263841 | rs1523127 | hCV278948 | rs1464599 | 0.51 | 0.401557164 | 0.569 |
| hCV263841 | rs1523127 | hCV29841665 | rs7623217 | 0.51 | 0.401557164 | 0.5802 |
| hCV263841 | rs1523127 | hCV30562884 | rs9815093 | 0.51 | 0.401557164 | 0.4905 |
| hCV263841 | rs1523127 | hCV30699687 | rs11711386 | 0.51 | 0.401557164 | 0.4402 |
| hCV263841 | rs1523127 | hCV30747432 | rs12488820 | 0.51 | 0.401557164 | 0.9212 |
| hCV263841 | rs1523127 | hCV9152783 | rs1523130 | 0.51 | 0.401557164 | 0.9003 |
| hCV27474895 | rs3756011 | hCV11786147 | rs4862662 | 0.51 | 0.046522553 | 0.1651 |
| hCV27474895 | rs3756011 | hCV11786235 | rs4253287 | 0.51 | 0.046522553 | 0.096 |
| hCV27474895 | rs3756011 | hCV11786258 | rs4253303 | 0.51 | 0.046522553 | 0.1518 |
| hCV27474895 | rs3756011 | hCV11786259 | rs4253304 | 0.51 | 0.046522553 | 0.2126 |
| hCV27474895 | rs3756011 | hCV11786295 | rs4253421 | 0.51 | 0.046522553 | 0.0964 |
| hCV27474895 | rs3756011 | hCV11786301 | rs5970 | 0.51 | 0.046522553 | 0.0964 |

## TABLE 3, page 44 of 71

| Interrogated SNP | Interrogated rs | LD SNP | LD SNP rs | Power | Threshold r² | r² |
|---|---|---|---|---|---|---|
| hCV27474895 | rs3756011 | hCV11786307 | rs1062547 | 0.51 | 0.046522553 | 0.3474 |
| hCV27474895 | rs3756011 | hCV11786311 | rs13145616 | 0.51 | 0.046522553 | 0.0964 |
| hCV27474895 | rs3756011 | hCV11786327 | rs13133050 | 0.51 | 0.046522553 | 0.2195 |
| hCV27474895 | rs3756011 | hCV12066116 | rs1877320 | 0.51 | 0.046522553 | 0.0698 |
| hCV27474895 | rs3756011 | hCV12066118 | rs2048 | 0.51 | 0.046522553 | 0.0958 |
| hCV27474895 | rs3756011 | hCV12066119 | rs1912826 | 0.51 | 0.046522553 | 0.1115 |
| hCV27474895 | rs3756011 | hCV12066124 | rs2036914 | 0.51 | 0.046522553 | 0.4851 |
| hCV27474895 | rs3756011 | hCV12066129 | rs1593 | 0.51 | 0.046522553 | 0.0705 |
| hCV27474895 | rs3756011 | hCV1333076 | rs7656944 | 0.51 | 0.046522553 | 0.0488 |
| hCV27474895 | rs3756011 | hCV1333077 | rs7656763 | 0.51 | 0.046522553 | 0.0488 |
| hCV27474895 | rs3756011 | hCV1333078 | rs9998003 | 0.51 | 0.046522553 | 0.0625 |
| hCV27474895 | rs3756011 | hCV1333102 | rs10016252 | 0.51 | 0.046522553 | 0.0488 |
| hCV27474895 | rs3756011 | hCV15793897 | rs3087505 | 0.51 | 0.046522553 | 0.0748 |
| hCV27474895 | rs3756011 | hCV15811716 | rs2102575 | 0.51 | 0.046522553 | 0.0679 |
| hCV27474895 | rs3756011 | hCV15880920 | rs2289253 | 0.51 | 0.046522553 | 0.0479 |
| hCV27474895 | rs3756011 | hCV15968025 | rs2292425 | 0.51 | 0.046522553 | 0.138 |
| hCV27474895 | rs3756011 | hCV15968026 | rs2292426 | 0.51 | 0.046522553 | 0.1681 |
| hCV27474895 | rs3756011 | hCV15968043 | rs2292423 | 0.51 | 0.046522553 | 0.1967 |
| hCV27474895 | rs3756011 | hCV16172925 | rs2241818 | 0.51 | 0.046522553 | 0.095 |
| hCV27474895 | rs3756011 | hCV16172935 | rs2241817 | 0.51 | 0.046522553 | 0.3529 |
| hCV27474895 | rs3756011 | hCV194962 | rs6552954 | 0.51 | 0.046522553 | 0.0471 |
| hCV27474895 | rs3756011 | hCV2103343 | rs4241824 | 0.51 | 0.046522553 | 0.5177 |
| hCV27474895 | rs3756011 | hCV2103388 | rs4613610 | 0.51 | 0.046522553 | 0.1053 |
| hCV27474895 | rs3756011 | hCV2103391 | rs1008728 | 0.51 | 0.046522553 | 0.3064 |
| hCV27474895 | rs3756011 | hCV2103392 | rs12500826 | 0.51 | 0.046522553 | 0.286 |
| hCV27474895 | rs3756011 | hCV22272267 | rs3733402 | 0.51 | 0.046522553 | 0.0893 |
| hCV27474895 | rs3756011 | hCV25474413 | rs3822057 | 0.51 | 0.046522553 | 0.5232 |
| hCV27474895 | rs3756011 | hCV25474414 | rs4253399 | 0.51 | 0.046522553 | 0.7565 |
| hCV27474895 | rs3756011 | hCV25634754 | rs4253331 | 0.51 | 0.046522553 | 0.0475 |
| hCV27474895 | rs3756011 | hCV25988221 | rs9995366 | 0.51 | 0.046522553 | 0.0748 |
| hCV27474895 | rs3756011 | hCV25990131 | rs13146272 | 0.51 | 0.046522553 | 0.1401 |
| hCV27474895 | rs3756011 | hCV26038139 | rs4253405 | 0.51 | 0.046522553 | 0.2975 |
| hCV27474895 | rs3756011 | hCV26265231 | rs7684025 | 0.51 | 0.046522553 | 0.2227 |
| hCV27474895 | rs3756011 | hCV27309991 | rs4572916 | 0.51 | 0.046522553 | 0.1096 |
| hCV27474895 | rs3756011 | hCV27473099 | rs3733403 | 0.51 | 0.046522553 | 0.0591 |
| hCV27474895 | rs3756011 | hCV27477533 | rs3756008 | 0.51 | 0.046522553 | 0.7565 |
| hCV27474895 | rs3756011 | hCV27490984 | rs3822058 | 0.51 | 0.046522553 | 0.3739 |
| hCV27474895 | rs3756011 | hCV27902803 | rs4862665 | 0.51 | 0.046522553 | 0.0748 |
| hCV27474895 | rs3756011 | hCV27902808 | rs4253236 | 0.51 | 0.046522553 | 0.0489 |
| hCV27474895 | rs3756011 | hCV28960679 | rs6844764 | 0.51 | 0.046522553 | 0.1341 |
| hCV27474895 | rs3756011 | hCV29053261 | rs6842047 | 0.51 | 0.046522553 | 0.0748 |
| hCV27474895 | rs3756011 | hCV29053264 | rs7667777 | 0.51 | 0.046522553 | 0.1527 |
| hCV27474895 | rs3756011 | hCV29053266 | rs7687961 | 0.51 | 0.046522553 | 0.0784 |
| hCV27474895 | rs3756011 | hCV29640635 | rs10029715 | 0.51 | 0.046522553 | 0.1232 |
| hCV27474895 | rs3756011 | hCV29718000 | rs4253238 | 0.51 | 0.046522553 | 0.1192 |
| hCV27474895 | rs3756011 | hCV29826351 | rs10025990 | 0.51 | 0.046522553 | 0.078 |
| hCV27474895 | rs3756011 | hCV29877725 | rs4253295 | 0.51 | 0.046522553 | 0.2015 |
| hCV27474895 | rs3756011 | hCV30307525 | rs10025152 | 0.51 | 0.046522553 | 0.1232 |
| hCV27474895 | rs3756011 | hCV30492573 | rs10471184 | 0.51 | 0.046522553 | 0.0748 |
| hCV27474895 | rs3756011 | hCV30562347 | rs4253418 | 0.51 | 0.046522553 | 0.0479 |
| hCV27474895 | rs3756011 | hCV30983902 | rs4862668 | 0.51 | 0.046522553 | 0.0667 |
| hCV27474895 | rs3756011 | hCV30983927 | rs6552962 | 0.51 | 0.046522553 | 0.0811 |
| hCV27474895 | rs3756011 | hCV32209629 | rs12715865 | 0.51 | 0.046522553 | 0.1297 |
| hCV27474895 | rs3756011 | hCV32209635 | rs6848311 | 0.51 | 0.046522553 | 0.1065 |
| hCV27474895 | rs3756011 | hCV32209636 | rs11132387 | 0.51 | 0.046522553 | 0.44 |

# TABLE 3, page 45 of 71

| Interrogated SNP | Interrogated rs | LD SNP | LD SNP rs | Power | Threshold r² | r² |
|---|---|---|---|---|---|---|
| hCV27474895 | rs3756011 | hCV32209637 | rs13143773 | 0.51 | 0.046522553 | 0.2103 |
| hCV27474895 | rs3756011 | hCV32209638 | rs12507040 | 0.51 | 0.046522553 | 0.2952 |
| hCV27474895 | rs3756011 | hCV32291256 | rs4253406 | 0.51 | 0.046522553 | 0.0719 |
| hCV27474895 | rs3756011 | hCV32291269 | rs4253417 | 0.51 | 0.046522553 | 0.9279 |
| hCV27474895 | rs3756011 | hCV32291286 | rs4253422 | 0.51 | 0.046522553 | 0.1355 |
| hCV27474895 | rs3756011 | hCV32291287 | rs4253423 | 0.51 | 0.046522553 | 0.1355 |
| hCV27474895 | rs3756011 | hCV3229991 | rs4241815 | 0.51 | 0.046522553 | 0.0893 |
| hCV27474895 | rs3756011 | hCV3229992 | rs3775298 | 0.51 | 0.046522553 | 0.0893 |
| hCV27474895 | rs3756011 | hCV3229995 | rs11132382 | 0.51 | 0.046522553 | 0.1192 |
| hCV27474895 | rs3756011 | hCV3230002 | rs4253297 | 0.51 | 0.046522553 | 0.1602 |
| hCV27474895 | rs3756011 | hCV3230003 | rs2304595 | 0.51 | 0.046522553 | 0.2636 |
| hCV27474895 | rs3756011 | hCV3230006 | rs4253308 | 0.51 | 0.046522553 | 0.2015 |
| hCV27474895 | rs3756011 | hCV3230007 | rs4253311 | 0.51 | 0.046522553 | 0.0893 |
| hCV27474895 | rs3756011 | hCV3230010 | rs4253315 | 0.51 | 0.046522553 | 0.0729 |
| hCV27474895 | rs3756011 | hCV3230011 | rs4253320 | 0.51 | 0.046522553 | 0.1602 |
| hCV27474895 | rs3756011 | hCV3230013 | rs3775303 | 0.51 | 0.046522553 | 0.2126 |
| hCV27474895 | rs3756011 | hCV3230016 | rs4253325 | 0.51 | 0.046522553 | 0.0964 |
| hCV27474895 | rs3756011 | hCV3230017 | rs4253327 | 0.51 | 0.046522553 | 0.0647 |
| hCV27474895 | rs3756011 | hCV3230021 | rs13135645 | 0.51 | 0.046522553 | 0.0602 |
| hCV27474895 | rs3756011 | hCV3230022 | rs11132383 | 0.51 | 0.046522553 | 0.1964 |
| hCV27474895 | rs3756011 | hCV3230025 | rs3756009 | 0.51 | 0.046522553 | 0.8008 |
| hCV27474895 | rs3756011 | hCV3230030 | rs4253408 | 0.51 | 0.046522553 | 0.0889 |
| hCV27474895 | rs3756011 | hCV3230031 | rs4253419 | 0.51 | 0.046522553 | 0.1355 |
| hCV27474895 | rs3756011 | hCV3230032 | rs5974 | 0.51 | 0.046522553 | 0.0964 |
| hCV27474895 | rs3756011 | hCV3230038 | rs2289252 | 0.51 | 0.046522553 | 1 |
| hCV27474895 | rs3756011 | hCV3230083 | rs10013653 | 0.51 | 0.046522553 | 0.2455 |
| hCV27474895 | rs3756011 | hCV3230084 | rs7682918 | 0.51 | 0.046522553 | 0.1899 |
| hCV27474895 | rs3756011 | hCV3230094 | rs7687818 | 0.51 | 0.046522553 | 0.2475 |
| hCV27474895 | rs3756011 | hCV3230096 | rs3817184 | 0.51 | 0.046522553 | 0.1852 |
| hCV27474895 | rs3756011 | hCV3230097 | rs3736455 | 0.51 | 0.046522553 | 0.181 |
| hCV27474895 | rs3756011 | hCV3230113 | rs1053094 | 0.51 | 0.046522553 | 0.1126 |
| hCV27474895 | rs3756011 | hCV3230118 | rs4253429 | 0.51 | 0.046522553 | 0.1355 |
| hCV27474895 | rs3756011 | hCV3230119 | rs4253430 | 0.51 | 0.046522553 | 0.3739 |
| hCV27474895 | rs3756011 | hCV3230121 | rs4253431 | 0.51 | 0.046522553 | 0.0964 |
| hCV27474895 | rs3756011 | hCV3230125 | rs11938564 | 0.51 | 0.046522553 | 0.198 |
| hCV27474895 | rs3756011 | hCV3230131 | rs13136269 | 0.51 | 0.046522553 | 0.2952 |
| hCV27474895 | rs3756011 | hCV3230133 | rs12511874 | 0.51 | 0.046522553 | 0.2952 |
| hCV27474895 | rs3756011 | hCV3230134 | rs12500151 | 0.51 | 0.046522553 | 0.2952 |
| hCV27474895 | rs3756011 | hCV3230136 | rs13116273 | 0.51 | 0.046522553 | 0.2675 |
| hCV27474895 | rs3756011 | hCV32313006 | rs4253248 | 0.51 | 0.046522553 | 0.1192 |
| hCV27474895 | rs3756011 | hCV32313007 | rs4862666 | 0.51 | 0.046522553 | 0.0748 |
| hCV27474895 | rs3756011 | hCV32313014 | rs4253243 | 0.51 | 0.046522553 | 0.0475 |
| hCV27474895 | rs3756011 | hCV32358975 | rs4253255 | 0.51 | 0.046522553 | 0.0845 |
| hCV27474895 | rs3756011 | hCV8241628 | rs907439 | 0.51 | 0.046522553 | 0.1096 |
| hCV27474895 | rs3756011 | hCV8241630 | rs925451 | 0.51 | 0.046522553 | 0.7876 |
| hCV27474895 | rs3756011 | hCV8241631 | rs1511802 | 0.51 | 0.046522553 | 0.2015 |
| hCV27474895 | rs3756011 | hCV8241632 | rs1511801 | 0.51 | 0.046522553 | 0.083 |
| hCV27474895 | rs3756011 | hCV8241633 | rs1511800 | 0.51 | 0.046522553 | 0.0748 |
| hCV27474895 | rs3756011 | hCV8241668 | rs1401570 | 0.51 | 0.046522553 | 0.0565 |
| hCV27474895 | rs3756011 | hDV68550952 | rs4253289 | 0.51 | 0.046522553 | 0.0624 |
| hCV27474895 | rs3756011 | hDV71222711 | rs4253252 | 0.51 | 0.046522553 | 0.1192 |
| hCV27474984 | rs3756668 | hCV11824867 | rs256507 | 0.51 | 0.529595036 | 0.7675 |
| hCV27474984 | rs3756668 | hCV1988068 | rs1445760 | 0.51 | 0.529595036 | 0.7246 |
| hCV27474984 | rs3756668 | hCV3164046 | rs9291926 | 0.51 | 0.529595036 | 0.8067 |
| hCV27474984 | rs3756668 | hCV3164066 | rs34292 | 0.51 | 0.529595036 | 0.7511 |

# TABLE 3, page 46 of 71

| Interrogated SNP | Interrogated rs | LD SNP | LD SNP rs | Power | Threshold r² | r² |
|---|---|---|---|---|---|---|
| hCV27474984 | rs3756668 | hCV977979 | rs256508 | 0.51 | 0.529595036 | 0.751 |
| hCV27477533 | rs3756008 | hCV11786147 | rs4862662 | 0.51 | 0.052089996 | 0.2067 |
| hCV27477533 | rs3756008 | hCV11786235 | rs4253287 | 0.51 | 0.052089996 | 0.09 |
| hCV27477533 | rs3756008 | hCV11786258 | rs4253303 | 0.51 | 0.052089996 | 0.315 |
| hCV27477533 | rs3756008 | hCV11786259 | rs4253304 | 0.51 | 0.052089996 | 0.3791 |
| hCV27477533 | rs3756008 | hCV11786295 | rs4253421 | 0.51 | 0.052089996 | 0.0524 |
| hCV27477533 | rs3756008 | hCV11786307 | rs1062547 | 0.51 | 0.052089996 | 0.273 |
| hCV27477533 | rs3756008 | hCV11786327 | rs13133050 | 0.51 | 0.052089996 | 0.1451 |
| hCV27477533 | rs3756008 | hCV12066116 | rs1877320 | 0.51 | 0.052089996 | 0.0836 |
| hCV27477533 | rs3756008 | hCV12066118 | rs2048 | 0.51 | 0.052089996 | 0.1524 |
| hCV27477533 | rs3756008 | hCV12066119 | rs1912826 | 0.51 | 0.052089996 | 0.1565 |
| hCV27477533 | rs3756008 | hCV12066124 | rs2036914 | 0.51 | 0.052089996 | 0.5443 |
| hCV27477533 | rs3756008 | hCV12066129 | rs1593 | 0.51 | 0.052089996 | 0.0893 |
| hCV27477533 | rs3756008 | hCV1333090 | rs6816112 | 0.51 | 0.052089996 | 0.0736 |
| hCV27477533 | rs3756008 | hCV1333099 | rs10020635 | 0.51 | 0.052089996 | 0.0654 |
| hCV27477533 | rs3756008 | hCV15793897 | rs3087505 | 0.51 | 0.052089996 | 0.0633 |
| hCV27477533 | rs3756008 | hCV15811716 | rs2102575 | 0.51 | 0.052089996 | 0.0597 |
| hCV27477533 | rs3756008 | hCV15968025 | rs2292425 | 0.51 | 0.052089996 | 0.156 |
| hCV27477533 | rs3756008 | hCV15968026 | rs2292426 | 0.51 | 0.052089996 | 0.2168 |
| hCV27477533 | rs3756008 | hCV15968034 | rs2292428 | 0.51 | 0.052089996 | 0.1109 |
| hCV27477533 | rs3756008 | hCV15968043 | rs2292423 | 0.51 | 0.052089996 | 0.3624 |
| hCV27477533 | rs3756008 | hCV16172925 | rs2241818 | 0.51 | 0.052089996 | 0.0782 |
| hCV27477533 | rs3756008 | hCV16172935 | rs2241817 | 0.51 | 0.052089996 | 0.2699 |
| hCV27477533 | rs3756008 | hCV2103343 | rs4241824 | 0.51 | 0.052089996 | 0.5858 |
| hCV27477533 | rs3756008 | hCV2103348 | rs11931515 | 0.51 | 0.052089996 | 0.0563 |
| hCV27477533 | rs3756008 | hCV2103388 | rs4613610 | 0.51 | 0.052089996 | 0.0953 |
| hCV27477533 | rs3756008 | hCV2103391 | rs1008728 | 0.51 | 0.052089996 | 0.1778 |
| hCV27477533 | rs3756008 | hCV2103392 | rs12500826 | 0.51 | 0.052089996 | 0.3498 |
| hCV27477533 | rs3756008 | hCV22272267 | rs3733402 | 0.51 | 0.052089996 | 0.1577 |
| hCV27477533 | rs3756008 | hCV25474413 | rs3822057 | 0.51 | 0.052089996 | 0.577 |
| hCV27477533 | rs3756008 | hCV25474414 | rs4253399 | 0.51 | 0.052089996 | 0.9414 |
| hCV27477533 | rs3756008 | hCV25634754 | rs4253331 | 0.51 | 0.052089996 | 0.1183 |
| hCV27477533 | rs3756008 | hCV25988221 | rs9995366 | 0.51 | 0.052089996 | 0.067 |
| hCV27477533 | rs3756008 | hCV25990131 | rs13146272 | 0.51 | 0.052089996 | 0.1634 |
| hCV27477533 | rs3756008 | hCV26038139 | rs4253405 | 0.51 | 0.052089996 | 0.3634 |
| hCV27477533 | rs3756008 | hCV26265231 | rs7684025 | 0.51 | 0.052089996 | 0.2597 |
| hCV27477533 | rs3756008 | hCV27309972 | rs13101296 | 0.51 | 0.052089996 | 0.1249 |
| hCV27477533 | rs3756008 | hCV27309991 | rs4572916 | 0.51 | 0.052089996 | 0.1028 |
| hCV27477533 | rs3756008 | hCV27473099 | rs3733403 | 0.51 | 0.052089996 | 0.081 |
| hCV27477533 | rs3756008 | hCV27474895 | rs3756011 | 0.51 | 0.052089996 | 0.7565 |
| hCV27477533 | rs3756008 | hCV27490984 | rs3822058 | 0.51 | 0.052089996 | 0.2804 |
| hCV27477533 | rs3756008 | hCV27521729 | rs3822056 | 0.51 | 0.052089996 | 0.0979 |
| hCV27477533 | rs3756008 | hCV27902803 | rs4862665 | 0.51 | 0.052089996 | 0.067 |
| hCV27477533 | rs3756008 | hCV27902808 | rs4253236 | 0.51 | 0.052089996 | 0.0524 |
| hCV27477533 | rs3756008 | hCV28960679 | rs6844764 | 0.51 | 0.052089996 | 0.1972 |
| hCV27477533 | rs3756008 | hCV29053261 | rs6842047 | 0.51 | 0.052089996 | 0.0633 |
| hCV27477533 | rs3756008 | hCV29053264 | rs7667777 | 0.51 | 0.052089996 | 0.2846 |
| hCV27477533 | rs3756008 | hCV29053265 | rs4253244 | 0.51 | 0.052089996 | 0.0589 |
| hCV27477533 | rs3756008 | hCV29718000 | rs4253238 | 0.51 | 0.052089996 | 0.1722 |
| hCV27477533 | rs3756008 | hCV29826351 | rs10025990 | 0.51 | 0.052089996 | 0.0992 |
| hCV27477533 | rs3756008 | hCV29877725 | rs4253295 | 0.51 | 0.052089996 | 0.248 |
| hCV27477533 | rs3756008 | hCV30492573 | rs10471184 | 0.51 | 0.052089996 | 0.0633 |
| hCV27477533 | rs3756008 | hCV30983902 | rs4862668 | 0.51 | 0.052089996 | 0.0836 |
| hCV27477533 | rs3756008 | hCV30983927 | rs6552962 | 0.51 | 0.052089996 | 0.0937 |
| hCV27477533 | rs3756008 | hCV32209629 | rs12715865 | 0.51 | 0.052089996 | 0.1094 |

# TABLE 3, page 47 of 71

| Interrogated SNP | Interrogated rs | LD SNP | LD SNP rs | Power | Threshold r$^2$ | r$^2$ |
|---|---|---|---|---|---|---|
| hCV27477533 | rs3756008 | hCV32209636 | rs11132387 | 0.51 | 0.052089996 | 0.3591 |
| hCV27477533 | rs3756008 | hCV32209637 | rs13143773 | 0.51 | 0.052089996 | 0.1889 |
| hCV27477533 | rs3756008 | hCV32209638 | rs12507040 | 0.51 | 0.052089996 | 0.19 |
| hCV27477533 | rs3756008 | hCV32291256 | rs4253406 | 0.51 | 0.052089996 | 0.1099 |
| hCV27477533 | rs3756008 | hCV32291269 | rs4253417 | 0.51 | 0.052089996 | 0.7459 |
| hCV27477533 | rs3756008 | hCV32291286 | rs4253422 | 0.51 | 0.052089996 | 0.145 |
| hCV27477533 | rs3756008 | hCV32291287 | rs4253423 | 0.51 | 0.052089996 | 0.145 |
| hCV27477533 | rs3756008 | hCV32291295 | rs4253292 | 0.51 | 0.052089996 | 0.0659 |
| hCV27477533 | rs3756008 | hCV3229991 | rs4241815 | 0.51 | 0.052089996 | 0.1577 |
| hCV27477533 | rs3756008 | hCV3229992 | rs3775298 | 0.51 | 0.052089996 | 0.1577 |
| hCV27477533 | rs3756008 | hCV3229995 | rs11132382 | 0.51 | 0.052089996 | 0.1702 |
| hCV27477533 | rs3756008 | hCV3230000 | rs4253294 | 0.51 | 0.052089996 | 0.0691 |
| hCV27477533 | rs3756008 | hCV3230002 | rs4253297 | 0.51 | 0.052089996 | 0.3082 |
| hCV27477533 | rs3756008 | hCV3230003 | rs2304595 | 0.51 | 0.052089996 | 0.3373 |
| hCV27477533 | rs3756008 | hCV3230004 | rs4253301 | 0.51 | 0.052089996 | 0.0562 |
| hCV27477533 | rs3756008 | hCV3230006 | rs4253308 | 0.51 | 0.052089996 | 0.248 |
| hCV27477533 | rs3756008 | hCV3230007 | rs4253311 | 0.51 | 0.052089996 | 0.1577 |
| hCV27477533 | rs3756008 | hCV3230010 | rs4253315 | 0.51 | 0.052089996 | 0.0836 |
| hCV27477533 | rs3756008 | hCV3230011 | rs4253320 | 0.51 | 0.052089996 | 0.3082 |
| hCV27477533 | rs3756008 | hCV3230013 | rs3775303 | 0.51 | 0.052089996 | 0.3791 |
| hCV27477533 | rs3756008 | hCV3230014 | rs4861709 | 0.51 | 0.052089996 | 0.0691 |
| hCV27477533 | rs3756008 | hCV3230016 | rs4253325 | 0.51 | 0.052089996 | 0.0821 |
| hCV27477533 | rs3756008 | hCV3230017 | rs4253327 | 0.51 | 0.052089996 | 0.117 |
| hCV27477533 | rs3756008 | hCV3230018 | rs925453 | 0.51 | 0.052089996 | 0.0596 |
| hCV27477533 | rs3756008 | hCV3230019 | rs4253332 | 0.51 | 0.052089996 | 0.0555 |
| hCV27477533 | rs3756008 | hCV3230021 | rs13135645 | 0.51 | 0.052089996 | 0.0833 |
| hCV27477533 | rs3756008 | hCV3230022 | rs11132383 | 0.51 | 0.052089996 | 0.3776 |
| hCV27477533 | rs3756008 | hCV3230025 | rs3756009 | 0.51 | 0.052089996 | 1 |
| hCV27477533 | rs3756008 | hCV3230030 | rs4253408 | 0.51 | 0.052089996 | 0.1104 |
| hCV27477533 | rs3756008 | hCV3230031 | rs4253419 | 0.51 | 0.052089996 | 0.145 |
| hCV27477533 | rs3756008 | hCV3230038 | rs2289252 | 0.51 | 0.052089996 | 0.7249 |
| hCV27477533 | rs3756008 | hCV3230051 | rs4862658 | 0.51 | 0.052089996 | 0.0568 |
| hCV27477533 | rs3756008 | hCV3230083 | rs10013653 | 0.51 | 0.052089996 | 0.3055 |
| hCV27477533 | rs3756008 | hCV3230084 | rs7682918 | 0.51 | 0.052089996 | 0.2208 |
| hCV27477533 | rs3756008 | hCV3230094 | rs7687818 | 0.51 | 0.052089996 | 0.3169 |
| hCV27477533 | rs3756008 | hCV3230096 | rs3817184 | 0.51 | 0.052089996 | 0.2279 |
| hCV27477533 | rs3756008 | hCV3230097 | rs3736455 | 0.51 | 0.052089996 | 0.2223 |
| hCV27477533 | rs3756008 | hCV3230101 | rs6835839 | 0.51 | 0.052089996 | 0.0923 |
| hCV27477533 | rs3756008 | hCV3230106 | rs1473597 | 0.51 | 0.052089996 | 0.1308 |
| hCV27477533 | rs3756008 | hCV3230110 | rs2276917 | 0.51 | 0.052089996 | 0.1218 |
| hCV27477533 | rs3756008 | hCV3230113 | rs1053094 | 0.51 | 0.052089996 | 0.2208 |
| hCV27477533 | rs3756008 | hCV3230118 | rs4253429 | 0.51 | 0.052089996 | 0.145 |
| hCV27477533 | rs3756008 | hCV3230119 | rs4253430 | 0.51 | 0.052089996 | 0.2733 |
| hCV27477533 | rs3756008 | hCV3230125 | rs11938564 | 0.51 | 0.052089996 | 0.1934 |
| hCV27477533 | rs3756008 | hCV3230131 | rs13136269 | 0.51 | 0.052089996 | 0.19 |
| hCV27477533 | rs3756008 | hCV3230133 | rs12511874 | 0.51 | 0.052089996 | 0.1373 |
| hCV27477533 | rs3756008 | hCV3230134 | rs12500151 | 0.51 | 0.052089996 | 0.196 |
| hCV27477533 | rs3756008 | hCV3230136 | rs13116273 | 0.51 | 0.052089996 | 0.1995 |
| hCV27477533 | rs3756008 | hCV32313006 | rs4253248 | 0.51 | 0.052089996 | 0.1794 |
| hCV27477533 | rs3756008 | hCV32313007 | rs4862666 | 0.51 | 0.052089996 | 0.067 |
| hCV27477533 | rs3756008 | hCV32313014 | rs4253243 | 0.51 | 0.052089996 | 0.1183 |
| hCV27477533 | rs3756008 | hCV32313024 | rs4253239 | 0.51 | 0.052089996 | 0.0659 |
| hCV27477533 | rs3756008 | hCV32358975 | rs4253255 | 0.51 | 0.052089996 | 0.1561 |
| hCV27477533 | rs3756008 | hCV32358984 | rs4253256 | 0.51 | 0.052089996 | 0.0676 |
| hCV27477533 | rs3756008 | hCV8241628 | rs907439 | 0.51 | 0.052089996 | 0.1028 |

# TABLE 3, page 48 of 71

| Interrogated SNP | Interrogated rs | LD SNP | LD SNP rs | Power | Threshold r² | r² |
|---|---|---|---|---|---|---|
| hCV27477533 | rs3756008 | hCV8241630 | rs925451 | 0.51 | 0.052089996 | 0.9804 |
| hCV27477533 | rs3756008 | hCV8241631 | rs1511802 | 0.51 | 0.052089996 | 0.265 |
| hCV27477533 | rs3756008 | hCV8241632 | rs1511801 | 0.51 | 0.052089996 | 0.1877 |
| hCV27477533 | rs3756008 | hCV8241633 | rs1511800 | 0.51 | 0.052089996 | 0.067 |
| hCV27477533 | rs3756008 | hDV68550952 | rs4253289 | 0.51 | 0.052089996 | 0.0687 |
| hCV27477533 | rs3756008 | hDV71222711 | rs4253252 | 0.51 | 0.052089996 | 0.1794 |
| hCV27859399 | rs7853989 | hCV11840510 | rs9411367 | 0.51 | 0.17565711 | 0.389 |
| hCV27859399 | rs7853989 | hCV153353 | rs7469576 | 0.51 | 0.17565711 | 0.4123 |
| hCV27859399 | rs7853989 | hCV16199728 | rs3025336 | 0.51 | 0.17565711 | 0.1833 |
| hCV27859399 | rs7853989 | hCV25610771 | rs8176751 | 0.51 | 0.17565711 | 1 |
| hCV27859399 | rs7853989 | hCV25610772 | rs8176746 | 0.51 | 0.17565711 | 0.817 |
| hCV27859399 | rs7853989 | hCV25610773 | rs8176747 | 0.51 | 0.17565711 | 0.6864 |
| hCV27859399 | rs7853989 | hCV25610781 | rs8176749 | 0.51 | 0.17565711 | 0.817 |
| hCV27859399 | rs7853989 | hCV25610791 | rs8176743 | 0.51 | 0.17565711 | 0.8167 |
| hCV27859399 | rs7853989 | hCV25610857 | rs8176693 | 0.51 | 0.17565711 | 0.817 |
| hCV27859399 | rs7853989 | hCV25987572 | rs4310274 | 0.51 | 0.17565711 | 0.297 |
| hCV27859399 | rs7853989 | hCV26744892 | rs11244079 | 0.51 | 0.17565711 | 0.6338 |
| hCV27859399 | rs7853989 | hCV27224736 | rs2073870 | 0.51 | 0.17565711 | 0.297 |
| hCV27859399 | rs7853989 | hCV27224742 | rs4454354 | 0.51 | 0.17565711 | 0.2157 |
| hCV27859399 | rs7853989 | hCV27224746 | rs10793959 | 0.51 | 0.17565711 | 0.2143 |
| hCV27859399 | rs7853989 | hCV27224748 | rs4246169 | 0.51 | 0.17565711 | 0.3057 |
| hCV27859399 | rs7853989 | hCV27224776 | rs7852396 | 0.51 | 0.17565711 | 0.3126 |
| hCV27859399 | rs7853989 | hCV27224778 | rs11244041 | 0.51 | 0.17565711 | 0.2967 |
| hCV27859399 | rs7853989 | hCV27478783 | rs3761823 | 0.51 | 0.17565711 | 0.2284 |
| hCV27859399 | rs7853989 | hCV27886018 | rs4962104 | 0.51 | 0.17565711 | 0.2137 |
| hCV27859399 | rs7853989 | hCV27936941 | rs4379511 | 0.51 | 0.17565711 | 0.2938 |
| hCV27859399 | rs7853989 | hCV27936942 | rs4424335 | 0.51 | 0.17565711 | 0.7126 |
| hCV27859399 | rs7853989 | hCV28002068 | rs4322078 | 0.51 | 0.17565711 | 0.3057 |
| hCV27859399 | rs7853989 | hCV29393501 | rs4507838 | 0.51 | 0.17565711 | 0.2887 |
| hCV27859399 | rs7853989 | hCV29393505 | rs4962039 | 0.51 | 0.17565711 | 0.5726 |
| hCV27859399 | rs7853989 | hCV29393508 | rs7046863 | 0.51 | 0.17565711 | 0.4137 |
| hCV27859399 | rs7853989 | hCV29531061 | rs9411464 | 0.51 | 0.17565711 | 0.6331 |
| hCV27859399 | rs7853989 | hCV29549191 | rs9411468 | 0.51 | 0.17565711 | 0.4432 |
| hCV27859399 | rs7853989 | hCV29597378 | rs8176672 | 0.51 | 0.17565711 | 0.817 |
| hCV27859399 | rs7853989 | hCV29711974 | rs7855466 | 0.51 | 0.17565711 | 0.3655 |
| hCV27859399 | rs7853989 | hCV2980259 | rs3761821 | 0.51 | 0.17565711 | 0.2256 |
| hCV27859399 | rs7853989 | hCV30504633 | rs9919007 | 0.51 | 0.17565711 | 0.4396 |
| hCV27859399 | rs7853989 | hCV30613004 | rs7855713 | 0.51 | 0.17565711 | 0.6426 |
| hCV27859399 | rs7853989 | hCV3183094 | rs8176731 | 0.51 | 0.17565711 | 0.1975 |
| hCV27859399 | rs7853989 | hCV3183096 | rs8176730 | 0.51 | 0.17565711 | 1 |
| hCV27859399 | rs7853989 | hCV3183097 | rs8176725 | 0.51 | 0.17565711 | 1 |
| hCV27859399 | rs7853989 | hCV3183098 | rs2073824 | 0.51 | 0.17565711 | 0.1773 |
| hCV27859399 | rs7853989 | hCV3183099 | rs8176722 | 0.51 | 0.17565711 | 1 |
| hCV27859399 | rs7853989 | hCV3183100 | rs8176720 | 0.51 | 0.17565711 | 0.2078 |
| hCV27859399 | rs7853989 | hCV3183111 | rs643434 | 0.51 | 0.17565711 | 0.1852 |
| hCV27859399 | rs7853989 | hCV3183246 | rs10901263 | 0.51 | 0.17565711 | 0.2412 |
| hCV27859399 | rs7853989 | hCV32126435 | rs11244034 | 0.51 | 0.17565711 | 0.297 |
| hCV27859399 | rs7853989 | hCV32126442 | rs7864821 | 0.51 | 0.17565711 | 0.2143 |
| hCV27859399 | rs7853989 | hCV32126443 | rs10793957 | 0.51 | 0.17565711 | 0.2286 |
| hCV27859399 | rs7853989 | hCV32126447 | rs6597610 | 0.51 | 0.17565711 | 0.2143 |
| hCV27859399 | rs7853989 | hCV32126454 | rs13300535 | 0.51 | 0.17565711 | 0.2888 |
| hCV27859399 | rs7853989 | hCV32126487 | rs10901250 | 0.51 | 0.17565711 | 0.3729 |
| hCV27859399 | rs7853989 | hCV442675 | rs9411471 | 0.51 | 0.17565711 | 0.4137 |
| hCV27859399 | rs7853989 | hCV7481808 | rs886082 | 0.51 | 0.17565711 | 0.2759 |
| hCV27859399 | rs7853989 | hCV7948166 | rs9411463 | 0.51 | 0.17565711 | 0.7297 |

## TABLE 3, page 49 of 71

| Interrogated SNP | Interrogated rs | LD SNP | LD SNP rs | Power | Threshold r² | r² |
|---|---|---|---|---|---|---|
| hCV27859399 | rs7853989 | hCV7948171 | rs4246170 | 0.51 | 0.17565711 | 0.6426 |
| hCV27859399 | rs7853989 | hCV9327931 | rs17150482 | 0.51 | 0.17565711 | 0.1872 |
| hCV27859399 | rs7853989 | hCV997907 | rs657152 | 0.51 | 0.17565711 | 0.2 |
| hCV27859399 | rs7853989 | hCV997909 | rs644234 | 0.51 | 0.17565711 | 0.2 |
| hCV27902808 | rs4253236 | hCV11786147 | rs4862662 | 0.51 | 0.163416276 | 0.2369 |
| hCV27902808 | rs4253236 | hCV11786203 | rs4253251 | 0.51 | 0.163416276 | 0.3848 |
| hCV27902808 | rs4253236 | hCV11786258 | rs4253303 | 0.51 | 0.163416276 | 0.366 |
| hCV27902808 | rs4253236 | hCV11786259 | rs4253304 | 0.51 | 0.163416276 | 0.4346 |
| hCV27902808 | rs4253236 | hCV11786327 | rs13133050 | 0.51 | 0.163416276 | 0.1961 |
| hCV27902808 | rs4253236 | hCV12066118 | rs2048 | 0.51 | 0.163416276 | 0.6716 |
| hCV27902808 | rs4253236 | hCV12066119 | rs1912826 | 0.51 | 0.163416276 | 0.6075 |
| hCV27902808 | rs4253236 | hCV12066124 | rs2036914 | 0.51 | 0.163416276 | 0.1725 |
| hCV27902808 | rs4253236 | hCV12066129 | rs1593 | 0.51 | 0.163416276 | 0.2043 |
| hCV27902808 | rs4253236 | hCV15968025 | rs2292425 | 0.51 | 0.163416276 | 0.399 |
| hCV27902808 | rs4253236 | hCV15968026 | rs2292426 | 0.51 | 0.163416276 | 0.4275 |
| hCV27902808 | rs4253236 | hCV15968043 | rs2292423 | 0.51 | 0.163416276 | 0.4376 |
| hCV27902808 | rs4253236 | hCV15975109 | rs2304596 | 0.51 | 0.163416276 | 0.364 |
| hCV27902808 | rs4253236 | hCV2103392 | rs12500826 | 0.51 | 0.163416276 | 0.192 |
| hCV27902808 | rs4253236 | hCV22271609 | rs4253326 | 0.51 | 0.163416276 | 0.3079 |
| hCV27902808 | rs4253236 | hCV22272267 | rs3733402 | 0.51 | 0.163416276 | 0.6716 |
| hCV27902808 | rs4253236 | hCV25634781 | rs4253299 | 0.51 | 0.163416276 | 0.3286 |
| hCV27902808 | rs4253236 | hCV25989001 | hCV25989001 | 0.51 | 0.163416276 | 0.3848 |
| hCV27902808 | rs4253236 | hCV25990131 | rs13146272 | 0.51 | 0.163416276 | 0.3974 |
| hCV27902808 | rs4253236 | hCV26265197 | rs10014399 | 0.51 | 0.163416276 | 0.3848 |
| hCV27902808 | rs4253236 | hCV26265199 | rs2221843 | 0.51 | 0.163416276 | 0.3286 |
| hCV27902808 | rs4253236 | hCV26265231 | rs7684025 | 0.51 | 0.163416276 | 0.3223 |
| hCV27902808 | rs4253236 | hCV27482765 | rs3775301 | 0.51 | 0.163416276 | 0.364 |
| hCV27902808 | rs4253236 | hCV27506149 | rs3822055 | 0.51 | 0.163416276 | 0.3286 |
| hCV27902808 | rs4253236 | hCV29053260 | rs4861707 | 0.51 | 0.163416276 | 0.164 |
| hCV27902808 | rs4253236 | hCV29053264 | rs7667777 | 0.51 | 0.163416276 | 0.2382 |
| hCV27902808 | rs4253236 | hCV29053265 | rs4253244 | 0.51 | 0.163416276 | 0.9634 |
| hCV27902808 | rs4253236 | hCV29718000 | rs4253238 | 0.51 | 0.163416276 | 0.6557 |
| hCV27902808 | rs4253236 | hCV29826351 | rs10025990 | 0.51 | 0.163416276 | 0.2048 |
| hCV27902808 | rs4253236 | hCV29877725 | rs4253295 | 0.51 | 0.163416276 | 0.3822 |
| hCV27902808 | rs4253236 | hCV32291217 | rs4253323 | 0.51 | 0.163416276 | 0.364 |
| hCV27902808 | rs4253236 | hCV32291295 | rs4253292 | 0.51 | 0.163416276 | 0.4307 |
| hCV27902808 | rs4253236 | hCV32291301 | rs4253302 | 0.51 | 0.163416276 | 0.3677 |
| hCV27902808 | rs4253236 | hCV32295028 | rs4253260 | 0.51 | 0.163416276 | 0.364 |
| hCV27902808 | rs4253236 | hCV3229991 | rs4241815 | 0.51 | 0.163416276 | 0.6716 |
| hCV27902808 | rs4253236 | hCV3229992 | rs3775298 | 0.51 | 0.163416276 | 0.6716 |
| hCV27902808 | rs4253236 | hCV3229995 | rs11132382 | 0.51 | 0.163416276 | 0.6412 |
| hCV27902808 | rs4253236 | hCV3230002 | rs4253297 | 0.51 | 0.163416276 | 0.3878 |
| hCV27902808 | rs4253236 | hCV3230003 | rs2304595 | 0.51 | 0.163416276 | 0.4392 |
| hCV27902808 | rs4253236 | hCV3230006 | rs4253308 | 0.51 | 0.163416276 | 0.3822 |
| hCV27902808 | rs4253236 | hCV3230007 | rs4253311 | 0.51 | 0.163416276 | 0.6716 |
| hCV27902808 | rs4253236 | hCV3230011 | rs4253320 | 0.51 | 0.163416276 | 0.3878 |
| hCV27902808 | rs4253236 | hCV3230012 | rs4241821 | 0.51 | 0.163416276 | 0.3286 |
| hCV27902808 | rs4253236 | hCV3230013 | rs3775303 | 0.51 | 0.163416276 | 0.4346 |
| hCV27902808 | rs4253236 | hCV3230083 | rs10013653 | 0.51 | 0.163416276 | 0.2827 |
| hCV27902808 | rs4253236 | hCV3230084 | rs7682918 | 0.51 | 0.163416276 | 0.2441 |
| hCV27902808 | rs4253236 | hCV3230094 | rs7687818 | 0.51 | 0.163416276 | 0.2896 |
| hCV27902808 | rs4253236 | hCV3230096 | rs3817184 | 0.51 | 0.163416276 | 0.2369 |
| hCV27902808 | rs4253236 | hCV3230097 | rs3736455 | 0.51 | 0.163416276 | 0.4807 |
| hCV27902808 | rs4253236 | hCV3230113 | rs1053094 | 0.51 | 0.163416276 | 0.3483 |
| hCV27902808 | rs4253236 | hCV32313006 | rs4253248 | 0.51 | 0.163416276 | 0.6434 |

# TABLE 3, page 50 of 71

| Interrogated SNP | Interrogated rs | LD SNP | LD SNP rs | Power | Threshold r² | r² |
|---|---|---|---|---|---|---|
| hCV27902808 | rs4253236 | hCV32313024 | rs4253239 | 0.51 | 0.163416276 | 0.4307 |
| hCV27902808 | rs4253236 | hCV32358975 | rs4253255 | 0.51 | 0.163416276 | 0.6676 |
| hCV27902808 | rs4253236 | hCV32358984 | rs4253256 | 0.51 | 0.163416276 | 1 |
| hCV27902808 | rs4253236 | hCV8241631 | rs1511802 | 0.51 | 0.163416276 | 0.3786 |
| hCV27902808 | rs4253236 | hCV8241632 | rs1511801 | 0.51 | 0.163416276 | 0.6346 |
| hCV27902808 | rs4253236 | hDV71222711 | rs4253252 | 0.51 | 0.163416276 | 0.6434 |
| hCV2892877 | rs6050 | hCV11503382 | rs1873369 | 0.51 | 0.118446629 | 0.2903 |
| hCV2892877 | rs6050 | hCV11503414 | rs2066865 | 0.51 | 0.118446629 | 0.873 |
| hCV2892877 | rs6050 | hCV11503416 | rs2066864 | 0.51 | 0.118446629 | 0.8694 |
| hCV2892877 | rs6050 | hCV11503431 | rs2066861 | 0.51 | 0.118446629 | 0.8734 |
| hCV2892877 | rs6050 | hCV11503469 | rs2066854 | 0.51 | 0.118446629 | 0.8287 |
| hCV2892877 | rs6050 | hCV11503470 | rs1800788 | 0.51 | 0.118446629 | 0.5042 |
| hCV2892877 | rs6050 | hCV11853378 | rs1907154 | 0.51 | 0.118446629 | 0.1345 |
| hCV2892877 | rs6050 | hCV11853384 | rs12646456 | 0.51 | 0.118446629 | 0.1345 |
| hCV2892877 | rs6050 | hCV11853387 | rs1490683 | 0.51 | 0.118446629 | 0.1884 |
| hCV2892877 | rs6050 | hCV11853483 | rs12644950 | 0.51 | 0.118446629 | 0.863 |
| hCV2892877 | rs6050 | hCV11853489 | rs7681423 | 0.51 | 0.118446629 | 0.8694 |
| hCV2892877 | rs6050 | hCV11853496 | rs7654093 | 0.51 | 0.118446629 | 0.8734 |
| hCV2892877 | rs6050 | hCV11853631 | rs12651106 | 0.51 | 0.118446629 | 0.1417 |
| hCV2892877 | rs6050 | hCV1190572 | rs1032335 | 0.51 | 0.118446629 | 0.1345 |
| hCV2892877 | rs6050 | hCV15860433 | rs2070006 | 0.51 | 0.118446629 | 0.4576 |
| hCV2892877 | rs6050 | hCV21680 | rs7666020 | 0.51 | 0.118446629 | 0.1673 |
| hCV2892877 | rs6050 | hCV21681 | rs6536018 | 0.51 | 0.118446629 | 0.2886 |
| hCV2892877 | rs6050 | hCV24834 | rs4235247 | 0.51 | 0.118446629 | 0.4645 |
| hCV2892877 | rs6050 | hCV26019871 | rs4547780 | 0.51 | 0.118446629 | 0.3118 |
| hCV2892877 | rs6050 | hCV26024202 | rs11731813 | 0.51 | 0.118446629 | 0.2608 |
| hCV2892877 | rs6050 | hCV265748 | rs12500118 | 0.51 | 0.118446629 | 0.1425 |
| hCV2892877 | rs6050 | hCV27020269 | rs7659613 | 0.51 | 0.118446629 | 0.4867 |
| hCV2892877 | rs6050 | hCV27020277 | rs6825454 | 0.51 | 0.118446629 | 0.948 |
| hCV2892877 | rs6050 | hCV27020280 | rs4463047 | 0.51 | 0.118446629 | 0.2695 |
| hCV2892877 | rs6050 | hCV27905214 | rs4323084 | 0.51 | 0.118446629 | 0.3304 |
| hCV2892877 | rs6050 | hCV27907560 | rs4696576 | 0.51 | 0.118446629 | 0.1284 |
| hCV2892877 | rs6050 | hCV27937396 | rs4634201 | 0.51 | 0.118446629 | 0.4655 |
| hCV2892877 | rs6050 | hCV286004 | rs1118824 | 0.51 | 0.118446629 | 0.1344 |
| hCV2892877 | rs6050 | hCV2892855 | rs6536024 | 0.51 | 0.118446629 | 0.1935 |
| hCV2892877 | rs6050 | hCV2892858 | rs12648395 | 0.51 | 0.118446629 | 0.1344 |
| hCV2892877 | rs6050 | hCV2892859 | rs13130318 | 0.51 | 0.118446629 | 0.7297 |
| hCV2892877 | rs6050 | hCV2892863 | rs1049636 | 0.51 | 0.118446629 | 0.1344 |
| hCV2892877 | rs6050 | hCV2892869 | rs13109457 | 0.51 | 0.118446629 | 0.9105 |
| hCV2892877 | rs6050 | hCV2892870 | rs2070011 | 0.51 | 0.118446629 | 0.477 |
| hCV2892877 | rs6050 | hCV2892893 | rs12648258 | 0.51 | 0.118446629 | 0.4392 |
| hCV2892877 | rs6050 | hCV2892905 | rs12642770 | 0.51 | 0.118446629 | 0.4305 |
| hCV2892877 | rs6050 | hCV2892918 | rs12511469 | 0.51 | 0.118446629 | 0.4276 |
| hCV2892877 | rs6050 | hCV2892923 | rs13435192 | 0.51 | 0.118446629 | 0.1513 |
| hCV2892877 | rs6050 | hCV2892924 | rs13435101 | 0.51 | 0.118446629 | 0.15 |
| hCV2892877 | rs6050 | hCV2892925 | rs7689945 | 0.51 | 0.118446629 | 0.1457 |
| hCV2892877 | rs6050 | hCV2892926 | rs7662567 | 0.51 | 0.118446629 | 0.4373 |
| hCV2892877 | rs6050 | hCV2892927 | rs13123551 | 0.51 | 0.118446629 | 0.1818 |
| hCV2892877 | rs6050 | hCV2892928 | rs13147579 | 0.51 | 0.118446629 | 0.4507 |
| hCV2892877 | rs6050 | hCV28953838 | rs7690851 | 0.51 | 0.118446629 | 0.3195 |
| hCV2892877 | rs6050 | hCV28953840 | rs6536017 | 0.51 | 0.118446629 | 0.1303 |
| hCV2892877 | rs6050 | hCV28966638 | rs7676857 | 0.51 | 0.118446629 | 0.1381 |
| hCV2892877 | rs6050 | hCV29420822 | rs4642230 | 0.51 | 0.118446629 | 0.5295 |
| hCV2892877 | rs6050 | hCV29983641 | rs10008078 | 0.51 | 0.118446629 | 0.5304 |
| hCV2892877 | rs6050 | hCV30679164 | rs12649437 | 0.51 | 0.118446629 | 0.1198 |

## TABLE 3, page 51 of 71

| Interrogated SNP | Interrogated rs | LD SNP | LD SNP rs | Power | Threshold $r^2$ | $r^2$ |
|---|---|---|---|---|---|---|
| hCV2892877 | rs6050 | hCV30679170 | rs13148992 | 0.51 | 0.118446629 | 0.2697 |
| hCV2892877 | rs6050 | hCV30711231 | rs12642469 | 0.51 | 0.118446629 | 0.5304 |
| hCV2892877 | rs6050 | hCV31863979 | rs12186294 | 0.51 | 0.118446629 | 0.212 |
| hCV2892877 | rs6050 | hCV31863982 | rs7659024 | 0.51 | 0.118446629 | 0.8734 |
| hCV2892877 | rs6050 | hCV32212659 | rs4622984 | 0.51 | 0.118446629 | 0.2094 |
| hCV2892877 | rs6050 | hCV354895 | rs11737226 | 0.51 | 0.118446629 | 0.2405 |
| hCV2892877 | rs6050 | hCV354896 | rs7690972 | 0.51 | 0.118446629 | 0.2405 |
| hCV2892877 | rs6050 | hCV426173 | rs12504201 | 0.51 | 0.118446629 | 0.1904 |
| hCV2892877 | rs6050 | hCV426175 | rs9884952 | 0.51 | 0.118446629 | 0.1345 |
| hCV2892877 | rs6050 | hCV426176 | rs9884775 | 0.51 | 0.118446629 | 0.1345 |
| hCV2892877 | rs6050 | hCV426178 | rs9884570 | 0.51 | 0.118446629 | 0.123 |
| hCV2892877 | rs6050 | hCV426181 | rs11099955 | 0.51 | 0.118446629 | 0.1345 |
| hCV2892877 | rs6050 | hCV426182 | rs10014536 | 0.51 | 0.118446629 | 0.1443 |
| hCV2892877 | rs6050 | hCV426183 | rs10014635 | 0.51 | 0.118446629 | 0.1452 |
| hCV2892877 | rs6050 | hCV426184 | rs1032336 | 0.51 | 0.118446629 | 0.1345 |
| hCV2892877 | rs6050 | hCV470979 | rs1490672 | 0.51 | 0.118446629 | 0.2588 |
| hCV2892877 | rs6050 | hCV7429780 | rs1800792 | 0.51 | 0.118446629 | 0.1939 |
| hCV2892877 | rs6050 | hCV7429782 | rs1118823 | 0.51 | 0.118446629 | 0.1313 |
| hCV2892877 | rs6050 | hCV7429793 | rs1025154 | 0.51 | 0.118446629 | 0.5304 |
| hCV2892877 | rs6050 | hCV7430148 | rs1490685 | 0.51 | 0.118446629 | 0.1345 |
| hCV2892877 | rs6050 | hCV7430158 | rs1466662 | 0.51 | 0.118446629 | 0.1425 |
| hCV2892877 | rs6050 | hDV70945235 | rs17373860 | 0.51 | 0.118446629 | 0.2179 |
| hCV2915511 | rs627530 | hCV11470250 | rs678276 | 0.51 | 0.808781214 | 1 |
| hCV2915511 | rs627530 | hCV2406318 | rs661315 | 0.51 | 0.808781214 | 1 |
| hCV2915511 | rs627530 | hCV2414147 | rs681747 | 0.51 | 0.808781214 | 1 |
| hCV2915511 | rs627530 | hCV2414148 | rs620698 | 0.51 | 0.808781214 | 1 |
| hCV2915511 | rs627530 | hCV2414150 | rs675291 | 0.51 | 0.808781214 | 1 |
| hCV2915511 | rs627530 | hCV31716873 | rs13403516 | 0.51 | 0.808781214 | 1 |
| hCV2915511 | rs627530 | hCV7540 | rs633200 | 0.51 | 0.808781214 | 1 |
| hCV2915511 | rs627530 | hCV8840562 | rs668034 | 0.51 | 0.808781214 | 1 |
| hCV2986566 | rs4149755 | hCV1264349 | rs12557491 | 0.51 | 0.454330944 | 0.6651 |
| hCV2986566 | rs4149755 | hCV29394017 | rs7058459 | 0.51 | 0.454330944 | 0.4683 |
| hCV30562347 | rs4253418 | hCV11786295 | rs4253421 | 0.51 | 0.434589475 | 0.4975 |
| hCV30562347 | rs4253418 | hCV12066129 | rs1593 | 0.51 | 0.434589475 | 0.4706 |
| hCV30562347 | rs4253418 | hCV29826351 | rs10025990 | 0.51 | 0.434589475 | 0.5058 |
| hCV30690777 | rs12045585 | hCV15760229 | rs3006939 | 0.51 | 0.480095851 | 0.4853 |
| hCV30690777 | rs12045585 | hCV15760280 | rs3006940 | 0.51 | 0.480095851 | 0.4853 |
| hCV30690777 | rs12045585 | hCV26034158 | rs4515770 | 0.51 | 0.480095851 | 0.4853 |
| hCV30690777 | rs12045585 | hCV29210363 | rs6656918 | 0.51 | 0.480095851 | 0.4853 |
| hCV30690777 | rs12045585 | hCV30690778 | rs12140414 | 0.51 | 0.480095851 | 0.5671 |
| hCV30690777 | rs12045585 | hCV30690780 | rs10737888 | 0.51 | 0.480095851 | 0.4853 |
| hCV30690777 | rs12045585 | hCV8688111 | rs1578275 | 0.51 | 0.480095851 | 0.5671 |
| hCV30690777 | rs12045585 | hCV97631 | rs1538773 | 0.51 | 0.480095851 | 0.4853 |
| hCV30690780 | rs10737888 | hCV12073160 | rs1973284 | 0.51 | 0.262666713 | 0.4193 |
| hCV30690780 | rs10737888 | hCV12073167 | rs2034915 | 0.51 | 0.262666713 | 0.4232 |
| hCV30690780 | rs10737888 | hCV12073172 | rs971285 | 0.51 | 0.262666713 | 0.4257 |
| hCV30690780 | rs10737888 | hCV12073836 | rs1008173 | 0.51 | 0.262666713 | 0.2647 |
| hCV30690780 | rs10737888 | hCV12073840 | rs14403 | 0.51 | 0.262666713 | 0.5374 |
| hCV30690780 | rs10737888 | hCV15755277 | rs3008657 | 0.51 | 0.262666713 | 0.3711 |
| hCV30690780 | rs10737888 | hCV15760229 | rs3006939 | 0.51 | 0.262666713 | 1 |
| hCV30690780 | rs10737888 | hCV15760238 | rs3006936 | 0.51 | 0.262666713 | 1 |
| hCV30690780 | rs10737888 | hCV15760239 | rs3006923 | 0.51 | 0.262666713 | 0.4737 |
| hCV30690780 | rs10737888 | hCV15760280 | rs3006940 | 0.51 | 0.262666713 | 1 |
| hCV30690780 | rs10737888 | hCV15776869 | rs2345994 | 0.51 | 0.262666713 | 0.4139 |
| hCV30690780 | rs10737888 | hCV15823024 | rs2125230 | 0.51 | 0.262666713 | 0.763 |

## TABLE 3, page 52 of 71

| Interrogated SNP | Interrogated rs | LD SNP | LD SNP rs | Power | Threshold r² | r² |
|---|---|---|---|---|---|---|
| hCV30690780 | rs10737888 | hCV15823033 | rs2125231 | 0.51 | 0.262666713 | 0.4257 |
| hCV30690780 | rs10737888 | hCV15885425 | rs2290754 | 0.51 | 0.262666713 | 0.763 |
| hCV30690780 | rs10737888 | hCV15885435 | rs2290753 | 0.51 | 0.262666713 | 0.4257 |
| hCV30690780 | rs10737888 | hCV15953062 | rs2953330 | 0.51 | 0.262666713 | 0.3229 |
| hCV30690780 | rs10737888 | hCV15953063 | rs2953331 | 0.51 | 0.262666713 | 0.3293 |
| hCV30690780 | rs10737888 | hCV15965328 | rs2291409 | 0.51 | 0.262666713 | 0.4454 |
| hCV30690780 | rs10737888 | hCV15965338 | rs2291410 | 0.51 | 0.262666713 | 0.7354 |
| hCV30690780 | rs10737888 | hCV16082410 | rs2881275 | 0.51 | 0.262666713 | 0.6818 |
| hCV30690780 | rs10737888 | hCV1678656 | rs1458024 | 0.51 | 0.262666713 | 0.6818 |
| hCV30690780 | rs10737888 | hCV1678668 | rs1379700 | 0.51 | 0.262666713 | 0.3257 |
| hCV30690780 | rs10737888 | hCV1678674 | rs1458023 | 0.51 | 0.262666713 | 0.7493 |
| hCV30690780 | rs10737888 | hCV1678683 | rs1486475 | 0.51 | 0.262666713 | 0.3257 |
| hCV30690780 | rs10737888 | hCV1678687 | rs320305 | 0.51 | 0.262666713 | 0.6181 |
| hCV30690780 | rs10737888 | hCV1678723 | rs1486472 | 0.51 | 0.262666713 | 0.3224 |
| hCV30690780 | rs10737888 | hCV233148 | rs1417121 | 0.51 | 0.262666713 | 0.6414 |
| hCV30690780 | rs10737888 | hCV26034142 | rs9428576 | 0.51 | 0.262666713 | 0.3104 |
| hCV30690780 | rs10737888 | hCV26034157 | rs2994329 | 0.51 | 0.262666713 | 0.2668 |
| hCV30690780 | rs10737888 | hCV26034158 | rs4515770 | 0.51 | 0.262666713 | 1 |
| hCV30690780 | rs10737888 | hCV26034160 | rs2994327 | 0.51 | 0.262666713 | 0.4781 |
| hCV30690780 | rs10737888 | hCV26719082 | rs10927046 | 0.51 | 0.262666713 | 0.6045 |
| hCV30690780 | rs10737888 | hCV26719085 | rs10927047 | 0.51 | 0.262666713 | 0.635 |
| hCV30690780 | rs10737888 | hCV26719086 | rs4658585 | 0.51 | 0.262666713 | 0.4054 |
| hCV30690780 | rs10737888 | hCV26719087 | rs4658401 | 0.51 | 0.262666713 | 0.2676 |
| hCV30690780 | rs10737888 | hCV26719107 | rs7538011 | 0.51 | 0.262666713 | 0.6653 |
| hCV30690780 | rs10737888 | hCV26719108 | rs10927035 | 0.51 | 0.262666713 | 0.5743 |
| hCV30690780 | rs10737888 | hCV26719113 | rs7517340 | 0.51 | 0.262666713 | 0.617 |
| hCV30690780 | rs10737888 | hCV26719114 | rs7549780 | 0.51 | 0.262666713 | 0.4257 |
| hCV30690780 | rs10737888 | hCV26719116 | rs10927039 | 0.51 | 0.262666713 | 0.7747 |
| hCV30690780 | rs10737888 | hCV26719117 | rs12144559 | 0.51 | 0.262666713 | 0.4257 |
| hCV30690780 | rs10737888 | hCV26719120 | rs10927040 | 0.51 | 0.262666713 | 0.7881 |
| hCV30690780 | rs10737888 | hCV26719121 | rs10927041 | 0.51 | 0.262666713 | 0.7881 |
| hCV30690780 | rs10737888 | hCV26719137 | rs12136847 | 0.51 | 0.262666713 | 0.4257 |
| hCV30690780 | rs10737888 | hCV26719149 | rs6675851 | 0.51 | 0.262666713 | 0.763 |
| hCV30690780 | rs10737888 | hCV26719162 | rs4132509 | 0.51 | 0.262666713 | 0.763 |
| hCV30690780 | rs10737888 | hCV26719163 | rs6429435 | 0.51 | 0.262666713 | 0.4257 |
| hCV30690780 | rs10737888 | hCV26719171 | rs10927075 | 0.51 | 0.262666713 | 0.4054 |
| hCV30690780 | rs10737888 | hCV26719176 | rs10927076 | 0.51 | 0.262666713 | 0.763 |
| hCV30690780 | rs10737888 | hCV26719179 | rs6672195 | 0.51 | 0.262666713 | 0.4257 |
| hCV30690780 | rs10737888 | hCV26719192 | rs10803161 | 0.51 | 0.262666713 | 0.7097 |
| hCV30690780 | rs10737888 | hCV26719194 | rs10927081 | 0.51 | 0.262666713 | 0.4257 |
| hCV30690780 | rs10737888 | hCV26719197 | rs4590656 | 0.51 | 0.262666713 | 0.4257 |
| hCV30690780 | rs10737888 | hCV26719201 | rs4478795 | 0.51 | 0.262666713 | 0.4255 |
| hCV30690780 | rs10737888 | hCV26719202 | rs4658588 | 0.51 | 0.262666713 | 0.4232 |
| hCV30690780 | rs10737888 | hCV26719215 | rs12144546 | 0.51 | 0.262666713 | 0.4257 |
| hCV30690780 | rs10737888 | hCV26719217 | rs7548254 | 0.51 | 0.262666713 | 0.4257 |
| hCV30690780 | rs10737888 | hCV26719219 | rs9782958 | 0.51 | 0.262666713 | 0.7247 |
| hCV30690780 | rs10737888 | hCV26719222 | rs4553169 | 0.51 | 0.262666713 | 0.763 |
| hCV30690780 | rs10737888 | hCV26719225 | rs11586029 | 0.51 | 0.262666713 | 0.4257 |
| hCV30690780 | rs10737888 | hCV26719227 | rs10927065 | 0.51 | 0.262666713 | 0.6111 |
| hCV30690780 | rs10737888 | hCV26719232 | rs10803158 | 0.51 | 0.262666713 | 0.7586 |
| hCV30690780 | rs10737888 | hCV26719233 | rs10927067 | 0.51 | 0.262666713 | 0.763 |
| hCV30690780 | rs10737888 | hCV27170898 | rs12753750 | 0.51 | 0.262666713 | 0.3576 |
| hCV30690780 | rs10737888 | hCV27171350 | rs4430311 | 0.51 | 0.262666713 | 0.3794 |
| hCV30690780 | rs10737888 | hCV27498250 | rs3766673 | 0.51 | 0.262666713 | 0.7605 |
| hCV30690780 | rs10737888 | hCV29210363 | rs6656918 | 0.51 | 0.262666713 | 1 |

## TABLE 3, page 53 of 71

| Interrogated SNP | Interrogated rs | LD SNP | LD SNP rs | Power | Threshold r² | r² |
|---|---|---|---|---|---|---|
| hCV30690780 | rs10737888 | hCV29542869 | rs7534117 | 0.51 | 0.262666713 | 0.7586 |
| hCV30690780 | rs10737888 | hCV29560960 | rs7519673 | 0.51 | 0.262666713 | 0.6181 |
| hCV30690780 | rs10737888 | hCV29741723 | rs7517921 | 0.51 | 0.262666713 | 0.7354 |
| hCV30690780 | rs10737888 | hCV29795761 | rs7528450 | 0.51 | 0.262666713 | 0.3632 |
| hCV30690780 | rs10737888 | hCV29994467 | rs6694738 | 0.51 | 0.262666713 | 0.6653 |
| hCV30690780 | rs10737888 | hCV30012351 | rs10158245 | 0.51 | 0.262666713 | 0.2721 |
| hCV30690780 | rs10737888 | hCV30084348 | rs9287269 | 0.51 | 0.262666713 | 0.7625 |
| hCV30690780 | rs10737888 | hCV30372886 | rs9782883 | 0.51 | 0.262666713 | 0.763 |
| hCV30690780 | rs10737888 | hCV30382231 | rs9428966 | 0.51 | 0.262666713 | 0.687 |
| hCV30690780 | rs10737888 | hCV30690777 | rs12045585 | 0.51 | 0.262666713 | 0.4853 |
| hCV30690780 | rs10737888 | hCV30690778 | rs12140414 | 0.51 | 0.262666713 | 0.8652 |
| hCV30690780 | rs10737888 | hCV30690784 | rs4658574 | 0.51 | 0.262666713 | 0.4781 |
| hCV30690780 | rs10737888 | hCV31523552 | rs12739344 | 0.51 | 0.262666713 | 0.4405 |
| hCV30690780 | rs10737888 | hCV31523555 | rs12749316 | 0.51 | 0.262666713 | 0.3008 |
| hCV30690780 | rs10737888 | hCV31523557 | rs10754807 | 0.51 | 0.262666713 | 0.763 |
| hCV30690780 | rs10737888 | hCV31523563 | rs10927051 | 0.51 | 0.262666713 | 0.6787 |
| hCV30690780 | rs10737888 | hCV31523573 | rs11589907 | 0.51 | 0.262666713 | 0.2935 |
| hCV30690780 | rs10737888 | hCV31523576 | rs12691548 | 0.51 | 0.262666713 | 0.4257 |
| hCV30690780 | rs10737888 | hCV31523608 | rs12744297 | 0.51 | 0.262666713 | 0.3354 |
| hCV30690780 | rs10737888 | hCV31523624 | rs10927044 | 0.51 | 0.262666713 | 0.4405 |
| hCV30690780 | rs10737888 | hCV31523638 | rs12037013 | 0.51 | 0.262666713 | 0.6415 |
| hCV30690780 | rs10737888 | hCV31523639 | rs12034588 | 0.51 | 0.262666713 | 0.6809 |
| hCV30690780 | rs10737888 | hCV31523643 | rs6671475 | 0.51 | 0.262666713 | 0.7881 |
| hCV30690780 | rs10737888 | hCV31523650 | rs12048930 | 0.51 | 0.262666713 | 0.7354 |
| hCV30690780 | rs10737888 | hCV31523658 | rs12047209 | 0.51 | 0.262666713 | 0.4836 |
| hCV30690780 | rs10737888 | hCV31523688 | rs12049228 | 0.51 | 0.262666713 | 0.6692 |
| hCV30690780 | rs10737888 | hCV31523691 | rs12021907 | 0.51 | 0.262666713 | 0.6181 |
| hCV30690780 | rs10737888 | hCV31523707 | rs10803152 | 0.51 | 0.262666713 | 0.6415 |
| hCV30690780 | rs10737888 | hCV31523710 | rs10927059 | 0.51 | 0.262666713 | 0.763 |
| hCV30690780 | rs10737888 | hCV31523723 | rs12140040 | 0.51 | 0.262666713 | 0.4909 |
| hCV30690780 | rs10737888 | hCV31523736 | rs12124113 | 0.51 | 0.262666713 | 0.6181 |
| hCV30690780 | rs10737888 | hCV31523737 | rs12117580 | 0.51 | 0.262666713 | 0.3458 |
| hCV30690780 | rs10737888 | hCV31523740 | rs12032342 | 0.51 | 0.262666713 | 0.6818 |
| hCV30690780 | rs10737888 | hCV31523744 | rs12031994 | 0.51 | 0.262666713 | 0.6181 |
| hCV30690780 | rs10737888 | hCV804126 | rs320320 | 0.51 | 0.262666713 | 0.6818 |
| hCV30690780 | rs10737888 | hCV8688079 | rs884808 | 0.51 | 0.262666713 | 0.5066 |
| hCV30690780 | rs10737888 | hCV8688080 | rs884328 | 0.51 | 0.262666713 | 0.5066 |
| hCV30690780 | rs10737888 | hCV8688098 | rs1531244 | 0.51 | 0.262666713 | 0.4026 |
| hCV30690780 | rs10737888 | hCV8688111 | rs1578275 | 0.51 | 0.262666713 | 0.8627 |
| hCV30690780 | rs10737888 | hCV8688770 | rs3856231 | 0.51 | 0.262666713 | 0.4257 |
| hCV30690780 | rs10737888 | hCV8689016 | rs897959 | 0.51 | 0.262666713 | 0.4257 |
| hCV30690780 | rs10737888 | hCV9115290 | rs1352162 | 0.51 | 0.262666713 | 0.429 |
| hCV30690780 | rs10737888 | hCV9493073 | rs1058305 | 0.51 | 0.262666713 | 0.6914 |
| hCV30690780 | rs10737888 | hCV9493081 | rs1058304 | 0.51 | 0.262666713 | 0.6914 |
| hCV30690780 | rs10737888 | hCV97631 | rs1538773 | 0.51 | 0.262666713 | 1 |
| hCV30690780 | rs10737888 | hDV69368808 | rs12145558 | 0.51 | 0.262666713 | 0.4257 |
| hCV30690780 | rs10737888 | hDV71836703 | rs6429433 | 0.51 | 0.262666713 | 0.6326 |
| hCV30690780 | rs10737888 | hDV90784784 | rs320339 | 0.51 | 0.262666713 | 0.713 |
| hCV30710896 | rs3136520 | hCV31699700 | rs11039103 | 0.51 | 0.599559553 | 0.8293 |
| hCV30710896 | rs3136520 | hCV31699705 | rs11039099 | 0.51 | 0.599559553 | 1 |
| hCV30710896 | rs3136520 | hCV31699798 | rs11039049 | 0.51 | 0.599559553 | 1 |
| hCV30710896 | rs3136520 | hCV32292446 | rs3136524 | 0.51 | 0.599559553 | 1 |
| hCV30710896 | rs3136520 | hCV32368695 | rs7938933 | 0.51 | 0.599559553 | 0.6861 |
| hCV30747430 | rs11712211 | hCV11906384 | rs2472670 | 0.51 | 0.337199375 | 1 |
| hCV30747430 | rs11712211 | hCV11906385 | rs2472671 | 0.51 | 0.337199375 | 1 |

# TABLE 3, page 54 of 71

| Interrogated SNP | Interrogated rs | LD SNP | LD SNP rs | Power | Threshold r² | r² |
|---|---|---|---|---|---|---|
| hCV30747430 | rs11712211 | hCV11906386 | rs2056530 | 0.51 | 0.337199375 | 1 |
| hCV30747430 | rs11712211 | hCV16090105 | rs2873951 | 0.51 | 0.337199375 | 1 |
| hCV30747430 | rs11712211 | hCV26079834 | rs2472672 | 0.51 | 0.337199375 | 1 |
| hCV30747430 | rs11712211 | hCV27986929 | rs4688030 | 0.51 | 0.337199375 | 0.3388 |
| hCV30747430 | rs11712211 | hCV28031759 | rs4234666 | 0.51 | 0.337199375 | 1 |
| hCV30747430 | rs11712211 | hCV30526128 | rs9841230 | 0.51 | 0.337199375 | 0.3388 |
| hCV30747430 | rs11712211 | hCV30747431 | rs13071341 | 0.51 | 0.337199375 | 1 |
| hCV30747430 | rs11712211 | hCV8760915 | rs1403527 | 0.51 | 0.337199375 | 1 |
| hCV31523608 | rs12744297 | hCV12073160 | rs1973284 | 0.51 | 0.302208039 | 0.9556 |
| hCV31523608 | rs12744297 | hCV12073167 | rs2034915 | 0.51 | 0.302208039 | 0.9556 |
| hCV31523608 | rs12744297 | hCV12073172 | rs971285 | 0.51 | 0.302208039 | 0.9556 |
| hCV31523608 | rs12744297 | hCV12073180 | rs1870272 | 0.51 | 0.302208039 | 0.9599 |
| hCV31523608 | rs12744297 | hCV15755277 | rs3008657 | 0.51 | 0.302208039 | 0.6842 |
| hCV31523608 | rs12744297 | hCV15760229 | rs3006939 | 0.51 | 0.302208039 | 0.3354 |
| hCV31523608 | rs12744297 | hCV15760280 | rs3006940 | 0.51 | 0.302208039 | 0.3354 |
| hCV31523608 | rs12744297 | hCV15776869 | rs2345994 | 0.51 | 0.302208039 | 0.9118 |
| hCV31523608 | rs12744297 | hCV15823016 | rs2125229 | 0.51 | 0.302208039 | 0.3716 |
| hCV31523608 | rs12744297 | hCV15823024 | rs2125230 | 0.51 | 0.302208039 | 0.4338 |
| hCV31523608 | rs12744297 | hCV15823033 | rs2125231 | 0.51 | 0.302208039 | 0.9556 |
| hCV31523608 | rs12744297 | hCV15885425 | rs2290754 | 0.51 | 0.302208039 | 0.4338 |
| hCV31523608 | rs12744297 | hCV15885435 | rs2290753 | 0.51 | 0.302208039 | 0.9556 |
| hCV31523608 | rs12744297 | hCV15953062 | rs2953330 | 0.51 | 0.302208039 | 0.5697 |
| hCV31523608 | rs12744297 | hCV15953063 | rs2953331 | 0.51 | 0.302208039 | 0.717 |
| hCV31523608 | rs12744297 | hCV15953071 | rs2953329 | 0.51 | 0.302208039 | 0.4589 |
| hCV31523608 | rs12744297 | hCV15965328 | rs2291409 | 0.51 | 0.302208039 | 1 |
| hCV31523608 | rs12744297 | hCV15965338 | rs2291410 | 0.51 | 0.302208039 | 0.3872 |
| hCV31523608 | rs12744297 | hCV16082410 | rs2881275 | 0.51 | 0.302208039 | 0.4269 |
| hCV31523608 | rs12744297 | hCV16082411 | rs2881274 | 0.51 | 0.302208039 | 0.3716 |
| hCV31523608 | rs12744297 | hCV1678656 | rs1458024 | 0.51 | 0.302208039 | 0.4269 |
| hCV31523608 | rs12744297 | hCV1678658 | rs897960 | 0.51 | 0.302208039 | 0.3716 |
| hCV31523608 | rs12744297 | hCV1678668 | rs1379700 | 0.51 | 0.302208039 | 0.829 |
| hCV31523608 | rs12744297 | hCV1678674 | rs1458023 | 0.51 | 0.302208039 | 0.4338 |
| hCV31523608 | rs12744297 | hCV1678683 | rs1486475 | 0.51 | 0.302208039 | 0.829 |
| hCV31523608 | rs12744297 | hCV1678687 | rs320305 | 0.51 | 0.302208039 | 0.3415 |
| hCV31523608 | rs12744297 | hCV1678723 | rs1486472 | 0.51 | 0.302208039 | 0.7959 |
| hCV31523608 | rs12744297 | hCV26034158 | rs4515770 | 0.51 | 0.302208039 | 0.3354 |
| hCV31523608 | rs12744297 | hCV26719082 | rs10927046 | 0.51 | 0.302208039 | 0.3415 |
| hCV31523608 | rs12744297 | hCV26719085 | rs10927047 | 0.51 | 0.302208039 | 0.3716 |
| hCV31523608 | rs12744297 | hCV26719086 | rs4658585 | 0.51 | 0.302208039 | 0.9119 |
| hCV31523608 | rs12744297 | hCV26719087 | rs4658401 | 0.51 | 0.302208039 | 0.9056 |
| hCV31523608 | rs12744297 | hCV26719102 | rs10927056 | 0.51 | 0.302208039 | 0.8824 |
| hCV31523608 | rs12744297 | hCV26719107 | rs7538011 | 0.51 | 0.302208039 | 0.4024 |
| hCV31523608 | rs12744297 | hCV26719108 | rs10927035 | 0.51 | 0.302208039 | 0.8066 |
| hCV31523608 | rs12744297 | hCV26719113 | rs7517340 | 0.51 | 0.302208039 | 0.3842 |
| hCV31523608 | rs12744297 | hCV26719114 | rs7549780 | 0.51 | 0.302208039 | 0.9556 |
| hCV31523608 | rs12744297 | hCV26719116 | rs10927039 | 0.51 | 0.302208039 | 0.3861 |
| hCV31523608 | rs12744297 | hCV26719117 | rs12144559 | 0.51 | 0.302208039 | 0.9556 |
| hCV31523608 | rs12744297 | hCV26719120 | rs10927040 | 0.51 | 0.302208039 | 0.4658 |
| hCV31523608 | rs12744297 | hCV26719121 | rs10927041 | 0.51 | 0.302208039 | 0.4658 |
| hCV31523608 | rs12744297 | hCV26719137 | rs12136847 | 0.51 | 0.302208039 | 0.9556 |
| hCV31523608 | rs12744297 | hCV26719140 | rs320323 | 0.51 | 0.302208039 | 0.3716 |
| hCV31523608 | rs12744297 | hCV26719149 | rs6675851 | 0.51 | 0.302208039 | 0.4338 |
| hCV31523608 | rs12744297 | hCV26719161 | rs6682456 | 0.51 | 0.302208039 | 0.3716 |
| hCV31523608 | rs12744297 | hCV26719162 | rs4132509 | 0.51 | 0.302208039 | 0.4338 |
| hCV31523608 | rs12744297 | hCV26719163 | rs6429435 | 0.51 | 0.302208039 | 0.9556 |

# TABLE 3, page 55 of 71

| Interrogated SNP | Interrogated rs | LD SNP | LD SNP rs | Power | Threshold r² | r² |
|---|---|---|---|---|---|---|
| hCV31523608 | rs12744297 | hCV26719171 | rs10927075 | 0.51 | 0.302208039 | 0.9556 |
| hCV31523608 | rs12744297 | hCV26719176 | rs10927076 | 0.51 | 0.302208039 | 0.4338 |
| hCV31523608 | rs12744297 | hCV26719179 | rs6672195 | 0.51 | 0.302208039 | 0.9556 |
| hCV31523608 | rs12744297 | hCV26719184 | rs4658593 | 0.51 | 0.302208039 | 0.3334 |
| hCV31523608 | rs12744297 | hCV26719192 | rs10803161 | 0.51 | 0.302208039 | 0.4124 |
| hCV31523608 | rs12744297 | hCV26719193 | rs6429439 | 0.51 | 0.302208039 | 0.372 |
| hCV31523608 | rs12744297 | hCV26719194 | rs10927081 | 0.51 | 0.302208039 | 0.9556 |
| hCV31523608 | rs12744297 | hCV26719197 | rs4590656 | 0.51 | 0.302208039 | 0.9556 |
| hCV31523608 | rs12744297 | hCV26719202 | rs4658588 | 0.51 | 0.302208039 | 0.9556 |
| hCV31523608 | rs12744297 | hCV26719215 | rs12144546 | 0.51 | 0.302208039 | 0.9556 |
| hCV31523608 | rs12744297 | hCV26719217 | rs7548254 | 0.51 | 0.302208039 | 0.9556 |
| hCV31523608 | rs12744297 | hCV26719218 | rs4658403 | 0.51 | 0.302208039 | 0.4141 |
| hCV31523608 | rs12744297 | hCV26719219 | rs9782958 | 0.51 | 0.302208039 | 0.3872 |
| hCV31523608 | rs12744297 | hCV26719222 | rs4553169 | 0.51 | 0.302208039 | 0.4338 |
| hCV31523608 | rs12744297 | hCV26719225 | rs11586029 | 0.51 | 0.302208039 | 0.9556 |
| hCV31523608 | rs12744297 | hCV26719227 | rs10927065 | 0.51 | 0.302208039 | 0.3415 |
| hCV31523608 | rs12744297 | hCV26719232 | rs10803158 | 0.51 | 0.302208039 | 0.4338 |
| hCV31523608 | rs12744297 | hCV26719233 | rs10927067 | 0.51 | 0.302208039 | 0.4338 |
| hCV31523608 | rs12744297 | hCV27170898 | rs12753750 | 0.51 | 0.302208039 | 0.717 |
| hCV31523608 | rs12744297 | hCV27171311 | rs4322213 | 0.51 | 0.302208039 | 0.3716 |
| hCV31523608 | rs12744297 | hCV27171350 | rs4430311 | 0.51 | 0.302208039 | 0.6842 |
| hCV31523608 | rs12744297 | hCV27498250 | rs3766673 | 0.51 | 0.302208039 | 0.4658 |
| hCV31523608 | rs12744297 | hCV27511819 | rs3753549 | 0.51 | 0.302208039 | 0.3716 |
| hCV31523608 | rs12744297 | hCV29210363 | rs6656918 | 0.51 | 0.302208039 | 0.3354 |
| hCV31523608 | rs12744297 | hCV29210367 | rs4518884 | 0.51 | 0.302208039 | 0.9599 |
| hCV31523608 | rs12744297 | hCV29210368 | rs4313380 | 0.51 | 0.302208039 | 0.3279 |
| hCV31523608 | rs12744297 | hCV29210370 | rs6676779 | 0.51 | 0.302208039 | 0.3716 |
| hCV31523608 | rs12744297 | hCV29542869 | rs7534117 | 0.51 | 0.302208039 | 0.4338 |
| hCV31523608 | rs12744297 | hCV29560960 | rs7519673 | 0.51 | 0.302208039 | 0.3415 |
| hCV31523608 | rs12744297 | hCV29633221 | rs10157763 | 0.51 | 0.302208039 | 0.9589 |
| hCV31523608 | rs12744297 | hCV29669242 | rs7547861 | 0.51 | 0.302208039 | 0.3716 |
| hCV31523608 | rs12744297 | hCV29723686 | rs7512207 | 0.51 | 0.302208039 | 0.4007 |
| hCV31523608 | rs12744297 | hCV29741723 | rs7517921 | 0.51 | 0.302208039 | 0.3872 |
| hCV31523608 | rs12744297 | hCV29795761 | rs7528450 | 0.51 | 0.302208039 | 1 |
| hCV31523608 | rs12744297 | hCV29831992 | rs7523198 | 0.51 | 0.302208039 | 0.3716 |
| hCV31523608 | rs12744297 | hCV29859556 | rs6704286 | 0.51 | 0.302208039 | 0.3716 |
| hCV31523608 | rs12744297 | hCV29958244 | rs4614244 | 0.51 | 0.302208039 | 0.312 |
| hCV31523608 | rs12744297 | hCV29994467 | rs6694738 | 0.51 | 0.302208039 | 0.4024 |
| hCV31523608 | rs12744297 | hCV30012351 | rs10158245 | 0.51 | 0.302208039 | 0.9568 |
| hCV31523608 | rs12744297 | hCV30048213 | rs7552982 | 0.51 | 0.302208039 | 0.3716 |
| hCV31523608 | rs12744297 | hCV30048215 | rs6703013 | 0.51 | 0.302208039 | 0.3716 |
| hCV31523608 | rs12744297 | hCV30084348 | rs9287269 | 0.51 | 0.302208039 | 0.432 |
| hCV31523608 | rs12744297 | hCV30210344 | rs9428970 | 0.51 | 0.302208039 | 0.3716 |
| hCV31523608 | rs12744297 | hCV30228080 | rs4593807 | 0.51 | 0.302208039 | 0.3842 |
| hCV31523608 | rs12744297 | hCV30264437 | rs7514510 | 0.51 | 0.302208039 | 0.3716 |
| hCV31523608 | rs12744297 | hCV30354518 | rs7517732 | 0.51 | 0.302208039 | 0.3399 |
| hCV31523608 | rs12744297 | hCV30372886 | rs9782883 | 0.51 | 0.302208039 | 0.4338 |
| hCV31523608 | rs12744297 | hCV30390695 | rs7553458 | 0.51 | 0.302208039 | 0.3992 |
| hCV31523608 | rs12744297 | hCV30462455 | rs6686591 | 0.51 | 0.302208039 | 0.3716 |
| hCV31523608 | rs12744297 | hCV30606791 | rs6688135 | 0.51 | 0.302208039 | 0.3716 |
| hCV31523608 | rs12744297 | hCV30690780 | rs10737888 | 0.51 | 0.302208039 | 0.3354 |
| hCV31523608 | rs12744297 | hCV31523552 | rs12739344 | 0.51 | 0.302208039 | 1 |
| hCV31523608 | rs12744297 | hCV31523555 | rs12749316 | 0.51 | 0.302208039 | 0.9202 |
| hCV31523608 | rs12744297 | hCV31523557 | rs10754807 | 0.51 | 0.302208039 | 0.4338 |
| hCV31523608 | rs12744297 | hCV31523563 | rs10927051 | 0.51 | 0.302208039 | 0.4005 |

# TABLE 3, page 56 of 71

| Interrogated SNP | Interrogated rs | LD SNP | LD SNP rs | Power | Threshold r² | r² |
|---|---|---|---|---|---|---|
| hCV31523608 | rs12744297 | hCV31523573 | rs11589907 | 0.51 | 0.302208039 | 0.9599 |
| hCV31523608 | rs12744297 | hCV31523576 | rs12691548 | 0.51 | 0.302208039 | 0.9556 |
| hCV31523608 | rs12744297 | hCV31523624 | rs10927044 | 0.51 | 0.302208039 | 1 |
| hCV31523608 | rs12744297 | hCV31523638 | rs12037013 | 0.51 | 0.302208039 | 0.3716 |
| hCV31523608 | rs12744297 | hCV31523639 | rs12034588 | 0.51 | 0.302208039 | 0.4302 |
| hCV31523608 | rs12744297 | hCV31523643 | rs6671475 | 0.51 | 0.302208039 | 0.4658 |
| hCV31523608 | rs12744297 | hCV31523650 | rs12048930 | 0.51 | 0.302208039 | 0.3872 |
| hCV31523608 | rs12744297 | hCV31523658 | rs12047209 | 0.51 | 0.302208039 | 0.3058 |
| hCV31523608 | rs12744297 | hCV31523659 | rs10733129 | 0.51 | 0.302208039 | 0.312 |
| hCV31523608 | rs12744297 | hCV31523680 | rs4484910 | 0.51 | 0.302208039 | 0.3716 |
| hCV31523608 | rs12744297 | hCV31523688 | rs12049228 | 0.51 | 0.302208039 | 0.4319 |
| hCV31523608 | rs12744297 | hCV31523691 | rs12021907 | 0.51 | 0.302208039 | 0.3415 |
| hCV31523608 | rs12744297 | hCV31523692 | rs10927082 | 0.51 | 0.302208039 | 0.3582 |
| hCV31523608 | rs12744297 | hCV31523707 | rs10803152 | 0.51 | 0.302208039 | 0.3716 |
| hCV31523608 | rs12744297 | hCV31523710 | rs10927059 | 0.51 | 0.302208039 | 0.4338 |
| hCV31523608 | rs12744297 | hCV31523723 | rs12140040 | 0.51 | 0.302208039 | 0.3192 |
| hCV31523608 | rs12744297 | hCV31523725 | rs10927060 | 0.51 | 0.302208039 | 0.9599 |
| hCV31523608 | rs12744297 | hCV31523731 | rs10803155 | 0.51 | 0.302208039 | 0.3716 |
| hCV31523608 | rs12744297 | hCV31523736 | rs12124113 | 0.51 | 0.302208039 | 0.3415 |
| hCV31523608 | rs12744297 | hCV31523737 | rs12117580 | 0.51 | 0.302208039 | 0.8702 |
| hCV31523608 | rs12744297 | hCV31523740 | rs12032342 | 0.51 | 0.302208039 | 0.4269 |
| hCV31523608 | rs12744297 | hCV31523744 | rs12031994 | 0.51 | 0.302208039 | 0.3415 |
| hCV31523608 | rs12744297 | hCV804118 | rs320342 | 0.51 | 0.302208039 | 0.3279 |
| hCV31523608 | rs12744297 | hCV804120 | rs320344 | 0.51 | 0.302208039 | 0.3716 |
| hCV31523608 | rs12744297 | hCV804121 | rs320345 | 0.51 | 0.302208039 | 0.3716 |
| hCV31523608 | rs12744297 | hCV804126 | rs320320 | 0.51 | 0.302208039 | 0.4269 |
| hCV31523608 | rs12744297 | hCV804128 | rs167661 | 0.51 | 0.302208039 | 0.372 |
| hCV31523608 | rs12744297 | hCV804132 | rs406323 | 0.51 | 0.302208039 | 0.3716 |
| hCV31523608 | rs12744297 | hCV804139 | rs320302 | 0.51 | 0.302208039 | 0.3716 |
| hCV31523608 | rs12744297 | hCV804146 | rs320308 | 0.51 | 0.302208039 | 0.312 |
| hCV31523608 | rs12744297 | hCV804147 | rs320309 | 0.51 | 0.302208039 | 0.3716 |
| hCV31523608 | rs12744297 | hCV804156 | rs320316 | 0.51 | 0.302208039 | 0.3716 |
| hCV31523608 | rs12744297 | hCV804160 | rs320331 | 0.51 | 0.302208039 | 0.3324 |
| hCV31523608 | rs12744297 | hCV804166 | rs320334 | 0.51 | 0.302208039 | 0.372 |
| hCV31523608 | rs12744297 | hCV8688098 | rs1531244 | 0.51 | 0.302208039 | 0.9556 |
| hCV31523608 | rs12744297 | hCV8688110 | rs946824 | 0.51 | 0.302208039 | 0.3716 |
| hCV31523608 | rs12744297 | hCV8688770 | rs3856231 | 0.51 | 0.302208039 | 0.9556 |
| hCV31523608 | rs12744297 | hCV8688837 | rs320318 | 0.51 | 0.302208039 | 0.3716 |
| hCV31523608 | rs12744297 | hCV8688866 | rs1531243 | 0.51 | 0.302208039 | 0.3716 |
| hCV31523608 | rs12744297 | hCV8689005 | rs1458022 | 0.51 | 0.302208039 | 0.3716 |
| hCV31523608 | rs12744297 | hCV8689016 | rs897959 | 0.51 | 0.302208039 | 0.9556 |
| hCV31523608 | rs12744297 | hCV8689017 | rs1458021 | 0.51 | 0.302208039 | 0.3716 |
| hCV31523608 | rs12744297 | hCV8689027 | rs1545654 | 0.51 | 0.302208039 | 0.3716 |
| hCV31523608 | rs12744297 | hCV9115290 | rs1352162 | 0.51 | 0.302208039 | 0.9555 |
| hCV31523608 | rs12744297 | hCV97631 | rs1538773 | 0.51 | 0.302208039 | 0.3354 |
| hCV31523608 | rs12744297 | hDV69368808 | rs12145558 | 0.51 | 0.302208039 | 0.9556 |
| hCV31523608 | rs12744297 | hDV71836703 | rs6429433 | 0.51 | 0.302208039 | 0.3315 |
| hCV31523608 | rs12744297 | hDV90784784 | rs320339 | 0.51 | 0.302208039 | 0.3699 |
| hCV31523650 | rs12048930 | hCV12073160 | rs1973284 | 0.51 | 0.430712711 | 0.5233 |
| hCV31523650 | rs12048930 | hCV12073167 | rs2034915 | 0.51 | 0.430712711 | 0.5107 |
| hCV31523650 | rs12048930 | hCV12073172 | rs971285 | 0.51 | 0.430712711 | 0.5119 |
| hCV31523650 | rs12048930 | hCV15760229 | rs3006939 | 0.51 | 0.430712711 | 0.7354 |
| hCV31523650 | rs12048930 | hCV15760238 | rs3006936 | 0.51 | 0.430712711 | 0.6267 |
| hCV31523650 | rs12048930 | hCV15760280 | rs3006940 | 0.51 | 0.430712711 | 0.7354 |
| hCV31523650 | rs12048930 | hCV15776869 | rs2345994 | 0.51 | 0.430712711 | 0.4998 |

# TABLE 3, page 57 of 71

| Interrogated SNP | Interrogated rs | LD SNP | LD SNP rs | Power | Threshold r² | r² |
|---|---|---|---|---|---|---|
| hCV31523650 | rs12048930 | hCV15823024 | rs2125230 | 0.51 | 0.430712711 | 0.9681 |
| hCV31523650 | rs12048930 | hCV15823033 | rs2125231 | 0.51 | 0.430712711 | 0.5119 |
| hCV31523650 | rs12048930 | hCV15885425 | rs2290754 | 0.51 | 0.430712711 | 0.9681 |
| hCV31523650 | rs12048930 | hCV15885435 | rs2290753 | 0.51 | 0.430712711 | 0.5119 |
| hCV31523650 | rs12048930 | hCV15965328 | rs2291409 | 0.51 | 0.430712711 | 0.4977 |
| hCV31523650 | rs12048930 | hCV15965338 | rs2291410 | 0.51 | 0.430712711 | 0.9368 |
| hCV31523650 | rs12048930 | hCV16082410 | rs2881275 | 0.51 | 0.430712711 | 0.9314 |
| hCV31523650 | rs12048930 | hCV1678656 | rs1458024 | 0.51 | 0.430712711 | 0.9314 |
| hCV31523650 | rs12048930 | hCV1678674 | rs1458023 | 0.51 | 0.430712711 | 0.9658 |
| hCV31523650 | rs12048930 | hCV1678687 | rs320305 | 0.51 | 0.430712711 | 0.7842 |
| hCV31523650 | rs12048930 | hCV26034158 | rs4515770 | 0.51 | 0.430712711 | 0.7354 |
| hCV31523650 | rs12048930 | hCV26719082 | rs10927046 | 0.51 | 0.430712711 | 0.7744 |
| hCV31523650 | rs12048930 | hCV26719085 | rs10927047 | 0.51 | 0.430712711 | 0.8098 |
| hCV31523650 | rs12048930 | hCV26719086 | rs4658585 | 0.51 | 0.430712711 | 0.4932 |
| hCV31523650 | rs12048930 | hCV26719107 | rs7538011 | 0.51 | 0.430712711 | 0.7825 |
| hCV31523650 | rs12048930 | hCV26719113 | rs7517340 | 0.51 | 0.430712711 | 0.8564 |
| hCV31523650 | rs12048930 | hCV26719114 | rs7549780 | 0.51 | 0.430712711 | 0.5119 |
| hCV31523650 | rs12048930 | hCV26719116 | rs10927039 | 0.51 | 0.430712711 | 0.7976 |
| hCV31523650 | rs12048930 | hCV26719117 | rs12144559 | 0.51 | 0.430712711 | 0.5119 |
| hCV31523650 | rs12048930 | hCV26719120 | rs10927040 | 0.51 | 0.430712711 | 0.9368 |
| hCV31523650 | rs12048930 | hCV26719121 | rs10927041 | 0.51 | 0.430712711 | 0.9368 |
| hCV31523650 | rs12048930 | hCV26719137 | rs12136847 | 0.51 | 0.430712711 | 0.5119 |
| hCV31523650 | rs12048930 | hCV26719149 | rs6675851 | 0.51 | 0.430712711 | 0.9681 |
| hCV31523650 | rs12048930 | hCV26719162 | rs4132509 | 0.51 | 0.430712711 | 0.9681 |
| hCV31523650 | rs12048930 | hCV26719163 | rs6429435 | 0.51 | 0.430712711 | 0.5119 |
| hCV31523650 | rs12048930 | hCV26719171 | rs10927075 | 0.51 | 0.430712711 | 0.4932 |
| hCV31523650 | rs12048930 | hCV26719176 | rs10927076 | 0.51 | 0.430712711 | 0.9681 |
| hCV31523650 | rs12048930 | hCV26719179 | rs6672195 | 0.51 | 0.430712711 | 0.5119 |
| hCV31523650 | rs12048930 | hCV26719192 | rs10803161 | 0.51 | 0.430712711 | 0.9274 |
| hCV31523650 | rs12048930 | hCV26719194 | rs10927081 | 0.51 | 0.430712711 | 0.5119 |
| hCV31523650 | rs12048930 | hCV26719197 | rs4590656 | 0.51 | 0.430712711 | 0.5119 |
| hCV31523650 | rs12048930 | hCV26719201 | rs4478795 | 0.51 | 0.430712711 | 0.7041 |
| hCV31523650 | rs12048930 | hCV26719202 | rs4658588 | 0.51 | 0.430712711 | 0.5107 |
| hCV31523650 | rs12048930 | hCV26719215 | rs12144546 | 0.51 | 0.430712711 | 0.5119 |
| hCV31523650 | rs12048930 | hCV26719217 | rs7548254 | 0.51 | 0.430712711 | 0.5119 |
| hCV31523650 | rs12048930 | hCV26719219 | rs9782958 | 0.51 | 0.430712711 | 0.9337 |
| hCV31523650 | rs12048930 | hCV26719222 | rs4553169 | 0.51 | 0.430712711 | 0.9681 |
| hCV31523650 | rs12048930 | hCV26719225 | rs11586029 | 0.51 | 0.430712711 | 0.5119 |
| hCV31523650 | rs12048930 | hCV26719227 | rs10927065 | 0.51 | 0.430712711 | 0.7793 |
| hCV31523650 | rs12048930 | hCV26719232 | rs10803158 | 0.51 | 0.430712711 | 0.9674 |
| hCV31523650 | rs12048930 | hCV26719233 | rs10927067 | 0.51 | 0.430712711 | 0.9681 |
| hCV31523650 | rs12048930 | hCV27170898 | rs12753750 | 0.51 | 0.430712711 | 0.4449 |
| hCV31523650 | rs12048930 | hCV27171350 | rs4430311 | 0.51 | 0.430712711 | 0.4349 |
| hCV31523650 | rs12048930 | hCV27498250 | rs3766673 | 0.51 | 0.430712711 | 0.9071 |
| hCV31523650 | rs12048930 | hCV29210363 | rs6656918 | 0.51 | 0.430712711 | 0.7354 |
| hCV31523650 | rs12048930 | hCV29542869 | rs7534117 | 0.51 | 0.430712711 | 0.9674 |
| hCV31523650 | rs12048930 | hCV29560960 | rs7519673 | 0.51 | 0.430712711 | 0.7842 |
| hCV31523650 | rs12048930 | hCV29741723 | rs7517921 | 0.51 | 0.430712711 | 0.9368 |
| hCV31523650 | rs12048930 | hCV29994467 | rs6694738 | 0.51 | 0.430712711 | 0.7825 |
| hCV31523650 | rs12048930 | hCV30084348 | rs9287269 | 0.51 | 0.430712711 | 0.9681 |
| hCV31523650 | rs12048930 | hCV30372886 | rs9782883 | 0.51 | 0.430712711 | 0.9681 |
| hCV31523650 | rs12048930 | hCV30382231 | rs9428966 | 0.51 | 0.430712711 | 0.489 |
| hCV31523650 | rs12048930 | hCV30690778 | rs12140414 | 0.51 | 0.430712711 | 0.5857 |
| hCV31523650 | rs12048930 | hCV30690780 | rs10737888 | 0.51 | 0.430712711 | 0.7354 |
| hCV31523650 | rs12048930 | hCV31523552 | rs12739344 | 0.51 | 0.430712711 | 0.4932 |

## TABLE 3, page 58 of 71

| Interrogated SNP | Interrogated rs | LD SNP | LD SNP rs | Power | Threshold $r^2$ | $r^2$ |
|---|---|---|---|---|---|---|
| hCV31523650 | rs12048930 | hCV31523557 | rs10754807 | 0.51 | 0.430712711 | 0.9681 |
| hCV31523650 | rs12048930 | hCV31523563 | rs10927051 | 0.51 | 0.430712711 | 1 |
| hCV31523650 | rs12048930 | hCV31523576 | rs12691548 | 0.51 | 0.430712711 | 0.5119 |
| hCV31523650 | rs12048930 | hCV31523624 | rs10927044 | 0.51 | 0.430712711 | 0.4932 |
| hCV31523650 | rs12048930 | hCV31523638 | rs12037013 | 0.51 | 0.430712711 | 0.814 |
| hCV31523650 | rs12048930 | hCV31523639 | rs12034588 | 0.51 | 0.430712711 | 0.9312 |
| hCV31523650 | rs12048930 | hCV31523643 | rs6671475 | 0.51 | 0.430712711 | 0.9368 |
| hCV31523650 | rs12048930 | hCV31523658 | rs12047209 | 0.51 | 0.430712711 | 0.6687 |
| hCV31523650 | rs12048930 | hCV31523688 | rs12049228 | 0.51 | 0.430712711 | 0.9276 |
| hCV31523650 | rs12048930 | hCV31523691 | rs12021907 | 0.51 | 0.430712711 | 0.7842 |
| hCV31523650 | rs12048930 | hCV31523707 | rs10803152 | 0.51 | 0.430712711 | 0.814 |
| hCV31523650 | rs12048930 | hCV31523710 | rs10927059 | 0.51 | 0.430712711 | 0.9681 |
| hCV31523650 | rs12048930 | hCV31523723 | rs12140040 | 0.51 | 0.430712711 | 0.6706 |
| hCV31523650 | rs12048930 | hCV31523736 | rs12124113 | 0.51 | 0.430712711 | 0.7842 |
| hCV31523650 | rs12048930 | hCV31523740 | rs12032342 | 0.51 | 0.430712711 | 0.9314 |
| hCV31523650 | rs12048930 | hCV31523744 | rs12031994 | 0.51 | 0.430712711 | 0.7842 |
| hCV31523650 | rs12048930 | hCV804126 | rs320320 | 0.51 | 0.430712711 | 0.9314 |
| hCV31523650 | rs12048930 | hCV8688098 | rs1531244 | 0.51 | 0.430712711 | 0.4837 |
| hCV31523650 | rs12048930 | hCV8688111 | rs1578275 | 0.51 | 0.430712711 | 0.5771 |
| hCV31523650 | rs12048930 | hCV8688770 | rs3856231 | 0.51 | 0.430712711 | 0.5119 |
| hCV31523650 | rs12048930 | hCV8689016 | rs897959 | 0.51 | 0.430712711 | 0.5119 |
| hCV31523650 | rs12048930 | hCV9115290 | rs1352162 | 0.51 | 0.430712711 | 0.484 |
| hCV31523650 | rs12048930 | hCV9493073 | rs1058305 | 0.51 | 0.430712711 | 0.4963 |
| hCV31523650 | rs12048930 | hCV9493081 | rs1058304 | 0.51 | 0.430712711 | 0.4963 |
| hCV31523650 | rs12048930 | hCV97631 | rs1538773 | 0.51 | 0.430712711 | 0.7354 |
| hCV31523650 | rs12048930 | hDV69368808 | rs12145558 | 0.51 | 0.430712711 | 0.5119 |
| hCV31523650 | rs12048930 | hDV71836703 | rs6429433 | 0.51 | 0.430712711 | 0.7322 |
| hCV31523650 | rs12048930 | hDV90784784 | rs320339 | 0.51 | 0.430712711 | 0.9053 |
| hCV32291301 | rs4253302 | hCV15968025 | rs2292425 | 0.51 | 0.239176625 | 0.382 |
| hCV32291301 | rs4253302 | hCV15968026 | rs2292426 | 0.51 | 0.239176625 | 0.4222 |
| hCV32291301 | rs4253302 | hCV15968034 | rs2292428 | 0.51 | 0.239176625 | 0.3408 |
| hCV32291301 | rs4253302 | hCV15975109 | rs2304596 | 0.51 | 0.239176625 | 1 |
| hCV32291301 | rs4253302 | hCV22272267 | rs3733402 | 0.51 | 0.239176625 | 0.2397 |
| hCV32291301 | rs4253302 | hCV25989001 | hCV25989001 | 0.51 | 0.239176625 | 0.9447 |
| hCV32291301 | rs4253302 | hCV25990131 | rs13146272 | 0.51 | 0.239176625 | 0.3944 |
| hCV32291301 | rs4253302 | hCV27482765 | rs3775301 | 0.51 | 0.239176625 | 1 |
| hCV32291301 | rs4253302 | hCV27902808 | rs4253236 | 0.51 | 0.239176625 | 0.3677 |
| hCV32291301 | rs4253302 | hCV28960679 | rs6844764 | 0.51 | 0.239176625 | 0.2663 |
| hCV32291301 | rs4253302 | hCV29053265 | rs4253244 | 0.51 | 0.239176625 | 0.3178 |
| hCV32291301 | rs4253302 | hCV29718000 | rs4253238 | 0.51 | 0.239176625 | 0.2447 |
| hCV32291301 | rs4253302 | hCV32291217 | rs4253323 | 0.51 | 0.239176625 | 1 |
| hCV32291301 | rs4253302 | hCV32291286 | rs4253422 | 0.51 | 0.239176625 | 0.3885 |
| hCV32291301 | rs4253302 | hCV32291287 | rs4253423 | 0.51 | 0.239176625 | 0.3885 |
| hCV32291301 | rs4253302 | hCV32291295 | rs4253292 | 0.51 | 0.239176625 | 1 |
| hCV32291301 | rs4253302 | hCV32295028 | rs4253260 | 0.51 | 0.239176625 | 1 |
| hCV32291301 | rs4253302 | hCV3229991 | rs4241815 | 0.51 | 0.239176625 | 0.2397 |
| hCV32291301 | rs4253302 | hCV3229992 | rs3775298 | 0.51 | 0.239176625 | 0.2397 |
| hCV32291301 | rs4253302 | hCV3229995 | rs11132382 | 0.51 | 0.239176625 | 0.2447 |
| hCV32291301 | rs4253302 | hCV3230007 | rs4253311 | 0.51 | 0.239176625 | 0.2397 |
| hCV32291301 | rs4253302 | hCV3230031 | rs4253419 | 0.51 | 0.239176625 | 0.3885 |
| hCV32291301 | rs4253302 | hCV3230097 | rs3736455 | 0.51 | 0.239176625 | 0.4296 |
| hCV32291301 | rs4253302 | hCV3230101 | rs6835839 | 0.51 | 0.239176625 | 0.327 |
| hCV32291301 | rs4253302 | hCV3230106 | rs1473597 | 0.51 | 0.239176625 | 0.3407 |
| hCV32291301 | rs4253302 | hCV3230110 | rs2276917 | 0.51 | 0.239176625 | 0.3408 |
| hCV32291301 | rs4253302 | hCV3230118 | rs4253429 | 0.51 | 0.239176625 | 0.3885 |

## TABLE 3, page 59 of 71

| Interrogated SNP | Interrogated rs | LD SNP | LD SNP rs | Power | Threshold $r^2$ | $r^2$ |
|---|---|---|---|---|---|---|
| hCV32291301 | rs4253302 | hCV3230125 | rs11938564 | 0.51 | 0.239176625 | 0.2776 |
| hCV32291301 | rs4253302 | hCV32313006 | rs4253248 | 0.51 | 0.239176625 | 0.2447 |
| hCV32291301 | rs4253302 | hCV32313024 | rs4253239 | 0.51 | 0.239176625 | 1 |
| hCV32291301 | rs4253302 | hCV32358984 | rs4253256 | 0.51 | 0.239176625 | 0.3641 |
| hCV32291301 | rs4253302 | hDV71222711 | rs4253252 | 0.51 | 0.239176625 | 0.2447 |
| hCV3230038 | rs2289252 | hCV11786147 | rs4862662 | 0.51 | 0.044201827 | 0.1313 |
| hCV3230038 | rs2289252 | hCV11786235 | rs4253287 | 0.51 | 0.044201827 | 0.106 |
| hCV3230038 | rs2289252 | hCV11786258 | rs4253303 | 0.51 | 0.044201827 | 0.1956 |
| hCV3230038 | rs2289252 | hCV11786259 | rs4253304 | 0.51 | 0.044201827 | 0.2636 |
| hCV3230038 | rs2289252 | hCV11786295 | rs4253421 | 0.51 | 0.044201827 | 0.075 |
| hCV3230038 | rs2289252 | hCV11786301 | rs5970 | 0.51 | 0.044201827 | 0.0938 |
| hCV3230038 | rs2289252 | hCV11786307 | rs1062547 | 0.51 | 0.044201827 | 0.3739 |
| hCV3230038 | rs2289252 | hCV11786311 | rs13145616 | 0.51 | 0.044201827 | 0.1125 |
| hCV3230038 | rs2289252 | hCV11786327 | rs13133050 | 0.51 | 0.044201827 | 0.1784 |
| hCV3230038 | rs2289252 | hCV12066116 | rs1877320 | 0.51 | 0.044201827 | 0.0748 |
| hCV3230038 | rs2289252 | hCV12066118 | rs2048 | 0.51 | 0.044201827 | 0.1136 |
| hCV3230038 | rs2289252 | hCV12066119 | rs1912826 | 0.51 | 0.044201827 | 0.1027 |
| hCV3230038 | rs2289252 | hCV12066124 | rs2036914 | 0.51 | 0.044201827 | 0.3834 |
| hCV3230038 | rs2289252 | hCV12066129 | rs1593 | 0.51 | 0.044201827 | 0.0795 |
| hCV3230038 | rs2289252 | hCV1333083 | rs10022988 | 0.51 | 0.044201827 | 0.0488 |
| hCV3230038 | rs2289252 | hCV1333090 | rs6816112 | 0.51 | 0.044201827 | 0.0764 |
| hCV3230038 | rs2289252 | hCV1333097 | rs4862680 | 0.51 | 0.044201827 | 0.0488 |
| hCV3230038 | rs2289252 | hCV1333099 | rs10020635 | 0.51 | 0.044201827 | 0.0659 |
| hCV3230038 | rs2289252 | hCV15793897 | rs3087505 | 0.51 | 0.044201827 | 0.0486 |
| hCV3230038 | rs2289252 | hCV15811716 | rs2102575 | 0.51 | 0.044201827 | 0.0448 |
| hCV3230038 | rs2289252 | hCV15968025 | rs2292425 | 0.51 | 0.044201827 | 0.0893 |
| hCV3230038 | rs2289252 | hCV15968026 | rs2292426 | 0.51 | 0.044201827 | 0.181 |
| hCV3230038 | rs2289252 | hCV15968034 | rs2292428 | 0.51 | 0.044201827 | 0.0718 |
| hCV3230038 | rs2289252 | hCV15968043 | rs2292423 | 0.51 | 0.044201827 | 0.2462 |
| hCV3230038 | rs2289252 | hCV16172925 | rs2241818 | 0.51 | 0.044201827 | 0.1263 |
| hCV3230038 | rs2289252 | hCV16172935 | rs2241817 | 0.51 | 0.044201827 | 0.3937 |
| hCV3230038 | rs2289252 | hCV194962 | rs6552954 | 0.51 | 0.044201827 | 0.0482 |
| hCV3230038 | rs2289252 | hCV2103343 | rs4241824 | 0.51 | 0.044201827 | 0.4188 |
| hCV3230038 | rs2289252 | hCV2103388 | rs4613610 | 0.51 | 0.044201827 | 0.1193 |
| hCV3230038 | rs2289252 | hCV2103391 | rs1008728 | 0.51 | 0.044201827 | 0.2177 |
| hCV3230038 | rs2289252 | hCV2103392 | rs12500826 | 0.51 | 0.044201827 | 0.3222 |
| hCV3230038 | rs2289252 | hCV22272267 | rs3733402 | 0.51 | 0.044201827 | 0.1192 |
| hCV3230038 | rs2289252 | hCV25474413 | rs3822057 | 0.51 | 0.044201827 | 0.4122 |
| hCV3230038 | rs2289252 | hCV25474414 | rs4253399 | 0.51 | 0.044201827 | 0.7079 |
| hCV3230038 | rs2289252 | hCV25634754 | rs4253331 | 0.51 | 0.044201827 | 0.0636 |
| hCV3230038 | rs2289252 | hCV25988221 | rs9995366 | 0.51 | 0.044201827 | 0.0512 |
| hCV3230038 | rs2289252 | hCV25990131 | rs13146272 | 0.51 | 0.044201827 | 0.0944 |
| hCV3230038 | rs2289252 | hCV26038139 | rs4253405 | 0.51 | 0.044201827 | 0.2621 |
| hCV3230038 | rs2289252 | hCV26265231 | rs7684025 | 0.51 | 0.044201827 | 0.1744 |
| hCV3230038 | rs2289252 | hCV27309972 | rs13101296 | 0.51 | 0.044201827 | 0.1074 |
| hCV3230038 | rs2289252 | hCV27309991 | rs4572916 | 0.51 | 0.044201827 | 0.1232 |
| hCV3230038 | rs2289252 | hCV27474895 | rs3756011 | 0.51 | 0.044201827 | 1 |
| hCV3230038 | rs2289252 | hCV27477533 | rs3756008 | 0.51 | 0.044201827 | 0.7249 |
| hCV3230038 | rs2289252 | hCV27490984 | rs3822058 | 0.51 | 0.044201827 | 0.4054 |
| hCV3230038 | rs2289252 | hCV27521729 | rs3822056 | 0.51 | 0.044201827 | 0.0849 |
| hCV3230038 | rs2289252 | hCV27902803 | rs4862665 | 0.51 | 0.044201827 | 0.0512 |
| hCV3230038 | rs2289252 | hCV28960679 | rs6844764 | 0.51 | 0.044201827 | 0.1196 |
| hCV3230038 | rs2289252 | hCV29053261 | rs6842047 | 0.51 | 0.044201827 | 0.0472 |
| hCV3230038 | rs2289252 | hCV29053264 | rs7667777 | 0.51 | 0.044201827 | 0.1852 |
| hCV3230038 | rs2289252 | hCV29640635 | rs10029715 | 0.51 | 0.044201827 | 0.0679 |

| Interrogated SNP | Interrogated rs | LD SNP | LD SNP rs | Power | Threshold r² | r² |
|---|---|---|---|---|---|---|
| hCV3230038 | rs2289252 | hCV29718000 | rs4253238 | 0.51 | 0.044201827 | 0.1009 |
| hCV3230038 | rs2289252 | hCV29826351 | rs10025990 | 0.51 | 0.044201827 | 0.0882 |
| hCV3230038 | rs2289252 | hCV29877725 | rs4253295 | 0.51 | 0.044201827 | 0.1339 |
| hCV3230038 | rs2289252 | hCV30307525 | rs10025152 | 0.51 | 0.044201827 | 0.0679 |
| hCV3230038 | rs2289252 | hCV30492573 | rs10471184 | 0.51 | 0.044201827 | 0.0472 |
| hCV3230038 | rs2289252 | hCV30983902 | rs4862668 | 0.51 | 0.044201827 | 0.0748 |
| hCV3230038 | rs2289252 | hCV30983927 | rs6552962 | 0.51 | 0.044201827 | 0.0784 |
| hCV3230038 | rs2289252 | hCV32209629 | rs12715865 | 0.51 | 0.044201827 | 0.1373 |
| hCV3230038 | rs2289252 | hCV32209636 | rs11132387 | 0.51 | 0.044201827 | 0.435 |
| hCV3230038 | rs2289252 | hCV32209637 | rs13143773 | 0.51 | 0.044201827 | 0.2331 |
| hCV3230038 | rs2289252 | hCV32209638 | rs12507040 | 0.51 | 0.044201827 | 0.2973 |
| hCV3230038 | rs2289252 | hCV32291256 | rs4253406 | 0.51 | 0.044201827 | 0.0781 |
| hCV3230038 | rs2289252 | hCV32291269 | rs4253417 | 0.51 | 0.044201827 | 0.9433 |
| hCV3230038 | rs2289252 | hCV32291286 | rs4253422 | 0.51 | 0.044201827 | 0.1539 |
| hCV3230038 | rs2289252 | hCV32291287 | rs4253423 | 0.51 | 0.044201827 | 0.1539 |
| hCV3230038 | rs2289252 | hCV3229991 | rs4241815 | 0.51 | 0.044201827 | 0.1192 |
| hCV3230038 | rs2289252 | hCV3229992 | rs3775298 | 0.51 | 0.044201827 | 0.1192 |
| hCV3230038 | rs2289252 | hCV3229995 | rs11132382 | 0.51 | 0.044201827 | 0.0979 |
| hCV3230038 | rs2289252 | hCV3230002 | rs4253297 | 0.51 | 0.044201827 | 0.2015 |
| hCV3230038 | rs2289252 | hCV3230003 | rs2304595 | 0.51 | 0.044201827 | 0.2003 |
| hCV3230038 | rs2289252 | hCV3230006 | rs4253308 | 0.51 | 0.044201827 | 0.1339 |
| hCV3230038 | rs2289252 | hCV3230007 | rs4253311 | 0.51 | 0.044201827 | 0.1192 |
| hCV3230038 | rs2289252 | hCV3230010 | rs4253315 | 0.51 | 0.044201827 | 0.0748 |
| hCV3230038 | rs2289252 | hCV3230011 | rs4253320 | 0.51 | 0.044201827 | 0.2015 |
| hCV3230038 | rs2289252 | hCV3230013 | rs3775303 | 0.51 | 0.044201827 | 0.2636 |
| hCV3230038 | rs2289252 | hCV3230016 | rs4253325 | 0.51 | 0.044201827 | 0.0662 |
| hCV3230038 | rs2289252 | hCV3230017 | rs4253327 | 0.51 | 0.044201827 | 0.0771 |
| hCV3230038 | rs2289252 | hCV3230021 | rs13135645 | 0.51 | 0.044201827 | 0.0564 |
| hCV3230038 | rs2289252 | hCV3230022 | rs11132383 | 0.51 | 0.044201827 | 0.2455 |
| hCV3230038 | rs2289252 | hCV3230025 | rs3756009 | 0.51 | 0.044201827 | 0.7937 |
| hCV3230038 | rs2289252 | hCV3230030 | rs4253408 | 0.51 | 0.044201827 | 0.0719 |
| hCV3230038 | rs2289252 | hCV3230031 | rs4253419 | 0.51 | 0.044201827 | 0.1539 |
| hCV3230038 | rs2289252 | hCV3230032 | rs5974 | 0.51 | 0.044201827 | 0.1125 |
| hCV3230038 | rs2289252 | hCV3230083 | rs10013653 | 0.51 | 0.044201827 | 0.2181 |
| hCV3230038 | rs2289252 | hCV3230084 | rs7682918 | 0.51 | 0.044201827 | 0.1434 |
| hCV3230038 | rs2289252 | hCV3230094 | rs7687818 | 0.51 | 0.044201827 | 0.2201 |
| hCV3230038 | rs2289252 | hCV3230096 | rs3817184 | 0.51 | 0.044201827 | 0.1484 |
| hCV3230038 | rs2289252 | hCV3230097 | rs3736455 | 0.51 | 0.044201827 | 0.1419 |
| hCV3230038 | rs2289252 | hCV3230101 | rs6835839 | 0.51 | 0.044201827 | 0.0447 |
| hCV3230038 | rs2289252 | hCV3230106 | rs1473597 | 0.51 | 0.044201827 | 0.0873 |
| hCV3230038 | rs2289252 | hCV3230110 | rs2276917 | 0.51 | 0.044201827 | 0.0803 |
| hCV3230038 | rs2289252 | hCV3230113 | rs1053094 | 0.51 | 0.044201827 | 0.1432 |
| hCV3230038 | rs2289252 | hCV3230118 | rs4253429 | 0.51 | 0.044201827 | 0.1539 |
| hCV3230038 | rs2289252 | hCV3230119 | rs4253430 | 0.51 | 0.044201827 | 0.3973 |
| hCV3230038 | rs2289252 | hCV3230121 | rs4253431 | 0.51 | 0.044201827 | 0.0887 |
| hCV3230038 | rs2289252 | hCV3230125 | rs11938564 | 0.51 | 0.044201827 | 0.2052 |
| hCV3230038 | rs2289252 | hCV3230131 | rs13136269 | 0.51 | 0.044201827 | 0.2973 |
| hCV3230038 | rs2289252 | hCV3230133 | rs12511874 | 0.51 | 0.044201827 | 0.2104 |
| hCV3230038 | rs2289252 | hCV3230134 | rs12500151 | 0.51 | 0.044201827 | 0.3043 |
| hCV3230038 | rs2289252 | hCV3230136 | rs13116273 | 0.51 | 0.044201827 | 0.2952 |
| hCV3230038 | rs2289252 | hCV32313006 | rs4253248 | 0.51 | 0.044201827 | 0.1068 |
| hCV3230038 | rs2289252 | hCV32313007 | rs4862666 | 0.51 | 0.044201827 | 0.0512 |
| hCV3230038 | rs2289252 | hCV32313014 | rs4253243 | 0.51 | 0.044201827 | 0.0636 |
| hCV3230038 | rs2289252 | hCV32358975 | rs4253255 | 0.51 | 0.044201827 | 0.1152 |
| hCV3230038 | rs2289252 | hCV32358984 | rs4253256 | 0.51 | 0.044201827 | 0.0489 |

## TABLE 3, page 61 of 71

| Interrogated SNP | Interrogated rs | LD SNP | LD SNP rs | Power | Threshold r² | r² |
|---|---|---|---|---|---|---|
| hCV3230038 | rs2289252 | hCV8241628 | rs907439 | 0.51 | 0.044201827 | 0.1232 |
| hCV3230038 | rs2289252 | hCV8241630 | rs925451 | 0.51 | 0.044201827 | 0.7423 |
| hCV3230038 | rs2289252 | hCV8241631 | rs1511802 | 0.51 | 0.044201827 | 0.1452 |
| hCV3230038 | rs2289252 | hCV8241632 | rs1511801 | 0.51 | 0.044201827 | 0.1183 |
| hCV3230038 | rs2289252 | hCV8241633 | rs1511800 | 0.51 | 0.044201827 | 0.0512 |
| hCV3230038 | rs2289252 | hDV68550952 | rs4253289 | 0.51 | 0.044201827 | 0.0632 |
| hCV3230038 | rs2289252 | hDV71222711 | rs4253252 | 0.51 | 0.044201827 | 0.1068 |
| hCV3230096 | rs3817184 | hCV11786147 | rs4862662 | 0.51 | 0.10562155 | 0.9607 |
| hCV3230096 | rs3817184 | hCV11786235 | rs4253287 | 0.51 | 0.10562155 | 0.1611 |
| hCV3230096 | rs3817184 | hCV11786258 | rs4253303 | 0.51 | 0.10562155 | 0.7346 |
| hCV3230096 | rs3817184 | hCV11786259 | rs4253304 | 0.51 | 0.10562155 | 0.6556 |
| hCV3230096 | rs3817184 | hCV12066106 | rs1914926 | 0.51 | 0.10562155 | 0.1148 |
| hCV3230096 | rs3817184 | hCV12066118 | rs2048 | 0.51 | 0.10562155 | 0.3722 |
| hCV3230096 | rs3817184 | hCV12066119 | rs1912826 | 0.51 | 0.10562155 | 0.3754 |
| hCV3230096 | rs3817184 | hCV12066124 | rs2036914 | 0.51 | 0.10562155 | 0.2824 |
| hCV3230096 | rs3817184 | hCV15968025 | rs2292425 | 0.51 | 0.10562155 | 0.414 |
| hCV3230096 | rs3817184 | hCV15968026 | rs2292426 | 0.51 | 0.10562155 | 0.3737 |
| hCV3230096 | rs3817184 | hCV15968034 | rs2292428 | 0.51 | 0.10562155 | 0.4839 |
| hCV3230096 | rs3817184 | hCV15968043 | rs2292423 | 0.51 | 0.10562155 | 0.6453 |
| hCV3230096 | rs3817184 | hCV15975109 | rs2304596 | 0.51 | 0.10562155 | 0.1582 |
| hCV3230096 | rs3817184 | hCV2103343 | rs4241824 | 0.51 | 0.10562155 | 0.2431 |
| hCV3230096 | rs3817184 | hCV22272267 | rs3733402 | 0.51 | 0.10562155 | 0.3722 |
| hCV3230096 | rs3817184 | hCV25474413 | rs3822057 | 0.51 | 0.10562155 | 0.2412 |
| hCV3230096 | rs3817184 | hCV25474414 | rs4253399 | 0.51 | 0.10562155 | 0.1977 |
| hCV3230096 | rs3817184 | hCV25989001 | hCV25989001 | 0.51 | 0.10562155 | 0.1672 |
| hCV3230096 | rs3817184 | hCV25990131 | rs13146272 | 0.51 | 0.10562155 | 0.4248 |
| hCV3230096 | rs3817184 | hCV26038139 | rs4253405 | 0.51 | 0.10562155 | 0.1427 |
| hCV3230096 | rs3817184 | hCV26265231 | rs7684025 | 0.51 | 0.10562155 | 0.7723 |
| hCV3230096 | rs3817184 | hCV27474895 | rs3756011 | 0.51 | 0.10562155 | 0.1852 |
| hCV3230096 | rs3817184 | hCV27477533 | rs3756008 | 0.51 | 0.10562155 | 0.2279 |
| hCV3230096 | rs3817184 | hCV27482765 | rs3775301 | 0.51 | 0.10562155 | 0.1582 |
| hCV3230096 | rs3817184 | hCV27902808 | rs4253236 | 0.51 | 0.10562155 | 0.2369 |
| hCV3230096 | rs3817184 | hCV28960679 | rs6844764 | 0.51 | 0.10562155 | 0.4298 |
| hCV3230096 | rs3817184 | hCV29053260 | rs4861707 | 0.51 | 0.10562155 | 0.2941 |
| hCV3230096 | rs3817184 | hCV29053264 | rs7667777 | 0.51 | 0.10562155 | 1 |
| hCV3230096 | rs3817184 | hCV29053265 | rs4253244 | 0.51 | 0.10562155 | 0.2244 |
| hCV3230096 | rs3817184 | hCV29053266 | rs7687961 | 0.51 | 0.10562155 | 0.1405 |
| hCV3230096 | rs3817184 | hCV29053271 | rs6814261 | 0.51 | 0.10562155 | 0.1124 |
| hCV3230096 | rs3817184 | hCV29718000 | rs4253238 | 0.51 | 0.10562155 | 0.3705 |
| hCV3230096 | rs3817184 | hCV29877725 | rs4253295 | 0.51 | 0.10562155 | 0.7382 |
| hCV3230096 | rs3817184 | hCV30983927 | rs6552962 | 0.51 | 0.10562155 | 0.1582 |
| hCV3230096 | rs3817184 | hCV32209636 | rs11132387 | 0.51 | 0.10562155 | 0.1797 |
| hCV3230096 | rs3817184 | hCV32209638 | rs12507040 | 0.51 | 0.10562155 | 0.1135 |
| hCV3230096 | rs3817184 | hCV32291217 | rs4253323 | 0.51 | 0.10562155 | 0.1582 |
| hCV3230096 | rs3817184 | hCV32291269 | rs4253417 | 0.51 | 0.10562155 | 0.1798 |
| hCV3230096 | rs3817184 | hCV32291295 | rs4253292 | 0.51 | 0.10562155 | 0.1783 |
| hCV3230096 | rs3817184 | hCV32291301 | rs4253302 | 0.51 | 0.10562155 | 0.1573 |
| hCV3230096 | rs3817184 | hCV32295028 | rs4253260 | 0.51 | 0.10562155 | 0.1582 |
| hCV3230096 | rs3817184 | hCV3229991 | rs4241815 | 0.51 | 0.10562155 | 0.3722 |
| hCV3230096 | rs3817184 | hCV3229992 | rs3775298 | 0.51 | 0.10562155 | 0.3722 |
| hCV3230096 | rs3817184 | hCV3229995 | rs11132382 | 0.51 | 0.10562155 | 0.3745 |
| hCV3230096 | rs3817184 | hCV3230000 | rs4253294 | 0.51 | 0.10562155 | 0.1467 |
| hCV3230096 | rs3817184 | hCV3230002 | rs4253297 | 0.51 | 0.10562155 | 0.7524 |
| hCV3230096 | rs3817184 | hCV3230003 | rs2304595 | 0.51 | 0.10562155 | 0.6237 |
| hCV3230096 | rs3817184 | hCV3230006 | rs4253308 | 0.51 | 0.10562155 | 0.7382 |

# TABLE 3, page 62 of 71

| Interrogated SNP | Interrogated rs | LD SNP | LD SNP rs | Power | Threshold r² | r² |
|---|---|---|---|---|---|---|
| hCV3230096 | rs3817184 | hCV3230007 | rs4253311 | 0.51 | 0.10562155 | 0.3722 |
| hCV3230096 | rs3817184 | hCV3230011 | rs4253320 | 0.51 | 0.10562155 | 0.7524 |
| hCV3230096 | rs3817184 | hCV3230013 | rs3775303 | 0.51 | 0.10562155 | 0.6556 |
| hCV3230096 | rs3817184 | hCV3230014 | rs4861709 | 0.51 | 0.10562155 | 0.1467 |
| hCV3230096 | rs3817184 | hCV3230017 | rs4253327 | 0.51 | 0.10562155 | 0.207 |
| hCV3230096 | rs3817184 | hCV3230018 | rs925453 | 0.51 | 0.10562155 | 0.1144 |
| hCV3230096 | rs3817184 | hCV3230019 | rs4253332 | 0.51 | 0.10562155 | 0.1092 |
| hCV3230096 | rs3817184 | hCV3230022 | rs11132383 | 0.51 | 0.10562155 | 0.2117 |
| hCV3230096 | rs3817184 | hCV3230025 | rs3756009 | 0.51 | 0.10562155 | 0.2784 |
| hCV3230096 | rs3817184 | hCV3230038 | rs2289252 | 0.51 | 0.10562155 | 0.1484 |
| hCV3230096 | rs3817184 | hCV3230079 | rs35641294 | 0.51 | 0.10562155 | 0.1147 |
| hCV3230096 | rs3817184 | hCV3230083 | rs10013653 | 0.51 | 0.10562155 | 0.7047 |
| hCV3230096 | rs3817184 | hCV3230084 | rs7682918 | 0.51 | 0.10562155 | 0.8657 |
| hCV3230096 | rs3817184 | hCV3230094 | rs7687818 | 0.51 | 0.10562155 | 0.8722 |
| hCV3230096 | rs3817184 | hCV3230097 | rs3736455 | 0.51 | 0.10562155 | 0.367 |
| hCV3230096 | rs3817184 | hCV3230101 | rs6835839 | 0.51 | 0.10562155 | 0.451 |
| hCV3230096 | rs3817184 | hCV3230106 | rs1473597 | 0.51 | 0.10562155 | 0.4966 |
| hCV3230096 | rs3817184 | hCV3230110 | rs2276917 | 0.51 | 0.10562155 | 0.4746 |
| hCV3230096 | rs3817184 | hCV3230113 | rs1053094 | 0.51 | 0.10562155 | 0.695 |
| hCV3230096 | rs3817184 | hCV3230125 | rs11938564 | 0.51 | 0.10562155 | 0.1059 |
| hCV3230096 | rs3817184 | hCV3230131 | rs13136269 | 0.51 | 0.10562155 | 0.1135 |
| hCV3230096 | rs3817184 | hCV3230134 | rs12500151 | 0.51 | 0.10562155 | 0.1188 |
| hCV3230096 | rs3817184 | hCV32313006 | rs4253248 | 0.51 | 0.10562155 | 0.3803 |
| hCV3230096 | rs3817184 | hCV32313024 | rs4253239 | 0.51 | 0.10562155 | 0.1783 |
| hCV3230096 | rs3817184 | hCV32358975 | rs4253255 | 0.51 | 0.10562155 | 0.3585 |
| hCV3230096 | rs3817184 | hCV32358984 | rs4253256 | 0.51 | 0.10562155 | 0.2382 |
| hCV3230096 | rs3817184 | hCV8241630 | rs925451 | 0.51 | 0.10562155 | 0.2184 |
| hCV3230096 | rs3817184 | hCV8241631 | rs1511802 | 0.51 | 0.10562155 | 0.7356 |
| hCV3230096 | rs3817184 | hCV8241632 | rs1511801 | 0.51 | 0.10562155 | 0.4035 |
| hCV3230096 | rs3817184 | hDV71222711 | rs4253252 | 0.51 | 0.10562155 | 0.3803 |
| hCV3230096 | rs3817184 | hDV76175111 | rs35079309 | 0.51 | 0.10562155 | 0.1765 |
| hCV3230113 | rs1053094 | hCV11786002 | rs4862633 | 0.51 | 0.086445499 | 0.1657 |
| hCV3230113 | rs1053094 | hCV11786003 | rs4608848 | 0.51 | 0.086445499 | 0.1129 |
| hCV3230113 | rs1053094 | hCV11786022 | rs2090628 | 0.51 | 0.086445499 | 0.0928 |
| hCV3230113 | rs1053094 | hCV11786028 | rs6848963 | 0.51 | 0.086445499 | 0.1295 |
| hCV3230113 | rs1053094 | hCV11786147 | rs4862662 | 0.51 | 0.086445499 | 0.6694 |
| hCV3230113 | rs1053094 | hCV11786203 | rs4253251 | 0.51 | 0.086445499 | 0.0997 |
| hCV3230113 | rs1053094 | hCV11786258 | rs4253303 | 0.51 | 0.086445499 | 0.491 |
| hCV3230113 | rs1053094 | hCV11786259 | rs4253304 | 0.51 | 0.086445499 | 0.579 |
| hCV3230113 | rs1053094 | hCV11786307 | rs1062547 | 0.51 | 0.086445499 | 0.1175 |
| hCV3230113 | rs1053094 | hCV11786327 | rs13133050 | 0.51 | 0.086445499 | 0.1384 |
| hCV3230113 | rs1053094 | hCV12066105 | rs1519309 | 0.51 | 0.086445499 | 0.1053 |
| hCV3230113 | rs1053094 | hCV12066106 | rs1914926 | 0.51 | 0.086445499 | 0.1419 |
| hCV3230113 | rs1053094 | hCV12066116 | rs1877320 | 0.51 | 0.086445499 | 0.1094 |
| hCV3230113 | rs1053094 | hCV12066118 | rs2048 | 0.51 | 0.086445499 | 0.5759 |
| hCV3230113 | rs1053094 | hCV12066119 | rs1912826 | 0.51 | 0.086445499 | 0.5742 |
| hCV3230113 | rs1053094 | hCV12066124 | rs2036914 | 0.51 | 0.086445499 | 0.3142 |
| hCV3230113 | rs1053094 | hCV15811716 | rs2102575 | 0.51 | 0.086445499 | 0.1014 |
| hCV3230113 | rs1053094 | hCV1589303 | rs11730434 | 0.51 | 0.086445499 | 0.1249 |
| hCV3230113 | rs1053094 | hCV1589308 | rs9998530 | 0.51 | 0.086445499 | 0.1249 |
| hCV3230113 | rs1053094 | hCV15968025 | rs2292425 | 0.51 | 0.086445499 | 0.1645 |
| hCV3230113 | rs1053094 | hCV15968026 | rs2292426 | 0.51 | 0.086445499 | 0.2198 |
| hCV3230113 | rs1053094 | hCV15968034 | rs2292428 | 0.51 | 0.086445499 | 0.6489 |
| hCV3230113 | rs1053094 | hCV15968043 | rs2292423 | 0.51 | 0.086445499 | 0.59 |
| hCV3230113 | rs1053094 | hCV15975109 | rs2304596 | 0.51 | 0.086445499 | 0.2245 |

# TABLE 3, page 63 of 71

| Interrogated SNP | Interrogated rs | LD SNP | LD SNP rs | Power | Threshold r² | r² |
|---|---|---|---|---|---|---|
| hCV3230113 | rs1053094 | hCV16172925 | rs2241818 | 0.51 | 0.086445499 | 0.0959 |
| hCV3230113 | rs1053094 | hCV16172935 | rs2241817 | 0.51 | 0.086445499 | 0.0973 |
| hCV3230113 | rs1053094 | hCV2103337 | rs13102931 | 0.51 | 0.086445499 | 0.0958 |
| hCV3230113 | rs1053094 | hCV2103343 | rs4241824 | 0.51 | 0.086445499 | 0.2774 |
| hCV3230113 | rs1053094 | hCV2103391 | rs1008728 | 0.51 | 0.086445499 | 0.1352 |
| hCV3230113 | rs1053094 | hCV2103392 | rs12500826 | 0.51 | 0.086445499 | 0.1352 |
| hCV3230113 | rs1053094 | hCV2103402 | rs9993749 | 0.51 | 0.086445499 | 0.115 |
| hCV3230113 | rs1053094 | hCV22271609 | rs4253326 | 0.51 | 0.086445499 | 0.1496 |
| hCV3230113 | rs1053094 | hCV22272267 | rs3733402 | 0.51 | 0.086445499 | 0.5831 |
| hCV3230113 | rs1053094 | hCV25474413 | rs3822057 | 0.51 | 0.086445499 | 0.2648 |
| hCV3230113 | rs1053094 | hCV25474414 | rs4253399 | 0.51 | 0.086445499 | 0.2252 |
| hCV3230113 | rs1053094 | hCV25634763 | rs4253241 | 0.51 | 0.086445499 | 0.1111 |
| hCV3230113 | rs1053094 | hCV25634781 | rs4253299 | 0.51 | 0.086445499 | 0.1041 |
| hCV3230113 | rs1053094 | hCV25988221 | rs9995366 | 0.51 | 0.086445499 | 0.1117 |
| hCV3230113 | rs1053094 | hCV25989001 | hCV25989001 | 0.51 | 0.086445499 | 0.2371 |
| hCV3230113 | rs1053094 | hCV25990131 | rs13146272 | 0.51 | 0.086445499 | 0.1593 |
| hCV3230113 | rs1053094 | hCV26038139 | rs4253405 | 0.51 | 0.086445499 | 0.1405 |
| hCV3230113 | rs1053094 | hCV26265197 | rs10014399 | 0.51 | 0.086445499 | 0.096 |
| hCV3230113 | rs1053094 | hCV26265199 | rs2221843 | 0.51 | 0.086445499 | 0.1041 |
| hCV3230113 | rs1053094 | hCV26265231 | rs7684025 | 0.51 | 0.086445499 | 0.7049 |
| hCV3230113 | rs1053094 | hCV27310170 | rs4862644 | 0.51 | 0.086445499 | 0.1611 |
| hCV3230113 | rs1053094 | hCV27310180 | rs11722584 | 0.51 | 0.086445499 | 0.1383 |
| hCV3230113 | rs1053094 | hCV27310216 | rs10018625 | 0.51 | 0.086445499 | 0.102 |
| hCV3230113 | rs1053094 | hCV27310218 | rs9992614 | 0.51 | 0.086445499 | 0.0932 |
| hCV3230113 | rs1053094 | hCV27310253 | rs13108688 | 0.51 | 0.086445499 | 0.1242 |
| hCV3230113 | rs1053094 | hCV27310255 | rs7657186 | 0.51 | 0.086445499 | 0.1279 |
| hCV3230113 | rs1053094 | hCV27474895 | rs3756011 | 0.51 | 0.086445499 | 0.1126 |
| hCV3230113 | rs1053094 | hCV27477533 | rs3756008 | 0.51 | 0.086445499 | 0.2208 |
| hCV3230113 | rs1053094 | hCV27482765 | rs3775301 | 0.51 | 0.086445499 | 0.2245 |
| hCV3230113 | rs1053094 | hCV27490984 | rs3822058 | 0.51 | 0.086445499 | 0.096 |
| hCV3230113 | rs1053094 | hCV27506149 | rs3822055 | 0.51 | 0.086445499 | 0.1041 |
| hCV3230113 | rs1053094 | hCV27902803 | rs4862665 | 0.51 | 0.086445499 | 0.1117 |
| hCV3230113 | rs1053094 | hCV27902808 | rs4253236 | 0.51 | 0.086445499 | 0.3483 |
| hCV3230113 | rs1053094 | hCV28960679 | rs6844764 | 0.51 | 0.086445499 | 0.1995 |
| hCV3230113 | rs1053094 | hCV29053260 | rs4861707 | 0.51 | 0.086445499 | 0.0954 |
| hCV3230113 | rs1053094 | hCV29053261 | rs6842047 | 0.51 | 0.086445499 | 0.1117 |
| hCV3230113 | rs1053094 | hCV29053264 | rs7667777 | 0.51 | 0.086445499 | 0.6901 |
| hCV3230113 | rs1053094 | hCV29053265 | rs4253244 | 0.51 | 0.086445499 | 0.3493 |
| hCV3230113 | rs1053094 | hCV29053271 | rs6814261 | 0.51 | 0.086445499 | 0.0876 |
| hCV3230113 | rs1053094 | hCV29718000 | rs4253238 | 0.51 | 0.086445499 | 0.552 |
| hCV3230113 | rs1053094 | hCV29877725 | rs4253295 | 0.51 | 0.086445499 | 0.5019 |
| hCV3230113 | rs1053094 | hCV30492573 | rs10471184 | 0.51 | 0.086445499 | 0.1117 |
| hCV3230113 | rs1053094 | hCV30983902 | rs4862668 | 0.51 | 0.086445499 | 0.1111 |
| hCV3230113 | rs1053094 | hCV30983907 | rs4253246 | 0.51 | 0.086445499 | 0.1111 |
| hCV3230113 | rs1053094 | hCV32209636 | rs11132387 | 0.51 | 0.086445499 | 0.1091 |
| hCV3230113 | rs1053094 | hCV32209637 | rs13143773 | 0.51 | 0.086445499 | 0.096 |
| hCV3230113 | rs1053094 | hCV32209638 | rs12507040 | 0.51 | 0.086445499 | 0.0986 |
| hCV3230113 | rs1053094 | hCV32209919 | rs11730526 | 0.51 | 0.086445499 | 0.1298 |
| hCV3230113 | rs1053094 | hCV32209928 | rs13148663 | 0.51 | 0.086445499 | 0.1277 |
| hCV3230113 | rs1053094 | hCV32291217 | rs4253323 | 0.51 | 0.086445499 | 0.2245 |
| hCV3230113 | rs1053094 | hCV32291269 | rs4253417 | 0.51 | 0.086445499 | 0.1699 |
| hCV3230113 | rs1053094 | hCV32291286 | rs4253422 | 0.51 | 0.086445499 | 0.2024 |
| hCV3230113 | rs1053094 | hCV32291287 | rs4253423 | 0.51 | 0.086445499 | 0.2024 |
| hCV3230113 | rs1053094 | hCV32291295 | rs4253292 | 0.51 | 0.086445499 | 0.2276 |
| hCV3230113 | rs1053094 | hCV32291301 | rs4253302 | 0.51 | 0.086445499 | 0.224 |

| Interrogated SNP | Interrogated rs | LD SNP | LD SNP rs | Power | Threshold r² | r² |
|---|---|---|---|---|---|---|
| hCV3230113 | rs1053094 | hCV32295028 | rs4253260 | 0.51 | 0.086445499 | 0.2245 |
| hCV3230113 | rs1053094 | hCV3229991 | rs4241815 | 0.51 | 0.086445499 | 0.5831 |
| hCV3230113 | rs1053094 | hCV3229992 | rs3775298 | 0.51 | 0.086445499 | 0.5831 |
| hCV3230113 | rs1053094 | hCV3229995 | rs11132382 | 0.51 | 0.086445499 | 0.552 |
| hCV3230113 | rs1053094 | hCV3230000 | rs4253294 | 0.51 | 0.086445499 | 0.2474 |
| hCV3230113 | rs1053094 | hCV3230001 | rs4253296 | 0.51 | 0.086445499 | 0.1111 |
| hCV3230113 | rs1053094 | hCV3230002 | rs4253297 | 0.51 | 0.086445499 | 0.5144 |
| hCV3230113 | rs1053094 | hCV3230003 | rs2304595 | 0.51 | 0.086445499 | 0.573 |
| hCV3230113 | rs1053094 | hCV3230004 | rs4253301 | 0.51 | 0.086445499 | 0.1014 |
| hCV3230113 | rs1053094 | hCV3230006 | rs4253308 | 0.51 | 0.086445499 | 0.5019 |
| hCV3230113 | rs1053094 | hCV3230007 | rs4253311 | 0.51 | 0.086445499 | 0.5831 |
| hCV3230113 | rs1053094 | hCV3230011 | rs4253320 | 0.51 | 0.086445499 | 0.5144 |
| hCV3230113 | rs1053094 | hCV3230012 | rs4241821 | 0.51 | 0.086445499 | 0.1041 |
| hCV3230113 | rs1053094 | hCV3230013 | rs3775303 | 0.51 | 0.086445499 | 0.579 |
| hCV3230113 | rs1053094 | hCV3230014 | rs4861709 | 0.51 | 0.086445499 | 0.2474 |
| hCV3230113 | rs1053094 | hCV3230017 | rs4253327 | 0.51 | 0.086445499 | 0.1179 |
| hCV3230113 | rs1053094 | hCV3230018 | rs925453 | 0.51 | 0.086445499 | 0.2496 |
| hCV3230113 | rs1053094 | hCV3230019 | rs4253332 | 0.51 | 0.086445499 | 0.2496 |
| hCV3230113 | rs1053094 | hCV3230022 | rs11132383 | 0.51 | 0.086445499 | 0.1336 |
| hCV3230113 | rs1053094 | hCV3230025 | rs3756009 | 0.51 | 0.086445499 | 0.1859 |
| hCV3230113 | rs1053094 | hCV3230031 | rs4253419 | 0.51 | 0.086445499 | 0.2024 |
| hCV3230113 | rs1053094 | hCV3230038 | rs2289252 | 0.51 | 0.086445499 | 0.1432 |
| hCV3230113 | rs1053094 | hCV3230079 | rs35641294 | 0.51 | 0.086445499 | 0.0879 |
| hCV3230113 | rs1053094 | hCV3230081 | rs10866290 | 0.51 | 0.086445499 | 0.1232 |
| hCV3230113 | rs1053094 | hCV3230083 | rs10013653 | 0.51 | 0.086445499 | 0.5963 |
| hCV3230113 | rs1053094 | hCV3230084 | rs7682918 | 0.51 | 0.086445499 | 0.5711 |
| hCV3230113 | rs1053094 | hCV3230094 | rs7687818 | 0.51 | 0.086445499 | 0.7766 |
| hCV3230113 | rs1053094 | hCV3230096 | rs3817184 | 0.51 | 0.086445499 | 0.695 |
| hCV3230113 | rs1053094 | hCV3230097 | rs3736455 | 0.51 | 0.086445499 | 0.2344 |
| hCV3230113 | rs1053094 | hCV3230101 | rs6835839 | 0.51 | 0.086445499 | 0.6667 |
| hCV3230113 | rs1053094 | hCV3230106 | rs1473597 | 0.51 | 0.086445499 | 0.6384 |
| hCV3230113 | rs1053094 | hCV3230110 | rs2276917 | 0.51 | 0.086445499 | 0.6489 |
| hCV3230113 | rs1053094 | hCV3230118 | rs4253429 | 0.51 | 0.086445499 | 0.2024 |
| hCV3230113 | rs1053094 | hCV3230119 | rs4253430 | 0.51 | 0.086445499 | 0.096 |
| hCV3230113 | rs1053094 | hCV3230125 | rs11938564 | 0.51 | 0.086445499 | 0.1519 |
| hCV3230113 | rs1053094 | hCV3230131 | rs13136269 | 0.51 | 0.086445499 | 0.0986 |
| hCV3230113 | rs1053094 | hCV3230133 | rs12511874 | 0.51 | 0.086445499 | 0.0986 |
| hCV3230113 | rs1053094 | hCV3230134 | rs12500151 | 0.51 | 0.086445499 | 0.0986 |
| hCV3230113 | rs1053094 | hCV3230136 | rs13116273 | 0.51 | 0.086445499 | 0.1269 |
| hCV3230113 | rs1053094 | hCV32313006 | rs4253248 | 0.51 | 0.086445499 | 0.552 |
| hCV3230113 | rs1053094 | hCV32313007 | rs4862666 | 0.51 | 0.086445499 | 0.1117 |
| hCV3230113 | rs1053094 | hCV32313024 | rs4253239 | 0.51 | 0.086445499 | 0.2276 |
| hCV3230113 | rs1053094 | hCV32358975 | rs4253255 | 0.51 | 0.086445499 | 0.573 |
| hCV3230113 | rs1053094 | hCV32358984 | rs4253256 | 0.51 | 0.086445499 | 0.3678 |
| hCV3230113 | rs1053094 | hCV441385 | rs1983369 | 0.51 | 0.086445499 | 0.1249 |
| hCV3230113 | rs1053094 | hCV79084 | rs1519312 | 0.51 | 0.086445499 | 0.1129 |
| hCV3230113 | rs1053094 | hCV8241630 | rs925451 | 0.51 | 0.086445499 | 0.207 |
| hCV3230113 | rs1053094 | hCV8241631 | rs1511802 | 0.51 | 0.086445499 | 0.5019 |
| hCV3230113 | rs1053094 | hCV8241632 | rs1511801 | 0.51 | 0.086445499 | 0.6225 |
| hCV3230113 | rs1053094 | hCV8241633 | rs1511800 | 0.51 | 0.086445499 | 0.1117 |
| hCV3230113 | rs1053094 | hCV8241661 | rs1715051 | 0.51 | 0.086445499 | 0.1249 |
| hCV3230113 | rs1053094 | hDV71222711 | rs4253252 | 0.51 | 0.086445499 | 0.552 |
| hCV3230113 | rs1053094 | hDV76175111 | rs35079309 | 0.51 | 0.086445499 | 0.2766 |
| hCV596331 | rs6048 | hCV2288124 | rs440051 | 0.51 | 0.256432106 | 0.4103 |
| hCV596331 | rs6048 | hCV26016183 | rs9887617 | 0.51 | 0.256432106 | 0.3131 |

622

## TABLE 3, page 65 of 71

| Interrogated SNP | Interrogated rs | LD SNP | LD SNP rs | Power | Threshold r² | r² |
|---|---|---|---|---|---|---|
| hCV596331 | rs6048 | hCV26225376 | rs3117074 | 0.51 | 0.256432106 | 0.4074 |
| hCV596331 | rs6048 | hCV26225377 | rs12008759 | 0.51 | 0.256432106 | 0.4103 |
| hCV596331 | rs6048 | hCV2969899 | rs434144 | 0.51 | 0.256432106 | 0.3772 |
| hCV596331 | rs6048 | hCV2969900 | rs434447 | 0.51 | 0.256432106 | 0.4074 |
| hCV596331 | rs6048 | hCV2986569 | rs11095801 | 0.51 | 0.256432106 | 0.4074 |
| hCV596331 | rs6048 | hCV2986570 | rs3117458 | 0.51 | 0.256432106 | 0.3714 |
| hCV596331 | rs6048 | hCV2986572 | rs4149670 | 0.51 | 0.256432106 | 0.4393 |
| hCV596331 | rs6048 | hCV2986574 | rs4149672 | 0.51 | 0.256432106 | 0.602 |
| hCV596331 | rs6048 | hCV2986575 | rs4149674 | 0.51 | 0.256432106 | 0.602 |
| hCV596331 | rs6048 | hCV596323 | rs438601 | 0.51 | 0.256432106 | 0.5056 |
| hCV596331 | rs6048 | hCV596326 | rs398101 | 0.51 | 0.256432106 | 0.8045 |
| hCV596331 | rs6048 | hCV596330 | rs422187 | 0.51 | 0.256432106 | 0.9745 |
| hCV596331 | rs6048 | hCV596335 | rs413957 | 0.51 | 0.256432106 | 0.4074 |
| hCV596331 | rs6048 | hCV596336 | rs110583 | 0.51 | 0.256432106 | 0.4329 |
| hCV596331 | rs6048 | hCV596337 | rs421766 | 0.51 | 0.256432106 | 0.4329 |
| hCV596331 | rs6048 | hCV596339 | rs370713 | 0.51 | 0.256432106 | 0.4103 |
| hCV596331 | rs6048 | hCV596340 | rs413536 | 0.51 | 0.256432106 | 0.3724 |
| hCV596331 | rs6048 | hCV596344 | rs445691 | 0.51 | 0.256432106 | 0.4103 |
| hCV596331 | rs6048 | hCV596669 | rs376165 | 0.51 | 0.256432106 | 0.6589 |
| hCV596331 | rs6048 | hDV70794854 | rs17002122 | 0.51 | 0.256432106 | 0.3457 |
| hCV596331 | rs6048 | hDV71066592 | rs17002116 | 0.51 | 0.256432106 | 0.2766 |
| hCV596331 | rs6048 | hDV76976791 | rs4149758 | 0.51 | 0.256432106 | 0.3068 |
| hCV8241630 | rs925451 | hCV11786147 | rs4862662 | 0.51 | 0.047967528 | 0.1977 |
| hCV8241630 | rs925451 | hCV11786235 | rs4253287 | 0.51 | 0.047967528 | 0.0779 |
| hCV8241630 | rs925451 | hCV11786258 | rs4253303 | 0.51 | 0.047967528 | 0.2889 |
| hCV8241630 | rs925451 | hCV11786259 | rs4253304 | 0.51 | 0.047967528 | 0.3548 |
| hCV8241630 | rs925451 | hCV11786295 | rs4253421 | 0.51 | 0.047967528 | 0.0512 |
| hCV8241630 | rs925451 | hCV11786307 | rs1062547 | 0.51 | 0.047967528 | 0.3046 |
| hCV8241630 | rs925451 | hCV11786327 | rs13133050 | 0.51 | 0.047967528 | 0.1424 |
| hCV8241630 | rs925451 | hCV12066116 | rs1877320 | 0.51 | 0.047967528 | 0.0806 |
| hCV8241630 | rs925451 | hCV12066118 | rs2048 | 0.51 | 0.047967528 | 0.1423 |
| hCV8241630 | rs925451 | hCV12066119 | rs1912826 | 0.51 | 0.047967528 | 0.1513 |
| hCV8241630 | rs925451 | hCV12066124 | rs2036914 | 0.51 | 0.047967528 | 0.5632 |
| hCV8241630 | rs925451 | hCV12066129 | rs1593 | 0.51 | 0.047967528 | 0.0859 |
| hCV8241630 | rs925451 | hCV1333083 | rs10022988 | 0.51 | 0.047967528 | 0.0533 |
| hCV8241630 | rs925451 | hCV1333090 | rs6816112 | 0.51 | 0.047967528 | 0.0839 |
| hCV8241630 | rs925451 | hCV1333097 | rs4862680 | 0.51 | 0.047967528 | 0.0533 |
| hCV8241630 | rs925451 | hCV1333099 | rs10020635 | 0.51 | 0.047967528 | 0.0737 |
| hCV8241630 | rs925451 | hCV15793897 | rs3087505 | 0.51 | 0.047967528 | 0.0621 |
| hCV8241630 | rs925451 | hCV15811716 | rs2102575 | 0.51 | 0.047967528 | 0.0585 |
| hCV8241630 | rs925451 | hCV15968025 | rs2292425 | 0.51 | 0.047967528 | 0.1498 |
| hCV8241630 | rs925451 | hCV15968026 | rs2292426 | 0.51 | 0.047967528 | 0.2045 |
| hCV8241630 | rs925451 | hCV15968034 | rs2292428 | 0.51 | 0.047967528 | 0.1077 |
| hCV8241630 | rs925451 | hCV15968043 | rs2292423 | 0.51 | 0.047967528 | 0.338 |
| hCV8241630 | rs925451 | hCV16172925 | rs2241818 | 0.51 | 0.047967528 | 0.0837 |
| hCV8241630 | rs925451 | hCV16172935 | rs2241817 | 0.51 | 0.047967528 | 0.287 |
| hCV8241630 | rs925451 | hCV2103343 | rs4241824 | 0.51 | 0.047967528 | 0.605 |
| hCV8241630 | rs925451 | hCV2103348 | rs11931515 | 0.51 | 0.047967528 | 0.0499 |
| hCV8241630 | rs925451 | hCV2103388 | rs4613610 | 0.51 | 0.047967528 | 0.0893 |
| hCV8241630 | rs925451 | hCV2103391 | rs1008728 | 0.51 | 0.047967528 | 0.1739 |
| hCV8241630 | rs925451 | hCV2103392 | rs12500826 | 0.51 | 0.047967528 | 0.3352 |
| hCV8241630 | rs925451 | hCV22272267 | rs3733402 | 0.51 | 0.047967528 | 0.1476 |
| hCV8241630 | rs925451 | hCV25474413 | rs3822057 | 0.51 | 0.047967528 | 0.596 |
| hCV8241630 | rs925451 | hCV25474414 | rs4253399 | 0.51 | 0.047967528 | 0.9606 |
| hCV8241630 | rs925451 | hCV25634754 | rs4253331 | 0.51 | 0.047967528 | 0.0907 |

## TABLE 3, page 66 of 71

| Interrogated SNP | Interrogated rs | LD SNP | LD SNP rs | Power | Threshold r² | r² |
|---|---|---|---|---|---|---|
| hCV8241630 | rs925451 | hCV25988221 | rs9995366 | 0.51 | 0.047967528 | 0.0657 |
| hCV8241630 | rs925451 | hCV25990131 | rs13146272 | 0.51 | 0.047967528 | 0.1564 |
| hCV8241630 | rs925451 | hCV26038139 | rs4253405 | 0.51 | 0.047967528 | 0.3823 |
| hCV8241630 | rs925451 | hCV26265231 | rs7684025 | 0.51 | 0.047967528 | 0.2509 |
| hCV8241630 | rs925451 | hCV27309972 | rs13101296 | 0.51 | 0.047967528 | 0.1189 |
| hCV8241630 | rs925451 | hCV27309991 | rs4572916 | 0.51 | 0.047967528 | 0.0972 |
| hCV8241630 | rs925451 | hCV27473099 | rs3733403 | 0.51 | 0.047967528 | 0.078 |
| hCV8241630 | rs925451 | hCV27474895 | rs3756011 | 0.51 | 0.047967528 | 0.7876 |
| hCV8241630 | rs925451 | hCV27477533 | rs3756008 | 0.51 | 0.047967528 | 0.9804 |
| hCV8241630 | rs925451 | hCV27490984 | rs3822058 | 0.51 | 0.047967528 | 0.2976 |
| hCV8241630 | rs925451 | hCV27521729 | rs3822056 | 0.51 | 0.047967528 | 0.096 |
| hCV8241630 | rs925451 | hCV27902803 | rs4862665 | 0.51 | 0.047967528 | 0.0657 |
| hCV8241630 | rs925451 | hCV27902808 | rs4253236 | 0.51 | 0.047967528 | 0.0514 |
| hCV8241630 | rs925451 | hCV28960679 | rs6844764 | 0.51 | 0.047967528 | 0.1817 |
| hCV8241630 | rs925451 | hCV29053261 | rs6842047 | 0.51 | 0.047967528 | 0.0621 |
| hCV8241630 | rs925451 | hCV29053264 | rs7667777 | 0.51 | 0.047967528 | 0.2641 |
| hCV8241630 | rs925451 | hCV29053265 | rs4253244 | 0.51 | 0.047967528 | 0.0566 |
| hCV8241630 | rs925451 | hCV29718000 | rs4253238 | 0.51 | 0.047967528 | 0.1666 |
| hCV8241630 | rs925451 | hCV29826351 | rs10025990 | 0.51 | 0.047967528 | 0.0954 |
| hCV8241630 | rs925451 | hCV29877725 | rs4253295 | 0.51 | 0.047967528 | 0.2376 |
| hCV8241630 | rs925451 | hCV30492573 | rs10471184 | 0.51 | 0.047967528 | 0.0621 |
| hCV8241630 | rs925451 | hCV30983902 | rs4862668 | 0.51 | 0.047967528 | 0.0806 |
| hCV8241630 | rs925451 | hCV30983927 | rs6552962 | 0.51 | 0.047967528 | 0.0884 |
| hCV8241630 | rs925451 | hCV32209629 | rs12715865 | 0.51 | 0.047967528 | 0.1026 |
| hCV8241630 | rs925451 | hCV32209636 | rs11132387 | 0.51 | 0.047967528 | 0.3826 |
| hCV8241630 | rs925451 | hCV32209637 | rs13143773 | 0.51 | 0.047967528 | 0.2155 |
| hCV8241630 | rs925451 | hCV32209638 | rs12507040 | 0.51 | 0.047967528 | 0.2055 |
| hCV8241630 | rs925451 | hCV32291256 | rs4253406 | 0.51 | 0.047967528 | 0.1121 |
| hCV8241630 | rs925451 | hCV32291269 | rs4253417 | 0.51 | 0.047967528 | 0.7639 |
| hCV8241630 | rs925451 | hCV32291286 | rs4253422 | 0.51 | 0.047967528 | 0.1422 |
| hCV8241630 | rs925451 | hCV32291287 | rs4253423 | 0.51 | 0.047967528 | 0.1422 |
| hCV8241630 | rs925451 | hCV32291295 | rs4253292 | 0.51 | 0.047967528 | 0.0633 |
| hCV8241630 | rs925451 | hCV3229991 | rs4241815 | 0.51 | 0.047967528 | 0.1476 |
| hCV8241630 | rs925451 | hCV3229992 | rs3775298 | 0.51 | 0.047967528 | 0.1476 |
| hCV8241630 | rs925451 | hCV3229995 | rs11132382 | 0.51 | 0.047967528 | 0.1645 |
| hCV8241630 | rs925451 | hCV3230000 | rs4253294 | 0.51 | 0.047967528 | 0.0677 |
| hCV8241630 | rs925451 | hCV3230002 | rs4253297 | 0.51 | 0.047967528 | 0.2853 |
| hCV8241630 | rs925451 | hCV3230003 | rs2304595 | 0.51 | 0.047967528 | 0.3258 |
| hCV8241630 | rs925451 | hCV3230004 | rs4253301 | 0.51 | 0.047967528 | 0.0548 |
| hCV8241630 | rs925451 | hCV3230006 | rs4253308 | 0.51 | 0.047967528 | 0.2376 |
| hCV8241630 | rs925451 | hCV3230007 | rs4253311 | 0.51 | 0.047967528 | 0.1476 |
| hCV8241630 | rs925451 | hCV3230010 | rs4253315 | 0.51 | 0.047967528 | 0.0806 |
| hCV8241630 | rs925451 | hCV3230011 | rs4253320 | 0.51 | 0.047967528 | 0.2853 |
| hCV8241630 | rs925451 | hCV3230013 | rs3775303 | 0.51 | 0.047967528 | 0.3548 |
| hCV8241630 | rs925451 | hCV3230014 | rs4861709 | 0.51 | 0.047967528 | 0.0677 |
| hCV8241630 | rs925451 | hCV3230016 | rs4253325 | 0.51 | 0.047967528 | 0.0805 |
| hCV8241630 | rs925451 | hCV3230017 | rs4253327 | 0.51 | 0.047967528 | 0.1125 |
| hCV8241630 | rs925451 | hCV3230018 | rs925453 | 0.51 | 0.047967528 | 0.0593 |
| hCV8241630 | rs925451 | hCV3230019 | rs4253332 | 0.51 | 0.047967528 | 0.0554 |
| hCV8241630 | rs925451 | hCV3230021 | rs13135645 | 0.51 | 0.047967528 | 0.0814 |
| hCV8241630 | rs925451 | hCV3230022 | rs11132383 | 0.51 | 0.047967528 | 0.3677 |
| hCV8241630 | rs925451 | hCV3230025 | rs3756009 | 0.51 | 0.047967528 | 1 |
| hCV8241630 | rs925451 | hCV3230030 | rs4253408 | 0.51 | 0.047967528 | 0.1145 |
| hCV8241630 | rs925451 | hCV3230031 | rs4253419 | 0.51 | 0.047967528 | 0.1422 |
| hCV8241630 | rs925451 | hCV3230038 | rs2289252 | 0.51 | 0.047967528 | 0.7423 |

# TABLE 3, page 67 of 71

| Interrogated SNP | Interrogated rs | LD SNP | LD SNP rs | Power | Threshold r² | r² |
|---|---|---|---|---|---|---|
| hCV8241630 | rs925451 | hCV3230051 | rs4862658 | 0.51 | 0.047967528 | 0.0538 |
| hCV8241630 | rs925451 | hCV3230083 | rs10013653 | 0.51 | 0.047967528 | 0.2951 |
| hCV8241630 | rs925451 | hCV3230084 | rs7682918 | 0.51 | 0.047967528 | 0.2117 |
| hCV8241630 | rs925451 | hCV3230094 | rs7687818 | 0.51 | 0.047967528 | 0.3063 |
| hCV8241630 | rs925451 | hCV3230096 | rs3817184 | 0.51 | 0.047967528 | 0.2184 |
| hCV8241630 | rs925451 | hCV3230097 | rs3736455 | 0.51 | 0.047967528 | 0.2161 |
| hCV8241630 | rs925451 | hCV3230101 | rs6835839 | 0.51 | 0.047967528 | 0.0876 |
| hCV8241630 | rs925451 | hCV3230106 | rs1473597 | 0.51 | 0.047967528 | 0.127 |
| hCV8241630 | rs925451 | hCV3230110 | rs2276917 | 0.51 | 0.047967528 | 0.1184 |
| hCV8241630 | rs925451 | hCV3230113 | rs1053094 | 0.51 | 0.047967528 | 0.207 |
| hCV8241630 | rs925451 | hCV3230118 | rs4253429 | 0.51 | 0.047967528 | 0.1422 |
| hCV8241630 | rs925451 | hCV3230119 | rs4253430 | 0.51 | 0.047967528 | 0.2905 |
| hCV8241630 | rs925451 | hCV3230125 | rs11938564 | 0.51 | 0.047967528 | 0.1896 |
| hCV8241630 | rs925451 | hCV3230131 | rs13136269 | 0.51 | 0.047967528 | 0.2055 |
| hCV8241630 | rs925451 | hCV3230133 | rs12511874 | 0.51 | 0.047967528 | 0.1512 |
| hCV8241630 | rs925451 | hCV3230134 | rs12500151 | 0.51 | 0.047967528 | 0.2115 |
| hCV8241630 | rs925451 | hCV3230136 | rs13116273 | 0.51 | 0.047967528 | 0.229 |
| hCV8241630 | rs925451 | hCV32313006 | rs4253248 | 0.51 | 0.047967528 | 0.1736 |
| hCV8241630 | rs925451 | hCV32313007 | rs4862666 | 0.51 | 0.047967528 | 0.0657 |
| hCV8241630 | rs925451 | hCV32313014 | rs4253243 | 0.51 | 0.047967528 | 0.0907 |
| hCV8241630 | rs925451 | hCV32313024 | rs4253239 | 0.51 | 0.047967528 | 0.0633 |
| hCV8241630 | rs925451 | hCV32358975 | rs4253255 | 0.51 | 0.047967528 | 0.1454 |
| hCV8241630 | rs925451 | hCV32358984 | rs4253256 | 0.51 | 0.047967528 | 0.0647 |
| hCV8241630 | rs925451 | hCV8241628 | rs907439 | 0.51 | 0.047967528 | 0.0972 |
| hCV8241630 | rs925451 | hCV8241631 | rs1511802 | 0.51 | 0.047967528 | 0.2539 |
| hCV8241630 | rs925451 | hCV8241632 | rs1511801 | 0.51 | 0.047967528 | 0.1877 |
| hCV8241630 | rs925451 | hCV8241633 | rs1511800 | 0.51 | 0.047967528 | 0.0657 |
| hCV8241630 | rs925451 | hDV68550952 | rs4253289 | 0.51 | 0.047967528 | 0.0659 |
| hCV8241630 | rs925451 | hDV71222711 | rs4253252 | 0.51 | 0.047967528 | 0.1736 |
| hCV8717873 | rs1613662 | hCV11977629 | rs1654459 | 0.51 | 0.291390182 | 0.824 |
| hCV8717873 | rs1613662 | hCV1376257 | rs10416380 | 0.51 | 0.291390182 | 0.688 |
| hCV8717873 | rs1613662 | hCV1376262 | rs1671150 | 0.51 | 0.291390182 | 0.7101 |
| hCV8717873 | rs1613662 | hCV1376264 | rs1671151 | 0.51 | 0.291390182 | 0.7101 |
| hCV8717873 | rs1613662 | hCV1376265 | rs1671152 | 0.51 | 0.291390182 | 0.881 |
| hCV8717873 | rs1613662 | hCV1376266 | rs1654413 | 0.51 | 0.291390182 | 0.8292 |
| hCV8717873 | rs1613662 | hCV1376342 | rs1654416 | 0.51 | 0.291390182 | 0.7313 |
| hCV8717873 | rs1613662 | hCV1376359 | rs2886412 | 0.51 | 0.291390182 | 0.8039 |
| hCV8717873 | rs1613662 | hCV1376386 | rs1671214 | 0.51 | 0.291390182 | 0.4218 |
| hCV8717873 | rs1613662 | hCV1376388 | rs1671215 | 0.51 | 0.291390182 | 0.4218 |
| hCV8717873 | rs1613662 | hCV1376414 | rs1671171 | 0.51 | 0.291390182 | 0.4652 |
| hCV8717873 | rs1613662 | hCV15973734 | rs2304167 | 0.51 | 0.291390182 | 0.7101 |
| hCV8717873 | rs1613662 | hCV16044361 | rs2569513 | 0.51 | 0.291390182 | 0.8557 |
| hCV8717873 | rs1613662 | hCV26895244 | rs1671153 | 0.51 | 0.291390182 | 0.7101 |
| hCV8717873 | rs1613662 | hCV26895257 | rs2886415 | 0.51 | 0.291390182 | 0.8732 |
| hCV8717873 | rs1613662 | hCV29271569 | rs1626971 | 0.51 | 0.291390182 | 0.8853 |
| hCV8717873 | rs1613662 | hCV31722831 | rs11671922 | 0.51 | 0.291390182 | 0.8358 |
| hCV8717873 | rs1613662 | hCV31722832 | rs11084381 | 0.51 | 0.291390182 | 0.8192 |
| hCV8717873 | rs1613662 | hCV31722834 | rs11084382 | 0.51 | 0.291390182 | 0.7187 |
| hCV8717873 | rs1613662 | hCV31722835 | rs11668169 | 0.51 | 0.291390182 | 0.8188 |
| hCV8717873 | rs1613662 | hCV31722836 | rs11672026 | 0.51 | 0.291390182 | 0.8093 |
| hCV8717873 | rs1613662 | hCV7841075 | rs1671196 | 0.51 | 0.291390182 | 0.8192 |
| hCV8717873 | rs1613662 | hCV8703249 | rs1654444 | 0.51 | 0.291390182 | 0.8822 |
| hCV8717873 | rs1613662 | hCV8704962 | rs775893 | 0.51 | 0.291390182 | 0.5398 |
| hCV8717873 | rs1613662 | hCV8717751 | rs1671218 | 0.51 | 0.291390182 | 0.4141 |
| hCV8717873 | rs1613662 | hCV8717752 | rs1671217 | 0.51 | 0.291390182 | 0.8853 |

## TABLE 3, page 68 of 71

| Interrogated SNP | Interrogated rs | LD SNP | LD SNP rs | Power | Threshold r² | r² |
|---|---|---|---|---|---|---|
| hCV8717873 | rs1613662 | hCV8717761 | rs1654439 | 0.51 | 0.291390182 | 0.776 |
| hCV8717873 | rs1613662 | hCV8717793 | rs1654433 | 0.51 | 0.291390182 | 0.8557 |
| hCV8717873 | rs1613662 | hCV8717794 | rs1654432 | 0.51 | 0.291390182 | 0.8557 |
| hCV8717873 | rs1613662 | hCV8717845 | rs892090 | 0.51 | 0.291390182 | 1 |
| hCV8717873 | rs1613662 | hCV8717846 | rs892089 | 0.51 | 0.291390182 | 0.8358 |
| hCV8717873 | rs1613662 | hCV8717871 | rs1654421 | 0.51 | 0.291390182 | 0.6875 |
| hCV8717873 | rs1613662 | hCV8717881 | rs1654420 | 0.51 | 0.291390182 | 0.8188 |
| hCV8717873 | rs1613662 | hCV8717893 | rs1671192 | 0.51 | 0.291390182 | 0.8737 |
| hCV8717873 | rs1613662 | hCV8718961 | rs1654451 | 0.51 | 0.291390182 | 0.8233 |
| hCV8717873 | rs1613662 | hCV8718968 | rs1671176 | 0.51 | 0.291390182 | 0.4332 |
| hCV8717873 | rs1613662 | hCV8718972 | rs1654447 | 0.51 | 0.291390182 | 0.8825 |
| hCV8717873 | rs1613662 | hCV9490926 | rs1654419 | 0.51 | 0.291390182 | 0.8188 |
| hCV8717873 | rs1613662 | hDV91225183 | rs1671171 | 0.51 | 0.291390182 | 0.4652 |
| hCV8718961 | rs1654451 | hCV11977629 | rs1654459 | 0.51 | 0.573058702 | 1 |
| hCV8718961 | rs1654451 | hCV1376265 | rs1671152 | 0.51 | 0.573058702 | 0.7073 |
| hCV8718961 | rs1654451 | hCV1376266 | rs1654413 | 0.51 | 0.573058702 | 0.7211 |
| hCV8718961 | rs1654451 | hCV1376342 | rs1654416 | 0.51 | 0.573058702 | 0.5824 |
| hCV8718961 | rs1654451 | hCV1376359 | rs2886412 | 0.51 | 0.573058702 | 0.7389 |
| hCV8718961 | rs1654451 | hCV16044361 | rs2569513 | 0.51 | 0.573058702 | 0.9121 |
| hCV8718961 | rs1654451 | hCV26895257 | rs2886415 | 0.51 | 0.573058702 | 0.8105 |
| hCV8718961 | rs1654451 | hCV29271569 | rs1626971 | 0.51 | 0.573058702 | 1 |
| hCV8718961 | rs1654451 | hCV31722831 | rs11671922 | 0.51 | 0.573058702 | 0.7314 |
| hCV8718961 | rs1654451 | hCV31722832 | rs11084381 | 0.51 | 0.573058702 | 0.6692 |
| hCV8718961 | rs1654451 | hCV31722834 | rs11084382 | 0.51 | 0.573058702 | 0.584 |
| hCV8718961 | rs1654451 | hCV31722835 | rs11668169 | 0.51 | 0.573058702 | 0.6692 |
| hCV8718961 | rs1654451 | hCV31722836 | rs11672026 | 0.51 | 0.573058702 | 0.7025 |
| hCV8718961 | rs1654451 | hCV7841075 | rs1671196 | 0.51 | 0.573058702 | 0.6692 |
| hCV8718961 | rs1654451 | hCV8703249 | rs1654444 | 0.51 | 0.573058702 | 0.9398 |
| hCV8718961 | rs1654451 | hCV8717752 | rs1671217 | 0.51 | 0.573058702 | 1 |
| hCV8718961 | rs1654451 | hCV8717761 | rs1654439 | 0.51 | 0.573058702 | 0.8855 |
| hCV8718961 | rs1654451 | hCV8717793 | rs1654433 | 0.51 | 0.573058702 | 0.9121 |
| hCV8718961 | rs1654451 | hCV8717794 | rs1654432 | 0.51 | 0.573058702 | 0.9121 |
| hCV8718961 | rs1654451 | hCV8717845 | rs892090 | 0.51 | 0.573058702 | 0.8233 |
| hCV8718961 | rs1654451 | hCV8717846 | rs892089 | 0.51 | 0.573058702 | 0.7314 |
| hCV8718961 | rs1654451 | hCV8717873 | rs1613662 | 0.51 | 0.573058702 | 0.8233 |
| hCV8718961 | rs1654451 | hCV8717881 | rs1654420 | 0.51 | 0.573058702 | 0.6692 |
| hCV8718961 | rs1654451 | hCV8717893 | rs1671192 | 0.51 | 0.573058702 | 0.756 |
| hCV8718961 | rs1654451 | hCV8718972 | rs1654447 | 0.51 | 0.573058702 | 0.94 |
| hCV8718961 | rs1654451 | hCV9490926 | rs1654419 | 0.51 | 0.573058702 | 0.6692 |
| hCV8911768 | rs941988 | hCV11342529 | rs1951627 | 0.51 | 0.228649809 | 0.3108 |
| hCV8911768 | rs941988 | hCV11975630 | rs2065170 | 0.51 | 0.228649809 | 1 |
| hCV8911768 | rs941988 | hCV15864094 | rs2068871 | 0.51 | 0.228649809 | 0.9425 |
| hCV8911768 | rs941988 | hCV15956059 | rs2227592 | 0.51 | 0.228649809 | 1 |
| hCV8911768 | rs941988 | hCV16135173 | rs2146372 | 0.51 | 0.228649809 | 1 |
| hCV8911768 | rs941988 | hCV16180170 | rs2227589 | 0.51 | 0.228649809 | 1 |
| hCV8911768 | rs941988 | hCV16290208 | rs2759328 | 0.51 | 0.228649809 | 1 |
| hCV8911768 | rs941988 | hCV1681325 | rs898657 | 0.51 | 0.228649809 | 0.288 |
| hCV8911768 | rs941988 | hCV1681328 | rs10912647 | 0.51 | 0.228649809 | 0.2457 |
| hCV8911768 | rs941988 | hCV25600635 | rs7539322 | 0.51 | 0.228649809 | 0.8856 |
| hCV8911768 | rs941988 | hCV25932979 | rs16846809 | 0.51 | 0.228649809 | 0.5549 |
| hCV8911768 | rs941988 | hCV27483572 | rs3791022 | 0.51 | 0.228649809 | 1 |
| hCV8911768 | rs941988 | hCV28998001 | rs6425251 | 0.51 | 0.228649809 | 0.2457 |
| hCV8911768 | rs941988 | hCV29517287 | rs2901747 | 0.51 | 0.228649809 | 0.2436 |
| hCV8911768 | rs941988 | hCV29989899 | rs6685043 | 0.51 | 0.228649809 | 0.6095 |
| hCV8911768 | rs941988 | hCV30205817 | rs10489254 | 0.51 | 0.228649809 | 0.5549 |

# TABLE 3, page 69 of 71

| Interrogated SNP | Interrogated rs | LD SNP | LD SNP rs | Power | Threshold r² | r² |
|---|---|---|---|---|---|---|
| hCV8911768 | rs941988 | hCV30404194 | rs6691053 | 0.51 | 0.228649809 | 0.3572 |
| hCV8911768 | rs941988 | hCV30472885 | rs7520441 | 0.51 | 0.228649809 | 0.315 |
| hCV8911768 | rs941988 | hCV30804119 | rs10912651 | 0.51 | 0.228649809 | 0.2376 |
| hCV8911768 | rs941988 | hCV30804135 | rs12078293 | 0.51 | 0.228649809 | 0.2457 |
| hCV8911768 | rs941988 | hCV30804139 | rs12089930 | 0.51 | 0.228649809 | 0.245 |
| hCV8911768 | rs941988 | hCV8911729 | rs941987 | 0.51 | 0.228649809 | 0.8292 |
| hCV8911768 | rs941988 | hCV9575253 | rs1031751 | 0.51 | 0.228649809 | 0.3146 |
| hCV8911768 | rs941988 | hCV9575263 | rs898658 | 0.51 | 0.228649809 | 0.2457 |
| hCV8911768 | rs941988 | hDV70683090 | rs16846433 | 0.51 | 0.228649809 | 0.9425 |
| hCV8911768 | rs941988 | hDV70683162 | rs16846526 | 0.51 | 0.228649809 | 1 |
| hCV8911768 | rs941988 | hDV70683177 | rs16846546 | 0.51 | 0.228649809 | 1 |
| hCV8911768 | rs941988 | hDV70683187 | rs16846561 | 0.51 | 0.228649809 | 1 |
| hCV8911768 | rs941988 | hDV70683212 | rs16846593 | 0.51 | 0.228649809 | 0.5549 |
| hCV8911768 | rs941988 | hDV70683382 | rs16846815 | 0.51 | 0.228649809 | 0.5078 |
| hCV8911768 | rs941988 | hDV70934851 | rs17301125 | 0.51 | 0.228649809 | 0.2534 |
| hCV8919444 | rs4524 | hCV11341772 | rs4589164 | 0.51 | 0.098333329 | 0.1285 |
| hCV8919444 | rs4524 | hCV11341879 | rs7527703 | 0.51 | 0.098333329 | 0.1112 |
| hCV8919444 | rs4524 | hCV11341886 | rs7539415 | 0.51 | 0.098333329 | 0.1052 |
| hCV8919444 | rs4524 | hCV11341964 | rs12124049 | 0.51 | 0.098333329 | 0.1062 |
| hCV8919444 | rs4524 | hCV11342057 | rs10919186 | 0.51 | 0.098333329 | 0.7473 |
| hCV8919444 | rs4524 | hCV11975196 | rs2040444 | 0.51 | 0.098333329 | 0.358 |
| hCV8919444 | rs4524 | hCV1264276 | rs17345170 | 0.51 | 0.098333329 | 0.1214 |
| hCV8919444 | rs4524 | hCV15802102 | rs2420369 | 0.51 | 0.098333329 | 0.3671 |
| hCV8919444 | rs4524 | hCV15847759 | rs2187952 | 0.51 | 0.098333329 | 1 |
| hCV8919444 | rs4524 | hCV15852051 | rs2213867 | 0.51 | 0.098333329 | 0.8264 |
| hCV8919444 | rs4524 | hCV15955265 | rs2227244 | 0.51 | 0.098333329 | 1 |
| hCV8919444 | rs4524 | hCV16141160 | rs2157597 | 0.51 | 0.098333329 | 0.1285 |
| hCV8919444 | rs4524 | hCV16175730 | rs2239851 | 0.51 | 0.098333329 | 1 |
| hCV8919444 | rs4524 | hCV16175731 | rs2239852 | 0.51 | 0.098333329 | 0.8118 |
| hCV8919444 | rs4524 | hCV16191269 | rs2298909 | 0.51 | 0.098333329 | 0.6586 |
| hCV8919444 | rs4524 | hCV22274637 | rs2301515 | 0.51 | 0.098333329 | 0.7941 |
| hCV8919444 | rs4524 | hCV2229795 | rs723751 | 0.51 | 0.098333329 | 0.4376 |
| hCV8919444 | rs4524 | hCV2456680 | rs6427193 | 0.51 | 0.098333329 | 0.119 |
| hCV8919444 | rs4524 | hCV2456690 | rs6692649 | 0.51 | 0.098333329 | 0.1119 |
| hCV8919444 | rs4524 | hCV2456692 | rs12128350 | 0.51 | 0.098333329 | 0.1136 |
| hCV8919444 | rs4524 | hCV2456693 | rs6672589 | 0.51 | 0.098333329 | 0.1331 |
| hCV8919444 | rs4524 | hCV2456695 | rs10919173 | 0.51 | 0.098333329 | 0.1331 |
| hCV8919444 | rs4524 | hCV2456709 | rs17577184 | 0.51 | 0.098333329 | 0.1068 |
| hCV8919444 | rs4524 | hCV2456710 | rs4656680 | 0.51 | 0.098333329 | 0.1112 |
| hCV8919444 | rs4524 | hCV2456716 | rs12730053 | 0.51 | 0.098333329 | 0.1112 |
| hCV8919444 | rs4524 | hCV2456722 | rs12119479 | 0.51 | 0.098333329 | 0.1112 |
| hCV8919444 | rs4524 | hCV2456729 | rs12143708 | 0.51 | 0.098333329 | 0.1068 |
| hCV8919444 | rs4524 | hCV2456767 | rs2014061 | 0.51 | 0.098333329 | 0.1287 |
| hCV8919444 | rs4524 | hCV2456774 | rs1014965 | 0.51 | 0.098333329 | 0.1025 |
| hCV8919444 | rs4524 | hCV2456776 | rs6669741 | 0.51 | 0.098333329 | 0.1052 |
| hCV8919444 | rs4524 | hCV2456780 | rs7534737 | 0.51 | 0.098333329 | 0.1052 |
| hCV8919444 | rs4524 | hCV2481727 | rs6670407 | 0.51 | 0.098333329 | 0.4176 |
| hCV8919444 | rs4524 | hCV2481728 | rs9332665 | 0.51 | 0.098333329 | 0.7359 |
| hCV8919444 | rs4524 | hCV2481731 | rs9332640 | 0.51 | 0.098333329 | 0.4021 |
| hCV8919444 | rs4524 | hCV2481732 | rs12131397 | 0.51 | 0.098333329 | 0.3978 |
| hCV8919444 | rs4524 | hCV2481733 | rs9332627 | 0.51 | 0.098333329 | 1 |
| hCV8919444 | rs4524 | hCV2481738 | rs4656187 | 0.51 | 0.098333329 | 1 |
| hCV8919444 | rs4524 | hCV2481741 | rs3766109 | 0.51 | 0.098333329 | 1 |
| hCV8919444 | rs4524 | hCV2481744 | rs9332600 | 0.51 | 0.098333329 | 1 |
| hCV8919444 | rs4524 | hCV2481747 | rs9332595 | 0.51 | 0.098333329 | 0.7683 |

# TABLE 3, page 70 of 71

| Interrogated SNP | Interrogated rs | LD SNP | LD SNP rs | Power | Threshold r² | r² |
|---|---|---|---|---|---|---|
| hCV8919444 | rs4524 | hCV2481748 | rs3766110 | 0.51 | 0.098333329 | 0.7683 |
| hCV8919444 | rs4524 | hCV2481750 | rs10800456 | 0.51 | 0.098333329 | 0.6306 |
| hCV8919444 | rs4524 | hCV2520857 | rs12118611 | 0.51 | 0.098333329 | 0.1356 |
| hCV8919444 | rs4524 | hCV2520872 | rs3766090 | 0.51 | 0.098333329 | 0.1059 |
| hCV8919444 | rs4524 | hCV2520887 | rs10442644 | 0.51 | 0.098333329 | 0.1285 |
| hCV8919444 | rs4524 | hCV2521003 | rs2040446 | 0.51 | 0.098333329 | 0.1214 |
| hCV8919444 | rs4524 | hCV25617181 | rs9332620 | 0.51 | 0.098333329 | 1 |
| hCV8919444 | rs4524 | hCV25922120 | rs9332643 | 0.51 | 0.098333329 | 1 |
| hCV8919444 | rs4524 | hCV27242356 | rs12121994 | 0.51 | 0.098333329 | 0.1358 |
| hCV8919444 | rs4524 | hCV27242515 | rs3818844 | 0.51 | 0.098333329 | 0.1009 |
| hCV8919444 | rs4524 | hCV27242533 | rs2138898 | 0.51 | 0.098333329 | 0.1023 |
| hCV8919444 | rs4524 | hCV27242706 | rs7524348 | 0.51 | 0.098333329 | 0.1331 |
| hCV8919444 | rs4524 | hCV27242809 | rs9332630 | 0.51 | 0.098333329 | 0.3659 |
| hCV8919444 | rs4524 | hCV27490260 | rs3820060 | 0.51 | 0.098333329 | 0.8118 |
| hCV8919444 | rs4524 | hCV275164 | rs12140572 | 0.51 | 0.098333329 | 0.1278 |
| hCV8919444 | rs4524 | hCV27928247 | rs4656182 | 0.51 | 0.098333329 | 0.1068 |
| hCV8919444 | rs4524 | hCV27972646 | rs4656677 | 0.51 | 0.098333329 | 0.1112 |
| hCV8919444 | rs4524 | hCV288901 | rs4656671 | 0.51 | 0.098333329 | 0.1112 |
| hCV8919444 | rs4524 | hCV29621699 | rs9332619 | 0.51 | 0.098333329 | 1 |
| hCV8919444 | rs4524 | hCV30018856 | rs6701330 | 0.51 | 0.098333329 | 0.7509 |
| hCV8919444 | rs4524 | hCV30036717 | rs9332653 | 0.51 | 0.098333329 | 0.102 |
| hCV8919444 | rs4524 | hCV30144962 | rs10158595 | 0.51 | 0.098333329 | 0.7453 |
| hCV8919444 | rs4524 | hCV30234691 | rs6662593 | 0.51 | 0.098333329 | 0.9762 |
| hCV8919444 | rs4524 | hCV30433255 | rs9332655 | 0.51 | 0.098333329 | 0.9135 |
| hCV8919444 | rs4524 | hCV30504827 | rs9332608 | 0.51 | 0.098333329 | 0.1235 |
| hCV8919444 | rs4524 | hCV30577322 | rs7516248 | 0.51 | 0.098333329 | 0.1052 |
| hCV8919444 | rs4524 | hCV32141090 | rs12039443 | 0.51 | 0.098333329 | 0.1294 |
| hCV8919444 | rs4524 | hCV32141333 | rs10800446 | 0.51 | 0.098333329 | 0.0998 |
| hCV8919444 | rs4524 | hCV32141337 | rs10919164 | 0.51 | 0.098333329 | 0.115 |
| hCV8919444 | rs4524 | hCV32141359 | rs12022776 | 0.51 | 0.098333329 | 0.0998 |
| hCV8919444 | rs4524 | hCV32141374 | rs10919174 | 0.51 | 0.098333329 | 0.1582 |
| hCV8919444 | rs4524 | hCV32398607 | rs4656658 | 0.51 | 0.098333329 | 0.1214 |
| hCV8919444 | rs4524 | hCV328321 | rs9332667 | 0.51 | 0.098333329 | 1 |
| hCV8919444 | rs4524 | hCV337817 | rs9332586 | 0.51 | 0.098333329 | 0.1619 |
| hCV8919444 | rs4524 | hCV340605 | rs1557572 | 0.51 | 0.098333329 | 0.7416 |
| hCV8919444 | rs4524 | hCV341935 | rs4656685 | 0.51 | 0.098333329 | 0.9762 |
| hCV8919444 | rs4524 | hCV342590 | rs6030 | 0.51 | 0.098333329 | 0.847 |
| hCV8919444 | rs4524 | hCV475606 | rs17349579 | 0.51 | 0.098333329 | 0.105 |
| hCV8919444 | rs4524 | hCV70275 | rs4656687 | 0.51 | 0.098333329 | 0.8118 |
| hCV8919444 | rs4524 | hCV8006091 | rs6656463 | 0.51 | 0.098333329 | 0.1151 |
| hCV8919444 | rs4524 | hCV8697038 | rs961403 | 0.51 | 0.098333329 | 0.1052 |
| hCV8919444 | rs4524 | hCV8697043 | rs1517747 | 0.51 | 0.098333329 | 0.1582 |
| hCV8919444 | rs4524 | hCV8919166 | rs1200139 | 0.51 | 0.098333329 | 0.1536 |
| hCV8919444 | rs4524 | hCV8919279 | rs1200079 | 0.51 | 0.098333329 | 0.2649 |
| hCV8919444 | rs4524 | hCV8919424 | rs974793 | 0.51 | 0.098333329 | 0.9762 |
| hCV8919444 | rs4524 | hCV8919429 | rs970741 | 0.51 | 0.098333329 | 0.9762 |
| hCV8919444 | rs4524 | hCV8919436 | rs916438 | 0.51 | 0.098333329 | 0.8118 |
| hCV8919444 | rs4524 | hCV8919438 | rs1557570 | 0.51 | 0.098333329 | 0.7402 |
| hCV8919444 | rs4524 | hCV8919441 | rs6032 | 0.51 | 0.098333329 | 1 |
| hCV8919444 | rs4524 | hCV8919442 | rs4525 | 0.51 | 0.098333329 | 1 |
| hCV8919444 | rs4524 | hCV8919446 | rs6021 | 0.51 | 0.098333329 | 1 |
| hCV8919444 | rs4524 | hCV8919450 | rs6017 | 0.51 | 0.098333329 | 1 |
| hCV8919444 | rs4524 | hCV8919451 | rs6016 | 0.51 | 0.098333329 | 0.9762 |
| hCV8919444 | rs4524 | hCV9945852 | rs1121789 | 0.51 | 0.098333329 | 1 |
| hCV8919444 | rs4524 | hDV70942075 | rs17349271 | 0.51 | 0.098333329 | 0.1214 |

## TABLE 3, page 71 of 71

| Interrogated SNP | Interrogated rs | LD SNP | LD SNP rs | Power | Threshold r² | r² |
|---|---|---|---|---|---|---|
| hCV8919444 | rs4524 | hDV70942101 | rs17349439 | 0.51 | 0.098333329 | 0.1214 |
| hCV8919444 | rs4524 | hDV77030721 | rs4656664 | 0.51 | 0.098333329 | 0.1224 |
| hCV9102827 | rs3795733 | hCV11258640 | rs6427323 | 0.51 | 0.60489105 | 0.7384 |
| hCV9102827 | rs3795733 | hCV25989540 | rs6682716 | 0.51 | 0.60489105 | 0.6304 |
| hCV9102827 | rs3795733 | hCV26627664 | rs3795727 | 0.51 | 0.60489105 | 0.7186 |
| hCV9102827 | rs3795733 | hCV26627665 | rs2365714 | 0.51 | 0.60489105 | 0.7186 |
| hCV9102827 | rs3795733 | hCV26627679 | rs7536235 | 0.51 | 0.60489105 | 0.7186 |
| hCV9102827 | rs3795733 | hCV31431594 | rs12567958 | 0.51 | 0.60489105 | 0.863 |
| hCV9102827 | rs3795733 | hCV31431603 | rs11264508 | 0.51 | 0.60489105 | 0.7186 |
| hCV9102827 | rs3795733 | hCV31431609 | rs12742817 | 0.51 | 0.60489105 | 0.7186 |
| hCV9102827 | rs3795733 | hCV31431620 | rs12023410 | 0.51 | 0.60489105 | 0.714 |
| hCV9102827 | rs3795733 | hCV31431621 | rs11576266 | 0.51 | 0.60489105 | 1 |
| hCV9102827 | rs3795733 | hCV9102814 | rs879461 | 0.51 | 0.60489105 | 0.8863 |
| hCV9102827 | rs3795733 | hCV9102822 | rs4661052 | 0.51 | 0.60489105 | 0.8863 |
| hCV9102827 | rs3795733 | hCV9102823 | rs12024215 | 0.51 | 0.60489105 | 0.7508 |
| hCV9102827 | rs3795733 | hCV9102829 | rs3795732 | 0.51 | 0.60489105 | 0.8859 |
| hCV9102827 | rs3795733 | hCV9102841 | rs4661188 | 0.51 | 0.60489105 | 0.8863 |
| hCV9102827 | rs3795733 | hCV9102976 | rs10908509 | 0.51 | 0.60489105 | 0.6291 |
| hCV916107 | rs670659 | hCV1874947 | rs494075 | 0.51 | 0.426900693 | 0.4398 |
| hCV916107 | rs670659 | hCV25653735 | rs7520707 | 0.51 | 0.426900693 | 0.5479 |
| hCV916107 | rs670659 | hCV26887401 | rs10802916 | 0.51 | 0.426900693 | 0.4941 |
| hCV916107 | rs670659 | hCV26887441 | rs9786932 | 0.51 | 0.426900693 | 0.5809 |
| hCV916107 | rs670659 | hCV26887461 | rs4660023 | 0.51 | 0.426900693 | 0.7128 |
| hCV916107 | rs670659 | hCV26887463 | rs6680767 | 0.51 | 0.426900693 | 0.7121 |
| hCV916107 | rs670659 | hCV26887464 | rs6669640 | 0.51 | 0.426900693 | 0.6131 |
| hCV916107 | rs670659 | hCV26887465 | rs10802919 | 0.51 | 0.426900693 | 0.5881 |
| hCV916107 | rs670659 | hCV31714435 | rs12143076 | 0.51 | 0.426900693 | 0.463 |
| hCV916107 | rs670659 | hCV31714436 | rs12132113 | 0.51 | 0.426900693 | 0.43 |
| hCV916107 | rs670659 | hCV31714438 | rs12731839 | 0.51 | 0.426900693 | 0.4771 |
| hCV916107 | rs670659 | hCV31714442 | rs12119557 | 0.51 | 0.426900693 | 0.4359 |
| hCV916107 | rs670659 | hCV31714442 | rs12758552 | 0.51 | 0.426900693 | 0.4423 |
| hCV916107 | rs670659 | hCV31714443 | rs12758552 | 0.51 | 0.426900693 | 0.4423 |
| hCV916107 | rs670659 | hCV31714447 | rs10926387 | 0.51 | 0.426900693 | 0.5544 |
| hCV916107 | rs670659 | hCV31714470 | rs10926390 | 0.51 | 0.426900693 | 0.4745 |
| hCV916107 | rs670659 | hCV31714471 | rs10926391 | 0.51 | 0.426900693 | 0.4562 |
| hCV916107 | rs670659 | hCV916106 | rs575226 | 0.51 | 0.426900693 | 1 |

## TABLE 4

Table 4. Association of statin with VT in 27 SNP genotype subgroups in MEGA

| hCV # | Gene Symbol (SNP rs #) | Risk Allele | Subgroup | OR (95%CI) | P | statin users, cases | statin nonuser, cases | statin users, controls | statin nonusers, controls | p(int) statin* SNP | Comparison for p(int) statin*SNP |
|---|---|---|---|---|---|---|---|---|---|---|---|
| hCV11286902 | LOC400499 (rs12932948) | G | GG | 0.34 (0.19-0.62) | 4E-04 | 14 (0.11) | 641 (0.19) | 54 (0.22) | 793 (0.2) | 0.010 | GG vs. AA |
| | | | GA | 0.6 (0.43-0.82) | 0.001 | 62 (0.5) | 1668 (0.48) | 124 (0.5) | 1907 (0.47) | 0.168 | GA vs. AA |
| | | | AA | 0.78 (0.53-1.15) | 0.208 | 47 (0.38) | 1142 (0.33) | 69 (0.28) | 1352 (0.33) | ref | |
| | | | GA+GG | 0.52 (0.4-0.69) | 5.E-06 | | | | | 0.044 | GA+GG vs. AA |
| | | | GA+AA | 0.66 (0.52-0.85) | 9E-04 | | | | | 0.029 | GG vs.GA+AA |
| hCV11786258 | KLKB1 (rs4253303) | A | AA | 0.35 (0.19-0.64) | 7E-04 | 15 (0.12) | 700 (0.2) | 41 (0.16) | 642 (0.15) | 0.035 | AA vs. GG |
| | | | AG | 0.57 (0.42-0.79) | 6E-04 | 61 (0.49) | 1742 (0.49) | 122 (0.47) | 2007 (0.48) | 0.384 | AG vs. GG |
| | | | GG | 0.73 (0.51-1.06) | 0.095 | 48 (0.39) | 1114 (0.31) | 94 (0.37) | 1552 (0.37) | ref | |
| | | | AG+AA | 0.51 (0.39-0.68) | 3.E-06 | | | | | 0.139 | AG+AA vs. GG |
| | | | AG+GG | 0.63 (0.5-0.81) | 2E-04 | | | | | 0.056 | AA vs.AG+GG |
| hCV12066124 | F11 (rs2036914) | C | CC | 0.38 (0.24-0.6) | 3.E-05 | 27 (0.22) | 1245 (0.35) | 68 (0.26) | 1138 (0.27) | 0.136 | CC vs. TT |
| | | | CT | 0.66 (0.49-0.9) | 0.008 | 72 (0.59) | 1679 (0.47) | 130 (0.5) | 2066 (0.49) | 0.662 | CT vs. TT |
| | | | TT | 0.64 (0.39-1.05) | 0.077 | 23 (0.19) | 623 (0.18) | 61 (0.24) | 994 (0.24) | ref | |
| | | | CT+CC | 0.55 (0.43-0.71) | 3.E-06 | | | | | 0.740 | CT+CC vs. TT |
| | | | CT+TT | 0.66 (0.51-0.85) | 0.001 | | | | | 0.022 | CC vs.CT+TT |
| hCV12092542 | CASP5 (rs507879) | T | TT | 0.75 (0.49-1.15) | 0.189 | 39 (0.33) | 1025 (0.3) | 54 (0.26) | 1083 (0.31) | 0.602 | TT vs. CC |
| | | | TC | 0.47 (0.34-0.66) | 1.E-05 | 52 (0.44) | 1704 (0.51) | 116 (0.57) | 1742 (0.49) | 0.027 | TC vs. CC |
| | | | CC | 0.94 (0.56-1.56) | 0.815 | 28 (0.24) | 638 (0.19) | 35 (0.17) | 725 (0.2) | ref | |
| | | | TC+TT | 0.56 (0.43-0.73) | 2.E-05 | | | | | 0.089 | TC+TT vs. CC |
| | | | TC+CC | 0.57 (0.43-0.76) | 1E-04 | | | | | 0.225 | TT vs.TC+CC |
| hCV15968043 | CYP4V2 (rs2292423) | A | AA | 0.33 (0.18-0.61) | 3E-04 | 15 (0.12) | 739 (0.21) | 44 (0.17) | 693 (0.17) | 0.040 | AA vs. TT |
| | | | AT | 0.61 (0.45-0.84) | 0.002 | 64 (0.53) | 1755 (0.5) | 122 (0.48) | 2021 (0.48) | 0.704 | AT vs. TT |
| | | | TT | 0.67 (0.46-0.98) | 0.038 | 42 (0.35) | 1021 (0.29) | 90 (0.35) | 1456 (0.35) | ref | |
| | | | AT+AA | 0.53 (0.4-0.7) | 8.E-06 | | | | | 0.291 | AT+AA vs. TT |
| | | | AT+TT | 0.63 (0.5-0.81) | 2E-04 | | | | | 0.038 | AA vs.AT+TT |
| hCV16171263 | PRLR (rs16871473) | G | GA | 0.2 (0.06-0.68) | 0.01 | 3 (0.02) | 207 (0.06) | 20 (0.08) | 231 (0.06) | 0.043 | GA vs. AA |
| | | | AA | 0.61 (0.49-0.77) | 2.E-05 | 122 (0.98) | 3335 (0.94) | 238 (0.92) | 3951 (0.94) | ref | |
| | | | GA+GG | 0.2 (0.06-0.68) | 0.01 | | | | | 0.041 | GA+GG vs. AA |
| | | | GA+AA | 0.58 (0.47-0.73) | 2.E-06 | | | | | n/a | |
| hCV1859855 | GOLGA3 (rs2291260) | C | CC | 0.16 (0.04-0.56) | 0.004 | 3 (0.02) | 232 (0.07) | 14 (0.05) | 175 (0.04) | 0.049 | CC vs. TT |
| | | | CT | 0.6 (0.42-0.86) | 0.005 | 48 (0.39) | 1207 (0.34) | 96 (0.37) | 1453 (0.35) | 0.899 | CT vs. TT |
| | | | TT | 0.6 (0.45-0.81) | 8E-04 | 71 (0.58) | 2088 (0.59) | 147 (0.57) | 2522 (0.61) | ref | |
| | | | CT+CC | 0.52 (0.37-0.73) | 2E-04 | | | | | 0.631 | CT+CC vs. TT |
| | | | CT+TT | 0.6 (0.48-0.76) | 1.E-05 | | | | | 0.045 | CC vs.CT+TT |
| hCV1948599 | CSMD2 (rs504527) | A | AA | 0.51 (0.31-0.84) | 0.008 | 24 (0.2) | 765 (0.22) | 56 (0.22) | 945 (0.22) | 0.198 | AA vs. CC |
| | | | AC | 0.49 (0.35-0.68) | 2.E-05 | 53 (0.43) | 1718 (0.49) | 137 (0.53) | 2113 (0.5) | 0.044 | AC vs. CC |
| | | | CC | 0.86 (0.58-1.27) | 0.44 | 45 (0.37) | 1005 (0.29) | 64 (0.25) | 1144 (0.27) | ref | |
| | | | AC+AA | 0.49 (0.38-0.65) | 4.E-07 | | | | | 0.044 | AC+AA vs. CC |
| | | | AC+CC | 0.61 (0.47-0.78) | 1.E-04 | | | | | 0.740 | AA vs.AC+CC |
| hCV1952126 | (rs7223784) | A | AA | 0.54 (0.4-0.74) | 9.E-05 | 64 (0.52) | 1976 (0.56) | 138 (0.53) | 2258 (0.54) | 0.019 | AA vs. CC |
| | | | AC | 0.52 (0.36-0.75) | 5E-04 | 43 (0.35) | 1343 (0.38) | 101 (0.39) | 1598 (0.38) | 0.018 | AC vs. CC |
| | | | CC | 1.21 (0.61-2.42) | 0.584 | 17 (0.14) | 236 (0.07) | 19 (0.07) | 338 (0.08) | ref | |
| | | | AC+AA | 0.53 (0.42-0.67) | 2.E-07 | | | | | 0.014 | AC+AA vs. CC |
| | | | AC+CC | 0.62 (0.45-0.85) | 0.003 | | | | | 0.507 | AA vs.AC+CC |
| hCV22272267 | KLKB1 (rs3733402) | A | AA | 0.41 (0.26-0.65) | 2E-04 | 27 (0.22) | 1141 (0.32) | 66 (0.26) | 1092 (0.26) | 0.141 | AA vs. GG |
| | | | AG | 0.67 (0.5-0.91) | 0.01 | 70 (0.56) | 1723 (0.49) | 126 (0.49) | 2112 (0.5) | 0.793 | AG vs. GG |
| | | | GG | 0.67 (0.42-1.06) | 0.085 | 28 (0.22) | 680 (0.19) | 64 (0.25) | 985 (0.24) | ref | |
| | | | AG+AA | 0.57 (0.44-0.74) | 2.E-05 | | | | | 0.665 | AG+AA vs. GG |
| | | | AG+GG | 0.67 (0.52-0.86) | 0.002 | | | | | 0.045 | AA vs.AG+GG |
| hCV2434510 | RNASE7 (rs1243469) | C | CC | 0.8 (0.07-9.85) | 0.863 | 1 (0.01) | 46 (0.01) | 2 (0.01) | 44 (0.01) | 0.804 | CC vs. TT |
| | | | CT | 0.35 (0.2-0.61) | 2E-04 | 17 (0.14) | 699 (0.2) | 55 (0.22) | 797 (0.19) | 0.047 | CT vs. TT |
| | | | TT | 0.66 (0.52-0.84) | 9E-04 | 106 (0.85) | 2774 (0.79) | 196 (0.77) | 3324 (0.8) | ref | |
| | | | CT+CC | 0.36 (0.21-0.62) | 3E-04 | | | | | 0.046 | CT+CC vs. TT |
| | | | CT+TT | 0.59 (0.47-0.74) | 4.E-06 | | | | | 0.872 | CC vs.CT+TT |
| hCV25610857 | (rs8176693) | T | TT | 1.61 (0.13-19.25) | 0.709 | 2 (0.02) | 31 (0.01) | 1 (0) | 28 (0.01) | 0.307 | TT vs. CC |
| | | | TC | 0.92 (0.55-1.54) | 0.755 | 31 (0.25) | 604 (0.17) | 34 (0.13) | 546 (0.13) | 0.081 | TC vs. CC |
| | | | CC | 0.5 (0.39-0.64) | 9.E-08 | 89 (0.73) | 2886 (0.82) | 224 (0.86) | 3629 (0.86) | ref | |
| | | | TC+TT | 0.94 (0.57-1.55) | 0.822 | | | | | 0.058 | TC+TT vs. CC |
| | | | TC+CC | 0.57 (0.45-0.71) | 8.E-07 | | | | | 0.349 | TT vs.TC+CC |
| hCV25631989 | ATF6 (rs1135983) | T | TT | 0.98 (0.07-12.9) | 0.985 | 1 (0.01) | 23 (0.01) | 2 (0.01) | 37 (0.01) | 0.659 | TT vs. CC |
| | | | TC | 1.09 (0.66-1.8) | 0.725 | 32 (0.26) | 505 (0.14) | 37 (0.14) | 604 (0.14) | 0.007 | TC vs. CC |
| | | | CC | 0.49 (0.38-0.63) | 5.E-08 | 88 (0.73) | 2963 (0.85) | 218 (0.85) | 3652 (0.85) | ref | |

Table 4. Association of statin with VT in 27 SNP genotype subgroups in MEGA

| hCV # | Gene Symbol (SNP rs #) | Risk Allele | Subgroup | OR (95%CI) | P | statin users, cases | statin nonuser, cases | statin users, controls | statin nonusers, controls | p(int) statin* SNP | Comparison for p(int) statin*SNP |
|---|---|---|---|---|---|---|---|---|---|---|---|
| | | | TC+TT | 1.09 (0.67-1.78) | 0.723 | | | | | 0.006 | TC+TT vs. CC |
| | | | TC+CC | 0.58 (0.46-0.72) | 2.E-06 | | | | | 0.749 | TT vs. TC+CC |
| hCV26175114 | TUBA4A (rs3731892) | G | GG | 1.19 (0.18-7.89) | 0.854 | 3 (0.02) | 83 (0.02) | 2 (0.01) | 73 (0.02) | 0.414 | GG vs. AA |
| | | | GA | 0.28 (0.14-0.57) | 4E-04 | 10 (0.08) | 638 (0.18) | 44 (0.17) | 749 (0.18) | 0.024 | GA vs. AA |
| | | | AA | 0.62 (0.49-0.79) | 1E-04 | 109 (0.89) | 2785 (0.79) | 210 (0.82) | 3336 (0.8) | ref | |
| | | | GA+GG | 0.34 (0.18-0.64) | 9E-04 | | | | | 0.054 | GA+GG vs. AA |
| | | | GA+AA | 0.57 (0.45-0.71) | 9.E-07 | | | | | 0.351 | GG vs. GA+AA |
| hCV27474984 | PIK3R1 (rs3756668) | A | AA | 0.64 (0.39-1.05) | 0.079 | 26 (0.21) | 773 (0.22) | 50 (0.2) | 876 (0.21) | 0.134 | AA vs. GG |
| | | | AG | 0.41 (0.3-0.57) | 9.E-08 | 54 (0.44) | 1761 (0.5) | 144 (0.57) | 1977 (0.47) | 0.002 | AG vs. GG |
| | | | GG | 0.98 (0.66-1.48) | 0.94 | 43 (0.35) | 985 (0.28) | 60 (0.24) | 1310 (0.31) | ref | |
| | | | AG+AA | 0.47 (0.36-0.62) | 4.E-08 | | | | | 0.003 | AG+AA vs. GG |
| | | | AG+GG | 0.57 (0.44-0.73) | 1.E-05 | | | | | 0.896 | AA vs. AG+GG |
| hCV27502514 | KLF3 (rs3796533) | A | AA | 2.69 (0.78-9.27) | 0.118 | 9 (0.07) | 97 (0.03) | 4 (0.02) | 109 (0.03) | 0.011 | AA vs. GG |
| | | | AG | 0.57 (0.37-0.87) | 0.008 | 36 (0.29) | 1005 (0.28) | 69 (0.27) | 1155 (0.28) | 0.502 | AG vs. GG |
| | | | GG | 0.53 (0.4-0.7) | 5.E-06 | 79 (0.64) | 2450 (0.69) | 185 (0.72) | 2910 (0.7) | ref | |
| | | | AG+AA | 0.68 (0.46-1) | 0.048 | | | | | 0.162 | AG+AA vs. GG |
| | | | AG+GG | 0.54 (0.43-0.68) | 1.E-07 | | | | | 0.014 | AA vs. AG+GG |
| hCV3054799 | KIF6 (rs20455) | G | GG | 0.52 (0.29-0.95) | 0.032 | 17 (0.14) | 515 (0.14) | 37 (0.14) | 587 (0.14) | 0.408 | GG vs. AA |
| | | | GA | 0.83 (0.6-1.15) | 0.261 | 66 (0.53) | 1581 (0.44) | 96 (0.37) | 1901 (0.45) | 0.003 | GA vs. AA |
| | | | AA | 0.41 (0.29-0.58) | 1.E-06 | 42 (0.34) | 1462 (0.41) | 125 (0.48) | 1710 (0.41) | ref | |
| | | | GA+GG | 0.74 (0.56-0.99) | 0.041 | | | | | 0.006 | GA+GG vs. AA |
| | | | GA+AA | 0.59 (0.47-0.75) | 2.E-05 | | | | | 0.763 | GG vs. GA+AA |
| hCV3054805 | KIF6 (rs2894424) | G | GG | 0.57 (0.34-0.96) | 0.035 | 23 (0.19) | 666 (0.19) | 46 (0.18) | 741 (0.18) | 0.181 | GG vs. CC |
| | | | GC | 0.78 (0.57-1.08) | 0.134 | 66 (0.55) | 1621 (0.46) | 104 (0.41) | 2019 (0.48) | 0.003 | GC vs. CC |
| | | | CC | 0.38 (0.25-0.57) | 3.E-06 | 32 (0.26) | 1204 (0.34) | 106 (0.41) | 1431 (0.34) | ref | |
| | | | GC+GG | 0.72 (0.55-0.95) | 0.018 | | | | | 0.005 | GC+GG vs. CC |
| | | | GC+CC | 0.58 (0.45-0.74) | 2.E-05 | | | | | 0.960 | GG vs. GC+CC |
| hCV3054808 | KIF6 (rs9462535) | A | AA | 0.49 (0.28-0.87) | 0.015 | 18 (0.15) | 584 (0.17) | 42 (0.16) | 675 (0.16) | 0.580 | AA vs. CC |
| | | | AC | 0.8 (0.57-1.1) | 0.169 | 64 (0.52) | 1598 (0.46) | 99 (0.39) | 1957 (0.47) | 0.009 | AC vs. CC |
| | | | CC | 0.43 (0.3-0.63) | 1.E-05 | 40 (0.33) | 1313 (0.38) | 116 (0.45) | 1565 (0.37) | ref | |
| | | | AC+AA | 0.71 (0.53-0.94) | 0.016 | | | | | 0.024 | AC+AA vs. CC |
| | | | AC+CC | 0.6 (0.47-0.77) | 4.E-05 | | | | | 0.612 | AA vs. AC+CC |
| hCV3054813 | KIF6 (rs9471077) | G | GG | 0.48 (0.28-0.84) | 0.011 | 19 (0.16) | 593 (0.17) | 44 (0.17) | 663 (0.16) | 0.561 | GG vs. AA |
| | | | GA | 0.82 (0.6-1.14) | 0.246 | 65 (0.53) | 1606 (0.46) | 98 (0.38) | 1977 (0.47) | 0.005 | GA vs. AA |
| | | | AA | 0.41 (0.28-0.6) | 5.E-06 | 38 (0.31) | 1294 (0.37) | 115 (0.45) | 1555 (0.37) | ref | |
| | | | GA+GG | 0.72 (0.54-0.95) | 0.021 | | | | | 0.015 | GA+GG vs. AA |
| | | | GA+AA | 0.6 (0.47-0.77) | 5.E-05 | | | | | 0.531 | GG vs. GA+AA |
| hCV3054822 | (rs11751357) | A | AA | 0.44 (0.18-1.08) | 0.074 | 7 (0.06) | 270 (0.08) | 17 (0.07) | 298 (0.07) | 0.819 | AA vs. TT |
| | | | AT | 0.9 (0.65-1.26) | 0.542 | 64 (0.52) | 1362 (0.39) | 89 (0.35) | 1646 (0.39) | 0.002 | AT vs. TT |
| | | | TT | 0.42 (0.3-0.58) | 2.E-07 | 51 (0.42) | 1852 (0.53) | 151 (0.59) | 2248 (0.54) | ref | |
| | | | AT+AA | 0.82 (0.6-1.11) | 0.2 | | | | | 0.004 | AT+AA vs. TT |
| | | | AT+TT | 0.6 (0.48-0.75) | 1.E-05 | | | | | 0.620 | AA vs. AT+TT |
| hCV3230113 | CYP4V2 (rs1053094) | T | TT | 0.36 (0.22-0.57) | 2.E-05 | 25 (0.21) | 1041 (0.3) | 67 (0.26) | 990 (0.24) | 0.060 | TT vs. AA |
| | | | TA | 0.67 (0.49-0.92) | 0.012 | 64 (0.53) | 1739 (0.5) | 119 (0.46) | 2132 (0.51) | 0.937 | TA vs. AA |
| | | | AA | 0.69 (0.45-1.07) | 0.096 | 32 (0.26) | 732 (0.21) | 73 (0.28) | 1078 (0.26) | ref | |
| | | | TA+TT | 0.54 (0.42-0.71) | 5.E-06 | | | | | 0.464 | TA+TT vs. AA |
| | | | TA+AA | 0.67 (0.52-0.86) | 0.002 | | | | | 0.024 | TT vs. TA+AA |
| hCV470708 | THBS2 (rs10945408) | T | TT | 0.82 (0.44-1.51) | 0.52 | 19 (0.15) | 373 (0.1) | 27 (0.11) | 400 (0.1) | 0.878 | TT vs. GG |
| | | | TG | 0.4 (0.29-0.58) | 8.E-07 | 42 (0.34) | 1519 (0.43) | 124 (0.48) | 1823 (0.43) | 0.021 | TG vs. GG |
| | | | GG | 0.73 (0.53-1.01) | 0.061 | 64 (0.51) | 1673 (0.47) | 106 (0.41) | 1982 (0.47) | ref | |
| | | | TG+TT | 0.48 (0.35-0.65) | 3.E-06 | | | | | 0.068 | TG+TT vs. GG |
| | | | TG+GG | 0.55 (0.44-0.7) | 1.E-06 | | | | | 0.338 | TT vs. TG+GG |
| hCV491830 | EPS8L2 (rs3087548) | T | TT | 0.48 (0.32-0.72) | 4E-04 | 34 (0.27) | 1167 (0.33) | 91 (0.32) | 1302 (0.31) | 0.036 | TT vs. CC |
| | | | TC | 0.55 (0.4-0.75) | 2E-04 | 62 (0.5) | 1700 (0.48) | 136 (0.53) | 2020 (0.48) | 0.070 | TC vs. CC |
| | | | CC | 0.97 (0.59-1.6) | 0.919 | 29 (0.23) | 666 (0.19) | 40 (0.16) | 846 (0.2) | ref | |
| | | | TC+TT | 0.52 (0.41-0.67) | 3.E-07 | | | | | 0.035 | TC+TT vs. CC |
| | | | TC+CC | 0.64 (0.49-0.83) | 9E-04 | | | | | 0.236 | TT vs. TC+CC |
| hCV8241630 | F11 (rs925451) | A | AA | 0.44 (0.24-0.78) | 0.005 | 18 (0.15) | 726 (0.21) | 38 (0.15) | 627 (0.15) | 0.070 | AA vs. GG |
| | | | AG | 0.52 (0.37-0.71) | 6.E-05 | 58 (0.47) | 1729 (0.49) | 125 (0.48) | 1922 (0.46) | 0.116 | AG vs. GG |
| | | | GG | 0.78 (0.54-1.12) | 0.175 | 47 (0.38) | 1079 (0.31) | 95 (0.37) | 1650 (0.39) | ref | |
| | | | AG+AA | 0.49 (0.37-0.65) | 9.E-07 | | | | | 0.052 | AG+AA vs. GG |
| | | | AG+GG | 0.61 (0.48-0.78) | 7.E-05 | | | | | 0.203 | AA vs. AG+GG |

Table 4. Association of statin with VT in 27 SNP genotype subgroups in MEGA

| hCV # | Gene Symbol (SNP rs #) | Risk Allele | Subgroup | OR (95%CI) | P | statin users, cases | statin nonuser, cases | statin users, controls | statin nonusers, controls | p(int) statin* SNP | Comparison for p(int) statin*SNP |
|---|---|---|---|---|---|---|---|---|---|---|---|
| hCV8919444 | F5 (rs4524) | T | TT | 0.66 (0.5-0.87) | 0.004 | 86 (0.69) | 2176 (0.61) | 135 (0.53) | 2295 (0.55) | 0.110 | TT vs. CC |
| | | | TC | 0.5 (0.34-0.74) | 5E-04 | 36 (0.29) | 1189 (0.34) | 104 (0.41) | 1589 (0.38) | 0.272 | TC vs. CC |
| | | | CC | 0.2 (0.05-0.89) | 0.034 | 2 (0.02) | 181 (0.05) | 17 (0.07) | 304 (0.07) | ref | |
| | | | TC+TT | 0.6 (0.48-0.75) | 9.E-06 | | | | | 0.150 | TC+TT vs. CC |
| | | | TC+CC | 0.46 (0.32-0.68) | 6.E-05 | | | | | 0.065 | TT vs. TC+CC |
| hDV68530934 | TF 1208 I/D (hDV68530934) | A | AA | 0.78 (0.49-1.23) | 0.281 | 33 (0.26) | 775 (0.22) | 49 (0.19) | 876 (0.21) | 0.036 | AA vs. CC |
| | | | AC | 0.62 (0.45-0.84) | 0.002 | 66 (0.53) | 1777 (0.5) | 127 (0.49) | 2081 (0.5) | 0.099 | AC vs. CC |
| | | | CC | 0.39 (0.25-0.62) | 6.E-05 | 26 (0.21) | 1004 (0.28) | 83 (0.32) | 1242 (0.3) | ref | |
| | | | AC+AA | 0.66 (0.51-0.86) | 0.002 | | | | | 0.046 | AC+AA vs. CC |
| | | | AC+CC | 0.53 (0.41-0.68) | 1.E-06 | | | | | 0.153 | AA vs. AC+CC |

27 SNPs (shown above in Table 4) had a p(int) statin*SNP <0.05 (Wald test) in any model.

p(int) statin*SNP: P value <0.05 (Wald test) for statin*SNP interaction term (ModelFormula: VTE~ SNP + statin user or nonuser + SNP*statin + age + sex) is specific for the subgroup shown.

Endpoint: VT (including DVT and PE)

Parameter: statin use (statin users or statin nonusers)

TABLE 5

Table 5. Association of 75 SNPs with VT risk in statin user and statin nonuser subgroups in MEGA (additive P <0.05).

| p(int) statin*SNP (additive) | marker (hCV #) | Gene (SNP rs #) | Risk Allele | parameter | Strata | OR (95%CI) | P-value | Allele1 (allele freq) | Allele2 (allele freq) | Genot ype 1 | Case 1 | Contr ol 1 | Genot ype 2 | Case 2 | Contr ol 2 | Genot ype 3 | Case 3 | Contr ol 3 |
|---|---|---|---|---|---|---|---|---|---|---|---|---|---|---|---|---|---|---|
| 0.034221351 | hCV8918444 | F5 (rs4524) | T | T_vs_C | statin_0 | 1.26 (1.17-1.35) | 5E-10 | C(0.26) | T(0.74) | CC | 181 | 304 | CT | 1189 | 1589 | TT | 2176 | 2295 |
| | | | | | statin_1 | 1.95 (1.31-2.90) | 0.001 | C(0.37) | T(0.73) | CC | 2 | 17 | CT | 38 | 104 | TT | 86 | 136 |
| 0.04127343 | hCV11786258 | KLKB1 (rs4253903) | A | A_vs_G | statin_0 | 1.23 (1.15-1.31) | 3E-10 | A(0.39) | G(0.61) | AA | 700 | 642 | AG | 1742 | 2007 | GG | 1114 | 1592 |
| | | | | | statin_1 | 0.88 (0.64-1.2) | 0.41 | A(0.4) | G(0.5) | AG | 61 | 122 | GG | 48 | 94 | AA | 15 | 41 |
| 0.048438831 | hCV9241630 | F11 (rs925451) | A | A_vs_G | statin_0 | 1.34 (1.26-1.43) | 5E-19 | A(0.38) | G(0.62) | AA | 725 | 627 | AG | 1729 | 1922 | GG | 1079 | 1550 |
| | | | | | statin_1 | 0.96 (0.7-1.32) | 0.805 | A(0.39) | G(0.61) | AA | 53 | 125 | AG | 18 | 38 | GG | 47 | 95 |
| 0.047140799 | hCV25810857 | (rs6176693) | T | T_vs_C | statin_0 | 1.35 (1.2-1.51) | 3E-07 | C(0.93) | T(0.07) | CC | 2886 | 3629 | CT | 804 | 546 | TT | 31 | 28 |
| | | | | | statin_1 | 2.27 (1.37-3.77) | 0.002 | C(0.93) | T(0.07) | CC | 89 | 224 | CT | 31 | 34 | TT | 2 | 1 |
| 0.048367213 | hCV491830 | EPS8L2 (rs3087546) | T | T_vs_C | statin_0 | 1.07 (1-1.14) | 0.046 | C(0.45) | T(0.55) | CC | 666 | 846 | CT | 1700 | 2029 | TT | 1167 | 1302 |
| | | | | | statin_1 | 0.76 (0.56-1.05) | 0.095 | C(0.42) | T(0.58) | CC | 29 | 136 | CT | 62 | 136 | TT | 34 | 81 |
| 0.06328739 | hCV3230113 | CYP4V2 (rs1053094) | T | T_vs_A | statin_0 | 1.25 (1.17-1.33) | 1E-11 | A(0.51) | T(0.49) | AA | 732 | 1078 | AT | 1739 | 2132 | TT | 1041 | 990 |
| | | | | | statin_1 | 0.93 (0.69-1.26) | 0.641 | A(0.51) | T(0.49) | AA | 32 | 73 | AT | 64 | 119 | TT | 25 | 67 |
| 0.065533427 | hCV15966043 | CYP4V2 (rs2292423) | A | A_vs_T | statin_0 | 1.23 (1.16-1.32) | 1E-10 | A(0.58) | T(0.42) | AA | 739 | 693 | AT | 1755 | 2021 | TT | 1021 | 1456 |
| | | | | | statin_1 | 0.91 (0.66-1.25) | 0.547 | A(0.41) | T(0.59) | AA | 15 | 44 | AT | 64 | 122 | TT | 42 | 90 |
| 0.069827287 | hCV27474984 | PIK3R1 (rs3756668) | A | A_vs_G | statin_0 | 1.09 (1.02-1.16) | 0.007 | A(0.55) | G(0.45) | AA | 773 | 876 | AG | 1761 | 1977 | GG | 985 | 1310 |
| | | | | | statin_1 | 0.81 (0.59-1.11) | 0.195 | A(0.48) | G(0.52) | AA | 26 | 60 | AG | 54 | 144 | GG | 43 | 60 |
| 0.078778264 | hCV11633415 | LOC730144 (rs4262503) | T | T_vs_C | statin_0 | 1.17 (1.04-1.32) | 0.011 | C(0.92) | T(0.08) | CC | 25 | 21 | CT | 431 | 616 | TT | 3105 | 3561 |
| | | | | | statin_1 | 0.68 (0.38-1.22) | 0.201 | C(0.07) | T(0.93) | CT | 23 | 36 | TT | 101 | 224 | 0 | 0 | 0 |
| 0.09822195 | hCV2442143 | ASAH1 (rs12544854) | T | T_vs_C | statin_0 | 0.92 (0.87-0.98) | 0.013 | C(0.49) | T(0.51) | CC | 896 | 1106 | CT | 1707 | 2055 | TT | 791 | 1032 |
| | | | | | statin_1 | 1.2 (0.88-1.64) | 0.242 | C(0.54) | T(0.46) | CC | 32 | 73 | CT | 57 | 134 | TT | 32 | 50 |
| 0.09898911 | hCV12066124 | F11 (rs2036914) | C | C_vs_T | statin_0 | 1.32 (1.24-1.41) | 5E-18 | C(0.51) | T(0.49) | CC | 1245 | 1138 | CT | 1679 | 1858 | TT | 683 | 994 |
| | | | | | statin_1 | 1 (0.73-1.37) | 0.999 | C(0.51) | T(0.49) | CC | 27 | 68 | CT | 72 | 130 | TT | 23 | 61 |
| 0.10233127 | hCV9102827 | GPATCH4 (rs3795733) | C | C_vs_T | statin_0 | 1.09 (1.02-1.17) | 0.015 | C(0.26) | T(0.74) | CC | 324 | 309 | CT | 1267 | 1318 | TT | 1857 | 2113 |
| | | | | | statin_1 | 1.45 (1.04-2.04) | 0.031 | C(0.22) | T(0.78) | CC | 13 | 13 | CT | 43 | 74 | TT | 63 | 138 |
| 0.110882818 | hCV1952126 | (rs7223784) | A | A_vs_C | statin_0 | 1.08 (1.01-1.16) | 0.027 | A(0.73) | C(0.27) | AA | 1976 | 2258 | AC | 1343 | 1598 | CC | 236 | 238 |
| | | | | | statin_1 | 0.83 (0.6-1.14) | 0.248 | A(0.73) | C(0.27) | AA | 64 | 138 | AC | 43 | 101 | CC | 17 | 19 |
| 0.121999557 | hCV27859399 | ABO (rs7853989) | C | C_vs_G | statin_0 | 1.28 (1.16-1.42) | 3E-06 | C(0.09) | G(0.91) | CC | 54 | 37 | CG | 690 | 683 | GG | 2780 | 3480 |
| | | | | | statin_1 | 1.87 (1.17-2.99) | 0.009 | C(0.09) | G(0.91) | CC | 2 | 2 | CG | 34 | 43 | GG | 86 | 213 |
| 0.122240353 | hCV3230039 | F11 (rs2289252) | T | T_vs_C | statin_0 | 1.36 (1.27-1.44) | 1E-20 | C(0.6) | T(0.4) | CC | 958 | 1513 | CT | 1752 | 1985 | TT | 812 | 704 |
| | | | | | statin_1 | 1.05 (0.77-1.43) | 0.757 | C(0.61) | T(0.39) | CC | 59 | 123 | CT | 59 | 96 | TT | 20 | 40 |
| 0.126690785 | hCV22272267 | KLKB1 (rs3733402) | A | A_vs_G | statin_0 | 1.23 (1.15-1.32) | 9E-11 | A(0.51) | G(0.49) | AA | 1141 | 1092 | AG | 1723 | 2112 | GG | 680 | 985 |
| | | | | | statin_1 | 0.97 (0.71-1.31) | 0.822 | A(0.5) | G(0.5) | AA | 27 | 66 | AG | 70 | 125 | GG | 28 | 64 |
| 0.129651976 | hCV27477533 | (rs3756009) | T | T_vs_A | statin_0 | 1.32 (1.24-1.41) | 9E-18 | A(0.61) | T(0.39) | AA | 1029 | 1560 | AT | 1755 | 1960 | TT | 773 | 677 |
| | | | | | statin_1 | 1.03 (0.76-1.4) | 0.84 | A(0.6) | T(0.4) | AA | 61 | 128 | AT | 71 | 123 | TT | 21 | 41 |
| 0.135285269 | hCV16182835 | PTPN21 (rs2274736) | G | G_vs_A | statin_0 | 1.11 (1.03-1.19) | 0.003 | A(0.68) | G(0.32) | AA | 1501 | 1930 | AG | 1588 | 1846 | GG | 405 | 422 |
| | | | | | statin_1 | 0.85 (0.61-1.19) | 0.336 | A(0.63) | G(0.37) | AA | 52 | 102 | AG | 59 | 121 | GG | 11 | 33 |
| 0.142757821 | hCV15703897 | KLKB1 (rs2087505) | G | G_vs_A | statin_0 | 1.26 (1.14-1.4) | 1E-05 | A(0.11) | G(0.89) | AA | 26 | 69 | AG | 590 | 800 | GG | 2943 | 3331 |
| | | | | | statin_1 | 0.91 (0.59-1.41) | 0.674 | A(0.12) | G(0.88) | AA | 3 | 7 | AG | 28 | 48 | GG | 95 | 206 |
| 0.154022293 | hCV916107 | LOC729138 (rs1670659) | C | C_vs_T | statin_0 | 1.11 (1.04-1.19) | 0.002 | C(0.36) | T(0.64) | CC | 1540 | 1725 | CT | 1618 | 1974 | TT | 375 | 544 |
| | | | | | statin_1 | 0.97 (0.63-1.21) | 0.417 | C(0.68) | T(0.32) | CC | 50 | 118 | CT | 60 | 111 | TT | 13 | 26 |
| 0.164073369 | hCV27474895 | F11 (rs3756011) | A | A_vs_C | statin_0 | 1.34 (1.26-1.43) | 1E-19 | A(0.6) | C(0.4) | AA | 824 | 711 | AC | 1743 | 1975 | CC | 977 | 1509 |
| | | | | | statin_1 | 1.06 (0.78-1.43) | 0.702 | A(0.4) | C(0.6) | AA | 21 | 40 | AC | 60 | 126 | CC | 42 | 83 |
| 0.179080773 | hCV25474413 | F11 (rs3822057) | C | C_vs_A | statin_0 | 1.3 (1.22-1.39) | 3E-16 | A(0.52) | C(0.48) | AA | 729 | 1140 | AC | 1733 | 2072 | CC | 1079 | 985 |
| | | | | | statin_1 | 1.04 (0.76-1.42) | 0.808 | A(0.52) | C(0.48) | AA | 27 | 69 | AC | 71 | 131 | CC | 25 | 59 |
| 0.213997396 | hCV31523650 | AKT3 (rs12048930) | T | T_vs_C | statin_0 | 1.12 (1.04-1.21) | 0.003 | C(0.8) | T(0.2) | CC | 2196 | 2738 | CT | 1170 | 1277 | TT | 179 | 183 |
| | | | | | statin_1 | 0.88 (0.61-1.28) | 0.516 | C(0.78) | T(0.22) | CC | 79 | 160 | CT | 41 | 83 | TT | 4 | 15 |
| 0.224108979 | hCV16180170 | SERPINC1 (rs2227589) | T | T_vs_C | statin_0 | 1.24 (1.12-1.38) | 3E-05 | C(0.91) | T(0.09) | CC | 2791 | 3443 | CT | 697 | 698 | TT | 53 | 40 |
| | | | | | statin_1 | 1.71 (1.06-2.76) | 0.029 | C(0.92) | T(0.08) | CC | 94 | 219 | CT | 29 | 36 | TT | 2 | 3 |
| 0.229050466 | hCV1959855 | GOLGA3 (rs2291260) | C | C_vs_T | statin_0 | 1.12 (1.04-1.21) | 0.004 | C(0.22) | T(0.78) | CC | 232 | 175 | CT | 1207 | 1459 | TT | 2088 | 2522 |
| | | | | | statin_1 | 0.88 (0.6-1.29) | 0.507 | C(0.24) | T(0.76) | CC | 3 | 14 | CT | 48 | 96 | TT | 71 | 147 |
| 0.278514601 | hCV1376266 | GP6 (rs1654413) | A | A_vs_T | statin_0 | 0.89 (0.83-0.97) | 0.007 | A(0.2) | T(0.8) | AA | 122 | 165 | AT | 1043 | 1351 | TT | 2346 | 2678 |
| | | | | | statin_1 | 1.11 (0.75-1.62) | 0.581 | A(0.2) | T(0.81) | AA | 40 | 78 | AT | 77 | 172 | | | |
| 0.285044775 | hCV3230096 | CYP4V2 (rs3817184) | T | T_vs_C | statin_0 | 1.22 (1.14-1.3) | 1E-09 | C(0.59) | T(0.41) | CC | 1036 | 1442 | CT | 1750 | 2029 | TT | 774 | 724 |
| | | | | | statin_1 | 1.03 (0.76-1.39) | 0.864 | C(0.58) | T(0.42) | CC | 40 | 89 | CT | 63 | 124 | TT | 22 | 47 |
| 0.288005598 | hCV16170618 | MET (rs2237712) | G | G_vs_A | statin_0 | 1.2 (1.01-1.43) | 0.037 | A(0.97) | G(0.03) | AA | 3258 | 3822 | AG | 253 | 252 | GG | 7 | 3 |
| | | | | | statin_1 | 0.81 (0.39-1.66) | 0.559 | A(0.95) | G(0.05) | AA | 113 | 222 | AG | 11 | 25 | GG | 0 | 1 |
| 0.288035569 | hCV2532034 | F13B (rs6003) | G | G_vs_A | statin_0 | 1.13 (1.02-1.26) | 0.022 | A(0.91) | G(0.09) | AA | 2803 | 3455 | AG | 598 | 647 | GG | 51 | 51 |
| | | | | | statin_1 | 1.53 (0.98-2.65) | 0.133 | A(0.94) | G(0.06) | AA | 101 | 225 | AG | 19 | 30 | GG | 2 | 1 |
| 0.302357106 | hCV2915511 | OBSL1 (rs627530) | C | C_vs_T | statin_0 | 1.18 (1.01-1.38) | 0.036 | C(0.04) | T(0.96) | CC | 28 | 11 | CT | 253 | 289 | TT | 3293 | 3897 |
| | | | | | statin_1 | 1.84 (0.8-4.25) | 0.15 | C(0.03) | T(0.97) | CT | 11 | 13 | TT | 112 | 244 | 0 | 0 | 0 |
| 0.31936143 | hCV8726802 | F2 (rs1799963) | A | A_vs_G | statin_0 | 2.66 (2.05-3.44) | 1E-13 | A(0.01) | G(0.99) | AA | 1 | 0 | AG | 186 | 85 | GG | 3344 | 4104 |
| | | | | | statin_1 | 1.23 (0.29-5.25) | 0.784 | A(0.01) | G(0.99) | AG | 3 | 5 | GG | 120 | 252 | 0 | 0 | 0 |
| 0.343434006 | hCV2590988 | ADCY9 (rs2230739) | C | C_vs_T | statin_0 | 1.09 (1-1.18) | 0.037 | C(0.73) | T(0.27) | CC | 1930 | 2199 | CT | 1319 | 1535 | TT | 217 | 317 |
| | | | | | statin_1 | 0.91 (0.64-1.29) | 0.592 | C(0.76) | T(0.24) | CC | 69 | 148 | CT | 48 | 91 | TT | 8 | 14 |
| 0.345514722 | hCV2303891 | APOH (rs1801690) | C | C_vs_T | statin_0 | 1.26 (1.08-1.45) | 0.002 | C(0.94) | G(0.06) | CC | 3249 | 3730 | CG | 300 | 455 | GG | 10 | 5 |
| | | | | | statin_1 | 0.89 (0.45-1.76) | 0.743 | C(0.95) | G(0.05) | CC | 112 | 235 | CG | 13 | 22 | GG | 0 | 1 |
| 0.35694798 | hCV8911768 | SERPINC1 (rs941988) | T | T_vs_C | statin_0 | 1.24 (1.12-1.38) | 3E-05 | C(0.91) | T(0.09) | CC | 2769 | 3458 | CT | 696 | 707 | TT | 55 | 42 |
| | | | | | statin_1 | 1.59 (0.97-2.62) | 0.066 | C(0.92) | T(0.08) | CC | 93 | 220 | CT | 28 | 36 | TT | 1 | 3 |
| 0.367055135 | hCV11500470 | (rs1800788) | T | T_vs_C | statin_0 | 1.21 (1.13-1.31) | 4E-07 | C(0.79) | T(0.21) | CC | 2057 | 2674 | CT | 1291 | 1325 | TT | 211 | 205 |
| | | | | | statin_1 | 1.02 (0.72-1.45) | 0.889 | C(0.76) | T(0.24) | CC | 70 | 149 | CT | 46 | 92 | TT | 8 | 17 |
| 0.367159692 | hCV22273419 | GP6 (rs2304167) | C | C_vs_T | statin_0 | 0.89 (0.82-0.96) | 0.004 | C(0.2) | T(0.8) | CC | 121 | 167 | CT | 1048 | 1388 | TT | 2372 | 2671 |
| | | | | | statin_1 | 1.06 (0.73-1.54) | 0.751 | C(0.19) | T(0.81) | CC | 5 | 12 | CT | 40 | 73 | TT | 80 | 169 |
| 0.371584565 | hCV1202880 | MTHFR (rs1801133) | G | G_vs_A | statin_0 | 1.09 (1-1.17) | 0.038 | A(0.3) | G(0.7) | AA | 172 | 269 | AG | 1695 | 1988 | GG | 1684 | 1934 |
| | | | | | statin_1 | 1.28 (0.9-1.82) | 0.177 | A(0.32) | G(0.68) | AA | 5 | 23 | AG | 57 | 117 | GG | 61 | 115 |
| 0.371786362 | hCV1376342 | GP6 (rs1654416) | C | C_vs_T | statin_0 | 0.88 (0.81-0.95) | 0.002 | C(0.2) | T(0.8) | CC | 115 | 159 | CT | 1029 | 1342 | TT | 2386 | 2683 |
| | | | | | statin_1 | 1.05 (0.72-1.51) | 0.809 | C(0.19) | T(0.81) | CC | 5 | 14 | CT | 39 | 71 | TT | 79 | 172 |
| 0.406756226 | hCV11075250 | F5 (rs6025) | T | T_vs_C | statin_0 | 3.42 (2.92-4.01) | 1E-51 | C(0.97) | T(0.03) | CC | 2934 | 3930 | CT | 655 | 207 | TT | 23 | 7 |
| | | | | | statin_1 | 4.78 (2.34-9.77) | 2E-05 | C(0.98) | T(0.02) | CC | 99 | 239 | CT | 22 | 12 | TT | 2 | 0 |
| 0.407783342 | hCV2103346 | DKFZP564J102 (rs11733307) | C | C_vs_T | statin_0 | 1.07 (1.01-1.14) | 0.03 | C(0.44) | T(0.56) | CC | 753 | 862 | CT | 1731 | 1988 | TT | 1029 | 1341 |
| | | | | | statin_1 | 1.22 (0.9-1.65) | 0.201 | C(0.44) | T(0.56) | CC | 28 | 52 | CT | 62 | 122 | TT | 31 | 65 |
| 0.410870664 | hCV15860324 | PROCR (rs2069946) | C | C_vs_T | statin_0 | 1.33 (1.16-1.52) | 4E-05 | C(0.05) | T(0.95) | CC | 17 | 13 | CT | 431 | 398 | TT | 3073 | 3789 |
| | | | | | statin_1 | 1.03 (0.56-1.87) | 0.928 | C(0.06) | T(0.94) | CT | 14 | 29 | TT | 107 | 226 | 0 | 0 | 0 |
| 0.412798308 | hCV11503469 | FGG (rs2066654) | A | A_vs_T | statin_0 | 1.37 (1.28-1.47) | 5E-19 | A(0.27) | T(0.73) | AA | 405 | 299 | AT | 1544 | 1623 | TT | 1600 | 2265 |
| | | | | | statin_1 | 1.19 (0.86-1.64) | 0.301 | A(0.3) | T(0.7) | AA | 18 | 31 | AT | 52 | 111 | TT | 57 | 125 |
| 0.423539585 | hCV30582347 | F11 (rs4253418) | G | G_vs_A | statin_0 | 1.28 (1.09-1.51) | 0.003 | A(0.04) | G(0.96) | AA | 7 | 9 | AG | 231 | 349 | GG | 3317 | 3824 |
| | | | | | statin_1 | 0.99 (0.5-1.95) | 0.972 | A(0.04) | G(0.96) | AA | 2 | 2 | AG | 7 | 18 | GG | 114 | 237 |
| 0.435904222 | hCV293941 | NR1I2 (rs1523127) | C | C_vs_A | statin_0 | 1.11 (1.04-1.18) | 0.002 | A(0.62) | C(0.38) | AA | 1263 | 1578 | AC | 1667 | 2003 | CC | 617 | 608 |
| | | | | | statin_1 | 0.97 (0.71-1.33) | 0.85 | A(0.62) | C(0.38) | AA | 46 | 97 | AC | 62 | 121 | CC | 15 | 38 |
| 0.43932399 | hCV596331 | F9 (rs6048) | A | A_vs_G | statin_0 | 1.1 (1.04-1.17) | 0.001 | A(0.7) | G(0.3) | AA | 2171 | 2457 | AG | 766 | 905 | GG | 583 | 813 |
| | | | | | statin_1 | 1.22 (0.93-1.61) | 0.148 | A(0.69) | G(0.31) | AA | 83 | 161 | AG | 21 | 33 | GG | 19 | 62 |
| 0.45280106 | hCV9718961 | RDH13 (rs1654451) | A | A_vs_T | statin_0 | 0.89 (0.81-0.97) | 0.009 | A(0.16) | T(0.84) | AA | 68 | 104 | AT | 866 | 1114 | TT | 2591 | 2986 |
| | | | | | statin_1 | 1.04 (0.69-1.58) | 0.853 | A(0.15) | T(0.85) | AA | 3 | 9 | AT | 33 | 52 | TT | 86 | 183 |
| 0.469253388 | hCV30710896 | F2 (rs3136520) | T | T_vs_C | statin_0 | 1.28 (1.06-1.56) | 0.012 | C(0.98) | T(0.02) | CC | 3315 | 4011 | CT | 198 | 189 | TT | 7 | 4 |

Table 5. Association of 75 SNPs with VT risk in statin user and statin nonuser subgroups in MEGA (additive P <0.05).

| p(int) statin*SNP (additive) | marker (hCV #) | Gene (SNP rs #) | Risk Allele | parameter | Strata | OR (95%CI) | P-value | Allele1 (allele freq) | Allele2 (allele freq) | Genot ype 1 | Case 1 | Contr ol 1 | Genot ype 2 | Case 2 | Contr ol 2 | Genot ype 3 | Case 3 | Contr ol 3 |
|---|---|---|---|---|---|---|---|---|---|---|---|---|---|---|---|---|---|---|
| | | | | | statin_1 | 0.9 (0.37-2.18) | 0.808 | C(0.97) | T(0.03) | CC | 114 | 244 | CT | 7 | 14 | TT | 0 | 1 |
| 0.497858665 | hCV8717973 | GP6 (rs1613662) | A | A_vs_G | statin_0 | 1.17 (1.08-1.27) | 2E-04 | A(0.82) | G(0.18) | AA | 2480 | 2783 | AG | 854 | 1265 | GG | 93 | 136 |
| | | | | | statin_1 | 1.02 (0.69-1.5) | 0.922 | A(0.82) | G(0.18) | AA | 86 | 174 | AG | 34 | 73 | GG | 5 | 10 |
| 0.518388541 | hCV2892877 | FGA (rs6050) | C | C_vs_T | statin_0 | 1.38 (1.26-1.52) | 4E-12 | C(0.24) | T(0.76) | CC | 14 | 18 | CT | 1808 | 1810 | TT | 1485 | 2090 |
| | | | | | statin_1 | 1.19 (0.78-1.85) | 0.447 | C(0.25) | T(0.75) | CC | 1 | 0 | CT | 61 | 121 | TT | 52 | 117 |
| 0.525140093 | hCV2489170 | (rs169713) | C | C_vs_T | statin_0 | 1.1 (1.02-1.19) | 0.015 | C(0.2) | T(0.8) | CC | 175 | 200 | CT | 1185 | 1305 | TT | 2135 | 2594 |
| | | | | | statin_1 | 0.96 (0.64-1.44) | 0.847 | C(0.2) | T(0.8) | CC | 3 | 5 | CT | 41 | 92 | TT | 78 | 160 |
| 0.541874674 | hCV11503414 | FGG (rs2066865) | A | A_vs_G | statin_0 | 1.37 (1.28-1.47) | 4E-19 | A(0.26) | G(0.74) | AA | 395 | 294 | AG | 1535 | 1623 | GG | 1564 | 2266 |
| | | | | | statin_1 | 1.23 (0.88-1.71) | 0.219 | A(0.29) | G(0.71) | AA | 15 | 20 | AG | 52 | 111 | GG | 54 | 126 |
| 0.546620019 | hCV15949414 | XYLB (rs2234629) | G | G_vs_A | statin_0 | 1.34 (1.15-1.56) | 2E-04 | A(0.05) | G(0.95) | AA | 13 | 14 | AG | 257 | 418 | GG | 3266 | 3782 |
| | | | | | statin_1 | 1.07 (0.49-2.36) | 0.862 | A(0.04) | G(0.96) | AG | 10 | 22 | GG | 112 | 237 | | 0 | 0 |
| 0.583697812 | hCV25597241 | AQP2 (rs3782320) | A | A_vs_G | statin_0 | 1.12 (1.01-1.25) | 0.038 | A(0.09) | G(0.91) | AA | 33 | 32 | AG | 607 | 659 | GG | 2877 | 3513 |
| | | | | | statin_1 | 0.96 (0.56-1.66) | 0.895 | A(0.09) | G(0.91) | AA | 0 | 3 | AG | 21 | 40 | GG | 101 | 216 |
| 0.619356604 | hCV16177220 | ODZ1 (rs2266911) | C | C_vs_T | statin_0 | 1.08 (1.02-1.17) | 0.009 | C(0.8) | T(0.2) | CC | 2513 | 2982 | CT | 593 | 737 | TT | 347 | 475 |
| | | | | | statin_1 | 1 (0.72-1.39) | 0.996 | C(0.83) | T(0.17) | CT | 23 | 34 | TT | 10 | 27 | CC | 92 | 195 |
| 0.621640282 | hCV27902808 | CYP4V2 (rs4253236) | T | T_vs_C | statin_0 | 0.85 (0.79-0.9) | 9E-07 | C(0.64) | T(0.36) | CC | 1597 | 1711 | CT | 1557 | 1935 | TT | 384 | 555 |
| | | | | | statin_1 | 0.92 (0.67-1.25) | 0.587 | C(0.61) | T(0.39) | CC | 44 | 100 | CT | 65 | 114 | TT | 13 | 45 |
| 0.652396012 | hCV11541681 | LOC200420 (rs2001490) | C | C_vs_G | statin_0 | 1.07 (1.01-1.14) | 0.032 | C(0.37) | G(0.63) | CC | 560 | 583 | CG | 1631 | 1849 | GG | 1351 | 1651 |
| | | | | | statin_1 | 0.99 (0.72-1.38) | 0.969 | C(0.35) | G(0.65) | CC | 15 | 28 | CG | 57 | 125 | GG | 52 | 104 |
| 0.66407521 | hDV71075942 | (rs8176719) | G | G_vs_T | statin_0 | 1.67 (1.58-1.79) | 6E-51 | G(0.34) | T(0.66) | GG | 669 | 512 | TG | 1896 | 1840 | TT | 951 | 1849 |
| | | | | | statin_1 | 1.81 (1.29-2.54) | 6E-04 | G(0.35) | T(0.65) | GG | 18 | 31 | TG | 80 | 120 | TT | 24 | 108 |
| 0.676072364 | hCV30690780 | AKT3 (rs10737888) | C | C_vs_A | statin_0 | 1.15 (1.07-1.24) | 2E-04 | A(0.76) | C(0.24) | AA | 2011 | 2315 | AC | 1236 | 1466 | CC | 268 | 212 |
| | | | | | statin_1 | 1.07 (0.76-1.51) | 0.702 | A(0.76) | C(0.24) | AA | 70 | 151 | AC | 42 | 91 | CC | 10 | 17 |
| 0.701826538 | hCV1825046 | PROCR (rs2069952) | C | C_vs_T | statin_0 | 0.84 (0.78-0.89) | 7E-08 | C(0.4) | T(0.6) | CC | 494 | 706 | CT | 1572 | 2005 | TT | 1457 | 1484 |
| | | | | | statin_1 | 0.79 (0.57-1.09) | 0.145 | C(0.4) | T(0.6) | CC | 15 | 38 | CT | 53 | 130 | TT | 53 | 91 |
| 0.713175712 | hCV31523608 | AKT3 (rs12744297) | G | G_vs_A | statin_0 | 1.13 (1.05-1.21) | 4E-04 | A(0.67) | G(0.33) | AA | 1482 | 1917 | AG | 1578 | 1823 | GG | 450 | 484 |
| | | | | | statin_1 | 1.2 (0.86-1.66) | 0.287 | A(0.66) | G(0.34) | AA | 45 | 110 | AG | 62 | 123 | GG | 15 | 26 |
| 0.715483519 | hCV15990789 | OTOG (rs2355466) | G | G_vs_A | statin_0 | 1.07 (1-1.14) | 0.045 | A(0.41) | G(0.59) | AA | 544 | 690 | AG | 1643 | 2001 | GG | 1320 | 1478 |
| | | | | | statin_1 | 0.99 (0.72-1.38) | 0.975 | A(0.36) | G(0.64) | AA | 15 | 33 | AG | 58 | 117 | GG | 48 | 101 |
| 0.737026509 | hCV30690777 | AKT3 (rs12045585) | A | A_vs_G | statin_0 | 1.14 (1.04-1.24) | 0.004 | A(0.14) | G(0.86) | AA | 99 | 73 | AG | 911 | 1035 | GG | 2565 | 3085 |
| | | | | | statin_1 | 1.07 (0.71-1.6) | 0.747 | A(0.16) | G(0.84) | AA | 4 | 7 | AG | 32 | 70 | GG | 85 | 180 |
| 0.738278618 | hCV15860433 | (rs2070006) | T | T_vs_C | statin_0 | 1.24 (1.16-1.32) | 1E-10 | C(0.61) | T(0.39) | CC | 1090 | 1550 | CT | 1749 | 1971 | TT | 713 | 559 |
| | | | | | statin_1 | 1.3 (0.96-1.75) | 0.091 | C(0.6) | T(0.4) | CC | 37 | 96 | CT | 60 | 119 | TT | 28 | 43 |
| 0.798652766 | hCV25748719 | NAP5 () | C | C_vs_T | statin_0 | 1.08 (1.01-1.17) | 0.036 | C(0.77) | T(0.23) | CC | 2163 | 2462 | CT | 1206 | 1490 | TT | 169 | 225 |
| | | | | | statin_1 | 1.15 (0.78-1.68) | 0.482 | C(0.77) | T(0.23) | CC | 74 | 152 | CT | 50 | 91 | TT | 1 | 13 |
| 0.804517403 | hCV2986566 | F9 (rs4149755) | A | A_vs_T | statin_0 | 1.15 (1.03-1.28) | 0.015 | A(0.06) | T(0.94) | AA | 113 | 106 | AT | 258 | 268 | TT | 3152 | 3826 |
| | | | | | statin_1 | 1.07 (0.65-1.76) | 0.783 | A(0.06) | T(0.94) | AA | 5 | 11 | AT | 8 | 9 | TT | 111 | 239 |
| 0.808271852 | hCV32291301 | KLKB1 (rs4253302) | A | A_vs_G | statin_0 | 1.2 (1.1-1.31) | 6E-05 | A(0.84) | G(0.16) | AA | 2649 | 2949 | AG | 835 | 1140 | GG | 79 | 114 |
| | | | | | statin_1 | 1.25 (0.83-1.87) | 0.292 | A(0.84) | G(0.16) | AA | 93 | 178 | AG | 29 | 70 | GG | 4 | 11 |
| 0.841280102 | hCV1841973 | (rs1799809) | T | T_vs_C | statin_0 | 0.94 (0.88-1) | 0.049 | C(0.65) | T(0.35) | CC | 1545 | 1756 | CT | 1565 | 1918 | TT | 405 | 525 |
| | | | | | statin_1 | 0.9 (0.67-1.23) | 0.515 | C(0.64) | T(0.36) | CC | 57 | 111 | CT | 48 | 105 | TT | 17 | 40 |
| 0.85593208 | hCV25990131 | CYP4V2 (rs13146272) | A | A_vs_C | statin_0 | 1.2 (1.12-1.28) | 2E-07 | A(0.64) | C(0.36) | AA | 1584 | 1665 | AC | 1430 | 1808 | CC | 351 | 527 |
| | | | | | statin_1 | 1.16 (0.84-1.6) | 0.358 | A(0.62) | C(0.38) | AA | 51 | 97 | AC | 55 | 107 | CC | 13 | 39 |
| 0.87783198 | hCV25820145 | PROCR (rs867186) | G | G_vs_A | statin_0 | 1.18 (1.07-1.29) | 5E-04 | A(0.87) | G(0.13) | AA | 2601 | 3203 | AG | 869 | 917 | GG | 74 | 52 |
| | | | | | statin_1 | 1.14 (0.73-1.77) | 0.565 | A(0.87) | G(0.13) | AA | 91 | 194 | AG | 30 | 61 | GG | 3 | 3 |
| 0.916970113 | hCV1841974 | (rs1799809) | G | G_vs_A | statin_0 | 1.15 (1.08-1.23) | 3E-05 | A(0.57) | G(0.43) | AA | 1007 | 1362 | AG | 1741 | 2046 | GG | 770 | 794 |
| | | | | | statin_1 | 1.17 (0.87-1.57) | 0.298 | A(0.56) | G(0.44) | AA | 38 | 82 | AG | 50 | 122 | GG | 34 | 53 |
| 0.927314636 | hCV203148 | AKT3 (rs1417121) | C | C_vs_G | statin_0 | 1.15 (1.08-1.23) | 6E-05 | C(0.72) | G(0.73) | CC | 378 | 318 | CG | 1434 | 1894 | GG | 1735 | 2171 |
| | | | | | statin_1 | 1.14 (0.82-1.59) | 0.44 | C(0.27) | G(0.73) | CC | 11 | 21 | CG | 52 | 99 | GG | 61 | 138 |
| 0.928029957 | hCV1841983 | PROC (rs5937) | C | C_vs_T | statin_0 | 1.13 (1.06-1.21) | 3E-04 | C(0.36) | T(0.64) | CC | 501 | 504 | CT | 1610 | 1873 | TT | 1392 | 1808 |
| | | | | | statin_1 | 1.12 (0.82-1.52) | 0.484 | C(0.36) | T(0.64) | CC | 22 | 34 | CT | 49 | 117 | TT | 50 | 107 |
| 0.96355332 | hCV30747430 | NR1I2 (rs11712211) | T | T_vs_C | statin_0 | 1.14 (1.06-1.24) | 8E-04 | C(0.82) | T(0.18) | CC | 2251 | 2803 | CT | 1095 | 1253 | TT | 179 | 148 |
| | | | | | statin_1 | 1.15 (0.78-1.69) | 0.484 | C(0.83) | T(0.17) | CC | 80 | 177 | CT | 35 | 74 | TT | 6 | 8 |
| 0.970695776 | hCV1841975 | PROC (rs1799810) | T | T_vs_A | statin_0 | 1.13 (1.06-1.21) | 1E-04 | A(0.57) | T(0.43) | AA | 1038 | 1374 | AT | 1748 | 2035 | TT | 765 | 789 |
| | | | | | statin_1 | 1.13 (0.85-1.52) | 0.403 | A(0.56) | T(0.44) | AA | 40 | 82 | AT | 49 | 122 | TT | 34 | 53 |

SNPs are ranked above in Table 5 by P(int) statin*SNP from the additive model.
p(int) statin*SNP: P value <0.05 (Wald test) for statin*SNP interaction term (ModelFormula: VTE~ SNP + statin user or nonuser + SNP*statin + age + sex) from an additive model.
Endpoint: VT (including DVT and PE)
Strata: statin_0 (statin nonusers), statin_1 (statin users)
Model: additive

| additive Pint statin*SNP | marker (hCV #) | Gene (SNP rs #) | RiskAl lele | parameter | Strata | OR (95%CI) | P value | Allele1 (allele freq) | Allele2 (allele freq) | Genot ype 1 | Case1 | Contr ol1 | Genot ype 2 | Case2 | Contr ol2 | Genot ype 3 | Case3 | Contr ol3 |
|---|---|---|---|---|---|---|---|---|---|---|---|---|---|---|---|---|---|---|
| 0.002528557 | hCV2211618 | ODT (rs2483950) | G | G_vs_C | statin_0 | 1.08 (1.02-1.15) | 0.015 | C(0.57) | G(0.43) | CC | 1088 | 1359 | CG | 1710 | 2002 | GG | 710 | 750 |
| | | | | | statin_1 | 0.66 (0.48-0.91) | 0.011 | C(0.53) | G(0.47) | CC | 46 | 74 | CG | 55 | 113 | GG | 13 | 58 |

**TABLE 6**

Table 8. Association of statin use and VTE in SNP genotype subgroups in MEGA, and VTE risk of that genotype in statin nonusers in MEGA (last 2 columns)

| Gene (rs #) | Risk Allele | Strata | model | OR(95%CI) for statin*VTE | P | P(int statin*S NP) | reference group for P(int statin*SNP) | statin users, cases | statin nonusers, cases | statin users, controls | statin nonusers, controls | OR(95%CI) for VTE risk | P for VTE risk |
|---|---|---|---|---|---|---|---|---|---|---|---|---|---|
| DDT (rs12483950) | G | GG | | 0.24 (0.13-0.44) | 6E-06 | 0.001202 | GGvs.CC | 13 (0.1) | 710 (0.2) | 58 (0.23) | 750 (0.18) | 1.18 (1.03-1.34) | 0.0134 |
| DDT (rs12483950) | G | GC | | 0.67 (0.49-0.92) | 0.013 | 0.494951 | GCvs.CC | 66 (0.53) | 1710 (0.49) | 118 (0.47) | 2002 (0.49) | 1.07 (0.98-1.18) | 0.2276 |
| DDT (rs12483950) | G | CC | | 0.78 (0.53-1.14) | 0.196 | 0.002529 | | 46 (0.37) | 1088 (0.31) | 74 (0.3) | 1358 (0.33) | 1.08 (1.02-1.15) | 0.0148 |
| DDT (rs12483950) | G | GC+CC | rec | 0.71 (0.56-0.91) | 0.006 | 0.001199 | GGvs.GC+CC | 13 | 710 | 58 | 750 | 1.13 (1.01-1.27) | 0.0309 |
| DDT (rs12483950) | G | GC+GG | dom | 0.52 (0.4-0.69) | 4E-06 | 0.077571 | GC+GGvs.CC | 79 | 2420 | 176 | 2752 | 1.1 (0.99-1.21) | 0.0633 |
| DDT (rs12483950) | G | | add | | | 0.002529 | | | | | | 1.08 (1.02-1.15) | 0.0148 |
| F2RL1 (rs1529505) | T | TT | | 0.47 (0.31-0.73) | 7E-04 | 0.067 | TTvs.CC | 31 (0.25) | 1095 (0.31) | 77 (0.3) | 1248 (0.3) | 1.08 (0.95-1.23) | 0.233 |
| F2RL1 (rs1529505) | T | TC | | 0.55 (0.4-0.75) | 2E-04 | 0.163 | TCvs.CC | 62 (0.5) | 1722 (0.49) | 131 (0.51) | 2025 (0.49) | 1.05 (0.93-1.18) | 0.457 |
| F2RL1 (rs1529505) | T | CC | | 0.88 (0.55-1.41) | 0.595 | | | 32 (0.26) | 709 (0.2) | 48 (0.19) | 872 (0.21) | ref | ref |
| F2RL1 (rs1529505) | T | TT | rec | 0.63 (0.49-0.82) | 5E-04 | 0.220 | TTvs.TT | 31 | 1095 | 77 | 1248 | 1.05 (0.95-1.15) | 0.350 |
| F2RL1 (rs1529505) | T | TC+TT | dom | 0.52 (0.4-0.67) | 4E-07 | 0.083 | TC+TTvs.CC | 93 | 2817 | 208 | 3273 | 1.06 (0.95-1.18) | 0.310 |
| LOC729672 (rs4334026) | T | TT | | 0.62 (0.31-1.23) | 0.168 | 0.199 | TTvs.CC | 15 (0.12) | 332 (0.09) | 22 (0.08) | 355 (0.08) | 1.11 (0.95-1.31) | 0.193 |
| LOC729672 (rs4334026) | T | TC | | 0.69 (0.5-0.95) | 0.023 | 0.082 | TCvs.CC | 63 (0.51) | 1521 (0.43) | 113 (0.44) | 1824 (0.43) | 0.99 (0.9-1.09) | 0.889 |
| LOC729672 (rs4334026) | T | CC | | 0.46 (0.32-0.65) | 1E-05 | | | 46 (0.37) | 1705 (0.48) | 124 (0.48) | 2030 (0.48) | ref | ref |
| LOC729672 (rs4334026) | T | TT | rec | 0.57 (0.45-0.72) | 2E-06 | 0.446 | TTvs.TT | 15 | 332 | 22 | 355 | 1.12 (0.96-1.31) | 0.164 |
| LOC729672 (rs4334026) | T | TC+TT | dom | 0.68 (0.51-0.91) | 0.009 | 0.060 | TC+TTvs.CC | 78 | 1853 | 135 | 2179 | 1.01 (0.93-1.11) | 0.777 |
| ASAH1 (rs12544854) | T | TT | | 0.85 (0.54-1.35) | 0.487 | 0.085 | TTvs.CC | 32 (0.2645) | 791 (0.2264) | 50 (0.1946) | 1032 (0.2456) | 0.85 (0.75-0.97) | 0.013 |
| ASAH1 (rs12544854) | T | TC | | 0.53 (0.38-0.73) | 1E-04 | 0.633 | TCvs.CC | 57 (0.4711) | 1707 (0.4886) | 134 (0.5214) | 2065 (0.4913) | 0.92 (0.82-1.02) | 0.113 |
| ASAH1 (rs12544854) | T | CC | | 0.5 (0.32-0.77) | 0.002 | | | 32 (0.2645) | 996 (0.2851) | 73 (0.284) | 1106 (0.2631) | ref | ref |
| ASAH1 (rs12544854) | T | TT | rec | 0.52 (0.4-0.67) | 8E-07 | 0.055 | TTvs.TT | 32 | 791 | 50 | 1032 | 0.9 (0.81-1) | 0.054 |
| ASAH1 (rs12544854) | T | TC+TT | dom | 0.61 (0.47-0.8) | 3E-04 | 0.404 | TC+TTvs.CC | 89 | 2498 | 184 | 3097 | 0.9 (0.81-0.99) | 0.032 |
| LOC730144 (rs4262503) | T | TC | | 1 (0.58-1.73) | 0.995 | 0.051 | TCvs.TT | 23 (0.19) | 431 (0.12) | 35 (0.14) | 618 (0.15) | 0.59 (0.33-1.06) | 0.080 |
| LOC730144 (rs4262503) | T | TT | | 0.52 (0.41-0.66) | 2E-07 | | | 101 (0.81) | 3105 (0.87) | 224 (0.86) | 3561 (0.85) | ref | ref |
| LOC730144 (rs4262503) | T | TT | rec | 0.98 (0.57-1.7) | 0.954 | 0.060 | TCvs.TT | 101 | 3105 | 224 | 3561 | 1.22 (1.07-1.39) | 0.003 |
| LOC730144 (rs4262503) | T | TC+TT | dom | 0.57 (0.46-0.72) | 1E-05 | n/a | | | | | | | |

SNPs in Table 8 had additive P interaction <0.1 in MEGA.
P(int) = P interaction from the Wald test for statin*SNP from the following model: VTE ~ SNP + statin user or nonuser + SNP*statin user or nonuser + age + sex.
OR (95%CI) and P value for VTE ~SNP in last 2 columns calculated in statin nonusers.
VT is interchangeably referred to as VTE.

| SNP | MODE | GENO_RESP | STATIN_USE | EVENTS_RESP | TOTAL_RESP | HR_RESP | HR 95% CI lower_RESP | HR 95% CI upper_RESP | P-value_RESP | P(INT)_RESP | GENO_PLACEBO | EVENTS_PLACEBO | TOT_AL_PLACEBO | HR_PLACEBO | HR 95% CI lower_PLACEBO | HR 95% CI upper_PLACEBO | P-value_PLACEBO | P_DF2_PLACEBO | GENO_statin | EVENTS_statin | TOTAL_statin | HR_statin | HR 95% CI lower_statin | HR 95% CI upper_statin | P-value_statin | P_DF2_statin |
|---|---|---|---|---|---|---|---|---|---|---|---|---|---|---|---|---|---|---|---|---|---|---|---|---|---|---|
| | | | | | | | Statin response by genotype group | | | | | | | Risk of VT in no statin use group | | | | | | | | Risk of VT in statin use group | | | |
| hCV7543812 | GEN | TT | statin | 40 | 51 | 1.04 | 0.631 | 1.7243 | 0.8698 | 0.00045 | TT | 136 | 195 | 0.71 | 0.5117 | 0.989 | 0.043 | 0.0373 | TT | 40 | 51 | 2.85 | 1.4892 | 5.4472 | 0.0016 | 0.0065 |
| hCV7543812 | GEN | TT | no statin | 136 | 195 | ref | | | | 0.00045 | | | | | | | | | | | | | | | | |
| hCV7543812 | GEN | TC | statin | 85 | 134 | 0.56 | 0.389 | 0.7992 | 0.0015 | 0.00045 | TC | 276 | 283 | 1 | 0.7414 | 1.343 | 0.9893 | 0.0373 | TC | 65 | 134 | 1.76 | 0.9862 | 3.1463 | 0.0557 | 0.0065 |
| hCV7543812 | GEN | TC | no statin | 276 | 283 | ref | | | | 0.00045 | | | | | | | | | | | | | | | | |
| hCV7543812 | GEN | CC | statin | 20 | 72 | 0.28 | 0.156 | 0.5093 | <.0001 | 0.00045 | CC | 126 | 129 | ref | | | | 0.0373 | CC | 20 | 72 | ref | | | | 0.0065 |
| hCV7543812 | GEN | CC | no statin | 126 | 129 | ref | | | | 0.00045 | | | | | | | | | | | | | | | | |
| hCV7543812 | REC | TC+CC | statin | 85 | 206 | 0.46 | 0.337 | 0.6217 | <.0001 | 0.00046 | TT | 136 | 195 | 0.71 | 0.5499 | 0.923 | 0.0103 | | TT | 40 | 51 | 1.9 | 1.1708 | 3.0924 | 0.0094 | |
| hCV7543812 | REC | TC+CC | no statin | 402 | 412 | ref | | | | 0.00046 | | | | | | | | | | | | | | | | |
| hCV2690378 | GEN | GG | statin | 51 | 71 | 0.82 | 0.53 | 1.2743 | 0.3805 | 0.00439 | GG | 187 | 203 | 1.35 | 0.9508 | 1.91 | 0.0936 | 0.133 | GG | 51 | 71 | 1.22 | 0.6713 | 2.2133 | 0.5154 | 0.0078 |
| hCV2690378 | GEN | GG | no statin | 187 | 203 | ref | | | | 0.00439 | | | | | | | | | | | | | | | | |
| hCV2690378 | GEN | GT | statin | 47 | 140 | 0.37 | 0.248 | 0.5445 | <.0001 | 0.00439 | GT | 272 | 287 | 1.39 | 1.0016 | 1.941 | 0.0489 | 0.133 | GT | 47 | 140 | 0.57 | 0.3215 | 1.0235 | 0.0599 | 0.0078 |
| hCV2690378 | GEN | GT | no statin | 272 | 287 | ref | | | | 0.00439 | | | | | | | | | | | | | | | | |
| hCV2690378 | GEN | TT | statin | 27 | 46 | 0.89 | 0.494 | 1.6077 | 0.7017 | 0.00439 | TT | 79 | 117 | ref | | | | 0.133 | TT | 27 | 46 | ref | | | | 0.0078 |
| hCV2690378 | GEN | TT | no statin | 79 | 117 | ref | | | | 0.00439 | | | | | | | | | | | | | | | | |
| hCV7543812 | DOM | TC+TT | statin | 105 | 185 | 0.69 | 0.519 | 0.9287 | 0.014 | 0.0048 | TC+TT | 412 | 478 | 0.88 | 0.666 | 1.165 | 0.3726 | | TC+TT | 105 | 185 | 2.06 | 1.1851 | 3.5833 | 0.0104 | |
| hCV7543812 | DOM | TC+TT | no statin | 412 | 478 | ref | | | | 0.0048 | | | | | | | | | | | | | | | | |
| hCV931685 | GEN | GG | statin | 90 | 205 | 0.48 | 0.356 | 0.6484 | <.0001 | 0.00984 | GG | 413 | 431 | 2.26 | 0.8633 | 5.895 | 0.0969 | 0.0639 | GG | 90 | 205 | 0.3 | 0.0821 | 1.0866 | 0.0667 | 0.104 |
| hCV931685 | GEN | GG | no statin | 413 | 431 | ref | | | | 0.00984 | | | | | | | | | | | | | | | | |
| hCV931685 | GEN | GT | statin | 29 | 48 | 0.86 | 0.492 | 1.5118 | 0.6056 | 0.00984 | GT | 120 | 161 | 1.76 | 0.6621 | 4.696 | 0.2564 | 0.0639 | GT | 29 | 48 | 0.42 | 0.1077 | 1.6061 | 0.2031 | 0.104 |
| hCV931685 | GEN | GT | no statin | 120 | 161 | ref | | | | 0.00984 | | | | | | | | | | | | | | | | |
| hCV931685 | GEN | TT | statin | 6 | 4 | 2.76 | 0.489 | 15.611 | 0.25 | 0.00984 | TT | 6 | 15 | ref | | | | 0.0639 | TT | 6 | 4 | ref | | | | 0.104 |
| hCV931685 | GEN | TT | no statin | 6 | 15 | ref | | | | 0.00984 | | | | | | | | | | | | | | | | |
| hCV11686277 | GEN | CC | statin | 51 | 70 | 0.82 | 0.531 | 1.2762 | 0.3845 | 0.01246 | CC | 190 | 207 | 1.25 | 0.8787 | 1.779 | 0.2145 | 0.357 | CC | 51 | 70 | 1.31 | 0.7207 | 2.3934 | 0.3734 | 0.0098 |
| hCV11686277 | GEN | CC | no statin | 190 | 207 | ref | | | | 0.01246 | | | | | | | | | | | | | | | | |
| hCV11686277 | GEN | CG | statin | 48 | 140 | 0.39 | 0.264 | 0.5766 | <.0001 | 0.01246 | CG | 271 | 292 | 1.27 | 0.9077 | 1.776 | 0.1632 | 0.357 | CG | 48 | 140 | 0.62 | 0.3476 | 1.1112 | 0.1086 | 0.0098 |
| hCV11686277 | GEN | CG | no statin | 271 | 292 | ref | | | | 0.01246 | | | | | | | | | | | | | | | | |
| hCV11686277 | GEN | GG | statin | 26 | 47 | 0.77 | 0.426 | 1.4035 | 0.3976 | 0.01246 | GG | 78 | 108 | ref | | | | 0.357 | GG | 26 | 47 | ref | | | | 0.0098 |
| hCV11686277 | GEN | GG | no statin | 78 | 108 | ref | | | | 0.01246 | | | | | | | | | | | | | | | | |
| hCV29260019 | REC | GA+AA | statin | 81 | 138 | 0.77 | 0.553 | 1.0792 | 0.1302 | 0.01356 | GG | 219 | 229 | 1.14 | 0.8974 | 1.446 | 0.2846 | | GG | 42 | 119 | 0.6 | 0.3805 | 0.9313 | 0.0231 | |
| hCV29260019 | REC | GA+AA | no statin | 319 | 378 | ref | | | | 0.01356 | | | | | | | | | | | | | | | | |
| hDV70820190 | DOM | GA+GG | statin | 116 | 252 | 0.54 | 0.416 | 0.7072 | <.0001 | 0.01407 | GA+GG | 525 | 584 | 1.43 | 0.7288 | 2.82 | 0.2967 | | GA+GG | 116 | 252 | 0.29 | 0.0922 | 0.903 | 0.0327 | |
| hDV70820190 | DOM | GA+GG | no statin | 525 | 584 | ref | | | | 0.01407 | | | | | | | | | | | | | | | | |
| hCV931685 | REC | GT+TT | statin | 35 | 52 | 0.98 | 0.579 | 1.6589 | 0.9396 | 0.01448 | GG | 413 | 431 | 1.33 | 1.0163 | 1.731 | 0.0376 | | GG | 90 | 205 | 0.65 | 0.3932 | 1.0614 | 0.0846 | |
| hCV931685 | REC | GT+TT | no statin | 126 | 176 | ref | | | | 0.01448 | | | | | | | | | | | | | | | | |
| hDV70437895 | DOM | CT+CC | statin | 108 | 237 | 0.52 | 0.397 | 0.6865 | <.0001 | 0.01491 | CT+CC | 505 | 550 | 1.53 | 0.9848 | 2.388 | 0.0585 | | CT+CC | 108 | 237 | 0.53 | 0.2635 | 1.0523 | 0.0694 | |
| hDV70437895 | DOM | CT+CC | no statin | 505 | 550 | ref | | | | 0.01491 | | | | | | | | | | | | | | | | |
| hCV931685 | DOM | GT+GG | statin | 119 | 253 | 0.55 | 0.422 | 0.7148 | <.0001 | 0.01611 | GT+GG | 533 | 592 | 2.12 | 0.8129 | 5.524 | 0.1245 | | GT+GG | 119 | 253 | 0.32 | 0.0884 | 1.1575 | 0.0824 | |
| hCV931685 | DOM | GT+GG | no statin | 533 | 592 | ref | | | | 0.01611 | | | | | | | | | | | | | | | | |
| hCV29245634 | GEN | CC | statin | 104 | 227 | 0.5 | 0.38 | 0.6657 | <.0001 | 0.01659 | CC | 471 | 509 | 0.26 | 0.0288 | 2.338 | 0.2291 | 0.066 | CC | 104 | 227 | 43000 | 0 | 1E+182 | 0.9592 | 0.2804 |
| hCV29245634 | GEN | CC | no statin | 471 | 509 | ref | | | | 0.01659 | | | | | | | | | | | | | | | | |
| hCV29245634 | GEN | CT | statin | 21 | 28 | 1.45 | 0.707 | 2.9636 | 0.3114 | 0.01659 | CT | 64 | 96 | 0.18 | 0.0201 | 1.691 | 0.1347 | 0.066 | CT | 21 | 28 | 71000 | 0 | 2E+182 | 0.9573 | 0.2804 |
| hCV29245634 | GEN | CT | no statin | 64 | 96 | ref | | | | 0.01659 | | | | | | | | | | | | | | | | |
| hCV29245634 | GEN | TT | statin | 0 | 2 | 0 | | 0 | 2E+158 | 0.9403 | 0.01659 | TT | 4 | 1 | ref | | | | 0.066 | TT | 0 | 2 | ref | | | 0.2804 |

EP 2 635 709 B1

EP 2 635 709 B1

| SNP | MODE | GENO_RESP | STATIN_USE | EVENTS_RESP | TOTAL_RESP | HR_RESP | HR 95% CI lower_RESP | HR 95% CI upper_RESP | P-value_RESP | P(INT)_RESP | GENO_PLACEBO | EVENTS_PLACEBO | TOTAL_PLACEBO | HR_PLACEBO | HR 95% CI lower_PLACEBO | HR 95% CI upper_PLACEBO | P-value_PLACEBO | P_DF2_PLACEBO | GENO_statin | EVENTS_statin | TOTAL_statin | HR_statin | HR 95% CI lower_statin | HR 95% CI upper_statin | P-value_statin | P_DF2_statin |
|---|---|---|---|---|---|---|---|---|---|---|---|---|---|---|---|---|---|---|---|---|---|---|---|---|---|---|
| | | | | | | | | Statin response by genotype group | | | | | | | | Risk of VT in no statin use group | | | | | | | Risk of VT in statin use group | | |
| hCV29245634 | GEN | TT | no statin | 4 | 1 | ref | | | | 0.01659 | | | | | | | | | | | | | | | | |
| hDV70437895 | GEN | CC | statin | 65 | 148 | 0.49 | 0.346 | 0.7051 | 0.0001 | 0.01843 | CC | 293 | 285 | 1.71 | 1.086 | 2.705 | 0.0206 | 0.0234 | CC | 65 | 148 | 0.51 | 0.25 | 1.0405 | 0.0642 | 0.1798 |
| hDV70437895 | GEN | CC | no statin | 293 | 285 | ref | | | | 0.01843 | | | | | | | | | | | | | | | | |
| hDV70437895 | GEN | CT | statin | 43 | 89 | 0.56 | 0.364 | 0.8634 | 0.0086 | 0.01843 | CT | 212 | 265 | 1.34 | 0.8434 | 2.128 | 0.2155 | 0.0234 | CT | 43 | 89 | 0.56 | 0.2621 | 1.1868 | 0.1297 | 0.1798 |
| hDV70437895 | GEN | CT | no statin | 212 | 265 | ref | | | | 0.01843 | | | | | | | | | | | | | | | | |
| hDV70437895 | GEN | TT | statin | 17 | 20 | 1.48 | 0.602 | 3.6304 | 0.3932 | 0.01843 | TT | 34 | 57 | ref | | | | 0.0234 | TT | 17 | 20 | ref | | | | 0.1798 |
| hDV70437895 | GEN | TT | no statin | 34 | 57 | ref | | | | 0.01843 | | | | | | | | | | | | | | | | |
| hDV72050312 | REC | GA+AA | statin | 63 | 98 | 0.83 | 0.562 | 1.2327 | 0.3593 | 0.01937 | GG | 337 | 368 | 1.1 | 0.8637 | 1.394 | 0.4473 | | GG | 62 | 159 | 0.61 | 0.3943 | 0.9355 | 0.0237 | |
| hDV72050312 | REC | GA+AA | no statin | 201 | 239 | ref | | | | 0.01937 | | | | | | | | | | | | | | | | |
| hCV29948033 | DOM | TC+TT | statin | 116 | 251 | 0.53 | 0.409 | 0.6979 | <.0001 | 0.02073 | TC+TT | 511 | 572 | 1.16 | 0.6865 | 1.945 | 0.5864 | | TC+TT | 116 | 251 | 0.3 | 0.1035 | 0.8704 | 0.0267 | |
| hCV29948033 | DOM | TC+TT | no statin | 511 | 572 | ref | | | | 0.02073 | | | | | | | | | | | | | | | | |
| hDV70794769 | REC | CT+TT | statin | 63 | 99 | 0.83 | 0.562 | 1.2319 | 0.358 | 0.02149 | CC | 334 | 364 | 1.1 | 0.8686 | 1.4 | 0.422 | | CC | 62 | 158 | 0.62 | 0.4006 | 0.9498 | 0.0282 | |
| hDV70794769 | REC | CT+TT | no statin | 204 | 243 | ref | | | | 0.02149 | | | | | | | | | | | | | | | | |
| hCV12066124 | REC | CT+TT | statin | 95 | 189 | 0.66 | 0.485 | 0.896 | 0.0078 | 0.02348 | CC | 193 | 169 | 1.49 | 1.1552 | 1.911 | 0.002 | | CC | 27 | 68 | 0.77 | 0.4624 | 1.29 | 0.3237 | |
| hCV12066124 | REC | CT+TT | no statin | 341 | 435 | ref | | | | 0.02348 | | | | | | | | | | | | | | | | |
| hCV3054799 | REC | AG+GG | statin | 83 | 132 | 0.7 | 0.503 | 0.9774 | 0.0382 | 0.02372 | AA | 211 | 247 | 0.94 | 0.7447 | 1.198 | 0.6363 | | AA | 41 | 125 | 0.52 | 0.3344 | 0.8198 | 0.0047 | |
| hCV3054799 | REC | AG+GG | no statin | 328 | 360 | ref | | | | 0.02372 | | | | | | | | | | | | | | | | |
| hDV70820190 | GEN | GG | statin | 79 | 178 | 0.49 | 0.354 | 0.6732 | <.0001 | 0.02944 | GG | 367 | 389 | 1.5 | 0.7614 | 2.974 | 0.2398 | 0.2887 | GG | 79 | 178 | 0.28 | 0.088 | 0.8787 | 0.0292 | 0.0908 |
| hDV70820190 | GEN | GG | no statin | 367 | 389 | ref | | | | 0.02944 | | | | | | | | | | | | | | | | |
| hDV70820190 | GEN | GA | statin | 37 | 74 | 0.68 | 0.421 | 1.0862 | 0.1057 | 0.02944 | GA | 158 | 195 | 1.29 | 0.642 | 2.597 | 0.4736 | 0.2887 | GA | 37 | 74 | 0.31 | 0.0957 | 1.0268 | 0.0553 | 0.0908 |
| hDV70820190 | GEN | GA | no statin | 158 | 195 | ref | | | | 0.02944 | | | | | | | | | | | | | | | | |
| hDV70820190 | GEN | AA | statin | 8 | 5 | 2.05 | 0.506 | 8.3068 | 0.3149 | 0.02944 | AA | 14 | 23 | ref | | | | 0.2887 | AA | 8 | 5 | ref | | | | 0.0908 |
| hDV70820190 | GEN | AA | no statin | 14 | 23 | ref | | | | 0.02944 | | | | | | | | | | | | | | | | |
| hCV1396435 | REC | GT+TT | statin | 95 | 172 | 0.68 | 0.501 | 0.9275 | 0.0147 | 0.02966 | GG | 187 | 191 | 1.16 | 0.905 | 1.485 | 0.242 | | GG | 30 | 85 | 0.63 | 0.3882 | 1.0284 | 0.0647 | |
| hCV1396435 | REC | GT+TT | no statin | 351 | 416 | ref | | | | 0.02966 | | | | | | | | | | | | | | | | |
| hDV70437895 | REC | CT+TT | statin | 60 | 109 | 0.68 | 0.461 | 0.9886 | 0.0435 | 0.03119 | CC | 293 | 285 | 1.34 | 1.0607 | 1.691 | 0.0141 | | CC | 65 | 148 | 0.79 | 0.5162 | 1.2238 | 0.297 | |
| hDV70437895 | REC | CT+TT | no statin | 246 | 322 | ref | | | | 0.03119 | | | | | | | | | | | | | | | | |
| hCV11686277 | REC | CG+GG | statin | 74 | 167 | 0.47 | 0.342 | 0.6558 | <.0001 | 0.03337 | CC | 190 | 207 | 1.04 | 0.8177 | 1.333 | 0.7295 | | CC | 51 | 70 | 1.83 | 1.1658 | 2.8751 | 0.0086 | |
| hCV11686277 | REC | CG+GG | no statin | 349 | 400 | ref | | | | 0.03337 | | | | | | | | | | | | | | | | |
| hCV11778561 | DOM | AT+AA | statin | 92 | 210 | 0.49 | 0.369 | 0.6625 | <.0001 | 0.03501 | AT+AA | 458 | 501 | 1.22 | 0.8884 | 1.675 | 0.2194 | | AT+AA | 92 | 210 | 0.61 | 0.3675 | 1.0239 | 0.0615 | |
| hCV11778561 | DOM | AT+AA | no statin | 458 | 501 | ref | | | | 0.03501 | | | | | | | | | | | | | | | | |
| hCV2690378 | REC | GT+TT | statin | 74 | 186 | 0.47 | 0.343 | 0.8562 | <.0001 | 0.04169 | GG | 187 | 203 | 1.05 | 0.8232 | 1.345 | 0.6842 | | GG | 51 | 71 | 1.79 | 1.1434 | 2.8144 | 0.011 | |
| hCV2690378 | REC | GT+TT | no statin | 351 | 404 | ref | | | | 0.04169 | | | | | | | | | | | | | | | | |
| hDV70830411 | REC | TG+GG | statin | 77 | 179 | 0.47 | 0.339 | 0.6482 | <.0001 | 0.04469 | TT | 186 | 231 | 0.85 | 0.6685 | 1.085 | 0.1942 | | TT | 48 | 77 | 1.45 | 0.9242 | 2.2691 | 0.1061 | |
| hDV70830411 | REC | TG+GG | no statin | 353 | 378 | ref | | | | 0.04469 | | | | | | | | | | | | | | | | |
| hCV7422169 | DOM | GA+GG | statin | 118 | 253 | 0.54 | 0.417 | 0.708 | <.0001 | 0.04504 | GA+GG | 515 | 581 | 0.99 | 0.5617 | 1.754 | 0.9792 | | GA+GG | 118 | 253 | 0.26 | 0.0748 | 0.9243 | 0.0373 | |
| hCV7422169 | DOM | GA+GG | no statin | 515 | 581 | ref | | | | 0.04504 | | | | | | | | | | | | | | | | |
| hCV29260019 | GEN | GG | statin | 42 | 119 | 0.36 | 0.234 | 0.5439 | <.0001 | 0.04625 | GG | 219 | 229 | 1.28 | 0.8796 | 1.853 | 0.1987 | 0.416 | GG | 42 | 119 | 0.71 | 0.3538 | 1.4308 | 0.3395 | 0.0605 |
| hCV29260019 | GEN | GG | no statin | 219 | 229 | ref | | | | 0.04625 | | | | | | | | | | | | | | | | |
| hCV29260019 | GEN | GA | statin | 65 | 106 | 0.75 | 0.513 | 1.0947 | 0.1357 | 0.04625 | GA | 254 | 292 | 1.16 | 0.8036 | 1.665 | 0.4335 | 0.416 | GA | 65 | 106 | 1.26 | 0.6363 | 2.4766 | 0.5118 | 0.0605 |
| hCV29260019 | GEN | GA | no statin | 254 | 292 | ref | | | | 0.04625 | | | | | | | | | | | | | | | | |
| hCV29260019 | GEN | AA | statin | 16 | 32 | 0.86 | 0.419 | 1.7833 | 0.6933 | 0.04625 | AA | 65 | 86 | ref | | | | 0.416 | AA | 16 | 32 | ref | | | | 0.0605 |
| hCV29260019 | GEN | AA | no statin | 65 | 86 | ref | | | | 0.04625 | | | | | | | | | | | | | | | | |

| SNP | MODE | GENO_RESP | STATIN USE | EVENTS_RESP | TOTAL_RESP | HR_RESP | HR 95% CI lower_RESP | HR 95% CI upper_RESP | P-value_RESP | P(INT)_RESP | GENO_PLACEBO | EVENTS_PLACEBO | TOTAL_PLACEBO | HR_PLACEBO | HR 95% CI lower_PLACEBO | HR 95% CI upper_PLACEBO | P-value_PLACEBO | P_DF2_PLACEBO | GENO_statin | EVENTS_statin | TOTAL_statin | HR_statin | HR 95% CI lower_statin | HR 95% CI upper_statin | P-value_statin | P_DF2_statin |
|---|---|---|---|---|---|---|---|---|---|---|---|---|---|---|---|---|---|---|---|---|---|---|---|---|---|---|
| hCV29245634 | REC | CT+TT | statin | 21 | 30 | 1.32 | 0.651 | 2.6788 | 0.4409 | 0.04661 | CC | 471 | 509 | 1.34 | 0.9614 | 1.881 | 0.0836 | | CC | 104 | 227 | 0.65 | 0.3571 | 1.1968 | 0.1683 | |
| hCV29245634 | REC | CT+TT | no statin | 68 | 97 | ref | | | | 0.04661 | | | | | | | | | | | | | | | | |
| hCV29948033 | GEN | TT | statin | 66 | 153 | 0.5 | 0.353 | 0.7033 | <.0001 | 0.05902 | TT | 338 | 383 | 1.14 | 0.6725 | 1.931 | 0.6271 | 0.8191 | TT | 66 | 153 | 0.28 | 0.0951 | 0.8275 | 0.0213 | 0.0674 |
| hCV29948033 | GEN | TT | no statin | 338 | 383 | ref | | | | 0.05902 | | | | | | | | | | | | | | | | |
| hCV29948033 | GEN | TC | statin | 50 | 98 | 0.58 | 0.382 | 0.8933 | 0.0131 | 0.05902 | TC | 173 | 189 | 1.19 | 0.6871 | 2.054 | 0.5374 | 0.8191 | TC | 50 | 98 | 0.33 | 0.1102 | 0.9849 | 0.0469 | 0.0674 |
| hCV29948033 | GEN | TC | no statin | 173 | 189 | ref | | | | 0.05902 | | | | | | | | | | | | | | | | |
| hCV29948033 | GEN | CC | statin | 9 | 6 | 2.72 | 0.715 | 10.345 | 0.1423 | 0.05902 | CC | 27 | 34 | ref | | | | 0.8191 | CC | 9 | 6 | ref | | | | 0.0674 |
| hCV29948033 | GEN | CC | no statin | 27 | 34 | ref | | | | 0.05902 | | | | | | | | | | | | | | | | |
| hCV3054799 | GEN | AA | statin | 41 | 125 | 0.42 | 0.273 | 0.6386 | <.0001 | 0.06051 | AA | 211 | 247 | 1.04 | 0.7272 | 1.499 | 0.8157 | 0.6888 | AA | 41 | 125 | 0.72 | 0.3683 | 1.4234 | 0.3491 | 0.0078 |
| hCV3054799 | GEN | AA | no statin | 211 | 247 | ref | | | | 0.06051 | | | | | | | | | | | | | | | | |
| hCV3054799 | GEN | AG | statin | 66 | 95 | 0.74 | 0.509 | 1.0894 | 0.1288 | 0.06051 | AG | 255 | 272 | 1.14 | 0.7991 | 1.626 | 0.4703 | 0.6888 | AG | 66 | 95 | 1.54 | 0.7943 | 2.9729 | 0.202 | 0.0078 |
| hCV3054799 | GEN | AG | no statin | 255 | 272 | ref | | | | 0.06051 | | | | | | | | | | | | | | | | |
| hCV3054799 | GEN | GG | statin | 17 | 37 | 0.57 | 0.284 | 1.1433 | 0.1135 | 0.06051 | GG | 73 | 88 | ref | | | | 0.6888 | GG | 17 | 37 | ref | | | | 0.0078 |
| hCV3054799 | GEN | GG | no statin | 73 | 88 | ref | | | | 0.06051 | | | | | | | | | | | | | | | | |
| hCV31671070 | DOM | AG+AA | statin | 125 | 251 | 0.59 | 0.451 | 0.7603 | <.0001 | 0.07368 | AG+AA | 526 | 591 | 1.43 | 0.6402 | 3.173 | 0.3655 | | AG+AA | 125 | 251 | 2E+06 | 0 | | 0.9868 | |
| hCV31671070 | DOM | AG+AA | no statin | 528 | 591 | ref | | | | 0.07368 | | | | | | | | | | | | | | | | |
| hCV12066124 | GEN | CC | statin | 27 | 68 | 0.38 | 0.225 | 0.625 | 0.0002 | 0.07542 | CC | 193 | 169 | 1.87 | 1.3346 | 2.634 | 0.0003 | 0.0012 | CC | 27 | 68 | 1.01 | 0.5219 | 1.9537 | 0.977 | 0.2627 |
| hCV12066124 | GEN | CC | no statin | 193 | 169 | ref | | | | 0.07542 | | | | | | | | | | | | | | | | |
| hCV12066124 | GEN | CT | statin | 72 | 129 | 0.68 | 0.475 | 0.9711 | 0.0339 | 0.07542 | CT | 254 | 296 | 1.38 | 1.0085 | 1.901 | 0.0442 | 0.0012 | CT | 72 | 129 | 1.45 | 0.8244 | 2.5485 | 0.1973 | 0.2627 |
| hCV12066124 | GEN | CT | no statin | 254 | 296 | ref | | | | 0.07542 | | | | | | | | | | | | | | | | |
| hCV12066124 | GEN | TT | statin | 23 | 60 | 0.6 | 0.328 | 1.1042 | 0.101 | 0.07542 | TT | 87 | 139 | ref | | | | 0.0012 | TT | 23 | 60 | ref | | | | 0.2627 |
| hCV12066124 | GEN | TT | no statin | 87 | 139 | ref | | | | 0.07542 | | | | | | | | | | | | | | | | |
| hCV1772768 | REC | GA+AA | statin | 84 | 146 | 0.64 | 0.463 | 0.8972 | 0.0093 | 0.0798 | GG | 230 | 256 | 1.03 | 0.8109 | 1.298 | 0.8305 | | GG | 41 | 110 | 0.65 | 0.4134 | 1.0147 | 0.0579 | |
| hCV1772768 | REC | GA+AA | no statin | 308 | 351 | ref | | | | 0.0798 | | | | | | | | | | | | | | | | |
| hDV72050312 | GEN | GG | statin | 62 | 159 | 0.43 | 0.305 | 0.6143 | <.0001 | 0.08047 | GG | 337 | 368 | 0.79 | 0.4485 | 1.382 | 0.405 | 0.3308 | GG | 62 | 159 | 0.45 | 0.2043 | 1.0116 | 0.0534 | 0.054 |
| hDV72050312 | GEN | GG | no statin | 337 | 368 | ref | | | | 0.08047 | | | | | | | | | | | | | | | | |
| hDV72050312 | GEN | GA | statin | 50 | 83 | 0.81 | 0.528 | 1.2483 | 0.3424 | 0.08047 | GA | 172 | 215 | 0.69 | 0.3843 | 1.223 | 0.2011 | 0.3308 | GA | 50 | 83 | 0.7 | 0.3087 | 1.6002 | 0.4009 | 0.054 |
| hDV72050312 | GEN | GA | no statin | 172 | 215 | ref | | | | 0.08047 | | | | | | | | | | | | | | | | |
| hDV72050312 | GEN | AA | statin | 13 | 15 | 0.79 | 0.285 | 2.1974 | 0.6528 | 0.08047 | AA | 29 | 24 | ref | | | | 0.3308 | AA | 13 | 15 | ref | | | | 0.054 |
| hDV72050312 | GEN | AA | no statin | 29 | 24 | ref | | | | 0.08047 | | | | | | | | | | | | | | | | |
| hCV9540478 | REC | TC+CC | statin | 38 | 94 | 0.44 | 0.282 | 0.6919 | 0.0004 | 0.08096 | TT | 335 | 402 | 0.84 | 0.6583 | 1.072 | 0.1604 | | TT | 87 | 163 | 1.33 | 0.8361 | 2.0967 | 0.2292 | |
| hCV9540478 | REC | TC+CC | no statin | 201 | 204 | ref | | | | 0.08096 | | | | | | | | | | | | | | | | |
| hCV2690378 | DOM | GT+GG | statin | 98 | 211 | 0.52 | 0.389 | 0.6943 | <.0001 | 0.08114 | GT+GG | 459 | 490 | 1.37 | 1.0052 | 1.881 | 0.0463 | | GT+GG | 98 | 211 | 0.79 | 0.4646 | 1.3477 | 0.3888 | |
| hCV2690378 | DOM | GT+GG | no statin | 459 | 490 | ref | | | | 0.08114 | | | | | | | | | | | | | | | | |
| hCV29245634 | DOM | CT+CC | statin | 125 | 255 | 0.58 | 0.446 | 0.7496 | <.0001 | 0.08203 | CT+CC | 535 | 605 | 0.25 | 0.0274 | 2.218 | 0.2115 | | CT+CC | 125 | 255 | 9E+05 | 0 | | 0.9883 | |
| hCV29245634 | DOM | CT+CC | no statin | 535 | 605 | ref | | | | 0.08203 | | | | | | | | | | | | | | | | |
| hCV16233239 | REC | AG+GG | statin | 48 | 86 | 0.8 | 0.522 | 1.2301 | 0.3111 | 0.0829 | AA | 342 | 356 | 1.25 | 0.9806 | 1.583 | 0.0719 | | AA | 77 | 171 | 0.8 | 0.5152 | 1.2554 | 0.3376 | |
| hCV16233239 | REC | AG+GG | no statin | 196 | 251 | ref | | | | 0.0829 | | | | | | | | | | | | | | | | |
| hCV3286482 | DOM | TC+TT | statin | 116 | 245 | 0.55 | 0.419 | 0.7162 | <.0001 | 0.08603 | TC+TT | 517 | 572 | 1.49 | 0.8535 | 2.591 | 0.1613 | | TC+TT | 116 | 245 | 0.59 | 0.2377 | 1.4739 | 0.2589 | |
| hCV3286482 | DOM | TC+TT | no statin | 517 | 572 | ref | | | | 0.08603 | | | | | | | | | | | | | | | | |
| hDV70794769 | GEN | CC | statin | 62 | 158 | 0.43 | 0.306 | 0.6163 | <.0001 | 0.0899 | CC | 334 | 364 | 0.73 | 0.4152 | 1.286 | 0.2765 | 0.2084 | CC | 62 | 158 | 0.46 | 0.2056 | 1.0179 | 0.0553 | 0.0614 |
| hDV70794769 | GEN | CC | no statin | 334 | 364 | ref | | | | 0.0899 | | | | | | | | | | | | | | | | |
| hDV70794769 | GEN | CT | statin | 50 | 84 | 0.82 | 0.534 | 1.2614 | 0.3676 | 0.0899 | CT | 174 | 220 | 0.63 | 0.3509 | 1.12 | 0.1148 | 0.2084 | CT | 50 | 84 | 0.7 | 0.3054 | 1.5824 | 0.3862 | 0.0614 |

| | | | Statin response by genotype group | | | | | | | | Risk of VT in no statin use group | | | | | | | | Risk of VT in statin use group | | | | | | |
|---|---|---|---|---|---|---|---|---|---|---|---|---|---|---|---|---|---|---|---|---|---|---|---|---|---|---|
| SNP | MOD E | GENO_ RESP | STATIN _USE | EVE NTS _RE SP | TOT AL_ RES P | HR_ RES P | HR 95% CI lower_ RESP | HR 95% CI upper_ RESP | P- value_R ESP | P(INT)_ RESP | GENO_ PLACE BO | EVE NTS_ PL ACE BO | TOT AL_ PLA CEB O | HR_ PLA CEB O | HR 95% CI lower_ PLACE BO | HR 95% CI upper_ PLAC EBO | P- value_ PLACE BO | P_DF2 _PLAC EBO | GENO_ statin | EVE NTS_ stat in | TOT AL_ stati n | HR_st atin | HR 95% CI lower_ statin | HR 95% CI upper_ statin | P- value_s tatin | P_DF2 _statin |
| hDV70794769 | GEN | CT | no statin | 174 | 220 | ref | | | | 0.0899 | | | | | | | | | | | | | | | | | |
| hDV70794769 | GEN | TT | statin | 13 | 15 | 0.69 | 0.246 | 1.9396 | 0.4823 | 0.0899 | TT | 30 | 23 | ref | | | | 0.2084 | TT | 13 | 15 | ref | | | | 0.0614 |
| hDV70794769 | GEN | TT | no statin | 30 | 23 | ref | | | | 0.0899 | | | | | | | | | | | | | | | | | |
| hDV77026147 | DOM | CT+CC | statin | 124 | 257 | 0.56 | 0.435 | 0.7324 | <.0001 | 0.09216 | CT+CC | 535 | 602 | 2.24 | 0.4312 | 11.62 | 0.3376 | | CT+CC | 124 | 257 | 0 | 0 | | 0.9867 | |
| hDV77026147 | DOM | CT+CC | no statin | 535 | 602 | ref | | | | 0.09216 | | | | | | | | | | | | | | | | | |
| hCV1396435 | GEN | GG | statin | 30 | 85 | 0.38 | 0.235 | 0.627 | 0.0001 | 0.09466 | GG | 187 | 191 | 1.17 | 0.8309 | 1.648 | 0.3681 | 0.5028 | GG | 30 | 85 | 0.66 | 0.3299 | 1.3053 | 0.2299 | 0.1794 |
| hCV1396435 | GEN | GG | no statin | 187 | 191 | ref | | | | 0.09466 | | | | | | | | | | | | | | | | | |
| hCV1396435 | GEN | GT | statin | 75 | 134 | 0.67 | 0.473 | 0.9506 | 0.0248 | 0.09466 | GT | 258 | 305 | 1.01 | 0.734 | 1.398 | 0.9373 | 0.5028 | GT | 75 | 134 | 1.05 | 0.567 | 1.942 | 0.8783 | 0.1794 |
| hCV1396435 | GEN | GT | no statin | 258 | 305 | ref | | | | 0.09466 | | | | | | | | | | | | | | | | | |
| hCV1396435 | GEN | TT | statin | 20 | 39 | 0.75 | 0.386 | 1.4425 | 0.384 | 0.09466 | TT | 93 | 111 | ref | | | | 0.5028 | TT | 20 | 38 | ref | | | | 0.1794 |
| hCV1396435 | GEN | TT | no statin | 93 | 111 | ref | | | | 0.09466 | | | | | | | | | | | | | | | | | |
| hDV70820190 | REC | GA+AA | statin | 45 | 79 | 0.77 | 0.491 | 1.1912 | 0.2358 | 0.09992 | GG | 367 | 389 | 1.19 | 0.9331 | 1.527 | 0.1589 | | GG | 79 | 178 | 0.78 | 0.4957 | 1.2255 | 0.2805 | |
| hDV70820190 | REC | GA+AA | no statin | 172 | 218 | ref | | | | 0.09992 | | | | | | | | | | | | | | | | | |

Above analysis adjusted for sex and age.

| SNP rs # | Gene | MODE | GENO TYPE | Strata | Odds Ratio | OR 95% CI lower | OR 95% CI upper | EFFECT LABEL | Variable | ProbChiSq | P_DF2 | HW(control)pExact | GENO_ALL | EVENTS_ALL | TOTAL_ALL | HR_ALL | HR 95% CI lower_ALL | HR 95% CI upper_ALL | P-value_ALL | P_DF2_ALL | Ref geno | EVENTS_ALL | TOTAL_ALL |
|---|---|---|---|---|---|---|---|---|---|---|---|---|---|---|---|---|---|---|---|---|---|---|---|
| | | | | | | | | **Primary VT** | | | | | | | | **Recurrent VT** | | | | | | | |
| rs3820059 | C1orf114 | GEN | AG | All | 1.127 | 1.027 | 1.237 | GEN HET | GEN | 0.0119 | 0.00364 | 0.0353 | AG | 248 | 1633 | 1.11 | 0.919 | 1.335 | 0.2836 | 0.0095 | GG | 199 | 1459 |
| rs3820059 | C1orf114 | GEN | AA | All | 1.228 | 1.072 | 1.408 | GEN HOM | GEN | 0.0031 | 0.00364 | 0.0353 | AA | 101 | 508 | 1.45 | 1.141 | 1.843 | 0.0024 | 0.0095 | GG | 199 | 1459 |
| rs3820059 | C1orf114 | ADD | A | All | 1.113 | 1.045 | 1.186 | ADD | ADD | 0.0009 | . | 0.0353 | | | | | | | | 0.0095 | GG | 199 | 1459 |
| rs3820059 | C1orf114 | DOM | AG+AA | All | 1.149 | 1.053 | 1.255 | DOM | DOM | 0.0019 | . | 0.0353 | AG+AA | 349 | 2141 | 1.19 | 0.998 | 1.415 | 0.0521 | 0.0095 | GG | 199 | 1459 |
| rs3820059 | C1orf114 | REC | AA | All | 1.155 | 1.017 | 1.313 | REC | REC | 0.0264 | . | 0.0353 | AA | 101 | 508 | 1.45 | 1.141 | 1.843 | 0.0024 | 0.0095 | GG | 199 | 1459 |
| rs6025 | F5 | GEN | AG | All | 3.587 | 3.047 | 4.176 | GEN HET | GEN | <.0001 | 0 | 0.0475 | AG | 127 | 580 | 1.53 | 1.258 | 1.871 | <.0001 | <.0001 | GG | 429 | 3072 |
| rs6025 | F5 | GEN | AA | All | 5.412 | 2.353 | 12.446 | GEN HOM | GEN | <.0001 | 0 | 0.0475 | AA | 7 | 27 | 1.73 | 0.817 | 3.647 | 0.1522 | <.0001 | GG | 429 | 3072 |
| rs6025 | F5 | ADD | A | All | 3.423 | 2.944 | 3.98 | ADD | ADD | <.0001 | . | 0.0475 | | | | | | | | <.0001 | GG | 429 | 3072 |
| rs6025 | F5 | DOM | AG+AA | All | 3.62 | 3.1 | 4.228 | DOM | DOM | <.0001 | . | 0.0475 | AG+AA | 134 | 607 | 1.54 | 1.27 | 1.874 | <.0001 | <.0001 | GG | 429 | 3072 |
| rs6025 | F5 | REC | AA | All | 4.768 | 2.074 | 10.963 | REC | REC | 0.0002 | . | 0.0475 | AA | 7 | 27 | 1.73 | 0.817 | 3.647 | 0.1522 | <.0001 | GG | 429 | 3072 |
| rs4262503 | LOC730144/LOC10050 5872 | GEN | CT | All | 0.817 | 0.718 | 0.928 | GEN HET | GEN | 0.0019 | 0.00294 | 0.258 | CT | 73 | 437 | 1.14 | 0.892 | 1.462 | 0.2913 | 0.0072 | TT | 462 | 3106 |
| rs4262503 | LOC730144/LOC10050 5872 | GEN | CC | All | 1.488 | 0.834 | 2.582 | GEN HOM | GEN | 0.1832 | 0.00294 | 0.258 | CC | 8 | 27 | 2.93 | 1.454 | 5.891 | 0.0026 | 0.0072 | TT | 462 | 3106 |
| rs4262503 | LOC730144/LOC10050 5872 | ADD | C | All | 0.87 | 0.774 | 0.979 | ADD | ADD | 0.0209 | . | 0.258 | | | | | | | | 0.0072 | TT | 462 | 3106 |
| rs4262503 | LOC730144/LOC10050 5872 | DOM | CT+CC | All | 0.837 | 0.739 | 0.949 | DOM | DOM | 0.0055 | . | 0.258 | CT+CC | 81 | 464 | 1.22 | 0.959 | 1.539 | 0.1059 | 0.0072 | TT | 462 | 3106 |
| rs4262503 | LOC730144/LOC10050 5872 | REC | CC | All | 1.508 | 0.858 | 2.653 | REC | REC | 0.1537 | . | 0.258 | CC | 8 | 27 | 2.93 | 1.454 | 5.891 | 0.0026 | 0.0072 | TT | 462 | 3106 |
| rs627530 | STK11IP/OBSL1 | GEN | CT | All | 1.094 | 0.924 | 1.295 | GEN HET | GEN | 0.2963 | 0.00378 | 3.57E-11 | CT | 44 | 260 | 1.21 | 0.891 | 1.65 | 0.2195 | 0.0259 | TT | 510 | 3372 |
| rs627530 | STK11IP/OBSL1 | GEN | CC | All | 2.998 | 1.527 | 5.885 | GEN HOM | GEN | 0.0014 | 0.00378 | 3.57E-11 | CC | 8 | 26 | 2.4 | 1.192 | 4.831 | 0.0143 | 0.0259 | TT | 510 | 3372 |
| rs627530 | STK11IP/OBSL1 | ADD | C | All | 1.207 | 1.04 | 1.401 | ADD | ADD | 0.0133 | . | 3.57E-11 | | | | | | | | 0.0259 | TT | 510 | 3372 |
| rs627530 | STK11IP/OBSL1 | DOM | CT+CC | All | 1.165 | 0.99 | 1.372 | DOM | DOM | 0.0667 | . | 3.57E-11 | CT+CC | 52 | 286 | 1.31 | 0.987 | 1.747 | 0.0615 | 0.0259 | TT | 510 | 3372 |
| rs627530 | STK11IP/OBSL1 | REC | CC | All | 2.979 | 1.518 | 5.847 | REC | REC | 0.0015 | . | 3.57E-11 | CC | 8 | 26 | 2.4 | 1.192 | 4.831 | 0.0143 | 0.0259 | TT | 510 | 3372 |
| rs1800788 | | GEN | TC | All | 1.231 | 1.121 | 1.351 | GEN HET | GEN | <.0001 | 0.00001 | 0.0217 | TC | 199 | 1284 | 1.09 | 0.911 | 1.306 | 0.3433 | 0.009 | CC | 293 | 2076 |
| rs1800788 | | GEN | TT | All | 1.307 | 1.076 | 1.589 | GEN HOM | GEN | 0.007 | 0.00001 | 0.0217 | TT | 48 | 207 | 1.61 | 1.187 | 2.186 | 0.0022 | 0.009 | CC | 293 | 2076 |

| | | | | | Primary VT | | | | | | | | Recurrent VT | | | | | | | | | | |
|---|---|---|---|---|---|---|---|---|---|---|---|---|---|---|---|---|---|---|---|---|---|---|---|
| SNP rs # | Gene | MODE | GENO TYPE | Strata | Odds Ratio | OR 95% CI lower | OR 95% CI upper | EFFECT LABEL | Variable | ProbChiSq | P_DF2 | HW(control)pExact | GENO_AL L | EVENTS_ ALL | TOTAL_ ALL | HR_AL L | HR 95% CI lower_A LL | HR 95% CI upper_ALL | P-value_AL L | P_DF2_A LL | Ref geno | EVENTS_A LL | TOTAL ALL |
| rs1800788 | | ADD | T | All | 1.189 | 1.105 | 1.278 | ADD | ADD | <.0001 | . | 0.0217 | | | | | | | | 0.009 | CC | 293 | 2076 |
| rs1800788 | | DOM | TC+TT | All | 1.241 | 1.135 | 1.356 | DOM | DOM | <.0001 | . | 0.0217 | TC+T T | 247 | 1491 | 1.16 | 0.983 | 1.379 | 0.0788 | 0.009 | CC | 293 | 2076 |
| rs1800788 | | REC | TT | All | 1.214 | 1.002 | 1.471 | REC | REC | 0.048 | . | 0.0217 | TT | 48 | 207 | 1.61 | 1.187 | 2.186 | 0.0022 | 0.009 | CC | 293 | 2076 |
| rs2066865 | FGG | GEN | AG | All | 1.327 | 1.21 | 1.456 | GEN HET | GEN | <.0001 | 0 | 0.97 | AG | 229 | 1528 | 1.05 | 0.872 | 1.263 | 0.6081 | 0.0027 | GG | 221 | 1585 |
| rs2066865 | FGG | GEN | AA | All | 1.939 | 1.655 | 2.271 | GEN HOM | GEN | <.0001 | 0 | 0.97 | AA | 85 | 406 | 1.53 | 1.194 | 1.971 | 0.0008 | 0.0027 | GG | 221 | 1585 |
| rs2066865 | FGG | ADD | A | All | 1.366 | 1.277 | 1.462 | ADD | ADD | <.0001 | . | 0.97 | | | | | | | | 0.0027 | GG | 221 | 1585 |
| rs2066865 | FGG | DOM | AG+A A | All | 1.421 | 1.302 | 1.551 | DOM | DOM | <.0001 | . | 0.97 | AG+A A | 314 | 1934 | 1.15 | 0.966 | 1.363 | 0.1174 | 0.0027 | GG | 221 | 1585 |
| rs2066865 | FGG | REC | AA | All | 1.704 | 1.464 | 1.985 | REC | REC | <.0001 | . | 0.97 | AA | 85 | 406 | 1.53 | 1.194 | 1.971 | 0.0008 | 0.0027 | GG | 221 | 1585 |
| rs2066854 | FGG | GEN | AT | All | 1.314 | 1.199 | 1.441 | GEN HET | GEN | <.0001 | 0 | 0.849 | AT | 228 | 1537 | 1.03 | 0.855 | 1.236 | 0.7678 | 0.002 | TT | 225 | 1607 |
| rs2066854 | FGG | GEN | AA | All | 1.927 | 1.647 | 2.254 | GEN HOM | GEN | <.0001 | 0 | 0.849 | AA | 87 | 415 | 1.53 | 1.196 | 1.964 | 0.0007 | 0.002 | TT | 225 | 1607 |
| rs2066854 | FGG | ADD | A | All | 1.359 | 1.271 | 1.453 | ADD | ADD | <.0001 | . | 0.849 | | | | | | | | 0.002 | TT | 225 | 1607 |
| rs2066854 | FGG | DOM | AT+A A | All | 1.41 | 1.292 | 1.538 | DOM | DOM | <.0001 | . | 0.849 | AT+A A | 315 | 1952 | 1.13 | 0.953 | 1.342 | 0.1595 | 0.002 | TT | 225 | 1607 |
| rs2066854 | FGG | REC | AA | All | 1.702 | 1.463 | 1.979 | REC | REC | <.0001 | . | 0.849 | AA | 87 | 415 | 1.53 | 1.196 | 1.964 | 0.0007 | 0.002 | TT | 225 | 1607 |
| rs3756008 | | GEN | TA | All | 1.349 | 1.222 | 1.489 | GEN HET | GEN | <.0001 | 0 | 0.644 | TA | 257 | 1751 | 0.95 | 0.778 | 1.165 | 0.635 | 0.0316 | AA | 149 | 1042 |
| rs3756008 | | GEN | TT | All | 1.721 | 1.517 | 1.952 | GEN HOM | GEN | <.0001 | 0 | 0.644 | TT | 137 | 774 | 1.25 | 0.993 | 1.58 | 0.0578 | 0.0316 | AA | 149 | 1042 |
| rs3756008 | | ADD | T | All | 1.317 | 1.238 | 1.401 | ADD | ADD | <.0001 | . | 0.644 | | | | | | | | 0.0316 | AA | 149 | 1042 |
| rs3756008 | | DOM | TA+TT | All | 1.444 | 1.316 | 1.585 | DOM | DOM | <.0001 | . | 0.644 | TA+T T | 394 | 2525 | 1.04 | 0.86 | 1.254 | 0.6945 | 0.0316 | AA | 149 | 1042 |
| rs3756008 | | REC | TT | All | 1.44 | 1.288 | 1.609 | REC | REC | <.0001 | . | 0.644 | TT | 137 | 774 | 1.25 | 0.993 | 1.58 | 0.0578 | 0.0316 | AA | 149 | 1042 |
| rs925451 | F11 | GEN | AG | All | 1.36 | 1.233 | 1.5 | GEN HET | GEN | <.0001 | 0 | 0.0947 | AG | 259 | 1719 | 1.02 | 0.838 | 1.252 | 0.815 | 0.0283 | GG | 151 | 1096 |
| rs925451 | F11 | GEN | AA | All | 1.747 | 1.537 | 1.985 | GEN HOM | GEN | <.0001 | 0 | 0.0947 | AA | 129 | 725 | 1.33 | 1.05 | 1.681 | 0.0179 | 0.0283 | GG | 151 | 1096 |
| rs925451 | F11 | ADD | A | All | 1.328 | 1.248 | 1.413 | ADD | ADD | <.0001 | . | 0.0947 | | | | | | | | 0.0283 | GG | 151 | 1096 |
| rs925451 | F11 | DOM | AG+A A | All | 1.455 | 1.327 | 1.596 | DOM | DOM | <.0001 | . | 0.0947 | AG+A A | 388 | 2444 | 1.11 | 0.918 | 1.338 | 0.2834 | 0.0283 | GG | 151 | 1096 |
| rs925451 | F11 | REC | AA | All | 1.462 | 1.304 | 1.64 | REC | REC | <.0001 | . | 0.0947 | AA | 129 | 725 | 1.33 | 1.05 | 1.681 | 0.0179 | 0.0283 | GG | 151 | 1096 |
| rs3822057 | F11 | GEN | AC | All | 0.785 | 0.707 | 0.871 | GEN HET | GEN | <.0001 | 0 | 0.514 | AC | 269 | 1740 | 0.84 | 0.694 | 1.011 | 0.0646 | 0.0042 | CC | 184 | 1072 |
| rs3822057 | F11 | GEN | AA | All | 0.6 | 0.53 | 0.679 | GEN HOM | GEN | <.0001 | 0 | 0.514 | AA | 86 | 734 | 0.65 | 0.505 | 0.842 | 0.0011 | 0.0042 | CC | 184 | 1072 |
| rs3822057 | F11 | ADD | A | All | 0.775 | 0.729 | 0.824 | ADD | ADD | <.0001 | . | 0.514 | | | | | | | | 0.0042 | CC | 184 | 1072 |
| rs3822057 | F11 | DOM | AC+A A | All | 0.72 | 0.652 | 0.794 | DOM | DOM | <.0001 | . | 0.514 | AC+A A | 355 | 2474 | 0.78 | 0.656 | 0.937 | 0.0073 | 0.0042 | CC | 184 | 1072 |
| rs3822057 | F11 | REC | AA | All | 0.703 | 0.633 | 0.779 | REC | REC | <.0001 | . | 0.514 | AA | 86 | 734 | 0.65 | 0.505 | 0.842 | 0.0011 | 0.0042 | CC | 184 | 1072 |

EP 2 635 709 B1

| SNP rs # | Gene | MODE | GENO TYPE | Strata | Odds Ratio | OR 95% CI lower | OR 95% CI upper | EFFECT LABEL | Variable | ProbChiSq | P_DF2 | HW(control)pExact | GENO_ALL | EVENTS_ALL | TOTAL_ALL | HR_AL | HR 95% CI lower_ALL | HR 95% CI upper_ALL | P-value_AL | P_DF2_ALL | Ref geno | EVENTS_ALL | TOTAL_ALL |
|---|---|---|---|---|---|---|---|---|---|---|---|---|---|---|---|---|---|---|---|---|---|---|---|
| | | | | | Primary VT | | | | | | | | Recurrent VT | | | | | | | | | | |
| rs2036914 | F11 | GEN | TC | All | 0.755 | 0.683 | 0.835 | GEN HET | GEN | <.0001 | 0 | 0.476 | TC | 262 | 1684 | 0.9 | 0.747 | 1.079 | 0.252 | 0.0359 | CC | 201 | 1238 |
| rs2036914 | F11 | GEN | TT | All | 0.586 | 0.517 | 0.664 | GEN HOM | GEN | <.0001 | 0 | 0.476 | TT | 77 | 631 | 0.71 | 0.544 | 0.921 | 0.01 | 0.0359 | CC | 201 | 1238 |
| rs2036914 | F11 | ADD | T | All | 0.764 | 0.719 | 0.813 | ADD | ADD | <.0001 | . | 0.476 | | | | | | | | 0.0359 | CC | 201 | 1238 |
| rs2036914 | F11 | DOM | TC+TT | All | 0.701 | 0.638 | 0.77 | DOM | DOM | <.0001 | . | 0.476 | TC+T | 339 | 2315 | 0.85 | 0.711 | 1.008 | 0.0611 | 0.0359 | CC | 201 | 1238 |
| rs2036914 | F11 | REC | TT | All | 0.696 | 0.624 | 0.776 | REC | REC | <.0001 | . | 0.476 | TT | 77 | 631 | 0.71 | 0.544 | 0.921 | 0.01 | 0.0359 | CC | 201 | 1238 |
| rs3756011 | F11 | GEN | AC | All | 1.347 | 1.218 | 1.489 | GEN HET | GEN | <.0001 | 0 | 0.391 | AC | 258 | 1730 | 1.01 | 0.823 | 1.247 | 0.9046 | 0.0344 | CC | 136 | 994 |
| rs3756011 | F11 | GEN | AA | All | 1.775 | 1.566 | 2.011 | GEN HOM | GEN | <.0001 | 0 | 0.391 | AA | 147 | 827 | 1.3 | 1.026 | 1.637 | 0.0295 | 0.0344 | CC | 136 | 994 |
| rs3756011 | F11 | ADD | A | All | 1.334 | 1.254 | 1.419 | ADD | ADD | <.0001 | . | 0.391 | | | | | | | | 0.0344 | CC | 136 | 994 |
| rs3756011 | F11 | DOM | AC+AA | All | 1.459 | 1.328 | 1.604 | DOM | DOM | <.0001 | . | 0.391 | AC+A A | 405 | 2557 | 1.1 | 0.906 | 1.336 | 0.3371 | 0.0344 | CC | 136 | 994 |
| rs3756011 | F11 | REC | AA | All | 1.482 | 1.329 | 1.653 | REC | REC | <.0001 | . | 0.391 | AA | 147 | 827 | 1.3 | 1.026 | 1.637 | 0.0295 | 0.0344 | CC | 136 | 994 |
| rs2289252 | F11 | GEN | TC | All | 1.381 | 1.249 | 1.527 | GEN HET | GEN | <.0001 | 0 | 0.817 | TC | 257 | 1739 | 1 | 0.807 | 1.226 | 0.9634 | 0.032 | CC | 134 | 974 |
| rs2289252 | F11 | GEN | TT | All | 1.807 | 1.593 | 2.049 | GEN HOM | GEN | <.0001 | 0 | 0.817 | TT | 144 | 814 | 1.29 | 1.017 | 1.63 | 0.0354 | 0.032 | CC | 134 | 974 |
| rs2289252 | F11 | ADD | T | All | 1.348 | 1.267 | 1.435 | ADD | ADD | <.0001 | . | 0.817 | | | | | | | | 0.032 | CC | 134 | 974 |
| rs2289252 | F11 | DOM | TC+TT | All | 1.492 | 1.357 | 1.64 | DOM | DOM | <.0001 | . | 0.817 | TC+T | 401 | 2553 | 1.08 | 0.891 | 1.318 | 0.4225 | 0.032 | CC | 134 | 974 |
| rs2289252 | F11 | REC | TT | All | 1.485 | 1.331 | 1.657 | REC | REC | <.0001 | . | 0.817 | TT | 144 | 814 | 1.29 | 1.017 | 1.63 | 0.0354 | 0.032 | CC | 134 | 974 |
| rs2281390 | LOC642074/LOC642043 | GEN | TG | All | 1.132 | 1.028 | 1.245 | GEN HET | GEN | 0.0113 | 0.02735 | 0.0129 | TG | 154 | 1100 | 0.89 | 0.742 | 1.079 | 0.2445 | 0.0318 | GG | 383 | 2448 |
| rs2281390 | LOC642074/LOC642043 | GEN | TT | All | 0.931 | 0.729 | 1.187 | GEN HOM | GEN | 0.5621 | 0.02735 | 0.0129 | TT | 27 | 112 | 1.54 | 1.043 | 2.278 | 0.0298 | 0.0318 | GG | 383 | 2448 |
| rs2281390 | LOC642074/LOC642043 | ADD | T | All | 1.067 | 0.986 | 1.155 | ADD | ADD | 0.1086 | . | 0.0129 | | | | | | | | 0.0318 | GG | 383 | 2448 |
| rs2281390 | LOC642074/LOC642043 | DOM | TG+TT | All | 1.109 | 1.011 | 1.216 | DOM | DOM | 0.0277 | . | 0.0129 | TG+T | 181 | 1212 | 0.95 | 0.8 | 1.139 | 0.6074 | 0.0318 | GG | 383 | 2448 |
| rs2281390 | LOC642074/LOC642043 | REC | TT | All | 0.898 | 0.705 | 1.143 | REC | REC | 0.382 | . | 0.0129 | TT | 27 | 112 | 1.54 | 1.043 | 2.278 | 0.0298 | 0.0318 | GG | 383 | 2448 |
| rs2274736 | PTPN21 | GEN | GA | All | 1.098 | 1 | 1.204 | GEN HET | GEN | 0.0489 | 0.02063 | 0.455 | GA | 245 | 1597 | 1.13 | 0.94 | 1.361 | 0.1919 | 0.0308 | AA | 208 | 1498 |
| rs2274736 | PTPN21 | GEN | GG | All | 1.207 | 1.041 | 1.399 | GEN HOM | GEN | 0.0126 | 0.02063 | 0.455 | GG | 77 | 402 | 1.42 | 1.092 | 1.842 | 0.0088 | 0.0308 | AA | 208 | 1498 |
| rs2274736 | PTPN21 | ADD | G | All | 1.098 | 1.028 | 1.173 | ADD | ADD | 0.0053 | . | 0.455 | | | | | | | | 0.0308 | AA | 208 | 1498 |

EP 2 635 709 B1

642

| | | Primary VT | | | | | | | | | | | Recurrent VT | | | | | | | | | | |
|---|---|---|---|---|---|---|---|---|---|---|---|---|---|---|---|---|---|---|---|---|---|---|---|
| SNP rs # | Gene | MODE | GENO TYPE | Strata | Odds Ratio | OR 95% CI lower | OR 95% CI upper | EFFECT LABEL | Variable | ProbChiSq | P_DF2 | HW(control)pExact | GENO_ALL | EVENTS_ALL | TOTAL_ALL | HR_ALL | HR 95% CI lower_ALL | HR 95% CI upper_ALL | P-value_AL | P_DF2_ALL | Ref geno | EVENTS_ALL | TOTAL_ALL |
| rs2274736 | PTPN21 | DOM | GA+G/G | All | 1.118 | 1.024 | 1.221 | DOM | DOM | 0.013 | . | 0.455 | GA+G/G | 322 | 1999 | 1.19 | 0.998 | 1.415 | 0.0522 | 0.0308 | AA | 208 | 1498 |
| rs2274736 | PTPN21 | REC | GG | All | 1.151 | 1.001 | 1.324 | REC | REC | 0.0485 | . | 0.455 | GG | 77 | 402 | 1.42 | 1.092 | 1.842 | 0.0088 | 0.0308 | AA | 208 | 1498 |
| rs2266911 | STAG2/ODZ1 | GEN | TC | Female adj age | 0.928 | 0.815 | 1.057 | GEN HET | GEN | 0.2614 | 0.50818 | | TC | 5 | 13 | 2.74 | 1.13 | 6.635 | 0.0257 | 0.0163 | CC | 278 | 1332 |
| rs2266911 | STAG2/ODZ1 | GEN | TT | Female adj age | 0.931 | 0.687 | 1.263 | GEN HOM | GEN | 0.647 | 0.50818 | | TT | 67 | 261 | 1.3 | 0.996 | 1.699 | 0.0533 | 0.0163 | CC | 278 | 1332 |
| rs2266911 | STAG2/ODZ1 | ADD | T | Female adj age | 0.943 | 0.848 | 1.048 | ADD | ADD | 0.2766 | . | | | | | | | | | 0.0163 | CC | 278 | 1332 |
| rs2266911 | STAG2/ODZ1 | DOM | TC+TT | Female adj age | 0.928 | 0.819 | 1.052 | DOM | DOM | 0.2447 | . | | TC+TT | 72 | 274 | 1.35 | 1.042 | 1.751 | 0.0232 | 0.0163 | CC | 278 | 1332 |
| rs2266911 | STAG2/ODZ1 | REC | TT | Female adj age | 0.954 | 0.706 | 1.291 | REC | REC | 0.7616 | . | | TT | 67 | 261 | 1.29 | 0.986 | 1.679 | 0.0639 | 0.0163 | CC | 278 | 1332 |
| rs3765407 | LUZP1 | GEN | GT | All | 0.958 | 0.869 | 1.055 | GEN HET | GEN | 0.3815 | 0.01959 | 0.338 | GT | 165 | 981 | 1.2 | 0.998 | 1.441 | 0.0521 | 0.1515 | TT | 372 | 2519 |
| rs3765407 | LUZP1 | GEN | GG | All | 1.392 | 1.08 | 1.794 | GEN HOM | GEN | 0.0106 | 0.01959 | 0.338 | GG | 18 | 128 | 1.07 | 0.661 | 1.725 | 0.7881 | 0.1515 | TT | 372 | 2519 |
| rs3765407 | LUZP1 | ADD | G | All | 1.031 | 0.951 | 1.118 | ADD | ADD | 0.4595 | . | 0.338 | | | | | | | | 0.1515 | TT | 372 | 2519 |
| rs3765407 | LUZP1 | DOM | GT+G/G | All | 0.994 | 0.905 | 1.091 | DOM | DOM | 0.8922 | . | 0.338 | GT+G/G | 183 | 1109 | 1.19 | 0.993 | 1.415 | 0.06 | 0.1515 | TT | 372 | 2519 |
| rs3765407 | LUZP1 | REC | GG | All | 1.409 | 1.095 | 1.814 | REC | REC | 0.0077 | . | 0.338 | GG | 18 | 128 | 1.07 | 0.661 | 1.725 | 0.7881 | 0.1515 | TT | 372 | 2519 |
| rs4524 | F5 | GEN | CT | All | 0.77 | 0.701 | 0.844 | GEN HET | GEN | <.0001 | 0 | 0.3 | CT | 159 | 1185 | 0.82 | 0.681 | 0.991 | 0.0404 | 0.1197 | TT | 350 | 2195 |
| rs4524 | F5 | GEN | CC | All | 0.613 | 0.507 | 0.741 | GEN HOM | GEN | <.0001 | 0 | 0.3 | CC | 28 | 175 | 0.98 | 0.666 | 1.439 | 0.9138 | 0.1197 | TT | 350 | 2195 |
| rs4524 | F5 | ADD | C | All | 0.776 | 0.722 | 0.834 | ADD | ADD | <.0001 | . | 0.3 | | | | | | | | 0.1197 | TT | 350 | 2195 |
| rs4524 | F5 | DOM | CT+CC | All | 0.745 | 0.682 | 0.814 | DOM | DOM | <.0001 | . | 0.3 | CT+CC | 187 | 1360 | 0.84 | 0.705 | 1.006 | 0.058 | 0.1197 | TT | 350 | 2195 |
| rs4524 | F5 | REC | CC | All | 0.677 | 0.562 | 0.816 | REC | REC | <.0001 | . | 0.3 | CC | 28 | 175 | 0.98 | 0.666 | 1.439 | 0.9138 | 0.1197 | TT | 350 | 2195 |
| rs2070006 | | GEN | TC | All | 1.271 | 1.152 | 1.402 | GEN HET | GEN | <.0001 | 0 | 0.251 | TC | 265 | 1756 | 1.1 | 0.903 | 1.348 | 0.3359 | 0.0761 | CC | 150 | 1084 |
| rs2070006 | | GEN | TT | All | 1.531 | 1.348 | 1.738 | GEN HOM | GEN | <.0001 | 0 | 0.251 | TT | 124 | 720 | 1.31 | 1.036 | 1.669 | 0.0244 | 0.0761 | CC | 150 | 1084 |
| rs2070006 | | ADD | T | All | 1.242 | 1.167 | 1.322 | ADD | ADD | <.0001 | . | 0.251 | | | | | | | | 0.0761 | CC | 150 | 1084 |
| rs2070006 | | DOM | TC+TT | All | 1.337 | 1.219 | 1.467 | DOM | DOM | <.0001 | . | 0.251 | TC+TT | 389 | 2476 | 1.16 | 0.963 | 1.404 | 0.1168 | 0.0761 | CC | 150 | 1084 |
| rs2070006 | | REC | TT | All | 1.329 | 1.187 | 1.487 | REC | REC | <.0001 | . | 0.251 | TT | 124 | 720 | 1.31 | 1.036 | 1.669 | 0.0244 | 0.0761 | CC | 150 | 1084 |
| rs4253418 | F11 | GEN | AG | All | 0.773 | 0.653 | 0.915 | GEN HET | GEN | 0.0028 | 0.01096 | 0.0809 | AG | 33 | 233 | 0.9 | 0.633 | 1.281 | 0.5596 | 0.0992 | GG | 506 | 3321 |
| rs4253418 | F11 | GEN | AA | All | 0.87 | 0.349 | 2.165 | GEN HOM | GEN | 0.7643 | 0.01096 | 0.0809 | AA | 3 | 8 | 3.29 | 1.058 | 10.254 | 0.0397 | 0.0992 | GG | 506 | 3321 |
| rs4253418 | F11 | ADD | A | All | 0.789 | 0.673 | 0.926 | ADD | ADD | 0.0036 | . | 0.0809 | | | | | | | | 0.0992 | GG | 506 | 3321 |
| rs4253418 | F11 | DOM | AG+AA | All | 0.776 | 0.657 | 0.916 | DOM | DOM | 0.0027 | . | 0.0809 | AG+AA | 36 | 241 | 0.96 | 0.683 | 1.344 | 0.8053 | 0.0992 | GG | 506 | 3321 |

EP 2 635 709 B1

| | | Primary VT | | | | | | | | | | | Recurrent VT | | | | | | | | | | |
|---|---|---|---|---|---|---|---|---|---|---|---|---|---|---|---|---|---|---|---|---|---|---|---|
| SNP rs # | Gene | MODE | GENO TYPE | Strata | Odds Ratio | OR 95% CI lower | OR 95% CI upper | EFFECT LABEL | Variable | ProbChiSq | P_DF2 | HW(control l)pExact | GENO_AL | EVENTS_ALL | TOTAL_ALL | HR_AL | HR 95% CI lower_A LL | HR 95% CI upper_ ALL | P-value_AL L | P_DF2_A LL | Ref geno | EVENTS_A LL | TOTAL ALL |
| rs4253418 | F11 | REC | AA | All | 0.887 | 0.356 | 2.208 | REC | REC | 0.7968 | . | 0.0809 | AA | 3 | 8 | 3.29 | 1.058 | 10.254 | 0.0397 | 0.0992 | GG | 508 | 3321 |
| rs169713 | | GEN | CT | All | 1.145 | 1.042 | 1.258 | GEN HET | GEN | 0.0048 | 0.01464 | 0.0383 | CT | 165 | 1186 | 0.85 | 0.709 | 1.029 | 0.0975 | 0.1511 | TT | 338 | 2143 |
| rs169713 | | GEN | CC | All | 1.129 | 0.917 | 1.389 | GEN HOM | GEN | 0.2521 | 0.01464 | 0.0383 | CC | 29 | 171 | 1.15 | 0.789 | 1.685 | 0.4617 | 0.1511 | TT | 338 | 2143 |
| rs169713 | | ADD | C | All | 1.108 | 1.028 | 1.194 | ADD | ADD | 0.0075 | . | 0.0383 | | | | | | | | 0.1511 | TT | 338 | 2143 |
| rs169713 | | DOM | CT+C C | All | 1.143 | 1.044 | 1.251 | DOM | DOM | 0.0037 | . | 0.0383 | CT+C C | 194 | 1357 | 0.89 | 0.745 | 1.06 | 0.1906 | 0.1511 | TT | 338 | 2143 |
| rs169713 | | REC | CC | All | 1.078 | 0.878 | 1.323 | REC | REC | 0.4732 | . | 0.0383 | CC | 29 | 171 | 1.15 | 0.789 | 1.685 | 0.4617 | 0.1511 | TT | 338 | 2143 |
| rs8176750 | ABO | GEN | AC | All | 0.93 | 0.815 | 1.061 | GEN HET | GEN | 0.281 | 0.47492 | 1 | AC | 62 | 425 | 0.95 | 0.727 | 1.235 | 0.6915 | 0.0912 | CC | 469 | 3083 |
| rs8176750 | ABO | GEN | AA | All | 0.813 | 0.413 | 1.602 | GEN HOM | GEN | 0.55 | 0.47492 | 1 | AA | 4 | 14 | 2.94 | 1.094 | 7.894 | 0.0325 | 0.0912 | CC | 469 | 3083 |
| rs8176750 | ABO | ADD | A | All | 0.926 | 0.818 | 1.049 | ADD | ADD | 0.2264 | . | 1 | | | | | | | | 0.0912 | CC | 469 | 3083 |
| rs8176750 | ABO | DOM | AC+A A | All | 0.926 | 0.813 | 1.055 | DOM | DOM | 0.2462 | . | 1 | AC+A A | 66 | 439 | 0.99 | 0.764 | 1.279 | 0.9286 | 0.0912 | CC | 469 | 3083 |
| rs8176750 | ABO | REC | AA | All | 0.821 | 0.417 | 1.616 | REC | REC | 0.5677 | . | 1 | AA | 4 | 14 | 2.94 | 1.094 | 7.894 | 0.0325 | 0.0912 | CC | 469 | 3083 |
| rs8176750 | ABO | GEN | AC | age sex among Dom (GGorGT) of rs817671 9 | 0.659 | 0.574 | 0.757 | GEN HET | GEN | <.0001 | 1.032E-08 | | AC | 61 | 421 | 0.88 | 0.672 | 1.159 | 0.3685 | 0.0704 | CC | 345 | 2125 |
| rs8176750 | ABO | GEN | AA | age sex among Dom (GGorGT) of rs817671 9 | 0.582 | 0.295 | 1.148 | GEN HOM | GEN | 0.1183 | 1.032E-08 | | AA | 4 | 14 | 2.87 | 1.063 | 7.725 | 0.0374 | 0.0704 | CC | 345 | 2125 |
| rs8176750 | ABO | ADD | A | age sex among Dom (GGorGT) of rs817671 9 | 0.672 | 0.59 | 0.765 | ADD | ADD | <.0001 | . | | | | | | | | | 0.0704 | CC | 345 | 2125 |
| rs8176750 | ABO | DOM | AC+A A | age sex among Dom (GGorGT) of rs817671 9 | 0.657 | 0.573 | 0.752 | DOM | DOM | <.0001 | . | | AC+A A | 65 | 435 | 0.92 | 0.707 | 1.201 | 0.5458 | 0.0704 | CC | 345 | 2125 |

EP 2 635 709 B1

| SNP rs # | Gene | MODE | GENO TYPE | Strata | Odds Ratio | OR 95% CI lower | OR 95% CI upper | EFFECT LABEL | Variable | ProbChiSq | P_DF2 | HW(control)pExact | GENO_ALL | EVENTS_ALL | TOTAL_ALL | HR_AL | HR 95% CI lower_ALL | HR 95% CI upper_ALL | P-value_AL | P_DF2_AL | Ref geno | EVENTS_ALL | TOTAL_ALL |
|---|---|---|---|---|---|---|---|---|---|---|---|---|---|---|---|---|---|---|---|---|---|---|---|
| rs8176750 | ABO | REC | AA | age sex among Dom (GGorGT) of rs8176719 | 0.632 | 0.321 | 1.246 | REC | REC | 0.1851 | . | | AA | 4 | 14 | 2.92 | 1.086 | 7.876 | 0.0337 | 0.0704 | CC | 345 | 2125 |
| rs8176719 | ABO | GEN | GT | All | 2.023 | 1.833 | 2.232 | GEN HET | GEN | <.0001 | 0 | 0.156 | GT | 301 | 1928 | 1.21 | 0.984 | 1.498 | 0.0704 | 0.0549 | TT | 123 | 952 |
| rs8176719 | ABO | GEN | GG | All | 2.491 | 2.174 | 2.853 | GEN HOM | GEN | <.0001 | 0 | 0.156 | GG | 111 | 642 | 1.36 | 1.052 | 1.759 | 0.019 | 0.0549 | TT | 123 | 952 |
| rs8176719 | ABO | ADD | G | All | 1.662 | 1.556 | 1.774 | ADD | ADD | <.0001 | . | 0.156 | | | | | | | | 0.0549 | TT | 123 | 952 |
| rs8176719 | ABO | DOM | GT+GG | All | 2.124 | 1.934 | 2.333 | DOM | DOM | <.0001 | . | 0.156 | GT+GG | 412 | 2570 | 1.25 | 1.022 | 1.529 | 0.0301 | 0.0549 | TT | 123 | 952 |
| rs8176719 | ABO | REC | GG | All | 1.646 | 1.457 | 1.86 | REC | REC | <.0001 | . | 0.156 | GG | 111 | 642 | 1.36 | 1.052 | 1.759 | 0.019 | 0.0549 | TT | 123 | 952 |
| rs2069946 | PROCR | GEN | CT | All | 1.304 | 1.133 | 1.5 | GEN HET | GEN | 0.0002 | 0.00076 | 0.131 | CT | 55 | 429 | 0.79 | 0.599 | 1.047 | 0.1017 | 0.1565 | TT | 482 | 3081 |
| rs2069946 | PROCR | GEN | CC | All | 1.344 | 0.691 | 2.613 | GEN HOM | GEN | 0.3835 | 0.00076 | 0.131 | CC | 1 | 16 | 0.36 | 0.05 | 2.545 | 0.3048 | 0.1585 | TT | 482 | 3081 |
| rs2069946 | PROCR | ADD | C | All | 1.282 | 1.125 | 1.46 | ADD | ADD | 0.0002 | . | 0.131 | | | | | | | | 0.1585 | TT | 482 | 3081 |
| rs2069946 | PROCR | DOM | CT+CC | All | 1.305 | 1.137 | 1.498 | DOM | DOM | 0.0002 | . | 0.131 | CT+CC | 56 | 445 | 0.78 | 0.588 | 1.023 | 0.0718 | 0.1585 | TT | 482 | 3081 |
| rs2069946 | PROCR | REC | CC | All | 1.305 | 0.672 | 2.538 | REC | REC | 0.4319 | . | 0.131 | CC | 1 | 16 | 0.36 | 0.05 | 2.545 | 0.3048 | 0.1585 | TT | 482 | 3081 |
| rs2266911 | STAG2/ODZ1 | GEN | TC | male age among | 2.858 | 1.089 | 7.553 | GEN HET | GEN | 0.0329 | 0.00558 | | TC | 51 | 590 | 0.75 | 0.542 | 1.033 | 0.0784 | 0.0881 | CC | 136 | 1292 |
| rs2266911 | STAG2/ODZ1 | GEN | TT | male age among | 0.815 | 0.688 | 0.967 | GEN HOM | GEN | 0.0189 | 0.00558 | | TT | 4 | 77 | 0.47 | 0.175 | 1.278 | 0.1399 | 0.0881 | CC | 136 | 1292 |
| rs2266911 | STAG2/ODZ1 | ADD | T | male age among | 0.909 | 0.835 | 0.99 | ADD | ADD | 0.0276 | . | | | | | | | | | 0.0881 | CC | 136 | 1292 |
| rs2266911 | STAG2/ODZ1 | DOM | TC+TT | male age among | 0.845 | 0.714 | 0.999 | DOM | DOM | 0.0493 | . | | TC+TT | 55 | 667 | 0.72 | 0.524 | 0.982 | 0.0384 | 0.0881 | CC | 136 | 1292 |
| rs2266911 | STAG2/ODZ1 | REC | TT | male age among | 0.81 | 0.683 | 0.961 | REC | REC | 0.0154 | . | | TT | 4 | 77 | 0.52 | 0.192 | 1.389 | 0.1901 | 0.0881 | CC | 136 | 1292 |
| rs6003 | F13B | GEN | GA | All | 1.181 | 1.049 | 1.33 | GEN HET | GEN | 0.0059 | 0.0099 | 0.00061 | GA | 94 | 605 | 1.01 | 0.81 | 1.267 | 0.9087 | 0.256 | AA | 421 | 2802 |
| rs6003 | F13B | GEN | GG | All | 1.315 | 0.904 | 1.914 | GEN HOM | GEN | 0.1523 | 0.0099 | 0.00061 | GG | 12 | 53 | 1.62 | 0.913 | 2.88 | 0.0988 | 0.256 | AA | 421 | 2802 |
| rs6003 | F13B | ADD | G | All | 1.172 | 1.057 | 1.298 | ADD | ADD | 0.0025 | . | 0.00061 | | | | | | | | 0.256 | AA | 421 | 2802 |

## TABLE 8, page 7 of 9

| SNP rs # | Gene | MODE | GENO TYPE | Strata | Odds Ratio | OR 95% CI lower | OR 95% CI upper | Variable LABEL | EFFECT LABEL | ProbChiSq | P_DF2 | HW(control)pExact | GENO_ALL | EVENTS_ALL | TOTAL_ALL | HR_ALL | HR 95% CI lower_ALL | HR 95% CI upper_ALL | P-value_ALL | P_DF2_ALL | Ref geno | EVENTS_ALL | TOTAL ALL |
|---|---|---|---|---|---|---|---|---|---|---|---|---|---|---|---|---|---|---|---|---|---|---|---|
| rs6003 | F13B | DOM | GA+G/G | All | 1.191 | 1.052 | 1.335 | DOM | DOM | 0.0028 | | 0.00061 | GA+G/G | 106 | 658 | 1.06 | 0.855 | 1.31 | 0.6027 | 0.256 | AA | 421 | 2802 |
| rs6003 | F13B | REC | GG | All | 1.279 | 0.88 | 1.861 | REC | REC | 0.1976 | | 0.00061 | GG | 12 | 53 | 1.62 | 0.913 | 2.88 | 0.0988 | 0.256 | AA | 421 | 2802 |
| rs1417121 | SDCCAG8/AKT3 | GEN | CG | All | 1.072 | 0.978 | 1.175 | GEN | GEN HET | 0.1388 | 0.00001 | 0.795 | CG | 219 | 1447 | 1.02 | 0.854 | 1.224 | 0.8117 | 0.2183 | GG | 259 | 1733 |
| rs1417121 | SDCCAG8/AKT3 | GEN | CC | All | 1.47 | 1.256 | 1.72 | GEN | GEN HOM | <.0001 | 0.00001 | 0.795 | CC | 64 | 377 | 1.27 | 0.967 | 1.672 | 0.0854 | 0.2183 | GG | 259 | 1733 |
| rs1417121 | SDCCAG8/AKT3 | ADD | C | All | 1.155 | 1.08 | 1.235 | ADD | ADD | <.0001 | | 0.795 | | | | | | | | 0.2183 | GG | 259 | 1733 |
| rs1417121 | SDCCAG8/AKT3 | DOM | CG+C/C | All | 1.135 | 1.041 | 1.239 | DOM | DOM | 0.0043 | | 0.795 | CG+C | 283 | 1824 | 1.07 | 0.904 | 1.266 | 0.4343 | 0.2183 | GG | 259 | 1733 |
| rs1417121 | SDCCAG8/AKT3 | REC | CC | All | 1.425 | 1.224 | 1.659 | REC | REC | <.0001 | | 0.795 | CC | 64 | 377 | 1.27 | 0.967 | 1.672 | 0.0854 | 0.2183 | GG | 259 | 1733 |
| rs12744297 | AKT3 | GEN | GA | All | 1.107 | 1.009 | 1.215 | GEN | GEN HET | 0.0324 | 0.00055 | 0.14 | GA | 259 | 1577 | 1.12 | 0.934 | 1.34 | 0.2246 | 0.2172 | AA | 217 | 1479 |
| rs12744297 | AKT3 | GEN | GG | All | 1.311 | 1.138 | 1.511 | GEN | GEN HOM | 0.0002 | 0.00055 | 0.14 | GG | 59 | 469 | 0.89 | 0.67 | 1.191 | 0.4428 | 0.2172 | AA | 217 | 1479 |
| rs12744297 | AKT3 | ADD | G | All | 1.133 | 1.063 | 1.209 | ADD | ADD | 0.0001 | | 0.14 | | | | | | | | 0.2172 | AA | 217 | 1479 |
| rs12744297 | AKT3 | DOM | GA+G/G | All | 1.148 | 1.051 | 1.253 | DOM | DOM | 0.0021 | | 0.14 | GA+G | 318 | 2046 | 1.07 | 0.889 | 1.27 | 0.4531 | 0.2172 | AA | 217 | 1479 |
| rs12744297 | AKT3 | REC | GG | All | 1.246 | 1.09 | 1.424 | REC | REC | 0.0012 | | 0.14 | GG | 59 | 469 | 0.89 | 0.67 | 1.191 | 0.4428 | 0.2172 | AA | 217 | 1479 |
| rs3733402 | KLKB1 | GEN | GA | All | 0.798 | 0.721 | 0.883 | GEN | GEN HET | <.0001 | 0 | 0.592 | GA | 252 | 1736 | 0.88 | 0.725 | 1.058 | 0.1696 | 0.2111 | AA | 189 | 1133 |
| rs3733402 | KLKB1 | GEN | GG | All | 0.874 | 0.595 | 0.763 | GEN | GEN HOM | <.0001 | 0 | 0.592 | GG | 97 | 685 | 0.82 | 0.644 | 1.051 | 0.1195 | 0.2111 | AA | 189 | 1133 |
| rs3733402 | KLKB1 | ADD | G | All | 0.819 | 0.77 | 0.871 | ADD | ADD | <.0001 | | 0.592 | | | | | | | | 0.2111 | AA | 189 | 1133 |
| rs3733402 | KLKB1 | DOM | GA+G/G | All | 0.758 | 0.689 | 0.835 | DOM | DOM | <.0001 | | 0.592 | GA+G | 349 | 2421 | 0.86 | 0.721 | 1.027 | 0.0967 | 0.2111 | AA | 189 | 1133 |
| rs3733402 | KLKB1 | REC | GG | All | 0.777 | 0.698 | 0.865 | REC | REC | 0.005 | | 0.592 | GG | 97 | 685 | 0.82 | 0.644 | 1.051 | 0.1189 | 0.2111 | AA | 189 | 1133 |
| rs3087505 | KLKB1 | GEN | AG | All | 0.86 | 0.758 | 0.952 | GEN | GEN HET | 0.0007 | 0.0001 | 0.00568 | AG | 84 | 595 | 0.89 | 0.705 | 1.123 | 0.3252 | 0.2805 | GG | 450 | 2940 |
| rs3087505 | KLKB1 | GEN | AA | All | 0.463 | 0.317 | 0.736 | GEN | GEN HOM | <.0001 | 0.0001 | 0.00568 | AA | 7 | 31 | 1.58 | 0.75 | 3.339 | 0.2284 | 0.2805 | GG | 450 | 2940 |
| rs3087505 | KLKB1 | ADD | A | All | 0.812 | 0.734 | 0.898 | ADD | ADD | <.0001 | | 0.00568 | | | | | | | | 0.2805 | GG | 450 | 2940 |
| rs3087505 | KLKB1 | DOM | AG+A/A | All | 0.82 | 0.734 | 0.916 | DOM | DOM | 0.0005 | | 0.00568 | AG+A | 91 | 626 | 0.92 | 0.735 | 1.153 | 0.4718 | 0.2805 | GG | 450 | 2940 |
| rs3087505 | KLKB1 | REC | AA | All | 0.498 | 0.327 | 0.757 | REC | REC | 0.0011 | | 0.00568 | AA | 7 | 31 | 1.58 | 0.75 | 3.339 | 0.2284 | 0.2805 | GG | 450 | 2940 |
| rs2480089 | KIF6 | GEN | CA | All | 0.89 | 0.811 | 0.976 | GEN | GEN HET | 0.0135 | 0.01339 | 0.0209 | CA | 236 | 1500 | 1.12 | 0.932 | 1.344 | 0.2266 | 0.2685 | AA | 225 | 1576 |
| rs2480089 | KIF6 | GEN | CC | All | 1.054 | 0.912 | 1.217 | GEN | GEN HOM | 0.4768 | 0.01339 | 0.0209 | CC | 71 | 424 | 1.22 | 0.931 | 1.588 | 0.151 | 0.2685 | AA | 225 | 1576 |
| rs2480089 | KIF6 | ADD | C | All | 0.98 | 0.918 | 1.046 | ADD | ADD | 0.5491 | | 0.0209 | | | | | | | | 0.2685 | AA | 225 | 1576 |
| rs2480089 | KIF6 | DOM | CA+C/C | All | 0.921 | 0.844 | 1.006 | DOM | DOM | 0.0671 | | 0.0209 | CA+C | 307 | 1924 | 1.14 | 0.96 | 1.354 | 0.135 | 0.2685 | AA | 225 | 1576 |
| rs2480089 | KIF6 | REC | CC | All | 1.117 | 0.975 | 1.281 | REC | REC | 0.1111 | | 0.0209 | CC | 71 | 424 | 1.22 | 0.931 | 1.588 | 0.151 | 0.2685 | AA | 225 | 1576 |

Primary VT | Recurrent VT

EP 2 635 709 B1

| | | Primary VT | | | | | | | | | | | Recurrent VT | | | | | | | | | |
|---|---|---|---|---|---|---|---|---|---|---|---|---|---|---|---|---|---|---|---|---|---|---|
| SNP rs # | Gene | MODE | GENO TYPE | Strata | Odds Ratio | OR 95% CI lower | OR 95% CI upper | EFFECT LABEL | Variable | ProbChiSq | P_DF2 | HW(control)pExact | GENO_ALL | EVENTS_ALL | TOTAL_ALL | HR_ALL | HR 95% CI lower_ALL | HR 95% CI upper_ALL | P-value_ALL | P_DF2_ALL | Refgeno | EVENTS_ALL | TOTAL_ALL |
| rs8176750 | ABO | GEN | AC | age sex among Dom (GGorGT) of rs8176719 | 0.659 | 0.574 | 0.757 | GEN HET | GEN | <.0001 | 1.032E-08 | | AC | 61 | 421 | 0.88 | 0.672 | 1.159 | 0.3685 | 0.0704 | CC | 345 | 2125 |
| rs8176750 | ABO | GEN | AA | age sex among Dom (GGorGT) of rs8176719 | 0.582 | 0.295 | 1.148 | GEN HOM | GEN | 0.1183 | 1.032E-08 | | AA | 4 | 14 | 2.87 | 1.063 | 7.725 | 0.0374 | 0.0704 | CC | 345 | 2125 |
| rs8176750 | ABO | ADD | A | age sex among Dom (GGorGT) of rs8176719 | 0.672 | 0.59 | 0.765 | ADD | ADD | <.0001 | | | | | | | | | | 0.0704 | CC | 345 | 2125 |
| rs8176750 | ABO | DOM | AC+AA | age sex among Dom (GGorGT) of rs8176719 | 0.657 | 0.573 | 0.752 | DOM | DOM | <.0001 | | | AC+AA | 65 | 435 | 0.92 | 0.707 | 1.201 | 0.5458 | 0.0704 | CC | 345 | 2125 |
| rs8176750 | ABO | REC | AA | age sex among Dom (GGorGT) of rs8176719 | 0.632 | 0.321 | 1.246 | REC | REC | 0.1851 | | | AA | 4 | 14 | 2.92 | 1.086 | 7.876 | 0.0337 | 0.0704 | CC | 345 | 2125 |
| rs8176719 | ABO | GEN | GT | All | 2.023 | 1.833 | 2.232 | GEN HET | GEN | <.0001 | 0 | 0.156 | GT | 301 | 1928 | 1.21 | 0.984 | 1.498 | 0.0704 | 0.0549 | TT | 123 | 952 |
| rs8176719 | ABO | GEN | GG | All | 2.491 | 2.174 | 2.853 | GEN HOM | GEN | <.0001 | 0 | 0.156 | GG | 111 | 642 | 1.38 | 1.052 | 1.759 | 0.019 | 0.0549 | TT | 123 | 952 |
| rs8176719 | ABO | ADD | G | All | 1.662 | 1.556 | 1.774 | ADD | ADD | <.0001 | | 0.156 | | | | | | | | 0.0549 | TT | 123 | 952 |
| rs8176719 | ABO | DOM | GT+GG | All | 2.124 | 1.934 | 2.333 | DOM | DOM | <.0001 | | 0.156 | GT+GG | 412 | 2570 | 1.25 | 1.022 | 1.529 | 0.0301 | 0.0549 | TT | 123 | 952 |
| rs8176719 | ABO | REC | GG | All | 1.646 | 1.457 | 1.86 | REC | REC | <.0001 | | 0.156 | GG | 111 | 642 | 1.38 | 1.052 | 1.759 | 0.019 | 0.0549 | TT | 123 | 952 |
| rs3730055 | AKT2 | GEN | TC | All | 0.981 | 0.868 | 1.11 | GEN HET | GEN | 0.7646 | 0.03411 | 0.766 | TC | 36 | 516 | 1.22 | 0.967 | 1.537 | 0.0934 | 0.2272 | CC | 436 | 2935 |

| | | | Primary VT | | | | | | | | | | | | Recurrent VT | | | | | | | | |
|---|---|---|---|---|---|---|---|---|---|---|---|---|---|---|---|---|---|---|---|---|---|---|---|
| SNP rs # | Gene | MODE | GENO TYPE | Strata | Odds Ratio | OR 95% CI lower | OR 95% CI upper | EFFECT LABEL | Variable | ProbChiSq | P_DF2 | HW(control l)pExact | GENO_ALL | EVENTS_ALL | TOTAL_ALL | HR_ALL | HR 95% CI lower_ALL | HR 95% CI upper_ALL | P-value_ALL | P_DF2_ALL | Ref geno | EVENTS_ALL | TOTAL ALL |
| rs3730055 | AKT2 | GEN | TT | All | 1.878 | 1.161 | 3.037 | GEN HOM | GEN | 0.0102 | 0.03411 | 0.766 | TT | 6 | 42 | 0.88 | 0.394 | 1.974 | 0.7595 | 0.2272 | CC | 436 | 2935 |
| rs3730055 | AKT2 | ADD | T | All | 1.05 | 0.94 | 1.173 | ADD | ADD | 0.397 | | 0.766 | | | | | | | | 0.2272 | CC | 436 | 2935 |
| rs3730055 | AKT2 | DOM | TC+TT | All | 1.017 | 0.902 | 1.146 | DOM | DOM | 0.7853 | | 0.766 | TC+TT | 92 | 558 | 1.19 | 0.95 | 1.489 | 0.1309 | 0.2272 | CC | 436 | 2935 |
| rs3730055 | AKT2 | REC | TT | All | 1.883 | 1.165 | 3.044 | REC | REC | 0.0098 | | 0.766 | TT | 6 | 42 | 0.88 | 0.394 | 1.974 | 0.7595 | 0.2272 | CC | 436 | 2935 |
| rs2304167 | GP6 | GEN | CT | All | 0.907 | 0.825 | 0.997 | GEN HET | GEN | 0.0442 | 0.04653 | 0.744 | CT | 176 | 1065 | 1.18 | 0.98 | 1.41 | 0.081 | 0.2165 | TT | 345 | 2364 |
| rs2304167 | GP6 | GEN | CC | All | 0.818 | 0.648 | 1.032 | GEN HOM | GEN | 0.0901 | 0.04653 | 0.744 | CC | 20 | 123 | 1.09 | 0.688 | 1.714 | 0.7231 | 0.2165 | TT | 345 | 2364 |
| rs2304167 | GP6 | ADD | C | All | 0.906 | 0.838 | 0.98 | ADD | ADD | 0.0133 | | 0.744 | | | | | | | | 0.2165 | TT | 345 | 2364 |
| rs2304167 | GP6 | DOM | CT+CC | All | 0.897 | 0.818 | 0.983 | DOM | DOM | 0.0198 | | 0.744 | CT+CC | 196 | 1188 | 1.17 | 0.978 | 1.39 | 0.0864 | 0.2165 | TT | 345 | 2364 |
| rs2304167 | GP6 | REC | CC | All | 0.844 | 0.67 | 1.063 | REC | REC | 0.1489 | | 0.744 | CC | 20 | 123 | 1.09 | 0.688 | 1.714 | 0.7231 | 0.2165 | TT | 345 | 2364 |
| rs1654416 | RDH13/GP6 | GEN | CT | All | 0.888 | 0.807 | 0.976 | GEN HET | GEN | 0.0143 | 0.01484 | | CT | 172 | 1042 | 1.17 | 0.975 | 1.405 | 0.0912 | 0.2362 | TT | 348 | 2379 |
| rs1654416 | RDH13/GP6 | GEN | CC | All | 0.798 | 0.629 | 1.013 | GEN HOM | GEN | 0.0641 | 0.01484 | | CC | 18 | 115 | 1.01 | 0.623 | 1.627 | 0.9773 | 0.2362 | TT | 348 | 2379 |
| rs1654416 | RDH13/GP6 | ADD | C | All | 0.89 | 0.822 | 0.963 | ADD | ADD | 0.0037 | | | | | | | | | | 0.2362 | TT | 348 | 2379 |
| rs1654416 | RDH13/GP6 | DOM | CT+CC | All | 0.878 | 0.801 | 0.962 | DOM | DOM | 0.0055 | | | CT+CC | 190 | 1157 | 1.15 | 0.966 | 1.377 | 0.1144 | 0.2362 | TT | 348 | 2379 |
| rs1654416 | RDH13/GP6 | REC | CC | All | 0.829 | 0.654 | 1.05 | REC | REC | 0.1203 | | | CC | 18 | 115 | 1.01 | 0.623 | 1.627 | 0.9773 | 0.2362 | TT | 348 | 2379 |

EP 2 635 709 B1

| SNP hCV # | SNP rs # | Gene | MODE | GENOTYPE | ADJUST | OR | OR 95% CI lower | OR 95% CI upper | ProbChiSq | P_DF2 | Risk allele | Rel allele | HW(CONTROL)pExact | Geno t | CASE cnt | CONTROL cnt | Geno t | CASE cnt | CONTROL cnt | Geno t | CASE cnt | CONTROL cnt |
|---|---|---|---|---|---|---|---|---|---|---|---|---|---|---|---|---|---|---|---|---|---|---|
| hCV16282389 | rs2726953 | SCARA5 | GEN | AG | sex age | 1.049 | 0.913 | 1.205 | 0.5017 | 0.0467 | A | G | 0.6 | A A | 201 | 149 | A G | 745 | 706 | G G | 895 | 887 |
| hCV16282389 | rs2726953 | SCARA5 | GEN | AA | sex age | 1.34 | 1.063 | 1.689 | 0.0133 | 0.0467 | A | G | 0.6 | A A | 201 | 149 | A G | 745 | 706 | G G | 895 | 887 |
| hCV16282389 | rs2726953 | SCARA5 | ADD | A | sex age | 1.115 | 1.01 | 1.232 | 0.0318 | | A | G | 0.6 | A A | 201 | 149 | A G | 745 | 706 | G G | 895 | 887 |
| hCV16282389 | rs2726953 | SCARA5 | DOM | AG or AA | sex age | 1.1 | 0.964 | 1.254 | 0.1567 | | A | G | 0.6 | A A | 201 | 149 | A G | 745 | 706 | G G | 895 | 887 |
| hCV16282389 | rs2726953 | SCARA5 | REC | AA | sex age | 1.312 | 1.049 | 1.639 | 0.0172 | | A | G | 0.6 | A A | 201 | 149 | A G | 745 | 706 | G G | 895 | 887 |
| hCV16282389 | rs2726953 | SCARA5 | GEN | AG | | 1.046 | 0.91 | 1.201 | 0.5266 | 0.0485 | A | G | 0.6 | A A | 201 | 149 | A G | 745 | 706 | G G | 895 | 887 |
| hCV16282389 | rs2726953 | SCARA5 | GEN | AA | | 1.337 | 1.061 | 1.685 | 0.0139 | 0.0485 | A | G | 0.6 | A A | 201 | 149 | A G | 745 | 706 | G G | 895 | 887 |
| hCV16282389 | rs2726953 | SCARA5 | ADD | A | | 1.113 | 1.008 | 1.23 | 0.0347 | | A | G | 0.6 | A A | 201 | 149 | A G | 745 | 706 | G G | 895 | 887 |
| hCV16282389 | rs2726953 | SCARA5 | DOM | AG or AA | | 1.097 | 0.962 | 1.25 | 0.1682 | | A | G | 0.6 | A A | 201 | 149 | A G | 745 | 706 | G G | 895 | 887 |
| hCV16282389 | rs2726953 | SCARA5 | REC | AA | | 1.31 | 1.049 | 1.637 | 0.0174 | | A | G | 0.6 | A A | 201 | 149 | A G | 745 | 706 | G G | 895 | 887 |
| hCV9326428 | rs687289 | ABO | GEN | AG | sex age | 2.188 | 1.891 | 2.532 | <.0001 | | 0 A | G | 0.377 | A A | 326 | 184 | A G | 1005 | 742 | G G | 512 | 824 |
| hCV9326428 | rs687289 | ABO | GEN | AA | sex age | 2.867 | 2.318 | 3.546 | <.0001 | | 0 A | G | 0.377 | A A | 326 | 184 | A G | 1005 | 742 | G G | 512 | 824 |
| hCV9326428 | rs687289 | ABO | ADD | A | sex age | 1.813 | 1.639 | 2.006 | <.0001 | | A | G | 0.377 | A A | 326 | 184 | A G | 1005 | 742 | G G | 512 | 824 |
| hCV9326428 | rs687289 | ABO | DOM | AG or AA | sex age | 2.322 | 2.021 | 2.668 | <.0001 | | A | G | 0.377 | A A | 326 | 184 | A G | 1005 | 742 | G G | 512 | 824 |
| hCV9326428 | rs687289 | ABO | REC | AA | sex age | 1.834 | 1.509 | 2.228 | <.0001 | | A | G | 0.377 | A A | 326 | 184 | A G | 1005 | 742 | G G | 512 | 824 |
| hCV9326428 | rs687289 | ABO | GEN | AG | | 2.18 | 1.885 | 2.521 | <.0001 | | 0 A | G | 0.377 | A A | 326 | 184 | A G | 1005 | 742 | G G | 512 | 824 |
| hCV9326428 | rs687289 | ABO | GEN | AA | | 2.851 | 2.307 | 3.524 | <.0001 | | 0 A | G | 0.377 | A A | 326 | 184 | A G | 1005 | 742 | G G | 512 | 824 |
| hCV9326428 | rs687289 | ABO | ADD | A | | 1.807 | 1.634 | 1.999 | <.0001 | | A | G | 0.377 | A A | 326 | 184 | A G | 1005 | 742 | G G | 512 | 824 |
| hCV9326428 | rs687289 | ABO | DOM | AG or AA | | 2.313 | 2.014 | 2.657 | <.0001 | | A | G | 0.377 | A A | 326 | 184 | A G | 1005 | 742 | G G | 512 | 824 |
| hCV9326428 | rs687289 | ABO | REC | AA | | 1.829 | 1.506 | 2.221 | <.0001 | | A | G | 0.377 | A A | 326 | 184 | A G | 1005 | 742 | G G | 512 | 824 |
| hCV15887091 | rs2519093 | ABO | GEN | TC | sex age | 2.135 | 1.852 | 2.462 | <.0001 | | 0 T | C | 0.0024 | T T | 121 | 79 | T C | 803 | 485 | C C | 921 | 1181 |
| hCV15887091 | rs2519093 | ABO | GEN | TT | sex age | 1.962 | 1.457 | 2.642 | <.0001 | | 0 T | C | 0.0024 | T T | 121 | 79 | T C | 803 | 485 | C C | 921 | 1181 |
| hCV15887091 | rs2519093 | ABO | ADD | T | sex age | 1.766 | 1.576 | 1.979 | <.0001 | | T | C | 0.0024 | T T | 121 | 79 | T C | 803 | 485 | C C | 921 | 1181 |
| hCV15887091 | rs2519093 | ABO | DOM | TC or TT | sex age | 2.111 | 1.843 | 2.419 | <.0001 | | T | C | 0.0024 | T T | 121 | 79 | T C | 803 | 485 | C C | 921 | 1181 |
| hCV15887091 | rs2519093 | ABO | REC | TT | sex age | 1.475 | 1.101 | 1.975 | 0.0092 | | T | C | 0.0024 | T T | 121 | 79 | T C | 803 | 485 | C C | 921 | 1181 |
| hCV15887091 | rs2519093 | ABO | GEN | TC | | 2.123 | 1.842 | 2.447 | <.0001 | | 0 T | C | 0.0024 | T T | 121 | 79 | T C | 803 | 485 | C C | 921 | 1181 |
| hCV15887091 | rs2519093 | ABO | GEN | TT | | 1.964 | 1.46 | 2.641 | <.0001 | | 0 T | C | 0.0024 | T T | 121 | 79 | T C | 803 | 485 | C C | 921 | 1181 |
| hCV15887091 | rs2519093 | ABO | ADD | T | | 1.76 | 1.571 | 1.972 | <.0001 | | T | C | 0.0024 | T T | 121 | 79 | T C | 803 | 485 | C C | 921 | 1181 |
| hCV15887091 | rs2519093 | ABO | DOM | TC or TT | | 2.101 | 1.834 | 2.406 | <.0001 | | T | C | 0.0024 | T T | 121 | 79 | T C | 803 | 485 | C C | 921 | 1181 |
| hCV15887091 | rs2519093 | ABO | REC | TT | | 1.48 | 1.106 | 1.981 | 0.0084 | | T | C | 0.0024 | T T | 121 | 79 | T C | 803 | 485 | C C | 921 | 1181 |
| hCV3188439 | rs4981022 | STAB2 | GEN | GA | sex age | 0.852 | 0.741 | 0.979 | 0.0242 | 0.0298 | G | A | 0.161 | G G | 190 | 206 | G A | 738 | 751 | A A | 919 | 796 |
| hCV3188439 | rs4981022 | STAB2 | GEN | GG | sex age | 0.799 | 0.642 | 0.995 | 0.0453 | 0.0298 | G | A | 0.161 | G G | 190 | 206 | G A | 738 | 751 | A A | 919 | 796 |
| hCV3188439 | rs4981022 | STAB2 | ADD | G | sex age | 0.88 | 0.798 | 0.97 | 0.0101 | | G | A | 0.161 | G G | 190 | 206 | G A | 738 | 751 | A A | 919 | 796 |
| hCV3188439 | rs4981022 | STAB2 | DOM | GA or GG | sex age | 0.841 | 0.737 | 0.959 | 0.0096 | | G | A | 0.161 | G G | 190 | 206 | G A | 738 | 751 | A A | 919 | 796 |
| hCV3188439 | rs4981022 | STAB2 | REC | GG | sex age | 0.861 | 0.699 | 1.062 | 0.1623 | | G | A | 0.161 | G G | 190 | 206 | G A | 738 | 751 | A A | 919 | 796 |
| hCV3188439 | rs4981022 | STAB2 | GEN | GA | | 0.851 | 0.741 | 0.978 | 0.0232 | 0.0285 | G | A | 0.161 | G G | 190 | 206 | G A | 738 | 751 | A A | 919 | 796 |
| hCV3188439 | rs4981022 | STAB2 | GEN | GG | | 0.799 | 0.642 | 0.994 | 0.0444 | 0.0285 | G | A | 0.161 | G G | 190 | 206 | G A | 738 | 751 | A A | 919 | 796 |
| hCV3188439 | rs4981022 | STAB2 | ADD | G | | 0.879 | 0.798 | 0.969 | 0.0096 | | G | A | 0.161 | G G | 190 | 206 | G A | 738 | 751 | A A | 919 | 796 |
| hCV3188439 | rs4981022 | STAB2 | DOM | GA or GG | | 0.84 | 0.737 | 0.958 | 0.0091 | | G | A | 0.161 | G G | 190 | 206 | G A | 738 | 751 | A A | 919 | 796 |
| hCV3188439 | rs4981022 | STAB2 | REC | GG | | 0.861 | 0.699 | 1.061 | 0.1606 | | G | A | 0.161 | G G | 190 | 206 | G A | 738 | 751 | A A | 919 | 796 |
| hCV3188431 | rs12229292 | STAB2 | GEN | TG | sex age | 0.994 | 0.867 | 1.14 | 0.9337 | 0.0033 | T | G | 0.0155 | T T | 158 | 99 | T G | 732 | 715 | G G | 961 | 942 |
| hCV3188431 | rs12229292 | STAB2 | GEN | TT | sex age | 1.563 | 1.196 | 2.042 | 0.0011 | 0.0033 | T | G | 0.0155 | T T | 158 | 99 | T G | 732 | 715 | G G | 961 | 942 |
| hCV3188431 | rs12229292 | STAB2 | ADD | T | sex age | 1.121 | 1.009 | 1.245 | 0.0332 | | T | G | 0.0155 | T T | 158 | 99 | T G | 732 | 715 | G G | 961 | 942 |
| hCV3188431 | rs12229292 | STAB2 | DOM | TG or TT | sex age | 1.063 | 0.933 | 1.212 | 0.3598 | | T | G | 0.0155 | T T | 158 | 99 | T G | 732 | 715 | G G | 961 | 942 |
| hCV3188431 | rs12229292 | STAB2 | REC | TT | sex age | 1.567 | 1.207 | 2.034 | 0.0007 | | T | G | 0.0155 | T T | 158 | 99 | T G | 732 | 715 | G G | 961 | 942 |
| hCV3188431 | rs12229292 | STAB2 | GEN | TG | | 1.004 | 0.875 | 1.151 | 0.9597 | 0.0035 | T | G | 0.0155 | T T | 158 | 99 | T G | 732 | 715 | G G | 961 | 942 |

| SNP hCV # | SNP rs # | Gene | MODE | GENO TYPE | ADJUST | OR | OR 95% CI lower | OR 95% CI upper | ProbCh iSq | P_DF2 | Risk allele | Ref allele | HW(CONTROL)pExact | Geno t | CASE cnt | CONTROL cnt | Geno t | CASE cnt | CONTROL cnt | Geno t | CASE cnt | CONTROL cnt |
|---|---|---|---|---|---|---|---|---|---|---|---|---|---|---|---|---|---|---|---|---|---|---|
| hCV3188431 | rs12229292 | STAB2 | GEN | TT | | 1.564 | 1.198 | 2.043 | 0.001 | 0.0035 | T | G | 0.0155 | T T | 158 | 99 | T G | 732 | 715 | G G | 961 | 942 |
| hCV3188431 | rs12229292 | STAB2 | ADD | T | | 1.126 | 1.014 | 1.25 | 0.0264 | | T | G | 0.0155 | T T | 158 | 99 | T G | 732 | 715 | G G | 961 | 942 |
| hCV3188431 | rs12229292 | STAB2 | DOM | TG or TT | | 1.072 | 0.94 | 1.222 | 0.2992 | | T | G | 0.0155 | T T | 158 | 99 | T G | 732 | 715 | G G | 961 | 942 |
| hCV3188431 | rs12229292 | STAB2 | REC | TT | | 1.562 | 1.204 | 2.026 | 0.0008 | | T | G | 0.0155 | T T | 158 | 99 | T G | 732 | 715 | G G | 961 | 942 |
| hCV2485050 | rs6575009 | | GEN | GA | sex age | 1.226 | 1.003 | 1.499 | 0.0465 | 0.1052 | G | A | 0.29 | G G | 13 | 9 | G A | 246 | 195 | A A | 1591 | 1551 |
| hCV2485050 | rs6575009 | | GEN | GG | sex age | 1.412 | 0.601 | 3.319 | 0.4282 | 0.1052 | G | A | 0.29 | G G | 13 | 9 | G A | 246 | 195 | A A | 1591 | 1551 |
| hCV2485050 | rs6575009 | | ADD | G | sex age | 1.22 | 1.015 | 1.466 | 0.0342 | | G | A | 0.29 | G G | 13 | 9 | G A | 246 | 195 | A A | 1591 | 1551 |
| hCV2485050 | rs6575009 | | DOM | GA or GG | sex age | 1.234 | 1.014 | 1.503 | 0.0358 | | G | A | 0.29 | G G | 13 | 9 | G A | 246 | 195 | A A | 1591 | 1551 |
| hCV2485050 | rs6575009 | | REC | GG | sex age | 1.378 | 0.587 | 3.236 | 0.4621 | | G | A | 0.29 | G G | 13 | 9 | G A | 246 | 195 | A A | 1591 | 1551 |
| hCV2485050 | rs6575009 | | GEN | GA | | 1.23 | 1.006 | 1.503 | 0.0432 | 0.0993 | G | A | 0.29 | G G | 13 | 9 | G A | 246 | 195 | A A | 1591 | 1551 |
| hCV2485050 | rs6575009 | | GEN | GG | | 1.408 | 0.6 | 3.304 | 0.4315 | 0.0993 | G | A | 0.29 | G G | 13 | 9 | G A | 246 | 195 | A A | 1591 | 1551 |
| hCV2485050 | rs6575009 | | ADD | G | | 1.222 | 1.017 | 1.468 | 0.0321 | | G | A | 0.29 | G G | 13 | 9 | G A | 246 | 195 | A A | 1591 | 1551 |
| hCV2485050 | rs6575009 | | DOM | GA or GG | | 1.238 | 1.017 | 1.506 | 0.0333 | | G | A | 0.29 | G G | 13 | 9 | G A | 246 | 195 | A A | 1591 | 1551 |
| hCV2485050 | rs6575009 | | REC | GG | | 1.373 | 0.585 | 3.22 | 0.4662 | | G | A | 0.29 | G G | 13 | 9 | G A | 246 | 195 | A A | 1591 | 1551 |
| hCV27960688 | rs4900088 | TC2N | GEN | AG | sex age | 1.131 | 0.973 | 1.314 | 0.1087 | 0.0034 | A | G | 0.59 | A A | 397 | 306 | A G | 913 | 865 | G G | 537 | 578 |
| hCV27960688 | rs4900088 | TC2N | GEN | AA | sex age | 1.387 | 1.147 | 1.677 | 0.0008 | 0.0034 | A | G | 0.59 | A A | 397 | 306 | A G | 913 | 865 | G G | 537 | 578 |
| hCV27960688 | rs4900088 | TC2N | ADD | A | sex age | 1.172 | 1.067 | 1.287 | 0.0009 | | A | G | 0.59 | A A | 397 | 306 | A G | 913 | 865 | G G | 537 | 578 |
| hCV27960688 | rs4900088 | TC2N | DOM | AG or AA | sex age | 1.198 | 1.039 | 1.38 | 0.0127 | | A | G | 0.59 | A A | 397 | 306 | A G | 913 | 865 | G G | 537 | 578 |
| hCV27960688 | rs4900088 | TC2N | REC | AA | sex age | 1.286 | 1.089 | 1.518 | 0.003 | | A | G | 0.59 | A A | 397 | 306 | A G | 913 | 865 | G G | 537 | 578 |
| hCV27960688 | rs4900088 | TC2N | GEN | AG | | 1.136 | 0.978 | 1.32 | 0.0951 | 0.0026 | A | G | 0.59 | A A | 397 | 306 | A G | 913 | 865 | G G | 537 | 578 |
| hCV27960688 | rs4900088 | TC2N | GEN | AA | | 1.396 | 1.155 | 1.688 | 0.0006 | 0.0026 | A | G | 0.59 | A A | 397 | 306 | A G | 913 | 865 | G G | 537 | 578 |
| hCV27960688 | rs4900088 | TC2N | ADD | A | | 1.176 | 1.071 | 1.291 | 0.0007 | | A | G | 0.59 | A A | 397 | 306 | A G | 913 | 865 | G G | 537 | 578 |
| hCV27960688 | rs4900088 | TC2N | DOM | AG or AA | | 1.204 | 1.045 | 1.387 | 0.0101 | | A | G | 0.59 | A A | 397 | 306 | A G | 913 | 865 | G G | 537 | 578 |
| hCV27960688 | rs4900088 | TC2N | REC | AA | | 1.291 | 1.094 | 1.524 | 0.0025 | | A | G | 0.59 | A A | 397 | 306 | A G | 913 | 865 | G G | 537 | 578 |
| hCV2889230 | rs11686314 | | GEN | AG | sex age | 0.943 | 0.807 | 1.103 | 0.4629 | 0.029 | A | G | 0.116 | A A | 24 | 43 | A G | 417 | 410 | G G | 1408 | 1303 |
| hCV2889230 | rs11686314 | | GEN | AA | sex age | 0.511 | 0.308 | 0.847 | 0.0092 | 0.029 | A | G | 0.116 | A A | 24 | 43 | A G | 417 | 410 | G G | 1408 | 1303 |
| hCV2889230 | rs11686314 | | ADD | A | sex age | 0.875 | 0.764 | 1.003 | 0.0545 | | A | G | 0.116 | A A | 24 | 43 | A G | 417 | 410 | G G | 1408 | 1303 |
| hCV2889230 | rs11686314 | | DOM | AG or AA | sex age | 0.902 | 0.775 | 1.049 | 0.1817 | | A | G | 0.116 | A A | 24 | 43 | A G | 417 | 410 | G G | 1408 | 1303 |
| hCV2889230 | rs11686314 | | REC | AA | sex age | 0.518 | 0.313 | 0.857 | 0.0105 | | A | G | 0.116 | A A | 24 | 43 | A G | 417 | 410 | G G | 1408 | 1303 |
| hCV2889230 | rs11686314 | | GEN | AG | | 0.941 | 0.805 | 1.1 | 0.4459 | 0.0316 | A | G | 0.116 | A A | 24 | 43 | A G | 417 | 410 | G G | 1408 | 1303 |
| hCV2889230 | rs11686314 | | GEN | AA | | 0.517 | 0.312 | 0.856 | 0.0104 | 0.0316 | A | G | 0.116 | A A | 24 | 43 | A G | 417 | 410 | G G | 1408 | 1303 |
| hCV2889230 | rs11686314 | | ADD | A | | 0.875 | 0.764 | 1.002 | 0.0542 | | A | G | 0.116 | A A | 24 | 43 | A G | 417 | 410 | G G | 1408 | 1303 |
| hCV2889230 | rs11686314 | | DOM | AG or AA | | 0.901 | 0.774 | 1.048 | 0.1761 | | A | G | 0.116 | A A | 24 | 43 | A G | 417 | 410 | G G | 1408 | 1303 |
| hCV2889230 | rs11686314 | | REC | AA | | 0.524 | 0.317 | 0.867 | 0.0119 | | A | G | 0.116 | A A | 24 | 43 | A G | 417 | 410 | G G | 1408 | 1303 |
| hCV31716902 | rs12999640 | | GEN | TC | sex age | 0.938 | 0.806 | 1.091 | 0.408 | 0.086 | T | C | 0.207 | T T | 34 | 50 | T C | 456 | 450 | C C | 1358 | 1252 |
| hCV31716902 | rs12999640 | | GEN | TT | sex age | 0.619 | 0.398 | 0.964 | 0.034 | 0.086 | T | C | 0.207 | T T | 34 | 50 | T C | 456 | 450 | C C | 1358 | 1252 |
| hCV31716902 | rs12999640 | | ADD | T | sex age | 0.889 | 0.781 | 1.012 | 0.0746 | | T | C | 0.207 | T T | 34 | 50 | T C | 456 | 450 | C C | 1358 | 1252 |
| hCV31716902 | rs12999640 | | DOM | TC or TT | sex age | 0.906 | 0.782 | 1.049 | 0.1866 | | T | C | 0.207 | T T | 34 | 50 | T C | 456 | 450 | C C | 1358 | 1252 |
| hCV31716902 | rs12999640 | | REC | TT | sex age | 0.63 | 0.405 | 0.979 | 0.0399 | | T | C | 0.207 | T T | 34 | 50 | T C | 456 | 450 | C C | 1358 | 1252 |
| hCV31716902 | rs12999640 | | GEN | TC | | 0.934 | 0.803 | 1.087 | 0.3778 | 0.092 | T | C | 0.207 | T T | 34 | 50 | T C | 456 | 450 | C C | 1358 | 1252 |
| hCV31716902 | rs12999640 | | GEN | TT | | 0.627 | 0.403 | 0.976 | 0.0386 | 0.092 | T | C | 0.207 | T T | 34 | 50 | T C | 456 | 450 | C C | 1358 | 1252 |

EP 2 635 709 B1

TABLE 9, page 3 of 3

| SNP hCV # | SNP rs # | Gene | MODE | GENO TYPE | ADJUST | OR | OR 95% CI lower | OR 95% CI upper | ProbChiSq | P_DF2 | Risk allele | Ref allele | HW(CONTROL)pExact | Genot | CASE cnt | CONTROL cnt | Genot | CASE cnt | CONTROL cnt | Genot | CASE cnt | CONTROL cnt |
|---|---|---|---|---|---|---|---|---|---|---|---|---|---|---|---|---|---|---|---|---|---|---|
| hCV31716902 | rs12999640 | | ADD | T | | 0.888 | 0.78 | 1.011 | 0.072 | . | T | C | 0.207 | T T | 34 | 50 | T C | 456 | 450 | C C | 1358 | 1252 |
| hCV31716902 | rs12999640 | | DOM | TC or TT | | 0.903 | 0.78 | 1.046 | 0.1741 | . | T | C | 0.207 | T T | 34 | 50 | T C | 456 | 450 | C C | 1358 | 1252 |
| hCV31716902 | rs12999640 | | REC | TT | | 0.638 | 0.411 | 0.991 | 0.0456 | . | T | C | 0.207 | T T | 34 | 50 | T C | 456 | 450 | C C | 1358 | 1252 |
| hCV27484761 | rs3783886 | PTPN21 | GEN | GA | sex age | 1.317 | 1.074 | 1.615 | 0.0081 | 0.0178 | G | A | 0.0779 | 0.17 | | | | | | | | |
| hCV27484761 | rs3783886 | PTPN21 | GEN | GG | sex age | 1.566 | 0.708 | 3.467 | 0.2683 | 0.0178 | G | A | 0.0779 | 0.17 | | | | | | | | |
| hCV27484761 | rs3783886 | PTPN21 | ADD | G | sex age | 1.304 | 1.085 | 1.567 | 0.0047 | . | G | A | 0.0779 | 0.17 | | | | | | | | |
| hCV27484761 | rs3783886 | PTPN21 | DOM | GA or GG | sex age | 1.33 | 1.09 | 1.623 | 0.005 | . | G | A | 0.0779 | 0.17 | | | | | | | | |
| hCV27484761 | rs3783886 | PTPN21 | REC | GG | sex age | 1.515 | 0.685 | 3.353 | 0.3049 | . | G | A | 0.0779 | 0.17 | | | | | | | | |
| hCV27484761 | rs3783886 | PTPN21 | GEN | GA | | 1.323 | 1.079 | 1.622 | 0.0071 | 0.0155 | G | A | 0.0779 | 0.17 | | | | | | | | |
| hCV27484761 | rs3783886 | PTPN21 | GEN | GG | | 1.575 | 0.712 | 3.481 | 0.2619 | 0.0155 | G | A | 0.0779 | 0.17 | | | | | | | | |
| hCV27484761 | rs3783886 | PTPN21 | ADD | G | | 1.309 | 1.089 | 1.573 | 0.004 | . | G | A | 0.0779 | 0.17 | | | | | | | | |
| hCV27484761 | rs3783886 | PTPN21 | DOM | GA or GG | | 1.336 | 1.095 | 1.63 | 0.0043 | . | G | A | 0.0779 | 0.17 | | | | | | | | |
| hCV27484761 | rs3783886 | PTPN21 | REC | GG | | 1.523 | 0.689 | 3.365 | 0.2984 | . | G | A | 0.0779 | 0.17 | | | | | | | | |

## Claims

1. A method for determining whether a human's risk for venous thrombosis (VT) is reduced by treatment with an HMG-CoA reductase inhibitor, the method comprising testing nucleic acid from said human for the presence or absence of an allele at a polymorphism (rs2036914) represented by position 101 of the nucleotide sequence of SEQ ID NO:713 or its complement, wherein the presence of C at position 101 of SEQ ID NO:713 or G at position 101 of its complement indicates said human's risk for VT is reduced by treatment with said HMG-CoA reductase inhibitor.

2. The method of claim 1, further comprising correlating the presence of said allele with a reduction of said risk for VT by an HMG-CoA reductase inhibitor.

3. The method of claim 2, wherein said correlating is performed by computer software.

4. The method of claim 1, wherein said HMG-CoA reductase inhibitor is a hydrophilic statin, or a hydrophobic statin.

5. The method of claim 1, wherein said HMG-CoA reductase inhibitor is selected from the group consisting of atorvastatin, rosuvastatin, pravastatin, simvastatin, fluvastatin, and lovastatin, or any combination thereof.

6. The method of claim 1, wherein said treatment with an HMG-CoA reductase inhibitor comprises treatment with an HMG-CoA reductase inhibitor in combination with at least one additional therapeutic agent.

7. The method of claim 6, wherein said HMG-CoA reductase inhibitor in combination with at least one additional therapeutic agent is selected from the group consisting of:

   simvastatin in combination with ezetimibe;
   lovastatin in combination with niacin;
   atorvastatin in combination with amlodipine besylate; and
   simvastatin in combination with niacin.

8. The method of claim 1, wherein said nucleic acid is a nucleic acid extract from a biological sample from said human.

9. The method of claim 8, wherein said biological sample is blood, saliva, or buccal cells.

10. The method of claim 8 or 9, further comprising preparing said nucleic acid extract from said biological sample prior to said testing, preferably further comprising obtaining said biological sample from said human prior to said preparing.

11. The method of claim 1, wherein said testing comprises nucleic acid amplification.

12. The method of claim 11, wherein said nucleic acid amplification is carried out by polymerase chain reaction.

13. The method of claim 1, wherein said testing is performed using sequencing, 5' nuclease digestion, molecular beacon assay, oligonucleotide ligation assay, single-stranded conformation polymorphism analysis, or denaturing gradient gel electrophoresis (DGGE) or an allele-specific method.

14. The method of claim 13, wherein said allele-specific method comprises allele-specific probe hybridization, allele-specific primer extension, or allele-specific amplification.

15. The method of claim 1 which is an automated method.

16. The method of claim 1, wherein said human is homozygous, or heterozygous for said allele.

17. The method of claim 1, wherein said VT is deep vein thrombosis (DVT), or pulmonary embolism (PE).

18. The method of claim 1, wherein said human did not have VT prior to said testing.

19. The method of claim 1, wherein said human had VT prior to said testing and said risk is for recurrent VT.

20. The method of claim 1, wherein the method further comprises detecting any one or more of the following polymorphisms: an *F5* polymorphism rs6025, an *FGG* polymorphism rs2066865, an *ABO* polymorphism rs8176719, and an *F2* polymorphism rs1799963.

21. The method of claim 20, wherein the method comprises detecting the following polymorphisms: an *F5* polymorphism rs6025, an *FGG* polymorphism rs2066865, an *ABO* polymorphism rs8176719, and an *F2* polymorphism rs1799963.

22. An HMG-CoA reductase inhibitor for use in a method of reducing the risk for VT, wherein the method comprises performing a method according to claim 1 and administering the HMG-CoA reductase inhibitor to a human who has said allele.

23. Use of a detection reagent in the method of claim 1, wherein said detection reagent is an allele-specific probe or an allele-specific primer.

24. Use of a test kit in the method of claim 1, wherein said test kit comprises one or more containers containing the detection reagent of claim 23 and one or more components selected from the group consisting of an enzyme, polymerase enzyme, ligase enzyme, buffer, amplification primer pair, dNTPs, ddNTPs, positive control nucleic acid, nucleic acid extraction reagent, and instructions for using said test kit which instruct that the presence of said allele indicates that said risk for VT is reduced by treatment with said HMG-CoA reductase inhibitor.

**Patentansprüche**

1. Verfahren zum Bestimmen, ob das Risiko eines Menschen für Venenthrombose (VT) durch Behandlung mit einem HMG-CoA-Reduktase-Inhibitor verringert wird, wobei das Verfahren ein Testen der Nukleinsäure von diesem Menschen auf die Anwesenheit oder Abwesenheit eines Allels bei einem Polymorphismus (rs2036914) umfasst, der durch Position 101 der Nukleotidsequenz von SEQ ID NO: 713 oder ihres Komplements dargestellt ist, wobei die Anwesenheit von C an Position 101 von SEQ ID NO: 713 oder G an Position 101 ihres Komplements anzeigt, dass das Risiko des Menschen für VT durch Behandlung mit dem HMG-CoA-Reduktase-Inhibitor verringert wird.

2. Verfahren nach Anspruch 1, ferner umfassend das Korrelieren der Anwesenheit des Allels mit einer Verringerung des Risikos für VT durch einen HMG-CoA-Reduktase-Inhibitor.

3. Verfahren nach Anspruch 2, wobei das Korrelieren durch Computer-Software durchgeführt wird.

4. Verfahren nach Anspruch 1, wobei der HMG-CoA-Reduktase-Inhibitor ein hydrophiles Statin oder ein hydrophobes Statin ist.

5. Verfahren nach Anspruch 1, wobei der HMG-CoA-Reduktase-Inhibitor ausgewählt ist aus der Gruppe bestehend aus Atorvastatin, Rosuvastatin, Pravastatin, Simvastatin, Fluvastatin und Lovastatin oder jeder Kombination davon.

6. Verfahren nach Anspruch 1, wobei die Behandlung mit einem HMG-CoA-Reduktase-Inhibitor eine Behandlung mit einem HMG-CoA-Reduktase-Inhibitor in Kombination mit mindestens einem zusätzlichen therapeutischen Wirkstoff umfasst.

7. Verfahren nach Anspruch 6, wobei der HMG-CoA-Reduktase-Inhibitor in Kombination mit mindestens einem zusätzlichen therapeutischen Wirkstoff ausgewählt ist aus der Gruppe bestehend aus:

Simvastatin in Kombination mit Ezetimib;
Lovastatin in Kombination mit Niacin;
Atorvastatin in Kombination mit Amlodipin besilat; und
Simvastatin in Kombination mit Niacin.

8. Verfahren nach Anspruch 1, wobei die Nukleinsäure ein Nukleinsäureextrakt aus einer biologischen Probe von dem Menschen ist.

9. Verfahren nach Anspruch 8, wobei die biologische Probe Blut, Speichel oder Wangenzellen ist.

10. Verfahren nach Anspruch 8 oder 9, ferner umfassend die Herstellung des Nukleinsäureextrakts aus der biologischen Probe vor dem Testen, vorzugsweise ferner umfassend das Gewinnen der biologischen Probe von dem Menschen vor der Herstellung.

11. Verfahren nach Anspruch 1, wobei das Testen eine Amplifikation der Nukleinsäure umfasst.

12. Verfahren nach Anspruch 11, wobei die Amplifikation der Nukleinsäure durch eine Polymerase-Kettenreaktion durchgeführt wird.

13. Verfahren nach Anspruch 1, wobei das Testen unter Verwendung von Sequenzierung, 5'-Nukleaseverdau, Molecular-Beacon-Assay, Oligonukleotid-Ligations-Assay, Einzelstrang-Konformationspolymorphismus-Analyse oder Denaturierender Gradienten-Gelelektrophorese (DGGE) oder einem allelspezifischen Verfahren durchgeführt wird.

**14.** Verfahren nach Anspruch 13, wobei das allelspezifische Verfahren allelspezifische Sondenhybridisierung, allelspezifische Primerextension oder allelspezifische Amplifikation umfasst.

**15.** Verfahren nach Anspruch 1, welches ein automatisiertes Verfahren ist.

**16.** Verfahren nach Anspruch 1, wobei der Mensch homozygot oder heterozygot für das Allel ist.

**17.** Verfahren nach Anspruch 1, wobei die VT eine Tiefe Venenthrombose (TVT) oder eine Lungenembolie (LE) ist.

**18.** Verfahren nach Anspruch 1, wobei der Mensch vor dem Testen keine VT hatte.

**19.** Verfahren nach Anspruch 1, wobei der Mensch vor dem Testen eine VT hatte und das Risiko für rezidivierende VT besteht.

**20.** Verfahren nach Anspruch 1, wobei das Verfahren ferner das Nachweisen eines oder mehrerer der folgenden Polymorphismen umfasst: eines *F5*-Polymorphismus rs6025, eines *FGG*-Polymorphismus rs2066865, eines *ABO*-Polymorphismus rs8176719 und eines *F2*-Polymorphismus rs1799963.

**21.** Verfahren nach Anspruch 20, wobei das Verfahren das Nachweisen der folgenden Polymorphismen umfasst: eines *F5*-Polymorphismus rs6025, eines *FGG*-Polymorphismus rs2066865, eines *ABO*-Polymorphismus rs8176719 und eines *F2*-Polymorphismus rs1799963.

**22.** HMG-CoA-Reduktase-Inhibitor zur Verwendung in einem Verfahren zum Verringern des Risikos für VT, wobei das Verfahren ein Durchführen eines Verfahrens nach Anspruch 1 und ein Verabreichen des HMG-CoA-Reduktase-Inhibitors an einen Menschen umfasst, der das Allel hat.

**23.** Verwendung eines Nachweisreagenzes im Verfahren von Anspruch 1, wobei das Nachweisreagenz eine allelspezifische Sonde oder ein allelspezifischer Primer ist.

**24.** Verwendung eines Testkits im Verfahren von Anspruch 1, wobei das Testkit einen oder mehrere Behälter umfasst, die das Nachweisreagenz von Anspruch 23 und eine oder mehrere Komponenten aufweisen, die ausgewählt sind aus der Gruppe bestehend aus einem Enzym, Polymerase-Enzym, Ligase-Enzym, Puffer, Primer-Paar für die Amplifikation, dNTPs, ddNTPs, einer positiven Kontrollnukleinsäure, einem Nukleinsäure-Extraktionsreagenz und Anweisungen zum Verwenden des Testkits, die angeben, dass die Anwesenheit des Allels anzeigt, dass das Risiko für VT durch eine Behandlung mit dem HMG-CoA-Reduktase-Inhibitor verringert ist.

**Revendications**

**1.** Procédé pour déterminer si le risque de thrombose veineuse (VT) chez un humain est réduit par traitement avec un inhibiteur de l'HMG-CoA réductase, le procédé comprenant le test d'acide nucléique dudit humain pour déceler la présence ou l'absence d'un allèle dans le cadre d'un polymorphisme (rs2036914) représenté par la position 101 de la séquence nucléotidique de SEQ ID N° 713 ou de son complément, dans lequel la présence de C en position 101 de la SEQ ID N° 713 ou de G en position 101 de son complément indique que le risque de VT pour ledit humain est réduit par traitement avec ledit inhibiteur de l'HMG-CoA réductase.

**2.** Procédé selon la revendication 1, comprenant en outre la corrélation de la présence dudit allèle avec une réduction dudit risque de VT par un inhibiteur de l'HMG-CoA réductase.

**3.** Procédé selon la revendication 2, dans lequel ladite corrélation est effectuée par un logiciel d'ordinateur.

**4.** Procédé selon la revendication 1, dans lequel ledit inhibiteur de l'HMG-CoA réductase est une statine hydrophile ou une statine hydrophobe.

**5.** Procédé selon la revendication 1, dans lequel ledit inhibiteur de l'HMG-CoA réductase est choisi dans le groupe constitué de l'atorvastatine, de la rosuvastatine, de la pravastatine, de la simvastatine, de la fluvastatine et de la lovastatine ou d'une quelconque de leurs combinaisons.

**6.** Procédé selon la revendication 1, dans lequel ledit traitement avec un inhibiteur de l'HMG-CoA réductase comprend le traitement avec un inhibiteur de l'HMG-CoA réductase en combinaison avec au moins un agent thérapeutique supplémentaire.

**7.** Procédé selon la revendication 6, dans lequel ledit inhibiteur de l'HMG-CoA réductase en combinaison avec au moins un agent thérapeutique supplémentaire est choisi dans le groupe constitué des suivants:

de la simvastatine en combinaison avec de l'ézétimibe;
de la lovastatine en combinaison avec de la niacine;
de l'atorvastatine en combinaison avec du bésylate d'amlodipine; et
de la simvastatine en combinaison avec de la niacine.

**8.** Procédé selon la revendication 1, dans lequel ledit acide nucléique est un extrait d'acide nucléique venant d'un échantillon biologique issu dudit humain.

**9.** Procédé selon la revendication 8, dans lequel ledit échantillon biologique est du sang, de la salive ou des cellules buccales.

**10.** Procédé selon la revendication 8 ou 9, comprenant en outre la préparation dudit extrait d'acide nucléique à partir dudit échantillon biologique avant ledit test, comprenant en outre de préférence l'obtention dudit échantillon biologique issu dudit humain avant ladite préparation.

**11.** Procédé selon la revendication 1, dans lequel ledit test comprend l'amplification de l'acide nucléique.

**12.** Procédé selon la revendication 11, dans lequel ladite amplification de l'acide nucléique est effectuée par une réaction en chaîne de polymérase.

**13.** Procédé selon la revendication 1, dans lequel ledit test est effectué en utilisant un séquencement, une digestion par nucléase en 5', un dosage par balise moléculaire, un dosage par ligature d'oligonucléotide, une analyse de polymorphisme de conformation monobrin ou une électrophorèse en gel sur gradient dénaturant (DGGE) ou un procédé spécifique à l'allèle.

**14.** Procédé selon la revendication 13, dans lequel ledit procédé spécifique à l'allèle comprend une hybridation de sonde spécifique à l'allèle, une extension d'amorce spécifique à l'allèle ou une amplification spécifique à l'allèle.

**15.** Procédé selon la revendication 1 qui est un procédé automatisé.

**16.** Procédé selon la revendication 1, dans lequel ledit humain est homozygote ou hétérozygote pour ledit allèle.

**17.** Procédé selon la revendication 1, dans lequel ladite VT est une thrombose veineuse profonde (DVT) ou une embolie pulmonaire (PE).

**18.** Procédé selon la revendication 1, dans lequel ledit humain n'a pas eu de VT avant ledit test.

**19.** Procédé selon la revendication 1, dans lequel ledit humain a eu une VT avant ledit test et ledit risque est pour une VT récurrente.

**20.** Procédé selon la revendication 1, dans lequel le procédé comprend en outre la détection de l'un quelconque ou plus des polymorphismes suivants: un polymorphisme de *F5* rs6025, un polymorphisme de *FGG* rs2066865, un polymorphisme d'*ABO* rs8176719 et un polymorphisme de *F2* rs1799963.

**21.** Procédé selon la revendication 20, dans lequel le procédé comprend la détection des polymorphismes suivants: un polymorphisme de *F5* rs6025, un polymorphisme de *FGG* rs2066865, un polymorphisme d'*ABO* rs8176719 et un polymorphisme de *F2* rs1799963.

**22.** Inhibiteur de réductase de HMG-CoA pour l'utilisation dans un procédé de réduction du risque de VT, dans lequel le procédé comprend la réalisation d'un procédé selon la revendication 1 et l'administration de l'inhibiteur de l'HMG-CoA réductase à un humain qui a ledit allèle.

**23.** Utilisation d'un réactif de détection dans le procédé selon la revendication 1, dans lequel ledit réactif de détection est une sonde spécifique à l'allèle ou une amorce spécifique à l'allèle.

**24.** Utilisation d'un kit d'essai dans le procédé de la revendication 1, dans laquelle ledit kit d'essai comprend un ou plusieurs récipients contenant le réactif de détection de la revendication 23 et un ou plusieurs composants choisis dans le groupe constitué d'un enzyme, d'un enzyme polymérase, d'un enzyme ligase, d'un tampon, d'une paire d'amorces d'amplification de dNTP, de ddNTP, d'un acide nucléique témoin positif, d'un réactif d'extraction d'acide nucléique et d'instructions pour utiliser ledit kit d'essai qui instruisent le fait que la présence dudit allène indique que ledit risque de VT est réduit par traitement avec ledit inhibiteur de l'HMG-CoA réductase.

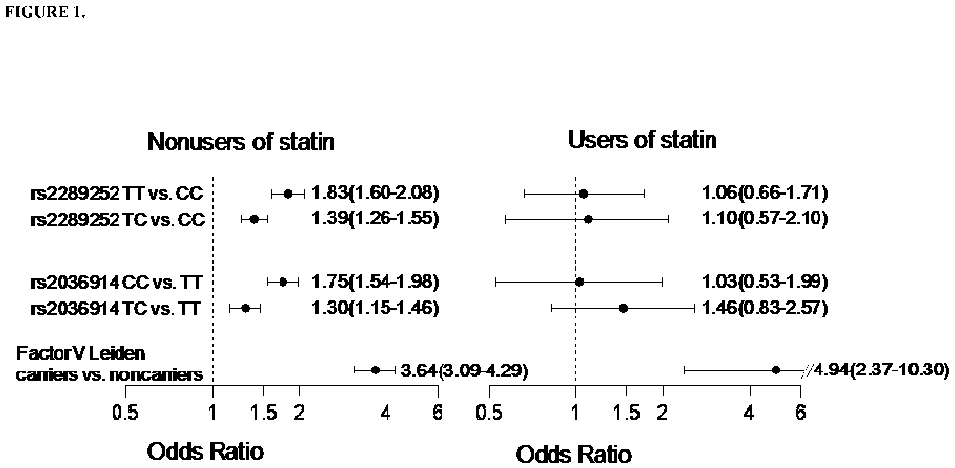

FIGURE 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9740462 A
- US 6770029 B
- US 4683195 A
- US 4683202 A
- US 5270184 A
- US 5422252 A
- US 5399491 A
- US 6027923 A
- US 5539082 A
- US 5527675 A
- US 5623049 A
- US 5714331 A
- WO 9604000 A
- US 4835263 A
- US 5801115 A
- EP 235726 A, Dattagupta
- WO 8911548 A, Saiki
- US 4988617 A
- US 4458066 A
- WO 9322456 A
- US 5210015 A
- US 5538848 A
- US 5118801 A
- US 5312728 A
- US 5866336 A
- US 6117635 A
- US 5837832 A, Chee
- WO 9511995 A
- US 5807522 A, Brown
- US 10620332 B
- US 10620333 B
- US 6124478 A
- US 6107024 A
- US 5994073 A
- US 5981768 A
- US 5871938 A
- US 5843681 A
- US 5800999 A
- US 5773628 A
- US 20020110828 A
- WO 95251116 A, Baldeschweiler
- US 5589136 A
- US 6153073 A, Dubrow
- US 6156181 A, Parce
- US 4988167 A
- US 6027889 A
- US 6268148 B
- US 5494810 A
- US 5830711 A
- US 6054564 A
- WO 9731256 A
- WO 0056927 A
- US 0117329 A
- US 09584905 B
- US 427818 P
- US 60445636 B
- US 60445494 B
- WO 9416101 A
- US 5498531 A
- US 4107288 A
- US 5145684 A
- US 5283317 A
- WO 9410300 A, Brent
- US 4816567 A
- US 4816397 A
- US 5693762 A
- US 5585089 A
- US 5565332 A
- US 4946778 A
- US 5637677 A
- US 4736866 A
- US 4870009 A, Leder
- US 4873191 A, Wagner
- WO 9707668 A
- WO 9707669 PCT

### Non-patent literature cited in the description

- *Seminars in Thrombosis and Hemostasis,* 2002, vol. 28 (2
- **STEIN et al.** *Chest,* 2002, vol. 122 (3), 960-962
- **WHITE.** *Circulation,* 2003, vol. 107 (1), I4-8
- HemAware newsletter. National Hemophilia Foundation, 2001, vol. 6
- **SELIGSOHN et al.** *New Eng J Med,* 2001, vol. 344 (16), 1222-1231
- **HEIT et al.** *Thromb Haemost,* 2001, vol. 86 (1), 452-463

- **SOUTO et al.** *Am J Hum Genet,* 2000, vol. 67 (6), 1452-1459
- **LARSEN et al.** *Epidemiology,* 2003, vol. 14 (3), 328-332
- **ROSENDAAL et al.** *Lancet,* 1999, vol. 353 (9159), 1167-1173
- **BERTINA et al.** *Nature,* 1994, vol. 369 (6475), 64-67
- **POORT et al.** *Blood,* 1996, vol. 88 (10), 3698-3703
- **ROSENDAAL.** *Lancet,* 1999, vol. 353 (9159), 1167-1173
- **KYRLE et al.** *N Engl J Med.,* 2000, vol. 343, 457-462
- **VAN HYLCKAMA VLIEG et al.** *Blood.,* 2000, vol. 95, 3678-3682
- **DE VISSER et al.** *Thromb Haemost.,* 2001, vol. 85, 1011-1017
- **MEIJERS et al.** *N Engl J Med.,* 2000, vol. 342, 696-701
- **LENSING et al.** *Lancet,* 1999, vol. 353, 479-485
- **KEARON et al.** *New Engl J Med,* 1999, vol. 340, 901-907
- **HEIT et al.** *Arch Intern Med.,* 2000, vol. 160, 761-768
- **SCHULMAN et al.** *J Thromb Haemost.,* 2006, vol. 4, 732-742
- **VAN DONGEN et al.** *Arch Intern Med.,* 2003, vol. 163, 1285-1293
- **MCRAE et al.** *Lancet,* 2006, vol. 368, 371-378
- **CUSHMAN et al.** *Am J Med.,* 2004, vol. 117, 19-25
- **BAGLIN et al.** *Lancet.,* 2003, vol. 362, 523-526
- **KEARON et al.** *N Engl J Med.,* 1999, vol. 340, 901-907
- **SCHULMAN et al.** *Am J Med.,* 1998, vol. 104, 332-338
- **VAN DEN BELT et al.** *Arch Intern Med.,* 1997, vol. 157, 227-232
- **PALARETI et al.** *N Engl J Med.,* 2006, vol. 355, 1780-1789
- **PRANDONI et al.** *Ann Intern Med.,* 2002, vol. 137, 955-960
- **LEE et al.** *Circulation,* 2003, vol. 107, I-17-I-21
- **RARH et al.** *Blood coagulation and fibrinolysis,* 1992, vol. 3, 451
- **D.D. WATERS.** *Clin Cardiol,* 2001, vol. 24 (8), III3-7
- **B.K. SINGH ; J.L. MEHTA.** *Curr Opin Cardiol,* 2002, vol. 17 (5), 503-11
- **K. ZIEGLER ; W. STUNKEL.** *Biochim Biophys Acta,* 1992, vol. 1139 (3), 203-9
- **M. YAMAZAKI et al.** *Am J Physiol,* 1993, vol. 264 (1), G36-44
- **T. KOMAI et al.** *Biochem Pharmacol,* 1992, vol. 43 (4), 667-70
- **B. HSIANG et al.** *J Biol Chem,* 1999, vol. 274 (52), 37161-37168
- **B.A. MASTERS et al.** *Toxicol Appl Pharmacol,* 1995, vol. 131 (1), 163-174
- **K. NAKAHARA et al.** *Toxicol Appl Pharmacol,* 1998, vol. 152 (1), 99-106
- **J.C. REIJNEVELD et al.** *Pediatr Res,* 1996, vol. 39 (6), 1028-1035
- **VAUGHAN et al.** Update on Statins: 2003. *Circulation,* 2004, vol. 110, 886-892
- **A.D. ROSES.** *Nature,* 2000, vol. 405 (6788), 857-865
- **V. MOOSER et al.** *J Thromb Haemost,* 2003, vol. 1 (7), 1398-1402
- **L.M. HUMMA ; S.G. TERRA.** *Am J Health Syst Pharm,* 2002, vol. 59 (13), 1241-1252
- **IAKOUBOVA et al.** Polymorphism in KIF6 gene and benefit from statins after acute coronary syndromes: results from the PROVE IT-TIMI 22 study. *J Am Coll Cardiol.,* 29 January 2008, vol. 51 (4), 449-55
- **IAKOUBOVA et al.** Association of the 719Arg variant of K1F6 with both increased risk of coronary events and with greater response to statin therapy. *J Am Coll Cardiol.,* 03 June 2008, vol. 51 (22), 2195
- **IAKOUBOVA et al.** KIF6 Trp719Arg polymorphism and the effect of statin therapy in elderly patients: results from the PROSPER study. *Eur J Cardiovasc Prev Rehabil.,* 20 April 2010
- **SHIFFMAN et al.** Effect of pravastatin therapy on coronary events in carriers of the KIF6 719Arg allele from the cholesterol and recurrent events trial. *Am J Cardiol.,* 01 May 2010, vol. 105 (9), 1300-5
- **RIDKER et al.** Rosuvastatin to prevent vascular events in men and women with elevated C-reactive protein. *N Engl J Med.,* 20 November 2008, vol. 359 (21), 2195-207
- **DOGGEN et al.** HMG CoA reductase inhibitors and the risk of venous thrombosis among postmenopausal women. *J Thromb Haemost,* 2004, vol. 2, 700-1
- **SORENSON et al.** Arterial cardiovascular events, statins, low-dose aspirin and subsequent risk of venous thromboembolism: a population based case-control study. *J Thromb Haemost,* 2009, vol. 7, 521-8
- **RAMCHARAN et al.** HMG-CoA reductase inhibitors, other lipid-lowering medication, antiplatelet therapy, and the risk of venous thrombosis. *J Thromb Haemost,* 2009, vol. 7, 514-20
- **GUSELLA.** *Ann Rev Biochem,* 1986, vol. 55, 831-854
- **WEBER et al.** Human diallelic insertion/deletion polymorphisms. *Am J Hum Genet,* October 2002, vol. 71 (4), 854-62
- **STEPHENS et al.** *Science,* July 2001, vol. 293, 489-493
- **LINDER et al.** *Clinical Chemistry,* 1997, vol. 43, 254

- **MARSHALL.** *Nature Biotechnology,* 1997, vol. 15, 1249
- **SCHAFER et al.** *Nature Biotechnology,* 1998, vol. 16, 3
- PCR Technology: Principles and Applications for DNA Amplification. Freeman Press, 1992
- **WU ; WALLACE.** *Genomics,* 1989, vol. 4, 560
- **LANDEGREN et al.** *Science,* 1988, vol. 241, 1077
- **GUATELLI et al.** *Proc Natl Acad Sci USA,* 1990, vol. 87, 1874
- **SAMBROOK ; RUSSELL.** Molecular Clotting: A Laboratory Manual. Cold Spring Harbor Press, 2000
- **SAMBROOK ; RUSSELL.** Molecular Cloning: A Laboratory Manual. Cold Spring Harbor Press, 2000
- **COREY.** Peptide nucleic acids: expanding the scope of nucleic acid recognition. *Trends Biotechnol,* June 1997, vol. 15 (6), 224-9
- **HYRUP et al.** Peptide nucleic acids (PNA): synthesis, properties and potential applications. *Bioorg Med Chem,* January 1996, vol. 4 (1), 5-23
- **LAGRIFFOUL et al.** *Bioorganic & Medicinal Chemistry Letters,* 1994, vol. 4, 1081-1082
- **PETERSEN et al.** *Bioorganic & Medicinal Chemistry Letters,* 1996, vol. 6, 793-796
- **KUMAR et al.** *Organic Letters,* 2001, vol. 3 (9), 1269-1272
- Current Protocols in Nucleic Acid Chemistry. John Wiley & Sons, 2002
- Computational Molecular Biology. Oxford University Press, 1988
- Biocomputing: Informatics and Genome Projects. Academic Press, 1993
- Computer Antilysis of Sequence Data, Part 1. Humana Press, 1994
- Sequence Analysis in Molecular Biology. Academic Press, 1987
- Sequence Analysis Primer. M. Stockton Press, 1991
- **NEEDLEMAN ; WUNSCH.** *J Mol Biol,* 1970, vol. 48, 444-453
- **J. DEVEREUX et al.** *Nucleic Acids Res.,* 1984, vol. 12 (1), 387
- **E. MYERS ; W. MILLER.** *CABIOS,* 1989, vol. 4, 11-17
- **ALTSCHUL et al.** *J Mol Biol,* 1990, vol. 215, 403-10
- **ALTSCHUL et al.** *Nucleic Acids Res,* 1997, vol. 25 (17), 3389-3402
- **PEARSON.** *Methods Mot Biol,* 1994, vol. 25, 365-389
- **DUFRESNE et al.** *Nat Biotechnol,* December 2002, vol. 20 (12), 1269-71
- Current Protocols in Bioinformatics. John Wiley & Sons, Inc,

- Mutation Detection: A Practical Approach. Oxford University Press, 1998
- **SAIKI et al.** *Nature,* 1986, vol. 324, 163-166
- **NARANG et al.** *Methods in Enzymology,* 1979, vol. 68, 90
- **BROWN et al.** *Methods in Enzymology,* 1979, vol. 68, 109
- **BEAUCAGE et al.** *Tetrahedron Letters,* 1981, vol. 22, 1859
- **GIBBS.** *Nucleic Acid Res,* 1989, vol. 17, 2427-2448
- **LIVAK et al.** *PCR Method Appl,* 1995, vol. 4, 357-362
- **TYAGI et al.** *Nature Biotechnology,* 1996, vol. 14, 303-308
- **NAZARENKO et al.** *Nucl Acids Res,* 1997, vol. 25, 2516-2521
- **D.J. LOCKHART et al.** *Nat Biotech,* 1996, vol. 14, 1675-1680
- **M. SCHENA et al.** *Proc Natl Acad Sci,* 1996, vol. 93, 10614-10619
- **ZAMMATTEO et al.** New chips for molecular biology and diagnostics. *Biotechnol Annu Rev,* 2002, vol. 8, 85-101
- **SOSNOWSKI et al.** Active microelectronic array system for DNA hybridization, genotyping and pharmacogenomic applications. *Psychiatr Genet,* December 2002, vol. 12 (4), 181-92
- **HELLER.** DNA microarray technology: devices, systems, and applications. *Annu Rev Biomed Eng,* 2002, vol. 4, 129-53
- **KOLCHINSKY et al.** Analysis of SNPs and other genomic variations using gel-based chips. *Hum Mutat,* April 2002, vol. 19 (4), 343-60
- **MCGALL et al.** High-density genechip oligonucleotide probe arrays. *Adv Biochem Eng Biotechnol,* 2002, vol. 77, 21-42
- Current Protocols in Molecular Biology. John Wiley & Sons, 1989, 6, , 3.1-6, 3.6
- **WEIGL et al.** Lab-on-a-chip for drug development. *Adv Drug Deliv Rev,* February 2003, vol. 55 (3), 349-77
- **SAMBROOK ; RUSSELL.** Molecular Cloning: A laboratory Manual. Cold Spring Harbor Press, 2000
- **CHEN et al.** Single nucleotide polymorphism genotyping: biochemistry. protocol, cost and throughput. *Pharmacogenomics J,* 2003, vol. 3 (2), 77-96
- **KWOK et al.** Detection of single nucleotide polymorphisms. *Curr Issues Mol Biol,* April 2003, vol. 5 (2), 43-60
- **SHI.** Technologies for individual genotyping: detection of genetic polymorphisms in drug targets and disease genes. *Am J Pharmacogenomics,* 2002, vol. 2 (3), 197-205
- **KWOK.** Methods for genotyping single nucleotide polymorphisms. *Annu Rev Genomics Hum Genet,* 2001, vol. 2, 235-58

- **MARNELLOS.** High-throughput SNP analysis for genetic association studies. *Curr Opin Drug Discov Devel,* May 2003, vol. 6 (3), 317-21
- **MYERS et al.** *Science,* 1985, vol. 230, 1242
- **COTTON et al.** *PNAS,* 1988, vol. 85, 4397
- **SALEEBA et al.** *Meth. Enzymol,* 1992, vol. 217, 286-295
- **ORITA et al.** *PNAS,* 1989, vol. 86, 2766
- **COTTON et al.** *Mutat Res,* 1993, vol. 285, 125-144
- **HAYASHI et al.** *Genet Anal Tech Appl,* 1992, vol. 9, 73-79
- **MYERS et al.** *Nature,* 1985, vol. 313, 495
- **WISE et al.** A standard protocol for single nucleotide primer extension in the human genome using matrix-assisted laser desorption/ionization time-of-flight mass spectrometry. *Rapid Commun Mass Spectrom,* 2003, vol. 17 (11), 1195-202
- **BOCKER.** SNP and mutation discovery using base-specific cleavage and MALDI-TOF mass spectrometry. *Bioinformatics,* July 2003, vol. 19 (1), I44-I53
- **STORM et al.** MALDI-TOF mass spectrometry-based SNP genotyping. *Methods Mol Biol,* 2003, vol. 212, 241-62
- **JURINKE et al.** The use of Mass ARRAY technology for high throughput genotyping. *Adv Biochem Eng Biotechnol,* 2002, vol. 77, 57-74
- **JURINKE et al.** Automated genotyping using the DNA MassArray technology. *Methods Mol Biol,* 2002, vol. 187, 179-92
- *Biotechniques,* 1995, vol. 19, 448
- **COHEN et al.** *Adv Chromatogr,* 1996, vol. 36, 127-162
- **GRIFFIN et al.** *Appl Biochem Biotechnol,* 1993, vol. 38, 147-159
- **ORITA et al.** *Proc. Nat. Acad.*
- PCR Technology: Principles and Applications for DNA Amplification. W.H. Freeman and Co, 1992
- Modern Epidemiology. Lippincott, Williams & Wilkins, 1998, 609-622
- **PIEGORSCH et al.** Non-hierarchical logistic models and case-only designs for assessing susceptibility in population-based case-control studies. *Statistics in Medicine,* 1994, vol. 13, 153-162
- **YANG et al.** Sample Size Requirements in Case-Only Designs to Detect Gene-Environment Interaction. *American Journal of Epidemiology,* 1997, vol. 146 (9), 713-720
- **B. WEIR.** Genetic Data Analysis. Sinauer, 1990
- **HOSMER ; LEMESHOW.** Applied Logistic Regression. Wiley, 2000
- **DALY et al.** *Nature Genetics,* 2001, vol. 29, 232-235
- **SCHAID et al.** *Am J Hum Genet,* 2002, vol. 70, 425-434
- **WESTFALL et al.** Multiple comparisons and multiple tests. SAS Institute, 1999
- **BENJAMINI ; HOCHBERG.** *Journal of the Royal Statistical Society,* 1995, vol. 57, 1289-1300
- **WESTFALL ; YOUNG.** Resampling-based Multiple Testing. Wiley, 1993
- Modern Epidemiology. Lippincott, Williams & Wilkins, 1998, 643-673
- **EWENS ; SPIELMAN.** *Am J Hum Genet,* 1995, vol. 62, 450-458
- **PRITCHARD et al.** *Am J Hum Gen,* 1999, vol. 65, 220-228
- **DEVLIN et al.** *Biometrics,* 1999, vol. 55, 997-1004
- **DEVLIN et al.** *Genet Epidem,* 2001, vol. 21, 273-284
- **BACANU et al.** *Am J Hum Genet,* 2000, vol. 66, 1933-1944
- **KEHOE et al.** *Hum Mol Genet,* 1999, vol. 8, 237-245
- **DRAPER ; SMITH.** Applied Regression Analysis. Wiley, 1998
- **HASTIE ; TIBSHIRANI ; FRIEDMAN.** The Elements of Statistical Learning. Springer, 2002
- **BALDING.** A tutorial on statistical methods for population association studies. *Nature Reviews Genetics,* 2006, vol. 7, 781
- **WALL et al.** Haplotype blocks and linkage disequilibrium in the human genome. *Nat Rev Genet,* August 2003, vol. 4 (8), 587-97
- **GARNER et al.** On selecting markers for association studies: patterns of linkage disequilibrium between two and three diallelic loci. *Genet Epidemiol,* January 2003, vol. 24 (1), 57-67
- **ARDLIE et al.** Patterns of linkage disequilibrium in the human genome. *Nat Rev Genet,* April 2002, vol. 3 (4), 299-309
- *Nat Rev Genet,* July 2002, vol. 3 (7), 566
- **REMM et al.** High-density genotyping and linkage disequilibrium in the human genome using chromosome 22 as a model. *Curr Opin Chem Biol,* February 2002, vol. 6 (1), 24-30
- **J.B.S. HALDANE.** The combination of linkage values, and the calculation of distances between the loci of linked factors. *J Genet,* 1919, vol. 8, 299-309
- **G. MENDEL.** Versuche über Pflanzen-Hybriden. Verhandlungen des naturforschenden Vereines in Brünn. *Proceedings of the Natural History Society of Brünn,* 1866
- Genes IV. Oxford University Press, 1990
- **D.L. HARTL ; A.G. CLARK.** Principles of Population Genetics. Sinauer Associates, Inc., Mass, 1989
- **J.H. GILLESPIE.** Population Genetics: A Concise Guide. Johns Hopkins University Press, 2004

- **R.C. LEWONTIN.** The interaction of selection and linkage. I. General considerations; heterotic models. *Genetics,* 1964, vol. 49, 49-67
- **P.G. HOEL.** Introduction to Mathematical Statistics. John Wiley & Sons, Inc, 1954
- **R.R. HUDSON.** Two-locus sampling distributions and their application. *Genetics,* 2001, vol. 159, 1805-1817
- **A.P. DEMPSTER ; N.M. LAIRD ; D.B. RUBIN.** Maximum likelihood from incomplete data via the EM algorithm. *J R Stat Soc,* 1977, vol. 39, 1-38
- **L. EXCOFFIER ; M. SLATKIN.** Maximum-likelihood estimation of molecular haplotype frequencies in a diploid population. *Mol Biol Evol,* 1995, vol. 12 (5), 921-927
- **D.A. TREGOUET ; S. ESCOLANO ; L. TIRET ; A. MALLET ; J.L. GOLMARD.** A new algorithm for haplotype-based association analysis: the Stochastic-EM algorithm. *Ann Hum Genet,* 2004, vol. 68, 165-177
- **A.D. LONG ; C.H. LANGLEY CH.** The power of association studies to detect the contribution of candidate genetic loci to variation in complex traits. *Genome Research,* 1999, vol. 9, 720-731
- **A. AGRESTI.** Categorical Data Analysis. John Wiley & Sons, Inc, 1990
- **K. LANGE.** Mathematical and Statistical Methods for Genetic Analysis. Springer-Verlag New York, Inc, 1997
- The International HapMap Project. *Nature,* 2003, vol. 426, 789-796
- A haplotype map of the human genome. *Nature,* 2005, vol. 437, 1299-1320
- **G.A. THORISSON ; A.V. SMITH ; L. KRISHNAN ; L.D. STEIN.** The International HapMap Project Web Site. *Genome Research,* 2005, vol. 15, 1591-1593
- **G. MCVEAN ; C.C.A. SPENCER ; R. CHAIX.** Perspectives on human genetic variation from the HapMap project. *PLoS Genetics,* 2005, vol. 1 (4), 413-418
- **J.N. HIRSCHHORN ; M.J. DALY.** Genome-wide association studies for common diseases and complex traits. *Nat Genet,* 2005, vol. 6, 95-108
- **S.J. SCHRODI.** A probabilistic approach to large-scale association scans: a semi-Bayesian method to detect disease-predisposing alleles. *SAGMB,* 2005, vol. 4 (1), 31
- **W.Y.S. WANG ; B.J. BARRATT ; D.G. CLAYTON ; J.A. TODD.** Genome-wide association studies: theoretical and practical concerns. *Nat Rev Genet,* 2005, vol. 6, 109-118
- **J.K. PRITCHARD ; M. PRZEWORSKI.** Linkage disequilibrium in humans: models and data. *Am J Hum Genet,* 2001, vol. 69, 1-14
- **SHEET ; STEPHENS.** *Am J Hum Genet,* 2006, vol. 76, 629-644
- **BROWNING.** *Hum Genet,* 2008, vol. 124, 439-450
- **BROWNING.** Missing data imputation and haplotype phase inference for genome-wide association studies. *Hum Genet,* 2008, vol. 124, 439-450
- **BROWNING et al.** A unified approach to genotype imputation and haplotype-phase inference for large data sets of trios and unrelated individuals. *Am J Hum Genet.,* February 2009, vol. 84 (2), 210-23
- **ROSES.** *Nature,* 2000, vol. 405, 857-865
- **GOULD ROTHBERG.** *Nature Biotechnology,* 2001, vol. 19, 209-211
- **EICHELBAUM.** *Clin Exp Pharmacol Physiol,* 1996, vol. 23 (10-11), 983-985
- **LINDER.** *Clin Chem,* 1997, vol. 43 (2), 254-266
- **PFOST et al.** *Trends in Biotechnology,* August 2000
- **ROSE et al.** Pharmacogenetic analysis of clinically relevant genetic polymorphisms. *Methods Mol Med,* 2003, vol. 85, 225-37
- **CACABELOS.** Pharmacogenomics for the treatment of dementia. *Ann Med,* 2002, vol. 34 (5), 357-79
- **MAIMONE et al.** Pharmacogenomics of neurodegenerative diseases. *Eur J Pharmacol,* February 2001, vol. 413 (1), 11-29
- **POIRIER.** Apolipoprotein E: a pharmacogenetic target for the treatment of Alzheimer's disease. *Mol Diagn,* December 1999, vol. 4 (4), 335-41
- **SIEST et al.** Pharmacogenomics of drugs affecting the cardiovascular system. *Clin Chem Lab Med,* April 2003, vol. 41 (4), 590-9
- **MUKHERJEE et al.** Pharmacogenomics in cardiovascular diseases. *Prog Cardiovasc Dis,* May 2002, vol. 44 (6), 479-98
- **MOOSER et al.** Cardiovascular pharmacogenetics in the SNP era. *J Thromb Haemost,* July 2003, vol. 1 (7), 1398-402
- **MCLEOD et al.** Cancer pharmacogenomics: SNPs, chips, and the individual patient. *Cancer Invest,* 2003, vol. 21 (4), 630-40
- **WATTERS et al.** Cancer pharmacogenomics: current and future applications. *Biochim Biophys Acta,* March 2003, vol. 1603 (2), 99-111
- Remington's Pharmaceutical Sciences. Mack Publishing Company, 1990
- Current Protocols in Pharmacology. John Wiley & Sons, Inc,
- Antisense Drug Technology: Principles, Strategies, and Applications. Marcel Dekker, Inc, 2001
- **THOMPSON.** *Drug Discovery Today,* 2002, vol. 7 (17), 912-917
- **LAVERY et al.** Antisense and RNAi: powerful tools in drug target discovery and validation. *Curr Opin Drug Discov Devel,* July 2003, vol. 6 (4), 561-9

- **STEPHENS et al.** Antisense oligonucleotide therapy in cancer. *Curr Opin Mol Ther,* April 2003, vol. 5 (2), 118-22
- **KURRECK.** Antisense technologies. Improvement through novel chemical modifications. *Eur J Biochem,* April 2003, vol. 270 (8), 1628-44
- **DIAS et al.** Antisense oligonucleotides: basic concepts and mechanisms. *Mol Cancer Ther,* March 2002, vol. 1 (5), 347-55
- **CHEN.** Clinical development of antisense oligonucleotides as anti-cancer therapeutics. *Methods Mol Med,* 2003, vol. 75, 621-36
- **WANG et al.** Antisense anticancer oligonucleotide therapeutics. *Curr Cancer Drug Targets,* November 2001, vol. 1 (3), 177-96
- **BENNETT.** Efficiency of antisense oligonucleotide drug discovery. *Antisense Nucleic Acid Drug Dev,* June 2002, vol. 12 (3), 215-24
- **REYNOLDS et al.** Rational siRNA design for RNA interference. *Nat Biotechnol,* March 2004, vol. 22 (3), 326-30
- **CHI et al.** Genomewide view of gene silencing by small interfering RNAs. *PNAS,* 2003, vol. 100 (11), 6343-6346
- **VICKERS et al.** Efficient Reduction of Target RNAs by Small Interfering RNA and RNase H-dependent Antisense Agents. *J Biol Chem,* 2003, vol. 278, 7108-7118
- **AGAMI.** RNAi and related mechanisms and their potential use for therapy. *Curr Opin Chem Biol,* December 2002, vol. 6 (6), 829-34
- **SHI.** Mammalian RNAi for the masses. *Trends Genet,* January 2003, vol. 19 (1), 9-12
- **SHUEY et al.** RNAi: gene-silencing in therapeutic intervention. *Drug Discovery Today,* October 2002, vol. 7 (20), 104-1046
- **MCMANUS et al.** *Nat Rev Genet,* October 2002, vol. 3 (10), 737-47
- **XIA et al.** *Nat Biotechnol,* October 2002, vol. 20 (10), 1006-10
- **PLASTERK et al.** *Curr Opin Genet Dev,* October 2000, vol. 10 (5), 562-7
- **BOSHER et al.** *Nat Cell Biol,* February 2000, vol. 2 (2), E31-6
- **HUNTER.** *Curr Biol,* June 1999, vol. 17; 9 (12), R440-2
- **JAZWINSKA.** *A Trends Guide to Genetic Variation and Genomic Medicine,* March 2002, S30-S36
- **ROTHBERG.** *Nat Biotechnol,* March 2001, vol. 19 (3), 209-11
- **JAZWINSKA.** *A Trends Guide Genetic Variation and Genomic Medicine,* March 2002, S30-S36
- Current Protocols in Toxicology. John Wiley & Sons, Inc,
- **KREN et al.** *Proc Natl Acad Sci USA,* 1999, vol. 96, 10349-10354
- **SAMBROOK ; RUSSELL.** Molecular Cloning: A Laboratory Manual. Cold Spring Harbor Laboratory Press, 2000
- **AUSUBEL et al.** *Current Protocols in Molecular Biology,* 1992
- **TERPE.** Overview of tag protein fusions: from molecular and biochemical fundamentals to commercial systems. *Appl Microbiol Biotechnol,* January 2003, vol. 60 (5), 523-33
- **GRADDIS et al.** Designing proteins that work using recombinant technologies. *Curr Pharm Biotechnol,* December 2002, vol. 3 (4), 285-97
- **NILSSON et al.** Affinity fusion strategies for detection, purification, and immobilization of recombinant proteins. *Protein Expr Purif,* October 1997, vol. 11 (1), 1-16
- **BOWIE et al.** *Science,* 1990, vol. 247, 1306-1310
- **CUNNINGHAM et al.** *Science,* 1989, vol. 244, 1081-1085
- **SMITH et al.** *J Mol Biol,* 1992, vol. 224, 899-904
- **DE VOS et al.** *Science,* 1992, vol. 255, 306-312
- **T.E. CREIGHTON.** Proteins - Structure and Molecular Properties. W.H. Freeman and Company, 1993
- **F. WOLD.** Posttranslational Covalent Modification of Proteins. Academic Press, 1983, 1-12
- **SEIFTER et al.** *Meth Enzymol,* 1990, vol. 182, 626-646
- **RATTAN et al.** *Ann NY Acad Sci,* 1992, vol. 663, 48-62
- Current Protocols in Protein Science. John Wiley & Sons, 2002
- Methods in Enzymology: Guide to Molecular Cloning Techniques. Academic Press, 1987
- Current Protocols in Immunology. John Wiley & Sons
- **HAGE.** Immunoassays. *Anal Chem,* June 1999, vol. 15;71 (12), 294R-304R
- **LAM et al.** *Nature,* 1991, vol. 354, 82-84
- **HOUGHTEN et al.** *Nature,* 1991, vol. 354, 84-86
- **SONGYANG et al.** *Cell,* 1993, vol. 72, 767-778
- **HODGSON.** *Bio/technology,* September 1992, vol. 10 (9), 973-80
- **ZERVOS et al.** *Cell,* 1993, vol. 72, 223-232
- **MADURA et al.** *J Biol Chem,* 1993, vol. 268, 12046-12054
- **BARTEL et al.** *Biotechniques,* 1993, vol. 14, 920-924
- **IWABUCHI et al.** *Oncogene,* 1993, vol. 8, 1693-1696
- **MORRISON et al.** *Proc Natl Acad Sci USA,* 1984, vol. 81, 6851

- **NEUBERGER et al.** *Nature,* 1984, vol. 312, 604
- **WARD et al.** *Nature,* 1989, vol. 334, 544
- **SEGAL et al.** *J Immunol Methods,* 2001, vol. 248, 1
- **CARTER.** *J Immunol Methods,* 2001, vol. 248, 7
- **TODOROVSKA et al.** *J Immunol Methods,* 2001, vol. 248, 47
- **HARLOW.** Antibodies. Cold Spring Harbor Press, 1989
- **KOHLER ; MILSTEIN.** *Nature,* 1975, vol. 256, 495
- **HE et al.** *J Immunol,* 2002, vol. 169, 595
- **HOOGENBOOM ; CHAMES.** *Immunol Today,* 2000, vol. 21, 371
- **LIU et al.** *J Mol Biol,* 2002, vol. 315, 1063
- **MORGAN.** Antibody therapy for Alzheimer's disease. *Expert Rev Vaccines,* February 2003, vol. 1, 53-9
- **ROSS et al.** Anticancer antibodies. *Am J Clin Pathol,* April 2003, vol. 119 (4), 472-85
- **GOLDENBERG.** Advancing role of radiolabeled antibodies in the therapy of cancer. *Cancer Immunol Immunother,* May 2003, vol. 52 (5), 281-96
- **ROSS et al.** Antibody-based therapeutics in oncology. *Expert Rev Anticancer Ther,* February 2003, vol. 3 (1), 107-21
- **CAO et al.** Bispecific antibody conjugates in therapeutics. *Adv Drug Deliv Rev,* February 2003, vol. 55 (2), 171-97
- **MEHREN et al.** Monoclonal antibody therapy for cancer. *Annu Rev Med,* 2003, vol. 54, 343-69
- **HUDSON et al.** Engineered antibodies. *Nat Med,* January 2003, vol. 9 (1), 129-34
- **BREKKE et al.** Therapeutic antibodies for human diseases at the dawn of the twenty-first century. *Not Rev Drug Discov,* January 2003, vol. 2 (1), 52-62
- *Nat Rev Drug Discov,* March 2003, vol. 2 (3), 240
- **HOUDEBINE.** Antibody manufacture in transgenic animals and comparisons with other systems. *Curr Opin Biotechnol,* December 2002, vol. 13 (6), 625-9
- **ANDREAKOS et al.** Monoclonal antibodies in immune and inflammatory diseases. *Curr Opin Biotechnol,* December 2002, vol. 13 (6), 615-20
- **KELLERMANN et al.** Antibody discovery: the use of transgenic mice to generate human monoclonal antibodies for therapeutics. *Curr Opin Biotechnol,* December 2002, vol. 13 (6), 593-7
- **PINI et al.** Phage display and colony filter screening for high-throughput selection of antibody libraries. *Comb Chem High Throughput Screen,* November 2002, vol. 5 (7), 503-10
- **BATRA et al.** Pharmacokinetics and biodistribution of genetically engineered antibodies. *Curr Opin Biotechnol,* December 2002, vol. 13 (6), 603-8
- **TANGRI et al.** Rationally engineered proteins or antibodies with absent or reduced immunogenicity. *Curr Med Chem,* December 2002, vol. 9 (24), 2191-9
- **LEONG et al.** *Cytokine,* 2001, vol. 16, 106
- **SMITH et al.** *Gene,* 1988, vol. 67, 31-40
- **AMANN et al.** *Gene,* 1988, vol. 69, 301-315
- **STUDIER et al.** *Gene Expression Technology: Methods in Enzymology,* 1990, vol. 185, 60-89
- **S. GOTTESMAN.** Gene Expression Technology: Methods in Enzymology. Academic Press, 1990, vol. 185, 119-128
- **WADA et al.** *Nucleic Acids Res,* 1992, vol. 20, 2111-2118
- **BALDARI et al.** *EMBO J,* 1987, vol. 6, 229-234
- **KURJAN et al.** *Cell,* 1982, vol. 30, 933-943
- **SCHULTZ et al.** *Gene,* 1987, vol. 54, 113-123
- **SMITH et al.** *Mol Cell Biol,* 1983, vol. 3, 2156-2165
- **LUCKLOW et al.** *Virology,* 1989, vol. 170, 31-39
- **B. SEED.** *Nature,* 1987, vol. 329, 840
- **KAUFMAN et al.** *EMBO J,* 1987, vol. 6, 187-195
- Current Protocols in Molecular Biology. John Wiley & Sons
- **B. HOGAN.** Manipulating the Mouse Embryo. Cold Spring Harbor Laboratory Press, 1986
- **LAKSO et al.** *PNAS,* 1992, vol. 89, 6232-6236
- **O'GORMAN et al.** *Science,* 1991, vol. 251, 1351-1355
- **I. WILMUT et al.** *Nature,* 1997, vol. 385, 810-813
- **PETTERS et al.** Transgenic animals as models for human disease. *Transgenic Res,* 2000, vol. 9 (4-5), 347-51
- **WOLF et al.** Use of transgenic animals in understanding molecular mechanisms of toxicity. *J Pharm Pharmacol,* June 1998, vol. 50 (6), 567-74
- **ECHELARD.** Recombinant protein production in transgenic animals. *Curr Opin Biotechnol,* October 1996, vol. 7 (5), 536-40
- **HOUDEBINE.** Transgenic animal bioreactors. *Transgenic Res,* 2000, vol. 9 (4-5), 305-20
- **PIRITY et al.** Embryonic stem cells, creating transgenic animals. *Methods Cell Biol,* 1998, vol. 57, 279-93
- **ROBL et al.** Artificial chromosome vectors and expression of complex proteins in transgenic animals. *Theriogenology,* January 2003, vol. 59 (1), 107-13
- **KOSTER et al.** *Lancet,* 1993, vol. 342 (8886-8887), 1503-1506

- **BLOM et al.** *JAMA,* 2005, vol. 293 (6), 715-722
- **VAN STRALEN et al.** *Arch Intern Med,* 2008, vol. 168 (1), 21-26
- **BLOM et al.** *JAMA,* 2005, vol. 293, 715-22
- **CHINTHAMMITR et al.** *J Thromb Haemost.,* 2006, vol. 4, 2587-92
- **GERMER et al.** *Genome Res.,* 2000, vol. 10, 258-66
- **FRAZER et al.** *Nature,* 2007, vol. 449, 851-61
- **DENTALI et al.** *Ann Intern Med.,* 2007, vol. 146, 278-88
- **SHIREMAN et al.** *Chest.,* 2006, vol. 130, 1390-6
- **WITTKOWSKY et al.** *Arch Intern Med.,* 2005, vol. 165, 703
- **BURESLY et al.** *Arch Intern Med.,* 2005, vol. 165, 784-9
- **FLINTERMAN et al.** Recurrent thrombosis and survival after a first venous thrombosis of the upper extremity. *Circulation,* 2008, vol. 118, 1366-72
- **BLOM et al.** Malignancies, prothrombotic mutations, and the risk of venous thrombosis. *JAMA,* 2005, vol. 293, 715-22
- **GERMER et al.** High-throughput SNP allele-frequency determination in pooled DNA samples by kinetic PCR. *Genome Res.,* 2000, vol. 10, 258-66
- **WEIR.** Genetic Data Analysis II. Sinauer Associates Inc, 1996
- **BENJAMINI et al.** *Journal of the Royal Statistical Society,* 1995, 1289-300